# EUROPEAN PATENT SPECIFICATION

(11) **EP 4 288 437 B1**
(45) Date of publication and mention of the grant of the patent: **24.06.2026**
(21) Application number: 22708793.9
(22) Date of filing: 04.02.2022
(51) Int. Cl.: C07D 471/20, C07D 487/20, C07D 498/20, A61P 35/00, A61K 31/407, A61K 31/5386

(54) **MAP4K1 INHIBITORS**
MAP4K1-INHIBITOREN
INHIBITEURS DE MAP4K1

(30) Priority: 05.02.2021 WO PCT/CN2021/075566; 13.04.2021 WO PCT/CN2021/086946
(43) Date of publication of application: 13.12.2023
(73) Proprietor: Bayer Aktiengesellschaft, 51373 Leverkusen (DE); Deutsches Krebsforschungszentrum, 69120 Heidelberg (DE)
(72) Inventor: LÜCKING, Ulrich, 10317 Berlin (DE); MOWAT, Jeffrey Stuart, 2000 Antwerp (BE); ZORN, Ludwig, 13509 Berlin (DE); WORTMANN, Lars, 88400 Biberach an der Riß (DE); MÜLLER, Steffen, 45472 Mülheim an der Ruhr (DE); LEDER, Gabriele, 14163 Berlin (DE); BERNDT, Sandra, 16540 Hohen Neuendorf (DE); GÜNTHER, Judith, 13187 Berlin (DE); KUHNKE, Lara, 13439 Berlin (DE); WENGNER, Antje Margret, 13086 Berlin (DE); CARRETERO, Rafael, 59121 Heidelberg (DE); WOHLFAHRT, Gerd, 10115 Berlin (DE); FRIBERG, Anders, 13189 Berlin (DE); BÖMER, Ulf, 16548 Glienicke/Nordbahn (DE); NEUHAUS, Roland, 12157 Berlin (DE); OSMERS, Maren, 14624 Dallgow-Döberitz (DE); ONDOZABAL, Hideki Miyatake, 10115 Berlin (DE); SCHÄFER, Martina, 13465 Berlin (DE); EAGLING, Louise, 10115 Berlin (DE); LEFRANC, Julien, 64287 Darmstadt (DE); NOWAK-REPPEL, Katrin, 13187 Berlin (DE); OFFRINGA, Rienk, 69115 Heidelberg (DE); CHEN, Peng, Xian City, Shaanxi (CN); WANG, Xuewei, Xian City, Shaanxi (CN); YAN, Yuanyuan, Xian City, Shaanxi (CN); THEDE, Kai, 10437 Berlin (DE); SITNIKOV, Nikolay, 15732 Schulzendorf (DE); BUCHMANN, Berndt, 16540 Hohen Neuendorf (DE); KOSEMUND, Dirk, 10245 Berlin (DE); STOECKIGT, Detlef, 14471 Potsdam (DE)
(74) Representative: Altmann Stößel Dick Patentanwälte PartG mbB
(86) International application number: PCT/EP2022/052794
(87) International publication number: WO 2022/167627

(56) References cited:
- WO-A1-2019/090198
- WO-A1-2020/061377
- WO-A1-2021/074279
- WO-A1-2021/249913

## Description

The present disclosure relates to MAP4K1 inhibitors, to pharmaceutical compositions and combinations comprising the compounds according to the invention, and to the prophylactic and therapeutic use of the inventive compounds, respectively to the use of said compounds for manufacturing pharmaceutical compositions for the treatment or prophylaxis of diseases, in particular for neoplastic disorders, repectively cancer or conditions with dysregulated immune responses or other disorders associated with aberrant MAP4K1 signaling, as a sole agent or in combination with other active ingredients.

The present disclosure further relates to the use, respectively to the use of said compounds for manufacturing pharmaceutical compositions for the treatment or prophylaxis of protein inhibitors in benign hyperplasias, atherosclerotic disorders, sepsis, autoimmune disorders, vascular disorders, viral infections, in neurodegenerative disorders, in inflammatory disorders, in atherosclerotic disorders and in male fertility control.

### Background

Although cancer cell commonly can be recognized by the adaptive immune system, the response generated is evidently not capable of eliminating the tumor. A major reason for this is the presence of immunosuppressive mechanisms in the tumor microenvironment. In this respect, inhibitors of T-cell immune checkpoint such as CTLA-4, PD-1 or PD-L1 were recently shown to result in a remarkable clinical efficacy in subsets of cancer patients. Besides cell surface receptors that act as negative immune regulators, several mediators of intracellular signaling have been identified that also represent potential immunoevasive mechanisms utilized by the tumor.

One of these is MAP4K1, also known as hematopoietic progenitor kinase 1 (HPK1). MAP4K1 (GenelD11184) is a serine/threonine kinase and member of the Germinal Center Kinase family. In the adult organism MAP4K1 expression is restricted to hematopoietic cell types. The MAP4K1 protein consist of a N-terminal kinase domain, followed by a proline-rich domain that can interact with adaptor molecules through SH2 and SH3 domains, and a C-terminal citron homology domain of which the exact function remains to be identified. Through its proline-rich domain, MAP4K1 is capable of binding to a diversity of adaptors in hematopoietic cells, including those involved in T-cell receptor (TCR), B-cell receptor (BCR) and cytokine signaling (Hu et al., Genes Dev. 1996 Sep 15;10(18):2251-64, 2.; Ling et al.,. J Biol Chem. 2001 Jun 1;276(22), Sauer et al., J Biol Chem. 2001 Nov 30;276(48):45207-16., Tsuji et al., J Exp Med. 2001 Aug 20;194(4):529-39, Boomer et al., J Cell Biochem. 2005 May 1;95(1):34-44).

The function of MAP4K1 has been studied in greatest detail in the context of TCR signaling. Upon TCR stimulation, MAP4K1 is phosphorylated on tyrosine 381 (Y-381; Y-379 in mouse) (Di Bartolo et al., J Exp Med. 2007 Mar 19;204(3):681-91). Consequently, MAP4K1 is recruited to the TCR-signaling complex where it induces dissociation of this complex through its serine/threonine kinase function. In particular MAP4K1 phosphorylates the SLP-76 adaptor protein at Serine-376, resulting in downregulation of AP-1 and Erk2 pathways. As, such, MAPK1 acts as a negative feedback on TCR-signaling (Liou et al., Immunity. 2000 Apr;12(4):399-408; Lasserre et al., J Cell Biol. 2011 Nov 28;195(5):839-53.). Alternatively, MAP4K1 can be triggered to suppress T cell function by prostaglandin E2 (PGE2), and possibly also by transforming growth factor beta (TGF-beta), factors that are commonly found in the tumor microenvironment. Notably, MAP4K1 activation by these mediators involves protein kinase A (PKA)-dependent phosphorylation of Serine 171 (S-171; also in mouse) (Alzabin et al., Cancer Immunol Immunother. 2010 Mar;59(3):419-29; Sawasdikosol et al., J Biol Chem. 2007 Nov 30;282(48):34693-9.).

Further important insights into the function of MAP4K1 in the regulation of T cell immunity stem from *in vivo* and *in vitro* experiments respectively with MAP4K1 deficient mice produced by two laboratories and with immune cells isolated from these mice (Shui et al., Nat Immunol. 2007 Jan;8(1):84-91; Alzabin et al., Cancer Immunol Immunother. 2010 Mar;59(3):419-29). MAP4K1-deficient mice show an apparent normal phenotype, are fertile and exhibit normal lymphocyte development. These animals are prone to develop T-cell dependent autoimmune reactivity as indicated by development of a more severe disease score in the EAE (experimental autoimmune encephalomyelitis) model of multiple sclerosis (Shui et al., Nat Immunol. 2007 Jan;8(1):84-91). In case of the second strain, a dysregulation of immune function was observed when, at the age of approximately 6 months, MAP4K1-deficient mice develop a spontaneous autoimmune phenotype (Alzabin et al., Cancer Immunol Immunother. 2010 Mar;59(3):419-29). *In vitro* studies showed that MAP4K1-/- T-cells display hyper-responsiveness upon TCR-stimulation. These cells proliferate and secrete pro-inflammatory cytokines like IL-2 or IFNg to a significantly greater extent than their wild-type counterparts (Shui et al., Nat Immunol. 2007 Jan;8(1):84-91). Furthermore, MAP4K1-/- T-cells are resistant to PGE2-mediated suppression of T cell proliferation, suppression of IL-2 production and induction of apoptosis (Alzabin et al., Cancer Immunol Immunother. 2010 Mar;59(3):419-29). In the context of tumor immunology, *in vivo* experiments revealed that MAP4K1-/- mice are much more resistant to tumorigenesis by PGE2-producing Lewis lung carcinoma than wild type mice, which correlated with increased T-lymphocyte infiltration in the tumor areas. The crucial role of T-cells in tumor rejection was supported by experiments in which MAP4K1-/- T-cells adoptively transferred into T-cell-deficient mice were able to eradicate tumors more efficiently than wild-type T-cells (Alzabin et al., Cancer Immunol Immunother. 2010 Mar;59(3):419-29). The important role of the kinase enzymatic activity was demonstrated by studies were only wild type MAP4K1, but not the MAP4K1 kinase-dead mutant, could mediate serine-phosphorylation of the TCR-signaling complex component SLP-76 and subsequent binding of SLP-76 to the negative regulator of TCR-signaling 14-3-3-t (Shui et al., Nat Immunol. 2007 Jan;8(1):84-91). MAP4K1 also regulates the stimulation and activation of dendritic cells. MAP4K1 deficient Bone marrow derived cells (BMDC) express after maturation and stimulation higher level of costimulatory molecules and produce more proinflammatory cytokines. Also elimination of tumors was observed to be more efficient by MAP4K1 -/- BMDC compared to their wildtype counterparts (Alzabin et al., J Immunol. 2009 May 15;182(10):6187-94).

### Prior art

In WO2019164846A1 HPK1 inhibitors and methods for their use in various forms of cancer are described. These compounds differ from the instant compounds in their chemical structure.

In US20190256500A1 HPK1 inhibitors and methods for their use in treating, preventing or ameliorating diseases or disorders associated with HPK1 such as cancer are described. These compounds differ from the instant compounds in their chemical structure.

In US20190256520A1 HPK1 inhibitors and methods for their use in treating, preventing or ameliorating diseases or disorders associated with HPK1 such as cancer are described. These compounds differ from the instant compounds in their chemical structure.

In CN109721620A HPK1 inhibitors and their uses are described. These compounds differ from the instant compounds in their chemical structure.

In WO2019090198A1, compounds used to modulate or inhibit the activity of HPK1 and methods for their use in treatment of viral infections and proliferative disorders, such as cancer are described. These compounds differ from the instant compounds in their chemical structure.

In WO 2018/215668, MAP4K1 (HPK1) inhibitors and methods for their use in diseases including hyperproliferative diseases, diseases of immune system dysfunction, intlammatory disorders, neurological diseases, and cardiovascular diseases are described. These compounds differ from the instant compounds in their chemical structure.

In WO 2018/049214, HPK1 modulators and methods for their use in cancer treatment are described. These compounds differ from the instant compounds in their chemical structure.

In WO 2018/049200, HPK1 modulators and methods for their use in cancer treatment are described. These compounds differ from the instant compounds in their chemical structure.

In WO 2018/049152, HPK1 modulators and methods for their use in cancer treatment are described. These compounds differ from the instant compounds in their chemical structure.

In WO 2018/049119, HPK1 modulators and methods for their use in cancer treatment are described. These compounds differ from the instant compounds in their chemical structure.

In WO 2018/102366, HPK1 inhibitors and methods for their use in the treatment of cancer are described. These compounds differ from the instant compounds in their chemical structure. In WO 2018/183956, HPK1 inhibitors and use of such compounds in treating HPK1-dependent disorders and enhancing immune response are described. These compounds differ from the instant compounds in their chemical structure.

In WO 2018/183964, HPK1 inhibitors and use of such compounds in treating HPK1-dependent disorders and enhancing immune response are described. These compounds differ from the instant compounds in their chemical structure.

In WO 2018/167147, HPK1 inhibitors and use of such compounds in treating HPK1-dependent disorders and enhancing immune response are described. These compounds differ from the instant compounds in their chemical structure.ln WO

In WO2016/205942 HPK1, respectively inhibitors and methods of their use in cancer treatment are described. Specifically, the application concerns thieno-pyridinones that can be used in anti-cancer therapy. These compounds differ from the instant compounds in their chemical structure.

In WO 2016/195776 inhibitors and methods for leukemia, cancer and diabetes treatment dependent on inhibition the interaction of menin with of MLL1, MLL2 and MLL-fusion oncoproteins are described. These compounds differ from the instant compounds in their chemical structure.

In WO 2006/014325 C-MET modulators and their use in cancer treatment are described. These compounds differ from the instant compounds in their chemical structure.

In WO 2005/058891 Rho kinase inhibitors and their use in cardiovascular and cancer treatment are described. These compounds differ from the instant compounds in their chemical structure.

In WO 2015/089479 several inhibitors are described that show inhibition of several kinases (e.g., BTK, HCK, TAK1 and HPK1). These compounds differ from the instant compounds in their chemical structure.

In WO2016/004272 BTK inhibitors and methods of their use in cancer treatment are described. No specific example is disclosed which falls in the group of compounds as defined according to the present invention.

In WO 2011/090738 Type II RAF kinase inhibitors and their use in various diseases are described. No specific example is disclosed which falls in the group of compounds as defined according to the present invention.

In CN102086211 and WO2006116713 protein kinase inhibitors and their use in prophylaxis and treatment of diseases including cancer are described. No specific example is disclosed which falls in the group of compounds as defined according to the present invention.

In WO 2010/045095 protein tyrosin kinase modulators and their use in the treatment of hyperproliferative disorders are described. No specific example is disclosed which falls in the group of compounds as defined according to the present invention.

In WO 2008/089307 compounds and methods of their use in the treatment of pain, inflammation and cancer are described. No specific example is disclosed which falls in the group of compounds as defined according to the present invention.

In WO 2006/114180 kinase inhibitors for treating diseases, particularly tumors are described. No specific example is disclosed which falls in the group of compounds as defined according to the present invention.

In WO 2006/014325 c-Met modulators and their methods of use to treat kinase-dependent diseases and conditions are described. No specific example is disclosed which falls in the group of compounds as defined according to the present invention.

In US 2003/0055049 compounds for treating disorders with abnormal cell growth in mammals are described. No specific example is disclosed which falls in the group of compounds as defined according to the present invention.

In WO 2001/23389 antagonists of NPY receptors compositions and methods of the treatment of physiological disorders associated with an excess of neuropeptide Y are described. No specific example is disclosed which falls in the group of compounds as defined according to the present invention.

In WO 2019/149738 protein kinase MKK4 inhibitors for promoting liver regeneration or reducing or preventing hepatocyte death are described.

It would therefore be desirable to provide novel MAP4K1 inhibitors having prophylactic and therapeutic properties.

Accordingly, it is an object of the present invention to provide compounds and pharmaceutical compositions comprising these compounds used for prophylactic and therapeutic applications for hyperproliferative disorders, in particular for cancer, respectively tumour disorders, and conditions with dysregulated immune responses, as a sole agent or in combination with other active ingredients.

A further object of the present invention is to provide compounds and pharmaceutical compositions comprising these compounds for manufacturing pharmaceutical compositions for the treatment or prophylaxis of benign hyperplasias, atherosclerotic disorders, sepsis, autoimmune disorders, vascular disorders, viral infections, in neurodegenerative disorders, in inflammatory disorders, in atherosclerotic disorders and in male fertility control.

Surprisingly, the compounds according to the invention inhibit the MAP4K1 protein and thereby enhance tumor immunogenicity leading to inhibition of cancer cells growth by the immune response. Accordingly, they provide novel structures for the therapy of human and animal disorders, in particular of cancers.

Any references herein to methods of treatment refer to the compounds, pharmaceutical compositions and medicaments of the present invention for use in a method for treatment of the human (or animal) body by therapy (or for diagnosis).

The present invention relates to compound of formula (I) (if referred to embodiment 1, this group of compounds is meant) 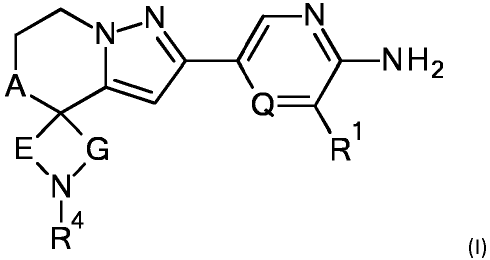 wherein
- A: is selected from a single bond, -CH2- or -O-;
- E: is -(CH2)n- with n = 1 or 2;
- G: is -(CH2)- and with the condition, that the cyclic substructure comprising E-N-G is a 4 to 5 membered heterocycloalkyl;
- Q: is selected from N or C-R²;
- R¹: is selected from
- -NO₂; -CN;
- C₁₋₄-alkyl optionally substituted by -OH or 1 to 3 -F;
- cyclopropyl;
- 4 to 6 membered heterocycloalkyl or heterocycloalkenyl, both with 1 - 2 nitrogen atoms, but always linked via a carbon atom to the phenyl group of compounds of formula (I) and both optionally substituted by C₁₋₄-alkyl;
- phenyl optionally substituted by halogen, -CN, C₁₋₄-alkyl, C₁₋₄-haloalkyl, C₃₋₆-cycloalkyl, O-C₁₋₄-alkyl, O-C₃₋₆-cycloalky, C₁₋₃-alkinyl, -C(O)-R_{d}, SR_{d}, SOR_{d}, SO₂R_{d}, SONR_{d}, SON(H)R_{d}, -SO₂-NH₂, - SO₂-N(H)-R_{d} or -SO₂-N(R_{d})₂, wherein R_{d} is selected from methyl, ethyl, cyclopropyl, isopropyl and tert. butyl;
- 5 to 6 membered heteroaryl with 1, 2 or 3 heteroatoms selected from N, O or S and optionally substituted by 1 or 2 of the following substituents: halogen, -CN, C₁₋₄-alkyl, C₁₋₄-haloalkyl, C₃₋₆-cycloalkyl, O-C₁₋₄-alkyl, O-C₃₋₆-cycloalkyl, C₁₋₃-alkinyl, SR_{d}, SOR_{d}, SO₂R_{d}, SONR_{d} or SON(H)R_{d} wherein R_{d} is selected from methyl, ethyl, cyclopropyl, isopropyl and tert. butyl;
- -O-CH₃; -O-CH(CH₃)₂ -O-CHF₂; -O-CF₃; 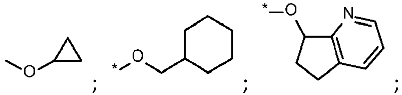
- -O-CHRₐ-phenyl optionally substituted by one, two or three of the following substituents: halogen, -CN, C₁₋₄-alkyl optionally substituted by one or more -F, O-C₁₋₄-alkyl, C₁₋₆-alkinyl-OH or -SO₂-CH₃ and with Rₐ selected from -H, -F or -CH₃, -CF₃, -CH₂-CH₃, -CH(CH₃)₂ or - cyclopropyl;
- -O-CHRₐ-6 membered heteroaryl optionally substituted one, two or three of the following substituents: halogen, -CN, C₁₋₄-alkyl optionally substituted by one or more -F or -SO₂-CH₃ and with Rₐ selected from -H, -F or -CH₃, -CF₃, -CH₂-CH₃, -CH(CH₃)₂ or -cyclopropyl;
- -COORₐ with Rₐ selected from -H or -CH₃;
- C(=O)-methyl, optionally substituted by 1 to 3 -F;
- -C(=O)-NR_{b}R_{c} wherein R_{b} is selected from -H, -CH₃ or -CH₂-CH₃ and wherein R_{c} is selected from -H, -CH₃, -CH₂-CH₃, cyclohexyl, phenyl or 5 to 6 membered heteroaryl, all optionally substituted by one or two halogen, -CN, -SO₂-CH₃ or C₁₋₄-alkyl, optionally substituted one or more times with halogen;
- -S-CR_{f}R_{g}Rₕ, wherein R_{f} is selected from -H or -F, wherein R_{g} is selected from -H, -F or -CH₃ and wherein Rₕ is selected from H, F, -CH₃ or C₅₋₆-cycloalkyl or phenyl;
- 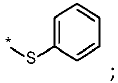
- R²: is selected from -H;
- R⁴: is selected from
- C₁₋₆-alkyl, C₃₋₆-cycloalkyl or C₁₋₃-alkyl-C₃₋₆-cycloalkyl, all optionally substituted one or independently more times by
   ∘ -F, -Cl, -Br, -CN;
   ∘ -OH, -ORᵢ wherein Rᵢ is selected from C₁₋₄-alkyl or C₃₋₆-Cycloalkyl, both optionally substituted one or more times by halogen,
   ∘ -CF₃, -CHF₂, -CH₂F, or -CH₂-CF₃;
   ∘ -NRⱼRₖ wherein Rⱼ and Rₖ are independently selected from -H and C₁-C₄-alkyl,
   ∘ -CH₂-O-CH₃ or
   ∘ C₃₋₄ cycloalkyl or 4-6 membered heterocycloalkyl, both optionally substituted by (=O), or optionally substituted one or more times by F or CH₃;
   ∘ C₆₋₈-spiro-alkyl and C₅₋₇-bicyclo-alkyl
   ∘ phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl; or
   ∘ phenyl is optionally substituted one or more times by -C(O)NRⱼRₖ wherein Rⱼ and Rₖ are independently selected from -H and C₁-C₄-alkyl or
   ∘ 5 to 6 membered heteroaryl is optionally substituted one or more times by -OH;
- C(=O)-R_{I} in which R_{I} is selected from
   ∘ C₁₋₄-alkyl, C₃₋₆-cycloalkyl or C₃₋₆-heterocycloalkyl, both optionally substituted one or more times by
      ▪ -F, -Cl, -Br, -CN;
      ▪ -OH, -ORᵢ with Rᵢ selected from C₁₋₄-alkyl or C₃₋₆Cycloalkyl), -CF₃, -CHF₂, -CH₂F;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CF₃, -CHF₂, -CH₂F,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   ∘ phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen; ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-NRₒRₚ wherein Rₒ and Rₚ are independently selected from
   ∘ H
   ∘ C₁₋₄-alkyl, C₃₋₆-cycloalkyl or C₁₋₃-alkyl-C₃₋₆-cycloalkyl, both optionally substituted one or more times by
      ▪ -F, -Cl, -Br, -CN;
      ▪ -OH, -ORᵢ with Rᵢ selected from C₁₋₄-alkyl or C₃₋₆-cycloalkyl, -CF₃, -CHF₂, -CH₂F;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CF₃, -CHF₂, -CH₂F,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   o phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
or a stereoisomer, a tautomer, an N-oxide, a hydrate, a solvate, or a salt thereof, or a mixture of same.

The compounds of formula (I) are particularly suitable for a large number of prophylactic and therapeutic applications, in particular for hyperproliferative disorders, for tumour disorders and as proteine inhibitors and further for viral infections, for neurodegenerative disorders, for inflammatory disorders, for atherosclerotic disorders and for male fertility control.

Further, it covers their use in combination with other anti cancer medications such as immunotherapeutics, targeted anti cancer agents, radiation or chemotherapy.

### DEFINITIONS

In case an asterix is used in a formula, like for instance in *-A-B or *-A-, this asterix indicates the bond towards the core of the compound.

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen or ... atom. Commonly, it is possible for the number of optional substituents, when present, to be 1, 2, 3, 4 or 5, in particular 1, 2 or 3.

As used herein, the term "one or more", e.g. in the definition of the substituents of the compounds of general formula (I) of the present invention, means "1, 2, 3, 4 or 5, particularly 1, 2, 3 or 4, more particularly 1, 2 or 3, even more particularly 1 or 2".

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one substituent.

As used herein, an oxo substituent represents an oxygen atom, which is bound to a carbon atom or to a sulfur atom via a double bond.

The term "ring substituent" means a substituent attached to an aromatic or nonaromatic ring which replaces an available hydrogen atom on the ring.

The term "comprising" when used in the specification includes "consisting of".

If within the present text any item is referred to as "as mentioned herein", it means that it may be mentioned anywhere in the present text.

The terms as mentioned in the present text have the following meanings:
The term "halogen atom" means a fluorine, chlorine, bromine or iodine atom, particularly a fluorine, chlorine or bromine atom.

The term "C₁-C₆-alkyl" means a linear or branched, saturated, monovalent hydrocarbon group having 1, 2, 3, 4, 5 or 6 carbon atoms, *e.g*. a methyl, ethyl, propyl, isopropyl, butyl, *sec*-butyl, isobutyl, *tert-*butyl, pentyl, isopentyl, 2-methylbutyl, 1-methylbutyl, 1-ethylpropyl, 1,2-dimethylpropyl, *neo*-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("C₁-C₄-alkyl"), *e.g*. a methyl, ethyl, propyl, isopropyl, butyl, *sec*-butyl isobutyl, or *tert-*butyl group, more particularly 1, 2 or 3 carbon atoms ("C₁-C₃-alkyl"), *e.g.* a methyl, ethyl, n-propyl or isopropyl group.

The term "C₁-C₆-hydroxyalkyl" means a linear or branched, saturated, monovalent hydrocarbon group in which the term "C₁-C₆-alkyl" is defined *supra,* and in which 1, 2 or 3 hydrogen atoms are replaced with a hydroxy group, e.g. a hydroxymethyl, 1-hydroxyethyl, 2-hydroxyethyl, 1,2-dihydroxyethyl, 3-hydroxypropyl, 2-hydroxypropyl, 1-hydroxypropyl, 1-hydroxypropan-2-yl, 2-hydroxypropan-2-yl, 2,3-dihydroxypropyl, 1,3-dihydroxypropan-2-yl, 3-hydroxy-2-methyl-propyl, 2-hydroxy-2-methyl-propyl, 1-hydroxy-2-methyl-propyl group.

The term "C₁-C₆-haloalkyl" means a linear or branched, saturated, monovalent hydrocarbon group in which the term "C₁-C₆-alkyl" is as defined *supra,* and in which one or more of the hydrogen atoms are replaced, identically or differently, with a halogen atom. Particularly, said halogen atom is a fluorine atom. Said C₁-C₆-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl. Preference is given to perfluorinated alkyl radicals which are named as "perfluoro-C₁-Cₓ-alkyl-" wherein x is the maximum number of carbon atoms such as trifluoromethyl or 2,2,2-trifluoroethyl.

The term "C₁-C₆-cyanoalkyl" means a linear or branched, saturated, monovalent hydrocarbon group in which the term "C₁-C₆-alkyl" is as defined *supra,* and in which one or more of the hydrogen atoms are replaced, identically or differently, with a cyano group.

The term "C₁-C₆-alkoxy" means a linear or branched, saturated, monovalent group of formula (C₁-C₆-alkyl)-O-, in which the term "C₁-C₆-alkyl" is as defined *supra, e.g.* a methoxy, ethoxy, *n*-propoxy, isopropoxy, *n*-butoxy, *sec*-butoxy, isobutoxy, *tert*-butoxy, pentyloxy, isopentyloxy or *n*-hexyloxy group, or an isomer thereof.

The term "C₁-C₆-haloalkoxy" means a linear or branched, saturated, monovalent C₁-C₆-alkoxy group, as defined *supra,* in which one or more of the hydrogen atoms is replaced, identically or differently, with a halogen atom. Particularly, said halogen atom is a fluorine atom. Said C₁-C₆-haloalkoxy group is, for example, fluoromethoxy, difluoromethoxy, trifluoromethoxy or 2,2,2-trifluoroethoxy. Preference is given to perfluorinated alkyl radicals which are named as "perfluoro-C₁-Cₓ-alkoxy-" wherein x is the maximum number of carbon atoms such as trifluoromethoxy and 2,2,2-trifluoroethoxy radicals.

The term "C₁-C₆-cyanoalkoxy" means a linear or branched, saturated, monovalent C₁-C₆-alkoxy group, as defined *supra,* in which one or more of the hydrogen atoms is replaced, identically or differently, with a cyano group.

Mono-(C₁-C₄)-alkylamino in the context of the invention means an amino group with one straight-chain or branched alkyl substituent which contains 1, 2, 3 or 4 carbon atoms, such as: methylamino, ethylamino, *n*-propylamino, isopropylamino, *n*-butylamino, and *tert*-butylamino, for example.

Di-(C₁-C₄)-alkylamino in the context of the invention means an amino group with two identical or different straight-chain or branched alkyl substituents which each contain 1, 2, 3 or 4 carbon atoms, such as: *N,N*-dimethylamino, *N,N*-diethylamino, *N*-ethyl-*N*-methylamino, *N*-methyl-*N*-*n*-propylamino, *N*-isopropyl-*N*-methylamino, *N*-isopropyl-*N*-*n*-propylamino, *N,N*-diisopropylamino, *N-n*-butyl-*N-*methylamino, and *N*-*tert*-butyl-*N*-methylamino, for example.

(C₁-C₄)-Alkylcarbonyl in the context of the invention means a straight-chain or branched alkyl group having having 1, 2, 3 or 4 carbon atoms which is bound to the rest of the molecule via a carbonyl group [-C(=O)-], such as: acetyl, propionyl, *n*-butyryl, isobutyryl, *n*-pentanoyl, and pivaloyl, for example.

(C₁-C₄)-Alkylcarbonyloxy in the context of the invention means a straight-chain or branched alkyl group having 1, 2, 3 or 4 carbon atoms which is bound to the rest of the molecule via a carboxy group [-C(=O)-O-], such as: acetoxy (=acyloxy), propionyloxy, *n*-butyryloxy, isobutyryloxy, *n*-pentanoyloxy, and pivaloyloxy, for example.

Mono-(C₁-C₄)-alkylaminocarbonyl in the context of the invention means an amino group which is bound to the rest of the molecule via a carbonyl group [-C(=O)-] and which has one straight-chain or branched alkyl substituent having 1, 2, 3 or 4 carbon atoms, such as: methylaminocarbonyl, ethylaminocarbonyl, *n*-propylaminocarbonyl, isopropylaminocarbonyl, *n*-butylaminocarbonyl, and *tert*-butylaminocarbonyl, for example.

Di-(C₁-C₄)-alkylaminocarbonyl in the context of the invention means an amino group which is bound to the rest of the molecule via a carbonyl group [-C(=O)-] and which has two identical or different straight-chain or branched alkyl substituents having in each case 1, 2, 3 or 4 carbon atoms, such as: *N,N*-dimethylaminocarbonyl, *N,N*-diethylaminocarbonyl, *N*-ethyl-*N*-methylaminocarbonyl, *N*-methyl-*N-n*-propylaminocarbonyl, *N*-isopropyl-*N*-methylaminocarbonyl, *N,N*-diisopropylaminocarbonyl, *N-n-*butyl-*N*-methylaminocarbonyl, and *N*-*tert*-butyl-*N*-methylaminocarbonyl, for example.

Mono-(C₁-C₄)-alkylaminosulfonyl in the context of the invention means an amino group which is bound to the rest of the molecule via a sulfonyl group [-S(=O)₂-] and which has one straight-chain or branched alkyl substituent having 1, 2, 3 or 4 carbon atoms, such as: methylaminosulfonyl, ethylaminosulfonyl, *n*-propylaminosulfonyl, isopropylaminosulfonyl, *n*-butylamino sulfonyl, and *tert-*butylaminosulfonyl, for example.

Di-(C₁-C₄)-alkylaminosulfonyl in the context of the invention means an amino group which is bound to the rest of the molecule via a sulfonyl group [-S(=O)₂-] and which has two identical or different straight-chain or branched alkyl substituents having in each case 1, 2, 3 or 4 carbon atoms, such as: *N,N*-dimethylaminosulfonyl, *N,N*-diethylaminosulfonyl, *N*-ethyl*-N*-methylaminosulfonyl, *N*-methyl-*N-n*-propylaminosulfonyl, *N*-isopropyl-*N*-methylaminosulfonyl, *N,N-*diisopropylaminosulfonyl, *N-n-*butyl-*N*-methylaminosulfonyl, and *N*-*tert*-butyl-*N*-methylaminosulfonyl, for example.

The term "C₃-C₈-cycloalkyl" means a saturated, monovalent, mono- or bicyclic hydrocarbon ring which contains 3, 4, 5, 6, 7 or 8 carbon atoms ("C₃-C₈-cycloalkyl"). Said C₃-C₈-cycloalkyl group is for example, a monocyclic hydrocarbon ring, *e.g*. a cyclopropyl, cyclobutyl, cyclopentyl, cyclohexyl, cycloheptyl or cyclooctyl group, or a bicyclic hydrocarbon ring, *e.g*. a bicyclo[4.2.0]octyl or octahydropentalenyl.

The term "C₃-C₈-cycloalkoxy" means a saturated, monovalent, mono- or bicyclic group of formula (C₃-C₈-cycloalkyl)-O-, which contains 3, 4, 5, 6, 7 or 8 carbon atoms, in which the term "C₃-C₈-cycloalkyl" is defined *supra, e.g.* a cyclopropyloxy, cyclobutyloxy, cyclopentyloxy, cyclohexyloxy, cycloheptyloxy or cyclooctyloxy group.

The terms "4- to 7-membered heterocycloalkyl" and "4- to 6-membered heterocycloalkyl" mean a monocyclic, saturated or unsaturated heterocycle with 4, 5, 6 or 7 or, respectively, 4, 5 or 6 ring atoms in total, which contains one or two identical or different ring heteroatoms from the series N, O and S, it being possible for said heterocycloalkyl group to be attached to the rest of the molecule via any one of the carbon atoms or, if present, a nitrogen atom. A carbon atom may be substituted with an oxo group or or the sulphur atom with one or two oxo groups to form a -C=O, -S(=O)- or - S(=O)₂-group in the ring.

Said heterocycloalkyl group, without being limited thereto, can be a 4-membered ring, such as azetidinyl, oxetanyl or thietanyl, for example; or a 5-membered ring, such as tetrahydrofuranyl, 1,3-dioxolanyl, thiolanyl, pyrrolidinyl, imidazolidinyl, pyrazolidinyl, 1,1-dioxidothiolanyl, 1,2-oxazolidinyl, 1,3-oxazolidinyl or 1,3-thiazolidinyl, for example; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl or 1,2-oxazinanyl, for example, or a 7-membered ring, such as azepanyl, 1,4-diazepanyl or 1,4-oxazepanyl, for example.

Particularly, "4- to 6-membered heterocycloalkyl" means a 4- to 6-membered heterocycloalkyl as defined *supra* containing one ring nitrogen atom and optionally one further ring heteroatom from the series: N, O, S. More particularly, "5- or 6-membered heterocycloalkyl" means a monocyclic, saturated heterocycle with 5 or 6 ring atoms in total, containing one ring nitrogen atom and optionally one further ring heteroatom from the series: N, O.

The term "bridged heterocycloalkyl" means a bicyclic, saturated or unsaturated heterocycle with 7, 8, 9 or 10 ring atoms in total (= "bridged bicyclic 7- to 10-membered heterocycloalkyl"), in which the two rings share two common ring atoms which are not adjacent, which "bridged heterocycloalkyl" contains one or two identical or different ring heteroatoms from the series: N, O, S; it being possible for said bridged heterocycloalkyl group to be attached to the rest of the molecule via any one of the carbon atoms, except the spiro carbon atom, or, if present, a nitrogen atom. A carbon atom may be substituted with an oxo group or or the sulphur atom with one or two oxo groups to form a -C=O, - S(=O)- or -S(=O)₂-group in the ring.

Said bridged heterocycloalkyl group is, for example, azabicyclo[2.2.1]heptyl, oxazabicyclo[2.2.1]heptyl, thiazabicyclo[2.2.1]heptyl, diazabicyclo[2.2.1]heptyl, azabicyclo-[2.2.2]octyl, diazabicyclo[2.2.2]octyl, oxazabicyclo[2.2.2]octyl, thiazabicyclo[2.2.2]octyl, azabicyclo[3.2.1]octyl, diazabicyclo[3.2.1]octyl, oxazabicyclo[3.2.1]octyl, thiazabicyclo[3.2.1]octyl, azabicyclo[3.3.1]nonyl, diazabicyclo[3.3.1]nonyl, oxazabicyclo[3.3.1]nonyl, thiazabicyclo[3.3.1]nonyl, azabicyclo[4.2.1]nonyl, diazabicyclo[4.2.1]nonyl, oxazabicyclo[4.2.1]nonyl, thiazabicyclo[4.2.1]nonyl, azabicyclo[3.3.2]decyl, diazabicyclo[3.3.2]decyl, oxazabicyclo[3.3.2]decyl, thiazabicyclo[3.3.2]decyl or azabicyclo[4.2.2]decyl.

The term "heteroaryl" means a monovalent, monocyclic, bicyclic or tricyclic aromatic ring having 5, 6, 8, 9, 10, 11, 12, 13 or 14 ring atoms (a "5- to 14-membered heteroaryl" group), particularly 5, 6, 9 or 10 ring atoms, which contains at least one ring heteroatom and optionally one, two or three further ring heteroatoms from the series: N, O and/or S, and which is bound via a ring carbon atom or optionally via a ring nitrogen atom (if allowed by valency).

Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl, pyridazinyl, pyrimidinyl, pyrazinyl or triazinyl; or a tricyclic heteroaryl group, such as, for example, carbazolyl, acridinyl or phenazinyl; or a 9-membered heteroaryl group, such as, for example, benzofuranyl, benzothienyl, benzoxazolyl, benzisoxazolyl, benzimidazolyl, benzothiazolyl, benzotriazolyl, indazolyl, indolyl, isoindolyl, indolizinyl or purinyl; or a 10-membered heteroaryl group, such as, for example, quinolinyl, quinazolinyl, isoquinolinyl, cinnolinyl, phthalazinyl, quinoxalinyl or pteridinyl.

In general, and unless otherwise mentioned, the heteroaryl or heteroarylene groups include all possible isomeric forms thereof, *e.g.*: tautomers and positional isomers with respect to the point of linkage to the rest of the molecule. Thus, for some illustrative non-restricting examples, the term pyridinyl includes pyridin-2-yl, pyridin-3-yl and pyridin-4-yl; or the term thienyl includes thien-2-yl and thien-3-yl.

Particularly, the heteroaryl group is a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group group such as, for example, pyridinyl (=pyridyl), pyridazinyl, pyrimidinyl, pyrazinyl or triazinyl.

The term "C₁-C₆", as used in the present text, e.g. in the context of the definition of "C₁-C₆-alkyl", "C₁-C₆-haloalkyl", "C₁-C₆-hydroxyalkyl", "C₁-C₆-alkoxy" or "C₁-C₆-haloalkoxy" means an alkyl group having a finite number of carbon atoms of 1 to 6, *i.e.* 1, 2, 3, 4, 5 or 6 carbon atoms.

Further, as used herein, the term "C₃-C₈", as used in the present text, e.g. in the context of the definition of "C₃-C₈-cycloalkyl", means a cycloalkyl group having a finite number of carbon atoms of 3 to 8, *i.e.* 3, 4, 5, 6, 7 or 8 carbon atoms.

When a range of values is given, said range encompasses each value and sub-range within said range.

For example:
"C₁-C₆" encompasses C₁, C2, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆;
"C₂-C₆" encompasses C₂, C₃, C₄, C₅, C₆, C₂-C₆, C₂-C₅, C₂-C₄, C₂-C₃, C₃-C₆, C₃-C₅, C₃-C₄, C₄-C₆, C₄-C₅, and C₅-C₆;
"C₃-C₁₀" encompasses C₃, C₄, C₅, C₆, C₇, C₈, C₉, C₁₀, C₃-C₁₀, C₃-C₉, C₃-C₈, C₃-C₇, C₃-C₆, C₃-C₅, C₃-C₄, C₄-C₁₀, C₄-C₉, C₄-C₈, C₄-C₇, C₄-C₆, C₄-C₅, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₃-C₈" encompasses C₃, C₄, C₅, C₆, C₇, C₈, C₃-C₈, C₃-C₇, C₃-C₆, C₃-C₅, C₃-C₄, C₄-C₈, C₄-C₇, C₄-C₆, C₄-C₅, C₅-C₈, C₅-C₇, C₅-C₆, C₆-C₈, C₆-C₇ and C₇-C₈;
"C₃-C₆" encompasses C₃, C₄, C₅, C₆, C₃-C₆, C₃-C₅, C₃-C₄, C₄-C₆, C₄-C₅, and C₅-C₆;
"C₄-C₈" encompasses C₄, C₅, C₆, C₇, C₈, C₄-C₈, C₄-C₇, C₄-C₆, C₄-C₅, C₅-C₈, C₅-C₇, C₅-C₆, C₆-C₈, C₆-C₇ and C₇-C₈;
"C₄-C₇" encompasses C₄, C₅, C₆, C₇, C₄-C₇, C₄-C₆, C₄-C₅, C₅-C₇, C₅-C₆ and C₆-C₇;
"C₄-C₆" encompasses C₄, C₅, C₆, C₄-C₆, C₄-C₅ and C₅-C₆;
"C₅-C₁₀" encompasses C₅, C₆, C₇, C₈, C₉, C₁₀, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₆-C₁₀" encompasses C₆, C₇, C₈, C₉, C₁₀, C₆-C₁₀, C₆-C₉, C₆-C₈, C₆-C₇, C₇-C₁₀, C₇-C₉, C₇-C₈, C₈-C₁₀, C₈-C₉ and C₉-C₁₀.

As used herein, the term "leaving group" means an atom or a group of atoms that is displaced in a chemical reaction as stable species taking with it the bonding electrons. In particular, such a leaving group is selected from the group comprising: halide, in particular fluoride, chloride, bromide or iodide, (methylsulfonyl)oxy, [(trifluoromethyl)sulfonyl]oxy, [(nonafluorobutyl)sulfonyl]oxy, (phenylsulfonyl)oxy, [(4-methylphenyl)sulfonyl]oxy, [(4-bromophenyl)sulfonyl]oxy, [(4-nitrophenyl)sulfonyl]oxy, [(2-nitrophenyl)sulfonyl]oxy, [(4-isopropylphenyl)sulfonyl]oxy, [(2,4,6-triisopropylphenyl)sulfonyl]oxy, [(2,4,6-trimethylphenyl)sulfonyl]oxy, [(4-*tert*-butylphenyl)sulfonyl]oxy and [(4-methoxyphenyl)sulfonyl]oxy.

Where the plural form of the word compounds, salts, polymorphs, hydrates, solvates and the like, is used herein, this is taken to mean also a single compound, salt, polymorph, isomer, hydrate, solvate or the like.

By "stable compound' or "stable structure" is meant a compound that is sufficiently robust to survive isolation to a useful degree of purity from a reaction mixture, and formulation into an efficacious therapeutic agent.

The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds.

Preferred compounds are those which produce the more desirable biological activity. Separated, pure or partially purified stereoisomers or racemic or diastereomeric mixtures of the compounds of the present invention are also included within the scope of the present invention. The purification and the separation of such materials can be accomplished by standard techniques known in the art.

Preferred stereoisomers are those which produce the more desirable biological activity. These separated, pure or partially purified stereoisomers or racemic mixtures of the compounds of this invention are also included within the scope of the present invention. The purification and the separation of such materials can be accomplished by standard techniques known in the art. The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g*., HPLC columns using a chiral phase), with or without conventional derivatisation, optimally chosen to maximise the separation of the enantiomers. Suitable HPLC columns using a chiral phase are commercially available, such as those manufactured by Daicel, *e.g*., Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.

In order to distinguish different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g*. (R)- or (S)- isomers, in any ratio. Isolation of a single stereoisomer, *e.g*. a single enantiomer or a single diastereomer, of a compound of the present invention is achieved by any suitable state of the art method, such as chromatography, especially chiral chromatography, for example.

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also covers useful forms of the compounds of the present invention, such as metabolites, hydrates, solvates, prodrugs, salts, in particular pharmaceutically acceptable salts, and/or co-precipitates.

The compounds of the present invention can exist as a hydrate, or as a solvate, wherein the compounds of the present invention contain polar solvents, in particular water, methanol or ethanol for example, as structural element of the crystal lattice of the compounds. It is possible for the amount of polar solvents, in particular water, to exist in a stoichiometric or non-stoichiometric ratio. In the case of stoichiometric solvates, *e.g*. a hydrate, hemi-, (semi-), mono-, sesqui-, di-, tri-, tetra-, penta- *etc.* solvates or hydrates, respectively, are possible. The present invention includes all such hydrates or solvates.

Further, it is possible for the compounds of the present invention to exist in free form, *e.g*. as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.

The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, para-toluenesulfonic, methanesulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, *N*-methylmorpholine, arginine, lysine, 1,2-ethylenediamine, *N*-methylpiperidine, *N-*methyl-glucamine, *N,N*-dimethyl-glucamine, *N*-ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(*n*-propyl)ammonium, tetra(*n*-butyl)ammonium, *N*-benzyl-*N,N,N*-trimethylammonium, choline or benzalkonium.

Those skilled in the art will further recognise that it is possible for acid addition salts of the claimed compounds to be prepared by reaction of the compounds with the appropriate inorganic or organic acid via any of a number of known methods. Alternatively, alkali and alkaline earth metal salts of acidic compounds of the present invention are prepared by reacting the compounds of the present invention with the appropriate base via a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.

This applies analogously to cases in which synthesis intermediates or example compounds or salts thereof have been obtained, by the preparation and/or purification processes described, as solvates, such as hydrates, with (if defined) unknown stoichiometric composition.

Furthermore, the present invention includes all possible crystalline forms, or polymorphs, of the compounds of the present invention, either as single polymorph, or as a mixture of more than one polymorph, in any ratio.

Moreover, the non-claimed disclosure also includes prodrugs of the compounds according to the invention. The term "prodrugs" here designates compounds which themselves can be biologically active or inactive, but are converted (for example metabolically or hydrolytically) into compounds according to the invention during their residence time in the body.

The invention further includes all possible crystallized and polymorphic forms of the inventive compounds, whereby the polymorphs are existing either as a single polymorph form or are existing as a mixture of several polymorphs in all concentrations.

The invention further includes all possible cyclodextrin clathrates, i.e alpha-, beta-, or gamma-cyclodextrins, hydroxypropyl-beta-cyclodextrins, methylbetacyclodextrins.

The invention also covers the following embodimdents:

### Embodiment 2:

Compound of formula (la) 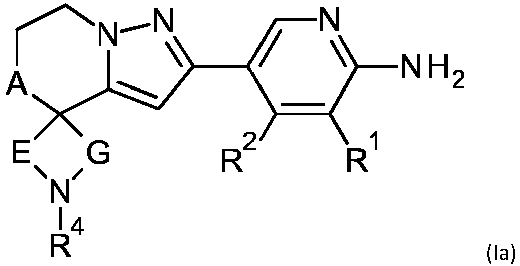 wherein
- A: is selected from a single bond, -CH₂- or -O-;
- E: is -(CH₂)ₙ- with n = 1 or 2;
- G: is -(CH₂)- and with the condition, that the cyclic substructure comprising E-N-G is a 4 to 5 membered heterocycloalkyl;
- R¹: is selected from
- -NO₂; -CN;
- C₁₋₄-alkyl optionally substituted by -OH or 1 to 3 -F;
- cyclopropyl;
- 4 to 6 membered heterocycloalkyl or heterocycloalkenyl, both with 1- 2 nitrogen atoms, but always linked via a carbon atom to the phenyl group of compounds of formula (I) and both optionally substituted by C₁₋₄-alkyl;
- phenyl optionally substituted by halogen, -CN, C₁₋₄-alkyl, C₁₋₄-haloalkyl, C₃₋₆-cycloalkyl, O-C₁₋₄-alkyl, O-C₃₋₆-cycloalky, C₁₋₃-alkinyl, -C(O)-R_{d}, SR_{d}, SOR_{d}, SO₂R_{d}, SONR_{d}, SON(H)R_{d}, -SO₂-NH₂, - SO₂-N(H)-R_{d} or -SO₂-N(R_{d})₂, wherein R_{d} is selected from methyl, ethyl, cyclopropyl, isopropyl and tert. butyl;
- 5 to 6 membered heteroaryl with 1 or 2 heteroatoms selected from N or S and optionally substituted by halogen, -CN, C₁₋₄-alkyl, C₁₋₄-haloalkyl, C₃₋₆-cycloalkyl, O-C₁₋₄-alkyl, O-C₃₋₆-cycloalkyl, C₁₋₃-alkinyl, SR_{d}, SOR_{d}, SO₂R_{d}, SONR_{d} or SON(H)R_{d} wherein R_{d} is selected from methyl, ethyl, cyclopropyl, isopropyl and tert. butyl;
- -O-CH₃; -O-CH(CH₃)₂; -O-CHF₂; -O-CF₃; 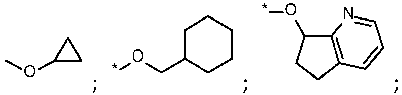
- -O-CHRₐ-phenyl optionally substituted by one or two of the following substituents: halogen, - CN, C₁₋₄-alkyl optionally substituted by one or more -F or -SO₂-CH₃ and with Rₐ selected from -H, -F or -CH₃, -CF₃, -CH₂-CH₃, -CH(CH₃)₂ or -cyclopropyl;
- -O-CHRₐ-6 membered heteroaryl optionally substituted one or two of the following substituents: halogen, -CN, C₁₋₄-alkyl optionally substituted by one or more -F or -SO₂-CH₃ and with Rₐ selected from -H, -F or -CH₃, -CF₃, -CH₂-CH₃, -CH(CH₃)₂ or -cyclopropyl;
- -COORₐ with Rₐ selected from -H or -CH₃;
- C(=O)-methyl, optionally substituted by 1 to 3 -F;
- -C(=O)-NR_{b}R_{c} wherein R_{b} is selected from -H, -CH₃ or -CH₂-CH₃ and wherein R_{c} is selected from -H, -CH₃, -CH₂-CH₃, cyclohexyl, phenyl or 5 to 6 membered heteroaryl, all optionally substituted by one or two halogen, -CN, -SO₂-CH₃ or C₁₋₄-alkyl, optionally substituted one or more times with halogen;
- -S-CR_{f}R_{g}Rₕ, wherein R_{f} is selected from -H or -F, wherein R_{g} is selected from -H, -F or -CH₃ and wherein Rₕ is selected from H, F, -CH₃ or C₅₋₆-Cycloalkyl or phenyl;
- 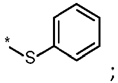
- R²: is selected from -H,; or
- R⁴: is selected from
- C₁₋₅-alkyl or C₃₋₆-cycloalkyl, both optionally substituted one or independently more times by
   ∘ -F, -Cl, -Br, -CN;
   ∘ -OH, -ORᵢ wherein Rᵢ is selected from C₁₋₄-alkyl or C₃₋₆Cycloalkyl),
   ∘ -CF₃, -CHF₂, -CH₂F, or -CH₂-CF₃;
   ∘ -NRⱼRₖ wherein Rⱼ and Rₖ are independently selected from -H and C₁-C₄-alkyl,
   ∘ -CH₂-O-CH₃ or
   ∘ C₃₋₄ cycloalkyl or 4 membered heterocycloalkyl, both optionally substituted by -F or - CH₃;
   ∘ phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-Rₗ in which Rₗ is selected from
   ∘ C₁₋₄-alkyl, C₃₋₆-cycloalkyl or C₃₋₆-heterocycloalkyl, both optionally substituted one or more times by
      ▪ -F, -Cl, -Br, -CN;
      ▪ -OH, -ORᵢ with Rᵢ selected from C₁₋₄-alkyl or C₃₋₆Cycloalkyl), -CF₃, -CHF₂, -CH₂F;
      ▪ -NRⱼRₖ wherein Rⱼ and Rₖ are independently selected from -H and C₁-C₄-alkyl ▪ phenyl or 5 to 6 membered heteroaryl, both optionally substituted one or more times by
         - halogen, -CN,
         - -CF₃, -CHF₂, -CH₂F,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   ∘ phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-NRₒRₚ wherein Rₒ and Rₚ are independently selected from
   ∘ H
   ∘ C₁₋₄-alkyl or C₃₋₆-cycloalkyl, both optionally substituted one or more times by
      ▪ -F, -Cl, -Br, -CN;
      ▪ -OH, -ORᵢ with Rᵢ selected from C₁₋₄-alkyl or C₃₋₆-cycloalkyl, -CF₃, -CHF₂, -CH₂F;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CF₃, -CHF₂, -CH₂F,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   ∘ phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
or a stereoisomer, a tautomer, an N-oxide, a hydrate, a solvate, or a salt thereof, or a mixture of same.

### Embodiment 3:

Compounds of formula (I) according to embodiment 1, wherein A is selected from a single bond or - O- or a stereoisomer, a tautomer, an N-oxide, a hydrate, a solvate, or a salt thereof, or a mixture of same.

### Embodiment 4:

Compounds of formula (I) according to embodiment 1, wherein E is -CH₂-CH₂- or a stereoisomer, a tautomer, an N-oxide, a hydrate, a solvate, or a salt thereof, or a mixture of same.

### Embodiment 5:

Compounds of formula (I) according to embodiment 1, wherein Q is C-R² or a stereoisomer, a tautomer, an N-oxide, a hydrate, a solvate, or a salt thereof, or a mixture of same.

### Embodiment 6:

Compounds of formula (I) according to embodiment 1, wherein Q is N or a stereoisomer, a tautomer, an N-oxide, a hydrate, a solvate, or a salt thereof, or a mixture of same.

### Embodiment 7:

Compounds of formula (I) according to embodiment 1, wherein R₄ is 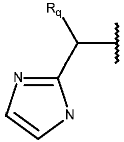 and R_{q} is selected from H, -CH₃,-CH₂-CH₃, -CH(CH₃)₂, -CH₂-O-CH₃, -CF₃, -CHF₂, -CH₂F, -CH₂-CF₃, cyclopropyl or cyclobutyl or a stereoisomer, a tautomer, an N-oxide, a hydrate, a solvate, or a salt thereof, or a mixture of same.

### Embodiment 8:

Compounds of formula (I) according to embodiment 1, wherein R₄ is selected from C(=O)-NRₒRₚ wherein Rₒ and Rₚ are independently selected from
∘ H
∘ C₁₋₄-alkyl or C₃₋₆-cycloalkyl, both optionally substituted one or more times by
   ▪ -F, -Cl, -Br, -CN;
   ▪ -OH, -ORᵢ with Rᵢ selected from C₁₋₄-alkyl or C₃₋₆-cycloalkyl, -CF₃, -CHF₂, -CH₂F;
   ▪ -NRⱼRₖ wherein Rⱼ and Rₖ are independently selected from -H and C₁-C₄-alkyl
   ▪ phenyl or 5 to 6 membered heteroaryl, both optionally substituted one or more times by
      - halogen, -CN,
      - -CF₃, -CHF₂, -CH₂F,
      - C₁-₄-alkyl optionally substituted with one or more halogen;
      - C₃₋₆-cycloalkyl;
      - O-C₁₋₄-alkyl,
      - O-C₃₋₆-cycloalky,
      - C₁₋₃-alkinyl,
      - -NRⱼRₖ wherein Rⱼ and Rₖ are independently selected from -H and C₁-C₄-alkyl
      - SR_{d}, SOR_{d}, SO₂R_{d}, SONR_{d} or SON(H)R_{d} wherein R_{d} is selected from methyl, ethyl, cyclopropyl, isopropyl and tert. butyl;
or a stereoisomer, a tautomer, an N-oxide, a hydrate, a solvate, or a salt thereof, or a mixture of same.

### Embodiment 9:

Compound according to embodiment 1, which is selected from:
(rac)-2'-[6-amino-5-(trifluoromethyl)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-(1-phenylcyclobutyl)-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-(1-phenylcyclobutyl)-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(rac)-2'-(5-acetyl-6-aminopyridin-3-yl)-N-(1-phenylcyclobutyl)-5',6'-dihydrospiro[pyrrolidine-3,4'-pyrrolo[1,2-b]pyrazole]-1-carboxamide
(rac)-2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide
(rac)-2'-(6-amino-5-cyanopyridin-3-yl)-N-[1-(2-chlorophenyl)cyclobutyl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
(rac)-2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(rac)-2'-(6-amino-5-cyanopyridin-3-yl)-N-(1-phenylcyclobutyl)-5',6'-dihydrospiro[pyrrolidine-3,4'-pyrrolo[1,2-b]pyrazole]-1-carboxamide
(rac)-2-(6-amino-5-cyanopyridin-3-yl)-N-[1-(4-fluorophenyl)cyclobutyl]-6,7-dihydrospiro[pyrazolo[5,1-c][1,4]oxazine-4,3'-pyrrolidine]-1'-carboxamide
(rac)-2'-(6-amino-5-cyanopyridin-3-yl)-N-[1-(4-fluorophenyl)cyclobutyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide
(rac)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide
(rac)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide
(rac)-2-(6-amino-5-cyanopyridin-3-yl)-N-(1-phenylcyclobutyl)-6,7-dihydrospiro[pyrazolo[5,1-c][1,4]oxazine-4,3'-pyrrolidine]-1'-carboxamide
(rac)-2-(6-amino-5-cyanopyridin-3-yl)-N-[2-(4-fluorophenyl)propan-2-yl]-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[1-(3-fluorophenyl)cyclobutyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-{(1R)-1-[3-(trifluoromethyl)phenyl]ethyl}-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(2-fluorophenyl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(3-fluorophenyl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-phenylpropyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-{(1S)-1-[3-(trifluoromethyl)phenyl]ethyl}-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(2-cyanophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1,3,5-trimethyl-1H-pyrazol-4-yl)ethyl]-5',6'-dihydrospiro[pyrrolidine-3,4'-pyrrolo[1,2-b]pyrazole]-1-carboxamide (diastereomeric mixture)
2'-[6-amino-5-(trifluoromethyl)pyridin-3-yl]-N-[(1R)-2,3-dihydro-1H-inden-1-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(2-cyanopyridin-4-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-ethyl-3,5-dimethyl-1H-pyrazol-4-yl)ethyl]-5',6'-dihydrospiro[pyrrolidine-3,4'-pyrrolo[1,2-b]pyrazole]-1-carboxamide (diastereometric mixture)
(*rac*)-2'-[6-amino-5-(trifluoromethyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1,3,4-trimethyl-1H-pyrazol-5-yl)ethyl]-5',6'-dihydrospiro[pyrrolidine-3,4'-pyrrolo[1,2-b]pyrazole]-1-carboxamide (diastereometric mixture)
2'-[6-amino-5-(trifluoromethyl)pyridin-3-yl]-N-[(1R)-1-(4-cyanopyridin-2-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(pyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-{[3-(trifluoromethyl)phenyl]methyl}-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-phenyl-1H-pyrazol-4-yl)ethyl]-5',6'-dihydrospiro[pyrrolidine-3,4'-pyrrolo[1,2-b]pyrazole]-1-carboxamide (diastereometric mixture)
2'-[6-amino-5-(trifluoromethyl)pyridin-3-yl]-N-[(3S)-2,3-dihydro-1-benzofuran-3-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(6-cyanopyridin-3-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-{(1S)-1-[1-methyl-3-(trifluoromethyl)-1H-pyrazol-4-yl]ethyl}-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(pyridin-2-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-benzyl-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-phenylethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1,2-oxazol-3-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1,5-dimethyl-1H-pyrazol-4-yl)ethyl]-5',6'-dihydrospiro[pyrrolidine-3,4'-pyrrolo[1,2-b]pyrazole]-1-carboxamide (diastereomeric mixture)
(*rac*)-2'-[6-amino-5-(trifluoromethyl)pyridin-3-yl]-N-[2-(oxan-4-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(pyridin-4-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1-ethyl-5-methyl-1H-pyrazol-4-yl)ethyl]-5',6'-dihydrospiro[pyrrolidine-3,4'-pyrrolo[1,2-b]pyrazole]-1-carboxamide (diastereomeric mixture)
(*rac*)-2'-[6-amino-5-(trifluoromethyl)pyridin-3-yl]-N-[(pyridin-3-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-2-methoxy-1-phenylethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-{[3-(trifluoromethyl)bicyclo[1.1.1]pentan-1-yl]methyl}-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(pyridin-4-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(3-cyanopyridin-2-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1-methyl-1H-benzimidazol-2-yl)ethyl]-5',6'-dihydrospiro[pyrrolidine-3,4'-pyrrolo[1,2-b]pyrazole]-1-carboxamide (diastereomeric mixture)
2'-[6-amino-5-(trifluoromethyl)pyridin-3-yl]-N-[1-(4-fluorophenyl)-2-methoxyethyl]-5',6'-dihydrospiro[pyrrolidine-3,4'-pyrrolo[1,2-b]pyrazole]-1-carboxamide (mixture of isomers)
(*rac*)-2'-[6-amino-5-(trifluoromethyl)pyridin-3-yl]-N-[(pyridin-2-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1-ethyl-1H-imidazol-2-yl)ethyl]-5',6'-dihydrospiro[pyrrolidine-3,4'-pyrrolo[1,2-b]pyrazole]-1-carboxamide (diastereomeric mixture)
2'-[6-amino-5-(trifluoromethyl)pyridin-3-yl]-N-[(1S)-1-(1-methyl-1H-imidazol-2-yl)ethyl]-5',6'-dihydrospiro[pyrrolidine-3,4'-pyrrolo[1,2-b]pyrazole]-1-carboxamide (diastereomeric mixture)
(*rac*)-2'-[6-amino-5-(trifluoromethyl)pyridin-3-yl]-N-[(pyrazin-2-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-2-methoxy-1-(pyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(pyridazin-4-yl)methyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(1-methylpiperidin-4-yl)methyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[3-(methylsulfamoyl)pyridin-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(1-methyl-2-oxabicyclo[2.1.1]hexan-4-yl)methyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(1-methyl-2-oxopiperidin-4-yl)methyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(2-cyclopropylpropan-2-yl)-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(4-cyanophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-methyl-2,3-dihydro-1H-inden-1-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[-2-methyl-1-phenylpropyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[2,2,2-trifluoro-1-phenylethyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[1-(pyrazin-2-yl)ethyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[(1R)-2-methoxy-1-phenylethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(pyridazin-3-yl)methyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(2-cyanophenyl)cyclobutyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-2,3-dihydro-1H-inden-1-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(4-fluorophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(2-cyanophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(6-cyanopyridin-3-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(pyridin-2-yl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyridin-4-yl)methyl]-5',6'-dihydrospiro[azetidine-3,4'-pyrrolo[1,2-b]pyrazole]-1-carboxamide
(3'RS)-2-[6-amino-5-(trifluoromethyl)pyridin-3-yl]-N-[(1R)-1-(4-cyanophenyl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(3-cyanopyridin-2-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(4-cyanophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(2-chloro-4-cyanophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(2-fluorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1-ethyl-3,5-dimethyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(pyridin-2-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1-methyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1,3,4-trimethyl-1H-pyrazol-5-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-{1-[3-(difluoromethyl)-1-methyl-1H-pyrazol-4-yl]ethyl}-5',6'-dihydrospiro[azetidine-3,4'-pyrrolo[1,2-b]pyrazole]-1-carboxamide
(*rac*)-2-[6-amino-5-(trifluoromethyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[1-(1-ethyl-5-methyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1,5-dimethyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1,3,5-trimethyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(pyridin-3-yl)ethyl]-5',6'-dihydrospiro[azetidine-3,4'-pyrrolo[1,2-b]pyrazole]-1-carboxamide
2-[6-amino-5-(trifluoromethyl)pyridin-3-yl]-N-[(1R)-1-(2-cyanopyridin-4-yl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(3-cyanopyridin-2-yl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(6-cyanopyridin-3-yl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(pyridin-2-yl)methyl]-5',6'-dihydrospiro[azetidine-3,4'-pyrrolo[1,2-b]pyrazole]-1-carboxamide
(*rac*)-2-[6-amino-5-(trifluoromethyl)pyridin-3-yl]-N-[(1R)-1-(2-cyanophenyl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(4-fluorophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(4-fluorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(pyridin-3-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(2-fluorophenyl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(pyridin-3-yl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(4-fluorophenyl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(pyridin-4-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(3-fluorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1S)-1-(3-chloropyridin-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1-methyl-2-oxabicyclo[2.1.1]hexan-4-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-{[3-(trifluoromethyl)bicyclo[1.1.1]pentan-1-yl]methyl}-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(2-chlorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(3-fluorophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(3-fluoropyridin-2-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(2-chlorophenyl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(3-fluorophenyl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-phenylpropyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyrimidin-4-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-fluoropyridin-2-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyrimidin-5-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-chloropyridin-4-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-fluorophenyl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyridin-3-yl)methyl]-5',6'-dihydrospiro[azetidine-3,4'-pyrrolo[1,2-b]pyrazole]-1-carboxamide
(*rac*)-2-[6-amino-5-(trifluoromethyl)pyridin-3-yl]-N-benzyl-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[(1R)-1-(2-methylpyrimidin-5-yl)ethyl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-[(1R)-1-(pyridin-3-yl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-benzyl-5',6'-dihydrospiro[azetidine-3,4'-pyrrolo[1,2-b]pyrazole]-1-carboxamide
2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide (enantiomer 1)
2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (enantiomer 1)
2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide (enantiomer 2)
2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (enantiomer 2)
2-[6-amino-5-(trifluoromethyl)pyridin-3-yl]-N-[(1R)-1-(4-cyanopyridin-2-yl)ethyl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[2-(1-methyl-1H-1,2,3-triazol-5-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(4-fluorophenyl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-2,3-dihydro-1H-inden-1-yl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(5-fluoropyridin-3-yl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(3-fluorophenyl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-phenylpropyl]-6,7-dihydrospiro[pyrazolo[5,1-c][1,4]oxazine-4,3'-pyrrolidine]-1'-carboxamide
(3'RS)-2-[6-amino-5-(trifluoromethyl)pyridin-3-yl]-N-[(35)-2,3-dihydro-1-benzofuran-3-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[(bicyclo[1.1.1]pentan-1-yl)methyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(1,2-oxazol-3-yl)ethyl]-5',6'-dihydrospiro[azetidine-3,4'-pyrrolo[1,2-b]pyrazole]-1-carboxamide
2-[6-amino-5-(trifluoromethyl)pyridin-3-yl]-N-[(1R)-1-(2-fluorophenyl)ethyl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(1-phenyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1S)-2-methoxy-1-phenylethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-fluorobicyclo[1.1.1]pentan-1-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(bicyclo[1.1.1]pentan-1-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1S)-1-(pyridin-3-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(2-methylpyrimidin-5-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(3-chloropyridin-4-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(pyridin-4-yl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-phenylethyl]-5',6'-dihydrospiro[azetidine-3,4'-pyrrolo[1,2-b]pyrazole]-1-carboxamide
2-[6-amino-5-(trifluoromethyl)pyridin-3-yl]-N-[(1S)-1-(pyridin-3-yl)ethyl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[(3-fluorobicyclo[1.1.1]pentan-1-yl)methyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1S)-1-(pyridin-4-yl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-(1-phenylcyclobutyl)-5',6'-dihydrospiro[azetidine-3,4'-pyrrolo[1,2-b]pyrazole]-1-carboxamide
2'-(6-amino-5-cyanopyridin-3-yl)-N-[1-(pyridin-4-yl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[1-(3-fluorophenyl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[2-(3-chloropyridin-4-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(5-fluoropyridin-3-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(4-fluorophenyl)ethyl]-5',6'-dihydrospiro[azetidine-3,4'-pyrrolo[1,2-b]pyrazole]-1-carboxamide
2'-(6-amino-5-cyanopyridin-3-yl)-N-[2-(3-fluorophenyl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[(1S)-1-(3-chloropyridin-4-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1-methyl-2-oxabicyclo[2.1.1]hexan-4-yl)methyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(4-cyanopyridin-2-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(2-cyanopyridin-4-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(2-chloro-4-cyanophenyl)ethyl]-5',6'-dihydrospiro[azetidine-3,4'-pyrrolo[1,2-b]pyrazole]-1-carboxamide
2'-(6-amino-5-cyanopyridin-3-yl)-N-[1-(2-fluorophenyl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[2-(4-fluorophenyl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[1-(2-chlorophenyl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[2-(pyridin-2-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[2-(pyridin-4-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[(1R)-1-phenylpropyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(3-fluorophenyl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(2-cyanophenyl)ethyl]-5',6'-dihydrospiro[azetidine-3,4'-pyrrolo[1,2-b]pyrazole]-1-carboxamide
(*rac*)-2'-(6-amino-5-cyanopyridin-3-yl)-N-[2-(pyridin-4-yl)propan-2-yl]-5',6'-dihydrospiro[pyrrolidine-3,4'-pyrrolo[1,2-b]pyrazole]-1-carboxamide
2'-(6-amino-5-cyanopyridin-3-yl)-N-[(1R)-1-(5-fluoropyridin-3-yl)ethyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(pyridin-2-yl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(pyridin-2-yl)cyclobutyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[1-(2-chlorophenyl)cyclobutyl]-5',6'-dihydrospiro[pyrrolidine-3,4'-pyrrolo[1,2-b]pyrazole]-1-carboxamide
2-(6-amino-5-cyanopyridin-3-yl)-N-[(1R)-1-phenylethyl]-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-phenylethyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[1-(4-fluorophenyl)cyclobutyl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[2-(4-fluorophenyl)propan-2-yl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide
2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (enantiomer 2)
2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide (enantiomer 2)
2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide (enantiomer 1)
2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide (enantiomer 1)
2'-(6-amino-5-cyanopyridin-3-yl)-N-[1-(pyridin-3-yl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[1-(pyridin-2-yl)cyclobutyl]-5',6'-dihydrospiro[azetidine-3,4'-pyrrolo[1,2-b]pyrazole]-1-carboxamide
2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (enantiomer 1)
2'-(6-amino-5-cyanopyridin-3-yl)-N-(1-phenylcyclobutyl)-5',6'-dihydrospiro[azetidine-3,4'-pyrrolo[1,2-b]pyrazole]-1-carboxamide
2'-(6-amino-5-cyanopyridin-3-yl)-N-[(3S)-2,3-dihydro-1-benzofuran-3-yl]-5',6'-dihydrospiro[pyrrolidine-3,4'-pyrrolo[1,2-b]pyrazole]-1-carboxamide
2-(6-amino-5-cyanopyridin-3-yl)-N-[(3S)-2,3-dihydro-1-benzofuran-3-yl]-6,7-dihydrospiro[pyrazolo[5,1-c][1,4]oxazine-4,3'-pyrrolidine]-1'-carboxamide
2-(6-amino-5-cyanopyridin-3-yl)-N-[(1R)-1-(2-fluorophenyl)ethyl]-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-(2-fluorophenyl)ethyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[2-(4-fluorophenyl)propan-2-yl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[2-(1-methyl-1H-1,2,3-triazol-4-yl)propan-2-yl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[(1R)-1-(5-fluoropyridin-3-yl)ethyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-(1-phenylcyclobutyl)-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[1-(4-fluorophenyl)cyclobutyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-(1-phenylcyclobutyl)-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-(1,3,4-trimethyl-1H-pyrazol-5-yl)ethyl]-5',6'-dihydrospiro[azetidine-3,4'-pyrrolo[1,2-b]pyrazole]-1-carboxamide
(*rac*)-2-(6-amino-5-cyanopyridin-3-yl)-N-[2-(pyridin-4-yl)propan-2-yl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[1-(pyridin-3-yl)cyclobutyl]-5',6'-dihydrospiro[azetidine-3,4'-pyrrolo[1,2-b]pyrazole]-1-carboxamide
(rac)-2'-[6-amino-5-(trifluoromethoxy)pyridin-3-yl]-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-(2-methylpyrimidin-5-yl)ethyl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-(pyridin-2-yl)ethyl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide (mixture of stereoisomer)
2'-[6-amino-5-(trifluoromethoxy)pyridin-3-yl]-N-[(1R)-1-(pyridin-3-yl)ethyl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-(1-phenylcyclobutyl)-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[2-(2-fluorophenyl)propan-2-yl]-5',6'-dihydrospiro[azetidine-3,4'-pyrrolo[1,2-b]pyrazole]-1-carboxamide
(*rac*)-2-[6-amino-5-(difluoromethoxy)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide
2-[6-amino-5-(difluoromethoxy)pyridin-3-yl]-N-[(1S)-1-(3-chloropyridin-4-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[1-(1,3,5-trimethyl-1H-pyrazol-4-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[1-(1-ethyl-3,5-dimethyl-1H-pyrazol-4-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-(2-cyanophenyl)ethyl]-6,7-dihydrospiro[pyrazolo[5,1-c][1,4]oxazine-4,3'-pyrrolidine]-1'-carboxamide
(*rac*)-2'-[6-amino-5-(difluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2-[6-amino-5-(trifluoromethoxy)pyridin-3-yl]-N-[(1R)-1-(4-fluorophenyl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-phenylpropyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-phenylethyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide
2-[6-amino-5-(difluoromethoxy)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[2-(4-fluorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(*rac*)-2'-[6-amino-5-(difluoromethoxy)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[1-(2-chlorophenyl)cyclobutyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(4-cyanophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(1,3,4-trimethyl-1H-pyrazol-5-yl)ethyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(5-fluoropyridin-3-yl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(4-fluorophenyl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(2-cyanophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(pyridin-4-yl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(4-fluorophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(2-chlorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(4-fluorophenyl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluoromethoxy)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[2-(4-fluorophenyl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(2-chlorophenyl)propan-2-yl]-5',6'-dihydrospiro[azetidine-3,4'-pyrrolo[1,2-b]pyrazole]-1-carboxamide
2-[6-amino-5-(trifluoromethoxy)pyridin-3-yl]-N-[(1R)-1-(3-fluorophenyl)ethyl]-6,7-dihydrospiro[pyrazolo[5,1-c][1,4]oxazine-4,3'-pyrrolidine]-1'-carboxamide
2'-[6-amino-5-(trifluoromethoxy)pyridin-3-yl]-N-(1-phenylcyclobutyl)-5',6'-dihydrospiro[azetidine-3,4'-pyrrolo[1,2-b]pyrazole]-1-carboxamide
2'-[6-amino-5-(trifluoromethoxy)pyridin-3-yl]-N-benzyl-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(4-cyanopyridin-2-yl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(5-fluoropyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[2-(3-fluorophenyl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1S)-1-(3-chloropyridin-4-yl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(3-chloropyridin-4-yl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(2-chloro-4-cyanophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(2-fluorophenyl)cyclobutyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide
2-[6-amino-5-(trifluoromethoxy)pyridin-3-yl]-N-[(1R)-1-(4-cyanopyridin-2-yl)ethyl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[1-(2-chlorophenyl)cyclobutyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(pyridin-2-yl)cyclobutyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-(2-cyanopyridin-4-yl)ethyl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[2-(2-chlorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(2-chlorophenyl)cyclobutyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluoromethoxy)pyridin-3-yl]-N-[1-(pyridin-4-yl)cyclobutyl]-5',6'-dihydro-1H-spiro[pyrrolidine-3,4'-pyrrolo[1,2-b]pyrazole]-1-carboxamide acetate (1:1)
(*rac*)-2-[6-amino-5-(trifluoromethoxy)pyridin-3-yl]-N-[(1-methyl-2-oxabicyclo[2.1.1]hexan-4-yl)methyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[2-(pyridin-2-yl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide
2-[6-amino-5-(trifluoromethoxy)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1S)-1-(3-chloropyridin-4-yl)ethyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluoromethoxy)pyridin-3-yl]-N-[(1-methyl-2-oxabicyclo[2.1.1]hexan-4-yl)methyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-phenylpropyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1S)-2-methoxy-1-phenylethyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(3-fluorophenyl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1S)-1-(pyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[1-(propan-2-yl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(pyridin-4-yl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-(bicyclo[2.2.1]heptan-1-yl)-5',6'-dihydrospiro[azetidine-3,4'-pyrrolo[1,2-b]pyrazole]-1-carboxamide
2'-[6-amino-5-(difluoromethoxy)pyridin-3-yl]-N-(1-benzylcyclobutyl)-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(4-chlorophenyl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-(1-methylcyclobutyl)-5',6'-dihydrospiro[azetidine-3,4'-pyrrolo[1,2-b]pyrazole]-1-carboxamide
(*rac*)-2-[6-amino-5-(trifluoromethoxy)pyridin-3-yl]-N-[1-(1,3,4-trimethyl-1H-pyrazol-5-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-2,3-dihydro-1H-inden-1-yl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[1-(pyridin-2-yl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(3-fluorophenyl)cyclobutyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
2-[6-amino-5-(difluoromethoxy)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(5-fluoropyridin-3-yl)ethyl]-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-[(1R)-1-phenylethyl]-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-[(1R)-1-phenylethyl]-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-{6-amino-5-[(trifluoromethyl)sulfanyl]pyridin-3-yl}-N-[(1R)-1-(3-fluorophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(3R)-2,3-dihydro-1-benzofuran-3-yl]-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-methyl-1H-imidazol-5-yl)ethyl]-5',6'-dihydrospiro[pyrrolidine-3,4'-pyrrolo[1,2-b]pyrazole]-1-carboxamide (diastereomeric mixture)
2'-{6-amino-5-[(1R)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[cyclopropyl(pyridin-2-yl)methyl]-5',6'-dihydrospiro[pyrrolidine-3,4'-pyrrolo[1,2-b]pyrazole]-1-carboxamide (diastereomeric mixture)
2'-{6-amino-5-[(1S)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide
2'-{6-amino-5-[(1S)-1-(pyridin-3-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[2-(2-cyanophenyl)propan-2-yl]-5',6'-dihydrospiro[pyrrolidine-3,4'-pyrrolo[1,2-b]pyrazole]-1-carboxamide
[2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazol]-1-yl](1H-imidazol-1-yl)methanone
(*rac*)-2'-[6-amino-5-(trifluoromethyl)pyridin-3-yl]-N-(1-methylcyclobutyl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(3-methyloxetan-3-yl)-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(3-fluoropyridin-2-yl)propan-2-yl]-6,7-dihydrospiro[pyrazolo[5,1-c][1,4]oxazine-4,3'-pyrrolidine]-1'-carboxamide
2'-{6-amino-5-[(1S)-1-phenylethoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide
2'-{6-amino-5-[(1S)-1-phenylethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-phenylethoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide
2'-{6-amino-5-[(1R)-1-phenylethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
2'-{6-amino-5-[(1R)-1-(pyridin-2-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-(pyridin-4-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-(6-amino-5-cyanopyridin-3-yl)-N-[(1R)-1-(pyridin-4-yl)ethyl]-5',6'-dihydrospiro[pyrrolidine-3,4'-pyrrolo[1,2-b]pyrazole]-1-carboxamide (isomer 1)
2'-(6-amino-5-cyanopyridin-3-yl)-N-[(1R)-1-(pyridin-4-yl)ethyl]-5',6'-dihydrospiro[pyrrolidine-3,4'-pyrrolo[1,2-b]pyrazole]-1-carboxamide (isomer 2)
2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (enantiomer 1)
(3R)-2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
2'-(6-amino-5-cyanopyridin-3-yl)-N-[(1S)-1-(pyridin-4-yl)ethyl]-5',6'-dihydrospiro[pyrrolidine-3,4'-pyrrolo[1,2-b]pyrazole]-1-carboxamide (isomer 1)
2'-(6-amino-5-cyanopyridin-3-yl)-N-[(1S)-1-(pyridin-4-yl)ethyl]-5',6'-dihydrospiro[pyrrolidine-3,4'-pyrrolo[1,2-b]pyrazole]-1-carboxamide (isomer 2)
2'-[6-amino-5-(trifluoromethyl)pyridin-3-yl]-N-[(1S)-1-(pyridin-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(pyridin-4-yl)ethyl]-5',6'-dihydrospiro[azetidine-3,4'-pyrrolo[1,2-b]pyrazole]-1-carboxamide
(*rac*)-2'-[6-amino-5-(trifluoromethyl)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-(1-phenylcyclobutyl)-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide
2-[6-amino-5-(trifluoromethyl)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide (mixture of stereoisomer)
2'-[6-amino-5-(trifluoromethyl)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[1-(3-fluorophenyl)cyclobutyl]-6,7-dihydrospiro[pyrazolo[5,1-c][1,4]oxazine-4,3'-pyrrolidine]-1'-carboxamide
(3'R)-2-[6-amino-5-(trifluoromethoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(3'R)-2-[6-amino-5-(trifluoromethyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide
(3R)-2'-[6-amino-5-(trifluoromethyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(3'R)-2-[6-amino-5-(trifluoromethyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(3R)-2'-[6-amino-5-(trifluoromethyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
(3R)-2'-[6-amino-5-(difluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide
(3'R)-2-[6-amino-5-(difluoromethoxy)pyridin-3-yl]-N-[(1R)-1-phenylethyl]-6,7-dihydrospiro[pyrazolo[5,1-c][1,4]oxazine-4,3'-pyrrolidine]-1'-carboxamide
(3R)-2'-[6-amino-5-(trifluoromethyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide (stereoisomer 1)
2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide (stereoisomer 2)
(*rac*)-2'-[6-amino-5-(trifluoromethyl)pyridin-3-yl]-N-[2-(3,5-difluorophenyl)propan-2-yl]-5',6'-dihydrospiro[pyrrolidine-3,4'-pyrrolo[1,2-b]pyrazole]-1-carboxamide
(*rac*)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-(5-fluoropyridin-2-yl)-2-methylpropan-1-one
(*rac*)-2'-[6-amino-5-(trifluoromethyl)pyridin-3-yl]-N-[2-(6-methylpyridin-3-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(1-phenylcyclohexyl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(5-fluoropyridin-2-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-2,2,2-trifluoro-1-methoxy-1-phenylethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyrimidin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyrimidin-5-yl)propan-2-yl]-5',6'-dihydrospiro[pyrrolidine-3,4'-pyrrolo[1,2-b]pyrazole]-1-carboxamide
(*rac*)-{2'-[6-amino-5-(trifluoromethyl)pyridin-3-yl]-5',6'-dihydrospiro[pyrrolidine-3,4'-pyrrolo[1,2-b]pyrazol]-1-yl}(3-phenyloxetan-3-yl)methanone
(*rac*)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-(3,5-difluorophenyl)-2-methylpropan-1-one
(*rac*)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-methyl-2-(6-methylpyridin-3-yl)propan-1-one
(*rac*)-2'-[6-amino-5-(trifluoromethyl)pyridin-3-yl]-N-(3-phenyloxetan-3-yl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-cyclobutyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-cyclobutyl-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-ethyl-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-(propan-2-yl)-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-cyclobutyl-5',6'-dihydrospiro[azetidine-3,4'-pyrrolo[1,2-b]pyrazole]-1-carboxamide
2'-[6-amino-5-(trifluoromethyl)pyridin-3-yl]-N-ethyl-5',6'-dihydrospiro[azetidine-3,4'-pyrrolo[1,2-b]pyrazole]-1-carboxamide
2'-[6-amino-5-(trifluoromethyl)pyridin-3-yl]-N-(propan-2-yl)-5',6'-dihydrospiro[azetidine-3,4'-pyrrolo[1,2-b]pyrazole]-1-carboxamide
(*rac*)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-{6-amino-5-[(2-chlorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(2-chlorophenyl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(2-chlorophenyl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-benzyl-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-benzyl-5',6'-dihydrospiro[pyrrolidine-3,4'-pyrrolo[1,2-b]pyrazole]-1-carboxamide
(*rac*)-2'-{6-amino-5-[(pyridin-2-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(pyridin-2-yl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(pyridin-2-yl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-chloro-5-fluorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(2-chloro-5-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide
2'-(6-amino-5-{[1-(5-chloro-3-fluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomeric mixture)
(rac)-2'-[6-amino-5-(benzyloxy)pyridin-3-yl]-N-cyclobutyl-5',6'-dihydrospiro[pyrrolidine-3,4'-pyrrolo[1,2-b]pyrazole]-1-carboxamide
2'-{6-amino-5-[(pyridazin-3-yl)methoxy]pyridin-3-yl}-N-ethyl-5',6'-dihydrospiro[pyrrolidine-3,4'-pyrrolo[1,2-b]pyrazole]-1-carboxamide (stereoisomer 2)
2'-{6-amino-5-[(pyridazin-3-yl)methoxy]pyridin-3-yl}-N-ethyl-5',6'-dihydrospiro[pyrrolidine-3,4'-pyrrolo[1,2-b]pyrazole]-1-carboxamide (stereoisomer 1)
(35)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 1)
(35)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 2)
(*rac*)-2'-(2-amino-1'-methyl-1',2',3',6'-tetrahydro[3,4'-bipyridin]-5-yl)-N-ethyl-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-ethyl-5',6'-dihydrospiro[azetidine-3,4'-pyrrolo[1,2-b]pyrazole]-1-carboxamide
2'-{6-amino-5-[(1S)-1-(pyridin-2-yl)ethoxy]pyridin-3-yl}-N-(propan-2-yl)-5',6'-dihydrospiro[azetidine-3,4'-pyrrolo[1,2-b]pyrazole]-1-carboxamide
2'-{6-amino-5-[(1S)-1-phenylethoxy]pyridin-3-yl}-N-ethyl-5',6'-dihydrospiro[azetidine-3,4'-pyrrolo[1,2-b]pyrazole]-1-carboxamide
2'-{6-amino-5-[(1R)-1-phenylethoxy]pyridin-3-yl}-N-ethyl-5',6'-dihydrospiro[azetidine-3,4'-pyrrolo[1,2-b]pyrazole]-1-carboxamide
(*rac*)-2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-ethyl-6,7-dihydro-5H-spiro[pyrazolo[1,5-a]pyridine-4,3'-pyrrolidine]-1'-carboxamide
(*rac*)-2'-(6-amino-5-cyanopyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(trifluoroacetyl)pyridin-3-yl]-N-ethyl-5',6'-dihydrospiro[pyrrolidine-3,4'-pyrrolo[1,2-b]pyrazole]-1-carboxamide
2'-(6-amino-5-{[1-phenylethyl]sulfanyl}pyridin-3-yl)-N-ethyl-5',6'-dihydrospiro[pyrrolidine-3,4'-pyrrolo[1,2-b]pyrazole]-1-carboxamide (diastereomeric mixture)
(*rac*)-2'-{6-amino-5-[(cyclohexylmethyl)sulfanyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(benzylsulfanyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(2-hydroxypropan-2-yl)pyridin-3-yl]-N-ethyl-5',6'-dihydrospiro[pyrrolidine-3,4'-pyrrolo[1,2-b]pyrazole]-1-carboxamide
2'-(6-amino-5-{[1-cyclohexylethyl]sulfanyl}pyridin-3-yl)-N-ethyl-5',6'-dihydrospiro[pyrrolidine-3,4'-pyrrolo[1,2-b]pyrazole]-1-carboxamide (diastereomeric mixture)
2'-{6-amino-5-[(1R)-1-(pyridin-3-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1S)-1-(pyridin-3-yl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(cyclopentylmethyl)sulfanyl]pyridin-3-yl}-N-ethyl-5',6'-dihydrospiro[pyrrolidine-3,4'-pyrrolo[1,2-b]pyrazole]-1-carboxamide
(*rac*)-2'-(6-amino-5-nitropyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(benzyloxy)pyridin-3-yl]-N-ethyl-5',6'-dihydrospiro[pyrrolidine-3,4'-pyrrolo[1,2-b]pyrazole]-1-carboxamide
(*rac*)-2'-(6-amino-5-methylpyridin-3-yl)-N-ethyl-5',6'-dihydrospiro[pyrrolidine-3,4'-pyrrolo[1,2-b]pyrazole]-1-carboxamide
(*rac*)-2-(6-amino-5-nitropyridin-3-yl)-N-ethyl-6,7-dihydro-5H-spiro[pyrazolo[1,5-a]pyridine-4,3'-pyrrolidine]-1'-carboxamide
methyl 2-amino-5-[(*rac*)-1-(ethylcarbamoyl)-5',6'-dihydrospiro[pyrrolidine-3,4'-pyrrolo[1,2-b]pyrazol]-2'-yl]pyridine-3-carboxylate
(*rac*)-2'-(6-amino-5-phenylpyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(methylsulfanyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(4-methylphenyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(2-methylphenyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(3-methylphenyl)pyridin-3-yl]-N-ethyl-5',6'-dihydrospiro[pyrrolidine-3,4'-pyrrolo[1,2-b]pyrazole]-1-carboxamide
(*rac*)-2'-(6-amino-5-methoxypyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(3-fluorophenyl)pyridin-3-yl]-N-ethyl-5',6'-dihydrospiro[pyrrolidine-3,4'-pyrrolo[1,2-b]pyrazole]-1-carboxamide
(*rac*)-2'-(2-amino[3,3'-bipyridin]-5-yl)-N-ethyl-5',6'-dihydrospiro[pyrrolidine-3,4'-pyrrolo[1,2-b]pyrazole]-1-carboxamide
(*rac*)-2'-[6-amino-5-(2-fluorophenyl)pyridin-3-yl]-N-ethyl-5',6'-dihydrospiro[pyrrolidine-3,4'-pyrrolo[1,2-b]pyrazole]-1-carboxamide
2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (diastereomeric mixture)
(*rac*)-2-[6-amino-5-(2-fluorophenyl)pyridin-3-yl]-N-ethyl-6,7-dihydro-5H-spiro[pyrazolo[1,5-a]pyridine-4,3'-pyrrolidine]-1'-carboxamide
(*rac*)-2'-[6-amino-5-(2-methoxyphenyl)pyridin-3-yl]-N-ethyl-5',6'-dihydrospiro[pyrrolidine-3,4'-pyrrolo[1,2-b]pyrazole]-1-carboxamide
(*rac*)-2'-(2-amino[3,4'-bipyridin]-5-yl)-N-ethyl-5',6'-dihydrospiro[pyrrolidine-3,4'-pyrrolo[1,2-b]pyrazole]-1-carboxamide
(*rac*)-2'-{6-amino-5-[3-(methanesulfonyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(phenylsulfanyl)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[4-(methanesulfonyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[5-(4-acetylphenyl)-6-aminopyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[4-(methanesulfinyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(3-methoxyphenyl)pyridin-3-yl]-N-ethyl-5',6'-dihydrospiro[pyrrolidine-3,4'-pyrrolo[1,2-b]pyrazole]-1-carboxamide
2'-{6-amino-5-[(1R)-1-(2,6-dichloro-3-fluorophenyl)ethoxy]pyridin-3-yl}-N-ethyl-5',6'-dihydrospiro[azetidine-3,4'-pyrrolo[1,2-b]pyrazole]-1-carboxamide
(*rac*)-2'-{6-amino-5-[4-(cyclopropanesulfonyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(4-sulfamoylphenyl)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[4-(methylsulfamoyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[4-(dimethylsulfamoyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(cyclohexylmethoxy)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[(pyridin-3-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(4-fluorophenyl)methoxy]pyridin-3-yl}-N-ethyl-5',6'-dihydrospiro[pyrrolidine-3,4'-pyrrolo[1,2-b]pyrazole]-1-carboxamide
2'-(6-amino-5-{[1-phenylethyl]oxy}pyridin-3-yl)-N-ethyl-5',6'-dihydrospiro[pyrrolidine-3,4'-pyrrolo[1,2-b]pyrazole]-1-carboxamide (diastereomeric mixture)
2-{6-amino-5-[(1R)-1-(2,6-dichloro-3-fluorophenyl)ethoxy]pyridin-3-yl}-N-ethyl-6,7-dihydrospiro[pyrazolo[5,1-c][1,4]oxazine-4,3'-pyrrolidine]-1'-carboxamide
(*rac*)-2'-[6-amino-5-(propan-2-yl)pyridin-3-yl]-N-ethyl-5',6'-dihydrospiro[pyrrolidine-3,4'-pyrrolo[1,2-b]pyrazole]-1-carboxamide
(*rac*)-2-[6-amino-5-(methylcarbamoyl)pyridin-3-yl]-N-ethyl-6,7-dihydrospiro[pyrazolo[5,1-c][1,4]oxazine-4,3'-pyrrolidine]-1'-carboxamide
(*rac*)-2'-[6-amino-5-(methylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-(6-amino-5-cyclopropylpyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(methylcarbamoyl)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2'-[6-amino-5-(dimethylcarbamoyl)pyridin-3-yl]-N-(1-phenylcyclobutyl)-5',6'-dihydrospiro[pyrrolidine-3,4'-pyrrolo[1,2-b]pyrazole]-1-carboxamide
2'-{6-amino-5-[(1R)-1-(pyridin-4-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1S)-1-(pyridin-4-yl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(1-methyl-1H-pyrazol-3-yl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(1,2-thiazol-5-yl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(1,5-dimethyl-1H-pyrazol-4-yl)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[(trifluoromethyl)sulfanyl]pyridin-3-yl}-N-ethyl-5',6'-dihydrospiro[pyrrolidine-3,4'-pyrrolo[1,2-b]pyrazole]-1-carboxamide
5-[1-methyl-5',6'-dihydrospiro[pyrrolidine-3,4'-pyrrolo[1,2-b]pyrazol]-2'-yl]-3-(trifluoromethyl)pyridin-2-amine (stereoisomer 1)
5-[1-methyl-5',6'-dihydrospiro[pyrrolidine-3,4'-pyrrolo[1,2-b]pyrazol]-2'-yl]-3-(trifluoromethyl)pyridin-2-amine (stereoisomer 2)
(3R)-2'-{6-amino-5-[(1R)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(2-chlorophenyl)ethoxy]pyridin-3-yl}-N-ethyl-5',6'-dihydrospiro[azetidine-3,4'-pyrrolo[1,2-b]pyrazole]-1-carboxamide
2'-{6-amino-5-[(1S)-1-(2-chlorophenyl)ethoxy]pyridin-3-yl}-N-ethyl-5',6'-dihydrospiro[azetidine-3,4'-pyrrolo[1,2-b]pyrazole]-1-carboxamide
(3'R)-2-{6-amino-5-[(1R)-1-(2,6-dichloro-3-fluorophenyl)ethoxy]pyridin-3-yl}-N-ethyl-6,7-dihydrospiro[pyrazolo[5,1-c][1,4]oxazine-4,3'-pyrrolidine]-1'-carboxamide
(3R)-2'-{6-amino-5-[(1R)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-ethyl-5',6'-dihydrospiro[azetidine-3,4'-pyrrolo[1,2-b]pyrazole]-1-carboxamide
(3R)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 1)
((3R)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 2)
(rac)-2'-[6-amino-5-(difluoromethyl)pyridin-3-yl]-N-ethyl-5',6'-dihydrospiro[pyrrolidine-3,4'-pyrrolo[1,2-b]pyrazole]-1-carboxamide
2'-{6-amino-5-[(1S)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(3-methylphenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(1-methyl-1H-pyrazol-5-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(1-methyl-1H-pyrazol-4-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(1,5-dimethyl-1H-pyrazol-3-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(4-methylphenyl)methoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(3-methylphenyl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(propan-2-yl)oxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1S)-1-(1-methyl-1H-pyrazol-3-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(1-methyl-1H-pyrazol-3-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-(6-amino-5-{[1-(3,5-difluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (stereoisomer 1)
(3R)-2'-(6-amino-5-{[1-(3,5-difluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (stereoisomer 2)
(3R)-2'-{6-amino-5-[(1R)-1-(2-fluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(15)-1-(2-fluorophenyl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-(5-acetyl-6-aminopyridin-3-yl)-N-(1-phenylcyclobutyl)-5',6'-dihydrospiro[pyrrolidine-3,4'-pyrrolo[1,2-b]pyrazole]-1-carboxamide
(3R)-2'-[6-amino-5-(trifluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
(3'R)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(3R)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide
(3'R)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2'-[6-amino-5-(phenylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(cyclohexylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(diethylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-(5-acetyl-6-aminopyridin-3-yl)-N-ethyl-5',6'-dihydrospiro[pyrrolidine-3,4'-pyrrolo[1,2-b]pyrazole]-1-carboxamide
5-{1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
5-{1-[1-(1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-imidazol-5-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1-methyl-1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-pyrazol-4-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(4-methyl-1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(3-methyl-1H-pyrazol-5-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(4-methyl-1H-pyrazol-5-yl)ethyl]-5',6'-dihydrospiro[pyrrolidine-3,4'-pyrrolo[1,2-b]pyrazol]-2'-yl}-3-(trifluoromethyl)pyridin-2-amine
5-[(3S)-1-(pyridin-3-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(pyridin-3-ylmethyl)-5',6'-dihydrospiro[pyrrolidine-3,4'-pyrrolo[1,2-b]pyrazol]-2'-yl]-3-(trifluoromethyl)pyridin-2-amine
5-{(*rac*)-1-[(1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
5-[(35)-1-(1H-imidazol-2-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(1H-imidazol-2-ylmethyl)-5',6'-dihydrospiro[pyrrolidine-3,4'-pyrrolo[1,2-b]pyrazol]-2'-yl]-3-(trifluoromethyl)pyridin-2-amine
5-{(*rac*)-1-[(4-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1H-pyrazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1-methyl-1H-pyrazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1H-imidazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1-methyl-1H-imidazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(4-chloro-1H-pyrazol-5-yl)methyl]-5',6'-dihydrospiro[pyrrolidine-3,4'-pyrrolo[1,2-b]pyrazol]-2'-yl}-3-(trifluoromethyl)pyridin-2-amine
3-[(2-fluorophenyl)methoxy]-5-{(*rac*)-1-[(1H-imidazol-2-yl)methyl]-5',6'-dihydrospiro[pyrrolidine-3,4'-pyrrolo[1,2-b]pyrazol]-2'-yl}pyridin-2-amine
3-[(3-fluorophenyl)methoxy]-5-{(*rac*)-1-[(1H-imidazol-2-yl)methyl]-5',6'-dihydrospiro[pyrrolidine-3,4'-pyrrolo[1,2-b]pyrazol]-2'-yl}pyridin-2-amine
5-{(*rac*)-1-[(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
5-{1-[(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine
5-{(*rac*)-1'-[(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine
2-amino-5-{1-[(1H-imidazol-2-yl)methyl]-5',6'-dihydrospiro[azetidine-3,4'-pyrrolo[1,2-b]pyrazol]-2'-yl}pyridine-3-carbonitrile
5-{(*rac*)-1-[(1-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
2-amino-5-{(*rac*)-1-[(1H-imidazol-2-yl)methyl]-5',6'-dihydrospiro[pyrrolidine-3,4'-pyrrolo[1,2-b]pyrazol]-2'-yl}pyridine-3-carbonitrile
5-{1-[(1-methyl-1H-imidazol-2-yl)methyl]-5',6'-dihydrospiro[azetidine-3,4'-pyrrolo[1,2-b]pyrazol]-2'-yl}-3-(trifluoromethyl)pyridin-2-amine
5-{(*rac*)-1-[(5-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(2,4-dimethyl-1H-imidazol-5-yl)methyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (stereoisomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (stereoisomer 2)
5-{(3R)-1-[(1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (stereoisomer 1)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (stereoisomer 2)
5-{(3R)-1-[1-(1-methyl-1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
3-(trifluoromethyl)-5-[(3R)-1-{[4-(trifluoromethyl)-1H-imidazol-2-yl]methyl}-5',6'-dihydrospiro[pyrrolidine-3,4'-pyrrolo[1,2-b]pyrazol]-2'-yl]pyridin-2-amine
2-amino-5-[(rac)-1-(ethylcarbamoyl)-5',6'-dihydrospiro[pyrrolidine-3,4'-pyrrolo[1,2-b]pyrazol]-2'-yl]pyridine-3-carboxylic acid
5-[(35)-1-(pyridin-2-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(pyridin-2-ylmethyl)-5',6'-dihydrospiro[pyrrolidine-3,4'-pyrrolo[1,2-b]pyrazol]-2'-yl]-3-(trifluoromethyl)pyridin-2-amine
5-[(3S)-1-(pyridin-4-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(pyridin-4-ylmethyl)-5',6'-dihydrospiro[pyrrolidine-3,4'-pyrrolo[1,2-b]pyrazol]-2'-yl]-3-(trifluoromethyl)pyridin-2-amine
5-{(3R)-1-[3-(dimethylamino)propyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[2-(dimethylamino)ethyl]-5',6'-dihydrospiro[pyrrolidine-3,4'-pyrrolo[1,2-b]pyrazol]-2'-yl}-3-(trifluoromethyl)pyridin-2-amine
2-amino-1-{2'-[6-amino-5-(trifluoromethyl)pyridin-3-yl]-5',6'-dihydrospiro[pyrrolidine-3,4'-pyrrolo[1,2-b]pyrazol]-1-yl}ethan-1-one (stereoisomer 2)
2-amino-1-{2'-[6-amino-5-(trifluoromethyl)pyridin-3-yl]-5',6'-dihydrospiro[pyrrolidine-3,4'-pyrrolo[1,2-b]pyrazol]-1-yl}ethan-1-one (stereoisomer 1)
2-amino-1-{2-[6-amino-5-(trifluoromethyl)pyridin-3-yl]-6,7-dihydrospiro[pyrazolo[5,1-c][1,4]oxazine-4,3'-pyrrolidin]-1'-yl}ethan-1-one (stereoisomer 2)
2-amino-1-{2-[6-amino-5-(trifluoromethyl)pyridin-3-yl]-6,7-dihydrospiro[pyrazolo[5,1-c][1,4]oxazine-4,3'-pyrrolidin]-1'-yl}ethan-1-one (stereoisomer 1)
2-amino-1-{2'-[6-amino-5-(trifluoromethyl)pyridin-3-yl]-5',6'-dihydrospiro[azetidine-3,4'-pyrrolo[1,2-b]pyrazol]-1-yl}ethan-1-one
(25)-2-amino-1-{2'-[6-amino-5-(trifluoromethyl)pyridin-3-yl]-5',6'-dihydrospiro[azetidine-3,4'-pyrrolo[1,2-b]pyrazol]-1-yl}propan-1-one
(2R)-2-amino-1-{2'-[6-amino-5-(trifluoromethyl)pyridin-3-yl]-5',6'-dihydrospiro[azetidine-3,4'-pyrrolo[1,2-b]pyrazol]-1-yl}propan-1-one
(*rac*)-2'-[6-amino-5-(1-methylpiperidin-4-yl)pyridin-3-yl]-N-ethyl-5',6'-dihydrospiro[pyrrolidine-3,4'-pyrrolo[1,2-b]pyrazole]-1-carboxamide
5-{(*rac*)-1-[(5-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (isomer 1)
2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (isomer 2)
5-{(3'R)-1'-[(1S)-1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine
5-{(3'R)-1'-[(1R)-1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine
5-{(3'R)-1'-[(S)-cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine
5-{(3'R)-1'-[(R)-cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine
5-{(3R)-1-[(S)-cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(R)-cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(1S)-1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(1R)-1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(R)-cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine
5-{(3R)-1-[(S)-cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine
3-(difluoromethoxy)-5-{(3R)-1-[(1R)-1-(1H-imidazol-2-yl)propyl]-5',6'-dihydrospiro[pyrrolidine-3,4'-pyrrolo[1,2-b]pyrazol]-2'-yl}pyridin-2-amine
3-(difluoromethoxy)-5-{(3R)-1-[(1S)-1-(1H-imidazol-2-yl)propyl]-5',6'-dihydrospiro[pyrrolidine-3,4'-pyrrolo[1,2-b]pyrazol]-2'-yl}pyridin-2-amine
2-amino-5-{(3R)-1-[(R)-cyclopropyl(1H-imidazol-2-yl)methyl]-5',6'-dihydrospiro[pyrrolidine-3,4'-pyrrolo[1,2-b]pyrazol]-2'-yl}nicotinonitrile
2-amino-5-{(3R)-1-[(S)-cyclopropyl(1H-imidazol-2-yl)methyl]-5',6'-dihydrospiro[pyrrolidine-3,4'-pyrrolo[1,2-b]pyrazol]-2'-yl}nicotinonitrile
2-amino-5-{(3R)-1-[(1R)-1-(1H-imidazol-2-yl)propyl]-5',6'-dihydrospiro[pyrrolidine-3,4'-pyrrolo[1,2-b]pyrazol]-2'-yl}nicotinonitrile
2-amino-5-{(3R)-1-[(1S)-1-(1H-imidazol-2-yl)propyl]-5',6'-dihydrospiro[pyrrolidine-3,4'-pyrrolo[1,2-b]pyrazol]-2'-yl}nicotinonitrile
(3R)-2'-(6-amino-5-{[1-(6-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(6-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(5-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(5-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(4-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(4-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(3-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(3-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(1,4-dimethyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(1,4-dimethyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[-1-(1,4-dimethyl-1H-pyrazol-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1)
(3R)-2'-(6-amino-5-{[1-(1,4-dimethyl-1H-pyrazol-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 2)
(3R)-2'-{6-amino-5-[(15)-1-(2,6-difluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(5-fluoropyridin-2-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(3-fluoropyridin-2-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(2,6-difluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 2)
(3'R)-2-[6-amino-5-(trifluoromethyl)pyridin-3-yl]-N-[1-(1-ethyl-1H-pyrazol-3-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[(1-methyl-1H-pyrazol-5-yl)methyl]-6,7-dihydrospiro[pyrazolo[5,1-c][1,4]oxazine-4,3'-pyrrolidine]-1'-carboxamide
(3'R)-2-[6-amino-5-(trifluoromethyl)pyridin-3-yl]-N-[(1-methyl-1H-pyrazol-4-yl)methyl]-6,7-dihydrospiro[pyrazolo[5,1-c][1,4]oxazine-4,3'-pyrrolidine]-1'-carboxamide
(3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1)
(3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 2)
(3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1)
(3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 2)
(3R)-2'-{6-amino-5-[(1R)-1-(3-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(4-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 1)
(3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 2)
(3R)-2'-{6-amino-5-[(1S)-1-(3-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(15)-1-(4-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(15)-1-(3,5-difluoropyridin-4-yl)ethoxy]pyridin-3-yl}-N-ethyl-5',6'-dihydrospiro[pyrrolidine-3,4'-pyrrolo[1,2-b]pyrazole]-1-carboxamide
(3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1)
(3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 2)
(3'R)-2-[6-amino-5-(trifluoromethyl)pyridin-3-yl]-N-[1-(1,5-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1-methyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3R)-2'-{6-amino-5-[(1R)-1-(3-fluoropyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(3-fluoropyridin-2-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-phenylethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[1-(1-methyl-1H-pyrazol-5-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(1-methyl-1H-pyrazol-5-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 2)
(3R)-2'-{6-amino-5-[1-(1-methyl-1H-pyrazol-5-yl)ethoxy]pyridin-3-yl}-N-cyclobutyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(1-methyl-1H-pyrazol-5-yl)ethoxy]pyridin-3-yl}-N-cyclobutyl-5',6'-dihydro-1H-spiro[pyrrolidine-3,4'-pyrrolo[1,2-b]pyrazole]-1-carboxamide (diastereomer 2)
(3R)-2'-{6-amino-5-[(1R)-1-(2-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 1 and enantiomer 2)
2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 1)
2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 2)
2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 1 and enantiomer 2)
2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 1)
2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 2)
(3S)-2'-[6-amino-5-(trifluoromethyl)pyridin-3-yl]-N-[1-(1-methyl-1H-pyrazol-5-yl)ethyl]-5',6'-dihydro-1H-spiro[pyrrolidine-3,4'-pyrrolo[1,2-b]pyrazole]-1-carboxamide (diastereomer 1 and diastereomer 2)
(3S)-2'-[6-amino-5-(trifluoromethyl)pyridin-3-yl]-N-[1-(1,3-dimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(2,6-difluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-(6-amino-5-{methyl[(1R)-1-phenylethyl]amino}pyridin-3-yl)-N-ethyl-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 1 and diastereomer 2)
2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 1)
2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 2)
(3R)-2'-{6-amino-5-[(1R)-1-(4-methylpyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(4-methylpyridin-2-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(6-methylpyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(6-methylpyridin-2-yl)ethoxy]pyridin-3-yl}-N-cyclobutyl-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 2)
(2'-{6-amino-5-[(1R)-1-phenylethoxy]pyridin-3-yl}-5',6'-dihydrospiro[azetidine-3,4'-pyrrolo[1,2-b]pyrazol]-1-yl)(cyclopropyl)methanone
2'-[6-amino-5-(trifluoromethyl)pyridin-3-yl]-N-[1-(1-ethyl-1H-pyrazol-3-yl)ethyl]-5',6'-dihydro-1H-spiro[azetidine-3,4'-pyrrolo[1,2-b]pyrazole]-1-carboxamide (enantiomer 1)
2'-[6-amino-5-(trifluoromethyl)pyridin-3-yl]-N-[1-(1-ethyl-1H-pyrazol-3-yl)ethyl]-5',6'-dihydro-1H-spiro[azetidine-3,4'-pyrrolo[1,2-b]pyrazole]-1-carboxamide (enantiomer 2)
(3S)-2'-[6-amino-5-(trifluoromethyl)pyridin-3-yl]-N-[1-(1-ethyl-1H-pyrazol-3-yl)ethyl]-5',6'-dihydro-1H-spiro[pyrrolidine-3,4'-pyrrolo[1,2-b]pyrazole]-1-carboxamide (diastereomer 1)
(3S)-2'-[6-amino-5-(trifluoromethyl)pyridin-3-yl]-N-[1-(1-ethyl-1H-pyrazol-3-yl)ethyl]-5',6'-dihydro-1H-spiro[pyrrolidine-3,4'-pyrrolo[1,2-b]pyrazole]-1-carboxamide (diastereomer 2)
5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-1,2,4-Triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[2,2-difluoro-1-(1H-imidazol-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[2,2-difluoro-1-(1H-imidazol-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 2)
2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 1 and diastereomer 2)
2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 1)
2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 2)
5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 1 and enantiomer 2)
5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 1)
5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 2)
5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[1-(1H-imidazol-2-yl)-2,2-dimethylpropyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 1 and enantiomer 2)
5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 1)
5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 2)
5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(35)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3S)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(35)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 1 and diastereomer 2)
2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 1)
2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 1 and diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 2)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 1 and diastereomer 2)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 1)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 2)
3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 1 and diastereomer 2)
3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 1)
3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 2)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 1)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 2)
5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(35)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(35)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(35)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer2)
{(3R)-2'-[6-amino-5-(trifluoromethyl)pyridin-3-yl]-5',6'-dihydrospiro[pyrrolidine-3,4'-pyrrolo[1,2-b]pyrazol]-1-yl}(1H-imidazol-2-yl)methanone
{(3R)-2'-[6-amino-5-(trifluoromethyl)pyridin-3-yl]-5',6'-dihydrospiro[pyrrolidine-3,4'-pyrrolo[1,2-b]pyrazol]-1-yl}(1-methyl-1H-imidazol-2-yl)methanone
3-[(1R)-1-(2,6-dichloro-3-fluorophenyl)ethoxy]-5-{(3R)-1-[(1H-imidazol-2-yl)methyl]-5',6'-dihydrospiro[pyrrolidine-3,4'-pyrrolo[1,2-b]pyrazol]-2'-yl}pyridin-2-amine
5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-[6-amino-5-(trifluoromethyl)pyridin-3-yl]-N-[1-(3-chloropyridin-4-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (enantiomer 2)
(3R)-2'-[6-amino-5-(trifluoromethyl)pyridin-3-yl]-N-[1-(3-methylpyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 2)
(3R)-2'-[6-amino-5-(trifluoromethyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 2)
(3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 2)
(3R)-2'-[6-amino-5-(trifluoromethyl)pyridin-3-yl]-N-[(1R)-1-(3-chloropyridin-4-yl)ethyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(2-methylpyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(3-methoxypyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(pyridin-4-yl)cyclopropyl]-5',6'-dihydrospiro[pyrrolidine-3,4'-pyrrolo[1,2-b]pyrazole]-1-carboxamide
(3R)-2'-{6-amino-5-[(1R)-1-(2-methoxyphenyl)ethoxy]pyridin-3-yl}-N-ethyl-5',6'-dihydrospiro[pyrrolidine-3,4'-pyrrolo[1,2-b]pyrazole]-1-carboxamide
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 1 and diastereomer 2)
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 1)
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 2)
(3S)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (enantiomer 1)
(3R)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (enantiomer 2)
(3R)-2'-{6-amino-5-[(1R)-1-(3-chlorophenyl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 2)
5-{(3R)-1-[1-(1H-pyrrol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
(3R)-2'-[6-amino-5-(trifluoromethyl)pyridin-3-yl]-N-[1-(2-methoxypyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(1-methyl-1H-pyrazol-5-yl)ethyl]-5',6'-dihydrospiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(1-methyl-1H-pyrazol-5-yl)ethyl]-5',6'-dihydrospiro[pyrrolidine-3,4'-pyrrolo[1,2-b]pyrazole]-1-carboxamide (diastereomer 2)
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzamide (diastereomer 1 and diastereomer 2)
3-(1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}propyl)benzamide (diastereomer 1)
3-(1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}propyl)benzamide (diastereomer 2)
(3R)-2'-{6-amino-5-[(1R)-1-(1,3-thiazol-2-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(3-chlorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(2-chloro-3-fluoropyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-{[3-(methylsulfanyl)pyridin-4-yl]methyl}-5',6'-dihydrospiro[pyrrolidine-3,4'-pyrrolo[1,2-b]pyrazole]-1-carboxamide
(3R)-2'-{6-amino-5-[(1R)-1-(1,3-thiazol-2-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(1,3-dimethyl-1H-pyrazol-4-yl)ethyl]-5',6'-dihydrospiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(1,3-dimethyl-1H-pyrazol-4-yl)ethyl]-5',6'-dihydrospiro[pyrrolidine-3,4'-pyrrolo[1,2-b]pyrazole]-1-carboxamide (diastereomer 2)
(3R)-2'-{6-amino-5-[(1R)-1-(3-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(pyridin-2-yl)cyclopropyl]-5',6'-dihydrospiro[pyrrolidine-3,4'-pyrrolo[1,2-b]pyrazole]-1-carboxamide
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-{6-amino-5-[(1R)-1-(1,3-thiazol-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[(3-methylpyridin-4-yl)methyl]-5',6'-dihydrospiro[pyrrolidine-3,4'-pyrrolo[1,2-b]pyrazole]-1-carboxamide
5-{(3R)-1-[1-(6-methoxypyridin-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(6-methoxypyridin-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-(6-amino-5-{1-[3-(3-hydroxy-3-methylbut-1-yn-1-yl)phenyl]ethoxy}pyridin-3-yl)-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[1-(1,3-oxazol-2-yl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(3-fluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (diastereomer 2)
5-{(3R)-1-[cyclopropyl(5-fluoropyridin-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
(3R)-2'-{6-amino-5-[(1R)-1-(pyrimidin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(4-fluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(4-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-{6-amino-5-[(1R)-1-(4-chlorophenyl)ethoxy]pyridin-3-yl}-N-ethyl-5',6'-dihydrospiro[pyrrolidine-3,4'-pyrrolo[1,2-b]pyrazole]-1-carboxamide
5-{(3R)-1-[1-(dimethylamino)propan-2-yl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
(3R)-2'-{6-amino-5-[(1R)-1-(1,2-thiazol-5-yl)ethoxy]pyridin-3-yl}-N-ethyl-5',6'-dihydrospiro[pyrrolidine-3,4'-pyrrolo[1,2-b]pyrazole]-1-carboxamide
6-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyridin-2(1H)-one
(3R)-2'-{6-amino-5-[(1R)-1-(pyridin-3-yl)ethoxy]pyridin-3-yl}-N-(propan-2-yl)-5',6'-dihydrospiro[pyrrolidine-3,4'-pyrrolo[1,2-b]pyrazole]-1-carboxamide
2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (4 isomers)
(3R)-2'-{6-amino-5-[1-(1,3-oxazol-4-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 1 and diastereomer 2)
5-{(3R)-1-[(1,3-thiazol-2-yl)methyl]-5',6'-dihydrospiro[pyrrolidine-3,4'-pyrrolo[1,2-b]pyrazol]-2'-yl}-3-(trifluoromethyl)pyridin-2-amine
6-({(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}methyl)pyridin-2(1H)-one
(3S)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1)
(3S)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 2)
(3R)-2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (diastereomer 1)
2'-{6-amino-5-[(1R)-1-(pyridin-2-yl)ethoxy]pyridin-3-yl}-N-ethyl-5',6'-dihydrospiro[azetidine-3,4'-pyrrolo[1,2-b]pyrazole]-1-carboxamide
1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-(methylamino)ethan-1-one
(2R)-1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-(methylamino)propan-1-one
(5R)-5-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyrrolidin-2-one
5-{(3R)-1-[(1,3,4-thiadiazol-2-yl)methyl]-5',6'-dihydrospiro[pyrrolidine-3,4'-pyrrolo[1,2-b]pyrazol]-2'-yl}-3-(trifluoromethyl)pyridin-2-amine
(55)-5-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyrrolidin-2-one
5-{(3'R)-1'-[1-(6-methoxypyridin-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclopropyl(6-methoxypyridin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[cyclopropyl(6-methoxypyridin-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[1-(3-fluoropyridin-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1 and diastereomer 2)
3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1)
3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 2)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastereomer 1 and diastereomer 2)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastereomer 1)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastereomer 2)
3-(difluoromethyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1 and diastereomer 2)
3-(difluoromethyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1)
3-(difluoromethyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 2)
(3R)-2'-(6-amino-5-{[1-(1-propyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(1,3-thiazol-4-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(pyrimidin-4-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(2,4-dimethylpyrimidin-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1)
(3R)-2'-(6-amino-5-{[1-(2,4-dimethylpyrimidin-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 2)
(3R)-2'-{6-amino-5-[(1R)-1-(5-methyl-1,3,4-oxadiazol-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(1-methyl-1H-1,2,3-triazol-5-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-(6-amino-5-{[1-(1-methyl-1H-1,2,3-triazol-4-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1 and diastereomer 2)
(55)-5-{[(3R)-2'-{6-amino-5-[(1R)-1-phenylethoxy]pyridin-3-yl}-5',6'-dihydrospiro[pyrrolidine-3,4'-pyrrolo[1,2-b]pyrazol]-1-yl]methyl}pyrrolidin-2-one
(5R)-5-{[(3R)-2'-{6-amino-5-[(1R)-1-phenylethoxy]pyridin-3-yl}-5',6'-dihydrospiro[pyrrolidine-3,4'-pyrrolo[1,2-b]pyrazol]-1-yl]methyl}pyrrolidin-2-one
(3R)-2'-(6-amino-5-{1-[4-(methylsulfonyl)phenyl]ethoxy}pyridin-3-yl)-N-isopropyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(2-methyl-1,3-thiazol-4-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-({1-[3-(methanesulfonyl)phenyl]ethyl}oxy)pyridin-3-yl]-N-(propan-2-yl)-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(4-methyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(4,5-dimethyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(5-methyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-{6-amino-5-[(1R)-1-(pyridin-4-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(pyridin-4-yl)ethoxy]pyridin-3-yl}-N-cyclobutyl-5',6'-dihydrospiro[pyrrolidine-3,4'-pyrrolo[1,2-b]pyrazole]-1-carboxamide
6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 1 and diastereomer 2)
6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 1)
6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 2)
6-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}(cyclopropyl)methyl)pyridin-2-ol (diastereomer 1 and diastereomer 2)
6-({(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}(cyclopropyl)methyl)pyridin-2-ol (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 1)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 2)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 3)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 4)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[cyclohexyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclohexyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyrazin-2-amine (diastereomer 1 and diastereomer 2)
5-{(3R)-1-[(1H-imidazol-2-yl)(1-methylcyclobutyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 1 and diastereomer 2)
5-{(3R)-1-[(1-methylcyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diasteroemr 1 and diastereomer 2)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 1 and diastereomer 2)
5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclopropyl(1-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3'R)-1'-[1-(4H-1,2,4-triazol-3-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diatereomer 1 and diastereomer 2)
5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(2-methoxypyridin-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
3-[{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}(cyclopropyl)methyl]pyridin-2(1H)-one (diastereomer 1 and diastereomer 2)
5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(4-methoxypyrimidin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclobutyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[(1-fluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(pyrimidin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 2)
5-{(3'R)-1'-[cyclobutyl(4H-1,2,4-triazol-3-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[2,2-difluoro-1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[cyclobutyl(4H-1,2,4-triazol-3-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 2)
(3R)-2'-{5-amino-6-[(1R)-1-phenylethoxy]pyrazin-2-yl}-N-ethyl-5',6'-dihydrospiro[pyrrolidine-3,4'-pyrrolo[1,2-b]pyrazole]-1-carboxamide
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1 and diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 2)
or a stereoisomer, a tautomer, an N-oxide, a hydrate, a solvate, or a salt thereof, or a mixture of same.

### Embodiment 10:

A compound of general formula (I) according to any one of embodiments 1, 2, 3, 4, 5, 6, 7, 8 or 9 for the use as a medicament.

### Embodiment 11:

A compound of general formula (I) according to any one of embodiments 1, 2, 3, 4, 5, 6, 7, 8 or 9 for use in the treatment or prophylaxis of a disease.

### Embodiment 12:

A pharmaceutical composition comprising a compound of general formula (I) according to any one of embodiments 1, 2, 3, 4, 5, 6, 7, 8 or 9 and one or more pharmaceutically acceptable excipients.

### Embodiment 13:

A pharmaceutical combination comprising:
one or more first active ingredients, in particular compounds of general formula (I) according to any one of embodiments 1, 2, 3, 4, 5, 6, 7, 8 or 9 and
one or more pharmaceutical active anti cancer compounds or
one or more pharmaceutical active immune checkpoint inhibitors.

### Embodiment 14:

A pharmaceutical combination according to claim 10, characterized in that the pharmaceutical active immune checkpoint inhibitor is an antibody.

### Embodiment 15:

Use of a compound of general formula (I) according to any one of embodiments 1, 2, 3, 4, 5, 6, 7, 8 or 9 for the treatment or prophylaxis of a disease.

### Embodiment 16:

Use of a compound of general formula (I) according to any one of embodiments 1, 2, 3, 4, 5, 6, 7, 8 or 9 for the preparation of a medicament for the treatment or prophylaxis of a disease.

### Embodiment 17:

Use according to embodiments 15 or 16, wherein the disease is cancer or conditions with dysregulated immune responses or other disorders associated with aberrant MAP4K1 signaling, such as liquid and solid tumours.

### Embodiment 18:

Use according to embodiments 15 or 16, wherein the diseases, respectively the disorders are benign hyperplasias, atherosclerotic disorders, sepsis, autoimmune disorders, vascular disorders, viral infections, neurodegenerative disorders, in inflammatory disorders, and male fertility control.

This invention also covers the following embodiments:

### Embodiment A'

Compounds of formula (I') 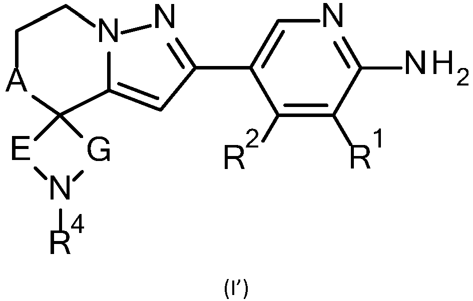 wherein
- A: is selected from a single bond, -CH₂- or -O-;
- E: is -(CH₂)ₙ- with n = 1 or 2;
- G: is -(CH₂)ₘ- with m = 1, 2 or 3 and with the condition, that the cyclic substructure comprising E-N-G is a 4 to 6 membered heterocycloalkyl;
- R¹: is selected from
- -NO₂; -CN;
- C₁₋₄-alkyl optionally substituted by -OH or 1 to 3 -F;
- 3 to 6 membered heterocycloalkyl with 1 - 2 nitrogen atoms;
- phenyl optionally substituted by halogen, -CN, C₁₋₄-alkyl, C₁₋₄-haloalkyl, C₃₋₆-cycloalkyl, O-C₁₋₄-alkyl, O-C₃₋₆-cycloalky, C₁₋₃-alkinyl, SR_{d}, SOR_{d}, SO₂R_{d}, SONR_{d} or SON(H)R_{d} wherein R_{d} is selected from methyl, ethyl, cyclopropyl, isopropyl and tert. butyl;
- 5 to 6 membered heteroaryl with 1 or 2 heteroatoms selected from N or S and optionally substituted by halogen, -CN, C₁₋₄-alkyl, C₁₋₄-haloalkyl, C₃₋₆-cycloalkyl, O-C₁₋₄-alkyl, O-C₃₋₆-cycloalky, C₁₋₃-alkinyl, SR_{d}, SOR_{d}, SO₂R_{d}, SONR_{d} or SON(H)R_{d} wherein R_{d} is selected from methyl, ethyl, cyclopropyl, isopropyl and tert. butyl; -O-CH₃; -O-CH(CH₃)₂; -O-CHF₂; -O-CF₃; 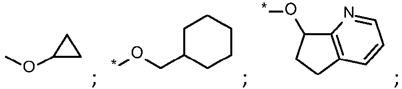
- -O-CHRₐ-phenyl optionally substituted by one or two halogen, -CN, C₁₋₄-alkyl or -SO₂-CH₃ and with Rₐ selected from -H or -CH₃, -CH₂-CH₃, -CH(CH₃)₂ or -cyclopropyl;
- -O-CHRₐ-6 membered heteroaryl optionally substituted one or two halogen, -CN, C₁₋₄-alkyl or -SO₂-CH₃ and with Rₐ selected from -H or -CH₃, -CH₂-CH₃, -CH(CH₃)₂ or -cyclopropyl;
- -COORₐ with Rₐ selected from -H or -CH₃;
- C(=O)-methyl, optionally substituted by 1 to 3 -F;
- -C(=O)-NR_{b}R_{c} wherein R_{b} is selected from -H, -CH₃ or -CH₂-CH₃ and wherein R_{c} is selected from -H, -CH₃, -CH₂-CH₃;
- -S-CR_{f}R_{g}Rₕ, wherein R_{f} is selected from -H or -F, wherein R_{g} is selected from -H, -F or -CH₃ and wherein Rₕ is selected from C₅₋₆-cycloalkyl or phenyl;
- 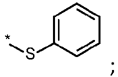
- R²: is selected from -H, -CH₃ or -F; or
- R⁴: is selected from
- C₁₋₄-alkyl or C₃₋₆-cycloalkyl, both optionally substituted one or more times by
   ∘ -F, -Cl, -Br, -CN;
   ∘ -OH, -ORᵢ wherein Rᵢ is selected from C₁₋₄-alkyl or C₃₋₆Cycloalkyl), -CFₓH_{y} with x and y = 0, 1, 2 or 3 and x + y = 3;
   ∘ -NRⱼRₖ wherein Rⱼ and Rₖ are independently selected from -H and C₁-C₄-alkyl or
   ∘ phenyl or 5 to 6 membered heteroaryl, both optionally substituted one or more times by
      ▪ halogen, -CN,
      ▪ -CFₓH_{y} with x and y = 0, 1, 2 or 3 and x + y = 3,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-Rₗ in which Rₗ is selected from
   ∘ C₁₋₄-alkyl or C₃₋₆-cycloalkyl, both optionally substituted one or more times by
      ▪ -F, -Cl, -Br, -CN;
      ▪ -OH, -ORᵢ with Rᵢ selected from C₁₋₄-alkyl or C₃₋₆Cycloalkyl), -CFₓH_{y} with x and y = 0, 1, 2 or 3 and x + y = 3;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CFₓH_{y} with x and y = 0, 1, 2 or 3 and x + y = 3,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   ∘ phenyl or 5 to 6 membered heteroaryl, both optionally substituted one or more times by
      ▪ halogen, -CN,
      ▪ -CFₓH_{y} with x and y = 0, 1, 2 or 3 and x + y = 3,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-NRₒRₚ wherein Rₒ and Rₚ are independently selected from
   ∘ H
   ∘ C₁₋₄-alkyl or C₃₋₆-cycloalkyl, both optionally substituted one or more times by
      ▪ -F, -Cl, -Br, -CN;
      ▪ -OH, -ORᵢ with Rᵢ selected from C₁₋₄-alkyl or C₃₋₆Cycloalkyl), -CFₓH_{y} with x and y = 0, 1, 2 or 3 and x + y = 3;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CFₓH_{y} with x and y = 0, 1, 2 or 3 and x + y = 3,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   ∘ phenyl or 5 to 6 membered heteroaryl, both optionally substituted one or more times by
      ▪ halogen, -CN,
      ▪ -CFₓH_{y} with x and y = 0, 1, 2 or 3 and x + y = 3,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
or a stereoisomer, a tautomer, an N-oxide, a hydrate, a solvate, or a salt thereof, or a mixture of same.

### Embodiment A"

Compounds of formula (I") 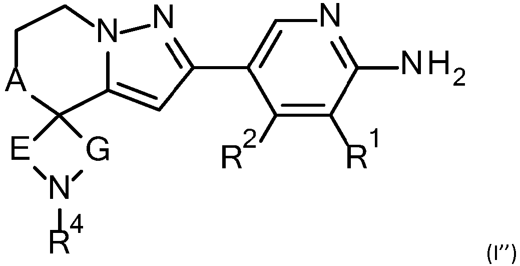 wherein
- A: is selected from a single bond, -CH₂- or -O-;
- E: is -(CH₂)ₙ- with n = 1 or 2;
- G: is -(CH₂)- and with the condition, that the cyclic substructure comprising E-N-G is a 4 to 5 membered heterocycloalkyl;
- R¹: is selected from
- -NO₂; -CN;
- C₁₋₄-alkyl optionally substituted by -OH or 1 to 3 -F;
- cyclopropyl;
- 4 to 6 membered heterocycloalkyl or heterocycloalkenyl, both with 1 - 2 nitrogen atoms, but always linked via a carbon atom to the phenyl group of compounds of formula (I) and both optionally substituted by C₁₋₄-alkyl;
- phenyl optionally substituted by halogen, -CN, C₁₋₄-alkyl, C₁₋₄-haloalkyl, C₃₋₆-cycloalkyl, O-C₁₋₄-alkyl, O-C₃₋₆-cycloalky, C₁₋₃-alkinyl, -C(O)-R_{d}, SR_{d}, SOR_{d}, SO₂R_{d}, SONR_{d}, SON(H)R_{d}, -SO₂-NH₂, - SO₂-N(H)-R_{d} or -SO₂-N(R_{d})₂, wherein R_{d} is selected from methyl, ethyl, cyclopropyl, isopropyl and tert. butyl;
- 5 to 6 membered heteroaryl with 1 or 2 heteroatoms selected from N or S and optionally substituted by halogen, -CN, C₁₋₄-alkyl, C₁₋₄-haloalkyl, C₃₋₆-cycloalkyl, O-C₁₋₄-alkyl, O-C₃₋₆-cycloalkyl, C₁₋₃-alkinyl, SR_{d}, SOR_{d}, SO₂R_{d}, SONR_{d} or SON(H)R_{d} wherein R_{d} is selected from methyl, ethyl, cyclopropyl, isopropyl and tert. butyl;
- -O-CH₃; -O-CH(CH₃)₂; -O-CHF₂; -O-CF₃; 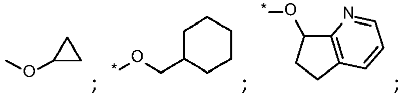
- -O-CHRₐ-phenyl optionally substituted by one or two halogen, -CN, C₁₋₄-alkyl or -SO₂-CH₃ and with Rₐ selected from -H or -CH₃, -CH₂-CH₃, -CH(CH₃)₂ or -cyclopropyl;
- -O-CHRₐ-6 membered heteroaryl optionally substituted one or two halogen, -CN, C₁₋₄-alkyl or -SO₂-CH₃ and with Rₐ selected from -H or -CH₃, -CH₂-CH₃, -CH(CH₃)₂ or -cyclopropyl;
- -COORₐ with Rₐ selected from -H or -CH₃;
- C(=O)-methyl, optionally substituted by 1 to 3 -F;
- -C(=O)-NR_{b}R_{c} wherein R_{b} is selected from -H, -CH₃ or -CH₂-CH₃ and wherein R_{c} is selected from -H, -CH₃, -CH₂-CH₃, cyclohexyl, phenyl or 5 to 6 membered heteroaryl, all optionally substituted by one or two halogen, -CN, -SO₂-CH₃ or C₁₋₄-alkyl, optionally substituted one or more times with halogen;
- -S-CR_{f}R_{g}Rₕ, wherein R_{f} is selected from -H or -F, wherein R_{g} is selected from -H, -F or -CH₃ and wherein Rₕ is selected from H, F, -CH₃ or C₅₋₆-cycloalkyl or phenyl;
- 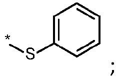
- R²: is selected from -H; or
- R⁴: is selected from
- C₁₋₅-alkyl or C₃₋₆-cycloalkyl, both optionally substituted one or independently more times by
   ∘ -F, -Cl, -Br, -CN;
   ∘ -OH, -ORᵢ wherein Rᵢ is selected from C₁₋₄-alkyl or C₃₋₆Cycloalkyl),
   ∘ -CF₃, -CHF₂, -CH₂F, or -CH₂-CF₃;
   ∘ -NRⱼRₖ wherein Rⱼ and Rₖ are independently selected from -H and C₁-C₄-alkyl,
   ∘ -CH₂-O-CH₃ or
   ∘ C₃₋₄ cycloalkyl or 4 membered heterocycloalkyl, both optionally substituted by -F or - CH₃;
   ∘ phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-Rₗ in which Rₗ is selected from
   ∘ C₁₋₄-alkyl, C₃₋₆-cycloalkyl or C₃₋₆-heterocycloalkyl, both optionally substituted one or more times by
      ▪ -F, -Cl, -Br, -CN;
      ▪ -OH, -ORᵢ with Rᵢ selected from C₁₋₄-alkyl or C₃₋₆Cycloalkyl), -CF₃, -CHF₂, -CH₂F;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CF₃, -CHF₂, -CH₂F,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   ∘ phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-NRₒRₚ wherein Rₒ and Rₚ are independently selected from
   o H
   o C₁₋₄-alkyl or C₃₋₆-cycloalkyl, both optionally substituted one or more times by
      ▪ -F, -Cl, -Br, -CN;
      ▪ -OH, -ORᵢ with Rᵢ selected from C₁₋₄-alkyl or C₃₋₆-cycloalkyl, -CF₃, -CHF₂, -CH₂F;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CF₃, -CHF₂, -CH₂F,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   o phenyl or 5 to 6 membered heteroaryl, both optionally substituted one or more times by
      ▪ halogen, -CN,
      ▪ -CF₃, -CHF₂, -CH₂F,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
or a stereoisomer, a tautomer, an N-oxide, a hydrate, a solvate, or a salt thereof, or a mixture of same.

### Embodiment B

Of selected interest are compounds of formulas (I), (Ia), (I') or (I") (see embodiments 1, A, A' and A"), wherein R₄ is 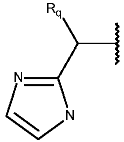 and R_{q} is selected from H, -CH₃,-CH₂-CH₃, -CH(CH₃)₂, -CH₂-O-CH₃, - CF₃, -CHF₂, -CH₂F, -CH₂-CF₃, cyclopropyl or cyclobutyl or a stereoisomer, a tautomer, an N-oxide, a hydrate, a solvate, or a salt thereof, or a mixture of same.

### Emboyment C

Of selected interest are compounds of formulas (I), (Ia), (I') or (I"), wherein R₄ is selected from C(=O)-NRₒRₚ wherein Rₒ and Rₚ are independently selected from
∘ H
∘ C₁₋₄-alkyl or C₃₋₆-cycloalkyl, both optionally substituted one or more times by
   ▪ -F, -Cl, -Br, -CN;
   ▪ -OH, -ORᵢ with Rᵢ selected from C₁₋₄-alkyl or C₃₋₆-cycloalkyl, -CF₃, -CHF₂, -CH₂F;
   ▪ -NRⱼRₖ wherein Rⱼ and Rₖ are independently selected from -H and C₁-C₄-alkyl
   ▪ phenyl or 5 to 6 membered heteroaryl, both optionally substituted one or more times by
      - halogen, -CN,
      - -CF₃, -CHF₂, -CH₂F,
      - C₁-₄-alkyl optionally substituted with one or more halogen;
      - C₃₋₆-cycloalkyl;
      - O-C₁₋₄-alkyl,
      - O-C₃₋₆-cycloalky,
      - C₁₋₃-alkinyl,
      - -NRⱼRₖ wherein Rⱼ and Rₖ are independently selected from -H and C₁-C₄-alkyl
      - SR_{d}, SOR_{d}, SO₂R_{d}, SONR_{d} or SON(H)R_{d} wherein R_{d} is selected from methyl, ethyl, cyclopropyl, isopropyl and tert. butyl;
or a stereoisomer, a tautomer, an N-oxide, a hydrate, a solvate, or a salt thereof, or a mixture of same.

### Embodiment C

Of selected interest are compounds of formulas (I), (Ia), (I') or (I") (see embodiments 1, A, A' and A"), wherein A is selected from a single bond or -O- or a stereoisomer, a tautomer, an N-oxide, a hydrate, a solvate, or a salt thereof, or a mixture of same.

### Embodiment D

Of selected interest are compounds of formulas (I), (Ia), (I') or (I") (see embodiments 1, A, A' and A"), wherein E is -CH₂-CH₂- or a stereoisomer, a tautomer, an N-oxide, a hydrate, a solvate, or a salt thereof, or a mixture of same.

Of selected interest are also the following groups of compounds:

### Embodiment E

The compound of formula (I-1) 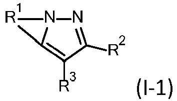 wherein
- R¹: is selected from the group consisting of 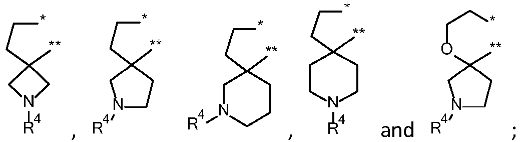
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 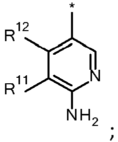
- R³: is selected from the group consisting of -H, -Cl, and -Br;
- R⁴: is selected from the group consisting of
- C₁₋₄-alkyl or C₃₋₆-cycloalkyl, both optionally substituted one or more times by
   ∘ -F, -Cl, -Br, -CN;
   ∘ -OH, -ORᵢ wherein Rᵢ is selected from C₁₋₄-alkyl or C₃₋₆Cycloalkyl), -CFₓH_{y} with x and y = 0, 1, 2 or 3 and x + y = 3;
   ∘ -NRⱼRₖ wherein Rⱼ and Rₖ are independently selected from -H and C₁-C₄-alkyl or
   ∘ phenyl or 5 to 6 membered heteroaryl, both optionally substituted one or more times by
      ▪ halogen, -CN,
      ▪ -CFₓH_{y} with x and y = 0, 1, 2 or 3 and x + y = 3,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-Rₗ in which Rₗ is selected from
   ∘ C₁₋₄-alkyl or C₃₋₆-cycloalkyl, both optionally substituted one or more times by
      ▪ -F, -Cl, -Br, -CN;
      ▪ -OH, -ORᵢ with Rᵢ selected from C₁₋₄-alkyl or C₃₋₆Cycloalkyl), -CFₓH_{y} with x and y = 0, 1, 2 or 3 and x + y = 3;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CFₓH_{y} with x and y = 0, 1, 2 or 3 and x + y = 3,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   o phenyl or 5 to 6 membered heteroaryl, both optionally substituted one or more times by
      ▪ halogen, -CN,
      ▪ -CFₓH_{y} with x and y = 0, 1, 2 or 3 and x + y = 3,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- C(=O)-NRₒRₚ wherein Rₒ and Rₚ are independently selected from
   ∘ H
   ∘ C₁₋₄-alkyl or C₃₋₆-cycloalkyl, both optionally substituted one or more times by
      ▪ -F, -Cl, -Br, -CN;
      ▪ -OH, -ORᵢ with Rᵢ selected from C₁₋₄-alkyl or C₃₋₆Cycloalkyl), -CFₓH_{y} with x and y = 0, 1, 2 or 3 and x + y = 3;
      ▪ -NRⱼRₖ wherein Rⱼ and Rₖ are independently selected from -H and C₁-C₄-alkyl
      ▪ phenyl or 5 to 6 membered heteroaryl, both optionally substituted one or more times by
         - halogen, -CN,
         - -CFₓH_{y} with x and y = 0, 1, 2 or 3 and x + y = 3,
         - C₁-₄-alkyl optionally substituted with one or more halogen;
         - C₃₋₆-cycloalkyl;
         - O-C₁₋₄-alkyl,
         - O-C₃₋₆-cycloalky,
         - C₁₋₃-alkinyl,
         - -NRⱼRₖ wherein Rⱼ and Rₖ are independently selected from -H and C₁-C₄-alkyl
         - SR_{d}, SOR_{d}, SO₂R_{d}, SONR_{d} or SON(H)R_{d} wherein R_{d} is selected from methyl, ethyl, cyclopropyl, isopropyl and tert. butyl;
   ∘ phenyl or 5 to 6 membered heteroaryl, both optionally substituted one or more times by
      ▪ halogen, -CN,
      ▪ -CFₓH_{y} with x and y = 0, 1, 2 or 3 and x + y = 3,
      ▪ C₁-₄-alkyl optionally substituted with one or more halogen;
      ▪ C₃₋₆-cycloalkyl;
      ▪ O-C₁₋₄-alkyl,
      ▪ O-C₃₋₆-cycloalky,
      ▪ C₁₋₃-alkinyl,
      ▪ -NRⱼRₖ wherein Rⱼ and Rₖ are independently selected from -H and C₁-C₄-alkyl
      ▪ SR_{d}, SOR_{d}, SO₂R_{d}, SONR_{d} or SON(H)R_{d} wherein R_{d} is selected from methyl, ethyl, cyclopropyl, isopropyl and tert. butyl;
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 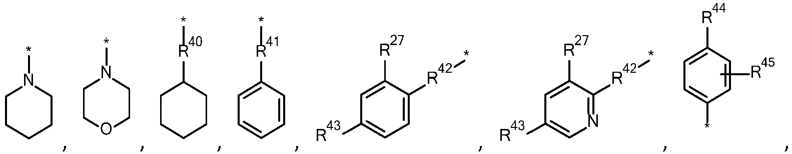 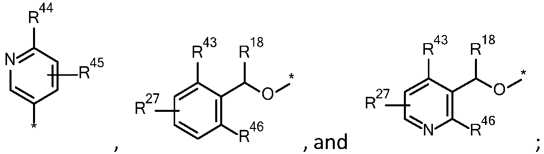
- R¹²: is selected from the group consisting of -H, -CH₃, -F, and -CF₃;
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and - N-CH₂-, -O-CH₂-, and -S-CH₂-;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁶⁴: is selected from the group consisting of -CH₃, -NH₂, -NHCH₃, cyclopropyl, and -N(CH₃)₂;
or a stereoisomer, a tautomer, an N-oxide, a hydrate, a solvate, or a salt thereof, or a mixture of same.

### Embodiment F

Compound according to embodiment E wherein
- R¹: is selected from the group consisting of 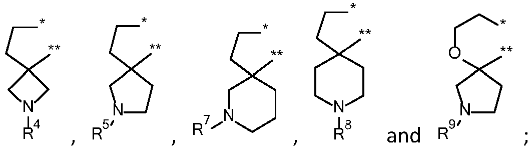
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 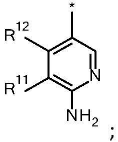
- R³: is selected from the group consisting of -H, -Cl, and -Br;
- R⁴: is selected from the group consisting of -CONHR¹³, (1H-imidazol-2-yl)methyl, and -COOC(CH₃)₃;
- R⁵: is selected from the group consisting of -COOC(R¹⁷)(R¹⁸)₂, -C(=R²¹)NHC(R¹⁸)(R¹⁹)R²⁰, 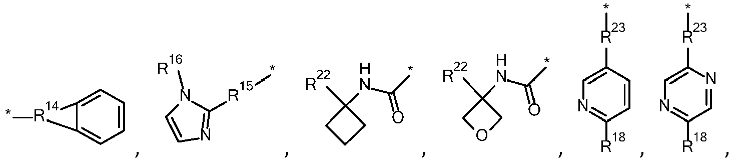 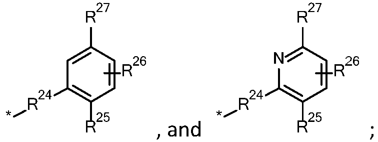
- R⁷: is selected from the group consisting of -CONHR²⁸ and -COOC(CH₃)₃;
- R⁸: is selected from the group consisting of -CONHR²⁹, (1H-imidazol-2-yl)methyl, and -COOC(CH₃)₃;
- R⁹: is selected from the group consisting of -CONHR³⁰, (1H-imidazol-2-yl)methyl, and -COOC(CH₃)₃;
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 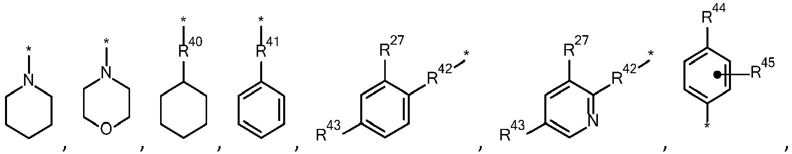 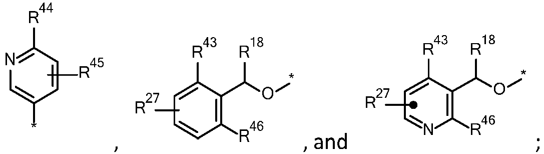
- R¹²: is selected from the group consisting of -H, -CH₃, -F, and -CF₃;
- R¹³: is selected from the group consisting of -CH₂CH₃, -C(CH₃)₂R⁴⁷, bicyclo[2.2.1]heptan-1-yl, (pyrimidin-4-yl)methyl, 1-(1,2-oxazol-3-yl)ethyl, 1-(5-fluoropyridin-3-yl)ethyl, 1-(1,3,4-trimethyl-1H-pyrazol-5-yl)ethyl, 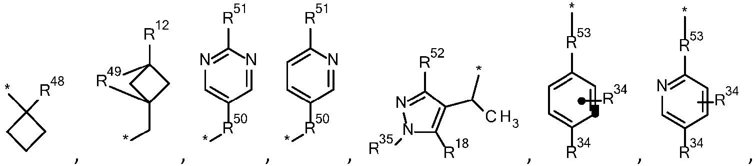 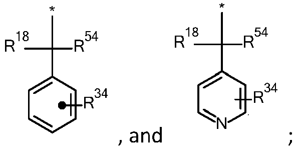
- R¹⁴: is selected from the group consisting of 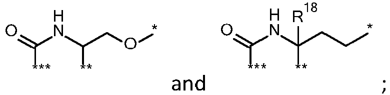
- R¹⁵: is selected from the group consisting of -CH₂- and -CHCH₃NHCO-;
- R¹⁶: is selected from the group consisting of -H, -CH₃, and -CH₂CH₃;
- R¹⁷: is selected from the group consisting of -CH₃ and phenyl;
- R¹⁸: is selected from the group consisting of -H and -CH₃;
- R¹⁹: is selected from the group consisting of -CH₃, cyclopropyl, 1,2-oxazol-3-yl, pyridazin-3-yl, pyridazin-4-yl, oxan-4-yl, 1-methyl-1H-1,2,3-triazol-4-yl, 1-methylpiperidin-4-yl, 1,3,4-trimethyl-1H-pyrazol-5-yl, 1-methyl-2-oxopiperidin-4-yl, 1-methyl-1H-1,3-benzodiazol-2-yl, 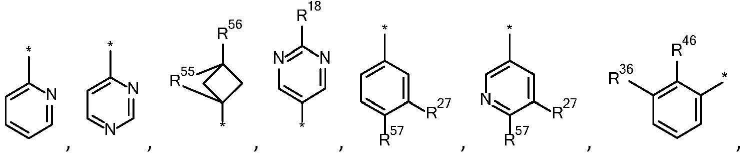 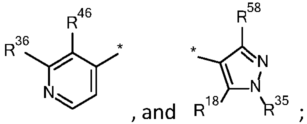
- R²⁰: is selected from the group consisting of -H, -CH₃, -CH₂CH₃, -CH(CH₃)₂, and -CH₂OCH₃;
- R²¹: is selected from the group consisting of =O and =NCN;
- R²²: is selected from the group consisting of -H, pyridin-4-yl, pyridin-3-yl, 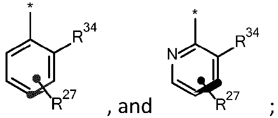
- R²³: is selected from the group consisting of -C(CH₃)₂CO- and -CH(R¹⁸)NHCO-;
- R²¹: is selected from the group consisting of -NHCO-, -C(CH₃)₂CO-, -C(R⁵⁹)(R⁶⁰)NHCO-, 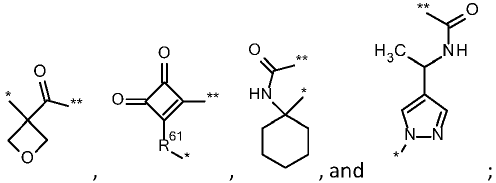
- R²⁵: is selected from the group consisting of -H, -F, -Cl, -CN, and -SO₂NHCH₃;
- R²⁶: is selected from the group consisting of -H, -F, -CN, and -CF₃;
- R²⁷: is selected from the group consisting of -H and -F;
- R²⁸: is selected from the group consisting of -CH₂CH₃, -CH(CH₃)₂, and cyclobutyl;
- R²⁹: is selected from the group consisting of -CH₂CH₃, -CH(CH₃)₂, cyclobutyl, and 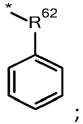
- R³⁰: is selected from the group consisting of -CH₂CH₃, cyclobutyl, -C(CH₃)₂R⁶³, 1-(5-fluoropyridin-3-yl)ethyl, 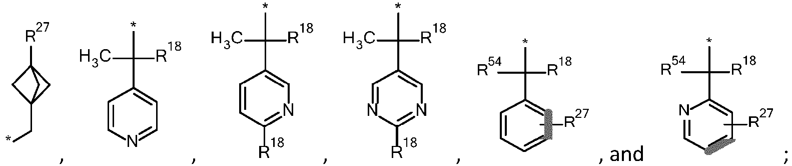
- R³¹: is selected from the group consisting of -H, -F, -CI, and -CN;
- R³⁵: is selected from the group consisting of -CH₃ and -CH₂CH₃;
- R³⁶: is selected from the group consisting of -H, -F, and -CN;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and - N-CH₂-, -O-CH₂-, and -S-CH₂-;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁴⁷: is selected from the group consisting of -H, 1-methyl-1H-1,2,3-triazol-4-yl, 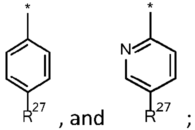
- R⁴⁸: is selected from the group consisting of -H, -CH₃, -CH(CH₃)₂, and pyridin-4-yl;
- R⁴⁹: is selected from the group consisting of -CH₂- and -CH₂O-;
- R⁵⁰: is selected from the group consisting of -CH₂-, -CHCH₃-, -C(CH₃)₂-, and 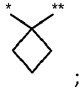
- R⁵¹: is selected from the group consisting of -H, -CH₃, and -CN;
- R⁵²: is selected from the group consisting of -H, -CH₃, and -CHF₂;
- R⁵³: is selected from the group consisting of -CH₂-, -CHCH₃-, -C(CH₃)₂-, 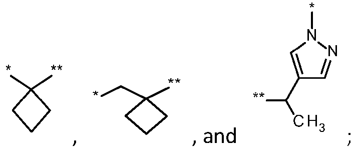
- R⁵⁴: is selected from the group consisting of -H, -CH₃, -CH₂CH₃, and -CH₂OCH₃;
- R⁵⁵: is selected from the group consisting of -CH₂- and -OCH₂-;
- R⁵⁶: is selected from the group consisting of -CH₃ and -CF₃;
- R⁵⁷: is selected from the group consisting of -H, -CH₃, -F, and -CN;
- R⁵⁸: is selected from the group consisting of -H, -CH₃, and -CF₃;
- R⁵⁹: is selected from the group consisting of -H, -CH₃, cyclopropyl, -CH₂OCH₃, and -CF₃;
- R⁶⁰: is selected from the group consisting of -H, -CH₃, and -OCH₃;
- R⁶¹: is selected from the group consisting of -NH- and -NHCH₂-;
- R⁶²: is selected from the group consisting of -CH₂- and 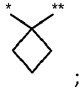
- R⁶³: is selected from the group consisting of -H, 1-methyl-1H-1,2,3-triazol-4-yl, and 3-chloropyridin-4-yl;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodiment G

The compound according to embodiment E wherein
- R¹: is selected from 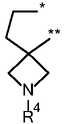
or a stereoisomer, a tautomer, an N-oxide, a hydrate, a solvate, or a salt thereof, or a mixture of same.

### Embodiment H

The compound according to embodiment E wherein
- R¹: is selected from 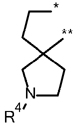
or a stereoisomer, a tautomer, an N-oxide, a hydrate, a solvate, or a salt thereof, or a mixture of same.

### Embodyment I

The compound according to embodiment E wherein
- R¹: is selected from the group consisting of 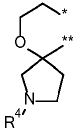
or a stereoisomer, a tautomer, an N-oxide, a hydrate, a solvate, or a salt thereof, or a mixture of same.

### Embodiment J

The compound according to embodiment F wherein
- R¹: is selected from 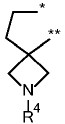
or a stereoisomer, a tautomer, an N-oxide, a hydrate, a solvate, or a salt thereof, or a mixture of same.

### Embodiment K

The compound according to embodiment F wherein
- R¹: is selected from 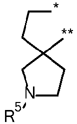
or a stereoisomer, a tautomer, an N-oxide, a hydrate, a solvate, or a salt thereof, or a mixture of same.

### Embodiment L

The compound according to embodiment F wherein
- R¹: is selected from 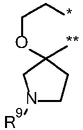
or a stereoisomer, a tautomer, an N-oxide, a hydrate, a solvate, or a salt thereof, or a mixture of same.
and their polymorphs, enantiomers, diastereomers, racemates, tautomers, solvates, physiologically acceptable salts and solvates of these salts.

### Embodiment M

Compound of formula (I-2) 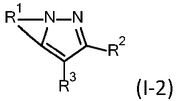 wherein
- R¹: is selected from the group consisting of 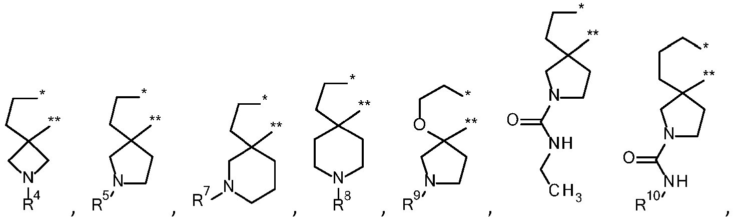 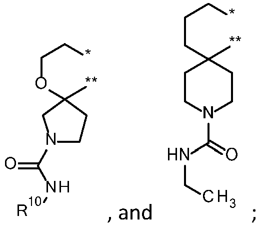
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 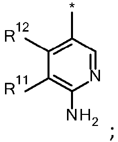
- R³: is selected from the group consisting of -H, -Cl, and -Br;
- R⁴: is selected from the group consisting of -CONHR¹³, (1H-imidazol-2-yl)methyl, and -COOC(CH₃)₃;
- R³: is selected from the group consisting of -COOC(R¹⁷)(R¹⁸)₂, -C(=R²¹)NHC(R¹⁸)(R¹⁹)R²⁰, 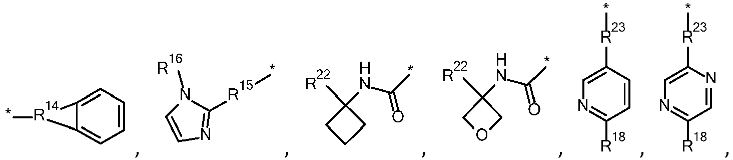 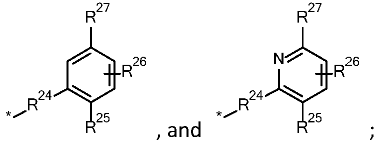
- R⁷: is selected from the group consisting of -CONHR²⁸ and -COOC(CH₃)₃;
- R³: is selected from the group consisting of -CONHR²⁹, (1H-imidazol-2-yl)methyl, and -COOC(CH₃)₃;
- R⁹: is selected from the group consisting of -CONHR³⁰, (1H-imidazol-2-yl)methyl, and -COOC(CH₃)₃;
- R¹⁰: is selected from the group consisting of -CH₂CH₃, {1-methyl-2-oxabicyclo[2.1.1]hexan-4-yl}methyl, 1-(1,3,4-trimethyl-1H-pyrazol-5-yl)ethyl, 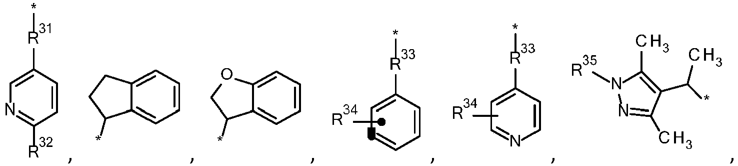 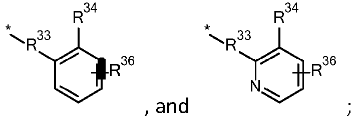
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 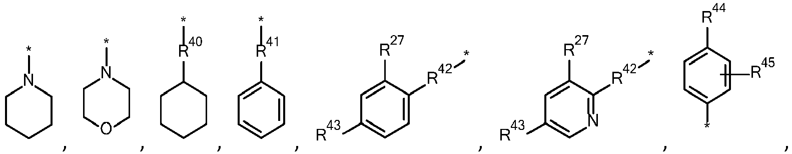 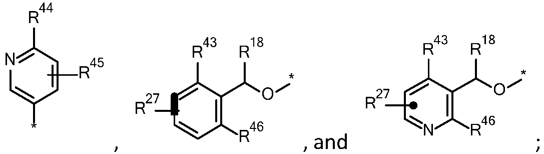
- R¹²: is selected from the group consisting of -H, -CH₃, -F, and -CF₃;
- R¹³: is selected from the group consisting of -CH₂CH₃, -C(CH₃)₂R⁴⁷, bicyclo[2.2.1]heptan-1-yl, (pyrimidin-4-yl)methyl, 1-(1,2-oxazol-3-yl)ethyl, 1-(5-fluoropyridin-3-yl)ethyl, 1-(1,3,4-trimethyl-1H-pyrazol-5-yl)ethyl, 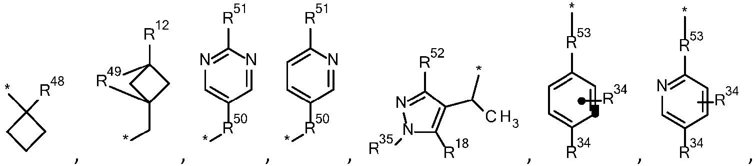 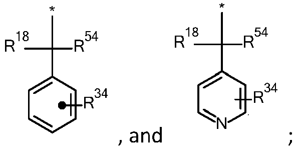
- R¹⁴: is selected from the group consisting of 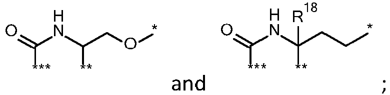
- R¹⁵: is selected from the group consisting of -CH₂- and -CHCH₃NHCO-;
- R¹⁶: is selected from the group consisting of -H, -CH₃, and -CH₂CH₃;
- R¹⁷: is selected from the group consisting of -CH₃ and phenyl;
- R¹⁸: is selected from the group consisting of -H and -CH₃;
- R¹⁹: is selected from the group consisting of -CH₃, cyclopropyl, 1,2-oxazol-3-yl, pyridazin-3-yl, pyridazin-4-yl, oxan-4-yl, 1-methyl-1H-1,2,3-triazol-4-yl, 1-methylpiperidin-4-yl, 1,3,4-trimethyl-1H-pyrazol-5-yl, 1-methyl-2-oxopiperidin-4-yl, 1-methyl-1H-1,3-benzodiazol-2-yl, 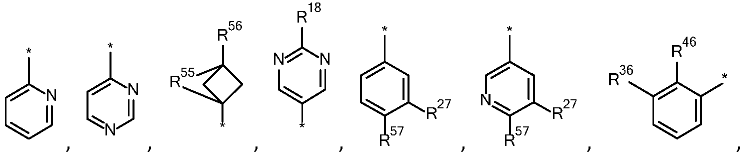 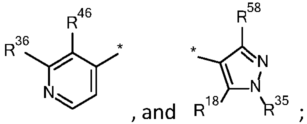
- R²⁰: is selected from the group consisting of -H, -CH₃, -CH₂CH₃, -CH(CH₃)₂, and -CH₂OCH₃;
- R²¹: is selected from the group consisting of =O and =NCN;
- R²²: is selected from the group consisting of -H, pyridin-4-yl, pyridin-3-yl, 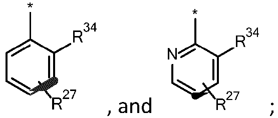
- R²³: is selected from the group consisting of -C(CH₃)₂CO- and -CH(R¹⁸)NHCO-;
- R²¹: is selected from the group consisting of -NHCO-, -C(CH₃)₂CO-, -C(R⁵⁹)(R⁶⁰)NHCO-, 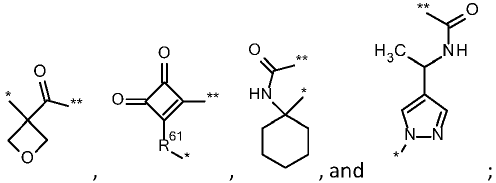
- R²⁵: is selected from the group consisting of -H, -F, -Cl, -CN, and -SO₂NHCH₃;
- R²⁶: is selected from the group consisting of -H, -F, -CN, and -CF₃;
- R²⁷: is selected from the group consisting of -H and -F;
- R²⁸: is selected from the group consisting of -CH₂CH₃, -CH(CH₃)₂, and cyclobutyl;
- R²⁹: is selected from the group consisting of -CH₂CH₃, -CH(CH₃)₂, cyclobutyl, and 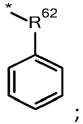
- R³⁰: is selected from the group consisting of -CH₂CH₃, cyclobutyl, -C(CH₃)₂R⁶³, 1-(5-fluoropyridin-3-yl)ethyl, 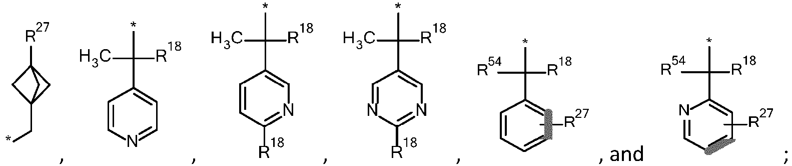
- R³¹: is selected from the group consisting of -CHCH₃- and 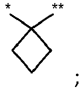
- R³²: is selected from the group consisting of -H and -CN;
- R³³: is selected from the group consisting of -CHCH₃-, -C(CH₃)₂-, and 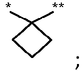
- R³⁴: is selected from the group consisting of -H, -F, -CI, and -CN;
- R³⁵: is selected from the group consisting of -CH₃ and -CH₂CH₃;
- R³⁶: is selected from the group consisting of -H, -F, and -CN;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl; R⁴⁴ is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁴⁷: is selected from the group consisting of -H, 1-methyl-1H-1,2,3-triazol-4-yl, 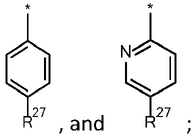
- R⁴⁸: is selected from the group consisting of -H, -CH₃, -CH(CH₃)₂, and pyridin-4-yl;
- R⁴⁹: is selected from the group consisting of -CH₂- and -CH₂O-;
- R⁵⁰: is selected from the group consisting of -CH₂-, -CHCH₃-, -C(CH₃)₂-, and 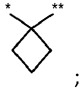
- R⁵¹: is selected from the group consisting of -H, -CH₃, and -CN;
- R⁵²: is selected from the group consisting of -H, -CH₃, and -CHF₂;
- R⁵³: is selected from the group consisting of -CH₂-, -CHCH₃-, -C(CH₃)₂-, 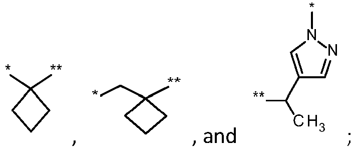
- R⁵⁴: is selected from the group consisting of -H, -CH₃, -CH₂CH₃, and -CH₂OCH₃;
- R⁵⁵: is selected from the group consisting of -CH₂- and -OCH₂-;
- R⁵⁶: is selected from the group consisting of -CH₃ and -CF₃;
- R⁵⁷: is selected from the group consisting of -H, -CH₃, -F, and -CN;
- R⁵⁸: is selected from the group consisting of -H, -CH₃, and -CF₃;
- R⁵⁹: is selected from the group consisting of -H, -CH₃, cyclopropyl, -CH₂OCH₃, and -CF₃;
- R⁶⁰: is selected from the group consisting of -H, -CH₃, and -OCH₃;
- R⁶¹: is selected from the group consisting of -NH- and -NHCH₂-;
- R⁶²: is selected from the group consisting of -CH₂- and 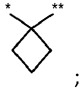
- R⁶³: is selected from the group consisting of -H, 1-methyl-1H-1,2,3-triazol-4-yl, and 3-chloropyridin-4-yl;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodiment N

A compound according to embodiment M of formula (I-3) 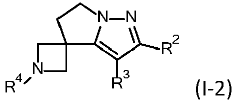 wherein
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 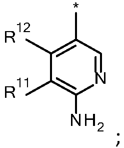
- R³: is selected from the group consisting of -H, -Cl, and -Br;
- R⁴: is selected from the group consisting of -CONHR¹³, (1H-imidazol-2-yl)methyl, and -COOC(CH₃)₃;
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 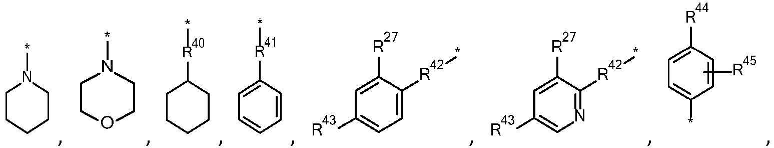 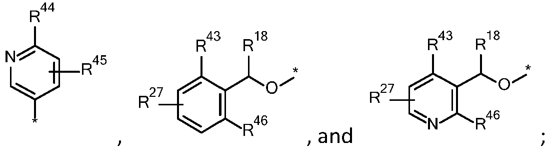
- R¹²: is selected from the group consisting of -H, -CH₃, -F, and -CF₃;
- R¹³: is selected from the group consisting of -CH₂CH₃, -C(CH₃)₂R⁴⁷, bicyclo[2.2.1]heptan-1-yl, (pyrimidin-4-yl)methyl, 1-(1,2-oxazol-3-yl)ethyl, 1-(5-fluoropyridin-3-yl)ethyl, 1-(1,3,4-trimethyl-1H-pyrazol-5-yl)ethyl, 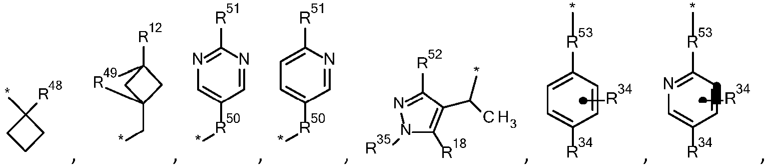 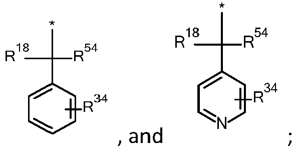
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R³⁴: is selected from the group consisting of -H, -F, -CI, and -CN;
- R³⁵: is selected from the group consisting of -CH₃ and -CH₂CH₃;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁴⁷: is selected from the group consisting of -H, 1-methyl-1H-1,2,3-triazol-4-yl, 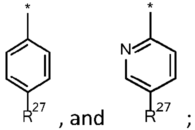
- R⁴⁸: is selected from the group consisting of -H, -CH₃, -CH(CH₃)₂, and pyridin-4-yl;
- R⁴⁹: is selected from the group consisting of -CH₂- and -CH₂O-;
- R⁵⁰: is selected from the group consisting of -CH₂-, -CHCH₃-, -C(CH₃)₂-, and 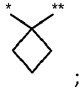
- R⁵¹: is selected from the group consisting of -H, -CH₃, and -CN;
- R⁵²: is selected from the group consisting of -H, -CH₃, and -CHF₂;
- R⁵³: is selected from the group consisting of -CH₂-, -CHCH₃-, -C(CH₃)₂-, 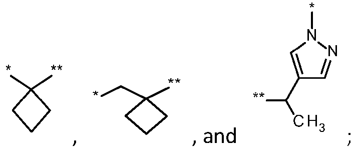
- R⁵⁴: is selected from the group consisting of -H, -CH₃, -CH₂CH₃, and -CH₂OCH₃;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodiment O

Compound according to embodiment M of formula (I-4) 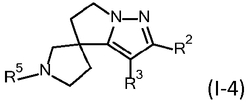 wherein
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 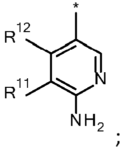
- R³: is selected from the group consisting of -H, -Cl, and -Br;
- R³: is selected from the group consisting of -COOC(R¹⁷)(R¹⁸)₂, -C(=R²¹)NHC(R¹⁸)(R¹⁹)R²⁰, 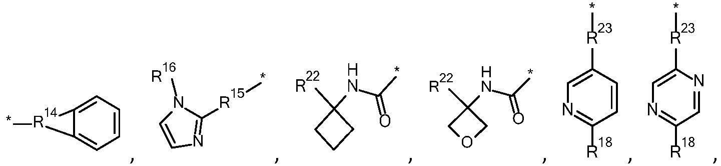 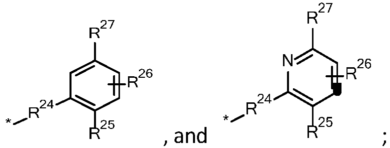
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 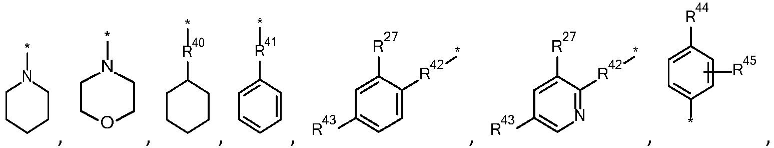 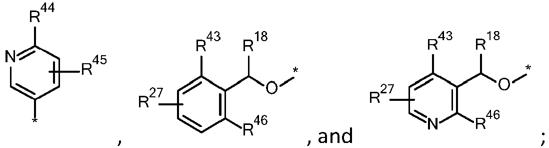
- R¹²: is selected from the group consisting of -H, -CH₃, -F, and -CF₃;
- R¹⁴: is selected from the group consisting of 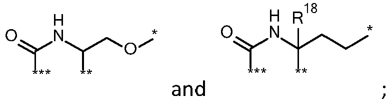
- R¹⁵: is selected from the group consisting of -CH₂- and -CHCH₃NHCO-;
- R¹⁶: is selected from the group consisting of -H, -CH₃, and -CH₂CH₃;
- R¹⁷: is selected from the group consisting of -CH₃ and phenyl;
- R¹⁸: is selected from the group consisting of -H and -CH₃;
- R¹⁹: is selected from the group consisting of -CH₃, cyclopropyl, 1,2-oxazol-3-yl, pyridazin-3-yl, pyridazin-4-yl, oxan-4-yl, 1-methyl-1H-1,2,3-triazol-4-yl, 1-methylpiperidin-4-yl, 1,3,4-trimethyl-1H-pyrazol-5-yl, 1-methyl-2-oxopiperidin-4-yl, 1-methyl-1H-1,3-benzodiazol-2-yl, 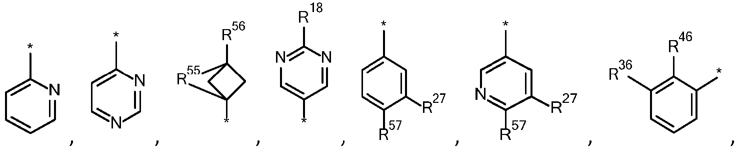 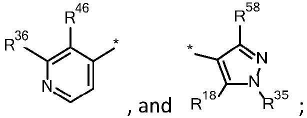
- R²⁰: is selected from the group consisting of -H, -CH₃, -CH₂CH₃, -CH(CH₃)₂, and -CH₂OCH₃;
- R²¹: is selected from the group consisting of =O and =NCN;
- R²²: is selected from the group consisting of -H, pyridin-4-yl, pyridin-3-yl, 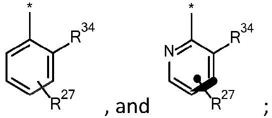
- R²³: is selected from the group consisting of -C(CH₃)₂CO- and -CH(R¹⁸)NHCO-;
- R²⁴: is selected from the group consisting of -NHCO-, -C(CH₃)₂CO-, -C(R⁵⁹)(R⁶⁰)NHCO-, 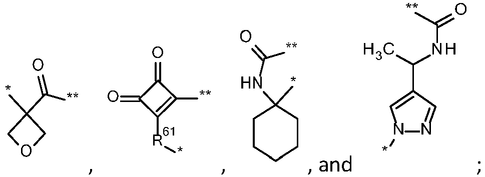
- R²⁵: is selected from the group consisting of -H, -F, -Cl, -CN, and -SO₂NHCH₃;
- R²⁶: is selected from the group consisting of -H, -F, -CN, and -CF₃;
- R²⁷: is selected from the group consisting of -H and -F;
- R³⁴: is selected from the group consisting of -H, -F, -CI, and -CN;
- R³⁵: is selected from the group consisting of -CH₃ and -CH₂CH₃;
- R³⁶: is selected from the group consisting of -H, -F, and -CN;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁵⁵: is selected from the group consisting of -CH₂- and -OCH₂-;
- R⁵⁶: is selected from the group consisting of -CH₃ and -CF₃;
- R⁵⁷: is selected from the group consisting of -H, -CH₃, -F, and -CN;
- R⁵⁸: is selected from the group consisting of -H, -CH₃, and -CF₃;
- R⁵⁹: is selected from the group consisting of -H, -CH₃, cyclopropyl, -CH₂OCH₃, and -CF₃;
- R⁶⁰: is selected from the group consisting of -H, -CH₃, and -OCH₃;
- R⁶¹: is selected from the group consisting of -NH- and -NHCH₂-;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodiment P

A compound according to embodiment M of formula (I-5) 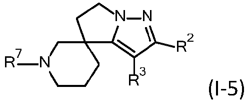 wherein
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 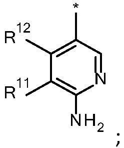
- R³: is selected from the group consisting of -H, -Cl, and -Br;
- R⁷: is selected from the group consisting of -CONHR²⁸ and -COOC(CH₃)₃;
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 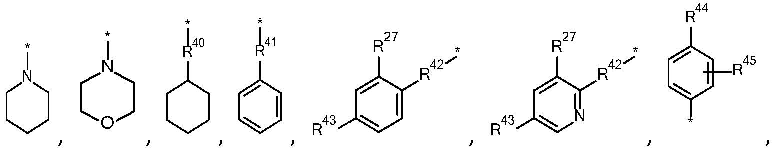 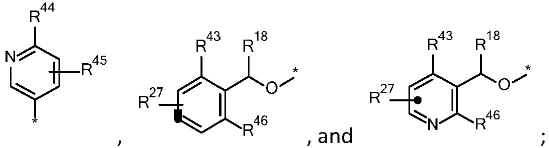
- R¹²: is selected from the group consisting of -H, -CH₃, -F, and -CF₃;
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R²⁸: is selected from the group consisting of -CH₂CH₃, -CH(CH₃)₂, and cyclobutyl;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁶⁴: is selected from the group consisting of -CH₃, -NH₂, -NHCH₃, cyclopropyl, and -N(CH₃)₂ or a stereoisomer, a tautomer, an N-oxide, a hydrate, a solvate, or a salt thereof, or a mixture of same.

### Embodyment Q

A compound according to embodiment M of formula (I-6) 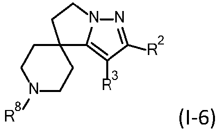
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 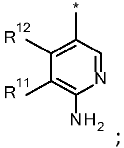
- R³: is selected from the group consisting of -H, -Cl, and -Br;
- R³: is selected from the group consisting of -CONHR²⁹, (1H-imidazol-2-yl)methyl, and -COOC(CH₃)₃;
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 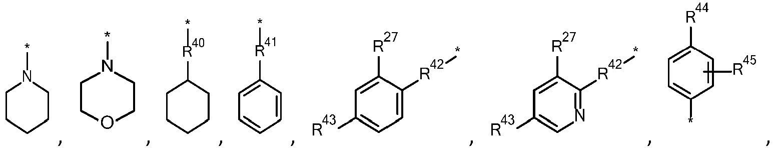 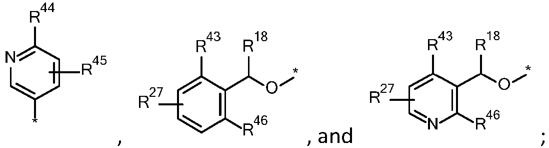
- R¹²: is selected from the group consisting of -H, -CH₃, -F, and -CF₃;
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R²⁹: is selected from the group consisting of -CH₂CH₃, -CH(CH₃)₂, cyclobutyl, and 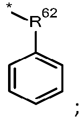
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁶²: is selected from the group consisting of -CH₂- and 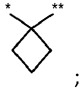
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodiment R

A compound according to embodiment M of formula (I-7) 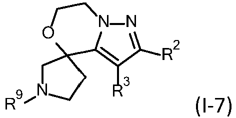 wherein
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 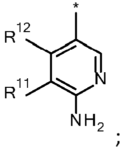
- R³: is selected from the group consisting of -H, -Cl, and -Br;
- R⁹: is selected from the group consisting of -CONHR³⁰, (1H-imidazol-2-yl)methyl, and -COOC(CH₃)₃;
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 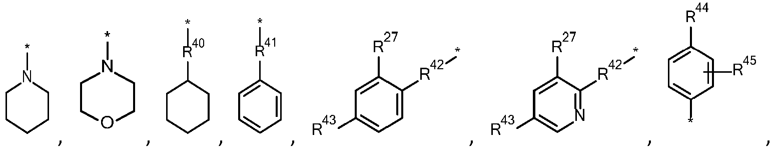 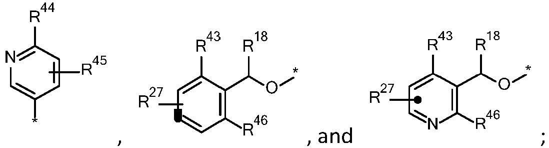
- R¹²: is selected from the group consisting of -H, -CH₃, -F, and -CF₃;
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R³⁰: is selected from the group consisting of -CH₂CH₃, cyclobutyl, -C(CH₃)₂R⁶³, 1-(5-fluoropyridin-3-yl)ethyl, 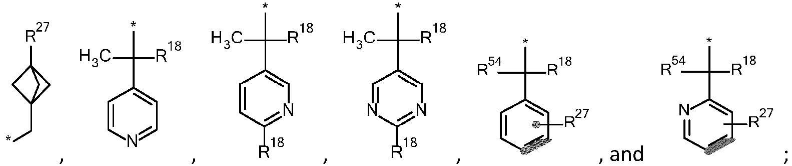
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁵⁴: is selected from the group consisting of -H, -CH₃, -CH₂CH₃, and -CH₂OCH₃;
- R⁶³: is selected from the group consisting of -H, 1-methyl-1H-1,2,3-triazol-4-yl, and 3-chloropyridin-4-yl;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodiment S

A compound according to embodiment M of formula (I-8) 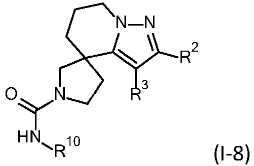 wherein
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 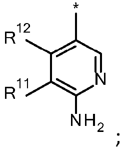
- R³: is selected from the group consisting of -H, -Cl, and -Br;
- R¹⁰: is selected from the group consisting of -CH₂CH₃, {1-methyl-2-oxabicyclo[2.1.1]hexan-4-yl}methyl, 1-(1,3,4-trimethyl-1H-pyrazol-5-yl)ethyl, 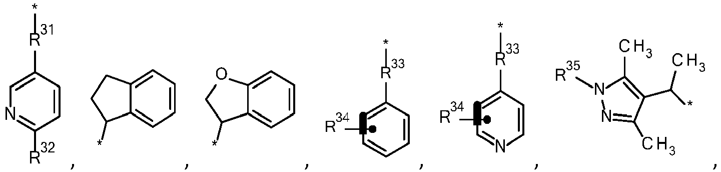 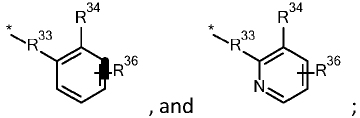
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 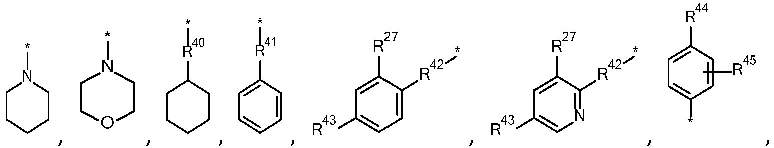 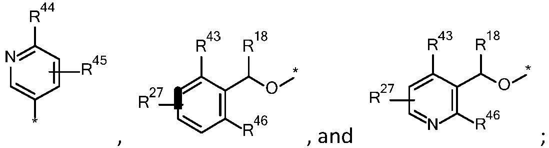
- R¹²: is selected from the group consisting of -H, -CH₃, -F, and -CF₃;
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R³¹: is selected from the group consisting of -CHCH₃- and 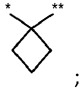
- R³²: is selected from the group consisting of -H and -CN;
- R³³: is selected from the group consisting of -CHCH₃-, -C(CH₃)₂-, and 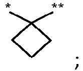
- R³⁴: is selected from the group consisting of -H, -F, -CI, and -CN;
- R³⁵: is selected from the group consisting of -CH₃ and -CH₂CH₃;
- R³⁶: is selected from the group consisting of -H, -F, and -CN;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodiment T

A compound according to embodyment M of formula (I-9) 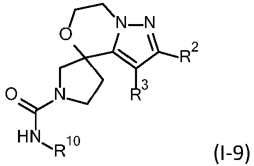 wherein
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 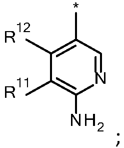
- R³: is selected from the group consisting of -H, -Cl, and -Br;
- R¹⁰: is selected from the group consisting of -CH₂CH₃, {1-methyl-2-oxabicyclo[2.1.1]hexan-4-yl}methyl, 1-(1,3,4-trimethyl-1H-pyrazol-5-yl)ethyl, 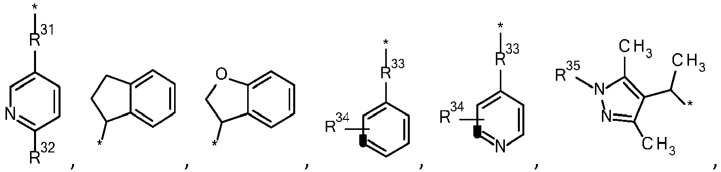 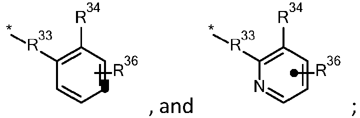
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 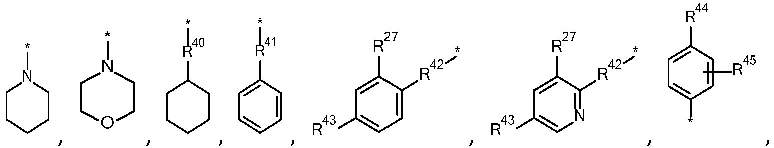 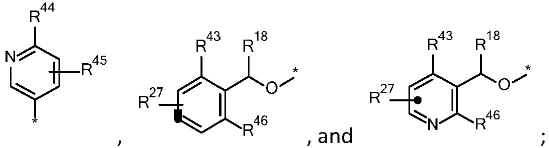
- R¹²: is selected from the group consisting of -H, -CH₃, -F, and -CF₃;
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R³¹: is selected from the group consisting of -CHCH₃- and 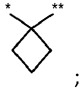
- R³²: is selected from the group consisting of -H and -CN;
- R³³: is selected from the group consisting of -CHCH₃-, -C(CH₃)₂-, and 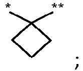
- R³⁴: is selected from the group consisting of -H, -F, -CI, and -CN;
- R³⁵: is selected from the group consisting of -CH₃ and -CH₂CH₃;
- R³⁶: is selected from the group consisting of -H, -F, and -CN;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

### Embodyment U

A compound according to embodyment M of formula (I-10) 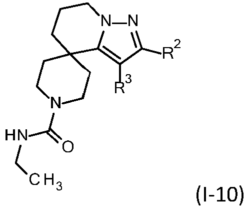 wherein
- R²: is selected from the group consisting of 4-amino-1H-imidazo[4,5-c]pyridin-7-yl, 6-amino-5-cyclohexylmethanesulfonyl-1-oxidopyridin-1-ium-3-yl, and 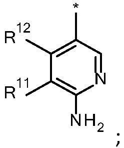
- R³: is selected from the group consisting of -H, -Cl, and -Br;
- R¹¹: is selected from the group consisting of -H, -CH₃, -F, -Cl, -Br, -CN, -OCH₃, -CH₂OH, -SCH₃, cyclopropyl, -NO₂, -NHCCH₃O, -P(CH₃)₂O, -C(CH₃)₂R³⁷, -COOR¹⁸, -CF₂R³⁸, -SO₂R³⁹, -SC(CH₃)₃, 1,2-thiazol-5-yl, -SCF₃, -OCF₂R²⁷, pyridin-4-yl, 1-methyl-1H-pyrazol-3-yl, -CON(R¹⁶)CH₂R¹⁸, -COC(R²⁷)₃, 1-methylpiperidin-4-yl, 1-methyl-1,2,3,6-tetrahydropyridin-4-yl, 1,5-dimethyl-1H-pyrazol-4-yl, (cyclopentylmethyl)sulfanyl, 1-(pyridin-4-yl)ethoxy, 5H,6H,7H-cyclopenta[b]pyridin-7-yloxy, 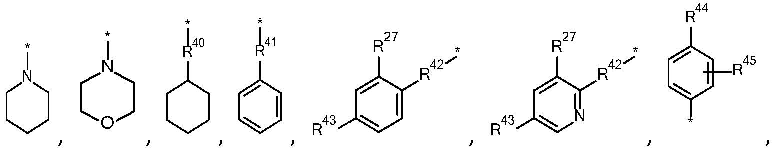 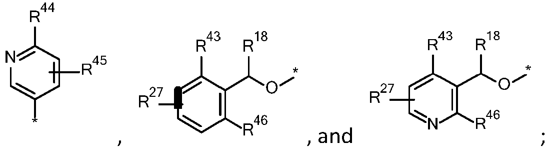
- R¹²: is selected from the group consisting of -H, -CH₃, -F, and -CF₃;
- R¹⁶: is selected from the group consisting of -H, -CH₃, and -CH₂CH₃;
- R¹⁸: is selected from the group consisting of -H and -CH₃;
- R²⁷: is selected from the group consisting of -H and -F;
- R³⁷: is selected from the group consisting of -H and -OH;
- R³⁸: is selected from the group consisting of -H, -CH₃, and -F;
- R³⁹: is selected from the group consisting of -CH₃, -NH₂, -NHCH₃, -N(CH₃)₂, morpholin-4-yl, and 4-methylpiperazin-1-yl;
- R⁴⁰: is selected from the group consisting of -NHCO-, -CHCH₃S-, -CH₂SO-, -CH₂SO₂-, and **-N-CH₂-*, **-O-CH₂-*, and **-S-CH₂-*;
- R⁴¹: is selected from the group consisting of -CH₂S-, -SO-, -NHCO-, -CHCH₃S-, -O-, and -S-;
- R⁴²: is selected from the group consisting of -CH₂O-, -CHCH₃O-, and -CHCH₃NHCO-;
- R⁴³: is selected from the group consisting of -H and -Cl;
- R⁴⁴: is selected from the group consisting of -H, -CH₃, -CCH₃O, -SCH₃O, -SO₂R⁶⁴, and dimethoxyphosphoryl;
- R⁴⁵: is selected from the group consisting of -H, -CH₃, -F, -OCH₃, and -SCH₃O₂;
- R⁴⁶: is selected from the group consisting of -H, -F, and -Cl;
- R⁶⁴: is selected from the group consisting of -CH₃, -NH₂, -NHCH₃, cyclopropyl, and -N(CH₃)₂; or a stereoisomer, a tautomer, an N-oxide, a hydrate, a solvate, or a salt thereof, or a mixture of same.

The compounds of general formula (I) of the present invention can be converted to any salt, preferably pharmaceutically acceptable salts, as described herein, by any method which is known to the person skilled in the art. Similarly, any salt of a compound of general formula (I) of the present invention can be converted into the free compound, by any method which is known to the person skilled in the art.

Compounds of general formula (I) of the present invention demonstrate a valuable pharmacological spectrum of action, which could not have been predicted. Compounds of the present invention have surprisingly been found to effectively inhibit MAP4K1 and it is possible therefore that said compounds be used for the treatment or prophylaxis of diseases, preferably cancer or conditions with dysregulated immune responses or other disorders associated with aberrant MAP4K1 signaling, in humans and animals.

Disorders and conditions particularly suitable for treatment with an MAP4K1 inhibitor of the present invention are liquid and solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancers include, but are not limited to, triple negative breast cancer, invasive ductal carcinoma, invasive lobular carcinoma, ductal carcinoma *in situ,* and lobular carcinoma *in situ.* Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalmic glioma, cerebellar and cerebral astrocytoma, glioblastoma, medulloblastoma, ependymoma, as well as neuroectodermal and pineal tumour.

Tumours of the male reproductive organs include, but are not limited to, prostate and testicular cancer.

Tumours of the female reproductive organs include, but are not limited to, endometrial, cervical, ovarian, vaginal, and vulvar cancer, as well as sarcoma of the uterus.

Examples of ovarian cancer include, but are not limited to serous tumour, endometrioid tumour, mucinous cystadenocarcinoma, granulosa cell tumour, Sertoli-Leydig cell tumour and arrhenoblastoma.

Examples of cervical cancer include, but are not limited to squamous cell carcinoma, adenocarcinoma, adenosquamous carcinoma, small cell carcinoma, neuroendocrine tumour, glassy cell carcinoma and villoglandular adenocarcinoma.

Tumours of the digestive tract include, but are not limited to, anal, colon, colorectal, esophageal, gallbladder, gastric, pancreatic, rectal, small-intestine, and salivary gland cancers.

Examples of esophageal cancer include, but are not limited to esophageal cell carcinomas and adenocarcinomas, as well as squamous cell carcinomas, leiomyosarcoma, malignant melanoma, rhabdomyosarcoma and lymphoma.

Examples of gastric cancer include, but are not limited to intestinal type and diffuse type gastric adenocarcinoma.

Examples of pancreatic cancer include, but are not limited to ductal adenocarcinoma, adenosquamous carcinomas and pancreatic endocrine tumours.

Tumours of the urinary tract include, but are not limited to, bladder, penile, kidney, renal pelvis, ureter, urethral and human papillary renal cancers.

Examples of kidney cancer include, but are not limited to renal cell carcinoma, urothelial cell carcinoma, juxtaglomerular cell tumour (reninoma), angiomyolipoma, renal oncocytoma, Bellini duct carcinoma, clear-cell sarcoma of the kidney, mesoblastic nephroma and Wilms' tumour.

Examples of bladder cancer include, but are not limited to transitional cell carcinoma, squamous cell carcinoma, adenocarcinoma, sarcoma and small cell carcinoma.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, squamous cell cancer of the head and neck, laryngeal, hypopharyngeal, nasopharyngeal, oropharyngeal cancer, salivary gland cancer, lip and oral cavity cancer and squamous cell.

Lymphomas include, but are not limited to, AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

The term "treating" or "treatment" as stated throughout this document is used conventionally, for example the management or care of a subject for the purpose of combating, alleviating, reducing, relieving, improving the condition of a disease or disorder, such as a carcinoma.

The compounds of the present invention can be used in particular in therapy and prevention, i.e. prophylaxis, of tumour growth and metastases, especially in solid tumours of all indications and stages with or without pre-treatment of the tumour growth.

Generally, the use of chemotherapeutic agents and/or anti-cancer agents in combination with a compound or pharmaceutical composition of the present invention will serve to:
1. yield better efficacy in reducing the growth of a tumour or even eliminate the tumour as compared to administration of either agent alone,
2. provide for the administration of lesser amounts of the administered chemotherapeutic agents,
3. provide for a chemotherapeutic treatment that is well tolerated in the patient with fewer deleterious pharmacological complications than observed with single agent chemotherapies and certain other combined therapies,
4. provide for treating a broader spectrum of different cancer types in mammals, especially humans,
5. provide for a higher response rate among treated patients,
6. provide for a longer survival time among treated patients compared to standard chemotherapy treatments,
7. provide a longer time for tumour progression, and/or
8. yield efficacy and tolerability results at least as good as those of the agents used alone, compared to known instances where other cancer agent combinations produce antagonistic effects.

In addition, the compounds of general formula (I) of the present invention can also be used in combination with radiotherapy and/or surgical intervention.

In a further embodiment of the present invention, the compounds of general formula (I) of the present invention are used in combination with radiation: i.e. radiation treatment sensitizes cancers to anti-tumor immune responses by induction of tumor cell death and subsequent presentation of tumor neoantigens to tumor-reactive Tcells. As MAP4K1 inhibition is enhancing the antigen specific activation of T cells, the overall effect results in a much stronger cancer cell attack as compared to irradiation treatment alone.

Thus, the present invention also provides a method of killing a tumor, wherein conventional radiation therapy is employed previous to administering one or more of the compounds of the present invention.

The compounds of the present invention can be administered as the sole pharmaceutical agent or in combination with one or more other pharmaceutically active ingredients where the combination causes no unacceptable adverse effects. The present invention also covers such pharmaceutical combinations. For example, the compounds of the present invention can be combined with: ¹³¹I-chTNT, abarelix, abiraterone, aclarubicin, adalimumab, ado-trastuzumab emtansine, afatinib, aflibercept, aldesleukin, alectinib, alemtuzumab, alendronic acid, alitretinoin, alpharain, altretamine, amifostine, aminoglutethimide, hexyl aminolevulinate, amrubicin, amsacrine, anastrozole, ancestim, anethole dithiolethione, anetumab ravtansine, angiotensin II, antithrombin III, aprepitant, arcitumomab, arglabin, arsenic trioxide, asparaginase, atezolizumab, axitinib, azacitidine, basiliximab, belotecan, bendamustine, besilesomab, belinostat, bevacizumab, bexarotene, bicalutamide, bisantrene, bleomycin, blinatumomab, bortezomib, buserelin, bosutinib, brentuximab vedotin, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, cemiplimab, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, darolutamide, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, eculizumab, edrecolomab, elliptinium acetate, elotuzumab, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (¹²³I), iomeprol, ipilimumab, irinotecan, Itraconazole, ixabepilone, ixazomib, lanreotide, lansoprazole, lapatinib, larotrectinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, necitumumab, nedaplatin, nelarabine, neridronic acid, netupitant/palonosetron, nivolumab, pentetreotide, nilotinib, nilutamide, nimorazole, nimotuzumab, nimustine, nintedanib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, rogaratinib, rolapitant, romidepsin, romiplostim, romurtide, roniciclib , samarium (¹⁵³Sm) lexidronam, sargramostim, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (^{99m}Tc) nofetumomab merpentan, ^{99m}Tc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tisagenlecleucel, tislelizumab, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

The compounds of the invention can further be combined with other reagents targeting the immune system, such as immune checkpoint inhibitors, e.g. aPD-1/-L1 axis antagonists. PD-1, along with its ligands PD-L1 and PD-L2, function as negative regulators of T cell activation. MAP4K1 suppresses immune cell function. PD-L1 is overexpressed in many cancers and overexpression of PD-1 often occurs concomitantly in tumor infiltrating T cells. Thus results in attenuation of T cell activation and evasion of immune surveillance, which contributes to impaired antitumor immune responses. (Keir M E et al. (2008) Annu. Rev. Immunol. 26:677).

In accordance with a further aspect, the present invention covers combinations comprising one or more of the compounds of general formula (I), as described *supra,* or stereoisomers, tautomers, N-oxides, hydrates, solvates, and salts thereof, particularly pharmaceutically acceptable salts thereof, or mixtures of same, and one or more immune checkpoint inhibitors.

In a further embodiment the immune checkpoint inhibitor is a aPD-1/-L1 axis antagonist.

A further use of the compounds of the invention is the combination with chimeric antigen receptor T cells (CAR-T cells) such as Axicabtagen-Ciloleucel or Tisagenlecleucel. The activity of CAR-T cells can be suppressed by the tumor micro environment (TME), which supposedly can be overcome by MAP4K1 inhibition.

In accordance with a further aspect, the present invention covers compounds of general formula (I), as described herein, or stereoisomers, tautomers, N-oxides, hydrates, solvates, and salts thereof, particularly pharmaceutically acceptable salts thereof, or mixtures of same, for use in the expansion of T cells including CAR-T cells, CAR-NKT cells or CAR-NK cells and tumor infiltrated lymphocytes ex-*vivo.*

Hence, the present invention also relates to the use of the compounds according to the invention for the expansion of T cells, including CAR-T cell, CAR-NKT cells or CAR-NK cells and tumor infiltrated lymphocytes, *ex-vivo.*

The present invention also comprises an *ex-vivo* method for the expansion of T cells, including CAR-T cells, CAR-NKT cells or CAR-NK cells and tumor infiltrated lymphocytes, contacting said T cells with compounds according to the invention.

In addition, the inventive compounds can also be used as a therapeutic in a variety of other disorders wherein MAP4K1 is involved such as, cardiovascular and lung diseases.

Accordingly, the compounds according to the invention are suitable for the treatment and/or prophylaxis in particular of cardiovascular, inflammatory and fibrotic disorders and of renal disorders, in particular of acute and chronic renal insufficiency, and also of acute and chronic renal failure.

Accordingly, the compounds according to the invention can be used in medicaments for the treatment and/or prophylaxis of cardiovascular, inflammatory and fibrotic disorders, renal disorders, in particular of acute and chronic renal insufficiency, and also of acute and chronic renal failure.

For the purpose of the present invention the term renal insufficiency comprises both acute and chronic manifestations of renal insufficiency, and also underlying or related renal disorders such as diabetic and non-diabetic nephropathies, hypertensive nephropathies, ischaemic renal disorders, renal hypoperfusion, intradialytic hypotension, obstructive uropathy, renal stenoses, glomerulopathies, glomerulonephritis (such as, for example, primary glomerulonephritides; minimal change glomerulonephritis (lipoidnephrosis); membranous glomerulonephritis; focal segmental glomerulosclerosis (FSGS); membrane-proliferative glomerulonephritis; crescentic glomerulonephritis; mesangioproliferative glomerulonephritis (IgA nephritis, Berger's disease); post-infectious glomerulonephritis; secondary glomerulonephritides: diabetes mellitus, lupus erythematosus, amyloidosis, Goodpasture syndrome, Wegener granulomatosis, Henoch-Schönlein purpura, microscopic polyangiitis, acute glomerulonephritis, pyelonephritis (for example as a result of: urolithiasis, benign prostate hyperplasia, diabetes, malformations, abuse of analgesics, Crohn's disease), glomerulosclerosis, arteriolonecrose of the kidney, tubulointerstitial diseases, nephropathic disorders such as primary and congenital or aquired renal disorder, Alport syndrome, nephritis, immunological kidney disorders such as kidney transplant rejection and immunocomplex-induced renal disorders, nephropathy induced by toxic substances, nephropathy induced by contrast agents, diabetic and non-diabetic nephropathy, renal cysts, nephrosclerosis, hypertensive nephrosclerosis and nephrotic syndrome which can be characterized diagnostically, for example by abnormally reduced creatinine and/or water excretion, abnormally elevated blood concentrations of urea, nitrogen, potassium and/or creatinine, altered activity of renal enzymes, for example glutamyl synthetase, altered urine osmolarity or urine volume, elevated microalbuminuria, macroalbuminuria, lesions on glomerulae and arterioles, tubular dilatation, hyperphosphataemia and/or the need for dialysis. The present invention also comprises the use of the compounds according to the invention for the treatment and/or prophylaxis of sequelae of renal insufficiency, for example pulmonary oedema, heart failure, uremia, anemia, electrolyte disturbances (for example hypercalemia, hyponatremia) and disturbances in bone and carbohydrate metabolism.

The present invention also comprises the use of the compounds according to the invention for the treatment and/or prevention of sequelae of renal insufficiency, for example pulmonary oedema, heart failure, uraemia, anaemia, electrolyte disturbances (for example hyperkalaemia, hyponatraemia) and disturbances in bone and carbohydrate metabolism.

The compounds according to the invention are further suitable for the treatment and/or prevention of polycystic kidney disease (PCKD) and of the syndrome of inappropriate ADH secretion (SIADH).

Furthermore, the compounds according to the invention are also suitable for the treatment and/or prophylaxis of metabolic syndrome, hypertension, resistant hypertension, acute and chronic heart failure, coronary heart disease, stable and unstable angina pectoris, peripheral and cardiac vascular disorders, arrhythmias, atrial and ventricular arrhythmias and impaired conduction, for example atrioventricular blocks degrees I-III (AB block I-III), supraventricular tachyarrhythmia, atrial fibrillation, atrial flutter, ventricular fibrillation, ventricular flutter, ventricular tachyarrhythmia, Torsade de pointes tachycardia, atrial and ventricular extrasystoles, AV-junctional extrasystoles, sick sinus syndrome, syncopes, AV-nodal re-entry tachycardia, Wolff-Parkinson-White syndrome, of acute coronary syndrome (ACS), autoimmune cardiac disorders (pericarditis, endocarditis, valvolitis, aortitis, cardiomyopathies), shock such as cardiogenic shock, septic shock and anaphylactic shock, aneurysms, boxer cardiomyopathy (premature ventricular contraction (PVC)), for treatment and/or prophylaxis of thromboembolic disorders and ischaemias such as myocardial ischaemia, myocardial infarction, stroke, cardiac hypertrophy, transient and ischaemic attacks, preeclampsia, inflammatory cardiovascular disorders, spasms of the coronary arteries and peripheral arteries, oedema formation, for example pulmonary oedema, cerebral oedema, renal oedema or oedema caused by heart failure, peripheral circulatory disturbances, reperfusion damage, arterial and venous thromboses, myocardial insufficiency, endothelial dysfunction, to prevent restenoses, for example after thrombolysis therapies, percutaneous transluminal angioplasties (PTA), transluminal coronary angioplasties (PTCA), heart transplants and bypass operations, and also micro- and macrovascular damage (vasculitis), increased levels of fibrinogen and of low-density lipoprotein (LDL) and increased concentrations of plasminogen activator inhibitor 1 (PAI-1), and also for treatment and/or prophylaxis of erectile dysfunction and female sexual dysfunction.

In addition, the compounds according to the invention are also suitable for treatment and/or prophylaxis of asthmatic disorders, pulmonary arterial hypertension (PAH) and other forms of pulmonary hypertension (PH) including left-heart disease, HIV, sickle cell anaemia, thromboembolisms (CTEPH), sarcoidosis, COPD or pulmonary fibrosis-associated pulmonary hypertension, chronic-obstructive pulmonary disease (COPD), acute respiratory distress syndrome (ARDS), acute lung injury (ALI), alpha-1-antitrypsin deficiency (AATD), pulmonary fibrosis, pulmonary emphysema (for example pulmonary emphysema induced by cigarette smoke) and cystic fibrosis (CF).

The compounds described in the present invention are also active compounds for control of central nervous system disorders characterized by disturbances of the NO/cGMP system. They are suitable in particular for improving perception, concentration, learning or memory after cognitive impairments like those occurring in particular in association with situations/diseases/syndromes such as mild cognitive impairment, age-associated learning and memory impairments, age-associated memory losses, vascular dementia, craniocerebral trauma, stroke, dementia occurring after strokes (post stroke dementia), post-traumatic craniocerebral trauma, general concentration impairments, concentration impairments in children with learning and memory problems, Alzheimer's disease, Lewy body dementia, dementia with degeneration of the frontal lobes including Pick's syndrome, Parkinson's disease, progressive dementia with corticobasal degeneration, amyolateral sclerosis (ALS), Huntington's disease, demyelinization, multiple sclerosis, thalamic degeneration, Creutzfeld-Jacob dementia, HIV dementia, schizophrenia with dementia or Korsakoff's psychosis. They are also suitable for treatment and/or prophylaxis of central nervous system disorders such as states of anxiety, tension and depression, CNS-related sexual dysfunctions and sleep disturbances, and for controlling pathological disturbances of the intake of food, stimulants and addictive substances.

The compounds according to the invention are furthermore also suitable for controlling cerebral blood flow and thus represent effective agents for controlling migraines. They are also suitable for the prophylaxis and control of sequelae of cerebral infarction (cerebral apoplexy) such as stroke, cerebral ischaemia and craniocerebral trauma. The compounds according to the invention can likewise be used for controlling states of pain and tinnitus. In addition, the compounds according to the invention have anti-inflammatory action and can therefore be used as anti-inflammatory agents for treatment and/or prophylaxis of sepsis (SIRS), multiple organ failure (MODS, MOF), inflammatory disorders of the kidney, chronic intestinal inflammations (IBD, Crohn's disease, UC), pancreatitis, peritonitis, rheumatoid disorders, inflammatory skin disorders and inflammatory eye disorders.

Furthermore, the compounds according to the invention can also be used for treatment and/or prophylaxis of autoimmune diseases.

The compounds according to the invention are also suitable for treatment and/or prophylaxis of fibrotic disorders of the internal organs, for example the lung, the heart, the kidney, the bone marrow and in particular the liver, and also dermatological fibroses and fibrotic eye disorders. In the context of the present invention, the term fibrotic disorders includes in particular the following terms: hepatic fibrosis, cirrhosis of the liver, pulmonary fibrosis, endomyocardial fibrosis, nephropathy, glomerulonephritis, interstitial renal fibrosis, fibrotic damage resulting from diabetes, bone marrow fibrosis and similar fibrotic disorders, scleroderma, morphea, keloids, hypertrophic scarring (also following surgical procedures), naevi, diabetic retinopathy, proliferative vitro retinopathy and disorders of the connective tissue (for example sarcoidosis).

The compounds according to the invention are also suitable for controlling postoperative scarring, for example as a result of glaucoma operations.

The compounds according to the invention can also be used cosmetically for ageing and keratinized skin.

Moreover, the compounds according to the invention are suitable for treatment and/or prophylaxis of hepatitis, neoplasms, osteoporosis, glaucoma and gastroparesis.

The present invention further provides the use of the compounds according to the invention for treatment and/or prophylaxis of disorders, especially the disorders mentioned above.

The present invention further provides the use of the compounds according to the invention for the treatment and/or prophylaxis of chronic renal disorders, acute and chronic renal insufficiency, diabetic, inflammatory or hypertensive nephropaties, fibrotic disorders, cardiac insufficiency, angina pectoris, hypertension, pulmonary hypertension, ischemias, vascular disorders, thromboembolic disorders, arteriosclerosis, sickle cell anemia, erectile dysfunction, benign prostate hyperplasia, dysuria associated with benign prostate hyperplasia, Huntington, dementia, Alzheimer and Creutzfeld-Jakob.

The present invention further provides a method for treatment and/or prophylaxis of disorders, in particular the disorders mentioned above, using an effective amount of at least one of the compounds according to the invention.

The present invention further provides a method for the treatment and/or prophylaxis of chronic renal disorders, acute and chronic renal insufficiency, diabetic, inflammatory or hypertensive nephropathies, fibrotic disorders, cardiac insufficiency, angina pectoris, hypertension, pulmonary hypertension, ischemias, vascular disorders, thromboembolic disorders, arteriosclerosis, sickle cell anemia, erectile dysfunction, benign prostate hyperplasia, dysuria associated with benign prostate hyperplasia, Huntington, dementia, Alzheimer and Creutzfeld-Jakob.

In another embodiment, the inventive compounds can also be used to treat or to prevent uterine fibroids (uterine leiomyoma or uterine myoma) in women.

Compounds of the present invention can be utilized to inhibit, block, reduce or decrease MAP4K1 activation by exogenous and/or endogenous ligands for the reduction of tumour growth and the modulation of dysregulated immune responses e.g. to block immunosuppression and increase immune cell activation and infiltration in the context of cancer and cancer immunotherapy; This method comprises administering to a mammal in need thereof, including a human, an amount of a compound of this invention, or a pharmaceutically acceptable salt, stereoisomer, polymorph, metabolite, hydrate, solvate or ester thereof; which is effective to treat the disorder.

The present disclosure also provides methods of treating a variety of other disorders wherein MAP4K1 is involved such as, but not limited to, disorders with dysregulated immune responses, inflammation, vaccination for infection & cancer, viral infections, obesity and diet-induced obesity, adiposity, metabolic disorders, hepatic steatosis and uterine fibroids. These disorders have been well characterized in humans, but also exist with a similar etiology in other mammals, and can be treated by administering pharmaceutical compositions of the present invention.

The term "treating" or "treatment" as used in the present text is used conventionally, e.g., the management or care of a subject for the purpose of combating, alleviating, reducing, relieving, improving the condition of a disease or disorder, such as liquid and solid tumours. In accordance with a further aspect, the present invention covers compounds of general formula (I), as described *supra,* or stereoisomers, tautomers, N-oxides, hydrates, solvates, and salts thereof, particularly pharmaceutically acceptable salts thereof, or mixtures of same, for use in the treatment or prophylaxis of diseases, in particular cancer or conditions with dysregulated immune responses or other disorders associated with aberrant MAP4K1 signaling.

The pharmaceutical activity of the compounds according to the invention can be explained by their activity as MAP4K1 inhibitors.

In accordance with a further aspect, the present invention covers the use of compounds of general formula (I), as described *supra,* or stereoisomers, tautomers, N-oxides, hydrates, solvates, and salts thereof, particularly pharmaceutically acceptable salts thereof, or mixtures of same, for the treatment or prophylaxis of diseases, in particular cancer or conditions with dysregulated immune responses or other disorders associated with aberrant MAP4K1 signaling, particularly liquid and solid tumours.

In accordance with a further aspect, the present invention covers the compounds of general formula (I), as described *supra,* or stereoisomers, tautomers, N-oxides, hydrates, solvates, and salts thereof, particularly pharmaceutically acceptable salts thereof, or mixtures of same, for the use of treatment or prophylaxis of diseases, in particular cancer or conditions with dysregulated immune responses or other disorders associated with aberrant MAP4K1 signaling, particularly liquid and solid tumours.

In accordance with a further aspect, the present invention covers the use of compounds of general formula (I), as described *supra,* or stereoisomers, tautomers, N-oxides, hydrates, solvates, and salts thereof, particularly pharmaceutically acceptable salts thereof, or mixtures of same, in a method of treatment or prophylaxis of diseases, in particular cancer or conditions with dysregulated immune responses or other disorders associated with aberrant MAP4K1 signaling, particularly liquid and solid tumours.

In accordance with a further aspect, the present invention covers use of a compound of general formula (I), as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular cancer or conditions with dysregulated immune responses or other disorders associated with aberrant MAP4K1 signaling, particularly liquid and solid tumours.

In accordance with a further aspect, the present invention covers a method of treatment or prophylaxis of diseases, in particular cancer or conditions with dysregulated immune responses or other disorders associated with aberrant MAP4K1 signaling, particularly liquid and solid tumours, using an effective amount of a compound of general formula (I), as described *supra,* or stereoisomers, tautomers, N-oxides, hydrates, solvates, and salts thereof, particularly pharmaceutically acceptable salts thereof, or mixtures of same.

In accordance with a further aspect, the present invention covers pharmaceutical compositions, in particular a medicament, comprising a compound of general formula (I), as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.

The present invention furthermore covers pharmaceutical compositions, in particular medicaments, which comprise at least one compound according to the invention, conventionally together with one or more pharmaceutically suitable excipients, and to their use for the above mentioned purposes.

It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette^{®}), sorbitan fatty acid esters (such as, for example, Span^{®}), polyoxyethylene sorbitan fatty acid esters (such as, for example, Tween^{®}), polyoxyethylene fatty acid glycerides (such as, for example, Cremophor^{®}), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®})),
- capsule materials (for example gelatine, hydroxypropylmethylcellulose),
- synthetic polymers (for example polylactides, polyglycolides, polyacrylates, polymethacrylates (such as, for example, Eudragit^{®}), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprise at least one compound according to the invention, conventionally together with one or more pharmaceutically suitable excipient(s), and to their use according to the present invention. In accordance with another aspect, the present invention covers pharmaceutical combinations, in particular medicaments, comprising at least one compound of general formula (I) of the present invention and at least one or more further active ingredients, in particular for the treatment and/or prophylaxis of cancer or conditions with dysregulated immune responses or other disorders associated with aberrant MAP4K1 signalinggeneric name disorders, particularly liquid and solid tumours.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

Based upon standard laboratory techniques known to evaluate compounds useful for the treatment of cancer or conditions with dysregulated immune responses or other disorders associated with aberrant MAP4K1 signaling, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### EXPERIMENTAL SECTION

NMR peak forms are stated as they appear in the spectra, possible higher order effects have not been considered. The multiplicities are stated according to the signal form which appears in the spectrum, NMR-spectroscopic effects of a higher order were not taken into consideration. Multiplicity of the NMR signals: s = singlet, d = doublet, t = triplet, q = quartet, quin = quintet, br = broad signal, m = multiplet. NMR signals: shift in [ppm]. Combinations of multiplicity could be e.g. dd = doublet from doublet.

In some cases not all H atoms are found as a signal in the NMR because the signal could overlays with a solvent signal or it is a very braod signal dependent on the NMR solvent used.

The ¹H-NMR data of selected examples / intermediates are listed in the form of ¹H-NMR peaklists. For each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity2), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of title compounds (also the subject of the invention), and/or peaks of impurities. The peaks of stereoisomers, and/or peaks of impurities are typically displayed with a lower intensity compared to the peaks of the title compounds (e.g., with a purity of >90%). Such stereoisomers and/or impurities may be typical for the particular manufacturing process, and therefore their peaks may help to identify the reproduction of our manufacturing process on the basis of "by-product fingerprints". An expert who calculates the peaks of the title compounds by known methods (MestReC, ACD simulation, or by use of empirically evaluated expectation values), can isolate the peaks of title compounds as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. Research Disclosure Database Number 605005, 2014, 01 Aug 2014, or http://www.researchdisclosure.com/searching-disclosures). In the peak picking routine, as described in the Research Disclosure Database Number 605005, the parameter "MinimumHeight" can be adjusted between 1% and 4%. Depending on the chemical structure and/or depending on the concentration of the measured compound it may be reasonable to set the parameter "MinimumHeight" <1%.

Chemical names were generated using the ACD/Name software from ACD/Labs. In some cases generally accepted names of commercially available reagents were used in place of ACD/Name generated names.

Table 1 lists the abbreviations used in this paragraph and in the Examples section as far as they are not explained within the text body. Other abbreviations have their meanings customary *per se* to the skilled person.

The various aspects of the invention described in this application are illustrated by the following examples which are not meant to limit the invention in any way. The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil^{®} or KP-NH^{®} in combination with a Biotage autopurifier system (SP4^{®} or Isolera Four^{®}) and eluents such as gradients of hexane/ethyl acetate, DCM/methanol, or DCM/ethanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### EXPERIMENTAL SECTION - GENERAL SYNTHESIS

The following paragraphs outline a variety of synthetic approaches suitable to prepare compounds of the general formula (I), and intermediates useful for their synthesis.

In addition to the routes described below, also other routes may be used to synthesize the title compounds, in accordance with common general knowledge of a person skilled in the art of organic synthesis. The order of transformations exemplified in the following schemes is therefore not intended to be limiting, and suitable synthesis steps from various schemes can be combined to form additional synthesis sequences. In addition, interconversion of any of the substituents, in particular R¹, R² and R⁴, which are as defined in formula (I) supra, can be achieved before and/or after the exemplified transformations. These modifications can be, for example, the introduction of protective groups, cleavage of protective groups, reduction or oxidation of functional groups, halogenation, metallation, metal catalysed coupling reactions, exemplified by but not limited to e.g. Buchwald, Suzuki, Sonogashira and Ullmann coupling, ester saponifications, amide coupling reactions, and/or substitution or other reactions known to a person skilled in the art. These transformations include those which introduce a functionality allowing for further interconversion of substituents. Appropriate protective groups and their introduction and cleavage are well-known to a person skilled in the art (see for example T.W. Greene and P.G.M. Wuts in Protective Groups in Organic Synthesis, 4th edition, Wiley 2006**).**

Further, it is possible that two or more successive steps may be performed without work-up being performed between said steps, e.g. a "one-pot" reaction, as it is well-known to a person skilled in the art.

The syntheses of the derivatives according to the present invention are preferably carried out according to the general synthetic sequence, shown in schemes 1-3.

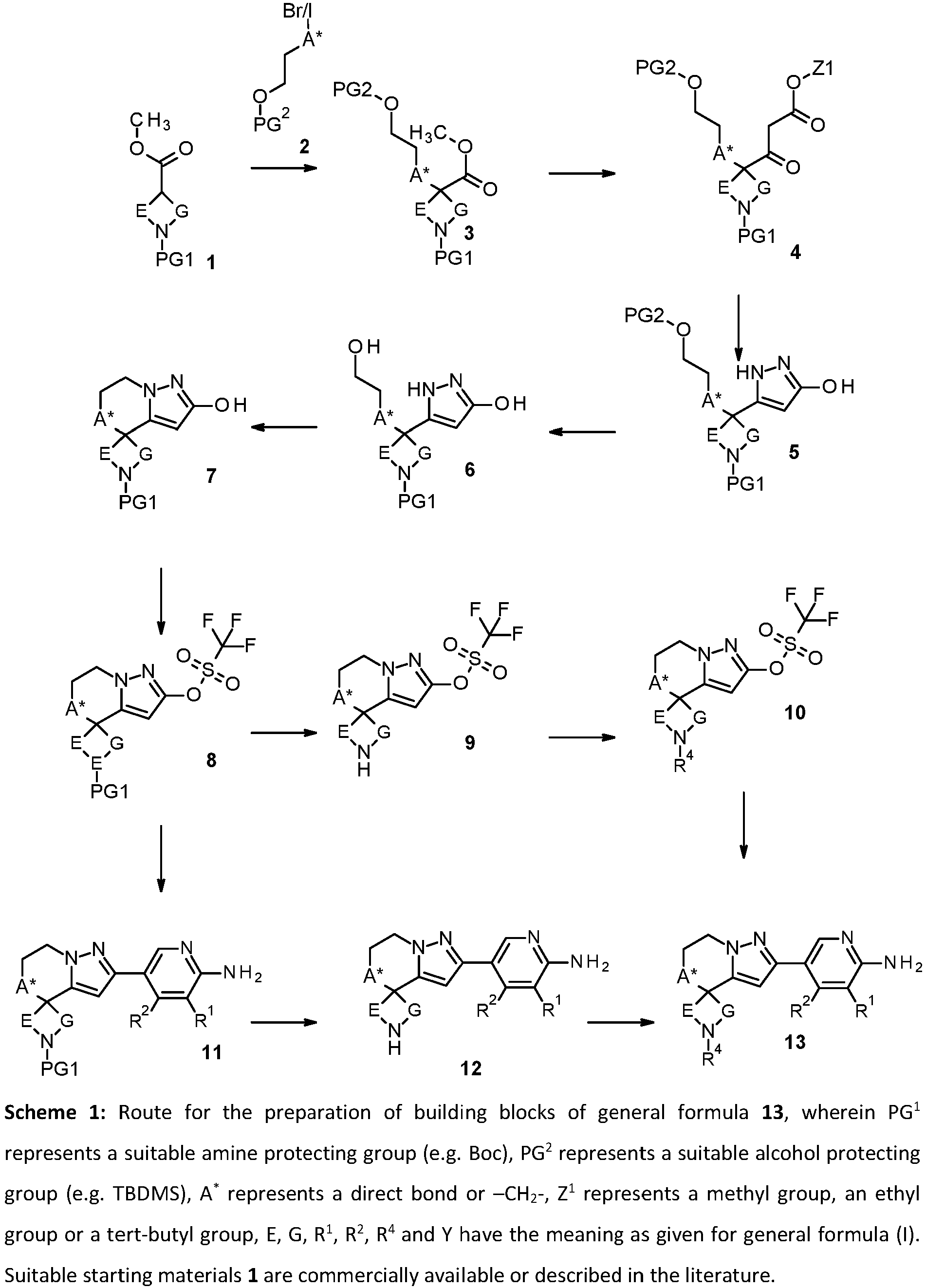

### Step 1 → 3 (Scheme 1)

### Alkylation

In the first step (scheme 1), ester derivative **1** can be alkylated using an alkylbromide or alkyliodide of formula **2** to give the desired product **3.**

For example ester **1** can be alkylated using (2-bromoethoxy)(tert-butyl)dimethylsilane 2 in an organic solvent such as THF in the presence of a base such as LiHMDS or LDA.

### Step 3 → 4 (Scheme 1)

### beta-Keto ester formation

Methylester **3** is reacted with a methyl acetate or tert-butyl acetate to give beta-keto esters of the general formula **4.** Typically the reaction is performed in the presence of a base like LiHMDS or LDA in an organic solvent like THF at a temperature range between -78°C and room temperature.

### Step 4 → 5 (Scheme 1)

### Pyrazol formation

beta-Keto esters of formula **4** can be converted with hydrazine to the corresponding pyrazole derivatives of formula **5.** Typically the reaction is performed in an organic solvent like ethanol at a temperature between -20°C and the boiling point of the selected solvent.

### Step 5 → 6 (Scheme 1)

### Deprotection of PG²

The protecting group PG² of pyrazoles of formula **5** can be cleaved to give an alcohol of formula **6.** The cleavage of suitable alcohol protecting groups is well-known to the person skilled in the art (see for example P.G.M. Wuts and T.W. Greene in "Protective Groups in Organic Synthesis", 4th edition, Wiley 2006). For example, when PG2 in compounds of formula **5** is TBDMS, cleavage can be achieved using e.g. HCl in an organic solvent such as methanol or TBAF in an organic solvent such as THF.

### Step 6 → 7 (Scheme 1)

### Ring closure

Alcohols of formula **6** can be converted to spiro compounds of formula **7** by ring closing reactions. For example, alcohols of formula **6** can be reacted with mesylchloride and DIEA in an organic solvent like DCM to give the corresponding mesylate, which is then reacted to give spiro compounds of formula **7,** e.g. in the presence of a base like NaOH using a solvent mixture like methanol / water. Moreover, ring closure can be achieved using Mitsunobu conditions known to the skilled person. For example, DEAD (diethyl azodicarboxylate) or DIAD (diisopropyl azodicarboxylate), triphenylphosphine in an organic solvent such as for example THF can be used.

### Step 7 → 8 (Scheme 1)

### Triflate formation

Spiro compounds of formula **7** can be converted to triflates of formula **8.** Typically the reaction is performed using Tf₂O in the presence of a base like DIEA in an organic solvent like DCM at a temperature range between -78°C and room temperature. Alternatively the reaction is performed using N,N-bis-(trifluormethansulfonyl)-aniline in the presence of a base like DIEA in an organic solvent like THF at a temperature range between room temperature and the boiling point of the selected solvent.

### Step 8 → 9 (Scheme 1)

### Deprotection of PG¹

The protecting group PG¹ of spiro compounds of formula **8** can be cleaved to give amines of formula 9. The cleavage of suitable amine protecting groups is well-known to the person skilled in the art (see for example P.G.M. Wuts and T.W. Greene in "Protective Groups in Organic Synthesis", 4th edition, Wiley 2006). For example, when PG¹ in compounds of formula **8** is BOC, cleavage can be achieved using e.g. TFA in an organic solvent such as DCM.

### Step 9 → 10 (Scheme 1)

### Amine decoration

Amines of formula **9** can be functionalized with a broad variety of substituents to give compounds of formula **10.** For examples, secondary amines of formula **9** can be reacted to give for example tertiary amines, amides, ureas, carbamates or sulphonamides of formula **10.** All these transformations are known to the skilled person.

### Step 10 → 13 (Scheme 1)

### C-C cross coupling reaction

Compounds of general formula **10** can be reacted with a boronic acid derivative 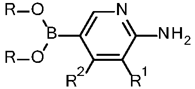 to give a compound of formula **13.** The boronic acid derivative may be a boronic acid (R = -H) or an ester of the boronic acid, e.g. its isopropyl ester (R = -CH(CH₃)₂), preferably an ester derived from pinacol in which the boronic acid intermediate forms a 4,4,5,5-tetramethyl - 1,3,2-dioxaborolane (R-R = -C(CH₃)₂-C(CH₃)₂-). The coupling reaction is catalyzed by palladium catalysts, e.g. by Pd(0) catalysts like tetrakis(triphenylphosphine)palladium(0) [Pd(PPh₃)₄], tris(dibenzylideneacetone)di-palladium(0) [Pd₂(dba)₃], or by Pd(ll) catalysts like dichlorobis(triphenylphosphine)-palladium (II) [Pd(PPh₃)₂Cl₂], palladium (II) acetate and triphenylphosphine, [1,1'-bis(diphenylphosphino)ferrocene] palladium dichloride or by second generation XPhos Pd (Chloro(2-dicyclohexylphosphino-2',4',6'-triisopropyl-1,1'-biphenyl)[2-(2'-amino-1,1'-biphenyl)]palladium(II), X-Phos aminobiphenyl palladium chloride precatalyst). The reaction is preferably carried out in a mixture of a solvent like 1,2-dimethoxyethane, dioxane, DMF, DME, THF, or isopropanol with water and in the presence of a base like potassium carbonate, sodium bicarbonate or potassium phosphate. (review: D.G. Hall, Boronic Acids, 2005 WILEY-VCH Verlag GmbH & Co. KGaA, Weinheim, ISBN 3-527-30991-8 and references cited therein). The reaction is performed at temperatures ranging from room temperature to the boiling point of the solvent. Further on, the reaction can be performed at temperatures above the boiling point under pressure. The reaction is preferably completed after 1 to 36 hours.

### Step 8 → 11 (Scheme 1)

### C-C cross coupling reaction

Compounds of general formula **8** can be reacted with a boronic acid derivative 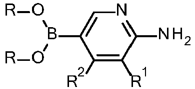 to give a compound of formula **11.** The boronic acid derivative may be a boronic acid (R = -H) or an ester of the boronic acid, e.g. its isopropyl ester (R = -CH(CH₃)₂), preferably an ester derived from pinacol in which the boronic acid intermediate forms a 4,4,5,5-tetramethyl - 1,3,2-dioxaborolane (R-R = -C(CH₃)₂-C(CH₃)₂-). The coupling reaction is catalyzed by palladium catalysts, e.g. by Pd(0) catalysts like tetrakis(triphenylphosphine)palladium(0) [Pd(PPh₃)₄], tris(dibenzylideneacetone)di-palladium(0) [Pd₂(dba)₃], or by Pd(ll) catalysts like dichlorobis(triphenylphosphine)-palladium (II) [Pd(PPh₃)₂Cl₂], palladium (II) acetate and triphenylphosphine, [1,1'-bis(diphenylphosphino)ferrocene] palladium dichloride or by second generation XPhos Pd (Chloro(2-dicyclohexylphosphino-2',4',6'-triisopropyl-1,1'-biphenyl)[2-(2'-amino-1,1'-biphenyl)]palladium(II), X-Phos aminobiphenyl palladium chloride precatalyst). The reaction is preferably carried out in a mixture of a solvent like 1,2-dimethoxyethane, dioxane, DMF, DME, THF, or isopropanol with water and in the presence of a base like potassium carbonate, sodium bicarbonate or potassium phosphate. (review: D.G. Hall, Boronic Acids, 2005 WILEY-VCH Verlag GmbH & Co. KGaA, Weinheim, ISBN 3-527-30991-8 and references cited therein). The reaction is performed at temperatures ranging from room temperature to the boiling point of the solvent. Further on, the reaction can be performed at temperatures above the boiling point under pressure. The reaction is preferably completed after 1 to 36 hours.

### Step 11 → 12 (Scheme 1)

### Deprotection of PG¹

The protecting group PG¹ of spiro compounds of formula **11** can be cleaved to give amines of formula **12.** The cleavage of suitable amine protecting groups is well-known to the person skilled in the art (see for example P.G.M. Wuts and T.W. Greene in "Protective Groups in Organic Synthesis", 4th edition, Wiley 2006). For example, when PG¹ in compounds of formula **12** is BOC, cleavage can be achieved using e.g. TFA in an organic solvent such as DCM.

### Step 12 → 13 (Scheme 1)

### Amine decoration

Amines of formula **12** can be functionalized with a broad variety of substituents to give compounds of formula **13.** For examples, secondary amines of formula **13** can be reacted to give tertiary amines, amides, ureas, carbamates or sulphonamides of formula **13.** All these tranformations are known to the skilled person.

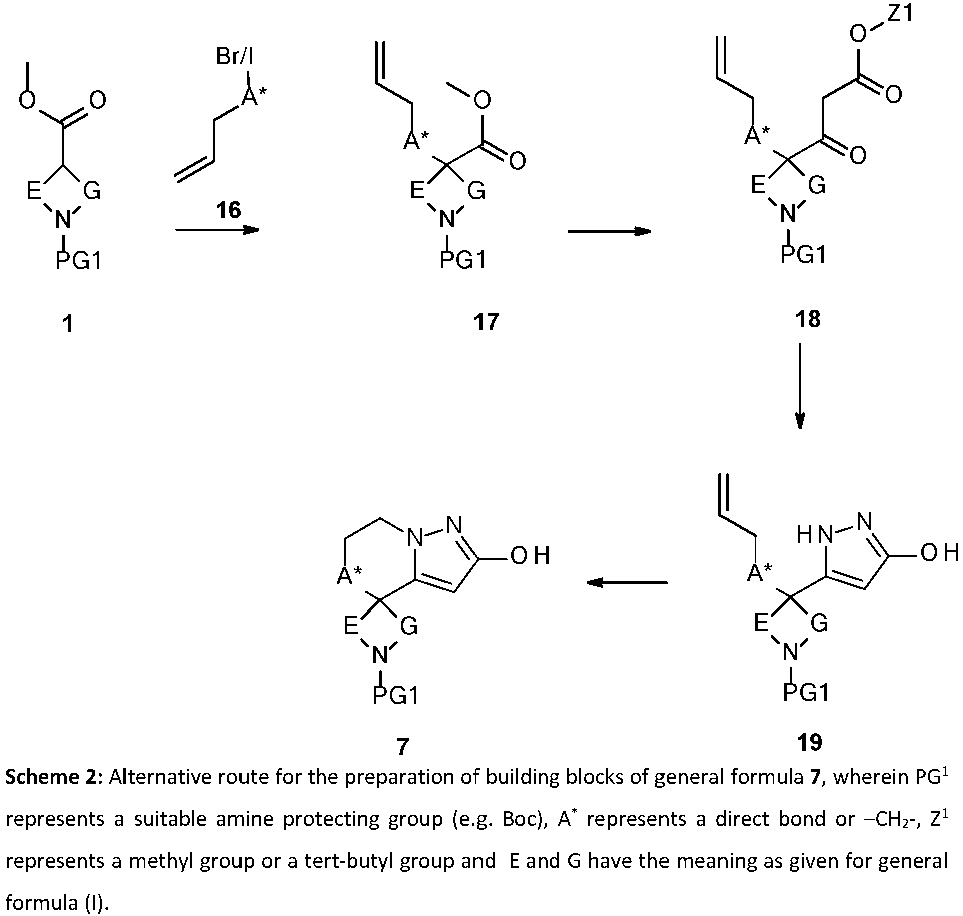

### Step 1 → 17 (Scheme 2)

### Alkylation

Compounds of the general formula 1 can be converted to compounds of the general formula **17** by alkylation. Typically the reaction is performed with an alkylating agent such as for example **16**, a base such as LiHMDS or LDA in an organic solvent such as THF.

### Step 17 → 18 (Scheme 2)

### beta-Keto ester formation

Methylester **17** is reacted with a methyl acetate or tert-butyl acetate to give beta-keto esters of the general formula **18.** Typically the reaction is performed in the presence of a base like LiHMDS or LDA in an organic solvent like THF at a temperature range between -78°C and room temperature.

### Step 18 → 19 (Scheme 2)

### Pyrazol formation

beta-Keto esters of formula **18** can be converted with hydrazine to the corresponding pyrazole derivatives of formula **19.** Typically the reaction is performed in an organic solvent like ethanol at a temperature between -20°C and the boiling point of the selected solvent.

### Step 19 → 7 (Scheme 2)

### Cyclization

Compounds of the general formula **19** can be converted to compounds of formula **7.** Reaction conditions are known to the skilled person. Typically the olefin part is converted to a diol followed by cleavage of the diol forming two aldehydes. The so called Lemieux-Johnson reaction is performed with osmium tetroxide and sodium metaperiodate in a mixture of water and an organic solvent for example dioxane at 5-40 °C, and preferably at room temperature. The osmium tetroxide can be generated in situ from an osmium(VI) salt (e.g. K₂OsO₄ dihydrate) easily oxidizable to osmium(VIII) by sodium metaperiodate. In another preferred procedure using the Jin-protocol 2,6-lutidine was added to the reaction mixture.

The aldehyde is reduced to the alcohol with e.g. sodium boronhydride in an organic solvent, for example an alcohol (e.g. methanol) at temperature starting from -10 °C to 10 °C (preferably at 0°C) and finally increasing to elevated temperature e.g. room temperature to 50°C.

Then, the alcohols can be converted to spiro compounds of formula **7** by ring closing reactions. For example, the alcohols can be reacted with mesylchloride and DIEA in an organic solvent like DCM to give the corresponding mesylate, which is then reacted to give spiro compounds of formula **7,** e.g. in the presence of a base like NaOH using a solvent mixture like methanol / water. Moreover, ring closure can be achieved using Mitsunobu conditions known to the skilled person. For example, DEAD (diethyl azodicarboxylate) or DIAD (diisopropyl azodicarboxylate), triphenylphosphine in an organic solvent such as for example THF can be used.

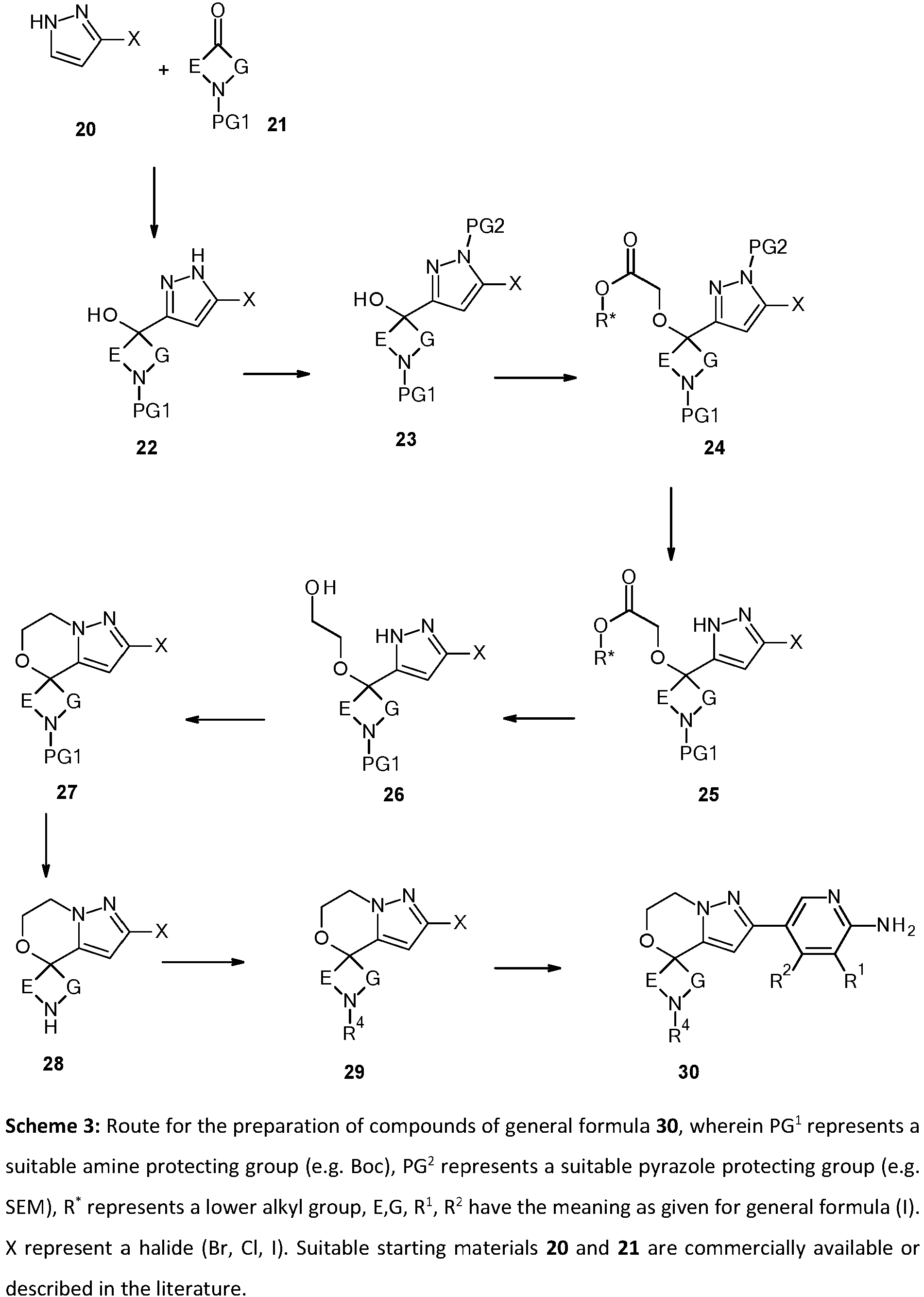

### Step 20 + 21 → 22 (Scheme 3)

### Pyrazole addition to the carbonyl group

Ketones of the general formula **21** and pyrazoles of the general formula **20** can be converted to compounds of the general formula **22.** The conversion is known to the person skilled in the art. For example, the conversion can be carried out in analogy to a literature procedure described in Tetrahedron, 1983, 39, 2023-2029.

### Step 22 → 23 (Scheme 3)

### Protection with PG²

Compounds of the general formula **22** can be converted to compounds of the general formula **23** using a suitable protecting group to protect the pyrazole NH. Protecting groups for pyrazoles are known to the skilled person (see for example P.G.M. Wuts and T.W. Greene in "Protective Groups in Organic Synthesis", 4th edition, Wiley 2006). For example, when PG2 in compounds of formula **23** is SEM, then 2-(trimethylsilyl)ethoxymethylchloride, a base such as sodium hydride in an organic solvent such as THF can be used.

### Step 23 → 24 (Scheme 3)

### Alkylation of the alcohol

Alcohols of the general formula **23** can be converted to compounds of the general formula **24** in an alkylation reaction known to the skilled person. For example, bromo ethyl acetate, a base, such as sodium hydride in an organic solvent such as dioxane at elevated temperature can be used.

### Step 24 → 25 (Scheme 3)

### Deprotection of PG²

The protecting group PG² of pyrazole compounds of general formula **24** can be cleaved to give compounds of formula **25.** The cleavage of suitable pyrazol protecting groups is well-known to the person skilled in the art (see for example P.G.M. Wuts and T.W. Greene in "Protective Groups in Organic Synthesis", 4th edition, Wiley 2006). For example, when PG2 in compounds of formula **24** is SEM, cleavage can be achieved using e.g. TFA or TBAF in an organic solvent such as DCM.

### Step 25 → 26 (Scheme 3)

### Reduction of the ester

Esters of the general formula **25** (R^{*} = lower alkyl group) can be converted to the corresponding alcohols of the general formula **26** with hydride reducing agents known to the skilled person. For example lithium borohydride in an organic solvent, such as THF can be used.

### Step 26 → 27 (Scheme 3)

### Mitsunobu reaction

Compounds of the general formula **26** can be converted to the corresponding morpholine derivatives of the general formula **27** using Mitsunobu conditions known to the skilled person. For example, DEAD (diethyl azodicarboxylate) or DIAD (diisopropyl azodicarboxylate), triphenylphosphine in an organic solvent such as for example THF can be used.

### Step 27 → 28 (Scheme 3)

### Deprotection of PG¹

The protecting group PG¹ of spiro compounds of formula **27** can be cleaved to give amines of formula **28.** The cleavage of suitable amine protecting groups is well-known to the person skilled in the art (see for example P.G.M. Wuts and T.W. Greene in "Protective Groups in Organic Synthesis", 4th edition, Wiley 2006). For example, when PG2 in compounds of formula **27** is BOC, cleavage can be achieved using e.g. TFA in an organic solvent such as DCM.

### Step 28 → 29(Scheme 3)

### Amine decoration

Amines of formula **28** can be functionalized with a broad variety of substituents to give compounds of formula **29.** For examples, secondary amines of formula **29** can be reacted to give tertiary amines, amides, ureas, carbamates or sulphonamides of formula **29.** All these transformations are known to the skilled person.

### Step 29 → 30(Scheme 3)

### C-C cross coupling reaction

Halogen compounds of general formula **29** can be reacted with a boronic acid derivative 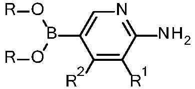 to give a compound of formula **30.** The boronic acid derivative may be a boronic acid (R = -H) or an ester of the boronic acid, e.g. its isopropyl ester (R = -CH(CH₃)₂), preferably an ester derived from pinacol in which the boronic acid intermediate forms a 4,4,5,5-tetramethyl - 1,3,2-dioxaborolane (R-R = -C(CH₃)₂-C(CH₃)₂-). The coupling reaction is catalyzed by palladium catalysts, e.g. by Pd(0) catalysts like tetrakis(triphenylphosphine)palladium(0) [Pd(PPh₃)₄], tris(dibenzylideneacetone)di-palladium(0) [Pd₂(dba)₃], or by Pd(ll) catalysts like dichlorobis(triphenylphosphine)-palladium (II) [Pd(PPh₃)₂Cl₂], palladium (II) acetate and triphenylphosphine, [1,1'-bis(diphenylphosphino)ferrocene] palladium dichloride or by second generation XPhos Pd (Chloro(2-dicyclohexylphosphino-2',4',6'-triisopropyl-1,1'-biphenyl)[2-(2'-amino-1,1'-biphenyl)]palladium(II), X-Phos aminobiphenyl palladium chloride precatalyst). The reaction is preferably carried out in a mixture of a solvent like 1,2-dimethoxyethane, dioxane, DMF, DME, THF, or isopropanol with water and in the presence of a base like potassium carbonate, sodium bicarbonate or potassium phosphate. (review: D.G. Hall, Boronic Acids, 2005 WILEY-VCH Verlag GmbH & Co. KGaA, Weinheim, ISBN 3-527-30991-8 and references cited therein). The reaction is performed at temperatures ranging from room temperature to the boiling point of the solvent. Further on, the reaction can be performed at temperatures above the boiling point under pressure. The reaction is preferably completed after 1 to 36 hours.

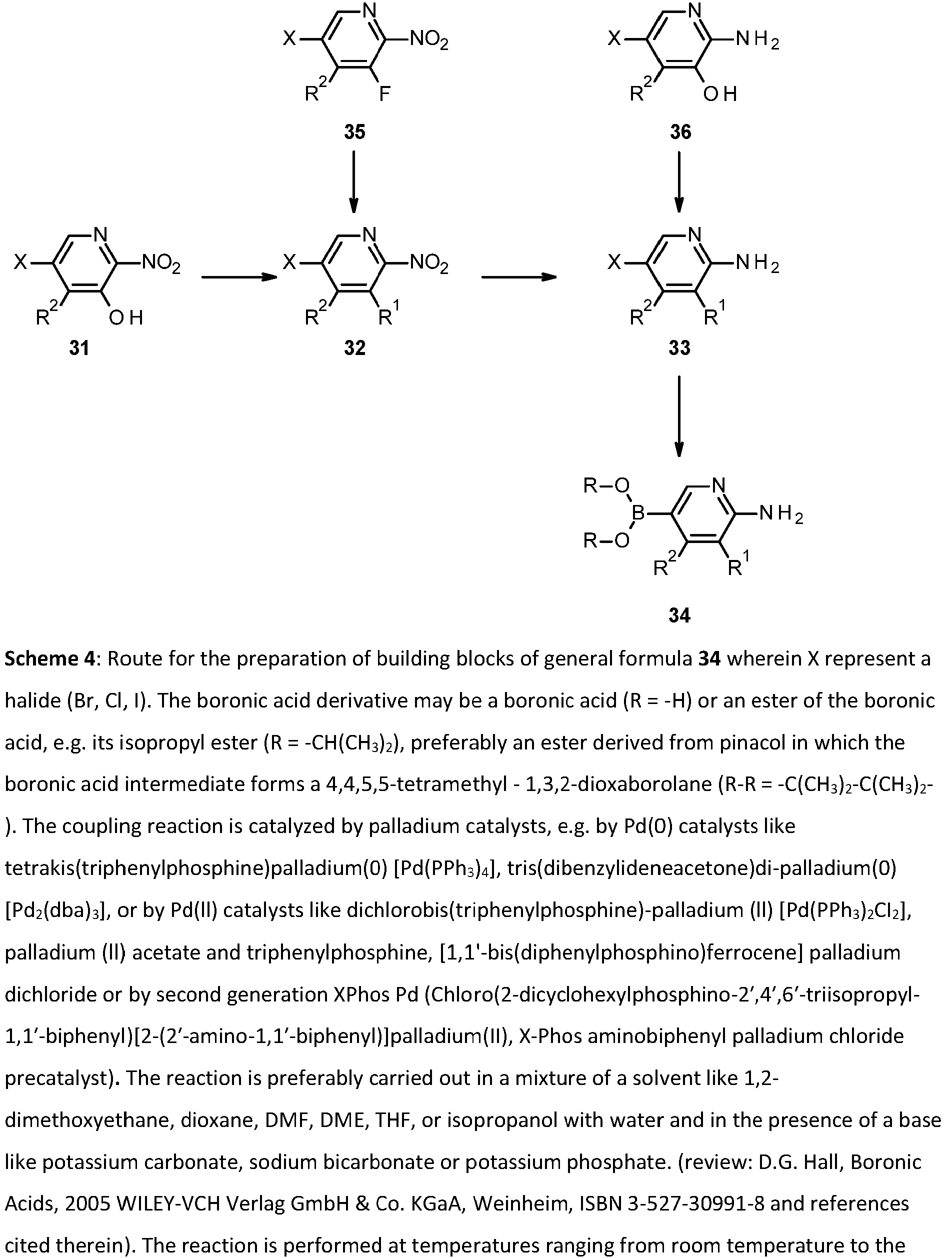

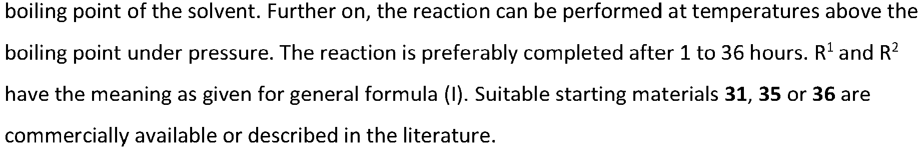

### Step 31 → 32 (Scheme 4)

### Hydroxy group transformation

Nitropyridine **31** can be converted to nitropyridine **32** using Mitsunobu conditions known to the skilled person. For example, DEAD (diethyl azodicarboxylate) or DIAD (diisopropyl azodicarboxylate), triphenylphosphine or tributylphosphine in an organic solvent such as for example THF, dioxane, DCM, acetonitrile or toluene can be used.

Alternatively, pyridine **31** can be alkylated using alkyl halides or sulfonates (tosylates, mesylates, triflates or nonaflates) and a base such as NaH, Cs₂CO3, K₂CO₃, NaOH in organic solvents such as THF or DMF.

### Step 35 → 32 (Scheme 4)

### Nucleophilic aromatic substitution

Fluoropyridine **35** can be converted to nitropyridine **32** using a nucleophilic aromatic substitution. For example, by using a nucleophile in the presence of a base such as NaH, Cs2CO3, K2CO3, NaOH in an organic solvent like THF, dioxane, DMA or acetonitrile.

### Step 32 → 33 (Scheme 4)

### Reduction

Pyridine **32** can be converted to aminopyridine **33** by reduction of the nitro group. For example, by using a metal such as Fe or Zn and acetic acid or hydrochloric acid or ammonium chloride. Alternatively, this reduction can be performed with hydrogen using a catalyst such as Pt/C, Pd/C or Raney Nickel.

### Step 36 → 33 (Scheme 4)

### Hydroxy group alkylation

Aminopyridine **36** can be converted to aminopyridine **33** using Mitsunobu conditions known to the skilled person. For example, DEAD (diethyl azodicarboxylate) or DIAD (diisopropyl azodicarboxylate), triphenylphosphine in an organic solvent such as for example THF can be used.

Alternatively, aminopyridine **36** can be alkylated using alkyl halides or alkylmesylates and a base such as sodium hydride, cesium carbonate, potassium carbonate or sodium hydroxide in organic solvents such as THF, DMF or ethanol.

### Step 33 → 34 (Scheme 4)

### Borylation

Pyridine halide **33** can be converted to the corresponding boronic acid or ester **34** using Miyaura borylation conditions know from the person skilled in the art. For example, using bis-pinacol borane, a palladium catalyst such as Pd(dppf)Cl2, and a base for example potassium acetate in organic solvent such as dioxane or DMSO.

The steps for the synthesis sequence giving rise to spiro compounds of formula 7, **15** or **30** may be also interchanged using similar reaction conditions for each step as described above.

### Abbreviations

**Table 1: Abbreviations**

| | |
|---|---|
| | |
| | |
| | |
| | |
| | |
| | |
| | |
| | |
| | |
| | |
| | |
| | |
| | |
| | |
| | |
| | |

| **Abbreviation** | **Meaning** |
|---|---|
| CDI | carbonyldiimidazole |
| DCM | dichloromethane |
| DIAD | diisopropyl azodicarboxylate |
| DMF | N,N-dimethylformamide |
| DMSO | dimethylsulfoxide |
| h | hour(s) |
| HATU | (1-[bis(dimethylamino)methylene]-1H-1,2,3-triazolo[4,5-b]pyridinium 3-oxide hexafluorophosphate |
| LDA | Lithium diisopropylamide |
| LiHMDS | Lithium bis(trimethylsilyl)amide |
| MeOH | methanol |
| min | minute(s) |
| MTBE | methyl tert-butyl ether |
| NaOH | sodium hydroxide |
| Pd(dppf)Cl₂ | [1,1'-bis(diphenylphosphino)ferrocene]dichloropalladium(II) |
| rt | room temperature |
| Rₜ | retention time |
| TBAF | tetrabutylammonium fluoride |
| TFA | trifluoro acetic acid |
| THF | tetrahydrofuran |
| TLC | thin layer chromatography |
| XPhos Pd G2 | (2'-aminobiphenyl-2-yl)(chloro)palladium - dicyclohexyl[2',4',6'-tri(propan-2-yl)biphenyl-3-yl]phosphane (1:1) |

### EXPERIMENTAL SECTION - METHODS:

### Analytical LC-MS methods:

### Method 1:

Instrument: Waters Acquity UPLCMS SingleQuad; Column: Acquity UPLC BEH C18 1.7 µm, 50x2.1mm; eluent A: water + 0.2 vol % aq. ammonia (32%), eluent B: acetonitrile; gradient: 0-1.6 min 1-99% B, 1.6-2.0 min 99% B; flow 0.8 mL/min; temperature: 60 °C; DAD scan: 210-400 nm.

### Method 2:

Instrument: Waters Acquity UPLCMS SingleQuad; Column: Acquity UPLC BEH C18 1.7 µm, 50x2.1mm; eluent A: water + 0.1 vol % formic acid (99%), eluent B: acetonitrile; gradient: 0-1.6 min 1-99% B, 1.6-2.0 min 99% B; flow 0.8 mL/min; temperature: 60 °C; DAD scan: 210-400 nm. It would be similar in the retention time

### Method 5:

Instrument: Thermo Scientific FT-MS UHPLC+: Thermo Scientific UltiMate 3000; column: Waters, HSST3, 2.1 x 75 mm, C18 1.8 µm; eluent A: water + 0.01% formic acid; eluent B: acetonitrile + 0.01% formic acid; gradient: 0.0 min 10% B → 2.5 min 95% B → 3.5 min 95% B; temperature: 50°C; flow: 0.90 mL/min; UV-detection: 210 nm/ optimum integration path 210-300 nm.

### Method 6:

Instrument: Waters Acquity UPLCMS SingleQuad; Column: Acquity UPLC BEH C18 1.7 µm, 50x2.1mm; eluent A: water + 0.1 vol % formic acid (99%), eluent B: acetonitrile; gradient: 0-1.7 min 1-45% B, 1.7-1.72 min 45-99% B, 1.72-2.0 min 99% B; flow 0.8 mL/min; temperature: 60 °C; DAD scan: 210-400 nm.

### Method 7:

Instrument: Waters Acquity UPLCMS SingleQuad; Column: Acquity UPLC BEH C18 1.7µ, 50x2.1mm; eluent A: water + 0.1 vol % formic acid; eluent B: acetonitrile; gradient: 0-1.6 min 1-99% B, 1.6-2.0 min 99% B; flow: 0.8 mL/min; temperature: 60°C; DAD scan: 210-400 nm.

### Method 8:

Instrument: Waters Acquity UPLCMS SingleQuad; Column: Acquity UPLC BEH C18 1.7 µm, 50x2.1mm; Eluent A: water + 0.2 vol-% ammonia (32%), Eluent B: acetonitrile; gradient: 0-1.6 min 1-99% B, 2.6-3.0 min 99% B; flow: 0.8 mL/min; temperature: 60°C; DAD scan: 210-400nm.

### Intermediate 1

### 5-(4,4,5,5-tetramethyl-1,3,2-dioxaborolan-2-yl)-3-(trifluoromethyl)pyridin-2-amine

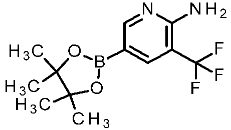

5-Bromo-3-(trifluoromethyl)pyridin-2-amine (2.00 g, 8.30 mmol) and 4,4,4',4',5,5,5',5'-octamethyl-2,2'-bi-1,3,2-dioxaborolane (2.53 g, 9.96 mmol) were solubilised in dioxane (43 mL), potassium acetate (2.44 g, 24.9 mmol) and Pd(dppf)Cl₂ (607 mg, 830 µmol) were added and the mixture was stirred overnight at 100°C. The mixture was diluted with dichloromethane, filtered and evaporated. The residue was purified by flash chromatography to give 2.70 g of the target compound.

1H-NMR (400 MHz, DMSO-d6) δ [ppm]: 1.052 (0.88), 1.065 (16.00), 1.069 (1.16), 1.145 (0.90), 1.154 (8.40), 1.163 (1.61), 1.176 (0.70), 1.269 (11.09), 1.290 (1.05), 3.332 (0.74), 3.936 (2.59), 7.799 (0.47), 7.803 (0.48), 7.939 (0.72), 8.375 (0.46), 8.377 (0.46).

### Intermediate 2

### 2-amino-5-(4,4,5,5-tetramethyl-1,3,2-dioxaborolan-2-yl)pyridine-3-carbonitrile

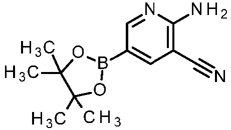

2-Amino-5-bromopyridine-3-carbonitrile (1.40 g, 7.07 mmol), 4,4,4',4',5,5,5',5'-octamethyl-2,2'-bi-1,3,2-dioxaborolane (1.97 g, 7.78 mmol), Pd(dppf)Cl₂ (289 mg, 353 µmol) and potassium acetate (2.08 g, 21.2 mmol) were stirred in degassed dioxane (35 mL) at 100°C overnight. The reaction mixture was filtered and used without further work up or purification in the next step.

¹H NMR (400 MHz, DMSO-*d*₆) δ ppm 1.27 (s, 12 H) 7.33 (s, 2 H) 7.92 (d, 1 H) 8.37 (d, 1 H).

### Intermediate 3

### ethyl 2-(3-chloropyridin-4-yl)-2-methylpropanoate

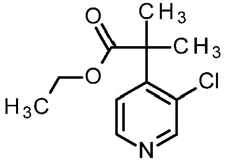

3-Chloropyridine-4-carbonitrile (500 mg, 3.61 mmol) in THF (12 mL) was cooled to -78°C under nitrogen atmosphere then ethyl 2-methylpropanoate (419 mg, 3.61 mmol) was added and potassium bis(trimethylsilyl)amide (7.9 mL, 0.50 M in toluene, 4.0 mmol) was added dropwise, the mixture was stirred 10 minutes before removing the cooling bath and stirred 1h at rt. The reaction was quenched with saturated ammonium chloride solution and extracted three times with dichloromethane. The combined organic phases were dried over sodium sulfate and concentrated. The residue was purified by flash chromatography to afford 300 mg (36 % yield) of the title compound.

### Intermediate 4

### 2-(3-chloropyridin-4-yl)-2-methylpropanoic acid

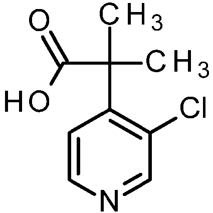

To a stirred solution of ethyl 2-(3-chloropyridin-4-yl)-2-methylpropanoate (300 mg, 1.32 mmol) in methanol (8.5 mL) and THF (26 mL) was added lithium hydroxide (5.3 mL, 1.0 M, 5.3 mmol) and stirred overnight at rt. Lithium hydroxide (2.8 mL, 1.0 M, 2.8 mmol) was added to the mixture and stirred overnight at rt, then the mixture was heated for 4h at 40°C and then overnight at 50°C. Potassium hydroxide (1.3 mL, 1.0 M, 1.3 mmol) was added and stirred 4h at 50°C and then 72h at rt. Potassium hydroxide (1.3 mL, 1.0 M, 1.3 mmol) was added again and stirred 2 days at 50°C. The reaction mixture was divided in two portions and stirred in the microwave at 100°C for 1h. The reaction mixture was diluted with water, evaporated from organic solvents and extracted three times with dichloromethane. The aqueous phase was acidified to pH 2-3 using hydrochloric acid (9.5 mL, 1M) and extracted with dichloromethane (+10% ethanol). The combined organic phases were concentrated to give 170 mg (64 % yield) of the title compound.

### Intermediate 5

### 3-chloro-4-(2-isocyanatopropan-2-yl)pyridine

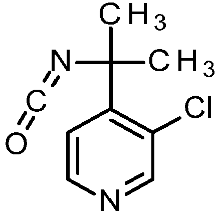

To a stirred solution of 2-(3-chloropyridin-4-yl)-2-methylpropanoic acid (170 mg, 852 µmol) in dioxane (2.8 mL) and triethylamine (150 µL) was added diphenyl phosphorazidate (240 µL, 1.1 mmol) and stirred at rt overnight. The reaction mixture was used in the next step without purification.

### Intermediate 6

### (rac)-1-tert-butyl-3-methyl-3-(2-{[tert-butyl(dimethyl)silyl]oxy}ethyl)pyrrolidine-1,3-dicarboxylate

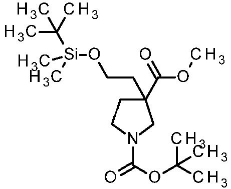

1-Tert-butyl 3-methyl pyrrolidine-1,3-dicarboxylate (9.59 g, 41.83 mmol) was dissolved in THF (96 mL) under argon atmosphere. It was cooled down to -78°C and LiHMDS (83.7 mL, 83.7 mmol, 1 M in THF) was added. It was stirred 1h at -78°C and then (2-bromoethoxy)(tert-butyl)dimethylsilane (20.01 g, 83.7 mmol) in THF (53 mL) were added dropwise. It was stirred overnight and allowed to reach rt. On an ice bath saturated ammonium chloride solution (160 mL) was added and stirred for 1h. It was extracte with ethyl acetate / THF, dried through a silicone filter and concentrated. The residue was purified by flash chromatography yielding 9.19 g (57%) of the title compound.

¹H NMR (400 MHz, DMSO-*d*₆) δ ppm: 0.01 (s, 6H), 0.85 (s, 9H), 1.38 (s, 9H), 1.75 - 1.97 (m, 3H), 2.14 - 2.25 (m, 1H), 3.02 - 3.17 (m, 2H), 3.24 - 3.30 (m, 1H), 3.46 - 3.64 (m, 5H), 3.67 - 3.82 (m, 1H).

### Intermediate 7

### (rac)-tert-butyl-3-(3-tert-butoxy-3-oxopropanoyl)-3-(2-{[tert-butyl(dimethyl)silyl]oxy}ethyl)pyrrolidine-1-carboxylate

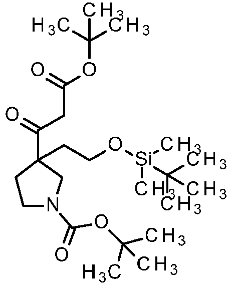

LiHMDS (6.5 mL, 1.0 M in THF, 6.5 mmol) was diluted in THF (10 mL) under argon and cooled down to -78°C then tert-butyl acetate (870 µL, 6.5 mmol) was added dropwise and stirred for 1h at -78°C. Then (rac)-1-tert-butyl-3-methyl-3-(2-{[tert-butyl(dimethyl)silyl]oxy}ethyl)pyrrolidine-1,3-dicarboxylate (500 mg, 1.29 mmol) dissolved in THF (10 mL) was added dropwise. The reaction mixture was allowed to warm up to rt and stirred overnight at rt. The mixture was poured into saturated ammonium chloride solution and extracted twice with ethyl acetate, dried over sodium sulfate and concentrated to afford 500 mg (82 % yield) of the title compound.

¹H-NMR (400 MHz, DMSO-d6) δ [ppm]: -0.029 (0.99), -0.011 (0.72), -0.003 (16.00), 0.009 (14.92), 0.017 (0.48), 0.057 (1.56), 0.069 (1.62), 0.079 (0.51), 0.168 (0.89), 0.840 (7.12), 1.345 (0.44), 1.381 (10.57), 1.443 (0.60), 1.987 (0.53), 2.518 (0.98), 2.522 (0.66), 3.593 (0.43), 3.655 (0.76).

### Intermediate 8

### (rac)-tert-butyl-3-(2-{[tert-butyl(dimethyl)silyl]oxy}ethyl)-3-(5-hydroxy-1H-pyrazol-3-yl)pyrrolidine-1-carboxylate

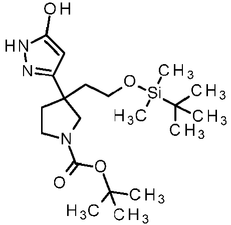

(Rac)-tert-butyl-3-(3-tert-butoxy-3-oxopropanoyl)-3-(2-{[tert-butyl(dimethyl)silyl]oxy}ethyl)pyrrolidine-1-carboxylate (8.60 g, 18.2 mmol) and hydrazine hydrate (3.5 mL, 73 mmol) were dissolved in ethanol (180 mL) under argon and stirred for 6h at 80°C. The reaction mixture was concentrated to give 10.5 g of the title compound.

¹H-NMR (400 MHz, DMSO-d6) δ [ppm]: -0.185 (0.47), -0.149 (0.52), -0.035 (13.08), -0.030 (1.92), 0.685 (0.59), 0.831 (11.97), 0.843 (16.00), 1.039 (6.72), 1.056 (14.62), 1.074 (6.60), 1.111 (10.59), 1.228 (0.50), 1.234 (0.50), 1.272 (0.68), 1.356 (3.62), 1.367 (9.35), 1.387 (13.19), 1.853 (0.46), 1.886 (0.43), 2.081 (11.55), 3.195 (0.87), 3.243 (0.41), 3.256 (0.46), 3.269 (0.46), 3.281 (0.46), 3.415 (2.96), 3.432 (8.65), 3.449 (6.98), 3.467 (2.59), 3.692 (0.46), 3.719 (0.43), 5.203 (2.88), 5.230 (1.05), 5.253 (0.66).

### Intermediate 9

### (rac)-tert-butyl-3-(2-hydroxyethyl)-3-(3-hydroxy-1H-pyrazol-5-yl)pyrrolidine-1-carboxylate

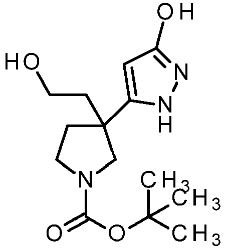

### (rac)-tert-butyl-3-(2-{[tert-butyl(dimethyl)silyl]oxy}ethyl)-3-(5-hydroxy-1H-pyrazol-3-yl)pyrrolidine-1-carboxylate

(243 mg, 590 µmol) was solubilised in methanol (1.1 mL), hydrochloride (440 µL, 4.0 M in dioxane, 1.8 mmol) was added and the mixture was stirred for 2h at rt under argon. Potassium carbonate (490 mg, 3.54 mmol) and di-tert-butyl dicarbonate (200 µL, 890 µmol) were added and the mixture was stirred for 1h at rt under argon. The mixture was filtered and evaporated to give the title compound.

### Intermediate 10

### (rac)-tert-butyl-2'-hydroxy-5',6'-dihydrospiro[pyrrolidine-3,4'-pyrrolo[1,2-b]pyrazole]-1-carboxylate

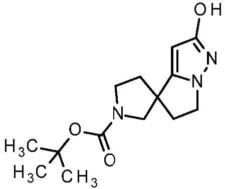

(Rac)-tert-butyl-3-(2-hydroxyethyl)-3-(3-hydroxy-1H-pyrazol-5-yl)pyrrolidine-1-carboxylate (5.24 g, 17.6 mmol) and triphenylphosphine (11.09 g, 42.3 mmol) were solubilised in THF (82 mL) under argon, DIAD (8.0 mL) was added dropwise and the mixture was stirred for 4h at rt. The mixture was evaporated and purified by flash chromatography to give 2.83 g (95 % purity, 55 % yield) of the target compound.

¹H-NMR (400 MHz, DMSO-d6) δ [ppm]: 1.055 (0.45), 1.391 (16.00), 1.418 (13.84), 1.936 (0.55), 1.950 (0.82), 1.964 (0.84), 1.982 (0.88), 2.008 (0.64), 2.325 (0.40), 2.329 (0.55), 2.334 (0.43), 2.347 (1.09), 2.363 (1.82), 2.381 (1.13), 2.521 (1.64), 2.525 (1.12), 2.671 (0.43), 3.365 (0.83), 3.426 (0.74), 3.434 (0.63), 3.441 (0.65), 3.454 (0.50), 3.913 (1.22), 3.918 (1.44), 3.931 (2.38), 3.935 (2.82), 3.947 (1.19), 3.952 (1.45), 5.208 (10.10), 9.584 (1.93).

### Intermediate 11

### (rac)-tert-butyl-2'-[(trifluoromethanesulfonyl)oxy]-5',6'-dihydrospiro[pyrrolidine-3,4'-pyrrolo[1,2-b]pyrazole]-1-carboxylate

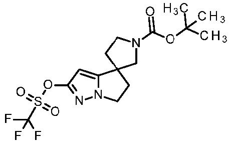

(Rac)-tert-butyl-2'-hydroxy-5',6'-dihydrospiro[pyrrolidine-3,4'-pyrrolo[1,2-b]pyrazole]-1-carboxylate (3.30 g, 11.8 mmol, 22% purity) was dissolved in THF (100 mL) under argon and treated with N,N-diisopropylethylamine (10 mL, 59 mmol) then N,N-bis(trifluoromethanesulfonyl)aniline (6.33 g, 17.7 mmol) was added and stirred at 60°C for 1h. The reaction mixture was concentrated and the residue was purified by flash chromatography to afford 600 mg (56 % yield) of the title compound.

1H-NMR (400 MHz, DMSO-d6) δ [ppm]: 0.000 (12.78), 1.156 (1.57), 1.173 (5.35), 1.188 (5.10), 1.192 (2.37), 1.231 (0.58), 1.235 (0.48), 1.248 (0.83), 1.265 (0.42), 1.394 (16.00), 1.422 (12.77), 1.989 (4.21), 2.032 (0.58), 2.048 (0.79), 2.060 (0.85), 2.077 (1.07), 2.097 (0.81), 2.108 (0.44), 2.121 (0.86), 2.471 (1.67), 2.520 (1.72), 2.525 (1.11), 3.371 (0.69), 3.379 (0.88), 3.397 (0.97), 3.428 (4.90), 3.455 (0.50), 3.462 (0.81), 3.474 (0.88), 3.482 (1.12), 3.489 (0.76), 3.494 (0.94), 3.501 (0.68), 3.509 (0.66), 3.521 (0.49), 4.020 (0.97), 4.037 (0.96), 4.191 (0.42), 4.201 (1.17), 4.212 (1.51), 4.217 (1.63), 4.220 (1.58), 4.229 (1.66), 4.232 (1.87), 4.237 (1.32), 4.248 (1.33), 4.258 (0.51), 4.768 (0.45), 6.255 (2.91), 8.883 (0.91).

### Intermediate 12

### (rac)-tert-butyl-3-[3-bromo-1-(2-hydroxyethyl)-1H-pyrazol-5-yl]-3-hydroxypyrrolidine-1-carboxylate

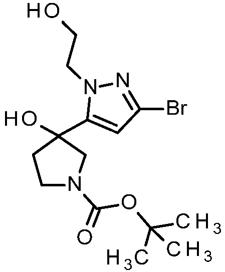

2-(3-Bromo-1H-pyrazol-1-yl)ethan-1-ol (1000 mg, 5.23 mmol) was solubilised in THF (25 mL), cooled to -78°C and LDA (5.2 mL, 2.0 M in THF/heptane/ethylbenzene, 10 mmol) was added dropwise. The mixture was allowed to warm up to -20°C and stirred at -20°C for 45 min. The mixture was cooled to -78°C, tert-butyl 3-oxopyrrolidine-1-carboxylate (808 mg, 4.36 mmol) solubilised in THF (10 mL) was added dropwise and the mixture was stirred for 1h at -78°C. The mixture was allowed to warm up to rt overnight. The mixture was quenched with saturated sodium bicarbonate solution, extracted with ethyl acetate, washed with brine, dried and concentrated. The residue was purified by flash chromatography to give 622 mg (38 % yield) of the target compound.

¹H-NMR (400 MHz, DMSO-d6) δ [ppm]: 1.398 (13.16), 1.408 (16.00), 2.173 (0.49), 2.196 (0.95), 2.219 (0.73), 2.518 (1.49), 2.523 (0.95), 3.375 (1.23), 3.388 (1.14), 3.396 (1.15), 3.409 (0.63), 3.424 (0.69), 3.454 (0.83), 3.481 (0.44), 3.509 (0.74), 3.574 (0.63), 3.623 (0.63), 3.651 (0.48), 3.728 (0.61), 3.742 (1.78), 3.757 (1.87), 3.771 (0.70), 4.288 (1.23), 4.303 (1.39), 4.320 (0.55), 4.944 (0.81), 4.953 (0.98), 4.966 (0.41), 5.759 (0.96), 5.871 (1.43), 5.885 (1.12), 6.315 (4.60).

### Intermediate 13

### (rac)-tert-butyl-2-bromo-6,7-dihydrospiro[pyrazolo[5,1-c][1,4]oxazine-4,3'-pyrrolidine]-1'-carboxylate

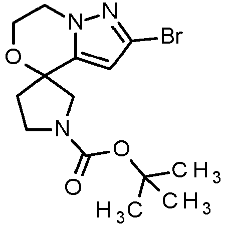

(Rac)-tert-butyl-3-[3-bromo-1-(2-hydroxyethyl)-1H-pyrazol-5-yl]-3-hydroxypyrrolidine-1-carboxylate (100 mg, 266 µmol) and triphenylphosphine (97.6 mg, 372 µmol) were solubilised in THF (1.5 mL), DIAD (68 µL) was added dropwise and the mixture was stirred at rt overnight. The mixture was evaporated and purified by flash chromatography to give 84.0 mg (88 % yield) of the target compound.

¹H-NMR (400 MHz, DMSO-d6) δ [ppm]: 1.170 (2.41), 1.186 (2.37), 1.392 (16.00), 1.411 (14.84), 2.255 (0.63), 2.266 (0.56), 2.322 (0.56), 2.326 (0.71), 2.331 (0.52), 2.518 (2.60), 2.522 (1.67), 2.664 (0.52), 2.669 (0.72), 2.673 (0.52), 3.312 (0.96), 3.355 (0.78), 3.375 (0.84), 3.405 (0.89), 3.426 (0.76), 3.455 (0.93), 3.471 (0.65), 3.495 (1.07), 3.518 (0.51), 3.664 (0.60), 3.691 (0.98), 3.719 (0.46), 4.071 (11.76), 5.759 (0.96), 6.480 (2.27), 6.492 (2.37), 8.882 (0.44).

### Intermediate 14

### (rac)-tert-butyl-2-(6-amino-5-cyanopyridin-3-yl)-6,7-dihydrospiro[pyrazolo[5,1-c][1,4]oxazine-4,3'-pyrrolidine]-1'-carboxylate

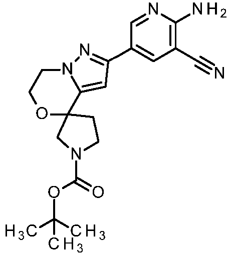

(Rac)-tert-butyl-2-bromo-6,7-dihydrospiro[pyrazolo[5,1-c][1,4]oxazine-4,3'-pyrrolidine]-1'-carboxylate (621 mg, 1.73 mmol), 2-amino-5-(4,4,5,5-tetramethyl-1,3,2-dioxaborolan-2-yl)pyridine-3-carbonitrile (850 mg, 3.47 mmol), XPhos Pd G2 (68.2 mg, 86.7 µmol) and potassium phosphate (10 mL, 0.50 M, 5.2 mmol) were stirred in degassed dioxane (25 mL) for 2h at 100°C. The reaction mixture was diluted with ethyl acetate and water. The phases were separated and the aqueous phase was extracted twice with ethyl acetate. The combined organic phases were dried over sodium sulfate and concentrated. The residue was purified by flash chromatography to afford 736 mg of the title compound.

¹H-NMR (400 MHz, DMSO-d6) δ [ppm]: 1.065 (4.11), 1.265 (0.50), 1.399 (7.96), 1.424 (6.94), 2.518 (0.73), 2.523 (0.49), 3.159 (16.00), 3.172 (15.27), 3.366 (0.44), 3.375 (0.41), 3.397 (0.49), 3.428 (0.42), 3.483 (0.44), 3.551 (0.65), 3.726 (0.52), 3.941 (0.69), 4.087 (1.54), 4.100 (4.02), 4.113 (6.91), 4.126 (1.93), 6.699 (0.95), 6.717 (1.09), 7.046 (2.34), 8.114 (1.17), 8.119 (1.15), 8.591 (2.11), 8.598 (2.12).

### Intermediate 15

### (rac)-tert-butyl-2'-(6-amino-5-cyanopyridin-3-yl)-5',6'-dihydrospiro[pyrrolidine-3,4'-pyrrolo[1,2-b]pyrazole]-1-carboxylate

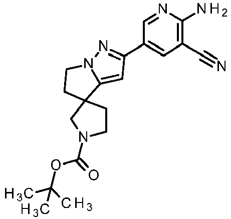

(Rac)-tert-butyl-2'-[(trifluoromethanesulfonyl)oxy]-5',6'-dihydrospiro[pyrrolidine-3,4'-pyrrolo[1,2-b]pyrazole]-1-carboxylate (713 mg, 1.73 mmol), 2-amino-5-(4,4,5,5-tetramethyl-1,3,2-dioxaborolan-2-yl)pyridine-3-carbonitrile (850 mg, 3.47 mmol), XPhos Pd G2 (68.2 mg, 86.7 µmol) and potassium phosphate (10 mL, 0.50 M, 5.2 mmol) were dissolved in degassed dioxane (25 mL) under argon and stirred at 100°C for 2h. The mixture was diluted with ethyl acetate and water, the layers were separated and the aqueous phase was extracted twice with ethyl acetate. The combined organic phases were dried over sodium sulfate and concentrated. Purified by flash chromatography to afford 730 mg of the title compound.

¹H-NMR (400 MHz, DMSO-d6) δ [ppm]: 1.065 (2.09), 1.396 (7.48), 1.432 (6.06), 2.029 (0.79), 2.047 (0.64), 2.518 (0.66), 2.523 (0.65), 2.534 (0.65), 2.542 (0.75), 2.560 (0.48), 3.159 (16.00), 3.172 (15.80), 3.386 (0.65), 3.407 (2.03), 3.434 (0.62), 3.496 (0.64), 4.087 (1.45), 4.101 (3.63), 4.113 (3.70), 4.127 (1.34), 4.154 (0.51), 4.163 (0.62), 4.172 (0.92), 4.181 (0.90), 4.189 (0.62), 4.198 (0.49), 6.454 (0.82), 6.479 (1.01), 6.985 (2.41), 8.151 (1.16), 8.156 (1.10), 8.611 (1.97), 8.618 (1.97).

### Intermediate 16

### (rac)-tert-butyl-2'-[6-amino-5-(trifluoromethyl)pyridin-3-yl]-5',6'-dihydrospiro[pyrrolidine-3,4'-pyrrolo[1,2-b]pyrazole]-1-carboxylate

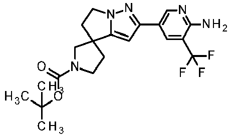

(Rac)-tert-butyl-2'-[(trifluoromethanesulfonyl)oxy]-5',6'-dihydrospiro[pyrrolidine-3,4'-pyrrolo[1,2-b]pyrazole]-1-carboxylate (2.50 g, 6.08 mmol) was solubilised in dioxane (51 mL) under nitrogen, 5-(4,4,5,5-tetramethyl-1,3,2-dioxaborolan-2-yl)-3-(trifluoromethyl)pyridin-2-amine (2.63 g, 9.12 mmol), potassium phosphate solution (36 mL, 0.50 M, 18 mmol) and XPhos Pd G2 (239 mg, 304 µmol) were added and the mixture was stirred overnight at 100°C. The mixture was diluted with ethyl acetate, washed with saturated sodium chloride solution, dried and concentrated under reduced pressure. The residue was purified by flash chromatography to give 2.70 g of the target compound.

¹H-NMR (400 MHz, DMSO-d6) δ [ppm]: 1.066 (16.00), 1.172 (0.51), 1.397 (3.81), 1.416 (0.58), 1.433 (2.99), 1.988 (1.01), 2.518 (0.54), 2.523 (0.45), 3.413 (1.28), 3.938 (2.53), 4.176 (0.46), 4.184 (0.45), 5.758 (1.10), 6.507 (0.50), 6.538 (1.08), 8.016 (0.55), 8.588 (0.61), 8.592 (0.59).

### Intermediate 17

### (rac)-tert-butyl-2-[6-amino-5-(trifluoromethyl)pyridin-3-yl]-6,7-dihydrospiro[pyrazolo[5,1-c][1,4]oxazine-4,3'-pyrrolidine]-1'-carboxylate

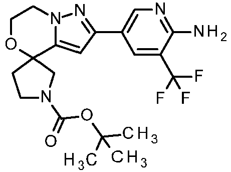

Intermediate 17 was prepared in analogy to intermediate (rac)-tert-butyl-2'-[6-amino-5-(trifluoromethyl)pyridin-3-yl]-5',6'-dihydrospiro[pyrrolidine-3,4'-pyrrolo[1,2-b]pyrazole]-1-carboxylate using (rac)- tert-butyl -2-bromo-6,7-dihydrospiro[pyrazolo[5,1-c][1,4]oxazine-4,3'-pyrrolidine]-1'-carboxylate and 5-(4,4,5,5-tetramethyl-1,3,2-dioxaborolan-2-yl)-3-(trifluoromethyl)pyridin-2-amine.

### Intermediate 18

### (rac)-5-[5',6'-dihydrospiro[pyrrolidine-3,4'-pyrrolo[1,2-b]pyrazol]-2'-yl]-3-(trifluoromethyl)pyridin-2-amine-hydrochloride

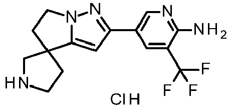

(Rac)-tert-butyl-2'-[6-amino-5-(trifluoromethyl)pyridin-3-yl]-5',6'-dihydrospiro[pyrrolidine-3,4'-pyrrolo[1,2-b]pyrazole]-1-carboxylate (2.70 g, 6.38 mmol) was solubilised in dioxane (56 mL), hydrochloric acid (16 mL, 4.0 M in dioxane, 64 mmol) was added and the mixture was stirred overnight at rt. The mixture was evaporated to give 3.30 g of the target compound.

1H-NMR (400 MHz, DMSO-d6) δ [ppm]: 1.065 (16.00), 1.156 (0.82), 2.164 (0.59), 2.172 (0.41), 2.332 (0.74), 2.518 (3.21), 2.522 (1.93), 2.542 (0.80), 2.546 (0.76), 2.673 (1.08), 3.565 (3.33), 3.911 (3.01), 4.182 (0.43), 4.197 (0.56), 6.624 (2.53), 8.023 (0.74), 8.028 (0.72), 8.576 (0.74).

### Intermediate 19

### (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride

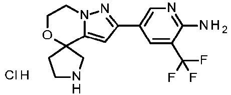

This intermediate was prepared in analogy to intermediate (rac)-5-[5',6'-dihydrospiro[pyrrolidine-3,4'-pyrrolo[1,2-b]pyrazol]-2'-yl]-3-(trifluoromethyl)pyridin-2-amine-hydrochloride using (rac)-tert-butyl-2-[6-amino-5-(trifluoromethyl)pyridin-3-yl]-6,7-dihydrospiro[pyrazolo[5,1-c][1,4]oxazine-4,3'-pyrrolidine]-1'-carboxylate.

### Intermediate 20

### (rac)-2-amino-5-[6,7-dihydrospiro[pyrazolo[5,1-c][1,4]oxazine-4,3'-pyrrolidin]-2-yl]pyridine-3-carbonitrile hydrochloride

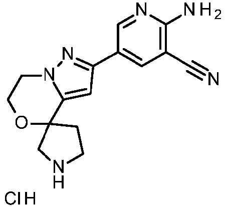

This intermediate was prepared in analogy to Intermediate (rac)-5-[5',6'-dihydrospiro[pyrrolidine-3,4'-pyrrolo[1,2-b]pyrazol]-2'-yl]-3-(trifluoromethyl)pyridin-2-amine-hydrochloride using (rac)-tert-butyl-2-(6-amino-5-cyanopyridin-3-yl)-6,7-dihydrospiro[pyrazolo[5,1-c][1,4]oxazine-4,3'-pyrrolidine]-1'-carboxylate.

¹H-NMR (400 MHz, DMSO-d6) δ [ppm]: 1.064 (3.61), 1.268 (1.74), 2.212 (0.53), 2.235 (1.04), 2.245 (0.99), 2.272 (1.27), 2.297 (0.74), 2.518 (6.21), 2.523 (3.83), 3.164 (8.69), 3.250 (0.63), 3.269 (1.35), 3.282 (1.11), 3.301 (1.82), 3.319 (1.23), 3.338 (0.95), 3.488 (0.80), 3.498 (1.02), 3.565 (2.56), 3.684 (1.03), 3.698 (1.41), 3.713 (1.14), 3.729 (1.01), 3.989 (9.14), 4.088 (2.10), 4.152 (16.00), 6.768 (14.71), 8.166 (9.01), 8.172 (8.69), 8.602 (8.75), 8.607 (8.64), 9.839 (0.80).

### Intermediate 21

### (rac)-2-amino-5-[5',6'-dihydrospiro[pyrrolidine-3,4'-pyrrolo[1,2-b]pyrazol]-2'-yl]pyridine-3-carbonitrile hydrochloride

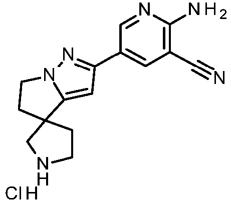

This intermediate was prepared in analogy to Intermediate (rac)-5-[5',6'-dihydrospiro[pyrrolidine-3,4'-pyrrolo[1,2-b]pyrazol]-2'-yl]-3-(trifluoromethyl)pyridin-2-amine-hydrochloride using (rac)-tert-butyl-2'-(6-amino-5-cyanopyridin-3-yl)-5',6'-dihydrospiro[pyrrolidine-3,4'-pyrrolo[1,2-b]pyrazole]-1-carboxylate.

¹H-NMR (400 MHz, DMSO-d6) δ [ppm]: 1.062 (1.01), 2.133 (0.79), 2.153 (1.92), 2.164 (1.30), 2.173 (1.17), 2.181 (1.08), 2.523 (0.88), 2.532 (0.52), 2.548 (0.68), 2.566 (0.94), 2.580 (1.01), 2.599 (0.58), 2.695 (0.56), 2.714 (1.00), 2.729 (0.90), 2.743 (0.54), 2.747 (0.63), 2.762 (0.41), 3.161 (16.00), 3.228 (0.66), 3.242 (0.71), 3.275 (0.47), 3.311 (0.41), 3.321 (0.47), 3.332 (0.64), 3.340 (0.54), 3.361 (0.40), 3.382 (0.90), 3.388 (0.66), 3.404 (1.08), 3.415 (1.30), 3.427 (1.04), 3.433 (1.00), 4.178 (1.12), 4.187 (1.19), 4.194 (1.57), 4.197 (1.63), 4.201 (1.66), 4.206 (1.51), 4.213 (1.19), 4.221 (0.99), 6.149 (0.46), 6.351 (0.50), 6.639 (8.60), 8.264 (4.17), 8.269 (4.21), 8.592 (4.68), 8.598 (4.48), 9.824 (0.61).

### Intermediate 22

### (rac)-5',6'-dihydrospiro[pyrrolidine-3,4'-pyrrolo[1,2-b]pyrazol]-2'-yl trifluoromethanesulfonate-hydrogen chloride

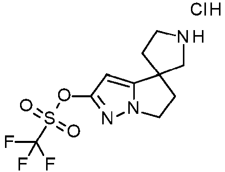

Tert-butyl 2'-{[(trifluoromethyl)sulfonyl]oxyl-5',6'-dihydro-1H-spiro[pyrrolidine-3,4'-pyrrolo[1,2-b]pyrazole]-1-carboxylate (1.00 g, 2.43 mmol) was dissolved in dioxane (21 mL) and cooled to 0°C. Hydrochloric acid (3.04 mL, 12.15 mmol, 4M in dioxane) was added and the reaction mixture was stirred at rt overnight. The rection mixture was concentrated to afford 1.11 g of the title compound.

¹H-NMR (400 MHz, DMSO-d6) δ [ppm]: 1.391 (1.27), 1.418 (1.03), 2.161 (0.45), 2.164 (0.43), 2.180 (1.26), 2.199 (0.82), 2.518 (0.50), 2.524 (0.64), 2.539 (0.48), 2.652 (0.41), 3.307 (0.49), 3.390 (0.49), 3.400 (1.01), 3.429 (0.61), 3.564 (16.00), 4.210 (0.56), 4.218 (0.61), 4.226 (0.78), 4.228 (0.78), 4.232 (0.67), 4.238 (0.61), 4.245 (0.62), 4.252 (0.47), 6.444 (2.92), 9.626 (0.44).

### Intermediate 23

### (rac)-1-[(1-phenylcyclobutyl)carbamoyl]-5',6'-dihydrospiro[pyrrolidine-3,4'-pyrrolo[1,2-b]pyrazol]-2'-yl trifluoromethanesulfonate

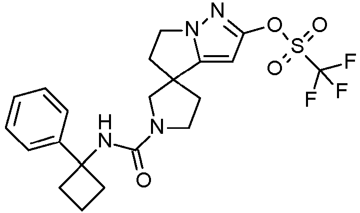

1-Phenylcyclobutan-1-amine (429 mg, 2.92 mmol), N,N'-carbonyldiimidazole (473 mg, 2.92 mmol) and N,N-diisopropylethylamine (2.1 ml, 12 mmol) were dissolved in DMF (25 mL). It was stirred for 1h at rt. Then (rac)-5',6'-dihydrospiro[pyrrolidine-3,4'-pyrrolo[1,2-b]pyrazol]-2'-yl trifluoromethanesulfonate-hydrogen chloride (845 mg, 2.43 mmol) in DMF (10 mL) was added and stirred at 60°C for 3h. Half concentrated sodium chloride solution was added and the mixture was extracted three times with ethyl acetate. The combined organic phases were dried and concentrated obtaining 1.55 g of the title compound.

¹H-NMR (400 MHz, DMSO-d6) δ [ppm]: 1.392 (2.75), 1.420 (2.20), 1.739 (0.41), 1.762 (0.63), 1.780 (0.60), 1.785 (0.59), 1.950 (0.50), 1.957 (0.51), 1.972 (0.74), 1.978 (0.60), 1.988 (0.44), 1.995 (0.57), 2.000 (0.58), 2.011 (0.42), 2.023 (0.47), 2.042 (0.73), 2.055 (0.69), 2.060 (0.67), 2.076 (0.66), 2.097 (1.02), 2.117 (0.52), 2.128 (0.47), 2.327 (0.81), 2.332 (0.60), 2.344 (1.28), 2.349 (1.21), 2.353 (1.12), 2.358 (1.13), 2.366 (1.44), 2.380 (1.43), 2.397 (1.02), 2.419 (0.67), 2.438 (0.70), 2.453 (1.77), 2.466 (2.62), 2.472 (2.45), 2.518 (1.05), 2.523 (0.72), 2.728 (13.33), 2.730 (13.09), 2.888 (16.00), 3.382 (0.83), 3.404 (0.54), 3.430 (2.61), 3.436 (2.45), 3.461 (0.46), 3.475 (0.46), 3.480 (0.44), 3.487 (0.56), 3.495 (0.60), 3.501 (0.54), 3.507 (0.60), 3.513 (0.42), 4.207 (1.69), 4.214 (0.70), 4.225 (2.94), 4.235 (0.64), 4.243 (1.59), 6.186 (4.87), 6.253 (0.51), 6.410 (0.71), 6.713 (2.26), 7.012 (1.62), 7.138 (0.73), 7.142 (0.46), 7.147 (0.47), 7.152 (0.57), 7.156 (1.71), 7.164 (1.03), 7.171 (0.78), 7.174 (1.27), 7.178 (0.78), 7.183 (0.81), 7.186 (0.44), 7.254 (1.09), 7.257 (0.73), 7.263 (2.01), 7.267 (0.98), 7.270 (1.03), 7.273 (2.14), 7.282 (3.26), 7.291 (1.47), 7.296 (0.90), 7.300 (1.88), 7.336 (1.74), 7.339 (1.95), 7.352 (0.44), 7.357 (1.32), 7.361 (0.94), 7.406 (2.77), 7.409 (2.96), 7.422 (0.74), 7.427 (2.42), 7.430 (1.93), 7.639 (1.02), 7.951 (1.94).

### Intermediate 24

### tert-butyl (3S)-2'-[(trifluoromethanesulfonyl)oxy]-5',6'-dihydrospiro[pyrrolidine-3,4'-pyrrolo[1,2-b]pyrazole]-1-carboxylate

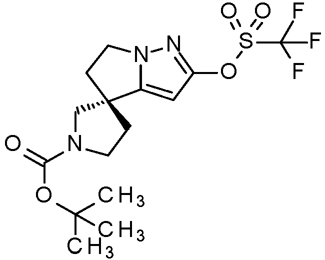

The title compound was prepared by chiral separation of the corresponding racemic mixture. Chiral separation protocol :

### Preparative

Instrument: Sepiatec: Prep SFC100; Column: Chiralpak IG 5µ 250x30mm; eluent A: CO2; eluent B: ethanol + 0.2 vol % aqueous ammonia (32%); isocratic: 5%B; flow: 100 mL/min; temperature: 40°C; BPR: 150bar; UV: 210 nm

### Analytical

Instrument: Agilent: 1260, Aurora SFC-Modul; Column: Chiralpak IG 5µ 100x4.6mm; eluent A: CO2; eluent B: ethanol + 0.2 vol % aqueous ammonia (32%); isocratic: 5%B; flow: 4 mL/min; temperature: 37.5°C; BPR: 100bar; UV: 210 nm
Retention time preparative method : 6,0 - 7,5 min
Retention Time analytical method: 1,60 min
[α]²⁰_{D} : -9.6° (c = 1.00, DMSO)

### Intermediate 25

### tert-butyl (3R)-2'-[(trifluoromethanesulfonyl)oxy]-5',6'-dihydrospiro[pyrrolidine-3,4'-pyrrolo[1,2-b]pyrazole]-1-carboxylate

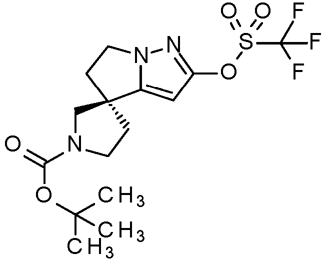

The title compound was prepared by chiral separation of the corresponding racemic mixture. Chiral separation protocol :

### Preparative

Instrument: Sepiatec: Prep SFC100; Column: Chiralpak IG 5µ 250x30mm; eluent A: CO2; eluent B: ethanol + 0.2 vol % aqueous ammonia (32%); isocratic: 5%B; flow: 100 mL/min; temperature: 40°C; BPR: 150bar; UV: 210 nm

### Analytical

Instrument: Agilent: 1260, Aurora SFC-Modul; Column: Chiralpak IG 5µ 100x4.6mm; eluent A: CO2; eluent B: ethanol + 0.2 vol % aqueous ammonia (32%); isocratic: 5%B; flow: 4 mL/min; temperature: 37.5°C; BPR: 100bar; UV: 210 nm
Retention time preparative method : 3,8 - 4,7 min
Retention Time analytical method: 1,05 min
[α]²⁰_{D} : +10.4° (c = 1.00, DMSO)

### Intermediate 26

### (3R)-5',6'-dihydrospiro[pyrrolidine-3,4'-pyrrolo[1,2-b]pyrazol]-2'-yl trifluoromethanesulfonate-hydrochloride salt

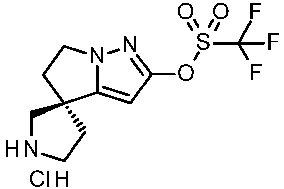

tert-butyl (3R)-2'-[(trifluoromethanesulfonyl)oxy]-5',6'-dihydrospiro[pyrrolidine-3,4'-pyrrolo[1,2-b]pyrazole]-1-carboxylate (5.00 g, 12.2 mmol) was solubilised in 1,4-dioxane (200 mL) and treated with HCl in dioxane (30 mL, 4.0 M, 120 mmol). The reaction mixture was stirred for 8h at 40°C. The reaction was then cooled to rt and concentrated under reduced pressure to give 4.69 g (90 % purity, 100 % yield) of the title compound that was used without further purification.
LC-MS (method 1): Rₜ = 1.00 min; MS (ESlpos): m/z = 312 [M+H]⁺

### Intermediate 27

### (rac)-1-(ethylcarbamoyl)-5',6'-dihydrospiro[pyrrolidine-3,4'-pyrrolo[1,2-b]pyrazol]-2'-yl trifluoromethanesulfonate

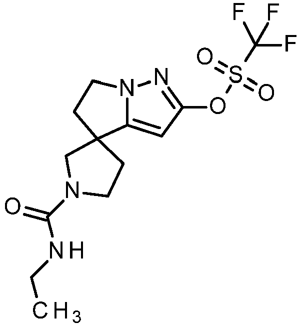

(rac)-5',6'-dihydrospiro[pyrrolidine-3,4'-pyrrolo[1,2-b]pyrazol]-2'-yl trifluoromethanesulfonate (3.00 g, 9.64 mmol) was solubilised in dichloromethane (110 mL), N,N-diisopropylethylamine (17 mL, 96 mmol) was added, cooled to 0°C and isocyanatoethane (790 µL, 9.6 mmol) was added. The mixture was stirred for 2 h at rt. It was concentrated under reduced pressure and purified by flash chromatography to give 2.80 g (76 % yield) of the title compound.
LC-MS (method 1): Rt = 1.04 min; MS (ESlpos): m/z = 383 [M+H]⁺
1H-NMR (400MHz, DMSO-d6): δ [ppm]= 1.01 (t, 3H), 2.00 - 2.16 (m, 2H), 2.43 - 2.48 (m, 2H), 2.99 - 3.09 (m, 2H), 3.35 - 3.51 (m, 4H), 4.22 (t, 2H), 6.16 (t, 1H), 6.24 (s, 1H).

### Intermediate 28

(3R)-1-(ethylcarbamoyl)-5',6'-dihydrospiro[pyrrolidine-3,4'-pyrrolo[1,2-b]pyrazol]-2'-yl trifluoromethanesulfonate

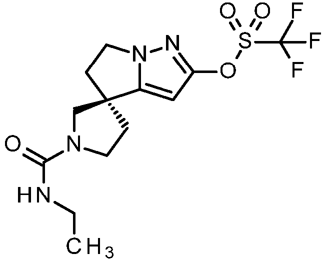

(3R)-5',6'-dihydrospiro[pyrrolidine-3,4'-pyrrolo[1,2-b]pyrazol]-2'-yl trifluoromethanesulfonate-hydrochloride salt (4.49 g, 90 % purity, 11.6 mmol) was solubilised in dichloromethane (200 mL), N,N-diisopropylethylamine (20 mL, 120 mmol) was added and the mixture was cooled to 0°C. Ethylisocyanate (1.1 mL, 14 mmol) was added and the mixture was allowed to warm up to rt for 2 h. The mixture was concentrated under reduced pressure and the residue was purified by flash chromatography to give 4.12 g (98 % purity, 91 % yield) of the title compound.
1H-NMR (400MHz, CHLOROFORM-d): δ [ppm]= 1.16 (t, 3H), 2.09 (ddd, 1H), 2.18 - 2.28 (m, 1H), 2.45 - 2.54 (m, 1H), 2.54 - 2.64 (m, 1H), 3.30 (qd, 2H), 3.47 - 3.63 (m, 4H), 4.17 (br t, 1H), 4.23 (t, 2H), 5.86 (s, 1H).
[α]²⁰_{D} : +19.4° (c = 1.00, MeOH)
LC-MS (method 1): Rₜ = 1.02 min; MS (ESlpos): m/z = 383 [M+H]⁺

### Intermediate 29

### (3R)-1-[(propan-2-yl)carbamoyl]-5',6'-dihydrospiro[pyrrolidine-3,4'-pyrrolo[1,2-b]pyrazol]-2'-yl trifluoromethanesulfonate

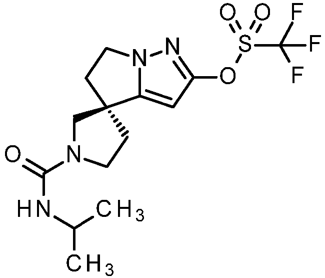

(3R)-5',6'-dihydrospiro[pyrrolidine-3,4'-pyrrolo[1,2-b]pyrazol]-2'-yl trifluoromethanesulfonate hydrogen chloride salt (200 mg, 80 % purity, 460 µmol) was solubilised in dichloromethane (7.9 mL), N,N-diisopropylethylamine (800 µl, 4.6 mmol) was added and the mixture was cooled to 0°C. isopropylisocyanate (54 µl, 550 µmol) was added slowly and the mixture allowed to warm up to r.t. After 2h isopropylisocyanate (54 µl, 550 µmol) was added again and the reaction was stirred at rt for 18h. The reaction mixture was then concentrated under reduced pressure and purified by flash column chromatography to give 186 mg (95 % purity, 97 % yield) of the title compound.
LC-MS (method 1): Rₜ = 1.10 min; MS (ESlpos): m/z = 397 [M+H]⁺
1H-NMR (400MHz, CHLOROFORM-d): δ [ppm]= 1.15 - 1.21 (m, 6H), 2.09 (ddd, 1H), 2.23 (dt, 1H), 2.45 - 2.54 (m, 1H), 2.55 - 2.63 (m, 1H), 3.46 - 3.54 (m, 3H), 3.57 (dd, 1H), 3.98 (br d, 2H), 4.24 (t, 2H), 5.86 (s, 1H).

### Intermediate 30

### (3R)-1-(cyclobutylcarbamoyl)-5',6'-dihydrospiro[pyrrolidine-3,4'-pyrrolo[1,2-b]pyrazol]-2'-yl trifluoromethanesulfonate

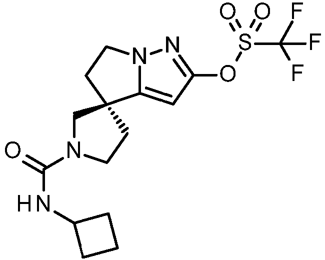

(3R)-5',6'-dihydrospiro[pyrrolidine-3,4'-pyrrolo[1,2-b]pyrazol]-2'-yl trifluoromethanesulfonate hydrogen chloride salt (430 mg, 80 % purity, 989 µmol) was suspended in dichloromethane (17 mL), N,N-diisopropylethylamine (1.7 mL, 9.9 mmol) was added and the mixture was cooled to 0°C. Cyclobutylisocyanate (110 µl, 1.2 mmol) was added and the mixture allowed to warm up to r.t. After 2h cyclobutylisocyanate (65 µl, 0.6 mmol) was added and the reaction was stirred 18h at r.t.. The reaction mixture was concentrated under reduced pressure and purified by dlash column chromatography to give 461 mg (90 % purity, 103 % yield) of the title compound.
LC-MS (method 1): Rₜ = 1.12 min; MS (ESlpos): m/z = 409 [M+H]⁺
1H-NMR (400MHz, CHLOROFORM-d): δ [ppm]= 1.63 - 1.73 (m, 2H), 1.76 - 1.89 (m, 2H), 2.09 (s, 1H), 2.18 - 2.27 (m, 1H), 2.31 - 2.41 (m, 2H), 2.50 (dd, 1H), 2.54 - 2.64 (m, 1H), 3.47 - 3.63 (m, 4H), 4.23 (t, 2H), 4.28 - 4.38 (m, 2H), 5.86 (s, 1H).

### Intermediate 31

### 1-tert-butyl 3-methyl 3-(prop-2-en-1-yl)azetidine-1,3-dicarboxylate

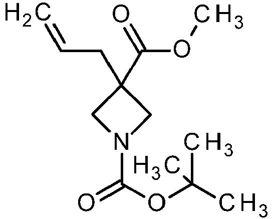

To a solution of 1-tert-butyl 3-methyl azetidine-1,3-dicarboxylate (940 g, 4.37 mol, 1.00 equiv, CAS-RN [610791-05-4]) in dry THF(9 L) was added LiHMDS (8.8 L, 8.74 mol, 2.00 equiv) at -78°C under nitrogen atmosphere, and the reaction mixture was stirred at this temperature for 30 min. Then a solution of 3-bromoprop-1-ene (1058 g, 8.74 mol, 2.00 equiv) in dry THF (5 L) was added and the mixture was left to warm to ambient temperature and stirred overnight (monitored by TLC). Sat. aq. solution of NH₄Cl (15 L) was added and the mixture was extracted with 3 x 10 L of EtOAc, and the combined organic layer was washed with 3x15 L of brine, dried over anhydrous Na₂SO₄, and concentrated under vacuum to yield a crude product which was directly purified by silica gel column (EtOAc / petroleum ether) to give 832 g (75%) of the title compound as yellow oil.

### Intermediate 32

### tert-butyl 3-(3-methoxy-3-oxopropanoyl)-3-(prop-2-en-1-yl)azetidine-1-carboxylate

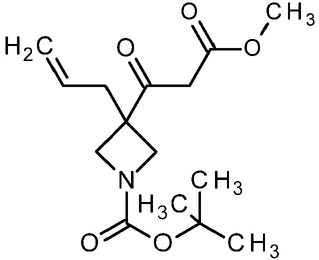

To a solution of methyl acetate (482 g, 6.52 mol, 2.00 equiv) in dry THF(9 L) was added LiHMDS (6.6 L, 6.52 mol, 2.00 equiv) at -78°C under nitrogen atmosphere, and the reaction mixture was stirred at this temperature for 1 h. Then a solution of 1-tert-butyl 3-methyl 3-(prop-2-en-1-yl)azetidine-1,3-dicarboxylate (832 g, 3.26 mol, 1.00 equiv) in dry THF( 2 L) was added and the mixture was left to warm to rt and stirred for another 2 h (monitored by TLC). Sat. aq. NH4Cl (10 L) was added and the mixture was extracted with 3x10 L of EtOAc, and the combined organic layer was washed with 3x10 L of brine, dried over anhydrous Na2SO4, and concentrated under vacuum to yield a crude product which was directly purified by silica gel column (EtOAc/petroleum ether) to give 810 g (84%) of the title compound as yellow oil.

### Intermediate 33

### tert-butyl 3-(3-hydroxy-1H-pyrazol-5-yl)-3-(prop-2-en-1-yl)azetidine-1-carboxylate

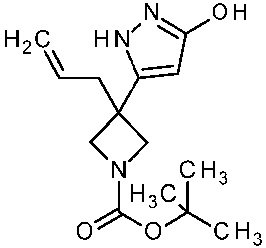

To a stirred solution of tert-butyl 3-(3-methoxy-3-oxopropanoyl)-3-(prop-2-en-1 -yl)azetidine-1-carboxylate (810 g, 2.73 mol, 1.00 equiv) in anhydrous EtOH (3 L) was added hydrazine-water (1:1) (280 g, 8.75 mol, 3.00 equiv). This mixture was stirred at an oil bath. The temperature was warmed to 80°C and stirred for another 1 h (monitored by LCMS). The solvent was removed in vacuum and the residue was dissolved in 2 L EtOAc and washed with 2x2 L of aq. HCl (1 M), 1x2 L of brine, dried over anhydrous Na2SO4 to afford 779 g (96%) of the title compound as white solid. LCMS: (ES, m/z):280 [M+H]+

### Intermediate 34

### tert-butyl 3-(prop-2-en-1-yl)-3-[3-(trifluoromethanesulfonyloxy)-1H-pyrazol-5-yl]azetidine-1-carboxylate

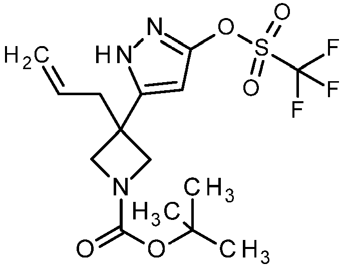

To a stirred solution of tert-butyl 3-(3-hydroxy-1H-pyrazol-5-yl)-3-(prop-2-en-1-yl)azetidine-1-carboxylate (779 g, 2.79 mol, 1.00 equiv) in anhydrous THF (10 L) was added DIPEA (1079 g, 8.37 mol, 3.00 equiv) followed by 1,1,1-trifluoro-N-phenyl-N-trifluoromethanesulfonylmethanesulfonamide (1095 g, 3.07 mol, 1.10 equiv). This mixture was stirred at 60 °C in an oil bath for 2 h, the solvent was removed under vacuum and the residue was dissolved in 2 L EtOAc and washed with 3x2 L of aq. HCl (1 M) and 3x2 L of water, dried over anhydrous Na2SO4, concentrated under vacuum and recrystallization from MTBE to afford 810 g (71%) of the title compound as white solid. LCMS: (ES, m/z):412 [M+H]+

### Intermediate 35

### tert-butyl 3-(2-hydroxyethyl)-3-[3-(trifluoromethanesulfonyloxy)-1H-pyrazol-5-yl]azetidine-1-carboxylate

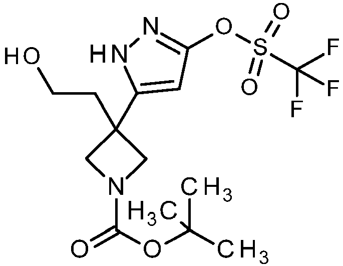

To a stirred solution of tert-butyl 3-(prop-2-en-1-yl)-3-[3-(trifluoromethanesulfonyloxy)-1H-pyrazol-5-yl]azetidine-1-carboxylate (400 g, 0.97 mol, 1.00 equiv) in dioxane/H2O (2L, 3/2, v/v) was added 2,6-Lutidine (207 g, 1.94 mol, 2.00 equiv) followed by K2OsO4*2H2O (7.87 g, 0.02 mol, 0.02 equiv). This mixture was stirred at rt for 40 min before NaIO4 (888 g, 4.15 mol, 4.00 equiv) was added in batches. The final mixture was stirred for another 2 h at rt. The reaction mixture was filtrated and the filtrate cake was washed with EtOAc (4 L), the filtrate was separated. The organic layer was concentrated under vacuum. The residue was dissolved in 2 L MeOH and stirred at 0 °C in an ice-water bath, then NaBH4 (19.0 g, 0.51 mol, 0.50 equiv) was added in small portions. The pH value of the reaction mixture was adjusted to 7 by 0.1 mol/L HCl aq. solution when the addition was completed. The resulting mixture was extracted with 3x5 L of DCM. The organic phase was combined, washed with 3x5 L of brine, dried over anhydrous Na2SO4, concentrated under vacuum yield a crude product which was directly purified by silica gel column (EtOAc/petroleum ether) to give 360 g (89.4%) of the title compound as yellow oil. LCMS: (ES, m/z):416 [M+H]+

### Intermediate 36

### tert-butyl 2'-(trifluoromethanesulfonyloxy)-5',6'-dihydrospiro[azetidine-3,4'-pyrrolo[1,2-b]pyrazole]-1-carboxylate

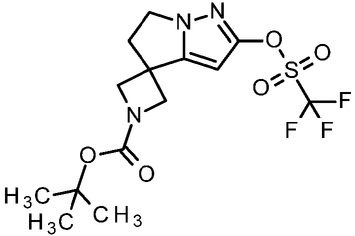

To a solution of tert-butyl 3-(2-hydroxyethyl)-3-[3-(trifluoromethanesulfonyloxy)-1H-pyrazol-5-yl]azetidine-1-carboxylate (360 g, 0.87 mol, 1.00 equiv) and PPh3 (320 g, 1.22 mol, 1.40 equiv) in dry THF (4 L) was added DIAD (246 g, 1.4 mol, 1.30 equiv) dropwise at rt under nitrogen atmosphere. The reaction mixture was stirred at this temperature for 16 h. The solvent was removed under vacuum and the residue was dissolved in MTBE (2 L) and stirred overnight until white solid was precipitated. The solid was removed by filtration. The filtrate was concentrated under vacuum and the crude product was re-crystallized from MeOH to give 240 g (69.5%) of the title compound as white solid. LCMS: (ES, m/z):398 [M+H]+, 1H-NMR (400 MHz, DMSO-d6) δ [ppm]: 1.399 (16.00), 2.787 (0.67), 2.805 (1.02), 2.822 (0.71), 4.044 (1.70), 4.129 (0.72), 4.148 (1.05), 4.165 (0.67), 6.536 (2.35).

### Intermediate 37

### 5',6'-dihydrospiro[azetidine-3,4'-pyrrolo[1,2-b]pyrazol]-2'-yl trifluoromethanesulfonate TFA salt

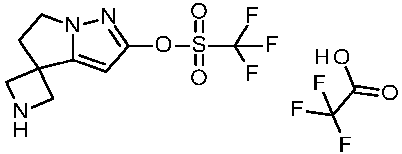

tert-butyl 2'-[(trifluoromethanesulfonyl)oxy]-5',6'-dihydrospiro[azetidine-3,4'-pyrrolo[1,2-b]pyrazole]-1-carboxylate (1.00 g, 2.52 mmol) was solubilised in dichloromethane (60 mL) under nitrogen, TFA (6.3 mL, 82 mmol) was added and the mixture was stirred for 2 h at rt. The mixture was concentrated under reduced pressure to give 1.23 g of the title compound, which was used without further purification.
LC-MS (method 1): Rt = 0.96 min; MS (ESlpos): m/z = 298 [M+H]⁺

### Intermediate 38

### 1-(ethylcarbamoyl)-5',6'-dihydrospiro[azetidine-3,4'-pyrrolo[1,2-b]pyrazol]-2'-yl trifluoromethanesulfonate

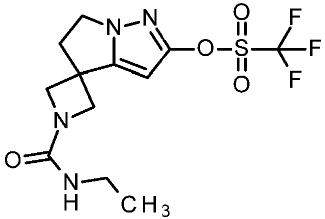

trifluoroacetate-5',6'-dihydrospiro[azetidine-3,4'-pyrrolo[1,2-b]pyrazol]-2'-yl trifluoromethanesulfonate (1.27 g, 81 % purity, 2.50 mmol) was solubilised in dichloromethane (60 mL) under nitrogen, N,N-diisopropylethylamine (4.4 mL, 25 mmol) and isocyanatoethane (990 µL, 13 mmol) were added and the mixture was stirred overnight at rt. The mixture was evaporated and the residue was purified by flash chromatography to give 970 mg of the title compound.
LC-MS (method 1): Rt = 0.99 min; MS (ESlpos): m/z = 369 [M+H]⁺
¹H-NMR (400 MHz, DMSO-d6) δ [ppm]: 0.984 (6.75), 1.001 (16.00), 1.019 (7.19), 1.035 (0.68), 1.052 (1.23), 1.070 (0.55), 1.137 (1.30), 1.154 (1.27), 2.331 (0.64), 2.518 (3.40), 2.522 (2.19), 2.673 (0.64), 2.771 (2.59), 2.789 (3.93), 2.806 (2.78), 2.986 (0.92), 3.003 (2.90), 3.018 (3.13), 3.022 (3.09), 3.036 (2.93), 3.053 (0.92), 3.934 (1.95), 3.955 (10.37), 3.963 (11.08), 3.983 (2.04), 4.140 (3.03), 4.149 (0.87), 4.158 (4.09), 4.166 (0.83), 4.175 (2.79), 6.443 (0.99), 6.457 (1.98), 6.471 (0.98), 6.498 (10.15).

### Intermediate 39

### (rac)-1-tert-butyl 3-methyl-3-(3-{[tert-butyl(dimethyl)silyl]oxy}propyl)pyrrolidine-1,3-dicarboxylate

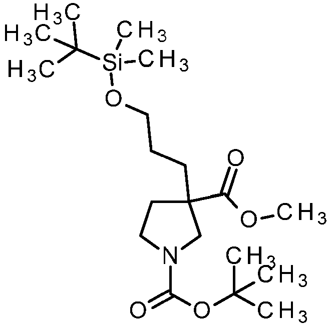

(rac)-1-tert-butyl 3-methyl-pyrrolidine-1,3-dicarboxylate (5.00 g, 21.8 mmol) was solubilised in THF (50 mL), cooled to -78°C and LiHMDS (44 mL, 1.0 M in THF, 43.6 mmol) was added dropwise. The mixture was stirred at -78°C for 1 h and (3-bromopropoxy) (tert-butyl)dimethylsilane (10 mL, 44 mmol), solubilised in THF (28 mL), was added dropwise. It was allowed to warm up to rt overnight. The mixture was diluted with sat. NH4Cl solution, extracted with ethyl acetate/THF and the organic layer was dried with a silicone filter and evaporated. The residue was purified by flash chromatography to give 5.65 g (65 % yield) of the title compound.

1H-NMR (400MHz, DMSO-d6): δ [ppm]= -0.04 - 0.06 (m, 6H), 0.79 - 0.89 (m, 9H), 1.21 - 1.35 (m, 2H), 1.38 (s, 9H), 1.60 - 1.69 (m, 2H), 1.72 - 1.85 (m, 1H), 2.15 - 2.26 (m, 1H), 3.08 (dd, 1H), 3.12 - 3.22 (m, 1H), 3.25 - 3.31 (m, 1H), 3.53 (t, 2H), 3.60 - 3.70 (m, 4H).

### Intermediate 40

### (rac)-tert-butyl-3-(3-{[tert-butyl(dimethyl)silyl]oxy}propyl)-3-(3-methoxy-3-oxopropanoyl)pyrrolidine-1-carboxylate

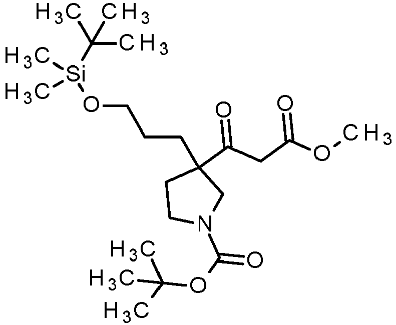

methyl acetate (790 µL, 10 mmol) was solubilised in THF (13 mL), cooled to -78°C and LiHMDS (10 mL, 1.0 M in THF, 10 mmol) was added dropwise. The mixture was stirred at - 78°C for 1 h and (rac)-1-tert-butyl 3-methyl-3-(3-{[tert-butyl(dimethyl)silyl]oxy}propyl)pyrrolidine-1,3-dicarboxylate (2.00 g, 4.98 mmol), solubilised in THF (2.9 mL), was added dropwise. It was allowed to warm up to rt overnight. The mixture was diluted with sat. NH4Cl solution, extracted with ethyl acetate/THF and the organic layer was dried with a silicone filter and evaporated. The residue was purified by flash chromatography to give 950 mg (43 % yield) of the title compound.

1H-NMR (400MHz, DMSO-d6): δ [ppm]= 0.02 (s, 6H), 0.79 - 0.90 (m, 9H), 1.14 - 1.31 (m, 2H), 1.38 (br d, 9H), 1.59 - 1.85 (m, 3H), 2.18 - 2.25 (m, 1H), 2.99 - 3.17 (m, 2H), 3.19 - 3.30 (m, 1H), 3.52 (t, 2H), 3.58 - 3.86 (m, 6H).

### Intermediate 41

### (rac)-tert-butyl-3-(3-{[tert-butyl(dimethyl)silyl]oxy}propyl)-3-(3-hydroxy-1H-pyrazol-5-yl)pyrrolidine-1-carboxylate

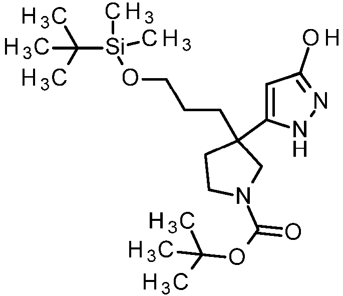

(rac)-tert-butyl-3-(3-{[tert-butyl(dimethyl)silyl]oxy}propyl)-3-(3-methoxy-3-oxopropanoyl)pyrrolidine-1-carboxylate (950 mg, 2.14 mmol) was solubilised in ethanol (910 µL), hydrazine hydrate (313 µL, 6.42 mmol) was added and the mixture was stirred 1 h at 80°C. It was evaporated and the residue was purified by flash chromatography to give 680 mg (75 % yield) of the title compound.
LC-MS (method 1): Rt = 1.09 min; MS (ESlpos): m/z = 426 [M+H]⁺
1H-NMR (400MHz, DMSO-d6): δ [ppm]= -0.02 (s, 6H), 0.81 - 0.86 (m, 9H), 1.10 - 1.32 (m, 2H), 1.38 (d, 9H), 1.60 (br t, 2H), 1.78 - 1.92 (m, 1H), 2.10 - 2.19 (m, 1H), 3.05 - 3.16 (m, 1H), 3.16 - 3.23 (m, 1H), 3.25 - 3.31 (m, 1H), 3.43 - 3.49 (m, 2H), 3.51 - 3.59 (m, 1H), 5.28 (s, 1H).

### Intermediate 42

### (rac)-tert-butyl-3-(3-hydroxypropyl)-3-(3-hydroxy-1H-pyrazol-5-yl)pyrrolidine-1-carboxylate

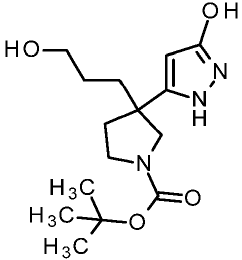

(rac)-tert-butyl-3-(3-{[tert-butyl(dimethyl)silyl]oxy}propyl)-3-(3-hydroxy-1H-pyrazol-5-yl)pyrrolidine-1-carboxylate (650 mg, 1.53 mmol) was solubilised in THF (3.3 mL) under nitrogen, cooled to 0°C, TBAF (1.8 mL, 1.0 M in THF, 1.8 mmol) was added and the mixture was stirred for 5 h at 0°C and was allowed to warm up to rt overnight. It was evaporated and the residue was purified by flash chromatography to give 490 mg of the title compound.
LC-MS (method 1): Rt = 0.54 min; MS (ESlpos): m/z = 312 [M+H]⁺
1H-NMR (400 MHz, DMSO-d6) δ [ppm]: 0.000 (0.28), 0.849 (0.41), 0.931 (0.76), 0.950 (2.08), 0.968 (0.93), 1.130 (0.31), 1.141 (0.40), 1.150 (0.44), 1.166 (0.50), 1.183 (0.44), 1.201 (0.42), 1.219 (0.56), 1.243 (0.49), 1.255 (0.39), 1.275 (0.24), 1.294 (0.29), 1.313 (0.42), 1.331 (0.43), 1.349 (0.32), 1.384 (16.00), 1.403 (15.99), 1.540 (0.18), 1.586 (1.14), 1.607 (1.50), 1.628 (0.80), 1.642 (0.32), 1.856 (0.44), 1.878 (0.29), 2.154 (0.44), 2.165 (0.42), 2.177 (0.40), 2.535 (1.46), 2.539 (0.95), 3.105 (0.35), 3.113 (0.38), 3.132 (0.67), 3.154 (0.71), 3.161 (0.73), 3.180 (0.90), 3.196 (0.84), 3.272 (1.25), 3.289 (2.31), 3.301 (1.67), 3.571 (0.92), 3.598 (0.82), 4.373 (0.51), 5.288 (0.93), 5.774 (4.98).

### Intermediate 43

### (rac)-tert-butyl-2-hydroxy-6,7-dihydro-5H-spiro[pyrazolo[1,5-a]pyridine-4,3'-pyrrolidine]-1'-carboxylate

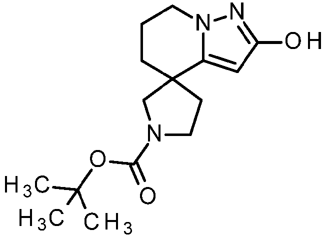

(rac)-tert-butyl-3-(3-hydroxypropyl)-3-(3-hydroxy-1H-pyrazol-5-yl)pyrrolidine-1-carboxylate (490 mg, 1.57 mmol) was solubilised in THF (10 mL), PPh3 (578 mg, 2.20 mmol) was added, DIAD (400 µL, 2.05 mmol) was added dropwise and the mixture was stirred for 2 h at rt. It was evaporated and purified by flash chromatography to give 300 mg (65 % yield) of the title compound.
LC-MS (method 1): Rt = 0.68 min; MS (ESlpos): m/z = 294 [M+H]⁺
1H-NMR (400 MHz, DMSO-d6) δ [ppm]: 1.394 (16.00), 1.414 (15.04), 1.696 (1.15), 1.708 (1.09), 1.727 (0.83), 1.907 (1.01), 1.931 (1.49), 1.944 (1.25), 2.012 (0.51), 2.040 (0.59), 2.337 (0.48), 2.518 (5.97), 2.523 (3.92), 3.262 (0.88), 3.289 (4.19), 3.312 (1.71), 3.364 (1.47), 3.378 (1.09), 3.770 (1.68), 3.785 (2.21), 3.800 (1.07), 3.815 (0.45), 5.246 (2.53), 5.254 (2.37), 5.759 (3.39), 9.471 (4.45).

### Intermediate 44

### (rac)-tert-butyl-2-[(trifluoromethanesulfonyl)oxy]-6,7-dihydro-5H-spiro[pyrazolo[1,5-a]pyridine-4,3'-pyrrolidine]-1'-carboxylate

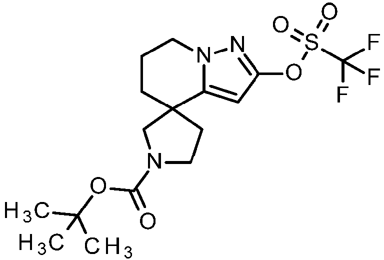

(rac)-tert-butyl-2-hydroxy-6,7-dihydro-5H-spiro[pyrazolo[1,5-a]pyridine-4,3'-pyrrolidine]-1'-carboxylate (300 mg, 1.02 mmol), 1,1,1-trifluoro-N-phenyl-N-[(trifluoromethyl)sulfonyl]methanesulfonamide (427 mg, 1.2 mmol) and N,N-diisopropylethylamine (530 µL, 3.1 mmol) were solubilised in THF (6.3 mL) and the mixture was stirred for 3 h at 60°C. It was diluted with brine, extracted 3x with ethyl acetate and the combined organic layers were dried with a silicone filter and evaporated. The residue was purified by preparative HPLC to give 310 mg (71 % yield) of the title compound.
LC-MS (method 1): Rt = 1.40 min; MS (ESlpos): m/z = 426 [M+H]⁺
1H-NMR (400MHz, DMSO-d6): δ [ppm]= 1.40 (d, 9H), 1.71 - 1.88 (m, 2H), 1.94 - 2.06 (m, 3H), 2.09 - 2.19 (m, 1H), 3.35 - 3.49 (m, 4H), 4.02 (t, 2H), 6.28 (s, 1H).

### Intermediate 45

### (rac)-6,7-dihydro-5H-spiro[pyrazolo[1,5-a]pyridine-4,3'-pyrrolidin]-2-yl trifluoromethanesulfonate

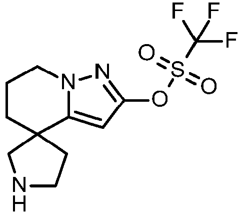

(rac)-tert-butyl-2-[(trifluoromethanesulfonyl)oxy]-6,7-dihydro-5H-spiro[pyrazolo[1,5-a]pyridine-4,3'-pyrrolidine]-1'-carboxylate (2.00 g, 4.70 mmol) was solubilised in dichloromethane (80 mL) and TFA (12 mL, 150 mmol) was added. The mixture was stirred for 1 h at rt. The mixture was concentrated under reduced pressure to give 3.20 g of the title compound, which was used without further purification.
LC-MS (method 1): Rt = 1.20 min; MS (ESlpos): m/z = 326 [M+H]⁺

### Intermediate 46

### (rac)-1'-(ethylcarbamoyl)-6,7-dihydro-5H-spiro[pyrazolo[1,5-a]pyridine-4,3'-pyrrolidin]-2-yl trifluoromethanesulfonate

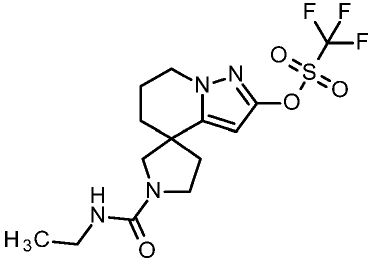

(rac)-6,7-dihydro-5H-spiro[pyrazolo[1,5-a]pyridine-4,3'-pyrrolidin]-2-yl trifluoromethanesulfonate (4.20 g, 12.9 mmol) and N,N-diisopropylethylamine (22 mL, 130 mmol) were solubilised in dichloromethane (310 mL), cooled to 0°C, isocyanatoethane (1.0 mL, 13 mmol) was added and the mixture was stirred overnight at rt. It was evaporated and the residue was purified by flash chromatography to give 3.05 g (60 % yield) of the title compound.
LC-MS (method 1): Rt = 1.08 min; MS (ESlpos): m/z = 397 [M+H]⁺
1H-NMR (400 MHz, DMSO-d6) δ [ppm]: 0.902 (0.46), 0.988 (4.24), 1.006 (9.82), 1.024 (4.54), 1.035 (1.52), 1.052 (3.03), 1.070 (1.39), 1.755 (0.65), 1.767 (1.23), 1.773 (1.15), 1.778 (1.14), 1.786 (1.29), 1.797 (0.79), 1.956 (0.41), 1.968 (0.58), 1.974 (0.67), 1.986 (1.27), 1.998 (1.26), 2.005 (1.16), 2.016 (1.24), 2.029 (0.66), 2.035 (0.63), 2.046 (0.47), 2.065 (0.86), 2.099 (0.47), 2.119 (0.96), 2.130 (0.42), 2.138 (0.56), 2.150 (0.66), 2.518 (0.50), 3.006 (0.59), 3.024 (1.87), 3.038 (2.03), 3.041 (2.01), 3.055 (1.82), 3.073 (0.54), 3.322 (1.52), 3.326 (1.39), 3.333 (16.00), 3.347 (3.28), 3.374 (2.59), 3.400 (1.22), 3.405 (0.81), 3.407 (0.74), 3.417 (1.02), 3.422 (1.24), 3.429 (0.70), 3.435 (1.11), 3.440 (0.93), 3.452 (0.94), 3.457 (0.40), 4.018 (1.54), 4.034 (3.27), 4.049 (1.48), 4.344 (0.50), 4.357 (0.97), 4.370 (0.47), 5.758 (12.91), 6.147 (0.68), 6.161 (1.32), 6.174 (0.66), 6.256 (5.96).

### Intermediate 47

### tert-butyl (3'S)-2-bromo-6,7-dihydro-1'H-spiro[pyrazolo[5,1-c][1,4]oxazine-4,3'-pyrrolidine]-1'-carboxylate

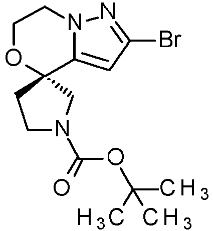

The racemate (*rac*)-tert-butyl-2-bromo-6,7-dihydrospiro[pyrazolo[5,1-c][1,4]oxazine-4,3'-pyrrolidine]-1'-carboxylate (5.00 g, 14.0 mmol) was separated by chrial HPLC to give 1.87 g (96 % purity, 37 % yield) of the target compound.

### Preparative method:

Instrument: Sepiatec: Prep SFC100; Column: Chiralpak IC 5µ 250x30mm; eluent A: CO2; eluent B: ethanol + 0.2 vol % aqueous ammonia (32%); isocratic: 10%B; flow: 100 mL/min; temperature: 40°C; BPR: 150bar; UV: 210 nm
Retention time: 9.5 - 13.0 min;
Analytical method:
   Instrument: Agilent: 1260, Aurora SFC-Modul; Column: Chiralpak IC 5µ 100x4.6mm; eluent A: CO2; eluent B: ethanol + 0.2 vol % aqueous ammonia (32%); isocratic: 10%B; flow: 4 mL/min; temperature: 37.5°C; BPR: 100bar; UV: 210 nm
   Retention time: 2,98 min
   [α]²⁰_{D} : -29.5° (c = 1.00, DMSO)

### Intermediate 48

### tert-butyl (3'R)-2-bromo-6,7-dihydro-1'H-spiro[pyrazolo[5,1-c][1,4]oxazine-4,3'-pyrrolidine]-1'-carboxylate

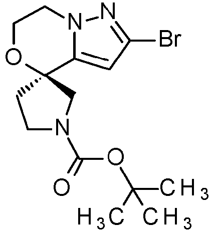

The racemate (*rac*)-tert-butyl-2-bromo-6,7-dihydrospiro[pyrazolo[5,1-c][1,4]oxazine-4,3'-pyrrolidine]-1'-carboxylate (5.00 g, 14.0 mmol) was separated by chrial HPLC to give 2.00 g (83 % purity, 40 % yield) of the target compound.

### Preparative method:

Instrument: Sepiatec: Prep SFC100; Column: Chiralpak IC 5µ 250x30mm; eluent A: CO2; eluent B: ethanol + 0.2 vol % aqueous ammonia (32%); isocratic: 10%B; flow: 100 mL/min; temperature: 40°C; BPR: 150bar; UV: 210 nm
Retention time: 15.5 - 22.0 min;
Analytcal method:
   Instrument: Agilent: 1260, Aurora SFC-Modul; Column: Chiralpak IC 5µ 100x4.6mm; eluent A: CO2; eluent B: ethanol + 0.2 vol % aqueous ammonia (32%); isocratic: 10%B; flow: 4 mL/min; temperature: 37.5°C; BPR: 100bar; UV: 210 nm
   Retention time: 4,84 min
   [α]²⁰_{D} : +27.1° (c = 1.00, DMSO)

### Intermediate 49

### (rac)-2-bromo-6,7-dihydrospiro[pyrazolo[5,1-c][1,4]oxazine-4,3'-pyrrolidine]-hydrochloride salt

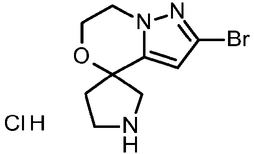

(rac)-tert-butyl-2-bromo-6,7-dihydrospiro[pyrazolo[5,1-c][1,4]oxazine-4,3'-pyrrolidine]-1'-carboxylate (2.00 g, 5.58 mmol) was solubilised in 1,4-dioxane (49 mL) and HCl (28 mL, 4.0 M in 1,4-dioxane, 112 mmol) was added. The mixture was stirred overnight at rt. It was concentrated under reduced pressure to give 1.70 g of the title compound.
LC-MS (method 1): Rt = 0.72 min; MS (ESlpos): m/z = 258 [M+H]⁺

### Intermediate 50

### (3'S)-2-bromo-6,7-dihydrospiro[pyrazolo[5,1-c][1,4]oxazine-4,3'-pyrrolidine] hydrochloride salt

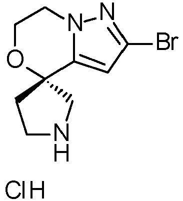

tert-butyl (3'S)-2-bromo-6,7-dihydrospiro[pyrazolo[5,1-c][1,4]oxazine-4,3'-pyrrolidine]-1'-carboxylate (300 mg, 837 µmol) was solubilised in 1,4-dioxane (10 mL) and treated with HCl in dioxane (4.2 mL, 4.0 M, 17 mmol). The reaction mixture was stirred for 18h at rt and then concentrated under reduced pressure to give 274 mg of the title compound that was used without further purification.
LC-MS (method 1): Rₜ = 0.70 min; MS (ESlpos): m/z = 260 [M+H]⁺
¹H-NMR (400 MHz, DMSO-d6) δ [ppm]: 2.249 (0.63), 2.260 (0.62), 2.277 (0.50), 2.287 (0.84), 2.311 (0.57), 2.418 (0.60), 2.437 (0.68), 2.453 (0.54), 2.471 (0.67), 2.518 (4.19), 2.523 (2.85), 3.294 (0.94), 3.442 (0.43), 3.468 (0.67), 3.565 (2.74), 3.644 (0.81), 3.674 (0.75), 4.110 (16.00), 6.553 (10.71).

### Intermediate 51

### (3'R)-2-bromo-6,7-dihydrospiro[pyrazolo[5,1-c][1,4]oxazine-4,3'-pyrrolidine]

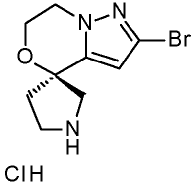

tert-butyl (3'R)-2-bromo-6,7-dihydrospiro[pyrazolo[5,1-c][1,4]oxazine-4,3'-pyrrolidine]-1'-carboxylate (300 mg, 837 µmol) was solubilised in 1,4-dioxane (10 mL) and treated with HCl in dioxane 1,4-dioxane (10 mL). The reaction mixture was stirred at rt for 18h and concentrated under reduced pressure to give 267 mg of the title compound that was used without further purification
LC-MS (method 1): Rₜ = 0.70 min; MS (ESlpos): m/z = 260 [M+H]⁺
¹H-NMR (400 MHz, DMSO-d6) δ [ppm]: 2.250 (0.59), 2.260 (0.57), 2.276 (0.45), 2.287 (0.80), 2.311 (0.54), 2.420 (0.56), 2.433 (0.44), 2.438 (0.63), 2.455 (0.47), 2.460 (0.43), 2.518 (3.13), 2.523 (2.12), 3.292 (0.79), 3.309 (0.85), 3.323 (0.99), 3.468 (0.49), 3.565 (3.49), 3.651 (0.53), 3.675 (0.46), 4.111 (16.00), 6.551 (9.85).

### Intermediate 52

### (rac)-2-bromo-N-ethyl-6,7-dihydrospiro[pyrazolo[5,1-c][1,4]oxazine-4,3'-pyrrolidine]-1'-carboxamide

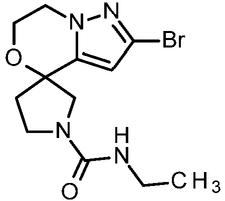

(rac)-2-bromo-6,7-dihydrospiro[pyrazolo[5,1-c][1,4]oxazine-4,3'-pyrrolidine] (1.70 g, 6.59 mmol) was solubilised in dichloromethane (160 mL), N,N-diisopropylethylamine (11 mL, 66 mmol) was added and the mixture was cooled to 0°C. isocyanatoethane (520 µL, 6.6 mmol) was added and it was stirred overnight under argon at rt. The mixture was evaporated and the residue was purified by flash chromatography to give 2.2 g of the title compound.
LC-MS (method 1): Rt = 0.80 min; MS (ESlpos): m/z = 329 [M+H]⁺
1H-NMR (400 MHz, DMSO-d6) δ [ppm]: 0.902 (0.84), 0.920 (0.42), 0.990 (4.71), 1.008 (10.94), 1.026 (5.00), 1.035 (8.72), 1.053 (16.00), 1.070 (8.50), 1.138 (0.53), 1.154 (0.52), 2.066 (1.11), 2.184 (0.40), 2.190 (0.56), 2.213 (0.72), 2.216 (0.74), 2.239 (0.53), 2.264 (0.67), 2.281 (0.76), 2.518 (1.19), 2.523 (0.77), 3.004 (0.60), 3.022 (1.97), 3.036 (2.10), 3.039 (2.10), 3.053 (1.94), 3.071 (0.58), 3.275 (0.41), 3.292 (0.60), 3.300 (0.86), 3.316 (0.93), 3.342 (0.66), 3.358 (2.07), 3.386 (2.37), 3.404 (1.21), 3.417 (1.24), 3.422 (4.11), 3.435 (4.20), 3.440 (3.64), 3.452 (3.84), 3.457 (1.41), 3.469 (1.73), 3.488 (1.02), 3.510 (0.50), 3.715 (1.17), 3.741 (0.97), 3.744 (0.98), 4.023 (0.81), 4.040 (1.17), 4.064 (2.93), 4.069 (1.92), 4.075 (4.13), 4.100 (0.83), 4.343 (2.56), 4.355 (5.05), 4.368 (2.49), 5.758 (14.96), 6.150 (0.64), 6.163 (1.27), 6.176 (0.63), 6.465 (9.62).

### Intermediate 53

### (3'S)-2-bromo-N-ethyl-6,7-dihydro-1'H-spiro[pyrazolo[5,1-c][1,4]oxazine-4,3'-pyrrolidine]-1'-carboxamide

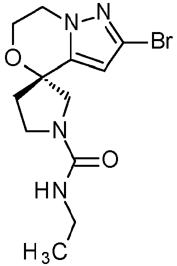

N,N-diisopropylethylamine (1.4 mL, 8.3 mmol) was added to a suspension of to a suspension (3'S)-2-bromo-6,7-dihydrospiro[pyrazolo[5,1-c][1,4]oxazine-4,3'-pyrrolidine] hydrochloride salt) (245 mg, 832 µmol) in dichloromethane (10 mL). The reaction mixture was cooled to 0°C and ethylisocyanate (66 µl, 830 µmol) was added and the reaction was stirred for 18h. The reaction mixture was then concentrated under reduced pressure and purified by flash column chromatography to give 233 mg (95 % purity, 81 % yield)
LC-MS (method 1): Rₜ = 0.79 min; MS (ESlpos): m/z = 331 [M+H]⁺
¹H-NMR (400 MHz, DMSO-d6) δ [ppm]: 0.990 (6.48), 1.008 (16.00), 1.026 (6.98), 2.180 (0.55), 2.184 (0.54), 2.190 (0.78), 2.207 (0.52), 2.213 (0.98), 2.217 (1.01), 2.239 (0.71), 2.265 (0.92), 2.281 (1.04), 2.297 (0.53), 2.314 (0.46), 2.518 (1.42), 2.523 (1.05), 3.004 (0.83), 3.022 (2.70), 3.036 (2.90), 3.039 (2.84), 3.053 (2.65), 3.071 (0.78), 3.159 (4.45), 3.172 (5.03), 3.275 (0.55), 3.292 (0.82), 3.300 (1.16), 3.317 (1.28), 3.343 (0.81), 3.358 (2.84), 3.386 (3.21), 3.468 (0.90), 3.488 (1.36), 3.509 (0.66), 3.712 (1.55), 3.715 (1.55), 3.741 (1.33), 3.744 (1.31), 4.008 (0.53), 4.023 (1.12), 4.040 (1.63), 4.060 (2.63), 4.064 (4.03), 4.069 (2.66), 4.076 (5.70), 4.083 (2.67), 4.095 (1.61), 4.100 (1.16), 4.109 (1.36), 4.122 (0.42), 6.150 (0.88), 6.164 (1.73), 6.177 (0.86), 6.466 (13.96).

### Intermediate 54

### (3'R)-2-bromo-N-ethyl-6,7-dihydro-1'H-spiro[pyrazolo[5,1-c][1,4]oxazine-4,3'-pyrrolidine]-1'-carboxamide

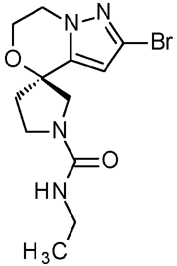

N,N-diisopropylethylamine (1.4 mL, 8.3 mmol) was added to a suspension of (3'R)-2-bromo-6,7-dihydrospiro[pyrazolo[5,1-c][1,4]oxazine-4,3'-pyrrolidine] hydrochloride salt (enantiomer 2) (245 mg, 832 µmol) in dichloromethane (10 mL) under argon. The reaction was cooled to 0°C and ethylisocyanate (66 µl, 830 µmol) was added. The reaction miyture was stirred at rt for 18h, concentrated under reduced pressure and purified by flash column chromatography to give 214 mg (96 % purity, 75 % yield) of the title compound.
LC-MS (method 1): Rₜ = 0.79 min; MS (ESlpos): m/z = 331 [M+H]⁺
¹H-NMR (400 MHz, DMSO-d6) δ [ppm]: 0.990 (6.47), 1.008 (16.00), 1.026 (7.01), 2.179 (0.55), 2.184 (0.56), 2.190 (0.77), 2.206 (0.52), 2.213 (0.98), 2.217 (1.02), 2.239 (0.72), 2.264 (0.92), 2.281 (1.05), 2.297 (0.53), 2.314 (0.49), 2.518 (2.18), 2.523 (1.67), 3.004 (0.84), 3.022 (2.73), 3.036 (2.91), 3.039 (2.87), 3.053 (2.68), 3.071 (0.78), 3.159 (3.54), 3.172 (3.71), 3.275 (0.58), 3.292 (0.87), 3.300 (1.18), 3.317 (1.34), 3.342 (0.83), 3.358 (2.86), 3.386 (3.29), 3.467 (0.90), 3.487 (1.36), 3.509 (0.64), 3.712 (1.55), 3.715 (1.55), 3.740 (1.35), 3.744 (1.31), 4.008 (0.54), 4.023 (1.14), 4.040 (1.64), 4.060 (2.64), 4.064 (4.07), 4.069 (2.63), 4.076 (5.69), 4.095 (1.16), 4.100 (1.14), 4.108 (0.93), 6.149 (0.89), 6.163 (1.74), 6.177 (0.85), 6.466 (13.75).

### Intermediate 55

### (3R)-1-{[2-(pyridin-2-yl)propan-2-yl]carbamoyl}-5',6'-dihydrospiro[pyrrolidine-3,4'-pyrrolo[1,2-b]pyrazol]-2'-yl trifluoromethanesulfonate

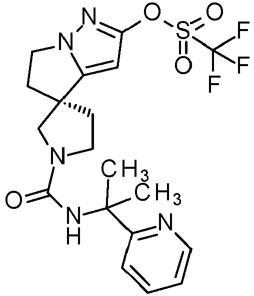

A solution of 2-(pyridin-2-yl)propan-2-amine (121 mg, 889 µmol), CDI (157 mg, 970 µmol) and DIPEA (560 µl, 3.2 mmol) in DMF (6mL) was stirred at RT for 1h. (3R)-5',6'-dihydrospiro[pyrrolidine-3,4'-pyrrolo[1,2-b]pyrazol]-2'-yl trifluoromethanesulfonate hydrogen chloride (281 mg, 808 µmol) in DMF (2mL) was added and the mixture was stirred at RT overnight. The mixture was diluted with water and then extracted with Ethyl acetate 2x. The combined organics were washed with Brine, dried over Sodium sulfate, filtered and concentrated in vacuo to give 484 mg of the title compound.
LC-MS (Method 1): Rt = 1.19 min; MS (ESlpos): m/z = 475 [M+H]⁺
¹H-NMR (400 MHz, DMSO-d6) δ [ppm]: 1.493 (2.66), 1.573 (2.70), 1.578 (2.64), 2.482 (0.67), 2.523 (0.40), 2.728 (13.70), 2.888 (16.00), 3.478 (1.16), 4.241 (0.56), 6.258 (1.58), 6.424 (0.63), 7.436 (0.43), 7.456 (0.48), 7.634 (0.50), 7.951 (2.07), 8.448 (0.43), 8.460 (0.43).

### Intermediate 56

### (3'R)-2-bromo-N-[2-(pyridin-2-yl)propan-2-yl]-6,7-dihydrospiro[pyrazolo[5,1-c][1,4]oxazine-4,3'-pyrrolidine]-1'-carboxamide

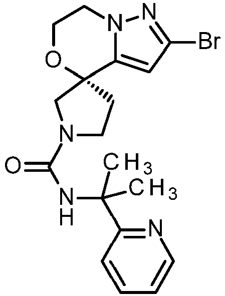

A solution of 2-(pyridin-2-yl)propan-2-amine (157 mg, 1.15 mmol), CDI (204 mg, 1.26 mmol) and DIPEA (730 µl, 4.2 mmol) in DMF (8mL) was stirred at RT for 1h. (3'R)-2-bromo-6,7-dihydrospiro[pyrazolo[5,1-c][1,4]oxazine-4,3'-pyrrolidine] hydrogen chloride (309 mg, 1.05 mmol) in DMF (3mL) was added and the mixture was stirred at RT overnight. The mixture was diluted with water and then extracted with Ethyl acetate 2x. The combined organics were washed with Brine, dried over Sodium sulfate, filtered and concentrated. The crude was purified by column chromatography to give 282 mg (64 % yield) of the title compound.
LC-MS (Method 1): Rt = 0.98 min; MS (ESlpos): m/z = 421 [M+H]⁺
¹H-NMR (400 MHz, DMSO-d6) δ [ppm]: 1.154 (3.10), 1.172 (5.84), 1.190 (3.02), 1.492 (16.00), 1.569 (10.95), 1.576 (10.63), 1.988 (11.74), 2.228 (0.46), 2.253 (0.69), 2.276 (0.58), 2.287 (0.68), 2.303 (0.77), 2.318 (0.92), 2.322 (1.50), 2.327 (1.88), 2.332 (1.49), 2.336 (0.83), 2.518 (6.55), 2.523 (4.43), 2.660 (0.54), 2.664 (1.13), 2.669 (1.63), 2.673 (1.17), 2.678 (0.52), 2.729 (9.88), 2.889 (11.18), 3.357 (0.41), 3.384 (0.60), 3.401 (0.63), 3.438 (0.90), 3.466 (1.03), 3.551 (0.52), 3.570 (0.83), 3.759 (1.25), 3.786 (1.11), 4.000 (0.98), 4.017 (2.83), 4.035 (2.85), 4.053 (1.15), 4.062 (0.72), 4.087 (7.24), 4.114 (0.81), 6.411 (2.62), 6.496 (8.35), 6.504 (0.53), 6.575 (1.73), 7.035 (0.63), 7.136 (0.73), 7.139 (0.81), 7.148 (0.75), 7.151 (0.87), 7.155 (0.85), 7.157 (0.78), 7.167 (0.89), 7.169 (0.91), 7.172 (1.20), 7.175 (1.14), 7.184 (1.05), 7.187 (1.22), 7.191 (1.20), 7.193 (1.15), 7.203 (1.14), 7.206 (1.19), 7.393 (0.76), 7.396 (1.37), 7.399 (0.83), 7.414 (0.93), 7.416 (1.58), 7.419 (0.95), 7.426 (1.16), 7.428 (2.04), 7.431 (1.24), 7.446 (1.30), 7.449 (2.20), 7.648 (0.80), 7.652 (0.89), 7.666 (0.94), 7.670 (0.92), 7.686 (0.65), 7.691 (0.67), 7.701 (1.19), 7.705 (1.14), 7.721 (1.34), 7.725 (1.37), 7.739 (0.93), 7.744 (0.85), 7.951 (1.39), 8.442 (0.89), 8.445 (1.05), 8.448 (1.84), 8.450 (1.95), 8.452 (1.73), 8.454 (2.04), 8.460 (1.85), 8.462 (1.89), 8.464 (1.44), 8.467 (1.17).

### Intermediate 57

### ethyl 2-(3-chloropyridin-4-yl)-2-methylpropanoate

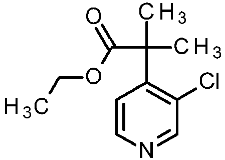

3-Chloropyridine-4-carbonitrile (500 mg, 3.61 mmol) in THF (12 mL) was cooled to -78°C under nitrogen atmosphere then ethyl 2-methylpropanoate (419 mg, 3.61 mmol) was added and potassium bis(trimethylsilyl)amide (7.9 mL, 0.50 M in toluene, 4.0 mmol) was added dropwise, the mixture was stirred 10 minutes before removing the cooling bath and stirred 1h at rt. The reaction was quenched with saturated ammonium chloride solution and extracted three times with dichloromethane. The combined organic phases were dried over sodium sulfate and concentrated. The residue was purified by flash chromatography to afford 300 mg (36 % yield) of the title compound.

### Intermediate 58

### 2-(3-chloropyridin-4-yl)-2-methylpropanoic acid

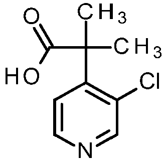

To a stirred solution of ethyl 2-(3-chloropyridin-4-yl)-2-methylpropanoate (300 mg, 1.32 mmol) in methanol (8.5 mL) and THF (26 mL) was added lithium hydroxide (5.3 mL, 1.0 M, 5.3 mmol) and stirred overnight at rt. Lithium hydroxide (2.8 mL, 1.0 M, 2.8 mmol) was added to the mixture and stirred overnight at rt, then the mixture was heated for 4h at 40°C and then overnight at 50°C. Potassium hydroxide (1.3 mL, 1.0 M, 1.3 mmol) was added and stirred 4h at 50°C and then 72h at rt. Potassium hydroxide (1.3 mL, 1.0 M, 1.3 mmol) was added again and stirred 2 days at 50°C. The reaction mixture was divided in two portions and stirred in the microwave at 100°C for 1h. The reaction mixture was diluted with water, evaporated from organic solvents and extracted three times with dichloromethane. The aqueous phase was acidified to pH 2-3 using hydrochloric acid (9.5 mL, 1M) and extracted with dichloromethane (+10% ethanol). The combined organic phases were concentrated to give 170 mg (64 % yield) of the title compound.

### Intermediate 59

### 3-chloro-4-(2-isocyanatopropan-2-yl)pyridine

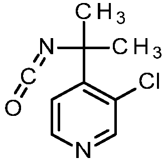

To a stirred solution of 2-(3-chloropyridin-4-yl)-2-methylpropanoic acid (170 mg, 852 µmol) in dioxane (2.8 mL) and triethylamine (150 µL) was added diphenyl phosphorazidate (240 µL, 1.1 mmol) and stirred at rt overnight. The reaction mixture was used in the next step without purification.

### Intermediate 60

### 5-fluoro-2-(2-isocyanatopropan-2-yl)pyridine

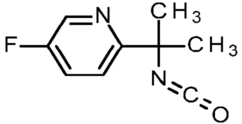

2-(5-fluoropyridin-2-yl)-2-methylpropanoic acid (126 mg, 686 µmol) was solubilised in 1,4-dioxane (2.3 mL), triethylamine (120 µL) and diphenyl phosphorazidate (190 µL, 890 µmol) were added and the mixture was stirred overnight at rt. The reaction mixture was used without further purification.
LC-MS (method 1): Rt = 0.75 min; MS (ESlpos): m/z = 181 [M+H]⁺

### Intermediate 61

### 1,3-difluoro-5-(2-isocyanatopropan-2-yl)benzene

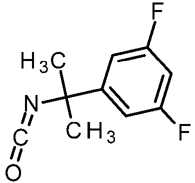

Intermediate 61 was prepared in analogy to Intermediate 60 using 2-(3,5-difluorophenyl)-2-methylpropanoic acid (100 mg, 500 µmol).

### Intermediate 62

### 5-(2-isocyanatopropan-2-yl)-2-methylpyridine

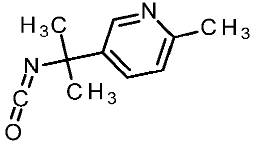

Intermediate 62 was prepared in analogy to Intermediate 60 using 2-methyl-2-(6-methylpyridin-3-yl)propanoic acid (100 mg, 558 µmol).
LC-MS (method 1): Rt = 1.01 min; MS (ESlpos): m/z = 177 [M+H]⁺

### Intermediate 63

### (1-isocyanatocyclohexyl)benzene

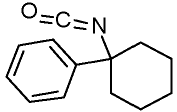

Intermediate 63 was prepared in analogy to Intermediate 60 using 1-phenylcyclohexane-1-carboxylic acid (100 mg, 490 µmol).

### Intermediate 64

### [(1S)-2,2,2-trifluoro-1-isocyanato-1-methoxyethyl]benzene

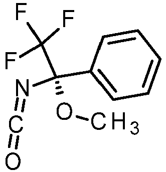

Intermediate 64 was prepared in analogy to Intermediate 60 using (2R)-3,3,3-trifluoro-2-methoxy-2-phenylpropanoic acid (120 mg, 512 µmol).
LC-MS (method 1): Rt = 0.96 min; MS (ESlpos): m/z = 234 [M+H]⁺

### Intermediate 65

### 4-(2-isocyanatopropan-2-yl)pyrimidine

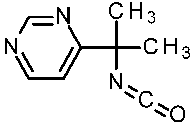

Intermediate 65 was prepared in analogy to Intermediate 60 using 2-methyl-2-(pyrimidin-4-yl)propanoic acid (114 mg, 686 µmol).
LC-MS (method 1): Rt = 0.85 min; MS (ESlpos): m/z = 164 [M+H]⁺

### Intermediate 66

### 5-(2-isocyanatopropan-2-yl)pyrimidine

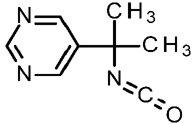

Intermediate 66 was prepared in analogy to Intermediate 60 using 2-methyl-2-(pyrimidin-5-yl)propanoic acid (120 mg, 722 µmol).
LC-MS (method 1): Rt = 0.80 min; MS (ESlpos): m/z = 164 [M+H]⁺

### Intermediate 67

### 3-isocyanato-3-phenyloxetane

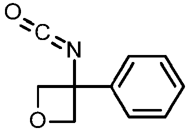

Intermediate 67 was prepared in analogy to Intermediate 60 using 3-phenyloxetane-3-carboxylic acid (100 mg, 561 µmol).
LC-MS (method 1): Rt = 1.04 min; MS (ESlpos): m/z = 176 [M+H]⁺

### Intermediate 68

### tert-butyl [3-(trifluoroacetyl)pyridin-2-yl]carbamate

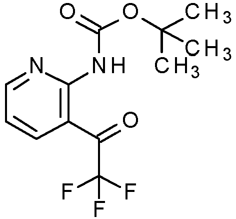

tert-butyl pyridin-2-ylcarbamate (3.00 g, 15.4 mmol) and N,N,N',N'-Tetramethylethylenediamine (5.8 mL, 39 mmol) were solubilised in THF (150 mL) under argon, cooled to -78°C, BuLi (24 mL, 1.6 M, 39 mmol) was added drowise and the mixture was stirred for 30 min at 0°C. The mixture was cooled to -78°C, ethyl trifluoroacetate (2.5 mL, 23 mmol) was added and it was stirred for 1 h at 0°C. It was diluted with sat. NH4Cl solution and extracted 3x with ethyl acetate. The combined organic layers were dried and evaporated. The residue was stirred in hexane, filtered and the solid was dried under reduced pressure at 40°C to give 2.40 g (54 % yield) of the title compound.
LC-MS (method 1): Rt = 0.64 min; MS (ESlneg): m/z = 289 [M-H]⁻
¹H-NMR (400 MHz, DMSO-d6) δ [ppm]: 1.406 (0.41), 1.426 (16.00), 1.433 (2.41), 1.466 (0.56), 1.494 (0.53), 2.445 (0.50), 7.289 (0.63), 7.301 (0.65), 7.308 (0.70), 7.320 (0.70), 7.921 (0.49), 7.940 (0.43), 8.546 (0.68), 8.551 (0.72), 8.558 (0.71), 8.563 (0.68), 10.635 (0.62).

### Intermediate 69

### 1-(2-aminopyridin-3-yl)-2,2,2-trifluoroethan-1-one-hydrogen chloride (1/1)

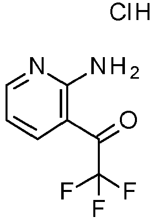

tert-butyl [3-(trifluoroacetyl)pyridin-2-yl]carbamate (2.40 g, 80 % purity, 6.62 mmol) was solubilised in 1,4-dioxane (25 mL), HCl (17 mL, 4.0 M in dioxane, 66 mmol) was added and the mixture was stirred over night at rt. It was concentrated under reduced pressure to give 1.37 g (91 % yield) of the title compound, which was used without further purification.
LC-MS (method 1): Rt = 0.84 min; MS (ESlneg): m/z = 189 [M-H]⁻
¹H-NMR (400 MHz, DMSO-d6) δ [ppm]: 0.884 (0.98), 0.899 (1.36), 0.917 (2.66), 0.936 (1.13), 1.154 (0.69), 1.172 (1.38), 1.190 (0.81), 1.325 (0.53), 1.343 (0.61), 1.362 (0.56), 1.499 (8.43), 1.517 (0.87), 1.709 (0.45), 1.987 (2.50), 2.336 (1.33), 2.518 (16.00), 2.523 (10.31), 2.678 (1.41), 2.831 (10.97), 3.483 (3.32), 3.565 (3.19), 3.677 (0.42), 4.017 (0.59), 4.034 (0.59), 6.741 (14.18), 6.752 (13.60), 6.761 (13.96), 6.772 (14.85), 6.946 (3.11), 6.961 (4.04), 6.964 (3.88), 6.980 (3.58), 6.994 (0.63), 7.307 (0.53), 7.328 (0.52), 7.407 (0.40), 7.649 (1.47), 7.924 (0.48), 7.940 (0.53), 8.021 (1.68), 8.026 (4.85), 8.031 (7.30), 8.037 (5.67), 8.041 (3.61), 8.047 (6.17), 8.052 (9.02), 8.057 (8.79), 8.068 (7.57), 8.090 (5.72), 8.095 (7.38), 8.106 (5.18), 8.110 (6.30), 8.116 (4.14), 8.135 (3.43), 8.408 (10.48), 8.413 (11.25), 8.419 (11.04), 8.424 (10.49), 8.536 (0.70), 8.550 (0.64), 8.610 (9.61), 8.758 (0.43).

### Intermediate 70

### (rac)-2-nitro-3-{[1-phenylethyl]sulfanyl}pyridine

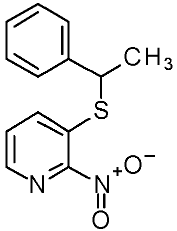

3-fluoro-2-nitropyridine (50.0 mg, 352 µmol), (rac)-1-phenylethane-1-thiol (50.1 mg, 362 µmol) and Cs2CO3 (126 mg, 387 µmol) were stirred in DMSO (500 µL) for 1 h at 65°C. The mixture was diluted with water and filtered. The precipitate was washed with water and dried under reduced pressure at 50°C to give 61.0 mg (86 % purity, 57 % yield) of the title compound, which was used without further purification.
¹H-NMR (400MHz, DMSO-d6): δ [ppm]= 1.59 (d, 3H), 4.92 (q, 1H), 7.21 - 7.27 (m, 1H), 7.29 - 7.34 (m, 2H), 7.41 - 7.46 (m, 2H), 7.71 (dd, 1H), 8.29 (dd, 1H), 8.36 (dd, 1H).
LC-MS (method 1): Rt = 1.27 min; MS (ESlpos): m/z = 261 [M+H]⁺

### Intermediate 71

### 5-bromo-3-[(cyclohexylmethyl)sulfanyl]-2-nitropyridine

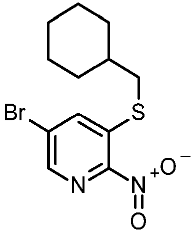

5-bromo-3-fluoro-2-nitropyridine (800 mg, 3.62 mmol), cyclohexylmethanethiol (486 mg, 3.73 mmol) and Na2CO3 (422 mg, 3.98 mmol) were stirred in DMSO (5.1 mL) for 1 h at 65°C. The mixture was diluted with water, stirred for 30 min at rt and extracted 2x with DCM. The combined organic layers were washed with water, dried over a silicone filter and concentrated under reduced pressure to give 1.32 g (84 % purity, 92 % yield) of the title compound, which was used without further purification.
¹H NMR (400 MHz, DMSO-d6) δ ppm 0.97 - 1.28 (m, 8 H) 1.49 - 1.72 (m, 6 H) 1.75 - 1.85 (m, 3 H) 3.06 (d, 2 H) 8.39 (d, 1 H) 8.52 (d, 1 H)
LC-MS (method 1): Rt = 1.56 min; MS (ESlpos): m/z = 331 [M+H]⁺

### Intermediate 72

### 3-(benzylsulfanyl)-5-bromo-2-nitropyridine

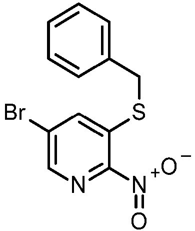

5-bromo-3-fluoro-2-nitropyridine (100 mg, 453 µmol), phenylmethanethiol (57.9 mg, 466 µmol) and CsHCO3 (96.5 mg, 498 µmol) were stirred in DMSO (640 µL) for 2 h at 65°C The mixture was diluted with water and filtered. The precipitate was washed with water and dried under reduced pressure at 50°C to give 110 mg (93 % purity, 70 % yield) of the title compound, which was used without further purification.
¹H-NMR (500MHz, DMSO-d6): δ [ppm]= 4.49 (s, 2H), 7.27 - 7.32 (m, 1H), 7.33 - 7.38 (m, 2H), 7.41 - 7.45 (m, 2H), 8.50 (d, 1H), 8.54 (d, 1H).
LC-MS (method 1): Rt = 1.35 min; MS (ESlpos): m/z = 325 [M+H]⁺

### Intermediate 73

### (rac)-5-bromo-3-{[1-cyclohexylethyl]sulfanyl}-2-nitropyridine

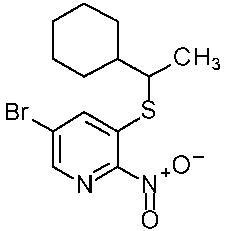

Intermediate 73 was prepared in analogy to Intermediate 71 using 5-bromo-3-fluoro-2-nitropyridine (300 mg, 1.36 mmol) and (rac)-1-cyclohexylethane-1-thiol (202 mg, 1.40 mmol).
LC-MS (method 1): Rt = 1.62 min; MS (ESlpos): m/z = 345 [M+H]⁺
¹H-NMR (400 MHz, DMSO-d6) δ [ppm]: 1.091 (0.42), 1.107 (0.42), 1.121 (0.47), 1.129 (0.59), 1.157 (0.71), 1.189 (0.48), 1.202 (0.45), 1.206 (0.44), 1.215 (3.06), 1.232 (3.03), 1.302 (0.46), 1.319 (0.46), 1.705 (0.58), 1.729 (0.60), 2.518 (0.45), 2.540 (16.00), 8.528 (0.67), 8.533 (1.74), 8.538 (2.17), 8.543 (0.89).

### Intermediate 74

### 5-bromo-3-[(cyclopentylmethyl)sulfanyl]-2-nitropyridine

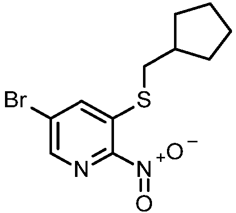

Intermediate 74 was prepared in analogy to Intermediate 71 using 5-bromo-3-fluoro-2-nitropyridine (500 mg, 2.26 mmol) and cyclopentylmethanethiol (271 mg, 2.33 mmol).
LC-MS (method 1): Rt = 1.49 min; MS (ESlpos): m/z = 317 [M+H]⁺
1H-NMR (400 MHz, DMSO-d6) δ [ppm]: 1.176 (0.83), 1.193 (1.95), 1.207 (2.14), 1.211 (2.35), 1.223 (2.93), 1.225 (2.97), 1.228 (3.23), 1.242 (2.67), 1.255 (2.21), 1.266 (1.60), 1.275 (2.94), 1.285 (2.78), 1.293 (3.28), 1.305 (3.58), 1.324 (3.05), 1.342 (1.37), 1.453 (0.57), 1.473 (1.59), 1.483 (2.72), 1.491 (3.54), 1.492 (3.60), 1.502 (5.52), 1.510 (5.66), 1.521 (6.97), 1.529 (4.30), 1.541 (4.72), 1.549 (3.20), 1.563 (4.25), 1.568 (3.28), 1.574 (2.74), 1.579 (3.64), 1.584 (3.80), 1.592 (3.27), 1.601 (3.66), 1.608 (4.54), 1.613 (3.40), 1.625 (4.47), 1.637 (3.28), 1.646 (2.22), 1.665 (0.79), 1.675 (0.50), 1.716 (1.14), 1.722 (1.11), 1.725 (1.32), 1.732 (2.17), 1.735 (1.94), 1.746 (3.20), 1.749 (2.70), 1.765 (3.03), 1.776 (3.40), 1.794 (3.54), 1.807 (3.59), 1.826 (3.07), 1.838 (2.22), 1.854 (1.04), 2.041 (1.16), 2.060 (2.64), 2.079 (3.47), 2.098 (2.96), 2.119 (3.39), 2.139 (3.87), 2.157 (2.77), 2.176 (1.16), 2.518 (2.48), 2.523 (1.61), 2.539 (13.08), 2.710 (16.00), 2.729 (15.06), 3.116 (1.92), 3.134 (2.13), 3.143 (11.40), 3.161 (11.09), 3.194 (2.76), 3.212 (2.81), 3.221 (2.23), 3.239 (2.15), 3.245 (0.65), 3.264 (0.50), 3.572 (0.40), 7.853 (1.18), 7.858 (1.22), 8.056 (0.93), 8.061 (0.91), 8.079 (0.91), 8.085 (0.96), 8.234 (0.43), 8.240 (0.42), 8.251 (1.64), 8.256 (1.64), 8.405 (7.33), 8.410 (8.22), 8.492 (1.01), 8.494 (1.13), 8.496 (1.15), 8.499 (1.00), 8.519 (9.61), 8.523 (8.30).

### Intermediate 75

### 5-bromo-3-(methylsulfanyl)pyridin-2-amine

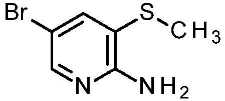

5-bromo-3-iodopyridin-2-amine (2.00 g, 6.69 mmol), sodium methanethiolate (2.81 g, 40.14 mmol) Cul (762 mg, 4.01 mmol) and ethylene glycol (660 µL, 12.06 mmol) were stirred in 2-propanol (16 mL) at 100°C for 3 d. The mixture was evaporated and the residue was purified by flash chromatography to give 680 mg (46 % yield) of the title compound.
LC-MS (method 1): Rt = 0.99 min; MS (ESlpos): m/z = 219 [M+H]⁺
¹H-NMR (400MHz, DMSO-d₆): δ [ppm]= 2.42 (s, 3H), 6.13 (s, 2H), 7.53 (d, 1H), 7.86 (d, 1H).

### Intermediate 76

### 5-bromo-3-(phenylsulfanyl)pyridin-2-amine

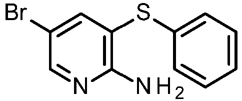

Intermediate 76 was prepared in analogy to Intermediate 75 using 5-bromo-3-iodopyridin-2-amine (2.00 g, 6.69 mmol) and sodium benzenethiolate (1.77 g, 13.4 mmol).
LC-MS (method 1): Rt = 1.29 min; MS (ESlpos): m/z = 281 [M+H]⁺
1H-NMR (400 MHz, DMSO-d6) δ [ppm]: 2.021 (0.98), 2.518 (0.94), 2.522 (0.61), 4.188 (0.57), 6.423 (11.24), 7.188 (1.18), 7.193 (8.74), 7.196 (11.55), 7.209 (3.30), 7.214 (16.00), 7.217 (12.99), 7.225 (3.34), 7.228 (1.85), 7.238 (2.46), 7.243 (7.65), 7.248 (2.13), 7.258 (3.91), 7.261 (5.77), 7.265 (2.69), 7.317 (2.11), 7.322 (11.43), 7.326 (4.45), 7.339 (7.78), 7.342 (14.16), 7.346 (3.36), 7.356 (2.37), 7.360 (5.68), 7.365 (1.20), 7.769 (13.05), 7.774 (13.64), 7.967 (0.49), 8.014 (0.52), 8.019 (0.44), 8.090 (14.79), 8.096 (14.91).

### Intermediate 77

### 5-bromo-2-nitro-3-[(1R)-1-(pyridin-3-yl)ethoxy]pyridine

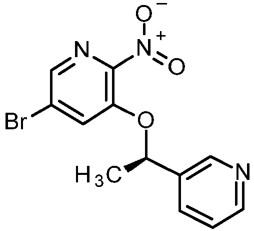

5-bromo-2-nitropyridin-3-ol (150 mg, 685 µmol), (1S)-1-(pyridin-3-yl)ethan-1-ol (84.4 mg, 685 µmol) and PPh3 (234 mg, 890 µmol) were stirred in THF (6.0 mL) for 1 h at rt. The mixture was cooled to 0°C, DIAD (180 µL, 890 µmol) was added and the mixture was stirred overnight at rt. It was evaporated and purified by preparative HPLC to give 70.0 mg (92 % purity, 29 % yield) of the title compound.
1H-NMR (400MHz, DMSO-d6): δ [ppm]= 1.62 (d, 3H), 6.05 (q, 1H), 7.44 (ddd, 1H), 7.83 (dt, 1H), 8.26 (d, 1H), 8.36 (d, 1H), 8.54 (dd, 1H), 8.67 (d, 1H).
LC-MS (method 2): Rt = 0.89 min; MS (ESlpos): m/z = 326 [M+H]⁺

### Intermediate 78

### 5-bromo-2-nitro-3-[(1S)-1-(pyridin-3-yl)ethoxy]pyridine

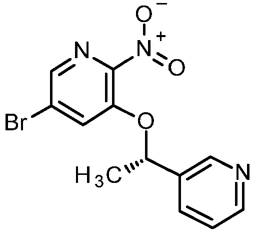

Intermediate 78 was prepared in analogy to Intermediate 77 using 5-bromo-2-nitropyridin-3-ol (250 mg, 1.14 mmol) and (1R)-1-(pyridin-3-yl)ethan-1-ol (155 mg, 1.26 mmol).
LC-MS (method 1): Rt = 0.92 min; MS (ESlpos): m/z = 326 [M+H]⁺

### Intermediate 79

### 5-bromo-2-nitro-3-[(1R)-1-(pyridin-4-yl)ethoxy]pyridine

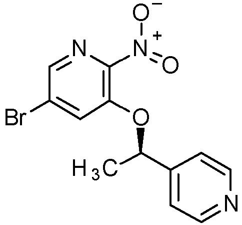

Intermediate 79 was prepared in analogy to Intermediate 77 using 5-bromo-2-nitropyridin-3-ol (75.0 mg, 342 µmol) and (1R)-1-(pyridin-4-yl)ethan-1-ol (42.2 mg, 342 µmol).
LC-MS (method 2): Rt = 0.88 min; MS (ESlpos): m/z = 326 [M+H]⁺

### Intermediate 80

### 5-bromo-2-nitro-3-[(1S)-1-(pyridin-4-yl)ethoxy]pyridine

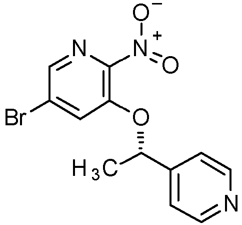

Intermediate 80 was prepared in analogy to Intermediate 77 using 5-bromo-2-nitropyridin-3-ol (500 mg, 2.28 mmol) and (1S)-1-(pyridin-4-yl)ethan-1-ol (281 mg, 2.28 mmol).
LC-MS (method 2): Rt = 0.87 min; MS (ESlpos): m/z = 326 [M+H]⁺

### Intermediate 81

### (rac)-5-{1-[(5-bromo-2-nitropyridin-3-yl)oxy]ethyl}pyrimidine

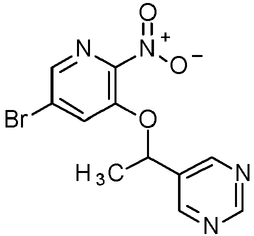

Intermediate 81 was prepared in analogy to Intermediate 77 using 5-bromo-2-nitropyridin-3-ol (826 mg, 3.77 mmol) and (rac)-1-(pyrimidin-5-yl)ethan-1-ol (468 mg, 3.77 mmol).
LC-MS (method 1): Rt = 0.97 min; MS (ESlpos): m/z = 327 [M+H]⁺
¹H-NMR (400MHz, DMSO-d₆): δ [ppm]= 1.67 (d, 3H), 6.07 (q, 1H), 8.30 (d, 1H), 8.44 (d, 1H), 8.90 (s, 2H), 9.18 (s, 1H).

### Intermediate 82

### 3-[(2,6-dichlorophenyl)methoxy]-2-nitropyridine

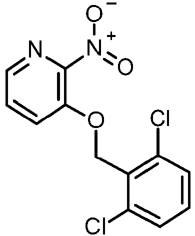

Intermediate 82 was prepared in analogy to Intermediate 77 using (2,6-dichlorophenyl)methanol (500 mg, 2.82 mmol) and 2-nitropyridin-3-ol (435 mg, 3.11 mmol).
LC-MS (method 1): Rt = 1.25 min; MS (ESlpos): m/z = 299 [M+H]⁺
¹H-NMR (400MHz, DMSO-d₆): δ [ppm]= 5.49 (s, 2H), 7.47 - 7.54 (m, 1H), 7.55 - 7.62 (m, 2H), 7.85 (dd, 1H), 8.19 (dd, 1H), 8.25 (dd, 1H).

### Intermediate 83

### 3-[(2-fluorophenyl)methoxy]-2-nitropyridine

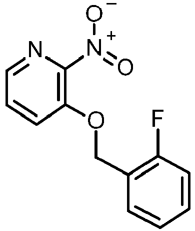

Intermediate 83 was prepared in analogy to Intermediate 77 using (2-fluorophenyl)methanol (430 mg, 3.41 mmol) and 2-nitropyridin-3-ol (478 mg, 3.41 mmol).
LC-MS (method 1): Rt = 1.15 min; MS (ESlpos): m/z = 249 [M+H]⁺
¹H-NMR (400MHz, DMSO-d₆): δ [ppm]= 5.42 (s, 2H), 7.23 - 7.32 (m, 2H), 7.42 - 7.49 (m, 1H), 7.54 (td, 1H), 7.80 (dd, 1H), 8.11 - 8.16 (m, 2H).

### Intermediate 84

### 3-[(3-fluorophenyl)methoxy]-2-nitropyridine

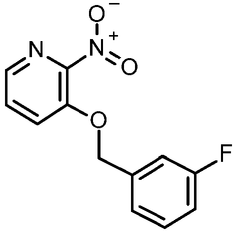

Intermediate 84 was prepared in analogy to Intermediate 77 using (3-fluorophenyl)methanol (430 mg, 3.41 mmol) and 2-nitropyridin-3-ol (478 mg, 3.41 mmol).
LC-MS (method 1): Rt = 1.16 min; MS (ESlpos): m/z = 249 [M+H]⁺
¹H-NMR (400MHz, DMSO-d₆): δ [ppm]= 5.40 (s, 2H), 7.17 - 7.23 (m, 1H), 7.24 - 7.30 (m, 2H), 7.47 (td, 1H), 7.79 (dd, 1H), 8.04 (dd, 1H), 8.14 (dd, 1H).

### Intermediate 85

### 3-[(2-chlorophenyl)methoxy]-2-nitropyridine

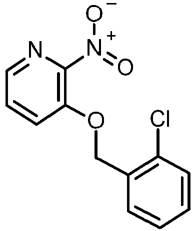

Intermediate 85 was prepared in analogy to Intermediate 77 using (2-chlorophenyl)methanol (450 mg, 3.16 mmol) and 2-nitropyridin-3-ol (486 mg, 3.47 mmol).
LC-MS (method 1): Rt = 1.23 min; MS (ESlpos): m/z = 265 [M+H]⁺
¹H-NMR (400MHz, DMSO-d₆): δ [ppm]= 5.42 (s, 2H), 7.41 - 7.44 (m, 2H), 7.52 - 7.55 (m, 1H), 7.57 - 7.61 (m, 1H), 7.81 (dd, 1H), 8.12 (dd, 1H), 8.16 (dd, 1H).

### Intermediate 86

### 2-nitro-3-[(pyridin-2-yl)methoxy]pyridine

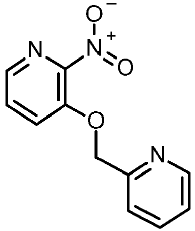

Intermediate 86 was prepared in analogy to Intermediate 77 using (pyridin-2-yl)methanol (400 mg, 3.67 mmol) and 2-nitropyridin-3-ol (514 mg, 3.67 mmol).
LC-MS (method 1): Rt = 0.90 min; MS (ESlpos): m/z = 233 [M+H]⁺
¹H-NMR (400MHz, DMSO-d₆): δ [ppm]= 5.45 (s, 2H), 7.38 (ddd, 1H), 7.49 (d, 1H), 7.78 (dd, 1H), 7.88 (td, 1H), 8.07 (dd, 1H), 8.14 (dd, 1H), 8.56 - 8.61 (m, 1H).

### Intermediate 87

### 3-[(2-chloro-5-fluorophenyl)methoxy]-2-nitropyridine

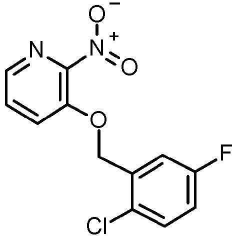

Intermediate 87 was prepared in analogy to Intermediate 77 using (2-chloro-5-fluorophenyl)methanol (589 mg, 3.67 mmol) and 2-nitropyridin-3-ol (514 mg, 3.67 mmol).
LC-MS (method 1): Rt = 1.24 min; MS (ESlpos): m/z = 283 [M+H]⁺
¹H-NMR (400MHz, DMSO-d₆): δ [ppm]= 5.41 (s, 2H), 7.32 (td, 1H), 7.45 (dd, 1H), 7.60 (dd, 1H), 7.82 (dd, 1H), 8.13 (dd, 1H), 8.18 (dd, 1H).

### Intermediate 88

### 5-bromo-2-nitro-3-[(1S)-1-(pyridin-2-yl)ethoxy]pyridine

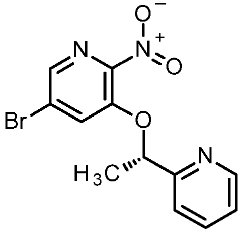

Intermediate 88 was prepared in analogy to Intermediate 77 using 5-bromo-2-nitropyridin-3-ol (889 mg, 4.06 mmol) and (1R)-1-(pyridin-2-yl)ethan-1-ol (500 mg, 4.06 mmol).
LC-MS (method 1): Rt = 1.17 min; MS (ESlpos): m/z = 326 [M+H]⁺
¹H-NMR (400 MHz, DMSO-d6) δ [ppm]: 1.133 (1.06), 1.153 (1.88), 1.170 (16.00), 1.185 (15.55), 1.615 (2.04), 1.631 (2.09), 1.986 (1.73), 2.518 (0.46), 4.734 (0.44), 4.750 (1.09), 4.766 (1.44), 4.781 (1.08), 4.797 (0.44), 5.933 (0.43), 5.949 (0.43), 7.476 (0.48), 7.496 (0.54), 7.859 (0.47), 7.864 (0.48), 8.241 (0.69), 8.245 (1.05), 8.259 (1.49), 8.264 (0.93), 8.882 (3.03).

### Intermediate 89

### 5-bromo-3-[(pyridazin-3-yl)methoxy]pyridin-2-amine

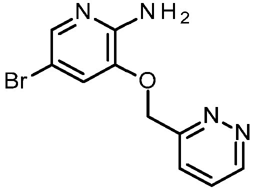

Intermediate 89 was prepared in analogy to Intermediate 77 using 2-amino-5-bromopyridin-3-ol (100 mg, 529 µmol) and (pyridazin-3-yl)methanol (58.3 mg, 529 µmol).
LC-MS (method 1): Rt = 0.74 min; MS (ESlpos): m/z = 283 [M+H]⁺
¹H-NMR (400MHz, DMSO-d₆): δ [ppm]= 5.44 (s, 2H), 6.12 (s, 2H), 7.40 (d, 1H), 7.63 (d, 1H), 7.77 (dd, 1H), 8.01 (dd, 1H), 9.22 (dd, 1H).

### Intermediate 90

### (rac)-2-{1-[(5-bromo-2-nitropyridin-3-yl)oxy]ethyl}-5-chloro-3-fluoropyridine

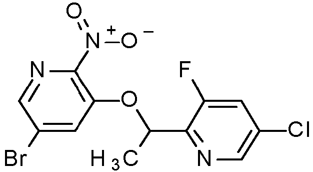

Intermediate 90 was prepared in analogy to Intermediate 77 using 5-bromo-2-nitropyridin-3-ol (200 mg, 913 µmol) and (rac)-1-(5-chloro-3-fluoropyridin-2-yl)ethan-1-ol (160 mg, 913 µmol).
LC-MS (method 1): Rt = 1.33 min; MS (ESlpos): m/z = 375 [M+H]⁺
¹H-NMR (400MHz, DMSO-d₆): δ [ppm]= 1.67 (d, 3H), 6.17 (q, 1H), 8.19 (dd, 1H), 8.29 (d, 1H), 8.36 (d, 1H), 8.54 (dd, 1H).

### Intermediate 91

### 5-bromo-3-[(1R)-1-(2-chlorophenyl)ethoxy]-2-nitropyridine

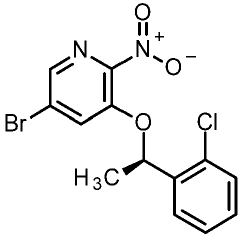

(1R)-1-(2-chlorophenyl)ethan-1-ol (521 mg, 3.33 mmol) was solubilised in THF (32 mL) under argon, cooled to 0°C, NaH (380 mg, 60 % purity, 9.50 mmol) was added and the mixture was stirred for 30 min at 0°C. 5-bromo-3-fluoro-2-nitropyridine (700 mg, 3.17 mmol) was added and it was stirred for 1 h at 0°C. The mixture was diluted with sat. NH4Cl solution, extracted 2x with ethyl acetate and the combined organic layers were washed with brine, dried over Na2SO4 and concentrated under reduced pressure to give 1.54 g of the title compound, which was used without further purification.
LC-MS (method 1): Rt = 1.44 min; MS (ESlpos): m/z = 357 [M+H]⁺

### Intermediate 92

### 5-bromo-3-[(1S)-1-(2-chlorophenyl)ethoxy]-2-nitropyridine

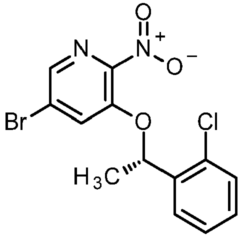

Intermediate 92 was prepared in analogy to Intermediate 91 using 5-bromo-3-fluoro-2-nitropyridine (700 mg, 3.17 mmol) and (1S)-1-(2-chlorophenyl)ethan-1-ol (521 mg, 3.33 mmol).
LC-MS (method 1): Rt = 1.44 min; MS (ESlpos): m/z = 357 [M+H]⁺

### Intermediate 93

### 5-bromo-2-nitro-3-[(1R)-1-(pyridin-2-yl)ethoxy]pyridine

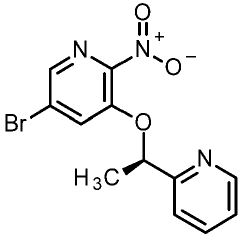

Intermediate 93 was prepared in analogy to Intermediate 91 using 5-bromo-3-fluoro-2-nitropyridine (300 mg, 1.36 mmol) and (1R)-1-(pyridin-2-yl)ethan-1-ol (176 mg, 1.43 mmol).
LC-MS (method 1): Rt = 1.14 min; MS (ESlpos): m/z = 326 [M+H]⁺

### Intermediate 94

### 5-bromo-3-[(1S)-1-(pyridin-2-yl)ethoxy]pyridin-2-amine

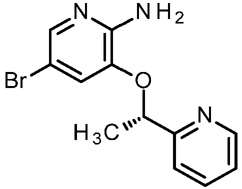

Intermediate 94 was prepared in analogy to Intermediate 91 using 5-bromo-3-fluoropyridin-2-amine (500 mg, 2.62 mmol) and (1S)-1-(pyridin-2-yl)ethan-1-ol (339 mg, 2.75 mmol).
¹H-NMR (400MHz, DMSO-d6): δ [ppm]= 1.60 (d, 3H), 5.51 (q, 1H), 6.06 (s, 2H), 7.02 (d, 1H), 7.31 (ddd, 1H), 7.47 - 7.57 (m, 2H), 7.81 (td, 1H), 8.52 - 8.59 (m, 1H).
LC-MS (method 1): Rt = 0.95 min; MS (ESlpos): m/z = 294 [M+H]⁺
¹H-NMR (400 MHz, DMSO-d6) δ [ppm]: 1.342 (1.35), 1.358 (1.37), 1.591 (15.49), 1.598 (2.65), 1.606 (16.00), 1.616 (1.52), 2.420 (0.79), 2.440 (0.48), 2.518 (2.24), 2.523 (1.60), 2.824 (0.45), 2.836 (0.44), 2.995 (2.46), 5.332 (0.50), 5.344 (0.43), 5.482 (0.95), 5.497 (3.35), 5.514 (3.36), 5.530 (1.00), 5.758 (1.85), 6.065 (6.33), 6.471 (0.63), 7.014 (5.76), 7.019 (5.80), 7.297 (2.15), 7.300 (2.22), 7.309 (2.22), 7.312 (2.35), 7.316 (2.38), 7.319 (2.40), 7.328 (2.34), 7.331 (2.41), 7.502 (4.02), 7.521 (12.34), 7.526 (9.41), 7.572 (1.12), 7.664 (0.44), 7.669 (0.46), 7.691 (0.44), 7.696 (0.46), 7.786 (2.25), 7.791 (2.32), 7.806 (3.41), 7.810 (3.49), 7.825 (1.82), 7.829 (1.82), 7.844 (0.61), 7.846 (0.64), 7.849 (0.59), 7.851 (0.56), 8.544 (2.33), 8.546 (2.83), 8.548 (2.81), 8.551 (2.63), 8.556 (2.46), 8.558 (2.91), 8.561 (2.65), 8.563 (2.45).

### Intermediate 95

### 5-bromo-3-[(3-methylphenyl)methoxy]pyridin-2-amine

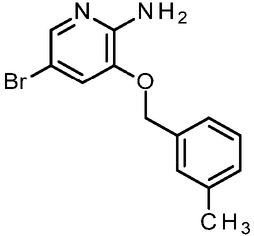

(3-methylphenyl)methanol (20.1 mg, 165 µmol) was solubilised in DMSO (2 mL) under argon, NaH (219 mg, 60 % purity, 314 µmol) was added and the mixture was stirred for 1 h at rt. 5-bromo-3-fluoropyridin-2-amine (30.0 mg, 157 µmol) was added and it was stirred for 20 h at 100°C. The mixture was diluted with water, extracted 3x with DCM and the combined organic layers were dried over MgSO4, filtered and concentrated under reduced pressure. The residue was purified by flash chromatography to give 30 mg (65 % yield) of the title compound
LC-MS (method 1): Rt = 1.27 min; MS (ESlpos): m/z = 295 [M+H]⁺
¹H-NMR (400 MHz, DMSO-d6) δ [ppm]: 1.156 (1.67), 1.165 (1.40), 1.233 (0.97), 2.251 (0.45), 2.289 (1.46), 2.323 (16.00), 2.331 (1.33), 2.336 (0.55), 2.518 (5.50), 2.523 (3.79), 2.540 (8.63), 2.664 (2.06), 2.669 (1.32), 2.673 (0.88), 2.994 (4.51), 5.107 (8.32), 5.960 (3.17), 7.134 (0.80), 7.144 (1.24), 7.146 (1.13), 7.152 (0.88), 7.195 (0.40), 7.263 (4.07), 7.266 (6.38), 7.268 (8.07), 7.274 (1.76), 7.279 (2.50), 7.282 (2.51), 7.306 (2.34), 7.577 (5.05), 7.582 (4.69).

### Intermediate 96

### 5-bromo-3-[(1-methyl-1H-pyrazol-5-yl)methoxy]pyridin-2-amine

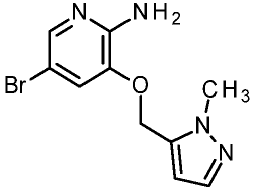

Intermediate 96 was prepared in analogy to Intermediate 95 using 5-bromo-3-fluoropyridin-2-amine (30.0 mg, 157 µmol) and (1-methyl-1H-pyrazol-5-yl)methanol (18.5 mg, 165 µmol).
LC-MS (method 1): Rt = 0.92 min; MS (ESlpos): m/z = 283 [M+H]⁺
¹H-NMR (400MHz, DMSO-d₆): δ [ppm]= 3.85 (s, 3H), 5.20 (s, 2H), 5.98 (s, 2H), 6.43 (d, 1H), 7.38 (dd, 2H), 7.60 (d, 1H).

### Intermediate 97

### 5-bromo-3-[(1S)-1-(1-methyl-1H-pyrazol-3-yl)ethoxy]-2-nitropyridine

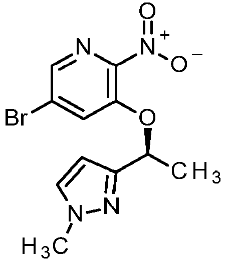

Intermediate 97 was prepared in analogy to Intermediate 91 using 5-bromo-3-fluoro-2-nitropyridine (150 mg, 679 µmol) and (1S)-1-(1-methyl-1H-pyrazol-3-yl)ethan-1-ol (85.6 mg, 679 µmol).
LC-MS (method 1): Rt = 1.10 min; MS (ESlpos): m/z = 327 [M+H]⁺
¹H-NMR (400MHz, CHLOROFORM-d): δ [ppm]= 1.74 (d, 3H), 3.89 (s, 3H), 5.54 (d, 1H), 6.28 (d, 1H), 7.32 (d, 1H), 7.91 (d, 1H), 8.05 (d, 1H).

### Intermediate 98

### 5-bromo-3-[(1R)-1-(1-methyl-1H-pyrazol-3-yl)ethoxy]-2-nitropyridine

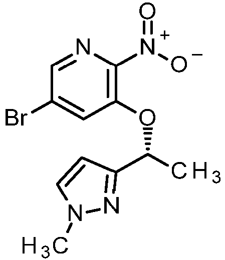

Intermediate 98 was prepared in analogy to Intermediate 91 using 5-bromo-3-fluoro-2-nitropyridine (150 mg, 679 µmol) and (1R)-1-(1-methyl-1H-pyrazol-3-yl)ethan-1-ol (85.6 mg, 679 µmol).
LC-MS (method 1): Rt = 1.11 min; MS (ESlpos): m/z = 327 [M+H]⁺

### Intermediate 99

### 5-bromo-3-[(1S)-1-phenylethoxy]pyridin-2-amine

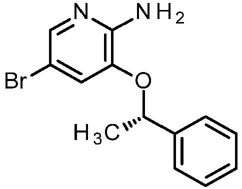

Intermediate 99 was prepared in analogy to Intermediate 95 using 5-bromo-3-fluoropyridin-2-amine (298 mg, 1.56 mmol) and (1S)-1-phenylethan-1-ol (200 mg, 1.64 mmol).
LC-MS (method 1): Rt = 1.22 min; MS (ESlpos): m/z = 293 [M+H]⁺
1H NMR (400 MHz, DMSO-d6) δ ppm 1.55 (d, 3 H) 5.57 (q, 1 H) 6.03 (s, 2 H) 7.05 (d, 1 H) 7.22 - 7.29 (m, 1 H) 7.31 - 7.39 (m, 2 H) 7.42 - 7.47 (m, 2 H) 7.49 (d, 1 H)

### Intermediate 100

### 5-bromo-3-[(1R)-1-phenylethoxy]pyridin-2-amine

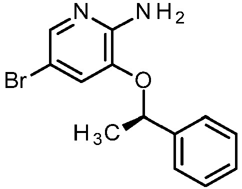

Intermediate 100 was prepared in analogy to Intermediate 95 using 5-bromo-3-fluoropyridin-2-amine (300 mg, 1.57 mmol) and (1R)-1-phenylethan-1-ol (200 µL, 1.6 mmol).
LC-MS (method 1): Rt = 1.19 min; MS (ESlpos): m/z = 293 [M+H]⁺
1H NMR (400 MHz, DMSO-d6) δ ppm 1.55 (d, 3 H) 5.57 (q, 1 H) 6.04 (s, 2 H) 7.05 (d, 1 H) 7.24 - 7.30 (m, 1 H) 7.32 - 7.39 (m, 2 H) 7.41 - 7.47 (m, 2 H) 7.49 (d,1 H)

### Intermediate 101

### 3-(benzyloxy)-5-bromo-2-nitropyridine

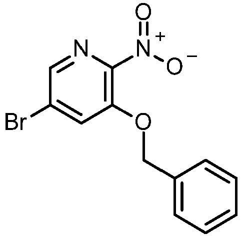

5-bromo-2-nitropyridin-3-ol (2.00 g, 9.13 mmol) was solubilised in DMF (70 mL), Cs2CO3 (2.98 g, 9.13 mmol) was added and the mixture was stirred for 30 min at rt. (bromomethyl)benzene (1.1 mL, 9.1 mmol) was added and it was stirred for 2.5 h at 50°C. The mixture was diluted with water, stirred for 10 min, filtered and the precipitate was washed with water. It was dried under reduced pressure at 60°C to give 2.70 g (98 % purity, 94 % yield) of the title compound, which was used without further purification.
¹H-NMR (400MHz, DMSO-d6): δ [ppm]= 5.40 (s, 2H), 7.35 - 7.41 (m, 1H), 7.43 (s, 2H), 7.44 (s, 2H), 8.31 (d, 1H), 8.43 (d, 1H).
LC-MS (method 1): Rt = 1.29 min; MS (ESlpos): m/z = 308 [M-H]⁺

### Intermediate 102

### (rac)-3-{[1-phenylethyl]sulfanyl}pyridin-2-amine

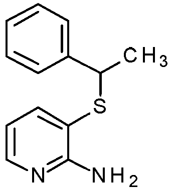

(rac)-2-nitro-3-{[1-phenylethyl]sulfanyl}pyridine (716 mg, 2.75 mmol) and Fe (307 mg, 5.50 mmol) were stirred in methanol (5.4 mL)/acetic acid (5.4 mL) overnight at 85°C. The mixture was diluted with ethyl acetate, filtered and the precipitate was washed with ethyl acetate and dried under reduced pressure. The precipitate was solubilized in ethyl acetate, washed with sat. NaHCO3 solution and brine, dried over Na2SO4, filtered and concentrated under reduced pressure to give 627 mg (88 % purity, 87 % yield) of the title compound, which was used without further purification.
LC-MS (method 1): Rt = 1.15 min; MS (ESlpos): m/z = 232 [M+H]⁺
¹H-NMR (400MHz, DMSO-d6): δ [ppm]= 1.50 (d, 3H), 4.38 (q, 1H), 6.08 (s, 2H), 6.42 (dd, 1H), 7.17 - 7.23 (m, 1H), 7.24 - 7.32 (m, 5H), 7.87 (dd, 1H).

### Intermediate 103

### 5-bromo-3-[(cyclohexylmethyl)sulfanyl]pyridin-2-amine

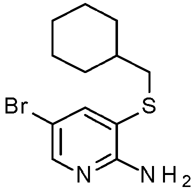

Intermediate 103 was prepared in analogy to Intermediate 102 using 5-bromo-3-[(cyclohexylmethyl)sulfanyl]-2-nitropyridine (1.00 g, 91 % purity, 2.75 mmol).
¹H-NMR (400MHz, DMSO-d6): δ [ppm]= 0.91 - 1.04 (m, 2H), 1.08 - 1.24 (m, 3H), 1.34 - 1.46 (m, 1H), 1.55 - 1.63 (m, 1H), 1.63 - 1.71 (m, 2H), 1.77 - 1.84 (m, 2H), 2.76 (d, 2H), 6.20 (s, 2H), 7.61 (d, 1H), 7.90 (d, 1H).
LC-MS (method 1): Rt = 1.52 min; MS (ESlpos): m/z = 301 [M+H]⁺

### Intermediate 104

### 3-(benzylsulfanyl)-5-bromopyridin-2-amine

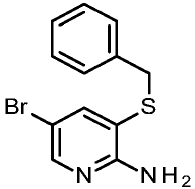

Intermediate 104 was prepared in analogy to Intermediate 102 using 3-(benzylsulfanyl)-5-bromo-2-nitropyridine (224 mg, 689 µmol).
¹H-NMR (400MHz, DMSO-d6): δ [ppm]= 4.10 (s, 2H), 6.28 (s, 2H), 7.21 - 7.32 (m, 5H), 7.46 (d, 1H), 7.90 (d, 1H).
LC-MS (method 1): Rt = 1.29 min; MS (ESlpos): m/z = 296 [M+H]⁺

### Intermediate 105

### (rac)-5-bromo-3-{[1-cyclohexylethyl]sulfanyl}pyridin-2-amine

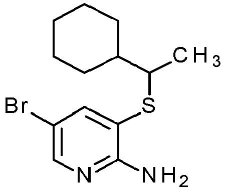

Intermediate 105 was prepared in analogy to Intermediate 102 using (rac)-5-bromo-3-{[1-cyclohexylethyl]sulfanyl}-2-nitropyridine (455 mg, 1.32 mmol).
¹H-NMR (400MHz, DMSO-d6): δ [ppm]= 1.03 - 1.24 (m, 8H), 1.37 - 1.47 (m, 1H), 1.57 - 1.65 (m, 1H), 1.67 - 1.83 (m, 4H), 3.12 - 3.21 (m, 1H), 6.25 (s, 2H), 7.67 (d, 1H), 7.96 (d, 1H).
LC-MS (method 1): Rt = 1.60 min; MS (ESlpos): m/z = 315 [M+H]⁺

### Intermediate 106

### 5-bromo-3-[(1R)-1-(pyridin-3-yl)ethoxy]pyridin-2-amine

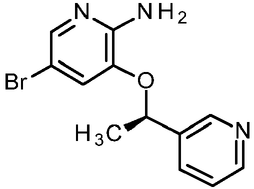

Intermediate 106 was prepared in analogy to Intermediate 102 using 5-bromo-2-nitro-3-[(1R)-1-(pyridin-3-yl)ethoxy]pyridine (760 mg, 32 % purity, 750 µmol).
¹H-NMR (400MHz, DMSO-d6): δ [ppm]= 1.58 (d, 3H), 5.68 (q, 1H), 6.08 (s, 2H), 7.20 (d, 1H), 7.38 (dd, 1H), 7.52 (d, 1H), 7.90 (dt, 1H), 8.49 (dd, 1H), 8.69 (d, 1H).
LC-MS (method 1): Rt = 0.89 min; MS (ESlpos): m/z = 294 [M+H]⁺

### Intermediate 107

### 5-bromo-3-[(1S)-1-(pyridin-3-yl)ethoxy]pyridin-2-amine

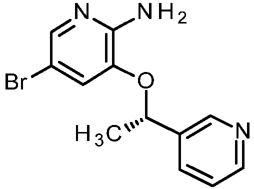

Intermediate 107 was prepared in analogy to Intermediate 102 using 5-bromo-2-nitro-3-[(1S)-1-(pyridin-3-yl)ethoxy]pyridine (500 mg, 27 % purity, 416 µmol).
¹H-NMR (400MHz, DMSO-d6): δ [ppm]= 1.58 (d, 3H), 5.68 (q, 1H), 6.08 (s, 2H), 7.20 (d, 1H), 7.38 (dd, 1H), 7.52 (d, 1H), 7.90 (dd, 1H), 8.49 (dd, 1H), 8.69 (d, 1H).
LC-MS (method 1): Rt = 0.92 min; MS (ESlpos): m/z = 294 [M+H]⁺

### Intermediate 108

### 5-bromo-3-[(cyclopentylmethyl)sulfanyl]pyridin-2-amine

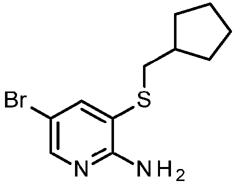

Intermediate 108 was prepared in analogy to Intermediate 102 using 5-bromo-3-[(cyclopentylmethyl)sulfanyl]-2-nitropyridine (503 mg, 1.59 mmol).
¹H-NMR (400MHz, DMSO-d6): δ [ppm]= 1.19 - 1.30 (m, 2H), 1.42 - 1.65 (m, 4H), 1.71 - 1.81 (m, 2H), 1.96 (spt, 1H), 2.85 (d, 2H), 6.21 (s, 2H), 7.64 (d, 1H), 7.90 (d, 1H).
LC-MS (method 1): Rt = 1.45 min; MS (ESlpos): m/z = 287 [M+H]⁺

### Intermediate 109

### 3-(benzyloxy)-5-bromopyridin-2-amine

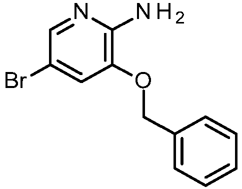

Intermediate 109 was prepared in analogy to Intermediate 102 using 3-(benzyloxy)-5-bromo-2-nitropyridine (2.70 g, 8.73 mmol).
1H-NMR (400MHz, DMSO-d6): δ [ppm]= 5.16 (s, 2H), 5.97 (s, 2H), 7.28 (d, 1H), 7.30 - 7.42 (m, 5H), 7.58 (d, 1H).
LC-MS (method 1): Rt = 1.19 min; MS (ESlneg): m/z = 277 [M-H]⁻

### Intermediate 110

### 5-bromo-3-[(1R)-1-(pyridin-4-yl)ethoxy]pyridin-2-amine

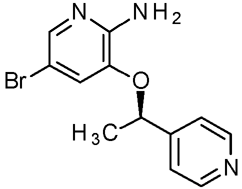

Intermediate 110 was prepared in analogy to Intermediate 102 using 5-bromo-2-nitro-3-[(1R)-1-(pyridin-4-yl)ethoxy]pyridine (1.60 g, 4.94 mmol).
LC-MS (method 2): Rt = 0.57 min; MS (ESlpos): m/z = 294 [M+H]⁺
¹H-NMR (400MHz, DMSO-d₆): δ [ppm]= 1.54 (d, 3H), 5.65 (q, 1H), 6.14 (br s, 2H), 7.11 (d, 1H), 7.48 (d, 2H), 7.53 (d, 1H), 8.56 (br d, 2H).

### Intermediate 111

### 5-bromo-3-[(1S)-1-(pyridin-4-yl)ethoxy]pyridin-2-amine

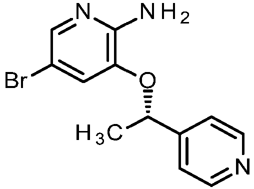

Intermediate 111 was prepared in analogy to Intermediate 102 using 5-bromo-2-nitro-3-[(1S)-1-(pyridin-4-yl)ethoxy]pyridine (1.50 g, 4.63 mmol).
LC-MS (method 2): Rt = 0.56 min; MS (ESlpos): m/z = 294 [M+H]⁺
¹H-NMR (400MHz, DMSO-d₆): δ [ppm]= 1.54 (d, 3H), 5.64 (q, 1H), 6.12 (s, 2H), 7.10 (d, 1H), 7.42 - 7.48 (m, 2H), 7.53 (d, 1H), 8.51 - 8.58 (m, 2H).

### Intermediate 112

### 5-bromo-3-[(1R)-1-(2-chlorophenyl)ethoxy]pyridin-2-amine

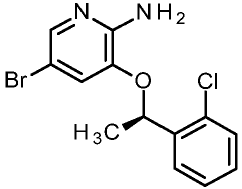

Intermediate 112 was prepared in analogy to Intermediate 102 using 5-bromo-3-[(1R)-1-(2-chlorophenyl)ethoxy]-2-nitropyridine (1.00 g, 2.80 mmol).
LC-MS (method 1): Rt = 1.31 min; MS (ESlpos): m/z = 329 [M+H]⁺
H-NMR-Data ¹H NMR (DMSO-d₆) δ: 7.57 (m, 1H), 7.52 (d, 1H), 7.44-7.49 (m, 1H), 7.33 (quind, 2H), 6.80 (d, 1H), 6.13 (s, 2H), 5.73 (m, 1H), 1.58 (d, 3H)

### Intermediate 113

### 5-bromo-3-[(1S)-1-(2-chlorophenyl)ethoxy]pyridin-2-amine

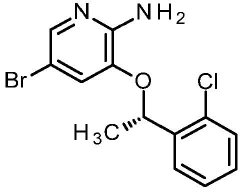

Intermediate 113 was prepared in analogy to Intermediate 102 using 5-bromo-3-[(1S)-1-(2-chlorophenyl)ethoxy]-2-nitropyridine (1.00 g, 2.80 mmol).
LC-MS (method 1): Rt = 1.31 min; MS (ESlpos): m/z = 329 [M+H]⁺
H-NMR-Data ¹H NMR (DMSO-d₆) δ: 7.57 (m, 1H), 7.52 (d, 1H), 7.46-7.49 (m, 1H), 7.34 (quind, 2H), 6.80 (d, 1H), 6.13 (s, 2H), 5.74 (m, 1H), 1.58 (d, 3H)

### Intermediate 114

### 5-bromo-3-[(1R)-1-(pyridin-2-yl)ethoxy]pyridin-2-amine

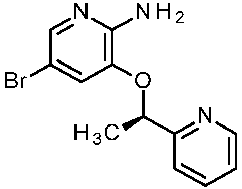

5-bromo-2-nitro-3-[(1R)-1-(pyridin-2-yl)ethoxy]pyridine (440 mg, 1.36 mmol) was solubilised in a degassed mixture of methanol (6.5 mL), water (13 mL) and THF (6.5 mL) under argon, Fe (379 mg, 6.79 mmol) and NH4CI (363 mg, 6.79 mmol) were added and the mixture was stirred overnight at 80°C. (379 mg, 6.79 mmol) and (363 mg, 6.79 mmol) were added and the mixture was stirred for 5 h at 80°C. It was filtered and the filtrate was extracted 2x with ethyl acetate. The combined organic layers were washed with brine, dried over Na2SO4 and concentrated under reduced pressure. The residue was purified by flash chromatography to give 188 mg (47 % yield) of the title compound.
¹H NMR (DMSO-d6, 400 MHz) δ 8.5-8.6 (m, 1H), 7.81 (dt, 1H), 7.5-7.6 (m, 2H), 7.31 (ddd, 1H), 7.02 (d, 1H), 6.06 (s, 2H), 5.51 (q, 1H), 1.60 (d, 3H)
LC-MS (method 1): Rt = 0.94 min; MS (ESlpos): m/z = 296 [M+H]⁺

### Intermediate 115

### 3-[(2,6-dichlorophenyl)methoxy]pyridin-2-amine

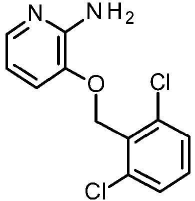

Intermediate 115 was prepared in analogy to Intermediate 114 using 3-[(2,6-dichlorophenyl)methoxy]-2-nitropyridine (600 mg, 2.01 mmol).
LC-MS (method 1): Rt = 1.15 min; MS (ESlpos): m/z = 269 [M+H]⁺
¹H-NMR (400MHz, DMSO-d₆): δ [ppm]= 5.21 (s, 2H), 5.50 (s, 2H), 6.53 (dd, 1H), 7.24 (dd, 1H), 7.43 - 7.51 (m, 1H), 7.53 - 7.59 (m, 3H).

### Intermediate 116

### 3-[(2-fluorophenyl)methoxy]pyridin-2-amine

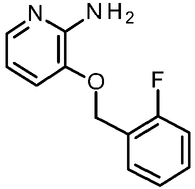

Intermediate 116 was prepared in analogy to Intermediate 114 using 3-[(2-fluorophenyl)methoxy]-2-nitropyridine (830 mg, 3.34 mmol).
LC-MS (method 1): Rt = 1.02 min; MS (ESlpos): m/z = 219 [M+H]⁺
¹H-NMR (400MHz, DMSO-d₆): δ [ppm]= 5.15 (s, 2H), 5.71 (br s, 2H), 6.51 (dd, 1H), 7.15 (dd, 1H), 7.21 - 7.29 (m, 2H), 7.38 - 7.46 (m, 1H), 7.53 (dd, 1H), 7.64 (td, 1H).

### Intermediate 117

### 3-[(3-fluorophenyl)methoxy]pyridin-2-amine

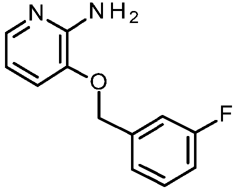

Intermediate 117 was prepared in analogy to Intermediate 114 using 3-[(3-fluorophenyl)methoxy]-2-nitropyridine (757 mg, 3.05 mmol).
LC-MS (method 1): Rt = 0.99 min; MS (ESlpos): m/z = 219 [M+H]⁺
¹H-NMR (400MHz, DMSO-d₆): δ [ppm]= 5.14 (s, 2H), 5.83 (br s, 2H), 6.49 (dd, 1H), 7.09 (dd, 1H), 7.11 - 7.18 (m, 1H), 7.32 (d, 1H), 7.35 - 7.47 (m, 2H), 7.51 (dd, 1H).

### Intermediate 118

### 3-[(2-chlorophenyl)methoxy]pyridin-2-amine

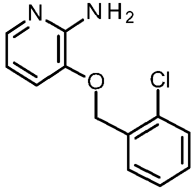

Intermediate 118 was prepared in analogy to Intermediate 114 using 3-[(2-chlorophenyl)methoxy]-2-nitropyridine (600 mg, 2.27 mmol).
LC-MS (method 1): Rt = 1.10 min; MS (ESlpos): m/z = 235 [M+H]⁺
¹H-NMR (400MHz, DMSO-d₆): δ [ppm]= 5.16 (s, 2H), 5.68 (s, 2H), 6.46 - 6.53 (m, 1H), 7.11 (dd, 1H), 7.36 - 7.42 (m, 2H), 7.48 - 7.55 (m, 2H), 7.67 - 7.73 (m, 1H).

### Intermediate 119

### 3-[(pyridin-2-yl)methoxy]pyridin-2-amine

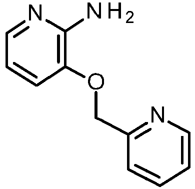

Intermediate 119 was prepared in analogy to Intermediate 114 using 2-nitro-3-[(pyridin-2-yl)methoxy]pyridine (865 mg, 3.74 mmol).
LC-MS (method 1): Rt = 0.72 min; MS (ESlpos): m/z = 202 [M+H]⁺
¹H-NMR (400MHz, DMSO-d₆): δ [ppm]= 5.17 (s, 2H), 5.77 (br s, 2H), 6.47 (dd, 1H), 7.07 (d, 1H), 7.34 (dd, 1H), 7.52 (d, 1H), 7.65 (d, 1H), 7.84 (td, 1H), 8.57 (d, 1H).

### Intermediate 120

### 3-[(2-chloro-5-fluorophenyl)methoxy]pyridin-2-amine

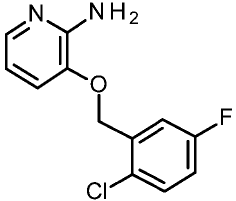

Intermediate 120 was prepared in analogy to Intermediate 114 using 3-[(2-chloro-5-fluorophenyl)methoxy]-2-nitropyridine (1.01 g, 3.57 mmol).
LC-MS (method 1): Rt = 1.09 min; MS (ESlpos): m/z = 253 [M+H]⁺
¹H-NMR (400MHz, DMSO-d₆): δ [ppm]= 5.13 (s, 2H), 5.82 (s, 2H), 6.50 (dd, 1H), 7.12 (dd, 1H), 7.25 (td, 1H), 7.51 - 7.59 (m, 2H), 7.66 (dd, 1H).

### Intermediate 121

### 5-bromo-3-[(1S)-1-(pyridin-2-yl)ethoxy]pyridin-2-amine

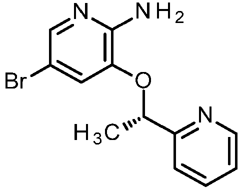

Intermediate 121 was prepared in analogy to Intermediate 102 using 5-bromo-2-nitro-3-[(1S)-1-(pyridin-2-yl)ethoxy]pyridine (2.57 g, 20 % purity, 1.59 mmol).
LC-MS (method 1): Rt = 0.97 min; MS (ESlpos): m/z = 296 [M+H]⁺
¹H-NMR (400 MHz, DMSO-d6) δ [ppm]: 1.590 (15.68), 1.606 (16.00), 1.895 (0.53), 1.986 (0.42), 2.518 (0.81), 2.523 (0.54), 3.159 (3.76), 3.173 (3.68), 4.099 (0.72), 4.112 (0.69), 5.482 (0.95), 5.497 (3.39), 5.514 (3.34), 5.530 (0.97), 6.066 (6.44), 7.014 (5.77), 7.019 (5.88), 7.297 (2.05), 7.299 (2.26), 7.309 (2.15), 7.311 (2.39), 7.315 (2.44), 7.318 (2.34), 7.327 (2.32), 7.330 (2.37), 7.501 (3.81), 7.521 (12.14), 7.526 (8.53), 7.785 (2.16), 7.790 (2.25), 7.804 (3.50), 7.809 (3.56), 7.824 (1.76), 7.829 (1.75), 8.544 (2.47), 8.546 (2.76), 8.548 (2.93), 8.551 (2.49), 8.556 (2.57), 8.558 (2.89), 8.560 (2.77), 8.563 (2.36).

### Intermediate 122

### (rac)-5-bromo-3-[1-(5-chloro-3-fluoropyridin-2-yl)ethoxy]pyridin-2-amine

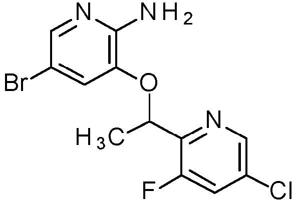

(rac)-2-{1-[(5-bromo-2-nitropyridin-3-yl)oxy]ethyl}-5-chloro-3-fluoropyridine (215 mg, 571 µmol) and Fe (191 mg, 3.43 mmol) were stirred in acetic acid (1.5 mL)/ethanol (5.0 mL) for 4 h at 80°C. The mixture was evaporated, diluted with DCM/EtOH (9:1), washed with water and neutralized with K2CO3 solution (2M). The organic layer was dried and concentrated under reduced pressure. The residue was purified by flash chromatography to give 176 mg (90 % purity, 80 % yield) of the title compound.
LC-MS (method 1): Rt = 1.19 min; MS (ESlpos): m/z = 346 [M+H]⁺
¹H-NMR (400MHz, DMSO-d₆): δ [ppm]= 1.66 (d, 3H), 5.73 - 5.81 (m, 1H), 5.88 (s, 2H), 7.13 (d, 1H), 7.56 (d, 1H), 8.15 (dd, 1H), 8.56 (dd, 1H).

### Intermediate 123

### (rac)-5-bromo-3-[1-(pyrimidin-5-yl)ethoxy]pyridin-2-amine

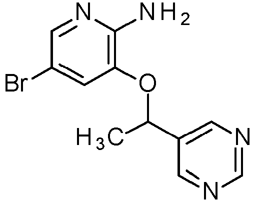

Intermediate 123 was prepared in analogy to Intermediate 122 using (rac)-5-{1-[(5-bromo-2-nitropyridin-3-yl)oxy]ethyl}pyrimidine (820 mg, 2.52 mmol).
LC-MS (method 1): Rt = 0.80 min; MS (ESlpos): m/z = 295 [M+H]⁺
¹H-NMR (400MHz, DMSO-d₆): δ [ppm]= 1.60 (d, 3H), 5.74 (q, 1H), 6.14 (s, 2H), 7.33 (d, 1H), 7.56 (d, 1H), 8.96 (s, 2H), 9.13 (s, 1H).

### Intermediate 124

### 5-bromo-3-{[1-(pyrimidin-5-yl)ethyl]oxy}pyridin-2-amine (stereoisomer 1)

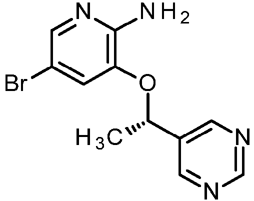

The racemate (rac)-5-bromo-3-{[1-(pyrimidin-5-yl)ethyl]oxy}pyridin-2-amine (671 mg, 2.27 mmol) was separated by chrial HPLC to give 253.7 g (99 % purity, 38 % yield) of the target compound.

PrepCon Labomatic HPLC-4; Column: YMC Amylose SA 10µ, 250x50; eluent A: hexane + 0.1 vol % diethylamine; eluent B: ethanol; isocratic: 60%A+40%B; flow: 100 mL/min; temperature: 25°C; UV: 254 nm;
Retention time: 9.8 - 12.2 min
[α]²⁰_{D} : -46.2° (c = 1.00, DMSO)
Instrument: Thermo Fisher UltiMate 3000; Column: YMC Amylose SA 3µ, 100x4.6; eluent A: hexane + 0.1 vol % diethylamine; eluent B: ethanol; isocratic: 60%A+40%B; flow: 1.4 mL/min; temperature: 25°C; UV: 254 nm;
¹H-NMR (400MHz, DMSO-d₆): δ [ppm]= 1.60 (d, 3H), 5.74 (q, 1H), 6.14 (s, 2H), 7.33 (d, 1H), 7.56 (d, 1H), 8.96 (s, 2H), 9.13 (s, 1H).

### Intermediate 125

### 5-bromo-3-{[1-(pyrimidin-5-yl)ethyl]oxy}pyridin-2-amine (stereoisomer 2)

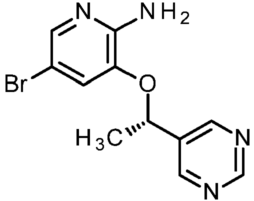

The racemate (rac)-5-bromo-3-{[1-(pyrimidin-5-yl)ethyl]oxy}pyridin-2-amine (671 mg, 2.27 mmol) was separated by chrial HPLC to give 184.8 g (99 % purity, 28 % yield) of the target compound.

PrepCon Labomatic HPLC-4; Column: YMC Amylose SA 10µ, 250x50; eluent A: hexane + 0.1 vol % diethylamine; eluent B: ethanol; isocratic: 60%A+40%B; flow: 100 mL/min; temperature: 25°C; UV: 254 nm;
Retention time: 21.9 - 30.0 min;
[α]²⁰_{D} : +44.4° (c = 1.00, DMSO)
Instrument: Thermo Fisher UltiMate 3000; Column: YMC Amylose SA 3µ, 100x4.6; eluent A: hexane + 0.1 vol % diethylamine; eluent B: ethanol; isocratic: 60%A+40%B; flow: 1.4 mL/min; temperature: 25°C; UV: 254 nm;
¹H-NMR (400MHz, DMSO-d₆): δ [ppm]= 1.60 (d, 3H), 5.74 (q, 1H), 6.14 (s, 2H), 7.33 (d, 1H), 7.56 (d, 1H), 8.96 (s, 2H), 9.13 (s, 1H).

### Intermediate 126

### 5-bromo-3-[(1S)-1-(1-methyl-1H-pyrazol-3-yl)ethoxy]pyridin-2-amine

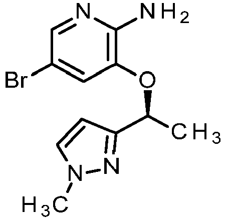

Intermediate 126 was prepared in analogy to Intermediate 122 using 5-bromo-3-[(1S)-1-(1-methyl-1H-pyrazol-3-yl)ethoxy]-2-nitropyridine (210 mg, 642 µmol).
LC-MS (method 1): Rt = 0.92 min; MS (ESlpos): m/z = 299 [M+H]⁺

### Intermediate 127

### 5-bromo-3-[(1R)-1-(1-methyl-1H-pyrazol-3-yl)ethoxy]pyridin-2-amine

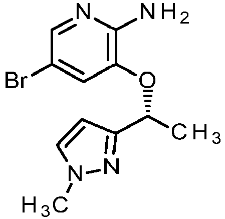

Intermediate 127 was prepared in analogy to Intermediate 122 using 5-bromo-3-[(1R)-1-(1-methyl-1H-pyrazol-3-yl)ethoxy]-2-nitropyridine (211 mg, 645 µmol).
LC-MS (method 1): Rt = 0.92 min; MS (ESlpos): m/z = 299 [M+H]⁺

### Intermediate 128

### (rac)-5-bromo-3-{[1-phenylethyl]sulfanyl}pyridin-2-amine

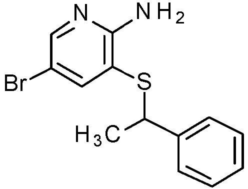

(rac)-3-{[1-phenylethyl]sulfanyl}pyridin-2-amine (600 mg, 2.60 mmol) was solubilised in acetonitrile (7.9 mL) under argon, cooled to -20°C, 1-bromopyrrolidine-2,5-dione (487 mg, 2.74 mmol), solubilised in acetonitrile (3.4 mL) was added and the mixture was stirred for 3 h h at rt. The mixture was diluted with DCM, washed with sat. Na2S2O3 solution and the aq. layer was extracted with DCM, dried over Na2SO4 and concentrated under reduced pressure. The residue was purified by preparative HPLC to give 336 mg (100 % purity, 42 % yield) of the title compound.
¹H-NMR (400MHz, DMSO-d6): δ [ppm]= 1.52 (d, 3H), 4.48 (q, 1H), 6.35 (s, 2H), 7.20 - 7.25 (m, 1H), 7.26 - 7.33 (m, 4H), 7.38 (d, 1H), 7.92 (d, 1H).
LC-MS (method 1): Rt = 1.34 min; MS (ESlpos): m/z = 309 [M+H]⁺

### Intermediate 129

### 1-(2-amino-5-bromopyridin-3-yl)-2,2,2-trifluoroethan-1-one

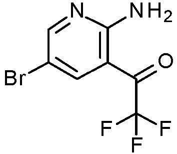

Intermediate 129 was prepared in analogy to Intermediate 128 using 1-(2-aminopyridin-3-yl)-2,2,2-trifluoroethan-1-one-hydrogen chloride (1/1) (1.37 g, 6.05 mmol).
LC-MS (method 1): Rt = 0.83 min; MS (ESlneg): m/z = 269 [M-H]⁻
¹H-NMR (400 MHz, DMSO-d6) δ [ppm]: 2.518 (8.51), 2.522 (5.28), 3.313 (0.85), 6.326 (1.42), 6.471 (0.76), 7.656 (0.99), 7.662 (1.07), 7.964 (1.26), 7.969 (1.44), 7.988 (1.89), 7.993 (6.15), 7.998 (8.71), 8.003 (6.17), 8.014 (1.80), 8.019 (1.50), 8.043 (1.81), 8.048 (1.72), 8.157 (1.87), 8.233 (4.53), 8.515 (15.86), 8.521 (16.00).

### Intermediate 130

### 5-bromo-3-(cyclohexylmethoxy)pyridin-2-amine

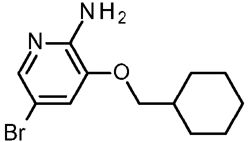

Intermediate 130 was prepared in analogy to Intermediate 128 using 3-(cyclohexylmethoxy)pyridin-2-amine (50.0 mg, 242 µmol).
LC-MS (method 1): Rt = 1.42 min; MS (ESlpos): m/z = 285 [M+H]⁺
1H-NMR (400 MHz, DMSO-d6) δ [ppm]: 1.008 (1.53), 1.026 (1.74), 1.054 (1.60), 1.084 (0.83), 1.155 (0.63), 1.209 (2.09), 1.234 (2.02), 1.266 (1.18), 1.658 (0.97), 1.691 (1.81), 1.725 (1.67), 1.804 (1.60), 1.835 (1.53), 1.988 (0.83), 2.332 (2.71), 2.518 (16.00), 2.522 (10.16), 2.673 (2.85), 3.305 (1.46), 3.405 (0.70), 3.770 (5.91), 3.786 (5.57), 4.221 (0.49), 5.869 (3.97), 6.239 (0.90), 7.136 (4.03), 7.141 (3.97), 7.542 (5.77), 7.547 (5.43).

### Intermediate 131

### 5-bromo-3-[(pyridin-3-yl)methoxy]pyridin-2-amine

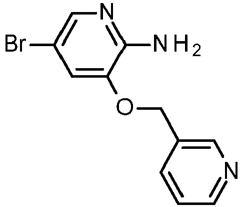

Intermediate 131 was prepared in analogy to Intermediate 128 using 3-[(pyridin-3-yl)methoxy]pyridin-2-amine (500 mg, 2.48 mmol).
LC-MS (method 1): Rt = 0.88 min; MS (ESlpos): m/z = 280 [M+H]⁺
¹H-NMR (400MHz, DMSO-d₆): δ [ppm]= 5.20 (s, 2H), 6.03 (s, 2H), 7.34 (d, 1H), 7.43 (ddd, 1H), 7.60 (d, 1H), 7.94 (dt, 1H), 8.55 (dd, 1H), 8.70 - 8.73 (m, 1H).

### Intermediate 132

### 5-bromo-3-[(4-fluorophenyl)methoxy]pyridin-2-amine

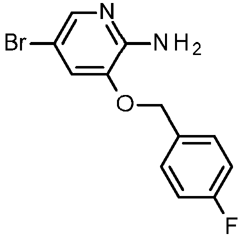

Intermediate 132 was prepared in analogy to Intermediate 128 using 3-[(4-fluorophenyl)methoxy]pyridin-2-amine (160 mg, 733 µmol).
LC-MS (method 1): Rt = 1.19 min; MS (ESlpos): m/z = 297 [M+H]⁺
1H-NMR (400 MHz, DMSO-d6) δ [ppm]: 0.987 (0.77), 1.005 (1.78), 1.023 (0.81), 2.073 (0.65), 2.462 (0.47), 2.466 (0.50), 2.518 (0.96), 2.522 (0.61), 2.539 (16.00), 3.027 (0.52), 3.045 (0.51), 3.404 (0.84), 3.408 (0.78), 4.201 (0.51), 4.219 (0.72), 4.237 (0.49), 5.264 (8.18), 6.235 (1.20), 7.199 (4.05), 7.222 (0.47), 7.229 (3.01), 7.235 (1.10), 7.245 (1.28), 7.251 (5.68), 7.257 (1.21), 7.268 (1.06), 7.274 (3.32), 7.325 (4.89), 7.452 (3.99), 7.553 (0.42), 7.560 (2.78), 7.566 (1.20), 7.574 (3.06), 7.582 (2.73), 7.591 (1.04), 7.596 (2.34), 7.675 (3.57), 7.680 (3.75), 7.820 (4.86), 7.825 (4.44).

### Intermediate 133

### (rac)-5-bromo-3-[1-phenylethoxy]pyridin-2-amine

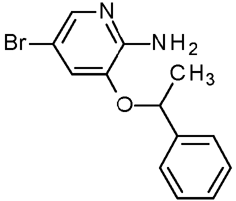

Intermediate 133 was prepared in analogy to Intermediate 128 using (rac)-3-(1-phenylethoxy)pyridin-2-amine (500 mg, 2.33 mmol).
LC-MS (method 1): Rt = 1.23 min; MS (ESlpos): m/z = 293 [M+H]⁺
¹H-NMR (400MHz, DMSO-d₆): δ [ppm]= 1.55 (d, 3H), 5.57 (q, 1H), 6.03 (s, 2H), 7.05 (d, 1H), 7.24 - 7.30 (m, 1H), 7.32 - 7.38 (m, 2H), 7.42 - 7.47 (m, 2H), 7.49 (d, 1H).

### Intermediate 134

### 5-bromo-3-[(trifluoromethyl)sulfanyl]pyridin-2-amine

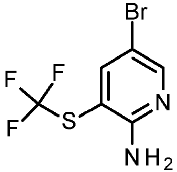

Intermediate 134 was prepared in analogy to Intermediate 128 using 3-[(trifluoromethyl)sulfanyl]pyridin-2-amine (935 mg, 4.82 mmol).
LC-MS (method 1): Rt = 1.19 min; MS (ESlpos): m/z = 273 [M+H]⁺
¹H-NMR (400MHz, DMSO-d₆): δ [ppm]= 6.91 (s, 2H), 7.96 (d, 1H), 8.21 (d, 1H).

### Intermediate 135

### 5-bromo-3-(difluoromethoxy)pyridin-2-amine

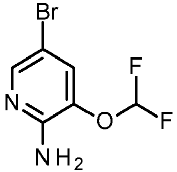

Intermediate 135 was prepared in analogy to Intermediate 128 using 3-(difluoromethoxy)pyridin-2-amine (3.00 g, 18.7 mmol).
LC-MS (method 1): Rt = 0.95 min; MS (ESlpos): m/z = 239 [M+H]⁺
¹H-NMR (400MHz, DMSO-d₆): δ [ppm]= 6.36 (s, 2H), 7.16 (br t, 1H), 7.51 (d, 1H), 7.89 (d, 1H).

### Intermediate 136

### 5-bromo-3-(trifluoromethoxy)pyridin-2-amine

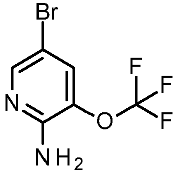

Intermediate 136 was prepared in analogy to Intermediate 128 using 3-(trifluoromethoxy)pyridin-2-amine (500 mg, 2.81 mmol).
LC-MS (method 1): Rt = 1.09 min; MS (ESlneg): m/z = 255 [M-H]⁻
¹H-NMR (400 MHz, DMSO-d6) δ [ppm]: 2.518 (2.65), 2.523 (1.85), 2.669 (0.41), 6.694 (4.67), 7.748 (2.45), 7.752 (6.02), 7.755 (7.26), 7.757 (7.93), 7.760 (6.21), 8.031 (16.00), 8.036 (15.58).

### Intermediate 137

### 5-bromo-3-[(2,6-dichlorophenyl)methoxy]pyridin-2-amine

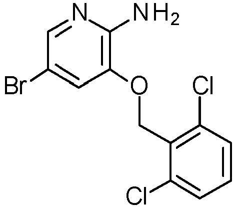

Intermediate 137 was prepared in analogy to Intermediate 128 using 3-[(2,6-dichlorophenyl)methoxy]pyridin-2-amine (411 mg, 1.53 mmol).
LC-MS (method 1): Rt = 1.30 min; MS (ESlpos): m/z = 349 [M+H]⁺
¹H-NMR (400MHz, DMSO-d₆): δ [ppm]= 5.24 (s, 2H), 5.82 (s, 2H), 7.43 - 7.52 (m, 2H), 7.55 - 7.59 (m, 2H), 7.63 (d, 1H).

### Intermediate 138

### 5-bromo-3-[(2-fluorophenyl)methoxy]pyridin-2-amine

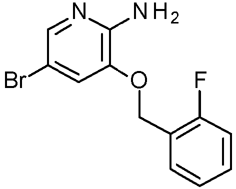

Intermediate 138 was prepared in analogy to Intermediate 128 using 3-[(2-fluorophenyl)methoxy]pyridin-2-amine (690 mg, 3.16 mmol).
LC-MS (method 1): Rt = 1.19 min; MS (ESlpos): m/z = 297 [M+H]⁺
¹H-NMR (400MHz, DMSO-d₆): δ [ppm]= 5.18 (s, 2H), 5.94 (s, 2H), 7.21 - 7.30 (m, 2H), 7.36 (d, 1H), 7.39 - 7.47 (m, 1H), 7.61 (d, 1H), 7.65 (td, 1H).

### Intermediate 139

### 5-bromo-3-[(3-fluorophenyl)methoxy]pyridin-2-amine

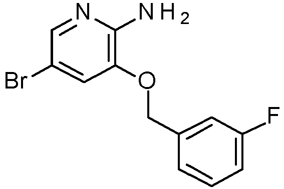

Intermediate 139 was prepared in analogy to Intermediate 128 using 3-[(3-fluorophenyl)methoxy]pyridin-2-amine (728 mg, 3.34 mmol).
LC-MS (method 1): Rt = 1.18 min; MS (ESlpos): m/z = 297 [M+H]⁺
¹H-NMR (400MHz, DMSO-d₆): δ [ppm]= 5.18 (s, 2H), 6.05 (s, 2H), 7.12 - 7.20 (m, 1H), 7.28 (d, 1H), 7.32 (d, 1H), 7.37 - 7.48 (m, 2H), 7.59 (d, 1H).

### Intermediate 140

### 5-bromo-3-[(2-chlorophenyl)methoxy]pyridin-2-amine

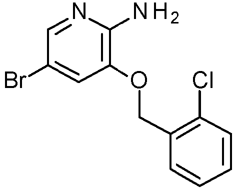

Intermediate 140 was prepared in analogy to Intermediate 128 using 3-[(2-chlorophenyl)methoxy]pyridin-2-amine (510 mg, 2.17 mmol).
LC-MS (method 1): Rt = 1.27 min; MS (ESlpos): m/z = 313 [M+H]⁺
¹H-NMR (400MHz, DMSO-d₆): δ [ppm]= 5.19 (s, 2H), 5.97 (s, 2H), 7.34 (d, 1H), 7.37 - 7.44 (m, 2H), 7.48 - 7.55 (m, 1H), 7.62 (d, 1H), 7.68 - 7.75 (m, 1H).

### Intermediate 141

### 5-bromo-3-[(pyridin-2-yl)methoxy]pyridin-2-amine

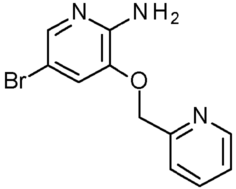

Intermediate 141 was prepared in analogy to Intermediate 128 using 3-[(pyridin-2-yl)methoxy]pyridin-2-amine (594 mg, 2.95 mmol).
LC-MS (method 1): Rt = 0.92 min; MS (ESlpos): m/z = 280 [M+H]⁺
¹H-NMR (400MHz, DMSO-d₆): δ [ppm]= 5.21 (s, 2H), 6.06 (s, 2H), 7.29 (d, 1H), 7.35 (ddd, 1H), 7.60 (d, 1H), 7.67 (d, 1H), 7.85 (td, 1H), 8.55 - 8.60 (m, 1H).

### Intermediate 142

### 5-bromo-3-[(2-chloro-5-fluorophenyl)methoxy]pyridin-2-amine

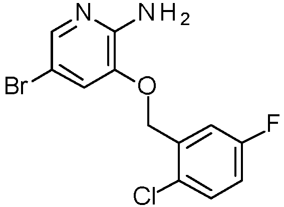

Intermediate 142 was prepared in analogy to Intermediate 128 using 3-[(2-chloro-5-fluorophenyl)methoxy]pyridin-2-amine (900 mg, 3.56 mmol).
LC-MS (method 1): Rt = 1.23 min; MS (ESlpos): m/z = 331 [M+H]⁺
¹H-NMR (400MHz, DMSO-d₆): δ [ppm]= 5.17 (s, 2H), 6.10 (s, 2H), 7.27 (td, 1H), 7.36 (d, 1H), 7.56 (dd, 1H), 7.63 (d, 1H), 7.70 (dd, 1H).

### Intermediate 143

### 5-bromo-3-(difluoromethyl)pyridin-2-amine

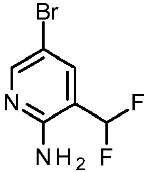

Intermediate 143 was prepared in analogy to Intermediate 128 using 3-(difluoromethyl)pyridin-2-amine (500 mg, 3.47 mmol).

### Intermediate 144

### 5-bromo-3-phenylpyridin-2-amine

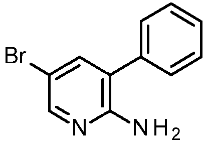

5-bromo-3-iodopyridin-2-amine (250 mg, 836 µmol) was solubilised in acetonitrile (2.5 mL), phenylboronic acid (120 mg, 987 µmol), Pd(PPh3)2Cl2 (29.4 mg, 41.8 µmol) and Na2CO3 (2.5 mL, 2.0 M, 5.0 mmol) were added and the mixture was stirred for 1 h at 60°C. The mixture was diluted with ethyl acetate/THF (1:1), washed with brine and the organic layer was dried over a silicone filtrer and evaporated. The residue was purified by flash chromatography to give 175 mg (84 % yield) of the title compound.
LC-MS (method 2): Rt = 1.02 min; MS (ESlpos): m/z = 249 [M+H]⁺
1H-NMR (400MHz, DMSO-d6): δ [ppm]= 5.84 (s, 2H), 7.35 - 7.43 (m, 1H), 7.43 - 7.52 (m, 5H), 8.02 (d, 1H).

### Intermediate 145

### 5-bromo-3-(4-methylphenyl)pyridin-2-amine

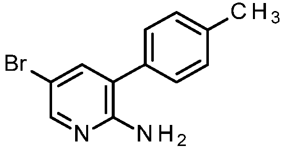

Intermediate 145 was prepared in analogy to Intermediate 144 using 5-bromo-3-iodopyridin-2-amine (250 mg, 836 µmol) and (4-methylphenyl)boronic acid (134 mg, 987 µmol).
LC-MS (method 1): Rt = 1.26 min; MS (ESlpos): m/z = 265 [M+H]⁺
1H-NMR (400MHz, DMSO-d6): δ [ppm]= 2.35 (s, 3H), 5.80 (s, 2H), 7.24 - 7.30 (m, 2H), 7.31 - 7.37 (m, 2H), 7.43 (d, 1H), 8.00 (d, 1H).

### Intermediate 146

### 5-bromo-3-(2-methylphenyl)pyridin-2-amine

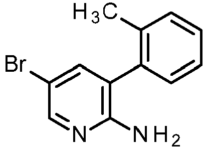

Intermediate 146 was prepared in analogy to Intermediate 144 using 5-bromo-3-iodopyridin-2-amine (250 mg, 836 µmol) and (2-methylphenyl)boronic acid (134 mg, 987 µmol).
LC-MS (method 1): Rt = 1.22 min; MS (ESlpos): m/z = 263 [M+H]⁺
¹H-NMR (400MHz, DMSO-d₆): δ [ppm]= 2.11 (s, 3H), 5.55 (s, 2H), 7.14 (d, 1H), 7.23 - 7.30 (m, 1H), 7.31 - 7.33 (m, 2H), 7.36 (d, 1H), 8.03 (d, 1H).

### Intermediate 147

### 5-bromo-3-(3-methylphenyl)pyridin-2-amine

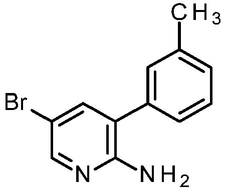

Intermediate 147 was prepared in analogy to Intermediate 144 using 5-bromo-3-iodopyridin-2-amine (250 mg, 836 µmol) and (3-methylphenyl)boronic acid (134 mg, 987 µmol).
LC-MS (method 1): Rt = 1.26 min; MS (ESlpos): m/z = 265 [M+H]⁺
¹H-NMR (400MHz, DMSO-d₆): δ [ppm]= 2.35 (s, 3H), 5.83 (s, 2H), 7.17 - 7.28 (m, 3H), 7.32 - 7.38 (m, 1H), 7.44 (d, 1H), 8.01 (d, 1H).

### Intermediate 148

### 5-bromo-3-(3-fluorophenyl)pyridin-2-amine

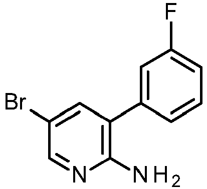

Intermediate 148 was prepared in analogy to Intermediate 144 using 5-bromo-3-iodopyridin-2-amine (250 mg, 836 µmol) and (3-fluorophenyl)boronic acid (138 mg, 987 µmol).
LC-MS (method 2): Rt = 1.11 min; MS (ESlpos): m/z = 267 [M+H]⁺
¹H-NMR (400MHz, DMSO-d₆): δ [ppm]= 5.97 (s, 2H), 7.18 - 7.25 (m, 1H), 7.27 - 7.32 (m, 2H), 7.46 - 7.54 (m, 2H), 7.54 - 7.66 (m, 1H), 8.03 (d, 1H).

### Intermediate 149

### 5-bromo[3,3'-bipyridin]-2-amine

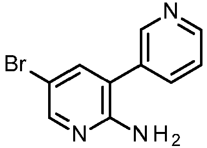

5-bromo-3-iodopyridin-2-amine (250 mg, 836 µmol) was solubilised in acetonitrile (2.5 mL), pyridin-3-ylboronic acid (121 mg, 987 µmol), Pd(PPh3)2Cl2 (29.4 mg, 41.8 µmol) and Na2CO3 (2.5 mL, 2.0 M, 5.0 mmol) were added and the mixture was stirred for 1 h at 60°C. pyridin-3-ylboronic acid (121 mg, 987 µmol), Pd(PPh3)2Cl2 (29.4 mg, 41.8 µmol) and Cs2CO3 (273 mg, 836 µmol) were added and the mixture was stirred for 1 h at 60°C. The mixture was diluted with ethyl acetate/THF (1:1), washed with brine and the organic layer was dried over a silicone filtrer and evaporated. The residue was purified by flash chromatography to give 225 mg of the title compound.
LC-MS (method 1): Rt = 0.87 min; MS (ESlpos): m/z = 252 [M+H]⁺
¹H-NMR (400MHz, DMSO-d₆): δ [ppm]= 6.02 (s, 2H), 7.47 (ddd, 1H), 7.54 (d, 1H), 7.87 (dt, 1H), 8.05 (d, 1H), 8.58 (dd, 1H), 8.62 (dd, 1H).

### Intermediate 150

### 5-bromo-3-(2-fluorophenyl)pyridin-2-amine

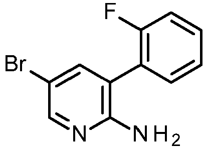

Intermediate 150 was prepared in analogy to Intermediate 144 using 5-bromo-3-iodopyridin-2-amine (250 mg, 836 µmol) and (2-fluorophenyl)boronic acid (138 mg, 987 µmol).
LC-MS (method 1): Rt = 1.16 min; MS (ESlpos): m/z = 267 [M+H]⁺
¹H-NMR (400MHz, DMSO-d₆): δ [ppm]= 5.86 (s, 2H), 7.25 - 7.34 (m, 2H), 7.39 (td, 1H), 7.43 - 7.51 (m, 2H), 8.06 (d, 1H).

### Intermediate 151

### 5-bromo[3,4'-bipyridin]-2-amine

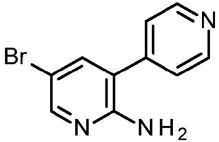

Intermediate 151 was prepared in analogy to Intermediate 149 using 5-bromo-3-iodopyridin-2-amine (250 mg, 836 µmol) and pyridin-4-ylboronic acid (121 mg, 987 µmol).
LC-MS (method 1): Rt = 0.88 min; MS (ESlpos): m/z = 250 [M+H]⁺
¹H-NMR (400MHz, DMSO-d₆): δ [ppm]= 6.11 (s, 2H), 7.46 - 7.51 (m, 2H), 7.58 (d, 1H), 8.08 (d, 1H), 8.60 - 8.66 (m, 2H).

### Intermediate 152

### 5-bromo-3-[3-(methanesulfonyl)phenyl]pyridin-2-amine

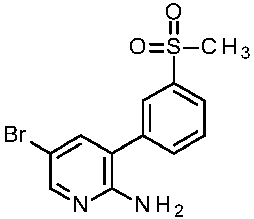

Intermediate 152 was prepared in analogy to Intermediate 144 using 5-bromo-3-iodopyridin-2-amine (250 mg, 836 µmol) and [3-(methanesulfonyl)phenyl]boronic acid (197 mg, 987 µmol).
LC-MS (method 1): Rt = 0.96 min; MS (ESlpos): m/z = 327 [M+H]⁺
¹H-NMR (400MHz, DMSO-d₆): δ [ppm]= 3.27 (s, 3H), 6.08 (s, 2H), 7.60 (d, 1H), 7.70 - 7.76 (m, 1H), 7.78 - 7.84 (m, 1H), 7.92 (dt, 1H), 7.94 - 7.96 (m, 1H), 8.07 (d, 1H).

### Intermediate 153

### 5-bromo-3-[4-(methanesulfonyl)phenyl]pyridin-2-amine

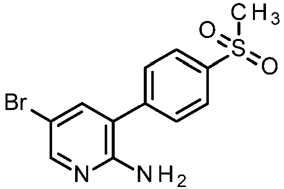

Intermediate 153 was prepared in analogy to Intermediate 144 using 5-bromo-3-iodopyridin-2-amine (250 mg, 836 µmol) and [4-(methanesulfonyl)phenyl]boronic acid (197 mg, 987 µmol).
LC-MS (method 1): Rt = 0.96 min; MS (ESlpos): m/z = 327 [M+H]⁺
¹H-NMR (400MHz, DMSO-d₆): δ [ppm]= 3.25 (s, 3H), 6.06 (s, 2H), 7.56 (d, 1H), 7.71 - 7.73 (m, 1H), 7.73 - 7.75 (m, 1H), 7.97 - 7.99 (m, 1H), 7.99 - 8.01 (m, 1H), 8.07 (d, 1H).

### Intermediate 154

### 1-[4-(2-amino-5-bromopyridin-3-yl)phenyl]ethan-1-one

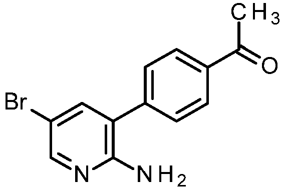

Intermediate 154 was prepared in analogy to Intermediate 144 using 5-bromo-3-iodopyridin-2-amine (250 mg, 836 µmol) and (4-acetylphenyl)boronic acid (162 mg, 987 µmol).
LC-MS (method 1): Rt = 1.09 min; MS (ESlpos): m/z = 293 [M+H]⁺

### Intermediate 155

### 5-bromo-3-[4-(methanesulfinyl)phenyl]pyridin-2-amine

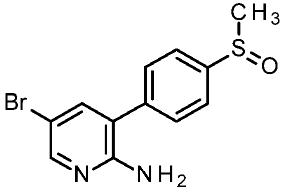

Intermediate 155 was prepared in analogy to Intermediate 144 using 5-bromo-3-iodopyridin-2-amine (250 mg, 836 µmol) and [4-(methanesulfinyl)phenyl]boronic acid (182 mg, 987 µmol).
LC-MS (method 1): Rt = 0.88 min; MS (ESlpos): m/z = 311 [M+H]⁺

### Intermediate 156

### 5-bromo-3-(3-methoxyphenyl)pyridin-2-amine

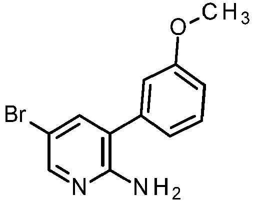

Intermediate 156 was prepared in analogy to Intermediate 144 using 5-bromo-3-iodopyridin-2-amine (250 mg, 836 µmol) and (3-methoxyphenyl)boronic acid (150 mg, 987 µmol).
LC-MS (method 1): Rt = 1.18 min; MS (ESlpos): m/z = 281 [M+H]⁺
¹H-NMR (400MHz, DMSO-d₆): δ [ppm]= 3.80 (s, 3H), 5.86 (s, 2H), 6.92 - 7.03 (m, 3H), 7.38 (t, 1H), 7.48 (d, 1H), 8.01 (d, 1H).

### Intermediate 157

### 5-bromo-3-[4-(cyclopropanesulfonyl)phenyl]pyridin-2-amine

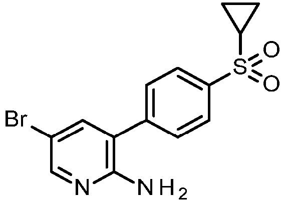

Intermediate 157 was prepared in analogy to Intermediate 144 using 5-bromo-3-iodopyridin-2-amine (250 mg, 836 µmol) and [4-(cyclopropanesulfonyl)phenyl]boronic acid (223 mg, 987 µmol).
LC-MS (method 1): Rt = 1.07 min; MS (ESlpos): m/z = 355 [M+H]⁺
¹H-NMR (400MHz, DMSO-d₆): δ [ppm]= 1.03 - 1.11 (m, 2H), 1.12 - 1.20 (m, 2H), 2.89 (tt, 1H), 6.08 (s, 2H), 7.58 (d, 1H), 7.71 - 7.76 (m, 2H), 7.93 - 7.97 (m, 2H), 8.07 (d, 1H).

### Intermediate 158

### 4-(2-amino-5-bromopyridin-3-yl)benzene-1-sulfonamide

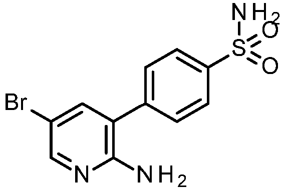

Intermediate 158 was prepared in analogy to Intermediate 144 using 5-bromo-3-iodopyridin-2-amine (250 mg, 836 µmol) and (4-sulfamoylphenyl)boronic acid (198 mg, 987 µmol).
LC-MS (method 1): Rt = 0.87 min; MS (ESlpos): m/z = 280 [M+H]⁺
¹H-NMR (400MHz, DMSO-d₆): δ [ppm]= 6.02 (s, 2H), 7.44 (s, 2H), 7.53 (d, 1H), 7.63 - 7.68 (m, 2H), 7.86 - 7.90 (m, 2H), 8.06 (d, 1H).

### Intermediate 159

### 4-(2-amino-5-bromopyridin-3-yl)-N-methylbenzene-1-sulfonamide

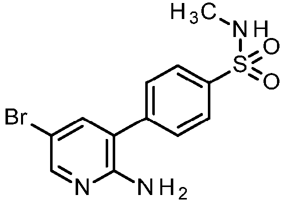

Intermediate 159 was prepared in analogy to Intermediate 144 using 5-bromo-3-iodopyridin-2-amine (250 mg, 836 µmol) and [4-(methylsulfamoyl)phenyl]boronic acid (212 mg, 987 µmol).
LC-MS (method 1): Rt = 0.98 min; MS (ESlpos): m/z = 342 [M+H]⁺

### Intermediate 160

### 4-(2-amino-5-bromopyridin-3-yl)-N,N-dimethylbenzene-1-sulfonamide

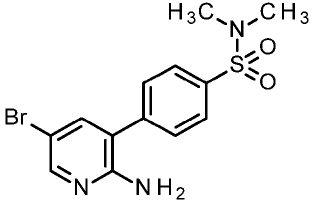

Intermediate 160 was prepared in analogy to Intermediate 144 using 5-bromo-3-iodopyridin-2-amine (250 mg, 836 µmol) and [4-(dimethylsulfamoyl)phenyl]boronic acid (226 mg, 987 µmol).
LC-MS (method 1): Rt = 1.11 min; MS (ESlpos): m/z = 358 [M+H]⁺
¹H-NMR (400MHz, DMSO-d₆): δ [ppm]= 2.62 - 2.69 (m, 6H), 6.08 (s, 2H), 7.56 (d, 1H), 7.68 - 7.73 (m, 2H), 7.77 - 7.82 (m, 2H), 8.07 (d, 1H).

### Intermediate 161

### 5-bromo-3-(1-methyl-1H-pyrazol-3-yl)pyridin-2-amine

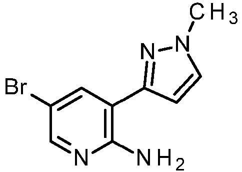

Intermediate 161 was prepared in analogy to Intermediate 144 using 5-bromo-3-iodopyridin-2-amine (250 mg, 836 µmol) and 1-methyl-3-(4,4,5,5-tetramethyl-1,3,2-dioxaborolan-2-yl)-1H-pyrazole (205 mg, 987 µmol).
LC-MS (method 1): Rt = 1.00 min; MS (ESlpos): m/z = 255 [M+H]⁺
¹H-NMR (400MHz, DMSO-d₆): δ [ppm]= 3.91 (s, 3H), 6.91 (d, 1H), 7.22 (br s, 2H), 7.80 (d, 1H), 7.96 (d, 1H), 8.05 (d, 1H).

### Intermediate 162

### 5-bromo-3-(1,2-thiazol-5-yl)pyridin-2-amine

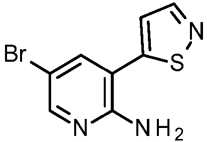

Intermediate 162 was prepared in analogy to Intermediate 144 using 5-bromo-3-iodopyridin-2-amine (250 mg, 836 µmol) and 5-(4,4,5,5-tetramethyl-1,3,2-dioxaborolan-2-yl)-1,2-thiazole (208 mg, 987 µmol).
LC-MS (method 1): Rt = 0.98 min; MS (ESlpos): m/z = 258 [M+H]⁺
¹H-NMR (400MHz, DMSO-d₆): δ [ppm]= 6.28 (s, 2H), 7.72 (d, 1H), 7.83 (d, 1H), 8.13 (d, 1H), 8.63 (d, 1H).

### Intermediate 163

### 5-bromo-3-(1,5-dimethyl-1H-pyrazol-4-yl)pyridin-2-amine

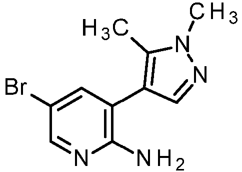

Intermediate 163 was prepared in analogy to Intermediate 144 using 5-bromo-3-iodopyridin-2-amine (250 mg, 836 µmol) and (1,5-dimethyl-1H-pyrazol-4-yl)boronic acid (138 mg, 987 µmol).
LC-MS (method 1): Rt = 0.90 min; MS (ESlpos): m/z = 269 [M+H]⁺
¹H-NMR (400MHz, DMSO-d₆): δ [ppm]= 2.15 (s, 3H), 3.76 (s, 3H), 5.76 (s, 2H), 7.34 (d, 1H), 7.41 (s, 1H), 7.95 (d, 1H).

### Intermediate 164

### 5-bromo-1'-methyl-1',2',3',6'-tetrahydro[3,4'-bipyridin]-2-amine

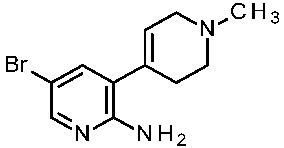

Intermediate 164 was prepared in analogy to Intermediate 144 using 5-bromo-3-iodopyridin-2-amine (250 mg, 836 µmol) and 1-methyl-4-(4,4,5,5-tetramethyl-1,3,2-dioxaborolan-2-yl)-1,2,3,6-tetrahydropyridine (224 mg, 1.00 mmol).
1H-NMR (400MHz, CHLOROFORM-d): δ [ppm]= 2.34 - 2.48 (m, 5H), 2.65 (t, 2H), 3.08 (q, 2H), 4.59 (br s, 2H), 5.86 (tt, 1H), 7.33 (d, 1H), 8.00 (d, 1H).
LC-MS (method 1): Rt = 0.97 min; MS (ESlpos): m/z = 270 [M+H]⁺

### Intermediate 165

### 5-(4,4,5,5-tetramethyl-1,3,2-dioxaborolan-2-yl)-3-(trifluoromethyl)pyridin-2-amine

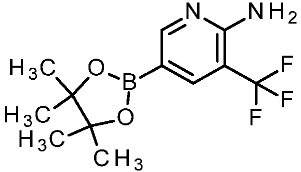

5-Bromo-3-(trifluoromethyl)pyridin-2-amine (2.00 g, 8.30 mmol) and 4,4,4',4',5,5,5',5'-octamethyl-2,2'-bi-1,3,2-dioxaborolane (2.53 g, 9.96 mmol) were solubilised in dioxane (43 mL), potassium acetate (2.44 g, 24.9 mmol) and Pd(dppf)Cl₂ (607 mg, 830 µmol) were added and the mixture was stirred overnight at 100°C. The mixture was diluted with dichloromethane, filtered and evaporated. The residue was purified by flash chromatography to give 2.70 g of the target compound.

1H-NMR (400 MHz, DMSO-d6) δ [ppm]: 1.052 (0.88), 1.065 (16.00), 1.069 (1.16), 1.145 (0.90), 1.154 (8.40), 1.163 (1.61), 1.176 (0.70), 1.269 (11.09), 1.290 (1.05), 3.332 (0.74), 3.936 (2.59), 7.799 (0.47), 7.803 (0.48), 7.939 (0.72), 8.375 (0.46), 8.377 (0.46).

### Intermediate 166

### 2-amino-5-(4,4,5,5-tetramethyl-1,3,2-dioxaborolan-2-yl)pyridine-3-carbonitrile

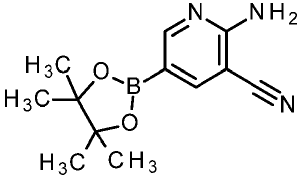

2-Amino-5-bromopyridine-3-carbonitrile (1.40 g, 7.07 mmol), 4,4,4',4',5,5,5',5'-octamethyl-2,2'-bi-1,3,2-dioxaborolane (1.97 g, 7.78 mmol), Pd(dppf)Cl₂ (289 mg, 353 µmol) and potassium acetate (2.08 g, 21.2 mmol) were stirred in degassed dioxane (35 mL) at 100°C overnight. The reaction mixture was filtered and used without further work up or purification in the next step.

¹H NMR (400 MHz, DMSO-*d*₆) δ ppm 1.27 (s, 12 H) 7.33 (s, 2 H) 7.92 (d, 1 H) 8.37 (d, 1 H).

### Intermediate 167

### 3-fluoro-5-(4,4,5,5-tetramethyl-1,3,2-dioxaborolan-2-yl)pyridin-2-amine

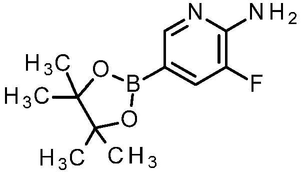

5-bromo-3-fluoropyridin-2-amine (75.2 mg, 394 µmol), 4,4,4',4',5,5,5',5'-octamethyl-2,2'-bi-1,3,2-dioxaborolane (110 mg, 433 µmol), Pd(dppf)Cl₂ (16.1 mg, 19.7 µmol), dppf (10.9 mg, 19.7 µmol) and KOAc (116 mg, 1.18 mmol) were stirred in degassed 1,4-dioxane (2.0 mL) overnight at 100°C. The mixture was filtered and the filtrate was concentrated under reduced pressure to give 124 mg of the title compound, which was used without further purification.
LC-MS (method 1): Rt = 0.54 min; MS (ESlpos): m/z = 239 [M+H]⁺
¹H-NMR (400 MHz, DMSO-d6) δ [ppm]: 1.066 (16.00), 1.260 (3.09).

### Intermediate 168

### 1-[2-amino-5-(4,4,5,5-tetramethyl-1,3,2-dioxaborolan-2-yl)pyridin-3-yl]-2,2,2-trifluoroethan-1-one

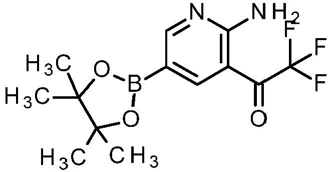

Intermediate 168 was prepared in analogy to Intermediate 167 using 1-(2-amino-5-bromopyridin-3-yl)-2,2,2-trifluoroethan-1-one (37.0 mg, 138 µmol).
LC-MS (method 1): Rt = 0.72 min; MS (ESlpos): m/z = 317 [M+H]⁺

### Intermediate 169

### (rac)- (6-amino-5-{[1-phenylethyl]sulfanyl}pyridin-3-yl)boronic acid

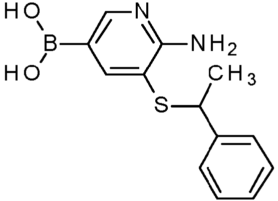

Intermediate 169 was prepared in analogy to Intermediate 167 using (rac)-5-bromo-3-{[1-phenylethyl]sulfanyl}pyridin-2-amine (50.0 mg, 162 µmol).
LC-MS (method 1): Rt = 0.79 min; MS (ESlpos): m/z = 275 [M+H]⁺

### Intermediate 170

### 3-[(1R)-1-(2,6-dichloro-3-fluorophenyl)ethoxy]-5-(4,4,5,5-tetramethyl-1,3,2-dioxaborolan-2-yl)pyridin-2-amine

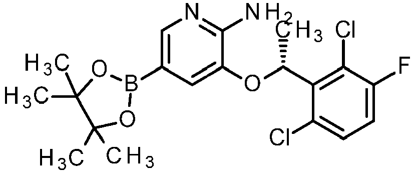

5-bromo-3-[(1R)-1-(2,6-dichloro-3-fluorophenyl)ethoxy]pyridin-2-amine (500 mg, 1.32 mmol), 4,4,4',4',5,5,5',5'-octamethyl-2,2'-bi-1,3,2-dioxaborolane (368 mg, 1.45 mmol), Pd(dppf)Cl₂ (53.7 mg, 65.8 µmol) and KOAc (387 mg, 3.9 mmol) were stirred in degassed 1,4-dioxane (13 mL) for 5 h at 100°C. The mixture was filtered and the filtrate was concentrated under reduced pressure to give 560 mg (100 % yield) of the title compound, which was used without further purification.
LC-MS (method 1): Rt = 1.38 min; MS (ESlpos): m/z = 427 [M+H]⁺

### Intermediate 171

[6-amino-5-(2-hydroxypropan-2-yl)pyridin-3-yl]boronic acid

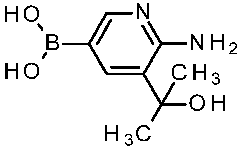

Intermediate 171 was prepared in analogy to Intermediate 170 using 2-(2-amino-5-bromopyridin-3-yl)propan-2-ol (300 mg, 1.30 mmol).
LC-MS (method 1): Rt = 0.74 min; MS (ESlpos): m/z = 197 [M+H]⁺
¹H-NMR (400 MHz, DMSO-d6) δ [ppm]: 1.066 (8.83), 1.156 (16.00), 1.164 (1.43), 1.255 (6.11), 1.276 (1.08), 1.292 (1.21), 1.466 (2.93), 1.492 (0.50), 3.566 (6.26), 3.941 (1.54), 5.409 (0.67), 6.469 (0.43), 6.548 (0.50), 7.943 (1.30).

### Intermediate 172

### {6-amino-5-[(cyclopentylmethyl)sulfanyl]pyridin-3-yl}boronic acid

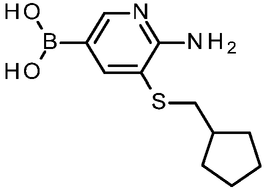

Intermediate 172 was prepared in analogy to Intermediate 170 using 5-bromo-3-[(cyclopentylmethyl)sulfanyl]pyridin-2-amine (79.0 mg, 275 µmol).
LC-MS (method 1): Rt = 0.82 min; MS (ESlpos): m/z = 253 [M+H]⁺

### Intermediate 173

### 3-(benzyloxy)-5-(4,4,5,5-tetramethyl-1,3,2-dioxaborolan-2-yl)pyridin-2-amine

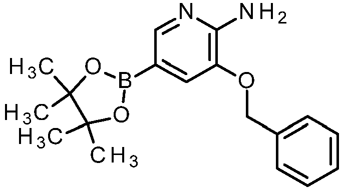

Intermediate 173 was prepared in analogy to Intermediate 170 using 3-(benzyloxy)-5-bromopyridin-2-amine (1.92 g, 6.88 mmol).

### Intermediate 174

### methyl 2-amino-5-(4,4,5,5-tetramethyl-1,3,2-dioxaborolan-2-yl)pyridine-3-carboxylate

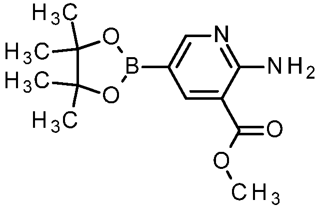

Intermediate 174 was prepared in analogy to Intermediate 170 using methyl 2-amino-5-bromopyridine-3-carboxylate (2.00 g, 8.66 mmol).

1H-NMR (400 MHz, DMSO-d6) δ [ppm]: 1.066 (14.18), 1.156 (16.00), 1.164 (1.99), 1.274 (7.97), 1.292 (0.30), 1.898 (0.69), 2.332 (0.23), 2.518 (1.32), 2.523 (0.79), 2.673 (0.23), 3.566 (0.16), 3.810 (0.62), 3.822 (3.20), 3.938 (1.56), 7.524 (0.55), 7.941 (0.63), 8.274 (0.48), 8.279 (0.55), 8.377 (0.58), 8.381 (0.56).

### Intermediate 175

### [6-amino-5-(methylsulfanyl)pyridin-3-yl]boronic acid

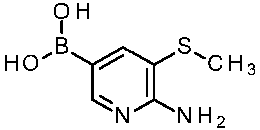

Intermediate 175 was prepared in analogy to Intermediate 170 using 5-bromo-3-(methylsulfanyl)pyridin-2-amine (680 mg, 3.10 mmol).

1H-NMR (400 MHz, DMSO-d6) δ [ppm]: 1.035 (0.65), 1.052 (1.36), 1.065 (12.03), 1.070 (1.06), 1.156 (16.00), 1.164 (15.62), 1.179 (0.19), 1.261 (3.77), 1.274 (0.23), 1.292 (0.84), 1.310 (0.30), 1.907 (0.31), 2.344 (1.86), 2.354 (0.33), 2.518 (0.44), 2.523 (0.29), 3.422 (0.27), 3.435 (0.28), 3.439 (0.27), 3.452 (0.28), 3.938 (1.80), 4.343 (0.19), 4.356 (0.37), 4.368 (0.18), 6.423 (0.21), 7.578 (0.24), 7.582 (0.24), 7.940 (1.14), 8.086 (0.23), 8.090 (0.22).

### Intermediate 176

### [6-amino-5-(3-fluorophenyl)pyridin-3-yl]boronic acid

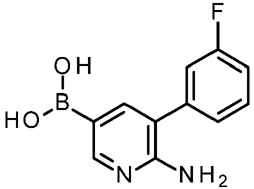

Intermediate 176 was prepared in analogy to Intermediate 170 using 5-bromo-3-(3-fluorophenyl)pyridin-2-amine (119 mg, 446 µmol).
LC-MS (method 2): Rt = 0.57 min; MS (ESlpos): m/z = 233 [M+H]⁺

### Intermediate 177

### [6-amino-5-(2-fluorophenyl)pyridin-3-yl]boronic acid

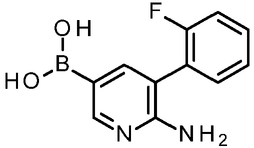

Intermediate 177 was prepared in analogy to Intermediate 170 using 5-bromo-3-(2-fluorophenyl)pyridin-2-amine (227 mg, 850 µmol).
LC-MS (method 2): Rt = 0.55 min; MS (ESlpos): m/z = 233 [M+H]⁺

### Intermediate 178

### [6-amino-5-(2-methoxyphenyl)pyridin-3-yl]boronic acid

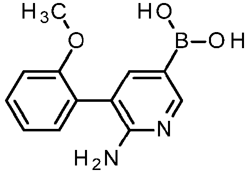

Intermediate 178 was prepared in analogy to Intermediate 170 using 5-bromo-3-(2-methoxyphenyl)pyridin-2-amine (85.0 mg, 305 µmol).
LC-MS (method 2): Rt = 0.58 min; MS (ESlpos): m/z = 245 [M+H]⁺

### Intermediate 179

### {6-amino-5-[3-(methanesulfonyl)phenyl]pyridin-3-yl}boronic acid

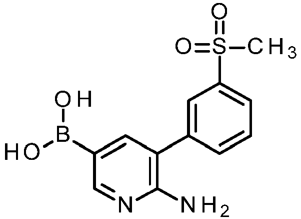

Intermediate 179 was prepared in analogy to Intermediate 170 using 5-bromo-3-[3-(methanesulfonyl)phenyl]pyridin-2-amine (100 mg, 306 µmol).
LC-MS (method 2): Rt = 0.46 min; MS (ESlpos): m/z = 293 [M+H]⁺

### Intermediate 180

### [6-amino-5-(phenylsulfanyl)pyridin-3-yl]boronic acid

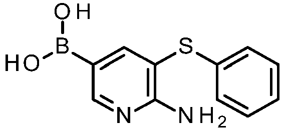

Intermediate 180 was prepared in analogy to Intermediate 170 using 5-bromo-3-(phenylsulfanyl)pyridin-2-amine (1.40 g, 4.98 mmol).
LC-MS (method 1): Rt = 0.59 min; MS (ESlpos): m/z = 247 [M+H]⁺

### Intermediate 181

### {6-amino-5-[4-(methanesulfonyl)phenyl]pyridin-3-yl}boronic acid

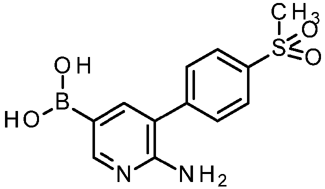

Intermediate 181 was prepared in analogy to Intermediate 170 using 5-bromo-3-[4-(methanesulfonyl)phenyl]pyridin-2-amine (200 mg, 611 µmol).
LC-MS (method 2): Rt = 0.44 min; MS (ESlpos): m/z = 293 [M+H]⁺

### Intermediate 182

### [5-(4-acetylphenyl)-6-aminopyridin-3-yl]boronic acid

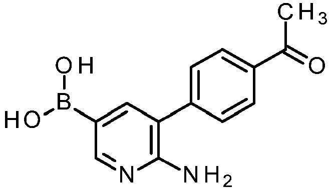

Intermediate 182 was prepared in analogy to Intermediate 170 using 1-[4-(2-amino-5-bromopyridin-3-yl)phenyl]ethan-1-one (240 mg, 824 µmol).
LC-MS (method 2): Rt = 0.52 min; MS (ESlpos): m/z = 257 [M+H]⁺

### Intermediate 183

### {6-amino-5-[4-(methanesulfinyl)phenyl]pyridin-3-yl}boronic acid

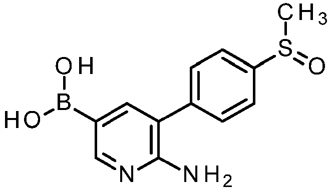

Intermediate 183 was prepared in analogy to Intermediate 170 using 5-bromo-3-[4-(methanesulfinyl)phenyl]pyridin-2-amine (240 mg, 771 µmol).
LC-MS (method 2): Rt = 0.43 min; MS (ESlpos): m/z = 277 [M+H]⁺

### Intermediate 184

### [6-amino-5-(3-methoxyphenyl)pyridin-3-yl]boronic acid

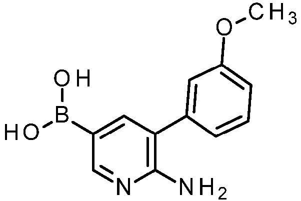

Intermediate 184 was prepared in analogy to Intermediate 170 using 5-bromo-3-(3-methoxyphenyl)pyridin-2-amine (200 mg, 716 µmol).
LC-MS (method 2): Rt = 0.61 min; MS (ESlpos): m/z = 245 [M+H]⁺

### Intermediate 185

### {6-amino-5-[4-(cyclopropanesulfonyl)phenyl]pyridin-3-yl}boronic acid

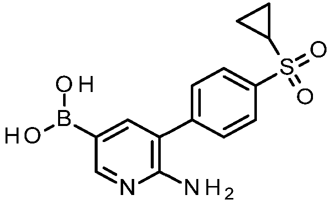

Intermediate 185 was prepared in analogy to Intermediate 170 using 5-bromo-3-[4-(cyclopropanesulfonyl)phenyl]pyridin-2-amine (220 mg, 623 µmol).
LC-MS (method 2): Rt = 0.55 min; MS (ESlpos): m/z = 319 [M+H]⁺

### Intermediate 186

### [6-amino-5-(4-sulfamoylphenyl)pyridin-3-yl]boronic acid

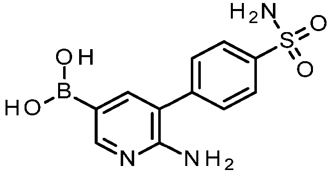

Intermediate 186 was prepared in analogy to Intermediate 170 using 4-(2-amino-5-bromopyridin-3-yl)benzene-1-sulfonamide (150 mg, 457 µmol).
LC-MS (method 2): Rt = 0.46 min; MS (ESlpos): m/z = 294 [M+H]⁺

### Intermediate 187

### {6-amino-5-[4-(methylsulfamoyl)phenyl]pyridin-3-yl}boronic acid

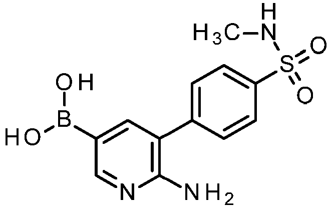

Intermediate 187 was prepared in analogy to Intermediate 170 using 4-(2-amino-5-bromopyridin-3-yl)-N,N-dimethylbenzene-1-sulfonamide (220 mg, 618 µmol).
LC-MS (method 2): Rt = 0.47 min; MS (ESlpos): m/z = 308 [M+H]⁺

### Intermediate 188

### {6-amino-5-[4-(dimethylsulfamoyl)phenyl]pyridin-3-yl}boronic acid

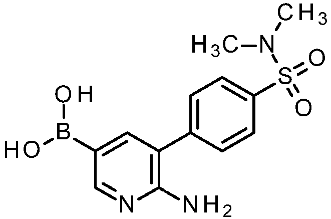

Intermediate 188 was prepared in analogy to Intermediate 170 using 4-(2-amino-5-bromopyridin-3-yl)-N,N-dimethylbenzene-1-sulfonamide (150 mg, 421 µmol).
LC-MS (method 2): Rt = 0.57 min; MS (ESlpos): m/z = 322 [M+H]⁺

### Intermediate 189

### [6-amino-5-(cyclohexylmethoxy)pyridin-3-yl]boronic acid

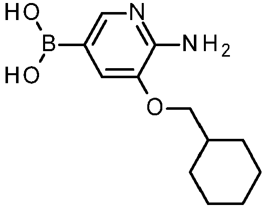

Intermediate 189 was prepared in analogy to Intermediate 170 using 5-bromo-3-(cyclohexylmethoxy)pyridin-2-amine (45.0 mg, 158 µmol).
LC-MS (method 2): Rt = 0.80 min; MS (ESlpos): m/z = 251 [M+H]⁺

### Intermediate 190

### {6-amino-5-[(pyridin-3-yl)methoxy]pyridin-3-yl}boronic acid

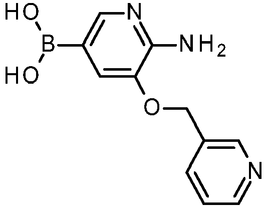

Intermediate 190 was prepared in analogy to Intermediate 170 using 5-bromo-3-[(pyridin-3-yl)methoxy]pyridin-2-amine (100 mg, 357 µmol).
LC-MS (method 1): Rt = 0.63 min; MS (ESlpos): m/z = 246 [M+H]⁺

### Intermediate 191

### {6-amino-5-[(4-fluorophenyl)methoxy]pyridin-3-yl}boronic acid

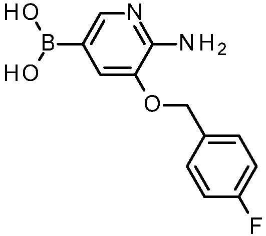

Intermediate 191 was prepared in analogy to Intermediate 170 using 5-bromo-3-[(4-fluorophenyl)methoxy]pyridin-2-amine (30.0 mg, 101 µmol).
LC-MS (method 1): Rt = 0.65 min; MS (ESlpos): m/z = 263 [M+H]⁺

### Intermediate 192

### (rac)-[6-amino-5-(1-phenylethoxy)pyridin-3-yl]boronic acid

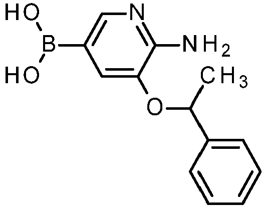

Intermediate 192 was prepared in analogy to Intermediate 170 using (rac)-5-bromo-3-(1-phenylethoxy)pyridin-2-amine (95.0 mg, 324 µmol).
LC-MS (method 1): Rt = 0.74 min; MS (ESlpos): m/z = 259 [M+H]⁺

### Intermediate 193

### [6-amino-5-(piperidin-1-yl)pyridin-3-yl]boronic acid

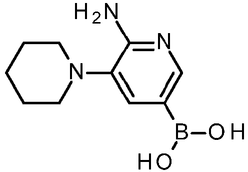

Intermediate 193 was prepared in analogy to Intermediate 170 using 5-bromo-3-(piperidin-1-yl)pyridin-2-amine (125 mg, 488 µmol).
LC-MS (method 1): Rt = 0.64 min; MS (ESlpos): m/z = 222 [M+H]⁺

### Intermediate 194

### {6-amino-5-[(1R)-1-(pyridin-4-yl)ethoxy]pyridin-3-yl}boronic acid

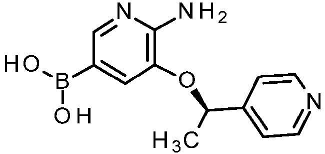

Intermediate 194 was prepared in analogy to Intermediate 170 using 5-bromo-3-[(1R)-1-(pyridin-4-yl)ethoxy]pyridin-2-amine (60.0 mg, 204 µmol).
LC-MS (method 1): Rt = 0.47 min; MS (ESlpos): m/z = 260 [M+H]⁺

### Intermediate 195

### {6-amino-5-[(1S)-1-(pyridin-4-yl)ethoxy]pyridin-3-yl}boronic acid

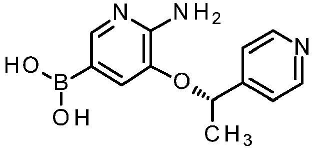

Intermediate 195 was prepared in analogy to Intermediate 170 using 5-bromo-3-[(1S)-1-(pyridin-4-yl)ethoxy]pyridin-2-amine (100 mg, 340 µmol).
LC-MS (method 1): Rt = 0.44 min; MS (ESlpos): m/z = 260 [M+H]⁺

### Intermediate 196

### [6-amino-5-(1-methyl-1H-pyrazol-3-yl)pyridin-3-yl]boronic acid

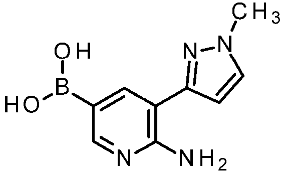

Intermediate 196 was prepared in analogy to Intermediate 170 using 5-bromo-3-(1-methyl-1H-pyrazol-3-yl)pyridin-2-amine (150 mg, 593 µmol).
LC-MS (method 2): Rt = 0.49 min; MS (ESlpos): m/z = 219 [M+H]⁺

### Intermediate 197

### [6-amino-5-(1,2-thiazol-5-yl)pyridin-3-yl]boronic acid

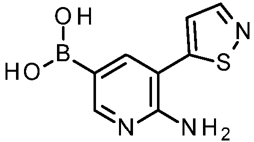

Intermediate 197 was prepared in analogy to Intermediate 170 using 5-bromo-3-(1,2-thiazol-5-yl)pyridin-2-amine (120 mg, 469 µmol).
LC-MS (method 2): Rt = 0.44 min; MS (ESlpos): m/z = 222 [M+H]⁺

### Intermediate 198

### [6-amino-5-(1,5-dimethyl-1H-pyrazol-4-yl)pyridin-3-yl]boronic acid

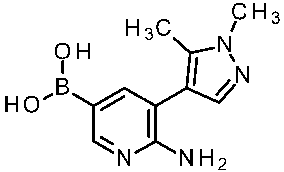

Intermediate 198 was prepared in analogy to Intermediate 170 using 5-bromo-3-(1,5-dimethyl-1H-pyrazol-4-yl)pyridin-2-amine (100 mg, 374 µmol).
LC-MS (method 2): Rt = 0.45 min; MS (ESlpos): m/z = 233 [M+H]⁺

### Intermediate 199

### {6-amino-5-[(trifluoromethyl)sulfanyl]pyridin-3-yl}boronic acid

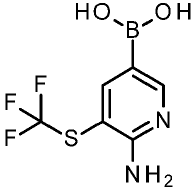

Intermediate 199 was prepared in analogy to Intermediate 170 using 5-bromo-3-[(trifluoromethyl)sulfanyl]pyridin-2-amine (1.25 g, 4.58 mmol).
LC-MS (method 2): Rt = 0.57 min; MS (ESlpos): m/z = 239 [M+H]⁺

### Intermediate 200

### 3-[(1R)-1-(2-chlorophenyl)ethoxy]-5-(4,4,5,5-tetramethyl-1,3,2-dioxaborolan-2-yl)pyridin-2-amine

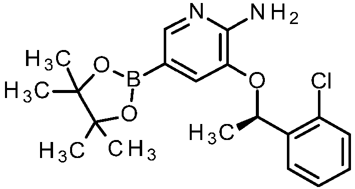

Intermediate 200 was prepared in analogy to Intermediate 170 using 5-bromo-3-[(1R)-1-(2-chlorophenyl)ethoxy]pyridin-2-amine (154 mg, 470 µmol).
LC-MS (method 1): Rt = 1.32 min; MS (ESlpos): m/z = 375 [M+H]⁺

### Intermediate 201

### 3-[(1S)-1-(2-chlorophenyl)ethoxy]-5-(4,4,5,5-tetramethyl-1,3,2-dioxaborolan-2-yl)pyridin-2-amine

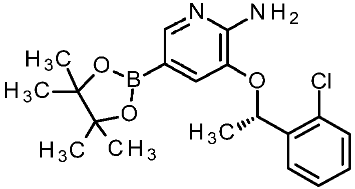

Intermediate 201 was prepared in analogy to Intermediate 170 using 5-bromo-3-[(1S)-1-(2-chlorophenyl)ethoxy]pyridin-2-amine (87.0 mg, 266 µmol).
LC-MS (method 1): Rt = 1.33 min; MS (ESlpos): m/z = 375 [M+H]⁺

### Intermediate 202

### 3-[(1R)-1-(pyridin-2-yl)ethoxy]-5-(4,4,5,5-tetramethyl-1,3,2-dioxaborolan-2-yl)pyridin-2-amine

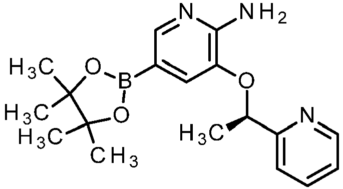

Intermediate 202 was prepared in analogy to Intermediate 170 using 5-bromo-3-[(1R)-1-(pyridin-2-yl)ethoxy]pyridin-2-amine (250 mg, 90 % purity, 765 µmol).
LC-MS (method 1): Rt = 0.87 min; MS (ESlpos): m/z = 342 [M+H]⁺

### Intermediate 203

### 3-{[1-(pyrimidin-5-yl)ethyl]oxy}-5-(4,4,5,5-tetramethyl-1,3,2-dioxaborolan-2-yl)pyridin-2-amine (stereoisomer 1)

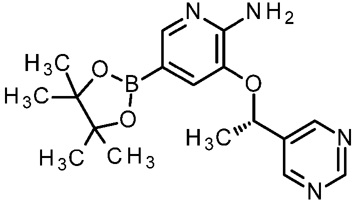

Intermediate 203 was prepared in analogy to Intermediate 170 using 5-bromo-3-{[1-(pyrimidin-5-yl)ethyl]oxy}pyridin-2-amine (100 mg, 339 µmol, stereoisomer 1).
LC-MS (method 1): Rt = 0.63 min; MS (ESlpos): m/z = 343 [M+H]⁺

### Intermediate 204

### 3-{[1-(pyrimidin-5-yl)ethyl]oxy}-5-(4,4,5,5-tetramethyl-1,3,2-dioxaborolan-2-yl)pyridin-2-amine (stereoisomer 2)

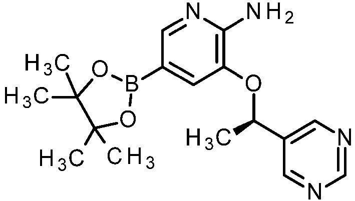

Intermediate 204 was prepared in analogy to Intermediate 170 using 5-bromo-3-{[1-(pyrimidin-5-yl)ethyl]oxy}pyridin-2-amine (100 mg, 339 µmol, stereoisomer 2).
LC-MS (method 1): Rt = 0.63 min; MS (ESlpos): m/z = 343 [M+H]⁺

### Intermediate 205

### [6-amino-5-(difluoromethoxy)pyridin-3-yl]boronic acid

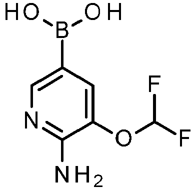

Intermediate 205 was prepared in analogy to Intermediate 170 using 5-bromo-3-(difluoromethoxy)pyridin-2-amine (3.00 g, 12.6 mmol).
LC-MS (method 2): Rt = 0.46 min; MS (ESlpos): m/z = 205 [M+H]⁺

### Intermediate 206

### [6-amino-5-(trifluoromethoxy)pyridin-3-yl]boronic acid

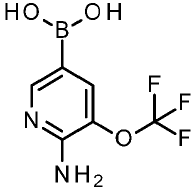

Intermediate 206 was prepared in analogy to Intermediate 170 using 5-bromo-3-(trifluoromethoxy)pyridin-2-amine (3.60 g, 14.0 mmol).
LC-MS (method 2): Rt = 0.50 min; MS (ESlpos): m/z = 223 [M+H]⁺

### Intermediate 207

### 3-[(2,6-dichlorophenyl)methoxy]-5-(4,4,5,5-tetramethyl-1,3,2-dioxaborolan-2-yl)pyridin-2-amine

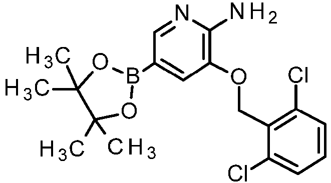

Intermediate 207 was prepared in analogy to Intermediate 170 using 5-bromo-3-[(2,6-dichlorophenyl)methoxy]pyridin-2-amine (393 mg, 1.13 mmol).
LC-MS (method 1): Rt = 1.32 min; MS (ESlpos): m/z = 395 [M+H]⁺

### Intermediate 208

### 3-[(2-fluorophenyl)methoxy]-5-(4,4,5,5-tetramethyl-1,3,2-dioxaborolan-2-yl)pyridin-2-amine

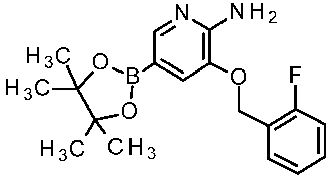

Intermediate 208 was prepared in analogy to Intermediate 170 using 5-bromo-3-[(2-fluorophenyl)methoxy]pyridin-2-amine (373 mg, 1.26 mmol).
LC-MS (method 1): Rt = 1.18 min; MS (ESlpos): m/z = 345 [M+H]⁺

### Intermediate 209

### 3-[(3-fluorophenyl)methoxy]-5-(4,4,5,5-tetramethyl-1,3,2-dioxaborolan-2-yl)pyridin-2-amine

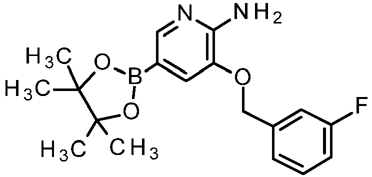

Intermediate 209 was prepared in analogy to Intermediate 170 using 5-bromo-3-[(3-fluorophenyl)methoxy]pyridin-2-amine (345 mg, 1.16 mmol).
LC-MS (method 1): Rt = 1.15 min; MS (ESlpos): m/z = 345 [M+H]⁺

### Intermediate 210

### 3-[(2-chlorophenyl)methoxy]-5-(4,4,5,5-tetramethyl-1,3,2-dioxaborolan-2-yl)pyridin-2-amine

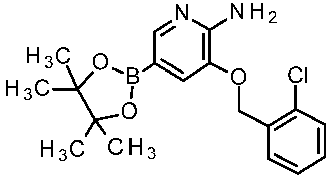

Intermediate 210 was prepared in analogy to Intermediate 170 using 5-bromo-3-[(2-chlorophenyl)methoxy]pyridin-2-amine (270 mg, 861 µmol).
LC-MS (method 1): Rt = 1.29 min; MS (ESlpos): m/z = 361 [M+H]⁺

### Intermediate 211

### 3-[(pyridin-2-yl)methoxy]-5-(4,4,5,5-tetramethyl-1,3,2-dioxaborolan-2-yl)pyridin-2-amine

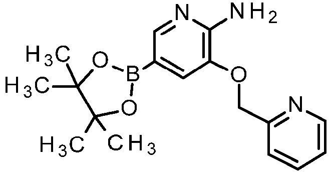

Intermediate 211 was prepared in analogy to Intermediate 170 using 5-bromo-3-[(pyridin-2-yl)methoxy]pyridin-2-amine (438 mg, 1.56 mmol).
LC-MS (method 1): Rt = 0.79 min; MS (ESlpos): m/z = 328 [M+H]⁺

### Intermediate 212

### 3-[(2-chloro-5-fluorophenyl)methoxy]-5-(4,4,5,5-tetramethyl-1,3,2-dioxaborolan-2-yl)pyridin-2-amine

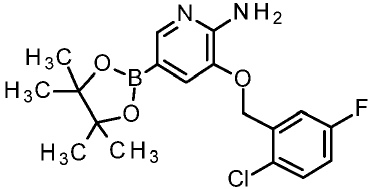

Intermediate 212 was prepared in analogy to Intermediate 170 using 5-bromo-3-[(2-chloro-5-fluorophenyl)methoxy]pyridin-2-amine (480 mg, 1.45 mmol).
LC-MS (method 1): Rt = 1.26 min; MS (ESlpos): m/z = 379 [M+H]⁺

### Intermediate 213

### 3-[(pyridazin-3-yl)methoxy]-5-(4,4,5,5-tetramethyl-1,3,2-dioxaborolan-2-yl)pyridin-2-amine

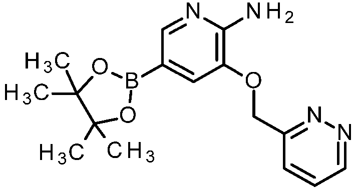

Intermediate 213 was prepared in analogy to Intermediate 170 using 5-bromo-3-[(pyridazin-3-yl)methoxy]pyridin-2-amine (180 mg, 640 µmol).
LC-MS (method 1): Rt = 0.54 min; MS (ESlpos): m/z = 329 [M+H]⁺

### Intermediate 214

### 3-[(1S)-1-(pyridin-2-yl)ethoxy]-5-(4,4,5,5-tetramethyl-1,3,2-dioxaborolan-2-yl)pyridin-2-amine

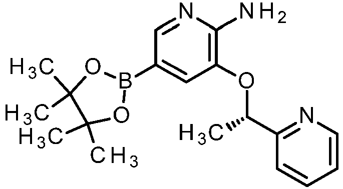

Intermediate 214 was prepared in analogy to Intermediate 170 using 5-bromo-3-[(1S)-1-(pyridin-2-yl)ethoxy]pyridin-2-amine (457 mg, 79 % purity, 1.23 mmol).
LC-MS (method 1): Rt = 0.86 min; MS (ESlpos): m/z = 342 [M+H]⁺

### Intermediate 215

### {6-amino-5-[(3-methylphenyl)methoxy]pyridin-3-yl}boronic acid

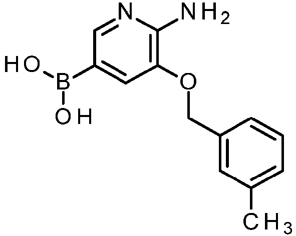

Intermediate 215 was prepared in analogy to Intermediate 170 using 5-bromo-3-[(3-methylphenyl)methoxy]pyridin-2-amine (30.0 mg, 102 µmol).
LC-MS (method 1): Rt = 0.74 min; MS (ESlpos): m/z = 259 [M+H]⁺
¹H-NMR (400 MHz, DMSO-d6) δ [ppm]: 1.066 (0.74), 1.156 (9.51), 1.165 (16.00), 1.261 (1.16), 1.293 (0.46), 3.566 (6.30).

### Intermediate 216

### 3-[(1-methyl-1H-pyrazol-5-yl)methoxy]-5-(4,4,5,5-tetramethyl-1,3,2-dioxaborolan-2-yl)pyridin-2-amine

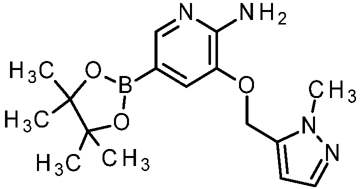

Intermediate 216 was prepared in analogy to Intermediate 170 using 5-bromo-3-[(1-methyl-1H-pyrazol-5-yl)methoxy]pyridin-2-amine (230 mg, 812 µmol).
LC-MS (method 1): Rt = 0.76 min; MS (ESlpos): m/z = 331 [M+H]⁺
¹H-NMR (400 MHz, DMSO-d6) δ [ppm]: 1.066 (5.65), 1.156 (2.20), 1.165 (16.00), 1.172 (0.46), 1.272 (1.68), 3.566 (6.36), 3.846 (1.83), 3.939 (0.86).

### Intermediate 217

### 3-[(1S)-1-(1-methyl-1H-pyrazol-3-yl)ethoxy]-5-(4,4,5,5-tetramethyl-1,3,2-dioxaborolan-2-yl)pyridin-2-amine

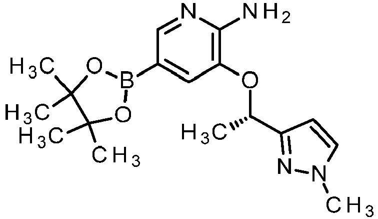

Intermediate 217 was prepared in analogy to Intermediate 170 using 5-bromo-3-[(1S)-1-(1-methyl-1H-pyrazol-3-yl)ethoxy]pyridin-2-amine (149 mg, 501 µmol).
LC-MS (method 1): Rt = 0.84 min; MS (ESlpos): m/z = 345 [M+H]⁺

### Intermediate 218

### 3-[(1R)-1-(1-methyl-1H-pyrazol-3-yl)ethoxy]-5-(4,4,5,5-tetramethyl-1,3,2-dioxaborolan-2-yl)pyridin-2-amine

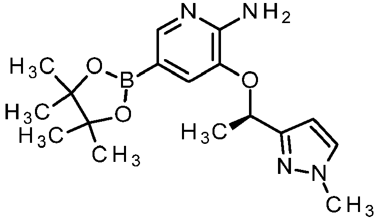

Intermediate 218 was prepared in analogy to Intermediate 170 using 5-bromo-3-[(1R)-1-(1-methyl-1H-pyrazol-3-yl)ethoxy]pyridin-2-amine (136 mg, 458 µmol).
LC-MS (method 1): Rt = 0.85 min; MS (ESlpos): m/z = 345 [M+H]⁺

### Intermediate 219

### 1'-methyl-5-(4,4,5,5-tetramethyl-1,3,2-dioxaborolan-2-yl)-1',2',3',6'-tetrahydro[3,4'-bipyridin]-2-amine

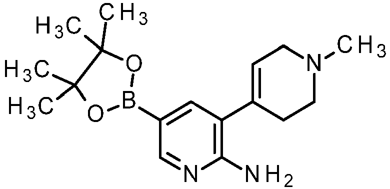

Intermediate 219 was prepared in analogy to Intermediate 170 using 5-bromo-1'-methyl-1',2',3',6'-tetrahydro[3,4'-bipyridin]-2-amine (168 mg, 626 µmol).
LC-MS (method 1): Rt = 0.95 min; MS (ESlpos): m/z = 316 [M+H]⁺

### Intermediate 220

### 3-[(1S)-1-phenylethoxy]-5-(4,4,5,5-tetramethyl-1,3,2-dioxaborolan-2-yl)pyridin-2-amine

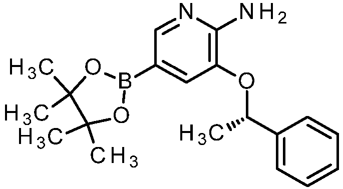

Intermediate 220 was prepared in analogy to Intermediate 170 using 5-bromo-3-[(1S)-1-phenylethoxy]pyridin-2-amine (160 mg, 90 % purity, 491 µmol).
LC-MS (method 1): Rt = 1.22 min; MS (ESlpos): m/z = 341 [M+H]⁺

### Intermediate 221

### 3-[(1R)-1-phenylethoxy]-5-(4,4,5,5-tetramethyl-1,3,2-dioxaborolan-2-yl)pyridin-2-amine

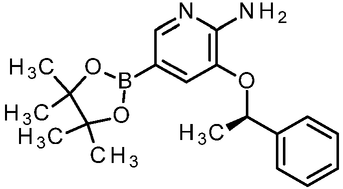

Intermediate 221 was prepared in analogy to Intermediate 170 using 5-bromo-3-[(1R)-1-phenylethoxy]pyridin-2-amine (160 mg, 90 % purity, 491 µmol).

1H-NMR (400 MHz, DMSO-d6) δ [ppm]: 1.066 (16.00), 1.156 (4.55), 1.164 (1.32), 1.172 (0.27), 1.213 (1.79), 1.217 (1.81), 1.275 (0.59), 1.292 (0.76), 1.520 (0.51), 1.536 (0.52), 1.548 (0.19), 1.564 (0.18), 1.987 (0.36), 2.518 (0.35), 2.523 (0.24), 3.566 (6.41), 3.940 (1.44), 6.960 (0.20), 6.963 (0.20), 7.252 (0.17), 7.319 (0.19), 7.334 (0.19), 7.338 (0.32), 7.356 (0.18), 7.436 (0.32), 7.454 (0.21).

### Intermediate 222

### [6-amino-5-(difluoromethyl)pyridin-3-yl]boronic acid

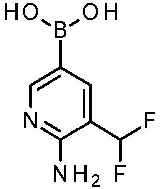

Intermediate 222 was prepared in analogy to Intermediate 170 using 5-bromo-3-(difluoromethyl)pyridin-2-amine (545 mg, 2.44 mmol).

1H-NMR (400 MHz, DMSO-d6) δ [ppm]: 1.065 (1.00), 1.156 (16.00), 1.164 (9.80), 1.267 (6.85), 1.292 (0.78), 1.899 (0.52), 3.565 (3.03), 6.674 (0.44), 7.008 (0.29), 7.782 (0.30), 7.943 (0.33), 8.289 (0.28).

### Intermediate 223

### {6-amino-5-[(cyclohexylmethyl)sulfanyl]pyridin-3-yl}boronic acid

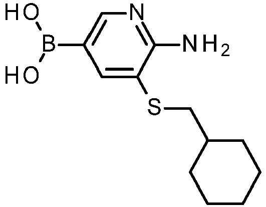

5-bromo-3-[(cyclohexylmethyl)sulfanyl]pyridin-2-amine (324 mg, 1.08 mmol), 4,4,4',4',5,5,5',5'-octamethyl-2,2'-bi-1,3,2-dioxaborolane (300 mg, 1.18 mmol), Pd(dppf)Cl2*DCM (317 mg, 3.23 mmol) and KOAc (317 mg, 3.2 mmol) were stirred in degassed 1,4-dioxane (5.5 mL) for 2 h at 100°C. The mixture was filtered and the filtrate was concentrated under reduced pressure to give 731 mg of the title compound, which was used without further purification.
¹H-NMR (400MHz, DMSO-d6): δ [ppm]= 0.90 - 1.03 (m, 2H), 1.08 - 1.20 (m, 15H), 1.31 - 1.40 (m, 2H), 1.54 - 1.62 (m, 1H), 1.62 - 1.70 (m, 2H), 1.76 - 1.84 (m, 2H), 2.65 (d, 2H), 6.47 (s, 2H), 7.63 (d, 1H), 7.94 (s, 1H), 8.11 (d, 1H).
LC-MS (method 1): Rt = 0.97 min; MS (ESlpos): m/z = 267 [M+H]⁺

### Intermediate 224

### 3-(benzylsulfanyl)-5-(4,4,5,5-tetramethyl-1,3,2-dioxaborolan-2-yl)pyridin-2-amine

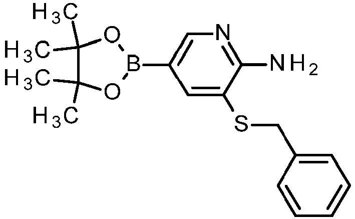

Intermediate 224 was prepared in analogy to Intermediate 223 using 3-(benzylsulfanyl)-5-bromopyridin-2-amine (112 mg, 379 µmol).
1H-NMR (400MHz, DMSO-d6): δ [ppm]= 1.24 (s, 12H), 3.98 (s, 2H), 6.55 (s, 2H), 7.18 - 7.29 (m, 5H), 7.48 (d, 1H), 7.94 (s, 1H), 8.11 (d, 1H).
LC-MS (method 1): Rt = 1.25 min; MS (ESlpos): m/z = 343 [M+H]⁺

### Intermediate 225

### (rac)-(6-amino-5-{[1-cyclohexylethyl]sulfanyl}pyridin-3-yl)boronic acid

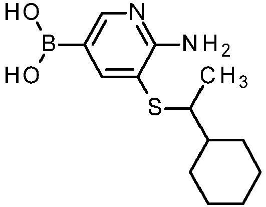

Intermediate 225 was prepared in analogy to Intermediate 223 using (rac)-5-bromo-3-{[1-cyclohexylethyl]sulfanyl}pyridin-2-amine (64.0 mg, 203 µmol).
LC-MS (method 1): Rt = 1.06 min; MS (ESlpos): m/z = 281 [M+H]⁺
¹H-NMR (400 MHz, DMSO-d6) δ [ppm]: 1.066 (1.86), 1.077 (0.98), 1.094 (1.09), 1.114 (0.48), 1.133 (0.42), 1.156 (16.00), 1.258 (4.86), 3.565 (12.80), 7.657 (0.44), 7.661 (0.46), 7.941 (1.28), 8.146 (0.43), 8.150 (0.43).

### Intermediate 226

### {6-amino-5-[(1R)-1-(pyridin-3-yl)ethoxy]pyridin-3-yl}boronic acid

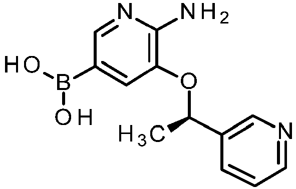

Intermediate 226 was prepared in analogy to Intermediate 223 using 5-bromo-3-[(1R)-1-(pyridin-3-yl)ethoxy]pyridin-2-amine (76.0 mg, 258 µmol).
¹H-NMR (400MHz, DMSO-d6): δ [ppm]= 1.23 (s, 12H), 1.55 (d, 3H), 5.66 (q, 1H), 6.27 (s, 2H), 7.05 (d, 1H), 7.38 (dd, 1H), 7.78 (d, 1H), 7.91 (dt, 1H), 8.48 (dd, 1H), 8.69 (d, 1H).
LC-MS (method 1): Rt = 0.49 min; MS (ESlpos): m/z = 260 [M+H]+

### Intermediate 227

### {6-amino-5-[(1S)-1-(pyridin-3-yl)ethoxy]pyridin-3-yl}boronic acid

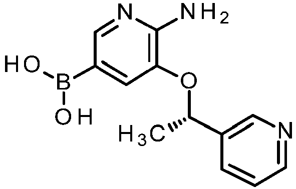

Intermediate 227 was prepared in analogy to Intermediate 223 using 5-bromo-3-[(1S)-1-(pyridin-3-yl)ethoxy]pyridin-2-amine (110 mg, 374 µmol).
¹H-NMR (400MHz, DMSO-d6): δ [ppm]= 1.23 (s, 12H), 1.55 (d, 3H), 5.66 (q, 1H), 6.27 (s, 2H), 7.05 (d, 1H), 7.35 - 7.41 (m, 1H), 7.78 (d, 1H), 7.91 (dt, 1H), 8.48 (dd, 1H), 8.69 (d, 1H).
LC-MS (method 1): Rt = 0.49 min; MS (ESlpos): m/z = 260 [M+H]⁺

### Intermediate 228

### (6-amino-5-phenylpyridin-3-yl)boronic acid

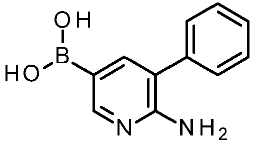

Intermediate 228 was prepared in analogy to Intermediate 223 using 5-bromo-3-phenylpyridin-2-amine (175 mg, 702 µmol).
LC-MS (method 2): Rt = 0.52 min; MS (ESlpos): m/z = 215 [M+H]⁺

### Intermediate 229

### [6-amino-5-(4-methylphenyl)pyridin-3-yl]boronic acid

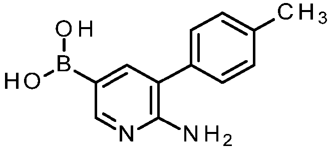

Intermediate 229 was prepared in analogy to Intermediate 223 using 5-bromo-3-(4-methylphenyl)pyridin-2-amine (190 mg, 722 µmol).
LC-MS (method 1): Rt = 0.65 min; MS (ESlpos): m/z = 229 [M+H]⁺

### Intermediate 230

### [6-amino-5-(2-methylphenyl)pyridin-3-yl]boronic acid

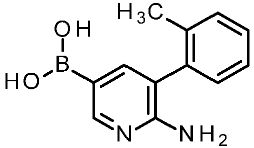

Intermediate 230 was prepared in analogy to Intermediate 223 using 5-bromo-3-(2-methylphenyl)pyridin-2-amine (191 mg, 726 µmol).
LC-MS (method 2): Rt = 0.61 min; MS (ESlpos): m/z = 229 [M+H]⁺

### Intermediate 231

### [6-amino-5-(3-methylphenyl)pyridin-3-yl]boronic acid

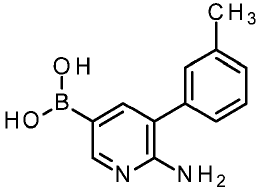

Intermediate 231 was prepared in analogy to Intermediate 223 using 5-bromo-3-(3-methylphenyl)pyridin-2-amine (187 mg, 711 µmol).
LC-MS (method 2): Rt = 0.66 min; MS (ESlpos): m/z = 229 [M+H]⁺

### Intermediate 232

### 5-(4,4,5,5-tetramethyl-1,3,2-dioxaborolan-2-yl)[3,3'-bipyridin]-2-amine

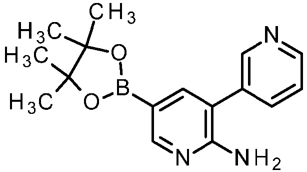

Intermediate 232 was prepared in analogy to Intermediate 223 using 5-bromo[3,3'-bipyridin]-2-amine (225 mg, 900 µmol).

1H-NMR (400 MHz, DMSO-d6) δ [ppm]: 1.066 (3.47), 1.156 (15.42), 1.164 (11.69), 1.263 (4.98), 1.292 (0.70), 1.311 (0.22), 1.905 (0.78), 2.518 (0.75), 2.523 (0.52), 3.566 (16.00), 3.938 (0.45), 6.224 (0.34), 7.425 (0.35), 7.429 (0.36), 7.939 (0.73), 8.228 (0.38), 8.233 (0.39), 8.557 (0.17), 8.561 (0.19), 8.569 (0.18), 8.573 (0.17), 8.586 (0.22), 8.590 (0.21).

### Intermediate 233

### (2-amino[3,4'-bipyridin]-5-yl)boronic acid

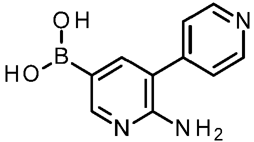

Intermediate 233 was prepared in analogy to Intermediate 223 using 5-bromo[3,4'-bipyridin]-2-amine (190 mg, 760 µmol).
LC-MS (method 1): Rt = 0.67 min; MS (ESlpos): m/z = 216 [M+H]⁺

### Intermediate 234

### (rac)-3-[1-(5-chloro-3-fluoropyridin-2-yl)ethoxy]-5-(4,4,5,5-tetramethyl-1,3,2-dioxaborolan-2-yl)pyridin-2-amine

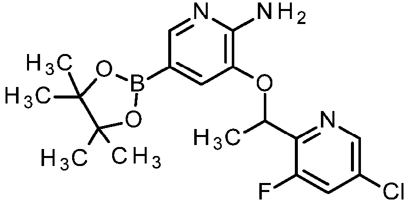

Intermediate 234 was prepared in analogy to Intermediate 223 using (rac)-5-bromo-3-[1-(5-chloro-3-fluoropyridin-2-yl)ethoxy]pyridin-2-amine (300 mg, 866 µmol).
LC-MS (method 1): Rt = 1.12 min; MS (ESlpos): m/z = 394 [M+H]⁺

### Intermediate 235

### (rac)-tert-butyl-2'-{6-amino-5-[(trifluoromethyl)sulfanyl]pyridin-3-yl}-5',6'-dihydrospiro[pyrrolidine-3,4'-pyrrolo[1,2-b]pyrazole]-1-carboxylate

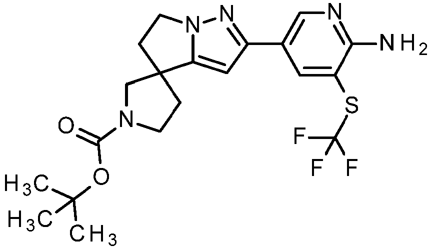

(rac)-tert-butyl-2'-[(trifluoromethanesulfonyl)oxy]-5',6'-dihydrospiro[pyrrolidine-3,4'-pyrrolo[1,2-b]pyrazole]-1-carboxylate (1.61 g, 3.92 mmol) was solubilised in 1,4-dioxane (33 mL) under nitrogen, {6-amino-5-[(trifluoromethyl)sulfanyl]pyridin-3-yl}boronic acid (1.40 g, 5.88 mmol), K3PO4 (23.5 mL, 0.50 M, 11.76 mmol) and XPhos Pd G2 (309 mg, 392 µmol) were added and the mixture was stirred overnight at 100°C. The mixture was diluted with ethyl acetate and washed with brine. The organic layer was dried over a silicone filter and evaporated. The residue was purified by flash chromatography and preparative HPLC to give 1.20 g (67 % yield) of the title compound.
LC-MS (method 1): Rt = 1.27 min; MS (ESlpos): m/z = 456 [M+H]⁺
¹H-NMR (400 MHz, DMSO-d6) δ [ppm]: 1.395 (16.00), 1.433 (13.07), 2.034 (1.64), 2.047 (1.52), 2.074 (1.50), 2.084 (1.38), 3.283 (0.48), 3.408 (6.28), 3.493 (0.69), 3.511 (1.27), 3.537 (0.80), 4.090 (2.05), 4.173 (2.19), 4.180 (2.13), 5.766 (0.49), 6.432 (1.68), 6.457 (2.12), 6.737 (5.20), 7.538 (0.74), 7.645 (0.51), 8.050 (2.87), 8.523 (3.46), 8.529 (3.50).

### Intermediate 236

### (rac)-tert-butyl-2'-[6-amino-5-(trifluoromethoxy)pyridin-3-yl]-5',6'-dihydrospiro[pyrrolidine-3,4'-pyrrolo[1,2-b]pyrazole]-1-carboxylate

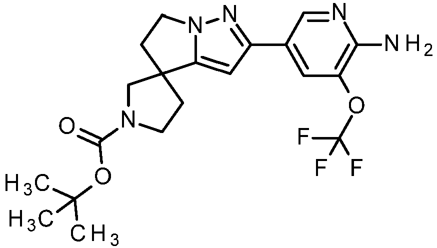

Intermediate 236 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (890 mg, 2.16 mmol) and [6-amino-5-(trifluoromethoxy)pyridin-3-yl]boronic acid (1.20 g, 60 % purity, 3.24 mmol).
LC-MS (method 1): Rt = 1.21 min; MS (ESlpos): m/z = 440 [M+H]⁺
1H-NMR (400 MHz, DMSO-d6) δ [ppm]: 1.066 (16.00), 1.154 (0.40), 1.173 (0.82), 1.190 (0.43), 1.395 (0.82), 1.432 (0.65), 1.988 (1.55), 2.518 (0.22), 2.523 (0.18), 3.408 (0.32), 3.937 (2.91), 4.017 (0.36), 4.035 (0.35), 6.495 (0.28), 8.330 (0.26), 8.334 (0.25).

### Intermediate 237

### (rac)-tert-butyl-2'-[6-amino-5-(difluoromethoxy)pyridin-3-yl]-5',6'-dihydrospiro[pyrrolidine-3,4'-pyrrolo[1,2-b]pyrazole]-1-carboxylate

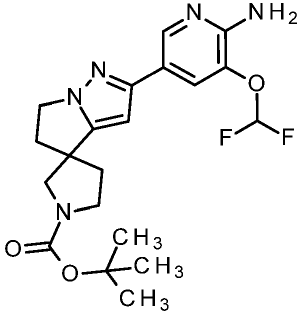

Intermediate 237 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (1.50 g, 3.64 mmol) and 3-(difluoromethoxy)-5-(4,4,5,5-tetramethyl-1,3,2-dioxaborolan-2-yl)pyridin-2-amine (2.50 g, 48 % purity, 4.18 mmol).
LC-MS (method 1): Rt = 1.12 min; MS (ESlpos): m/z = 422 [M+H]⁺
1H-NMR (400MHz, DMSO-d6): δ [ppm]= 1.41 (d, 9H), 1.95 - 2.12 (m, 3H), 2.53 - 2.59 (m, 2H), 3.37 - 3.46 (m, 3H), 3.47 - 3.60 (m, 1H), 4.12 - 4.24 (m, 2H), 6.16 (s, 2H), 6.39 (d, 1H), 7.18 (t, 1H), 7.62 (s, 1H), 8.20 (s, 1H).

### Intermediate 238

### tert-butyl-2'-{6-amino-5-[(1R)-1-(pyridin-2-yl)ethoxy]pyridin-3-yl}-5',6'-dihydrospiro[pyrrolidine-3,4'-pyrrolo[1,2-b]pyrazole]-1-carboxylate

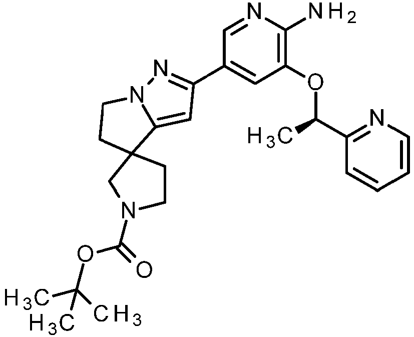

Intermediate 238 was prepared in analogy to Intermediate 235 using 3-[(1R)-1-(pyridin-2-yl)ethoxy]-5-(4,4,5,5-tetramethyl-1,3,2-dioxaborolan-2-yl)pyridin-2-amine (312 mg, 914 µmol) and (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (451 mg, 1.10 mmol).
LC-MS (method 1): Rt = 1.11 min; MS (ESlpos): m/z = 478 [M+H]⁺
¹H-NMR (400 MHz, DMSO-d6) δ [ppm]: 0.798 (0.54), 0.815 (0.54), 0.822 (0.57), 0.905 (0.72), 1.035 (5.63), 1.053 (12.08), 1.066 (13.92), 1.070 (5.69), 1.388 (13.53), 1.392 (13.86), 1.430 (16.00), 1.589 (0.99), 1.609 (9.82), 1.625 (9.97), 2.016 (1.42), 2.322 (1.11), 2.327 (1.66), 2.332 (1.24), 2.518 (7.50), 2.523 (5.03), 2.660 (0.54), 2.665 (1.21), 2.669 (1.72), 2.673 (1.21), 3.374 (5.15), 3.380 (4.61), 3.405 (1.60), 3.418 (1.30), 3.422 (2.47), 3.435 (2.32), 3.440 (2.23), 3.452 (2.20), 3.457 (0.81), 3.463 (0.72), 3.469 (0.96), 3.481 (1.21), 3.497 (0.84), 3.508 (0.75), 3.525 (0.45), 3.938 (1.96), 4.100 (1.18), 4.109 (1.48), 4.119 (2.20), 4.125 (2.26), 4.135 (1.48), 4.143 (1.33), 4.343 (1.21), 4.355 (2.38), 4.368 (1.15), 5.507 (1.21), 5.522 (1.24), 5.903 (3.59), 6.224 (2.02), 6.236 (1.72), 6.242 (1.48), 7.208 (2.86), 7.275 (1.18), 7.278 (1.02), 7.290 (1.69), 7.305 (1.36), 7.493 (2.23), 7.511 (2.56), 7.767 (1.15), 7.787 (2.05), 7.806 (0.93), 7.864 (3.95), 7.868 (4.01), 8.539 (1.81), 8.546 (1.84), 8.551 (1.75).

### Intermediate 239

### tert-butyl-2'-{6-amino-5-[(1S)-1-(pyridin-2-yl)ethoxy]pyridin-3-yl}-5',6'-dihydrospiro[pyrrolidine-3,4'-pyrrolo[1,2-b]pyrazole]-1-carboxylate

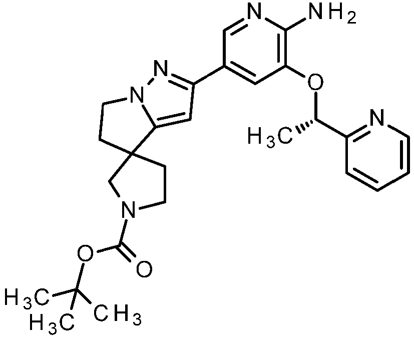

Intermediate 239 was prepared in analogy to Intermediate 235 using 3-[(1S)-1-(pyridin-2-yl)ethoxy]-5-(4,4,5,5-tetramethyl-1,3,2-dioxaborolan-2-yl)pyridin-2-amine (360 mg, 1.06 mmol) and (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (521 mg, 1.27 mmol).
LC-MS (method 1): Rt = 1.11 min; MS (ESlpos): m/z = 478 [M+H]⁺
¹H-NMR (400 MHz, DMSO-d6) δ [ppm]: 1.035 (7.56), 1.053 (16.00), 1.066 (1.32), 1.070 (7.02), 1.388 (10.77), 1.392 (11.11), 1.430 (12.71), 1.594 (1.78), 1.611 (8.85), 1.627 (7.76), 2.005 (1.09), 2.015 (1.14), 2.322 (0.58), 2.327 (0.86), 2.332 (0.63), 2.518 (4.60), 2.523 (3.03), 2.665 (0.61), 2.669 (0.89), 2.673 (0.65), 3.375 (4.17), 3.381 (3.77), 3.405 (1.34), 3.417 (1.05), 3.423 (1.80), 3.435 (1.69), 3.440 (1.58), 3.452 (1.51), 3.457 (0.74), 3.463 (0.69), 3.469 (0.74), 3.480 (0.98), 3.496 (0.71), 3.508 (0.61), 4.102 (0.97), 4.110 (1.18), 4.121 (1.72), 4.126 (1.83), 4.136 (1.18), 4.144 (1.09), 4.343 (0.66), 4.356 (1.26), 4.369 (0.66), 5.514 (1.00), 5.530 (1.03), 5.955 (1.77), 6.228 (1.63), 6.242 (1.38), 6.247 (1.20), 7.218 (2.31), 7.276 (0.91), 7.278 (0.92), 7.290 (1.49), 7.297 (1.05), 7.306 (1.05), 7.309 (0.97), 7.492 (1.75), 7.494 (1.75), 7.512 (2.04), 7.514 (2.00), 7.766 (1.03), 7.786 (1.81), 7.805 (0.91), 7.863 (3.32), 7.867 (3.37), 8.541 (1.54), 8.546 (1.61), 8.551 (1.46).

### Intermediate 240

### tert-butyl-2'-{6-amino-5-[(1S)-1-(pyridin-3-yl)ethoxy]pyridin-3-yl}-5',6'-dihydrospiro[pyrrolidine-3,4'-pyrrolo[1,2-b]pyrazole]-1-carboxylate

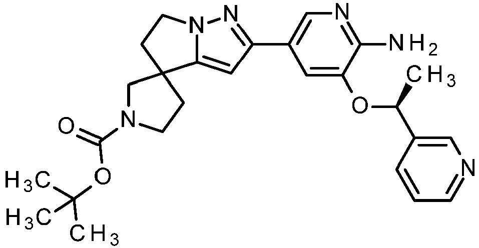

Intermediate 240 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (540 mg, 1.31 mmol) and 3-[(1S)-1-(pyridin-3-yl)ethoxy]-5-(4,4,5,5-tetramethyl-1,3,2-dioxaborolan-2-yl)pyridin-2-amine (1.60 g, 31 % purity, 1.44 mmol).
¹H-NMR (400MHz, DMSO-d6): δ [ppm]= 1.37 - 1.46 (m, 9H), 1.60 (d, 3H), 1.95 - 2.09 (m, 2H), 3.36 - 3.45 (m, 3H), 3.47 - 3.55 (m, 1H), 4.08 - 4.19 (m, 2H), 5.70 (q, 1H), 5.90 (s, 2H), 6.28 (dd, 1H), 7.32 (s, 1H), 7.35 - 7.40 (m, 1H), 7.87 (d, 1H), 7.89 - 7.94 (m, 1H), 8.47 (dd, 1H), 8.70 (s, 1H).
LC-MS (method 1): Rt = 1.08 min; MS (ESlpos): m/z = 478 [M+H]⁺

### Intermediate 241

### (rac)-tert-butyl-2'-{6-amino-5-[(2-fluorophenyl)methoxy]pyridin-3-yl}-5',6'-dihydrospiro[pyrrolidine-3,4'-pyrrolo[1,2-b]pyrazole]-1-carboxylate

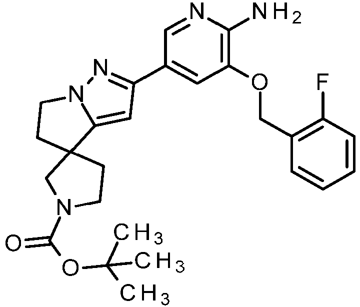

Intermediate 241 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (60.0 mg, 146 µmol) and 3-[(2-fluorophenyl)methoxy]-5-(4,4,5,5-tetramethyl-1,3,2-dioxaborolan-2-yl)pyridin-2-amine (122 mg, 47 % purity, 168 µmol).
LC-MS (method 1): Rt = 1.25 min; MS (ESlpos): m/z = 480 [M+H]⁺
1H-NMR (400MHz, DMSO-d6): δ [ppm]= 1.42 (d, 9H), 1.96 - 2.13 (m, 2H), 3.37 - 3.46 (m, 3H), 3.53 (dt, 1H), 4.17 (td, 2H), 5.21 (s, 2H), 5.77 (s, 2H), 6.37 (d, 1H), 7.21 - 7.31 (m, 2H), 7.38 - 7.46 (m, 1H), 7.48 (s, 1H), 7.67 (td, 1H), 7.96 (d, 1H).

### Intermediate 242

### (rac)-tert-butyl-2'-{6-amino-5-[(3-fluorophenyl)methoxy]pyridin-3-yl}-5',6'-dihydrospiro[pyrrolidine-3,4'-pyrrolo[1,2-b]pyrazole]-1-carboxylate

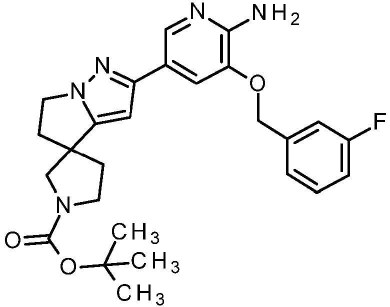

Intermediate 242 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (415 mg, 1.01 mmol) and 3-[(3-fluorophenyl)methoxy]-5-(4,4,5,5-tetramethyl-1,3,2-dioxaborolan-2-yl)pyridin-2-amine (938 mg, 43 % purity, 1.16 mmol).
LC-MS (method 1): Rt = 1.24 min; MS (ESlpos): m/z = 480 [M+H]⁺
1H-NMR (400 MHz, DMSO-d6) δ [ppm]: 1.066 (1.99), 1.154 (4.82), 1.172 (9.61), 1.190 (4.87), 1.398 (10.46), 1.435 (8.48), 1.988 (16.00), 2.039 (0.88), 2.047 (0.89), 2.068 (0.62), 2.327 (0.44), 2.669 (0.47), 3.369 (0.40), 3.410 (3.92), 3.426 (0.89), 3.509 (0.73), 3.524 (0.50), 3.536 (0.47), 3.999 (1.24), 4.017 (3.69), 4.035 (3.62), 4.053 (1.22), 4.138 (0.69), 4.147 (0.84), 4.156 (1.31), 4.163 (1.38), 4.173 (0.95), 4.181 (0.82), 5.204 (4.87), 5.251 (0.96), 5.808 (0.59), 5.876 (2.95), 6.341 (1.07), 6.364 (1.42), 7.132 (0.47), 7.153 (0.98), 7.169 (0.52), 7.173 (0.58), 7.350 (1.04), 7.369 (1.73), 7.410 (1.46), 7.426 (3.52), 7.430 (3.89), 7.445 (1.44), 7.450 (1.01), 7.466 (0.59), 7.773 (0.46), 7.778 (0.49), 7.939 (2.34), 7.943 (2.52).

### Intermediate 243

### (rac)-tert-butyl-2'-{6-amino-5-[(2,6-dichlorophenyl)methoxy]pyridin-3-yl}-5',6'-dihydrospiro[pyrrolidine-3,4'-pyrrolo[1,2-b]pyrazole]-1-carboxylate

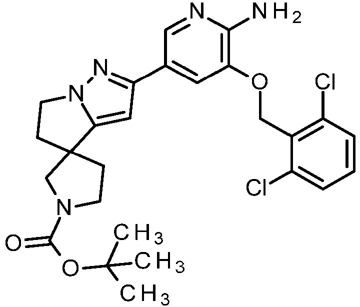

Intermediate 243 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (404 mg, 981 µmol) and 3-[(2,6-dichlorophenyl)methoxy]-5-(4,4,5,5-tetramethyl-1,3,2-dioxaborolan-2-yl)pyridin-2-amine (1.33 g, 33 % purity, 1.13 mmol).
LC-MS (method 1): Rt = 1.34 min; MS (ESlpos): m/z = 530 [M+H]⁺

### Intermediate 244

### (rac)-tert-butyl-2'-{6-amino-5-[(2-chlorophenyl)methoxy]pyridin-3-yl}-5',6'-dihydrospiro[pyrrolidine-3,4'-pyrrolo[1,2-b]pyrazole]-1-carboxylate

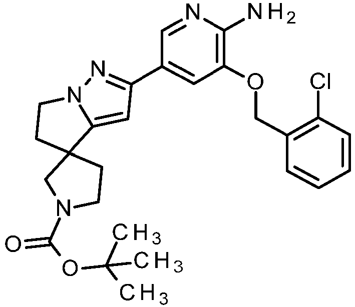

Intermediate 244 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (307 mg, 747 µmol) and 3-[(2-chlorophenyl)methoxy]-5-(4,4,5,5-tetramethyl-1,3,2-dioxaborolan-2-yl)pyridin-2-amine (632 mg, 49 % purity, 859 µmol).
LC-MS (method 1): Rt = 1.29 min; MS (ESlpos): m/z = 497 [M+H]⁺

### Intermediate 245

### (rac)-tert-butyl-2'-{6-amino-5-[(pyridin-2-yl)methoxy]pyridin-3-yl}-5',6'-dihydrospiro[pyrrolidine-3,4'-pyrrolo[1,2-b]pyrazole]-1-carboxylate

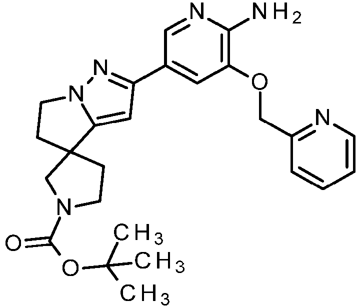

Intermediate 245 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (559 mg, 1.36 mmol) and 3-[(pyridin-2-yl)methoxy]-5-(4,4,5,5-tetramethyl-1,3,2-dioxaborolan-2-yl)pyridin-2-amine (950 mg, 54 % purity, 1.56 mmol).
LC-MS (method 1): Rt = 1.07 min; MS (ESlpos): m/z = 463 [M+H]⁺

### Intermediate 246

### (rac)-tert-butyl-2'-{6-amino-5-[(2-chloro-5-fluorophenyl)methoxy]pyridin-3-yl}-5',6'-dihydrospiro[pyrrolidine-3,4'-pyrrolo[1,2-b]pyrazole]-1-carboxylate

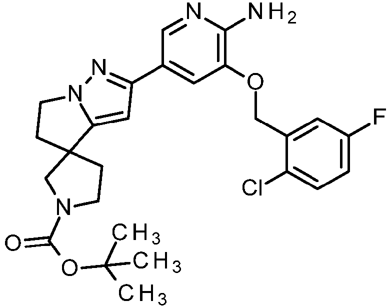

Intermediate 246 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (515 mg, 1.25 mmol) and 3-[(2-chloro-5-fluorophenyl)methoxy]-5-(4,4,5,5-tetramethyl-1,3,2-dioxaborolan-2-yl)pyridin-2-amine (940 mg, 58 % purity, 1.44 mmol).
LC-MS (method 1): Rt = 1.30 min; MS (ESlpos): m/z = 514 [M+H]⁺

### Intermediate 247

### tert-butyl-2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazole]-1-carboxylate

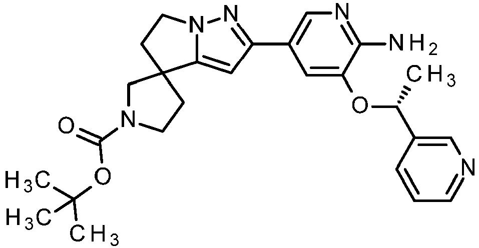

Intermediate 247 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (581 mg, 1.41 mmol) and 3-[(1R)-1-(pyridin-3-yl)ethoxy]-5-(4,4,5,5-tetramethyl-1,3,2-dioxaborolan-2-yl)pyridin-2-amine (1.00 g, 53 % purity, 1.55 mmol).
1H-NMR (400MHz, DMSO-d6): δ [ppm]= 1.37 - 1.45 (m, 9H), 1.60 (d, 3H), 1.95 - 2.09 (m, 2H), 3.36 - 3.45 (m, 3H), 3.46 - 3.55 (m, 1H), 4.07 - 4.19 (m, 2H), 5.70 (q, 1H), 5.90 (s, 2H), 6.28 (dd, 1H), 7.32 (s, 1H), 7.34 - 7.40 (m, 1H), 7.87 (d, 1H), 7.91 (br d, 1H), 8.46 and 8.47 (2d, 1H), 8.70 (s, 1H).
LC-MS (method 1): Rt = 1.05 min; MS (ESlpos): m/z = 477 [M+H]⁺

### Intermediate 248

### (rac)-tert-butyl-2'-(2-amino-1'-methyl-1',2',3',6'-tetrahydro[3,4'-bipyridin]-5-yl)-5',6'-dihydrospiro[pyrrolidine-3,4'-pyrrolo[1,2-b]pyrazole]-1-carboxylate

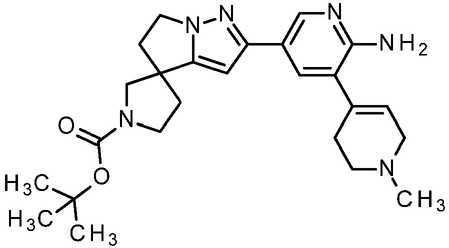

Intermediate 248 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (159 mg, 386 µmol) and 1'-methyl-5-(4,4,5,5-tetramethyl-1,3,2-dioxaborolan-2-yl)-1',2',3',6'-tetrahydro[3,4'-bipyridin]-2-amine (434 mg, 42 % purity, 578 µmol).
LC-MS (method 1): Rt = 1.09 min; MS (ESlpos): m/z = 452 [M+H]⁺
¹H-NMR (400 MHz, CHLOROFORM-d) δ [ppm]: 1.250 (16.00), 1.329 (0.65), 1.474 (1.26), 1.509 (0.86), 2.427 (1.53), 5.309 (0.76).

### Intermediate 249

### tert-butyl-2'-{6-amino-5-[(1R)-1-(pyridin-4-yl)ethoxy]pyridin-3-yl}-5',6'-dihydrospiro[pyrrolidine-3,4'-pyrrolo[1,2-b]pyrazole]-1-carboxylate

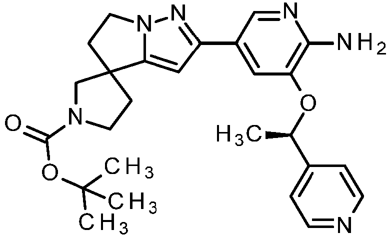

Intermediate 249 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (180 mg, 437 µmol) and {6-amino-5-[(1R)-1-(pyridin-4-yl)ethoxy]pyridin-3-yl}boronic acid (170 mg, 656 µmol).
LC-MS (method 1): Rt = 1.08 min; MS (ESlpos): m/z = 477 [M+H]⁺

### Intermediate 250

### tert-butyl-2'-{6-amino-5-[(1S)-1-phenylethoxy]pyridin-3-yl}-5',6'-dihydrospiro[pyrrolidine-3,4'-pyrrolo[1,2-b]pyrazole]-1-carboxylate

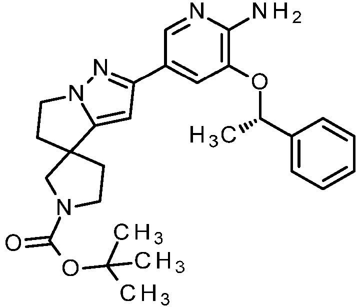

Intermediate 250 was prepared in analogy to Intermediate 235 using 3-[(1S)-1-phenylethoxy]-5-(4,4,5,5-tetramethyl-1,3,2-dioxaborolan-2-yl)pyridin-2-amine (200 mg, 60 % purity, 353 µmol) and (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (160 mg, 388 µmol).
LC-MS (method 1): Rt = 1.26 min; MS (ESlpos): m/z = 476 [M+H]⁺
1H-NMR (400 MHz, DMSO-d6) δ [ppm]: 1.066 (16.00), 1.173 (0.21), 1.389 (2.77), 1.398 (2.89), 1.433 (3.22), 1.563 (2.22), 1.579 (2.19), 1.988 (0.54), 2.017 (0.38), 2.323 (0.26), 2.327 (0.36), 2.332 (0.28), 2.523 (1.32), 2.665 (0.26), 2.669 (0.38), 2.673 (0.27), 3.379 (1.37), 3.405 (0.38), 3.468 (0.17), 3.486 (0.32), 3.500 (0.22), 3.939 (2.84), 4.090 (0.36), 4.102 (0.32), 4.111 (0.39), 4.121 (0.58), 4.127 (0.58), 4.137 (0.38), 4.144 (0.34), 5.568 (0.30), 5.583 (0.30), 5.831 (1.44), 6.222 (0.40), 6.237 (0.39), 6.245 (0.36), 7.230 (0.90), 7.241 (0.51), 7.245 (0.47), 7.259 (0.36), 7.314 (0.47), 7.319 (0.53), 7.333 (0.91), 7.338 (0.89), 7.352 (0.47), 7.357 (0.43), 7.452 (1.21), 7.471 (0.88), 7.837 (1.22), 7.842 (1.22).

### Intermediate 251

### tert-butyl-2'-{6-amino-5-[(1R)-1-phenylethoxy]pyridin-3-yl}-5',6'-dihydrospiro[pyrrolidine-3,4'-pyrrolo[1,2-b]pyrazole]-1-carboxylate

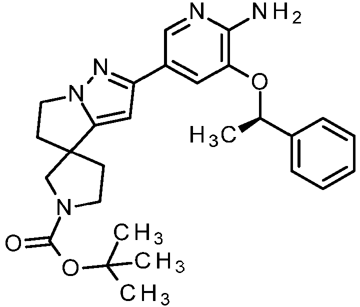

Intermediate 251 was prepared in analogy to Intermediate 235 using 3-[(1R)-1-phenylethoxy]-5-(4,4,5,5-tetramethyl-1,3,2-dioxaborolan-2-yl)pyridin-2-amine (270 mg, 75 % purity, 595 µmol) and (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (269 mg, 655 µmol).
LC-MS (method 1): Rt = 1.26 min; MS (ESlpos): m/z = 476 [M+H]⁺
¹H-NMR (400 MHz, DMSO-d6) δ [ppm]: 1.066 (16.00), 1.155 (0.95), 1.173 (2.06), 1.190 (1.04), 1.390 (2.91), 1.398 (3.03), 1.433 (3.31), 1.563 (2.54), 1.579 (2.49), 1.988 (3.70), 2.518 (1.08), 2.523 (0.83), 3.380 (1.36), 3.938 (2.63), 4.017 (0.73), 4.036 (0.70), 4.121 (0.59), 4.127 (0.59), 5.759 (5.03), 5.831 (1.48), 6.223 (0.41), 6.237 (0.42), 7.231 (0.92), 7.240 (0.58), 7.245 (0.51), 7.259 (0.42), 7.314 (0.58), 7.318 (0.60), 7.333 (1.06), 7.338 (0.98), 7.351 (0.53), 7.357 (0.47), 7.452 (1.30), 7.471 (0.92), 7.838 (1.50), 7.842 (1.43).

### Intermediate 252

### tert-butyl-2'-(6-amino-5-{[1-(5-chloro-3-fluoropyridin-2-yl)ethyl]oxy}pyridin-3-yl)-5',6'-dihydrospiro[pyrrolidine-3,4'-pyrrolo[1,2-b]pyrazole]-1-carboxylate (mixture of isomers)

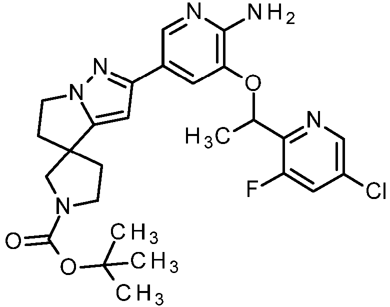

Intermediate 252 was prepared in analogy to Intermediate 235 using (rac)-tert-butyl-2'-[(trifluoromethanesulfonyl)oxy]-5',6'-dihydrospiro[pyrrolidine-3,4'-pyrrolo[1,2-b]pyrazole]-1-carboxylate (310 mg, 753 µmol) and (rac)-3-{[-1-(5-chloro-3-fluoropyridin-2-yl)ethyl]oxy}-5-(4,4,5,5-tetramethyl-1,3,2-dioxaborolan-2-yl)pyridin-2-amine (620 mg, 55 % purity, 866 µmol).
LC-MS (method 2): Rt = 1.26 min; MS (ESlpos): m/z = 529 [M+H]⁺

### Intermediate 253

### (rac)-tert-butyl-2'-{6-amino-5-[(2-fluorophenyl)methoxy]pyridin-3-yl}-5',6'-dihydrospiro[pyrrolidine-3,4'-pyrrolo[1,2-b]pyrazole]-1-carboxylate

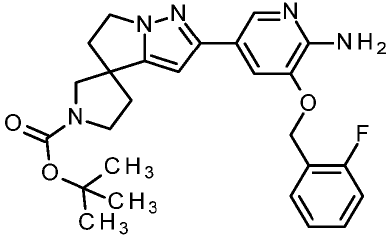

(rac)-tert-butyl-2'-[(trifluoromethanesulfonyl)oxy]-5',6'-dihydrospiro[pyrrolidine-3,4'-pyrrolo[1,2-b]pyrazole]-1-carboxylate (86.7 mg, 211 µmol), 3-[(2-fluorophenyl)methoxy]-5-(4,4,5,5-tetramethyl-1,3,2-dioxaborolan-2-yl)pyridin-2-amine (139 mg, 60 % purity, 242 µmol), K3PO4 (1.3 mL, 0.50 M, 630 µmol), dicyclohexyl[2',4',6'-tri(propan-2-yl)[1,1'-biphenyl]-2-yl]phosphane (10.0 mg, 21.1 µmol) and XPhos Pd G2 (8.29 mg, 10.5 µmol) were stirred in degassed 1,4-dioxane (3.5 mL) for 1 h at 100°C. The mixture was diluted with water and extracted 3x with DCM. The combined organic layers were dried and evaporated.

The residue was purified by flash chromatography to give 36.0 mg (36 % yield) of the title compound.
LC-MS (method 1): Rt = 1.23 min; MS (ESlpos): m/z = 480 [M+H]⁺

### Intermediate 254

### (rac)-tert-butyl-2'-[6-amino-5-(benzyloxy)pyridin-3-yl]-5',6'-dihydrospiro[pyrrolidine-3,4'-pyrrolo[1,2-b]pyrazole]-1-carboxylate

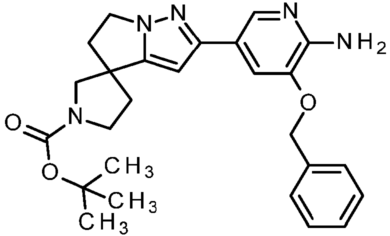

(*rac*)-tert-butyl-2'-[(trifluoromethanesulfonyl)oxy]-5',6'-dihydrospiro[pyrrolidine-3,4'-pyrrolo[1,2-b]pyrazole]-1-carboxylate (80.1 mg, 195 µmol), 3-(benzyloxy)-5-(4,4,5,5-tetramethyl-1,3,2-dioxaborolan-2-yl)pyridin-2-amine (116 mg, 63 % purity, 224 µmol), K3PO4 (1.2 mL, 0.50 M, 580 µmol), dicyclohexyl[2',4',6'-tri(propan-2-yl)[1,1'-biphenyl]-2-yl]phosphane (9.29 mg, 19.5 µmol) and XPhos Pd G2 (7.66 mg, 9.74 µmol) were stirred in degassed 1,4-dioxane (3.2 mL) for 1 h at 100°C. The mixture was diluted with water and extracted 3x with DCM. The combined organic layers were dried and evaporated. The residue was purified by flash chromatography to give 78.0 mg (87 % yield) of the title compound.
LC-MS (method 1): Rt = 1.23 min; MS (ESlpos): m/z = 462 [M+H]⁺
1H-NMR (400MHz, DMSO-d6): δ [ppm]= 1.42 (d, 9H), 2.00 - 2.10 (m, 2H), 2.51 - 2.59 (m, 2H), 3.36 - 3.46 (m, 3H), 3.48 - 3.57 (m, 1H), 4.16 (td, 2H), 5.18 (s, 2H), 5.79 (s, 2H), 6.35 (d, 1H), 7.28 - 7.35 (m, 1H), 7.36 - 7.45 (m, 3H), 7.48 - 7.55 (m, 2H), 7.93 (d, 1H).

### Intermediate 255

### tert-butyl (3S)-2'-[6-amino-5-(pyridazin-3-ylmethoxy)pyridin-3-yl]-5',6'-dihydro-1H-spiro[pyrrolidine-3,4'-pyrrolo[1,2-b]pyrazole]-1-carboxylate

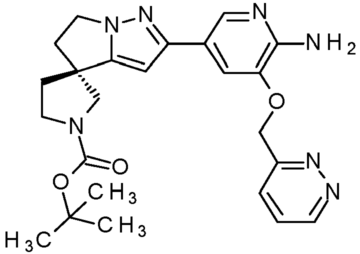

Intermediate 255 was prepared in analogy to Intermediate 235 using tert-butyl (3S)-2'-[(trifluoromethanesulfonyl)oxy]-5',6'-dihydrospiro[pyrrolidine-3,4'-pyrrolo[1,2-b]pyrazole]-1-carboxylate (100 mg, 244 µmol) and 3-[(pyridazin-3-yl)methoxy]-5-(4,4,5,5-tetramethyl-1,3,2-dioxaborolan-2-yl)pyridin-2-amine (206 mg, 51 % purity, 317 µmol).
[α]²⁰_{D} : -39.2° (c = 1.00, DMSO)
LC-MS (method 1): Rt = 0.96 min; MS (ESlpos): m/z = 464 [M+H]⁺
¹H NMR (400 MHz, DMSO-d6, 22°C): δ = 9.22 (dd, 1H), 8.01 (dd, 1H), 7.97 (d, 1H), 7.74-7.80 (m, 1H), 7.47-7.55 (m, 1H), 6.38 (d, 1H), 5.95 (s, 2H), 5.76 (s, 1H), 5.47 (s, 2H), 4.17 (td, 2H), 3.37-3.55 (m, 4H), 2.52-2.57 (m, 2H), 1.98-2.11 (m, 2H), 1.37-1.46 (m, 9H), 1.03-1.09 (m, 1H)

### Intermediate 256

### tert-butyl (3R)-2'-[6-amino-5-(pyridazin-3-ylmethoxy)pyridin-3-yl]-5',6'-dihydro-1H-spiro[pyrrolidine-3,4'-pyrrolo[1,2-b]pyrazole]-1-carboxylate

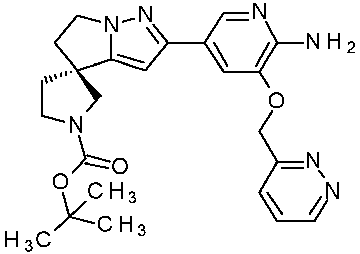

Intermediate 256 was prepared in analogy to Intermediate 235 using tert-butyl-(3R)-2'-[(trifluoromethanesulfonyl)oxy]-5',6'-dihydrospiro[pyrrolidine-3,4'-pyrrolo[1,2-b]pyrazole]-1-carboxylate (100 mg, 244 µmol) and 3-[(pyridazin-3-yl)methoxy]-5-(4,4,5,5-tetramethyl-1,3,2-dioxaborolan-2-yl)pyridin-2-amine (206 mg, 51 % purity, 317 µmol).
[α]²⁰_{D} : +37.1 ° (c = 1.00, DMSO)
LC-MS (method 1): Rt = 0.95 min; MS (ESlpos): m/z = 464 [M+H]⁺
¹H NMR (400 MHz, DMSO-d6, 22°C): δ = 9.21 (d, J = 4.8 Hz, 1H), 8.01 (dd, J = 8.5, 1.6 Hz, 1H), 7.97 (d, J = 1.8 Hz, 1H), 7.73-7.81 (m, 1H), 7.48-7.55 (m, 1H), 6.38 (d, J = 9.9 Hz, 1H), 5.96 (s, 2H), 5.76 (s, 1H), 5.47 (s, 2H), 4.11-4.22 (m, 2H), 3.46-3.57 (m, 1H), 3.37-3.46 (m, 3H), 2.52-2.56 (m, 2H), 1.99-2.11 (m, 2H), 1.37-1.46 ppm (m, 8H)

### Intermediate 257

### tert-butyl (3S)-2'-{6-amino-5-[1-(pyrimidin-5-yl)ethoxy]pyridin-3-yl}-5',6'-dihydro-1H-spiro[pyrrolidine-3,4'-pyrrolo[1,2-b]pyrazole]-1-carboxylate (isomer 1)

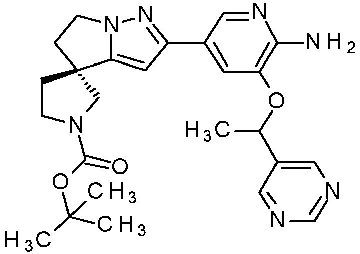

Intermediate 257 was prepared in analogy to Intermediate 235 using tert-butyl-(3S)-2'-[(trifluoromethanesulfonyl)oxy]-5',6'-dihydrospiro[pyrrolidine-3,4'-pyrrolo[1,2-b]pyrazole]-1-carboxylate (103 mg, 251 µmol, stereoisomer 1) and 3-{[1-(pyrimidin-5-yl)ethyl]oxy}-5-(4,4,5,5-tetramethyl-1,3,2-dioxaborolan-2-yl)pyridin-2-amine (186 mg, 60 % purity, 326 µmol, stereoisomer 1).
[α]²⁰_{D} : -118.6° (c = 1.00, DMSO)
LC-MS (method 1): Rt = 0.99 min; MS (ESlpos): m/z = 478 [M+H]⁺
¹H NMR (400 MHz, DMSO-d6, 22°C): δ = 9.11 (s, 1H), 8.98 (s, 2H), 7.91 (d, J = 1.8 Hz, 1H), 7.41 (s, 1H), 6.32 (d, J = 9.4 Hz, 1H), 5.96 (s, 2H), 5.78 (q, J = 6.4 Hz, 1H), 4.09-4.21 (m, 2H), 3.35-3.54 (m, 4H), 1.98-2.11 (m, 2H), 1.62 (d, J = 6.6 Hz, 3H), 1.36-1.46 ppm (m, 9H)

### Intermediate 258

### tert-butyl (3S)-2'-{6-amino-5-[1-(pyrimidin-5-yl)ethoxy]pyridin-3-yl}-5',6'-dihydro-1H-spiro[pyrrolidine-3,4'-pyrrolo[1,2-b]pyrazole]-1-carboxylate (isomer 1)

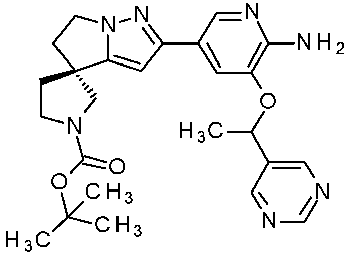

Intermediate 258 was prepared in analogy to Intermediate 235 using tert-butyl (3S)-2'-[(trifluoromethanesulfonyl)oxy]-5',6'-dihydrospiro[pyrrolidine-3,4'-pyrrolo[1,2-b]pyrazole]-1-carboxylate (96.5 mg, 235 µmol, stereoisomer 1) and 3-{[1-(pyrimidin-5-yl)ethyl]oxy}-5-(4,4,5,5-tetramethyl-1,3,2-dioxaborolan-2-yl)pyridin-2-amine (174 mg, 60 % purity, 305 µmol, stereoisomer 2).
[α]²⁰_{D} : +37.6° (c = 1.00, DMSO)
LC-MS (method 1): Rt = 1.00 min; MS (ESlpos): m/z = 478 [M+H]⁺
¹H NMR (400 MHz, DMSO-d6, 22°C): δ = 9.11 (s, 1H), 8.98 (s, 2H), 7.90 (s, 1H), 7.41 (s, 1H), 6.31 (d, J = 9.6 Hz, 1H), 5.96 (s, 2H), 5.79 (q, J = 6.2 Hz, 1H), 4.15 (td, J = 6.9, 2.2 Hz, 2H), 3.45-3.56 (m, 1H), 3.36-3.44 (m, 3H), 2.52-2.56 (m, 2H), 1.98-2.10 (m, 2H), 1.62 (d, J = 6.3 Hz, 3H), 1.37-1.46 ppm (m, 9H)

### Intermediate 259

### tert-butyl 2'-[6-amino-5-(trifluoromethyl)pyridin-3-yl]-5',6'-dihydrospiro[azetidine-3,4'-pyrrolo[1,2-b]pyrazole]-1-carboxylate

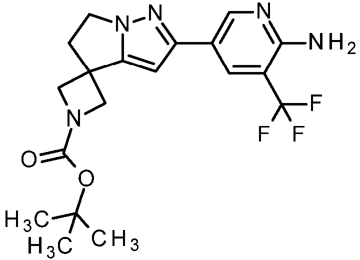

tert-butyl 2'-[(trifluoromethanesulfonyl)oxy]-5',6'-dihydrospiro[azetidine-3,4'-pyrrolo[1,2-b]pyrazole]-1-carboxylate (2.00 g, 5.03 mmol) was solubilised in 1,4-dioxane (42 mL) under nitrogen, 5-(4,4,5,5-tetramethyl-1,3,2-dioxaborolan-2-yl)-3-(trifluoromethyl)pyridin-2-amine (2.17 g, 7.55 mmol), K3PO4 (30 mL, 0.50 M, 15 mmol) and XPhos Pd G2 (198 mg, 252 µmol) were added and the mixture was stirred overnight at 100°C. It was diluted with ethyl acetate, washed with brine, dried with a silicone filter and evaporated. The residue was purified by flash chromatography to give 2.10 g of the title compound.
LC-MS (method 1): Rt = 1.19 min; MS (ESlpos): m/z = 410 [M+H]⁺
1H-NMR (400MHz, DMSO-d6): δ [ppm]= 1.41 (s, 9H), 2.85 (t, 2H), 3.99 - 4.15 (m, 6H), 6.55 (s, 2H), 6.76 (s, 1H), 8.03 (d, 1H), 8.61 (d, 1H).

### Intermediate 260

### tert-butyl 2'-(6-amino-5-cyanopyridin-3-yl)-5',6'-dihydrospiro[azetidine-3,4'-pyrrolo[1,2-b]pyrazole]-1-carboxylate

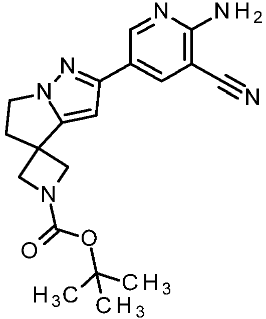

Intermediate 260 was prepared in analogy to Intermediate 259 using tert-butyl 2'-[(trifluoromethanesulfonyl)oxy]-5',6'-dihydrospiro[azetidine-3,4'-pyrrolo[1,2-b]pyrazole]-1-carboxylate (2.05 g, 5.17 mmol) and 2-amino-5-(4,4,5,5-tetramethyl-1,3,2-dioxaborolan-2-yl)pyridine-3-carbonitrile (1.90 g, 7.75 mmol).
LC-MS (method 1): Rt = 1.06 min; MS (ESlpos): m/z = 367 [M+H]⁺
¹H-NMR (400 MHz, DMSO-d6) δ [ppm]: 1.052 (0.57), 1.065 (2.31), 1.156 (0.63), 1.172 (0.51), 1.410 (16.00), 1.988 (0.83), 2.518 (2.02), 2.523 (1.39), 2.828 (0.58), 2.846 (0.95), 2.863 (0.64), 3.566 (2.05), 3.940 (0.40), 4.017 (0.56), 4.024 (0.61), 4.035 (0.42), 4.072 (0.63), 4.092 (1.03), 4.110 (1.07), 4.127 (0.64), 5.760 (4.70), 6.711 (2.96), 7.002 (1.50), 8.148 (1.31), 8.154 (1.43), 8.628 (1.42), 8.634 (1.51).

### Intermediate 261 tert-butyl 2'-[6-amino-5-(difluoromethoxy)pyridin-3-yl]-5',6'-dihydrospiro[azetidine-3,4'-pyrrolo[1,2-b]pyrazole]-1-carboxylate

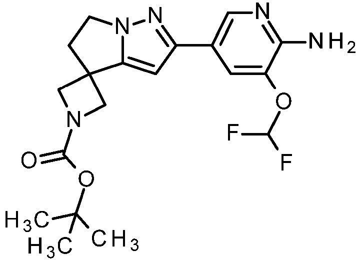

Intermediate 261 was prepared in analogy to Intermediate 259 using tert-butyl 2'-[(trifluoromethanesulfonyl)oxy]-5',6'-dihydrospiro[azetidine-3,4'-pyrrolo[1,2-b]pyrazole]-1-carboxylate (2.41 g, 6.06 mmol) and 3-(difluoromethoxy)-5-(4,4,5,5-tetramethyl-1,3,2-dioxaborolan-2-yl)pyridin-2-amine (2.60 g, 9.09 mmol).
LC-MS (method 1): Rt = 1.11 min; MS (ESlpos): m/z = 408 [M+H]⁺
1H-NMR (400 MHz, DMSO-d6) δ [ppm]: 1.066 (16.00), 1.155 (0.75), 1.173 (1.59), 1.190 (0.80), 1.409 (4.36), 1.988 (2.82), 2.518 (0.39), 2.523 (0.26), 2.562 (0.27), 2.843 (0.25), 2.860 (0.16), 2.888 (0.17), 3.937 (2.60), 4.000 (0.22), 4.017 (0.69), 4.035 (0.72), 4.053 (0.29), 4.065 (0.19), 4.085 (0.26), 4.103 (0.28), 4.120 (0.17), 6.165 (0.40), 6.656 (0.79), 6.996 (0.17), 7.181 (0.35), 7.634 (0.22), 7.636 (0.22), 8.228 (0.40), 8.232 (0.36).

### Intermediate 262

### tert-butyl 2'-[6-amino-5-(trifluoromethoxy)pyridin-3-yl]-5',6'-dihydrospiro[azetidine-3,4'-pyrrolo[1,2-b]pyrazole]-1-carboxylate

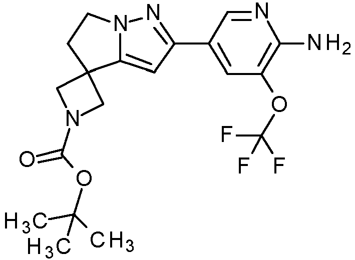

Intermediate 262 was prepared in analogy to Intermediate 259 using tert-butyl 2'-[(trifluoromethanesulfonyl)oxy]-5',6'-dihydrospiro[azetidine-3,4'-pyrrolo[1,2-b]pyrazole]-1-carboxylate (816 mg, 2.05 mmol) and [6-amino-5-(trifluoromethoxy)pyridin-3-yl]boronic acid (1.14 g, 60 % purity, 3.08 mmol).
LC-MS (method 1): Rt = 1.20 min; MS (ESlpos): m/z = 426 [M+H]⁺
¹H-NMR (400 MHz, DMSO-d6) δ [ppm]: 1.066 (16.00), 1.173 (0.54), 1.409 (2.71), 1.988 (1.03), 3.938 (2.70), 6.701 (0.52).

### Intermediate 263

### tert-butyl 2'-{6-amino-5-[(1S)-1-(pyridin-2-yl)ethoxy]pyridin-3-yl}-5',6'-dihydrospiro[azetidine-3,4'-pyrrolo[1,2-b]pyrazole]-1-carboxylate

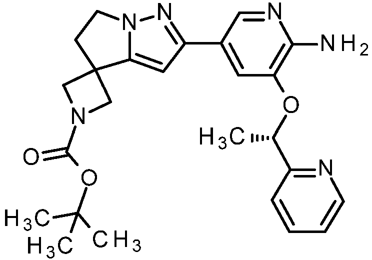

Intermediate 263 was prepared in analogy to Intermediate 259 using tert-butyl 2'-[(trifluoromethanesulfonyl)oxy]-5',6'-dihydrospiro[azetidine-3,4'-pyrrolo[1,2-b]pyrazole]-1-carboxylate (122 mg, 307 µmol) and 3-[(1S)-1-(pyridin-2-yl)ethoxy]-5-(4,4,5,5-tetramethyl-1,3,2-dioxaborolan-2-yl)pyridin-2-amine (105 mg, 307 µmol).
¹H-NMR (400MHz, DMSO-d6): δ [ppm]= 1.41 (s, 9H), 1.62 (br d, 3H), 2.81 (br t, 2H), 3.97 - 4.15 (m, 6H), 5.53 (q, 1H), 5.90 (s, 2H), 6.52 (s, 1H), 7.24 (s, 1H), 7.26 - 7.33 (m, 1H), 7.51 (br d, 1H), 7.79 (br t, 1H), 7.90 (s, 1H), 8.55 (br d, 1H).
LC-MS (method 1): Rt = 1.10 min; MS (ESlpos): m/z = 463 [M+H]⁺

### Intermediate 264

### tert-butyl 2'-{6-amino-5-[(1R)-1-(pyridin-2-yl)ethoxy]pyridin-3-yl}-5',6'-dihydrospiro[azetidine-3,4'-pyrrolo[1,2-b]pyrazole]-1-carboxylate

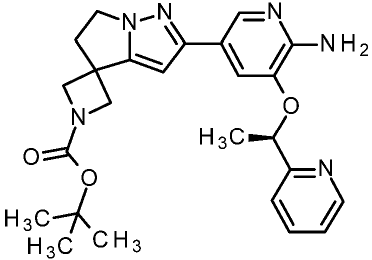

Intermediate 264 was prepared in analogy to Intermediate 259 using tert-butyl 2'-[(trifluoromethanesulfonyl)oxy]-5',6'-dihydrospiro[azetidine-3,4'-pyrrolo[1,2-b]pyrazole]-1-carboxylate (175 mg, 441 µmol) and 3-[(1R)-1-(pyridin-2-yl)ethoxy]-5-(4,4,5,5-tetramethyl-1,3,2-dioxaborolan-2-yl)pyridin-2-amine (150 mg, 441 µmol).
LC-MS (method 1): Rt = 1.11 min; MS (ESlpos): m/z = 463 [M+H]⁺

### Intermediate 265

### tert-butyl 2'-{6-amino-5-[(1R)-1-phenylethoxy]pyridin-3-yl}-5',6'-dihydrospiro[azetidine-3,4'-pyrrolo[1,2-b]pyrazole]-1-carboxylate

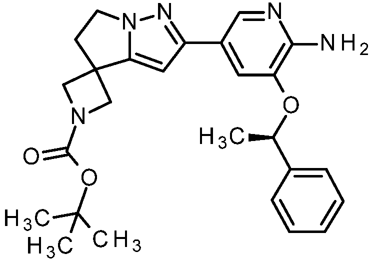

Intermediate 265 was prepared in analogy to Intermediate 259 using tert-butyl 2'-[(trifluoromethanesulfonyl)oxy]-5',6'-dihydrospiro[azetidine-3,4'-pyrrolo[1,2-b]pyrazole]-1-carboxylate (175 mg, 441 µmol) and 3-[(1R)-1-phenylethoxy]-5-(4,4,5,5-tetramethyl-1,3,2-dioxaborolan-2-yl)pyridin-2-amine (150 mg, 441 µmol).
LC-MS (method 1): Rt = 1.25 min; MS (ESlpos): m/z = 462 [M+H]⁺

### Intermediate 266 tert-butyl 2'-{6-amino-5-[(1S)-1-phenylethoxy]pyridin-3-yl}-5',6'-dihydrospiro[azetidine-3,4'-pyrrolo[1,2-b]pyrazole]-1-carboxylate

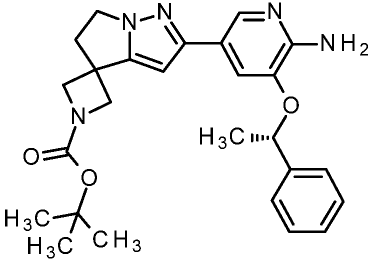

Intermediate 266 was prepared in analogy to Intermediate 259 using tert-butyl 2'-[(trifluoromethanesulfonyl)oxy]-5',6'-dihydrospiro[azetidine-3,4'-pyrrolo[1,2-b]pyrazole]-1-carboxylate (175 mg, 441 µmol) and 3-[(1S)-1-phenylethoxy]-5-(4,4,5,5-tetramethyl-1,3,2-dioxaborolan-2-yl)pyridin-2-amine (150 mg, 441 µmol).
LC-MS (method 1): Rt = 1.27 min; MS (ESlpos): m/z = 462 [M+H]⁺

### Intermediate 267

### (rac)-tert-butyl-2-(6-amino-5-cyanopyridin-3-yl)-6,7-dihydrospiro[pyrazolo[5,1-c][1,4]oxazine-4,3'-pyrrolidine]-1'-carboxylate

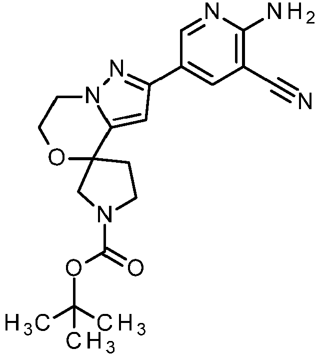

(Rac)-tert-butyl-2-bromo-6,7-dihydrospiro[pyrazolo[5,1-c][1,4]oxazine-4,3'-pyrrolidine]-1'-carboxylate (621 mg, 1.73 mmol), 2-amino-5-(4,4,5,5-tetramethyl-1,3,2-dioxaborolan-2-yl)pyridine-3-carbonitrile (850 mg, 3.47 mmol), XPhos Pd G2 (68.2 mg, 86.7 µmol) and potassium phosphate (10 mL, 0.50 M, 5.2 mmol) were stirred in degassed dioxane (25 mL) for 2h at 100°C. The reaction mixture was diluted with ethyl acetate and water. The phases were separated and the aqueous phase was extracted twice with ethyl acetate. The combined organic phases were dried over sodium sulfate and concentrated. The residue was purified by flash chromatography to afford 736 mg of the title compound.

¹H-NMR (400 MHz, DMSO-d6) δ [ppm]: 1.065 (4.11), 1.265 (0.50), 1.399 (7.96), 1.424 (6.94), 2.518 (0.73), 2.523 (0.49), 3.159 (16.00), 3.172 (15.27), 3.366 (0.44), 3.375 (0.41), 3.397 (0.49), 3.428 (0.42), 3.483 (0.44), 3.551 (0.65), 3.726 (0.52), 3.941 (0.69), 4.087 (1.54), 4.100 (4.02), 4.113 (6.91), 4.126 (1.93), 6.699 (0.95), 6.717 (1.09), 7.046 (2.34), 8.114 (1.17), 8.119 (1.15), 8.591 (2.11), 8.598 (2.12).

### Intermediate 268

### (rac)-tert-butyl-2-[6-amino-5-(trifluoromethoxy)pyridin-3-yl]-6,7-dihydrospiro[pyrazolo[5,1-c][1,4]oxazine-4,3'-pyrrolidine]-1'-carboxylate

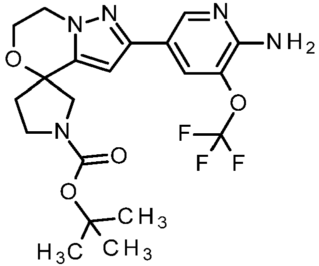

Intermediate 268 was prepared in analogy to Intermediate 235 using (rac)-tert-butyl-2-bromo-6,7-dihydrospiro[pyrazolo[5,1-c][1,4]oxazine-4,3'-pyrrolidine]-1'-carboxylate (1.13 g, 3.15 mmol) and [6-amino-5-(trifluoromethoxy)pyridin-3-yl]boronic acid (1.75 g, 60 % purity, 4.73 mmol).
LC-MS (method 1): Rt = 1.18 min; MS (ESlpos): m/z = 456 [M+H]⁺

### Intermediate 269

### (rac)-tert-butyl-2-[6-amino-5-(difluoromethoxy)pyridin-3-yl]-6,7-dihydrospiro[pyrazolo[5,1-c][1,4]oxazine-4,3'-pyrrolidine]-1'-carboxylate

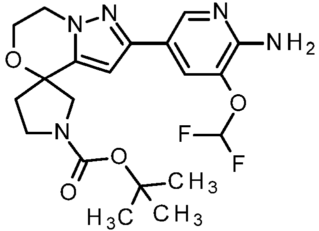

(rac)-tert-butyl-2-bromo-6,7-dihydrospiro[pyrazolo[5,1-c][1,4]oxazine-4,3'-pyrrolidine]-1'-carboxylate (1.05 g, 2.94 mmol) was solubilized in 1,4-dioxane (29 mL), [6-amino-5-(difluoromethoxy)pyridin-3-yl]boronic acid (1.50 g, 60 % purity, 4.41 mmol), K3PO4 (18 mL, 0.50 M, 8.8 mmol) and XPhos Pd G2 (347 mg, 441 µmol) were added and the mixture was stirred overnight at 100°C. It was diluted with water and extracted 2x with ethyl acetate. The combined organic layers were dried over a silicone filter and evaporated. The residue was purified by flash chromatography to give 1.39 g of the title compound.
LC-MS (method 1): Rt = 1.11 min; MS (ESlpos): m/z = 438 [M+H]⁺
¹H-NMR (400 MHz, DMSO-d6) δ [ppm]: 1.066 (16.00), 1.401 (3.24), 1.425 (2.79), 2.518 (1.46), 2.523 (1.02), 3.939 (2.63), 4.110 (1.50), 6.218 (0.87), 6.676 (0.43), 7.179 (0.72), 7.605 (0.48), 8.188 (0.56), 8.192 (0.54).

### Intermediate 270

### tert-butyl (3'R)-2-[6-amino-5-(trifluoromethoxy)pyridin-3-yl]-6,7-dihydrospiro[pyrazolo[5,1-c][1,4]oxazine-4,3'-pyrrolidine]-1'-carboxylate

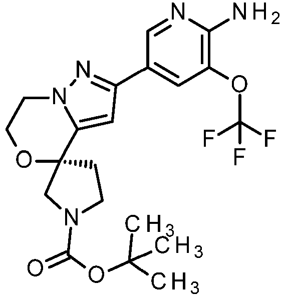

To a solution of 5-(4,4,5,5-tetramethyl-1,3,2-dioxaborolan-2-yl)-3-(trifluoromethoxy)pyridin-2-amine (357 mg, 1.17 mmol) in Dioxane (12 mL) under Argon, tert-butyl (3'R)-2-bromo-6,7-dihydrospiro[pyrazolo[5,1-c][1,4]oxazine-4,3'-pyrrolidine]-1'-carboxylate (442 mg, 1.23 mmol) and Potassium phosphate (7.0 ml, 0.50 M in water, 3.5 mmol) were added. The mixture was degassed and XPhos Pd G2 (46.2 mg, 58.7 µmol) was added and stirred at 100°C under Argon for 1h. The mixture was diluted with water and then extracted with Ethyl acetate 2x. The combined organics were washed with Brine, dried over Sodium sulfate, filtered and concentrated to give 602 mg of the title compound.
LC-MS (Method 1): Rt = 1.20 min; MS (ESlpos): m/z = 456 [M+H]⁺
¹H-NMR (400 MHz, DMSO-d6) δ [ppm]: 1.066 (16.00), 1.156 (1.47), 1.400 (1.23), 1.424 (1.01), 3.565 (15.63), 3.938 (2.49), 4.112 (0.41).

### Intermediate 271

### tert-butyl (3R)-2'-[6-amino-5-(trifluoromethyl)pyridin-3-yl]-5',6'-dihydrospiro[pyrrolidine-3,4'-pyrrolo[1,2-b]pyrazole]-1-carboxylate

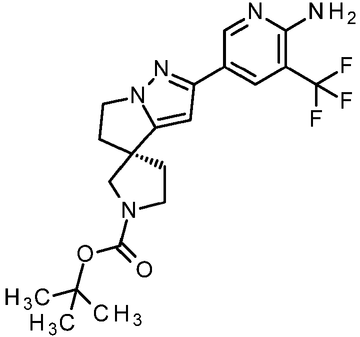

To a crude solution of 5-(4,4,5,5-tetramethyl-1,3,2-dioxaborolan-2-yl)-3-(trifluoromethyl)pyridin-2-amine (2.00 g, 6.94 mmol), tert-butyl (3R)-2'-[(trifluoromethanesulfonyl)oxy]-5',6'-dihydrospiro[pyrrolidine-3,4'-pyrrolo[1,2-b]pyrazole]-1-carboxylate (3.00 g, 7.29 mmol) and Potassium phosphate (42 ml, 0.50 M in water, 21 mmol) were added. The mixture was degassed and XPhos Pd G2 (273 mg, 347 µmol) was added and stirred at 100°C under Argon for 2h. The mixture was diluted with water and then extracted with Ethyl acetate 2x. The combined organics were washed with Brine, dried over Sodium sulfate, filterd and concentrated. The crude was purified by column chromatography to give 3.50 g of the title compound.
LC-MS (Method 1): Rt = 1.20 min; MS (ESlpos): m/z = 425 [M+H]⁺

### Intermediate 272

### tert-butyl (3'R)-2-[6-amino-5-(difluoromethoxy)pyridin-3-yl]-6,7-dihydrospiro[pyrazolo[5,1-c][1,4]oxazine-4,3'-pyrrolidine]-1'-carboxylate

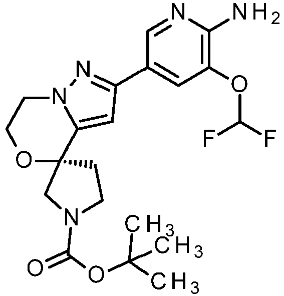

To a solution of 3-(difluoromethoxy)-5-(4,4,5,5-tetramethyl-1,3,2-dioxaborolan-2-yl)pyridin-2-amine (250 mg, 874 µmol) in Dioxane (9 mL), tert-butyl (3'R)-2-bromo-6,7-dihydrospiro[pyrazolo[5,1-c][1,4]oxazine-4,3'-pyrrolidine]-1'-carboxylate (344 mg, 961 µmol) and Potassium phosphate (5.2 ml, 0.50 M in water, 2.6 mmol) were added. The mixture was degassed and XPhos Pd G2 (34.4 mg, 43.7 µmol) was added. The mixture was stirred at 100°C under Argon for 1h. The mixture was diluted with water and then extracted with Ethyl acetate 2x. The combined organics were washed with Brine, dried over Sodium sulfate, filtered and concentrated in vacuo to give 423 mg of the title compound.
H-NMR-Data - 1H NMR (DMSO-d6, 400 MHz) δ 8.19 (d, 1H), 7.61 (s, 1H), 7.0-7.4 (m, 1H), 6.67 (d, 1H), 6.22 (s, 2H), 4.11 (s, 4H), 3.7-3.8 (m, 1H), 3.4-3.6 (m, 3H), 2.2-2.3 (m, 1H), 1.07 (s, 9H)
LC-MS (Method 1): Rt = 1.11 min; MS (ESlpos): m/z = 439 [M+H]⁺
¹H-NMR (400 MHz, DMSO-d6) δ [ppm]: 1.066 (16.00), 1.401 (2.51), 1.425 (2.11), 2.518 (0.45), 3.333 (13.40), 3.939 (2.32), 4.110 (1.11), 6.217 (0.68), 7.178 (0.64), 8.189 (0.44), 8.193 (0.43).

### Intermediate 273

### tert-butyl (3R)-2'-[6-amino-5-(difluoromethoxy)pyridin-3-yl]-5',6'-dihydrospiro[pyrrolidine-3,4'-pyrrolo[1,2-b]pyrazole]-1-carboxylate

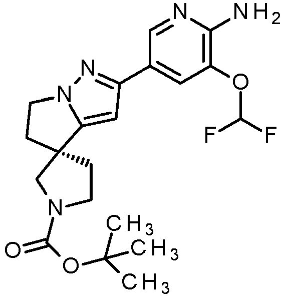

To a solution of 3-(difluoromethoxy)-5-(4,4,5,5-tetramethyl-1,3,2-dioxaborolan-2-yl)pyridin-2-amine (250 mg, 874 µmol) in Dioxane (9 mL), tert-butyl (3R)-2'-[(trifluoromethanesulfonyl)oxy]-5',6'-dihydrospiro[pyrrolidine-3,4'-pyrrolo[1,2-b]pyrazole]-1-carboxylate (395 mg, 961 µmol) and Potassium phosphate (5.2 ml, 0.50 M in water, 2.6 mmol) were added. The mixture was degassed and XPhos Pd G2 (34.4 mg, 43.7 µmol) was added and stirred at 100°C under Argon for 1h. The mixture was diluted with water and then extracted with Ethyl acetate 2x. The combined organics were washed with Brine, dried over Sodium sulfate, filtered and concentrated to give 401 mg of the title compound.
1H NMR (DMSO-d6, 400 MHz) δ 8.20 (d, 1H), 7.62 (s, 1H), 7.0-7.4 (m, 1H), 6.39 (d, 1H), 6.16 (s, 2H), 4.17 (dt, 2H), 3.51 (br d, 1H), 3.4-3.4 (m, 3H), 2.0-2.1 (m, 2H), 1.07 (s, 10H)
LC-MS (Method 1): Rt = 1.12 min; MS (ESlpos): m/z = 423 [M+H]⁺
¹H-NMR (400 MHz, DMSO-d6) δ [ppm]: 1.066 (16.00), 1.156 (0.45), 1.396 (2.13), 1.433 (1.66), 2.518 (0.44), 3.333 (6.64), 3.409 (0.77), 3.940 (1.29), 6.158 (0.60), 7.179 (0.60), 8.201 (0.58), 8.205 (0.61).

### Intermediate 274

### tert-butyl (3'R)-2-[6-amino-5-(trifluoromethyl)pyridin-3-yl]-6,7-dihydrospiro[pyrazolo[5,1-c][1,4]oxazine-4,3'-pyrrolidine]-1'-carboxylate

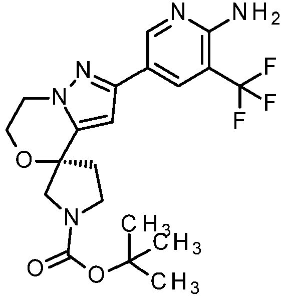

To a solution of 5-(4,4,5,5-tetramethyl-1,3,2-dioxaborolan-2-yl)-3-(trifluoromethyl)pyridin-2-amine (500 mg, 1.74 mmol) in Dioxane (18 mL), tert-butyl (3'R)-2-bromo-6,7-dihydrospiro[pyrazolo[5,1-c][1,4]oxazine-4,3'-pyrrolidine]-1'-carboxylate (933 mg, 2.60 mmol) and Potassium phosphate (10 ml, 0.50 M in water, 5.2 mmol) were added. The mixture was degassed and XPhos Pd G2 (68.3 mg, 86.8 µmol) was added and stirred at 100°C under Argon for 1h. The mixture was diluted with water and then extracted with Ethyl acetate 2x. The combined organics were washed with Brine, dried over Sodium sulfate and concentrated in vacuo. The crude was purified by column chromatography to give 448 mg (59 % yield) of the title compound.
1H NMR (DMSO-d6, 400 MHz) δ 8.57 (d, 1H), 8.01 (s, 1H), 6.75 (d, 1H), 6.60 (s, 2H), 4.1-4.2 (m, 4H), 3.7-3.8 (m, 1H), 3.5-3.6 (m, 1H), 3.4-3.5 (m, 1H), 3.4-3.4 (m, 1H), 2.1-2.3 (m, 1H), 1.4-1.4 (m, 9H)
LC-MS (Method 1): Rt = 1.21 min; MS (ESlpos): m/z = 440 [M+H]⁺
¹H-NMR (400 MHz, DMSO-d6) δ [ppm]: 1.066 (1.70), 1.401 (16.00), 1.415 (4.28), 1.425 (13.53), 2.318 (0.88), 2.322 (1.31), 2.326 (1.49), 2.332 (1.23), 2.336 (0.77), 2.518 (5.12), 2.523 (3.38), 2.664 (0.86), 2.669 (1.14), 2.673 (0.86), 3.159 (0.70), 3.171 (0.71), 3.345 (1.03), 3.364 (0.66), 3.376 (0.67), 3.392 (0.56), 3.421 (0.88), 3.451 (0.88), 3.476 (0.78), 3.505 (1.02), 3.527 (0.80), 3.550 (1.24), 3.573 (0.54), 3.695 (0.68), 3.727 (1.00), 3.757 (0.45), 4.084 (1.08), 4.095 (0.88), 4.119 (5.84), 6.598 (4.08), 6.744 (1.86), 6.765 (2.21), 8.005 (2.10), 8.572 (2.18), 8.576 (2.14).

### Intermediate 275

### tert-butyl (3'S)-2-[6-amino-5-(trifluoromethyl)pyridin-3-yl]-6,7-dihydro-1'H-spiro[pyrazolo[5,1-c][1,4]oxazine-4,3'-pyrrolidine]-1'-carboxylate

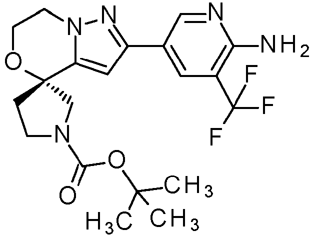

Intermediate 275 was prepared in analogy to Intermediate 270 using tert-butyl-(3'S)-2-bromo-6,7-dihydrospiro[pyrazolo[5,1-c][1,4]oxazine-4,3'-pyrrolidine]-1'-carboxylate (150 mg, 419 µmol, stereoisomer 1) and 5-(4,4,5,5-tetramethyl-1,3,2-dioxaborolan-2-yl)-3-(trifluoromethyl)pyridin-2-amine (181 mg, 628 µmol).
LC-MS (method 1): Rt = 1.20 min; MS (ESlpos): m/z = 440 [M+H]⁺
¹H-NMR (400 MHz, DMSO-d6) δ [ppm]: 1.066 (3.40), 1.156 (1.42), 1.232 (0.40), 1.401 (13.73), 1.425 (11.60), 2.085 (7.46), 2.323 (1.19), 2.327 (1.36), 2.331 (1.10), 2.518 (3.51), 2.523 (2.25), 2.665 (0.70), 2.669 (0.97), 2.673 (0.67), 3.365 (0.53), 3.375 (0.52), 3.391 (0.45), 3.421 (0.64), 3.451 (0.66), 3.476 (0.62), 3.506 (0.78), 3.528 (0.62), 3.551 (1.08), 3.573 (0.47), 3.697 (0.56), 3.726 (0.88), 3.939 (0.56), 4.119 (5.22), 5.759 (16.00), 6.600 (3.62), 6.746 (1.63), 6.766 (1.92), 8.006 (1.94), 8.572 (1.92), 8.577 (1.88).

### Intermediate 276

### tert-butyl (3'S)-2-(6-amino-5-cyanopyridin-3-yl)-6,7-dihydro-1'H-spiro[pyrazolo[5,1-c][1,4]oxazine-4,3'-pyrrolidine]-1'-carboxylate

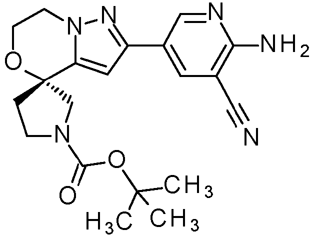

Intermediate 276 was prepared in analogy to Intermediate 270 using tert-butyl-(3'S)-2-bromo-6,7-dihydrospiro[pyrazolo[5,1-c][1,4]oxazine-4,3'-pyrrolidine]-1'-carboxylate (250 mg, 698 µmol, stereoisomer 1) and 2-amino-5-(4,4,5,5-tetramethyl-1,3,2-dioxaborolan-2-yl)pyridine-3-carbonitrile (257 mg, 1.05 mmol).
LC-MS (method 1): Rt = 1.08 min; MS (ESlpos): m/z = 397 [M+H]⁺
1H-NMR (400 MHz, DMSO-d6) δ [ppm]: 1.066 (2.44), 1.154 (1.13), 1.172 (2.22), 1.190 (1.08), 1.401 (16.00), 1.425 (14.03), 1.988 (3.85), 2.178 (0.42), 2.205 (0.45), 2.322 (1.05), 2.327 (1.22), 2.332 (1.02), 2.518 (2.92), 2.523 (1.80), 2.665 (0.50), 2.669 (0.73), 2.673 (0.51), 3.366 (0.71), 3.377 (0.67), 3.397 (0.97), 3.428 (0.82), 3.454 (0.75), 3.483 (0.88), 3.529 (0.76), 3.551 (1.31), 3.575 (0.57), 3.696 (0.67), 3.727 (1.06), 3.758 (0.49), 3.938 (0.46), 4.017 (0.92), 4.035 (0.92), 4.115 (7.84), 6.699 (1.92), 6.718 (2.22), 7.045 (4.84), 8.114 (2.41), 8.119 (2.39), 8.591 (4.30), 8.597 (4.25).

### Intermediate 277

### tert-butyl (3'R)-2-(6-amino-5-cyanopyridin-3-yl)-6,7-dihydro-1'H-spiro[pyrazolo[5,1-c][1,4]oxazine-4,3'-pyrrolidine]-1'-carboxylate

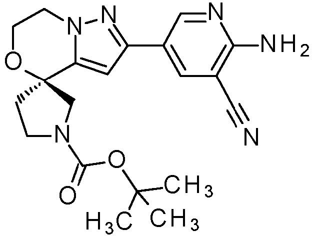

Intermediate 277 was prepared in analogy to Intermediate 270 using tert-butyl-(3'R)-2-bromo-6,7-dihydrospiro[pyrazolo[5,1-c][1,4]oxazine-4,3'-pyrrolidine]-1'-carboxylate (300 mg, 837 µmol, stereoisomer 2) and 2-amino-5-(4,4,5,5-tetramethyl-1,3,2-dioxaborolan-2-yl)pyridine-3-carbonitrile (308 mg, 1.26 mmol).
LC-MS (method 1): Rt = 1.06 min; MS (ESlneg): m/z = 395 [M-H]⁻
¹H-NMR (400 MHz, DMSO-d6) δ [ppm]: 1.066 (16.00), 1.154 (0.66), 1.172 (1.22), 1.190 (0.61), 1.401 (1.85), 1.425 (1.55), 1.988 (2.25), 2.518 (0.85), 2.523 (0.59), 3.938 (2.92), 4.017 (0.52), 4.035 (0.52), 4.115 (0.86), 5.758 (2.26), 7.045 (0.53), 8.591 (0.56), 8.597 (0.50).

### Intermediate 278

### (rac)-tert-butyl-2'-(6-amino-5-cyanopyridin-3-yl)-5',6'-dihydrospiro[pyrrolidine-3,4'-pyrrolo[1,2-b]pyrazole]-1-carboxylate

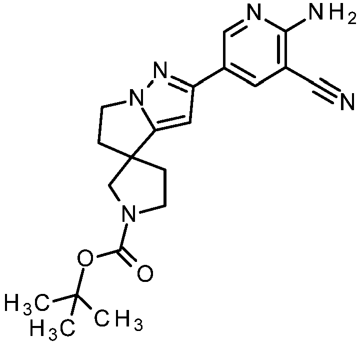

(Rac)-tert-butyl-2'-[(trifluoromethanesulfonyl)oxy]-5',6'-dihydrospiro[pyrrolidine-3,4'-pyrrolo[1,2-b]pyrazole]-1-carboxylate (713 mg, 1.73 mmol), 2-amino-5-(4,4,5,5-tetramethyl-1,3,2-dioxaborolan-2-yl)pyridine-3-carbonitrile (850 mg, 3.47 mmol), XPhos Pd G2 (68.2 mg, 86.7 µmol) and potassium phosphate (10 mL, 0.50 M, 5.2 mmol) were dissolved in degassed dioxane (25 mL) under argon and stirred at 100°C for 2h. The mixture was diluted with ethyl acetate and water, the layers were separated and the aqueous phase was extracted twice with ethyl acetate. The combined organic phases were dried over sodium sulfate and concentrated. Purified by flash chromatography to afford 730 mg of the title compound.

¹H-NMR (400 MHz, DMSO-d6) δ [ppm]: 1.065 (2.09), 1.396 (7.48), 1.432 (6.06), 2.029 (0.79), 2.047 (0.64), 2.518 (0.66), 2.523 (0.65), 2.534 (0.65), 2.542 (0.75), 2.560 (0.48), 3.159 (16.00), 3.172 (15.80), 3.386 (0.65), 3.407 (2.03), 3.434 (0.62), 3.496 (0.64), 4.087 (1.45), 4.101 (3.63), 4.113 (3.70), 4.127 (1.34), 4.154 (0.51), 4.163 (0.62), 4.172 (0.92), 4.181 (0.90), 4.189 (0.62), 4.198 (0.49), 6.454 (0.82), 6.479 (1.01), 6.985 (2.41), 8.151 (1.16), 8.156 (1.10), 8.611 (1.97), 8.618 (1.97).

### Intermediate 279

### (rac)-5-[5',6'-dihydrospiro[pyrrolidine-3,4'-pyrrolo[1,2-b]pyrazol]-2'-yl]-3-(trifluoromethoxy)pyridin-2-amine

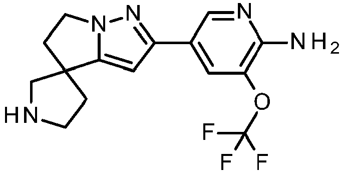

(rac)-tert-butyl-2'-[6-amino-5-(trifluoromethoxy)pyridin-3-yl]-5',6'-dihydrospiro[pyrrolidine-3,4'-pyrrolo[1,2-b]pyrazole]-1-carboxylate (590 mg, 1.34 mmol) was solubilised in 1,4-dioxane (12 mL), HCl (3.4 mL, 4.0 M in 1,4-dioxane, 13 mmol) was added and the mixture was stirred overnight at rt. HCl (1.7 mL, 4.0 M, 6.7 mmol) was added and the mixture was stirred for 1 h at 50°C. The mixture was concentrated under reduced pressure to give 590 mg of the title compound.
LC-MS (method 1): Rt = 0.85 min; MS (ESlpos): m/z = 340 [M+H]⁺

### Intermediate 280

### (rac)-3-(difluoromethoxy)-5-[5',6'-dihydrospiro[pyrrolidine-3,4'-pyrrolo[1,2-b]pyrazol]-2'-yl]pyridin-2-amine-hydrochloride salt

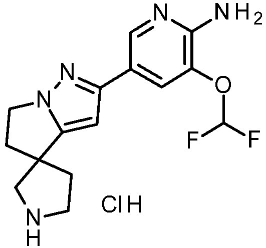

Intermediate 280 was prepared in analogy to Intermediate 279 using (rac)-tert-butyl-2'-[6-amino-5-(difluoromethoxy)pyridin-3-yl]-5',6'-dihydrospiro[pyrrolidine-3,4'-pyrrolo[1,2-b]pyrazole]-1-carboxylate (1.36 g, 3.23 mmol).
LC-MS (method 1): Rt = 0.77 min; MS (ESlpos): m/z = 323 [M+H]⁺

### Intermediate 281

### 5-[5',6'-dihydrospiro[pyrrolidine-3,4'-pyrrolo[1,2-b]pyrazol]-2'-yl]-3-[(1R)-1-(pyridin-3-yl)ethoxy]pyridin-2-amine-hydrochloride salt

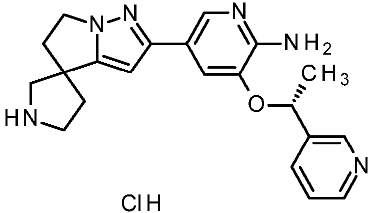

Intermediate 281 was prepared in analogy to Intermediate 279 using (rac)-tert-butyl-2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazole]-1-carboxylate (55.0 mg, 115 µmol).
LC-MS (method 1): Rt = 0.77 min; MS (ESlpos): m/z = 377 [M+H]⁺
¹H-NMR (400MHz, DMSO-d6): δ [ppm]= 1.68 (d, 3H), 2.07 - 2.24 (m, 2H), 2.55 - 2.62 (m, 1H), 2.69 - 2.77 (m, 1H), 3.20 - 3.50 (m, 4H), 4.17 - 4.25 (m, 2H), 6.19 (q, 1H), 6.70 (2s, 1H), 7.84 (s, 1H), 7.87 (s, 1H), 7.93 (br dd, 1H), 8.32 (br d, 2H), 8.56 (br s, 1H), 8.80 (d, 1H), 9.12 (s, 1H), 9.71 - 9.92 (m, 2H).

### Intermediate 282

### 5-[5',6'-dihydrospiro[pyrrolidine-3,4'-pyrrolo[1,2-b]pyrazol]-2'-yl]-3-[(1S)-1-(pyridin-3-yl)ethoxy]pyridin-2-amine-hydrochloride salt

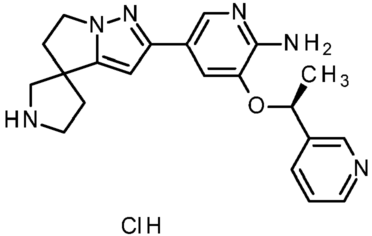

Intermediate 282 was prepared in analogy to Intermediate 279 using (rac)-tert-butyl-2'-{6-amino-5-[(1S)-1-(pyridin-3-yl)ethoxy]pyridin-3-yl}-5',6'-dihydrospiro[pyrrolidine-3,4'-pyrrolo[1,2-b]pyrazole]-1-carboxylate (60.0 mg, 126 µmol).
LC-MS (method 1): Rt = 0.77 min; MS (ESlpos): m/z = 377 [M+H]⁺
¹H-NMR (400MHz, DMSO-d6): δ [ppm]= 1.67 (d, 3H), 2.09 - 2.25 (m, 2H), 2.53 - 2.62 (m, 1H), 2.67 - 2.77 (m, 1H), 3.19 - 3.48 (m, 4H), 4.14 - 4.27 (m, 2H), 6.13 (q, 1H), 6.68 (2s, 1H), 7.75 - 7.83 (m, 2H), 7.85 (s, 1H), 8.26 (br s, 2H), 8.40 (br d, 1H), 8.72 (br d, 1H), 9.02 (s, 1H), 9.63 - 9.84 (m, 2H).

### Intermediate 283

### 5-[5',6'-dihydrospiro[pyrrolidine-3,4'-pyrrolo[1,2-b]pyrazol]-2'-yl]-3-[(1R)-1-(pyridin-2-yl)ethoxy]pyridin-2-amine-hydrochloride salt

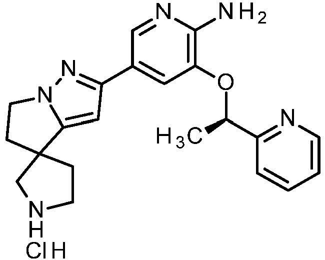

Intermediate 283 was prepared in analogy to Intermediate 279 using (rac)-tert-butyl-2'-{6-amino-5-[(1R)-1-(pyridin-2-yl)ethoxy]pyridin-3-yl}-5',6'-dihydrospiro[pyrrolidine-3,4'-pyrrolo[1,2-b]pyrazole]-1-carboxylate (238 mg, 499 µmol).
LC-MS (method 1): Rt = 0.79 min; MS (ESlpos): m/z = 378 [M+H]⁺
¹H-NMR (400 MHz, DMSO-d6) δ [ppm]: 1.065 (0.61), 1.593 (16.00), 1.688 (2.46), 1.705 (2.44), 2.144 (0.41), 2.323 (0.43), 2.327 (0.63), 2.332 (0.47), 2.518 (2.08), 2.523 (1.47), 2.665 (0.50), 2.669 (0.72), 2.673 (0.67), 3.384 (0.72), 3.396 (0.48), 3.551 (0.52), 3.941 (2.69), 4.184 (0.57), 6.604 (2.46), 7.618 (0.50), 7.641 (1.19), 7.826 (1.15), 7.922 (0.43), 8.604 (0.54), 8.616 (0.49).

### Intermediate 284

### 5-[5',6'-dihydrospiro[pyrrolidine-3,4'-pyrrolo[1,2-b]pyrazol]-2'-yl]-3-[(1S)-1-(pyridin-2-yl)ethoxy]pyridin-2-amine-hydrochloride salt

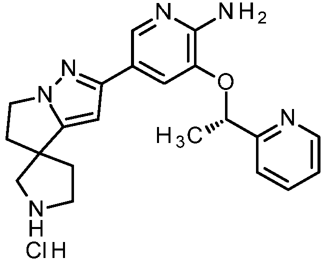

Intermediate 284 was prepared in analogy to Intermediate 279 using (rac)-tert-butyl-2'-{6-amino-5-[(1S)-1-(pyridin-2-yl)ethoxy]pyridin-3-yl}-5',6'-dihydrospiro[pyrrolidine-3,4'-pyrrolo[1,2-b]pyrazole]-1-carboxylate (335 mg, 93 % purity, 654 µmol).
LC-MS (method 1): Rt = 0.79 min; MS (ESlpos): m/z = 378 [M+H]⁺
¹H-NMR (400 MHz, DMSO-d6) δ [ppm]: 1.593 (16.00), 1.663 (0.65), 1.679 (0.90), 1.688 (3.30), 1.703 (3.30), 2.133 (0.49), 2.143 (0.64), 2.153 (0.58), 2.323 (0.74), 2.327 (1.04), 2.331 (0.78), 2.518 (6.25), 2.523 (4.40), 2.558 (0.58), 2.574 (0.51), 2.665 (0.81), 2.669 (1.27), 3.384 (0.81), 3.396 (0.72), 3.730 (0.41), 3.914 (4.94), 4.184 (0.85), 5.841 (0.58), 5.857 (0.57), 6.600 (3.18), 7.384 (0.48), 7.400 (0.60), 7.415 (0.55), 7.608 (0.72), 7.627 (0.92), 7.638 (1.45), 7.823 (1.57), 7.910 (0.67), 8.599 (0.85), 8.611 (0.76).

### Intermediate 285

### (rac)-5-[5',6'-dihydrospiro[pyrrolidine-3,4'-pyrrolo[1,2-b]pyrazol]-2'-yl]-3-[(trifluoromethyl)sulfanyl]pyridin-2-amine-hydrochloride salt

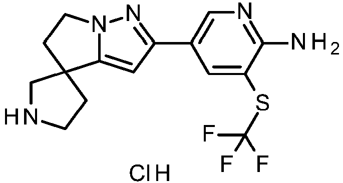

Intermediate 285 was prepared in analogy to Intermediate 279 using (rac)-tert-butyl-2'-{6-amino-5-[(trifluoromethyl)sulfanyl]pyridin-3-yl}-5',6'-dihydrospiro[pyrrolidine-3,4'-pyrrolo[1,2-b]pyrazole]-1-carboxylate (100 mg, 220 µmol).
LC-MS (method 1): Rt = 0.93 min; MS (ESlpos): m/z = 356 [M+H]⁺

### Intermediate 286

### (rac)-5-[5',6'-dihydrospiro[pyrrolidine-3,4'-pyrrolo[1,2-b]pyrazol]-2'-yl]-3-[(2-fluorophenyl)methoxy]pyridin-2-amine-hydrochloride salt

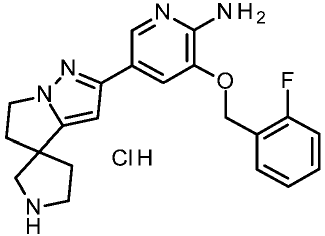

Intermediate 286 was prepared in analogy to Intermediate 279 using (rac)-tert-butyl-2'-{6-amino-5-[(2-fluorophenyl)methoxy]pyridin-3-yl}-5',6'-dihydrospiro[pyrrolidine-3,4'-pyrrolo[1,2-b]pyrazole]-1-carboxylate (488 mg, 1.02 mmol).
LC-MS (method 1): Rt = 0.95 min; MS (ESlpos): m/z = 380 [M+H]⁺

### Intermediate 287

### (rac)-5-[5',6'-dihydrospiro[pyrrolidine-3,4'-pyrrolo[1,2-b]pyrazol]-2'-yl]-3-[(3-fluorophenyl)methoxy]pyridin-2-amine-hydrochloride salt

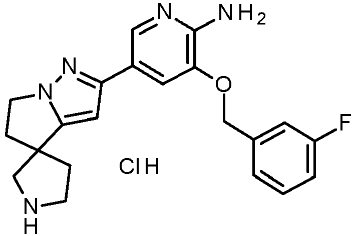

Intermediate 287 was prepared in analogy to Intermediate 279 using (rac)-tert-butyl-2'-{6-amino-5-[(3-fluorophenyl)methoxy]pyridin-3-yl}-5',6'-dihydrospiro[pyrrolidine-3,4'-pyrrolo[1,2-b]pyrazole]-1-carboxylate (440 mg, 918 µmol).
LC-MS (method 1): Rt = 0.95 min; MS (ESlpos): m/z = 380 [M+H]⁺

### Intermediate 288

### (rac)-3-[(2,6-dichlorophenyl)methoxy]-5-[5',6'-dihydrospiro[pyrrolidine-3,4'-pyrrolo[1,2-b]pyrazol]-2'-yl]pyridin-2-amine-hydrochloride salt

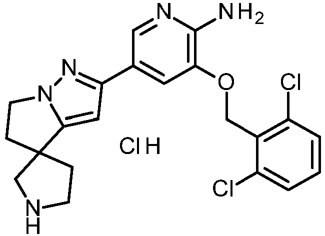

Intermediate 288 was prepared in analogy to Intermediate 279 using (rac)-tert-butyl-2'-{6-amino-5-[(2,6-dichlorophenyl)methoxy]pyridin-3-yl}-5',6'-dihydrospiro[pyrrolidine-3,4'-pyrrolo[1,2-b]pyrazole]-1-carboxylate (70.0 mg, 132 µmol).
LC-MS (method 1): Rt = 1.04 min; MS (ESlpos): m/z = 430 [M+H]⁺

### Intermediate 289

### (rac)-3-[(2-chlorophenyl)methoxy]-5-[5',6'-dihydrospiro[pyrrolidine-3,4'-pyrrolo[1,2-b]pyrazol]-2'-yl]pyridin-2-amine-hydrochloride salt

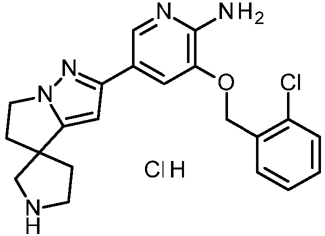

Intermediate 289 was prepared in analogy to Intermediate 279 using (rac)-tert-butyl-2'-{6-amino-5-[(2-chlorophenyl)methoxy]pyridin-3-yl}-5',6'-dihydrospiro[pyrrolidine-3,4'-pyrrolo[1,2-b]pyrazole]-1-carboxylate (188 mg, 379 µmol).
LC-MS (method 1): Rt = 0.99 min; MS (ESlpos): m/z = 396 [M+H]⁺

### Intermediate 290

### (rac)-5-[5',6'-dihydrospiro[pyrrolidine-3,4'-pyrrolo[1,2-b]pyrazol]-2'-yl]-3-[(pyridin-2-yl)methoxy]pyridin-2-amine-hydrochloride salt

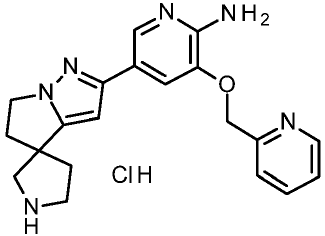

Intermediate 290 was prepared in analogy to Intermediate 279 using (rac)-tert-butyl-2'-{6-amino-5-[(pyridin-2-yl)methoxy]pyridin-3-yl}-5',6'-dihydrospiro[pyrrolidine-3,4'-pyrrolo[1,2-b]pyrazole]-1-carboxylate (310 mg, 670 µmol).
LC-MS (method 1): Rt = 0.76 min; MS (ESlpos): m/z = 363 [M+H]⁺

### Intermediate 291

### (rac)-3-[(2-chloro-5-fluorophenyl)methoxy]-5-[5',6'-dihydrospiro[pyrrolidine-3,4'-pyrrolo[1,2-b]pyrazol]-2'-yl]pyridin-2-amine-hydrochloride salt

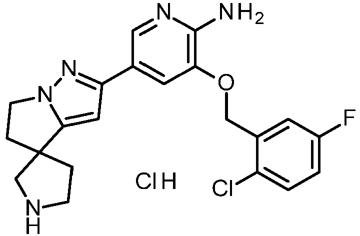

Intermediate 291 was prepared in analogy to Intermediate 279 using (rac)-tert-butyl-2'-{6-amino-5-[(2-chloro-5-fluorophenyl)methoxy]pyridin-3-yl}-5',6'-dihydrospiro[pyrrolidine-3,4'-pyrrolo[1,2-b]pyrazole]-1-carboxylate (130 mg, 253 µmol).
LC-MS (method 1): Rt = 1.01 min; MS (ESlpos): m/z = 414 [M+H]⁺

### Intermediate 292

### (rac)-3-(benzyloxy)-5-[5',6'-dihydrospiro[pyrrolidine-3,4'-pyrrolo[1,2-b]pyrazol]-2'-yl]pyridin-2-amine-hydrochloride salt

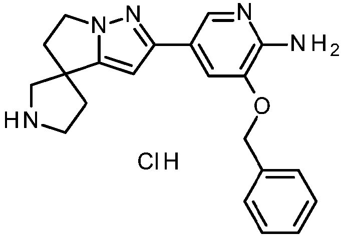

Intermediate 292 was prepared in analogy to Intermediate 279 using (rac)-tert-butyl-2'-[6-amino-5-(benzyloxy)pyridin-3-yl]-5',6'-dihydrospiro[pyrrolidine-3,4'-pyrrolo[1,2-b]pyrazole]-1-carboxylate (78.0 mg, 169 µmol).
LC-MS (method 1): Rt = 0.95 min; MS (ESlpos): m/z = 362 [M+H]⁺

### Intermediate 293

### (rac)-5-[5',6'-dihydrospiro[pyrrolidine-3,4'-pyrrolo[1,2-b]pyrazol]-2'-yl]-1'-methyl-1',2',3',6'-tetrahydro[3,4'-bipyridin]-2-amine-hydrochloride salt

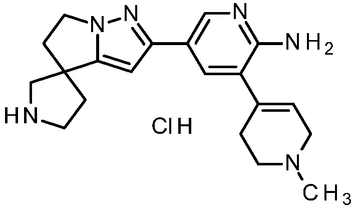

Intermediate 293 was prepared in analogy to Intermediate 279 using (rac)-tert-butyl-2'-(2-amino-1'-methyl-1',2',3',6'-tetrahydro[3,4'-bipyridin]-5-yl)-5',6'-dihydrospiro[pyrrolidine-3,4'-pyrrolo[1,2-b]pyrazole]-1-carboxylate (80.0 mg, 80 % purity, 142 µmol).
LC-MS (method 1): Rt = 0.70 min; MS (ESlpos): m/z = 352 [M+H]⁺
¹H-NMR (400 MHz, DMSO-d6) δ [ppm]: 1.064 (16.00), 1.154 (0.48), 1.291 (1.14), 2.158 (0.55), 2.178 (0.60), 2.522 (2.97), 2.879 (2.00), 2.891 (1.80), 3.255 (0.42), 3.269 (0.51), 3.284 (0.63), 3.383 (2.49), 3.564 (1.08), 4.212 (0.54), 4.223 (0.49), 5.944 (0.48), 6.687 (2.26), 8.070 (0.93), 8.075 (1.01), 8.259 (1.08), 8.264 (1.01).

### Intermediate 294

### 5-[5',6'-dihydrospiro[pyrrolidine-3,4'-pyrrolo[1,2-b]pyrazol]-2'-yl]-3-[(1R)-1-(pyridin-4-yl)ethoxy]pyridin-2-amine

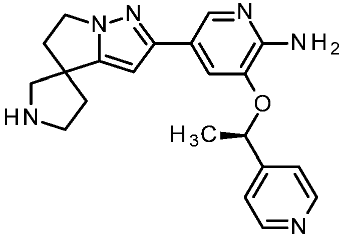

Intermediate 294 was prepared in analogy to Intermediate 279 using (rac)-tert-butyl-2'-{6-amino-5-[(1R)-1-(pyridin-4-yl)ethoxy]pyridin-3-yl}-5',6'-dihydrospiro[pyrrolidine-3,4'-pyrrolo[1,2-b]pyrazole]-1-carboxylate (220 mg, 462 µmol).
LC-MS (method 1): Rt = 0.71 min; MS (ESlpos): m/z = 377 [M+H]⁺
¹H-NMR (400 MHz, DMSO-d6) δ [ppm]: 1.064 (16.00), 1.383 (0.68), 1.399 (0.68), 1.592 (11.43), 1.639 (0.70), 1.648 (1.00), 1.655 (0.81), 1.663 (0.96), 2.518 (2.55), 2.522 (1.59), 3.383 (0.48), 6.658 (0.86), 6.660 (0.81), 7.728 (0.47), 7.879 (0.58), 8.853 (0.55), 8.869 (0.89), 8.882 (0.45).

### Intermediate 295

### 5-[5',6'-dihydrospiro[pyrrolidine-3,4'-pyrrolo[1,2-b]pyrazol]-2'-yl]-3-[(1S)-1-phenylethoxy]pyridin-2-amine-hydrochloride salt

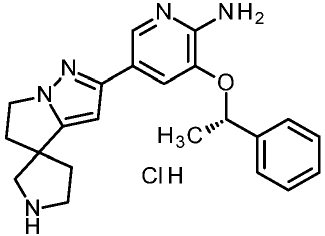

Intermediate 295 was prepared in analogy to Intermediate 279 using (rac)-tert-butyl-2'-{6-amino-5-[(1S)-1-phenylethoxy]pyridin-3-yl}-5',6'-dihydrospiro[pyrrolidine-3,4'-pyrrolo[1,2-b]pyrazole]-1-carboxylate (80.0 mg, 168 µmol).
LC-MS (method 1): Rt = 0.96 min; MS (ESlpos): m/z = 376 [M+H]⁺

### Intermediate 296

### 5-[5',6'-dihydrospiro[pyrrolidine-3,4'-pyrrolo[1,2-b]pyrazol]-2'-yl]-3-[(1R)-1-phenylethoxy]pyridin-2-amine-hydrochloride salt

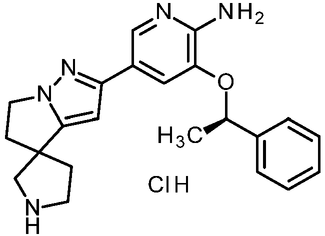

Intermediate 296 was prepared in analogy to Intermediate 279 using (rac)-tert-butyl-2'-{6-amino-5-[(1R)-1-phenylethoxy]pyridin-3-yl}-5',6'-dihydrospiro[pyrrolidine-3,4'-pyrrolo[1,2-b]pyrazole]-1-carboxylate (140 mg, 294 µmol).
LC-MS (method 1): Rt = 0.95 min; MS (ESlpos): m/z = 376 [M+H]⁺
¹H-NMR (400 MHz, DMSO-d6) δ [ppm]: 1.065 (1.41), 1.638 (0.50), 1.654 (0.48), 2.327 (0.48), 2.518 (1.92), 2.523 (1.23), 2.665 (0.45), 2.669 (0.56), 3.566 (16.00), 3.580 (3.69), 6.597 (0.84).

### Intermediate 297

### 5-[(3S)-5',6'-dihydrospiro[pyrrolidine-3,4'-pyrrolo[1,2-b]pyrazol]-2'-yl]-3-(trifluoromethyl)pyridin-2-amine

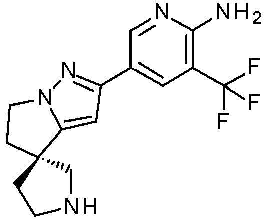

tert-butyl-(3S)-2'-[6-amino-5-(trifluoromethyl)pyridin-3-yl]-5',6'-dihydrospiro[pyrrolidine-3,4'-pyrrolo[1,2-b]pyrazole]-1-carboxylate (290 mg, 685 µmol) was stirred in TFE (7.0 mL, 96 mmol) at 130°C in the microwave for 16 h. The mixture was evaporated and purified by preparative HPLC to give 55.0 mg (95 % purity, 24 % yield) of the title compound.
¹H NMR (DMSO-d6) δ: 8.57 (d, 1H), 8.00 (d, 1H), 6.52 (s, 2H), 6.46 (s, 1H), 4.09-4.16 (m, 2H), 2.94-3.00 (m, 2H), 2.78-2.90 (m, 2H), 2.39-2.49 (m, 2H), 1.84-2.00 (m, 2H)
[α]²⁰_{D} : -11.1° (c = 1.00, MeOH)
LC-MS (method 1): Rt = 0.85 min; MS (ESlpos): m/z = 324 [M+H]⁺

### Intermediate 298

### 5-[(3R)-5',6'-dihydrospiro[pyrrolidine-3,4'-pyrrolo[1,2-b]pyrazol]-2'-yl]-3-(trifluoromethyl)pyridin-2-amine

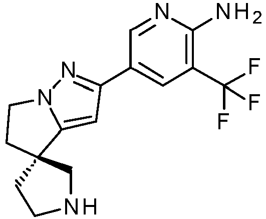

Intermediate 298 was prepared in analogy to Intermediate 297 using tert-butyl-(3R)-2'-[6-amino-5-(trifluoromethyl)pyridin-3-yl]-5',6'-dihydrospiro[pyrrolidine-3,4'-pyrrolo[1,2-b]pyrazole]-1-carboxylate (100 mg, 236 µmol).
1H NMR (DMSO-d6) δ: 8.57 (d, 1H), 8.00 (d, 1H), 6.52 (s, 2H), 6.46 (s, 1H), 4.06-4.23 (m, 2H), 2.91-3.03 (m, 2H), 2.77-2.90 (m, 2H),1.84-2.00 (m, 2H)
[α]²⁰_{D} : +14.3° (c = 1.00, MeOH)
LC-MS (method 1): Rt = 0.83 min; MS (ESlpos): m/z = 324 [M+H]⁺

### Intermediate 299

### 5-[(3S)-5',6'-dihydrospiro[pyrrolidine-3,4'-pyrrolo[1,2-b]pyrazol]-2'-yl]-3-(trifluoromethyl)pyridin-2-amine-hydrochloride salt

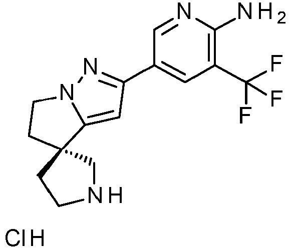

Intermediate 299 was prepared in analogy to Intermediate 279 using tert-butyl-(3S)-2'-[6-amino-5-(trifluoromethyl)pyridin-3-yl]-5',6'-dihydrospiro[pyrrolidine-3,4'-pyrrolo[1,2-b]pyrazole]-1-carboxylate (7.96 g, 18.8 mmol, stereoisomer 2).
LC-MS (method 1): Rt = 0.84 min; MS (ESlpos): m/z = 325 [M+H]⁺

### Intermediate 300

### 5-[(3S)-5',6'-dihydrospiro[pyrrolidine-3,4'-pyrrolo[1,2-b]pyrazol]-2'-yl]-3-(pyridazin-3-ylmethoxy)pyridin-2-amine-hydrochloride salt

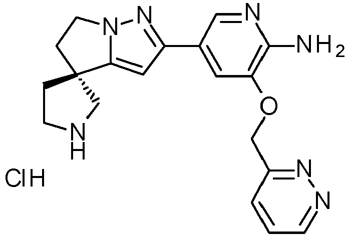

Intermediate 300 was prepared in analogy to Intermediate 279 using tert-butyl-(3S)-2'-{6-amino-5-[(pyridazin-3-yl)methoxy]pyridin-3-yl}-5',6'-dihydrospiro[pyrrolidine-3,4'-pyrrolo[1,2-b]pyrazole]-1-carboxylate (98.0 mg, 211 µmol, stereoisomer 1).
LC-MS (method 1): Rt = 0.64 min; MS (ESlpos): m/z = 364 [M+H]⁺

### Intermediate 301

### 5-[(3R)-5',6'-dihydrospiro[pyrrolidine-3,4'-pyrrolo[1,2-b]pyrazol]-2'-yl]-3-(pyridazin-3-ylmethoxy)pyridin-2-amine-hydrochloride salt

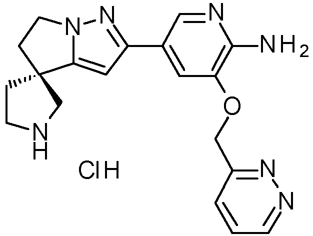

Intermediate 301 was prepared in analogy to Intermediate 279 using tert-butyl-(3R)-2'-{6-amino-5-[(pyridazin-3-yl)methoxy]pyridin-3-yl}-5',6'-dihydrospiro[pyrrolidine-3,4'-pyrrolo[1,2-b]pyrazole]-1-carboxylate (105 mg, 227 µmol, stereoisomer 2).
LC-MS (method 1): Rt = 0.64 min; MS (ESlpos): m/z = 364 [M+H]⁺

### Intermediate 302

### 5-[(3S)-5',6'-dihydrospiro[pyrrolidine-3,4'-pyrrolo[1,2-b]pyrazol]-2'-yl]-3-[1-(pyrimidin-5-yl)ethoxy]pyridin-2-amine hydrochloride (isomer 1)

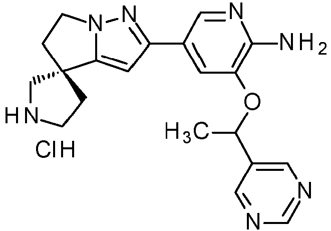

Intermediate 302 was prepared in analogy to Intermediate 279 using tert-butyl -2'-(6-amino-5-{[1-(pyrimidin-5-yl)ethyl]oxy}pyridin-3-yl)-5',6'-dihydrospiro[pyrrolidine-3,4'-pyrrolo[1,2-b]pyrazole]-1-carboxylate (84.0 mg, 176 µmol, isomer 1).
LC-MS (method 1): Rt = 0.69 min; MS (ESlpos): m/z = 378 [M+H]⁺

### Intermediate 303

### 5-[(S)-5',6'-dihydrospiro[pyrrolidine-3,4'-pyrrolo[1,2-b]pyrazol]-2'-yl]-3-{[1-(pyrimidin-5-yl)ethyl]oxy}pyridin-2-amine-hydrochloride salt (isomer 2)

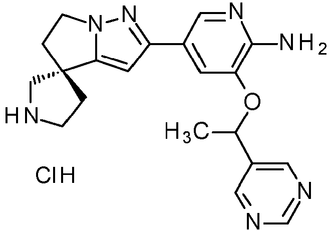

Intermediate 303 was prepared in analogy to Intermediate 279 using tert-butyl-2'-(6-amino-5-{[1-(pyrimidin-5-yl)ethyl]oxy}pyridin-3-yl)-5',6'-dihydrospiro[pyrrolidine-3,4'-pyrrolo[1,2-b]pyrazole]-1-carboxylate (104 mg, 218 µmol, isomer 3).
[α]²⁰_{D} : +5.7° (c = 1.00, DMSO)
LC-MS (method 1): Rt = 0.69 min; MS (ESlpos): m/z = 378 [M+H]⁺

### Intermediate 304

### 3-{[1-(5-chloro-3-fluoropyridin-2-yl)ethyl]oxy}-5-[5',6'-dihydrospiro[pyrrolidine-3,4'-pyrrolo[1,2-b]pyrazol]-2'-yl]pyridin-2-amine-hydrochloride salt (diastereomeric mixture)

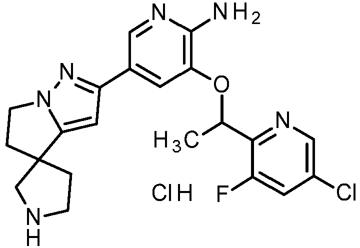

Intermediate 304 was prepared in analogy to Intermediate 279 using tert-butyl-2'-(6-amino-5-{[1-(5-chloro-3-fluoropyridin-2-yl)ethyl]oxy}pyridin-3-yl)-5',6'-dihydrospiro[pyrrolidine-3,4'-pyrrolo[1,2-b]pyrazole]-1-carboxylate (diastereomeric mixture) (65.0 mg, 123 µmol).
LC-MS (method 1): Rt = 0.96 min; MS (ESlpos): m/z = 429 [M+H]⁺

### Intermediate 305

### 5-(5',6'-dihydrospiro[azetidine-3,4'-pyrrolo[1,2-b]pyrazol]-2'-yl)-3-(trifluoromethyl)pyridin-2-amine

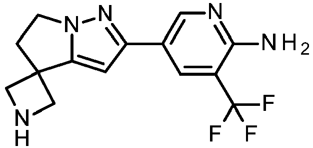

tert-butyl 2'-[6-amino-5-(trifluoromethyl)pyridin-3-yl]-5',6'-dihydrospiro[azetidine-3,4'-pyrrolo[1,2-b]pyrazole]-1-carboxylate (2.10 g, 5.13 mmol) was solubilised in 1,4-dioxane (45 mL) and HCl (13 mL, 4.0 M in 1,4-dioxane, 51 mmol) was added. The mixture was stirred overnight at rt. It was concentrated under reduced pressure to give 2.2 g of the title compound.
LC-MS (method 1): Rt = 0.81 min; MS (ESlpos): m/z = 310 [M+H]⁺

### Intermediate 306

### 2-amino-5-(5',6'-dihydrospiro[azetidine-3,4'-pyrrolo[1,2-b]pyrazol]-2'-yl)pyridine-3-carbonitrile-hydrochloride salt

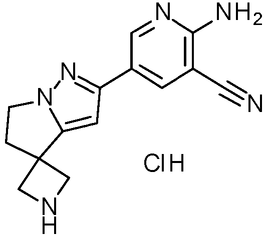

Intermediate 306 was prepared in analogy to Intermediate 305 using tert-butyl 2'-(6-amino-5-cyanopyridin-3-yl)-5',6'-dihydrospiro[azetidine-3,4'-pyrrolo[1,2-b]pyrazole]-1-carboxylate (2.60 g, 7.10 mmol).
LC-MS (method 1): Rt = 0.66 min; MS (ESlpos): m/z = 267 [M+H]⁺
¹H-NMR (400 MHz, DMSO-d6) δ [ppm]: 1.064 (9.31), 1.154 (7.68), 1.230 (0.76), 1.248 (0.74), 1.269 (2.13), 1.351 (0.40), 2.297 (0.91), 2.523 (7.54), 2.934 (3.74), 2.952 (6.03), 2.968 (4.00), 3.247 (0.64), 3.383 (0.92), 3.676 (0.46), 3.700 (0.47), 4.032 (2.51), 4.088 (2.88), 4.106 (3.92), 4.123 (6.34), 4.140 (4.27), 4.157 (5.70), 4.174 (10.27), 4.188 (5.13), 4.403 (0.45), 4.785 (3.36), 6.625 (2.98), 6.780 (16.00), 7.526 (0.82), 7.553 (0.84), 8.137 (0.46), 8.185 (7.99), 8.191 (7.86), 8.201 (1.88), 8.207 (1.84), 8.245 (0.97), 8.603 (8.32), 8.609 (8.25), 8.617 (2.05), 8.622 (1.85), 9.343 (1.15).

### Intermediate 307

### 3-(difluoromethoxy)-5-(5',6'-dihydrospiro[azetidine-3,4'-pyrrolo[1,2-b]pyrazol]-2'-yl)pyridin-2-amine-hydrochloride salt

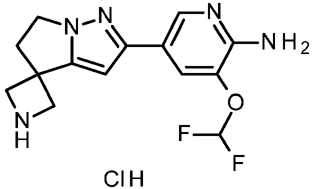

Intermediate 307 was prepared in analogy to Intermediate 305 using tert-butyl 2'-[6-amino-5-(difluoromethoxy)pyridin-3-yl]-5',6'-dihydrospiro[azetidine-3,4'-pyrrolo[1,2-b]pyrazole]-1-carboxylate (3.40 g, 8.35 mmol).
LC-MS (method 1): Rt = 0.72 min; MS (ESlpos): m/z = 308 [M+H]⁺

### Intermediate 308

### 5-(5',6'-dihydrospiro[azetidine-3,4'-pyrrolo[1,2-b]pyrazol]-2'-yl)-3-(trifluoromethoxy)pyridin-2-amine-hydrochloride salt

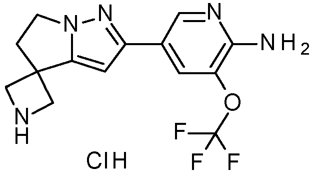

Intermediate 308 was prepared in analogy to Intermediate 305 using tert-butyl 2'-[6-amino-5-(trifluoromethoxy)pyridin-3-yl]-5',6'-dihydrospiro[azetidine-3,4'-pyrrolo[1,2-b]pyrazole]-1-carboxylate (1.40 g, 3.29 mmol).
LC-MS (method 1): Rt = 0.82 min; MS (ESlpos): m/z = 326 [M+H]⁺
¹H-NMR (400 MHz, DMSO-d6) δ [ppm]: 1.064 (16.00), 1.118 (0.84), 1.123 (0.86), 2.518 (10.94), 2.523 (7.50), 2.931 (1.49), 2.948 (2.41), 2.966 (1.62), 3.384 (1.06), 4.030 (0.92), 4.112 (1.58), 4.130 (2.52), 4.147 (1.63), 4.165 (2.49), 4.181 (4.28), 4.197 (2.45), 5.003 (0.61), 6.647 (0.85), 6.811 (4.35), 7.917 (1.40), 8.342 (3.33), 8.347 (3.44), 8.353 (0.77), 9.326 (0.79).

### Intermediate 309

### 5-(5',6'-dihydrospiro[azetidine-3,4'-pyrrolo[1,2-b]pyrazol]-2'-yl)-3-[(1S)-1-(pyridin-2-yl)ethoxy]pyridin-2-amine-hydrochloride salt

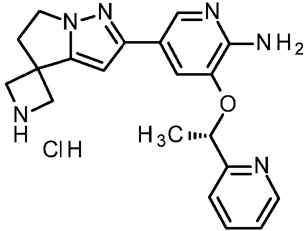

Intermediate 309 was prepared in analogy to Intermediate 305 using tert-butyl 2'-{6-amino-5-[(1S)-1-(pyridin-2-yl)ethoxy]pyridin-3-yl}-5',6'-dihydrospiro[azetidine-3,4'-pyrrolo[1,2-b]pyrazole]-1-carboxylate (31.6 mg, 68.3 µmol).
¹H-NMR (400MHz, DMSO-d6): δ [ppm]= 1.70 (d, 3H), 2.94 (t, 2H), 5.90 (q, 1H), 6.79 (s, 1H), 7.39 - 7.50 (m, 1H), 7.62 - 7.70 (m, 2H), 7.87 (d, 1H), 7.96 (br t, 1H), 8.14 - 8.49 (m, 2H), 8.64 (d, 1H), 9.38 - 9.66 (m, 2H), 13.40 - 14.82 (m, 1H).
LC-MS (method 1): Rt = 0.78 min; MS (ESlpos): m/z = 363 [M+H]⁺

### Intermediate 310

### 5-(5',6'-dihydrospiro[azetidine-3,4'-pyrrolo[1,2-b]pyrazol]-2'-yl)-3-[(1R)-1-(pyridin-2-yl)ethoxy]pyridin-2-amine-hydrochloride salt

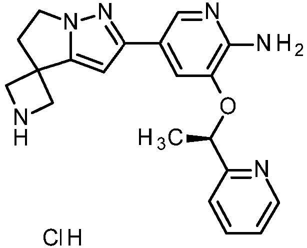

Intermediate 310 was prepared in analogy to Intermediate 305 using tert-butyl 2'-{6-amino-5-[(1R)-1-(pyridin-2-yl)ethoxy]pyridin-3-yl}-5',6'-dihydrospiro[azetidine-3,4'-pyrrolo[1,2-b]pyrazole]-1-carboxylate (100 mg, 70 % purity, 151 µmol).
LC-MS (method 1): Rt = 0.78 min; MS (ESlpos): m/z = 363 [M+H]⁺
¹H-NMR (400 MHz, DMSO-d6) δ [ppm]: 1.065 (16.00), 1.238 (2.15), 1.251 (2.00), 1.648 (0.67), 1.664 (0.68), 1.687 (1.04), 1.703 (1.01), 1.907 (0.89), 2.331 (2.28), 2.336 (1.00), 2.518 (14.04), 2.523 (9.58), 2.673 (2.29), 2.678 (1.04), 2.928 (0.46), 3.385 (0.62), 3.566 (6.08), 4.109 (0.49), 4.156 (0.50), 4.168 (0.58), 6.718 (0.86), 7.568 (0.49), 7.832 (0.69), 7.849 (0.46), 8.571 (0.42), 8.580 (0.42).

### Intermediate 311

### 5-(5',6'-dihydrospiro[azetidine-3,4'-pyrrolo[1,2-b]pyrazol]-2'-yl)-3-[(1R)-1-phenylethoxy]pyridin-2-amine-hydrochloride salt

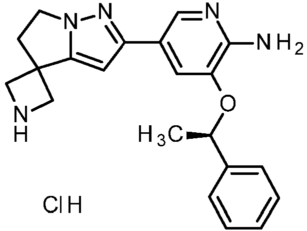

Intermediate 311 was prepared in analogy to Intermediate 305 using tert-butyl 2'-{6-amino-5-[(1R)-1-phenylethoxy]pyridin-3-yl}-5',6'-dihydrospiro[azetidine-3,4'-pyrrolo[1,2-b]pyrazole]-1-carboxylate (300 mg, 70 % purity, 455 µmol).
LC-MS (method 1): Rt = 0.96 min; MS (ESlpos): m/z = 362 [M+H]⁺
¹H-NMR (400 MHz, DMSO-d6) δ [ppm]: 1.065 (0.79), 1.156 (10.27), 1.232 (0.88), 1.256 (0.46), 1.642 (1.09), 1.657 (1.05), 1.780 (3.25), 1.797 (3.19), 1.907 (0.70), 2.327 (3.04), 2.331 (2.21), 2.518 (16.00), 2.523 (10.08), 2.669 (3.04), 2.673 (2.19), 2.939 (0.92), 3.252 (0.47), 3.385 (0.96), 3.457 (0.82), 3.470 (1.04), 3.487 (1.23), 3.499 (1.38), 3.663 (1.07), 3.667 (0.94), 3.677 (1.03), 3.699 (0.84), 3.709 (0.64), 3.714 (0.71), 3.725 (0.43), 3.729 (0.42), 4.005 (0.62), 4.025 (0.68), 4.094 (0.64), 4.113 (0.99), 4.137 (0.90), 4.153 (0.93), 4.174 (1.20), 4.189 (1.83), 4.205 (1.17), 5.336 (0.52), 5.353 (0.50), 5.760 (2.62), 6.602 (0.83), 6.652 (1.21), 6.656 (0.40), 6.744 (0.98), 6.784 (1.45), 7.286 (0.49), 7.303 (0.53), 7.321 (0.58), 7.339 (0.57), 7.355 (0.65), 7.362 (0.77), 7.375 (1.08), 7.381 (1.23), 7.393 (0.64), 7.398 (0.59), 7.467 (1.09), 7.486 (1.40), 7.489 (1.46), 7.507 (0.75), 7.619 (0.87), 7.775 (0.65), 7.803 (0.51), 7.958 (0.52), 8.134 (0.61), 11.749 (0.45).

### Intermediate 312

### 5-(5',6'-dihydrospiro[azetidine-3,4'-pyrrolo[1,2-b]pyrazol]-2'-yl)-3-[(1S)-1-phenylethoxy]pyridin-2-amine-hydrochloride salt

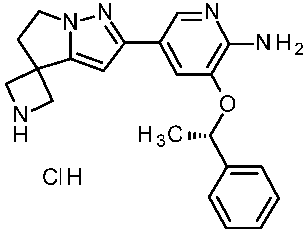

Intermediate 312 was prepared in analogy to Intermediate 305 using tert-butyl 2'-{6-amino-5-[(1S)-1-phenylethoxy]pyridin-3-yl}-5',6'-dihydrospiro[azetidine-3,4'-pyrrolo[1,2-b]pyrazole]-1-carboxylate (110 mg, 80 % purity, 191 µmol).
LC-MS (method 1): Rt = 1.02 min; MS (ESlpos): m/z = 398 [M+H]⁺

### Intermediate 313

### (rac)-5-[6,7-dihydrospiro[pyrazolo[5,1-c][1,4]oxazine-4,3'-pyrrolidin]-2-yl]-3-(trifluoromethoxy)pyridin-2-amine-hydrochloride salt

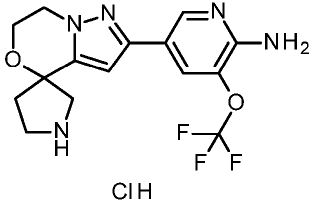

(rac)-tert-butyl-2-[6-amino-5-(trifluoromethoxy)pyridin-3-yl]-6,7-dihydrospiro[pyrazolo[5,1-c][1,4]oxazine-4,3'-pyrrolidine]-1'-carboxylate (2.00 g, 4.39 mmol) was solubilised in 1,4-dioxane (39 mL) and HCl (16.5 mL, 4.0 M in 1,4-dioxane, 66 mmol) was added. The mixture was stirred overnight at 50°C. It was concentrated under reduced pressure to give 1.20 g (70 % yield) of the title compound.
LC-MS (method 1): Rt = 0.82 min; MS (ESlpos): m/z = 356 [M+H]⁺
¹H-NMR (400 MHz, DMSO-d6) δ [ppm]: 1.064 (16.00), 1.281 (0.58), 3.564 (0.41), 4.155 (0.44), 4.162 (0.47), 6.822 (0.83), 8.339 (0.48), 8.344 (0.47).

### Intermediate 314

### (rac)-3-(difluoromethoxy)-5-[6,7-dihydrospiro[pyrazolo[5,1-c][1,4]oxazine-4,3'-pyrrolidin]-2-yl]pyridin-2-amine-hydrochloride salt

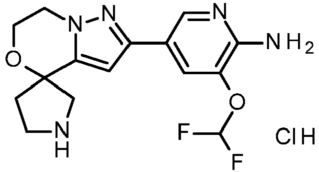

(rac)-tert-butyl-2-[6-amino-5-(difluoromethoxy)pyridin-3-yl]-6,7-dihydrospiro[pyrazolo[5,1-c][1,4]oxazine-4,3'-pyrrolidine]-1'-carboxylate (1.39 g, 3.18 mmol) was solubilised in 1,4-dioxane (28 mL) and HCl (11.9 mL, 4.0 M in 1,4-dioxane, 48 mmol) was added. The mixture was stirred overnight at 50°C. It was concentrated under reduced pressure to give 1.30 g of the title compound.
LC-MS (method 2): Rt = 0.50 min; MS (ESlpos): m/z = 338 [M+H]⁺
¹H-NMR (400 MHz, DMSO-d6) δ [ppm]: 1.063 (16.00), 2.518 (2.43), 2.523 (1.66), 3.314 (0.47), 3.383 (0.53), 4.157 (0.88), 4.164 (1.10), 4.178 (1.12), 6.875 (2.57), 7.221 (0.56), 7.402 (1.19), 7.582 (0.52), 8.009 (1.03), 8.207 (1.54), 8.211 (1.48).

### Intermediate 315

### 5-[(3'R)-6,7-dihydrospiro[pyrazolo[5,1-c][1,4]oxazine-4,3'-pyrrolidin]-2-yl]-3-(trifluoromethoxy)pyridin-2-amine hydrogen chloride

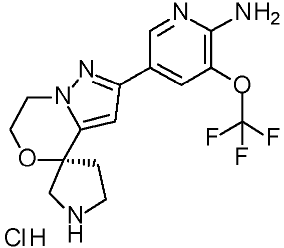

To a mixture of tert-butyl (3'R)-2-[6-amino-5-(trifluoromethoxy)pyridin-3-yl]-6,7-dihydrospiro[pyrazolo[5,1-c][1,4]oxazine-4,3'-pyrrolidine]-1'-carboxylate (534 mg, 1.17 mmol) in Dioxane (5.8 mL), HCl (5.9 ml, 4.0 M in dioxane, 23 mmol) was added and the solution was stirred at RT overnight. The mixture was concentrated in vacuo to give 760 mg of the title compound.
LC-MS (Method 1): Rt = 0.83 min; MS (ESlpos): m/z = 356 [M+H]⁺
¹H-NMR (400 MHz, DMSO-d6) δ [ppm]: 1.064 (16.00), 1.154 (2.10), 2.518 (0.58), 4.148 (0.68), 4.154 (0.79), 4.161 (0.81), 6.818 (1.06), 8.338 (0.61), 8.343 (0.57).

### Intermediate 316

### 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride

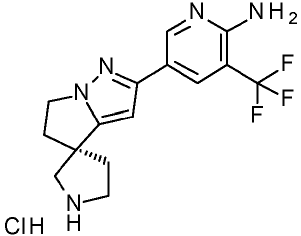

To a mixture of tert-butyl (3R)-2'-[6-amino-5-(trifluoromethyl)pyridin-3-yl]-5',6'-dihydrospiro[pyrrolidine-3,4'-pyrrolo[1,2-b]pyrazole]-1-carboxylate (2.90 g, 6.85 mmol) in Dioxane (34 mL), HCl (34 ml, 4.0 M in dioxane, 140 mmol) was added. The solution was stirred at RT overnight. The resultant mixture was concentrated in vacuo to give 3.10 g of the title compound.
LC-MS (Method 1): Rt = 0.83 min; MS (ESlpos): m/z = 324 [M+H]⁺
¹H-NMR (400 MHz, DMSO-d6) δ [ppm]: 1.064 (16.00), 1.122 (0.62), 1.154 (0.46), 2.144 (0.45), 2.156 (0.50), 2.163 (1.04), 2.171 (0.76), 2.183 (0.64), 2.188 (0.62), 2.522 (0.93), 2.580 (0.52), 2.594 (0.53), 2.678 (0.45), 2.696 (0.54), 2.712 (0.48), 3.287 (0.49), 3.402 (0.44), 3.409 (0.46), 3.419 (0.60), 3.436 (0.56), 3.448 (0.54), 3.564 (1.08), 4.184 (0.64), 4.192 (0.67), 4.203 (0.95), 4.211 (0.84), 4.219 (0.68), 4.226 (0.60), 6.666 (3.69), 8.107 (1.24), 8.112 (1.27), 8.584 (1.30), 8.589 (1.26).

### Intermediate 317

### 3-(difluoromethoxy)-5-[(3'R)-6,7-dihydrospiro[pyrazolo[5,1-c][1,4]oxazine-4,3'-pyrrolidin]-2-yl]pyridin-2-amine hydrogen chloride

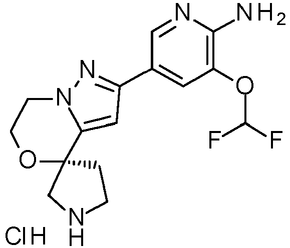

ICDE-245-1 -> to a mixture of tert-butyl (3'R)-2-[6-amino-5-(difluoromethoxy)pyridin-3-yl]-6,7-dihydrospiro[pyrazolo[5,1-c][1,4]oxazine-4,3'-pyrrolidine]-1'-carboxylate (382 mg, 873 µmol) in Dioxane, HCl (4mol/L in Dioxane) was added -> the solution was stirred at RT overnight, UPLC: ok -> the mixture was concentrated in vacuo -> the residue was stirred in Diethylether for 30min., then filtered and dried -> to give 311 mg (95 % yield) 311mg as a beige solid in a ~95% yield
LC-MS (Method 1): Rt = 0.74 min; MS (ESlpos): m/z = 338 [M+H]⁺
¹H-NMR (400 MHz, DMSO-d6) δ [ppm]: 1.064 (16.00), 1.069 (3.64), 1.087 (7.71), 1.104 (3.65), 1.399 (0.79), 1.424 (0.68), 2.518 (1.50), 2.523 (1.02), 3.288 (0.53), 3.305 (0.54), 3.319 (0.73), 3.336 (0.49), 3.352 (1.47), 3.370 (3.39), 3.384 (0.79), 3.387 (3.66), 3.405 (1.19), 3.698 (0.48), 4.105 (0.48), 4.119 (0.51), 4.155 (1.46), 4.162 (1.89), 4.174 (1.81), 6.852 (3.96), 7.188 (0.81), 7.369 (1.73), 7.550 (0.75), 7.949 (1.34), 8.205 (2.34), 8.210 (2.24).

### Intermediate 318

### 3-(difluoromethoxy)-5-[(3R)-5',6'-dihydrospiro[pyrrolidine-3,4'-pyrrolo[1,2-b]pyrazol]-2'-yl]pyridin-2-amine hydrogen chloride

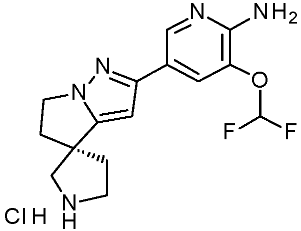

To a mixture of tert-butyl (3R)-2'-[6-amino-5-(difluoromethoxy)pyridin-3-yl]-5',6'-dihydrospiro[pyrrolidine-3,4'-pyrrolo[1,2-b]pyrazole]-1-carboxylate (368 mg, 873 µmol) in Dioxane (4.4 mL), HCl (4.4 ml, 4.0 M in dioxane, 17 mmol) was added. The solution was stirred at RT overnight. The mixture was concentrated and the residue was stirred in Diethylether for 30min, then filtered and concentrated to give 340 mg of the title compound.
LC-MS (Method 1): Rt = 0.76 min; MS (ESlpos): m/z = 322 [M+H]⁺
¹H-NMR (400 MHz, DMSO-d6) δ [ppm]: 1.064 (16.00), 1.070 (1.51), 1.087 (2.15), 1.105 (1.09), 1.108 (0.63), 1.154 (0.71), 2.144 (0.52), 2.163 (1.14), 2.178 (0.74), 2.183 (0.66), 2.192 (0.61), 2.518 (2.11), 2.523 (1.47), 2.583 (0.54), 2.598 (0.56), 2.712 (0.56), 2.728 (0.50), 3.254 (0.47), 3.268 (0.42), 3.283 (0.64), 3.343 (0.42), 3.353 (0.67), 3.371 (1.27), 3.384 (0.98), 3.388 (1.19), 3.405 (0.69), 3.412 (0.80), 3.426 (0.76), 3.442 (0.78), 3.455 (0.47), 4.198 (0.72), 4.207 (0.80), 4.213 (1.03), 4.222 (0.92), 4.227 (0.96), 4.232 (0.78), 4.241 (0.68), 6.681 (3.85), 7.189 (0.77), 7.370 (1.54), 7.551 (0.70), 7.955 (1.31), 8.178 (2.13), 8.182 (2.10), 9.688 (0.61).

### Intermediate 319

### 5-[(3'R)-6,7-dihydrospiro[pyrazolo[5,1-c][1,4]oxazine-4,3'-pyrrolidin]-2-yl]-3-(trifluoromethyl)pyridin-2-amine hydrogen chloride

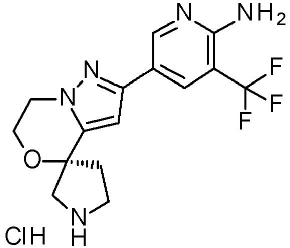

To a mixture of tert-butyl (3'R)-2-[6-amino-5-(trifluoromethyl)pyridin-3-yl]-6,7-dihydrospiro[pyrazolo[5,1-c][1,4]oxazine-4,3'-pyrrolidine]-1'-carboxylate (448 mg, 1.02 mmol) in Dioxane (5 mL), HCl (5.1 ml, 4.0 M in dioxane, 20 mmol) was added. The solution was stirred at RT overnight. The mixture was concentrated to give 480 mg of the title compound.
LC-MS (Method 1): Rt = 0.81 min; MS (ESlpos): m/z = 341 [M+H]⁺
¹H-NMR (400 MHz, DMSO-d6) δ [ppm]: 2.238 (0.58), 2.262 (1.07), 2.272 (1.06), 2.289 (0.90), 2.299 (1.39), 2.323 (2.56), 2.327 (2.83), 2.331 (1.88), 2.336 (0.83), 2.518 (10.46), 2.523 (7.21), 2.660 (0.80), 2.665 (1.78), 2.669 (2.52), 2.673 (1.73), 2.678 (0.76), 3.280 (0.71), 3.294 (1.80), 3.312 (1.91), 3.325 (2.35), 3.343 (1.51), 3.359 (0.83), 3.384 (2.87), 3.457 (0.47), 3.469 (0.82), 3.478 (0.95), 3.486 (1.55), 3.488 (1.51), 3.496 (1.44), 3.498 (1.43), 3.516 (1.08), 3.547 (0.51), 3.587 (0.66), 3.598 (0.53), 3.605 (0.40), 3.662 (0.55), 3.666 (0.64), 3.685 (1.17), 3.699 (1.50), 3.700 (1.51), 3.713 (1.20), 3.731 (1.04), 3.818 (0.79), 3.827 (0.51), 3.829 (0.91), 3.832 (0.49), 3.841 (1.07), 4.018 (1.26), 4.102 (0.45), 4.114 (1.23), 4.122 (2.19), 4.131 (1.27), 4.161 (9.94), 4.181 (1.52), 4.203 (0.55), 4.276 (0.53), 4.313 (0.90), 4.319 (0.79), 4.329 (3.90), 4.411 (2.64), 4.420 (2.35), 4.423 (2.84), 4.426 (2.49), 4.428 (2.39), 4.434 (3.13), 4.499 (4.03), 4.636 (1.25), 6.071 (0.51), 6.342 (1.37), 6.347 (1.56), 6.836 (16.00), 7.479 (1.31), 7.485 (1.16), 8.096 (4.36), 8.101 (4.41), 8.136 (2.26), 8.598 (4.41), 8.601 (4.34), 9.441 (0.80), 9.863 (0.82).

### Intermediate 320

### 5-[(3'S)-6,7-dihydrospiro[pyrazolo[5,1-c][1,4]oxazine-4,3'-pyrrolidin]-2-yl]-3-(trifluoromethyl)pyridin-2-amine -hydrochloride salt

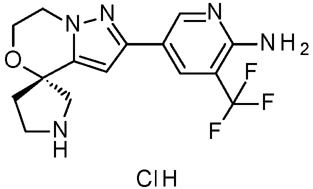

Intermediate 320 was prepared in analogy to Intermediate 319 using tert-butyl-(3'S)2-[6-amino-5-(trifluoromethyl)pyridin-3-yl]-6,7-dihydrospiro[pyrazolo[5,1-c][1,4]oxazine-4,3'-pyrrolidine]-1'-carboxylate (203 mg, 462 µmol).
LC-MS (method 2): Rt = 0.62 min; MS (ESlpos): m/z = 340 [M+H]⁺
¹H-NMR (400 MHz, DMSO-d6) δ [ppm]: 1.154 (1.60), 1.230 (0.58), 2.233 (0.53), 2.257 (1.07), 2.267 (0.98), 2.284 (0.88), 2.294 (1.35), 2.318 (1.11), 2.322 (1.06), 2.327 (1.14), 2.332 (0.78), 2.463 (1.34), 2.518 (3.84), 2.523 (2.52), 2.665 (0.76), 2.669 (1.08), 2.673 (0.76), 3.273 (0.61), 3.292 (1.63), 3.309 (1.53), 3.323 (2.29), 3.341 (1.35), 3.354 (0.75), 3.366 (0.47), 3.384 (0.59), 3.469 (0.50), 3.485 (0.81), 3.487 (0.78), 3.498 (1.09), 3.514 (0.93), 3.681 (0.98), 3.698 (1.27), 3.713 (1.11), 3.728 (0.90), 4.158 (8.69), 4.162 (9.17), 5.418 (1.21), 6.851 (16.00), 8.134 (4.43), 8.139 (4.37), 8.604 (4.60), 8.607 (4.38), 9.486 (0.79), 9.977 (0.81).

### Intermediate 321

### 2-amino-5-[(3'S)-6,7-dihydrospiro[pyrazolo[5,1-c][1,4]oxazine-4,3'-pyrrolidin]-2-yl]nicotinonitrile-hydrochloride salt

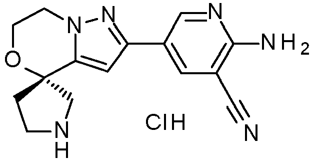

Intermediate 321 was prepared in analogy to Intermediate 319 using tert-butyl-(3'S)-2-(6-amino-5-cyanopyridin-3-yl)-6,7-dihydrospiro[pyrazolo[5,1-c][1,4]oxazine-4,3'-pyrrolidine]-1'-carboxylate (320 mg, 807 µmol).
[α]²⁰_{D} : -17.3° (c = 1.00, DMSO)
LC-MS (method 1): Rt = 0.66 min; MS (ESlpos): m/z = 297 [M+H]⁺

### Intermediate 322

### 2-amino-5-[(3'R)-6,7-dihydrospiro[pyrazolo[5,1-c][1,4]oxazine-4,3'-pyrrolidin]-2-yl]nicotinonitrile hydrochloride salt

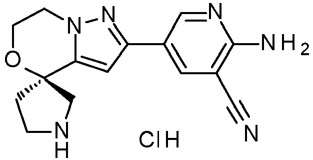

Intermediate 322 was prepared in analogy to Intermediate 319 using tert-butyl-2-(6-amino-5-cyanopyridin-3-yl)-6,7-dihydrospiro[pyrazolo[5,1-c][1,4]oxazine-4,3'-pyrrolidine]-1'-carboxylate (450 mg, 1.14 mmol, stereoisomer 2).
[α]²⁰_{D} : +6.2° (c = 1.00, DMSO)
LC-MS (method 1): Rt = 0.66 min; MS (ESlpos): m/z = 297 [M+H]⁺
¹H-NMR (400 MHz, DMSO-d6) δ [ppm]: 1.064 (16.00), 2.518 (1.23), 2.523 (0.81), 3.299 (0.42), 4.152 (3.52), 4.872 (0.56), 6.771 (3.16), 8.177 (1.60), 8.183 (1.56), 8.602 (2.01), 8.607 (2.06).

### Intermediate 323

### (rac)-methyl 2-amino-5-[1-(ethylcarbamoyl)-5',6'-dihydrospiro[pyrrolidine-3,4'-pyrrolo[1,2-b]pyrazol]-2'-yl]pyridine-3-carboxylate

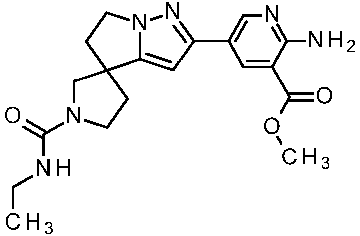

(rac)-1-(ethylcarbamoyl)-5',6'-dihydrospiro[pyrrolidine-3,4'-pyrrolo[1,2-b]pyrazol]-2'-yl trifluoromethanesulfonate (917 mg, 2.40 mmol) was solubilised in 1,4-dioxane (20 mL) under nitrogen, methyl 2-amino-5-(4,4,5,5-tetramethyl-1,3,2-dioxaborolan-2-yl)pyridine-3-carboxylate (1.00 g, 3.60 mmol), K3PO4 (14 mL, 0.50 M, 7.2 mmol) and XPhos Pd G2 (94.3 mg, 120 µmol) were added and the mixture was stirred overnight at 100°C. The mixture was diluted with ethyl acetate and washed with brine. The organic layer was dried over s silicone filter and evaporated. The residue was purified by flash chromatography to give 510 mg (55 % yield) of the title compound.
LC-MS (method 1): Rt = 0.82 min; MS (ESlpos): m/z = 385 [M+H]⁺
1H-NMR (400 MHz, DMSO-d6) δ [ppm]: 0.990 (0.17), 1.003 (0.60), 1.021 (1.11), 1.035 (0.42), 1.038 (0.49), 1.053 (0.66), 1.066 (16.00), 1.070 (0.88), 1.156 (2.03), 2.066 (0.17), 2.323 (0.29), 2.327 (0.40), 2.332 (0.28), 2.518 (1.15), 2.523 (0.98), 2.541 (0.21), 2.665 (0.26), 2.669 (0.37), 2.673 (0.27), 3.038 (0.26), 3.052 (0.24), 3.055 (0.24), 3.069 (0.18), 3.404 (0.63), 3.844 (1.90), 3.938 (1.36), 4.162 (0.17), 4.179 (0.25), 6.415 (0.67), 7.241 (0.31), 8.383 (0.34), 8.389 (0.34), 8.609 (0.39), 8.615 (0.37).

### Intermediate 324

### (rac)-1-(1,4-dimethyl-1H-pyrazol-5-yl)ethan-1-ol

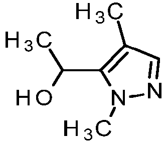

1-(1,4-Dimethyl-1H-pyrazol-5-yl)ethan-1-one (300 mg, 2.17 mmol) was solubilized in methanol (7.5 mL) and the reaction was cooled to 0 °C. Sodium borohydride (32.9 mg, 868 µmol) was added and the mixture was stirred at rt for 1 h. The reaction mixture was diluted with dichloromethane, washed with water and sat. sodium chloride solution, dried over sodium sulfate, filtered and concentrated under reduced pressure to give 193 mg (95 % purity, 60 % yield) of the title compound that was used without further purification in the next step.
LC-MS (Method 1): Rt = 0.59 min; MS (ESlpos): m/z = 141 [M+H]⁺
¹H-NMR (400MHz, CHLOROFORM-d): δ [ppm]= 1.64 (d, 3H), 2.16 (s, 3H), 2.26 (d, 1H), 4.00 (s, 3H), 5.18 (dd, 1H), 7.36 (s, 1H).

### SnAr NO2

### Intermediate 325

### (rac)-5-bromo-3-{[1-(6-methylpyridin-2-yl)ethyl]oxy}-2-nitropyridine

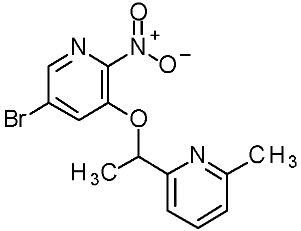

(Rac)-1-(6-Methylpyridin-2-yl)ethan-1-ol (200 mg, 1.46 mmol) was solubilised in THF (7.0 mL) and the reaction mixture was cooled to 0°C. Sodium hydride (79.5 mg, 60 % purity, 1.99 mmol) was then slowly added and the reaction mixture was stirred at 0°C for 30 min. A solution of 5-bromo-3-fluoro-2-nitropyridine (293 mg, 1.33 mmol) in THF (7.0 mL) was added slowly at 0°C and the reaction mixture was stirred for 1 h at 0°C. The reaction mixture was then slowly quenched with water and the organic solvent was removed under reduced pressure. The aqueous phase was extracted with dichloromethane and the organic phase was dried over sodium sulfate, filtered and concentrated under reduced pressure to give 440 mg (65 % purity, 63 % yield) of the title compound which was used without further purififcation in the next step.
LC-MS (Method 1): Rt = 1.25 min; MS (ESlpos): m/z = 338 [M+H]⁺

### Intermediate 326

### (rac)-5-bromo-3-{[1-(5-methylpyridin-2-yl)ethyl]oxy}-2-nitropyridine

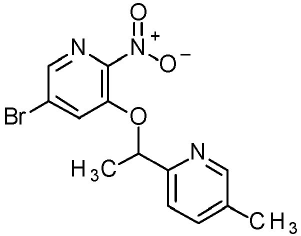

The compound was prepared similarly to **Intermediate 325** starting from (rac)-1-(5-methylpyridin-2-yl)ethan-1-ol (215 mg, 1.57 mmol) to give 490 mg (70% purity, 71 % yield) of the title compound.
LC-MS (Method 1): Rt = 1.24 min; MS (ESlpos): m/z = 338 [M+H]⁺

### Intermediate 327

### (rac)-5-bromo-3-{[1-(4-methylpyridin-2-yl)ethyl]oxy}-2-nitropyridine

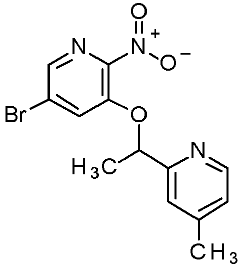

The compound was prepared similarly to **Intermediate 325** starting from (rac)-1-(4-methylpyridin-2-yl)ethan-1-ol (206 mg, 1.50 mmol) to give 466 mg (57 % purity, 57 % yield) of the title compound.
LC-MS (Method 1): Rt = 1.23 min; MS (ESlpos): m/z = 338 [M+H]⁺

### Intermediate 328

### (rac)-5-bromo-3-{[1-(3-methylpyridin-2-yl)ethyl]oxy}-2-nitropyridine

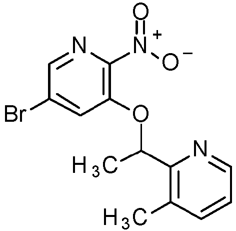

The compound was prepared similarly to **Intermediate 325** starting from (rac)-1-(3-methylpyridin-2-yl)ethan-1-ol (90.0 mg, 656 µmol) to give 240 mg (74 % purity, 88 % yield) of the title compound.
LC-MS (Method 1): Rt = 1.21 min; MS (ESlpos): m/z = 338 [M+H]⁺

### Intermediate 329

### (rac)-5-bromo-3-[1-(1,4-dimethyl-1H-pyrazol-5-yl)ethoxy]-2-nitropyridine

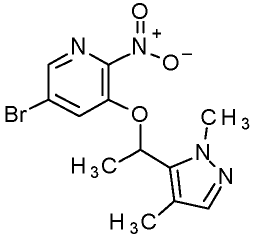

The compound was prepared similarly to **Intermediate 325** starting from (rac)-1-(1,4-dimethyl-1H-pyrazol-5-yl)ethan-1-ol (190 mg, 1.36 mmol) to give 452.7 mg (60 % purity, 58 %yield) of the title compound.
LC-MS (Method 1): Rt = 1.13 min; MS (ESlpos): m/z = 343 [M+H]⁺

### Intermediate 330

### 5-bromo-3-[(1S)-1-(2,6-difluorophenyl)ethoxy]-2-nitropyridine

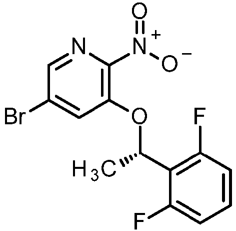

(1S)-1-(2,6-Difluorophenyl)ethan-1-ol (150 mg, 948 µmol) was dissolved in THF (4 mL) and cooled to 0 °C. Sodium hydride (41.7 mg, 60 % purity, 1.04 mmol) was added slowly and the mixture was stirred for 30 min at 0 °C. A solution of 5-bromo-3-fluoro-2-nitropyridine (210 mg, 948 µmol) in THF (4 mL) was added slowly and the mixture was stirred for 1 h at 0 °C. The reaction mixture was quenched with water and extracted with ethyl acetate. The combined organic layers were dried over sodium sulfate, filtered and concentrated to give 364 mg (80 % purity, 85 % yield) of the title compound which was used without further purification in the next step.

### Intermediate 331

### 5-bromo-3-[(1R)-1-(5-fluoropyridin-2-yl)ethoxy]-2-nitropyridine

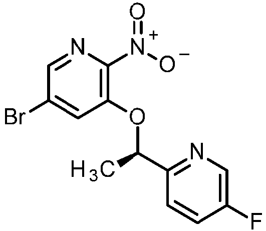

The compound was prepared similarly to **Intermediate 330** starting from (1R)-1-(5-fluoropyridin-2-yl)ethan-1-ol (150 mg, 1.06 mmol) to give 370 mg (75 % purity, 76 % yield) of the title compound.
LC-MS (Method 1): Rt = 1.23 min; MS (ESlpos): m/z = 342 [M+H]⁺

### Intermediate 332

### 5-bromo-3-[(1R)-1-(3-fluoropyridin-2-yl)ethoxy]-2-nitropyridine

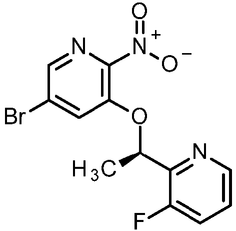

The compound was prepared similarly to **Intermediate 330** starting from (1R)-1-(3-fluoropyridin-2-yl)ethan-1-ol (150 mg, 1.06 mmol) to give 370 mg (65 % purity, 66 % yield) of the title compound.
LC-MS (Method 1): Rt = 1.18 min; MS (ESlpos): m/z = 342 [M+H]⁺

### Intermediate 333

### 5-bromo-3-[(1R)-1-(2,6-difluorophenyl)ethoxy]-2-nitropyridine

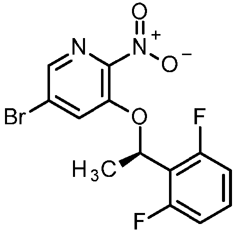

The compound was prepared similarly to **Intermediate 330** starting from (1R)-1-(2,6-difluorophenyl)ethan-1-ol (150 mg, 948 µmol) to give 322 mg (80 % purity, 76 % yield) of the title compound.
LC-MS (Method 1): Rt = 1.35 min; MS (ESlpos): m/z = 359 [M+H]⁺
¹H NMR (400 MHz, CHLOROFORM-d, 22°C) δ ppm 1.85 (d, 3 H), 5.82 (q, 1 H), 6.93 (t, 2 H), 7.31 (tt, 1 H), 7.58 (d, 1 H), 8.08 (d, 1 H).

### Intermediate 334

### (rac)-2-{1-[(5-bromo-2-nitropyridin-3-yl)oxy]ethyl}benzonitrile

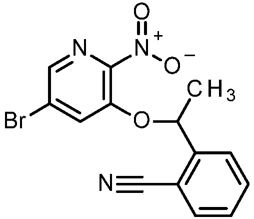

THF (6 mL) was initially introduced and cooled to 0 °C. Then, (rac)-2-[1-hydroxyethyl]benzonitrile (140 mg, 950 µmol) and sodium hydride (45.2 mg, 60 % purity, 1.13 mmol) were added and the mixture was stirred for 30 min at 0 °C. A solution of 5-bromo-3-fluoro-2-nitropyridine (200 mg, 905 µmol) in THF (4 mL) was added dropwise and the mixture was stirred for 2 h at 0 °C. The reaction mixture was quenched with water and extracted with ethyl acetate. The combined organic layers were dried over a hydrophobic filter and concentrated to give 333 mg (83 % purity, 88 % yield) of the title compound.
LC-MS (Method 1): Rt = 1.25 min; MS (ESlpos): m/z = 348 [M+H]⁺
¹H-NMR (400 MHz, DMSO-d6) δ [ppm]: 0.806 (0.40), 0.822 (0.51), 0.831 (0.60), 0.850 (0.73), 1.171 (0.41), 1.232 (1.95), 1.352 (2.14), 1.485 (0.44), 1.502 (0.43), 1.552 (4.42), 1.568 (4.58), 1.683 (15.91), 1.699 (16.00), 1.839 (0.43), 2.523 (1.96), 5.698 (0.66), 5.715 (0.63), 6.107 (1.02), 6.124 (3.35), 6.140 (3.33), 6.155 (0.97), 7.542 (2.05), 7.545 (2.13), 7.561 (4.29), 7.563 (4.34), 7.580 (2.97), 7.582 (2.75), 7.597 (1.05), 7.616 (0.67), 7.668 (3.09), 7.687 (5.07), 7.705 (1.05), 7.706 (1.02), 7.759 (2.63), 7.762 (2.83), 7.770 (0.92), 7.773 (1.12), 7.778 (3.64), 7.781 (3.71), 7.791 (1.09), 7.797 (1.57), 7.801 (1.57), 7.807 (0.47), 7.822 (0.89), 7.841 (0.76), 7.900 (3.88), 7.903 (3.82), 7.920 (3.53), 7.922 (3.34), 8.066 (0.40), 8.303 (6.06), 8.307 (10.67), 8.317 (9.08), 8.321 (5.28).

### Intermediate 335

### 3-{(1R)-1-[(5-bromo-2-nitropyridin-3-yl)oxy]ethyl}benzonitrile

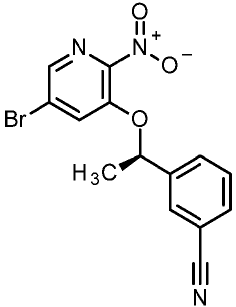

3-[(1R)-1-Hydroxyethyl]benzonitrile (150 mg, 1.02 mmol) was dissolved in THF (5 mL) and cooled to 0 °C. Sodium hydride (44.8 mg, 60 % purity, 1.12 mmol) was added slowly and the raction mixture was stirred for 30 min at 0 °C. A solution of 5-bromo-3-fluoro-2-nitropyridine 225 mg, 1.02 mmol) in THF (4 mL) was added slowly and the mixture was stirred for 1 h at 0 °C. The reaction mixture was quenched with water and extracted with ethyl acetate. The combined organic layers were dried over sodium sulfate, filtered and concentrated to give 371 mg (50 % purity, 52 % yield) of the title compound.
LC-MS (Method 1): Rt = 1.25 min; MS (ESlpos): m/z = 365 [M+NH₄]⁺

### Intermediate 336

### 4-{(1R)-1-[(5-bromo-2-nitropyridin-3-yl)oxy]ethyl}benzonitrile

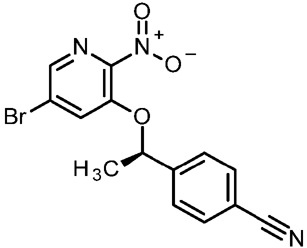

The compound was prepared similarly to **Intermediate 335** starting from 4-[(1R)-1-hydroxyethyl]benzonitrile (150 mg, 1.02 mmol) to give 363 mg (45 % purity, 46 % yield) of the title compound.
LC-MS (Method 1): Rt = 1.26 min; MS (ESlpos): m/z = 365 [M+NH₄]⁺

### Intermediate 337

### 5-bromo-3-[(1R)-1-(3,5-difluoropyridin-4-yl)ethoxy]-2-nitropyridine

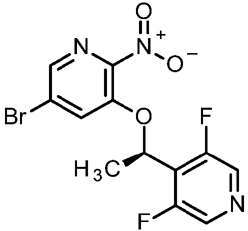

The compound was prepared similarly to **Intermediate 335** starting from (1R)-1-(3,5-difluoropyridin-4-yl)ethan-1-ol (150 mg, 943 µmol) to give 332 mg (60 % purity, 59 % yield) of the title compound.
LC-MS (Method 1): Rt = 1.17 min; MS (ESlpos): m/z = 360 [M+H]⁺

### Intermediate 338

### (rac)-5-bromo-2-nitro-3-(1-phenylpropoxy)pyridine

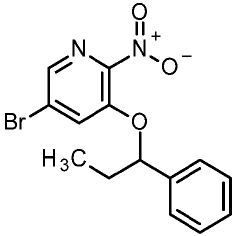

The compound was prepared similarly to **Intermediate 335** starting from (rac)-1-phenylpropan-1-ol (240 µL, 67 % purity, 1.2 mmol) to give 324 mg (75 % purity, 64 % yield) of the title compound which was used without further purification in the next step.
LC-MS (Method 1): Rt = 1.44 min; MS (ESlneg): m/z = 335 [M-H]⁻

### Intermediate 339

### 3-{(1S)-1-[(5-bromo-2-nitropyridin-3-yl)oxy]ethyl}benzonitrile

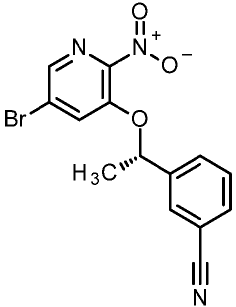

The compound was prepared similarly to **Intermediate 335** starting from 3-[(1S)-1-hydroxyethyl]benzonitrile (150 mg, 1.02 mmol) to give 374 mg (55 % purity, 58 % yield) of the title compound.
LC-MS (Method 1): Rt = 1.25 min; MS (ESlpos): m/z = 365 [M+NH₄]⁺

### Intermediate 340

### 4-{(1S)-1-[(5-bromo-2-nitropyridin-3-yl)oxy]ethyl}benzonitrile

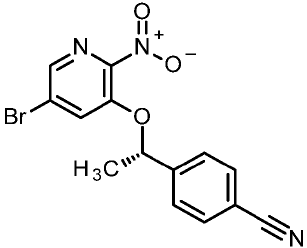

The compound was prepared similarly to **Intermediate 335** starting from 4-[(1S)-1-hydroxyethyl]benzonitrile (150 mg, 1.02 mmol) to give 366 mg (50 % purity, 52 % yield) of the title compound.
LC-MS (Method 1): Rt = 1.26 min; MS (ESlpos): m/z = 348 [M+N]⁺

### Intermediate 341

### 5-bromo-3-[(1S)-1-(3,5-difluoropyridin-4-yl)ethoxy]-2-nitropyridine

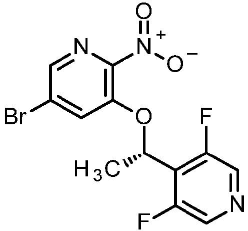

The compound was prepared similarly to **Intermediate 335** starting from (1S)-1-(3,5-difluoropyridin-4-yl)ethan-1-ol (162 mg, 1.02 mmol) to give 337 mg (68 % purity, 62 % yield) of the title compound.
LC-MS (Method 1): Rt = 1.18 min; MS (ESlpos): m/z = 360 [M+H]⁺

### Intermediate 342

### 5-bromo-2-nitro-3-[(1R)-1-phenylethoxy]pyridine

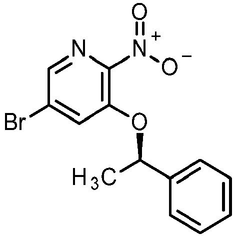

The compound was prepared similarly to **Intermediate 335** starting from (1R)-1-phenylethan-1-ol (230 µL, 63 % purity, 1.2 mmol) to give 349 mg (81 % purity, 77 % yield) of the title compound.
LC-MS (Method 1): Rt = 1.36 min; MS (ESlpos): m/z = 323 [M+H]⁺

### Intermediate 343

### (rac)-5-bromo-3-[1-(1-methyl-1H-pyrazol-5-yl)ethoxy]-2-nitropyridine

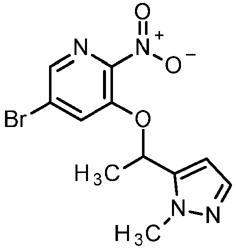

The compound was prepared similarly to **Intermediate 335** starting from 1-(1-methyl-1H-pyrazol-5-yl)ethan-1-ol (200 mg, 1.59 mmol) to give 379 mg (98 % purity, 72 % yield) of the title compound.
LC-MS (Method 1): Rt = 1.06 min; MS (ESlpos): m/z = 327 [M+H]⁺
¹H-NMR (400 MHz, CHLOROFORM-d) δ [ppm]: 1.791 (6.34), 1.807 (6.55), 3.946 (16.00), 5.595 (1.14), 5.612 (1.13), 6.324 (2.12), 6.328 (2.15), 7.459 (1.97), 7.463 (1.97), 7.546 (2.11), 7.550 (2.13), 8.151 (2.49), 8.157 (2.40).

### Intermediate 344

### 5-bromo-3-[(1R)-1-(2-fluorophenyl)ethoxy]-2-nitropyridine

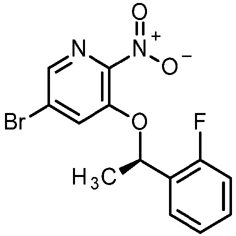

The compound was prepared similarly to **Intermediate 335** starting from (1R)-1-(2-fluorophenyl)ethan-1-ol (250 mg, 1.78 mmol) to give 495 mg (90 % purity, 73 % yield) of the title compound.
LC-MS (Method 1): Rt = 1.36 min; MS (ESlpos): m/z = 341 [M+H]⁺
¹H-NMR (400 MHz, CHLOROFORM-d) δ [ppm]: 1.682 (1.18), 1.698 (0.95), 1.708 (0.56), 1.718 (16.00), 1.734 (15.84), 5.776 (0.82), 5.792 (2.57), 5.807 (2.56), 5.823 (0.80), 7.092 (1.44), 7.095 (1.48), 7.112 (1.79), 7.116 (2.02), 7.117 (1.70), 7.121 (1.68), 7.138 (1.76), 7.141 (1.81), 7.173 (1.26), 7.175 (1.24), 7.192 (2.91), 7.194 (2.71), 7.211 (1.87), 7.213 (1.69), 7.307 (1.01), 7.312 (1.13), 7.321 (1.02), 7.326 (1.87), 7.328 (1.14), 7.330 (1.05), 7.333 (1.12), 7.340 (1.02), 7.341 (1.11), 7.344 (1.15), 7.346 (1.52), 7.351 (0.76), 7.360 (0.68), 7.364 (0.65), 7.446 (1.32), 7.450 (1.26), 7.465 (2.37), 7.469 (2.22), 7.484 (1.33), 7.489 (1.24), 7.502 (5.59), 7.507 (5.64), 7.921 (0.45), 7.927 (0.42), 8.082 (7.50), 8.087 (7.61).

### Intermediate 345

### 5-bromo-N-methyl-2-nitro-N-[(1R)-1-phenylethyl]pyridin-3-amine

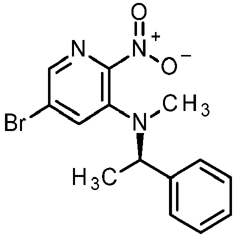

5-Bromo-3-fluoro-2-nitropyridine (200 mg, 905 µmol), (1R)-N-methyl-1-phenylethan-1-amine (150 µL, 1000 µmol) and triethylamine (130 µL, 950 µmol) were dissolved in THF (4.0 mL) and stirred over night at rt. The mixture was stirred for 2 h at 60 °C and concentrated. The residue was purified by flash column chromatography (silica gel, hexane / ethyla acetate (0-20 %) gradient) to give 184 mg (95 % purity, 57 % yield) of the title compound.
LC-MS (Method 1): Rt = 1.42 min; MS (ESlpos): m/z = 336 [M+H]⁺
¹H-NMR (400 MHz, CHLOROFORM-d) δ [ppm]: 1.612 (7.58), 1.629 (7.65), 2.582 (16.00), 4.730 (0.93), 4.748 (0.91), 7.230 (1.72), 7.247 (2.24), 7.249 (2.19), 7.252 (1.78), 7.260 (14.60), 7.307 (1.31), 7.322 (0.65), 7.324 (1.01), 7.328 (0.48), 7.357 (2.25), 7.361 (0.87), 7.372 (1.44), 7.376 (2.66), 7.380 (0.53), 7.393 (0.95), 7.396 (0.52), 7.570 (2.55), 7.575 (2.62), 7.978 (3.05), 7.982 (3.01), 7.986 (0.42).

### Intermediate 346

### 5-bromo-3-[(1R)-1-(4-methylpyridin-2-yl)ethoxy]-2-nitropyridine

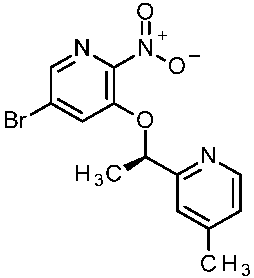

The compound was prepared similarly to **Intermediate 335** starting from (1R)-1-(4-methylpyridin-2-yl)ethan-1-ol (155 mg, 1.13 mmol) to give 290 mg (97 % purity, 74 % yield) of the title compound.
LC-MS (Method 1): Rt = 1.23 min; MS (ESlpos): m/z = 338 [M+H]⁺
¹H-NMR (400 MHz, CHLOROFORM-d) δ [ppm]: 1.240 (0.48), 1.258 (0.86), 1.276 (0.42), 1.718 (9.75), 1.734 (10.33), 2.045 (1.67), 2.357 (13.58), 5.462 (0.55), 5.477 (1.85), 5.494 (1.85), 5.510 (0.56), 7.067 (0.97), 7.069 (1.21), 7.071 (1.23), 7.082 (1.26), 7.084 (1.26), 7.260 (16.00), 7.272 (2.20), 7.274 (2.40), 7.618 (3.29), 7.622 (3.43), 8.060 (4.12), 8.065 (4.31), 8.426 (2.05), 8.439 (2.04).

### Intermediate 347

### 5-bromo-3-[(1R)-1-(6-methylpyridin-2-yl)ethoxy]-2-nitropyridine

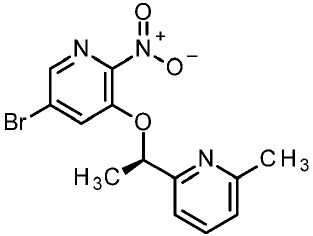

(1R)-1-(6-Methylpyridin-2-yl)ethan-1-ol (155 mg, 1.13 mmol) was dissolved in THF (6 mL) and cooled to 0 °C. Sodium hydride (49.8 mg, 60 % purity, 1.24 mmol) was added and the mixture was stirred for 30 min at 0 °C. A solution of 5-bromo-3-fluoro-2-nitropyridine (250 mg, 1.13 mmol) in THF (4 mL) was added slowly at 0 °C and the mixture was stirred for 1 h at 0 °C and over night at rt. The reaction mixture was stirred for 6 h at 70 °C. Sodium hydride (50 mg, 60 % purity, 1.24 mmol) was added at rt and stirred over night at rt. Sodium hydride (100 mg, 60 % purity, 2.48 mmol) was added again and the mixture was stirred over night at rt. The reaction mixture was diluted with water and extracted 2 times with ethyl acetate. The combined organic layers were dried over sodium sulfate, filtered and concentrated. The residue was purified by flash column chromatography (silica gel, hexane / ethyla acetate (0-45 %) gradient) to give 158 mg (95 % purity, 39 % yield) of the title compound.
LC-MS (Method 1): Rt = 1.26 min; MS (ESlpos): m/z = 338 [M+H]⁺
¹H-NMR (400 MHz, CHLOROFORM-d) δ [ppm]: 1.268 (0.75), 1.407 (0.43), 1.524 (3.09), 1.732 (11.25), 1.749 (10.85), 2.055 (1.11), 2.560 (0.80), 2.591 (16.00), 5.465 (0.55), 5.480 (1.77), 5.497 (1.79), 5.513 (0.56), 7.111 (1.78), 7.130 (2.00), 7.263 (1.71), 7.282 (2.08), 7.602 (1.68), 7.622 (3.10), 7.641 (1.40), 7.656 (3.39), 7.660 (3.55), 8.064 (5.31), 8.069 (5.30).

### Intermediate 348

### 5-bromo-3-[(1R)-1-(3-chlorophenyl)ethoxy]-2-nitropyridine

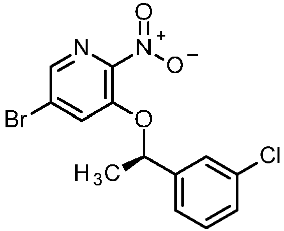

(1R)-1-(3-Chlorophenyl)ethan-1-ol (464 mg, 2.96 mmol) was dissolved in THF (5 mL), cooled to 0 °C, sodium hydride (158 mg, 60 % purity, 3.95 mmol) was added and the mixture was stirred for 30 min at 0 °C. 5-Bromo-3-fluoro-2-nitropyridine (450 mg, 97 % purity, 1.98 mmol) was dissolved in THF (5 mL) and added dropwise at 0 °C. The mixture was stirred for 1 h at 0 °C and 30 min at room temperature. The reaction mixture was poured into water. THF was distilled off and the aqueous phase was extracted with dichloromethane. The organic phase was dried over a hydrophobic filter and concentrated. The residue was purified by column chromatography (silica gel, hexane / ethyl acetate (2 - 20 %) gradient) to give 273 mg (98 % purity, 38 % yield) of the title compound.
[α]²⁰_{D}: +109.5° (c = 1.00, methanol)
LC-MS (Method 2): Rt = 1.44 min; MS (ESlpos): m/z = 357 [M+H]⁺
¹H-NMR (400 MHz, DMSO-d6) δ [ppm]: 1.153 (0.96), 1.165 (0.66), 1.170 (1.94), 1.188 (0.91), 1.566 (16.00), 1.582 (15.88), 1.986 (3.61), 2.518 (1.01), 2.523 (0.67), 4.016 (0.80), 4.034 (0.80), 5.948 (1.01), 5.963 (3.44), 5.979 (3.40), 5.995 (0.96), 7.372 (1.93), 7.376 (2.90), 7.380 (3.85), 7.385 (2.98), 7.394 (6.03), 7.396 (9.16), 7.402 (7.07), 7.405 (1.32), 7.420 (6.42), 7.433 (2.80), 7.438 (2.01), 7.443 (0.95), 7.458 (1.56), 7.508 (3.80), 7.512 (6.19), 7.517 (3.44), 8.254 (8.65), 8.259 (11.49), 8.283 (9.00), 8.288 (6.84).

### Intermediate 349

### 5-bromo-3-[(1R)-1-(2-methoxyphenyl)ethoxy]-2-nitropyridine

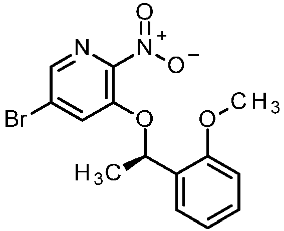

(1R)-1-(2-Methoxyphenyl)ethan-1-ol (390 µl, 2.7 mmol) was dissolved in THF (5 mL), cooled to 0 °C, sodium hydride (145 mg, 60 % purity, 3.62 mmol) was added and the mixture was stirred for 30 min at 0 °C. 5-Bromo-3-fluoro-2-nitropyridine (400 mg, 1.81 mmol) was dissolved in THF (4 mL) and added dropwise at 0 °C. The mixture was stirred for 1 h at 0 °C and 1 h at rt. The reaction mixture was poured into water. THF was distilled off and the aqueous phase was extracted with dichloromethane. The organic phase was dried over a hydrophobic filter and concentrated. The residue was purified by column chromatography (silica gel, hexane / ethyl acetate (0 - 15 %) gradient) to give 494 mg (97 % purity, 75 % yield) of the title compound.
[α]²⁰_{D}: +10.4° (c = 1.00, methanol)
LC-MS (Method 2): Rt = 1.42 min; MS (ESlpos): m/z = 353 [M+H]⁺
¹H NMR (400 MHz, DMSO-*d*₆, 22°C) δ ppm 1.57 (d, 3 H), 3.87 (s, 3 H), 6.05 (q, 1 H), 6.97 (td, 1 H), 7.06 - 7.09 (m, 1 H), 7.29 - 7.34 (m, 2 H), 7.97 (d, 1 H), 8.22 (d, 1 H).

### Intermediate 350

### 5-bromo-2-nitro-3-[(1R)-1-(pyridin-3-yl)ethoxy]pyridine

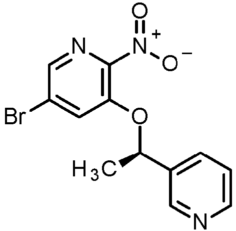

(1R)-1-(Pyridin-3-yl)ethan-1-ol (111 mg, 905 µmol) was dissolved in THF (1.8 mL), cooled to 0 °C, sodium hydride (39.8 mg, 60 % purity, 996 µmol) was added and the mxture was stirred for 30 min at 0 °C. 5-Bromo-3-fluoro-2-nitropyridine (200 mg, 905 µmol) was dissolved in THF (3 mL) and added dropwise. The mxture was stirred for 2 h at 0 °C. Sodium hydride (39.8 mg, 60 % purity, 996 µmol) was added again and stirred over night at room temperature. The reaction mixture was diluted with water and extracted with ethyl acetate. The combined organic layers were dried and concentrated to give 268 mg (85 % purity, 78 % yield) of the title compound.
LC-MS (Method 1): Rt = 1.08 min; MS (ESlpos): m/z = 324 [M+H]⁺
¹H-NMR (400 MHz, DMSO-d6) δ [ppm]: 0.822 (0.44), 0.831 (0.54), 0.851 (0.73), 1.232 (1.97), 1.600 (1.51), 1.609 (16.00), 1.625 (15.61), 1.640 (2.90), 1.657 (2.79), 2.518 (5.06), 2.523 (3.76), 2.632 (2.33), 2.679 (0.41), 6.022 (0.90), 6.038 (3.06), 6.054 (3.04), 6.070 (0.88), 6.220 (0.50), 6.236 (0.51), 7.393 (0.47), 7.405 (0.46), 7.421 (2.40), 7.423 (2.42), 7.433 (2.50), 7.435 (2.53), 7.441 (2.50), 7.444 (2.50), 7.453 (2.56), 7.455 (2.57), 7.574 (0.40), 7.814 (1.80), 7.819 (3.01), 7.823 (1.99), 7.829 (0.56), 7.834 (2.24), 7.839 (3.02), 7.844 (1.75), 7.848 (0.47), 7.854 (0.53), 8.081 (1.33), 8.086 (1.98), 8.098 (1.00), 8.104 (0.48), 8.123 (0.88), 8.128 (0.64), 8.263 (9.65), 8.267 (10.78), 8.356 (7.52), 8.360 (6.86), 8.490 (0.60), 8.494 (0.61), 8.502 (0.60), 8.506 (0.73), 8.532 (3.84), 8.536 (4.25), 8.544 (4.00), 8.548 (3.92), 8.651 (0.81), 8.656 (0.99), 8.669 (4.32), 8.673 (4.32).

### Intermediate 351

### 5-bromo-2-nitro-3-[(1R)-1-(1,3-thiazol-2-yl)ethoxy]pyridine

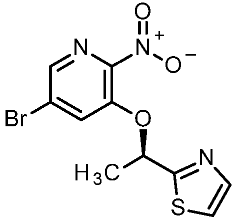

The compound was prepared similarly to **Intermediate 335** starting from (1R)-1-(1,3-thiazol-2-yl)ethanol (421 mg, 3.2 mmol) to give 343.6 mg (100 % purity, 33 % yield) of the title compound.
LC-MS (Method 2): Rt = 1.17 min; MS (ESlpos): m/z = 330 [M+H]⁺
¹H-NMR (400MHz, DMSO-d₆): δ [ppm]= 1.72 (d, 3H), 6.32 (q, 1H), 7.80 (d, 1H), 7.84 (d, 1H), 8.33 (d, 1H), 8.51 (d, 1H).

### Intermediate 352

### 5-bromo-3-[(1R)-1-(3-fluorophenyl)ethoxy]-2-nitropyridine

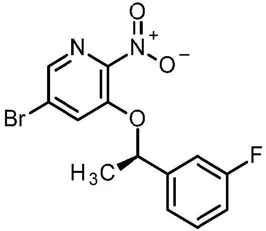

The compound was prepared similarly to **Intermediate 335** starting from (1R)-1-(3-fluorophenyl)ethan-1-ol (360 µL, 98 % purity, 3.3 mmol) to give 346 mg (100 % purity, 37 % yield) of the title compound.
[α]²⁰_{D}: +108.6° (c = 1.00, methanol)
LC-MS (Method 1): Rt = 1.35 min; MS (ESlpos): m/z = 341 [M+H]⁺
¹H-NMR (400 MHz, DMSO-d6) δ [ppm]: 1.569 (15.99), 1.585 (16.00), 1.986 (0.51), 2.518 (0.71), 2.523 (0.47), 5.955 (0.94), 5.971 (3.16), 5.987 (3.12), 6.003 (0.91), 7.132 (0.99), 7.134 (1.08), 7.138 (1.20), 7.141 (1.23), 7.155 (2.17), 7.161 (2.41), 7.175 (1.26), 7.180 (1.35), 7.184 (1.40), 7.250 (2.46), 7.256 (4.23), 7.271 (1.91), 7.275 (5.52), 7.280 (5.67), 7.423 (1.90), 7.438 (2.29), 7.443 (2.86), 7.453 (0.53), 7.458 (2.94), 7.462 (1.63), 7.476 (0.75), 7.479 (1.07), 8.249 (7.61), 8.253 (12.59), 8.266 (9.55), 8.270 (5.84).

### Intermediate 353

### (rac)-5-bromo-2-nitro-3-[1-(1,3-oxazol-4-yl)ethoxy]pyridine (enantiomer 1 and enantiomer 2)

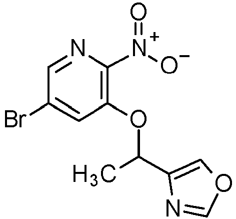

The compound was prepared similarly to **Intermediate 335** starting from (rac)-1-(1,3-oxazol-4-yl)ethanol (270 mg, 1.2 mmol) to give 194 mg (100 % purity, 51 % yield) of the title compound.
LC-MS (Method 2): Rt = 1.06 min; MS (ESlpos): m/z = 314 [M+H]⁺
¹H-NMR (400MHz, DMSO-d₆): δ [ppm]= 1.61 (d, 3H), 5.93 (q, 1H), 8.23 (s, 1H), 8.28 (d, 1H), 8.39 (d, 1H), 8.50 (d, 1H).

### Intermediate 354

### (rac)-5-bromo-2-nitro-3-[1-(1,3-oxazol-2-yl)ethoxy]pyridine (enantiomer 1 and enantiomer 2)

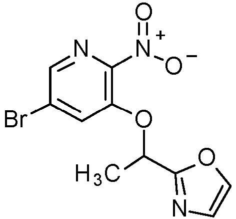

5-Bromo-3-fluoro-2-nitropyridine (100 mg, 453 µmol) and (rac)-1-(1,3-oxazol-2-yl)ethan-1-ol (52.7 mg, 466 µmol) were dissolved in THF (3 mL). Cesium carbonate (162 mg, 498 µmol) was added and the mixture was stirred for 4.5 h at 65 °C. The reaction mixture was diluted with water and extracted with dichloromethane. The combined organic layers were dried over magnesium sulfate, filtered and concentrated to give 101 mg (85 % purity, 60 % yield) of the title compound.
LC-MS (Method 2): Rt = 1.07 min; MS (ESlpos): m/z = 314 [M+H]⁺
¹H-NMR (400MHz, DMSO-d₆): δ [ppm]= 1.70 (d, 3H), 6.12 (q, 1H), 7.27 (d, 1H), 8.19 (d, 1H), 8.34 (d, 1H), 8.53 (d, 1H).

### Intermediate 355

### 2-{(1R)-1-[(5-bromo-2-nitropyridin-3-yl)oxy]ethyl}pyrimidine

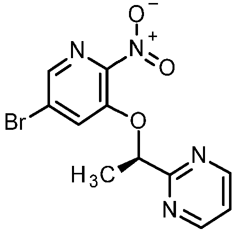

The compound was prepared similarly to **Intermediate 335** starting from (1R)-1-(pyrimidin-2-yl)ethan-1-ol (295 mg, 2.38 mmol) to give 688 mg (94 % yield) of the title compound.
[α]²⁰_{D}: +25.1° (c = 1.00, methanol)
LC-MS (Method 1): Rt = 1.03 min; MS (ESlpos): m/z = 325 [M+H]⁺
¹H NMR (DMSO-d₆, 400 MHz): δ (ppm) 8.83 (d, 2H), 8.26 (d, 1H), 8.22 (d, 1H), 7.48 (t, 1H), 6.00 (q, 1H), 1.69 (d, 3H).

### Intermediate 356

### 5-bromo-2-nitro-3-[(1R)-1-(pyridin-3-yl)ethoxy]pyridine

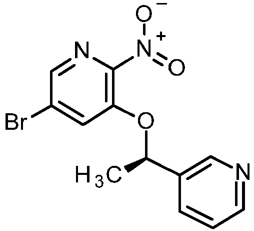

5-Bromo-2-nitropyridin-3-ol (1.14 g, 5.20 mmol) was dissolved in THF (46 mL) under argon. (1S)-1-(pyridin-3-yl)ethan-1-ol (646 mg, 5.25 mmol) and triphenylphosphine (1.77 g, 6.76 mmol) were added and stirred for 1 h at room temperature. The reaction mixture was cooled to 0 to 4 °C and diisopropylazodicarboxylate (1.3 ml, 6.8 mmol) was added. The mixture was stirred over night at room temperature. The reaction mixture was concentrated to give 5.07 g (301 % yield) of the title compound.
LC-MS (Method 2): Rt = 0.89 min; MS (ESlpos): m/z = 324 [M+H]⁺
¹H-NMR (400MHz, DMSO-d₆): δ [ppm]= 1.62 (d, 3H), 6.05 (q, 1H), 7.41 - 7.47 (m, 1H), 7.83 (dt, 1H), 8.26 (d, 1H), 8.36 (d, 1H), 8.54 (dd, 1H), 8.67 (d, 1H).

### Intermediate 357

### 5-bromo-2-nitro-3-[(1R)-1-(1,2-thiazol-5-yl)ethoxy]pyridine

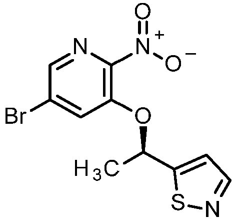

5-Bromo-3-fluoro-2-nitropyridine (230.0 mg, 1041 µmol) and (1R)-1-(1,2-thiazol-5-yl)ethan-1-ol (138.5 mg, 1072 µmol) were dissolved in THF (6.9 mL). Cesium carbonate (373.0 mg, 1145 µmol) was added and the mixture was stirred for 20 h at 65 °C. The reaction mixture was allowed to cool down, diluted with water and extracted with dichloromethane. The combined organic layers were dried over magnesium sulfate, filtered and concentrated to give 262.0 mg (68 % purity, 52 % yield) of the title compound.
LC-MS (Method 2): Rt = 1.19 min; MS (ESlpos): m/z = 330 [M+H]⁺
¹H-NMR (400MHz, DMSO-d₆): δ [ppm]= 1.70 (d, 3H), 6.45 (q, 1H), 7.47 (dd, 1H), 8.32 (d, 1H), 8.47 (d, 1H), 8.53 (d, 1H).

### Intermediate 358

### (rac)-5-bromo-3-[1-(2,6-dichlorophenyl)ethoxy]-2-nitropyridine

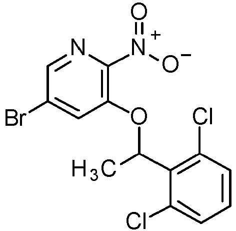

(Rac)1-(2,6-Dichlorophenyl)ethan-1-ol (500 mg, 2.62 mmol) was dissolved in THF (24.5 mL) and DMF, cooled to 0 °C, sodium hydride (110 mg, 60 % purity, 2.74 mmol) was added and stirred for 30 min at 0 °C. A solution of 5-bromo-3-fluoro-2-nitropyridine (551 mg, 2.49 mmol) in THF (0.5 mL) was added and the mixture was stirred for 2 h at 0 °C and over night at room temperature.The reaction mixture was quenched with saturated ammonium chloride solution and extracted with ethyl acetate. The combined organic layers were washed with brine, dried over sodium sulfate, filtered and concentrated to give 1.04 g (107 % yield) of the title compound.
LC-MS (Method 1): Rt = 1.45 min; MS (ESlpos): m/z = 393 [M+H]⁺
¹H-NMR (400 MHz, DMSO-d6) δ [ppm]: 1.152 (1.57), 1.170 (3.38), 1.188 (1.75), 1.226 (0.69), 1.350 (1.86), 1.447 (2.88), 1.464 (2.94), 1.722 (1.46), 1.739 (2.47), 1.744 (16.00), 1.754 (1.44), 1.761 (15.95), 1.986 (5.94), 2.518 (1.19), 2.522 (0.78), 3.998 (0.41), 4.015 (1.27), 4.033 (1.26), 4.051 (0.40), 6.353 (0.95), 6.370 (3.55), 6.386 (3.52), 6.403 (0.94), 7.235 (0.46), 7.253 (0.63), 7.256 (0.60), 7.275 (0.84), 7.319 (0.49), 7.370 (3.50), 7.385 (2.06), 7.389 (3.54), 7.392 (3.82), 7.404 (1.24), 7.411 (5.96), 7.419 (0.97), 7.439 (0.56), 7.508 (12.09), 7.528 (7.13), 7.770 (0.54), 7.779 (6.13), 7.784 (6.22), 7.979 (0.73), 7.984 (0.74), 8.077 (0.48), 8.262 (8.00), 8.267 (7.80).

### Intermediate 359

### 5-bromo-3-{[1-(3-{3-methyl-3-[(-oxan-2-yl)oxy]but-1-yn-1-yl}phenyl)ethyl]oxy}-2-nitropyridine (mixture of 4 isomers)

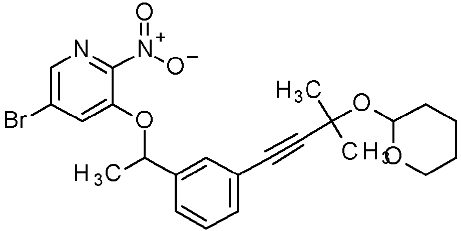

Sodium hydride (106 mg, 60 % purity, 2.65 mmol) was suspended in THF (5 mL) and cooled to 0 °C. 1-(3-{3-Methyl-3-[(oxan-2-yl)oxy]but-1-yn-1-yl}phenyl)ethan-1-ol (267 mg, 926 µmol) was dissolved in THF (1.5 mL) and added. The mixture was stirred for 30 min at 0 °C. A solution of 5-bromo-3-fluoro-2-nitropyridine (195 mg, 882 µmol) in THF (1.5 mL) was added and stirred for 3 h at 0 °C. The reaction mixture was quenched with saturated ammonium chloride solution and extracted with ethyl acetate. The combined organic layers were dried and concentrated. The residue was purified by column chromatography (silica gel, hexane / ethyl acetate (0 - 20 %) gradient) followed by column chromatography (silica gel NH2, hexane / ethyl acetate (0 - 20 %) gradient) to give 2.08 g (74 % yield) of the title compound.
LC-MS (Method 1): Rt = 1.60 min; MS (ESlpos): m/z = 489 [M+H]⁺
¹H-NMR (400 MHz, DMSO-d6) δ [ppm]: 1.084 (0.40), 1.154 (2.45), 1.172 (4.88), 1.190 (2.38), 1.232 (0.50), 1.259 (0.57), 1.406 (0.57), 1.425 (1.21), 1.435 (1.76), 1.448 (1.98), 1.464 (1.62), 1.475 (1.36), 1.485 (1.10), 1.513 (16.00), 1.545 (12.17), 1.558 (7.79), 1.574 (7.43), 1.606 (0.45), 1.615 (0.52), 1.628 (0.55), 1.642 (0.86), 1.659 (0.40), 1.671 (0.62), 1.710 (1.02), 1.720 (0.57), 1.729 (0.74), 1.739 (0.74), 1.749 (0.62), 1.759 (0.55), 1.987 (8.40), 2.318 (0.43), 2.518 (5.45), 2.523 (3.40), 2.660 (0.40), 3.419 (0.64), 3.435 (0.86), 3.447 (0.93), 3.463 (0.64), 3.826 (0.69), 3.834 (0.64), 3.843 (0.93), 3.853 (0.93), 3.862 (0.57), 3.872 (0.60), 4.000 (0.62), 4.017 (1.93), 4.035 (1.93), 4.053 (0.62), 5.074 (0.86), 5.080 (1.33), 5.085 (1.52), 5.092 (1.14), 5.098 (0.81), 5.940 (0.50), 5.956 (1.76), 5.972 (1.74), 5.988 (0.48), 7.347 (0.74), 7.352 (0.88), 7.354 (0.98), 7.357 (0.95), 7.364 (1.31), 7.370 (1.93), 7.374 (1.76), 7.389 (1.17), 7.407 (3.14), 7.417 (1.98), 7.423 (4.19), 7.440 (0.50), 7.487 (2.93), 8.248 (4.36), 8.252 (6.55), 8.268 (3.40).

### Intermediate 360

### (rac)-7-[(5-bromo-2-nitropyridin-3-yl)oxy]-6,7-dihydro-5H-cyclopenta[b]pyridine

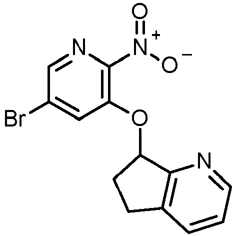

5-Bromo-2-nitropyridin-3-ol (100 mg, 457 µmol) was dissolved in THF (4.2 mL) under argon and (rac)-6,7-dihydro-5H-cyclopenta[b]pyridin-7-ol (61.7 mg, 457 µmol) and triphenylphosphine (934 mg, 3.5 mmol) were added and stirred for 1 h at room temperature. The reaction mixture was cooled to 0 to 4 °C, diisopropyl azodicarboxylate (120 µL, 590 µmol) was added and stirred for 1.5 h at 4 °C and over night at room temperature. The mixture was diluted with aqueous potassium carbonate solution and extracted with ethyl acetate. The organic phase was dried and concentrated. The residue was purified by column chromatography (silica gel, hexane / ethyl acetate (0 - 40 %) gradient). The combined fractions were concentrated and the residue was treated with MTBE. The crude product was filtered and the filtrate was concentrated to give 120 mg ( 78 % yield) of the title compound.
LC-MS (Method 1): Rt = 1.18 min; MS (ESlpos): m/z = 336 [M+H]⁺
¹H NMR (DMSO-d₆, 400 MHz): δ (ppm) 8.78 (d, 1H), 8.43 - 8.47 (m, 1H), 8.32 (d, 1H), 7.81 (dd, 1H), 7.36 (dd, 1H), 6.21 (dd, 1H), 3.01 - 3.12 (m, 1H), 2.87 - 2.96 (m, 1H), 2.56 - 2.69 (m, 1H), 2.17 (dddd, 1H).

### Intermediate 361

### (rac)-5-bromo-3-[1-(4,5-dimethyl-1,3-thiazol-2-yl)ethoxy]-2-nitropyridine

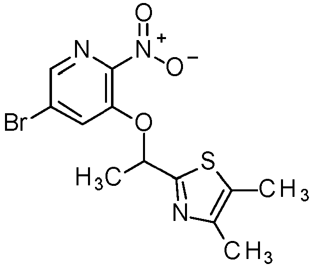

Sodium hydride (10.9 mg, 60 % purity, 272 µmol) was added to (rac)-1-(4,5-dimethyl-1,3-thiazol-2-yl)ethan-1-ol (36.6 mg, 233 µmol) in THF (2 mL) under argon at 0 °C. The mixture was stirred for 1 h at 0 °C. A solution of 5-bromo-3-fluoro-2-nitropyridine (50.0 mg, 226 µmol) in THF was added dropwise at 0 °C and stirred for 1.5 h at 0 °C. The reaction mixture was diluted with water and extracted with dichloromethane. The organic phase was dried over magnesium sulfate, filtered and concentrated. The residue was purified by preparative HPLC to give 42.0 mg (92 % purity, 48 % yield) of the title compound.
LC-MS (Method 1): Rt = 1.34 min; MS (ESlpos): m/z = 358 [M+H]⁺
¹H-NMR (400MHz, DMSO-d₆): δ [ppm]= 1.66 (d, 3H), 2.24 (s, 3H), 2.31 (s, 3H), 6.16 (q, 1H), 8.32 (d, 1H), 8.48 (d, 1H).

### Intermediate 362

### (rac)-5-bromo-3-[1-(5-methyl-1,3-thiazol-2-yl)ethoxy]-2-nitropyridine

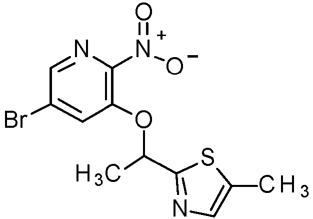

(Rac)-1-(5-Methyl-1,3-thiazol-2-yl)ethan-1-ol (167 mg, 1.17 mmol) was dissolved in THF (5 mL), cooled to 0 °C and sodium hydride (54.3 mg, 60 % purity, 1.36 mmol) was added. The mixture was stirred for 1 h at 0 °C. A solution of 5-bromo-3-fluoro-2-nitropyridine (250 mg, 1.13 mmol) in THF (5 mL) was added dropwise and stirred for 3 h at 0 °C. The reaction mixture was diluted with water and extracted with dichloromethane. The combined oranic layers were dried over magnesium sulfate, filtered and concentrated. The residue was purified by preparative HPLC to give 225 mg (100 % purity, 58 % yield) of the title compound.
LC-MS (Method 2): Rt = 1.27 min; MS (ESlpos): m/z = 344 [M+H]⁺
¹H-NMR (400MHz, DMSO-d₆): δ [ppm]= 1.68 (d, 3H), 2.43 (d, 3H), 6.21 (q, 1H), 7.50 (q, 1H), 8.32 (d, 1H), 8.48 (d, 1H).

### Intermediate 363

### (rac)-5-bromo-2-nitro-3-{[1-(1,3-thiazol-4-yl)ethyl]oxy}pyridine

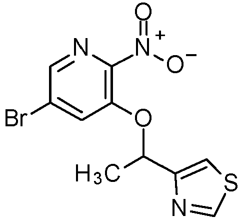

Sodium hydride (39.1 mg, 1.63 mmol, 60% in oil) was added to (rac)-1-(1,3-thiazol-4-yl)ethan-1-ol (181 mg, 1.40 mmol) in THF (4.5 mL) at 0 °C. The mixture was stirred for 1 h at 0 °C. A solution of 5-bromo-3-fluoro-2-nitropyridine (300 mg, 1.36 mmol) in THF (4.5 mL) was added dropwise at 0 °C and stirred over night and allwed to warm up to rt. (Rac)-1-(1,3-thiazol-4-yl)ethan-1-ol (90 mg, 0.70 mmol) was dissolved in THF, cooled to 0 °C, sodium hydride (19.5 mg, 0.81 mmol, 60% in oil) was added and stirred for 1 h at 0 °C. This reaction mixture was added to the first reaction mixture at 0 °C and stirred for 1 h at 0 °C. The reaction mixture was diluted with water and extracted with dichloromethane. The combined orgaic layers were washed with brine, dried over a hydrophobic filter and concentrated. The residue was purified by preparative HPLC to give 239 mg (100 % purity, 53 % yield) of the title compound.
LC-MS (Method 2): Rt = 1.13 min; MS (ESlpos): m/z = 330 [M+H]⁺
¹H-NMR (400MHz, DMSO-d₆): δ [ppm]= 1.67 (d, 3H), 6.10 (q, 1H), 7.80 (d, 1H), 8.27 (d, 1H), 8.44 (d, 1H), 9.12 (d, 1H).

### Intermediate 364

### 5-bromo-3-[(1R)-1-(5-methyl-1,3,4-oxadiazol-2-yl)ethoxy]-2-nitropyridine

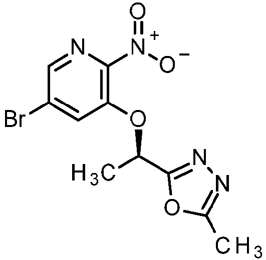

(1R)-1-(5-Methyl-1,3,4-oxadiazol-2-yl)ethan-1-ol (133 mg, 1.04 mmol) was dissolved in THF (4 mL), cooled to 0 °C and sodium hydride (27.5 mg, 1.15 mmol, 60% in oil) was added. The mixture was stirred for 1 h at 0 °C. A solution of 5-bromo-3-fluoro-2-nitropyridine (200 mg, 905 µmol) in THF (4 mL) was added dropwise at 0 °C and stirred for 17 h at 0°C. The reaction mixture was diluted with water and extracted with dichloromethane. The combined organic layers were dried over magnesium sulfate, filtered and concentrated. The residue was purified by preparative HPLC to give 167 mg (86 % purity, 48 % yield) of the title compound.
LC-MS (Method 2): Rt = 0.98 min; MS (ESlpos): m/z = 329 [M+H]⁺
¹H-NMR (400MHz, DMSO-d₆): δ [ppm]= 1.71 (d, 3H), 6.21 (q, 1H), 8.37 (d, 1H), 8.56 (d, 1H).

### Intermediate 365

### 5-bromo-3-[(1R)-1-(2-methyl-1,3-thiazol-4-yl)ethoxy]-2-nitropyridine

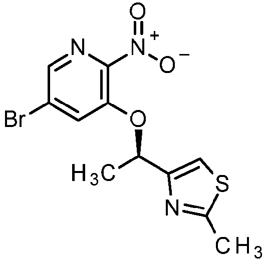

(1R)-1-(2-Methyl-1,3-thiazol-4-yl)ethan-1-ol (171 mg, 1.19 mmol) was dissolved in THF (7 mL), cooled to 0 °C and sodium hydride (47.8 mg, 1.99 mmol, 60% in oil) was added. The mixture was stirred for 1 h at 0 °C. A solution of 5-bromo-3-fluoro-2-nitropyridine (220 mg, 996 µmol) in THF (7 mL) was added dropwise at 0 °C and stirred over night to reach slowly rt. The reaction mixture was diluted with water and extracted with dichloromethane. The combined organic layers were dried over a hydrophobic filter and concentrated. The residue was purified by preparative HPLC to give 183 mg (98 % purity, 52 % yield) of the title compound.
LC-MS (Method 2): Rt = 1.23 min; MS (ESlpos): m/z = 344 [M+H]⁺
¹H-NMR (400MHz, DMSO-d₆): δ [ppm]= 1.63 (d, 3H), 2.63 (s, 3H), 5.98 (q, 1H), 7.55 (s, 1H), 8.27 (d, 1H), 8.43 (d, 1H).

### Intermediate 366

### (rac)-5-bromo-3-[1-(4-methyl-1,3-thiazol-2-yl)ethoxy]-2-nitropyridine

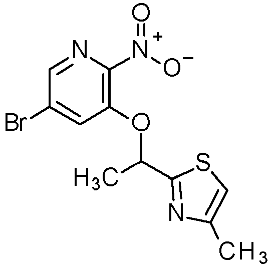

The compound was prepared similarly to **Intermediate 335** starting from (rac)-1-(4-methyl-1,3-thiazol-2-yl)ethan-1-ol (33.4 mg, 233 µmol) to give 48.0 mg (100 % purity, 62 % yield) of the title compound.
LC-MS (Method 2): Rt = 1.24 min; MS (ESlpos): m/z = 344 [M+H]⁺
¹H-NMR (400MHz, DMSO-d₆): δ [ppm]= 1.69 (d, 3H), 2.35 (d, 3H), 6.24 (q, 1H), 7.32 (q, 1H), 8.33 (d, 1H), 8.50 (d, 1H).

### Intermediate 367

### (rac)-5-bromo-3-{1-[3-(methanesulfonyl)phenyl]ethoxy}-2-nitropyridine

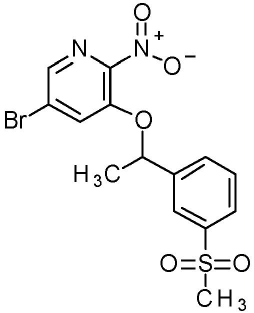

The compound was prepared similarly to **Intermediate 335** starting from (rac)-1-[3-(methanesulfonyl)phenyl]ethan-1-ol (800 mg, 3.99 mmol) to give 2.22 g (145 % yield) of the title compound.
LC-MS (Method 1): Rt = 1.13 min; MS (ESlneg): m/z = 399 [M-H]⁻
¹H-NMR (400 MHz, DMSO-d6) δ [ppm]: 1.150 (1.67), 1.168 (3.16), 1.187 (1.50), 1.344 (1.86), 1.350 (1.28), 1.361 (1.86), 1.612 (4.86), 1.627 (4.84), 1.639 (0.55), 1.656 (0.45), 1.735 (0.43), 1.751 (1.23), 1.985 (6.29), 2.153 (0.53), 3.204 (4.57), 3.222 (16.00), 3.228 (1.91), 3.239 (0.43), 3.594 (0.94), 3.611 (0.43), 3.996 (0.41), 4.014 (1.28), 4.032 (1.28), 4.050 (0.41), 6.102 (1.07), 6.118 (1.06), 7.594 (0.55), 7.679 (1.01), 7.698 (2.29), 7.718 (1.59), 7.768 (0.85), 7.771 (1.59), 7.774 (1.25), 7.787 (0.61), 7.791 (1.03), 7.794 (0.83), 7.883 (0.90), 7.886 (1.12), 7.888 (1.14), 7.891 (0.92), 7.903 (1.08), 7.907 (1.50), 7.910 (1.00), 8.033 (1.28), 8.038 (2.17), 8.042 (1.13), 8.079 (0.40), 8.260 (3.41), 8.265 (3.74), 8.357 (2.73), 8.362 (2.51), 8.678 (0.46).

### Intermediate 368

### (rac)-5-bromo-2-nitro-3-[1-(1,3-oxazol-4-yl)ethoxy]pyridine

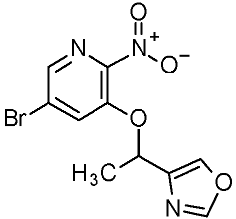

The compound was prepared similarly to **Intermediate 335** starting from (rac)-1-(1,3-oxazol-4-yl)ethan-1-ol (142 mg, 1.26 mmol) to give 194 mg (100 % purity, 51 % yield) of the title compound.
LC-MS (Method 2): Rt = 1.06 min; MS (ESlpos): m/z = 314 [M+H]⁺
¹H-NMR (400MHz, DMSO-d₆): δ [ppm]= 1.61 (d, 3H), 5.93 (q, 1H), 8.23 (s, 1H), 8.28 (d, 1H), 8.39 (d, 1H), 8.50 (d, 1H).

### Intermediate 369

### (rac)-5-bromo-2-nitro-3-[1-(1-propyl-1H-pyrazol-5-yl)ethoxy]pyridine

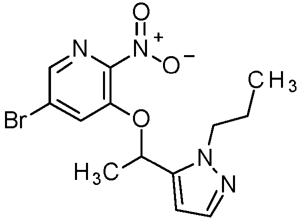

The compound was prepared similarly to **Intermediate 335** starting from (rac)-1-(1-propyl-1H-pyrazol-5-yl)ethan-1-ol (355 mg, 87 % purity, 2.00 mmol) to give 697 mg (103 % yield) of the title compound.
LC-MS (Method 1): Rt = 1.21 min; MS (ESlpos): m/z = 355 [M+H]⁺
¹H-NMR (400 MHz, DMSO-d6) δ [ppm]: 0.744 (2.16), 0.762 (5.00), 0.769 (6.73), 0.781 (2.91), 0.788 (16.00), 0.806 (7.55), 0.825 (3.23), 0.831 (0.68), 0.843 (6.31), 0.851 (0.82), 0.862 (3.23), 1.233 (1.60), 1.399 (5.68), 1.414 (6.24), 1.619 (11.29), 1.635 (11.27), 1.657 (3.68), 1.672 (3.57), 1.683 (0.75), 1.702 (2.05), 1.720 (3.20), 1.738 (3.05), 1.752 (2.66), 1.756 (1.71), 1.770 (2.20), 1.789 (1.03), 2.518 (3.43), 2.523 (2.56), 3.990 (0.57), 3.999 (0.46), 4.009 (0.71), 4.019 (3.77), 4.025 (1.25), 4.038 (5.33), 4.056 (4.90), 4.075 (0.82), 4.090 (0.43), 4.784 (0.51), 4.799 (0.74), 4.815 (0.52), 5.231 (1.60), 5.246 (1.52), 5.758 (1.63), 6.110 (1.63), 6.114 (1.65), 6.152 (0.65), 6.167 (2.23), 6.183 (2.22), 6.199 (0.65), 6.364 (5.57), 6.368 (6.01), 6.382 (2.51), 6.386 (1.85), 7.293 (1.71), 7.297 (1.71), 7.395 (1.51), 7.400 (1.42), 7.428 (5.34), 7.433 (5.44), 8.123 (0.92), 8.129 (1.08), 8.148 (0.77), 8.153 (1.42), 8.163 (2.99), 8.169 (1.88), 8.314 (7.08), 8.318 (7.38), 8.478 (4.99), 8.482 (4.74), 8.678 (1.00), 8.682 (1.17), 8.737 (0.95), 8.742 (0.79), 8.763 (0.83), 8.767 (0.85).

### Intermediate 370

### (rac)-4-{1-[(5-bromo-2-nitropyridin-3-yl)oxy]ethyl}pyrimidine

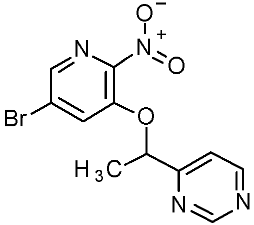

The compound was prepared similarly to **Intermediate 335** starting from (rac)-1-(pyrimidin-4-yl)ethan-1-ol (590 mg, 4.75 mmol) to give 1.13 g (77 % yield) of the title compound.
LC-MS (Method 1): Rt = 1.01 min; MS (ESlpos): m/z = 325 [M+H]⁺
¹H NMR (400 MHz, DMSO-*d*₆) δ ppm 1.63 (d, 3 H) 5.97 (q, 1 H) 7.60 (dd, 1 H) 8.32 (d, 1 H), 8.34 (d, 1 H), 8.88 (d, 1 H) 9.20 (d, 1 H).

### Intermediate 371

### (rac)-5-{1-[(5-bromo-2-nitropyridin-3-yl)oxy]ethyl}-2,4-dimethylpyrimidine

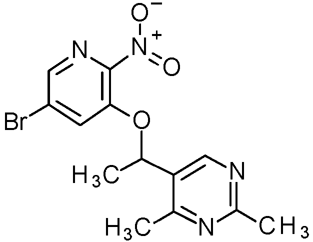

The compound was prepared similarly to **Intermediate 335** starting from (rac)-1-(2,4-dimethylpyrimidin-5-yl)ethan-1-ol (434 mg, 2.85 mmol)) to give 860 mg (90 % yield) of the title compound.
LC-MS (Method 1): Rt = 1.04 min; MS (ESlpos): m/z = 353 [M+H]⁺
¹H NMR (DMSO-d₆, 400 MHz): δ (ppm) 8.60 (s, 1H), 8.41 (d, 1H), 8.31 (d, 1H), 6.12 (q, 1H), 2.56 (s, 3H), 2.52 (s, 3H), 1.60 (d, 3H).

### Intermediate 372 and Intermediate 373

### 5-{1-[(5-bromo-2-nitropyridin-3-yl)oxy]ethyl}-2,4-dimethylpyrimidine (enantiomer 1 and enantiomer 2)

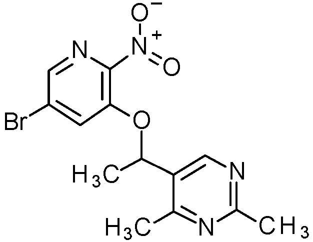

The title compound from **Intermediate 371** was separated into enantiomers by preparative chiral HPLC to give 426.8 mg (99 % purity, 42 % yield) of the title compound (enantiomer 1) and 413.3 mg (99 % purity, 41 % yield) of the title compound (enantiomer 2).

### Preparative chiral HPLC method:

Instrument: Sepiatec: Prep SFC100; Column: Chiralpak IG 5µ, 250x50; eluent A: CO2; eluent B: 2-propanol + 0.4 vol % diethylamine; isocratic: 10%B; flow: 100 mL/min; temperature: 40 °C; BPR: 150 bar; UV: 254 nm

### Analytical chiral HPLC method:

Instrument: Agilent: 1260, Aurora SFC-Modul; Column: Chiralpak IG 5µ, 100x4.6; eluent A: CO2; eluent B: 2-propanol + 0.4 vol % diethylamine; isocratic: 10%B; flow: 4 mL/min; temperature: 37.5 °C; BPR: 100 bar; UV: 254 nm

### Enantiomer 1:

Retention time preparative method : 9.25 - 11.50 min
Retention time analytical method: 3.07 min
[α]²⁰_{D}: -104.2° (c = 1.00, methanol)
¹H NMR (DMSO-d₆, 400 MHz): δ (ppm) 8.60 (s, 1H), 8.41 (d, 1H), 8.31 (d, 1H), 6.12 (q, 1H), 2.55 (s, 3H), 2.52 (s, 3H), 1.60 (d, 3H).

### Enantiomer 2:

Retention time preparative method : 12.75 - 17.00 min
Retention time analytical method: 4.30 min
[α]²⁰_{D}: +94.5° (c = 1.00, methanol)
¹H NMR (DMSO-d₆, 400 MHz): δ (ppm) 8.60 (s, 1H), 8.41 (d, 1H), 8.31 (d, 1H), 6.12 (q, 1H), 2.55 (s, 3H), 2.52 (s, 3H), 1.60 (d, 3H).

### Intermediate 374

### (rac)-5-bromo-3-{1-[4-(methanesulfonyl)phenyl]ethoxy}-2-nitropyridine

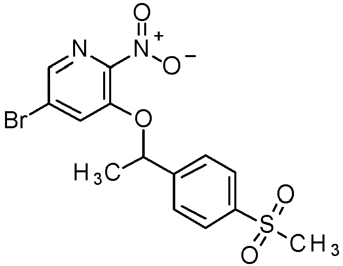

The compound was prepared similarly to Intermediate 335 starting from (rac)-1-[4-(methanesulfonyl)phenyl]ethan-1-ol (237.8 mg, 1.18 mmol) to give 503 mg (110 % yield) of the title compound.
LC-MS (Method 1): Rt = 1.13 min; MS (ESlneg): m/z = 399 [M-H]⁻
¹H-NMR (400 MHz, DMSO-d6) δ [ppm]: 1.153 (0.46), 1.171 (1.02), 1.189 (0.57), 1.229 (0.68), 1.299 (5.02), 1.315 (5.26), 1.352 (3.07), 1.565 (16.00), 1.581 (15.95), 1.595 (3.88), 1.611 (3.83), 1.986 (1.70), 2.518 (1.53), 2.523 (1.07), 5.936 (0.95), 5.952 (3.24), 5.968 (3.21), 5.984 (0.91), 6.183 (0.69), 6.199 (0.68), 7.200 (0.56), 7.217 (0.50), 7.274 (0.79), 7.279 (0.78), 7.288 (0.56), 7.292 (1.17), 7.295 (2.03), 7.299 (2.42), 7.305 (1.07), 7.312 (3.09), 7.318 (2.52), 7.320 (1.92), 7.325 (3.26), 7.329 (3.97), 7.334 (2.23), 7.340 (0.67), 7.346 (1.06), 7.350 (1.85), 7.363 (0.43), 7.368 (1.59), 7.372 (3.25), 7.376 (1.59), 7.388 (2.70), 7.391 (8.17), 7.404 (2.96), 7.409 (9.17), 7.414 (8.49), 7.418 (10.51), 7.429 (1.29), 7.435 (3.01), 7.440 (2.06), 8.066 (1.33), 8.071 (2.64), 8.075 (1.65), 8.080 (0.51), 8.100 (1.02), 8.105 (0.89), 8.222 (5.12), 8.226 (14.87), 8.230 (11.55), 8.235 (3.96), 8.240 (0.44).

### Intermediate 375

### (rac)-5-bromo-3-[1-(1-methyl-1H-1,2,3-triazol-4-yl)ethoxy]-2-nitropyridine

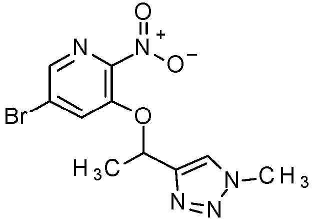

The compound was prepared similarly to **Intermediate 335** starting from (rac)-1-(1-methyl-1H-1,2,3-triazol-4-yl)ethan-1-ol (138 mg, 1.09 mmol) to yield 319 mg (107 % yield) of the title product which was used without further purification in the next step.
LC-MS (Method 2): Rt = 0.99 min; MS (ESlpos): m/z = 328 [M+H]⁺
¹H-NMR (400MHz, DMSO-d6): δ [ppm]= 1.66 (d, 3H), 4.03 (s, 3H), 6.08 (q, 1H), 8.20 (s, 1H), 8.27 (d, 1H), 8.52 (d, 1H).

### Reduction + Chiral sep

### Intermediate 376 and Intermediate 377

### 5-bromo-3-[1-(6-methylpyridin-2-yl)ethoxy]pyridin-2-amine (enantiomer 1 and enantiomer 2)

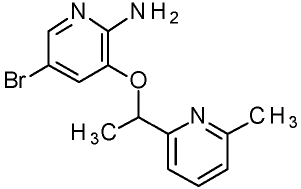

To a suspension of (rac)-5-bromo-3-{[-1-(6-methylpyridin-2-yl)ethyl]oxy}-2-nitropyridine (**Intermediate 325,** 440 mg, 65 % purity, 846 µmol) in ethanol (4.5 mL) under argon were added iron (189 mg, 3.38 mmol) and acetic acid (2.3 mL). The mixture was stirred for 3 h at 80°C. The reaction mixture was then cooled to rt, diluted with dichloromethane and filtered. The filtrate was washed with 2M hydrochloric acid. The aqueous layer was extracted with dichloromethane, basified with 2M sodium hydroxide solution and extracted with ethyl acetate. The organic layer was washed with sat. sodium chloride solution, dried over sodium sulfate, filtered and concentrated under reduced pressure. The crude material was purified by flash column chromatography (silica gel, hexane / ethyl acetate (0-80%) gradient) and chiral HPLC to give the title compounds.
LC-MS (Method 1): Rt = 1.05 min; MS (ESlpos): m/z = 308 [M+H]+
¹H-NMR (400MHz, CHLOROFORM-d): δ [ppm]= 1.70 (d, 3H), 2.58 (s, 3H), 4.82 (br s, 2H), 5.30 (q, 1H), 6.90 (d, 1H), 7.09 (dd, 2H), 7.56 (t, 1H), 7.66 (d, 1H).

### Chiral separation:

### Preparative

Instrument: Sepiatec: Prep SFC100; Column: Chiralpak IG 5µ 250x30mm; eluent A: CO2; eluent B: methanol + 0.2 vol % aqueous ammonia (32%); isocratic: 10% B; flow: 100 mL/min; temperature: 40 °C; BPR: 150 bar; UV: 254 nm

### Analytical

Instrument: Waters Acquity UPLCMS SingleQuad; Column: Acquity UPLC BEH C18 1.7µ, 50x2.1mm; eluent A: water + 0.2 vol % aqueous ammonia (32%); eluent B: acetonitrile; gradient: 0-1.6 min 1-99% B, 1.6-2.0 min 99% B; flow: 0.8 mL/min; temperature: 60 °C; DAD scan: 210-400 nm

### Enantiomer 1:

98 mg (37 % yield)
Retention time preparative method : 7.5 - 10.5 min
Retention time analytical method: 2.35 min
[α]²⁰_{D}: +38.9° (c = 1.00, MeOH)

### Enantiomer 2:

106 mg (38 % yield)
Retention time preparative method : 11.5 - 15.0 min
Retention time analytical method: 3.26 min
[α]²⁰_{D}: -32.0° (c = 1.00, MeOH)

### Intermediate 378 and Intermediate 379

### 5-bromo-3-[1-(5-methylpyridin-2-yl)ethoxy]pyridin-2-amine (enantiomer 1 and enantiomer 2)

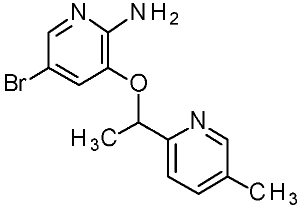

The title compounds were prepared similarly to **Intermediate 376 and Intermediate 377** starting from (rac)-5-bromo-3-{[1-(5-methylpyridin-2-yl)ethyl]oxy}-2-nitropyridine (490 mg, 70 % purity, 1.01 mmol).
LC-MS (Method 1): Rt = 1.05 min; MS (ESlpos): m/z = 308 [M+H]⁺
¹H-NMR (400MHz, CHLOROFORM-d): δ [ppm]= 1.69 (d, 3H), 2.33 (s, 3H), 4.80 (br s, 2H), 5.32 (q, 1H), 6.88 (d, 1H), 7.21 (d, 1H), 7.48 (dd, 1H), 7.65 (d, 1H), 8.40 - 8.42 (m, 1H).

### Chiral separation:

### Preparative

Instrument: PrepCon Labomatic HPLC-1; Column: YMC Cellulose SB 5µ, 250x30; eluent A: hexane + 0.1 vol % diethylamine; eluent B: ethanol + 0.1 vol % diethylamine; isocratic: 95%A+5%B; flow: 50 mL/min; temperature: 25 °C; UV: 254 nm

### Analytical

Instrument: Thermo Fisher UltiMate 3000; Column: YMC Cellulose SB 3µ, 100x4.6; eluent A: hexane + 0.1 vol % diethylamine; eluent B: ethanol; isocratic: 95%A+5%B; flow: 1.4 mL/min; temperature: 25 °C; UV: 254 nm

### Enantiomer 1:

75.9 mg (24 % yield)
Retention time preparative method: 8.3 - 9.0 min
Retention time analytical method: 2.42 min
[α]²⁰_{D}: -37.1° (c = 1.00, MeOH)

### Enantiomer 2:

80.2 mg (25 % yield)
Retention time preparative method: 9.4 - 10.3 min
Retention time analytical method: 2.75 min
[α]²⁰_{D}: +40.5° (c = 1.00, MeOH)

### Intermediate 380 and Intermediate 381

### 5-bromo-3-[1-(4-methylpyridin-2-yl)ethoxy]pyridin-2-amine (enantiomer 1 and enantiomer 2)

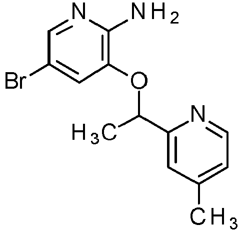

The title compounds were prepared similarly to **Intermediate 376 and Intermediate 377** starting from (rac)-5-bromo-3-{[1-(4-methylpyridin-2-yl)ethyl]oxy}-2-nitropyridine (466 mg, 57 % purity, 785 µmol).
LC-MS (Method 1): Rt = 1.03 min; MS (ESlpos): m/z = 308 [M+H]⁺
¹H-NMR (400MHz, CHLOROFORM-d): δ [ppm]= 1.69 (d, 3H), 2.33 (s, 3H), 4.81 (br s, 2H), 5.30 (q, 1H), 6.88 (d, 1H), 7.03 - 7.06 (m, 1H), 7.13 (s, 1H), 7.65 - 7.69 (m, 1H), 8.44 (d, 1H).

### Chiral separation:

### Preparative

Instrument: PrepCon Labomatic HPLC-1; Column: YMC Cellulose SB 5µ, 250x30; eluent A: hexane + 0.1 vol % diethylamine; eluent B: ethanol + 0.1 vol % diethylamine; isocratic: 95%A+5%B; flow: 50 mL/min; temperature: 25 °C; UV: 254 nm

### Analytical

Instrument: Thermo Fisher UltiMate 3000; Column: YMC Cellulose SB 3µ, 100x4.6; eluent A: hexane + 0.1 vol % diethylamine; eluent B: ethanol; isocratic: 95%A+5%B; flow: 1.4 mL/min; temperature: 25 °C; UV: 254 nm

### Enantiomer 1:

54.7 mg (22 % yield)
(Retention time preparative method: 8.5 - 9.3 min
Retention time analytical method: 2.46 min
[α]²⁰_{D}: -34.1° (c = 1.00, MeOH)

### Enantiomer 2:

(53.6 mg (21 % yield)
Retention time preparative method: 9.5 - 10.2 min
Retention time analytical method: 2.76 min
[α]²⁰_{D}: +35.7° (c = 1.00, MeOH)

### Intermediate 382 and Intermediate 383

### 5-bromo-3-[1-(3-methylpyridin-2-yl)ethoxy]pyridin-2-amine (enantiomer 1 and enantiomer 2)

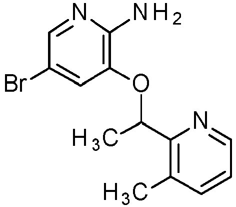

The title compounds were prepared similarly to **Intermediate 376 and Intermediate 377** starting from (rac)- -bromo-3-{[1-(3-methylpyridin-2-yl)ethyl]oxy}-2-nitropyridine (240 mg, 74 % purity, 525 µmol)
LC-MS (Method 1): Rt = 1.02 min; MS (ESlpos): m/z = 308 [M+H]⁺
¹H-NMR (400MHz, CHLOROFORM-d): δ [ppm]= 1.74 (d, 3H), 2.38 (s, 3H), 4.79 (br s, 2H), 5.51 (q, 1H), 6.86 (d, 1H), 7.16 (dd, 1H), 7.45 - 7.49 (m, 1H), 7.66 (d, 1H), 8.46 (dd, 1H).

### Chrial separation:

### Preparative

Instrument: PrepCon Labomatic HPLC-1; Column: YMC Cellulose SB 5µ, 250x30; eluent A: hexane + 0.1 vol % diethylamine; eluent B: ethanol + 0.1 vol % diethylamine; isocratic: 95%A+5%B; flow: 50 mL/min; temperature: 25 °C; UV: 254 nm

### Analytical

Instrument: Thermo Fisher UltiMate 3000; Column: YMC Cellulose SB 3µ, 100x4.6; eluent A: hexane + 0.1 vol % diethylamine; eluent B: ethanol; isocratic: 95%A+5%B; flow: 1.4 mL/min; temperature: 25 °C; UV: 254 nm

### Enantiomer 1:

34.4 mg (21 % yield)
Retention time preparative method: 9.0 - 10.2 min
Retention time analytical method: 2.72 min
[α]²⁰_{D}: +4.3° (c = 1.00, MeOH)

### Enantiomer 2:

(34.9 mg (21 % yield)
Retention time preparative method: 10.4 - 11.6 min
Retention time analytical method: 3.18 min
[α]²⁰_{D}: -3.4° (c = 1.00, MeOH)

### Intermediate 384 and Intermediate 385

### 5-bromo-3-[1-(1,4-dimethyl-1H-pyrazol-5-yl)ethoxy]pyridin-2-amine (enantiomer 1 and enantiomer 2)

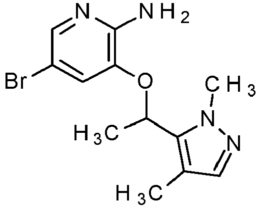

The title compounds were prepared similarly to **Intermediate 376 and Intermediate 377** starting from (rac)-5-bromo-3-[1-(1,4-dimethyl-1H-pyrazol-5-yl)ethoxy]-2-nitropyridine (452 mg, 1.32 mmol)
LC-MS (Method 1): Rt = 0.97 min; MS (ESlneg): m/z = 309 [M-H]⁻
¹H-NMR (400MHz, CHLOROFORM-d): δ [ppm]= 1.73 (d, 3H), 2.11 (s, 3H), 3.89 (s, 3H), 4.68 (br s, 2H), 5.38 (d, 1H), 6.77 (d, 1H), 7.25 (s, 1H), 7.70 (d, 1H).

### Chrial separation:

### Preparative

Instrument: Sepiatec: Prep SFC100; Column: Chiralpak IG 5µ 250x30mm; eluent A: CO2; eluent B: ethanol + 0.2 vol % aqueous ammonia (32%); isocratic: 10%B; flow: 100 mL/min; temperature: 40 °C; BPR: 150 bar; UV: 254 nm;

### Analytical

Instrument: Agilent: 1260, Aurora SFC-Modul; Column: Chiralpak IG 5µ 100x4.6mm; eluent A: CO2; eluent B: ethanol + 0.2 vol % aqueous ammonia (32%); isocratic: 10%B; flow: 4 mL/min; temperature: 37.5 °C; BPR: 100 bar; UV: 254 nm;

### Enantiomer 1:

71.7 mg (32 % yield)
Retention time preparative method: 11.00 - 14.50 min
Retention time analytical method: 3.55 min
[α]²⁰_{D}: +16.8° (c = 1.00, MeOH)

### Enantiomer 2:

64.8 mg (29 %, yield)
Retention time preparative method: 14.50 - 19.50 min
Retention time analytical method: 4.52 min
[α]²⁰_{D}: -17.1° (c = 1.00, MeOH)

### Intermediate 386

### 5-bromo-3-[(1R)-1-(3-fluoropyridin-2-yl)ethoxy]pyridin-2-amine

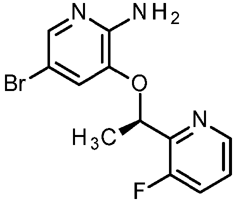

To a suspension of 5-bromo-3-[(1R)-1-(3-fluoropyridin-2-yl)ethoxy]-2-nitropyridine **(Intermediate 332,** 370 mg, 65 % purity, 703 µmol) in ethanol (3.8 mL) under argon were added iron (157 mg, 2.81 mmol) and acetic acid (1.9 mL). The mixture was stirred for 3 h at 80 °C. The cold reaction mixture was diluted with dichloromethane, filtered and washed with dichloromethane. The filtrate was extracted twice with 2M NaOH-solution and filtered over celite. The organic layer was washed with brine, dried over sodium sulfate, filtered and concentrated under reduced pressure. The residue was purified by column chromatography (silica gel, hexane / ethyl acetate (0-90%) gradient) to give 197 mg (95 % purity, 85 % yield) of the title compound.
LC-MS (Method 1): Rt = 1.01 min; MS (ESlpos): m/z = 312 [M+H]⁺
¹H-NMR (400MHz, CHLOROFORM-d): δ [ppm]= 1.77 (d, 3H), 4.80 (br s, 2H), 5.63 (q, 1H), 7.02 (d, 1H), 7.29 (dt, 1H), 7.39 - 7.46 (m, 1H), 7.67 (d, 1H), 8.43 (dt, 1H).

### Intermediate 387

### 5-bromo-3-[(1R)-1-(5-fluoropyridin-2-yl)ethoxy]pyridin-2-amine

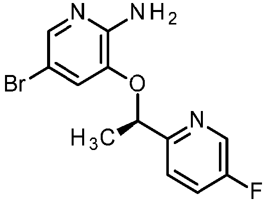

The compound was prepared similarly to **Intermediate 386** starting from 5-bromo-3-[(1R)-1-(5-fluoropyridin-2-yl)ethoxy]-2-nitropyridine (**Intermediate 331**, 370 mg, 75 % purity, 811 µmol) to give 200 mg (95 % purity, 75 % yield) of the title compound.
LC-MS (Method 1): Rt = 1.04 min; MS (ESlpos): m/z = 312 [M+H]⁺
¹H-NMR (400MHz, CHLOROFORM-d): δ [ppm]= 1.70 (d, 3H), 4.78 (br s, 2H), 5.36 (q, 1H), 6.86 (d, 1H), 7.31 - 7.46 (m, 2H), 7.68 (d, 1H), 8.45 (d, 1H).

### Intermediate 388

### 5-bromo-3-[(1S)-1-(2,6-difluorophenyl)ethoxy]pyridin-2-amine

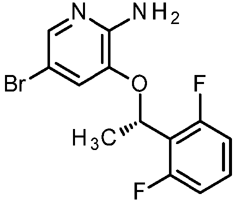

To a suspension of 5-bromo-3-[(1S)-1-(2,6-difluorophenyl)ethoxy]-2-nitropyridine **(Intermediate 330,** 364 mg, 80 % purity, 811 µmol) in ethanol (4.3 mL) under argon were added iron (181 mg, 3.24 mmol]) and acetic acid (2.2 mL). The mixture was stirred for 3 h at 80 °C. The cold reaction mixture was diluted with dichloromethane, filtered and washed with dichloromethane. The filtrate was extracted twice with 2M NaOH-solution and filtered over celite. The organic layer was washed with brine, dried over sodium sulfate, filtered and concentrated under reduced pressure. The residue was purified by column chromatography (silica gel, hexane / ethyl acetate (0-50%) gradient) to give 233 mg (95 % purity, 83 % yield) of the title compound.
LC-MS (Method 1): Rt = 1.23 min; MS (ESlpos): m/z = 329 [M+H]⁺
¹H-NMR (400 MHz, CHLOROFORM-d) δ [ppm]: 0.008 (0.50), 1.269 (0.51), 1.635 (0.51), 1.651 (0.46), 1.799 (15.80), 1.816 (16.00), 2.056 (0.71), 4.744 (2.92), 5.698 (1.00), 5.715 (3.06), 5.731 (3.02), 5.748 (0.96), 6.872 (0.45), 6.875 (0.64), 6.884 (4.85), 6.892 (0.62), 6.896 (0.94), 6.904 (9.74), 6.914 (0.75), 6.917 (0.67), 6.926 (5.49), 6.934 (0.65), 6.938 (0.44), 7.017 (5.91), 7.021 (6.01), 7.235 (1.18), 7.250 (2.39), 7.255 (1.71), 7.266 (1.96), 7.276 (1.49), 7.287 (1.63), 7.292 (2.09), 7.308 (1.03), 7.672 (8.09), 7.677 (8.36).

### Intermediate 389

### 5-bromo-3-[(1R)-1-(2,6-difluorophenyl)ethoxy]pyridin-2-amine

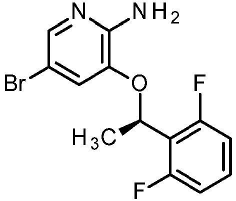

The compound was prepared similarly to **Intermediate 386** starting from 5-bromo-3-[(1R)-1-(2,6-difluorophenyl)ethoxy]-2-nitropyridine **(Intermediate 333,** 322 mg, 80 % purity, 717 µmol) to give 208 mg (88 % yield) of the title compound.
LC-MS (Method 1): Rt = 1.22 min; MS (ESlpos): m/z = 329 [M+H]⁺
¹H-NMR (400 MHz, CHLOROFORM-d) δ [ppm]: 1.269 (0.49), 1.799 (15.91), 1.815 (16.00), 2.055 (0.80), 4.744 (2.96), 5.698 (0.99), 5.715 (3.05), 5.731 (3.03), 5.748 (0.97), 6.875 (0.59), 6.884 (4.52), 6.892 (0.61), 6.896 (0.83), 6.904 (9.91), 6.913 (0.74), 6.917 (0.62), 6.925 (5.13), 6.934 (0.66), 6.938 (0.44), 7.017 (5.98), 7.021 (5.98), 7.234 (1.16), 7.250 (2.39), 7.255 (1.64), 7.266 (1.72), 7.276 (1.27), 7.287 (1.67), 7.292 (1.83), 7.308 (0.90), 7.672 (8.53), 7.677 (7.95).

### Intermediate 390

### (rac)-2-{1-[(2-amino-5-bromopyridin-3-yl)oxy]ethyl}benzonitrile

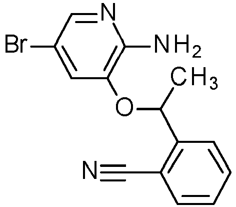

(Rac)-2-{1-[(5-Bromo-2-nitropyridin-3-yl)oxy]ethyl}benzonitrile **(Intermediate 334,** 333 mg, 83 % purity, 793 µmol) was dissolved in ethanol (2.3 mL). Acetic acid (1.6 mL) and iron (122 mg, 2.18 mmol) were added and the mixture was stirred for 2 h at 85 °C. The reaction mixture was diluted with ethyl acetate, filtered through celite, basified with 2M sodium hydroxide solution and extracted 2 times with ethyl acetate. The combined organic layers were dried over a hydrophobic filter and concentrated. The residue was purified by column chromatography (silica gel, dichlormethan / methanol (0-5%) gradient) to give 142 mg (95 % purity, 53 % yield) of the title compound.
LC-MS (Method 1): Rt = 1.10 min; MS (ESlpos): m/z = 318 [M+H]⁺
¹H-NMR (400 MHz, DMSO-d6) δ [ppm]: 0.967 (0.52), 0.984 (1.18), 1.002 (0.57), 1.232 (1.14), 1.633 (2.36), 1.641 (15.85), 1.657 (16.00), 2.518 (2.20), 2.523 (1.42), 3.159 (0.52), 3.172 (0.42), 5.735 (0.98), 5.751 (3.59), 5.767 (3.56), 5.782 (0.97), 6.083 (6.74), 7.077 (6.13), 7.081 (6.22), 7.496 (1.96), 7.502 (2.12), 7.513 (2.36), 7.516 (2.66), 7.518 (2.77), 7.521 (2.43), 7.532 (2.27), 7.537 (2.62), 7.558 (10.37), 7.563 (10.02), 7.623 (0.41), 7.627 (0.42), 7.714 (1.15), 7.717 (1.25), 7.733 (3.62), 7.737 (3.92), 7.750 (8.08), 7.754 (8.19), 7.769 (1.23), 7.774 (0.52), 7.830 (1.09), 7.868 (3.66), 7.870 (4.03), 7.887 (2.90), 7.889 (3.99).

### Intermediate 391

### 3-{(1R)-1-[(2-amino-5-bromopyridin-3-yl)oxy]ethyl}benzonitrile

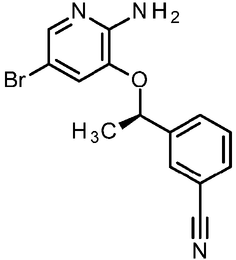

To a suspension of 3-{(1R)-1-[(5-bromo-2-nitropyridin-3-yl)oxy]ethyl}benzonitrile **(Intermediate 335,** 371 mg, 50 % purity, 533 µmol) in ethanol (2.8 mL) under argon were added iron (119 mg, 2.13 mmol) and acetic acid (1.4 mL). The mixture was stirred for 3 h at 80 °C. The cold reaction mixture was diluted with dichloromethane, filtered and washed with dichloromethane. The filtrate was extracted twice with 2M NaOH-solution and filtered over celite. The organic layer was washed with brine, dried over sodium sulfate, filtered and concentrated under reduced pressure. The residue was purified by column chromatography (silica gel, hexane / ethyl acetate (0-90%) gradient) to give 135 mg (95 % purity, 75 % yield) of the title compound.
LC-MS (Method 1): Rt = 1.10 min; MS (ESlpos): m/z = 318 [M+H]⁺
¹H-NMR (400 MHz, CHLOROFORM-d) δ [ppm]: 1.250 (2.23), 1.268 (4.18), 1.286 (1.96), 1.676 (16.00), 1.692 (15.62), 2.055 (8.07), 4.103 (0.55), 4.121 (1.62), 4.138 (1.52), 4.157 (0.53), 4.766 (2.60), 5.283 (0.77), 5.299 (2.44), 5.315 (2.39), 5.331 (0.75), 6.771 (4.42), 6.775 (4.41), 7.494 (1.24), 7.513 (3.54), 7.531 (2.89), 7.571 (1.45), 7.575 (3.02), 7.578 (2.03), 7.591 (0.96), 7.594 (1.75), 7.616 (1.83), 7.620 (3.54), 7.623 (1.95), 7.634 (0.96), 7.638 (2.93), 7.642 (4.30), 7.647 (4.00), 7.651 (1.73), 7.708 (6.10), 7.712 (5.73).

### Intermediate 392

### 4-{(1R)-1-[(2-amino-5-bromopyridin-3-yl)oxy]ethyl}benzonitrile

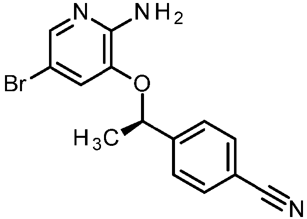

To a suspension of 4-{(1R)-1-[(5-bromo-2-nitropyridin-3-yl)oxy]ethyl}benzonitrile **(Intermediate 336,** 363 mg, 45 % purity, 469 µmol) in ethanol (2.5 mL) under argon were added iron (105 mg, 1.88 mmol) and acetic acid (1.2 mL). The mixture was stirred for 3 h at 80 °C. The cold reaction mixture was diluted with dichloromethane, filtered and washed with dichloromethane. The filtrate was extracted twice with 2M NaOH-solution and filtered over celite. The organic layer was washed with brine, dried over sodium sulfate, filtered and concentrated under reduced pressure. The residue was purified by column chromatography (silica gel, hexane / ethyl acetate (0-90%) gradient) to give 120 mg (95 % purity, 76 % yield) of the title compound.
LC-MS (Method 1): Rt = 1.09 min; MS (ESlpos): m/z = 318 [M+H]⁺
¹H-NMR (400 MHz, CHLOROFORM-d) δ [ppm]: 1.251 (2.27), 1.268 (4.58), 1.286 (2.13), 1.676 (16.00), 1.693 (15.06), 2.055 (8.01), 4.104 (0.56), 4.121 (1.73), 4.139 (1.69), 4.157 (0.53), 4.760 (2.79), 5.297 (0.79), 5.314 (2.43), 5.330 (2.44), 5.345 (0.73), 6.748 (4.52), 6.753 (4.59), 7.447 (6.20), 7.450 (1.99), 7.464 (2.21), 7.468 (7.44), 7.676 (1.33), 7.680 (8.26), 7.684 (2.55), 7.696 (2.78), 7.700 (10.69), 7.705 (6.74).

### Intermediate 393

### 5-bromo-3-[(1R)-1-(3,5-difluoropyridin-4-yl)ethoxy]pyridin-2-amine

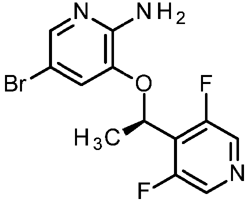

To a suspension of 5-bromo-3-[(1R)-1-(3,5-difluoropyridin-4-yl)ethoxy]-2-nitropyridine **(Intermediate 337,** 366 mg, 60 % purity, 610 µmol) in ethanol (3.3 mL) under argon were added iron (136 mg, 2.44 mmol) and acetic acid (1.6 mL). The mixture was stirred for 3 h at 80 °C. The cold reaction mixture was diluted with dichloromethane, filtered and washed with dichloromethane. The filtrate was extracted twice with 2M NaOH-solution and filtered over celite. The organic layer was washed with brine, dried over sodium sulfate, filtered and concentrated under reduced pressure. The residue was purified by column chromatography (silica gel, hexane / ethyl acetate (0-90%) gradient) to give 166 mg (95 % purity, 78 % yield) of the title compound.
LC-MS (Method 1): Rt = 1.04 min; MS (ESlpos): m/z = 330 [M+H]⁺
¹H-NMR (400MHz, CHLOROFORM-d): δ [ppm]= 1.81 (d, 3H), 4.74 (br s, 2H), 5.67 - 5.78 (m, 1H), 6.97 (d, 1H), 7.71 (d, 1H), 8.36 (s, 2H).

### Intermediate 394

### (rac)-5-bromo-3-(1-phenylpropoxy)pyridin-2-amine (enantiomer 1 and enantiomer 2)

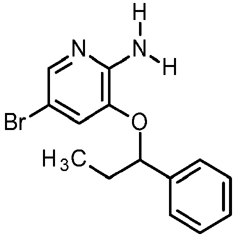

(Rac)-5-bromo-2-nitro-3-(1-phenylpropoxy)pyridine **(Intermediate 338,** 324 mg, 962 µmol) was dissolved in ethanol (2.8 mL). Iron (148 mg, 2.64 mmol) and acetic acid (1.9 mL) were added and the mixture was stirred for 1 h at 85 °C. The reaction mixture was tempered to rt, basified with 2M NaOH-solution, diluted with ethyl acetate and filtered over celite. The aqueous layer was extracted 2 times with ethyl acetate. The combined organic layers were washed with brine, dried over a hydrophobic filter and concentrated to give 320 mg (90 % purity, 97 % yield) of the title compound.
LC-MS (Method 1): Rt = 1.28 min; MS (ESlpos): m/z = 307 [M+H]⁺
¹H-NMR (400 MHz, DMSO-d6) δ [ppm]: 0.807 (0.44), 0.851 (0.61), 0.863 (1.19), 0.882 (2.72), 0.889 (6.62), 0.901 (2.12), 0.907 (16.00), 0.926 (7.02), 1.233 (1.62), 1.352 (0.68), 1.761 (0.80), 1.775 (1.07), 1.780 (0.98), 1.795 (1.72), 1.810 (1.28), 1.814 (1.33), 1.828 (1.07), 1.933 (1.34), 1.952 (2.19), 1.970 (1.77), 1.986 (1.84), 2.004 (1.01), 2.332 (1.18), 2.336 (0.52), 2.518 (5.77), 2.522 (3.76), 2.673 (1.19), 2.678 (0.54), 5.309 (1.78), 5.326 (2.49), 5.341 (1.74), 5.990 (0.44), 6.006 (0.43), 6.035 (6.70), 6.993 (5.79), 6.998 (5.80), 7.241 (0.73), 7.244 (1.37), 7.247 (0.86), 7.256 (1.01), 7.262 (3.85), 7.268 (1.32), 7.277 (1.82), 7.280 (3.42), 7.284 (1.76), 7.324 (3.99), 7.328 (1.98), 7.335 (0.80), 7.340 (4.07), 7.344 (8.11), 7.357 (1.94), 7.362 (4.67), 7.369 (1.28), 7.386 (0.51), 7.409 (7.12), 7.426 (4.78), 7.430 (3.36), 7.469 (8.92), 7.475 (8.56), 8.036 (1.10), 8.041 (1.46), 8.069 (0.60), 8.074 (0.48), 8.093 (0.57), 8.098 (0.53).

### Intermediate 395

### 3-{(1S)-1-[(2-amino-5-bromopyridin-3-yl)oxy]ethyl}benzonitrile

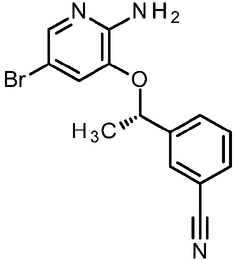

The compound was prepared similarly to **Intermediate 393** starting from 3-{(1S)-1-[(5-bromo-2-nitropyridin-3-yl)oxy]ethyl}benzonitrile **(Intermediate 339,** 374 mg, 55 % purity, 591 µmol) to give 167 mg (95 % purity, 84 % yield) of the title compound.
LC-MS (Method 1): Rt = 1.09 min; MS (ESlneg): m/z = 316 [M-H]⁻
¹H-NMR (400 MHz, CHLOROFORM-d) δ [ppm]: 0.008 (0.44), 1.251 (4.15), 1.269 (9.14), 1.286 (4.37), 1.677 (15.50), 1.693 (16.00), 2.056 (15.38), 4.103 (1.01), 4.122 (2.90), 4.140 (3.03), 4.157 (1.01), 4.761 (2.53), 5.284 (0.75), 5.299 (2.35), 5.316 (2.35), 5.332 (0.73), 6.771 (4.27), 6.775 (4.36), 7.494 (1.18), 7.495 (1.18), 7.514 (3.38), 7.533 (2.88), 7.572 (1.38), 7.575 (2.97), 7.578 (2.04), 7.592 (0.94), 7.595 (1.71), 7.599 (0.99), 7.617 (1.80), 7.621 (3.45), 7.624 (2.03), 7.635 (0.94), 7.639 (3.03), 7.643 (4.79), 7.645 (3.19), 7.647 (3.95), 7.651 (1.75), 7.709 (5.80), 7.713 (6.32).

### Intermediate 396

### 4-{(1S)-1-[(2-amino-5-bromopyridin-3-yl)oxy]ethyl}benzonitrile

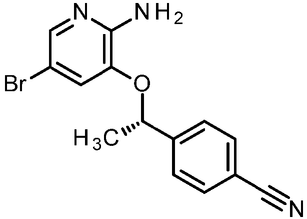

The compound was prepared similarly to **Intermediate 393** starting from 4-{(1S)-1-[(5-bromo-2-nitropyridin-3-yl)oxy]ethyl}benzonitrile **(Intermediate 340,** 366 mg, 50 % purity, 526 µmol) to give 131 mg (95 % purity, 74 % yield) of the title compound.
LC-MS (Method 1): Rt = 1.09 min; MS (ESlpos): m/z = 318 [M+H]⁺
¹H-NMR (400 MHz, CHLOROFORM-d) δ [ppm]: 0.007 (0.42), 1.250 (1.92), 1.268 (4.09), 1.286 (1.91), 1.676 (16.00), 1.692 (14.82), 2.055 (6.92), 4.103 (0.44), 4.121 (1.32), 4.139 (1.36), 4.157 (0.45), 4.759 (2.79), 5.297 (0.76), 5.313 (2.40), 5.329 (2.40), 5.345 (0.74), 6.747 (4.48), 6.751 (4.48), 7.447 (6.05), 7.450 (2.03), 7.463 (2.23), 7.467 (7.48), 7.675 (1.36), 7.680 (8.20), 7.684 (2.56), 7.696 (2.90), 7.699 (10.51), 7.704 (6.71).

### Intermediate 397

### 5-bromo-3-[(1R)-1-(2-fluorophenyl)ethoxy]pyridin-2-amine

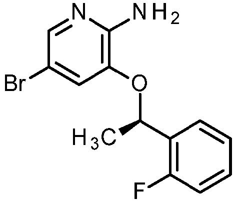

5-Bromo-3-[(1R)-1-(2-fluorophenyl)ethoxy]-2-nitropyridine **(Intermediate 344,** 492 mg, 1.44 mmol) was dissolved in ethanol (7.7 mL). Iron (322 mg, 5.77 mmol) and acetic acid (3.8 mL) were added under argon and the mixture was stirred for 3 h at 80 °C. The reaction mixture was allowed to cool down, diluted with dichloromethane and filtered. The filtrate was extracted with 2M NaOH. The organic phase was washed with brine, dried over sodium sulfate, filtered and concentrated to give 457 mg (85 % purity, 87 % yield) of the title compound.
LC-MS (Method 1): Rt = 1.24 min; MS (ESlpos): m/z = 311 [M+H]⁺

### Intermediate 398

### 5-bromo-3-[(1S)-1-(3,5-difluoropyridin-4-yl)ethoxy]pyridin-2-amine

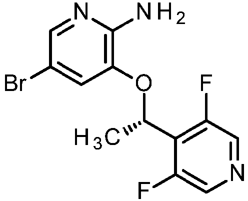

The compound was prepared similarly to **Intermediate 393** starting from 5-bromo-3-[(1S)-1-(3,5-difluoropyridin-4-yl)ethoxy]-2-nitropyridine **(Intermediate 341,** 366 mg, 68 % purity, 691 µmol) to give 153 mg (95 % purity, 64 % yield) of the title compound.
LC-MS (Method 1): Rt = 1.04 min; MS (ESlpos): m/z = 330 [M+H]⁺
¹H-NMR (400MHz, CHLOROFORM-d): δ [ppm]= 1.81 (d, 3H), 4.74 (br s, 2H), 5.72 (d, 1H), 6.97 (d, 1H), 7.71 (d, 1H), 8.36 (s, 2H).

### Intermediate 399

### (rac)-5-bromo-3-[1-(1-methyl-1H-pyrazol-5-yl)ethoxy]pyridin-2-amine

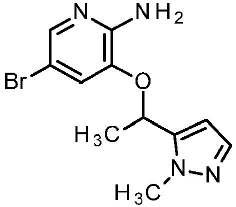

To a suspension of (rac)-5-bromo-3-[1-(1-methyl-1H-pyrazol-5-yl)ethoxy]-2-nitropyridine **(Intermediate 343,** 375 mg, 1.15 mmol) in ethanol (6.1 mL) under argon were added iron (256 mg, 4.59 mmol) and acetic acid (3.1 mL) and the mixture was stirred for 3 h at 80 °C. The reaction mixture was allowed to cool down, diluted with dichloromethane and filtered. The filtrate was extracted with 2M NaOH and filtered over celite. The organic layer was washed with brine, dried over sodium sulfate, filtered and concentrated to give 318 mg (96 % purity, 90 % yield) of the title compound.
LC-MS (Method 1): Rt = 0.90 min; MS (ESlneg): m/z = 295 [M-H]⁻
¹H-NMR (400MHz, CHLOROFORM-d): δ [ppm]= 1.75 (d, 3H), 3.85 (s, 3H), 4.66 (br s, 2H), 5.40 (q, 1H), 6.30 (d, 1H), 7.00 (d, 1H), 7.45 (d, 1H), 7.74 (d, 1H).

### Intermediate 400

### 5-bromo-N³-methyl-N³-[(1R)-1-phenylethyl]pyridine-2,3-diamine

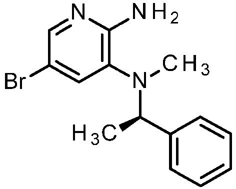

5-Bromo-N-methyl-2-nitro-N-[(1R)-1-phenylethyl]pyridin-3-amine **(Intermediate 345,** 182 mg, 541 µmol) was suspended in ethanol (2.9 mL). Iron (121 mg, 2.17 mmol) and acetic acid (1.4 mL) were added and the mixture was stirred for 3 h at 80 °C. The cold reaction mixture was diluted with dichloromethane and filtered. The filtrate was extracted with 2M NaOH and filtered over celite. The organic phase was washed with brine, dried over sodium sulfate, filtered and concentrated to give 103 mg (90 % purity, 56 % yield) of the tilte compound.
LC-MS (Method 1): Rt = 1.31 min; MS (ESlpos): m/z = 306 [M+H]⁺
¹H-NMR (400MHz, CHLOROFORM-d): δ [ppm]= 1.38 (br d, 3H), 2.43 (s, 3H), 4.25 (br q, 1H), 4.88 (br s, 2H), 7.17 (s, 1H), 7.27 - 7.40 (m, 5H), 7.87 (s, 1H).

### Intermediate 401

### 5-bromo-3-[(1R)-1-(4-methylpyridin-2-yl)ethoxy]pyridin-2-amine

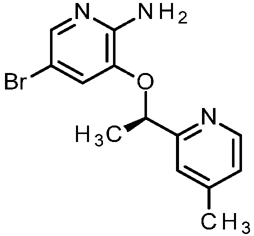

5-Bromo-3-[(1R)-1-(4-methylpyridin-2-yl)ethoxy]-2-nitropyridine **(Intermediate 346,** 187 mg, 553 µmol) was suspended in ethanol (3.0 mL). Iron (124 mg, 2.21 mmol) and acetic acid (1.5 mL) were added and the mixture was stirred for 3 h at 80 °C. The cold reaction mixture was diluted with dichloromethane and filtered. The filtrate was extracted with 2M NaOH and filtered over celite. The organic phase was washed with brine, dried over sodium sulfate, filtered and concentrated. The residue was purified by flash column chromatography (silica gel, hexane / ethyla acetate (0-100 %) gradient) to give 185 mg (90 % purity, 98 % yield) of the title compound.
LC-MS (Method 1): Rt = 1.03 min; MS (ESlpos): m/z = 308 [M+H]⁺
¹H NMR (400 MHz, CHLOROFORM-d, 22°C): δ = 8.44 (d, 1H), 7.67 (d, 1H), 7.13 (s, 1H), 7.04 (dd, 1H), 6.88 (d, 1H), 5.30 (q, 1H), 4.80 (br s, 2H), 2.33 (s, 3H), 1.69 ppm (d, 3H).

### Intermediate 402

### 5-bromo-3-[(1R)-1-(6-methylpyridin-2-yl)ethoxy]pyridin-2-amine

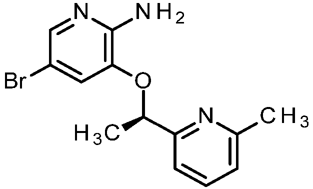

To a suspension of 5-bromo-3-[(1R)-1-(6-methylpyridin-2-yl)ethoxy]-2-nitropyridine **(Intermediate 347,** 156 mg, 461 µmol) in ethanol (2.5 mL) were added iron (103 mg, 1.85 mmol) and acetic acid (1.2 mL). The mixture was stirred for 3 h at 80 °C. The cold reaction mixture was diluted with dichloromethane and filtered. The filtrate was extracted with 2M NaOH and filtered over celite. The organic phase was washed with brine, dried over sodium sulfate, filtered and concentrated. The residue was purified by flash column chromatography (silica gel, hexane / ethyla acetate (0-100 %) gradient) to give 123 mg (98 % purity, 85 % yield) of the title compound.

¹H NMR (400 MHz, CHLOROFORM-d, 22°C): δ = 7.66 (d, 1H), 7.56 (t, 1H), 7.09 (dd, 2H), 6.90 (d, 1H), 5.30 (q, 1H), 4.81 (br s, 2H), 2.58 (s, 3H), 1.70 ppm (d, 3H).

### Intermediate 403

### 5-bromo-3-[(1R)-1-(3-chlorophenyl)ethoxy]pyridin-2-amine

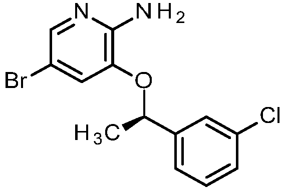

5-Bromo-3-[(1R)-1-(3-chlorophenyl)ethoxy]-2-nitropyridine (495 mg, 98 % purity, 1.36 mmol) was dissolved in ethanol (20 mL). Acetic acid (3.6 mL) was added dropwise. Iron (303 mg, 5.43 mmol) was added and the mixture was stirred for 30 min at 80 °C. The reaction mixture was allowed to reach rt, diluted with dichloromethane and filtered over celite. The organic phase was washed with 2M hydrochloric acid. The aqueous phase was extracted with dichloromethane. The combined organic layers were washed with saturated sodium bicarbonate solution, dried over a hydrophobic filter and concentrated. The residue was purified by column chromatography (silica gel, hexane / ethyl acetate (0 - 100 %) gradient) to give 380 mg (98 % purity, 84 % yield) of the title compound.
LC-MS (Method 1): Rt = 1.29 min; MS (ESlpos): m/z = 327 [M+H]⁺

### Intermediate 404

### 5-bromo-3-[(1R)-1-(2-methoxyphenyl)ethoxy]pyridin-2-amine

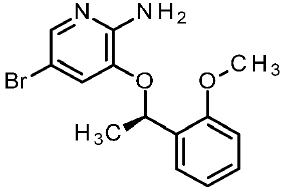

5-Bromo-3-[(1R)-1-(2-methoxyphenyl)ethoxy]-2-nitropyridine (470 mg, 97 % purity, 1.29 mmol) was dissolved in ethanol (10 mL). Acetic acid (3.4 mL) was added dropwise. Iron (288 mg, 5.16 mmol) was added and the mixture was stirred for 30 min at 80 °C. The reaction mixture was allowed to cool down, diluted with dichloromethane and filtered over celite. The organic phase was washed with 2M hydrochloric acid. The aqueous phase was extracted with dichloromethane. The combined organic layers were washed with saturated sodium bicarbonate solution, dried over a hydrophobic filter and concentrated. The residue was purified by column chromatography (silica gel, hexane / ethyl acetate (0 - 40 %) gradient) to give 391 mg (100 % purity, 94 % yield) of the title compound.
[α]²⁰_{D}: -65.9° (c = 1.00, methanol)
LC-MS (Method 2): Rt = 1.17 min; MS (ESlpos): m/z = 323 [M+H]⁺
¹H-NMR (400 MHz, DMSO-d6) δ [ppm]: 1.533 (5.88), 1.548 (5.88), 2.518 (1.02), 2.523 (0.65), 3.882 (16.00), 5.670 (1.26), 5.686 (1.26), 6.020 (2.76), 6.797 (2.37), 6.803 (2.39), 6.912 (0.70), 6.914 (0.74), 6.930 (1.53), 6.932 (1.56), 6.949 (0.86), 6.951 (0.86), 7.034 (1.37), 7.036 (1.39), 7.055 (1.74), 7.056 (1.60), 7.245 (0.86), 7.248 (0.98), 7.263 (0.97), 7.265 (1.04), 7.267 (1.11), 7.269 (1.00), 7.283 (0.68), 7.287 (0.70), 7.342 (1.41), 7.346 (1.33), 7.361 (1.32), 7.365 (1.18), 7.481 (3.34), 7.486 (3.32).

### Intermediate 405

### 5-bromo-3-[(1R)-1-(pyridin-3-yl)ethoxy]pyridin-2-amine

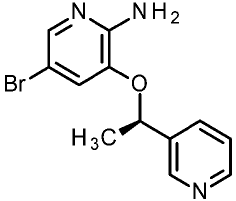

5-Bromo-2-nitro-3-[(1R)-1-(pyridin-3-yl)ethoxy]pyridine (203 mg, 82 % purity, 512 µmol) was dissolved in ethanol (2.7 mL). Iron (114 mg, 2.05 mmol) and acetic acid (1.3 mL) were added and the mixture was stirred for 3 h at 80 °C. The cold reaction mixture was diluted with dichloromethane and filtered. The filtrate was extracted with 2 M sodium hydroxide solution. The organic phase was washed with water and brine, dried and concentrated. The residue was purified by column chromatography (silica gel, hexane / ethyl acetate (0 - 100 %) gradient) to give 88.3 mg (89 % purity, 52 % yield) of the title compound.
LC-MS (Method 1): Rt = 0.90 min; MS (ESlneg): m/z = 292 [M-H]⁻
¹H-NMR (400 MHz, DMSO-d6) δ [ppm]: 1.570 (16.00), 1.586 (15.99), 2.518 (3.41), 2.523 (2.20), 5.658 (1.02), 5.673 (3.48), 5.689 (3.45), 5.705 (1.01), 5.758 (6.68), 6.084 (8.13), 7.194 (6.55), 7.199 (6.76), 7.369 (2.60), 7.381 (2.75), 7.388 (2.85), 7.400 (2.86), 7.515 (8.98), 7.520 (8.71), 7.881 (2.07), 7.886 (3.37), 7.891 (2.23), 7.901 (2.01), 7.905 (3.07), 7.910 (1.98), 8.480 (4.54), 8.484 (4.51), 8.492 (4.57), 8.496 (4.28), 8.687 (5.36), 8.692 (5.37).

### Intermediate 406

### 5-bromo-3-[(1R)-1-(1,3-thiazol-2-yl)ethoxy]pyridin-2-amine

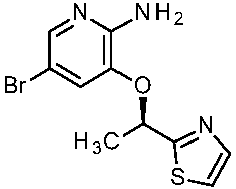

5-Bromo-2-nitro-3-[(1R)-1-(1,3-thiazol-2-yl)ethoxy]pyridine (200 mg, 606 µmol) was dissolved in ethanol (3.2 mL). Acetic acid (1.6 mL) and iron (135 mg, 2.42 mmol) were added and the mixture was stirred for 1.5 h at 80 °C. The reaction mixture was allowed to cool down, diluted with ethyl acetate and filtered. The filtrate was concentrated and diluted with ethyl acetate again. The organic phase was washed with saturated sodium bicarbonate solution and brine, dried over a hydrophobic filter and concentrated to give 157 mg (97 % purity, 84 % yield) of the title compound.
[α]²⁰_{D}: +26.3° (c = 1.00, methanol)
LC-MS (Method 1): Rt = 0.95 min; MS (ESlpos): m/z = 300 [M+H]⁺
¹H-NMR (400MHz, DMSO-d₆): δ [ppm]= 1.70 (d, 3H), 5.90 (q, 1H), 6.02 (s, 2H), 7.28 (d, 1H), 7.59 (d, 1H), 7.74 (d, 1H), 7.81 (d, 1H).

### Intermediate 407

### 5-bromo-3-[(1R)-1-(3-fluorophenyl)ethoxy]pyridin-2-amine

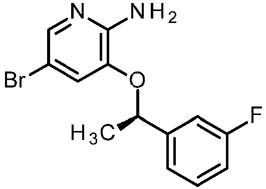

5-Bromo-3-[(1R)-1-(3-fluorophenyl)ethoxy]-2-nitropyridine (343 mg, 100 % purity, 1.01 mmol) was dissolved in ethanol (12 mL). Acetic acid (2.7 mL) and iron (225 mg, 4.02 mmol) were added and the mixture was stirred for 30 min at 80 °C. The reaction mixture was allowed to cool down, diluted with dichloromethane and filtered over celite. The organic phase was washed with 2M hydrochloric acid. The aqueous phase was extracted with dichloromethane.

The combined organic layers were washed with saturated sodium bicarbonate solution, dried over a hydrophobic filter and concentrated. The residue was purified by column chromatography (silica gel, hexane / ethyl acetate (0 - 80 %) gradient) to give 270 mg (97 % purity, 84 % yield) of the title compound.
[α]²⁰_{D}: +17.3° (c = 1.00, methanol)
LC-MS (Method 1): Rt = 1.21 min; MS (ESlpos): m/z = 311 [M+H]⁺
¹H-NMR (400 MHz, DMSO-d6) δ [ppm]: 1.153 (1.82), 1.171 (3.65), 1.189 (1.76), 1.535 (15.95), 1.551 (16.00), 1.986 (6.83), 2.518 (1.65), 2.523 (0.99), 3.998 (0.50), 4.016 (1.48), 4.034 (1.43), 4.052 (0.46), 5.592 (0.97), 5.608 (3.27), 5.623 (3.24), 5.639 (0.95), 6.084 (8.35), 7.069 (1.09), 7.072 (1.20), 7.076 (1.27), 7.079 (1.26), 7.092 (2.41), 7.097 (2.18), 7.099 (2.60), 7.119 (8.15), 7.124 (7.32), 7.284 (3.00), 7.303 (4.42), 7.328 (2.12), 7.335 (2.39), 7.338 (1.83), 7.354 (2.10), 7.360 (2.35), 7.364 (2.09), 7.367 (2.58), 7.382 (2.50), 7.387 (3.16), 7.402 (3.07), 7.407 (1.68), 7.422 (1.44), 7.506 (9.38), 7.511 (9.00).

### Intermediate 408

### (rac)-5-bromo-3-[(1-(1,3-oxazol-4-yl)ethoxy]pyridin-2-amine

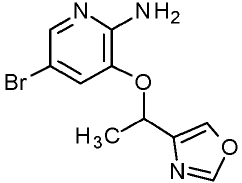

5-Bromo-2-nitro-3-[1-(1,3-oxazol-4-yl)ethoxy]pyridine (185 mg, 589 µmol) was dissolved in ethanol (3.1 mL). Acetic acid (1.6 mL) and iron (98.7 mg, 1.77 mmol) were added and the mixture was stirred for 1.5 h at 80 °C. The reaction mixture was allowed to cool down, diluted with ethyl acetate and filtered. The filtrate was concentrated and diluted with ethyl acetate again. The organic phase was washed with saturated sodium bicarbonate solution and brine, dried over a hydrophobic filter and concentrated to give 140 mg (100 % purity, 84 % yield) of the title compound.
LC-MS (Method 2): Rt = 0.71 min; MS (ESlpos): m/z = 284 [M+H]⁺
¹H-NMR (400MHz, DMSO-d₆): δ [ppm]= 1.57 (br d, 3H), 5.46 - 5.54 (m, 1H), 5.91 (br s, 2H), 7.34 (br s, 1H), 7.58 (br s, 1H), 8.21 (br s, 1H), 8.38 (br s, 1H).

### Intermediate 409

### (rac)-5-bromo-3-[1-(1,3-oxazol-2-yl)ethoxy]pyridin-2-amine

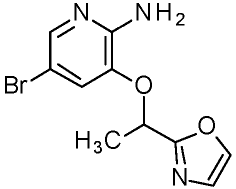

(Rac)-5-bromo-2-nitro-3-[1-(1,3-oxazol-2-yl)ethoxy]pyridine (155 mg, 493 µmol) was dissolved in ethanol (2.6 mL). Acetic acid (1.3 mL) and iron (82.7 mg, 1.48 mmol) were added and the mixture was stirred for 2 h at 80 °C. The reaction mixture was allowed to cool down, diluted with ethyl acetate and filtered. The filtrate was concentrated and diluted with ethyl acetate again. The organic phase was washed with saturated sodium bicarbonate solution and brine, dried over a hydrophobic filter and concentrated to give 107 mg (76 % purity, 58 % yield) of the title compound.
LC-MS (Method 1): Rt = 0.89 min; MS (ESlpos): m/z = 284 [M+H]⁺
¹H-NMR (400MHz, DMSO-d₆): δ [ppm]= 1.68 (d, 3H), 5.69 (q, 1H), 5.96 (s, 2H), 7.25 (s, 1H), 7.31 (d, 1H), 7.60 (d, 1H), 8.15 (s, 1H).

### Intermediate 410

### 5-bromo-3-[(1R)-1-(pyrimidin-2-yl)ethoxy]pyridin-2-amine

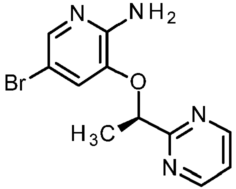

2-{(1R)-1-[(5-bromo-2-nitropyridin-3-yl)oxy]ethyl}pyrimidine (679 mg, 2.09 mmol) was dissolved in ethanol (18 mL) under argon. Acetic acid (5.6 mL) and iron (700 mg, 12.5 mmol) were added and the mixture was stirred for 4 h at 80 °C. The reaction mixture was allowed to cool down, diluted with ethyl acetate and filtered. The filtrate was concentrated and diluted with ethyl acetate again. The organic phase was washed with saturated sodium bicarbonate solution, dried and concentrated. The residue was purified by column chromatography (silica gel, dichloromethane / ethanol (0 - 10 %) gradient) to give 600 mg (97 % yield) of the title compound.
LC-MS (Method 1): Rt = 0.85 min; MS (ESlpos): m/z = 295 [M+H]⁺
¹H NMR (DMSO-d₆, 400 MHz): δ (ppm) 8.84 (d, 2H), 7.53 (d, 1H), 7.46 (t, 1H), 6.99 (d, 1H), 5.96 (s, 2H), 5.52 (q, 1H), 1.67 (d, 3H).

### Intermediate 411

### 5-bromo-3-[(1R)-1-(pyridin-3-yl)ethoxy]pyridin-2-amine

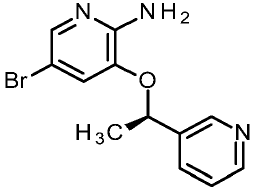

5-Bromo-2-nitro-3-[(1R)-1-(pyridin-3-yl)ethoxy]pyridine (4.95 g, 33 % purity, 5.07 mmol) was dissolved in acetic acid (9.9 mL) and methanol (9.9 mL). Iron (849 mg, 15 mmol) was added and the mixture was stirred for 1.5 h at 85 °C. The reaction mixture was allowed to cool down, diluted with ethyl acetate and filtered. The filtrate was concentrated and diluted with dichloromethane. The organic phase was washed with saturated sodium bicarbonate solution and brine, dried over a hydrophobic filter and concentrated. The residue was purified by column chromatography (silica gel, hexane / ethanol (0 - 30 %) gradient) to give 950 mg (63 % yield) of the title compound.
LC-MS (Method 1): Rt = 0.89 min; MS (ESlpos): m/z = 294 [M+H]⁺
¹H-NMR (400MHz, DMSO-d₆): δ [ppm]= 1.58 (d, 3H), 5.68 (q, 1H), 6.09 (s, 2H), 7.20 (d, 1H), 7.38 (dd, 1H), 7.52 (d, 1H), 7.90 (dt, 1H), 8.49 (dd, 1H), 8.69 (d, 1H).

### Intermediate 412

### 5-bromo-3-[(1R)-1-(1,2-thiazol-5-yl)ethoxy]pyridin-2-amine

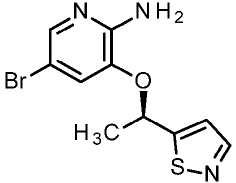

5-Bromo-2-nitro-3-[(1R)-1-(1,2-thiazol-5-yl)ethoxy]pyridine (157 mg, 476 µmol) was dissolved in ethanol (2.5 mL). Acetic acid (1.3 mL) and iron (79.7 mg, 1.43 mmol) were added and the mixture was stirred for 1.5 h at 80 °C. The reaction mixture was allowed to cool down, diluted with ethyl acetate and filtered. The filtrate was concentrated and diluted with ethyl acetate again. The organic phase was washed with saturated sodium bicarbonate solution and brine, dried over a hydrophobic filter and concentrated to give 125 mg (100 % purity, 88 % yield) of the title compound.
LC-MS (Method 1): Rt = 0.97 min; MS (ESlpos): m/z = 300 [M+H]⁺
¹H-NMR (400MHz, DMSO-d₆): δ [ppm]= 1.67 (d, 3H), 6.03 (s, 2H), 6.09 (q, 1H), 7.31 (d, 1H), 7.47 (dd, 1H), 7.58 (d, 1H), 8.50 (d, 1H).

### Intermediate 413

### (rac)-5-bromo-3-[1-(2,6-dichlorophenyl)ethoxy]pyridin-2-amine

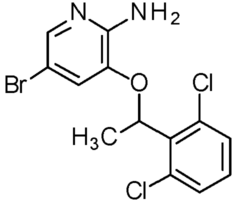

(Rac)-5-Bromo-3-[1-(2,6-dichlorophenyl)ethoxy]-2-nitropyridine (977 mg, 2.49 mmol) was dissolved in methanol (13 mL) and acetic acid (13 mL). Iron (418 mg, 7.48 mmol) was added and the mixture was stirred for 1 h at 85 °C. The reaction mixture was allowed to cool down, filtered and the filtrate was concentrated. The residue was purified by column chromatography (silica gel, hexane / ethyl acetate (0 - 70 %) gradient) to give 950 mg (63 % yield) of the title compound.
LC-MS (Method 1): Rt = 1.39 min; MS (ESlpos): m/z = 361 [M+H]⁺
¹H NMR (DMSO-d₆, 400 MHz): δ (ppm) 7.53 (d, 1H), 7.47 - 7.51 (m, 2H), 7.33 - 7.39 (m, 1H), 6.73 (d, 1H), 5.94 - 6.03 (m, 3H), 1.76 (d, 3H).

### Intermediate 414

### (rac)-4-(3-{1-[(2-amino-5-bromopyridin-3-yl)oxy]ethyl}phenyl)-2-methylbut-3-yn-2-ol

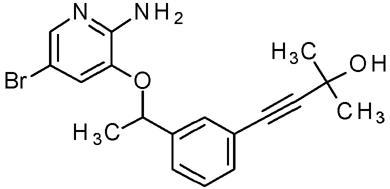

5-Bromo-3-{[1-(3-{3-methyl-3-[(-oxan-2-yl)oxy]but-1-yn-1-yl}phenyl)ethyl]oxy}-2-nitropyridine (214 mg, 437 µmol) was suspended in ethanol (2.3 mL). Acetic acid (1.2 mL) and iron (97.7 mg, 1.75 mmol) were added and the mixture was stirred for 3 h at 80 °C and over night at room temperature. The reaction mixture was heated to 80 °C, after 3 h hydrochloric acid in 1,4-dioxane (30 µL, 4M) was added and stirred for 3 h at 80 °C again. The reaction mixture was concentrated and diluted with dichloromethane / ethanol 9:1 and filtered. The filtrate was dried and concentrated. The residue was purified by column chromatography (silica gel, dichloromethane / methanol (0 - 5 %) gradient) to give 161 mg (98 % yield) of the title compound.
LC-MS (Method 1): Rt = 1.16 min; MS (ESlpos): m/z = 375 [M+H]⁺
¹H NMR (DMSO-d₆, 400 MHz): δ (ppm) 7.50 (d, 1H), 7.43 - 7.47 (m, 2H), 7.32 - 7.37 (m, 1H), 7.25 - 7.30 (m, 1H), 7.05 (d, 1H), 6.09 (s, 2H), 5.58 (q, 1H), 5.47 (s, 1H), 1.54 (d, 3H), 1.45 (s, 6H).

### Intermediate 415

### 5-bromo-3-[(6,7-dihydro-5H-cyclopenta[b]pyridin-7-yl)oxy]pyridin-2-amine (enantiomer 1 and enantiomer 2)

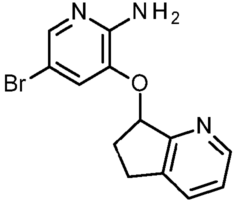

(Rac)-7-[(5-Bromo-2-nitropyridin-3-yl)oxy]-6,7-dihydro-5H-cyclopenta[b]pyridine (935 mg, 2.78 mmol) was suspended in ethanol (15 mL). Iron (621 mg, 11.1 mmol) and acetic acid (7.4 mL) were added and the mixture was stirred for 3 h at 80 °C. The reaction mixture was allowed to cool down, diluted with dichloromethane and filtered. The filtrate was concentrated, diluted with saturated sodium bicarbonate solution and ethyl acetate / methanol 95:5 and filtered over celite. The layers were separated. The aqueous phase was extracted with ethyl acetate / methanol 95:5. The combined organic layers were dried and concentrated. The crude product was purified by column chromatography (silica gel, dichloromethane / ethanol (0 - 5 %) gradient) to give 879 mg (103 % yield) of the title compound.
LC-MS (Method 1): Rt = 1.03 min; MS (ESlpos): m/z = 306 [M+H]⁺
¹H NMR (DMSO-d₆, 400 MHz): δ (ppm) 8.43 - 8.47 (m, 1H), 7.78 (dd, 1H), 7.68 (d, 1H), 7.61 (d, 1H), 7.32 (dd, 1H), 5.89 (s, 2H), 5.73 (dd, 1H), 3.08 - 3.18 (m, 1H), 2.89 (ddd, 1H), 2.52 - 2.60 (m, 1H), 2.21 (dddd, 1H).

### Intermediate 416 and Intermediate 417

### 5-bromo-3-[(6,7-dihydro-5H-cyclopenta[b]pyridin-7-yl)oxy]pyridin-2-amine (enantiomer 1 and enantiomer 2)

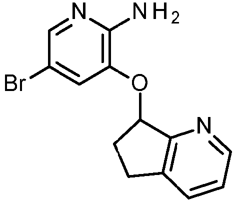

The compound from Intermediate 415 was separated into enantiomers by preparative chiral HPLC.

### Preparative

Instrument: PrepCon Labomati HPLC-4; Column: Reprosil NR 8µ, 250x50; eluent A: hexane + 0.1 vol % diethylamine; eluent B: ethanol; isocratic: 40%B; flow: 100 mL/min; temperature: 25 °C; UV: 325 nm

### Analytical

Instrument: Thermo Fisher UltiMate 3000; Column: Reprosil NR 5µ, 100x4.6; eluent A: hexane + 0.1 vol % diethylamine; eluent B: ethanol; isocratic: 40%B; flow: 1.4 mL/min; temperature: 25 °C; UV: 325 nm

### Enantiomer 1:

127.9 mg (27 % yield)
Retention time preparative method: 8.89 - 11.57 min
Retention time analytical method: 2.55 min
LC-MS (Method 1): Rt = 1.00 min; MS (ESlpos): m/z = 306 [M+H]⁺
¹H-NMR (400MHz, CHLOROFORM-d): δ [ppm]= 2.30 (dddd, 1H), 2.62 (dddd, 1H), 2.95 (ddd, 1H), 3.11 - 3.23 (m, 1H), 4.89 (br s, 2H), 5.56 (dd, 1H), 7.21 - 7.26 (m, 1H), 7.46 (d, 1H), 7.65 (dd, 1H), 7.77 (d, 1H), 8.45 - 8.59 (m, 1H).

### Enantiomer 2:

123.1 mg (26 % yield)
Retention time preparative method: 14.52 - 18.00 min
Retention time analytical method: 5.27 min
LC-MS (Method 1): Rt = 1.00 min; MS (ESlpos): m/z = 306 [M+H]⁺
¹H-NMR (400MHz, CHLOROFORM-d): δ [ppm]= 2.25 - 2.35 (m, 1H), 2.57 - 2.68 (m, 1H), 2.97 (br dd, 1H), 3.12 - 3.23 (m, 1H), 4.76 - 5.04 (m, 2H), 5.54 - 5.59 (m, 1H), 7.22 - 7.26 (m, 1H), 7.46 (d, 1H), 7.65 (dd, 1H), 7.77 (d, 1H), 8.46 - 8.58 (m, 1H).

### Intermediate 418

### (rac)-5-bromo-3-[1-(4,5-dimethyl-1,3-thiazol-2-yl)ethoxy]pyridin-2-amine

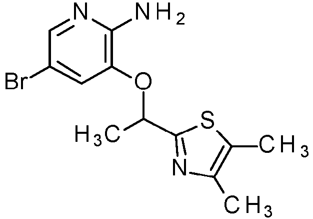

(Rac)-5-Bromo-3-[1-(4,5-dimethyl-1,3-thiazol-2-yl)ethoxy]-2-nitropyridine (269 mg, 751 µmol) was dissolved in ethanol (4 mL) under argon.Acetic acid (2 mL) and iron (126 mg, 2.25 mmol) were added and the mixture was stirred for 1.5 h at 80 °C. The reaction mixture was allowed to cool down, diluted with ethyl acetate and filtered. The filtrate was concentrated and diluted in ethyl acetate. The organic phase was washed with saturated sodium bicarbonate solution and brine, dried over a hydrophobic filter and concentrated to give 194 mg (96 % purity, 76 % yield) of the title compound.
LC-MS (Method 1): Rt = 1.13 min; MS (ESlpos): m/z = 328 [M+H]⁺
¹H-NMR (400MHz, DMSO-d₆): δ [ppm]= 1.64 (d, 3H), 2.24 (s, 3H), 2.29 (s, 3H), 5.70 - 5.77 (m, 1H), 5.99 (s, 2H), 7.26 (d, 1H), 7.58 (d, 1H).

### Intermediate 419

### (rac)-5-bromo-3-[1-(5-methyl-1,3-thiazol-2-yl)ethoxy]pyridin-2-amine

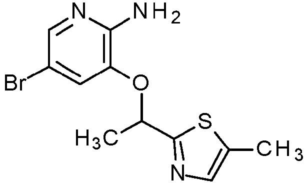

(Rac)-5-Bromo-3-[1-(5-methyl-1,3-thiazol-2-yl)ethoxy]-2-nitropyridine (257 mg, 747 µmol) was dissolved in ethanol (4 mL) under argon. Acetic acid (2 mL) and iron (125 mg, 2.24 mmol) were added and the mixture was stirred for 1.5 h at 80 °C. The reaction mixture was allowed to reach rt, diluted with ethyl acetate and filtered. The filtrate was concentrated and diluted in ethyl acetate again. The organic phase was washed with saturated sodium bicarbonate solution and brine, dried over a hydrophobic filter and concentrated to give 212 mg (96 % purity, 87 % yield) of the title compound.
LC-MS (Method 1): Rt = 1.06 min; MS (ESlpos): m/z = 314 [M+H]⁺
¹H-NMR (400MHz, DMSO-d₆): δ [ppm]= 1.66 (d, 3H), 2.41 (d, 3H), 5.79 (q, 1H), 6.00 (s, 2H), 7.26 (d, 1H), 7.46 (d, 1H), 7.58 (d, 1H).

### Intermediate 420

### (rac)-5-bromo-3-[1-(1,3-thiazol-4-yl)ethoxy]pyridin-2-amine

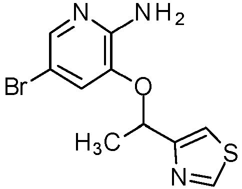

(Rac)-5-Bromo-2-nitro-3-[1-(1,3-thiazol-4-yl)ethoxy]pyridine (237 mg, 718 µmol) was dissolved in ethanol (3.8 mL). Acetc acid (1.9 mL) and iron (120 mg, 2.15 mmol) were added and the mixture was stirred for 1.5 h at 80 °C. The reaction mixture was allowed to cool down, diluted with ethyl acetate and filtered. The filtrate was concentrated and diluted in ethyl acetate again. The organic phase was washed with saturated sodium bicarbonate solution and brine, dried over a hydrophobic filter and concentrated to give 185 mg (100 % purity, 86 % yield) of the title compound.
LC-MS (Method 1): Rt = 0.96 min; MS (ESlpos): m/z = 300 [M+H]⁺
¹H-NMR (400MHz, DMSO-d₆): δ [ppm]= 1.63 (d, 3H), 5.70 (q, 1H), 5.96 (s, 2H), 7.25 (d, 1H), 7.56 (d, 1H), 7.78 (d, 1H), 9.10 (d, 1H).

### Intermediate 421

### 5-bromo-3-[(1R)-1-(5-methyl-1,3,4-oxadiazol-2-yl)ethoxy]pyridin-2-amine

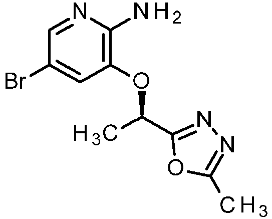

5-Bromo-3-[(1R)-1-(5-methyl-1,3,4-oxadiazol-2-yl)ethoxy]-2-nitropyridine (160 mg, 486 µmol) was dissolved in ethanol (2.6 mL). Acetic acid (1.3 mL) and iron (81.4 mg, 1.46 mmol) were added and the mixture was stirred for 1.5 h at 80 °C. The reaction mixture was allowed to cool down, diluted with ethyl acetate and filtered. The filtrate was concentrated and diluted in ethyl acetate again. The organic phase was washed with saturated sodium bicarbonate solution and brine, dried over a hydrophobic filter and concentrated to give 101 mg (93 % purity, 65 % yield) of the title compound.
LC-MS (Method 1): Rt = 0.80 min; MS (ESlpos): m/z = 299 [M+H]⁺
¹H-NMR (400MHz, DMSO-d₆): δ [ppm]= 1.70 (d, 3H), 5.83 (q, 1H), 6.01 (s, 2H), 7.39 (d, 1H), 7.63 (d, 1H).

### Intermediate 422

### 5-bromo-3-[(1R)-1-(2-methyl-1,3-thiazol-4-yl)ethoxy]pyridin-2-amine

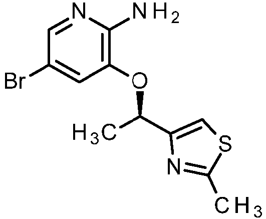

5-Bromo-3-[(1R)-1-(2-methyl-1,3-thiazol-4-yl)ethoxy]-2-nitropyridine (183 mg, 531 µmol) was dissolved in ethanol (2.8 mL). Acetic acid (1.4 mL) and iron (119 mg, 2.12 mmol) were added and the mixture was stirred for 2 h at 80 °C. The reaction mixture was allowe to cool down, diluted with ethyl acetate and filtered. The filtrate was concentrated and diluted in ethyl acetate again. The organic phase was washed with saturated sodium bicarbonate solution and brine, dried over a hydrophobic filter and concentrated to give 158 mg (95 % purity, 90 % yield) of the title compound.
LC-MS (Method 1): Rt = 1.02 min; MS (ESlpos): m/z = 314 [M+H]⁺
¹H-NMR (400MHz, DMSO-d₆): δ [ppm]= 1.59 (d, 3H), 2.64 (s, 3H), 5.58 (q, 1H), 5.95 (s, 2H), 7.25 (d, 1H), 7.52 (s, 1H), 7.56 (d, 1H).

### Intermediate 423

### (rac)-5-bromo-3-[1-(4-methyl-1,3-thiazol-2-yl)ethoxy]pyridin-2-amine

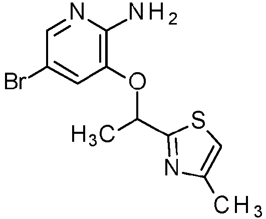

(Rac)-5-Bromo-3-[1-(4-methyl-1,3-thiazol-2-yl)ethoxy]-2-nitropyridine (186 mg, 540 µmol) was dissolved in ethanol (2.9 mL). Acetic acid (1.4 mL) and iron (121 mg, 2.16 mmol) were added and the mixture was stirred for 1.5 h at 80 °C. The reaction mixture was allowed to cool down, diluted with ethyl acetate and filtered. The filtrate was concentrated and diluted in ethyl acetate again. The organic phase was washed with saturated sodium bicarbonate solution and brine, dried over a hydrophobic filter and concentrated to give 129 mg (91 % purity, 69 % yield) of the title compound.
LC-MS (Method 1): Rt = 1.04 min; MS (ESlpos): m/z = 314 [M+H]⁺
¹H-NMR (400MHz, DMSO-d₆): δ [ppm]= 1.67 (d, 3H), 2.35 (d, 3H), 5.83 (q, 1H), 6.01 (s, 2H), 7.26 (d, 1H), 7.28 (d, 1H), 7.59 (d, 1H).

### Intermediate 424

### (rac)--bromo-3-[1-(1-methyl-1H-1,2,3-triazol-4-yl)ethoxy]pyridin-2-amine

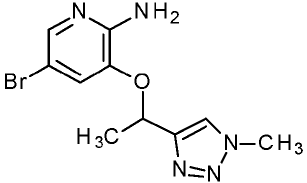

(Rac)-5-Bromo-3-[1-(1-methyl-1H-1,2,3-triazol-4-yl)ethoxy]-2-nitropyridine (319 mg, 972 µmol) was dissolved in ethanol (5.2 mL). Acetic acid (2.6 mL) and iron (217 mg, 3.89 mmol) were added and the mixture was stirred for 3 h at 80 °C. The reaction mixture was allowed to cool down, diluted with ethyl acetate and filtered. The filtrate was concentrated and diluted in ethyl acetate again. The organic phase was washed with saturated sodium bicarbonate solution and brine, dried over a hydrophobic filter and concentrated to give 281 mg (75 % purity, 73 % yield) of the title compound.
LC-MS (Method 1): Rt = 0.77 min; MS (ESlpos): m/z = 298 [M+H]⁺
¹H-NMR (400MHz, DMSO-d₆): δ [ppm]= 1.62 (d, 3H), 4.02 (s, 3H), 5.66 (q, 1H), 5.90 (s, 2H), 7.35 (d, 1H), 7.55 (d, 1H), 8.15 (s, 1H).

### Intermediate 425

### (rac)-5-bromo-3-{1-[3-(methanesulfonyl)phenyl]ethoxy}pyridin-2-amine

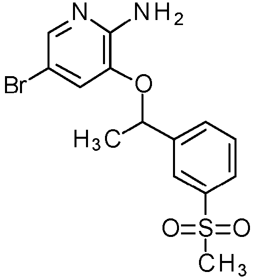

(Rac)-5-Bromo-3-{1-[3-(methanesulfonyl)phenyl]ethoxy}-2-nitropyridine (1.50 g, 3.74 mmol) was dissolved in methanol (19 mL) and acetic acid (19 mL). Iron (626 mg, 11.2 mmol) was added and the mixture was stirred for 1 h at 85 °C. The reaction mixture was filtered and concentrated. The residue was purified by column chromatography (silica gel, hexane / ethyl acetate (0 - 90 %) gradient) to give 989 mg (71 % yield) of the title compound.
LC-MS (Method 1): Rt = 0.97 min; MS (ESlpos): m/z = 371 [M+H]⁺
¹H-NMR (400 MHz, DMSO-d6) δ [ppm]: 1.345 (1.59), 1.352 (0.48), 1.361 (1.64), 1.572 (2.31), 1.588 (2.28), 1.907 (16.00), 3.144 (0.98), 3.166 (9.45), 3.191 (1.04), 3.204 (4.02), 3.215 (7.57), 3.228 (0.62), 3.338 (0.63), 5.739 (0.54), 5.755 (0.58), 6.117 (1.35), 7.196 (1.07), 7.202 (1.09), 7.516 (1.83), 7.521 (1.80), 7.595 (0.52), 7.627 (0.50), 7.647 (0.97), 7.666 (0.77), 7.835 (1.08), 7.839 (1.32), 7.854 (1.05), 7.859 (1.18), 7.906 (0.45), 8.061 (0.64), 8.065 (1.07), 8.070 (0.58).

### Intermediate 426

### (rac)-5-bromo-3-[1-(1,3-oxazol-4-yl)ethoxy]pyridin-2-amine

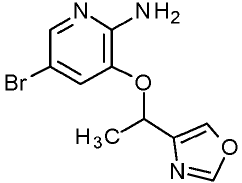

(Rac)-5-Bromo-2-nitro-3-[1-(1,3-oxazol-4-yl)ethoxy]pyridine (185 mg, 589 µmol) was dissolved in ethanol (3.1 mL). Acetic acid (1.6 mL) and iron (98.7 mg, 1.77 mmol) were added and the mixture was stirred for 1.5 h at 80 °C. The reaction mixture was allowed to cool down, diluted with ethyl acetate and filtered. The filtrate was concentrated and diluted in ethyl acetate again. The organic phase was washed with saturated sodium bicarbonate solution and brine, dried over a hydrophobic filter and concentrated to give 140 mg (100 % purity, 84 % yield) of the title compound.
LC-MS (Method 2): Rₜ = 0.71 min; MS (ESlpos): m/z = 284 [M+H]⁺
¹H-NMR (400MHz, DMSO-d₆): δ [ppm]= 1.57 (br d, 3H), 5.46 - 5.54 (m, 1H), 5.91 (br s, 2H), 7.34 (br s, 1H), 7.58 (br s, 1H), 8.21 (br s, 1H), 8.38 (br s, 1H).

### Intermediate 427

### (rac)-5-bromo-3-[1-(1-propyl-1H-pyrazol-5-yl)ethoxy]pyridin-2-amine (enantiomer 1 and enantiomer 2)

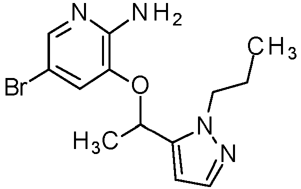

(Rac)-5-Bromo-2-nitro-3-[1-(1-propyl-1H-pyrazol-5-yl)ethoxy]pyridine (675 mg, 1.90 mmol) was dissolved in methanol (9 mL), THF (9mL) and water (18 mL). Iron (1.06 g, 19.0 mmol) and ammonium chloride (1.01 g, 19 mmol) were added and the mixture was stirred for 3 h at 80 °C. The reaction mixture was allowed to cool down, filtered and the filtrate was extracted with ethyl acetate. The combined organic layers were washed with brine, dried over sodium sulfate, filtered and concentrated. The crude product was purified by column chromatography (silica gel, hexane / ethyl acetate (0 - 50 %) gradient) followd by a second column chromatography (silica gel NH2, dichloromethane (100 %) isocratic) to give 159 mg (26 % yield) of the title compound.
LC-MS (Method 1): Rt = 1.05 min; MS (ESlpos): m/z = 325 [M+H]⁺
¹H-NMR (400 MHz, DMSO-d6) δ [ppm]: 0.781 (5.44), 0.799 (13.02), 0.818 (6.09), 0.825 (7.33), 0.844 (16.00), 0.862 (7.49), 1.172 (0.46), 1.399 (14.58), 1.415 (15.23), 1.579 (9.34), 1.595 (9.43), 1.670 (0.41), 1.688 (1.53), 1.707 (2.68), 1.715 (0.95), 1.725 (2.71), 1.734 (3.07), 1.743 (1.63), 1.752 (5.58), 1.762 (0.57), 1.771 (5.24), 1.789 (2.54), 1.808 (0.51), 1.988 (0.78), 2.518 (2.89), 2.523 (1.96), 3.982 (0.54), 3.999 (1.09), 4.016 (2.28), 4.021 (3.13), 4.034 (4.60), 4.038 (5.18), 4.052 (2.35), 4.057 (5.88), 4.075 (1.88), 4.091 (1.06), 4.109 (0.50), 4.769 (0.41), 4.785 (1.54), 4.800 (2.22), 4.816 (1.59), 4.832 (0.42), 5.234 (6.68), 5.248 (6.16), 5.759 (9.80), 5.777 (0.56), 5.793 (1.97), 5.809 (1.95), 5.825 (0.57), 5.961 (4.35), 6.110 (4.33), 6.114 (4.37), 6.383 (4.69), 6.388 (4.79), 7.294 (4.69), 7.299 (5.91), 7.305 (3.88), 7.383 (4.81), 7.388 (4.72), 7.564 (5.55), 7.569 (5.20).

### Intermediate 428

### (rac)-5-bromo-3-[1-(pyrimidin-4-yl)ethoxy]pyridin-2-amine

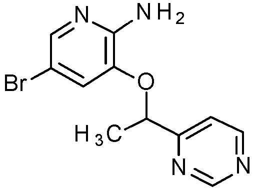

(Rac)-4-{1-[(5-Bromo-2-nitropyridin-3-yl)oxy]ethyl}pyrimidine (1.13 g, 3.47 mmol) was dissolved in ethanol (30 mL). Acetic acid (9.2 mL) and iron (1.16 g, 20.8 mmol]) were added and stirred for 4 h at 80 °C. The reaction mixture was allowed to cool down, diluted with ethyl acetate and filtered. The filtrate was concentrated and diluted in ethyl acetate again. The organic phase was washed with saturated sodium bicarbonate solution and water, dried and concentrated. The residue was purified by column chromatography (silica gel, dichloromethane / ethanol (0 - 10 %) gradient) followd by column chromatography (silica gel, ethyl acetate / ethanol (0 - 10 %) gradient) to give 757 mg (74 % yield) of the title compound.
LC-MS (Method 1): Rt = 0.84 min; MS (ESlpos): m/z = 295 [M+H]⁺
¹H-NMR (400 MHz, DMSO-d6) δ [ppm]: 1.154 (3.26), 1.172 (6.62), 1.189 (3.20), 1.581 (15.99), 1.597 (16.00), 1.987 (12.12), 2.470 (0.56), 2.518 (2.29), 2.523 (1.48), 3.999 (0.87), 4.017 (2.67), 4.035 (2.68), 4.053 (0.87), 5.540 (0.96), 5.556 (3.27), 5.572 (3.26), 5.589 (0.93), 6.147 (6.32), 7.138 (5.61), 7.143 (5.68), 7.570 (8.18), 7.575 (7.98), 7.679 (3.51), 7.683 (3.57), 7.692 (3.67), 7.695 (3.61), 8.819 (7.19), 8.832 (6.97), 9.182 (6.99), 9.186 (6.86).

### Intermediate 429

### 5-bromo-3-{[1-(2,4-dimethylpyrimidin-5-yl)ethyl]oxy}pyridin-2-amine (enantiomer 2)

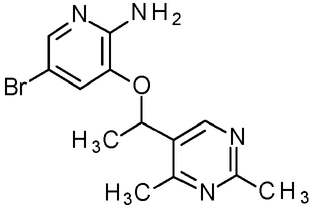

5-{1-[(5-Bromo-2-nitropyridin-3-yl)oxy]ethyl}-2,4-dimethylpyrimidine (enantiomer 2) (406 mg, 1.15 mmol) was dissolved in ethanol (10 mL). Acetic acid (3.1 mL) and iron (385 mg, 6.90 mmol) were added and the mixture was stirred for 3 h at 80 °C. The reaction mixture was allowed to cool down, diluted with ethyl acetate and filtered. The filtrate was concentrated and diluted in ethyl acetate again. The organic phase was washed with saturated sodium bicarbonate solution and water, dried and concentrated. The residue was purified by column chromatography (silica gel, dichloromethane / ethanol (0 - 10 %) gradient) to give 363 mg (98 % yield) of the title compound.
[α]²⁰_{D}: -3.3° (c = 1.00, methanol)
LC-MS (Method 1): Rt = 0.89 min; MS (ESlpos): m/z = 323 [M+H]⁺
¹H NMR (400 MHz, DMSO-*d*₆) δ ppm= 1.56 (d, 3 H), 2.53 (s, 3 H), 5.78 (q, 1 H), 6.13 (s, 2 H), 7.22 (d, 1 H), 7.55 (d, 1 H), 8.73 (s, 1 H).

### Intermediate 430

### 5-bromo-3-{[1-(2,4-dimethylpyrimidin-5-yl)ethyl]oxy}pyridin-2-amine (enantiomer 1)

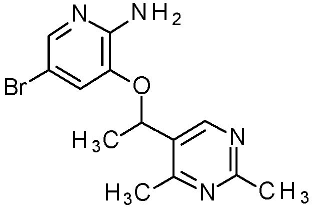

5-{1-[(5-Bromo-2-nitropyridin-3-yl)oxy]ethyl}-2,4-dimethylpyrimidine (enantiomer 1) (414 mg, 1.17 mmol) was dissolved in ethanol (10 mL). Acetic acid (3.1 mL) and iron (393 mg, 7.03 mmol) were added and the mixture was stirred for 3 h at 80 °C. The reaction mixture was allowed to cool down, diluted with ethyl acetate and filtered. The filtrate was concentrated and diluted in ethyl acetate again. The organic phase was washed with saturated sodium bicarbonate solution and water, dried and concentrated. The residue was purified by column chromatography (silica gel, dichloromethane / ethanol (0 - 10 %) gradient) to give 383 mg (101 % yield) of the title compound.
[α]²⁰_{D}: +4.1° (c = 1.00, methanol)
LC-MS (Method 1): Rt = 0.89 min; MS (ESlpos): m/z = 323 [M+H]⁺
¹H NMR (400 MHz, DMSO-*d*₆) δ ppm= 1.56 (d, 3 H), 2.53 (s, 3 H), 5.78 (q, 1 H), 6.13 (s, 2 H), 7.22 (d, 1 H), 7.55 (d, 1 H), 8.73 (s, 1 H).

### Intermediate 431

### (rac)-5-bromo-3-{1-[4-(methanesulfonyl)phenyl]ethoxy}pyridin-2-amine

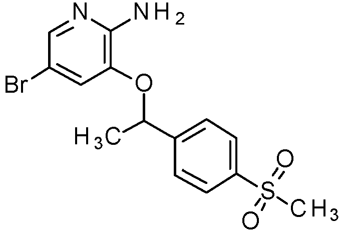

(Rac)-5-Bromo-3-{1-[4-(methanesulfonyl)phenyl]ethoxy}-2-nitropyridine (450 mg, 1.12 mmol) was dissolved in methanol (5.5 mL) and acetic acid (5.5 mL). Iron (188 mg, 3.36 mmol) was added and the mixture was stirred for 1 h at 85 °C. The reaction mxture was allowed to cool down, filtered and concentrated. The residue was purified by column chromatography (silica gel, hexane / ethyl acetate (0 - 100 %) gradient) to give 298 mg (72 % yield) of the title compound.
LC-MS (Method 1): Rt = 0.98 min; MS (ESlpos): m/z = 371 [M+H]⁺
¹H-NMR (400 MHz, DMSO-d6) δ [ppm]: 1.154 (1.42), 1.172 (3.08), 1.190 (1.51), 1.326 (0.51), 1.343 (0.53), 1.553 (4.75), 1.569 (4.77), 1.987 (4.26), 2.518 (2.30), 2.523 (1.55), 3.188 (1.34), 3.214 (16.00), 4.017 (0.96), 4.035 (0.98), 5.736 (1.03), 5.751 (1.04), 6.125 (2.81), 7.137 (2.23), 7.142 (2.28), 7.516 (3.93), 7.521 (3.87), 7.735 (2.88), 7.740 (0.97), 7.752 (1.07), 7.756 (3.74), 7.902 (0.59), 7.906 (4.29), 7.912 (1.20), 7.924 (1.04), 7.928 (3.40), 7.932 (0.44).

### Borylation

### Intermediate 432

### {6-amino-5-[1-(6-methylpyridin-2-yl)ethoxy]pyridin-3-yl}boronic acid (enantiomer 1)

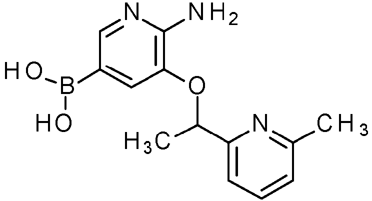

5-Bromo-3-[1-(6-methylpyridin-2-yl)ethoxy]pyridin-2-amine (enantiomer 1) **(Intermediate 376 and Intermediate 377,** 96.0 mg, 312 µmol), 4,4,4',4',5,5,5',5'-octamethyl-2,2'-bi-1,3,2-dioxaborolane (94.9 mg, 374 µmol) and potassium acetate (91.7 mg, 935 µmol) were suspended in 1,4-dioxane (1.9 mL) and the reaction mixture was degassed. Pd(dppf)Cl₂ (11.4 mg, 15.6 µmol) was added and the mixture was stirred under argon for 2 h at 100 °C. The reaction mixture was cooled to rt, diluted with ethyl acetate and filtered. The filtrate was washed with water and brine, dried over sodium sulfate, filtered and concentrated under reduced pressure to give 182 mg (22 % purity, 47 % yield) of crude material that was used without further purification in the next step.
LC-MS (Method 1): Rt = 0.56 min; MS (ESlpos): m/z = 274 [M+H]⁺

### Intermediate 433

### {6-amino-5-[1-(6-methylpyridin-2-yl)ethoxy]pyridin-3-yl}boronic acid (enantiomer 2)

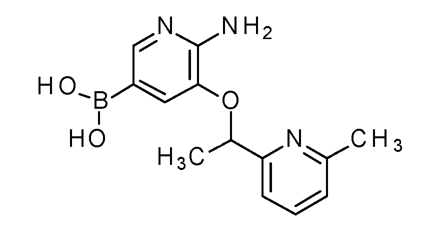

The compound was prepared similarly to **Intermediate 432** starting from 5-bromo-3-[1-(6-methylpyridin-2-yl)ethoxy]pyridin-2-amine (enantiomer 2) **(Intermediate 376 and Intermediate 377,** 104 mg, 337 µmol) to give 158 mg (35 % purity, 60 % yield) of the title compound.
LC-MS (Method 1): Rt = 0.56 min; MS (ESlpos): m/z = 274 [M+H]⁺

### Intermediate 434

### {6-amino-5-[1-(5-methylpyridin-2-yl)ethoxy]pyridin-3-yl}boronic acid (enantiomer 1)

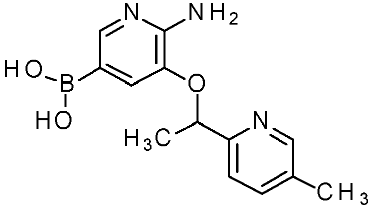

5-Bromo-3-[1-(5-methylpyridin-2-yl)ethoxy]pyridin-2-amine (enantiomer1) **(Intermediate 378 and Intermediate 379,** 76.0 mg, 247 µmol), 4,4,4',4',5,5,5',5'-octamethyl-2,2'-bi-1,3,2-dioxaborolane (75 mg, 295 µmol) and potassium acetate (72.6 mg, 739 µmol) were suspended in 1,4-dioxane (1.3 mL) and the reaction mixture was degassed. Pd(dppf)Cl₂ (9 mg, 12 µmol) was added and the mixture was stirred under argon for 2 h at 100 °C. 4,4,4',4',5,5,5',5'-Octamethyl-2,2'-bi-1,3,2-dioxaborolane (25 mg, 73.7 µmol) and Pd(dppf)Cl₂ (5 mg, 6.6 µmol) were added again and the mixture was stirred for 3 h at 100 °C. The reaction mixture was allowed to cool down, diluted with ethyl acetate and filtered. The filtrate was washed with water, brine, dried over sodium sulfate, filtered and concentrated under reduced pressure to give 161 mg (25 % purity, 60 % yield) of the title compound which was used without further purification in the next step.
LC-MS (Method 1): Rt = 0.60 min; MS (ESlpos): m/z = 274 [M+H]⁺

### Intermediate 435

### {6-amino-5-[1-(5-methylpyridin-2-yl)ethoxy]pyridin-3-yl}boronic acid (enantiomer 2)

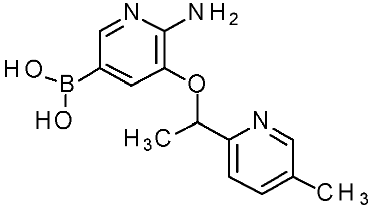

The compound was prepared similarly to **Intermediate 434** starting from 5-bromo-3-[1-(5-methylpyridin-2-yl)ethoxy]pyridin-2-amine (enantiomer 2) **(Intermediate 378 and Intermediate 379,** 80.0 mg, 260 µmol) to give 178.6 mg (25% purity, 63 % yield) of the title compound.
LC-MS (Method 1): Rt = 0.60 min; MS (ESlpos): m/z = 274 [M+H]⁺

### Intermediate 436

### {6-amino-5-[1-(4-methylpyridin-2-yl)ethoxy]pyridin-3-yl}boronic acid (enantiomer 1)

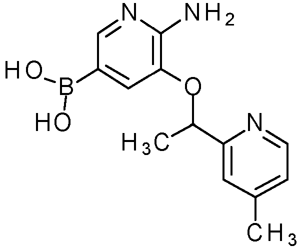

5-Bromo-3-[1-(4-methylpyridin-2-yl)ethoxy]pyridin-2-amine (enantiomer 1) **(Intermediate 380 and Intermediate 381,** 55.0 mg, 178 µmol), 4,4,4',4',5,5,5',5'-octamethyl-2,2'-bi-1,3,2-dioxaborolane (54 mg, 214 µmol) and potassium acetate (52.5 mg, 535 µmol) were suspended in 1,4-dioxane (1 mL) and the reaction mixture was degassed. Pd(dppf)Cl₂ (6.5 mg, 8.9 µmol) was added and the mixture was stirred under argon for 2 h at 100 °C. 4,4,4',4',5,5,5',5'-Octamethyl-2,2'-bi-1,3,2-dioxaborolane (54 mg, 214 µmol) and Pd(dppf)Cl₂ (6.5 mg, 8.9 µmol) were added again and the mixture was stirred for 3 h at 100 °C. The reaction mixture was allowed to cool down, diluted with ethyl acetate and filtered. The filtrate was washed with water and brine, dried over sodium sulfate, filtered and concentrated under reduced pressure to give 142.7 mg (20 % purity, 58 % yield) of the title compound.
LC-MS (Method 1): Rt = 0.58 min; MS (ESlpos): m/z = 274 [M+H]⁺

### Intermediate 437

### {6-amino-5-[1-(4-methylpyridin-2-yl)ethoxy]pyridin-3-yl}boronic acid (enantiomer 2)

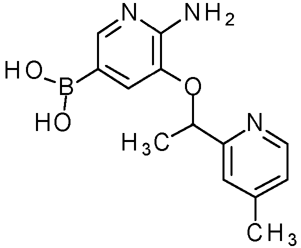

The compound was prepared similarly to **Intermediate 436** starting from 5-bromo-3-[1-(4-methylpyridin-2-yl)ethoxy]pyridin-2-amine (enantiomer 2) **(Intermediate 380 and Intermediate 381,** 54.0 mg, 175 µmol) to give 143.5 mg (20 % purity, 60 % yield) of the title compound.
LC-MS (Method 1): Rt = 0.58 min; MS (ESlpos): m/z = 274 [M+H]⁺

### Intermediate 438

### {6-amino-5-[1-(3-methylpyridin-2-yl)ethoxy]pyridin-3-yl}boronic acid (enantiomer 1)

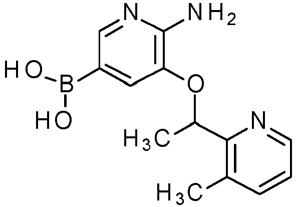

5-Bromo-3-[1-(3-methylpyridin-2-yl)ethoxy]pyridin-2-amine (enantiomer 1) **(Intermediate 382 and Intermediate 383,** 34.0 mg, 110 µmol), 4,4,4',4',5,5,5',5'-octamethyl-2,2'-bi-1,3,2-dioxaborolane (34 mg, 132 µmol) and potassium acetate (32.4 mg, 331 µmol) were suspended in 1,4-dioxane (0.5 mL) and the reaction mixture was degassed. Pd(dppf)Cl₂ (4 mg, 5.5 µmol) was added and the mixture was stirred under argon for 2 h at 100 °C. 4,4,4',4',5,5,5',5'-Octamethyl-2,2'-bi-1,3,2-dioxaborolane (34 mg, 110 µmol) and Pd(dppf)Cl₂ (4 mg, 5.5 µmol) were added again and the mixture was stirred for 3 h at 100 °C. The reaction mixture was allowed to cool down, diluted with ethyl acetate and filtered. The filtrate was washed with water, brine, dried over sodium sulfate, filtered and concentrated under reduced pressure to give 87.6 mg (15 % purity, 43 % yield) of the title compound.
LC-MS (Method 1): Rt = 0.56 min; MS (ESlpos): m/z = 274 [M+H]⁺

### Intermediate 439

### {6-amino-5-[1-(3-methylpyridin-2-yl)ethoxy]pyridin-3-yl}boronic acid (enantiomer 2)

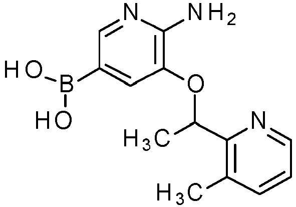

The compound was prepared similarly to **Intermediate 438** starting from 5-bromo-3-[1-(3-methylpyridin-2-yl)ethoxy]pyridin-2-amine (enantiomer 2) **(Intermediate 382 and Intermediate 383,** 34.0 mg, 110 µmol) to give 83 mg (20 % purity, 55 % yield) of the tilte compound.
LC-MS (Method 1): Rt = 0.55 min; MS (ESlpos): m/z = 274 [M+H]⁺

### Intermediate 440

### 3-[1-(1,4-dimethyl-1H-pyrazol-5-yl)ethoxy]-5-(4,4,5,5-tetramethyl-1,3,2-dioxaborolan-2-yl)pyridin-2-amine (enantiomer 1)

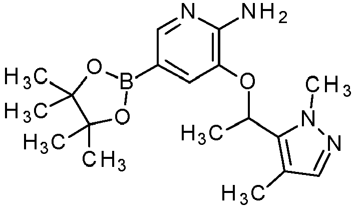

5-Bromo-3-[1-(1,4-dimethyl-1H-pyrazol-5-yl)ethoxy]pyridin-2-amine (enantiomer 1) **(Intermediate 384 and Intermediate 385,** 88.0 mg, 283 µmol), 4,4,4',4',5,5,5',5'-octamethyl-2,2'-bi-1,3,2-dioxaborolane (86 mg, 339 µmol) and potassium acetate (83 mg, 848 µmol) were suspended in 1,4-dioxane (1.9 mL) and the reaction mixture was degassed. Pd(dppf)Cl₂ (10 mg, 14 µmol) was added and the mixture was stirred under argon for 2 h at 100 °C. The reaction mixture was allowed to cool down, diluted with ethyl acetate and filtered. The filtrate was washed with water, brine, dried over sodium sulfate, filtered and concentrated under reduced pressure to give 135 mg (50 % purity, 66 % yield) of the title compound.
LC-MS (Method 1): Rt = 0.92 min; MS (ESlpos): m/z = 359 [M+H]⁺

### Intermediate 441

### {6-amino-5-[1-(1,4-dimethyl-1H-pyrazol-5-yl)ethoxy]pyridin-3-yl}boronic acid (enantiomer 1)

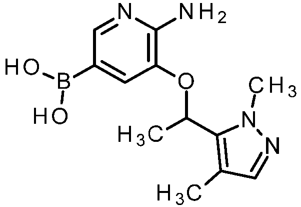

5-Bromo-3-[1-(1,4-dimethyl-1H-pyrazol-5-yl)ethoxy]pyridin-2-amine (enantiomer 2) **(Intermediate 384 and Intermediate 385,** 72.0 mg, 231 µmol), 4,4,4',4',5,5,5',5'-octamethyl-2,2'-bi-1,3,2-dioxaborolane (70.5 mg, 278 µmol) and potassium acetate (68.1 mg, 694 µmol) were suspended in 1,4-dioxane (1.6 mL) and the reaction mixture was degassed. Pd(dppf)Cl₂ (8.46 mg, 11.6 µmol) was added and the mixture was stirred under argon for 2 h at 100 °C. 4,4,4',4',5,5,5',5'-octamethyl-2,2'-bi-1,3,2-dioxaborolane (35 mg, 139 µmol) and Pd(dppf)Cl₂ (5 mg, 6.8 µmol) were added again and the mixture was stirred for 1 h at 100 °C. The reaction mixture was allowed to cool down, diluted with ethyl acetate and filtered. The filtrate was washed with water, brine, dried over sodium sulfate, filtered and concentrated under reduced pressure to give 167 mg (20 % purity, 52 % yield) of the title compound.
LC-MS (Method 1): Rt = 0.53 min; MS (ESlpos): m/z = 277 [M+H]⁺

### Intermediate 442

### 3-[1-(1,4-dimethyl-1H-pyrazol-5-yl)ethoxy]-5-(4,4,5,5-tetramethyl-1,3,2-dioxaborolan-2-yl)pyridin-2-amine (enantiomer 2)

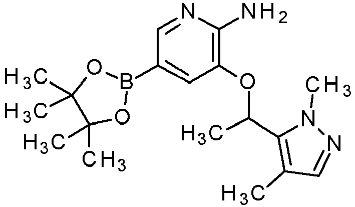

The compound was prepared similarly to **Intermediate 440** starting from 5-bromo-3-[1-(1,4-dimethyl-1H-pyrazol-5-yl)ethoxy]pyridin-2-amine (enantiomer 2) **(Intermediate 384 and Intermediate 385,** 69.0 mg, 222 µmol) to give 110 mg (50 % purity, 69 % yield) of the title compound.
LC-MS (Method 1): Rt = 0.92 min; MS (ESlpos): m/z = 359 [M+H]⁺

### Intermediate 443

### {6-amino-5-[1-(1,4-dimethyl-1H-pyrazol-5-yl)ethoxy]pyridin-3-yl}boronic acid (enantiomer 2)

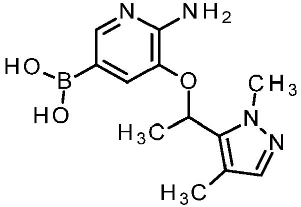

The compound was prepared similarly to **Intermediate 440** starting from 5-bromo-3-[1-(1,4-dimethyl-1H-pyrazol-5-yl)ethoxy]pyridin-2-amine (enantiomer 2) **(Intermediate 384 and Intermediate 385,** 69.0 mg, 222 µmol) to give 115 mg (25 % purity, 50 % yield) of the title compound.
LC-MS (Method 1): Rt = 0.54 min; MS (ESlpos): m/z = 277 [M+H]⁺

### Intermediate 444

### {6-amino-5-[(1S)-1-(2,6-difluorophenyl)ethoxy]pyridin-3-yl}boronic acid

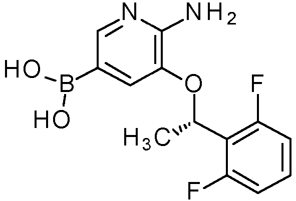

5-Bromo-3-[(1S)-1-(2,6-difluorophenyl)ethoxy]pyridin-2-amine (230 mg, 699 µmol), 4,4,4',4',5,5,5',5'-octamethyl-2,2'-bi-1,3,2-dioxaborolane (213 mg, 839 µmol) and potassium acetate (206 mg, 2.10 mmol) were suspended in 1,4-dioxane (4.9 mL) and the reaction mixture was degassed. Pd(dppf)Cl₂ (25.6 mg, 34.9 µmol) was added and the mixture was stirred under argon for 2 h at 100 °C. The reaction mixture was allowed to cool down, diluted with ethyl acetate and filtered. The filtrate was washed with water and brine, dried over sodium sulfate, filtered and concentrated under reduced pressure to give 464 mg (30 % purity, 67 % yield) of the title compound.
LC-MS (Method 1): Rt = 0.78 min; MS (ESlpos): m/z = 295 [M+H]⁺

### Intermediate 445

### {6-amino-5-[(1R)-1-(5-fluoropyridin-2-yl)ethoxy]pyridin-3-yl}boronic acid

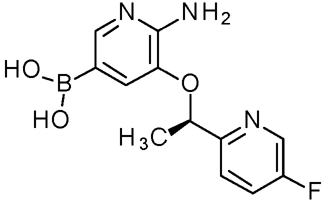

5-Bromo-3-[(1R)-1-(5-fluoropyridin-2-yl)ethoxy]pyridin-2-amine (198 mg, 634 µmol), 4,4,4',4',5,5,5',5'-octamethyl-2,2'-bi-1,3,2-dioxaborolane (193 mg, 761 µmol) and potassium acetate (187 mg, 1.90 mmol) were suspended in 1,4-dioxane (4.4 mL) and the reaction mixture was degassed. Pd(dppf)Cl₂ (23.2 mg, 31.7 µmol) was added and the mixture was stirred under argon for 2 h at 100 °C. The reaction mixture was allowed to cool down, diluted with ethyl acetate and filtered. The filtrate was washed with water and brine, dried over sodium sulfate, filtered and concentrated under reduced pressure to give 423 mg (33 % purity, 79 % yield) of the title compound.
LC-MS (Method 1): Rt = 0.55 min; MS (ESlpos): m/z = 278 [M+H]⁺

### Intermediate 446

### {6-amino-5-[(1R)-1-(3-fluoropyridin-2-yl)ethoxy]pyridin-3-yl}boronic acid

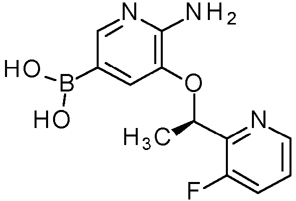

The compound was prepared similarly to **Intermediate 445** starting from 5-bromo-3-[(1R)-1-(3-fluoropyridin-2-yl)ethoxy]pyridin-2-amine (193 mg, 618 µmol) to give 383 mg (35 % purity, 78 % yield) of the title compound.
LC-MS (Method 1): Rt = 0.53 min; MS (ESlpos): m/z = 278 [M+H]⁺

### Intermediate 447

### {6-amino-5-[(1R)-1-(2,6-difluorophenyl)ethoxy]pyridin-3-yl}boronic acid

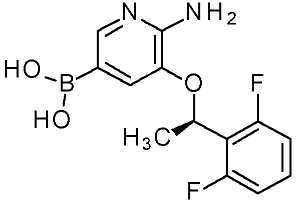

The compound was prepared similarly to **Intermediate 445** starting from 5-bromo-3-[(1R)-1-(2,6-difluorophenyl)ethoxy]pyridin-2-amine (204 mg, 620 µmol) to give 154 mg (32 % purity, 68 % yield) of the title compound.
LC-MS (Method 1): Rt = 0.78 min; MS (ESlpos): m/z = 295 [M+H]⁺

### Intermediate 448

### (rac)-2-(1-{[2-amino-5-(4,4,5,5-tetramethyl-1,3,2-dioxaborolan-2-yl)pyridin-3-yl]oxy}ethyl)benzonitrile

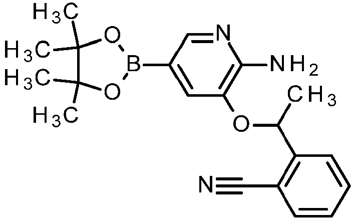

(Rac)-2-{1-[(2-Amino-5-bromopyridin-3-yl)oxy]ethyl}benzonitrile (142 mg, 446 µmol), 4,4,4',4',5,5,5',5'-octamethyl-2,2'-bi-1,3,2-dioxaborolane (125 mg, 491 µmol) and potassium acetate (131 mg, 1.34 mmol) were suspended in 1,4-dioxane (7 mL) and the reaction mixture was degassed. Pd(dppf)Cl₂ (32.7 mg, 44.6 µmol) was added and the mixture was stirred under argon for 2 h at 100 °C. The cold reaction mixture was allowed to cool down, diluted with ethyl acetate and water. The aqueous phase was extracted 3 times with ethyl acetate. The combined organic layers were dried over a hydrophobic filter and concentrated to give 321 mg (40 % purity, 79 % yield) of the title compound.
LC-MS (Method 1): Rt = 0.65 min; MS (ESlpos): m/z = 284 [M+H]⁺
¹H-NMR (400 MHz, DMSO-d6) δ [ppm]: 1.066 (3.03), 1.156 (16.00), 1.164 (8.36), 1.221 (1.61), 1.229 (1.66), 1.292 (0.42), 1.610 (0.49), 1.626 (0.50), 1.642 (0.34), 1.657 (0.32), 1.906 (0.59), 2.518 (0.27), 2.523 (0.16), 3.565 (3.34), 3.940 (0.32), 6.279 (0.19), 7.015 (0.22), 7.017 (0.22), 7.502 (0.21), 7.505 (0.20), 7.521 (0.18), 7.524 (0.17), 7.559 (0.18), 7.564 (0.17), 7.728 (0.20), 7.788 (0.39), 7.791 (0.29), 7.943 (0.63).

### Intermediate 449

### {6-amino-5-[(1R)-1-(3-cyanophenyl)ethoxy]pyridin-3-yl}boronic acid

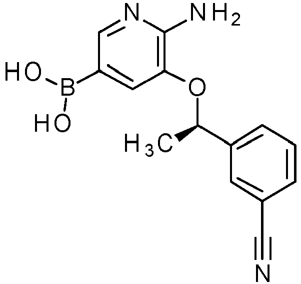

3-{(1R)-1-[(2-Amino-5-bromopyridin-3-yl)oxy]ethyl}benzonitrile (133 mg, 418 µmol), 4,4,4',4',5,5,5',5'-octamethyl-2,2'-bi-1,3,2-dioxaborolane (127 mg, 502 µmol) and potassium acetate (123 mg, 1.25 mmol) were suspended in 1,4-dioxane (2.9 mL) and the reaction mixture was degassed. Pd(dppf)Cl₂ (15.3 mg, 20.9 µmol) was added and the mixture was stirred under argon for 2 h at 100 °C. The reaction mixture was allowed to cool down, diluted with ethyl acetate and filtered. The filtrate was washed with water and brine, dried over sodium sulfate, filtered and concentrated under reduced pressure to give 223 mg (38 % purity, 72 % yield) of the title compound.
LC-MS (Method 1): Rt = 0.68 min; MS (ESlpos): m/z = 284 [M+H]⁺

### Intermediate 450

### {6-amino-5-[(1R)-1-(4-cyanophenyl)ethoxy]pyridin-3-yl}boronic acid

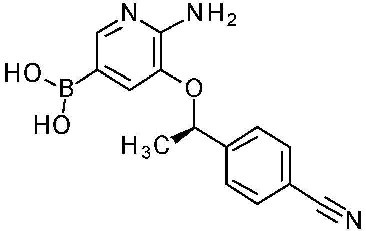

The compound was prepared similarly to **Intermediate 449** starting from 4-{(1R)-1-[(2-amino-5-bromopyridin-3-yl)oxy]ethyl}benzonitrile (117 mg, 368 µmol) to give 176 mg (45 % purity, 76 % yield) of the title compound.
LC-MS (Method 1): Rt = 0.64 min; MS (ESlpos): m/z = 284 [M+H]⁺

### Intermediate 451

### {6-amino-5-[(1R)-1-(3,5-difluoropyridin-4-yl)ethoxy]pyridin-3-yl}boronic acid

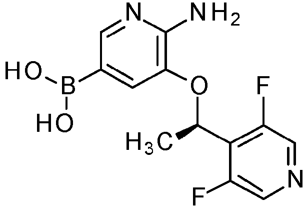

The compound was prepared similarly to **Intermediate 449** starting from 5-bromo-3-[(1R)-1-(3,5-difluoropyridin-4-yl)ethoxy]pyridin-2-amine (163 mg, 494 µmol) to give 256 mg (43 % purity, 75 % yield) of the title compound.
LC-MS (Method 1): Rt = 0.55 min; MS (ESlpos): m/z = 296 [M+H]⁺

### Intermediate 452

### (rac)-[6-amino-5-(1-phenylpropoxy)pyridin-3-yl]boronic acid

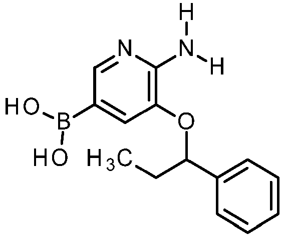

(Rac)-5-Bromo-3-(1-phenylpropoxy)pyridin-2-amine (320 mg, 1.04 mmol), 4,4,4',4',5,5,5',5'-octamethyl-2,2'-bi-1,3,2-dioxaborolane (317 mg, 1.25 mmol) and potassium acetate (307 mg, 3.12 mmol) were suspended in 1,4-dioxane (9.7 mL) and the reaction mixture was degassed. Pd(dppf)Cl₂ (76.2 mg, 104 µmol) was added and the mixture was stirred under argon for 2 h at 100 °C. The reaction mixture was allowed to cool down, diluted with ethyl acetate and water. The aqueous phase was extracted 3 times with ethyl acetate. The combined organic layers were dried over a hydrophobic filter and concentrated to give 551.1 mg (55 % purity, 106 % yield) of the title compound.
LC-MS (Method 1): Rt = 0.84 min; MS (ESlpos): m/z = 273 [M+H]⁺

### Intermediate 453

### {6-amino-5-[(1S)-1-(3-cyanophenyl)ethoxy]pyridin-3-yl}boronic acid

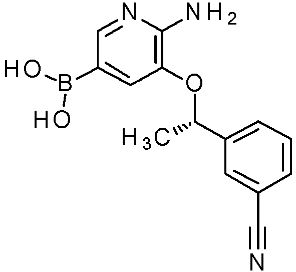

3-{(1S)-1-[(2-Amino-5-bromopyridin-3-yl)oxy]ethyl}benzonitrile (164 mg, 515 µmol), 4,4,4',4',5,5,5',5'-octamethyl-2,2'-bi-1,3,2-dioxaborolane (157 mg, 619 µmol) and potassium acetate (152 mg, 1.55 mmol) were suspended in 1,4-dioxane (3.6 mL) and the reaction mixture was degassed. Pd(dppf)Cl₂ (18.9 mg, 25.8 µmol) was added and the mixture was stirred under argon for 2 h at 100 °C. The reaction mixture was allowed to cool down, diluted with ethyl acetate and filtered. The filtrate was washed with water and brine. The organic phase was dried over sodium sufate, filtered and concentrated to give 260 mg (41 % purity, 73 % yield) of the title compound.
LC-MS (Method 1): Rt = 0.65 min; MS (ESlpos): m/z = 284 [M+H]⁺

### Intermediate 454

### {6-amino-5-[(1S)-1-(4-cyanophenyl)ethoxy]pyridin-3-yl}boronic acid

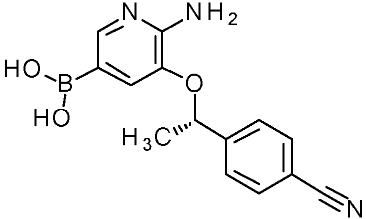

The compound was prepared similarly to **Intermediate 453** starting from 4-{(1S)-1-[(2-amino-5-bromopyridin-3-yl)oxy]ethyl}benzonitrile (128 mg, 402 µmol) to give 210 mg (40 % purity, 73 % yield) of the title compound.
LC-MS (Method 1): Rt = 0.64 min; MS (ESlpos): m/z = 284 [M+H]⁺

### Intermediate 455

### {6-amino-5-[(1S)-1-(3,5-difluoropyridin-4-yl)ethoxy]pyridin-3-yl}boronic acid

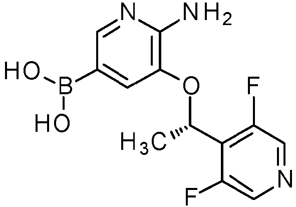

The compound was prepared similarly to **Intermediate 453** starting from 5-bromo-3-[(1S)-1-(3,5-difluoropyridin-4-yl)ethoxy]pyridin-2-amine (150 mg, 454 µmol) to give 241 mg (41 % purity, 74 % yield) of the title compound.
LC-MS (Method 1): Rt = 0.55 min; MS (ESlpos): m/z = 296 [M+H]⁺

### Intermediate 456

### {6-amino-5-[(1 R)-1-phenylethoxy]pyridin-3-yl}boronic acid

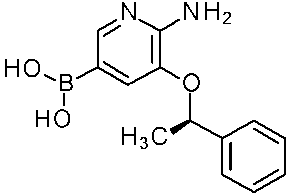

5-Bromo-3-[(1R)-1-phenylethoxy]pyridin-2-amine (219 mg, 748 µmol), 4,4,4',4',5,5,5',5'-octamethyl-2,2'-bi-1,3,2-dioxaborolane (228 mg, 897 µmol) and potassium acetate (220 mg, 2.24 mmol) were suspended in degassed 1,4-dioxane (7 mL) under nitrogen. Pd(dppf)Cl₂ (52.5 mg, 74.8 µmol) was added and the mixture was stirred for 2 h at 100 °C. The reaction mixture was allowed to cool down, diluted with ethyl acetate and water. The aqueous phase was extracted 3 times with ethyl acetate. The combined organic layers were dried over a hydrophobic filter and concentrated to give 396.9 mg (45 % purity, 92 % yield) of the title compound.
LC-MS (Method 1): Rt = 0.71 min; MS (ESlpos): m/z = 259 [M+H]⁺

### Intermediate 457

### (rac)-{6-amino-5-[1-(1-methyl-1H-pyrazol-5-yl)ethoxy]pyridin-3-yl}boronic acid

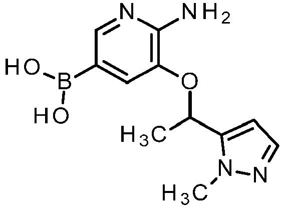

(Rac)-5-Bromo-3-[1-(1-methyl-1H-pyrazol-5-yl)ethoxy]pyridin-2-amine (315 mg, 1.06 mmol), 4,4,4',4',5,5,5',5'-octamethyl-2,2'-bi-1,3,2-dioxaborolane (323 mg, 1.27 mmol) and potassium acetate (312 mg, 3.18 mmol) were suspended in degassed 1,4-dioxane (7 mL). Pd(dppf)Cl₂ (38.8 mg, 53.0 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated to give 454 mg (45 % purity, 73 % yield) of the title compound.
LC-MS (Method 1): Rt = 0.54 min; MS (ESlpos): m/z = 263 [M+H]⁺

### Intermediate 458

### {6-amino-5-[(1R)-1-(2-fluorophenyl)ethoxy]pyridin-3-yl}boronic acid

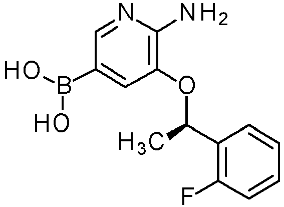

5-Bromo-3-[(1R)-1-(2-fluorophenyl)ethoxy]pyridin-2-amine (457 mg, 85 % purity, 1.25 mmol), 4,4,4',4',5,5,5',5'-octamethyl-2,2'-bi-1,3,2-dioxaborolane (380 mg, 1.50 mmol) and potassium acetate (368 mg, 3.75 mmol) were suspended in degassed 1,4-dioxane (5.9 mL). Pd(dppf)Cl₂ (38.8 mg, 53.0 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated to give 825 mg (25 % purity, 60 % yield) of the title compound.
LC-MS (Method 1): Rt = 0.75 min; MS (ESlpos): m/z = 277 [M+H]⁺

### Intermediate 459

### (6-amino-5-{methyl[(1R)-1-phenylethyl]amino}pyridin-3-yl)boronic acid

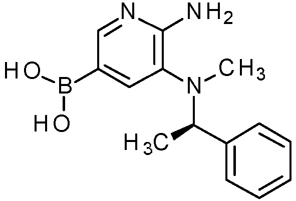

5-Bromo-N³-methyl-N³-[(1R)-1-phenylethyl]pyridine-2,3-diamine (100 mg, 90 % purity, 294 µmol), 4,4,4',4',5,5,5',5'-octamethyl-2,2'-bi-1,3,2-dioxaborolane (89.6 mg, 353 µmol) and potassium acetate (86.5 mg, 882 µmol) were suspended in 1,4-dioxane (2.1 mL) and degassed. Pd(dppf)Cl₂ (10.8 mg, 14.7 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated to give 258 mg (17 % purity, 55 % yield) of the title compound.
LC-MS (Method 1): Rt = 0.85 min; MS (ESlpos): m/z = 272 [M+H]⁺

### Intermediate 460

### {6-amino-5-[(1R)-1-(4-methylpyridin-2-yl)ethoxy]pyridin-3-yl}boronic acid

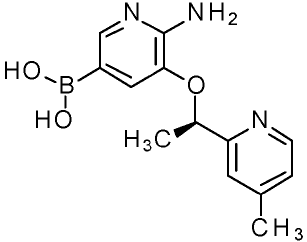

5-Bromo-3-[(1R)-1-(4-methylpyridin-2-yl)ethoxy]pyridin-2-amine (183 mg, 594 µmol), 4,4,4',4',5,5,5',5'-octamethyl-2,2'-bi-1,3,2-dioxaborolane (181 mg, 713 µmol) and potassium acetate (175 mg, 1.78 µmol) were suspended in 1,4-dioxane (2.8 mL) and degassed. Pd(dppf)Cl₂ (20.8 mg, 29.7 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated to give 375 mg (34 % purity, 78 % yield) of the title compound.
LC-MS (Method 1): Rt = 0.62 min; MS (ESlpos): m/z = 274 [M+H]⁺

### Intermediate 461

### {6-amino-5-[(1R)-1-(6-methylpyridin-2-yl)ethoxy]pyridin-3-yl}boronic acid

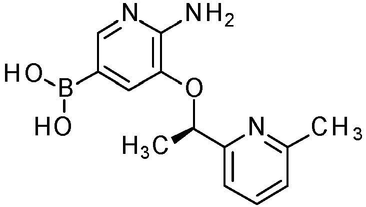

5-Bromo-3-[(1R)-1-(6-methylpyridin-2-yl)ethoxy]pyridin-2-amine (120 mg, 389 µmol), 4,4,4',4',5,5,5',5'-octamethyl-2,2'-bi-1,3,2-dioxaborolane (119 mg, 467 µmol) and potassium acetate (115 mg, 1.17 mmol) were suspended in 1,4-dioxane (1.8 mL) and degassed. Pd(dppf)Cl₂ (13.7 mg, 19.5 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated to give 219 mg (33 % purity, 68 % yield) of the title compound.
LC-MS (Method 1): Rt = 0.59 min; MS (ESlpos): m/z = 274 [M+H]⁺

### Intermediate 462

### (rac)-3-[1-(1,3-oxazol-2-yl)ethoxy]-5-(4,4,5,5-tetramethyl-1,3,2-dioxaborolan-2-yl)pyridin-2-amine

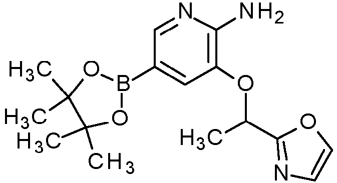

(Rac)-5-Bromo-3-[1-(1,3-oxazol-2-yl)ethoxy]pyridin-2-amine (105 mg, 370 µmol) and 4,4,4',4',5,5,5',5'-octamethyl-2,2'-bi-1,3,2-dioxaborolane (113 mg, 443 µmol) were dissolved in 1,4-dioxane (1.9 mL) and degassed. Potassium acetate (109 mg, 1.11 mmol) and Pd(dppf)Cl₂ (13.5 mg, 18.5 µmol) were added and the mixture was stirred for 3 h at 100 °C.The reaction mixture was allowed to cool down, diluted with ethyl acetate and filtered. The filtrate was washed with water and brine, dried over magnesium sulfate, filtered and concentrated to give 182 mg (48 % purity, 71 % yield) of the title compound.
LC-MS (Method 1): Rt = 0.81 min; MS (ESlpos): m/z = 332 [M+H]⁺
¹H-NMR (400MHz, DMSO-d₆): δ [ppm]= 1.25 (s, 12H), 1.66 (d, 3H), 5.56 - 5.63 (m, 1H), 6.14 (br s, 2H), 7.17 (s, 1H), 7.22 (s, 1H), 7.83 (d, 1H), 8.12 (s, 1H).

### Intermediate 463

### {6-amino-5-[(1R)-1-(3-chlorophenyl)ethoxy]pyridin-3-yl}boronic acid

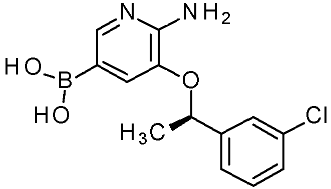

5-Bromo-3-[(1R)-1-(3-chlorophenyl)ethoxy]pyridin-2-amine (245 mg, 94 % purity, 703 µmol), 4,4,4',4',5,5,5',5'-octamethyl-2,2'-bi-1,3,2-dioxaborolane (234 mg, 99 % purity, 914 µmol) and potassium acetate (207 mg, 2.11 mmol) were dissolved in 1,4-dioxane (15 mL) under argon. Pd(dppf)Cl₂dichloromethane complex (57.4 mg, 70.3 µmol) was added and stirred for 4 h at 100 °C. The reaction mixture was allowed to cool down, concentrated, diluted with ethyl acetate and filtered over celite. The organic phase was washed with water and brine, dried over a hydrophobic filter and concentrated. The residue was pufified by preparative HPLC to give 79.9 mg (88 % purity, 27 % yield) of th title compound.
LC-MS (Method 1): Rt = 0.86 min; MS (ESlpos): m/z = 293 [M+H]⁺

### Intermediate 464

### {6-amino-5-[(1R)-1-(2-methoxyphenyl)ethoxy]pyridin-3-yl}boronic acid

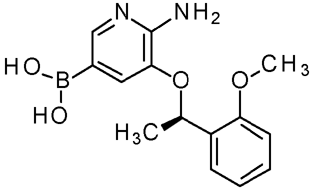

5-Bromo-3-[(1R)-1-(2-methoxyphenyl)ethoxy]pyridin-2-amine (380 mg, 1.18 mmol), 4,4,4',4',5,5,5',5'-octamethyl-2,2'-bi-1,3,2-dioxaborolane (392 mg, 99 % purity, 1.53 mmol) and potassium acetate (346 mg, 3.53 mmol) were dissoved in 1,4-dioxane (25 mL) under nitrogen. Pd(dppf)Cl₂ dichloromethane complex (96.0 mg, 118 µmol) was added and the mixture was stirred for 23 h at 100 °C. The reaction mixture was allowed to cool down, concentrated, diluted with ethyl acetate and filtered over celite. The organic phase was washed with water and brine, dried over a hydrophobic filter and concentrated. The residue was purified by preparative HPLC to give 142 mg (63 % purity, 20 % yield) of the title compound.
LC-MS (Method 1): Rt = 0.81 min; MS (ESlpos): m/z = 289 [M+H]⁺

### Intermediate 465

### {6-amino-5-[(1R)-1-(pyridin-3-yl)ethoxy]pyridin-3-yl}boronic acid

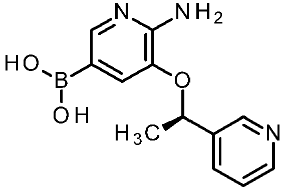

5-Bromo-3-[(1R)-1-(pyridin-3-yl)ethoxy]pyridin-2-amine (120 mg, 408 µmol), 4,4,4',4',5,5,5',5'-octamethyl-2,2'-bi-1,3,2-dioxaborolane (124 mg, 490 µmol) and potassium acetate (120 mg, 1.22 mmol) were dissolved in degassed 1,4-dioxane (1.9 mL). Pd(dppf)Cl₂ (14.3 mg, 20.4 µmol) was added and the mixture was stirred for 3 h at 100 °C. The reaction mixture was allowed to cool down, diluted with ethyl acetate and filtered. The filtrate was washed with water and brine, dried and concentrated to give 252 mg (13 % purity, 23 % yield) of the title compound.
LC-MS (Method 1): Rt = 0.48 min; MS (ESlpos): m/z = 260 [M+H]⁺
¹H-NMR (400 MHz, DMSO-d₆) δ [ppm]: 1.066 (3.27), 1.156 (16.00), 1.164 (0.87), 1.173 (0.21), 1.229 (1.93), 1.292 (0.38), 1.541 (0.39), 1.557 (0.39), 1.907 (0.45), 1.988 (0.35), 2.518 (0.61), 2.523 (0.46), 3.566 (9.98), 3.938 (0.52), 6.266 (0.17), 7.043 (0.16), 7.046 (0.16), 7.773 (0.23), 7.776 (0.23), 7.939 (1.14).

### Intermediate 466

### 5-(4,4,5,5-tetramethyl-1,3,2-dioxaborolan-2-yl)-3-[(1R)-1-(1,3-thiazol-2-yl)ethoxy]pyridin-2-amine

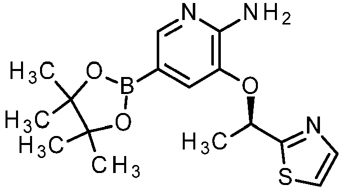

5-Bromo-3-[(1R)-1-(1,3-thiazol-2-yl)ethoxy]pyridin-2-amine (140 mg, 466 µmol) and 4,4,4',4',5,5,5',5'-octamethyl-2,2'-bi-1,3,2-dioxaborolane (142 mg, 560 µmol) were dissolved in degassed 1,4-dioxane (2.4 mL). Potassium acetate (137 mg, 1.40 mmol) and Pd(dppf)Cl₂ (17.1 mg, 23.3 µmol) were added and the mixture was stirred for 3 h at 100 °C. The reaction mixture was allowed to cool down, diluted with ethyl acetate and filtered. The filtrate was washed with water and brine, dried over magnesium sulfate, filtered and concentrated to give 254 mg (49 % purity, 76 % yield) of the title compound.
¹H-NMR (400MHz, DMSO-d₆): δ [ppm]= 1.23 (s, 12H), 1.68 (d, 3H), 5.82 (q, 1H), 6.22 (s, 2H), 7.11 (d, 1H), 7.71 (d, 1H), 7.79 (d, 1H), 7.82 (d, 1H).

### Intermediate 467

### 3-[(1R)-1-(3-fluorophenyl)ethoxy]-5-(4,4,5,5-tetramethyl-1,3,2-dioxaborolan-2-yl)pyridin-2-amine

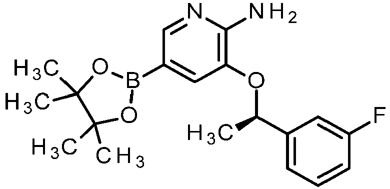

5-Bromo-3-[(1R)-1-(3-fluorophenyl)ethoxy]pyridin-2-amine (265 mg, 97 % purity, 826 µmol), 4,4,4',4',5,5,5',5'-octamethyl-2,2'-bi-1,3,2-dioxaborolane (275 mg, 99 % purity, 1.07 mmol) and potassium acetate (243 mg, 2.48 mmol) were dissolved in 1,4-dioxane (25 mL) under argon. Pd(dppf)Cl₂ (60.4 mg, 82.6 µmol) was added and the mixture was stirred for 3 h at 100 °C. The reacton mixture was allowed to cool down, concentrated, diluted with ethyl acetate and filtered over celite. The organic phase was washed with water and brine, dried over a hydrophobic filter and concentrated. The crude product was purified by preparative HPLC to give 104 mg (71 % purity, 25 % yield) of the title compound.

### Intermediate 468

### 3-[1-(1,3-oxazol-4-yl)ethoxy]-5-(4,4,5,5-tetramethyl-1,3,2-dioxaborolan-2-yl)pyridin-2-amine (enantiomer 1 and enantiomer 2)

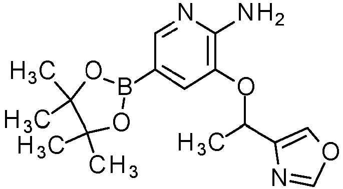

5-Bromo-3-[1-(1,3-oxazol-4-yl)ethoxy]pyridin-2-amine (140 mg, 493 µmol) and 4,4,4',4',5,5,5',5'-octamethyl-2,2'-bi-1,3,2-dioxaborolane (150 mg, 591 µmol) were dissolved in 1,4-dioxane (2.5 mL) and degassed. Potassium acetate (145 mg, 1.48 mmol) and Pd(dppf)Cl₂ (36.1 mg, 49.3 µmol) were added and the mixture was stirred for 3 h at 100 °C. The reaction mixture was allowed to cool down, diluted with ethyl acetate and filtered. The filtrate was washed with water and brine, dried over magnesium sulfate, filtered and concentrated to give 279 mg (51 % purity, 87 % yield) of the title compound.
LC-MS (Method 1): Rt = 0.78 min; MS (ESlpos): m/z = 332 [M+H]⁺
¹H-NMR (400MHz, DMSO-d₆): δ [ppm]= 1.25 (s, 12H), 1.56 (d, 3H), 5.39 (q, 1H), 6.12 (br s, 2H), 7.15 (s, 1H), 7.83 (s, 1H), 8.13 (s, 1H), 8.38 (d, 1H).

### Intermediate 469

### 3-[(1R)-1-(pyrimidin-2-yl)ethoxy]-5-(4,4,5,5-tetramethyl-1,3,2-dioxaborolan-2-yl)pyridin-2-amine

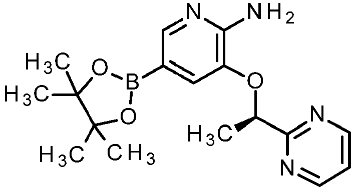

5-Bromo-3-[(1R)-1-(pyrimidin-2-yl)ethoxy]pyridin-2-amine (100 mg, 339 µmol) was dissoved in degassed 1,4-dioxane (3 mL). 4,4,4',4',5,5,5',5'-octamethyl-2,2'-bi-1,3,2-dioxaborolane (103 mg, 407 µmol), potassium acetate (99.7 mg, 1.0 mmol) and Pd(dppf)Cl₂ (24.7 mg, 34 µmol) were added and the mixture was stirred over night at 100 °C. The reaction mixture was allowed to cool down, filtered and used without purification in the next step.

### Intermediate 470

### 3-[(1R)-1-(pyridin-3-yl)ethoxy]-5-(4,4,5,5-tetramethyl-1,3,2-dioxaborolan-2-yl)pyridin-2-amine

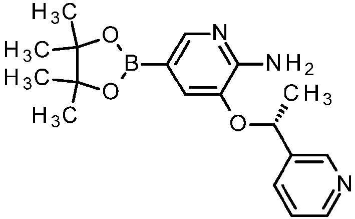

5-Bromo-3-[(1R)-1-(pyridin-3-yl)ethoxy]pyridin-2-amine (940 mg, 3.20 mmol) and 4,4,4',4',5,5,5',5'-octamethyl-2,2'-bi-1,3,2-dioxaborolane (893 mg, 3.52 mmol) were dissolved in 1,4-dioxane (28 mL) under argon. Potassium acetate (941 mg, 9.59 mmol) and Pd(dppf)Cl₂ complex with dichloromethane (261 mg, 320 µmol) were added and the mixture was stirred for 4.5 h at 100 °C. The reaction mixture was allowed to cool down, diluted with dichloromethane, filtered and concentrated to give 2.05 g (33 % purity, 62 % yield) of the title compound.

¹H-NMR (400 MHz, DMSO-d6) δ [ppm]: 1.065 (0.48), 1.155 (16.00), 1.228 (4.79), 1.256 (0.51), 1.291 (0.68), 1.541 (0.73), 1.556 (0.74), 1.900 (0.50), 3.565 (6.87), 6.267 (0.47), 7.044 (0.41), 7.774 (0.41), 7.945 (0.45).

### Intermediate 471

### 5-(4,4,5,5-tetramethyl-1,3,2-dioxaborolan-2-yl)-3-[(1R)-1-(1,2-thiazol-5-yl)ethoxy]pyridin-2-amine

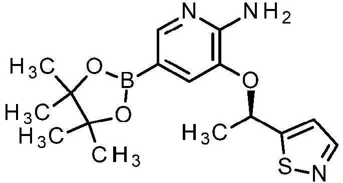

5-Bromo-3-[(1R)-1-(1,2-thiazol-5-yl)ethoxy]pyridin-2-amine (123 mg, 410 µmol) and 4,4,4',4',5,5,5',5'-octamethyl-2,2'-bi-1,3,2-dioxaborolane (125 mg, 492 µmol) were dissolved in degassed 1,4-dioxane (2.1 mL). Potassium acetate (121 mg, 1.23 mmol) and Pd(dppf)Cl₂ (15.0 mg, 20.5 µmol) were added and the mixture was stirred for 4.5 h at 100 °C. The reaction mixture was allowed to cool down, diluted with ethyl acetate and filtered. The filtrate was washed with water and brine, dried over magnesium sulfate, filtered and concentrated to give 197 mg (62 % purity, 86 % yield) of the title compound.
LC-MS (Method 1): Rt = 0.91 min; MS (ESlpos): m/z = 348 [M+H]⁺
¹H-NMR (400MHz, DMSO-d₆): δ [ppm]= 1.24 (s, 12H), 1.64 (d, 3H), 6.06 (q, 1H), 6.22 (s, 2H), 7.13 (s, 1H), 7.43 (d, 1H), 7.83 (d, 1H), 8.49 (d, 1H).

### Intermediate 472

### (rac)-3-[1-(2,6-dichlorophenyl)ethoxy]-5-(4,4,5,5-tetramethyl-1,3,2-dioxaborolan-2-yl)pyridin-2-amine

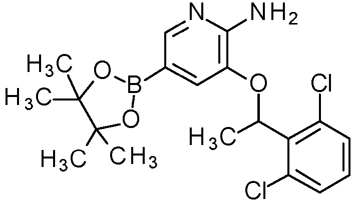

(Rac)-5-Bromo-3-[1-(2,6-dichlorophenyl)ethoxy]pyridin-2-amine (337 mg, 931 µmol), 4,4,4',4',5,5,5',5'-octamethyl-2,2'-bi-1,3,2-dioxaborolane (260 mg, 1.02 mmol) and potassium acetate (274 mg, 2.7 mmol) were dissolved in degassed 1,4-dioxane (9 mL) under argon. Pd(dppf)Cl₂ (38.0 mg, 46.5 µmol) was added and the mixture was stirred for 3 h at 100 °C. The reaction mixture was allowed to cool down, filtered and the filtrate was used in the next step without further work-up or purification.

### Intermediate 473

### (rac)-4-[3-(1-{[2-amino-5-(4,4,5,5-tetramethyl-1,3,2-dioxaborolan-2-yl)pyridin-3-yl]oxy}ethyl)phenyl]-2-methylbut-3-yn-2-ol

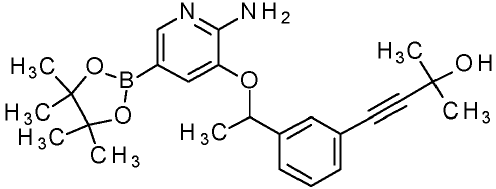

(Rac)-4-(3-{1-[(2-Amino-5-bromopyridin-3-yl)oxy]ethyl}phenyl)-2-methylbut-3-yn-2-ol (155 mg, 413 µmol) was dissolved in degassed 1,4-dioxane (3.7 mL). 4,4,4',4',5,5,5',5'-Octamethyl-2,2'-bi-1,3,2-dioxaborolane (126 mg, 496 µmol), potassium acetate (121.6 mg, 1.2 mmol) and Pd(dppf)Cl₂ (30.2 mg, 41 µmol) were added and the mixture was stirred over night at 100 °C. The recation mixture was allowed to cool down, filtered and the filtrate was used in the next step without further work-up or purification

### Intermediate 474

### 3-[(6,7-dihydro-5H-cyclopenta[b]pyridin-7-yl)oxy]-5-(4,4,5,5-tetramethyl-1,3,2-dioxaborolan-2-yl)pyridin-2-amine (enantiomer 2)

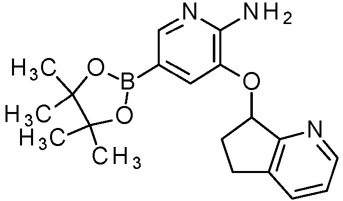

5-Bromo-3-[(6,7-dihydro-5H-cyclopenta[b]pyridin-7-yl)oxy]pyridin-2-amine (enantiomer 2) (120 mg, 392 µmol), 4,4,4',4',5,5,5',5'-octamethyl-2,2'-bi-1,3,2-dioxaborolane (119 mg, 470 µmol) and potassium acetate (115 mg, 1.18 mmol) were suspended in degassed 1,4-dioxane (2 mL). Pd(dppf)Cl₂ (14.3 mg, 19.6 µmol) was added and the mixture was stirred for 2 h at 100 °C.The reaction mixture was allowed to cool down, diluted with ethyl acetate and filtered. The filtrate was washed with water and brine, dried over sodium sulfate, filtered and concentrated to give 223 mg (40 % purity, 83 % yield) of the title compound.
LC-MS (Method 1): Rt = 0.82 min; MS (ESlpos): m/z = 354 [M+H]⁺

### Intermediate 475

### (rac)-3-[(6,7-dihydro-5H-cyclopenta[b]pyridin-7-yl)oxy]-5-(4,4,5,5-tetramethyl-1,3,2-dioxaborolan-2-yl)pyridin-2-amine

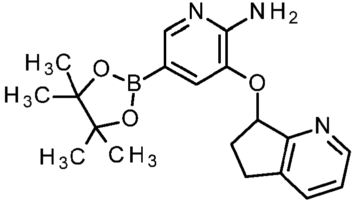

(Rac)-5-Bromo-3-[(6,7-dihydro-5H-cyclopenta[b]pyridin-7-yl)oxy]pyridin-2-amine (300 mg, 980 µmol) was dissolved in degassed 1,4-dioxane (8.7 mL). 4,4,4',4',5,5,5',5'-octamethyl-2,2'-bi-1,3,2-dioxaborolane (299 mg, 1.18 mmol), potassium acetate (288 mg, 2.94 mmol) and Pd(dppf)Cl₂ (71.7 mg, 98.0 µmol) were added and the mixture was stirred over night at 100 °C. The raction mixture was allowed to cool down, filtered and the filtrate was used in the next step without further work-up or purification.

### Intermediate 476

### 3-(difluoromethyl)-5-(4,4,5,5-tetramethyl-1,3,2-dioxaborolan-2-yl)pyridin-2-amine

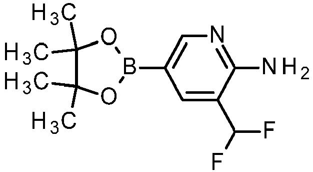

5-Bromo-3-(difluoromethyl)pyridin-2-amine (350 mg, 1.57 mmol) and 4,4,4',4',5,5,5',5'-octamethyl-2,2'-bi-1,3,2-dioxaborolane (438 mg, 1.73 mmol) were dissolved in 1,4-dioxane (14 mL). Potassium acetate (462 mg, 4.71 mmol) was added and the mixture was degassed. Pd(dppf)Cl₂ (64.1 mg, 78.5 µmol) was added and stirred for 2 h at 100 °C. The reaction mixture was allowed to cool down, diluted with water and extracted with ethyl acetate. The combined organic layers were washed with brine, dried over a hydrophobic filter and concentrated to give 970 mg (43 % purity, 98 % yield) of the title compound.
LC-MS (Method 1): Rt = 0.72 min; MS (ESlpos): m/z = 271.5 [M+H]⁺

### Intermediate 477

### 5-(4,4,5,5-tetramethyl-1,3,2-dioxaborolan-2-yl)-3-(trifluoromethyl) pyridin-2-amine

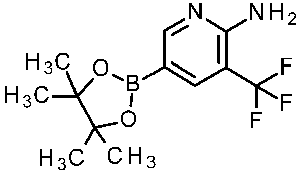

5-Bromo-3-(trifluoromethyl)pyridin-2-amine (5.40 g, 22.4 mmol), 4,4,4',4',5,5,5',5'-octamethyl-2,2'-bi-1,3,2-dioxaborolane (6.26 g, 24.6 mmol) and potassium acetate (6.6 g, 67.2 mmol) were dissolved in dry 1,4-dioxane (230 mL). The mixture was degassed and Pd(dppf)Cl₂ (915 mg, 1.12 mmol) was added and stirred for 3 h at 100 °C. The reaction mixture was allowed to cool down, diluted with water and extracted with ethyl acetate. The combined organic layers were dried over sodium sulfate, filtered and concentrated to give 7.30 g (113 % yield) of the title compound.
LC-MS (Method 1): Rt = 0.76 min; MS (ESlpos): m/z = 289 [M+H]⁺
¹H-NMR (400 MHz, DMSO-d6) δ [ppm]: 1.065 (0.73), 1.154 (16.00), 1.163 (0.81), 1.269 (14.15), 1.284 (0.53), 1.291 (0.84), 6.922 (0.52), 7.800 (0.62), 7.802 (0.63), 8.384 (0.58).

### Intermediate 478

3-[(1R)-1-(2,6-dichloro-3-fluorophenyl)ethoxy]-5-(4,4,5,5-tetramethyl-1,3,2-dioxaborolan-2-yl)pyridin-2-amine

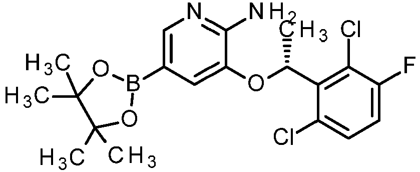

5-Bromo-3-[(1R)-1-(2,6-dichloro-3-fluorophenyl)ethoxy]pyridin-2-amine (500 mg, 1.32 mmol), 4,4,4',4',5,5,5',5'-octamethyl-2,2'-bi-1,3,2-dioxaborolane (368 mg, 1.45 mmol) and potassium acetate (387 mg, 3.9 mmol) were dissolved in degassed 1,4-dioxane (13 mL). Pd(dppf)Cl₂ (53.7 mg, 65.8 µmol) was added and the mixture was stirred for 5 h at 100 °C. The reaction mixture was allowed to cool down, filtered and the filtrate was used without further purification in the next step.
LC-MS (Method 1): Rt = 1.38 min; MS (ESlpos): m/z = 427 [M+H]⁺

### Intermediate 479

### (rac)-3-[1-(4,5-dimethyl-1,3-thiazol-2-yl)ethoxy]-5-(4,4,5,5-tetramethyl-1,3,2-dioxaborolan-2-yl)pyridin-2-amine

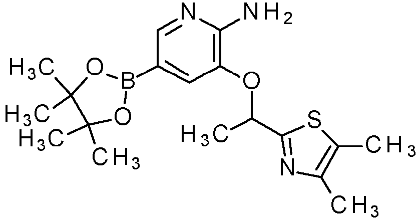

(Rac)-5-Bromo-3-[1-(4,5-dimethyl-1,3-thiazol-2-yl)ethoxy]pyridin-2-amine (186 mg, 567 µmol) and 4,4,4',4',5,5,5',5'-octamethyl-2,2'-bi-1,3,2-dioxaborolane (187 mg, 737 µmol) were dissolved in degassed 1,4-dioxane (2.9 mL) under argon. Potassium acetate (167 mg, 1.70 mmol) and Pd(dppf)Cl₂ (41.5 mg, 56.7 µmol) were added and the mixture was stirred for 3 h at 100 °C. The reaction mixture was allowed to cool down, diluted with ethyl acetate and filtered. The filtrate was washed with water and brine, dried over magnesium sulfate, filtered and concentrated to give 356 mg (39 % purity, 65 % yield) of the title compound.
LC-MS (Method 1): Rt = 1.10 min; MS (ESlpos): m/z = 376 [M+H]⁺
¹H-NMR (400 MHz, DMSO-d6) δ [ppm]: 1.065 (1.42), 1.156 (16.00), 1.163 (8.28), 1.239 (6.27), 1.292 (0.71), 1.621 (0.99), 1.628 (0.49), 1.637 (1.00), 2.228 (2.16), 2.281 (2.00), 3.565 (3.37), 6.180 (0.47), 7.123 (0.51), 7.802 (0.52), 7.805 (0.52), 7.943 (1.34).

### Intermediate 480

### 3-[(1R)-1-(pyridin-4-yl)ethoxy]-5-(4,4,5,5-tetramethyl-1,3,2-dioxaborolan-2-yl)pyridin-2-amine

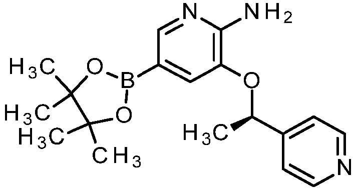

5-Bromo-3-[(1R)-1-(pyridin-4-yl)ethoxy]pyridin-2-amine (489 mg, 75 % purity, 1.25 mmol), 4,4,4',4',5,5,5',5'-octamethyl-2,2'-bi-1,3,2-dioxaborolane (380 mg, 1.50 mmol) and potassium acetate (367 mg, 3.74 mmol) were dissolved in 1,4-dioxane (5.9 mL) under argon. Pd(dppf)Cl₂ (43.8 mg, 62.3 µmol; CAS-RN:[13965-03-2]) was added and the mixture was stirred for 1 h at 100 °C. 4,4,4',4',5,5,5',5'-octamethyl-2,2'-bi-1,3,2-dioxaborolane (100 mg, 0.39 mmol) was added again and stirred for 1 h at 100 °C. The reaction mixture was allowed to cool down, diluted with ethyl acetate and filtered. The filtrate was washed with water and brine, dried and concentrated to give 833 mg (26 % purity, 51 % yield) of the title compound.
LC-MS (Method 1): Rt = 0.74 min; MS (ESlpos): m/z = 342 [M+H]⁺
¹H-NMR (400 MHz, DMSO-d6) δ [ppm]: 1.066 (8.27), 1.156 (16.00), 1.164 (9.04), 1.171 (0.46), 1.189 (0.18), 1.217 (1.73), 1.219 (1.75), 1.232 (0.33), 1.239 (0.24), 1.274 (0.51), 1.292 (0.27), 1.511 (0.46), 1.527 (0.46), 1.542 (0.32), 1.558 (0.31), 1.907 (0.27), 1.987 (0.40), 2.518 (0.28), 2.522 (0.19), 3.330 (3.03), 3.937 (1.12), 6.300 (0.20), 6.963 (0.19), 6.965 (0.19), 7.468 (0.24), 7.471 (0.17), 7.479 (0.17), 7.483 (0.24), 7.782 (0.22), 7.785 (0.23), 7.940 (1.02), 8.532 (0.30), 8.536 (0.26), 8.547 (0.22).

### Intermediate 481

### (rac)-3-[1-(5-methyl-1,3-thiazol-2-yl)ethoxy]-5-(4,4,5,5-tetramethyl-1,3,2-dioxaborolan-2-yl)pyridin-2-amine

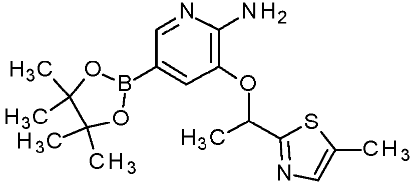

(Rac)-5-Bromo-3-[1-(5-methyl-1,3-thiazol-2-yl)ethoxy]pyridin-2-amine (204 mg, 649 µmol) and 4,4,4',4',5,5,5',5'-octamethyl-2,2'-bi-1,3,2-dioxaborolane (214 mg, 844 µmol) were dissolved in degassed 1,4-dioxane (3.3 mL). Potassium acetate (191 mg, 1.95 mmol) and Pd(dppf)Cl₂ (47.5 mg, 64.9 µmol) were added and the mixture was stirred for 3 h at 100 °C. The reaction mixture was allowed to cool down, diluted with ethyl acetate and filtered. The filtrate was washed with water and brine, dried over magnesium sulfate, filtered and concentrated to give 335 mg (38 % purity, 54 % yield) of the title compound which was used in the next step without further purification.
LC-MS (Method 1): Rt = 0.97 min; MS (ESlpos): m/z = 362 [M+H]⁺
¹H-NMR (400 MHz, DMSO-d6) δ [ppm]: 1.066 (1.69), 1.156 (16.00), 1.164 (1.41), 1.237 (3.24), 1.637 (0.51), 1.652 (0.73), 2.394 (0.85), 2.397 (1.09), 2.401 (0.50), 3.566 (4.65), 7.943 (1.40).

### Intermediate 482

### (rac)-5-(4,4,5,5-tetramethyl-1,3,2-dioxaborolan-2-yl)-3-[1-(1,3-thiazol-4-yl)ethoxy]pyridin-2-amine

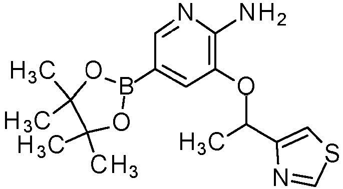

(Rac)-5-Bromo-3-[1-(1,3-thiazol-4-yl)ethoxy]pyridin-2-amine (180 mg, 600 µmol) and 4,4,4',4',5,5,5',5'-octamethyl-2,2'-bi-1,3,2-dioxaborolane (183 mg, 720 µmol) were dissolved in degassed 1,4-dioxane (3.1 mL). Potassium acetate (177 mg, 1.80 mmol) and Pd(dppf)Cl₂ (21.9 mg, 30.0 µmol) were added and the mixture was stirred for 1.5 h at 65 °C and 2.5 h at 100 °C. The reaction mixture was allowed to cool down, diluted with ethyl acetate and filtered. The filtrate was washed with water and brine, dried over magnesium sulfate, filtered and concentrated to give 288 mg (36 % purity, 50 % yield) of the title compound which was used without further purification in the next step.
LC-MS (Method 1): Rt = 0.88 min; MS (ESlpos): m/z = 348 [M+H]⁺
¹H-NMR (400MHz, DMSO-d₆): δ [ppm]= 1.24 (s, 12H), 1.61 (d, 3H), 5.57 - 5.65 (m, 1H), 6.19 (s, 2H), 7.08 (d, 1H), 7.68 (d, 1H), 7.81 (d, 1H), 9.10 (d, 1H).

### Intermediate 483

### 3-[(1R)-1-(5-methyl-1,3,4-oxadiazol-2-yl)ethoxy]-5-(4,4,5,5-tetramethyl-1,3,2-dioxaborolan-2-yl)pyridin-2-amine

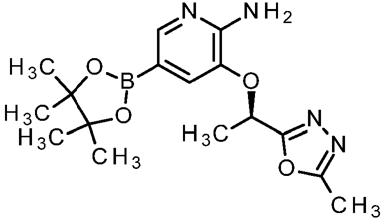

5-Bromo-3-[(1R)-1-(5-methyl-1,3,4-oxadiazol-2-yl)ethoxy]pyridin-2-amine (100 mg, 334 µmol) and 4,4,4',4',5,5,5',5'-octamethyl-2,2'-bi-1,3,2-dioxaborolane (102 mg, 401 µmol) were dissolved in degassed 1,4-dioxane (1.7 mL). Potassium acetate (98.4 mg, 1.00 mmol) and Pd(dppf)Cl₂ (12.2 mg, 16.7 µmol) were added and the mixture was stirred for 3 h at 100 °C. The reaction mixture was allowed to reac rt, diluted with ethyl acetate and filtered. The filtrate was washed with water and brine, dried over magnesium sulfate, filtered and concentrated to give 100 mg (86 % yield) which was used without further purification in the next step.

### Intermediate 484

### 3-[(1R)-1-(2-methyl-1,3-thiazol-4-yl)ethoxy]-5-(4,4,5,5-tetramethyl-1,3,2-dioxaborolan-2-yl)pyridin-2-amine

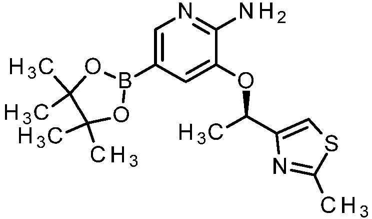

5-Bromo-3-[(1R)-1-(2-methyl-1,3-thiazol-4-yl)ethoxy]pyridin-2-amine (158 mg, 503 µmol) and 4,4,4',4',5,5,5',5'-octamethyl-2,2'-bi-1,3,2-dioxaborolane (166 mg, 653 µmol) were dissolved in degassed 1,4-dioxane (4.5 mL). Potassium acetate (148 mg, 1.51 mmol) and Pd(dppf)Cl₂ (36.8 mg, 50.3 µmol) were added and the mixture was stirred for 2 h at 100 °C. The reaction mixture was allowed to cool down, diluted with ethyl acetate and filtered. The filtrate was washed with water and brine, dried over a hydrophobic filter and concentrated to give 304 mg (40 % purity, 67 % yield) of the title compound.
LC-MS (Method 1): Rt = 1.00 min; MS (ESlpos): m/z = 362 [M+H]⁺
¹H-NMR (400 MHz, DMSO-d6) δ [ppm]: 1.065 (3.58), 1.156 (16.00), 1.164 (6.12), 1.242 (3.70), 1.574 (0.70), 1.590 (0.71), 2.518 (0.69), 2.523 (0.47), 2.645 (2.18), 3.565 (5.87), 3.940 (0.58), 7.416 (0.53), 7.942 (1.26).

### Intermediate 485

### (rac)-3-[1-(4-methyl-1,3-thiazol-2-yl)ethoxy]-5-(4,4,5,5-tetramethyl-1,3,2-dioxaborolan-2-yl)pyridin-2-amine

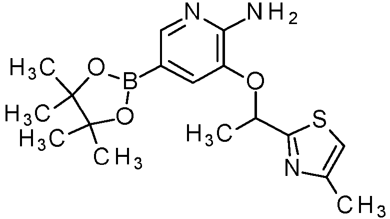

(Rac)-5-Bromo-3-[1-(4-methyl-1,3-thiazol-2-yl)ethoxy]pyridin-2-amine (126 mg, 401 µmol) and 4,4,4',4',5,5,5',5'-octamethyl-2,2'-bi-1,3,2-dioxaborolane (132 mg, 521 µmol) were dissolved in degassed 1,4-dioxane (2.1 mL). Potassium acetate (118 mg, 1.20 mmol) and Pd(dppf)Cl₂ (29.3 mg, 40.1 µmol) were added and the mixture was stirred for 3 h at 100 °C. The reaction mixture was allowed to reach rt, diluted with ethyl acetate and filtered. The filtrate was washed with water and brine, dried over a magnesium sulfate, filtered and concentrated to give 222 mg (44 % purity, 67 % yield) of the title compound.

¹H-NMR (400 MHz, DMSO-d6) δ [ppm]: 1.066 (2.89), 1.156 (16.00), 1.164 (8.17), 1.237 (5.44), 1.292 (0.92), 1.648 (1.18), 1.664 (1.18), 2.347 (2.41), 2.349 (2.47), 2.518 (0.63), 2.523 (0.43), 3.566 (7.03), 3.938 (0.49), 6.203 (0.43), 7.117 (0.50), 7.120 (0.50), 7.230 (0.63), 7.232 (0.66), 7.811 (0.59), 7.814 (0.57), 7.940 (1.36).

### Intermediate 486

### (rac)-3-[1-(1-methyl-1H-1,2,3-triazol-4-yl)ethoxy]-5-(4,4,5,5-tetramethyl-1,3,2-dioxaborolan-2-yl)pyridin-2-amine

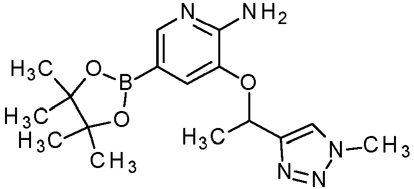

(Rac)-5-Bromo-3-[1-(1-methyl-1H-1,2,3-triazol-4-yl)ethoxy]pyridin-2-amine (281 mg, 941 µmol) and 4,4,4',4',5,5,5',5'-octamethyl-2,2'-bi-1,3,2-dioxaborolane (311 mg, 1.22 mmol) were dissolved in degassed 1,4-dioxane (8.4 mL). Potassium acetate ((277 mg, 2.82 mmol) and Pd(dppf)Cl₂ (68.9 mg, 94.1 µmol) were added and the mixture was stirred for 2 h at 100 °C. The reaction mixture was allowed to cool down, diluted with ethyl acetate and filtered. The filtrate was concentrated to give 701 mg (24 % purity, 52 % yield) of the title compound.
LC-MS (Method 1): Rt = 0.71 min; MS (ESlpos): m/z = 346 [M+H]⁺
¹H-NMR (400MHz, DMSO-d₆): δ [ppm]= 1.25 (s, 12H), 1.60 (d, 3H), 4.01 (s, 3H), 5.57 (q, 1H), 6.10 (s, 2H), 7.17 (d, 1H), 7.80 (d, 1H), 8.09 (s, 1H).

### Intermediate 487

### (rac)-3-{1-[3-(methanesulfonyl)phenyl]ethoxy}-5-(4,4,5,5-tetramethyl-1,3,2-dioxaborolan-2-yl)pyridin-2-amine

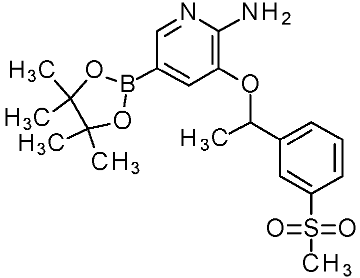

(Rac)-5-Bromo-3-{1-[3-(methanesulfonyl)phenyl]ethoxy}pyridin-2-amine (989 mg, 2.66 mmol), 4,4,4',4',5,5,5',5'-octamethyl-2,2'-bi-1,3,2-dioxaborolane (744 mg, 2.93 mmol) and potassium acetate (784 mg, 7.99 mmol]) were dissolved in degassed 1,4-dioxane (27 mL) under argon. Pd(dppf)Cl₂ (109 mg, 133 µmol) was added and the mixture was stirred over night at 100 °C. The reaction mixture was allowed to cool down, filtered and the filtrate was diluted with ethyl acetate and. The organic phase was washed with water. The aqueous phase was extracted with ethy acetate. The combined organic layers were dried over sodium sulfate, filtered and concentrated to give 1.50 g (135 % yield) of the title compound.
LC-MS (Method 1): Rt = 0.90 min; MS (ESlpos): m/z = 419 [M+H]⁺
¹H-NMR (400 MHz, DMSO-d6) δ [ppm]: 1.066 (9.70), 1.156 (15.59), 1.164 (16.00), 1.214 (2.26), 1.222 (2.46), 1.260 (0.80), 1.274 (0.48), 1.292 (1.16), 1.344 (0.51), 1.360 (0.48), 1.550 (0.60), 1.566 (0.60), 1.904 (4.50), 3.203 (1.25), 3.210 (2.09), 3.334 (0.62), 7.766 (0.41), 7.770 (0.44).

### Intermediate 488

### 3-[(1R)-1-(1-methyl-1H-1,2,3-triazol-5-yl)ethoxy]-5-(4,4,5,5-tetramethyl-1,3,2-dioxaborolan-2-yl)pyridin-2-amine

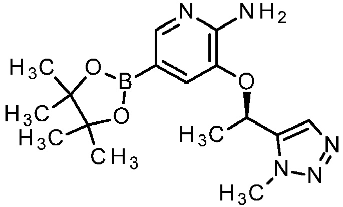

5-Bromo-3-[(1R)-1-(1-methyl-1H-1,2,3-triazol-5-yl)ethoxy]pyridin-2-amine (131 mg, 439 µmol) and 4,4,4',4',5,5,5',5'-octamethyl-2,2'-bi-1,3,2-dioxaborolane (134 mg, 527 µmol) were dissolved in degassed 1,4-dioxane (2.3 mL). Potassium acetate (129 mg, 1.32 mmol) and Pd(dppf)Cl₂ (32.1 mg, 43.9 µmol) were added and the mixture was stirred for 4 h at 100 °C. The reaction mixture was allowed to cool down, diluted with ethyl acetate and filtered. The filtrate was washed with water and brine, dried over magnesium sulfate, filtered and concentrated to give 226 mg (31 % purity, 46 % yield) of the title compound.
LC-MS (Method 1): Rt = 0.67 min; MS (ESlpos): m/z = 346 [M+H]⁺
¹H-NMR (400MHz, DMSO-d₆): δ [ppm]= 1.26 (s, 12H), 1.60 (d, 3H), 4.00 (s, 3H), 5.87 (q, 1H), 6.22 (s, 2H), 7.19 (d, 1H), 7.85 (s, 1H), 7.94 (s, 1H).

### Intermediate 489

### (rac)-3-[1-(1-propyl-1H-pyrazol-5-yl)ethoxy]-5-(4,4,5,5-tetramethyl-1,3,2-dioxaborolan-2-yl)pyridin-2-amine

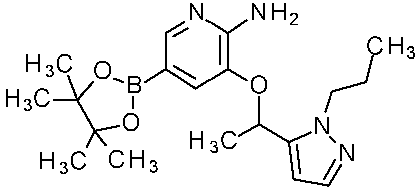

(Rac)-5-Bromo-3-[1-(1-propyl-1H-pyrazol-5-yl)ethoxy]pyridin-2-amine (159 mg, 50 % purity, 244 µmol), 4,4,4',4',5,5,5',5'-octamethyl-2,2'-bi-1,3,2-dioxaborolane (93.1 mg, 367 µmol) and potassium acetate (96 mg, 978 µmol) were dissolved in degassed 1,4-dioxane (2.5 mL) under argon. Pd(dppf)Cl₂ (9.98 mg, 12.2 µmol) was added and the mixture was stirred for 3 h at 100 °C. The reaction mixture was allowed to cool down, filtered and the filtrate was used in the next step without further work-up or purification.
LC-MS (Method 1): Rt = 1.00 min; MS (ESlpos): m/z = 373 [M+H]⁺

### Intermediate 490

### (rac)-3-[1-(pyrimidin-4-yl)ethoxy]-5-(4,4,5,5-tetramethyl-1,3,2-dioxaborolan-2-yl)pyridin-2-amine

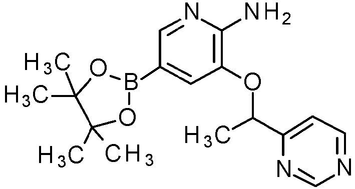

(Rac)-5-Bromo-3-[1-(pyrimidin-4-yl)ethoxy]pyridin-2-amine (120 mg, 407 µmol) was dissolved in degasse 1,4-dioxane (3.6 mL). 4,4,4',4',5,5,5',5'-Octamethyl-2,2'-bi-1,3,2-dioxaborolane (124 mg, 488 µmol), potassium acetate (119.7 mg, 1.2 mmol) and Pd(dppf)Cl₂ (29.7 mg, 41 µmol) were added and the mixture was stirred over night at 100 °C. The reaction mixture was allowed to cool down, filtered and the filtrate was used in the next step without further work-up or purification.

### Intermediate 491

### 3-{[1-(2,4-dimethylpyrimidin-5-yl)ethyl]oxy}-5-(4,4,5,5-tetramethyl-1,3,2-dioxaborolan-2-yl)pyridin-2-amine (enantiomer 2)

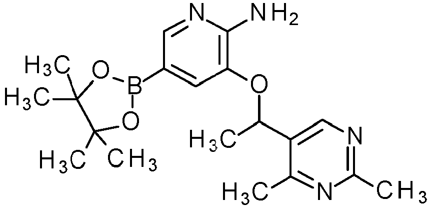

5-Bromo-3-{[1-(2,4-dimethylpyrimidin-5-yl)ethyl]oxy}pyridin-2-amine (enantiomer 2) (80.0 mg, 248 µmol) was dissolved In degassed 1,4-dioxane (2.2 mL). 4,4,4',4',5,5,5',5'-Octamethyl-2,2'-bi-1,3,2-dioxaborolane (75.4 mg, 297 µmol), potassium acetate (73 mg, 0.7 mmol) and Pd(dppf)Cl₂ (18.1 mg, 25 µmol) were added and the mixture was stirred over night at 100 °C. The reaction mixture was allowed to cool down, filtered and the filtrate was used in the next step without further work-up or purification.
LC-MS (Method 1): Rt = 0.72 min; MS (ESlpos): m/z = 371 [M+H]⁺
¹H-NMR (400 MHz, DMSO-d6) δ [ppm]: 1.066 (0.38), 1.156 (16.00), 1.164 (4.38), 1.231 (2.48), 1.236 (2.45), 1.293 (0.46), 1.550 (0.67), 1.565 (0.69), 1.586 (0.21), 2.467 (0.18), 2.523 (2.11), 3.566 (9.17), 6.287 (0.36), 7.036 (0.31), 7.039 (0.30), 7.777 (0.41), 7.781 (0.40), 8.733 (0.57).

### Intermediate 492

### 3-{[1-(2,4-dimethylpyrimidin-5-yl)ethyl]oxy}-5-(4,4,5,5-tetramethyl-1,3,2-dioxaborolan-2-yl)pyridin-2-amine (enantiomer 1)

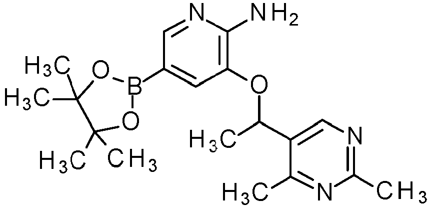

5-Bromo-3-{[1-(2,4-dimethylpyrimidin-5-yl)ethyl]oxy}pyridin-2-amine (enantiomer 1) (80.0 mg, 248 µmol) was dissolved in degassed 1,4-dioxane (2.2 mL). 4,4,4',4',5,5,5',5'-Octamethyl-2,2'-bi-1,3,2-dioxaborolane (75.4 mg, 297 µmol), potassium acetate (73 mg, 0.7 mmol) and Pd(dppf)Cl₂ (18.1 mg, 25 µmol) were added and the mixture was stirred over night at 100 °C. The reaction mixture was allowed to cool down, filtered and the filtrate was used in the next step without further work-up or purification.
LC-MS (Method 1): Rt = 0.72 min; MS (ESlpos): m/z = 371 [M+H]⁺

### Intermediate 493

### [6-amino-5-(6,7-dihydro-5H-cyclopenta[b]pyridin-7-yloxy)pyridin-3-yl]boronic acid (enantiomer 1)

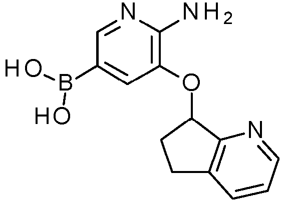

5-Bromo-3-[(6,7-dihydro-5H-cyclopenta[b]pyridin-7-yl)oxy]pyridin-2-amine (enantiomer 1) (125 mg, 408 µmol), 4,4,4',4',5,5,5',5'-octamethyl-2,2'-bi-1,3,2-dioxaborolane (124 mg, 490 µmol) and potassium acetate (120 mg, 1.22 mmol) were suspended in degassed 1,4-dioxane. Pd(dppf)Cl₂ (14.9 mg, 20.4 µmol) was added and the mixture was stirred for 2 h at 100 °C. The reaction mixture was allowed to cool down, diluted with ethyl acetate and filtered. The fitrate was washed with water and brine, dried over sodium sulfate, filtered and concentrated to give 212 mg (76 % yield, 40 % purity) of the title compound which was used without further purification in the next step.
LC-MS (Method 1): Rt = 0.53 min; MS (ESlpos): m/z = 272 [M+H]⁺

### Intermediate 494

### 3-{1-[4-(methanesulfonyl)phenyl]ethoxy}-5-(4,4,5,5-tetramethyl-1,3,2-dioxaborolan-2-yl)pyridin-2-amine (enantiomer 1 and enantiomer 2)

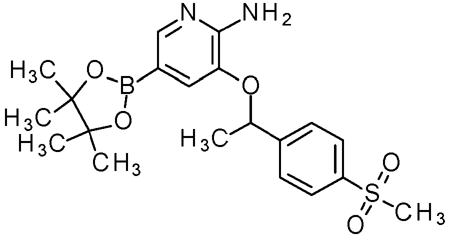

5-Bromo-3-{1-[4-(methanesulfonyl)phenyl]ethoxy}pyridin-2-amine (298 mg, 803 µmol), 4,4,4',4',5,5,5',5'-octamethyl-2,2'-bi-1,3,2-dioxaborolane (224 mg, 883 µmol) and potassium acetate (236 mg, 2.41 mmol) were dissolved in degassed 1,4-dioxane (8 mL). Pd(dppf)Cl₂ (32.8 mg, 40.1 µmol) was added and the mixture was stirred over night at 100 °C. The reaction mixture was allowed to cool down and filtered. The filtrate was diluted with ethyl acetate and washed with water. The aqueous phase was extracted with ethyl acetate. The combined organic layers were dried over sodium sulfate, filtered and concentrated to give 429 mg (128 % yield) of the title compound.
LC-MS (Method 1): Rt = 0.81 min; MS (ESlpos): m/z = 419 [M+H]⁺
¹H-NMR (400 MHz, DMSO-d6) δ [ppm]: 1.065 (4.14), 1.156 (16.00), 1.164 (8.03), 1.219 (1.60), 1.292 (1.12), 1.901 (1.04), 2.518 (0.41), 3.202 (0.47), 3.206 (1.16), 3.566 (14.17).

### Intermediate 495

### di-tert-butyl [5-(4,4,5,5-tetramethyl-1,3,2-dioxaborolan-2-yl)-3-(trifluoromethyl)pyrazin-2-yl]-2-imidodicarbonate

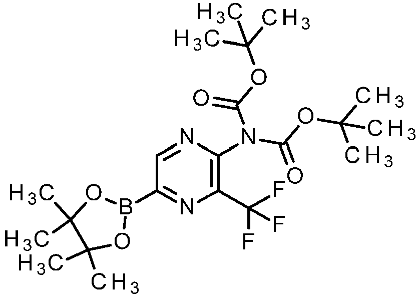

Di-tert-butyl [5-bromo-3-(trifluoromethyl)pyrazin-2-yl]-2-imidodicarbonate (200 mg, 452 µmol, prepared from commercially available 2-amino-5-bromo-3-trifluoromethylpyrazine according to WO2014096423 see the preparation of bis-tert-butoxycarbonyl derivative of 2-amino-5-bromo-3-methylpyrazine), 4,4,4',4',5,5,5',5'-octamethyl-2,2'-bi-1,3,2-dioxaborolane (253 mg, 995 µmol) and potassium acetate (133 mg, 1.3 mmol) were dissolved in 1,4-dioxane (4.5 mL) under argon. Pd(dppf)Cl₂ (18.5 mg, 22.6 µmol) was added and the mixture was stirred for 2 h at 100 °C. The reaction mixture was allowed to cool down, diluted with water (and a little bit of brine) and extracted with ethyl acetate. The combined organic layers were dried over a hydrophobic filter and concentrated. The residue was used in the next step without further purification.

### Intermediate 496

### 3-[(1R)-1-(2,6-difluorophenyl)ethoxy]-5-(4,4,5,5-tetramethyl-1,3,2-dioxaborolan-2-yl)pyridin-2-amine

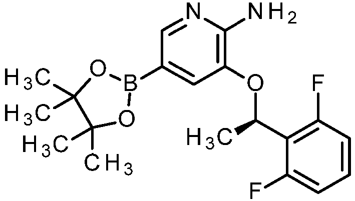

5-Bromo-3-[(1R)-1-(2,6-difluorophenyl)ethoxy]pyridin-2-amine (196 mg, 595 µmol) and 4,4,4',4',5,5,5',5'-octamethyl-2,2'-bi-1,3,2-dioxaborolane (181 mg, 715 µmol) were dissolved in degassed 1,4-dioxane und argon. Potassium acetate (175 mg, 1.79 mmol) and Pd(dppf)Cl₂ (21.8 mg, 29.8 µmol) were added and the mixture was stirred for 3.5 h at 100 °C. The reaction mixture was allowed to cool down, diluted with ethyl acetate and filtered. The filtrate was washed with water and brinde, dried over magnesium sulfate, filtered and concentrated to give 330 mg (49 % purity, 72 % yield) of the title compound which was used without further purification in the next step.
LC-MS (Method 1): Rt = 1.26 min; MS (ESlpos): m/z = 377 [M+H]⁺
¹H NMR (DMSO-d₆) δ: 7.76 (d, 1H), 7.36 - 7.45 (m, 1H), 7.05 - 7.13 (m, 2H), 7.04 (d, 1H), 6.02 (br s, 2H), 5.70 - 5.78 (m, 1H), 1.72 (m, 3H), 1.16 (s, 12H).

### Intermediate 497

### 3-[(1R)-1-phenylethoxy]-5-(4,4,5,5-tetramethyl-1,3,2-dioxaborolan-2-yl)pyrazin-2-amine

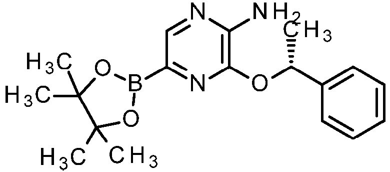

5-Bromo-3-[(1R)-1-phenylethoxy]pyrazin-2-amine (37.0 mg, 126 µmol), 4,4,4',4',5,5,5',5'-octamethyl-2,2'-bi-1,3,2-dioxaborolane (35.1 mg, 138 µmol) and potassium acetate (37.0 mg, 377 µmol) were combined in degassed 1,4-dioxane (1 mL). Pd(dppf)Cl₂ was added and the mixture was stirred for 4 h at 100 °C. The reaction mixture was allowed to cool down, filtered and used without further work-up or purification in the next step.
LC-MS (Method 2): Rt = 0.73 min; MS (ESlneg): m/z = 339 [M-H]⁻

### Suzuki

### Intermediate 498

### tert-butyl (3R)-2'-{6-amino-5-[(1R)-1-(3-fluoropyridin-2-yl)ethoxy]pyridin-3-yl}-5',6'-dihydrospiro[pyrrolidine-3,4'-pyrrolo[1,2-b]pyrazole]-1-carboxylate

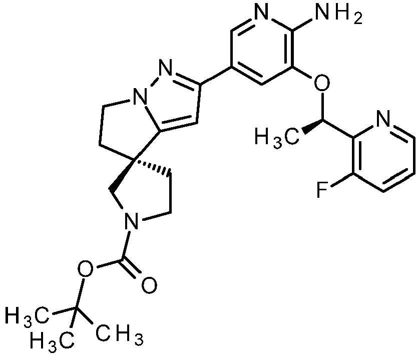

Tert-butyl (3R)-2'-[(trifluoromethanesulfonyl)oxy]-5',6'-dihydrospiro[pyrrolidine-3,4'-pyrrolo[1,2-b]pyrazole]-1-carboxylate (75.0 mg, 182 µmol), {6-amino-5-[(1R)-1-(3-fluoropyridin-2-yl)ethoxy]pyridin-3-yl}boronic acid (202 mg, 35 % purity, 255 µmol) and potassium phosphate (1.1 mL, 0.50 M, 550 µmol) were dissolved in degassed 1,4-dioxane (3.0 mL) under argon. XPhos Pd G2 (7.17 mg, 9.11 µmol) was added and the mixture was stirred for 2 h at 100 °C. The reaction mixture was allowed to cool down, diluted with ethyl acetate and filtered. The filtrate was washed with water and brine. The organic phase was dried over sodium sulfate, filtered and concentrated to give a residue. The crude product was purified by flash column chromatography (silica gel, dichloromethane / methanol (0-20%) gradient) to give 94.9 mg (60 % purity, 63 % yield) of the title compound.
LC-MS (Method 1): Rt = 1.16 min; MS (ESlpos): m/z = 496 [M+H]⁺
¹H-NMR (400 MHz, CHLOROFORM-d) δ [ppm]: 1.249 (16.00), 1.310 (0.41), 1.317 (0.41), 1.476 (1.41), 1.775 (0.35), 1.781 (0.80), 1.791 (0.37), 1.797 (0.79), 3.497 (0.38), 4.249 (0.21), 4.875 (0.39), 6.104 (0.20), 7.399 (0.17), 8.041 (0.18), 8.433 (0.20).

### Intermediate 499

### tert-butyl (3R)-2'-{6-amino-5-[(1R)-1-(3,5-difluoropyridin-4-yl)ethoxy]pyridin-3-yl}-5',6'-dihydrospiro[pyrrolidine-3,4'-pyrrolo[1,2-b]pyrazole]-1-carboxylate

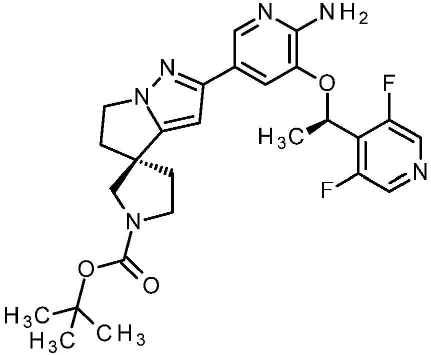

Tert-butyl (3R)-2'-[(trifluoromethanesulfonyl)oxy]-5',6'-dihydrospiro[pyrrolidine-3,4'-pyrrolo[1,2-b]pyrazole]-1-carboxylate (61.2 mg, 149 µmol), {6-amino-5-[(1R)-1-(3,5-difluoropyridin-4-yl)ethoxy]pyridin-3-yl}boronic acid (143 mg, 43 % purity, 208 µmol) and potassium phosphate (890 µL, 0.50 M, 450 µmol) were dissolved in degassed 1,4-dioxane (2.4 mL) under argon. XPhos Pd G2 (5.86 mg, 7.44 µmol) was added and the mixture was stirred for 2 h at 100 °C. The reaction mixture was allowed to cool down, diluted with ethyl acetate and filtered. The filtrate was washed with water and brine. The organic phase was dried over sodium sulfate, filtered and concentrated to give a residue. The crude product was purified by flash column chromatography (silica gel, dichloromethane / methanol (0-20%) gradient) to give 108 mg (60 % purity, 85 % yield) of the title compound.
LC-MS (Method 1): Rt = 1.18 min; MS (ESlpos): m/z = 514 [M+H]⁺

### Intermediate 500

### tert-butyl (3R)-2'-{6-amino-5-[1-(1-methyl-1H-pyrazol-5-yl)ethoxy]pyridin-3-yl}-5',6'-dihydro-1H-spiro[pyrrolidine-3,4'-pyrrolo[1,2-b]pyrazole]-1-carboxylate (diastereomer 1 and diastereomer 2)

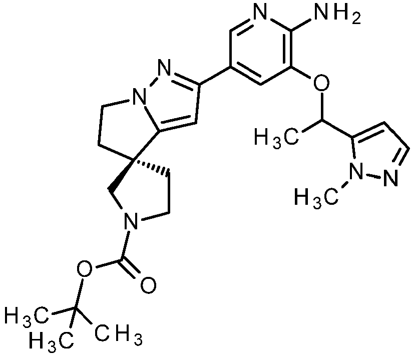

Tert-butyl (3R)-2'-[(trifluoromethanesulfonyl)oxy]-5',6'-dihydrospiro[pyrrolidine-3,4'-pyrrolo[1,2-b]pyrazole]-1-carboxylate (229 mg, 557 µmol), {6-amino-5-[1-(1-methyl-1H-pyrazol-5-yl)ethoxy]pyridin-3-yl}boronic acid (454 mg, 45 % purity, 780 µmol) and potassium phosphate (3.3 mL, 0.50 M, 1.7 mmol) were dissolved in degassed 1,4-dioxane (9.2 mL). XPhos Pd G2 (21.9 mg, 27.8 µmol) was added and the reaction mixture was stirred for 2 h at 100 °C. The reaction mixture was allowed to cool down, diluted with ethyl acetate and filtered. The filtrate was washed with water and brine. The organic phase was dried over sodium sufate, filtered and concentrated. The residue was purified by column chromatography (silica gel, dichloromethane / methanol (0-15 %) gradient) to give 152 mg (80 % purity, 46 % yield) of the title compound.
LC-MS (Method 1): Rt = 1.06 min; MS (ESlpos): m/z = 480 [M+H]⁺
¹H-NMR (400 MHz, DMSO-d6) δ [ppm]: 1.066 (1.11), 1.398 (12.26), 1.415 (3.26), 1.434 (11.74), 1.591 (1.85), 1.613 (6.70), 1.629 (6.51), 2.026 (1.27), 2.037 (1.44), 2.084 (0.60), 3.159 (6.69), 3.172 (6.77), 3.405 (5.59), 3.486 (0.62), 3.504 (1.14), 3.519 (0.79), 3.530 (0.68), 3.788 (3.93), 3.813 (16.00), 4.083 (0.64), 4.097 (1.74), 4.109 (1.82), 4.123 (0.84), 4.136 (1.11), 4.145 (1.36), 4.154 (2.01), 4.161 (2.00), 4.169 (1.35), 4.178 (1.09), 5.206 (0.43), 5.655 (0.86), 5.666 (0.64), 5.682 (0.43), 5.768 (4.77), 5.786 (1.67), 5.802 (1.39), 5.817 (0.51), 6.299 (1.40), 6.321 (1.53), 6.336 (0.85), 6.340 (0.83), 6.373 (3.32), 7.329 (2.62), 7.333 (3.52), 7.407 (2.59), 7.494 (0.50), 7.507 (0.52), 7.921 (3.20), 7.924 (3.21).

### Intermediate 501

### tert-butyl (3S)-2'-[6-amino-5-(trifluoromethyl)pyridin-3-yl]-5',6'-dihydro-1H-spiro[pyrrolidine-3,4'-pyrrolo[1,2-b]pyrazole]-1-carboxylate

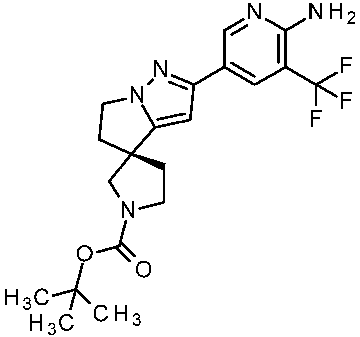

5-(4,4,5,5-Tetramethyl-1,3,2-dioxaborolan-2-yl)-3-(trifluoromethyl)pyridin-2-amine (1.50 g, 5.21 mmol) was dissolved in 1,4-dioxane. Tert-butyl (3S)-2'-[(trifluoromethanesulfonyl)oxy]-5',6'-dihydrospiro[pyrrolidine-3,4'-pyrrolo[1,2-b]pyrazole]-1-carboxylate (2.36 g, 5.73 mmol) and potassium phosphate (31 mL, 0.50 M, 16 mmol) were added and the mixture was degassed with argon. XPhos Pd G2 (205 mg, 260 µmol) was added and the mixture was stirred for 2 h at 100 °C. The reaction mixture was allowed to cool down, diluted with water and extracted 2 times with ethyl acetate. The combined organic layers were washed with brine, dried over sodium sufate, filtered and concentrated to give a crude product. The residue was purified by column chromatography (silica gel, hexane / ethyl acetate (0-100%) gradient) to give 2.30 g (98 % purity, 102 % yield) of the title compound.
LC-MS (Method 1): Rt = 1.20 min; MS (ESlpos): m/z = 424 [M+H]⁺
¹H-NMR (400 MHz, DMSO-d6) δ [ppm]: 1.066 (8.94), 1.154 (0.49), 1.172 (0.96), 1.189 (0.46), 1.396 (16.00), 1.433 (12.64), 1.987 (1.68), 2.019 (0.73), 2.036 (1.54), 2.052 (1.25), 2.065 (0.83), 2.518 (1.64), 2.523 (1.43), 2.536 (1.28), 2.544 (1.64), 2.561 (0.93), 3.159 (7.02), 3.172 (5.86), 3.371 (0.45), 3.378 (0.42), 3.398 (1.58), 3.412 (5.51), 3.427 (0.95), 3.439 (0.81), 3.494 (0.60), 3.512 (1.23), 3.521 (0.59), 3.528 (0.71), 3.539 (0.74), 3.940 (1.49), 4.084 (0.51), 4.096 (1.49), 4.110 (1.55), 4.123 (0.56), 4.146 (0.41), 4.158 (1.15), 4.167 (1.28), 4.175 (2.02), 4.184 (1.99), 4.193 (1.26), 4.201 (1.06), 6.480 (1.73), 6.508 (2.23), 6.539 (4.73), 8.016 (2.36), 8.588 (2.64), 8.592 (2.56).

### Intermediate 502

### tert-butyl (3R)-2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazole]-1-carboxylate

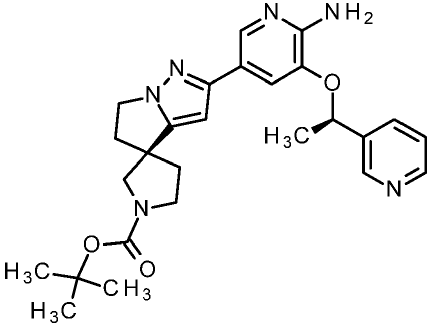

Tert-Butyl (3R)-2'-[(trifluoromethanesulfonyl)oxy]-5',6'-dihydrospiro[pyrrolidine-3,4'-pyrrolo[1,2-b]pyrazole]-1-carboxylate (37.8 mg, 92.0 µmol), {6-amino-5-[(1R)-1-(pyridin-3-yl)ethoxy]pyridin-3-yl}boronic acid (220 mg, 13 % purity, 110 µmol) and potassium phosphate (550 µl, 0.50 M, 280 µmol) were dissolved in degassed 1,4-dioxane (2.3 mL). XPhos Pd G2 (3.62 mg, 4.60 µmol) was added and the mixture was stirred for 3 h at 100 °C. The reaction mixture was allowed to cool down, diluted with dichloromethane and filtered. The organic phase was washed with water and brine, dried and concentrated. The residue was purified by column chromatography (silica gel, dichloromethane / methanol (0 - 10 %) gradient) to give 31.4 mg (72 % yield) of the title compound.
LC-MS (Method 1): Rt = 1.07 min; MS (ESlpos): m/z = 477 [M+H]⁺
¹H-NMR (400 MHz, CHLOROFORM-d) δ [ppm]: 1.174 (0.77), 1.183 (0.45), 1.388 (7.90), 1.427 (5.03), 1.596 (0.64), 1.614 (1.11), 1.629 (5.38), 1.645 (4.91), 1.936 (0.57), 1.950 (0.48), 2.513 (0.41), 2.530 (0.40), 3.360 (0.51), 3.419 (0.71), 3.444 (0.94), 3.452 (0.75), 3.678 (0.87), 4.136 (0.67), 4.152 (1.18), 4.171 (0.60), 4.697 (2.08), 5.233 (6.62), 5.447 (0.60), 5.463 (0.58), 5.999 (0.61), 6.024 (1.00), 7.194 (16.00), 7.205 (0.92), 7.216 (2.66), 7.220 (2.19), 7.236 (0.81), 7.634 (0.63), 7.653 (0.55), 7.922 (2.30), 7.926 (2.28), 8.463 (1.20), 8.467 (1.26), 8.475 (1.23), 8.479 (1.21), 8.601 (1.44), 8.606 (1.44).

### Intermediate 503

### tert-butyl (3R)-2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazole]-1-carboxylate

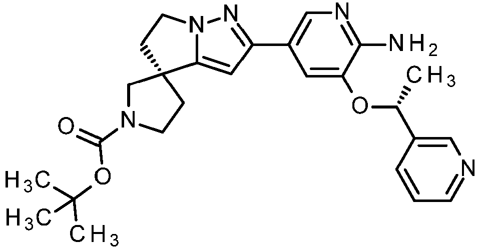

3-[(1R)-1-(Pyridin-3-yl)ethoxy]-5-(4,4,5,5-tetramethyl-1,3,2-dioxaborolan-2-yl)pyridin-2-amine (350 mg, 33 % purity, 338 µmol) was dissolved in 1,4-dioxane (2 mL) under argon. Tert-Butyl (3R)-2'-[(trifluoromethanesulfonyl)oxy]-5',6'-dihydrospiro[pyrrolidine-3,4'-pyrrolo[1,2-b]pyrazole]-1-carboxylate (127 mg, 308 µmol), potassium phosphate (1.8 ml, 0.50 M, 920 µmol) and XPhos Pd G2 (12.1 mg, 15.4 µmol) were added and the mixture was stirred for 1 h at 100 °C. The reaction mixture was allowed to cool down and diluted with dichloromethane. The phases were separated, the organic phase was washed with brine, dried over a hydrophobic filter and concentrated. The residue was purified by preparative HPLC to give 73.0 mg (100 % purity, 50 % yield) of the title compound.
LC-MS (Method 1): Rt = 1.04 min; MS (ESlpos): m/z = 477 [M+H]⁺
¹H-NMR (400MHz, DMSO-d₆): δ [ppm]= 1.39 -1.43 (m, 9H), 1.60 (d, 3H), 1.96 - 2.11 (m, 2H), 3.36 - 3.45 (m, 3H), 3.46 - 3.55 (m, 1H), 4.08 - 4.19 (m, 2H), 5.70 (q, 1H), 5.90 (s, 2H), 6.25 - 6.31 (m, 1H), 7.32 (s, 1H), 7.37 (dd, 1H), 7.87 (d, 1H), 7.89 - 7.94 (m, 1H), 8.47 (dd, 1H), 8.70 (d, 1H).

### Intermediate 504

### tert-butyl 2'-{6-amino-5-[(6,7-dihydro-5H-cyclopenta[b]pyridin-7-yl)oxy]pyridin-3-yl}-5',6'-dihydrospiro[pyrrolidine-3,4'-pyrrolo[1,2-b]pyrazole]-1-carboxylate (4 isomers)

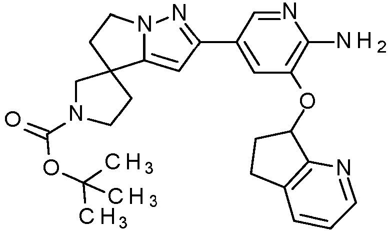

Tert-Butyl 2'-[(trifluoromethanesulfonyl)oxy]-5',6'-dihydrospiro[pyrrolidine-3,4'-pyrrolo[1,2-b]pyrazole]-1-carboxylate (249 mg, 605 µmol), 3-[(6,7-dihydro-5H-cyclopenta[b]pyridin-7-yl)oxy]-5-(4,4,5,5-tetramethyl-1,3,2-dioxaborolan-2-yl)pyridin-2-amine (346 mg, 71 % purity, 695 µmol), dicyclohexyl[2',4',6'-tri(propan-2-yl)[1,1'-biphenyl]-2-yl]phosphane (28.8 mg, 60.5 µmol), XPhos Pd G2 (23.8 mg, 30.2 µmol) and potassium phosphate (3.6 ml, 0.50 M, 1.8 mmol) were combined in degassed 1,4-dioxane (10 mL) and stirred for 1 h at 100 °C. The reaction mixture was allowed to cool down and diluted with dichloromethane and water. The phases were separated and the aqueous phase was extracted with dichloromethane / ethanol 9:1. The combined organic layers were dried and concentrated. The residue was purified by column chromatography (silica gel, ethyl acetate / ethanol (0 - 10 %) gradient) to give 258 mg (87 % yield) of the title compound.
LC-MS (Method 1): Rt = 1.13 min; MS (ESlpos): m/z = 489 [M+H]⁺

### Intermediate 505

### tert-butyl (3'R)-2-[6-amino-5-(difluoromethyl)pyridin-3-yl]-6,7-dihydrospiro[pyrazolo[5,1-c][1,4]oxazine-4,3'-pyrrolidine]-1'-carboxylate

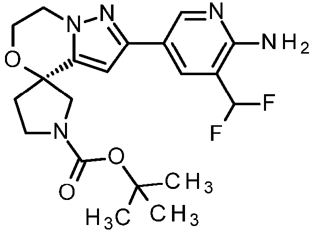

Tert-Butyl (3'R)-2-bromo-6,7-dihydrospiro[pyrazolo[5,1-c][1,4]oxazine-4,3'-pyrrolidine]-1'-carboxylate (527 mg, 1.47 mmol) and 3-(difluoromethyl)-5-(4,4,5,5-tetramethyl-1,3,2-dioxaborolan-2-yl)pyridin-2-amine (970 mg, 43 % purity, 1.54 mmol, **Intermediate 476)** were dissolved in 1,4-dioxane. Potassium phosphate (937 mg, 4.41 mmol) was added and the mixture was degassed. XPhos Pd G2 (57.9 mg, 73.5 µmol) was added and the reaction mixture was stirred for 2 h at 100 °C. The reaction mixture was allowed to cool down, diluted with ethyl acetate and filtered over celite. The organic phase was washed with water and brine, dried over a hydrophobic filter and concentrated. The residue was purified by column chromatography (silica gel, dichloromethane / methanol (0 - 4 %) gradient) to give 490 mg (79 % yield) of the title compound.
LC-MS (Method 1): Rt = 1.10 min; MS (ESlpos): m/z = 422 [M+H]⁺
¹H NMR (400 MHz, DMSO-d₆): δ [ppm] = 1.41 (d, 9 H), 2.14 - 2.31 (m, 2 H), 3.35 - 3.59 (m, 3 H), 3.68 - 3.77 (m, 1 H), 3.94 (s, 1 H), 4.11 (s, 4 H), 6.38 (s, 2 H), 6.70 (d, 1 H), 6.91 - 7.22 (m, 1 H), 7.95 (s, 1 H), 8.43 - 8.51 (m, 1 H).

### Intermediate 506

### tert-butyl (3'R)-2-(6-amino-5-cyanopyridin-3-yl)-6,7-dihydrospiro[pyrazolo[5,1-c][1,4]oxazine-4,3'-pyrrolidine]-1'-carboxylate

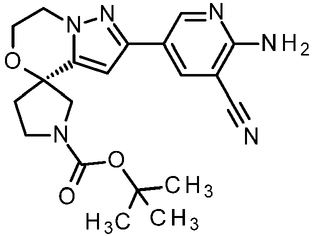

Tert-Butyl (3'R)-2-bromo-6,7-dihydrospiro[pyrazolo[5,1-c][1,4]oxazine-4,3'-pyrrolidine]-1'-carboxylate (300 mg, 837 µmol) and 2-amino-5-(4,4,5,5-tetramethyl-1,3,2-dioxaborolan-2-yl)pyridine-3-carbonitrile (216 mg, 879 µmol, CAS 1246372-66-6 , Intermediate 2) were dissolved in 1,4-dioxane. Potassium phosphate (533 mg, 2.51 mmol) was added and the mixture was degassed. XPhos Pd G2 (32.9 mg, 41.9 µmol) was added and stirred for 2 h at 100 °C. The reaction mixture was allowed to cool down, diluted with ethyl acetate and filtered over celite. The organic phase was washed with water and brine, dried over a hydrophobic filter and concentrated. The residue was purified by column chromatography (silica gel, dichloromethane / methanol (0 - 4 %) gradient) to give 411 mg (80 % purity, 99 % yield) of the title compound.
LC-MS (Method 1): Rt = 1.05 min; MS (ESlpos): m/z = 397 [M+H]⁺
¹H-NMR (400 MHz, DMSO-d6) δ [ppm]: 1.066 (16.00), 1.156 (0.57), 1.401 (2.65), 1.426 (2.26), 2.085 (2.56), 2.518 (0.95), 2.523 (0.70), 3.938 (2.60), 4.115 (1.25), 7.045 (0.76), 8.592 (0.80), 8.597 (0.81).

### Intermediate 507

### tert-butyl (3'R)-2-[6-amino-5-(difluoromethoxy)pyridin-3-yl]-6,7-dihydrospiro[pyrazolo[5,1-c][1,4]oxazine-4,3'-pyrrolidine]-1'-carboxylate

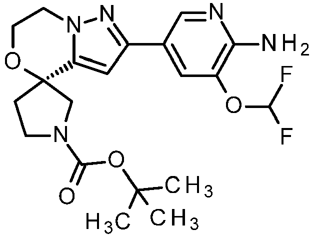

Tert-Butyl (3'R)-2-bromo-6,7-dihydrospiro[pyrazolo[5,1-c][1,4]oxazine-4,3'-pyrrolidine]-1'-carboxylate (300 mg, 837 µmol) and 3-(difluoromethoxy)-5-(4,4,5,5-tetramethyl-1,3,2-dioxaborolan-2-yl)pyridin-2-amine (252 mg, 879 µmol, CAS 1188302-00-2) were dissolved in 1,4-dioxane. Potassium phosphate (533 mg, 2.51 mmol) was added and the mixture was degassed. XPhos Pd G2 (32.9 mg, 41.9 µmol) was added and stirred for 2 h at 100 °C. The reaction mixture was allowed to cool down, diluted with ethyl acetate and filtered over celite. The organic phase was washed with water and brine, dried over a hydrophobic filter and concentrated. The residue was purified by column chromatography (silica gel, dichloromethane / methanol (0 - 4 %) gradient) to give 292 mg (80 % yield) of the title compound.
LC-MS (Method 1): Rt = 1.11 min; MS (ESlpos): m/z = 438 [M+H]⁺
¹H-NMR (400 MHz, DMSO-d6) δ [ppm]: 0.852 (0.50), 1.066 (3.19), 1.156 (1.91), 1.173 (0.48), 1.233 (0.92), 1.401 (16.00), 1.425 (13.52), 1.988 (0.93), 2.233 (0.41), 2.318 (1.01), 2.323 (1.68), 2.327 (2.19), 2.332 (1.52), 2.337 (0.78), 2.518 (6.10), 2.523 (4.43), 2.660 (0.54), 2.665 (1.24), 2.669 (1.73), 2.673 (1.17), 2.679 (0.50), 3.362 (0.58), 3.372 (0.57), 3.390 (0.49), 3.420 (0.71), 3.450 (0.75), 3.475 (0.68), 3.505 (0.91), 3.521 (0.68), 3.544 (1.17), 3.566 (0.58), 3.693 (0.63), 3.722 (1.00), 3.751 (0.44), 3.938 (0.55), 4.111 (7.31), 5.759 (11.94), 6.216 (4.46), 6.657 (1.90), 6.676 (2.24), 6.994 (1.95), 7.178 (3.89), 7.362 (1.69), 7.606 (2.39), 8.188 (2.88), 8.193 (2.82).

### Intermediate 508

### tert-butyl (3R)-2'-[6-amino-5-(trifluoromethoxy)pyridin-3-yl]-5',6'-dihydrospiro[pyrrolidine-3,4'-pyrrolo[1,2-b]pyrazole]-1-carboxylate

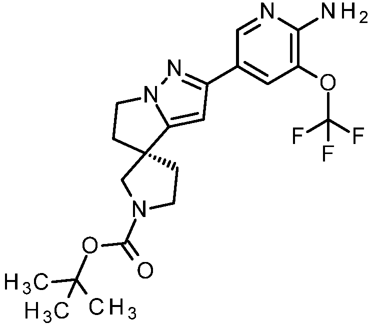

Tert-Butyl (3R)-2'-[(trifluoromethanesulfonyl)oxy]-5',6'-dihydrospiro[pyrrolidine-3,4'-pyrrolo[1,2-b]pyrazole]-1-carboxylate (1.00 g, 2.43 mmol), [6-amino-5-(trifluoromethoxy)pyridin-3-yl]boronic acid (1.35 g, 40 % purity, 2.43 mmol, intermediate 206) and potassium phosphate (15 mL, 0.50 M, 7.3 mmol) were dissolved in 1,4-dioxane. The mixture was degassed, XPhos Pd G2 (95.6 mg, 122 µmol) was added and the reaction mixture was stirred for 3 h at 100 °C. The reaction mixture was allowed to cool down, diluted with water and extracted with ethyl acetate. The combined organic layers were dried over a hydrophobic filter and concentrated. The residue was purified by column chromatography (silica gel, hexane / ethyl acetate (0 - 100 %) gradient) to give 932 mg (97 % purity, 85 % yield) of the title compound.
LC-MS (Method 1): Rt = 1.21 min; MS (ESlpos): m/z = 440 [M+H]⁺
¹H-NMR (400 MHz, DMSO-d6) δ [ppm]: 1.154 (5.17), 1.172 (10.41), 1.190 (4.91), 1.395 (16.00), 1.432 (12.57), 1.987 (15.97), 2.016 (0.72), 2.034 (1.29), 2.047 (1.19), 2.064 (0.84), 2.518 (3.08), 2.523 (2.64), 3.378 (0.56), 3.396 (1.84), 3.407 (6.01), 3.421 (0.94), 3.433 (0.73), 3.490 (0.65), 3.508 (1.18), 3.517 (0.61), 3.523 (0.72), 3.535 (0.70), 4.000 (1.19), 4.017 (3.69), 4.035 (3.69), 4.053 (1.19), 4.141 (0.40), 4.152 (1.12), 4.160 (1.25), 4.169 (2.01), 4.177 (2.01), 4.187 (1.21), 4.195 (1.05), 6.424 (1.71), 6.448 (2.18), 6.496 (4.96), 7.753 (2.30), 8.330 (5.16), 8.335 (4.75).

### Intermediate 509

### tert-butyl (3R)-2'-(6-amino-5-cyanopyridin-3-yl)-5',6'-dihydrospiro[pyrrolidine-3,4'-pyrrolo[1,2-b]pyrazole]-1-carboxylate

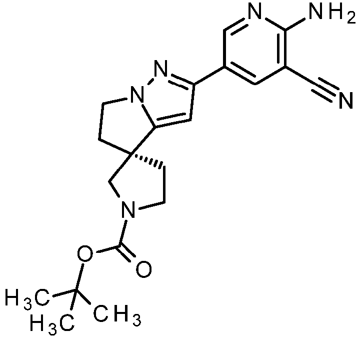

Tert-Butyl (3R)-2'-[(trifluoromethanesulfonyl)oxy]-5',6'-dihydrospiro[pyrrolidine-3,4'-pyrrolo[1,2-b]pyrazole]-1-carboxylate (1.00 g, 2.43 mmol), 2-amino-5-(4,4,5,5-tetramethyl-1,3,2-dioxaborolan-2-yl)pyridine-3-carbonitrile (655 mg, 2.67 mmol, CAS 1246372-66-6) and potassium phosphate (15 mL, 0.50 M, 7.3 mmol) were dissolved in 1,4-dioxane (35 mL). The mixture was degassed. XPhos Pd G2 (95.6 mg, 122 µmol) was added and stirred for 3 h at 100 °C. The reaction mixture was allowed to cool down, diluted with water and extracted with ethyl acetate. The combined organic layers were dried over a hydrophobic filter and concentrated. The residue was purified by column chromatography (silica gel, hexane / ethyl acetate (0 - 100 %) gradient) to give 915 mg (100 % purity, 99 % yield) of the title compound.
LC-MS (Method 1): Rt = 1.07 min; MS (ESlpos): m/z = 381 [M+H]⁺
¹H NMR (DMSO-d₆, 400 MHz): δ (ppm) 8.61 (d, 1H), 8.15 (d, 1H), 6.99 (s, 2H), 6.46 and 6.48 (2s, 1H), 4.12 - 4.23 (m, 2H), 3.47 - 3.55 (m, 1H), 3.35 - 3.46 (m, 3H), 2.52 - 2.59 (m, 2H), 2.00 - 2.09 (m, 2H), 1.40 and 1.46 (2s, 9H).

### Intermediate 510

### tert-butyl (3R)-2'-[6-amino-5-(difluoromethoxy)pyridin-3-yl]-5',6'-dihydrospiro[pyrrolidine-3,4'-pyrrolo[1,2-b]pyrazole]-1-carboxylate

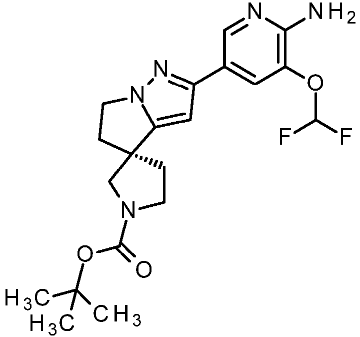

Tert-Butyl (3R)-2'-[(trifluoromethanesulfonyl)oxy]-5',6'-dihydrospiro[pyrrolidine-3,4'-pyrrolo[1,2-b]pyrazole]-1-carboxylate (1.00 g, 2.43 mmol), 3-(difluoromethoxy)-5-(4,4,5,5-tetramethyl-1,3,2-dioxaborolan-2-yl)pyridin-2-amine (765 mg, 2.67 mmol, CAS 1188302-00-2) and potassium phosphate (15 mL, 0.50 M, 7.3 mmol) were dissolved in 1,4-dioxane (35 mL). The mixture was degassed. XPhos Pd G2 (95.6 mg, 122 µmol) was added and stirred for 3 h at 100 °C. The reaction mixture was allowed to cool down, diluted with water and extracted with ethyl acetate. The combined organic layers were dried over a hydrophobic filter and concentrated. The residue was purified by column chromatography (silica gel, hexane / ethyl acetate (0 - 100 %) gradient) to give 996 mg (99 % purity, 96 % yield) of the title compound.
LC-MS (Method 1): Rt = 1.12 min; MS (ESlpos): m/z = 422 [M+H]⁺
¹H-NMR (400 MHz, DMSO-d6) δ [ppm]: 1.066 (0.70), 1.154 (1.84), 1.172 (3.62), 1.190 (1.69), 1.397 (16.00), 1.433 (12.83), 1.988 (6.38), 2.031 (1.09), 2.040 (1.13), 2.047 (1.15), 2.068 (0.78), 2.332 (0.49), 2.518 (3.16), 2.523 (2.57), 2.673 (0.48), 3.409 (6.16), 3.421 (1.00), 3.431 (0.72), 3.488 (0.63), 3.506 (1.16), 3.515 (0.61), 3.521 (0.76), 3.533 (0.68), 4.000 (0.48), 4.017 (1.45), 4.035 (1.44), 4.053 (0.46), 4.149 (1.12), 4.157 (1.28), 4.166 (2.05), 4.174 (2.10), 4.183 (1.26), 4.192 (1.12), 6.157 (5.25), 6.376 (1.77), 6.396 (2.25), 6.994 (2.43), 7.178 (4.89), 7.363 (2.10), 7.617 (2.81), 8.200 (5.35), 8.204 (5.02).

### Intermediate 511

### tert-butyl (3R)-2'-{6-amino-5-[(1R)-1-(2,6-dichloro-3-fluorophenyl)ethoxy]pyridin-3-yl}-5',6'-dihydrospiro[pyrrolidine-3,4'-pyrrolo[1,2-b]pyrazole]-1-carboxylate

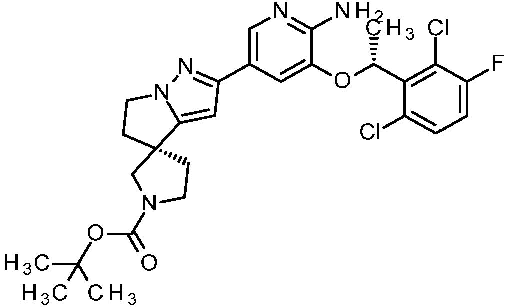

3-[(1R)-1-(2,6-Dichloro-3-fluorophenyl)ethoxy]-5-(4,4,5,5-tetramethyl-1,3,2-dioxaborolan-2-yl)pyridin-2-amine (560 mg, 1.31 mmol), tert-butyl (3R)-2'-[(trifluoromethanesulfonyl)oxy]-5',6'-dihydrospiro[pyrrolidine-3,4'-pyrrolo[1,2-b]pyrazole]-1-carboxylate (593 mg, 1.44 mmol) and potassium phosphate (7.9 ml, 0.50 M, 3.9 mmol) were dissolved in 1,4-dioxane and the mixture was degassed. XPhos Pd G2 (51.5 mg, 65.6 µmol) was added and stirred for 2 h at 100 °C. The reaction mixture was allowed to cool down, diluted with water and extracted with ethyl acetate. The combined organic layers were washed with brine, dried over sodium sulfate, filtered and concentrated. The residue was purified by column chromatography (silica gel, hexane / ethyl acetate (0 - 90 %) and dichloromethane / methanol (0-10 %) gradient) to give 445 mg (69 % purity, 42 % yield) of the title compound.
LC-MS (Method 1): Rt = 1.38 min; MS (ESlpos): m/z = 562 [M+H]⁺
¹H-NMR (400 MHz, DMSO-d6) δ [ppm]: 1.053 (0.66), 1.066 (9.51), 1.070 (0.72), 1.154 (2.72), 1.172 (5.70), 1.190 (2.97), 1.352 (0.53), 1.389 (13.38), 1.415 (8.44), 1.437 (7.44), 1.776 (4.21), 1.793 (4.21), 1.948 (0.49), 1.963 (0.54), 1.987 (11.53), 2.008 (0.96), 2.024 (0.95), 2.036 (0.84), 2.323 (0.46), 2.327 (0.65), 2.332 (0.50), 2.345 (0.62), 2.362 (1.00), 2.379 (0.62), 2.518 (2.43), 2.523 (1.59), 2.530 (0.79), 2.669 (0.53), 3.159 (15.51), 3.171 (16.00), 3.384 (2.74), 3.404 (0.99), 3.423 (0.99), 3.455 (0.52), 3.473 (0.65), 3.489 (0.42), 3.916 (0.68), 3.933 (1.30), 3.939 (1.03), 3.950 (0.68), 3.999 (0.84), 4.017 (2.44), 4.034 (2.40), 4.052 (0.80), 4.089 (1.47), 4.098 (2.75), 4.112 (3.12), 4.124 (2.02), 4.142 (0.73), 4.148 (0.72), 5.206 (3.17), 5.787 (1.71), 6.035 (0.41), 6.051 (0.44), 6.133 (0.89), 6.157 (1.04), 7.066 (0.61), 7.081 (0.69), 7.417 (0.78), 7.438 (1.59), 7.460 (1.12), 7.530 (0.71), 7.542 (0.77), 7.552 (0.61), 7.565 (0.52), 7.882 (0.88), 9.578 (0.63).

### Intermediate 512

### tert-butyl (3R)-2'-{6-amino-5-[(1R)-1-(pyridin-4-yl)ethoxy]pyridin-3-yl}-5',6'-dihydrospiro[pyrrolidine-3,4'-pyrrolo[1,2-b]pyrazole]-1-carboxylate

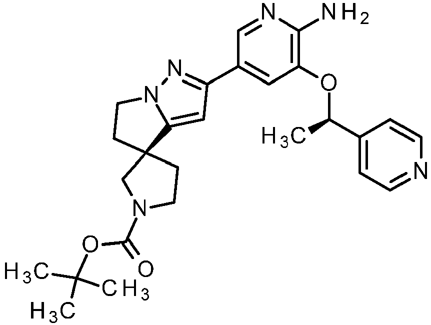

Tert-Butyl (3R)-2'-[(trifluoromethanesulfonyl)oxy]-5',6'-dihydrospiro[pyrrolidine-3,4'-pyrrolo[1,2-b]pyrazole]-1-carboxylate (188 mg, 456 µmol), {6-amino-5-[(1R)-1-(pyridin-4-yl)ethoxy]pyridin-3-yl}boronic acid (500 mg, 26 % purity, 502 µmol, **Intermediate 480)** and potassium phosphate (2.2 ml, 1.4 mmol) were dissolved in degassed 1,4-dioxane (2.3 mL). XPhos Pd G2 (17.9 mg, 22.8 µmol) was added and the mixture was stirred for 2 h at 100 °C. {6-Amino-5-[(1R)-1-(pyridin-4-yl)ethoxy]pyridin-3-yl}boronic acid (152 mg, 26 % purity, 139 µmol), potassium phosphate (0.7 ml, 0.4 mmol) and XPhos Pd G2 (10 mg, 12.7 µmol) were added again and stirred for 1 h at 100 °C. The reaction mixture was allowed to cool down, diluted with ethyl acetate and filtered. The filtrate was washed with water and brine. The organic phase was dried and concentrated. The residue was purified by column chromatography (silica gel, dichloromethane / methanol (0 - 10 %) gradient) to give 54.0 mg (25 % yield) of the title compound.
LC-MS (Method 1): Rt = 1.27 min; MS (ESlpos): m/z = 477 [M+H]⁺

### Intermediate 513

### tert-butyl (3R)-2'-{6-amino-5-[(1R)-1-phenylethoxy]pyridin-3-yl}-5',6'-dihydrospiro[pyrrolidine-3,4'-pyrrolo[1,2-b]pyrazole]-1-carboxylate

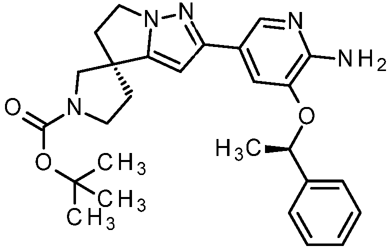

3-[(1R)-1-Phenylethoxy]-5-(4,4,5,5-tetramethyl-1,3,2-dioxaborolan-2-yl)pyridin-2-amine (476 mg, 1.40 mmol), tert-butyl (3R)-2'-[(trifluoromethanesulfonyl)oxy]-5',6'-dihydrospiro[pyrrolidine-3,4'-pyrrolo[1,2-b]pyrazole]-1-carboxylate (634 mg, 1.54 mmol) and potassium phosphate (8.4 ml, 0.50 M, 4.2 mmol) were dissolved in degassed 1,4-dioxane (15 mL). XPhos Pd G2 (55.1 mg, 70.0 µmol) was added and the mixture was stirred for 1 h at 100 °C. The reaction mixture was allowed to reach rt, diluted with water and extracted with ethyl acetate. The combined organic layers were washed with brine, dried over sodium sulfate, filtered and concentrated. The residue was purified by column chromatography (silica gel, hexane / ethyl acetate (20 - 100 %) and ethyl acetate / methanol (5-10 %) gradient) to give 377 mg (57 % yield) of the title compound.
LC-MS (Method 1): Rt = 1.27 min; MS (ESlpos): m/z = 476 [M+H]⁺
¹H-NMR (400 MHz, DMSO-d6) δ [ppm]: 1.154 (4.32), 1.172 (9.21), 1.190 (4.57), 1.389 (16.00), 1.432 (12.28), 1.563 (7.30), 1.579 (7.26), 1.988 (15.51), 2.005 (1.13), 2.017 (1.26), 2.108 (0.45), 2.518 (3.24), 2.523 (2.41), 3.364 (1.51), 3.378 (5.02), 3.405 (1.43), 3.468 (0.50), 3.487 (0.91), 3.498 (0.64), 3.513 (0.56), 3.999 (1.14), 4.017 (3.42), 4.035 (3.29), 4.053 (1.05), 4.102 (1.05), 4.110 (1.10), 4.120 (1.92), 4.126 (1.80), 4.137 (1.16), 4.144 (1.04), 5.552 (0.41), 5.568 (1.36), 5.584 (1.38), 5.599 (0.42), 5.833 (4.23), 6.192 (0.52), 6.224 (1.59), 6.245 (2.01), 7.231 (2.78), 7.245 (2.16), 7.263 (1.52), 7.319 (2.56), 7.338 (4.69), 7.352 (0.88), 7.357 (2.32), 7.453 (4.00), 7.471 (3.17), 7.475 (2.28), 7.838 (3.72), 7.842 (3.63).

### Intermediate 514

### tert-butyl (3'R)-2-{5-[bis(tert-butoxycarbonyl)amino]-6-(trifluoromethyl)pyrazin-2-yl}-6,7-dihydrospiro[pyrazolo[5,1-c][1,4]oxazine-4,3'-pyrrolidine]-1'-carboxylate

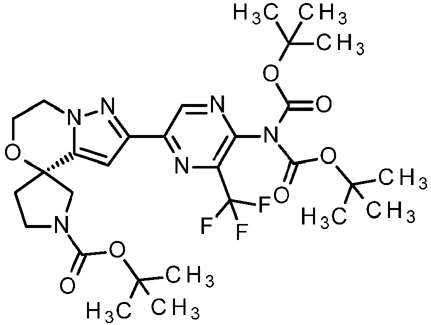

{5-[Bis(tert-butoxycarbonyl)amino]-6-(trifluoromethyl)pyrazin-2-yl}boronic acid (184 mg, 452 µmol, Intermediate 495), tert-butyl (3'R)-2-bromo-6,7-dihydrospiro[pyrazolo[5,1-c][1,4]oxazine-4,3'-pyrrolidine]-1'-carboxylate (154 mg, 430 µmol) and potassium phosphate (2.6 mL, 0.50 M, 1.3 mmol) were suspended in 1,4-dioxane (4.3 mL) under argon. XPhos Pd G2 (16.9 mg, 21.5 µmol) was added and the mixture was stirred for 1.5 h at 100 °C. The reaction mixture was allowed to cool down, diluted with water and extracted with ethyl acetate. The combined organic layers were dried over a hydrophobic filter and concentrated. The residue was purified by column chromatography (silica gel NH2, hexane / ethyl acetate (0 - 100 %) and ethyl acetate / ethanol (0 - 65 %) gradient) to give 168 mg (80 % purity, 49 % yield) of the title compound.
LC-MS (Method 1): Rt = 1.58 min; MS (ESlpos): m/z = 542 [M-Boc+2H]⁺

### Intermediate 515

### tert-butyl (3R)-2'-{5-[bis(tert-butoxycarbonyl)amino]-6-(trifluoromethyl)pyrazin-2-yl}-5',6'-dihydrospiro[pyrrolidine-3,4'-pyrrolo[1,2-b]pyrazole]-1-carboxylate

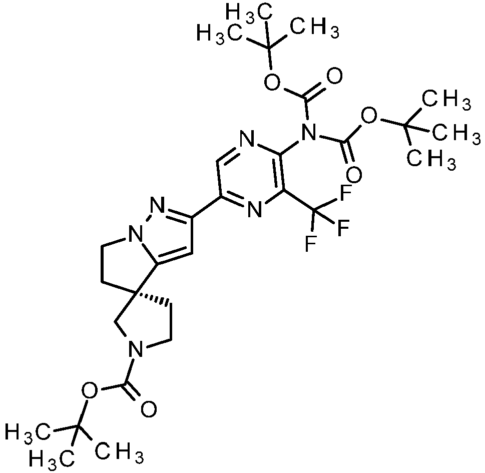

{5-[Bis(tert-butoxycarbonyl)amino]-6-(trifluoromethyl)pyrazin-2-yl}boronic acid (92.0 mg, 226 µmol, Intermediate 495), tert-butyl (3R)-2'-[(trifluoromethanesulfonyl)oxy]-5',6'-dihydrospiro[pyrrolidine-3,4'-pyrrolo[1,2-b]pyrazole]-1-carboxylate (88.5 mg, 215 µmol) and potassium phosphate (5.2 mL, 0.50 M, 2.58 mmol) were dissolved in degassed 1,4-dioxane (2.2 mL) under argon. XPhos Pd G2 (8.47 mg, 10.8 µmol) was added and the mixture was stirred for 1.5 h at 100 °C. The reaction mixture was allowed to cool down, diluted with water and extracted with ethyl acetate. The combined organic layers were dried over a hydrophobic filter and concentrated. The residue was purified by column chromatography (silica gel NH2, hexane / ethyl acetate (0 - 100 %) and ethyl acetate / ethanol (0 - 65 %) gradient) to give 54 mg (38 % yield) of the title compound.
LC-MS (Method 1): Rt = 1.59 min; MS (ESlpos): m/z = 626 [M+H]⁺

### Intermediate 516

### tert-butyl (3R)-2'-{6-amino-5-[(1R)-1-(2,6-difluorophenyl)ethoxy]pyridin-3-yl}-5',6'-dihydrospiro[pyrrolidine-3,4'-pyrrolo[1,2-b]pyrazole]-1-carboxylate

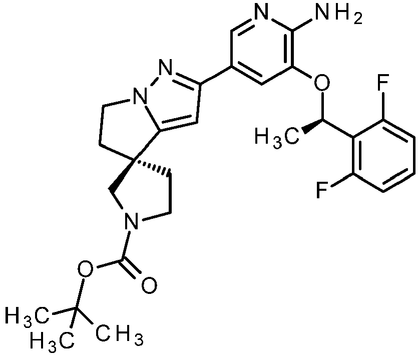

3-[(1R)-1-(2,6-difluorophenyl)ethoxy]-5-(4,4,5,5-tetramethyl-1,3,2-dioxaborolan-2-yl)pyridin-2-amine (327 mg, 49 % purity, 426 µmol) was dissolved in degassed 1,4-dioxane (7 mL). Tert-Butyl (3R)-2'-[(trifluoromethanesulfonyl)oxy]-5',6'-dihydrospiro[pyrrolidine-3,4'-pyrrolo[1,2-b]pyrazole]-1-carboxylate (175 mg, 426 µmol), potassium phosphate (2.6 mL, 0.50 M, 1.3 mmol) and XPhos Pd G2 (16.8 mg, 21.3 µmol) were added and the mixture was stirred for 2 h at 100 °C. The reaction mixture was allowed to cool down, diluted with dichloromethane and washed with water and brine. The organic phase was dried over a hydrophobic filter and concentrated. The residue was purified by preparative HPLC to give 111 mg (91 % purity, 46 % yield) of the title compound.
LC-MS (Method 1): Rt = 1.26 min; MS (ESlpos): m/z = 512 [M+H]⁺
¹H NMR (DMSO-d₆, 400 MHz): δ (ppm) 7.89 (s, 1H), 7.36 - 7.44 (m, 1H), 7.30 (d, 1H), 7.07 - 7.13 (m, 2H), 6.21 and 6.22 (2s, 1H), 5.81 (q, 1H), 5.67 (s, 2H), 4.09 - 4.20 (m, 2H), 3.46 - 3.54 (m, 1H), 3.37 - 3.45 (m, 3H), 2.12 (s, 2H), 1.75 (d, 3H), 1.40 and 1.44 (2s, 9H).

### BOC-Abspaltung

### Intermediate 517

### 5-[(3R)-5',6'-dihydrospiro[pyrrolidine-3,4'-pyrrolo[1,2-b]pyrazol]-2'-yl]-3-[(1R)-1-(3-fluoropyridin-2-yl)ethoxy]pyridin-2-amine-hydrochloride

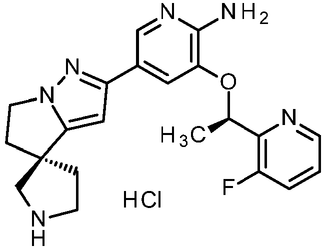

Tert-butyl (3R)-2'-{6-amino-5-[(1R)-1-(3-fluoropyridin-2-yl)ethoxy]pyridin-3-yl}-5',6'-dihydrospiro[pyrrolidine-3,4'-pyrrolo[1,2-b]pyrazole]-1-carboxylate (92.0 mg, 60 % purity, 112 µmol) was dissolved in 1,4-dioxane (1.8 mL). Hydrochloric acid in 1,4-dioxane (280 µL, 4.0 M, 1.1 mmol) was added and the mixture was stirred for 1 h at 40 °C. The reaction mixture was concentrated to dryness to give 142 mg (35 % purity, 103 % yield) of the title compound.
LC-MS (Method 1): Rt = 0.81 min; MS (ESlpos): m/z = 396 [M+H]⁺

### Intermediate 518

### 3-[(1R)-1-(3,5-difluoropyridin-4-yl)ethoxy]-5-[(3R)-5',6'-dihydrospiro[pyrrolidine-3,4'-pyrrolo[1,2-b]pyrazol]-2'-yl]pyridin-2-amine-hydrochloride

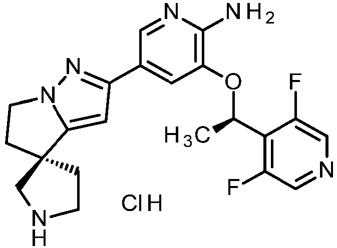

Tert-butyl (3R)-2'-{6-amino-5-[(1R)-1-(3,5-difluoropyridin-4-yl)ethoxy]pyridin-3-yl}-5',6'-dihydrospiro[pyrrolidine-3,4'-pyrrolo[1,2-b]pyrazole]-1-carboxylate (108 mg, 60 % purity, 126 µmol) was dissolved in 1,4-dioxane (2.1 mL). Hydrochloric acid in 1,4-dioxane (320 µL, 4.0 M, 1.3 mmol) was added and the mixture was stirred for 1 h at 40 °C. The reaction mixture was concentrated to dryness to give 139 mg (40 % purity, 98 % yield) of the title compound. LC-MS (Method 1): Rt = 0.84 min; MS (ESlpos): m/z = 414 [M+H]⁺

### Intermediate 519

### 5-[(3R)-5',6'-dihydrospiro[pyrrolidine-3,4'-pyrrolo[1,2-b]pyrazol]-2'-yl]-3-[1-(1-methyl-1H-pyrazol-5-yl)ethoxy]pyridin-2-amine-hydrochloride (diastereomer 1 and diastereomer 2)

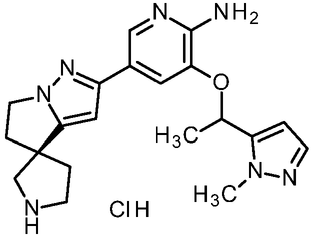

Tert-butyl (3R)-2'-{6-amino-5-[1-(1-methyl-1H-pyrazol-5-yl)ethoxy]pyridin-3-yl}-5',6'-dihydro-1H-spiro[pyrrolidine-3,4'-pyrrolo[1,2-b]pyrazole]-1-carboxylate (150 mg, 80 % purity, 250 µmol) was dissolved in 1,4-dioxane (4.1 mL). Hydrochloric acid in 1,4-dioxane (630 µL, 4.0 M, 2.5 mmol) was added and the reaction mixture was stirred for 1 h at 40 °C. The reaction mixture was concentrated to give 120 mg (80 % purity, 92 % yield) of the tilte compound which was used without further purification in the next step.
LC-MS (Method 1): Rt = 0.75 min; MS (ESlpos): m/z = 381 [M+H]⁺

### Intermediate 520

### trifluoroacetic acid-5-(5',6'-dihydrospiro[azetidine-3,4'-pyrrolo[1,2-b]pyrazol]-2'-yl)-3-[(1R)-1-phenylethoxy]pyridin-2-amine

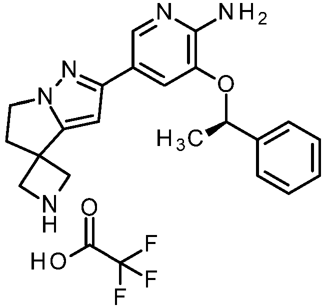

Tert-butyl 2'-{6-amino-5-[(1R)-1-phenylethoxy]pyridin-3-yl}-5',6'-dihydrospiro[azetidine-3,4'-pyrrolo[1,2-b]pyrazole]-1-carboxylate (2.39 g, 5.18 mmol) was dissolved in dichloromethane (25 mL). Trifluoroacetic acid (25 mL, 320 mmol) was added and the mixture was stirred for 1 h at rt. The reaction mixture was concentrated to give 5.1 g (207 % yield) of the title compound.
LC-MS (Method 2): Rt = 0.59 min; MS (ESlpos): m/z = 362 [M+H]⁺
¹H NMR (400 MHz, DMSO-*d*₆, 22°C) δ ppm 1.61 (d, 3 H), 2.91 (t, 2 H), 4.09 (t, 2 H), 4.18 (br s, 4 H), 5.72 (q, 1 H), 6.63 (s, 1 H), 7.23 - 7.29 (m, 1 H), 7.35 (t, 2 H), 7.42 (s, 1 H), 7.46 - 7.50 (m, 2 H), 7.84 (d, 1 H), 9.04 (br s, 2 H).

### Intermediate 521

### 5-[(3R)-5',6'-dihydrospiro[pyrrolidine-3,4'-pyrrolo[1,2-b]pyrazol]-2'-yl]-3-[(1R)-1-(pyridin-3-yl)ethoxy]pyridin-2-amine hydrogen chloride (1/1)

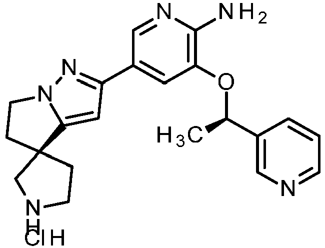

Tert-Butyl (3R)-2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazole]-1-carboxylate (31.0 mg, 65.0 µmol) was dissolved in 1,4-dioxane (0.8 mL). Hydrochloric acid (110 µL, 4M in 1,4-dioxane) was added and the mixture was stirred for 1 h at room temperature. The reaction mixture was concentrated to give 36.0 mg (134 % yield) of the title compound.
LC-MS (Method 1): Rt = 0.76 min; MS (ESlpos): m/z = 377 [M+H]⁺
¹H-NMR (400 MHz, DMSO-d6) δ [ppm]: 1.107 (0.50), 1.232 (0.54), 1.593 (16.00), 1.664 (2.10), 1.680 (2.28), 1.987 (0.54), 2.153 (0.47), 2.174 (0.50), 2.327 (1.06), 2.332 (0.77), 2.518 (5.23), 2.523 (3.40), 2.669 (1.18), 2.673 (0.89), 3.384 (0.70), 3.429 (0.42), 3.990 (0.45), 4.185 (0.60), 4.200 (0.76), 4.214 (0.77), 4.230 (0.56), 6.658 (2.30), 7.797 (0.91), 7.841 (1.11), 7.844 (0.96), 8.677 (0.43), 8.690 (0.46), 8.960 (0.53).

### Intermediate 522

### 3-(6,7-dihydro-5H-cyclopenta[b]pyridin-7-yloxy)-5-(5',6'-dihydrospiro[pyrrolidine-3,4'-pyrrolo[1,2-b]pyrazol]-2'-yl)pyridin-2-amine dihydrochloride (4 isomers)

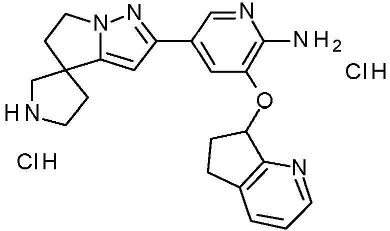

Tert-Butyl 2'-{6-amino-5-[(6,7-dihydro-5H-cyclopenta[b]pyridin-7-yl)oxy]pyridin-3-yl}-5',6'-dihydrospiro[pyrrolidine-3,4'-pyrrolo[1,2-b]pyrazole]-1-carboxylate (308 mg, 630 µmol) was dissolved in 1,4-dioxne (16 mL) and methanol (1.6 mL). Hydrochloric acid in 1,4-dioxane (630 µL, 4.0 M, 2.5 mmol) was added and the mixture was stirred over the weekend at rt. The reaction mixture was concentrated, diluted with toluene and evaporated again to give 446 mg (153 % yield) of the title compound.
LC-MS (Method 1): Rt = 0.82 min; MS (ESlpos): m/z = 389 [M+H]⁺

### Intermediate 523

### 3-(difluoromethyl)-5-[(3'R)-6,7-dihydrospiro[pyrazolo[5,1-c][1,4]oxazine-4,3'-pyrrolidin]-2-yl]pyridin-2-amine

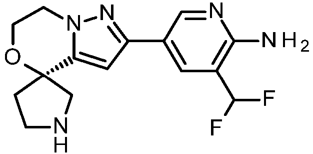

Tert-butyl (3'R)-2-[6-amino-5-(difluoromethyl)pyridin-3-yl]-6,7-dihydrospiro[pyrazolo[5,1-c][1,4]oxazine-4,3'-pyrrolidine]-1'-carboxylate (490 mg, 1.16 mmol, **Intermediate 505)** was dissolved in 1,4-dioxane. Hydrochloric acid in 1,4-dioxane (2.9 mL, 4.0 M, 12 mmol) was added and the mixture was stirred for 1 h at room temperature. Hydrochloric acid in 1,4-dioxane (2.9 mL, 4.0 M, 12 mmol) was added again and the reaction mixture was stirred for 2 h at room temperature.The reaction mixture was concentrated. The residue was diluted with ethyl acetate and extracted with 2 M sodium hydroxide solution. The aqueous phase was extracted with ethyl acetate. The combined organic layers were dried over a hydrophobic filter and concentrated to give 275 mg (95 % purity, 70 % yield) of the title compound.
LC-MS (Method 1): Rt = 0.72 min; MS (ESlpos): m/z = 322 [M+H]⁺

### Intermediate 524

### 2-amino-5-[(3'R)-6,7-dihydrospiro[pyrazolo[5,1-c][1,4]oxazine-4,3'-pyrrolidin]-2-yl]pyridine-3-carbonitrile

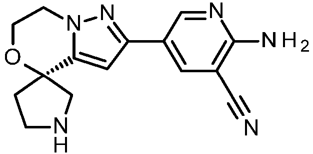

Tert-Butyl (3'R)-2-(6-amino-5-cyanopyridin-3-yl)-6,7-dihydrospiro[pyrazolo[5,1-c][1,4]oxazine-4,3'-pyrrolidine]-1'-carboxylate (411 mg, 80 % purity, 829 µmol, **Intermediate 506)** was dissolved in 1,4-dioxane. Hydrochloric acid in 1,4-dioxane (2.1 mL, 4.0 M, 8.3 mmol) was added and the mixture was stirred for 1 h at room temperature. The reaction mixture was concentrated. The residue was dissolved in ethyl acetate and extracted with 2 M sodium hydoxide solution. The aqueous phase was extracted with ethyl acetate. The combined organic layers were dried over a hydrophobic filter and concentrated to give 215 mg (100 % purity, 87 % yield) of the title compound.
LC-MS (Method 1): Rt = 0.67 min; MS (ESlpos): m/z = 297 [M+H]⁺

### Intermediate 525

### 3-(difluoromethoxy)-5-[(3'R)-6,7-dihydrospiro[pyrazolo[5,1-c][1,4]oxazine-4,3'-pyrrolidin]-2-yl]pyridin-2-amine

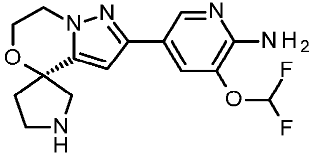

Tert-Butyl (3'R)-2-[6-amino-5-(difluoromethoxy)pyridin-3-yl]-6,7-dihydrospiro[pyrazolo[5,1-c][1,4]oxazine-4,3'-pyrrolidine]-1'-carboxylate (292 mg, 668 µmol, Intermediate 507) was dissolved in 1,4-dioxane. Hydrochloric acid in 1,4-dioxane (1.7 mL, 4.0 M, 6.7 mmol) was added and the mixture was stirred for 1 h at room temperature. Hydrochloric acid in 1,4-dioxane (1.7 mL, 4.0 M, 6.7 mmol) was added again and the resulting mixture was stirred for 2 h at room temperature. The reaction mixture was concentrated. The residue was dissolved in ethyl acetate and extracted with 2 M sodium hydoxide solution. The aqueous phase was extracted with ethyl acetate. The combined organic layers were dried over a hydrophobic filter and concentrated to give 208 mg (92 % purity, 85 % yield) of the title compound.
LC-MS (Method 1): Rt = 0.73 min; MS (ESlpos): m/z = 338 [M+H]⁺

### Intermediate 526

### 5-(5',6'-dihydrospiro[azetidine-3,4'-pyrrolo[1,2-b]pyrazol]-2'-yl)-3-(trifluoromethyl)pyridin-2-amine

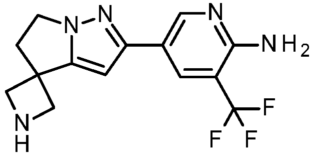

5-(5',6'-Dihydrospiro[azetidine-3,4'-pyrrolo[1,2-b]pyrazol]-2'-yl)-3-(trifluoromethyl)pyridin-2-amine hydrogen chloride (1/1) (830 mg, 2.40 mmol) was suspended in ethyl acetate and extracted with 2 M sodium hydroxide solution. The aqueous phase was extracted with ethyl acetate. The combined organic layers were dried and concentrated to give 583 mg (79 % yield) of the title compound.
LC-MS (Method 1): Rt = 0.81 min; MS (ESlpos): m/z = 310 [M+H]⁺
¹H NMR (DMSO-d₆, 400 MHz): δ (ppm) 8.61 - 8.63 (m, 1H), 8.03 (d, 1H), 6.67 (s, 1H), 6.53 (s, 1H), 4.05 - 4.11 (m, 2H), 3.73 (d, 2H), 3.58 (d, 2H), 2.78 - 2.83 (m, 2H).

### Intermediate 527

### 5-[(3R)-5',6'-dihydrospiro[pyrrolidine-3,4'-pyrrolo[1,2-b]pyrazol]-2'-yl]-3-(trifluoromethoxy)pyridin-2-amine hydrogen chloride (1/1)

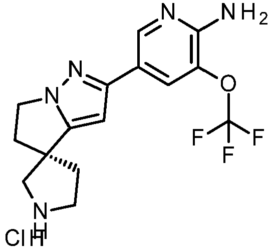

Tert-Butyl (3R)-2'-[6-amino-5-(trifluoromethoxy)pyridin-3-yl]-5',6'-dihydrospiro[pyrrolidine-3,4'-pyrrolo[1,2-b]pyrazole]-1-carboxylate (929 mg, 2.11 mmol, **Intermediate 508)** was dissolved in 1,4-dioxane (9 mL). Hydrochloric acid in 1,4-dioxane (5.3 mL, 4.0 M, 21 mmol) was added dropwise and stirred for 1.5 h at room temperature. The reaction mixture was concentrated to give 888 mg (98 % purity, 110 % yield) of the title compound.
LC-MS (Method 1): Rt = 0.85 min; MS (ESlpos): m/z = 340 [M+H]⁺
¹H NMR (400 MHz, DMSO-*d*₆, 22°C) δ ppm= 2.09 - 2.22 (m, 2 H), 2.53 - 2.62 (m, 1 H), 2.68 - 2.77 (m, 1 H), 3.22 - 3.49 (m, 4 H), 4.17 - 4.25 (m, 2 H), 6.68 (s, 1 H), 8.03 (s, 1 H), 8.33 (d, 1 H), 9.67 - 9.79 (m, 2 H).

### Intermediate 528

### 2-amino-5-[(3R)-5',6'-dihydrospiro[pyrrolidine-3,4'-pyrrolo[1,2-b]pyrazol]-2'-yl]pyridine-3-carbonitrile hydrogen chloride (1/1)

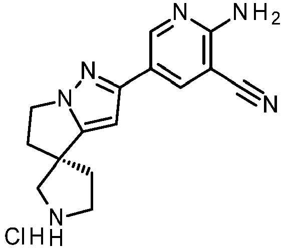

Tert-Butyl (3R)-2'-(6-amino-5-cyanopyridin-3-yl)-5',6'-dihydrospiro[pyrrolidine-3,4'-pyrrolo[1,2-b]pyrazole]-1-carboxylate (915 mg, 2.41 mmol, **Intermediate 509)** was dissoved in 1,4-dioxane (10 mL). Hydochloric acid in 1,4-dioxane (6.0 ml, 4.0 M, 24 mmol) was added dropwise and the mixture was stirred over night at room temperature. The reaction mixture was concentrated to give 852 mg (112 % yield) of the title compound.
LC-MS (Method 1): Rt = 0.69 min; MS (ESlpos): m/z = 281 [M+H]⁺
¹H NMR (400 MHz, DMSO-*d*₆) δ ppm= 2.10 - 2.22 (m, 2 H), 2.53 - 2.61 (m, 1 H), 2.68 - 2.76 (m, 1 H), 3.21 - 3.48 (m, 4 H), 4.14 - 4.25 (m, 2 H), 6.63 (s, 1 H), 8.23 (d, 1 H), 8.59 (d, 1 H), 9.67 - 9.87 (m, 2 H).

### Intermediate 529

### 3-(difluoromethoxy)-5-[(3R)-5',6'-dihydrospiro[pyrrolidine-3,4'-pyrrolo[1,2-b]pyrazol]-2'-yl]pyridin-2-amine hydrogen chloride (1/1)

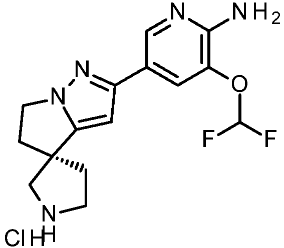

Tert-Butyl (3R)-2'-[6-amino-5-(difluoromethoxy)pyridin-3-yl]-5',6'-dihydrospiro[pyrrolidine-3,4'-pyrrolo[1,2-b]pyrazole]-1-carboxylate (996 mg, 2.36 mmol, **Intermediate 510)** was dissoved in 1,4-dioxane (10 mL). Hydochloric acid in 1,4-dioxane (5.9 ml, 4.0 M, 24 mmol) was added dropwise and the mixture was stirred over night at room temperature. The reaction mixture was concentrated to give 996 mg (100 % purity, 118 % yield) of the title compound.
LC-MS (Method 1): Rt = 0.76 min; MS (ESlpos): m/z = 322 [M+H]⁺
¹H NMR (400 MHz, DMSO-*d*₆) δ ppm= 2.10 - 2.23 (m, 2 H), 2.54 - 2.62 (m, 1 H), 2.70 - 2.78 (m, 1 H), 3.22 - 3.47 (m, 4 H), 4.17 - 4.28 (m, 2 H), 6.72 (s, 1 H), 7.42 (t, 1 H), 8.03 - 8.05 (m, 1 H), 8.19 (d, 1 H), 9.85 (br s, 2 H).

### Intermediate 530

### 5-[(3S)-5',6'-dihydrospiro[pyrrolidine-3,4'-pyrrolo[1,2-b]pyrazol]-2'-yl]-3-(trifluoromethyl)pyridin-2-amine hydrogen chloride (1/1)

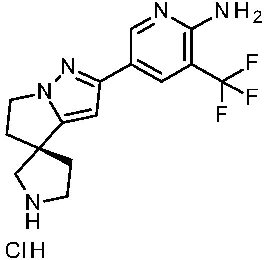

Tert-Butyl (3S)-2'-[6-amino-5-(trifluoromethyl)pyridin-3-yl]-5',6'-dihydrospiro[pyrrolidine-3,4'-pyrrolo[1,2-b]pyrazole]-1-carboxylate (1.19 g, 2.81 mmol) was dissolved in 1,4-dioxane (14 mL). Hydrochloric acid in 1,4-dioxane (14 ml, 4.0 M, 56 mmol) was added and the mixture was stirred over night at room temperature. The reaction mixture was concentrated to give 1.17 g (116 % yield) of the title compound.
LC-MS (Method 1): Rt = 0.84 min; MS (ESlpos): m/z = 324 [M+H]⁺
¹H-NMR (400 MHz, DMSO-d6) δ [ppm]: 1.064 (4.59), 2.083 (1.74), 2.131 (0.53), 2.144 (1.48), 2.156 (1.54), 2.163 (3.38), 2.170 (2.26), 2.183 (1.94), 2.188 (1.87), 2.518 (5.37), 2.523 (4.15), 2.562 (1.04), 2.566 (0.88), 2.579 (1.60), 2.594 (1.80), 2.613 (1.04), 2.679 (1.46), 2.698 (1.85), 2.714 (1.46), 2.727 (0.87), 2.731 (1.03), 2.746 (0.68), 3.242 (0.57), 3.257 (1.00), 3.271 (1.17), 3.286 (1.52), 3.304 (0.94), 3.319 (0.48), 3.330 (0.68), 3.347 (1.06), 3.367 (0.90), 3.384 (2.71), 3.392 (1.00), 3.402 (1.22), 3.408 (1.31), 3.420 (1.72), 3.437 (1.52), 3.447 (1.52), 3.467 (0.87), 3.486 (0.52), 4.173 (0.54), 4.185 (1.91), 4.192 (2.00), 4.200 (2.78), 4.204 (2.70), 4.207 (2.52), 4.212 (2.54), 4.219 (2.01), 4.227 (1.80), 4.239 (0.48), 5.079 (1.26), 6.668 (16.00), 8.109 (3.93), 8.113 (3.88), 8.137 (0.68), 8.585 (4.13), 8.588 (3.93), 9.579 (0.93).

### Intermediate 531

### 3-[(1R)-1-(2,6-dichloro-3-fluorophenyl)ethoxy]-5-[(3R)-5',6'-dihydrospiro[pyrrolidine-3,4'-pyrrolo[1,2-b]pyrazol]-2'-yl)pyridin-2-amine hydrogen chloride (1/1)

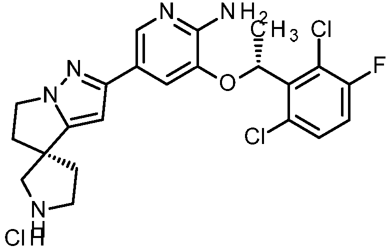

Tert-Butyl (3R)-2'-{6-amino-5-[(1R)-1-(2,6-dichloro-3-fluorophenyl)ethoxy]pyridin-3-yl}-5',6'-dihydrospiro[pyrrolidine-3,4'-pyrrolo[1,2-b]pyrazole]-1-carboxylate (445 mg, 69 % purity, 546 µmol) was dissoved in 1,4-dioxane (2.7 mL). Hydrochloric acid in 1,4-dioxane ((2.7 ml, 4.0 M, 11 mmol) was added and the mixture was stirred over night at room temperature. The reaction mixture was concentrated to give 299 mg (110 % yield) of the title compound.

¹H-NMR (400 MHz, DMSO-d6) δ [ppm]: 1.064 (3.89), 1.230 (0.59), 1.247 (0.59), 1.592 (2.74), 1.839 (5.89), 1.855 (5.78), 1.906 (0.52), 2.068 (2.00), 2.085 (3.81), 2.104 (2.30), 2.118 (0.63), 2.138 (1.63), 2.158 (1.19), 2.167 (1.04), 2.322 (0.81), 2.326 (1.15), 2.332 (0.78), 2.384 (0.56), 2.389 (0.52), 2.402 (1.11), 2.417 (1.00), 2.435 (0.67), 2.518 (5.22), 2.522 (4.07), 2.541 (1.44), 2.547 (1.19), 2.558 (1.07), 2.563 (1.22), 2.573 (0.78), 2.581 (0.63), 2.664 (1.26), 2.669 (1.37), 2.673 (1.04), 2.682 (1.11), 2.699 (0.78), 2.715 (0.67), 3.205 (0.85), 3.220 (1.22), 3.233 (1.59), 3.250 (1.44), 3.266 (0.70), 3.299 (1.33), 3.307 (1.67), 3.316 (1.70), 3.328 (1.59), 3.345 (1.78), 3.363 (1.48), 3.384 (4.33), 3.395 (1.85), 3.406 (1.22), 3.549 (2.15), 3.666 (16.00), 3.751 (2.41), 3.929 (1.33), 3.936 (1.37), 3.947 (1.96), 3.956 (1.63), 3.963 (1.33), 3.970 (1.26), 4.152 (1.33), 4.168 (2.30), 4.187 (1.44), 5.435 (9.33), 6.228 (1.37), 6.245 (1.37), 6.526 (7.07), 7.353 (2.59), 7.356 (2.67), 7.461 (1.26), 7.483 (2.44), 7.504 (1.96), 7.553 (0.44), 7.572 (1.48), 7.584 (1.56), 7.594 (1.15), 7.607 (0.96), 7.827 (2.81), 7.831 (2.81), 8.181 (0.78), 9.502 (0.52), 9.690 (0.56).

### Intermediate 532

### 5-[(3R)-5',6'-dihydrospiro[pyrrolidine-3,4'-pyrrolo[1,2-b]pyrazol]-2'-yl]-3-[(1R)-1-(pyridin-4-yl)ethoxy]pyridin-2-amine hydrogen chloride (1/1)

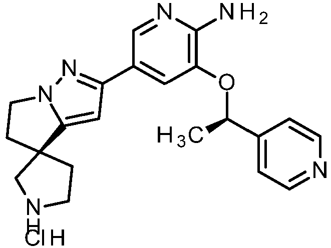

Tert-Butyl (3R)-2'-{6-amino-5-[(1R)-1-(pyridin-4-yl)ethoxy]pyridin-3-yl}-5',6'-dihydrospiro[pyrrolidine-3,4'-pyrrolo[1,2-b]pyrazole]-1-carboxylate (54.0 mg, 113 µmol, **Intermediate 512)** was dissolved in 1,4-dioxane (1.5 mL). Hydrochloric acid in 1,4-dioxane (170 µL, 680 µmol, 4M) was added slowly and the mixture was stirred for 1 h at room temperature. The reaction mixture was concentrated to give 80.0 mg (58 % purity, 99 % yield) of the title compound.
LC-MS (Method 1): Rt = 0.77 min; MS (ESlpos): m/z = 377 [M+H]⁺
¹H-NMR (400 MHz, DMSO-d6) δ [ppm]: 0.850 (0.41), 1.063 (1.61), 1.105 (0.76), 1.154 (0.74), 1.230 (2.97), 1.247 (1.51), 1.388 (0.69), 1.428 (0.56), 1.591 (16.00), 1.618 (0.42), 1.648 (8.27), 1.665 (8.22), 1.906 (0.87), 2.083 (0.65), 2.109 (0.59), 2.121 (0.94), 2.142 (2.00), 2.160 (2.39), 2.178 (1.26), 2.562 (1.73), 2.577 (1.60), 2.595 (0.84), 2.685 (0.76), 2.705 (1.33), 2.720 (1.19), 2.736 (0.84), 2.753 (0.49), 3.190 (0.45), 3.208 (0.92), 3.221 (1.01), 3.237 (1.16), 3.252 (0.65), 3.326 (0.89), 3.337 (0.89), 3.384 (2.61), 3.414 (1.87), 3.423 (1.69), 3.456 (0.72), 3.467 (0.61), 3.487 (0.54), 3.587 (0.56), 3.675 (0.45), 3.699 (0.45), 3.712 (0.40), 4.017 (0.45), 4.088 (0.43), 4.102 (0.46), 4.112 (0.45), 4.180 (2.09), 4.193 (2.91), 4.206 (2.07), 4.318 (0.69), 4.327 (0.54), 4.652 (0.40), 5.758 (3.85), 6.219 (1.21), 6.235 (1.23), 6.668 (7.60), 7.742 (3.35), 7.887 (4.27), 7.890 (4.20), 8.111 (2.61), 8.124 (2.72), 8.137 (1.05), 8.361 (1.53), 8.896 (3.49), 8.910 (3.30), 9.789 (0.75), 9.872 (0.74).

### Intermediate 533

### 5-[(3R)-5',6'-dihydrospiro[pyrrolidine-3,4'-pyrrolo[1,2-b]pyrazol]-2'-yl]-3-[(1R)-1-phenylethoxy]pyridin-2-amine

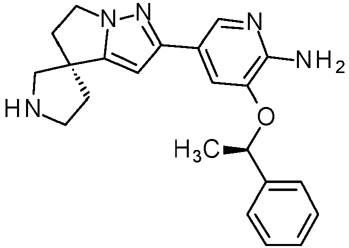

Tert-Butyl (3R)-2'-{6-amino-5-[(1R)-1-phenylethoxy]pyridin-3-yl}-5',6'-dihydrospiro[pyrrolidine-3,4'-pyrrolo[1,2-b]pyrazole]-1-carboxylate (377 mg, 793 µmol) was dissolved in 1,4-dioxane (4 mL). Hydrochloric acid in 1,4-dioxane (4.0 ml, 4.0 M, 16 mmol) was added and the mixture was stirred for 1 h at room temperature. The reaction mixture was concentrated. The residue was purified by preparative HPLC to give 140 mg (47 % yield) of the title compound.
LC-MS (Method 1): Rt = 0.96 min; MS (ESlpos): m/z = 376 [M+H]⁺
¹H NMR (400 MHz, DMSO-*d*₆) δ ppm= 1.57 (d, 3 H), 1.82 - 1.97 (m, 2 H), 2.36 - 2.44 (m, 1 H), 2.75 - 2.80 (m, 1 H), 2.83 - 2.88 (m, 1 H), 2.92 - 3.02 (m, 2 H), 4.01 - 4.12 (m, 2 H), 5.58 (q, 1 H), 5.81 (s, 2 H), 6.23 (s, 1 H), 7.22 - 7.27 (m, 2 H), 7.31 - 7.36 (m, 2 H), 7.44 - 7.49 (m, 2 H), 7.83 (d, 1 H).

### Intermediate 534

### 5-[(3'R)-6,7-dihydrospiro[pyrazolo[5,1-c][1,4]oxazine-4,3'-pyrrolidin]-2-yl]-3-(trifluoromethyl)pyrazin-2-amine

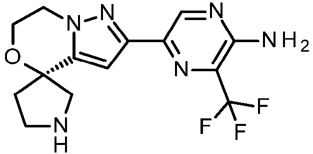

Tert-Butyl (3'R)-2-{5-[bis(tert-butoxycarbonyl)amino]-6-(trifluoromethyl)pyrazin-2-yl}-6,7-dihydrospiro[pyrazolo[5,1-c][1,4]oxazine-4,3'-pyrrolidine]-1'-carboxylate (168 mg, 262 µmol, Intermediate 514) was dissolved in 1,4-dioxane (2.6 mL). Hydrochloric acid in 1,4-dioxane (1.3 mL, 4.0 M, 5.2 mmol) was added and the mixture was stirred for 16 h at room temperature. The reaction mixture was concentrated to give 100 mg (90 % purity, 101 % yield) of the title compound.
LC-MS (Method 1): Rt = 0.79 min; MS (ESlpos): m/z = 342 [M+H]⁺
¹H-NMR (400 MHz, DMSO-d6) δ [ppm]: 1.134 (16.00), 1.300 (0.46), 1.317 (0.26), 1.387 (0.36), 1.500 (0.39), 2.392 (0.26), 2.396 (0.38), 2.401 (0.35), 2.426 (0.20), 2.435 (0.25), 2.450 (0.18), 2.461 (0.36), 2.485 (0.28), 2.515 (0.32), 2.587 (1.54), 2.592 (0.99), 2.734 (0.25), 2.738 (0.33), 2.743 (0.24), 3.350 (0.19), 3.369 (0.28), 3.381 (0.39), 3.399 (0.35), 3.453 (0.24), 3.492 (0.33), 3.505 (0.29), 3.524 (0.51), 3.543 (0.39), 3.555 (0.36), 3.567 (0.32), 3.634 (1.00), 3.718 (0.35), 3.733 (0.41), 3.749 (0.33), 3.986 (2.06), 4.191 (0.99), 4.225 (0.98), 4.236 (1.14), 4.260 (1.16), 4.268 (0.88), 4.273 (0.93), 6.411 (0.59), 6.416 (0.62), 6.871 (3.55), 7.079 (0.18), 7.548 (0.53), 7.553 (0.51), 8.206 (0.19), 8.824 (1.75), 9.491 (0.22), 9.678 (0.19).

### Intermediate 535

### 5-[(3R)-5',6'-dihydrospiro[pyrrolidine-3,4'-pyrrolo[1,2-b]pyrazol]-2'-yl]-3-(trifluoromethyl)pyrazin-2-amine

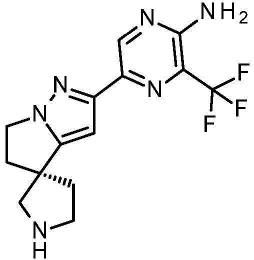

Tert-Butyl (3R)-2'-{5-[bis(tert-butoxycarbonyl)amino]-6-(trifluoromethyl)pyrazin-2-yl}-5',6'-dihydrospiro[pyrrolidine-3,4'-pyrrolo[1,2-b]pyrazole]-1-carboxylate (399 mg, 639 µmol, Intermediate 515) was dissolved in 1,4-dioxane (6.3 mL). Hydrochloric acid in 1,4-dioxane (3.2 mL, 4.0 M, 13 mmol) was added and the mixture was stirred for 16 h at room temperature. The reaction mixture was concentrated to give 243 mg (90 % purity, 106 % yield) of the title compound.
LC-MS (Method 1): Rt = 0.81 min; MS (ESlpos): m/z = 326 [M+H]⁺
¹H-NMR (400 MHz, DMSO-d6) δ [ppm]: 1.064 (16.00), 1.231 (0.55), 2.171 (0.69), 2.190 (0.77), 2.518 (1.21), 2.523 (0.69), 2.726 (0.41), 3.353 (0.56), 3.367 (0.82), 3.372 (0.76), 3.383 (0.90), 3.564 (6.04), 4.210 (0.42), 4.215 (0.41), 4.227 (0.75), 4.235 (0.54), 4.245 (0.42), 4.250 (0.42), 4.528 (0.93), 6.634 (3.33), 8.736 (1.43), 8.738 (1.44).

### Intermediate 536

### 3-[(1R)-1-(2,6-difluorophenyl)ethoxy]-5-[(3R)-5',6'-dihydrospiro[pyrrolidine-3,4'-pyrrolo[1,2-b]pyrazol]-2'-yl]pyridin-2-amine-hydrogen chloride (1/1)

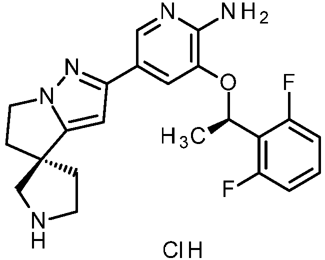

Tert-Butyl (3R)-2'-{6-amino-5-[(1R)-1-(2,6-difluorophenyl)ethoxy]pyridin-3-yl}-5',6'-dihydrospiro[pyrrolidine-3,4'-pyrrolo[1,2-b]pyrazole]-1-carboxylate (110 mg, 215 µmol) was dissolved in 1,4-dioxane (0.9 mL). Hydrochloric acid in 1,4-dioxane (540 µL, 4.0 M, 2.2 mmol) was added and the mixture was stirred for 20 h at 40°C. The reaction mixture allowed to cool down and concentrated to give 120 mg (76 % purity, 94 % yield) of the title compound.
LC-MS (Method 1): Rt = 0.96 min; MS (ESlpos): m/z = 412 [M+H]⁺
¹H NMR (DMSO-d₆, 400 MHz): δ (ppm) 9.56 - 9.70 (m, 2H), 7.89 (br s, 2H), 7.83 (d, 1H), 7.60 (d, 1H), 7.40 - 7.49 (m, 1H), 7.09 - 7.18 (m, 2H), 6.55 (s, 1H), 6.04 (q, 1H), 4.15 - 4.26 (m, 2H), 3.21 - 3.29 (m, 1H), 2.65 - 2.74 (m, 1H), 2.53 - 2.61 (m, 1H), 2.09 - 2.23 (m, 2H), 1.81 (d, 3H).

### Neutralisation

### Intermediate 537

### 5-[(3'R)-6,7-dihydrospiro[pyrazolo[5,1-c][1,4]oxazine-4,3'-pyrrolidin]-2-yl]-3-(trifluoromethyl)pyridin-2-amine

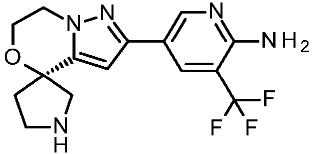

(3'R)-2-[6-Amino-5-(trifluoromethyl)pyridin-3-yl]-6,7-dihydrospiro[pyrazolo[5,1-c][1,4]oxazine-4,3'-pyrrolidin[1]ium] chloride (100 mg, 266 µmol, intermediate 319) was suspended in ethyl acetate and washed with 2 M sodium hydroxide solution. The organic phase was washed with water and brine, dried over sodium sulfate, filtered and concentrated to give 71.7 mg (100 % purity, 79 % yield) of the title compound.
LC-MS (Method 1): Rt = 0.81 min; MS (ESlpos): m/z = 340 [M+H]⁺

### Other

### Intermediate 538

### 2-[(2-methylbut-3-yn-2-yl)oxy]oxane (enantiomer 1 and enantiomer 2)

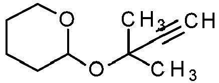

2-Methylbut-3-yn-2-ol (2.00 g, 23.8 mmol) was dissolved in dichloromethane (24 mL) and cooled to 0 °C. 3,4-Dihydro-2H-pyran (2.8 mL, 30 mmol) and toluene-4-sulfonic acid monohydrate (12.3 mg, 71.3 µmol) were added and the mixture was stirred over night at room temperature. The reaction mixture was diluted with saturated sodium bicarbonate solution and the phases were separated. The aqueous phase was extracted with dichloromethane. The combined organic layers were dried and concentrated to give 3.96 g (99 % yield) of the title compound.

¹H NMR (400 MHz, DMSO-*d*₆) δ ppm= 1.35 - 1.54 (m, 10 H), 1.55 - 1.65 (m, 1 H), 1.66 - 1.77 (m, 1 H), 3.38 - 3.45 (m, 1 H), 3.45 (s, 1 H), 3.80 (ddd, 1 H), 5.01 (dd, 1 H).

### Intermediate 539

### 1-(3-{3-methyl-3-[(-oxan-2-yl)oxy]but-1-yn-1-yl}phenyl)ethan-1-ol (mixture of 4 isomers)

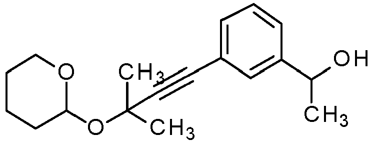

Triethylamine (500 µL, 3.6 mmol) was dissolved in degassed 1,4-dioxane. 2-[(2-Methylbut-3-yn-2-yl)oxy]oxane (182 mg, 1.08 mmol), 1-(3-iodophenyl)ethan-1-ol (295 mg, 1.19 mmol), dichloridopalladium-triphenylphosphane (1/2) (75.9 mg, 108 µmol) and copper (I) iodide (61.8 mg, 324 µmol) were added and the mixture was stirred over night at room temperature. The reaction mixture was concentrated. The crude product was purified by column chromatography (silica gel, dichloromethane / methanol (0 - 5 %) gradient) to give 267 mg (85 % yield) of the title compound.
LC-MS (Method 1): Rt = 1.28 min; MS (ESlneg): m/z = 287 [M-H]⁻

### Intermediate 540

### (3R)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1 and diastereomer 2)

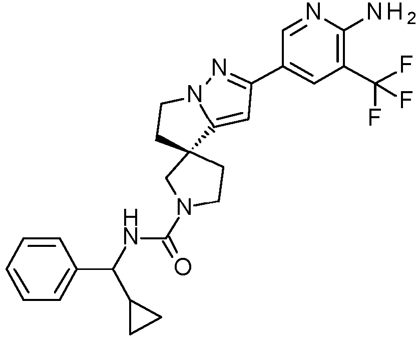

1-Cyclopropyl-1-phenylmethanamine (98.2 mg, 667 µmol) was dissolved in DMF (10 mL) under argon. CDI (108 mg, 667 µmol) and N,N-diisopropylethylamine (390 µL, 2.2 mmol) were added and stirred for 1 h at rt. 5-[(3R)-5',6'-Dihydrospiro[pyrrolidine-3,4'-pyrrolo[1,2-b]pyrazol]-2'-yl]-3-(trifluoromethyl)pyridin-2-amine-hydrogen chloride (1/1) (200 mg, 556 µmol) was added and the mixture was stirred for 2 h at room temperature. The reaction mixture was concentrated, diluted with water and extracted with dichloromethane. The combined organic phases were concentrated. The residue was purified by column chromatography (silica gel, ethyl acetate / methanol (0 - 10 %) gradient) to give 222 mg (80 % yield) of the title compound.
LC-MS (Method 1): Rt = 1.13 min; MS (ESlpos): m/z = 497 [M+H]⁺
¹H NMR (DMSO-d₆, 400 MHz): δ (ppm) 8.55 - 8.59 (m, 1H), 8.00 (s, 1H), 7.36 - 7.44 (m, 2H), 7.25 - 7.33 (m, 2H), 7.16 - 7.23 (m, 1H), 6.65 (d, 1H), 6.55 (s, 2H), 6.43 (s, 1H), 4.19 (t, 2H), 4.06 (t, 1H), 3.52 - 3.62 (m, 1H), 3.40 - 3.51 (m, 3H), 2.52 - 2.57 (m, 2H), 2.01 - 2.14 (m, 2H), 1.14 - 1.27 (m, 1H), 0.42 - 0.54 (m, 2H), 0.27 - 0.40 (m, 2H).

### Intermediate 541

### (3S)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1 and diastereomer 2)

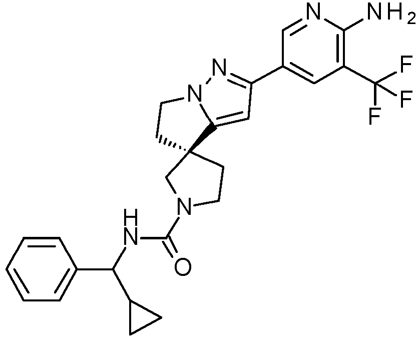

1-Cyclopropyl-1-phenylmethanamine (98.2 mg, 667 µmol) was dissolved in DMF (10 mL). CDI (108 mg, 667 µmol) and N,N-diisopropylethylamine (390 µL, 2.2 mmol) were added and stirred for 1 h at room temperature. 5-[(3S)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (200 mg, 556 µmol) was added and the mixture was stirred for 2 h at room temperature. The reaction mixture was concentrated, diluted with water and extracted with dichloromethane. The combined organic layers were concentrated. The residue was purified by column chromatography (silica gel, ethyl acetate / methanol (0 - 10 %) gradient) to give 227 mg (82,24% yield) of the title compound.
LC-MS (Method 1): Rt = 1.13 min; MS (ESlpos): m/z = 497 [M+H]⁺
¹H NMR (DMSO-d₆, 400 MHz): δ (ppm) 8.57 (s, 1H), 8.00 (s, 1H), 7.37 - 7.43 (m, 2H), 7.25 - 7.32 (m, 2H), 7.15 - 7.23 (m, 1H), 6.65 (d, 1H), 6.55 (s, 2H), 6.43 (s, 1H), 4.19 (t, 2H), 4.03 - 4.09 (m, 1H), 3.52 - 3.61 (m, 1H), 3.40 - 3.50 (m, 3H), 2.52 - 2.57 (m, 2H), 2.01 - 2.13 (m, 2H), 1.15 - 1.26 (m, 1H), 0.43 - 0.54 (m, 2H), 0.28 - 0.40 (m, 2H).

### Oxidation

### Intermediate 542

### 1-(1H-imidazol-2-yl)-2-methylpropan-1-one

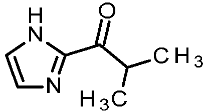

1-(1H-Imidazol-2-yl)-2-methylpropan-1-ol hydrogen chloride (1/1) (200 mg, 1.13 mmol) was dissolved in THF (11 mL). Mangan(IV)oxide (1.48 g, 17.0 mmol) was added and the mixture was stirred for 16 h at room temperature. The reaction mixture was filtered over celite and washed with ethyl acetate. The filtrate was concentrated to give 153 mg (98 % purity, 96 % yield) of the title compound.
LC-MS (Method 1): Rt = 0.62 min; MS (ESlpos): m/z = 139 [M+H]⁺
¹H-NMR (400 MHz, DMSO-d6) δ [ppm]: 1.109 (0.56), 1.114 (16.00), 1.126 (0.62), 1.131 (15.23), 2.518 (0.43), 3.700 (1.04), 3.717 (1.44), 3.734 (0.99), 7.171 (1.86), 7.174 (1.24), 7.412 (1.11), 7.414 (1.19), 7.418 (1.20), 7.420 (1.11).

### Intermediate 543

### 2-(1-chloro-2,2-dimethylpropyl)-1H-imidazol-3-ium chloride

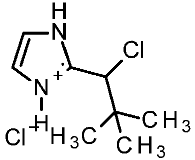

1-(1H-Imidazol-2-yl)-2,2-dimethylpropan-1-ol (60.0 mg, 389 µmol) was dissolved in dichloromethane (5 mL) and cooled to 0 °C. Thionylchloride (57 µL, 780 µmol) was added and the mixture was stirred for 2 h at room temperature. The reaction mixture was concentrated to give 85.0 mg (104 % yield) of the title compound which was used without further purification in the next step.

### Acid Chloride

### Intermediate 544

### 3,3-difluorocyclobutane-1-carbonyl chloride

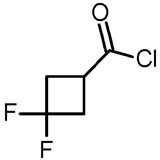

3,3-Difluorocyclobutane-1-carboxylic acid (1.00 g, 7.35 mmol) was dissolved in dichloromethane (20 mL) and DMF (20 µL, 0.26 mmol). Ethanedioyl dichloride (640 µL, 7.35 mmol) was added dropwise and the mixture was stirred for 1 h at room temperature. The reaction mixture was concentrated under reduce pressure (800 mbar) and used in the next step without further purification.

### SEM-Protection

### Intermediate 545

### 1-{[2-(trimethylsilyl)ethoxy]methyl}-1H-imidazole

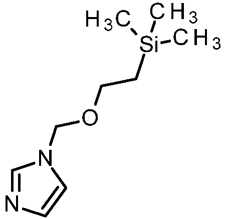

1H-Imidazole (3.00 g, 44.1 mmol) was dissolved in DMF (30 mL), cooled to 0 °C and sodium hydride (2.64 g, 60 % purity, 66.1 mmol) was added. The mixture was stirred for 1 h at 0 °C and [2-(chloromethoxy)ethyl](trimethyl)silane (8.08 g, 48.5 mmol) was added at 0 °C and stirred over night to reach rt. The reaction mixture was diluted with water and extracted with ethyl acetate. The combined organic layers were washed with brine, dried over a hydrophobic filter and concentrated. The residue was purified by column chromatography (silica gel, hexane / ethyl acetate (0 - 100 %) and ethyl acetate / ethanol (0-100 %) gradient) to give 7.10 g (96 % purity, 78 % yield) of the title compound.
LC-MS (Method 1): Rt = 1.10 min; MS (ESlpos): m/z = 199 [M+H]⁺
¹H-NMR (400 MHz, DMSO-d6) δ [ppm]: -0.150 (0.42), 0.016 (0.54), 0.035 (1.86), 0.147 (0.41), 0.853 (4.10), 0.859 (0.53), 0.861 (0.44), 0.872 (4.30), 0.883 (0.40), 0.885 (0.54), 0.892 (4.27), 2.566 (0.45), 2.776 (1.13), 2.778 (1.08), 2.936 (1.33), 3.472 (4.55), 3.479 (0.49), 3.491 (3.46), 3.493 (4.64), 3.506 (0.47), 3.513 (4.55), 5.362 (16.00), 6.972 (2.70), 6.974 (4.35), 6.977 (2.71), 7.302 (3.02), 7.305 (5.56), 7.308 (2.88), 7.813 (2.59), 7.816 (4.08), 7.818 (2.52).

### Intermediate 546

### 1-{[2-(trimethylsilyl)ethoxy]methyl}-1H-1,2,4-triazole

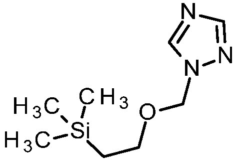

4H-1,2,4-Triazole (500 mg, 7.24 mmol) was provided in acetonitrile (32 mL). N,N-Diisopropylethylamine (1.9 mL, 10.9 mmol) and [2-(chloromethoxy)ethyl](trimethyl)silane (1.4 mL, 7.96 mmol) were added and the mixture was stirred for 19 h at room temperature. The reaction mixture was poured into water and extracted with ethyl acetate. The combined organic layers were washed with half concentrated aqueous sodium chloride solution, dried over a hydrophobic filter and concentrated. The residue was purified by column chromatography (silica gel, hexane / ethyl acetate gradient) to give 1230 mg (85 % yield) of the title compound.

¹H-NMR (400 MHz, DMSO-d6) δ [ppm]: -0.150 (0.42), 0.010 (1.99), 0.037 (1.19), 0.147 (0.42), 0.866 (4.06), 0.874 (0.57), 0.887 (4.33), 0.900 (0.55), 0.907 (4.40), 2.571 (0.68), 2.575 (0.47), 3.588 (4.55), 3.595 (0.56), 3.606 (3.44), 3.609 (4.53), 3.621 (0.53), 3.628 (4.66), 5.558 (16.00), 8.095 (6.25), 8.766 (7.30).

### Intermediate 547

### 1-(1-{[2-(trimethylsilyl)ethoxy]methyl}-1H-imidazol-2-yl)propan-1-one

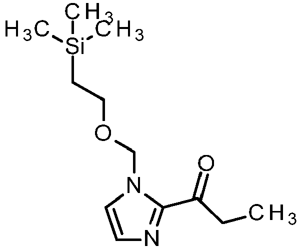

2-Propanoyl-1H-imidazol-1-ium chloride (300 mg, 1.87 mmol, CAS 1314916-37-4), [2-(chloromethoxy)ethyl](trimethyl)silane (330 µL, 1.9 mmol) and N,N-diisopropylethylamine (810 µL, 4.7 mmol) were dissolved in dichloromethane (4.8 mL) and stirred overnight at room temperature. The reaction mixture was diluted with water and extracted with dichloromethane. The combined organic layers were washed with brine, dried over sodium sulfate, filtered and concentrated. The residue was purified by column chromatography (silica gel, hexane / ethyl acetate (0 - 35 %) gradient) to give 339 mg (98 % purity, 70 % yield) of the title compound.
LC-MS (Method 1): Rt = 1.28 min; MS (ESlpos): m/z = 255 [M+H]⁺
¹H-NMR (400 MHz, CHLOROFORM-d) δ [ppm]: 0.025 (0.49), 0.026 (1.37), 0.041 (0.95), 0.045 (1.77), 0.929 (3.98), 0.936 (0.53), 0.947 (2.49), 0.949 (3.30), 0.952 (2.35), 0.963 (0.56), 0.970 (4.18), 1.201 (7.06), 1.219 (15.87), 1.238 (7.72), 3.156 (2.49), 3.175 (7.90), 3.193 (7.69), 3.211 (2.28), 3.565 (4.65), 3.572 (0.56), 3.583 (2.66), 3.586 (3.46), 3.588 (2.68), 3.599 (0.56), 3.606 (4.52), 5.811 (16.00), 7.205 (4.61), 7.207 (4.68), 7.286 (6.23), 7.309 (4.53), 7.311 (4.46).

### Intermediate 548

### 1-{[2-(trimethylsilyl)ethoxy]methyl}-1H-1,2,4-triazole

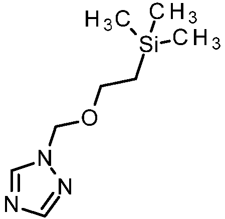

4H-1,2,4-Triazole (3.00 g, 43.4 mmol) was dissolved in DMF (20 mL), cooled to 0 °C and sodium hydride (2.61 g, 60 % purity, 65.2 mmol) was added. The mixture was stirred for 1 h at 0 °C, [2-(chloromethoxy)ethyl](trimethyl)silane (8.69 g, 52.1 mmol) was added and stirred over night at room temperature. The reaction mixture was diluted with water and extracted with ethyl acetate. The combined organic layers were washed with brine, dried over a hydrophobic filter and concentrated. The crude product was purified by column chromatography (silica gel, hexane / ethyl acetate (0 - 100 %) and ethyl acetate / ethanol (0 - 100 %) gradient) to give 5.30 g (61 % yield) of the title compound.
LC-MS (Method 1): Rt = 1.03 min; MS (ESlpos): m/z = 200 [M+H]⁺
¹H-NMR (400 MHz, DMSO-d6) δ [ppm]: -0.183 (0.44), -0.054 (0.47), -0.047 (0.46), -0.041 (3.46), -0.025 (3.91), -0.018 (0.56), -0.011 (0.47), -0.008 (0.55), 0.008 (0.56), 0.013 (0.57), 0.018 (0.46), 0.113 (0.43), 0.834 (4.27), 0.841 (0.58), 0.854 (4.42), 0.866 (0.60), 0.874 (4.21), 3.555 (4.73), 3.562 (0.65), 3.576 (4.65), 3.588 (0.65), 3.595 (4.41), 5.525 (16.00), 5.535 (0.46), 8.061 (6.13), 8.732 (7.46).

### Intermediate 549

### (rac)-1-(1-propyl-1H-pyrazol-5-yl)ethan-1-ol

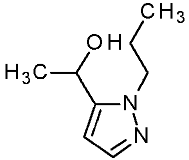

1-Propyl-1H-pyrazole-5-carbaldehyde (300 mg, 2.17 mmol) was dissolved in diethylether (20 mL) and cooled to 0 °C. Methyl magnesium chloride (1.4 ml, 3.0 M, 4.3 mmol) was added and the mixture was stirred for 1 h at 0 °C. A few drops of a saturated ammonium chloride solution were added and stirred for 10 min. The mixture was dried over sodium sulfate, filtered and concentrated to give 355 mg (87 % purity, 92 % yield) of the title compound.
LC-MS (Method 1): Rt = 0.64 min; MS (ESlpos): m/z = 155 [M+H]⁺
¹H-NMR (400 MHz, DMSO-d6) δ [ppm]: 0.798 (0.44), 0.826 (7.33), 0.844 (16.00), 0.863 (7.43), 1.072 (0.86), 1.089 (2.02), 1.107 (0.98), 1.399 (15.76), 1.415 (14.54), 1.716 (0.56), 1.734 (2.79), 1.741 (1.14), 1.753 (5.87), 1.758 (3.31), 1.762 (0.74), 1.767 (1.26), 1.771 (5.31), 1.789 (2.49), 1.808 (0.51), 3.372 (1.00), 3.389 (0.83), 3.583 (1.03), 3.585 (0.56), 3.589 (0.53), 3.593 (0.73), 3.599 (2.33), 3.606 (0.72), 3.608 (0.43), 3.610 (0.49), 3.614 (0.57), 3.616 (0.89), 3.982 (0.51), 4.000 (0.99), 4.016 (1.97), 4.034 (3.97), 4.038 (1.99), 4.052 (1.92), 4.057 (3.83), 4.076 (1.79), 4.091 (1.04), 4.109 (0.49), 4.786 (1.52), 4.800 (2.16), 4.817 (1.56), 4.832 (0.41), 5.234 (7.16), 5.248 (6.28), 6.110 (4.11), 6.115 (4.29), 7.293 (4.38), 7.298 (4.45).

### Intermediate 550

### tert-butyl (2-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-oxoethyl)methylcarbamate

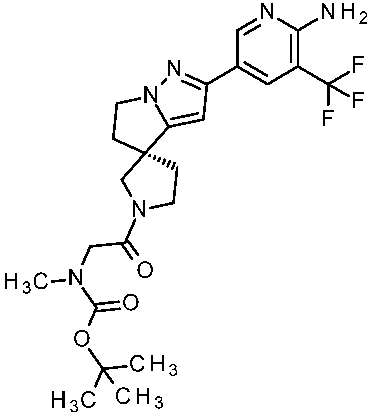

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (100 mg, 278 µmol) and N-(tert-butoxycarbonyl)-N-methylglycine (52.6 mg, 278 µmol, CAS 13734-36-6) were dissolved in DMF (1 mL). N,N-diisopropylethylamine (290 µL, 1.7 mmol) and propane phosphonic anhydride (560 µL, 50 % purity, 830 µmol) were added and the mixture was stirred for 1 h at room temperature. The reaction mixture was diluted with water and extracted with ethyl acetate. The combined organic layers were dried over a hydrophibic filter and concentrated. The residue was purified by preparative HPLC to give 43.5 mg (100 % purity, 32 % yield) of the title compound.
LC-MS (Method 1): Rt = 1.08 min; MS (ESlpos): m/z = 495 [M+H]⁺
¹H-NMR (400 MHz, DMSO-d6) δ [ppm]: 1.318 (13.35), 1.338 (15.27), 1.392 (16.00), 1.404 (15.99), 2.048 (1.29), 2.056 (1.71), 2.075 (10.89), 2.084 (4.83), 2.131 (1.18), 2.148 (2.54), 2.165 (1.22), 2.518 (3.99), 2.523 (3.03), 2.563 (3.70), 2.577 (2.12), 2.778 (4.75), 2.801 (5.94), 2.812 (5.97), 2.841 (5.64), 3.480 (0.49), 3.509 (3.36), 3.519 (1.98), 3.544 (1.32), 3.566 (1.18), 3.575 (1.20), 3.598 (1.11), 3.616 (1.76), 3.642 (0.80), 3.665 (0.42), 3.684 (0.81), 3.708 (0.55), 3.859 (0.43), 3.893 (1.80), 3.947 (1.00), 3.981 (1.22), 4.022 (1.04), 4.048 (1.93), 4.090 (0.77), 4.172 (1.01), 4.185 (2.54), 4.189 (2.64), 4.202 (4.00), 4.219 (2.12), 6.458 (2.11), 6.474 (1.87), 6.521 (2.65), 6.528 (2.72), 6.546 (6.20), 7.979 (1.08), 8.003 (1.60), 8.021 (1.16), 8.567 (1.05), 8.587 (2.04).

### Intermediate 551

### tert-butyl [(2R)-1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-1-oxopropan-2-yl]methylcarbamate

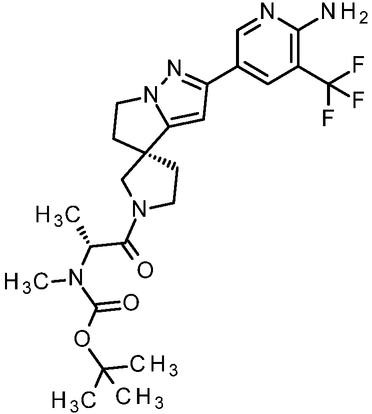

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (100 mg, 278 µmol) and N-(tert-butoxycarbonyl)-N-methyl-D-alanine (56.5 mg, 278 µmol, CAS 19914-38-6) were dissolved in DMF. N,N-Diisopropylethylamine (290 µL, 1.7 mmol) and propane phosphonic anhydride (265 mg, 834 µmol) were added and the mixture was stirred for 1 h at rt. The reaction mixture was diluted with water and extracted with ethyl acetate. The combined organic layers were dried over a hydrophibic filter and concentrated. The residue was purified by preparative HPLC to give 29.3 mg (100 % purity, 21 % yield) of the title compound.
LC-MS (Method 1): Rt = 1.13 min; MS (ESlpos): m/z = 509 [M+H]⁺
¹H-NMR (400 MHz, DMSO-d6) δ [ppm]: 1.122 (2.30), 1.139 (2.48), 1.181 (2.08), 1.196 (3.14), 1.227 (16.00), 1.258 (8.16), 1.409 (10.61), 1.420 (11.99), 1.751 (0.82), 2.045 (1.21), 2.062 (2.21), 2.075 (2.65), 2.084 (1.46), 2.151 (1.27), 2.318 (0.61), 2.323 (1.35), 2.327 (1.94), 2.332 (1.40), 2.336 (0.62), 2.518 (8.82), 2.523 (6.68), 2.576 (1.50), 2.641 (4.91), 2.660 (1.10), 2.665 (1.78), 2.669 (2.33), 2.673 (1.73), 2.678 (0.96), 2.706 (5.26), 2.726 (3.02), 3.476 (2.46), 3.505 (4.87), 3.555 (1.08), 3.572 (1.00), 3.590 (1.55), 3.606 (1.00), 3.620 (0.89), 3.637 (0.63), 3.748 (0.57), 4.126 (0.58), 4.184 (2.53), 4.200 (2.24), 4.801 (0.63), 4.818 (0.62), 4.896 (0.40), 6.409 (1.68), 6.495 (0.97), 6.540 (10.61), 7.992 (1.51), 8.009 (3.71), 8.013 (3.75), 8.568 (1.48), 8.594 (2.93), 8.599 (2.73).

### Intermediate 552

### (rac)-1-(pyrimidin-4-yl)ethan-1-ol

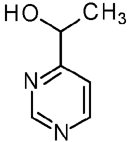

1-(Pyrimidin-4-yl)ethan-1-one (466 mg, 3.82 mmol) was dissolved in methanol (9.3 mL) and cooled to 0 °C. Sodium borohydride (289 mg, 7.63 mmol) was added and allowed to reach rt within 2 h. The reaction mixture was diluted with brine and extracted with ethyl acetate and ethyl acetate / methanol 9:1. The combined organic layers were dried and concentrated. The residue was purified by column chromatography (silica gel, dichloromethane / methanol (0 - 10 %) gradient) to give 500 mg (105 % yield) of the title compound.

¹H NMR (400 MHz, DMSO-d) δ ppm= 1.36 (d, 3 H), 4.67 (qd, 1 H), 5.62 (d, 1 H), 7.59 - 7.62 (m, 1 H), 8.77 (d, 1 H), 9.07 (d, 1 H).

### Acylation

### Intermediate 553

### cyclobutyl(1-{[2-(trimethylsilyl)ethoxy]methyl}-1H-imidazol-2-yl)methanone

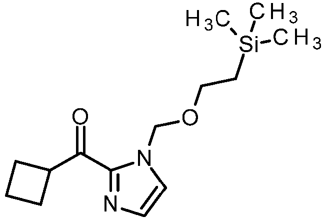

1-{[2-(Trimethylsilyl)ethoxy]methyl}-1H-imidazole (750 mg, 3.78 mmol, Intermediate 545) was dissolved in THF (38 mL) and cooled to -78 °C. N-Butyllithium (1.8 mL, 2.5 M, 4.5 mmol) was added and stirred for 1 h at -78 °C. A solution of cyclobutanecarbonyl chloride (470 µL, 4.2 mmol) in THF (2 mL) was added at -78 °C and the reaction mixture was stirred and allowed to reach rt over night. The reaction mixture was diluted with saturated sodium bicarbonate solution and extracted with ethyl acetate. The organic phase was washed with a saturated sodium bicarbonate solution, water and brine, dried over a hydrophobic filter and concentrated. The residue was purified by column chromatography (silica gel, hexane / ethyl acetate (0 - 100 %) and ethyl acetate / ethanol (0-25 %) gradient) to give 292 mg (90 % purity, 25 % yield) of the title compound.
LC-MS (Method 1): Rt = 1.42 min; MS (ESlpos): m/z = 281 [M+H]⁺
¹H-NMR (400 MHz, DMSO-d6) δ [ppm]: -0.150 (0.46), 0.016 (0.87), 0.019 (0.55), 0.028 (2.69), 0.035 (15.44), 0.040 (1.74), 0.045 (11.20), 0.051 (4.28), 0.054 (1.40), 0.058 (1.16), 0.061 (1.25), 0.066 (0.68), 0.068 (0.45), 0.146 (0.47), 0.878 (4.31), 0.885 (0.92), 0.897 (5.16), 0.905 (0.92), 0.911 (1.00), 0.918 (4.66), 0.925 (0.91), 0.931 (0.67), 0.945 (0.69), 1.313 (0.64), 1.339 (1.00), 1.818 (0.40), 1.833 (0.67), 1.837 (0.62), 1.840 (0.99), 1.843 (1.06), 1.851 (0.87), 1.854 (1.01), 1.857 (0.96), 1.859 (0.92), 1.862 (0.89), 1.867 (1.20), 1.869 (1.12), 1.873 (0.60), 1.876 (0.75), 1.879 (0.94), 1.882 (0.78), 1.889 (0.74), 1.892 (0.72), 1.900 (0.44), 1.902 (0.42), 2.021 (0.45), 2.035 (0.41), 2.044 (1.13), 2.065 (1.90), 2.071 (0.85), 2.088 (1.33), 2.091 (1.30), 2.108 (0.83), 2.115 (1.01), 2.135 (0.68), 2.159 (0.79), 2.168 (0.48), 2.180 (0.91), 2.184 (0.72), 2.190 (0.79), 2.196 (0.65), 2.202 (0.96), 2.204 (0.99), 2.207 (0.74), 2.214 (0.96), 2.218 (1.33), 2.222 (1.65), 2.226 (1.55), 2.229 (1.49), 2.232 (2.44), 2.239 (2.48), 2.243 (2.21), 2.249 (1.96), 2.254 (3.66), 2.256 (3.84), 2.264 (1.63), 2.271 (1.27), 2.277 (3.62), 2.281 (2.58), 2.284 (1.36), 2.298 (1.32), 2.301 (1.22), 2.305 (1.10), 2.308 (1.06), 2.328 (0.47), 2.598 (1.55), 2.603 (1.05), 2.745 (0.40), 2.749 (0.49), 3.484 (0.40), 3.548 (4.68), 3.555 (0.59), 3.568 (5.07), 3.577 (0.60), 3.581 (0.64), 3.588 (4.28), 3.595 (0.42), 3.597 (0.47), 4.316 (1.21), 4.319 (1.24), 4.338 (1.77), 4.341 (1.80), 4.359 (1.12), 4.362 (1.16), 4.789 (0.42), 4.989 (1.24), 5.365 (0.70), 5.800 (16.00), 6.959 (0.55), 6.961 (0.53), 6.964 (0.50), 6.968 (0.58), 7.026 (0.62), 7.034 (0.44), 7.218 (5.94), 7.220 (5.91), 7.340 (0.78), 7.344 (0.69), 7.350 (0.56), 7.353 (0.54), 7.686 (0.75), 7.690 (0.80), 7.725 (6.88), 7.727 (6.81).

### Intermediate 554

### cyclopropyl(6-methoxypyridin-2-yl)methanone

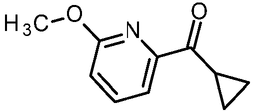

2-Bromo-6-methoxypyridine (910 mg, 4.84 mmol) was dissolved in diethylether (9.1 mL) and cooled to -70 °C. N-Butyllithium (2.0 ml, 2.5 M, 5.1 mmol) was added dropwise over 30 min. The mixture was stirred for 1 h at -70 °C. This solution was added dropwise to a solution of cyclopropanecarbonyl chloride (900 µL, 9.9 mmol) in THF (1.8 mL) over 10 min at -70 °C. The reaction mixture was stirred for 4 h at -70 °C and allowed to reach rt over night. The reaction mixture was diluted with water and extracted twice with ethyl acetate. The combined organic layers were dried over sodium sulfate, filtered and concentrated. The residue was purified by column chromatography (silica gel, hexane / ethyl acetate (0 - 15 %) gradient) to give 389 mg (70 % purity, 32 % yield) of the title compound.
LC-MS (Method 1): Rt = 1.14 min; MS (ESlpos): m/z = 178 [M+H]⁺
¹H-NMR (400 MHz, CHLOROFORM-d) δ [ppm]: 0.915 (0.75), 0.926 (2.41), 0.934 (3.68), 0.944 (2.48), 0.946 (2.68), 0.954 (3.87), 0.962 (1.09), 0.964 (0.98), 1.053 (1.01), 1.055 (1.16), 1.063 (3.80), 1.071 (3.14), 1.073 (2.92), 1.074 (4.46), 1.082 (4.22), 1.091 (0.99), 1.093 (1.59), 1.102 (1.36), 1.110 (0.46), 1.219 (0.47), 1.226 (1.34), 1.230 (0.56), 1.238 (1.56), 1.246 (0.90), 1.583 (0.68), 1.594 (1.22), 1.603 (1.27), 1.606 (0.75), 1.615 (2.54), 1.623 (0.76), 1.626 (1.21), 1.635 (1.08), 1.646 (0.55), 3.487 (0.51), 3.495 (0.52), 3.506 (1.10), 3.518 (0.52), 3.526 (0.50), 3.899 (0.62), 3.932 (1.74), 3.954 (0.75), 4.043 (16.00), 6.943 (1.33), 6.946 (1.35), 6.963 (1.40), 6.966 (1.38), 7.637 (0.96), 7.639 (1.06), 7.655 (1.76), 7.658 (1.58), 7.694 (1.66), 7.712 (0.96), 7.714 (1.55), 7.732 (0.83).

### Intermediate 555

### (3,3-difluorocyclobutyl)(1-{[2-(trimethylsilyl)ethoxy]methyl}-1H-1,2,4-triazol-5-yl)methanone

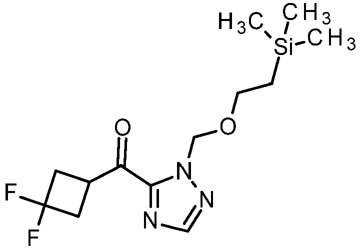

1-{[2-(Trimethylsilyl)ethoxy]methyl}-1H-1,2,4-triazole (1.23 g, 6.17 mmol, Intermediate 546) was provided in THF (20 mL) and cooled to -78 °C. N-Butyllithium (4.2 mL, 1.6 M in hexane, 6.8 mmol) was added dropewise at -78 °C. A solution of 3,3-difluorocyclobutane-1-carbonyl chloride (1.05 g, 6.79 mmol, Intermediate 544) in THF (20 mL) was added dropwise and the mixture was stirred over night at room temperature. The reaction mixture was diluted with a saturated aqueous sodium bicarbonate solution and extracted with ethyl acetate. The combined organic layers were washed with brine, dried over a hydrophobic filter and concentrated. The residue was purified by column chromatography (silica gel, hexane / ethyl acetate gradient) to give 285 mg (12 % yield) of the title compound.
LC-MS (Method 1): Rt = 1.39 min; MS (ESlpos): m/z = 318 [M+H]⁺
¹H-NMR (400 MHz, DMSO-d6) δ [ppm]: -0.226 (0.07), -0.115 (0.03), -0.100 (0.04), -0.085 (0.61), -0.076 (16.00), -0.068 (0.64), -0.062 (0.32), -0.057 (1.65), -0.051 (0.19), -0.049 (0.19), -0.036 (0.07), -0.032 (0.08), -0.022 (0.08), -0.007 (0.02), 0.070 (0.07), 0.749 (0.05), 0.771 (0.04), 0.792 (0.05), 0.813 (0.56), 0.821 (0.09), 0.833 (0.67), 0.846 (0.09), 0.853 (0.59), 1.232 (0.03), 1.353 (0.01), 2.331 (0.05), 2.518 (0.26), 2.523 (0.17), 2.673 (0.06), 2.806 (0.03), 2.829 (0.03), 2.839 (0.14), 2.846 (0.09), 2.858 (0.23), 2.868 (0.16), 2.875 (0.28), 2.880 (0.35), 2.887 (0.23), 2.892 (0.26), 2.897 (0.33), 2.906 (0.23), 2.915 (0.32), 2.927 (0.15), 2.931 (0.17), 2.934 (0.23), 2.948 (0.05), 2.962 (0.06), 2.970 (0.02), 3.150 (0.04), 3.412 (0.03), 3.441 (0.10), 3.462 (0.05), 3.483 (0.05), 3.594 (0.63), 3.601 (0.08), 3.615 (0.71), 3.627 (0.08), 3.634 (0.61), 3.648 (0.03), 3.677 (0.05), 3.706 (0.02), 3.978 (0.03), 3.984 (0.03), 3.999 (0.09), 4.006 (0.10), 4.020 (0.12), 4.027 (0.13), 4.042 (0.08), 4.049 (0.09), 4.065 (0.02), 4.070 (0.02), 5.550 (0.15), 5.743 (0.03), 5.782 (2.10), 5.822 (0.01), 8.061 (0.17), 8.267 (1.54).

### Intermediate 556

### (1-methylcyclobutyl)(1-{[2-(trimethylsilyl)ethoxy]methyl}-1H-imidazol-2-yl)methanone

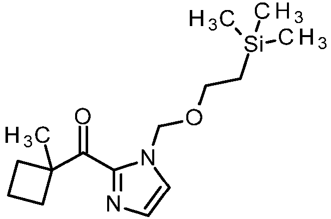

1-{[2-(Trimethylsilyl)ethoxy]methyl}-1H-imidazole (596 mg, 3.01 mmol, Intermediate 545,) was dissolved in THF (9 mL) and cooled to -78 °C. Butyllithium in hexane (1.4 mL, 2.5 M, 3.6 mmol) was added and stirred for 1 h at -78 °C. A solution of N-methoxy-N,1-dimethylcyclobutane-1-carboxamide (520 mg, 3.31 mmol, Intermediate 574) in THF (2 mL) was added, the reaction mixture was allowed to reach rt and stirred over the weekend at rt. The reaction mixture was diluted with a saturated sodium bicarbonate solution and extracted three times with ethyl acetate. The combined organic layers were washed with water and brine, dried over a hydrophobic filter and concentrated. The residue was purified by column chromatography (silica gel, hexane / ethyl acetate (0 - 10 %) gradient) to give 83.0 mg (94 % purity, 9 % yield) of the title compound.
LC-MS (Method 1): Rt = 1.30 min; MS (ESlpos): m/z = 296 [M+H]⁺
¹H-NMR (400 MHz, DMSO-d6) δ [ppm]: -0.064 (0.56), -0.056 (16.00), -0.047 (0.53), 0.822 (0.59), 0.842 (0.76), 0.862 (0.61), 1.196 (0.96), 1.208 (0.77), 1.618 (3.12), 3.082 (0.63), 3.491 (0.66), 3.510 (0.77), 3.530 (0.63), 3.643 (0.58), 5.733 (2.31), 7.179 (1.00), 7.182 (1.00), 7.631 (0.99), 7.634 (0.96).

### Intermediate 557

### cyclohexyl(1-{[2-(trimethylsilyl)ethoxy]methyl}-1H-imidazol-2-yl)methanone

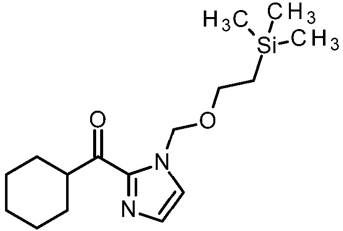

1-{[2-(Trimethylsilyl)ethoxy]methyl}-1H-imidazole (1.50 g, 7.56 mmol, Intermediate 545) was dissolved in THF (70 mL) and cooled to -78 °C. N-Butyllithiumin (3.6 mL, 2.5 M in hexane, 9.1 mmol) was added and stirred for 1 h at -78 °C. A solution of cyclohexanecarbonyl chloride (1.22 g, 8.32 mmol) in THF (5 mL) was added at -78 °C, the reaction mixture was allowed to warm up to rt and stirred over night at room temperature. The reaction mixture was washed with saturated sodium bicarbonate solution, water and brine, dried over a hydrophobic filter and concentrated. The residue was purified by column chromatography (silica gel, hexane / ethyl acetate (0 - 80 %) gradient) to give 540 mg (90 % purity, 21 % yield) of the title compound.
LC-MS (Method 1): Rt = 1.54 min; MS (ESlpos): m/z = 310 [M+H]⁺
¹H-NMR (400 MHz, DMSO-d6) δ [ppm]: -0.077 (0.49), -0.069 (16.00), -0.061 (0.54), -0.009 (1.39), 0.805 (0.50), 0.824 (0.63), 0.845 (0.54), 1.194 (0.47), 1.211 (0.47), 1.243 (0.46), 1.280 (0.59), 1.317 (0.64), 1.345 (0.73), 1.666 (0.41), 1.800 (0.48), 1.813 (0.42), 1.828 (0.46), 2.008 (0.43), 3.352 (0.47), 3.465 (0.58), 3.486 (0.66), 3.505 (0.54), 5.715 (2.08), 7.181 (0.84), 7.184 (0.84), 7.671 (0.84), 7.674 (0.84).

### Intermediate 558

### cyclobutyl(1-{[2-(trimethylsilyl)ethoxy]methyl}-4H-1,2,4-triazol-5-yl)methanone

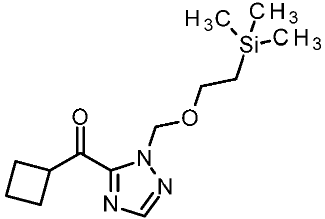

1-{[2-(Trimethylsilyl)ethoxy]methyl}-1H-1,2,4-triazole (750 mg, 3.76 mmol, Intermediate 548) was dissoved in THF (27 mL) and cooled to -78 °C. N-butyllithium (1.8 mL, 2.5 M in hexane, 4.5 mmol) was added at -78 °C and stirred for 1 h. A solution of cyclobutanecarbonyl chloride (470 µL, 4.1 mmol) in THF (10 mL) was added, the mixture was allowed to warm up and stirred over night at room temperature. The reaction mixture was washed with saturated sodium bicarbonate solution, water and brine, dried over a hydrophobic filter and concentrated. The residue was purified by column chromatography (silica gel, hexane / ethyl acetate (0 - 100 %) gradient) to give 435 mg (41 % yield) of the title compound.
LC-MS (Method 1): Rt = 1.43 min; MS (ESlpos): m/z = 283 [M+H]⁺
¹H-NMR (400 MHz, DMSO-d6) δ [ppm]: -0.071 (0.50), -0.063 (13.09), -0.054 (0.50), -0.040 (0.70), -0.032 (16.00), -0.024 (1.85), -0.020 (1.14), -0.016 (1.84), 0.780 (0.72), 0.801 (0.94), 0.822 (1.10), 0.842 (0.59), 0.862 (0.54), 0.885 (0.56), 1.874 (0.53), 1.895 (0.76), 1.918 (0.70), 1.922 (0.42), 1.939 (0.49), 1.945 (0.55), 2.096 (0.45), 2.099 (1.57), 2.101 (0.44), 2.110 (0.44), 2.118 (1.10), 2.120 (1.95), 2.124 (0.99), 2.129 (0.64), 2.132 (0.58), 2.135 (0.43), 2.139 (0.77), 2.142 (1.32), 2.144 (1.19), 2.150 (0.43), 2.165 (0.44), 2.195 (0.41), 2.205 (0.74), 2.215 (0.54), 2.227 (1.28), 2.237 (0.53), 2.240 (0.52), 2.248 (1.26), 2.261 (0.48), 2.265 (0.59), 2.269 (0.71), 3.042 (0.46), 3.061 (0.54), 3.064 (0.54), 3.353 (1.56), 3.365 (1.14), 3.390 (0.47), 3.467 (0.44), 3.487 (0.82), 3.508 (1.07), 3.530 (0.48), 3.588 (0.54), 3.608 (0.57), 3.628 (0.47), 5.499 (1.27), 5.812 (1.64), 7.902 (0.67), 7.938 (0.42), 8.002 (1.32), 8.008 (2.91), 8.237 (1.07).

### Intermediate 559

### (1-methylcyclopropyl)(1-{[2-(trimethylsilyl)ethoxy]methyl}-1H-imidazol-2-yl)methanone

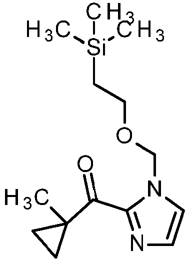

1-Methylcyclopropane-1-carboxylic acid (1.00 g, 9.99 mmol) was dissolved in dichloromethane (30 mL) and DMF (7 drops). Ethanedioyl dichloride (870 µL, 9.99 mmol) was added dropwise and the mixture was stirred for 2 h at room temperature. The reaction mixture was concentrated under reduce pressure (800 mbar) and used without further purification. 1-{[2-(Trimethylsilyl)ethoxy]methyl}-1H-imidazole (1.97 g, 9.95 mmol, Intermediate 545) was dissolved in THF (40 mL) and cooled to -75 °C. N-Butyllithium (4.2 mL, 2.5 M in hexane, 10 mmol) was added and stirred for 1 h at -75 °C. 1-Methylcyclopropane-1-carbonyl chloride (1.18 g, 9.95 mmol) was added dropwise at -75 °C and the mixture was stirred over the weekend at room temperature. The reaction mixture was diluted with a saturated aqueous sodium bicarbonate solution and extracted with ethyl acetate. The combined organic layers were washed with brine, dried over a hydrophobic filter and concentrated. The residue was purified by column chromatography (silica gel, dichloromethane / ethyl acetate gradient) to give 293 mg (10 % yield) of the title compound.

¹H-NMR (400 MHz, DMSO-d6) δ [ppm]: -0.071 (0.57), -0.063 (16.00), -0.054 (0.56), 0.795 (0.62), 0.815 (0.76), 0.835 (0.63), 0.912 (0.97), 0.919 (0.99), 1.404 (2.99), 1.790 (0.67), 1.798 (0.66), 3.437 (0.68), 3.457 (0.77), 3.477 (0.64), 5.625 (2.38), 7.081 (0.98), 7.083 (0.94), 7.567 (0.99), 7.570 (0.95).

### Intermediate 560

### (3,3-difluorocyclobutyl)(1-{[2-(trimethylsilyl)ethoxy]methyl}-1H-imidazol-2-yl)methanone

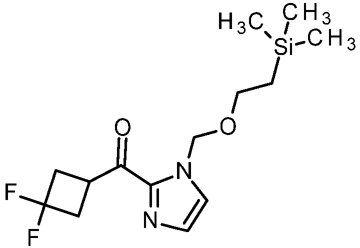

1-{[2-(Trimethylsilyl)ethoxy]methyl}-1H-imidazole (1.32 g, 6.68 mmol, Intermediate 545) was dissolved in THF (40 mL) and cooled to -78 °C. N-Butyllithium (4.6 mL, 1.6 M in hexane, 7.3 mmol) was added and the mixture was stirred for 20 min at -78 °C. 3,3-Difluorocyclobutane-1-carbonyl chloride (1.14 g, 7.34 mmol, Intermediate 544) was added dropwise at -78 °C and stirred for 1 h at -78 °C. The reaction mixture was allowed to warm up to room temperature, quenched with a saturated sodium bicarbonate solution and extracted with ethyl acetate. The combined organic layers were washed with brine, dried over a hydrophobic filter and concentrated. The residue was purified by column chromatography (silica gel, hexane / ethyl acetate gradient) to give 285 mg (13 % yield) of the title compound.

¹H-NMR (400 MHz, DMSO-d6) δ [ppm]: -0.062 (0.45), -0.054 (12.29), -0.045 (0.49), -0.037 (1.09), -0.026 (16.00), -0.023 (3.43), -0.018 (0.71), -0.007 (0.33), 0.011 (0.23), 0.709 (0.17), 0.724 (0.17), 0.735 (0.33), 0.751 (0.31), 0.761 (0.19), 0.777 (0.17), 0.831 (0.49), 0.851 (0.62), 0.871 (0.49), 2.823 (0.21), 2.842 (0.23), 2.853 (0.17), 2.858 (0.17), 2.882 (0.19), 3.156 (0.17), 3.171 (0.17), 3.196 (0.28), 3.222 (0.17), 3.237 (0.17), 3.515 (0.50), 3.535 (0.61), 3.555 (0.47), 5.015 (0.23), 5.041 (0.50), 5.072 (0.54), 5.098 (0.26), 5.339 (0.38), 5.740 (1.56), 6.240 (0.57), 6.904 (0.78), 6.907 (0.80), 7.234 (0.68), 7.237 (0.69), 7.264 (0.76), 7.267 (0.76), 7.752 (0.62), 7.754 (0.66).

### Intermediate 561

### cyclopentyl(1-{[2-(trimethylsilyl)ethoxy]methyl}-1H-imidazol-2-yl)methanone

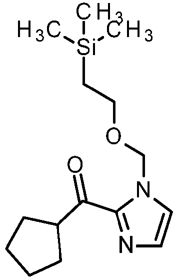

1-{[2-(Trimethylsilyl)ethoxy]methyl}-1H-imidazole (1.00 g, 5.04 mmol, Intermediate 545) was dissolved in THF (10 mL) and cooled to -70 °C. N-Butyllithium (2.2 mL, 2.5 M in hexane, 5.5 mmol) was added dropwise and stirred for 1 h at -70 °C. Cyclopentanecarbonyl chloride (670 µL, 5.5 mmol) was added dropwise and the reaction mixture was allowed to reach room temperature and stirred for 17.5 h at room temperature. The reaction mixture was diluted with saturated aqueous sodium bicarbonate solution and extracted with ethyl acetate. The organic phase was washed with brine, dried over a hydrophobic filter and concentrated. The crude product was purified by column chromatography (silica gel, dichloromethane / ethanol gradient) followed by a second column chromatography (silica gel, hexane / ethyl acetate gradient) to give a first batch of 177 mg (10 % yield) and a second batch of 65.0 mg (4 % yield) of the title compound.
LC-MS (Method 1): Rt = 1.49 min; MS (ESlpos): m/z = 295 [M+H]⁺
¹H-NMR (400 MHz, DMSO-d6) δ [ppm]: -0.150 (0.48), 0.019 (0.49), 0.027 (4.31), 0.033 (0.48), 0.059 (5.72), 0.146 (0.48), 0.876 (4.26), 0.883 (1.31), 0.895 (5.12), 0.902 (0.85), 0.907 (0.78), 0.916 (4.53), 0.921 (0.61), 0.925 (0.65), 0.932 (0.42), 0.939 (0.58), 0.951 (0.58), 1.094 (0.67), 1.123 (0.66), 1.173 (1.55), 1.321 (1.31), 1.348 (2.40), 1.595 (0.48), 1.604 (0.50), 1.612 (0.69), 1.627 (0.54), 1.632 (0.60), 1.637 (0.53), 1.642 (0.49), 1.659 (0.96), 1.669 (1.44), 1.678 (2.52), 1.686 (4.02), 1.690 (3.82), 1.698 (3.76), 1.701 (3.97), 1.708 (3.10), 1.713 (2.65), 1.715 (2.45), 1.727 (1.22), 1.735 (1.03), 1.741 (1.42), 1.748 (1.08), 1.758 (1.32), 1.762 (1.24), 1.771 (1.48), 1.777 (1.40), 1.790 (1.83), 1.795 (1.08), 1.805 (1.09), 1.809 (1.41), 1.864 (0.44), 1.883 (0.41), 1.907 (0.74), 1.916 (1.10), 1.920 (1.19), 1.928 (1.17), 1.938 (1.48), 1.942 (1.44), 1.946 (1.36), 1.951 (1.18), 1.952 (1.13), 1.958 (1.07), 1.964 (0.83), 1.968 (1.02), 1.980 (0.46), 1.984 (0.40), 2.607 (1.09), 2.612 (0.72), 3.083 (0.66), 3.541 (4.90), 3.548 (0.66), 3.561 (5.42), 3.574 (0.59), 3.581 (4.68), 4.066 (0.57), 4.085 (1.26), 4.089 (1.15), 4.106 (1.91), 4.124 (1.11), 4.129 (0.86), 4.146 (0.44), 5.019 (0.77), 5.741 (0.55), 5.797 (16.00), 7.251 (6.53), 7.254 (6.53), 7.271 (0.54), 7.274 (0.48), 7.579 (0.43), 7.582 (0.50), 7.737 (6.96), 7.739 (6.95).

### Intermediate 562

### (1-methylcyclobutyl)(4-{[2-(trimethylsilyl)ethoxy]methyl}-1H-1,2,4-triazol-5-yl)methanone

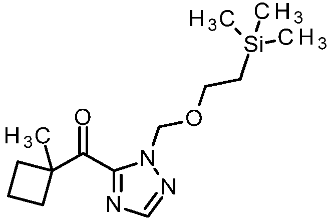

1-{[2-(Trimethylsilyl)ethoxy]methyl}-1H-1,2,4-triazole (596 mg, 2.99 mmol, Intermediate 548) was dissolved in THF (9 mL) and cooled to -78 °C. N-Butyllithium (1.4 mL, 2.5 M in hexane, 3.6 mmol) was added and stirred for 1 h at -78 °C. A solution of N-methoxy-N,1-dimethylcyclobutane-1-carboxamide (517 mg, 3.29 mmol, Intermediate 574) in THF (2 mL) was added dropwise at -78 °C and the reaction mixture was allowed to reach rt and stirred for 1 h at room temperature. The reaction mixture was diluted with saturated sodium bicarbonate solution and extracted three times with ethyl acetate. The combined organic phase was washed with water and brine, dried over a hydrophobic filter and concentrated. The crude product was purified by column chromatography (silica gel, hexane / ethyl acetate (0 - 10 %) gradient) to give 364 mg (95 % purity, 39 % yield) of the title compound
LC-MS (Method 1): Rt = 296.70 min; MS (ESlpos): m/z = 2 [M+H]⁺
¹H-NMR (400 MHz, DMSO-d6) δ [ppm]: -0.073 (0.60), -0.065 (16.00), -0.057 (0.54), 0.820 (0.59), 0.840 (0.83), 0.859 (0.60), 1.616 (3.27), 3.580 (0.64), 3.600 (0.84), 3.619 (0.63), 5.808 (2.35), 8.262 (1.49).

### SEM-Cleavage

### Intermediate 563

### cyclobutyl(1H-imidazol-2-yl)methanone

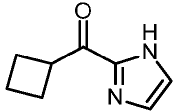

Cyclobutyl(1-{[2-(trimethylsilyl)ethoxy]methyl}-1H-imidazol-2-yl)methanone (292 mg, 1.04 mmol, Intermediate 553) was dissolved in dichloromethane (2 mL). Trifluoroacetic acid (1.0 ml, 13 mmol) was added and the mixture was stirred over night at room temperature. The reaction mixture was concentrated and diluted with dichloromethane. The organic phase was extracted with saturated sodium bicarbonate solution, water and brine, dried and concentrated. The residue was purified by column chromatography (silica gel, hexane / ethyl acetate (0 - 100 %) and ethyl acetate / ethanol (0-100 %) gradient) to give 64.0 mg (100 % purity, 41 % yield) of the title compound.
LC-MS (Method 1): Rt = 0.68 min; MS (ESlpos): m/z = 151 [M+H]⁺
¹H-NMR (400 MHz, DMSO-d6) δ [ppm]: -0.081 (0.40), -0.045 (1.29), 0.818 (1.01), 0.836 (2.29), 0.854 (1.28), 1.154 (0.55), 1.172 (1.02), 1.190 (0.56), 1.232 (1.92), 1.264 (0.91), 1.282 (0.57), 1.749 (0.66), 1.753 (0.66), 1.760 (1.34), 1.763 (1.26), 1.772 (1.77), 1.774 (1.90), 1.779 (1.32), 1.782 (2.81), 1.786 (3.13), 1.790 (2.03), 1.794 (2.31), 1.798 (2.74), 1.801 (2.44), 1.805 (2.28), 1.809 (3.57), 1.812 (3.49), 1.815 (1.40), 1.819 (1.89), 1.822 (2.73), 1.824 (1.94), 1.832 (2.13), 1.834 (2.04), 1.842 (1.07), 1.844 (1.08), 1.899 (0.53), 1.921 (0.45), 1.957 (1.12), 1.980 (3.48), 1.984 (1.16), 1.988 (2.04), 2.001 (6.91), 2.007 (2.75), 2.024 (4.87), 2.028 (5.10), 2.045 (2.82), 2.051 (3.62), 2.071 (1.75), 2.106 (0.49), 2.113 (0.49), 2.126 (0.87), 2.130 (1.72), 2.133 (1.99), 2.135 (1.69), 2.140 (1.94), 2.142 (1.88), 2.146 (2.13), 2.152 (2.92), 2.157 (4.20), 2.159 (4.57), 2.160 (5.80), 2.166 (4.62), 2.170 (5.38), 2.174 (3.77), 2.177 (7.11), 2.180 (8.95), 2.182 (10.36), 2.187 (7.28), 2.192 (7.08), 2.204 (13.14), 2.212 (3.77), 2.214 (3.80), 2.224 (10.99), 2.228 (7.92), 2.245 (3.82), 2.249 (3.95), 2.252 (3.53), 2.254 (3.39), 2.272 (0.77), 2.276 (1.59), 2.337 (0.85), 2.518 (10.58), 2.523 (7.73), 2.679 (0.84), 4.141 (1.41), 4.143 (1.37), 4.162 (5.11), 4.165 (5.40), 4.184 (7.29), 4.187 (7.32), 4.205 (5.00), 4.207 (4.95), 4.226 (1.25), 4.228 (1.10), 5.725 (0.61), 6.243 (0.41), 7.136 (11.68), 7.139 (16.00), 7.142 (11.37), 7.401 (11.09), 7.404 (11.61), 7.407 (11.77), 7.410 (10.67), 13.200 (1.33).

### Intermediate 564

### (1H-imidazol-2-yl)(1-methylcyclobutyl)methanone

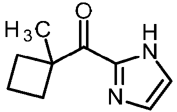

(1-Methylcyclobutyl)(1-{[2-(trimethylsilyl)ethoxy]methyl}-1H-imidazol-2-yl)methanone (83.0 mg, 282 µmol, Intermediate 556) was dissolved in dichloromethane (1.9 mL). Trifluoroacetic acid (720 µL, 9.3 mmol) was added and the mixture was stirred over night at room temperature. The reaction mixture was concentrated. The residue was diluted with dichloromethane and extracted with a saturated sodium bicarbonate solution and water. The organic phase was washed with brine, dried and concentrated. The crude product was purified by column chromatography (silica gel NH2, hexane / ethyl acetate (0 - 100 %) and ethyl acetate / ethanol (0 - 100 %) gradient) to give 34.0 mg (100 % purity, 73 % yield) of the title compound.
LC-MS (Method 1): Rt = 0.79 min; MS (ESlneg): m/z = 163 [M-H]⁻
¹H-NMR (400 MHz, DMSO-d6) δ [ppm]: 0.851 (0.19), 1.178 (1.79), 1.234 (1.13), 1.259 (0.18), 1.334 (0.42), 1.339 (0.54), 1.375 (0.29), 1.601 (16.00), 1.637 (0.19), 1.645 (0.27), 1.655 (0.25), 1.658 (0.40), 1.661 (0.31), 1.668 (0.95), 1.672 (0.35), 1.678 (0.49), 1.681 (0.51), 1.684 (0.45), 1.691 (0.56), 1.694 (0.82), 1.701 (0.34), 1.705 (0.53), 1.708 (0.31), 1.719 (0.36), 1.729 (0.28), 1.888 (0.72), 1.898 (0.58), 1.900 (0.45), 1.907 (0.48), 1.912 (1.05), 1.917 (1.15), 1.921 (1.43), 1.926 (0.82), 1.931 (0.37), 1.937 (0.77), 1.942 (0.96), 1.946 (0.99), 1.949 (0.87), 1.970 (0.43), 1.988 (0.66), 1.993 (0.35), 2.008 (0.68), 2.010 (0.63), 2.014 (0.70), 2.019 (0.21), 2.027 (0.30), 2.031 (0.52), 2.036 (0.70), 2.052 (0.21), 2.055 (0.29), 2.058 (0.43), 2.318 (0.16), 2.323 (0.34), 2.327 (0.49), 2.331 (0.37), 2.336 (0.17), 2.350 (0.35), 2.518 (2.99), 2.523 (1.62), 2.561 (0.45), 2.660 (0.16), 2.665 (0.35), 2.669 (0.49), 2.673 (0.35), 2.678 (0.16), 3.052 (0.91), 3.492 (0.50), 3.613 (0.82), 5.759 (0.23), 7.151 (2.24), 7.154 (3.08), 7.157 (2.22), 7.353 (2.08), 7.355 (2.23), 7.358 (2.24), 7.361 (2.13), 13.122 (0.27).

### Intermediate 565

### (3,3-difluorocyclobutyl)(1H-1,2,4-triazol-5-yl)methanone

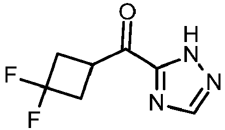

(3,3-Difluorocyclobutyl)(1-{[2-(trimethylsilyl)ethoxy]methyl}-1H-1,2,4-triazol-5-yl)methanone (285 mg, 898 µmol, Intermediate 555) was provided in dichloromethane (20 mL). Trifluoroacetic acid (460 µL, 6.0 mmol) was added and the mixture was stirred for 2 h at room temperature. The reaction mixture was concentrated. The residue was purified by column chromatography (silica gel, dichloromethane / ethanol gradient) to give 136 mg (81 % yield) of the title compound.

¹H-NMR (400 MHz, DMSO-d6) δ [ppm]: 1.035 (1.11), 1.052 (2.49), 1.070 (1.00), 2.331 (1.42), 2.337 (0.62), 2.518 (7.05), 2.523 (4.67), 2.673 (1.49), 2.678 (0.66), 2.819 (4.32), 2.844 (12.34), 2.857 (8.47), 2.865 (10.37), 2.882 (15.17), 2.903 (11.44), 3.929 (1.56), 3.937 (2.21), 3.949 (2.94), 3.959 (3.18), 3.970 (2.94), 3.980 (2.04), 3.991 (1.62), 5.546 (8.22), 5.565 (8.12), 5.759 (0.59), 7.256 (1.90), 7.275 (4.08), 7.294 (1.80), 8.203 (0.93), 8.783 (16.00), 8.812 (13.41), 14.735 (2.04).

### Intermediate 566

### 4-chloro-2-propanoyl-1H-imidazol-1-ium chloride

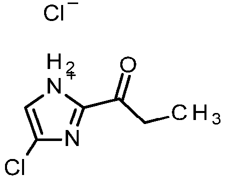

1-(4-Chloro-1-{[2-(trimethylsilyl)ethoxy]methyl}-1H-imidazol-2-yl)propan-1-one (85.0 mg, 294 µmol, **Intermediate 576**) was dissolved in THF (6.8 mL) and methanol (6.8 mL). Hydrochloric acid in 1,4-dioxane (3.4 mL, 4.0 M, 14 mmol) was added and the mixture was stirred over night at room temperature and 2 h at 50 °C. The reaction mixture was allowed to cool down, concentrated to give 63.8 mg (75 % purity, 83 % yield) of the title compound which was used without further purification in the next step.

¹H-NMR (400 MHz, DMSO-d6) δ [ppm]: 1.046 (7.04), 1.064 (16.00), 1.083 (7.25), 1.347 (0.92), 1.496 (0.41), 1.513 (0.47), 1.533 (0.47), 1.723 (0.47), 1.743 (0.55), 1.760 (0.47), 2.518 (1.10), 2.523 (0.74), 2.923 (2.09), 2.941 (7.06), 2.959 (6.87), 2.977 (1.92), 3.201 (0.41), 3.336 (0.82), 3.620 (1.03), 3.636 (2.09), 3.654 (1.00), 7.550 (8.23).

### Intermediate 567

### cyclohexyl(1H-imidazol-2-yl)methanone

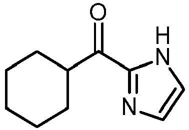

Cyclohexyl(1-{[2-(trimethylsilyl)ethoxy]methyl}-1H-imidazol-2-yl)methanone (540 mg, 1.75 mmol, Intermediate 557) was dissolved in 1,4-dioxane. Hydrochloric acid in 1,4-dioxane (2.2 mL, 4.0 M, 8.8 mmol) was added and the mixture was stirred for 3 days at room temperature. The reaction mixture was concentrated and diluted with dichloromethane. The organic phase was washed with a saturated sodium bicarbonate solution, water and brine, dried and concentrated. The crude product was purified by column chromatography (silica gel, hexane / ethyl acetate (0 - 100 %) and ethyl acetate / ethanol (0 - 100 %) gradient) to give 96.0 mg (90 % purity, 28 % yield) of the title compound.
LC-MS (Method 1): Rt = 0.88 min; MS (ESlpos): m/z = 179 [M+H]⁺
1H-NMR (400 MHz, DMSO-d6) δ [ppm]: -0.078 (1.88), -0.055 (1.01), -0.045 (4.91), -0.040 (1.21), -0.038 (1.08), -0.029 (16.00), -0.021 (0.67), -0.010 (0.47), 0.015 (0.67), 0.032 (0.67), 0.039 (0.40), 0.864 (0.47), 1.167 (1.48), 1.178 (1.48), 1.185 (2.89), 1.195 (2.02), 1.203 (2.35), 1.209 (2.22), 1.218 (2.69), 1.226 (2.08), 1.245 (2.69), 1.293 (1.55), 1.299 (1.88), 1.306 (1.82), 1.332 (9.01), 1.344 (5.31), 1.355 (15.46), 1.384 (8.47), 1.416 (1.68), 1.669 (3.36), 1.697 (2.76), 1.701 (2.96), 1.752 (4.71), 1.760 (5.92), 1.766 (5.38), 1.773 (6.12), 1.780 (4.77), 1.838 (6.52), 1.857 (5.18), 2.000 (2.42), 2.349 (1.21), 2.690 (1.21), 3.464 (0.87), 3.472 (1.61), 3.481 (1.61), 3.493 (2.55), 3.501 (3.23), 3.509 (2.22), 3.520 (1.68), 3.529 (1.48), 4.030 (0.54), 4.047 (0.54), 5.202 (0.47), 5.228 (0.54), 5.699 (1.55), 6.813 (0.47), 6.866 (0.94), 6.869 (0.94), 7.171 (6.79), 7.206 (1.01), 7.210 (1.01), 7.419 (6.18), 13.179 (1.61).

### Intermediate 568

### cyclobutyl(4H-1,2,4-triazol-3-yl)methanone

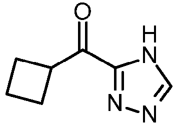

Cyclobutyl(1-{[2-(trimethylsilyl)ethoxy]methyl}-4H-1,2,4-triazol-5-yl)methanone (435 mg, 1.55 mmol, Intermediate 558) was dissolved in dichloromethane (3.3 mL). Trifluoroacetic acid (1.5 mL, 19 mmol) was added and the mixture was stirred over night at room temperature. The reaction mixture was concentrated and diluted with dichloromethane. The organic phase was washed with saturated sodium bicarbonate, water and brine, dried and concentrated. The crude product was purified by column chromatography (silica gel, hexane / ethyl acetate (0 - 100 %) and ethyl acetate / ethanol (0 - 100 %) gradient) to give 40.7 mg (93 % purity, 16 % yield) of the title compound.

¹H-NMR (400 MHz, DMSO-d6) δ [ppm]: 0.855 (1.00), 0.873 (1.94), 0.878 (0.80), 0.891 (0.87), 1.154 (2.54), 1.173 (5.22), 1.190 (2.88), 1.232 (1.27), 1.282 (0.47), 1.352 (0.47), 1.784 (0.67), 1.799 (0.80), 1.816 (0.94), 1.825 (1.00), 1.827 (1.00), 1.840 (1.34), 1.843 (1.27), 1.847 (1.61), 1.850 (1.47), 1.852 (1.61), 1.855 (1.67), 1.858 (1.67), 1.863 (1.87), 1.874 (2.34), 1.877 (2.41), 1.886 (1.74), 1.888 (1.81), 1.896 (1.54), 1.900 (1.54), 1.908 (0.94), 1.912 (1.14), 1.916 (1.34), 1.938 (3.01), 1.943 (0.80), 1.960 (5.96), 1.966 (1.87), 1.982 (3.95), 1.988 (13.12), 2.003 (3.35), 2.009 (3.01), 2.022 (5.49), 2.030 (2.48), 2.042 (7.70), 2.061 (5.69), 2.081 (1.94), 2.088 (1.61), 2.097 (0.60), 2.105 (2.21), 2.109 (1.94), 2.126 (2.48), 2.130 (1.54), 2.146 (1.34), 2.164 (0.94), 2.183 (1.27), 2.192 (1.34), 2.205 (2.08), 2.215 (3.28), 2.218 (3.88), 2.222 (3.68), 2.231 (5.89), 2.237 (6.03), 2.240 (5.62), 2.252 (9.24), 2.255 (9.04), 2.261 (5.09), 2.276 (8.97), 2.279 (5.22), 2.296 (2.54), 2.300 (2.21), 2.306 (1.87), 2.318 (1.47), 2.323 (3.15), 2.327 (4.55), 2.332 (3.01), 2.336 (1.34), 2.518 (16.00), 2.523 (11.18), 2.665 (5.49), 2.669 (6.96), 2.673 (5.76), 2.687 (6.69), 2.704 (2.81), 2.711 (2.61), 3.049 (2.95), 3.053 (3.01), 3.057 (2.74), 3.069 (5.36), 3.072 (5.22), 3.088 (2.81), 3.092 (2.61), 3.164 (0.47), 3.185 (0.67), 3.209 (0.40), 3.362 (3.48), 3.385 (1.81), 3.405 (0.60), 4.000 (0.74), 4.017 (2.28), 4.035 (2.21), 4.053 (0.67), 4.128 (0.47), 4.241 (0.40), 5.388 (2.41), 5.406 (2.54), 5.759 (10.64), 6.186 (3.88), 6.960 (0.40), 6.978 (0.80), 6.998 (0.47), 8.303 (0.87), 8.518 (8.10), 8.524 (0.74), 8.752 (0.60), 8.915 (2.68), 13.935 (0.87).

### Intermediate 569

### (1H-imidazol-2-yl)(1-methylcyclopropyl)methanone

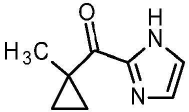

(1-Methylcyclopropyl)(1-{[2-(trimethylsilyl)ethoxy]methyl}-1H-imidazol-2-yl)methanone (293 mg, 1.04 mmol, Intermediate 559) was dissolved in dichloromethane (20 mL). Trifluoroacetic acid (1.1 mL, 14 mmol) was added and the mixture was stirred for 50 h at room temperature. Trifluoroacetic acid (1.1 mL, 14 mmol) was added and stirred for 23 h at 50 °C. The reaction mixture was concentrated and the residue was diluted with ethyl acetate. The organic phase was washed with saturated aqueous sodium bicarbonate solution and brine, dried over a hydrophobic filter and concentrated to give 162 mg (98 % yield) of the title compound which was used without further purification in the next step.
LC-MS (Method 1): Rt = 0.71 min; MS (ESlpos): m/z = 151 [M+H]⁺
¹H-NMR (400 MHz, DMSO-d6) δ [ppm]: -0.034 (0.59), 0.944 (1.22), 0.946 (1.35), 0.953 (4.93), 0.961 (5.16), 0.968 (1.33), 0.971 (1.25), 0.985 (0.31), 1.237 (0.16), 1.246 (0.16), 1.280 (0.33), 1.397 (16.00), 1.954 (1.17), 1.962 (3.65), 1.970 (3.57), 1.978 (1.09), 5.280 (0.17), 7.211 (0.21).

### Intermediate 570

### (3,3-difluorocyclobutyl)(1H-imidazol-2-yl)methanone

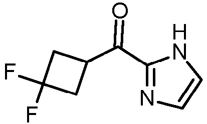

(3,3-Difluorocyclobutyl)(1-{[2-(trimethylsilyl)ethoxy]methyl}-1H-imidazol-2-yl)methanone (892 mg, 2.82 mmol, Intermediate 560) was dissolved in dichloromethane (30 mL). Trifluoroacetic acid (0.8 mL, 10 mmol) was added and the mixture was stirred over night at room temperature. Trifluoroacetic acid (2 mL, 26 mmol) was added and the mixture was stirred for 72 h at room temperature. The reaction mixture was concentrated to give a first batch of crude product. (3,3-Difluorocyclobutyl)(1-{[2-(trimethylsilyl)ethoxy]methyl}-1H-imidazol-2-yl)methanone (218 mg, 0.69 mmol, Intermediate 560) was dissolved in dichloromethane (20 mL). Trifluoroacetic acid (0.5 mL, 6 mmol) was added and the mixture was stirred over night at room temperature. Trifluoroacetic acid (2 mL, 26 mmol) was added and the mixture was stirred for 72 h at room temperature. The reaction mixture was concentrated to give a second batch of crude product. The two batches were purified together by column chromatography (silica gel, dichloromethane / ethyl acetate gradient) to give 372 mg (57 % yield) of the title compound.
LC-MS (Method 1): Rt = 0.69 min; MS (ESlpos): m/z = 187 [M+H]⁺
¹H-NMR (400 MHz, DMSO-d6) δ [ppm]: 1.154 (0.52), 1.172 (1.04), 1.190 (0.57), 1.232 (0.57), 1.907 (0.65), 1.987 (2.11), 2.331 (1.09), 2.518 (7.40), 2.523 (4.42), 2.673 (1.17), 2.794 (0.65), 2.809 (3.48), 2.834 (10.01), 2.848 (6.26), 2.855 (8.10), 2.872 (12.60), 2.894 (9.57), 3.967 (0.67), 3.973 (0.72), 3.988 (2.06), 3.995 (2.06), 4.010 (2.83), 4.017 (3.20), 4.031 (1.96), 4.039 (1.96), 4.053 (0.82), 4.060 (0.67), 4.592 (0.87), 4.673 (0.47), 5.682 (1.47), 7.179 (0.57), 7.181 (0.55), 7.384 (16.00), 7.650 (0.57), 7.653 (0.60).

### Intermediate 571

### cyclopentyl(1H-imidazol-2-yl)methanone

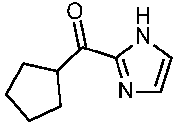

Cyclopentyl(1-{[2-(trimethylsilyl)ethoxy]methyl}-1H-imidazol-2-yl)methanone (242 mg, 678 µmol, Intermediate 561) was dissolved in dichloromethane (7.1 mL). Trifluoroacetic acid (710 µL, 9.3 mmol) was added and the mixture was stirred for 22 h at room temperature.

The reaction mixture was concentrated. The residue was dissolved In ethyl acetate and washed with saturated aqueous sodium bicarbonate solution and brine. The organic phase was dried over a hydrophobic filter and concentrated. The crude product was purified by column chromatography (silica gel, hexane / ethyl acetate gradient) to give 54.8 mg (48 % yield) of the title compound.
LC-MS (Method 1): Rt = 0.80 min; MS (ESlpos): m/z = 165 [M+H]⁺
¹H-NMR (400 MHz, DMSO-d6) δ [ppm]: 1.230 (0.84), 1.258 (0.67), 1.559 (1.01), 1.565 (1.18), 1.576 (2.36), 1.579 (2.86), 1.590 (5.05), 1.598 (7.07), 1.607 (11.45), 1.611 (14.15), 1.614 (13.98), 1.627 (15.33), 1.637 (7.92), 1.642 (8.93), 1.661 (4.88), 1.673 (4.55), 1.691 (4.38), 1.694 (4.21), 1.703 (5.56), 1.710 (6.06), 1.722 (7.75), 1.728 (5.39), 1.734 (4.21), 1.741 (5.89), 1.757 (1.85), 1.842 (2.53), 1.852 (4.55), 1.856 (5.22), 1.864 (5.22), 1.870 (6.06), 1.873 (6.91), 1.878 (6.57), 1.886 (5.05), 1.888 (5.05), 1.893 (5.05), 1.904 (4.55), 1.916 (1.85), 1.920 (1.52), 2.518 (4.88), 2.523 (3.20), 3.399 (1.01), 3.863 (2.53), 3.882 (5.73), 3.885 (5.39), 3.893 (0.84), 3.903 (8.76), 3.921 (5.05), 3.924 (4.21), 3.942 (2.02), 7.163 (16.00), 7.408 (14.99), 13.179 (1.85).

### Intermediate 572

### (1-methylcyclobutyl)(4H-1,2,4-triazol-3-yl)methanone

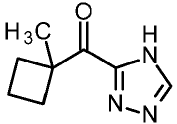

(1-Methylcyclobutyl)(1-{[2-(trimethylsilyl)ethoxy]methyl}-4H-1,2,4-triazol-5-yl)methanone (364 mg, 1.23 mmol, Intermediate 562) was dissoved in dichloromethane (8.4 mL). Trifluoroacetic acid (3.1 mL, 41 mmol) was added and the mixture was stirred over night at room temperature. The reaction mixture was concentrated and the residue was dissolved in dichloromethane. The organic phase was washed with saturated sodium bicarbonate solution, water and brine, dried and concentrated. The crude product was purified by column chromatography (silica gel, hexane / ethyl acetate (0 - 100 %) and ethyl acetate / ethanol (0 - 100 %) gradient) to give 67.0 mg (100 % purity, 33 % yield) of the title compound.
LC-MS (Method 2): Rt = 0.75 min; MS (ESlneg): m/z = 164 [M-H]⁻
¹H-NMR (400 MHz, DMSO-d6) δ [ppm]: 1.640 (16.00), 1.740 (0.86), 1.750 (0.45), 1.753 (0.53), 1.757 (0.41), 1.764 (0.52), 1.767 (0.83), 1.778 (0.47), 1.958 (0.74), 1.967 (0.57), 1.970 (0.45), 1.976 (0.43), 1.981 (1.02), 1.987 (1.06), 1.991 (1.43), 1.996 (0.91), 2.003 (0.57), 2.006 (0.70), 2.012 (0.96), 2.016 (0.88), 2.020 (0.81), 2.070 (0.66), 2.092 (0.70), 2.097 (0.70), 2.115 (0.53), 2.120 (0.74), 2.141 (0.43), 2.586 (2.79), 2.591 (1.95), 2.593 (1.78), 2.616 (0.49), 8.617 (0.57).

### Intermediate 573

### 1-(1H-pyrazol-3-yl)propan-1-one

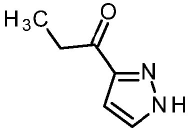

1-[1-(Oxan-2-yl)-1H-pyrazol-3-yl]propan-1-one (314 mg, 75 % purity, 1.13 mmol, **Intermediate 577**) was dissolved in methanol. Hydrochloric acid (840 µL, 36 % purity, 11 mmol) was added dropwise and the mixture was stirred for 3 h at room temperature. The reaction mixture was concentrated. The residue was diluted with a saturated sodium bicarbonate solution and extracted with ethyl acetate. The combined organic layers were dried over a hydrophobic filter and concentrated. The crude product was purified by column chromatography (silica gel, hexane / ethyl acetate (0 - 100 %) gradient) to give 58.0 mg (41 % yield) of the title compound.
¹H NMR (400 MHz, CHLOROFORM-d) δ ppm 1.23 (t, 3 H), 2.99 (q, 2 H), 6.84 (d, H), 7.64 (d, 1 H), 10.94 (br d, 1 H).

### Weinreb Amide

### Intermediate 574

### N-methoxy-N,1-dimethylcyclobutane-1-carboxamide

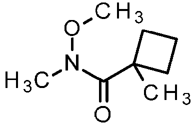

1-Methylcyclobutane-1-carboxylic acid (490 mg, 4.29 mmol) and N-methoxymethanamine hydrogen chloride (1/1) (461 mg, 4.72 mmol) were dissolved in DMF (4.5 mL). Triethylamine (1.8 mL, 13 mmol) and HATU (1.96 g, 5.15 mmol) were added and the mixture was stirred over night at room temperature. The reaction mixture was diluted with ethyl acetate and extracted with saturated sodium bicarbonate solution and water. The organic phase was washed with brine, dried and concentrated. The crude product was purified by column chromatography (silica gel, hexane / ethyl acetate (0 - 100 %) and ethyl acetate / ethanol (0 - 95 %) gradient) to give 520 mg (82 % purity, 64 % yield) of the title compound.
LC-MS (Method 1): Rt = 0.83 min; MS (ESlpos): m/z = 158 [M+H]⁺
¹H-NMR (400 MHz, DMSO-d6) δ [ppm]: 1.155 (0.19), 1.173 (0.41), 1.190 (0.20), 1.339 (7.87), 1.572 (0.19), 1.589 (0.19), 1.596 (0.45), 1.600 (0.23), 1.603 (0.25), 1.607 (0.19), 1.616 (0.21), 1.619 (0.28), 1.623 (0.49), 1.627 (0.19), 1.631 (0.28), 1.653 (0.18), 1.678 (0.50), 1.684 (0.45), 1.691 (0.25), 1.695 (0.21), 1.700 (0.53), 1.702 (0.47), 1.707 (0.78), 1.713 (0.50), 1.716 (0.30), 1.723 (0.44), 1.730 (0.70), 1.733 (0.28), 1.737 (0.46), 1.890 (0.34), 1.894 (0.16), 1.912 (0.45), 1.914 (0.37), 1.917 (0.30), 1.934 (0.32), 1.940 (0.43), 1.941 (0.25), 1.962 (0.24), 1.987 (0.61), 2.311 (0.35), 2.317 (0.23), 2.335 (0.87), 2.341 (0.66), 2.358 (0.65), 2.364 (0.80), 2.382 (0.18), 2.389 (0.25), 2.687 (0.35), 2.728 (0.59), 2.730 (0.67), 2.889 (0.75), 3.052 (16.00), 3.328 (1.82).

### Intermediate 575

### N-methoxy-N-methyl-1H-pyrazole-3-carboxamide

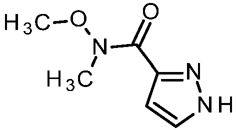

1H-Pyrazole-3-carboxylic acid (500 mg, 4.46 mmol) and N-methoxymethanamine (300 mg, 4.91 mmol) were dissoved DMF (7.5 mL). Triethylamine (1.9 mL, 13 mmol) and HATU (2.04 g, 5.35 mmol) were added and stirred for 2 h at room temperature. The reaction mixture was diluted with ethyl acetate. The organic phase was washed with sodium bicarbonate solution, water and brine, dried over a hydrophobic filter and concentrated. The crude product was purified by column chromatography (silica gel). The residue was dissoved in ethyl acetate and washed with ammonium chloride solution, water and brine. The organic phase was dried over a hydrophobic filter and concentrated to give 194 mg (28 % yield) of the title compound.
LC-MS (Method 1): Rt = 0.53 min; MS (ESlpos): m/z = 156 [M+H]⁺
¹H-NMR (400 MHz, DMSO-d6) δ [ppm]: 1.153 (4.50), 1.171 (10.10), 1.189 (4.65), 2.518 (2.70), 2.523 (1.77), 2.687 (16.00), 2.728 (2.42), 2.888 (3.05), 3.065 (0.40), 3.084 (1.24), 3.095 (1.13), 3.102 (1.31), 3.112 (1.06), 3.119 (0.52), 3.276 (1.06), 3.355 (0.52), 3.697 (1.29), 3.725 (1.16).

### Intermediate 576

### 1-(4-chloro-1-{[2-(trimethylsilyl)ethoxy]methyl}-1H-imidazol-2-yl)propan-1-one

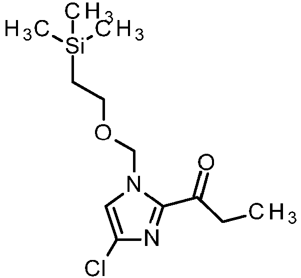

1-(1-{[2-(Trimethylsilyl)ethoxy]methyl}-1H-imidazol-2-yl)propan-1-one (150 mg, 590 µmol, **Intermediate 547**) was dissolved in acetonitrile (1.9 mL). N-Chlorsuccinimid (78.7 mg, 590 µmol) was added and the mixture was stirred for 1 h at room temperature and for 4 h at 60 °C. The reaction mixture was allowed to cool down, diluted with a saturated sodium carbonate solution and extracted with ethyl acetate. The combined organic layers were dried over sodium sulfate, filtered and concentrated. The residue was purified by column chromatography (silica gel, hexane / ethyl acetate (0 - 25 %) gradient) to give 87.8 mg (52 % yield) of the title compound as a mixture of isomers of 4:1.
LC-MS (Method 1): Rt = 1.47 min; MS (ESlpos): m/z = 289 [M+H]⁺
¹H-NMR (400 MHz, CHLOROFORM-d) δ [ppm]: -0.037 (0.53), -0.028 (16.00), -0.019 (0.94), -0.008 (2.28), 0.008 (2.28), 0.012 (0.53), 0.894 (0.72), 0.912 (0.47), 0.914 (0.63), 0.917 (0.53), 0.926 (2.85), 0.935 (0.91), 0.945 (1.67), 0.947 (2.15), 0.951 (1.56), 0.968 (2.87), 1.163 (5.38), 1.179 (1.95), 1.182 (11.92), 1.197 (3.24), 1.200 (5.63), 1.215 (1.39), 1.567 (7.15), 3.095 (1.67), 3.102 (0.46), 3.113 (5.53), 3.121 (1.36), 3.132 (5.40), 3.139 (1.36), 3.150 (1.56), 3.158 (0.43), 3.565 (3.33), 3.574 (0.90), 3.583 (1.84), 3.586 (2.31), 3.589 (1.96), 3.591 (0.70), 3.595 (0.74), 3.596 (0.58), 3.600 (0.48), 3.607 (3.23), 3.615 (0.85), 5.755 (12.09), 5.851 (2.86), 7.107 (1.53), 7.210 (6.08).

### Intermediate 577

### 1-[1-(oxan-2-yl)-1H-pyrazol-3-yl]propan-1-one

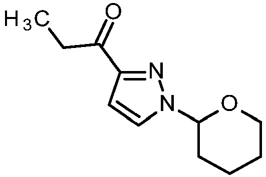

N-Methoxy-N-methyl-1-(oxan-2-yl)-1H-pyrazole-3-carboxamide (400 mg, 78 % purity, 1.30 mmol, **Intermediate 578**) was dissolved in dry THF (4.4 mL) and cooled to 0 °C. Ethyl magnesium bromide (530 µL, 3.2 M in THF, 1.7 mmol) was added slowly and the mixture was stirred for 1 h at 0 °C. The reaction mixture was diluted with saturated ammonium chloride solution and extracted with ethyl acetate. The combined organic layers were concentrated to give 132 mg (48 % yield) of the title compound.
LC-MS (Method 1): Rₜ = 1.00 min; MS (ESlpos): m/z = 209 [M+H]⁺
¹H-NMR (400 MHz, DMSO-d6) δ [ppm]: 1.045 (7.25), 1.063 (16.00), 1.081 (7.54), 1.165 (0.49), 1.232 (0.49), 1.352 (1.03), 1.434 (0.96), 1.519 (0.75), 1.529 (1.15), 1.542 (1.71), 1.551 (2.27), 1.560 (1.76), 1.572 (1.25), 1.582 (0.78), 1.605 (0.45), 1.637 (0.55), 1.660 (0.78), 1.666 (0.77), 1.677 (0.81), 1.683 (0.82), 1.689 (0.90), 1.697 (0.68), 1.707 (0.74), 1.713 (0.75), 1.729 (0.52), 1.738 (0.44), 1.917 (0.92), 1.926 (1.45), 1.931 (1.06), 1.950 (1.67), 1.957 (1.23), 1.967 (0.62), 2.053 (0.42), 2.062 (0.62), 2.078 (0.62), 2.083 (0.67), 2.087 (0.83), 2.092 (0.78), 2.109 (0.72), 2.114 (0.69), 2.116 (0.76), 2.139 (0.44), 2.518 (1.59), 2.523 (1.13), 2.687 (13.23), 2.926 (2.20), 2.945 (7.05), 2.963 (6.34), 2.981 (1.73), 3.625 (0.88), 3.639 (0.66), 3.643 (0.62), 3.653 (1.15), 3.659 (0.94), 3.667 (0.92), 3.674 (1.00), 3.688 (1.09), 3.700 (2.56), 3.925 (0.92), 3.953 (0.70), 3.956 (0.70), 5.492 (1.34), 5.498 (1.56), 5.518 (1.51), 5.523 (1.30), 6.732 (5.30), 6.739 (5.38), 8.011 (5.24), 8.017 (5.23).

### Intermediate 578

### N-methoxy-N-methyl-1-(oxan-2-yl)-1H-pyrazole-3-carboxamide

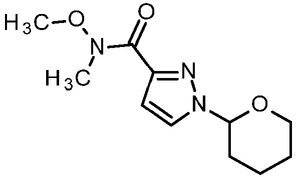

N-Methoxy-N-methyl-1H-pyrazole-3-carboxamide (6.17 g, 67 % purity, 26.6 mmol) was dissoved in 3,4-dihydro-2H-pyran (15 mL, 160 mmol). Trifluoro acetic acid (100 µL, 1.3 mmol) was added and the mixture was stirred for 5 h at 95 °C. The reaction mixture was allowed to cool down and diluted with THF. Sodium bicarbonate solution and ethyl acetate were added the organic phase was washed twice with sodium bicarbonate solution, water and brine, dried over a hydrophobic filter and concentrated. The crude product was purified by column chromatography (silica gel, dichloromethane / methanol (0 - 7 %) gradient) followed by a second column chromatography (silica gel, hexane / ethyl acetate (0 - 100 %) gradient) to give 1.76 g (28 % yield) of the title compound.
LC-MS (Method 1): Rt = 0.80 min; MS (ESlpos): m/z = 240 [M+H]⁺
¹H-NMR (400 MHz, DMSO-d6) δ [ppm]: 1.173 (0.66), 1.532 (0.49), 1.542 (0.68), 1.551 (0.60), 1.560 (0.52), 1.921 (0.49), 1.945 (0.53), 1.988 (1.15), 2.518 (0.48), 2.687 (16.00), 3.700 (13.11), 3.720 (0.78), 5.457 (0.45), 5.463 (0.53), 5.482 (0.52), 5.487 (0.44), 6.660 (1.81), 6.667 (1.76), 7.957 (1.69), 7.964 (1.73).

### Intermediate 579

### di-tert-butyl [5-bromo-3-(trifluoromethyl)pyrazin-2-yl]-2-imidodicarbonate

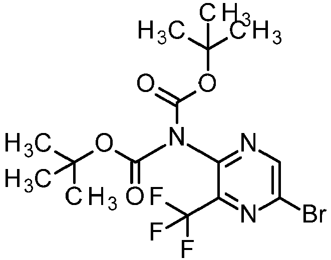

5-Bromo-3-(trifluoromethyl)pyrazin-2-amine (5.00 g, 20.7 mmol) and di-tert-butyl dicarbonate (12 mL, 52 mmol) were dissolved in dichloromethane (150 mL). Triethylamine (2.9 mL, 21 mmol) and 4-dimethylaminopyridine (50.5 mg, 413 µmol) were added and the mixture was stirred 4 h at room temperature. The reaction mixture was concentrated. The residue was diluted with hexane. The precipitate was filtered off, washed and dried to give a first batch with 4.9 g (100 % purity, 54 % yield) of the title compound.

The filtrate was purified by column chromatography (silica gel, cyclohexane / ethyl acetate (0 - 20 %) gradient) to give a second batch with 4.23 g (90 % purity, 42 % yield) of the title compound.
LC-MS (Method 5): Rt = 2.42 min; MS (ESlpos): m/z = 464 [M+Na]+⁺
¹H-NMR (400 MHz, DMSO-d6) δ [ppm]: 1.346 (16.00), 9.293 (0.80)

### Intermediate 580

### cyclopropyl(1H-1,2,3-triazol-5-yl)methanone

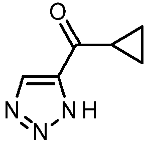

Sodium azide (212 mg, 3.27 mmol) was suspended in DMF (5 mL) and heated to 60 °C. A solution of 1-cyclopropylprop-2-yn-1-one (500 mg, 41 % purity, 2.18 mmol) in DMF (1.5 mL) was added dropwise and stirred for 1 h at 60 °C. The reaction mixture was allowed to cool down, diluted with water and extracted three times with dichloromethane. The aqueous phase was acidified with 2 N hydrochloric acid and extracted twice with ethyl acetate. The organic phase was washed with brine, dried over a hydrophobic filter and concentrated to give 289 mg (96 % yield) of the title compound.
LC-MS (Method 6): Rt = 0.75 min; MS (ESlneg): m/z = 136 [M-H]⁻
¹H NMR (DMSO-d₆, 400 MHz): δ (ppm) 15.75 (br s, 1H), 8.54 (br s, 1H), 2.99 (br s, 1H), 1.03 - 1.10 (m, 4H).

### Intermediate 581

### 1-cyclopropylprop-2-yn-1-one

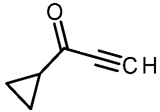

Cyclopropanecarbonyl chloride (1.00 g, 9.57 mmol) was dissolved in dichloromethane (7.2 mL). Ethynyl(trimethyl)silane (1.03 g, 10.5 mmol) was added, cooled to 0 °C and aluminum trichloride (3.83 g, 28.7 mmol) was added and stirred for 30 min at 0 °C and for 30 min at room temperature. The reaction mixture was diluted with 2 N hydrochloric acid and extracted with dichloromethane. The organic phase was washed with saturated sodium bicarbonate solution and brine, dried over a hydrophobic filter and concentrated to give 2.17 g (241 % yield) of the title compound which was used without further purification in the next step.

### Intermediate 582

### 5-bromo-3-[(1R)-1-phenylethoxy]pyrazin-2-amine

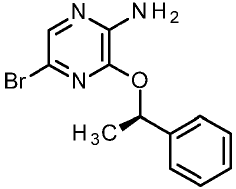

Sodium hydride (23.7 mg, 60 % purity, 593 µmol) was dissolved in THF (1 mL) and cooled to 0 °C. (1R)-1-Phenylethan-1-ol (72.5 mg, 593 µmol) was added slowly and stirred for 30 min at 0 °C. A solution of 3,5-dibromopyrazin-2-amine (100 mg, 395 µmol) in THF was added and the mixture was stirred over night at reflux. The reaction mixture was allowed to cool down, diluted with water and extracted with ethyl acetate. The combined organic layers were washed with brine, dried over sodium sulfate, filtered and concentrated. The crude product was purified by coloumn chromatography (silica gel, hexane / ethyl acetate gradient) followed by preparative HPLC to give 54.0 mg (46 % yield) of the title compound.
LC-MS (Method 1): Rt = 1.26 min; MS (ESlpos): m/z = 294 [M+H]⁺
¹H-NMR (500 MHz, DMSO-d6) δ [ppm]: 1.582 (15.72), 1.595 (15.97), 2.699 (1.56), 3.301 (0.50), 3.315 (0.40), 5.756 (4.40), 6.056 (0.99), 6.069 (3.32), 6.082 (3.36), 6.095 (0.95), 6.593 (5.28), 7.265 (0.71), 7.268 (1.36), 7.270 (0.80), 7.278 (0.98), 7.282 (3.88), 7.287 (1.13), 7.294 (1.58), 7.297 (2.83), 7.299 (1.51), 7.343 (4.73), 7.358 (8.08), 7.369 (1.31), 7.372 (3.82), 7.492 (6.63), 7.507 (5.84), 7.509 (4.05), 7.552 (16.00).

### EXPERIMENTAL SECTION - EXAMPLES

### Example 1

### (rac)-2'-[6-amino-5-(trifluoromethyl)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide

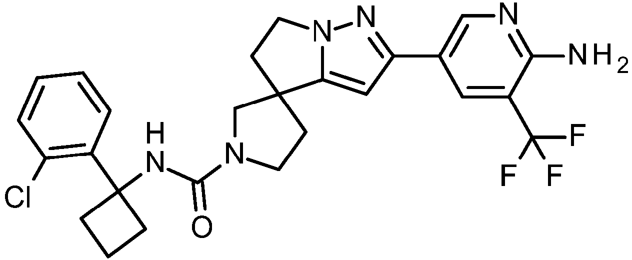

1-(2-Chlorophenyl)cyclobutan-1-amine-hydrochloride (50.0 mg, 229 µmol), N,N'-carbonyldiimidazole (40.6 mg, 250 µmol) and N,N-diisopropylethylamine (360 µL, 2.1 mmol) were solubilised in DMF (3.0 mL) and the mixture was stirred for 1h at rt. (Rac)-5-[5',6'-dihydrospiro[pyrrolidine-3,4'-pyrrolo[1,2-b]pyrazol]-2'-yl]-3-(trifluoromethyl)pyridin-2-amine-hydrochloride (75.0 mg, 208 µmol) solubilised in DMF was added dropwise and the mixture was stirred overnight at 60°C. The mixture was purified by preparative HPLC to give 5.90 mg (99 % purity, 5 % yield) of the target compound.

1H-NMR (400 MHz, DMSO-d6) δ [ppm]: 1.649 (0.74), 1.659 (1.29), 1.670 (1.83), 1.682 (1.83), 1.686 (1.89), 1.697 (2.04), 1.708 (1.54), 1.719 (0.94), 1.730 (0.52), 2.004 (2.70), 2.017 (4.47), 2.030 (5.91), 2.047 (4.86), 2.069 (2.58), 2.074 (2.81), 2.095 (1.61), 2.116 (0.45), 2.322 (0.67), 2.326 (0.95), 2.332 (0.64), 2.434 (0.42), 2.452 (1.89), 2.467 (4.34), 2.476 (6.63), 2.518 (6.13), 2.522 (5.23), 2.539 (2.60), 2.548 (3.18), 2.569 (1.41), 2.597 (1.96), 2.608 (2.31), 2.621 (3.72), 2.632 (3.25), 2.639 (2.61), 2.644 (2.93), 2.655 (2.90), 2.664 (1.86), 2.668 (2.43), 2.678 (1.42), 3.355 (2.66), 3.365 (3.07), 3.382 (7.91), 3.393 (9.26), 3.420 (2.19), 3.451 (1.46), 3.469 (2.70), 3.484 (1.96), 3.493 (1.76), 3.509 (0.87), 4.143 (5.61), 4.161 (11.19), 4.178 (5.36), 6.344 (12.15), 6.520 (10.84), 6.554 (16.00), 7.155 (2.53), 7.159 (2.66), 7.173 (6.08), 7.178 (6.23), 7.192 (5.65), 7.197 (5.41), 7.224 (4.29), 7.228 (5.50), 7.243 (6.22), 7.247 (7.62), 7.261 (3.30), 7.265 (3.30), 7.273 (9.75), 7.276 (8.03), 7.291 (6.50), 7.295 (5.85), 7.528 (6.30), 7.532 (6.33), 7.547 (5.70), 7.551 (5.41), 7.973 (8.88), 7.978 (8.93), 8.544 (8.28), 8.548 (8.09).

### Example 2

### (rac)-2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide

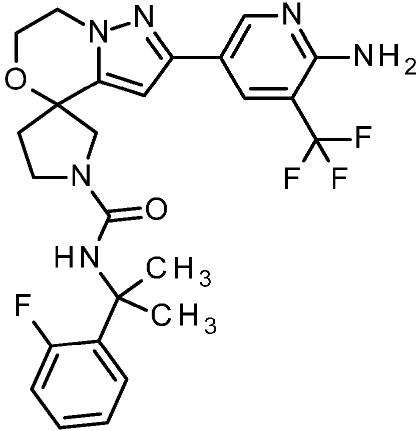

Example 2 was prepared in analogy to Example 1 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride and 2-(2-fluorophenyl)propan-2-amine-hydrochloride.

1H-NMR (400 MHz, DMSO-d6) δ [ppm]: 1.066 (0.63), 1.629 (15.95), 1.635 (16.00), 2.212 (0.78), 2.221 (0.87), 2.247 (1.23), 2.270 (0.66), 2.315 (1.04), 2.327 (1.43), 2.346 (0.72), 2.362 (0.61), 2.522 (1.29), 3.363 (0.72), 3.389 (1.23), 3.406 (1.24), 3.431 (0.57), 3.471 (1.84), 3.501 (2.15), 3.570 (0.94), 3.591 (1.66), 3.613 (0.75), 3.779 (1.95), 3.808 (1.66), 4.069 (0.59), 4.084 (1.25), 4.101 (1.78), 4.119 (2.96), 4.130 (5.04), 4.142 (5.33), 6.114 (5.49), 6.600 (6.89), 6.760 (10.59), 7.030 (1.27), 7.033 (1.42), 7.050 (1.64), 7.053 (1.85), 7.062 (1.32), 7.065 (1.58), 7.074 (1.64), 7.078 (1.68), 7.084 (2.16), 7.093 (3.41), 7.096 (2.79), 7.112 (2.30), 7.115 (1.95), 7.190 (0.85), 7.194 (1.02), 7.202 (1.01), 7.207 (1.42), 7.214 (1.35), 7.221 (1.32), 7.226 (1.32), 7.233 (0.70), 7.240 (0.61), 7.245 (0.55), 7.300 (1.39), 7.303 (1.38), 7.321 (2.22), 7.341 (1.22), 7.345 (1.07), 8.014 (4.23), 8.019 (4.31), 8.583 (4.00), 8.587 (3.98).

### Example 3

### (rac)-2-[6-amino-5-(trifluoromethyl)pyridin-3-yl]-N-(1-phenylcyclobutyl)-6,7-dihydrospiro[pyrazolo[5,1-c][1,4]oxazine-4,3'-pyrrolidine]-1'-carboxamide

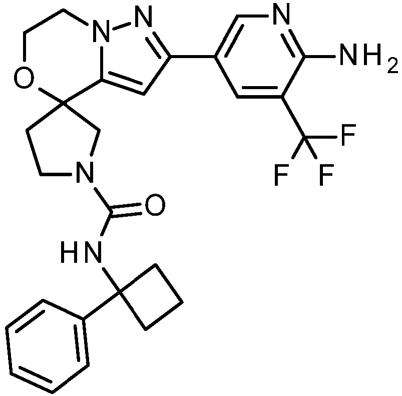

Example 3 was prepared in analogy to Example 1 using (rac)-5-[6,7-dihydrospiro[pyrazolo[5,1-c][1,4]oxazine-4,3'-pyrrolidin]-2-yl]-3-(trifluoromethyl)pyridin-2-amine and 1-phenylcyclobutan-1-amine-hydrochloride.

1H-NMR (400 MHz, DMSO-d6) δ [ppm]: 0.999 (0.75), 1.016 (0.86), 1.042 (0.43), 1.742 (0.89), 1.759 (1.68), 1.764 (1.79), 1.780 (1.79), 1.786 (2.00), 1.803 (1.25), 1.825 (0.50), 1.964 (0.86), 1.970 (1.07), 1.986 (1.90), 2.009 (1.75), 2.013 (1.68), 2.030 (1.04), 2.036 (1.00), 2.052 (0.64), 2.074 (0.64), 2.176 (0.61), 2.207 (1.36), 2.233 (1.90), 2.257 (1.00), 2.318 (2.04), 2.322 (2.86), 2.326 (3.94), 2.331 (3.15), 2.351 (1.72), 2.368 (2.29), 2.374 (2.43), 2.398 (3.65), 2.419 (2.90), 2.441 (3.54), 2.464 (4.94), 2.518 (8.88), 2.522 (5.69), 2.664 (1.54), 2.669 (2.08), 2.673 (1.54), 3.379 (2.47), 3.449 (2.40), 3.478 (2.72), 3.558 (1.61), 3.580 (2.76), 3.603 (1.29), 3.757 (3.29), 3.785 (2.83), 4.051 (1.11), 4.066 (2.04), 4.081 (2.79), 4.099 (3.22), 4.111 (3.54), 4.119 (5.87), 4.133 (10.49), 4.184 (0.79), 6.418 (1.22), 6.555 (0.97), 6.599 (12.78), 6.702 (8.20), 6.748 (16.00), 7.138 (1.54), 7.141 (2.76), 7.144 (1.83), 7.159 (6.80), 7.164 (2.68), 7.174 (2.68), 7.178 (4.65), 7.181 (2.58), 7.256 (0.61), 7.270 (7.80), 7.290 (12.42), 7.303 (2.54), 7.309 (7.19), 7.413 (10.99), 7.416 (12.74), 7.433 (9.91), 7.437 (8.09), 8.004 (7.59), 8.009 (7.55), 8.572 (7.55), 8.576 (7.41).

### Example 4

### (rac)-2'-[6-amino-5-(trifluoromethyl)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide

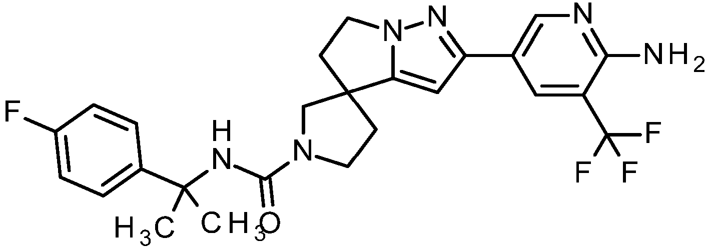

Example 4 was prepared in analogy to Example 1 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(4-fluorophenyl)propan-2-amine.

1H-NMR (400 MHz, DMSO-d6) δ [ppm]: 1.547 (15.16), 1.556 (16.00), 2.024 (0.40), 2.038 (1.09), 2.052 (2.16), 2.069 (2.59), 2.073 (3.02), 2.086 (1.07), 2.099 (0.53), 2.518 (3.81), 2.534 (3.73), 2.539 (3.86), 2.553 (2.37), 3.394 (0.53), 3.413 (1.09), 3.420 (1.32), 3.449 (5.63), 3.476 (0.57), 3.532 (0.59), 3.549 (1.12), 3.564 (0.86), 3.575 (0.82), 3.590 (0.43), 4.175 (2.65), 4.193 (4.91), 4.210 (2.63), 6.071 (5.18), 6.456 (11.78), 6.558 (6.90), 7.020 (3.48), 7.025 (1.21), 7.042 (7.50), 7.059 (1.29), 7.064 (3.99), 7.072 (0.49), 7.351 (3.60), 7.357 (1.63), 7.365 (4.02), 7.374 (3.80), 7.382 (1.47), 7.388 (3.30), 8.008 (3.90), 8.013 (3.95), 8.148 (0.63), 8.591 (3.68), 8.596 (3.63).

### Example 5

### (rac)-2'-[6-amino-5-(trifluoromethyl)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide

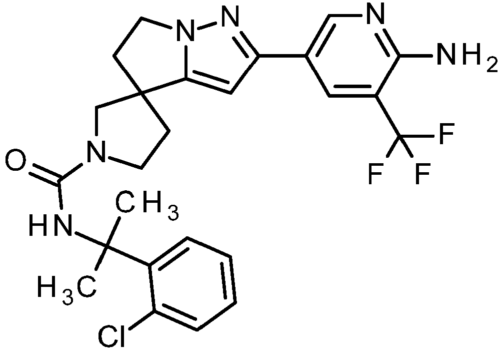

Example 5 was prepared in analogy to Example 1 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(2-chlorophenyl)propan-2-amine-hydrochloride.

¹H-NMR (400 MHz, DMSO-d6) δ [ppm]: 1.231 (0.74), 1.683 (16.00), 1.692 (15.08), 2.033 (1.15), 2.047 (2.21), 2.064 (2.62), 2.081 (1.09), 2.094 (0.51), 2.336 (0.60), 2.518 (11.25), 2.522 (6.20), 2.529 (2.70), 2.678 (0.65), 3.382 (0.71), 3.416 (8.52), 3.443 (0.79), 3.488 (0.68), 3.506 (1.27), 3.521 (0.92), 3.530 (0.82), 3.546 (0.42), 4.162 (2.58), 4.180 (5.48), 4.198 (2.51), 5.759 (8.09), 6.121 (5.58), 6.432 (11.74), 6.559 (7.21), 7.157 (1.11), 7.161 (1.12), 7.176 (2.60), 7.180 (2.73), 7.195 (2.50), 7.199 (2.38), 7.227 (1.68), 7.231 (2.24), 7.247 (2.35), 7.250 (2.99), 7.265 (1.51), 7.269 (1.51), 7.275 (4.51), 7.279 (3.76), 7.294 (3.07), 7.298 (2.67), 7.457 (2.72), 7.461 (2.81), 7.477 (2.35), 7.481 (2.26), 7.995 (4.07), 8.000 (4.11), 8.204 (0.64), 8.575 (3.81), 8.579 (3.77).

### Example 6

### (rac)-2-[6-amino-5-(trifluoromethyl)pyridin-3-yl]-N-[(1R)-1-(2-chloro-4-cyanophenyl)ethyl]-6, 7-dihydrospiro[pyrazolo[5,1-c][1,4]oxazine-4,3'-pyrrolidine]-1'-carboxamide

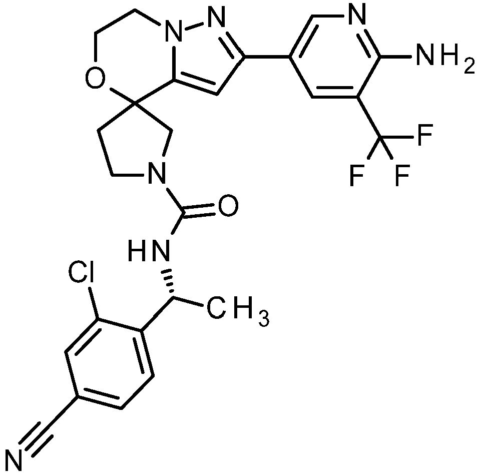

Example 6 was prepared in analogy to Example 1 using (rac)-tert-butyl-2-[6-amino-5-(trifluoromethyl)pyridin-3-yl]-6,7-dihydrospiro[pyrazolo[5,1-c][1,4]oxazine-4,3'-pyrrolidine]-1'-carboxylate and 4-[(1R)-1-aminoethyl]-3-chlorobenzonitrile.

1H-NMR (400 MHz, DMSO-d6) δ [ppm]: 0.829 (0.56), 1.052 (0.68), 1.329 (15.85), 1.347 (16.00), 2.073 (5.58), 2.202 (0.49), 2.226 (1.48), 2.254 (1.78), 2.278 (0.82), 2.322 (0.51), 2.327 (0.74), 2.332 (0.85), 2.354 (1.81), 2.518 (1.97), 2.522 (1.27), 2.669 (0.51), 3.387 (1.42), 3.404 (1.60), 3.430 (1.68), 3.449 (2.11), 3.478 (1.55), 3.514 (1.26), 3.544 (1.53), 3.559 (1.05), 3.583 (1.54), 3.606 (0.68), 3.644 (0.88), 3.666 (1.57), 3.689 (0.72), 3.773 (1.93), 3.802 (1.60), 3.850 (1.98), 3.877 (1.71), 4.052 (0.76), 4.066 (1.61), 4.082 (2.54), 4.100 (2.81), 4.112 (3.46), 4.123 (4.58), 4.132 (6.17), 4.138 (8.08), 4.144 (9.33), 5.108 (0.48), 5.120 (1.82), 5.126 (2.02), 5.138 (2.90), 5.144 (3.04), 5.156 (1.95), 5.162 (1.92), 5.172 (0.49), 6.600 (12.74), 6.760 (8.29), 6.766 (9.95), 6.784 (2.39), 6.803 (2.28), 6.822 (2.24), 6.841 (2.09), 7.661 (4.45), 7.681 (7.00), 7.700 (5.28), 7.819 (3.39), 7.823 (3.51), 7.832 (3.83), 7.836 (4.19), 7.839 (3.00), 7.843 (2.97), 7.851 (2.84), 7.856 (3.00), 7.988 (10.66), 7.991 (10.30), 8.008 (4.39), 8.014 (6.71), 8.021 (4.68), 8.136 (3.18), 8.576 (4.07), 8.581 (4.19), 8.587 (4.49), 8.592 (4.20).

### Example 7

### (rac)-2'-[6-amino-5-(trifluoromethyl)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide

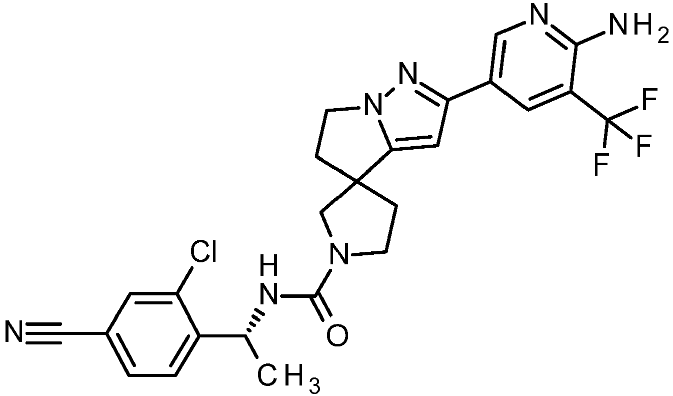

Example 7 was prepared in analogy to Example 1 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 4-[(1R)-1-aminoethyl]-3-chlorobenzonitrile.

1H-NMR (400 MHz, DMSO-d6) δ [ppm]: 1.323 (9.01), 1.330 (9.90), 1.341 (9.38), 1.347 (9.52), 2.074 (3.85), 2.083 (3.94), 2.518 (4.04), 2.523 (5.54), 2.539 (15.01), 2.558 (3.80), 3.382 (0.47), 3.429 (2.86), 3.453 (5.44), 3.483 (3.00), 3.493 (3.75), 3.518 (2.25), 3.530 (1.50), 3.546 (0.94), 3.556 (0.75), 3.574 (0.70), 3.594 (0.94), 4.172 (3.05), 4.177 (3.28), 4.190 (5.35), 4.194 (5.96), 4.207 (3.19), 4.212 (2.82), 5.111 (0.56), 5.128 (2.53), 5.146 (3.94), 5.164 (2.53), 5.181 (0.56), 6.472 (16.00), 6.544 (12.57), 6.773 (2.21), 6.789 (3.66), 6.807 (2.16), 7.642 (3.05), 7.663 (3.94), 7.705 (2.96), 7.725 (4.08), 7.775 (2.39), 7.778 (2.39), 7.795 (1.83), 7.799 (1.92), 7.808 (3.24), 7.812 (3.28), 7.828 (2.11), 7.832 (2.25), 7.980 (6.71), 7.984 (8.73), 7.989 (6.10), 8.014 (6.57), 8.133 (0.70), 8.590 (6.57), 8.595 (6.43).

### Example 8

### (rac)-2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide

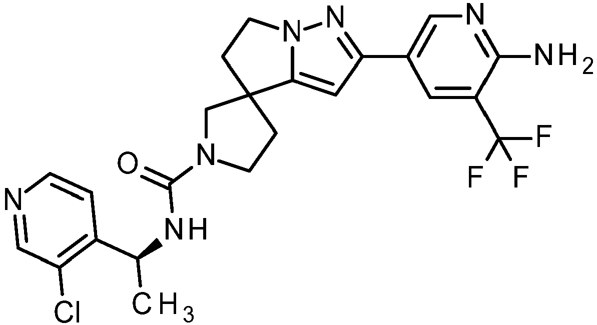

Example 8 was prepared in analogy to Example 1 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and (1S)-1-(3-chloropyridin-4-yl)ethan-1-amine-hydrochloride.

¹H-NMR (400 MHz, DMSO-d6) δ [ppm]: 1.232 (0.89), 1.332 (12.11), 1.336 (12.56), 1.350 (12.44), 1.353 (12.48), 1.902 (3.12), 2.085 (4.66), 2.323 (1.66), 2.327 (2.35), 2.332 (1.70), 2.518 (10.21), 2.523 (6.97), 2.530 (5.51), 2.548 (7.90), 2.566 (4.82), 2.660 (0.81), 2.665 (1.74), 2.669 (2.43), 2.673 (1.74), 3.420 (0.81), 3.438 (3.40), 3.464 (6.64), 3.491 (4.62), 3.520 (2.55), 3.539 (1.78), 3.555 (1.09), 3.565 (0.89), 3.583 (0.81), 3.603 (1.13), 3.627 (0.85), 4.179 (4.17), 4.195 (7.37), 4.211 (3.93), 5.036 (0.57), 5.054 (2.35), 5.072 (3.61), 5.088 (2.39), 5.106 (0.61), 6.469 (16.00), 6.552 (13.73), 6.762 (2.63), 6.779 (3.93), 6.795 (2.43), 7.454 (3.48), 7.466 (3.56), 7.532 (3.77), 7.544 (3.85), 8.014 (7.53), 8.019 (7.74), 8.375 (0.41), 8.435 (4.09), 8.448 (3.89), 8.477 (5.87), 8.490 (5.63), 8.525 (11.50), 8.528 (11.42), 8.588 (7.53), 8.593 (7.45).

### Example 9

### (rac)-2'-[6-amino-5-(trifluoromethyl)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide

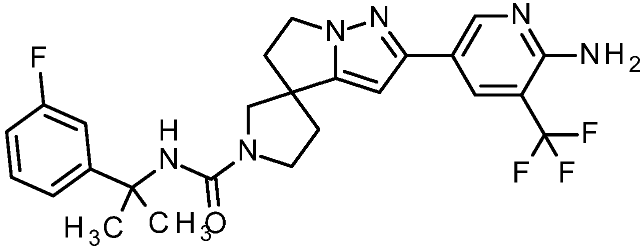

Example 9 was prepared in analogy to Example 1 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(3-fluorophenyl)propan-2-amine.

1H-NMR (400 MHz, DMSO-d6) δ [ppm]: 1.550 (14.22), 1.556 (16.00), 2.047 (1.03), 2.062 (1.97), 2.076 (2.35), 2.092 (1.00), 2.327 (0.78), 2.523 (4.68), 2.544 (3.82), 2.561 (2.43), 2.669 (0.78), 3.426 (1.55), 3.451 (3.61), 3.466 (3.18), 3.492 (0.78), 3.542 (0.54), 3.559 (1.01), 4.178 (2.35), 4.196 (4.13), 4.213 (2.35), 6.121 (4.44), 6.444 (8.64), 6.556 (6.03), 6.925 (0.79), 6.931 (0.92), 6.946 (1.50), 6.951 (1.89), 6.967 (0.88), 6.974 (0.93), 7.112 (1.64), 7.117 (1.33), 7.140 (1.63), 7.175 (1.73), 7.196 (2.52), 7.257 (1.22), 7.276 (2.11), 7.292 (1.90), 7.312 (0.76), 8.003 (3.38), 8.009 (3.60), 8.584 (3.25), 8.588 (3.36).

### Example 10

### (rac)-2-[6-amino-5-(trifluoromethyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide

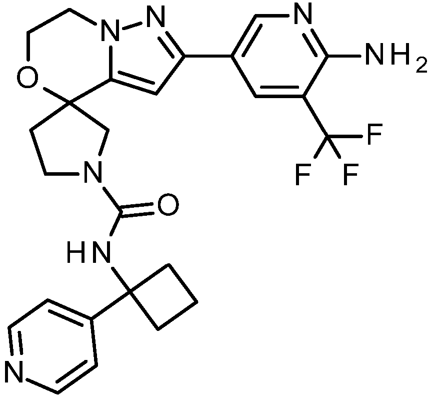

Example 10 was prepared in analogy to Example 1 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride and 1-(pyridin-4-yl)cyclobutan-1-amine.

¹H-NMR (400 MHz, DMSO-d6) δ [ppm]: 1.009 (0.86), 1.026 (0.86), 1.231 (1.35), 1.822 (2.02), 1.843 (2.40), 1.861 (1.59), 2.007 (2.35), 2.030 (2.16), 2.074 (6.87), 2.221 (1.87), 2.245 (2.40), 2.269 (1.30), 2.327 (5.33), 2.344 (3.84), 2.387 (5.24), 2.411 (7.78), 2.430 (7.35), 2.451 (5.24), 2.669 (2.88), 3.471 (2.45), 3.498 (2.74), 3.602 (3.17), 3.770 (3.75), 3.797 (3.36), 4.062 (1.44), 4.078 (2.93), 4.093 (3.56), 4.129 (10.38), 4.141 (14.32), 6.604 (16.00), 6.765 (14.75), 6.884 (8.84), 7.039 (0.82), 7.386 (15.23), 7.390 (10.19), 7.402 (14.75), 7.638 (0.48), 8.010 (9.61), 8.014 (9.90), 8.140 (14.94), 8.205 (0.58), 8.476 (15.57), 8.480 (10.09), 8.491 (14.32), 8.577 (8.98).

### Example 11

### (rac)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide

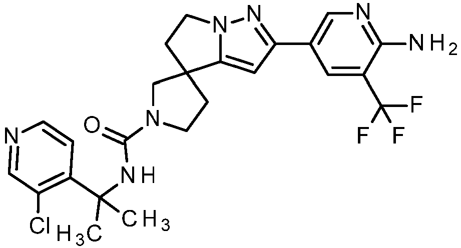

Example 11 was prepared in analogy to Example 1 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(3-chloropyridin-4-yl)propan-2-amine.

¹H-NMR (400 MHz, DMSO-d6) δ [ppm]: 1.164 (0.49), 1.232 (0.79), 1.247 (0.70), 1.257 (0.82), 1.332 (0.78), 1.560 (0.62), 1.654 (16.00), 2.036 (0.59), 2.051 (1.09), 2.067 (1.30), 2.084 (0.56), 2.322 (0.47), 2.327 (0.65), 2.332 (0.48), 2.518 (3.72), 2.522 (2.95), 2.537 (0.96), 2.664 (0.46), 2.669 (0.66), 2.673 (0.48), 2.728 (2.37), 2.888 (3.01), 3.417 (4.16), 3.440 (0.54), 3.505 (0.64), 3.521 (0.48), 3.528 (0.43), 4.165 (1.43), 4.183 (3.04), 4.200 (1.41), 5.758 (1.02), 6.332 (2.86), 6.443 (5.96), 6.558 (3.76), 7.427 (2.41), 7.440 (2.44), 8.001 (2.08), 8.006 (2.16), 8.147 (3.29), 8.389 (3.42), 8.402 (3.34), 8.408 (6.45), 8.578 (1.96), 8.581 (1.98).

### Example 12

### (rac)-2'-[6-amino-5-(trifluoromethyl)pyridin-3-yl]-N-(1-phenylcyclobutyl)-5',6'-dihydrospiro[pyrrolidine-3,4'-pyrrolo[1,2-b]pyrazole]-1-carboxamide

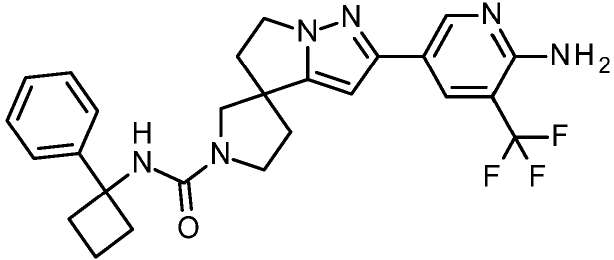

Example 12 was prepared in analogy to Example 1 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 1-phenylcyclobutan-1-amine-hydrochloride.

1H-NMR (400 MHz, DMSO-d6) δ [ppm]: 0.999 (0.66), 1.015 (0.69), 1.042 (0.42), 1.056 (1.13), 1.066 (0.98), 1.072 (1.13), 1.153 (3.41), 1.171 (6.70), 1.189 (3.35), 1.230 (2.11), 1.243 (2.86), 1.248 (1.82), 1.259 (3.21), 1.262 (3.13), 1.279 (2.49), 1.745 (0.76), 1.762 (1.46), 1.768 (1.56), 1.784 (1.62), 1.790 (1.80), 1.807 (1.14), 1.829 (0.44), 1.957 (0.79), 1.963 (0.94), 1.979 (1.86), 1.986 (13.20), 2.002 (1.85), 2.021 (1.54), 2.033 (2.34), 2.048 (3.61), 2.061 (4.05), 2.078 (1.98), 2.326 (0.65), 2.354 (1.36), 2.376 (2.75), 2.385 (3.45), 2.401 (4.11), 2.407 (3.48), 2.422 (2.50), 2.447 (2.01), 2.470 (3.84), 2.526 (7.40), 2.530 (6.83), 2.546 (4.36), 2.668 (0.61), 3.127 (0.43), 3.383 (0.98), 3.401 (3.15), 3.426 (8.82), 3.436 (6.98), 3.462 (1.72), 3.509 (1.57), 3.526 (2.71), 3.542 (2.34), 3.552 (2.35), 3.569 (1.92), 3.662 (3.47), 3.999 (1.02), 4.016 (2.82), 4.034 (2.80), 4.052 (1.00), 4.141 (0.47), 4.167 (4.66), 4.184 (8.94), 4.201 (4.69), 6.424 (16.00), 6.597 (1.82), 6.702 (7.56), 6.745 (0.79), 7.011 (0.86), 7.127 (2.25), 7.146 (5.62), 7.164 (3.82), 7.255 (6.46), 7.275 (10.79), 7.294 (6.17), 7.419 (9.98), 7.437 (8.42), 7.440 (6.54), 7.700 (0.55), 8.009 (6.99), 8.014 (7.10), 8.574 (6.54), 8.579 (6.46).

### Example 13

### (rac)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide

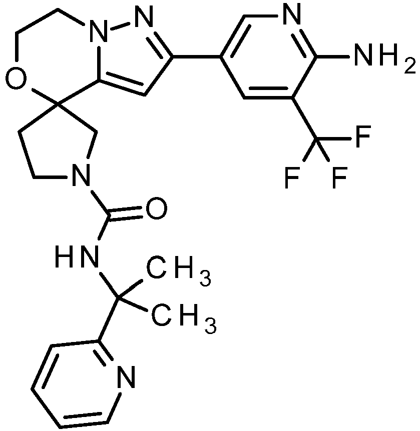

Example 13 was prepared in analogy to Example 1 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride and 2-(pyridin-2-yl)propan-2-amine.

1H-NMR (400 MHz, DMSO-d6) δ [ppm]: 1.177 (0.43), 1.194 (0.45), 1.232 (0.58), 1.579 (16.00), 1.585 (15.80), 2.075 (1.24), 2.240 (0.63), 2.265 (0.86), 2.290 (0.45), 2.332 (1.59), 2.337 (1.21), 2.352 (0.88), 2.368 (0.48), 2.385 (0.43), 2.518 (5.38), 2.523 (3.58), 2.540 (0.66), 2.674 (1.09), 2.679 (0.48), 3.387 (0.68), 3.411 (0.96), 3.429 (0.91), 3.454 (0.45), 3.501 (1.19), 3.530 (1.41), 3.605 (0.73), 3.626 (1.31), 3.648 (0.61), 3.801 (1.59), 3.829 (1.34), 4.097 (0.86), 4.115 (1.56), 4.136 (5.15), 4.146 (3.89), 6.425 (4.06), 6.601 (5.70), 6.776 (9.44), 7.171 (1.62), 7.174 (1.72), 7.184 (1.64), 7.186 (1.89), 7.190 (1.89), 7.193 (1.74), 7.202 (1.84), 7.205 (1.84), 7.438 (1.74), 7.440 (3.08), 7.458 (2.07), 7.461 (3.33), 7.698 (1.79), 7.702 (1.74), 7.717 (2.07), 7.721 (2.12), 7.736 (1.41), 7.740 (1.31), 8.017 (3.41), 8.022 (3.46), 8.163 (2.22), 8.449 (1.84), 8.451 (2.15), 8.453 (2.25), 8.455 (1.92), 8.461 (1.92), 8.463 (2.22), 8.465 (2.07), 8.467 (1.77), 8.587 (3.18), 8.591 (3.18).

### Example 14

### (rac)-2'-(5-acetyl-6-aminopyridin-3-yl)-N-(1-phenylcyclobutyl)-5',6'-dihydrospiro[pyrrolidine-3,4'-pyrrolo[1,2-b]pyrazole]-1-carboxamide

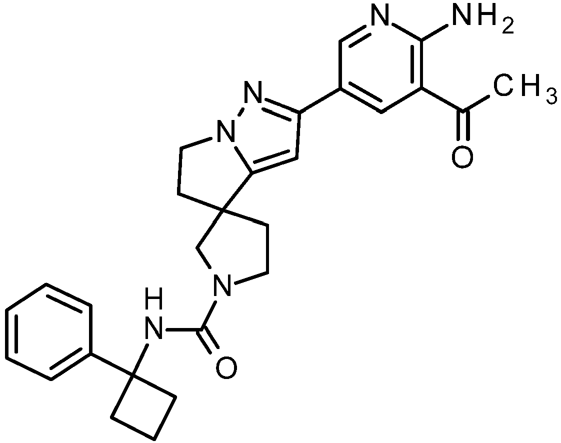

(Rac)-1-[(1-phenylcyclobutyl)carbamoyl]-5',6'-dihydrospiro[pyrrolidine-3,4'-pyrrolo[1,2-b]pyrazol]-2'-yl trifluoromethanesulfonate (194 mg, 400 µmol), (5-acetyl-6-aminopyridin-3-yl)boronic acid (82.8 mg, 460 µmol), XPhos Pd G2 (47.2 mg, 60.0 µmol) and potassium phosphate (2.4 mL, 0.50 M, 1.2 mmol) were solubilised in dioxane (4.0 mL) and the mixture was stirred for 1h at 100°C. The mixture was diluted with water and extracted three times with ethyl acetate. The combined organic layers were dried and evaporated. The residue was purified by preparative HPLC to give 27.5 mg (97 % purity, 14 % yield) of the target compound.

1H-NMR (400 MHz, DMSO-d6) δ [ppm]: 1.036 (1.47), 1.053 (2.85), 1.071 (1.56), 1.764 (0.45), 1.769 (0.47), 1.786 (0.49), 1.791 (0.56), 1.982 (0.53), 1.997 (0.41), 2.005 (0.50), 2.009 (0.51), 2.026 (0.41), 2.032 (0.44), 2.045 (0.71), 2.059 (1.09), 2.072 (1.24), 2.089 (0.56), 2.359 (0.40), 2.382 (0.86), 2.390 (1.00), 2.397 (0.83), 2.405 (1.22), 2.411 (0.97), 2.427 (0.71), 2.452 (0.70), 2.475 (1.30), 2.518 (2.01), 2.523 (1.96), 2.537 (1.90), 2.542 (1.85), 2.558 (1.18), 2.596 (16.00), 3.160 (3.62), 3.172 (3.72), 3.418 (0.85), 3.423 (1.03), 3.447 (3.80), 3.535 (0.65), 3.551 (0.49), 3.560 (0.45), 4.101 (0.67), 4.114 (0.66), 4.181 (1.46), 4.198 (2.81), 4.215 (1.43), 6.463 (4.93), 6.704 (2.38), 7.134 (0.71), 7.137 (0.43), 7.152 (1.77), 7.167 (0.72), 7.170 (1.19), 7.173 (0.69), 7.262 (1.98), 7.282 (3.33), 7.296 (0.73), 7.301 (1.92), 7.421 (2.89), 7.423 (3.12), 7.441 (2.60), 7.445 (2.01), 8.381 (2.64), 8.387 (2.78), 8.597

### Example 15

### (rac)-2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide

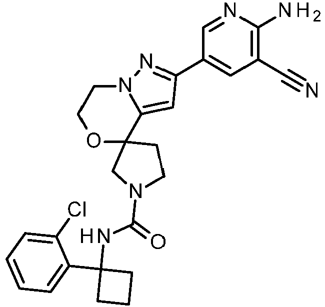

1-(2-Chlorophenyl)cyclobutan-1-amine hydrochloride (55.8 mg, 256 µmol), 1,1'-carbonyldiimidazole (41.5 mg, 256 µmol) and N,N-diisopropylethylamine (150 µL, 850 µmol) were stirred in DMF (1.0 mL) for 1h at rt, then (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) in DMF (1.0 mL) was added and stirred at rt overnight. Water was added to the mixture and purified by HPLC to afford 26.0 mg (95 % purity, 23 % yield) of the title compound.

¹H-NMR (400 MHz, DMSO-d6) δ [ppm]: 0.931 (2.90), 0.948 (2.92), 1.652 (0.65), 1.664 (1.10), 1.675 (1.60), 1.690 (1.67), 1.702 (1.78), 1.712 (1.33), 1.723 (0.77), 1.734 (0.43), 2.029 (1.32), 2.050 (2.36), 2.074 (6.68), 2.098 (1.44), 2.119 (0.53), 2.131 (0.65), 2.164 (1.40), 2.188 (1.86), 2.213 (0.94), 2.281 (1.65), 2.297 (1.94), 2.318 (1.68), 2.322 (2.22), 2.326 (2.88), 2.332 (2.43), 2.451 (0.99), 2.518 (8.72), 2.522 (6.66), 2.544 (2.14), 2.565 (0.94), 2.646 (3.19), 2.660 (2.48), 2.664 (3.05), 2.668 (3.22), 2.673 (2.43), 3.365 (0.98), 3.392 (2.48), 3.420 (2.83), 3.502 (1.48), 3.525 (2.65), 3.549 (1.19), 3.720 (2.76), 3.750 (2.36), 4.022 (1.15), 4.037 (1.94), 4.053 (2.92), 4.072 (2.92), 4.085 (3.48), 4.093 (4.09), 4.112 (9.21), 6.539 (9.98), 6.663 (16.00), 7.044 (14.11), 7.176 (2.05), 7.181 (2.20), 7.195 (5.77), 7.199 (5.53), 7.214 (5.23), 7.218 (5.24), 7.230 (4.31), 7.234 (4.92), 7.249 (5.67), 7.252 (6.78), 7.267 (2.85), 7.271 (2.46), 7.298 (8.25), 7.302 (7.98), 7.317 (5.47), 7.321 (5.39), 7.522 (5.56), 7.527 (5.44), 7.541 (5.20), 7.546 (4.68), 8.084 (10.87), 8.090 (11.42), 8.575 (12.43), 8.580 (12.81).

### Example 16

### (rac)-2'-(6-amino-5-cyanopyridin-3-yl)-N-[1-(2-chlorophenyl)cyclobutyl]-5',6'-dihydrospiro[pyrrolidine-3,4'-pyrrolo[1,2-b]pyrazole]-1-carboxamide

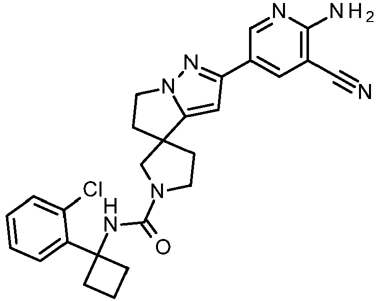

1-(2-Chlorophenyl)cyclobutan-1-amine hydrochloride (63.6 mg, 292 µmol), 1,1'-carbonyldiimidazole (47.3 mg, 292 µmol) and N,N-diisopropylethylamine (170 µL, 970 µmol) were stirred in DMF (1 mL) for 1h at rt, then (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) in DMF (1.0 mL) was added and stirred at rt overnight. Water was added to the mixture and purified by HPLC to afford 50.0 mg (95 % purity, 40 % yield) of the title compound.

¹H-NMR (400 MHz, DMSO-d6) δ [ppm]: 0.932 (2.38), 0.948 (2.33), 1.648 (1.13), 1.670 (2.19), 1.687 (1.88), 1.697 (2.02), 1.708 (1.46), 1.719 (0.81), 2.002 (3.39), 2.020 (5.99), 2.047 (3.39), 2.068 (2.62), 2.073 (2.69), 2.094 (1.72), 2.117 (0.92), 2.124 (0.81), 2.131 (0.68), 2.146 (1.73), 2.157 (0.47), 2.174 (0.42), 2.199 (0.41), 2.322 (1.69), 2.326 (2.27), 2.332 (1.62), 2.386 (0.47), 2.407 (0.82), 2.413 (0.96), 2.435 (1.16), 2.454 (2.06), 2.466 (4.40), 2.518 (10.61), 2.522 (8.42), 2.548 (2.78), 2.570 (1.24), 2.600 (1.85), 2.611 (2.21), 2.623 (3.54), 2.634 (3.07), 2.646 (2.77), 2.659 (3.18), 2.664 (2.91), 2.669 (3.53), 2.673 (2.51), 2.678 (1.57), 2.720 (4.16), 3.289 (0.40), 3.378 (8.40), 3.387 (9.66), 3.411 (2.46), 3.438 (1.47), 3.456 (2.75), 3.471 (1.82), 3.480 (1.75), 3.496 (0.81), 4.141 (5.33), 4.159 (10.80), 4.176 (5.06), 6.310 (11.34), 6.524 (10.14), 7.000 (16.00), 7.168 (2.30), 7.173 (2.61), 7.187 (6.10), 7.192 (5.86), 7.206 (5.69), 7.210 (5.84), 7.223 (0.96), 7.230 (5.17), 7.233 (5.54), 7.248 (5.97), 7.252 (6.98), 7.267 (3.32), 7.271 (4.33), 7.275 (2.11), 7.288 (9.52), 7.291 (8.38), 7.307 (5.83), 7.310 (5.44), 7.341 (0.98), 7.362 (0.67), 7.530 (6.16), 7.535 (5.99), 7.549 (5.48), 7.554 (5.04), 8.074 (11.61), 8.079 (12.11), 8.571 (14.30), 8.576 (14.19).

### Example 17

### (rac)-2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide

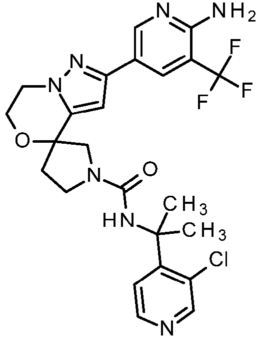

To half of the reaction mixture of 3-chloro-4-(2-isocyanatopropan-2-yl)pyridine (80.0 mg, 407 µmol) was added N,N-diisopropylethylamine (180 µL, 1.0 mmol) and (rac)-5-[6,7-dihydrospiro[pyrazolo[5,1-c][1,4]oxazine-4,3'-pyrrolidin]-2-yl]-3-(trifluoromethyl)pyridin-2-amine hydrochloride (127 mg, 339 µmol). The mixture was stirred at rt overnight under nitrogen atmosphere and then 5h at 60°C. Saturated potassium carbonate solution was added and stirred for 5 min. The layers were separated and the aqueous phase was extracted three times with dichloromethane (+10 % methanol). The combined organic phases were concentrated to give 240 mg of a residue which was purified by flash chromatography to afford 82.0 mg (90 % purity, 41 % yield) of the title compound.
¹H NMR (DMSO-d₆) δ: 8.58 (d, 1H), 8.37-8.45 (m, 2H), 8.02 (d, 1H), 7.43 (d, 1H), 6.75 (s, 1H), 6.60 (s, 2H), 6.36 (s, 1H), 4.04-4.19 (m, 4H), 3.76 (br d, 1H), 3.56 (br m, 1H), 3.46 (br d, 1H), 3.38 (br d, 1H), 2.29-2.34 (m, 1H), 2.17-2.27 (m, 1H), 1.66 (d, 6H)

### Example 18

### (rac)-2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide

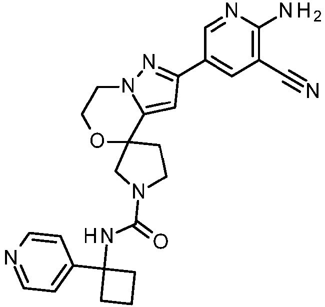

1-(Pyridin-4-yl)cyclobutan-1-amine (37.9 mg, 256 µmol), 1,1'-carbonyldiimidazole (41.5 mg, 256 µmol) and N,N-diisopropylethylamine (150 µL, 850 µmol) was stirred in DMF (1.0 mL) for 1h at rt, then (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) in DMF (1.0 mL) was added and stirred at rt overnight. Water was added to the mixture and purified by HPLC to afford 37.0 mg (95 % purity, 35 % yield) of the title compound.

¹H-NMR (400 MHz, DMSO-d6) δ [ppm]: 1.821 (0.98), 1.843 (1.22), 1.861 (0.77), 2.006 (1.09), 2.029 (1.01), 2.194 (0.83), 2.220 (1.05), 2.323 (2.36), 2.327 (3.38), 2.332 (2.71), 2.387 (2.34), 2.411 (3.31), 2.430 (3.08), 2.452 (2.02), 2.518 (16.00), 2.523 (11.28), 2.665 (1.91), 2.669 (2.52), 2.673 (1.87), 3.295 (0.66), 3.304 (0.82), 3.385 (1.61), 3.445 (1.12), 3.476 (1.19), 3.602 (1.39), 3.768 (1.68), 3.797 (1.49), 4.060 (0.63), 4.075 (1.28), 4.092 (1.65), 4.125 (4.56), 4.136 (5.32), 6.714 (7.99), 6.890 (4.36), 7.051 (7.95), 7.386 (8.34), 7.390 (5.17), 7.398 (5.32), 7.402 (8.36), 8.115 (6.22), 8.121 (6.24), 8.476 (9.25), 8.481 (5.32), 8.488 (5.40), 8.491 (8.53), 8.597 (6.86), 8.603 (6.74).

### Example 19

### (rac)-2-[6-amino-5-(trifluoromethyl)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide

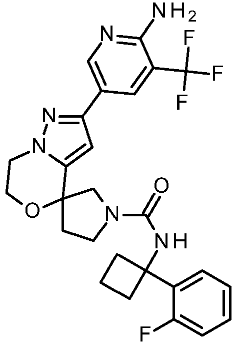

1-(2-Fluorophenyl)cyclobutan-1-amine (52.8 mg, 319 µmol), 1,1'-carbonyldiimidazole (51.8 mg, 319 µmol) and N,N-diisopropylethylamine (280 µL, 1.6 mmol) were stirred in DMF (3 mL) for 1h at rt, then (rac)-5-[6,7-dihydrospiro[pyrazolo[5,1-c][1,4]oxazine-4,3'-pyrrolidin]-2-yl]-3-(trifluoromethyl)pyridin-2-amine hydrochloride (100 mg, 266 µmol) in DMF (3 mL) was added and stirred at 60°C overnight. Water was added to the mixture and purified by HPLC to afford 30.2 mg (95 % purity, 20 % yield) of the title compound.

¹H-NMR (400 MHz, DMSO-d6) δ [ppm]: 0.917 (0.45), 0.932 (3.79), 0.934 (1.29), 0.948 (3.92), 0.952 (0.66), 1.401 (1.31), 1.425 (1.11), 1.707 (0.51), 1.714 (0.62), 1.719 (0.76), 1.727 (1.17), 1.733 (1.19), 1.741 (1.29), 1.748 (1.09), 1.755 (1.37), 1.762 (0.80), 1.768 (0.86), 1.775 (0.60), 2.014 (0.90), 2.033 (1.54), 2.055 (1.42), 2.060 (1.40), 2.074 (4.20), 2.080 (0.88), 2.156 (0.45), 2.187 (1.05), 2.213 (1.44), 2.238 (0.76), 2.292 (1.31), 2.308 (1.60), 2.323 (1.68), 2.327 (2.01), 2.332 (1.46), 2.336 (1.05), 2.405 (0.45), 2.423 (0.45), 2.454 (0.78), 2.518 (7.24), 2.523 (6.95), 2.665 (0.80), 2.669 (1.13), 2.673 (0.82), 3.370 (0.86), 3.417 (2.05), 3.445 (2.40), 3.520 (1.27), 3.542 (2.30), 3.565 (1.03), 3.732 (2.58), 3.761 (2.20), 4.030 (0.90), 4.045 (1.44), 4.061 (2.22), 4.080 (2.15), 4.092 (2.71), 4.102 (2.99), 4.122 (7.51), 6.595 (16.00), 6.729 (11.84), 7.041 (1.81), 7.044 (1.99), 7.061 (2.40), 7.064 (2.71), 7.072 (5.03), 7.091 (8.70), 7.110 (3.32), 7.113 (2.60), 7.197 (1.21), 7.201 (1.37), 7.209 (1.40), 7.214 (2.13), 7.221 (1.83), 7.228 (1.74), 7.233 (1.99), 7.239 (0.96), 7.248 (0.86), 7.252 (0.80), 7.404 (1.85), 7.409 (1.87), 7.425 (3.04), 7.444 (1.74), 7.995 (5.76), 8.000 (5.93), 8.560 (5.46), 8.564 (5.39).

### Example 20

### (rac)-2'-(6-amino-5-cyanopyridin-3-yl)-N-(1-phenylcyclobutyl)-5',6'-dihydrospiro[pyrrolidine-3,4'-pyrrolo[1,2-b]pyrazole]-1-carboxamide

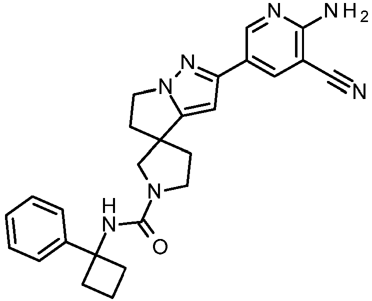

1-Phenylcyclobutan-1-amine hydrochloride (53.6 mg, 292 µmol), 1,1'-carbonyldiimidazole (47.3 mg, 292 µmol) and N,N-diisopropylethylamine (170 µL, 970 µmol) were stirred in DMF (1.0 mL) for 1h at rt, then (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) in DMF (1.0 mL) was added and stirred at rt overnight. Water was added to the mixture and purified by HPLC to afford 25.0 mg (98 % purity, 22 % yield) of the title compound.

¹H-NMR (400 MHz, DMSO-d6) δ [ppm]: 1.741 (0.74), 1.758 (1.41), 1.763 (1.52), 1.780 (1.55), 1.785 (1.74), 1.802 (1.06), 1.824 (0.42), 1.969 (0.89), 1.985 (1.66), 2.007 (1.53), 2.012 (1.46), 2.024 (0.83), 2.151 (0.58), 2.175 (1.27), 2.209 (1.69), 2.233 (0.87), 2.318 (2.18), 2.322 (3.46), 2.326 (4.74), 2.331 (3.60), 2.368 (1.92), 2.375 (1.89), 2.397 (2.95), 2.412 (2.43), 2.418 (2.57), 2.441 (3.19), 2.463 (4.84), 2.518 (12.23), 2.522 (7.48), 2.539 (0.56), 2.660 (0.96), 2.664 (2.19), 2.668 (2.95), 2.673 (2.19), 2.678 (0.96), 3.289 (0.44), 3.356 (1.76), 3.364 (1.83), 3.381 (1.70), 3.408 (0.92), 3.427 (2.11), 3.457 (2.40), 3.558 (1.35), 3.579 (2.43), 3.603 (1.10), 3.753 (3.06), 3.782 (2.60), 4.048 (1.03), 4.064 (1.94), 4.079 (2.31), 4.096 (3.09), 4.103 (3.16), 4.116 (6.34), 4.128 (9.60), 6.696 (16.00), 6.707 (7.85), 6.948 (0.42), 7.046 (12.25), 7.076 (0.60), 7.140 (1.43), 7.143 (2.44), 7.161 (5.83), 7.177 (2.32), 7.180 (4.03), 7.183 (2.23), 7.271 (6.82), 7.291 (10.85), 7.305 (2.21), 7.310 (6.44), 7.413 (9.83), 7.416 (10.71), 7.433 (8.48), 7.437 (6.30), 8.105 (10.31), 8.111 (10.94), 8.592 (12.03), 8.597 (11.75).

### Example 21

### (rac)-2-(6-amino-5-cyanopyridin-3-yl)-N-[1-(4-fluorophenyl)cyclobutyl]-6,7-dihydrospiro[pyrazolo[5,1-c][1,4]oxazine-4,3'-pyrrolidine]-1'-carboxamide

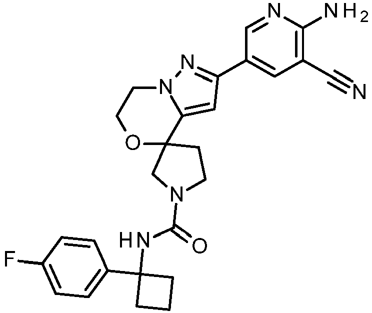

1-(4-Fluorophenyl)cyclobutan-1-amine hydrochloride (51.6 mg, 256 µmol), 1,1'-carbonyldiimidazole (41.5 mg, 256 µmol) and N,N-diisopropylethylamine (150 µL, 850 µmol) were stirred in DMF (1.0 mL) for 1h at rt, then (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) in DMF (1.0 mL) was added and stirred at rt overnight. Water was added to the mixture and purified by HPLC to afford 52.0 mg (95 % purity, 47 % yield) of the title compound.

¹H-NMR (400 MHz, DMSO-d6) δ [ppm]: 1.252 (0.69), 1.723 (0.92), 1.739 (1.72), 1.745 (1.84), 1.761 (1.88), 1.767 (2.06), 1.784 (1.26), 1.805 (0.48), 1.960 (1.07), 1.976 (1.96), 1.998 (1.88), 2.026 (0.92), 2.042 (0.45), 2.074 (2.87), 2.144 (0.67), 2.177 (1.56), 2.203 (2.00), 2.227 (1.02), 2.326 (4.41), 2.331 (3.73), 2.361 (3.58), 2.370 (3.65), 2.386 (3.77), 2.402 (3.09), 2.418 (1.64), 2.432 (3.37), 2.453 (5.34), 2.522 (6.66), 2.665 (1.66), 2.669 (2.21), 2.673 (1.67), 3.354 (2.51), 3.372 (2.18), 3.397 (1.25), 3.418 (2.53), 3.447 (2.85), 3.550 (1.78), 3.572 (3.15), 3.596 (1.49), 3.748 (3.77), 3.777 (3.22), 4.045 (1.18), 4.060 (2.33), 4.076 (2.92), 4.093 (3.79), 4.113 (7.29), 4.126 (11.64), 6.701 (16.00), 6.723 (8.64), 7.046 (14.65), 7.078 (7.60), 7.083 (2.97), 7.100 (15.38), 7.117 (2.90), 7.122 (8.20), 7.422 (7.75), 7.427 (3.57), 7.436 (8.69), 7.444 (7.80), 7.452 (3.17), 7.458 (6.65), 8.108 (10.92), 8.114 (11.21), 8.592 (11.80), 8.598 (11.44).

### Example 22

### (rac)-2'-(6-amino-5-cyanopyridin-3-yl)-N-[1-(4-fluorophenyl)cyclobutyl]-5',6'-dihydrospiro[pyrrolidine-3,4'-pyrrolo[1,2-b]pyrazole]-1-carboxamide

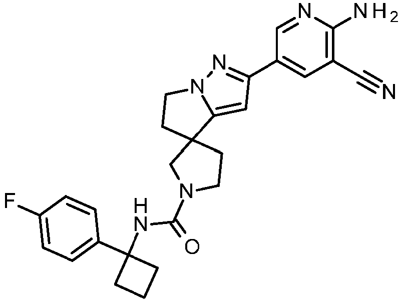

1-(4-Fluorophenyl)cyclobutan-1-amine hydrochloride (58.8 mg, 292 µmol), 1,1'-carbonyldiimidazole (47.3 mg, 292 µmol) and N,N-diisopropylethylamine (170 µL, 970 µmol) were stirred in DMF (1.0 mL) for 1h at rt, then (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) in DMF (1.0 mL) was added and stirred at rt overnight. Water was added to the mixture and purified by HPLC to afford 53.0 mg (99 % purity, 46 % yield) of the title compound.

¹H-NMR (400 MHz, DMSO-d6) δ [ppm]: 1.725 (0.63), 1.730 (0.56), 1.742 (1.22), 1.747 (1.30), 1.763 (1.31), 1.768 (1.48), 1.786 (0.91), 1.946 (0.62), 1.953 (0.80), 1.968 (1.43), 1.984 (1.10), 1.991 (1.35), 1.995 (1.39), 2.011 (1.15), 2.028 (2.29), 2.047 (3.72), 2.074 (0.74), 2.339 (1.28), 2.355 (1.58), 2.361 (2.12), 2.370 (2.82), 2.377 (2.09), 2.386 (3.28), 2.392 (2.61), 2.408 (1.94), 2.434 (1.24), 2.441 (1.56), 2.450 (2.13), 2.457 (2.85), 2.463 (2.93), 2.473 (3.18), 2.518 (7.89), 2.523 (6.69), 2.536 (2.73), 2.539 (2.87), 2.555 (0.56), 2.754 (0.90), 3.375 (0.83), 3.393 (1.86), 3.415 (11.30), 3.435 (1.18), 3.488 (0.92), 3.506 (1.81), 3.514 (1.18), 3.520 (1.27), 3.531 (1.23), 3.547 (0.59), 4.161 (3.96), 4.178 (7.82), 4.195 (3.80), 6.417 (16.00), 6.712 (6.60), 6.994 (11.00), 7.055 (0.67), 7.063 (5.90), 7.068 (1.95), 7.079 (2.56), 7.085 (12.56), 7.090 (2.24), 7.102 (2.08), 7.107 (6.63), 7.116 (0.64), 7.418 (0.77), 7.426 (6.04), 7.431 (2.51), 7.440 (6.62), 7.448 (6.08), 7.456 (2.26), 7.462 (5.28), 7.469 (0.50), 8.126 (9.78), 8.132 (9.36), 8.601 (10.42), 8.607 (10.75).

### Example 23

### (rac)-2'-[6-amino-5-(trifluoromethyl)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide

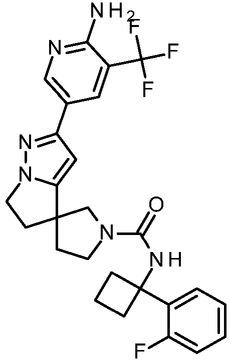

1-(2-Fluorophenyl)cyclobutan-1-amine (27.6 mg, 167 µmol), 1,1'-carbonyldiimidazole (27.0 mg, 167 µmol) and N,N-diisopropylethylamine (150 µL, 830 µmol) were stirred in DMF (1 mL) for 1h at rt, then (rac)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) in DMF (1.5 mL) was added and stirred at rt overnight. Water was added to the mixture and purified by HPLC to afford 6.00 mg (95 % purity, 8 % yield) of the title compound.

¹H-NMR (400 MHz, DMSO-d6) δ [ppm]: 0.949 (2.09), 0.965 (1.93), 1.704 (0.68), 1.712 (0.78), 1.717 (0.95), 1.725 (1.50), 1.731 (1.53), 1.739 (1.61), 1.745 (1.40), 1.753 (1.94), 1.766 (1.09), 1.774 (0.79), 1.787 (0.42), 2.009 (3.17), 2.026 (5.12), 2.036 (5.21), 2.049 (3.24), 2.054 (3.63), 2.068 (1.57), 2.084 (0.62), 2.094 (0.46), 2.323 (0.56), 2.327 (0.78), 2.332 (0.58), 2.457 (1.19), 2.518 (8.72), 2.524 (10.01), 2.547 (3.28), 2.576 (0.82), 2.665 (0.60), 2.669 (0.82), 2.673 (0.60), 3.368 (3.24), 3.397 (10.10), 3.403 (9.09), 3.429 (1.35), 3.470 (1.27), 3.488 (2.33), 3.496 (1.54), 3.502 (1.70), 3.514 (1.54), 3.529 (0.83), 4.151 (5.04), 4.169 (10.49), 4.186 (4.86), 6.389 (16.00), 6.549 (13.29), 6.582 (8.78), 7.025 (2.30), 7.027 (2.59), 7.045 (2.99), 7.048 (3.41), 7.054 (2.45), 7.057 (2.82), 7.064 (3.24), 7.067 (2.99), 7.077 (3.83), 7.083 (7.41), 7.086 (5.55), 7.102 (4.27), 7.105 (3.41), 7.182 (1.58), 7.186 (1.81), 7.195 (1.84), 7.200 (2.73), 7.206 (2.30), 7.214 (2.33), 7.219 (2.42), 7.225 (1.21), 7.233 (1.12), 7.238 (1.02), 7.407 (2.45), 7.412 (2.47), 7.428 (3.84), 7.431 (3.65), 7.448 (2.22), 7.452 (1.94), 7.984 (7.45), 7.989 (7.57), 8.556 (6.98), 8.561 (6.86).

### Example 24

### (rac)-2'-[6-amino-5-(trifluoromethyl)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide

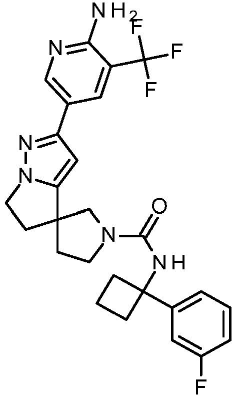

1-(3-Fluorophenyl)cyclobutan-1-amine hydrochloride (33.6 mg, 167 µmol), 1,1'-carbonyldiimidazole (27.0 mg, 167 µmol) and N,N-diisopropylethylamine (150 µL, 830 µmol) were stirred in DMF (1 mL) for 1h at rt, then (rac)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) in DMF (1 mL) was added and stirred 1.5h at 60°C. Water was added to the mixture and purified by HPLC to afford 4.60 mg (96 % purity, 6 % yield) of the title compound.

¹H-NMR (400 MHz, DMSO-d6) δ [ppm]: 0.945 (2.14), 0.961 (2.04), 1.753 (0.63), 1.760 (0.62), 1.776 (1.24), 1.781 (1.28), 1.798 (1.32), 1.803 (1.53), 1.820 (0.93), 1.959 (0.60), 1.966 (0.77), 1.981 (1.45), 1.997 (1.07), 2.004 (1.38), 2.009 (1.43), 2.024 (1.18), 2.032 (1.18), 2.041 (1.77), 2.055 (2.85), 2.068 (3.17), 2.084 (1.64), 2.097 (0.59), 2.323 (0.58), 2.327 (0.81), 2.332 (0.60), 2.348 (0.98), 2.372 (2.15), 2.380 (2.68), 2.388 (2.09), 2.396 (3.24), 2.402 (2.73), 2.418 (2.12), 2.430 (1.87), 2.453 (3.13), 2.460 (2.26), 2.470 (2.50), 2.476 (2.49), 2.518 (5.46), 2.523 (3.29), 2.532 (5.37), 2.536 (5.37), 2.551 (3.64), 2.665 (0.60), 2.669 (0.84), 2.674 (0.58), 3.389 (0.88), 3.406 (2.63), 3.431 (6.90), 3.442 (5.07), 3.468 (1.18), 3.516 (0.88), 3.533 (1.69), 3.548 (1.22), 3.558 (1.21), 3.574 (0.60), 4.169 (4.12), 4.187 (7.49), 4.204 (3.99), 6.421 (16.00), 6.551 (10.69), 6.763 (7.02), 6.949 (1.18), 6.952 (1.28), 6.955 (1.49), 6.958 (1.47), 6.971 (2.56), 6.975 (2.46), 6.978 (2.78), 6.991 (1.39), 6.993 (1.49), 6.998 (1.62), 7.000 (1.55), 7.163 (2.04), 7.169 (2.63), 7.173 (2.23), 7.190 (2.12), 7.195 (2.67), 7.200 (2.16), 7.259 (2.58), 7.279 (5.25), 7.298 (3.05), 7.313 (3.16), 7.317 (3.96), 7.333 (3.82), 7.337 (1.62), 7.352 (1.45), 7.994 (6.07), 7.999 (6.17), 8.570 (5.65), 8.575 (5.62).

### Example 25

### (rac)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide

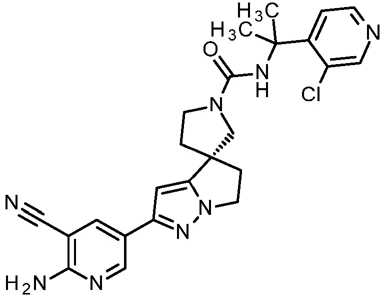

To half of the reaction mixture of 3-chloro-4-(2-isocyanatopropan-2-yl)pyridine (80.0 mg, 407 µmol) was added N,N-diisopropylethylamine (180 µL, 1.0 mmol) and (rac)-2-amino-5-[5',6'-dihydrospiro[pyrrolidine-3,4'-pyrrolo[1,2-b]pyrazol]-2'-yl]pyridine-3-carbonitrile hydrochloride (107 mg, 339 µmol). The mixture was stirred at rt overnight under nitrogen atmosphere and then 5h at 60°C. Saturated potassium carbonate solution was added and stirred for 5 min. The layers were separated and the aqueous phase was extracted three times with dichloromethane (+10 % methanol). The combined organic phases were concentrated to give 190 mg of crude material which was purified by flash chromatography to afford 78.0 mg (90 % purity, 43 % yield) of the title compound.

1H-NMR (400 MHz, DMSO-d6) δ [ppm]: 1.231 (0.29), 1.653 (16.00), 2.056 (1.38), 2.074 (0.69), 2.457 (0.16), 2.518 (4.74), 2.522 (2.99), 2.534 (1.35), 2.549 (0.46), 2.567 (0.22), 3.381 (0.64), 3.407 (2.04), 3.421 (2.58), 3.446 (0.67), 3.478 (0.37), 3.495 (0.66), 3.511 (0.50), 3.520 (0.44), 3.537 (0.24), 4.162 (1.48), 4.180 (3.14), 4.198 (1.50), 6.331 (2.87), 6.431 (5.40), 7.003 (4.16), 7.426 (2.40), 7.439 (2.44), 8.127 (3.11), 8.132 (3.18), 8.390 (3.32), 8.403 (3.24), 8.419 (6.35), 8.600 (3.47), 8.606 (3.44).

### Example 26

### (rac)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide

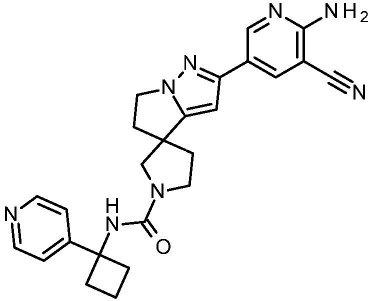

1-(Pyridin-4-yl)cyclobutan-1-amine (43.2 mg, 292 µmol), 1,1'-carbonyldiimidazole (47.3 mg, 292 µmol) and N,N-diisopropylethylamine (170 µL, 970 µmol) were stirred in DMF (1.0 mL) for 1h at rt, then (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) in DMF (1.0 mL) was added and stirred 3h at 60°C. Water was added to the mixture and purified by HPLC to afford 39.0 mg (99 % purity, 35 % yield) of the title compound.

¹H-NMR (400 MHz, DMSO-d6) δ [ppm]: 1.803 (0.71), 1.826 (1.42), 1.849 (1.66), 1.867 (1.06), 1.889 (0.42), 2.001 (1.56), 2.015 (1.51), 2.028 (1.69), 2.051 (2.92), 2.067 (4.33), 2.074 (5.11), 2.347 (1.19), 2.377 (3.02), 2.393 (3.50), 2.415 (3.32), 2.434 (2.60), 2.452 (3.19), 2.518 (16.00), 2.523 (11.32), 2.535 (7.13), 2.555 (4.01), 3.440 (9.76), 3.534 (1.75), 4.170 (4.20), 4.188 (8.19), 4.205 (4.19), 6.440 (14.09), 6.884 (6.89), 7.002 (11.81), 7.411 (10.35), 7.415 (6.86), 7.422 (7.07), 7.426 (10.58), 8.138 (9.27), 8.144 (9.33), 8.472 (12.42), 8.475 (7.27), 8.483 (7.57), 8.487 (11.45), 8.608 (10.05), 8.614 (10.13).

### Example 27

### (rac)-2-(6-amino-5-cyanopyridin-3-yl)-N-(1-phenylcyclobutyl)-6,7-dihydrospiro[pyrazolo[5,1-c][1,4]oxazine-4,3'-pyrrolidine]-1'-carboxamide

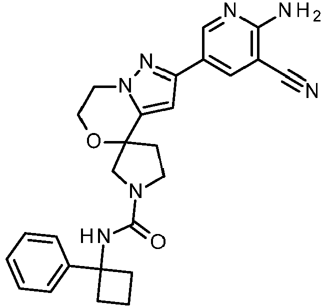

1-Phenylcyclobutan-1-amine hydrochloride (47.0 mg, 256 µmol), 1,1'-carbonyldiimidazole (41.5 mg, 256 µmol) and N,N-diisopropylethylamine (150 µL, 850 µmol) were stirred in DMF (1.0 mL) for 1h at rt, then (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) in DMF (1.0 mL) was added and stirred overnight at rt. Water was added to the mixture and purified by HPLC to afford 26.0 mg (98 % purity, 25 % yield) of the title compound.

¹H-NMR (400 MHz, DMSO-d6) δ [ppm]: 1.760 (0.92), 1.765 (0.93), 1.782 (0.97), 1.788 (1.09), 1.805 (0.72), 1.978 (1.07), 2.000 (1.04), 2.028 (1.81), 2.050 (2.81), 2.064 (1.46), 2.336 (1.31), 2.356 (0.74), 2.385 (1.72), 2.401 (2.06), 2.423 (1.31), 2.518 (16.00), 2.522 (12.73), 2.543 (2.70), 2.678 (1.24), 2.762 (1.55), 3.290 (0.53), 3.348 (1.59), 3.370 (0.48), 3.422 (6.56), 3.494 (0.64), 3.510 (1.30), 3.535 (0.89), 4.162 (2.84), 4.180 (5.32), 4.197 (2.79), 6.392 (10.48), 6.697 (4.90), 6.999 (7.83), 7.138 (1.50), 7.156 (3.69), 7.171 (1.47), 7.174 (2.58), 7.178 (1.43), 7.263 (4.15), 7.283 (6.78), 7.301 (3.86), 7.418 (5.87), 7.421 (6.34), 7.439 (5.09), 7.442 (3.90), 8.111 (6.98), 8.117 (6.72), 8.596 (7.41), 8.602 (7.88).

### Example 28

### (rac)-2-(6-amino-5-cyanopyridin-3-yl)-N-[2-(4-fluorophenyl)propan-2-yl]-6,7-dihydrospiro[pyrazolo[5,1-c][1,4]oxazine-4,3'-pyrrolidine]-1'-carboxamide

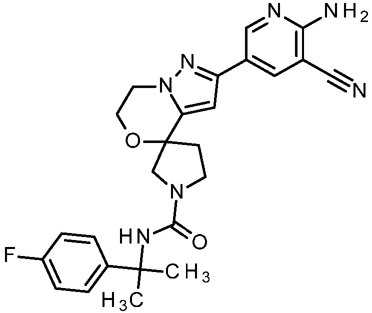

2-(4-Fluorophenyl)propan-2-amine hydrochloride (48.6 mg, 256 µmol), 1,1'-carbonyldiimidazole (41.5 mg, 256 µmol) and N,N-diisopropylethylamine (150 µL, 850 µmol) were stirred in DMF (1.0 mL) for 1h at rt, then (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) in DMF (1.0 mL) was added and stirred overnight at rt. Water was added to the mixture and purified by HPLC to afford 35.0 mg (98 % purity, 34 % yield) of the title compound.

¹H-NMR (400 MHz, DMSO-d6) δ [ppm]: 1.549 (16.00), 1.555 (15.81), 2.074 (1.15), 2.178 (0.68), 2.186 (0.71), 2.212 (0.97), 2.236 (0.50), 2.311 (0.80), 2.322 (1.36), 2.326 (1.77), 2.331 (1.22), 2.336 (0.69), 2.342 (0.62), 2.359 (0.53), 2.518 (3.21), 2.522 (2.07), 2.664 (0.62), 2.668 (0.84), 2.673 (0.62), 3.361 (0.46), 3.385 (0.91), 3.403 (0.92), 3.429 (0.49), 3.449 (1.29), 3.478 (1.48), 3.578 (0.73), 3.599 (1.31), 3.622 (0.60), 3.781 (1.58), 3.809 (1.35), 4.068 (0.47), 4.082 (1.01), 4.099 (1.56), 4.124 (4.56), 4.134 (4.16), 4.150 (1.22), 6.102 (4.92), 6.714 (10.77), 7.046 (9.22), 7.063 (1.84), 7.069 (8.08), 7.074 (1.56), 7.086 (1.29), 7.091 (4.09), 7.099 (0.45), 7.343 (0.42), 7.351 (3.77), 7.357 (1.62), 7.365 (4.14), 7.373 (3.77), 7.382 (1.37), 7.387 (3.39), 8.118 (6.29), 8.124 (6.05), 8.602 (7.06), 8.607 (7.02).

### Example 29

### (rac)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide

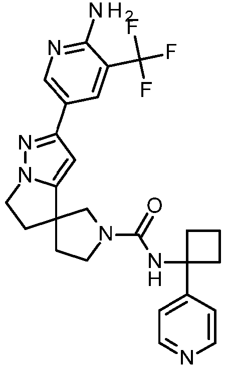

1-(Pyrdin-4-yl)cyclobutan-1-amine hydrochloride (36.9 mg, 167 µmol), 1,1'-carbonyldiimidazole (27.0 mg, 167 µmol) and N,N-diisopropylethylamine (150 µL, 830 µmol) were stirred in DMF (1.5 mL) for 1h at rt, then (rac)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) in DMF (1.5 mL) was added and stirred 1.5h at 60°C. Water was added to the mixture and purified by HPLC to afford 19.4 mg (95 % purity, 27 % yield) of the title compound.

¹H-NMR (400 MHz, DMSO-d6) δ [ppm]: 0.840 (0.44), 0.858 (0.44), 0.919 (0.61), 0.934 (4.85), 0.950 (4.81), 1.237 (0.44), 1.803 (0.78), 1.825 (1.59), 1.848 (2.00), 1.866 (1.22), 1.888 (0.54), 2.000 (1.76), 2.023 (1.80), 2.050 (2.81), 2.065 (3.93), 2.078 (4.07), 2.323 (1.46), 2.327 (2.07), 2.332 (1.49), 2.344 (1.36), 2.375 (3.56), 2.391 (4.34), 2.413 (4.00), 2.444 (3.76), 2.518 (10.68), 2.523 (9.05), 2.537 (6.64), 2.543 (6.54), 2.558 (4.00), 2.665 (1.49), 2.669 (2.03), 2.673 (1.46), 3.445 (10.37), 3.549 (2.00), 4.173 (4.92), 4.190 (9.39), 4.208 (4.75), 6.458 (16.00), 6.556 (13.08), 6.872 (8.03), 7.390 (12.75), 7.395 (8.24), 7.402 (8.41), 7.406 (12.61), 8.009 (7.36), 8.014 (7.32), 8.458 (14.58), 8.461 (8.71), 8.469 (8.61), 8.473 (13.69), 8.584 (6.98), 8.588 (6.78).

### Example 30

### (rac)-2-[6-amino-5-(trifluoromethyl)pyridin-3-yl]-N-[1-(3-fluorophenyl)cyclobutyl]-6,7-dihydrospiro[pyrazolo[5,1-c][1,4]oxazine-4,3'-pyrrolidine]-1'-carboxamide

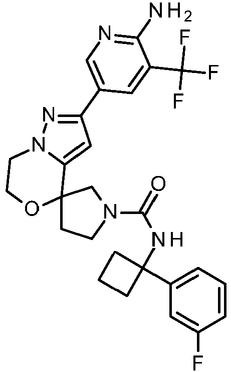

1-(3-Fluorophenyl)cyclobutan-1-amine hydrochloride (64.4 mg, 319 µmol), 1,1'-carbonyldiimidazole (51.8 mg, 319 µmol) and N,N-diisopropylethylamine (280 µL, 1.6 mmol) were stirred in DMF (1 mL) for 1h at rt, then (rac)-5-[6,7-dihydrospiro[pyrazolo[5,1-c][1,4]oxazine-4,3'-pyrrolidin]-2-yl]-3-(trifluoromethyl)pyridin-2-amine hydrochloride (100 mg, 266 µmol) in DMF (1 mL) was added and stirred overnight at rt. Water was added to the mixture and purified by HPLC to afford 73.6 mg (90 % purity, 47 % yield) of the title compound.

¹H-NMR (400 MHz, DMSO-d6) δ [ppm]: 0.917 (1.89), 0.931 (16.00), 0.934 (5.08), 0.948 (15.70), 0.952 (2.62), 1.004 (0.61), 1.021 (0.61), 1.401 (1.10), 1.425 (0.91), 1.753 (0.85), 1.759 (0.67), 1.770 (1.43), 1.776 (1.55), 1.787 (1.13), 1.793 (1.55), 1.798 (1.79), 1.809 (0.76), 1.815 (1.16), 1.836 (0.46), 1.966 (0.73), 1.971 (0.94), 1.988 (1.67), 1.993 (1.34), 2.004 (1.16), 2.010 (1.55), 2.015 (1.49), 2.026 (0.82), 2.031 (0.79), 2.038 (0.79), 2.182 (0.55), 2.214 (1.25), 2.240 (1.67), 2.264 (0.88), 2.322 (2.34), 2.327 (2.46), 2.332 (2.46), 2.336 (2.31), 2.347 (1.73), 2.358 (1.83), 2.364 (2.28), 2.370 (2.65), 2.380 (2.89), 2.387 (3.25), 2.396 (3.22), 2.404 (4.59), 2.410 (2.83), 2.422 (5.05), 2.440 (4.08), 2.446 (4.29), 2.463 (3.25), 2.471 (2.68), 2.478 (2.68), 2.518 (5.08), 2.523 (3.22), 2.665 (0.85), 2.669 (1.19), 2.673 (0.88), 2.940 (1.00), 2.957 (1.31), 2.973 (0.97), 3.370 (1.73), 3.389 (1.64), 3.413 (0.79), 3.456 (1.79), 3.485 (2.07), 3.562 (1.37), 3.584 (2.40), 3.608 (1.10), 3.762 (2.98), 3.791 (2.49), 4.056 (0.94), 4.070 (1.86), 4.086 (2.68), 4.112 (3.68), 4.121 (5.78), 4.135 (9.46), 6.598 (12.14), 6.753 (14.14), 6.769 (7.39), 6.965 (1.46), 6.967 (1.61), 6.971 (1.86), 6.974 (1.79), 6.987 (3.10), 6.993 (3.44), 7.007 (1.67), 7.009 (1.73), 7.014 (1.92), 7.015 (1.76), 7.164 (2.43), 7.169 (3.13), 7.174 (2.68), 7.191 (2.46), 7.195 (3.25), 7.201 (2.52), 7.253 (3.44), 7.273 (5.54), 7.311 (3.29), 7.326 (3.47), 7.331 (4.65), 7.347 (4.41), 7.350 (2.13), 7.366 (1.83), 8.004 (7.03), 8.009 (7.15), 8.543 (0.49), 8.572 (6.66), 8.576 (6.57).

### Example 31

### 2'-[6-amino-5-(trifluoromethyl)pyridin-3-yl]-N-{(1R)-1-[3-(trifluoromethyl)phenyl]ethyl}-5',6'-dihydrospiro[pyrrolidine-3,4'-pyrrolo[1,2-b]pyrazole]-1-carboxamide (mixture of stereoisomers)

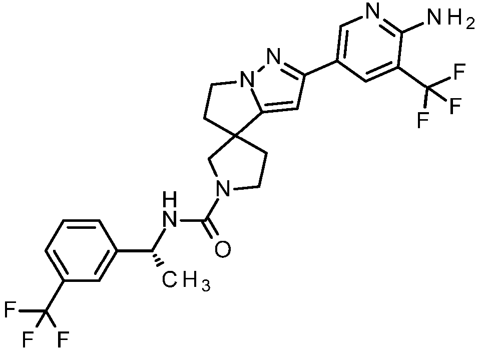

(1R)-1-[3-(trifluoromethyl)phenyl]ethan-1-amine (56.8 mg, 300 µmol), 1,1'-carbonyldiimidazole (48.7 mg, 300 µmol) and N,N-diisopropylethylamine (170 µL, 1.0 mmol) were stirred in DMF (5.0 mL) for 1 h at rt. (rac)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 90 % purity, 250 µmol) was added and the mixture was stirred for 2 h at 60°C. Water was added and the mixture was purified by preparative HPLC to give 38.0 mg (98 % purity, 28 % yield) of the title compound.
LC-MS (method 1): Rt = 1.19 min; MS (ESlpos): m/z = 539 [M+H]⁺
¹H-NMR (400MHz, DMSO-d₆): δ [ppm]= 1.39 (dd, 3H), 2.08 (br d, 2H), 2.52 - 2.57 (m, 2H), 3.41 - 3.49 (m, 3H), 3.50 - 3.62 (m, 1H), 4.19 (t, 2H), 4.92 (br t, 1H), 6.40 - 6.47 (m, 1H), 6.52 - 6.58 (m, 2H), 6.63 (dd, 1H), 7.49 - 7.59 (m, 2H), 7.61 - 7.76 (m, 2H), 8.00 (s, 1H), 8.56 - 8.60 (m, 1H).

### Example 32

### (rac)-2'-[6-amino-5-(trifluoromethyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide

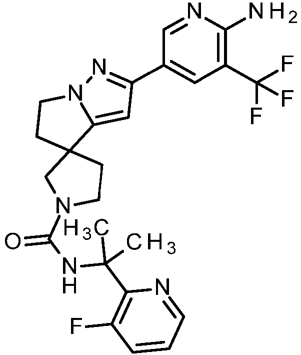

Example 32 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(3-fluoropyridin-2-yl)propan-2-amine (48.2 mg, 313 µmol).
LC-MS (method 1): Rt = 1.09 min; MS (ESlpos): m/z = 504 [M+H]⁺
¹H-NMR (400MHz, DMSO-d6): δ [ppm]= 1.63 (s, 6H), 2.01 - 2.14 (m, 2H), 2.52 - 2.57 (m, 2H), 3.38 - 3.47 (m, 3H), 3.49 - 3.58 (m, 1H), 4.19 (t, 2H), 6.45 - 6.50 (m, 2H), 6.55 (s, 2H), 7.33 (ddd, 1H), 7.55 (ddd, 1H), 8.01 (d, 1H), 8.31 (dt, 1H), 8.58 (d, 1H).

### Example 33

### (rac)-2'-[6-amino-5-(trifluoromethyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide

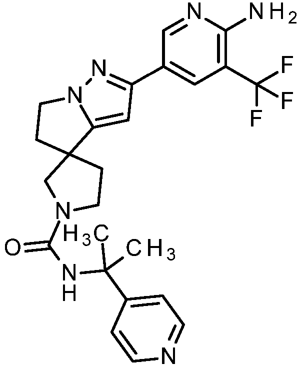

Example 33 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(pyridin-4-yl)propan-2-amine (42.6 mg, 313 µmol).
LC-MS (method 1): Rt = 0.96 min; MS (ESlpos): m/z = 486 [M+H]⁺
¹H-NMR (400MHz, DMSO-d6): δ [ppm]= 1.53 (s, 3H), 1.54 (s, 3H), 2.00 - 2.14 (m, 2H), 2.52 - 2.58 (m, 2H), 3.39 - 3.50 (m, 3H), 3.53 - 3.62 (m, 1H), 4.20 (t, 2H), 6.22 (s, 1H), 6.47 (s, 1H), 6.56 (s, 2H), 7.30 - 7.33 (m, 2H), 8.02 (d, 1H), 8.40 - 8.44 (m, 2H), 8.60 (d, 1H).

### Example 34

### (rac)-2'-[6-amino-5-(trifluoromethyl)pyridin-3-yl]-N-[2-(2-fluorophenyl)propan-2-yl]-5',6'-dihydrospiro[pyrrolidine-3,4'-pyrrolo[1,2-b]pyrazole]-1-carboxamide

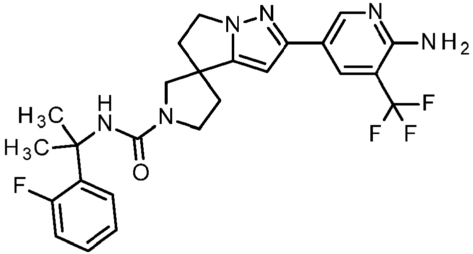

Example 34 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(2-fluorophenyl)propan-2-amine hydrochloride (43.5 mg, 229 µmol).
LC-MS (method 2): Rt = 1.14 min; MS (ESlpos): m/z = 504 [M+H]⁺
¹H-NMR (400MHz, DMSO-d₆): δ [ppm]= 1.63 (s, 6H), 1.98 - 2.16 (m, 2H), 2.52 - 2.55 (m, 2H), 3.44 (br d, 2H), 3.51 - 3.58 (m, 1H), 4.19 (t, 2H), 6.09 (s, 1H), 6.44 (s, 1H), 6.53 - 6.58 (m, 2H), 6.95 - 7.11 (m, 3H), 7.17 - 7.25 (m, 1H), 7.29 - 7.35 (m, 1H), 8.01 (d, 1H), 8.59 (d, 1H).

### Example 35

### 2'-[6-amino-5-(trifluoromethyl)pyridin-3-yl]-N-[(1R)-1-(3-fluorophenyl)ethyl]-5',6'-dihydrospiro[pyrrolidine-3,4'-pyrrolo[1,2-b]pyrazole]-1-carboxamide (mixture of stereoisomers)

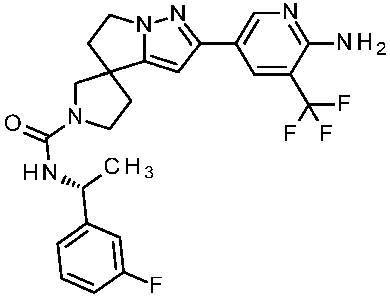

Example 35 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1R)-1-(3-fluorophenyl)ethan-1-amine (31.9 mg, 229 µmol).
LC-MS (method 2): Rt = 1.08 min; MS (ESlpos): m/z = 489 [M+H]⁺
¹H NMR (400 MHz, DMSO-*d*₆) δ ppm 1.36 (dd, 3 H) 2.02 - 2.12 (m, 2 H) 2.52 - 2.60 (m, 2 H) 3.42 - 3.50 (m, 3 H) 3.51 - 3.63 (m, 1 H) 4.19 (t, 2 H) 4.85 (quin, 1 H) 6.43 - 6.47 (m, 1 H) 6.49 - 6.58 (m, 3 H) 6.97 - 7.04 (m, 1 H) 7.13 - 7.20 (m, 2 H) 7.28 - 7.36 (m, 1 H) 8.01 (d, 1 H) 8.58 (s, 1 H)

### Example 36

### (rac)-2'-[6-amino-5-(trifluoromethyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide

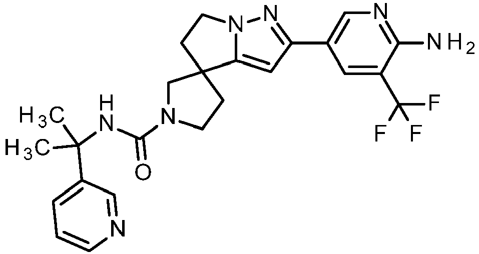

Example 36 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(pyridin-3-yl)propan-2-amine (31.2 mg, 229 µmol).
LC-MS (Method 2): Rt = 0.73 min; MS (ESlpos): m/z = 486 [M+H]⁺
¹H NMR (400 MHz, DMSO-*d*₆) δ ppm 1.56 - 1.61 (m, 5 H) 2.00 - 2.14 (m, 2 H) 2.54 (t, 2 H) 3.24 - 3.41 (m, 1 H) 3.45 - 3.51 (m, 2 H) 3.53 - 3.65 (m, 1 H) 4.19 (t, 2 H) 6.19 (s, 1 H) 6.45 (s, 1 H) 6.56 (s, 2 H) 7.27 (dd, 1 H) 7.68 - 7.74 (m, 1 H) 8.03 (d, 1 H) 8.15 (s, 1 H) 8.35 (br d, 1 H) 8.57 - 8.62 (m, 2 H)

### Example 37

### (rac)-2'-[6-amino-5-(trifluoromethyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide

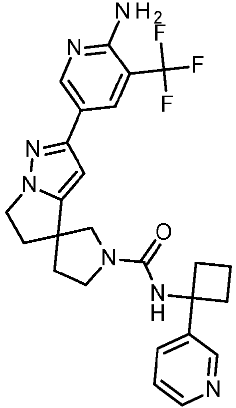

Example 37 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) and 1-(pyridin-3-yl)cyclobutan-1-amine (24.7 mg, 167 µmol).
LC-MS (method 1): Rt = 0.97 min; MS (ESlpos): m/z = 499 [M+H]⁺
¹H NMR (400 MHz, DMSO-*d*₆) δ ppm 1.74 - 1.86 (m, 1 H) 1.96 - 2.11 (m, 3 H) 2.40 - 2.47 (m, 3 H) 2.52 - 2.58 (m, 3 H) 3.38 - 3.49 (m, 3 H) 3.54 (dt, 1 H) 4.18 (t, 2 H) 6.43 (s, 1 H) 6.55 (s, 2 H) 6.83 (s, 1 H) 7.28 - 7.33 (m, 1 H) 7.80 (dt, 1 H) 8.01 (d, 1 H) 8.37 (dd, 1 H) 8.58 (d, 1 H) 8.64 (d, 1 H)

### Example 38

### 2'-[6-amino-5-(trifluoromethyl)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide (mixture of stereoisomers)

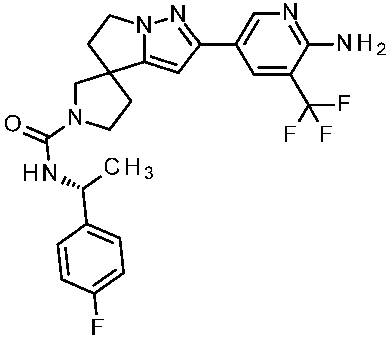

Example 38 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1R)-1-(4-fluorophenyl)ethan-1-amine (31.9 mg, 229 µmol).
LC-MS (method 2): Rt = 1.08 min; MS (ESlpos): m/z = 489 [M+H]⁺
¹H NMR (400 MHz, DMSO-*d*₆) δ ppm 1.35 (dd, 3 H) 2.02 - 2.12 (m, 2 H) 2.52 - 2.57 (m, 2 H) 3.41 - 3.50 (m, 3 H) 3.50 - 3.61 (m, 1 H) 4.18 (t, 2 H) 4.84 (br t, 1 H) 6.44 - 6.51 (m, 2 H) 6.54 (s, 2 H) 7.06 - 7.14 (m, 2 H) 7.32 - 7.41 (m, 2 H) 8.01 (d, 1 H) 8.59 (s, 1 H)

### Example 39

### 2'-[6-amino-5-(trifluoromethyl)pyridin-3-yl]-N-[(1R)-1-phenylpropyl]-5',6'-dihydrospiro[pyrrolidine-3,4'-pyrrolo[1,2-b]pyrazole]-1-carboxamide (mixture of stereoisomers)

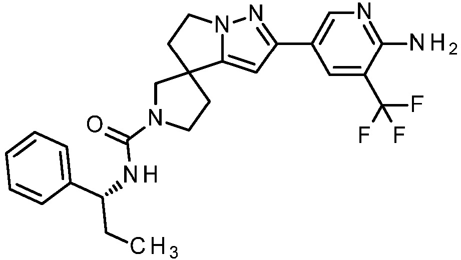

Example 39 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1R)-1-phenylpropan-1-amine (31.0 mg, 229 µmol).
LC-MS (Method 2): Rt = 1.12 min; MS (ESlpos): m/z = 485 [M+H]⁺
¹H NMR (400 MHz, DMSO-*d*₆) δ ppm 0.80 - 0.92 (m, 3 H) 1.62 - 1.79 (m, 2 H) 2.01 - 2.12 (m, 2 H) 2.52 - 2.57 (m, 2 H) 3.38 - 3.49 (m, 3 H) 3.50 - 3.65 (m, 1 H) 4.18 (t, 2 H) 4.54 - 4.61 (m, 1 H) 6.37 - 6.48 (m, 2 H) 6.55 (s, 2 H) 7.13 - 7.23 (m, 1 H) 7.24 - 7.36 (m, 4 H) 7.98 - 8.01 (m, 1 H) 8.57 (s, 1 H)

### Example 40

### 2'-[6-amino-5-(trifluoromethyl)pyridin-3-yl]-N-{(1S)-1-[3-(trifluoromethyl)phenyl]ethyl}-5',6'-dihydrospiro[pyrrolidine-3,4'-pyrrolo[1,2-b]pyrazole]-1-carboxamide (mixture of stereoisomers)

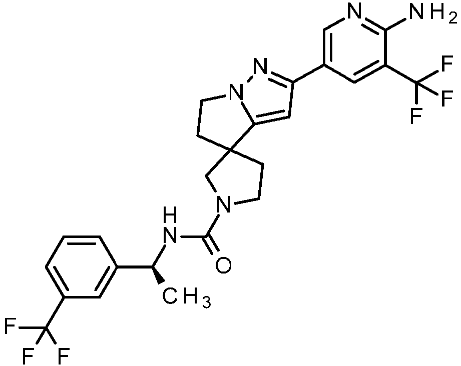

Example 40 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 90 % purity, 250 µmol) and (1S)-1-[3-(trifluoromethyl)phenyl]ethan-1-amine (56.8 mg, 300 µmol).
LC-MS (method 1): Rt = 1.19 min; MS (ESlpos): m/z = 539 [M+H]⁺
¹H NMR (400 MHz, DMSO-*d*₆) δ ppm 1.39 (dd, 3 H) 2.08 (br d, 2 H) 2.52 - 2.59 (m, 2 H) 3.41 - 3.51 (m, 3 H) 3.51 - 3.63 (m, 1 H) 4.19 (t, 2 H) 4.88 - 4.96 (m, 1 H) 6.41 - 6.46 (m, 1 H) 6.55 (s, 2 H) 6.63 (dd, 1 H) 7.50 - 7.59 (m, 2 H) 7.62 - 7.73 (m, 2 H) 8.00 (s, 1 H) 8.55 - 8.62 (m, 1 H)

### Example 41

### 2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide (mixture of stereoisomers)

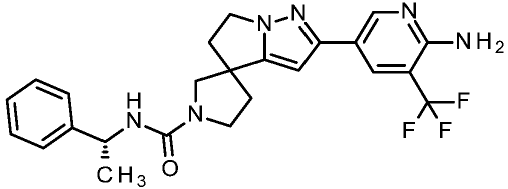

Example 41 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1R)-1-phenylethan-1-amine (27.8 mg, 229 µmol).
LC-MS (Method 2): Rt = 1.06 min; MS (ESlpos): m/z = 471 [M+H]⁺
¹H NMR (400 MHz, DMSO-*d*₆) δ ppm 1.37 (dd, 3 H) 2.02 - 2.12 (m, 2 H) 2.53 - 2.57 (m, 2 H) 3.41 - 3.51 (m, 3 H) 3.52 - 3.65 (m, 1 H) 4.18 (t, 2 H) 4.84 (br t, 1 H) 6.43 - 6.50 (m, 2 H) 6.55 (s, 2 H) 7.14 - 7.21 (m, 1 H) 7.24 - 7.40 (m, 4 H) 8.01 (d, 1 H) 8.58 (br s, 1 H)

### Example 42

### (rac)-2'-[6-amino-5-(trifluoromethyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide

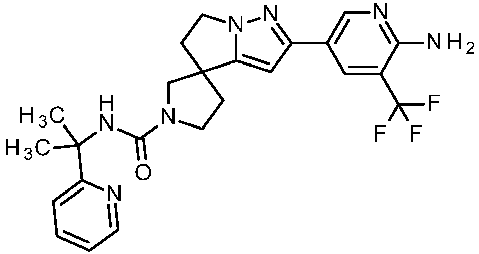

Example 42 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(pyridin-2-yl)propan-2-amine (31.2 mg, 229 µmol).
LC-MS (method 2): Rt = 0.72 min; MS (ESlpos): m/z = 486 [M+H]⁺
¹H NMR (400 MHz, DMSO-*d*₆) δ ppm 1.59 (s, 6 H) 2.03 - 2.15 (m, 2 H) 2.52 - 2.59 (m, 2 H) 3.42 - 3.50 (m, 3 H) 3.52 - 3.66 (m, 1 H) 4.20 (t, 2 H) 6.43 (s, 1 H) 6.49 (s, 1 H) 6.55 (s, 2 H) 7.18 (ddd, 1 H) 7.46 (d, 1 H) 7.70 (td, 1 H) 8.02 (d, 1 H) 8.46 (d, 1 H) 8.60 (d, 1 H)

### Example 43

### 2'-[6-amino-5-(trifluoromethyl)pyridin-3-yl]-N-[(1R)-1-(2-cyanophenyl)ethyl]-5',6'-dihydrospiro[pyrrolidine-3,4'-pyrrolo[1,2-b]pyrazole]-1-carboxamide (mixture of stereoisomers)

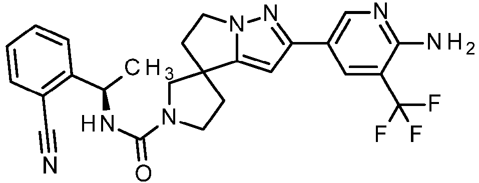

Example 43 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-[(1R)-1-aminoethyl]benzonitrile (33.5 mg, 229 µmol).
LC-MS (Method 2): Rt = 1.01 min; MS (ESlpos): m/z = 496 [M+H]⁺
¹H NMR (400 MHz, DMSO-*d*₆) δ ppm 1.32 - 1.44 (m, 3 H) 2.01 - 2.14 (m, 2 H) 3.39 - 3.51 (m, 3 H) 3.52 - 3.65 (m, 1 H) 4.19 (t, 2 H) 5.02 - 5.10 (m, 1 H) 6.42 - 6.49 (m, 1 H) 6.56 (br s, 2 H) 6.76 (dd, 1 H) 7.34 - 7.43 (m, 1 H) 7.59 - 7.77 (m, 3 H) 8.01 (t, 1 H) 8.58 (s, 1 H)

### Example 44

### 2'-[6-amino-5-(trifluoromethyl)pyridin-3-yl]-N-[1-(1,3,5-trimethyl-1H-pyrazol-4-yl)ethyl]-5',6'-dihydrospiro[pyrrolidine-3,4'-pyrrolo[1,2-b]pyrazole]-1-carboxamide (diastereomeric mixture)

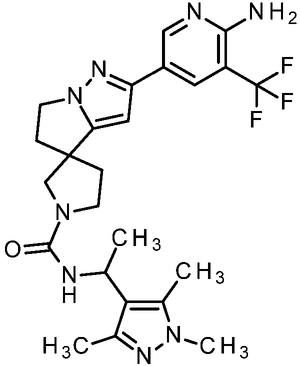

Example 44 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (*rac*)-1-(1,3,5-trimethyl-1H-pyrazol-4-yl)ethan-1-amine (47.9 mg, 313 µmol).
LC-MS (method 1): Rt = 0.97 min; MS (ESlpos): m/z = 503 [M+H]⁺
¹H-NMR (400MHz, DMSO-d6): δ [ppm]= 1.33 and 1.34 (2d, 3H), 1.99 - 2.08 (m, 2H), 2.11 and 2.13 (2s, 3H), 2.18 and 2.19 (2s, 3H), 3.35 - 3.59 (m, 7H), 4.17 (t, 2H), 4.70 (quin, 1H), 6.08 and 6.08 (2d, 1H), 6.44 and 6.44 (2s, 1H), 6.54 (s, 2H), 8.00 (s, 1H), 8.57 and 8.58 (2d, 1H).

### Example 45

### 2'-[6-amino-5-(trifluoromethyl)pyridin-3-yl]-N-[(1R)-2,3-dihydro-1H-inden-1-yl]-5',6'-dihydrospiro[pyrrolidine-3,4'-pyrrolo[1,2-b]pyrazole]-1-carboxamide (mixture of stereoisomers)

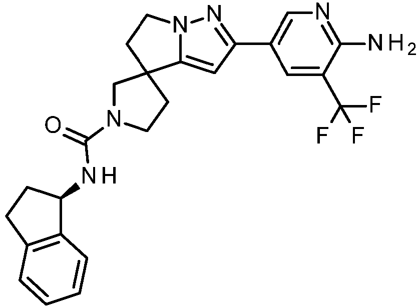

Example 45 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1R)-2,3-dihydro-1H-inden-1-amine (30.5 mg, 229 µmol).
LC-MS (method 2): Rt = 1.10 min; MS (ESlpos): m/z = 484 [M+H]⁺
¹H NMR (400 MHz, DMSO-*d*₆) δ ppm 1.79 - 1.91 (m, 1 H) 1.98 - 2.13 (m, 2 H) 2.33 - 2.45 (m, 1 H) 2.52 - 2.58 (m, 2 H) 2.74 - 2.82 (m, 1 H) 3.42 - 3.53 (m, 3 H) 3.53 - 3.65 (m, 1 H) 4.19 (t, 2 H) 5.22 (q, 1 H) 6.45 - 6.58 (m, 4 H) 7.12 - 7.27 (m, 4 H) 7.95 (s, 1 H) 8.00 - 8.04 (m, 1 H) 8.60 (s, 1 H)

### Example 46

### 2'-[6-amino-5-(trifluoromethyl)pyridin-3-yl]-N-[(1R)-1-(2-cyanopyridin-4-yl)ethyl]-5',6'-dihydrospiro[pyrrolidine-3,4'-pyrrolo[1,2-b]pyrazole]-1-carboxamide (mixture of stereoisomers)

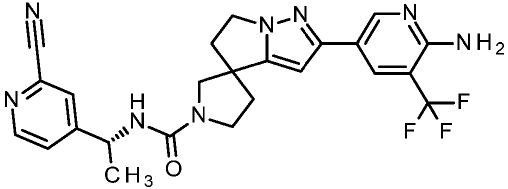

Example 46 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 4-[(1R)-1-aminoethyl]pyridine-2-carbonitrile (33.7 mg, 229 µmol).
LC-MS (Method 2): Rt = 0.94 min; MS (ESlpos): m/z = 497 [M+H]⁺
¹H NMR (400 MHz, DMSO-*d*₆) δ ppm 1.02 - 1.08 (m, 1 H) 1.31 - 1.44 (m, 3 H) 2.02 - 2.14 (m, 2 H) 2.52 - 2.58 (m, 2 H) 3.36 - 3.52 (m, 3 H) 3.53 (br d, 1 H) 4.17 - 4.22 (m, 1 H) 4.82 - 4.92 (m, 1 H) 6.49 (d, 1 H) 6.54 (s, 2 H) 6.68 (dd, 1 H) 7.62 - 7.72 (m, 1 H) 7.99 - 8.03 (m, 2 H) 8.59 (d, 1 H) 8.64 - 8.70 (m, 1 H)

### Example 47

### 2'-[6-amino-5-(trifluoromethyl)pyridin-3-yl]-N-[1-(1-ethyl-3,5-dimethyl-1H-pyrazol-4-yl)ethyl]-5',6'-dihydrospiro[pyrrolidine-3,4'-pyrrolo[1,2-b]pyrazole]-1-carboxamide (diastereometric mixture)

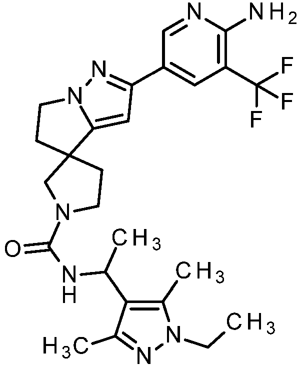

Example 47 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (*rac*)-1-(1-ethyl-3,5-dimethyl-1H-pyrazol-4-yl)ethan-1-amine (52.3 mg, 313 µmol).
LC-MS (method 1): Rt = 0.99 min; MS (ESlpos): m/z = 517 [M+H]⁺
¹H-NMR (400 MHz, DMSO-d6) δ [ppm]: 1.194 (3.80), 1.204 (4.08), 1.212 (9.50), 1.223 (9.14), 1.230 (4.12), 1.240 (3.95), 1.324 (5.19), 1.334 (5.68), 1.342 (5.58), 1.352 (5.45), 2.017 (1.28), 2.031 (1.98), 2.042 (2.29), 2.059 (1.01), 2.124 (15.13), 2.142 (15.21), 2.190 (16.00), 2.206 (15.73), 2.322 (0.44), 2.327 (0.61), 2.332 (0.45), 2.523 (2.49), 2.532 (2.39), 2.664 (0.42), 2.669 (0.62), 2.673 (0.45), 3.354 (0.64), 3.366 (1.46), 3.372 (0.96), 3.378 (1.38), 3.391 (2.62), 3.403 (2.64), 3.423 (2.62), 3.431 (2.30), 3.447 (1.04), 3.457 (1.11), 3.472 (0.42), 3.490 (0.71), 3.504 (0.81), 3.521 (0.81), 3.529 (0.62), 3.546 (0.50), 3.855 (0.94), 3.872 (3.28), 3.886 (3.50), 3.890 (3.46), 3.903 (3.21), 3.922 (0.91), 4.155 (2.59), 4.173 (4.99), 4.190 (2.52), 4.698 (1.56), 4.715 (2.34), 4.732 (1.53), 6.063 (1.80), 6.076 (1.70), 6.080 (1.68), 6.440 (4.08), 6.447 (7.13), 6.540 (6.24), 8.003 (3.43), 8.576 (3.51).

### Example 48

### (rac)-2'-[6-amino-5-(trifluoromethyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide

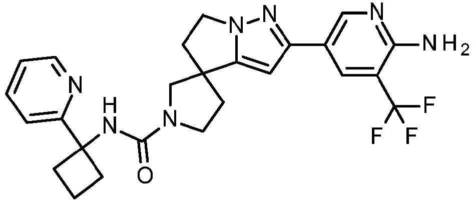

Example 48 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(pyridin-2-yl)cyclobutan-1-amine (34.0 mg, 229 µmol).
LC-MS (Method 2): Rt = 0.77 min; MS (ESlpos): m/z = 498 [M+H]⁺
¹H NMR (400 MHz, DMSO-*d*₆) δ ppm 1.23 (s, 1 H) 1.85 - 2.15 (m, 4 H) 2.52 - 2.61 (m, 5 H) 3.43 - 3.52 (m, 3 H) 3.53 - 3.67 (m, 1 H) 4.20 (t, 2 H) 6.48 (s, 1 H) 6.56 (s, 2 H) 6.79 (s, 1 H) 7.17 (ddd, 1 H) 7.37 (d, 1 H) 7.67 (td, 1 H) 8.01 (d, 1 H) 8.53 (d, 1 H) 8.59 (d, 1 H)

### Example 49

### 2'-[6-amino-5-(trifluoromethyl)pyridin-3-yl]-N-[1-(1,3,4-trimethyl-1H-pyrazol-5-yl)ethyl]-5',6'-dihydrospiro[pyrrolidine-3,4'-pyrrolo[1,2-b]pyrazole]-1-carboxamide (diastereometric mixture)

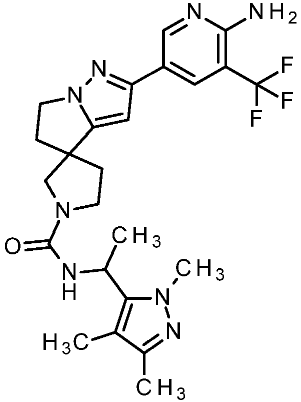

Example 49 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (33.0 mg, 91.8 µmol) and (*rac*)-1-(1,3,4-trimethyl-1H-pyrazol-5-yl)ethan-1-amine (21.1 mg, 138 µmol).
LC-MS (method 1): Rt = 0.98 min; MS (ESlpos): m/z = 503 [M+H]⁺
¹H-NMR (400 MHz, DMSO-d6) δ [ppm]: 1.383 (4.24), 1.389 (3.94), 1.401 (4.53), 1.408 (3.75), 1.919 (6.99), 1.956 (16.00), 1.972 (9.50), 2.049 (2.01), 2.327 (0.85), 2.539 (2.84), 2.669 (0.91), 3.372 (2.06), 3.398 (2.16), 3.404 (2.49), 3.424 (0.80), 3.452 (2.99), 3.477 (1.62), 3.538 (0.58), 3.564 (0.43), 3.695 (7.90), 3.708 (9.89), 4.160 (1.82), 4.177 (3.49), 4.194 (1.82), 4.892 (0.78), 4.909 (1.11), 4.926 (0.78), 6.434 (3.72), 6.440 (4.68), 6.452 (1.75), 6.545 (4.85), 8.003 (2.94), 8.575 (2.56).

### Example 50

### 2'-[6-amino-5-(trifluoromethyl)pyridin-3-yl]-N-[(1R)-1-(4-cyanopyridin-2-yl)ethyl]-5',6'-dihydrospiro[pyrrolidine-3,4'-pyrrolo[1,2-b]pyrazole]-1-carboxamide (mixture of stereoisomers)

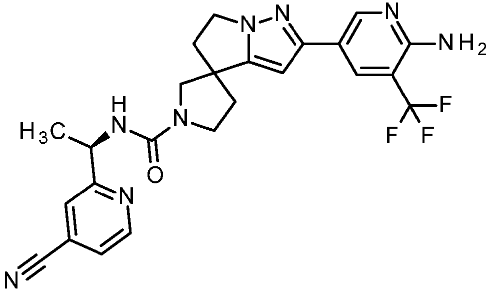

Example 50 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-[(1R)-1-aminoethyl]pyridine-4-carbonitrile hydrochloride (42.1 mg, 229 µmol).
LC-MS (Method 2): Rt = 0.92 min; MS (ESlpos): m/z = 497 [M+H]⁺
¹H NMR (400 MHz, DMSO-*d*₆) δ ppm 1.41 (dd, 3 H) 2.02 - 2.13 (m, 2 H) 2.52 - 2.57 (m, 2 H) 3.37 - 3.51 (m, 3 H) 3.52 - 3.65 (m, 1 H) 4.19 (t, 2 H) 4.88 - 4.97 (m, 1 H) 6.47 (s, 1 H) 6.54 (s, 2 H) 6.63 (dd, 1 H) 8.01 (t, 1 H) 8.21 - 8.29 (m, 1 H) 8.58 (s, 1 H) 8.79 - 8.88 (m, 2 H)

### Example 51

### 2'-[6-amino-5-(trifluoromethyl)pyridin-3-yl]-N-[(1R)-1-(pyridin-3-yl)ethyl]-5',6'-dihydrospiro[pyrrolidine-3,4'-pyrrolo[1,2-b]pyrazole]-1-carboxamide (mixture of stereoisomers)

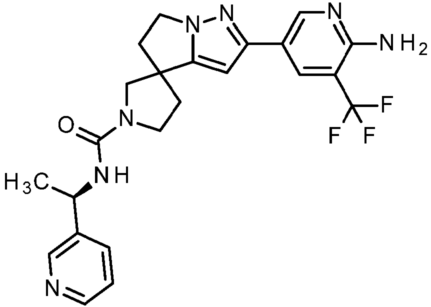

Example 51 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1R)-1-(pyridin-3-yl)ethan-1-amine hydrochloride (61.0 mg, 313 µmol).
LC-MS (method 2): Rt = 0.71 min; MS (ESlpos): m/z = 472 [M+H]⁺
¹H NMR (400 MHz, DMSO-*d*₆) δ ppm 1.40 (dd, 3 H) 2.01 - 2.12 (m, 2 H) 2.53 - 2.57 (m, 2 H) 3.41 - 3.49 (m, 3 H) 3.50 - 3.62 (m, 1 H) 4.16 - 4.22 (m, 2 H) 4.83 - 4.91 (m, 1 H) 6.43 - 6.48 (m, 1 H) 6.51 - 6.60 (m, 3 H) 7.32 (td, 1 H) 7.71 - 7.77 (m, 1 H) 8.01 (s, 1 H) 8.38 - 8.43 (m, 1 H) 8.53 - 8.60 (m, 2 H)

### Example 52

### (rac)-2'-[6-amino-5-(trifluoromethyl)pyridin-3-yl]-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide

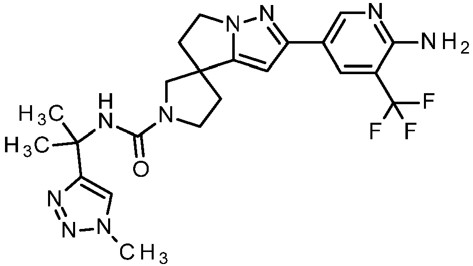

Example 52 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(1-methyl-1H-1,2,3-triazol-4-yl)propan-2-amine hydrochloride (48.9 mg, 229 µmol).
LC-MS (Method 2): Rt = 0.85 min; MS (ESlpos): m/z = 490 [M+H]⁺
¹H NMR (400 MHz, DMSO-*d*₆) δ ppm 1.60 (d, 6 H) 2.00 - 2.11 (m, 2 H) 2.53 - 2.56 (m, 2 H) 3.36 - 3.46 (m, 3 H) 3.48 - 3.58 (m, 1 H) 3.97 (s, 3 H) 4.18 (t, 2 H) 5.92 (s, 1 H) 6.48 (s, 1 H) 6.54 (s, 2 H) 7.80 (s, 1 H) 8.02 (d, 1 H) 8.60 (d, 1 H)

### Example 53

### (rac)-2'-[6-amino-5-(trifluoromethyl)pyridin-3-yl]-N-{[3-(trifluoromethyl)phenyl]methyl}-5',6'-dihydrospiro[pyrrolidine-3,4'-pyrrolo[1,2-b]pyrazole]-1-carboxamide

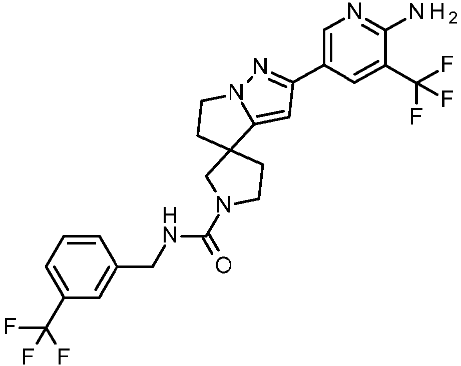

Example 53 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 90 % purity, 250 µmol) and 1-[3-(trifluoromethyl)phenyl]methanamine (52.6 mg, 300 µmol).
LC-MS (method 1): Rt = 1.15 min; MS (ESlpos): m/z = 525 [M+H]⁺
¹H NMR (400 MHz, DMSO-*d*₆) δ ppm 2.05 - 2.14 (m, 2 H) 2.53 - 2.59 (m, 2 H) 3.42 - 3.51 (m, 3 H) 3.52 - 3.62 (m, 1 H) 4.19 (t, 2 H) 4.33 (d, 2 H) 6.45 (s, 1 H) 6.55 (s, 2 H) 6.93 (t, 1 H) 7.52 - 7.63 (m, 4 H) 8.01 (d, 1 H) 8.59 (d, 1 H)

### Example 54

### 2'-[6-amino-5-(trifluoromethyl)pyridin-3-yl]-N-[1-(1-phenyl-1H-pyrazol-4-yl)ethyl]-5',6'-dihydrospiro[pyrrolidine-3,4'-pyrrolo[1,2-b]pyrazole]-1-carboxamide (diastereometric mixture)

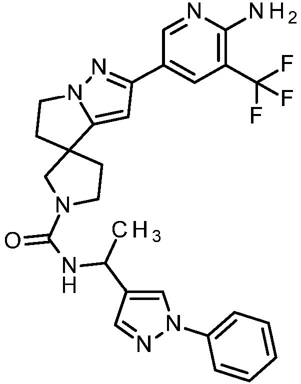

Example 54 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (*rac*)-1-(1-phenyl-1H-pyrazol-4-yl)ethan-1-amine (58.5 mg, 313 µmol).
LC-MS (method 1): Rt = 1.10 min; MS (ESlpos): m/z = 537 [M+H]⁺
¹H-NMR (400MHz, DMSO-d6): δ [ppm]= 1.43 and 1.44 (2d, 3H), 2.01 - 2.13 (m, 2H), 2.52 - 2.57 (m, 2H), 3.40 - 3.51 (m, 3H), 3.53 - 3.61 (m, 1H), 4.19 (t, 2H), 4.91 (quin, 1H), 6.34 and 6.36 (2s, 1H), 6.48 and 6.49 (2s, 1H), 6.54 (s, 2H), 7.24 - 7.30 (m, 1H), 7.43 - 7.51 (m, 2H), 7.66 and 7.69 (2s, 1H), 7.76 - 7.82 (m, 2H), 8.02 (s, 1H), 8.32 and 8.34 (2s, 1H), 8.60 (d, 1H).

### Example 55

### 2'-[6-amino-5-(trifluoromethyl)pyridin-3-yl]-N-[(3S)-2,3-dihydro-1-benzofuran-3-yl]-5',6'-dihydrospiro[pyrrolidine-3,4'-pyrrolo[1,2-b]pyrazole]-1-carboxamide (mixture of stereoisomers)

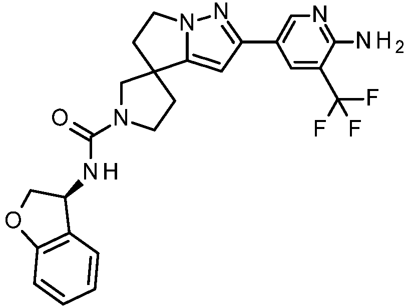

Example 55 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (3S)-2,3-dihydro-1-benzofuran-3-amine (31.0 mg, 229 µmol).
LC-MS (Method 2): Rt = 1.02 min; MS (ESlpos): m/z = 485 [M+H]⁺
¹H NMR (400 MHz, DMSO-*d*₆) δ ppm 2.01 - 2.10 (m, 2 H) 2.52 - 2.56 (m, 2 H) 3.40 - 3.50 (m, 3 H) 3.50 - 3.62 (m, 1 H) 4.17 (t, 2 H) 4.24 (dt, 1 H) 4.66 (td, 1 H) 5.43 - 5.50 (m, 1 H) 6.47 (d, 1 H) 6.54 (s, 2 H) 6.73 - 6.78 (m, 1 H) 6.80 (d, 1 H) 6.84 - 6.91 (m, 1 H) 7.18 (tdd, 1 H) 7.33 (t, 1 H) 8.01 (s, 1 H) 8.59 (d, 1 H)

### Example 56

### 2'-[6-amino-5-(trifluoromethyl)pyridin-3-yl]-N-[(1R)-1-(6-cyanopyridin-3-yl)ethyl]-5',6'-dihydrospiro[pyrrolidine-3,4'-pyrrolo[1,2-b]pyrazole]-1-carboxamide (mixture of stereoisomers)

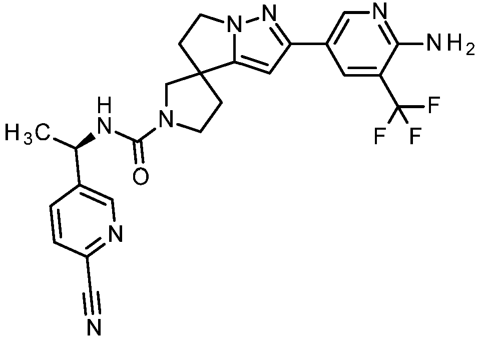

Example 56 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 5-[(1R)-1-aminoethyl]pyridine-2-carbonitrile (33.7 mg, 229 µmol).
LC-MS (Method 2): Rt = 0.94 min; MS (ESlpos): m/z = 497 [M+H]⁺
¹H NMR (400 MHz, DMSO-*d*₆) δ ppm 1.41 (dd, 3 H) 1.99 - 2.15 (m, 2 H) 2.52 - 2.57 (m, 2 H) 3.47 - 3.54 (m, 3 H) 3.55 - 3.63 (m, 1 H) 4.18 (br t, 2 H) 4.92 (quin, 1 H) 6.47 (s, 1 H) 6.53 (s, 2 H) 6.72 (dd, 1 H) 7.95 - 7.99 (m, 2 H) 8.01 (s, 1 H) 8.58 (d, 1 H) 8.73 (d, 1 H)

### Example 57

### 2'-[6-amino-5-(trifluoromethyl)pyridin-3-yl]-N-{(1S)-1-[1-methyl-3-(trifluoromethyl)-1 H-pyrazol-4-yl]ethyl}-5',6'-dihydrospiro[pyrrolidine-3,4'-pyrrolo[1,2-b]pyrazole]-1-carboxamide (mixture of stereoisomers)

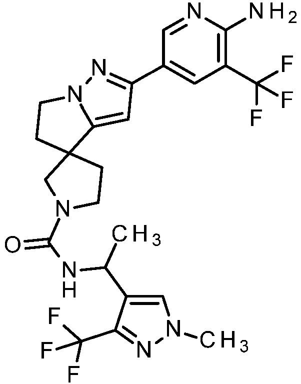

Example 57 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (31.3 mg, 87.1 µmol) and 1): 1-[1-methyl-3-(trifluoromethyl)-1H-pyrazol-4-yl]ethan-1-amine hydrochloride (30.0 mg, 131 µmol).
¹H-NMR (400MHz, DMSO-d6): δ [ppm]= 1.34 and 1.34 (2d, 3H), 2.01 - 2.11 (m, 2H), 3.36 - 3.58 (m, 5H), 3.85 - 3.88 (m, 4H), 4.18 (t, 2H), 4.89 - 5.01 (m, 1H), 6.36 and 6.38 (2s, 1H), 6.42 - 6.47 (m, 1H), 6.55 (br d, 2H), 7.80 and 7.85 (2s, 1H), 7.99 - 8.02 (m, 1H), 8.57 - 8.61 (m, 1H).
LC-MS (method 1): Rt = 1.04 min; MS (ESlneg): m/z = 541 [M-H]⁻

### Example 58

### 2'-[6-amino-5-(trifluoromethyl)pyridin-3-yl]-N-[(1R)-1-(pyridin-2-yl)ethyl]-5',6'-dihydrospiro[pyrrolidine-3,4'-pyrrolo[1,2-b]pyrazole]-1-carboxamide (mixture of stereoisomers)

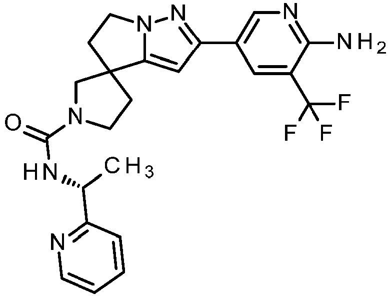

Example 58 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1R)-1-(pyridin-2-yl)ethan-1-amine (28.0 mg, 229 µmol).
LC-MS (method 2): Rt = 0.75 min; MS (ESlpos): m/z = 472 [M+H]⁺
¹H NMR (400 MHz, DMSO-*d*₆) δ ppm 1.39 (dd, 3 H) 2.00 - 2.14 (m, 2 H) 2.54 - 2.59 (m, 2 H) 3.44 - 3.51 (m, 3 H) 3.51 - 3.64 (m, 1 H) 4.15 - 4.23 (m, 2 H) 4.84 - 4.92 (m, 1 H) 6.47 - 6.49 (m, 1 H) 6.55 (s, 2 H) 7.19 - 7.25 (m, 1 H) 7.39 (dd, 1 H) 7.73 (dt, 1 H) 7.73 (dt, 1 H) 8.01 (d, 1 H) 8.48 (ddt, 1 H) 8.59 (s, 1 H)

### Example 59

### (rac)-2'-[6-amino-5-(trifluoromethyl)pyridin-3-yl]-N-benzyl-5',6'-dihydrospiro[pyrrolidine-3,4'-pyrrolo[1,2-b]pyrazole]-1-carboxamide

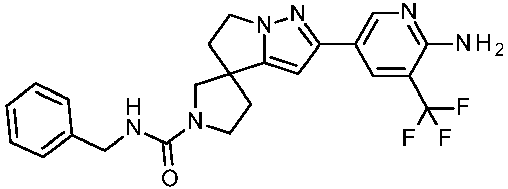

Example 59 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-phenylmethanamine (27 µL, 250 µmol).
LC-MS (Method 2): Rt = 1.01 min; MS (ESlpos): m/z = 457 [M+H]⁺
¹H NMR (400 MHz, DMSO-*d*₆) δ ppm 2.03 - 2.14 (m, 2 H) 3.46 (s, 3 H) 3.52 - 3.60 (m, 1 H) 4.19 (t, 2 H) 4.25 (d, 2 H) 6.47 (s, 1 H) 6.55 (s, 2 H) 6.80 (t, 1 H) 7.20 (s, 1 H) 7.24 - 7.32 (m, 4 H) 8.02 (d, 1 H) 8.59 (d, 1 H)

### Example 60

### 2'-[6-amino-5-(trifluoromethyl)pyridin-3-yl]-N-[(1S)-1-phenylethyl]-5',6'-dihydrospiro[pyrrolidine-3,4'-pyrrolo[1,2-b]pyrazole]-1-carboxamide (mixture of stereoisomers)

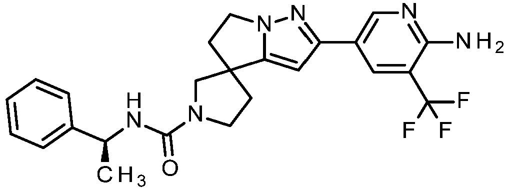

Example 60 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1S)-1-phenylethan-1-amine (27.8 mg, 229 µmol).
LC-MS (Method 1): Rt = 1.08 min; MS (ESlpos): m/z = 471 [M+H]⁺
¹H NMR (400 MHz, DMSO-*d*₆) δ ppm 1.37 (dd, 3 H) 1.77 (s, 1 H) 2.00 - 2.13 (m, 2 H) 2.52 - 2.56 (m, 2 H) 3.56 (br s, 3 H) 4.18 (t, 2 H) 4.84 (quin, 1 H) 6.43 - 6.49 (m, 2 H) 6.55 (s, 2 H) 7.14 - 7.23 (m, 1 H) 7.24 - 7.38 (m, 4 H) 8.01 (d, 1 H) 8.58 (t, 1 H)

### Example 61

### 2'-[6-amino-5-(trifluoromethyl)pyridin-3-yl]-N-[(1S)-1-(1,2-oxazol-3-yl)ethyl]-5',6'-dihydrospiro[pyrrolidine-3,4'-pyrrolo[1,2-b]pyrazole]-1-carboxamide (mixture of stereoisomers)

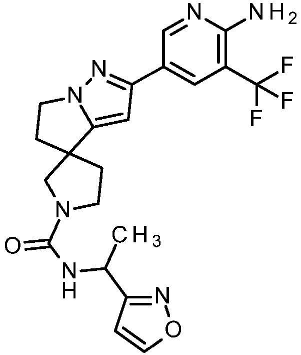

Example 61 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1): 1-(1,2-oxazol-3-yl)ethan-1-amine hydrochloride (46.5 mg, 313 µmol).
¹H-NMR (400MHz, DMSO-d6): δ [ppm]= 1.42 and 1.43 (2d, 3H), 2.01 - 2.13 (m, 2H), 2.52 - 2.57 (m, 2H), 3.40 - 3.50 (m, 3H), 3.52 - 3.60 (m, 1H), 4.19 (t, 2H), 5.01 (quin, 1H), 6.48 (d, 1H), 6.50 - 6.56 (m, 3H), 6.58 and 6.60 (2d, 1H), 8.02 (d, 1H), 8.59 (d, 1H), 8.76 and 8.77 (2d, 1H).
LC-MS (method 1): Rt = 0.93 min; MS (ESlpos): m/z = 462 [M+H]⁺

### Example 62

### 2'-[6-amino-5-(trifluoromethyl)pyridin-3-yl]-N-[(1S)-1-(1,5-dimethyl-1H-pyrazol-4-yl)ethyl]-5',6'-dihydrospiro[pyrrolidine-3,4'-pyrrolo[1,2-b]pyrazole]-1-carboxamide (diastereomeric mixture)

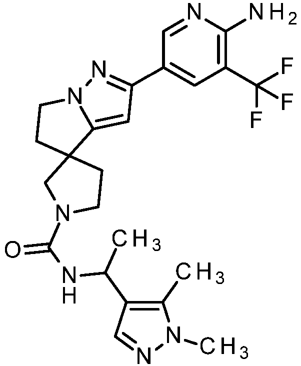

Example 62 was prepared in analogy to Example 31 using (*rac*)-5-[-5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(1,5-dimethyl-1H-pyrazol-4-yl)ethan-1-amine (43.5 mg, 313 µmol).
¹H-NMR (400MHz, DMSO-d6): δ [ppm]= 1.32 and 1.34 (2d, 3H), 1.98 - 2.10 (m, 2H), 2.18 and 2.19 (2s, 3H), 3.35 - 3.45 (m, 3H), 3.46 - 3.56 (m, 1H), 3.65 and 3.66 (2s, 3H), 4.17 (t, 2H), 4.71 - 4.80 (m, 1H), 6.14 and 6.16 (2d, 1H), 6.44 and 6.45 (2s, 1H), 6.54 (s, 2H), 7.28 and 7.30 (2s, 1H), 8.01 (d, 1H), 8.58 (d, 1H).
LC-MS (method 1): Rt = 0.92 min; MS (ESlpos): m/z = 489 [M+H]⁺

### Example 63

### (rac)-2'-[6-amino-5-(trifluoromethyl)pyridin-3-yl]-N-[2-(oxan-4-yl)propan-2-yl]-5',6'-dihydrospiro[pyrrolidine-3,4'-pyrrolo[1,2-b]pyrazole]-1-carboxamide

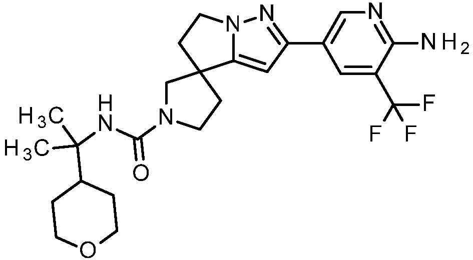

Example 63 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(oxan-4-yl)propan-2-amine hydrochloride (41.2 mg, 229 µmol).
LC-MS (Method 2): Rt = 1.00 min; MS (ESlpos): m/z = 494 [M+H]⁺
¹H NMR (400 MHz, DMSO-*d*₆) δ ppm 1.18 (d, 6 H) 1.20 - 1.32 (m, 2 H) 1.46 (br s, 2 H) 1.98 - 2.10 (m, 2 H) 2.22 - 2.32 (m, 1 H) 2.53 - 2.58 (m, 1 H) 3.20 (br t, 2 H) 3.35 - 3.43 (m, 3 H) 3.46 - 3.56 (m, 1 H) 3.83 - 3.90 (m, 2 H) 4.18 (t, 2 H) 5.14 (s, 1 H) 6.43 (s, 1 H) 6.55 (s, 2 H) 8.00 (d, 1 H) 8.57 (d, 1 H)

### Example 64

### 2'-[6-amino-5-(trifluoromethyl)pyridin-3-yl]-N-[(1R)-1-(pyridin-4-yl)ethyl]-5',6'-dihydrospiro[pyrrolidine-3,4'-pyrrolo[1,2-b]pyrazole]-1-carboxamide (mixture of stereoisomers)

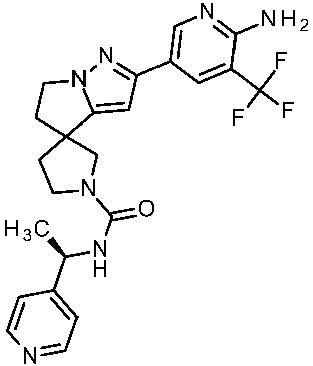

Example 64 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) and (1R)-1-(pyridin-4-yl)ethan-1-amine hydrochloride (32.5 mg, 167 µmol).
¹H NMR (400 MHz, DMSO-*d*₆) δ ppm 1.37 (dd, 3 H) 2.02 - 2.14 (m, 2 H) 2.52 - 2.58 (m, 2 H) 3.44 - 3.52 (m, 3 H) 3.54 - 3.65 (m, 1 H) 4.19 (t, 2 H) 4.77 - 4.86 (m, 1 H) 6.45 - 6.51 (m, 1 H) 6.51 - 6.62 (m, 3 H) 7.26 - 7.36 (m, 2 H) 8.02 (d, 1 H) 8.44 - 8.49 (m, 2 H) 8.59 (s, 1 H)
LC-MS (method 1): Rt = 0.89 min; MS (ESlneg): m/z = 470 [M-H]⁻

### Example 65

### 2'-[6-amino-5-(trifluoromethyl)pyridin-3-yl]-N-[(1S)-1-(1-ethyl-5-methyl-1H-pyrazol-4-yl)ethyl]-5',6'-dihydrospiro[pyrrolidine-3,4'-pyrrolo[1,2-b]pyrazole]-1-carboxamide (diastereomeric mixture)

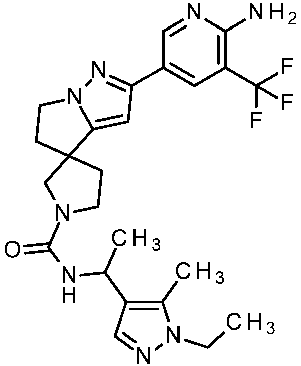

Example 65 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(1-ethyl-5-methyl-1H-pyrazol-4-yl)ethan-1-amine (47.9 mg, 313 µmol).
¹H-NMR (400MHz, DMSO-d6): δ [ppm]= 1.22 - 1.28 (m, 3H), 1.33 and 1.34 (2d, 3H), 1.98 - 2.10 (m, 2H), 2.19 and 2.20 (2s, 3H), 3.36 - 3.45 (m, 3H), 3.46 - 3.56 (m, 1H), 3.93 - 4.03 (m, 2H), 4.17 (t, 2H), 4.77 (quin, 1H), 6.15 and 6.17 (2d, 1H), 6.45 (d, 1H), 6.54 (s, 2H), 7.32 (d, 1H), 8.01 (d, 1H), 8.58 (d, 1H).
LC-MS (method 1): Rt = 0.97 min; MS (ESlpos): m/z = 503 [M+H]⁺

### Example 66

### (rac)-2'-[6-amino-5-(trifluoromethyl)pyridin-3-yl]-N-[(pyridin-3-yl)methyl]-5',6'-dihydrospiro[pyrrolidine-3,4'-pyrrolo[1,2-b]pyrazole]-1-carboxamide

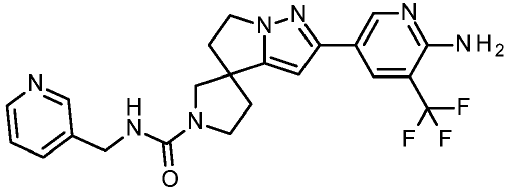

Example 66 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(pyridin-3-yl)methanamine (24.8 mg, 229 µmol).
LC-MS (Method 2): Rt = 0.86 min; MS (ESlpos): m/z = 458 [M+H]⁺
¹H NMR (400 MHz, DMSO-*d*₆) δ ppm 2.02 - 2.14 (m, 2 H) 2.53 - 2.59 (m, 2 H) 3.36 - 3.58 (m, 4 H) 4.19 (t, 2 H) 4.26 (d, 2 H) 6.47 (s, 1 H) 6.54 (s, 2 H) 6.87 (t, 1 H) 7.32 (ddd, 1 H) 7.68 (dt, 1 H) 8.02 (d, 1 H) 8.42 (dd, 1 H) 8.50 (d, 1 H) 8.59 (d, 1 H)

### Example 67

### 2'-[6-amino-5-(trifluoromethyl)pyridin-3-yl]-N-[(1S)-2-methoxy-1-phenylethyl]-5',6'-dihydrospiro[pyrrolidine-3,4'-pyrrolo[1,2-b]pyrazole]-1-carboxamide (mixture of stereoisomers)

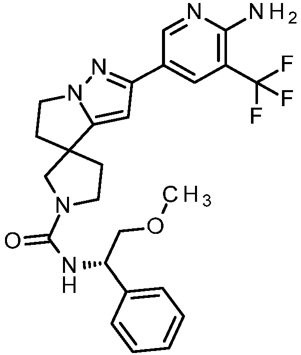

Example 67 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1S)-2-methoxy-1-phenylethan-1-amine (47.3 mg, 313 µmol).

LC-MS (Method 2): Rt = 1.03 min; MS (ESlpos): m/z = 501 [M+H]⁺ 1H-NMR (400MHz, DMSO-d6): δ [ppm]= 2.03 - 2.11 (m, 2H), 2.54 - 2.58 (m, 2H), 3.24 and 3.26 (2d, 3H), 3.41 - 3.63 (m, 6H), 4.19 (t, 2H), 4.91 - 4.99 (m, 1H), 6.42 - 6.50 (m, 2H), 6.55 (s, 2H), 7.17 - 7.24 (m, 1H), 7.25 - 7.39 (m, 4H), 8.00 (s, 1H), 8.58 (s, 1H).

### Example 68

### (rac)-2'-[6-amino-5-(trifluoromethyl)pyridin-3-yl]-N-{[3-(trifluoromethyl)bicyclo[1.1.1]pentan-1-yl]methyl}-5',6'-dihydrospiro[pyrrolidine-3,4'-pyrrolo[1,2-b]pyrazole]-1-carboxamide

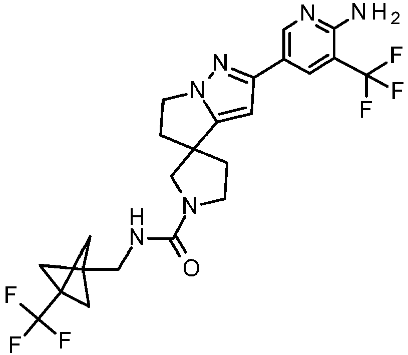

Example 68 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-[3-(trifluoromethyl)bicyclo[1.1.1]pentan-1-yl]methanamine hydrochloride (50.4 mg, 250 µmol).
LC-MS (Method 1): Rt = 1.14 min; MS (ESlpos): m/z = 515 [M+H]⁺
¹H NMR (400 MHz, DMSO-*d*₆) δ ppm 1.82 (s, 6 H) 2.01 - 2.12 (m, 2 H) 3.15 - 3.22 (m, 2 H) 3.40 - 3.45 (m, 3 H) 3.52 (dt, 1 H) 4.19 (t, 2 H) 6.26 (t, 1 H) 6.44 (s, 1 H) 6.54 (s, 2 H) 8.00 (d, 1 H) 8.58 (d, 1 H)

### Example 69

### (rac)-2'-[6-amino-5-(trifluoromethyl)pyridin-3-yl]-N-[(pyridin-4-yl)methyl]-5',6'-dihydrospiro[pyrrolidine-3,4'-pyrrolo[1,2-b]pyrazole]-1-carboxamide

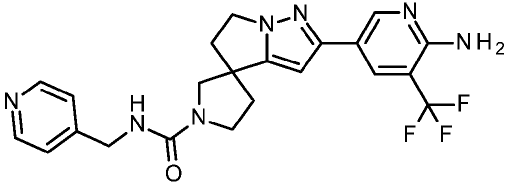

Example 69 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(pyridin-4-yl)methanamine (24.8 mg, 229 µmol).
LC-MS (Method 1): Rt = 0.85 min; MS (ESlpos): m/z = 458 [M+H]⁺
¹H NMR (400 MHz, DMSO-*d*₆) δ ppm 1.85 - 2.02 (m, 1 H) 2.02 - 2.14 (m, 2 H) 2.56 (t, 2 H) 3.44 - 3.51 (m, 3 H) 3.57 (s, 1 H) 4.19 (t, 2 H) 4.27 (d, 2 H) 6.49 (s, 1 H) 6.55 (s, 1 H) 6.91 (t, 1 H) 7.27 (d, 2 H) 8.03 (d, 1 H) 8.47 (d, 2 H) 8.60 (d, 1 H)

### Example 70

### 2'-[6-amino-5-(trifluoromethyl)pyridin-3-yl]-N-[(1R)-1-(3-cyanopyridin-2-yl)ethyl]-5',6'-dihydrospiro[pyrrolidine-3,4'-pyrrolo[1,2-b]pyrazole]-1-carboxamide (mixture of stereoisomers)

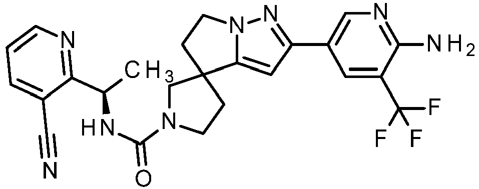

Example 70 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-[(1R)-1-aminoethyl]pyridine-3-carbonitrile hydrochloride (42.1 mg, 229 µmol).
LC-MS (Method 2): Rt = 0.93 min; MS (ESlpos): m/z = 497 [M+H]⁺
¹H NMR (400 MHz, DMSO-*d*₆) δ ppm 0.98 - 1.08 (m, 1 H) 1.37 - 1.47 (m, 3 H) 1.95 - 2.17 (m, 2 H) 3.41 - 3.51 (m, 3 H) 3.55 (br s, 1 H) 4.11 - 4.26 (m, 2 H) 5.05 - 5.15 (m, 1 H) 6.46 (s, 1 H) 6.50 - 6.58 (m, 2 H) 6.66 (dd, 1 H) 7.43 - 7.52 (m, 1 H) 8.01 (d, 1 H) 8.25 (d, 1 H) 8.58 (t, 1 H) 8.73 - 8.84 (m, 1 H)

### Example 71

### 2'-[6-amino-5-(trifluoromethyl)pyridin-3-yl]-N-[(1S)-1-(1-methyl-1H-benzimidazol-2-yl)ethyl]-5',6'-dihydrospiro[pyrrolidine-3,4'-pyrrolo[1,2-b]pyrazole]-1-carboxamide (diastereomeric mixture)

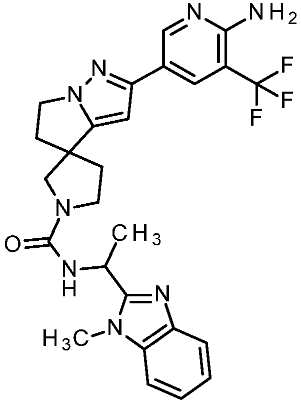

Example 71 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(1-methyl-1H-benzimidazol-2-yl)ethan-1-amine (54.8 mg, 313 µmol).
LC-MS (method 1): Rt = 1.02 min; MS (ESlpos): m/z = 525 [M+H]⁺
¹H-NMR (400MHz, DMSO-d6): δ [ppm]= 1.55 and 1.56 (2d, 3H), 2.00 - 2.12 (m, 2H), 2.51 - 2.55 (m, 2H), 3.39 - 3.50 (m, 3H), 3.51 - 3.61 (m, 1H), 3.77 and 3.78 (2s, 3H), 4.13 - 4.21 (m, 2H), 5.19 - 5.30 (m, 1H), 6.46 (s, 1H), 6.54 (s, 2H), 6.68 and 6.69 (2d, 1H), 7.13 - 7.26 (m, 2H), 7.47 - 7.52 (m, 1H), 7.55 - 7.61 (m, 1H), 8.00 (d, 1H), 8.58 (d, 1H).

### Example 72

### 2'-[6-amino-5-(trifluoromethyl)pyridin-3-yl]-N-[1-(4-fluorophenyl)-2-methoxyethyl]-5',6'-dihydrospiro[pyrrolidine-3,4'-pyrrolo[1,2-b]pyrazole]-1-carboxamide (mixture of isomers)

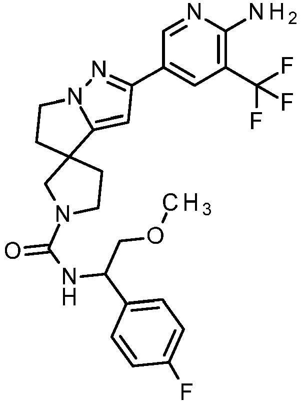

Example 72 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (70.0 mg, 195 µmol) and 1-(4-fluorophenyl)-2-methoxyethan-1-amine (39.5 mg, 233 µmol).
LC-MS (method 1): Rt = 1.04 min; MS (ESlpos): m/z = 519 [M+H]⁺
1H-NMR (400MHz, DMSO-d6): δ [ppm]= 2.01 - 2.13 (m, 2H), 2.52 - 2.57 (m, 2H), 3.24 and 3.25 (2s, 3H), 3.40 - 3.50 (m, 4H), 3.51 - 3.62 (m, 2H), 4.19 (t, 2H), 4.91 - 5.00 (m, 1H), 6.43 - 6.51 (m, 2H), 6.54 (s, 2H), 7.07 - 7.16 (m, 2H), 7.34 - 7.43 (m, 2H), 8.00 and 8.00 (2d, 1H), 8.58 (d, 1H).

### Example 73

### (rac)-2'-[6-amino-5-(trifluoromethyl)pyridin-3-yl]-N-[(pyridin-2-yl)methyl]-5',6'-dihydrospiro[pyrrolidine-3,4'-pyrrolo[1,2-b]pyrazole]-1-carboxamide

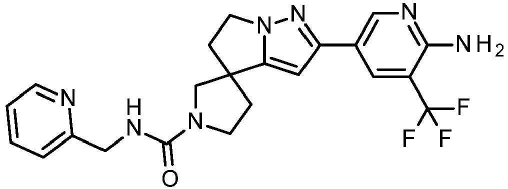

Example 73 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(pyridin-2-yl)methanamine (24.8 mg, 229 µmol).
LC-MS (Method 1): Rt = 0.88 min; MS (ESlneg): m/z = 456 [M-H]⁻
¹H NMR (400 MHz, DMSO-*d*₆) δ ppm 2.03 - 2.18 (m, 2 H) 2.54 - 2.59 (m, 2 H) 3.49 (s, 3 H) 3.56 - 3.64 (m, 1 H) 4.20 (t, 2 H) 4.34 (d, 2 H) 6.50 (s, 1 H) 6.55 (s, 2 H) 6.88 (t, 1 H) 7.23 (dd, 1 H) 7.32 (d, 1 H) 7.73 (td, 1 H) 8.02 (d, 1 H) 8.47 (d, 1 H) 8.60 (d, 1 H)

### Example 74

### 2'-[6-amino-5-(trifluoromethyl)pyridin-3-yl]-N-[(1S)-1-(1-ethyl-1H-imidazol-2-yl)ethyl]-5',6'-dihydrospiro[pyrrolidine-3,4'-pyrrolo[1,2-b]pyrazole]-1-carboxamide (diastereomeric mixture)

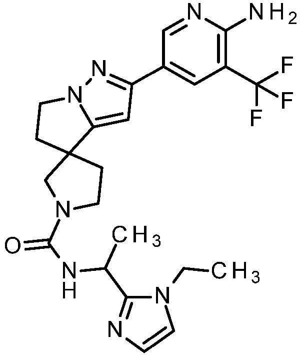

Example 74 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(1-ethyl-1H-imidazol-2-yl)ethan-1-amine hydrochloride (66.3 mg, 313 µmol).
¹H-NMR (400MHz, DMSO-d6): δ [ppm]= 1.25 and 1.27 (2t, 3H), 1.43 and 1.444 (2d, 3H), 1.99 - 2.11 (m, 2H), 3.36 - 3.60 (m, 4H), 3.86 - 4.05 (m, 2H), 4.17 (t, 2H), 4.99 - 5.08 (m, 1H), 6.41 - 6.49 (m, 2H), 6.54 (s, 2H), 6.78 and 6.78 (2d, 1H), 7.09 and 7.09 (2d, 1H), 7.98 - 8.02 (m, 1H), 8.56 - 8.59 (m, 1H).
LC-MS (method 1): Rt = 0.92 min; MS (ESlpos): m/z = 489 [M+H]⁺

### Example 75

### 2'-[6-amino-5-(trifluoromethyl)pyridin-3-yl]-N-[(1S)-1-(1-methyl-1H-imidazol-2-yl)ethyl]-5',6'-dihydrospiro[pyrrolidine-3,4'-pyrrolo[1,2-b]pyrazole]-1-carboxamide (diastereomeric mixture)

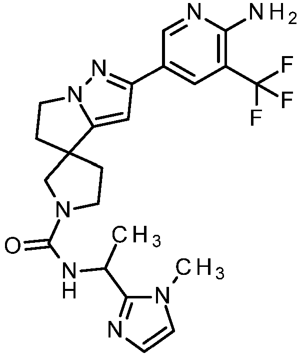

Example 75 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(1-methyl-1H-imidazol-2-yl)ethan-1-amine hydrochloride (61.9 mg, 313 µmol).
¹H-NMR (400MHz, DMSO-d6): δ [ppm]= 1.42 and 1.43 (2d, 3H), 1.99 - 2.11 (m, 2H), 3.36 - 3.56 (m, 4H), 3.58 and 3.60 (2s, 3H), 4.17 (t, 2H), 4.97 - 5.06 (m, 1H), 6.39 - 6.46 (m, 2H), 6.54 (s, 2H), 6.76 and 6.76 (2d, 1H), 7.02 and 7.02 (2d, 1H), 8.01 (d, 1H), 8.58 (d, 1H).
LC-MS (method 1): Rt = 0.87 min; MS (ESlpos): m/z = 475 [M+H]⁺

### Example 76

### (rac)-2'-[6-amino-5-(trifluoromethyl)pyridin-3-yl]-N-[(pyrazin-2-yl)methyl]-5',6'-dihydrospiro[pyrrolidine-3,4'-pyrrolo[1,2-b]pyrazole]-1-carboxamide

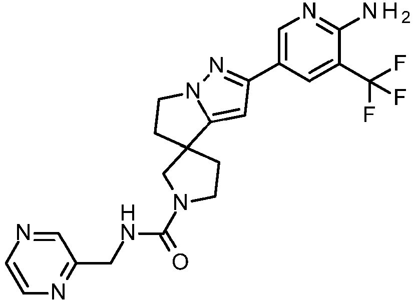

Example 76 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 90 % purity, 250 µmol) and 1-(pyrazin-2-yl)methanamine (32.8 mg, 300 µmol).
¹H NMR (400 MHz, DMSO-*d*₆) δ ppm 2.04 - 2.16 (m, 2 H) 2.52 - 2.58 (m, 2 H) 3.44 - 3.52 (m, 3 H) 3.53 - 3.63 (m, 1 H) 4.19 (t, 2 H) 4.39 (d, 2 H) 6.49 (s, 1 H) 6.54 (s, 2 H) 6.98 (t, 1 H) 8.02 (d, 1 H) 8.50 (d, 1 H) 8.56 (dd, 1 H) 8.60 (dt, 2 H)
LC-MS (method 1): Rt = 0.83 min; MS (ESlneg): m/z = 457 [M-H]⁻

### Example 77

### 2'-[6-amino-5-(trifluoromethyl)pyridin-3-yl]-N-[(1R)-2-methoxy-1-(pyridin-3-yl)ethyl]-5',6'-dihydrospiro[pyrrolidine-3,4'-pyrrolo[1,2-b]pyrazole]-1-carboxamide (mixture of stereoisomers)

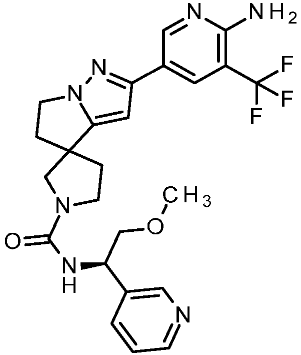

Example 77 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (70.0 mg, 195 µmol) and (1R)-2-methoxy-1-(pyridin-3-yl)ethan-1-amine hydrochloride (52.6 mg, 233 µmol).
LC-MS (method 1): Rt = 0.86 min; MS (ESlpos): m/z = 502 [M+H]⁺
1H-NMR (400MHz, DMSO-d6): δ [ppm]= 2.01 - 2.14 (m, 2H), 2.52 - 2.57 (m, 2H), 3.25 and 3.26 (2s, 3H), 3.40 - 3.66 (m, 6H), 4.19 (t, 2H), 4.98 (q, 1H), 6.43 - 6.48 (m, 1H), 6.51 - 6.60 (m, 3H), 7.33 (td, 1H), 7.73 - 7.81 (m, 1H), 8.01 (t, 1H), 8.43 (td, 1H), 8.54 - 8.60 (m, 2H).

### Example 78

### (rac)-2'-[6-amino-5-(trifluoromethyl)pyridin-3-yl]-N-[(pyridazin-4-yl)methyl]-5',6'-dihydrospiro[pyrrolidine-3,4'-pyrrolo[1,2-b]pyrazole]-1-carboxamide

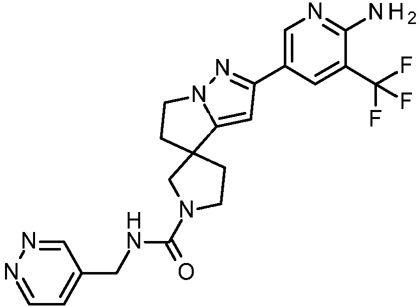

Example 78 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 90 % purity, 250 µmol) and 1-(pyridazin-4-yl)methanamine (32.8 mg, 300 µmol).
¹H NMR (400 MHz, DMSO-*d*₆) δ ppm 2.04 - 2.16 (m, 2 H) 2.53 - 2.59 (m, 2 H) 3.44 - 3.53 (m, 3 H) 3.54 - 3.63 (m, 1 H) 4.19 (t, 2 H) 4.30 (d, 2 H) 6.50 (s, 1 H) 6.55 (s, 2 H) 6.97 (t, 1 H) 7.54 (dd, 1 H) 8.03 (d, 1 H) 8.60 (d, 1 H) 9.12 (dd, 1 H) 9.16 (dd, 1 H)
LC-MS (method 1): Rt = 0.79 min; MS (ESlpos): m/z = 459 [M+H]⁺

### Example 79

### (rac)-2'-[6-amino-5-(trifluoromethyl)pyridin-3-yl]-N-[(1-methylpiperidin-4-yl)methyl]-5',6'-dihydrospiro[pyrrolidine-3,4'-pyrrolo[1,2-b]pyrazole]-1-carboxamide

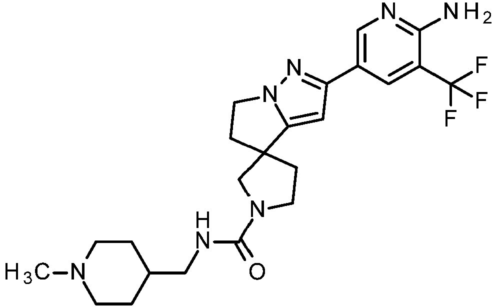

Example 79 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 90 % purity, 250 µmol) and 1-(1-methylpiperidin-4-yl)methanamine (38.5 mg, 300 µmol).
¹H NMR (400 MHz, DMSO-*d*₆) δ ppm 1.02 - 1.14 (m, 2 H) 1.35 (ddd, 1 H) 1.58 (br d, 2 H) 1.75 (br t, 2 H) 2.05 (br d, 2 H) 2.11 (s, 3 H) 2.52 - 2.58 (m, 2 H) 2.66 - 2.75 (m, 2 H) 2.90 (t, 2 H) 3.37 - 3.43 (m, 3 H) 3.51 (dt, 1 H) 4.18 (t, 2 H) 6.16 (t, 1 H) 6.44 (s, 1 H) 6.54 (s, 2 H) 8.00 (d, 1 H) 8.58 (d, 1 H)
LC-MS (method 1): Rt = 0.93 min; MS (ESlpos): m/z = 479 [M+H]⁺

### Example 80

### (rac)-2'-[6-amino-5-(trifluoromethyl)pyridin-3-yl]-N-[3-(methylsulfamoyl)pyridin-2-yl]-5',6'-dihydrospiro[pyrrolidine-3,4'-pyrrolo[1,2-b]pyrazole]-1-carboxamide

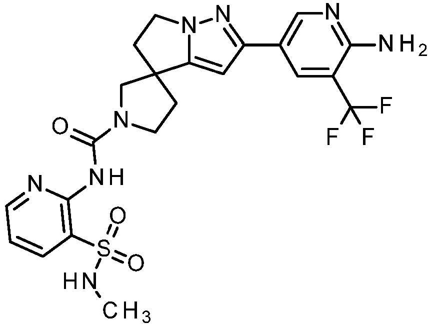

Example 80 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-amino-N-methylpyridine-3-sulfonamide (42.9 mg, 229 µmol).
LC-MS (method 2): Rt = 0.86 min; MS (ESlneg): m/z = 535 [M-H]⁻
¹H NMR (400 MHz, DMSO-*d*₆) δ ppm 2.16 (br t, 2 H) 2.33 - 2.34 (m, 1 H) 2.52 - 2.64 (m, 4 H) 3.54 - 3.63 (m, 3 H) 3.67 (br d, 1 H) 4.21 (t, 2 H) 6.56 (d, 3 H) 7.27 (dd, 1 H) 7.79 (br d, 1 H) 8.02 (d, 1 H) 8.12 (dd, 1 H) 8.55 (br d, 1 H) 8.60 (d, 1 H) 8.72 (br d, 1 H)

### Example 81

### (rac)-2'-[6-amino-5-(trifl uoromethyl)pyridin-3-yl]-N-[(1-methyl-2-oxabicyclo[2.1.1]hexan-4-yl)methyl]-5',6'-dihydrospiro[pyrrolidine-3,4'-pyrrolo[1,2-b]pyrazole]-1-carboxamide

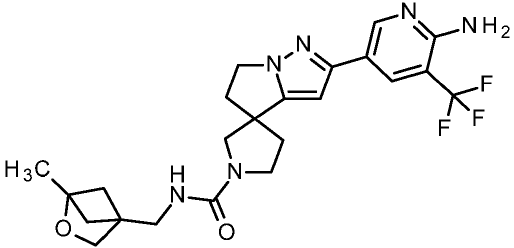

Example 81 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-(1-methyl-2-oxabicyclo[2.1.1]hexan-4-yl)methanamine (31.8 mg, 250 µmol).
¹H NMR (400 MHz, DMSO-*d*₆) δ ppm 1.15 (d, 1 H) 1.29 (s, 3 H) 1.34 (dd, 2 H) 1.49 (t, 2 H) 2.08 (s, 2 H) 2.52 - 2.57 (m, 2 H) 3.35 (br s, 1 H) 3.42 (s, 3 H) 3.46 (s, 2 H) 3.48 - 3.56 (m, 1 H) 4.18 (t, 2 H) 6.26 (t, 1 H) 6.44 (s, 1 H) 6.54 (s, 2 H) 8.01 (d, 1 H) 8.58 (d, 1 H)
LC-MS (method 1): Rt = 0.90 min; MS (ESlneg): m/z = 475 [M-H]⁻

### Example 82

### (rac)-2'-[6-amino-5-(trifluoromethyl)pyridin-3-yl]-N-[(1-methyl-2-oxopiperidin-4-yl)methyl]-5',6'-dihydrospiro[pyrrolidine-3,4'-pyrrolo[1,2-b]pyrazole]-1-carboxamide

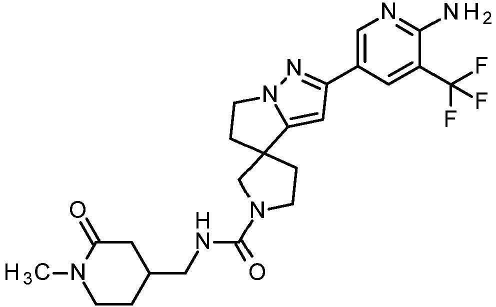

Example 82 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 90 % purity, 250 µmol) and 4-(aminomethyl)-1-methylpiperidin-2-one (42.7 mg, 300 µmol).
¹H NMR (400 MHz, DMSO-*d*₆) δ ppm 1.33 - 1.44 (m, 1 H) 1.79 - 1.97 (m, 3 H) 2.01 - 2.12 (m, 2 H) 2.19 - 2.29 (m, 1 H) 2.52 - 2.56 (m, 2 H) 2.78 (s, 3 H) 2.92 - 3.02 (m, 2 H) 3.17 - 3.31 (m, 2 H) 3.42 (s, 3 H) 3.47 - 3.58 (m, 1 H) 4.18 (t, 2 H) 6.26 (t, 1 H) 6.47 (s, 1 H) 6.54 (s, 2 H) 8.02 (d, 1 H) 8.59 (d, 1 H)
LC-MS (method 1): Rt = 0.83 min; MS (ESlneg): m/z = 490 [M-H]⁻

### Example 83

### (rac)-2'-[6-amino-5-(trifluoromethyl)pyridin-3-yl]-N-(2-cyclopropylpropan-2-yl)-5',6'-dihydrospiro[pyrrolidine-3,4'-pyrrolo[1,2-b]pyrazole]-1-carboxamide

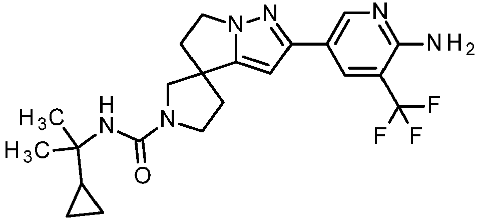

Example 83 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-cyclopropylpropan-2-amine hydrochloride (31.1 mg, 229 µmol).
LC-MS (Method 1): Rt = 1.12 min; MS (ESlpos): m/z = 449 [M+H]⁺
¹H NMR (400 MHz, DMSO-*d*₆) δ ppm 0.25 - 0.31 (m, 4 H) 1.15 (s, 6 H) 1.28 - 1.44 (m, 1 H) 1.96 - 2.13 (m, 2 H) 2.52 - 2.55 (m, 2 H) 3.38 - 3.44 (m, 3 H) 3.46 - 3.59 (m, 1 H) 4.18 (t, 2 H) 5.15 (s, 1 H) 6.47 (s, 1 H) 6.54 (s, 2 H) 8.01 (d, 1 H) 8.58 (d, 1 H)

### Example 84

### 2'-[6-amino-5-(trifluoromethyl)pyridin-3-yl]-N-[(1R)-1-(4-cyanophenyl)ethyl]-5',6'-dihydrospiro[pyrrolidine-3,4'-pyrrolo[1,2-b]pyrazole]-1-carboxamide (mixture of stereoisomers)

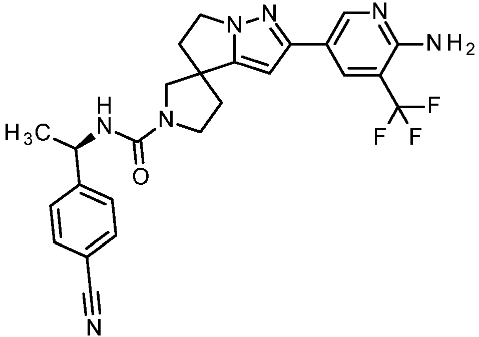

Example 84 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 4-[(1R)-1-aminoethyl]benzonitrile (33.5 mg, 229 µmol).
LC-MS (Method 2): Rt = 0.98 min; MS (ESlpos): m/z = 496 [M+H]⁺

### Example 85

### 2'-[6-amino-5-(trifluoromethyl)pyridin-3-yl]-N-[1-methyl-2,3-dihydro-1H-inden-1-yl]-5',6'-dihydrospiro[pyrrolidine-3,4'-pyrrolo[1,2-b]pyrazole]-1-carboxamide

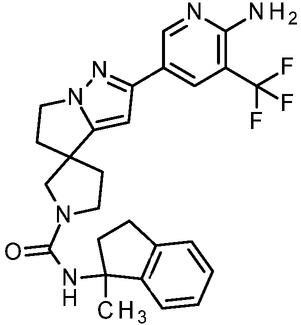

Example 85 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1-methyl-2,3-dihydro-1H-inden-1-amine hydrochloride (45.9 mg, 250 µmol).
1H-NMR (400MHz, DMSO-d6): δ [ppm]= 1.48 (d, 3H), 1.95 - 2.13 (m, 3H), 2.54 - 2.63 (m, 2H), 2.74 - 2.84 (m, 1H), 2.87 - 2.97 (m, 1H), 3.38 - 3.46 (m, 3H), 3.48 - 3.58 (m, 1H), 4.17 (br t, 2H), 5.81 and 5.84 (2s, 1H), 6.43 and 6.46 (2s, 1H), 6.55 (s, 2H), 7.08 - 7.20 (m, 3H), 7.28 - 7.37 (m, 1H), 7.99 and 8.00 (2d, 1H), 8.58 (br s, 1H).
LC-MS (Method 1): Rt = 1.17 min; MS (ESIPos): m/z = 497 [M+H]⁺

### Example 86

### 2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (mixture of isomers)

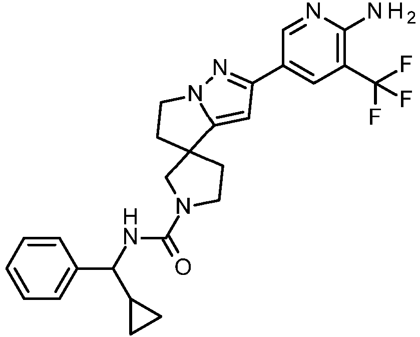

Example 86 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 278 µmol) and (RS)-1-cyclopropyl-1-phenylmethanamine (49.1 mg, 334 µmol).
¹H-NMR (400MHz, DMSO-d₆): δ [ppm]= 0.28 - 0.41 (m, 2H), 0.42 - 0.54 (m, 2H), 1.20 (dt, 1H), 2.07 (br s, 2H), 2.53 - 2.59 (m, 2H), 3.40 - 3.51 (m, 3H), 3.52 - 3.62 (m, 1H), 4.06 (t, 1H), 4.19 (t, 2H), 6.43 (s, 1H), 6.55 (s, 2H), 6.65 (br d, 1H), 7.15 - 7.22 (m, 1H), 7.24 - 7.33 (m, 2H), 7.36 - 7.46 (m, 2H), 8.00 (s, 1H), 8.58 (s, 1H).
LC-MS (method 1): Rt = 1.14 min; MS (ESlneg): m/z = 495 [M-H]⁻

### Example 87

### 2'-[6-amino-5-(trifluoromethyl)pyridin-3-yl]-N-[-2-methyl-1-phenylpropyl]-5',6'-dihydrospiro[pyrrolidine-3,4'-pyrrolo[1,2-b]pyrazole]-1-carboxamide (mixture of isomers)

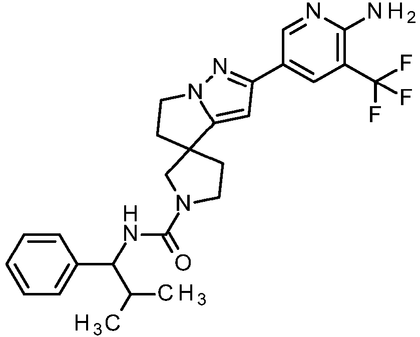

Example 87 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 278 µmol) and 2-methyl-1-phenylpropan-1-amine (49.8 mg, 334 µmol).
¹H-NMR (400MHz, DMSO-d₆): δ [ppm]= 0.66 (dd, 3H), 0.97 (dd, 3H), 1.94 - 2.11 (m, 3H), 2.52 - 2.55 (m, 2H), 3.37 - 3.63 (m, 4H), 4.18 (t, 2H), 4.34 (td, 1H), 6.33 (dd, 1H), 6.37 - 6.44 (m, 1H), 6.55 (s, 2H), 7.14 - 7.22 (m, 1H), 7.23 - 7.37 (m, 4H), 7.96 - 8.00 (m, 1H), 8.54 - 8.58 (m, 1H).
LC-MS (method 1): Rt = 1.19 min; MS (ESlpos): m/z = 499 [M+H]⁺

### Example 88

### 2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide (mixture of stereoisomers)

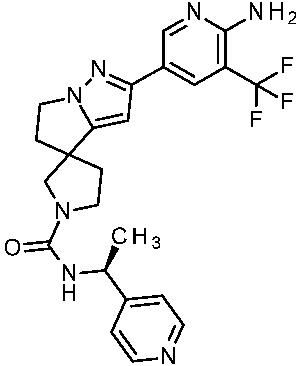

Example 88 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (70.0 mg, 195 µmol) and (1S)-1-(pyridin-4-yl)ethan-1-amine (28.5 mg, 233 µmol).
¹H-NMR (400MHz, DMSO-d6): δ [ppm]= 1.36 and 1.37 (2d, 3H), 2.01 - 2.15 (m, 2H), 2.53 - 2.58 (m, 2H), 3.41 - 3.64 (m, 4H), 4.19 (br t, 2H), 4.77 - 4.86 (m, 1H), 6.48 (s, 1H), 6.55 (s, 2H), 6.60 (br dd, 1H), 7.29 - 7.37 (m, 2H), 8.02 (d, 1H), 8.43 - 8.50 (m, 2H), 8.59 (s, 1H).
LC-MS (Method 1): Rt = 0.90 min; MS (ESlpos): m/z = 472 [M+H]⁺

### Example 89

### 2'-[6-amino-5-(trifluoromethyl)pyridin-3-yl]-N-[2,2,2-trifluoro-1-phenylethyl]-5',6'-dihydrospiro[pyrrolidine-3,4'-pyrrolo[1,2-b]pyrazole]-1-carboxamide (mixture of isomers)

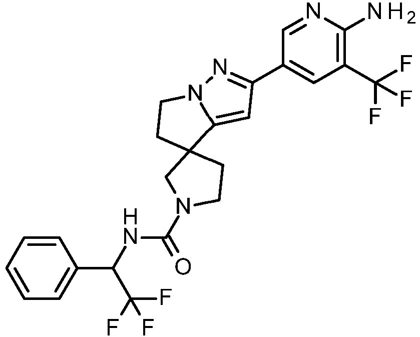

Example 89 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 278 µmol) and 2,2,2-trifluoro-1-phenylethan-1-amine hydrochloride (70.6 mg, 334 µmol).
1H-NMR (400MHz, DMSO-d6): δ [ppm]= 2.09 (br s, 2H), 2.53 - 2.59 (m, 2H), 3.46 - 3.69 (m, 4H), 4.19 (t, 2H), 5.71 (quin, 1H), 6.42 - 6.50 (m, 1H), 6.54 (s, 2H), 7.15 (br d, 1H), 7.34 - 7.50 (m, 3H), 7.57 - 7.65 (m, 2H), 8.00 (br s, 1H), 8.57 (s, 1H).
LC-MS (method 1): Rt = 1.16 min; MS (ESlpos): m/z = 525 [M+H]⁺

### Example 90

### 2'-[6-amino-5-(trifluoromethyl)pyridin-3-yl]-N-[1-(pyrazin-2-yl)ethyl]-5',6'-dihydrospiro[pyrrolidine-3,4'-pyrrolo[1,2-b]pyrazole]-1-carboxamide (mixture of isomers)

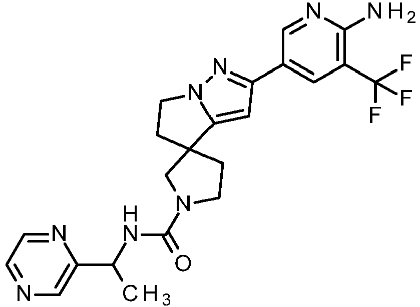

Example 90 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 278 µmol) and 1-(pyrazin-2-yl)ethan-1-amine (41.1 mg, 334 µmol).
¹H-NMR (400MHz, DMSO-d₆): δ [ppm]= 1.43 (dd, 3H), 2.03 - 2.11 (m, 2H), 2.52 - 2.58 (m, 2H), 3.44 - 3.53 (m, 3H), 3.53 - 3.64 (m, 1H), 4.19 (t, 2H), 4.89 - 4.97 (m, 1H), 6.47 (s, 1H), 6.55 (s, 2H), 6.62 (dd, 1H), 8.02 (d, 1H), 8.49 (dd, 1H), 8.56 (ddd, 1H), 8.59 (d, 1H), 8.67 (dd, 1H).
LC-MS (method 1): Rt = 0.88 min; MS (ESlneg): m/z = 471 [M-H]⁻

### Example 91

### 2'-[6-amino-5-(trifluoromethyl)pyridin-3-yl]-N-[(1R)-2-methoxy-1-phenylethyl]-5',6'-dihydrospiro[pyrrolidine-3,4'-pyrrolo[1,2-b]pyrazole]-1-carboxamide (mixture of stereoisomers)

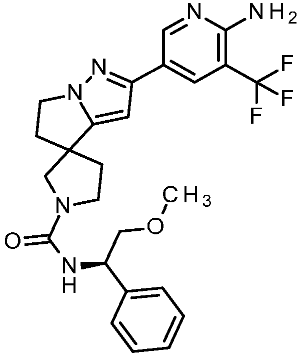

Example 91 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (70.0 mg, 195 µmol) and (1R)-2-methoxy-1-phenylethan-1-amine (35.3 mg, 233 µmol).
1H-NMR (400MHz, DMSO-d6): δ [ppm]= 2.01 - 2.15 (m, 2H), 2.52 - 2.57 (m, 2H), 3.24 and 3.26 (2s, 3H), 3.41 - 3.62 (m, 6H), 4.19 (t, 2H), 4.92 - 5.00 (m, 1H), 6.42 - 6.49 (m, 2H), 6.55 (s, 2H), 7.17 - 7.25 (m, 1H), 7.26 - 7.40 (m, 4H), 8.00 (s, 1H), 8.58 (s, 1H).
LC-MS (Method 1): Rt = 1.03 min; MS (ESlpos): m/z = 501 [M+H]⁺

### Example 92

### (rac)-2'-[6-amino-5-(trifluoromethyl)pyridin-3-yl]-N-[(pyridazin-3-yl)methyl]-5',6'-dihydrospiro[pyrrolidine-3,4'-pyrrolo[1,2-b]pyrazole]-1-carboxamide

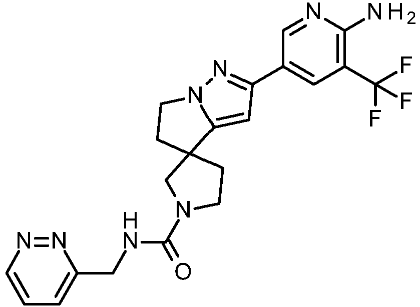

Example 92 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 278 µmol) and 1-(pyridazin-3-yl)methanamine (36.4 mg, 334 µmol).
¹H-NMR (400MHz, DMSO-d₆): δ [ppm]= 2.04 - 2.15 (m, 2H), 2.56 (t, 2H), 3.45 - 3.52 (m, 3H), 3.57 (s, 1H), 4.19 (t, 2H), 4.54 (d, 2H), 6.48 - 6.52 (m, 1H), 6.54 (s, 2H), 7.02 (t, 1H), 7.59 - 7.67 (m, 2H), 8.03 (d, 1H), 8.60 (d, 1H), 9.11 (dd, 1H).
LC-MS (method 1): Rt = 0.80 min; MS (ESlpos): m/z = 459 [M+H]⁺

### Example 93

### (rac)-2'-[6-amino-5-(trifluoromethyl)pyridin-3-yl]-N-[1-(2-cyanophenyl)cyclobutyl]-5',6'-dihydrospiro[pyrrolidine-3,4'-pyrrolo[1,2-b]pyrazole]-1-carboxamide

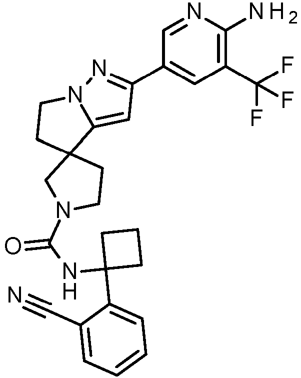

Example 93 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 2-(1-aminocyclobutyl)benzonitrile (43.1 mg, 250 µmol).
1H-NMR (400MHz, DMSO-d6): δ [ppm]= 1.90 - 2.02 (m, 1H), 2.06 - 2.25 (m, 3H), 2.27 - 2.36 (m, 2H), 2.55 - 2.65 (m, 2H), 2.77 - 2.89 (m, 2H), 3.50 - 3.70 (m, 2H), 3.74 - 3.91 (m, 2H), 4.15 - 4.26 (m, 2H), 6.50 - 6.54 (m, 3H), 7.41 - 7.52 (m, 1H), 7.60 - 7.77 (m, 2H), 7.89 and 7.92 (2d, 1H), 8.03 (t, 1H), 8.58 - 8.62 (m, 1H), 10.13 and 10.16 (2s, 1H).
LC-MS (Method 1): Rt = 1.27 min; MS (ESlpos): m/z = 522 [M+H]⁺

### Example 94

### 2'-[6-amino-5-(trifluoromethyl)pyridin-3-yl]-N-[(1S)-2,3-dihydro-1H-inden-1-yl]-5',6'-dihydrospiro[pyrrolidine-3,4'-pyrrolo[1,2-b]pyrazole]-1-carboxamide (mixture of stereoisomers)

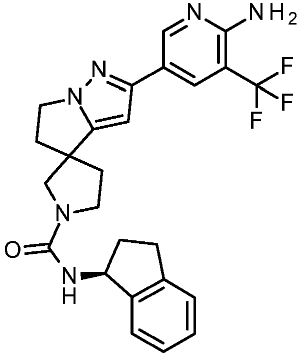

Example 94 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and (1S)-2,3-dihydro-1H-inden-1-amine (44.4 mg, 75 % purity, 250 µmol).
1H-NMR (400MHz, DMSO-d6): δ [ppm]= 1.81 - 1.91 (m, 1H), 2.02 - 2.14 (m, 2H), 2.31 - 2.42 (m, 1H), 2.52 - 2.58 (m, 2H), 2.71 - 2.82 (m, 1H), 2.86 - 2.94 (m, 1H), 3.41 - 3.52 (m, 3H), 3.53 - 3.64 (m, 1H), 4.19 (t, 2H), 5.17 - 5.27 (m, 1H), 6.42 - 6.50 (m, 2H), 6.55 (s, 2H), 7.11 - 7.28 (m, 4H), 8.02 and 8.03 (2d, 1H), 8.60 (2s, 1H).
LC-MS (Method 1): Rt = 1.12 min; MS (ESlpos): m/z = 483 [M+H]⁺

### Example 95

### 2'-[6-amino-5-(trifluoromethyl)pyridin-3-yl]-N-[(1S)-1-(4-fluorophenyl)ethyl]-5',6'-dihydrospiro[pyrrolidine-3,4'-pyrrolo[1,2-b]pyrazole]-1-carboxamide (mixture of stereoisomers)

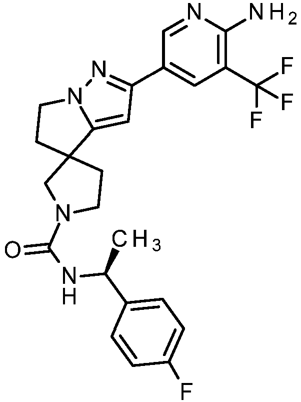

Example 95 was prepared in analogy to Example 31 using (rac)-5-(5',6'-dihydrospiro[pyrrolidine-3,4'-pyrrolo[1,2-b]pyrazol]-2'-yl)-3-(trifluoromethyl)pyridin-2-amine hydrochloride (70.0 mg, 195 µmol) and (1S)-1-(4-fluorophenyl)ethan-1-amine (32.5 mg, 233 µmol).
1H-NMR (400MHz, DMSO-d6): δ [ppm]= 1.35 and 1.36 (2d, 3H), 2.00 - 2.13 (m, 2H), 2.52 - 2.57 (m, 2H), 3.40 - 3.49 (m, 3H), 3.50 - 3.61 (m, 1H), 4.18 (t, 2H), 4.79 - 4.88 (m, 1H), 6.46 (s, 1H), 6.47 and 6.49 (2d, 1H), 6.54 (s, 2H), 7.05 - 7.15 (m, 2H), 7.32 - 7.41 (m, 2H), 8.01 (2s, 1H), 8.59 (s, 1H).
LC-MS (method 2): Rt = 1.04 min; MS (ESlpos): m/z = 489 [M+H]⁺

### Example 96

### 2'-[6-amino-5-(trifluoromethyl)pyridin-3-yl]-N-[(1R)-1-(2-cyanophenyl)ethyl]-5',6'-dihydrospiro[azetidine-3,4'-pyrrolo[1,2-b]pyrazole]-1-carboxamide

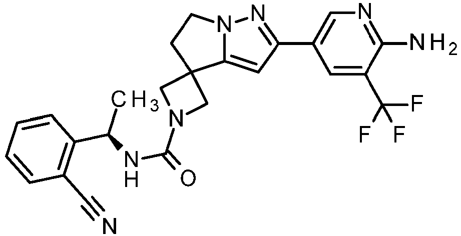

Example 96 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (75.0 mg, 217 µmol) and 2-[(1R)-1-aminoethyl]benzonitrile (34.9 mg, 239 µmol).
LC-MS (method 1): Rt = 0.99 min; MS (ESlpos): m/z = 482 [M+H]⁺
¹H NMR (400 MHz, DMSO-*d*₆) δ ppm 1.39 (d, 3 H) 2.81 - 2.93 (m, 2 H) 3.99 - 4.09 (m, 3 H) 4.13 (t, 2 H) 4.99 - 5.11 (m, 1 H) 6.55 (s, 2 H) 6.72 (s, 1 H) 7.19 (d, 1 H) 7.42 (td, 1 H) 7.62 (d, 2 H) 7.68 - 7.82 (m, 2 H) 8.04 (d, 1 H) 8.62 (d, 1 H)

### Example 97

### 2'-[6-amino-5-(trifluoromethyl)pyridin-3-yl]-N-[(1R)-1-(6-cyanopyridin-3-yl)ethyl]-5',6'-dihydrospiro[azetidine-3,4'-pyrrolo[1,2-b]pyrazole]-1-carboxamide

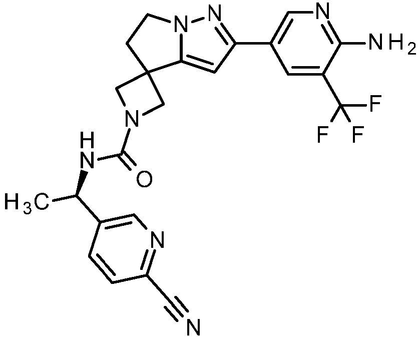

Example 97 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (75.0 mg, 217 µmol) and 5-[(1R)-1-aminoethyl]pyridine-2-carbonitrile (35.1 mg, 239 µmol).
LC-MS (method 1): Rt = 0.90 min; MS (ESlpos): m/z = 483 [M+H]+
¹H NMR (400 MHz, DMSO-*d*₆) δ ppm 1.40 (d, 3 H) 2.85 (t, 2 H) 3.99 - 4.09 (m, 4 H) 4.13 (t, 2 H) 4.91 (quin, 1 H) 6.55 (s, 2 H) 6.73 (s, 1 H) 7.08 (d, 1 H) 7.94 - 8.07 (m, 3 H) 8.62 (d, 1 H) 8.74 (d, 1 H)

### Example 98

### 2'-[6-amino-5-(trifluoromethyl)pyridin-3-yl]-N-[1-(pyridin-2-yl)cyclobutyl]-5',6'-dihydrospiro[azetidine-3,4'-pyrrolo[1,2-b]pyrazole]-1-carboxamide

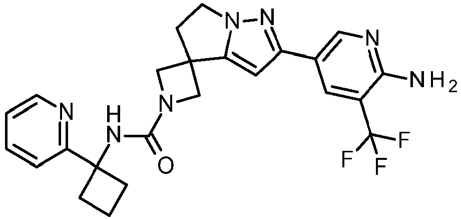

Example 98 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyridin-2-yl)cyclobutan-1-amine (43.1 mg, 291 µmol).
LC-MS (method 2): Rt = 0.71 min; MS (ESlpos): m/z = 484 [M+H]⁺
¹H NMR (400 MHz, DMSO-*d*₆) δ ppm 1.81 - 1.96 (m, 1 H) 1.96 - 2.09 (m, 1 H) 2.37 (ddd, 2 H) 2.55 - 2.64 (m, 2 H) 2.87 (t, 2 H) 4.02 - 4.09 (m, 4 H) 4.14 (t, 2 H) 6.55 (s, 2 H) 6.75 (s, 1 H) 7.15 - 7.24 (m, 2 H) 7.38 (dt, 1 H) 7.73 (td, 1 H) 8.06 (d, 1 H) 8.51 - 8.58 (m, 1 H) 8.64 (d, 1 H)

### Example 99

### 2'-[6-amino-5-(trifluoromethyl)pyridin-3-yl]-N-[(pyridin-4-yl)methyl]-5',6'-dihydrospiro[azetidine-3,4'-pyrrolo[1,2-b]pyrazole]-1-carboxamide

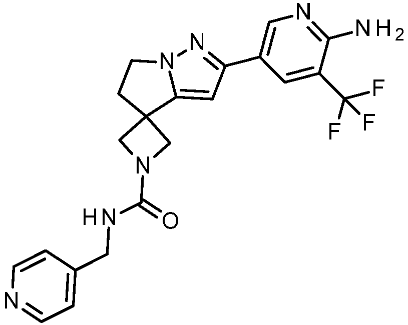

Example 99 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyridin-4-yl)methanamine (31.5 mg, 291 µmol).
LC-MS (Method 1): Rt = 0.82 min; MS (ESlpos): m/z = 444 [M+H]⁺
¹H NMR (400 MHz, DMSO-*d*₆) δ ppm 2.87 (t, 2 H) 4.03 - 4.10 (m, 4 H) 4.14 (t, 2 H) 4.25 (d, 2 H) 6.55 (s, 2 H) 6.75 (s, 1 H) 7.18 (t, 1 H) 7.26 - 7.30 (m, 2 H) 8.05 (d, 1 H) 8.48 - 8.52 (m, 2 H) 8.63 (d, 1 H)

### Example 100

### (3'RS)-2-[6-amino-5-(trifluoromethyl)pyridin-3-yl]-N-[(1R)-1-(4-cyanophenyl)ethyl]-6,7-dihydrospiro[pyrazolo[5,1-c][1,4]oxazine-4,3'-pyrrolidine]-1'-carboxamide (mixture of stereoisomers)

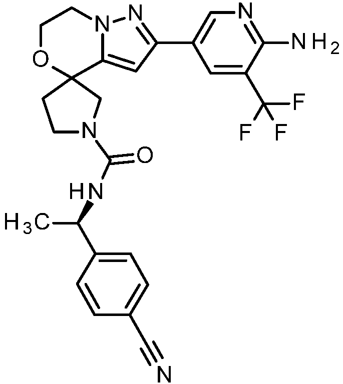

Example 100 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (75 mg, 200 µmol) and 4-[(1R)-1-aminoethyl]benzonitrile (29.9 mg, 205 µmol).
LC-MS (Method 2): Rt = 1.02 min; MS (ESlpos): m/z = 512 [M+H]⁺
¹H NMR (400 MHz, DMSO-*d*₆) δ ppm 1.37 (d, 3 H) 2.19 - 2.29 (m, 1 H) 2.35 (br s, 1 H) 3.38 - 3.54 (m, 2 H) 3.55 - 3.68 (m, 1 H) 3.75 - 3.89 (m, 1 H) 4.04 - 4.20 (m, 4 H) 4.88 (td, 1 H) 6.58 - 6.70 (m, 3 H) 6.76 (d, 1 H) 7.53 (dd, 2 H) 7.78 (dd, 2 H) 8.01 (s, 1 H) 8.58 (br s, 1 H)

### Example 101

### 2'-[6-amino-5-(trifluoromethyl)pyridin-3-yl]-N-[(1R)-1-(3-cyanopyridin-2-yl)ethyl]-5',6'-dihydrospiro[azetidine-3,4'-pyrrolo[1,2-b]pyrazole]-1-carboxamide

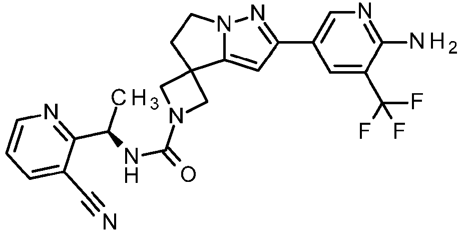

Example 101 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (75.0 mg, 217 µmol) and 2-[(1R)-1-aminoethyl]pyridine-4-carbonitrile-hydrochloride salt (43.8 mg, 239 µmol).
LC-MS (method 1): Rt = 0.88 min; MS (ESlpos): m/z = 483 [M+H]⁺
¹H NMR (400 MHz, DMSO-*d*₆) δ ppm 1.41 (d, 3 H) 2.85 (t, 2 H) 3.99 - 4.06 (m, 3 H) 4.06 - 4.10 (m, 1 H) 4.13 (t, 2 H) 4.90 (quin, 1 H) 6.55 (s, 2 H) 6.73 (s, 1 H) 7.01 (d, 1 H) 8.04 (d, 1 H) 8.26 (t, 1 H) 8.62 (d, 1 H) 8.85 (d, 1 H) 8.90 (d, 1 H)

### Example 102

### 2'-[6-amino-5-(trifluoromethyl)pyridin-3-yl]-N-[(1R)-1-(4-cyanophenyl)ethyl]-5',6'-dihydrospiro[azetidine-3,4'-pyrrolo[1,2-b]pyrazole]-1-carboxamide

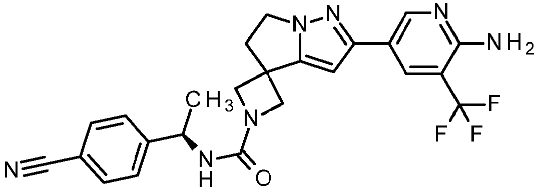

Example 102 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (75.0 mg, 217 µmol) and 4-[(1R)-1-aminoethyl]benzonitrile (34.9 mg, 239 µmol).
LC-MS (Method 1): Rt = 1.01 min; MS (ESlpos): m/z = 482 [M+H]⁺
¹H NMR (400 MHz, DMSO-*d*₆) δ ppm 1.36 (d, 3 H) 2.85 (t, 2 H) 3.98 - 4.09 (m, 4 H) 4.13 (t, 2 H) 4.85 (quin, 1 H) 6.55 (s, 2 H) 6.73 (s, 1 H) 7.02 (d, 1 H) 7.53 (d, 2 H) 7.80 (d, 2 H) 8.04 (d, 1 H) 8.62 (d, 1 H)

### Example 103

### 2'-[6-amino-5-(trifluoromethyl)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide

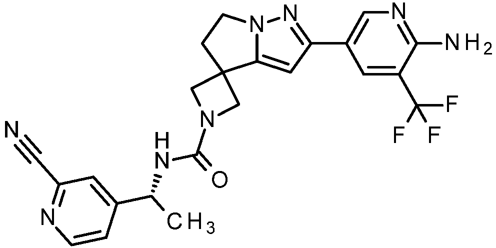

Example 103 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (75.0 mg, 217 µmol) and 4-[(1R)-1-aminoethyl]pyridine-2-carbonitrile (35.1 mg, 239 µmol).
LC-MS (Method 1): Rt = 0.93 min; MS (ESlpos): m/z = 483 [M+H]⁺
¹H NMR (400 MHz, DMSO-*d*₆) δ ppm 1.38 (d, 3 H) 2.86 (t, 2 H) 4.01 - 4.07 (m, 3 H) 4.07 - 4.18 (m, 3 H) 4.85 (quin, 1 H) 6.55 (s, 2 H) 6.73 (s, 1 H) 7.07 (d, 1 H) 7.69 (dd, 1 H) 7.99 - 8.01 (m, 1 H) 8.04 (d, 1 H) 8.62 (d, 1 H) 8.70 (d, 1 H)

### Example 104

### 2'-[6-amino-5-(trifluoromethyl)pyridin-3-yl]-N-[(1R)-1-(2-chloro-4-cyanophenyl)ethyl]-5',6'-dihydrospiro[azetidine-3,4'-pyrrolo[1,2-b]pyrazole]-1-carboxamide

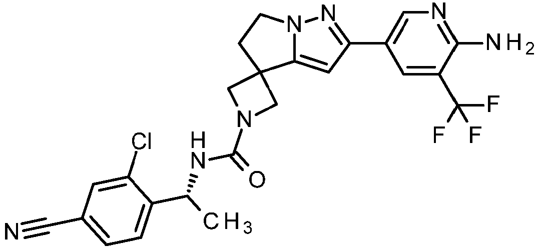

Example 104 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (75.0 mg, 217 µmol) and 4-[(1R)-1-aminoethyl]-3-chlorobenzonitrile (43.1 mg, 239 µmol).
LC-MS (method 1): Rt = 1.06 min; MS (ESlpos): m/z = 516 [M+H]⁺
¹H NMR (400 MHz, DMSO-*d*₆) δ ppm 1.33 (d, 3 H) 2.86 (t, 2 H) 4.00 - 4.06 (m, 3 H) 4.06 - 4.10 (m, 1 H) 4.13 (t, 2 H) 5.11 (quin, 1 H) 6.55 (s, 2 H) 6.73 (s, 1 H) 7.20 (d, 1 H) 7.67 (d, 1 H) 7.87 (dd, 1 H) 8.01 (d, 1 H) 8.04 (d, 1 H) 8.62 (d, 1 H)

### Example 105

### 2'-[6-amino-5-(trifluoromethyl)pyridin-3-yl]-N-[2-(2-fluorophenyl)propan-2-yl]-5',6'-dihydrospiro[azetidine-3,4'-pyrrolo[1,2-b]pyrazole]-1-carboxamide

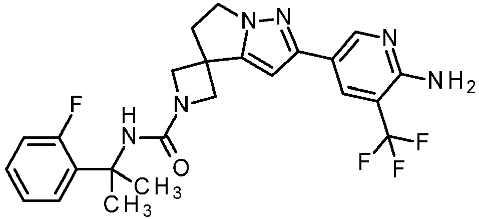

Example 105 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(2-fluorophenyl)propan-2-amine-hydrochloride salt (55.2 mg, 291 µmol).

### Example 106

### 2'-[6-amino-5-(trifluoromethyl) pyridin-3-yl]-N-[1-(1-ethyl-3,5-dimethyl-1H-pyrazol-4-yl)ethyl]-5',6'-dihydrospiro[azetidine-3,4'-pyrrolo[1,2-b]pyrazole]-1-carboxamide

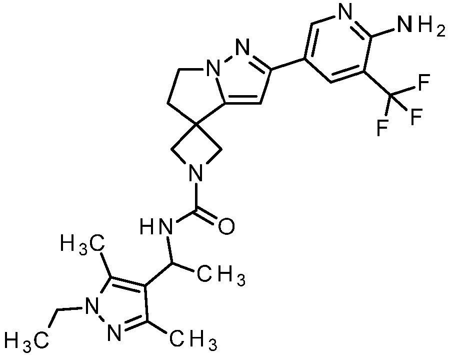

Example 106 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(1-ethyl-3,5-dimethyl-1H-pyrazol-4-yl)ethan-1-amine (48.7 mg, 291 µmol).
LC-MS (method 2): Rt = 0.85 min; MS (ESlpos): m/z = 503 [M+H]⁺
¹H NMR (400 MHz, DMSO-*d*₆) δ ppm 1.20 - 1.26 (m, 3 H) 1.34 (d, 3 H) 2.14 (s, 3 H) 2.21 (s, 3 H) 2.79 - 2.86 (m, 2 H) 3.87 - 4.02 (m, 6 H) 4.11 (t, 2 H) 4.62 - 4.73 (m, 1 H) 6.50 - 6.60 (m, 3 H) 6.68 - 6.72 (m, 1 H) 8.03 (d, 1 H) 8.61 (d, 1 H)

### Example 107

### 2'-[6-amino-5-(trifluoromethyl)pyridin-3-yl]-N-[2-(pyridin-2-yl)propan-2-yl]-5',6'-dihydrospiro[azetidine-3,4'-pyrrolo[1,2-b]pyrazole]-1-carboxamide

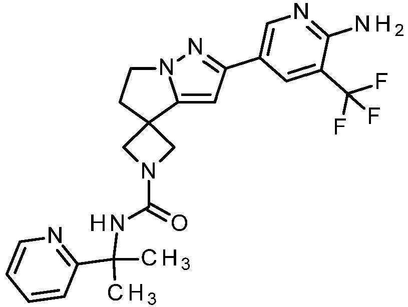

Example 107 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(pyridin-2-yl)propan-2-amine (39.6 mg, 291 µmol).
LC-MS (Method 2): Rt = 0.75 min; MS (ESlpos): m/z = 472 [M+H]⁺
¹H NMR (400 MHz, DMSO-*d*₆) δ ppm 1.57 (s, 6 H) 2.86 (t, 2 H) 4.00 - 4.08 (m, 4 H) 4.14 (t, 2 H) 6.55 (s, 2 H) 6.71 - 6.76 (m, 2 H) 7.20 (ddd, 1 H) 7.45 (d, 1 H) 7.74 (td, 1 H) 8.05 (d, 1 H) 8.45 - 8.49 (m, 1 H) 8.64 (d, 1 H)

### Example 108

### 2'-[6-amino-5-(trifluoromethyl)pyridin-3-yl]-N-[1-(1-methyl-1H-pyrazol-4-yl)ethyl]-5',6'-dihydrospiro[azetidine-3,4'-pyrrolo[1,2-b]pyrazole]-1-carboxamide

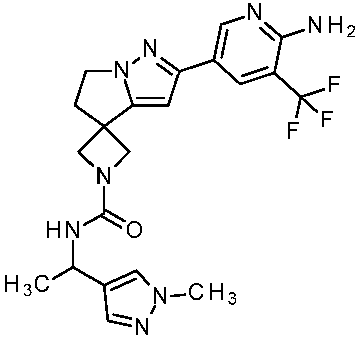

Example 108 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(1-methyl-1H-pyrazol-4-yl)ethan-1-amine (36.4 mg, 291 µmol).
LC-MS (Method 2): Rt = 0.88 min; MS (ESlpos): m/z = 461 [M+H]⁺
¹H NMR (400 MHz, DMSO-*d*₆) δ ppm 1.33 (d, 3 H) 2.84 (t, 2 H) 3.74 - 3.82 (m, 3 H) 3.95 - 4.04 (m, 4 H) 4.12 (t, 2 H) 4.70 - 4.81 (m, 1 H) 6.54 (s, 2 H) 6.63 (d, 1 H) 6.72 (s, 1 H) 7.31 (s, 1 H) 7.53 (s, 1 H) 8.04 (d, 1 H) 8.62 (d, 1 H)

### Example 109

### 2'-[6-amino-5-(trifluoromethyl)pyridin-3-yl]-N-[1-(1,3,4-trimethyl-1H-pyrazol-5-yl)ethyl]-5',6'-dihydrospiro[azetidine-3,4'-pyrrolo[1,2-b]pyrazole]-1-carboxamide

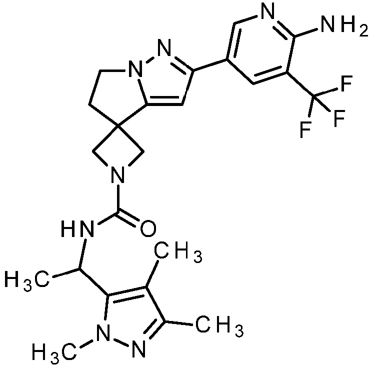

Example 109 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (50.5 mg, 163 µmol) and 1-(1,3,4-trimethyl-1H-pyrazol-5-yl)ethan-1-amine (30.0 mg, 196 µmol).
LC-MS (Method 2): Rt = 0.89 min; MS (ESlpos): m/z = 489 [M+H]⁺
¹H NMR (400 MHz, DMSO-*d*₆) δ ppm 1.40 (d, 3 H) 1.97 (d, 6 H) 2.83 (t, 2 H) 3.71 (s, 3 H) 3.93 - 4.05 (m, 4 H) 4.12 (t, 2 H) 4.88 (quin, 1 H) 6.54 (s, 2 H) 6.70 (s, 1 H) 6.91 (d, 1 H) 8.03 (d, 1 H) 8.61 (d, 1 H)

### Example 110

### 2'-[6-amino-5-(trifluoromethyl)pyridin-3-yl]-N-{1-[3-(difluoromethyl)-1-methyl-1H-pyrazol-4-yl]ethyl}-5',6'-dihydrospiro[azetidine-3,4'-pyrrolo[1,2-b]pyrazole]-1-carboxamide

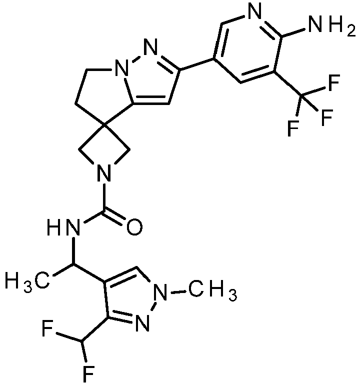

Example 110 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-[3-(difluoromethyl)-1-methyl-1H-pyrazol-4-yl]ethan-1-amine (51.0 mg, 291 µmol).
LC-MS (Method 2): Rt = 0.93 min; MS (ESlpos): m/z = 511 [M+H]⁺
¹H NMR (400 MHz, DMSO-*d*₆) δ ppm 1.35 (d, 3 H) 2.83 (t, 2 H) 3.84 (s, 3 H) 3.95 - 4.04 (m, 4 H) 4.12 (t, 2 H) 4.90 (quin, 1 H) 6.55 (s, 2 H) 6.68 - 6.75 (m, 2 H) 6.98 (s, 1 H) 7.70 (s, 1 H) 8.04 (d, 1 H) 8.61 (d, 1 H)

### Example 111

### (rac)-2-[6-amino-5-(trifluoromethyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide

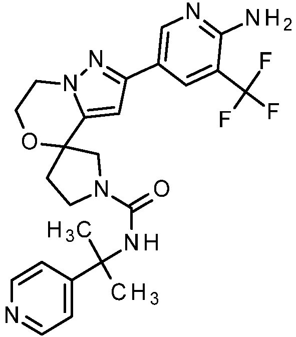

Example 111 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (100 mg, 266 µmol) and 2-(pyridin-4-yl)propan-2-amine (43.5 mg, 319 µmol).
¹H NMR (400 MHz, DMSO-*d*₆) δ ppm 1.54 (s, 6 H) 2.18 - 2.30 (m, 1 H) 2.31 - 2.38 (m, 1 H) 3.36 - 3.53 (m, 2 H) 3.61 (br t, 1 H) 3.80 (br d, 1 H) 4.07 - 4.18 (m, 4 H) 6.25 (s, 1 H) 6.60 (s, 2 H) 6.77 (s, 1 H) 7.30 - 7.33 (m, 2 H) 8.02 (d, 1 H) 8.41 - 8.48 (m, 2 H) 8.54 - 8.60 (m, 1 H)
LC-MS (Method 1): Rt = 0.94 min; MS (ESlpos): m/z = 502 [M+H]⁺

### Example 112

### (rac)-2-[6-amino-5-(trifluoromethyl)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide

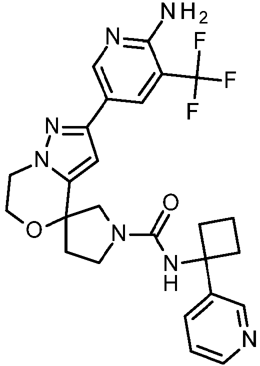

Example 112 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (100 mg, 266 µmol) and 1-(pyridin-3-yl)cyclobutan-1-amine (47.3 mg, 319 µmol).
LC-MS (Method 1): Rt = 0.94 min; MS (ESlpos): m/z = 514 [M+H]⁺
¹H NMR (400 MHz, DMSO-*d*₆) δ ppm 1.74 - 1.86 (m, 1 H) 1.97 - 2.09 (m, 1 H) 2.22 (br d, 1 H) 2.30 - 2.37 (m, 1 H) 2.38 - 2.48 (m, 4 H) 3.36 - 3.43 (m, 1 H) 3.47 (br d, 1 H) 3.58 (br t, 1 H) 3.77 (br d, 1 H) 4.05 - 4.17 (m, 4 H) 6.60 (s, 2 H) 6.75 (s, 1 H) 6.83 (s, 1 H) 7.28 - 7.36 (m, 1 H) 7.76 - 7.82 (m, 1 H) 8.01 (d, 1 H) 8.38 (dd, 1 H) 8.57 (d, 1 H) 8.64 (dd, 1 H)

### Example 113

### 2'-[6-amino-5-(trifluoromethyl)pyridin-3-yl]-N-[1-(1-ethyl-5-methyl-1H-pyrazol-4-yl)ethyl]-5',6'-dihydrospiro[azetidine-3,4'-pyrrolo[1,2-b]pyrazole]-1-carboxamide

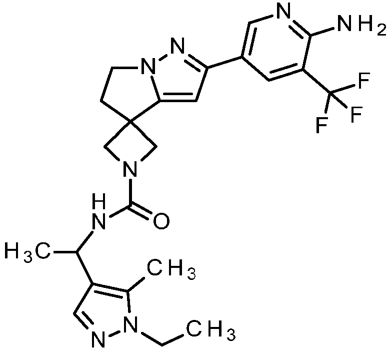

Example 113 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(1-ethyl-5-methyl-1H-pyrazol-4-yl)ethan-1-amine (44.6 mg, 291 µmol).
LC-MS (Method 2): Rt = 0.89 min; MS (ESlpos): m/z = 489 [M+H]⁺
¹H NMR (400 MHz, DMSO-*d*₆) δ ppm 1.26 (t, 3 H) 1.33 (d, 3 H) 2.21 (s, 3 H) 2.82 (t, 2 H) 3.93 - 4.04 (m, 6 H) 4.12 (t, 2 H) 4.68 - 4.78 (m, 1 H) 6.54 (s, 2 H) 6.60 (d, 1 H) 6.70 (s, 1 H) 7.31 (s, 1 H) 8.04 (d, 1 H) 8.61 (d, 1 H)

### Example 114

### 2'-[6-amino-5-(trifluoromethyl)pyridin-3-yl]-N-[1-(1,5-dimethyl-1H-pyrazol-4-yl)ethyl]-5',6'-dihydrospiro[azetidine-3,4'-pyrrolo[1,2-b]pyrazole]-1-carboxamide

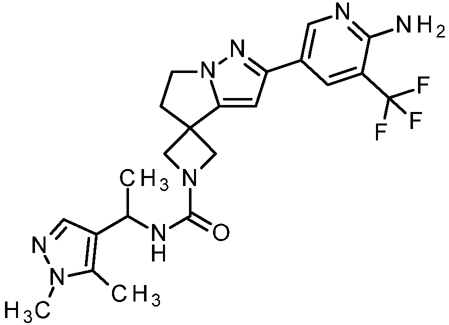

Example 114 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(1,5-dimethyl-1H-pyrazol-4-yl)ethan-1-amine (40.5 mg, 291 µmol).
LC-MS (Method 2): Rt = 0.85 min; MS (ESlpos): m/z = 475 [M+H]⁺
¹H NMR (400 MHz, DMSO-*d*₆) δ ppm 1.33 (d, 3 H) 2.20 (s, 3 H) 2.82 (t, 2 H) 3.67 (s, 3 H) 3.92 - 4.02 (m, 4 H) 4.12 (t, 2 H) 4.67 - 4.77 (m, 1 H) 6.54 (s, 2 H) 6.59 (d, 1 H) 6.70 (s, 1 H) 7.28 (s, 1 H) 8.04 (d, 1 H) 8.62 (d, 1 H)

### Example 115

### 2'-[6-amino-5-(trifluoromethyl)pyridin-3-yl]-N-[1-(1,3,5-trimethyl-1H-pyrazol-4-yl)ethyl]-5',6'-dihydrospiro[azetidine-3,4'-pyrrolo[1,2-b]pyrazole]-1-carboxamide

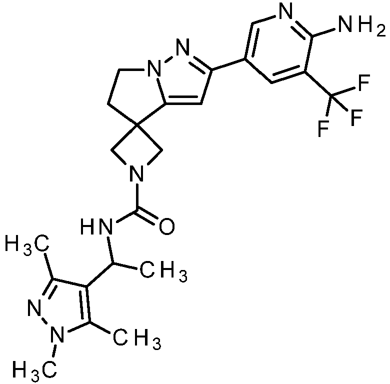

Example 115 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(1,3,5-trimethyl-1H-pyrazol-4-yl)ethan-1-amine (44.6 mg, 291 µmol).
LC-MS (Method 2): Rt = 0.86 min; MS (ESlpos): m/z = 489 [M+H]⁺
¹H NMR (500 MHz, DMSO-*d*₆) δ ppm 1.34 (d, 3 H) 2.13 (s, 3 H) 2.20 (s, 3 H) 2.82 (t, 2 H) 3.57 (s, 3 H) 3.92 - 4.01 (m, 4 H) 4.12 (t, 2 H) 4.66 (quin, 1 H) 6.53 (s, 2 H) 6.58 (d, 1 H) 6.68 (s, 1 H) 8.03 (d, 1 H) 8.61 (d, 1 H)

### Example 116

### 2'-[6-amino-5-(trifluoromethyl)pyridin-3-yl]-N-[(1R)-1-(pyridin-3-yl)ethyl]-5',6'-dihydrospiro[azetidine-3,4'-pyrrolo[1,2-b]pyrazole]-1-carboxamide

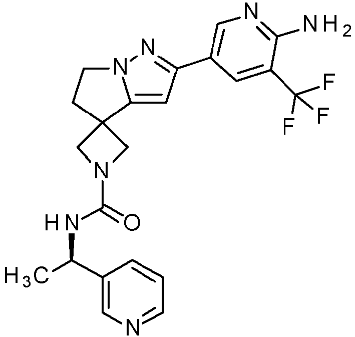

Example 116 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1R)-1-(pyridin-3-yl)ethan-1-amine-hydrochloride salt (56.8 mg, 291 µmol).
LC-MS (Method 2): Rt = 0.68 min; MS (ESlpos): m/z = 458 [M+H]⁺
¹H NMR (400 MHz, DMSO-*d*₆) δ ppm 1.39 (d, 3 H) 2.85 (t, 2 H) 3.99 - 4.07 (m, 4 H) 4.13 (t, 2 H) 4.85 (t, 1 H) 6.49 - 6.61 (m, 2 H) 6.72 (s, 1 H) 6.95 - 7.04 (m, 1 H) 7.32 - 7.38 (m, 1 H) 7.74 (dt, 1 H) 8.04 (d, 1 H) 8.43 (dd, 1 H) 8.56 (d, 1 H) 8.62 (d, 1 H)

### Example 117

### 2-[6-amino-5-(trifluoromethyl)pyridin-3-yl]-N-[(1R)-1-(2-cyanopyridin-4-yl)ethyl]-6,7-dihydrospiro[pyrazolo[5,1-c][1,4]oxazine-4,3'-pyrrolidine]-1'-carboxamide (mixture of stereoisomers)

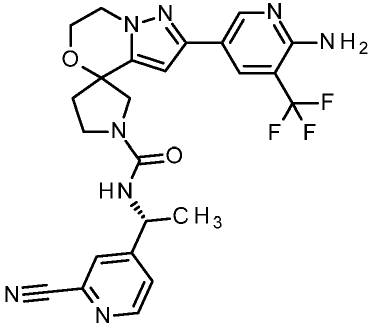

Example 117 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (75 mg, 200 µmol) and 4-[(1R)-1-aminoethyl]pyridine-2-carbonitrile (30.1 mg, 205 µmol).
LC-MS (Method 2): Rt = 0.94 min; MS (ESlpos): m/z = 513 [M+H]⁺
¹H NMR (400 MHz, DMSO-*d*₆) δ ppm 1.38 (d, 3 H) 2.19 - 2.32 (m, 1 H) 2.34 - 2.41 (m, 1 H) 3.38 - 3.71 (m, 3 H) 3.76 - 3.89 (m, 1 H) 4.01 - 4.18 (m, 4 H) 4.80 - 4.93 (m, 1 H) 6.60 (s, 2 H) 6.66 - 6.74 (m, 1 H) 6.76 (d, 1 H) 7.69 (ddd, 1 H) 7.99 - 8.03 (m, 2 H) 8.58 (s, 1 H) 8.66 - 8.70 (m, 1 H)

### Example 118

### 2-[6-amino-5-(trifluoromethyl)pyridin-3-yl]-N-[(1R)-1-(3-cyanopyridin-2-yl)ethyl]-6,7-dihydrospiro[pyrazolo[5,1-c][1,4]oxazine-4,3'-pyrrolidine]-1'-carboxamide (mixture of stereoisomers)

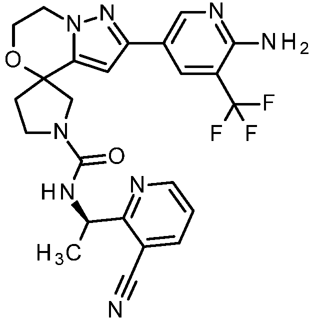

Example 118 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (75 mg, 200 µmol) and 2-[(1R)-1-aminoethyl]pyridine-3-carbonitrile-hydrochloride salt (37.6 mg, 205 µmol).
LC-MS (Method 2): Rt = 0.94 min; MS (ESlpos): m/z = 513 [M+H]⁺
¹H NMR (400 MHz, DMSO-*d*₆) δ ppm 1.42 (dd, 3 H) 2.17 - 2.28 (m, 1 H) 2.30 - 2.39 (m, 1 H) 3.37 - 3.52 (m, 2 H) 3.61 (dt, 1 H) 3.81 (br dd, 1 H) 4.00 - 4.18 (m, 4 H) 5.03 - 5.18 (m, 1 H) 6.60 (s, 2 H) 6.69 (dd, 1 H) 6.72 - 6.78 (m, 1 H) 7.47 (ddd, 1 H) 8.01 (d, 1 H) 8.26 (dd, 1 H) 8.58 (s, 1 H) 8.80 (dt, 1 H)

### Example 119

### 2-[6-amino-5-(trifluoromethyl)pyridin-3-yl]-N-[(1R)-1-(6-cyanopyridin-3-yl)ethyl]-6,7-dihydrospiro[pyrazolo[5,1-c][1,4]oxazine-4,3'-pyrrolidine]-1'-carboxamide (mixture of stereoisomers)

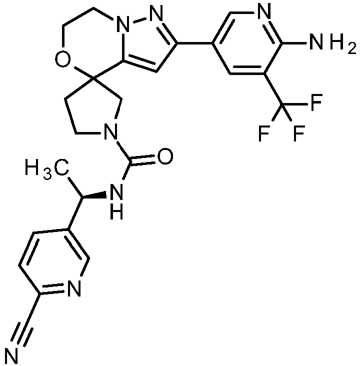

Example 119 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (75 mg, 200 µmol) and 5-[(1R)-1-aminoethyl]pyridine-2-carbonitrile (30.1 mg, 205 µmol).
LC-MS (Method 2): Rt = 0.94 min; MS (ESlpos): m/z = 513 [M+H]⁺
¹H NMR (400 MHz, DMSO-*d*₆) δ ppm 1.41 (d, 3 H) 2.17 - 2.29 (m, 1 H) 2.31 - 2.41 (m, 1 H) 3.37 - 3.67 (m, 3 H) 3.75 - 3.87 (m, 1 H) 4.04 - 4.18 (m, 4 H) 4.88 - 4.97 (m, 1 H) 6.60 (s, 2 H) 6.66 - 6.74 (m, 1 H) 6.76 (d, 1 H) 7.95 - 8.04 (m, 3 H) 8.58 (t, 1 H) 8.74 (d, 1 H)

### Example 120

### 2'-[6-amino-5-(trifluoromethyl)pyridin-3-yl]-N-[(pyridin-2-yl)methyl]-5',6'-dihydrospiro[azetidine-3,4'-pyrrolo[1,2-b]pyrazole]-1-carboxamide

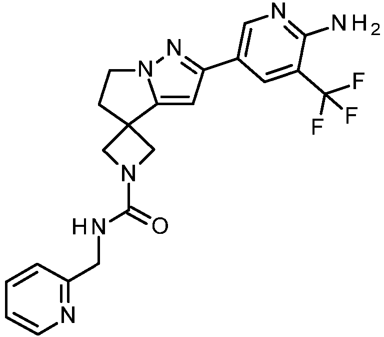

Example 120 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyridin-2-yl)methanamine (31.5 mg, 291 µmol).
LC-MS (OAo1b02): Rt = 0.86 min; MS (ESlpos): m/z = 444 [M+H]⁺
¹H NMR (400 MHz, DMSO-*d*₆) δ ppm 2.87 (t, 2 H) 4.03 - 4.10 (m, 4 H) 4.14 (t, 2 H) 4.32 (d, 2 H) 6.55 (s, 2 H) 6.75 (s, 1 H) 7.16 (t, 1 H) 7.25 (ddd, 1 H) 7.32 (d, 1 H) 7.77 (td, 1 H) 8.05 (d, 1 H) 8.46 - 8.52 (m, 1 H) 8.63 (d, 1 H)

### Example 121

### (rac)-2-[6-amino-5-(trifluoromethyl)pyridin-3-yl]-N-[(1R)-1-(2-cyanophenyl)ethyl]-6,7-dihydrospiro[pyrazolo[5,1-c][1,4]oxazine-4,3'-pyrrolidine]-1'-carboxamide

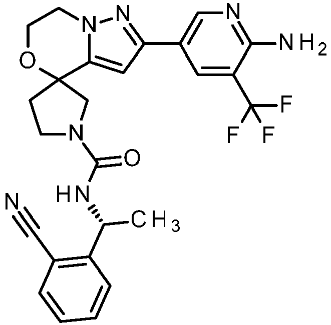

Example 121 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (75 mg, 200 µmol) and 2-[(1R)-1-aminoethyl]benzonitrile (29.9 mg, 205 µmol).
LC-MS (Method 2): Rt = 1.01 min; MS (ESlpos): m/z = 512 [M+H]⁺
¹H NMR (400 MHz, DMSO-*d*₆) δ ppm 1.40 (d, 3 H) 2.19 - 2.29 (m, 1 H) 2.31 - 2.41 (m, 1 H) 3.39 - 3.69 (m, 3 H) 3.82 (dd, 1 H) 4.04 - 4.20 (m, 4 H) 5.05 (quin, 1 H) 6.60 (s, 2 H) 6.73 - 6.84 (m, 2 H) 7.40 (tt, 1 H) 7.61 - 7.66 (m, 1 H) 7.68 (ddd, 1 H) 7.76 (dd, 1 H) 8.01 (t, 1 H) 8.56 - 8.60 (m, 1 H)

### Example 122

### 2'-[6-amino-5-(trifluoromethyl)pyridin-3-yl]-N-[(1R)-1-(4-fluorophenyl)ethyl]-5',6'-dihydrospiro[azetidine-3,4'-pyrrolo[1,2-b]pyrazole]-1-carboxamide

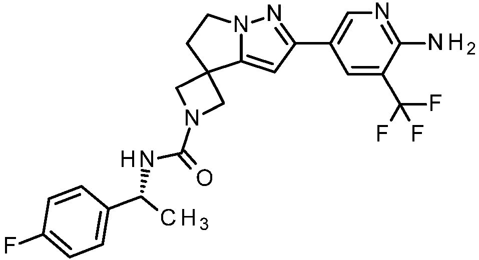

Example 122 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1R)-1-(4-fluorophenyl)ethan-1-amine (40.5 mg, 291 µmol).
LC-MS (Method 2): Rt = 1.05 min; MS (ESlpos): m/z = 475 [M+H]⁺
¹H NMR (400 MHz, DMSO-*d*₆) δ ppm 1.35 (d, 3 H) 2.84 (t, 2 H) 3.97 - 4.07 (m, 4 H) 4.13 (t, 2 H) 4.81 (quin, 1 H) 6.57 (br s, 2 H) 6.72 (s, 1 H) 6.90 (d, 1 H) 7.14 (t, 2 H) 7.37 (dd, 2 H) 8.05 (d, 1 H) 8.62 (d, 1 H)

### Example 123

### 2'-[6-amino-5-(trifluoromethyl)pyridin-3-yl]-N-[2-(4-fluorophenyl)propan-2-yl]-5',6'-dihydrospiro[azetidine-3,4'-pyrrolo[1,2-b]pyrazole]-1-carboxamide

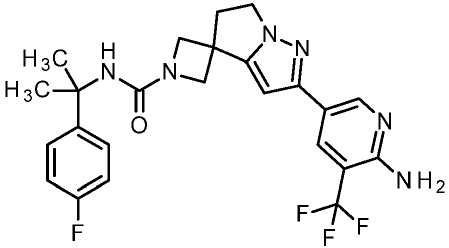

Example 123 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(4-fluorophenyl)propan-2-amine-hydrochloride salt (55.2 mg, 291 µmol).
LC-MS (Method 2): Rt = 1.11 min; MS (ESlpos): m/z = 489 [M+H]⁺
¹H NMR (400 MHz, DMSO-*d*₆) δ ppm 1.55 (s, 6 H) 2.80 - 2.89 (m, 2 H) 3.94 - 4.08 (m, 4 H) 4.13 (t, 2 H) 6.50 - 6.61 (m, 3 H) 6.71 (s, 1 H) 7.06 - 7.13 (m, 2 H) 7.34 - 7.41 (m, 2 H) 8.05 (d, 1 H) 8.63 (d, 1 H)

### Example 124

### 2'-[6-amino-5-(trifluoromethyl)pyridin-3-yl]-N-[2-(pyridin-3-yl)propan-2-yl]-5',6'-dihydrospiro[azetidine-3,4'-pyrrolo[1,2-b]pyrazole]-1-carboxamide

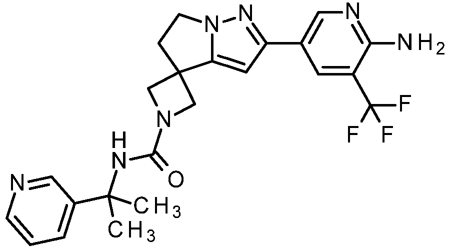

Example 124 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(pyridin-3-yl)propan-2-amine (39.6 mg, 291 µmol).
LC-MS (Method 2): Rt = 0.70 min; MS (ESlpos): m/z = 472 [M+H]⁺
¹H NMR (400 MHz, DMSO-*d*₆) δ ppm 1.58 (s, 6 H) 2.85 (t, 2 H) 3.99 - 4.08 (m, 4 H) 4.13 (t, 2 H) 6.55 (s, 2 H) 6.68 (s, 1 H) 6.72 (s, 1 H) 7.29 - 7.35 (m, 1 H) 7.70 - 7.75 (m, 1 H) 8.06 (d, 1 H) 8.38 (dd, 1 H) 8.60 (d, 1 H) 8.64 (d, 1 H)

### Example 125

### 2'-[6-amino-5-(trifluoromethyl)pyridin-3-yl]-N-[(2-fluorophenyl)methyl]-5',6'-dihydrospiro[azetidine-3,4'-pyrrolo[1,2-b]pyrazole]-1-carboxamide

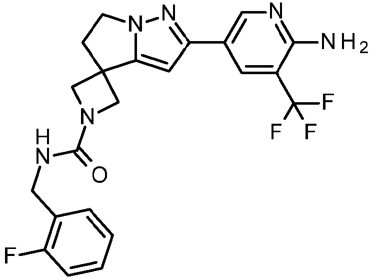

Example 125 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(2-fluorophenyl)methanamine (36.4 mg, 291 µmol).
LC-MS (Method 1): Rt = 1.03 min; MS (ESlpos): m/z = 461 [M+H]⁺
¹H NMR (400 MHz, DMSO-*d*₆) δ ppm 2.86 (t, 2 H) 4.00 - 4.09 (m, 4 H) 4.21 - 4.31 (m, 4 H) 6.55 (s, 2 H) 6.74 (s, 1 H) 7.07 (t, 1 H) 7.24 - 7.33 (m, 3 H) 7.37 (td, 1 H) 8.04 (d, 1 H) 8.62 (d, 1 H)

### Example 126

### 2'-[6-amino-5-(trifluoromethyl)pyridin-3-yl]-N-[1-(pyridin-3-yl)cyclobutyl]-5',6'-dihydrospiro[azetidine-3,4'-pyrrolo[1,2-b]pyrazole]-1-carboxamide

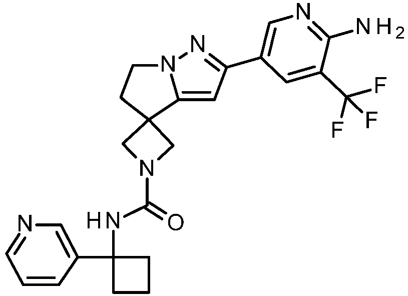

Example 126 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyridin-3-yl)cyclobutan-1-amine-hydrochloride salt (64.3 mg, 291 µmol).
LC-MS (method 2): Rt = 0.75 min; MS (ESlpos): m/z = 484 [M+H]⁺
¹H NMR (400 MHz, DMSO-*d*₆) δ ppm 1.13 (d, 2 H) 1.75 - 1.90 (m, 1 H) 1.96 - 2.11 (m, 1 H) 2.38 - 2.46 (m, 2 H) 2.84 (t, 2 H) 3.97 - 4.06 (m, 4 H) 4.12 (t, 2 H) 6.55 (s, 2 H) 6.71 (s, 1 H) 7.20 (s, 1 H) 7.34 (ddd, 1 H) 7.75 - 7.83 (m, 1 H) 8.04 (d, 1 H) 8.40 (dd, 1 H) 8.61 - 8.67 (m, 2 H)

### Example 127

### 2'-[6-amino-5-(trifluoromethyl)pyridin-3-yl]-N-[(4-fluorophenyl)methyl]-5',6'-dihydrospiro[azetidine-3,4'-pyrrolo[1,2-b]pyrazole]-1-carboxamide

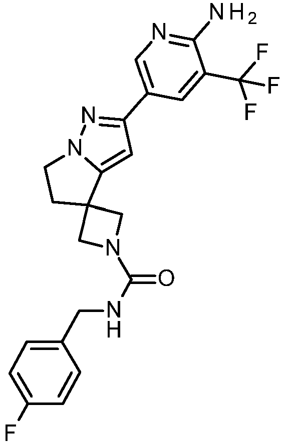

Example 127 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(4-fluorophenyl)methanamine (36.4 mg, 291 µmol).
LC-MS (Method 1): Rt = 1.02 min; MS (ESlpos): m/z = 461 [M+H]⁺
¹H NMR (400 MHz, DMSO-*d*₆) δ ppm 2.85 (t, 2 H) 4.00 - 4.08 (m, 4 H) 4.13 (t, 2 H) 4.20 (d, 2 H) 6.55 (s, 2 H) 6.73 (s, 1 H) 7.07 - 7.11 (m, 1 H) 7.14 (t, 2 H) 7.31 (dd, 2 H) 8.04 (d, 1 H) 8.62 (d, 1 H)

### Example 128

### 2'-[6-amino-5-(trifluoromethyl)pyridin-3-yl]-N-[2-(pyridin-4-yl)propan-2-yl]-5',6'-dihydrospiro[azetidine-3,4'-pyrrolo[1,2-b]pyrazole]-1-carboxamide

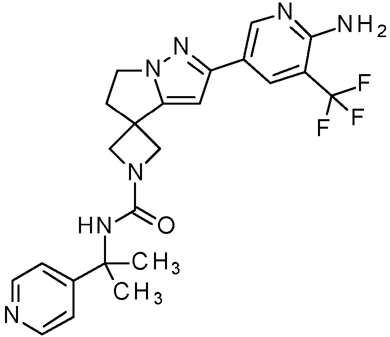

Example 128 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(pyridin-4-yl)propan-2-amine (39.6 mg, 291 µmol).
LC-MS (Method 1): Rt = 0.93 min; MS (ESlpos): m/z = 472 [M+H]⁺
¹H NMR (400 MHz, DMSO-*d*₆) δ ppm 1.53 (s, 6 H) 2.85 (t, 2 H) 3.99 - 4.07 (m, 4 H) 4.13 (t, 2 H) 6.55 (s, 2 H) 6.69 - 6.75 (m, 2 H) 7.31 - 7.34 (m, 2 H) 8.05 (d, 1 H) 8.41 - 8.49 (m, 2 H) 8.64 (d, 1 H)

### Example 129

### 2'-[6-amino-5-(trifluoromethyl)pyridin-3-yl]-N-[2-(3-fluorophenyl)propan-2-yl]-5',6'-dihydrospiro[azetidine-3,4'-pyrrolo[1,2-b]pyrazole]-1-carboxamide

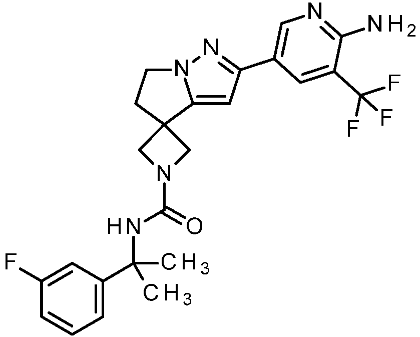

Example 129 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(3-fluorophenyl)propan-2-amine (44.6 mg, 291 µmol).
LC-MS (Method 1): Rt = 1.15 min; MS (ESlpos): m/z = 489 [M+H]⁺
¹H NMR (400 MHz, DMSO-*d*₆) δ ppm 1.55 (s, 6 H) 2.85 (t, 2 H) 3.99 - 4.08 (m, 4 H) 4.13 (t, 2 H) 6.55 (s, 2 H) 6.63 (s, 1 H) 6.71 (s, 1 H) 6.99 (td, 1 H) 7.13 (dt, 1 H) 7.19 (d, 1 H) 7.30 - 7.36 (m, 1 H) 8.05 (d, 1 H) 8.63 (d, 1 H)

### Example 130

### 2'-[6-amino-5-(trifluoromethyl)pyridin-3-yl]-N-[(1S)-1-(3-chloropyridin-4-yl)ethyl]-5',6'-dihydrospiro[azetidine-3,4'-pyrrolo[1,2-b]pyrazole]-1-carboxamide

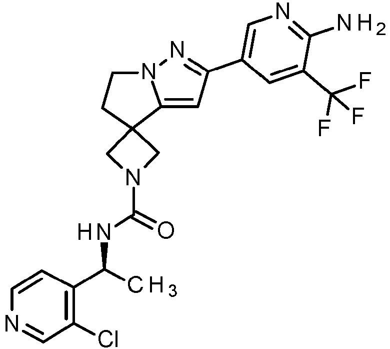

Example 130 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1S)-1-(3-chloropyridin-4-yl)ethan-1-amine-hydrochloride salt (56.2 mg, 291 µmol).

¹H-NMR (400MHz, DMSO-d₆): δ [ppm]= 1.34 (d, 3H), 2.86 (t, 2H), 4.00 - 4.17 (m, 6H), 5.04 (quin, 1H), 6.55 (s, 2H), 6.73 (s, 1H), 7.19 (d, 1H), 7.49 (d, 1H), 8.04 (d, 1H), 8.52 (d, 1H), 8.55 (s, 1H), 8.62 (d, 1H).

### Example 131

### 2'-[6-amino-5-(trifluoromethyl)pyridin-3-yl]-N-[(1-methyl-2-oxabicyclo[2.1.1]hexan-4-yl)methyl]-5',6'-dihydrospiro[azetidine-3,4'-pyrrolo[1,2-b]pyrazole]-1-carboxamide

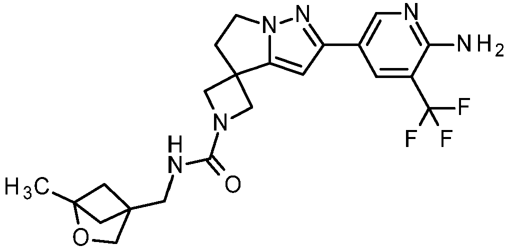

Example 131 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(1-methyl-2-oxabicyclo[2.1.1]hexan-4-yl)methanamine (37.0 mg, 291 µmol).
LC-MS (method 1): Rt = 0.88 min; MS (ESlpos): m/z = 463 [M+H]⁺
¹H-NMR (400 MHz, DMSO-d6) δ [ppm]: 1.139 (0.70), 1.155 (0.78), 1.288 (0.51), 1.312 (16.00), 1.327 (0.47), 1.350 (2.46), 1.354 (2.52), 1.361 (2.81), 1.365 (2.87), 1.489 (2.66), 1.493 (2.51), 1.500 (2.25), 1.504 (2.31), 2.518 (1.78), 2.523 (1.21), 2.828 (1.43), 2.846 (2.36), 2.863 (1.55), 3.310 (3.16), 3.325 (4.00), 3.469 (8.56), 3.970 (0.73), 3.990 (8.50), 4.013 (0.75), 4.108 (1.56), 4.125 (2.46), 4.143 (1.53), 6.546 (3.75), 6.594 (0.77), 6.609 (1.61), 6.624 (0.76), 6.718 (6.91), 8.037 (2.16), 8.042 (2.21), 8.618 (2.03), 8.622 (2.04).

### Example 132

### 2'-[6-amino-5-(trifluoromethyl)pyridin-3-yl]-N-{[3-(trifluoromethyl)bicyclo[1.1.1]pentan-1-yl]methyl}-5',6'-dihydrospiro[azetidine-3,4'-pyrrolo[1,2-b]pyrazole]-1-carboxamide

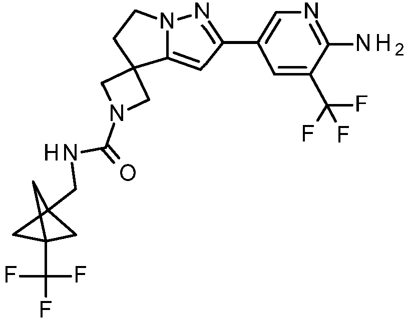

Example 132 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-[3-(trifluoromethyl)bicyclo[1.1.1]pentan-1-yl]methanamine-hydrochloride salt (58.7 mg, 291 µmol).
LC-MS (method 1): Rt = 1.13 min; MS (ESlpos): m/z = 501 [M+H]⁺
¹H-NMR (400 MHz, DMSO-d6) δ [ppm]: 1.833 (16.00), 2.074 (1.07), 2.518 (0.74), 2.523 (0.49), 2.831 (0.93), 2.848 (1.53), 2.865 (1.00), 3.163 (1.90), 3.178 (1.90), 3.976 (0.60), 3.996 (4.60), 4.000 (4.46), 4.021 (0.58), 4.109 (1.02), 4.127 (1.60), 4.143 (0.98), 6.546 (2.40), 6.596 (0.51), 6.610 (1.08), 6.625 (0.49), 6.713 (4.80), 8.034 (1.40), 8.040 (1.44), 8.616 (1.32), 8.620 (1.32).

### Example 133

### 2'-[6-amino-5-(trifluoromethyl)pyridin-3-yl]-N-[2-(2-chlorophenyl)propan-2-yl]-5',6'-dihydrospiro[azetidine-3,4'-pyrrolo[1,2-b]pyrazole]-1-carboxamide

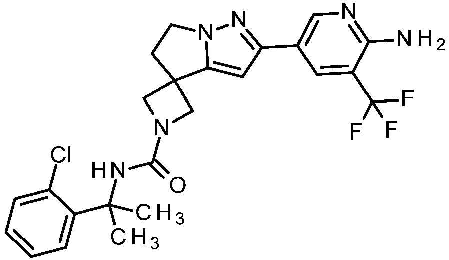

Example 133 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(2-chlorophenyl)propan-2-amine-hydrochloride salt (60.0 mg, 291 µmol).

¹H-NMR (400MHz, DMSO-d₆): δ [ppm]= 1.68 (s, 6H), 2.83 (t, 2H), 4.00 (s, 4H), 4.12 (t, 2H), 6.55 (s, 2H), 6.63 - 6.69 (m, 2H), 7.18 - 7.23 (m, 1H), 7.27 (td, 1H), 7.34 (dd, 1H), 7.47 (dd, 1H), 8.05 (d, 1H), 8.63 (d, 1H).

### Example 134

### 2'-[6-amino-5-(trifluoromethyl)pyridin-3-yl]-N-[(1R)-1-(3-fluorophenyl)ethyl]-5',6'-dihydrospiro[azetidine-3,4'-pyrrolo[1,2-b]pyrazole]-1-carboxamide

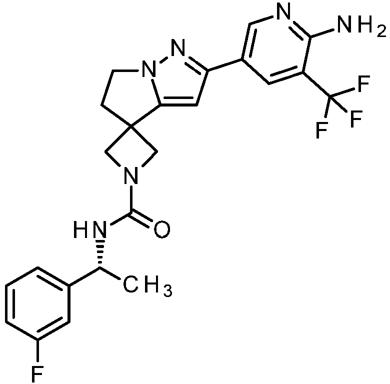

Example 134 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1R)-1-(3-fluorophenyl)ethan-1-amine (40.5 mg, 291 µmol).

¹H-NMR (400MHz, DMSO-d₆): δ [ppm]= 1.36 (d, 3H), 2.85 (t, 2H), 3.98 - 4.09 (m, 4H), 4.13 (t, 2H), 4.76 - 4.87 (m, 1H), 6.55 (s, 2H), 6.72 (s, 1H), 6.93 (d, 1H), 7.00 - 7.07 (m, 1H), 7.13 - 7.21 (m, 2H), 7.29 - 7.40 (m, 1H), 8.04 (d, 1H), 8.62 (d, 1H).

### Example 135

### 2'-[6-amino-5-(trifluoromethyl)pyridin-3-yl]-N-[2-(3-fluoropyridin-2-yl)propan-2-yl]-5',6'-dihydrospiro[azetidine-3,4'-pyrrolo[1,2-b]pyrazole]-1-carboxamide

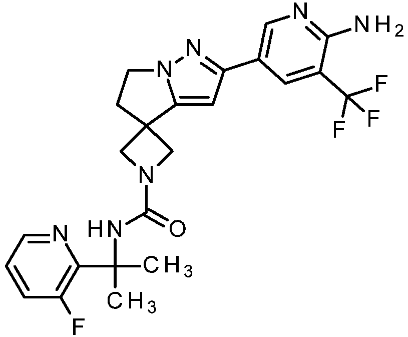

Example 135 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(3-fluoropyridin-2-yl)propan-2-amine (44.9 mg, 291 µmol).
LC-MS (method 1): Rt = 1.05 min; MS (ESlneg): m/z = 488 [M-H]⁻
¹H-NMR (400 MHz, DMSO-d6) δ [ppm]: 1.612 (16.00), 2.518 (0.79), 2.523 (0.49), 2.823 (1.40), 2.840 (2.28), 2.858 (1.49), 3.970 (0.61), 3.992 (8.57), 4.014 (0.61), 4.111 (1.54), 4.128 (2.41), 4.145 (1.46), 5.758 (11.42), 6.545 (3.70), 6.701 (7.18), 6.802 (3.32), 7.316 (0.74), 7.325 (0.87), 7.328 (0.84), 7.336 (1.58), 7.346 (0.98), 7.348 (1.08), 7.357 (0.86), 7.561 (0.91), 7.564 (0.92), 7.582 (0.81), 7.585 (0.81), 7.592 (0.90), 7.595 (0.91), 7.613 (0.78), 7.616 (0.77), 8.048 (2.15), 8.053 (2.17), 8.307 (0.92), 8.311 (1.49), 8.315 (1.07), 8.318 (1.01), 8.322 (1.44), 8.326 (0.90), 8.628 (2.00), 8.633 (1.98).

### Example 136

### 2'-[6-amino-5-(trifluoromethyl)pyridin-3-yl]-N-[1-(2-chlorophenyl)cyclobutyl]-5',6'-dihydrospiro[azetidine-3,4'-pyrrolo[1,2-b]pyrazole]-1-carboxamide

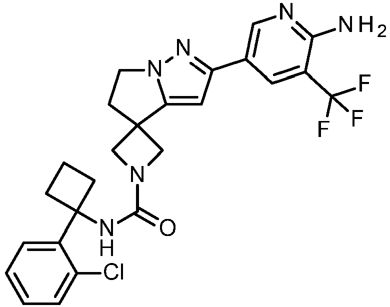

Example 136 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(2-chlorophenyl)cyclobutan-1-amine-hydrochloride salt (63.5 mg, 291 µmol).

¹H-NMR (400MHz, DMSO-d₆): δ [ppm]= 1.63 - 1.76 (m, 1H), 1.99 - 2.14 (m, 1H), 2.57 - 2.69 (m, 2H), 2.80 (t, 2H), 3.91 - 3.99 (m, 4H), 4.10 (t, 2H), 6.54 (s, 2H), 6.64 (s, 1H), 7.02 (s, 1H), 7.17 - 7.29 (m, 2H), 7.33 (dd, 1H), 7.54 (dd, 1H), 8.03 (d, 1H), 8.60 (d, 1H).

### Example 137

### 2'-[6-amino-5-(trifluoromethyl)pyridin-3-yl]-N-[1-(3-fluorophenyl)cyclobutyl]-5',6'-dihydrospiro[azetidine-3,4'-pyrrolo[1,2-b]pyrazole]-1-carboxamide

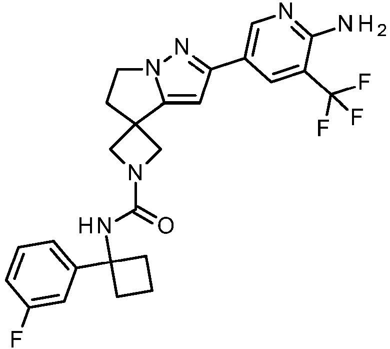

Example 137 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(3-fluorophenyl)cyclobutan-1-amine-hydrochloride salt (58.7 mg, 291 µmol).

¹H-NMR (400MHz, DMSO-d₆): δ [ppm]= 1.75 (dddd, 1H), 1.98 - 2.12 (m, 3H), 2.81 (t, 2H), 3.90 - 4.02 (m, 4H), 4.11 (t, 2H), 6.54 (s, 2H), 6.67 (s, 1H), 7.04 - 7.15 (m, 3H), 7.24 (tdd, 1H), 7.43 (td, 1H), 8.03 (d, 1H), 8.61 (d, 1H).

### Example 138

### 2'-[6-amino-5-(trifluoromethyl)pyridin-3-yl]-N-[(1R)-1-phenylpropyl]-5',6'-dihydrospiro[azetidine-3,4'-pyrrolo[1,2-b]pyrazole]-1-carboxamide

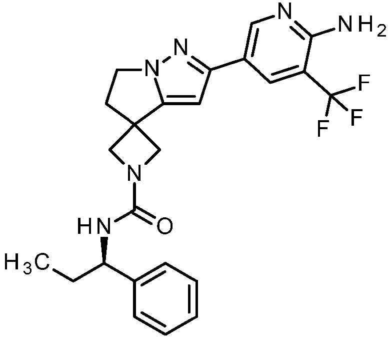

Example 138 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1R)-1-phenylpropan-1-amine (39.3 mg, 291 µmol).

¹H-NMR (400MHz, DMSO-d₆): δ [ppm]= 0.86 (t, 3H), 1.61 - 1.75 (m, 2H), 2.84 (t, 2H), 3.97 - 4.06 (m, 4H), 4.12 (t, 2H), 4.49 - 4.59 (m, 1H), 6.54 (s, 2H), 6.71 (s, 1H), 6.83 (d, 1H), 7.16 - 7.25 (m, 1H), 7.27 - 7.36 (m, 4H), 8.04 (d, 1H), 8.61 (d, 1H).

### Example 139

### 2'-[6-amino-5-(trifluoromethyl)pyridin-3-yl]-N-[(pyrimidin-4-yl)methyl]-5',6'-dihydrospiro[azetidine-3,4'-pyrrolo[1,2-b]pyrazole]-1-carboxamide

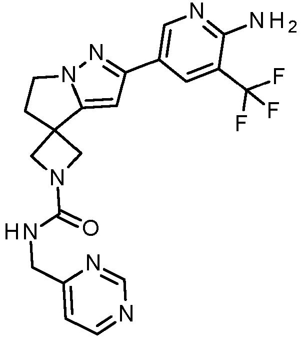

Example 139 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyrimidin-4-yl)methanamine (31.8 mg, 291 µmol).

¹H-NMR (400MHz, DMSO-d₆): δ [ppm]= 2.88 (t, 2H), 4.05 - 4.20 (m, 6H), 4.30 (d, 2H), 6.56 (s, 2H), 6.76 (s, 1H), 7.26 (t, 1H), 7.44 (dd, 1H), 8.05 (d, 1H), 8.63 (d, 1H), 8.76 (d, 1H), 9.09 (d, 1H).

### Example 140

### 2'-[6-amino-5-(trifluoromethyl)pyridin-3-yl]-N-[(3-fluoropyridin-2-yl)methyl]-5',6'-dihydrospiro[azetidine-3,4'-pyrrolo[1,2-b]pyrazole]-1-carboxamide

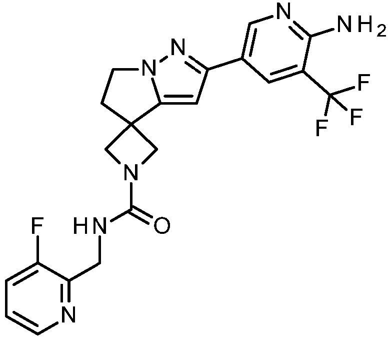

Example 140 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(3-fluoropyridin-2-yl)methanamine (36.7 mg, 291 µmol).

¹H-NMR (400MHz, DMSO-d₆): δ [ppm]= 4.04 (s, 4H), 4.13 (t, 2H), 4.40 (dd, 2H), 6.55 (s, 2H), 6.72 (s, 1H), 7.00 (t, 1H), 7.39 (dt, 1H), 7.67 (ddd, 1H), 8.04 (d, 1H), 8.39 (dt, 1H), 8.62 (d, 1H).

### Example 141

### 2'-[6-amino-5-(trifluoromethyl)pyridin-3-yl]-N-[(pyrimidin-5-yl)methyl]-5',6'-dihydrospiro[azetidine-3,4'-pyrrolo[1,2-b]pyrazole]-1-carboxamide

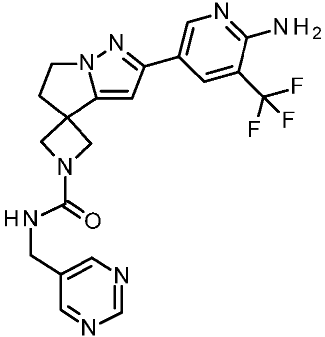

Example 141 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyrimidin-5-yl)methanamine (31.8 mg, 291 µmol).

¹H-NMR (400MHz, DMSO-d₆): δ [ppm]= 2.86 (t, 2H), 3.99 - 4.08 (m, 4H), 4.13 (t, 2H), 4.25 (d, 2H), 6.55 (s, 2H), 6.73 (s, 1H), 7.17 (t, 1H), 8.04 (d, 1H), 8.61 (d, 1H), 8.73 (s, 2H), 9.08 (s, 1H).

### Example 142

### 2'-[6-amino-5-(trifluoromethyl)pyridin-3-yl]-N-[(3-chloropyridin-4-yl)methyl]-5',6'-dihydrospiro[azetidine-3,4'-pyrrolo[1,2-b]pyrazole]-1-carboxamide

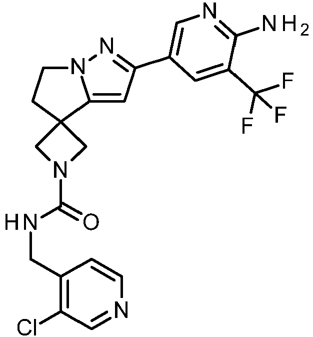

Example 142 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(3-chloropyridin-4-yl)methanamine-hydrochloride salt (62.7 mg, 291 µmol).

¹H-NMR (400MHz, DMSO-d₆): δ [ppm]= 2.89 (t, 2H), 4.04 - 4.18 (m, 6H), 4.31 (d, 2H), 6.56 (s, 2H), 6.76 (s, 1H), 7.22 (t, 1H), 7.38 (d, 1H), 8.05 (d, 1H), 8.52 (d, 1H), 8.57 (s, 1H), 8.63 (d, 1H).

### Example 143

### 2'-[6-amino-5-(trifluoromethyl)pyridin-3-yl]-N-[(3-fluorophenyl)methyl]-5',6'-dihydrospiro[azetidine-3,4'-pyrrolo[1,2-b]pyrazole]-1-carboxamide

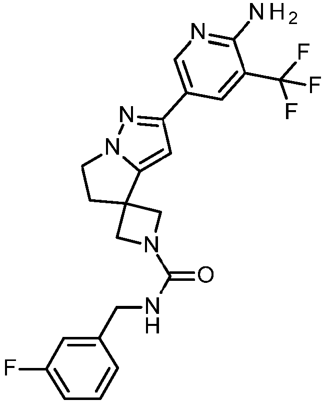

Example 143 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(3-fluorophenyl)methanamine (36.4 mg, 291 µmol).

¹H-NMR (400MHz, DMSO-d₆): δ [ppm]= 2.86 (t, 2H), 4.01 - 4.08 (m, 4H), 4.13 (t, 2H), 4.24 (d, 2H), 6.55 (s, 2H), 6.73 (s, 1H), 6.99 - 7.17 (m, 4H), 7.33 - 7.41 (m, 1H), 8.04 (d, 1H), 8.62 (d, 1H).

### Example 144

### 2'-[6-amino-5-(trifluoromethyl)pyridin-3-yl]-N-[(pyridin-3-yl)methyl]-5',6'-dihydrospiro[azetidine-3,4'-pyrrolo[1,2-b]pyrazole]-1-carboxamide

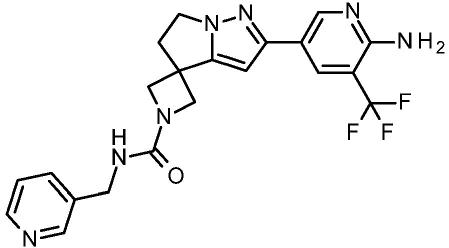

Example 144 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyridin-3-yl)methanamine (31.5 mg, 291 µmol).

¹H-NMR (400MHz, DMSO-d₆): δ [ppm]= 2.86 (t, 2H), 3.99 - 4.08 (m, 4H), 4.13 (t, 2H), 4.25 (d, 2H), 6.55 (s, 2H), 6.73 (s, 1H), 7.14 (t, 1H), 7.34 - 7.39 (m, 1H), 7.69 (dt, 1H), 8.04 (d, 1H), 8.45 (dd, 1H), 8.50 (d, 1H), 8.62 (d, 1H).

### Example 145

### (rac)-2-[6-amino-5-(trifluoromethyl)pyridin-3-yl]-N-benzyl-6,7-dihydrospiro[pyrazolo[5,1-c][1,4]oxazine-4,3'-pyrrolidine]-1'-carboxamide

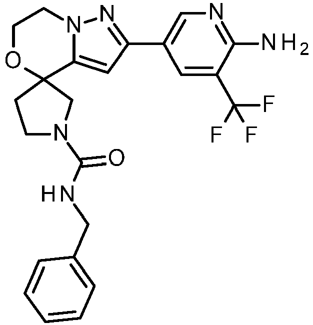

Example 145 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine (75.0 mg, 221 µmol) and 1-phenylmethanamine (29 µL, 270 µmol).

1H-NMR (400 MHz, DMSO-d6) δ [ppm]: 1.066 (0.44), 2.261 (0.54), 2.336 (0.55), 2.358 (0.52), 2.518 (3.87), 2.522 (2.44), 3.394 (0.52), 3.403 (0.75), 3.419 (0.78), 3.429 (0.60), 3.445 (0.50), 3.468 (1.49), 3.497 (1.90), 3.578 (2.70), 3.601 (2.27), 3.625 (1.19), 3.807 (0.99), 3.835 (0.86), 4.068 (0.58), 4.084 (0.68), 4.102 (0.78), 4.118 (0.97), 4.125 (1.61), 4.138 (2.83), 4.221 (9.96), 4.235 (10.31), 4.258 (2.32), 6.433 (2.58), 6.620 (0.70), 6.758 (5.12), 6.807 (0.52), 6.821 (1.01), 6.836 (0.50), 7.200 (2.37), 7.204 (1.71), 7.212 (2.06), 7.218 (6.45), 7.223 (2.75), 7.236 (10.46), 7.238 (11.01), 7.255 (14.73), 7.259 (9.99), 7.283 (4.86), 7.291 (16.00), 7.297 (4.71), 7.308 (11.40), 7.310 (13.14), 7.324 (2.34), 7.328 (5.33), 8.019 (1.97), 8.024 (1.98), 8.581 (1.79), 8.585 (1.79).

### Example 146

### (rac)-2-[6-amino-5-(trifluoromethyl)pyridin-3-yl]-N-[(1R)-1-(2-methylpyrimidin-5-yl)ethyl]-6,7-dihydrospiro[pyrazolo[5,1-c][1,4]oxazine-4,3'-pyrrolidine]-1'-carboxamide (mixture of stereoisomers)

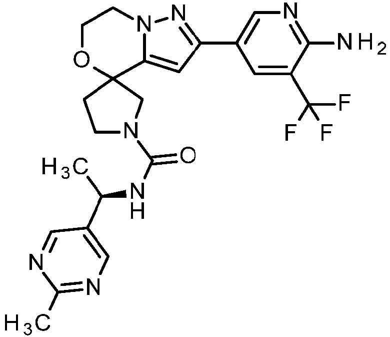

Example 146 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (1R)-1-(2-methylpyrimidin-5-yl)ethan-1-amine (30.1 mg, 220 µmol).

¹H-NMR (400 MHz, DMSO-d6) δ [ppm]: 1.232 (0.66), 1.407 (7.43), 1.425 (7.45), 2.236 (0.61), 2.323 (1.38), 2.327 (1.97), 2.331 (1.60), 2.518 (5.63), 2.523 (3.77), 2.540 (4.58), 2.579 (16.00), 2.665 (0.96), 2.669 (1.32), 2.674 (0.90), 3.386 (1.53), 3.405 (0.94), 3.443 (0.92), 3.471 (0.99), 3.487 (0.88), 3.516 (0.94), 3.551 (0.50), 3.573 (0.83), 3.595 (0.79), 3.617 (0.83), 3.754 (0.94), 3.785 (0.77), 3.810 (0.90), 3.837 (0.79), 4.077 (0.99), 4.083 (1.01), 4.106 (1.62), 4.118 (1.93), 4.132 (4.30), 4.804 (0.64), 4.813 (0.75), 4.822 (1.03), 4.831 (1.03), 4.840 (0.75), 4.849 (0.66), 6.600 (6.31), 6.617 (2.26), 6.637 (1.05), 6.747 (3.59), 6.754 (4.56), 8.007 (3.24), 8.171 (2.41), 8.575 (3.20), 8.640 (9.38), 8.646 (9.14).

### Example 147

### (rac)-2-[6-amino-5-(trifluoromethyl)pyridin-3-yl]-N-[(1R)-1-(pyridin-3-yl)ethyl]-6,7-dihydrospiro[pyrazolo[5,1-c][1,4]oxazine-4,3'-pyrrolidine]-1'-carboxamide (mixture of stereoisomers)

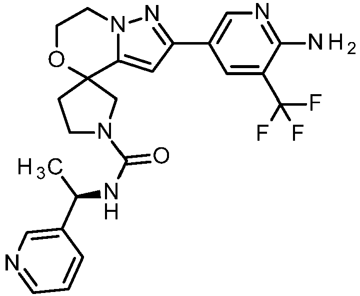

Example 147 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (1R)-1-(pyridin-3-yl)ethan-1-amine-hydrochloride salt (42.8 mg, 220 µmol).

¹H-NMR (400MHz, DMSO-d₆): δ [ppm]= 1.40 (d, 3H), 2.16 - 2.29 (m, 1H), 2.30 - 2.40 (m, 1H), 3.49 (br t, 2H), 3.54 - 3.67 (m, 1H), 3.81 (dd, 1H), 4.01 - 4.19 (m, 4H), 4.80 - 4.93 (m, 1H), 6.55 - 6.64 (m, 3H), 6.75 (d, 1H), 7.29 - 7.37 (m, 1H), 7.74 (dd, 1H), 8.01 (s, 1H), 8.41 (d, 1H), 8.52 - 8.60 (m, 2H).

### Example 148

### 2'-[6-amino-5-(trifluoromethyl)pyridin-3-yl]-N-benzyl-5',6'-dihydrospiro[azetidine-3,4'-pyrrolo[1,2-b]pyrazole]-1-carboxamide

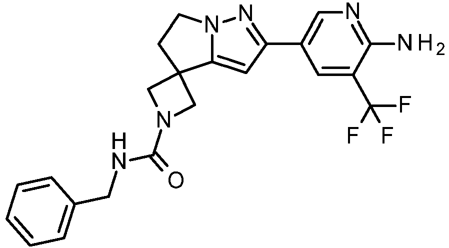

Example 148 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-phenylmethanamine (32 µL, 290 µmol).

1H-NMR (400 MHz, DMSO-d6) δ [ppm]: 2.518 (4.48), 2.522 (2.90), 2.840 (2.15), 2.857 (3.37), 2.874 (2.25), 4.014 (1.40), 4.034 (9.82), 4.039 (9.51), 4.060 (1.40), 4.112 (2.31), 4.130 (3.59), 4.147 (2.19), 4.221 (15.88), 4.236 (16.00), 6.419 (1.85), 6.434 (3.43), 6.449 (1.78), 6.546 (5.54), 6.735 (10.71), 7.063 (1.14), 7.078 (2.45), 7.093 (1.09), 7.200 (1.89), 7.204 (1.26), 7.211 (1.58), 7.218 (5.37), 7.223 (2.51), 7.227 (2.94), 7.236 (8.98), 7.238 (9.57), 7.249 (3.73), 7.254 (12.47), 7.259 (8.50), 7.271 (2.60), 7.288 (8.44), 7.291 (14.52), 7.297 (4.14), 7.307 (12.53), 7.310 (11.42), 7.312 (9.04), 7.324 (6.06), 7.328 (6.10), 7.340 (0.77), 7.343 (1.62), 8.039 (3.24), 8.044 (3.27), 8.618 (2.98), 8.622 (2.94).

### Example 149

### 2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide (enantiomer 1)

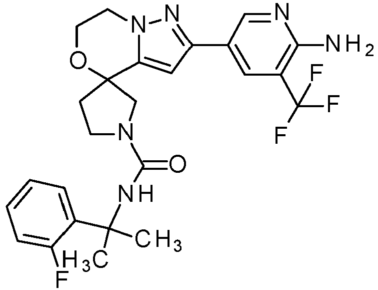

Example 149 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (86.0 mg, 229 µmol) and 2-(2-fluorophenyl)propan-2-amine (42.1 mg, 275 µmol).
[α]²⁰_{D} : -53.3° (c = 1.00, DMSO)
¹H-NMR (400 MHz, DMSO-d6) δ [ppm]: 1.137 (0.43), 1.232 (1.06), 1.629 (16.00), 1.635 (15.74), 2.085 (11.75), 2.221 (0.85), 2.247 (1.20), 2.269 (0.64), 2.318 (1.38), 2.323 (1.87), 2.327 (2.70), 2.332 (2.27), 2.346 (0.67), 2.362 (0.56), 2.518 (5.33), 2.523 (3.85), 2.660 (0.54), 2.665 (1.21), 2.669 (1.73), 2.673 (1.20), 2.679 (0.53), 3.363 (0.56), 3.388 (1.18), 3.404 (1.28), 3.430 (0.55), 3.471 (1.87), 3.500 (2.20), 3.570 (0.92), 3.591 (1.64), 3.613 (0.73), 3.778 (1.95), 3.807 (1.66), 4.069 (0.62), 4.084 (1.26), 4.101 (1.81), 4.119 (2.91), 4.130 (5.03), 4.142 (5.18), 4.155 (1.63), 5.759 (11.02), 6.114 (5.82), 6.600 (7.28), 6.760 (12.67), 7.031 (1.40), 7.034 (1.56), 7.051 (1.76), 7.054 (1.95), 7.062 (1.34), 7.065 (1.66), 7.075 (1.89), 7.078 (1.85), 7.083 (1.67), 7.086 (2.25), 7.093 (3.68), 7.097 (2.89), 7.112 (2.51), 7.116 (2.14), 7.191 (0.94), 7.195 (1.08), 7.203 (1.07), 7.207 (1.44), 7.215 (1.38), 7.222 (1.33), 7.227 (1.35), 7.233 (0.72), 7.242 (0.63), 7.246 (0.60), 7.299 (1.48), 7.303 (1.45), 7.321 (2.26), 7.341 (1.28), 7.345 (1.11), 8.013 (4.42), 8.018 (4.44), 8.582 (4.14), 8.586 (4.04).

### Example 150

### 2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (enantiomer 1)

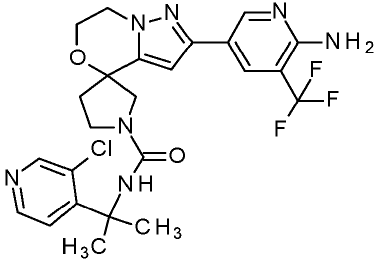

Example 150 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (86.0 mg, 229 µmol) and 2-(3-chloropyridin-4-yl)propan-2-amine (46.9 mg, 275 µmol).
[α]²⁰_{D} : -59.8° (c = 1.00, DMSO)
LC-MS (method 1): Rt = 1.01 min; MS (ESlpos): m/z = 536 [M+H]⁺
¹H-NMR (400 MHz, DMSO-d6) δ [ppm]: 1.232 (0.48), 1.627 (0.50), 1.649 (7.71), 1.664 (7.17), 2.085 (2.63), 2.228 (0.59), 2.306 (0.47), 2.323 (1.34), 2.327 (1.53), 2.332 (1.07), 2.337 (0.65), 2.518 (3.65), 2.523 (2.59), 2.665 (0.86), 2.669 (1.20), 2.673 (0.83), 3.375 (0.52), 3.392 (0.52), 3.444 (0.83), 3.472 (0.97), 3.562 (0.68), 3.746 (0.82), 3.776 (0.68), 4.071 (0.60), 4.085 (0.71), 4.102 (0.94), 4.108 (1.05), 4.121 (2.05), 4.133 (2.98), 5.759 (16.00), 6.354 (2.84), 6.599 (3.52), 6.748 (6.20), 7.423 (2.45), 7.436 (2.48), 8.012 (2.13), 8.017 (2.13), 8.397 (3.95), 8.410 (3.78), 8.428 (7.25), 8.582 (1.98), 8.586 (1.95).

### Example 151

### 2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide (enantiomer 2)

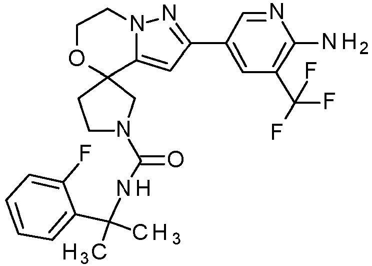

Example 151 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (48.0 mg, 128 µmol) and 2-(2-fluorophenyl)propan-2-amine (23.5 mg, 153 µmol).
[α]²⁰_{D} : +46.8° (c = 1.00, DMSO)
LC-MS (method 1): Rt = 1.15 min; MS (ESlpos): m/z = 519 [M+H]⁺
¹H-NMR (400 MHz, DMSO-d6) δ [ppm]: 1.232 (1.41), 1.629 (16.00), 1.635 (15.62), 2.220 (0.84), 2.246 (1.20), 2.269 (0.62), 2.318 (1.80), 2.323 (2.86), 2.327 (4.01), 2.332 (3.17), 2.363 (0.55), 2.518 (9.47), 2.523 (6.66), 2.660 (0.99), 2.665 (2.17), 2.669 (2.99), 2.673 (2.08), 2.678 (0.92), 3.363 (0.61), 3.387 (1.18), 3.405 (1.19), 3.430 (0.55), 3.471 (1.86), 3.499 (2.15), 3.569 (0.94), 3.591 (1.64), 3.614 (0.72), 3.778 (1.99), 3.807 (1.65), 4.069 (0.63), 4.084 (1.30), 4.101 (1.82), 4.119 (3.00), 4.130 (5.10), 4.142 (5.22), 5.759 (9.17), 6.114 (5.71), 6.600 (7.40), 6.760 (11.84), 7.031 (1.36), 7.034 (1.48), 7.051 (1.75), 7.054 (1.89), 7.065 (1.61), 7.075 (1.82), 7.078 (1.72), 7.086 (2.15), 7.094 (3.55), 7.097 (2.80), 7.113 (2.42), 7.116 (2.02), 7.191 (0.92), 7.195 (1.06), 7.203 (1.08), 7.208 (1.46), 7.215 (1.33), 7.222 (1.32), 7.227 (1.30), 7.233 (0.67), 7.242 (0.60), 7.246 (0.57), 7.299 (1.46), 7.303 (1.39), 7.321 (2.22), 7.341 (1.23), 7.345 (1.04), 8.013 (4.44), 8.018 (4.40), 8.581 (4.17), 8.586 (4.04).

### Example 152

### 2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (enantiomer 2)

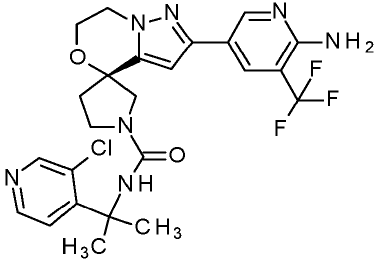

Example 152 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (48.0 mg, 128 µmol) and 2-(3-chloropyridin-4-yl)propan-2-amine (26.2 mg, 153 µmol).
[α]²⁰_{D} : +59.6° (c = 1.00, DMSO)
LC-MS (method 1): Rt = 1.01 min; MS (ESlpos): m/z = 536 [M+H]⁺
¹H-NMR (400 MHz, DMSO-d6) δ [ppm]: 0.852 (0.41), 1.232 (1.54), 1.649 (16.00), 1.664 (14.91), 2.195 (0.81), 2.202 (0.84), 2.228 (1.21), 2.252 (0.62), 2.306 (0.95), 2.323 (3.25), 2.327 (3.82), 2.332 (2.72), 2.336 (1.59), 2.518 (10.89), 2.523 (7.64), 2.660 (1.00), 2.665 (2.23), 2.669 (3.12), 2.673 (2.20), 2.678 (0.95), 3.374 (1.22), 3.392 (1.15), 3.418 (0.59), 3.444 (1.75), 3.472 (2.03), 3.541 (0.85), 3.563 (1.45), 3.585 (0.69), 3.746 (1.72), 3.776 (1.43), 4.055 (0.67), 4.069 (1.22), 4.086 (1.51), 4.108 (2.19), 4.121 (4.28), 4.133 (6.24), 5.759 (12.16), 6.354 (5.81), 6.599 (7.30), 6.748 (12.34), 7.423 (4.91), 7.436 (5.06), 8.012 (4.36), 8.017 (4.45), 8.397 (7.68), 8.410 (7.57), 8.428 (14.72), 8.581 (4.06), 8.586 (4.09).

### Example 153

### 2-[6-amino-5-(trifluoromethyl)pyridin-3-yl]-N-[(1R)-1-(4-cyanopyridin-2-yl)ethyl]-6,7-dihydrospiro[pyrazolo[5,1-c][1,4]oxazine-4,3'-pyrrolidine]-1'-carboxamide (mixture of stereoisomers)

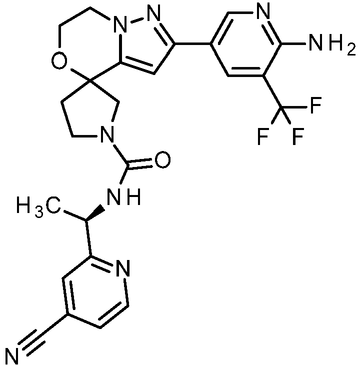

Example 153 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75 mg, 200 µmol) and 2-[(1R)-1-aminoethyl]pyridine-4-carbonitrile-hydrochloride salt (37.6 mg, 205 µmol).

¹H-NMR (400MHz, DMSO-d₆): δ [ppm]= 1.42 (d, 3H), 2.17 - 2.29 (m, 1H), 2.31 - 2.41 (m, 1H), 3.36 - 3.69 (m, 3H), 3.74 - 3.88 (m, 1H), 4.03 - 4.19 (m, 4H), 4.85 - 4.97 (m, 1H), 6.60 (s, 2H), 6.65 (dd, 1H), 6.76 (d, 1H), 8.01 (s, 1H), 8.26 (q, 1H), 8.58 (s, 1H), 8.87 (dt, 2H).

### Example 154

### (rac)-2-[6-amino-5-(trifluoromethyl)pyridin-3-yl]-N-[2-(1-methyl-1H-1,2,3-triazol-5-yl)propan-2-yl]-6,7-dihydrospiro[pyrazolo[5,1-c][1,4]oxazine-4,3'-pyrrolidine]-1'-carboxamide

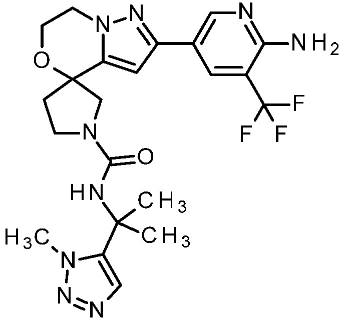

Example 154 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (80.0 mg, 213 µmol) and 2-(1-methyl-1H-1,2,3-triazol-4-yl)propan-2-amine-hydrochloride salt (49.9 mg, 234 µmol).
LC-MS (method 2): Rt = 0.82 min; MS (ESlpos): m/z = 506 [M+H]⁺
¹H-NMR (400 MHz, DMSO-d6) δ [ppm]: 1.595 (9.55), 1.600 (9.66), 2.226 (0.52), 2.301 (0.45), 2.318 (0.71), 2.323 (0.76), 2.327 (0.88), 2.332 (0.76), 2.518 (2.20), 2.523 (1.48), 2.665 (0.47), 2.669 (0.65), 2.673 (0.45), 3.351 (0.56), 3.359 (0.64), 3.377 (0.60), 3.385 (0.42), 3.444 (1.00), 3.473 (1.14), 3.558 (0.47), 3.580 (0.86), 3.764 (0.99), 3.791 (0.84), 3.981 (16.00), 4.072 (0.54), 4.090 (0.76), 4.108 (1.33), 4.119 (1.83), 4.132 (2.89), 5.951 (2.63), 6.597 (3.32), 6.746 (5.53), 7.791 (6.40), 8.011 (2.02), 8.016 (2.05), 8.580 (1.89), 8.585 (1.87).

### Example 155

### (rac)-2-[6-amino-5-(trifluoromethyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide

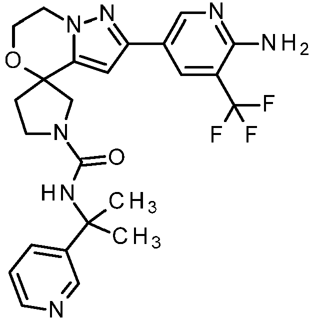

Example 155 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (80.0 mg, 213 µmol) and 2-(pyridin-3-yl)propan-2-amine (31.9 mg, 234 µmol).

¹H-NMR (400 MHz, DMSO-d6) δ [ppm]: 1.583 (16.00), 2.075 (0.43), 2.221 (0.55), 2.245 (0.77), 2.269 (0.41), 2.318 (1.11), 2.323 (1.59), 2.327 (1.97), 2.332 (1.75), 2.349 (0.46), 2.518 (4.88), 2.523 (3.29), 2.660 (0.43), 2.665 (1.06), 2.669 (1.49), 2.673 (1.01), 2.679 (0.46), 3.391 (0.79), 3.410 (0.74), 3.485 (0.89), 3.513 (1.03), 3.587 (0.58), 3.610 (0.96), 3.632 (0.46), 3.788 (1.15), 3.817 (0.98), 4.088 (0.82), 4.106 (1.32), 4.124 (2.64), 4.131 (3.96), 4.142 (3.53), 6.207 (3.89), 6.600 (5.02), 6.767 (7.93), 7.270 (1.63), 7.271 (1.66), 7.282 (1.59), 7.290 (1.66), 7.301 (1.75), 7.303 (1.75), 7.697 (1.35), 7.701 (1.78), 7.703 (1.66), 7.707 (1.47), 7.717 (1.30), 7.721 (1.47), 7.723 (1.56), 7.727 (1.27), 8.014 (3.00), 8.019 (3.05), 8.150 (3.48), 8.350 (3.05), 8.354 (3.10), 8.361 (2.81), 8.366 (2.71), 8.580 (4.44), 8.583 (5.86), 8.585 (5.43).

### Example 156

### 2-[6-amino-5-(trifluoromethyl)pyridin-3-yl]-N-[(1R)-1-(4-fluorophenyl)ethyl]-6,7-dihydrospiro[pyrazolo[5,1-c][1,4]oxazine-4,3'-pyrrolidine]-1'-carboxamide (mixture of stereoisomers)

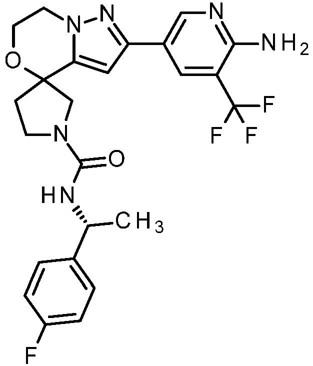

Example 156 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (1R)-1-(4-fluorophenyl)ethan-1-amine (30.6 mg, 220 µmol).

1H-NMR (400 MHz, DMSO-d6) δ [ppm]: 1.003 (12.03), 1.019 (11.16), 1.349 (14.43), 1.366 (14.41), 2.074 (1.26), 2.210 (1.45), 2.236 (1.62), 2.327 (3.19), 2.523 (6.74), 2.669 (1.59), 3.105 (0.70), 3.400 (3.58), 3.449 (2.18), 3.472 (2.80), 3.500 (2.55), 3.560 (1.09), 3.582 (1.90), 3.614 (1.85), 3.638 (0.84), 3.777 (1.93), 3.804 (3.27), 3.832 (1.62), 4.073 (2.07), 4.120 (4.22), 4.134 (9.09), 4.814 (1.73), 4.833 (2.77), 4.851 (1.85), 6.494 (2.32), 6.504 (2.41), 6.514 (2.43), 6.524 (2.32), 6.599 (11.72), 6.751 (16.00), 7.090 (3.44), 7.096 (3.83), 7.112 (7.78), 7.119 (7.69), 7.134 (4.62), 7.141 (4.06), 7.351 (4.31), 7.365 (5.59), 7.369 (5.26), 7.383 (3.89), 8.010 (6.80), 8.202 (1.03), 8.580 (6.38).

### Example 157

### 2-[6-amino-5-(trifluoromethyl)pyridin-3-yl]-N-[(1R)-2,3-dihydro-1H-inden-1-yl]-6,7-dihydrospiro[pyrazolo[5,1-c][1,4]oxazine-4,3'-pyrrolidine]-1'-carboxamide (mixture of stereoisomers)

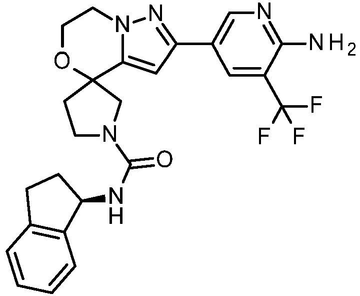

Example 157 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (1R)-2,3-dihydro-1H-inden-1-amine (29.2 mg, 220 µmol).

1H-NMR (400 MHz, DMSO-d6) δ [ppm]: 1.816 (1.01), 1.840 (1.32), 1.864 (1.07), 2.074 (1.43), 2.084 (16.00), 2.232 (1.04), 2.259 (1.32), 2.282 (0.61), 2.331 (1.90), 2.347 (2.00), 2.366 (2.09), 2.378 (1.79), 2.410 (0.70), 2.669 (0.80), 2.723 (0.65), 2.745 (0.96), 2.763 (1.39), 2.784 (1.65), 2.806 (0.82), 2.875 (1.27), 2.893 (1.34), 2.909 (0.82), 2.933 (0.74), 3.388 (1.31), 3.413 (1.79), 3.431 (1.11), 3.439 (1.01), 3.472 (1.82), 3.492 (1.96), 3.501 (2.15), 3.521 (2.05), 3.589 (0.63), 3.611 (1.25), 3.640 (1.25), 3.666 (0.53), 3.819 (1.21), 3.841 (1.50), 3.868 (1.09), 4.060 (0.82), 4.074 (1.54), 4.090 (1.47), 4.108 (1.92), 4.132 (5.01), 4.143 (6.26), 5.180 (0.82), 5.201 (2.32), 5.220 (2.28), 5.241 (0.82), 6.492 (1.79), 6.499 (1.92), 6.513 (1.81), 6.520 (1.78), 6.604 (5.30), 6.760 (10.04), 7.169 (2.94), 7.174 (3.70), 7.182 (4.67), 7.191 (4.40), 7.199 (1.74), 7.214 (3.75), 7.226 (4.22), 7.234 (3.21), 8.015 (4.95), 8.020 (5.06), 8.584 (4.67).

### Example 158

### 2-[6-amino-5-(trifluoromethyl)pyridin-3-yl]-N-[(1R)-1-(5-fluoropyridin-3-yl)ethyl]-6,7-dihydrospiro[pyrazolo[5,1-c][1,4]oxazine-4,3'-pyrrolidine]-1'-carboxamide (mixture of stereoisomers)

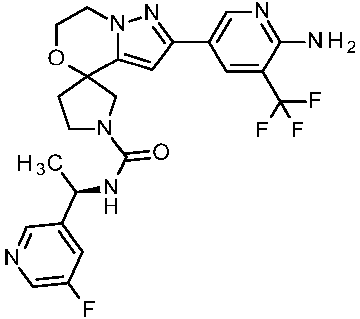

Example 158 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (1R)-1-(5-fluoropyridin-3-yl)ethan-1-amine (30.8 mg, 220 µmol).

¹H-NMR (400MHz, DMSO-d₆): δ [ppm]= 1.41 (d, 3H), 2.19 - 2.30 (m, 1H), 2.31 - 2.41 (m, 1H), 3.36 - 3.68 (m, 3H), 3.75 - 3.87 (m, 1H), 4.03 - 4.17 (m, 4H), 4.91 (td, 1H), 6.57 - 6.67 (m, 3H), 6.76 (d, 1H), 7.63 - 7.71 (m, 1H), 8.01 (s, 1H), 8.40 - 8.46 (m, 2H), 8.58 (s, 1H).

### Example 159

### 2-[6-amino-5-(trifluoromethyl)pyridin-3-yl]-N-[(1R)-1-(3-fluorophenyl)ethyl]-6,7-dihydrospiro[pyrazolo[5,1-c][1,4]oxazine-4,3'-pyrrolidine]-1'-carboxamide (mixture of stereoisomers)

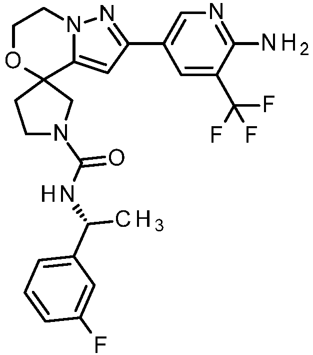

Example 159 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (1R)-1-(3-fluorophenyl)ethan-1-amine (30.6 mg, 220 µmol).

¹H-NMR (400MHz, DMSO-d₆): δ [ppm]= 1.36 (d, 3H), 2.17 - 2.29 (m, 1H), 2.31 - 2.40 (m, 1H), 3.36 - 3.53 (m, 2H), 3.55 - 3.67 (m, 1H), 3.77 - 3.87 (m, 1H), 4.01 - 4.18 (m, 4H), 4.79 - 4.90 (m, 1H), 6.54 (t, 1H), 6.60 (s, 2H), 6.76 (d, 1H), 6.97 - 7.05 (m, 1H), 7.12 - 7.20 (m, 2H), 7.34 (tdd, 1H), 8.01 (s, 1H), 8.58 (t, 1H).

### Example 160

### 2-[6-amino-5-(trifluoromethyl)pyridin-3-yl]-N-[(1R)-1-phenylpropyl]-6,7-dihydrospiro[pyrazolo[5,1-c][1,4]oxazine-4,3'-pyrrolidine]-1'-carboxamide (mixture of stereoisomers)

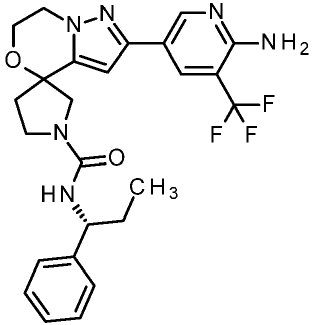

Example 160 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (1R)-1-phenylpropan-1-amine (29.7 mg, 220 µmol).

1H-NMR (400 MHz, DMSO-d6) δ [ppm]: 0.832 (5.98), 0.836 (6.36), 0.851 (14.07), 0.854 (14.27), 0.869 (7.06), 0.873 (6.82), 1.134 (0.81), 1.153 (0.50), 1.245 (0.66), 1.267 (0.69), 1.283 (0.58), 1.624 (0.89), 1.642 (1.73), 1.659 (3.05), 1.677 (3.28), 1.694 (2.58), 1.719 (2.51), 1.736 (1.93), 1.752 (1.23), 2.158 (1.08), 2.178 (2.70), 2.184 (2.39), 2.204 (2.27), 2.242 (2.39), 2.265 (1.27), 2.327 (4.20), 2.331 (3.28), 2.518 (14.77), 2.523 (9.64), 2.534 (4.74), 2.548 (2.78), 2.563 (1.20), 2.606 (0.89), 2.627 (1.16), 2.643 (0.96), 2.659 (1.31), 2.665 (1.77), 2.669 (2.27), 2.673 (1.89), 3.319 (1.23), 3.348 (2.31), 3.371 (2.47), 3.388 (2.85), 3.398 (3.32), 3.413 (3.78), 3.440 (1.81), 3.455 (2.78), 3.486 (5.24), 3.515 (4.67), 3.578 (9.91), 3.623 (3.97), 3.647 (2.04), 3.771 (2.47), 3.799 (2.27), 3.809 (2.47), 3.838 (1.93), 4.073 (2.47), 4.115 (4.32), 4.132 (10.06), 4.213 (1.31), 4.222 (1.47), 4.232 (1.93), 4.242 (1.54), 4.249 (1.50), 4.257 (1.27), 4.538 (1.39), 4.559 (3.20), 4.576 (2.97), 4.596 (1.43), 6.416 (10.56), 6.438 (3.01), 6.647 (1.89), 6.754 (14.69), 7.010 (2.04), 7.138 (2.39), 7.168 (1.70), 7.172 (1.73), 7.188 (4.32), 7.203 (3.08), 7.206 (3.20), 7.266 (4.51), 7.270 (3.82), 7.286 (8.56), 7.290 (8.02), 7.304 (9.29), 7.309 (16.00), 7.312 (15.96), 7.330 (4.36), 8.020 (7.71), 8.574 (7.75), 9.330 (0.89).

### Example 161

### (3'RS)-2-[6-amino-5-(trifluoromethyl)pyridin-3-yl]-N-[(3S)-2,3-dihydro-1-benzofuran-3-yl]-6,7-dihydrospiro[pyrazolo[5,1-c][1,4]oxazine-4,3'-pyrrolidine]-1'-carboxamide (mixture of stereoisomers)

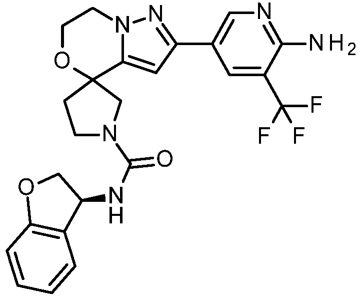

Example 161 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (75.0 mg, 200 µmol) and (3S)-2,3-dihydro-1-benzofuran-3-amine (29.7 mg, 220 µmol).
LC-MS (method 2): Rt = 1.02 min; MS (ESlpos): m/z = 501 [M+H]⁺
¹H-NMR (400MHz, DMSO-d₆): δ [ppm]= 2.15 - 2.28 (m, 1H), 2.30 - 2.39 (m, 1H), 3.38 - 3.42 (m, 1H), 3.47 (t, 1H), 3.54 - 3.66 (m, 1H), 3.82 (br t, 1H), 4.01 - 4.17 (m, 4H), 4.19 - 4.27 (m, 1H), 4.66 (t, 1H), 5.41 - 5.50 (m, 1H), 6.61 (br s, 2H), 6.74 (d, 1H), 6.77 - 6.84 (m, 2H), 6.88 (tdd, 1H), 7.15 - 7.22 (m, 1H), 7.32 (d, 1H), 8.01 (d, 1H), 8.58 (s, 1H).

### Example 162

### (rac)-2-[6-amino-5-(trifluoromethyl)pyridin-3-yl]-N-[(bicyclo[1.1.1]pentan-1-yl)methyl]-6,7-dihydrospiro[pyrazolo[5,1-c][1,4]oxazine-4,3'-pyrrolidine]-1'-carboxamide

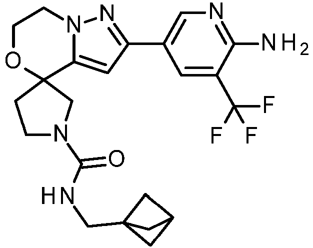

Example 162 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (70.0 mg, 186 µmol) and 1-(bicyclo[1.1.1]pentan-1-yl)methanamine-hydrochloride salt (27.4 mg, 205 µmol).
LC-MS (method 2): Rt = 1.02 min; MS (ESlpos): m/z = 463 [M+H]⁺
¹H-NMR (400 MHz, DMSO-d6) δ [ppm]: 1.623 (16.00), 2.435 (2.60), 2.518 (0.57), 3.057 (0.78), 3.061 (0.74), 3.075 (0.81), 3.431 (0.63), 3.460 (0.72), 3.766 (0.45), 4.117 (0.57), 4.132 (1.14), 6.113 (0.54), 6.599 (1.47), 6.741 (2.67), 8.009 (0.88), 8.014 (0.89), 8.580 (0.83), 8.585 (0.80).

### Example 163

### 2-[6-amino-5-(trifluoromethyl)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide (mixture of stereoisomers)

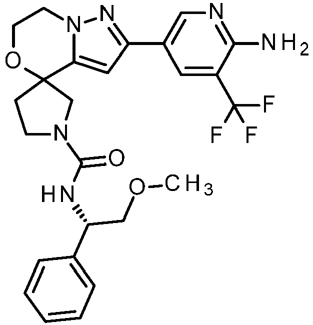

Example 163 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (70.0 mg, 186 µmol) and (1S)-2-methoxy-1-phenylethan-1-amine (31.0 mg, 205 µmol).
LC-MS (method 2): Rt = 1.01 min; MS (ESlpos): m/z = 517 [M+H]⁺
¹H-NMR (400 MHz, DMSO-d6) δ [ppm]: 2.323 (0.83), 2.327 (1.25), 2.332 (1.06), 2.337 (0.72), 2.350 (0.42), 2.518 (3.55), 2.523 (2.42), 2.665 (0.81), 2.669 (1.11), 2.674 (0.78), 3.257 (16.00), 3.291 (0.57), 3.400 (0.48), 3.429 (0.55), 3.438 (0.51), 3.443 (0.62), 3.453 (0.99), 3.463 (1.06), 3.468 (0.72), 3.478 (0.60), 3.491 (0.85), 3.520 (0.51), 3.546 (0.95), 3.567 (1.25), 3.571 (1.06), 3.592 (1.06), 3.627 (0.41), 3.786 (0.46), 3.815 (0.79), 4.074 (0.55), 4.094 (0.53), 4.111 (0.81), 4.119 (0.92), 4.124 (0.99), 4.136 (1.94), 4.945 (0.58), 4.960 (0.58), 6.495 (0.78), 6.515 (0.72), 6.599 (2.61), 6.754 (2.72), 6.758 (1.77), 7.221 (1.02), 7.237 (0.76), 7.240 (0.76), 7.285 (0.79), 7.289 (0.94), 7.300 (0.69), 7.303 (1.91), 7.308 (1.78), 7.321 (1.22), 7.326 (1.13), 7.346 (2.42), 7.362 (0.97), 7.367 (1.22), 8.009 (1.55), 8.576 (1.55).

### Example 164

### 2'-[6-amino-5-(trifluoromethyl)pyridin-3-yl]-N-[(1R)-1-(1,2-oxazol-3-yl)ethyl]-5',6'-dihydrospiro[azetidine-3,4'-pyrrolo[1,2-b]pyrazole]-1-carboxamide

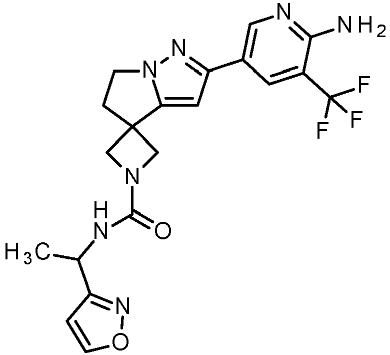

Example 164 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1:1-(1,2-oxazol-3-yl)ethan-1-amine-hydrochloride salt (43.2 mg, 291 µmol).
LC-MS (method 2): Rt = 0.87 min; MS (ESlpos): m/z = 448 [M+H]⁺
¹H-NMR (400MHz, DMSO-d₆): δ [ppm]= 1.41 (d, 3H), 2.85 (t, 2H), 3.98 - 4.07 (m, 4H), 4.13 (t, 2H), 4.91 - 5.03 (m, 1H), 6.49 - 6.58 (m, 3H), 6.74 (s, 1H), 6.98 (d, 1H), 8.04 (d, 1H), 8.62 (d, 1H), 8.81 (d, 1H).

### Example 165

### 2-[6-amino-5-(trifluoromethyl)pyridin-3-yl]-N-[(1R)-1-(2-fluorophenyl)ethyl]-6,7-dihydrospiro[pyrazolo[5,1-c][1,4]oxazine-4,3'-pyrrolidine]-1'-carboxamide (mixture of stereoisomers)

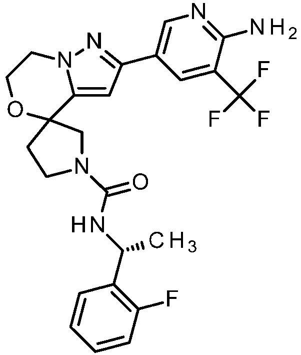

Example 165 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (100 mg, 266 µmol) and (1R)-1-(2-fluorophenyl)ethan-1-amine (44.4 mg, 319 µmol).
LC-MS (method 1): Rt = 1.08 min; MS (ESlpos): m/z = 505 [M+H]⁺
¹H-NMR (400 MHz, DMSO-d6) δ [ppm]: 0.934 (1.09), 0.951 (1.09), 1.028 (0.57), 1.035 (0.45), 1.046 (0.67), 1.237 (2.54), 1.253 (2.52), 1.345 (15.87), 1.363 (16.00), 2.198 (0.45), 2.222 (1.36), 2.247 (1.61), 2.272 (0.78), 2.327 (1.67), 2.331 (1.87), 2.349 (1.73), 2.522 (3.58), 2.664 (0.58), 2.668 (0.76), 2.673 (0.54), 3.383 (1.38), 3.394 (1.45), 3.408 (2.05), 3.423 (1.53), 3.433 (1.25), 3.449 (0.72), 3.466 (1.46), 3.494 (3.04), 3.522 (1.90), 3.577 (0.82), 3.599 (1.49), 3.620 (1.37), 3.641 (1.65), 3.664 (0.74), 3.794 (1.79), 3.822 (3.32), 3.849 (1.63), 4.049 (0.65), 4.063 (1.38), 4.079 (2.16), 4.098 (2.41), 4.115 (3.66), 4.122 (4.36), 4.128 (5.16), 4.140 (8.53), 4.239 (0.47), 4.256 (0.47), 5.068 (0.46), 5.088 (1.95), 5.106 (2.98), 5.124 (2.01), 5.142 (0.54), 6.564 (2.34), 6.576 (2.75), 6.584 (3.11), 6.602 (11.55), 6.758 (11.76), 7.089 (2.45), 7.111 (3.25), 7.137 (3.82), 7.151 (1.54), 7.156 (3.02), 7.160 (2.87), 7.169 (3.21), 7.172 (3.13), 7.178 (2.12), 7.188 (2.13), 7.191 (1.78), 7.221 (1.20), 7.226 (2.08), 7.230 (1.42), 7.234 (1.36), 7.239 (2.65), 7.244 (2.74), 7.259 (2.38), 7.263 (1.91), 7.277 (0.95), 7.422 (1.07), 7.426 (1.13), 7.441 (2.67), 7.445 (2.15), 7.454 (2.27), 7.459 (2.59), 7.474 (1.12), 7.561 (0.41), 8.008 (3.99), 8.013 (7.02), 8.018 (4.08), 8.575 (3.49), 8.582 (5.16), 8.587 (3.56).

### Example 166

### (rac)-2-[6-amino-5-(trifluoromethyl)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide

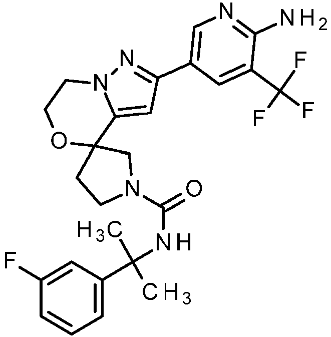

Example 166 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (100 mg, 266 µmol) and 2-(3-fluorophenyl)propan-2-amine (48.9 mg, 319 µmol).
LC-MS (method 1): Rt = 1.16 min; MS (ESlpos): m/z = 520 [M+H]⁺
¹H-NMR (400 MHz, DMSO-d6) δ [ppm]: 0.934 (2.46), 0.951 (2.43), 1.401 (0.63), 1.425 (0.53), 1.549 (16.00), 1.555 (15.67), 2.221 (0.70), 2.247 (0.99), 2.271 (0.53), 2.318 (0.99), 2.322 (1.01), 2.327 (1.21), 2.332 (1.32), 2.348 (0.58), 2.365 (0.51), 2.518 (1.97), 2.523 (1.23), 2.669 (0.48), 3.370 (0.60), 3.394 (0.91), 3.412 (0.88), 3.437 (0.43), 3.484 (1.12), 3.513 (1.32), 3.587 (0.70), 3.608 (1.20), 3.631 (0.56), 3.790 (1.47), 3.819 (1.25), 4.076 (0.41), 4.090 (0.97), 4.107 (1.72), 4.123 (3.63), 4.131 (4.88), 4.143 (4.62), 6.152 (4.56), 6.599 (6.18), 6.765 (9.03), 6.938 (0.78), 6.943 (0.89), 6.958 (1.57), 6.965 (1.75), 6.981 (0.90), 6.985 (0.99), 7.105 (1.20), 7.110 (1.65), 7.115 (1.30), 7.133 (1.20), 7.138 (1.67), 7.143 (1.31), 7.176 (1.87), 7.197 (2.47), 7.275 (1.37), 7.290 (1.60), 7.294 (2.24), 7.310 (2.14), 7.314 (1.18), 7.330 (0.89), 8.013 (3.59), 8.019 (3.63), 8.583 (3.42), 8.587 (3.37).

### Example 167

### 2'-[6-amino-5-(trifluoromethyl)pyridin-3-yl]-N-[(1R)-1-(1-phenyl-1H-pyrazol-4-yl)ethyl]-5',6'-dihydrospiro[azetidine-3,4'-pyrrolo[1,2-b]pyrazole]-1-carboxamide

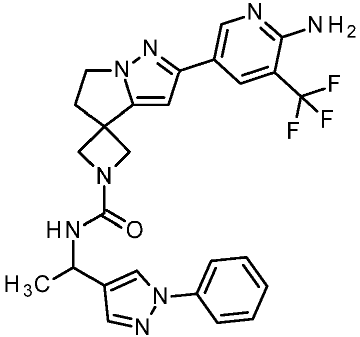

Example 167 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(1-phenyl-1H-pyrazol-4-yl)ethan-1-amine (54.5 mg, 291 µmol).
LC-MS (method 2): Rt = 1.06 min; MS (ESlpos): m/z = 523 [M+H]⁺
¹H-NMR (400MHz, DMSO-d₆): δ [ppm]= 1.43 (d, 3H), 2.85 (t, 2H), 3.99 - 4.07 (m, 4H), 4.08 - 4.18 (m, 2H), 4.88 (quin, 1H), 6.55 (s, 2H), 6.72 - 6.79 (m, 2H), 7.25 - 7.32 (m, 1H), 7.43 - 7.53 (m, 2H), 7.68 (s, 1H), 7.78 - 7.84 (m, 2H), 8.04 (d, 1H), 8.35 (s, 1H), 8.62 (d, 1H).

### Example 168

### 2'-[6-amino-5-(trifluoromethyl)pyridin-3-yl]-N-[(1S)-2-methoxy-1-phenylethyl]-5',6'-dihydrospiro[azetidine-3,4'-pyrrolo[1,2-b]pyrazole]-1-carboxamide

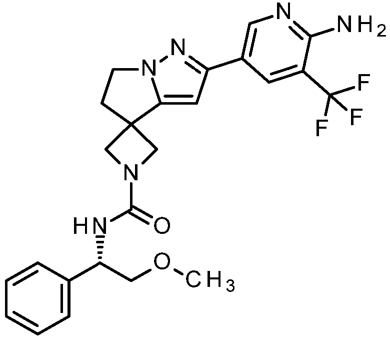

Example 168 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1S)-2-methoxy-1-phenylethan-1-amine (44.0 mg, 291 µmol).
LC-MS (method 2): Rt = 1.03 min; MS (ESlpos): m/z = 487 [M+H]⁺
¹H-NMR (400MHz, DMSO-d₆): δ [ppm]= 2.85 (t, 2H), 3.27 (s, 3H), 3.41 - 3.48 (m, 1H), 3.51 - 3.58 (m, 1H), 3.97 - 4.07 (m, 4H), 4.08 - 4.18 (m, 2H), 4.92 (td, 1H), 6.55 (s, 2H), 6.72 (s, 1H), 6.91 (d, 1H), 7.20 - 7.27 (m, 1H), 7.29 - 7.41 (m, 4H), 8.04 (d, 1H), 8.62 (d, 1H).

### Example 169

### 2'-[6-amino-5-(trifluoromethyl)pyridin-3-yl]-N-[(3-fluorobicyclo[1.1.1]pentan-1-yl)methyl]-5',6'-dihydrospiro[azetidine-3,4'-pyrrolo[1,2-b]pyrazole]-1-carboxamide

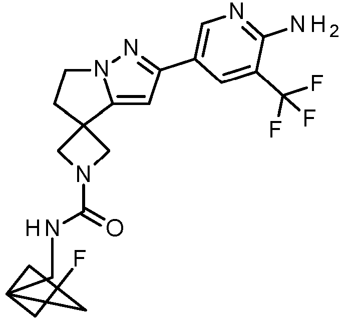

Example 169 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(3-fluorobicyclo[1.1.1]pentan-1-yl)methanamine-hydrochloride salt (44.1 mg, 291 µmol).
LC-MS (method 2): Rt = 0.97 min; MS (ESlpos): m/z = 451 [M+H]⁺
¹H-NMR (400MHz, DMSO-d₆): δ [ppm]= 1.86 - 1.94 (m, 8H), 2.85 (t, 2H), 3.95 - 4.03 (m, 4H), 4.12 (t, 2H), 6.55 (s, 2H), 6.62 (t, 1H), 6.72 (s, 1H), 8.04 (d, 1H), 8.62 (d, 1H).

### Example 170

### 2'-[6-amino-5-(trifluoromethyl)pyridin-3-yl]-N-[(bicyclo[1.1.1]pentan-1-yl)methyl]-5',6'-dihydrospiro[azetidine-3,4'-pyrrolo[1,2-b]pyrazole]-1-carboxamide

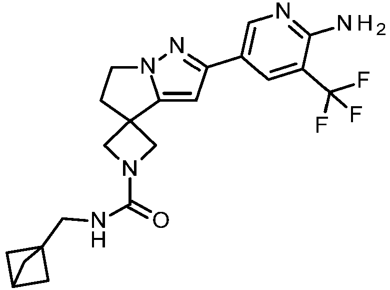

Example 170 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(bicyclo[1.1.1]pentan-1-yl)methanamine-hydrochloride salt (38.9 mg, 291 µmol).
LC-MS (method 2): Rt = 1.03 min; MS (ESlpos): m/z = 433 [M+H]⁺
¹H-NMR (400MHz, DMSO-d₆): δ [ppm]= 1.63 (s, 6H), 2.45 (s, 1H), 2.84 (t, 2H), 3.04 (d, 2H), 3.94 - 4.02 (m, 4H), 4.12 (t, 2H), 6.47 (t, 1H), 6.54 (s, 2H), 6.72 (s, 1H), 8.04 (d, 1H), 8.62 (d, 1H).

### Example 171

### 2'-[6-amino-5-(trifluoromethyl)pyridin-3-yl]-N-[(1S)-1-(pyridin-3-yl)ethyl]-5',6'-dihydrospiro[azetidine-3,4'-pyrrolo[1,2-b]pyrazole]-1-carboxamide

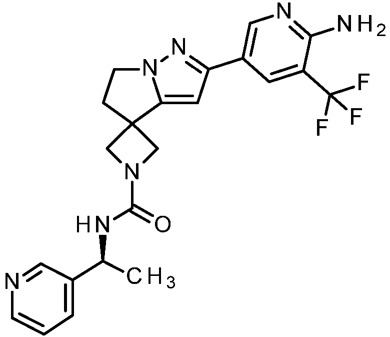

Example 171 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1S)-1-(pyridin-3-yl)ethan-1-amine-hydrochloride salt (56.8 mg, 291 µmol).
LC-MS (method 2): Rt = 0.69 min; MS (ESlpos): m/z = 458 [M+H]⁺
¹H-NMR (400MHz, DMSO-d₆): δ [ppm]= 1.39 (d, 3H), 2.85 (t, 2H), 3.99 - 4.08 (m, 4H), 4.13 (t, 2H), 4.84 (quin, 1H), 6.55 (s, 2H), 6.73 (s, 1H), 6.97 (d, 1H), 7.35 (dd, 1H), 7.74 (dt, 1H), 8.04 (d, 1H), 8.43 (dd, 1H), 8.56 (d, 1H), 8.62 (d, 1H).

### Example 172

### 2'-[6-amino-5-(trifluoromethyl)pyridin-3-yl]-N-[(1R)-1-(2-methylpyrimidin-5-yl)ethyl]-5',6'-dihydrospiro[azetidine-3,4'-pyrrolo[1,2-b]pyrazole]-1-carboxamide

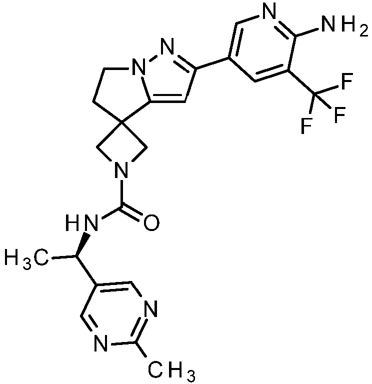

Example 172 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1R)-1-(2-methylpyrimidin-5-yl)ethan-1-amine (39.9 mg, 291 µmol).
LC-MS (method 2): Rt = 0.82 min; MS (ESlpos): m/z = 473 [M+H]⁺
¹H-NMR (400MHz, DMSO-d₆): δ [ppm]= 1.41 (d, 3H), 2.59 (s, 3H), 2.85 (t, 2H), 3.97 - 4.07 (m, 4H), 4.09 - 4.16 (m, 2H), 4.81 (quin, 1H), 6.55 (s, 2H), 6.72 (s, 1H), 6.98 (d, 1H), 8.04 (d, 1H), 8.62 (d, 1H), 8.65 (s, 1H).

### Example 173

### 2'-[6-amino-5-(trifluoromethyl)pyridin-3-yl]-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide

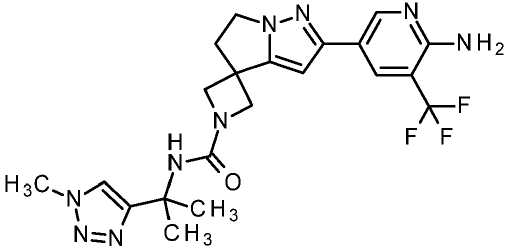

Example 173 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(1-methyl-1H-1,2,3-triazol-4-yl)propan-2-amine-hydrochloride salt (62.0 mg, 291 µmol).
LC-MS (method 2): Rt = 0.83 min; MS (ESlpos): m/z = 476 [M+H]⁺
¹H-NMR (400MHz, DMSO-d₆): δ [ppm]= 1.59 (s, 6H), 2.83 (t, 2H), 3.94 - 4.03 (m, 7H), 4.12 (t, 2H), 6.41 (s, 1H), 6.55 (s, 2H), 6.71 (s, 1H), 7.80 (s, 1H), 8.04 (d, 1H), 8.62 (d, 1H).

### Example 174

### 2'-[6-amino-5-(trifluoromethyl)pyridin-3-yl]-N-[2-(3-chloropyridin-4-yl)propan-2-yl]-5',6'-dihydrospiro[azetidine-3,4'-pyrrolo[1,2-b]pyrazole]-1-carboxamide

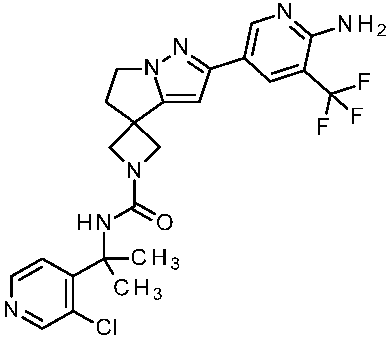

Example 174 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-(3-chloropyridin-4-yl)propan-2-amine (49.7 mg, 291 µmol).
LC-MS (method 2): Rt = 0.94 min; MS (ESlpos): m/z = 506 [M+H]⁺
¹H-NMR (400MHz, DMSO-d₆): δ [ppm]= 1.65 (s, 6H), 2.83 (t, 2H), 4.00 (s, 4H), 4.12 (t, 2H), 6.55 (s, 2H), 6.68 (s, 1H), 6.86 (s, 1H), 7.44 (d, 1H), 8.05 (d, 1H), 8.42 (d, 1H), 8.47 (s, 1H), 8.63 (d, 1H).

### Example 175

### 2'-[6-amino-5-(trifluoromethyl)pyridin-3-yl]-N-[1-(pyridin-4-yl)cyclobutyl]-5',6'-dihydrospiro[azetidine-3,4'-pyrrolo[1,2-b]pyrazole]-1-carboxamide

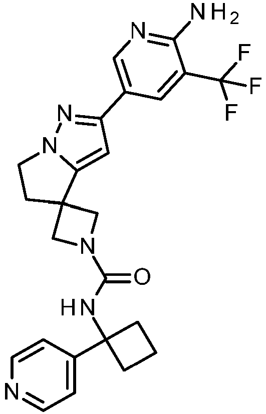

Example 175 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-(pyridin-4-yl)cyclobutan-1-amine (43.1 mg, 291 µmol).
LC-MS (method 2): Rt = 0.67 min; MS (ESlpos): m/z = 484 [M+H]⁺
¹H-NMR (400MHz, DMSO-d₆): δ [ppm]= 1.78 - 1.91 (m, 1H), 1.97 - 2.11 (m, 1H), 2.34 - 2.48 (m, 3H), 2.85 (t, 2H), 3.97 - 4.07 (m, 4H), 4.13 (t, 2H), 6.55 (s, 2H), 6.73 (s, 1H), 7.25 (s, 1H), 7.37 - 7.43 (m, 2H), 8.05 (d, 1H), 8.16 (s, 1H), 8.51 (d, 2H), 8.63 (d, 1H).

### Example 176

### 2'-[6-amino-5-(trifluoromethyl)pyridin-3-yl]-N-[(1R)-1-phenylethyl]-5',6'-dihydrospiro[azetidine-3,4'-pyrrolo[1,2-b]pyrazole]-1-carboxamide

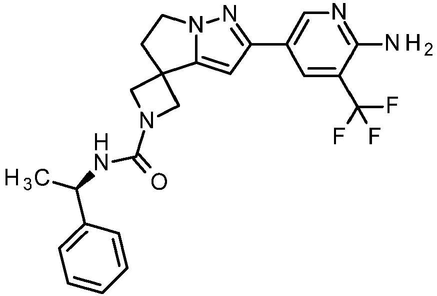

Example 176 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and (1R)-1-phenylethan-1-amine (35.3 mg, 291 µmol).
LC-MS (method 2): Rt = 1.04 min; MS (ESlpos): m/z = 457 [M+H]⁺
¹H-NMR (400MHz, DMSO-d₆): δ [ppm]= 1.36 (d, 3H), 2.85 (t, 2H), 3.97 - 4.07 (m, 4H), 4.13 (t, 2H), 4.76 - 4.86 (m, 1H), 6.55 (s, 2H), 6.72 (s, 1H), 6.89 (d, 1H), 7.17 - 7.24 (m, 1H), 7.28 - 7.38 (m, 4H), 8.04 (d, 1H), 8.62 (d, 1H).

### Example 177

### 2-[6-amino-5-(trifluoromethyl)pyridin-3-yl]-N-[(1S)-1-(pyridin-3-yl)ethyl]-6,7-dihydrospiro[pyrazolo[5,1-c][1,4]oxazine-4,3'-pyrrolidine]-1'-carboxamide

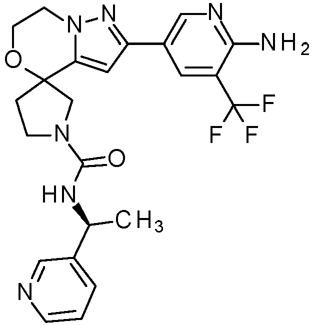

Example 177 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (70.0 mg, 186 µmol) and (1S)-1-(pyridin-3-yl)ethan-1-amine-hydrochloride salt (40.0 mg, 205 µmol).
LC-MS (method 2): Rt = 0.73 min; MS (ESlpos): m/z = 488 [M+H]⁺
¹H-NMR (400 MHz, DMSO-d6) δ [ppm]: 1.232 (1.55), 1.393 (16.00), 1.411 (16.00), 2.085 (0.40), 2.214 (1.26), 2.241 (1.43), 2.263 (0.63), 2.273 (0.63), 2.323 (3.10), 2.327 (4.53), 2.332 (3.73), 2.337 (2.35), 2.518 (11.35), 2.523 (7.68), 2.540 (0.63), 2.660 (1.09), 2.665 (2.52), 2.669 (3.50), 2.674 (2.47), 2.679 (1.09), 3.378 (2.92), 3.396 (2.92), 3.411 (1.84), 3.422 (1.66), 3.437 (0.92), 3.454 (1.95), 3.485 (3.15), 3.516 (1.89), 3.563 (0.86), 3.585 (1.43), 3.606 (1.43), 3.625 (1.78), 3.649 (0.80), 3.776 (1.66), 3.806 (1.55), 3.812 (2.12), 3.842 (1.66), 4.047 (0.69), 4.070 (1.61), 4.077 (1.89), 4.085 (1.89), 4.109 (3.21), 4.122 (4.42), 4.135 (8.66), 4.832 (0.40), 4.851 (1.66), 4.869 (2.29), 4.888 (1.55), 6.577 (2.81), 6.597 (13.71), 6.612 (2.75), 6.751 (8.89), 6.756 (9.35), 7.315 (1.66), 7.319 (2.12), 7.321 (2.12), 7.327 (1.84), 7.333 (3.67), 7.338 (2.35), 7.340 (2.29), 7.347 (1.95), 7.350 (2.24), 7.352 (2.24), 7.723 (1.20), 7.728 (3.27), 7.733 (3.56), 7.738 (1.72), 7.743 (1.32), 7.747 (2.92), 7.753 (3.15), 7.758 (1.38), 8.005 (4.24), 8.010 (6.94), 8.194 (4.24), 8.406 (3.10), 8.409 (5.16), 8.413 (3.90), 8.418 (3.21), 8.421 (5.05), 8.425 (3.50), 8.548 (6.65), 8.554 (6.59), 8.572 (3.73), 8.577 (6.02).

### Example 178

### (rac)-2-[6-amino-5-(trifluoromethyl)pyridin-3-yl]-N-[(3-fluorobicyclo[1.1.1]pentan-1-yl)methyl]-6,7-dihydrospiro[pyrazolo[5,1-c][1,4]oxazine-4,3'-pyrrolidine]-1'-carboxamide

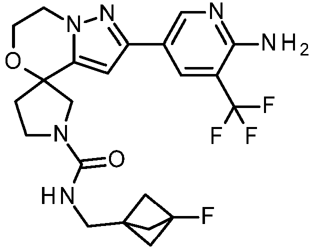

Example 178 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (70.0 mg, 186 µmol) and 1-(3-fluorobicyclo[1.1.1]pentan-1-yl)methanamine-hydrochloride salt (31.1 mg, 205 µmol).
LC-MS (method 2): Rt = 0.96 min; MS (ESlpos): m/z = 481 [M+H]⁺
¹H-NMR (400 MHz, DMSO-d6) δ [ppm]: 1.886 (16.00), 1.892 (15.84), 2.074 (1.51), 2.214 (0.43), 2.241 (0.58), 2.322 (0.62), 2.326 (0.55), 2.337 (0.63), 2.518 (1.10), 2.522 (0.74), 3.334 (12.35), 3.355 (2.11), 3.360 (2.34), 3.379 (0.85), 3.388 (0.57), 3.431 (1.41), 3.459 (1.63), 3.528 (0.51), 3.550 (0.89), 3.761 (0.98), 3.789 (0.84), 4.061 (0.54), 4.077 (0.81), 4.096 (0.81), 4.109 (0.89), 4.117 (1.34), 4.132 (2.67), 6.266 (0.57), 6.281 (1.15), 6.295 (0.57), 6.600 (3.39), 6.746 (5.91), 8.009 (2.05), 8.014 (2.05), 8.133 (1.48), 8.580 (1.93), 8.585 (1.86).

### Example 179

### 2-[6-amino-5-(trifluoromethyl)pyridin-3-yl]-N-[(1S)-1-(pyridin-4-yl)ethyl]-6,7-dihydrospiro[pyrazolo[5,1-c][1,4]oxazine-4,3'-pyrrolidine]-1'-carboxamide (mixture of stereoisomers)

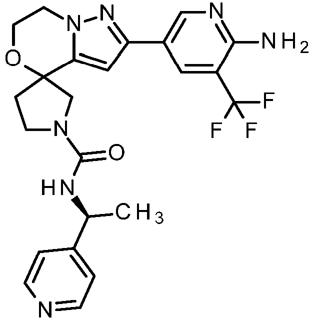

Example 179 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride salt (70.0 mg, 186 µmol) and (1S)-1-(pyridin-4-yl)ethan-1-amine (25.0 mg, 205 µmol).
LC-MS (method 2): Rt = 0.66 min; MS (ESlpos): m/z = 488 [M+H]⁺
¹H-NMR (400 MHz, DMSO-d6) δ [ppm]: 0.852 (0.41), 1.232 (1.50), 1.359 (15.45), 1.378 (15.66), 2.225 (1.16), 2.252 (1.44), 2.273 (0.68), 2.318 (1.37), 2.323 (3.15), 2.327 (4.58), 2.332 (3.62), 2.337 (2.46), 2.352 (1.44), 2.386 (0.82), 2.518 (16.00), 2.523 (10.32), 2.540 (0.96), 2.660 (1.30), 2.665 (3.08), 2.669 (4.24), 2.673 (2.94), 2.679 (1.37), 3.388 (0.96), 3.414 (1.85), 3.431 (1.50), 3.466 (0.96), 3.504 (2.05), 3.534 (2.32), 3.576 (1.23), 3.596 (2.05), 3.622 (1.16), 3.649 (0.89), 3.672 (0.41), 3.788 (2.39), 3.814 (2.05), 3.828 (1.09), 3.858 (0.89), 4.053 (0.82), 4.067 (1.37), 4.084 (1.98), 4.102 (2.19), 4.118 (3.15), 4.126 (4.65), 4.131 (4.38), 4.139 (7.93), 4.789 (1.64), 4.808 (2.39), 4.826 (1.64), 6.603 (10.87), 6.625 (3.83), 6.645 (2.60), 6.760 (4.92), 6.767 (11.83), 7.321 (7.59), 7.326 (6.70), 7.332 (6.29), 7.337 (8.82), 7.343 (3.56), 8.015 (6.36), 8.021 (4.79), 8.217 (0.48), 8.472 (9.57), 8.477 (8.27), 8.483 (6.22), 8.487 (10.39), 8.492 (4.17), 8.583 (5.54), 8.589 (4.38).

### Example 180

### 2'-[6-amino-5-(trifluoromethyl)pyridin-3-yl]-N-(1-phenylcyclobutyl)-5',6'-dihydrospiro[azetidine-3,4'-pyrrolo[1,2-b]pyrazole]-1-carboxamide

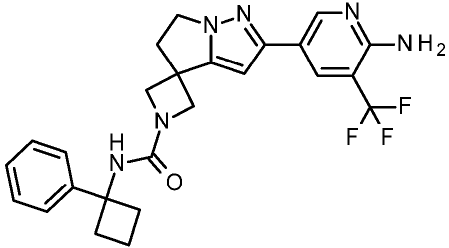

Example 180 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1-phenylcyclobutan-1-amine-hydrochloride salt (49.0 mg, 267 µmol).
LC-MS (method 1): Rt = 1.17 min; MS (ESlpos): m/z = 483 [M+H]⁺
¹H-NMR (400MHz, DMSO-d₆): δ [ppm]= 1.78 (ddt, 1H), 1.96 - 2.07 (m, 1H), 2.35 - 2.48 (m, 4H), 2.84 (t, 2H), 3.95 - 4.03 (m, 4H), 4.12 (t, 2H), 6.55 (s, 2H), 6.70 (s, 1H), 7.08 (s, 1H), 7.15 - 7.22 (m, 1H), 7.28 - 7.35 (m, 2H), 7.40 - 7.46 (m, 2H), 8.04 (d, 1H), 8.62 (d, 1H).

### Example 181

### 2'-(6-amino-5-cyanopyridin-3-yl)-N-[1-(pyridin-4-yl)cyclobutyl]-5',6'-dihydrospiro[azetidine-3,4'-pyrrolo[1,2-b]pyrazole]-1-carboxamide

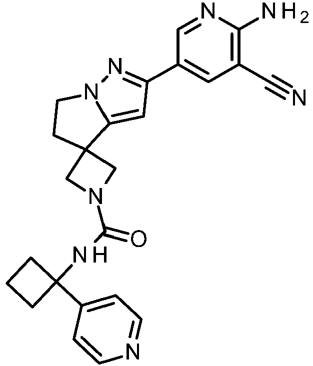

Example 181 was prepared in analogy to Example 31 using 2-amino-5-(5',6'-dihydrospiro[azetidine-3,4'-pyrrolo[1,2-b]pyrazol]-2'-yl)pyridine-3-carbonitrile hydrochloride (75.0 mg, 248 µmol) and 1-(pyridin-4-yl)cyclobutan-1-amine (44.1 mg, 297 µmol).
¹H NMR (400 MHz, DMSO-*d*₆) δ ppm 1.79 - 1.90 (m, 1 H) 1.98 - 2.09 (m, 1 H) 2.36 - 2.47 (m, 4 H) 2.82 - 2.89 (m, 2 H) 4.02 (q, 4 H) 4.13 (t, 2 H) 6.69 (s, 1 H) 7.00 (s, 2 H) 7.26 (s, 1 H) 7.41 (d, 2 H) 8.17 (d, 1 H) 8.52 (d, 2 H) 8.65 (d, 1 H)
LC-MS (method 1): Rt = 0.81 min; MS (ESlneg): m/z = 439 [M-H]⁻

### Example 182

### 2'-(6-amino-5-cyanopyridin-3-yl)-N-[1-(3-fluorophenyl)cyclobutyl]-5',6'-dihydrospiro[azetidine-3,4'-pyrrolo[1,2-b]pyrazole]-1-carboxamide

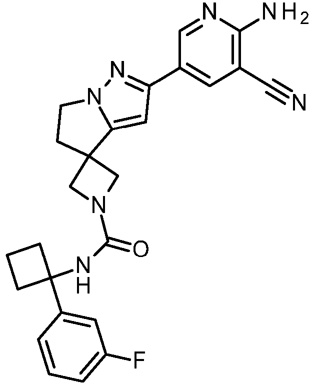

Example 182 was prepared in analogy to Example 31 using 2-amino-5-(5',6'-dihydrospiro[azetidine-3,4'-pyrrolo[1,2-b]pyrazol]-2'-yl)pyridine-3-carbonitrile hydrochloride (75.0 mg, 248 µmol) and 1-(3-fluorophenyl)cyclobutan-1-amine hydrochloride (59.9 mg, 297 µmol).
¹H NMR (400 MHz, DMSO-*d*₆) δ ppm 1.74 - 1.85 (m, 1 H) 1.96 - 2.09 (m, 1 H) 2.36 - 2.48 (m, 4 H) 2.84 (t, 2 H) 4.00 (q, 4 H) 4.12 (t, 2 H) 6.67 (s, 1 H) 6.96 - 7.05 (m, 3 H) 7.12 - 7.16 (m, 1 H) 7.19 (dt, 1 H) 7.26 (d, 1 H) 7.36 (td, 1 H) 8.16 (d, 1 H) 8.64 (d, 1 H)
LC-MS (method 1): Rt = 1.04 min; MS (ESlneg): m/z = 456 [M-H]⁻

### Example 183

### 2'-(6-amino-5-cyanopyridin-3-yl)-N-[2-(3-chloropyridin-4-yl)propan-2-yl]-5',6'-dihydrospiro[azetidine-3,4'-pyrrolo[1,2-b]pyrazole]-1-carboxamide

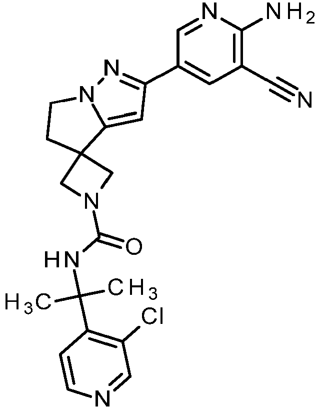

Example 183 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-(3-chloropyridin-4-yl)propan-2-amine (57.7 mg, 338 µmol).
¹H NMR (400 MHz, DMSO-*d*₆) δ ppm 1.64 (s, 5 H) 2.52 - 2.56 (m, 1 H) 2.78 - 2.89 (m, 2 H) 3.99 (d, 3 H) 4.07 - 4.21 (m, 2 H) 6.60 - 6.72 (m, 1 H) 6.87 (s, 1 H) 6.94 - 7.09 (m, 2 H) 7.44 (d, 1 H) 8.18 (d, 1 H) 8.43 (d, 1 H) 8.48 (s, 1 H) 8.65 (d, 1 H)
LC-MS (method 1): Rt = 0.88 min; MS (ESlpos): m/z = 463 [M+H]⁺

### Example 184

### 2'-(6-amino-5-cyanopyridin-3-yl)-N-[(1R)-1-(5-fluoropyridin-3-yl)ethyl]-5',6'-dihydrospiro[azetidine-3,4'-pyrrolo[1,2-b]pyrazole]-1-carboxamide

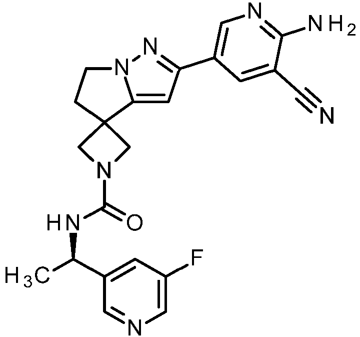

Example 184 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and (1R)-1-(5-fluoropyridin-3-yl)ethan-1-amine (47.4 mg, 338 µmol).
¹H NMR (400 MHz, DMSO-*d*₆) δ ppm 1.40 (d, 3 H) 2.85 (t, 2 H) 3.98 - 4.04 (m, 3 H) 4.05 - 4.09 (m, 1 H) 4.12 (t, 2 H) 4.89 (quin, 1 H) 6.68 (s, 1 H) 6.99 - 7.01 (m, 2 H) 7.66 (s, 1 H) 7.68 - 7.70 (m, 1 H) 8.15 (d, 1 H) 8.43 - 8.47 (m, 2 H) 8.63 (d, 1 H)
LC-MS (method 1): Rt = 0.81 min; MS (ESlpos): m/z = 433 [M+H]⁺

### Example 185

### 2'-(6-amino-5-cyanopyridin-3-yl)-N-[(1R)-1-(4-fluorophenyl)ethyl]-5',6'-dihydrospiro[azetidine-3,4'-pyrrolo[1,2-b]pyrazole]-1-carboxamide

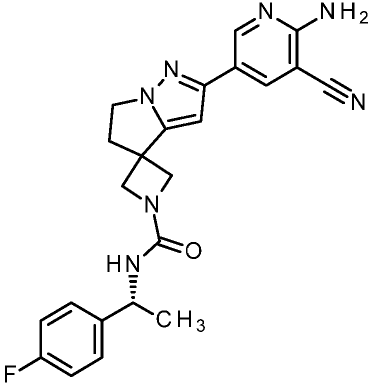

Example 185 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and (1R)-1-(4-fluorophenyl)ethan-1-amine (47.0 mg, 338 µmol).
¹H NMR (400 MHz, DMSO-*d*₆) δ ppm 1.35 (d, 3 H) 2.84 (t, 2 H) 3.96 - 4.06 (m, 4 H) 4.12 (t, 2 H) 4.81 (quin, 1 H) 6.68 (s, 1 H) 6.91 (d, 1 H) 6.95 - 7.07 (m, 2 H) 7.13 (t, 2 H) 7.36 (t, 2 H) 8.15 (d, 1 H) 8.64 (d, 1 H)
LC-MS (method 1): Rt = 0.97 min; MS (ESlneg): m/z = 430 [M-H]⁻

### Example 186

### 2'-(6-amino-5-cyanopyridin-3-yl)-N-[2-(3-fluorophenyl)propan-2-yl]-5',6'-dihydrospiro[azetidine-3,4'-pyrrolo[1,2-b]pyrazole]-1-carboxamide

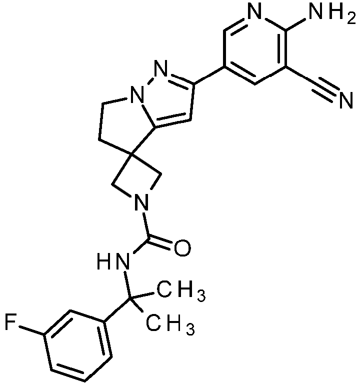

Example 186 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-(3-fluorophenyl)propan-2-amine (51.8 mg, 338 µmol).
¹H NMR (400 MHz, DMSO-*d*₆) δ ppm 1.55 (s, 6 H) 2.81 - 2.89 (m, 2 H) 3.97 - 4.07 (m, 4 H) 4.13 (t, 2 H) 6.63 (s, 1 H) 6.67 (s, 1 H) 6.95 - 7.04 (m, 3 H) 7.13 (dt, 1 H) 7.20 (d, 1 H) 7.33 (td, 1 H) 8.17 (d, 1 H) 8.65 (d, 1 H)
LC-MS (method 1): Rt = 1.04 min; MS (ESlpos): m/z = 446 [M+H]⁺

### Example 187

### 2'-(6-amino-5-cyanopyridin-3-yl)-N-[(1S)-1-(3-chloropyridin-4-yl)ethyl]-5',6'-dihydrospiro[azetidine-3,4'-pyrrolo[1,2-b]pyrazole]-1-carboxamide

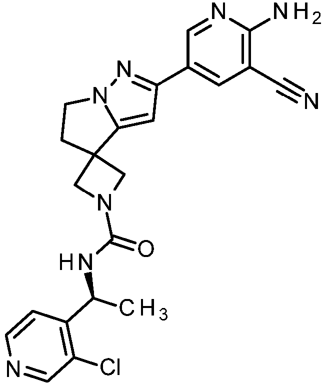

Example 187 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and (1S)-1-(3-chloropyridin-4-yl)ethan-1-amine hydrochloride (65.3 mg, 338 µmol).
1H NMR (400 MHz, DMSO-d6) δ ppm 2.86 (t, 2 H) 4.00 - 4.06 (m, 3 H) 4.07 - 4.16 (m, 3 H) 5.04 (quin, 1 H) 6.69 (s, 1 H) 6.77 (d, 1 H) 7.00 (s, 2 H) 7.20 (d, 1 H) 7.39 (d, 1 H) 7.49 (d, 1 H) 8.16 (d, 1 H) 8.50 - 8.54 (m, 2 H) 8.55 (s, 1 H) 8.64 (d, 1 H)
LC-MS (method 1): Rt = 0.85 min; MS (ESlpos): m/z = 449 [M+H]⁺

### Example 188

### 2'-(6-amino-5-cyanopyridin-3-yl)-N-[(1-methyl-2-oxabicyclo[2.1.1]hexan-4-yl)methyl]-5',6'-dihydrospiro[azetidine-3,4'-pyrrolo[1,2-b]pyrazole]-1-carboxamide

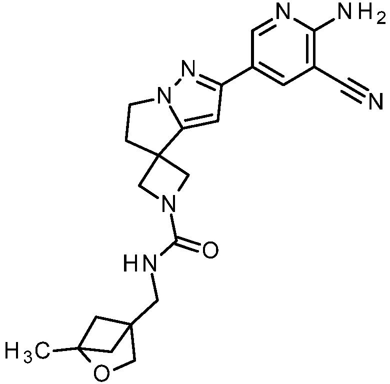

Example 188 was prepared in analogy to Example 31 using 2-amino-5-(5',6'-dihydrospiro[azetidine-3,4'-pyrrolo[1,2-b]pyrazol]-2'-yl)pyridine-3-carbonitrile hydrochloride (75.0 mg, 248 µmol) and 1-(1-methyl-2-oxabicyclo[2.1.1]hexan-4-yl)methanamine (37.8 mg, 297 µmol).
¹H NMR (400 MHz, DMSO-*d*₆) δ ppm 1.23 - 1.29 (m, 3 H) 1.31 (s, 3 H) 1.36 (dd, 2 H) 1.44 - 1.55 (m, 2 H) 2.80 - 2.89 (m, 2 H) 3.47 (s, 2 H) 3.95 - 4.01 (m, 3 H) 4.12 (t, 2 H) 6.62 (s, 1 H) 6.67 (s, 1 H) 6.92 - 7.08 (m, 2 H) 8.16 (d, 1 H) 8.64 (d, 1 H)
LC-MS (method 1): Rt = 0.75 min; MS (ESlpos): m/z = 420 [M+H]⁺

### Example 189

### 2'-(6-amino-5-cyanopyridin-3-yl)-N-[(1R)-1-(4-cyanopyridin-2-yl)ethyl]-5',6'-dihydrospiro[azetidine-3,4'-pyrrolo[1,2-b]pyrazole]-1-carboxamide

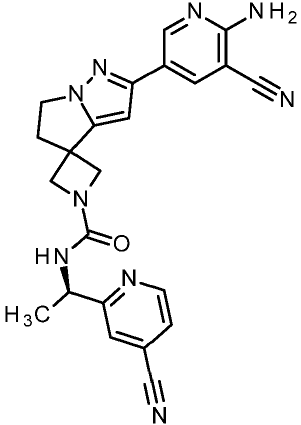

Example 189 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-[(1R)-1-aminoethyl]pyridine-4-carbonitrile hydrochloride (62.1 mg, 338 µmol).
¹H NMR (400 MHz, DMSO-*d*₆) δ ppm 1.41 (d, 3 H) 2.85 (t, 2 H) 3.98 - 4.06 (m, 3 H) 4.06 - 4.16 (m, 3 H) 4.90 (quin, 1 H) 6.68 (s, 1 H) 6.96 - 7.05 (m, 3 H) 8.15 (d, 1 H) 8.26 (t, 1 H) 8.63 (d, 1 H) 8.85 (d, 1 H) 8.91 (d, 1 H)
LC-MS (method 1): Rt = 0.79 min; MS (ESlpos): m/z = 440 [M+H]⁺

### Example 190

### 2'-(6-amino-5-cyanopyridin-3-yl)-N-[(1R)-1-(2-cyanopyridin-4-yl)ethyl]-5',6'-dihydrospiro[azetidine-3,4'-pyrrolo[1,2-b]pyrazole]-1-carboxamide

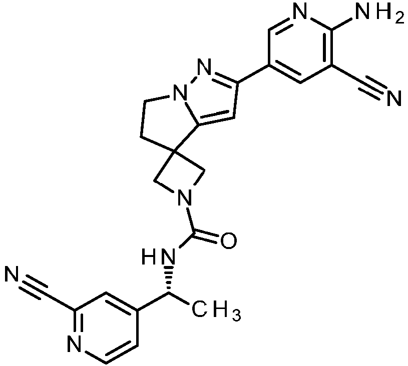

Example 190 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 4-[(1R)-1-aminoethyl]pyridine-2-carbonitrile (49.7 mg, 338 µmol).
¹H NMR (400 MHz, DMSO-*d*₆) δ ppm 1.41 (d, 3 H) 2.84 (t, 2 H) 3.98 - 4.06 (m, 3 H) 4.06 - 4.17 (m, 3 H) 4.89 (quin, 1 H) 6.68 (s, 1 H) 7.00 (s, 2 H) 7.15 (d, 1 H) 8.15 (d, 1 H) 8.29 (t, 1 H) 8.63 (d, 1 H) 8.86 (d, 1 H) 8.90 (d, 1 H)
LC-MS (Method 1): Rt = 0.79 min; MS (ESlpos): m/z = 440 [M+H]⁺

### Example 191

### 2'-(6-amino-5-cyanopyridin-3-yl)-N-[(1R)-1-(2-chloro-4-cyanophenyl)ethyl]-5',6'-dihydrospiro[azetidine-3,4'-pyrrolo[1,2-b]pyrazole]-1-carboxamide

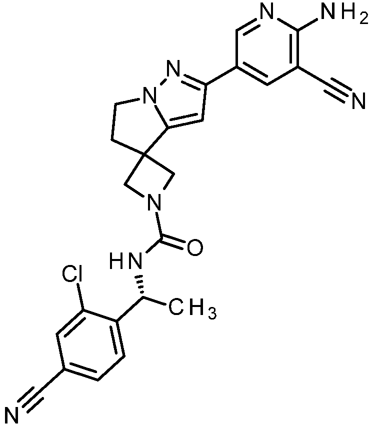

Example 191 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 4-[(1R)-1-aminoethyl]-3-chlorobenzonitrile (61.0 mg, 338 µmol).
¹H NMR (400 MHz, DMSO-*d*₆) δ ppm 1.33 (d, 3 H) 2.85 (t, 2 H) 3.99 - 4.05 (m, 2 H) 4.05 - 4.19 (m, 3 H) 5.11 (quin, 1 H) 6.54 (s, 1 H) 6.69 (s, 1 H) 7.00 (s, 2 H) 7.20 (d, 1 H) 7.66 (d, 1 H) 7.87 (dd, 1 H) 7.94 - 8.03 (m, 1 H) 8.13 - 8.19 (m, 1 H) 8.61 - 8.67 (m, 1 H)
LC-MS (method 1): Rt = 1.00 min; MS (ESlpos): m/z = 473 [M+H]⁺

### Example 192

### 2'-(6-amino-5-cyanopyridin-3-yl)-N-[1-(2-fluorophenyl)cyclobutyl]-5',6'-dihydrospiro[azetidine-3,4'-pyrrolo[1,2-b]pyrazole]-1-carboxamide

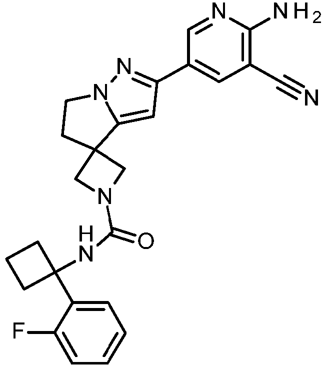

Example 192 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 1-(2-fluorophenyl)cyclobutan-1-amine hydrochloride (68.2 mg, 338 µmol).
¹H NMR (400 MHz, DMSO-*d*₆) δ ppm 1.75 (dt, 1 H) 1.99 - 2.11 (m, 1 H) 2.81 (t, 2 H) 3.89 - 4.02 (m, 4 H) 4.10 (t, 2 H) 6.63 (s, 1 H) 6.99 (s, 2 H) 7.04 - 7.14 (m, 3 H) 7.18 - 7.31 (m, 1 H) 7.39 - 7.47 (m, 1 H) 8.14 (d, 1 H) 8.63 (d, 1 H)
LC-MS (method 1): Rt = 1.06 min; MS (ESlneg): m/z = 456 [M-H]⁻

### Example 193

### 2'-(6-amino-5-cyanopyridin-3-yl)-N-[2-(4-fluorophenyl)propan-2-yl]-5',6'-dihydrospiro[azetidine-3,4'-pyrrolo[1,2-b]pyrazole]-1-carboxamide

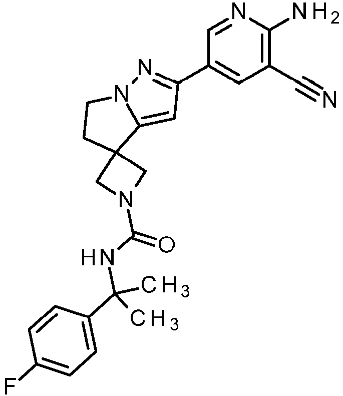

Example 193 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-(4-fluorophenyl)propan-2-amine (51.8 mg, 338 µmol).
¹H NMR (400 MHz, DMSO-*d*₆) δ ppm 1.49 - 1.59 (m, 6 H) 2.80 - 2.89 (m, 2 H) 3.94 - 4.05 (m, 4 H) 4.13 (t, 2 H) 6.58 (s, 1 H) 6.67 (s, 1 H) 6.98 - 7.13 (m, 4 H) 7.28 - 7.40 (m, 2 H) 8.17 (d, 1 H) 8.65 (d, 1 H)
LC-MS (method 1): Rt = 1.03 min; MS (ESlneg): m/z = 444 [M-H]⁻

### Example 194

### 2'-(6-amino-5-cyanopyridin-3-yl)-N-[1-(2-chlorophenyl)cyclobutyl]-5',6'-dihydrospiro[azetidine-3,4'-pyrrolo[1,2-b]pyrazole]-1-carboxamide

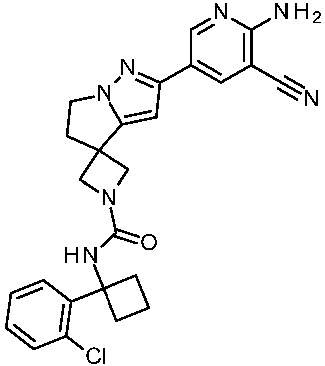

Example 194 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 1-(2-chlorophenyl)cyclobutan-1-amine hydrochloride (73.7 mg, 338 µmol).
¹H NMR (400 MHz, DMSO-*d*₆) δ ppm 1.64 - 1.75 (m, 1 H) 2.00 - 2.12 (m, 1 H) 2.52 - 2.56 (m, 1 H) 2.57 - 2.68 (m, 2 H) 2.79 (t, 2 H) 3.90 - 4.00 (m, 4 H) 4.10 (t, 2 H) 6.60 (s, 1 H) 6.99 (s, 2 H) 7.03 (s, 1 H) 7.19 - 7.29 (m, 2 H) 7.33 (dd, 1 H) 7.54 (dd, 1 H) 8.14 (d, 1 H) 8.62 (d, 1 H)
LC-MS (method 1): Rt = 1.12 min; MS (ESlneg): m/z = 472 [M-H]⁻

### Example 195

### 2'-(6-amino-5-cyanopyridin-3-yl)-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide

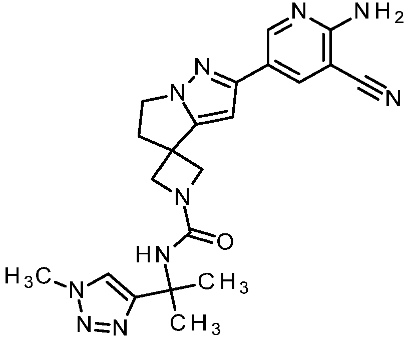

Example 195 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-(1-methyl-1H-1,2,3-triazol-4-yl)propan-2-amine hydrochloride (72.0 mg, 338 µmol).
¹H NMR (400 MHz, DMSO-*d*₆) δ ppm 1.59 (s, 6 H) 2.82 (t, 2 H) 3.95 - 4.03 (m, 7 H) 4.12 (t, 2 H) 6.42 (s, 1 H) 6.67 (s, 1 H) 7.00 (s, 2 H) 7.80 (s, 1 H) 8.16 (d, 1 H) 8.64 (d, 1 H)
LC-MS (method 1): Rt = 0.73 min; MS (ESlpos): m/z = 433 [M+H]⁺

### Example 196

### 2'-(6-amino-5-cyanopyridin-3-yl)-N-[2-(pyridin-2-yl)propan-2-yl]-5',6'-dihydrospiro[azetidine-3,4'-pyrrolo[1,2-b]pyrazole]-1-carboxamide

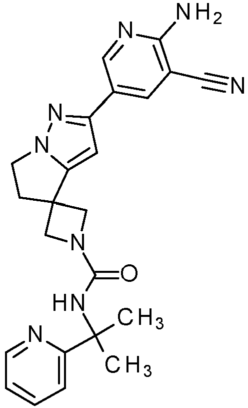

Example 196 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-(pyridin-2-yl)propan-2-amine (46.0 mg, 338 µmol).
¹H NMR (400 MHz, DMSO-*d*₆) δ ppm 1.57 (s, 6 H) 2.86 (t, 2 H) 3.99 - 4.08 (m, 4 H) 4.13 (t, 2 H) 6.70 (s, 1 H) 6.73 (s, 1 H) 7.00 (s, 2 H) 7.20 (ddd, 1 H) 7.45 (d, 1 H) 7.74 (td, 1 H) 8.18 (d, 1 H) 8.47 (d, 1 H) 8.65 (d, 1 H)
LC-MS (method 1): Rt = 0.86 min; MS (ESlpos): m/z = 429 [M+H]⁺

### Example 197

### 2'-(6-amino-5-cyanopyridin-3-yl)-N-[2-(pyridin-4-yl)propan-2-yl]-5',6'-dihydrospiro[azetidine-3,4'-pyrrolo[1,2-b]pyrazole]-1-carboxamide

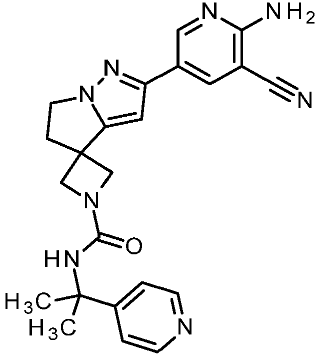

Example 197 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-(pyridin-4-yl)propan-2-amine (46.0 mg, 338 µmol).
¹H NMR (400 MHz, DMSO-*d*₆) δ ppm 1.53 (s, 6 H) 2.80 - 2.89 (m, 2 H) 3.98 - 4.07 (m, 4 H) 4.13 (t, 2 H) 6.68 (s, 1 H) 6.73 (s, 1 H) 7.00 (s, 2 H) 7.29 - 7.34 (m, 2 H) 8.18 (d, 1 H) 8.44 - 8.52 (m, 2 H) 8.65 (d, 1 H)
LC-MS (method 1): Rt = 0.81 min; MS (ESlpos): m/z = 429 [M+H]⁺

### Example 198

### 2'-(6-amino-5-cyanopyridin-3-yl)-N-[(1R)-1-phenylpropyl]-5',6'-dihydrospiro[azetidine-3,4'-pyrrolo[1,2-b]pyrazole]-1-carboxamide

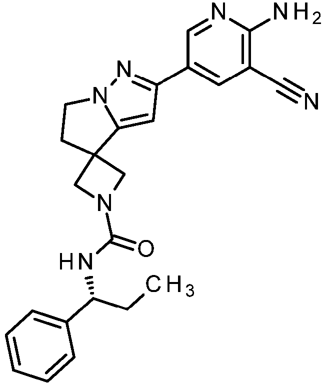

Example 198 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and (1R)-1-phenylpropan-1-amine (45.7 mg, 338 µmol).
¹H NMR (400 MHz, DMSO-*d*₆) δ ppm 0.86 (t, 3 H) 1.58 - 1.75 (m, 2 H) 2.84 (t, 2 H) 3.96 - 4.06 (m, 4 H) 4.12 (t, 2 H) 4.48 - 4.58 (m, 1 H) 6.67 (s, 1 H) 6.83 (d, 1 H) 6.99 (s, 2 H) 7.17 - 7.25 (m, 1 H) 7.28 - 7.35 (m, 4 H) 8.15 (d, 1 H) 8.63 (d, 1 H)
LC-MS (method 1): Rt = 1.02 min; MS (ESlpos): m/z = 428 [M+H]⁺

### Example 199

### 2'-(6-amino-5-cyanopyridin-3-yl)-N-[(1R)-1-(3-fluorophenyl)ethyl]-5',6'-dihydrospiro[azetidine-3,4'-pyrrolo[1,2-b]pyrazole]-1-carboxamide

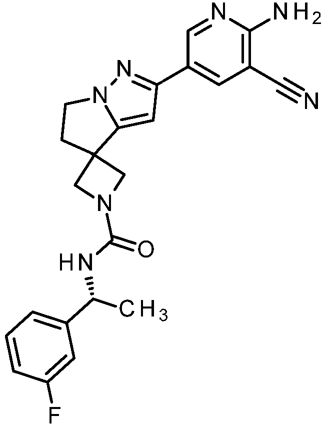

Example 199 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and (1R)-1-(3-fluorophenyl)ethan-1-amine (47.0 mg, 338 µmol).
¹H NMR (400 MHz, DMSO-*d*₆) δ ppm 1.36 (d, 3 H) 2.85 (t, 2 H) 3.98 - 4.08 (m, 4 H) 4.12 (t, 2 H) 4.78 - 4.86 (m, 1 H) 6.68 (s, 1 H) 6.93 (d, 1 H) 7.00 (s, 2 H) 7.01 - 7.07 (m, 1 H) 7.13 - 7.20 (m, 2 H) 7.31 - 7.39 (m, 1 H) 8.15 (d, 1 H) 8.64 (d, 1 H)
LC-MS (method 1): Rt = 0.98 min; MS (ESlpos): m/z = 432 [M+H]⁺

### Example 200

### 2'-(6-amino-5-cyanopyridin-3-yl)-N-[(1R)-1-(2-cyanophenyl)ethyl]-5',6'-dihydrospiro[azetidine-3,4'-pyrrolo[1,2-b]pyrazole]-1-carboxamide

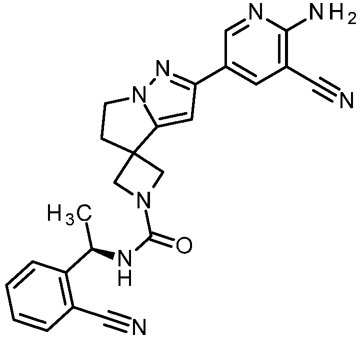

Example 200 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 2-[(1R)-1-aminoethyl]benzonitrile (49.4 mg, 338 µmol).
¹H NMR (400 MHz, DMSO-*d*₆) δ ppm 1.39 (d, 3 H) 2.81 - 2.89 (m, 2 H) 3.99 - 4.10 (m, 4 H) 4.13 (t, 2 H) 5.03 (quin, 1 H) 6.67 (s, 1 H) 7.00 (s, 2 H) 7.19 (d, 1 H) 7.42 (td, 1 H) 7.62 (d, 1 H) 7.69 - 7.79 (m, 2 H) 8.15 (d, 1 H) 8.64 (d, 1 H)
LC-MS (method 1): Rt = 0.91 min; MS (ESlpos): m/z = 439 [M+H]⁺

### Example 201

### (rac)-2'-(6-amino-5-cyanopyridin-3-yl)-N-[2-(pyridin-4-yl)propan-2-yl]-5',6'-dihydrospiro[pyrrolidine-3,4'-pyrrolo[1,2-b]pyrazole]-1-carboxamide

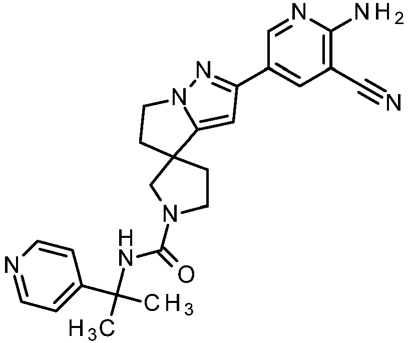

Example 201 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (125 mg, 395 µmol) and 2-(pyridin-4-yl)propan-2-amine (64.5 mg, 473 µmol).
¹H NMR (400 MHz, DMSO-*d*₆) δ ppm 1.55 (s, 6 H) 1.98 - 2.15 (m, 2 H) 2.52 - 2.59 (m, 2 H) 3.45 (br s, 2 H) 3.49 - 3.60 (m, 2 H) 4.19 (t, 2 H) 6.33 (s, 1 H) 6.46 (s, 1 H) 7.01 (s, 2 H) 7.49 (d, 2 H) 8.15 (d, 1 H) 8.52 (d, 2 H) 8.62 (d, 1 H)
LC-MS (method 1): Rt = 0.83 min; MS (ESlpos): m/z = 444 [M+H]⁺

### Example 202

2'-(6-amino-5-cyanopyridin-3-yl)-N-[(1R)-1-(5-fluoropyridin-3-yl)ethyl]-5',6'-dihydrospiro[pyrrolidine-3,4'-pyrrolo[1,2-b]pyrazole]-1-carboxamide (mixture of stereoisomers)

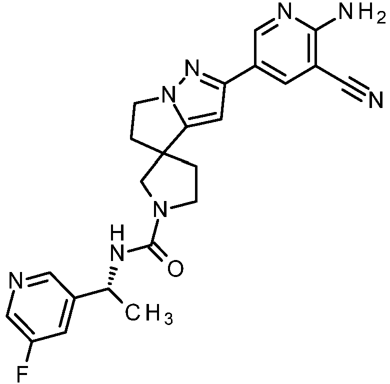

Example 202 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (125 mg, 395 µmol) and (1R)-1-(5-fluoropyridin-3-yl)ethan-1-amine (66.4 mg, 473 µmol).
¹H NMR (400 MHz, DMSO-*d*₆) δ ppm 1.41 (dd, 3 H) 2.01 - 2.12 (m, 2 H) 2.52 - 2.57 (m, 2 H) 3.46 (br dd, 3 H) 3.50 - 3.63 (m, 1 H) 4.15 - 4.22 (m, 2 H) 4.92 (td, 1 H) 6.40 - 6.45 (m, 1 H) 6.61 (dd, 1 H) 6.99 (s, 2 H) 7.64 - 7.72 (m, 1 H) 8.14 (dd, 1 H) 8.40 - 8.47 (m, 2 H) 8.61 (d, 1 H)
LC-MS (method 1): Rt = 0.83 min; MS (ESlpos): m/z = 448 [M+H]⁺

### Example 203

### (rac)-2'-(6-amino-5-cyanopyridin-3-yl)-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide

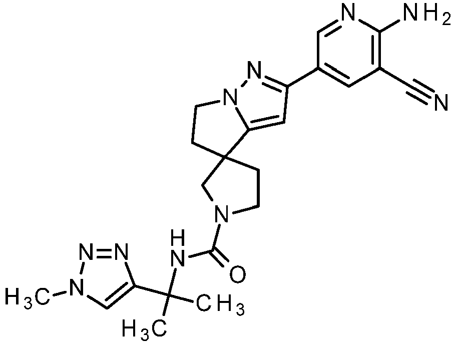

Example 203 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (125 mg, 395 µmol) and 2-(1-methyl-1H-1,2,3-triazol-4-yl)propan-2-amine hydrochloride (101 mg, 473 µmol).
¹H NMR (400 MHz, DMSO-*d*₆) δ ppm 1.60 (d, 6 H) 2.00 - 2.10 (m, 2 H) 2.52 - 2.56 (m, 2 H) 3.44 (br d, 3 H) 3.51 (br s, 1 H) 3.98 (s, 3 H) 4.18 (t, 2 H) 5.93 (s, 1 H) 6.46 (s, 1 H) 6.99 (s, 2 H) 7.80 (s, 1 H) 8.14 - 8.19 (m, 1 H) 8.62 (d, 1 H)
LC-MS (method 1): Rt = 0.74 min; MS (ESlpos): m/z = 448 [M+H]⁺

### Example 204

### (rac)-2-(6-amino-5-cyanopyridin-3-yl)-N-[1-(pyridin-2-yl)cyclobutyl]-6,7-dihydrospiro[pyrazolo[5,1-c][1,4]oxazine-4,3'-pyrrolidine]-1'-carboxamide

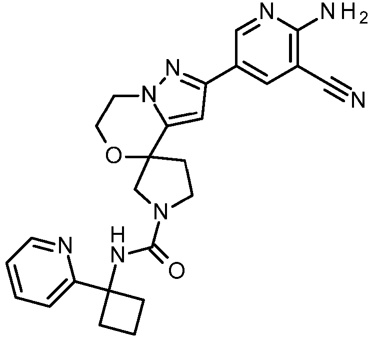

Example 204 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and 1-(pyridin-2-yl)cyclobutan-1-amine (37.9 mg, 256 µmol).
¹H NMR (400 MHz, DMSO-*d*₆) δ ppm 1.83 - 2.08 (m, 2 H) 2.18 - 2.31 (m, 1 H) 2.32 - 2.43 (m, 3 H) 2.53 - 2.65 (m, 2 H) 3.35 - 3.53 (m, 2 H) 3.64 (br t, 1 H) 3.83 (br d, 1 H) 4.06 - 4.18 (m, 4 H) 6.73 (s, 1 H) 6.82 (s, 1 H) 7.05 (s, 2 H) 7.19 (ddd, 1 H) 7.36 (d, 1 H) 7.66 - 7.72 (m, 1 H) 8.12 (d, 1 H) 8.53 (d, 1 H) 8.61 (d, 1 H)
LC-MS (method 1): Rt = 0.90 min; MS (ESlpos): m/z = 471 [M+H]⁺

### Example 205

### (rac)-2'-(6-amino-5-cyanopyridin-3-yl)-N-[1-(pyridin-2-yl)cyclobutyl]-5',6'-dihydrospiro[pyrrolidine-3,4'-pyrrolo[1,2-b]pyrazole]-1-carboxamide

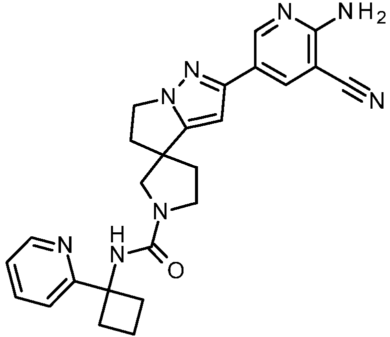

Example 205 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) and 1-(pyridin-2-yl)cyclobutan-1-amine (43.2 mg, 292 µmol).
¹H NMR (400 MHz, DMSO-*d*₆) δ ppm 1.83 - 2.04 (m, 2 H) 2.05 - 2.14 (m, 2 H) 2.53 - 2.63 (m, 4 H) 3.43 - 3.51 (m, 3 H) 3.52 - 3.64 (m, 1 H) 4.20 (t, 3 H) 6.46 (s, 1 H) 6.79 (s, 1 H) 7.00 (s, 3 H) 7.18 (ddd, 1 H) 7.37 (d, 1 H) 7.68 (td, 1 H) 8.14 (d, 1 H) 8.53 (d, 1 H) 8.62 (d, 1 H)
LC-MS (method 1): Rt = 0.90 min; MS (ESlpos): m/z = 455 [M+H]⁺

### Example 206

### (rac)-2'-(6-amino-5-cyanopyridin-3-yl)-N-[1-(2-chlorophenyl)cyclobutyl]-5',6'-dihydrospiro[pyrrolidine-3,4'-pyrrolo[1,2-b]pyrazole]-1-carboxamide

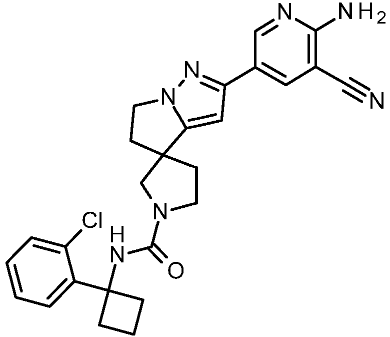

Example 206 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) and 1-(2-chlorophenyl)cyclobutan-1-amine hydrochloride (63.6 mg, 292 µmol).
¹H NMR (400 MHz, DMSO-*d*₆) δ ppm 1.63 - 1.73 (m, 1 H) 1.98 - 2.14 (m, 3 H) 2.38 - 2.47 (m, 1 H) 2.52 - 2.70 (m, 4 H) 3.38 (br d, 3 H) 3.43 - 3.51 (m, 1 H) 4.16 (t, 2 H) 6.31 (s, 1 H) 6.52 (s, 1 H) 7.00 (s, 2 H) 7.17 - 7.32 (m, 4 H) 7.54 (dd, 1 H) 8.08 (d, 1 H) 8.57 (d, 1 H)
LC-MS (method 1): Rt = 1.14 min; MS (ESlpos): m/z = 488 [M+H]⁺

### Example 207

### 2-(6-amino-5-cyanopyridin-3-yl)-N-[(1R)-1-phenylethyl]-6,7-dihydrospiro[pyrazolo[5,1-c][1,4]oxazine-4,3'-pyrrolidine]-1'-carboxamide (mixture of stereoisomers)

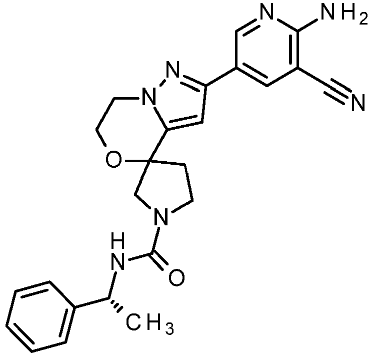

Example 207 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and (1R)-1-phenylethan-1-amine (31.0 mg, 256 µmol).
¹H NMR (400 MHz, DMSO-*d*₆) δ ppm 1.37 (d, 3 H) 2.15 - 2.25 (m, 1 H) 2.31 - 2.38 (m, 1 H) 3.35 - 3.49 (m, 2 H) 3.56 - 3.65 (m, 1 H) 3.81 (dd, 1 H) 4.04 - 4.16 (m, 4 H) 4.84 (quin, 1 H) 6.50 (dd, 1 H) 6.70 (s, 1 H) 7.05 (s, 2 H) 7.17 - 7.22 (m, 1 H) 7.27 - 7.35 (m, 4 H) 8.12 (t, 1 H) 8.60 (t, 1 H)
LC-MS (method 1): Rt = 0.98 min; MS (ESlpos): m/z = 444 [M+H]⁺

### Example 208

### 2'-(6-amino-5-cyanopyridin-3-yl)-N-[(1R)-1-phenylethyl]-5',6'-dihydrospiro[pyrrolidine-3,4'-pyrrolo[1,2-b]pyrazole]-1-carboxamide (mixture of stereoisomers)

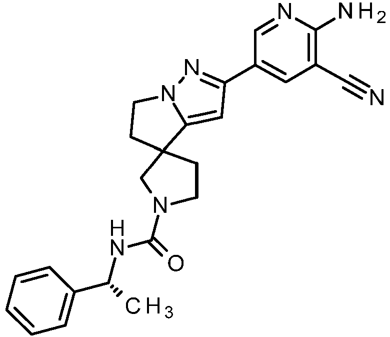

Example 208 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) and (1R)-1-phenylethan-1-amine (35.3 mg, 292 µmol).
¹H NMR (400 MHz, DMSO-*d*₆) δ ppm 1.37 (dd, 3 H) 2.01 - 2.10 (m, 2 H) 2.52 - 2.58 (m, 2 H) 3.41 - 3.49 (m, 3 H) 3.50 - 3.59 (m, 1 H) 4.18 (t, 2 H) 4.80 - 4.88 (m, 1 H) 6.40 - 6.44 (m, 1 H) 6.47 (dd, 1 H) 6.99 (s, 2 H) 7.15 - 7.22 (m, 1 H) 7.24 - 7.37 (m, 4 H) 8.13 (dd, 1 H) 8.61 (t, 1 H)
LC-MS (method 1): Rt = 0.97 min; MS (ESlpos): m/z = 429 [M+H]⁺

### Example 209

### (rac)-2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide

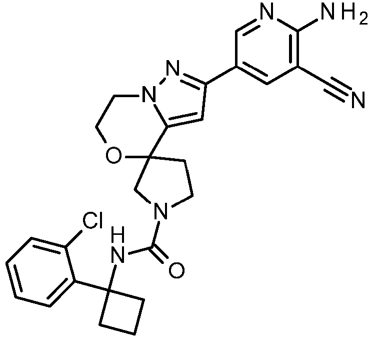

Example 209 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and 1-(2-chlorophenyl)cyclobutan-1-amine hydrochloride (55.8 mg, 256 µmol).
¹H NMR (400 MHz, DMSO-*d*₆) δ ppm 1.64 - 1.74 (m, 1 H) 2.01 - 2.22 (m, 2 H) 2.26 - 2.35 (m, 1 H) 2.52 - 2.57 (m, 1 H) 2.60 - 2.68 (m, 2 H) 3.36 - 3.44 (m, 1 H) 3.53 (br t, 1 H) 3.73 (br d, 1 H) 4.01 - 4.15 (m, 4 H) 6.54 (s, 1 H) 6.66 (s, 1 H) 7.04 (s, 2 H) 7.18 - 7.32 (m, 3 H) 7.53 (dd, 1 H) 8.09 (d, 1 H) 8.58 (d, 1 H)
LC-MS (method 1): Rt = 1.14 min; MS (ESlpos): m/z = 505 [M+H]⁺

### Example 210

### (rac)-2-(6-amino-5-cyanopyridin-3-yl)-N-[1-(4-fluorophenyl)cyclobutyl]-6,7-dihydrospiro[pyrazolo[5,1-c][1,4]oxazine-4,3'-pyrrolidine]-1'-carboxamide

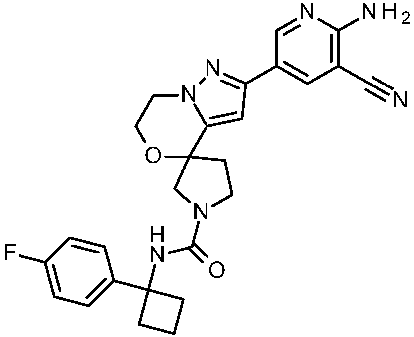

Example 210 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and 1-(4-fluorophenyl)cyclobutan-1-amine hydrochloride (51.6 mg, 256 µmol).
¹H NMR (400 MHz, DMSO-*d*₆) δ ppm 1.70 - 1.81 (m, 1 H) 1.94 - 2.08 (m, 1 H) 2.13 - 2.25 (m, 1 H) 2.31 - 2.46 (m, 5 H) 3.35 - 3.47 (m, 2 H) 3.57 (br t, 1 H) 3.76 (br d, 1 H) 4.04 - 4.16 (m, 4 H) 6.67 - 6.75 (m, 2 H) 7.01 - 7.14 (m, 4 H) 7.41 - 7.47 (m, 2 H) 8.11 (d, 1 H) 8.59 (d, 1 H)
LC-MS (method 1): Rt = 1.07 min; MS (ESlpos): m/z = 489 [M+H]⁺

### Example 211

### (rac)-2-(6-amino-5-cyanopyridin-3-yl)-N-[2-(4-fluorophenyl)propan-2-yl]-6,7-dihydrospiro[pyrazolo[5,1-c][1,4]oxazine-4,3'-pyrrolidine]-1'-carboxamide

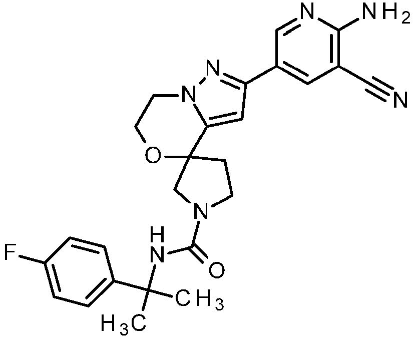

Example 211 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and 2-(4-fluorophenyl)propan-2-amine hydrochloride (48.6 mg, 256 µmol).
¹H NMR (400 MHz, DMSO-*d*₆) δ ppm 1.55 (d, 6 H) 2.15 - 2.25 (m, 1 H) 2.30 - 2.37 (m, 1 H) 3.34 - 3.50 (m, 2 H) 3.60 (br t, 1 H) 3.79 (br d, 1 H) 4.05 - 4.17 (m, 4 H) 6.10 (s, 1 H) 6.71 (s, 1 H) 7.01 - 7.11 (m, 4 H) 7.35 - 7.37 (m, 1 H) 7.38 (s, 1 H) 8.12 (d, 1 H) 8.60 (d, 1 H)
LC-MS (method 1): Rt = 1.05 min; MS (ESlpos): m/z = 477 [M+H]⁺

### Example 212

### (rac)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide

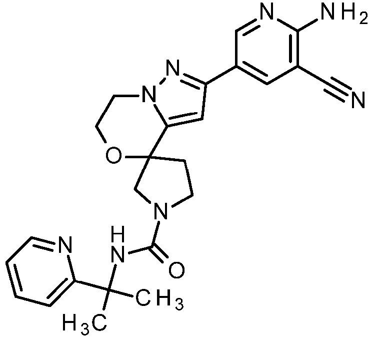

Example 212 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and 2-(pyridin-2-yl)propan-2-amine (34.9 mg, 256 µmol).
¹H NMR (400 MHz, DMSO-*d*₆) δ ppm 1.58 (d, 6 H) 2.18 - 2.27 (m, 1 H) 2.52 - 2.54 (m, 1 H) 3.36 - 3.52 (m, 2 H) 3.63 (br t, 1 H) 3.81 (d, 1 H) 4.08 - 4.18 (m, 4 H) 6.43 (s, 1 H) 6.73 (s, 1 H) 7.05 (s, 2 H) 7.14 - 7.21 (m, 1 H) 7.44 (d, 1 H) 7.71 (t, 1 H) 8.13 (d, 1 H) 8.46 (d, 1 H) 8.61 (d, 1 H)
LC-MS (method 1): Rt = 0.90 min; MS (ESlpos): m/z = 459 [M+H]⁺

### Example 213

### (rac)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide

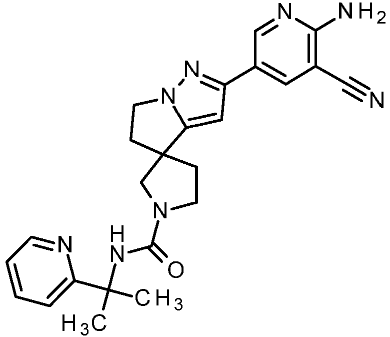

Example 213 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) and 2-(pyridin-2-yl)propan-2-amine (39.7 mg, 292 µmol).
¹H NMR (400 MHz, DMSO-*d*₆) δ ppm 1.59 (s, 6 H) 2.04 - 2.14 (m, 2 H) 2.56 (t, 2 H) 3.42 - 3.50 (m, 3 H) 3.52 - 3.62 (m, 1 H) 4.20 (t, 2 H) 6.43 (s, 1 H) 6.47 (s, 1 H) 7.00 (s, 2 H) 7.19 (ddd, 1 H) 7.47 (d, 1 H) 7.71 (td, 1 H) 8.15 (d, 1 H) 8.46 (d, 1 H) 8.62 (d, 1 H)
LC-MS (method 1): Rt = 0.91 min; MS (ESlpos): m/z = 443 [M+H]⁺

### Example 214

### 2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (enantiomer 2)

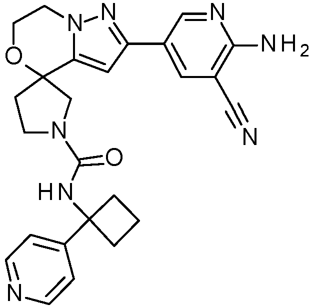

Example 214 was prepared in analogy to Example 31 using 2-amino-5-[6,7-dihydrospiro[pyrazolo[5,1-c][1,4]oxazine-4,3'-pyrrolidin]-2-yl]pyridine-3-carbonitrile hydrochloride (75.0 mg, 225 µmol) and 1-(pyridin-4-yl)cyclobutan-1-amine (40.1 mg, 270 µmol).
¹H NMR (400 MHz, DMSO-*d*₆) δ ppm 1.77 - 1.88 (m, 1 H) 1.96 - 2.08 (m, 1 H) 2.15 - 2.28 (m, 1 H) 2.32 - 2.47 (m, 5 H) 3.37 - 3.51 (m, 2 H) 3.60 (br t, 1 H) 3.78 (br d, 1 H) 4.04 - 4.20 (m, 4 H) 6.71 (s, 1 H) 6.89 (s, 1 H) 7.05 (s, 2 H) 7.39 (d, 2 H) 8.12 (d, 1 H) 8.48 (d, 2 H) 8.60 (d, 1 H)
[α]²⁰_{D} : +69.4° (c = 1.00, DMSO)
LC-MS (method 1): Rt = 0.84 min; MS (ESlneg): m/z = 469 [M-H]⁻

### Example 215

### 2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide (enantiomer 2)

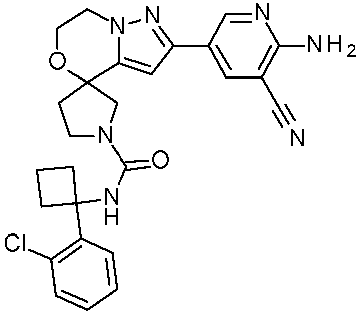

Example 215 was prepared in analogy to Example 31 using 2-amino-5-[6,7-dihydrospiro[pyrazolo[5,1-c][1,4]oxazine-4,3'-pyrrolidin]-2-yl]pyridine-3-carbonitrile hydrochloride (75.0 mg, 225 µmol) and 1-(2-chlorophenyl)cyclobutan-1-amine hydrochloride (54.1 mg, 248 µmol).
¹H NMR (400 MHz, DMSO-*d*₆) δ ppm 1.19 - 1.28 (m, 1 H) 1.64 - 1.74 (m, 1 H) 2.01 - 2.13 (m, 1 H) 2.13 - 2.23 (m, 1 H) 2.26 - 2.36 (m, 1 H) 2.52 - 2.60 (m, 2 H) 2.60 - 2.68 (m, 2 H) 3.36 - 3.45 (m, 1 H) 3.47 - 3.63 (m, 1 H) 3.73 (br d, 1 H) 4.01 - 4.15 (m, 4 H) 6.54 (s, 1 H) 6.66 (s, 1 H) 7.04 (s, 2 H) 7.17 - 7.33 (m, 3 H) 7.53 (dd, 1 H) 8.09 (d, 1 H) 8.58 (d, 1 H)
[α]²⁰_{D} : +43.5° (c = 1.00, DMSO)
LC-MS (method 1): Rt = 1.13 min; MS (ESlpos): m/z = 504 [M+H]⁺

### Example 216

### 2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide (enantiomer 1)

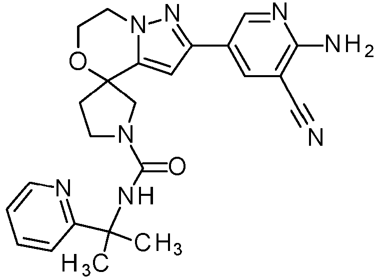

Example 216 was prepared in analogy to Example 31 using 2-amino-5-[6,7-dihydrospiro[pyrazolo[5,1-c][1,4]oxazine-4,3'-pyrrolidin]-2-yl]pyridine-3-carbonitrile hydrochloride (75.0 mg, 225 µmol) and 2-(pyridin-2-yl)propan-2-amine (36.8 mg, 270 µmol).
¹H NMR (400 MHz, DMSO-*d*₆) δ ppm 1.05 (t, 1 H) 1.58 (d, 5 H) 2.15 - 2.30 (m, 1 H) 2.31 - 2.41 (m, 1 H) 3.42 - 3.54 (m, 2 H) 3.62 (br t, 1 H) 3.81 (br d, 1 H) 4.05 - 4.18 (m, 4 H) 6.44 (s, 1 H) 6.72 (s, 1 H) 7.04 (s, 2 H) 7.19 (ddd, 1 H) 7.45 (d, 1 H) 7.72 (td, 1 H) 8.13 (d, 1 H) 8.46 (d, 1 H) 8.61 (d, 1 H)
[α]²⁰_{D} : -60.4° (c = 1.00, DMSO)
LC-MS (method 1): Rt = 0.88 min; MS (ESlneg): m/z = 457 [M-H]⁻

### Example 217

### 2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide (enantiomer 1)

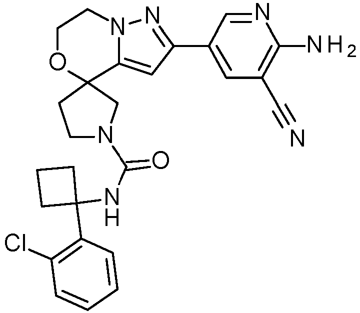

Example 217 was prepared in analogy to Example 31 using 2-amino-5-[6,7-dihydrospiro[pyrazolo[5,1-c][1,4]oxazine-4,3'-pyrrolidin]-2-yl]pyridine-3-carbonitrile hydrochloride (75.0 mg, 225 µmol) and 1-(2-chlorophenyl)cyclobutan-1-amine hydrochloride (54.1 mg, 248 µmol).
¹H NMR (400 MHz, DMSO-*d*₆) δ ppm 1.64 - 1.74 (m, 1 H) 2.01 - 2.23 (m, 2 H) 2.25 - 2.35 (m, 1 H) 2.52 - 2.60 (m, 1 H) 2.61 - 2.68 (m, 2 H) 3.36 - 3.45 (m, 1 H) 3.53 (br t, 1 H) 3.74 (br d, 1 H) 4.01 - 4.17 (m, 4 H) 6.54 (s, 1 H) 6.66 (s, 1 H) 7.04 (s, 2 H) 7.17 - 7.34 (m, 3 H) 7.53 (dd, 1 H) 8.09 (d, 1 H) 8.58 (d, 1 H)
[α]²⁰_{D} : -69.8° (c = 1.00, DMSO)
LC-MS (method 1): Rt = 1.13 min; MS (ESlpos): m/z = 504 [M+H]⁺

### Example 218

### 2'-(6-amino-5-cyanopyridin-3-yl)-N-[1-(pyridin-3-yl)cyclobutyl]-5',6'-dihydrospiro[azetidine-3,4'-pyrrolo[1,2-b]pyrazole]-1-carboxamide

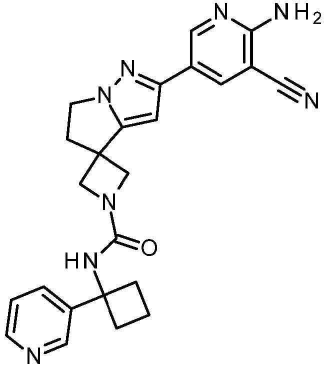

Example 218 was prepared in analogy to Example 31 using 2-amino-5-(5',6'-dihydrospiro[azetidine-3,4'-pyrrolo[1,2-b]pyrazol]-2'-yl)pyridine-3-carbonitrile hydrochloride (75.0 mg, 248 µmol) and 1-(pyridin-3-yl)cyclobutan-1-amine (44.1 mg, 297 µmol).
¹H NMR (500 MHz, DMSO-*d*₆) δ ppm 1.05 (t, 1 H) 1.76 - 1.86 (m, 1 H) 1.96 - 2.11 (m, 1 H) 2.41 - 2.48 (m, 3 H) 2.83 (t, 2 H) 3.95 - 4.05 (m, 4 H) 4.12 (t, 2 H) 6.67 (s, 1 H) 7.00 (s, 2 H) 7.21 (s, 1 H) 7.33 - 7.36 (m, 1 H) 7.79 (dt, 1 H) 8.16 (d, 1 H) 8.41 (dd, 1 H) 8.64 (d, 2 H)
LC-MS (method 1): Rt = 0.84 min; MS (ESlneg): m/z = 439 [M-H]⁻

### Example 219

### 2'-(6-amino-5-cyanopyridin-3-yl)-N-[1-(pyridin-2-yl)cyclobutyl]-5',6'-dihydrospiro[azetidine-3,4'-pyrrolo[1,2-b]pyrazole]-1-carboxamide

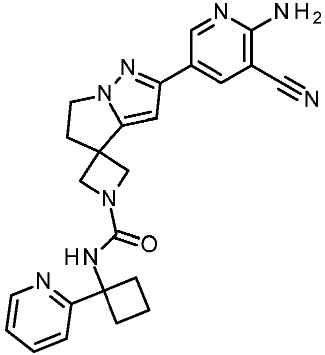

Example 219 was prepared in analogy to Example 31 using 2-amino-5-(5',6'-dihydrospiro[azetidine-3,4'-pyrrolo[1,2-b]pyrazol]-2'-yl)pyridine-3-carbonitrile hydrochloride (75.0 mg, 248 µmol) and 1-(pyridin-2-yl)cyclobutan-1-amine (44.1 mg, 297 µmol).
¹H NMR (500 MHz, DMSO-*d*₆) δ ppm 1.83 - 2.06 (m, 2 H) 2.34 - 2.41 (m, 2 H) 2.83 - 2.90 (m, 2 H) 3.99 - 4.10 (m, 5 H) 4.14 (t, 3 H) 6.71 (s, 1 H) 7.00 (s, 2 H) 7.17 - 7.23 (m, 2 H) 7.38 (d, 1 H) 7.73 (td, 1 H) 8.18 (d, 1 H) 8.55 (d, 1 H) 8.65 (d, 1 H)
LC-MS (method 1): Rt = 0.85 min; MS (ESlpos): m/z = 441 [M+H]⁺

### Example 220

### 2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (enantiomer 1)

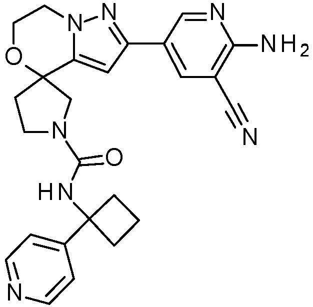

Example 220 was prepared in analogy to Example 31 using 2-amino-5-[6,7-dihydrospiro[pyrazolo[5,1-c][1,4]oxazine-4,3'-pyrrolidin]-2-yl]pyridine-3-carbonitrile hydrochloride (75.0 mg, 225 µmol) and 1-(pyridin-4-yl)cyclobutan-1-amine (40.1 mg, 270 µmol).
¹H NMR (400 MHz, DMSO-*d*₆) δ ppm 1.77 - 1.91 (m, 1 H) 1.97 - 2.10 (m, 1 H) 2.16 - 2.29 (m, 1 H) 2.30 - 2.47 (m, 5 H) 3.36 - 3.46 (m, 2 H) 3.58 - 3.64 (m, 1 H) 3.76 - 3.81 (m, 1 H) 4.04 - 4.20 (m, 4 H) 6.69 - 6.74 (m, 1 H) 6.96 (s, 1 H) 7.01 - 7.09 (m, 2 H) 7.49 - 7.54 (m, 2 H) 8.12 (d, 1 H) 8.52 - 8.57 (m, 2 H) 8.60 (d, 1 H)
[α]²⁰_{D} : -155.6° (c = 1.00, DMSO)
LC-MS (method 1): Rt = 0.83 min; MS (ESlneg): m/z = 469 [M-H]⁻

### Example 221

### 2'-(6-amino-5-cyanopyridin-3-yl)-N-(1-phenylcyclobutyl)-5',6'-dihydrospiro[azetidine-3,4'-pyrrolo[1,2-b]pyrazole]-1-carboxamide

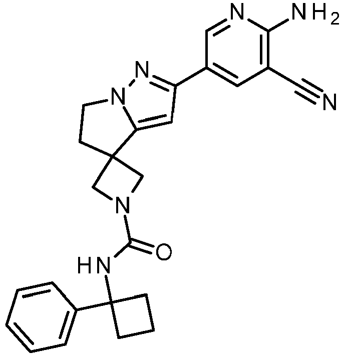

Example 221 was prepared in analogy to Example 31 using 2-amino-5-(5',6'-dihydrospiro[azetidine-3,4'-pyrrolo[1,2-b]pyrazol]-2'-yl)pyridine-3-carbonitrile hydrochloride (75.0 mg, 248 µmol) and 1-phenylcyclobutan-1-amine hydrochloride (54.6 mg, 297 µmol).
¹H NMR (400 MHz, DMSO-*d*₆) δ ppm 1.73 - 1.84 (m, 1 H) 1.95 - 2.10 (m, 1 H) 2.34 - 2.48 (m, 4 H) 2.83 (t, 2 H) 3.93 - 4.04 (m, 4 H) 4.12 (t, 2 H) 6.66 (s, 1 H) 7.00 (s, 2 H) 7.08 (s, 1 H) 7.14 - 7.22 (m, 1 H) 7.32 (t, 2 H) 7.40 - 7.45 (m, 2 H) 8.16 (d, 1 H) 8.64 (d, 1 H)
LC-MS (method 1): Rt = 1.01 min; MS (ESlneg): m/z = 438 [M-H]⁻

### Example 222

### 2'-(6-amino-5-cyanopyridin-3-yl)-N-[(3S)-2,3-dihydro-1-benzofuran-3-yl]-5',6'-dihydrospiro[pyrrolidine-3,4'-pyrrolo[1,2-b]pyrazole]-1-carboxamide (mixture of stereoisomers)

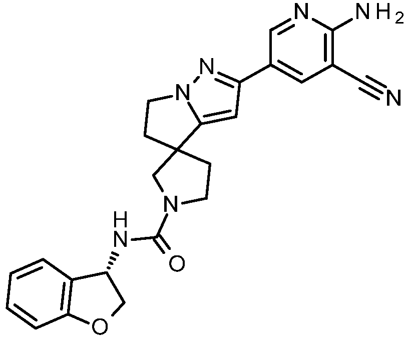

Example 222 was prepared in analogy to Example 31 using (*rac*)-2-amino-5-[5',6'-dihydrospiro[pyrrolidine-3,4'-pyrrolo[1,2-b]pyrazol]-2'-yl]pyridine-3-carbonitrile hydrochloride (100 mg, 316 µmol) and (3S)-2,3-dihydro-1-benzofuran-3-amine (51.2 mg, 379 µmol).
¹H NMR (400 MHz, DMSO-*d*₆) δ ppm 2.05 (t, 2 H) 2.52 - 2.56 (m, 2 H) 3.35 - 3.57 (m, 4 H) 4.17 (t, 2 H) 4.24 (dt, 1 H) 4.66 (td, 1 H) 5.43 - 5.50 (m, 1 H) 6.44 (d, 1 H) 6.73 - 6.90 (m, 3 H) 6.99 (s, 2 H) 7.18 (tdd, 1 H) 7.33 (dd, 1 H) 8.14 (dd, 1 H) 8.61 (d, 1 H)
LC-MS (method 1): Rt = 0.92 min; MS (ESlneg): m/z = 440 [M-H]⁻

### Example 223

### 2-(6-amino-5-cyanopyridin-3-yl)-N-[(3S)-2,3-dihydro-1-benzofuran-3-yl]-6,7-dihydrospiro[pyrazolo[5,1-c][1,4]oxazine-4,3'-pyrrolidine]-1'-carboxamide (mixture of stereoisomers)

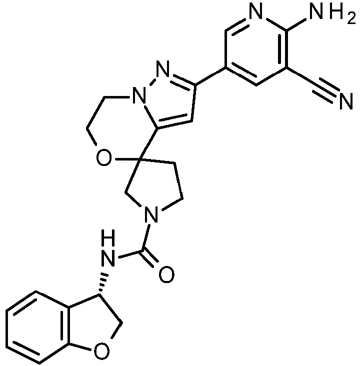

Example 223 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (95.0 mg, 285 µmol) and (3S)-2,3-dihydro-1-benzofuran-3-amine (46.3 mg, 343 µmol).
¹H NMR (400 MHz, DMSO-*d*₆) δ ppm 2.14 - 2.26 (m, 1 H) 2.29 - 2.40 (m, 1 H) 3.35 - 3.49 (m, 2 H) 3.60 (dt, 1 H) 3.82 (br t, 1 H) 4.03 - 4.16 (m, 4 H) 4.23 (ddd, 1 H) 4.66 (t, 1 H) 5.42 - 5.49 (m, 1 H) 6.69 (d, 1 H) 6.81 (d, 2 H) 6.88 (tdd, 1 H) 7.05 (s, 2 H) 7.19 (t, 1 H) 7.32 (d, 1 H) 8.12 (dd, 1 H) 8.60 (dd, 1 H)
LC-MS (method 1): Rt = 0.92 min; MS (ESlpos): m/z = 458 [M+H]⁺

### Example 224

### 2-(6-amino-5-cyanopyridin-3-yl)-N-[(1R)-1-(2-fluorophenyl)ethyl]-6,7-dihydrospiro[pyrazolo[5,1-c][1,4]oxazine-4,3'-pyrrolidine]-1'-carboxamide (mixture of stereoisomers)

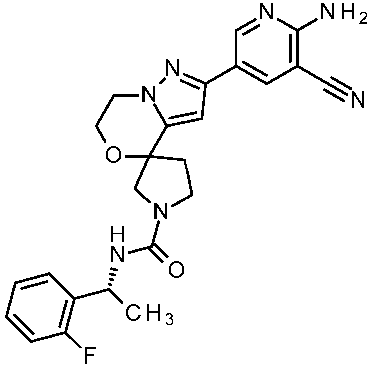

Example 224 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (95.0 mg, 285 µmol) and (1R)-1-(2-fluorophenyl)ethan-1-amine (47.7 mg, 343 µmol).
¹H NMR (400 MHz, DMSO-*d*₆) δ ppm 1.35 (d, 3 H) 2.16 - 2.27 (m, 1 H) 2.31 - 2.40 (m, 1 H) 3.36 - 3.51 (m, 2 H) 3.57 - 3.67 (m, 1 H) 3.82 (t, 1 H) 4.04 - 4.17 (m, 4 H) 5.07 - 5.14 (m, 1 H) 6.59 (dd, 1 H) 6.70 (s, 1 H) 7.05 (s, 2 H) 7.09 - 7.20 (m, 2 H) 7.21 - 7.29 (m, 1 H) 7.42 - 7.48 (m, 1 H) 8.12 (dd, 1 H) 8.59 - 8.62 (m, 1 H)
LC-MS (method 1): Rt = 0.98 min; MS (ESlneg): m/z = 460 [M-H]⁻

### Example 225

### 2'-(6-amino-5-cyanopyridin-3-yl)-N-[(1R)-1-(2-fluorophenyl)ethyl]-5',6'-dihydrospiro[pyrrolidine-3,4'-pyrrolo[1,2-b]pyrazole]-1-carboxamide (mixture of stereoisomers)

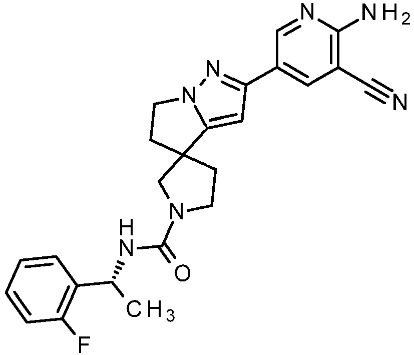

Example 225 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (100 mg, 316 µmol) and (1R)-1-(2-fluorophenyl)ethan-1-amine (52.7 mg, 379 µmol).
¹H NMR (400 MHz, DMSO-*d*₆) δ ppm 1.35 (dd, 3 H) 2.07 (br t, 2 H) 2.52 - 2.57 (m, 2 H) 3.42 - 3.61 (m, 4 H) 4.19 (t, 2 H) 5.07 - 5.15 (m, 1 H) 6.40 - 6.45 (m, 1 H) 6.56 (dd, 1 H) 6.99 (s, 2 H) 7.07 - 7.20 (m, 2 H) 7.20 - 7.30 (m, 1 H) 7.40 - 7.51 (m, 1 H) 8.13 (dd, 1 H) 8.61 (t, 1 H)
LC-MS (method 1): Rt = 0.98 min; MS (ESlneg): m/z = 444 [M-H]⁻

### Example 226

### (rac)-2'-(6-amino-5-cyanopyridin-3-yl)-N-[2-(4-fluorophenyl)propan-2-yl]-5',6'-dihydrospiro[pyrrolidine-3,4'-pyrrolo[1,2-b]pyrazole]-1-carboxamide

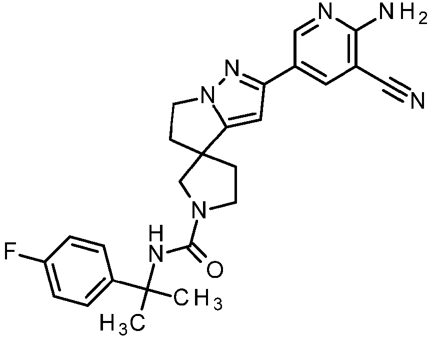

Example 226 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (100 mg, 316 µmol) and 2-(4-fluorophenyl)propan-2-amine (58.0 mg, 379 µmol).
¹H NMR (400 MHz, DMSO-*d*₆) δ ppm 1.52 - 1.58 (m, 6 H) 2.00 - 2.11 (m, 2 H) 2.52 - 2.58 (m, 2 H) 3.35 - 3.47 (m, 3 H) 3.48 - 3.60 (m, 1 H) 4.19 (t, 2 H) 6.07 (s, 1 H) 6.44 (s, 1 H) 6.98 - 7.02 (m, 2 H) 7.05 (t, 2 H) 7.35 - 7.37 (m, 1 H) 7.38 (s, 1 H) 8.14 (d, 1 H) 8.62 (d, 1 H)
LC-MS (method 1): Rt = 1.04 min; MS (ESlneg): m/z = 458 [M-H]⁻

### Example 227

### (rac)-2-(6-amino-5-cyanopyridin-3-yl)-N-[2-(1-methyl-1H-1,2,3-triazol-4-yl)propan-2-yl]-6,7-dihydrospiro[pyrazolo[5,1-c][1,4]oxazine-4,3'-pyrrolidine]-1'-carboxamide

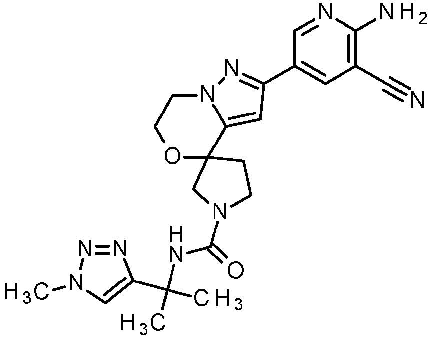

Example 227 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (95.0 mg, 285 µmol) and 2-(1-methyl-1H-1,2,3-triazol-4-yl)propan-2-amine hydrochloride (73.0 mg, 343 µmol).
¹H NMR (400 MHz, DMSO-*d*₆) δ ppm 1.60 (d, 6 H) 2.13 - 2.24 (m, 1 H) 2.28 - 2.36 (m, 1 H) 3.35 - 3.47 (m, 2 H) 3.58 (br t, 1 H) 3.72 - 3.82 (m, 1 H) 3.98 (s, 3 H) 4.04 - 4.17 (m, 4 H) 5.96 (s, 1 H) 6.70 (s, 1 H) 7.04 (s, 2 H) 7.79 (s, 1 H) 8.13 (d, 1 H) 8.61 (d, 1 H)
LC-MS (method 1): Rt = 0.75 min; MS (ESlpos): m/z = 464 [M+H]⁺

### Example 228

### (rac)-2-(6-amino-5-cyanopyridin-3-yl)-N-[(1R)-1-(5-fluoropyridin-3-yl)ethyl]-6,7-dihydrospiro[pyrazolo[5,1-c][1,4]oxazine-4,3'-pyrrolidine]-1'-carboxamide

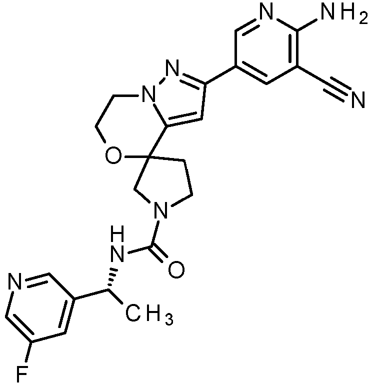

Example 228 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (93.0 mg, 279 µmol) and (1R)-1-(5-fluoropyridin-3-yl)ethan-1-amine (47.0 mg, 335 µmol).
¹H NMR (400 MHz, DMSO-*d*₆) δ ppm 1.41 (d, 3 H) 2.16 - 2.27 (m, 1 H) 2.31 - 2.40 (m, 1 H) 3.35 - 3.52 (m, 2 H) 3.55 - 3.67 (m, 1 H) 3.74 - 3.87 (m, 1 H) 4.05 - 4.17 (m, 4 H) 4.87 - 4.96 (m, 1 H) 6.63 (dd, 1 H) 6.70 (d, 1 H) 7.05 (s, 2 H) 7.68 (ddt, 1 H) 8.12 (t, 1 H) 8.41 - 8.46 (m, 2 H) 8.60 (t, 1 H)
LC-MS (method 1): Rt = 0.82 min; MS (ESlpos): m/z = 463 [M+H]⁺

### Example 229

### (rac)-2'-(6-amino-5-cyanopyridin-3-yl)-N-(1-phenylcyclobutyl)-5',6'-dihydrospiro[pyrrolidine-3,4'-pyrrolo[1,2-b]pyrazole]-1-carboxamide

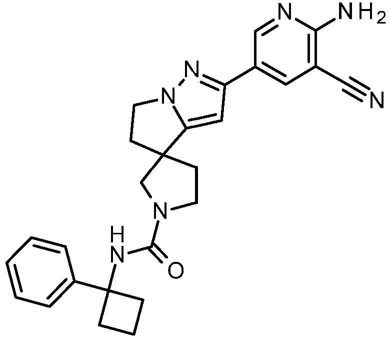

Example 229 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) and 1-phenylcyclobutan-1-amine hydrochloride (53.6 mg, 292 µmol).
¹H NMR (400 MHz, DMSO-*d*₆) δ ppm 1.72 - 1.83 (m, 1 H) 1.95 - 2.05 (m, 1 H) 2.14 - 2.25 (m, 1 H) 2.31 - 2.47 (m, 5 H) 3.35 - 3.47 (m, 2 H) 3.58 (br t, 1 H) 3.77 (br d, 1 H) 4.04 - 4.16 (m, 4 H) 6.67 - 6.73 (m, 2 H) 7.05 (s, 2 H) 7.13 - 7.20 (m, 1 H) 7.29 (t, 2 H) 7.42 (d, 2 H) 8.11 (d, 1 H) 8.59 (d, 1 H)
LC-MS (method 1): Rt = 1.06 min; MS (ESlpos): m/z = 454 [M+H]⁺

### Example 230

### (rac)-2'-(6-amino-5-cyanopyridin-3-yl)-N-[1-(4-fluorophenyl)cyclobutyl]-5',6'-dihydrospiro[pyrrolidine-3,4'-pyrrolo[1,2-b]pyrazole]-1-carboxamide

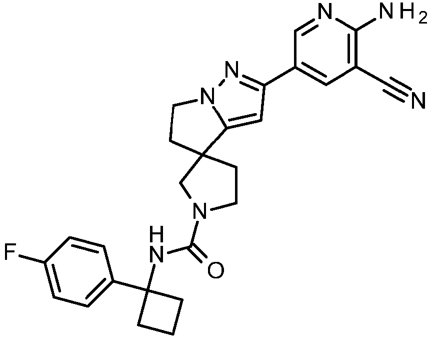

Example 230 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) and 1-(4-fluorophenyl)cyclobutan-1-amine hydrochloride (58.8 mg, 292 µmol).
¹H NMR (400 MHz, DMSO-*d*₆) δ ppm 1.70 - 1.81 (m, 1 H) 1.93 - 2.10 (m, 3 H) 2.34 - 2.47 (m, 4 H) 2.52 - 2.57 (m, 2 H) 3.35 - 3.44 (m, 3 H) 3.46 - 3.56 (m, 1 H) 4.18 (t, 2 H) 6.42 (s, 1 H) 6.71 (s, 1 H) 6.99 (s, 2 H) 7.05 - 7.12 (m, 2 H) 7.41 - 7.47 (m, 2 H) 8.13 (d, 1 H) 8.60 (d, 1 H)
LC-MS (method 1): Rt = 1.07 min; MS (ESlpos): m/z = 473 [M+H]⁺

### Example 231

### (rac)-2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide

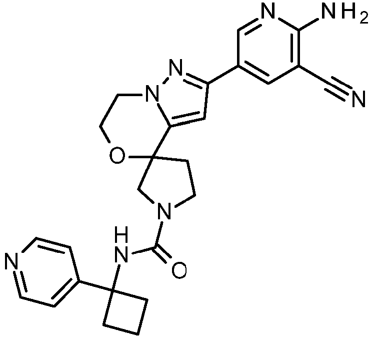

Example 231 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and 1-(pyridin-4-yl)cyclobutan-1-amine (37.9 mg, 256 µmol).
¹H NMR (400 MHz, DMSO-*d*₆) δ ppm 1.79 - 1.88 (m, 1 H) 1.97 - 2.07 (m, 1 H) 2.21 (br d, 1 H) 2.35 - 2.47 (m, 5 H) 3.36 - 3.50 (m, 2 H) 3.60 (br t, 1 H) 3.78 (br d, 1 H) 4.05 - 4.17 (m, 4 H) 6.71 (s, 1 H) 6.89 (s, 1 H) 7.05 (s, 2 H) 7.39 (d, 2 H) 8.12 (d, 1 H) 8.48 (d, 2 H) 8.60 (d, 1 H)
LC-MS (method 1): Rt = 0.86 min; MS (ESlpos): m/z = 471 [M+H]⁺

### Example 232

### (rac)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide

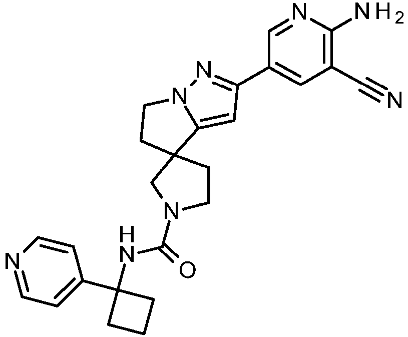

Example 232 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile hydrochloride (77.0 mg, 243 µmol) and 1-(pyridin-4-yl)cyclobutan-1-amine (43.2 mg, 292 µmol).
¹H NMR (400 MHz, DMSO-*d*₆) δ ppm 1.79 - 1.89 (m, 1 H) 1.97 - 2.11 (m, 3 H) 2.35 - 2.48 (m, 4 H) 2.53 - 2.57 (m, 2 H) 3.44 (s, 3 H) 3.55 (br d, 1 H) 4.19 (t, 2 H) 6.44 (s, 1 H) 6.88 (s, 1 H) 7.00 (s, 2 H) 7.42 (d, 2 H) 8.14 (d, 1 H) 8.48 (d, 2 H) 8.61 (d, 1 H)
LC-MS (method 1): Rt = 0.86 min; MS (ESlpos): m/z = 455 [M+H]⁺

### Example 233

### (rac)-2-(6-amino-5-cyanopyridin-3-yl)-N-(1-phenylcyclobutyl)-6,7-dihydrospiro[pyrazolo[5,1-c][1,4]oxazine-4,3'-pyrrolidine]-1'-carboxamide

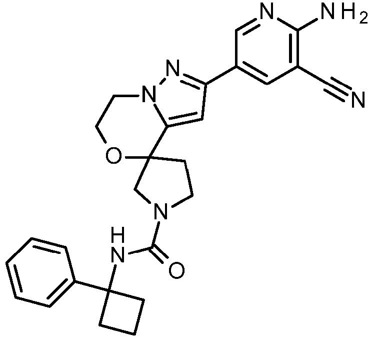

Example 233 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (71.0 mg, 213 µmol) and 1-phenylcyclobutan-1-amine hydrochloride (47.0 mg, 256 µmol).
¹H NMR (400 MHz, DMSO-*d*₆) δ ppm 1.77 (td, 1 H) 1.94 - 2.09 (m, 3 H) 2.33 - 2.46 (m, 3 H) 2.52 - 2.55 (m, 3 H) 3.35 - 3.45 (m, 3 H) 3.46 - 3.56 (m, 1 H) 4.18 (t, 2 H) 6.39 (s, 1 H) 6.70 (s, 1 H) 7.00 (s, 2 H) 7.13 - 7.18 (m, 1 H) 7.28 (t, 2 H) 7.43 (d, 2 H) 8.11 (d, 1 H) 8.60 (d, 1 H)
LC-MS (method 1): Rt = 1.06 min; MS (ESlpos): m/z = 470 [M+H]⁺

### Example 234

### 2'-(6-amino-5-cyanopyridin-3-yl)-N-[(1R)-1-(1,3,4-trimethyl-1H-pyrazol-5-yl)ethyl]-5',6'-dihydrospiro[azetidine-3,4'-pyrrolo[1,2-b]pyrazole]-1-carboxamide

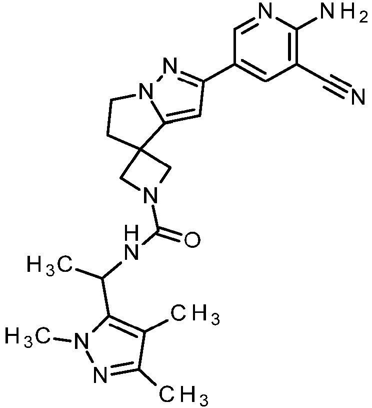

Example 234 was prepared in analogy to Example 31 using 2-amino-5-(5',6'-dihydrospiro[azetidine-3,4'-pyrrolo[1,2-b]pyrazol]-2'-yl)pyridine-3-carbonitrile (75.0 mg, 282 µmol) and 1-(1,3,4-trimethyl-1H-pyrazol-5-yl)ethan-1-amine (51.8 mg, 338 µmol).
¹H NMR (400 MHz, DMSO-*d*₆) δ ppm 1.40 (d, 3 H) 1.96 (s, 3 H) 1.99 (s, 3 H) 2.83 (t, 2 H) 3.71 (s, 3 H) 3.93 - 4.05 (m, 4 H) 4.11 (t, 2 H) 4.88 (t, 1 H) 6.65 (s, 1 H) 6.92 (d, 1 H) 7.00 (s, 2 H) 8.15 (d, 1 H) 8.63 (d, 1 H)
LC-MS (Method 1): Rt = 0.83 min; MS (ESlpos): m/z = 446 [M+H]⁺

### Example 235

### (rac)-2-(6-amino-5-cyanopyridin-3-yl)-N-[2-(pyridin-4-yl)propan-2-yl]-6,7-dihydrospiro[pyrazolo[5,1-c][1,4]oxazine-4,3'-pyrrolidine]-1'-carboxamide

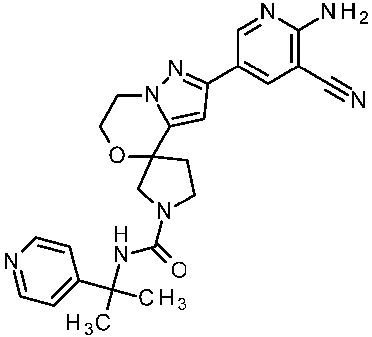

Example 235 was prepared in analogy to Example 31 using (rac)-2-amino-5-[6,7-dihydrospiro[pyrazolo[5,1-c][1,4]oxazine-4,3'-pyrrolidin]-2-yl]pyridine-3-carbonitrile hydrochloride (93.0 mg, 279 µmol) and 2-(pyridin-4-yl)propan-2-amine (45.7 mg, 335 µmol).
¹H NMR (400 MHz, DMSO-*d*₆) δ ppm 1.54 (s, 6 H) 2.15 - 2.27 (m, 1 H) 2.29 - 2.40 (m, 1 H) 3.35 - 3.51 (m, 2 H) 3.61 (br t, 1 H) 3.80 (br d, 1 H) 4.07 - 4.18 (m, 4 H) 6.25 (s, 1 H) 6.72 (s, 1 H) 7.05 (s, 2 H) 7.32 (d, 2 H) 8.13 (d, 1 H) 8.44 (d, 2 H) 8.61 (d, 1 H)
LC-MS (method 1): Rt = 0.82 min; MS (ESlneg): m/z = 457 [M-H]⁻

### Example 236

### 2'-[6-amino-5-(difluoromethoxy)pyridin-3-yl]-N-[1-(pyridin-3-yl)cyclobutyl]-5',6'-dihydrospiro[azetidine-3,4'-pyrrolo[1,2-b]pyrazole]-1-carboxamide

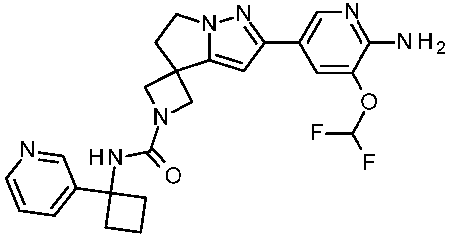

Example 236 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-(pyridin-3-yl)cyclobutan-1-amine (35.6 mg, 240 µmol).
LC-MS (method 1): Rt = 0.88 min; MS (ESlpos): m/z = 482 [M+H]⁺
¹H-NMR (400 MHz, DMSO-d6) δ [ppm]: 1.066 (3.99), 1.235 (0.40), 1.245 (1.29), 1.254 (0.89), 1.261 (1.42), 1.267 (1.40), 1.284 (1.24), 1.779 (0.54), 1.785 (0.47), 1.796 (1.09), 1.803 (1.07), 1.813 (0.86), 1.819 (1.04), 1.824 (1.35), 1.834 (0.53), 1.841 (0.89), 2.008 (0.71), 2.024 (1.22), 2.031 (1.03), 2.036 (0.80), 2.042 (0.82), 2.047 (1.18), 2.052 (1.09), 2.063 (0.60), 2.069 (0.58), 2.073 (0.72), 2.413 (0.71), 2.430 (1.13), 2.442 (2.62), 2.458 (5.56), 2.479 (6.00), 2.518 (2.60), 2.522 (1.93), 2.727 (3.16), 2.815 (3.09), 2.833 (5.23), 2.850 (3.40), 2.887 (3.90), 3.979 (2.66), 4.000 (10.91), 4.009 (11.07), 4.029 (2.72), 4.095 (3.45), 4.113 (5.53), 4.130 (3.39), 6.207 (3.15), 6.616 (16.00), 7.005 (3.56), 7.023 (0.46), 7.150 (0.41), 7.190 (7.34), 7.219 (7.44), 7.374 (4.95), 7.386 (2.50), 7.388 (2.55), 7.396 (2.65), 7.408 (2.64), 7.652 (5.00), 7.654 (5.21), 7.656 (5.19), 7.831 (2.05), 7.836 (2.71), 7.841 (2.23), 7.851 (1.93), 7.857 (2.52), 7.861 (1.96), 7.950 (0.50), 8.236 (8.81), 8.241 (8.52), 8.423 (4.19), 8.427 (4.26), 8.435 (4.31), 8.439 (3.99), 8.656 (5.05), 8.661 (5.22), 8.663 (4.93).

### Example 237

### (rac)-2'-[6-amino-5-(trifluoromethoxy)pyridin-3-yl]-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide

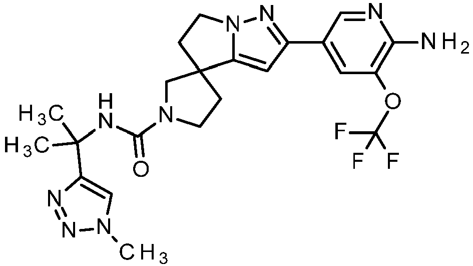

Example 237 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 2-(1-methyl-1H-1,2,3-triazol-4-yl)propan-2-amine-hydrochloride salt (56.5 mg, 265 µmol).
LC-MS (method 2): Rt = 0.85 min; MS (ESlpos): m/z = 506 [M+H]⁺
1H-NMR (400 MHz, DMSO-d6) δ [ppm]: 0.969 (2.09), 0.984 (1.89), 1.510 (0.99), 1.593 (8.69), 1.601 (8.54), 2.023 (0.61), 2.038 (1.12), 2.055 (1.35), 2.074 (0.97), 2.084 (2.27), 2.336 (0.46), 2.518 (6.88), 2.522 (5.45), 2.539 (1.81), 3.399 (1.40), 3.420 (4.08), 3.443 (0.74), 3.502 (0.51), 3.521 (0.74), 3.535 (0.56), 3.542 (0.48), 3.975 (16.00), 4.136 (0.43), 4.159 (1.48), 4.176 (2.19), 4.194 (1.38), 5.923 (2.55), 6.428 (5.76), 6.499 (3.95), 7.756 (1.45), 7.760 (2.06), 7.795 (5.86), 8.312 (0.41), 8.317 (0.43), 8.336 (2.73), 8.341 (2.73).

### Example 238

### 2'-[6-amino-5-(trifluoromethoxy)pyridin-3-yl]-N-[(1R)-1-(2-methylpyrimidin-5-yl)ethyl]-5',6'-dihydrospiro[pyrrolidine-3,4'-pyrrolo[1,2-b]pyrazole]-1-carboxamide (mixture of stereoisomers)

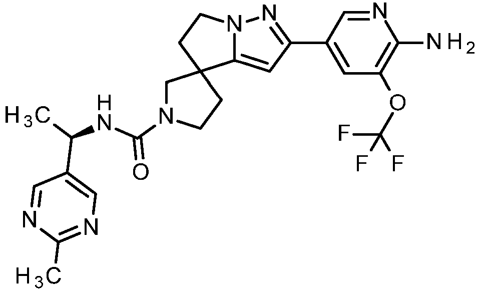

Example 238 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1R)-1-(2-methylpyrimidin-5-yl)ethan-1-amine (36.4 mg, 265 µmol).
LC-MS (method 1): Rt = 0.87 min; MS (ESlpos): m/z = 503 [M+H]⁺
1H-NMR (400 MHz, DMSO-d6) δ [ppm]: 1.065 (0.44), 1.122 (0.96), 1.138 (0.96), 1.161 (0.91), 1.179 (0.91), 1.241 (0.62), 1.256 (0.93), 1.381 (0.69), 1.401 (5.88), 1.408 (5.97), 1.419 (6.00), 1.425 (5.55), 2.060 (2.41), 2.074 (2.52), 2.154 (0.78), 2.327 (1.05), 2.523 (6.08), 2.544 (3.36), 2.569 (16.00), 2.579 (15.06), 2.622 (0.58), 2.669 (1.18), 3.007 (0.40), 3.174 (0.51), 3.409 (2.23), 3.434 (4.19), 3.440 (3.95), 3.447 (3.66), 3.473 (1.05), 3.483 (0.93), 3.513 (0.93), 3.537 (0.93), 3.553 (0.98), 3.840 (0.45), 4.158 (2.94), 4.176 (5.17), 4.193 (3.48), 4.818 (1.02), 4.837 (1.41), 4.847 (0.94), 6.390 (3.10), 6.401 (6.64), 6.504 (6.44), 6.587 (1.60), 6.599 (1.76), 6.606 (1.76), 6.618 (1.54), 7.051 (0.63), 7.752 (4.14), 7.756 (4.59), 8.324 (3.81), 8.328 (6.17), 8.333 (3.79), 8.351 (1.16), 8.580 (0.74), 8.631 (10.58), 8.656 (10.72).

### Example 239

### 2'-[6-amino-5-(trifluoromethoxy)pyridin-3-yl]-N-[(1R)-1-(pyridin-2-yl)ethyl]-5',6'-dihydrospiro[pyrrolidine-3,4'-pyrrolo[1,2-b]pyrazole]-1-carboxamide (mixture of stereoisomers)

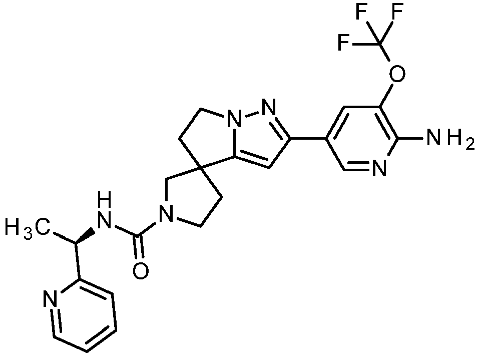

Example 239 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1R)-1-(pyridin-2-yl)ethan-1-amine (32.4 mg, 265 µmol).
LC-MS (method 1): Rt = 0.95 min; MS (ESlpos): m/z = 488 [M+H]⁺
¹H-NMR (400MHz, DMSO-d₆): δ [ppm]= 1.39 (dd, 3H), 2.02 - 2.16 (m, 2H), 2.54 (br t, 2H), 3.43 - 3.52 (m, 3H), 3.53 - 3.64 (m, 1H), 4.19 (t, 2H), 4.83 - 4.94 (m, 1H), 6.39 - 6.45 (m, 1H), 6.51 (s, 3H), 7.17 - 7.26 (m, 1H), 7.40 (dd, 1H), 7.69 - 7.79 (m, 2H), 8.33 (t, 1H), 8.46 - 8.52 (m, 1H).

### Example 240

### 2'-[6-amino-5-(trifluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide (mixture of stereoisomer)

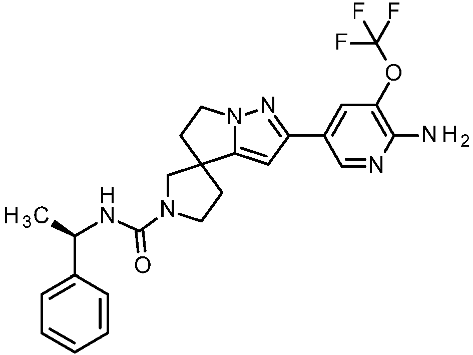

Example 240 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1R)-1-phenylethan-1-amine (32.1 mg, 265 µmol).
LC-MS (method 1): Rt = 1.09 min; MS (ESlpos): m/z = 487 [M+H]⁺
1H-NMR (400 MHz, DMSO-d6) δ [ppm]: 1.018 (0.65), 1.036 (0.88), 1.354 (10.31), 1.360 (11.19), 1.372 (10.68), 1.378 (10.77), 2.061 (4.16), 2.084 (15.58), 2.327 (1.76), 2.523 (10.08), 2.527 (10.17), 2.546 (6.24), 2.669 (1.85), 3.363 (1.43), 3.379 (1.06), 3.427 (3.05), 3.453 (16.00), 3.533 (1.62), 3.556 (1.94), 4.162 (4.90), 4.179 (8.60), 4.196 (4.62), 4.824 (1.85), 4.840 (2.77), 4.858 (2.03), 6.195 (0.46), 6.390 (5.69), 6.407 (11.38), 6.464 (3.14), 6.485 (3.33), 6.505 (12.53), 7.155 (1.16), 7.172 (3.56), 7.189 (4.53), 7.207 (2.13), 7.253 (2.77), 7.273 (6.52), 7.292 (6.24), 7.296 (6.66), 7.315 (10.45), 7.334 (4.67), 7.339 (6.80), 7.360 (3.19), 7.747 (5.27), 8.315 (5.18), 8.320 (6.20), 8.323 (7.12), 8.328 (6.06).

### Example 241

### 2'-[6-amino-5-(trifluoromethoxy)pyridin-3-yl]-N-[(1R)-1-(pyridin-3-yl)ethyl]-5',6'-dihydrospiro[pyrrolidine-3,4'-pyrrolo[1,2-b]pyrazole]-1-carboxamide (mixture of stereoisomers)

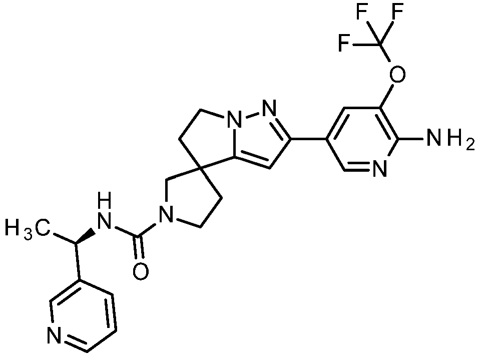

Example 241 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1R)-1-(pyridin-3-yl)ethan-1-amine-hydrochloride salt (51.7 mg, 265 µmol).
LC-MS (method 1): Rt = 0.91 min; MS (ESlpos): m/z = 488 [M+H]⁺
1H-NMR (400 MHz, DMSO-d6) δ [ppm]: 1.101 (1.56), 1.126 (1.38), 1.142 (1.37), 1.165 (1.34), 1.181 (1.35), 1.365 (0.86), 1.388 (12.18), 1.394 (13.05), 1.406 (12.86), 1.412 (12.55), 2.056 (4.78), 2.063 (4.81), 2.071 (4.71), 2.326 (0.58), 2.664 (0.50), 2.668 (0.63), 2.727 (3.61), 2.886 (4.48), 3.404 (2.95), 3.422 (5.39), 3.428 (4.29), 3.448 (11.34), 3.454 (13.21), 3.462 (10.60), 3.481 (3.11), 3.488 (3.13), 3.507 (2.14), 3.525 (2.84), 3.541 (2.64), 3.561 (2.59), 3.576 (1.90), 3.583 (1.73), 3.602 (1.17), 3.724 (0.46), 4.160 (5.68), 4.177 (10.04), 4.194 (5.62), 4.852 (2.02), 4.857 (2.12), 4.870 (3.08), 4.875 (3.03), 4.888 (2.19), 6.395 (6.88), 6.412 (14.46), 6.506 (16.00), 6.569 (3.17), 6.579 (3.61), 6.588 (3.43), 6.599 (3.19), 7.272 (9.12), 7.274 (8.92), 7.291 (2.34), 7.305 (2.55), 7.312 (4.80), 7.324 (4.81), 7.332 (2.75), 7.344 (2.53), 7.714 (1.79), 7.719 (2.85), 7.723 (1.87), 7.734 (1.92), 7.754 (9.07), 7.771 (1.89), 7.950 (0.60), 8.192 (3.36), 8.323 (8.36), 8.325 (10.67), 8.330 (8.50), 8.392 (4.08), 8.396 (4.08), 8.404 (6.73), 8.408 (6.46), 8.417 (3.97), 8.421 (3.59), 8.541 (4.90), 8.547 (4.90), 8.561 (4.80), 8.567 (4.63).

### Example 242

### 2'-[6-amino-5-(trifluoromethoxy)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide (mixture of stereoisomers)

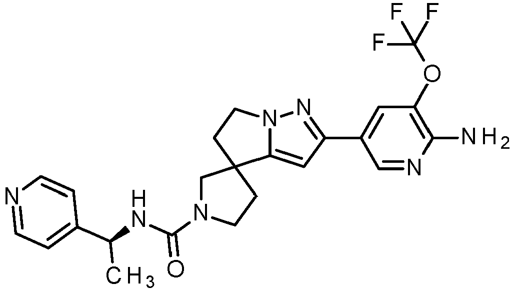

Example 242 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1S)-1-(pyridin-4-yl)ethan-1-amine (32.4 mg, 265 µmol).
LC-MS (method 2): Rt = 0.65 min; MS (ESlpos): m/z = 488 [M+H]⁺
1H-NMR (400 MHz, DMSO-d6) δ [ppm]: 1.067 (5.09), 1.141 (1.95), 1.154 (5.30), 1.168 (2.06), 1.185 (1.79), 1.245 (1.65), 1.261 (2.10), 1.267 (2.06), 1.284 (1.56), 1.364 (15.54), 1.378 (15.40), 2.080 (7.06), 2.539 (12.20), 2.556 (7.28), 2.625 (0.68), 3.464 (14.42), 3.478 (13.70), 3.502 (5.24), 3.525 (3.07), 3.542 (3.65), 3.566 (3.53), 3.584 (3.55), 4.169 (6.54), 4.185 (11.09), 4.202 (6.41), 4.810 (3.13), 4.827 (4.46), 4.844 (3.05), 6.423 (8.52), 6.430 (10.27), 6.513 (16.00), 6.614 (3.83), 6.623 (4.48), 6.632 (4.45), 6.641 (3.76), 7.353 (6.70), 7.366 (7.17), 7.384 (6.93), 7.396 (6.72), 7.761 (9.06), 8.137 (1.39), 8.336 (11.55), 8.494 (8.05).

### Example 243

### (rac)-2'-[6-amino-5-(trifluoromethoxy)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide

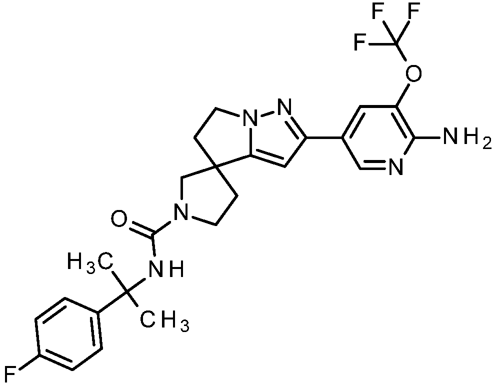

Example 243 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 2-(4-fluorophenyl)propan-2-amine (40.6 mg, 265 µmol).
LC-MS (method 2): Rt = 1.12 min; MS (ESlpos): m/z = 519 [M+H]⁺
¹H-NMR (400MHz, DMSO-d₆): δ [ppm]= 1.55 (d, 6H), 1.99 - 2.13 (m, 2H), 2.52 - 2.56 (m, 2H), 3.38 - 3.48 (m, 3H), 3.51 - 3.61 (m, 1H), 4.19 (t, 2H), 6.07 (s, 1H), 6.41 (s, 1H), 6.51 (s, 2H), 6.99 - 7.09 (m, 2H), 7.33 - 7.41 (m, 2H), 7.75 (t, 1H), 8.33 (d, 1H).

### Example 244

### 2'-[6-amino-5-(trifluoromethoxy)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide (mixture of stereoisomers)

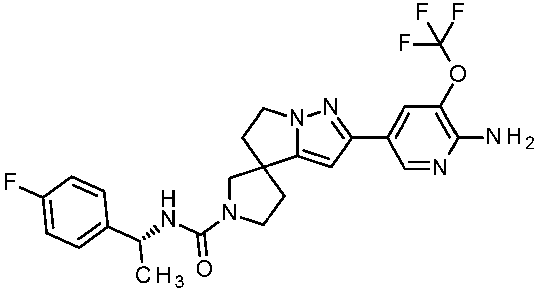

Example 244 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1R)-1-(4-fluorophenyl)ethan-1-amine (36.9 mg, 265 µmol).
LC-MS (method 2): Rt = 1.06 min; MS (ESlpos): m/z = 505 [M+H]⁺
1H-NMR (400 MHz, DMSO-d6) δ [ppm]: 1.012 (1.72), 1.028 (2.12), 1.044 (0.54), 1.064 (1.39), 1.154 (1.13), 1.228 (0.70), 1.342 (12.14), 1.351 (13.32), 1.360 (13.24), 1.368 (11.59), 1.539 (0.53), 1.556 (0.51), 2.043 (4.14), 2.059 (7.76), 2.073 (4.41), 2.327 (0.85), 2.539 (9.93), 2.555 (6.13), 2.587 (0.53), 2.669 (0.88), 3.385 (1.95), 3.421 (3.00), 3.430 (2.91), 3.447 (14.80), 3.480 (2.78), 3.502 (1.47), 3.519 (2.65), 3.527 (2.22), 3.543 (3.27), 3.559 (2.00), 3.568 (1.85), 3.588 (1.80), 3.679 (0.91), 3.701 (0.99), 3.713 (0.99), 4.007 (3.07), 4.177 (6.72), 4.194 (10.83), 4.211 (6.42), 4.314 (1.04), 4.642 (0.54), 4.820 (2.40), 4.837 (3.53), 4.855 (2.44), 6.494 (16.00), 6.644 (0.77), 7.066 (3.83), 7.089 (11.40), 7.111 (12.69), 7.133 (4.87), 7.159 (0.53), 7.181 (0.69), 7.203 (0.53), 7.231 (1.66), 7.271 (0.54), 7.311 (0.80), 7.337 (4.49), 7.352 (5.64), 7.359 (7.01), 7.376 (6.31), 7.384 (4.97), 7.398 (3.94), 7.506 (0.67), 7.517 (0.64), 7.530 (0.72), 7.551 (0.70), 7.637 (0.46), 7.695 (1.09), 7.995 (5.80), 8.321 (0.51), 8.326 (0.57), 8.345 (10.63), 8.350 (10.32).

### Example 245

### (rac)-2-[6-amino-5-(trifluoromethoxy)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide

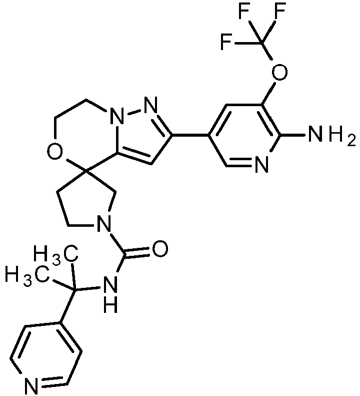

Example 245 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-(pyridin-4-yl)propan-2-amine (34.5 mg, 253 µmol).
LC-MS (method 1): Rt = 0.94 min; MS (ESlneg): m/z = 516 [M-H]⁻
¹H-NMR (400 MHz, DMSO-d6) δ [ppm]: 1.536 (3.38), 2.727 (13.33), 2.886 (16.00), 3.160 (0.98), 3.172 (1.02), 4.104 (0.42), 4.118 (0.73), 4.127 (0.81), 4.137 (0.74), 5.756 (1.44), 6.254 (0.74), 6.561 (1.11), 6.723 (1.27), 7.308 (1.00), 7.313 (0.68), 7.320 (0.69), 7.324 (1.03), 7.749 (0.43), 7.752 (0.58), 7.950 (2.28), 8.330 (0.81), 8.335 (0.84), 8.434 (1.04), 8.438 (0.67), 8.446 (0.67), 8.449 (1.01).

### Example 246

### (rac)-2-[6-amino-5-(trifluoromethoxy)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide

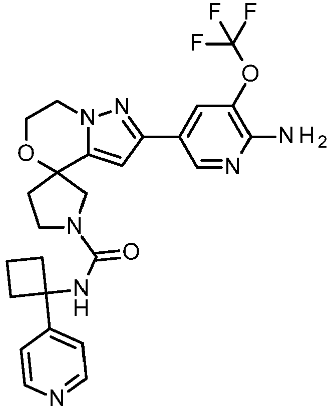

Example 246 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(pyridin-4-yl)cyclobutan-1-amine (37.5 mg, 253 µmol).
LC-MS (method 1): Rt = 0.96 min; MS (ESlneg): m/z = 528 [M-H]⁻
¹H-NMR (400 MHz, DMSO-d6) δ [ppm]: 1.035 (0.61), 1.052 (1.47), 1.065 (13.73), 1.070 (1.50), 1.170 (0.65), 1.185 (0.67), 1.199 (0.79), 1.215 (1.34), 1.231 (2.12), 1.252 (11.23), 1.269 (16.00), 1.282 (10.47), 1.286 (4.86), 1.298 (10.00), 1.816 (0.43), 1.821 (0.46), 1.838 (0.48), 1.843 (0.54), 1.907 (0.62), 2.009 (0.51), 2.031 (0.47), 2.036 (0.45), 2.244 (0.51), 2.323 (0.68), 2.327 (0.71), 2.332 (0.67), 2.337 (0.73), 2.360 (0.72), 2.373 (0.89), 2.389 (1.01), 2.416 (1.39), 2.434 (1.41), 2.456 (1.00), 2.523 (1.39), 2.540 (0.57), 3.087 (0.43), 3.098 (0.48), 3.105 (1.36), 3.115 (1.35), 3.124 (1.39), 3.134 (1.32), 3.142 (0.52), 3.153 (0.54), 3.164 (1.01), 3.426 (0.48), 3.468 (0.54), 3.496 (0.56), 3.565 (7.41), 3.577 (1.20), 3.587 (1.16), 3.594 (1.59), 3.603 (1.58), 3.610 (1.11), 3.620 (1.09), 3.770 (0.72), 3.799 (0.62), 4.071 (0.60), 4.089 (0.73), 4.121 (2.15), 4.134 (2.87), 6.561 (3.59), 6.718 (3.36), 6.917 (2.02), 7.412 (3.55), 7.417 (2.32), 7.424 (2.38), 7.428 (3.54), 7.739 (1.51), 7.743 (2.01), 8.320 (3.09), 8.324 (2.97), 8.489 (3.68), 8.493 (2.35), 8.501 (2.32), 8.505 (3.50).

### Example 247

### 2'-[6-amino-5-(difluoromethoxy)pyridin-3-yl]-N-(1-phenylcyclobutyl)-5',6'-dihydrospiro[azetidine-3,4'-pyrrolo[1,2-b]pyrazole]-1-carboxamide

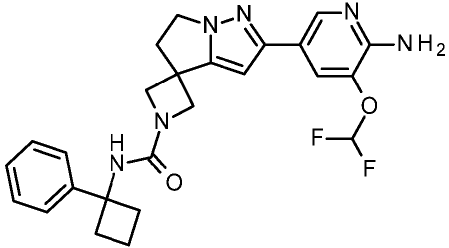

Example 247 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-phenylcyclobutan-1-amine-hydrochloride salt (44.1 mg, 240 µmol).
LC-MS (method 1): Rt = 1.07 min; MS (ESlpos): m/z = 480 [M+H]⁺
¹H-NMR (400 MHz, DMSO-d6) δ [ppm]: 1.053 (0.43), 1.066 (6.06), 1.753 (0.53), 1.769 (1.05), 1.775 (1.08), 1.791 (1.11), 1.797 (1.29), 1.813 (0.81), 1.982 (0.65), 1.998 (1.21), 2.021 (1.14), 2.025 (1.08), 2.074 (0.78), 2.084 (0.48), 2.364 (1.02), 2.395 (2.50), 2.411 (2.98), 2.416 (2.55), 2.433 (1.85), 2.448 (2.13), 2.465 (2.54), 2.471 (3.08), 2.523 (4.41), 2.728 (1.44), 2.812 (3.06), 2.829 (5.13), 2.847 (3.37), 2.888 (1.74), 3.315 (1.05), 3.319 (1.25), 3.363 (1.62), 3.942 (1.05), 3.968 (2.23), 3.989 (12.81), 3.994 (12.52), 4.015 (2.27), 4.095 (3.42), 4.113 (5.34), 4.130 (3.31), 5.770 (0.43), 6.164 (8.96), 6.603 (16.00), 6.999 (3.76), 7.070 (7.38), 7.164 (1.03), 7.167 (1.76), 7.170 (1.08), 7.184 (10.45), 7.201 (1.80), 7.204 (2.90), 7.207 (1.60), 7.296 (4.69), 7.315 (7.77), 7.329 (1.78), 7.334 (4.77), 7.368 (3.36), 7.415 (7.24), 7.418 (7.87), 7.436 (6.06), 7.439 (4.64), 7.643 (5.10), 8.236 (8.83), 8.241 (7.98).

### Example 248

### (rac)-2'-[6-amino-5-(trifluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide

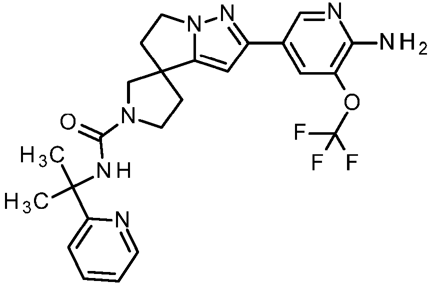

Example 248 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 2-(pyridin-2-yl)propan-2-amine (36.1 mg, 265 µmol).
LC-MS (method 1): Rt = 1.02 min; MS (ESlpos): m/z = 502 [M+H]⁺
¹H-NMR (400MHz, DMSO-d₆): δ [ppm]= 1.59 (s, 6H), 2.02 - 2.16 (m, 2H), 2.55 (br t, 2H), 3.41 - 3.51 (m, 3H), 3.54 - 3.64 (m, 1H), 4.19 (t, 2H), 6.40 - 6.46 (m, 2H), 6.51 (s, 2H), 7.19 (dd, 1H), 7.47 (d, 1H), 7.71 (td, 1H), 7.74 - 7.78 (m, 1H), 8.34 (d, 1H), 8.43 - 8.49 (m, 1H).

### Example 249

### (rac)-2'-[6-amino-5-(trifluoromethoxy)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide

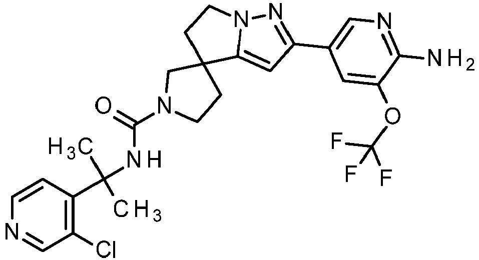

Example 249 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 2-(3-chloropyridin-4-yl)propan-2-amine (45.3 mg, 265 µmol).
LC-MS (method 1): Rt = 1.02 min; MS (ESlpos): m/z = 536 [M+H]⁺
¹H-NMR (400 MHz, DMSO-d6) δ [ppm]: 0.997 (0.75), 1.014 (0.75), 1.627 (1.43), 1.653 (16.00), 2.032 (0.66), 2.046 (1.23), 2.064 (1.43), 2.081 (0.59), 2.518 (3.42), 2.522 (2.58), 3.296 (0.47), 3.348 (1.89), 3.363 (0.41), 3.376 (0.48), 3.380 (0.41), 3.384 (0.40), 3.413 (4.36), 3.500 (0.65), 4.158 (1.56), 4.176 (3.31), 4.194 (1.52), 6.332 (3.14), 6.391 (6.61), 6.514 (4.24), 7.426 (2.66), 7.439 (2.70), 7.735 (1.72), 7.739 (2.33), 7.743 (1.63), 8.319 (3.80), 8.323 (3.93), 8.388 (3.84), 8.402 (3.59), 8.409 (7.15).

### Example 250

### 2'-[6-amino-5-(difluoromethoxy)pyridin-3-yl]-N-[2-(2-fluorophenyl)propan-2-yl]-5',6'-dihydrospiro[azetidine-3,4'-pyrrolo[1,2-b]pyrazole]-1-carboxamide

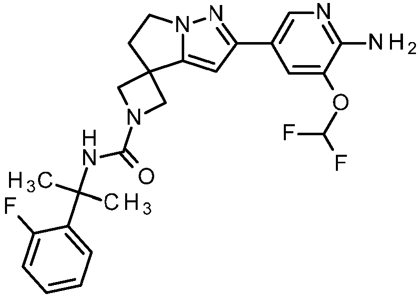

Example 250 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 2-(2-fluorophenyl)propan-2-amine-hydrochloride salt (45.5 mg, 240 µmol).
LC-MS (method 1): Rt = 1.08 min; MS (ESlneg): m/z = 485 [M-H]⁻
¹H-NMR (400 MHz, DMSO-d6) δ [ppm]: 1.066 (16.00), 1.625 (8.69), 1.665 (2.88), 2.522 (0.41), 2.727 (2.33), 2.819 (0.84), 2.837 (1.39), 2.854 (0.91), 2.887 (2.85), 3.944 (2.27), 4.009 (5.89), 4.102 (0.92), 4.120 (1.48), 4.137 (0.91), 6.167 (2.44), 6.606 (4.65), 6.611 (2.24), 7.007 (0.93), 7.058 (0.41), 7.062 (0.45), 7.079 (0.52), 7.082 (0.57), 7.093 (0.47), 7.104 (0.48), 7.107 (0.52), 7.114 (0.65), 7.122 (1.05), 7.126 (0.85), 7.142 (0.73), 7.145 (0.61), 7.191 (1.92), 7.231 (0.45), 7.238 (0.42), 7.245 (0.41), 7.250 (0.42), 7.309 (0.68), 7.313 (0.47), 7.331 (0.80), 7.375 (0.83), 7.652 (1.37), 8.248 (2.15), 8.253 (2.06).

### Example 251

### (rac)-2-[6-amino-5-(difluoromethoxy)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide

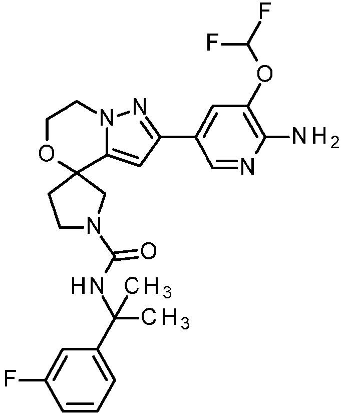

Example 251 was prepared in analogy to Example 31 using (rac)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and 2-(3-fluorophenyl)propan-2-amine (36.9 mg, 241 µmol).
LC-MS (method 1): Rt = 1.08 min; MS (ESlpos): m/z = 517 [M+H]⁺
¹H-NMR (400 MHz, DMSO-d6) δ [ppm]: 1.137 (0.72), 1.232 (0.86), 1.494 (1.28), 1.549 (16.00), 1.555 (15.49), 2.226 (0.70), 2.251 (0.96), 2.275 (0.53), 2.309 (0.85), 2.323 (2.30), 2.327 (2.94), 2.332 (2.02), 2.336 (1.06), 2.357 (0.45), 2.518 (6.15), 2.523 (4.30), 2.660 (0.65), 2.665 (1.44), 2.669 (2.07), 2.673 (1.46), 2.678 (0.61), 3.366 (0.42), 3.391 (0.82), 3.408 (0.84), 3.482 (1.08), 3.511 (1.28), 3.575 (0.67), 3.597 (1.17), 3.621 (0.54), 3.788 (1.48), 3.816 (1.27), 4.070 (0.43), 4.084 (0.94), 4.101 (1.63), 4.123 (5.31), 4.132 (4.10), 4.149 (1.06), 6.148 (4.92), 6.217 (6.77), 6.680 (10.42), 6.941 (0.87), 6.945 (0.95), 6.947 (0.97), 6.960 (1.65), 6.967 (1.80), 6.982 (0.97), 6.987 (1.09), 6.989 (1.08), 6.994 (3.11), 7.105 (1.33), 7.111 (1.71), 7.116 (1.40), 7.133 (1.27), 7.138 (1.74), 7.144 (1.39), 7.179 (8.00), 7.197 (2.53), 7.276 (1.58), 7.292 (1.78), 7.297 (2.48), 7.312 (2.37), 7.316 (1.25), 7.332 (1.02), 7.363 (2.58), 7.617 (3.76), 7.619 (3.60), 8.198 (6.84), 8.203 (6.16).

### Example 252

### 2-[6-amino-5-(difluoromethoxy)pyridin-3-yl]-N-[(1S)-1-(3-chloropyridin-4-yl)ethyl]-6,7-dihydrospiro[pyrazolo[5,1-c][1,4]oxazine-4,3'-pyrrolidine]-1'-carboxamide (mixture of stereoisomers)

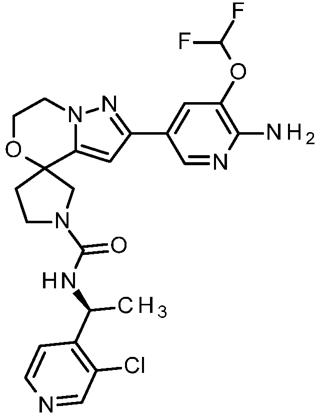

Example 252 was prepared in analogy to Example 31 using (rac)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and (1S)-1-(3-chloropyridin-4-yl)ethan-1-amine-hydrochloride salt (46.5 mg, 241 µmol).
LC-MS (method 1): Rt = 0.90 min; MS (ESlpos): m/z = 520 [M+H]⁺
¹H-NMR (400 MHz, DMSO-d6) δ [ppm]: 1.041 (0.44), 1.060 (0.48), 1.137 (0.67), 1.232 (1.01), 1.335 (8.93), 1.337 (9.20), 1.352 (9.24), 1.355 (9.12), 2.084 (16.00), 2.235 (0.82), 2.262 (0.99), 2.332 (2.50), 2.336 (1.65), 2.348 (1.11), 2.518 (8.14), 2.523 (5.73), 2.673 (1.91), 2.678 (0.83), 3.370 (0.49), 3.393 (0.74), 3.410 (0.91), 3.432 (0.89), 3.458 (1.10), 3.489 (0.85), 3.527 (0.72), 3.556 (1.14), 3.585 (0.95), 3.610 (0.43), 3.646 (0.54), 3.668 (0.92), 3.690 (0.43), 3.776 (1.23), 3.803 (1.02), 3.842 (1.22), 3.870 (1.06), 4.050 (0.52), 4.064 (1.08), 4.080 (1.55), 4.097 (2.10), 4.121 (4.37), 4.132 (5.74), 5.046 (1.10), 5.055 (1.30), 5.064 (1.77), 5.073 (1.88), 5.082 (1.26), 5.090 (1.18), 6.221 (7.84), 6.673 (5.21), 6.681 (6.04), 6.774 (1.45), 6.793 (1.46), 6.810 (1.43), 6.828 (1.34), 6.993 (2.08), 6.998 (2.23), 7.178 (4.52), 7.183 (4.64), 7.362 (1.95), 7.367 (1.91), 7.483 (3.12), 7.495 (3.61), 7.499 (3.50), 7.511 (3.04), 7.615 (2.93), 7.620 (2.92), 8.193 (4.55), 8.197 (4.47), 8.203 (5.02), 8.208 (4.85), 8.483 (4.11), 8.494 (5.27), 8.506 (4.10), 8.534 (14.65).

### Example 253

### 2-[6-amino-5-(trifluoromethoxy)pyridin-3-yl]-N-[1-(1,3,5-trimethyl-1H-pyrazol-4-yl)ethyl]-6,7-dihydrospiro[pyrazolo[5,1-c][1,4]oxazine-4,3'-pyrrolidine]-1'-carboxamide (mixture of stereoisomers)

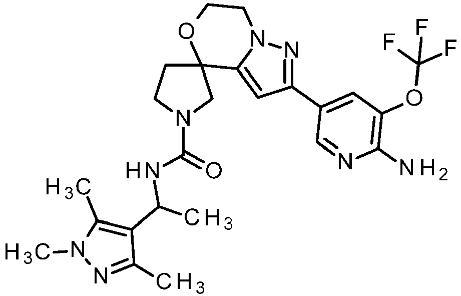

Example 253 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(1,3,5-trimethyl-1H-pyrazol-4-yl)ethan-1-amine (38.8 mg, 253 µmol).
LC-MS (method 1): Rt = 0.92 min; MS (ESlneg): m/z = 533 [M-H]⁻
¹H-NMR (400 MHz, DMSO-d6) δ [ppm]: 1.066 (4.69), 1.109 (0.88), 1.125 (0.91), 1.137 (0.88), 1.154 (0.89), 1.285 (0.45), 1.304 (0.51), 1.327 (3.63), 1.333 (3.82), 1.345 (3.75), 1.351 (3.72), 2.096 (1.22), 2.120 (13.83), 2.169 (1.47), 2.191 (16.00), 2.213 (0.80), 2.238 (0.42), 2.288 (0.62), 2.304 (0.74), 2.322 (0.56), 2.327 (0.49), 2.332 (0.44), 2.336 (0.44), 2.518 (0.69), 2.523 (0.45), 2.728 (0.74), 2.888 (0.84), 3.318 (0.63), 3.357 (0.55), 3.374 (0.47), 3.384 (1.18), 3.413 (1.02), 3.435 (0.70), 3.464 (0.81), 3.523 (0.62), 3.563 (15.13), 3.578 (0.78), 3.724 (0.67), 3.751 (0.54), 3.797 (0.65), 3.824 (0.58), 3.940 (0.80), 4.052 (0.68), 4.059 (0.75), 4.077 (0.76), 4.090 (1.11), 4.098 (1.28), 4.118 (3.14), 4.682 (0.71), 4.686 (0.72), 4.700 (1.01), 4.703 (1.04), 4.717 (0.70), 4.720 (0.68), 6.086 (0.82), 6.103 (0.80), 6.134 (0.87), 6.150 (0.84), 6.554 (4.73), 6.677 (3.88), 6.694 (3.09), 7.734 (1.29), 7.738 (2.26), 7.742 (2.19), 8.312 (2.16), 8.317 (3.94), 8.321 (2.11).

### Example 254

### 2-[6-amino-5-(trifluoromethoxy)pyridin-3-yl]-N-[1-(1-ethyl-3,5-dimethyl-1H-pyrazol-4-yl)ethyl]-6,7-dihydrospiro[pyrazolo[5,1-c][1,4]oxazine-4,3'-pyrrolidine]-1'-carboxamide (mixture of stereoisomers)

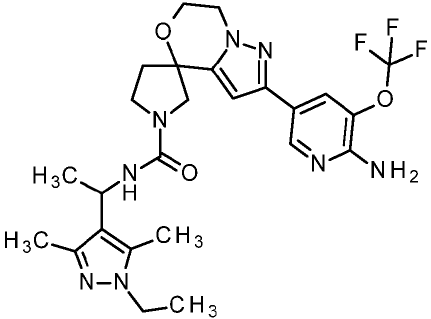

Example 254 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(1-ethyl-3,5-dimethyl-1H-pyrazol-4-yl)ethan-1-amine (42.4 mg, 253 µmol).
LC-MS (method 1): Rt = 0.99 min; MS (ESlpos): m/z = 549 [M+H]⁺
¹H-NMR (400 MHz, DMSO-d6) δ [ppm]: 1.209 (4.01), 1.227 (8.63), 1.244 (4.19), 1.331 (3.79), 1.337 (4.16), 1.349 (3.94), 1.355 (3.96), 2.143 (16.00), 2.179 (0.73), 2.186 (0.89), 2.207 (15.83), 2.238 (0.47), 2.291 (0.69), 2.306 (0.83), 2.323 (0.65), 2.327 (0.62), 2.337 (0.47), 2.523 (0.60), 2.540 (0.91), 3.317 (0.62), 3.326 (0.87), 3.421 (2.24), 3.438 (1.28), 3.468 (1.08), 3.509 (0.46), 3.532 (0.72), 3.559 (0.54), 3.584 (0.69), 3.731 (0.72), 3.760 (0.60), 3.798 (0.75), 3.827 (0.66), 3.874 (1.16), 3.892 (3.55), 3.910 (3.48), 3.928 (1.11), 4.043 (0.61), 4.054 (0.80), 4.060 (0.89), 4.078 (0.91), 4.091 (1.22), 4.100 (1.42), 4.119 (3.56), 4.700 (0.67), 4.706 (0.74), 4.717 (1.00), 4.723 (1.05), 4.735 (0.70), 4.741 (0.69), 6.090 (0.84), 6.107 (0.83), 6.139 (0.88), 6.156 (0.85), 6.576 (1.44), 6.681 (3.96), 6.698 (3.09), 7.740 (1.42), 7.744 (2.47), 7.747 (2.47), 8.311 (2.27), 8.316 (4.41), 8.321 (2.50).

### Example 255

### 2-[6-amino-5-(trifluoromethoxy)pyridin-3-yl]-N-[(1R)-1-(2-cyanophenyl)ethyl]-6,7-dihydrospiro[pyrazolo[5,1-c][1,4]oxazine-4,3'-pyrrolidine]-1'-carboxamide (mixture of stereoisomers)

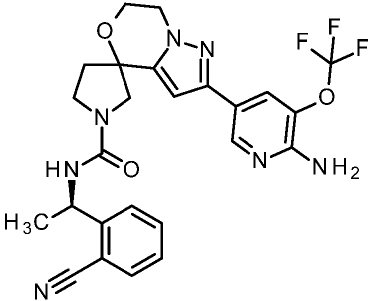

Example 255 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-[(1R)-1-aminoethyl]benzonitrile (37.0 mg, 253 µmol).
LC-MS (method 1): Rt = 1.04 min; MS (ESlpos): m/z = 528 [M+H]⁺
¹H-NMR (400 MHz, DMSO-d6) δ [ppm]: 1.067 (0.89), 1.170 (0.74), 1.185 (0.75), 1.199 (0.85), 1.215 (1.08), 1.227 (1.64), 1.392 (15.91), 1.410 (16.00), 2.197 (0.49), 2.221 (1.48), 2.247 (1.75), 2.272 (0.80), 2.332 (1.84), 2.346 (1.89), 2.518 (1.04), 2.523 (0.73), 2.539 (0.85), 2.996 (0.44), 3.167 (0.98), 3.392 (1.48), 3.410 (1.70), 3.419 (1.79), 3.436 (1.60), 3.462 (1.66), 3.492 (1.53), 3.502 (1.73), 3.532 (1.70), 3.565 (2.19), 3.587 (1.48), 3.613 (0.77), 3.626 (0.99), 3.648 (1.65), 3.672 (0.76), 3.779 (1.88), 3.807 (1.64), 3.826 (2.09), 3.856 (1.75), 4.044 (0.72), 4.058 (1.54), 4.074 (2.46), 4.093 (2.91), 4.105 (3.70), 4.112 (4.33), 4.124 (5.97), 4.131 (7.71), 4.136 (9.05), 5.021 (0.66), 5.039 (2.91), 5.057 (4.59), 5.074 (3.01), 5.092 (0.67), 6.559 (12.87), 6.711 (12.86), 6.767 (2.48), 6.784 (2.67), 6.793 (2.61), 6.810 (2.24), 7.379 (1.76), 7.383 (3.11), 7.386 (1.94), 7.398 (3.38), 7.402 (5.83), 7.405 (3.50), 7.416 (2.21), 7.420 (3.63), 7.424 (2.21), 7.621 (2.07), 7.629 (2.15), 7.638 (4.06), 7.646 (4.11), 7.663 (2.39), 7.667 (2.59), 7.673 (2.64), 7.676 (2.88), 7.685 (2.87), 7.691 (2.76), 7.694 (2.96), 7.701 (1.18), 7.705 (1.18), 7.711 (1.12), 7.714 (1.16), 7.744 (11.31), 7.754 (5.39), 7.758 (3.71), 7.762 (6.79), 8.319 (6.49), 8.324 (6.54), 8.329 (7.65), 8.334 (6.98).

### Example 256

### (rac)-2'-[6-amino-5-(difluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide

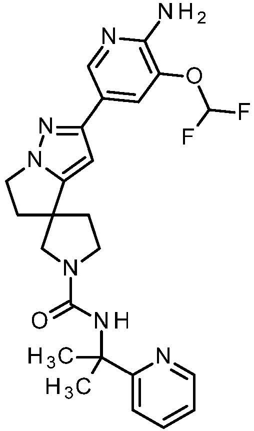

Example 256 was prepared in analogy to Example 31 using (rac)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and 2-(pyridin-2-yl)propan-2-amine (53.9 mg, 396 µmol).
LC-MS (method 1): Rt = 0.95 min; MS (ESlpos): m/z = 484 [M+H]⁺
¹H-NMR (400MHz, DMSO-d₆): δ [ppm]= 1.60 (s, 6H), 2.01 - 2.16 (m, 2H), 2.53 - 2.59 (m, 2H), 3.43 - 3.50 (m, 4H), 3.54 - 3.63 (m, 1H), 4.19 (t, 2H), 6.33 (br d, 1H), 6.41 (s, 1H), 6.47 (s, 1H), 7.00 - 7.40 (m, 1H), 7.28 (br d, 1H), 7.54 (br d, 1H), 7.66 (s, 1H), 7.74 - 7.87 (m, 1H), 8.20 (d, 1H), 8.49 (d, 1H).

### Example 257

### 2-[6-amino-5-(trifluoromethoxy)pyridin-3-yl]-N-[(1R)-1-(4-fluorophenyl)ethyl]-6,7-dihydrospiro[pyrazolo[5,1-c][1,4]oxazine-4,3'-pyrrolidine]-1'-carboxamide (mixture of stereoisomers)

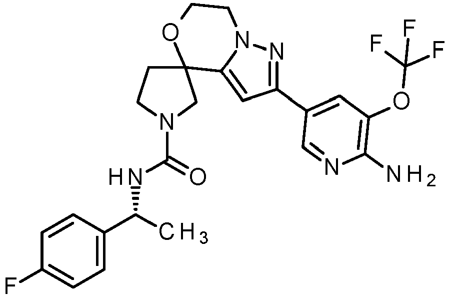

Example 257 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and (1R)-1-(4-fluorophenyl)ethan-1-amine (35.3 mg, 253 µmol).
LC-MS (method 1): Rt = 1.10 min; MS (ESlpos): m/z = 521 [M+H]⁺
¹H-NMR (400 MHz, DMSO-d6) δ [ppm]: 1.243 (0.62), 1.248 (0.41), 1.260 (0.74), 1.278 (0.52), 1.348 (3.32), 1.366 (3.26), 1.472 (0.62), 1.489 (0.64), 2.084 (16.00), 2.322 (0.61), 2.326 (0.71), 2.332 (0.64), 2.518 (1.59), 2.522 (0.99), 2.669 (0.40), 3.372 (0.60), 3.388 (0.63), 3.397 (0.79), 3.577 (0.67), 3.610 (0.57), 3.772 (0.43), 3.798 (0.74), 4.068 (0.43), 4.075 (0.42), 4.114 (0.84), 4.128 (1.89), 4.832 (0.55), 6.495 (0.50), 6.506 (0.53), 6.515 (0.54), 6.526 (0.47), 6.712 (2.82), 6.991 (0.48), 7.090 (0.75), 7.096 (0.95), 7.112 (1.75), 7.118 (2.35), 7.135 (1.08), 7.140 (0.92), 7.246 (0.48), 7.288 (0.44), 7.349 (1.14), 7.363 (1.34), 7.370 (1.20), 7.385 (0.96), 7.763 (1.13), 7.768 (1.09), 8.314 (1.37), 8.319 (2.36), 8.324 (1.37).

### Example 258

### 2-[6-amino-5-(trifluoromethoxy)pyridin-3-yl]-N-[(1R)-1-phenylpropyl]-6,7-dihydrospiro[pyrazolo[5,1-c][1,4]oxazine-4,3'-pyrrolidine]-1'-carboxamide (mixture of stereoisomers)

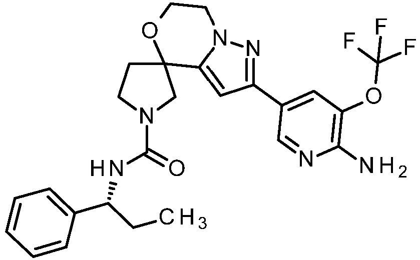

Example 258 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and (1R)-1-phenylpropan-1-amine (34.2 mg, 253 µmol).
LC-MS (method 1): Rt = 1.15 min; MS (ESlpos): m/z = 517 [M+H]⁺
¹H-NMR (400 MHz, DMSO-d6) δ [ppm]: 0.742 (1.15), 0.761 (2.66), 0.779 (1.28), 0.836 (5.54), 0.851 (12.66), 0.854 (11.95), 0.869 (6.27), 1.066 (1.85), 1.239 (1.24), 1.255 (1.13), 1.625 (0.80), 1.642 (1.46), 1.658 (2.52), 1.675 (2.76), 1.694 (2.33), 1.717 (2.22), 1.736 (1.69), 1.752 (1.21), 2.213 (1.49), 2.240 (1.99), 2.263 (1.12), 2.327 (3.67), 2.331 (3.64), 2.523 (8.62), 2.665 (1.61), 2.669 (2.18), 3.236 (0.55), 3.277 (0.74), 3.290 (1.35), 3.302 (1.25), 3.381 (2.87), 3.392 (3.09), 3.413 (1.65), 3.435 (1.01), 3.454 (1.97), 3.484 (3.87), 3.513 (2.20), 3.552 (0.87), 3.575 (1.70), 3.597 (1.55), 3.617 (1.64), 3.764 (1.94), 3.798 (2.31), 3.830 (1.55), 4.125 (8.42), 4.536 (1.01), 4.557 (2.47), 4.577 (2.37), 4.596 (1.11), 6.414 (2.68), 6.436 (2.76), 6.556 (10.52), 6.699 (7.45), 6.706 (9.77), 7.170 (1.33), 7.188 (3.58), 7.205 (2.68), 7.271 (3.03), 7.287 (7.46), 7.291 (7.47), 7.309 (16.00), 7.330 (3.90), 7.443 (5.61), 7.737 (6.17), 7.741 (5.93), 8.229 (0.55), 8.309 (5.61), 8.315 (10.71), 8.320 (6.12).

### Example 259

### 2-[6-amino-5-(trifluoromethoxy)pyridin-3-yl]-N-[(1R)-1-phenylethyl]-6,7-dihydrospiro[pyrazolo[5,1-c][1,4]oxazine-4,3'-pyrrolidine]-1'-carboxamide (mixture of stereoisomers)

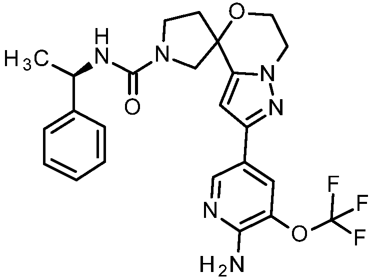

Example 259 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and (1R)-1-phenylethan-1-amine (30.7 mg, 253 µmol).
LC-MS (method 1): Rt = 1.10 min; MS (ESlpos): m/z = 503 [M+H]⁺
¹H-NMR (400 MHz, DMSO-d6) δ [ppm]: 0.760 (0.44), 1.019 (0.63), 1.036 (0.81), 1.066 (2.30), 1.225 (1.51), 1.241 (5.98), 1.257 (10.08), 1.274 (5.13), 1.358 (15.82), 1.376 (16.00), 1.479 (1.11), 1.496 (1.11), 2.084 (1.05), 2.211 (1.32), 2.237 (1.64), 2.323 (2.89), 2.327 (3.52), 2.331 (3.14), 2.518 (14.44), 2.523 (10.15), 2.665 (1.62), 2.669 (2.11), 2.673 (1.64), 2.729 (0.42), 3.122 (0.69), 3.133 (0.70), 3.141 (0.71), 3.152 (0.77), 3.397 (3.16), 3.457 (2.00), 3.472 (2.32), 3.487 (2.28), 3.501 (2.56), 3.566 (0.98), 3.590 (2.31), 3.614 (2.32), 3.636 (1.04), 3.787 (1.94), 3.800 (2.03), 3.813 (1.78), 3.828 (1.72), 4.068 (2.00), 4.103 (3.23), 4.115 (4.56), 4.129 (9.05), 4.818 (1.93), 4.837 (3.00), 4.856 (2.05), 6.489 (2.99), 6.508 (3.11), 6.567 (5.47), 6.704 (8.70), 6.708 (10.99), 6.730 (0.59), 6.969 (0.48), 7.097 (0.58), 7.173 (1.54), 7.191 (4.08), 7.208 (2.90), 7.224 (0.56), 7.273 (2.78), 7.278 (3.46), 7.293 (7.48), 7.298 (7.70), 7.311 (6.03), 7.316 (5.87), 7.329 (11.77), 7.347 (4.87), 7.445 (1.40), 7.469 (0.66), 7.745 (5.16), 7.781 (0.55), 8.222 (1.14), 8.228 (1.24), 8.315 (6.28), 8.321 (8.35), 8.326 (6.81).

### Example 260

### (rac)-2-[6-amino-5-(trifluoromethoxy)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide

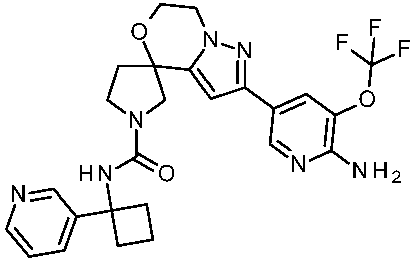

Example 260 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(pyridin-3-yl)cyclobutan-1-amine (37.5 mg, 253 µmol).
LC-MS (method 1): Rt = 0.98 min; MS (ESlpos): m/z = 530 [M+H]+
¹H-NMR (400 MHz, DMSO-d6) δ [ppm]: 0.851 (0.92), 1.035 (4.80), 1.052 (10.51), 1.065 (6.04), 1.070 (4.99), 1.114 (1.05), 1.130 (1.39), 1.170 (4.69), 1.185 (4.93), 1.199 (5.53), 1.215 (7.04), 1.231 (7.57), 1.333 (0.62), 1.788 (1.92), 1.810 (2.33), 1.828 (1.56), 2.011 (2.12), 2.034 (2.05), 2.208 (1.64), 2.234 (2.10), 2.256 (1.18), 2.327 (3.89), 2.449 (7.70), 2.471 (8.16), 2.518 (11.76), 2.523 (7.86), 2.540 (3.49), 2.669 (1.73), 2.729 (1.12), 2.888 (1.34), 3.159 (4.11), 3.172 (4.22), 3.236 (0.56), 3.256 (0.62), 3.273 (0.45), 3.280 (0.60), 3.292 (1.49), 3.319 (3.49), 3.382 (7.05), 3.405 (2.17), 3.410 (2.16), 3.417 (1.92), 3.422 (2.53), 3.429 (1.89), 3.435 (2.67), 3.440 (2.81), 3.452 (3.80), 3.481 (2.60), 3.514 (0.68), 3.557 (1.64), 3.565 (1.63), 3.578 (2.88), 3.600 (1.37), 3.751 (3.44), 3.780 (2.87), 3.946 (0.70), 4.046 (1.15), 4.061 (2.29), 4.076 (3.39), 4.111 (6.70), 4.125 (11.62), 4.353 (0.83), 4.366 (1.72), 4.379 (0.92), 4.780 (0.56), 4.860 (0.58), 4.876 (0.82), 4.891 (0.63), 6.555 (16.00), 6.706 (14.55), 6.836 (8.58), 7.305 (4.40), 7.306 (4.55), 7.319 (4.58), 7.326 (4.73), 7.336 (4.91), 7.338 (4.75), 7.735 (6.15), 7.738 (8.29), 7.776 (3.91), 7.781 (5.15), 7.787 (4.09), 7.797 (3.59), 7.802 (4.64), 7.807 (3.55), 8.311 (12.50), 8.316 (12.99), 8.373 (7.75), 8.377 (7.81), 8.384 (7.34), 8.389 (7.31), 8.634 (8.86), 8.638 (8.82), 8.883 (0.67), 9.732 (0.90).

### Example 261

### 2-[6-amino-5-(difluoromethoxy)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide (mixture of stereoisomers)

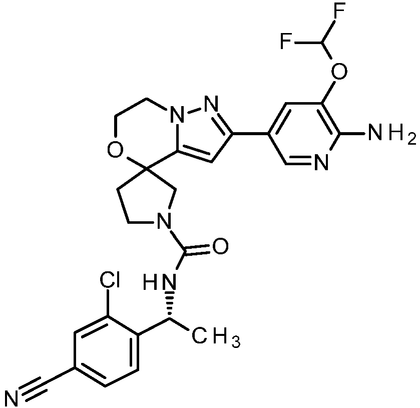

Example 261 was prepared in analogy to Example 31 using (rac)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and 4-[(1R)-1-aminoethyl]-3-chlorobenzonitrile (43.5 mg, 241 µmol).
LC-MS (method 1): Rt = 1.03 min; MS (ESlpos): m/z = 544 [M+H]⁺
¹H-NMR (400 MHz, DMSO-d6) δ [ppm]: 1.232 (1.01), 1.256 (0.54), 1.329 (6.29), 1.346 (6.22), 2.084 (0.41), 2.231 (0.59), 2.258 (0.76), 2.518 (16.00), 2.522 (11.23), 3.381 (0.53), 3.398 (0.61), 3.424 (0.61), 3.446 (0.76), 3.474 (0.58), 3.511 (0.50), 3.542 (0.69), 3.573 (0.64), 3.659 (0.64), 3.768 (0.74), 3.795 (0.66), 3.843 (0.82), 3.872 (0.73), 4.060 (0.67), 4.075 (1.00), 4.092 (1.22), 4.129 (3.69), 5.116 (0.70), 5.125 (0.81), 5.135 (1.15), 5.143 (1.23), 5.153 (0.80), 5.161 (0.79), 5.759 (7.07), 6.221 (5.45), 6.675 (3.28), 6.682 (3.53), 6.781 (0.92), 6.799 (0.91), 6.820 (0.89), 6.839 (0.82), 6.993 (1.29), 6.997 (1.36), 7.177 (2.74), 7.182 (2.83), 7.361 (1.20), 7.366 (1.18), 7.618 (2.05), 7.664 (1.83), 7.677 (1.86), 7.684 (2.26), 7.697 (2.13), 7.825 (1.37), 7.829 (1.46), 7.834 (1.59), 7.838 (1.61), 7.845 (1.20), 7.849 (1.21), 7.855 (1.20), 7.859 (1.21), 7.992 (4.47), 7.996 (4.22), 8.190 (2.76), 8.195 (2.72), 8.201 (3.00), 8.206 (3.02).

### Example 262

### (rac)-2-[6-amino-5-(difluoromethoxy)pyridin-3-yl]-N-[2-(4-fluorophenyl)propan-2-yl]-6,7-dihydrospiro[pyrazolo[5,1-c][1,4]oxazine-4,3'-pyrrolidine]-1'-carboxamide

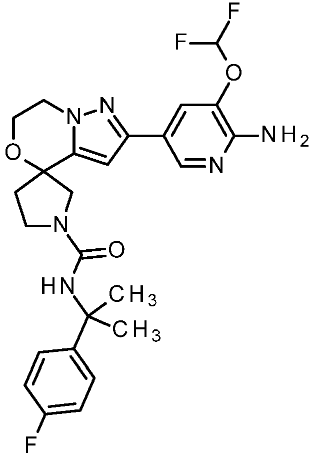

Example 262 was prepared in analogy to Example 31 using (rac)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and 2-(4-fluorophenyl)propan-2-amine (36.9 mg, 241 µmol).
LC-MS (method 1): Rt = 1.09 min; MS (ESlpos): m/z = 517 [M+H]⁺
¹H-NMR (400 MHz, DMSO-d6) δ [ppm]: 1.066 (1.28), 1.232 (0.59), 1.550 (16.00), 1.556 (15.55), 2.074 (3.12), 2.084 (1.43), 2.215 (0.74), 2.240 (1.07), 2.265 (0.56), 2.306 (0.93), 2.323 (2.20), 2.327 (2.31), 2.331 (1.76), 2.337 (1.21), 2.352 (0.56), 2.518 (11.08), 2.523 (8.12), 2.665 (1.18), 2.669 (1.66), 2.673 (1.16), 2.728 (0.54), 2.889 (0.66), 3.381 (1.69), 3.398 (1.87), 3.424 (1.63), 3.499 (2.36), 3.571 (1.15), 3.592 (1.65), 3.615 (0.87), 3.778 (1.68), 3.807 (1.44), 4.067 (0.52), 4.081 (1.08), 4.099 (1.63), 4.124 (4.85), 4.134 (4.44), 6.099 (4.87), 6.385 (0.81), 6.689 (9.59), 7.015 (2.45), 7.049 (3.58), 7.054 (1.21), 7.071 (7.73), 7.088 (1.35), 7.093 (4.05), 7.101 (0.48), 7.198 (5.06), 7.352 (3.77), 7.358 (1.71), 7.366 (4.23), 7.374 (3.93), 7.383 (3.62), 7.388 (3.59), 7.653 (3.50), 8.192 (6.26), 8.197 (6.56).

### Example 263

### (rac)-2-[6-amino-5-(difluoromethoxy)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide

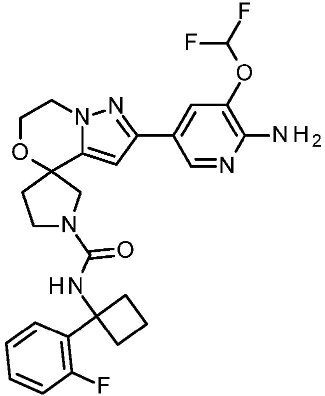

Example 263 was prepared in analogy to Example 31 using (rac)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and 1-(2-fluorophenyl)cyclobutan-1-amine-hydrochloride salt (48.6 mg, 241 µmol).
LC-MS (method 1): Rt = 1.11 min; MS (ESlpos): m/z = 529 [M+H]⁺
¹H-NMR (400 MHz, DMSO-d6) δ [ppm]: 1.232 (0.48), 1.728 (0.42), 1.734 (0.43), 1.741 (0.45), 1.755 (0.47), 2.033 (0.53), 2.055 (0.51), 2.060 (0.50), 2.085 (16.00), 2.218 (0.48), 2.285 (0.45), 2.300 (0.54), 2.318 (0.76), 2.323 (1.22), 2.327 (1.61), 2.332 (1.26), 2.336 (0.65), 2.518 (6.63), 2.523 (5.43), 2.660 (0.46), 2.665 (1.03), 2.669 (1.45), 2.673 (1.01), 2.678 (0.43), 3.416 (0.67), 3.445 (0.79), 3.513 (0.43), 3.534 (0.78), 3.727 (0.87), 3.755 (0.75), 4.039 (0.52), 4.055 (0.79), 4.074 (0.79), 4.087 (0.90), 4.095 (1.16), 4.112 (2.54), 6.213 (3.75), 6.592 (2.33), 6.644 (4.04), 6.990 (1.59), 7.043 (0.63), 7.046 (0.72), 7.063 (0.85), 7.066 (0.98), 7.074 (1.75), 7.093 (3.00), 7.111 (1.24), 7.114 (0.94), 7.174 (3.36), 7.198 (0.45), 7.202 (0.51), 7.211 (0.52), 7.216 (0.76), 7.222 (0.64), 7.235 (0.70), 7.359 (1.38), 7.404 (0.68), 7.409 (0.69), 7.428 (1.08), 7.445 (0.61), 7.601 (2.05), 8.174 (3.24), 8.178 (3.35).

### Example 264

### (rac)-2'-[6-amino-5-(difluoromethoxy)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide

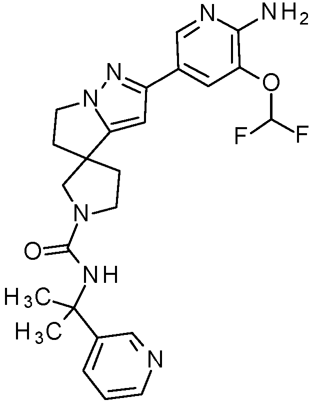

Example 264 was prepared in analogy to Example 31 using (rac)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and 2-(pyridin-3-yl)propan-2-amine (53.9 mg, 396 µmol).
LC-MS (method 1): Rt = 0.89 min; MS (ESlpos): m/z = 484 [M+H]⁺
¹H-NMR (400MHz, DMSO-d₆): δ [ppm]= 1.61 (s, 6H), 1.99 - 2.15 (m, 2H), 2.52 - 2.57 (m, 2H), 3.40 - 3.49 (m, 4H), 3.51 - 3.60 (m, 1H), 4.19 (t, 2H), 6.31 - 6.42 (m, 2H), 6.42 - 6.56 (m, 1H), 7.01 - 7.43 (m, 1H), 7.63 - 7.71 (m, 2H), 8.15 - 8.23 (m, 2H), 8.55 - 8.60 (m, 1H), 8.72 (d, 1H).

### Example 265

### 2'-[6-amino-5-(difluoromethoxy)pyridin-3-yl]-N-[1-(2-chlorophenyl)cyclobutyl]-5',6'-dihydrospiro[azetidine-3,4'-pyrrolo[1,2-b]pyrazole]-1-carboxamide

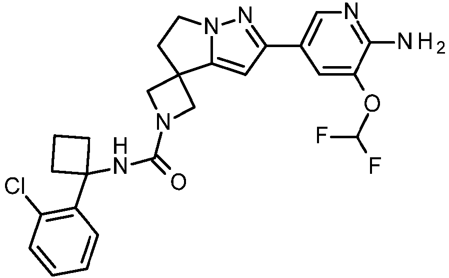

Example 265 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 1-(2-chlorophenyl)cyclobutan-1-amine-hydrochloride salt (63.9 mg, 293 µmol).
LC-MS (method 1): Rt = 1.17 min; MS (ESlpos): m/z = 515 [M+H]⁺
¹H-NMR (400 MHz, DMSO-d6) δ [ppm]: 1.208 (2.07), 1.216 (0.50), 1.224 (2.19), 1.241 (0.90), 1.248 (1.61), 1.259 (2.04), 1.265 (1.95), 1.273 (1.80), 1.277 (1.37), 1.289 (1.50), 1.661 (0.40), 1.672 (0.69), 1.683 (1.03), 1.694 (1.00), 1.698 (1.04), 1.710 (1.16), 1.720 (0.85), 1.731 (0.49), 2.030 (0.83), 2.051 (1.51), 2.073 (1.45), 2.077 (1.47), 2.099 (0.88), 2.322 (0.43), 2.327 (0.62), 2.332 (0.48), 2.470 (1.19), 2.518 (6.02), 2.522 (5.30), 2.543 (1.44), 2.596 (1.66), 2.606 (1.95), 2.618 (2.22), 2.628 (2.83), 2.649 (1.18), 2.660 (1.12), 2.669 (0.81), 2.673 (0.56), 2.727 (0.54), 2.774 (3.23), 2.791 (5.24), 2.808 (3.38), 2.887 (0.57), 3.928 (1.70), 3.951 (16.00), 3.972 (1.67), 4.019 (0.56), 4.074 (3.37), 4.092 (5.42), 4.109 (3.32), 6.160 (8.39), 6.537 (15.50), 6.543 (2.13), 6.994 (3.62), 7.017 (7.32), 7.046 (0.41), 7.179 (7.56), 7.193 (1.42), 7.197 (1.46), 7.211 (3.39), 7.216 (3.29), 7.230 (3.19), 7.235 (3.22), 7.241 (2.63), 7.245 (3.15), 7.260 (3.41), 7.264 (3.86), 7.278 (1.71), 7.282 (1.48), 7.317 (4.96), 7.321 (4.86), 7.336 (3.21), 7.340 (3.24), 7.363 (3.06), 7.453 (0.62), 7.462 (0.43), 7.524 (3.39), 7.528 (3.29), 7.543 (3.10), 7.547 (2.84), 7.629 (5.09), 7.631 (4.98), 8.214 (7.65), 8.219 (7.93).

### Example 266

### 2'-[6-amino-5-(trifluoromethoxy)pyridin-3-yl]-N-[(1R)-1-(4-cyanophenyl)ethyl]-5',6'-dihydrospiro[azetidine-3,4'-pyrrolo[1,2-b]pyrazole]-1-carboxamide

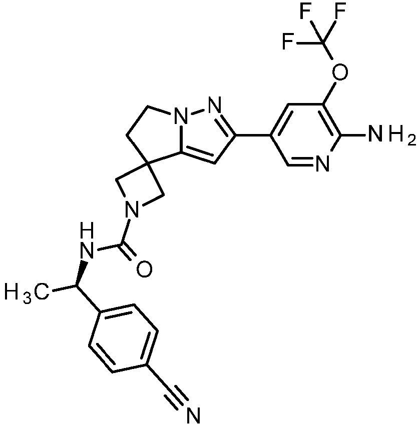

Example 266 was prepared in analogy to Example 31 using 5-(5',6'-dihydrospiro[azetidine-3,4'-pyrrolo[1,2-b]pyrazol]-2'-yl)-3-(trifluoromethoxy)pyridin-2-amine-hydrochloride salt (75.0 mg, 207 µmol) and 4-[(1R)-1-aminoethyl]benzonitrile (36.4 mg, 249 µmol).
LC-MS (method 1): Rt = 1.03 min; MS (ESlpos): m/z = 498 [M+H]⁺
¹H-NMR (400 MHz, DMSO-d6) δ [ppm]: 1.035 (1.35), 1.052 (3.02), 1.070 (1.39), 1.224 (0.64), 1.354 (10.97), 1.371 (11.03), 1.824 (0.92), 2.518 (0.87), 2.523 (0.60), 2.827 (2.97), 2.845 (4.93), 2.862 (3.19), 2.884 (0.45), 3.167 (11.97), 3.430 (1.13), 3.447 (0.88), 3.464 (0.42), 3.563 (0.51), 4.003 (2.66), 4.029 (10.49), 4.050 (5.45), 4.071 (2.53), 4.104 (3.44), 4.122 (5.37), 4.139 (3.30), 4.835 (1.61), 4.853 (2.32), 4.872 (1.58), 6.504 (8.86), 6.675 (16.00), 7.018 (3.65), 7.038 (3.53), 7.522 (7.74), 7.542 (9.03), 7.770 (3.53), 7.775 (5.01), 7.779 (4.00), 7.785 (11.00), 7.790 (3.32), 7.802 (2.97), 7.806 (8.61), 8.366 (7.98), 8.371 (7.64).

### Example 267

### 2'-[6-amino-5-(difluoromethoxy)pyridin-3-yl]-N-[(1R)-1-(1,3,4-trimethyl-1H-pyrazol-5-yl)ethyl]-5',6'-dihydrospiro[azetidine-3,4'-pyrrolo[1,2-b]pyrazole]-1-carboxamide

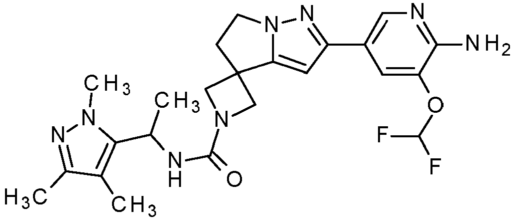

Example 267 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-(1,3,4-trimethyl-1H-pyrazol-5-yl)ethan-1-amine (40.1 mg, 262 µmol).
LC-MS (method 1): Rt = 0.89 min; MS (ESlpos): m/z = 487 [M+H]⁺
¹H-NMR (400 MHz, DMSO-d6) δ [ppm]: 1.066 (8.53), 1.156 (2.86), 1.387 (5.86), 1.406 (5.87), 1.795 (0.42), 1.926 (0.44), 1.957 (15.99), 1.969 (0.73), 1.986 (14.44), 2.518 (2.28), 2.523 (1.51), 2.807 (1.46), 2.824 (2.48), 2.842 (1.56), 3.682 (0.42), 3.713 (16.00), 3.939 (1.35), 3.951 (1.47), 3.972 (2.74), 3.989 (2.69), 4.003 (2.88), 4.012 (2.45), 4.024 (1.12), 4.033 (1.37), 4.091 (1.63), 4.109 (2.62), 4.125 (1.55), 4.860 (1.00), 4.878 (1.44), 4.896 (1.00), 6.162 (4.33), 6.596 (7.27), 6.895 (1.79), 6.911 (1.70), 6.995 (1.59), 7.180 (3.32), 7.364 (1.45), 7.634 (2.45), 8.226 (3.71), 8.231 (3.70).

### Example 268

### (rac)-2'-[6-amino-5-(trifluoromethoxy)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide

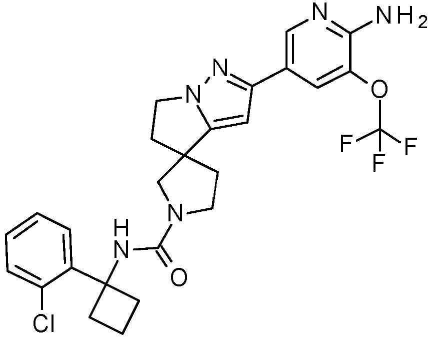

Example 268 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine-hydrochloride salt (75.0 mg, 200 µmol) and 1-(2-chlorophenyl)cyclobutan-1-amine (43.5 mg, 239 µmol).
LC-MS (method 1): Rt = 1.26 min; MS (ESlpos): m/z = 547 [M+H]⁺
¹H-NMR (400 MHz, DMSO-d6) δ [ppm]: 0.917 (0.99), 0.932 (8.27), 0.934 (2.71), 0.948 (8.54), 0.952 (1.44), 1.232 (0.95), 1.649 (0.70), 1.660 (1.13), 1.671 (1.70), 1.686 (1.66), 1.698 (1.78), 1.708 (1.32), 1.720 (0.81), 1.998 (2.15), 2.012 (3.86), 2.028 (5.66), 2.048 (3.74), 2.074 (2.65), 2.084 (0.92), 2.096 (1.40), 2.116 (0.42), 2.322 (1.09), 2.327 (1.57), 2.332 (1.11), 2.404 (0.82), 2.423 (0.93), 2.459 (3.22), 2.476 (8.12), 2.518 (7.91), 2.523 (6.64), 2.551 (2.81), 2.572 (1.29), 2.597 (1.70), 2.608 (1.99), 2.620 (3.02), 2.628 (2.76), 2.649 (2.45), 2.660 (2.67), 2.664 (2.15), 2.669 (2.95), 2.673 (1.93), 2.679 (1.44), 2.941 (0.47), 2.957 (0.65), 2.974 (0.49), 3.345 (3.18), 3.347 (2.93), 3.374 (6.12), 3.392 (7.16), 3.418 (2.24), 3.445 (1.34), 3.464 (2.33), 3.478 (1.67), 3.488 (1.51), 3.504 (0.81), 4.137 (4.88), 4.155 (9.90), 4.173 (4.72), 6.295 (9.98), 6.511 (16.00), 6.521 (11.11), 7.160 (2.08), 7.164 (2.38), 7.178 (5.52), 7.183 (5.56), 7.197 (5.27), 7.202 (4.98), 7.226 (3.93), 7.230 (5.11), 7.245 (5.50), 7.249 (6.72), 7.263 (2.99), 7.267 (2.81), 7.280 (8.52), 7.283 (7.26), 7.298 (5.82), 7.302 (5.30), 7.528 (5.50), 7.532 (5.61), 7.547 (5.12), 7.552 (4.85), 7.711 (5.47), 7.715 (7.61), 7.719 (5.22), 8.281 (12.38), 8.286 (11.77).

### Example 269

### 2'-[6-amino-5-(difluoromethoxy)pyridin-3-yl]-N-[(1R)-1-(5-fluoropyridin-3-yl)ethyl]-5',6'-dihydrospiro[azetidine-3,4'-pyrrolo[1,2-b]pyrazole]-1-carboxamide

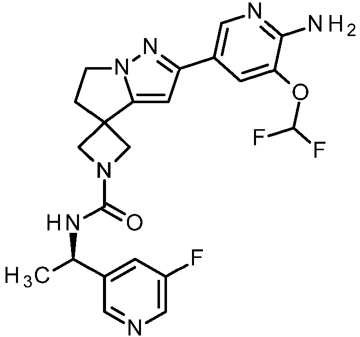

Example 269 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and (1R)-1-(5-fluoropyridin-3-yl)ethan-1-amine (41.0 mg, 293 µmol).
LC-MS (method 1): Rt = 0.87 min; MS (ESlpos): m/z = 474 [M+H]⁺
¹H-NMR (400 MHz, DMSO-d6) δ [ppm]: 0.798 (0.54), 0.815 (0.58), 0.822 (0.58), 0.905 (0.67), 1.035 (8.33), 1.053 (16.00), 1.070 (8.13), 1.390 (3.11), 1.408 (3.12), 2.518 (1.89), 2.523 (1.22), 2.829 (0.78), 2.846 (1.27), 2.864 (0.85), 3.404 (1.05), 3.418 (1.13), 3.422 (3.58), 3.435 (3.66), 3.440 (3.33), 3.452 (3.39), 3.457 (1.19), 3.469 (1.13), 4.002 (0.74), 4.022 (1.40), 4.035 (3.20), 4.058 (1.30), 4.078 (0.75), 4.100 (0.91), 4.117 (1.44), 4.135 (0.86), 4.343 (2.34), 4.356 (4.53), 4.369 (2.18), 4.876 (0.40), 4.894 (0.58), 4.914 (0.41), 5.759 (0.90), 6.164 (2.32), 6.630 (4.50), 6.974 (0.97), 6.996 (1.50), 7.181 (2.06), 7.365 (0.84), 7.636 (1.42), 7.641 (1.31), 7.656 (0.44), 7.662 (0.59), 7.667 (0.47), 7.682 (0.43), 7.687 (0.57), 7.693 (0.45), 8.230 (2.16), 8.234 (2.23), 8.439 (1.73), 8.446 (2.34), 8.451 (1.70), 8.456 (0.97).

### Example 270

### 2'-[6-amino-5-(trifluoromethoxy)pyridin-3-yl]-N-[(1R)-1-(4-fluorophenyl)ethyl]-5',6'-dihydrospiro[azetidine-3,4'-pyrrolo[1,2-b]pyrazole]-1-carboxamide

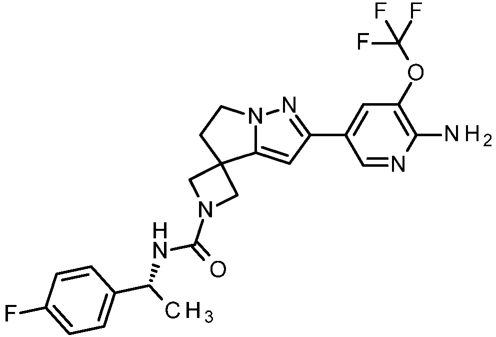

Example 270 was prepared in analogy to Example 31 using 5-(5',6'-dihydrospiro[azetidine-3,4'-pyrrolo[1,2-b]pyrazol]-2'-yl)-3-(trifluoromethoxy)pyridin-2-amine-hydrochloride salt (75.0 mg, 207 µmol) and (1R)-1-(4-fluorophenyl)ethan-1-amine (34.6 mg, 249 µmol).
LC-MS (method 1): Rt = 1.10 min; MS (ESlpos): m/z = 491 [M+H]⁺
¹H-NMR (400 MHz, DMSO-d6) δ [ppm]: 0.833 (0.44), 0.850 (0.58), 1.153 (0.85), 1.171 (1.61), 1.189 (0.88), 1.231 (3.15), 1.339 (11.89), 1.357 (11.97), 1.986 (3.01), 2.083 (0.97), 2.518 (11.96), 2.523 (8.33), 2.819 (3.10), 2.836 (5.17), 2.853 (3.43), 3.159 (0.78), 3.171 (0.93), 3.295 (0.73), 3.300 (0.86), 3.306 (0.99), 3.310 (0.82), 3.329 (3.13), 3.391 (2.84), 3.401 (1.52), 3.413 (1.29), 3.421 (0.92), 3.433 (0.62), 3.443 (0.57), 3.458 (0.42), 3.985 (2.38), 4.008 (14.82), 4.023 (6.04), 4.034 (1.32), 4.044 (2.31), 4.100 (3.40), 4.118 (5.45), 4.134 (3.36), 4.784 (1.53), 4.802 (2.12), 4.821 (1.54), 6.502 (9.14), 6.664 (16.00), 6.884 (3.81), 6.905 (3.63), 7.112 (4.71), 7.117 (1.64), 7.129 (2.03), 7.134 (9.60), 7.140 (2.08), 7.151 (1.81), 7.157 (5.43), 7.164 (0.71), 7.348 (4.63), 7.353 (2.01), 7.362 (5.19), 7.369 (4.55), 7.377 (1.78), 7.383 (3.93), 7.764 (3.60), 7.769 (4.87), 7.773 (3.43), 8.359 (8.11), 8.363 (7.84).

### Example 271

### 2'-[6-amino-5-(trifluoromethoxy)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide (mixture of stereoisomers)

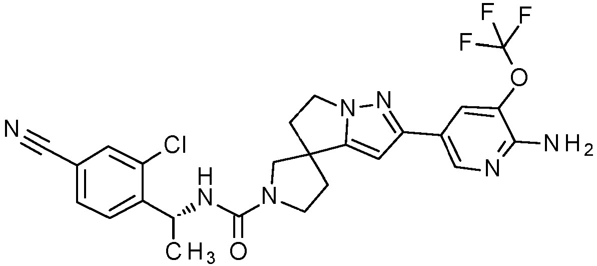

Example 271 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 4-[(1R)-1-aminoethyl]-3-chlorobenzonitrile (47.9 mg, 265 µmol).
LC-MS (method 1): Rt = 1.11 min; MS (ESlpos): m/z = 546 [M+H]⁺
¹H-NMR (400 MHz, DMSO-d6) δ [ppm]: 1.066 (0.59), 1.323 (8.06), 1.330 (8.78), 1.341 (8.65), 1.347 (8.71), 1.465 (2.43), 2.069 (3.06), 2.073 (3.09), 2.081 (3.35), 2.523 (3.91), 2.532 (6.25), 2.550 (3.71), 2.729 (0.52), 2.886 (0.59), 3.403 (0.58), 3.427 (2.72), 3.452 (4.57), 3.492 (3.77), 3.509 (2.16), 3.516 (2.11), 3.542 (0.94), 3.571 (0.60), 3.590 (0.85), 4.165 (2.67), 4.171 (3.00), 4.184 (4.85), 4.188 (5.35), 4.200 (3.03), 4.205 (2.64), 5.111 (0.49), 5.128 (2.15), 5.146 (3.38), 5.164 (2.24), 5.181 (0.54), 6.425 (16.00), 6.505 (11.92), 6.775 (1.98), 6.792 (3.37), 6.809 (1.96), 7.647 (2.69), 7.667 (3.47), 7.703 (2.68), 7.723 (3.72), 7.757 (5.84), 7.779 (2.32), 7.782 (2.30), 7.799 (1.76), 7.803 (1.92), 7.808 (3.05), 7.812 (2.96), 7.828 (1.92), 7.832 (2.03), 7.979 (5.78), 7.983 (8.82), 7.988 (5.23), 8.334 (8.94), 8.339 (8.59).

### Example 272

### 2'-[6-amino-5-(difluoromethoxy)pyridin-3-yl]-N-[(1R)-1-(2-cyanophenyl)ethyl]-5',6'-dihydrospiro[azetidine-3,4'-pyrrolo[1,2-b]pyrazole]-1-carboxamide

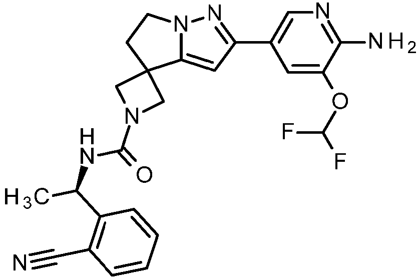

Example 272 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-[(1R)-1-aminoethyl]benzonitrile (42.8 mg, 293 µmol).
LC-MS (method 1): Rt = 0.96 min; MS (ESlpos): m/z = 480 [M+H]⁺
¹H-NMR (400 MHz, DMSO-d6) δ [ppm]: 1.014 (0.47), 1.031 (0.96), 1.047 (0.58), 1.050 (0.57), 1.325 (0.50), 1.342 (0.53), 1.383 (11.62), 1.401 (11.64), 2.074 (1.88), 2.518 (3.41), 2.523 (2.17), 2.827 (2.96), 2.845 (4.89), 2.862 (3.19), 3.361 (0.41), 3.810 (0.42), 4.014 (2.75), 4.036 (8.12), 4.042 (7.29), 4.062 (5.72), 4.083 (2.53), 4.102 (3.40), 4.120 (5.31), 4.137 (3.26), 4.992 (0.41), 5.010 (1.87), 5.028 (2.88), 5.045 (1.86), 5.063 (0.42), 5.768 (1.53), 6.147 (1.02), 6.167 (8.94), 6.424 (0.56), 6.618 (16.00), 6.998 (3.71), 7.165 (3.69), 7.183 (11.18), 7.200 (0.74), 7.367 (3.26), 7.385 (0.51), 7.405 (2.00), 7.408 (2.01), 7.424 (3.86), 7.426 (4.00), 7.442 (2.39), 7.445 (2.44), 7.556 (0.41), 7.609 (2.89), 7.629 (4.57), 7.639 (5.13), 7.641 (5.06), 7.698 (2.24), 7.701 (2.52), 7.717 (3.04), 7.720 (3.36), 7.736 (1.38), 7.739 (1.50), 7.763 (3.94), 7.765 (3.78), 7.782 (3.55), 7.785 (3.16), 8.079 (0.60), 8.084 (0.62), 8.231 (8.24), 8.235 (8.56).

### Example 273

### 2'-[6-amino-5-(difluoromethoxy)pyridin-3-yl]-N-[2-(pyridin-4-yl)propan-2-yl]-5',6'-dihydrospiro[azetidine-3,4'-pyrrolo[1,2-b]pyrazole]-1-carboxamide

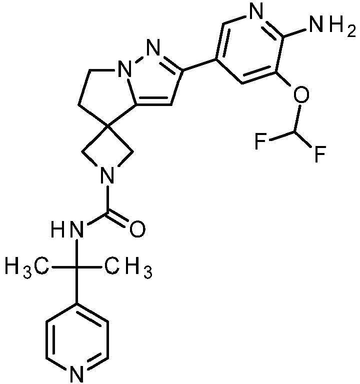

Example 273 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-(pyridin-4-yl)propan-2-amine (39.9 mg, 293 µmol).
LC-MS (method 1): Rt = 0.87 min; MS (ESlpos): m/z = 470 [M+H]⁺
¹H-NMR (400 MHz, DMSO-d6) δ [ppm]: 1.230 (0.40), 1.531 (16.00), 1.871 (0.57), 2.518 (0.81), 2.522 (0.52), 2.814 (0.65), 2.829 (1.26), 2.846 (2.08), 2.863 (1.35), 3.166 (1.55), 3.910 (0.56), 4.005 (0.66), 4.027 (6.19), 4.049 (0.73), 4.106 (1.46), 4.124 (2.34), 4.141 (1.44), 6.150 (0.47), 6.175 (3.61), 6.440 (0.66), 6.631 (6.54), 6.713 (2.96), 7.008 (1.48), 7.193 (3.10), 7.320 (3.75), 7.324 (2.40), 7.331 (2.45), 7.336 (3.79), 7.377 (1.38), 7.655 (2.01), 7.657 (1.98), 8.253 (3.33), 8.258 (3.24), 8.463 (4.01), 8.468 (2.32), 8.475 (2.36), 8.479 (3.70).

### Example 274

### 2'-[6-amino-5-(difluoromethoxy)pyridin-3-yl]-N-[(1R)-1-(4-fluorophenyl)ethyl]-5',6'-dihydrospiro[azetidine-3,4'-pyrrolo[1,2-b]pyrazole]-1-carboxamide

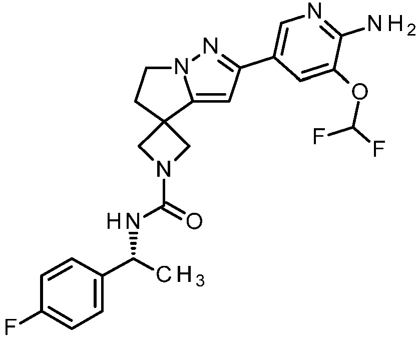

Example 274 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and (1R)-1-(4-fluorophenyl)ethan-1-amine (40.8 mg, 293 µmol).
LC-MS (method 1): Rt = 102.00 min; MS (ESlpos): m/z = 473 [M+H]⁺
¹H-NMR (400 MHz, DMSO-d6) δ [ppm]: 1.233 (0.50), 1.341 (11.77), 1.358 (11.82), 1.463 (0.44), 1.480 (0.41), 2.518 (7.97), 2.523 (4.73), 2.819 (3.16), 2.837 (5.23), 2.854 (3.39), 3.987 (2.42), 4.010 (15.90), 4.024 (5.90), 4.045 (2.23), 4.097 (3.56), 4.115 (5.61), 4.132 (3.38), 4.786 (1.60), 4.805 (2.13), 4.823 (1.52), 6.162 (9.18), 6.622 (16.00), 6.876 (3.82), 6.897 (3.67), 6.995 (3.46), 7.114 (4.68), 7.119 (1.60), 7.130 (2.00), 7.136 (9.47), 7.142 (1.96), 7.153 (1.78), 7.159 (5.41), 7.180 (7.08), 7.349 (4.66), 7.354 (2.16), 7.364 (7.47), 7.370 (4.78), 7.380 (1.74), 7.384 (3.81), 7.639 (5.15), 8.231 (8.28), 8.235 (7.89).

### Example 275

### 2'-[6-amino-5-(difluoromethoxy)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide

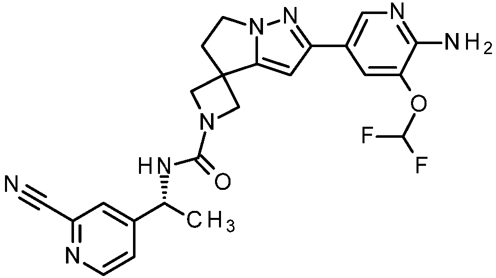

Example 275 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 4-[(1R)-1-aminoethyl]pyridine-2-carbonitrile (43.1 mg, 293 µmol).
LC-MS (method 1): Rt = 0.88 min; MS (ESlpos): m/z = 481 [M+H]⁺
¹H-NMR (400 MHz, DMSO-d6) δ [ppm]: 1.044 (0.55), 1.367 (11.57), 1.385 (11.84), 1.402 (0.43), 2.073 (0.67), 2.518 (0.93), 2.523 (0.57), 2.841 (2.94), 2.858 (4.78), 2.876 (3.17), 4.022 (2.90), 4.033 (1.72), 4.043 (4.83), 4.053 (5.82), 4.060 (5.90), 4.081 (1.86), 4.089 (4.35), 4.109 (4.74), 4.124 (5.53), 4.142 (3.24), 4.833 (1.64), 4.851 (2.44), 4.870 (1.63), 6.148 (1.05), 6.170 (8.70), 6.630 (0.48), 6.641 (16.00), 6.999 (3.64), 7.052 (3.62), 7.071 (3.48), 7.183 (7.57), 7.202 (0.70), 7.367 (3.21), 7.559 (0.40), 7.642 (4.77), 7.644 (4.89), 7.646 (4.79), 7.684 (2.96), 7.687 (3.07), 7.697 (3.14), 7.700 (3.08), 8.000 (5.53), 8.002 (5.51), 8.004 (5.46), 8.080 (0.67), 8.085 (0.64), 8.238 (8.33), 8.243 (8.12), 8.694 (5.24), 8.707 (4.90).

### Example 276

### (rac)-2'-[6-amino-5-(trifluoromethoxy)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide

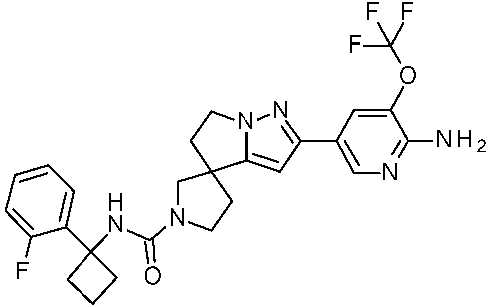

Example 276 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 1-(2-fluorophenyl)cyclobutan-1-amine-hydrochloride salt (53.5 mg, 265 µmol).
LC-MS (method 1): Rt = 1.20 min; MS (ESlpos): m/z = 531 [M+H]⁺
¹H-NMR (400 MHz, DMSO-d6) δ [ppm]: 0.847 (0.56), 1.033 (0.92), 1.051 (1.69), 1.064 (0.50), 1.068 (1.06), 1.227 (3.09), 1.701 (0.59), 1.714 (0.82), 1.723 (1.27), 1.729 (1.32), 1.736 (1.39), 1.743 (1.27), 1.750 (1.48), 1.764 (1.01), 2.003 (2.63), 2.022 (3.69), 2.031 (4.75), 2.050 (3.10), 2.064 (1.42), 2.091 (0.46), 2.331 (1.04), 2.459 (0.97), 2.518 (16.00), 2.522 (12.70), 3.271 (0.54), 3.298 (0.48), 3.305 (1.54), 3.313 (1.41), 3.323 (0.93), 3.331 (1.58), 3.338 (1.04), 3.345 (1.80), 3.352 (2.47), 3.361 (9.53), 3.414 (11.00), 3.417 (10.12), 3.438 (3.18), 3.449 (2.92), 3.457 (1.67), 3.466 (1.88), 3.478 (3.52), 3.488 (2.18), 3.506 (1.79), 3.522 (1.06), 3.531 (0.49), 3.535 (0.48), 4.141 (4.05), 4.158 (8.48), 4.176 (4.14), 5.750 (0.90), 6.335 (12.48), 6.501 (12.12), 6.587 (7.06), 7.025 (1.92), 7.028 (2.07), 7.046 (2.53), 7.048 (2.80), 7.055 (2.12), 7.057 (2.41), 7.063 (2.84), 7.067 (2.44), 7.077 (3.46), 7.082 (6.15), 7.086 (4.47), 7.101 (3.55), 7.104 (2.89), 7.183 (1.29), 7.188 (1.47), 7.196 (1.58), 7.201 (2.32), 7.207 (1.92), 7.215 (2.05), 7.221 (2.05), 7.226 (1.17), 7.234 (0.98), 7.239 (0.89), 7.404 (2.00), 7.408 (2.02), 7.425 (3.26), 7.444 (1.92), 7.448 (1.62), 7.721 (4.56), 7.725 (6.14), 7.729 (4.28), 8.290 (9.93), 8.296 (9.26).

### Example 277

### (rac)-2-[6-amino-5-(trifluoromethoxy)pyridin-3-yl]-N-[2-(2-chlorophenyl)propan-2-yl]-6,7-dihydrospiro[pyrazolo[5,1-c][1,4]oxazine-4,3'-pyrrolidine]-1'-carboxamide

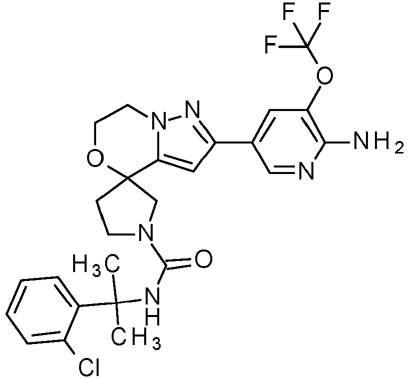

Example 277 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-(2-chlorophenyl)propan-2-amine-hydrochloride salt (52.2 mg, 253 µmol).
LC-MS (method 1): Rt = 1.20 min; MS (ESlpos): m/z = 551 [M+H]⁺
¹H-NMR (400 MHz, DMSO-d6) δ [ppm]: 1.238 (0.47), 1.254 (0.48), 1.678 (16.00), 1.701 (15.31), 1.765 (0.62), 2.171 (0.41), 2.202 (1.01), 2.228 (1.49), 2.251 (0.78), 2.291 (1.20), 2.306 (1.42), 2.323 (1.25), 2.327 (1.25), 2.539 (1.30), 2.669 (0.55), 2.728 (0.64), 2.888 (0.71), 3.391 (1.62), 3.416 (0.77), 3.443 (2.19), 3.471 (2.54), 3.537 (1.03), 3.558 (1.80), 3.580 (0.86), 3.744 (2.06), 3.773 (1.77), 4.049 (0.66), 4.064 (1.40), 4.081 (1.69), 4.101 (2.84), 4.113 (4.92), 4.126 (6.79), 6.152 (6.01), 6.559 (7.44), 6.692 (9.92), 7.166 (1.04), 7.170 (1.11), 7.184 (2.76), 7.188 (2.83), 7.203 (2.41), 7.207 (2.35), 7.234 (1.77), 7.238 (2.09), 7.256 (3.06), 7.272 (1.44), 7.275 (1.37), 7.295 (4.04), 7.299 (3.71), 7.315 (2.99), 7.318 (2.70), 7.454 (2.98), 7.458 (3.06), 7.474 (2.56), 7.478 (2.48), 7.745 (4.55), 8.325 (6.02), 8.330 (6.04).

### Example 278

### (rac)-2-[6-amino-5-(trifluoromethoxy)pyridin-3-yl]-N-[2-(4-fluorophenyl)propan-2-yl]-6,7-dihydrospiro[pyrazolo[5,1-c][1,4]oxazine-4,3'-pyrrolidine]-1'-carboxamide

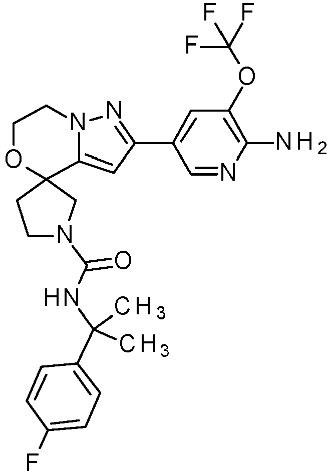

Example 278 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-(4-fluorophenyl)propan-2-amine (38.8 mg, 253 µmol).
LC-MS (method 1): Rt = 1.17 min; MS (ESlpos): m/z = 535 [M+H]⁺
¹H-NMR (400 MHz, DMSO-d6) δ [ppm]: 0.833 (0.71), 0.852 (1.03), 1.232 (4.66), 1.295 (0.56), 1.333 (0.69), 1.348 (0.59), 1.549 (10.08), 1.554 (9.85), 2.074 (16.00), 2.210 (0.52), 2.235 (0.69), 2.322 (2.58), 2.327 (3.22), 2.332 (2.41), 2.518 (12.78), 2.523 (8.56), 2.539 (0.51), 2.665 (2.04), 2.669 (2.88), 2.673 (2.07), 3.159 (2.43), 3.171 (2.46), 3.369 (0.85), 3.392 (0.68), 3.470 (0.82), 3.504 (1.00), 3.570 (0.47), 3.591 (0.83), 3.774 (1.03), 3.803 (0.83), 4.079 (0.66), 4.096 (1.36), 4.111 (2.01), 4.123 (3.05), 4.135 (2.92), 5.758 (0.44), 6.099 (3.02), 6.556 (4.39), 6.715 (6.04), 7.046 (2.13), 7.068 (4.50), 7.091 (2.52), 7.351 (2.19), 7.365 (2.49), 7.373 (2.35), 7.387 (2.07), 7.745 (2.30), 8.325 (3.84), 8.330 (3.68).

### Example 279

### (rac)-2'-[6-amino-5-(trifluoromethoxy)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide

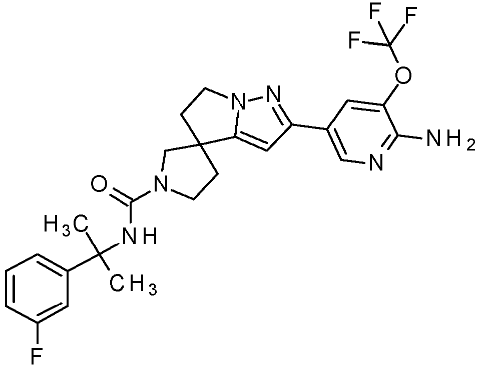

Example 279 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 2-(3-fluorophenyl)propan-2-amine (40.6 mg, 265 µmol).
LC-MS (method 1): Rt = 1.17 min; MS (ESlpos): m/z = 519 [M+H]⁺
¹H-NMR (400 MHz, DMSO-d6) δ [ppm]: 1.036 (3.70), 1.054 (7.76), 1.071 (4.11), 1.230 (0.50), 1.555 (16.00), 2.041 (0.81), 2.056 (1.64), 2.073 (2.40), 2.083 (0.72), 2.091 (0.76), 2.518 (2.60), 2.522 (1.27), 2.535 (2.94), 2.553 (2.02), 3.161 (0.65), 3.173 (0.67), 3.406 (0.61), 3.424 (2.19), 3.437 (1.74), 3.442 (2.14), 3.449 (3.09), 3.453 (2.49), 3.464 (2.58), 3.490 (0.68), 3.537 (0.42), 3.555 (0.81), 3.570 (0.61), 3.578 (0.59), 4.171 (2.08), 4.189 (3.41), 4.206 (2.01), 4.349 (0.47), 4.361 (0.90), 4.373 (0.45), 6.121 (4.15), 6.399 (10.02), 6.513 (5.92), 6.925 (0.61), 6.927 (0.66), 6.931 (0.74), 6.934 (0.76), 6.947 (1.33), 6.953 (1.46), 6.967 (0.73), 6.969 (0.75), 6.974 (0.84), 6.975 (0.77), 7.108 (1.00), 7.114 (1.36), 7.119 (1.12), 7.136 (1.01), 7.141 (1.40), 7.147 (1.08), 7.176 (1.54), 7.196 (2.13), 7.258 (1.28), 7.274 (1.42), 7.278 (1.99), 7.293 (1.91), 7.297 (0.95), 7.314 (0.79), 7.744 (2.26), 7.748 (3.07), 7.752 (2.12), 8.328 (5.19), 8.333 (5.26).

### Example 280

### 2'-[6-amino-5-(trifluoromethoxy)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide (mixture of stereoisomers)

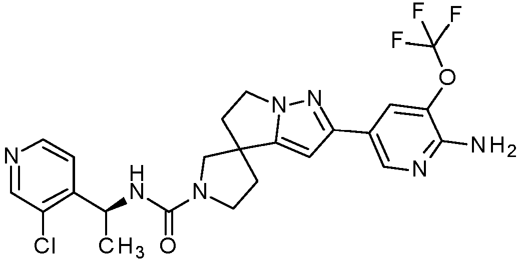

Example 280 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1S)-1-(3-chloropyridin-4-yl)ethan-1-amine-hydrochloride salt (51.2 mg, 265 µmol).
LC-MS (method 1): Rt = 0.99 min; MS (ESlpos): m/z = 522 [M+H]⁺
¹H-NMR (400 MHz, DMSO-d6) δ [ppm]: 1.274 (0.47), 1.291 (0.49), 1.332 (12.00), 1.335 (11.94), 1.349 (12.65), 1.353 (11.99), 2.074 (5.14), 2.083 (4.03), 2.518 (6.46), 2.523 (7.26), 2.540 (7.70), 2.557 (4.48), 3.318 (0.57), 3.368 (0.85), 3.386 (0.42), 3.433 (2.83), 3.459 (5.41), 3.487 (3.97), 3.495 (3.55), 3.512 (2.29), 3.534 (1.69), 3.549 (1.04), 3.582 (0.70), 3.600 (1.04), 4.172 (3.86), 4.188 (6.86), 4.203 (3.73), 5.037 (0.52), 5.055 (2.05), 5.069 (2.98), 5.073 (3.06), 5.087 (2.03), 5.104 (0.55), 6.421 (16.00), 6.511 (13.40), 6.764 (2.54), 6.780 (3.40), 6.796 (2.31), 7.456 (3.34), 7.469 (3.38), 7.529 (3.63), 7.541 (3.75), 7.756 (6.50), 7.761 (6.21), 8.330 (11.58), 8.335 (10.86), 8.438 (3.81), 8.450 (3.71), 8.477 (5.60), 8.490 (5.43), 8.525 (10.94), 8.528 (10.79), 8.571 (0.40).

### Example 281

### 2'-[6-amino-5-(difluoromethoxy)pyridin-3-yl]-N-[2-(4-fluorophenyl)propan-2-yl]-5',6'-dihydrospiro[azetidine-3,4'-pyrrolo[1,2-b]pyrazole]-1-carboxamide

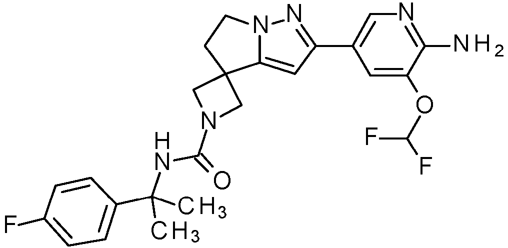

Example 281 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-(4-fluorophenyl)propan-2-amine (44.9 mg, 293 µmol).
LC-MS (method 1): Rt = 1.09 min; MS (ESlpos): m/z = 487 [M+H]⁺
¹H-NMR (400 MHz, DMSO-d6) δ [ppm]: 1.066 (1.49), 1.548 (16.00), 2.518 (1.40), 2.523 (0.94), 2.820 (1.28), 2.837 (2.08), 2.855 (1.36), 3.987 (0.52), 4.008 (8.06), 4.029 (0.51), 4.103 (1.41), 4.121 (2.21), 4.138 (1.34), 6.191 (2.01), 6.568 (3.09), 6.618 (6.86), 7.007 (1.48), 7.074 (1.91), 7.079 (0.61), 7.091 (0.74), 7.097 (4.03), 7.114 (0.64), 7.119 (2.11), 7.192 (3.07), 7.359 (2.04), 7.365 (0.89), 7.373 (2.52), 7.376 (2.46), 7.381 (2.10), 7.390 (0.73), 7.395 (1.82), 7.654 (1.99), 7.656 (2.03), 7.658 (2.02), 8.247 (3.54), 8.252 (3.56).

### Example 282

### 2'-[6-amino-5-(difluoromethoxy)pyridin-3-yl]-N-[2-(2-chlorophenyl)propan-2-yl]-5',6'-dihydrospiro[azetidine-3,4'-pyrrolo[1,2-b]pyrazole]-1-carboxamide

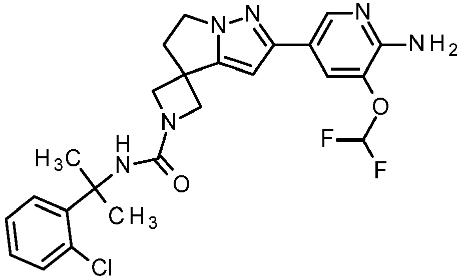

Example 282 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-(2-chlorophenyl)propan-2-amine (49.7 mg, 293 µmol).
LC-MS (method 1): Rt = 1.11 min; MS (ESlpos): m/z = 503 [M+H]⁺
¹H-NMR (400 MHz, DMSO-d6) δ [ppm]: 1.066 (0.81), 1.156 (2.64), 1.634 (1.91), 1.677 (16.00), 2.074 (1.73), 2.518 (3.86), 2.523 (2.59), 2.728 (0.60), 2.801 (1.32), 2.819 (2.17), 2.836 (1.44), 2.888 (0.77), 3.993 (10.15), 4.095 (1.45), 4.112 (2.31), 4.130 (1.40), 6.167 (3.90), 6.541 (1.48), 6.567 (7.25), 6.646 (3.25), 7.005 (1.58), 7.190 (3.78), 7.204 (1.35), 7.208 (1.37), 7.223 (1.23), 7.227 (1.22), 7.252 (0.92), 7.256 (1.07), 7.272 (1.27), 7.276 (1.47), 7.290 (0.75), 7.294 (0.69), 7.332 (2.19), 7.336 (2.16), 7.351 (1.61), 7.355 (1.52), 7.374 (1.49), 7.459 (1.45), 7.463 (1.49), 7.479 (1.24), 7.483 (1.16), 7.646 (2.16), 8.241 (3.52), 8.245 (3.68).

### Example 283

### 2-[6-amino-5-(trifluoromethoxy)pyridin-3-yl]-N-[(1R)-1-(3-fluorophenyl)ethyl]-6,7-dihydrospiro[pyrazolo[5,1-c][1,4]oxazine-4,3'-pyrrolidine]-1'-carboxamide (mixture of stereoisomers)

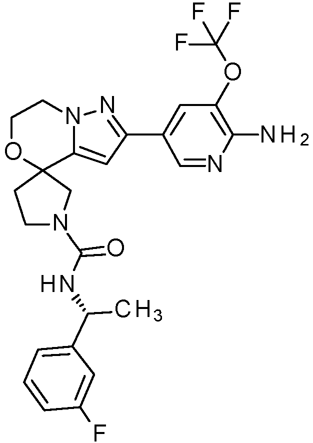

Example 283 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and (1R)-1-(3-fluorophenyl)ethan-1-amine (35.3 mg, 253 µmol).
LC-MS (method 1): Rt = 1.10 min; MS (ESlpos): m/z = 521 [M+H]⁺
¹H-NMR (400 MHz, DMSO-d6) δ [ppm]: 1.066 (0.58), 1.240 (0.65), 1.258 (1.00), 1.275 (0.56), 1.356 (15.96), 1.373 (16.00), 2.073 (0.51), 2.217 (1.34), 2.229 (1.15), 2.244 (1.58), 2.266 (0.79), 2.277 (0.64), 2.327 (1.96), 2.342 (1.91), 2.358 (1.19), 2.374 (1.02), 2.728 (1.09), 2.886 (1.31), 3.391 (2.00), 3.399 (2.79), 3.417 (1.73), 3.425 (1.56), 3.442 (0.87), 3.457 (1.79), 3.488 (2.97), 3.520 (2.11), 3.560 (1.00), 3.582 (1.75), 3.606 (1.64), 3.630 (1.77), 3.652 (0.78), 3.777 (2.03), 3.807 (1.90), 3.817 (2.12), 3.846 (1.70), 4.043 (0.74), 4.059 (1.32), 4.074 (2.16), 4.092 (2.55), 4.105 (3.89), 4.119 (4.92), 4.131 (9.29), 4.830 (1.67), 4.848 (2.33), 4.862 (1.64), 4.879 (0.48), 6.530 (2.66), 6.550 (5.67), 6.566 (8.45), 6.708 (7.29), 6.714 (8.27), 6.989 (1.52), 6.992 (1.57), 6.995 (1.68), 7.015 (3.21), 7.034 (1.75), 7.038 (1.76), 7.150 (3.41), 7.161 (4.67), 7.180 (8.14), 7.307 (1.40), 7.312 (1.52), 7.322 (1.73), 7.327 (3.44), 7.332 (2.45), 7.342 (2.37), 7.347 (3.08), 7.363 (1.05), 7.368 (0.88), 7.747 (5.37), 8.317 (5.29), 8.324 (7.27), 8.329 (5.48).

### Example 284

### 2'-[6-amino-5-(trifluoromethoxy)pyridin-3-yl]-N-(1-phenylcyclobutyl)-5',6'-dihydrospiro[azetidine-3,4'-pyrrolo[1,2-b]pyrazole]-1-carboxamide

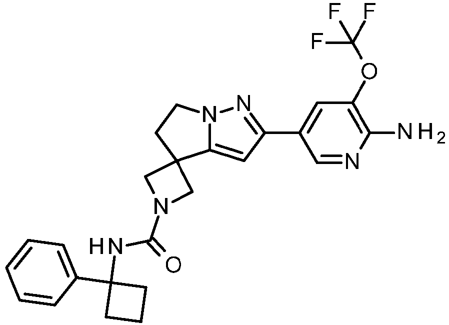

Example 284 was prepared in analogy to Example 31 using 5-(5',6'-dihydrospiro[azetidine-3,4'-pyrrolo[1,2-b]pyrazol]-2'-yl)-3-(trifluoromethoxy)pyridin-2-amine-hydrochloride salt (75.0 mg, 207 µmol) and 1-phenylcyclobutan-1-amine-hydrochloride salt (45.7 mg, 249 µmol).
LC-MS (method 1): Rt = 1.15 min; MS (ESlneg): m/z = 497 [M-H]⁻
¹H-NMR (400 MHz, DMSO-d6) δ [ppm]: 1.156 (0.50), 1.242 (0.90), 1.246 (0.65), 1.258 (1.47), 1.276 (0.80), 1.747 (0.44), 1.752 (0.47), 1.757 (0.41), 1.768 (0.94), 1.774 (0.97), 1.780 (0.68), 1.785 (0.70), 1.791 (0.99), 1.796 (1.15), 1.812 (0.68), 1.818 (0.56), 1.976 (0.46), 1.981 (0.59), 1.998 (1.10), 2.003 (0.82), 2.014 (0.72), 2.020 (0.98), 2.025 (0.96), 2.036 (0.51), 2.041 (0.51), 2.048 (0.50), 2.074 (0.98), 2.084 (11.36), 2.365 (0.91), 2.387 (1.57), 2.395 (2.15), 2.411 (2.61), 2.416 (2.23), 2.432 (1.62), 2.449 (1.82), 2.466 (2.29), 2.472 (2.77), 2.479 (2.01), 2.518 (3.38), 2.523 (2.04), 2.729 (1.80), 2.812 (2.81), 2.829 (4.60), 2.846 (3.00), 2.888 (2.25), 3.967 (1.96), 3.988 (11.55), 3.993 (11.40), 4.014 (1.94), 4.098 (3.12), 4.116 (4.94), 4.133 (3.03), 6.504 (7.58), 6.542 (0.69), 6.564 (0.41), 6.651 (16.00), 6.718 (0.57), 7.073 (6.79), 7.163 (0.81), 7.166 (1.53), 7.169 (0.92), 7.180 (1.14), 7.184 (3.91), 7.188 (1.38), 7.199 (1.50), 7.202 (2.72), 7.206 (1.50), 7.295 (4.28), 7.298 (1.77), 7.314 (7.20), 7.328 (1.45), 7.333 (4.40), 7.415 (6.53), 7.418 (7.37), 7.431 (1.61), 7.435 (5.57), 7.438 (4.23), 7.770 (3.23), 7.775 (4.58), 7.778 (3.12), 8.366 (7.44), 8.370 (7.65).

### Example 285

### 2'-[6-amino-5-(trifluoromethoxy)pyridin-3-yl]-N-benzyl-5',6'-dihydrospiro[azetidine-3,4'-pyrrolo[1,2-b]pyrazole]-1-carboxamide

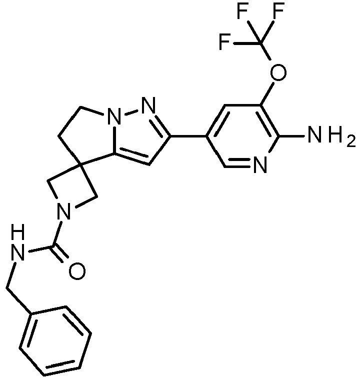

Example 285 was prepared in analogy to Example 31 using 5-(5',6'-dihydrospiro[azetidine-3,4'-pyrrolo[1,2-b]pyrazol]-2'-yl)-3-(trifluoromethoxy)pyridin-2-amine-hydrochloride salt (75.0 mg, 207 µmol) and 1-phenylmethanamine (26.7 mg, 249 µmol).
LC-MS (method 1): Rt = 1.03 min; MS (ESlpos): m/z = 459 [M+H]⁺
¹H-NMR (400 MHz, DMSO-d6) δ [ppm]: 1.040 (0.88), 1.057 (1.11), 1.067 (1.22), 1.156 (2.22), 2.074 (1.60), 2.084 (0.90), 2.518 (1.82), 2.523 (1.24), 2.729 (6.30), 2.833 (2.79), 2.850 (4.52), 2.867 (2.99), 2.887 (7.60), 4.014 (1.58), 4.034 (14.65), 4.037 (14.32), 4.058 (1.55), 4.105 (3.06), 4.124 (4.76), 4.140 (2.93), 4.223 (12.89), 4.238 (13.13), 6.422 (1.12), 6.436 (2.12), 6.452 (1.11), 6.505 (8.06), 6.545 (0.70), 6.682 (16.00), 7.062 (1.55), 7.078 (3.38), 7.093 (1.54), 7.196 (0.57), 7.200 (1.19), 7.204 (0.94), 7.210 (1.35), 7.218 (3.48), 7.227 (3.42), 7.239 (6.61), 7.244 (3.44), 7.249 (2.42), 7.256 (7.69), 7.260 (5.32), 7.271 (3.46), 7.288 (9.73), 7.292 (12.67), 7.297 (2.95), 7.307 (11.09), 7.310 (7.58), 7.312 (6.52), 7.324 (7.03), 7.328 (4.78), 7.339 (0.94), 7.343 (2.13), 7.345 (1.31), 7.771 (3.05), 7.775 (4.33), 7.779 (2.93), 7.951 (0.92), 8.366 (7.23), 8.371 (7.43).

### Example 286

### 2'-[6-amino-5-(trifluoromethoxy)pyridin-3-yl]-N-[(1R)-1-(4-cyanopyridin-2-yl)ethyl]-5',6'-dihydrospiro[azetidine-3,4'-pyrrolo[1,2-b]pyrazole]-1-carboxamide

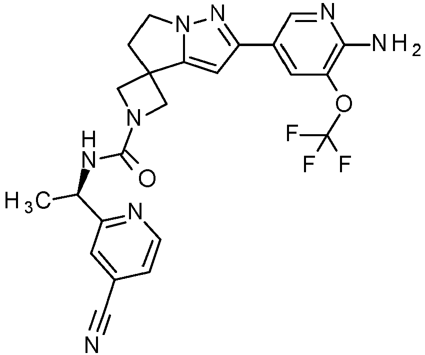

Example 286 was prepared in analogy to Example 31 using 5-(5',6'-dihydrospiro[azetidine-3,4'-pyrrolo[1,2-b]pyrazol]-2'-yl)-3-(trifluoromethoxy)pyridin-2-amine-hydrochloride salt (75.0 mg, 207 µmol) and 2-[(1R)-1-aminoethyl]pyridine-4-carbonitrile (36.6 mg, 249 µmol).
LC-MS (method 1): Rt = 0.92 min; MS (ESlpos): m/z = 499 [M+H]⁺
¹H-NMR (400 MHz, DMSO-d6) δ [ppm]: 1.066 (2.05), 1.397 (11.00), 1.415 (11.00), 2.083 (1.88), 2.460 (0.45), 2.465 (0.55), 2.518 (1.84), 2.523 (1.21), 2.729 (0.51), 2.823 (2.88), 2.830 (3.15), 2.848 (4.77), 2.865 (3.20), 2.887 (0.70), 3.908 (1.96), 3.911 (1.96), 4.003 (2.95), 4.024 (4.83), 4.041 (8.08), 4.063 (1.49), 4.070 (4.45), 4.091 (2.99), 4.104 (3.53), 4.122 (5.57), 4.130 (1.82), 4.138 (3.38), 4.149 (0.83), 4.882 (1.65), 4.900 (2.38), 4.919 (1.61), 6.484 (4.28), 6.488 (2.41), 6.504 (8.96), 6.677 (16.00), 6.997 (3.72), 7.017 (3.57), 7.754 (0.79), 7.765 (3.76), 7.770 (4.88), 7.773 (3.30), 8.252 (3.42), 8.257 (6.27), 8.262 (3.38), 8.337 (1.52), 8.342 (1.46), 8.361 (7.99), 8.366 (7.83), 8.848 (6.60), 8.854 (6.39), 8.901 (7.37), 8.906 (7.37).

### Example 287

### 2'-[6-amino-5-(trifluoromethoxy)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide

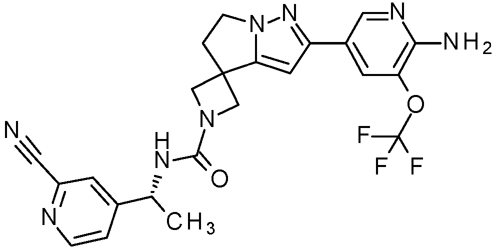

Example 287 was prepared in analogy to Example 31 using 5-(5',6'-dihydrospiro[azetidine-3,4'-pyrrolo[1,2-b]pyrazol]-2'-yl)-3-(trifluoromethoxy)pyridin-2-amine-hydrochloride salt (75.0 mg, 207 µmol) and 4-[(1R)-1-aminoethyl]pyridine-2-carbonitrile (36.6 mg, 249 µmol).
LC-MS (method 1): Rt = 0.94 min; MS (ESlneg): m/z = 497 [M-H]⁻
¹H-NMR (400 MHz, DMSO-d6) δ [ppm]: 1.240 (1.04), 1.255 (1.17), 1.270 (0.73), 1.366 (10.98), 1.383 (11.13), 2.074 (2.53), 2.083 (1.46), 2.518 (3.40), 2.523 (2.40), 2.539 (9.58), 2.727 (0.95), 2.840 (2.81), 2.857 (4.54), 2.875 (3.04), 2.888 (1.29), 4.018 (2.82), 4.031 (1.54), 4.038 (4.54), 4.051 (5.99), 4.057 (6.16), 4.078 (1.44), 4.086 (4.10), 4.109 (4.58), 4.128 (5.22), 4.145 (3.08), 4.832 (1.57), 4.850 (2.35), 4.869 (1.57), 6.509 (6.94), 6.683 (16.00), 7.048 (3.57), 7.067 (3.43), 7.683 (2.91), 7.686 (2.95), 7.696 (3.11), 7.700 (3.02), 7.768 (3.33), 7.772 (4.72), 7.777 (3.20), 7.998 (5.21), 8.001 (5.26), 8.003 (5.42), 8.364 (8.08), 8.369 (7.96), 8.694 (5.12), 8.696 (5.14), 8.707 (4.88), 8.709 (4.88).

### Example 288

### 2'-[6-amino-5-(trifluoromethoxy)pyridin-3-yl]-N-[(1R)-1-(5-fluoropyridin-3-yl)ethyl]-5',6'-dihydrospiro[pyrrolidine-3,4'-pyrrolo[1,2-b]pyrazole]-1-carboxamide (mixture of stereoisomers)

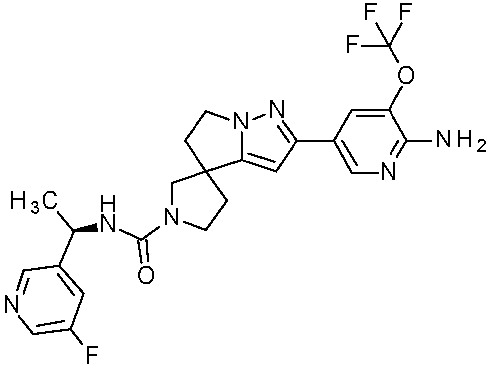

Example 288 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1R)-1-(5-fluoropyridin-3-yl)ethan-1-amine (37.2 mg, 265 µmol).
LC-MS (method 1): Rt = 0.95 min; MS (ESlneg): m/z = 504 [M-H]⁻
¹H-NMR (400 MHz, DMSO-d6) δ [ppm]: 1.067 (7.42), 1.154 (0.90), 1.395 (12.36), 1.401 (13.25), 1.413 (12.84), 1.418 (12.87), 2.050 (2.89), 2.062 (4.44), 2.069 (4.61), 2.074 (4.42), 2.077 (4.20), 2.108 (0.68), 2.518 (7.56), 2.536 (9.89), 2.553 (6.30), 2.727 (0.40), 2.886 (0.49), 3.406 (0.75), 3.423 (3.74), 3.431 (2.94), 3.448 (7.92), 3.456 (7.12), 3.466 (6.87), 3.481 (4.40), 3.491 (2.40), 3.507 (2.60), 3.527 (1.93), 3.534 (1.08), 3.541 (1.30), 3.552 (1.16), 3.560 (1.00), 3.567 (0.92), 3.578 (1.53), 3.586 (1.03), 3.593 (1.10), 3.604 (1.06), 3.619 (0.58), 3.943 (1.24), 4.164 (5.85), 4.181 (9.82), 4.198 (5.67), 4.889 (0.53), 4.901 (1.92), 4.907 (2.05), 4.920 (2.99), 4.924 (2.97), 4.939 (2.08), 4.956 (0.54), 5.779 (0.95), 6.399 (6.74), 6.411 (16.00), 6.504 (13.68), 6.590 (3.08), 6.604 (3.73), 6.609 (3.64), 6.623 (3.02), 7.641 (1.59), 7.647 (2.14), 7.652 (1.68), 7.667 (1.66), 7.672 (2.28), 7.677 (3.14), 7.682 (2.27), 7.687 (1.69), 7.703 (1.58), 7.708 (2.07), 7.713 (1.60), 7.754 (8.52), 8.325 (13.55), 8.330 (13.14), 8.398 (6.79), 8.405 (6.86), 8.411 (6.88), 8.418 (6.55), 8.433 (3.45), 8.437 (6.29), 8.442 (3.57), 8.452 (3.35), 8.457 (5.95), 8.461 (3.29).

### Example 289

### (rac)-2'-[6-amino-5-(trifluoromethoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide

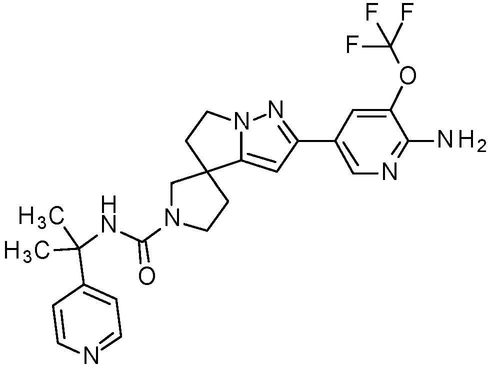

Example 289 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 2-(pyridin-4-yl)propan-2-amine (36.1 mg, 265 µmol).
LC-MS (method 1): Rt = 0.95 min; MS (ESlneg): m/z = 500 [M-H]⁻
¹H-NMR (400 MHz, DMSO-d6) δ [ppm]: 1.066 (0.71), 1.486 (3.82), 1.537 (16.00), 1.907 (0.60), 2.043 (0.87), 2.059 (1.69), 2.076 (1.87), 2.083 (0.92), 2.095 (0.79), 2.107 (0.43), 2.518 (3.97), 2.536 (2.89), 2.539 (3.12), 2.555 (1.92), 2.729 (0.59), 2.888 (0.73), 3.403 (0.49), 3.423 (1.27), 3.449 (3.35), 3.459 (2.83), 3.485 (0.60), 3.539 (0.46), 3.557 (0.83), 3.572 (0.67), 4.172 (2.09), 4.189 (3.83), 4.207 (2.02), 6.221 (4.04), 6.423 (8.42), 6.517 (6.11), 7.310 (5.40), 7.315 (3.51), 7.322 (3.55), 7.326 (5.40), 7.502 (0.68), 7.506 (0.48), 7.513 (0.49), 7.518 (0.74), 7.753 (2.27), 7.758 (3.06), 8.336 (4.67), 8.341 (4.61), 8.414 (5.84), 8.419 (3.60), 8.426 (3.49), 8.430 (5.52), 8.558 (0.63), 8.562 (0.43), 8.570 (0.41), 8.573 (0.58).

### Example 290

### (rac)-2'-[6-amino-5-(trifluoromethoxy)pyridin-3-yl]-N-(1-phenylcyclobutyl)-5',6'-dihydrospiro[pyrrolidine-3,4'-pyrrolo[1,2-b]pyrazole]-1-carboxamide

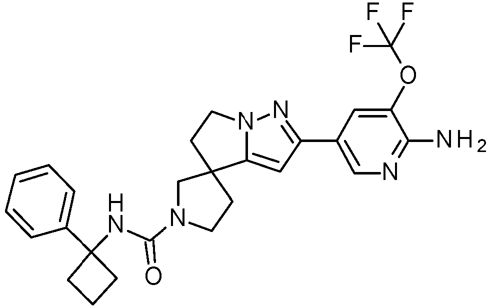

Example 290 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 1-phenylcyclobutan-1-amine-hydrochloride salt (48.7 mg, 265 µmol).
LC-MS (method 1): Rt = 1.17 min; MS (ESlneg): m/z = 511 [M-H]⁻
¹H-NMR (400 MHz, DMSO-d6) δ [ppm]: 1.000 (1.59), 1.016 (1.58), 1.043 (0.80), 1.066 (6.87), 1.156 (9.86), 1.239 (1.19), 1.254 (1.39), 1.271 (0.67), 1.746 (0.74), 1.763 (1.46), 1.768 (1.55), 1.785 (1.63), 1.790 (1.78), 1.807 (1.10), 1.829 (0.41), 1.964 (0.96), 1.979 (1.70), 2.002 (1.70), 2.007 (1.75), 2.030 (2.59), 2.043 (3.72), 2.059 (4.11), 2.075 (1.77), 2.088 (0.85), 2.356 (1.40), 2.385 (3.17), 2.401 (3.74), 2.423 (2.29), 2.449 (2.16), 2.470 (4.14), 2.518 (13.18), 2.523 (10.55), 2.539 (4.06), 2.555 (0.67), 2.728 (2.71), 2.888 (3.31), 3.367 (0.52), 3.375 (1.02), 3.396 (3.20), 3.421 (8.07), 3.433 (6.04), 3.459 (1.45), 3.504 (1.09), 3.521 (1.99), 3.537 (1.57), 3.546 (1.42), 3.563 (0.77), 3.939 (1.13), 4.159 (4.64), 4.177 (8.96), 4.194 (4.49), 6.371 (16.00), 6.511 (13.43), 6.541 (1.39), 6.696 (8.02), 7.131 (2.32), 7.149 (5.73), 7.167 (3.75), 7.258 (6.65), 7.278 (10.79), 7.297 (6.22), 7.383 (0.43), 7.417 (9.38), 7.419 (10.12), 7.437 (8.20), 7.440 (6.16), 7.733 (4.96), 7.738 (6.71), 7.741 (4.65), 7.942 (1.00), 7.951 (0.46), 8.312 (10.59), 8.316 (10.48).

### Example 291

### (rac)-2'-[6-amino-5-(trifluoromethoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide

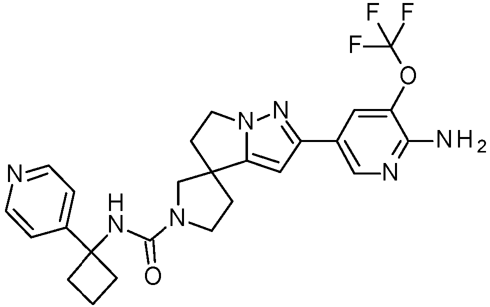

Example 291 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 1-(pyridin-4-yl)cyclobutan-1-amine (39.3 mg, 265 µmol).
LC-MS (method 1): Rt = 0.97 min; MS (ESlneg): m/z = 512 [M-H]⁻
¹H-NMR (400 MHz, DMSO-d6) δ [ppm]: 1.017 (0.84), 1.033 (0.85), 1.058 (0.48), 1.066 (2.98), 1.123 (0.48), 1.136 (1.35), 1.153 (1.46), 1.204 (0.42), 1.236 (1.78), 1.246 (4.77), 1.254 (3.26), 1.262 (5.19), 1.268 (4.93), 1.285 (4.48), 1.812 (0.84), 1.829 (1.60), 1.835 (1.74), 1.857 (2.04), 1.876 (1.32), 1.897 (0.52), 1.972 (0.42), 1.988 (0.84), 1.995 (1.09), 2.011 (2.04), 2.033 (2.41), 2.038 (2.38), 2.047 (2.96), 2.061 (4.32), 2.077 (4.42), 2.094 (1.79), 2.126 (0.48), 2.362 (1.47), 2.392 (3.60), 2.408 (4.16), 2.413 (4.08), 2.429 (4.25), 2.454 (3.74), 2.518 (10.29), 2.523 (6.57), 2.530 (6.62), 2.535 (6.58), 2.539 (6.75), 2.551 (4.04), 2.565 (1.47), 2.585 (0.76), 2.784 (0.52), 3.114 (0.96), 3.124 (1.00), 3.133 (1.09), 3.143 (1.08), 3.162 (0.75), 3.418 (6.35), 3.445 (12.32), 3.486 (2.46), 3.546 (2.45), 3.585 (1.49), 3.601 (1.02), 3.611 (0.93), 3.628 (0.76), 4.019 (1.85), 4.168 (4.96), 4.185 (9.53), 4.202 (4.76), 6.412 (16.00), 6.518 (9.77), 6.942 (7.52), 7.429 (0.42), 7.509 (9.82), 7.524 (9.42), 7.553 (0.68), 7.557 (0.46), 7.564 (0.44), 7.568 (0.66), 7.684 (0.61), 7.748 (5.44), 7.752 (7.39), 7.756 (5.24), 8.326 (11.59), 8.331 (11.50), 8.497 (0.49), 8.523 (11.55), 8.527 (7.63), 8.539 (11.12), 8.673 (0.67), 8.677 (0.47), 8.688 (0.60).

### Example 292

### 2'-[6-amino-5-(difluoromethoxy)pyridin-3-yl]-N-[2-(3-fluorophenyl)propan-2-yl]-5',6'-dihydrospiro[azetidine-3,4'-pyrrolo[1,2-b]pyrazole]-1-carboxamide

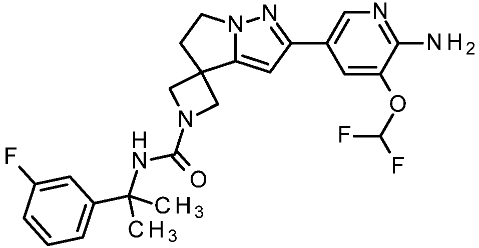

Example 292 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-(3-fluorophenyl)propan-2-amine (44.9 mg, 293 µmol).
LC-MS (method 1): Rt = 1.08 min; MS (ESlpos): m/z = 487 [M+H]⁺
¹H-NMR (400 MHz, DMSO-d6) δ [ppm]: 1.065 (12.56), 1.155 (0.52), 1.239 (1.75), 1.247 (5.98), 1.258 (4.08), 1.263 (6.52), 1.271 (6.16), 1.276 (2.65), 1.288 (5.71), 1.547 (16.00), 1.624 (0.91), 2.518 (4.58), 2.523 (3.29), 2.827 (1.26), 2.844 (2.08), 2.862 (1.36), 3.112 (0.78), 3.123 (0.82), 3.131 (0.81), 3.141 (0.79), 3.583 (0.49), 3.594 (0.50), 3.600 (0.65), 3.610 (0.65), 3.616 (0.50), 3.627 (0.47), 3.999 (0.75), 4.020 (5.91), 4.024 (5.76), 4.046 (0.82), 4.106 (1.40), 4.124 (2.23), 4.141 (1.40), 6.263 (0.83), 6.622 (7.38), 6.965 (0.43), 6.972 (0.49), 6.985 (0.88), 6.992 (1.00), 7.008 (0.56), 7.016 (1.46), 7.036 (0.99), 7.112 (0.70), 7.118 (0.93), 7.122 (0.76), 7.140 (0.69), 7.144 (0.96), 7.151 (0.78), 7.163 (1.16), 7.185 (1.10), 7.201 (3.17), 7.290 (0.95), 7.304 (0.81), 7.320 (0.92), 7.324 (1.27), 7.339 (1.21), 7.344 (0.71), 7.360 (0.55), 7.385 (1.15), 7.671 (1.94), 8.244 (3.62), 8.248 (3.46).

### Example 293

### 2'-[6-amino-5-(difluoromethoxy)pyridin-3-yl]-N-[(1S)-1-(3-chloropyridin-4-yl)ethyl]-5',6'-dihydrospiro[azetidine-3,4'-pyrrolo[1,2-b]pyrazole]-1-carboxamide

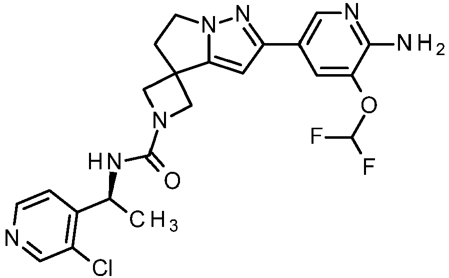

Example 293 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and (1S)-1-(3-chloropyridin-4-yl)ethan-1-amine-hydrochloride salt (56.5 mg, 293 µmol).
LC-MS (method 1): Rt = 0.90 min; MS (ESlneg): m/z = 488 [M-H]⁻
¹H-NMR (400 MHz, DMSO-d6) δ [ppm]: 1.212 (0.40), 1.231 (1.83), 1.263 (1.15), 1.281 (1.19), 1.328 (13.28), 1.346 (13.41), 2.518 (7.29), 2.522 (5.21), 2.838 (3.18), 2.855 (5.34), 2.872 (3.43), 3.824 (0.68), 4.022 (3.02), 4.047 (7.49), 4.054 (6.92), 4.081 (4.88), 4.106 (4.37), 4.124 (5.83), 4.141 (3.56), 5.005 (0.44), 5.023 (2.00), 5.042 (3.05), 5.059 (2.01), 5.078 (0.47), 6.171 (9.51), 6.431 (0.88), 6.635 (16.00), 7.000 (3.61), 7.184 (9.11), 7.200 (3.83), 7.369 (3.16), 7.483 (5.70), 7.496 (5.89), 7.643 (5.20), 8.213 (0.54), 8.218 (0.54), 8.238 (8.54), 8.243 (7.96), 8.418 (0.40), 8.517 (7.88), 8.530 (7.19), 8.548 (13.03).

### Example 294

### 2'-[6-amino-5-(difluoromethoxy)pyridin-3-yl]-N-[2-(3-chloropyridin-4-yl)propan-2-yl]-5',6'-dihydrospiro[azetidine-3,4'-pyrrolo[1,2-b]pyrazole]-1-carboxamide

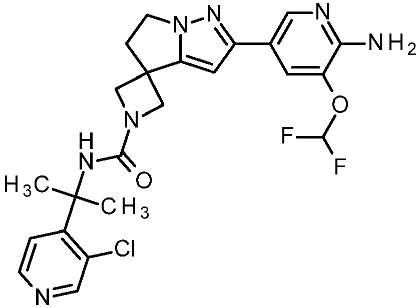

Example 294 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-(3-chloropyridin-4-yl)propan-2-amine (50.0 mg, 293 µmol).
LC-MS (method 1): Rt = 0.93 min; MS (ESlpos): m/z = 504 [M+H]⁺
¹H-NMR (400 MHz, DMSO-d6) δ [ppm]: 1.066 (2.58), 1.232 (1.82), 1.498 (0.53), 1.560 (2.88), 1.645 (16.00), 2.518 (6.33), 2.523 (4.35), 2.728 (8.84), 2.808 (1.30), 2.825 (2.10), 2.842 (1.39), 2.888 (10.66), 3.980 (0.47), 4.000 (9.00), 4.096 (1.42), 4.114 (2.24), 4.130 (1.39), 6.169 (3.79), 6.577 (7.72), 6.857 (3.23), 7.006 (1.62), 7.190 (3.45), 7.374 (1.42), 7.433 (2.36), 7.446 (2.37), 7.647 (2.08), 7.951 (1.29), 8.243 (3.83), 8.248 (3.53), 8.316 (0.57), 8.330 (0.53), 8.417 (3.94), 8.425 (1.17), 8.430 (3.51), 8.469 (7.07).

### Example 295

### 2'-[6-amino-5-(difluoromethoxy)pyridin-3-yl]-N-[(1R)-1-(2-chloro-4-cyanophenyl)ethyl]-5',6'-dihydrospiro[azetidine-3,4'-pyrrolo[1,2-b]pyrazole]-1-carboxamide

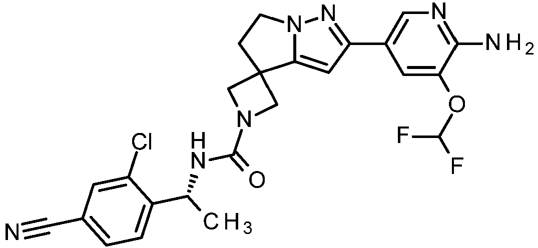

Example 295 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 4-[(1R)-1-aminoethyl]-3-chlorobenzonitrile (52.9 mg, 293 µmol).
LC-MS (method 1): Rt = 1.04 min; MS (ESlneg): m/z = 512 [M-H]⁻
¹H-NMR (400 MHz, DMSO-d6) δ [ppm]: 1.066 (2.20), 1.259 (0.98), 1.277 (0.91), 1.321 (15.42), 1.339 (15.48), 2.074 (0.68), 2.539 (1.98), 2.834 (4.36), 2.852 (7.61), 2.869 (4.62), 3.814 (0.75), 4.015 (4.90), 4.036 (12.64), 4.045 (9.39), 4.074 (6.45), 4.095 (4.18), 4.106 (5.20), 4.123 (8.06), 4.140 (4.72), 5.078 (0.60), 5.096 (2.60), 5.114 (3.91), 5.132 (2.59), 5.150 (0.62), 6.228 (5.08), 6.433 (0.70), 6.639 (16.00), 7.006 (3.69), 7.190 (9.81), 7.205 (5.02), 7.374 (3.46), 7.625 (0.44), 7.658 (13.85), 7.678 (7.67), 7.857 (5.01), 7.861 (5.26), 7.877 (4.08), 7.881 (4.35), 7.978 (0.43), 8.005 (9.38), 8.009 (9.26), 8.214 (0.56), 8.237 (9.23), 8.241 (9.45).

### Example 296

### 2'-[6-amino-5-(difluoromethoxy)pyridin-3-yl]-N-[1-(2-fluorophenyl)cyclobutyl]-5',6'-dihydrospiro[azetidine-3,4'-pyrrolo[1,2-b]pyrazole]-1-carboxamide

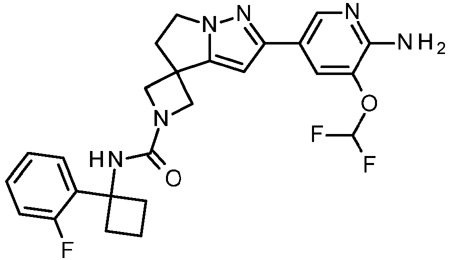

Example 296 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 1-(2-fluorophenyl)cyclobutan-1-amine-hydrochloride salt (59.1 mg, 293 µmol).
LC-MS (method 1): Rt = 1.11 min; MS (ESlpos): m/z = 499 [M+H]⁺
¹H-NMR (400 MHz, DMSO-d6) δ [ppm]: 1.714 (0.44), 1.720 (0.51), 1.726 (0.62), 1.734 (0.95), 1.740 (1.00), 1.748 (1.04), 1.755 (0.92), 1.761 (1.14), 1.775 (0.69), 1.782 (0.48), 2.019 (0.75), 2.039 (1.27), 2.061 (1.16), 2.066 (1.17), 2.074 (3.69), 2.087 (0.70), 2.458 (0.71), 2.518 (4.76), 2.523 (4.99), 2.540 (2.12), 2.571 (0.45), 2.786 (2.95), 2.803 (4.89), 2.821 (3.14), 3.936 (2.08), 3.956 (12.51), 3.962 (12.24), 3.983 (2.03), 4.081 (3.25), 4.098 (5.13), 4.116 (3.11), 6.159 (8.56), 6.575 (16.00), 6.994 (3.53), 7.051 (6.99), 7.065 (1.66), 7.068 (1.76), 7.086 (2.02), 7.089 (2.55), 7.091 (2.43), 7.094 (3.83), 7.110 (3.88), 7.113 (4.24), 7.129 (2.82), 7.132 (1.99), 7.179 (7.27), 7.219 (0.96), 7.223 (1.11), 7.232 (1.15), 7.237 (1.72), 7.244 (1.45), 7.250 (1.08), 7.256 (1.60), 7.262 (0.76), 7.271 (0.70), 7.275 (0.65), 7.363 (3.00), 7.411 (1.49), 7.415 (1.51), 7.431 (2.52), 7.435 (2.51), 7.451 (1.45), 7.456 (1.16), 7.634 (4.80), 7.636 (4.70), 8.222 (8.17), 8.227 (7.57).

### Example 297

### (rac)-2'-[6-amino-5-(trifluoromethoxy)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide

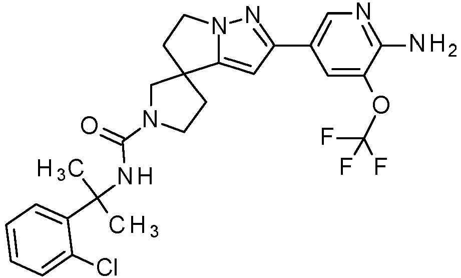

Example 297 was prepared in analogy to Example 31 using (rac)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 2-(2-chlorophenyl)propan-2-amine-hydrochloride salt (54.7 mg, 265 µmol).
LC-MS (method 1): Rt = 1.20 min; MS (ESlneg): m/z = 533 [M-H]⁻
¹H-NMR (400 MHz, DMSO-d6) δ [ppm]: 1.520 (0.85), 1.681 (16.00), 1.693 (15.12), 1.902 (1.48), 2.014 (0.46), 2.028 (1.19), 2.043 (2.38), 2.061 (2.76), 2.073 (2.52), 2.078 (1.16), 2.091 (0.58), 2.518 (3.35), 2.523 (3.57), 2.542 (0.47), 2.849 (0.45), 2.887 (0.46), 2.900 (0.41), 3.413 (8.38), 3.440 (1.06), 3.483 (0.76), 3.502 (1.35), 3.516 (1.00), 3.526 (0.88), 3.542 (0.47), 4.121 (0.41), 4.145 (0.43), 4.155 (2.76), 4.173 (5.77), 4.191 (2.68), 6.121 (5.87), 6.382 (12.45), 6.418 (1.35), 6.478 (0.89), 6.516 (8.31), 7.157 (1.11), 7.162 (1.18), 7.176 (2.70), 7.180 (2.85), 7.195 (2.52), 7.199 (2.48), 7.227 (1.80), 7.231 (2.32), 7.247 (2.43), 7.250 (3.08), 7.265 (1.50), 7.269 (1.46), 7.278 (4.70), 7.282 (4.03), 7.297 (3.16), 7.301 (2.75), 7.457 (2.84), 7.461 (2.96), 7.476 (2.48), 7.480 (2.36), 7.732 (3.30), 7.735 (4.60), 7.739 (3.20), 8.317 (7.48), 8.322 (6.90).

### Example 298

### 2-[6-amino-5-(trifluoromethoxy)pyridin-3-yl]-N-[(1R)-1-(4-cyanopyridin-2-yl)ethyl]-6,7-dihydrospiro[pyrazolo[5,1-c][1,4]oxazine-4,3'-pyrrolidine]-1'-carboxamide (mixture of stereoisomers)

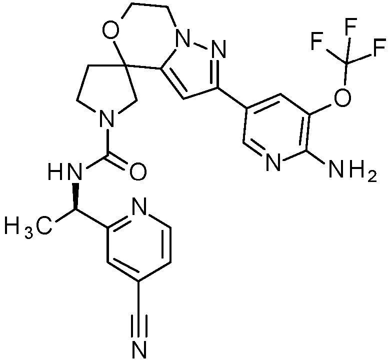

Example 298 was prepared in analogy to Example 31 using (rac)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-[(1R)-1-aminoethyl]pyridine-4-carbonitrile (37.3 mg, 253 µmol).
LC-MS (method 1): Rt = 0.93 min; MS (ESlpos): m/z = 529 [M+H]⁺
¹H-NMR (400 MHz, DMSO-d6) δ [ppm]: 1.066 (0.87), 1.126 (0.44), 1.144 (0.47), 1.156 (0.46), 1.167 (0.44), 1.183 (0.41), 1.195 (0.51), 1.211 (0.52), 1.226 (1.52), 1.241 (5.73), 1.258 (10.11), 1.274 (4.94), 1.406 (13.87), 1.424 (13.88), 1.907 (1.05), 2.084 (10.64), 2.242 (1.12), 2.323 (1.87), 2.327 (2.77), 2.332 (2.44), 2.336 (1.85), 2.518 (6.12), 2.523 (4.14), 2.539 (16.00), 2.665 (1.31), 2.669 (1.84), 2.673 (1.32), 3.122 (0.65), 3.133 (0.66), 3.141 (0.63), 3.152 (0.62), 3.183 (0.80), 3.376 (1.75), 3.393 (1.43), 3.424 (1.13), 3.450 (1.54), 3.479 (1.33), 3.508 (1.14), 3.538 (1.42), 3.571 (1.46), 3.593 (1.01), 3.610 (0.75), 3.620 (1.41), 3.642 (1.48), 3.668 (0.65), 3.758 (1.70), 3.784 (1.40), 3.826 (1.65), 3.855 (1.39), 4.078 (1.80), 4.112 (3.96), 4.118 (4.23), 4.131 (7.42), 4.889 (1.16), 4.907 (1.93), 4.920 (2.00), 4.938 (1.21), 6.559 (7.78), 6.628 (1.77), 6.648 (2.03), 6.654 (2.12), 6.674 (1.73), 6.705 (5.95), 6.717 (7.50), 7.741 (5.41), 7.745 (5.24), 8.257 (2.55), 8.262 (6.61), 8.267 (6.54), 8.272 (2.50), 8.313 (5.59), 8.319 (9.96), 8.324 (6.22), 8.841 (5.12), 8.846 (7.55), 8.850 (4.86), 8.882 (5.67), 8.886 (7.48), 8.889 (5.49).

### Example 299

### (rac)-2-[6-amino-5-(difluoromethoxy)pyridin-3-yl]-N-[1-(2-chlorophenyl)cyclobutyl]-6,7-dihydrospiro[pyrazolo[5,1-c][1,4]oxazine-4,3'-pyrrolidine]-1'-carboxamide

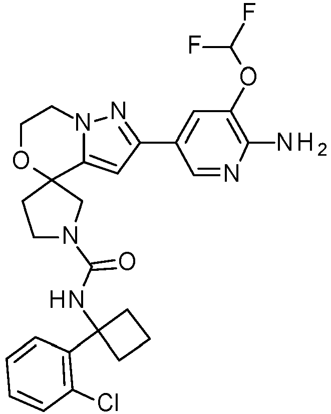

Example 299 was prepared in analogy to Example 31 using (*rac*)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and 1-(2-chlorophenyl)cyclobutan-1-amine-hydrochloride salt (52.5 mg, 241 µmol).

¹H-NMR (400 MHz, DMSO-d6) δ [ppm]: 1.137 (1.02), 1.232 (1.23), 1.676 (0.80), 1.691 (0.84), 1.702 (0.92), 1.713 (0.65), 1.724 (0.41), 2.030 (0.64), 2.051 (1.18), 2.078 (1.24), 2.084 (16.00), 2.099 (0.70), 2.116 (0.54), 2.194 (0.67), 2.219 (0.93), 2.243 (0.54), 2.275 (0.85), 2.291 (1.01), 2.318 (1.29), 2.322 (2.57), 2.327 (3.40), 2.332 (2.38), 2.336 (1.01), 2.518 (10.90), 2.523 (7.42), 2.550 (1.09), 2.570 (0.50), 2.609 (0.83), 2.619 (1.16), 2.629 (1.62), 2.639 (1.65), 2.649 (1.61), 2.660 (2.22), 2.665 (2.79), 2.669 (3.79), 2.673 (2.63), 2.678 (1.43), 2.720 (0.42), 3.411 (1.16), 3.440 (1.31), 3.497 (0.74), 3.520 (1.28), 3.542 (0.56), 3.713 (1.35), 3.742 (1.13), 4.019 (0.55), 4.033 (0.97), 4.050 (1.41), 4.068 (1.45), 4.080 (1.74), 4.089 (2.05), 4.108 (4.48), 6.214 (7.08), 6.532 (4.94), 6.632 (7.08), 6.989 (2.96), 7.173 (6.53), 7.180 (1.42), 7.193 (2.90), 7.199 (2.80), 7.212 (2.85), 7.217 (2.53), 7.229 (2.13), 7.233 (2.66), 7.247 (2.85), 7.252 (3.36), 7.266 (1.46), 7.270 (1.31), 7.298 (4.37), 7.302 (4.14), 7.317 (2.82), 7.321 (2.70), 7.357 (2.71), 7.521 (2.78), 7.526 (2.75), 7.540 (2.58), 7.545 (2.36), 7.596 (3.87), 8.166 (5.90), 8.170 (6.03).

### Example 300

### (rac)-2-[6-amino-5-(trifluoromethoxy)pyridin-3-yl]-N-[1-(pyridin-2-yl)cyclobutyl]-6,7-dihydrospiro[pyrazolo[5,1-c][1,4]oxazine-4,3'-pyrrolidine]-1'-carboxamide

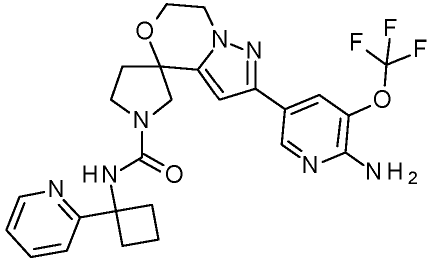

Example 300 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(pyridin-2-yl)cyclobutan-1-amine (37.5 mg, 253 µmol).
LC-MS (method 1): Rt = 1.00 min; MS (ESlneg): m/z = 528 [M-H]⁻
¹H-NMR (400 MHz, DMSO-d6) δ [ppm]: 1.066 (0.92), 1.898 (1.99), 1.983 (1.89), 2.007 (1.64), 2.075 (4.06), 2.085 (1.06), 2.231 (1.73), 2.264 (2.05), 2.323 (3.95), 2.327 (5.92), 2.332 (5.66), 2.336 (5.12), 2.358 (4.73), 2.366 (4.46), 2.382 (4.77), 2.406 (2.13), 2.454 (1.41), 2.458 (2.01), 2.463 (2.41), 2.518 (15.20), 2.523 (10.36), 2.535 (1.56), 2.539 (1.93), 2.545 (1.50), 2.568 (2.66), 2.590 (3.16), 2.613 (2.11), 2.665 (3.72), 2.669 (5.28), 2.673 (3.62), 2.728 (0.50), 2.888 (0.60), 2.942 (0.65), 2.971 (0.73), 3.098 (0.83), 3.289 (1.60), 3.413 (1.70), 3.499 (2.10), 3.528 (2.23), 3.634 (2.49), 3.810 (2.84), 3.838 (2.45), 4.057 (1.73), 4.068 (2.18), 4.086 (2.48), 4.105 (4.14), 4.131 (8.01), 4.143 (8.35), 4.161 (2.65), 6.544 (3.79), 6.559 (15.00), 6.623 (4.16), 6.729 (16.00), 6.810 (8.18), 7.169 (3.79), 7.173 (3.98), 7.181 (3.80), 7.185 (4.24), 7.188 (4.28), 7.191 (4.17), 7.200 (3.98), 7.203 (4.08), 7.352 (6.72), 7.372 (7.46), 7.677 (4.16), 7.682 (4.42), 7.696 (5.14), 7.701 (5.14), 7.716 (3.19), 7.720 (3.37), 7.744 (7.32), 7.749 (8.68), 8.177 (1.00), 8.326 (13.50), 8.331 (13.46), 8.523 (4.37), 8.526 (4.96), 8.528 (4.96), 8.530 (4.56), 8.535 (4.39), 8.538 (5.15), 8.541 (4.54).

### Example 301

### 2-[6-amino-5-(trifluoromethoxy)pyridin-3-yl]-N-[(1R)-1-(2-cyanopyridin-4-yl)ethyl]-6,7-dihydrospiro[pyrazolo[5,1-c][1,4]oxazine-4,3'-pyrrolidine]-1'-carboxamide (mixture of stereoisomers)

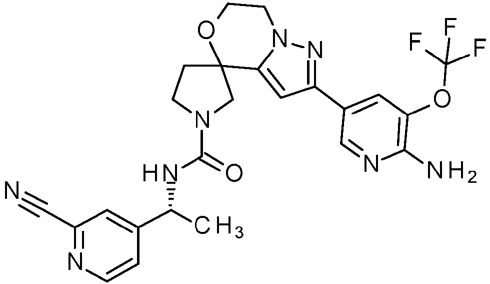

Example 301 was prepared in analogy to Example 31 using (*rac*)-5-[-6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 4-[(1R)-1-aminoethyl]pyridine-2-carbonitrile (37.3 mg, 253 µmol).
LC-MS (method 1): Rt = 0.95 min; MS (ESlneg): m/z = 527 [M-H]⁻
¹H-NMR (400 MHz, DMSO-d6) δ [ppm]: 1.065 (1.20), 1.140 (0.43), 1.157 (0.43), 1.169 (0.42), 1.186 (0.45), 1.231 (0.64), 1.374 (15.97), 1.392 (16.00), 2.074 (2.57), 2.083 (1.10), 2.228 (1.19), 2.253 (1.42), 2.322 (1.01), 2.327 (1.54), 2.332 (1.51), 2.337 (1.36), 2.351 (1.64), 2.518 (3.29), 2.523 (2.11), 2.665 (0.83), 2.669 (1.15), 2.673 (0.78), 2.727 (1.88), 2.888 (2.29), 3.355 (1.03), 3.378 (0.88), 3.391 (1.08), 3.408 (1.36), 3.417 (1.21), 3.427 (1.12), 3.444 (1.08), 3.461 (1.38), 3.491 (1.39), 3.521 (1.07), 3.551 (1.39), 3.584 (1.55), 3.606 (0.66), 3.641 (0.77), 3.663 (1.42), 3.686 (0.66), 3.772 (1.88), 3.800 (1.50), 3.841 (1.76), 3.868 (1.50), 4.053 (0.62), 4.066 (1.31), 4.083 (2.04), 4.098 (2.68), 4.119 (4.84), 4.126 (5.93), 4.138 (8.18), 4.840 (1.37), 4.858 (2.28), 4.872 (2.37), 4.889 (1.41), 6.558 (12.93), 6.673 (1.99), 6.692 (2.13), 6.712 (7.85), 6.722 (9.17), 7.675 (2.51), 7.680 (2.83), 7.682 (2.92), 7.687 (4.68), 7.693 (3.13), 7.695 (2.77), 7.700 (2.50), 7.744 (5.39), 7.748 (5.25), 8.003 (7.10), 8.320 (6.18), 8.325 (10.27), 8.331 (6.44), 8.672 (4.38), 8.674 (4.60), 8.677 (4.73), 8.678 (4.41), 8.685 (4.16), 8.687 (4.41), 8.689 (4.48).

### Example 302

### (rac)-2-[6-amino-5-(difluoromethoxy)pyridin-3-yl]-N-[2-(2-chlorophenyl)propan-2-yl]-6,7-dihydrospiro[pyrazolo[5,1-c][1,4]oxazine-4,3'-pyrrolidine]-1'-carboxamide

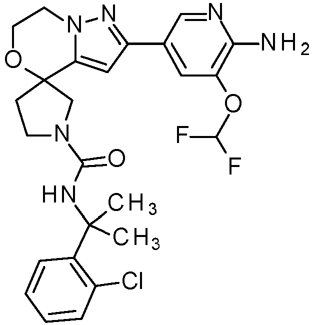

Example 302 was prepared in analogy to Example 31 using (*rac*)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and 2-(2-chlorophenyl)propan-2-amine-hydrochloride salt (49.6 mg, 241 µmol).
LC-MS (method 1): Rt = 1.12 min; MS (ESlpos): m/z = 533 [M+H]⁺
¹H-NMR (400 MHz, DMSO-d6) δ [ppm]: 1.065 (1.27), 1.225 (1.56), 1.241 (5.97), 1.257 (9.69), 1.274 (4.86), 1.679 (8.91), 1.701 (8.39), 2.206 (0.56), 2.230 (0.80), 2.253 (0.45), 2.295 (0.63), 2.318 (1.11), 2.322 (1.86), 2.327 (2.56), 2.331 (1.93), 2.518 (16.00), 2.523 (11.15), 2.659 (0.73), 2.665 (1.53), 2.669 (2.18), 2.673 (1.62), 2.678 (0.77), 2.729 (0.95), 2.888 (1.17), 3.124 (0.68), 3.134 (0.66), 3.142 (0.69), 3.153 (0.67), 3.377 (0.80), 3.395 (0.83), 3.420 (0.52), 3.440 (1.36), 3.469 (1.59), 3.540 (0.80), 3.561 (1.25), 3.586 (0.91), 3.594 (0.87), 3.603 (0.81), 3.610 (0.91), 3.620 (0.92), 3.627 (0.81), 3.636 (0.79), 3.750 (1.52), 4.054 (0.48), 4.069 (0.85), 4.087 (0.98), 4.118 (2.76), 4.130 (3.58), 6.152 (3.43), 6.683 (6.02), 6.969 (1.24), 7.039 (1.32), 7.097 (1.48), 7.167 (0.67), 7.171 (0.78), 7.186 (1.65), 7.189 (1.65), 7.205 (1.60), 7.208 (1.55), 7.223 (3.38), 7.235 (1.27), 7.239 (1.47), 7.255 (1.56), 7.258 (1.83), 7.273 (0.92), 7.277 (0.85), 7.298 (2.67), 7.303 (2.38), 7.318 (2.02), 7.322 (1.80), 7.407 (1.21), 7.455 (1.72), 7.459 (1.78), 7.474 (1.49), 7.478 (1.38), 7.697 (2.00), 8.183 (4.23), 8.187 (3.97).

### Example 303

### (rac)-2-[6-amino-5-(trifluoromethoxy)pyridin-3-yl]-N-[1-(2-chlorophenyl)cyclobutyl]-6,7-dihydrospiro[pyrazolo[5,1-c][1,4]oxazine-4,3'-pyrrolidine]-1'-carboxamide

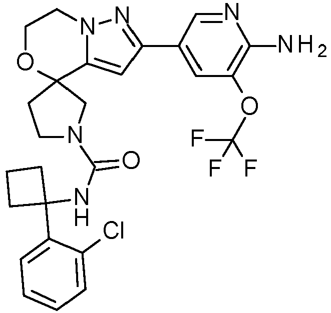

Example 303 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(2-chlorophenyl)cyclobutan-1-amine-hydrochloride salt (55.2 mg, 253 µmol).
LC-MS (method 1): Rt = 1.26 min; MS (ESlpos): m/z = 563 [M+H]⁺
¹H-NMR (400 MHz, DMSO-d6) δ [ppm]: 1.036 (8.66), 1.054 (16.00), 1.071 (8.66), 1.230 (0.91), 1.653 (0.48), 1.674 (1.53), 1.686 (1.12), 1.690 (1.16), 1.702 (1.24), 1.712 (0.91), 1.723 (0.53), 2.030 (0.92), 2.052 (1.64), 2.073 (2.44), 2.078 (1.57), 2.099 (0.97), 2.155 (0.40), 2.186 (0.97), 2.212 (1.32), 2.236 (0.72), 2.277 (1.23), 2.292 (1.44), 2.309 (0.84), 2.323 (0.96), 2.327 (1.10), 2.444 (0.57), 2.467 (1.33), 2.474 (1.58), 2.518 (3.55), 2.522 (2.52), 2.540 (1.07), 2.550 (1.50), 2.571 (0.68), 2.609 (1.15), 2.620 (1.64), 2.630 (2.31), 2.639 (2.36), 2.649 (2.29), 2.660 (1.95), 2.669 (1.49), 2.679 (0.83), 2.729 (0.58), 2.887 (0.74), 3.166 (0.60), 3.412 (2.61), 3.430 (3.24), 3.447 (3.44), 3.464 (1.05), 3.501 (1.07), 3.522 (1.86), 3.545 (0.84), 3.714 (1.90), 3.743 (1.62), 4.018 (0.80), 4.033 (1.31), 4.049 (2.13), 4.056 (1.40), 4.069 (1.83), 4.080 (2.65), 4.089 (2.62), 4.111 (6.58), 6.536 (7.31), 6.554 (10.29), 6.671 (9.18), 7.173 (1.35), 7.178 (1.48), 7.192 (3.78), 7.196 (3.67), 7.211 (3.53), 7.215 (3.40), 7.228 (2.64), 7.231 (3.40), 7.246 (3.78), 7.250 (4.25), 7.265 (1.83), 7.269 (1.57), 7.295 (5.65), 7.299 (5.27), 7.314 (3.74), 7.318 (3.49), 7.522 (3.71), 7.527 (3.68), 7.541 (3.40), 7.546 (3.13), 7.720 (3.80), 7.725 (5.12), 7.728 (3.62), 8.295 (7.79), 8.300 (7.49).

### Example 304

### (rac)-2-[6-amino-5-(trifluoromethoxy)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide

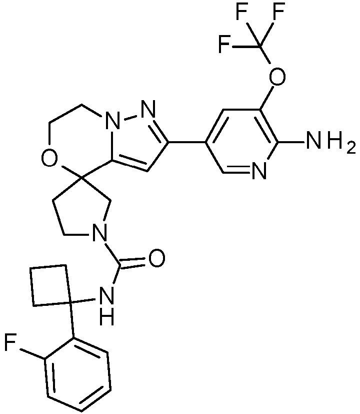

Example 304 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(2-fluorophenyl)cyclobutan-1-amine-hydrochloride salt (51.1 mg, 253 µmol).
LC-MS (method 1): Rt = 1.20 min; MS (ESlpos): m/z = 547 [M+H]⁺
¹H-NMR (400 MHz, DMSO-d6) δ [ppm]: 1.678 (1.45), 1.700 (1.42), 1.726 (0.46), 1.732 (0.46), 1.739 (0.48), 1.746 (0.42), 1.753 (0.49), 2.032 (0.55), 2.055 (0.56), 2.059 (0.69), 2.072 (16.00), 2.211 (0.55), 2.287 (0.56), 2.302 (0.67), 2.322 (0.53), 2.326 (0.50), 2.331 (0.50), 2.518 (2.84), 2.522 (2.60), 2.539 (2.61), 3.414 (1.28), 3.443 (1.25), 3.471 (0.43), 3.515 (0.53), 3.537 (0.94), 3.559 (0.56), 3.726 (0.91), 3.754 (0.81), 4.037 (0.52), 4.054 (0.81), 4.073 (0.84), 4.084 (1.08), 4.094 (1.22), 4.114 (2.88), 6.153 (0.56), 6.554 (4.41), 6.600 (2.36), 6.683 (3.84), 6.690 (1.46), 7.041 (0.65), 7.043 (0.72), 7.061 (1.91), 7.072 (1.78), 7.091 (2.96), 7.109 (1.18), 7.112 (0.92), 7.196 (0.46), 7.200 (0.57), 7.208 (0.60), 7.214 (0.78), 7.220 (0.65), 7.228 (0.62), 7.233 (0.88), 7.252 (0.52), 7.294 (0.46), 7.404 (0.65), 7.408 (0.66), 7.427 (1.06), 7.445 (0.64), 7.727 (1.51), 7.731 (2.06), 7.735 (1.64), 8.301 (2.95), 8.306 (3.02), 8.325 (0.72), 8.330 (0.70).

### Example 305

### (rac)-2-[6-amino-5-(trifluoromethoxy)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide

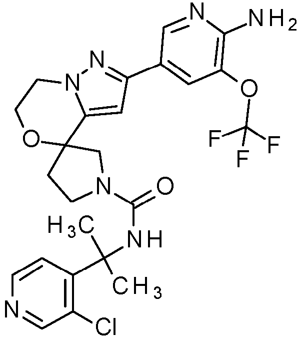

Example 305 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-(3-chloropyridin-4-yl)propan-2-amine (43.2 mg, 253 µmol).
LC-MS (method 1): Rt = 1.03 min; MS (ESlpos): m/z = 552 [M+H]⁺
¹H-NMR (400 MHz, DMSO-d6) δ [ppm]: 1.066 (1.27), 1.115 (0.44), 1.130 (0.58), 1.170 (2.00), 1.186 (2.09), 1.199 (2.26), 1.215 (2.79), 1.229 (2.54), 1.648 (16.00), 1.664 (15.30), 2.200 (0.95), 2.225 (1.32), 2.249 (0.70), 2.300 (1.06), 2.317 (1.51), 2.323 (1.55), 2.327 (1.79), 2.331 (1.56), 2.518 (9.13), 2.523 (6.29), 2.539 (1.91), 2.665 (0.87), 2.669 (1.12), 2.673 (0.83), 2.728 (0.68), 2.888 (0.82), 3.388 (1.32), 3.412 (0.71), 3.442 (1.74), 3.471 (2.05), 3.537 (0.90), 3.556 (1.55), 3.579 (0.73), 3.741 (1.76), 3.770 (1.50), 4.049 (0.65), 4.064 (1.30), 4.079 (1.61), 4.101 (2.42), 4.114 (4.46), 4.127 (6.52), 6.357 (5.88), 6.557 (8.40), 6.701 (10.85), 7.422 (4.73), 7.435 (4.82), 7.742 (3.34), 7.745 (4.39), 8.325 (6.79), 8.330 (6.65), 8.397 (6.78), 8.410 (6.60), 8.429 (13.24).

### Example 306

### (rac)-2'-[6-amino-5-(trifluoromethoxy)pyridin-3-yl]-N-[1-(pyridin-4-yl)cyclobutyl]-5',6'-dihydro-1H-spiro[pyrrolidine-3,4'-pyrrolo[1,2-b]pyrazole]-1-carboxamide acetate (1:1)

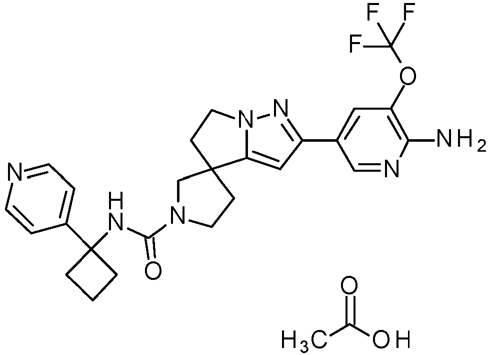

Example 306 was prepared in analogy to Example 31 using (*rac*)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and 1-(pyridin-4-yl)cyclobutan-1-amine (39.3 mg, 265 µmol).
LC-MS (method 2): Rt = 0.70 min; MS (ESlpos): m/z = 574 [M+H]⁺
1H-NMR (400 MHz, DMSO-d6) δ [ppm]: 1.136 (0.77), 1.153 (0.78), 1.820 (0.44), 1.825 (0.47), 1.842 (0.50), 1.847 (0.57), 1.999 (0.55), 2.014 (0.62), 2.027 (0.66), 2.043 (0.83), 2.058 (1.16), 2.075 (1.28), 2.083 (0.71), 2.092 (0.58), 2.344 (0.44), 2.375 (1.07), 2.390 (1.20), 2.412 (1.13), 2.442 (1.08), 2.463 (0.92), 2.522 (1.21), 2.530 (1.88), 2.534 (1.86), 2.550 (1.19), 3.415 (1.02), 3.527 (0.48), 3.544 (0.73), 4.166 (1.43), 4.183 (2.70), 4.201 (1.46), 4.287 (0.61), 5.756 (16.00), 6.407 (4.57), 6.513 (4.45), 6.872 (2.23), 7.392 (2.26), 7.407 (2.38), 7.747 (1.57), 7.751 (2.20), 7.755 (1.69), 8.143 (2.56), 8.326 (2.98), 8.331 (3.02), 8.463 (1.48), 8.473 (1.47).

### Example 307

### (rac)-2-[6-amino-5-(trifluoromethoxy)pyridin-3-yl]-N-[(1-methyl-2-oxabicyclo[2.1.1]hexan-4-yl)methyl]-6,7-dihydrospiro[pyrazolo[5,1-c][1,4]oxazine-4,3'-pyrrolidine]-1'-carboxamide

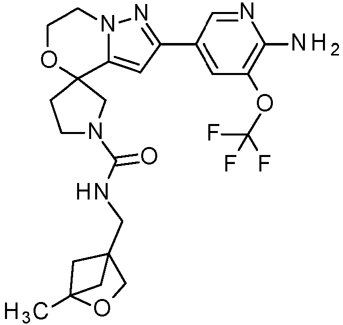

Example 307 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine-hydrochloride salt (75.0 mg, 191 µmol) and 1-(1-methyl-2-oxabicyclo[2.1.1]hexan-4-yl)methanamine (29.2 mg, 230 µmol).
LC-MS (method 1): Rt = 0.91 min; MS (ESlneg): m/z = 507 [M-H]⁻
¹H-NMR (400 MHz, DMSO-d6) δ [ppm]: 1.139 (0.68), 1.155 (0.69), 1.243 (0.66), 1.247 (0.45), 1.260 (0.97), 1.277 (0.69), 1.298 (16.00), 1.316 (0.49), 1.338 (2.67), 1.342 (2.72), 1.349 (3.07), 1.353 (3.10), 1.375 (0.40), 1.482 (2.70), 1.487 (2.29), 1.493 (2.42), 2.213 (0.51), 2.238 (0.71), 2.314 (0.61), 2.328 (0.87), 2.346 (0.45), 3.294 (0.42), 3.405 (0.88), 3.431 (1.79), 3.462 (10.58), 3.528 (0.65), 3.549 (1.12), 3.571 (0.50), 3.759 (1.20), 3.787 (1.03), 4.056 (0.66), 4.072 (0.94), 4.090 (0.99), 4.102 (1.11), 4.111 (1.52), 4.127 (3.14), 6.258 (0.68), 6.272 (1.35), 6.287 (0.67), 6.577 (1.32), 6.699 (5.78), 7.747 (1.85), 7.750 (2.45), 7.754 (1.72), 8.321 (3.77), 8.326 (3.67).

### Example 308

### (rac)-2-[6-amino-5-(trifluoromethoxy)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide

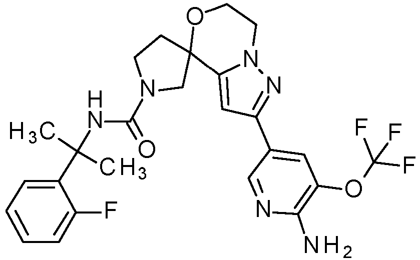

Example 308 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-(2-fluorophenyl)propan-2-amine-hydrochloride salt (48.0 mg, 253 µmol).
LC-MS (method 1): Rt = 1.16 min; MS (ESlpos): m/z = 535 [M+H]⁺
¹H-NMR (400 MHz, DMSO-d6) δ [ppm]: 1.240 (1.25), 1.255 (1.46), 1.270 (0.91), 1.628 (16.00), 1.635 (15.80), 2.074 (0.56), 2.084 (1.25), 2.219 (0.91), 2.245 (1.27), 2.268 (0.67), 2.323 (2.61), 2.327 (2.80), 2.523 (9.40), 2.665 (1.45), 2.669 (1.98), 2.728 (2.01), 2.888 (2.41), 3.401 (1.66), 3.468 (1.88), 3.497 (2.21), 3.563 (1.04), 3.585 (1.80), 3.771 (2.04), 3.801 (1.71), 4.063 (0.68), 4.077 (1.33), 4.094 (1.83), 4.124 (5.29), 4.135 (5.53), 6.117 (5.67), 6.561 (5.93), 6.714 (11.13), 7.035 (1.52), 7.055 (1.89), 7.067 (1.58), 7.075 (1.76), 7.079 (1.77), 7.087 (2.32), 7.094 (3.69), 7.113 (2.43), 7.116 (2.00), 7.195 (1.06), 7.209 (1.78), 7.228 (1.43), 7.246 (0.68), 7.299 (1.48), 7.320 (2.34), 7.340 (1.30), 7.743 (3.27), 7.747 (4.36), 8.324 (7.16), 8.328 (6.72).

### Example 309

### 2-[6-amino-5-(trifluoromethoxy)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide (mixture of stereoisomers)

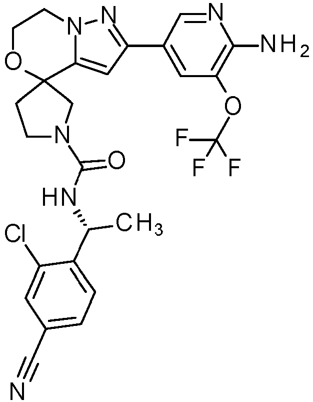

Example 309 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 4-[(1R)-1-aminoethyl]-3-chlorobenzonitrile (45.8 mg, 253 µmol).
LC-MS (method 1): Rt = 1.12 min; MS (ESlpos): m/z = 562 [M+H]⁺
¹H-NMR (400 MHz, DMSO-d6) δ [ppm]: 1.229 (0.72), 1.328 (15.71), 1.346 (16.00), 2.074 (1.81), 2.083 (1.79), 2.250 (1.97), 2.327 (3.37), 2.331 (3.11), 2.522 (14.82), 2.669 (2.81), 3.264 (0.54), 3.286 (0.95), 3.292 (1.06), 3.308 (2.04), 3.370 (2.52), 3.381 (2.00), 3.398 (2.22), 3.423 (1.92), 3.447 (1.93), 3.472 (1.67), 3.511 (1.44), 3.542 (1.66), 3.575 (1.63), 3.659 (1.58), 3.766 (1.92), 3.794 (1.70), 3.840 (2.10), 3.871 (1.82), 4.075 (2.82), 4.092 (3.04), 4.137 (9.91), 5.116 (1.88), 5.125 (2.12), 5.134 (2.86), 5.142 (3.05), 5.152 (1.98), 5.159 (1.99), 6.559 (14.75), 6.715 (8.19), 6.723 (8.72), 6.785 (2.33), 6.803 (2.35), 6.823 (2.36), 6.842 (2.17), 7.662 (4.48), 7.676 (4.70), 7.682 (5.83), 7.696 (5.37), 7.746 (5.09), 7.750 (4.93), 7.823 (3.56), 7.826 (3.78), 7.833 (4.03), 7.837 (4.05), 7.842 (2.91), 7.846 (3.02), 7.853 (2.98), 7.858 (2.89), 7.991 (11.46), 7.995 (10.64), 8.316 (6.42), 8.321 (6.50), 8.328 (7.05), 8.333 (6.68).

### Example 310

### 2'-[6-amino-5-(difluoromethoxy)pyridin-3-yl]-N-[2-(pyridin-2-yl)propan-2-yl]-5',6'-dihydrospiro[azetidine-3,4'-pyrrolo[1,2-b]pyrazole]-1-carboxamide

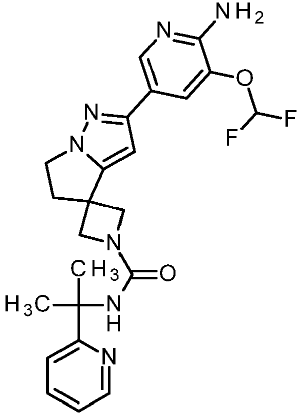

Example 310 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-(pyridin-2-yl)propan-2-amine (39.9 mg, 293 µmol).
LC-MS (method 1): Rt = 0.93 min; MS (ESlpos): m/z = 470 [M+H]⁺
¹H-NMR (400 MHz, DMSO-d6) δ [ppm]: 1.054 (0.50), 1.570 (16.00), 2.836 (1.12), 2.853 (1.86), 2.871 (1.22), 3.167 (0.55), 4.017 (0.72), 4.038 (5.03), 4.042 (5.02), 4.063 (0.73), 4.110 (1.26), 4.128 (1.99), 4.145 (1.21), 6.171 (3.30), 6.637 (6.27), 6.721 (2.81), 7.007 (1.32), 7.180 (0.88), 7.183 (0.95), 7.191 (3.21), 7.195 (1.27), 7.199 (1.06), 7.201 (0.97), 7.211 (0.94), 7.213 (0.94), 7.375 (1.20), 7.442 (1.57), 7.444 (0.96), 7.462 (1.76), 7.656 (1.80), 7.658 (1.80), 7.717 (0.89), 7.721 (0.90), 7.736 (1.06), 7.740 (1.11), 7.756 (0.72), 7.760 (0.70), 8.252 (3.11), 8.256 (2.95), 8.458 (0.96), 8.461 (1.08), 8.463 (1.13), 8.465 (1.01), 8.470 (1.01), 8.473 (1.13), 8.475 (1.07), 8.477 (0.94).

### Example 311

### 2'-[6-amino-5-(difluoromethoxy)pyridin-3-yl]-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide

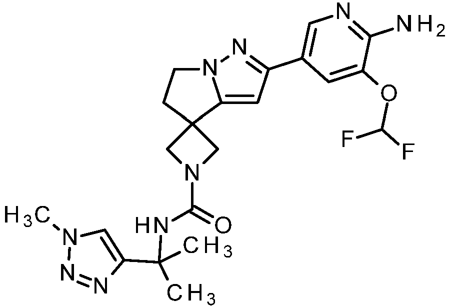

Example 311 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and 2-(1-methyl-1H-1,2,3-triazol-4-yl)propan-2-amine-hydrochloride salt (62.4 mg, 293 µmol).
LC-MS (method 1): Rt = 0.79 min; MS (ESlneg): m/z = 472 [M-H]⁻
¹H-NMR (400 MHz, DMSO-d6) δ [ppm]: 1.243 (0.45), 1.261 (0.57), 1.587 (15.68), 2.563 (0.69), 2.728 (1.30), 2.805 (1.38), 2.822 (2.43), 2.839 (1.50), 2.888 (1.44), 3.993 (16.00), 4.097 (1.51), 4.115 (2.50), 4.131 (1.49), 6.254 (1.27), 6.404 (2.75), 6.622 (3.96), 7.012 (0.96), 7.195 (1.92), 7.380 (0.90), 7.665 (2.37), 7.803 (4.05), 8.236 (2.46), 8.240 (2.59).

### Example 312

### 2-[6-amino-5-(trifluoromethoxy)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide (mixture of stereoisomers)

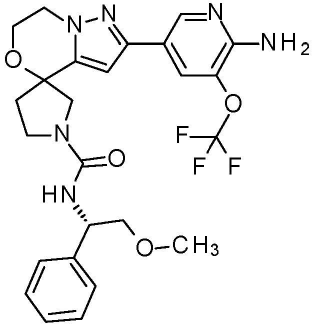

Example 312 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and (1S)-2-methoxy-1-phenylethan-1-amine (38.3 mg, 253 µmol).
LC-MS (method 1): Rt = 1.08 min; MS (ESlpos): m/z = 533 [M+H]⁺
¹H-NMR (400 MHz, DMSO-d6) δ [ppm]: 2.522 (0.95), 3.204 (16.00), 3.247 (0.72), 3.257 (6.57), 3.443 (0.52), 3.454 (2.98), 3.469 (2.70), 3.490 (0.49), 3.547 (0.48), 3.567 (0.65), 3.592 (0.49), 4.103 (0.41), 4.129 (0.99), 4.774 (0.78), 4.781 (0.48), 4.788 (0.49), 4.795 (0.80), 6.501 (0.42), 6.558 (1.47), 6.584 (1.21), 6.604 (1.17), 6.709 (1.41), 7.212 (0.51), 7.222 (0.79), 7.229 (1.38), 7.234 (0.72), 7.241 (0.75), 7.246 (0.88), 7.250 (0.67), 7.269 (1.27), 7.286 (3.73), 7.303 (3.24), 7.308 (1.59), 7.320 (2.60), 7.338 (0.97), 7.346 (1.36), 7.367 (0.61), 7.742 (0.68), 7.745 (0.68), 8.315 (0.60), 8.320 (1.14), 8.325 (0.66).

### Example 313

### 2-[6-amino-5-(trifluoromethoxy)pyridin-3-yl]-N-[(1S)-1-(3-chloropyridin-4-yl)ethyl]-6,7-dihydrospiro[pyrazolo[5,1-c][1,4]oxazine-4,3'-pyrrolidine]-1'-carboxamide (mixture of stereoisomers)

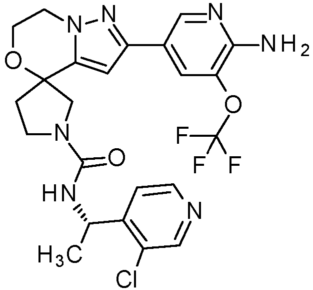

Example 313 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and (1S)-1-(3-chloropyridin-4-yl)ethan-1-amine-hydrochloride salt (48.9 mg, 253 µmol).
LC-MS (method 1): Rt = 0.99 min; MS (ESlpos): m/z = 538 [M+H]⁺
¹H-NMR (400 MHz, DMSO-d6) δ [ppm]: 1.035 (4.87), 1.052 (10.54), 1.065 (2.30), 1.070 (5.47), 1.130 (0.44), 1.154 (0.95), 1.170 (1.58), 1.185 (1.63), 1.199 (1.62), 1.215 (2.07), 1.227 (2.21), 1.336 (10.41), 1.351 (10.41), 1.354 (10.45), 1.646 (0.76), 1.752 (0.47), 2.228 (1.09), 2.254 (1.26), 2.322 (0.68), 2.326 (0.99), 2.331 (1.13), 2.349 (1.41), 2.518 (4.83), 2.522 (3.40), 2.539 (1.54), 2.664 (0.51), 2.668 (0.66), 2.673 (0.51), 3.165 (1.17), 3.393 (1.16), 3.410 (1.64), 3.429 (2.22), 3.447 (2.20), 3.461 (1.76), 3.489 (1.12), 3.526 (0.88), 3.564 (15.07), 3.587 (1.21), 3.611 (0.55), 3.649 (0.64), 3.671 (1.14), 3.694 (0.54), 3.775 (1.45), 3.804 (1.20), 3.842 (1.44), 3.871 (1.25), 4.049 (0.56), 4.063 (1.27), 4.080 (2.01), 4.098 (2.39), 4.120 (3.97), 4.134 (6.31), 4.140 (6.76), 4.365 (0.42), 5.046 (1.29), 5.054 (1.48), 5.064 (2.03), 5.072 (2.18), 5.081 (1.41), 5.090 (1.38), 5.758 (0.47), 6.563 (9.81), 6.715 (5.55), 6.723 (6.38), 6.785 (1.73), 6.803 (1.73), 6.821 (1.67), 6.839 (1.54), 7.483 (3.32), 7.497 (3.83), 7.500 (3.70), 7.513 (3.36), 7.743 (3.36), 7.749 (3.34), 7.754 (3.33), 8.320 (4.68), 8.324 (4.57), 8.330 (4.93), 8.335 (4.85), 8.481 (4.12), 8.492 (6.67), 8.504 (4.50), 8.533 (16.00).

### Example 314

### (rac)-2-[6-amino-5-(trifluoromethoxy)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide

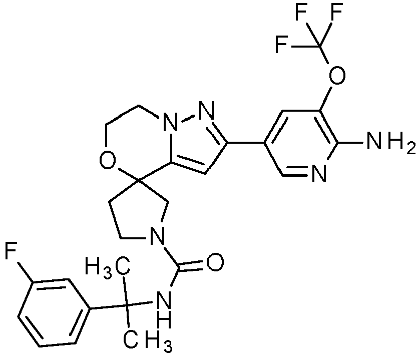

Example 314 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-(3-fluorophenyl)propan-2-amine (38.8 mg, 253 µmol).
LC-MS (method 1): Rt = 1.17 min; MS (ESlneg): m/z = 533 [M-H]⁻
¹H-NMR (400 MHz, DMSO-d6) δ [ppm]: 1.066 (5.27), 1.156 (3.40), 1.380 (0.51), 1.409 (0.72), 1.427 (0.70), 1.548 (16.00), 1.554 (15.66), 2.084 (0.92), 2.219 (0.77), 2.245 (1.03), 2.268 (0.58), 2.310 (0.90), 2.323 (1.56), 2.327 (1.93), 2.359 (0.52), 2.518 (3.63), 2.523 (2.51), 2.665 (0.66), 2.669 (0.91), 2.673 (0.64), 3.365 (0.62), 3.392 (0.90), 3.409 (0.90), 3.434 (0.45), 3.482 (1.14), 3.510 (1.33), 3.578 (0.76), 3.601 (1.25), 3.623 (0.59), 3.785 (1.52), 3.814 (1.29), 4.069 (0.45), 4.083 (1.01), 4.100 (1.73), 4.117 (3.56), 4.124 (4.93), 4.136 (4.76), 6.153 (4.85), 6.541 (1.32), 6.555 (6.83), 6.720 (9.24), 6.939 (0.85), 6.943 (0.94), 6.959 (1.67), 6.965 (1.83), 6.981 (0.90), 6.986 (1.00), 7.105 (1.27), 7.109 (1.71), 7.115 (1.36), 7.133 (1.24), 7.137 (1.73), 7.143 (1.34), 7.176 (1.93), 7.196 (2.53), 7.275 (1.52), 7.291 (1.71), 7.295 (2.37), 7.310 (2.26), 7.315 (1.18), 7.331 (0.97), 7.742 (2.72), 7.746 (3.74), 7.750 (2.55), 8.324 (5.97), 8.330 (5.94).

### Example 315

### (rac)-2'-[6-amino-5-(trifluoromethoxy)pyridin-3-yl]-N-[(1-methyl-2-oxabicyclo[2.1.1]hexan-4-yl)methyl]-5',6'-dihydrospiro[pyrrolidine-3,4'-pyrrolo[1,2-b]pyrazole]-1-carboxamide

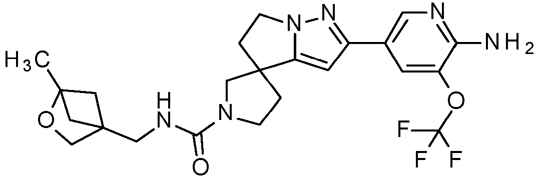

Example 315 was prepared in analogy to Example 31 using (*rac*)-5-[5',6'-dihydrospiro[pyrrolidine-3,4'-pyrrolo[1,2-b]pyrazol]-2'-yl]-3-(trifluoromethoxy)pyridin-2-amine-hydrochloride salt (75.0 mg, 200 µmol) and 1-(1-methyl-2-oxabicyclo[2.1.1]hexan-4-yl)methanamine (30.5 mg, 239 µmol).
LC-MS (method 1): Rt = 0.91 min; MS (ESlneg): m/z = 491 [M-H]⁻
¹H-NMR (400 MHz, DMSO-d6) δ [ppm]: 1.138 (0.52), 1.155 (0.52), 1.289 (9.42), 1.337 (1.48), 1.341 (1.50), 1.347 (1.68), 1.351 (1.73), 1.475 (0.86), 1.486 (1.42), 1.497 (0.77), 2.041 (0.43), 2.055 (0.70), 2.067 (0.85), 2.083 (1.12), 2.518 (1.67), 2.522 (1.51), 2.537 (0.86), 2.727 (1.14), 2.888 (1.40), 3.340 (16.00), 3.348 (2.04), 3.390 (0.49), 3.398 (0.43), 3.433 (0.42), 3.464 (5.17), 3.501 (0.50), 4.160 (0.92), 4.178 (1.67), 4.195 (0.91), 6.257 (0.78), 6.388 (3.62), 6.502 (2.57), 7.742 (0.98), 7.746 (1.32), 7.750 (0.92), 8.322 (2.08), 8.327 (2.09).

### Example 316

### 2'-[6-amino-5-(difluoromethoxy)pyridin-3-yl]-N-[(1R)-1-phenylpropyl]-5',6'-dihydrospiro[azetidine-3,4'-pyrrolo[1,2-b]pyrazole]-1-carboxamide

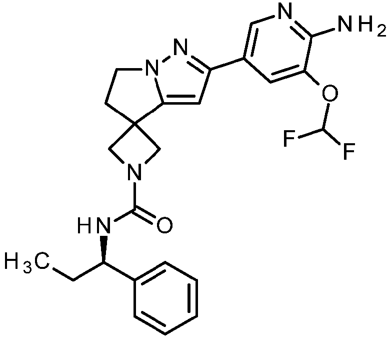

Example 316 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and (1R)-1-phenylpropan-1-amine (39.6 mg, 293 µmol).
LC-MS (method 1): Rt = 1.07 min; MS (ESlpos): m/z = 469 [M+H]⁺
¹H-NMR (400 MHz, DMSO-d6) δ [ppm]: 0.846 (4.06), 0.864 (9.69), 0.882 (4.38), 1.644 (0.63), 1.662 (1.48), 1.672 (1.31), 1.679 (1.90), 1.693 (1.73), 1.711 (1.22), 1.727 (0.42), 2.729 (0.60), 2.817 (2.17), 2.835 (3.69), 2.852 (2.33), 2.886 (0.75), 3.986 (1.69), 4.007 (4.58), 4.015 (9.02), 4.027 (4.19), 4.048 (1.69), 4.097 (2.39), 4.114 (3.86), 4.131 (2.30), 4.512 (0.67), 4.534 (1.61), 4.550 (1.42), 4.572 (0.65), 6.162 (6.45), 6.609 (10.00), 6.814 (2.55), 6.836 (2.46), 6.997 (2.25), 7.181 (4.73), 7.192 (0.90), 7.199 (1.13), 7.206 (1.62), 7.214 (1.42), 7.221 (1.41), 7.228 (1.01), 7.286 (1.01), 7.291 (0.61), 7.307 (6.85), 7.312 (7.39), 7.320 (16.00), 7.333 (0.94), 7.366 (2.10), 7.642 (3.68), 8.232 (5.13), 8.236 (5.17).

### Example 317

### 2'-[6-amino-5-(trifluoromethoxy)pyridin-3-yl]-N-[(1S)-2-methoxy-1-phenylethyl]-5',6'-dihydrospiro[pyrrolidine-3,4'-pyrrolo[1,2-b]pyrazole]-1-carboxamide (mixture of stereoisomers)

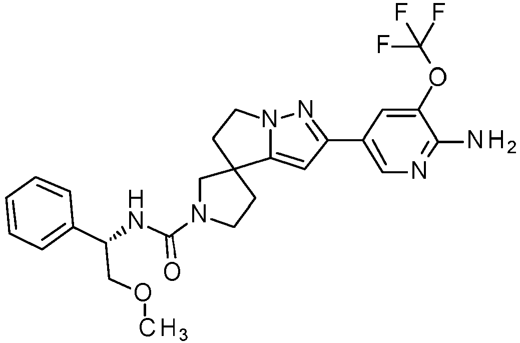

Example 317 was prepared in analogy to Example 31 using (*rac*)-5-[5',6'-dihydrospiro[pyrrolidine-3,4'-pyrrolo[1,2-b]pyrazol]-2'-yl]-3-(trifluoromethoxy)pyridin-2-amine-hydrochloride salt (75.0 mg, 200 µmol) and (1S)-2-methoxy-1-phenylethan-1-amine (36.2 mg, 239 µmol).
LC-MS (method 1): Rt = 1.06 min; MS (ESlpos): m/z = 517 [M+H]⁺
¹H-NMR (400 MHz, DMSO-d6) δ [ppm]: 1.017 (0.62), 1.029 (0.88), 1.033 (0.85), 1.047 (0.89), 2.062 (1.86), 2.073 (2.85), 2.083 (4.54), 2.534 (3.74), 2.551 (2.36), 3.204 (5.09), 3.223 (0.48), 3.245 (15.36), 3.259 (16.00), 3.425 (1.51), 3.433 (1.48), 3.450 (3.65), 3.461 (5.26), 3.468 (4.00), 3.479 (2.63), 3.504 (0.87), 3.517 (0.43), 3.535 (0.75), 3.547 (1.53), 3.556 (1.55), 3.567 (1.89), 3.571 (1.76), 3.576 (1.88), 3.592 (1.21), 3.601 (0.88), 4.163 (2.08), 4.181 (3.69), 4.198 (2.05), 4.930 (0.62), 4.950 (1.42), 4.964 (1.44), 4.985 (0.67), 6.395 (7.26), 6.459 (1.17), 6.469 (1.36), 6.480 (1.33), 6.490 (1.52), 6.507 (6.48), 7.186 (0.50), 7.203 (1.69), 7.221 (2.02), 7.225 (1.25), 7.234 (0.82), 7.238 (1.09), 7.241 (0.76), 7.266 (1.63), 7.286 (4.07), 7.303 (3.42), 7.307 (3.59), 7.320 (1.68), 7.325 (2.08), 7.337 (3.24), 7.359 (3.52), 7.379 (1.59), 7.743 (2.80), 7.747 (2.90), 8.315 (2.67), 8.318 (4.06), 8.322 (3.05).

### Example 318

### 2'-[6-amino-5-(difluoromethoxy)pyridin-3-yl]-N-[(1R)-1-(3-fluorophenyl)ethyl]-5',6'-dihydrospiro[azetidine-3,4'-pyrrolo[1,2-b]pyrazole]-1-carboxamide

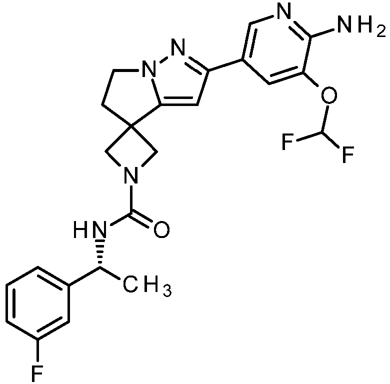

Example 318 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine (75.0 mg, 244 µmol) and (1R)-1-(3-fluorophenyl)ethan-1-amine (40.8 mg, 293 µmol).
LC-MS (method 1): Rt = 1.03 min; MS (ESlpos): m/z = 473 [M+H]⁺
¹H-NMR (400 MHz, DMSO-d6) δ [ppm]: 1.234 (0.70), 1.245 (1.50), 1.253 (0.98), 1.261 (1.60), 1.266 (1.57), 1.271 (0.84), 1.283 (1.78), 1.302 (0.59), 1.347 (13.14), 1.365 (12.92), 2.074 (1.13), 2.518 (3.12), 2.523 (2.00), 2.534 (0.76), 2.539 (0.52), 2.727 (0.68), 2.828 (3.28), 2.846 (5.39), 2.863 (3.57), 2.888 (0.91), 3.824 (0.66), 3.998 (2.89), 4.019 (6.84), 4.027 (13.50), 4.046 (5.58), 4.066 (2.83), 4.103 (3.60), 4.121 (5.81), 4.137 (3.47), 4.782 (0.43), 4.800 (1.69), 4.820 (2.31), 4.838 (1.68), 4.856 (0.43), 6.285 (1.77), 6.436 (0.48), 6.637 (16.00), 6.680 (0.57), 6.918 (3.90), 6.939 (3.80), 7.011 (6.21), 7.031 (2.30), 7.038 (2.60), 7.054 (1.33), 7.058 (1.50), 7.060 (1.42), 7.138 (1.96), 7.148 (2.36), 7.154 (2.11), 7.164 (3.37), 7.175 (2.69), 7.181 (5.39), 7.195 (7.31), 7.204 (0.53), 7.266 (1.64), 7.330 (2.19), 7.345 (2.33), 7.350 (3.10), 7.366 (3.52), 7.369 (1.85), 7.379 (3.34), 7.386 (1.70), 7.667 (4.96), 7.695 (0.47), 7.698 (0.45), 8.228 (8.68), 8.233 (8.72).

### Example 319

### 2'-[6-amino-5-(trifluoromethoxy)pyridin-3-yl]-N-[(1S)-1-(pyridin-3-yl)ethyl]-5',6'-dihydrospiro[pyrrolidine-3,4'-pyrrolo[1,2-b]pyrazole]-1-carboxamide (mixture of stereoisomers)

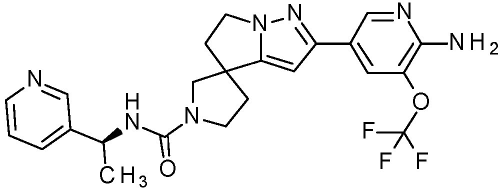

Example 319 was prepared in analogy to Example 31 using (*rac*)-5-[5',6'-dihydrospiro[pyrrolidine-3,4'-pyrrolo[1,2-b]pyrazol]-2'-yl]-3-(trifluoromethoxy)pyridin-2-amine (75.0 mg, 221 µmol) and (1S)-1-(pyridin-3-yl)ethan-1-amine-hydrochloride salt (51.7 mg, 265 µmol).
LC-MS (method 1): Rt = 0.61 min; MS (ESlpos): m/z = 488 [M+H]⁺
¹H-NMR (400 MHz, DMSO-d6) δ [ppm]: 1.166 (0.50), 1.232 (1.15), 1.388 (12.62), 1.395 (12.76), 1.406 (13.14), 1.413 (12.34), 2.045 (2.96), 2.064 (4.60), 2.072 (4.54), 2.518 (8.38), 2.523 (7.14), 2.529 (9.66), 2.539 (2.61), 2.547 (6.32), 3.403 (1.66), 3.422 (3.89), 3.428 (2.60), 3.447 (9.62), 3.454 (12.05), 3.461 (8.77), 3.480 (1.70), 3.487 (1.84), 3.506 (1.13), 3.525 (1.87), 3.541 (1.86), 3.560 (1.92), 3.574 (1.28), 3.586 (1.16), 3.601 (0.65), 4.161 (5.68), 4.178 (9.95), 4.195 (5.41), 4.851 (1.86), 4.856 (2.09), 4.875 (2.94), 4.888 (2.04), 4.893 (1.89), 6.395 (7.43), 6.411 (14.61), 6.502 (16.00), 6.563 (3.27), 6.573 (3.40), 6.582 (3.38), 6.593 (2.98), 7.293 (2.14), 7.305 (2.32), 7.312 (4.65), 7.324 (4.74), 7.332 (2.67), 7.344 (2.54), 7.712 (1.71), 7.717 (2.83), 7.721 (1.85), 7.732 (1.75), 7.753 (8.44), 7.770 (1.74), 8.321 (8.40), 8.324 (10.53), 8.329 (8.74), 8.392 (3.69), 8.396 (3.99), 8.404 (6.53), 8.408 (6.26), 8.417 (3.90), 8.421 (3.66), 8.540 (4.53), 8.546 (4.61), 8.560 (4.68), 8.565 (4.64).

### Example 320

### (rac)-2'-[6-amino-5-(trifluoromethoxy)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide

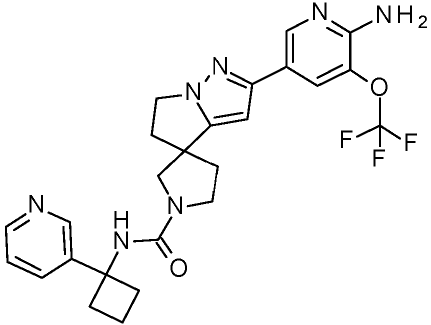

Example 320 was prepared in analogy to Example 31 using (*rac*)-5-[5',6'-dihydrospiro[pyrrolidine-3,4'-pyrrolo[1,2-b]pyrazol]-2'-yl]-3-(trifluoromethoxy)pyridin-2-amine-hydrochloride salt (75.0 mg, 200 µmol) and 1-(pyridin-3-yl)cyclobutan-1-amine (35.5 mg, 239 µmol).
LC-MS (method 1): Rt = 0.99 min; MS (ESlpos): m/z = 514 [M+H]⁺
¹H-NMR (400 MHz, DMSO-d6) δ [ppm]: 0.852 (0.50), 0.917 (0.50), 0.932 (4.03), 0.935 (1.31), 0.948 (4.08), 0.953 (0.68), 1.232 (1.89), 1.765 (0.67), 1.787 (1.32), 1.793 (1.33), 1.815 (1.80), 1.832 (1.08), 1.854 (0.41), 1.967 (0.45), 1.989 (1.03), 2.004 (1.96), 2.011 (1.50), 2.020 (1.72), 2.032 (2.92), 2.047 (3.56), 2.062 (3.52), 2.078 (1.48), 2.318 (1.22), 2.397 (0.98), 2.405 (0.89), 2.426 (3.03), 2.447 (4.76), 2.465 (5.11), 2.518 (16.00), 2.523 (13.98), 2.539 (4.27), 2.660 (1.24), 3.358 (0.69), 3.399 (2.47), 3.425 (6.69), 3.435 (4.99), 3.461 (1.08), 3.505 (0.96), 3.523 (1.62), 3.564 (0.60), 4.159 (4.18), 4.177 (7.44), 4.194 (4.01), 6.376 (13.65), 6.507 (12.00), 6.828 (6.88), 7.291 (3.22), 7.293 (3.23), 7.302 (3.46), 7.304 (3.41), 7.310 (3.59), 7.312 (3.51), 7.322 (3.48), 7.324 (3.41), 7.746 (4.59), 7.750 (6.21), 7.754 (4.22), 7.782 (2.69), 7.785 (3.53), 7.792 (2.77), 7.801 (2.61), 7.807 (3.16), 7.811 (2.52), 8.319 (10.62), 8.324 (10.00), 8.364 (6.28), 8.368 (6.01), 8.376 (5.93), 8.380 (5.66), 8.640 (6.22), 8.642 (6.52), 8.646 (6.44).

### Example 321

### (rac)-2'-[6-amino-5-(trifluoromethoxy)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide

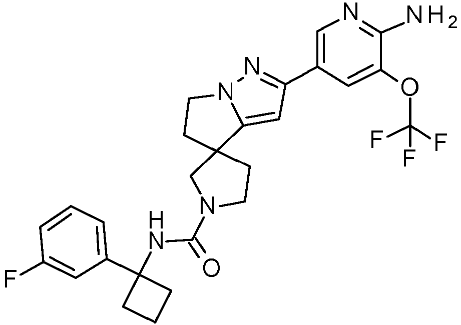

Example 321 was prepared in analogy to Example 31 using (*rac*)-5-[5',6'-dihydrospiro[pyrrolidine-3,4'-pyrrolo[1,2-b]pyrazol]-2'-yl]-3-(trifluoromethoxy)pyridin-2-amine-hydrochloride salt (75.0 mg, 200 µmol) and 1-(3-fluorophenyl)cyclobutan-1-amine (39.6 mg, 239 µmol).
LC-MS (method 1): Rt = 1.18 min; MS (ESlpos): m/z = 531 [M+H]⁺
¹H-NMR (400 MHz, DMSO-d6) δ [ppm]: 0.937 (0.83), 0.953 (0.81), 1.759 (0.71), 1.776 (1.37), 1.781 (1.47), 1.798 (1.49), 1.803 (1.67), 1.820 (1.04), 1.842 (0.40), 1.966 (0.92), 1.981 (1.66), 1.997 (1.29), 2.004 (1.73), 2.009 (1.63), 2.020 (1.42), 2.033 (2.06), 2.049 (3.37), 2.065 (3.67), 2.084 (1.94), 2.337 (0.91), 2.349 (1.33), 2.380 (3.18), 2.395 (3.79), 2.418 (2.71), 2.432 (2.17), 2.449 (3.62), 2.518 (10.18), 2.523 (10.77), 2.543 (4.01), 2.674 (1.66), 2.678 (0.75), 2.728 (0.55), 2.889 (0.65), 3.383 (0.81), 3.402 (2.86), 3.427 (7.30), 3.439 (5.27), 3.464 (1.34), 3.511 (1.00), 3.528 (1.81), 3.542 (1.39), 3.551 (1.30), 3.570 (0.66), 4.162 (4.49), 4.180 (8.11), 4.198 (4.30), 6.374 (16.00), 6.510 (12.97), 6.763 (7.58), 6.952 (1.39), 6.956 (1.48), 6.959 (1.70), 6.962 (1.70), 6.975 (2.89), 6.978 (2.66), 6.982 (3.15), 6.995 (1.60), 6.997 (1.62), 7.001 (1.84), 7.004 (1.74), 7.164 (2.38), 7.169 (2.91), 7.174 (2.50), 7.192 (2.36), 7.196 (3.04), 7.202 (2.43), 7.259 (3.03), 7.279 (5.76), 7.300 (3.36), 7.315 (3.48), 7.320 (4.42), 7.335 (4.34), 7.339 (1.80), 7.355 (1.66), 7.731 (4.97), 7.736 (6.64), 7.740 (4.59), 8.221 (0.44), 8.227 (0.47), 8.310 (11.25), 8.315 (11.23).

### Example 322

### 2'-[6-amino-5-(difluoromethoxy)pyridin-3-yl]-N-[1-(propan-2-yl)cyclobutyl]-5',6'-dihydrospiro[azetidine-3,4'-pyrrolo[1,2-b]pyrazole]-1-carboxamide

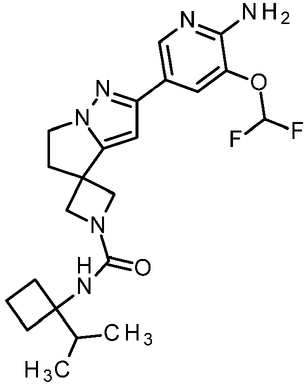

Example 322 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-(propan-2-yl)cyclobutan-1-amine (29.6 mg, 262 µmol).
LC-MS (method 1): Rt = 1.06 min; MS (ESlneg): m/z = 445 [M-H]⁻
¹H-NMR (400 MHz, DMSO-d6) δ [ppm]: 0.843 (15.63), 0.860 (16.00), 1.066 (0.63), 1.363 (0.59), 1.587 (0.67), 1.611 (0.76), 1.630 (0.49), 1.805 (0.73), 1.820 (0.67), 1.830 (0.70), 1.844 (0.56), 2.014 (0.62), 2.045 (1.57), 2.059 (2.02), 2.068 (1.56), 2.076 (1.95), 2.084 (2.17), 2.092 (2.13), 2.109 (2.88), 2.128 (1.60), 2.159 (0.48), 2.523 (2.41), 2.810 (1.99), 2.827 (3.32), 2.845 (2.10), 2.888 (0.46), 3.299 (0.52), 3.965 (14.44), 3.986 (0.75), 4.097 (2.24), 4.114 (3.57), 4.131 (2.14), 5.767 (0.42), 6.159 (5.79), 6.186 (0.50), 6.209 (4.12), 6.490 (0.41), 6.623 (9.10), 7.000 (2.17), 7.184 (4.25), 7.368 (1.87), 7.547 (0.47), 7.565 (0.52), 7.573 (0.44), 7.596 (0.68), 7.615 (0.63), 7.622 (0.83), 7.649 (3.27), 8.243 (4.54), 8.248 (4.56).

### Example 323

### 2'-[6-amino-5-(difluoromethoxy)pyridin-3-yl]-N-[1-(pyridin-4-yl)cyclobutyl]-5',6'-dihydrospiro[azetidine-3,4'-pyrrolo[1,2-b]pyrazole]-1-carboxamide

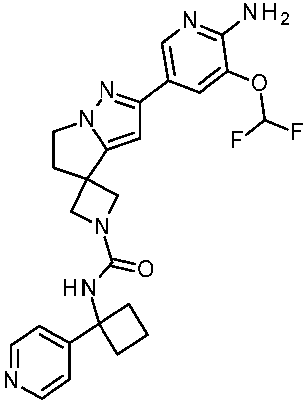

Example 323 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-(pyridin-4-yl)cyclobutan-1-amine (38.8 mg, 262 µmol).
LC-MS (method 1): Rt = 0.86 min; MS (ESlpos): m/z = 482 [M+H]⁺
¹H-NMR (400 MHz, DMSO-d6) δ [ppm]: 0.852 (0.58), 0.922 (0.46), 0.937 (3.45), 0.953 (3.33), 1.014 (2.27), 1.032 (2.28), 1.056 (1.05), 1.073 (0.44), 1.232 (2.04), 1.812 (0.50), 1.829 (0.88), 1.835 (0.96), 1.858 (1.10), 1.875 (0.68), 1.985 (0.53), 2.004 (0.91), 2.020 (1.12), 2.033 (0.72), 2.043 (0.90), 2.048 (0.86), 2.059 (0.50), 2.071 (0.46), 2.337 (1.68), 2.357 (0.95), 2.387 (2.17), 2.403 (2.60), 2.409 (2.72), 2.416 (2.78), 2.425 (2.51), 2.434 (2.98), 2.439 (3.08), 2.457 (2.87), 2.518 (16.00), 2.523 (10.44), 2.678 (1.47), 2.829 (2.34), 2.846 (3.80), 2.863 (2.44), 3.821 (1.14), 3.996 (1.75), 4.016 (8.37), 4.023 (8.20), 4.044 (1.76), 4.102 (2.68), 4.120 (4.15), 4.137 (2.67), 6.169 (6.80), 6.421 (1.54), 6.628 (13.09), 6.863 (0.61), 7.003 (2.91), 7.010 (0.45), 7.188 (6.13), 7.194 (0.98), 7.244 (5.61), 7.312 (1.00), 7.315 (0.67), 7.323 (0.63), 7.327 (1.08), 7.340 (0.73), 7.344 (0.46), 7.351 (0.46), 7.355 (0.77), 7.372 (2.64), 7.379 (0.55), 7.393 (7.92), 7.396 (4.89), 7.403 (4.71), 7.408 (8.09), 7.646 (3.71), 7.649 (3.69), 8.215 (0.82), 8.220 (0.80), 8.244 (6.62), 8.248 (6.66), 8.432 (1.13), 8.437 (0.71), 8.444 (0.63), 8.448 (1.02), 8.455 (0.75), 8.460 (0.49), 8.467 (0.50), 8.470 (0.76), 8.501 (8.04), 8.506 (5.02), 8.513 (4.82), 8.517 (7.74).

### Example 324

### 2'-[6-amino-5-(difluoromethoxy)pyridin-3-yl]-N-(bicyclo[2.2.1]heptan-1-yl)-5',6'-dihydrospiro[azetidine-3,4'-pyrrolo[1,2-b]pyrazole]-1-carboxamide

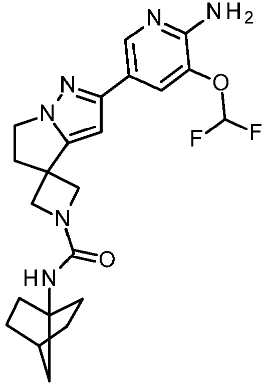

Example 324 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and bicyclo[2.2.1]heptan-1-amine-hydrochloride salt (38.7 mg, 262 µmol).
LC-MS (method 1): Rt = 1.03 min; MS (ESlneg): m/z = 443 [M-H]⁻
¹H-NMR (400 MHz, DMSO-d6) δ [ppm]: 0.936 (0.50), 0.953 (0.48), 1.278 (0.91), 1.300 (1.82), 1.319 (1.01), 1.329 (0.97), 1.544 (6.46), 1.588 (1.41), 1.600 (0.94), 1.638 (1.50), 1.664 (1.38), 1.683 (1.54), 1.696 (1.10), 1.716 (1.41), 1.737 (0.50), 2.057 (1.77), 2.084 (3.29), 2.518 (2.03), 2.522 (1.33), 2.802 (2.22), 2.820 (3.58), 2.837 (2.39), 3.950 (0.82), 3.970 (16.00), 3.992 (0.81), 4.093 (2.42), 4.111 (3.75), 4.128 (2.32), 5.770 (0.62), 6.159 (6.18), 6.438 (5.14), 6.623 (12.99), 6.996 (2.73), 7.180 (5.78), 7.364 (2.40), 7.638 (3.32), 7.640 (3.39), 7.642 (3.34), 8.233 (5.98), 8.237 (6.25).

### Example 325

### 2'-[6-amino-5-(difluoromethoxy)pyridin-3-yl]-N-(1-benzylcyclobutyl)-5',6'-dihydrospiro[azetidine-3,4'-pyrrolo[1,2-b]pyrazole]-1-carboxamide

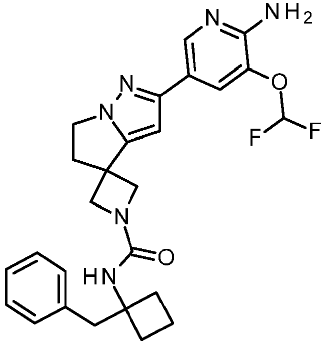

Example 325 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-benzylcyclobutan-1-amine-hydrochloride salt (51.8 mg, 262 µmol).
LC-MS (method 1): Rt = 1.11 min; MS (ESlpos): m/z = 495 [M+H]⁺
¹H-NMR (400 MHz, DMSO-d6) δ [ppm]: 1.232 (1.07), 1.753 (1.09), 1.774 (2.36), 1.793 (2.34), 1.809 (1.35), 1.827 (0.56), 2.042 (5.80), 2.059 (8.35), 2.080 (4.23), 2.084 (5.27), 2.332 (1.74), 2.336 (0.79), 2.518 (9.48), 2.522 (5.91), 2.673 (1.83), 2.678 (0.84), 2.817 (2.64), 2.834 (4.28), 2.852 (2.79), 3.079 (9.51), 3.910 (1.26), 3.933 (16.00), 3.954 (1.28), 4.103 (2.87), 4.121 (4.53), 4.138 (2.77), 6.172 (7.77), 6.274 (6.19), 6.617 (15.13), 7.004 (3.18), 7.161 (4.73), 7.178 (5.94), 7.181 (5.47), 7.188 (7.88), 7.202 (1.14), 7.207 (3.38), 7.223 (1.61), 7.226 (2.24), 7.291 (4.90), 7.310 (6.65), 7.328 (2.59), 7.373 (2.84), 7.656 (4.26), 7.658 (4.25), 8.259 (7.24), 8.264 (7.69).

### Example 326

### 2'-[6-amino-5-(difluoromethoxy)pyridin-3-yl]-N-[1-(4-chlorophenyl)cyclobutyl]-5',6'-dihydrospiro[azetidine-3,4'-pyrrolo[1,2-b]pyrazole]-1-carboxamide

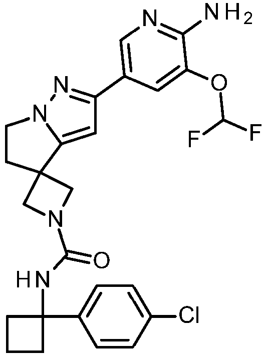

Example 326 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-(4-chlorophenyl)cyclobutan-1-amine (47.6 mg, 262 µmol).
LC-MS (method 1): Rt = 1.14 min; MS (ESlpos): m/z = 515 [M+H]⁺
¹H-NMR (400 MHz, DMSO-d6) δ [ppm]: 1.137 (1.41), 1.232 (0.79), 1.743 (0.54), 1.760 (1.02), 1.765 (1.09), 1.782 (1.09), 1.787 (1.24), 1.804 (0.76), 1.978 (0.67), 1.993 (1.22), 2.005 (0.89), 2.016 (1.12), 2.021 (1.10), 2.043 (0.53), 2.084 (7.02), 2.116 (0.62), 2.344 (1.02), 2.366 (1.76), 2.373 (2.33), 2.390 (2.81), 2.396 (2.45), 2.411 (1.80), 2.428 (2.04), 2.444 (2.59), 2.450 (3.03), 2.457 (2.19), 2.467 (3.01), 2.518 (6.34), 2.523 (3.97), 2.812 (3.00), 2.830 (5.02), 2.847 (3.16), 3.964 (2.11), 3.984 (12.26), 3.990 (12.20), 4.011 (2.05), 4.095 (3.32), 4.112 (5.30), 4.130 (3.19), 5.768 (1.01), 6.164 (8.92), 6.612 (16.00), 6.998 (3.48), 7.122 (7.21), 7.183 (7.27), 7.356 (6.83), 7.362 (2.72), 7.367 (3.67), 7.373 (3.60), 7.378 (13.64), 7.384 (2.10), 7.415 (2.04), 7.421 (13.44), 7.426 (3.48), 7.437 (2.51), 7.443 (6.35), 7.643 (5.02), 8.238 (7.68), 8.242 (7.87).

### Example 327

### 2'-[6-amino-5-(difluoromethoxy)pyridin-3-yl]-N-(1-methylcyclobutyl)-5',6'-dihydrospiro[azetidine-3,4'-pyrrolo[1,2-b]pyrazole]-1-carboxamide

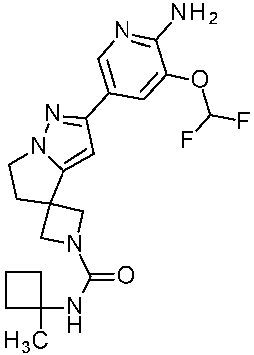

Example 327 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-methylcyclobutan-1-amine-hydrochloride salt (31.8 mg, 262 µmol).
LC-MS (method 1): Rt = 0.93 min; MS (ESIneg): m/z = 417 [M-H]⁻
¹H-NMR (400 MHz, DMSO-d6) δ [ppm]: 1.066 (3.40), 1.297 (6.75), 1.363 (14.56), 1.661 (0.44), 1.680 (1.10), 1.691 (0.79), 1.702 (1.44), 1.708 (1.21), 1.725 (1.87), 1.729 (1.78), 1.735 (1.58), 1.748 (2.62), 1.756 (1.33), 1.762 (0.99), 1.770 (1.50), 1.786 (0.78), 1.794 (0.55), 1.812 (1.09), 1.817 (1.06), 1.822 (1.18), 1.830 (1.29), 1.837 (1.43), 1.841 (1.72), 1.854 (1.51), 1.860 (1.14), 1.873 (0.67), 2.209 (1.01), 2.231 (2.71), 2.254 (2.25), 2.260 (1.89), 2.276 (0.64), 2.283 (0.74), 2.523 (0.66), 2.729 (0.44), 2.805 (2.12), 2.823 (3.54), 2.840 (2.36), 2.888 (0.57), 3.939 (1.33), 3.959 (16.00), 3.980 (0.87), 4.094 (2.31), 4.112 (3.68), 4.129 (2.26), 5.702 (1.07), 5.770 (0.57), 6.159 (5.89), 6.382 (4.70), 6.627 (10.15), 6.998 (2.50), 7.182 (4.60), 7.366 (2.00), 7.644 (3.26), 7.646 (3.19), 8.237 (5.00), 8.242 (5.03).

### Example 328

### (rac)-2-[6-amino-5-(trifluoromethoxy)pyridin-3-yl]-N-[1-(1,3,4-trimethyl-1H-pyrazol-5-yl)ethyl]-6,7-dihydrospiro[pyrazolo[5,1-c][1,4]oxazine-4,3'-pyrrolidine]-1'-carboxamide

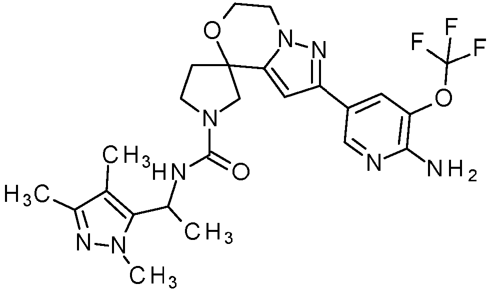

Example 328 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 1-(1,3,4-trimethyl-1H-pyrazol-5-yl)ethan-1-amine (38.8 mg, 253 µmol).
LC-MS (method 1): Rt = 0.96 min; MS (ESlpos): m/z = 535 [M+H]⁺
¹H-NMR (400 MHz, DMSO-d6) δ [ppm]: 1.389 (3.36), 1.394 (3.61), 1.408 (3.52), 1.413 (3.55), 1.926 (0.44), 1.941 (8.44), 1.947 (8.58), 1.974 (10.98), 2.084 (2.24), 2.202 (0.48), 2.228 (0.64), 2.307 (0.51), 2.323 (0.75), 2.518 (0.92), 2.523 (0.58), 3.359 (0.64), 3.376 (0.52), 3.384 (0.43), 3.397 (1.03), 3.426 (1.03), 3.466 (0.51), 3.495 (0.64), 3.509 (0.42), 3.532 (0.65), 3.606 (0.52), 3.708 (16.00), 3.720 (0.83), 3.751 (0.52), 3.827 (0.61), 3.853 (0.53), 4.043 (0.62), 4.059 (0.78), 4.078 (0.70), 4.096 (0.97), 4.104 (1.23), 4.122 (3.00), 4.897 (0.72), 4.915 (1.09), 4.934 (0.71), 6.444 (0.69), 6.460 (0.68), 6.487 (0.76), 6.503 (0.73), 6.554 (4.17), 6.686 (3.29), 6.699 (2.36), 7.736 (1.50), 7.740 (1.71), 7.744 (1.52), 8.309 (1.84), 8.314 (1.90), 8.318 (2.10), 8.323 (1.93).

### Example 329

### 2-[6-amino-5-(trifluoromethoxy)pyridin-3-yl]-N-[(1R)-2,3-dihydro-1H-inden-1-yl]-6,7-dihydrospiro[pyrazolo[5,1-c][1,4]oxazine-4,3'-pyrrolidine]-1'-carboxamide (mixture of stereoisomers)

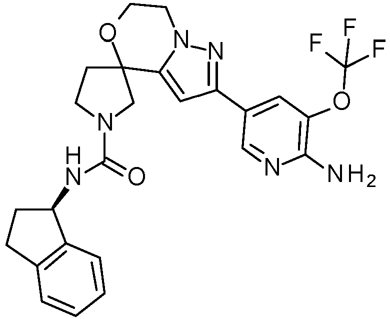

Example 329 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and (1R)-2,3-dihydro-1H-inden-1-amine (33.7 mg, 253 µmol).
LC-MS (method 1): Rt = 1.09 min; MS (ESlpos): m/z = 515 [M+H]⁺
¹H-NMR (400 MHz, DMSO-d6) δ [ppm]: 1.066 (1.06), 1.788 (0.40), 1.815 (1.31), 1.837 (1.62), 1.864 (1.41), 1.890 (0.50), 2.074 (0.47), 2.230 (1.29), 2.257 (1.62), 2.280 (0.82), 2.327 (2.89), 2.332 (2.23), 2.340 (2.52), 2.358 (2.50), 2.376 (2.32), 2.395 (1.29), 2.408 (0.72), 2.518 (4.51), 2.523 (3.05), 2.563 (1.42), 2.665 (0.94), 2.669 (1.30), 2.673 (0.89), 2.728 (2.24), 2.744 (1.27), 2.762 (1.77), 2.783 (2.17), 2.805 (1.15), 2.876 (1.69), 2.888 (3.39), 2.909 (1.07), 2.932 (0.93), 3.295 (0.53), 3.368 (1.07), 3.385 (1.32), 3.393 (1.31), 3.411 (2.22), 3.428 (1.34), 3.437 (1.20), 3.454 (0.63), 3.469 (2.47), 3.489 (2.55), 3.498 (2.90), 3.518 (2.76), 3.585 (0.78), 3.606 (1.58), 3.637 (1.57), 3.662 (0.65), 3.813 (1.57), 3.833 (1.84), 3.860 (1.39), 4.052 (1.13), 4.067 (2.03), 4.083 (1.97), 4.101 (2.64), 4.125 (6.55), 4.137 (7.99), 4.150 (2.46), 5.178 (1.06), 5.199 (3.08), 5.219 (3.08), 5.240 (1.10), 6.492 (2.53), 6.498 (2.56), 6.513 (2.52), 6.519 (2.47), 6.556 (12.53), 6.711 (16.00), 7.164 (2.67), 7.169 (3.70), 7.173 (4.76), 7.181 (6.46), 7.186 (5.40), 7.191 (5.99), 7.199 (2.13), 7.213 (4.86), 7.226 (5.85), 7.234 (4.14), 7.748 (6.40), 8.326 (7.87), 8.330 (7.79).

### Example 330

### 2'-[6-amino-5-(difluoromethoxy)pyridin-3-yl]-N-[1-(pyridin-2-yl)cyclobutyl]-5',6'-dihydrospiro[azetidine-3,4'-pyrrolo[1,2-b]pyrazole]-1-carboxamide

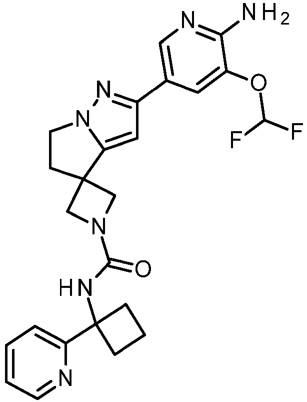

Example 330 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-(pyridin-2-yl)cyclobutan-1-amine (38.8 mg, 262 µmol).
LC-MS (method 1): Rt = 0.91 min; MS (ESlpos): m/z = 482 [M+H]⁺
¹H-NMR (400 MHz, DMSO-d6) δ [ppm]: 1.232 (1.03), 1.812 (0.51), 1.828 (1.17), 1.836 (1.49), 1.839 (1.41), 1.851 (2.96), 1.855 (2.62), 1.863 (2.72), 1.870 (2.46), 1.878 (4.21), 1.886 (2.41), 1.893 (2.82), 1.901 (2.53), 1.908 (1.44), 1.916 (1.38), 1.924 (0.74), 1.931 (0.64), 1.953 (0.80), 1.970 (1.88), 1.977 (2.07), 1.993 (4.14), 2.016 (3.63), 2.019 (3.71), 2.035 (1.95), 2.043 (1.76), 2.060 (0.87), 2.075 (0.48), 2.085 (0.59), 2.224 (3.73), 2.239 (4.14), 2.247 (6.00), 2.254 (6.16), 2.262 (5.04), 2.269 (6.83), 2.277 (4.56), 2.292 (3.41), 2.318 (0.85), 2.337 (1.61), 2.361 (1.94), 2.368 (1.99), 2.377 (1.63), 2.385 (2.22), 2.391 (1.60), 2.408 (1.21), 2.456 (0.53), 2.461 (0.96), 2.518 (9.66), 2.523 (6.86), 2.531 (5.81), 2.537 (8.03), 2.544 (5.94), 2.555 (6.15), 2.561 (7.96), 2.567 (5.03), 2.584 (5.41), 2.601 (1.82), 2.609 (1.94), 2.632 (0.94), 2.845 (2.71), 2.862 (4.39), 2.879 (2.87), 3.295 (0.43), 3.597 (0.59), 4.027 (1.65), 4.048 (11.31), 4.053 (11.15), 4.074 (1.61), 4.113 (3.09), 4.131 (4.69), 4.148 (2.86), 6.170 (7.92), 6.647 (16.00), 6.760 (11.52), 7.007 (3.19), 7.169 (8.98), 7.171 (13.21), 7.181 (7.17), 7.183 (8.15), 7.187 (8.52), 7.190 (13.26), 7.196 (3.48), 7.199 (8.00), 7.202 (7.54), 7.205 (3.06), 7.207 (2.67), 7.211 (2.46), 7.214 (2.40), 7.223 (2.32), 7.226 (2.40), 7.327 (9.18), 7.347 (10.09), 7.375 (6.64), 7.393 (2.65), 7.395 (4.42), 7.398 (2.51), 7.658 (10.31), 7.662 (7.29), 7.677 (8.54), 7.681 (8.58), 7.696 (5.37), 7.701 (5.35), 7.712 (2.29), 7.717 (2.40), 7.731 (2.90), 7.737 (2.82), 7.751 (1.87), 7.756 (1.79), 8.252 (7.42), 8.257 (7.79), 8.513 (7.34), 8.515 (8.71), 8.518 (9.00), 8.520 (7.56), 8.525 (7.52), 8.527 (9.03), 8.530 (8.23), 8.532 (7.15), 8.539 (2.69), 8.541 (2.96), 8.544 (2.82), 8.546 (2.63), 8.551 (2.56), 8.553 (2.87), 8.555 (2.52), 8.558 (2.33).

### Example 331

### 2'-[6-amino-5-(difluoromethoxy)pyridin-3-yl]-N-[1-(3-fluorophenyl)cyclobutyl]-5',6'-dihydrospiro[azetidine-3,4'-pyrrolo[1,2-b]pyrazole]-1-carboxamide

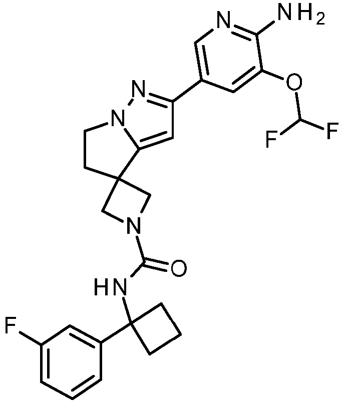

Example 331 was prepared in analogy to Example 31 using 3-(difluoromethoxy)-5-(5',6'-dihydrospiro[azetidine-3,4'-pyrrolo[1,2-b]pyrazol]-2'-yl)pyridin-2-amine-hydrochloride salt (75.0 mg, 218 µmol) and 1-(3-fluorophenyl)cyclobutan-1-amine-hydrochloride salt (52.8 mg, 262 µmol).
LC-MS (method 1): Rt = 1.09 min; MS (ESlpos): m/z = 499 [M+H]⁺
¹H-NMR (400 MHz, DMSO-d6) δ [ppm]: 1.232 (0.49), 1.760 (0.41), 1.765 (0.44), 1.781 (0.89), 1.787 (0.96), 1.798 (0.67), 1.803 (0.96), 1.809 (1.10), 1.819 (0.44), 1.825 (0.68), 1.977 (0.48), 1.983 (0.62), 1.999 (1.09), 2.005 (0.88), 2.016 (0.69), 2.022 (0.99), 2.026 (0.92), 2.038 (0.50), 2.043 (0.47), 2.049 (0.46), 2.318 (0.43), 2.361 (0.80), 2.390 (2.08), 2.407 (2.63), 2.412 (2.35), 2.430 (3.04), 2.447 (2.40), 2.453 (2.66), 2.470 (2.26), 2.518 (6.00), 2.523 (4.12), 2.660 (0.44), 2.819 (2.69), 2.836 (4.43), 2.853 (2.87), 3.979 (2.17), 4.000 (10.04), 4.007 (9.89), 4.028 (2.09), 4.097 (2.98), 4.115 (4.70), 4.131 (2.84), 6.164 (7.89), 6.608 (16.00), 6.992 (0.98), 6.994 (1.14), 6.999 (4.37), 7.014 (1.91), 7.021 (2.13), 7.034 (1.01), 7.036 (1.06), 7.041 (1.20), 7.043 (1.17), 7.129 (6.55), 7.167 (1.58), 7.171 (2.01), 7.173 (2.02), 7.177 (1.90), 7.183 (7.29), 7.194 (1.62), 7.198 (2.05), 7.204 (1.68), 7.253 (2.26), 7.256 (1.65), 7.273 (3.15), 7.336 (1.88), 7.352 (2.10), 7.356 (2.91), 7.367 (3.35), 7.372 (2.97), 7.376 (1.50), 7.392 (1.23), 7.640 (4.25), 7.642 (4.36), 7.644 (4.31), 8.234 (7.76), 8.240 (7.71).

### Example 332

### (rac)-2'-[6-amino-5-(trifluoromethoxy)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide

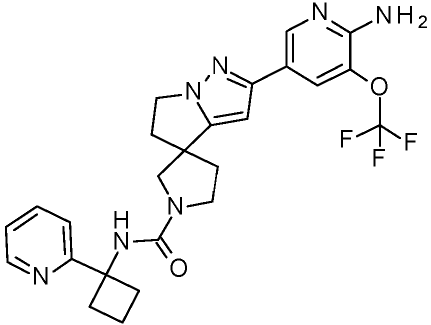

Example 332 was prepared in analogy to Example 31 using (*rac*)-5-[5',6'-dihydrospiro[pyrrolidine-3,4'-pyrrolo[1,2-b]pyrazol]-2'-yl]-3-(trifluoromethoxy)pyridin-2-amine-hydrochloride salt (75.0 mg, 200 µmol) and 1-(pyridin-2-yl)cyclobutan-1-amine (35.5 mg, 239 µmol).
LC-MS (method 1): Rt = 1.00 min; MS (ESlneg): m/z = 512 [M-H]⁻
¹H-NMR (400 MHz, DMSO-d6) δ [ppm]: 1.222 (0.66), 1.240 (3.09), 1.255 (2.87), 1.270 (1.78), 1.855 (0.46), 1.862 (0.60), 1.878 (1.14), 1.882 (1.17), 1.889 (1.00), 1.905 (1.62), 1.921 (0.93), 1.927 (0.84), 1.943 (0.58), 1.957 (0.74), 1.964 (0.84), 1.980 (1.58), 1.997 (1.08), 2.004 (1.35), 2.023 (0.79), 2.031 (0.76), 2.047 (0.81), 2.064 (1.60), 2.078 (2.98), 2.096 (3.38), 2.114 (1.38), 2.126 (0.72), 2.337 (0.66), 2.377 (2.31), 2.383 (2.17), 2.389 (2.14), 2.396 (2.37), 2.518 (6.16), 2.523 (4.43), 2.534 (3.71), 2.550 (6.19), 2.553 (6.37), 2.569 (5.52), 2.582 (2.56), 2.594 (2.29), 2.598 (2.16), 2.623 (0.65), 2.679 (0.58), 3.431 (0.94), 3.452 (2.01), 3.478 (8.50), 3.565 (0.89), 3.582 (1.58), 3.596 (1.37), 3.605 (1.26), 3.622 (0.76), 4.178 (4.03), 4.195 (7.04), 4.213 (3.89), 6.427 (16.00), 6.518 (8.39), 6.802 (5.57), 7.178 (1.67), 7.192 (2.11), 7.196 (2.16), 7.209 (1.91), 7.378 (2.60), 7.397 (2.93), 7.671 (1.30), 7.688 (2.19), 7.708 (1.08), 7.750 (4.44), 7.754 (6.14), 7.758 (4.17), 8.330 (10.12), 8.335 (9.89), 8.524 (3.07), 8.526 (3.59), 8.528 (3.68), 8.530 (3.27), 8.536 (3.17), 8.538 (3.68), 8.541 (3.41), 8.542 (2.97).

### Example 333

### 2-[6-amino-5-(difluoromethoxy)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide (mixture of stereoisomers)

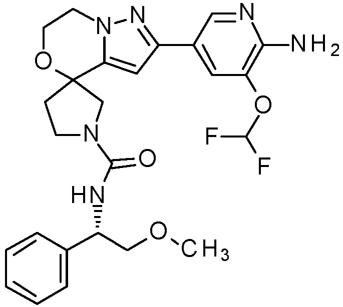

Example 333 was prepared in analogy to Example 31 using (rac)-3-(difluoromethoxy)-5-[6,7-dihydrospiro[pyrazolo[5,1-c][1,4]oxazine-4,3'-pyrrolidin]-2-yl]pyridin-2-amine-hydrochloride salt (75.0 mg, 201 µmol) and (1S)-2-methoxy-1-phenylethan-1-amine (36.4 mg, 241 µmol).
LC-MS (method 1): Rt = 0.98 min; MS (ESlpos): m/z = 515 [M+H]⁺
1H-NMR (400 MHz, DMSO-d6) δ [ppm]: 2.257 (0.41), 2.323 (0.97), 2.327 (1.39), 2.331 (1.10), 2.518 (7.21), 2.523 (5.04), 2.539 (2.31), 2.665 (0.72), 2.669 (1.00), 2.673 (0.72), 3.257 (16.00), 3.397 (0.59), 3.412 (0.41), 3.428 (0.51), 3.438 (0.62), 3.442 (0.61), 3.453 (1.08), 3.463 (1.15), 3.478 (0.67), 3.490 (0.87), 3.519 (0.56), 3.546 (0.98), 3.567 (1.31), 3.571 (1.02), 3.592 (1.00), 3.620 (0.44), 3.780 (0.51), 3.808 (0.87), 3.837 (0.43), 4.068 (0.57), 4.087 (0.67), 4.104 (1.05), 4.116 (1.52), 4.129 (2.24), 4.942 (0.61), 4.960 (0.62), 6.218 (3.10), 6.496 (0.93), 6.516 (0.88), 6.669 (3.67), 6.996 (0.88), 7.179 (1.74), 7.181 (1.79), 7.203 (0.44), 7.221 (1.24), 7.239 (0.97), 7.289 (1.03), 7.305 (2.21), 7.309 (2.03), 7.323 (1.31), 7.327 (1.36), 7.346 (2.95), 7.364 (2.01), 7.614 (1.83), 8.187 (1.51), 8.192 (2.67), 8.198 (1.67).

### Example 334

### 2'-[6-amino-5-(difluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide (mixture of stereoisomers)

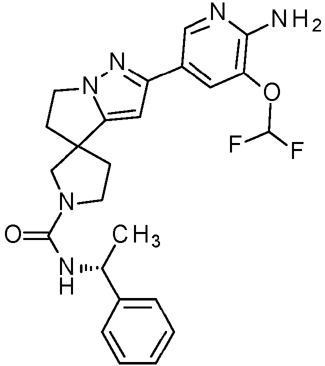

Example 334 was prepared in analogy to Example 31 using (rac)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and (1R)-1-phenylethan-1-amine (48.0 mg, 396 µmol).

1H-NMR (400 MHz, DMSO-d6) δ [ppm]: 1.354 (10.16), 1.361 (10.54), 1.372 (10.68), 1.379 (10.21), 2.056 (3.90), 2.066 (3.50), 2.073 (3.76), 2.323 (0.68), 2.327 (0.96), 2.331 (0.73), 2.523 (5.91), 2.529 (8.09), 2.546 (5.26), 2.665 (0.71), 2.669 (1.01), 2.673 (0.77), 3.411 (0.89), 3.428 (2.77), 3.454 (16.00), 3.485 (1.13), 3.514 (0.80), 3.532 (1.59), 3.555 (2.07), 3.570 (1.24), 4.158 (4.72), 4.175 (8.33), 4.193 (4.62), 4.824 (2.04), 4.842 (3.05), 4.861 (2.02), 5.759 (3.69), 6.167 (12.22), 6.346 (6.55), 6.361 (11.17), 6.459 (2.86), 6.464 (3.02), 6.480 (2.95), 6.485 (2.82), 6.999 (5.07), 7.157 (1.10), 7.175 (3.61), 7.183 (11.21), 7.190 (4.10), 7.205 (1.31), 7.208 (2.13), 7.256 (2.72), 7.275 (6.59), 7.294 (6.68), 7.298 (7.20), 7.317 (10.51), 7.339 (7.69), 7.360 (3.43), 7.367 (5.23), 7.617 (7.36), 8.188 (5.77), 8.193 (6.66), 8.195 (7.55), 8.200 (6.27).

### Example 335

### (rac)-2'-[6-amino-5-(difluoromethoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide

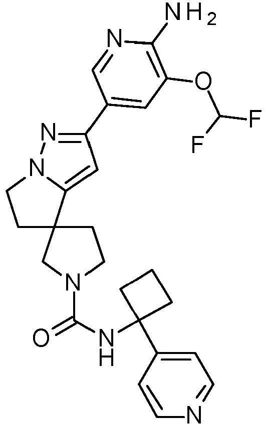

Example 335 was prepared in analogy to Example 31 using (*rac*)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and 1-(pyridin-4-yl)cyclobutan-1-amine (58.6 mg, 396 µmol).
LC-MS (method 1): Rt = 0.90 min; MS (ESlpos): m/z = 496 [M+H]⁺
1H-NMR (400 MHz, DMSO-d6) δ [ppm]: 1.803 (0.74), 1.820 (1.40), 1.826 (1.60), 1.843 (1.60), 1.848 (1.95), 1.866 (1.20), 1.888 (0.46), 1.978 (0.69), 1.985 (0.89), 1.991 (0.89), 2.000 (1.69), 2.008 (1.32), 2.014 (1.89), 2.028 (2.06), 2.043 (2.49), 2.060 (3.32), 2.078 (3.43), 2.084 (9.07), 2.096 (1.49), 2.109 (0.80), 2.261 (0.63), 2.278 (0.77), 2.283 (0.66), 2.300 (0.54), 2.309 (0.63), 2.322 (1.46), 2.326 (2.23), 2.332 (2.00), 2.336 (1.09), 2.347 (1.57), 2.362 (1.77), 2.375 (3.18), 2.391 (3.46), 2.397 (3.06), 2.413 (3.29), 2.442 (2.81), 2.463 (2.52), 2.518 (9.59), 2.523 (5.92), 2.530 (5.90), 2.534 (5.72), 2.550 (3.72), 2.660 (0.54), 2.664 (1.14), 2.669 (1.63), 2.673 (1.20), 2.678 (0.54), 3.415 (2.20), 3.441 (7.44), 3.473 (0.94), 3.525 (0.86), 3.542 (1.57), 4.163 (4.26), 4.180 (8.19), 4.198 (4.18), 5.759 (6.07), 6.174 (12.39), 6.360 (16.00), 6.755 (1.35), 6.871 (7.16), 7.000 (5.12), 7.184 (10.65), 7.304 (2.78), 7.309 (1.72), 7.316 (1.77), 7.319 (2.78), 7.368 (4.55), 7.390 (12.62), 7.394 (7.84), 7.402 (7.58), 7.405 (12.94), 7.618 (6.58), 7.620 (6.41), 8.197 (11.65), 8.202 (10.79), 8.439 (2.98), 8.444 (1.69), 8.451 (1.95), 8.454 (3.23), 8.461 (13.94), 8.464 (8.47), 8.471 (8.19), 8.476 (13.42).

### Example 336

### (rac)-2'-[6-amino-5-(difluoromethoxy)pyridin-3-yl]-N-(1-phenylcyclobutyl)-5',6'-dihydrospiro[pyrrolidine-3,4'-pyrrolo[1,2-b]pyrazole]-1-carboxamide

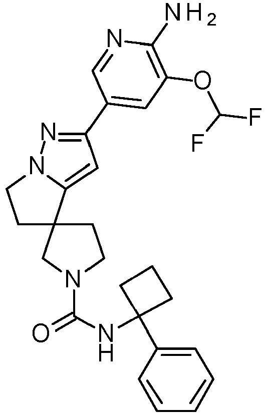

Example 336 was prepared in analogy to Example 31 using (*rac*)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and 1-phenylcyclobutan-1-amine-hydrochloride salt (72.7 mg, 396 µmol).
LC-MS (method 1): Rt = 1.09 min; MS (ESlpos): m/z = 495 [M+H]⁺
1H-NMR (400 MHz, DMSO-d6) δ [ppm]: 1.232 (0.47), 1.764 (1.24), 1.769 (1.32), 1.786 (1.42), 1.791 (1.57), 1.808 (0.99), 1.830 (0.40), 1.964 (0.84), 1.980 (1.54), 2.002 (1.57), 2.007 (1.61), 2.029 (2.24), 2.043 (3.73), 2.062 (3.83), 2.080 (1.57), 2.092 (0.92), 2.356 (1.22), 2.385 (2.73), 2.402 (3.18), 2.423 (2.11), 2.449 (1.94), 2.470 (3.40), 2.518 (16.00), 2.522 (12.72), 2.537 (5.66), 2.888 (0.42), 3.376 (1.04), 3.396 (2.71), 3.422 (6.96), 3.433 (5.39), 3.459 (1.42), 3.502 (0.92), 3.520 (1.69), 3.535 (1.34), 3.561 (0.70), 4.156 (3.88), 4.174 (7.40), 4.191 (3.88), 5.759 (3.70), 6.174 (11.06), 6.329 (14.19), 6.697 (6.61), 6.997 (4.20), 7.134 (1.99), 7.136 (1.22), 7.152 (4.92), 7.170 (3.50), 7.173 (2.24), 7.182 (8.97), 7.261 (5.64), 7.280 (9.47), 7.299 (5.39), 7.366 (3.88), 7.417 (7.85), 7.419 (8.89), 7.438 (7.35), 7.441 (5.89), 7.610 (6.11), 7.612 (5.99), 8.184 (9.49), 8.189 (9.96).

### Example 337

### (rac)-2'-[6-amino-5-(difluoromethoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide

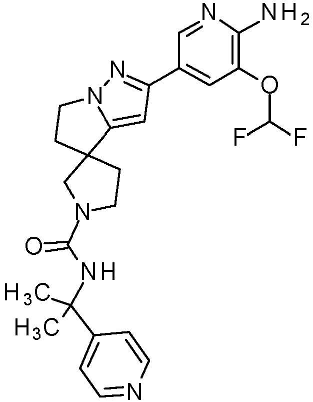

Example 337 was prepared in analogy to Example 31 using (*rac*)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and 2-(pyridin-4-yl)propan-2-amine (53.9 mg, 396 µmol).
LC-MS (method 1): Rt = 0.88 min; MS (ESlpos): m/z = 484 [M+H]⁺
¹H-NMR (400MHz, DMSO-d₆): δ [ppm]= 1.54 (d, 6H), 2.00 - 2.14 (m, 2H), 2.53 - 2.58 (m, 3H), 3.39 - 3.50 (m, 3H), 3.52 - 3.61 (m, 1H), 4.19 (t, 2H), 6.20 (d, 3H), 6.38 (s, 1H), 6.98 - 7.39 (m, 1H), 7.29 - 7.34 (m, 1H), 7.60 - 7.65 (m, 1H), 8.21 (d, 1H), 8.40 - 8.45 (m, 2H).

### Example 338

### 2'-[6-amino-5-(difluoromethoxy)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide (mixture of stereoisomers)

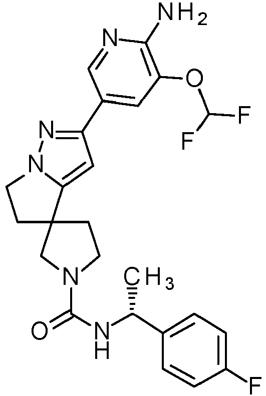

Example 338 was prepared in analogy to Example 31 using (*rac*)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and (1R)-1-(4-fluorophenyl)ethan-1-amine (55.1 mg, 396 µmol).
LC-MS (method 1): Rt = 1.03 min; MS (ESlpos): m/z = 487 [M+H]⁺
1H-NMR (400 MHz, DMSO-d6) δ [ppm]: 1.233 (1.08), 1.344 (11.91), 1.351 (12.63), 1.361 (12.63), 1.369 (12.09), 2.038 (2.65), 2.053 (4.81), 2.069 (5.01), 2.083 (2.17), 2.518 (6.64), 2.524 (9.48), 2.527 (9.04), 2.544 (5.87), 3.399 (0.80), 3.418 (2.85), 3.426 (2.73), 3.449 (14.96), 3.475 (1.94), 3.506 (0.94), 3.524 (1.78), 3.532 (1.80), 3.538 (1.58), 3.551 (2.47), 3.558 (1.30), 3.565 (1.66), 3.577 (1.08), 4.157 (5.49), 4.175 (10.29), 4.192 (5.33), 4.820 (2.49), 4.839 (3.71), 4.857 (2.47), 6.166 (15.92), 6.360 (10.33), 6.365 (16.00), 6.470 (3.29), 6.479 (3.41), 6.490 (3.33), 6.499 (3.11), 6.997 (5.97), 7.071 (4.15), 7.076 (1.56), 7.093 (10.11), 7.114 (10.69), 7.130 (1.60), 7.136 (5.23), 7.182 (12.35), 7.338 (4.37), 7.343 (1.98), 7.352 (5.27), 7.361 (8.30), 7.366 (8.42), 7.374 (7.98), 7.383 (4.71), 7.392 (1.64), 7.397 (3.69), 7.620 (9.40), 8.195 (8.32), 8.198 (11.01), 8.202 (8.86).

### Example 339

### (rac)-2'-[6-amino-5-(difluoromethoxy)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide

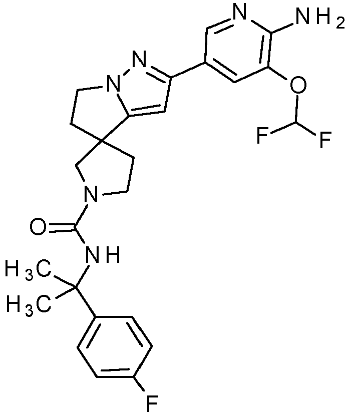

Example 339 was prepared in analogy to Example 31 using (*rac*)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and 2-(4-fluorophenyl)propan-2-amine (60.6 mg, 396 µmol).
LC-MS (method 1): Rt = 1.09 min; MS (ESlpos): m/z = 501 [M+H]⁺
1H-NMR (400 MHz, DMSO-d6) δ [ppm]: 1.555 (16.00), 2.033 (0.88), 2.050 (1.54), 2.070 (1.66), 2.084 (3.71), 2.523 (3.12), 2.526 (3.43), 2.546 (1.99), 3.410 (0.86), 3.417 (1.04), 3.445 (4.48), 3.543 (0.87), 3.557 (0.70), 3.565 (0.64), 4.164 (2.09), 4.182 (3.83), 4.199 (2.07), 6.069 (4.13), 6.175 (6.04), 6.364 (8.90), 7.000 (2.11), 7.027 (2.66), 7.032 (1.04), 7.050 (5.65), 7.067 (1.01), 7.072 (3.11), 7.184 (4.31), 7.353 (2.84), 7.358 (1.44), 7.367 (4.54), 7.375 (3.15), 7.384 (1.19), 7.389 (2.60), 7.620 (3.25), 8.202 (4.68), 8.207 (4.76).

### Example 340

### 2'-[6-amino-5-(difluoromethoxy)pyridin-3-yl]-N-[(1R)-1-(5-fluoropyridin-3-yl)ethyl]-5',6'-dihydrospiro[pyrrolidine-3,4'-pyrrolo[1,2-b]pyrazole]-1-carboxamide (mixture of stereoisomers)

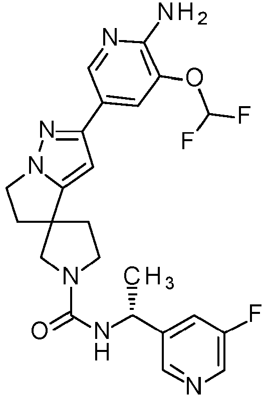

Example 340 was prepared in analogy to Example 31 using (*rac*)-3-(difluoromethoxy)-5-[5',6'-dihydrospiro[pyrrolidine-3,4'-pyrrolo[1,2-b]pyrazol]-2'-yl]pyridin-2-amine-hydrochloride salt (130 mg, 330 µmol) and (1R)-1-(5-fluoropyridin-3-yl)ethan-1-amine (55.5 mg, 396 µmol).
LC-MS (method 1): Rt = 0.89 min; MS (ESlpos): m/z = 488 [M+H]⁺
¹H-NMR (400MHz, DMSO-d₆): δ [ppm]= 1.41 (dd, 3H), 2.00 - 2.14 (m, 2H), 2.52 - 2.58 (m, 2H), 3.39 - 3.64 (m, 4H), 4.18 (t, 2H), 4.87 - 4.97 (m, 1H), 6.16 (s, 2H), 6.33 - 6.39 (m, 1H), 6.60 (dd, 1H), 6.98 - 7.39 (m, 1H), 7.62 (s, 1H), 7.68 (ddt, 1H), 8.16 - 8.22 (m, 1H), 8.38 - 8.48 (m, 2H).

### Example 341

### 2'-{6-amino-5-[(1R)-1-(pyridin-2-yl)ethoxy]pyridin-3-yl}-N-[(1R)-1-phenylethyl]-5',6'-dihydrospiro[pyrrolidine-3,4'-pyrrolo[1,2-b]pyrazole]-1-carboxamide (mixture of stereoisomers)

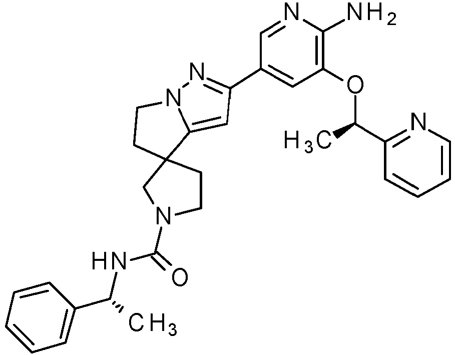

Example 341 was prepared in analogy to Example 31 using 5-[5',6'-dihydrospiro[pyrrolidine-3,4'-pyrrolo[1,2-b]pyrazol]-2'-yl]-3-[(1R)-1-(pyridin-2-yl)ethoxy]pyridin-2-amine-hydrochloride salt (50.0 mg, 121 µmol) and (1R)-1-phenylethan-1-amine (17.6 mg, 145 µmol).
LC-MS (method 1): Rt = 0.99 min; MS (ESlpos): m/z = 524 [M+H]⁺
¹H-NMR (400 MHz, DMSO-d6) δ [ppm]: 0.851 (0.63), 1.233 (2.25), 1.284 (0.95), 1.301 (0.85), 1.351 (8.50), 1.356 (8.28), 1.369 (8.85), 1.374 (8.05), 1.501 (0.57), 1.519 (0.64), 1.610 (15.95), 1.626 (16.00), 2.022 (2.91), 2.037 (3.75), 2.055 (1.62), 2.067 (0.81), 2.083 (0.43), 2.472 (3.83), 2.522 (3.96), 3.383 (0.95), 3.393 (1.36), 3.401 (1.68), 3.419 (6.31), 3.426 (12.08), 3.452 (1.43), 3.490 (0.62), 3.509 (1.30), 3.532 (1.58), 3.546 (0.98), 3.571 (0.43), 4.109 (3.48), 4.125 (6.34), 4.143 (3.45), 4.818 (1.10), 4.824 (1.27), 4.837 (1.63), 4.843 (1.75), 4.855 (1.23), 4.862 (1.16), 5.487 (1.03), 5.503 (3.73), 5.520 (3.74), 5.536 (1.05), 5.901 (8.53), 6.200 (5.92), 6.210 (9.28), 6.446 (2.23), 6.452 (2.20), 6.466 (2.23), 6.473 (2.02), 7.151 (0.81), 7.170 (2.86), 7.188 (3.86), 7.202 (4.87), 7.206 (7.79), 7.248 (2.18), 7.268 (5.80), 7.275 (4.31), 7.279 (3.86), 7.281 (4.20), 7.286 (6.34), 7.291 (4.53), 7.296 (6.63), 7.303 (3.40), 7.310 (6.17), 7.314 (5.96), 7.327 (3.02), 7.338 (5.34), 7.356 (2.63), 7.384 (0.45), 7.490 (4.17), 7.510 (4.72), 7.762 (1.58), 7.766 (2.26), 7.769 (1.58), 7.781 (2.65), 7.785 (3.79), 7.788 (2.52), 7.800 (1.36), 7.804 (1.81), 7.808 (1.24), 7.850 (4.47), 7.854 (4.74), 7.859 (5.04), 7.864 (4.65), 8.539 (3.39), 8.544 (2.27), 8.546 (2.35), 8.549 (3.36), 8.552 (3.36).

### Example 342

### 2'-{6-amino-5-[(1R)-1-(pyridin-2-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide (mixture of stereoisomers)

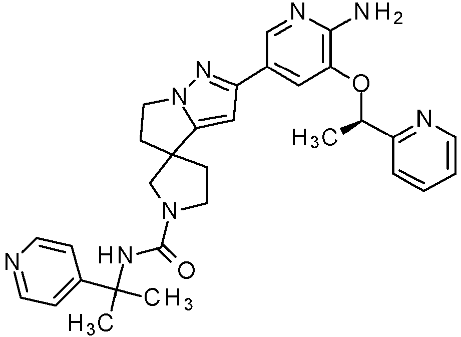

Example 342 was prepared in analogy to Example 31 using 5-[5',6'-dihydrospiro[pyrrolidine-3,4'-pyrrolo[1,2-b]pyrazol]-2'-yl]-3-[(1R)-1-(pyridin-2-yl)ethoxy]pyridin-2-amine-hydrochloride salt (50.0 mg, 121 µmol) and 2-(pyridin-4-yl)propan-2-amine (19.8 mg, 145 µmol).
LC-MS (method 1): Rt = 0.87 min; MS (ESlpos): m/z = 539 [M+H]⁺
¹H-NMR (400 MHz, DMSO-d6) δ [ppm]: 1.020 (0.68), 1.036 (0.73), 1.448 (0.44), 1.516 (1.99), 1.527 (7.04), 1.534 (16.00), 1.616 (6.47), 1.632 (6.52), 2.005 (0.49), 2.029 (0.92), 2.045 (1.00), 2.053 (0.84), 2.074 (0.55), 2.518 (3.37), 2.522 (1.72), 3.399 (0.91), 3.424 (2.69), 3.529 (0.63), 4.119 (1.80), 4.137 (3.26), 4.154 (1.76), 5.491 (0.47), 5.507 (1.73), 5.523 (1.74), 5.539 (0.47), 5.914 (4.77), 6.207 (3.24), 6.231 (6.45), 7.211 (1.95), 7.214 (3.34), 7.219 (1.89), 7.271 (0.78), 7.274 (1.41), 7.276 (0.89), 7.283 (0.84), 7.286 (1.52), 7.289 (1.47), 7.292 (1.57), 7.295 (1.00), 7.302 (3.70), 7.307 (5.03), 7.310 (2.34), 7.314 (2.17), 7.317 (4.40), 7.322 (3.17), 7.495 (1.92), 7.497 (1.92), 7.514 (2.18), 7.517 (2.14), 7.766 (0.96), 7.770 (1.03), 7.785 (1.68), 7.789 (1.71), 7.805 (0.78), 7.808 (0.80), 7.872 (2.71), 7.875 (3.57), 7.879 (2.70), 8.412 (3.17), 8.416 (2.08), 8.420 (3.56), 8.423 (3.43), 8.427 (3.41), 8.430 (1.98), 8.435 (3.09), 8.541 (1.51), 8.543 (1.74), 8.545 (1.76), 8.548 (1.58), 8.553 (1.55), 8.555 (1.75), 8.558 (1.65), 8.560 (1.44).

### Example 343

### 2'-{6-amino-5-[(1S)-1-(pyridin-2-yl)ethoxy]pyridin-3-yl}-N-[(1R)-1-phenylethyl]-5',6'-dihydrospiro[pyrrolidine-3,4'-pyrrolo[1,2-b]pyrazole]-1-carboxamide (mixture of stereoisomers)

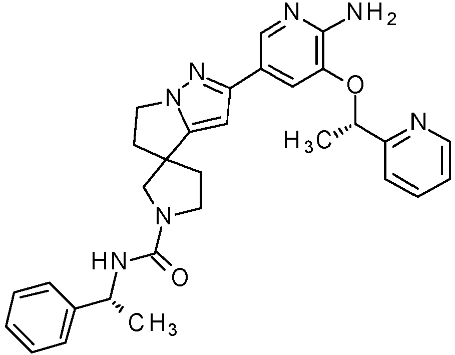

Example 343 was prepared in analogy to Example 31 using 5-[5',6'-dihydrospiro[pyrrolidine-3,4'-pyrrolo[1,2-b]pyrazol]-2'-yl]-3-[(1S)-1-(pyridin-2-yl)ethoxy]pyridin-2-amine-hydrochloride salt (65.0 mg, 157 µmol) and (1R)-1-phenylethan-1-amine (22.9 mg, 189 µmol).
LC-MS (method 1): Rt = 0.99 min; MS (ESlpos): m/z = 524 [M+H]⁺
¹H-NMR (400 MHz, DMSO-d6) δ [ppm]: 0.852 (0.57), 0.943 (0.42), 1.232 (2.20), 1.256 (0.57), 1.284 (1.07), 1.301 (1.05), 1.350 (8.42), 1.361 (9.11), 1.367 (9.37), 1.379 (8.14), 1.611 (15.80), 1.627 (16.00), 1.754 (0.76), 1.988 (0.52), 2.002 (1.29), 2.014 (2.48), 2.030 (3.55), 2.045 (2.84), 2.062 (1.24), 2.076 (0.61), 2.473 (3.58), 2.518 (2.11), 2.523 (1.43), 3.167 (1.88), 3.390 (1.50), 3.400 (1.63), 3.409 (2.64), 3.422 (10.44), 3.433 (7.01), 3.452 (1.39), 3.457 (1.17), 3.490 (0.72), 3.507 (1.35), 3.516 (1.28), 3.521 (1.14), 3.534 (1.69), 3.542 (0.93), 3.549 (1.19), 3.556 (0.77), 3.575 (0.47), 4.109 (3.93), 4.126 (7.26), 4.143 (3.81), 4.655 (0.92), 4.805 (0.49), 4.823 (1.99), 4.841 (2.91), 4.860 (1.97), 4.877 (0.48), 5.491 (1.02), 5.506 (3.63), 5.523 (3.60), 5.539 (1.03), 5.902 (10.00), 6.202 (6.84), 6.214 (10.81), 6.446 (2.35), 6.455 (2.35), 6.466 (2.31), 6.476 (2.11), 7.156 (0.50), 7.160 (0.96), 7.164 (1.00), 7.167 (1.12), 7.171 (1.14), 7.178 (2.54), 7.185 (2.79), 7.191 (1.76), 7.195 (2.13), 7.199 (2.36), 7.207 (8.57), 7.212 (7.93), 7.261 (2.95), 7.263 (2.49), 7.275 (7.06), 7.278 (7.62), 7.283 (5.01), 7.286 (2.93), 7.295 (9.05), 7.302 (2.42), 7.305 (2.39), 7.315 (6.12), 7.318 (5.75), 7.336 (7.98), 7.354 (2.85), 7.358 (2.00), 7.492 (3.60), 7.495 (3.63), 7.512 (4.05), 7.514 (4.09), 7.758 (1.43), 7.763 (2.47), 7.768 (1.63), 7.777 (2.41), 7.782 (4.18), 7.787 (2.59), 7.796 (1.24), 7.801 (1.97), 7.806 (1.26), 7.856 (5.92), 7.860 (7.99), 7.863 (6.19), 8.067 (2.23), 8.532 (1.72), 8.534 (2.08), 8.538 (3.44), 8.544 (3.49), 8.550 (3.37), 8.554 (2.12), 8.556 (1.81).

### Example 344

### 2'-{6-amino-5-[(1S)-1-(pyridin-2-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide (mixture of stereoisomers)

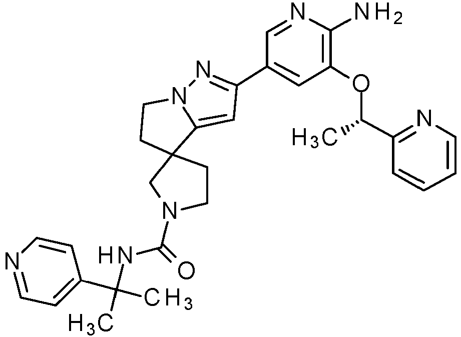

Example 344 was prepared in analogy to Example 31 using 5-[5',6'-dihydrospiro[pyrrolidine-3,4'-pyrrolo[1,2-b]pyrazol]-2'-yl]-3-[(1S)-1-(pyridin-2-yl)ethoxy]pyridin-2-amine-hydrochloride salt (65.0 mg, 157 µmol) and 2-(pyridin-4-yl)propan-2-amine (25.7 mg, 189 µmol).
LC-MS (method 1): Rt = 0.87 min; MS (ESlpos): m/z = 539 [M+H]⁺
¹H-NMR (400 MHz, DMSO-d6) δ [ppm]: 1.233 (0.64), 1.527 (7.26), 1.534 (16.00), 1.617 (6.59), 1.632 (6.59), 2.006 (0.55), 2.028 (0.97), 2.045 (1.02), 2.054 (0.83), 2.062 (0.53), 2.072 (0.46), 2.482 (1.79), 2.518 (2.14), 3.162 (11.10), 3.171 (11.24), 3.399 (0.94), 3.425 (2.72), 3.530 (0.64), 4.104 (1.82), 4.119 (3.23), 4.137 (3.63), 4.155 (1.84), 5.492 (0.48), 5.508 (1.76), 5.525 (1.76), 5.541 (0.48), 5.915 (4.86), 6.209 (3.28), 6.232 (6.55), 7.212 (1.99), 7.216 (3.35), 7.221 (1.95), 7.270 (0.74), 7.273 (1.30), 7.276 (0.81), 7.282 (0.81), 7.285 (1.43), 7.289 (1.45), 7.291 (1.54), 7.295 (0.92), 7.303 (4.17), 7.307 (5.04), 7.311 (2.24), 7.314 (2.18), 7.319 (4.34), 7.322 (3.11), 7.495 (1.95), 7.497 (1.92), 7.515 (2.23), 7.518 (2.15), 7.766 (0.98), 7.769 (1.00), 7.785 (1.70), 7.789 (1.71), 7.804 (0.79), 7.808 (0.78), 7.874 (2.78), 7.877 (3.70), 7.882 (2.66), 8.413 (3.06), 8.416 (1.99), 8.420 (3.46), 8.424 (3.30), 8.428 (3.26), 8.432 (1.90), 8.435 (2.92), 8.541 (1.53), 8.544 (1.76), 8.546 (1.83), 8.548 (1.56), 8.553 (1.55), 8.556 (1.78), 8.558 (1.69), 8.560 (1.41).

### Example 345

### 2'-{6-amino-5-[(trifluoromethyl)sulfanyl]pyridin-3-yl}-N-[(1R)-1-(3-fluorophenyl)ethyl]-5',6'-dihydrospiro[pyrrolidine-3,4'-pyrrolo[1,2-b]pyrazole]-1-carboxamide (mixture of stereoisomers)

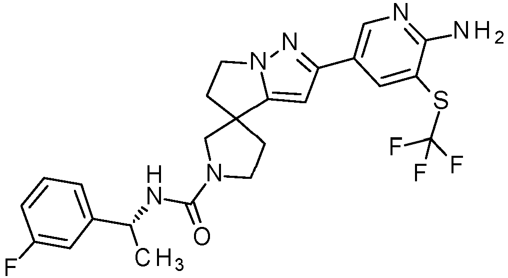

Example 345 was prepared in analogy to Example 31 using (*rac*)-5-[5',6'-dihydrospiro[pyrrolidine-3,4'-pyrrolo[1,2-b]pyrazol]-2'-yl]-3-[(trifluoromethyl)sulfanyl]pyridin-2-amine-hydrochloride salt (20.0 mg, 51.0 µmol) and (1R)-1-(3-fluorophenyl)ethan-1-amine (8.52 mg, 61.2 µmol).
LC-MS (method 1): Rt = 1.15 min; MS (ESlpos): m/z = 521 [M+H]⁺
¹H-NMR (400 MHz, DMSO-d6) δ [ppm]: 1.021 (5.37), 1.026 (4.82), 1.039 (6.90), 1.057 (1.83), 1.231 (0.72), 1.282 (1.39), 1.299 (1.55), 1.344 (5.57), 1.351 (14.20), 1.357 (15.16), 1.369 (14.52), 1.375 (14.05), 1.496 (0.76), 1.513 (0.76), 2.050 (3.51), 2.068 (6.04), 2.074 (6.57), 2.084 (7.45), 2.518 (14.51), 2.536 (11.93), 2.554 (7.38), 3.113 (0.43), 3.131 (1.28), 3.148 (1.22), 3.283 (0.44), 3.353 (3.16), 3.426 (3.97), 3.452 (10.23), 3.458 (14.36), 3.471 (6.99), 3.497 (1.97), 3.513 (1.17), 3.530 (2.10), 3.554 (2.01), 3.571 (2.31), 3.585 (1.35), 3.596 (1.29), 3.612 (0.68), 4.167 (6.50), 4.184 (11.23), 4.202 (6.32), 4.263 (0.50), 4.280 (0.69), 4.297 (0.53), 4.833 (2.41), 4.850 (3.64), 4.867 (2.46), 5.454 (0.44), 5.768 (0.47), 6.396 (7.95), 6.414 (15.55), 6.506 (3.49), 6.517 (3.79), 6.526 (3.70), 6.537 (3.41), 6.743 (16.00), 6.972 (1.40), 6.978 (1.62), 6.992 (4.01), 7.014 (4.01), 7.028 (1.57), 7.033 (1.94), 7.037 (1.63), 7.102 (0.84), 7.134 (2.87), 7.140 (3.16), 7.148 (3.53), 7.168 (8.65), 7.190 (6.29), 7.289 (2.00), 7.303 (2.37), 7.309 (3.51), 7.318 (1.64), 7.324 (3.80), 7.331 (3.89), 7.347 (3.10), 7.366 (1.17), 7.529 (0.42), 8.036 (5.69), 8.041 (10.57), 8.047 (6.25), 8.512 (8.02), 8.517 (13.46), 8.522 (8.70).

### Example 346

### 2'-[6-amino-5-(trifluoromethyl)pyridin-3-yl]-N-[(3R)-2,3-dihydro-1-benzofuran-3-yl]-5',6'-dihydrospiro[pyrrolidine-3,4'-pyrrolo[1,2-b]pyrazole]-1-carboxamide (mixture of stereoisomers)

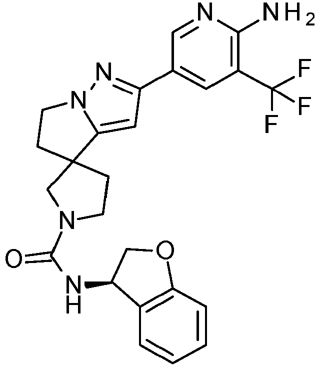

Example 346 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride salt (75.0 mg, 208 µmol) and 1): (3R)-2,3-dihydro-1-benzofuran-3-amine-hydrochloride salt (42.9 mg, 250 µmol).
LC-MS (method 1): Rt = 1.05 min; MS (ESlpos): m/z = 485 [M+H]⁺
1H-NMR (400MHz, DMSO-d6): δ [ppm]= 2.00-2.11 (m, 2H), 2.52-2.56 (m, 2H), 3.39-3.61 (m, 4H), 4.17 (t, 2H), 4.24 (dt, 1H), 4.66 (td, 1H), 5.43-5.50 (m, 1H), 6.46 and 6.48 (2s, 1H), 6.54 (s, 2H), 6.76 (dd, 1H), 6.80 (d, 1H), 6.83-6.90 (m, 1H), 7.18 (tdd, 1H), 7.30-7.35 (m, 1H), 8.01 (s, 1H), 8.59 (d, 1H).

### Example 347

### 2'-{6-amino-5-[(1R)-1-(pyridin-3-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide (mixture of stereoisomers)

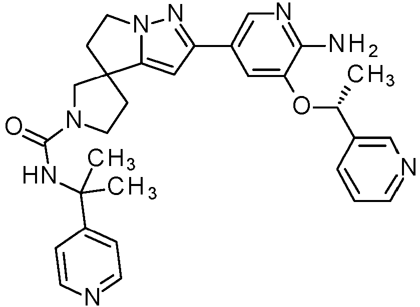

Example 347 was prepared in analogy to Example 31 using 5-[5',6'-dihydrospiro[pyrrolidine-3,4'-pyrrolo[1,2-b]pyrazol]-2'-yl]-3-[(1R)-1-(pyridin-3-yl)ethoxy]pyridin-2-amine-hydrochloride salt (82.7 mg, 79 % purity, 157 µmol) and 2-(pyridin-4-yl)propan-2-amine (25.7 mg, 189 µmol).
LC-MS (method 1): Rt = 0.87 min; MS (ESlpos): m/z = 539 [M+H]⁺
1H-NMR (400MHz, DMSO-d6): δ [ppm]= 1.52 and 1.54 (2s, 6H), 1.60 (d, 3H), 1.98 - 2.12 (m, 2H), 3.38 - 3.48 (m, 3H), 3.51 - 3.59 (m, 1H), 4.15 (t, 2H), 5.67 - 5.74 (m, 1H), 5.91 (s, 2H), 6.21 and 6.22 (2s, 1H), 6.28 (s, 1H), 7.29 - 7.34 (m, 3H), 7.38 (dd, 1H), 7.88 (d, 1H), 7.92 (dt, 1H), 8.40 - 8.44 (m, 2H), 8.47 (dd, 1H), 8.71 (d, 1H).

### Example 348

### 2'-[6-amino-5-(trifluoromethyl)pyridin-3-yl]-N-[1-(1-methyl-1H-imidazol-5-yl)ethyl]-5',6'-dihydrospiro[pyrrolidine-3,4'-pyrrolo[1,2-b]pyrazole]-1-carboxamide (diastereomeric mixture)

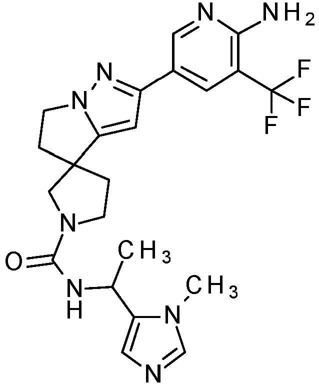

Example 348 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride salt (70.0 mg, 195 µmol) and 1-(1-methyl-1H-imidazol-5-yl)ethan-1-amine-hydrochloride salt (46.3 mg, 233 µmol).
LC-MS (method 1): Rt = 0.84 min; MS (ESlpos): m/z = 475 [M+H]⁺
1H-NMR (400MHz, DMSO-d6): δ [ppm]= 1.41 and 1.43 (2d, 3H), 1.99-2.12 (m, 2H), 3.39-3.61 (m, 7H), 4.17 (t, 2H), 4.93 and 4.96 (2q, 1H), 6.34 an 6.36 (2br d, 1H), 6.44 and 6.45 (2s, 1H), 6.54 (s, 2H), 6.79 and 6.80 (2s, 1H), 7.49 and 7.50 (2br s, 1H), 8.01 (d, 1H), 8.58 (d, 1H).

### Example 349

### 2'-{6-amino-5-[(1R)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide (mixture of stereoisomers)

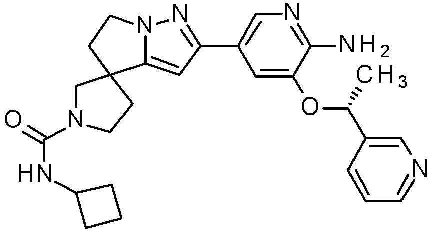

Example 349 was prepared in analogy to Example 31 using 5-[5',6'-dihydrospiro[pyrrolidine-3,4'-pyrrolo[1,2-b]pyrazol]-2'-yl]-3-[(1R)-1-(pyridin-3-yl)ethoxy]pyridin-2-amine-hydrochloride salt (82.7 mg, 79 % purity, 157 µmol) and cyclobutanamine (13.4 mg, 189 µmol).
LC-MS (method 1): Rt = 0.87 min; MS (ESlpos): m/z = 474 [M+H]⁺
1H-NMR (400MHz, DMSO-d6): δ [ppm]= 1.46 - 1.62 (m, 5H), 1.86 - 2.16 (m, 6H), 3.36 - 3.43 (m, 3H), 3.44 - 3.53 (m, 1H), 4.06 - 4.19 (m, 3H), 5.71 (q, 1H), 5.89 (s, 2H), 6.27 (2s, 1H), 6.31 (d, 1H), 7.32 (s, 1H), 7.37 (dd, 1H), 7.87 (t, 1H), 7.88 - 7.94 (m, 1H), 8.47 (dd, 1H), 8.70 (2s, 1H).

### Example 350

### 2'-{6-amino-5-[(1R)-1-(pyridin-3-yl)ethoxy]pyridin-3-yl}-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide (mixture of stereoisomers)

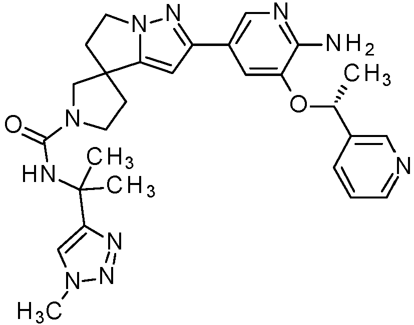

Example 350 was prepared in analogy to Example 31 using 5-[5',6'-dihydrospiro[pyrrolidine-3,4'-pyrrolo[1,2-b]pyrazol]-2'-yl]-3-[(1R)-1-(pyridin-3-yl)ethoxy]pyridin-2-amine-hydrochloride salt (82.7 mg, 79 % purity, 157 µmol) and 2-(1-methyl-1H-1,2,3-triazol-4-yl)propan-2-amine-hydrochloride salt (40.2 mg, 189 µmol).
LC-MS (method 1): Rt = 0.81 min; MS (ESlpos): m/z = 543 [M+H]⁺
¹H-NMR (400MHz, DMSO-d₆): δ [ppm]= 1.56-1.66 (m, 9H), 1.96-2.10 (m, 2H), 3.37 - 3.40 (m, 3H), 3.46-3.57 (m, 1H), 3.97 (2s, 3H), 4.14 (br t, 2H), 5.67 - 5.74 (m, 1H), 5.84-5.94 (m, 3H), 6.28 (s, 1H), 7.32 (s, 1H), 7.37 (dd, 1H), 7.79 (d, 1H), 7.84-7.94 (m, 2H), 8.47 (dd, 1H), 8.71 (s, 1H).

### Example 351

### 2'-[6-amino-5-(trifluoromethyl)pyridin-3-yl]-N-[cyclopropyl(pyridin-2-yl)methyl]-5',6'-dihydrospiro[pyrrolidine-3,4'-pyrrolo[1,2-b]pyrazole]-1-carboxamide (diastereomeric mixture)

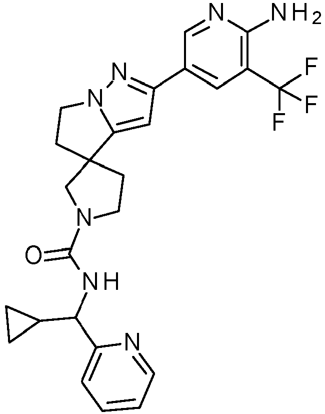

Example 351 was prepared in analogy to Example 31 using 5-[5',6'-dihydrospiro[pyrrolidine-3,4'-pyrrolo[1,2-b]pyrazol]-2'-yl]-3-(trifluoromethyl)pyridin-2-amine-hydrochloride salt (75.0 mg, 208 µmol) and (RS)-1-cyclopropyl-1-(pyridin-2-yl)methanamine (37.1 mg, 250 µmol).
LC-MS (method 1): Rt = 1.00 min; MS (ESlpos): m/z = 498 [M+H]⁺
1H-NMR (400MHz, DMSO-d6): δ [ppm]= 0.32-0.51 (m, 4H), 1.16-1.26 (m, 1H), 2.01-2.14 (m, 2H), 2.52-2.58 (m, 2H), 3.42-3.51 (m, 3H), 3.53-3.62 (m, 1H), 4.15-4.23 (m, 3H), 6.43-6.47 (m, 1H), 6.51-6.58 (m, 3H), 7.20-7.27 (m, 1H), 7.44 (dd, 1H), 7.73 (dtd, 1H), 8.00 (s, 1H), 8.46-8.52 (m, 1H), 8.58 (s, 1H)

### Example 352

### 2'-{6-amino-5-[(1S)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide (mixture of stereoisomers)

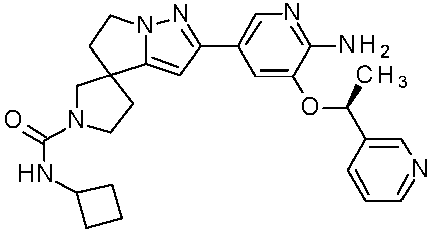

Example 352 was prepared in analogy to Example 31 using 5-[5',6'-dihydrospiro[pyrrolidine-3,4'-pyrrolo[1,2-b]pyrazol]-2'-yl]-3-[(1S)-1-(pyridin-3-yl)ethoxy]pyridin-2-amine-hydrochloride salt (75.0 mg, 182 µmol) and cyclobutanamine (15.5 mg, 218 µmol).
LC-MS (method 1): Rt = 0.88 min; MS (ESlpos): m/z = 474 [M+H]⁺
1H-NMR (400MHz, DMSO-d6): δ [ppm]= 1.47 - 1.62 (m, 5H), 1.85 - 2.15 (m, 6H), 3.36 - 3.43 (m, 3H), 3.44 - 3.52 (m, 1H), 4.05 - 4.21 (m, 3H), 5.71 (q, 1H), 5.89 (s, 2H), 6.27 (2s, 1H), 6.31 (d, 1H), 7.32 (s, 1H), 7.37 (dd, 1H), 7.87 (t, 1H), 7.91 (dt, 1H), 8.47 (dd, 1H), 8.69 - 8.72 (m, 1H).

### Example 353

### 2'-{6-amino-5-[(1S)-1-(pyridin-3-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide (mixture of stereoisomers)

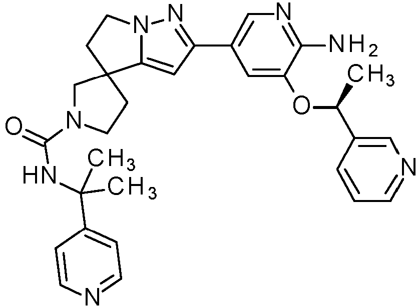

Example 353 was prepared in analogy to Example 31 using 5-[5',6'-dihydrospiro[pyrrolidine-3,4'-pyrrolo[1,2-b]pyrazol]-2'-yl]-3-[(1S)-1-(pyridin-3-yl)ethoxy]pyridin-2-amine-hydrochloride salt (75.0 mg, 182 µmol) and 2-(pyridin-4-yl)propan-2-amine (29.7 mg, 218 µmol).
LC-MS (method 1): Rt = 0.54 min; MS (ESlpos): m/z = 539 [M+H]⁺
1H-NMR (400MHz, DMSO-d6): δ [ppm]= 1.52 (s, 3H), 1.54 (s, 3H), 1.60 (d, 3H), 1.98 - 2.12 (m, 2H), 3.37 - 3.48 (m, 3H), 3.51 - 3.59 (m, 1H), 4.15 (t, 2H), 5.67 - 5.74 (m, 1H), 5.91 (s, 2H), 6.21 (d, 1H), 6.28 (s, 1H), 7.30 - 7.34 (m, 3H), 7.38 (dd, 1H), 7.88 (d, 1H), 7.91 (dt, 1H), 8.43 (ddd, 2H), 8.47 (dd, 1H), 8.71 (d, 1H).

### Example 354

### (rac)-2'-[6-amino-5-(trifluoromethyl)pyridin-3-yl]-N-[2-(2-cyanophenyl)propan-2-yl]-5',6'-dihydrospiro[pyrrolidine-3,4'-pyrrolo[1,2-b]pyrazole]-1-carboxamide

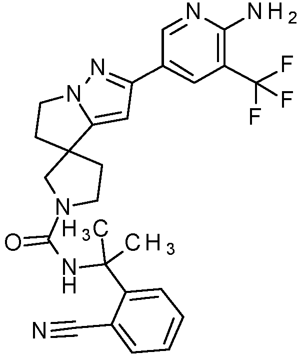

Example 354 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride salt (75.0 mg, 208 µmol) and 2-(2-aminopropan-2-yl)benzonitrile (40.1 mg, 250 µmol).
LC-MS (method 1): Rt = 1.34 min; MS (ESlpos): m/z = 510 [M+H]⁺
1H-NMR (400MHz, DMSO-d6): δ [ppm]= 1.49 and 1.50 (2s, 6H), 2.04 - 2.17 (m, 2H), 2.54 - 2.65 (m, 2H), 3.49 - 3.69 (m, 2H), 3.75 - 3.92 (m, 2H), 4.15 - 4.27 (m, 2H), 6.50 - 6.55 (m, 3H), 7.41 and 7.47 (2ddd, 1H), 7.54 - 7.65 (m, 2H), 7.71 and 7.77 (2d, 1H), 8.02 - 8.05 (m, 1H), 8.61 (s, 1H), 9.94 and 9.97 (2s, 1H).

### Example 355

### [2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazol]-1-yl](1H-imidazol-1-yl)methanone (mixture of stereoisomers)

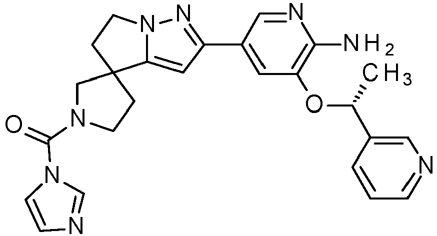

Example 355 was prepared in analogy to Example 31 using 5-[5',6'-dihydrospiro[pyrrolidine-3,4'-pyrrolo[1,2-b]pyrazol]-2'-yl]-3-[(1R)-1-(pyridin-3-yl)ethoxy]pyridin-2-amine-hydrochloride salt (82.7 mg, 79 % purity, 157 µmol) and 2-(1-methyl-1H-1,2,3-triazol-4-yl)propan-2-amine-hydrochloride salt (40.2 mg, 189 µmol).
LC-MS (method 1): Rt = 0.79 min; MS (ESlpos): m/z = 472 [M+H]⁺
1H-NMR (400MHz, DMSO-d6): δ [ppm]= 1.60 (d, 3H), 1.99-2.24 (m, 2H), 2.53-2.70 (m, 2H), 3.65-3.90 (m, 3H), 4.06 - 4.24 (m, 2H), 5.66-5.75 (m, 1H), 5.84-5.96 (m, 2H), 6.21-6.60 (m, 1H), 7.03 (br s, 1H), 7.32 (s, 1H), 7.37 (dd, 1H), 7.53-7.72 (m, 1H), 7.84-7.95 (m, 2H), 8.21 (br s, 1H), 8.44-8.50 (m, 1H), 8.71 (d, 1H)

### Example 356

### (rac)-2'-[6-amino-5-(trifluoromethyl)pyridin-3-yl]-N-(1-methylcyclobutyl)-5',6'-dihydrospiro[pyrrolidine-3,4'-pyrrolo[1,2-b]pyrazole]-1-carboxamide

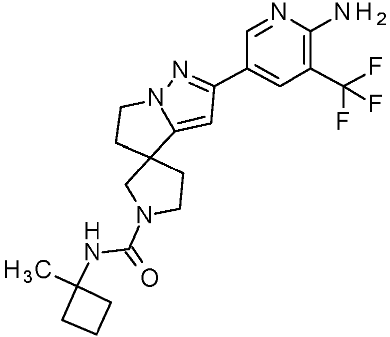

Example 356 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride salt (120 mg, 334 µmol) and 1-methylcyclobutan-1-amine-hydrochloride salt (48.7 mg, 400 µmol).
LC-MS (method 1): Rt = 1.04 min; MS (ESlpos): m/z = 435 [M+H]⁺
¹H NMR (400 MHz, DMSO-d6) ppm: δ = 8.59 (d, 1H), 8.01 (d, 1H), 6.54 (s, 2H), 6.47 (s, 1H), 5.97 (s, 1H), 5.76 (s, 1H), 4.18 (t, 2H), 3.46-3.55 (m, 1H), 3.35-3.43 (m, 3H), 2.52-2.55 (m, 2H), 2.24 (q, 2H), 1.99-2.10 (m, 2H), 1.85 (ddd, 2H), 1.73 (td, 2H), 1.38 (s, 3H)

### Example 357

### (rac)-2'-[6-amino-5-(trifluoromethyl)pyridin-3-yl]-N-(3-methyloxetan-3-yl)-5',6'-dihydrospiro[pyrrolidine-3,4'-pyrrolo[1,2-b]pyrazole]-1-carboxamide

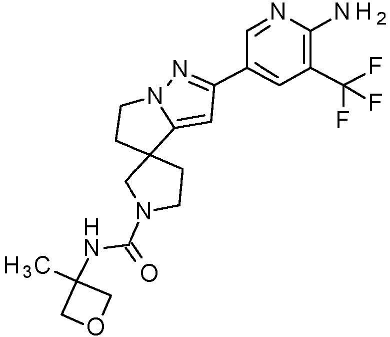

Example 357 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride salt (120 mg, 334 µmol) and 3-methyloxetan-3-amine (34.9 mg, 400 µmol).
LC-MS (method 1): Rt = 0.85 min; MS (ESlpos): m/z = 437 [M+H]⁺
¹H NMR (DMSO-d6) δ: 8.59 (d, 1H), 8.02 (d, 1H), 6.58 (s, 1H), 6.54 (s, 2H), 6.49 (s, 1H), 4.58 (dd, 2H), 4.24 (d, J=6.3 Hz, 2H), 4.19 (t, 2H), 3.47-3.56 (m, 1H), 3.37-3.45 (m, 3H), 2.52-2.57 (m, 2H), 2.00-2.13 (m, 2H), 1.52 (s, 3H)

### Example 358

### (rac)-2-[6-amino-5-(trifluoromethoxy)pyridin-3-yl]-N-[2-(3-fluoropyridin-2-yl)propan-2-yl]-6,7-dihydrospiro[pyrazolo[5,1-c][1,4]oxazine-4,3'-pyrrolidine]-1'-carboxamide

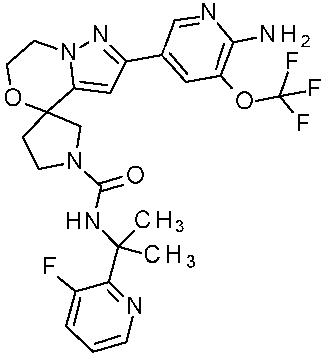

Example 358 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine-hydrochloride salt (75.0 mg, 191 µmol) and 2-(3-fluoropyridin-2-yl)propan-2-amine (35.4 mg, 230 µmol).
LC-MS (method 1): Rt = 1.04 min; MS (ESlpos): m/z = 536 [M+H]⁺
1H-NMR (400 MHz, DMSO-d6) δ [ppm]: 1.013 (0.71), 1.030 (0.81), 1.049 (1.89), 1.067 (16.00), 1.084 (14.94), 1.623 (2.01), 1.631 (1.90), 2.522 (0.65), 2.706 (0.73), 2.724 (0.75), 3.219 (0.53), 3.235 (0.68), 3.251 (0.53), 4.118 (0.58), 4.130 (0.74), 6.468 (0.59), 6.556 (0.88), 6.715 (1.09), 7.744 (0.48), 8.250 (1.24), 8.318 (0.41), 8.322 (0.90), 8.327 (0.76).

### Example 359

### 2'-{6-amino-5-[(1S)-1-phenylethoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide (mixture of stereoisomers)

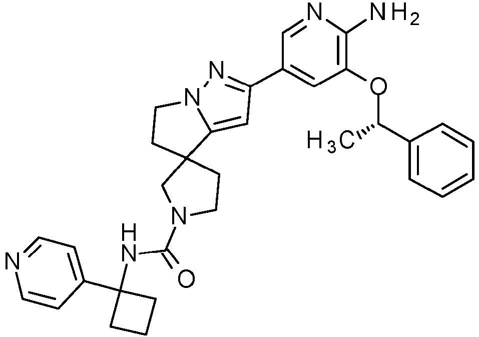

Example 359 was prepared in analogy to Example 31 using 5-[5',6'-dihydrospiro[pyrrolidine-3,4'-pyrrolo[1,2-b]pyrazol]-2'-yl]-3-[(1S)-1-phenylethoxy]pyridin-2-amine-hydrochloride salt (40.0 mg, 97.1 µmol) and 1-(pyridin-4-yl)cyclobutan-1-amine (17.3 mg, 117 µmol).
LC-MS (method 1): Rt = 1.05 min; MS (ESlpos): m/z = 550 [M+H]⁺
¹H-NMR (400 MHz, DMSO-d6) δ [ppm]: 1.066 (0.77), 1.137 (4.54), 1.153 (4.61), 1.232 (0.41), 1.567 (11.65), 1.583 (11.48), 1.822 (0.97), 1.845 (1.13), 1.863 (0.76), 1.907 (0.59), 2.030 (2.72), 2.046 (2.49), 2.073 (2.10), 2.322 (1.06), 2.326 (1.44), 2.332 (1.20), 2.375 (2.13), 2.390 (2.19), 2.411 (2.22), 2.435 (2.25), 2.522 (3.85), 2.539 (6.22), 2.664 (0.91), 2.668 (1.25), 2.673 (0.87), 2.727 (13.23), 2.887 (16.00), 3.416 (7.40), 3.520 (1.46), 4.116 (2.88), 4.133 (5.40), 4.150 (2.83), 5.568 (1.52), 5.575 (1.55), 5.585 (1.61), 5.591 (1.53), 5.874 (4.88), 6.222 (6.87), 6.224 (6.21), 6.865 (3.68), 7.225 (1.44), 7.238 (6.15), 7.242 (8.15), 7.261 (2.46), 7.319 (3.85), 7.338 (7.06), 7.356 (3.74), 7.384 (3.82), 7.392 (4.76), 7.396 (4.94), 7.400 (4.75), 7.407 (4.07), 7.452 (5.52), 7.468 (3.67), 7.473 (4.58), 7.834 (3.67), 7.838 (6.09), 7.842 (3.75), 7.950 (2.15), 8.457 (3.95), 8.466 (5.07), 8.477 (3.03), 8.481 (3.62).

### Example 360

### 2'-{6-amino-5-[(1S)-1-phenylethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide (mixture of stereoisomers)

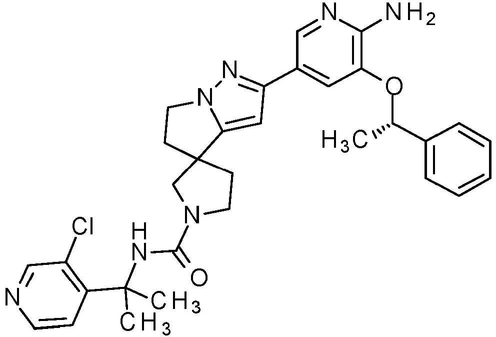

Example 360 was prepared in analogy to Example 31 using 5-[5',6'-dihydrospiro[pyrrolidine-3,4'-pyrrolo[1,2-b]pyrazol]-2'-yl]-3-[(1S)-1-phenylethoxy]pyridin-2-amine-hydrochloride salt (40.0 mg, 97.1 µmol) and 2-(3-chloropyridin-4-yl)propan-2-amine (19.9 mg, 117 µmol).
LC-MS (method 1): Rt = 1.10 min; MS (ESlpos): m/z = 572 [M+H]⁺
¹H-NMR (400 MHz, DMSO-d6) δ [ppm]: 1.066 (0.54), 1.232 (0.55), 1.349 (0.44), 1.548 (0.83), 1.569 (4.52), 1.585 (4.45), 1.650 (14.14), 2.018 (0.83), 2.033 (0.87), 2.074 (1.33), 2.469 (1.90), 2.518 (3.79), 2.523 (2.65), 2.728 (3.66), 2.888 (4.60), 3.387 (6.75), 3.475 (1.97), 3.565 (1.04), 4.107 (1.35), 4.124 (2.62), 4.142 (1.32), 5.568 (0.75), 5.583 (0.75), 5.850 (3.14), 6.214 (4.57), 6.320 (1.70), 6.325 (1.56), 7.034 (16.00), 7.228 (2.76), 7.233 (2.50), 7.244 (1.85), 7.263 (1.33), 7.319 (1.94), 7.329 (0.66), 7.339 (3.46), 7.357 (1.70), 7.421 (1.63), 7.427 (1.42), 7.434 (1.39), 7.440 (1.47), 7.454 (2.87), 7.475 (2.09), 7.536 (0.51), 7.549 (0.67), 7.565 (0.42), 7.596 (0.44), 7.625 (0.45), 7.684 (6.45), 7.831 (1.82), 7.835 (2.72), 7.839 (1.73), 7.950 (0.57), 8.138 (0.75), 8.385 (1.63), 8.392 (1.77), 8.399 (1.66), 8.405 (1.58), 8.416 (3.43), 8.420 (3.27), 8.568 (0.40), 8.581 (0.40), 8.611 (0.88).

### Example 361

### 2'-{6-amino-5-[(1R)-1-phenylethoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide (mixture of stereoisomers)

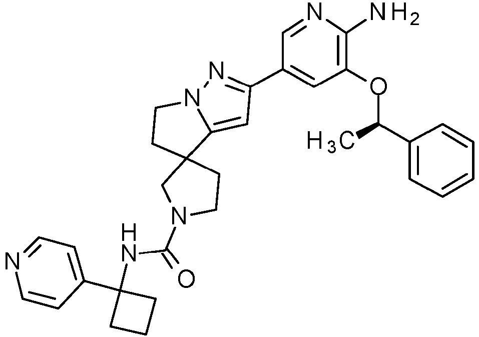

Example 361 was prepared in analogy to Example 31 using 5-[5',6'-dihydrospiro[pyrrolidine-3,4'-pyrrolo[1,2-b]pyrazol]-2'-yl]-3-[(1R)-1-phenylethoxy]pyridin-2-amine-hydrochloride salt (75.0 mg, 182 µmol) and 1-(pyridin-4-yl)cyclobutan-1-amine (32.4 mg, 218 µmol).
LC-MS (method 1): Rt = 1.05 min; MS (ESlpos): m/z = 550 [M+H]⁺
¹H-NMR (400 MHz, DMSO-d6) δ [ppm]: 1.067 (0.41), 1.552 (0.46), 1.568 (5.72), 1.584 (5.40), 1.753 (1.22), 1.824 (0.45), 1.846 (0.51), 1.995 (0.55), 2.003 (0.67), 2.018 (0.93), 2.031 (1.24), 2.047 (1.16), 2.376 (0.92), 2.391 (0.97), 2.414 (0.95), 2.437 (1.00), 2.463 (0.94), 2.523 (0.92), 2.727 (12.68), 2.729 (12.64), 2.887 (16.00), 3.388 (2.02), 3.418 (3.29), 3.504 (0.51), 3.521 (0.68), 4.117 (1.34), 4.135 (2.50), 4.152 (1.34), 5.572 (0.70), 5.578 (0.72), 5.587 (0.73), 5.594 (0.70), 5.888 (1.62), 6.224 (3.30), 6.227 (2.95), 6.869 (1.64), 7.226 (0.57), 7.243 (3.54), 7.263 (1.16), 7.279 (0.59), 7.319 (1.80), 7.339 (3.12), 7.357 (1.64), 7.386 (1.89), 7.390 (1.55), 7.393 (2.28), 7.398 (2.39), 7.402 (2.32), 7.405 (1.68), 7.409 (2.10), 7.454 (2.53), 7.470 (1.69), 7.474 (2.17), 7.837 (1.73), 7.841 (2.91), 7.845 (1.87), 7.951 (2.09), 8.459 (1.90), 8.463 (1.35), 8.468 (2.31), 8.471 (2.15), 8.474 (2.27), 8.478 (1.39), 8.483 (1.86).

### Example 362

### 2'-{6-amino-5-[(1R)-1-phenylethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide (mixture of stereoisomers)

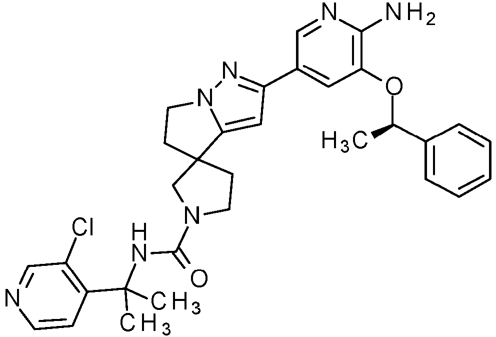

Example 362 was prepared in analogy to Example 31 using 5-[5',6'-dihydrospiro[pyrrolidine-3,4'-pyrrolo[1,2-b]pyrazol]-2'-yl]-3-[(1R)-1-phenylethoxy]pyridin-2-amine-hydrochloride salt (75.0 mg, 182 µmol) and 2-(3-chloropyridin-4-yl)propan-2-amine (37.3 mg, 218 µmol).
LC-MS (method 1): Rt = 1.10 min; MS (ESlpos): m/z = 572 [M+H]⁺
¹H-NMR (400 MHz, DMSO-d6) δ [ppm]: 1.066 (1.67), 1.156 (1.90), 1.561 (0.64), 1.574 (3.10), 1.589 (3.04), 1.650 (7.29), 2.020 (0.59), 2.034 (0.60), 2.454 (0.57), 2.471 (1.16), 2.518 (1.16), 2.523 (0.77), 2.729 (13.52), 2.888 (16.00), 3.389 (2.10), 4.111 (0.87), 4.128 (1.76), 4.145 (0.87), 5.585 (0.46), 5.590 (0.47), 5.601 (0.48), 5.607 (0.46), 5.981 (0.65), 6.226 (2.71), 6.319 (1.07), 6.325 (1.07), 7.229 (0.45), 7.247 (1.33), 7.254 (1.73), 7.258 (1.63), 7.266 (0.98), 7.322 (1.41), 7.341 (2.41), 7.355 (0.54), 7.359 (1.16), 7.421 (1.00), 7.428 (1.11), 7.434 (0.91), 7.440 (0.93), 7.457 (1.90), 7.478 (1.43), 7.827 (1.24), 7.831 (1.88), 7.835 (1.18), 7.951 (2.04), 8.386 (1.13), 8.392 (1.27), 8.399 (1.16), 8.406 (1.12), 8.416 (2.30), 8.420 (2.32).

### Example 363

### (rac)-2-[6-amino-5-(trifluoromethoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide

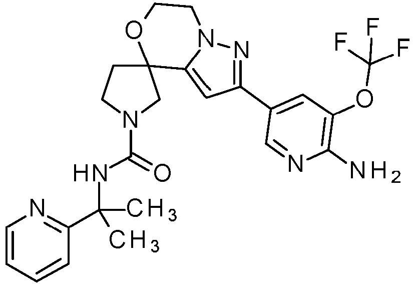

Example 363 was prepared in analogy to Example 31 using (*rac*)-5-[6,7-dihydrospiro[pyrazolo[5,1-c][1,4]oxazine-4,3'-pyrrolidin]-2-yl]-3-(trifluoromethoxy)pyridin-2-amine (75.0 mg, 211 µmol) and 2-(pyridin-2-yl)propan-2-amine (34.5 mg, 253 µmol).

¹H-NMR (400 MHz, DMSO-d6) δ [ppm]: 1.137 (1.06), 1.233 (1.00), 1.256 (0.46), 1.578 (6.63), 1.584 (6.51), 2.116 (0.48), 2.323 (0.48), 2.327 (0.72), 2.332 (0.62), 2.345 (0.40), 2.518 (1.77), 2.523 (1.25), 2.540 (16.00), 2.669 (0.48), 3.408 (0.43), 3.499 (0.50), 3.528 (0.59), 3.620 (0.56), 3.796 (0.67), 3.824 (0.57), 4.109 (0.68), 4.130 (2.19), 4.140 (1.66), 6.425 (1.68), 6.557 (2.69), 6.729 (3.53), 7.171 (0.64), 7.174 (0.67), 7.183 (0.67), 7.186 (0.76), 7.190 (0.76), 7.192 (0.69), 7.202 (0.72), 7.204 (0.72), 7.440 (1.21), 7.459 (1.31), 7.698 (0.70), 7.702 (0.73), 7.716 (0.86), 7.721 (0.86), 7.736 (0.61), 7.740 (0.79), 7.746 (1.12), 7.750 (1.46), 7.754 (0.98), 8.329 (2.33), 8.334 (2.32), 8.449 (0.79), 8.451 (0.90), 8.453 (0.92), 8.455 (0.81), 8.461 (0.80), 8.463 (0.90), 8.465 (0.84), 8.467 (0.72).

### Example 364

### 2'-{6-amino-5-[(1R)-1-(pyridin-2-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide (mixture of stereoisomers)

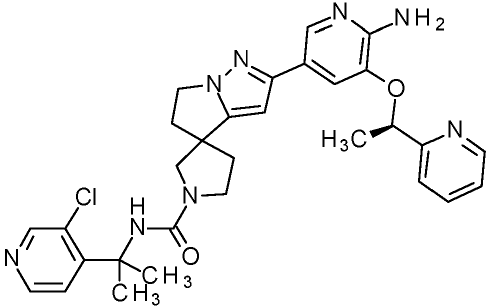

Example 364 was prepared in analogy to Example 31 using 5-[5',6'-dihydrospiro[pyrrolidine-3,4'-pyrrolo[1,2-b]pyrazol]-2'-yl]-3-[(1R)-1-(pyridin-2-yl)ethoxy]pyridin-2-amine-hydrochloride salt (120 mg, 291 µmol) and 2-(3-chloropyridin-4-yl)propan-2-amine (59.5 mg, 349 µmol).
LC-MS (method 1): Rt = 0.96 min; MS (ESlpos): m/z = 573 [M+H]⁺
¹H-NMR (400 MHz, DMSO-d6) δ [ppm]: 1.065 (4.29), 1.155 (2.81), 1.206 (0.93), 1.222 (1.17), 1.231 (0.69), 1.239 (1.24), 1.247 (3.20), 1.257 (2.27), 1.263 (3.31), 1.271 (3.05), 1.276 (1.34), 1.288 (2.87), 1.347 (0.46), 1.409 (1.34), 1.535 (2.51), 1.551 (3.02), 1.559 (8.01), 1.629 (5.56), 1.648 (16.00), 1.769 (7.60), 2.021 (0.97), 2.030 (1.04), 2.038 (0.97), 2.518 (3.67), 2.523 (2.51), 3.112 (0.42), 3.123 (0.42), 3.131 (0.43), 3.141 (0.41), 3.383 (4.69), 3.473 (0.77), 3.565 (0.63), 4.114 (1.47), 4.132 (2.90), 4.149 (1.50), 5.562 (1.09), 5.578 (1.09), 5.633 (0.45), 5.649 (0.45), 6.128 (0.68), 6.245 (3.74), 6.247 (4.04), 6.324 (3.10), 6.542 (1.18), 7.103 (0.96), 7.107 (1.00), 7.281 (2.48), 7.284 (2.43), 7.294 (1.27), 7.296 (1.28), 7.300 (1.23), 7.303 (1.14), 7.313 (1.10), 7.315 (1.07), 7.336 (1.06), 7.350 (1.03), 7.420 (1.86), 7.423 (1.93), 7.433 (1.95), 7.436 (1.98), 7.455 (1.56), 7.459 (1.00), 7.467 (0.96), 7.471 (1.49), 7.507 (1.72), 7.527 (3.31), 7.532 (1.80), 7.776 (1.11), 7.781 (1.15), 7.795 (1.77), 7.800 (1.82), 7.814 (0.90), 7.819 (0.90), 7.849 (2.17), 7.852 (3.04), 7.856 (2.28), 8.316 (1.49), 8.329 (1.39), 8.384 (2.36), 8.390 (2.28), 8.398 (2.17), 8.403 (2.02), 8.419 (4.33), 8.425 (2.73), 8.434 (3.93), 8.545 (2.85), 8.549 (2.42), 8.557 (2.37), 8.560 (2.71), 8.636 (1.00), 8.649 (0.95), 8.685 (1.82).

### Example 365

### 2'-{6-amino-5-[(1R)-1-(pyridin-3-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide (mixture of stereoisomers)

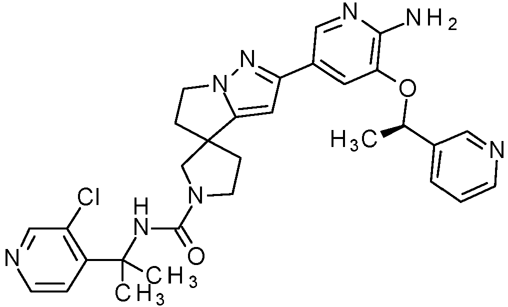

Example 365 was prepared in analogy to Example 31 using 5-[5',6'-dihydrospiro[pyrrolidine-3,4'-pyrrolo[1,2-b]pyrazol]-2'-yl]-3-[(1R)-1-(pyridin-3-yl)ethoxy]pyridin-2-amine-hydrochloride salt (210 mg, 509 µmol) and 2-(3-chloropyridin-4-yl)propan-2-amine (104 mg, 610 µmol).
LC-MS (method 1): Rt = 0.91 min; MS (ESlpos): m/z = 573 [M+H]⁺
¹H-NMR (400 MHz, DMSO-d6) δ [ppm]: 1.052 (0.59), 1.222 (0.42), 1.230 (0.91), 1.498 (5.44), 1.595 (6.96), 1.611 (7.01), 1.650 (16.00), 1.708 (4.96), 2.026 (1.15), 2.040 (1.21), 2.048 (0.99), 2.058 (0.61), 2.067 (0.50), 2.083 (0.64), 2.459 (1.09), 2.468 (1.13), 2.476 (2.31), 2.518 (1.70), 2.523 (1.10), 3.166 (13.81), 3.447 (1.48), 3.464 (1.14), 3.483 (1.19), 4.119 (1.83), 4.137 (3.72), 4.154 (1.77), 5.696 (1.01), 5.700 (1.03), 5.711 (1.04), 5.716 (1.00), 5.915 (4.77), 6.256 (4.78), 6.258 (5.16), 6.326 (2.25), 6.332 (2.28), 7.315 (3.27), 7.320 (3.26), 7.359 (1.50), 7.361 (1.52), 7.371 (1.54), 7.373 (1.55), 7.379 (1.61), 7.381 (1.60), 7.391 (1.62), 7.393 (1.59), 7.422 (1.84), 7.426 (1.88), 7.434 (1.92), 7.440 (1.85), 7.860 (2.92), 7.863 (3.43), 7.864 (3.36), 7.866 (2.87), 7.901 (1.05), 7.906 (1.68), 7.911 (1.07), 7.920 (0.99), 7.925 (1.59), 7.929 (0.99), 8.386 (2.96), 8.391 (3.00), 8.399 (2.68), 8.404 (2.61), 8.417 (5.15), 8.425 (5.10), 8.451 (0.56), 8.463 (2.89), 8.467 (2.77), 8.475 (2.69), 8.479 (2.49), 8.486 (0.75), 8.707 (2.94), 8.712 (2.92).

### Example 366

### 2'-{6-amino-5-[(1R)-1-(pyridin-4-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide (mixture of stereoisomers)

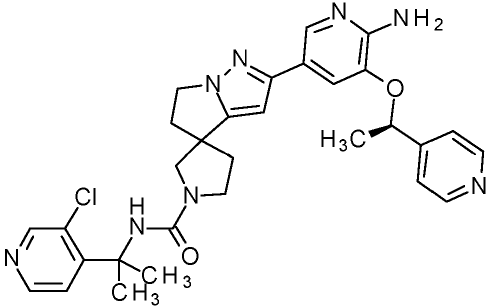

Example 366 was prepared in analogy to Example 31 using 5-[5',6'-dihydrospiro[pyrrolidine-3,4'-pyrrolo[1,2-b]pyrazol]-2'-yl]-3-[(1R)-1-(pyridin-4-yl)ethoxy]pyridin-2-amine-hydrochloride salt (250 mg, 605 µmol) and 2-(3-chloropyridin-4-yl)propan-2-amine (124 mg, 727 µmol).
LC-MS (method 1): Rt = 0.90 min; MS (ESlpos): m/z = 573 [M+H]⁺
¹H-NMR (400 MHz, DMSO-d6) δ [ppm]: 1.497 (0.44), 1.561 (3.39), 1.575 (8.11), 1.647 (8.44), 2.017 (0.62), 2.033 (0.63), 2.451 (0.57), 2.459 (0.54), 2.467 (1.11), 2.476 (1.07), 2.518 (0.63), 2.523 (0.46), 3.165 (16.00), 3.336 (0.73), 3.428 (1.04), 3.446 (0.61), 3.459 (0.51), 3.463 (0.51), 3.476 (0.58), 3.564 (2.02), 4.105 (0.97), 4.123 (1.96), 4.140 (0.93), 5.642 (0.59), 5.657 (0.59), 5.949 (2.47), 6.234 (3.94), 6.333 (1.25), 6.337 (1.22), 7.232 (1.53), 7.420 (0.97), 7.425 (0.97), 7.433 (1.02), 7.438 (0.93), 7.475 (2.71), 7.479 (1.62), 7.486 (1.66), 7.490 (2.74), 7.867 (1.41), 7.870 (2.03), 7.875 (1.43), 8.385 (1.51), 8.389 (1.44), 8.398 (1.46), 8.402 (1.36), 8.415 (3.48), 8.532 (3.71), 8.536 (2.00), 8.543 (2.04), 8.547 (3.39).

### Example 367

### 2'-(6-amino-5-cyanopyridin-3-yl)-N-[(1R)-1-(pyridin-4-yl)ethyl]-5',6'-dihydrospiro[pyrrolidine-3,4'-pyrrolo[1,2-b]pyrazole]-1-carboxamide (isomer 1)

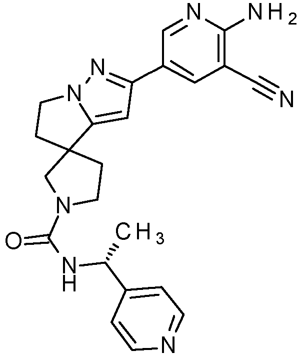

Example 367 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile-hydrochloride salt (150 mg, 90 % purity, 426 µmol) and (1R)-1-(pyridin-4-yl)ethan-1-amine (62.5 mg, 511 µmol).

The mixture of isomers was then separated by chrial HPLC to give 30.0 mg (98 % purity, 16 % yield) of the title compound. Instrument: PrepCon Labomatic HPLC-3; Column: YMC Cellulose SB 10µ, 250x50; eluent A: methyl tert-butyl ether + 0.1 vol % diethylamine; eluent B: acetonitrile + 0.1 vol % diethylamine; isocratic: 50%A+50%B; flow: 100 mL/min; temperature: 25°C; UV: 280 nm
Retention time: 7.4 - 8.9 min
LC-MS (method 1): Rt = 0.76 min; MS (ESlpos): m/z = 429 [M+H]⁺
¹H-NMR (400MHz, DMSO-d₆): δ [ppm]= 1.37 (d, 3H), 2.07 (br t, 2H), 2.52 - 2.58 (m, 2H), 3.41 - 3.60 (m, 4H), 4.19 (t, 2H), 4.81 (quin, 1H), 6.45 (s, 1H), 6.61 (d, 1H), 6.99 (s, 2H), 7.29 - 7.35 (m, 2H), 8.15 (d, 1H), 8.43 - 8.49 (m, 2H), 8.62 (d, 1H).
α²⁰_{D}: -96.4° (c=1, in MeOH)

### Example 368

### 2'-(6-amino-5-cyanopyridin-3-yl)-N-[(1R)-1-(pyridin-4-yl)ethyl]-5',6'-dihydrospiro[pyrrolidine-3,4'-pyrrolo[1,2-b]pyrazole]-1-carboxamide (isomer 2)

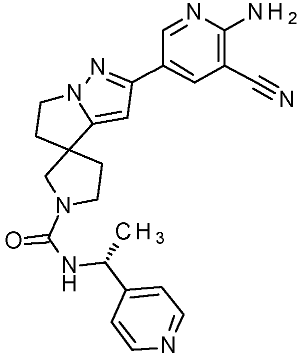

The mixture of isomers was separated by chrial HPLC to give 35.0 mg (100 % purity, 18 % yield) of the title compound.

Instrument: PrepCon Labomatic HPLC-3; Column: YMC Cellulose SB 10µ, 250x50; eluent A: methyl tert-butyl ether + 0.1 vol % diethylamine; eluent B: acetonitrile + 0.1 vol % diethylamine; isocratic: 50%A+50%B; flow: 100 mL/min; temperature: 25°C; UV: 280 nm
Retention time: 12.2 - 14.7min
α²⁰_{D}: +61.3° (c=1, in MeOH)

### Example 369

### 2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (enantiomer 1)

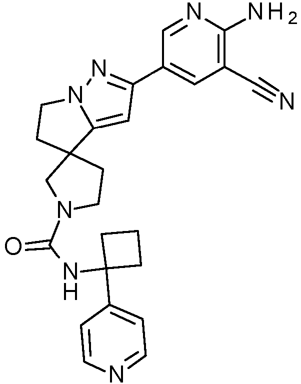

Example 369 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile-hydrochloride salt (150 mg, 90 % purity, 426 µmol) and 1-(pyridin-4-yl)cyclobutan-1-amine (75.8 mg, 511 µmol). The racemate (rac)-2-amino-5-[5',6'-dihydrospiro[pyrrolidine-3,4'-pyrrolo[1,2-b]pyrazol]-2'-yl]pyridine-3-carbonitrile-hydrochloride salt (150 mg, 90 % purity, 426 µmol) was then separated by chrial HPLC to give 21.3 mg (95 % purity, 10 % yield) of the title compound.

Instrument: PrepCon Labomatic HPLC-3; Column: YMC Cellulose SB 10µ, 250x50; eluent A: methyl tert-butyl ether + 0.1 vol % diethylamine; eluent B: acetonitrile + 0.1 vol % diethylamine; isocratic: 10%A+90%B; flow: 100 mL/min; temperature: 25°C; UV: 254 nm
Retention time: 9.0 - 10.7 min;
LC-MS (method 1): Rt = 0.83 min; MS (ESlpos): m/z = 455 [M+H]⁺
¹H-NMR (400MHz, CHLOROFORM-d): δ [ppm]= 1.94 (br d, 1H), 2.07 - 2.20 (m, 2H), 2.22 - 2.32 (m, 1H), 2.44 - 2.53 (m, 2H), 2.54 - 2.74 (m, 4H), 3.48 - 3.59 (m, 3H), 3.65 (dq, 1H), 4.27 (t, 2H), 4.83 (s, 1H), 5.21 (s, 2H), 6.15 (s, 1H), 7.33 - 7.43 (m, 2H), 8.07 (d, 1H), 8.58 (br d, 2H), 8.62 (d, 1H).
α²⁰_{D}: -63.2° (c=1, in CHCl3)

### Example 370

### (3R)-2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide

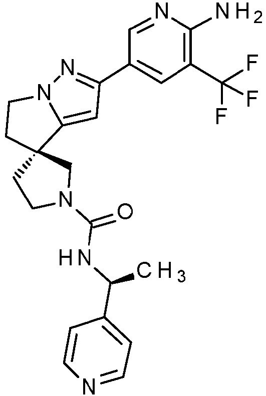

Example 370 was prepared in analogy to Example 31 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (60.0 mg, 84 % purity, 140 µmol) and (1S)-1-(pyridin-4-yl)ethan-1-amine (20.5 mg, 168 µmol).
α²⁰_{D}: +60.6° (c=1, in CHCl3)
1H-NMR (400MHz, CHLOROFORM-d): δ [ppm]= 1.47 - 1.53 (m, 3H), 2.12 (ddd, 1H), 2.23 - 2.33 (m, 1H), 2.54 - 2.73 (m, 2H), 3.52 - 3.63 (m, 3H), 3.68 (br dd, 1H), 4.28 (t, 2H), 4.45 (br d, 1H), 4.94 - 5.08 (m, 3H), 6.19 (s, 1H), 7.17 - 7.38 (m, 6H), 8.10 (d, 1H), 8.48 - 8.63 (m, 3H).

### Example 371

### (3S)-2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide

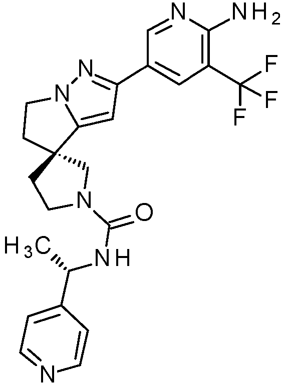

Example 371 was prepared in analogy to Example 31 using 5-[(3S)-5',6'-dihydrospiro[pyrrolidine-3,4'-pyrrolo[1,2-b]pyrazol]-2'-yl]-3-(trifluoromethyl)pyridin-2-amine-hydrochloride salt (60.0 mg, 84 % purity, 140 µmol) and (1S)-1-(pyridin-4-yl)ethan-1-amine (20.5 mg, 168 µmol).
α²⁰_{D}: -40,9° (c=1, in CHCl3)
1H-NMR (400MHz, CHLOROFORM-d): δ [ppm]= 1.51 (d, 3H), 2.06 - 2.19 (m, 1H), 2.22 - 2.35 (m, 1H), 2.54 - 2.75 (m, 2H), 3.52 - 3.64 (m, 3H), 3.64 - 3.73 (m, 1H), 4.28 (t, 2H), 4.46 (br d, 1H), 4.92 - 5.14 (m, 3H), 6.20 (s, 1H), 7.24 (d, 1H), 8.10 (d, 1H), 8.48 - 8.63 (m, 3H).

### Example 372

### 2'-(6-amino-5-cyanopyridin-3-yl)-N-[(1S)-1-(pyridin-4-yl)ethyl]-5',6'-dihydrospiro[pyrrolidine-3,4'-pyrrolo[1,2-b]pyrazole]-1-carboxamide (isomer 1)

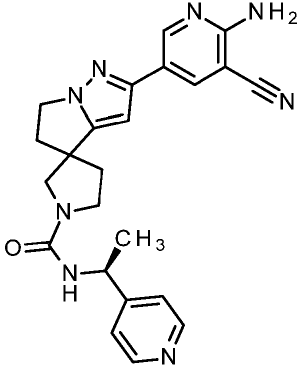

Example 372 was prepared in analogy to Example 31 using (rac)-2-amino-5-[5',6'-dihydrospiro[pyrrolidine-3,4'-pyrrolo[1,2-b]pyrazol]-2'-yl]pyridine-3-carbonitrile-hydrochloride salt (113 mg, 90 % purity, 320 µmol) and (1S)-1-(pyridin-4-yl)ethan-1-amine (43.0 mg, 352 µmol). The mixture of isomers was separated by chrial HPLC to give 2.6 mg (95 % purity, 2 % yield) of the title compound.

Instrument: PrepCon Labomatic HPLC-3; Column: YMC Cellulose SB 10µ, 250x50; eluent A: methyl tert-butyl ether + 0.1 vol % diethylamine; eluent B: acetonitrile; isocratic: 70%A+30%B; flow: 100 mL/min; temperature: 25°C; UV: 254 nm;
Retention time: 14.26 - 15.98 min;
α²⁰_{D}: -50.5° (c=1, in MeOH)
¹H-NMR (400 MHz, DMSO-d6) δ [ppm]: 0.848 (0.50), 1.170 (0.40), 1.229 (2.25), 1.360 (5.24), 1.378 (5.26), 2.053 (0.96), 2.071 (1.82), 2.088 (0.93), 2.518 (1.77), 2.523 (1.70), 2.526 (1.61), 2.543 (2.18), 2.561 (1.47), 3.322 (0.52), 3.329 (0.74), 3.336 (0.81), 3.340 (0.88), 3.412 (1.20), 3.419 (0.87), 3.424 (1.16), 3.429 (0.85), 3.454 (2.54), 3.468 (2.53), 3.486 (1.07), 3.493 (0.87), 3.502 (0.63), 3.515 (0.54), 3.532 (0.99), 3.548 (0.59), 3.558 (0.56), 4.166 (1.49), 4.184 (2.46), 4.201 (1.47), 4.791 (0.68), 4.810 (1.04), 4.828 (0.67), 5.748 (16.00), 6.449 (7.65), 6.610 (1.28), 6.630 (1.22), 6.984 (4.01), 7.327 (1.48), 8.144 (3.69), 8.149 (3.95), 8.469 (0.45), 8.611 (3.27), 8.617 (3.20).

### Example 373

### 2'-(6-amino-5-cyanopyridin-3-yl)-N-[(1S)-1-(pyridin-4-yl)ethyl]-5',6'-dihydrospiro[pyrrolidine-3,4'-pyrrolo[1,2-b]pyrazole]-1-carboxamide (isomer 2)

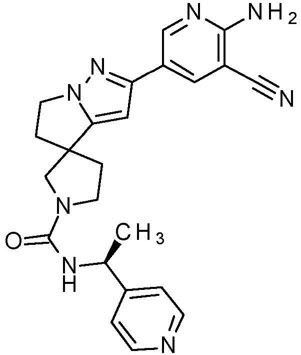

The mixture of isomers was separated by chrial HPLC to give 3.50 mg (95 % purity, 2 % yield) of the title compound.

Instrument: PrepCon Labomatic HPLC-3; Column: YMC Cellulose SB 10µ, 250x50; eluent A: methyl tert-butyl ether + 0.1 vol % diethylamine; eluent B: acetonitrile; isocratic: 70%A+30%B; flow: 100 mL/min; temperature: 25°C; UV: 254 nm;
Retention time: 20.07 - 24.33 min;
α²⁰_{D}: 67.9° (c=1, in MeOH)

### Example 374

### 2'-[6-amino-5-(trifluoromethyl)pyridin-3-yl]-N-[(1S)-1-(pyridin-4-yl)ethyl]-5',6'-dihydrospiro[azetidine-3,4'-pyrrolo[1,2-b]pyrazole]-1-carboxamide

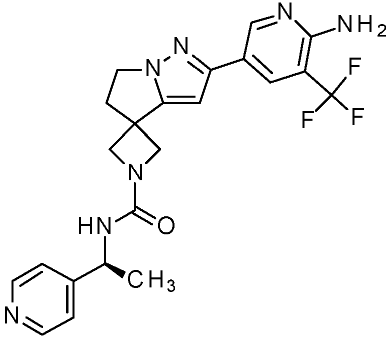

Example 374 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (100 mg, 57 % purity, 165 µmol) and (1S)-1-(pyridin-4-yl)ethan-1-amine (30.2 mg, 247 µmol).
1H NMR (400 MHz, DMSO-d6) δ ppm 8.62 (d, J=1.5 Hz, 1 H) 8.46 - 8.53 (m, 1 H) 8.04 (d, J=2.3 Hz, 1 H) 7.30 - 7.37 (m, 2 H) 7.01 (d, J=8.1 Hz, 1 H, NH) 6.74 (s, 1 H) 6.55 (s, 2 H, NH2) 4.79 (quin, J=7.4 Hz, 1 H) 4.13 (t, J=7.0 Hz, 2 H) 3.96 - 4.10 (m, 4 H) 2.86 (t, J=6.8 Hz, 2 H) 1.36 (d, J=7.1 Hz, 3 H)
LC-MS (method 1): Rt = 0.86 min; MS (ESlpos): m/z = 459 [M+H]⁺

### Example 375

### 2'-[6-amino-5-(trifluoromethyl)pyridin-3-yl]-N-[(1R)-1-(pyridin-4-yl)ethyl]-5',6'-dihydrospiro[azetidine-3,4'-pyrrolo[1,2-b]pyrazole]-1-carboxamide

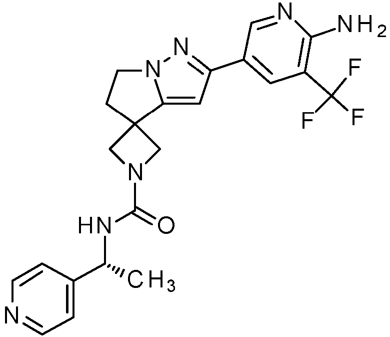

Example 375 was prepared in analogy to Example 31 using 5-(5',6'-dihydrospiro[azetidine-3,4'-pyrrolo[1,2-b]pyrazol]-2'-yl)-3-(trifluoromethyl)pyridin-2-amine-hydrochloride salt (100 mg, 57 % purity, 165 µmol) and (1R)-1-(pyridin-4-yl)ethan-1-amine-hydrochloride salt (39.2 mg, 247 µmol).
1H NMR (400 MHz, DMSO-d6) δ ppm 8.62 (d, J=1.5 Hz, 1 H) 8.48 - 8.53 (m, 2 H) 8.04 (d, J=2.0 Hz, 1 H) 7.31 - 7.36 (m, 2 H) 7.21 - 7.27 (m, 1 H) 7.01 (d, J=8.1 Hz, 1 H, NH) 6.74 (s, 1 H) 6.55 (s, 2 H, NH2) 4.79 (m, 1 H) 4.13 (t, J=6.8 Hz, 2 H) 3.99 - 4.11 (m, 4 H) 2.86 (t, J=6.8 Hz, 2 H) 1.36 (d, J=7.1 Hz, 3 H)
LC-MS (method 1): Rt = 0.86 min; MS (ESlpos): m/z = 458 [M+H]⁺

### Example 376

### (rac)-2'-[6-amino-5-(trifluoromethyl)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide

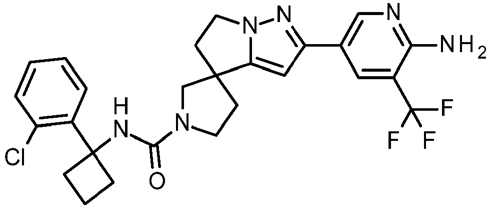

Example 376 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride (75.0 mg, 208 µmol) and 1-(2-Chlorophenyl)cyclobutan-1-amine-hydrochloride (50.0 mg, 229 µmol).

1H-NMR (400 MHz, DMSO-d6) δ [ppm]: 1.649 (0.74), 1.659 (1.29), 1.670 (1.83), 1.682 (1.83), 1.686 (1.89), 1.697 (2.04), 1.708 (1.54), 1.719 (0.94), 1.730 (0.52), 2.004 (2.70), 2.017 (4.47), 2.030 (5.91), 2.047 (4.86), 2.069 (2.58), 2.074 (2.81), 2.095 (1.61), 2.116 (0.45), 2.322 (0.67), 2.326 (0.95), 2.332 (0.64), 2.434 (0.42), 2.452 (1.89), 2.467 (4.34), 2.476 (6.63), 2.518 (6.13), 2.522 (5.23), 2.539 (2.60), 2.548 (3.18), 2.569 (1.41), 2.597 (1.96), 2.608 (2.31), 2.621 (3.72), 2.632 (3.25), 2.639 (2.61), 2.644 (2.93), 2.655 (2.90), 2.664 (1.86), 2.668 (2.43), 2.678 (1.42), 3.355 (2.66), 3.365 (3.07), 3.382 (7.91), 3.393 (9.26), 3.420 (2.19), 3.451 (1.46), 3.469 (2.70), 3.484 (1.96), 3.493 (1.76), 3.509 (0.87), 4.143 (5.61), 4.161 (11.19), 4.178 (5.36), 6.344 (12.15), 6.520 (10.84), 6.554 (16.00), 7.155 (2.53), 7.159 (2.66), 7.173 (6.08), 7.178 (6.23), 7.192 (5.65), 7.197 (5.41), 7.224 (4.29), 7.228 (5.50), 7.243 (6.22), 7.247 (7.62), 7.261 (3.30), 7.265 (3.30), 7.273 (9.75), 7.276 (8.03), 7.291 (6.50), 7.295 (5.85), 7.528 (6.30), 7.532 (6.33), 7.547 (5.70), 7.551 (5.41), 7.973 (8.88), 7.978 (8.93), 8.544 (8.28), 8.548 (8.09).

### Example 377

### (rac)-2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide

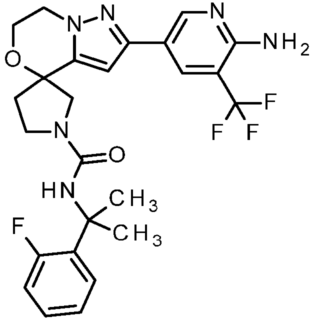

Example 2 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride and 2-(2-fluorophenyl)propan-2-amine-hydrochloride.

1H-NMR (400 MHz, DMSO-d6) δ [ppm]: 1.066 (0.63), 1.629 (15.95), 1.635 (16.00), 2.212 (0.78), 2.221 (0.87), 2.247 (1.23), 2.270 (0.66), 2.315 (1.04), 2.327 (1.43), 2.346 (0.72), 2.362 (0.61), 2.522 (1.29), 3.363 (0.72), 3.389 (1.23), 3.406 (1.24), 3.431 (0.57), 3.471 (1.84), 3.501 (2.15), 3.570 (0.94), 3.591 (1.66), 3.613 (0.75), 3.779 (1.95), 3.808 (1.66), 4.069 (0.59), 4.084 (1.25), 4.101 (1.78), 4.119 (2.96), 4.130 (5.04), 4.142 (5.33), 6.114 (5.49), 6.600 (6.89), 6.760 (10.59), 7.030 (1.27), 7.033 (1.42), 7.050 (1.64), 7.053 (1.85), 7.062 (1.32), 7.065 (1.58), 7.074 (1.64), 7.078 (1.68), 7.084 (2.16), 7.093 (3.41), 7.096 (2.79), 7.112 (2.30), 7.115 (1.95), 7.190 (0.85), 7.194 (1.02), 7.202 (1.01), 7.207 (1.42), 7.214 (1.35), 7.221 (1.32), 7.226 (1.32), 7.233 (0.70), 7.240 (0.61), 7.245 (0.55), 7.300 (1.39), 7.303 (1.38), 7.321 (2.22), 7.341 (1.22), 7.345 (1.07), 8.014 (4.23), 8.019 (4.31), 8.583 (4.00), 8.587 (3.98).

### Example 378

### (rac)-2-[6-amino-5-(trifluoromethyl)pyridin-3-yl]-N-(1-phenylcyclobutyl)-6,7-dihydrospiro[pyrazolo[5,1-c][1,4]oxazine-4,3'-pyrrolidine]-1'-carboxamide

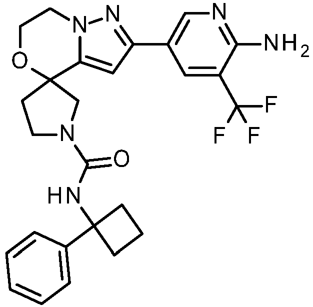

Example 3 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine and 1-phenylcyclobutan-1-amine-hydrochloride.

1H-NMR (400 MHz, DMSO-d6) δ [ppm]: 0.999 (0.75), 1.016 (0.86), 1.042 (0.43), 1.742 (0.89), 1.759 (1.68), 1.764 (1.79), 1.780 (1.79), 1.786 (2.00), 1.803 (1.25), 1.825 (0.50), 1.964 (0.86), 1.970 (1.07), 1.986 (1.90), 2.009 (1.75), 2.013 (1.68), 2.030 (1.04), 2.036 (1.00), 2.052 (0.64), 2.074 (0.64), 2.176 (0.61), 2.207 (1.36), 2.233 (1.90), 2.257 (1.00), 2.318 (2.04), 2.322 (2.86), 2.326 (3.94), 2.331 (3.15), 2.351 (1.72), 2.368 (2.29), 2.374 (2.43), 2.398 (3.65), 2.419 (2.90), 2.441 (3.54), 2.464 (4.94), 2.518 (8.88), 2.522 (5.69), 2.664 (1.54), 2.669 (2.08), 2.673 (1.54), 3.379 (2.47), 3.449 (2.40), 3.478 (2.72), 3.558 (1.61), 3.580 (2.76), 3.603 (1.29), 3.757 (3.29), 3.785 (2.83), 4.051 (1.11), 4.066 (2.04), 4.081 (2.79), 4.099 (3.22), 4.111 (3.54), 4.119 (5.87), 4.133 (10.49), 4.184 (0.79), 6.418 (1.22), 6.555 (0.97), 6.599 (12.78), 6.702 (8.20), 6.748 (16.00), 7.138 (1.54), 7.141 (2.76), 7.144 (1.83), 7.159 (6.80), 7.164 (2.68), 7.174 (2.68), 7.178 (4.65), 7.181 (2.58), 7.256 (0.61), 7.270 (7.80), 7.290 (12.42), 7.303 (2.54), 7.309 (7.19), 7.413 (10.99), 7.416 (12.74), 7.433 (9.91), 7.437 (8.09), 8.004 (7.59), 8.009 (7.55), 8.572 (7.55), 8.576 (7.41).

### Example 379

### (rac)-2'-[6-amino-5-(trifluoromethyl)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide

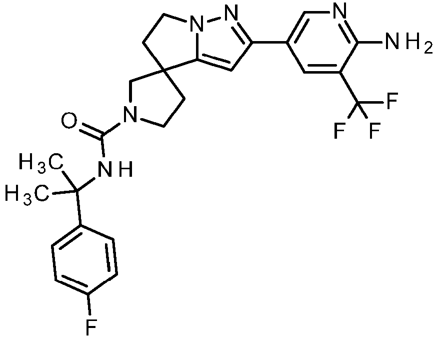

Example 4 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(4-fluorophenyl)propan-2-amine.

1H-NMR (400 MHz, DMSO-d6) δ [ppm]: 1.547 (15.16), 1.556 (16.00), 2.024 (0.40), 2.038 (1.09), 2.052 (2.16), 2.069 (2.59), 2.073 (3.02), 2.086 (1.07), 2.099 (0.53), 2.518 (3.81), 2.534 (3.73), 2.539 (3.86), 2.553 (2.37), 3.394 (0.53), 3.413 (1.09), 3.420 (1.32), 3.449 (5.63), 3.476 (0.57), 3.532 (0.59), 3.549 (1.12), 3.564 (0.86), 3.575 (0.82), 3.590 (0.43), 4.175 (2.65), 4.193 (4.91), 4.210 (2.63), 6.071 (5.18), 6.456 (11.78), 6.558 (6.90), 7.020 (3.48), 7.025 (1.21), 7.042 (7.50), 7.059 (1.29), 7.064 (3.99), 7.072 (0.49), 7.351 (3.60), 7.357 (1.63), 7.365 (4.02), 7.374 (3.80), 7.382 (1.47), 7.388 (3.30), 8.008 (3.90), 8.013 (3.95), 8.148 (0.63), 8.591 (3.68), 8.596 (3.63).

### Example 380

### (rac)-2'-[6-amino-5-(trifluoromethyl)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide

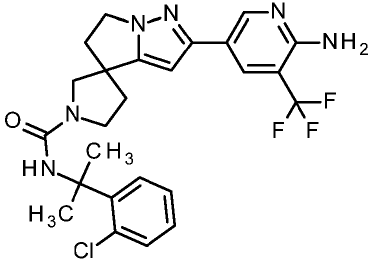

Example 5 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(2-chlorophenyl)propan-2-amine-hydrochloride.

¹H-NMR (400 MHz, DMSO-d6) δ [ppm]: 1.231 (0.74), 1.683 (16.00), 1.692 (15.08), 2.033 (1.15), 2.047 (2.21), 2.064 (2.62), 2.081 (1.09), 2.094 (0.51), 2.336 (0.60), 2.518 (11.25), 2.522 (6.20), 2.529 (2.70), 2.678 (0.65), 3.382 (0.71), 3.416 (8.52), 3.443 (0.79), 3.488 (0.68), 3.506 (1.27), 3.521 (0.92), 3.530 (0.82), 3.546 (0.42), 4.162 (2.58), 4.180 (5.48), 4.198 (2.51), 5.759 (8.09), 6.121 (5.58), 6.432 (11.74), 6.559 (7.21), 7.157 (1.11), 7.161 (1.12), 7.176 (2.60), 7.180 (2.73), 7.195 (2.50), 7.199 (2.38), 7.227 (1.68), 7.231 (2.24), 7.247 (2.35), 7.250 (2.99), 7.265 (1.51), 7.269 (1.51), 7.275 (4.51), 7.279 (3.76), 7.294 (3.07), 7.298 (2.67), 7.457 (2.72), 7.461 (2.81), 7.477 (2.35), 7.481 (2.26), 7.995 (4.07), 8.000 (4.11), 8.204 (0.64), 8.575 (3.81), 8.579 (3.77).

### Example 381

### 2-[6-amino-5-(trifluoromethyl)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide (mixture of stereoisomer)

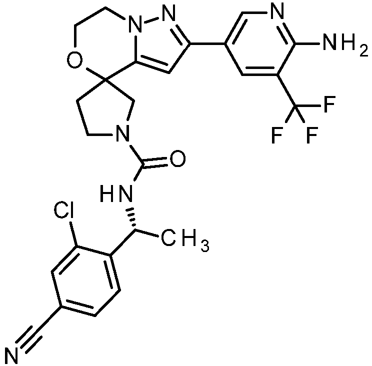

Example 6 was prepared in analogy to Example 31 using 5-[6,7-dihydrospiro[pyrazolo[5,1-c][1,4]oxazine-4,3'-pyrrolidin]-2-yl]-3-(trifluoromethyl)pyridin-2-amine-hydrochloride salt (75 mg, 200 µmol) and 4-[(1R)-1-aminoethyl]-3-chlorobenzonitrile (37 mg, 205 µmol).

1H-NMR (400 MHz, DMSO-d6) δ [ppm]: 0.829 (0.56), 1.052 (0.68), 1.329 (15.85), 1.347 (16.00), 2.073 (5.58), 2.202 (0.49), 2.226 (1.48), 2.254 (1.78), 2.278 (0.82), 2.322 (0.51), 2.327 (0.74), 2.332 (0.85), 2.354 (1.81), 2.518 (1.97), 2.522 (1.27), 2.669 (0.51), 3.387 (1.42), 3.404 (1.60), 3.430 (1.68), 3.449 (2.11), 3.478 (1.55), 3.514 (1.26), 3.544 (1.53), 3.559 (1.05), 3.583 (1.54), 3.606 (0.68), 3.644 (0.88), 3.666 (1.57), 3.689 (0.72), 3.773 (1.93), 3.802 (1.60), 3.850 (1.98), 3.877 (1.71), 4.052 (0.76), 4.066 (1.61), 4.082 (2.54), 4.100 (2.81), 4.112 (3.46), 4.123 (4.58), 4.132 (6.17), 4.138 (8.08), 4.144 (9.33), 5.108 (0.48), 5.120 (1.82), 5.126 (2.02), 5.138 (2.90), 5.144 (3.04), 5.156 (1.95), 5.162 (1.92), 5.172 (0.49), 6.600 (12.74), 6.760 (8.29), 6.766 (9.95), 6.784 (2.39), 6.803 (2.28), 6.822 (2.24), 6.841 (2.09), 7.661 (4.45), 7.681 (7.00), 7.700 (5.28), 7.819 (3.39), 7.823 (3.51), 7.832 (3.83), 7.836 (4.19), 7.839 (3.00), 7.843 (2.97), 7.851 (2.84), 7.856 (3.00), 7.988 (10.66), 7.991 (10.30), 8.008 (4.39), 8.014 (6.71), 8.021 (4.68), 8.136 (3.18), 8.576 (4.07), 8.581 (4.19), 8.587 (4.49), 8.592 (4.20).

### Example 382

### 2'-[6-amino-5-(trifluoromethyl)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide (mixture of stereoisomers)

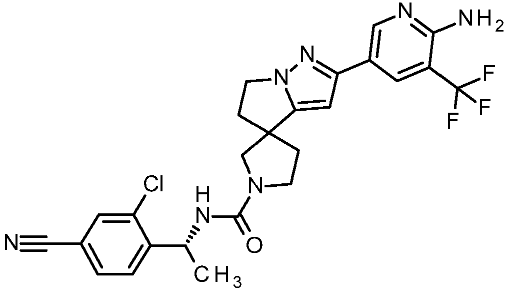

Example 7 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride and 4-[(1R)-1-aminoethyl]-3-chlorobenzonitrile.

1H-NMR (400 MHz, DMSO-d6) δ [ppm]: 1.323 (9.01), 1.330 (9.90), 1.341 (9.38), 1.347 (9.52), 2.074 (3.85), 2.083 (3.94), 2.518 (4.04), 2.523 (5.54), 2.539 (15.01), 2.558 (3.80), 3.382 (0.47), 3.429 (2.86), 3.453 (5.44), 3.483 (3.00), 3.493 (3.75), 3.518 (2.25), 3.530 (1.50), 3.546 (0.94), 3.556 (0.75), 3.574 (0.70), 3.594 (0.94), 4.172 (3.05), 4.177 (3.28), 4.190 (5.35), 4.194 (5.96), 4.207 (3.19), 4.212 (2.82), 5.111 (0.56), 5.128 (2.53), 5.146 (3.94), 5.164 (2.53), 5.181 (0.56), 6.472 (16.00), 6.544 (12.57), 6.773 (2.21), 6.789 (3.66), 6.807 (2.16), 7.642 (3.05), 7.663 (3.94), 7.705 (2.96), 7.725 (4.08), 7.775 (2.39), 7.778 (2.39), 7.795 (1.83), 7.799 (1.92), 7.808 (3.24), 7.812 (3.28), 7.828 (2.11), 7.832 (2.25), 7.980 (6.71), 7.984 (8.73), 7.989 (6.10), 8.014 (6.57), 8.133 (0.70), 8.590 (6.57), 8.595 (6.43).

### Example 383

### 2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide (mixture of stereoisomers)

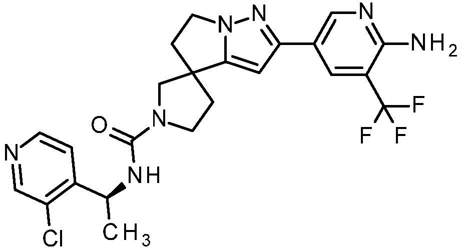

Example 8 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride and (1S)-1-(3-chloropyridin-4-yl)ethan-1-amine-hydrochloride.

¹H-NMR (400 MHz, DMSO-d6) δ [ppm]: 1.232 (0.89), 1.332 (12.11), 1.336 (12.56), 1.350 (12.44), 1.353 (12.48), 1.902 (3.12), 2.085 (4.66), 2.323 (1.66), 2.327 (2.35), 2.332 (1.70), 2.518 (10.21), 2.523 (6.97), 2.530 (5.51), 2.548 (7.90), 2.566 (4.82), 2.660 (0.81), 2.665 (1.74), 2.669 (2.43), 2.673 (1.74), 3.420 (0.81), 3.438 (3.40), 3.464 (6.64), 3.491 (4.62), 3.520 (2.55), 3.539 (1.78), 3.555 (1.09), 3.565 (0.89), 3.583 (0.81), 3.603 (1.13), 3.627 (0.85), 4.179 (4.17), 4.195 (7.37), 4.211 (3.93), 5.036 (0.57), 5.054 (2.35), 5.072 (3.61), 5.088 (2.39), 5.106 (0.61), 6.469 (16.00), 6.552 (13.73), 6.762 (2.63), 6.779 (3.93), 6.795 (2.43), 7.454 (3.48), 7.466 (3.56), 7.532 (3.77), 7.544 (3.85), 8.014 (7.53), 8.019 (7.74), 8.375 (0.41), 8.435 (4.09), 8.448 (3.89), 8.477 (5.87), 8.490 (5.63), 8.525 (11.50), 8.528 (11.42), 8.588 (7.53), 8.593 (7.45).

### Example 384

### (rac)-2'-[6-amino-5-(trifluoromethyl)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide

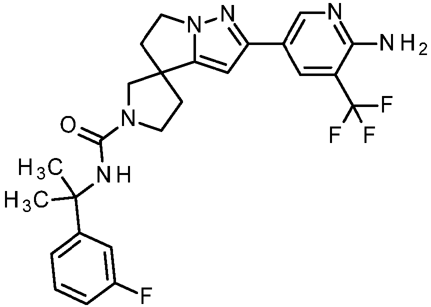

Example 9 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(3-fluorophenyl)propan-2-amine.

1H-NMR (400 MHz, DMSO-d6) δ [ppm]: 1.550 (14.22), 1.556 (16.00), 2.047 (1.03), 2.062 (1.97), 2.076 (2.35), 2.092 (1.00), 2.327 (0.78), 2.523 (4.68), 2.544 (3.82), 2.561 (2.43), 2.669 (0.78), 3.426 (1.55), 3.451 (3.61), 3.466 (3.18), 3.492 (0.78), 3.542 (0.54), 3.559 (1.01), 4.178 (2.35), 4.196 (4.13), 4.213 (2.35), 6.121 (4.44), 6.444 (8.64), 6.556 (6.03), 6.925 (0.79), 6.931 (0.92), 6.946 (1.50), 6.951 (1.89), 6.967 (0.88), 6.974 (0.93), 7.112 (1.64), 7.117 (1.33), 7.140 (1.63), 7.175 (1.73), 7.196 (2.52), 7.257 (1.22), 7.276 (2.11), 7.292 (1.90), 7.312 (0.76), 8.003 (3.38), 8.009 (3.60), 8.584 (3.25), 8.588 (3.36).

### Example 385

### (rac)-2-[6-amino-5-(trifluoromethyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide

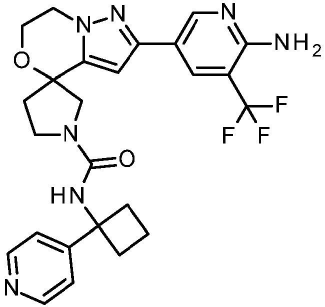

Example 10 was prepared in analogy to Example 31 using (rac)-5-[6,7-dihydrospiro[pyrazolo[5,1-c][1,4]oxazine-4,3'-pyrrolidin]-2-yl]-3-(trifluoromethyl)pyridin-2-amine-hydrochloride and 1-(pyridin-4-yl)cyclobutan-1-amine.

¹H-NMR (400 MHz, DMSO-d6) δ [ppm]: 1.009 (0.86), 1.026 (0.86), 1.231 (1.35), 1.822 (2.02), 1.843 (2.40), 1.861 (1.59), 2.007 (2.35), 2.030 (2.16), 2.074 (6.87), 2.221 (1.87), 2.245 (2.40), 2.269 (1.30), 2.327 (5.33), 2.344 (3.84), 2.387 (5.24), 2.411 (7.78), 2.430 (7.35), 2.451 (5.24), 2.669 (2.88), 3.471 (2.45), 3.498 (2.74), 3.602 (3.17), 3.770 (3.75), 3.797 (3.36), 4.062 (1.44), 4.078 (2.93), 4.093 (3.56), 4.129 (10.38), 4.141 (14.32), 6.604 (16.00), 6.765 (14.75), 6.884 (8.84), 7.039 (0.82), 7.386 (15.23), 7.390 (10.19), 7.402 (14.75), 7.638 (0.48), 8.010 (9.61), 8.014 (9.90), 8.140 (14.94), 8.205 (0.58), 8.476 (15.57), 8.480 (10.09), 8.491 (14.32), 8.577 (8.98).

### Example 386

### (rac)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide

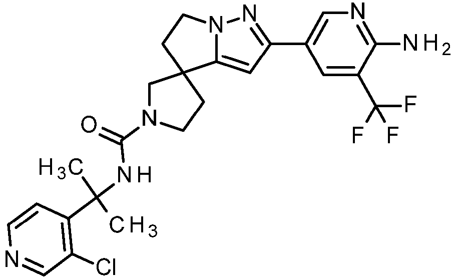

Example 11 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride and 2-(3-chloropyridin-4-yl)propan-2-amine.

¹H-NMR (400 MHz, DMSO-d6) δ [ppm]: 1.164 (0.49), 1.232 (0.79), 1.247 (0.70), 1.257 (0.82), 1.332 (0.78), 1.560 (0.62), 1.654 (16.00), 2.036 (0.59), 2.051 (1.09), 2.067 (1.30), 2.084 (0.56), 2.322 (0.47), 2.327 (0.65), 2.332 (0.48), 2.518 (3.72), 2.522 (2.95), 2.537 (0.96), 2.664 (0.46), 2.669 (0.66), 2.673 (0.48), 2.728 (2.37), 2.888 (3.01), 3.417 (4.16), 3.440 (0.54), 3.505 (0.64), 3.521 (0.48), 3.528 (0.43), 4.165 (1.43), 4.183 (3.04), 4.200 (1.41), 5.758 (1.02), 6.332 (2.86), 6.443 (5.96), 6.558 (3.76), 7.427 (2.41), 7.440 (2.44), 8.001 (2.08), 8.006 (2.16), 8.147 (3.29), 8.389 (3.42), 8.402 (3.34), 8.408 (6.45), 8.578 (1.96), 8.581 (1.98).

### Example 387

### (rac)-2'-[6-amino-5-(trifluoromethyl)pyridin-3-yl]-N-(1-phenylcyclobutyl)-5',6'-dihydrospiro[pyrrolidine-3,4'-pyrrolo[1,2-b]pyrazole]-1-carboxamide

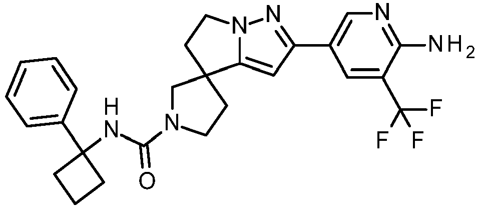

Example 12 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine-hydrochloride and 1-phenylcyclobutan-1-amine-hydrochloride.

1H-NMR (400 MHz, DMSO-d6) δ [ppm]: 0.999 (0.66), 1.015 (0.69), 1.042 (0.42), 1.056 (1.13), 1.066 (0.98), 1.072 (1.13), 1.153 (3.41), 1.171 (6.70), 1.189 (3.35), 1.230 (2.11), 1.243 (2.86), 1.248 (1.82), 1.259 (3.21), 1.262 (3.13), 1.279 (2.49), 1.745 (0.76), 1.762 (1.46), 1.768 (1.56), 1.784 (1.62), 1.790 (1.80), 1.807 (1.14), 1.829 (0.44), 1.957 (0.79), 1.963 (0.94), 1.979 (1.86), 1.986 (13.20), 2.002 (1.85), 2.021 (1.54), 2.033 (2.34), 2.048 (3.61), 2.061 (4.05), 2.078 (1.98), 2.326 (0.65), 2.354 (1.36), 2.376 (2.75), 2.385 (3.45), 2.401 (4.11), 2.407 (3.48), 2.422 (2.50), 2.447 (2.01), 2.470 (3.84), 2.526 (7.40), 2.530 (6.83), 2.546 (4.36), 2.668 (0.61), 3.127 (0.43), 3.383 (0.98), 3.401 (3.15), 3.426 (8.82), 3.436 (6.98), 3.462 (1.72), 3.509 (1.57), 3.526 (2.71), 3.542 (2.34), 3.552 (2.35), 3.569 (1.92), 3.662 (3.47), 3.999 (1.02), 4.016 (2.82), 4.034 (2.80), 4.052 (1.00), 4.141 (0.47), 4.167 (4.66), 4.184 (8.94), 4.201 (4.69), 6.424 (16.00), 6.597 (1.82), 6.702 (7.56), 6.745 (0.79), 7.011 (0.86), 7.127 (2.25), 7.146 (5.62), 7.164 (3.82), 7.255 (6.46), 7.275 (10.79), 7.294 (6.17), 7.419 (9.98), 7.437 (8.42), 7.440 (6.54), 7.700 (0.55), 8.009 (6.99), 8.014 (7.10), 8.574 (6.54), 8.579 (6.46).

### Example 388

### (rac)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide

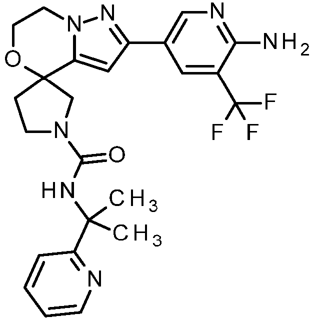

Example 13 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine-hydrochloride and 2-(pyridin-2-yl)propan-2-amine.

1H-NMR (400 MHz, DMSO-d6) δ [ppm]: 1.177 (0.43), 1.194 (0.45), 1.232 (0.58), 1.579 (16.00), 1.585 (15.80), 2.075 (1.24), 2.240 (0.63), 2.265 (0.86), 2.290 (0.45), 2.332 (1.59), 2.337 (1.21), 2.352 (0.88), 2.368 (0.48), 2.385 (0.43), 2.518 (5.38), 2.523 (3.58), 2.540 (0.66), 2.674 (1.09), 2.679 (0.48), 3.387 (0.68), 3.411 (0.96), 3.429 (0.91), 3.454 (0.45), 3.501 (1.19), 3.530 (1.41), 3.605 (0.73), 3.626 (1.31), 3.648 (0.61), 3.801 (1.59), 3.829 (1.34), 4.097 (0.86), 4.115 (1.56), 4.136 (5.15), 4.146 (3.89), 6.425 (4.06), 6.601 (5.70), 6.776 (9.44), 7.171 (1.62), 7.174 (1.72), 7.184 (1.64), 7.186 (1.89), 7.190 (1.89), 7.193 (1.74), 7.202 (1.84), 7.205 (1.84), 7.438 (1.74), 7.440 (3.08), 7.458 (2.07), 7.461 (3.33), 7.698 (1.79), 7.702 (1.74), 7.717 (2.07), 7.721 (2.12), 7.736 (1.41), 7.740 (1.31), 8.017 (3.41), 8.022 (3.46), 8.163 (2.22), 8.449 (1.84), 8.451 (2.15), 8.453 (2.25), 8.455 (1.92), 8.461 (1.92), 8.463 (2.22), 8.465 (2.07), 8.467 (1.77), 8.587 (3.18), 8.591 (3.18).

### Example 389

### (rac)-2-[6-amino-5-(trifluoromethyl)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide

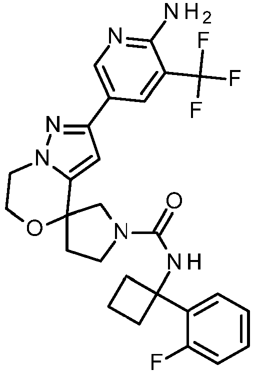

Example 389 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine hydrochloride (100 mg, 266 µmol) and 1-(2-Fluorophenyl)cyclobutan-1-amine (52.8 mg, 319 µmol).

¹H-NMR (400 MHz, DMSO-d6) δ [ppm]: 0.917 (0.45), 0.932 (3.79), 0.934 (1.29), 0.948 (3.92), 0.952 (0.66), 1.401 (1.31), 1.425 (1.11), 1.707 (0.51), 1.714 (0.62), 1.719 (0.76), 1.727 (1.17), 1.733 (1.19), 1.741 (1.29), 1.748 (1.09), 1.755 (1.37), 1.762 (0.80), 1.768 (0.86), 1.775 (0.60), 2.014 (0.90), 2.033 (1.54), 2.055 (1.42), 2.060 (1.40), 2.074 (4.20), 2.080 (0.88), 2.156 (0.45), 2.187 (1.05), 2.213 (1.44), 2.238 (0.76), 2.292 (1.31), 2.308 (1.60), 2.323 (1.68), 2.327 (2.01), 2.332 (1.46), 2.336 (1.05), 2.405 (0.45), 2.423 (0.45), 2.454 (0.78), 2.518 (7.24), 2.523 (6.95), 2.665 (0.80), 2.669 (1.13), 2.673 (0.82), 3.370 (0.86), 3.417 (2.05), 3.445 (2.40), 3.520 (1.27), 3.542 (2.30), 3.565 (1.03), 3.732 (2.58), 3.761 (2.20), 4.030 (0.90), 4.045 (1.44), 4.061 (2.22), 4.080 (2.15), 4.092 (2.71), 4.102 (2.99), 4.122 (7.51), 6.595 (16.00), 6.729 (11.84), 7.041 (1.81), 7.044 (1.99), 7.061 (2.40), 7.064 (2.71), 7.072 (5.03), 7.091 (8.70), 7.110 (3.32), 7.113 (2.60), 7.197 (1.21), 7.201 (1.37), 7.209 (1.40), 7.214 (2.13), 7.221 (1.83), 7.228 (1.74), 7.233 (1.99), 7.239 (0.96), 7.248 (0.86), 7.252 (0.80), 7.404 (1.85), 7.409 (1.87), 7.425 (3.04), 7.444 (1.74), 7.995 (5.76), 8.000 (5.93), 8.560 (5.46), 8.564 (5.39).

### Example 390

### (rac)-2'-[6-amino-5-(trifluoromethyl)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide

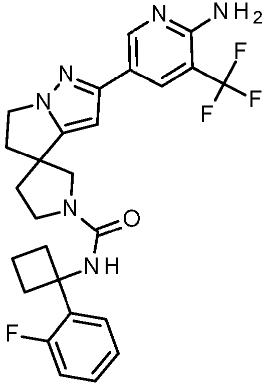

Example 390 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) and 1-(2-Fluorophenyl)cyclobutan-1-amine (27.6 mg, 167 µmol).

¹H-NMR (400 MHz, DMSO-d6) δ [ppm]: 0.949 (2.09), 0.965 (1.93), 1.704 (0.68), 1.712 (0.78), 1.717 (0.95), 1.725 (1.50), 1.731 (1.53), 1.739 (1.61), 1.745 (1.40), 1.753 (1.94), 1.766 (1.09), 1.774 (0.79), 1.787 (0.42), 2.009 (3.17), 2.026 (5.12), 2.036 (5.21), 2.049 (3.24), 2.054 (3.63), 2.068 (1.57), 2.084 (0.62), 2.094 (0.46), 2.323 (0.56), 2.327 (0.78), 2.332 (0.58), 2.457 (1.19), 2.518 (8.72), 2.524 (10.01), 2.547 (3.28), 2.576 (0.82), 2.665 (0.60), 2.669 (0.82), 2.673 (0.60), 3.368 (3.24), 3.397 (10.10), 3.403 (9.09), 3.429 (1.35), 3.470 (1.27), 3.488 (2.33), 3.496 (1.54), 3.502 (1.70), 3.514 (1.54), 3.529 (0.83), 4.151 (5.04), 4.169 (10.49), 4.186 (4.86), 6.389 (16.00), 6.549 (13.29), 6.582 (8.78), 7.025 (2.30), 7.027 (2.59), 7.045 (2.99), 7.048 (3.41), 7.054 (2.45), 7.057 (2.82), 7.064 (3.24), 7.067 (2.99), 7.077 (3.83), 7.083 (7.41), 7.086 (5.55), 7.102 (4.27), 7.105 (3.41), 7.182 (1.58), 7.186 (1.81), 7.195 (1.84), 7.200 (2.73), 7.206 (2.30), 7.214 (2.33), 7.219 (2.42), 7.225 (1.21), 7.233 (1.12), 7.238 (1.02), 7.407 (2.45), 7.412 (2.47), 7.428 (3.84), 7.431 (3.65), 7.448 (2.22), 7.452 (1.94), 7.984 (7.45), 7.989 (7.57), 8.556 (6.98), 8.561 (6.86).

### Example 391

### (rac)-2'-[6-amino-5-(trifluoromethyl)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide

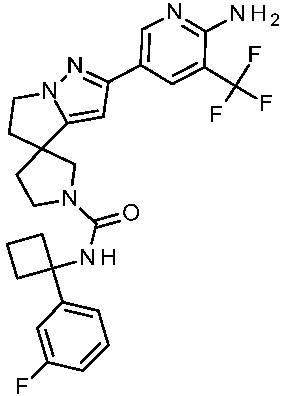

Example 391 was prepared in analogy to Example 31 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) and 1-(3-Fluorophenyl)cyclobutan-1-amine hydrochloride (33.6 mg, 167 µmol).

¹H-NMR (400 MHz, DMSO-d6) δ [ppm]: 0.945 (2.14), 0.961 (2.04), 1.753 (0.63), 1.760 (0.62), 1.776 (1.24), 1.781 (1.28), 1.798 (1.32), 1.803 (1.53), 1.820 (0.93), 1.959 (0.60), 1.966 (0.77), 1.981 (1.45), 1.997 (1.07), 2.004 (1.38), 2.009 (1.43), 2.024 (1.18), 2.032 (1.18), 2.041 (1.77), 2.055 (2.85), 2.068 (3.17), 2.084 (1.64), 2.097 (0.59), 2.323 (0.58), 2.327 (0.81), 2.332 (0.60), 2.348 (0.98), 2.372 (2.15), 2.380 (2.68), 2.388 (2.09), 2.396 (3.24), 2.402 (2.73), 2.418 (2.12), 2.430 (1.87), 2.453 (3.13), 2.460 (2.26), 2.470 (2.50), 2.476 (2.49), 2.518 (5.46), 2.523 (3.29), 2.532 (5.37), 2.536 (5.37), 2.551 (3.64), 2.665 (0.60), 2.669 (0.84), 2.674 (0.58), 3.389 (0.88), 3.406 (2.63), 3.431 (6.90), 3.442 (5.07), 3.468 (1.18), 3.516 (0.88), 3.533 (1.69), 3.548 (1.22), 3.558 (1.21), 3.574 (0.60), 4.169 (4.12), 4.187 (7.49), 4.204 (3.99), 6.421 (16.00), 6.551 (10.69), 6.763 (7.02), 6.949 (1.18), 6.952 (1.28), 6.955 (1.49), 6.958 (1.47), 6.971 (2.56), 6.975 (2.46), 6.978 (2.78), 6.991 (1.39), 6.993 (1.49), 6.998 (1.62), 7.000 (1.55), 7.163 (2.04), 7.169 (2.63), 7.173 (2.23), 7.190 (2.12), 7.195 (2.67), 7.200 (2.16), 7.259 (2.58), 7.279 (5.25), 7.298 (3.05), 7.313 (3.16), 7.317 (3.96), 7.333 (3.82), 7.337 (1.62), 7.352 (1.45), 7.994 (6.07), 7.999 (6.17), 8.570 (5.65), 8.575 (5.62).

### Example 392

### (rac)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide

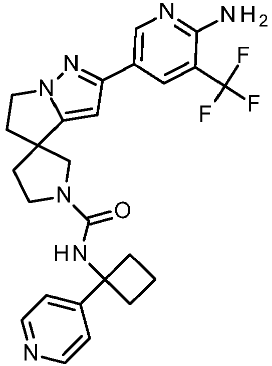

Example 392 was prepared in analogy to Example 31 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (50.0 mg, 139 µmol) and 1-(Pyrdin-4-yl)cyclobutan-1-amine hydrochloride (36.9 mg, 167 µmol).

¹H-NMR (400 MHz, DMSO-d6) δ [ppm]: 0.840 (0.44), 0.858 (0.44), 0.919 (0.61), 0.934 (4.85), 0.950 (4.81), 1.237 (0.44), 1.803 (0.78), 1.825 (1.59), 1.848 (2.00), 1.866 (1.22), 1.888 (0.54), 2.000 (1.76), 2.023 (1.80), 2.050 (2.81), 2.065 (3.93), 2.078 (4.07), 2.323 (1.46), 2.327 (2.07), 2.332 (1.49), 2.344 (1.36), 2.375 (3.56), 2.391 (4.34), 2.413 (4.00), 2.444 (3.76), 2.518 (10.68), 2.523 (9.05), 2.537 (6.64), 2.543 (6.54), 2.558 (4.00), 2.665 (1.49), 2.669 (2.03), 2.673 (1.46), 3.445 (10.37), 3.549 (2.00), 4.173 (4.92), 4.190 (9.39), 4.208 (4.75), 6.458 (16.00), 6.556 (13.08), 6.872 (8.03), 7.390 (12.75), 7.395 (8.24), 7.402 (8.41), 7.406 (12.61), 8.009 (7.36), 8.014 (7.32), 8.458 (14.58), 8.461 (8.71), 8.469 (8.61), 8.473 (13.69), 8.584 (6.98), 8.588 (6.78).

### Example 393

### (rac)-2-[6-amino-5-(trifluoromethyl)pyridin-3-yl]-N-[1-(3-fluorophenyl)cyclobutyl]-6,7-dihydrospiro[pyrazolo[5,1-c][1,4]oxazine-4,3'-pyrrolidine]-1'-carboxamide

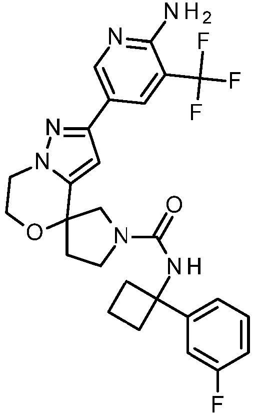

Example 393 was prepared in analogy to Example 31 using (*rac*)-5-[6,7-dihydrospiro[pyrazolo[5,1-c][1,4]oxazine-4,3'-pyrrolidin]-2-yl]-3-(trifluoromethyl)pyridin-2-amine hydrochloride (100 mg, 266 µmol) and 1-(3-Fluorophenyl)cyclobutan-1-amine hydrochloride (64.4 mg, 319 µmol).

¹H-NMR (400 MHz, DMSO-d6) δ [ppm]: 0.917 (1.89), 0.931 (16.00), 0.934 (5.08), 0.948 (15.70), 0.952 (2.62), 1.004 (0.61), 1.021 (0.61), 1.401 (1.10), 1.425 (0.91), 1.753 (0.85), 1.759 (0.67), 1.770 (1.43), 1.776 (1.55), 1.787 (1.13), 1.793 (1.55), 1.798 (1.79), 1.809 (0.76), 1.815 (1.16), 1.836 (0.46), 1.966 (0.73), 1.971 (0.94), 1.988 (1.67), 1.993 (1.34), 2.004 (1.16), 2.010 (1.55), 2.015 (1.49), 2.026 (0.82), 2.031 (0.79), 2.038 (0.79), 2.182 (0.55), 2.214 (1.25), 2.240 (1.67), 2.264 (0.88), 2.322 (2.34), 2.327 (2.46), 2.332 (2.46), 2.336 (2.31), 2.347 (1.73), 2.358 (1.83), 2.364 (2.28), 2.370 (2.65), 2.380 (2.89), 2.387 (3.25), 2.396 (3.22), 2.404 (4.59), 2.410 (2.83), 2.422 (5.05), 2.440 (4.08), 2.446 (4.29), 2.463 (3.25), 2.471 (2.68), 2.478 (2.68), 2.518 (5.08), 2.523 (3.22), 2.665 (0.85), 2.669 (1.19), 2.673 (0.88), 2.940 (1.00), 2.957 (1.31), 2.973 (0.97), 3.370 (1.73), 3.389 (1.64), 3.413 (0.79), 3.456 (1.79), 3.485 (2.07), 3.562 (1.37), 3.584 (2.40), 3.608 (1.10), 3.762 (2.98), 3.791 (2.49), 4.056 (0.94), 4.070 (1.86), 4.086 (2.68), 4.112 (3.68), 4.121 (5.78), 4.135 (9.46), 6.598 (12.14), 6.753 (14.14), 6.769 (7.39), 6.965 (1.46), 6.967 (1.61), 6.971 (1.86), 6.974 (1.79), 6.987 (3.10), 6.993 (3.44), 7.007 (1.67), 7.009 (1.73), 7.014 (1.92), 7.015 (1.76), 7.164 (2.43), 7.169 (3.13), 7.174 (2.68), 7.191 (2.46), 7.195 (3.25), 7.201 (2.52), 7.253 (3.44), 7.273 (5.54), 7.311 (3.29), 7.326 (3.47), 7.331 (4.65), 7.347 (4.41), 7.350 (2.13), 7.366 (1.83), 8.004 (7.03), 8.009 (7.15), 8.543 (0.49), 8.572 (6.66), 8.576 (6.57).

### Example 394

### (3'R)-2-[6-amino-5-(trifluoromethoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide

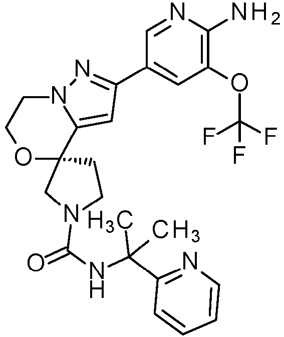

A solution of 2-(pyridin-2-yl)propan-2-amine (50.0 mg, 367 µmol), CDI (64.9 mg, 400 µmol) and DIPEA (230 µl, 1.3 mmol) in DMF (2 mL) was stirred at RT for 1h. 5-[(3'R)-6,7-dihydrospiro[pyrazolo[5,1-c][1,4]oxazine-4,3'-pyrrolidin]-2-yl]-3-(trifluoromethoxy)pyridin-2-amine hydrogen chloride (218 mg, 60 % purity, 334 µmol) in DMF (1.5 mL) was added and the mixture was stirred at RT overnight. The crude was directly purified by basic HPLC to give 14.0 mg (99 % purity, 8 % yield) of the title compound.
1H NMR (DMSO-d6, 400 MHz) δ 8.4-8.5 (m, 1H), 8.33 (d, 1H), 7.7-7.8 (m, 2H), 7.4-7.5 (m, 1H), 7.19 (ddd, 1H), 6.73 (s, 1H), 6.56 (s, 2H), 6.42 (s, 1H), 4.1-4.2 (m, 4H), 3.81 (d, 1H), 3.62 (br t, 1H), 3.51 (br d, 1H), 3.4-3.5 (m, 1H), 2.2-2.3 (m, 1H), 1.58 (d, 6H)
LC-MS (method 1): Rt = 1.00 min; MS (ESlneg): m/z = 516 [M-H]⁻

### Example 395

### (3'R)-2-[6-amino-5-(trifluoromethyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide

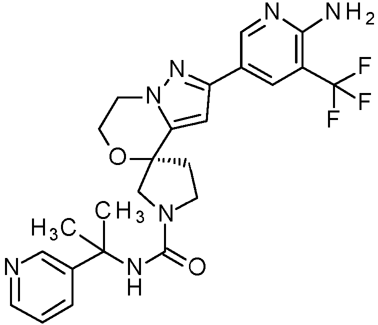

A solution of 2-(pyridin-3-yl)propan-2-amine (51.0 mg, 375 µmol), CDI (66.3 mg, 409 µmol) and DIPEA (240 µl, 1.4 mmol) in DMF (2mL) was stirred at RT for 1h. 5-[(3'R)-6,7-dihydrospiro[pyrazolo[5,1-c][1,4]oxazine-4,3'-pyrrolidin]-2-yl]-3-(trifluoromethyl)pyridin-2-amine hydrogen chloride (160 mg, 80 % purity, 341 µmol) in DMF (1.5mL) was added and the mixture was stirred at RT overnight. The crude reaction mixture was sent to the HPLC-Lab for purification to give 18.0 mg (95 % purity, 10 % yield) of the title compound.
1H NMR (DMSO-d6, 400 MHz) δ 8.5-8.6 (m, 2H), 8.36 (dd, 1H), 8.02 (d, 1H), 7.7-7.7 (m, 1H), 7.29 (ddd, 1H), 6.77 (s, 1H), 6.6-6.6 (m, 2H), 6.21 (s, 1H), 4.0-4.2 (m, 5H), 3.80 (br d, 1H), 3.61 (br t, 1H), 3.50 (br d, 1H), 3.40 (br d, 1H), 2.3-2.4 (m, 1H), 2.2-2.3 (m, 1H), 1.58 (s, 6H)
LC-MS (method 1): Rt = 0.94 min; MS (ESlpos): m/z = 503 [M+H]⁺

### Example 396

### (3R)-2'-[6-amino-5-(trifluoromethyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide

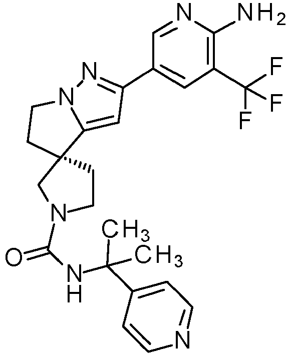

A solution of 2-(pyridin-4-yl)propan-2-amine (51.0 mg, 374 µmol), CDI (66.2 mg, 408 µmol) and DIPEA (240 µl, 1.4 mmol) in DMF (2mL) was stirred at RT for 1h. 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (157 mg, 78 % purity, 340 µmol) in DMF (1.5mL) was added and the mixture was stirred at RT overnight. The crude reaction mixture was sent to the HPLC-Lab for purification to give 37.0 mg (90 % purity, 20 % yield) of the title compound.
1H NMR (DMSO-d6, 400 MHz) δ 8.60 (d, 1H), 8.4-8.5 (m, 2H), 8.02 (d, 1H), 7.4-7.5 (m, 2H), 6.56 (s, 2H), 6.48 (s, 1H), 6.29 (s, 1H), 4.20 (t, 2H), 3.5-3.6 (m, 1H), 3.4-3.5 (m, 3H), 2.5-2.6 (m, 2H), 2.0-2.2 (m, 2H), 1.55 (s, 6H)
LC-MS (method 1): Rt = 0.94 min; MS (ESlpos): m/z = 487 [M+H]⁺

### Example 397

### (3'R)-2-[6-amino-5-(trifluoromethyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide

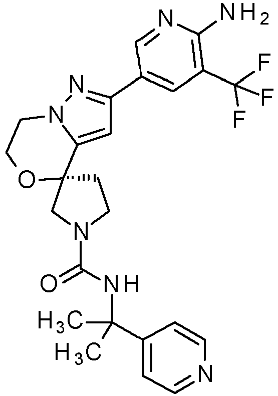

A solution of 2-(pyridin-4-yl)propan-2-amine (51.0 mg, 375 µmol), CDI (66.3 mg, 409 µmol ) and DIPEA (240 µl, 1.4 mmol) in DMF (2mL) was stirred at RT for 1h. 5-[(3'R)-6,7-dihydrospiro[pyrazolo[5,1-c][1,4]oxazine-4,3'-pyrrolidin]-2-yl]-3-(trifluoromethyl)pyridin-2-amine hydrogen chloride (160 mg, 80 % purity, 341 µmol) in DMF (1.5mL) was added and the mixture was stirred at RT overnight. The crude reaction mixture was sent to the HPLC-Lab for purification to give 71.0 mg (99 % purity, 41 % yield) of the title compound.
1H NMR (DMSO-d6, 400 MHz) δ 8.59 (d, 1H), 8.4-8.5 (m, 2H), 8.02 (d, 1H), 7.3-7.4 (m, 2H), 6.77 (s, 1H), 6.60 (s, 2H), 6.25 (s, 1H), 4.1-4.2 (m, 4H), 3.80 (br d, 1H), 3.6-3.7 (m, 1H), 3.50 (br d, 1H), 3.4-3.5 (m, 1H), 2.3-2.4 (m, 1H), 2.2-2.3 (m, 1H), 1.54 (s, 6H)
LC-MS (method 1): Rt = 0.94 min; MS (ESlpos): m/z = 503 [M+H]⁺

### Example 398

### (3R)-2'-[6-amino-5-(trifluoromethyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide

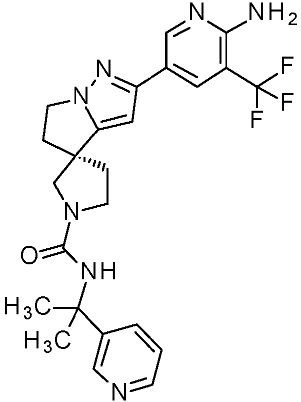

A solution of 2-(pyridin-3-yl)propan-2-amine (51.0 mg, 374 µmol), CDI (66.2 mg, 408 µmol) and DIPEA (240 µl, 1.4 mmol) in DMF (2mL) was stirred at RT for 1h. 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (157 mg, 78 % purity, 340 µmol) in DMF (1.5mL) was added and the mixture was stirred at RT overnight. The crude reaction mixture was sent to the HPLC-Lab for purification to give 8.00 mg (99 % purity, 5 % yield) of the title compound.
1H NMR (DMSO-d6, 400 MHz) δ 8.6-8.6 (m, 2H), 8.35 (dd, 1H), 8.02 (d, 1H), 7.7-7.8 (m, 1H), 7.27 (ddd, 1H), 6.55 (s, 2H), 6.45 (s, 1H), 6.19 (s, 1H), 4.19 (t, 2H), 3.57 (td, 1H), 3.4-3.5 (m, 3H), 2.5-2.6 (m, 2H), 2.0-2.1 (m, 2H), 1.58 (d, 6H)
LC-MS (method 1): Rt = 0.95 min; MS (ESlpos): m/z = 487 [M+H]⁺

### Example 399

### (3R)-2'-[6-amino-5-(difluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide

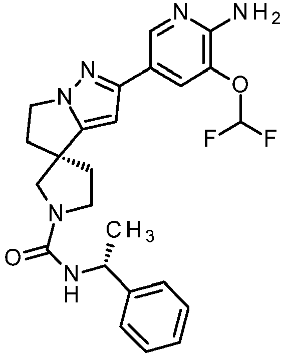

A solution of (1R)-1-phenylethan-1-amine (58.3 mg, 481 µmol), CDI (85.1 mg, 525 µmol) and DIPEA (300 µl, 1.7 mmol) in DMF (2mL) was stirred at RT for 1h. 3-(difluoromethoxy)-5-[(3R)-5',6'-dihydrospiro[pyrrolidine-3,4'-pyrrolo[1,2-b]pyrazol]-2'-yl]pyridin-2-amine hydrogen chloride (170 mg, 92 % purity, 437 µmol) in DMF (2mL) was added and the mixture was stirred at RT overnight. The mixture was purified by basic HPLC to give 6.00 mg (95 % purity, 3 % yield) of the title compound.
1H NMR (DMSO-d6, 400 MHz) δ 8.20 (d, 1H), 7.6-7.6 (m, 1H), 7.37 (s, 0,25H), 7.3-7.3 (m, 2H), 7.2-7.3 (m, 2H), 7.1-7.2 (m, 1H), 7.18 (s, 0,5H), 7.00 (s, 0,25H), 6.47 (d, 1H), 6.36 (s, 1H), 6.17 (s, 2H), 4.84 (t, 1H), 4.17 (t, 2H), 3.5-3.6 (m, 1H), 3.4-3.5 (m, 3H), 2.5-2.6 (m, 2H), 2.0-2.1 (m, 2H), 1.37 (d, 3H)
LC-MS (method 1): Rt = 1.01 min; MS (ESlpos): m/z = 469 [M+H]⁺

### Example 400

### (3'R)-2-[6-amino-5-(difluoromethoxy)pyridin-3-yl]-N-[(1R)-1-phenylethyl]-6,7-dihydrospiro[pyrazolo[5,1-c][1,4]oxazine-4,3'-pyrrolidine]-1'-carboxamide

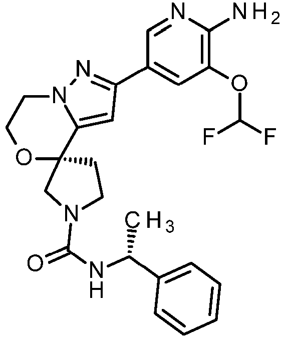

A solution of (1R)-1-phenylethan-1-amine (55.3 mg, 456 µmol), CDI (80.7 mg, 498 µmol) and DIPEA (290 µl, 1.7 mmol) in DMF (2mL) was stirred at RT for 1h. 3-(difluoromethoxy)-5-[(3'R)-6,7-dihydrospiro[pyrazolo[5,1-c][1,4]oxazine-4,3'-pyrrolidin]-2-yl]pyridin-2-amine hydrogen chloride (155 mg, 415 µmol) in DMF (2.5 mL) was added and the mixture was stirred at RT overnight. The crude was directly purified by basic HPLC to give 21.0 mg (99 % purity, 10 % yield) of the title compound.
1H NMR (DMSO-d6, 400 MHz) δ 8.20 (d, 1H), 7.6-7.6 (m, 1H), 7.36 (s, 0,25H), 7.3-7.4 (m, 2H), 7.3-7.3 (m, 2H), 7.2-7.2 (m, 1H), 7,18 (s, 0,5H), 7.00 (s, 0,25H), 6.67 (s, 1H), 6.5-6.5 (m, 1H), 6.22 (s, 2H), 4.84 (quin, 1H), 4.0-4.2 (m, 4H), 3.8-3.9 (m, 1H), 3.6-3.7 (m, 1H), 3.49 (d, 1H), 3.4-3.4 (m, 1H), 2.2-2.3 (m, 1H), 1.37 (d, 3H)
LC-MS (method 1): Rt = 1.01 min; MS (ESlpos): m/z = 486 [M+H]⁺

### Example 401

### (3R)-2'-[6-amino-5-(trifluoromethyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide

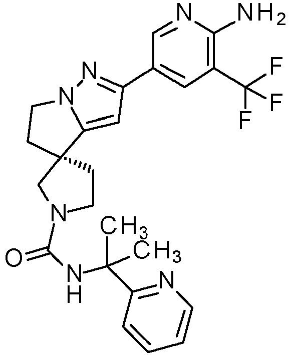

A solution of 2-(pyridin-2-yl)propan-2-amine (42.8 mg, 314 µmol), CDI (55.5 mg, 343 µmol) and DIPEA (200 µl, 1.1 mmol) in DMF (2mL) was stirred at RT for 1h. 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (130 mg, 79 % purity, 285 µmol) in DMF (1 mL) was added and the mixture was stirred at RT overnight. The crude was directly purified by basic HPLC to give 41.0 mg (99 % purity, 29 % yield) of the title compound.
1H NMR (DMSO-d6, 400 MHz) δ 8.60 (d, 1H), 8.4-8.5 (m, 1H), 8.02 (d, 1H), 7.70 (dt, 1H), 7.4-7.5 (m, 1H), 7.18 (ddd, 1H), 6.55 (s, 2H), 6.49 (s, 1H), 6.43 (s, 1H), 4.20 (t, 2H), 3.6-3.6 (m, 1H), 3.4-3.5 (m, 3H), 2.5-2.6 (m, 2H), 2.0-2.2 (m, 2H), 1.59 (s, 6H)
LC-MS (method 1): Rt = 1.02 min; MS (ESlpos): m/z = 487 [M+H]⁺

### Example 402

### 2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide (stereoisomer 1)

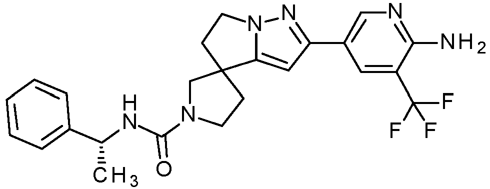

The mixture of isomers was separated by chrial HPLC to give 12.0 mg (98 % purity, 12 % yield) of the title compound.

Instrument: PrepCon Labomatic HPLC-3; Column: YMC Cellulose SB 10µ, 250x50; eluent A: methyl tert-butyl ether + 0.1 vol % diethylamine; eluent B: acetonitrile + 0.1 vol % diethylamine; isocratic: 60%A+40%B; flow: 80 mL/min; temperature: 25°C; UV: 254 nm
Retention time: 7.2 - 8.1 min
Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 60%A+40%B; flow: 1.4 mL/min; temperature: 25°C; UV: 254 nm
¹H NMR (400 MHz, DMSO-*d*₆) δ ppm 1.36 (d, 3 H) 2.02 - 2.14 (m, 2 H) 2.52 - 2.59 (m, 2 H) 3.40 - 3.50 (m, 3 H) 3.57 (dt, 1 H) 4.19 (t, 2 H) 4.84 (quin, 1 H) 6.43 - 6.49 (m, 2 H) 6.55 (s, 2 H) 7.15 - 7.22 (m, 1 H) 7.26 - 7.32 (m, 2 H) 7.33 - 7.39 (m, 2 H) 8.01 (d, 1 H) 8.58 (d, 1 H)

### Example 403

### 2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide (stereoisomer 2)

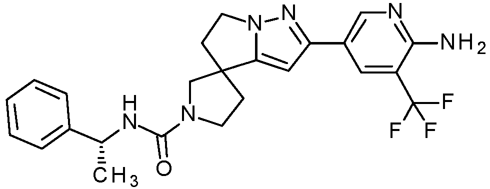

The mixture of isomers was separated by chrial HPLC to give 9.00 mg (95 % purity, 9 % yield) of the title compound.

Instrument: PrepCon Labomatic HPLC-3; Column: YMC Cellulose SB 10µ, 250x50; eluent A: methyl tert-butyl ether + 0.1 vol % diethylamine; eluent B: acetonitrile + 0.1 vol % diethylamine; isocratic: 60%A+40%B; flow: 80 mL/min; temperature: 25°C; UV: 254 nm
Retention time: 8.3 - 9.3 min
Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 60%A+40%B; flow: 1.4 mL/min; temperature: 25°C; UV: 254 nm
¹H-NMR (400MHz, DMSO-d₆): δ [ppm]= 1.37 (d, 3H), 2.01 - 2.11 (m, 2H), 2.52 - 2.57 (m, 2H), 3.39 - 3.50 (m, 3H), 3.50 - 3.60 (m, 1H), 4.18 (t, 2H), 4.84 (quin, 1H), 6.43 - 6.50 (m, 2H), 6.55 (s, 2H), 7.14 - 7.21 (m, 1H), 7.24 - 7.30 (m, 2H), 7.30 - 7.35 (m, 2H), 8.01 (d, 1H), 8.59 (d, 1H).

### Example 404

### (rac)-2'-[6-amino-5-(trifluoromethyl)pyridin-3-yl]-N-[2-(3,5-difluorophenyl)propan-2-yl]-5',6'-dihydrospiro[pyrrolidine-3,4'-pyrrolo[1,2-b]pyrazole]-1-carboxamide

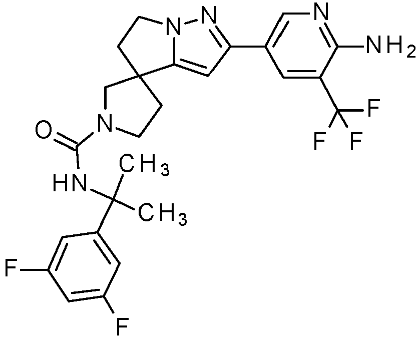

(*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 254 µmol) and N,N-diisopropylethylamine (130 µL) were stirred in the reaction mixture of 1,3-difluoro-5-(2-isocyanatopropan-2-yl)benzene (60.0 mg, 304 µmol) in 1,4-dioxane (2.6 mL) over the weekend at rt. NaOH (0.1 M) was added and the mixture was stirred for 5 min. The layers were separated and the aq. layer was extracted 4x with DCM. The combined organic layers were washed with NaOH (0.1 M), the aq. layer was extracted with DCM and the organic layer was evaporated. The residue was purified by flash chromatography to give 25.0 mg (90 % purity, 17 % yield) of the title compound.
LC-MS (method 1): Rt = 1.19 min; MS (ESlpos): m/z = 521 [M+H]⁺
¹H NMR (400 MHz, DMSO-d₆) δ ppm 1.54 (d, 6 H) 1.99 - 2.13 (m, 2 H) 2.54 (t, 2 H) 3.41 - 3.52 (m, 3 H) 3.53 - 3.64 (m, 1 H) 4.20 (t, 2 H) 6.17 (s, 1 H) 6.44 (s, 1 H) 6.55 (s, 2 H) 6.94 - 7.05 (m, 3 H) 8.00 (d, 1 H) 8.58 (d, 1 H)

### Example 405

### (rac)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-(5-fluoropyridin-2-yl)-2-methylpropan-1-one

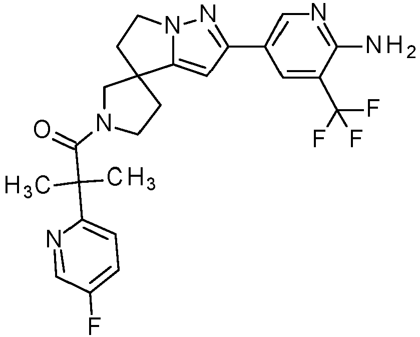

Example 405 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (110 mg, 277 µmol) and 5-fluoro-2-(2-isocyanatopropan-2-yl)pyridine (60.0 mg, 333 µmol).
LC-MS (Method 1): Rt = 1.07 min; MS (ESlpos): m/z = 489 [M+H]⁺
¹H NMR (400 MHz, DMSO-*d*₆) δ ppm 1.86 (br s, 1 H) 1.92 (br s, 2 H) 2.07 - 2.22 (m, 1 H) 2.29 - 2.42 (m, 2 H) 2.62 - 2.71 (m, 1 H) 2.73 - 2.84 (m, 2 H) 3.48 - 3.64 (m, 3 H) 3.85 - 3.95 (m, 1 H) 4.02 - 4.20 (m, 2 H) 6.32 (s, 2 H) 6.54 (br s, 3 H) 7.43 (dd, 1 H) 7.61 (td, 1 H) 7.70 - 7.82 (m, 1 H) 8.47 (d, 1 H)

### Example 406

### (rac)-2'-[6-amino-5-(trifluoromethyl)pyridin-3-yl]-N-[2-(6-methylpyridin-3-yl)propan-2-yl]-5',6'-dihydrospiro[pyrrolidine-3,4'-pyrrolo[1,2-b]pyrazole]-1-carboxamide

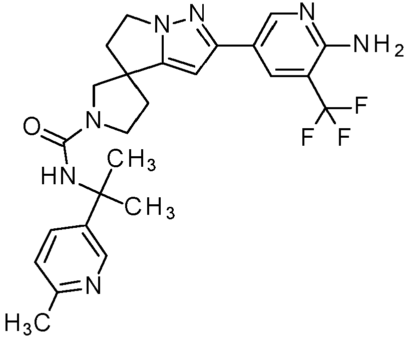

Example 406 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (112 mg, 284 µmol) and 5-(2-isocyanatopropan-2-yl)-2-methylpyridine (60.0 mg, 340 µmol).
LC-MS (Method 1): Rt = 1.00 min; MS (ESlpos): m/z = 500 [M+H]⁺
¹H NMR (400 MHz, DMSO-d₆) δ ppm 1.56 (d, 6 H) 2.00 - 2.13 (m, 2 H) 2.40 (s, 3 H) 2.53 (br s, 2 H) 3.41 - 3.50 (m, 3 H) 3.55 (br s, 1 H) 4.19 (t, 2 H) 6.13 (s, 1 H) 6.45 (s, 1 H) 6.55 (s, 2 H) 7.10 (d, 1 H) 7.59 (dd, 1 H) 8.02 (d, 1 H) 8.43 (d, 1 H) 8.60 (d, 1 H)

### Example 407

### (rac)-2'-[6-amino-5-(trifluoromethyl)pyridin-3-yl]-N-(1-phenylcyclohexyl)-5',6'-dihydrospiro[pyrrolidine-3,4'-pyrrolo[1,2-b]pyrazole]-1-carboxamide

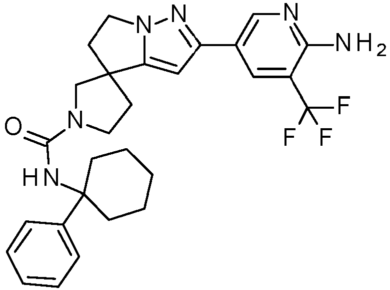

Example 407 was prepared in analogy to Example 404 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (98.4 mg, 248 µmol) and (1-isocyanatocyclohexyl)benzene (60.0 mg, 298 µmol).
LC-MS (Method 1): Rt = 1.27 min; MS (ESlpos): m/z = 525 [M+H]⁺
¹H NMR (400 MHz, DMSO-*d*₆) δ ppm 1.23 (br s, 1 H) 1.47 - 1.70 (m, 7 H) 2.02 - 2.14 (m, 2 H) 2.37 (br s, 2 H) 2.52 - 2.60 (m, 2 H) 3.43 - 3.55 (m, 3 H) 3.56 - 3.65 (m, 1 H) 4.20 (t, 2 H) 5.78 (s, 1 H) 6.42 (s, 1 H) 6.56 (s, 2 H) 7.10 - 7.16 (m, 1 H) 7.25 (t, 2 H) 7.38 (d, 2 H) 7.99 (d, 1 H) 8.58 (d, 1 H)

### Example 408

### (rac)-2'-[6-amino-5-(trifluoromethyl)pyridin-3-yl]-N-[2-(5-fluoropyridin-2-yl)propan-2-yl]-5',6'-dihydrospiro[pyrrolidine-3,4'-pyrrolo[1,2-b]pyrazole]-1-carboxamide

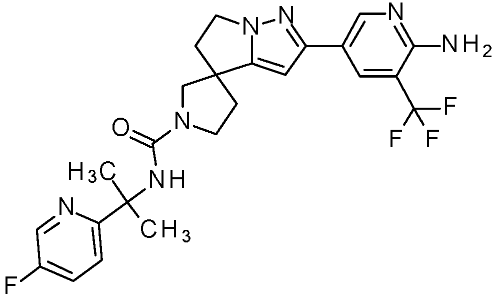

Example 408 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (110 mg, 277 µmol) and 5-fluoro-2-(2-isocyanatopropan-2-yl)pyridine (60.0 mg, 333 µmol).
LC-MS (Method 1): Rt = 1.06 min; MS (ESlpos): m/z = 504 [M+H]⁺
¹H NMR (400 MHz, DMSO-*d*₆) δ ppm 1.57 (s, 6 H) 2.01 - 2.14 (m, 2 H) 2.53 - 2.58 (m, 2 H) 3.41 - 3.49 (m, 3 H) 3.50 - 3.63 (m, 1 H) 4.20 (t, 2 H) 6.30 (s, 1 H) 6.49 (s, 1 H) 6.55 (s, 2 H) 7.49 (dd, 1 H) 7.60 (td, 1 H) 8.02 (d, 1 H) 8.42 (d, 1 H) 8.60 (d, 1 H)

### Example 409

### 2'-[6-amino-5-(trifluoromethyl)pyridin-3-yl]-N-[(1R)-2,2,2-trifluoro-1-methoxy-1-phenylethyl]-5',6'-dihydrospiro[pyrrolidine-3,4'-pyrrolo[1,2-b]pyrazole]-1-carboxamide (mixture of stereoisomers)

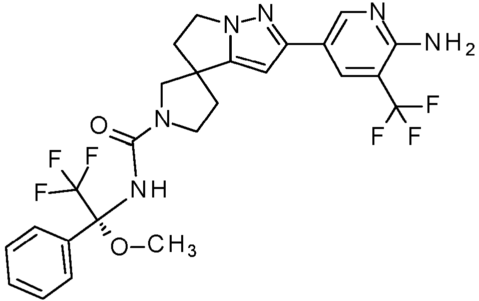

Example 409 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (85.7 mg, 216 µmol) and [(1S)-2,2,2-trifluoro-1-isocyanato-1-methoxyethyl]benzene (60.0 mg, 260 µmol).
LC-MS (Method 1): Rt = 1.18 min; MS (ESlpos): m/z = 555 [M+H]⁺
¹H NMR (400 MHz, DMSO-*d*₆) δ ppm 1.21 - 1.30 (m, 1 H) 2.09 (br s, 2 H) 2.53 - 2.61 (m, 2 H) 3.41 - 3.47 (m, 3 H) 3.49 (br s, 1 H) 3.57 - 3.76 (m, 2 H) 4.22 (br t, 2 H) 6.34 - 6.64 (m, 3 H) 7.08 (s, 1 H) 7.29 - 7.40 (m, 3 H) 7.41 - 7.46 (m, 1 H) 7.50 (br d, 1 H) 8.01 (dd, 1 H) 8.59 (dd, 1 H)

### Example 410

### (rac)-2'-[6-amino-5-(trifluoromethyl)pyridin-3-yl]-N-[2-(pyrimidin-4-yl)propan-2-yl]-5',6'-dihydrospiro[pyrrolidine-3,4'-pyrrolo[1,2-b]pyrazole]-1-carboxamide

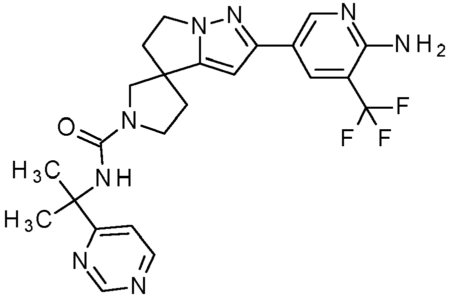

Example 410 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (110 mg, 306 µmol) and 4-(2-isocyanatopropan-2-yl)pyrimidine (60.0 mg, 368 µmol).
LC-MS (Method 1): Rt = 0.91 min; MS (ESlpos): m/z = 487 [M+H]⁺
¹H NMR (400 MHz, DMSO-*d*₆) δ ppm 1.54 (s, 6 H) 2.00 - 2.15 (m, 2 H) 2.52 - 2.58 (m, 2 H) 3.36 - 3.50 (m, 3 H) 3.51 - 3.65 (m, 1 H) 4.20 (t, 2 H) 6.36 (s, 1 H) 6.49 (s, 1 H) 6.56 (s, 2 H) 7.50 (dd, 1 H) 8.02 (d, 1 H) 8.60 (d, 1 H) 8.64 (d, 1 H) 9.05 (d, 1 H)

### Example 411

### (rac)-2'-[6-amino-5-(trifluoromethyl)pyridin-3-yl]-N-[2-(pyrimidin-5-yl)propan-2-yl]-5',6'-dihydrospiro[pyrrolidine-3,4'-pyrrolo[1,2-b]pyrazole]-1-carboxamide

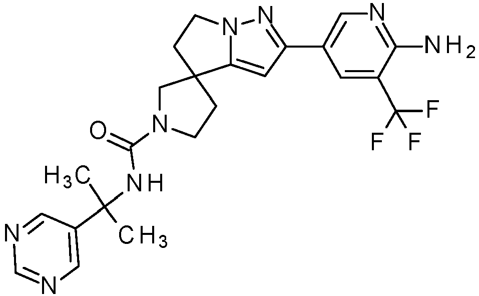

Example 411 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (121 mg, 306 µmol) and 5-(2-isocyanatopropan-2-yl)pyrimidine (60.0 mg, 368 µmol).
LC-MS (Method 1): Rt = 0.89 min; MS (ESlpos): m/z = 487 [M+H]⁺
¹H NMR (400 MHz, DMSO-*d*₆) δ ppm 1.57 - 1.63 (m, 6 H) 2.01 - 2.13 (m, 2 H) 2.52 - 2.57 (m, 2 H) 3.39 - 3.51 (m, 3 H) 3.56 (br dd, 1 H) 4.19 (t, 2 H) 6.28 (s, 1 H) 6.45 (s, 1 H) 6.55 (s, 2 H) 8.02 (d, 1 H) 8.60 (d, 1 H) 8.76 (s, 2 H) 8.98 (s, 1 H)

### Example 412

### (rac)-{2'-[6-amino-5-(trifluoromethyl)pyridin-3-yl]-5',6'-dihydrospiro[pyrrolidine-3,4'-pyrrolo[1,2-b]pyrazol]-1-yl}(3-phenyloxetan-3-yl)methanone

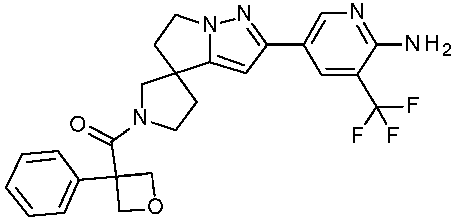

Example 412 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (113 mg, 285 µmol) and 3-isocyanato-3-phenyloxetane (60.0 mg, 342 µmol).
LC-MS (Method 1): Rt = 1.03 min; MS (ESlpos): m/z = 484 [M+H]⁺
¹H NMR (400 MHz, DMSO-*d*₆) δ ppm 1.91 - 2.06 (m, 2 H) 2.36 - 2.47 (m, 1 H) 2.94 - 3.07 (m, 2 H) 3.58 - 3.68 (m, 2 H) 3.92 - 4.00 (m, 1 H) 4.04 - 4.21 (m, 1 H) 4.63 - 4.72 (m, 3 H) 5.17 (dd, 1 H) 6.50 - 6.60 (m, 2 H) 7.23 - 7.30 (m, 1 H) 7.35 - 7.41 (m, 2 H) 7.46 - 7.53 (m, 3 H) 7.55 - 7.60 (m, 1 H) 7.93 - 7.99 (m, 1 H) 8.52 - 8.57 (m, 1 H)

### Example 413

### (rac)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-(3,5-difluorophenyl)-2-methylpropan-1-one

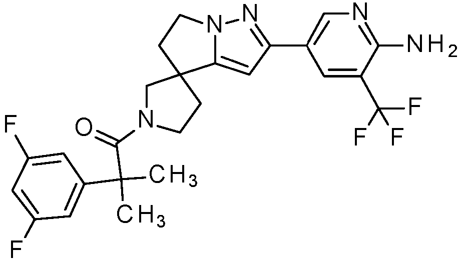

Example 413 was prepared in analogy to Example 404 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (100 mg, 254 µmol) and 1,3-difluoro-5-(2-isocyanatopropan-2-yl)benzene (60.0 mg, 304 µmol).
LC-MS (Method 1): Rt = 1.22 min; MS (ESlpos): m/z = 506 [M+H]⁺
¹H NMR (400 MHz, DMSO-*d*₆) δ ppm 1.40 (s, 2 H) 1.43 - 1.54 (m, 4 H) 1.93 (br t, 2 H) 2.15 - 2.27 (m, 1 H) 2.36 - 2.44 (m, 1 H) 2.84 - 2.99 (m, 1 H) 3.06 (br d, 1 H) 3.52 - 3.67 (m, 2 H) 3.95 (dt, 1 H) 4.02 - 4.23 (m, 2 H) 6.49 - 6.62 (m, 2 H) 6.91 - 7.09 (m, 3 H) 7.96 - 8.04 (m, 1 H) 8.57 (br s, 1 H)

### Example 414

### (rac)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-methyl-2-(6-methylpyridin-3-yl)propan-1-one

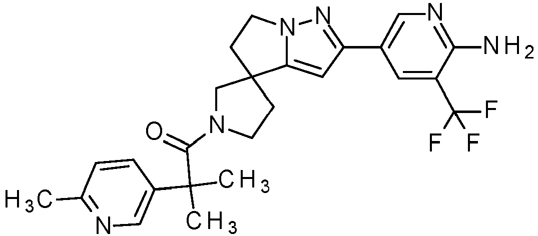

Example 414 was prepared in analogy to Example 404 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (112 mg, 284 µmol) and 5-(2-isocyanatopropan-2-yl)-2-methylpyridine (60.0 mg, 340 µmol).
LC-MS (Method 1): Rt = 1.01 min; MS (ESlpos): m/z = 485 [M+H]⁺
¹H NMR (400 MHz, DMSO-*d*₆) δ ppm 1.40 - 1.54 (m, 6 H) 1.81 - 2.02 (m, 2 H) 2.05 - 2.15 (m, 1 H) 2.29 - 2.44 (m, 3 H) 2.52 - 2.55 (m, 1 H) 2.83 - 3.02 (m, 2 H) 3.51 - 3.65 (m, 2 H) 3.81 - 3.90 (m, 1 H) 4.01 - 4.20 (m, 1 H) 6.26 (s, 1 H) 6.49 - 6.61 (m, 2 H) 7.10 - 7.30 (m, 1 H) 7.50 - 7.65 (m, 1 H) 7.99 (br s, 1 H) 8.30 - 8.38 (m, 1 H) 8.54 - 8.60 (m, 1 H)

### Example 415

### (rac)-2'-[6-amino-5-(trifluoromethyl)pyridin-3-yl]-N-(3-phenyloxetan-3-yl)-5',6'-dihydrospiro[pyrrolidine-3,4'-pyrrolo[1,2-b]pyrazole]-1-carboxamide

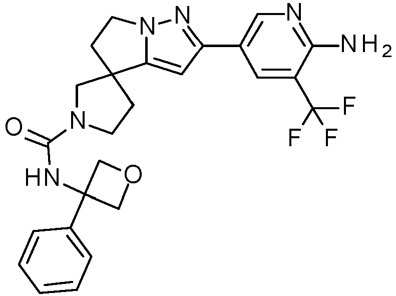

Example 415 was prepared in analogy to Example 404 using (rac)-5-[5',6'-dihydrospiro[pyrrolidine-3,4'-pyrrolo[1,2-b]pyrazol]-2'-yl]-3-(trifluoromethyl)pyridin-2-amine hydrochloride (113 mg, 285 µmol) and 3-isocyanato-3-phenyloxetane (60.0 mg, 342 µmol).
LC-MS (Method 1): Rt = 0.97 min; MS (ESlpos): m/z = 499 [M+H]⁺
¹H NMR (400 MHz, DMSO-*d*₆) δ ppm 2.11 (br d, 2 H) 2.57 (t, 2 H) 3.48 (s, 3 H) 3.52 - 3.67 (m, 1 H) 4.20 (t, 2 H) 4.65 (dd, 2 H) 4.90 (dd, 2 H) 6.48 (s, 1 H) 6.56 (s, 2 H) 7.22 - 7.32 (m, 2 H) 7.36 (t, 2 H) 7.53 (d, 2 H) 8.02 (d, 1 H) 8.60 (d, 1 H)

### Example 416

### (rac)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide

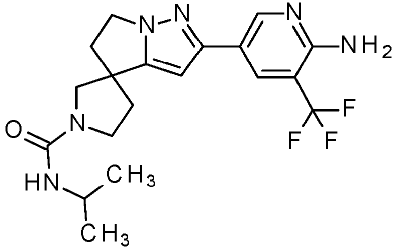

(*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (3.30 g, 9.17 mmol) was solubilised in dichloromethane (75 mL), N,N-diisopropylethylamine (4.8 mL, 28 mmol) and 2-isocyanatopropane (390 mg, 4.59 mmol) were added an dthe mixture was stirred for 1 h at rt under argon. The mixture was evaporated and the residue was purified by flash chromatography. The resulting crude was diluted with brine and water and extracted 3x with DCM. The organic layer was dried over a silicone filter and concentrated under reduced pressure to give 2.10 g (99 % purity, 55 % yield) of the title compound.
LC-MS (Method 1): Rt = 0.96 min; MS (ESlpos): m/z = 409 [M+H]⁺
¹H NMR (400 MHz, DMSO-d₆) δ ppm 1.06 (dd, 6 H) 1.99 - 2.10 (m, 2 H) 2.52 - 2.55 (m, 2 H) 3.36 - 3.43 (m, 3 H) 3.45 - 3.55 (m, 1 H) 3.71 - 3.80 (m, 1 H) 4.18 (t, 2 H) 5.82 (d, 1 H) 6.47 (s, 1 H) 6.54 (s, 2 H) 8.01 (d, 1 H) 8.59 (d, 1 H)

### Example 417

### (rac)-2'-[6-amino-5-(trifluoromethyl)pyridin-3-yl]-N-cyclobutyl-5',6'-dihydrospiro[pyrrolidine-3,4'-pyrrolo[1,2-b]pyrazole]-1-carboxamide

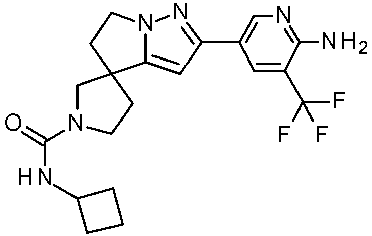

Example 417 was prepared in analogy to
Example 416 using (*rac*)-5-[5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and isocyanatocyclobutane (20.2 mg, 208 µmol).
LC-MS (Method 1): Rt = 0.98 min; MS (ESlpos): m/z = 421 [M+H]⁺
¹H NMR (400 MHz, DMSO-*d*₆) δ ppm 1.45 - 1.62 (m, 2 H) 1.85 - 1.99 (m, 2 H) 2.00 - 2.07 (m, 2 H) 3.07 (br d, 2 H) 3.40 - 3.61 (m, 7 H) 4.17 (t, 2 H) 6.34 (d, 1 H) 6.45 - 6.50 (m, 1 H) 6.53 (s, 2 H) 8.02 (d, 1 H) 8.59 (d, 1 H)

### Example 418

### (3R)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide

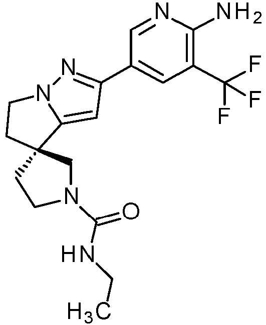

Example 418 was prepared in analogy to
Example 416 using 5-[(3R)-5',6'-dihydrospiro[pyrrolidine-3,4'-pyrrolo[1,2-b]pyrazol]-2'-yl]-3-(trifluoromethyl)pyridin-2-amine hydrochloride (54.0 mg, 150 µmol, stereoisomer 2) and isocyanatoethane (12 µL, 150 µmol)
¹H-NMR (400MHz, DMSO-d₆): δ [ppm]= 1.02 (t, 3H), 2.00 - 2.10 (m, 2H), 2.52 - 2.55 (m, 2H), 3.01 - 3.10 (m, 2H), 3.36 - 3.43 (m, 3H), 3.46 - 3.53 (m, 1H), 4.18 (t, 2H), 6.15 (t, 1H), 6.48 (s, 1H), 6.54 (s, 2H), 8.02 (d, 1H), 8.59 (d, 1H).
[α]²⁰_{D} : +51.8° (c = 1.00, DMSO)
LC-MS (method 1): Rt = 0.89 min; MS (ESlpos): m/z = 395 [M+H]⁺

### Example 419

### (3S)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide

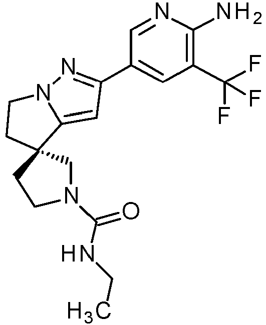

Example 419 was prepared in analogy to
Example 416 using 5-[(3S)-5',6'-dihydrospiro[pyrrolidine-3,4'-pyrrolo[1,2-b]pyrazol]-2'-yl]-3-(trifluoromethyl)pyridin-2-amine hydrochloride (49.0 mg, 136 µmol) and isocyanatoethane (11 µL, 136 µmol).
¹H-NMR (400MHz, DMSO-d₆): δ [ppm]= 1.02 (t, 3H), 2.01 - 2.10 (m, 2H), 2.52 - 2.55 (m, 2H), 3.01 - 3.09 (m, 2H), 3.36 - 3.43 (m, 3H), 3.46 - 3.55 (m, 1H), 4.18 (t, 2H), 6.15 (t, 1H), 6.48 (s, 1H), 6.54 (s, 2H), 8.02 (d, 1H), 8.59 (d, 1H).
[α]²⁰_{D} : -46.6° (c = 1.00, DMSO)
LC-MS (method 1): Rt = 0.89 min; MS (ESlpos): m/z = 396 [M+H]⁺

### Example 420

### (3R)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide

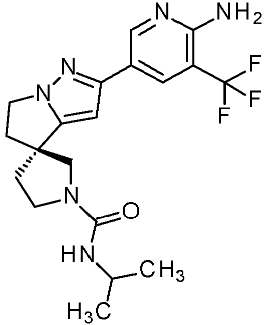

Example 420 was prepared in analogy to
Example 416 using 5-[(3R)-5',6'-dihydrospiro[pyrrolidine-3,4'-pyrrolo[1,2-b]pyrazol]-2'-yl]-3-(trifluoromethyl)pyridin-2-amine hydrochloride (68.0 mg, 189 µmol) and 2-isocyanatopropane (13 mg, 0.19 mmol).
LC-MS (method 1): Rt = 0.94 min; MS (ESlpos): m/z = 409 [M+H]⁺
¹H-NMR (400MHz, DMSO-d₆): δ [ppm]= 1.06 (dd, 6H), 1.99 - 2.10 (m, 2H), 2.52 - 2.55 (m, 2H), 3.37 - 3.44 (m, 3H), 3.45 - 3.56 (m, 1H), 3.71 - 3.80 (m, 1H), 4.18 (t, 2H), 5.82 (d, 1H), 6.47 (s, 1H), 6.54 (s, 2H), 8.02 (d, 1H), 8.59 (d, 1H).
[α]²⁰_{D} : +47.3° (c = 1.00, DMSO)

### Example 421

### (3S)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide

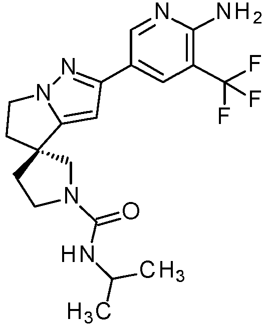

Example 421 was prepared in analogy to
Example 416 using 5-[(3S)-5',6'-dihydrospiro[pyrrolidine-3,4'-pyrrolo[1,2-b]pyrazol]-2'-yl]-3-(trifluoromethyl)pyridin-2-amine hydrochloride (77.0 mg, 214 µmol, stereoisomer 1) and 2-isocyanatopropane (15 mg, 0.21 mmol).
[α]²⁰_{D} : -46.4° (c = 1.00, DMSO)
¹H-NMR (400MHz, DMSO-d₆): δ [ppm]= 1.06 (dd, 6H), 2.00 - 2.10 (m, 2H), 2.52 - 2.56 (m, 2H), 3.36 - 3.43 (m, 3H), 3.51 (dt, 1H), 3.71 - 3.80 (m, 1H), 4.18 (t, 2H), 5.82 (d, 1H), 6.47 (s, 1H), 6.54 (s, 2H), 8.02 (d, 1H), 8.59 (d, 1H).
LC-MS (method 1): Rt = 0.94 min; MS (ESlpos): m/z = 409 [M+H]⁺

### Example 422

### (rac)-2-[6-amino-5-(trifluoromethyl)pyridin-3-yl]-N-cyclobutyl-6,7-dihydrospiro[pyrazolo[5,1-c][1,4]oxazine-4,3'-pyrrolidine]-1'-carboxamide

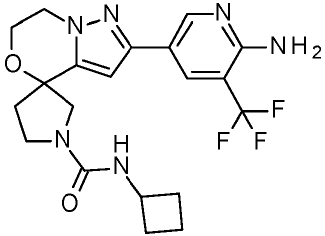

Example 422 was prepared in analogy to
Example 416 using (*rac*)-5-[6,7-dihydrospiro[pyrazolo[5,1-c][1,4]oxazine-4,3'-pyrrolidin]-2-yl]-3-(trifluoromethyl)pyridin-2-amine (75.0 mg, 221 µmol) and isocyanatocyclobutane (21.5 mg, 221 µmol).
LC-MS (Method 1): Rt = 0.95 min; MS (ESlpos): m/z = 437 [M+H]⁺
¹H NMR (400 MHz, DMSO-*d*₆) δ ppm 1.46 - 1.64 (m, 2 H) 1.85 - 2.02 (m, 2 H) 2.06 - 2.14 (m, 2 H) 2.16 - 2.28 (m, 1 H) 2.29 - 2.39 (m, 1 H) 3.35 - 3.49 (m, 2 H) 3.55 (t, 1 H) 3.73 - 3.81 (m, 1 H) 4.02 - 4.18 (m, 5 H) 6.32 (d, 1 H) 6.60 (s, 2 H) 6.74 (s, 1 H) 8.01 (d, 1 H) 8.58 (d, 1 H)

### Example 423

### (rac)-2-[6-amino-5-(trifluoromethyl)pyridin-3-yl]-N-ethyl-6,7-dihydrospiro[pyrazolo[5,1-c][1,4]oxazine-4,3'-pyrrolidine]-1'-carboxamide

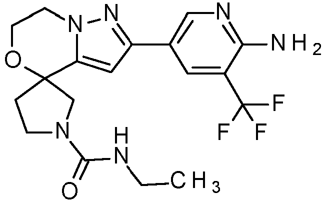

Example 423 was prepared in analogy to
Example 416 using (*rac*)-5-[6,7-dihydrospiro[pyrazolo[5,1-c][1,4]oxazine-4,3'-pyrrolidin]-2-yl]-3-(trifluoromethyl)pyridin-2-amine (75.0 mg, 221 µmol) and isocyanatoethane (17 µL, 220 µmol).
LC-MS (Method 1): Rt = 0.87 min; MS (ESlpos): m/z = 411 [M+H]⁺
¹H NMR (400 MHz, DMSO-*d*₆) δ ppm 1.02 (t, 3 H) 2.22 (br d, 1 H) 2.30 - 2.35 (m, 1 H) 3.05 (dd, 2 H) 3.10 - 3.17 (m, 1 H) 3.35 - 3.46 (m, 2 H) 3.76 (br d, 1 H) 4.03 - 4.17 (m, 4 H) 6.18 (t, 1 H) 6.60 (s, 2 H) 6.74 (s, 1 H) 8.01 (d, 1 H) 8.58 (d, 1 H)

### Example 424

### (rac)-2-[6-amino-5-(trifluoromethyl)pyridin-3-yl]-N-(propan-2-yl)-6,7-dihydrospiro[pyrazolo[5,1-c][1,4]oxazine-4,3'-pyrrolidine]-1'-carboxamide

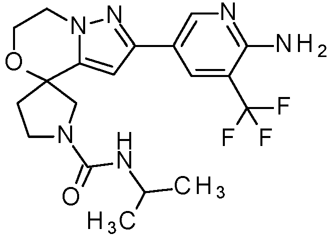

Example 424 was prepared in analogy to
Example 416 using (*rac*)-5-[6,7-dihydrospiro[pyrazolo[5,1-c][1,4]oxazine-4,3'-pyrrolidin]-2-yl]-3-(trifluoromethyl)pyridin-2-amine (75.0 mg, 221 µmol) and 2-isocyanatopropane (18.8 mg, 221 µmol).
LC-MS (Method 1): Rt = 0.93 min; MS (ESlpos): m/z = 425 [M+H]⁺
¹H NMR (400 MHz, DMSO-*d*₆) δ ppm 1.06 (d, 6 H) 1.26 (br s, 4 H) 2.17 - 2.26 (m, 1 H) 2.29 - 2.38 (m, 1 H) 3.43 (d, 1 H) 3.70 - 3.81 (m, 2 H) 4.11 - 4.17 (m, 2 H) 5.84 (d, 1 H) 6.60 (s, 2 H) 6.75 (s, 1 H) 8.01 (d, 1 H) 8.58 (d, 1 H)

### Example 425

### 2'-[6-amino-5-(trifluoromethyl)pyridin-3-yl]-N-cyclobutyl-5',6'-dihydrospiro[azetidine-3,4'-pyrrolo[1,2-b]pyrazole]-1-carboxamide

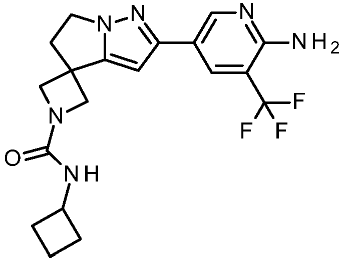

Example 425 was prepared in analogy to
Example 416 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and isocyanatocyclobutane (23.5 mg, 242 µmol).
LC-MS (Method 1): Rt = 0.93 min; MS (ESlpos): m/z = 407 [M+H]⁺
¹H NMR (400 MHz, DMSO-*d*₆) δ ppm 1.46 - 1.63 (m, 2 H) 1.83 - 1.98 (m, 2 H) 2.04 - 2.17 (m, 2 H) 2.83 (t, 2 H) 3.97 (s, 4 H) 4.04 - 4.16 (m, 3 H) 6.55 (s, 2 H) 6.67 (d, 1 H) 6.72 (s, 1 H) 8.04 (d, 1 H) 8.61 (d, 1 H)

### Example 426

### 2'-[6-amino-5-(trifluoromethyl)pyridin-3-yl]-N-ethyl-5',6'-dihydrospiro[azetidine-3,4'-pyrrolo[1,2-b]pyrazole]-1-carboxamide

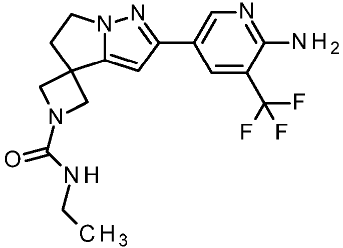

Example 426 was prepared in analogy to
Example 416 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and isocyanatoethane (19 µL, 240 µmol).
LC-MS (Method 1): Rt = 0.83 min; MS (ESlpos): m/z = 381 [M+H]⁺
¹H NMR (400 MHz, DMSO-*d*₆) δ ppm 1.01 (t, 3 H) 2.84 (t, 2 H) 2.98 - 3.08 (m, 2 H) 3.97 (s, 4 H) 4.12 (t, 2 H) 6.47 (t, 1 H) 6.55 (br s, 2 H) 6.72 (s, 1 H) 8.04 (d, 1 H) 8.62 (d, 1 H)

### Example 427

### 2'-[6-amino-5-(trifluoromethyl)pyridin-3-yl]-N-(propan-2-yl)-5',6'-dihydrospiro[azetidine-3,4'-pyrrolo[1,2-b]pyrazole]-1-carboxamide

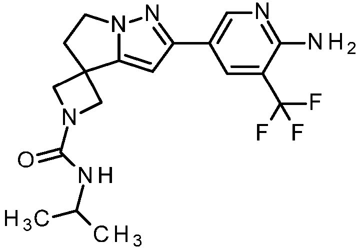

Example 427 was prepared in analogy to
Example 416 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 2-isocyanatopropane (20.6 mg, 242 µmol).
LC-MS (Method 1): Rt = 0.92 min; MS (ESlpos): m/z = 395 [M+H]⁺
¹H NMR (400 MHz, DMSO-*d*₆) δ ppm 1.06 (d, 6 H) 2.83 (t, 2 H) 3.67 - 3.78 (m, 1 H) 3.97 (s, 4 H) 4.12 (t, 2 H) 6.23 (d, 1 H) 6.49 - 6.60 (m, 2 H) 6.73 (s, 1 H) 8.00 - 8.08 (m, 1 H) 8.59 - 8.65 (m, 1 H)

### Example 428

### (rac)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide

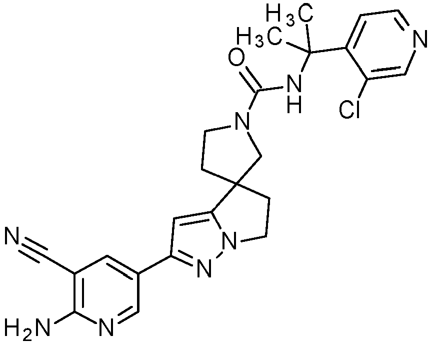

Example 428 was prepared in analogy to Example 404 using (*rac*)-2-amino-5-[5',6'-dihydrospiro[pyrrolidine-3,4'-pyrrolo[1,2-b]pyrazol]-2'-yl]pyridine-3-carbonitrile hydrochloride (107 mg, 339 µmol) and 3-chloro-4-(2-isocyanatopropan-2-yl)pyridine (80.0 mg, 407 µmol).
¹H NMR (400 MHz, DMSO-*d*₆) δ ppm 1.65 (s, 6 H) 1.97 - 2.14 (m, 2 H) 2.52 - 2.57 (m, 2 H) 3.35 - 3.54 (m, 4 H) 4.18 (t, 2 H) 6.33 (s, 1 H) 6.43 (s, 1 H) 7.00 (s, 2 H) 7.43 (d, 1 H) 8.13 (d, 1 H) 8.40 (d, 1 H) 8.42 (s, 1 H) 8.60 (d, 1 H)
LC-MS (Method 1): Rt = 0.90 min; MS (ESlpos): m/z = 477 [M+H]⁺

### Example 429

### (rac)-2'-{6-amino-5-[(2-chlorophenyl)methoxy]pyridin-3-yl}-N-ethyl-5',6'-dihydrospiro[pyrrolidine-3,4'-pyrrolo[1,2-b]pyrazole]-1-carboxamide

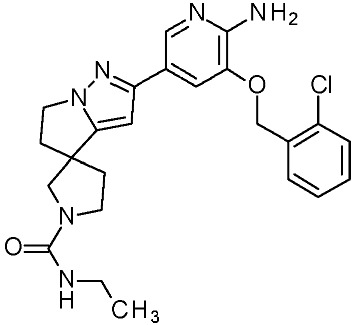

Example 429 was prepared in analogy to
Example 416 using (*rac*)-3-[(2-chlorophenyl)methoxy]-5-[5',6'-dihydrospiro[pyrrolidine-3,4'-pyrrolo[1,2-b]pyrazol]-2'-yl]pyridin-2-amine-hydrochloride salt (70.0 mg, 149 µmol) and isocyanatoethane (13 µL, 160 µmol).
LC-MS (Method 1): Rt = 1.02 min; MS (ESlpos): m/z = 467 [M+H]⁺
¹H NMR (400 MHz, DMSO-*d*₆) δ ppm 0.98 - 1.06 (m, 3 H) 1.98 - 2.12 (m, 2 H) 2.52 - 2.55 (m, 1 H) 3.01 - 3.09 (m, 2 H) 3.35 - 3.44 (m, 3 H) 3.45 - 3.54 (m, 1 H) 4.10 - 4.20 (m, 2 H) 5.22 (s, 2 H) 5.82 (s, 2 H) 6.15 (t, 1 H) 6.36 (s, 1 H) 7.37 - 7.42 (m, 2 H) 7.45 (d, 1 H) 7.49 - 7.56 (m, 1 H) 7.66 - 7.76 (m, 1 H) 7.91 - 7.98 (m, 1 H)

### Example 430

### (rac)-2'-{6-amino-5-[(2-chlorophenyl)methoxy]pyridin-3-yl}-N-(propan-2-yl)-5',6'-dihydrospiro[pyrrolidine-3,4'-pyrrolo[1,2-b]pyrazole]-1-carboxamide

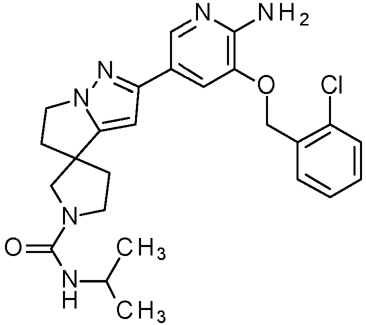

Example 430 was prepared in analogy to
Example 416 using (*rac*)-3-[(2-chlorophenyl)methoxy]-5-[5',6'-dihydrospiro[pyrrolidine-3,4'-pyrrolo[1,2-b]pyrazol]-2'-yl]pyridin-2-amine-hydrochloride salt (68.0 mg, 145 µmol) and 2-isocyanatopropane (13.0 mg, 152 µmol).
LC-MS (Method 1): Rt = 1.07 min; MS (ESlpos): m/z = 481 [M+H]⁺
¹H NMR (400 MHz, DMSO-*d*₆) δ ppm 1.02 - 1.11 (m, 6 H) 1.23 (s, 1 H) 1.97 - 2.11 (m, 2 H) 2.52 - 2.55 (m, 1 H) 3.36 - 3.43 (m, 3 H) 3.45 - 3.55 (m, 1 H) 3.70 - 3.80 (m, 1 H) 4.11 - 4.20 (m, 2 H) 5.22 (s, 2 H) 5.78 - 5.87 (m, 3 H) 6.36 (s, 1 H) 7.34 - 7.46 (m, 3 H) 7.49 - 7.55 (m, 1 H) 7.66 - 7.76 (m, 1 H) 7.91 - 7.98 (m, 1 H)

### Example 431

### (rac)-2'-{6-amino-5-[(2-chlorophenyl)methoxy]pyridin-3-yl}-N-cyclobutyl-5',6'-dihydrospiro[pyrrolidine-3,4'-pyrrolo[1,2-b]pyrazole]-1-carboxamide

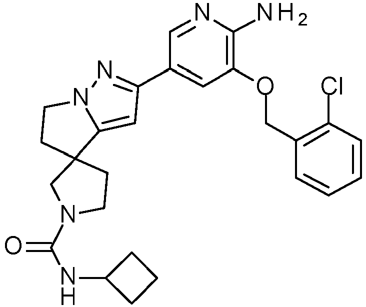

Example 431 was prepared in analogy to
Example 416 using (rac)-3-[(2-chlorophenyl)methoxy]-5-[5',6'-dihydrospiro[pyrrolidine-3,4'-pyrrolo[1,2-b]pyrazol]-2'-yl]pyridin-2-amine-hydrochloride salt (66.0 mg, 141 µmol) and isocyanatocyclobutane (14.4 mg, 148 µmol).
LC-MS (Method 1): Rt = 1.09 min; MS (ESlpos): m/z = 493 [M+H]⁺
¹H NMR (400 MHz, DMSO-*d*₆) δ ppm 1.46 - 1.63 (m, 2 H) 1.86 - 1.93 (m, 1 H) 1.93 - 1.99 (m, 1 H) 2.00 - 2.04 (m, 1 H) 2.06 (br d, 1 H) 2.08 - 2.10 (m, 1 H) 2.10 - 2.15 (m, 1 H) 2.52 - 2.54 (m, 1 H) 3.36 - 3.39 (m, 1 H) 3.39 - 3.41 (m, 2 H) 3.42 (br s, 1 H) 3.46 - 3.54 (m, 1 H) 4.06 - 4.19 (m, 3 H) 5.22 (s, 2 H) 5.82 (s, 2 H) 6.27 - 6.37 (m, 2 H) 7.35 - 7.54 (m, 4 H) 7.66 - 7.76 (m, 1 H) 7.91 - 7.98 (m, 1 H)

### Example 432

### (rac)-2'-{6-amino-5-[(2-fluorophenyl)methoxy]pyridin-3-yl}-N-ethyl-5',6'-dihydrospiro[pyrrolidine-3,4'-pyrrolo[1,2-b]pyrazole]-1-carboxamide

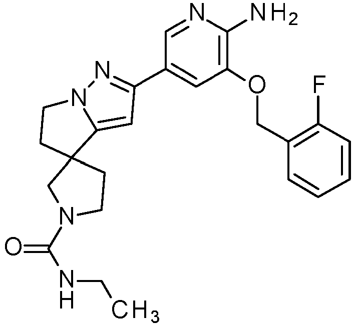

Example 432 was prepared in analogy to
Example 416 using (*rac*)-5-[5',6'-dihydrospiro[pyrrolidine-3,4'-pyrrolo[1,2-b]pyrazol]-2'-yl]-3-[(2-fluorophenyl)methoxy]pyridin-2-amine-hydrochloride salt (104 mg, 230 µmol) and isocyanatoethane (20 µL, 240 µmol).
LC-MS (Method 1): Rt = 0.97 min; MS (ESlpos): m/z = 451 [M+H]⁺
¹H NMR (400 MHz, DMSO-*d*₆) δ ppm 1.02 (t, 4 H) 1.10 - 1.26 (m, 1 H) 1.99 - 2.11 (m, 2 H) 2.96 - 3.09 (m, 2 H) 3.36 - 3.53 (m, 4 H) 4.16 (t, 2 H) 5.21 (s, 2 H) 5.77 (s, 2 H) 6.16 (t, 1 H) 6.36 (s, 1 H) 7.22 - 7.29 (m, 2 H) 7.39 - 7.49 (m, 2 H) 7.67 (td, 1 H) 7.96 (d, 1 H)

### Example 433

### (rac)-2'-{6-amino-5-[(2-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide

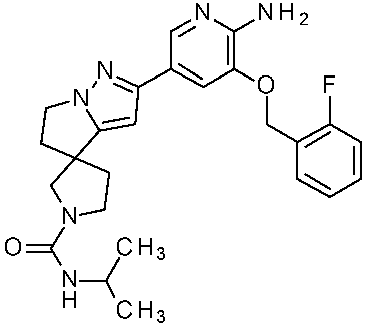

Example 433 was prepared in analogy to
Example 416 using (*rac*)-5-[5',6'-dihydrospiro[pyrrolidine-3,4'-pyrrolo[1,2-b]pyrazol]-2'-yl]-3-[(2-fluorophenyl)methoxy]pyridin-2-amine-hydrochloride salt (104 mg, 230 µmol) and 2-isocyanatopropane (20.5 mg, 241 µmol).
LC-MS (Method 1): Rt = 1.03 min; MS (ESlpos): m/z = 465 [M+H]⁺
¹H NMR (400 MHz, DMSO-*d*₆) δ ppm 1.02 - 1.09 (m, 6 H) 1.99 - 2.10 (m, 2 H) 2.52 - 2.55 (m, 1 H) 3.36 - 3.43 (m, 3 H) 3.45 - 3.58 (m, 1 H) 3.71 - 3.81 (m, 1 H) 4.17 (t, 2 H) 5.21 (s, 2 H) 5.73 - 5.84 (m, 3 H) 6.36 (s, 1 H) 7.23 - 7.29 (m, 2 H) 7.39 - 7.49 (m, 2 H) 7.67 (td, 1 H) 7.96 (d, 1 H)

### Example 434

### (rac)-2'-{6-amino-5-[(2-fluorophenyl)methoxy]pyridin-3-yl}-N-cyclobutyl-5',6'-dihydrospiro[pyrrolidine-3,4'-pyrrolo[1,2-b]pyrazole]-1-carboxamide

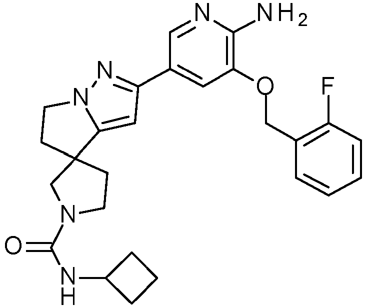

Example 434 was prepared in analogy to
Example 416 using (*rac*)-5-[5',6'-dihydrospiro[pyrrolidine-3,4'-pyrrolo[1,2-b]pyrazol]-2'-yl]-3-[(2-fluorophenyl)methoxy]pyridin-2-amine-hydrochloride salt (104 mg, 230 µmol) and isocyanatocyclobutane (23.4 mg, 241 µmol).
LC-MS (Method 1): Rt = 1.05 min; MS (ESlpos): m/z = 477 [M+H]⁺
¹H NMR (400 MHz, DMSO-*d*₆) δ ppm 1.46 - 1.61 (m, 2 H) 1.87 - 2.13 (m, 6 H) 2.52 - 2.55 (m, 1 H) 3.35 - 3.53 (m, 4 H) 4.07 - 4.20 (m, 3 H) 5.21 (s, 2 H) 5.76 (s, 2 H) 6.31 (d, 1 H) 6.36 (s, 1 H) 7.22 - 7.29 (m, 2 H) 7.39 - 7.49 (m, 2 H) 7.67 (td, 1 H) 7.96 (d, 1 H)

### Example 435

### (rac)-2'-{6-amino-5-[(2-fluorophenyl)methoxy]pyridin-3-yl}-N-benzyl-5',6'-dihydrospiro[pyrrolidine-3,4'-pyrrolo[1,2-b]pyrazole]-1-carboxamide

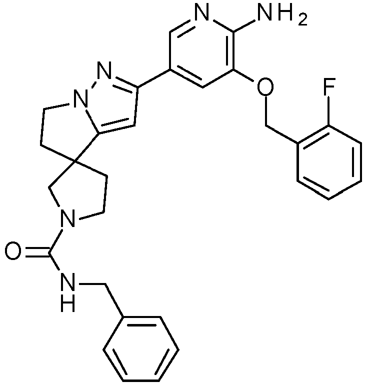

Example 435 was prepared in analogy to
Example 416 using (rac)-5-[5',6'-dihydrospiro[pyrrolidine-3,4'-pyrrolo[1,2-b]pyrazol]-2'-yl]-3-[(2-fluorophenyl)methoxy]pyridin-2-amine-hydrochloride salt (104 mg, 230 µmol) and (isocyanatomethyl)benzene (32.1 mg, 241 µmol).
LC-MS (Method 1): Rt = 1.09 min; MS (ESlpos): m/z = 513 [M+H]⁺
¹H NMR (400 MHz, DMSO-*d*₆) δ ppm 2.04 - 2.14 (m, 2 H) 3.46 (s, 3 H) 3.53 - 3.61 (m, 1 H) 4.17 (t, 2 H) 4.26 (d, 3 H) 5.21 (s, 2 H) 5.79 (br s, 2 H) 6.36 (s, 1 H) 6.80 (t, 1 H) 7.18 - 7.32 (m, 8 H) 7.39 - 7.45 (m, 1 H) 7.49 (s, 1 H) 7.67 (t, 1 H) 7.96 (d, 1 H)

### Example 436

### (rac)-2'-{6-amino-5-[(3-fluorophenyl)methoxy]pyridin-3-yl}-N-ethyl-5',6'-dihydrospiro[pyrrolidine-3,4'-pyrrolo[1,2-b]pyrazole]-1-carboxamide

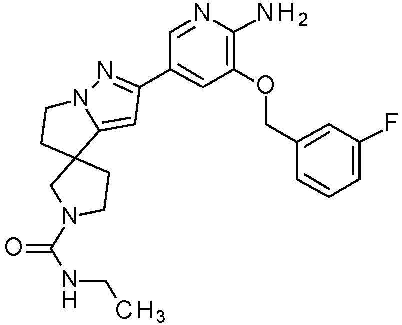

Example 436 was prepared in analogy to
Example 416 using (*rac*)-5-[5',6'-dihydrospiro[pyrrolidine-3,4'-pyrrolo[1,2-b]pyrazol]-2'-yl]-3-[(3-fluorophenyl)methoxy]pyridin-2-amine-hydrochloride salt (90.0 mg, 199 µmol) and isocyanatoethane (17 µL, 210 µmol).
LC-MS (Method 1): Rt = 0.97 min; MS (ESlpos): m/z = 451 [M+H]+
¹H NMR (400 MHz, DMSO-*d*₆) δ ppm 1.02 (t, 3 H) 1.08 - 1.35 (m, 1 H) 1.99 - 2.11 (m, 2 H) 3.01 - 3.09 (m, 2 H) 3.35 - 3.52 (m, 4 H) 4.16 (t, 2 H) 5.20 (s, 2 H) 5.87 (s, 2 H) 6.15 (t, 1 H) 6.34 (s, 1 H) 7.15 (t, 1 H) 7.35 - 7.47 (m, 4 H) 7.95 (d, 1 H)

### Example 437

### (rac)-2'-{6-amino-5-[(3-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide

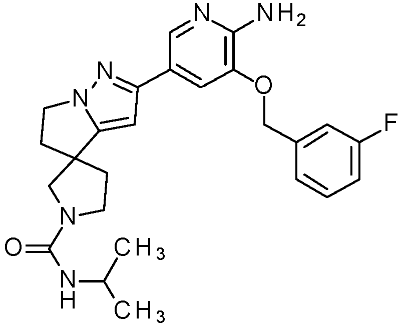

Example 437 was prepared in analogy to
Example 416 using (rac)-5-[5',6'-dihydrospiro[pyrrolidine-3,4'-pyrrolo[1,2-b]pyrazol]-2'-yl]-3-[(3-fluorophenyl)methoxy]pyridin-2-amine-hydrochloride salt (86.0 mg, 190 µmol) and 2-isocyanatopropane (17.0 mg, 200 µmol).
LC-MS (Method 1): Rt = 1.03 min; MS (ESlpos): m/z = 465 [M+H]⁺
¹H NMR (400 MHz, DMSO-*d*₆) δ ppm 1.02 - 1.11 (m, 6 H) 1.99 - 2.10 (m, 2 H) 2.52 - 2.57 (m, 2 H) 3.35 - 3.53 (m, 4 H) 3.76 (dq, 1 H) 4.16 (t, 2 H) 5.20 (s, 2 H) 5.80 - 5.92 (m, 3 H) 6.34 (s, 1 H) 7.15 (t, 1 H) 7.35 - 7.47 (m, 4 H) 7.94 (d, 1 H)

### Example 438

### (rac)-2'-{6-amino-5-[(3-fluorophenyl)methoxy]pyridin-3-yl}-N-cyclobutyl-5',6'-dihydrospiro[pyrrolidine-3,4'-pyrrolo[1,2-b]pyrazole]-1-carboxamide

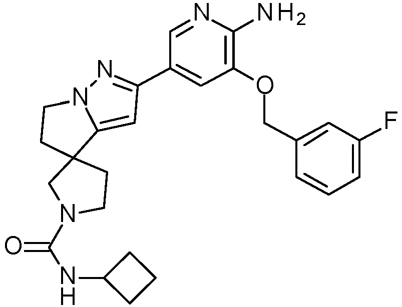

Example 438 was prepared in analogy to
Example 416 using (*rac*)-5-[5',6'-dihydrospiro[pyrrolidine-3,4'-pyrrolo[1,2-b]pyrazol]-2'-yl]-3-[(3-fluorophenyl)methoxy]pyridin-2-amine-hydrochloride salt (86.0 mg, 190 µmol) and isocyanatocyclobutane (19.4 mg, 200 µmol).
LC-MS (Method 1): Rt = 1.05 min; MS (ESlpos): m/z = 477 [M+H]⁺
¹H NMR (400 MHz, DMSO-*d*₆) δ ppm 1.46 - 1.62 (m, 2 H) 1.87 - 2.14 (m, 6 H) 2.52 - 2.54 (m, 1 H) 3.36 - 3.53 (m, 4 H) 4.07 - 4.20 (m, 3 H) 5.21 (s, 2 H) 5.88 (s, 2 H) 6.31 (d, 1 H) 6.34 (s, 1 H) 7.15 (t, 1 H) 7.35 - 7.47 (m, 4 H) 7.95 (d, 1 H)

### Example 439

### (rac)-2'-{6-amino-5-[(3-fluorophenyl)methoxy]pyridin-3-yl}-N-benzyl-5',6'-dihydrospiro[pyrrolidine-3,4'-pyrrolo[1,2-b]pyrazole]-1-carboxamide

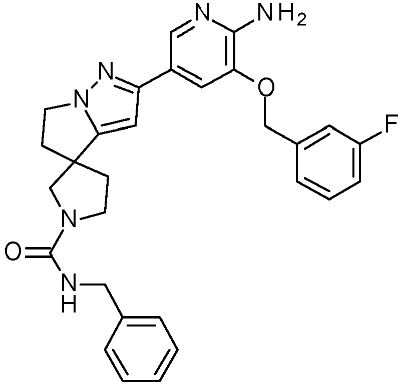

Example 439 was prepared in analogy to
Example 416 using (*rac*)-5-[5',6'-dihydrospiro[pyrrolidine-3,4'-pyrrolo[1,2-b]pyrazol]-2'-yl]-3-[(3-fluorophenyl)methoxy]pyridin-2-amine-hydrochloride salt (86.0 mg, 190 µmol) and (isocyanatomethyl)benzene (26.6 mg, 200 µmol).
LC-MS (Method 1): Rt = 1.10 min; MS (ESlpos): m/z = 513 [M+H]⁺
¹H NMR (400 MHz, DMSO-*d*₆) δ ppm 2.03 - 2.14 (m, 2 H) 2.53 - 2.56 (m, 2 H) 3.38 - 3.59 (m, 4 H) 4.17 (t, 2 H) 4.26 (d, 2 H) 5.21 (s, 2 H) 5.89 (s, 2 H) 6.34 (s, 1 H) 6.80 (t, 1 H) 7.12 - 7.24 (m, 2 H) 7.27 - 7.33 (m, 4 H) 7.35 - 7.39 (m, 1 H) 7.40 - 7.47 (m, 3 H) 7.94 (d, 1 H)

### Example 440

### (rac)-2'-{6-amino-5-[(pyridin-2-yl)methoxy]pyridin-3-yl}-N-ethyl-5',6'-dihydrospiro[pyrrolidine-3,4'-pyrrolo[1,2-b]pyrazole]-1-carboxamide

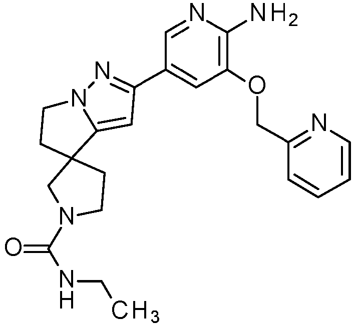

Example 440 was prepared in analogy to
Example 416 using (*rac*)-5-[5',6'-dihydrospiro[pyrrolidine-3,4'-pyrrolo[1,2-b]pyrazol]-2'-yl]-3-[(pyridin-2-yl)methoxy]pyridin-2-amine-hydrochloride salt (106 mg, 225 µmol) and isocyanatoethane (19 µL, 240 µmol).
LC-MS (Method 1): Rt = 0.79 min; MS (ESlpos): m/z = 434 [M+H]⁺
¹H NMR (400 MHz, DMSO-*d*₆) δ ppm 1.02 (t, 3 H) 1.99 - 2.10 (m, 2 H) 2.52 - 2.56 (m, 2 H) 3.01 - 3.09 (m, 2 H) 3.35 - 3.52 (m, 4 H) 4.12 - 4.22 (m, 2 H) 5.24 (s, 2 H) 5.90 (s, 2 H) 6.15 (t, 1 H) 6.34 (s, 1 H) 7.35 (ddd, 1 H) 7.41 (d, 1 H) 7.67 (d, 1 H) 7.84 (td, 1 H) 7.95 (d, 1 H) 8.58 (d, 1 H)

### Example 441

### (rac)-2'-{6-amino-5-[(pyridin-2-yl)methoxy]pyridin-3-yl}-N-(propan-2-yl)-5',6'-dihydrospiro[pyrrolidine-3,4'-pyrrolo[1,2-b]pyrazole]-1-carboxamide

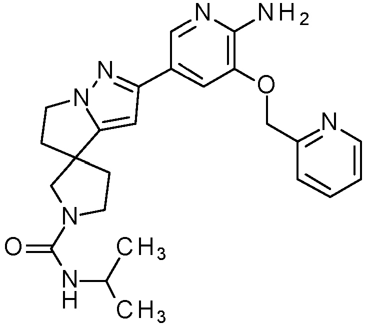

Example 441 was prepared in analogy to
Example 416 using (*rac*)-5-[5',6'-dihydrospiro[pyrrolidine-3,4'-pyrrolo[1,2-b]pyrazol]-2'-yl]-3-[(pyridin-2-yl)methoxy]pyridin-2-amine-hydrochloride salt (106 mg, 225 µmol) and 2-isocyanatopropane (20.1 mg, 236 µmol).
LC-MS (Method 1): Rt = 0.85 min; MS (ESlpos): m/z = 448 [M+H]⁺
¹H NMR (400 MHz, DMSO-*d*₆) δ ppm 1.06 (br d, 3 H) 1.07 (br d, 3 H) 1.98 - 2.09 (m, 2 H) 2.52 - 2.56 (m, 2 H) 3.35 - 3.43 (m, 3 H) 3.45 - 3.57 (m, 1 H) 3.70 - 3.81 (m, 1 H) 4.15 (t, 2 H) 5.24 (s, 2 H) 5.82 (d, 1 H) 5.90 (s, 2 H) 6.34 (s, 1 H) 7.32 - 7.43 (m, 2 H) 7.67 (d, 1 H) 7.84 (td, 1 H) 7.95 (d, 1 H) 8.58 (d, 1 H)

### Example 442

### (rac)-2'-{6-amino-5-[(pyridin-2-yl)methoxy]pyridin-3-yl}-N-cyclobutyl-5',6'-dihydrospiro[pyrrolidine-3,4'-pyrrolo[1,2-b]pyrazole]-1-carboxamide

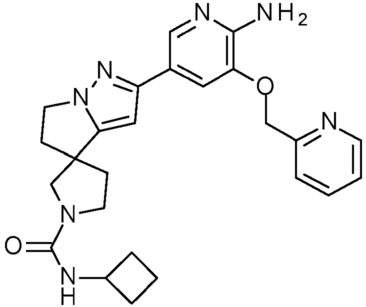

Example 442 was prepared in analogy to
Example 416 using (*rac*)-5-[5',6'-dihydrospiro[pyrrolidine-3,4'-pyrrolo[1,2-b]pyrazol]-2'-yl]-3-[(pyridin-2-yl)methoxy]pyridin-2-amine-hydrochloride salt (106 mg, 225 µmol) and isocyanatocyclobutane (22.9 mg, 236 µmol).
LC-MS (Method 1): Rt = 0.87 min; MS (ESlpos): m/z = 460 [M+H]⁺
¹H NMR (400 MHz, DMSO-*d*₆) δ ppm 1.46 - 1.62 (m, 2 H) 1.87 - 2.13 (m, 6 H) 2.52 - 2.56 (m, 2 H) 3.35 - 3.52 (m, 4 H) 4.06 - 4.22 (m, 3 H) 5.24 (s, 2 H) 5.90 (s, 2 H) 6.31 (d, 1 H) 6.34 (s, 1 H) 7.32 - 7.44 (m, 2 H) 7.67 (d, 1 H) 7.84 (td, 1 H) 7.95 (d, 1 H) 8.58 (d, 1 H)

### Example 443

### (rac)-2'-{6-amino-5-[(2-chloro-5-fluorophenyl)methoxy]pyridin-3-yl}-N-ethyl-5',6'-dihydrospiro[pyrrolidine-3,4'-pyrrolo[1,2-b]pyrazole]-1-carboxamide

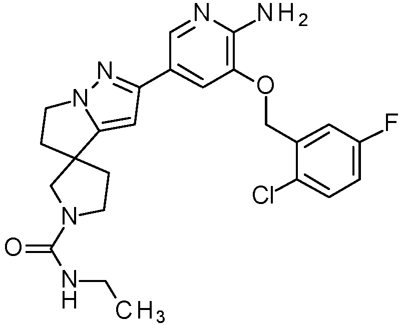

Example 443 was prepared in analogy to
Example 416 using (*rac*)-3-[(2-chloro-5-fluorophenyl)methoxy]-5-[5',6'-dihydrospiro[pyrrolidine-3,4'-pyrrolo[1,2-b]pyrazol]-2'-yl]pyridin-2-amine-hydrochloride salt (80.0 mg, 164 µmol) and isocyanatoethane (14 µL, 170 µmol).
LC-MS (Method 1): Rt = 1.03 min; MS (ESlpos): m/z = 485 [M+H]⁺
¹H NMR (400 MHz, DMSO-*d*₆) δ ppm 1.02 (t, 5 H) 2.00 - 2.11 (m, 3 H) 2.52 - 2.56 (m, 2 H) 3.01 - 3.10 (m, 3 H) 4.18 (t, 3 H) 5.22 (s, 3 H) 6.13 - 6.19 (m, 1 H) 6.39 (s, 1 H) 7.27 (td, 1 H) 7.51 (d, 1 H) 7.57 (dd, 1 H) 7.72 (dd, 1 H) 7.97 (d, 1 H)

### Example 444

### (rac)-2'-{6-amino-5-[(2-chloro-5-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide

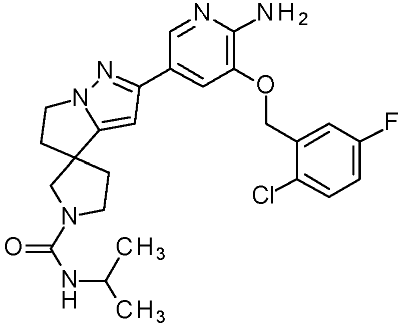

Example 444 was prepared in analogy to
Example 416 using (*rac*)-3-[(2-chloro-5-fluorophenyl)methoxy]-5-[5',6'-dihydrospiro[pyrrolidine-3,4'-pyrrolo[1,2-b]pyrazol]-2'-yl]pyridin-2-amine-hydrochloride salt (80.0 mg, 164 µmol) and 2-isocyanatopropane (14.7 mg, 173 µmol).
LC-MS (Method 1): Rt = 1.09 min; MS (ESlpos): m/z = 499 [M+H]⁺
¹H NMR (400 MHz, DMSO-*d*₆) δ ppm 1.06 (br d, 3 H) 1.07 (br d, 3 H) 1.98 - 2.11 (m, 2 H) 2.52 - 2.54 (m, 2 H) 3.36 - 3.58 (m, 4 H) 3.71 - 3.81 (m, 1 H) 4.17 (t, 2 H) 5.21 (s, 2 H) 5.82 (d, 1 H) 6.02 (br s, 2 H) 6.37 (s, 1 H) 7.27 (td, 1 H) 7.47 (d, 1 H) 7.57 (dd, 1 H) 7.72 (dd, 1 H) 7.97 (d, 1 H)

### Example 445

### 2'-{6-amino-5-[(1R)-1-(pyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide (mixture of stereoisomers)

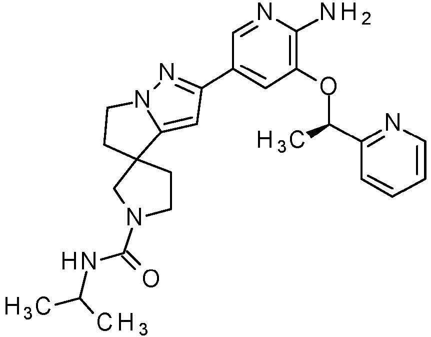

Example 445 was prepared in analogy to
Example 416 using (*rac*)-5-[5',6'-dihydrospiro[pyrrolidine-3,4'-pyrrolo[1,2-b]pyrazol]-2'-yl]-3-[(1R)-1-(pyridin-2-yl)ethoxy]pyridin-2-amine-hydrochloride salt (50.0 mg, 121 µmol, diastereomeric mixture) and 2-isocyanatopropane (12 µL, 120 µmol).
LC-MS (method 1): Rt = 0.89 min; MS (ESlpos): m/z = 463 [M+H]⁺
¹H-NMR (400 MHz, DMSO-d6) δ [ppm]: 0.995 (0.76), 1.012 (0.81), 1.022 (0.40), 1.034 (0.64), 1.047 (11.90), 1.050 (12.63), 1.057 (14.52), 1.059 (15.07), 1.064 (14.31), 1.066 (13.81), 1.074 (13.59), 1.609 (16.00), 1.625 (15.96), 1.984 (1.18), 1.997 (2.26), 2.003 (2.46), 2.015 (3.00), 2.021 (2.69), 2.033 (1.32), 2.039 (1.08), 2.052 (0.53), 2.460 (3.18), 2.479 (5.83), 2.518 (2.93), 2.522 (1.80), 3.368 (10.79), 3.372 (11.23), 3.397 (1.03), 3.451 (1.10), 3.469 (1.91), 3.477 (1.25), 3.483 (1.47), 3.491 (1.18), 3.495 (1.18), 3.509 (0.75), 3.725 (0.87), 3.730 (1.06), 3.746 (1.66), 3.762 (1.66), 3.778 (1.04), 3.783 (0.88), 4.103 (3.96), 4.120 (7.01), 4.138 (3.93), 5.490 (0.99), 5.506 (3.73), 5.522 (3.76), 5.538 (1.02), 5.798 (3.33), 5.817 (3.28), 5.892 (9.98), 6.213 (10.52), 6.216 (11.00), 7.200 (3.62), 7.206 (6.03), 7.211 (3.81), 7.269 (1.33), 7.272 (1.62), 7.275 (1.60), 7.277 (1.57), 7.282 (1.55), 7.284 (1.82), 7.288 (2.54), 7.290 (2.59), 7.293 (1.90), 7.296 (1.72), 7.300 (1.74), 7.303 (1.78), 7.305 (1.72), 7.308 (1.61), 7.490 (2.67), 7.492 (2.82), 7.494 (2.74), 7.509 (3.07), 7.512 (3.27), 7.514 (3.08), 7.763 (1.52), 7.766 (2.21), 7.770 (1.68), 7.782 (2.41), 7.785 (3.74), 7.789 (2.62), 7.801 (1.30), 7.805 (1.80), 7.809 (1.25), 7.864 (7.71), 7.868 (7.99), 8.537 (1.71), 8.541 (3.17), 8.544 (3.17), 8.546 (2.09), 8.549 (2.07), 8.551 (3.10), 8.554 (3.17), 8.558 (1.79).

### Example 446

### 2'-{6-amino-5-[(1R)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

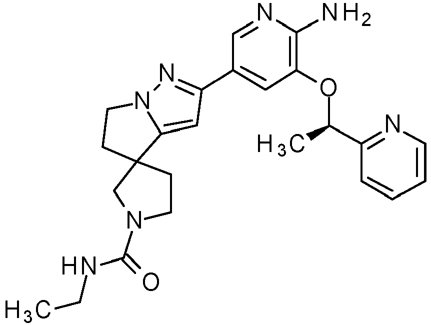

Example 446 was prepared in analogy to
Example 416 using (*rac*)-5-[5',6'-dihydrospiro[pyrrolidine-3,4'-pyrrolo[1,2-b]pyrazol]-2'-yl]-3-[(1R)-1-(pyridin-2-yl)ethoxy]pyridin-2-amine-hydrochloride salt (50.0 mg, 121 µmol, diastereomeric mixture) and isocyanatoethane (9.9 µL, 120 µmol).
LC-MS (method 1): Rt = 0.83 min; MS (ESlpos): m/z = 449 [M+H]⁺
¹H-NMR (400 MHz, DMSO-d6) δ [ppm]: 0.997 (6.34), 1.000 (6.79), 1.015 (14.29), 1.018 (14.57), 1.033 (7.01), 1.035 (6.94), 1.137 (0.70), 1.154 (0.74), 1.589 (0.72), 1.609 (15.35), 1.626 (15.58), 2.009 (2.73), 2.021 (3.20), 2.026 (3.01), 2.038 (1.52), 2.459 (3.50), 2.478 (6.13), 3.015 (0.89), 3.018 (0.97), 3.032 (3.32), 3.036 (2.99), 3.050 (4.93), 3.064 (3.00), 3.068 (3.29), 3.082 (1.01), 3.086 (0.90), 3.343 (16.00), 3.365 (11.75), 3.398 (1.45), 3.442 (1.19), 3.460 (2.03), 3.467 (1.38), 3.474 (1.56), 3.481 (1.31), 3.499 (0.77), 4.102 (3.98), 4.120 (6.86), 4.137 (4.05), 5.495 (1.02), 5.512 (3.71), 5.528 (3.74), 5.544 (1.08), 5.913 (5.18), 6.131 (1.80), 6.145 (3.59), 6.159 (1.84), 6.215 (9.05), 6.218 (9.47), 7.209 (3.98), 7.214 (6.86), 7.218 (4.13), 7.269 (1.36), 7.273 (1.82), 7.276 (1.67), 7.281 (1.64), 7.288 (2.97), 7.295 (1.85), 7.299 (1.79), 7.304 (2.08), 7.307 (1.70), 7.491 (3.58), 7.494 (3.65), 7.512 (4.15), 7.514 (4.10), 7.765 (1.96), 7.767 (2.02), 7.769 (1.83), 7.787 (3.44), 7.804 (1.64), 7.806 (1.65), 7.868 (6.80), 7.871 (6.93), 8.539 (3.38), 8.542 (3.79), 8.544 (3.63), 8.552 (3.44), 8.554 (3.66), 8.556 (3.33).

### Example 447

### 2'-{6-amino-5-[(1S)-1-(pyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide (mixture of stereoisomers)

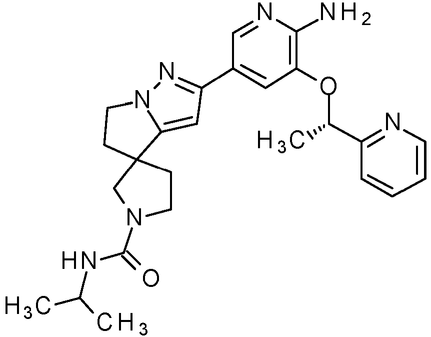

Example 447 was prepared in analogy to
Example 416 using (*rac*)-5-[5',6'-dihydrospiro[pyrrolidine-3,4'-pyrrolo[1,2-b]pyrazol]-2'-yl]-3-[(1S)-1-(pyridin-2-yl)ethoxy]pyridin-2-amine-hydrochloride salt (65.0 mg, 157 µmol, diastereomeric mixture) and 2-isocyanatopropane (15 µL, 160 µmol).
LC-MS (method 1): Rt = 0.89 min; MS (ESlpos): m/z = 463 [M+H]⁺
¹H-NMR (400 MHz, DMSO-d6) δ [ppm]: 0.995 (0.99), 1.012 (1.06), 1.022 (0.44), 1.034 (0.71), 1.047 (12.32), 1.050 (12.96), 1.059 (15.65), 1.064 (14.76), 1.066 (13.86), 1.074 (13.98), 1.609 (16.00), 1.625 (15.88), 1.984 (1.26), 1.997 (2.40), 2.003 (2.59), 2.015 (3.11), 2.021 (2.82), 2.033 (1.36), 2.039 (1.12), 2.051 (0.55), 2.460 (3.28), 2.479 (6.16), 2.518 (3.10), 2.522 (1.92), 3.368 (11.29), 3.372 (11.61), 3.397 (1.12), 3.451 (1.16), 3.469 (1.97), 3.477 (1.31), 3.483 (1.52), 3.491 (1.22), 3.495 (1.24), 3.509 (0.76), 3.730 (1.09), 3.746 (1.72), 3.762 (1.69), 3.778 (1.05), 4.103 (4.08), 4.120 (7.21), 4.138 (3.98), 5.198 (0.45), 5.490 (1.04), 5.506 (3.77), 5.522 (3.80), 5.539 (1.06), 5.798 (3.44), 5.817 (3.37), 5.892 (10.26), 6.213 (10.18), 6.216 (10.73), 7.200 (3.72), 7.206 (6.17), 7.211 (3.91), 7.270 (1.32), 7.272 (1.61), 7.275 (1.64), 7.277 (1.56), 7.282 (1.55), 7.284 (1.84), 7.290 (2.64), 7.293 (1.93), 7.296 (1.72), 7.300 (1.69), 7.303 (1.81), 7.305 (1.77), 7.308 (1.60), 7.490 (2.74), 7.492 (2.92), 7.494 (2.81), 7.509 (3.13), 7.512 (3.36), 7.514 (3.16), 7.763 (1.51), 7.766 (2.28), 7.770 (1.66), 7.782 (2.53), 7.785 (3.80), 7.789 (2.67), 7.801 (1.29), 7.805 (1.83), 7.809 (1.26), 7.863 (7.74), 7.868 (7.96), 8.537 (1.77), 8.541 (3.31), 8.546 (2.17), 8.549 (2.17), 8.554 (3.30).

### Example 448

### 2'-{6-amino-5-[(1S)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

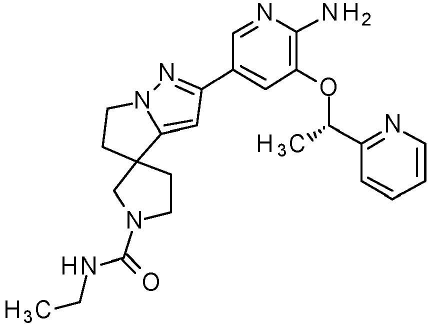

Example 448 was prepared in analogy to
Example 416 using (*rac*)-5-[5',6'-dihydrospiro[pyrrolidine-3,4'-pyrrolo[1,2-b]pyrazol]-2'-yl]-3-[(1S)-1-(pyridin-2-yl)ethoxy]pyridin-2-amine-hydrochloride salt (65.0 mg, 157 µmol, diastereomeric mixture) and isocyanatoethane (13 µL, 160 µmol).
LC-MS (method 1): Rt = 0.83 min; MS (ESlpos): m/z = 449 [M+H]⁺
¹H-NMR (400 MHz, DMSO-d6) δ [ppm]: 0.997 (6.32), 1.000 (6.65), 1.015 (14.84), 1.018 (14.39), 1.033 (6.91), 1.036 (6.63), 1.587 (0.71), 1.609 (15.81), 1.625 (16.00), 1.991 (1.11), 2.009 (2.33), 2.021 (2.79), 2.026 (2.63), 2.039 (1.26), 2.045 (1.02), 2.057 (0.45), 2.460 (3.23), 2.479 (5.77), 2.518 (2.12), 2.523 (1.36), 3.014 (0.79), 3.018 (0.87), 3.032 (3.12), 3.036 (2.73), 3.046 (2.94), 3.050 (4.69), 3.053 (2.95), 3.064 (2.70), 3.067 (3.05), 3.081 (0.83), 3.085 (0.75), 3.319 (0.50), 3.365 (9.75), 3.369 (10.19), 3.379 (2.75), 3.398 (1.01), 3.441 (1.06), 3.459 (1.81), 3.466 (1.13), 3.473 (1.35), 3.480 (1.08), 3.485 (1.10), 3.499 (0.66), 4.102 (3.87), 4.120 (6.55), 4.137 (3.79), 5.492 (0.98), 5.508 (3.70), 5.525 (3.67), 5.541 (1.01), 5.891 (9.60), 6.129 (1.69), 6.143 (3.42), 6.156 (1.67), 6.212 (10.16), 6.216 (10.34), 7.204 (3.60), 7.209 (6.39), 7.213 (3.71), 7.270 (1.38), 7.273 (1.76), 7.277 (1.59), 7.282 (1.49), 7.285 (1.96), 7.289 (2.70), 7.291 (2.10), 7.296 (1.59), 7.300 (1.60), 7.305 (1.90), 7.308 (1.55), 7.491 (3.60), 7.494 (3.60), 7.511 (4.04), 7.513 (3.95), 7.763 (1.61), 7.765 (1.88), 7.768 (1.84), 7.770 (1.72), 7.784 (3.09), 7.787 (3.08), 7.802 (1.34), 7.804 (1.53), 7.806 (1.44), 7.808 (1.32), 7.865 (6.13), 7.867 (6.82), 7.870 (6.80), 7.872 (6.21), 8.539 (3.17), 8.542 (3.68), 8.544 (3.40), 8.551 (3.18), 8.554 (3.55), 8.556 (3.12).

### Example 449

### 2'-(6-amino-5-{[1-(5-chloro-3-fluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomeric mixture)

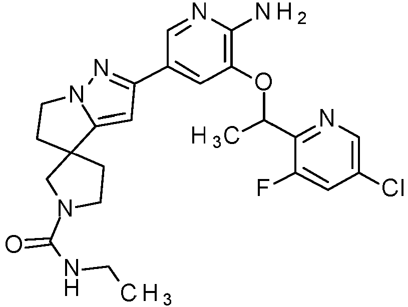

Example 449 was prepared in analogy to
Example 416 using 3-{[1-(5-chloro-3-fluoropyridin-2-yl)ethyl]oxy}-5-[5',6'-dihydrospiro[pyrrolidine-3,4'-pyrrolo[1,2-b]pyrazol]-2'-yl]pyridin-2-amine-hydrochloride salt (82.0 mg, 163 µmol, diastereomeric mixture) and isocyanatoethane (14 µL, 172 µmol).
LC-MS (Method 1): Rt = 0.96 min; MS (ESlpos): m/z = 500 [M+H]⁺
¹H NMR (400 MHz, DMSO-*d*₆) δ ppm 1.02 (t, 3 H) 1.17 - 1.26 (m, 2 H) 1.74 (d, 3 H) 2.05 (t, 2 H) 2.52 - 2.56 (m, 3 H) 2.99 - 3.11 (m, 2 H) 3.42 - 3.51 (m, 2 H) 4.19 (t, 2 H) 6.04 - 6.20 (m, 2 H) 6.44 (s, 1 H) 7.64 - 7.76 (m, 1 H) 7.80 (br dd, 1 H) 7.87 (s, 1 H) 8.13 - 8.21 (m, 1 H) 8.57 (s, 1 H)

### Example 450

### (rac)-2'-[6-amino-5-(benzyloxy)pyridin-3-yl]-N-cyclobutyl-5',6'-dihydrospiro[pyrrolidine-3,4'-pyrrolo[1,2-b]pyrazole]-1-carboxamide

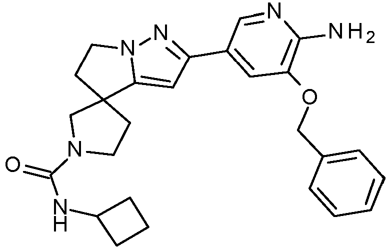

Example 450 was prepared in analogy to
Example 416 using (*rac*)-3-(benzyloxy)-5-[5',6'-dihydrospiro[pyrrolidine-3,4'-pyrrolo[1,2-b]pyrazol]-2'-yl]pyridin-2-amine-hydrochloride salt (150 mg, 50 % purity, 173 µmol) and isocyanatocyclobutane (17.6 mg, 181 µmol).
¹H-NMR (400MHz, DMSO-d6): δ [ppm]= 1.45 - 1.62 (m, 2H), 1.86 - 2.15 (m, 6H), 3.36 - 3.44 (m, 3H), 3.46 - 3.54 (m, 1H), 4.06 - 4.19 (m, 3H), 5.18 (s, 2H), 5.80 (s, 2H), 6.31 (d, 1H), 6.34 (s, 1H), 7.30 - 7.35 (m, 1H), 7.37 - 7.42 (m, 2H), 7.43 (d, 1H), 7.50 - 7.54 (m, 2H), 7.94 (d, 1H).
LC-MS (Method 1): Rt = 1.03 min; MS (ESlpos): m/z = 459 [M+H]⁺

### Example 451

### 2'-{6-amino-5-[(pyridazin-3-yl)methoxy]pyridin-3-yl}-N-ethyl-5',6'-dihydrospiro[pyrrolidine-3,4'-pyrrolo[1,2-b]pyrazole]-1-carboxamide (stereoisomer 2)

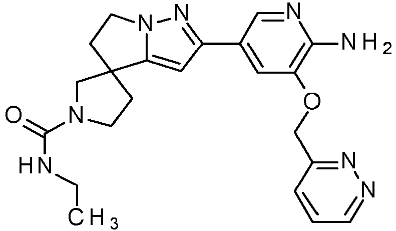

Example 451 was prepared in analogy to
Example 416 using 5-[5',6'-dihydrospiro[pyrrolidine-3,4'-pyrrolo[1,2-b]pyrazol]-2'-yl]-3-[(pyridazin-3-yl)methoxy]pyridin-2-amine-hydrochloride salt (205 mg, 50 % purity, 235 µmol, stereoisomer 2) and isocyanatoethane (20 µL, 250 µmol).
1H-NMR (400MHz, DMSO-d6): δ [ppm]= 1.02 (t, 3H), 1.99 - 2.11 (m, 2H), 3.01 - 3.09 (m, 2H), 3.36 - 3.44 (m, 3H), 3.46 - 3.53 (m, 1H), 4.16 (t, 2H), 5.47 (s, 2H), 5.98 (s, 2H), 6.15 (t, 1H), 6.37 (s, 1H), 7.50 (d, 1H), 7.77 (dd, 1H), 7.97 (d, 1H), 8.01 (dd, 1H), 9.22 (dd, 1H).
LC-MS (Method 1): Rt = 0.68 min; MS (ESlpos): m/z = 435 [M+H]⁺

### Example 452

### 2'-{6-amino-5-[(pyridazin-3-yl)methoxy]pyridin-3-yl}-N-ethyl-5',6'-dihydrospiro[pyrrolidine-3,4'-pyrrolo[1,2-b]pyrazole]-1-carboxamide (stereoisomer 1)

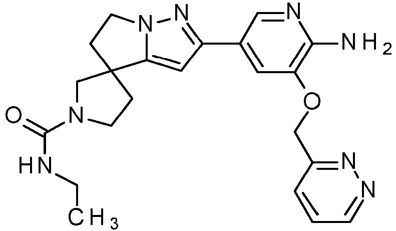

Example 452 was prepared in analogy to
Example 416 using 5-[5',6'-dihydrospiro[pyrrolidine-3,4'-pyrrolo[1,2-b]pyrazol]-2'-yl]-3-[(pyridazin-3-yl)methoxy]pyridin-2-amine-hydrochloride salt (117 mg, 268 µmol, stereoisomer 1) and isocyanatoethane (22 µL, 280 µmol).
1H-NMR (400MHz, DMSO-d6): δ [ppm]= 1.02 (t, 3H), 1.99 - 2.11 (m, 2H), 3.02 - 3.09 (m, 2H), 3.36 - 3.43 (m, 3H), 3.46 - 3.53 (m, 1H), 4.16 (t, 2H), 5.47 (s, 2H), 5.98 (br s, 2H), 6.15 (t, 1H), 6.37 (s, 1H), 7.50 (d, 1H), 7.77 (dd, 1H), 7.97 (d, 1H), 8.01 (dd, 1H), 9.22 (dd, 1H).
LC-MS (Method 1): Rt = 0.70 min; MS (ESlpos): m/z = 435 [M+H]⁺

### Example 453

### (3S)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 1)

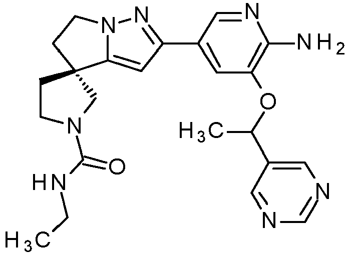

Example 453 was prepared in analogy to
Example 416 using 5-[(3S)-5',6'-dihydrospiro[pyrrolidine-3,4'-pyrrolo[1,2-b]pyrazol]-2'-yl]-3-{[(1R)-1-(pyrimidin-5-yl)ethyl]oxy}pyridin-2-amine-hydrochloride salt (80.0 mg, 193 µmol, isomer 1) and isocyanatoethane (16 µL, 200 µmol).
¹H NMR (DMSO-d6) δ: 9.12 (s, 1H), 8.99 (s, 2H), 7.91 (d, 1H), 7.42 (d, 1H), 6.31 (s, 1H), 6.15 (t, 1H), 5.96 (s, 2H), 5.79 (q, 1H), 4.14 (t, 2H), 3.35-3.53 (m, 4H), 3.01-3.09 (m, 2H), 1.97-2.10 (m, 2H), 1.62 (d, 3H), 1.02 (t, 3H)
LC-MS (Method 1): Rt = 0.73 min; MS (ESlpos): m/z = 449 [M+H]⁺

### Example 454

### (3S)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 2)

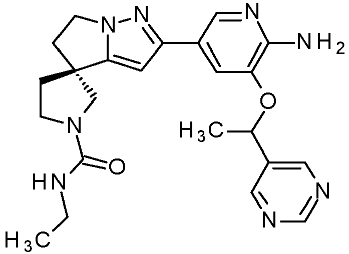

Example 454 was prepared in analogy to
Example 416 using 5-[(3S)-5',6'-dihydrospiro[pyrrolidine-3,4'-pyrrolo[1,2-b]pyrazol]-2'-yl]-3-{[(1S)-1-(pyrimidin-5-yl)ethyl]oxy}pyridin-2-amine-hydrochloride salt (105 mg, 254 µmol, isomer 3) and isocyanatoethane (21 µL, 270 µmol).
[α]²⁰_{D} : +39.6° (c = 1.00, DMSO)
¹H NMR (DMSO-d6) δ: 9.11 (s, 1H), 8.98 (s, 2H), 7.91 (d, 1H), 7.42 (d, 1H), 6.30 (s, 1H), 6.15 (t, 1H), 5.96 (s, 2H), 5.79 (q, 1H), 4.15 (t, 2H), 3.45-3.53 (m, 1H), 3.36-3.43 (m, 3H), 3.01-3.10 (m, 2H), 1.96-2.12 (m, 2H), 1.62 (d, 3H), 1.02 (t, 3H)

### Example 455

### (rac)-2'-(2-amino-1'-methyl-1',2',3',6'-tetrahydro[3,4'-bipyridin]-5-yl)-N-ethyl-5',6'-dihydrospiro[pyrrolidine-3,4'-pyrrolo[1,2-b]pyrazole]-1-carboxamide

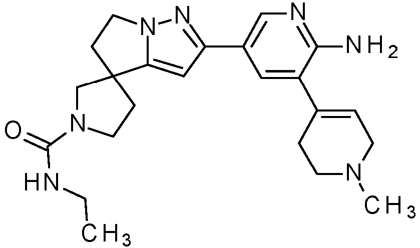

Example 455 was prepared in analogy to
Example 416 using (*rac*)-5-[5',6'-dihydrospiro[pyrrolidine-3,4'-pyrrolo[1,2-b]pyrazol]-2'-yl]-1'-methyl-1',2',3',6'-tetrahydro[3,4'-bipyridin]-2-amine-hydrochloride salt (67.0 mg, 173 µmol) and isocyanatoethane (15 µL, 190 µmol).
1H-NMR (400MHz, CHLOROFORM-d): δ [ppm]= 1.17 (t, 3H), 2.03 - 2.14 (m, 1H), 2.19 - 2.30 (m, 1H), 2.42 (s, 3H), 2.46 - 2.52 (m, 2H), 2.57 (s, 1H), 2.61 - 2.71 (m, 3H), 3.09 (q, 2H), 3.31 (qd, 2H), 3.54 (dt, 3H), 3.60 - 3.68 (m, 1H), 4.16 (s, 1H), 4.25 (t, 2H), 4.64 (s, 2H), 5.87 (dt, 1H), 6.16 (s, 1H), 7.66 (d, 1H), 8.34 (d, 1H).

### Example 456

### 2'-{6-amino-5-[(1S)-1-(pyridin-2-yl)ethoxy]pyridin-3-yl}-N-ethyl-5',6'-dihydrospiro[azetidine-3,4'-pyrrolo[1,2-b]pyrazole]-1-carboxamide

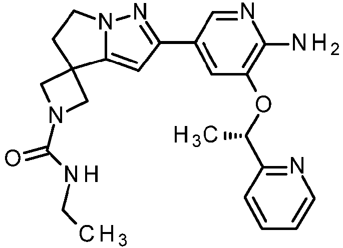

Example 456 was prepared in analogy to
Example 416 using 5-(5',6'-dihydrospiro[azetidine-3,4'-pyrrolo[1,2-b]pyrazol]-2'-yl)-3-[(1S)-1-(pyridin-2-yl)ethoxy]pyridin-2-amine-hydrochloride salt (14.0 mg, 35.1 µmol) and isocyanatoethane (3.1 µL, 39 µmol).
¹H-NMR (400MHz, CHLOROFORM-d): δ [ppm]= 1.16 (t, 3H), 1.25 (s, 2H), 1.73 (d, 3H), 2.80 - 2.90 (m, 2H), 3.29 (qd, 2H), 4.06 (dd, 2H), 4.10 (s, 1H), 4.14 - 4.21 (m, 4H), 4.86 (s, 2H), 5.52 (q, 1H), 6.24 (s, 1H), 7.20 (ddd, 1H), 7.25 (d, 1H), 7.35 - 7.42 (m, 1H), 7.66 (td, 1H), 8.04 (d, 1H), 8.57 - 8.63 (m, 1H).
LC-MS (method 1): Rt = 0.82 min; MS (ESlpos): m/z = 434 [M+H]⁺

### Example 457

### 2'-{6-amino-5-[(1S)-1-(pyridin-2-yl)ethoxy]pyridin-3-yl}-N-(propan-2-yl)-5',6'-dihydrospiro[azetidine-3,4'-pyrrolo[1,2-b]pyrazole]-1-carboxamide

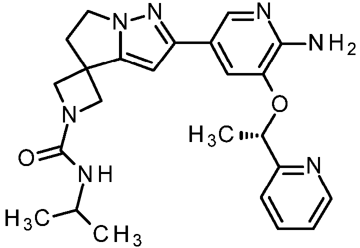

Example 457 was prepared in analogy to
Example 416 using 5-(5',6'-dihydrospiro[azetidine-3,4'-pyrrolo[1,2-b]pyrazol]-2'-yl)-3-[(1S)-1-(pyridin-2-yl)ethoxy]pyridin-2-amine-hydrochloride salt (14.0 mg, 35.1 µmol) and 2-isocyanatopropane (3.8 µL, 39 µmol).
¹H-NMR (400MHz, CHLOROFORM-d): δ [ppm]= 1.18 (d, 6H), 1.73 (d, 3H), 2.82 - 2.88 (m, 2H), 3.88 - 3.94 (m, 1H), 3.94 - 4.01 (m, 1H), 4.04 (dd, 2H), 4.13 - 4.21 (m, 4H), 4.86 (s, 2H), 5.52 (q, 1H), 6.24 (s, 1H), 7.20 (ddd, 1H), 7.25 (d, 1H), 7.35 - 7.41 (m, 1H), 7.66 (td, 1H), 8.05 (d, 1H), 8.58 - 8.63 (m, 1H).
LC-MS (method 1): Rt = 0.89 min; MS (ESlpos): m/z = 448 [M+H]⁺

### Example 458

### 2'-{6-amino-5-[(1S)-1-phenylethoxy]pyridin-3-yl}-N-ethyl-5',6'-dihydrospiro[azetidine-3,4'-pyrrolo[1,2-b]pyrazole]-1-carboxamide

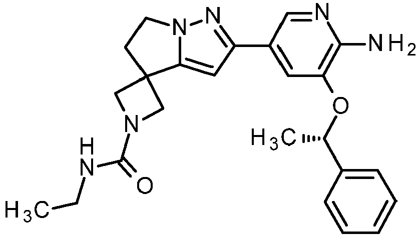

Example 458 was prepared in analogy to
Example 416 using 5-(5',6'-dihydrospiro[azetidine-3,4'-pyrrolo[1,2-b]pyrazol]-2'-yl)-3-[(1S)-1-phenylethoxy]pyridin-2-amine-hydrochloride salt (98.0 mg, 70 % purity, 172 µmol) and isocyanatoethane (13.5 mg, 190 µmol).
1H-NMR (400MHz, DMSO-d6): δ [ppm]= 1.01 (t, 3H), 1.57 (d, 3H), 2.79 (t, 2H), 2.98 - 3.09 (m, 2H), 3.91 - 3.98 (m, 4H), 4.06 (t, 2H), 5.60 (q, 1H), 5.85 (s, 2H), 6.43 - 6.47 (m, 1H), 6.49 (s, 1H), 7.21 - 7.26 (m, 1H), 7.27 (d, 1H), 7.34 (t, 2H), 7.43 - 7.51 (m, 2H), 7.88 (d, 1H).
LC-MS (method 1): Rt = 0.97 min; MS (ESlpos): m/z = 434 [M+H]⁺

### Example 459

### 2'-{6-amino-5-[(1R)-1-phenylethoxy]pyridin-3-yl}-N-ethyl-5',6'-dihydrospiro[azetidine-3,4'-pyrrolo[1,2-b]pyrazole]-1-carboxamide

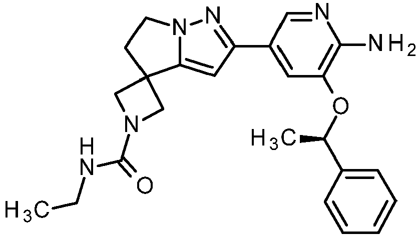

Example 459 was prepared in analogy to
Example 416 using 5-(5',6'-dihydrospiro[azetidine-3,4'-pyrrolo[1,2-b]pyrazol]-2'-yl)-3-[(1R)-1-phenylethoxy]pyridin-2-amine-hydrochloride salt (145 mg, 80 % purity, 292 µmol) and isocyanatoethane (22.8 mg, 321 µmol).
1H-NMR (400MHz, DMSO-d6): δ [ppm]= 1.01 (t, 3H), 1.57 (d, 3H), 2.79 (br t, 2H), 2.97 - 3.08 (m, 2H), 3.89 - 3.99 (m, 4H), 4.06 (br t, 2H), 5.60 (q, 1H), 5.85 (br s, 2H), 6.45 (t, 1H), 6.49 (s, 1H), 7.19 - 7.29 (m, 2H), 7.34 (t, 2H), 7.47 (d, 2H), 7.88 (d, 1H).
LC-MS (method 1): Rt = 0.97 min; MS (ESlpos): m/z = 434 [M+H]⁺

### Example 460

### (rac)-2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide

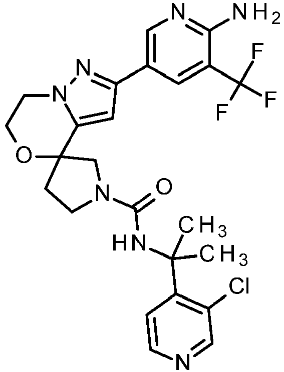

To half of the reaction mixture of 3-chloro-4-(2-isocyanatopropan-2-yl)pyridine (80.0 mg, 407 µmol) was added N,N-diisopropylethylamine (180 µL, 1.0 mmol) and (rac)-5-[6,7-dihydrospiro[pyrazolo[5,1-c][1,4]oxazine-4,3'-pyrrolidin]-2-yl]-3-(trifluoromethyl)pyridin-2-amine hydrochloride (127 mg, 339 µmol). The mixture was stirred at rt overnight under nitrogen atmosphere and then 5h at 60°C. Saturated potassium carbonate solution was added and stirred for 5 min. The layers were separated and the aqueous phase was extracted three times with dichloromethane (+10 % methanol). The combined organic phases were concentrated to give 240 mg of a residue which was purified by flash chromatography to afford 82.0 mg (90 % purity, 41 % yield) of the title compound.
LC-MS (Method 1): Rt = 1.02 min; MS (ESlpos): m/z = 536 [M+H]⁺
¹H-NMR (400 MHz, DMSO-d6) δ [ppm]: 1.559 (2.11), 1.648 (16.00), 1.663 (15.19), 2.203 (0.92), 2.227 (1.33), 2.253 (0.72), 2.306 (1.07), 2.323 (1.94), 2.326 (1.92), 2.331 (1.42), 2.354 (0.67), 2.522 (4.29), 2.664 (0.79), 2.669 (1.09), 2.673 (0.81), 3.374 (1.27), 3.393 (1.18), 3.443 (1.85), 3.472 (2.07), 3.542 (0.89), 3.563 (1.53), 3.586 (0.78), 3.747 (1.81), 3.775 (1.55), 4.054 (0.67), 4.070 (1.33), 4.085 (1.61), 4.108 (2.40), 4.120 (4.56), 4.133 (6.73), 6.355 (5.82), 6.601 (7.80), 6.748 (11.16), 7.422 (4.88), 7.436 (5.08), 8.013 (4.62), 8.018 (4.67), 8.397 (7.15), 8.410 (6.34), 8.427 (13.25), 8.582 (4.38), 8.586 (4.30).

### Example 461

### (rac)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide

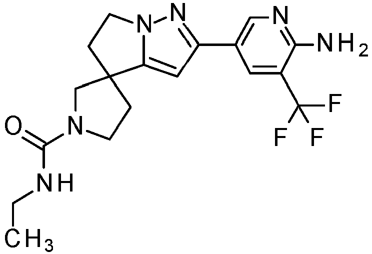

(*rac*)-1-(ethylcarbamoyl)-5',6'-dihydrospiro[pyrrolidine-3,4'-pyrrolo[1,2-b]pyrazol]-2'-yl trifluoromethanesulfonate (44.2 mg, 116 µmol) was solubilised in 1,4-dioxane (970 µL) under nitrogen, 5-(4,4,5,5-tetramethyl-1,3,2-dioxaborolan-2-yl)-3-(trifluoromethyl)pyridin-2-amine (50.0 mg, 174 µmol), K3PO4 (690 µL, 0.50 M, 350 µmol) and XPhos Pd G2 (45.5 mg, 57.9 µmol) were added. The mixture was stirred overnight at 100°C. It was diluted with ethyl acetate, washed with brine and the organic layer was dried over a silicone filter and concentrated under reduced pressure. The residue was purified by preparative HPLC to give 5.00 mg (95 % purity, 10 % yield) of the title compound.
LC-MS (Method 1): Rt = 0.88 min; MS (ESlneg): m/z = 393 [M-H]⁻
¹H NMR (400 MHz, DMSO-*d*₆) δ ppm 1.02 (t, 3 H) 1.99 - 2.12 (m, 2 H) 2.52 - 2.53 (m, 1 H) 2.99 - 3.09 (m, 2 H) 3.36 - 3.45 (m, 3 H) 3.46 - 3.55 (m, 1 H) 4.18 (t, 2 H) 6.15 (t, 1 H) 6.48 (s, 1 H) 6.54 (s, 2 H) 8.02 (d, 1 H) 8.59 (d, 1 H)

### Example 462

### (rac)-2-[6-amino-5-(trifluoromethyl)pyridin-3-yl]-N-ethyl-6,7-dihydro-5H-spiro[pyrazolo[1,5-a]pyridine-4,3'-pyrrolidine]-1'-carboxamide

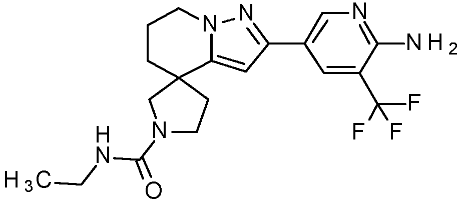

Example 462 was prepared in analogy to Example 461 using (*rac*)-1'-(ethylcarbamoyl)-6,7-dihydro-5H-spiro[pyrazolo[1,5-a]pyridine-4,3'-pyrrolidin]-2-yl trifluoromethanesulfonate (75.0 mg, 189 µmol) and 5-(4,4,5,5-tetramethyl-1,3,2-dioxaborolan-2-yl)-3-(trifluoromethyl)pyridin-2-amine (81.8 mg, 284 µmol).
LC-MS (Method 1): Rt = 0.93 min; MS (ESlpos): m/z = 409 [M+H]+
1H NMR (400 MHz, DMSO-d6) δ ppm 1.02 (t, 3 H) 1.69 - 1.85 (m, 2 H) 1.94 - 2.17 (m, 4 H) 3.01 - 3.10 (m, 2 H) 3.36 - 3.42 (m, 3 H) 3.44 - 3.56 (m, 1 H) 4.09 (t, 2 H) 6.14 (t, 1 H) 6.56 (d, 3 H) 8.00 (d, 1 H) 8.57 (s, 1 H)

### Example 463

### (rac)-2'-(6-amino-5-cyanopyridin-3-yl)-N-ethyl-5',6'-dihydrospiro[pyrrolidine-3,4'-pyrrolo[1,2-b]pyrazole]-1-carboxamide

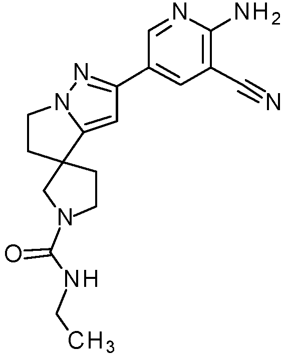

Example 463 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (78.0 mg, 204 µmol) and 2-amino-5-(4,4,5,5-tetramethyl-1,3,2-dioxaborolan-2-yl)pyridine-3-carbonitrile (100 mg, 408 µmol).
¹H NMR (400 MHz, DMSO-*d*₆) δ ppm 1.02 (t, 3 H) 2.05 (t, 2 H) 2.52 - 2.55 (m, 2 H) 3.00 - 3.10 (m, 2 H) 3.36 - 3.52 (m, 4 H) 4.18 (t, 2 H) 6.15 (t, 1 H) 6.45 (s, 1 H) 6.98 (s, 2 H) 8.15 (d, 1 H) 8.62 (d, 1 H)
LC-MS (method 1): Rt = 0.75 min; MS (ESlpos): m/z = 352 [M+H]⁺

### Example 464

### (rac)-2'-[6-amino-5-(trifluoromethoxy)pyridin-3-yl]-N-ethyl-5',6'-dihydrospiro[pyrrolidine-3,4'-pyrrolo[1,2-b]pyrazole]-1-carboxamide

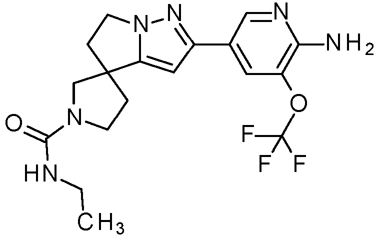

Example 464 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and [6-amino-5-(trifluoromethoxy)pyridin-3-yl]boronic acid (87.1 mg, 75 % purity, 294 µmol).
LC-MS (method 2): Rt = 0.84 min; MS (ESlpos): m/z = 412 [M+H]⁺
¹H-NMR (400 MHz, DMSO-d6) δ [ppm]: 1.000 (7.13), 1.018 (16.00), 1.036 (7.33), 1.156 (1.00), 1.232 (0.44), 2.028 (1.30), 2.034 (1.30), 2.042 (1.85), 2.052 (2.56), 2.069 (1.15), 2.082 (0.41), 2.323 (0.52), 2.327 (0.80), 2.332 (0.58), 2.518 (6.18), 2.523 (2.81), 2.533 (2.90), 2.539 (7.17), 2.665 (0.58), 2.669 (0.82), 2.673 (0.55), 3.017 (0.88), 3.035 (2.86), 3.049 (3.13), 3.053 (3.04), 3.067 (2.81), 3.084 (0.83), 3.357 (1.07), 3.375 (1.54), 3.383 (1.34), 3.397 (8.83), 3.419 (0.88), 3.464 (0.74), 3.482 (1.31), 3.488 (0.78), 3.496 (0.91), 3.507 (0.82), 3.521 (0.45), 4.155 (2.64), 4.173 (4.35), 4.190 (2.60), 5.759 (8.19), 6.138 (1.10), 6.152 (2.23), 6.165 (1.10), 6.422 (10.89), 6.494 (6.94), 7.754 (2.56), 7.758 (3.55), 7.762 (2.47), 8.138 (3.41), 8.331 (5.91), 8.336 (5.75).

### Example 465

### (rac)-2'-[6-amino-5-(difluoromethoxy)pyridin-3-yl]-N-ethyl-5',6'-dihydrospiro[pyrrolidine-3,4'-pyrrolo[1,2-b]pyrazole]-1-carboxamide

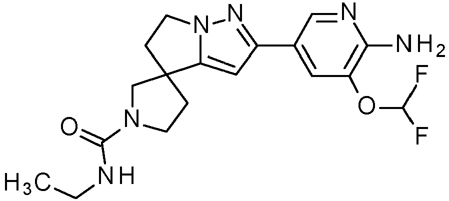

Example 465 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (574 mg, 1.50 mmol) and [6-amino-5-(difluoromethoxy)pyridin-3-yl]boronic acid (352 mg, 1.73 mmol).
1H-NMR (400MHz, DMSO-d6): δ [ppm]= 1.02 (t, 3H), 1.97 - 2.14 (m, 2H), 3.00 - 3.10 (m, 2H), 3.36 - 3.44 (m, 3H), 3.45 - 3.53 (m, 1H), 4.17 (t, 2H), 6.12 - 6.19 (m, 3H), 6.37 (s, 1H), 7.18 (t, 1H), 7.59 - 7.66 (m, 1H), 8.20 (d, 1H).
LC-MS (method 1): Rt = 0.82 min; MS (ESlpos): m/z = 392 [M]⁺

### Example 466

### (rac)-2'-[6-amino-5-(trifluoroacetyl)pyridin-3-yl]-N-ethyl-5',6'-dihydrospiro[pyrrolidine-3,4'-pyrrolo[1,2-b]pyrazole]-1-carboxamide

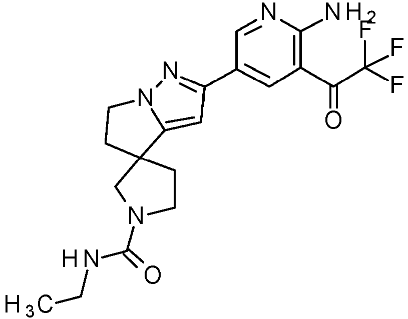

Example 466 was prepared in analogy to Example 461 using 1-[2-amino-5-(4,4,5,5-tetramethyl-1,3,2-dioxaborolan-2-yl)pyridin-3-yl]-2,2,2-trifluoroethan-1-one (43.0 mg, 136 µmol) and (rac)-1-(ethylcarbamoyl)-5',6'-dihydrospiro[pyrrolidine-3,4'-pyrrolo[1,2-b]pyrazol]-2'-yl trifluoromethanesulfonate (62.4 mg, 163 µmol).
LC-MS (method 1): Rt = 0.83 min; MS (ESlpos): m/z = 423 [M+H]⁺
¹H-NMR (400 MHz, DMSO-d6) δ [ppm]: 1.002 (6.91), 1.020 (16.00), 1.038 (7.39), 1.227 (1.00), 1.906 (1.12), 2.024 (0.51), 2.041 (1.27), 2.056 (2.24), 2.074 (2.67), 2.093 (1.10), 2.105 (0.66), 2.529 (4.25), 2.548 (2.86), 3.020 (0.89), 3.038 (2.92), 3.052 (3.32), 3.056 (3.24), 3.070 (2.94), 3.088 (0.91), 3.159 (1.41), 3.171 (1.44), 3.363 (1.10), 3.381 (1.39), 3.388 (1.26), 3.400 (1.32), 3.407 (2.76), 3.416 (10.90), 3.474 (0.80), 3.493 (1.30), 3.500 (0.93), 3.506 (1.07), 3.513 (0.85), 3.518 (0.79), 3.532 (0.50), 4.195 (2.67), 4.213 (4.43), 4.230 (2.68), 5.757 (1.99), 6.152 (1.19), 6.166 (2.37), 6.180 (1.21), 6.491 (10.51), 8.149 (4.21), 8.302 (2.25), 8.308 (3.22), 8.313 (2.12), 8.822 (5.27), 8.828 (5.24).

### Example 467

### 2'-(6-amino-5-{[1-phenylethyl]sulfanyl}pyridin-3-yl)-N-ethyl-5',6'-dihydrospiro[pyrrolidine-3,4'-pyrrolo[1,2-b]pyrazole]-1-carboxamide (diastereomeric mixture)

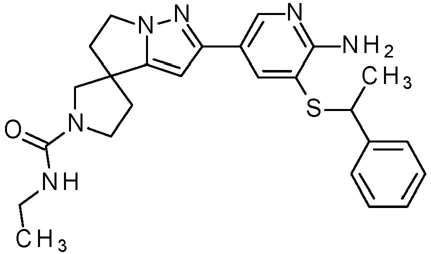

Example 467 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (62.5 mg, 65 % purity, 106 µmol) and (rac)-(6-amino-5-{[1-phenylethyl]sulfanyl}pyridin-3-yl)boronic acid (93.0 mg, 47 % purity, 159 µmol).

¹H-NMR (400MHz, DMSO-d6): δ [ppm]= 1.03 (t, 3H), 1.52 (d, 3H), 2.00 - 2.09 (m, 2H), 3.02 - 3.11 (m, 2H), 3.36 - 3.43 (m, 3H), 3.45 - 3.52 (m, 1H), 4.15 (t, 2H), 4.44 (qd, 1H), 6.14 - 6.24 (m, 4H), 7.18 - 7.24 (m, 1H), 7.25 - 7.31 (m, 2H), 7.31 - 7.36 (m, 2H), 7.64 (d, 1H, diastereomer 1), 7.65 (d, 1H, diastereomer 2), 8.28 (d, 1H).

### Example 468

### (rac)-2'-{6-amino-5-[(cyclohexylmethyl)sulfanyl]pyridin-3-yl}-N-ethyl-5',6'-dihydrospiro[pyrrolidine-3,4'-pyrrolo[1,2-b]pyrazole]-1-carboxamide

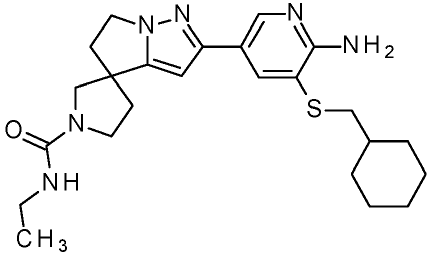

Example 468 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (194 mg, 60 % purity, 302 µmol) and {6-amino-5-[(cyclohexylmethyl)sulfanyl]pyridin-3-yl}boronic acid (214 mg, 45 % purity, 362 µmol).

¹H-NMR (500MHz, DMSO-d6): δ [ppm]= 0.93 - 1.04 (m, 5H), 1.09 - 1.21 (m, 3H), 1.35 - 1.44 (m, 1H), 1.55 - 1.62 (m, 1H), 1.63 - 1.70 (m, 2H), 1.82 (br d, 2H), 2.00 - 2.10 (m, 2H), 2.73 (d, 2H), 3.02 - 3.08 (m, 2H), 3.36 - 3.42 (m, 3H), 3.47 - 3.53 (m, 1H), 4.17 (t, 2H), 6.08 (s, 2H), 6.15 (t, 1H), 6.37 (s, 1H), 7.83 (d, 1H), 8.26 (d, 1H).

### Example 469

### (rac)-2'-[6-amino-5-(benzylsulfanyl)pyridin-3-yl]-N-ethyl-5',6'-dihydrospiro[pyrrolidine-3,4'-pyrrolo[1,2-b]pyrazole]-1-carboxamide

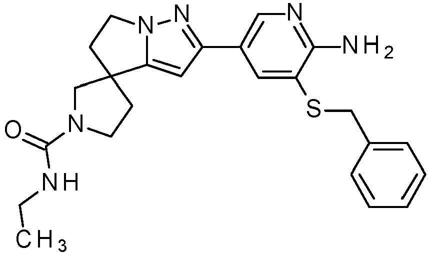

Example 469 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (83.5 mg, 92 % purity, 201 µmol) and 3-(benzylsulfanyl)-5-(4,4,5,5-tetramethyl-1,3,2-dioxaborolan-2-yl)pyridin-2-amine (220 mg, 38 % purity, 241 µmol).

¹H-NMR (400MHz, DMSO-d6): δ [ppm]= 1.02 (t, 3H), 1.98 - 2.10 (m, 2H), 3.01 - 3.10 (m, 2H), 3.36 - 3.43 (m, 3H), 3.45 - 3.53 (m, 1H), 4.07 (s, 2H), 4.15 (t, 2H), 6.11 - 6.18 (m, 3H), 6.25 (s, 1H), 7.18 - 7.24 (m, 1H), 7.25 - 7.28 (m, 4H), 7.68 (d, 1H), 8.26 (d, 1H).

### Example 470

### (rac)-2'-[6-amino-5-(2-hydroxypropan-2-yl)pyridin-3-yl]-N-ethyl-5',6'-dihydrospiro[pyrrolidine-3,4'-pyrrolo[1,2-b]pyrazole]-1-carboxamide

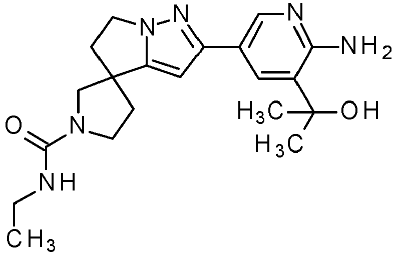

Example 470 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and 2-[2-amino-5-(4,4,5,5-tetramethyl-1,3,2-dioxaborolan-2-yl)pyridin-3-yl]propan-2-ol (115 mg, 76 % purity, 314 µmol).

¹H-NMR (400MHz, DMSO-d6): δ [ppm]= 1.02 (t, 3H), 1.51 (s, 6H), 1.98 - 2.12 (m, 2H), 3.01 - 3.10 (m, 2H), 3.36 - 3.43 (m, 3H), 3.46 - 3.54 (m, 1H), 4.16 (t, 2H), 5.45 (s, 1H), 6.12 - 6.19 (m, 3H), 6.33 (s, 1H), 7.64 (d, 1H), 8.21 (d, 1H).

### Example 471

### 2'-(6-amino-5-{[1-cyclohexylethyl]sulfanyl}pyridin-3-yl)-N-ethyl-5',6'-dihydrospiro[pyrrolidine-3,4'-pyrrolo[1,2-b]pyrazole]-1-carboxamide (diastereomeric mixture)

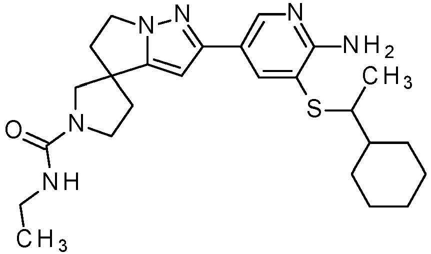

Example 471 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (70.3 mg, 92 % purity, 169 µmol) and (rac)-(6-amino-5-{[1-cyclohexylethyl]sulfanyl}pyridin-3-yl)boronic acid (141 mg, 40 % purity, 203 µmol).

¹H NMR (400 MHz, DMSO-d6) δ ppm 1.02 (t, 3 H) 1.06 - 1.25 (m, 8 H) 1.39 - 1.50 (m, 1 H) 1.57 - 1.65 (m, 1 H) 1.67 - 1.77 (m, 3 H) 1.78 - 1.85 (m, 1 H) 1.99 - 2.11 (m, 2 H) 3.01 - 3.14 (m, 3 H) 3.36 - 3.44 (m, 3 H) 3.45 - 3.54 (m, 1 H) 4.17 (br t, 2 H) 6.11 (s, 2 H) 6.15 (br t, 1 H) 6.37 (s, 1 H) 7.86 (d, 1 H) 8.31 (d, 1 H)

### Example 472

### 2'-{6-amino-5-[(1R)-1-(pyridin-3-yl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

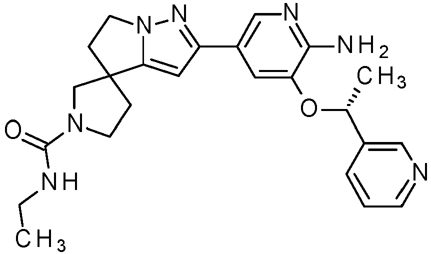

Example 472 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (95.3 mg, 92 % purity, 229 µmol) and {6-amino-5-[(1R)-1-(pyridin-3-yl)ethoxy]pyridin-3-yl}boronic acid (168 mg, 39 % purity, 252 µmol).

¹H-NMR (400MHz, DMSO-d6): δ [ppm]= 1.02 (2t, 3H), 1.60 (d, 3H), 1.97 - 2.08 (m, 2H), 3.00 - 3.10 (m, 2H), 3.36 - 3.43 (m, 3H), 3.44 - 3.53 (m, 1H), 4.13 (t, 2H), 5.71 (q, 1H), 5.89 (s, 2H), 6.15 (t, 1H), 6.27 (d, 1H), 7.32 (s, 1H), 7.37 (dd, 1H), 7.87 (t, 1H), 7.91 (dt, 1H), 8.47 ( dd, 1H), 8.71 (d, 1H).

### Example 473

### 2'-{6-amino-5-[(1S)-1-(pyridin-3-yl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

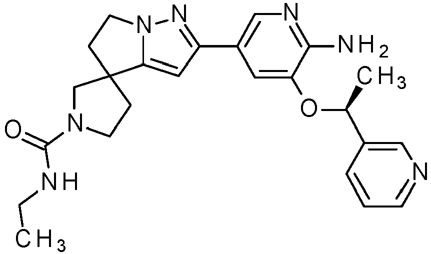

Example 473 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (138 mg, 92 % purity, 333 µmol) and {6-amino-5-[(1S)-1-(pyridin-3-yl)ethoxy]pyridin-3-yl}boronic acid (265 mg, 36 % purity, 366 µmol).

¹H-NMR (400MHz, DMSO-d6): δ [ppm]= 1.02 (2t, 3H), 1.60 (d, 3H), 1.96 - 2.10 (m, 2H), 3.01 - 3.09 (m, 2H), 3.36 - 3.43 (m, 3H), 3.44 - 3.52 (m, 1H), 4.13 (t, 2H), 5.71 (q, 1H), 5.89 (s, 2H), 6.15 (t, 1H), 6.27 (d, 1H), 7.32 (s, 1H), 7.37 (dd, 1H), 7.87 (t, 1H), 7.91 (dt, 1H), 8.47 (dd, 1H), 8.71 (d, 1H).

### Example 474

### (rac)-2'-{6-amino-5-[(cyclopentylmethyl)sulfanyl]pyridin-3-yl}-N-ethyl-5',6'-dihydrospiro[pyrrolidine-3,4'-pyrrolo[1,2-b]pyrazole]-1-carboxamide

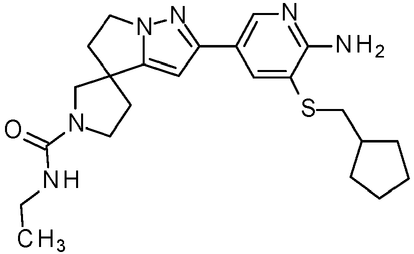

Example 474 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (93.3 mg, 92 % purity, 224 µmol) and 3-[(cyclopentylmethyl)sulfanyl]-5-(4,4,5,5-tetramethyl-1,3,2-dioxaborolan-2-yl)pyridin-2-amine (168 mg, 54 % purity, 269 µmol).

¹H-NMR (400MHz, DMSO-d6): δ [ppm]= 1.02 (t, 3H), 1.20 - 1.31 (m, 2H), 1.42 - 1.64 (m, 4H), 1.70 - 1.81 (m, 2H), 1.89 - 2.11 (m, 3H), 2.82 (d, 2H), 3.00 - 3.10 (m, 2H), 3.36 - 3.43 (m, 3H), 3.46 - 3.53 (m, 1H), 4.17 (t, 2H), 6.09 (s, 2H), 6.15 (t, 1H), 6.37 (s, 1H), 7.84 (d, 1H), 8.26 (d, 1H).

### Example 475

### (rac)-2'-(6-amino-5-nitropyridin-3-yl)-N-ethyl-5',6'-dihydrospiro[pyrrolidine-3,4'-pyrrolo[1,2-b]pyrazole]-1-carboxamide

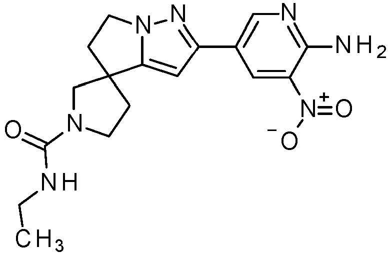

Example 475 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (96.2 mg, 251 µmol) and 3-nitro-5-(4,4,5,5-tetramethyl-1,3,2-dioxaborolan-2-yl)pyridin-2-amine (100 mg, 377 µmol).

¹H NMR (400 MHz, DMSO-*d*₆) δ ppm 1.02 (t, 3 H) 2.03 - 2.12 (m, 2 H) 2.53 - 2.57 (m, 2 H) 3.02 - 3.09 (m, 2 H) 3.37 - 3.54 (m, 4 H) 4.21 (t, 2 H) 6.16 (t, 1 H) 6.56 (s, 1 H) 8.00 (br s, 2 H) 8.61 (d, 1 H) 8.82 (d, 1 H)

### Example 476

### (rac)-2'-[6-amino-5-(benzyloxy)pyridin-3-yl]-N-ethyl-5',6'-dihydrospiro[pyrrolidine-3,4'-pyrrolo[1,2-b]pyrazole]-1-carboxamide

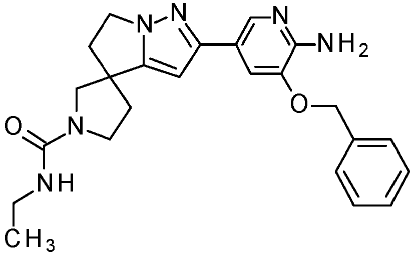

Example 476 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-(benzyloxy)-5-(4,4,5,5-tetramethyl-1,3,2-dioxaborolan-2-yl)pyridin-2-amine (96.0 mg, 294 µmol).
LC-MS (Method 1): Rt = 0.97 min; MS (ESlpos): m/z = 433 [M+H]+
¹H-NMR (400 MHz, DMSO-d6) δ [ppm]: 1.002 (7.23), 1.020 (16.00), 1.038 (7.32), 2.011 (0.42), 2.025 (1.36), 2.040 (2.16), 2.054 (2.82), 2.071 (1.18), 2.083 (0.52), 2.322 (0.66), 2.327 (0.88), 2.331 (0.66), 2.523 (3.16), 2.528 (3.28), 2.539 (1.48), 2.665 (0.67), 2.669 (0.91), 2.673 (0.69), 3.020 (1.00), 3.038 (3.09), 3.052 (3.45), 3.055 (3.36), 3.069 (2.97), 3.087 (0.87), 3.361 (0.69), 3.371 (0.61), 3.379 (1.62), 3.397 (10.81), 3.423 (1.08), 3.465 (0.81), 3.483 (1.41), 3.490 (0.87), 3.497 (1.02), 3.508 (0.85), 3.523 (0.48), 4.139 (2.80), 4.156 (4.68), 4.174 (2.70), 5.182 (11.01), 5.786 (7.12), 6.142 (1.27), 6.155 (2.52), 6.169 (1.24), 6.336 (10.81), 7.306 (0.90), 7.324 (2.97), 7.330 (0.96), 7.343 (2.37), 7.380 (3.87), 7.399 (6.57), 7.417 (3.21), 7.429 (4.75), 7.433 (4.69), 7.512 (5.71), 7.531 (4.35), 7.933 (5.97), 7.938 (5.83).

### Example 477

### (rac)-2'-(6-amino-5-methylpyridin-3-yl)-N-ethyl-5',6'-dihydrospiro[pyrrolidine-3,4'-pyrrolo[1,2-b]pyrazole]-1-carboxamide

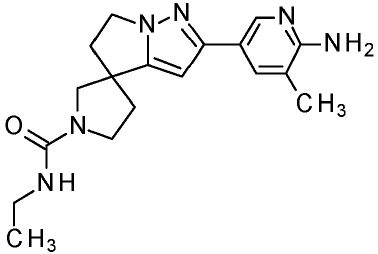

Example 477 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-methyl-5-(4,4,5,5-tetramethyl-1,3,2-dioxaborolan-2-yl)pyridin-2-amine (68.9 mg, 294 µmol).
LC-MS (Method 1): Rt = 0.73 min; MS (ESlpos): m/z = 341 [M+H]⁺
¹H-NMR (400 MHz, DMSO-d6) δ [ppm]: 1.000 (5.72), 1.018 (12.97), 1.035 (5.92), 2.005 (0.41), 2.022 (1.17), 2.036 (2.12), 2.062 (16.00), 2.331 (0.52), 2.518 (3.76), 2.522 (4.23), 2.539 (0.52), 2.673 (0.53), 3.017 (0.80), 3.034 (2.51), 3.048 (2.80), 3.052 (2.74), 3.066 (2.44), 3.084 (0.72), 3.365 (1.68), 3.372 (1.28), 3.391 (10.18), 3.408 (0.86), 3.461 (0.69), 3.480 (1.11), 3.487 (0.76), 3.493 (0.86), 3.500 (0.70), 3.506 (0.67), 3.519 (0.43), 4.131 (2.46), 4.149 (4.12), 4.166 (2.38), 5.755 (6.35), 6.136 (1.02), 6.149 (1.99), 6.163 (0.97), 6.283 (8.67), 7.589 (3.04), 7.592 (3.00), 8.172 (3.39), 8.177 (3.29).

### Example 478

### (rac)-2-(6-amino-5-nitropyridin-3-yl)-N-ethyl-6,7-dihydro-5H-spiro[pyrazolo[1,5-a]pyridine-4,3'-pyrrolidine]-1'-carboxamide

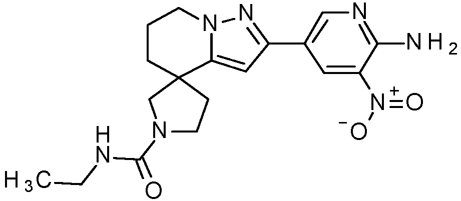

Example 478 was prepared in analogy to Example 461 using (*rac*)-1'-(ethylcarbamoyl)-6,7-dihydro-5H-spiro[pyrazolo[1,5-a]pyridine-4,3'-pyrrolidin]-2-yl trifluoromethanesulfonate (75.0 mg, 189 µmol) and 3-nitro-5-(4,4,5,5-tetramethyl-1,3,2-dioxaborolan-2-yl)pyridin-2-amine (75.2 mg, 284 µmol).

¹H NMR (400 MHz, DMSO-*d*₆) δ ppm 1.02 (t, 3 H) 1.79 (br d, 2 H) 1.95 - 2.08 (m, 3 H) 2.08 - 2.18 (m, 1 H) 3.02 - 3.09 (m, 2 H) 3.38 - 3.43 (m, 3 H) 3.49 (br dd, 1 H) 4.11 (t, 2 H) 6.14 (t, 1 H) 6.65 (s, 1 H) 8.01 (br s, 2 H) 8.60 (d, 1 H) 8.80 (d, 1 H)

### Example 479

### methyl 2-amino-5-[(rac)-1-(ethylcarbamoyl)-5',6'-dihydrospiro[pyrrolidine-3,4'-pyrrolo[1,2-b]pyrazol]-2'-yl]pyridine-3-carboxylate

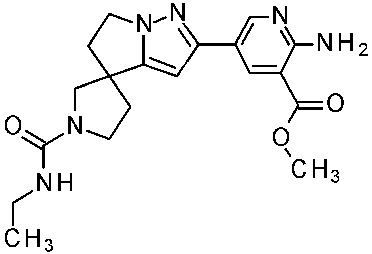

Example 479 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and methyl 2-amino-5-(4,4,5,5-tetramethyl-1,3,2-dioxaborolan-2-yl)pyridine-3-carboxylate (81.8 mg, 294 µmol).

¹H NMR (400 MHz, DMSO-*d*₆) δ ppm 1.02 (t, 3 H) 2.00 - 2.12 (m, 2 H) 2.52 (br s, 2 H) 3.05 (dd, 2 H) 3.40 (s, 3 H) 3.46 - 3.57 (m, 1 H) 3.84 (s, 3 H) 4.18 (t, 2 H) 6.16 (t, 1 H) 6.41 (s, 1 H) 7.24 (s, 2 H) 8.39 (d, 1 H) 8.61 (d, 1 H)

### Example 480

### (rac)-2'-(6-amino-5-phenylpyridin-3-yl)-N-ethyl-5',6'-dihydrospiro[pyrrolidine-3,4'-pyrrolo[1,2-b]pyrazole]-1-carboxamide

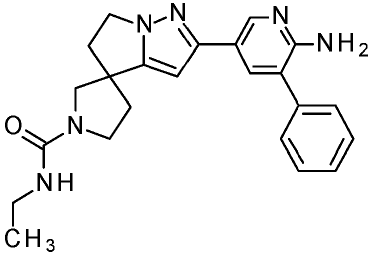

Example 480 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (179 mg, 467 µmol) and (6-amino-5-phenylpyridin-3-yl)boronic acid (150 mg, 701 µmol).

¹H NMR (600 MHz, DMSO-*d*₆) δ ppm 1.02 (t, 3 H) 1.99 - 2.11 (m, 2 H) 2.52 - 2.56 (m, 2 H) 3.02 - 3.09 (m, 2 H) 3.36 - 3.44 (m, 3 H) 3.50 (ddd, 1 H) 4.14 - 4.20 (m, 2 H) 5.67 (s, 2 H) 6.14 (t, 1 H) 6.39 (s, 1 H) 7.27 - 7.43 (m, 1 H) 7.46 - 7.52 (m, 4 H) 7.66 (d, 1 H) 8.36 (d, 1 H)

### Example 481

### (rac)-2'-[6-amino-5-(methylsulfanyl)pyridin-3-yl]-N-ethyl-5',6'-dihydrospiro[pyrrolidine-3,4'-pyrrolo[1,2-b]pyrazole]-1-carboxamide

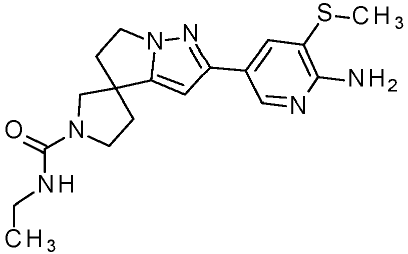

Example 481 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and [6-amino-5-(methylsulfanyl)pyridin-3-yl]boronic acid (54.1 mg, 294 µmol).
LC-MS (Method 1): Rt = 0.81 min; MS (ESlpos): m/z = 373 [M+H]⁺
1H-NMR (400 MHz, DMSO-d6) delta [ppm]: 0.798 (0.43), 0.822 (0.43), 0.905 (0.49), 1.001 (2.76), 1.019 (6.01), 1.035 (9.93), 1.053 (16.00), 1.066 (5.33), 1.070 (8.83), 1.261 (1.72), 1.751 (2.82), 1.864 (1.47), 2.055 (0.92), 2.344 (0.80), 2.411 (13.00), 2.518 (12.93), 2.523 (8.34), 2.564 (0.61), 3.036 (1.04), 3.050 (1.23), 3.067 (1.16), 3.378 (1.72), 3.384 (1.59), 3.392 (2.57), 3.399 (3.37), 3.404 (2.15), 3.417 (1.78), 3.422 (4.05), 3.435 (3.92), 3.439 (3.49), 3.452 (3.49), 3.457 (1.35), 3.469 (1.53), 3.938 (0.80), 4.149 (0.98), 4.168 (1.66), 4.185 (0.86), 4.343 (2.64), 4.355 (5.09), 4.368 (2.45), 4.451 (0.67), 6.001 (2.33), 6.150 (0.80), 6.180 (0.43), 6.380 (3.80), 7.754 (1.72), 7.760 (1.78), 8.220 (1.96), 8.226 (2.08)

### Example 482

### (rac)-2'-[6-amino-5-(4-methylphenyl)pyridin-3-yl]-N-ethyl-5',6'-dihydrospiro[pyrrolidine-3,4'-pyrrolo[1,2-b]pyrazole]-1-carboxamide

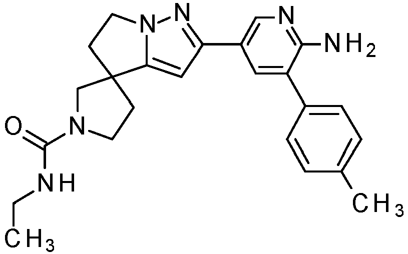

Example 482 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and 3-(4-methylphenyl)-5-(4,4,5,5-tetramethyl-1,3,2-dioxaborolan-2-yl)pyridin-2-amine (122 mg, 392 µmol).
LC-MS (Method 1): Rt = 1.00 min; MS (ESlpos): m/z = 417 [M+H]⁺
¹H-NMR (400 MHz, DMSO-d6) δ [ppm]: 0.851 (0.46), 0.995 (4.94), 1.013 (11.11), 1.031 (5.04), 1.232 (2.72), 1.900 (0.43), 2.027 (0.94), 2.040 (1.37), 2.054 (1.83), 2.072 (0.79), 2.084 (0.52), 2.331 (2.84), 2.336 (1.42), 2.358 (12.03), 2.518 (16.00), 2.522 (10.28), 2.539 (7.11), 2.669 (3.88), 2.673 (2.82), 3.012 (0.65), 3.030 (1.98), 3.044 (2.22), 3.048 (2.18), 3.062 (1.95), 3.080 (0.58), 3.367 (1.89), 3.395 (6.80), 3.465 (0.54), 3.483 (0.89), 3.497 (0.70), 4.144 (1.82), 4.162 (3.15), 4.179 (1.76), 5.636 (4.48), 6.134 (0.82), 6.148 (1.68), 6.162 (0.85), 6.382 (7.65), 7.276 (2.85), 7.296 (4.42), 7.361 (5.75), 7.381 (3.39), 7.622 (3.80), 7.627 (3.76), 8.334 (4.08), 8.340 (3.92).

### Example 483

### (rac)-2'-[6-amino-5-(2-methylphenyl)pyridin-3-yl]-N-ethyl-5',6'-dihydrospiro[pyrrolidine-3,4'-pyrrolo[1,2-b]pyrazole]-1-carboxamide

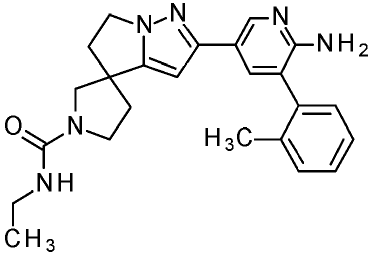

Example 483 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (150 mg, 392 µmol) and 3-(2-methylphenyl)-5-(4,4,5,5-tetramethyl-1,3,2-dioxaborolan-2-yl)pyridin-2-amine (200 mg, 645 µmol).
LC-MS (Method 1): Rt = 0.98 min; MS (ESlpos): m/z = 417 [M+H]+
¹H-NMR (400 MHz, DMSO-d6) δ [ppm]: 0.991 (0.87), 1.009 (1.93), 1.027 (0.91), 2.123 (3.06), 2.518 (0.64), 2.523 (0.72), 2.539 (16.00), 3.041 (0.45), 3.044 (0.44), 3.361 (0.47), 3.387 (1.33), 4.149 (0.66), 5.362 (0.91), 6.371 (1.46), 7.170 (0.42), 7.310 (0.50), 7.313 (0.55), 7.320 (0.57), 7.325 (0.64), 7.329 (0.54), 7.516 (0.73), 7.522 (0.74), 8.375 (0.78), 8.380 (0.77).

### Example 484

### (rac)-2'-[6-amino-5-(3-methylphenyl)pyridin-3-yl]-N-ethyl-5',6'-dihydrospiro[pyrrolidine-3,4'-pyrrolo[1,2-b]pyrazole]-1-carboxamide

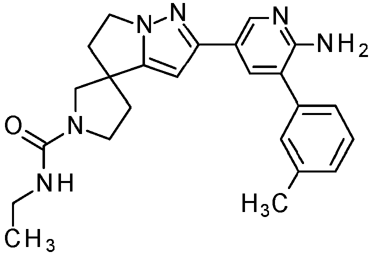

Example 484 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (121 mg, 316 µmol) and 3-(3-methylphenyl)-5-(4,4,5,5-tetramethyl-1,3,2-dioxaborolan-2-yl)pyridin-2-amine (147 mg, 474 µmol).
LC-MS (Method 1): Rt = 1.00 min; MS (ESlpos): m/z = 417 [M+H]⁺
¹H-NMR (400 MHz, DMSO-d6) δ [ppm]: 0.852 (0.44), 0.996 (5.69), 1.014 (12.60), 1.032 (5.77), 1.232 (1.46), 2.028 (1.15), 2.042 (1.69), 2.055 (2.22), 2.073 (0.97), 2.084 (0.45), 2.331 (2.22), 2.352 (2.76), 2.369 (16.00), 2.522 (9.23), 2.531 (3.83), 2.539 (5.86), 2.673 (2.17), 3.013 (0.79), 3.031 (2.40), 3.045 (2.72), 3.048 (2.64), 3.063 (2.39), 3.081 (0.70), 3.369 (2.30), 3.396 (8.46), 3.467 (0.70), 3.485 (1.15), 3.499 (0.87), 3.507 (0.76), 3.524 (0.43), 4.146 (2.25), 4.163 (3.85), 4.181 (2.14), 5.523 (0.57), 5.668 (5.62), 6.131 (1.01), 6.145 (2.04), 6.159 (1.00), 6.392 (8.45), 7.186 (1.98), 7.205 (2.25), 7.242 (0.59), 7.256 (1.55), 7.276 (2.47), 7.288 (3.70), 7.306 (0.45), 7.311 (0.48), 7.326 (0.48), 7.343 (2.62), 7.362 (3.43), 7.381 (1.32), 7.636 (4.42), 7.642 (4.56), 8.347 (4.76), 8.353 (4.63).

### Example 485

### (rac)-2'-(6-amino-5-methoxypyridin-3-yl)-N-ethyl-5',6'-dihydrospiro[pyrrolidine-3,4'-pyrrolo[1,2-b]pyrazole]-1-carboxamide

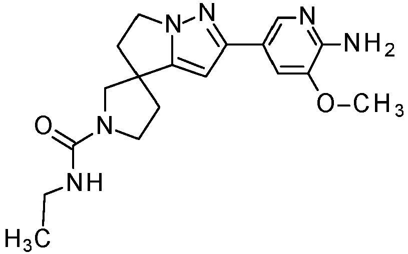

Example 485 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (200 mg, 523 µmol) and 3-methoxy-5-(4,4,5,5-tetramethyl-1,3,2-dioxaborolan-2-yl)pyridin-2-amine (196 mg, 785 µmol).
LC-MS (Method 1): Rt = 0.74 min; MS (ESlpos): m/z = 357 [M+H]⁺
¹H-NMR (400 MHz, DMSO-d6) δ [ppm]: 1.002 (4.43), 1.019 (10.22), 1.037 (4.51), 1.066 (0.55), 2.028 (0.75), 2.042 (1.18), 2.057 (1.61), 2.075 (0.67), 2.332 (0.61), 2.523 (2.55), 2.532 (1.91), 2.669 (0.86), 2.673 (0.61), 3.019 (0.57), 3.036 (1.78), 3.050 (1.95), 3.054 (1.89), 3.068 (1.72), 3.086 (0.50), 3.360 (0.53), 3.373 (0.49), 3.378 (0.93), 3.386 (0.86), 3.399 (5.67), 3.422 (0.54), 3.467 (0.46), 3.485 (0.79), 3.492 (0.48), 3.499 (0.58), 3.507 (0.46), 3.511 (0.46), 3.566 (0.65), 3.817 (16.00), 4.144 (1.69), 4.162 (2.76), 4.179 (1.63), 5.755 (4.26), 5.758 (3.59), 6.138 (0.69), 6.152 (1.36), 6.166 (0.67), 6.349 (7.02), 7.315 (2.75), 7.319 (2.77), 7.910 (3.67), 7.915 (3.55).

### Example 486

### (rac)-2'-[6-amino-5-(3-fluorophenyl)pyridin-3-yl]-N-ethyl-5',6'-dihydrospiro[pyrrolidine-3,4'-pyrrolo[1,2-b]pyrazole]-1-carboxamide

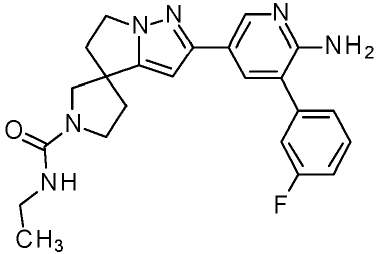

Example 486 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and [6-amino-5-(3-fluorophenyl)pyridin-3-yl]boronic acid (508 mg, 2.19 mmol).

¹H NMR (400 MHz, DMSO-*d*₆) δ ppm 1.01 (t, 3 H) 2.00 - 2.10 (m, 2 H) 2.52 - 2.55 (m, 2 H) 3.01-3.09 (m, 2 H) 3.36 - 3.52 (m, 4 H) 4.16 (t, 2 H) 5.81 (s, 2 H) 6.14 (t, 1 H) 6.41 (s, 1 H) 7.21 (t, 1 H) 7.29 - 7.32 (m, 1 H) 7.33 (s, 1 H) 7.51 (td, 1 H) 7.68 (d, 1 H) 8.38 (d, 1 H)

### Example 487

### (rac)-2'-(2-amino[3,3'-bipyridin]-5-yl)-N-ethyl-5',6'-dihydrospiro[pyrrolidine-3,4'-pyrrolo[1,2-b]pyrazole]-1-carboxamide

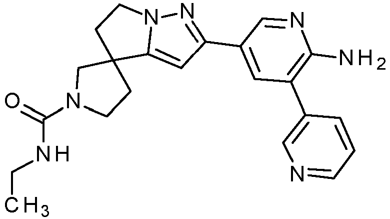

Example 487 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 5-(4,4,5,5-tetramethyl-1,3,2-dioxaborolan-2-yl)[3,3'-bipyridin]-2-amine (87.4 mg, 294 µmol).

¹H NMR (400 MHz, DMSO-*d*₆) δ ppm 0.99 - 1.07 (m, 3 H) 2.00 - 2.12 (m, 2 H) 2.51 - 2.54 (m, 2 H) 3.01 - 3.09 (m, 2 H) 3.35 - 3.52 (m, 4 H) 4.17 (t, 2 H) 5.87 (s, 2 H) 6.14 (t, 1 H) 6.42 (s, 1 H) 7.48 (ddd, 1 H) 7.69 (d, 1 H) 7.90 (dt, 1 H) 8.40 (d, 1 H) 8.58 (dd, 1 H) 8.64 - 8.68 (m, 1 H)

### Example 488

### (rac)-2'-[6-amino-5-(2-fluorophenyl)pyridin-3-yl]-N-ethyl-5',6'-dihydrospiro[pyrrolidine-3,4'-pyrrolo[1,2-b]pyrazole]-1-carboxamide

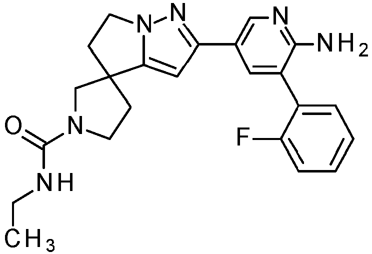

Example 488 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and [6-amino-5-(2-fluorophenyl)pyridin-3-yl]boronic acid (195 mg, 840 µmol). LC-MS (Method 1): Rt = 0.92 min; MS (ESlpos): m/z = 421 [M+H]⁺

¹H-NMR (400 MHz, DMSO-d6) δ [ppm]: 0.994 (6.69), 1.012 (16.00), 1.022 (0.73), 1.029 (7.08), 2.024 (1.24), 2.030 (1.25), 2.041 (1.75), 2.048 (2.41), 2.066 (1.08), 2.332 (0.69), 2.518 (4.52), 2.523 (3.10), 2.528 (2.80), 3.011 (0.82), 3.029 (2.59), 3.043 (2.94), 3.046 (2.77), 3.060 (2.58), 3.078 (0.75), 3.366 (1.73), 3.373 (1.16), 3.391 (8.39), 3.409 (0.84), 3.419 (0.69), 3.461 (0.66), 3.479 (1.20), 3.486 (0.73), 3.494 (0.85), 3.504 (0.76), 3.519 (0.44), 4.140 (2.44), 4.158 (4.06), 4.175 (2.35), 5.675 (6.04), 6.126 (1.09), 6.140 (2.19), 6.153 (1.09), 6.382 (10.76), 7.281 (1.24), 7.283 (1.93), 7.286 (1.50), 7.289 (1.44), 7.300 (2.70), 7.302 (3.19), 7.307 (2.08), 7.310 (2.24), 7.314 (1.61), 7.318 (2.36), 7.321 (2.07), 7.333 (1.64), 7.395 (1.21), 7.399 (1.69), 7.414 (2.10), 7.419 (2.34), 7.431 (1.32), 7.433 (1.45), 7.436 (1.34), 7.444 (1.16), 7.449 (1.50), 7.452 (1.18), 7.456 (1.02), 7.463 (0.95), 7.465 (1.12), 7.469 (1.28), 7.475 (0.59), 7.483 (0.60), 7.488 (0.52), 7.634 (3.90), 7.640 (3.93), 8.397 (5.77), 8.403 (5.66).

### Example 489

### 2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (diastereomeric mixture)

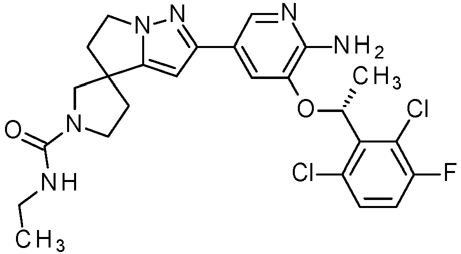

Example 489 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-[(1R)-1-(2,6-dichloro-3-fluorophenyl)ethoxy]-5-(4,4,5,5-tetramethyl-1,3,2-dioxaborolan-2-yl)pyridin-2-amine (126 mg, 294 µmol).
LC-MS (Method 1): Rt = 1.10 min; MS (ESlpos): m/z = 533 [M+H]+
¹H-NMR (400 MHz, DMSO-d6) δ [ppm]: 1.002 (0.64), 1.005 (0.67), 1.020 (1.39), 1.023 (1.43), 1.038 (0.67), 1.041 (0.67), 1.776 (1.58), 1.792 (1.59), 2.074 (0.61), 2.468 (0.47), 2.518 (0.73), 2.522 (0.50), 2.539 (16.00), 3.058 (0.41), 3.374 (1.41), 4.126 (0.66), 5.786 (0.96), 6.131 (1.20), 7.079 (0.65), 7.878 (0.88), 7.883 (0.87).

### Example 490

### (rac)-2-[6-amino-5-(2-fluorophenyl)pyridin-3-yl]-N-ethyl-6,7-dihydro-5H-spiro[pyrazolo[1,5-a]pyridine-4,3'-pyrrolidine]-1'-carboxamide

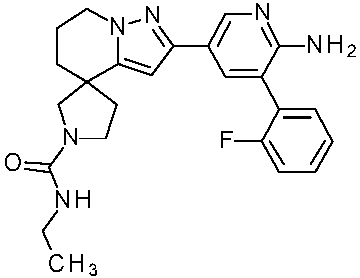

Example 490 was prepared in analogy to Example 461 using (*rac*)-1'-(ethylcarbamoyl)-6,7-dihydro-5H-spiro[pyrazolo[1,5-a]pyridine-4,3'-pyrrolidin]-2-yl trifluoromethanesulfonate (100 mg, 252 µmol) and [6-amino-5-(2-fluorophenyl)pyridin-3-yl]boronic acid (197 mg, 849 µmol). LC-MS (Method 1): Rt = 0.95 min; MS (ESlpos): m/z = 435 [M+H]⁺

¹H-NMR (400 MHz, DMSO-d6) δ [ppm]: 0.986 (7.18), 1.004 (16.00), 1.022 (7.74), 1.755 (2.43), 1.763 (2.59), 1.772 (2.68), 1.806 (0.52), 1.938 (0.77), 1.956 (1.27), 1.969 (2.00), 1.981 (2.22), 1.988 (2.21), 1.998 (2.06), 2.024 (1.45), 2.086 (0.84), 2.106 (1.60), 2.125 (1.04), 2.136 (1.20), 2.155 (0.62), 2.522 (5.98), 3.007 (1.00), 3.025 (3.22), 3.039 (3.87), 3.057 (3.29), 3.075 (1.00), 3.284 (0.43), 3.351 (2.97), 3.370 (10.34), 3.396 (1.60), 3.452 (0.84), 3.464 (1.05), 3.472 (1.29), 3.485 (1.18), 3.497 (0.76), 3.510 (0.54), 4.048 (2.57), 4.063 (4.99), 4.078 (2.36), 5.529 (0.79), 5.682 (7.55), 6.114 (1.43), 6.127 (2.76), 6.141 (1.42), 6.257 (0.49), 6.463 (9.25), 6.620 (0.46), 6.632 (0.51), 6.637 (0.51), 6.650 (0.50), 7.283 (2.65), 7.300 (4.29), 7.309 (3.09), 7.317 (3.11), 7.332 (2.04), 7.354 (0.41), 7.373 (0.59), 7.391 (1.68), 7.395 (1.85), 7.410 (2.52), 7.414 (2.78), 7.431 (2.10), 7.445 (1.42), 7.449 (1.90), 7.465 (1.43), 7.469 (1.48), 7.483 (0.69), 7.488 (0.57), 7.616 (4.78), 7.621 (4.80), 7.979 (0.48), 7.983 (0.48), 7.991 (0.47), 7.996 (0.42), 8.376 (5.87), 8.381 (5.71), 8.545 (1.49).

### Example 491

### (rac)-2'-[6-amino-5-(2-methoxyphenyl)pyridin-3-yl]-N-ethyl-5',6'-dihydrospiro[pyrrolidine-3,4'-pyrrolo[1,2-b]pyrazole]-1-carboxamide

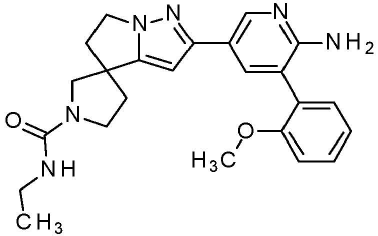

Example 491 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and [6-amino-5-(2-methoxyphenyl)pyridin-3-yl]boronic acid (337 mg, 1.38 mmol).

1H NMR (400 MHz, DMSO-d6) δ ppm 1.01 (t, 3 H) 1.18 - 1.36 (m, 1 H) 1.99 - 2.12 (m, 2 H) 2.52 - 2.55 (m, 1 H) 3.00 - 3.09 (m, 2 H) 3.35 - 3.52 (m, 4 H) 3.76 (s, 3 H) 4.15 (t, 2 H) 5.36 (s, 2 H) 6.14 (t, 1 H) 6.36 (s, 1 H) 7.04 (td, 1 H) 7.12 (d, 1 H) 7.20 (dd, 1 H) 7.40 (t, 1 H) 7.56 (d, 1 H) 8.34 (d, 1 H)

### Example 492

### (rac)-2'-(2-amino[3,4'-bipyridin]-5-yl)-N-ethyl-5',6'-dihydrospiro[pyrrolidine-3,4'-pyrrolo[1,2-b]pyrazole]-1-carboxamide

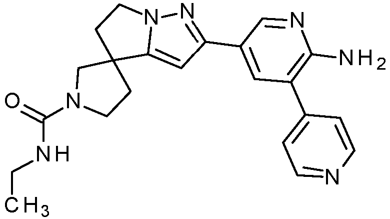

Example 492 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 5-(4,4,5,5-tetramethyl-1,3,2-dioxaborolan-2-yl)[3,4'-bipyridin]-2-amine (87.4 mg, 294 µmol).
LC-MS (Method 1): Rt = 0.73 min; MS (ESlpos): m/z = 404 [M+H]⁺
¹H-NMR (400 MHz, DMSO-d6) δ [ppm]: 0.997 (6.76), 1.015 (16.00), 1.024 (1.17), 1.033 (7.26), 1.042 (1.17), 1.060 (0.48), 1.156 (0.54), 1.174 (1.10), 1.192 (0.55), 1.232 (0.42), 2.033 (1.52), 2.051 (2.80), 2.063 (1.31), 2.069 (1.26), 2.084 (1.02), 2.518 (6.35), 2.522 (5.95), 2.539 (10.09), 3.014 (0.86), 3.032 (2.71), 3.046 (3.09), 3.049 (2.92), 3.064 (2.71), 3.081 (0.88), 3.109 (0.47), 3.370 (2.60), 3.379 (2.53), 3.387 (2.38), 3.396 (7.43), 3.399 (7.10), 3.424 (1.35), 3.441 (0.48), 3.463 (0.86), 3.480 (1.48), 3.488 (0.84), 3.495 (0.99), 3.505 (1.02), 3.521 (0.51), 4.152 (2.48), 4.170 (4.03), 4.187 (2.39), 6.057 (2.99), 6.134 (1.17), 6.148 (2.26), 6.162 (1.14), 6.430 (10.09), 7.536 (6.49), 7.540 (4.15), 7.547 (4.17), 7.552 (6.58), 7.580 (1.12), 7.584 (0.70), 7.591 (0.70), 7.595 (1.11), 7.758 (4.64), 7.763 (4.79), 7.772 (0.74), 7.778 (0.66), 8.345 (0.86), 8.352 (0.80), 8.418 (5.98), 8.424 (5.77), 8.639 (7.26), 8.642 (4.64), 8.649 (4.34), 8.653 (6.90).

### Example 493

### (rac)-2'-{6-amino-5-[3-(methanesulfonyl)phenyl]pyridin-3-yl}-N-ethyl-5',6'-dihydrospiro[pyrrolidine-3,4'-pyrrolo[1,2-b]pyrazole]-1-carboxamide

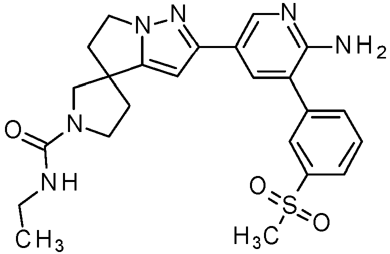

Example 493 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-[3-(methanesulfonyl)phenyl]-5-(4,4,5,5-tetramethyl-1,3,2-dioxaborolan-2-yl)pyridin-2-amine (472 mg, 1.26 mmol).
LC-MS (Method 1): Rt = 0.80 min; MS (ESlpos): m/z = 481 [M+H]⁺
¹H-NMR (500 MHz, DMSO-d6) δ [ppm]: 1.006 (4.29), 1.020 (8.95), 1.034 (4.42), 2.038 (0.85), 2.050 (1.57), 2.064 (1.86), 2.079 (0.74), 2.089 (0.41), 2.525 (3.42), 2.543 (12.61), 3.026 (0.60), 3.040 (1.87), 3.052 (2.16), 3.054 (2.14), 3.066 (1.83), 3.080 (0.58), 3.281 (16.00), 3.369 (0.42), 3.383 (1.28), 3.388 (0.85), 3.404 (5.40), 3.417 (0.80), 3.428 (0.46), 3.475 (0.50), 3.491 (0.83), 3.496 (0.60), 3.502 (0.66), 3.507 (0.58), 4.159 (1.77), 4.174 (3.00), 4.187 (1.73), 5.916 (4.49), 6.129 (0.82), 6.141 (1.61), 6.152 (0.81), 6.408 (5.88), 7.730 (3.84), 7.734 (3.28), 7.744 (2.76), 7.760 (1.74), 7.840 (1.77), 7.856 (1.33), 7.913 (1.63), 7.929 (1.40), 7.989 (1.94), 7.992 (3.09), 8.413 (3.33), 8.417 (3.30).

### Example 494

### (rac)-2'-[6-amino-5-(phenylsulfanyl)pyridin-3-yl]-N-ethyl-5',6'-dihydrospiro[pyrrolidine-3,4'-pyrrolo[1,2-b]pyrazole]-1-carboxamide

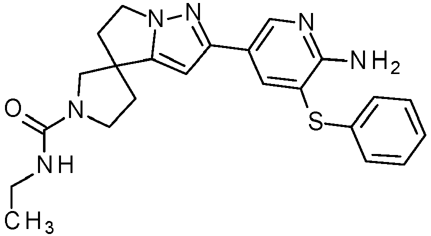

Example 494 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (600 mg, 1.57 mmol) and [6-amino-5-(phenylsulfanyl)pyridin-3-yl]boronic acid (1.65 g, 6.72 mmol).
LC-MS (Method 1): Rt = 1.03 min; MS (ESlpos): m/z = 435 [M+H]⁺
¹H-NMR (400 MHz, DMSO-d6) δ [ppm]: 0.995 (5.69), 1.004 (2.25), 1.013 (13.09), 1.031 (5.95), 2.044 (1.98), 2.518 (16.00), 2.523 (11.50), 3.030 (2.51), 3.044 (2.64), 3.062 (2.12), 3.365 (2.38), 3.390 (7.54), 3.478 (1.06), 4.136 (2.25), 4.153 (3.57), 4.170 (1.98), 5.194 (0.93), 5.758 (9.92), 6.145 (1.98), 6.158 (0.93), 6.264 (5.16), 6.397 (9.26), 7.158 (3.83), 7.176 (5.16), 7.179 (4.36), 7.203 (2.64), 7.222 (1.85), 7.295 (3.83), 7.314 (4.89), 7.332 (2.25), 7.969 (4.50), 7.974 (4.50), 8.440 (4.89), 8.446 (4.63).

### Example 495

### (rac)-2'-{6-amino-5-[4-(methanesulfonyl)phenyl]pyridin-3-yl}-N-ethyl-5',6'-dihydrospiro[pyrrolidine-3,4'-pyrrolo[1,2-b]pyrazole]-1-carboxamide

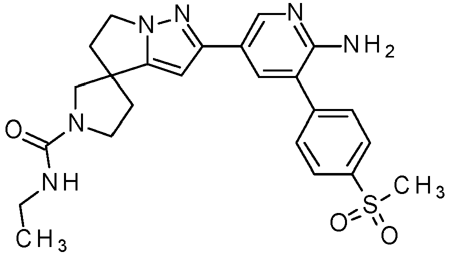

Example 495 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (150 mg, 392 µmol) and {6-amino-5-[4-(methanesulfonyl)phenyl]pyridin-3-yl}boronic acid (491 mg, 1.68 mmol).
LC-MS (Method 1): Rt = 0.78 min; MS (ESlpos): m/z = 481 [M+H]⁺
¹H-NMR (500 MHz, DMSO-d6) δ [ppm]: 1.005 (3.55), 1.013 (0.77), 1.019 (8.14), 1.027 (0.44), 1.034 (3.58), 1.069 (1.85), 2.042 (0.79), 2.056 (1.70), 2.070 (0.89), 2.518 (1.56), 2.522 (1.50), 2.525 (1.21), 2.536 (1.26), 2.543 (16.00), 2.550 (1.78), 2.564 (1.21), 2.642 (0.40), 3.040 (0.98), 3.051 (1.14), 3.065 (0.93), 3.170 (1.01), 3.381 (0.67), 3.400 (2.21), 3.411 (2.26), 3.422 (0.45), 3.429 (1.05), 3.442 (0.43), 3.458 (0.44), 3.471 (0.80), 3.477 (0.41), 3.484 (0.50), 3.491 (0.52), 4.184 (1.16), 4.198 (1.80), 4.212 (1.14), 6.142 (0.41), 6.153 (0.72), 6.553 (3.19), 7.792 (0.45), 7.796 (3.26), 7.800 (1.05), 7.809 (1.13), 7.813 (3.61), 7.817 (0.53), 8.063 (3.56), 8.067 (1.09), 8.076 (1.05), 8.080 (2.93), 8.096 (0.84), 8.313 (0.80), 8.401 (2.41), 8.405 (2.38).

### Example 496

### (rac)-2'-[5-(4-acetylphenyl)-6-aminopyridin-3-yl]-N-ethyl-5',6'-dihydrospiro[pyrrolidine-3,4'-pyrrolo[1,2-b]pyrazole]-1-carboxamide

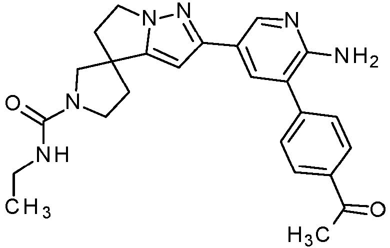

Example 496 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (200 mg, 523 µmol) and [5-(4-acetylphenyl)-6-aminopyridin-3-yl]boronic acid (574 mg, 2.24 mmol).
LC-MS (Method 1): Rt = 0.88 min; MS (ESlpos): m/z = 445 [M+H]⁺
¹H-NMR (400 MHz, DMSO-d6) δ [ppm]: 0.996 (1.01), 1.014 (2.20), 1.032 (1.01), 1.066 (0.49), 1.156 (1.43), 2.055 (0.38), 2.074 (0.35), 2.518 (1.72), 2.522 (1.04), 2.534 (0.50), 2.539 (0.41), 2.609 (16.00), 2.620 (4.67), 3.031 (0.40), 3.046 (0.48), 3.049 (0.45), 3.064 (0.40), 3.399 (1.39), 4.150 (0.37), 4.168 (0.61), 4.185 (0.35), 5.709 (2.12), 5.844 (0.88), 6.145 (0.35), 6.409 (1.48), 6.542 (0.46), 6.666 (1.38), 6.679 (1.47), 6.684 (1.43), 6.697 (1.48), 7.367 (1.30), 7.372 (1.34), 7.386 (1.27), 7.390 (1.21), 7.594 (3.23), 7.598 (1.13), 7.610 (1.27), 7.615 (3.41), 7.645 (0.97), 7.666 (1.03), 7.705 (0.75), 7.711 (0.77), 7.979 (1.35), 7.984 (1.42), 7.991 (1.41), 7.996 (1.30), 8.021 (3.60), 8.026 (1.20), 8.038 (2.19), 8.042 (3.38), 8.059 (0.93), 8.396 (0.82), 8.402 (0.80).

### Example 497

### (rac)-2'-{6-amino-5-[4-(methanesulfinyl)phenyl]pyridin-3-yl}-N-ethyl-5',6'-dihydrospiro[pyrrolidine-3,4'-pyrrolo[1,2-b]pyrazole]-1-carboxamide

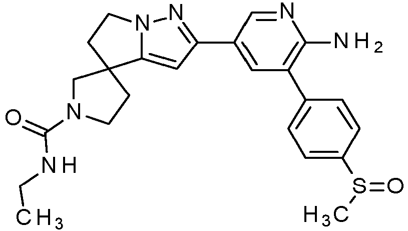

Example 497 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (200 mg, 523 µmol) and {6-amino-5-[4-(methanesulfinyl)phenyl]pyridin-3-yl}boronic acid (619 mg, 2.24 mmol).
LC-MS (Method 1): Rt = 0.74 min; MS (ESlpos): m/z = 465 [M+H]⁺
¹H-NMR (400 MHz, DMSO-d6) δ [ppm]: 0.996 (3.64), 1.014 (8.65), 1.032 (3.85), 2.034 (0.73), 2.052 (1.31), 2.071 (0.58), 2.074 (0.65), 2.518 (4.44), 2.523 (2.44), 2.535 (1.45), 2.539 (0.80), 2.787 (1.64), 2.793 (16.00), 3.013 (0.44), 3.031 (1.38), 3.045 (1.56), 3.048 (1.53), 3.063 (1.38), 3.373 (0.98), 3.382 (0.84), 3.398 (4.11), 3.417 (0.40), 3.482 (0.65), 3.489 (0.40), 3.497 (0.47), 3.508 (0.44), 4.149 (1.31), 4.166 (2.07), 4.183 (1.20), 5.821 (2.91), 6.131 (0.58), 6.145 (1.20), 6.159 (0.62), 6.408 (5.67), 7.682 (2.80), 7.686 (1.16), 7.695 (2.98), 7.703 (5.13), 7.762 (5.05), 7.767 (1.38), 7.778 (1.09), 7.784 (2.84), 8.390 (3.09), 8.396 (2.98).

### Example 498

### (rac)-2'-[6-amino-5-(3-methoxyphenyl)pyridin-3-yl]-N-ethyl-5',6'-dihydrospiro[pyrrolidine-3,4'-pyrrolo[1,2-b]pyrazole]-1-carboxamide

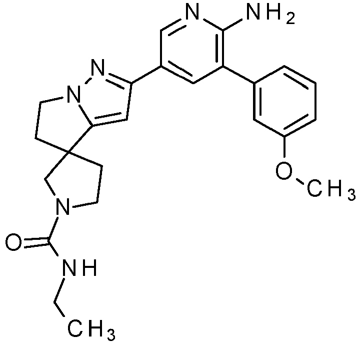

Example 498 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (170 mg, 445 µmol) and [6-amino-5-(3-methoxyphenyl)pyridin-3-yl]boronic acid (163 mg, 667 µmol).
LC-MS (Method 1): Rt = 0.90 min; MS (ESlpos): m/z = 433 [M+H]⁺
¹H-NMR (400 MHz, DMSO-d6) δ [ppm]: 0.995 (3.30), 1.013 (7.93), 1.031 (3.49), 1.064 (0.85), 2.028 (0.57), 2.041 (0.80), 2.054 (1.13), 2.071 (0.47), 2.518 (2.45), 2.522 (1.46), 2.530 (1.23), 3.031 (1.23), 3.044 (1.42), 3.048 (1.32), 3.062 (1.23), 3.368 (1.32), 3.375 (0.80), 3.395 (4.06), 3.412 (0.47), 3.485 (0.57), 3.499 (0.42), 3.803 (16.00), 4.146 (1.13), 4.163 (1.94), 4.181 (1.09), 5.700 (2.78), 6.133 (0.52), 6.147 (1.04), 6.161 (0.52), 6.396 (5.10), 6.934 (0.66), 6.937 (0.80), 6.941 (0.80), 6.943 (0.90), 6.955 (0.76), 6.957 (0.80), 6.961 (0.99), 6.964 (0.94), 7.006 (1.13), 7.010 (1.70), 7.016 (1.37), 7.020 (1.09), 7.023 (1.23), 7.039 (0.99), 7.041 (1.13), 7.045 (0.76), 7.370 (1.27), 7.389 (1.70), 7.409 (0.99), 7.654 (2.55), 7.660 (2.60), 8.353 (3.07), 8.359 (3.02).

### Example 499

### 2'-{6-amino-5-[(1R)-1-(2,6-dichloro-3-fluorophenyl)ethoxy]pyridin-3-yl}-N-ethyl-5',6'-dihydrospiro[azetidine-3,4'-pyrrolo[1,2-b]pyrazole]-1-carboxamide

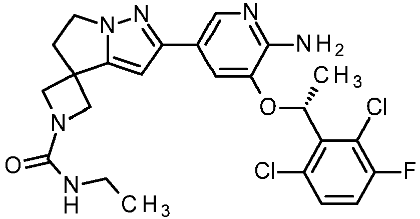

Example 499 was prepared in analogy to Example 461 using 1-(ethylcarbamoyl)-5',6'-dihydrospiro[azetidine-3,4'-pyrrolo[1,2-b]pyrazol]-2'-yl trifluoromethanesulfonate (75.0 mg, 204 µmol) and 3-[(1R)-1-(2,6-dichloro-3-fluorophenyl)ethoxy]-5-(4,4,5,5-tetramethyl-1,3,2-dioxaborolan-2-yl)pyridin-2-amine (104 mg, 244 µmol).
LC-MS (Method 1): Rt = 1.10 min; MS (ESlpos): m/z = 519 [M+H]⁺
¹H-NMR (400 MHz, DMSO-d6) δ [ppm]: 0.992 (7.03), 1.009 (15.84), 1.027 (7.26), 1.052 (0.70), 1.065 (16.00), 1.154 (0.92), 1.172 (1.67), 1.189 (0.92), 1.231 (2.19), 1.774 (10.41), 1.791 (10.31), 1.810 (1.52), 1.987 (3.00), 2.331 (0.72), 2.518 (4.09), 2.522 (2.56), 2.539 (1.78), 2.673 (0.72), 2.782 (2.43), 2.800 (4.11), 2.817 (2.63), 2.994 (0.97), 3.012 (3.03), 3.026 (3.35), 3.030 (3.31), 3.044 (2.96), 3.062 (0.90), 3.165 (4.09), 3.920 (1.00), 3.927 (1.07), 3.947 (10.63), 3.964 (1.27), 3.970 (1.00), 4.016 (0.78), 4.034 (0.78), 4.052 (2.82), 4.070 (4.18), 4.087 (2.57), 5.758 (1.78), 5.784 (5.97), 6.036 (0.65), 6.053 (2.47), 6.070 (2.47), 6.086 (0.67), 6.413 (11.47), 6.433 (1.41), 6.447 (2.89), 6.461 (1.39), 7.136 (4.30), 7.140 (4.32), 7.413 (2.06), 7.435 (4.26), 7.457 (2.94), 7.534 (2.33), 7.547 (2.43), 7.557 (1.81), 7.569 (1.69), 7.908 (5.66), 7.913 (5.62).

### Example 500

### (rac)-2'-{6-amino-5-[4-(cyclopropanesulfonyl)phenyl]pyridin-3-yl}-N-ethyl-5',6'-dihydrospiro[pyrrolidine-3,4'-pyrrolo[1,2-b]pyrazole]-1-carboxamide

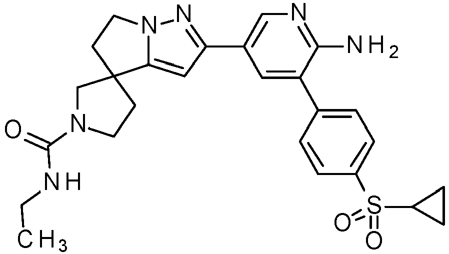

Example 500 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and {6-amino-5-[4-(cyclopropanesulfonyl)phenyl]pyridin-3-yl}boronic acid (125 mg, 392 µmol).
LC-MS (Method 1): Rt = 0.88 min; MS (ESlpos): m/z = 507 [M+H]⁺
¹H-NMR (400 MHz, DMSO-d6) δ [ppm]: 0.996 (6.70), 1.014 (16.00), 1.032 (7.19), 1.044 (0.65), 1.060 (2.71), 1.066 (3.99), 1.074 (1.98), 1.080 (2.58), 1.085 (2.60), 1.097 (1.09), 1.105 (0.41), 1.112 (0.45), 1.118 (0.42), 1.131 (0.47), 1.143 (1.41), 1.152 (3.46), 1.156 (3.15), 1.163 (3.42), 1.169 (2.56), 1.182 (0.68), 2.031 (1.41), 2.050 (2.60), 2.062 (1.18), 2.068 (1.15), 2.518 (6.02), 2.537 (2.30), 2.860 (0.60), 2.872 (1.25), 2.879 (1.22), 2.884 (0.88), 2.892 (2.22), 2.898 (0.80), 2.903 (1.18), 2.911 (1.09), 2.923 (0.54), 3.013 (0.78), 3.031 (2.48), 3.045 (2.79), 3.048 (2.66), 3.063 (2.45), 3.081 (0.70), 3.362 (0.76), 3.370 (0.80), 3.377 (1.41), 3.395 (6.22), 3.398 (5.96), 3.423 (0.91), 3.463 (0.65), 3.480 (1.25), 3.488 (0.71), 3.495 (0.83), 3.505 (0.83), 3.520 (0.41), 4.150 (2.19), 4.168 (3.60), 4.185 (2.13), 5.794 (0.81), 5.933 (5.21), 6.130 (1.05), 6.143 (2.19), 6.157 (1.07), 6.420 (10.56), 6.671 (0.58), 6.683 (0.65), 6.689 (0.60), 6.701 (0.65), 7.390 (0.49), 7.395 (0.52), 7.409 (0.47), 7.414 (0.49), 7.708 (1.25), 7.713 (0.42), 7.726 (5.23), 7.732 (5.27), 7.763 (6.07), 7.767 (2.06), 7.779 (2.22), 7.784 (7.30), 7.932 (1.53), 7.938 (0.60), 7.948 (8.08), 7.953 (2.95), 7.964 (2.08), 7.969 (6.04), 7.991 (0.65), 7.996 (0.62), 8.003 (0.62), 8.008 (0.57), 8.414 (5.94), 8.420 (5.97).

### Example 501

### (rac)-2'-[6-amino-5-(4-sulfamoylphenyl)pyridin-3-yl]-N-ethyl-5',6'-dihydrospiro[pyrrolidine-3,4'-pyrrolo[1,2-b]pyrazole]-1-carboxamide

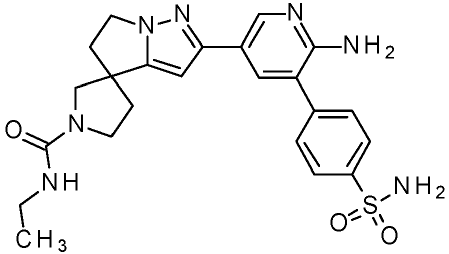

Example 501 was prepared in analogy to Example 461 using (r*ac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and [6-amino-5-(4-sulfamoylphenyl)pyridin-3-yl]boronic acid (115 mg, 392 µmol).
LC-MS (Method 1): Rt = 0.68 min; MS (ESlpos): m/z = 482 [M+H]⁺
¹H-NMR (400 MHz, DMSO-d6) δ [ppm]: 0.997 (6.85), 1.015 (16.00), 1.033 (7.21), 2.034 (1.33), 2.053 (2.46), 2.062 (1.21), 2.071 (1.14), 2.074 (1.05), 2.083 (0.49), 2.518 (5.50), 2.535 (2.60), 3.014 (0.81), 3.032 (2.63), 3.046 (2.93), 3.050 (2.86), 3.064 (2.60), 3.081 (0.77), 3.356 (0.90), 3.375 (1.46), 3.382 (1.21), 3.400 (7.20), 3.418 (0.83), 3.425 (0.70), 3.466 (0.68), 3.483 (1.25), 3.490 (0.77), 3.497 (0.87), 3.508 (0.81), 3.523 (0.45), 4.150 (2.22), 4.168 (3.81), 4.185 (2.19), 5.813 (0.69), 5.859 (5.22), 6.131 (1.11), 6.145 (2.27), 6.159 (1.11), 6.411 (9.68), 7.411 (2.20), 7.679 (6.19), 7.683 (2.30), 7.697 (6.87), 7.700 (8.92), 7.720 (0.81), 7.742 (0.95), 7.879 (1.47), 7.884 (7.76), 7.889 (2.68), 7.901 (2.24), 7.905 (6.09), 8.301 (0.63), 8.307 (0.62), 8.397 (5.57), 8.402 (5.62).

### Example 502

### (rac)-2'-{6-amino-5-[4-(methylsulfamoyl)phenyl]pyridin-3-yl}-N-ethyl-5',6'-dihydrospiro[pyrrolidine-3,4'-pyrrolo[1,2-b]pyrazole]-1-carboxamide

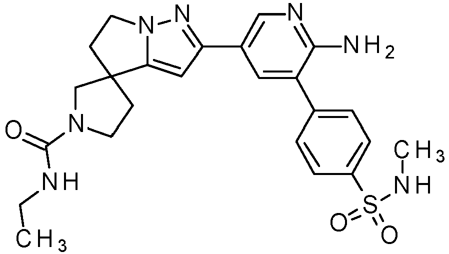

Example 502 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and {6-amino-5-[4-(methylsulfamoyl)phenyl]pyridin-3-yl}boronic acid (120 mg, 392 µmol).
LC-MS (Method 1): Rt = 0.78 min; MS (ESlpos): m/z = 496 [M+H]⁺
¹H-NMR (400 MHz, DMSO-d6) δ [ppm]: 0.996 (6.89), 1.014 (16.00), 1.032 (7.45), 1.137 (0.44), 1.232 (0.84), 1.256 (0.88), 1.296 (0.42), 2.031 (1.65), 2.050 (3.06), 2.061 (1.58), 2.067 (1.51), 2.458 (7.49), 2.468 (8.16), 2.536 (5.59), 2.539 (8.32), 3.013 (0.98), 3.031 (2.88), 3.045 (3.32), 3.049 (3.31), 3.063 (2.99), 3.081 (1.11), 3.359 (3.31), 3.369 (3.64), 3.376 (4.10), 3.398 (9.55), 3.419 (3.01), 3.462 (1.83), 3.480 (2.34), 3.488 (1.72), 3.495 (1.79), 3.506 (1.72), 3.521 (1.09), 4.148 (2.46), 4.166 (4.25), 4.183 (2.58), 5.901 (5.73), 6.133 (1.20), 6.146 (2.46), 6.159 (1.30), 6.414 (10.65), 7.524 (1.21), 7.535 (1.27), 7.711 (7.53), 7.717 (6.05), 7.730 (8.28), 7.830 (8.44), 7.852 (5.89), 8.403 (4.20), 8.408 (4.31).

### Example 503

### (rac)-2'-{6-amino-5-[4-(dimethylsulfamoyl)phenyl]pyridin-3-yl}-N-ethyl-5',6'-dihydrospiro[pyrrolidine-3,4'-pyrrolo[1,2-b]pyrazole]-1-carboxamide

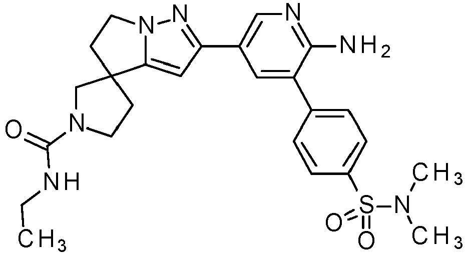

Example 503 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and {6-amino-5-[4-(dimethylsulfamoyl)phenyl]pyridin-3-yl}boronic acid (126 mg, 392 µmol).
LC-MS (Method 1): Rt = 0.91 min; MS (ESlpos): m/z = 510 [M+H]⁺
¹H-NMR (400 MHz, DMSO-d6) δ [ppm]: 0.996 (1.12), 1.014 (2.51), 1.031 (1.14), 2.032 (0.25), 2.051 (0.44), 2.062 (0.22), 2.069 (0.20), 2.518 (1.10), 2.539 (16.00), 2.657 (9.92), 3.031 (0.44), 3.045 (0.50), 3.048 (0.49), 3.063 (0.44), 3.370 (0.17), 3.378 (0.25), 3.396 (1.23), 3.423 (0.17), 3.480 (0.23), 4.147 (0.40), 4.165 (0.67), 4.182 (0.39), 5.934 (0.95), 6.132 (0.19), 6.146 (0.39), 6.159 (0.19), 6.415 (1.61), 7.722 (0.80), 7.727 (0.83), 7.739 (0.69), 7.744 (0.30), 7.760 (1.46), 7.796 (1.57), 7.817 (0.70), 8.410 (0.92), 8.416 (0.89).

### Example 504

### (rac)-2'-[6-amino-5-(cyclohexylmethoxy)pyridin-3-yl]-N-ethyl-5',6'-dihydrospiro[pyrrolidine-3,4'-pyrrolo[1,2-b]pyrazole]-1-carboxamide

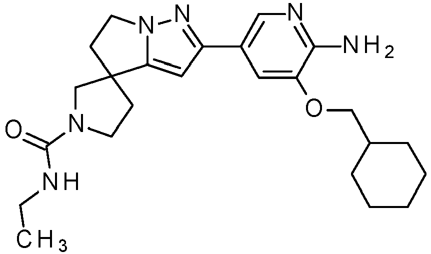

Example 504 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (60.0 mg, 157 µmol) and [6-amino-5-(cyclohexylmethoxy)pyridin-3-yl]boronic acid (58.9 mg, 235 µmol).
LC-MS (Method 1): Rt = 1.12 min; MS (ESlpos): m/z = 439 [M+H]⁺
¹H-NMR (400 MHz, DMSO-d6) δ [ppm]: 1.000 (6.94), 1.018 (16.00), 1.036 (7.44), 1.056 (1.46), 1.085 (1.74), 1.114 (0.86), 1.170 (0.61), 1.200 (0.96), 1.222 (2.20), 1.250 (1.79), 1.281 (1.19), 1.643 (0.81), 1.671 (1.01), 1.707 (1.97), 1.738 (1.72), 1.771 (0.86), 1.781 (0.71), 1.845 (1.84), 1.874 (1.74), 2.024 (1.21), 2.038 (1.92), 2.053 (2.57), 2.071 (1.06), 2.083 (0.50), 2.518 (6.69), 2.523 (4.49), 2.527 (3.26), 3.017 (0.91), 3.035 (2.80), 3.050 (3.10), 3.053 (3.00), 3.067 (2.73), 3.084 (0.81), 3.357 (0.68), 3.376 (1.41), 3.395 (8.88), 3.421 (0.91), 3.466 (0.73), 3.484 (1.24), 3.498 (0.93), 3.524 (0.43), 3.795 (5.27), 3.811 (5.05), 4.137 (2.52), 4.155 (4.29), 4.172 (2.50), 5.705 (4.11), 6.138 (1.14), 6.152 (2.25), 6.165 (1.11), 6.347 (10.45), 7.282 (4.14), 7.287 (4.11), 7.898 (5.65), 7.902 (5.50).

### Example 505

### (rac)-2'-{6-amino-5-[(pyridin-3-yl)methoxy]pyridin-3-yl}-N-ethyl-5',6'-dihydrospiro[pyrrolidine-3,4'-pyrrolo[1,2-b]pyrazole]-1-carboxamide

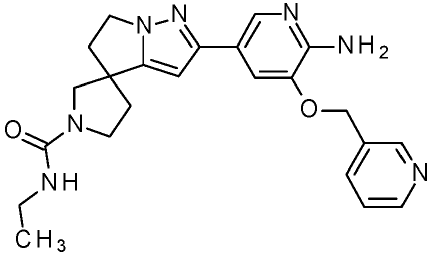

Example 505 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and {6-amino-5-[(pyridin-3-yl)methoxy]pyridin-3-yl}boronic acid (96.1 mg, 392 µmol).
LC-MS (Method 1): Rt = 0.77 min; MS (ESlpos): m/z = 434 [M+H]⁺
¹H-NMR (400 MHz, DMSO-d6) δ [ppm]: 1.003 (7.09), 1.021 (16.00), 1.039 (7.25), 1.065 (3.57), 1.155 (5.11), 1.232 (0.58), 1.292 (0.70), 2.040 (1.84), 2.058 (3.50), 2.074 (2.57), 2.083 (0.79), 2.331 (0.96), 2.522 (4.52), 2.532 (4.59), 2.549 (2.80), 2.562 (1.07), 2.673 (0.98), 3.021 (0.96), 3.039 (2.92), 3.053 (3.27), 3.057 (3.20), 3.070 (2.85), 3.088 (0.93), 3.405 (9.66), 3.424 (3.55), 3.434 (2.12), 3.442 (1.87), 3.462 (1.49), 3.480 (2.19), 3.496 (1.40), 3.506 (1.38), 3.521 (0.75), 4.165 (2.59), 4.182 (4.15), 4.200 (2.50), 5.270 (0.77), 5.301 (9.19), 6.153 (1.19), 6.167 (2.36), 6.180 (1.21), 6.437 (8.42), 6.549 (0.58), 6.659 (0.49), 6.720 (0.42), 7.438 (1.80), 7.450 (2.01), 7.458 (1.98), 7.470 (1.94), 7.639 (3.31), 7.937 (5.41), 7.941 (5.32), 7.968 (1.35), 7.972 (2.17), 7.977 (1.45), 7.987 (1.31), 7.992 (2.01), 7.997 (1.28), 8.557 (2.38), 8.560 (2.52), 8.569 (2.43), 8.572 (2.40), 8.761 (3.29), 8.765 (3.29).

### Example 506

### (rac)-2'-{6-amino-5-[(4-fluorophenyl)methoxy]pyridin-3-yl}-N-ethyl-5',6'-dihydrospiro[pyrrolidine-3,4'-pyrrolo[1,2-b]pyrazole]-1-carboxamide

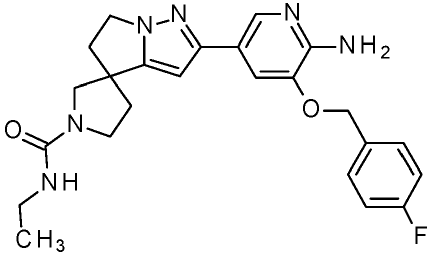

Example 506 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and {6-amino-5-[(4-fluorophenyl)methoxy]pyridin-3-yl}boronic acid (103 mg, 392 µmol).
LC-MS (Method 1): Rt = 0.98 min; MS (ESlpos): m/z = 451 [M+H]⁺
¹H-NMR (400 MHz, DMSO-d6) δ [ppm]: 1.002 (7.09), 1.020 (16.00), 1.038 (7.33), 1.233 (2.30), 1.256 (1.07), 1.295 (0.48), 2.028 (1.35), 2.042 (2.10), 2.055 (2.77), 2.073 (1.23), 2.084 (0.75), 2.522 (8.24), 2.530 (4.36), 3.020 (0.91), 3.038 (2.97), 3.052 (3.37), 3.055 (3.25), 3.069 (2.93), 3.087 (0.87), 3.382 (2.06), 3.398 (10.22), 3.425 (1.23), 3.465 (0.83), 3.483 (1.43), 3.497 (1.03), 3.505 (0.95), 3.523 (0.51), 4.141 (2.69), 4.159 (4.51), 4.176 (2.61), 5.158 (9.54), 5.795 (6.46), 6.140 (1.27), 6.154 (2.46), 6.168 (1.23), 6.339 (10.14), 7.202 (3.52), 7.207 (1.31), 7.224 (7.01), 7.246 (3.96), 7.433 (4.44), 7.437 (4.44), 7.562 (3.29), 7.577 (3.72), 7.584 (3.41), 7.598 (2.85), 7.937 (4.08), 7.941 (3.92).

### Example 507

### 2'-(6-amino-5-{[1-phenylethyl]oxy}pyridin-3-yl)-N-ethyl-5',6'-dihydrospiro[pyrrolidine-3,4'-pyrrolo[1,2-b]pyrazole]-1-carboxamide (diastereomeric mixture)

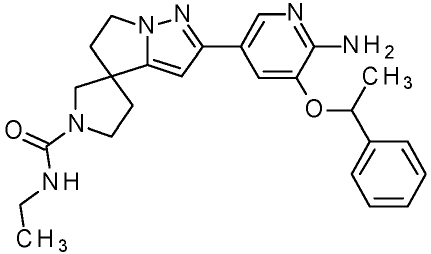

Example 507 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (100 mg, 262 µmol) and (rac)-[6-amino-5-(1-phenylethoxy)pyridin-3-yl]boronic acid (101 mg, 392 µmol).
LC-MS (Method 1): Rt = 1.00 min; MS (ESlpos): m/z = 447 [M+H]⁺
¹H-NMR (400 MHz, DMSO-d6) δ [ppm]: 0.997 (6.32), 1.002 (6.79), 1.015 (14.27), 1.020 (14.84), 1.033 (6.57), 1.038 (6.68), 1.561 (16.00), 1.577 (16.00), 2.010 (2.60), 2.023 (3.25), 2.028 (3.18), 2.041 (1.48), 2.463 (3.83), 2.482 (7.95), 2.518 (6.14), 2.523 (4.05), 3.019 (0.94), 3.032 (3.29), 3.036 (2.93), 3.046 (3.14), 3.050 (4.95), 3.064 (2.82), 3.068 (3.25), 3.081 (0.90), 3.086 (0.83), 3.355 (2.31), 3.373 (11.16), 3.386 (2.35), 3.399 (1.12), 3.445 (1.19), 3.463 (2.13), 3.469 (1.30), 3.477 (1.52), 3.484 (1.26), 3.502 (0.76), 4.104 (4.26), 4.122 (7.19), 4.139 (4.15), 5.558 (0.98), 5.574 (3.47), 5.589 (3.43), 5.605 (1.01), 5.829 (10.73), 6.131 (1.73), 6.145 (3.40), 6.158 (1.66), 6.219 (10.33), 6.225 (9.64), 7.224 (1.59), 7.231 (4.59), 7.235 (7.55), 7.240 (6.25), 7.260 (2.89), 7.264 (2.89), 7.319 (4.44), 7.337 (7.77), 7.339 (7.80), 7.357 (4.26), 7.454 (8.70), 7.472 (6.72), 7.839 (5.45), 7.843 (8.81), 7.847 (5.67).

### Example 508

### 2-{6-amino-5-[(1R)-1-(2,6-dichloro-3-fluorophenyl)ethoxy]pyridin-3-yl}-N-ethyl-6,7-dihydrospiro[pyrazolo[5,1-c][1,4]oxazine-4,3'-pyrrolidine]-1'-carboxamide (mixture of stereoisomers)

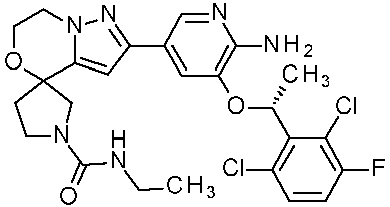

Example 508 was prepared in analogy to Example 461 using (rac)-2-bromo-N-ethyl-6,7-dihydrospiro[pyrazolo[5,1-c][1,4]oxazine-4,3'-pyrrolidine]-1'-carboxamide (100 mg, 304 µmol) and 3-[(1R)-1-(2,6-dichloro-3-fluorophenyl)ethoxy]-5-(4,4,5,5-tetramethyl-1,3,2-dioxaborolan-2-yl)pyridin-2-amine (156 mg, 365 µmol).
LC-MS (Method 1): Rt = 1.11 min; MS (ESlpos): m/z = 549 [M+H]⁺
¹H-NMR (400 MHz, DMSO-d6) δ [ppm]: 0.998 (6.00), 1.002 (6.59), 1.016 (13.76), 1.020 (14.82), 1.034 (6.28), 1.038 (6.69), 1.245 (1.19), 1.254 (0.88), 1.261 (1.27), 1.268 (1.22), 1.273 (0.53), 1.284 (1.13), 1.780 (15.99), 1.798 (16.00), 2.074 (3.94), 2.148 (0.51), 2.171 (1.02), 2.180 (1.15), 2.197 (0.93), 2.205 (1.63), 2.229 (0.91), 2.278 (1.28), 2.294 (1.47), 2.311 (0.88), 2.327 (1.12), 2.518 (1.32), 2.523 (0.85), 2.539 (7.74), 2.669 (0.53), 3.016 (0.73), 3.020 (0.87), 3.034 (2.86), 3.038 (2.69), 3.048 (2.80), 3.052 (4.25), 3.056 (2.98), 3.065 (2.54), 3.070 (2.91), 3.083 (0.83), 3.088 (0.78), 3.226 (0.48), 3.249 (0.61), 3.373 (7.30), 3.403 (4.72), 3.494 (1.36), 3.515 (2.34), 3.539 (1.08), 3.719 (2.22), 3.747 (1.93), 4.010 (0.50), 4.024 (1.40), 4.047 (2.24), 4.059 (6.60), 4.065 (6.69), 4.072 (6.24), 4.093 (1.00), 4.100 (1.40), 4.232 (0.66), 5.915 (3.36), 6.033 (0.58), 6.038 (0.66), 6.050 (2.08), 6.055 (2.18), 6.067 (2.12), 6.072 (2.19), 6.083 (0.67), 6.089 (0.60), 6.169 (1.66), 6.183 (3.33), 6.196 (1.66), 6.433 (13.08), 6.440 (2.05), 7.130 (3.50), 7.135 (4.09), 7.138 (4.09), 7.142 (3.66), 7.412 (1.89), 7.416 (1.83), 7.434 (3.67), 7.438 (3.56), 7.456 (2.58), 7.460 (2.58), 7.535 (1.88), 7.541 (1.97), 7.547 (2.17), 7.554 (2.05), 7.557 (1.77), 7.563 (1.56), 7.570 (1.55), 7.576 (1.35), 7.876 (7.59), 7.880 (7.68).

### Example 509

### (rac)-2'-[6-amino-5-(propan-2-yl)pyridin-3-yl]-N-ethyl-5',6'-dihydrospiro[pyrrolidine-3,4'-pyrrolo[1,2-b]pyrazole]-1-carboxamide

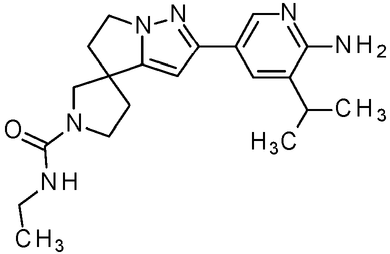

Example 509 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-(propan-2-yl)-5-(4,4,5,5-tetramethyl-1,3,2-dioxaborolan-2-yl)pyridin-2-amine (77.1 mg, 294 µmol).
LC-MS (Method 1): Rt = 0.86 min; MS (ESlpos): m/z = 369 [M+H]⁺
¹H-NMR (400 MHz, DMSO-d6) δ [ppm]: 1.001 (5.11), 1.019 (12.03), 1.037 (5.40), 1.065 (5.22), 1.133 (0.54), 1.150 (0.80), 1.163 (16.00), 1.179 (15.97), 2.024 (0.90), 2.038 (1.60), 2.055 (2.07), 2.073 (0.78), 2.085 (0.41), 2.523 (2.04), 2.529 (2.50), 2.907 (0.91), 2.923 (1.23), 2.940 (0.87), 3.018 (0.67), 3.036 (2.09), 3.050 (2.31), 3.053 (2.29), 3.068 (2.06), 3.086 (0.63), 3.374 (1.54), 3.381 (1.30), 3.397 (7.86), 3.417 (0.76), 3.468 (0.59), 3.486 (0.96), 3.492 (0.61), 3.500 (0.71), 3.507 (0.59), 4.146 (1.96), 4.163 (3.30), 4.181 (1.90), 6.046 (1.41), 6.145 (0.87), 6.159 (1.67), 6.173 (0.83), 6.344 (7.45), 7.679 (2.70), 7.685 (2.72), 8.161 (4.00), 8.166 (3.78).

### Example 510

### (rac)-2-[6-amino-5-(methylcarbamoyl)pyridin-3-yl]-N-ethyl-6,7-dihydrospiro[pyrazolo[5,1-c][1,4]oxazine-4,3'-pyrrolidine]-1'-carboxamide

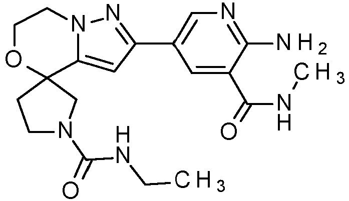

Example 510 was prepared in analogy to Example 461 using (rac)-2-bromo-N-ethyl-6,7-dihydrospiro[pyrazolo[5,1-c][1,4]oxazine-4,3'-pyrrolidine]-1'-carboxamide (75.0 mg, 228 µmol) and 2-amino-N-methyl-5-(4,4,5,5-tetramethyl-1,3,2-dioxaborolan-2-yl)pyridine-3-carboxamide (75.8 mg, 273 µmol).
LC-MS (Method 1): Rt = 0.69 min; MS (ESlpos): m/z = 400 [M+H]⁺
¹H-NMR (400 MHz, DMSO-d6) δ [ppm]: 1.002 (7.02), 1.020 (16.00), 1.038 (7.46), 2.208 (0.95), 2.233 (1.25), 2.256 (0.72), 2.326 (1.63), 2.341 (1.32), 2.358 (0.85), 2.375 (0.71), 2.756 (9.25), 3.019 (0.90), 3.037 (2.89), 3.051 (3.46), 3.069 (2.91), 3.086 (0.92), 3.365 (2.07), 3.376 (1.44), 3.392 (0.88), 3.411 (3.03), 3.440 (3.42), 3.521 (1.06), 3.544 (1.88), 3.566 (0.86), 3.764 (2.10), 3.792 (1.76), 4.051 (0.70), 4.066 (1.31), 4.082 (1.47), 4.099 (1.95), 4.122 (4.28), 4.133 (5.69), 6.171 (1.33), 6.185 (2.65), 6.199 (1.32), 6.623 (10.23), 7.167 (5.09), 8.197 (4.67), 8.203 (4.87), 8.456 (5.89), 8.462 (5.69), 8.542 (1.52).

### Example 511

### (rac)-2'-[6-amino-5-(methylcarbamoyl)pyridin-3-yl]-N-ethyl-5',6'-dihydrospiro[pyrrolidine-3,4'-pyrrolo[1,2-b]pyrazole]-1-carboxamide

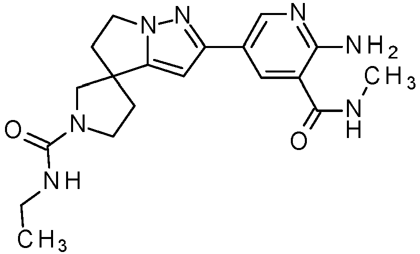

Example 511 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 2-amino-N-methyl-5-(4,4,5,5-tetramethyl-1,3,2-dioxaborolan-2-yl)pyridine-3-carboxamide (65.2 mg, 235 µmol).
LC-MS (Method 1): Rt = 0.69 min; MS (ESlpos): m/z = 384 [M+H]⁺
¹H-NMR (400 MHz, DMSO-d6) δ [ppm]: 0.988 (0.42), 1.005 (7.74), 1.022 (16.00), 1.041 (7.27), 2.050 (1.88), 2.068 (4.12), 2.085 (2.02), 2.518 (6.85), 2.523 (4.35), 2.539 (2.99), 2.557 (3.91), 2.574 (2.61), 2.781 (10.82), 2.793 (10.94), 2.801 (2.26), 2.812 (1.69), 3.042 (2.05), 3.055 (2.38), 3.072 (1.95), 3.320 (0.61), 3.411 (5.65), 3.418 (6.05), 3.443 (2.94), 3.452 (1.36), 3.470 (2.09), 3.487 (1.11), 3.495 (1.01), 3.512 (0.45), 3.931 (0.49), 4.151 (0.80), 4.188 (2.89), 4.206 (4.40), 4.223 (2.78), 6.178 (1.29), 6.465 (9.04), 6.691 (0.61), 6.962 (0.66), 7.090 (0.75), 7.217 (0.66), 8.447 (0.71), 8.454 (0.92), 8.482 (3.60), 8.488 (6.14), 8.499 (3.01), 8.834 (1.15), 8.846 (1.15).

### Example 512

### (rac)-2'-(6-amino-5-cyclopropylpyridin-3-yl)-N-ethyl-5',6'-dihydrospiro[pyrrolidine-3,4'-pyrrolo[1,2-b]pyrazole]-1-carboxamide

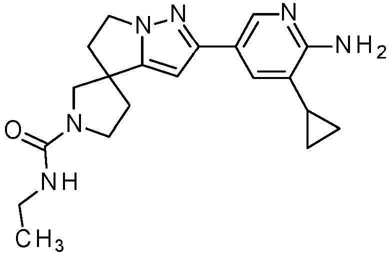

Example 512 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-cyclopropyl-5-(4,4,5,5-tetramethyl-1,3,2-dioxaborolan-2-yl)pyridin-2-amine (76.5 mg, 294 µmol).
LC-MS (Method 1): Rt = 0.81 min; MS (ESlpos): m/z = 367 [M+H]⁺
¹H-NMR (400 MHz, DMSO-d6) δ [ppm]: 0.570 (0.93), 0.581 (2.77), 0.585 (2.92), 0.594 (3.27), 0.599 (2.78), 0.609 (1.08), 0.889 (0.97), 0.899 (2.78), 0.904 (2.57), 0.910 (1.45), 0.914 (1.46), 0.920 (2.78), 0.924 (2.66), 0.935 (0.93), 1.000 (5.84), 1.018 (13.69), 1.036 (6.19), 1.691 (0.70), 1.698 (0.77), 1.711 (1.29), 1.724 (0.71), 1.732 (0.63), 2.024 (1.28), 2.043 (2.38), 2.054 (1.15), 2.061 (1.07), 2.527 (2.61), 2.539 (16.00), 3.017 (0.79), 3.035 (2.48), 3.049 (2.75), 3.053 (2.69), 3.067 (2.45), 3.084 (0.77), 3.362 (1.57), 3.376 (2.02), 3.392 (6.06), 3.401 (2.54), 3.418 (1.46), 3.461 (0.87), 3.479 (1.39), 3.487 (0.86), 3.494 (0.96), 3.504 (0.89), 3.520 (0.51), 4.139 (2.28), 4.157 (3.79), 4.174 (2.22), 6.137 (0.98), 6.151 (1.98), 6.165 (0.99), 6.335 (1.12), 6.371 (8.91), 7.509 (2.95), 7.514 (2.96), 8.134 (5.19), 8.165 (3.53), 8.170 (3.53).

### Example 513

### (rac)-2'-[6-amino-5-(methylcarbamoyl)pyridin-3-yl]-N-(1-phenylcyclobutyl)-5',6'-dihydrospiro[pyrrolidine-3,4'-pyrrolo[1,2-b]pyrazole]-1-carboxamide

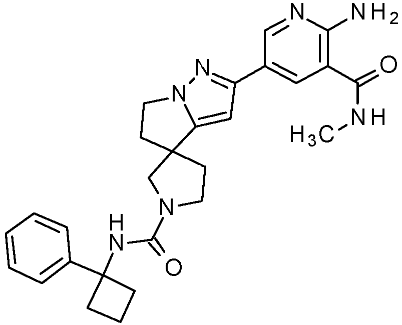

Example 513 was prepared in analogy to Example 461 using (*rac*)-1-[(1-phenylcyclobutyl)carbamoyl]-5',6'-dihydrospiro[pyrrolidine-3,4'-pyrrolo[1,2-b]pyrazol]-2'-yl trifluoromethanesulfonate (194 mg, 400 µmol) and 2-amino-N-methyl-5-(4,4,5,5-tetramethyl-1,3,2-dioxaborolan-2-yl)pyridine-3-carboxamide (127 mg, 460 µmol).
LC-MS (Method 1): Rt = 0.98 min; MS (ESlpos): m/z = 486 [M+H]⁺
¹H-NMR (400 MHz, DMSO-d6) δ [ppm]: 1.232 (0.21), 1.760 (0.23), 1.766 (0.24), 1.783 (0.25), 1.788 (0.27), 1.805 (0.17), 1.975 (0.26), 1.990 (0.21), 2.002 (0.24), 2.054 (0.48), 2.071 (0.95), 2.088 (0.50), 2.384 (0.41), 2.394 (0.50), 2.406 (0.53), 2.428 (0.29), 2.451 (0.35), 2.475 (0.72), 2.522 (1.02), 2.539 (16.00), 2.560 (1.05), 2.576 (0.68), 2.791 (2.99), 2.801 (3.02), 3.413 (0.19), 3.441 (1.26), 3.466 (0.53), 3.484 (0.26), 3.509 (0.42), 3.526 (0.26), 3.535 (0.24), 4.197 (0.68), 4.215 (1.27), 4.232 (0.82), 6.472 (2.40), 6.718 (1.08), 6.984 (0.41), 7.112 (0.48), 7.133 (0.34), 7.151 (0.86), 7.170 (0.57), 7.240 (0.51), 7.263 (0.97), 7.284 (1.64), 7.302 (0.95), 7.422 (1.58), 7.440 (1.26), 7.443 (1.01), 8.466 (1.16), 8.471 (1.29), 8.568 (1.05), 8.572 (0.99), 8.921 (0.42), 8.932 (0.43).

### Example 514

### (rac)-2'-[6-amino-5-(dimethylcarbamoyl)pyridin-3-yl]-N-(1-phenylcyclobutyl)-5',6'-dihydrospiro[pyrrolidine-3,4'-pyrrolo[1,2-b]pyrazole]-1-carboxamide

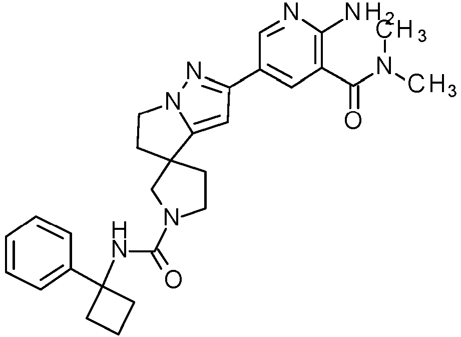

Example 514 was prepared in analogy to Example 461 using (*rac*)-1-[(1-phenylcyclobutyl)carbamoyl]-5',6'-dihydrospiro[pyrrolidine-3,4'-pyrrolo[1,2-b]pyrazol]-2'-yl trifluoromethanesulfonate (169 mg, 348 µmol) and 2-amino-N,N-dimethyl-5-(4,4,5,5-tetramethyl-1,3,2-dioxaborolan-2-yl)pyridine-3-carboxamide (116 mg, 400 µmol).
LC-MS (Method 1): Rt = 0.98 min; MS (ESlpos): m/z = 500 [M+H]⁺
¹H-NMR (400 MHz, DMSO-d6) δ [ppm]: 1.746 (0.67), 1.763 (1.25), 1.768 (1.32), 1.773 (0.92), 1.779 (0.92), 1.784 (1.32), 1.790 (1.55), 1.807 (0.92), 1.956 (0.65), 1.963 (0.82), 1.979 (1.50), 1.994 (1.20), 2.002 (1.47), 2.006 (1.55), 2.017 (1.20), 2.029 (2.27), 2.043 (3.05), 2.056 (3.32), 2.074 (2.45), 2.086 (0.67), 2.356 (0.95), 2.383 (2.45), 2.394 (2.25), 2.399 (2.75), 2.415 (1.32), 2.421 (1.55), 2.447 (1.52), 2.469 (3.32), 2.475 (2.87), 2.518 (10.51), 2.522 (7.66), 2.539 (7.41), 2.941 (12.06), 3.375 (0.90), 3.391 (2.55), 3.400 (1.70), 3.415 (5.97), 3.432 (4.89), 3.457 (1.47), 3.504 (0.92), 3.521 (1.70), 3.529 (1.20), 3.537 (1.25), 3.546 (1.20), 4.150 (3.94), 4.167 (7.49), 4.185 (3.87), 6.058 (10.83), 6.326 (16.00), 6.694 (7.16), 7.126 (1.20), 7.129 (2.27), 7.133 (1.27), 7.143 (1.57), 7.148 (5.32), 7.152 (1.82), 7.163 (2.10), 7.166 (3.57), 7.169 (2.02), 7.257 (6.04), 7.261 (2.40), 7.277 (9.68), 7.290 (1.87), 7.295 (5.67), 7.416 (8.26), 7.419 (9.04), 7.432 (2.25), 7.437 (7.44), 7.440 (5.79), 7.659 (9.19), 7.665 (9.49), 8.135 (2.62), 8.383 (11.13), 8.388 (10.81).

### Example 515

### 2'-{6-amino-5-[(1R)-1-(pyridin-4-yl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

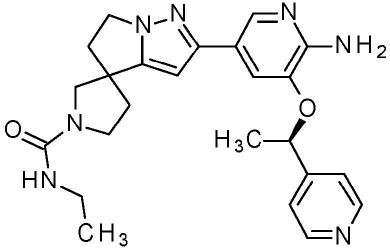

Example 515 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (59.0 mg, 154 µmol) and {6-amino-5-[(1R)-1-(pyridin-4-yl)ethoxy]pyridin-3-yl}boronic acid (60.0 mg, 232 µmol).
LC-MS (Method 1): Rt = 0.79 min; MS (ESlpos): m/z = 448 [M+H]⁺
¹H-NMR (400 MHz, DMSO-d6) δ [ppm]: 0.851 (1.66), 0.995 (6.95), 0.999 (7.34), 1.013 (15.45), 1.016 (15.94), 1.031 (7.28), 1.035 (8.61), 1.052 (3.42), 1.070 (2.15), 1.232 (8.77), 1.255 (1.99), 1.334 (1.49), 1.347 (1.27), 1.554 (14.73), 1.570 (14.68), 1.900 (1.99), 2.022 (3.53), 2.463 (4.47), 2.518 (16.00), 2.523 (9.82), 3.015 (1.21), 3.032 (3.64), 3.047 (5.35), 3.064 (3.86), 3.082 (1.21), 3.164 (2.32), 3.443 (2.81), 3.461 (3.37), 4.103 (3.97), 4.121 (6.46), 4.137 (3.86), 5.649 (3.03), 5.665 (3.03), 5.928 (9.49), 6.133 (1.77), 6.147 (3.64), 6.160 (2.15), 6.180 (3.09), 6.245 (10.21), 6.247 (9.38), 7.239 (6.29), 7.474 (8.83), 7.490 (9.27), 7.874 (5.08), 7.878 (7.89), 7.882 (4.86), 8.376 (1.99), 8.529 (7.34), 8.532 (8.06), 8.545 (7.94), 8.547 (6.34).

### Example 516

### 2'-{6-amino-5-[(1S)-1-(pyridin-4-yl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

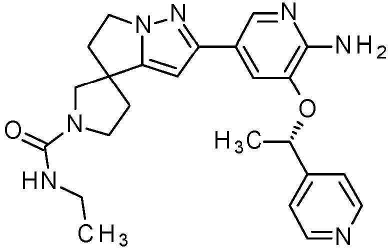

Example 516 was prepared in analogy to Example 461 using (rac)-1-(ethylcarbamoyl)-5',6'-dihydrospiro[pyrrolidine-3,4'-pyrrolo[1,2-b]pyrazol]-2'-yl trifluoromethanesulfonate (98.4 mg, 257 µmol) and {6-amino-5-[(1S)-1-(pyridin-4-yl)ethoxy]pyridin-3-yl}boronic acid (100 mg, 386 µmol).
LC-MS (Method 1): Rt = 0.79 min; MS (ESlpos): m/z = 448 [M+H]⁺
¹H-NMR (400 MHz, DMSO-d6) δ [ppm]: 0.996 (6.71), 0.999 (6.97), 1.014 (14.82), 1.017 (14.97), 1.031 (7.21), 1.035 (7.15), 1.066 (0.47), 1.495 (0.53), 1.554 (15.82), 1.570 (16.00), 2.022 (3.65), 2.028 (3.56), 2.463 (4.74), 3.014 (1.12), 3.031 (3.74), 3.047 (5.38), 3.064 (3.76), 3.079 (1.12), 3.372 (11.97), 3.443 (1.38), 3.461 (2.35), 3.476 (1.76), 3.500 (0.85), 4.103 (4.24), 4.121 (7.32), 4.138 (4.26), 5.633 (1.06), 5.649 (3.47), 5.665 (3.47), 5.681 (1.03), 5.927 (11.12), 6.129 (1.97), 6.143 (3.97), 6.157 (2.06), 6.245 (10.15), 6.247 (10.29), 7.240 (7.06), 7.475 (9.82), 7.490 (10.15), 7.874 (5.21), 7.878 (8.47), 7.882 (5.35), 8.530 (7.97), 8.532 (9.38), 8.544 (9.24), 8.547 (7.94).

### Example 517

### (rac)-2'-[6-amino-5-(1-methyl-1H-pyrazol-3-yl)pyridin-3-yl]-N-ethyl-5',6'-dihydrospiro[pyrrolidine-3,4'-pyrrolo[1,2-b]pyrazole]-1-carboxamide

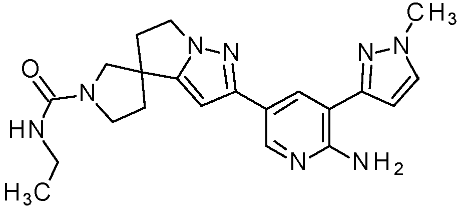

Example 517 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (117 mg, 306 µmol) and [6-amino-5-(1-methyl-1H-pyrazol-3-yl)pyridin-3-yl]boronic acid (100 mg, 459 µmol).
LC-MS (Method 1): Rt = 0.81 min; MS (ESlpos): m/z = 407 [M+H]⁺
¹H-NMR (400 MHz, DMSO-d6) δ [ppm]: 1.005 (4.62), 1.024 (10.37), 1.041 (4.85), 1.865 (5.43), 2.041 (0.96), 2.055 (1.42), 2.067 (1.89), 2.085 (0.84), 2.527 (3.03), 2.539 (14.06), 2.544 (2.36), 3.024 (0.68), 3.042 (2.06), 3.056 (2.34), 3.060 (2.27), 3.074 (2.05), 3.091 (0.67), 3.387 (3.67), 3.392 (3.75), 3.400 (3.29), 3.414 (8.12), 3.436 (1.72), 3.483 (1.00), 3.501 (1.31), 3.508 (0.92), 3.516 (0.98), 3.527 (0.85), 3.541 (0.56), 3.921 (16.00), 4.164 (1.87), 4.181 (3.15), 4.198 (1.80), 6.148 (0.85), 6.162 (1.70), 6.176 (0.86), 6.451 (7.12), 6.870 (3.91), 6.875 (3.99), 7.076 (2.26), 7.801 (3.47), 7.806 (3.42), 8.138 (3.43), 8.143 (3.62), 8.332 (4.09), 8.338 (3.93).

### Example 518

### (rac)-2'-[6-amino-5-(1,2-thiazol-5-yl)pyridin-3-yl]-N-ethyl-5',6'-dihydrospiro[pyrrolidine-3,4'-pyrrolo[1,2-b]pyrazole]-1-carboxamide

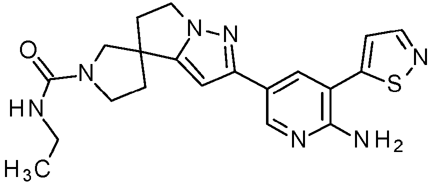

Example 518 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (138 mg, 362 µmol) and [6-amino-5-(1,2-thiazol-5-yl)pyridin-3-yl]boronic acid (120 mg, 543 µmol).
LC-MS (Method 1): Rt = 0.81 min; MS (ESlpos): m/z = 410 [M+H]⁺
¹H-NMR (400 MHz, DMSO-d6) δ [ppm]: 1.001 (7.05), 1.010 (0.94), 1.019 (16.00), 1.026 (1.12), 1.037 (7.12), 2.036 (1.28), 2.042 (1.29), 2.051 (1.89), 2.060 (2.53), 2.068 (1.31), 2.078 (1.17), 2.090 (0.47), 2.518 (1.68), 2.525 (3.83), 2.543 (2.73), 3.019 (0.87), 3.037 (2.78), 3.051 (3.16), 3.054 (3.07), 3.069 (2.85), 3.086 (0.89), 3.380 (1.25), 3.385 (1.75), 3.392 (1.57), 3.406 (8.70), 3.429 (1.08), 3.471 (0.84), 3.489 (1.34), 3.497 (0.81), 3.504 (1.08), 3.514 (0.85), 3.530 (0.49), 4.166 (2.54), 4.184 (4.11), 4.201 (2.45), 6.154 (7.57), 6.171 (1.41), 6.466 (10.91), 7.732 (7.30), 7.736 (7.64), 7.931 (5.66), 7.937 (5.71), 8.465 (6.41), 8.471 (6.27), 8.629 (7.51), 8.633 (7.18).

### Example 519

### (rac)-2'-[6-amino-5-(1,5-dimethyl-1H-pyrazol-4-yl)pyridin-3-yl]-N-ethyl-5',6'-dihydrospiro[pyrrolidine-3,4'-pyrrolo[1,2-b]pyrazole]-1-carboxamide

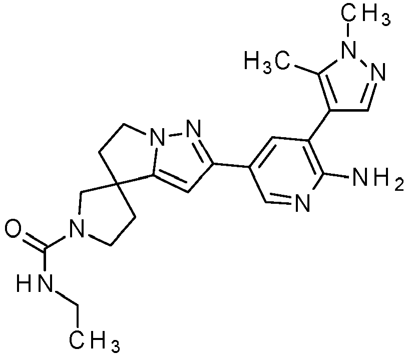

Example 519 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (110 mg, 287 µmol) and [6-amino-5-(1,5-dimethyl-1H-pyrazol-4-yl)pyridin-3-yl]boronic acid (100 mg, 431 µmol).
LC-MS (Method 1): Rt = 0.74 min; MS (ESlpos): m/z = 421 [M+H]⁺
¹H-NMR (400 MHz, DMSO-d6) δ [ppm]: 0.998 (4.35), 1.016 (10.15), 1.033 (4.62), 2.024 (0.77), 2.036 (1.26), 2.054 (1.71), 2.072 (0.68), 2.171 (15.49), 2.186 (1.19), 2.518 (0.89), 2.524 (1.69), 3.015 (0.55), 3.033 (1.74), 3.047 (1.94), 3.051 (1.88), 3.065 (1.72), 3.083 (0.51), 3.365 (1.14), 3.372 (0.89), 3.396 (5.87), 3.466 (0.45), 3.484 (0.74), 3.491 (0.51), 3.498 (0.57), 3.505 (0.46), 3.509 (0.46), 3.774 (16.00), 4.141 (1.56), 4.159 (2.66), 4.176 (1.50), 5.582 (3.75), 6.134 (0.71), 6.148 (1.45), 6.162 (0.73), 6.344 (6.43), 7.419 (5.94), 7.531 (3.40), 7.537 (3.49), 8.295 (3.83), 8.300 (3.79).

### Example 520

### (rac)-2'-{6-amino-5-[(trifluoromethyl)sulfanyl]pyridin-3-yl}-N-ethyl-5',6'-dihydrospiro[pyrrolidine-3,4'-pyrrolo[1,2-b]pyrazole]-1-carboxamide

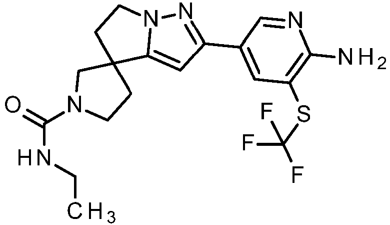

Example 520 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (161 mg, 420 µmol) and {6-amino-5-[(trifluoromethyl)sulfanyl]pyridin-3-yl}boronic acid (150 mg, 630 µmol).
LC-MS (method 1): Rt = 0.95 min; MS (ESlneg): m/z = 425 [M-H]⁻
¹H-NMR (400 MHz, DMSO-d6) δ [ppm]: 1.001 (6.98), 1.019 (16.00), 1.036 (7.52), 1.053 (1.26), 1.070 (0.63), 1.231 (1.75), 2.029 (1.42), 2.035 (1.44), 2.046 (2.04), 2.053 (2.70), 2.071 (1.21), 2.083 (0.44), 2.518 (5.64), 2.536 (2.85), 3.019 (0.91), 3.036 (2.84), 3.050 (3.21), 3.053 (3.10), 3.068 (2.81), 3.086 (0.82), 3.377 (1.88), 3.384 (1.62), 3.398 (10.06), 3.420 (0.98), 3.466 (0.82), 3.484 (1.39), 3.492 (0.84), 3.499 (0.98), 3.509 (0.86), 3.525 (0.48), 4.157 (2.74), 4.175 (4.50), 4.192 (2.63), 6.140 (1.19), 6.153 (2.34), 6.167 (1.16), 6.428 (10.94), 6.736 (6.68), 8.052 (4.00), 8.057 (4.07), 8.526 (6.03), 8.532 (5.80).

### Example 521

### 5-[1-methyl-5',6'-dihydrospiro[pyrrolidine-3,4'-pyrrolo[1,2-b]pyrazol]-2'-yl]-3-(trifluoromethyl)pyridin-2-amine (stereoisomer 1)

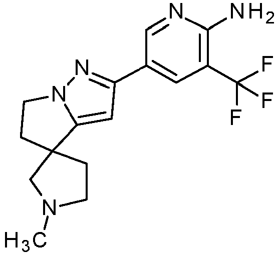

Example 521 was prepared in analogy to Example 461 using 1-methyl-5',6'-dihydrospiro[pyrrolidine-3,4'-pyrrolo[1,2-b]pyrazol]-2'-yl trifluoromethanesulfonate (230 mg, 707 µmol, stereoisomer 1) and 5-(4,4,5,5-tetramethyl-1,3,2-dioxaborolan-2-yl)-3-(trifluoromethyl)pyridin-2-amine (306 mg, 1.06 mmol).
1H NMR (DMSO-d6) δ: 8.59 (d, 1H), 8.02 (d, 1H), 6.51 (s, 2H), 6.45 (s, 1H), 4.10 (m, 2H), 2.66-2.74 (m, 2H), 2.53 (br d, 4H), 1.97-2.08 (m, 2H)
[α]²⁰_{D} : -21.8° (c = 1.00, DMSO)
LC-MS (method 1): Rt = 0.97 min; MS (ESlpos): m/z = 339 [M+H]⁺

### Example 522

### 5-[1-methyl-5',6'-dihydrospiro[pyrrolidine-3,4'-pyrrolo[1,2-b]pyrazol]-2'-yl]-3-(trifluoromethyl)pyridin-2-amine (stereoisomer 2)

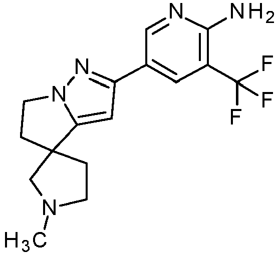

Example 522 was prepared in analogy to Example 461 using 1-methyl-5',6'-dihydrospiro[pyrrolidine-3,4'-pyrrolo[1,2-b]pyrazol]-2'-yl trifluoromethanesulfonate (240 mg, 738 µmol, stereoisomer 2) and 5-(4,4,5,5-tetramethyl-1,3,2-dioxaborolan-2-yl)-3-(trifluoromethyl)pyridin-2-amine (319 mg, 1.11 mmol).
1H NMR (DMSO-d6) δ: 8.59 (d, 1H), 8.02 (d, 1H), 6.51 (s, 2H), 6.45 (s, 1H), 4.10 (m, 2H), 2.66-2.75 (m, 2H), 2.51-2.64 (m, 4H), 1.96-2.10 (m, 2H)
[α]²⁰_{D} : +21.6° (c = 1.00, DMSO)
LC-MS (method 1): Rt = 0.97 min; MS (ESlpos): m/z = 339 [M+H]⁺

### Example 523

### (3R)-2'-{6-amino-5-[(1R)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide

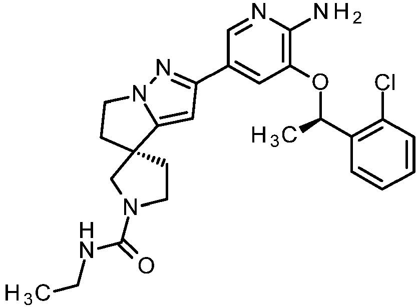

Example 523 was prepared in analogy to Example 461 using 3-[(1R)-1-(2-chlorophenyl)ethoxy]-5-(4,4,5,5-tetramethyl-1,3,2-dioxaborolan-2-yl)pyridin-2-amine (85.0 mg, 227 µmol) and (3R)-1-(ethylcarbamoyl)-5',6'-dihydrospiro[pyrrolidine-3,4'-pyrrolo[1,2-b]pyrazol]-2'-yl trifluoromethanesulfonate (91.1 mg, 238 µmol).
1H NMR (DMSO-d6, 400 MHz) δ 7.86 (d, 1H), 7.60 (dd, 1H), 7.47 (dd, 1H), 7.3-7.4 (m, 2H), 7.09 (d, 1H), 6.17 (s, 1H), 6.14 (t, 1H), 5.94 (s, 2H), 5.77 (q, 1H), 4.12 (t, 2H), 3.4-3.5 (m, 1H), 3.3-3.4 (m, 3H), 3.0-3.1 (m, 2H), 2.0-2.1 (m, 2H), 1.60 (d, 3H), 1.01 (t, 3H)
LC-MS (method 1): Rt = 1.04 min; MS (ESlpos): m/z = 482 [M+H]⁺

### Example 524

### 2'-{6-amino-5-[(1R)-1-(2-chlorophenyl)ethoxy]pyridin-3-yl}-N-ethyl-5',6'-dihydrospiro[azetidine-3,4'-pyrrolo[1,2-b]pyrazole]-1-carboxamide

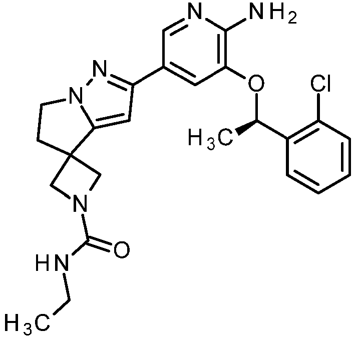

Example 524 was prepared in analogy to Example 461 using 3-[(1R)-1-(2-chlorophenyl)ethoxy]-5-(4,4,5,5-tetramethyl-1,3,2-dioxaborolan-2-yl)pyridin-2-amine (85.0 mg, 227 µmol) and 1-(ethylcarbamoyl)-5',6'-dihydrospiro[azetidine-3,4'-pyrrolo[1,2-b]pyrazol]-2'-yl trifluoromethanesulfonate (87.7 mg, 238 µmol).
1H NMR (DMSO-d6, 400 MHz) δ 7.89 (d, 1H), 7.60 (dd, 1H), 7.46 (dd, 1H), 7.3-7.4 (m, 2H), 7.13 (d, 1H), 6.4-6.5 (m, 2H), 5.96 (s, 2H), 5.78 (q, 1H),4.0-4.1 (m, 2H), 3.9-4.0 (m, 4H), 3.0-3.1 (m, 2H), 2.79 (t, 2H), 1.60 (d, 3H), 1.0-1.0 (m, 3H)
LC-MS (method 1): Rt = 1.02 min; MS (ESlpos): m/z = 468 [M+H]⁺

### Example 525

### 2'-{6-amino-5-[(1S)-1-(2-chlorophenyl)ethoxy]pyridin-3-yl}-N-ethyl-5',6'-dihydrospiro[azetidine-3,4'-pyrrolo[1,2-b]pyrazole]-1-carboxamide

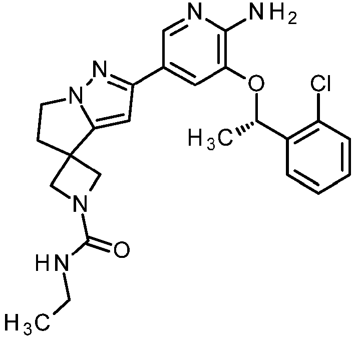

Example 525 was prepared in analogy to Example 461 using 3-[(1S)-1-(2-chlorophenyl)ethoxy]-5-(4,4,5,5-tetramethyl-1,3,2-dioxaborolan-2-yl)pyridin-2-amine (50.0 mg, 133 µmol) and 1-(ethylcarbamoyl)-5',6'-dihydrospiro[azetidine-3,4'-pyrrolo[1,2-b]pyrazol]-2'-yl trifluoromethanesulfonate (51.6 mg, 140 µmol).
1H NMR (DMSO-d6, 400 MHz) δ 7.89 (d, 1H), 7.60 (dd, 1H), 7.46 (dd, 1H), 7.3-7.4 (m, 2H), 7.13 (d, 1H), 6.4-6.5 (m, 2H), 5.94 (s, 2H), 5.7-5.8 (m, 1H), 4.07 (t, 2H), 3.9-4.0 (m, 4H), 3.02 (dd, 2H), 2.79 (t, 2H), 1.60 (d, 3H), 1.01 (t, 3H)
LC-MS (method 1): Rt = 1.02 min; MS (ESlpos): m/z = 468 [M+H]⁺

### Example 526

### (3'R)-2-{6-amino-5-[(1R)-1-(2,6-dichloro-3-fluorophenyl)ethoxy]pyridin-3-yl}-N-ethyl-6,7-dihydrospiro[pyrazolo[5,1-c][1,4]oxazine-4,3'-pyrrolidine]-1'-carboxamide

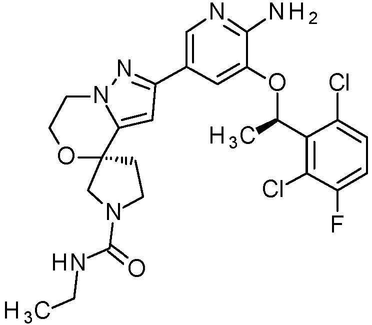

Example 526 was prepared in analogy to Example 461 using 3-[(1R)-1-(2,6-dichloro-3-fluorophenyl)ethoxy]-5-(4,4,5,5-tetramethyl-1,3,2-dioxaborolan-2-yl)pyridin-2-amine (140 mg, 328 µmol) and (3'R)-2-bromo-N-ethyl-6,7-dihydrospiro[pyrazolo[5,1-c][1,4]oxazine-4,3'-pyrrolidine]-1'-carboxamide (119 mg, 361 µmol).
1H NMR (DMSO-d6, 400 MHz) δ 7.88 (d, 1H), 7.56 (dd, 1H), 7.4-7.5 (m, 1H), 7.12 (d, 1H), 6.43 (s, 1H), 6.17 (t, 1H), 6.05 (q, 1H), 5.83 (s, 2H), 4.0-4.1 (m, 4H), 3.73 (d, 1H), 3.5-3.6 (m, 1H), 3.39 (d, 1H), 3.0-3.1 (m, 2H), 2.3-2.3 (m, 1H), 2.1-2.2 (m, 1H), 1.79 (d, 3H), 1.02 (t, 3H)
LC-MS (method 1): Rt = 1.09 min; MS (ESlpos): m/z = 549 [M+H]⁺

### Example 527

### (3R)-2'-{6-amino-5-[(1R)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide

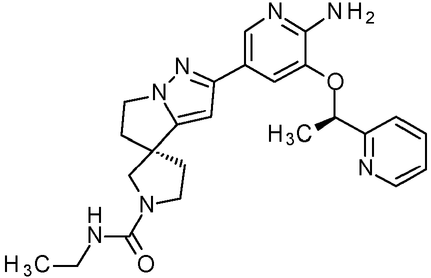

Example 527 was prepared in analogy to Example 461 using 3-[(1R)-1-(pyridin-2-yl)ethoxy]-5-(4,4,5,5-tetramethyl-1,3,2-dioxaborolan-2-yl)pyridin-2-amine (220 mg, 645 µmol) and (3R)-1-(ethylcarbamoyl)-5',6'-dihydrospiro[pyrrolidine-3,4'-pyrrolo[1,2-b]pyrazol]-2'-yl trifluoromethanesulfonate (222 mg, 580 µmol).
1H NMR (DMSO-d6, 400 MHz) δ 8.7-8.7 (m, 1H), 8.47 (dd, 1H), 7.91 (td, 1H), 7.87 (d, 1H), 7.38 (ddd, 1H), 7.33 (d, 1H), 6.27 (s, 1H), 6.15 (t, 1H), 5.89 (s, 2H), 5.71 (q, 1H), 4.13 (t, 2H), 3.4-3.5 (m, 1H), 3.4-3.4 (m, 3H), 3.0-3.1 (m, 2H), 2.0-2.1 (m, 2H), 1.60 (d, 3H), 1.02 (t, 3H)
LC-MS (method 1): Rt = 0.80 min; MS (ESlpos): m/z = 449 [M+H]⁺

### Example 528

### 2'-{6-amino-5-[(1R)-1-(pyridin-3-yl)ethoxy]pyridin-3-yl}-N-ethyl-5',6'-dihydrospiro[azetidine-3,4'-pyrrolo[1,2-b]pyrazole]-1-carboxamide

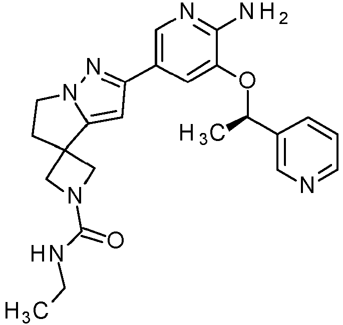

Example 528 was prepared in analogy to Example 461 using 3-[(1R)-1-(pyridin-3-yl)ethoxy]-5-(4,4,5,5-tetramethyl-1,3,2-dioxaborolan-2-yl)pyridin-2-amine (40.0 mg, 117 µmol) and 1-(ethylcarbamoyl)-5',6'-dihydrospiro[azetidine-3,4'-pyrrolo[1,2-b]pyrazol]-2'-yl trifluoromethanesulfonate (47.5 mg, 129 µmol).
1H NMR (DMSO-d6, 400 MHz) δ 8.4-8.6 (m, 1H), 7.90 (d, 1H), 7.79 (dt, 1H), 7.51 (d, 1H), 7.29 (ddd, 1H), 7.24 (d, 1H), 6.48 (s, 1H), 6.44 (t, 1H), 5.89 (s, 2H), 5.53 (q, 1H), 4.06 (t, 2H), 3.9-4.0 (m, 4H), 3.0-3.1 (m, 2H), 2.79 (t, 2H), 1.62 (d, 3H), 1.01 (t, 3H)
LC-MS (method 1): Rt = 0.81 min; MS (ESlpos): m/z = 435 [M+H]⁺

### Example 529

### (3R)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 1)

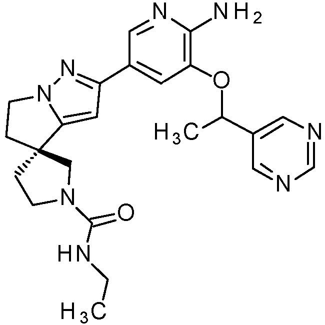

Example 529 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (98.8 mg, 259 µmol) and 3-{[1-(pyrimidin-5-yl)ethyl]oxy}-5-(4,4,5,5-tetramethyl-1,3,2-dioxaborolan-2-yl)pyridin-2-amine (115 mg, 336 µmol, stereoisomer 2).

1H-NMR (400MHz, DMSO-d6): δ [ppm]= 1.02 (t, 3H), 1.62 (d, 3H), 1.98 - 2.11 (m, 2H), 3.00 - 3.10 (m, 2H), 3.35 - 3.42 (m, 3H), 3.44 - 3.52 (m, 1H), 4.15 (t, 2H), 5.79 (q, 1H), 5.96 (s, 2H), 6.15 (t, 1H), 6.30 (s, 1H), 7.42 (d, 1H), 7.91 (d, 1H), 8.98 (s, 2H), 9.11 (s, 1H).

### Example 530

### ((3R)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 2)

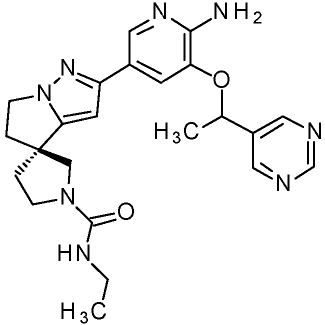

Example 530 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (98.8 mg, 259 µmol) and 3-{[1-(pyrimidin-5-yl)ethyl]oxy}-5-(4,4,5,5-tetramethyl-1,3,2-dioxaborolan-2-yl)pyridin-2-amine (115 mg, 336 µmol, stereoisomer 2).
[α]²⁰_{D} : +125.9° (c = 1.00, DMSO)
¹H-NMR (400 MHz, DMSO-d6) δ [ppm]: 0.999 (5.23), 1.017 (11.91), 1.035 (5.29), 1.066 (2.84), 1.613 (7.15), 1.629 (7.23), 2.018 (0.90), 2.032 (1.35), 2.044 (1.83), 2.062 (0.83), 2.518 (4.42), 2.523 (2.11), 3.017 (0.63), 3.034 (2.03), 3.048 (2.25), 3.052 (2.23), 3.066 (2.02), 3.084 (0.60), 3.358 (0.97), 3.383 (4.66), 3.388 (4.14), 3.402 (1.49), 3.413 (0.73), 3.420 (0.60), 3.456 (0.53), 3.474 (0.95), 3.482 (0.57), 3.488 (0.69), 3.499 (0.57), 3.938 (0.53), 4.127 (1.78), 4.145 (3.01), 4.162 (1.78), 5.766 (0.45), 5.782 (1.60), 5.798 (1.61), 5.813 (0.47), 5.962 (4.70), 6.135 (0.83), 6.149 (1.70), 6.163 (0.84), 6.307 (7.98), 7.418 (3.03), 7.423 (3.11), 7.909 (4.15), 7.913 (4.13), 8.986 (16.00), 9.115 (8.60).

### Example 531

### (rac)-2'-[6-amino-5-(difluoromethyl)pyridin-3-yl]-N-ethyl-5',6'-dihydrospiro[pyrrolidine-3,4'-pyrrolo[1,2-b]pyrazole]-1-carboxamide

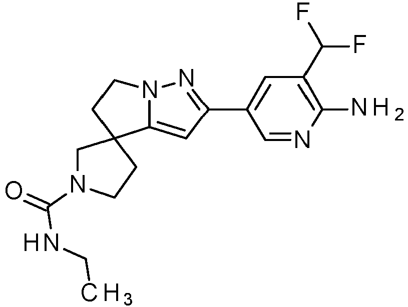

Example 531 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (102 mg, 266 µmol) and [6-amino-5-(difluoromethyl)pyridin-3-yl]boronic acid (100 mg, 75 % purity, 399 µmol).
LC-MS (method 1): Rt = 0.80 min; MS (ESlpos): m/z = 377 [M+H]⁺
¹H-NMR (400 MHz, DMSO-d6) δ [ppm]: 1.000 (5.76), 1.018 (13.49), 1.036 (6.25), 1.232 (0.60), 2.032 (1.11), 2.045 (1.85), 2.060 (2.54), 2.078 (1.16), 2.090 (0.55), 2.518 (16.00), 2.536 (5.08), 2.673 (1.17), 3.018 (0.74), 3.035 (2.47), 3.050 (2.79), 3.053 (2.93), 3.067 (2.44), 3.085 (0.76), 3.372 (1.88), 3.379 (1.41), 3.400 (9.52), 3.413 (2.09), 3.468 (0.76), 3.485 (1.28), 3.500 (0.97), 3.507 (0.81), 3.525 (0.49), 4.155 (2.29), 4.173 (4.00), 4.190 (2.63), 6.138 (0.95), 6.152 (1.97), 6.165 (1.14), 6.338 (3.80), 6.400 (8.92), 6.433 (1.15), 6.912 (1.29), 7.048 (2.79), 7.184 (1.12), 7.612 (0.59), 7.966 (2.73), 8.358 (0.49), 8.365 (0.54), 8.474 (2.68), 8.477 (2.73), 8.479 (2.71), 8.653 (0.60), 8.658 (0.60), 9.927 (1.23).

### Example 532

### 2'-{6-amino-5-[(1S)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

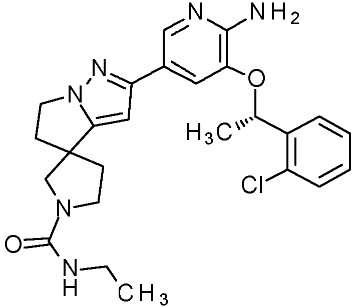

Example 532 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (40.8 mg, 107 µmol) and 3-[(1S)-1-(2-chlorophenyl)ethoxy]-5-(4,4,5,5-tetramethyl-1,3,2-dioxaborolan-2-yl)pyridin-2-amine (60.0 mg, 160 µmol).
1H-NMR (400 MHz, DMSO-d6): δ [ppm] = 7.85 (dd, 1H), 7.59 (dt, 1H), 7.46 (ddd, 1H), 7.39-7.32 (m, 1H), 7.32-7.26 (m, 1H), 7.08 (t, 1H), 6.17 (d, 1H), 6.16-6.12 (m, 1H), 5.94 (s, 2H), 5.81-5.72 (m, 1H), 4.24 (dd, 1H), 4.12 (t, 2H), 3.54-3.42 (m, 1H), 3.37 (d, 2H), 3.10-2.98 (m, 2H), 2.10-1.95 (m, 2H), 1.60 (d, 3H), 1.46-1.31 (m, 2H), 1.02 (td, 3H)
LC-MS (method 1): Rt = 1.08 min; MS (ESlneg): m/z = 479 [M-H]⁻

### Example 533

### 2'-{6-amino-5-[(1R)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

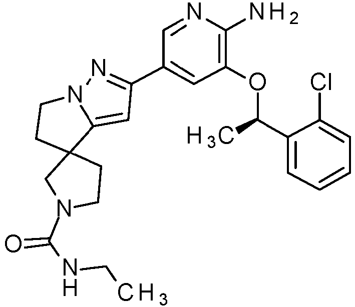

Example 533 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (40.8 mg, 107 µmol) and 3-[(1R)-1-(2-chlorophenyl)ethoxy]-5-(4,4,5,5-tetramethyl-1,3,2-dioxaborolan-2-yl)pyridin-2-amine (60.0 mg, 160 µmol).

1H-NMR (400 MHz, DMSO-d6): δ [ppm] = 7.85 (dd, 1H), 7.59 (dt, 1H), 7.46 (ddd, 1H), 7.39-7.32 (m, 1H), 7.32-7.26 (m, 1H), 7.08 (t, 1H), 6.17 (d, 1H), 6.16-6.12 (m, 1H), 5.94 (s, 2H), 5.81-5.72 (m, 1H), 4.24 (dd, 1H), 4.12 (t, 2H), 3.54-3.42 (m, 1H), 3.37 (d, 2H), 3.10-2.98 (m, 2H), 2.10-1.95 (m, 2H), 1.60 (d, 3H), 1.46-1.31 (m, 2H), 1.02 (td, 3H).

### Example 534

### (rac)-2'-{6-amino-5-[(3-methylphenyl)methoxy]pyridin-3-yl}-N-ethyl-5',6'-dihydrospiro[pyrrolidine-3,4'-pyrrolo[1,2-b]pyrazole]-1-carboxamide

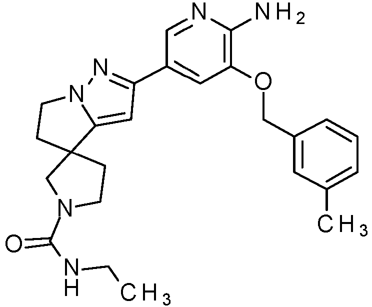

Example 534 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (24.7 mg, 64.6 µmol) and {6-amino-5-[(3-methylphenyl)methoxy]pyridin-3-yl}boronic acid (25.0 mg, 96.9 µmol).
LC-MS (method 1): Rt = 1.12 min; MS (ESlneg): m/z = 445 [M-H]⁻
¹H-NMR (400 MHz, DMSO-d6) δ [ppm]: 1.002 (3.03), 1.020 (7.27), 1.038 (3.20), 1.232 (0.97), 1.528 (16.00), 2.026 (0.51), 2.040 (0.77), 2.053 (1.05), 2.071 (0.45), 2.326 (9.15), 2.518 (4.59), 2.523 (3.21), 2.528 (1.41), 2.540 (0.47), 2.665 (0.73), 2.669 (1.00), 2.673 (0.69), 3.038 (1.19), 3.051 (1.30), 3.055 (1.25), 3.069 (1.16), 3.164 (2.14), 3.397 (5.21), 3.424 (1.03), 3.465 (0.53), 3.483 (0.70), 3.497 (0.51), 3.505 (0.44), 4.139 (1.05), 4.157 (1.68), 4.174 (0.99), 5.133 (4.01), 5.778 (2.49), 6.152 (0.46), 6.166 (0.92), 6.179 (0.43), 6.335 (4.72), 7.128 (0.68), 7.144 (0.74), 7.255 (0.43), 7.274 (1.48), 7.291 (2.52), 7.338 (1.44), 7.418 (1.80), 7.422 (1.77), 7.931 (2.62), 7.936 (2.53).

### Example 535

### (rac)-2'-{6-amino-5-[(1-methyl-1H-pyrazol-5-yl)methoxy]pyridin-3-yl}-N-ethyl-5',6'-dihydrospiro[pyrrolidine-3,4'-pyrrolo[1,2-b]pyrazole]-1-carboxamide

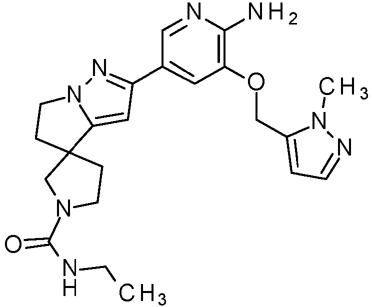

Example 535 was prepared in analogy to Example 461 using (*r*ac)-1-(ethylcarbamoyl)-5',6'-dihydrospiro[pyrrolidine-3,4'-pyrrolo[1,2-b]pyrazol]-2'-yl trifluoromethanesulfonate (108 mg, 283 µmol) and 3-[(1-methyl-1H-pyrazol-5-yl)methoxy]-5-(4,4,5,5-tetramethyl-1,3,2-dioxaborolan-2-yl)pyridin-2-amine (200 mg, 70 % purity, 424 µmol).
LC-MS (method 1): Rt = 0.75 min; MS (ESlpos): m/z = 437.5 [M+H]⁺
¹H-NMR (400 MHz, DMSO-d6) δ [ppm]: 1.003 (0.32), 1.021 (0.71), 1.039 (0.32), 1.232 (0.15), 1.313 (0.11), 1.328 (0.10), 2.051 (0.08), 2.064 (0.10), 2.331 (0.19), 2.337 (0.09), 2.518 (1.08), 2.523 (0.82), 2.540 (16.00), 2.673 (0.19), 2.678 (0.09), 3.039 (0.13), 3.053 (0.13), 3.057 (0.13), 3.070 (0.12), 3.407 (0.40), 3.825 (0.10), 3.870 (1.48), 3.882 (0.10), 4.153 (0.11), 4.171 (0.17), 4.188 (0.10), 5.227 (0.45), 5.787 (0.26), 6.159 (0.10), 6.364 (0.49), 6.426 (0.25), 6.431 (0.24), 7.376 (0.25), 7.381 (0.26), 7.507 (0.18), 7.512 (0.18), 7.955 (0.25), 7.959 (0.24).

### Example 536

### (rac)-2'-{6-amino-5-[(1-methyl-1H-pyrazol-4-yl)methoxy]pyridin-3-yl}-N-ethyl-5',6'-dihydrospiro[pyrrolidine-3,4'-pyrrolo[1,2-b]pyrazole]-1-carboxamide

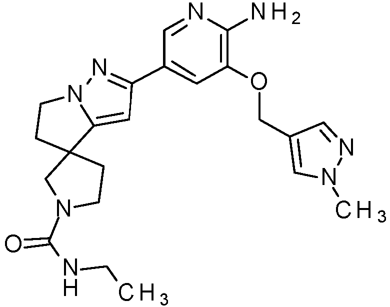

Example 536 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (38.6 mg, 101 µmol) and 3-[(1-methyl-1H-pyrazol-4-yl)methoxy]-5-(4,4,5,5-tetramethyl-1,3,2-dioxaborolan-2-yl)pyridin-2-amine (50.0 mg, 151 µmol).
LC-MS (method 1): Rt = 0.73 min; MS (ESlpos): m/z = 437 [M+H]⁺
¹H-NMR (400 MHz, DMSO-d6) δ [ppm]: 1.003 (3.67), 1.021 (8.95), 1.039 (3.91), 1.065 (0.61), 1.154 (1.30), 1.172 (2.38), 1.190 (1.17), 1.231 (0.51), 1.600 (2.84), 1.987 (4.60), 2.031 (0.60), 2.045 (0.93), 2.060 (1.24), 2.078 (0.53), 2.518 (2.92), 2.523 (1.39), 2.534 (1.35), 2.540 (2.31), 2.994 (0.56), 3.021 (0.45), 3.039 (1.41), 3.053 (1.57), 3.057 (1.50), 3.070 (1.40), 3.088 (0.42), 3.165 (0.63), 3.404 (5.68), 3.428 (0.85), 3.472 (0.50), 3.490 (0.73), 3.497 (0.48), 3.504 (0.53), 3.512 (0.44), 3.516 (0.44), 3.821 (16.00), 4.017 (1.03), 4.035 (1.02), 4.148 (1.27), 4.165 (2.06), 4.182 (1.21), 5.004 (5.35), 5.673 (2.97), 6.152 (0.57), 6.166 (1.14), 6.179 (0.56), 6.363 (5.61), 7.440 (2.16), 7.445 (2.18), 7.531 (3.53), 7.533 (3.64), 7.825 (3.35), 7.925 (3.29), 7.929 (3.23).

### Example 537

### (rac)-2'-{6-amino-5-[(1,5-dimethyl-1H-pyrazol-3-yl)methoxy]pyridin-3-yl}-N-ethyl-5',6'-dihydrospiro[pyrrolidine-3,4'-pyrrolo[1,2-b]pyrazole]-1-carboxamide

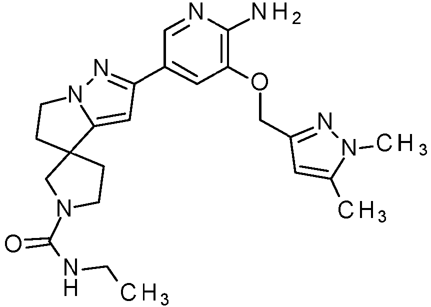

Example 537 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (68.1 mg, 178 µmol) and {6-amino-5-[(1,5-dimethyl-1H-pyrazol-3-yl)methoxy]pyridin-3-yl}boronic acid (70.0 mg, 267 µmol).
LC-MS (method 1): Rt = 0.80 min; MS (ESlpos): m/z = 451 [M+H]⁺
¹H-NMR (400 MHz, DMSO-d6) δ [ppm]: 1.003 (3.26), 1.021 (7.83), 1.038 (3.48), 2.033 (0.56), 2.040 (0.58), 2.049 (0.86), 2.058 (1.09), 2.065 (0.63), 2.076 (0.49), 2.227 (10.35), 2.518 (2.63), 2.523 (2.79), 2.539 (1.46), 3.020 (0.40), 3.038 (1.27), 3.052 (1.42), 3.056 (1.35), 3.070 (1.24), 3.390 (1.08), 3.403 (4.72), 3.415 (1.14), 3.433 (0.45), 3.483 (0.66), 3.497 (0.44), 3.693 (2.78), 3.696 (16.00), 4.154 (1.12), 4.172 (1.79), 4.189 (1.08), 5.000 (0.59), 5.024 (4.74), 5.999 (0.40), 6.156 (3.27), 6.175 (0.52), 6.366 (4.62), 7.541 (1.74), 7.546 (1.75), 7.911 (2.68), 7.915 (2.53).

### Example 538

### (rac)-2'-{6-amino-5-[(4-methylphenyl)methoxy]pyridin-3-yl}-N-ethyl-5',6'-dihydrospiro[pyrrolidine-3,4'-pyrrolo[1,2-b]pyrazole]-1-carboxamide

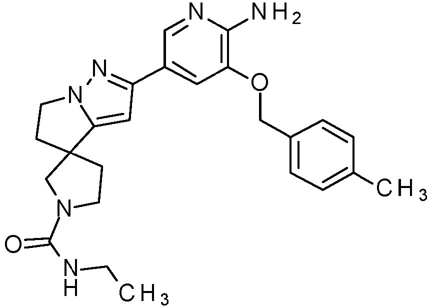

Example 538 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (49.4 mg, 129 µmol) and {6-amino-5-[(4-methylphenyl)methoxy]pyridin-3-yl}boronic acid (50.0 mg, 194 µmol).
LC-MS (method 1): Rt = 1.01 min; MS (ESlpos): m/z = 447.5 [M+H]⁺
¹H-NMR (400 MHz, DMSO-d6) δ [ppm]: 1.003 (6.34), 1.021 (14.07), 1.038 (6.44), 2.024 (1.24), 2.039 (1.96), 2.052 (2.52), 2.070 (1.09), 2.302 (16.00), 2.523 (2.50), 2.527 (2.91), 2.539 (8.33), 3.020 (0.86), 3.038 (2.69), 3.053 (3.06), 3.056 (3.01), 3.071 (2.63), 3.088 (0.79), 3.371 (1.28), 3.380 (1.87), 3.397 (9.63), 3.423 (1.08), 3.465 (0.76), 3.483 (1.31), 3.490 (0.84), 3.497 (0.96), 3.508 (0.79), 4.138 (2.46), 4.155 (4.12), 4.173 (2.36), 5.131 (8.71), 5.784 (3.44), 6.142 (1.15), 6.156 (2.29), 6.169 (1.14), 6.337 (8.95), 7.188 (4.44), 7.207 (5.38), 7.385 (5.91), 7.404 (4.91), 7.418 (4.21), 7.423 (4.31), 7.924 (5.19), 7.928 (5.13).

### Example 539

### 2'-{6-amino-5-[(1R)-1-(3-methylphenyl)ethoxy]pyridin-3-yl}-N-ethyl-5',6'-dihydrospiro[pyrrolidine-3,4'-pyrrolo[1,2-b]pyrazole]-1-carboxamide (mixture of stereoisomers)

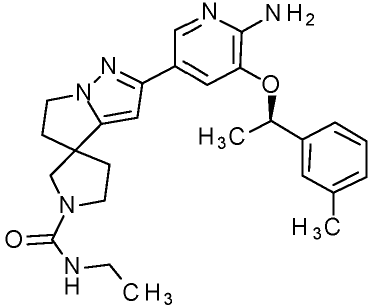

Example 539 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (65.6 mg, 171 µmol) and {6-amino-5-[(1R)-1-(3-methylphenyl)ethoxy]pyridin-3-yl}boronic acid (70.0 mg, 257 µmol).
LC-MS (method 1): Rt = 1.04 min; MS (ESlpos): m/z = 461 [M+H]⁺
¹H-NMR (400 MHz, DMSO-d6) δ [ppm]: 0.997 (4.29), 1.001 (4.38), 1.015 (9.70), 1.019 (9.76), 1.033 (4.51), 1.037 (4.48), 1.066 (5.58), 1.233 (0.72), 1.545 (9.96), 1.561 (9.95), 2.028 (2.41), 2.073 (0.64), 2.283 (16.00), 2.465 (3.14), 2.518 (8.35), 2.523 (5.65), 2.539 (0.83), 3.018 (0.67), 3.032 (2.37), 3.050 (3.43), 3.067 (2.24), 3.081 (0.61), 3.294 (0.45), 3.304 (1.18), 3.372 (9.11), 3.444 (0.81), 3.463 (1.33), 3.476 (1.04), 3.502 (0.49), 3.941 (0.92), 4.105 (2.76), 4.123 (4.75), 4.140 (2.67), 5.500 (0.66), 5.515 (2.33), 5.531 (2.31), 5.547 (0.68), 5.817 (6.84), 6.131 (1.23), 6.145 (2.41), 6.159 (1.19), 6.217 (6.22), 6.224 (5.85), 7.048 (1.72), 7.064 (2.03), 7.198 (1.04), 7.217 (3.21), 7.234 (8.13), 7.255 (0.89), 7.284 (3.89), 7.840 (3.57), 7.844 (5.09), 7.848 (3.56).

### Example 540

### (rac)-2'-{6-amino-5-[(propan-2-yl)oxy]pyridin-3-yl}-N-ethyl-5',6'-dihydrospiro[pyrrolidine-3,4'-pyrrolo[1,2-b]pyrazole]-1-carboxamide

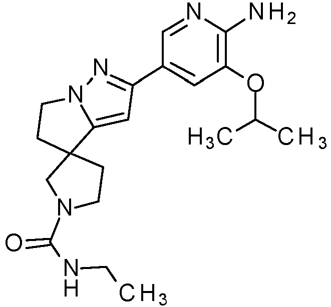

Example 540 was prepared in analogy to Example 461 using (*rac*)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-[(propan-2-yl)oxy]-5-(4,4,5,5-tetramethyl-1,3,2-dioxaborolan-2-yl)pyridin-2-amine (69.9 mg, 78 % purity, 196 µmol).
¹H-NMR (400MHz, DMSO-d6): δ [ppm]= 1.02 (t, 3H), 1.29 (d, 6H), 1.99 - 2.11 (m, 2H), 2.52 - 2.55 (m, 2H), 3.00 - 3.10 (m, 2H), 3.40 (s, 3H), 3.45 - 3.55 (m, 1H), 4.16 (t, 2H), 4.61 (dt, 1H), 5.65 (s, 2H), 6.15 (t, 1H), 6.34 (s, 1H), 7.33 (d, 1H), 7.90 (d, 1H).
LC-MS (method 1): Rt = 0.86 min; MS (ESlpos): m/z = 385 [M+H]⁺

### Example 541

### (3R)-2'-{6-amino-5-[(1S)-1-(1-methyl-1H-pyrazol-3-yl)ethoxy]pyridin-3-yl}-N-ethyl-5',6'-dihydrospiro[pyrrolidine-3,4'-pyrrolo[1,2-b]pyrazole]-1-carboxamide

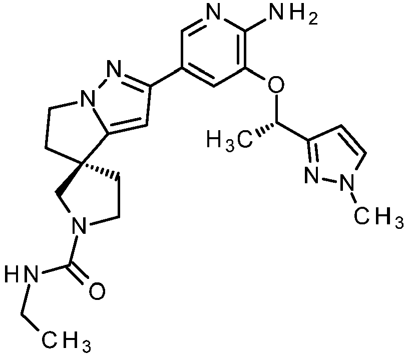

Example 541 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (65.7 mg, 172 µmol) and {6-amino-5-[(1S)-1-(1-methyl-1H-pyrazol-3-yl)ethoxy]pyridin-3-yl}boronic acid (225 mg, 20 % purity, 172 µmol).
LC-MS (method 1): Rt = 0.78 min; MS (ESlpos): m/z = 452 [M+H]⁺
1H-NMR (400MHz, CHLOROFORM-d): δ [ppm]= 1.16 (t, 3H), 1.71 (d, 3H), 2.01 - 2.10 (m, 1H), 2.22 (dt, 1H), 2.48 - 2.57 (m, 1H), 2.58 - 2.68 (m, 1H), 3.30 (qd, 2H), 3.47 - 3.57 (m, 3H), 3.58 - 3.67 (m, 1H), 3.86 (s, 3H), 4.23 (t, 3H), 4.78 (s, 2H), 5.50 (d, 1H), 6.12 (s, 1H), 6.22 (d, 1H), 7.49 (d, 1H), 8.02 (d, 1H).
[α]²⁰_{D} : -39.29° (c = 1.00, MeOH)

### Example 542

### (3R)-2'-{6-amino-5-[(1R)-1-(1-methyl-1H-pyrazol-3-yl)ethoxy]pyridin-3-yl}-N-ethyl-5',6'-dihydrospiro[pyrrolidine-3,4'-pyrrolo[1,2-b]pyrazole]-1-carboxamide

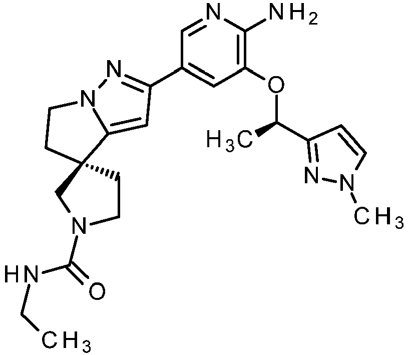

Example 542 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (62.2 mg, 163 µmol) and {6-amino-5-[(1R)-1-(1-methyl-1H-pyrazol-3-yl)ethoxy]pyridin-3-yl}boronic acid (213 mg, 20 % purity, 163 µmol).
LC-MS (method 1): Rt = 0.78 min; MS (ESlpos): m/z = 452 [M+H]⁺
1H-NMR (400MHz, CHLOROFORM-d): δ [ppm]= 1.16 (t, 3H), 1.71 (d, 3H), 2.01 - 2.10 (m, 1H), 2.22 (dt, 1H), 2.48 - 2.57 (m, 1H), 2.58 - 2.68 (m, 1H), 3.30 (qd, 2H), 3.47 - 3.57 (m, 3H), 3.58 - 3.67 (m, 1H), 3.86 (s, 3H), 4.23 (t, 3H), 4.78 (s, 2H), 5.50 (d, 1H), 6.12 (s, 1H), 6.22 (d, 1H), 7.49 (d, 1H), 8.02 (d, 1H).
[α]²⁰_{D} : +145.6° (c = 1.00, MeOH)

### Example 543

### (3R)-2'-(6-amino-5-{[1-(3,5-difluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (stereoisomer 1)

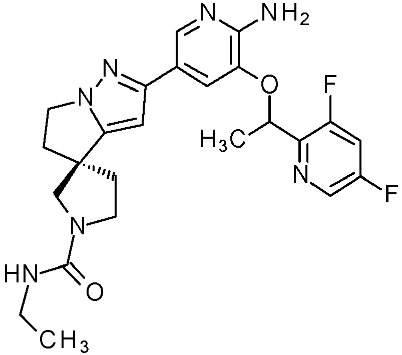

Example 543 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and (6-amino-5-{[1-(3,5-difluoropyridin-2-yl)ethyl]oxy}pyridin-3-yl)boronic acid (223 mg, 35 % purity, 265 µmol, stereoisomer 1).
1H-NMR (400MHz, CHLOROFORM-d): δ [ppm]= 1.17 (t, 3H), 1.76 (d, 3H), 2.03 - 2.12 (m, 1H), 2.23 (dt, 1H), 2.49 - 2.59 (m, 1H), 2.59 - 2.69 (m, 1H), 3.32 (qd, 2H), 3.47 - 3.59 (m, 3H), 3.59 - 3.68 (m, 1H), 4.12 - 4.21 (m, 1H), 4.25 (t, 2H), 4.80 (s, 2H), 5.78 (d, 1H), 6.11 (s, 1H), 7.15 - 7.23 (m, 1H), 7.43 (d, 1H), 8.03 (d, 1H), 8.34 (d, 1H).
[α]²⁰_{D} : -71.63° (c = 1.00, MeOH)
LC-MS (method 1): Rt = 0.90 min; MS (ESlneg): m/z = 482 [M-H]⁻

### Example 544

### (3R)-2'-(6-amino-5-{[1-(3,5-difluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (stereoisomer 2)

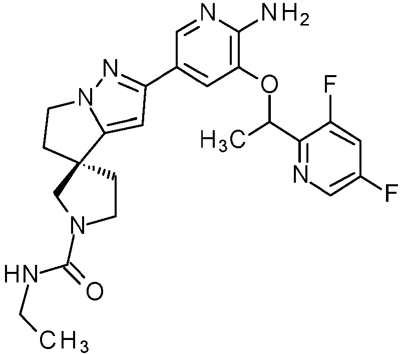

Example 544 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and (6-amino-5-{[1-(3,5-difluoropyridin-2-yl)ethyl]oxy}pyridin-3-yl)boronic acid (231 mg, 35 % purity, 275 µmol, stereoisomer 2).
LC-MS (method 1): Rt = 0.89 min; MS (ESlpos): m/z = 485 [M+H]⁺
1H-NMR (400MHz, CHLOROFORM-d): δ [ppm]= 1.17 (t, 3H), 1.76 (d, 3H), 2.08 (s, 1H), 2.19 - 2.29 (m, 1H), 2.50 - 2.59 (m, 1H), 2.59 - 2.69 (m, 1H), 3.31 (dd, 2H), 3.47 - 3.59 (m, 3H), 3.59 - 3.68 (m, 1H), 4.12 - 4.20 (m, 1H), 4.25 (t, 2H), 4.80 (s, 2H), 5.78 (d, 1H), 6.11 (s, 1H), 7.19 (br d, 1H), 7.44 (d, 1H), 8.02 (d, 1H), 8.34 (d, 1H).
[α]²⁰_{D} : +162.73° (c = 1.00, MeOH)

### Example 545

### (3R)-2'-{6-amino-5-[(1R)-1-(2-fluorophenyl)ethoxy]pyridin-3-yl}-N-ethyl-5',6'-dihydrospiro[pyrrolidine-3,4'-pyrrolo[1,2-b]pyrazole]-1-carboxamide

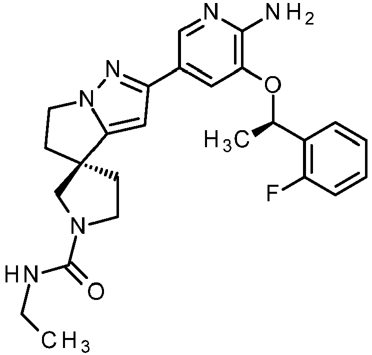

Example 545 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-[(1R)-1-(2-fluorophenyl)ethoxy]-5-(4,4,5,5-tetramethyl-1,3,2-dioxaborolan-2-yl)pyridin-2-amine (229 mg, 43 % purity, 275 µmol).
LC-MS (method 1): Rt = 1.00 min; MS (ESlpos): m/z = 466 [M+H]⁺
1H-NMR (400MHz, CHLOROFORM-d): δ [ppm]= 1.16 (t, 3H), 1.69 (d, 3H), 2.01 - 2.11 (m, 1H), 2.21 (s, 1H), 2.48 - 2.57 (m, 1H), 2.57 - 2.67 (m, 1H), 3.31 (dd, 2H), 3.46 - 3.57 (m, 3H), 3.58 - 3.67 (m, 1H), 4.15 (s, 1H), 4.23 (t, 2H), 4.77 (s, 2H), 5.76 (q, 1H), 6.07 (s, 1H), 7.01 - 7.16 (m, 2H), 7.21 - 7.25 (m, 1H), 7.27 - 7.29 (m, 1H), 7.41 (d, 1H), 8.01 (d, 1H).
[α]²⁰_{D} : +156.09° (c = 1.00, MeOH)

### Example 546

### (3R)-2'-{6-amino-5-[(1S)-1-(2-fluorophenyl)ethoxy]pyridin-3-yl}-N-ethyl-5',6'-dihydrospiro[pyrrolidine-3,4'-pyrrolo[1,2-b]pyrazole]-1-carboxamide

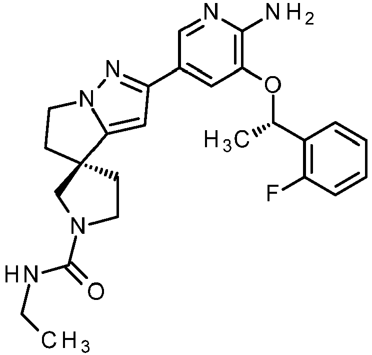

Example 546 was prepared in analogy to Example 461 using (3R)-1-(ethylcarbamoyl)-5',6'-dihydrospiro[pyrrolidine-3,4'-pyrrolo[1,2-b]pyrazol]-2'-yl trifluoromethanesulfonate (75.0 mg, 196 µmol) and 3-[(1S)-1-(2-fluorophenyl)ethoxy]-5-(4,4,5,5-tetramethyl-1,3,2-dioxaborolan-2-yl)pyridin-2-amine (219 mg, 45 % purity, 275 µmol).
LC-MS (method 1): Rt = 1.00 min; MS (ESlpos): m/z = 466 [M+H]⁺
1H-NMR (400MHz, CHLOROFORM-d): δ [ppm]= 1.17 (t, 3H), 1.69 (d, 3H), 2.00 - 2.10 (m, 1H), 2.17 - 2.28 (m, 1H), 2.54 (s, 1H), 2.60 (s, 1H), 3.26 - 3.37 (m, 2H), 3.46 - 3.57 (m, 3H), 3.58 - 3.67 (m, 1H), 4.12 - 4.19 (m, 1H), 4.23 (t, 2H), 4.77 (s, 2H), 5.76 (d, 1H), 6.07 (s, 1H), 7.11 (d, 2H), 7.21 - 7.25 (m, 1H), 7.27 (s, 1H), 7.41 (d, 1H), 8.01 (d, 1H).
[α]²⁰_{D} : -44.34° (c = 1.00, MeOH)

### Example 547

### (rac)-2'-(5-acetyl-6-aminopyridin-3-yl)-N-(1-phenylcyclobutyl)-5',6'-dihydrospiro[pyrrolidine-3,4'-pyrrolo[1,2-b]pyrazole]-1-carboxamide

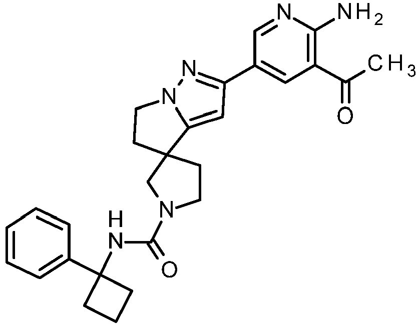

*(rac)-1-[(1-phenylcyclobutyl)carbamoyl]-5',6'-dihydrospiro[pyrrolidine-3,4'-pyrrolo[1,2-*b]pyrazol]-2'-yl trifluoromethanesulfonate (194 mg, 400 µmol), (5-acetyl-6-aminopyridin-3-yl)boronic acid (82.8 mg, 460 µmol), XPhos Pd G2 (47.2 mg, 60.0 µmol) and potassium phosphate (2.4 mL, 0.50 M, 1.2 mmol) were solubilised in dioxane (4.0 mL) and the mixture was stirred for 1h at 100°C. The mixture was diluted with water and extracted three times with ethyl acetate. The combined organic layers were dried and evaporated. The residue was purified by preparative HPLC to give 27.5 mg (97 % purity, 14 % yield) of the title compound.

1H-NMR (400 MHz, DMSO-d6) δ [ppm]: 1.036 (1.47), 1.053 (2.85), 1.071 (1.56), 1.764 (0.45), 1.769 (0.47), 1.786 (0.49), 1.791 (0.56), 1.982 (0.53), 1.997 (0.41), 2.005 (0.50), 2.009 (0.51), 2.026 (0.41), 2.032 (0.44), 2.045 (0.71), 2.059 (1.09), 2.072 (1.24), 2.089 (0.56), 2.359 (0.40), 2.382 (0.86), 2.390 (1.00), 2.397 (0.83), 2.405 (1.22), 2.411 (0.97), 2.427 (0.71), 2.452 (0.70), 2.475 (1.30), 2.518 (2.01), 2.523 (1.96), 2.537 (1.90), 2.542 (1.85), 2.558 (1.18), 2.596 (16.00), 3.160 (3.62), 3.172 (3.72), 3.418 (0.85), 3.423 (1.03), 3.447 (3.80), 3.535 (0.65), 3.551 (0.49), 3.560 (0.45), 4.101 (0.67), 4.114 (0.66), 4.181 (1.46), 4.198 (2.81), 4.215 (1.43), 6.463 (4.93), 6.704 (2.38), 7.134 (0.71), 7.137 (0.43), 7.152 (1.77), 7.167 (0.72), 7.170 (1.19), 7.173 (0.69), 7.262 (1.98), 7.282 (3.33), 7.296 (0.73), 7.301 (1.92), 7.421 (2.89), 7.423 (3.12), 7.441 (2.60), 7.445 (2.01), 8.381 (2.64), 8.387 (2.78), 8.597

### Example 548

### (3R)-2'-[6-amino-5-(trifluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide

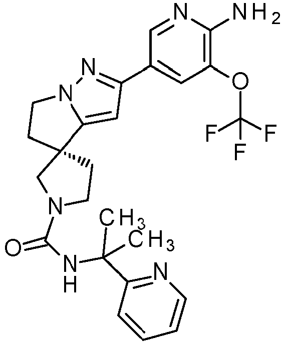

To a solution of 5-(4,4,5,5-tetramethyl-1,3,2-dioxaborolan-2-yl)-3-(trifluoromethoxy)pyridin-2-amine (116 mg, 382 µmol) in Dioxane (4 mL) under Argon, (3R)-1-{[2-(pyridin-2-yl)propan-2-yl]carbamoyl}-5',6'-dihydrospiro[pyrrolidine-3,4'-pyrrolo[1,2-b]pyrazol]-2'-yl trifluoromethanesulfonate (190 mg, 401 µmol) and Potassium phosphate (2.3 ml, 0.50 M in water) were added. The mixture was degassed and XPhos Pd G2 (15.0 mg, 19.1 µmol) was added. The reaction mixture was stirred at 100°C under Argon for 1h. The mixture was diluted with water and then extracted with Ethyl acetate 2x. The combined organics were washed with Brine, dried over Sodium sulfate, filtered and concentrated. The crude material was purified by basic to give 41.0 mg (99 % purity, 21 % yield) of the title compound.
1H NMR (DMSO-d6, 400 MHz) δ 8.4-8.5 (m, 1H), 8.34 (d, 1H), 7.76 (t, 1H), 7.7-7.7 (m, 1H), 7.4-7.5 (m, 1H), 7.18 (ddd, 1H), 6.51 (s, 2H), 6.4-6.5 (m, 2H), 4.19 (t, 2H), 3.5-3.6 (m, 1H), 3.4-3.5 (m, 3H), 2.55 (t, 2H), 2.0-2.2 (m, 2H), 1.59 (s, 6H)
LC-MS (method 1): Rt = 1.02 min; MS (ESlpos): m/z = 503 [M+H]⁺

### Example 549

### (3'R)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide

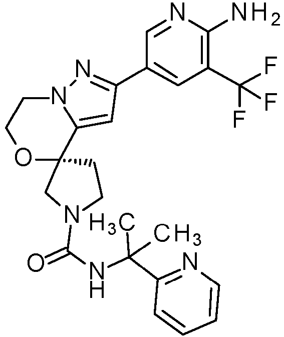

To a solution of 5-(4,4,5,5-tetramethyl-1,3,2-dioxaborolan-2-yl)-3-(trifluoromethyl)pyridin-2-amine (104 mg, 362 µmol) in Dioxane (4 mL), (3'R)-2-bromo-N-[2-(pyridin-2-yl)propan-2-yl]-6,7-dihydrospiro[pyrazolo[5,1-c][1,4]oxazine-4,3'-pyrrolidine]-1'-carboxamide (190 mg, 452 µmol) and Potassium phosphate (2.2 ml, 0.50 M in water) were added. The mixture was degassed and XPhos Pd G2 (14.2 mg, 18.1 µmol) was added. The resultant mixture was stirred at 100°C under Argon for 1h. The mixture was diluted with water and then extracted with Ethyl acetate 2x. The combined organics were washed with Brine, dried over Sodium sulfate, filtered and concentrated in vacuo. The crude was purified by basic HPLC to give 49.0 mg (99 % purity, 27 % yield) of the title compound.
1H NMR (DMSO-d6, 400 MHz) δ 8.59 (d, 1H), 8.4-8.5 (m, 1H), 8.02 (d, 1H), 7.72 (dt, 1H), 7.4-7.5 (m, 1H), 7.19 (ddd, 1H), 6.78 (s, 1H), 6.60 (s, 2H), 6.43 (s, 1H), 4.1-4.2 (m, 4H), 3.82 (d, 1H), 3.63 (br t, 1H), 3.52 (br d, 1H), 3.4-3.5 (m, 1H), 2.2-2.4 (m, 2H), 1.58 (d, 6H)
LC-MS (method 1): Rt = 1.01 min; MS (ESlpos): m/z = 503 [M+H]⁺

### Example 550

### (3R)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide

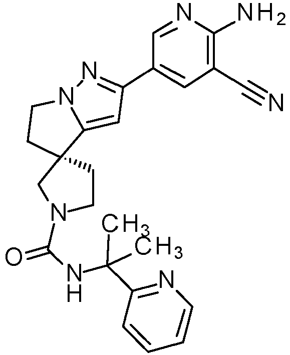

To a solution of 2-amino-5-(4,4,5,5-tetramethyl-1,3,2-dioxaborolan-2-yl)pyridine-3-carbonitrile (78.7 mg, 321 µmol) in Dioxane (3.5 mL), (3R)-1-{[2-(pyridin-2-yl)propan-2-yl]carbamoyl}-5',6'-dihydrospiro[pyrrolidine-3,4'-pyrrolo[1,2-b]pyrazol]-2'-yl trifluoromethanesulfonate (190 mg, 401 µmol) and Potassium phosphate (1.9 ml, 0.50 M in water) were added. The mixture was degassed and XPhos Pd G2 (12.6 mg, 16.1 µmol)was added. The resultant mixture was stirred at 100°C under Argon for 1h. The mixture was diluted with water and then extracted with Ethyl acetate 2x. The combined organics were washed with Brine, dried over Sodium sulfate, filtered and concentrated in vacuo. The crude was purified by basic HPLC to give 45.0 mg (99 % purity, 31 % yield) of the title compound.
1H NMR (DMSO-d6, 400 MHz) δ 8.62 (d, 1H), 8.4-8.5 (m, 1H), 8.15 (d, 1H), 7.71 (dt, 1H), 7.47 (d, 1H), 7.19 (ddd, 1H), 7.00 (s, 2H), 6.47 (s, 1H), 6.43 (s, 1H), 4.20 (t, 2H), 3.5-3.6 (m, 1H), 3.4-3.5 (m, 3H), 2.56 (t, 2H), 2.0-2.1 (m, 2H), 1.59 (s, 6H)
LC-MS (method 1): Rt = 0.89 min; MS (ESlpos): m/z = 444 [M+H]⁺

### Example 551

### (3'R)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide

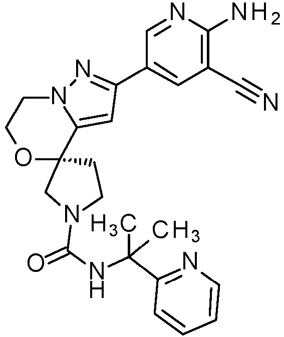

To a solution of 2-amino-5-(4,4,5,5-tetramethyl-1,3,2-dioxaborolan-2-yl)pyridine-3-carbonitrile (88.6 mg, 362 µmol) in Dioxane (4 mL), (3'R)-2-bromo-N-[2-(pyridin-2-yl)propan-2-yl]-6,7-dihydrospiro[pyrazolo[5,1-c][1,4]oxazine-4,3'-pyrrolidine]-1'-carboxamide (190 mg, 452 µmol) and Potassium phosphate (2.2 ml, 0.50 M in water) were added. The mixture was degassed and XPhos Pd G2 (14.2 mg, 18.1 µmol)was added. The resultant mixture was stirred at 100°C under Argon for 1h. The mixture was diluted with water and then extracted with Ethyl acetate 2x. The combined organics were washed with Brine, dried over Sodium sulfate, filtered and concentrated in vacuo. The crude was purified by acidic HPLC. The compound was basified by dissolved in Ethyl acetate and washed with std. NaHCO3-solution. The organics were dried over sodium sulfate, filtered and concentrated to give 57.0 mg (99 % purity, 34 % yield) of the title compound.
1H NMR (DMSO-d6, 400 MHz) δ 8.61 (d, 1H), 8.4-8.5 (m, 1H), 8.13 (d, 1H), 7.72 (dt, 1H), 7.4-7.5 (m, 1H), 7.19 (ddd, 1H), 7.05 (s, 2H), 6.73 (s, 1H), 6.43 (s, 1H), 4.1-4.2 (m, 4H), 3.81 (br d, 1H), 3.63 (br t, 1H), 3.4-3.5 (m, 2H), 2.3-2.4 (m, 1H), 2.1-2.3 (m, 1H), 1.58 (d, 6H)
LC-MS (method 1): Rt = 0.88 min; MS (ESlpos): m/z = 460 [M+H]⁺

### Example 552

### (rac)-2'-[6-amino-5-(phenylcarbamoyl)pyridin-3-yl]-N-ethyl-5',6'-dihydrospiro[pyrrolidine-3,4'-pyrrolo[1,2-b]pyrazole]-1-carboxamide

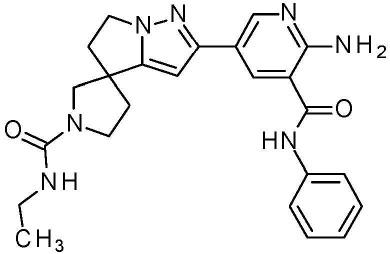

2-amino-5-[(rac)-1-(ethylcarbamoyl)-5',6'-dihydrospiro[pyrrolidine-3,4'-pyrrolo[1,2-b]pyrazol]-2'-yl]pyridine-3-carboxylic acid (50.0 mg, 135 µmol) was solubilised in THF (1000 µL), aniline (15.1 mg, 162 µmol), HATU (77.0 mg, 202 µmol) and triethylamine (47 µL, 340 µmol) were added and the mixture was stirred for 6 h at rt. aniline (15.1 mg, 162 µmol) and HATU (77.0 mg, 202 µmol) were added and the mixture was stirred overnight at rt. The mixture was diluted with water and extracted 3x with ethyl acetate. The combined organic layers were dried and evaporated. The residue was purified by prepaprative HPLC to give 35.0 mg (100 % purity, 58 % yield) of the title compound.
LC-MS (Method 1): Rt = 0.95 min; MS (ESlpos): m/z = 446 [M+H]⁺
¹H-NMR (400 MHz, DMSO-d6) δ [ppm]: 1.002 (7.06), 1.020 (16.00), 1.038 (7.15), 1.243 (0.85), 1.258 (0.88), 1.263 (0.87), 1.280 (0.72), 2.050 (1.34), 2.054 (1.37), 2.066 (2.16), 2.074 (2.80), 2.083 (1.28), 2.090 (1.20), 2.518 (1.18), 2.524 (2.63), 2.542 (3.84), 2.559 (2.55), 3.021 (0.88), 3.039 (2.76), 3.053 (3.04), 3.057 (2.99), 3.070 (2.70), 3.088 (0.82), 3.382 (1.64), 3.391 (1.42), 3.400 (1.84), 3.418 (8.20), 3.445 (1.21), 3.473 (0.83), 3.490 (1.49), 3.498 (0.81), 3.506 (0.96), 3.515 (0.89), 4.177 (2.48), 4.195 (3.89), 4.212 (2.37), 6.159 (1.15), 6.173 (2.27), 6.186 (1.10), 6.446 (11.17), 7.070 (3.42), 7.092 (1.22), 7.094 (1.77), 7.098 (1.08), 7.113 (3.27), 7.129 (1.20), 7.131 (1.90), 7.134 (1.15), 7.336 (3.83), 7.341 (1.38), 7.357 (5.04), 7.371 (1.20), 7.376 (3.52), 7.690 (4.59), 7.692 (5.23), 7.707 (1.45), 7.711 (4.84), 7.714 (3.75), 8.354 (4.30), 8.360 (4.59), 8.545 (6.17), 8.551 (5.58), 10.318 (4.08).

### Example 553

### (rac)-2'-[6-amino-5-(cyclohexylcarbamoyl)pyridin-3-yl]-N-ethyl-5',6'-dihydrospiro[pyrrolidine-3,4'-pyrrolo[1,2-b]pyrazole]-1-carboxamide

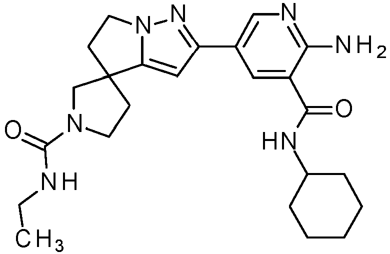

2-amino-5-[(*rac*)-1-(ethylcarbamoyl)-5',6'-dihydrospiro[pyrrolidine-3,4'-pyrrolo[1,2-b]pyrazol]-2'-yl]pyridine-3-carboxylic acid (75.0 mg, 202 µmol) was solubilised in THF (1.5 mL), cyclohexanamine (28 µL, 240 µmol), HATU (115 mg, 304 µmol) and triethylamine (71 µL, 510 µmol) were added and the mixture was stirred for 3 h at rt. cyclohexanamine (28 µL, 240 µmol) and N-methylpyrrolidone (250 µL, 2.6 mmol) were added and the mixture was stirred overnight at rt. cyclohexanamine (28 µL, 240 µmol), HATU (115 mg, 304 µmol) and triethylamine (71 µL, 510 µmol) were added and the mixture was stirred at 80°C. The mixture was diluted with water and extracted 3x with ethyl acetate. The combined organic layers were dried and evaporated. The residue was purified by prepaprative HPLC to give 2.00 mg (98 % purity, 2 % yield) of the title compound.
LC-MS (Method 1): Rt = 0.98 min; MS (ESlpos): m/z = 452 [M+H]⁺
¹H-NMR (400 MHz, DMSO-d6) δ [ppm]: 1.004 (7.08), 1.022 (16.00), 1.040 (7.13), 1.111 (0.55), 1.133 (0.65), 1.242 (0.40), 1.275 (1.25), 1.296 (2.69), 1.308 (3.19), 1.337 (1.50), 1.370 (0.40), 1.600 (0.90), 1.630 (0.80), 1.734 (1.50), 1.746 (1.84), 1.806 (2.04), 2.042 (1.20), 2.057 (1.89), 2.069 (2.44), 2.073 (2.59), 2.084 (3.34), 2.099 (0.45), 2.332 (0.60), 2.518 (4.49), 2.523 (2.99), 2.532 (4.24), 2.539 (2.64), 2.550 (2.74), 3.022 (0.85), 3.039 (2.84), 3.053 (3.09), 3.057 (3.04), 3.071 (2.79), 3.089 (0.80), 3.377 (0.85), 3.383 (1.10), 3.395 (1.50), 3.408 (6.48), 3.413 (6.38), 3.420 (2.54), 3.438 (1.35), 3.464 (0.75), 3.482 (1.40), 3.489 (0.80), 3.497 (0.95), 3.507 (0.80), 3.523 (0.45), 3.739 (0.65), 3.748 (0.70), 3.757 (0.70), 4.164 (2.49), 4.181 (3.99), 4.199 (2.44), 6.154 (1.15), 6.168 (2.29), 6.182 (1.15), 6.375 (11.51), 7.079 (4.88), 8.167 (3.99), 8.172 (4.24), 8.338 (1.89), 8.358 (1.84), 8.458 (5.43), 8.463 (5.33).

### Example 554

### (rac)-2'-[6-amino-5-(diethylcarbamoyl)pyridin-3-yl]-N-ethyl-5',6'-dihydrospiro[pyrrolidine-3,4'-pyrrolo[1,2-b]pyrazole]-1-carboxamide

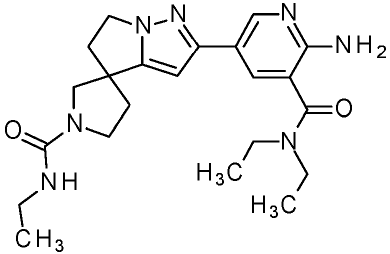

2-amino-5-[(*rac*)-1-(ethylcarbamoyl)-5',6'-dihydrospiro[pyrrolidine-3,4'-pyrrolo[1,2-b]pyrazol]-2'-yl]pyridine-3-carboxylic acid (100 mg, 270 µmol) was solubilised in THF (2.0 mL), N-ethylethanamine (34 µL, 320 µmol), HATU (154 mg, 405 µmol), N-methylpyrrolidone (330 µL, 3.5 mmol) and triethylamine (94 µL, 670 µmol) were added and the mixture was stirred for 3 h at 80°C. The mixture was diluted with water and extracted 3x with ethyl acetate. The combined organic layers were dried and evaporated. The residue was purified by prepaprative HPLC to give 2.80 mg (100 % purity, 2 % yield) of the title compound.
LC-MS (Method 1): Rt = 0.80 min; MS (ESlpos): m/z = 426 [M+H]⁺
¹H-NMR (600 MHz, DMSO-d6) δ [ppm]: 1.006 (7.73), 1.018 (16.00), 1.030 (7.69), 1.066 (1.15), 1.099 (3.29), 1.599 (0.81), 2.030 (1.43), 2.042 (1.75), 2.051 (1.33), 2.058 (2.29), 2.070 (1.20), 2.078 (0.83), 2.513 (4.64), 2.515 (4.73), 2.525 (3.16), 2.539 (0.74), 3.031 (1.09), 3.042 (3.46), 3.052 (3.57), 3.054 (3.73), 3.064 (3.01), 3.075 (0.97), 3.289 (0.87), 3.301 (1.06), 3.365 (2.59), 3.378 (2.71), 3.383 (2.42), 3.391 (2.00), 3.396 (3.71), 3.401 (11.35), 3.418 (0.84), 3.478 (0.95), 3.487 (1.16), 3.491 (1.41), 3.495 (1.12), 3.500 (1.22), 3.504 (1.06), 3.507 (1.00), 3.517 (0.73), 4.152 (3.22), 4.165 (5.58), 4.176 (3.22), 5.857 (1.20), 5.866 (7.02), 6.139 (1.24), 6.149 (2.38), 6.158 (1.25), 6.386 (10.60), 7.647 (4.93), 7.650 (5.20), 8.310 (6.02), 8.393 (5.42), 8.397 (5.44).

### Example 555

### (rac)-2'-(5-acetyl-6-aminopyridin-3-yl)-N-ethyl-5',6'-dihydrospiro[pyrrolidine-3,4'-pyrrolo[1,2-b]pyrazole]-1-carboxamide

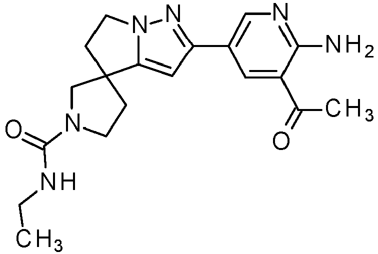

*(rac)-1-(ethylcarbamoyl)-5',6'-dihydrospiro[pyrrolidine-3,4'-pyrrolo[1,2-b]pyrazol]-2'-yl* trifluoromethanesulfonate (100 mg, 262 µmol), 5-acetyl-6-aminopyridin-3-yl)boronic acid (51 mg, 288 µmol), Na2CO3 (83.2 mg, 785 µmol) and Pd(dppf)2Cl2 (3.83 mg, 5.23 µmol) were stirred in a mixture of 1,2-dimethoxyethane (700 µl), ethanol (300 µl) and water (200 µl) under argon for 1 h at 120°C. The mixture was diluted with brine, extracted with ethyl acetate and the organic layer was dried over a silicone filter and concentrated under reduced pressure. The residue was purified by preparative HPLC to give 11.2 mg (97 % purity, 12 % yield) of the title compound.
LC-MS (Method 1): Rt = 0.74 min; MS (ESlpos): m/z = 369 [M+H]⁺
¹H-NMR (400 MHz, DMSO-d6) δ [ppm]: 1.004 (4.21), 1.022 (10.02), 1.040 (4.50), 2.048 (1.05), 2.066 (2.10), 2.083 (1.05), 2.523 (10.62), 2.541 (4.28), 2.560 (2.44), 2.605 (0.84), 2.619 (16.00), 3.041 (1.12), 3.054 (1.27), 3.071 (1.15), 3.388 (1.29), 3.414 (5.12), 3.417 (4.90), 3.430 (2.77), 3.447 (2.56), 4.182 (1.58), 4.200 (2.58), 4.217 (1.65), 6.167 (0.72), 6.525 (6.03), 6.618 (0.22), 7.856 (0.24), 8.470 (1.89), 8.476 (2.10), 8.616 (3.73), 8.622 (3.59).

### Example 556

### 5-{1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

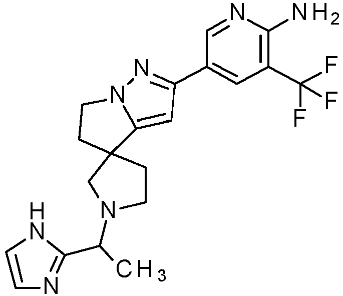

5-[5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 84 % purity, 233 µmol, stereoisomer 1), 1-(1H-imidazol-2-yl)ethan-1-one (38.6 mg, 350 µmol), titanium(IV) isopropoxide (210 µL, 700 µmol) and N,N-diisopropylethylamine (160 µL) were stirred in methanol (2.5 mL) for 2 h at 60°C. NaBH3CN (14.7 mg, 233 µmol) was added and the mixture was stirred at rt. The mixture was filtered and the filtrate was evaporated. The residue was purified by preparative HPLC to give 7.00 mg (90 % purity, 6 % yield) of the title compound.
1H NMR (DMSO-d6, 400 MHz) δ 10.5-13.1 (m, 1H), 8.5-8.7 (m, 1H), 8.0-8.0 (m, 1H), 7.0-7.1 (m, 2H), 6.5-6.6 (m, 2H), 6.3-6.4 (m, 1H), 4.0-4.1 (m, 2H), 3.8-3.9 (m, 1H), 2.8-2.9 (m, 1H), 2.7-2.8 (m, 1H), 2.6-2.7 (m, 2H), 2.5-2.6 (m, 1H), 1.9-2.0 (m, 2H), 1.4-1.4 (m, 3H)
LC-MS (method 1): Rt = 0.87 min; MS (ESlpos): m/z = 419 [M+H]⁺

### Example 557

### 5-{1-[1-(1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

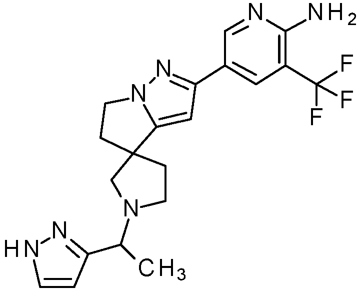

Example 557 was prepared in analogy to Example 556 using 5-[5',6'-dihydrospiro[pyrrolidine-3,4'-pyrrolo[1,2-b]pyrazol]-2'-yl]-3-(trifluoromethyl)pyridin-2-amine-hydrochloride salt (75.0 mg, 208 µmol, stereoisomer 1) and 1-(1H-pyrazol-3-yl)ethan-1-one-hydrochloride salt (45.8 mg, 313 µmol).
1H NMR (DMSO-d6, 400 MHz) δ 12.4-12.7 (m, 1H), 8.5-8.6 (m, 1H), 7.8-8.2 (m, 1H), 7.3-7.7 (m, 1H), 6.5-6.6 (m, 2H), 6.2-6.4 (m, 1H), 5.9-6.2 (m, 1H), 4.0-4.2 (m, 2H), 3.6-3.8 (m, 1H), 2.7-2.8 (m, 1H), 2.61 (br d, 3H), 2.4-2.5 (m, 1H), 1.8-2.1 (m, 2H), 1.35 (d, 3H)
[α]²⁰_{D} : -33.9° (c = 1.00, DMSO)
LC-MS (method 1): Rt = 0.96 min; MS (ESlpos): m/z = 419 [M+H]⁺

### Example 558

### 5-{(3R)-1-[1-(1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

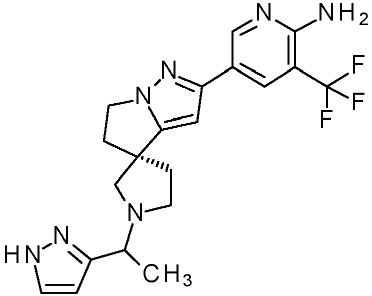

Example 558 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 1-(1H-pyrazol-3-yl)ethan-1-one-hydrochloride salt (61.1 mg, 417 µmol).
1H NMR (DMSO-d6) δ: 12.34-12.85 (m, 1H), 8.52-8.67 (m, 1H), 7.92-8.12 (m, 1H), 7.28-7.70 (m, 1H), 6.51 (s, 2H), 6.38 (d, 1H), 6.16 (s, 1H), 3.96-4.17 (m, 2H), 3.68 (br s, 1H), 2.69-2.77 (m, 1H), 2.57-2.66 (m, 2H), 2.41-2.49 (m, 2H), 1.87-2.02 (m, 2H), 1.35 (d, 3H)
[α]²⁰_{D} : +37.6° (c = 1.00, DMSO)
LC-MS (method 1): Rt = 0.94 min; MS (ESlpos): m/z = 419 [M+H]⁺

### Example 559

### 5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

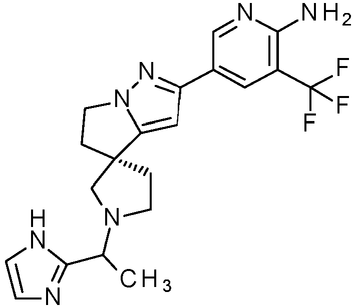

Example 559 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 1-(1H-imidazol-2-yl)ethan-1-one (45.9 mg, 417 µmol).
1H NMR (DMSO-d6) δ: 11.77 (br s, 1H), 8.58 (s, 1H), 8.00 (d, 1H), 7.02 (br s, 1H), 6.78 (br s, 1H), 6.52 (s, 2H), 6.34-6.43 (m, 1H), 4.05-4.13 (m, 2H), 3.67-3.76 (m, 1H), 2.66-2.89 (m, 2H), 2.54-2.64 (m, 2H), 2.40-2.49 (m, 2H), 1.88-2.02 (m, 2H), 1.38 (d, 3H)
[α]²⁰_{D} : +39.4° (c = 1.00, DMSO)
LC-MS (method 1): Rt = 0.87 min; MS (ESlpos): m/z = 418 [M+H]⁺

### Example 560

### 5-{(3R)-1-[1-(1H-imidazol-5-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

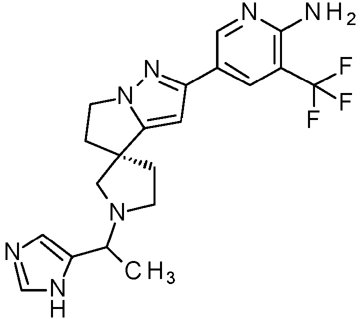

Example 560 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 1-(1H-imidazol-5-yl)ethan-1-one (45.9 mg, 417 µmol).
1H NMR (DMSO-d6) δ: 11.76-12.04 (m, 1H), 8.52-8.65 (m, 1H), 8.00 (d, 1H), 7.52 (s, 1H), 6.67-6.99 (m, 1H), 6.51 (br s, 2H), 6.31-6.44 (m, 1H), 4.07 (m, 2H), 3.55-3.69 (m, 1H), 2.55-2.92 (m, 4H), 1.88-2.03 (m, 2H), 1.34 (br m, 3H)
[α]²⁰_{D} : +47.3° (c = 1.00, DMSO)
LC-MS (method 1): Rt = 0.86 min; MS (ESlpos): m/z = 419 [M+H]⁺

### Example 561

### 5-{(3R)-1-[1-(1-methyl-1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

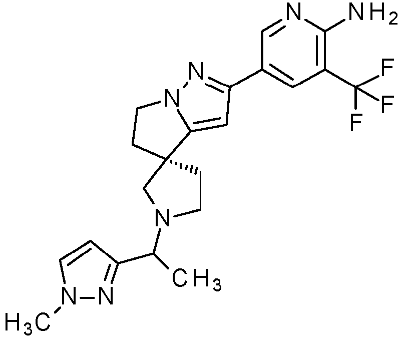

Example 561 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 1-(1-methyl-1H-pyrazol-3-yl)ethan-1-one (51.8 mg, 417 µmol).
1H NMR (DMSO-d6) δ: 8.58 (m, 1H), 8.01 (m, 1H), 7.56 (m, 1H), 6.51 (s, 2H), 6.39 (d, 1H), 6.14 (d, 1H), 4.02-4.13 (m, 2H), 3.76 (d, 3H), 3.58 (m, 1H), 2.68-2.76 (m, 1H), 2.53-2.68 (m, 3H), 2.40-2.48 (m, 1H), 1.89-2.03 (m, 2H), 1.25-1.40 (m, 3H)
[α]²⁰_{D} : +43.0° (c = 1.00, DMSO)
LC-MS (method 1): Rt = 1.00 min; MS (ESlpos): m/z = 433 [M+H]⁺

### Example 562

### 5-{(3R)-1-[1-(1H-pyrazol-4-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

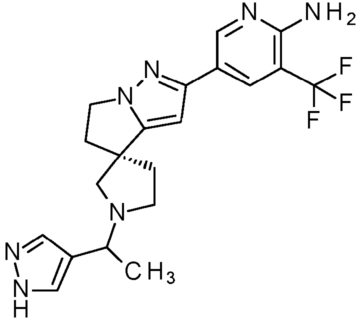

Example 562 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 1-(1H-pyrazol-4-yl)ethan-1-one (45.9 mg, 417 µmol).
1H NMR (DMSO-d6) δ: 12.61 (s, 1H), 8.47-8.68 (m, 1H), 8.01 (m, 1H), 7.51 (br s, 2H), 6.51 (s, 2H), 6.37 (d, 1H), 4.03-4.13 (m, 2H), 3.58 (qd, 1H), 2.61-2.72 (m, 2H), 2.56 (d, 1H), 2.39-2.48 (m, 1H), 1.87-2.03 (m, 2H), 1.33 (d, 3H)
[α]²⁰_{D} : +55.0° (c = 1.00, DMSO)
LC-MS (method 1): Rt = 0.92 min; MS (ESlpos): m/z = 419 [M+H]⁺

### Example 563

### 5-{(3R)-1-[1-(4-methyl-1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

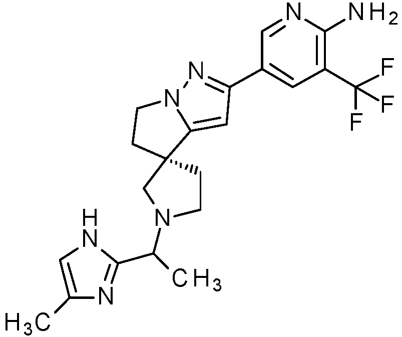

Example 563 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 1-(4-methyl-1H-imidazol-2-yl)ethanone (65 mg, 521 µmol).
1H NMR (DMSO-d6) δ: 11.32-11.57 (m, 1H), 8.58 (d, 1H), 7.89-8.05 (m, 1H), 6.59-6.77 (m, 0,6H), 6.52 (s, 2H), 6.40-6.47 (m, 0,4H), 6.32-6.39 (m, 1H), 4.03-4.15 (m, 2H), 3.56-3.68 (m, 1H), 2.81 (br d, 1H), 2.65-2.78 (m, 1H), 2.52-2.65 (m, 3H), 2.41-2.48 (m, 1H), 2.02-2.16 (br m, 3H), 1.88-2.01 (m, 2H), 1.35 (d, 3H)
[α]²⁰_{D} : +41.9° (c = 1.00, DMSO)
LC-MS (method 1): Rt = 0.91 min; MS (ESlpos): m/z = 433 [M+H]⁺

### Example 564

### 5-{(3R)-1-[1-(3-methyl-1H-pyrazol-5-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

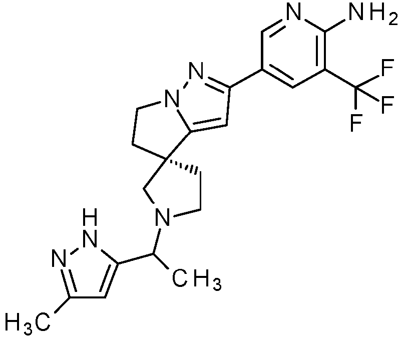

Example 564 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 1-(3-methyl-1H-pyrazol-5-yl)ethan-1-one (64.7 mg, 521 µmol).
1H NMR (DMSO-d6, 400 MHz) δ 12.0-12.3 (m, 1H), 8.58 (t, 1H), 7.9-8.1 (m, 1H), 6.52 (s, 2H), 6.3-6.4 (m, 1H), 5.90 (s, 1H), 4.0-4.1 (m, 2H), 3.5-3.7 (m, 1H), 2.7-2.8 (m, 1H), 2.5-2.7 (m, 3H), 2.16 (br s, 3H), 1.9-2.0 (m, 2H), 1.31 (br d, 3H)
[α]²⁰_{D} : +42.2° (c = 1.00, DMSO)
LC-MS (method 1): Rt = 0.99 min; MS (ESlpos): m/z = 433 [M+H]⁺

### Example 565

### 5-{(3R)-1-[1-(4-methyl-1H-pyrazol-5-yl)ethyl]-5',6'-dihydrospiro[pyrrolidine-3,4'-pyrrolo[1, 2-b]pyrazol]-2'-yl}-3-(trifluoromethyl)pyridin-2-amine (mixture of stereoisomers)

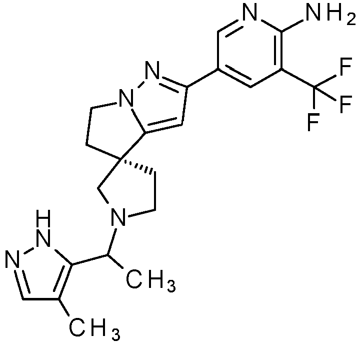

Example 565 was prepared in analogy to Example 556 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 1-(4-methyl-1H-pyrazol-5-yl)ethan-1-one (64.7 mg, 521 µmol).
1H NMR (DMSO-d6, 400 MHz) δ 12.1-12.5 (m, 1H), 8.57 (s, 1H), 8.00 (d, 1H), 7.1-7.5 (m, 1H), 6.52 (s, 2H), 6.41 (br s, 1H), 4.0-4.2 (m, 2H), 3.61 (quin, 1H), 2.73 (br d, 1H), 2.5-2.6 (m, 2H), 1.9-2.1 (m, 5H), 1.34 (br d, 3H)
[α]²⁰_{D} : +45.5° (c = 1.00, DMSO)
LC-MS (method 1): Rt = 1.05 min; MS (ESlpos): m/z = 433 [M+H]⁺

### Example 566

### 5-[(3S)-1-(pyridin-3-ylmethyl)-5',6'-dihydrospiro[pyrrolidine-3,4'-pyrrolo[1,2-b]pyrazol]-2'-yl]-3-(trifluoromethyl)pyridin-2-amine

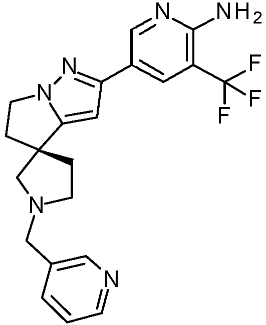

5-[(3S)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 84 % purity, 233 µmol) and pyridine-3-carbaldehyde (33 µL, 350 µmol) were solubilised in THF (2.5 mL), acetic acid (20 µL) was added and the mixture was stirred for 30 min at rt. Na(CH3COO)3BH was added and the mixture was stirred overnight at rt. It was diluted with water and extracted 2x with ethyl acetate. The combined organic layers were washed with brine, dried over Na2SO4 and concentrated under reduced pressure. The residue was purified by preparative HPLC to give 14.0 mg (99 % purity, 14 % yield) of the title compound.
1H NMR (DMSO-d6, 400 MHz) δ 8.6-8.6 (m, 1H), 8.5-8.6 (m, 1H), 8.4-8.5 (m, 1H), 7.9-8.1 (m, 1H), 7.6-7.8 (m, 1H), 7.3-7.4 (m, 1H), 6.5-6.6 (m, 2H), 6.48 (s, 1H), 4.0-4.2 (m, 2H), 3.6-3.8 (m, 2H), 2.5-2.8 (m, 6H), 2.0-2.1 (m, 2H)
[α]²⁰_{D} : -53.2° (c = 1.00, DMSO)
LC-MS (method 1): Rt = 1.01 min; MS (ESlpos): m/z = 415 [M+H]⁺

### Example 567

### 5-[(3R)-1-(pyridin-3-ylmethyl)-5',6'-dihydrospiro[pyrrolidine-3,4'-pyrrolo[1,2-b]pyrazol]-2'-yl]-3-(trifluoromethyl)pyridin-2-amine

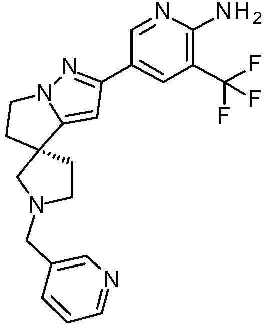

Example 567 was prepared in analogy to Example 566 using 5-[(3R9-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 82 % purity, 228 µmol) and pyridine-3-carbaldehyde (32 µL, 340 µmol).
1H NMR (DMSO-d6, 400 MHz) δ 8.6-8.6 (m, 1H), 8.5-8.6 (m, 1H), 8.4-8.5 (m, 1H), 8.0-8.0 (m, 1H), 7.7-7.8 (m, 1H), 7.3-7.4 (m, 1H), 6.5-6.6 (m, 2H), 6.5-6.5 (m, 1H), 4.0-4.1 (m, 2H), 3.6-3.7 (m, 2H), 2.5-2.8 (m, 6H), 2.0-2.1 (m, 2H)
[α]²⁰_{D} : +50.4° (c = 1.00, DMSO)
LC-MS (method 1): Rt = 1.01 min; MS (ESlpos): m/z = 415 [M+H]⁺

### Example 568

### 5-{(rac)-1-[(1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine

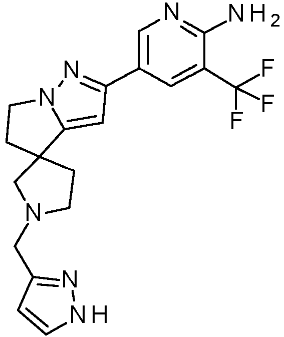

Example 568 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (200 mg, 556 µmol) and 1H-pyrazole-3-carbaldehyde (80.1 mg, 834 µmol).
1H NMR (DMSO-d6, 400 MHz) δ 12.5-12.7 (m, 1H), 8.6-8.6 (m, 1H), 8.01 (d, 1H), 7.3-7.7 (m, 1H), 6.51 (s, 2H), 6.4-6.5 (m, 1H), 6.18 (br s, 1H), 4.1-4.1 (m, 2H), 3.6-3.7 (m, 2H), 2.7-2.8 (m, 1H), 2.6-2.7 (m, 4H), 2.0-2.1 (m, 2H)
LC-MS (method 1): Rt = 0.90 min; MS (ESlpos): m/z = 405 [M+H]⁺

### Example 569

### 5-[(3S)-1-(1H-imidazol-2-ylmethyl)-5',6'-dihydrospiro[pyrrolidine-3,4'-pyrrolo[1,2-b]pyrazol]-2'-yl]-3-(trifluoromethyl)pyridin-2-amine

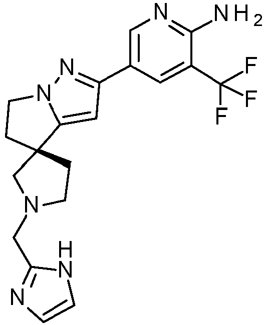

Example 569 was prepared in analogy to Example 566 using 5-[(3S9-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 84 % purity, 233 µmol) and 1H-imidazole-2-carbaldehyde (33.7 mg, 350 µmol).
1H NMR (DMSO-d6, 400 MHz) δ 11.5-12.1 (m, 1H), 8.5-8.7 (m, 1H), 7.9-8.1 (m, 1H), 6.8-7.1 (m, 2H), 6.52 (s, 2H), 6.46 (s, 1H), 4.1-4.1 (m, 2H), 3.67 (s, 2H), 2.7-2.8 (m, 2H), 2.6-2.7 (m, 2H), 2.5-2.6 (m, 1H), 2.0-2.1 (m, 2H)
[α]²⁰_{D} : -45.5° (c = 1.00, DMSO)
LC-MS (method 1): Rt = 0.83 min; MS (ESlpos): m/z = 404 [M+H]⁺

### Example 570

### 5-[(3R)-1-(1H-imidazol-2-ylmethyl)-5',6'-dihydrospiro[pyrrolidine-3,4'-pyrrolo[1,2-b]pyrazol]-2'-yl]-3-(trifluoromethyl)pyridin-2-amine

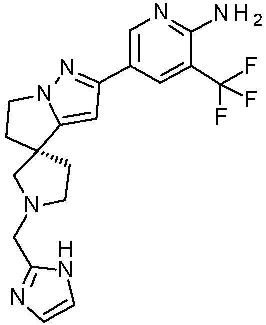

Example 570 was prepared in analogy to Example 566 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 82 % purity, 228 µmol) and 1H-imidazole-2-carbaldehyde (32.9 mg, 342 µmol).
1H NMR (DMSO-d6, 400 MHz) δ 9.6-11.8 (m, 1H), 8.5-8.7 (m, 1H), 7.9-8.1 (m, 1H), 6.8-7.0 (m, 2H), 6.5-6.6 (m, 2H), 6.4-6.5 (m, 1H), 4.0-4.1 (m, 2H), 3.6-3.7 (m, 2H), 2.7-2.8 (m, 2H), 2.6-2.7 (m, 2H), 2.5-2.6 (m, 1H), 1.9-2.1 (m, 2H)
[α]²⁰_{D} : +45.3° (c = 1.00, DMSO)
LC-MS (method 1): Rt = 0.83 min; MS (ESlpos): m/z = 404 [M+H]⁺

### Example 571

### 5-{(rac)-1-[(4-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine

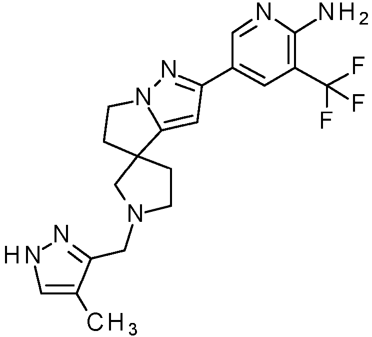

Example 571 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 4-methyl-1H-pyrazole-3-carbaldehyde (45.9 mg, 417 µmol).
1H NMR (DMSO-d6) δ: 12.17-12.58 (m, 1H), 8.58 (d, 1H), 8.00 (d, 1H), 7.16-7.51 (m, 1H), 6.52 (s, 2H), 6.42 (s, 1H), 4.01-4.17 (m, 2H), 3.60 (s, 2H), 2.55-2.79 (m, 5H), 1.92-2.13 (m, 5H)
LC-MS (method 1): Rt = 1.00 min; MS (ESlpos): m/z = 419 [M+H]⁺

### Example 572

### 5-{(rac)-1-[(1H-pyrazol-4-yl)methyl]-5',6'-dihydrospiro[pyrrolidine-3,4'-pyrrolo[1,2-b]pyrazol]-2'-yl}-3-(trifluoromethyl)pyridin-2-amine

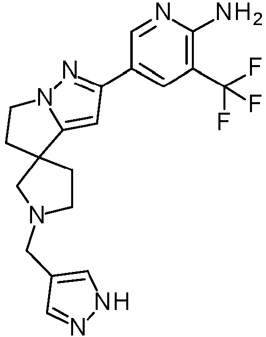

Example 572 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 1H-pyrazole-4-carbaldehyde (50.1 mg, 521 µmol).
1H NMR (DMSO-d6) δ: 12.64 (br s, 1H), 8.59 (d, 1H), 8.02 (d, 1H), 7.33-7.74 (m, 2H), 6.51 (s, 2H), 6.44 (s, 1H), 4.09 (m, 2H), 3.53 (s, 2H), 2.67-2.78 (m, 2H), 2.53-2.66 (m, 3H), 1.96-2.07 (m, 2H)
LC-MS (method 1): Rt = 0.88 min; MS (ESlpos): m/z = 404 [M+H]⁺

### Example 573

### 5-{(rac)-1-[(1-methyl-1H-pyrazol-4-yl)methyl]-5',6'-dihydrospiro[pyrrolidine-3,4'-pyrrolo[1,2-b]pyrazol]-2'-yl}-3-(trifluoromethyl)pyridin-2-amine

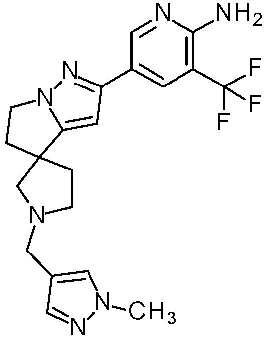

Example 573 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 1-methyl-1H-pyrazole-4-carbaldehyde (57.4 mg, 521 µmol).
1H NMR (DMSO-d6) δ: 8.59 (d, 1H), 8.02 (d, 1H), 7.58 (s, 1H), 7.33 (s, 1H), 6.51 (s, 2H), 6.43 (s, 1H), 4.08 (m, 2H), 3.78 (s, 3H), 3.48 (s, 2H), 2.67-2.77 (m, 2H), 2.53-2.64 (m, 3H), 1.94-2.07 (m, 2H)
LC-MS (method 1): Rt = 0.94 min; MS (ESlpos): m/z = 418 [M+H]⁺

### Example 574

### 5-{(rac)-1-[(1H-imidazol-4-yl)methyl]-5',6'-dihydrospiro[pyrrolidine-3,4'-pyrrolo[1,2-b]pyrazol]-2'-yl}-3-(trifluoromethyl)pyridin-2-amine

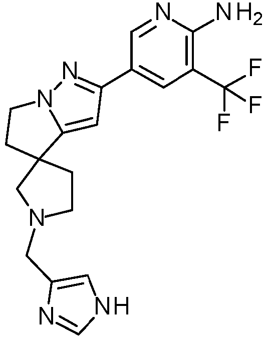

Example 574 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 1H-imidazole-4-carbaldehyde (50.1 mg, 521 µmol).
1H NMR (DMSO-d6) δ: 8.58 (d, 1H), 8.01 (d, 1H), 7.62 (d, 1H), 7.00 (s, 1H), 6.53 (s, 2H), 6.46 (s, 1H), 4.10 (m, 2H), 3.73 (s, 2H), 2.78-2.98 (m, 4H), 2.55-2.64 (m, 1H), 1.99-2.09 (m, 2H)
LC-MS (method 1): Rt = 0.83 min; MS (ESlneg): m/z = 402 [M-H]⁻

### Example 575

### 5-{(rac)-1-[(1-methyl-1H-imidazol-4-yl)methyl]-5',6'-dihydrospiro[pyrrolidine-3,4'-pyrrolo[1,2-b]pyrazol]-2'-yl}-3-(trifluoromethyl)pyridin-2-amine

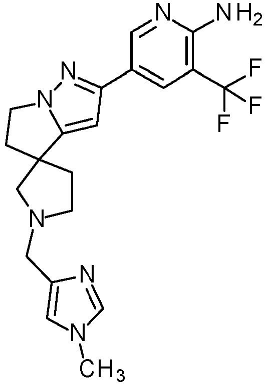

Example 575 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 1-methyl-1H-imidazole-4-carbaldehyde (57.4 mg, 521 µmol).
1H NMR (DMSO-d6) δ: 8.59 (d, 1H), 8.01 (d, 1H), 7.45 (d, 1H), 6.96 (d, 1H), 6.51 (s, 2H), 6.43 (s, 1H), 4.05-4.14 (m, 2H), 3.60 (s, 3H), 3.50 (s, 2H), 2.74-2.82 (m, 2H), 2.60-2.71 (m, 2H), 2.52-2.60 (m, 1H), 1.93-2.06 (m, 2H)
LC-MS (method 1): Rt = 0.87 min; MS (ESlpos): m/z = 419 [M+H]⁺

### Example 576

### 5-{(rac)-1-[(4-chloro-1H-pyrazol-5-yl)methyl]-5',6'-dihydrospiro[pyrrolidine-3,4'-pyrrolo[1,2-b]pyrazol]-2'-yl}-3-(trifluoromethyl)pyridin-2-amine

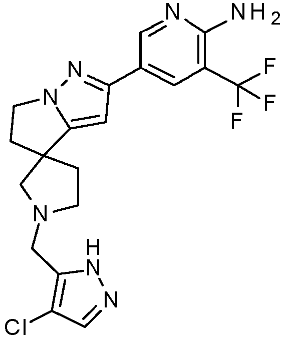

Example 576 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 347 µmol) and 4-chloro-1H-pyrazole-5-carbaldehyde (68.0 mg, 521 µmol).
1H NMR (DMSO-d6, 400 MHz) δ 12.9-13.2 (m, 1H), 8.58 (d, 1H), 8.00 (d, 1H), 7.5-8.0 (m, 1H), 6.52 (s, 2H), 6.4-6.5 (m, 1H), 4.09 (t, 2H), 3.6-3.8 (m, 2H), 2.7-2.8 (m, 2H), 2.6-2.7 (m, 3H), 2.0-2.1 (m, 2H)
LC-MS (method 1): Rt = 1.04 min; MS (ESlpos): m/z = 438 [M+H]⁺

### Example 577

### 3-[(2-fluorophenyl)methoxy]-5-{(rac)-1-[(1H-imidazol-2-yl)methyl]-5',6'-dihydrospiro[pyrrolidine-3,4'-pyrrolo[1,2-b]pyrazol]-2'-yl}pyridin-2-amine

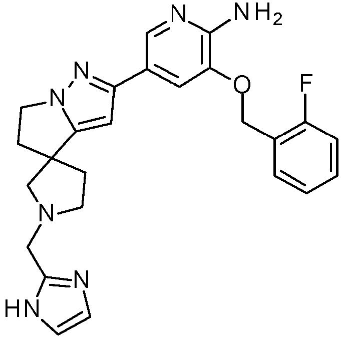

Example 577 was prepared in analogy to Example 566 using 5-[(rac)-5',6'-dihydrospiro[pyrrolidine-3,4'-pyrrolo[1,2-b]pyrazol]-2'-yl]-3-[(2-fluorophenyl)methoxy]pyridin-2-amine-hydrochloride salt (104 mg, 230 µmol) and 1H-imidazole-2-carbaldehyde (24.3 mg, 253 µmol).
¹H-NMR (400 MHz, DMSO-d6) δ [ppm]: 1.157 (1.35), 1.232 (2.04), 1.974 (0.97), 1.988 (1.95), 2.007 (3.61), 2.024 (3.71), 2.043 (2.17), 2.326 (1.98), 2.563 (3.49), 2.580 (2.77), 2.596 (1.89), 2.613 (1.23), 2.642 (3.55), 2.665 (6.76), 2.698 (2.45), 2.717 (1.35), 2.741 (5.00), 2.765 (4.75), 2.787 (2.14), 2.809 (0.88), 3.674 (16.00), 4.067 (2.73), 4.080 (4.78), 4.095 (3.02), 5.208 (13.86), 5.749 (9.93), 6.355 (11.22), 6.823 (0.66), 7.231 (2.45), 7.241 (2.95), 7.249 (5.19), 7.266 (5.38), 7.286 (2.83), 7.397 (1.41), 7.411 (2.51), 7.431 (2.26), 7.446 (1.10), 7.479 (6.44), 7.653 (1.92), 7.671 (3.33), 7.690 (1.79), 7.961 (6.82), 7.965 (6.95), 11.854 (1.85).
LC-MS (Method 1): Rt = 0.93 min; MS (ESlpos): m/z = 460 [M+H]⁺

### Example 578

### 3-[(3-fluorophenyl)methoxy]-5-{(rac)-1-[(1H-imidazol-2-yl)methyl]-5',6'-dihydrospiro[pyrrolidine-3,4'-pyrrolo[1,2-b]pyrazol]-2'-yl}pyridin-2-amine

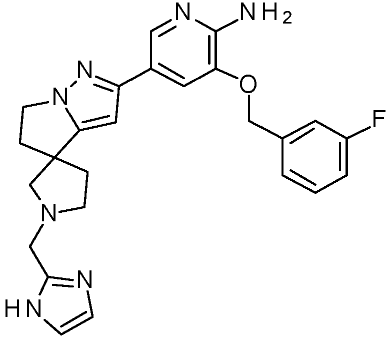

Example 578 was prepared in analogy to Example 566 using 5-[(rac)-5',6'-dihydrospiro[pyrrolidine-3,4'-pyrrolo[1,2-b]pyrazol]-2'-yl]-3-[(3-fluorophenyl)methoxy]pyridin-2-amine-hydrochloride salt (86.0 mg, 190 µmol) and 1H-imidazole-2-carbaldehyde (20.1 mg, 209 µmol).
¹H-NMR (400 MHz, DMSO-d6) δ [ppm]: 1.166 (0.54), 1.201 (0.67), 1.232 (1.31), 1.954 (0.43), 1.973 (0.79), 1.985 (1.68), 2.004 (3.59), 2.020 (3.35), 2.039 (1.95), 2.051 (0.75), 2.071 (0.54), 2.327 (1.31), 2.331 (0.94), 2.449 (0.71), 2.466 (1.80), 2.544 (2.34), 2.560 (2.86), 2.578 (2.47), 2.592 (1.61), 2.610 (1.05), 2.639 (3.41), 2.661 (5.54), 2.669 (2.47), 2.674 (2.45), 2.678 (2.56), 2.697 (2.13), 2.717 (1.18), 2.737 (4.75), 2.751 (1.70), 2.761 (3.78), 2.786 (1.83), 2.807 (0.82), 3.505 (0.43), 3.674 (16.00), 4.054 (2.34), 4.060 (2.41), 4.072 (4.30), 4.089 (2.66), 4.094 (2.60), 5.204 (14.80), 5.855 (9.79), 6.334 (14.95), 6.920 (1.46), 7.126 (1.22), 7.131 (1.31), 7.153 (2.53), 7.169 (1.50), 7.174 (1.52), 7.351 (3.07), 7.370 (4.81), 7.412 (4.17), 7.427 (10.89), 7.431 (11.21), 7.447 (3.48), 7.467 (1.46), 7.946 (8.20), 7.950 (8.10), 11.855 (0.52).
LC-MS (Method 1): Rt = 0.92 min; MS (ESlpos): m/z = 460 [M+H]⁺

### Example 579

### 5-{(rac)-1-[(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine

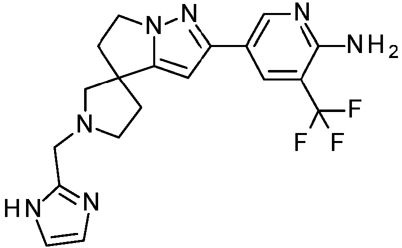

Example 579 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine hydrochloride (75.0 mg, 208 µmol) and 1H-imidazole-2-carbaldehyde (15.0 mg, 156 µmol).
LC-MS (Method 1): Rt = 0.84 min; MS (ESlneg): m/z = 402 [M-H]⁻
¹H NMR (500 MHz, DMSO-*d*₆) δ ppm 2.00 - 2.11 (m, 2 H) 2.52 - 2.54 (m, 3 H) 2.57 - 2.63 (m, 1 H) 2.72 - 2.89 (m, 3 H) 3.80 (br s, 2 H) 4.07 - 4.15 (m, 2 H) 6.46 (s, 1 H) 6.51 (s, 2 H) 7.06 (br s, 2 H) 8.00 (d, 1 H) 8.58 (d, 1 H)

### Example 580

### 5-{1-[(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine

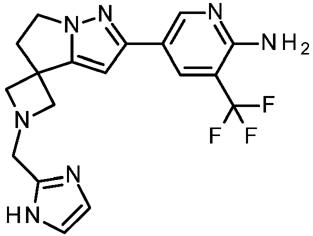

Example 580 was prepared in analogy to Example 566 using 5-(5',6'-dihydrospiro[azetidine-3,4'-pyrrolo[1,2-b]pyrazol]-2'-yl)-3-(trifluoromethyl)pyridin-2-amine (75.0 mg, 242 µmol) and 1H-imidazole-2-carbaldehyde (17.5 mg, 182 µmol).
LC-MS (Method 1): Rt = 0.81 min; MS (ESlpos): m/z = 390 [M+H]⁺
¹H-NMR (400 MHz, DMSO-d6) δ [ppm]: 0.811 (0.28), 0.827 (0.28), 1.107 (3.18), 1.234 (0.23), 1.352 (0.23), 1.906 (0.51), 2.084 (1.31), 2.518 (16.00), 2.523 (11.13), 2.768 (0.51), 2.786 (0.89), 2.803 (0.61), 3.388 (2.34), 3.428 (1.45), 3.447 (0.84), 3.618 (2.29), 4.066 (0.56), 4.085 (0.89), 4.102 (0.56), 6.537 (1.36), 6.638 (2.25), 8.025 (0.70), 8.224 (0.37), 8.599 (0.75).

### Example 581

### 5-{(rac)-1'-[(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine

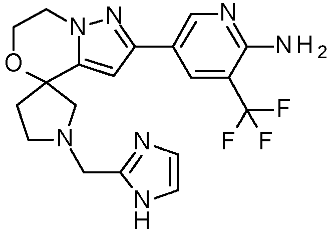

Example 581 was prepared in analogy to Example 566 using 5-[(*rac*)-6,7-dihydrospiro[pyrazolo[5,1-c][1,4]oxazine-4,3'-pyrrolidin]-2-yl]-3-(trifluoromethyl)pyridin-2-amine (75.0 mg, 221 µmol) and 1H-imidazole-2-carbaldehyde (15.9 mg, 166 µmol).
LC-MS (Method 1): Rt = 0.85 min; MS (ESlpos): m/z = 420 [M+H]⁺
¹H-NMR (500 MHz, DMSO-d6) δ [ppm]: 1.001 (1.28), 1.015 (3.00), 1.029 (1.30), 1.909 (1.67), 2.140 (1.24), 2.152 (1.49), 2.167 (2.28), 2.181 (1.18), 2.206 (1.00), 2.219 (1.77), 2.231 (1.77), 2.245 (0.92), 2.373 (0.84), 2.387 (0.71), 2.518 (2.93), 2.522 (2.91), 2.526 (2.32), 2.783 (1.43), 2.799 (2.49), 2.813 (2.87), 2.827 (1.35), 2.853 (3.24), 2.874 (3.83), 3.023 (3.24), 3.044 (3.14), 3.054 (0.69), 3.058 (0.71), 3.751 (7.72), 3.754 (7.87), 3.997 (0.80), 4.010 (1.39), 4.022 (2.85), 4.035 (3.91), 4.044 (2.47), 4.060 (1.26), 4.069 (1.22), 4.077 (4.91), 4.086 (5.24), 4.099 (1.79), 4.169 (0.71), 4.184 (0.77), 6.427 (1.75), 6.564 (8.60), 6.647 (16.00), 7.017 (13.80), 8.035 (5.14), 8.039 (5.24), 8.314 (1.94), 8.612 (4.73), 8.616 (4.65), 9.647 (0.75).

### Example 582

### 2-amino-5-{1-[(1H-imidazol-2-yl)methyl]-5',6'-dihydrospiro[azetidine-3,4'-pyrrolo[1,2-b]pyrazol]-2'-yl}pyridine-3-carbonitrile

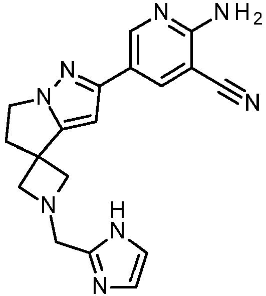

Example 582 was prepared in analogy to Example 566 using 2-amino-5-(5',6'-dihydrospiro[azetidine-3,4'-pyrrolo[1,2-b]pyrazol]-2'-yl)pyridine-3-carbonitrile-hydrochloride salt (100 mg, 330 µmol) and 1H-imidazole-2-carbaldehyde (47.6 mg, 495 µmol).
1H NMR (400 MHz, DMSO-d6) δ 11.62-12.01 (m, 1H), 8.58-8.68 (m, 1H), 8.05-8.22 (m, 1H), 7.00-7.10 (m, 1H), 6.99 (s, 2H), 6.73-6.90 (m, 1H), 6.60 (s, 1H), 4.04-4.11 (m, 2H), 3.57-3.65 (m, 2H), 3.41-3.48 (m, 2H), 3.36-3.39 (m, 2H), 2.75-2.81 (m, 2H)
LC-MS (method 1): Rt = 0.66 min; MS (ESlpos): m/z = 347 [M+H]⁺

### Example 583

### 5-{(rac)-1-[(1-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine

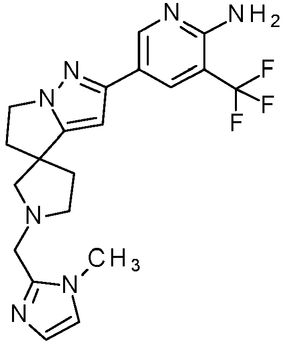

Example 583 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 1-methyl-1H-imidazole-2-carbaldehyde (45.9 mg, 417 µmol).
1H NMR (400 MHz, DMSO-d6) δ 8.53-8.62 (m, 1H), 7.90-8.05 (m, 1H), 6.98-7.12 (m, 1H), 6.67-6.80 (m, 1H), 6.48-6.60 (m, 2H), 6.33-6.47 (m, 1H), 4.04-4.16 (m, 2H), 3.69 (s, 5H), 2.51-2.75 (m, 6H), 1.92-2.11 (m, 2H)
LC-MS (method 1): Rt = 0.94 min; MS (ESlpos): m/z = 419 [M+H]⁺

### Example 584

### 5-{(rac)-1-[(1-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine

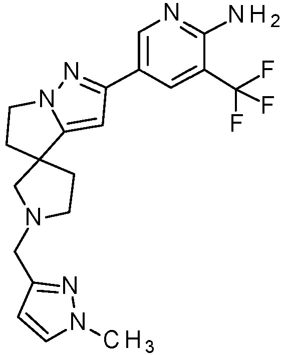

Example 584 was prepared in analogy to Example 566 using 5-[(*rac*)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 1-methyl-1H-pyrazole-3-carbaldehyde (45.9 mg, 417 µmol).
1H NMR (400 MHz, DMSO-d6) δ 8.56-8.62 (m, 1H), 7.98-8.05 (m, 1H), 7.54-7.61 (m, 1H), 6.48-6.56 (m, 2H), 6.41-6.48 (m, 1H), 6.11-6.21 (m, 1H), 4.03-4.15 (m, 2H), 3.73-3.79 (m, 3H), 3.50-3.64 (m, 2H), 2.51-2.81 (m, 6H), 1.91-2.10 (m, 2H)
LC-MS (method 1): Rt = 0.95 min; MS (ESlpos): m/z = 419 [M+H]⁺

### Example 585

### 2-amino-5-{(rac)-1-[(1H-imidazol-2-yl)methyl]-5',6'-dihydrospiro[pyrrolidine-3,4'-pyrrolo[1,2-b]pyrazol]-2'-yl}pyridine-3-carbonitrile

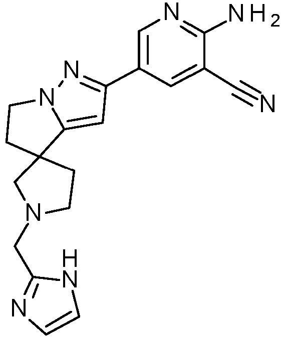

Example 585 was prepared in analogy to Example 566 using 2-amino-5-[(rac)-5',6'-dihydrospiro[pyrrolidine-3,4'-pyrrolo[1,2-b]pyrazol]-2'-yl]pyridine-3-carbonitrile-hydrochloride salt (98.0 mg, 309 µmol) and 1H-imidazole-2-carbaldehyde (44.6 mg, 464 µmol).
1H NMR (400 MHz, DMSO-d6) δ 11.34-11.97 (m, 1H), 8.45-8.82 (m, 1H), 7.76-8.30 (m, 1H), 7.00-7.08 (m, 1H), 6.94-7.00 (m, 2H), 6.74-6.86 (m, 1H), 6.44 (s, 1H), 4.02-4.18 (m, 2H), 3.67 (s, 2H), 2.54-2.81 (m, 5H), 1.94-2.07 (m, 2H)
LC-MS (method 1): Rt = 0.70 min; MS (ESlpos): m/z = 361 [M+H]⁺

### Example 586

### 5-{1-[(1-methyl-1H-imidazol-2-yl)methyl]-5',6'-dihydrospiro[azetidine-3,4'-pyrrolo[1,2-b]pyrazol]-2'-yl}-3-(trifluoromethyl)pyridin-2-amine

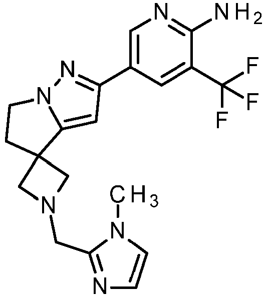

Example 586 was prepared in analogy to Example 566 using 5-(5',6'-dihydrospiro[azetidine-3,4'-pyrrolo[1,2-b]pyrazol]-2'-yl)-3-(trifluoromethyl)pyridin-2-amine-hydrochloride salt (100 mg, 57 % purity, 165 µmol) and 1-methyl-1H-imidazole-2-carbaldehyde (27.2 mg, 247 µmol).
1H NMR (400 MHz, DMSO-d6) δ 8.62 (d, 1H), 8.03 (d, 1H), 7.07 (d, 1H), 6.74 (d, 1H), 6.65 (s, 1H), 6.53 (s, 2H, NH2), 4.07 (t, 2H), 3.62-3.70 (m, 5H), 3.35-3.43 (m, 5H), 2.77 (t, 2H)
LC-MS (method 1): Rt = 0.87 min; MS (ESlpos): m/z = 405 [M+H]⁺

### Example 587

### 5-{(rac)-1-[(5-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine

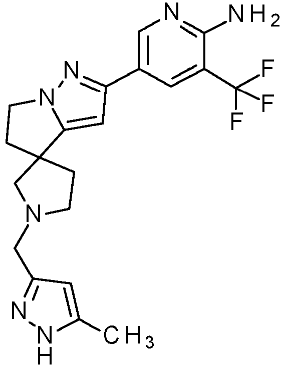

Example 587 was prepared in analogy to Example 566 using 5-[(*rac*)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 5-methyl-1H-pyrazole-3-carbaldehyde (45.9 mg, 417 µmol).
1H NMR (400 MHz, DMSO-d6) δ 11.79-12.56 (m, 1H), 8.25-8.82 (m, 1H), 7.77-8.18 (m, 1H), 6.48-6.56 (m, 2H), 6.42-6.46 (m, 1H), 5.86-5.94 (m, 1H), 4.03-4.16 (m, 2H), 3.51-3.64 (m, 2H), 2.51-2.80 (m, 6H), 2.11-2.22 (m, 3H), 1.94-2.08 (m, 2H)

### Example 588

### 5-{(rac)-1-[(2,4-dimethyl-1H-imidazol-5-yl)methyl]-5',6'-dihydrospiro[pyrrolidine-3,4'-pyrrolo[1,2-b]pyrazol]-2'-yl}-3-(trifluoromethyl)pyridin-2-amine

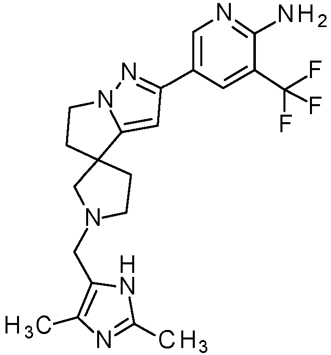

Example 588 was prepared in analogy to Example 566 using 5-[(rac)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 2,4-dimethyl-1H-imidazole-5-carbaldehyde (51.8 mg, 417 µmol).
H-NMR-Data 1H NMR (400 MHz, DMSO-d6) δ 8.47-8.66 (m, 1H), 7.90-8.12 (m, 1H), 6.47-6.57 (m, 2H), 6.39-6.45 (m, 1H), 4.03-4.15 (m, 2H), 3.44-3.47 (m, 2H), 2.69-2.80 (m, 2H), 2.51-2.64 (m, 4H), 2.12-2.19 (m, 3H), 2.03-2.07 (m, 3H),1.92-2.03 (m, 2H)
LC-MS (method 1): Rt = 0.90 min; MS (ESlneg): m/z = 430 [M-H]⁻

### Example 589

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

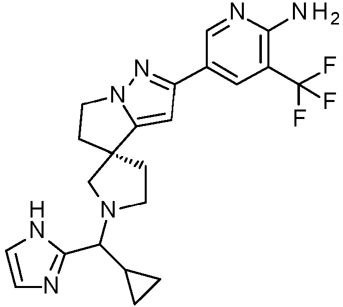

A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (125 mg, 79 % purity, 274 µmol), cyclopropyl(1H-imidazol-2-yl)methanone (56.1 mg, 412 µmol), Titanium(IV) isopropoxide (240 µl, 820 µmol) and DIPEA (190 µl, 1.1 mmol) in MeOH (2.7 mL) was stirred at 60°C for 2h. To this sodium cyanoborohydride (43.1 mg, 686 µmol) was added and the mixture was stirred at RT overnight.

The mixture was filtered and the filtrate concentrated in vacuo. The crude was purified by basic HPLC to give 4.00 mg (90 % purity, 3 % yield) of the title compound.
H-NMR-Data - 1H NMR (DMSO-d6, 400 MHz) δ 11.79 (br s, 1H), 8.59 (d, 1H), 8.00 (s, 1H), 7.0-7.1 (m, 1H), 6.75 (d, 1H), 6.52 (s, 2H), 6.44 (d, 1H), 4.0-4.2 (m, 2H), 2.9-3.0 (m, 1H), 2.7-2.8 (m, 1H), 2.7-2.7 (m, 1H), 2.6-2.6 (m, 2H), 1.9-2.1 (m, 2H), 1.23 (dt, 1H), 0.6-0.7 (m, 1H), 0.3-0.4 (m, 2H), 0.0-0.1 (m, 1H)
LC-MS (method 1): Rt = 0.95 min; MS (ESlpos): m/z = 445 [M+H]⁺

### Example 590

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (stereoisomer 1)

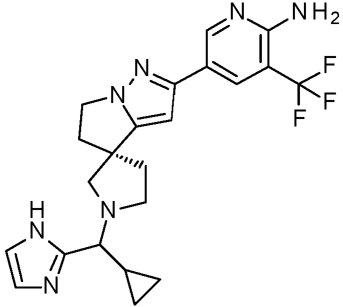

5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (296 mg, 667 µmol) was submitted to the HPLC-Lab for chiral separation. Enantiomer #1 was purified further by basic HPLC to give 62.0 mg (99 % purity, 21 % yield) of the title compound.
H-NMR-Data - 1H NMR (DMSO-d6, 400 MHz) δ 11.79 (br s, 1H), 8.59 (d, 1H), 8.00 (d, 1H), 7.02 (br s, 1H), 6.75 (br s, 1H), 6.53 (s, 2H), 6.45 (s, 1H), 4.10 (t, 2H), 2.94 (d, 1H), 2.78 (dt, 1H), 2.66 (d, 1H), 2.5-2.6 (m, 3H), 1.9-2.1 (m, 2H), 1.2-1.3 (m, 1H), 0.6-0.7 (m, 1H), 0.3-0.4 (m, 2H), 0.0-0.1 (m, 1H)
[α]²⁰_{D} : +54.38° (c = 1.00, DMSO)
LC-MS (method 1): Rt = 0.96 min; MS (ESlpos): m/z = 445 [M+H]⁺

### Example 591

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (stereoisomer 2)

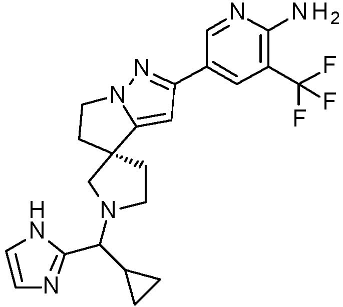

5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (296 mg, 667 µmol) was submitted to the HPLC-Lab for chiral separation. Enantiomer #2 was purified further by basic HPLC to give 54.0 mg (99 % purity, 18 % yield) of the title compound.
1H NMR (DMSO-d6, 400 MHz) δ 11.80 (br s, 1H), 8.59 (d, 1H), 8.00 (d, 1H), 7.03 (br s, 1H), 6.75 (br s, 1H), 6.52 (s, 2H), 6.44 (s, 1H), 4.0-4.2 (m, 2H), 2.7-2.8 (m, 2H), 2.5-2.6 (m, 3H), 1.9-2.0 (m, 2H), 1.1-1.3 (m, 1H), 0.5-0.7 (m, 1H), 0.2-0.4 (m, 2H), 0.08 (qd, 1H)
[α]²⁰_{D} : +49.51° (c = 1.00, DMSO)
LC-MS (method 1): Rt = 0.96 min; MS (ESlpos): m/z = 445 [M+H]⁺

### Example 592

### 5-{(3R)-1-[(1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

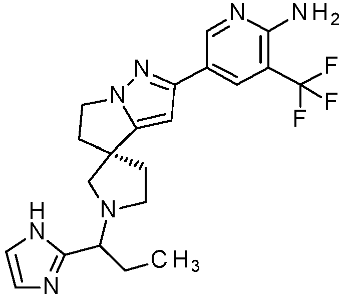

A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride salt (125 mg, 79 % purity, 274 µmol), 1-(1H-imidazol-2-yl)propan-1-one (51.1 mg, 412 µmol), Titanium(IV) isopropoxide (240 µl, 820 µmol) and DIPEA (190 µl, 1.1 mmol) in MeOH (2.7 mL) was stirred at 60°C for 2h. To this sodium cyanoborohydride (43.1 mg, 686 µmol) was added and the mixture was stirred at RT overnight. The resultant mixture was filtered and the filtrate concentrated in vacuo. The crude was purified by basic HPLC to give 33.0 mg (98 % purity, 27 % yield) of the title compound.
H-NMR-Data - 1H NMR (DMSO-d6, 400 MHz) δ 11.78 (br s, 1H), 8.57 (t, 1H), 7.99 (t, 1H), 7.04 (dt, 1H), 6.8-6.9 (m, 1H), 6.52 (s, 2H), 6.31 (d, 1H), 4.0-4.1 (m, 2H), 3.52 (ddd, 1H), 2.7-2.8 (m, 1H), 2.5-2.7 (m, 3H), 2.4-2.5 (m, 1H), 1.7-2.0 (m, 4H), 0.73 (dt, 3H)
LC-MS (method 1): Rt = 0.93 min; MS (ESlpos): m/z = 433 [M+H]⁺

### Example 593

### 5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (stereoisomer 1)

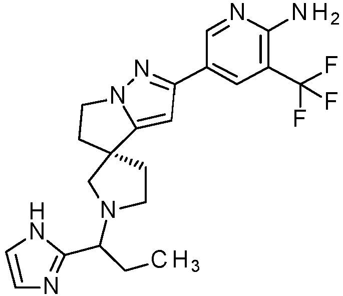

5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (270 mg, 626 µmol) was submitted to the HPLC-Lab for chiral separation. Enantiomer #1 was purified further by basic HPLC to give 59.0 mg (95 % purity, 21 % yield) of the title compound.
H-NMR-Data - 1H NMR (DMSO-d6, 400 MHz) δ 11.78 (br s, 1H), 8.5-8.7 (m, 1H), 7.99 (d, 1H), 7.04 (br s, 1H), 6.81 (br s, 1H), 6.52 (s, 2H), 6.34 (s, 1H), 4.08 (t, 2H), 3.52 (dd, 1H), 2.7-2.8 (m, 2H), 2.6-2.6 (m, 1H), 2.55 (d, 1H), 2.4-2.5 (m, 1H), 1.7-2.0 (m, 4H), 0.73 (t, 3H)
[α]²⁰_{D} : +35.53° (c = 1.00, DMSO)
LC-MS (method 1): Rt = 0.94 min; MS (ESlpos): m/z = 433 [M+H]⁺

### Example 594

### 5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (stereoisomer 2)

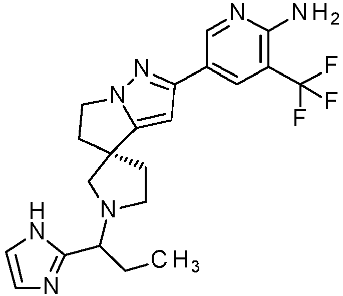

5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (270 mg, 626 µmol) was submitted to the HPLC-Lab for chiral separation. Enantiomer #2 was purified further by basic HPLC to give 44.0 mg (95 % purity, 15 % yield) of the title compound.
H-NMR-Data - 1H NMR (DMSO-d6, 400 MHz) δ 11.78 (br s, 1H), 8.57 (d, 1H), 7.99 (d, 1H), 7.04 (br s, 1H), 6.81 (br s, 1H), 6.52 (s, 2H), 6.29 (s, 1H), 4.0-4.2 (m, 2H), 3.53 (dd, 1H), 2.8-2.9 (m, 1H), 2.6-2.7 (m, 1H), 2.6-2.6 (m, 2H), 2.4-2.5 (m, 2H), 1.92 (t, 2H), 1.7-1.9 (m, 2H), 0.7-0.8 (m, 3H)
[α]²⁰_{D} : +53.59° (c = 1.00, DMSO)
LC-MS (method 1): Rt = 0.94 min; MS (ESlpos): m/z = 433 [M+H]⁺

### Example 595

### 5-{(3R)-1-[1-(1-methyl-1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (mixture of stereoisomers)

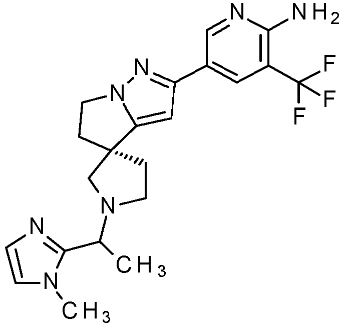

A mixture of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (800 mg, 79 % purity, 1.76 mmol), 1-methyl-1H-imidazole-2-carbaldehyde (290 mg, 2.63 mmol) and Na2SO4 in THF under Argon was stirred at RT overnight. The mixture was cooled to 0°C and treated with methyl magnesium chloride (2.3 ml, 3.0 M, 7.0 mmol) and continued stirring at 0°C for 1h. The mixture was quenched with 2mL of std. NH4Cl-solution, the mixture was filtered and concentrated in vacuo. The crude was pre-purified by colum chromatography with an isocratic gradient of Ethyl acetate followed by 0-20% methanol in DCM. The obtained product (186mg) was further purified by basic HPLC to give 24.0 mg (99 % purity, 3 % yield) of the title compound.
H-NMR-Data - 1H NMR (DMSO-d6, 400 MHz) δ 8.58 (t, 1H), 8.00 (t, 1H), 7.02 (dd, 1H), 6.73 (dd, 1H), 6.52 (s, 2H), 6.40 (d, 1H), 4.09 (t, 2H), 3.94 (qd, 1H), 3.73 (d, 3H), 2.79 (dt, 1H), 2.7-2.7 (m, 1H), 2.5-2.6 (m, 3H), 1.9-2.1 (m, 2H), 1.37 (dd, 3H)
LC-MS (method 1): Rt = 0.99 min; MS (ESlpos): m/z = 433 [M+H]⁺

### Example 596

### 3-(trifluoromethyl)-5-[(3R)-1-{[4-(trifluoromethyl)-1H-imidazol-2-yl]methyl}-5',6'-dihydrospiro[pyrrolidine-3,4'-pyrrolo[1,2-b]pyrazol]-2'-yl]pyridin-2-amine

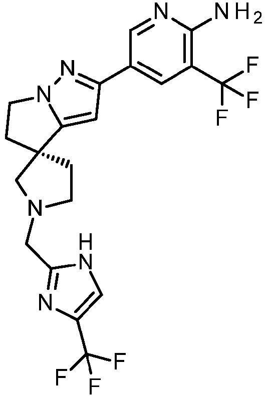

To a mixture of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (125 mg, 79 % purity, 274 µmol) and 4-(trifluoromethyl)-1H-imidazole-2-carbaldehyde (67.6 mg, 412 µmol) in THF (2.7 mL), acetic acid (24 µl) was added and stirred for 30min. To this sodium triacetoxyborohydride (116 mg, 549 µmol) was added and the mixture was stirred at RT overnight. The mixture was diluted with water and then extracted with Ethyl acetate (2x). The combined organics were washed with brine, dried over sodium sulfate and concentrated in vacuo. The crude was purified by basic to give 7.00 mg (98 % purity, 5 % yield) of the title compound.
H-NMR-Data - 1H NMR (DMSO-d6, 400 MHz) δ 12.59 (br s, 1H), 8.58 (d, 1H), 8.00 (d, 1H), 7.70 (s, 1H), 6.53 (s, 2H), 6.47 (s, 1H), 4.10 (t, 2H), 3.73 (s, 2H), 2.7-2.8 (m, 2H), 2.6-2.7 (m, 2H), 2.6-2.6 (m, 1H), 1.9-2.1 (m, 2H)
LC-MS (method 1): Rt = 1.02 min; MS (ESlpos): m/z = 472 [M+H]⁺

### Example 597

### 2-amino-5-[(rac)-1-(ethylcarbamoyl)-5',6'-dihydrospiro[pyrrolidine-3,4'-pyrrolo[1,2-b]pyrazol]-2'-yl]pyridine-3-carboxylic acid

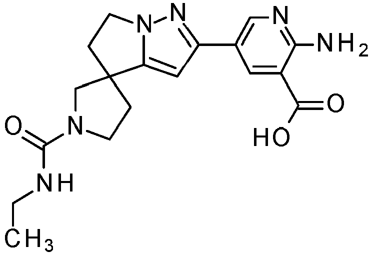

LiOH (115 mg, 4.81 mmol) was stirred in water (8.3 mL) for 15 min at rt. methyl 2-amino-5-[(*rac*)-1-(ethylcarbamoyl)-5',6'-dihydrospiro[pyrrolidine-3,4'-pyrrolo[1,2-b]pyrazol]-2'-yl]pyridine-3-carboxylate (100 mg, 260 µmol) was solubilised in methanol (8.3 mL) and added to the mixture. It was stirred overnight at rt. The mixture was evaporated and the residue was purified by preparative HPLC to give 18.0 mg (95 % purity, 18 % yield) of the title compound.
LC-MS (Method 1): Rt = 0.34 min; MS (ESlpos): m/z = 371 [M+H]⁺
¹H-NMR (500 MHz, DMSO-d6) δ [ppm]: 1.010 (1.78), 1.024 (3.54), 1.039 (1.54), 1.043 (0.88), 1.057 (1.66), 1.071 (0.83), 2.079 (0.38), 2.522 (0.78), 2.525 (0.75), 2.528 (0.69), 2.543 (16.00), 3.045 (0.63), 3.056 (0.71), 3.059 (0.66), 3.070 (0.61), 3.352 (1.07), 3.365 (1.10), 3.371 (0.95), 3.380 (0.81), 3.386 (0.94), 3.406 (2.58), 3.420 (0.48), 3.434 (0.70), 3.448 (0.64), 4.152 (0.58), 4.167 (1.00), 4.180 (0.56), 6.148 (0.48), 6.295 (1.39), 8.315 (0.34), 8.323 (0.72), 8.328 (0.86), 8.381 (0.42).

### Example 598

### 5-[(3S)-1-(pyridin-2-ylmethyl)-5',6'-dihydrospiro[pyrrolidine-3,4'-pyrrolo[1,2-b]pyrazol]-2'-yl]-3-(trifluoromethyl)pyridin-2-amine

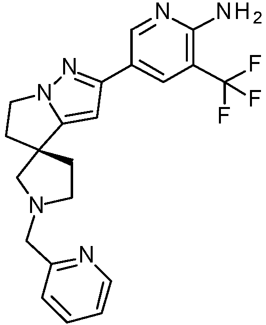

5-[(3S)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 84 % purity, 233 µmol) , 2-(chloromethyl)pyridine (35.7 mg, 280 µmol) and K2CO3 (129 mg, 934 µmol) were stirred in THF (2.5 mL) overnight at rt. KI (38.8 mg, 233 µmol) was added and the mixture was stirred for 2 h at 60°C. It was diluted with water and extracted 2x with ethyl acetate. The combined organic layers were washed with brine, dried over Na2SO4 and evaporated. The residue was purified by preparative HPLC to give 25.0 mg (99 % purity, 26 % yield) of the title compound.
1H NMR (DMSO-d6, 400 MHz) δ 8.60 (d, 1H), 8.5-8.5 (m, 1H), 8.02 (d, 1H), 7.77 (dt, 1H), 7.50 (d, 1H), 7.25 (ddd, 1H), 6.5-6.5 (m, 2H), 6.49 (s, 1H), 4.1-4.2 (m, 2H), 3.7-3.8 (m, 2H), 2.7-2.8 (m, 4H), 2.5-2.6 (m, 2H), 2.0-2.1 (m, 2H)
[α]²⁰_{D} : -62.2° (c = 1.00, DMSO)
LC-MS (method 1): Rt = 1.03 min; MS (ESlpos): m/z = 415 [M+H]⁺

### Example 599

### 5-[(3R)-1-(pyridin-2-ylmethyl)-5',6'-dihydrospiro[pyrrolidine-3,4'-pyrrolo[1,2-b]pyrazol]-2'-yl]-3-(trifluoromethyl)pyridin-2-amine

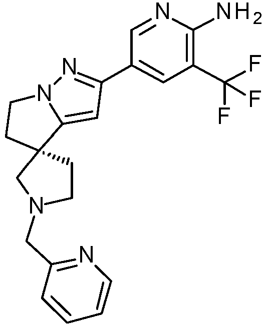

Example 599 was prepared in analogy to Example 598 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 82 % purity, 228 µmol, stereoisomer 2) and 2-(chloromethyl)pyridine (34.9 mg, 273 µmol).
1H NMR (DMSO-d6, 400 MHz) δ 8.6-8.7 (m, 1H), 8.4-8.6 (m, 1H), 7.9-8.1 (m, 1H), 7.7-7.8 (m, 1H), 7.4-7.6 (m, 1H), 7.2-7.4 (m, 1H), 6.5-6.6 (m, 2H), 6.49 (s, 1H), 4.1-4.2 (m, 2H), 3.7-3.9 (m, 2H), 2.7-2.9 (m, 4H), 2.5-2.7 (m, 2H), 2.0-2.1 (m, 2H)
[α]²⁰_{D} : +65.3° (c = 1.00, DMSO)
LC-MS (method 1): Rt = 1.03 min; MS (ESlpos): m/z = 415 [M+H]⁺

### Example 600

### 5-[(3S)-1-(pyridin-4-ylmethyl)-5',6'-dihydrospiro[pyrrolidine-3,4'-pyrrolo[1,2-b]pyrazol]-2'-yl]-3-(trifluoromethyl)pyridin-2-amine

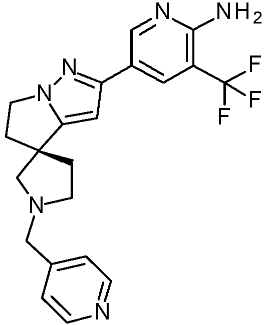

Example 600 was prepared in analogy to Example 598 using 5-[(3S)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 84 % purity, 233 µmol) and 4-(chloromethyl)pyridine-hydrochloride salt (46.0 mg, 280 µmol).
1H NMR (DMSO-d6, 400 MHz) δ 8.6-8.7 (m, 1H), 8.4-8.6 (m, 2H), 7.8-8.2 (m, 1H), 7.3-7.5 (m, 2H), 6.5-6.6 (m, 2H), 6.5-6.5 (m, 1H), 4.0-4.2 (m, 2H), 3.6-3.8 (m, 2H), 2.5-2.8 (m, 6H), 2.0-2.2 (m, 2H)
[α]²⁰_{D} : -66.6° (c = 1.00, DMSO)
LC-MS (method 1): Rt = 1.02 min; MS (ESlpos): m/z = 415 [M+H]⁺

### Example 601

### 5-[(3R)-1-(pyridin-4-ylmethyl)-5',6'-dihydrospiro[pyrrolidine-3,4'-pyrrolo[1,2-b]pyrazol]-2'-yl]-3-(trifluoromethyl)pyridin-2-amine

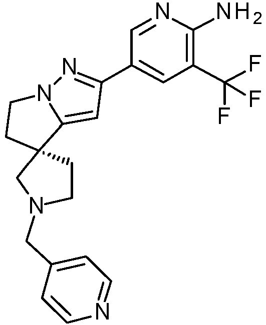

Example 601 was prepared in analogy to Example 598 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 82 % purity, 228 µmol) and 4-(chloromethyl)pyridine-hydrochloride salt (44.9 mg, 273 µmol).
1H NMR (DMSO-d6, 400 MHz) δ 8.6-8.6 (m, 1H), 8.4-8.6 (m, 2H), 7.9-8.0 (m, 1H), 7.3-7.5 (m, 2H), 6.5-6.6 (m, 2H), 6.5-6.5 (m, 1H), 4.0-4.2 (m, 2H), 3.6-3.8 (m, 2H), 2.5-2.8 (m, 6H), 2.0-2.1 (m, 2H)
[α]²⁰_{D} : +67.4° (c = 1.00, DMSO)
LC-MS (method 1): Rt = 1.02 min; MS (ESlpos): m/z = 415 [M+H]⁺

### Example 602

### 5-{(3R)-1-[3-(dimethylamino)propyl]-5',6'-dihydrospiro[pyrrolidine-3,4'-pyrrolo[1,2-b]pyrazol]-2'-yl}-3-(trifluoromethyl)pyridin-2-amine

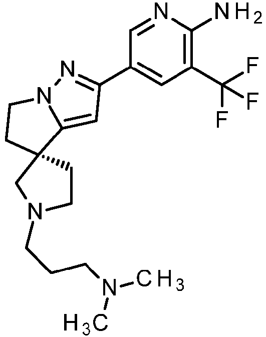

5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 79 % purity, 274 µmol), 3-chloro-N,N-dimethylpropan-1-amine-hydrochloride salt (52.1 mg, 329 µmol) and K2CO3 (152 mg, 1.10 mmol) were stirred in THF (3.0 mL) overnight at rt. Nal (82.2 mg, 548 µmol) was added and the mixture was stirred for 3 h at 70°C. It was diluted with water and extracted 2x with ethyl acetate. The combined organic layers were washed with brine, dried over Na2SO4 and evaporated. The residue was purified by preparative HPLC to give 7.00 mg (98 % purity, 6 % yield) of the title compound.
1H NMR (DMSO-d6, 400 MHz) δ 8.59 (d, 1H), 8.01 (d, 1H), 6.51 (s, 2H), 6.43 (s, 1H), 4.10 (t, 2H), 2.7-2.8 (m, 2H), 2.5-2.6 (m, 3H), 2.4-2.5 (m, 2H), 2.23 (t, 2H), 2.10 (s, 6H), 1.9-2.1 (m, 2H), 1.56 (quin, 2H)
LC-MS (method 1): Rt = 1.05 min; MS (ESlpos): m/z = 410 [M+H]⁺

### Example 603

### 5-{(3R)-1-[2-(dimethylamino)ethyl]-5',6'-dihydrospiro[pyrrolidine-3,4'-pyrrolo[1,2-b]pyrazol]-2'-yl}-3-(trifluoromethyl)pyridin-2-amine

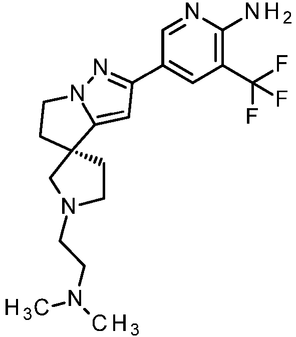

Example 603 was prepared in analogy to Example 602 using 5-[(3R)-5',6'-dihydrospiro[pyrrolidine-3,4'-pyrrolo[1,2-b]pyrazol]-2'-yl]-3-(trifluoromethyl)pyridin-2-amine-hydrochloride salt (125 mg, 79 % purity, 274 µmol) and 2-chloro-N,N-dimethylethan-1-amine-hydrochloride salt (47.4 mg, 329 µmol).
1H NMR (DMSO-d6, 400 MHz) δ 8.59 (d, 1H), 8.01 (d, 1H), 6.51 (s, 2H), 6.44 (s, 1H), 4.1-4.2 (m, 2H), 2.7-2.8 (m, 2H), 2.5-2.6 (m, 5H), 2.3-2.4 (m, 2H), 2.15 (s, 6H), 1.9-2.1 (m, 2H)
LC-MS (method 1): Rt = 0.99 min; MS (ESlpos): m/z = 396 [M+H]⁺

### Example 604

### 2-amino-1-{2'-[6-amino-5-(trifluoromethyl)pyridin-3-yl]-5',6'-dihydrospiro[pyrrolidine-3,4'-pyrrolo[1,2-b]pyrazol]-1-yl}ethan-1-one (stereoisomer 2)

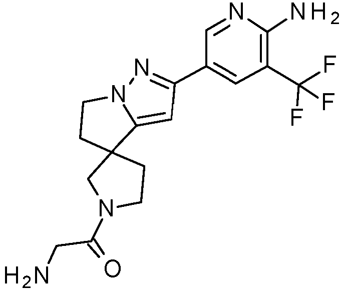

tert-butyl (2-{2'-[6-amino-5-(trifluoromethyl)pyridin-3-yl]-5',6'-dihydrospiro[pyrrolidine-3,4'-pyrrolo[1,2-b]pyrazol]-1-yl}-2-oxoethyl)carbamate (60.0 mg, 125 µmol, stereoisomer 2) was solubilised in 1,4-dioxane (600 µL), HCl (620 µL, 4.0 M in 1,4-dioxane, 2.5 mmol) was added and the mixture was stirred overnight at rt. It was evaporated and the residue was purified by preparative HPLC to give 7.00 mg (98 % purity, 14 % yield) of the title compound.
1H NMR (DMSO-d6, 400 MHz) δ 8.5-8.6 (m, 1H), 7.9-8.2 (m, 1H), 6.5-6.6 (m, 2H), 6.5-6.5 (m, 1H), 4.1-4.3 (m, 2H), 3.51 (s, 4H), 3.2-3.3 (m, 2H), 2.5-2.6 (m, 2H), 2.1-2.2 (m, 1H), 2.0-2.1 (m, 1H)
[α]²⁰_{D} : +80.0° (c = 1.00, DMSO)
LC-MS (method 1): Rt = 0.75 min; MS (ESlpos): m/z = 381 [M+H]⁺

### Example 605

### 2-amino-1-{2'-[6-amino-5-(trifluoromethyl)pyridin-3-yl]-5',6'-dihydrospiro[pyrrolidine-3,4'-pyrrolo[1,2-b]pyrazol]-1-yl}ethan-1-one (stereoisomer 1)

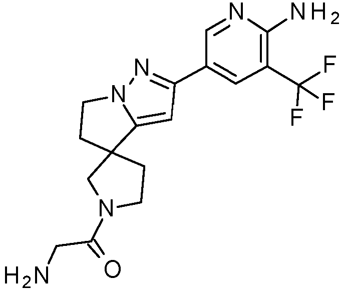

Example 605 was prepared in analogy to Example 604 using tert-butyl (2-{2'-[6-amino-5-(trifluoromethyl)pyridin-3-yl]-5',6'-dihydrospiro[pyrrolidine-3,4'-pyrrolo[1,2-b]pyrazol]-1-yl}-2-oxoethyl)carbamate (96.0 mg, 200 µmol, stereoisomer 1).
1H NMR (DMSO-d6, 400 MHz) δ 8.5-8.7 (m, 1H), 7.9-8.1 (m, 1H), 6.5-6.6 (m, 2H), 6.5-6.5 (m, 1H), 4.1-4.3 (m, 2H), 3.5-3.7 (m, 4H), 3.2-3.3 (m, 2H), 2.5-2.6 (m, 2H), 2.1-2.2 (m, 1H), 2.0-2.1 (m, 1H)
[α]²⁰_{D} : -73.6° (c = 1.00, DMSO)
LC-MS (method 1): Rt = 0.75 min; MS (ESlpos): m/z = 381 [M+H]⁺

### Example 606

### 2-amino-1-{2-[6-amino-5-(trifluoromethyl)pyridin-3-yl]-6,7-dihydrospiro[pyrazolo[5,1-c][1,4]oxazine-4,3'-pyrrolidin]-1'-yl}ethan-1-one (stereoisomer 2)

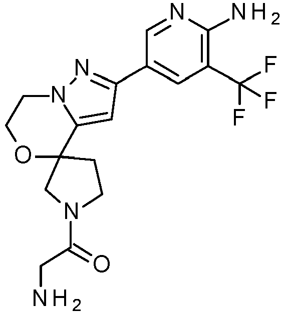

Example 606 was prepared in analogy to Example 604 using tert-butyl (2-{2-[6-amino-5-(trifluoromethyl)pyridin-3-yl]-6,7-dihydrospiro[pyrazolo[5,1-c][1,4]oxazine-4,3'-pyrrolidin]-1'-yl}-2-oxoethyl)carbamate (163 mg, 328 µmol, stereoisomer 2).
1H NMR (DMSO-d6, 400 MHz) δ 8.5-8.7 (m, 1H), 8.0-8.1 (m, 1H), 6.7-6.8 (m, 1H), 6.5-6.7 (m, 2H), 4.14 (s, 4H), 3.9-3.9 (m, 1H), 3.7-3.8 (m, 1H), 3.5-3.6 (m, 1H), 3.4-3.5 (m, 1H), 3.1-3.3 (m, 2H), 2.2-2.5 (m, 2H)
[α]²⁰_{D} : +71.5° (c = 1.00, DMSO)
LC-MS (method 1): Rt = 0.75 min; MS (ESlpos): m/z = 397 [M+H]⁺

### Example 607

### 2-amino-1-{2-[6-amino-5-(trifluoromethyl)pyridin-3-yl]-6, 7-dihydrospiro[pyrazolo[5, 1-c][1,4]oxazine-4,3'-pyrrolidin]-1'-yl}ethan-1-one (stereoisomer 1)

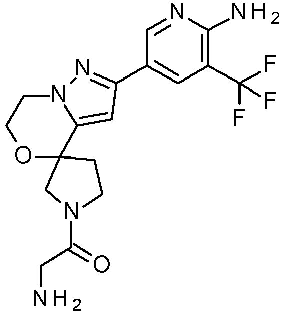

Example 607 was prepared in analogy to Example 604 using tert-butyl (2-{2-[6-amino-5-(trifluoromethyl)pyridin-3-yl]-6,7-dihydrospiro[pyrazolo[5,1-c][1,4]oxazine-4,3'-pyrrolidin]-1'-yl}-2-oxoethyl)carbamate (163 mg, 328 µmol, stereoisomer 1).
1H NMR (DMSO-d6, 400 MHz) δ 8.5-8.6 (m, 1H), 8.0-8.0 (m, 1H), 6.7-6.8 (m, 1H), 6.5-6.7 (m, 2H), 4.14 (s, 4H), 3.8-4.0 (m, 1H), 3.6-3.8 (m, 1H), 3.5-3.6 (m, 1H), 3.4-3.5 (m, 1H), 3.1-3.3 (m, 2H), 2.2-2.5 (m, 2H)
[α]²⁰_{D} : -68.6° (c = 1.00, DMSO)
LC-MS (method 1): Rt = 0.75 min; MS (ESlpos): m/z = 398 [M+H]⁺

### Example 608

### 2-amino-1-{2'-[6-amino-5-(trifluoromethyl)pyridin-3-yl]-5',6'-dihydrospiro[azetidine-3,4'-pyrrolo[1,2-b]pyrazol]-1-yl}ethan-1-one

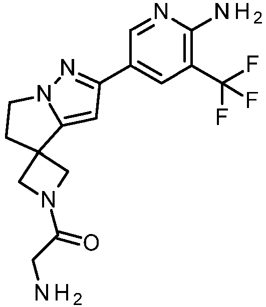

Example 608 was prepared in analogy to Example 604 using tert-butyl (2-{2'-[6-amino-5-(trifluoromethyl)pyridin-3-yl]-5',6'-dihydrospiro[azetidine-3,4'-pyrrolo[1,2-b]pyrazol]-1-yl}-2-oxoethyl)carbamate (70.0 mg, 150 µmol).
1H NMR (DMSO-d6) δ: 8.61 (d, 1H), 8.02 (d, 1H), 6.73 (s, 1H), 6.56 (s, 2H), 4.29-4.36 (m, 2H), 4.04-4.18 (m, 4H), 3.14 (s, 2H), 2.87 (m, 2H)
LC-MS (method 1): Rt = 0.73 min; MS (ESlpos): m/z = 368 [M+H]⁺

### Example 609

### (2S)-2-amino-1-{2'-[6-amino-5-(trifluoromethyl)pyridin-3-yl]-5',6'-dihydrospiro[azetidine-3,4'-pyrrolo[1,2-b]pyrazol]-1-yl}propan-1-one

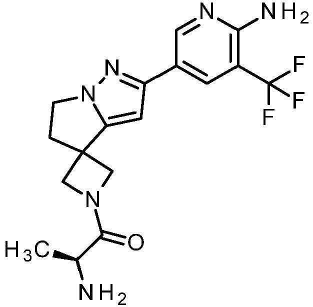

Example 609 was prepared in analogy to Example 604 using tert-butyl [(2S)-1-{2'-[6-amino-5-(trifluoromethyl)pyridin-3-yl]-5',6'-dihydrospiro[azetidine-3,4'-pyrrolo[1,2-b]pyrazol]-1-yl}-1-oxopropan-2-yl]carbamate (70.0 mg, 146 µmol).
1H NMR (DMSO-d6) δ: 8.61 (br s, 1H), 8.03 (m, 1H), 6.69-6.80 (m, 1H), 6.56 (s, 2H), 4.30-4.55 (m, 2H), 4.00-4.19 (m, 4H), 3.43 (br m, 1H), 2.86-2.95 (m, 2H), 1.11 (m, 3H)
LC-MS (method 1): Rt = 0.76 min; MS (ESlpos): m/z = 382 [M+H]⁺

### Example 610

### (2R)-2-amino-1-{2'-[6-amino-5-(trifluoromethyl)pyridin-3-yl]-5',6'-dihydrospiro[azetidine-3,4'-pyrrolo[1,2-b]pyrazol]-1-yl}propan-1-one

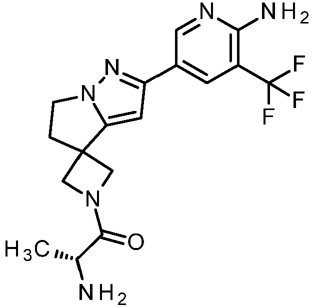

Example 610 was prepared in analogy to Example 604 using tert-butyl [(2R)-1-{2'-[6-amino-5-(trifluoromethyl)pyridin-3-yl]-5',6'-dihydrospiro[azetidine-3,4'-pyrrolo[1,2-b]pyrazol]-1-yl}-1-oxopropan-2-yl]carbamate (70.0 mg, 146 µmol).
1H NMR (DMSO-d6) δ: 8.61 (br s, 1H), 7.97-8.08 (m, 1H), 6.74 (d, 1H), 6.56 (s, 2H), 4.47 (m, 1H), 4.36 (m, 1H), 3.99-4.20 (m, 4H), 3.38 (br m, 2H), 2.83-2.93 (m, 2H), 1.10 (m, 3H)
LC-MS (method 1): Rt = 0.76 min; MS (ESlpos): m/z = 382 [M+H]⁺

### Example 611

### (rac)-2'-[6-amino-5-(1-methylpiperidin-4-yl)pyridin-3-yl]-N-ethyl-5',6'-dihydrospiro[pyrrolidine-3,4'-pyrrolo[1,2-b]pyrazole]-1-carboxamide

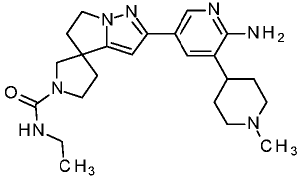

(*rac*)-2'-(2-amino-1'-methyl-1',2',3',6'-tetrahydro[3,4'-bipyridin]-5-yl)-N-ethyl-5',6'-dihydrospiro[pyrrolidine-3,4'-pyrrolo[1,2-b]pyrazole]-1-carboxamide (39.0 mg, 92.5 µmol) was solubilised in ethanol (2.0 mL), sparged with argon and Pd/C (4.92 mg, 10 % purity, 4.63 µmol) was added. The flask was purged 3x with hydrogen and it was stirred overn the weekend at rt. The mixture was filtered over Celite and the filtrate was evaporated. The residue was purified by preparative TLC to give 27.6 mg (93 % purity, 66 % yield) of the title compound.
1H-NMR (400MHz, CHLOROFORM-d): δ [ppm]= 1.17 (t, 3H), 1.90 (br s, 4H), 2.07 (br d, 3H), 2.23 (s, 1H), 2.33 (s, 4H), 2.57 (s, 1H), 2.63 (s, 1H), 3.01 (br d, 2H), 3.27 - 3.36 (m, 2H), 3.48 - 3.59 (m, 4H), 3.60 - 3.68 (m, 1H), 4.17 (br t, 1H), 4.26 (t, 2H), 4.49 (s, 2H), 6.13 - 6.16 (m, 1H), 7.76 (d, 1H), 8.33 (d, 1H).
LC-MS (method 1): Rt = 0.80 min; MS (ESlpos): m/z = 425 [M+H]⁺

### Example 612

### 5-{(rac)-1-[(5-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine

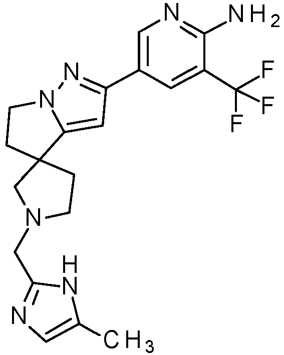

5-[(*rac*)-5',6'-dihydrospiro[pyrrolidine-3,4'-pyrrolo[1,2-b]pyrazol]-2'-yl]-3-(trifluoromethyl)pyridin-2-amine-hydrochloride salt (100 mg, 278 µmol) and 2-(chloromethyl)-5-methyl-1H-imidazole-hydrochloride salt (55.7 mg, 334 µmol) were solubilized in THF (3.0 mL), K2CO3 (154 mg, 1.11 mmol) was added and the mixture was stirred for 1 h at rt. The mixture was diluted with water and extracted 2x with EtOAc. The combined organic layers were washed with brine, dried over Na2SO4 and evaporated. The residue was purified by preparative HPLC to give 16.0 mg (99 % purity, 14 % yield) of the title compound.
1H NMR (400 MHz, DMSO-d6) δ 10.96-12.10 (m, 1H), 8.47-8.66 (m, 1H), 7.93-8.05 (m, 1H), 6.56-6.62 (m, 1H), 6.50-6.56 (m, 2H), 6.38-6.49 (m, 1H), 4.02-4.16 (m, 2H), 3.55-3.66 (m, 2H), 2.72-2.81 (m, 2H), 2.65 (s, 2H), 2.54-2.62 (m, 1H), 2.06-2.11 (m, 3H), 1.95-2.05 (m, 2H)
LC-MS (method 1): Rt = 0.88 min; MS (ESlneg): m/z = 416 [M-H]⁻

### Example 613

### 2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (isomer 1)

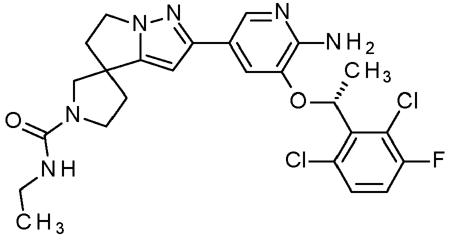

The mixture of isomers was separated by chrial HPLC to give 11.0 mg (98 % purity, 3 % yield) of the tilte compound.

Instrument: PrepCon Labomatic HPLC-4; Column: YMC Amylose SA 5µ, 250x30;eluent A: methyl tert-butyl ether + 0.1 vol % diethylamine; eluent B: methanol; isocratic: 70%A+30%B; flow: 50 mL/min; temperature: 25°C; UV: 254 nm;
Retention time: 3.5 - 5.6 min
Instrument: Thermo Fisher UltiMate 3000; Column: YMC Amylose SA 3µ, 100x4.6; eluent A: methyl tert-butyl ether + 0.1 vol % diethylamine; eluent B: methanol; isocratic: 70%A+30%B; flow: 1.4 mL/min; temperature: 25°C; UV: 254 nm
¹H-NMR (400 MHz, DMSO-d6) δ [ppm]: 1.002 (6.93), 1.020 (16.00), 1.038 (7.09), 1.232 (1.83), 1.775 (10.15), 1.792 (10.26), 2.009 (1.46), 2.028 (2.62), 2.040 (1.21), 2.046 (1.17), 2.469 (2.54), 2.518 (4.74), 2.522 (3.02), 3.022 (0.98), 3.039 (2.66), 3.053 (2.87), 3.057 (2.85), 3.071 (2.60), 3.089 (0.75), 3.359 (1.58), 3.375 (9.26), 3.384 (2.52), 3.402 (0.89), 3.437 (0.69), 3.454 (1.27), 3.462 (0.73), 3.469 (0.85), 3.479 (0.77), 4.108 (2.37), 4.126 (3.66), 4.143 (2.29), 5.785 (5.43), 6.037 (2.25), 6.053 (2.29), 6.130 (10.57), 6.138 (1.31), 6.152 (2.14), 6.166 (1.04), 7.074 (3.89), 7.079 (3.97), 7.419 (2.27), 7.442 (4.20), 7.463 (3.12), 7.546 (2.00), 7.559 (2.14), 7.569 (1.62), 7.581 (1.50), 7.879 (5.35), 7.883 (5.39).

### Example 614

### 2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (isomer 2)

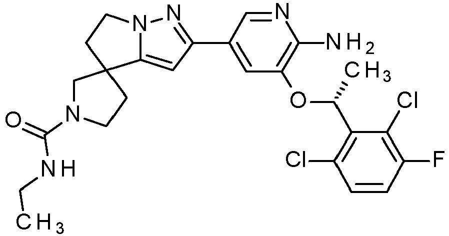

The mixture of isomers was separated by chrial HPLC to give 14.3 mg (98 % purity, 3 % yield) of the title compound.

Instrument: PrepCon Labomatic HPLC-4; Column: YMC Amylose SA 5µ, 250x30;eluent A: methyl tert-butyl ether + 0.1 vol % diethylamine; eluent B: methanol; isocratic: 70%A+30%B; flow: 50 mL/min; temperature: 25°C; UV: 254 nm;
Retention time: 14.0 - 17.0 min
Instrument: Thermo Fisher UltiMate 3000; Column: YMC Amylose SA 3µ, 100x4.6; eluent A: methyl tert-butyl ether + 0.1 vol % diethylamine; eluent B: methanol; isocratic: 70%A+30%B; flow: 1.4 mL/min; temperature: 25°C; UV: 254 nm
¹H-NMR (400 MHz, DMSO-d6) δ [ppm]: 0.752 (0.57), 0.834 (0.71), 0.851 (1.05), 0.864 (0.64), 1.006 (6.88), 1.024 (15.61), 1.041 (7.34), 1.102 (7.52), 1.120 (16.00), 1.138 (8.18), 1.161 (1.39), 1.232 (5.81), 1.256 (1.66), 1.278 (0.84), 1.295 (0.98), 1.332 (0.80), 1.348 (0.52), 1.776 (10.42), 1.793 (10.48), 2.007 (1.28), 2.020 (2.03), 2.036 (2.67), 2.054 (1.12), 2.066 (0.55), 2.467 (2.89), 2.518 (5.06), 2.522 (3.10), 2.794 (1.55), 2.812 (4.19), 2.830 (4.10), 2.848 (1.44), 3.027 (1.05), 3.045 (2.99), 3.060 (3.33), 3.063 (3.28), 3.077 (2.96), 3.095 (1.12), 3.333 (4.44), 3.351 (5.61), 3.358 (5.54), 3.394 (4.13), 3.447 (2.39), 3.465 (2.58), 3.479 (2.12), 3.486 (1.82), 3.504 (1.66), 4.109 (2.58), 4.127 (4.38), 4.144 (2.53), 5.785 (5.68), 6.022 (0.66), 6.039 (2.37), 6.056 (2.39), 6.072 (0.66), 6.131 (10.12), 6.139 (1.48), 6.153 (2.32), 6.166 (1.14), 7.079 (4.17), 7.083 (4.03), 7.412 (2.21), 7.434 (4.17), 7.456 (3.03), 7.535 (2.23), 7.547 (2.37), 7.558 (1.78), 7.570 (1.60), 7.879 (4.38), 7.882 (4.19), 8.332 (0.96).

### Example 615

### (3R)-2'-(6-amino-5-{[1-(6-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)

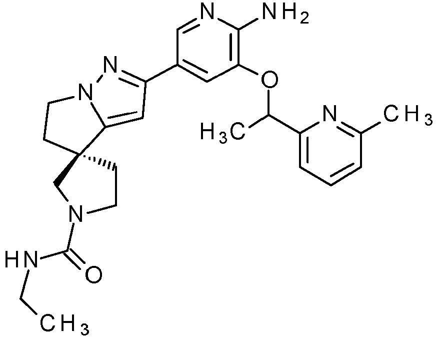

(3R)-1-(Ethylcarbamoyl)-5',6'-dihydrospiro[pyrrolidine-3,4'-pyrrolo[1,2-b]pyrazol]-2'-yl trifluoromethanesulfonate (40.0 mg, 105 µmol), {6-amino-5-[1-(6-methylpyridin-2-yl)ethoxy]pyridin-3-yl}boronic acid (enantiomer 1) (**Intermediate 432,** 182 mg, 22 % purity, 147 µmol) and potassium phosphate (630 µL, 0.50 M, 310 µmol) were combined in degassed 1,4-dioxane (1.7 mL) under argon. XPhos Pd G2 (4.12 mg, 5.24 µmol) was added and the mixture was stirred at 100 °C for 2 h. The reaction mixture was allowed to cool down, diluted with ethyl acetate and filtered. The filtrate was washed with water and brine, dried over sodium sulfate and concentrated under reduced pressure. The crude material was purified by flash chromatography (silica gel, dichloromethane / methanol (0-20%) gradient) followed by preparative TLC (gradient: dichloromethane / methanol 9:1) to give 19.8 mg (90 % purity, 37 % yield) of the title compound.
LC-MS (Method 1): Rt = 0.88 min; MS (ESlpos): m/z = 462 [M+H]⁺
¹H-NMR (400MHz, CHLOROFORM-d): δ [ppm]= 1.17 (t, 3H), 1.74 (d, 3H), 2.06 (ddd, 1H), 2.16 - 2.25 (m, 1H), 2.48 - 2.56 (m, 1H), 2.57 (s, 3H), 2.60 (br t, 1H), 3.27 - 3.35 (m, 2H), 3.48 - 3.56 (m, 3H), 3.57 - 3.65 (m, 1H), 4.16 (br t, 1H), 4.21 (t, 2H), 5.47 (d, 1H), 5.53 - 5.83 (m, 2H), 6.04 (s, 1H), 7.06 (d, 1H), 7.16 (d, 1H), 7.41 (d, 1H), 7.56 (t, 1H), 7.93 (d, 1H).

### Example 616

### (3R)-2'-(6-amino-5-{[1-(6-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)

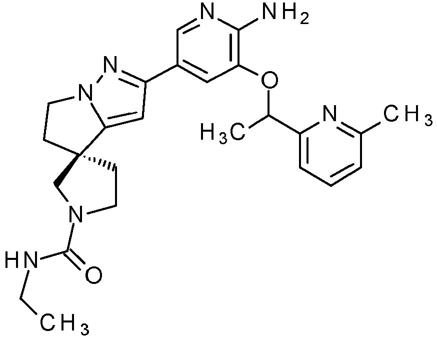

(3R)-1-(Ethylcarbamoyl)-5',6'-dihydrospiro[pyrrolidine-3,4'-pyrrolo[1,2-b]pyrazol]-2'-yl trifluoromethanesulfonate (55.3 mg, 145 µmol), {6-amino-5-[1-(6-methylpyridin-2-yl)ethoxy]pyridin-3-yl}boronic acid (enantiomer 2) (**Intermediate 433,** 158 mg, 35 % purity, 202 µmol) and potassium phosphate (870 µL, 0.50 M, 430 µmol) were combined in degassed 1,4-dioxane (2.4 mL) under argon. XPhos Pd G2 (5.69 mg, 7.23 µmol) was added and the mixture was stirred at 100 °C for 2 h. The reaction mixture was allowed to cool down, diluted with ethyl acetate and filtered. The filtrate was washed with water and brine. The organic phase was dried over sodium sulfate, filtered and concentrated under reduced pressure. The crude material was purified by flash column chromatography (silica gel, dichloromethane / methanol (0-20%) gradient) followed by preparative TLC (gradient: dichloromethane / methanol 9:1) to give 20.4 mg (95 % purity, 29 % yield) of the title compound.
LC-MS (Method 1): Rt = 0.88 min; MS (ESlneg): m/z = 460 [M-H]⁻
¹H-NMR (400MHz, CHLOROFORM-d): δ [ppm]= 1.17 (t, 3 H), 1.73 (d, 3 H), 2.06 (ddd, 1 H), 2.17 - 2.25 (m, 1 H), 2.48 - 2.66 (m, 5 H), 3.27 - 3.35 (m, 2 H), 3.47 - 3.55 (m, 3 H), 3.57 - 3.65 (m, 1 H), 4.16 (br t, 1 H), 4.21 (t, H), 5.39 - 5.53 (m, 2 H), 6.05 (s, 1 H), 7.05 (d, 1 H), 7.17 (d, 1 H), 7.40 (d, 1 H), 7.55 (t, 1 H), 7.94 (d, 1 H).

### Example 617

### (3R)-2'-(6-amino-5-{[1-(5-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)

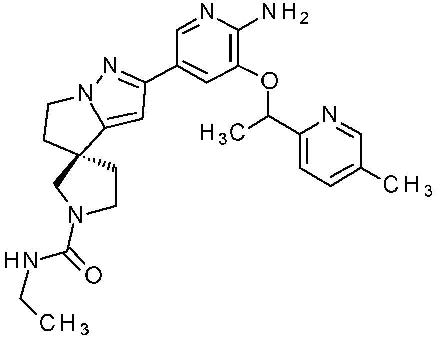

The compound was prepared similarly to **Example 615** using (3R)-1-(ethylcarbamoyl)-5',6'-dihydrospiro[pyrrolidine-3,4'-pyrrolo[1,2-b]pyrazol]-2'-yl trifluoromethanesulfonate (40.4 mg, 106 µmol) and {6-amino-5-[1-(5-methylpyridin-2-yl)ethoxy]pyridin-3-yl}boronic acid (enantiomer 1) (**Intermediate 434,** 162 mg, 25 % purity, 148 µmol) to give 12.9 mg (95 % purity, 25 % yield) of the title compound.
LC-MS (Method 1): Rt = 0.88 min; MS (ESlpos): m/z = 462 [M+H]⁺
¹H-NMR (400MHz, DMSO-d6): δ [ppm]= 1.02 (t, 3H), 1.59 (d, 3H), 2.01 (br d, 2H), 2.25 (s, 3H), 2.98 - 3.10 (m, 2H), 3.43 - 3.52 (m, 1H), 4.12 (t, 2H), 5.48 (d, 1H), 5.86 (s, 2H), 6.15 (s, 1H), 6.21 (s, 1H), 7.20 (d, 1H), 7.38 (d, 1H), 7.59 (dd, 1H), 7.85 (d, 1H), 8.34 - 8.39 (m, 1H).

### Example 618

### (3R)-2'-(6-amino-5-{[1-(5-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)

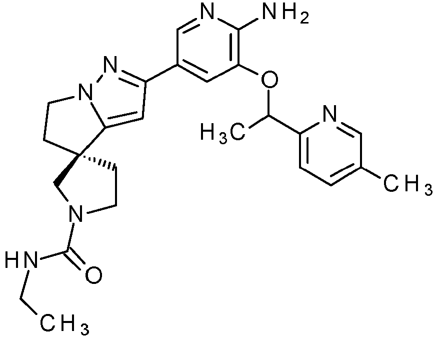

The compound was prepared similarly to **Example 615** using (3R)-1-(ethylcarbamoyl)-5',6'-dihydrospiro[pyrrolidine-3,4'-pyrrolo[1,2-b]pyrazol]-2'-yl trifluoromethanesulfonate (44.7 mg, 117 µmol) and {6-amino-5-[1-(5-methylpyridin-2-yl)ethoxy]pyridin-3-yl}boronic acid (enantiomer 2) (**Intermediate 435,** 179 mg, 25 % purity, 163 µmol) to give 13.6 mg (95 % purity, 24 % yield) of the title compound.
LC-MS (Method 1): Rt = 0.88 min; MS (ESlpos): m/z = 462 [M+H]⁺
¹H-NMR (400MHz, CHLOROFORM-d): δ [ppm]= 1.17 (t, 3H), 1.73 (d, 3H), 2.07 (ddd, 1H), 2.18 - 2.26 (m, 1H), 2.32 (s, 3H), 2.49 - 2.67 (m, 2H), 3.26 - 3.36 (m, 2H), 3.47 - 3.56 (m, 3H), 3.61 (td, 1H), 4.13 - 4.27 (m, 3H), 5.51 (q, 1H), 5.58 - 5.93 (m, 2H), 6.05 (s, 1H), 7.24 (s, 1H), 7.39 - 7.44 (m, 1H), 7.48 (dd, 1H), 7.88 (d, 1H), 8.41 (d, 1H).

### Example 619

### (3R)-2'-(6-amino-5-{[1-(4-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)

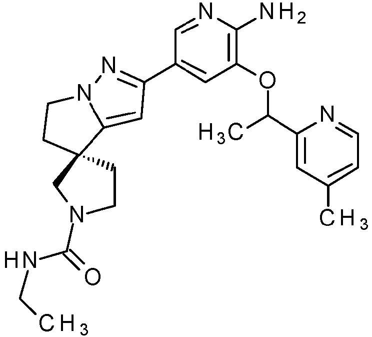

(3R)-1-(Ethylcarbamoyl)-5',6'-dihydrospiro[pyrrolidine-3,4'-pyrrolo[1,2-b]pyrazol]-2'-yl trifluoromethanesulfonate (28.5 mg, 74.6 µmol), {6-amino-5-[1-(4-methylpyridin-2-yl)ethoxy]pyridin-3-yl}boronic acid (enantiomer 1) (**Intermediate 436,** 143 mg, 20 % purity, 105 µmol) and potassium phosphate (448 µL, 0.50 M, 224 µmol) were combined in degassed 1,4-dioxane (1.2 mL) under argon. XPhos Pd G2 (2.9 mg, 4 µmol) was added and the mixture was stirred at 100 °C for 2 h. The reaction mixture was allowed to cool down, diluted with ethyl acetate and filtered. The filtrated was washed with water and brine. The organic phase was dried over sodium sulfate, filtered and concentrated under reduced pressure. The crude material was purified by preparative TLC (gradient: dichloromethane / methanol 9:1) to give 7.2 mg (95 % purity, 20 % yield) of the title compound.
LC-MS (Method 1): Rt = 0.87 min; MS (ESlpos): m/z = 462 [M+H]⁺
¹H-NMR (400MHz, CHLOROFORM-d): δ [ppm]= 1.16 (t, 3H), 1.70 (d, 3H), 2.00 - 2.09 (m, 1H), 2.20 (br d, 1H), 2.31 (s, 3H), 2.45 - 2.55 (m, 1H), 2.56 - 2.66 (m, 1H), 3.25 - 3.37 (m, 2H), 3.46 - 3.55 (m, 3H), 3.56 - 3.65 (m, 1H), 4.13 - 4.26 (m, 3H), 4.87 (s, 2H), 5.47 (q, 1H), 6.03 (s, 1H), 7.00 (dd, 1H), 7.18 (s, 1H), 7.24 (d, 1H), 8.03 (d, 1H), 8.43 (d, 1H).

### Example 620

### (3R)-2'-(6-amino-5-{[1-(4-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)

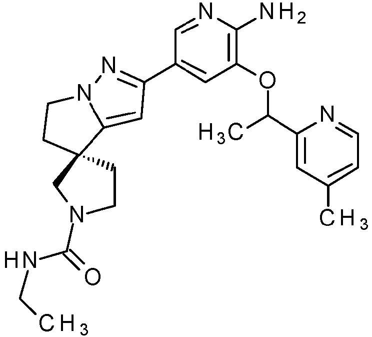

(3R)-1-(Ethylcarbamoyl)-5',6'-dihydrospiro[pyrrolidine-3,4'-pyrrolo[1,2-b]pyrazol]-2'-yl trifluoromethanesulfonate (28.7 mg, 75.1 µmol) and {6-amino-5-[1-(4-methylpyridin-2-yl)ethoxy]pyridin-3-yl}boronic acid (enantiomer 2) (**Intermediate 437,** 144 mg, 20 % purity, 105 µmol) and potassium phosphate (450 µL, 0.50 M, 225 µmol) were combined in degassed 1,4-dioxane (1.2 mL) under argon. XPhos Pd G2 (2.9 mg, 4 µmol) was added and the mixture was stirred at 100 °C for 2 h. The reaction mixture was allowed to cool down, diluted with ethyl acetate and filtered. The filtrate was washed with water and brine. The organic phase was dried over sodium sulfate and concentrated under reduced pressure. The crude material was purified by flash column chromatography (silica gel, dichloromethane / methanol (0-15%) gradient) followed by preparative TLC (1 plate, 20x20cm, gradient: dichloromethane / methanol 9:1) to give 6.7 mg (95 % purity, 18 % yield) of the title compound.
LC-MS (Method 1): Rt = 0.87 min; MS (ESlpos): m/z = 462 [M+H]⁺
¹H-NMR (400MHz, CHLOROFORM-d): δ [ppm]= 1.16 (t, 3H), 1.70 (d, 3H), 2.00 - 2.10 (m, 1H), 2.14 - 2.29 (m, 1H), 2.31 (s, 3H), 2.46 - 2.54 (m, 1H), 2.56 - 2.63 (m, 1H), 3.30 (qd, 2H), 3.45 - 3.56 (m, 3H), 3.56 - 3.66 (m, 1H), 4.12 - 4.26 (m, 3H), 4.87 (s, 2H), 5.47 (d, 1H), 6.04 (s, 1H), 7.00 (dd, 1H), 7.18 (s, 1H), 7.24 - 7.26 (m, 1H), 8.02 (d, 1H), 8.43 (d, 1H).

### Example 621

### (3R)-2'-(6-amino-5-{[1-(3-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)

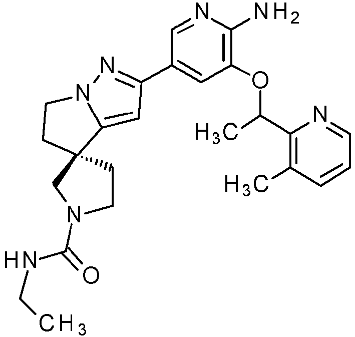

The compound was prepared similarly to **Example 619** using (3R)-1-(ethylcarbamoyl)-5',6'-dihydrospiro[pyrrolidine-3,4'-pyrrolo[1,2-b]pyrazol]-2'-yl trifluoromethanesulfonate (13.1 mg, 34.4 µmol) and {6-amino-5-[1-(3-methylpyridin-2-yl)ethoxy]pyridin-3-yl}boronic acid (enantiomer 1) (**Intermediate 438,** 87.6 mg, 15 % purity, 48.1 µmol) to give 2.7 mg (95 % purity, 16 % yield) of the title compound.
LC-MS (Method 1): Rt = 0.85 min; MS (ESlpos): m/z = 462 [M+H]⁺
¹H-NMR (400MHz, CHLOROFORM-d): δ [ppm]= 1.17 (t, 3H), 1.76 (d, 3H), 2.01 - 2.12 (m, 1H), 2.17 - 2.26 (m, 1H), 2.39 (s, 3H), 2.48 - 2.57 (m, 1H), 2.58 - 2.68 (m, 1H), 3.26 - 3.36 (m, 2H), 3.50 - 3.57 (m, 3H), 3.58 - 3.67 (m, 1H), 4.13 - 4.20 (m, 1H), 4.23 (t, 2H), 4.87 (s, 2H), 5.67 (d, 1H), 6.05 (s, 1H), 7.14 (dd, 1H), 7.32 (d, 1H), 7.44 (br d, 1H), 8.01 (d, 1H), 8.46 (dd, 1H).

### Example 622

### (3R)-2'-(6-amino-5-{[1-(3-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)

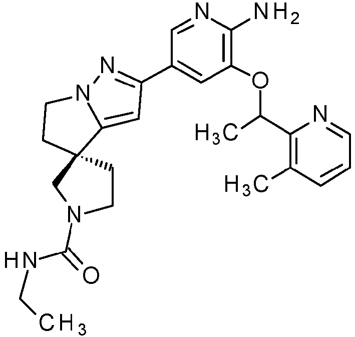

The compound was prepared similarly to **Example 619** using (3R)-1-(ethylcarbamoyl)-5',6'-dihydrospiro[pyrrolidine-3,4'-pyrrolo[1,2-b]pyrazol]-2'-yl trifluoromethanesulfonate (16.8 mg, 43.8 µmol) and {6-amino-5-[1-(3-methylpyridin-2-yl)ethoxy]pyridin-3-yl}boronic acid (enantiomer 2) (**Intermediate 439,** 83.8 mg, 20 % purity, 61.4 µmol) to give 7.7 mg (95 % purity, 36 % yield) of the title compound.
LC-MS (Method 1): Rt = 0.85 min; MS (ESlpos): m/z = 462 [M+H]⁺
¹H-NMR (400MHz, CHLOROFORM-d): δ [ppm]= 1.17 (t, 3H), 1.76 (d, 3H), 2.07 (br d, 1H), 2.17 - 2.27 (m, 1H), 2.39 (s, 3H), 2.50 - 2.57 (m, 1H), 2.59 - 2.66 (m, 1H), 3.26 - 3.37 (m, 2H), 3.46 - 3.57 (m, 3H), 3.58 - 3.67 (m, 1H), 4.11 - 4.19 (m, 1H), 4.23 (t, 2H), 4.84 (s, 2H), 5.67 (d, 1H), 6.06 (s, 1H), 7.14 (dd, 1H), 7.34 (d, 1H), 7.44 (s, 1H), 8.01 (d, 1H), 8.47 (d, 1H).

### Example 623

### (3R)-2'-(6-amino-5-{[1-(1,4-dimethyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)

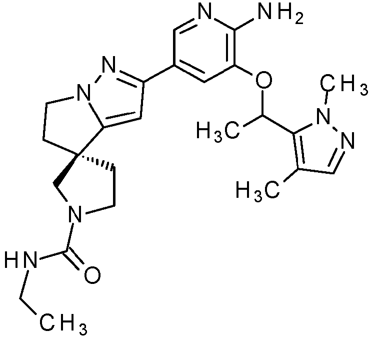

(3R)-1-(Ethylcarbamoyl)-5',6'-dihydrospiro[pyrrolidine-3,4'-pyrrolo[1,2-b]pyrazol]-2'-yl trifluoromethanesulfonate (51.5 mg, 135 µmol), 3-[1-(1,4-dimethyl-1H-pyrazol-5-yl)ethoxy]-5-(4,4,5,5-tetramethyl-1,3,2-dioxaborolan-2-yl)pyridin-2-amine (enantiomer 1) (**Intermediate 440,** 135 mg, 50 % purity, 188 µmol) and potassium phosphate (807 µL, 0.50 M, 404 µmol) were combined in degassed 1,4-dioxane (2.2 mL) under argon. XPhos Pd G2 (5.2 mg, 7 µmol) was added and the mixture was stirred at 100 °C for 2 h. The reaction mixture was allowed to cool down, diluted with ethyl acetate and filtered. The filtrate was washed with water and brine. The organic phase was dried over sodium sufate, filtered and concentrated under reduced pressure. The crude material was purified by flash column chromatography (silica gel, dichloromethane / methanol (0-20%) gradient) and TLC (gradient: dichloromethane / methanol 85:15) to give 20.3 mg (97 % purity, 31 % yield) of the title compound.
LC-MS (Method 1): Rt = 0.84 min; MS (ESlpos): m/z = 465 [M+H]⁺
¹H-NMR (400MHz, DMSO-d6): δ [ppm]= 1.02 (t, 3H), 1.66 (d, 3H), 1.97 - 2.07 (m, 2H), 2.08 (s, 3H), 3.00 - 3.11 (m, 2H), 3.43 - 3.52 (m, 1H), 3.85 (s, 3H), 4.14 (s, 2H), 5.71 (d, 1H), 5.85 (s, 2H), 6.12 - 6.18 (m, 1H), 6.19 (s, 1H), 7.11 (s, 1H), 7.14 (d, 1H), 7.87 (d, 1H).
[α]²⁰_{D} : -11.5° (c = 1.00, MeOH)

### Example 624

### (3R)-2'-(6-amino-5-{[1-(1,4-dimethyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)

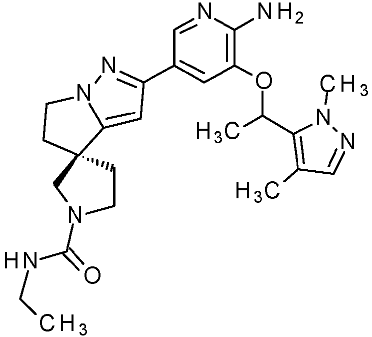

The compound was prepared similarly to **Example 623** using (3R)-1-(ethylcarbamoyl)-5',6'-dihydrospiro[pyrrolidine-3,4'-pyrrolo[1,2-b]pyrazol]-2'-yl trifluoromethanesulfonate (41.9 mg, 110 µmol) and 3-[1-(1,4-dimethyl-1H-pyrazol-5-yl)ethoxy]-5-(4,4,5,5-tetramethyl-1,3,2-dioxaborolan-2-yl)pyridin-2-amine (enantiomer 2) (110 mg, 50 % purity, 154 µmol) to give 22.2 mg (98 % purity, 43 % yield) of the title compound.
LC-MS (Method 1): Rt = 0.84 min; MS (ESlpos): m/z = 465 [M+H]⁺
¹H-NMR (400MHz, DMSO-d6): δ [ppm]= 1.02 (t, 3H), 1.66 (d, 3H), 1.98 - 2.06 (m, 2H), 2.08 (s, 3H), 2.99 - 3.10 (m, 2H), 3.37 - 3.42 (m, 3H), 3.43 - 3.53 (m, 1H), 3.86 (s, 3H), 4.14 (t, 2H), 5.69 - 5.75 (m, 1H), 5.85 (s, 2H), 6.12 - 6.18 (m, 1H), 6.19 (s, 1H), 7.12 (s, 1H), 7.14 (d, 1H), 7.87 (d, 1H).
[α]²⁰_{D} : +107.0° (c = 1.00, MeOH)

### Example 625

### (3R)-2'-(6-amino-5-{[-1-(1,4-dimethyl-1H-pyrazol-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1)

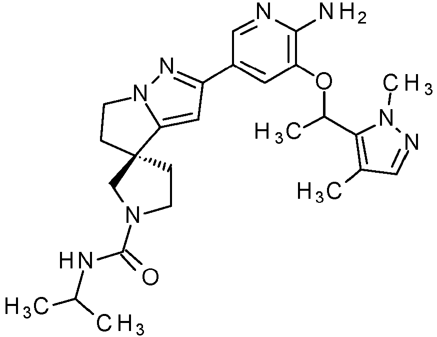

The compound was prepared similarly to **Example 623** using (3R)-1-[(propan-2-yl)carbamoyl]-5',6'-dihydrospiro[pyrrolidine-3,4'-pyrrolo[1,2-b]pyrazol]-2'-yl trifluoromethanesulfonate (34.3 mg, 86.4 µmol) and {6-amino-5-[1-(1,4-dimethyl-1H-pyrazol-5-yl)ethoxy]pyridin-3-yl}boronic acid (enantiomer 1) **(Intermediate 441,** 167 mg, 20 % purity, 121 µmol) to give 12.9 mg (85 % purity, 26 % yield) of the title compound.
LC-MS (Method 1): Rt = 0.88 min; MS (ESlpos): m/z = 480 [M+H]⁺
¹H-NMR (400MHz, DMSO-d6): δ [ppm]= 1.06 (dd, 6H), 1.66 (d, 3H), 2.03 (dt, 2H), 2.08 (s, 3H), 3.36 - 3.42 (m, 3H), 3.44 - 3.53 (m, 1H), 3.70 - 3.81 (m, 1H), 3.86 (s, 3H), 4.10 - 4.16 (m, 2H), 5.71 (d, 1H), 5.80 - 5.90 (m, 3H), 6.19 - 6.20 (m, 1H), 7.11 - 7.13 (m, 1H), 7.13 - 7.16 (m, 1H), 7.87 (d, 1H).

### Example 626

### (3R)-2'-(6-amino-5-{[1-(1,4-dimethyl-1H-pyrazol-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 2)

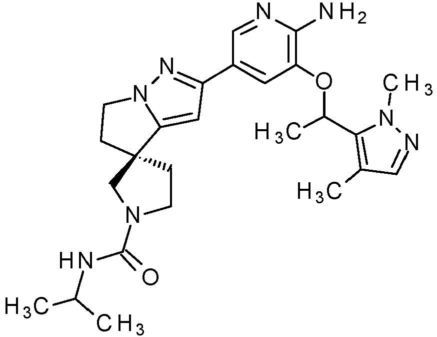

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (29.7 mg, 75.0 µmol), {6-amino-5-[1-(1,4-dimethyl-1H-pyrazol-5-yl)ethoxy]pyridin-3-yl}boronic acid (enantiomer 2) **(Intermediate 443,** 116 mg, 25 % purity, 105 µmol) and potassium phosphate (450 µL, 0.50 M, 225 µmol) were combined in degassed 1,4-dioxane (1.2 mL) under argon. XPhos Pd G2 (2.9 mg, 4 µmol) was added and the mixture was stirred at 100 °C for 2 h. The reaction mixture was allowed to cool down, diluted with ethyl acetate and filtered. The filtrate was washed with water and brine. The organic phase was dried over sodium sulfate, filtered and concentrated under reduced pressure. The crude material was purified by flash column chromatography (silica gel, dichloromethane / methanol (0-20%) gradient) and preparative TLC (gradient: dichloromethane / methanol 85:15) to give 20.2 mg (90 % purity, 50 % yield) of the title compound.
LC-MS (Method 1): Rt = 0.89 min; MS (ESlpos): m/z = 480 [M+H]⁺
¹H-NMR (400MHz, DMSO-d6): δ [ppm]= 1.06 (dd, 6H), 1.66 (d, 3H), 2.03 (dt, 2H), 2.08 (s, 3H), 3.36 - 3.42 (m, 3H), 3.44 - 3.53 (m, 1H), 3.70 - 3.81 (m, 1H), 3.86 (s, 3H), 4.10 - 4.16 (m, 2H), 5.71 (d, 1H), 5.80 - 5.90 (m, 3H), 6.19 - 6.20 (m, 1H), 7.11 - 7.13 (m, 1H), 7.13 - 7.16 (m, 1H), 7.87 (d, 1H).

### Example 627

### (3R)-2'-{6-amino-5-[(1S)-1-(2,6-difluorophenyl)ethoxy]pyridin-3-yl}-N-ethyl-5',6'-dihydrospiro[pyrrolidine-3,4'-pyrrolo[1,2-b]pyrazole]-1-carboxamide

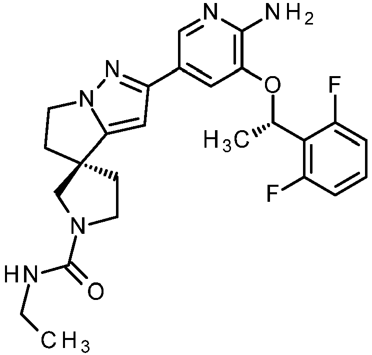

(3R)-1-(Ethylcarbamoyl)-5',6'-dihydrospiro[pyrrolidine-3,4'-pyrrolo[1,2-b]pyrazol]-2'-yl trifluoromethanesulfonate (50.0 mg, 131 µmol), {6-amino-5-[(1S)-1-(2,6-difluorophenyl)ethoxy]pyridin-3-yl}boronic acid **(Intermediate 444,** 179 mg, 30 % purity, 183 µmol) and potassium phosphate (780 µL, 0.50 M, 390 µmol) were combined in degassed 1,4-dioxane (2.2 mL) under argon. XPhos Pd G2 (5.14 mg, 6.54 µmol) was added and the mixture was stirred at 100 °C for 2 h. The reaction mixture was allowed to cool down, diluted with ethyl acetate and filtered. The filtrate was washed with water and brine. The organic phase was dried over sodium sulfate, filtered and concentrated under reduced pressure. The crude material was purified by flash column chromatography (silica gel, dichloromethane / methanol (0-20%) gradient) followed by preparative TLC (gradient: dichloromethane / methanol 85:15) to give 38.6 mg (98 % purity, 60 % yield) of the title compound.
LC-MS (Method 1): Rt = 1.01 min; MS (ESlneg): m/z = 481 [M-H]⁻
¹H-NMR (400 MHz, DMSO-d6) δ [ppm]: 1.005 (6.76), 1.022 (15.75), 1.040 (6.99), 1.064 (2.82), 1.230 (0.46), 1.739 (8.10), 1.755 (7.96), 2.014 (1.17), 2.028 (1.96), 2.044 (2.52), 2.061 (0.97), 2.074 (0.48), 2.332 (1.07), 2.336 (0.47), 2.518 (6.40), 2.523 (3.70), 2.678 (0.47), 3.026 (0.83), 3.043 (2.65), 3.057 (2.93), 3.061 (2.83), 3.075 (2.58), 3.093 (0.75), 3.309 (1.22), 3.316 (1.55), 3.384 (13.46), 3.401 (1.75), 3.452 (0.82), 3.471 (1.25), 3.478 (0.83), 3.485 (0.97), 3.492 (0.76), 3.510 (0.44), 4.127 (2.42), 4.145 (4.06), 4.162 (2.32), 5.660 (5.50), 5.754 (16.00), 5.790 (0.57), 5.806 (1.90), 5.823 (1.90), 5.839 (0.55), 6.149 (1.08), 6.162 (2.12), 6.176 (1.08), 6.195 (10.62), 7.077 (2.57), 7.098 (5.40), 7.119 (3.05), 7.299 (3.98), 7.303 (3.98), 7.355 (0.51), 7.371 (1.14), 7.376 (0.94), 7.392 (1.92), 7.409 (0.95), 7.413 (1.04), 7.430 (0.43), 7.884 (5.51), 7.888 (5.42).

### Example 628

### (3R)-2'-{6-amino-5-[(1R)-1-(5-fluoropyridin-2-yl)ethoxy]pyridin-3-yl}-N-ethyl-5',6'-dihydrospiro[pyrrolidine-3,4'-pyrrolo[1,2-b]pyrazole]-1-carboxamide

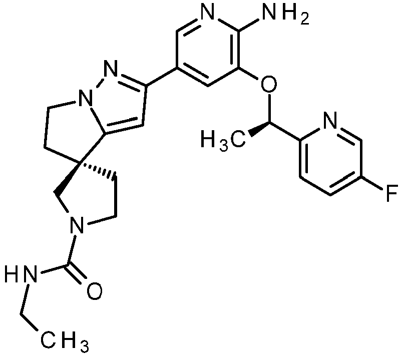

The compound was prepared similarly to **Example 627** using (3R)-1-(ethylcarbamoyl)-5',6'-dihydrospiro[pyrrolidine-3,4'-pyrrolo[1,2-b]pyrazol]-2'-yl trifluoromethanesulfonate (50.0 mg, 131 µmol) and {6-amino-5-[(1R)-1-(5-fluoropyridin-2-yl)ethoxy]pyridin-3-yl}boronic acid **(Intermediate 445,** 154 mg, 33 % purity, 183 µmol) to give 27.0 mg (98 % purity, 43 % yield) of the title compound.
LC-MS (Method 1): Rt = 0.88 min; MS (ESlpos): m/z = 467 [M+H]⁺
¹H-NMR (400 MHz, DMSO-d6) δ [ppm]: 0.996 (7.01), 1.015 (15.96), 1.033 (7.37), 1.232 (0.43), 1.597 (10.31), 1.613 (10.22), 2.004 (1.27), 2.017 (2.02), 2.033 (2.68), 2.051 (1.08), 2.063 (0.52), 2.466 (2.50), 2.518 (5.65), 2.523 (3.45), 3.013 (0.92), 3.031 (2.92), 3.045 (3.21), 3.049 (3.17), 3.063 (2.83), 3.081 (0.84), 3.359 (3.50), 3.370 (9.80), 3.385 (2.46), 3.397 (0.83), 3.403 (0.94), 3.446 (0.77), 3.464 (1.32), 3.471 (0.85), 3.478 (1.00), 3.490 (0.80), 3.504 (0.49), 4.109 (2.68), 4.127 (4.75), 4.144 (2.57), 5.542 (0.67), 5.558 (2.42), 5.575 (2.40), 5.590 (0.66), 5.758 (16.00), 5.893 (6.63), 6.132 (1.18), 6.145 (2.38), 6.159 (1.16), 6.240 (10.86), 7.233 (4.45), 7.237 (4.29), 7.601 (1.72), 7.612 (1.80), 7.623 (2.54), 7.634 (2.40), 7.715 (1.53), 7.721 (1.70), 7.736 (2.58), 7.744 (2.76), 7.758 (1.11), 7.765 (1.14), 7.877 (6.16), 7.881 (6.03), 8.548 (4.34), 8.556 (4.26).
[α]²⁰_{D} : +121.1° (c = 1.00, MeOH)

### Example 629

### (3R)-2'-{6-amino-5-[(1R)-1-(3-fluoropyridin-2-yl)ethoxy]pyridin-3-yl}-N-ethyl-5',6'-dihydrospiro[pyrrolidine-3,4'-pyrrolo[1,2-b]pyrazole]-1-carboxamide

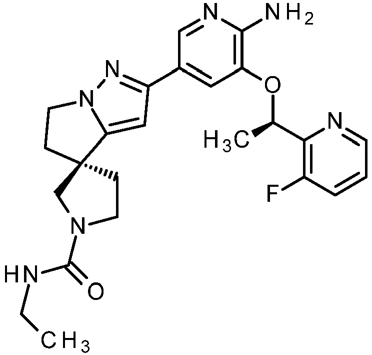

The compound was prepared similarly to **Example 627** using (3R)-1-(ethylcarbamoyl)-5',6'-dihydrospiro[pyrrolidine-3,4'-pyrrolo[1,2-b]pyrazol]-2'-yl trifluoromethanesulfonate (50.0 mg, 131 µmol) and {6-amino-5-[(1R)-1-(3-fluoropyridin-2-yl)ethoxy]pyridin-3-yl}boronic acid **(Intermediate 446,** 145 mg, 35 % purity, 183 µmol) to give 24.9 mg (98 % purity, 40 % yield) of the title compound.
LC-MS (Method 1): Rt = 0.86 min; MS (ESlneg): m/z = 464 [M-H]⁻
¹H-NMR (400 MHz, DMSO-d6) δ [ppm]: 1.002 (6.79), 1.020 (16.00), 1.038 (7.24), 1.232 (0.41), 1.494 (1.19), 1.684 (9.66), 1.700 (9.54), 2.013 (1.18), 2.026 (1.93), 2.042 (2.56), 2.060 (1.03), 2.072 (0.50), 2.332 (1.88), 2.336 (0.82), 2.518 (11.36), 2.523 (7.55), 2.673 (1.91), 2.678 (0.80), 3.020 (0.84), 3.038 (2.79), 3.052 (3.02), 3.056 (3.01), 3.070 (2.71), 3.088 (0.80), 3.366 (1.59), 3.382 (10.59), 3.391 (2.57), 3.409 (0.80), 3.449 (0.70), 3.468 (1.23), 3.475 (0.78), 3.482 (0.90), 3.493 (0.74), 3.508 (0.42), 4.124 (2.54), 4.142 (4.18), 4.159 (2.45), 5.680 (6.07), 5.743 (0.69), 5.759 (15.67), 5.775 (2.31), 5.791 (0.66), 6.141 (1.12), 6.155 (2.24), 6.169 (1.10), 6.212 (11.44), 7.308 (4.24), 7.312 (4.25), 7.435 (1.30), 7.445 (2.11), 7.456 (2.51), 7.467 (2.55), 7.478 (1.56), 7.714 (1.67), 7.717 (1.68), 7.735 (1.58), 7.739 (1.73), 7.740 (1.92), 7.744 (1.75), 7.761 (1.46), 7.765 (1.37), 7.884 (6.03), 7.888 (5.99), 8.436 (1.65), 8.440 (2.68), 8.444 (1.75), 8.447 (1.72), 8.451 (2.65), 8.455 (1.56).

### Example 630

### (3R)-2'-{6-amino-5-[(1R)-1-(2,6-difluorophenyl)ethoxy]pyridin-3-yl}-N-ethyl-5',6'-dihydrospiro[pyrrolidine-3,4'-pyrrolo[1,2-b]pyrazole]-1-carboxamide

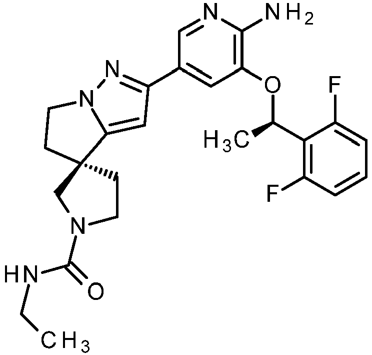

The compound was prepared similarly to **Example 627** using (3R)-1-(ethylcarbamoyl)-5',6'-dihydrospiro[pyrrolidine-3,4'-pyrrolo[1,2-b]pyrazol]-2'-yl trifluoromethanesulfonate (50.0 mg, 131 µmol) and {6-amino-5-[(1R)-1-(2,6-difluorophenyl)ethoxy]pyridin-3-yl}boronic acid **(Intermediate 447,** 146 mg, 37 % purity, 183 µmol) to give 44.0 mg (98 % purity, 68 % yield) of the title compound.
LC-MS (Method 1): Rt = 1.01 min; MS (ESlneg): m/z = 481 [M-H]⁻
¹H-NMR (400 MHz, DMSO-d6) δ [ppm]: 1.003 (6.77), 1.021 (15.24), 1.038 (6.90), 1.066 (3.42), 1.233 (0.40), 1.739 (8.79), 1.755 (8.78), 2.016 (1.35), 2.030 (2.09), 2.043 (2.66), 2.061 (1.13), 2.074 (0.51), 2.332 (1.66), 2.522 (6.39), 3.022 (0.91), 3.039 (2.88), 3.054 (3.24), 3.057 (3.20), 3.072 (2.78), 3.089 (0.82), 3.295 (0.82), 3.304 (1.42), 3.363 (2.15), 3.366 (2.42), 3.384 (11.46), 3.410 (0.94), 3.448 (0.76), 3.466 (1.34), 3.473 (0.85), 3.480 (0.97), 3.488 (0.82), 3.506 (0.47), 4.128 (2.66), 4.145 (4.28), 4.162 (2.54), 5.663 (6.20), 5.758 (16.00), 5.787 (0.66), 5.805 (2.10), 5.821 (2.10), 5.837 (0.63), 6.145 (1.22), 6.159 (2.36), 6.172 (1.21), 6.195 (10.10), 7.083 (2.79), 7.105 (5.73), 7.126 (3.18), 7.298 (4.36), 7.302 (4.33), 7.362 (0.54), 7.378 (1.28), 7.382 (1.06), 7.399 (2.03), 7.415 (1.02), 7.420 (1.02), 7.436 (0.45), 7.887 (5.80), 7.891 (5.69).

### Example 631

### (3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)

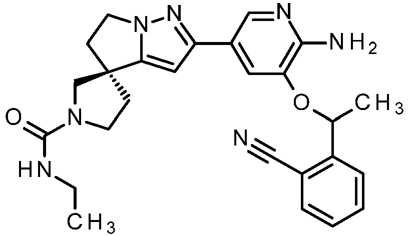

(3R)-1-(Ethylcarbamoyl)-5',6'-dihydrospiro[pyrrolidine-3,4'-pyrrolo[1,2-b]pyrazol]-2'-yl trifluoromethanesulfonate (50.0 mg, 131 µmol), 2-(1-{[2-amino-5-(4,4,5,5-tetramethyl-1,3,2-dioxaborolan-2-yl)pyridin-3-yl]oxy}ethyl)benzonitrile **(Intermediate 448,** 167 mg, 40 % purity, 183 µmol) and potassium phosphate (780 µL, 0.50 M, 390 µmol) were suspended in degassed 1,4-dioxane under argon. XPhos Pd G2 (5.14 mg, 6.54 µmol) was added and the mixture was stirred at 100 °C for 2 h. The reaction mixture was allowed to cool down, diluted with ethyl acetate and filtered. The filtrate was washed with water and brine. The organic phase was dried over sodium sulfate, filtered and concentrated under reduced pressure. The crude product was purified by flash column chromatography (silica gel, dichloromethane / methanol (0-10%) gradient) to give 24.7 mg (96 % purity, 38 % yield) of the title compound.
LC-MS (Method 1): Rt = 0.94 min; MS (ESlpos): m/z = 472 [M+H]⁺
¹H-NMR (400 MHz, CHLOROFORM-d) δ [ppm]: 0.887 (0.45), 1.156 (5.69), 1.167 (6.06), 1.175 (12.99), 1.185 (12.43), 1.192 (6.08), 1.203 (5.98), 1.261 (3.60), 1.292 (0.48), 1.762 (16.00), 1.779 (15.93), 2.040 (0.53), 2.055 (0.90), 2.070 (1.27), 2.085 (1.26), 2.101 (0.80), 2.204 (0.68), 2.220 (1.40), 2.237 (1.04), 2.252 (0.98), 2.269 (0.54), 2.503 (0.48), 2.518 (1.04), 2.521 (0.96), 2.536 (1.49), 2.551 (2.11), 2.568 (1.02), 2.593 (1.01), 2.610 (2.26), 2.625 (1.64), 2.640 (1.10), 2.643 (1.07), 2.658 (0.54), 3.283 (0.78), 3.296 (1.23), 3.301 (2.56), 3.308 (1.30), 3.314 (3.26), 3.319 (2.64), 3.326 (2.96), 3.330 (3.19), 3.332 (3.27), 3.337 (1.16), 3.344 (2.51), 3.349 (1.11), 3.362 (0.77), 3.482 (1.07), 3.490 (1.47), 3.507 (2.95), 3.514 (3.62), 3.532 (1.94), 3.540 (3.75), 3.551 (3.41), 3.565 (1.46), 3.575 (1.32), 3.603 (0.58), 3.609 (0.67), 3.616 (0.78), 3.622 (1.30), 3.628 (1.13), 3.635 (0.99), 3.641 (1.04), 3.646 (0.96), 3.660 (0.44), 4.179 (1.52), 4.191 (1.49), 4.205 (0.72), 4.218 (4.80), 4.236 (8.51), 4.253 (4.42), 4.882 (6.11), 5.751 (0.76), 5.767 (2.32), 5.782 (2.29), 5.799 (0.72), 6.098 (9.47), 6.104 (9.43), 7.233 (2.83), 7.238 (2.81), 7.254 (2.84), 7.259 (2.99), 7.377 (1.53), 7.383 (1.62), 7.394 (1.87), 7.397 (2.14), 7.399 (2.27), 7.402 (2.03), 7.413 (1.94), 7.418 (2.14), 7.529 (0.42), 7.560 (0.52), 7.565 (1.32), 7.580 (5.39), 7.582 (6.16), 7.585 (8.30), 7.598 (3.10), 7.601 (3.20), 7.604 (0.89), 7.618 (0.83), 7.622 (0.88), 7.691 (3.28), 7.693 (3.41), 7.695 (1.65), 7.712 (2.89), 8.038 (3.83), 8.042 (3.87), 8.053 (3.92), 8.058 (3.84).

### Example 632

### (3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1)

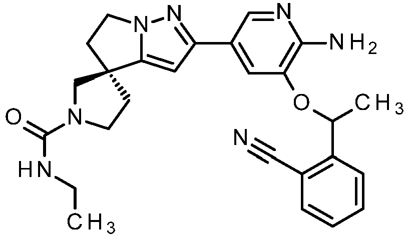

The title compound from **Example 631** was separated into diastereomers by preparative chiral HPLC to give 6.43 mg (93 % purity, 30 % yield) of the title compound (diastereomer 1, Rₜ = 14.5 - 17.2 min)

### Preparative chiral HPLC method:

Instrument: PrepCon Labomatic HPLC-1; Column: YMC Cellulose SC 5µ, 250x30; eluent A: hexane + 0.1 vol % diethylamine; eluent B: ethanol; isocratic: 40%A+60%B; flow: 40 mL/min; temperature: 25 °C; UV: 280 nm

### Analytical chiral HPLC method:

Instrument: Thermo Fisher UltiMate 3000; Column: YMC Cellulose SC 3µ, 100x4.6; eluent A: hexane + 0.1 vol % diethylamine; eluent B: ethanol; isocratic: 40%A+60%B; flow: 1.4 mL/min; temperature: 25 °C; UV: 280 nm
Analytical chiral HPLC: Rt = 3.90 min.
[α]²⁰_{D} +40.9° (c = 1.00, MeOH)
LC-MS (Method 1): Rₜ = 0.94 min; MS (ESlpos): m/z = 472 [M+H]⁺
¹H-NMR (400 MHz, DMSO-d6) δ [ppm]: 1.002 (4.30), 1.019 (10.04), 1.037 (4.62), 1.084 (0.51), 1.232 (0.91), 1.259 (0.86), 1.353 (3.52), 1.656 (5.92), 1.672 (5.99), 2.005 (0.84), 2.019 (1.13), 2.029 (1.58), 2.048 (0.68), 2.181 (0.47), 2.518 (16.00), 2.523 (10.97), 3.018 (0.55), 3.037 (1.75), 3.051 (1.94), 3.054 (1.89), 3.069 (1.71), 3.086 (0.52), 3.355 (1.21), 3.363 (0.92), 3.379 (5.31), 3.400 (0.56), 3.449 (0.43), 3.467 (0.75), 3.481 (0.58), 3.492 (0.47), 4.119 (1.56), 4.137 (2.54), 4.154 (1.49), 5.770 (1.40), 5.786 (1.39), 5.921 (3.76), 6.135 (0.75), 6.149 (1.44), 6.163 (0.69), 6.235 (6.83), 7.247 (2.61), 7.251 (2.55), 7.478 (0.90), 7.481 (0.89), 7.497 (1.83), 7.500 (1.77), 7.516 (1.16), 7.519 (1.10), 7.709 (0.76), 7.712 (0.78), 7.728 (1.55), 7.731 (1.60), 7.747 (1.15), 7.750 (1.12), 7.784 (1.98), 7.801 (1.11), 7.865 (1.74), 7.868 (1.77), 7.885 (1.65), 7.887 (1.64), 7.896 (3.58), 7.901 (3.42).

### Example 633

### (3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 2)

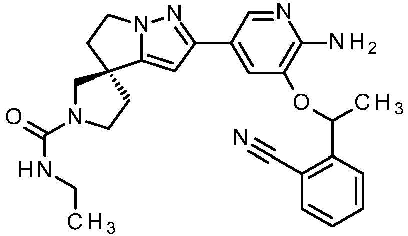

For the preparation of the diastereomeric mixture of the title compound see **Example 631.** The diastereomers were separated by preparative chiral HPLC (method see **Example 632**) to give 5.00 mg (94 % purity) of the title compound (diastereomer 2, Rₜ = 22.74 - 26.16 min).
Analytical chiral HPLC (method see **Example 632):** Rₜ = 6.11 min.
[α]²⁰_{D} : +45.3° (c = 1.00, MeOH)
LC-MS (Method 1): Rₜ = 0.94 min; MS (ESlpos): m/z = 472 [M+H]⁺
¹H-NMR (400 MHz, DMSO-d6) δ [ppm]: 0.795 (0.62), 0.814 (0.44), 0.862 (0.41), 0.997 (5.14), 1.006 (1.02), 1.015 (12.06), 1.033 (5.58), 1.084 (1.14), 1.233 (0.69), 1.259 (2.00), 1.353 (2.36), 1.655 (7.19), 1.671 (7.19), 2.009 (1.02), 2.016 (1.02), 2.023 (1.39), 2.034 (1.87), 2.051 (0.82), 2.331 (2.54), 2.336 (1.14), 2.518 (16.00), 2.523 (10.86), 2.673 (2.53), 2.678 (1.16), 3.013 (0.63), 3.031 (2.08), 3.046 (2.28), 3.049 (2.25), 3.063 (2.03), 3.081 (0.61), 3.362 (1.58), 3.372 (5.38), 3.387 (1.67), 3.402 (0.74), 3.447 (0.57), 3.465 (0.97), 3.479 (0.71), 3.490 (0.60), 4.118 (1.89), 4.136 (3.36), 4.153 (1.82), 5.756 (0.46), 5.772 (1.68), 5.788 (1.70), 5.804 (0.48), 5.920 (4.60), 6.129 (0.84), 6.143 (1.71), 6.157 (0.84), 6.241 (8.40), 7.253 (3.13), 7.256 (3.11), 7.481 (1.09), 7.484 (1.10), 7.500 (2.17), 7.503 (2.16), 7.519 (1.33), 7.522 (1.32), 7.710 (0.84), 7.714 (0.93), 7.730 (1.83), 7.733 (1.93), 7.749 (1.38), 7.752 (1.39), 7.787 (2.43), 7.804 (1.32), 7.866 (2.05), 7.868 (2.14), 7.885 (1.99), 7.888 (1.98), 7.898 (4.53), 7.902 (4.43), 10.851 (0.77).

### Example 634

### (3'R)-2-[6-amino-5-(trifluoromethyl)pyridin-3-yl]-N-[1-(1-ethyl-1H-pyrazol-3-yl)ethyl]-6,7-dihydro-1'H-spiro[pyrazolo[5,1-c][1,4]oxazine-4,3'-pyrrolidine]-1'-carboxamide (diastereomer 1 and diastereomer 2)

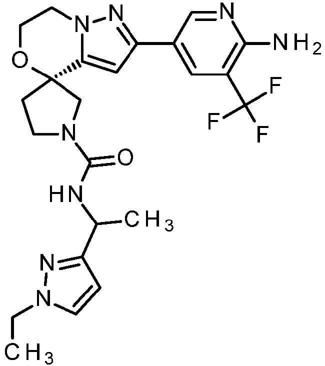

1-(1-Ethyl-1H-pyrazol-3-yl)ethan-1-amine (20.9 mg, 150 µmol) was dissolved in DMF (1.5 mL). CDI (22.4 mg, 138 µmol) and N,N-diisopropylethylamine (87 µL, 500 µmol) were added and the mixture was stirred for 1 h at rt. A solution of 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (47.1 mg, 125 µmol) in DMF was added dropwise. The reaction mixture was stirred for 1 h at 60 °C. Molsieve 4Å was added and the mixture was stirred for 1 h at rt. CDI (8.1 mg, 50 µmol) was added to the mixture and stirred for 1 h at 60 °C.The reaction mixture was allowed to cool down, diluted with water and extracted 3 times with ethyl acetate. The combined organic layers were dried over a hydrophobic filter and concentrated to give a crude product. The residue was purified by preparative HPLC to give 49.5 mg (90 % purity, 70 % yield) of the title compound.
LC-MS (Method 1): Rt = 0.97 min; MS (ESlpos): m/z = 505 [M+H]⁺
¹H-NMR (400 MHz, CHLOROFORM-d) δ [ppm]: 0.006 (0.96), 0.960 (0.49), 1.122 (1.17), 1.132 (1.22), 1.139 (1.20), 1.150 (1.13), 1.183 (0.66), 1.201 (1.35), 1.219 (0.69), 1.260 (0.67), 1.324 (0.44), 1.341 (0.42), 1.440 (0.66), 1.450 (7.77), 1.455 (7.58), 1.468 (15.99), 1.473 (16.00), 1.486 (7.63), 1.492 (7.66), 1.499 (1.43), 1.516 (1.88), 1.524 (11.00), 1.540 (12.63), 2.269 (0.46), 2.294 (1.02), 2.301 (0.93), 2.327 (1.62), 2.350 (0.84), 2.400 (1.31), 2.416 (1.43), 2.433 (0.88), 2.450 (0.74), 3.149 (0.50), 3.168 (0.47), 3.591 (2.82), 3.594 (2.96), 3.603 (0.80), 3.610 (1.55), 3.619 (3.73), 3.623 (3.52), 3.634 (1.58), 3.642 (0.93), 3.652 (0.75), 3.658 (0.77), 3.677 (1.12), 3.696 (1.67), 3.717 (0.61), 3.953 (1.72), 3.982 (1.46), 4.101 (2.26), 4.107 (1.99), 4.119 (6.39), 4.124 (5.89), 4.138 (7.40), 4.143 (7.51), 4.148 (3.99), 4.156 (5.09), 4.161 (5.96), 4.169 (3.07), 4.226 (3.58), 4.237 (4.18), 5.009 (1.25), 5.030 (8.46), 5.044 (2.43), 5.054 (2.74), 5.070 (1.83), 5.075 (1.88), 5.087 (1.03), 5.093 (1.09), 6.114 (0.44), 6.120 (0.45), 6.140 (5.79), 6.145 (5.84), 6.283 (9.23), 6.288 (10.12), 7.305 (0.56), 7.310 (0.72), 7.316 (4.49), 7.319 (4.68), 7.321 (4.80), 7.324 (4.27), 8.105 (5.29), 8.110 (4.62), 8.588 (4.31), 8.593 (4.27).

### Example 635

### (3'R)-2-[6-amino-5-(trifluoromethyl)pyridin-3-yl]-N-[(1-methyl-1H-pyrazol-5-yl)methyl]-6,7-dihydrospiro[pyrazolo[5,1-c][1,4]oxazine-4,3'-pyrrolidine]-1'-carboxamide

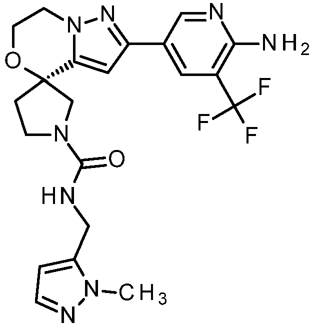

1-(1-Methyl-1H-pyrazol-5-yl)methanamine (16.7 mg, 150 µmol) was dissolved in DMF (1.5 mL). CDI (22.4 mg, 138 µmol) and N,N-diisopropylethylamine (87 µL, 500 µmol) were added and the mixture was stirred for 1 h at rt. A solution of 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (1/1) (47.1 mg, 125 µmol) in DMF was added dropwise. The reaction mixture was stirred over night at 60 °C. Molsieve 4Å was added and the mixture was stirred for 1 h at rt. CDI (8.1 mg, 50 µmol) was added to the mixture and stirred for 1 h at 60 °C. The reaction mixture was allowed to cool down, diluted with water and extracted 3 times with ethyl acetate. The combined organic layers were dried over a hydrophobic filter and concentrated to give a crude product. The residue was purified by preparative HPLC and preparative TLC to give 16.8 mg (100 % purity, 28 % yield) of the title compound.
LC-MS (Method 1): Rt = 0.81 min; MS (ESlpos): m/z = 477 [M+H]⁺
¹H-NMR (400 MHz, CHLOROFORM-d) δ [ppm]: 1.260 (0.48), 1.615 (0.56), 2.339 (0.50), 2.429 (0.44), 2.445 (0.47), 3.569 (1.44), 3.585 (0.52), 3.598 (1.87), 3.611 (0.57), 3.627 (0.61), 3.641 (0.50), 3.902 (16.00), 3.955 (0.48), 3.984 (0.41), 4.133 (0.56), 4.141 (0.62), 4.146 (1.09), 4.152 (1.36), 4.162 (1.32), 4.230 (1.70), 4.242 (1.56), 4.252 (0.63), 4.257 (0.63), 4.400 (0.55), 4.502 (1.32), 4.514 (1.13), 4.521 (1.35), 4.535 (1.06), 5.046 (1.83), 5.308 (6.01), 6.185 (1.89), 6.190 (1.97), 6.285 (5.87), 7.414 (1.90), 7.419 (1.89), 8.101 (1.40), 8.106 (1.42), 8.584 (1.36), 8.588 (1.32).

### Example 636

### (3'R)-2-[6-amino-5-(trifluoromethyl)pyridin-3-yl]-N-[(1-methyl-1H-pyrazol-4-yl)methyl]-6,7-dihydrospiro[pyrazolo[5,1-c][1,4]oxazine-4,3'-pyrrolidine]-1'-carboxamide

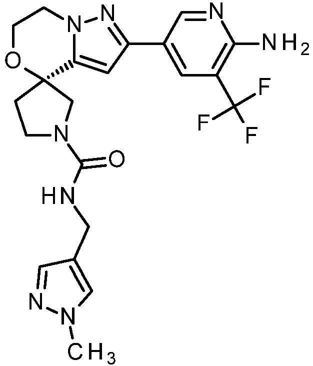

1-(1-Methyl-1H-pyrazol-4-yl)methanamine (16.7 mg, 150 µmol) was dissolved in DMF (1.5 mL). CDI (22.4 mg, 138 µmol) and N,N-diisopropylethylamine (87 µL, 500 µmol) were added and the mixture was stirred for 1 h at rt. A solution of 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (47.1 mg, 125 µmol) in DMF was added dropwise. The reaction mixture was stirred for 1 h at 60 °C. Molsieve 4Å was added and the mixture was stirred for 1 h at rt. CDI (8.1 mg, 50 µmol) was added to the mixture and stirred for 1 h at 60 °C.The reaction mixture was allowed to cool down, diluted with water and extracted 3 times with ethyl acetate. The combined organic layers were dried over a hydrophobic filter and concentrated to give a crude product. The residue was purified by preparative HPLC and preparative TLC to give 16.0 mg (100 % purity, 27 % yield) of the title compound.
LC-MS (Method 1): Rt = 0.80 min; MS (ESlpos): m/z = 477 [M+H]⁺
¹H-NMR (400 MHz, CHLOROFORM-d) δ [ppm]: 1.260 (0.56), 2.296 (0.42), 2.319 (0.58), 2.403 (0.48), 2.418 (0.52), 3.554 (1.77), 3.561 (0.63), 3.582 (2.05), 3.603 (0.84), 3.621 (0.57), 3.880 (16.00), 3.935 (0.61), 3.963 (0.53), 4.127 (0.63), 4.133 (0.70), 4.140 (1.20), 4.145 (1.59), 4.155 (1.63), 4.223 (1.89), 4.235 (1.54), 4.245 (0.70), 4.250 (0.71), 4.288 (1.38), 4.293 (1.44), 4.305 (2.11), 4.371 (0.51), 4.384 (0.70), 5.038 (2.26), 5.308 (5.12), 6.274 (5.86), 7.394 (2.95), 7.447 (2.94), 8.101 (1.55), 8.106 (1.58), 8.583 (1.49), 8.588 (1.45).

### Example 637

### (3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1 and diastereomer 2)

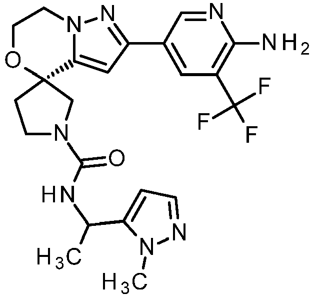

1-(1-Methyl-1H-pyrazol-5-yl)ethan-1-amine (18.8 mg, 150 µmol) was dissolved in DMF (1.5 mL). CDI (22.4 mg, 138 µmol) and N,N-diisopropylethylamine (87 µL, 500 µmol) were added and the mixture was stirred for 1 h at rt. A solution of 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (47.1 mg, 125 µmol) in DMF was added dropwise. The reaction mixture was stirred for 1 h at 60 °C. Molsieve 4Å was added and the mixture was stirred for 1 h at rt. CDI (8.1 mg, 50 µmol) was added to the mixture and stirred for 1 h at 60 °C. The reaction mixture was allowed to cool down, diluted with water and extracted 3 times with ethyl acetate. The combined organic layers were dried over a hydrophobic filter and concentrated to give a crude product. The residue was purified by preparative HPLC and preparative TLC again to give 16.3 mg (90 % purity, 24 % yield) of the title compound.
LC-MS (Method 1): Rt = 0.88 min; MS (ESlpos): m/z = 491 [M+H]⁺
¹H-NMR (400 MHz, CHLOROFORM-d) δ [ppm]: 1.261 (1.29), 1.558 (7.55), 1.575 (7.65), 2.296 (0.49), 2.328 (0.73), 2.416 (0.55), 2.421 (0.54), 2.432 (0.63), 3.539 (0.46), 3.552 (1.33), 3.558 (1.66), 3.580 (1.98), 3.587 (2.04), 3.603 (0.88), 3.622 (0.46), 3.903 (16.00), 3.906 (15.73), 3.920 (0.74), 3.951 (0.57), 4.135 (0.82), 4.147 (1.89), 4.160 (1.98), 4.228 (2.53), 4.240 (3.10), 4.250 (1.65), 4.263 (0.85), 5.038 (3.41), 5.228 (0.78), 5.245 (0.85), 5.248 (0.86), 5.266 (0.74), 5.309 (15.30), 6.190 (3.01), 6.195 (2.95), 6.278 (4.70), 6.289 (5.03), 6.345 (1.19), 7.412 (2.59), 7.416 (2.63), 8.098 (2.18), 8.104 (2.38), 8.581 (2.15), 8.586 (2.11).

### Example 638

### (3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1)

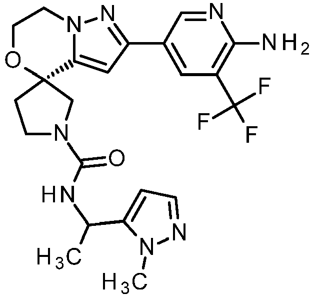

The title compound from **Example 637** was separated into diastereomers by preparative chiral HPLC to give 1.43 mg (97 % purity) of the title compound (diastereomer 1, Rt = 7.62 - 9.57 min)

### Preparative chiral HPLC method: NPB

Instrument: PrepCon Labomatic HPLC-1; Column: YMC Cellulose SB 5µ, 250x30; eluent A: hexane + 0.1 vol % diethylamine; eluent B: ethanol; isocratic: 80%A+20%B; flow: 90 mL/min; temperature: 25 °C; UV: 254 nm

### Analytical chiral HPLC method: NPB

Instrument: Thermo Fisher UltiMate 3000; Column: YMC Cellulose SB 3µ, 100x4.6; eluent A: hexane + 0.1 vol % diethylamine; eluent B: ethanol; isocratic: 80%A+20%B; flow: 1.4 mL/min; temperature: 25 °C; UV: 254 nm
Analytical chiral HPLC: Rₜ = 3.78 min.
[α]²⁰_{D}: +94.5° (c = 1.00, MeOH)
LC-MS (Method 1): Rₜ = 0.89 min; MS (ESlpos): m/z = 491 [M+H]⁺
¹H-NMR (400 MHz, CHLOROFORM-d) δ [ppm]: 1.225 (0.52), 1.264 (1.32), 1.277 (0.45), 1.406 (1.02), 1.440 (6.10), 1.558 (4.58), 1.575 (4.81), 2.280 (0.68), 2.325 (0.47), 2.328 (0.46), 2.416 (0.44), 2.433 (0.47), 3.539 (0.45), 3.552 (1.48), 3.565 (0.42), 3.580 (1.75), 3.622 (0.46), 3.906 (16.00), 3.924 (0.48), 4.133 (0.60), 4.137 (0.61), 4.147 (1.36), 4.160 (1.63), 4.226 (1.73), 4.236 (1.46), 4.241 (2.16), 4.249 (0.78), 4.254 (0.77), 4.263 (0.71), 5.043 (2.02), 5.228 (0.50), 5.245 (0.55), 5.249 (0.53), 5.266 (0.47), 6.191 (1.79), 6.195 (1.81), 6.278 (5.19), 7.411 (1.94), 7.416 (1.92), 8.099 (1.33), 8.104 (1.35), 8.581 (1.28), 8.585 (1.24).

### Example 639

### (3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 2)

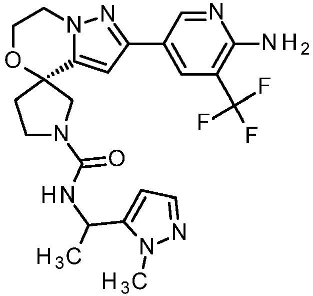

For the preparation of the diastereomeric title compound see **Example 637.** The diastereomers were separated by preparative chiral HPLC (method see **Example 638)** to give 2.13 mg (98 % purity) of the title compound (diastereomer 2, Rₜ = 11.01 - 13.52 min).

Analytical chiral HPLC (method see **Example 638):** Rₜ = 5.75 min.
[α]²⁰_{D} : +73.4° (c = 1.00, MeOH)
LC-MS (Method 1): Rₜ = 0.88 min; MS (ESlpos): m/z = 491 [M+H]⁺
¹H-NMR (400 MHz, CHLOROFORM-d) δ [ppm]: 1.224 (0.45), 1.261 (1.02), 1.406 (1.01), 1.439 (2.20), 1.558 (4.67), 1.575 (4.89), 2.297 (0.42), 2.330 (0.57), 2.435 (0.43), 3.558 (1.61), 3.578 (0.82), 3.587 (2.29), 3.603 (0.81), 3.903 (16.00), 3.919 (0.49), 4.135 (0.56), 4.149 (1.08), 4.153 (1.40), 4.164 (1.27), 4.229 (2.38), 4.241 (1.74), 4.244 (1.46), 4.251 (1.37), 5.048 (2.19), 5.226 (0.52), 5.243 (0.57), 5.248 (0.56), 5.265 (0.49), 6.190 (1.89), 6.194 (1.90), 6.289 (5.32), 7.413 (2.00), 7.418 (1.98), 8.100 (1.43), 8.105 (1.44), 8.580 (1.38), 8.584 (1.34).

### Example 640

### (3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)

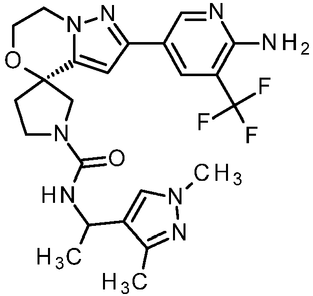

1-(1,3-Dimethyl-1H-pyrazol-4-yl)ethan-1-amine (20.9 mg, 150 µmol) was dissolved in DMF (1.5 mL). CDI (22.4 mg, 138 µmol) and N,N-diisopropylethylamine (87 µL, 500 µmol) were added and the mixture was stirred for 1 h at rt. A solution of 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (47.1 mg, 125 µmol) in DMF was added dropwise. The reaction mixture was stirred for 1 h at 60 °C. Molsieve 4Å was added and the mixture was stirred for 1 h at rt. CDI (8.1 mg, 50 µmol) was added to the mixture and stirred for 1 h at 60 °C. The reaction mixture was allowed to cool down, diluted with water and extracted 3 times with ethyl acetate. The combined organic layers were dried over a hydrophobic filter and concentrated to give a crude product. The residue was purified by preparative HPLC and preparative TLC to give 21.7 mg (90 % purity, 31 % yield) of the title compound.
LC-MS (Method 1): Rt = 0.88 min; MS (ESlpos): m/z = 505 [M+H]⁺
¹H-NMR (400 MHz, CHLOROFORM-d) δ [ppm]: 0.052 (0.46), 1.306 (1.22), 1.537 (7.05), 1.554 (7.05), 2.313 (12.88), 2.316 (12.17), 2.338 (0.59), 2.348 (0.47), 2.370 (0.82), 2.391 (0.49), 2.440 (0.69), 2.455 (0.76), 2.473 (0.50), 2.489 (0.41), 3.573 (0.60), 3.581 (1.45), 3.602 (1.82), 3.609 (2.40), 3.631 (2.21), 3.658 (0.54), 3.856 (14.40), 3.955 (0.81), 3.968 (0.67), 3.983 (0.58), 3.995 (0.56), 4.178 (1.13), 4.183 (1.07), 4.194 (2.82), 4.205 (3.22), 4.216 (1.21), 4.229 (1.10), 4.235 (1.13), 4.268 (2.67), 4.281 (2.51), 4.289 (1.13), 4.295 (0.94), 5.001 (1.10), 5.018 (1.54), 5.036 (1.05), 5.078 (3.97), 5.354 (16.00), 6.317 (5.33), 6.332 (5.66), 6.390 (1.01), 7.270 (3.87), 8.144 (2.53), 8.150 (2.72), 8.628 (2.48), 8.633 (2.42).

### Example 641

### (3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1)

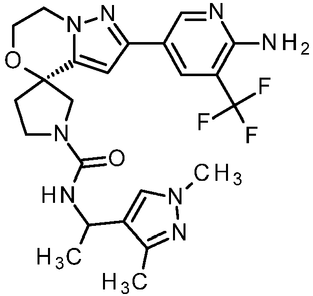

The title compound from **Example 640** was separated into diastereomers by preparative chiral HPLC to give 3.29 mg (99 % purity) of the title compound (diastereomer 1, Rt = 8.48 - 10.61 min)

### Preparative chiral HPLC method: NPB

Instrument: PrepCon Labomatic HPLC-1; Column: YMC Cellulose SB 5µ, 250x30; eluent A: hexane + 0.1 vol % diethylamine; eluent B: ethanol; isocratic: 80%A+20%B; flow: 90 mL/min; temperature: 25 °C; UV: 280 nm

### Analytical chiral HPLC method: NPB

Instrument: Thermo Fisher UltiMate 3000; Column: YMC Cellulose SB 3µ, 100x4.6; eluent A: hexane + 0.1 vol % diethylamine; eluent B: ethanol; isocratic: 80%A+20%B; flow: 1.4 mL/min; temperature: 25 °C; UV: 280 nm
Analytical chiral HPLC: Rₜ = 4.11 min.
[α]²⁰_{D} : +55.4° (c = 1.00, MeOH)
LC-MS (Method 1): Rₜ = 0.89 min; MS (ESlpos): m/z = 505 [M+H]⁺
¹H-NMR (400 MHz, CHLOROFORM-d) δ [ppm]: 1.268 (0.55), 1.305 (1.20), 1.449 (1.20), 1.483 (4.65), 1.536 (6.54), 1.553 (6.51), 2.311 (16.00), 2.323 (0.74), 2.330 (0.55), 2.341 (0.57), 2.356 (0.41), 2.363 (0.77), 2.389 (0.50), 2.437 (0.64), 2.454 (0.70), 2.470 (0.44), 2.669 (0.41), 3.579 (2.01), 3.588 (0.79), 3.597 (0.69), 3.608 (2.25), 3.638 (0.53), 3.656 (0.76), 3.853 (15.59), 3.965 (0.83), 3.994 (0.73), 4.176 (0.92), 4.180 (0.95), 4.190 (2.38), 4.202 (2.47), 4.216 (1.20), 4.234 (1.20), 4.265 (2.33), 4.274 (1.88), 4.279 (2.09), 4.287 (0.87), 4.292 (0.82), 4.998 (0.89), 5.015 (1.29), 5.033 (0.86), 5.067 (0.53), 5.082 (3.18), 6.316 (6.88), 7.270 (4.18), 8.143 (2.02), 8.147 (2.05), 8.625 (1.95), 8.629 (1.90).

### Example 642

### (3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 2)

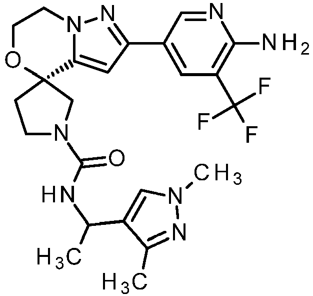

For the preparation of the diastereomeric mixture of the title compound see **Example 640.** The diastereomers were separated by preparative chiral HPLC (method see **Example 641)** to give 2.62 mg (100 % purity) of the title compound (enantiomer 2, Rₜ = 10.61 - 13.77 min). Analytical chiral HPLC (method see **Example 641):** Rₜ = 5.05 min.
[α]²⁰_{D} : +58.7° (c = 1.00, MeOH)
LC-MS (Method 1): Rₜ = 0.88 min; MS (ESlpos): m/z = 505 [M+H]⁺
¹H-NMR (400 MHz, CHLOROFORM-d) δ [ppm]: 0.936 (0.71), 1.197 (0.52), 1.221 (0.47), 1.271 (1.18), 1.309 (1.05), 1.452 (2.14), 1.487 (2.19), 1.538 (6.50), 1.555 (6.35), 1.613 (0.45), 1.632 (0.62), 2.318 (15.92), 2.340 (0.67), 2.349 (0.56), 2.364 (0.40), 2.373 (0.91), 2.397 (0.51), 2.454 (0.64), 3.603 (2.42), 3.611 (1.40), 3.622 (1.07), 3.632 (3.18), 3.860 (16.00), 3.956 (0.78), 3.985 (0.67), 4.179 (0.83), 4.185 (0.95), 4.192 (1.59), 4.196 (2.37), 4.207 (2.46), 4.232 (1.21), 4.270 (2.18), 4.280 (1.96), 4.283 (1.84), 4.291 (0.90), 4.298 (0.79), 5.003 (0.86), 5.021 (1.24), 5.038 (0.83), 5.088 (3.14), 6.333 (6.92), 7.270 (4.16), 8.148 (2.05), 8.152 (2.05), 8.630 (1.99), 8.633 (1.93).

### Example 643

### (3R)-2'-{6-amino-5-[(1R)-1-(3-cyanophenyl)ethoxy]pyridin-3-yl}-N-ethyl-5',6'-dihydrospiro[pyrrolidine-3,4'-pyrrolo[1,2-b]pyrazole]-1-carboxamide

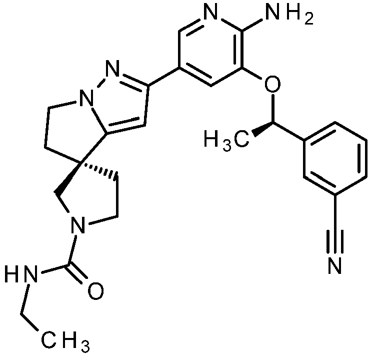

(3R)-1-(Ethylcarbamoyl)-5',6'-dihydrospiro[pyrrolidine-3,4'-pyrrolo[1,2-b]pyrazol]-2'-yl trifluoromethanesulfonate (40.0 mg, 105 µmol), {6-amino-5-[(1R)-1-(3-cyanophenyl)ethoxy]pyridin-3-yl}boronic acid **(Intermediate 449,** 109 mg, 38 % purity, 146 µmol) and potassium phosphate (630 µL, 0.50 M, 310 µmol) were combined in degassed 1,4-dioxane (1.7 mL) under argon. XPhos Pd G2 (4.12 mg, 5.23 µmol) was added and the mixture was stirred for 2 h at 100 °C. The reaction mixture was allowed to cool down, diluted with ethyl acetate and filtered. The filtrate was washed with water and brine, dried over sodium sulfate and concentrated under reduced pressure. The crude product was purified by preparative TLC (dichlormethan / methanol 9:1 gradient) to give 29.3 mg (98 % purity, 58 % yield) of the title compound.
LC-MS (Method 1): Rt = 0.92 min; MS (ESlpos): m/z = 473 [M+H]⁺
¹H-NMR (400 MHz, CHLOROFORM-d) δ [ppm]: 1.152 (7.47), 1.170 (16.00), 1.188 (7.65), 1.249 (1.06), 1.259 (0.41), 1.681 (12.44), 1.697 (13.15), 2.044 (0.54), 2.061 (0.79), 2.075 (1.27), 2.089 (1.14), 2.107 (0.78), 2.194 (0.80), 2.212 (1.44), 2.225 (0.82), 2.230 (1.02), 2.243 (0.96), 2.261 (0.57), 2.508 (0.54), 2.526 (1.18), 2.541 (1.47), 2.559 (1.87), 2.576 (1.11), 2.600 (1.11), 2.635 (1.40), 2.649 (1.30), 2.667 (0.60), 3.280 (1.08), 3.294 (1.46), 3.298 (3.38), 3.312 (3.67), 3.316 (3.48), 3.330 (3.30), 3.333 (1.42), 3.348 (1.02), 3.479 (1.47), 3.504 (3.74), 3.522 (1.23), 3.529 (1.27), 3.538 (4.45), 3.546 (2.03), 3.562 (2.33), 3.599 (0.77), 3.613 (1.02), 3.618 (1.17), 3.632 (0.96), 3.637 (0.82), 3.642 (0.65), 3.656 (0.44), 4.190 (0.97), 4.204 (1.80), 4.216 (4.31), 4.234 (6.25), 4.251 (3.22), 4.796 (5.90), 5.308 (10.54), 5.467 (0.68), 5.483 (2.20), 5.499 (2.18), 5.515 (0.65), 6.073 (11.41), 6.085 (0.42), 7.210 (4.26), 7.214 (4.29), 7.459 (1.58), 7.478 (4.07), 7.498 (2.82), 7.573 (1.82), 7.576 (3.20), 7.580 (2.16), 7.592 (1.42), 7.595 (2.23), 7.599 (1.43), 7.623 (1.63), 7.626 (2.55), 7.646 (2.01), 7.701 (4.27), 8.006 (5.31), 8.011 (5.44).

### Example 644

### (3R)-2'-{6-amino-5-[(1R)-1-(4-cyanophenyl)ethoxy]pyridin-3-yl}-N-ethyl-5',6'-dihydrospiro[pyrrolidine-3,4'-pyrrolo[1,2-b]pyrazole]-1-carboxamide

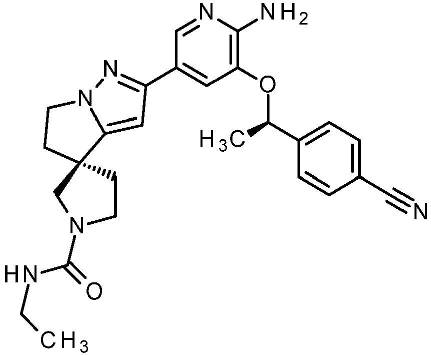

The compound was prepared similarly to **Example 643** using (3R)-1-(ethylcarbamoyl)-5',6'-dihydrospiro[pyrrolidine-3,4'-pyrrolo[1,2-b]pyrazol]-2'-yl trifluoromethanesulfonate (40.0 mg, 105 µmol) and {6-amino-5-[(1R)-1-(4-cyanophenyl)ethoxy]pyridin-3-yl}boronic acid **(Intermediate 450,** 92.1 mg, 45 % purity, 146 µmol) to give 30.7 mg (98 % purity, 61 % yield) of the title compound.
LC-MS (Method 1): Rₜ = 0.91 min; MS (ESlpos): m/z = 473 [M+H]⁺
¹H-NMR (400 MHz, CHLOROFORM-d) δ [ppm]: 0.006 (0.47), 1.151 (6.99), 1.169 (16.00), 1.188 (7.05), 1.250 (0.91), 1.656 (2.29), 1.679 (11.12), 1.695 (10.34), 2.039 (0.48), 2.054 (0.67), 2.057 (0.66), 2.071 (1.15), 2.084 (0.99), 2.088 (0.89), 2.102 (0.70), 2.189 (0.72), 2.208 (1.31), 2.220 (0.68), 2.226 (0.88), 2.239 (0.87), 2.257 (0.50), 2.505 (0.48), 2.523 (0.92), 2.538 (1.30), 2.554 (1.32), 2.557 (1.50), 2.572 (0.99), 2.594 (0.98), 2.630 (1.21), 2.644 (1.02), 2.662 (0.51), 3.280 (1.03), 3.294 (1.34), 3.297 (3.08), 3.311 (3.44), 3.316 (3.28), 3.330 (3.11), 3.333 (1.19), 3.347 (0.97), 3.474 (1.21), 3.499 (3.04), 3.527 (4.33), 3.546 (1.72), 3.551 (1.75), 3.564 (0.70), 3.592 (0.68), 3.606 (0.87), 3.611 (0.99), 3.625 (0.82), 3.635 (0.51), 4.155 (0.82), 4.169 (1.44), 4.183 (0.78), 4.205 (2.65), 4.224 (4.73), 4.242 (2.23), 4.791 (5.14), 5.308 (13.37), 5.477 (0.59), 5.493 (1.94), 5.509 (1.91), 5.526 (0.57), 6.054 (10.60), 7.170 (3.91), 7.174 (3.90), 7.493 (4.89), 7.514 (6.33), 7.646 (1.38), 7.650 (7.20), 7.654 (2.26), 7.666 (2.04), 7.671 (5.50), 7.675 (0.92), 8.003 (5.46), 8.007 (5.34).

### Example 645

### (3R)-2'-{6-amino-5-[(1R)-1-(3,5-difluoropyridin-4-yl)ethoxy]pyridin-3-yl}-N-ethyl-5',6'-dihydrospiro[pyrrolidine-3,4'-pyrrolo[1,2-b]pyrazole]-1-carboxamide

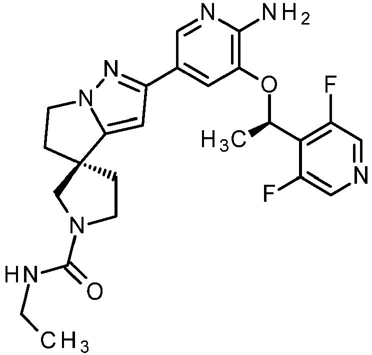

The compound was prepared similarly to **Example 643** using (3R)-1-(ethylcarbamoyl)-5',6'-dihydrospiro[pyrrolidine-3,4'-pyrrolo[1,2-b]pyrazol]-2'-yl trifluoromethanesulfonate (40.0 mg, 105 µmol) and {6-amino-5-[(1R)-1-(3,5-difluoropyridin-4-yl)ethoxy]pyridin-3-yl}boronic acid **(Intermediate 451,** 100 mg, 43 % purity, 146 µmol) to give 31.7 mg (98 % purity, 61 % yield) of the title compound.
[α]²⁰_{D}: +198.9° (c = 1.00, MeOH)
LC-MS (Method 1): Rt = 0.86 min; MS (ESlpos): m/z = 485 [M+H]⁺
¹H-NMR (400 MHz, CHLOROFORM-d) δ [ppm]: 0.007 (0.52), 1.158 (6.50), 1.176 (14.25), 1.194 (6.57), 1.250 (0.69), 1.814 (9.06), 1.830 (8.92), 2.060 (0.50), 2.074 (0.67), 2.078 (0.68), 2.092 (1.30), 2.104 (0.98), 2.123 (0.67), 2.220 (0.72), 2.239 (1.39), 2.251 (0.69), 2.256 (0.89), 2.269 (0.96), 2.288 (0.50), 2.526 (0.48), 2.543 (1.12), 2.558 (1.30), 2.576 (1.82), 2.593 (0.98), 2.634 (2.04), 2.651 (1.22), 2.667 (1.14), 2.684 (0.51), 3.288 (1.00), 3.302 (1.39), 3.305 (2.96), 3.319 (3.33), 3.324 (3.18), 3.338 (2.98), 3.342 (1.21), 3.356 (0.91), 3.504 (1.27), 3.516 (0.70), 3.529 (3.22), 3.539 (1.13), 3.558 (5.11), 3.576 (0.88), 3.582 (1.54), 3.616 (0.70), 3.629 (0.87), 3.635 (1.00), 3.649 (0.85), 3.659 (0.54), 4.160 (0.85), 4.173 (1.47), 4.186 (0.77), 4.245 (3.19), 4.262 (5.96), 4.280 (3.01), 4.785 (5.11), 5.309 (13.07), 5.874 (0.64), 5.890 (2.04), 5.906 (2.01), 5.923 (0.61), 6.122 (9.74), 7.401 (3.80), 7.405 (3.82), 8.040 (4.83), 8.044 (4.64), 8.346 (16.00).

### Example 646

### (3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 1 and diastereomer 2)

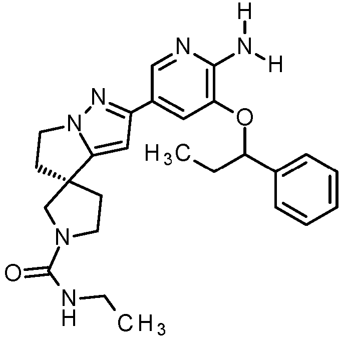

(3R)-1-(Ethylcarbamoyl)-5',6'-dihydrospiro[pyrrolidine-3,4'-pyrrolo[1,2-b]pyrazol]-2'-yl trifluoromethanesulfonate (235 mg, 615 µmol), [6-amino-5-(1-phenylpropoxy)pyridin-3-yl]boronic acid **(Intermediate 452,** 551 mg, 33 % purity, 677 µmol) and potassium phosphate (3.7 mL, 0.50 M, 1.8 mmol) were suspended in degassed 1,4-dioxane (6.9 mL) under nitrogen. XPhos Pd G2 (24.2 mg, 30.8 µmol) was added and the mixture was stirred over night at 100 °C. The reaction mixture was cooled to rt, diluted with ethyl acetate, filtered over celite and washed with water and brine. The organic layer was dried over a hydrophobic filter and concentrated to give a residue. The crude product was purified by flash chromatography (silica gel, dichloromethane / methanol 9:1 gradient) and preparative TLC to give 9.20 mg (90 % purity, 3 % yield) of the title compound.
LC-MS (Method 1): Rt = 1.05 min; MS (ESlpos): m/z = 462 [M+H]⁺
¹H-NMR (400 MHz, CHLOROFORM-d) δ [ppm]: -0.069 (0.59), 0.765 (0.94), 0.812 (1.30), 0.828 (0.64), 0.885 (0.55), 0.911 (6.80), 0.930 (16.00), 0.948 (7.37), 1.077 (5.09), 1.081 (5.46), 1.095 (10.33), 1.099 (10.96), 1.113 (5.35), 1.118 (5.53), 1.164 (0.77), 1.184 (6.45), 1.216 (1.85), 1.265 (1.36), 1.268 (1.88), 1.354 (1.01), 1.440 (0.52), 1.457 (0.58), 1.609 (0.91), 1.626 (1.85), 1.642 (1.50), 1.833 (0.75), 1.848 (0.98), 1.868 (1.63), 1.883 (1.34), 1.886 (1.37), 1.901 (1.16), 1.919 (0.40), 1.956 (1.86), 1.974 (2.99), 1.991 (2.49), 2.008 (1.60), 2.026 (1.06), 2.098 (0.54), 2.117 (1.10), 2.129 (1.39), 2.148 (1.30), 2.160 (0.78), 2.321 (3.45), 2.331 (0.76), 2.386 (0.62), 2.413 (0.42), 2.430 (0.77), 2.450 (1.08), 2.463 (1.79), 2.480 (0.83), 2.485 (0.77), 2.505 (0.76), 2.509 (0.82), 2.526 (1.78), 2.540 (1.17), 2.544 (1.01), 2.554 (0.79), 2.559 (0.72), 2.573 (0.40), 2.577 (0.44), 3.205 (0.71), 3.210 (0.80), 3.219 (1.00), 3.224 (2.69), 3.228 (2.39), 3.237 (2.53), 3.241 (4.04), 3.246 (2.48), 3.255 (2.25), 3.260 (2.78), 3.273 (0.76), 3.278 (0.70), 3.401 (0.96), 3.420 (2.74), 3.426 (2.53), 3.439 (3.58), 3.455 (3.47), 3.479 (1.75), 3.514 (0.81), 3.534 (1.13), 3.547 (1.01), 3.571 (0.46), 4.073 (1.42), 4.130 (3.78), 4.148 (6.01), 4.165 (3.45), 4.737 (4.61), 5.117 (1.21), 5.131 (2.35), 5.149 (1.13), 5.233 (0.67), 5.947 (7.19), 5.959 (7.77), 6.929 (1.05), 7.123 (2.73), 7.130 (3.48), 7.134 (2.85), 7.166 (0.93), 7.170 (1.07), 7.241 (1.77), 7.261 (6.41), 7.278 (14.00), 7.294 (1.59), 7.299 (0.83), 7.451 (1.03), 7.603 (0.44), 7.608 (0.47), 7.887 (3.86), 7.891 (5.00), 7.894 (3.69), 8.029 (0.57), 8.192 (0.76).

### Example 647

### (3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 1)

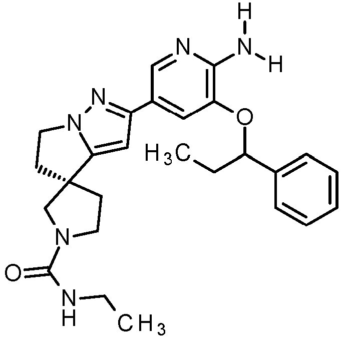

The title compound from **Example 646** was separated into diastereomers by preparative chiral HPLC to give 4.00 mg (90 % purity, 43 % yield) of the title compound (diastereomer 1, Rₜ = 11.9 - 13.4 min)

### Preparative chiral HPLC method: MTBE

Instrument: PrepCon Labomatic HPLC-2; Column: YMC Cellulose SB 5µ, 250x30; eluent A: methyl tert-butyl ether + 0.1 vol % diethylamine; eluent B: acetonitrile; isocratic: 50%A+50%B; flow: 50 mL/min; temperature: 25 °C; UV: 254 nm

### Analytical chiral HPLC method: MTBE

Instrument: Thermo Fisher UltiMate 3000; Column: YMC Cellulose SB 3µ, 100x4.6; eluent A: methyl tert-butyl ether + 0.1 vol % diethylamine; eluent B: acetonitrile; isocratic: 50%A+50%B; flow: 1.4 mL/min; temperature: 25 °C; UV: 254 nm
Analytical chiral HPLC: Rₜ = 4.81 min.
LC-MS (Method 1): Rt = 1.05 min; MS (ESlpos): m/z = 462 [M+H]⁺
¹H-NMR (400 MHz, CHLOROFORM-d) δ [ppm]: 0.759 (1.09), 0.793 (1.09), 0.810 (1.72), 0.826 (0.87), 0.912 (5.34), 0.930 (12.27), 0.949 (5.62), 1.076 (7.20), 1.093 (16.00), 1.112 (7.54), 1.185 (7.41), 1.206 (2.06), 1.220 (1.82), 1.224 (1.43), 1.231 (1.42), 1.236 (1.60), 1.261 (0.80), 1.347 (0.55), 1.360 (0.53), 1.833 (0.66), 1.848 (0.83), 1.868 (1.35), 1.883 (1.14), 1.886 (1.12), 1.901 (1.01), 1.942 (5.28), 1.955 (1.58), 1.974 (2.16), 1.984 (1.51), 1.991 (1.75), 1.997 (1.24), 2.008 (1.37), 2.015 (0.94), 2.027 (0.71), 2.107 (0.83), 2.127 (1.44), 2.139 (0.74), 2.145 (0.92), 2.157 (1.05), 2.176 (0.56), 2.416 (0.51), 2.434 (0.92), 2.449 (1.30), 2.468 (1.57), 2.483 (1.03), 2.503 (0.99), 2.520 (1.70), 2.539 (1.27), 2.553 (1.02), 2.571 (0.56), 3.201 (0.99), 3.215 (1.36), 3.219 (3.13), 3.233 (3.46), 3.237 (3.31), 3.250 (3.07), 3.269 (0.95), 3.391 (1.03), 3.415 (3.47), 3.436 (4.58), 3.458 (2.04), 3.476 (0.70), 3.510 (0.69), 3.529 (1.01), 3.543 (0.85), 3.638 (5.36), 4.081 (0.85), 4.094 (1.46), 4.107 (0.85), 4.127 (2.73), 4.144 (4.85), 4.162 (2.33), 4.809 (2.67), 5.110 (1.46), 5.128 (2.03), 5.142 (1.37), 5.947 (10.52), 6.931 (0.47), 7.123 (3.85), 7.127 (3.86), 7.172 (0.82), 7.203 (2.51), 7.209 (1.33), 7.243 (1.45), 7.263 (5.60), 7.274 (6.56), 7.280 (14.82), 7.292 (1.43), 7.454 (0.53), 7.867 (2.33).

### Example 648

### (3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 2)

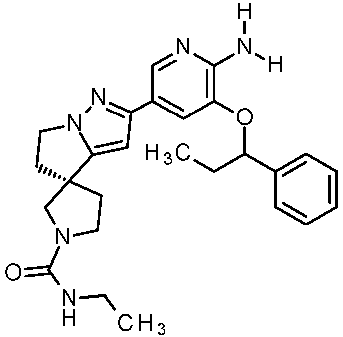

For the preparation of the diastereomeric mixture of the title compound see **Example 646.** The diastereomers were separated by preparative chiral HPLC (method see **Example 647)** to give 2.90 mg (90 % purity, 65 % yield) of the title compound (diastereomer 2, Rₜ = 22.8 - 25.3 min).
Analytical chiral HPLC (method see **Example 647):** Rₜ = 7.07 min
LC-MS (Method 1): Rt = 1.05 min; MS (ESlpos): m/z = 462 [M+H]⁺
¹H-NMR (400 MHz, CHLOROFORM-d) δ [ppm]: 0.018 (0.47), 0.740 (1.06), 0.759 (1.48), 0.765 (1.46), 0.772 (1.41), 0.783 (1.44), 0.788 (1.39), 0.811 (1.87), 0.826 (1.03), 0.852 (0.43), 0.869 (0.45), 0.910 (5.00), 0.928 (11.55), 0.947 (5.45), 1.081 (7.10), 1.099 (16.00), 1.117 (7.36), 1.185 (8.23), 1.220 (1.43), 1.225 (1.56), 1.231 (1.71), 1.349 (0.56), 1.361 (0.69), 1.387 (0.80), 1.398 (0.54), 1.403 (0.88), 1.416 (0.83), 1.435 (0.52), 1.532 (0.74), 1.834 (0.64), 1.848 (0.80), 1.869 (1.27), 1.883 (1.10), 1.887 (1.10), 1.902 (0.96), 1.940 (0.85), 1.957 (1.74), 1.975 (2.14), 1.984 (1.18), 1.993 (1.84), 2.002 (0.85), 2.010 (1.17), 2.028 (0.65), 2.093 (0.69), 2.112 (1.41), 2.124 (0.67), 2.131 (0.86), 2.143 (0.95), 2.162 (0.50), 2.411 (0.47), 2.427 (0.91), 2.443 (1.21), 2.460 (2.05), 2.477 (0.95), 2.506 (0.92), 2.524 (2.08), 2.541 (1.15), 2.557 (0.90), 2.574 (0.51), 3.186 (1.41), 3.208 (0.98), 3.222 (1.32), 3.226 (3.00), 3.240 (3.33), 3.245 (3.16), 3.258 (2.97), 3.262 (1.23), 3.276 (0.94), 3.400 (1.10), 3.412 (0.64), 3.425 (3.12), 3.449 (3.59), 3.473 (1.62), 3.507 (0.64), 3.521 (0.82), 3.527 (0.95), 3.540 (0.83), 3.638 (0.66), 4.073 (0.62), 4.092 (1.24), 4.126 (2.92), 4.143 (5.12), 4.161 (2.68), 5.119 (1.37), 5.136 (1.96), 5.151 (1.32), 5.951 (6.24), 6.929 (0.43), 7.147 (3.10), 7.162 (0.58), 7.168 (0.76), 7.176 (0.84), 7.184 (2.11), 7.199 (2.49), 7.205 (1.34), 7.239 (1.30), 7.245 (0.82), 7.255 (1.21), 7.259 (5.59), 7.270 (6.17), 7.275 (14.51), 7.290 (1.18), 7.451 (0.45), 7.863 (1.45).

### Example 649

### (3R)-2'-{6-amino-5-[(1S)-1-(3-cyanophenyl)ethoxy]pyridin-3-yl}-N-ethyl-5',6'-dihydrospiro[pyrrolidine-3,4'-pyrrolo[1,2-b]pyrazole]-1-carboxamide

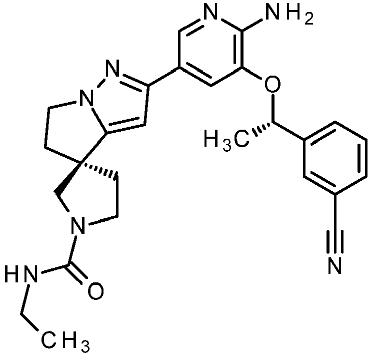

The compound was prepared similarly to **Example 643** using (3R)-1-(ethylcarbamoyl)-5',6'-dihydrospiro[pyrrolidine-3,4'-pyrrolo[1,2-b]pyrazol]-2'-yl trifluoromethanesulfonate (40.0 mg, 105 µmol) and {6-amino-5-[(1S)-1-(3-cyanophenyl)ethoxy]pyridin-3-yl}boronic acid **(Intermediate 453,** 101 mg, 41 % purity, 146 µmol) to give 29.3 mg (98 % purity, 58 % yield) of the title compound.
LC-MS (Method 1): Rt = 0.92 min; MS (ESlpos): m/z = 473 [M+H]⁺
¹H-NMR (400 MHz, CHLOROFORM-d) δ [ppm]: 0.007 (0.49), 1.157 (7.09), 1.175 (16.00), 1.193 (7.29), 1.250 (1.18), 1.260 (0.41), 1.679 (10.94), 1.695 (11.08), 2.035 (0.48), 2.049 (0.65), 2.053 (0.68), 2.066 (1.16), 2.080 (0.98), 2.084 (0.89), 2.099 (0.71), 2.189 (0.70), 2.208 (1.34), 2.220 (0.68), 2.225 (0.91), 2.238 (0.89), 2.256 (0.52), 2.507 (0.51), 2.524 (1.14), 2.539 (1.29), 2.557 (2.09), 2.574 (1.00), 2.601 (1.03), 2.636 (1.27), 2.651 (1.19), 2.669 (0.56), 3.285 (1.01), 3.299 (1.37), 3.303 (3.09), 3.316 (3.46), 3.321 (3.28), 3.335 (3.19), 3.338 (1.20), 3.352 (0.97), 3.491 (1.29), 3.500 (0.64), 3.515 (3.35), 3.524 (1.13), 3.546 (4.16), 3.560 (0.82), 3.570 (1.60), 3.593 (0.69), 3.608 (0.87), 3.613 (1.01), 3.627 (0.85), 3.632 (0.71), 3.637 (0.58), 4.172 (0.80), 4.186 (1.45), 4.199 (0.83), 4.216 (3.47), 4.234 (6.68), 4.252 (3.21), 4.789 (5.06), 5.309 (10.53), 5.472 (0.61), 5.488 (1.99), 5.503 (1.96), 5.520 (0.59), 6.074 (0.45), 6.085 (11.76), 7.226 (3.86), 7.230 (3.90), 7.459 (1.44), 7.479 (3.75), 7.499 (2.59), 7.570 (1.57), 7.574 (2.94), 7.577 (2.02), 7.590 (1.24), 7.593 (2.01), 7.597 (1.32), 7.624 (1.43), 7.628 (2.32), 7.631 (1.49), 7.644 (1.12), 7.648 (1.81), 7.694 (2.33), 7.699 (3.86), 7.999 (5.20), 8.003 (5.23).

### Example 650

### (3R)-2'-{6-amino-5-[(1S)-1-(4-cyanophenyl)ethoxy]pyridin-3-yl}-N-ethyl-5',6'-dihydrospiro[pyrrolidine-3,4'-pyrrolo[1,2-b]pyrazole]-1-carboxamide

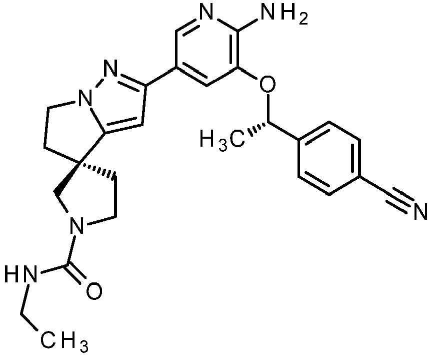

The compound was prepared similarly to **Example 643** using (3R)-1-(ethylcarbamoyl)-5',6'-dihydrospiro[pyrrolidine-3,4'-pyrrolo[1,2-b]pyrazol]-2'-yl trifluoromethanesulfonate (40.0 mg, 105 µmol) and {6-amino-5-[(1S)-1-(4-cyanophenyl)ethoxy]pyridin-3-yl}boronic acid **(Intermediate 454,** 104 mg, 40 % purity, 146 µmol) to give 33.5 mg (95 % purity, 65 % yield) of the title compound.
LC-MS (Method 1): Rt = 0.91 min; MS (ESlpos): m/z = 473 [M+H]⁺
¹H-NMR (400 MHz, CHLOROFORM-d) δ [ppm]: 0.007 (0.63), 1.156 (7.16), 1.174 (15.89), 1.192 (7.24), 1.250 (0.91), 1.678 (10.97), 1.695 (10.76), 2.030 (0.48), 2.044 (0.67), 2.048 (0.67), 2.061 (1.18), 2.075 (1.00), 2.079 (0.89), 2.094 (0.68), 2.182 (0.78), 2.201 (1.33), 2.213 (0.71), 2.218 (0.89), 2.231 (0.88), 2.250 (0.50), 2.502 (0.52), 2.519 (1.08), 2.535 (1.31), 2.552 (2.20), 2.569 (0.99), 2.597 (1.01), 2.633 (1.25), 2.648 (1.05), 2.665 (0.52), 3.285 (1.03), 3.299 (1.48), 3.303 (3.20), 3.317 (3.43), 3.321 (3.23), 3.335 (3.25), 3.339 (1.15), 3.352 (0.91), 3.488 (1.28), 3.499 (0.77), 3.513 (3.26), 3.523 (1.15), 3.541 (5.10), 3.559 (0.85), 3.565 (1.56), 3.587 (0.70), 3.602 (0.89), 3.607 (1.02), 3.621 (0.83), 3.631 (0.55), 4.156 (0.86), 4.170 (1.50), 4.183 (0.80), 4.205 (3.40), 4.223 (6.31), 4.240 (3.16), 4.789 (5.31), 5.309 (16.00), 5.485 (0.61), 5.502 (1.97), 5.517 (1.95), 5.533 (0.56), 6.054 (0.45), 6.081 (10.96), 7.191 (3.89), 7.195 (3.90), 7.494 (5.07), 7.510 (2.16), 7.514 (6.79), 7.647 (7.30), 7.651 (2.53), 7.663 (2.02), 7.668 (5.56), 7.671 (1.11), 7.996 (5.13), 8.001 (4.98).

### Example 651

### (3R)-2'-{6-amino-5-[(1S)-1-(3,5-difluoropyridin-4-yl)ethoxy]pyridin-3-yl}-N-ethyl-5',6'-dihydrospiro[pyrrolidine-3,4'-pyrrolo[1,2-b]pyrazole]-1-carboxamide

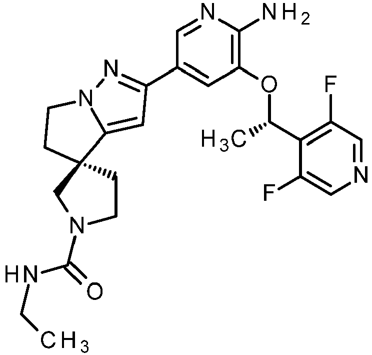

The compound was prepared similarly to **Example 643** using (3R)-1-(ethylcarbamoyl)-5',6'-dihydrospiro[pyrrolidine-3,4'-pyrrolo[1,2-b]pyrazol]-2'-yl trifluoromethanesulfonate (40.0 mg, 105 µmol) and {6-amino-5-[(1S)-1-(3,5-difluoropyridin-4-yl)ethoxy]pyridin-3-yl}boronic acid **(Intermediate 455,** 105 mg, 41 % purity, 146 µmol) to give 31.7 mg (98 % purity, 61 % yield) of the title compound.
[α]²⁰_{D}: -89.6° (c = 1.00, MeOH)
LC-MS (Method 1): Rt = 0.86 min; MS (ESlpos): m/z = 485 [M+H]⁺
¹H-NMR (400 MHz, CHLOROFORM-d) δ [ppm]: 0.007 (0.42), 1.163 (6.51), 1.181 (14.06), 1.199 (6.70), 1.250 (0.72), 1.814 (9.26), 1.830 (9.25), 2.056 (0.50), 2.069 (0.68), 2.073 (0.70), 2.087 (1.27), 2.101 (1.01), 2.118 (0.72), 2.215 (0.73), 2.234 (1.46), 2.246 (0.71), 2.252 (0.93), 2.265 (1.02), 2.283 (0.53), 2.525 (0.48), 2.543 (1.11), 2.558 (1.32), 2.576 (1.83), 2.592 (1.02), 2.634 (2.01), 2.652 (1.27), 2.667 (1.19), 2.684 (0.54), 3.294 (0.96), 3.307 (1.39), 3.311 (2.99), 3.325 (3.35), 3.330 (3.19), 3.343 (2.98), 3.361 (0.94), 3.509 (1.48), 3.533 (3.70), 3.555 (1.81), 3.565 (3.87), 3.589 (1.60), 3.615 (0.71), 3.628 (0.88), 3.634 (1.04), 3.647 (0.90), 3.659 (0.59), 3.671 (0.43), 4.165 (0.85), 4.179 (1.50), 4.192 (0.81), 4.244 (3.24), 4.262 (5.97), 4.280 (3.08), 4.787 (5.26), 5.310 (13.56), 5.874 (0.66), 5.890 (2.12), 5.907 (2.14), 5.923 (0.64), 6.128 (10.25), 7.407 (3.90), 7.411 (3.90), 8.039 (4.91), 8.043 (4.82), 8.345 (16.00).

### Example 652

### (3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1 and diastereomer 2)

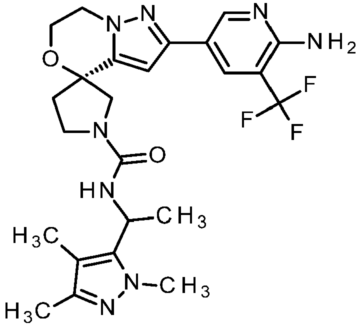

1-(1,3,4-Trimethyl-1H-pyrazol-5-yl)ethan-1-amine (47.0 mg, 307 µmol) was dissolved in DMF (2.5 mL). CDI (45.6 mg, 281 µmol) and N,N-diisopropylethylamine (180 µL, 1.0 mmol) were added and the mixture was stirred for 1 h at rt. A solution of 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (1/1) (120 mg, 80 % purity, 255 µmol) in DMF was added dropwise to the reaction mixture and stirred for 1 h at 60 °C. The reaction mixture was allowed to cool down, diluted with water and extracted 3 times with ethyl acetate. The combind organic layers were dried over a hydrophobic filter and concentrated. The crude product was purified by flash chromatography (silica gel, dichloromethane / methanol (0-15 %) gradient) to give 125 mg (95 % purity, 89 % yield) of the title compound.
LC-MS (Method 1): Rt = 0.92 min; MS (ESlpos): m/z = 519 [M+H]⁺
¹H-NMR (400 MHz, DMSO-d6) δ [ppm]: 1.326 (4.32), 1.333 (4.39), 1.345 (4.35), 1.350 (4.11), 2.069 (0.71), 2.120 (13.15), 2.135 (0.76), 2.165 (0.82), 2.191 (15.74), 2.216 (0.95), 2.228 (0.75), 2.240 (0.60), 2.283 (0.69), 2.294 (0.74), 2.309 (0.84), 2.326 (1.00), 2.522 (1.76), 2.669 (0.55), 2.673 (0.40), 3.159 (3.08), 3.172 (3.28), 3.384 (1.72), 3.413 (1.28), 3.435 (0.86), 3.464 (0.97), 3.504 (0.50), 3.527 (0.81), 3.563 (16.00), 3.582 (0.90), 3.606 (0.43), 3.615 (0.62), 3.732 (0.78), 3.760 (0.72), 3.801 (0.76), 3.830 (0.65), 4.063 (1.00), 4.095 (1.74), 4.100 (1.81), 4.124 (3.71), 4.681 (0.75), 4.686 (0.79), 4.698 (1.10), 4.703 (1.13), 4.715 (0.76), 4.720 (0.73), 5.758 (10.38), 6.084 (0.90), 6.101 (0.84), 6.130 (0.99), 6.147 (0.88), 6.595 (4.71), 6.724 (3.72), 6.740 (3.12), 7.012 (3.67), 7.416 (0.63), 7.421 (0.66), 7.639 (2.38), 8.006 (2.64), 8.574 (2.59).

### Example 653

### (3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1)

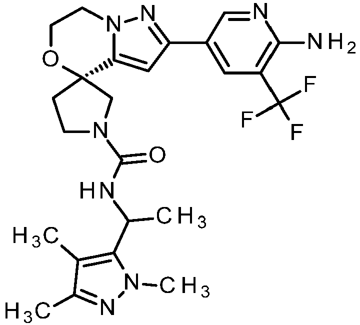

The title compound from **Example 652** was separated into diastereomers by preparative chiral HPLC to give 41.0 mg (100 % purity, 31 % yield) of the title compound (diastereomer 1, Rt = 8.0 - 10.7 min)

### Preparative chiral HPLC method: MTBE

Instrument: PrepCon Labomatic HPLC-3; Column: Chiralpak IF 5µ, 250x30; eluent A: methyl tert-butyl ether + 0.1 vol % diethylamine; eluent B: ethanol + 0.1 vol % diethylamine; isocratic: 80%A+20%B; flow: 40 mL/min; temperature: 25 °C; UV: 254 nm

### Analytical chiral HPLC method: MTBE

Instrument: Thermo Fisher UltiMate 3000; Column: Chiralpak IF 3µ, 100x4.6; eluent A: methyl tert-butyl ether + 0.1 vol % diethylamine; eluent B: ethanol; isocratic: 80%A+20%B; flow: 1.4 mL/min; temperature: 25 °C; UV: 254 nm
Analytical chiral HPLC: Rₜ = 2.99 min.
[α]²⁰_{D}: +37.3° (c = 1.00, MeOH)
LC-MS (Method 1): Rt = 0.92 min; MS (ESlpos): m/z = 519 [M+H]⁺
¹H-NMR (400 MHz, DMSO-d6) δ [ppm]: 0.798 (1.10), 0.803 (0.47), 0.814 (1.12), 0.821 (1.13), 0.840 (0.56), 0.886 (0.56), 0.904 (1.26), 0.922 (0.65), 1.137 (0.58), 1.183 (0.95), 1.324 (1.17), 1.333 (5.01), 1.342 (1.44), 1.350 (5.07), 2.074 (0.71), 2.113 (3.31), 2.118 (13.56), 2.185 (4.16), 2.190 (15.38), 2.210 (0.64), 2.293 (0.49), 2.308 (0.57), 2.322 (0.65), 2.326 (0.82), 2.331 (0.56), 2.518 (1.85), 2.522 (1.12), 2.669 (0.52), 3.294 (0.41), 3.313 (0.86), 3.363 (0.67), 3.384 (1.44), 3.413 (1.48), 3.562 (16.00), 3.582 (0.93), 3.800 (0.99), 3.829 (0.86), 4.047 (0.63), 4.064 (0.81), 4.083 (0.97), 4.095 (1.08), 4.104 (1.08), 4.123 (2.47), 4.685 (0.86), 4.703 (1.24), 4.720 (0.83), 6.083 (1.24), 6.100 (1.16), 6.235 (0.57), 6.239 (0.58), 6.596 (3.23), 6.724 (5.93), 7.417 (0.53), 7.421 (0.52), 8.005 (1.95), 8.010 (1.97), 8.574 (1.82), 8.578 (1.79).

### Example 654

### (3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 2)

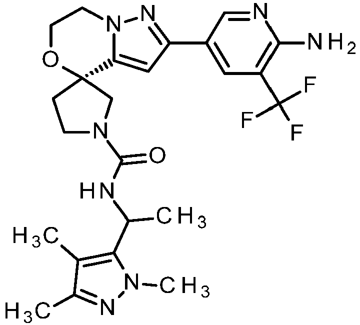

For the preparation of the diastereomeric mixture of the title compound see **Example 652.** The diastereomers were separated by preparative chiral HPLC (method see **Example 653)** to give 55.0 mg (100 % purity, 41 % yield) of the title compound (diastereomer 2, Rt = 12.1 - 15.1 min).
Analytical chiral HPLC (method see **Example 653):** Rt = 5.28 min
[α]²⁰_{D}: +62.5° (c = 1.00, MeOH)
LC-MS (Method 1): Rt = 0.92 min; MS (ESlpos): m/z = 519 [M+H]⁺
¹H-NMR (400 MHz, DMSO-d6) δ [ppm]: 1.326 (5.46), 1.344 (5.53), 2.073 (0.55), 2.120 (14.81), 2.186 (3.81), 2.191 (16.00), 2.217 (0.75), 2.240 (0.46), 2.294 (0.59), 2.311 (0.68), 2.322 (0.68), 2.326 (0.90), 2.331 (0.66), 2.522 (1.60), 2.668 (0.51), 3.375 (1.03), 3.384 (0.71), 3.401 (0.45), 3.435 (1.26), 3.464 (1.49), 3.505 (0.68), 3.526 (1.08), 3.564 (14.89), 3.731 (1.19), 3.758 (0.96), 4.026 (0.42), 4.041 (0.67), 4.057 (0.94), 4.094 (1.26), 4.104 (1.24), 4.124 (2.87), 4.680 (0.93), 4.697 (1.36), 4.715 (0.92), 6.130 (1.52), 6.147 (1.43), 6.246 (0.41), 6.251 (0.43), 6.594 (3.82), 6.740 (5.02), 7.416 (0.44), 7.421 (0.43), 8.002 (2.25), 8.007 (2.28), 8.568 (2.13), 8.573 (2.11).

### Example 655

### (3'R)-2-[6-amino-5-(trifluoromethyl)pyridin-3-yl]-N-[1-(1,5-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)

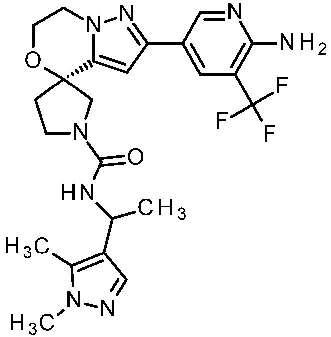

1-(1,5-Dimethyl-1H-pyrazol-4-yl)ethan-1-amine (20.9 mg, 150 µmol) was dissolved in DMF (1.5 mL). CDI (22.4 mg, 138 µmol) and N,N-diisopropylethylamine (87 µL, 500 µmol) were added and the mixture was stirred for 1 h at rt. A solution of 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (47.1 mg, 125 µmol) in DMF was added dropwise to the reaction mixture and stirred for 1 h at 60 °C. The reaction mixture was allowed to cool down, diluted with water and extracted 3 times with ethyl acetate. The combined organic layers were dried over a hydrophobic filter and concentrated. The crude product was purified by preparative HPLC and preparative TLC to give 14.5 mg (90 % purity, 21 % yield) of the title compound.
LC-MS (Method 1): Rt = 0.89 min; MS (ESlpos): m/z = 505 [M+H]⁺
¹H-NMR (400 MHz, CHLOROFORM-d) δ [ppm]: 0.782 (0.64), 0.792 (0.67), 0.809 (1.17), 0.826 (0.65), 0.835 (0.47), 0.883 (0.47), 1.120 (0.55), 1.138 (1.00), 1.147 (0.95), 1.157 (1.40), 1.183 (8.92), 1.214 (1.17), 1.263 (0.60), 1.394 (0.44), 1.428 (10.80), 1.444 (10.80), 1.500 (0.40), 1.506 (0.56), 1.523 (0.60), 2.179 (0.57), 2.188 (0.48), 2.204 (12.49), 2.210 (7.48), 2.221 (0.77), 2.235 (1.18), 2.244 (0.84), 2.260 (0.58), 2.269 (0.57), 2.328 (1.21), 2.343 (0.88), 2.362 (0.74), 3.442 (0.60), 3.462 (1.92), 3.467 (1.68), 3.477 (2.35), 3.490 (2.34), 3.497 (1.67), 3.506 (2.66), 3.524 (0.88), 3.540 (0.99), 3.596 (0.46), 3.695 (13.50), 3.698 (7.98), 3.788 (0.81), 3.800 (16.00), 3.823 (0.92), 3.850 (1.34), 3.873 (0.82), 4.064 (2.83), 4.069 (3.08), 4.076 (3.53), 4.085 (2.27), 4.142 (3.01), 4.148 (3.64), 4.156 (4.63), 4.195 (0.84), 4.203 (0.73), 4.215 (0.68), 4.223 (0.61), 4.860 (0.68), 4.867 (0.44), 4.878 (0.98), 4.885 (0.56), 4.895 (0.68), 4.947 (0.77), 4.966 (1.43), 4.982 (6.90), 6.193 (5.25), 6.195 (3.30), 6.202 (4.08), 6.204 (3.68), 7.273 (3.48), 7.308 (3.68), 7.375 (2.59), 7.897 (1.68), 7.901 (1.69), 7.918 (1.67), 7.922 (1.59), 8.027 (4.09), 8.407 (2.37), 8.411 (2.29), 8.504 (3.72).

### Example 656

### (3'R)-2-[6-amino-5-(trifluoromethyl)pyridin-3-yl]-N-[1-(1-methyl-1H-pyrazol-4-yl)ethyl]-6, 7-dihydro-1'H-spiro[pyrazolo[5,1-c][1,4]oxazine-4,3'-pyrrolidine]-1'-carboxamide (diastereomer 1 and diastereomer 2)

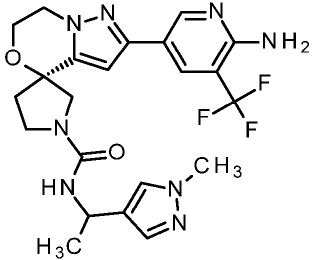

The compound was prepared similarly to **Example 655** using 5-[(3'R)-6,7-dihydrospiro[pyrazolo[5,1-c][1,4]oxazine-4,3'-pyrrolidin]-2-yl]-3-(trifluoromethyl)pyridin-2-amine-hydrogen chloride (47.1 mg, 125 µmol) and 1-(1-methyl-1H-pyrazol-4-yl)ethan-1-amine (18.8 mg, 150 µmol) to give 5.00 mg (90 % purity, 7 % yield) of the title compound.
LC-MS (Method 1): Rt = 0.86 min; MS (ESlpos): m/z = 491 [M+H]⁺
¹H-NMR (400 MHz, CHLOROFORM-d) δ [ppm]: 1.253 (2.54), 1.495 (5.91), 2.273 (2.48), 2.278 (1.77), 2.289 (0.59), 2.304 (0.57), 2.312 (0.76), 2.337 (0.55), 2.396 (0.68), 2.411 (0.68), 2.430 (0.44), 3.531 (0.55), 3.547 (2.15), 3.559 (0.86), 3.568 (0.89), 3.575 (2.43), 3.594 (0.77), 3.616 (0.69), 3.763 (2.63), 3.767 (1.49), 3.867 (16.00), 3.918 (0.87), 3.945 (0.66), 4.126 (0.98), 4.132 (1.24), 4.143 (2.31), 4.153 (2.16), 4.216 (2.38), 4.224 (2.28), 4.229 (2.30), 4.238 (1.16), 4.243 (1.00), 4.288 (0.64), 4.296 (0.68), 4.307 (0.70), 4.315 (0.65), 5.015 (0.70), 5.033 (1.00), 5.051 (0.69), 5.093 (3.98), 6.262 (1.07), 6.265 (0.75), 6.271 (4.08), 6.274 (3.76), 6.340 (0.44), 7.270 (4.85), 7.376 (0.78), 7.443 (2.93), 8.094 (2.39), 8.571 (2.26), 8.574 (2.09).

### Example 657

### (3R)-2'-{6-amino-5-[(1R)-1-(3-fluoropyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide

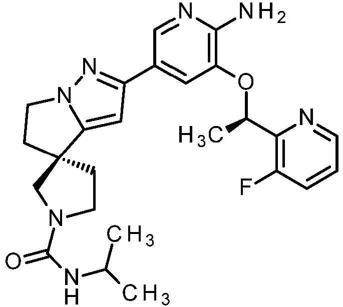

5-[(3R)-5',6'-Dihydrospiro[pyrrolidine-3,4'-pyrrolo[1,2-b]pyrazol]-2'-yl]-3-[(1R)-1-(3-fluoropyridin-2-yl)ethoxy]pyridin-2-amine-hydrochloride **(Intermediate 517,** 70.0 mg, 35 % purity, 56.9 µmol) was suspended in dichloromethane (0.9 mL). N,N-Diisopropylethylamine (99 µL, 570 µmol) was added, cooled to 0 °C, isopropyl isocyanate (6.7 µL, 68 µmol) was added and the mixture was allowed to warm up to rt over 2 h. The reaction mixture was diluted with water and brine, extracted 2 times with dichloromethane. The combined organic layers were dried over sodium sulfate, filtered and concentrated. The residue was purified by preparative TLC (gradient: dichloromethane / methanol 9:1) to give 22.8 mg (98 % purity, 82 % yield) of the title compound.
LC-MS (Method 1): Rt = 0.92 min; MS (ESlpos): m/z = 481 [M+H]⁺
¹H-NMR (400 MHz, CHLOROFORM-d) δ [ppm]: 1.179 (11.95), 1.186 (12.83), 1.195 (13.52), 1.202 (11.04), 1.260 (0.49), 1.783 (12.69), 1.799 (12.59), 2.044 (0.58), 2.058 (0.77), 2.062 (0.79), 2.075 (1.48), 2.089 (1.23), 2.092 (1.45), 2.107 (0.83), 2.207 (0.89), 2.226 (1.81), 2.238 (0.83), 2.244 (1.10), 2.256 (1.27), 2.275 (0.66), 2.515 (0.61), 2.533 (1.40), 2.548 (1.60), 2.565 (2.11), 2.582 (1.24), 2.642 (1.61), 2.656 (1.54), 2.674 (0.69), 3.482 (1.43), 3.497 (0.87), 3.507 (3.58), 3.514 (1.83), 3.521 (1.26), 3.537 (5.74), 3.561 (2.13), 3.604 (0.82), 3.617 (0.99), 3.624 (1.16), 3.636 (1.01), 3.647 (0.68), 3.661 (0.50), 3.979 (0.86), 3.998 (3.72), 4.012 (2.08), 4.027 (1.46), 4.045 (0.70), 4.236 (4.15), 4.254 (7.39), 4.271 (3.82), 4.862 (6.21), 5.310 (14.29), 5.794 (0.81), 5.811 (2.61), 5.826 (2.57), 5.843 (0.77), 6.106 (16.00), 7.006 (0.52), 7.247 (1.65), 7.258 (2.47), 7.278 (3.32), 7.290 (2.19), 7.378 (2.22), 7.381 (2.16), 7.399 (1.85), 7.402 (3.42), 7.406 (2.38), 7.424 (1.57), 7.427 (1.66), 7.449 (4.89), 7.454 (4.86), 7.528 (0.51), 8.029 (6.68), 8.034 (6.55), 8.041 (0.41), 8.429 (1.88), 8.433 (3.39), 8.436 (2.02), 8.441 (2.01), 8.445 (3.23), 8.448 (1.84).

### Example 658

### (3R)-2'-{6-amino-5-[(1R)-1-(3-fluoropyridin-2-yl)ethoxy]pyridin-3-yl}-N-cyclobutyl-5',6'-dihydrospiro[pyrrolidine-3,4'-pyrrolo[1,2-b]pyrazole]-1-carboxamide

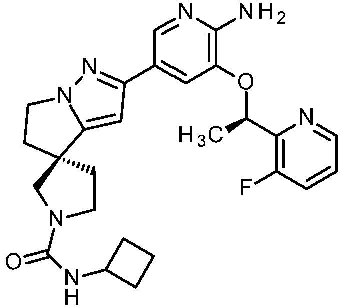

5-[(3R)-5',6'-Dihydrospiro[pyrrolidine-3,4'-pyrrolo[1,2-b]pyrazol]-2'-yl]-3-[(1R)-1-(3-fluoropyridin-2-yl)ethoxy]pyridin-2-amine-hydrochloride **(Intermediate 517,** 70.0 mg, 35 % purity, 56.9 µmol) was suspended in dichloromethane (0.9 mL). N,N-Diisopropylethylamine (99 µL, 570 µmol) was added, cooled to 0 °C, cyclobutyl isocyanate (6.5 µL, 68 µmol) was added and the mixture was allowed to warm up to rt over 2 h. The reaction mixture was diluted with water and brine, extracted 2 times with dichloromethane. The combined organic layers were dried over sodium sulfate, filtered and concentrated. The residue was purified by preparative TLC (gradient: dichloromethane / methanol 9:1) to give 23.7 mg (98 % purity, 83 % yield) of the title compound.
LC-MS (Method 1): Rt = 0.94 min; MS (ESlpos): m/z = 493 [M+H]⁺
¹H-NMR (400 MHz, CHLOROFORM-d) δ [ppm]: 1.260 (0.49), 1.657 (2.96), 1.666 (2.37), 1.683 (2.47), 1.688 (2.08), 1.693 (1.32), 1.698 (1.63), 1.710 (2.54), 1.721 (0.97), 1.724 (0.89), 1.783 (10.63), 1.799 (11.00), 1.816 (1.14), 1.829 (1.20), 1.838 (1.15), 1.855 (0.82), 2.042 (0.48), 2.056 (0.66), 2.060 (0.65), 2.073 (1.18), 2.087 (0.98), 2.092 (1.08), 2.105 (0.68), 2.203 (0.71), 2.222 (1.42), 2.234 (0.67), 2.240 (0.87), 2.253 (0.99), 2.272 (0.52), 2.340 (0.59), 2.349 (0.75), 2.358 (1.25), 2.368 (1.46), 2.377 (1.43), 2.382 (1.25), 2.386 (1.31), 2.393 (1.01), 2.510 (0.48), 2.527 (1.08), 2.543 (1.27), 2.560 (1.64), 2.577 (1.00), 2.600 (1.04), 2.636 (1.29), 2.650 (1.23), 2.668 (0.56), 3.485 (1.07), 3.510 (2.81), 3.519 (1.31), 3.526 (1.08), 3.535 (4.22), 3.544 (1.77), 3.560 (1.91), 3.605 (0.65), 3.618 (0.79), 3.624 (0.94), 3.637 (0.81), 3.648 (0.54), 4.233 (3.17), 4.251 (5.66), 4.268 (2.95), 4.313 (0.42), 4.334 (1.57), 4.345 (3.17), 4.360 (1.01), 4.380 (0.46), 4.862 (5.13), 5.310 (16.00), 5.794 (0.66), 5.810 (2.12), 5.826 (2.11), 5.843 (0.64), 6.104 (11.79), 7.006 (0.43), 7.247 (1.33), 7.258 (2.03), 7.279 (2.74), 7.290 (1.72), 7.378 (1.75), 7.381 (1.87), 7.399 (1.44), 7.402 (2.66), 7.405 (1.85), 7.423 (1.39), 7.426 (1.32), 7.448 (3.95), 7.452 (4.00), 7.528 (0.44), 8.029 (5.33), 8.033 (5.23), 8.429 (1.54), 8.432 (2.70), 8.436 (1.63), 8.440 (1.63), 8.444 (2.68), 8.447 (1.47).

### Example 659

### (3R)-2'-{6-amino-5-[(1R)-1-(3,5-difluoropyridin-4-yl)ethoxy]pyridin-3-yl}-N-(propan-2-yl)-5',6'-dihydrospiro[pyrrolidine-3,4'-pyrrolo[1,2-b]pyrazole]-1-carboxamide

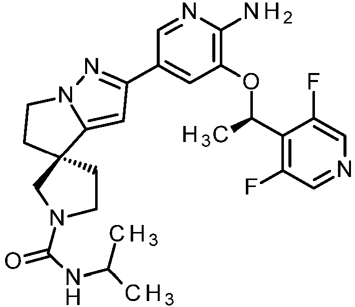

3-[(1R)-1-(3,5-Difluoropyridin-4-yl)ethoxy]-5-[(3R)-5',6'-dihydrospiro[pyrrolidine-3,4'-pyrrolo[1,2-b]pyrazol]-2'-yl]pyridin-2-amine-hydrochloride **(Intermediate 518,** 70.0 mg, 40 % purity, 62.4 µmol) was suspended in dichloromethane (1.1 mL). N,N-Diisopropylethylamine (110 µL, 620 µmol) was added, cooled to 0 °C and isopropyl isocyanate (7.4 µL, 75 µmol) was added. The mixture was allowed to warm up to rt over 2 h. The reaction mixture was diluted with water and brine, extracted 2 times with dichloromethane. The combined organic layers were dried over sodium sulfate, filtered and concentrated. The residue was purified by preparative TLC (gradient: dichloromethane / methanol 9:1) to give 21.6 mg (98 % purity, 68 % yield) of the title compound.
LC-MS (Method 1): Rt = 0.94 min; MS (ESlpos): m/z = 499 [M+H]⁺
¹H-NMR (400 MHz, CHLOROFORM-d) δ [ppm]: 1.177 (8.84), 1.186 (9.61), 1.192 (10.12), 1.201 (8.14), 1.261 (0.51), 1.814 (8.57), 1.831 (8.54), 2.055 (0.44), 2.068 (0.61), 2.073 (0.61), 2.086 (1.12), 2.099 (1.20), 2.117 (0.63), 2.218 (0.65), 2.237 (1.35), 2.250 (0.64), 2.256 (0.82), 2.269 (0.93), 2.287 (0.48), 2.526 (0.47), 2.543 (1.09), 2.559 (1.17), 2.576 (1.76), 2.593 (0.94), 2.637 (1.95), 2.655 (1.19), 2.669 (1.13), 2.687 (0.50), 3.491 (1.13), 3.503 (0.61), 3.516 (2.81), 3.527 (0.95), 3.545 (4.74), 3.563 (0.80), 3.570 (1.47), 3.610 (0.59), 3.622 (0.75), 3.628 (0.86), 3.642 (0.77), 3.652 (0.51), 3.979 (0.66), 3.997 (2.81), 4.011 (1.55), 4.027 (1.09), 4.045 (0.52), 4.245 (3.13), 4.263 (5.91), 4.281 (2.88), 4.787 (4.79), 5.310 (7.35), 5.874 (0.60), 5.890 (1.94), 5.907 (1.93), 5.923 (0.57), 6.123 (10.85), 7.006 (0.52), 7.401 (3.54), 7.405 (3.58), 7.528 (0.51), 8.041 (4.89), 8.044 (4.79), 8.346 (16.00).

### Example 660

### (3R)-2'-{6-amino-5-[(1R)-1-(3,5-difluoropyridin-4-yl)ethoxy]pyridin-3-yl}-N-cyclobutyl-5',6'-dihydrospiro[pyrrolidine-3,4'-pyrrolo[1,2-b]pyrazole]-1-carboxamide

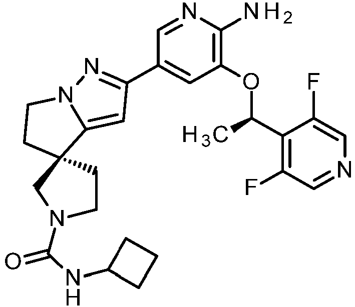

3-[(1R)-1-(3,5-Difluoropyridin-4-yl)ethoxy]-5-[(3R)-5',6'-dihydrospiro[pyrrolidine-3,4'-pyrrolo[1,2-b]pyrazol]-2'-yl]pyridin-2-amine-hydrochloride **(Intermediate 518,** 70.0 mg, 40 % purity, 62.4 µmol) was suspended in dichloromethane (1.1 mL). N,N-Diisopropylethylamine (110 µL, 620 µmol) was added, cooled to 0 °C, cyclobutyl isocyanate (7.1 µL, 75 µmol) was added and the mixture was allowed to warm up to rt over 2 h. The reaction mixture was diluted with water and brine, extracted 2 times with dichloromethane. The combined organic layers were dried over sodium sulfate, filtered and concentrated. The residue was purified by preparative TLC (gradient: dichloromethane / methanol 9:1) to give 24.0 mg (98 % purity, 74 % yield) of the title compound.
LC-MS (Method 1): Rt = 0.97 min; MS (ESlpos): m/z = 511 [M+H]⁺
¹H-NMR (400 MHz, CHLOROFORM-d) δ [ppm]: 1.261 (0.42), 1.640 (1.03), 1.657 (1.22), 1.666 (1.54), 1.683 (2.03), 1.688 (1.77), 1.693 (1.11), 1.698 (1.46), 1.710 (2.28), 1.720 (0.89), 1.723 (0.79), 1.795 (0.83), 1.814 (9.67), 1.831 (9.58), 1.851 (0.81), 2.053 (0.43), 2.066 (0.59), 2.071 (0.61), 2.084 (1.11), 2.097 (1.01), 2.115 (0.62), 2.214 (0.64), 2.233 (1.29), 2.246 (0.61), 2.251 (0.80), 2.264 (0.92), 2.283 (0.48), 2.339 (0.54), 2.347 (0.73), 2.356 (1.19), 2.366 (1.41), 2.376 (1.31), 2.380 (1.26), 2.384 (1.21), 2.521 (0.46), 2.539 (1.03), 2.554 (1.17), 2.571 (1.65), 2.588 (0.92), 2.630 (1.87), 2.648 (1.16), 2.663 (1.14), 2.681 (0.51), 3.494 (1.01), 3.518 (2.70), 3.525 (1.33), 3.532 (1.00), 3.544 (4.07), 3.549 (1.81), 3.568 (1.89), 3.610 (0.60), 3.624 (0.75), 3.630 (0.87), 3.643 (0.76), 3.654 (0.50), 4.243 (3.05), 4.261 (5.63), 4.278 (2.80), 4.333 (1.53), 4.344 (2.98), 4.360 (0.94), 4.379 (0.45), 4.783 (4.95), 5.310 (7.83), 5.874 (0.60), 5.890 (1.95), 5.907 (1.95), 5.923 (0.59), 6.121 (10.39), 7.006 (0.42), 7.400 (3.58), 7.404 (3.64), 7.528 (0.43), 8.040 (4.86), 8.044 (4.91), 8.346 (16.00).

### Example 661

### (3R)-2'-{6-amino-5-[(1R)-1-phenylethoxy]pyridin-3-yl}-N-ethyl-5',6'-dihydrospiro[pyrrolidine-3,4'-pyrrolo[1,2-b]pyrazole]-1-carboxamide

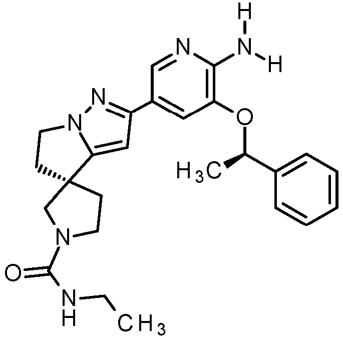

(3R)-1-(Ethylcarbamoyl)-5',6'-dihydrospiro[pyrrolidine-3,4'-pyrrolo[1,2-b]pyrazol]-2'-yl trifluoromethanesulfonate (117 mg, 305 µmol), {6-amino-5-[(1R)-1-phenylethoxy]pyridin-3-yl}boronic acid **(Intermediate 456,** 397 mg, 22 % purity, 335 µmol) and potassium phosphate (1.8 mL, 0.50 M, 910 µmol) were suspended in degassed 1,4-dioxane (3.4 mL) under nitrogen. XPhos Pd G2 (12.0 mg, 15.2 µmol) was added and the mixture was stirred for 2 h at 100 °C. The reaction mixture was allowed to cool down, diluted with ethyl acetate and filtered. The filtrate was washed with water and brine. The organic phase was dried over a hydrophobic filter and concentrated. The crude product was purified by flash column chromatography (silica gel, dichloromethane / methanol 9:1 gradient) to give 20.1 mg (90 % purity, 13 % yield) of the title compound.
LC-MS (Method 1): Rt = 0.98 min; MS (ESlpos): m/z = 448 [M+H]⁺
¹H-NMR (400 MHz, CHLOROFORM-d) δ [ppm]: 1.078 (7.35), 1.096 (16.00), 1.114 (7.54), 1.173 (0.42), 1.183 (0.96), 1.191 (0.66), 1.204 (0.78), 1.601 (12.99), 1.617 (12.74), 1.959 (0.58), 1.973 (0.79), 1.978 (1.31), 1.990 (1.40), 2.003 (1.13), 2.008 (1.03), 2.022 (0.83), 2.115 (0.82), 2.134 (1.57), 2.147 (0.79), 2.153 (1.04), 2.165 (1.10), 2.184 (0.60), 2.424 (0.56), 2.442 (1.04), 2.457 (1.47), 2.475 (1.70), 2.492 (1.12), 2.513 (1.13), 2.529 (1.83), 2.548 (1.41), 2.563 (1.14), 2.580 (0.58), 3.207 (1.08), 3.220 (1.48), 3.224 (3.35), 3.238 (3.67), 3.243 (3.55), 3.256 (3.29), 3.260 (1.46), 3.274 (1.07), 3.403 (1.26), 3.427 (3.56), 3.441 (1.51), 3.451 (4.70), 3.465 (1.95), 3.476 (1.73), 3.483 (0.89), 3.519 (0.81), 3.533 (0.98), 3.538 (1.14), 3.552 (1.03), 3.562 (0.68), 3.576 (0.50), 4.075 (0.94), 4.089 (1.62), 4.102 (0.91), 4.138 (2.95), 4.156 (5.23), 4.173 (2.60), 4.764 (3.48), 5.232 (12.88), 5.366 (0.72), 5.382 (2.34), 5.398 (2.35), 5.414 (0.72), 5.980 (10.63), 7.182 (0.86), 7.187 (1.66), 7.200 (5.69), 7.210 (1.33), 7.217 (1.39), 7.222 (2.37), 7.225 (1.49), 7.261 (2.49), 7.277 (2.36), 7.281 (6.08), 7.294 (1.29), 7.299 (4.81), 7.309 (5.63), 7.313 (6.53), 7.330 (2.69), 7.334 (1.92), 7.906 (4.50), 7.909 (4.42).

### Example 662

### (3R)-2'-{6-amino-5-[1-(1-methyl-1H-pyrazol-5-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 1)

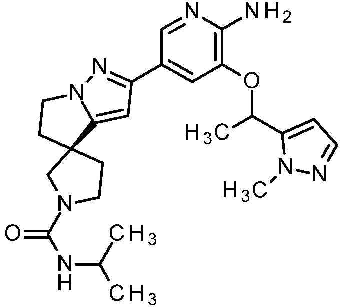

5-[(3R)-5',6'-Dihydrospiro[pyrrolidine-3,4'-pyrrolo[1,2-b]pyrazol]-2'-yl]-3-[1-(1-methyl-1H-pyrazol-5-yl)ethoxy]pyridin-2-amine-hydrochloride **(Intermediate 519,** 60.0 mg, 80 % purity, 115 µmol) was suspended in dichloromethane (2.0 mL). N,N-Diisopropylethylamine (200 µL, 1.2 mmol) was added and cooled to 0 °C. Isopropyl isocyanate (14 µL, 140 µmol) was added and the mixture was allowed to warm up to rt over 2 h. The reaction mixture was concentrated and purified by column chromatography (silica gel, dichloromethane / methanol (0-20 %) gradient). The combined fractions were diluted with water and brine and extracted 2 times with ethyl acetate. The organic phase was dried over sodium sulfate, filtered and concentrated to give 41.8 mg (78 % yield) of the title compound as a diastereomeric mixture.

The diastereomers were separated by preparative chiral HPLC to give 14.2 mg (95 % purity, 32 % yield) of the title compound (diastereomer 1, Rₜ = 6.4 - 7.0 min)

### Preparative chiral HPLC method:

Instrument: PrepCon Labomatic HPLC-2; Column: YMC Cellulose SB 5µ, 250x30; eluent A: methyl tert-butyl ether + 0.1 vol % diethylamine; eluent B: ethanol + 0.1 vol % diethylamine; isocratic: 80%A+20%B; flow: 40 mL/min; temperature: 25 °C; UV: 280 nm

### Analytical chiral HPLC method:

Instrument: Thermo Fisher UltiMate 3000; Column: YMC Cellulose SB 3µ, 100x4.6; eluent A: methyl tert-butyl ether + 0.1 vol % diethylamine; eluent B: ethanol; isocratic: 80%A+20%B; flow: 1.4 mL/min; temperature: 25 °C; UV: 280 nm
Analytical chiral HPLC: Rt = 3.26 min.
[α]²⁰_{D}: +82.6° (c = 1.00, MeOH)
LC-MS (Method 1): Rt = 0.84 min; MS (ESlpos): m/z = 466 [M+H]⁺
¹H-NMR (400 MHz, CHLOROFORM-d) δ [ppm]: 1.180 (3.98), 1.188 (4.46), 1.195 (4.79), 1.204 (3.76), 1.263 (1.54), 1.759 (5.65), 1.775 (5.57), 2.092 (0.54), 2.105 (0.43), 2.241 (0.60), 2.271 (0.41), 2.553 (0.51), 2.568 (0.56), 2.585 (0.78), 2.602 (0.44), 2.630 (0.45), 2.647 (0.87), 2.665 (0.55), 2.680 (0.54), 3.499 (0.55), 3.523 (1.44), 3.532 (0.47), 3.554 (1.80), 3.578 (0.68), 3.632 (0.41), 3.870 (16.00), 4.001 (1.24), 4.013 (0.80), 4.028 (0.55), 4.251 (1.44), 4.268 (2.61), 4.286 (1.34), 4.822 (0.44), 5.310 (6.55), 5.568 (0.93), 5.583 (0.92), 6.162 (4.78), 6.337 (1.97), 6.342 (1.98), 7.448 (2.29), 7.453 (3.56), 7.457 (1.70), 8.035 (1.19), 8.039 (1.17).

### Example 663

### (3R)-2'-{6-amino-5-[1-(1-methyl-1H-pyrazol-5-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 2)

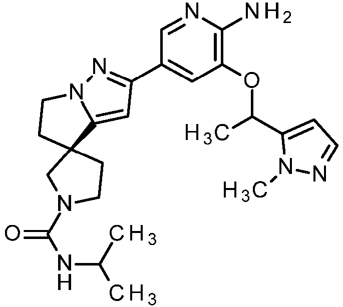

For the preparation of the diasteroemric mixture of the title compound see **Example 662.** The diastereomers were separated by preparative chiral HPLC (method see **Example 662)** to give 11.4 mg (95 % purity, 26 % yield) of the title compound (diastereomer 2, Rₜ = 7.2 - 7.8 min).

Analytical chiral HPLC (method see **Example 662):** Rₜ = 3.71 min.
[α]²⁰_{D}: +0.8° (c = 1.00, MeOH)
¹H-NMR (400 MHz, CHLOROFORM-d) δ [ppm]: 1.182 (4.30), 1.188 (4.94), 1.197 (5.21), 1.204 (4.04), 1.263 (1.02), 1.757 (5.65), 1.773 (5.79), 2.092 (0.63), 2.106 (0.48), 2.240 (0.68), 2.258 (0.44), 2.271 (0.46), 2.552 (0.54), 2.567 (0.62), 2.584 (0.92), 2.601 (0.47), 2.628 (0.47), 2.646 (1.02), 2.663 (0.60), 2.679 (0.59), 3.501 (0.68), 3.525 (1.75), 3.547 (0.81), 3.559 (1.79), 3.584 (0.76), 3.623 (0.40), 3.629 (0.48), 3.643 (0.41), 3.868 (16.00), 4.002 (1.39), 4.014 (0.92), 4.029 (0.58), 4.250 (1.54), 4.267 (2.98), 4.285 (1.47), 4.735 (0.82), 5.310 (4.74), 5.568 (1.05), 5.583 (1.06), 6.166 (4.58), 6.337 (2.22), 6.342 (2.21), 7.446 (2.47), 7.451 (3.26), 7.457 (1.89), 8.043 (1.51), 8.047 (1.48).

### Example 664

### (3R)-2'-{6-amino-5-[1-(1-methyl-1H-pyrazol-5-yl)ethoxy]pyridin-3-yl}-N-cyclobutyl-5',6'-dihydro-1H-spiro[pyrrolidine-3,4'-pyrrolo[1,2-b]pyrazole]-1-carboxamide (diastereomer 1)

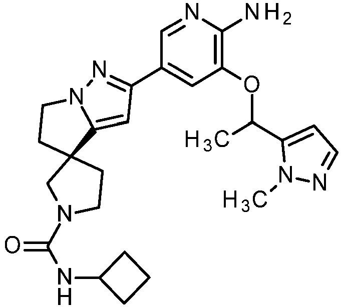

5-[(3R)-5',6'-Dihydrospiro[pyrrolidine-3,4'-pyrrolo[1,2-b]pyrazol]-2'-yl]-3-[1-(1-methyl-1H-pyrazol-5-yl)ethoxy]pyridin-2-amine-hydrochloride **(Intermediate 519,** 60.0 mg, 80 % purity, 115 µmol) was suspended in dichloromethane (2.0 mL). N,N-Diisopropylethylamine (200 µL, 1.2 mmol) was added and cooled to 0 °C. Cyclobutyl isocyanate (13 µL, 140 µmol) was added and the mixture was allowed to warm up to rt over 2 h. The reaction mixture was concentrated. The residue was purified by column chromatography (silica gel, dichloromethane / methanol (0-10 %) gradient). The combined fractions were diluted with water and brine and extracted 2 times with ethyl acetate. The organic phase was dried over sodium sulfate, filtered and concentrated to give 39.8 mg (72 % yield) of the title compound as a diastereomeric mixture.

The diastereomers were separated by preparative chiral HPLC to give 15.0 mg (95 % purity, 36 % yield) of the title compound (diastereomer 1, Rₜ = 9.6 - 11.1 min)

### Preparative chiral HPLC method:

Instrument: PrepCon Labomatic HPLC-2; Column: YMC Cellulose SB 5µ, 250x30; eluent A: methyl tert-butyl ether + 0.1 vol % diethylamine; eluent B: ethanol + 0.1 vol % diethylamine; isocratic: 80%A+20%B; flow: 40 mL/min; temperature: 25 °C; UV: 280 nm

### Analytical chiral HPLC method:

Instrument: Thermo Fisher UltiMate 3000; Column: YMC Cellulose SB 3µ, 100x4.6; eluent A: methyl tert-butyl ether + 0.1 vol % diethylamine; eluent B: ethanol; isocratic: 80%A+20%B; flow: 1.4 mL/min; temperature: 25°C; UV: 280 nm
Analytical chiral HPLC: Rt = 4.36 min.
[α]²⁰_{D}: +94.7° (c = 1.00, MeOH)
LC-MS (Method 1): Rt = 0.87 min; MS (ESlpos): m/z = 478 [M+H]⁺
¹H-NMR (400 MHz, CHLOROFORM-d) δ [ppm]: 1.263 (1.08), 1.643 (0.94), 1.660 (1.12), 1.669 (1.19), 1.686 (1.31), 1.691 (1.15), 1.696 (0.76), 1.701 (0.92), 1.712 (1.30), 1.723 (0.56), 1.757 (5.69), 1.773 (5.61), 1.798 (0.42), 1.808 (0.49), 1.816 (0.51), 1.827 (0.57), 1.837 (0.55), 2.089 (0.55), 2.102 (0.47), 2.236 (0.63), 2.254 (0.42), 2.267 (0.43), 2.358 (0.57), 2.368 (0.70), 2.378 (0.63), 2.383 (0.64), 2.386 (0.64), 2.394 (0.49), 2.548 (0.51), 2.563 (0.58), 2.580 (0.78), 2.597 (0.44), 2.640 (0.86), 2.658 (0.60), 2.673 (0.54), 3.500 (0.55), 3.525 (1.37), 3.538 (0.50), 3.552 (2.03), 3.577 (0.81), 3.633 (0.47), 3.868 (16.00), 4.248 (1.48), 4.266 (2.64), 4.283 (1.34), 4.333 (0.59), 4.348 (1.52), 4.362 (0.58), 4.727 (1.32), 5.310 (6.60), 5.565 (1.03), 5.581 (1.00), 6.161 (4.86), 6.337 (2.11), 6.342 (2.13), 7.447 (3.89), 7.452 (3.46), 8.048 (1.28), 8.051 (1.26).

### Example 665

### (3R)-2'-{6-amino-5-[1-(1-methyl-1H-pyrazol-5-yl)ethoxy]pyridin-3-yl}-N-cyclobutyl-5',6'-dihydro-1H-spiro[pyrrolidine-3,4'-pyrrolo[1,2-b]pyrazole]-1-carboxamide (diastereomer 2)

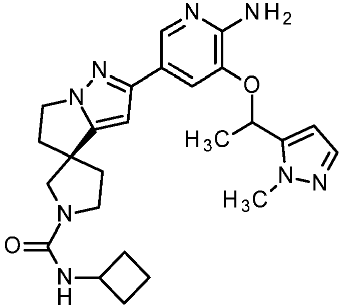

For the preparation of the diastereomeric mixture of the title compound see **Example 664.** The diastereomers were separated by preparative chiral HPLC (method see **Example 664)** to give 15.6 mg (95 % purity, 37 % yield) of the title compound (diastereomer 2, Rₜ = 11.3 - 13.2 min).

Analytical chiral HPLC (method see **Example 664):** Rt = 5.06 min.
[α]²⁰_{D}: +6.9° (c = 1.00, MeOH)
¹H-NMR (400 MHz, CHLOROFORM-d) δ [ppm]: 1.263 (1.08), 1.644 (0.65), 1.660 (0.97), 1.670 (1.12), 1.687 (1.26), 1.691 (1.10), 1.696 (0.75), 1.701 (0.88), 1.714 (1.22), 1.723 (0.55), 1.727 (0.49), 1.757 (5.25), 1.773 (5.24), 1.810 (0.45), 1.818 (0.45), 1.829 (0.52), 1.839 (0.49), 2.090 (0.50), 2.104 (0.42), 2.236 (0.55), 2.359 (0.49), 2.370 (0.64), 2.378 (0.57), 2.383 (0.57), 2.387 (0.56), 2.396 (0.43), 2.547 (0.48), 2.562 (0.53), 2.580 (0.75), 2.596 (0.40), 2.639 (0.84), 2.658 (0.52), 2.672 (0.52), 3.503 (0.50), 3.528 (1.46), 3.557 (1.71), 3.582 (0.63), 3.868 (16.00), 4.247 (1.35), 4.264 (2.52), 4.282 (1.26), 4.335 (0.51), 4.348 (1.33), 4.362 (0.53), 4.732 (1.05), 5.310 (5.94), 5.567 (0.90), 5.583 (0.89), 6.164 (4.54), 6.338 (1.91), 6.342 (1.91), 7.446 (2.37), 7.450 (3.42), 8.043 (1.49), 8.046 (1.48).

### Example 666

### (3R)-2'-{6-amino-5-[(1R)-1-(2-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide

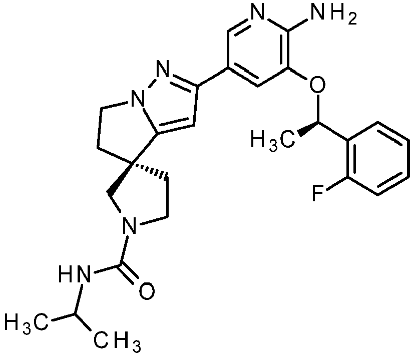

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (75.0 mg, 189 µmol), {6-amino-5-[(1R)-1-(2-fluorophenyl)ethoxy]pyridin-3-yl}boronic acid **(Intermediate 458,** 293 mg, 25 % purity, 265 µmol) and potassium phosphate (1.1 mL, 0.50 M, 570 µmol) were dissolved in degassed 1,4-dioxane (3.1 mL) under argon. XPhos Pd G2 (7.44 mg, 9.46 µmol) was added and the mixture was stirred for 2 h at 100 °C. The reaction mixture was allowed to cool down, diluted with ethyl acetate and filtered. The filtrate was washed with water and brine. The organic phase was dried over sodium sulfate, filtered and concentrated. The crude product was purified by preparative TLC (gradient: dichloromethane / methanol 9:1) to give 46.0 mg (95 % purity, 48 % yield) of the title compound.
LC-MS (Method 1): Rt = 1.06 min; MS (ESlpos): m/z = 480 [M+H]⁺
¹H-NMR (400 MHz, CHLOROFORM-d) δ [ppm]: 1.164 (3.29), 1.172 (3.53), 1.180 (3.73), 1.187 (3.05), 1.241 (2.00), 1.684 (3.57), 1.700 (3.51), 2.054 (0.40), 2.207 (0.51), 2.525 (0.44), 2.542 (0.58), 2.599 (0.66), 2.632 (0.42), 3.484 (0.96), 3.495 (0.44), 3.512 (1.45), 3.519 (0.63), 3.537 (0.69), 3.982 (0.85), 3.996 (0.56), 4.211 (1.13), 4.229 (1.99), 4.246 (1.01), 4.778 (1.65), 5.299 (5.19), 5.754 (0.68), 5.770 (0.67), 6.063 (4.26), 7.040 (0.41), 7.058 (0.49), 7.060 (0.55), 7.063 (0.46), 7.066 (0.45), 7.084 (0.45), 7.086 (0.51), 7.113 (0.89), 7.116 (0.80), 7.132 (0.57), 7.135 (0.50), 7.242 (0.51), 7.260 (16.00), 7.267 (1.68), 7.408 (0.65), 7.412 (0.64), 8.007 (1.92), 8.011 (1.92).

### Example 667

### 2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 1 and enantiomer 2)

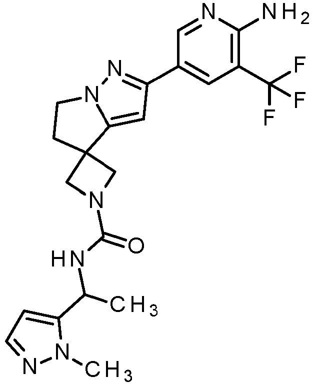

1-(1-Methyl-1H-pyrazol-5-yl)ethan-1-amine (25.0 mg, 200 µmol), CDI (35.3 mg, 218 µmol) and N,N-diisopropylethylamine (130 µL, 730 µmol) were dissolved in DMF and stirred for 1 h. A solution of 5-(5',6'-dihydrospiro[azetidine-3,4'-pyrrolo[1,2-b]pyrazol]-2'-yl)-3-(trifluoromethyl)pyridin-2-amine-hydrogen chloride (62.8 mg, 182 µmol) in DMF was added dropwise. The mixture was stirred over night at rt. The reaction mixture was diluted with water and lyophilised. The crude product was purified by column chromatography (silica gel, dichloromethane / methanol (0-15 %) gradient) to give 63.1 mg (94 % purity, 71 % yield) of the title compound.
LC-MS (Method 1): Rt = 0.88 min; MS (ESlpos): m/z = 461 [M+H]⁺
¹H-NMR (400 MHz, DMSO-d6) δ [ppm]: 1.137 (0.80), 1.223 (0.64), 1.242 (2.59), 1.260 (2.59), 1.390 (4.34), 1.408 (4.38), 2.332 (0.49), 2.518 (3.76), 2.522 (2.36), 2.819 (1.09), 2.838 (1.80), 2.854 (1.21), 3.159 (7.53), 3.171 (7.78), 3.670 (0.52), 3.696 (0.59), 3.720 (0.52), 3.748 (0.54), 3.771 (16.00), 3.972 (0.90), 3.993 (2.50), 4.001 (4.58), 4.010 (2.38), 4.031 (0.84), 4.086 (0.59), 4.100 (2.44), 4.118 (2.46), 4.135 (1.29), 4.948 (0.59), 4.969 (0.71), 4.986 (0.58), 6.182 (2.37), 6.186 (2.39), 6.544 (3.03), 6.717 (5.88), 6.868 (1.39), 6.889 (1.32), 7.070 (1.84), 7.289 (2.63), 7.293 (2.58), 7.759 (0.73), 8.034 (1.72), 8.040 (1.78), 8.614 (1.64), 8.619 (1.63).

### Example 668

### 2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 1)

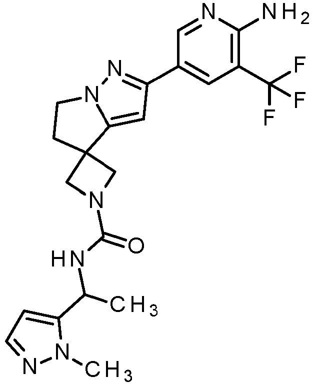

The title compound from **Example 667** was separated into enantiomers by preparative chiral HPLC to give 8.20 mg (100 % purity) of the title compound (enantiomer 1, Rt = 8.4 - 10.4 min).

### Preparative chiral HPLC method:

Instrument: Sepiatec Prep SFC100; Column: Chiralpak IA 5µ, 250x30; eluent A: CO2; eluent B: methanol + 0.2 % aqueous ammonia (32 %); isocratic: 20%B; flow: 100 mL/min; temperature: 40 °C; BPR: 150 bar UV: 254 nm

### Analytical chiral HPLC method:

Instrument: Agilent 1260, Aurora SFC-Modul; Column: Chiralpak IA 3µ, 100x4.6; eluent A: CO2; eluent B: methanol + 0.2 % aqueous ammonia (32 %); isocratic: 20%B; flow: 4 mL/min; temperature: 37.5 °C; BPR: 100 bar; UV: 254 nm
Analytical chiral HPLC: Rₜ = 2.49 min.
¹H-NMR (400 MHz, DMSO-d6) δ [ppm]: 1.390 (4.02), 1.407 (4.02), 2.518 (1.76), 2.523 (1.23), 2.819 (1.01), 2.836 (1.61), 2.854 (1.09), 2.994 (0.67), 3.720 (0.56), 3.771 (16.00), 3.972 (0.83), 3.993 (2.35), 4.001 (4.23), 4.011 (2.11), 4.032 (0.75), 4.100 (1.14), 4.118 (1.80), 4.135 (1.07), 4.948 (0.53), 4.967 (0.63), 4.986 (0.52), 6.182 (2.13), 6.186 (2.15), 6.544 (2.70), 6.717 (5.67), 6.871 (1.27), 6.892 (1.24), 7.289 (2.38), 7.294 (2.31), 8.034 (1.58), 8.040 (1.58), 8.614 (1.41), 8.618 (1.37).

### Example 669

### 2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 2)

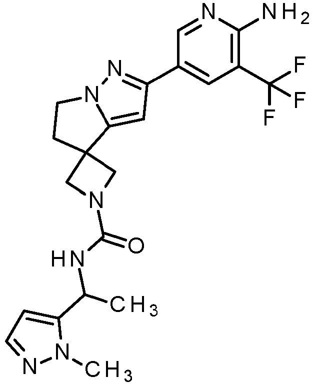

For the preparation of the racemate of the title compound see **Example 667.** Separation of enantiomers by preparative chiral HPLC (method see **Example 668)** to give 9.10 mg (100 % purity) of the title compound (enantiomer 2, Rₜ = 13.3 - 16.3 min).
Analytical chiral HPLC (method see **Example 668):** Rₜ = 3.91 min.
¹H-NMR (400 MHz, DMSO-d6) δ [ppm]: 1.391 (3.73), 1.408 (3.77), 2.084 (0.56), 2.518 (1.80), 2.523 (1.28), 2.820 (0.94), 2.837 (1.49), 2.855 (1.03), 2.994 (0.96), 3.671 (0.43), 3.771 (16.00), 3.972 (0.78), 3.993 (2.16), 4.001 (3.88), 4.011 (1.96), 4.032 (0.72), 4.101 (1.06), 4.119 (1.69), 4.136 (1.02), 4.948 (0.50), 4.968 (0.58), 4.986 (0.48), 6.183 (2.04), 6.186 (2.07), 6.546 (2.50), 6.718 (5.36), 6.868 (1.20), 6.890 (1.15), 7.289 (2.24), 7.294 (2.21), 8.035 (1.47), 8.040 (1.48), 8.615 (1.36), 8.619 (1.34).

### Example 670

### 2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 1 and enantiomer 2)

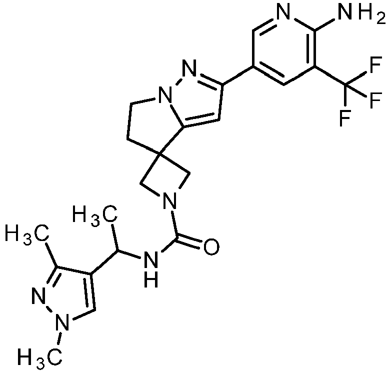

1-(1,3-Dimethyl-1H-pyrazol-4-yl)ethan-1-amine (33.2 mg, 239 µmol), CDI (42.2 mg, 260 µmol) and N,N-diisopropylethylamine (150 µL, 870 µmol) were dissolved in DMF and stirred for 1 h. A solution of 5-(5',6'-dihydrospiro[azetidine-3,4'-pyrrolo[1,2-b]pyrazol]-2'-yl)-3-(trifluoromethyl)pyridin-2-amine-hydrogen chloride (75.0 mg, 217 µmol) in DMF was added dropwise. The mixture was stirred over night at rt. The reaction mixture was diluted with water and lyophilised. The crude product was purified by column chromatography (silica gel, dichloromethane / methanol (0-15 %) gradient) to give 112 mg (90 % purity, 98 % yield) of the title compound.
LC-MS (Method 1): Rt = 0.87 min; MS (ESlpos): m/z = 475 [M+H]⁺
¹H-NMR (400 MHz, DMSO-d6) δ [ppm]: 1.237 (1.93), 1.241 (1.37), 1.256 (11.78), 1.273 (9.37), 1.307 (1.86), 1.324 (1.82), 2.039 (1.61), 2.067 (1.68), 2.089 (5.25), 2.518 (1.16), 2.523 (0.74), 2.810 (0.49), 2.827 (0.82), 2.845 (0.53), 3.098 (0.93), 3.117 (0.92), 3.164 (16.00), 3.336 (0.71), 3.571 (0.65), 3.604 (0.63), 3.674 (1.72), 3.682 (1.77), 3.695 (5.28), 3.941 (0.44), 3.962 (0.99), 3.972 (1.32), 3.975 (1.40), 3.983 (1.02), 4.003 (0.46), 4.100 (0.69), 4.117 (1.01), 4.135 (0.67), 6.534 (0.87), 6.542 (1.37), 6.554 (0.73), 6.703 (2.30), 7.052 (3.98), 7.055 (4.04), 7.411 (0.49), 7.416 (0.49), 7.449 (1.51), 7.728 (1.56), 8.035 (0.74), 8.040 (0.76), 8.615 (0.71), 8.619 (0.71).

### Example 671

### 2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 1)

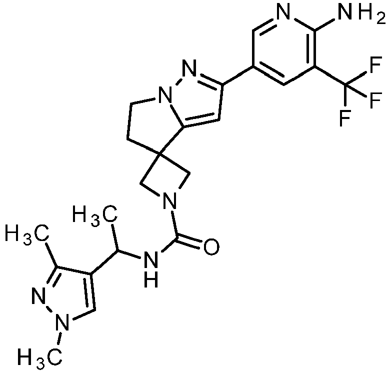

The title compound from **Example 670** was separated into enantiomers by preparative chiral HPLC to give 24.6 mg (100 % purity) of the title compound (enantiomer 1, Rt = 8.0 - 10.0 min)

### Preparative chiral HPLC method:

Instrument: Sepiatec Prep SFC100; Column: Reprosil Chiral NR 8µ, 250x30; eluent A: CO2; eluent B: methanol + 0.2 % aqueous ammonia (32 %); isocratic: 30%B; flow: 100 mL/min; temperature: 40°C; BPR: 150 bar UV: 254 nm

### Analytical chiral HPLC method:

Instrument: Agilent 1260, Aurora SFC-Modul; Column: Reprosil Chiral NR 5µ, 100x4.6; eluent A: CO2; eluent B: methanol + 0.2 % aqueous ammonia (32 %); isocratic: 30%B; flow: 4 mL/min; temperature: 37.5°C; BPR: 100 bar; UV: 254 nm
Analytical chiral HPLC: Rₜ = 2.67 min.
¹H-NMR (400 MHz, DMSO-d6) δ [ppm]: 1.250 (0.75), 1.269 (2.12), 1.287 (1.42), 1.307 (5.14), 1.324 (5.05), 2.066 (0.48), 2.088 (16.00), 2.366 (0.68), 2.518 (2.59), 2.523 (2.15), 2.709 (0.72), 2.810 (1.49), 2.827 (2.34), 2.844 (1.56), 2.994 (2.23), 3.109 (0.62), 3.127 (0.63), 3.146 (0.41), 3.385 (0.58), 3.519 (0.57), 3.563 (0.53), 3.580 (0.65), 3.597 (0.70), 3.613 (0.61), 3.629 (0.47), 3.681 (0.91), 3.941 (1.39), 3.951 (0.78), 3.961 (2.90), 3.971 (3.79), 3.975 (3.95), 3.982 (2.85), 3.996 (0.76), 4.003 (1.24), 4.099 (1.60), 4.117 (2.43), 4.134 (1.52), 4.686 (0.76), 4.706 (0.90), 4.724 (0.74), 6.543 (3.92), 6.559 (1.88), 6.702 (7.34), 7.450 (4.40), 7.556 (4.69), 8.035 (2.13), 8.040 (2.14), 8.614 (1.98), 8.618 (1.95), 8.804 (2.56).

### Example 672

### 2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 2)

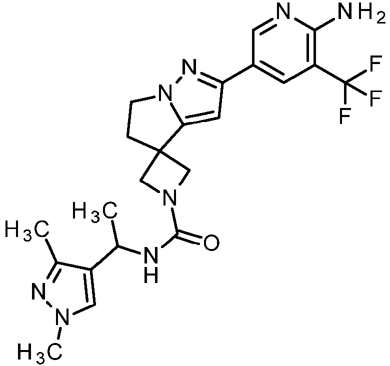

For the preparation of the racemate of the title compound see **Example 670.** The enantiomers were separated by preparative chiral HPLC (method see **Example 671)** to give 22.2 mg (100 % purity) of the title compound (enantiomer 2, Rₜ = 11.1 - 14.1 min).

Analytical chiral HPLC (method see **Example 671):** Rₜ = 3.69 min.

¹H-NMR (400 MHz, DMSO-d6) δ [ppm]: 1.244 (1.28), 1.261 (1.31), 1.308 (5.12), 1.325 (5.12), 2.067 (3.38), 2.084 (3.88), 2.090 (16.00), 2.518 (1.54), 2.523 (1.10), 2.810 (1.38), 2.828 (2.24), 2.845 (1.46), 3.683 (3.53), 3.696 (15.65), 3.942 (1.24), 3.951 (0.64), 3.963 (2.78), 3.971 (3.60), 3.975 (3.79), 3.983 (2.78), 3.996 (0.65), 4.004 (1.11), 4.100 (1.54), 4.118 (2.35), 4.135 (1.47), 4.688 (0.74), 4.708 (0.89), 4.726 (0.72), 6.528 (1.96), 6.546 (4.08), 6.704 (7.65), 7.415 (0.89), 7.447 (4.42), 8.036 (2.14), 8.041 (2.18), 8.616 (1.92), 8.620 (1.91).

### Example 673

### (3S)-2'-[6-amino-5-(trifluoromethyl)pyridin-3-yl]-N-[1-(1-methyl-1H-pyrazol-5-yl)ethyl]-5',6'-dihydro-1H-spiro[pyrrolidine-3,4'-pyrrolo[1,2-b]pyrazole]-1-carboxamide (diastereomer 1 and diastereomer 2)

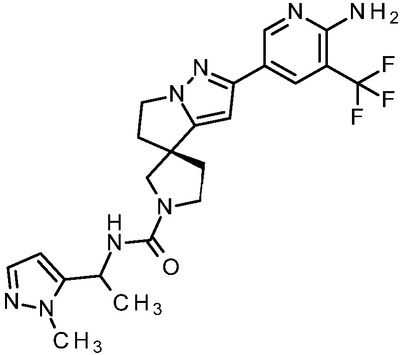

1-(1-Methyl-1H-pyrazol-5-yl)ethan-1-amine (25.0 mg, 200 µmol), CDI (35.3 mg, 218 µmol) and N,N-diisopropylethylamine (130 µL, 730 µmol) were dissolved in DMF and stirred for 1 h. A solution of 5-[(3S)-5',6'-dihydrospiro[pyrrolidine-3,4'-pyrrolo[1,2-b]pyrazol]-2'-yl]-3-(trifluoromethyl)pyridin-2-amine-hydrogen chloride (65.3 mg, 182 µmol) in DMF was added dropwise. The mixture was stirred over night at rt and concentrated. The residue was purified by preparative HPLC to give 25.0 mg (94 % purity, 27 % yield) of the title compound.
LC-MS (Method 1): Rt = 0.90 min; MS (ESlpos): m/z = 475 [M+H]⁺
¹H-NMR (400 MHz, DMSO-d6) δ [ppm]: 1.137 (0.54), 1.388 (4.64), 1.397 (5.02), 1.405 (4.99), 1.415 (4.59), 1.751 (0.86), 2.057 (2.35), 2.084 (3.58), 2.115 (0.41), 2.323 (0.87), 2.327 (1.18), 2.331 (0.88), 2.518 (8.85), 2.523 (7.23), 2.665 (0.88), 2.669 (1.20), 2.673 (0.88), 3.397 (1.65), 3.423 (4.12), 3.436 (3.00), 3.442 (3.06), 3.450 (1.25), 3.468 (1.24), 3.488 (0.42), 3.507 (0.78), 3.521 (0.70), 3.534 (0.95), 3.550 (0.61), 3.561 (0.52), 3.751 (15.77), 3.764 (16.00), 4.158 (2.52), 4.175 (4.69), 4.193 (2.41), 4.986 (1.19), 5.005 (1.58), 5.023 (1.20), 5.758 (4.29), 6.157 (2.23), 6.162 (2.29), 6.185 (2.26), 6.190 (2.32), 6.442 (3.40), 6.447 (5.96), 6.454 (1.55), 6.468 (1.49), 6.475 (1.47), 6.489 (1.30), 6.546 (6.54), 7.252 (2.42), 7.256 (2.44), 7.268 (2.60), 7.273 (2.57), 8.002 (3.55), 8.006 (3.58), 8.576 (3.52), 8.580 (3.47).

### Example 674

### (3S)-2'-[6-amino-5-(trifluoromethyl)pyridin-3-yl]-N-[1-(1,3-dimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)

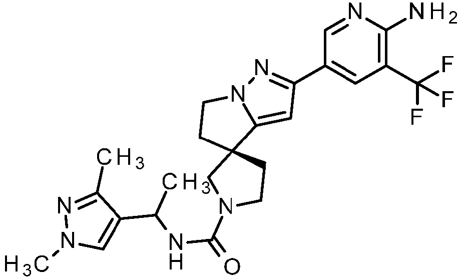

1-(1,3-Dimethyl-1H-pyrazol-4-yl)ethan-1-amine (31.9 mg, 229 µmol), CDI (40.6 mg, 250 µmol) and N,N-diisopropylethylamine (150 µL, 830 µmol) were dissolved in DMF and stirred for 1 h. A solution of 5-[(3S)-5',6'-dihydrospiro[pyrrolidine-3,4'-pyrrolo[1,2-b]pyrazol]-2'-yl]-3-(trifluoromethyl)pyridin-2-amine-hydrogen chloride (75.0 mg, 208 µmol) in DMF was added dropwise. The mixture was stirred over night at rt and concentrated. The residue was purified by preparative HPLC to give 18.8 mg (96 % purity, 18 % yield) of the title compound.
LC-MS (Method 1): Rt = 0.89 min; MS (ESlpos): m/z = 489 [M+H]⁺
¹H-NMR (400 MHz, DMSO-d6) δ [ppm]: 1.137 (0.69), 1.305 (2.69), 1.315 (2.91), 1.322 (2.97), 1.332 (2.60), 1.750 (0.52), 2.048 (1.41), 2.062 (8.20), 2.083 (8.34), 2.331 (1.17), 2.336 (0.53), 2.518 (8.71), 2.523 (5.83), 2.673 (1.15), 2.678 (0.53), 3.369 (0.98), 3.373 (0.92), 3.394 (1.76), 3.409 (0.58), 3.416 (0.44), 3.430 (1.98), 3.455 (0.99), 3.482 (0.43), 3.506 (0.40), 3.523 (0.47), 3.672 (7.53), 3.689 (7.86), 4.158 (1.38), 4.175 (2.72), 4.193 (1.34), 4.734 (0.54), 4.751 (0.81), 4.767 (0.53), 5.759 (16.00), 6.083 (0.83), 6.090 (0.83), 6.104 (0.83), 6.110 (0.78), 6.442 (2.88), 6.444 (4.28), 6.544 (3.48), 7.427 (2.05), 7.454 (2.16), 8.003 (1.87), 8.577 (1.96).

### Example 675

### (3R)-2'-{6-amino-5-[(1R)-1-(2,6-difluorophenyl)ethoxy]pyridin-3-yl}-N-(propan-2-yl)-5',6'-dihydrospiro[pyrrolidine-3,4'-pyrrolo[1,2-b]pyrazole]-1-carboxamide

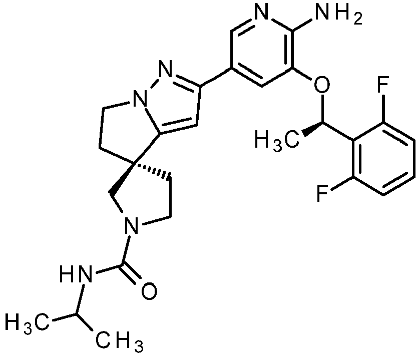

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (47.4 mg, 120 µmol), {6-amino-5-[(1R)-1-(2,6-difluorophenyl)ethoxy]pyridin-3-yl}boronic acid **(Intermediate 447,** 154 mg, 32 % purity, 168 µmol) and potassium phosphate (720 µL, 0.50 M, 360 µmol) were dissolved in degassed 1,4-dioxane (2.0 mL) under argon. XPhos Pd G2 (4.71 mg, 5.99 µmol) was added and the mixture was stirred for 2 h at 100 °C. XPhos Pd G2 (10 mg, 12.7 µmol) was added again and stirred for 3 h at 100 °C. The reaction mixture was concentrated. The residue was filtered over silica gel with dichloromethane / methanol 80:20. The filtrate was concentrated.

The residue was dissolved in 1,4-dioxane. XPhos Pd G2 (4.71 mg, 5.99 µmol), potassium phosphate (720 µL, 0.50 M, 360 µmol) and (3R)-1-[(propan-2-yl)carbamoyl]-5',6'-dihydrospiro[pyrrolidine-3,4'-pyrrolo[1,2-b]pyrazol]-2'-yl trifluoromethanesulfonate (20 mg, 50.6 µmol) were added and the mixture was stirred for 2 h at 100 °C. The reaction mixture was allowed to cool down, diluted with ethy acetate and filtered. The filtrate was washed with water and brine, dried over sodium sulfate, filtered and concentrated. The crude product was purified by preparative TLC (gradient: dichloromethane / methanol 9:1) to give 10.5 mg (95 % purity, 17 % yield) of the title compound.
LC-MS (Method 1): Rt = 1.01 min; MS (ESlpos): m/z = 498 [M+H]⁺
¹H-NMR (400 MHz, CHLOROFORM-d) δ [ppm]: 0.888 (0.47), 1.181 (14.12), 1.188 (15.39), 1.197 (16.00), 1.203 (12.91), 1.251 (5.07), 1.261 (1.95), 1.801 (13.12), 1.818 (12.98), 2.050 (0.72), 2.063 (0.96), 2.068 (0.98), 2.081 (1.80), 2.095 (1.59), 2.112 (1.02), 2.216 (1.03), 2.235 (2.10), 2.247 (1.00), 2.254 (1.28), 2.266 (1.48), 2.285 (0.81), 2.520 (0.76), 2.537 (1.67), 2.553 (1.83), 2.570 (2.92), 2.587 (1.46), 2.630 (3.20), 2.648 (1.80), 2.663 (1.68), 2.681 (0.75), 3.489 (1.84), 3.501 (1.02), 3.513 (4.31), 3.525 (1.61), 3.546 (5.99), 3.561 (1.20), 3.571 (2.35), 3.609 (0.98), 3.622 (1.15), 3.629 (1.37), 3.642 (1.21), 3.653 (0.79), 3.666 (0.59), 3.981 (1.09), 4.000 (4.27), 4.015 (2.43), 4.030 (1.71), 4.048 (0.84), 4.245 (4.71), 4.263 (9.28), 4.281 (4.52), 4.806 (5.82), 5.310 (6.09), 5.868 (0.86), 5.884 (2.73), 5.901 (2.68), 5.917 (0.84), 6.112 (15.20), 6.855 (0.58), 6.865 (3.94), 6.877 (0.89), 6.885 (8.38), 6.906 (4.48), 6.915 (0.70), 7.006 (0.74), 7.198 (0.88), 7.214 (1.86), 7.219 (1.50), 7.230 (1.20), 7.235 (3.17), 7.240 (1.08), 7.250 (1.66), 7.256 (1.85), 7.429 (5.47), 7.433 (5.43), 7.528 (0.72), 8.025 (6.98), 8.029 (6.97).

### Example 676

### (3R)-2'-(6-amino-5-{methyl[(1R)-1-phenylethyl]amino}pyridin-3-yl)-N-ethyl-5',6'-dihydrospiro[pyrrolidine-3,4'-pyrrolo[1,2-b]pyrazole]-1-carboxamide

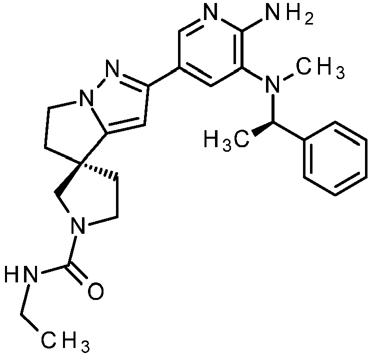

(3R)-1-(Ethylcarbamoyl)-5',6'-dihydrospiro[pyrrolidine-3,4'-pyrrolo[1,2-b]pyrazol]-2'-yl trifluoromethanesulfonate (44.2 mg, 116 µmol), (6-amino-5-{methyl[(1R)-1-phenylethyl]amino}pyridin-3-yl)boronic acid **(Intermediate 459,** 258 mg, 17 % purity, 162 µmol) and potassium phosphate (690 µL, 0.50 M, 350 µmol) were dissolved in degassed 1,4-dioxane (1.9 mL) under argon. XPhos Pd G2 (4.55 mg, 5.78 µmol) was added and the mixture was stirred for 2 h at 100 °C. The reaction mixture was allowed to cool down, diluted with ethy acetate and filtered. The filtrate was washed with water and brine, dried over sodium sulfate, filtered and concentrated. The residue was purified by flash column chromatography (silica gel, dichloromethane / methanol (0-20 %) gradient) to give 37.0 mg (98 % purity, 68 % yield) of the title compound.
LC-MS (Method 1): Rt = 1.05 min; MS (ESlpos): m/z = 461 [M+H]⁺
¹H-NMR (400 MHz, CHLOROFORM-d) δ [ppm]: 1.164 (5.54), 1.182 (12.13), 1.200 (5.69), 1.341 (7.63), 1.358 (7.66), 2.082 (0.52), 2.096 (0.89), 2.109 (0.77), 2.127 (0.53), 2.222 (0.54), 2.241 (1.01), 2.253 (0.54), 2.258 (0.69), 2.270 (0.69), 2.289 (0.40), 2.480 (16.00), 2.551 (0.89), 2.566 (1.01), 2.584 (1.38), 2.600 (0.77), 2.626 (0.82), 2.643 (1.57), 2.662 (0.96), 2.676 (0.95), 2.694 (0.42), 3.295 (0.76), 3.308 (1.01), 3.312 (2.49), 3.326 (2.61), 3.330 (2.48), 3.344 (2.50), 3.349 (0.94), 3.363 (0.74), 3.510 (1.01), 3.534 (2.25), 3.545 (0.87), 3.552 (0.75), 3.570 (3.78), 3.587 (0.66), 3.594 (1.32), 3.626 (0.53), 3.640 (0.67), 3.646 (0.77), 3.659 (0.64), 3.665 (0.53), 3.670 (0.44), 4.165 (0.62), 4.178 (1.11), 4.191 (0.59), 4.259 (2.52), 4.277 (4.55), 4.294 (2.47), 4.305 (1.68), 4.321 (1.57), 4.338 (0.46), 5.003 (3.58), 5.310 (14.30), 6.175 (8.80), 7.245 (0.40), 7.250 (0.67), 7.255 (0.53), 7.278 (0.93), 7.284 (1.39), 7.288 (0.87), 7.322 (1.29), 7.338 (1.22), 7.343 (3.83), 7.360 (7.49), 7.364 (5.01), 7.381 (1.18), 7.386 (0.75), 7.655 (3.20), 7.660 (3.21), 8.222 (3.97), 8.227 (3.86).

### Example 677

### 2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 1 and diastereomer 2)

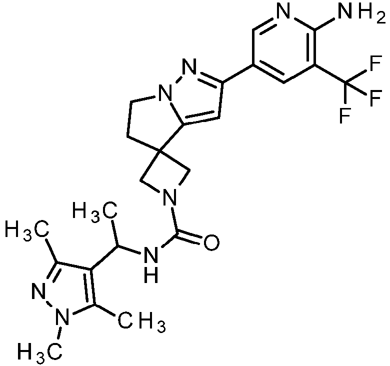

1-(1,3,5-Trimethyl-1H-pyrazol-4-yl)ethan-1-amine (36.6 mg, 239 µmol), CDI (42.2 mg, 260 µmol) and N,N-diisopropylethylamine (150 µL, 870 µmol) were dissolved in DMF and stirred for 1 h at rt. A solution of 5-(5',6'-dihydrospiro[azetidine-3,4'-pyrrolo[1,2-b]pyrazol]-2'-yl)-3-(trifluoromethyl)pyridin-2-amine-hydrogen chloride (75.0 mg, 217 µmol) in DMF was added dropwise. The mixture was stirred over night at rt. The reaction mixture was diluted with water and lyophilised. The residue was purified by flash column chromatography (silica gel, dichloromethane / methanol (0-15 %) gradient) to give 101 mg (95 % purity, 91 % yield) of the title compound.
LC-MS (Method 1): Rt = 0.90 min; MS (ESlpos): m/z = 489 [M+H]⁺
¹H-NMR (400 MHz, DMSO-d6) δ [ppm]: 1.066 (1.93), 1.216 (0.44), 1.226 (1.15), 1.234 (0.89), 1.245 (3.78), 1.249 (4.94), 1.265 (5.04), 1.328 (4.66), 1.346 (4.76), 2.071 (0.93), 2.095 (0.87), 2.126 (13.70), 2.153 (0.96), 2.198 (14.30), 2.518 (4.47), 2.523 (3.07), 2.801 (1.17), 2.819 (1.90), 2.836 (1.26), 3.119 (0.83), 3.138 (0.90), 3.165 (16.00), 3.233 (1.06), 3.549 (1.42), 3.573 (13.30), 3.589 (0.70), 3.606 (0.73), 3.622 (0.56), 3.930 (1.12), 3.941 (0.81), 3.950 (2.15), 3.961 (2.36), 3.970 (2.62), 3.976 (2.33), 3.991 (0.91), 3.996 (1.15), 4.097 (1.57), 4.114 (2.30), 4.131 (1.50), 4.643 (0.85), 4.660 (1.32), 4.678 (0.85), 6.540 (3.03), 6.574 (1.43), 6.592 (1.44), 6.688 (6.25), 7.121 (8.76), 7.122 (9.02), 7.870 (3.28), 8.029 (1.76), 8.035 (1.81), 8.608 (1.68), 8.613 (1.66).

### Example 678

### 2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 1)

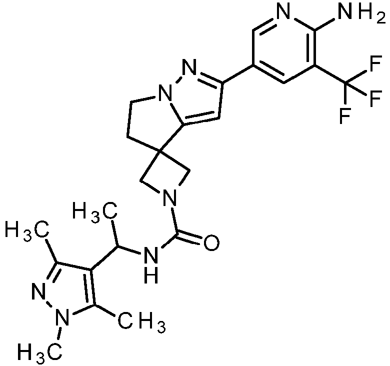

The title compound from Example 677 was separated into diastereomers by preparative chiral HPLC to give 35.0 mg (96 % purity) of the title compound (diastereomer 1, Rₜ = 6.5 - 9.1 min)

### Preparative chiral HPLC method:

Instrument: PrepCon Labomatic HPLC-2; Column: YMC Amylose SA 5µ, 250x30; eluent A: hexane + 0,1 vol % diethylamine; eluent B: ethanol + 0,1 vol % diethylamine; isocratic: 70%A+30%B; flow: 50 mL/min; temperature: 25 °C; UV: 254 nm

### Analytical chiral HPLC method:

Instrument: Thermo Fisher UltiMate 3000; Column: YMC Amylose SA 3µ, 100x4.6; eluent A: hexane + 0,1 vol % diethylamine; eluent B: ethanol; isocratic: 70%A+30%B; flow: 1,4 mL/min; temperature: 35 °C; UV: 254 nm
Analytical chiral HPLC: Rₜ = 2.67 min.
LC-MS (Method 1): Rt = 0.90 min; MS (ESlpos): m/z = 489 [M+H]⁺
¹H-NMR (400 MHz, DMSO-d6) δ [ppm]: 0.795 (0.60), 1.006 (0.48), 1.034 (0.51), 1.066 (0.96), 1.084 (1.17), 1.259 (1.87), 1.328 (5.52), 1.346 (5.62), 2.074 (0.46), 2.127 (15.22), 2.144 (0.47), 2.198 (16.00), 2.518 (3.49), 2.523 (2.40), 2.802 (1.37), 2.819 (2.21), 2.837 (1.45), 3.574 (14.85), 3.930 (1.30), 3.941 (1.00), 3.950 (2.52), 3.961 (2.72), 3.971 (3.09), 3.976 (2.69), 3.991 (0.95), 3.997 (1.19), 4.097 (1.50), 4.115 (2.33), 4.132 (1.47), 4.643 (1.00), 4.660 (1.56), 4.678 (0.99), 6.543 (3.63), 6.574 (1.70), 6.592 (1.60), 6.690 (7.55), 8.030 (2.10), 8.035 (2.13), 8.609 (1.97), 8.613 (1.92), 10.852 (0.77).

### Example 679

### 2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 2)

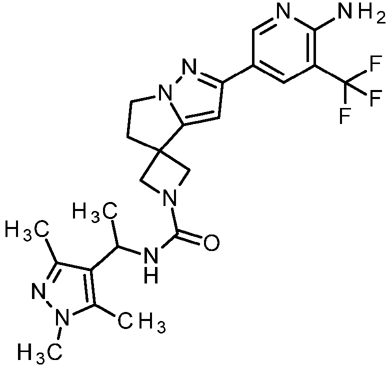

For the preparation of the diastereomeric mixture of the title compound see Example 677. The diastereomers were separated by preparative chiral HPLC (method see Example 678) to give 38.0 mg (100 % purity) of the title compound (diastereomer 2, Rt = 12.3 - 17.3 min).
Analytical chiral HPLC (method see Example 678): Rt = 5.63 min.
LC-MS (Method 1): Rt = 0.90 min; MS (ESlpos): m/z = 489 [M+H]⁺
¹H-NMR (400 MHz, DMSO-d6) δ [ppm]: 0.795 (0.74), 0.798 (0.62), 0.814 (0.76), 0.819 (0.41), 0.821 (0.65), 0.862 (0.42), 0.904 (0.72), 1.006 (0.58), 1.034 (0.62), 1.084 (1.37), 1.259 (2.02), 1.328 (5.48), 1.346 (5.51), 2.127 (15.19), 2.199 (16.00), 2.518 (2.77), 2.523 (1.90), 2.802 (1.34), 2.819 (2.17), 2.837 (1.43), 3.574 (14.71), 3.930 (1.27), 3.941 (0.89), 3.950 (2.46), 3.961 (2.66), 3.971 (3.04), 3.976 (2.63), 3.991 (0.93), 3.997 (1.17), 4.097 (1.47), 4.115 (2.26), 4.132 (1.41), 4.643 (0.99), 4.660 (1.53), 4.678 (0.97), 6.543 (3.48), 6.574 (1.67), 6.592 (1.58), 6.690 (7.69), 8.030 (2.06), 8.035 (2.09), 8.609 (1.91), 8.613 (1.89), 10.852 (0.84).

### Example 680

### (3R)-2'-{6-amino-5-[(1R)-1-(4-methylpyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide

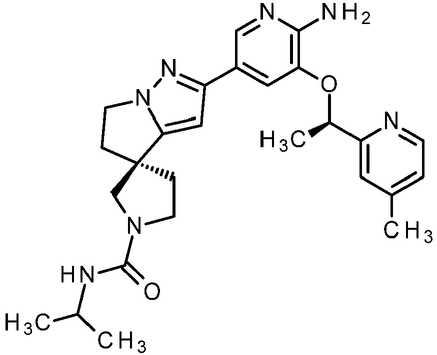

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (65.4 mg, 165 µmol), {6-amino-5-[(1R)-1-(4-methylpyridin-2-yl)ethoxy]pyridin-3-yl}boronic acid **(Intermediate 460,** 186 mg, 34 % purity, 231 µmol) and potassium phosphate (990 µL, 0.50 M, 500 µmol) were dissolved in degassed 1,4-dioxane (2.7 mL) under argon. XPhos Pd G2 (6.50 mg, 8.26 µmol) was added and the mixture was stirred for 2 h at 100 °C. The reaction mixture was allowed to cool down, diluted with ethy acetate and filtered. The filtrate was washed with water and brine, dried over sodium sufate, filtered and concentrated. The residue was purified by flash column chromatography (silica gel, dichloromethane / methanol (0-20 %) gradient) followed by preparative TLC (gradient: dichloromethane / methanol 9:1) to give 28.0 mg (95 % purity, 34 % yield) of the title compound.
LC-MS (Method 1): Rt = 0.93 min; MS (ESlpos): m/z = 477 [M+H]⁺
¹H-NMR (400 MHz, CHLOROFORM-d) δ [ppm]: 1.249 (7.43), 1.257 (8.83), 1.265 (9.73), 1.272 (7.10), 1.781 (8.80), 1.798 (8.90), 1.993 (0.72), 2.095 (0.55), 2.112 (0.72), 2.125 (1.18), 2.138 (0.97), 2.156 (0.70), 2.253 (0.67), 2.272 (1.38), 2.284 (0.68), 2.291 (0.85), 2.303 (1.00), 2.322 (0.51), 2.395 (16.00), 2.581 (0.84), 2.596 (1.09), 2.631 (0.85), 2.654 (0.86), 2.672 (1.56), 2.689 (1.13), 2.704 (0.94), 2.722 (0.45), 3.535 (0.90), 3.560 (2.75), 3.570 (1.61), 3.582 (3.93), 3.594 (1.56), 3.606 (1.41), 3.660 (0.61), 3.673 (0.78), 3.680 (0.93), 3.693 (0.82), 3.704 (0.54), 4.067 (2.49), 4.077 (2.02), 4.092 (1.05), 4.110 (0.51), 4.276 (2.62), 4.293 (4.73), 4.310 (2.49), 4.960 (4.79), 5.382 (5.54), 5.537 (0.57), 5.553 (1.85), 5.569 (1.83), 5.585 (0.55), 6.119 (0.94), 6.131 (7.38), 7.083 (1.95), 7.095 (1.92), 7.270 (3.38), 7.334 (0.56), 8.101 (3.50), 8.105 (3.63), 8.116 (0.54), 8.121 (0.47), 8.509 (2.63), 8.521 (2.58).

### Example 681

### (3R)-2'-{6-amino-5-[(1R)-1-(4-methylpyridin-2-yl)ethoxy]pyridin-3-yl}-N-cyclobutyl-5',6'-dihydrospiro[pyrrolidine-3,4'-pyrrolo[1,2-b]pyrazole]-1-carboxamide

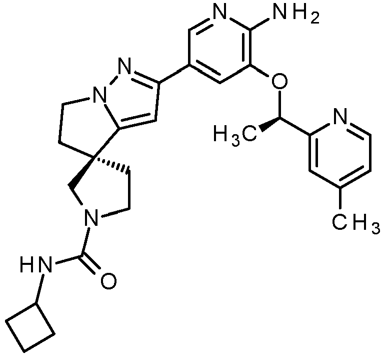

(3R)-1-(Cyclobutylcarbamoyl)-5',6'-dihydrospiro[pyrrolidine-3,4'-pyrrolo[1,2-b]pyrazol]-2'-yl trifluoromethanesulfonate (67.4 mg, 165 µmol), {6-amino-5-[(1R)-1-(4-methylpyridin-2-yl)ethoxy]pyridin-3-yl}boronic acid **(Intermediate 460,** 186 mg, 34 % purity, 231 µmol) and potassium phosphate (990 µL, 0.50 M, 500 µmol) were dissolved in degassed 1,4-dioxane (2.7 mL) under argon. XPhos Pd G2 (6.50 mg, 8.26 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated. The residue was purified by flash column chromatography (silica gel, dichloromethane / methanol (0-20 %) gradient) followed by preparative TLC (gradient: dichloromethane / methanol 9:1) to give 37.0 mg (95 % purity, 44 % yield) of the title compound.
LC-MS (Method 1): Rt = 0.96 min; MS (ESlpos): m/z = 489 [M+H]⁺
¹H-NMR (400 MHz, CHLOROFORM-d) δ [ppm]: 1.728 (0.75), 1.737 (1.09), 1.742 (0.61), 1.754 (1.83), 1.760 (1.50), 1.770 (1.40), 1.782 (10.80), 1.799 (9.48), 1.873 (0.77), 1.884 (0.93), 1.896 (1.03), 1.901 (1.15), 1.907 (1.13), 1.914 (1.08), 1.918 (1.00), 1.928 (0.92), 1.940 (0.75), 1.957 (0.49), 2.092 (0.42), 2.106 (0.56), 2.111 (0.58), 2.123 (1.03), 2.137 (0.83), 2.141 (0.76), 2.155 (0.61), 2.249 (0.59), 2.268 (1.25), 2.280 (0.64), 2.287 (0.76), 2.299 (0.90), 2.318 (0.46), 2.395 (16.00), 2.410 (0.59), 2.427 (1.12), 2.438 (1.40), 2.447 (1.24), 2.453 (1.22), 2.456 (1.23), 2.465 (0.99), 2.576 (0.79), 2.591 (1.05), 2.625 (0.83), 2.648 (0.84), 2.665 (1.43), 2.683 (1.07), 2.698 (0.93), 2.716 (0.45), 3.539 (0.74), 3.563 (2.41), 3.582 (4.16), 3.594 (0.89), 3.600 (1.36), 3.607 (1.25), 3.618 (0.60), 3.661 (0.56), 3.675 (0.71), 3.681 (0.83), 3.694 (0.73), 3.705 (0.47), 4.273 (2.47), 4.291 (4.43), 4.308 (2.34), 4.320 (0.48), 4.400 (0.82), 4.418 (2.68), 4.428 (1.52), 4.949 (4.40), 5.383 (13.07), 5.536 (0.53), 5.553 (1.80), 5.568 (1.80), 5.585 (0.52), 6.115 (0.97), 6.128 (8.45), 6.397 (0.68), 7.081 (1.56), 7.083 (1.68), 7.093 (1.61), 7.095 (1.64), 7.270 (3.03), 7.331 (0.40), 8.107 (3.68), 8.111 (3.63), 8.121 (0.49), 8.126 (0.46), 8.510 (2.45), 8.523 (2.40).

### Example 682

### (3R)-2'-{6-amino-5-[(1R)-1-(6-methylpyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide

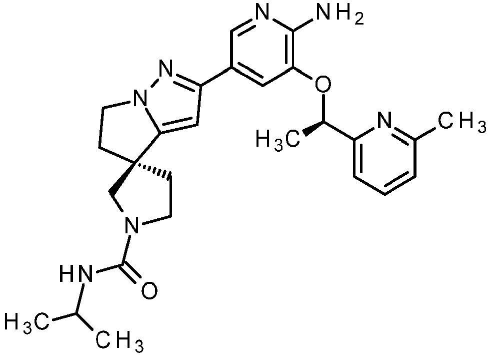

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (37.6 mg, 94.9 µmol), {6-amino-5-[(1R)-1-(6-methylpyridin-2-yl)ethoxy]pyridin-3-yl}boronic acid (**Intermediate** 461, 110 mg, 33 % purity, 133 µmol) and potassium phosphate (570 µL, 0.50 M, 280 µmol) were dissolved in degassed 1,4-dioxane (1.6 mL) under argon. XPhos Pd G2 (3.73 mg, 4.75 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated. The residue was purified by preparative TLC (gradient: dichloromethane / methanol 9:1) to give 21.1 mg (98 % purity, 46 % yield) of the title compound.
LC-MS (Method 1): Rt = 0.95 min; MS (ESlpos): m/z = 477 [M+H]⁺
¹H-NMR (400 MHz, CHLOROFORM-d) δ [ppm]: 1.173 (7.23), 1.182 (9.11), 1.188 (9.64), 1.197 (7.09), 1.248 (0.58), 1.258 (0.40), 1.712 (7.78), 1.728 (7.95), 1.843 (0.84), 2.022 (0.46), 2.040 (0.67), 2.053 (1.10), 2.067 (0.96), 2.084 (0.67), 2.172 (0.62), 2.191 (1.29), 2.209 (0.83), 2.222 (0.91), 2.241 (0.46), 2.491 (0.40), 2.508 (0.91), 2.524 (1.12), 2.541 (1.44), 2.558 (1.12), 2.574 (16.00), 2.600 (1.58), 2.633 (0.88), 2.651 (0.44), 3.462 (0.86), 3.487 (3.02), 3.504 (4.21), 3.525 (1.78), 3.542 (0.69), 3.583 (0.62), 3.602 (0.97), 3.615 (0.85), 3.991 (2.69), 4.001 (1.92), 4.017 (1.09), 4.034 (0.55), 4.199 (2.30), 4.217 (4.07), 4.235 (2.16), 4.882 (4.87), 5.307 (3.35), 5.458 (0.58), 5.474 (1.82), 5.490 (1.81), 5.507 (0.57), 6.057 (6.45), 7.034 (2.21), 7.053 (2.37), 7.176 (2.09), 7.195 (2.32), 7.317 (3.33), 7.321 (3.41), 7.523 (1.68), 7.542 (2.96), 7.561 (1.38), 8.026 (3.42), 8.030 (3.59).

### Example 683

### (3R)-2'-{6-amino-5-[(1R)-1-(6-methylpyridin-2-yl)ethoxy]pyridin-3-yl}-N-cyclobutyl-5',6'-dihydrospiro[pyrrolidine-3,4'-pyrrolo[1,2-b]pyrazole]-1-carboxamide

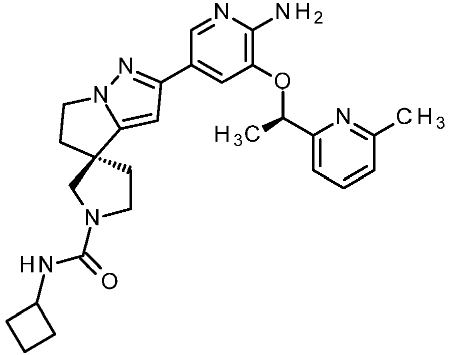

(3R)-1-(Cyclobutylcarbamoyl)-5',6'-dihydrospiro[pyrrolidine-3,4'-pyrrolo[1,2-b]pyrazol]-2'-yl trifluoromethanesulfonate (38.8 mg, 94.9 µmol), {6-amino-5-[(1R)-1-(6-methylpyridin-2-yl)ethoxy]pyridin-3-yl}boronic acid (**Intermediate** 461, 110 mg, 33 % purity, 133 µmol) and potassium phosphate (570 µL, 0.50 M, 280 µmol) were dissolved in degassed 1,4-dioxane (1.6 mL) under argon. XPhos Pd G2 (3.73 mg, 4.75 µmol) was added and the mixture was stirred for 2 h at 100 °C. The reaction mixture was allowed to cool down, diluted with ethyl acetate and filtered. The filtrate was washed with water and brine, dried over sodium sulfate, filtered and concentrated. The residue was purified by preparative TLC (gradient: dichloromethane / methanol 9:1) to give 24.7 mg (98 % purity, 52 % yield) of the title compound.
LC-MS (Method 1): Rt = 0.97 min; MS (ESlpos): m/z = 489 [M+H]⁺
¹H-NMR (400 MHz, CHLOROFORM-d) δ [ppm]: 1.651 (0.74), 1.660 (1.07), 1.677 (1.80), 1.684 (1.53), 1.693 (1.45), 1.705 (2.58), 1.712 (8.13), 1.727 (7.74), 1.808 (1.08), 1.823 (1.41), 1.836 (1.41), 2.019 (0.41), 2.036 (0.60), 2.050 (1.01), 2.064 (0.86), 2.081 (0.61), 2.167 (0.56), 2.186 (1.20), 2.198 (0.60), 2.204 (0.77), 2.217 (0.83), 2.235 (0.43), 2.351 (1.13), 2.361 (1.44), 2.370 (1.33), 2.375 (1.31), 2.387 (1.03), 2.501 (0.73), 2.517 (1.04), 2.535 (1.26), 2.551 (0.86), 2.573 (16.00), 2.592 (1.47), 2.643 (0.43), 3.463 (0.73), 3.488 (2.80), 3.504 (4.08), 3.528 (2.11), 3.545 (0.63), 3.582 (0.57), 3.596 (0.75), 3.601 (0.87), 3.615 (0.75), 4.195 (2.04), 4.214 (3.59), 4.231 (1.89), 4.324 (0.94), 4.340 (2.85), 4.882 (4.47), 5.306 (5.05), 5.458 (0.54), 5.473 (1.74), 5.490 (1.73), 5.506 (0.52), 6.054 (6.61), 7.034 (2.08), 7.053 (2.23), 7.176 (1.96), 7.195 (2.17), 7.315 (3.11), 7.319 (3.16), 7.522 (1.67), 7.541 (2.93), 7.561 (1.37), 8.026 (3.32), 8.031 (3.32).

### Example 684

### (3S)-2'-[6-amino-5-(trifluoromethyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide

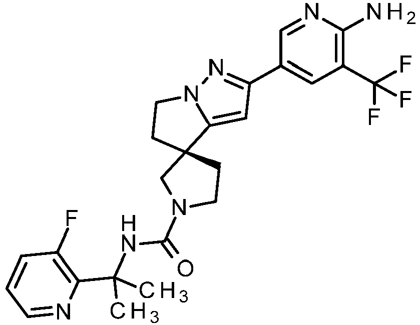

2-(3-Fluoropyridin-2-yl)propan-2-amine (35.4 mg, 229 µmol), CDI (40.6 mg, 250 µmol) and N,N-diisopropylethylamine (150 µL, 830 µmol) were dissolved in DMF and stirred for 1 h at rt. A solution of 5-[(3S)-5',6'-dihydrospiro[pyrrolidine-3,4'-pyrrolo[1,2-b]pyrazol]-2'-yl]-3-(trifluoromethyl)pyridin-2-amine-hydrogen chloride (75.0 mg, 208 µmol) in DMF was added dropwise. The mixture was stirred over night at rt, 2 h at 60 °C, 2 h at 80 °C and 3 h at 100 °C. The reaction mixture was allowed to coll down, filtered, and the concentrated filtrate was purified by preparative HPLC to give 23.0 mg (95 % purity, 21 % yield) of the title compound.
LC-MS (Method 1): Rt = 1.07 min; MS (ESlpos): m/z = 504 [M+H]⁺
¹H-NMR (400 MHz, CHLOROFORM-d) δ [ppm]: 1.696 (0.48), 1.712 (0.45), 1.854 (8.29), 1.858 (8.90), 1.863 (8.69), 1.867 (8.24), 2.106 (0.42), 2.110 (0.43), 2.123 (0.76), 2.137 (0.63), 2.155 (0.44), 2.247 (0.44), 2.266 (0.90), 2.278 (0.44), 2.285 (0.56), 2.297 (0.63), 2.581 (0.71), 2.597 (0.82), 2.631 (0.64), 2.678 (0.66), 2.696 (1.50), 2.713 (0.87), 2.729 (0.72), 2.746 (0.41), 3.580 (1.06), 3.605 (2.32), 3.629 (0.95), 3.641 (2.98), 3.654 (1.34), 3.666 (1.42), 3.672 (0.66), 3.722 (0.51), 3.736 (0.63), 3.742 (0.74), 3.747 (0.52), 3.755 (0.64), 3.760 (0.53), 3.767 (0.43), 4.272 (2.28), 4.290 (4.08), 4.307 (2.12), 4.994 (3.32), 6.236 (6.62), 6.985 (2.21), 7.235 (0.75), 7.243 (0.92), 7.246 (0.92), 7.255 (2.03), 7.260 (16.00), 7.266 (1.46), 7.276 (1.03), 7.384 (0.99), 7.387 (1.07), 7.404 (0.84), 7.407 (0.85), 7.413 (1.06), 7.416 (1.07), 7.433 (0.81), 7.436 (0.76), 8.108 (2.10), 8.112 (2.13), 8.307 (0.96), 8.310 (1.62), 8.314 (1.10), 8.318 (1.07), 8.322 (1.59), 8.326 (0.97), 8.602 (2.05), 8.606 (2.00).

### Example 685

### (3S)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)

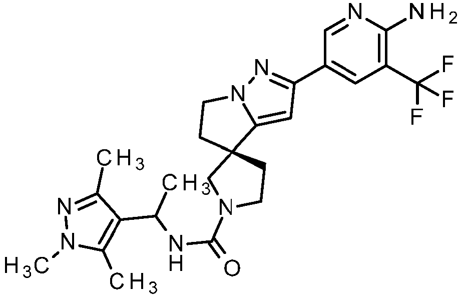

1-(1,3,5-Trimethyl-1H-pyrazol-4-yl)ethan-1-amine (35.1 mg, 229 µmol), CDI ((40.6 mg, 250 µmol) and N,N-diisopropylethylamine (150 µL, 830 µmol) were dissolved in DMF and stirred for 1 h at rt. A solution of 5-[(3S)-5',6'-dihydrospiro[pyrrolidine-3,4'-pyrrolo[1,2-b]pyrazol]-2'-yl]-3-(trifluoromethyl)pyridin-2-amine-hydrogen chloride (75.0 mg, 208 µmol) in DMF was added and the mixture was stirred over night at rt. The reaction mixture was filtered and the concentrated filtrate was purified by preparative HPLC followed by preparative TLC to give 6.10 mg (100 % purity, 6 % yield) of the title compound.
LC-MS (Method 1): Rt = 0.92 min; MS (ESlpos): m/z = 503 [M+H]⁺
¹H-NMR (400 MHz, CHLOROFORM-d) δ [ppm]: 0.075 (0.43), 0.868 (0.51), 0.886 (0.99), 0.901 (0.42), 1.240 (0.80), 1.259 (4.57), 1.290 (0.78), 1.316 (0.64), 1.489 (6.10), 1.496 (6.19), 1.506 (6.00), 1.513 (6.10), 1.991 (0.80), 2.029 (2.58), 2.062 (0.51), 2.076 (0.65), 2.080 (0.68), 2.093 (1.23), 2.097 (1.04), 2.107 (1.06), 2.111 (0.96), 2.125 (0.71), 2.179 (0.70), 2.207 (0.65), 2.214 (0.54), 2.227 (1.22), 2.233 (0.96), 2.240 (0.75), 2.245 (1.08), 2.259 (1.02), 2.267 (16.00), 2.274 (15.84), 2.293 (13.44), 2.303 (13.01), 2.547 (0.62), 2.556 (0.64), 2.563 (0.69), 2.572 (0.75), 2.580 (0.97), 2.589 (0.95), 2.597 (0.58), 2.628 (0.62), 2.639 (1.05), 2.645 (1.18), 2.658 (0.79), 2.663 (0.72), 2.673 (0.66), 2.678 (0.70), 3.478 (0.62), 3.501 (1.56), 3.525 (2.50), 3.532 (2.52), 3.540 (1.04), 3.546 (1.96), 3.557 (1.12), 3.571 (1.03), 3.609 (0.65), 3.623 (0.69), 3.630 (0.72), 3.643 (0.61), 3.654 (0.40), 3.679 (1.11), 3.687 (14.03), 3.696 (13.34), 4.252 (3.31), 4.270 (6.08), 4.287 (3.15), 4.368 (0.90), 4.375 (0.98), 4.383 (1.04), 4.391 (0.92), 4.894 (1.30), 4.911 (1.93), 4.929 (1.22), 5.024 (4.07), 6.186 (5.44), 6.189 (7.03), 8.098 (2.97), 8.103 (3.04), 8.587 (2.70), 8.592 (2.68).

### Example 686

### (3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)

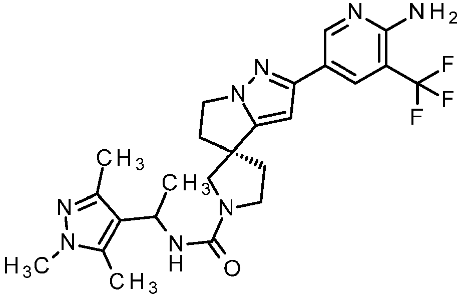

1-(1,3,5-Trimethyl-1H-pyrazol-4-yl)ethan-1-amine (37.5 mg, 245 µmol), CDI (43.3 mg, 267 µmol) and N,N-diisopropylethylamine (150 µL, 830 µmol) were dissolved in DMF and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine-hydrogen chloride (100 mg, 80 % purity, 222 µmol) in DMF was added dropwise and the mixture was stirred over night at rt. The reaction mixture was diluted with water and extracted 3 times with ethyl acetate. The combined organic layers were dried over a hydrophobic filter and concentrated. The residue was purified by column chromatography (silica gel, dichloromethane / methanol (0-20%) gradient) to give 111 mg (90 % purity, 89 % yield) of the title compound.
LC-MS (Method 1): Rt = 0.94 min; MS (ESlpos): m/z = 503 [M+H]⁺
¹H-NMR (400 MHz, DMSO-d6) δ [ppm]: 1.217 (1.35), 1.234 (1.72), 1.244 (1.50), 1.262 (1.39), 1.322 (4.88), 1.330 (4.98), 1.339 (4.99), 1.348 (4.45), 1.475 (2.94), 1.494 (2.92), 1.999 (0.43), 2.015 (1.36), 2.032 (1.81), 2.040 (1.96), 2.057 (0.95), 2.071 (3.78), 2.096 (3.70), 2.106 (11.61), 2.115 (2.01), 2.126 (13.74), 2.150 (7.92), 2.153 (4.47), 2.178 (12.15), 2.186 (2.29), 2.194 (12.55), 2.229 (8.11), 2.331 (1.21), 2.336 (0.60), 2.518 (8.38), 2.523 (5.93), 2.673 (1.19), 2.678 (0.56), 3.160 (4.59), 3.171 (4.71), 3.371 (2.92), 3.383 (3.04), 3.397 (3.00), 3.419 (2.47), 3.429 (2.05), 3.444 (1.12), 3.455 (1.16), 3.466 (0.68), 3.481 (0.77), 3.497 (0.73), 3.517 (0.80), 3.532 (0.65), 3.551 (13.50), 3.558 (3.20), 3.564 (12.41), 3.571 (4.26), 3.578 (7.64), 4.101 (0.81), 4.115 (1.02), 4.132 (0.66), 4.155 (2.17), 4.172 (4.00), 4.190 (2.06), 4.679 (1.41), 4.696 (1.98), 4.714 (1.37), 4.826 (0.46), 4.844 (0.57), 4.861 (0.46), 5.903 (0.42), 5.922 (0.45), 5.943 (0.53), 6.073 (1.61), 6.086 (1.45), 6.436 (3.08), 6.445 (5.39), 6.542 (4.93), 6.995 (1.39), 6.999 (1.72), 7.001 (1.64), 7.013 (15.85), 7.015 (16.00), 7.402 (0.41), 7.407 (0.42), 7.643 (8.17), 7.708 (1.04), 7.712 (1.68), 7.715 (1.04), 8.004 (2.90), 8.242 (1.06), 8.244 (1.55), 8.247 (1.00), 8.575 (3.13), 8.592 (0.55).

### Example 687

### (3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1)

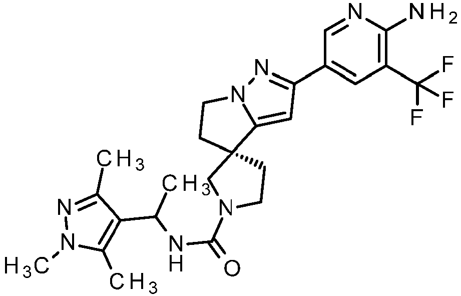

The title compound from **Example 686** was separated into diastereomers by preparative chiral HPLC to give 36.0 mg (97 % purity) of the title compound (diastereomer 1, Rt = 3.5 - 4.5 min)

### Preparative chiral HPLC method:

Instrument: Sepiatec Prep SFC100; Column: YMC Amylose SA 5µ, 250x30; eluent A: CO2; eluent B: 2-propanol + 0.4 vol % diethylamin; isocratic: 30%B; flow: 100 mL/min; temperature: 40 °C; BPR: 150 bar; UV: 280 nm

### Analytical chiral HPLC method:

Instrument: Agilent 1260, Aurora SFC-Modul; Column: YMC Amylose SA 3µ, 100x4.6; eluent A: CO2; eluent B: 2-propanol + 0.4 vol % diethylamin; isocratic: 30%B; flow: 4 mL/min; temperature: 37.5 °C; BPR: 100 bar; UV: 280 nm
Analytical chiral HPLC: Rₜ = 1.37 min.
LC-MS (Method 1): Rt = 0.94 min; MS (ESlpos): m/z = 503 [M+H]⁺
¹H-NMR (400 MHz, DMSO-d6) δ [ppm]: 0.846 (0.80), 0.853 (1.26), 0.859 (1.82), 0.861 (1.90), 0.864 (1.78), 0.871 (2.25), 0.887 (2.32), 0.906 (2.09), 0.925 (0.67), 1.277 (1.70), 1.286 (1.38), 1.330 (5.84), 1.348 (5.84), 1.368 (0.69), 1.387 (0.48), 1.409 (0.46), 2.016 (0.79), 2.031 (1.11), 2.039 (1.35), 2.057 (0.63), 2.106 (14.81), 2.178 (16.00), 2.518 (3.45), 2.523 (2.50), 2.994 (0.55), 3.358 (1.57), 3.363 (1.00), 3.371 (0.72), 3.383 (2.52), 3.407 (0.40), 3.429 (1.96), 3.455 (1.12), 3.518 (0.76), 3.526 (0.52), 3.532 (0.61), 3.552 (14.68), 3.669 (0.42), 3.695 (0.48), 3.702 (0.45), 4.155 (1.72), 4.165 (1.04), 4.172 (2.69), 4.190 (1.57), 4.248 (0.49), 4.260 (0.47), 4.678 (0.92), 4.696 (1.31), 4.714 (0.90), 6.073 (1.38), 6.090 (1.31), 6.445 (7.27), 6.540 (3.60), 7.832 (0.48), 7.852 (0.53), 8.004 (2.09), 8.009 (2.09), 8.213 (0.44), 8.233 (0.43), 8.255 (0.63), 8.258 (0.52), 8.577 (1.91), 8.581 (1.89).

### Example 688

### (3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 2)

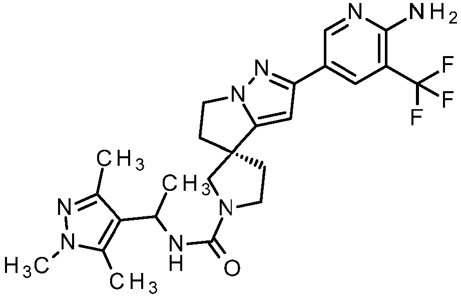

For the preparation of the diastereomeric mixture of the title compound see **Example** 686. The diastereomers were separated by preparative chiral HPLC (method see **Example** 687) to give 36.0 mg (95 % purity) of the title compound (diastereomer 2, Rₜ = 4.85 - 6.0 min).
Analytical chiral HPLC (method see **Example** 687): Rt = 2.32 min.
LC-MS (Method 1): Rt = 0.94 min; MS (ESlpos): m/z = 503 [M+H]⁺
¹H-NMR (400 MHz, DMSO-d6) δ [ppm]: 0.845 (1.20), 0.852 (1.91), 0.858 (2.74), 0.861 (2.85), 0.863 (2.67), 0.871 (3.31), 0.887 (3.51), 0.905 (3.11), 0.924 (1.01), 1.231 (0.55), 1.277 (2.52), 1.286 (2.07), 1.322 (6.01), 1.340 (6.07), 1.368 (0.96), 1.387 (0.70), 1.409 (0.67), 1.427 (0.44), 1.460 (0.44), 2.016 (0.68), 2.033 (1.04), 2.041 (1.35), 2.059 (0.60), 2.126 (14.44), 2.195 (16.00), 2.518 (2.73), 2.523 (2.29), 2.994 (0.68), 3.371 (0.92), 3.380 (0.43), 3.397 (2.62), 3.420 (2.55), 3.445 (1.04), 3.464 (0.43), 3.482 (0.73), 3.490 (0.44), 3.497 (0.53), 3.508 (0.42), 3.564 (14.44), 3.645 (0.50), 3.670 (0.64), 3.695 (0.69), 3.702 (0.63), 4.155 (1.84), 4.164 (1.04), 4.173 (3.02), 4.184 (1.09), 4.191 (1.72), 4.248 (0.73), 4.259 (0.69), 4.679 (0.92), 4.697 (1.26), 4.714 (0.90), 6.069 (1.33), 6.085 (1.28), 6.436 (3.86), 6.544 (3.52), 7.831 (0.74), 7.851 (0.80), 8.000 (2.01), 8.006 (2.01), 8.208 (0.61), 8.213 (0.67), 8.228 (0.45), 8.232 (0.65), 8.255 (0.94), 8.258 (0.78), 8.571 (1.84), 8.574 (1.79).

### Example 689

### (2'-{6-amino-5-[(1R)-1-phenylethoxy]pyridin-3-yl}-5',6'-dihydrospiro[azetidine-3,4'-pyrrolo[1,2-b]pyrazol]-1-yl)(cyclopropyl)methanone

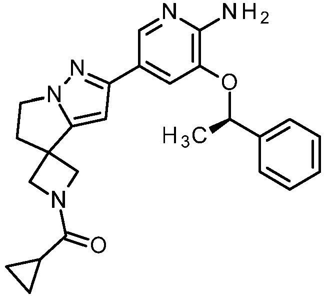

5-(5',6'-Dihydrospiro[azetidine-3,4'-pyrrolo[1,2-b]pyrazol]-2'-yl)-3-[(1R)-1-phenylethoxy]pyridin-2-amine-hydrogen chloride (**Intermediate 520,** 150 mg, 50 % purity, 188 µmol) and cyclopropanecarboxylic acid (16.2 mg, 188 µmol) were dissolved in DMF (0.62 mL). N,N-Diisopropylethylamine (200 µL, 1.1 mmol) and 2,4,6-tripropyl-1,3,5,2,4,6-trioxatriphosphinane-2,4,6-trioxide (380 µL, 50 % purity, 570 µmol) were added and the mixture was stirred for 1 h at rt.The reaction mixture was diluted with water and extracted with ethyl acetate. The combined organic layers were concentrated. The crude product was purified by preparative TLC and HPTLC (gradient: dichloromethane / methanol) to give 3.20 mg (90 % purity, 4 % yield) of the title compound.
LC-MS (Method 1): Rt = 1.03 min; MS (ESlpos): m/z = 430 [M+H]⁺
¹H-NMR (400 MHz, CHLOROFORM-d) δ [ppm]: 0.729 (1.28), 0.746 (1.36), 0.810 (0.45), 0.937 (0.45), 0.954 (1.15), 0.961 (1.18), 0.972 (0.54), 1.173 (10.06), 1.183 (2.68), 1.205 (0.51), 1.215 (0.42), 1.340 (0.40), 1.349 (0.49), 1.359 (0.70), 1.370 (0.43), 1.604 (6.35), 1.620 (6.41), 2.814 (1.48), 2.831 (2.02), 2.849 (1.57), 4.084 (0.48), 4.109 (0.76), 4.122 (1.21), 4.130 (0.57), 4.138 (2.09), 4.157 (1.14), 4.194 (0.54), 4.208 (0.55), 4.218 (0.41), 4.326 (0.53), 4.346 (0.72), 4.422 (0.52), 4.436 (0.58), 4.795 (2.34), 5.232 (3.61), 5.376 (1.13), 5.392 (1.12), 6.205 (1.13), 6.218 (1.12), 7.187 (2.03), 7.193 (16.00), 7.205 (1.15), 7.211 (0.49), 7.217 (0.55), 7.222 (1.06), 7.226 (0.64), 7.264 (1.05), 7.269 (0.57), 7.280 (1.00), 7.284 (2.78), 7.297 (0.55), 7.302 (2.38), 7.309 (2.64), 7.313 (3.12), 7.330 (1.09), 7.335 (0.73), 7.934 (2.56), 7.938 (2.54).

### Example 690

### 2'-[6-amino-5-(trifluoromethyl)pyridin-3-yl]-N-[1-(1-ethyl-1H-pyrazol-3-yl)ethyl]-5',6'-dihydro-1H-spiro[azetidine-3,4'-pyrrolo[1,2-b]pyrazole]-1-carboxamide (enantiomer 1)

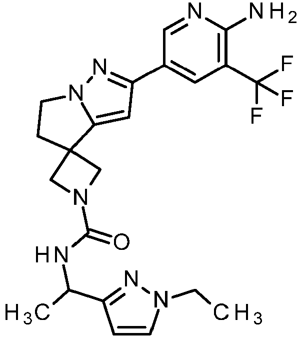

1-(1-Ethyl-1H-pyrazol-3-yl)ethan-1-amine (67 µL, 30 % purity, 160 µmol, CDI (28.1 mg, 174 µmol) and N,N-diisopropylethylamine (100 µL, 580 µmol) were dissolved in DMF and stirred for 1 h at rt. A solution of 5-(5',6'-dihydrospiro[azetidine-3,4'-pyrrolo[1,2-b]pyrazol]-2'-yl)-3-(trifluoromethyl)pyridin-2-amine-hydrogen chloride (50.0 mg, 145 µmol) in DMF was added dropwise. The reaction mixture was stirred over night at rt. CDI (11.7 mg, 72 µmol) and N,N-diisopropylethylamine (12.7 µL, 72.5 µmol) were added again and the mixture was stirred for 2 h at rt. The reaction mixture was diluted with water and extracted 3 times with ethyl acetate. The combined organic layers were washed with brine, dried over a hydrophobic filter and concentrated. The residue was purified by column chromatography (silica gel, dichloromethane / methanol (0-15%) gradient) and preparative TLC (gradient: dichloromethane / methanol (9:1)) to give 16.1 mg (60 % purity, 14 % yield) of the title compound.

The enantiomers were separated by preparative chiral HPLC to give 2.00 mg (90 % purity, 11 % yield) of the title compound (enantiomer 1, Rₜ = 7.8 - 8.8 min)

### Preparative chiral HPLC method:

Instrument: PrepCon Labomatic HPLC-4; Column: YMC Cellulose SC 5µ, 250x30; eluent A: methyl tert-butyl ether + 0.1 vol % diethylamin; eluent B: ethanol; isocratic: 80%A+20%B; flow: 65 mL/min; temperature: 25 °C; UV: 280 nm

### Analytical chiral HPLC method:

Instrument: Thermo Fisher UltiMate 3000; Column: YMC Cellulose SC 3µ, 100x4.6; eluent A: methyl tert-butyl ether + 0.1 vol % diethylamin; eluent B: ethanol; isocratic: 80%A+20%B; flow: 1.4 mL/min; temperature: 25 °C; UV: 280 nm
Analytical chiral HPLC: Rt = 4.39 min.
LC-MS (Method 1): Rt = 0.92 min; MS (ESlpos): m/z = 476 [M+H]⁺
¹H-NMR (400 MHz, CHLOROFORM-d) δ [ppm]: 0.759 (0.30), 0.774 (0.33), 0.783 (0.39), 0.793 (0.39), 0.800 (0.35), 0.811 (0.61), 0.827 (0.31), 1.124 (0.23), 1.141 (0.37), 1.159 (0.41), 1.185 (2.82), 1.215 (0.55), 1.231 (0.37), 1.264 (0.28), 1.288 (0.23), 1.306 (0.30), 1.324 (0.20), 1.354 (0.19), 1.358 (0.24), 1.368 (0.20), 1.379 (1.51), 1.387 (0.31), 1.397 (3.16), 1.404 (0.25), 1.416 (1.59), 1.444 (1.79), 1.461 (1.81), 2.825 (0.42), 2.842 (0.60), 2.860 (0.44), 3.424 (16.00), 4.031 (0.36), 4.049 (1.12), 4.053 (0.51), 4.058 (0.48), 4.067 (1.11), 4.073 (0.62), 4.078 (0.59), 4.085 (0.37), 4.138 (0.50), 4.156 (0.69), 4.165 (0.74), 4.169 (0.73), 4.173 (0.56), 4.184 (0.52), 4.188 (0.49), 4.861 (0.20), 4.879 (0.28), 4.950 (0.26), 4.967 (0.53), 4.974 (0.64), 4.984 (0.34), 6.068 (0.72), 6.074 (0.73), 6.355 (1.99), 7.251 (0.74), 7.256 (0.74), 8.044 (0.42), 8.048 (0.42), 8.531 (0.39), 8.535 (0.39).

### Example 691

### 2'-[6-amino-5-(trifluoromethyl)pyridin-3-yl]-N-[1-(1-ethyl-1H-pyrazol-3-yl)ethyl]-5',6'-dihydro-1H-spiro[azetidine-3,4'-pyrrolo[1,2-b]pyrazole]-1-carboxamide (enantiomer 2)

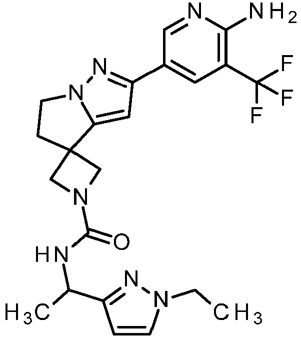

For the preparation of the racemic mixture of the title compound see **Example 690.** The enantiomers were separated by preparative chiral HPLC (method see **Example 690)** to give 2.00 mg (90 % purity, 11 % yield) of the title compound (enantiomer 2, Rₜ = 9.7 - 10.8 min).
Analytical chiral HPLC (method see **Example 690):** Rₜ = 5.71 min.
LC-MS (Method 1): Rt = 0.92 min; MS (ESpos): m/z = 476 [M+H]⁺
¹H-NMR (400 MHz, CHLOROFORM-d) δ [ppm]: 0.720 (0.40), 0.836 (1.30), 0.852 (1.37), 0.860 (1.64), 0.870 (1.75), 0.877 (1.52), 0.887 (2.73), 0.903 (1.33), 0.929 (0.59), 0.946 (0.56), 1.081 (0.56), 1.098 (0.64), 1.106 (0.64), 1.218 (1.43), 1.262 (11.97), 1.292 (2.40), 1.308 (1.59), 1.340 (1.23), 1.369 (0.97), 1.387 (1.36), 1.405 (0.87), 1.431 (0.89), 1.435 (1.11), 1.455 (5.04), 1.474 (10.57), 1.492 (5.50), 1.521 (6.46), 1.538 (6.61), 1.610 (0.65), 1.649 (0.66), 1.665 (0.63), 2.356 (0.43), 2.902 (1.65), 2.919 (2.34), 2.937 (1.71), 3.501 (16.00), 4.108 (1.35), 4.126 (4.27), 4.130 (1.97), 4.135 (1.81), 4.144 (4.30), 4.150 (2.44), 4.155 (2.28), 4.162 (1.39), 4.215 (2.01), 4.224 (0.59), 4.233 (2.79), 4.241 (3.02), 4.245 (2.80), 4.250 (2.32), 4.260 (2.07), 4.264 (1.95), 4.943 (0.77), 4.961 (1.11), 5.026 (0.92), 5.043 (1.11), 5.061 (0.80), 5.087 (1.82), 6.146 (2.70), 6.152 (2.66), 6.431 (7.13), 6.445 (0.85), 7.328 (2.76), 7.333 (2.68), 7.528 (0.41), 8.124 (1.81), 8.129 (1.97), 8.603 (1.49), 8.608 (1.58).

### Example 692

### (3S)-2'-[6-amino-5-(trifluoromethyl)pyridin-3-yl]-N-[1-(1-ethyl-1H-pyrazol-3-yl)ethyl]-5',6'-dihydro-1H-spiro[pyrrolidine-3,4'-pyrrolo[1,2-b]pyrazole]-1-carboxamide (diastereomer 1)

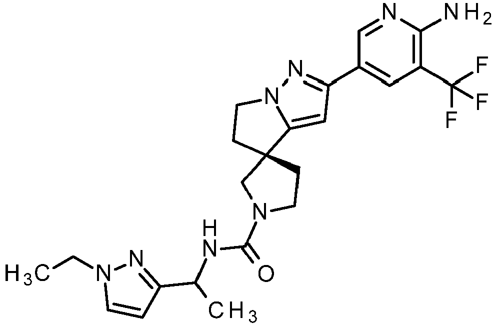

1-(1-Ethyl-1H-pyrazol-3-yl)ethan-1-amine (64 µL, 30 % purity, 150 µmol), CDI (27.0 mg, 167 µmol) and N,N-diisopropylethylamine (97 µL, 560 µmol) were dissolved in DMF and stirred for 1 h at rt. A solution of 5-[(3S)-5',6'-dihydrospiro[pyrrolidine-3,4'-pyrrolo[1,2-b]pyrazol]-2'-yl]-3-(trifluoromethyl)pyridin-2-amine-hydrogen chloride (50.0 mg, 139 µmol) in DMF was added dropwise. The mixture was stirred over night at rt. CDI (11.2 mg, 69 µmol) was added again and the mixture was stirred for 2 h at rt. The reaction mixture was diluted with water and extracted 3 times with ethyl acetate. The combined organic layers were washed with brine, dried over a hydrophobic filter and concentrated. The residue was purified by column chromatography (silica gel, dichloromethane / methanol (0-15%) gradient) to give 43.0 mg (73 % purity, 46 % yield) of the title compound as a diastereomeric mixture.

The diastereomers were separated by preparative chiral HPLC to give 4.00 mg (95 % purity, 9 % yield) of the title compound (diastereomer 1, Rt = 8.0 - 9.0 min)

### Preparative chiral HPLC method:

Instrument: PrepCon Labomatic HPLC-4; Column: YMC Amylose SA 5µ, 250x30; eluent A: methyl tert-butyl ether + 0.1 vol % diethylamin; eluent B: ethanol; isocratic: 80%A+20%B; flow: 50 mL/min; temperature: 25 °C; UV: 280 nm

### Analytical chiral HPLC method:

Instrument: Thermo Fisher UltiMate 3000; Column: YMC Amylose SA 3µ, 100x4.6; eluent A: methyl tert-butyl ether + 0.1 vol % diethylamin; eluent B: ethanol; isocratic: 80%A+20%B; flow: 1.4 mL/min; temperature: 25 °C; UV: 280 nm
Analytical chiral HPLC: Rt = 3.65 min.
LC-MS (Method 1): Rt = 0.95 min; MS (ESlpos): m/z = 490 [M+H]⁺
¹H-NMR (400 MHz, CHLOROFORM-d) δ [ppm]: 0.759 (0.53), 0.774 (0.56), 0.783 (0.61), 0.793 (0.62), 0.800 (0.57), 0.811 (0.96), 0.827 (0.51), 1.185 (3.79), 1.206 (1.32), 1.213 (1.42), 1.231 (1.07), 1.354 (0.43), 1.363 (0.56), 1.380 (7.88), 1.397 (16.00), 1.416 (8.28), 1.448 (7.18), 1.464 (7.40), 1.942 (0.62), 1.998 (0.48), 2.015 (0.69), 2.029 (1.06), 2.043 (0.91), 2.061 (0.63), 2.144 (0.66), 2.163 (1.24), 2.175 (0.66), 2.181 (0.87), 2.193 (0.86), 2.212 (0.54), 2.470 (0.51), 2.487 (1.18), 2.502 (1.17), 2.520 (1.67), 2.537 (0.95), 2.578 (0.93), 2.596 (1.81), 2.629 (1.23), 2.647 (0.55), 3.474 (1.09), 3.481 (0.62), 3.498 (2.97), 3.524 (1.50), 3.535 (3.31), 3.559 (1.55), 3.586 (0.59), 3.606 (0.84), 3.620 (0.71), 4.031 (2.24), 4.050 (6.78), 4.068 (6.70), 4.087 (2.15), 4.183 (3.04), 4.201 (5.71), 4.218 (2.97), 4.935 (4.18), 4.963 (0.75), 4.984 (3.03), 4.999 (1.76), 5.016 (0.87), 6.067 (5.05), 6.072 (5.08), 6.136 (9.98), 7.250 (4.49), 7.255 (4.54), 8.028 (2.81), 8.033 (2.80), 8.520 (2.72), 8.524 (2.71).

### Example 693

### (3S)-2'-[6-amino-5-(trifluoromethyl)pyridin-3-yl]-N-[1-(1-ethyl-1H-pyrazol-3-yl)ethyl]-5',6'-dihydro-1H-spiro[pyrrolidine-3,4'-pyrrolo[1,2-b]pyrazole]-1-carboxamide (diastereomer 2)

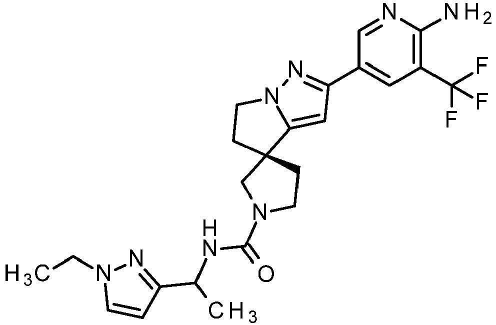

For the preparation of the diastereomeric mixture of the title compound see Example 692. The diastereomer were separated by chiral HPLC (method see Example 692) to give 4.00 mg (95 % purity, 9 % yield) of the title compound (diastereomer 2, Rt = 11.3 - 12.5 min).
Analytical chiral HPLC (method see Example 692): Rt = 5.02 min.
LC-MS (Method 1): Rt = 0.95 min; MS (ESlpos): m/z = 490 [M+H]⁺
¹H-NMR (400 MHz, CHLOROFORM-d) δ [ppm]: 0.759 (0.65), 0.775 (0.68), 0.784 (0.76), 0.793 (0.80), 0.801 (0.73), 0.811 (1.23), 0.827 (0.63), 1.186 (4.70), 1.206 (1.51), 1.225 (1.21), 1.354 (0.62), 1.364 (1.00), 1.368 (7.73), 1.387 (16.00), 1.405 (7.51), 1.456 (5.51), 1.472 (5.61), 1.609 (0.48), 1.943 (0.58), 1.993 (0.45), 2.012 (0.71), 2.025 (1.04), 2.039 (0.86), 2.057 (0.60), 2.147 (0.61), 2.166 (1.14), 2.178 (0.60), 2.184 (0.75), 2.197 (0.79), 2.216 (0.44), 2.467 (0.44), 2.485 (0.97), 2.500 (1.02), 2.518 (1.58), 2.535 (0.81), 2.570 (0.89), 2.587 (1.82), 2.638 (0.48), 3.476 (0.91), 3.501 (2.64), 3.522 (3.22), 3.532 (1.40), 3.547 (1.29), 3.588 (0.58), 3.602 (0.74), 3.607 (0.85), 3.621 (0.72), 3.632 (0.48), 4.020 (2.08), 4.038 (6.29), 4.057 (6.23), 4.075 (1.98), 4.184 (2.71), 4.201 (5.35), 4.219 (2.60), 4.934 (3.97), 4.970 (1.03), 4.982 (3.34), 4.993 (1.91), 5.010 (0.70), 6.058 (4.62), 6.064 (4.81), 6.134 (9.60), 7.238 (4.36), 7.244 (4.47), 8.031 (2.65), 8.035 (2.70), 8.522 (2.55), 8.526 (2.53).

### Example 694

### 5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

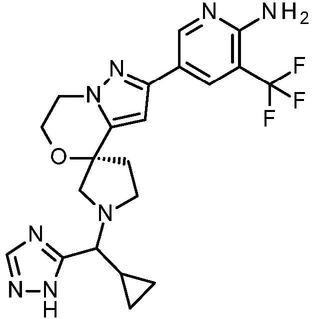

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (150 mg, 95 % purity, 379 µmol) and cyclopropyl(1H-1,2,4-triazol-5-yl)methanone (78.0 mg, 569 µmol) were dissolved in methanol (4 mL). N,N-Diisopropylethylamine (260 µL, 1.5 mmol) and titanium(IV) isopropoxide (340 µL, 1.1 mmol) were added and the mixture was stirred for 1.5 h at 60 °C. To the cold reaction mixture was added sodium cyanoborohydride (71.5 mg, 1.14 mmol) and the mixture was stirred over night at 60 °C. The reaction mixture was allowed to cool down, diluted with water (0.5 mL) and stirred for 15 min. The suspension was filtered and the filter cake was washed with methanol. The filtrate was concentrated. The crude product was purified by preparative HPLC to give 45.7 mg (100 % purity, 26 % yield) of the title compound.
LC-MS (Method 2): Rt = 0.67 min; MS (ESlpos): m/z = 461 [M+H]⁺
¹H-NMR (400 MHz, DMSO-d₆) δ [ppm]: 0.087 (0.93), 0.355 (1.35), 0.428 (0.85), 0.679 (0.87), 1.256 (0.92), 2.133 (1.21), 2.178 (1.10), 2.318 (1.07), 2.323 (2.40), 2.327 (3.49), 2.331 (2.50), 2.336 (1.15), 2.518 (16.00), 2.523 (10.65), 2.659 (2.26), 2.665 (3.69), 2.669 (4.76), 2.673 (3.63), 2.739 (1.49), 2.765 (1.61), 2.844 (2.22), 2.864 (2.45), 2.887 (1.44), 2.987 (0.94), 3.190 (0.76), 3.217 (0.93), 4.020 (2.65), 4.065 (4.28), 6.577 (7.11), 6.644 (1.61), 6.663 (1.04), 7.832 (1.28), 8.026 (3.98), 8.031 (6.41), 8.139 (7.05), 8.444 (0.71), 8.610 (4.13), 13.742 (0.58), 13.894 (1.38).

### Example 695

### 5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)

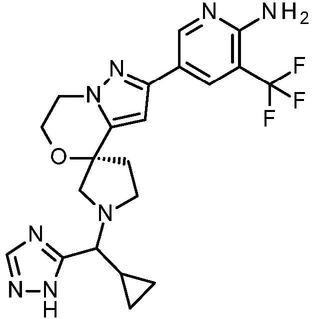

The title compound from Example 694 was separated into diastereomers by preparative chiral HPLC to give 17.9 mg (99 % purity, 40 % yield) of the title compound (diastereomer 1 Rₜ = 11.8 - 13.5 min).

### Preparative chiral HPLC method:

Instrument: Sepiatec: Prep SFC100; Column: Chiral Art Cellulose SB 5µ, 250x50; eluent A: CO2; eluent B: 2-propanol + 0.4 vol % diethylamine; isocratic: 15%B; flow: 100 mL/min; temperature: 40 °C; BPR: 150 bar; UV: 267 nm

### Analytical chiral HPLC method:

Instrument: Waters Acquity UPC2 QDA; Column: Chiral Art Cellulose SB 3µ, 100x4.6; eluent A: CO2; eluent B: 2-propanol + 0.4 vol % diethylamine; isocratic: 15%B; flow: 4 mL/min; temperature: 40 °C; BPR: 1800 psi; UV: 254 nm
Analytical chiral HPLC: Rt = 3.00 min
[α]²⁰_{D}: +102.1° (c = 1.00, methanol)
LC-MS (Method 1): Rt = 0.81 min; MS (ESlpos): m/z = 461 [M+H]⁺
¹H NMR (400 MHz, DMSO-d₆) δ ppm 0.01 - 0.12 (m, 1 H), 0.30 - 0.47 (m, 2 H), 0.59 - 0.72 (m, 1 H), 1.20 - 1.34 (m, 1 H), 2.04 - 2.23 (m, 2 H), 2.61 - 2.69 (m, 1 H), 2.83 - 3.03 (m, 4 H), 3.92 - 4.13 (m, 4 H), 6.52 - 6.70 (m, 3 H), 7.74 - 7.97 (m, 1 H), 8.03 (d, 1 H), 8.61 (d, 1 H), 13.65 - 13.97 (m, 1 H).

### Example 696

### 5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)

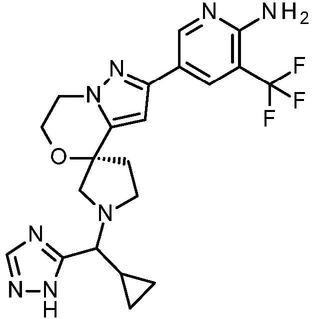

For the preparation of the diastereomeric mixture of the title compound see Example 694. The diastereomers were separated by preparative chiral HPLC (method see Example 695) to give 8.50 mg (99 % purity, 19 % yield) of the title compound (diastereomer 2 Rt = 14.2 - 16.6 min).
Analytical chiral HPLC (method see Example 695): Rₜ = 3.74 min.
[α]²⁰_{D}: +37.3° (c = 1.00, methanol)
LC-MS (Method 1): Rt = 0.83 min; MS (ESlpos): m/z = 461 [M+H]⁺
¹H NMR (400 MHz, DMSO-d6) δ ppm 0.01 - 0.12 (m, 1 H), 0.30 - 0.47 (m, 2 H), 0.59 - 0.72 (m, 1 H), 1.20 - 1.34 (m, 1 H), 2.04 - 2.23 (m, 2 H), 2.61 - 2.69 (m, 1 H), 2.83 - 3.03 (m, 4 H), 3.92 - 4.13 (m, 4 H), 6.52 - 6.70 (m, 3 H), 7.74 - 7.97 (m, 1 H), 8.03 (d, 1 H), 8.61 (d, 1 H), 13.65 - 13.97 (m, 1 H).

### Example 697

### 5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

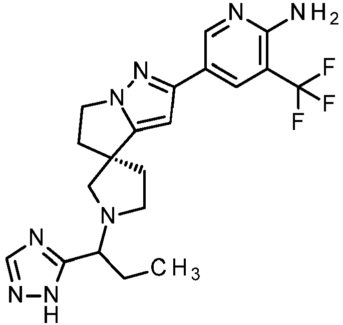

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (50.0 mg, 95 % purity, 132 µmol) and 1-(1H-1,2,4-triazol-5-yl)propan-1-one (24.8 mg, 198 µmol) were dissolved in methanol (1.4 mL). N,N-Diisopropylethylamine (92 µL, 530 µmol) and titanium(IV) isopropoxide (120 µL, 400 µmol) were added and the mixture was stirred for 3 h at 60 °C. To the cold reaction mixture was added sodium cyanoborohydride (24.9 mg, 396 µmol) and the mixture was stirred for 2.5 h at 60 °C. The reaction mixture was allowed to cool down, diluted with water (0.5 mL) and stirred for 15 min. The suspension was filtered and the filter cake was washed with methanol. The filtrate was concentrated. The crude product was purified by preparative HPLC to give 18.0 mg (96 % purity, 30 % yield) of the title compound.
LC-MS (Method 1): Rt = 0.81 min; MS (ESlpos): m/z = 433 [M+H]⁺
¹H-NMR (400 MHz, DMSO-d6) δ [ppm]: 0.755 (4.12), 0.759 (3.97), 0.773 (9.47), 0.777 (8.34), 0.792 (4.58), 0.932 (0.85), 0.948 (0.80), 1.794 (0.44), 1.812 (0.80), 1.827 (1.86), 1.847 (2.72), 1.866 (2.74), 1.877 (2.25), 1.893 (3.47), 1.909 (4.50), 1.927 (2.24), 2.318 (1.12), 2.323 (2.39), 2.327 (3.31), 2.331 (2.39), 2.336 (1.10), 2.378 (0.46), 2.396 (1.04), 2.411 (1.26), 2.428 (2.01), 2.439 (1.77), 2.446 (1.46), 2.458 (2.22), 2.518 (16.00), 2.523 (10.61), 2.605 (2.18), 2.627 (2.68), 2.634 (1.57), 2.660 (2.81), 2.665 (4.41), 2.673 (8.28), 2.699 (0.70), 2.766 (0.63), 2.787 (1.27), 2.802 (1.21), 2.813 (2.63), 2.835 (2.03), 2.849 (0.59), 2.865 (0.96), 2.888 (0.94), 2.905 (0.47), 3.165 (0.81), 3.681 (1.62), 3.699 (2.65), 3.718 (1.48), 4.057 (1.74), 4.066 (2.82), 4.075 (3.51), 4.084 (5.04), 4.101 (2.31), 6.291 (6.60), 6.329 (8.70), 6.513 (9.71), 7.992 (5.36), 8.104 (2.51), 8.552 (1.29), 8.565 (5.05).

### Example 698

### 5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)

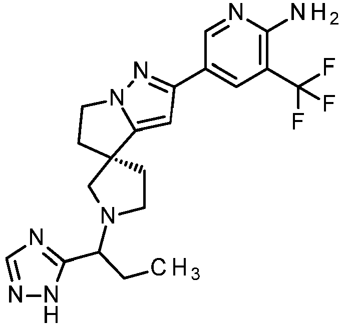

The title compound from **Example 697** was separated into diastereomers by preparative chiral HPLC to give 17.7 mg (98 % purity, 30 % yield) of the title compound (diastereomer 1 Rₜ = 9.44 - 12.39 min).

### Preparative chiral HPLC method:

Instrument: PrepCon Labomatic HPLC-1; Column: YMC Amylose SA 10µ, 250x50; eluent A: hexane + 0.1 vol % diethylamine; eluent B: ethanol; isocratic: 20%B; flow: 140 mL/min; temperature: 25 °C; UV: 280 nm

### Analytical chiral HPLC method:

Instrument: Thermo Fisher UltiMate 3000; Column: YMC Amylose SA 3µ, 100x4.6; eluent A: hexane + 0.1 vol % diethylamine; eluent B: ethanol; isocratic: 20%B; flow: 1.4 mL/min; temperature: 25 °C; UV: 280 nm
Analytical chiral HPLC: Rₜ = 2.01 min
LC-MS (Method 2): Rt = 0.65 min; MS (ESlpos): m/z = 433 [M+H]⁺
¹H-NMR (400 MHz, DMSO-d6) δ [ppm]: 0.756 (7.34), 0.774 (16.00), 0.793 (7.99), 0.814 (1.27), 0.821 (0.98), 0.836 (0.75), 0.862 (0.70), 0.886 (0.43), 0.904 (0.65), 1.005 (0.87), 1.034 (0.76), 1.071 (0.44), 1.084 (1.82), 1.204 (0.47), 1.232 (1.90), 1.259 (2.61), 1.352 (14.18), 1.392 (0.41), 1.741 (0.55), 1.757 (1.55), 1.774 (0.74), 1.832 (2.40), 1.850 (3.99), 1.868 (4.16), 1.884 (3.74), 1.903 (4.35), 1.917 (3.75), 2.181 (2.03), 2.322 (2.72), 2.326 (3.56), 2.331 (2.63), 2.404 (1.66), 2.419 (2.30), 2.435 (3.38), 2.454 (3.46), 2.522 (15.61), 2.602 (2.59), 2.624 (2.95), 2.664 (4.79), 2.669 (5.18), 2.673 (4.26), 2.764 (1.36), 2.786 (2.66), 2.810 (4.96), 2.833 (3.47), 3.565 (0.97), 3.582 (0.55), 3.599 (1.20), 3.615 (0.53), 3.690 (1.66), 3.707 (2.74), 3.725 (1.61), 4.070 (5.22), 4.087 (10.43), 4.104 (5.00), 6.338 (8.74), 6.512 (13.19), 6.652 (0.57), 6.867 (1.26), 7.992 (7.78), 7.997 (7.72), 8.567 (7.27), 8.570 (7.11), 13.808 (2.04).

### Example 699

### 5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)

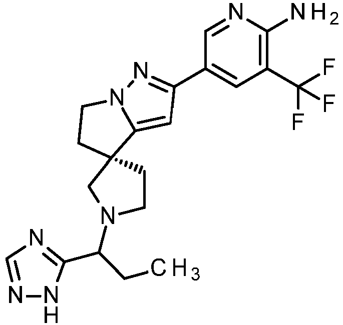

For the preparation of the diastereomeric mixture of the title compound see **Example 697.** The diastereomers were separated by preparative chiral HPLC (method see **Example 698)** to give 19.9 mg (92 % purity, 32 % yield) of the title compound (diastereomer 2 Rt = 12.77 - 16.93 min).
Analytical chiral HPLC (method see **Example 698):** Rt = 2.54 min.
LC-MS (Method 2): Rt = 0.81 min; MS (ESlpos): m/z = 433 [M+H]⁺
¹H-NMR (400 MHz, DMSO-d6) δ [ppm]: 0.760 (6.42), 0.778 (14.04), 0.797 (7.87), 0.814 (2.30), 0.821 (1.97), 0.840 (1.17), 0.862 (0.90), 0.886 (0.88), 0.904 (1.61), 0.922 (0.81), 1.005 (1.08), 1.034 (1.02), 1.053 (0.54), 1.071 (0.85), 1.083 (2.20), 1.159 (0.58), 1.204 (0.52), 1.233 (1.66), 1.259 (3.16), 1.352 (1.61), 1.391 (0.43), 1.741 (0.44), 1.757 (1.33), 1.774 (0.69), 1.798 (0.70), 1.816 (1.13), 1.831 (1.81), 1.851 (2.62), 1.872 (2.95), 1.903 (3.68), 1.916 (5.43), 1.933 (3.11), 2.322 (2.35), 2.326 (3.12), 2.331 (2.31), 2.394 (0.88), 2.413 (1.74), 2.428 (2.18), 2.446 (3.60), 2.522 (12.52), 2.612 (1.22), 2.631 (2.64), 2.654 (3.45), 2.669 (16.00), 2.850 (0.95), 2.867 (2.00), 2.888 (1.83), 2.905 (0.76), 3.565 (0.83), 3.582 (0.47), 3.599 (0.99), 3.615 (0.44), 3.704 (2.10), 4.061 (3.87), 4.078 (7.53), 4.096 (3.92), 6.306 (6.63), 6.513 (11.50), 7.988 (6.67), 7.993 (6.79), 8.561 (6.18), 8.565 (6.18), 13.811 (1.62).

### Example 700

### 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

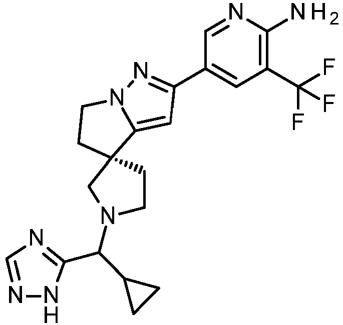

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (150 mg, 95 % purity, 396 µmol) and cyclopropyl(1H-1,2,4-triazol-5-yl)methanone (81.5 mg, 594 µmol) were dissolved in methanol (4.2 mL). N,N-Diisopropylethylamine (280 µL, 1.6 mmol) and titanium(IV) isopropoxide (350 µL, 1.2 mmol) were added and the mixture was stirred for 1.5 h at 60 °C. To the cold reaction mixture was added sodium cyanoborohydride (74.7 mg, 1.19 mmol) and the mixture was stirred for 16 h at 60 °C. The reaction mixture was allowed to cool down, diluted with water (0.5 mL) and stirred for 15 min. The suspension was filtered and the filter cake was washed with methanol. The filtrate was concentrated. The crude product was purified by preparative HPLC to give 44.0 mg (100 % purity, 25 % yield) of the title compound.
LC-MS (Method 1): Rt = 0.83 min; MS (ESlpos): m/z = 445 [M+H]⁺
¹H-NMR (400 MHz, DMSO-d6) δ [ppm]: 0.090 (0.47), 0.310 (0.62), 0.531 (0.44), 0.676 (0.61), 1.232 (0.62), 2.074 (1.27), 2.327 (4.25), 2.331 (3.10), 2.336 (1.48), 2.518 (16.00), 2.523 (11.04), 2.577 (0.63), 2.669 (4.43), 2.673 (3.19), 2.678 (1.53), 4.122 (1.50), 6.454 (0.77), 6.542 (3.86), 6.578 (1.75), 7.993 (3.06), 8.132 (0.58), 8.579 (3.24).

### Example 701

### 5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

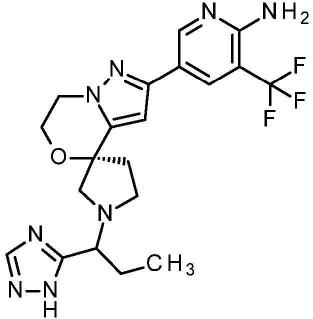

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (150 mg, 85 % purity, 339 µmol) and 1-(1H-1,2,4-triazol-5-yl)propan-1-one (63.7 mg, 509 µmol) were dissolved in methanol (3.6 mL). N,N-Diisopropylethylamine (240 µL, 1.4 mmol) and titanium(IV) isopropoxide (300 µL, 1.0 mmol) were added and the mixture was stirred for 3 h at 60 °C. To the cold reaction mixture was added sodium cyanoborohydride (64.0 mg, 1.02 mmol) and the mixture was stirred for 2 h at 60 °C. The reaction mixture was allowed to cool down, diluted with water (0.5 mL) and stirred for 15 min. The suspension was filtered and the filter cake was washed with methanol. The filtrate was concentrated. The crude product was purified by preparative HPLC to give a first batch of 21.0 mg (86 % purity, 12 % yield) and a second batch of 49.0 mg (91 % purity, 29 % yield) of the title compound.
LC-MS (Method 1): Rt = 0.77 min; MS (ESlpos): m/z = 449 [M+H]⁺
¹H-NMR (400 MHz, DMSO-d6) δ [ppm]: 0.705 (0.56), 0.726 (3.99), 0.731 (3.57), 0.745 (9.23), 0.750 (7.53), 0.763 (4.44), 0.768 (3.43), 0.917 (0.94), 0.932 (8.14), 0.934 (2.40), 0.948 (8.00), 0.952 (1.24), 1.788 (0.42), 1.805 (0.61), 1.821 (1.42), 1.840 (2.33), 1.858 (2.50), 1.875 (1.84), 1.893 (0.71), 2.003 (0.67), 2.020 (1.27), 2.036 (1.53), 2.052 (1.88), 2.074 (1.29), 2.081 (1.44), 2.099 (1.63), 2.113 (1.05), 2.131 (0.60), 2.318 (1.35), 2.323 (2.89), 2.327 (4.12), 2.331 (2.96), 2.336 (1.35), 2.386 (0.42), 2.404 (1.02), 2.421 (1.06), 2.440 (0.64), 2.518 (16.00), 2.523 (11.08), 2.619 (0.55), 2.638 (1.52), 2.659 (3.27), 2.665 (3.72), 2.669 (4.69), 2.673 (3.82), 2.678 (2.23), 2.784 (2.36), 2.809 (3.38), 2.818 (2.06), 2.830 (1.21), 2.840 (1.25), 2.865 (1.05), 2.891 (1.92), 2.924 (2.39), 2.941 (0.80), 2.950 (1.20), 2.957 (0.93), 2.974 (0.62), 3.052 (2.33), 3.078 (1.99), 3.165 (1.74), 3.640 (0.94), 3.654 (1.85), 3.668 (1.79), 3.674 (1.84), 3.689 (1.16), 3.962 (2.80), 3.974 (4.15), 3.987 (2.90), 4.042 (3.84), 4.051 (4.92), 6.436 (0.54), 6.471 (4.52), 6.504 (7.36), 6.568 (8.65), 7.620 (0.46), 8.018 (5.46), 8.023 (5.63), 8.083 (3.54), 8.589 (4.96).

### Example 702

### 5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)

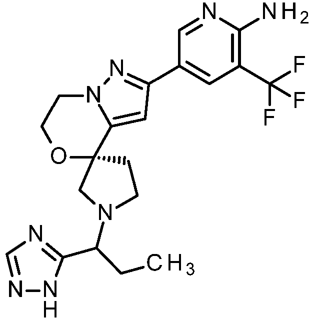

The title compound from **Example 701** was separated into diastereomers by preparative chiral HPLC to give 39.0 mg (99 % purity, 61 % yield) of the title compound (diastereomer 1 Rₜ = 14.0 - 15.9 min).

### Preparative chiral HPLC method:

Instrument: PrepCon Labomatic HPLC-3; Column: YMC Amylose SB 5µ, 250x50; eluent A: hexane + 0.1 vol % diethylamine; eluent B: ethanol; isocratic: 10%B; flow: 50 mL/min; temperature: 25 °C; UV: 280 nm

### Analytical chiral HPLC method:

Instrument: Thermo Fisher UltiMate 3000; Column: YMC Amylose SB 3µ, 100x4.6; eluent A: hexane + 0.1 vol % diethylamine; eluent B: ethanol; isocratic: 10%B; flow: 1.4 mL/min; temperature: 25 °C; UV: 280 nm
Analytical chiral HPLC: Rₜ = 5.83 min
LC-MS (Method 1): Rₜ = 0.77 min; MS (ESlpos): m/z = 449 [M+H]⁺
¹H NMR (400 MHz, DMSO-*d*₆) δ ppm 0.75 (t, 3 H), 1.78 - 1.96 (m, 2 H), 1.99 - 2.18 (m, 2 H), 2.61 - 2.69 (m, 1 H), 2.75 - 2.86 (m, 2 H), 3.07 (br d, 1 H), 3.64 - 3.73 (m, 1 H), 3.93 - 4.01 (m, 2 H), 4.02 - 4.09 (m, 2 H), 6.47 - 6.61 (m, 3 H), 7.88 and 8.45 (2 br s, 1 H), 8.02 (d, 1 H), 8.59 (d, 1 H), 13.83 (br s, 1 H).

### ^{E}xample 703

### 5-{(3'R)-1'-[1-(1H-1,2,4-Triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 2)

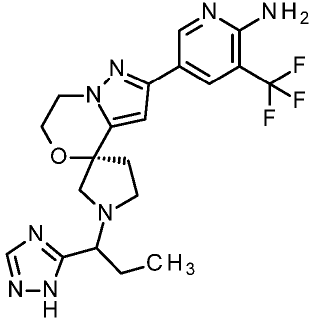

For the preparation of the diastereomeric mixture of the title compound see Example 701. The diastereomers were separated by preparative chiral HPLC (method see Example 702) to give 36.0 mg (98 % purity, 56 % yield) of the title compound (diastereomer 2 Rt = 16.9 - 19.3 min).
Analytical chiral HPLC (method see Example 702): Rₜ = 7.16 min.
LC-MS (Method 1): Rₜ = 0.77 min; MS (ESlpos): m/z = 449 [M+H]⁺
¹H NMR (400 MHz, DMSO-*d*₆) δ ppm 0.75 (br s, 3 H), 1.78 - 1.95 (m, 2 H), 1.96 - 2.20 (m, 2 H), 2.62 - 2.71 (m, 1 H), 2.78 - 2.95 (m, 3 H), 3.59 - 3.73 (m, 1 H), 3.90 - 4.10 (m, 4 H), 6.45 - 6.62 (m, 3 H), 7.89 and 8.47 (2s, 1 H), 8.02 (d, 1 H), 8.59 (s, 1 H), 13.80 and 13.87 (2s, 1 H).

### Example 704

### 5-{(3'R)-1'-[2,2-difluoro-1-(1H-imidazol-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 2)

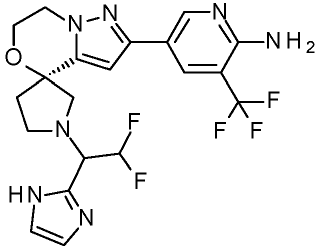

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (400 mg, 1.06 mmol) and 2,2-difluoro-1-(1H-Imidazol-2-yl)ethan-1-one (233 mg, 1.60 mmol) were dissolved in methanol (12 mL). N,N-Diisopropylethylamine (740 µL, 4.3 mmol) and titanium(IV) isopropoxide (940 µL, 3.2 mmol) were added and the mixture was stirred for 3 h at 60 °C. To the cold reaction mixture was added sodium cyanoborohydride (201 mg, 3.19 mmol) and the mixture was stirred for 16 h at 60 °C. Titanium(IV) isopropoxide (626 µL, 2.1 mmol) and sodium cyanoborohydride (134 mg, 2.1 mmol) were added again and the mixture was stirred for 22 h at 60 °C. The reaction mixture was allowed to cool down, diluted with water and stirred for 15 min. The suspension was filtered and the filter cake was washed with methanol. The filtrate was concentrated. The residue was purified by column chromatography (silica gel NH2, hexane / ethyl acetate, 0 - 100 % gradient). The combined fractions were purified by preparative HPLC to give 2.8 mg of the title compound as a mixture of diastereomers.

The diastereomers were separated by preparative chiral HPLC to give 3.90 mg (80 % purity, 1 % yield) of the title compound (diastereomer 2 Rt = 13.8 - 15.6 min).

### Preparative chiral HPLC method:

Instrument: PrepCon Labomatic HPLC-3; Column: YMC Amylose SA 5µ, 250x50; eluent A: hexane + 0.1 vol % diethylamine; eluent B: ethanol; isocratic: 20%B; flow: 50 mL/min; temperature: 25 °C; UV: 280 nm

### Analytical chiral HPLC method:

Instrument: Thermo Fisher UltiMate 3000; Column: YMC Cellulose SA 3µ, 100x4.6; eluent A: hexane + 0.1 vol % diethylamine; eluent B: ethanol; isocratic: 50%B; flow: 1.4 mL/min; temperature: 25 °C; UV: 280 nm
Analytical chiral HPLC: Rₜ = 3.84 min
LC-MS (Method 1): Rt = 0.93 min; MS (ESlpos): m/z = 470 [M+H]+
¹H NMR (400 MHz, DMSO-*d*₆) δ ppm: 12.14 (br s, 1H), 8.59 (d, 1H), 8.01 (d, 1H), 7.16 (s, 1H), 6.92 (s, 1H), 6.58 (s, 2H), 6.48 (s, 1H), 6.41 (dt, 1H), 3.93 - 4.19 (m, 5H), 2.89 - 3.06 (m, 3H), 2.74 (td, 1H), 2.12 - 2.21 (m, 1H), 2.02 - 2.10 (m, 1H).

### Example 705

### 5-{(3'R)-1'-[2,2-difluoro-1-(1H-imidazol-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 1)

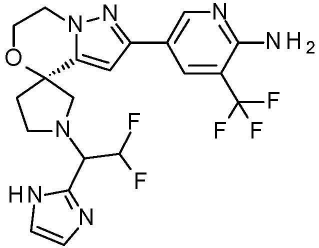

For the preparation of the diastereomeric mixture of the title compound see **Example 704.** The diastereomers were separated by preparative chiral HPLC (method see **Example 704)** to give 8.10 mg (80 % purity, 1 % yield) of the title compound (diastereomer 1 Rt = 7.9 - 9.1 min).
Analytical chiral HPLC (method see **Example 704):** Rₜ = 2.21 min
LC-MS (Method 1): Rt = 0.93 min; MS (ESlpos): m/z = 470 [M+H]⁺
¹H NMR (400 MHz, DMSO-*d*₆) δ ppm: 12.13 (br s, 1H), 8.59 (d, 1H), 8.01 (d, 1H), 7.14 - 7.16 (m, 1H), 6.92 (t, 1H), 6.58 (s, 2H), 6.54 (s, 1H), 6.40 (dt, 1H), 4.03 - 4.16 (m, 3H), 3.91 - 4.02 (m, 2H), 3.19 (d, 1H), 2.94 (td, 1H), 2.86 (d, 1H), 2.71 - 2.79 (m, 1H), 2.02 - 2.20 (m, 2H).

### Example 706

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 1 and diastereomer 2)

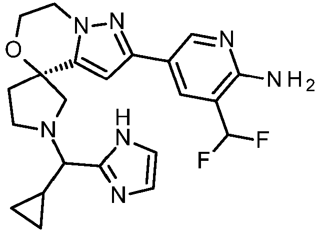

3-(Difluoromethyl)-5-[(3'R)-6,7-dihydrospiro[pyrazolo[5,1-c][1,4]oxazine-4,3'-pyrrolidin]-2-yl]pyridin-2-amine (130 mg, 405 µmol, **Intermediate** 523) and cyclopropyl(1H-imidazol-2-yl)methanone (66.1 mg, 485 µmol, CAS 952200-96-3) were dissolved in methanol (1.4 mL). N,N-Diisopropylethylamine (350 µL, 2.0 mmol) and titanium(IV) isopropoxide (360 µL, 1.2 mmol) were added and the mixture was stirred for 16 h at 60 °C. The suspension was cooled to room temperature and sodium cyanoborohydride (76.3 mg, 1.21 mmol) was added and the mixture was stirred over the weekend at 60 °C. The reaction mixture was allowed to cool down, diluted with water (1 mL) and stirred for 30 min. The suspension was diluted with methanol and filtered over celite. The filtrate was concentrated. The residue was dissolved in dichloromethane / methanol (small amount of methanol) and washed twice with saturated sodium hydrogen carbonate solution. The aqueous phase was extracted with dichloromethane / methanol. The combined organic layers were dried over an hydrophic filter and concentrated under reduced pressure. The crude product was purified by column chromatography (silica gel, dichloromethane / methanol (0 - 10 %) gradient) to give 57.0 mg (94 % purity, 30 % yield) of the title compound.
LC-MS (Method 1): Rt = 0.86 min; MS (ESlpos): m/z = 442 [M+H]⁺
¹H NMR (400 MHz, CHLOROFORM-d) δ ppm: 8.52 - 8.56 (m, 1H), 7.92 - 7.96 (m, 1H), 7.00 (2s, 2H), 6.62 and 6.61 (2t, 1H), 6.29 and 6.28 (2s, 1H), 4.97 (br d, 2H), 4.15 - 4.22 (m, 2H), 4.04 - 4.14 (m, 2H), 3.46 (d, 1H, one diastereomer), 3.25-3.32 (m, 1H, one diastereomer), 2.92 - 3.04 (m, 1H), 2.65 - 2.90 (m, 3H), 2.20 - 2.43 (m, 2H), 1.04 - 1.17 (m, 1H), 0.80 - 0.91 (m, 1H), 0.68 - 0.77 (m, 1H), 0.42 - 0.52 (m, 2H), 0.33 - 0.42 (m, 1H)

### Example 707

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 1)

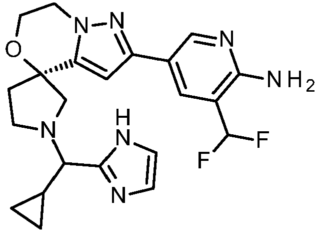

The title compound from **Example 706** was separated into diastereomers by preparative chiral HPLC to give 14.1 mg (98 % purity) of the title compound (diastereomer 1 Rₜ = 6.0 - 7.2 min).

### Preparative chiral HPLC method:

Instrument: Sepiatec: Prep SFC100; Column: Chiral Art Amylose SA 5µ, 250x50; eluent A: CO2; eluent B: 2-propanol + 0.4 vol % diethylamine; isocratic: 30%B; flow: 100 mL/min; temperature: 40 °C; BPR: 150 bar UV: 267 nm

### Analytical chiral HPLC method:

Instrument: Waters Acquity UPC2 QDA ; Column: Chiral Art Amylose SA 3µ, 100x4.6; eluent A: CO2; eluent B: 2-propanol + 0.4 vol % diethylamine; isocratic: 30%B; flow: 4 mL/min; temperature: 40 °C; BPR: 1800 psi; UV: 254 nm
Analytical chiral HPLC: Rₜ = 1.56 min
LC-MS (Method 1): Rt = 0.86 min; MS (ESlpos): m/z = 442 [M+H]⁺

### Example 708

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 2)

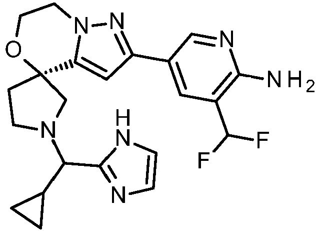

For the preparation of the diastereomeric mixture of the title compound see **Example** 706. The diastereomers were separated by preparative chiral HPLC (method see **Example 707)** to give 13.5 mg (99 % purity) of the title compound (diastereomer 2 Rₜ = 11.1 - 13.0 min).
Analytical chiral HPLC (method see **Example 707):** Rₜ = 2.98 min.
LC-MS (Method 1): Rt = 0.86 min; MS (ESlpos): m/z = 442 [M+H]⁺

### Example 709

### 2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 1 and diastereomer 2)

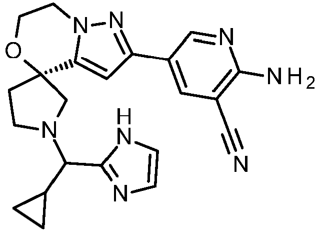

The compound was prepared similarly to **Example 706** using 2-amino-5-[(3'R)-6,7-dihydrospiro[pyrazolo[5,1-c][1,4]oxazine-4,3'-pyrrolidin]-2-yl]pyridine-3-carbonitrile (100 mg, 337 µmol, **Intermediate 524)** and cyclopropyl(1H-imidazol-2-yl)methanone (55.1 mg, 405 µmol) to give 35.0 mg (91 % purity, 23 % yield) of the title compound.
LC-MS (Method 1): Rt = 0.81 min; MS (ESlpos): m/z = 417 [M+H]⁺
¹H NMR (400 MHz, CHLOROFORM-d) δ ppm: 8.65 and 8.63 (2d, 1H), 8.10 and 8.09 (2d, 1H), 7.00 (2s, 2H), 6.27 6.26 (2s, 1H), 5.24 (s, 2H), 4.14 - 4.22 (m, 2H), 4.06 - 4.14 (m, 2H), 3.46 (d, 1H, one diastereomer), 3.25 - 3.32 (m, 1H, one diastereomer), 2.91 - 3.03 (m, 1H), 2.65 - 2.90 (m, 3H), 2.20 - 2.43 (m, 2H), 1.19 - 1.37 (m, 1H), 1.05 - 1.18 (m, 1H), 0.78 - 0.91 (m, 1H), 0.68 - 0.77 (m, 1H), 0.42 - 0.52 (m, 2H), 0.33 - 0.41 (m, 1H).

### Example 710

### 2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 1)

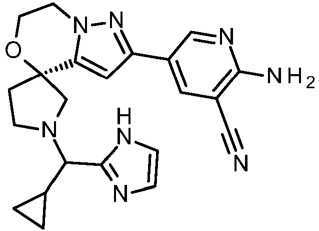

The title compound from **Example 709** was separated into diastereomers by preparative chiral HPLC to give 8.70 mg (99 % purity) of the title compound (diastereomer 1 Rt = 6.0 - 7.5 min).

### Preparative chiral HPLC method:

Instrument: Sepiatec: Prep SFC100; Column: Chiral Art Cellulose SA 10µ, 480x30mm; eluent A: CO2; eluent B: 2-propanol + 0.4 vol % diethylamine; isocratic: 30%B; flow: 100 mL/min; temperature: 40 °C; BPR: 150 bar; UV: 272 nm

### Analytical chiral HPLC method:

Instrument: Waters Acquity UPC2 QDA; Column: Chiral Art Cellulose SA 3µ, 100x4.6; eluent A: CO2; eluent B: 2-propanol + 0.4 vol % diethylamine; isocratic: 30%B; flow: 4 mL/min; temperature: 40 °C; BPR: 1800 psi UV: 254 nm
Analytical chiral HPLC: Rₜ = 1.58 min
LC-MS (Method 1): Rt = 0.81 min; MS (ESlpos): m/z = 417 [M+H]⁺
¹H NMR (CHLOROFORM-d, 400 MHz): δ (ppm) 9.47 (br s, 1H), 8.63 (d, 1H), 8.09 (d, 1H), 6.95 - 7.06 (m, 2H), 6.26 (s, 1H), 5.23 (s, 2H), 4.02 - 4.20 (m, 4H), 3.25 - 3.32 (m, 1H), 2.91 - 3.04 (m, 2H), 2.81 (d, 1H), 2.66 - 2.74 (m, 1H), 2.38 (dt, 1H), 2.21 - 2.29 (m, 1H), 1.09 - 1.18 (m, 1H), 0.69 - 0.78 (m, 1H), 0.43 -0.52 (m, 2H), 0.33 - 0.41 (m, 1H).

### Example 711

### 2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 2)

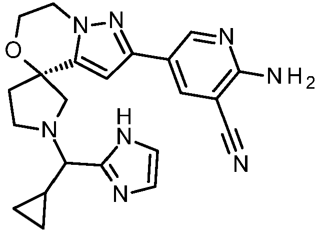

For the preparation of the diastereomeric mixture of the title compound see **Example 709.** The diastereomers were separated by preparative chiral HPLC (method see **Example 710)** to give 10.7 mg (99 % purity) of the title compound (diastereomer 2 Rₜ = 13.0 - 17.0 min).
Analytical chiral HPLC (method see Example 710): Rₜ = 3.57 min
LC-MS (Method 1): Rt = 0.81 min; MS (ESlpos): m/z = 417 [M+H]⁺
¹H NMR (CHLOROFORM-d, 400 MHz): δ (ppm) 9.57 (br s, 1H), 8.65 (d, 1H), 8.10 (d, 1H), 7.00 (br s, 2H), 6.27 (s, 1H), 5.26 (s, 2H), 4.17 - 4.21 (m, 2H), 4.09 - 4.14 (m, 2H), 3.46 (d, 1H), 2.86 (t, 2H), 2.79 (dd, 2H), 2.30 - 2.39 (m, 1H), 2.19 - 2.28 (m, 1H), 1.07 - 1.17 (m, 1H), 0.68 - 0.75 (m, 1H), 0.42 - 0.51 (m, 2H), 0.34 - 0.41 (m, 1H).

### Example 712

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 1 and diastereomer 2)

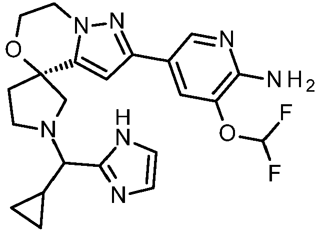

The compound was prepared similarly to **Example 706** using 3-(difluoromethoxy)-5-[(3'R)-6,7-dihydrospiro[pyrazolo[5,1-c][1,4]oxazine-4,3'-pyrrolidin]-2-yl]pyridin-2-amine (100 mg, 296 µmol, **Intermediate 525)** and cyclopropyl(1H-imidazol-2-yl)methanone (48.4 mg, 356 µmol) to give 39.0 mg (96 % purity, 28 % yield) of the title compound.
LC-MS (Method 1): Rt = 0.87 min; MS (ESlpos): m/z = 458 [M+H]⁺
¹H NMR (400 MHz, CHLOROFORM-d): δ ppm 0.009 (16.00), 0.018 (0.63), 0.317 (0.46), 0.389 (0.42), 0.396 (0.58), 0.407 (0.82), 0.424 (0.69), 0.435 (0.47), 0.654 (0.69), 0.672 (0.67), 0.686 (0.49), 0.769 (1.22), 0.783 (1.28), 0.793 (1.25), 0.803 (1.19), 0.821 (1.97), 0.828 (1.08), 1.042 (0.77), 1.194 (15.25), 1.219 (1.82), 1.226 (2.26), 1.277 (1.89), 1.312 (0.89), 1.363 (1.73), 1.556 (2.75), 1.988 (0.83), 2.181 (0.40), 2.637 (0.69), 2.655 (0.66), 2.750 (0.49), 2.775 (0.43), 3.381 (0.58), 3.409 (0.53), 4.014 (0.53), 4.028 (0.62), 4.041 (0.65), 4.054 (1.13), 4.064 (0.81), 4.095 (0.58), 4.107 (0.89), 4.118 (0.94), 4.128 (0.86), 4.134 (0.79), 4.727 (1.59), 5.242 (3.02), 6.202 (1.09), 6.214 (1.78), 6.312 (0.56), 6.317 (0.77), 6.495 (1.08), 6.500 (1.54), 6.678 (0.55), 6.683 (0.67), 6.936 (2.32), 7.631 (0.64), 7.646 (0.87), 8.228 (0.97), 8.233 (1.01), 8.245 (1.43), 8.250 (1.33).

### Example 713

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 1)

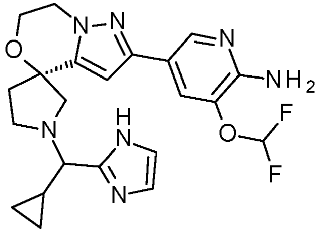

The compound from example **Example 712** was separated into diastereomers by preparative chiral HPLC to give 8.80 mg (99 % purity) of the title compound (diastereomer 1 Rt = 4.5 - 7.5 min).

### Preparative chiral HPLC method:

Instrument: Sepiatec: Prep SFC100; Column: Chiralpak ID 5µ, 250x50; eluent A: CO2; eluent B: 2-propanol + 0.4 vol % diethylamine; isocratic: 40%B; flow: 100 mL/min; temperature: 40 °C; BPR: 150 bar; UV: 267 nm

### Analytical chiral HPLC method:

Instrument: Waters Acquity UPC2 QDA; Column: Chiralpak ID 5µ, 100x4.6; eluent A: CO2; eluent B: 2-propanol + 0.4 vol % diethylamine; isocratic: 40%B; flow: 4 mL/min; temperature: 40 °C; BPR: 100 bar; UV: 267 nm
Analytical chiral HPLC Rt = 1.40 min
LC-MS (Method 1): Rt = 0.87 min; MS (ESlpos): m/z = 458 [M+H]⁺
¹H-NMR (400 MHz, CHLOROFORM-d) δ [ppm]: 0.277 (1.23), 0.293 (2.46), 0.304 (3.18), 0.322 (2.11), 0.338 (0.58), 0.395 (5.42), 0.409 (6.29), 0.424 (4.69), 0.429 (3.26), 0.444 (1.08), 0.628 (0.63), 0.644 (1.86), 0.657 (2.81), 0.673 (2.56), 0.689 (1.18), 0.794 (0.60), 0.811 (1.30), 0.829 (0.70), 1.049 (1.58), 1.066 (2.06), 1.101 (1.66), 1.117 (1.20), 1.136 (2.53), 1.151 (2.76), 1.188 (5.79), 1.215 (1.48), 1.601 (5.42), 1.958 (0.43), 2.111 (1.61), 2.134 (0.40), 2.156 (1.43), 2.171 (1.76), 2.177 (1.78), 2.191 (3.39), 2.205 (2.68), 2.211 (2.63), 2.225 (2.18), 2.277 (1.78), 2.295 (3.44), 2.312 (2.98), 2.329 (2.01), 2.347 (1.15), 2.549 (11.64), 2.567 (0.83), 2.602 (1.33), 2.642 (2.61), 2.663 (1.20), 2.738 (3.69), 2.761 (3.51), 2.866 (2.31), 2.893 (5.42), 2.925 (5.77), 2.953 (2.33), 3.191 (1.55), 3.211 (2.53), 3.229 (2.33), 3.248 (1.25), 3.960 (0.63), 3.975 (1.08), 3.990 (2.83), 4.004 (7.37), 4.018 (7.87), 4.030 (4.59), 4.047 (1.23), 4.059 (1.73), 4.085 (7.55), 4.097 (12.11), 4.110 (4.36), 4.713 (12.89), 6.193 (16.00), 6.302 (7.22), 6.485 (13.84), 6.669 (6.72), 6.917 (5.09), 6.929 (3.96), 6.952 (5.24), 7.451 (1.58), 7.622 (9.71), 8.219 (8.15), 8.223 (8.03), 9.379 (1.23).

### Example 714

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 2)

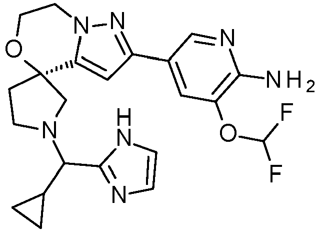

The diastereomers were separated by preparative chiral HPLC (method see Example 713) to give 9.70 mg (99 % purity) of the title compound (diastereomer 2 Rt = 9.3 - 13.3 min).
Analytical chiral HPLC (method see Example 713): Rt = 2.82 min
LC-MS (Method 1): Rt = 0.87 min; MS (ESlpos): m/z = 458 [M+H]⁺
¹H-NMR (400 MHz, CHLOROFORM-d) δ [ppm]: 0.304 (2.39), 0.317 (2.86), 0.335 (1.89), 0.360 (1.43), 0.379 (4.87), 0.395 (6.24), 0.409 (3.73), 0.624 (1.35), 0.638 (2.79), 0.657 (2.73), 0.671 (1.30), 0.811 (0.68), 0.828 (0.44), 1.006 (0.54), 1.037 (2.00), 1.049 (2.03), 1.059 (1.98), 1.068 (1.35), 1.093 (0.54), 1.104 (0.90), 1.120 (0.90), 1.138 (3.08), 1.153 (3.16), 1.189 (4.56), 1.736 (3.10), 2.112 (1.50), 2.143 (0.88), 2.160 (1.81), 2.177 (2.76), 2.195 (3.48), 2.212 (2.02), 2.230 (1.74), 2.247 (3.54), 2.264 (2.55), 2.281 (1.70), 2.298 (0.86), 2.550 (1.60), 2.568 (0.76), 2.698 (4.94), 2.725 (6.23), 2.732 (5.13), 2.756 (4.64), 2.771 (5.05), 2.788 (8.31), 2.806 (3.83), 3.371 (4.73), 3.397 (4.25), 4.028 (3.83), 4.040 (8.80), 4.051 (7.15), 4.077 (0.85), 4.108 (6.68), 4.118 (7.88), 4.122 (7.62), 4.135 (3.15), 4.769 (11.95), 6.195 (16.00), 6.312 (5.68), 6.494 (11.56), 6.678 (5.38), 6.920 (6.52), 6.936 (6.55), 7.453 (0.48), 7.636 (9.39), 8.235 (5.80), 9.556 (1.43).

### Example 715

### 5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 1 and enantiomer 2)

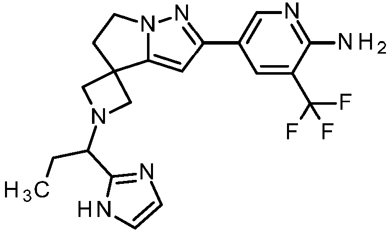

5-(5',6'-Dihydrospiro[azetidine-3,4'-pyrrolo[1,2-b]pyrazol]-2'-yl)-3-(trifluoromethyl)pyridin-2-amine (210 mg, 679 µmol, Intermediate 526) and 1-(1H-imidazol-2-yl)propan-1-one hydrogen chloride (1/1) (109 mg, 679 µmol) were dissolved in methanol (2.3 mL). N,N-Diisopropylethylamine (590 µL, 3.4 mmol) and titanium(IV) isopropoxide (600 µL, 2.0 mmol; 1) were added and the mixture was stirred for 16 h at 60 °C. The suspension was allowed to cool down, sodium cyanoborohydride (128 mg, 2.04 mmol) was added and the mixture was stirred for 3 h at 60 °C. The reaction mixture was allowed to coll down, diluted with water (1 mL) and stirred for 30 min. The suspension was diluted with methanol and filtered over celite. The filtrate was concentrated. The crude product was purified by column chromatography (silica gel, dichloromethane/ methanol (0 - 10 %) gradient). The combined fractions were dissoved in dichloromethane/ methanol and washed two times with saturated sodium hydrogen carbonate solution. The organic phase was dried over a hydrophobic filter and concentrated to give 70.0 mg (100 % purity, 25 % yield) of the title compound.
LC-MS (Method 1): Rt = 0.90 min; MS (ESlpos): m/z = 418 [M+H]⁺
¹H-NMR (400 MHz, DMSO-d6) δ [ppm]: 0.671 (4.42), 0.690 (10.55), 0.708 (4.73), 1.233 (1.09), 1.572 (0.54), 1.586 (1.03), 1.609 (1.29), 1.627 (1.26), 1.634 (1.21), 1.641 (1.04), 1.653 (1.14), 1.668 (0.49), 2.336 (0.77), 2.518 (9.22), 2.523 (6.63), 2.678 (0.80), 2.728 (13.50), 2.752 (2.19), 2.767 (3.44), 2.772 (3.46), 2.787 (2.44), 2.889 (16.00), 3.189 (1.88), 3.206 (2.29), 3.233 (2.60), 3.250 (2.85), 3.316 (3.76), 3.345 (2.22), 3.356 (1.71), 3.368 (1.48), 3.478 (2.26), 3.495 (1.99), 4.059 (2.80), 4.077 (5.30), 4.095 (2.76), 5.759 (6.17), 6.537 (6.92), 6.584 (13.63), 6.807 (1.09), 7.036 (1.08), 7.951 (2.00), 8.007 (4.00), 8.012 (4.03), 8.588 (3.69), 8.593 (3.67), 11.789 (1.78).

### Example 716

### 5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 1)

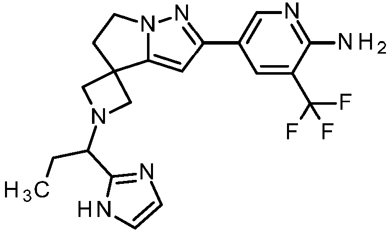

The title compound from **Example 715** was separated into enantiomers by preparative chiral HPLC to give 17.0 mg (100 % purity) of the title compound (enantiomer 1 Rt = 4.6 - 6.4 min).

### Preparative chiral HPLC method:

Instrument: Sepiatec: Prep SFC100; Column: Chiral Art Cellulose-SC 5µ, 250x50; eluent A: CO2; eluent B: 2-propanol + 0.4 vol % diethylamine; isocratic: 30%B; flow: 100 mL/min; temperature: 40 °C; BPR: 150 bar; UV: 271 nm

### Analytical chiral HPLC method:

Instrument: Waters Acquity UPC2 QDA; Column: Chiral Art Cellulose-SC 3µ, 100x4.6; eluent A: CO2; eluent B: 2-propanol + 0.4 vol % diethylamine; isocratic: 30%B; flow: 4 mL/min; temperature: 40 °C; BPR: 1800 psi; UV: 280 nm
Analytical chiral HPLC: Rₜ = 1.14 min
[α]²⁰_{D}: +34.7° (c = 1.00, methanol)
LC-MS (Method 1): Rt = 0.90 min; MS (ESlpos): m/z = 418 [M+H]⁺
¹H-NMR (400 MHz, CHLOROFORM-d) δ [ppm]: 0.737 (3.78), 0.756 (8.55), 0.774 (4.16), 1.137 (0.46), 1.153 (0.47), 1.189 (0.98), 1.396 (0.46), 1.460 (0.49), 1.479 (0.64), 1.482 (0.62), 1.495 (0.76), 1.516 (0.80), 1.535 (0.63), 1.693 (0.70), 1.702 (0.76), 1.711 (0.76), 1.721 (0.82), 1.726 (0.71), 1.736 (0.65), 1.745 (0.59), 1.755 (0.53), 2.769 (1.48), 2.773 (1.48), 2.785 (2.78), 2.791 (2.72), 2.807 (1.73), 3.275 (1.65), 3.294 (1.94), 3.361 (1.78), 3.379 (2.57), 3.442 (2.11), 3.460 (1.51), 3.472 (2.50), 3.478 (1.70), 3.489 (2.82), 3.501 (1.42), 3.510 (1.20), 4.091 (2.91), 4.108 (5.27), 4.125 (2.74), 4.961 (4.06), 6.332 (8.12), 6.957 (16.00), 8.047 (2.68), 8.052 (2.74), 8.550 (2.62), 8.554 (2.60).

### Example 717

### 5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 2)

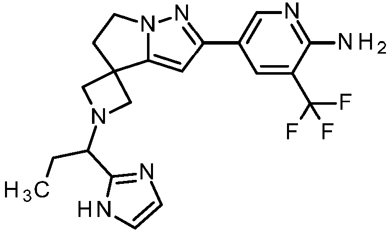

For the preparation of the enantiomers of the title compound see **Example 715.** The enantiomers were separated by preparative chiral HPLC (method see **Example 716)** to give 14.0 mg (100 % purity) of the title compound (enantiomer 2 Rt = 11.0 - 14.0 min).
Analytical chiral HPLC (method see **Example 716):** Rt = 2.88 min
[α]²⁰_{D}: -34.4° (c = 1.00, methanol)
LC-MS (Method 1): Rₜ = 0.90 min; MS (ESlpos): m/z = 418 [M+H]⁺
¹H-NMR (400 MHz, CHLOROFORM-d) δ [ppm]: 0.737 (4.55), 0.756 (10.26), 0.774 (4.99), 1.137 (0.81), 1.153 (0.86), 1.189 (1.40), 1.396 (0.44), 1.454 (0.55), 1.472 (0.74), 1.488 (0.87), 1.510 (0.94), 1.529 (0.76), 1.673 (0.59), 1.682 (0.58), 1.691 (0.99), 1.701 (1.02), 1.710 (1.00), 1.719 (1.03), 1.725 (0.91), 1.734 (0.80), 1.743 (0.71), 1.754 (0.65), 1.942 (0.76), 2.769 (1.80), 2.773 (1.78), 2.786 (3.32), 2.792 (3.15), 2.807 (2.08), 3.271 (1.85), 3.289 (2.15), 3.357 (1.92), 3.374 (2.82), 3.435 (2.31), 3.453 (1.70), 3.465 (3.03), 3.483 (2.61), 3.491 (1.63), 3.501 (1.30), 4.091 (3.56), 4.109 (6.29), 4.127 (3.26), 4.951 (4.72), 6.332 (8.90), 6.955 (16.00), 8.049 (3.16), 8.053 (3.21), 8.552 (3.07), 8.556 (3.04).

### Example 718

### 5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

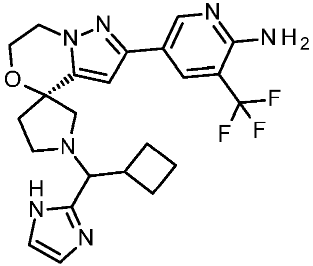

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (107 mg, 284 µmol) and cyclobutyl(1H-imidazol-2-yl)methanone (64.0 mg, 426 µmol, Intermediate 563) were dissolved in methanol (6.1 mL). N,N-Diisopropylethylamine (200 µL, 1.1 mmol) and titanium(IV) isopropoxide (250 µL, 850 µmol) were added and the mixture was stirred for 3 h at 60 °C. Sodium cyanoborohydride (53.6 mg, 852 µmol) was added and the mixture was stirred over night at 60 °C. The cold reaction mixture was filtered. The filtrate was diluted with ethyl acetate. The organic phase was washed with a saturated sodium bicarbonate solution, water and brine, dried and concentrated. The crude product was purified by column chromatography (silica gel NH2, hexane / ethyl acetate (0 - 100 %) gradient). The combined fractions were purified by preparative HPLC to give 40.0 mg (97 % purity, 29 % yield) of the title compound.
LC-MS (Method 1): Rt = 1.00 min; MS (ESlpos): m/z = 475 [M+H]⁺
¹H-NMR (400 MHz, DMSO-d6) δ [ppm]: 0.851 (0.51), 1.231 (1.39), 1.465 (0.44), 1.487 (1.47), 1.512 (2.62), 1.536 (2.52), 1.559 (1.07), 1.593 (1.66), 1.604 (1.75), 1.613 (1.85), 1.621 (1.81), 1.657 (1.90), 1.678 (1.99), 1.700 (0.90), 1.742 (1.06), 1.764 (1.71), 1.790 (1.55), 1.812 (0.89), 1.839 (1.19), 1.861 (2.08), 1.886 (2.64), 1.909 (2.01), 1.931 (0.60), 1.976 (0.49), 1.987 (0.85), 1.993 (1.06), 2.011 (1.71), 2.026 (2.66), 2.045 (3.61), 2.064 (3.97), 2.083 (4.20), 2.097 (2.92), 2.112 (2.47), 2.130 (1.40), 2.331 (2.18), 2.336 (1.00), 2.518 (12.26), 2.523 (8.55), 2.576 (2.87), 2.595 (1.58), 2.617 (0.55), 2.673 (2.33), 2.678 (1.22), 2.687 (3.70), 2.713 (4.10), 2.737 (1.04), 2.758 (3.68), 2.769 (2.75), 2.783 (3.85), 2.808 (0.63), 2.820 (1.06), 2.842 (2.24), 2.851 (3.69), 2.863 (2.51), 2.877 (2.58), 2.884 (1.89), 2.905 (0.73), 2.937 (3.90), 2.962 (3.25), 3.535 (6.06), 3.559 (5.62), 3.920 (0.41), 3.936 (1.01), 3.949 (2.50), 3.961 (3.94), 3.976 (5.23), 3.989 (4.17), 4.046 (6.68), 4.054 (5.92), 5.759 (5.17), 6.437 (10.76), 6.483 (16.00), 6.576 (12.83), 6.796 (1.37), 7.014 (1.21), 8.005 (8.64), 8.010 (8.66), 8.585 (8.08), 8.589 (7.92), 11.785 (3.61).

### Example 719

### 5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)

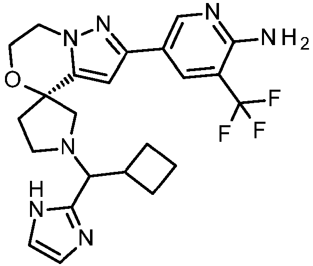

The title compound from **Example 718** was separated into diastereomers by preparative chiral HPLC (diastereomer 1 Rt = 7.4 - 8.8 min).

### Preparative chiral HPLC method:

Instrument: Sepiatec: Prep SFC100; Column: Chiral Art Aylose-SA 5µ, 250x50; eluent A: CO2; eluent B: methanol + 0.2 vol % aqueous ammonia (32 %); isocratic: 15%B; flow: 100 mL/min; temperature: 40 °C; BPR: 150 bar; UV: 267 nm

### Analytical chiral HPLC method:

Instrument: Waters Acquity UPC2 QDA; Column: Chiral Art Amylose-SA 3µ, 100x4.6; eluent A: CO2; eluent B: methanol + 0.2 vol % aqueous ammonia (32 %); isocratic: 20%B; flow: 4 mL/min; temperature: 40 °C; BPR: 100 bar; UV: 267 nm
Analytical chiral HPLC: Rₜ = 1.29 min

The isolated fraction was purified by preparative HPLC once more to give 6.30 mg (95 % purity) of the title compound.
LC-MS (Method 1): Rt = 1.00 min; MS (ESlpos): m/z = 474 [M+H]⁺
¹H-NMR (400 MHz, METHANOL-d4) δ [ppm]: 0.097 (0.60), 0.881 (0.62), 0.899 (1.26), 0.916 (0.59), 1.286 (3.78), 1.331 (1.54), 1.396 (0.46), 1.587 (0.41), 1.613 (1.13), 1.637 (2.94), 1.658 (5.37), 1.679 (3.44), 1.721 (1.55), 1.745 (1.67), 1.766 (0.80), 1.821 (0.44), 1.844 (1.30), 1.866 (2.21), 1.890 (2.01), 1.911 (1.09), 1.935 (1.27), 1.958 (1.88), 1.984 (2.54), 2.007 (1.96), 2.030 (0.91), 2.147 (0.54), 2.166 (1.50), 2.181 (2.87), 2.200 (6.82), 2.218 (5.84), 2.234 (3.11), 2.249 (0.98), 2.268 (0.50), 2.655 (1.27), 2.672 (2.80), 2.694 (3.21), 2.713 (1.28), 2.774 (5.21), 2.800 (6.23), 2.827 (1.46), 2.841 (2.19), 2.848 (2.41), 2.862 (3.48), 2.883 (3.27), 2.906 (2.26), 2.927 (1.16), 3.014 (5.68), 3.040 (4.63), 3.130 (0.45), 3.478 (0.50), 3.574 (6.22), 3.599 (5.75), 4.062 (1.56), 4.071 (2.80), 4.082 (5.22), 4.092 (7.97), 4.099 (4.22), 4.107 (8.70), 4.111 (8.22), 4.855 (1.01), 5.491 (5.00), 6.466 (16.00), 6.979 (9.72), 8.124 (6.45), 8.129 (6.55), 8.538 (6.95), 8.542 (6.30).

### Example 720

### 5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)

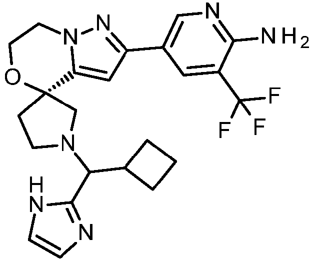

For the preparation of the diastereomeric mixture of the title compound see **Example 718.** The diastereomers were separated by preparative chiral HPLC (method see **Example 719)** (diastereomer 2 Rt = 11.0 - 14.0 min).
Analytical chiral HPLC (method see **Example 719):** Rt = 2.88 min

The crude fraction was purified by preparative HPLC once more to give 2.60 mg (95 % purity) of the title compound.
LC-MS (Method 1): Rt = 1.00 min; MS (ESlpos): m/z = 474 [M+H]⁺
¹H-NMR (400 MHz, METHANOL-d4) δ [ppm]: 0.886 (1.34), 0.903 (2.00), 1.029 (0.48), 1.291 (7.58), 1.334 (2.41), 1.401 (0.79), 1.594 (1.59), 1.620 (2.97), 1.644 (3.21), 1.667 (2.78), 1.677 (2.34), 1.686 (2.04), 1.705 (2.14), 1.730 (2.24), 1.757 (2.13), 1.778 (1.02), 1.830 (0.47), 1.854 (1.41), 1.875 (2.36), 1.900 (2.16), 1.921 (1.18), 1.946 (1.36), 1.967 (2.07), 1.993 (2.76), 2.016 (2.22), 2.040 (1.06), 2.117 (0.83), 2.136 (1.62), 2.151 (2.28), 2.169 (3.40), 2.188 (2.88), 2.210 (3.44), 2.223 (4.13), 2.242 (3.55), 2.256 (1.57), 2.275 (0.88), 2.647 (1.22), 2.660 (2.02), 2.668 (2.55), 2.682 (2.65), 2.702 (1.39), 2.829 (1.59), 2.855 (5.59), 2.870 (3.29), 2.881 (6.78), 2.918 (2.41), 2.940 (2.41), 2.962 (1.40), 2.974 (6.97), 3.000 (4.64), 3.133 (0.72), 3.482 (0.69), 3.596 (6.32), 3.620 (5.87), 4.017 (0.97), 4.032 (1.95), 4.048 (4.02), 4.055 (3.47), 4.064 (5.02), 4.075 (4.00), 4.100 (11.85), 4.110 (6.67), 4.614 (0.58), 5.345 (0.41), 5.495 (2.86), 6.451 (16.00), 7.005 (12.49), 8.128 (6.99), 8.132 (7.06), 8.545 (7.08).

### Example 721

### 5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

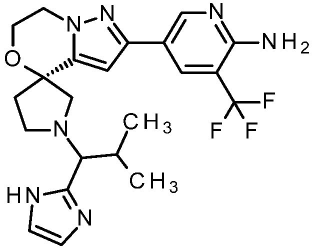

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (277 mg, 738 µmol) and 1-(1H-imidazol-2-yl)-2-methylpropan-1-one (153 mg, 1.11 mmol, Intermediate 542) were dissolved in methanol (8.0 mL). N,N-Diisopropylethylamine (510 µL, 3.0 mmol) and titanium(IV) isopropoxide (650 µL, 2.2 mmol) were added and the mixture was stirred for 3 h at 60 °C. To the cold reaction mixture was added sodium cyanoborohydride (139 mg, 2.21 mmol) and the mixture was stirred for 16 h at 60 °C. Titanium(IV) isopropoxide (433 µL, 1.46 mmol) and sodium cyanoborohydride (92 mg, 1.47 mmol) were added again and stirred for 16 h at 60 °C. The reaction mixture was allowed to cool down, diluted with water and stirred for 15 min. The suspension was filtered and the filter cake was washed with methanol. The filtrate was concentrated. The crude product was purified by column chromatography (silica gel NH2, hexane / ethyl acetate (0 - 100 %) and ethyl acetate / ethanol (0-40 %) gradient). The combined fractions were diltuted with ethyl acetate. The organic phase was washed 3 times with a sodium bicarbonate solution and concentrated. The residue was purified by preparative HPLC to give 15.0 mg (95 % purity, 4 % yield) of the title compound.
LC-MS (Method 1): Rt = 0.99 min; MS (ESlpos): m/z = 462 [M+H]⁺
¹H-NMR (400 MHz, DMSO-d6) δ [ppm]: -0.045 (0.59), 0.694 (4.97), 0.701 (4.09), 0.711 (5.39), 0.717 (3.85), 0.929 (4.53), 0.945 (6.32), 0.960 (3.31), 1.232 (1.00), 1.988 (0.43), 2.005 (0.61), 2.022 (0.80), 2.037 (0.74), 2.055 (1.45), 2.074 (1.79), 2.084 (16.00), 2.097 (0.70), 2.114 (0.48), 2.131 (0.58), 2.149 (0.77), 2.169 (0.87), 2.186 (0.72), 2.518 (4.67), 2.523 (2.85), 2.577 (0.88), 2.598 (0.47), 2.608 (0.48), 2.622 (0.48), 2.750 (0.57), 2.772 (0.49), 2.789 (0.60), 2.801 (2.08), 2.825 (2.50), 2.934 (1.12), 2.960 (0.84), 2.991 (1.55), 3.017 (1.23), 3.159 (0.78), 3.171 (0.79), 3.353 (2.39), 3.364 (1.51), 3.375 (1.78), 3.975 (2.68), 3.987 (2.29), 4.046 (2.49), 4.059 (3.29), 4.070 (1.50), 5.759 (1.14), 6.375 (3.84), 6.427 (5.43), 6.576 (4.99), 6.846 (1.37), 7.037 (1.02), 7.061 (0.72), 8.002 (3.32), 8.007 (2.20), 8.579 (3.00), 11.740 (1.32).

### Example 722

### 5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 1)

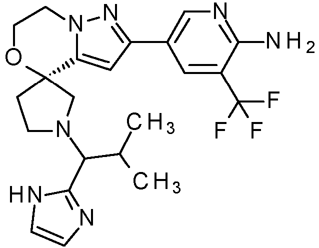

The compound 5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2, Example 721) was separated into diastereomers by preparative chiral HPLC to give 30.0 mg (95 % purity, 8 % yield) of the title compound (diastereomer 1 Rt = 7.4 - 13.0 min).

### Preparative chiral HPLC method:

Instrument: Sepiatec: Prep SFC100; Column: Chiral Art Amylose SA 5µ, 250x50; eluent A: CO2; eluent B: 2-propanol + 0.4 vol % diethylamine; isocratic: 20%B; flow: 100 mL/min; temperature: 40 °C; BPR: 150 bar; UV: 267 nm

### Analytical chiral HPLC method:

Instrument: Waters Acquity UPC2 QDA; Column: Chiral Art Amylose SA 3µ, 100x4.6; eluent A: CO2; eluent B: 2-propanol + 0.4 vol % diethylamine; isocratic: 20%B; flow: 4 mL/min; temperature: 40 °C; BPR: 1800 psi; UV: 254 nm
Analytical chiral HPLC Rt = 2.87 min
LC-MS (Method 1): Rt = 0.99 min; MS (ESlpos): m/z = 462 [M+H]+
¹H-NMR (400 MHz, DMSO-d6) δ [ppm]: 0.695 (2.60), 0.701 (2.15), 0.712 (2.77), 0.718 (2.00), 0.733 (4.24), 0.749 (4.19), 0.847 (4.51), 0.863 (4.68), 0.929 (2.39), 0.945 (3.20), 0.960 (1.70), 1.154 (4.00), 1.173 (7.77), 1.190 (4.11), 1.232 (0.67), 1.944 (0.42), 1.961 (0.67), 1.978 (0.77), 1.988 (16.00), 1.994 (0.60), 2.023 (0.42), 2.055 (0.73), 2.065 (0.56), 2.083 (0.59), 2.148 (0.44), 2.170 (0.46), 2.295 (0.44), 2.518 (2.15), 2.523 (1.46), 2.578 (0.40), 2.801 (1.06), 2.826 (1.26), 2.935 (0.56), 2.961 (0.43), 2.992 (0.78), 3.017 (0.61), 3.354 (1.10), 3.365 (0.74), 3.375 (0.92), 3.976 (1.31), 3.988 (1.13), 4.000 (1.34), 4.017 (3.60), 4.035 (3.70), 4.046 (1.33), 4.053 (2.16), 4.059 (1.72), 4.071 (0.82), 4.124 (0.43), 4.130 (0.44), 4.267 (0.80), 4.279 (0.88), 4.283 (0.86), 4.295 (0.77), 5.325 (1.90), 5.337 (1.86), 6.375 (2.14), 6.427 (3.29), 6.574 (2.51), 6.601 (0.42), 6.744 (0.60), 6.774 (0.66), 6.845 (0.85), 7.037 (0.64), 7.063 (0.43), 7.997 (0.82), 8.002 (1.70), 8.007 (1.26), 8.580 (1.68), 8.585 (1.24), 11.736 (0.73).

### Example 723

### 5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 2)

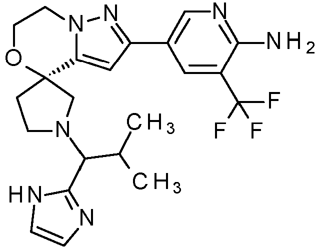

The diastereomers were separated by preparative chiral HPLC (method see **Example 722)** to give 54.4 mg (95 % purity, 15 % yield) of the title compound (diastereomer 2 Rt = 14.0 - 18.4 min).
Analytical chiral HPLC (method see **Example 722):** Rt = 5.94 min
LC-MS (Method 1): Rt = 0.99 min; MS (ESlpos): m/z = 462 [M+H]+
¹H-NMR (400 MHz, DMSO-d6) δ [ppm]: 0.695 (2.60), 0.701 (2.15), 0.712 (2.77), 0.718 (2.00), 0.733 (4.24), 0.749 (4.19), 0.847 (4.51), 0.863 (4.68), 0.929 (2.39), 0.945 (3.20), 0.960 (1.70), 1.154 (4.00), 1.173 (7.77), 1.190 (4.11), 1.232 (0.67), 1.944 (0.42), 1.961 (0.67), 1.978 (0.77), 1.988 (16.00), 1.994 (0.60), 2.023 (0.42), 2.055 (0.73), 2.065 (0.56), 2.083 (0.59), 2.148 (0.44), 2.170 (0.46), 2.295 (0.44), 2.518 (2.15), 2.523 (1.46), 2.578 (0.40), 2.801 (1.06), 2.826 (1.26), 2.935 (0.56), 2.961 (0.43), 2.992 (0.78), 3.017 (0.61), 3.354 (1.10), 3.365 (0.74), 3.375 (0.92), 3.976 (1.31), 3.988 (1.13), 4.000 (1.34), 4.017 (3.60), 4.035 (3.70), 4.046 (1.33), 4.053 (2.16), 4.059 (1.72), 4.071 (0.82), 4.124 (0.43), 4.130 (0.44), 4.267 (0.80), 4.279 (0.88), 4.283 (0.86), 4.295 (0.77), 5.325 (1.90), 5.337 (1.86), 6.375 (2.14), 6.427 (3.29), 6.574 (2.51), 6.601 (0.42), 6.744 (0.60), 6.774 (0.66), 6.845 (0.85), 7.037 (0.64), 7.063 (0.43), 7.997 (0.82), 8.002 (1.70), 8.007 (1.26), 8.580 (1.68), 8.585 (1.24), 11.736 (0.73).

### Example 724

### 5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

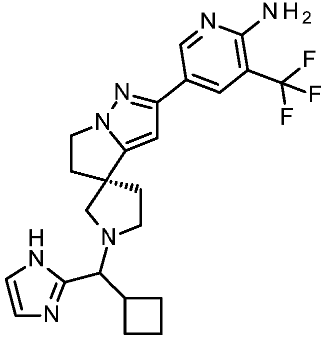

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (232 mg, 644 µmol) and cyclobutyl(1H-imidazol-2-yl)methanone (145 mg, 965 µmol, Intermediate 563) were dissolved in methanol (14 mL). N,N-Diisopropylethylamine (450 µL, 2.6 mmol) and titanium(IV) isopropoxide (570 µL, 1.9 mmol) were added and the mixture was stirred for 3 h at 60 °C. The suspension was cooled to room temperature and sodium cyanoborohydride (121 mg, 1.93 mmol) was added and the mixture was stirred over night at 60 °C. The cold reaction mixture was filtered. The filtrate was diluted with ethyl acetate, washed with saturated sodium bicarbonate solution, water and brine, dried and concentrated. The residue was purified by column chromatography (silica gel NH2, hexane / ethyl acetate (0 - 100 %) and ethyl acetate / ethanol (0-100 %) gradient). The combined fractions were purified by preparative HPLC to give 81.7 mg (90 % purity, 25 % yield) of the title compound.
LC-MS (Method 1): Rt = 1.03 min; MS (ESlpos): m/z = 458 [M+H]⁺
¹H-NMR (400 MHz, DMSO-d6) δ [ppm]: 1.154 (4.41), 1.172 (9.57), 1.190 (4.79), 1.232 (0.61), 1.475 (0.91), 1.499 (1.43), 1.523 (1.35), 1.546 (0.48), 1.628 (1.01), 1.636 (1.02), 1.647 (1.19), 1.655 (1.27), 1.675 (1.28), 1.695 (1.10), 1.723 (0.56), 1.748 (0.59), 1.756 (0.58), 1.777 (0.97), 1.799 (0.87), 1.853 (1.41), 1.876 (2.44), 1.893 (3.61), 1.912 (2.13), 1.936 (0.55), 1.987 (16.00), 2.106 (0.91), 2.379 (0.78), 2.394 (0.85), 2.401 (0.84), 2.411 (1.42), 2.421 (1.13), 2.428 (1.31), 2.437 (0.79), 2.443 (1.08), 2.461 (1.68), 2.477 (2.47), 2.518 (5.77), 2.523 (4.42), 2.563 (1.93), 2.580 (2.00), 2.602 (3.84), 2.608 (3.42), 2.631 (0.65), 2.702 (0.51), 2.723 (1.04), 2.743 (2.56), 2.766 (1.84), 2.784 (0.46), 2.800 (0.85), 2.823 (0.90), 2.838 (0.92), 2.857 (1.27), 2.879 (1.21), 2.901 (0.61), 3.159 (0.76), 3.172 (0.69), 3.557 (2.44), 3.582 (4.50), 3.608 (2.08), 3.999 (1.13), 4.017 (3.36), 4.035 (3.95), 4.053 (2.86), 4.057 (2.74), 4.062 (1.78), 4.074 (4.37), 4.092 (1.98), 5.758 (3.60), 6.240 (8.26), 6.294 (9.81), 6.515 (8.55), 6.803 (0.74), 7.019 (0.73), 7.980 (4.74), 8.559 (4.53), 11.734 (2.21).

### Example 725

### 5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)

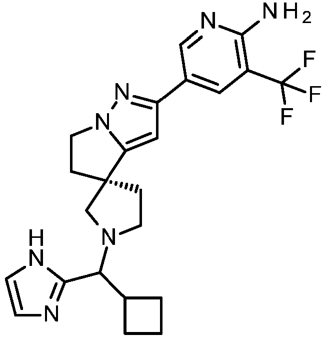

The title compound from **Example 724** was separated into diastereomers by preparative chiral HPLC (diastereomer 1 Rt = 4.0 - 5.3 min).

### Preparative chiral HPLC method:

Instrument: Sepiatec: Prep SFC100; Column: Chiralpak 5µ, 250x50; eluent A: CO2; eluent B: methanol + 0.2 vol % aqueous ammonia (32 %); isocratic: 30%B; flow: 100 mL/min; temperature: 40 °C; BPR: 150 bar; UV: 271 nm

### Analytical chiral HPLC method:

Instrument: Waters Acquity UPC2 QDA; Column: Chiralpak IG 3µ, 100x4.6; eluent A: CO2; eluent B: methanol + 0.2 vol % aqueous ammonia (32 %); isocratic: 30%B; flow: 4 mL/min; temperature: 40 °C; BPR: 100 bar; UV: 280 nm
Analytical chiral HPLC: Rₜ = 1.21 min

The isolated fraction was purified by preparative HPLC to give 8.10 mg (95 % purity) of the title compound.
LC-MS (Method 1): Rt = 1.04 min; MS (ESlpos): m/z = 458 [M+H]⁺
¹H-NMR (400 MHz, DMSO-d6) δ [ppm]: 0.852 (0.51), 1.154 (0.93), 1.172 (1.81), 1.190 (0.88), 1.232 (1.68), 1.473 (1.03), 1.499 (1.50), 1.522 (1.42), 1.545 (0.52), 1.627 (1.14), 1.636 (1.12), 1.647 (1.42), 1.655 (1.37), 1.667 (1.34), 1.675 (1.44), 1.696 (1.26), 1.718 (0.64), 1.724 (0.56), 1.748 (0.96), 1.770 (1.50), 1.792 (1.21), 1.819 (0.93), 1.836 (1.08), 1.853 (2.52), 1.873 (2.71), 1.884 (2.07), 1.900 (2.76), 1.920 (1.67), 1.931 (0.74), 1.936 (0.68), 1.951 (0.53), 1.988 (3.43), 2.084 (7.92), 2.101 (0.99), 2.109 (0.89), 2.318 (0.70), 2.361 (0.60), 2.379 (1.49), 2.394 (1.51), 2.411 (2.38), 2.428 (1.18), 2.460 (1.70), 2.478 (4.93), 2.518 (8.80), 2.523 (7.21), 2.565 (1.76), 2.582 (1.79), 2.601 (0.78), 2.703 (0.88), 2.724 (1.92), 2.744 (4.67), 2.766 (3.25), 2.834 (0.79), 2.856 (1.29), 2.878 (1.32), 2.899 (0.71), 3.558 (4.38), 3.583 (4.04), 4.017 (0.75), 4.035 (0.79), 4.058 (3.42), 4.075 (7.01), 4.092 (3.33), 5.759 (4.25), 6.294 (16.00), 6.515 (8.65), 6.811 (0.60), 7.012 (0.63), 7.979 (4.92), 7.984 (4.93), 8.559 (4.55), 8.563 (4.44), 11.735 (1.80).

### Example 726

### 5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

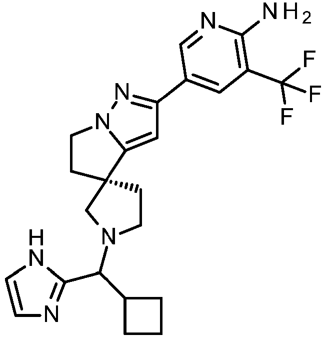

For the preparation of the diastereomeric mixture of the title compound see **Example 724.** The diastereomers were separated by preparative chiral HPLC (method see **Example 725)** (diastereomer 2 Rt = 7.3 - 9.7 min).
Analytical chiral HPLC (method see **Example 725:** Rₜ = 2.81 min

The isolted fractions were purified by preparative HPLC again to give 8.60 mg (95 % purity) of the title compound.
LC-MS (Method 1): Rt = 1.04 min; MS (ESlpos): m/z = 458 [M+H]⁺
¹H-NMR (400 MHz, DMSO-d6) δ [ppm]: 0.852 (0.49), 1.154 (3.13), 1.172 (6.02), 1.190 (2.89), 1.232 (1.56), 1.475 (0.95), 1.501 (1.43), 1.524 (1.31), 1.547 (0.48), 1.622 (0.65), 1.629 (1.08), 1.638 (1.01), 1.650 (1.21), 1.658 (1.17), 1.679 (1.31), 1.702 (1.16), 1.724 (0.56), 1.756 (0.86), 1.779 (1.38), 1.801 (1.12), 1.827 (0.66), 1.845 (0.81), 1.877 (2.56), 1.893 (5.91), 1.912 (3.53), 1.935 (0.41), 1.988 (11.46), 2.088 (0.45), 2.106 (1.04), 2.113 (0.96), 2.125 (0.91), 2.133 (0.84), 2.318 (0.69), 2.389 (0.71), 2.402 (1.43), 2.421 (2.11), 2.428 (1.57), 2.437 (1.69), 2.444 (2.27), 2.447 (2.03), 2.462 (2.21), 2.518 (8.62), 2.523 (6.27), 2.563 (2.03), 2.580 (2.14), 2.585 (2.24), 2.602 (6.56), 2.608 (6.34), 2.631 (1.02), 2.660 (0.77), 2.785 (0.76), 2.801 (1.57), 2.818 (1.28), 2.824 (1.60), 2.839 (1.26), 2.859 (1.21), 2.883 (1.23), 2.903 (0.67), 3.584 (3.96), 3.609 (3.62), 4.000 (0.88), 4.017 (2.65), 4.029 (0.64), 4.035 (3.76), 4.053 (3.47), 4.063 (2.44), 4.068 (2.36), 4.083 (1.64), 4.090 (0.55), 4.095 (0.49), 5.759 (2.14), 6.241 (16.00), 6.516 (8.11), 7.976 (4.63), 7.981 (4.65), 8.555 (4.27), 8.559 (4.21), 11.749 (1.19).

### Example 727

### 5-{(3R)-1-[1-(1H-imidazol-2-yl)-2,2-dimethylpropyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

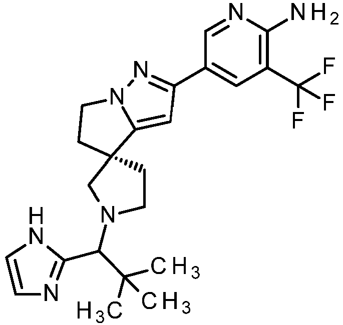

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (50.0 mg, 139 µmol) and 2-(1-chloro-2,2-dimethylpropyl)-1H-imidazol-3-ium chloride (87.2 mg, 417 µmol, Intermediate 543) were dissolved in THF (2 mL) and water (0.2 mL) and the mixture was stirred for 2 h at 0 °C. The reaction mixture was diluted with ethyl acetate and washed twice with a saturated sodium bicarbonate solution and water. The organic layer was washed with brine, dried and concentrated. The residue was purified by column chromatography (silica gel NH2, hexane / ethyl acetate (0 - 100 %) and ethyl acetate / ethanol (0-100 %) gradient). The combined fractions were purified by preparative HPLC to give 7.70 mg (95 % purity, 11 % yield) of the title compound.
LC-MS (Method 1): Rt = 1.12 min; MS (ESlpos): m/z = 460 [M+H]⁺
¹H-NMR (400 MHz, DMSO-d6) δ [ppm]: 0.990 (16.00), 0.994 (15.51), 1.232 (0.72), 1.817 (0.68), 1.834 (1.09), 1.852 (0.71), 2.254 (0.63), 2.273 (0.67), 2.332 (0.92), 2.435 (0.48), 2.518 (4.55), 2.523 (3.42), 2.756 (0.52), 2.779 (0.79), 2.786 (0.49), 2.803 (0.77), 2.813 (0.54), 2.825 (0.92), 2.835 (0.48), 2.858 (0.82), 2.881 (0.51), 2.957 (0.67), 2.980 (0.53), 3.029 (0.42), 3.035 (0.40), 3.520 (1.56), 3.539 (1.48), 4.045 (0.66), 4.063 (1.00), 4.080 (1.41), 4.097 (0.64), 6.012 (2.62), 6.295 (3.23), 6.511 (3.13), 7.035 (0.54), 7.953 (0.90), 7.958 (0.91), 7.976 (0.93), 7.981 (0.92), 8.526 (0.83), 8.530 (0.82), 8.548 (0.85), 8.552 (0.82).

### Example 728

### 5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 1 and enantiomer 2)

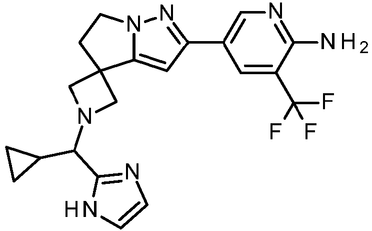

5-(5',6'-Dihydrospiro[azetidine-3,4'-pyrrolo[1,2-b]pyrazol]-2'-yl)-3-(trifluoromethyl)pyridin-2-amine (210 mg, 679 µmol, **Intermediate 526)** and cyclopropyl(1H-imidazol-2-yl)methanone (92.4 mg, 679 µmol) were dissolved in methanol (6.3 mL). N,N-Diisopropylethylamine (470 µL, 2.7 mmol) and titanium(IV) isopropoxide (600 µL, 2.0 mmol) were added and the mixture was stirred for 16 h at 60 °C. The suspension was cooled to room temperature and sodium cyanoborohydride (128 mg, 2.04 mmol) was added and the mixture was stirred for 3 h at 60 °C. The mixture was diuted with water (1 mL) and stirred for 30min. The suspension was diluted with methanol and filtered over celite. The filtrate was concentrated under reduced pressure. The residue was purified by column chromatography (silica gel, dichloromethane / methanol (0-10 %) gradient). The combined fractions were dissolved in dichloromethane / methanol and washed twice with a saturated sodium bicarbonate solution. The organic layer was dried over a hydrophobic filter and concentrated to give 120 mg (100 % purity, 41 % yield) of the title compound.
LC-MS (Method 1): Rt = 0.92 min; MS (ESlpos): m/z = 430 [M+H]⁺
¹H-NMR (400 MHz, DMSO-d6) δ [ppm]: 0.034 (0.59), 0.046 (0.60), 0.058 (0.46), 0.260 (0.43), 0.272 (0.57), 0.283 (0.49), 0.385 (0.49), 0.397 (0.66), 0.409 (0.60), 0.420 (0.40), 0.541 (0.42), 0.553 (0.54), 1.040 (0.53), 1.050 (0.44), 1.062 (0.52), 1.155 (3.67), 1.173 (8.21), 1.190 (4.30), 1.233 (0.77), 1.988 (16.00), 2.332 (1.00), 2.336 (0.43), 2.518 (4.84), 2.523 (3.46), 2.673 (1.01), 2.678 (0.43), 2.727 (1.68), 2.749 (1.65), 2.780 (0.81), 2.784 (0.75), 2.796 (1.45), 2.803 (1.45), 2.815 (0.77), 2.820 (0.84), 3.231 (0.83), 3.249 (1.10), 3.320 (2.17), 3.349 (1.48), 3.534 (1.02), 3.551 (0.88), 4.000 (1.12), 4.017 (3.39), 4.035 (3.33), 4.053 (1.08), 4.069 (1.31), 4.086 (2.83), 4.104 (1.28), 5.759 (3.77), 6.537 (3.37), 6.631 (7.48), 6.765 (0.92), 7.020 (0.86), 8.007 (1.89), 8.013 (1.91), 8.591 (1.79), 8.595 (1.75), 11.819 (0.87).

### Example 729

### 5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 1)

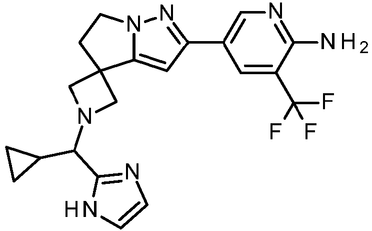

The title compound from **Example 728** was separated into enantiomers by preparative chiral HPLC to give 47.1 mg (96 % purity) of the title compound (enantiomer 1 Rt = 5.2 - 6.9 min).

### Preparative chiral HPLC method:

Instrument: Sepiatec: Prep SFC100; Column: Chiral Art Cellulose-SB 5µ, 250x50; eluent A: CO2; eluent B: 2-propanol + 0.4 vol % diethylamine; isocratic: 20%B; flow: 100 mL/min; temperature: 40 °C; BPR: 150 bar; UV: 271 nm

### Analytical chiral HPLC method:

Instrument: Waters Acquity UPC2 QDA; Column: Chiral Art Cellulose-SB 3µ, 100x4.6; eluent A: CO2; eluent B: 2-propanol + 0.4 vol % diethylamine; isocratic: 20%B; flow: 4 mL/min; temperature: 40 °C; BPR: 100 bar; UV: 280 nm
Analytical chiral HPLC: Rₜ = 1.50 min (ee value: = 100 %)
[α]²⁰_{D}: +13.5° (c = 1.00, methanol)
LC-MS (Method 1): Rt = 0.91 min; MS (ESlpos): m/z = 430 [M+H]⁺
¹H NMR (400 MHz, DMSO-*d*₆) δ ppm: 0.04 (dd, 1 H) 0.21 - 0.33 (m, 1 H) 0.36 - 0.45 (m, 1 H) 0.50 - 0.60 (m, 1 H) 1.03 (d, 2 H) 2.74 (d, 1 H) 2.80 (td, 2 H) 3.24 (d, 1 H) 3.34 - 3.36 (m, 1 H) 3.54 (d, 1 H) 4.09 (t, 2 H) 6.54 (s, 2 H) 6.63 (s, 1 H) 6.77 (br s, 1 H) 7.02 (br s, 1 H) 8.01 (d, 1 H) 8.59 (d, 1 H) 11.82 (br s, 1 H).

### Example 730

### 5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 2)

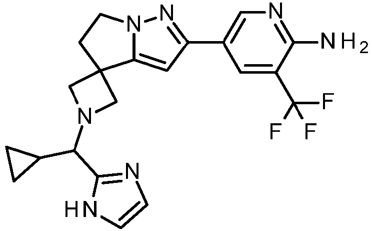

For the preparation of the racemic mixture of the title compound see **Example 728.** The enantiomers were separated by preparative chiral HPLC (method see **Example 729)** to give 41.9 mg (92 % purity) of the title compound (enantiomer 2 Rₜ = 8.7 - 10.7 min).
Analytical chiral HPLC (method see **Example 729):** Rt = 2.50 min (ee value: = 99 %)
[α]²⁰_{D}: -13.9° (c = 1.00, methanol)
LC-MS (Method 1): Rₜ = 0.91 min; MS (ESlpos): m/z = 430 [M+H]⁺
¹H NMR (400 MHz, DMSO-d6) δ ppm: 0.04 (dq, 1 H) 0.27 (tt, 1 H) 0.36 - 0.44 (m, 1 H) 0.51 - 0.60 (m, 1 H) 1.00 - 1.10 (m, 1 H) 1.03 (d, 1 H) 2.74 (d, 1 H) 2.80 (td, 2 H) 3.24 (d, 1 H) 3.35 (br s, 1 H) 3.54 (d, 1 H) 4.09 (t, 2 H) 6.54 (s, 2 H) 6.63 (s, 1 H) 6.76 (s, 1 H) 7.02 (s, 1 H) 8.01 (d, 1 H) 8.59 (d, 1 H) 11.82 (br s, 1 H).

### Example 731

### 5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

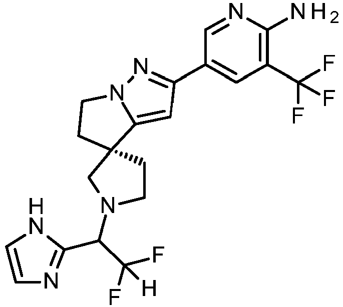

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (410 mg, 1.14 mmol) and 2,2-difluoro-1-(1H-imidazol-2-yl)ethan-1-one (250 mg, 1.71 mmol) were dissolved in methanol (10 mL). N,N-Diisopropylethylamine (790 µL, 4.6 mmol) and titanium(IV) isopropoxide (1.0 mL, 3.4 mmol) were added and the mixture was stirred for 3 h at 60 °C. Sodium cyanoborohydride (215 mg, 3.42 mmol) was added and the mixture was stirred over night at 60 °C. Titanium(IV) isopropoxide (0.33 mL, 1.1 mmol) and sodium cyanoborohydride (71 mg, 1.14 mmol) were added and the mixture was stirred over night at 60 °C. The suspension was filtered. The filtrate was diluted with ethyl acetate and extracted with a saturated sodium bicarbonate solution and water. The organic layer was washed with brine, dried and concentrated. The residue was purified by column chromatography (silica gel NH2, hexane / ethyl acetate (0-100 %) and ethyl actetate / ethanol (0-100 %) gradient). The combined fractions were purified by preparative HPLC to give 6.20 mg (95 % purity, 1 % yield) of the title compound.
LC-MS (Method 1): Rt = 0.94 min; MS (ESlpos): m/z = 454 [M+H]⁺
¹H-NMR (400 MHz, DMSO-d6) δ [ppm]: 0.852 (0.70), 1.233 (2.05), 1.924 (2.96), 1.941 (3.92), 1.959 (1.84), 1.986 (0.51), 2.005 (0.43), 2.336 (1.32), 2.402 (0.55), 2.416 (1.02), 2.435 (1.69), 2.452 (1.90), 2.465 (2.20), 2.518 (16.00), 2.522 (10.63), 2.660 (1.35), 2.690 (2.03), 2.701 (0.52), 2.713 (2.55), 2.729 (1.62), 2.740 (1.82), 2.752 (3.44), 2.775 (3.07), 2.799 (1.08), 2.902 (0.52), 2.930 (2.35), 2.953 (2.00), 2.974 (0.93), 2.997 (0.88), 4.072 (2.32), 4.087 (3.47), 4.107 (2.39), 4.126 (1.15), 4.143 (1.28), 4.157 (1.06), 4.169 (0.88), 4.183 (0.44), 5.759 (7.07), 6.257 (1.09), 6.271 (1.40), 6.276 (8.15), 6.313 (8.89), 6.395 (1.72), 6.408 (1.82), 6.523 (9.10), 6.547 (1.21), 6.905 (2.30), 6.908 (3.95), 6.912 (2.34), 6.923 (2.41), 6.927 (4.20), 6.930 (2.45), 7.149 (4.37), 7.153 (5.59), 7.157 (4.24), 7.983 (5.15), 8.556 (2.57), 8.562 (4.49), 12.103 (2.17).

### Example 732

### 5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)

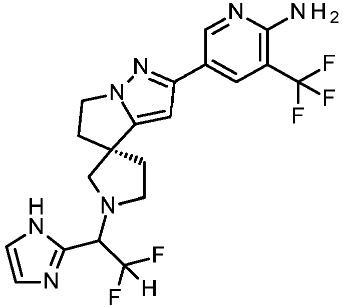

The title compound from **Example 731** was separated into diastereomers by preparative chiral HPLC (diastereomer 1 Rt = 3.6 - 5.0 min).

### Preparative chiral HPLC method:

Instrument: Sepiatec: Prep SFC100; Column: Chiralpak IG 5µ, 250x50; eluent A: CO2; eluent B: methanol + 0.2 vol % aqueous ammonia (32%); isocratic: 30%B; flow: 100 mL/min; temperature: 40 °C; BPR: 150 bar; UV: 271 nm

### Analytical chiral HPLC method:

Instrument: Waters Acquity UPC2 QDA; Column: Chiralpak IG 3µ, 100x4.6; eluent A: CO2; eluent B: methanol + 0.2 vol % aqueous ammonia (32%); isocratic: 30%B; flow: 4 mL/min; temperature: 40 °C; BPR: 100 bar; UV: 280 nm
Analytical chiral HPLC: Rt = 1.04 min

The isolated fraction was purified by preparative HPLC to give 9.50 mg (95 % purity) of the title compound.
LC-MS (Method 1): Rt = 0.95 min; MS (ESlpos): m/z = 454 [M+H]⁺
¹H-NMR (400 MHz, DMSO-d6) δ [ppm]: 0.852 (0.43), 1.154 (3.84), 1.172 (7.98), 1.190 (3.89), 1.233 (1.28), 1.399 (0.90), 1.418 (0.96), 1.893 (0.44), 1.909 (1.68), 1.917 (1.76), 1.926 (2.78), 1.935 (2.79), 1.942 (2.92), 1.952 (1.77), 1.968 (0.53), 1.988 (13.43), 2.318 (0.83), 2.385 (0.54), 2.404 (1.00), 2.418 (1.76), 2.437 (2.43), 2.452 (2.40), 2.468 (3.65), 2.518 (10.04), 2.523 (6.77), 2.692 (3.44), 2.715 (4.05), 2.739 (1.45), 2.758 (1.81), 2.777 (0.76), 2.900 (0.88), 2.919 (1.99), 2.930 (4.14), 2.953 (3.30), 3.999 (0.99), 4.017 (2.92), 4.035 (2.86), 4.042 (0.44), 4.053 (1.02), 4.068 (2.12), 4.074 (2.09), 4.087 (3.64), 4.089 (3.56), 4.103 (2.39), 4.108 (2.56), 4.120 (1.35), 4.137 (1.20), 4.151 (1.10), 4.165 (0.97), 4.178 (0.73), 5.332 (0.62), 6.263 (0.89), 6.276 (0.93), 6.317 (16.00), 6.401 (1.55), 6.414 (1.63), 6.527 (8.24), 6.553 (1.05), 7.058 (1.55), 7.983 (4.67), 7.988 (4.71), 8.564 (4.35), 8.568 (4.29).

### Example 733

### 5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)

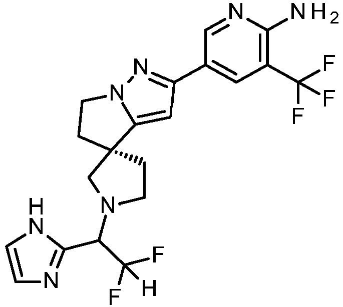

For the preparation of the diastereomeric mixture of the title compound see **Example 731.** The diastereomers were separated by preparative chiral HPLC (method see **Example 732)** (diastereomer 2 Rt = 8.5 - 11.4 min).
Analytical chiral HPLC (method see **Example 732):** Rt = 3.11 min

The isolated fraction was purified by preparative HPLC to give 6.80 mg (95 % purity) of the title compound.
LC-MS (Method 1): Rt = 0.95 min; MS (ESlpos): m/z = 454 [M+H]⁺
¹H-NMR (400 MHz, DMSO-d6) δ [ppm]: 0.852 (0.51), 1.232 (1.68), 1.924 (2.36), 1.941 (5.03), 1.959 (2.81), 2.337 (0.73), 2.405 (0.56), 2.425 (0.90), 2.437 (1.50), 2.453 (1.87), 2.456 (2.13), 2.518 (9.70), 2.523 (6.63), 2.678 (0.75), 2.701 (0.72), 2.719 (1.95), 2.729 (2.39), 2.741 (2.12), 2.752 (4.84), 2.776 (4.73), 2.799 (1.92), 2.959 (0.76), 2.975 (1.56), 2.997 (1.47), 3.015 (0.59), 4.051 (0.50), 4.054 (0.53), 4.061 (1.73), 4.073 (1.93), 4.077 (2.52), 4.080 (2.60), 4.087 (2.41), 4.092 (2.36), 4.096 (2.00), 4.107 (1.79), 4.113 (1.14), 4.119 (0.72), 4.126 (1.03), 4.139 (0.95), 4.144 (1.15), 4.152 (0.92), 4.158 (1.18), 4.170 (0.84), 4.184 (0.70), 5.759 (6.26), 6.257 (0.95), 6.276 (16.00), 6.395 (1.43), 6.409 (1.52), 6.525 (7.78), 6.543 (0.75), 6.548 (1.03), 6.906 (4.07), 6.909 (6.88), 6.913 (3.94), 7.149 (4.11), 7.152 (4.68), 7.154 (4.68), 7.157 (3.84), 7.978 (4.41), 7.983 (4.45), 8.557 (4.10), 8.560 (3.97), 12.108 (2.04).

### Example 734

### 5-{(3S)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

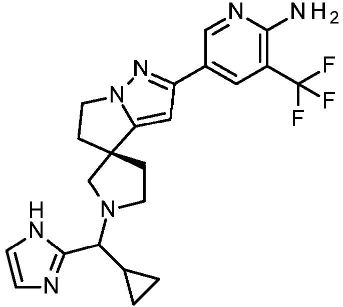

5-[(3S)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (468 mg, 85 % purity, 1.11 mmol) and cyclopropyl(1H-imidazol-2-yl)methanone (226 mg, 1.66 mmol) were dissolved in methanol (12 mL). N,N-Diisopropylethylamine (770 µL, 4.4 mmol) and titanium(IV) isopropoxide (980 µL, 3.3 mmol) were added and the mixture was stirred for 3 h at 60 °C. The reaction mixture was allowed to reach rt, sodium cyanoborohydride (208 mg, 3.32 mmol) was added and the mixture was stirred over night at 60 °C. The reaction mixture was allowed to cool down, diluted with water and stirred for 15 min. The suspension was filtered over celite and the filter cake was washed with methanol. The filtrate was concentrated. The residue was purified by column chromatography (silica gel, hexane / ethyl acetate (0-100 %) and dichloromethane / methanol (0-10 %) gradient). The combined fractions were purified by preparative HPLC to give 23.0 mg (99 % purity, 5 % yield) of the title compound.
LC-MS (Method 1): Rt = 0.95 min; MS (ESlpos): m/z = 445 [M+H]⁺
¹H NMR (400 MHz, DMSO-*d*₆) δ ppm: 0.04 - 0.10 (m, 1 H) 0.29 - 0.40 (m, 2 H) 0.57 - 0.68 (m, 1 H) 1.18 - 1.29 (m, 1 H) 1.92 - 2.03 (m, 2 H) 2.42 - 2.47 (m, 1 H) 2.52 - 2.71 (m, 4 H) 2.74 - 2.80 (m, 1 H) 2.90 - 2.97 (m, 1 H) 4.04 - 4.13 (m, 2 H) 6.44 (d, 1 H) 6.52 (s, 2 H) 6.75 (s, 1 H) 7.03 (br d, 1 H) 8.00 (s, 1 H) 8.59 (d, 1 H) 11.78 (br s, 1 H).

### Example 735

### 5-{(3S)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)

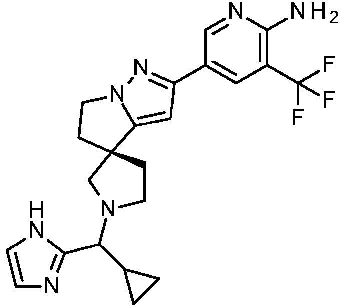

The title compound from **Example 734** was separated into diastereomers by preparative chiral HPLC (diastereomer 1 Rt = 3.7 - 4.5 min).

### Preparative chiral HPLC method:

Instrument: Sepiatec: Prep SFC100; Column: Chiral Art Amylose-SA 5µ, 250x50; eluent A: CO2; eluent B: 2-propanol + 0.4 vol % diethylamine; isocratic: 30%B; flow: 100 mL/min; temperature: 40 °C; BPR: 150 bar; UV: 271 nm

### Analytical chiral HPLC method:

Instrument: Waters Acquity UPC2 QDA; Column: Chiral Art Amylose-SA 3µ, 100x4.6; eluent A: CO2; eluent B: 2-propanol + 0.4 vol % diethylamine; isocratic: 30%B; flow: 4 mL/min; temperature: 40 °C; BPR: 100 bar; UV: 280 nm
Analytical chiral HPLC: Rₜ = 1.31 min

The crude fraction was purified by preparative HPLC to give 67.0 mg (95 % purity, 32 % yield) of the title compound.
[α]²⁰_{D}: -45.9° (c = 1.00, DMSO)
LC-MS (Method 1): Rt = 0.95 min; MS (ESlpos): m/z = 445 [M+H]⁺
¹H-NMR (400 MHz, DMSO-d6) δ [ppm]: 0.056 (0.73), 0.069 (1.26), 0.083 (1.43), 0.095 (0.79), 0.301 (0.66), 0.309 (1.18), 0.321 (2.44), 0.330 (2.66), 0.342 (2.17), 0.354 (0.92), 0.605 (0.93), 0.615 (0.87), 0.620 (0.89), 0.627 (1.22), 0.631 (1.10), 0.634 (0.99), 0.646 (0.63), 1.027 (11.52), 1.042 (12.24), 1.206 (0.50), 1.213 (0.63), 1.217 (0.63), 1.226 (1.07), 1.236 (0.93), 1.249 (0.98), 1.256 (0.56), 1.261 (0.55), 1.268 (0.46), 1.929 (0.57), 1.942 (1.15), 1.959 (2.03), 1.976 (1.72), 1.991 (1.27), 1.996 (1.42), 2.011 (1.21), 2.022 (0.57), 2.028 (0.53), 2.043 (0.42), 2.327 (0.40), 2.444 (0.61), 2.461 (1.36), 2.476 (1.52), 2.523 (1.66), 2.568 (2.20), 2.583 (3.13), 2.601 (1.92), 2.616 (1.41), 2.634 (0.67), 2.654 (3.77), 2.665 (0.51), 2.669 (0.69), 2.677 (3.72), 2.752 (0.75), 2.773 (1.56), 2.789 (1.28), 2.795 (0.87), 2.811 (0.54), 2.931 (3.14), 2.954 (2.77), 4.085 (2.64), 4.102 (5.03), 4.120 (2.60), 6.435 (0.53), 6.445 (16.00), 6.523 (7.39), 6.752 (4.89), 7.019 (4.28), 8.000 (4.21), 8.005 (4.26), 8.584 (3.93), 8.588 (3.87), 11.794 (1.79).

### Example 736

### 5-{(3S)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

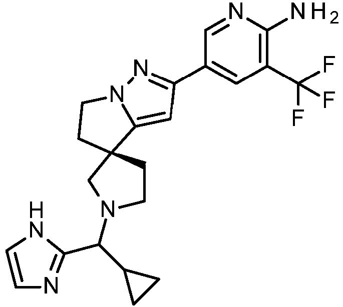

For the preparation of the diastereomeric mixture of the title compound see **Example 734.** The diastereomers were separated by preparative chiral HPLC (method see **Example 735)** (diastereomer 2 Rt = 4.7 - 5.6 min).
Analytical chiral HPLC (method see **Example 735):** Rₜ = 1.93 min

The crude fraction was purified by preparative HPLC to give 52.0 mg (95 % purity, 25 % yield) of the title compound.
[α]²⁰_{D}: -34.0° (c = 1.00, DMSO)
LC-MS (Method 1): Rt = 0.95 min; MS (ESlpos): m/z = 445 [M+H]⁺
¹H-NMR (400 MHz, DMSO-d6) δ [ppm]: 0.058 (0.84), 0.070 (1.27), 0.083 (1.39), 0.094 (1.07), 0.106 (0.47), 0.298 (0.61), 0.308 (0.99), 0.319 (1.27), 0.330 (1.43), 0.341 (0.97), 0.347 (0.97), 0.351 (1.03), 0.358 (1.22), 0.369 (1.48), 0.381 (1.34), 0.392 (0.88), 0.596 (0.48), 0.606 (0.74), 0.610 (0.66), 0.618 (1.07), 0.621 (1.00), 0.629 (1.18), 0.641 (0.97), 0.652 (0.60), 0.984 (0.56), 0.990 (0.47), 1.000 (0.54), 1.027 (7.26), 1.042 (7.81), 1.086 (0.45), 1.196 (0.57), 1.207 (0.69), 1.216 (1.17), 1.228 (1.17), 1.239 (1.14), 1.249 (0.62), 1.259 (0.52), 1.949 (0.45), 1.959 (1.52), 1.962 (1.34), 1.972 (1.51), 1.981 (2.90), 1.986 (2.18), 1.991 (2.09), 1.999 (1.63), 2.005 (1.72), 2.327 (0.44), 2.444 (0.52), 2.460 (0.73), 2.463 (0.96), 2.476 (1.73), 2.518 (2.12), 2.523 (1.59), 2.564 (2.31), 2.572 (5.32), 2.593 (1.90), 2.611 (0.69), 2.669 (0.52), 2.673 (0.43), 2.687 (3.99), 2.711 (3.84), 2.746 (3.71), 2.770 (2.98), 2.908 (0.73), 2.922 (1.13), 2.927 (1.21), 2.945 (1.28), 2.964 (0.61), 4.017 (0.46), 4.033 (0.61), 4.037 (0.73), 4.045 (1.52), 4.052 (0.80), 4.060 (1.97), 4.065 (2.82), 4.080 (2.93), 4.086 (1.89), 4.093 (0.84), 4.100 (1.52), 4.107 (0.78), 4.112 (0.61), 4.127 (0.44), 6.435 (16.00), 6.445 (0.42), 6.522 (7.92), 6.754 (4.31), 7.033 (3.64), 7.998 (4.50), 8.003 (4.58), 8.585 (4.18), 8.589 (4.16), 11.800 (1.85).

### Example 737

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

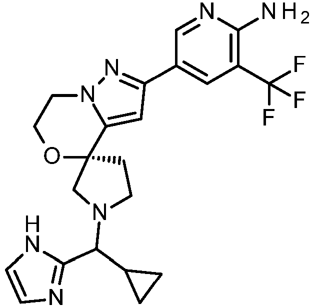

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (546 mg, 70 % purity, 1.02 mmol) and cyclopropyl(1H-imidazol-2-yl)methanone (208 mg, 1.53 mmol) were dissolved in methanol (11 mL). N,N-Diisopropylethylamine (710 µL, 4.1 mmol) and titanium(IV) isopropoxide (900 µL, 3.1 mmol) were added and the mixture was stirred for 3 h at 60 °C. To the cold reaction mixture was added sodium cyanoborohydride (192 mg, 3.05 mmol) and the mixture was stirred over night at 60 °C. The reaction mixture was allowed to cool down, diluted with water and stirred for 15 min. The suspension was filtered over celite and the filter cake was washed with methanol. The filtrate was concentrated. The residue was purified by column chromatography (silica gel, hexane / ethyl acetate (0-100 %) and dichloromethane / methanol (0-10 %) gradient). 50 mg of the combined fractions were purified by preparative HPLC to give 31.0 mg (99 % purity, 7 % yield) of the title compound.
LC-MS (Method 1): Rt = 0.93 min; MS (ESlpos): m/z = 461 [M+H]⁺
¹H NMR (400 MHz, DMSO-*d*₆) δ ppm: 11.84 - 11.91 (m, 1H), 8.62 (d, 1H), 8.03 (d, 1H), 6.99 - 7.05 (m, 1H), 6.75 (d, 1H), 6.62 - 6.64 (m, 1H), 6 .58 (s, 2H), 3.93 - 4.10 (m, 4H), 2.91 - 3.22 (m, 1H), 2.76 - 2.86 (m, 1H), 2.55 - 2.70 (m, 3H), 2.06 - 2.22 (m, 2H), 1.18 - 1.29 (m, 1H), 0.60 - 0.69 (m, 1H), 0.28 - 0.41 (m, 2H), 0.02 - 0.11 (m, 1H).

### Example 738

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)

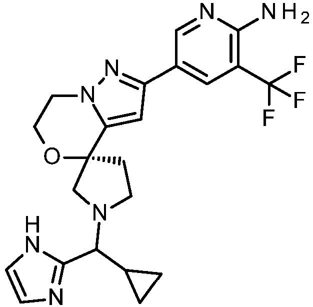

The title compound from **Example 737** was separated into diastereomers by preparative chiral HPLC (diastereomer 1 Rt = 3.6 - 4.8 min).

### Preparative chiral HPLC method:

Instrument: Sepiatec: Prep SFC100; Column: Chiral Art Amylose-SA 5µ, 250x50; eluent A: CO2; eluent B: 2-propanol + 0.4 vol % diethylamine; isocratic: 30%B; flow: 100 mL/min; temperature: 40 °C; BPR: 150 bar; UV: 267 nm

### Analytical chiral HPLC method:

Instrument: Waters Acquity UPC2 QDA; Column: Chiral Art Amylose-SA 3µ, 100x4.6; eluent A: CO2; eluent B: 2-propanol + 0.4 vol % diethylamine; isocratic: 30%B; flow: 4 mL/min; temperature: 40 °C; BPR: 100 bar; UV: 254 nm
Analytical chiral HPLC: Rₜ = 1.20 min

The crude fraction was purified by preparative HPLC to give 43.0 mg (95 % purity, 20 % yield) of the title compound.
[α]²⁰_{D}: +38.7° (c = 1.00, DMSO)
LC-MS (Method 1): Rt = 0.93 min; MS (ESlpos): m/z = 461 [M+H]⁺
¹H-NMR (400 MHz, DMSO-d6) δ [ppm]: 0.041 (0.88), 0.054 (1.30), 0.066 (1.40), 0.078 (1.08), 0.090 (0.44), 0.301 (0.62), 0.312 (1.01), 0.323 (1.25), 0.333 (1.46), 0.345 (1.40), 0.355 (1.60), 0.368 (1.49), 0.380 (1.34), 0.391 (0.86), 0.616 (0.49), 0.625 (0.76), 0.637 (1.11), 0.648 (1.17), 0.660 (0.97), 0.670 (0.58), 0.984 (0.90), 0.990 (0.57), 1.000 (0.81), 1.026 (12.85), 1.041 (11.94), 1.086 (0.49), 1.137 (0.91), 1.214 (0.57), 1.227 (0.78), 1.235 (1.20), 1.246 (1.05), 1.258 (1.08), 1.278 (0.48), 1.900 (0.40), 1.959 (1.10), 2.068 (0.44), 2.074 (0.46), 2.083 (0.74), 2.087 (0.77), 2.102 (1.42), 2.116 (1.67), 2.136 (1.00), 2.148 (0.88), 2.166 (1.65), 2.181 (1.46), 2.198 (0.78), 2.215 (0.45), 2.327 (0.52), 2.518 (1.53), 2.523 (1.18), 2.558 (0.58), 2.578 (1.29), 2.596 (1.36), 2.615 (0.57), 2.670 (4.28), 2.693 (3.71), 2.774 (2.56), 2.800 (3.23), 2.922 (3.83), 2.934 (0.92), 2.948 (3.85), 2.973 (1.29), 2.990 (0.58), 3.272 (0.43), 3.387 (0.90), 3.754 (0.45), 3.769 (0.59), 3.785 (0.43), 3.941 (0.67), 3.958 (1.12), 3.973 (2.29), 3.979 (1.69), 3.992 (2.76), 4.004 (1.84), 4.025 (0.95), 4.029 (0.88), 4.037 (0.74), 4.051 (3.41), 4.064 (4.25), 4.078 (1.66), 6.578 (7.69), 6.627 (16.00), 8.030 (4.66), 8.035 (4.74), 8.616 (4.34), 8.619 (4.30).

### Example 739

### 5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)

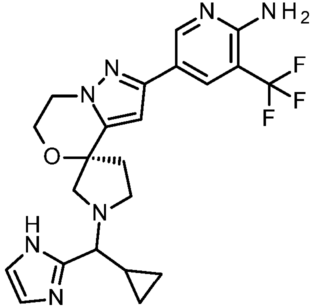

For the preparation of the diastereomeric mixture of the title compound see **Example 737.** The diastereomers were separated by preparative chiral HPLC (method see **Example 738)** (diastereomer 2 Rt = 5.9 - 7.6 min).
Analytical chiral HPLC (method see **Example 738):** Rₜ = 2.37 min

The crude fraction was purified by preparative HPLC to give 77.0 mg (95 % purity, 37 % yield) of the title compound.
[α]²⁰_{D}: +46.3° (c = 1.00, DMSO)
LC-MS (Method 1): Rt = 0.93 min; MS (ESlpos): m/z = 461 [M+H]⁺
¹H-NMR (400 MHz, DMSO-d6) δ [ppm]: 0.039 (0.42), 0.050 (0.88), 0.062 (1.35), 0.075 (1.51), 0.085 (1.17), 0.096 (0.48), 0.298 (0.68), 0.308 (1.10), 0.319 (1.36), 0.329 (1.89), 0.343 (1.54), 0.348 (2.29), 0.356 (1.73), 0.368 (1.46), 0.379 (0.94), 0.612 (0.52), 0.621 (0.84), 0.633 (1.24), 0.641 (1.26), 0.652 (1.02), 0.656 (1.08), 0.667 (0.63), 0.677 (0.42), 0.984 (0.68), 0.990 (0.46), 1.000 (0.76), 1.026 (2.30), 1.041 (2.10), 1.086 (0.43), 1.137 (0.52), 1.199 (0.57), 1.210 (0.72), 1.221 (1.25), 1.231 (1.17), 1.242 (1.15), 1.254 (0.62), 1.263 (0.55), 1.959 (0.92), 2.064 (0.46), 2.074 (1.49), 2.083 (0.92), 2.097 (1.42), 2.115 (1.95), 2.133 (1.12), 2.142 (0.96), 2.159 (1.76), 2.175 (1.60), 2.191 (0.79), 2.208 (0.48), 2.327 (0.52), 2.518 (2.04), 2.523 (1.42), 2.566 (0.65), 2.583 (1.56), 2.605 (1.61), 2.623 (0.71), 2.653 (7.61), 2.665 (0.80), 2.669 (1.20), 2.677 (5.75), 2.794 (0.78), 2.814 (1.46), 2.831 (1.38), 2.850 (0.61), 3.183 (3.29), 3.209 (2.96), 3.387 (0.65), 4.004 (1.39), 4.021 (3.94), 4.031 (4.50), 4.063 (4.40), 4.072 (3.68), 4.077 (3.67), 4.090 (1.53), 6.578 (8.18), 6.621 (0.58), 6.630 (16.00), 6.850 (0.59), 6.869 (0.53), 8.028 (4.92), 8.033 (5.00), 8.614 (4.63), 8.618 (4.56).

### Example 740 2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 1 and diastereomer 2)

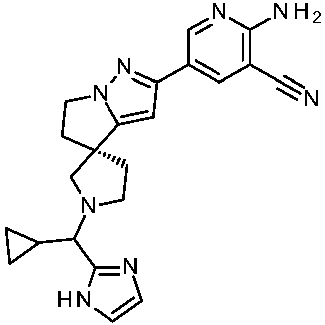

2-Amino-5-[(3R)-5',6'-dihydrospiro[pyrrolidine-3,4'-pyrrolo[1,2-b]pyrazol]-2'-yl]pyridine-3-carbonitrile hydrogen chloride (1/1) (200 mg, 631 µmol, **Intermediate 528),** cyclopropyl(1H-imidazol-2-yl)methanone (129 mg, 947 µmol), N,N-diisopropylethylamine (440 µL, 2.5 mmol) and titanium(IV) isopropoxide (560 µL, 1.9 mmol) were dissolved in methanol (6 mL) and stirred for 4 h at 60 °C. The reaction mixture was allowed to cool down, sodium cyanoborohydride (99.2 mg, 1.58 mmol) was added and the mixture was stirred over night at 60 °C. The reaction mixture was allowed to cool down, diluted with water and stirred for 10 min. The suspension was filtered over celite and the filter cake was washed with methanol. The filtrate was extracted twice with ethyl acetate. The combined organic layers were dried over a hydrophobic filter and concentrated. The residue was purified by column chromatography (silica gel, dichloromethane / methanol (0-10 %) to give 167 mg (98 % purity, 65 % yield) of the title compound.
LC-MS (Method 1): Rt = 0.82 min; MS (ESlpos): m/z = 401 [M+H]⁺
¹H-NMR (400 MHz, DMSO-d6) δ [ppm]: 0.239 (1.32), 0.248 (1.31), 0.546 (0.72), 1.080 (0.82), 1.146 (1.08), 1.177 (5.77), 1.195 (5.68), 1.263 (16.00), 1.321 (0.43), 1.894 (0.91), 2.239 (0.80), 2.495 (1.44), 2.582 (1.40), 2.697 (0.57), 2.830 (0.71), 2.852 (0.68), 3.997 (1.20), 4.013 (2.22), 4.030 (1.19), 4.742 (0.51), 4.759 (0.69), 4.777 (0.47), 6.348 (2.66), 6.886 (4.94), 6.905 (2.43), 6.924 (1.35), 7.256 (0.86), 7.274 (1.55), 7.290 (0.77), 7.475 (0.81), 7.494 (0.80), 7.588 (0.82), 7.603 (1.46), 7.619 (0.80), 8.028 (2.23), 8.033 (2.31), 8.519 (2.27), 8.524 (2.26), 11.715 (0.44).

### Example 741

### 2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 1)

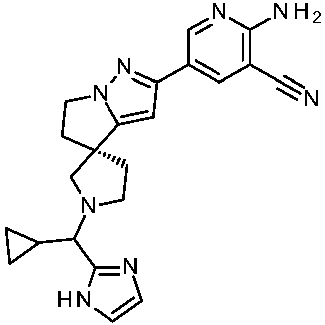

The tille compound from **Example 740** was separated into diastereomers by preparative chiral HPLC to give 97.0 mg (90 % purity) of the title compound (diastereomer 1 Rₜ = 5.0 - 6.0 min).

### Preparative chiral HPLC method:

Instrument: Sepiatec: Prep SFC360; Column: Chiralpak IG 5µ, 250x50; eluent A: CO2; eluent B: ethanol + 0.2 vol % aqueous ammonia (32%); isocratic: 30%B; flow: 300 mL/min; temperature: 40 °C; BPR: 120 bar; UV: 276 nm

### Analytical chiral HPLC method:

Instrument: Waters Acquity UPC2 QDA; Column: Chiralpak IG 3µ, 100x4.6; eluent A: CO2; eluent B: ethanol + 0.2 vol % aqueous ammonia (32%); isocratic: 30%B; flow: 4 mL/min; temperature: 37.5 °C; BPR: 100 bar; UV: 254 nm
Analytical chiral HPLC: Rₜ = 1.71 min (ee value: = 98.2 %)
[α]²⁰_{D}: +42.7° (c = 1.00, methanol)
LC-MS (Method 1): Rt = 0.82 min; MS (ESlpos): m/z = 401 [M+H]⁺
¹H NMR (400 MHz, DMSO-*d*₆) δ ppm: 11.80 (br s, 1H), 8.61 (d, 1H), 8.12 (d, 1H), 6.94 - 7.02 (m, 4H), 6.43 (s, 1H), 4.04 - 4.15 (m, 2H), 2.88 - 2.98 (m, 1H), 2.74 - 2.84 (m, 1H), 2.65 - 2.71 (m, 1H), 2.55 - 2.64 (m, 2H), 1.89 - 2.05 (m, 2H), 1.19 - 1.25 (m, 1H), 0.57 - 0.67 (m, 1H), 0.28 - 0.38 (m, 2H), 0.04 - 0.13 (m, 1H).

### Example 742

### 2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 2)

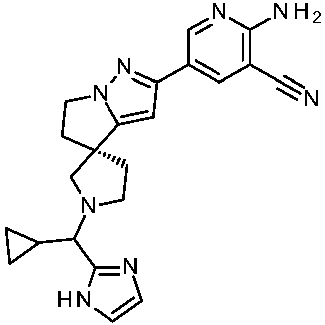

For the preparation of the diastereomeric mixture of the title compound see **Example 740** The diastereomers were separated by preparative chiral HPLC (method see **Example 741)** to give 25.0 mg (95 % purity) of the title compound (diastereomer 2 Rₜ = 6.8 - 7.77 min).
Analytical chiral HPLC (method see **Example 741):** Rₜ = 2.33 min. (ee value: = 92.2 %)
[α]²⁰_{D}: +39.6° (c = 1.00, methanol)
LC-MS (Method 1): Rₜ = 0.82 min; MS (ESlpos): m/z = 401 [M+H]⁺
¹H NMR (400 MHz, DMSO-*d*₆) δ ppm: 11.81 (br s, 1H), 8.61 (d, 1H), 8.11 (d, 1H), 6.97 (s, 2H), 6.43 (s, 1H), 4.01 - 4.14 (m, 2H), 2.87 - 2.99 (m, 1H), 2.65 - 2.80 (m, 2H), 2.53 - 2.63 (m, 2H), 1.93 - 2.03 (m, 2H), 1.17 - 1.28 (m, 1H), 0.59 - 0.68 (m, 1H), 0.27 - 0.42 (m, 2H), 0.04 - 0.13 (m, 1H).

### Example 743

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 1 and diastereomer 2)

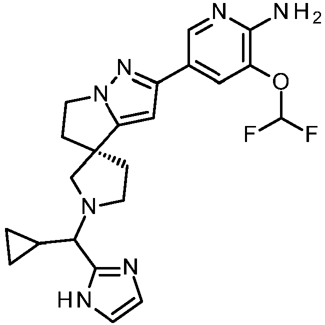

The compound was prepared similarly to **Example 740** using 3-(difluoromethoxy)-5-[(3R)-5',6'-dihydrospiro[pyrrolidine-3,4'-pyrrolo[1,2-b]pyrazol]-2'-yl]pyridin-2-amine hydrogen chloride (1/1) (200 mg, 559 µmol, **Intermediate 529)** and cyclopropyl(1H-imidazol-2-yl)methanone (114 mg, 838 µmol) to give 164 mg (100 % purity, 66 % yield) of the title compound.
LC-MS (Method 1): Rt = 0.88 min; MS (ESlpos): m/z = 442 [M+H]⁺
¹H-NMR (400 MHz, DMSO-d6) δ [ppm]: 0.094 (1.08), 0.107 (1.06), 0.118 (0.72), 0.310 (0.45), 0.334 (0.79), 0.349 (1.35), 0.365 (1.49), 0.377 (0.91), 0.389 (0.72), 0.402 (0.66), 0.413 (0.50), 0.621 (0.51), 0.653 (0.86), 0.667 (0.59), 1.235 (0.94), 1.254 (0.80), 1.955 (0.55), 1.974 (0.99), 1.994 (1.44), 2.002 (1.39), 2.017 (0.97), 2.322 (0.57), 2.326 (0.80), 2.332 (0.57), 2.447 (0.60), 2.465 (1.30), 2.518 (2.84), 2.522 (1.81), 2.568 (1.13), 2.589 (1.61), 2.605 (1.44), 2.620 (1.63), 2.660 (0.47), 2.664 (0.71), 2.668 (0.86), 2.673 (0.62), 2.772 (1.10), 2.793 (1.05), 2.966 (0.98), 2.989 (0.84), 3.165 (16.00), 4.041 (0.66), 4.060 (1.36), 4.081 (2.18), 4.098 (3.14), 4.116 (1.77), 6.143 (6.49), 6.349 (5.99), 6.356 (7.28), 6.971 (4.07), 6.981 (3.67), 6.992 (2.19), 6.994 (2.11), 7.176 (3.61), 7.179 (3.71), 7.361 (1.53), 7.363 (1.65), 7.606 (3.63), 7.608 (3.58), 8.201 (6.94), 8.205 (6.51).

### Example 744

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 1)

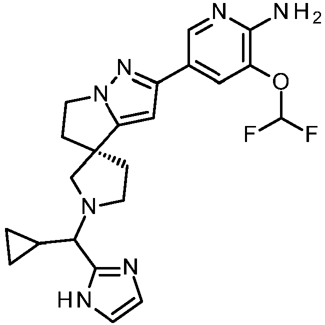

The title compound from **Example 743** was separated into diastereomers by preparative chiral HPLC to give 93.0 mg (90 % purity) of the title compound (diastereomer 1 Rt = 4.3 - 5.9 min).

### Preparative chiral HPLC method:

Instrument: Sepiatec: Prep SFC360; Column: Chiralpak IG 5µ, 250x50; eluent A: CO2; eluent B: ethanol + 0.2 vol % aqueous ammonia (32%); isocratic: 30%B; flow: 100 mL/min; temperature: 40 °C; BPR: 120 bar; UV: 254 nm

### Analytical chiral HPLC method:

Instrument: Waters Acquity UPC2 QDA; Column: Chiralpak IG 3µ, 100x4.6; eluent A: CO2; eluent B: ethanol + 0.2 vol % aqueous ammonia (32%); isocratic: 30%B; flow: 4 mL/min; temperature: 37.5 °C; BPR: 100 bar; UV: 254 nm
Analytical chiral HPLC: Rₜ = 1.43 min
[α]²⁰_{D}: +37.8° (c = 1.00, methanol)
LC-MS (Method 1): Rt = 0.94 min; MS (ESlpos): m/z = 442 [M+H]⁺
¹H NMR (400 MHz, DMSO-*d*₆) δ ppm: 11.79 (br s, 1H), 8.20 (d, 1H), 7.60 - 7.62 (m, 1H), 7.18 (t, 1H), 6.96 - 7.02 (m, 2H), 6.35 (s, 1H), 6.14 (s, 2H), 4.09 (t, 2H), 2.95 (d, 1H), 2.73 - 2.80 (m, 1H), 2.53 - 2.69 (m, 4H), 1.89 - 2.04 (m, 2H), 1.20 - 1.26 (m, 1H), 0.59 - 0.66 (m, 1H), 0.27 - 0.37 (m, 2H), 0.03 - 0.12 (m, 1H).

### Example 745

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 2)

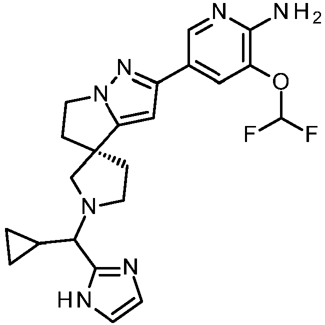

For the preparation of the diastereomeric mixture of the title compound see **Example 743.** The diastereomers were separated by preparative chiral HPLC (method see **Example 744)** to give 53.0 mg (95 % purity) of the title compound (diastereomer 2 Rₜ = 9.8 - 12.9 min).
Analytical chiral HPLC (method see **Example 744):** Rt = 3.45 min.
[α]²⁰_{D}: +41.0° (c = 1.00, methanol)
LC-MS (Method 1): Rₜ = 0.94 min; MS (ESlpos): m/z = 442 [M+H]⁺
¹H NMR (400 MHz, DMSO-*d*₆) δ ppm: 11.81 (br s, 1H), 8.20 (d, 1H), 7.61 (s, 1H), 7.18 (t, 1H), 6.69-7.08 (m, 2H), 6.34 (s, 1H), 6.14 (s, 2H), 3.99 - 4.13 (m, 2H), 2.90 - 2.98 (m, 1H), 2.68 - 2.78 (m, 2H), 2.54 - 2.63 (m, 3H), 1.91 - 2.05 (m, 2H), 1.18 - 1.26 (m, 1H), 0.58 - 0.68 (m, 1H), 0.27 - 0.43 (m, 2H), 0.03 - 0.13 (m, 1H).

### Example 746

### 2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 1 and diastereomer 2)

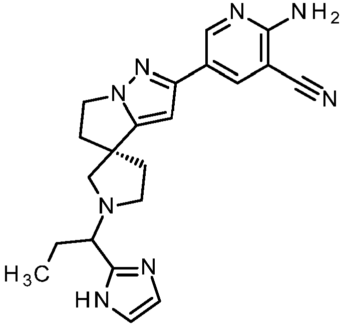

The compound was prepared similarly to **Example 740** using 2-amino-5-[(3R)-5',6'-dihydrospiro[pyrrolidine-3,4'-pyrrolo[1,2-b]pyrazol]-2'-yl]pyridine-3-carbonitrile hydrogen chloride (1/1) (200 mg, 631 µmol, **Intermediate 528)** and 1-(1H-imidazol-2-yl)propan-1-one (118 mg, 947 µmol) to give 192 mg (100 % purity, 78 % yield) of the title compound.
LC-MS (Method 1): Rt = 0.79 min; MS (ESlpos): m/z = 389 [M+H]⁺
¹H-NMR (400 MHz, DMSO-d6) δ [ppm]: 0.715 (1.07), 0.720 (1.13), 0.734 (2.52), 0.739 (2.45), 0.752 (1.20), 0.757 (1.13), 0.826 (0.64), 1.832 (0.41), 1.876 (0.41), 1.889 (0.42), 1.895 (0.40), 1.907 (0.54), 1.917 (0.88), 1.934 (1.31), 1.951 (0.63), 2.447 (0.47), 2.456 (0.44), 2.473 (0.62), 2.518 (1.20), 2.522 (0.79), 2.626 (0.53), 2.647 (0.64), 2.664 (0.43), 2.668 (0.44), 2.679 (0.40), 2.817 (0.47), 3.165 (16.00), 3.604 (0.42), 4.070 (1.05), 4.078 (0.68), 4.088 (1.39), 4.097 (0.53), 4.105 (0.68), 6.327 (2.48), 6.352 (2.69), 6.973 (3.02), 7.032 (2.86), 7.039 (3.14), 8.103 (1.43), 8.109 (2.66), 8.115 (1.51), 8.589 (1.54), 8.594 (2.19), 8.599 (1.54).

### Example 747

### 2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 1)

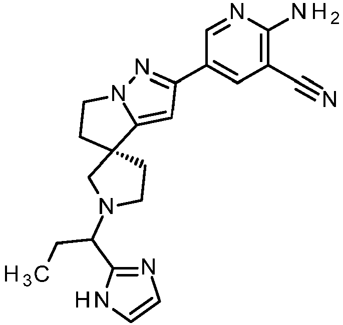

The title compound from **Example 746** was separated into diastereomers by preparative chiral HPLC to give 62.9 mg (97 % purity) of the title compound (diastereomer 1 Rₜ = 9.5 - 10.7 min).

### Preparative chiral HPLC method:

Instrument: Sepiatec: Prep SFC100; Column: Chiral Art Cellulose-SB 5µ, 250x50; eluent A: CO2; eluent B: 2-propanol + 0.4 vol % diethylamine; isocratic: 20%B; flow: 100 mL/min; temperature: 40 °C; BPR: 150 bar; UV: 276 nm

### Analytical chiral HPLC method:

Instrument: Waters Acquity UPC2 QDA; Column: Chiral Art Cellulose-SB 3µ, 100x4.6; eluent A: CO2; eluent B: 2-propanol + 0.4 vol % diethylamine; isocratic: 20%B; flow: 4 mL/min; temperature: 40 °C; BPR: 100 bar; UV: 280 nm
Analytical chiral HPLC: Rₜ = 2.95 min
[α]²⁰_{D}: +52.1° (c = 1.00, methanol)
LC-MS (Method 1): Rt = 0.81 min; MS (ESlpos): m/z = 389 [M+H]⁺
¹H NMR (400 MHz, DMSO-*d*₆) δ ppm: 11.77 (br s, 1H), 8.59 (d, 1H), 8.11 (d, 1H), 6.71 - 7.10 (m, 4H), 6.31 (s, 1H), 4.01 - 4.13 (m, 2H), 3.52 (dd, 1H), 2.79 - 2.90 (m, 1H), 2.63 - 2.69 (m, 1H), 2.55 - 2.62 (m, 2H), 2.38 - 2.46 (m, 1H), 1.73 - 1.95 (m, 4H), 0.74 (t, 3H).

### Example 748

### 2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 2)

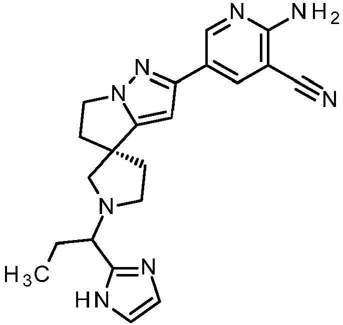

For the preparation of the diastereomeric mixture of the title compound see **Example 746.** The diastereomers were separated by preparative chiral HPLC (method see **Example 747)** to give 62.9 mg (90 % purity) of the title compound (diastereomer 2 Rₜ = 11.6 - 13.1 min).
Analytical chiral HPLC (method see **Example 747):** Rₜ = 3.58 min.
[α]²⁰_{D}: +69.9° (c = 1.00, methanol)
LC-MS (Method 1): Rₜ = 0.81 min; MS (ESlpos): m/z = 389 [M+H]⁺
¹H NMR (400 MHz, DMSO-*d*₆) δ ppm: 11.77 (br s, 1H), 8.60 (d, 1H), 8.11 (d, 1H), 6.74 - 7.10 (m, 4H), 6.34 (s, 1H), 4.08 (br t, 2H), 3.52 (br dd, 1H), 2.74 - 2.83 (m, 2H), 2.54 - 2.64 (m, 2H), 2.37 - 2.46 (m, 1H), 1.73 - 1.98 (m, 4H), 0.73 (br t, 3H).

### Example 749

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)

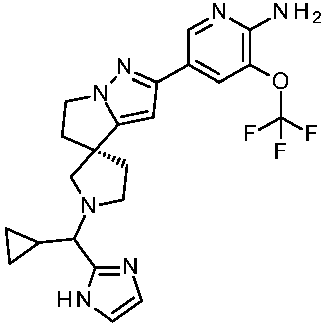

The compound was prepared similarly to **Example 740** using 5-[(3R)-5',6'-dihydrospiro[pyrrolidine-3,4'-pyrrolo[1,2-b]pyrazol]-2'-yl]-3-(trifluoromethoxy)pyridin-2-amine hydrogen chloride (1/1) (200 mg, 532 µmol, **Intermediate 527)** and 1 cyclopropyl(1H-imidazol-2-yl)methanone (109 mg, 798 µmol) to give 161 mg (99 % purity, 65 % yield) of the title compound.
LC-MS (Method 1): Rt = 0.95 min; MS (ESlneg): m/z = 458 [M-H]⁻
¹H-NMR (400 MHz, DMSO-d6) δ [ppm]: -0.131 (0.61), 0.093 (1.22), 0.105 (1.25), 0.119 (1.36), 0.312 (0.73), 0.344 (1.02), 0.361 (1.69), 0.377 (1.85), 0.391 (0.99), 0.404 (0.90), 0.417 (0.82), 0.429 (0.69), 0.440 (0.44), 0.533 (0.62), 0.631 (0.64), 0.651 (1.07), 0.665 (1.13), 0.677 (0.76), 1.027 (0.78), 1.042 (0.78), 1.217 (1.25), 1.237 (4.49), 1.254 (4.44), 1.950 (0.41), 1.962 (0.72), 1.981 (1.35), 2.000 (1.99), 2.009 (1.72), 2.041 (0.44), 2.323 (0.69), 2.327 (0.98), 2.332 (0.70), 2.450 (0.68), 2.468 (1.58), 2.518 (4.10), 2.523 (2.46), 2.572 (1.12), 2.591 (1.64), 2.609 (1.75), 2.624 (1.80), 2.642 (1.62), 2.660 (1.12), 2.665 (1.34), 2.669 (1.48), 2.673 (1.08), 2.785 (1.36), 2.804 (1.43), 2.978 (1.19), 2.999 (1.03), 3.141 (0.95), 3.166 (16.00), 4.048 (0.86), 4.067 (1.68), 4.087 (2.69), 4.105 (3.48), 4.123 (1.82), 6.394 (6.48), 6.402 (7.86), 6.487 (8.51), 7.008 (5.93), 7.020 (5.12), 7.039 (0.41), 7.729 (2.17), 7.733 (3.80), 7.737 (3.83), 8.328 (7.14), 8.333 (6.94).

### Example 750

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1)

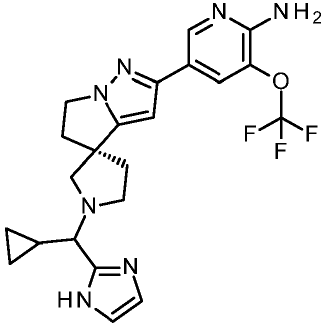

The title compound from **Example 749** was separated into diastereomers by preparative chiral HPLC to give 56.5 mg (93 % purity) of the title compound (diastereomer 1 Rₜ = 3.45 - 5.29 min).

### Preparative chiral HPLC method:

Instrument: Sepiatec: Prep SFC100; Column: Chiralpak IG 5µ, 250x50; eluent A: CO2; eluent B: methanol + 0.2 vol % aqueous ammonia (32%); isocratic: 30%B; flow: 100 mL/min; temperature: 40 °C; BPR: 150 bar; UV: 280 nm

### Analytical chiral HPLC method:

Instrument: Waters Acquity UPC2 QDA; Column: Chiralpak IG 3µ, 100x4.6; eluent A: CO2; eluent B: methanol + 0.2 vol % aqueous ammonia (32%); isocratic: 30%B; flow: 4 mL/min; temperature: 37.5 °C; BPR: 100 bar; UV: 280 nm
Analytical chiral HPLC: Rₜ = 0.93 min (ee value: = 98 %)
[α]²⁰_{D}: +75.7° (c = 1.00, methanol)
LC-MS (Method 1): Rt = 0.97 min; MS (ESlneg): m/z = 458 [M-H]⁻
¹H NMR (400 MHz, DMSO-*d*₆) δ ppm: 11.79 (br s, 1H), 8.33 (d, 1H), 7.74 (t, 1H), 6.94 - 7.08 (m, 1H), 6.75 (br s, 1H), 6.48 (s, 2H), 6.40 (s, 1H), 2.74 - 2.81 (m, 1H), 2.52 - 2.69 (m, 4H), 2.42 - 2.48 (m, 1H), 1.89 - 2.06 (m, 2H), 1.17 - 1.29 (m, 1H), 0.57 - 0.66 (m, 1H), 0.27 - 0.38 (m, 2H), 0.03 - 0.12 (m, 1H).

### Example 751

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 2)

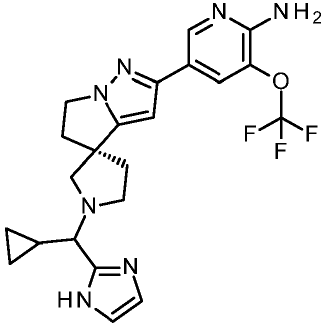

For the preparation of the diastereomeric mixture of the title compound see **Example 749** The diastereomers were separated by preparative chiral HPLC (method see **Example 750)** to give 47.4 mg (94 % purity) of the title compound (diastereomer 2 Rₜ = 5.80 - 7.50 min).
Analytical chiral HPLC (method see **Example 750):** Rₜ = 1.99 min.
[α]²⁰_{D}: +40.5° (c = 1.00, methanol)
LC-MS (Method 1): Rₜ = 0.97 min; MS (ESlneg): m/z = 458 [M-H]⁻
¹H NMR (400 MHz, DMSO-*d*₆) δ ppm: 11.79 (br s, 1H), 8.33 (d, 1H), 7.72 - 7.75 (m, 1H), 7.03 (br s, 1H), 6.75 (br s, 1H), 6.48 (s, 2H), 6.39 (s, 1H), 4.00 - 4.13 (m, 2H), 2.89 - 2.97 (m, 1H), 2.75 (d, 1H), 2.70 (d, 1H), 2.53 - 2.62 (m, 3H), 2.43 - 2.48 (m, 1H), 1.92 - 2.05 (m, 2H), 1.17 - 1.28 (m, 1H), 0.58 - 0.67 (m, 1H), 0.27 - 0.43 (m, 2H), 0.07 (dq, 1H).

### Example 752

### 3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 1 and diastereomer 2)

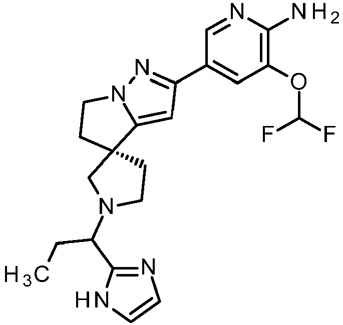

The compound was prepared similarly to **Example 740** using 3-(difluoromethoxy)-5-[(3R)-5',6'-dihydrospiro[pyrrolidine-3,4'-pyrrolo[1,2-b]pyrazol]-2'-yl]pyridin-2-amine hydrogen chloride (1/1) (413 mg, 1.15 mmol, **Intermediate 529)** and 1-(1H-imidazol-2-yl)propan-1-one (215 mg, 1.73 mmol) to give 405 mg (97 % purity, 79 % yield) of the title compound.
LC-MS (Method 1): Rt = 0.85 min; MS (ESlpos): m/z = 430 [M+H]⁺
¹H-NMR (400 MHz, DMSO-*d*₆) δ [ppm]: 8.18 (t, 1H), 7.58 - 7.61 (s, 1H), 7.18 (t, 1H), 7.06 (d, 2H), 6.22 and 6.26 (2s, 1H), 6.14 (s, 2H), 4.02 - 4.11 (m, 2H), 3.58 - 3.67 (m, 1H), 2.59 - 2.94 (m, 4H), 2.39 - 2.47 (m, 1H), 1.75 - 2.00 (m, 4H), 0.70 - 0.77 (m, 3H).

### Example 753

### 3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 1)

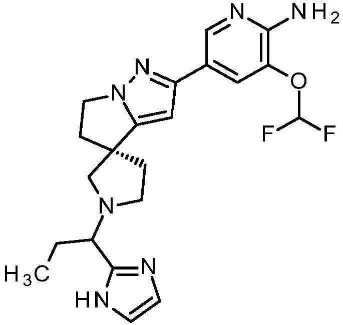

The title compound from **Example 752** was separated into diastereomers by preparative chiral HPLC to give 134 mg (96 % purity) of the title compound (diastereomer 1 Rt = 9.70 - 11.90 min).

### Preparative chiral HPLC method:

Instrument: Sepiatec: Prep SFC100; Column: Chiral Art Amylose-SA 5µ, 250x50; eluent A: CO2; eluent B: methanol + 0.2 vol % aqueous ammonia (32%); isocratic: 10%B; flow: 100 mL/min; temperature: 40 °C; BPR: 150 bar; UV: 280 nm

### Analytical chiral HPLC method:

Instrument: Waters Acquity UPC2 QDA; Column: Chiral Art Amylose-SA 3µ, 100x4.6; eluent A: CO2; eluent B: methanol + 0.2 vol % aqueous ammonia (32%); isocratic: 20%B; flow: 4 mL/min; temperature: 37.5 °C; BPR: 100 bar; UV: 280 nm
Analytical chiral HPLC: Rₜ = 1.01 min
[α]²⁰_{D}: +58.5° (c = 1.00, methanol)
LC-MS (Method 1): Rt = 0.87 min; MS (ESlpos): m/z = 430 [M+H]⁺
¹H NMR (DMSO-*d*₆, 400 MHz): δ (ppm) 11.78 (br s, 1H), 8.19 (d, 1H), 7.59 - 7.61 (m, 1H), 7.18 (t, 1H), 7.04 (br s, 1H), 6.82 (br s, 1H), 6.24 (s, 1H), 6.13 (s, 2H), 4.04 - 4.11 (m, 2H), 3.52 (dd, 1H), 2.73 - 2.83 (m, 2H), 2.53 - 2.64 (m, 4H), 2.36 - 2.47 (m, 1H), 1.73 - 1.99 (m, 4H), 0.73 (t, 3H).

### Example 754

### 3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 2)

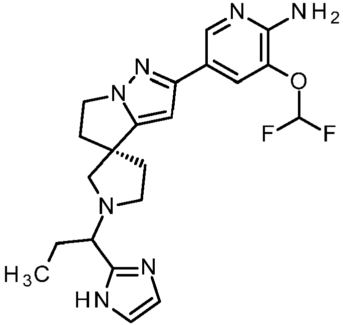

For the preparation of the diastereomeric mixture of the title compound see **Example 752.** The diastereomers were separated by preparative chiral HPLC (method see **Example 753)** to give 152 mg (99 % purity) of the title compound (diastereomer 2 Rt = 12.80 - 15.90 min).
Analytical chiral HPLC (method see **Example 753):** Rt = 1.44 min.
[α]²⁰_{D}: +40.0° (c = 1.00, methanol)
LC-MS (Method 1): Rt = 0.86 min; MS (ESlpos): m/z = 430 [M+H]⁺
¹H NMR (400 MHz, DMSO-*d*₆) δ ppm: 12.35 (br s, 1H), 8.18 (d, 1H), 7.58 - 7.61 (m, 1H), 7.18 (t, 1H), 7.06 (s, 2H), 6.22 (s, 1H), 6.14 (s, 2H), 4.01 - 4.12 (m, 2H), 3.59 - 3.67 (m, 1H), 2.87 - 2.95 (m, 1H), 2.60 - 2.75 (m, 3H), 2.40 - 2.46 (m, 1H), 1.76 - 1.98 (m, 4H), 0.74 (t, 3H).

### Example 755

### 5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)

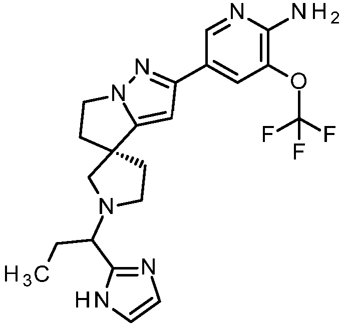

The compound was prepared similarly to **Example 740** using 5-[(3R)-5',6'-dihydrospiro[pyrrolidine-3,4'-pyrrolo[1,2-b]pyrazol]-2'-yl]-3-(trifluoromethoxy)pyridin-2-amine hydrogen chloride (1/1) (200 mg, 532 µmol, **Intermediate 527)** and 1-(1H-imidazol-2-yl)propan-1-one (99.1 mg, 798 µmol) to give 171 mg (100 % purity, 72 % yield) of the title compound.
LC-MS (Method 1): Rt = 0.93 min; MS (ESlpos): m/z = 448 [M+H]⁺
¹H-NMR (400 MHz, DMSO-d6) δ [ppm]: -0.131 (0.51), 0.090 (0.51), 0.311 (0.52), 0.532 (0.51), 0.714 (4.06), 0.721 (4.36), 0.733 (9.65), 0.739 (9.23), 0.751 (4.58), 0.758 (4.07), 0.807 (2.09), 0.825 (4.92), 0.843 (2.21), 1.027 (2.44), 1.042 (2.40), 1.234 (1.88), 1.253 (1.54), 1.670 (0.41), 1.686 (0.58), 1.704 (0.77), 1.722 (0.52), 1.739 (0.49), 1.753 (0.59), 1.757 (0.54), 1.772 (1.03), 1.791 (1.09), 1.806 (1.42), 1.827 (1.65), 1.848 (1.73), 1.869 (1.80), 1.883 (1.82), 1.888 (1.68), 1.900 (1.70), 1.907 (1.61), 1.917 (3.55), 1.933 (4.89), 1.952 (2.63), 2.323 (0.89), 2.327 (1.28), 2.332 (0.86), 2.392 (0.43), 2.400 (0.44), 2.409 (1.04), 2.424 (1.28), 2.432 (1.30), 2.442 (1.86), 2.451 (1.62), 2.458 (1.35), 2.470 (1.96), 2.518 (4.81), 2.523 (3.34), 2.578 (1.11), 2.602 (1.80), 2.619 (2.12), 2.641 (2.66), 2.660 (1.11), 2.665 (1.45), 2.669 (1.63), 2.674 (1.24), 2.685 (1.42), 2.709 (0.75), 2.766 (0.44), 2.787 (0.87), 2.803 (0.80), 2.825 (1.56), 2.848 (1.14), 2.878 (0.85), 2.900 (0.75), 3.166 (16.00), 3.337 (1.07), 3.577 (1.55), 3.596 (1.48), 4.047 (1.43), 4.065 (3.90), 4.072 (2.32), 4.082 (5.30), 4.091 (1.81), 4.099 (2.40), 4.537 (0.49), 5.759 (0.85), 6.255 (10.66), 6.295 (10.65), 6.479 (11.68), 6.994 (5.16), 7.006 (10.71), 7.012 (9.94), 7.718 (2.58), 7.723 (4.34), 7.727 (4.28), 8.307 (5.79), 8.312 (8.80), 8.317 (5.84).

### Example 756

### 5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 1)

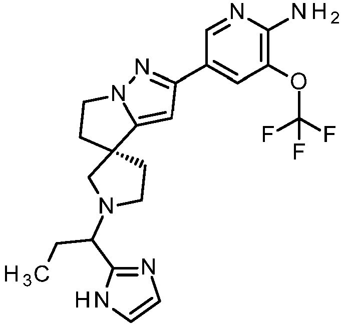

The title compound from **Example 755** was separated into diastereomers by preparative chiral HPLC to give 56.4 mg (100 % purity) of the title compound (diastereomer 1 Rt = 3.30 - 4.30 min).

### Preparative chiral HPLC method:

Instrument: Sepiatec: Prep SFC100; Column: Chiralpak IG 5µ, 250x50; eluent A: CO2; eluent B: methanol + 0.2 vol % aqueous ammonia (32%); isocratic: 30%B; flow: 100 mL/min; temperature: 40 °C; BPR: 150 bar; UV: 280 nm

### Analytical chiral HPLC method:

Instrument: Waters Acquity UPC2 QDA; Column: Chiralpak IG 3µ, 100x4.6; eluent A: CO2; eluent B: methanol + 0.2 vol % aqueous ammonia (32%); isocratic: 30%B; flow: 4 mL/min; temperature: 37.5 °C; BPR: 100 bar; UV: 280 nm
Analytical chiral HPLC: Rt = 0.84 min
[α]²⁰_{D}: +61.4° (c = 1.00, methanol)
LC-MS (Method 1): Rt = 0.93 min; MS (ESlpos): m/z = 448 [M+H]⁺
¹H NMR (400 MHz, DMSO-*d*₆) δ ppm: 11.77 (br s, 1H), 8.32 (d, 1H), 7.72 - 7.74 (m, 1H), 7.03 (br s, 1H), 6.81 (br s, 1H), 6.47 (s, 2H), 6.29 (s, 1H), 4.08 (t, 2H), 3.52 (dd, 1H), 2.73 - 2.82 (m, 2H), 2.53 - 2.64 (m, 2H), 2.37 - 2.47 (m, 1H), 1.73 - 1.98 (m, 4H), 0.73 (t, 3H).

### Example 757

### 5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 2)

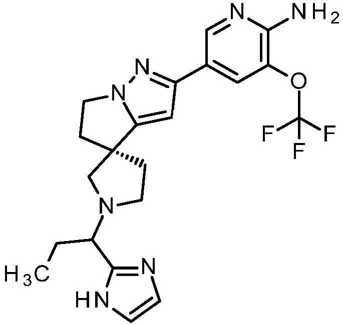

For the preparation of the diastereomeric mixture of the title compound see **Example 755** The diastereomers were separated by preparative chiral HPLC (method see **Example 756)** to give 54.7 mg (100 % purity) of the title compound (diastereomer 2 Rₜ = 5.02 - 7.00 min).
Analytical chiral HPLC (method see **Example 756):** Rₜ = 1.37 min.
[α]²⁰_{D}: +39.2° (c = 1.00, methanol)
LC-MS (Method 1): Rt = 0.93 min; MS (ESlpos): m/z = 448 [M+H]⁺
¹H NMR (400 MHz, DMSO-*d*₆) δ ppm: 11.78 (br s, 1H), 8.31 (d, 1H), 7.70 - 7.74 (m, 1H), 7.04 (br s, 1H), 6.81 (br s, 1H), 6.47 (s, 2H), 6.24 (s, 1H), 4.00 - 4.12 (m, 2H), 3.53 (dd, 1H), 2.81 - 2.89 (m, 1H), 2.64 - 2.69 (m, 1H), 2.55 - 2.63 (m, 2H), 2.38 - 2.47 (m, 1H), 1.72 - 1.96 (m, 4H), 0.74 (t, 3H).

### Example 758

### 5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

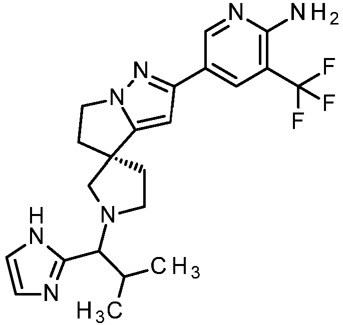

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (618 mg, 57 % purity, 979 µmol) and 1-(1H-imidazol-2-yl)-2-methylpropan-1-one (203 mg, 1.47 mmol) were dissolved in methanol (10 mL). N,N-Diisopropylethylamine (680 µL, 3.9 mmol) and titanium(IV) isopropoxide (870 µL, 2.9 mmol) were added and the mixture was stirred for 3 h at 60 °C. The reaction mixture was allowed to cool down, sodium cyanoborohydride (185 mg, 2.94 mmol) was added and stirred over night at 60 °C. The reaction mixture was allowed to cool down, treated with water (2 mL) and stirred for 15 min. The suspension was filtered over celite and the filter cake was washed with methanol. The filtrate was concentrated. The crude product was purified by column chromatography (silica gel, hexane / ethyl acetate (0-100%) and dichloromethane / methanol (0-10%) gradient). 24 mg of the combined fractions were purified by preparative HPLC to give 8.00 mg (95 % purity, 2 % yield) of the title compound.
LC-MS (Method 1): Rt = 1.03 min; MS (ESlpos): m/z = 447 [M+H]⁺
¹H-NMR (400 MHz, DMSO-d6) δ [ppm]: 0.673 (11.60), 0.684 (12.64), 0.689 (13.35), 0.700 (11.15), 0.962 (10.62), 0.976 (15.21), 0.977 (15.31), 0.991 (11.44), 1.839 (0.57), 1.851 (1.45), 1.868 (3.68), 1.886 (5.84), 1.903 (3.13), 1.914 (1.46), 1.925 (0.61), 1.945 (0.42), 2.074 (1.76), 2.127 (0.56), 2.144 (1.50), 2.160 (2.12), 2.166 (1.81), 2.177 (1.74), 2.182 (2.17), 2.199 (1.48), 2.216 (0.53), 2.323 (1.00), 2.327 (1.39), 2.332 (0.99), 2.363 (0.50), 2.380 (1.23), 2.394 (1.41), 2.412 (2.55), 2.422 (1.62), 2.430 (1.43), 2.441 (2.13), 2.449 (1.49), 2.458 (1.71), 2.466 (3.53), 2.518 (6.70), 2.523 (4.31), 2.561 (0.86), 2.580 (4.90), 2.602 (6.58), 2.621 (2.34), 2.632 (2.09), 2.642 (1.00), 2.655 (5.09), 2.669 (6.50), 2.692 (1.70), 2.779 (0.87), 2.793 (4.47), 2.799 (3.01), 2.815 (5.90), 2.837 (1.92), 2.854 (0.67), 3.349 (8.28), 3.368 (5.06), 3.372 (5.12), 4.064 (4.76), 4.081 (9.92), 4.099 (4.84), 6.221 (16.00), 6.271 (15.66), 6.515 (13.98), 6.866 (0.88), 7.026 (0.88), 7.981 (8.47), 8.558 (8.13), 11.719 (1.97).

### Example 759

### 5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 1)

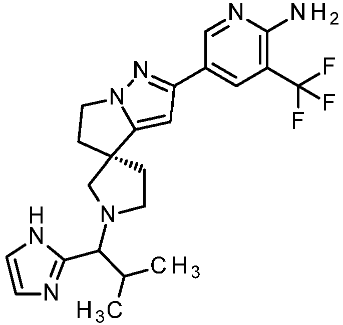

The title compound from **Example 758** was separated into diastereomers by preparative chiral HPLC (diastereomer 1 Rt = 5.71 - 7.08 min).

### Preparative chiral HPLC method:

Instrument: PrepCon Labomatic HPLC-2; Column: Chiralpak IG 5µ, 250x50; eluent A: hexane + 0.1 vol % diethylamine; eluent B: ethanol; isocratic: 20%B; flow: 70 mL/min; temperature: 25 °C; UV: 280 nm

### Analytical chiral HPLC method:

Instrument: Thermo Fisher UltiMate 3000; Column: Chiralpak IG 3µ, 100x4.6; eluent A: hexane + 0.1 vol % diethylamine; eluent B: ethanol; isocratic: 20%B; flow: 1.4 mL/min; temperature: 25 °C; UV: 280 nm
Analytical chiral HPLC: Rₜ = 3.19 min

The crude fraction was purified by preparative HPLC to give 13.0 mg (99 % purity, 16 % yield) of the title compound.
[α]²⁰_{D}: +36.6° (c = 1.00, DMSO)
LC-MS (Method 1): Rt = 1.03 min; MS (ESlpos): m/z = 447 [M+H]⁺
¹H-NMR (400 MHz, DMSO-d6) δ [ppm]: 0.683 (6.44), 0.700 (6.54), 0.962 (6.22), 0.979 (6.40), 1.851 (0.75), 1.865 (0.86), 1.870 (0.87), 1.878 (0.80), 1.885 (0.77), 1.893 (0.89), 1.898 (0.95), 1.914 (0.72), 2.142 (0.50), 2.159 (0.69), 2.165 (0.57), 2.175 (0.55), 2.182 (0.71), 2.198 (0.48), 2.380 (0.78), 2.394 (0.79), 2.412 (1.37), 2.429 (0.66), 2.449 (0.70), 2.465 (1.68), 2.518 (1.73), 2.523 (1.19), 2.579 (2.01), 2.602 (2.79), 2.622 (0.99), 2.642 (0.44), 2.779 (0.52), 2.793 (2.62), 2.799 (1.32), 2.815 (2.45), 3.334 (16.00), 3.344 (3.17), 3.367 (2.38), 4.064 (1.86), 4.081 (3.82), 4.099 (1.80), 6.271 (9.09), 6.515 (4.53), 6.851 (1.26), 7.038 (1.21), 7.980 (2.60), 7.985 (2.61), 8.558 (2.39), 8.562 (2.36), 11.723 (1.02).

### Example 760

### 5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 2)

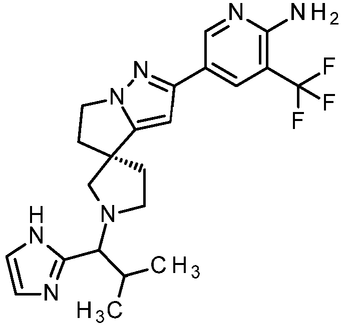

For the preparation of the diastereomeric mixture of the title compound see **Example 758.** The diastereomers were separated by preparative chiral HPLC (method see **Example 759)** (diastereomer 2 Rt = 10.03 - 13.90 min).
Analytical chiral HPLC (method see **Example 759):** Rt = 6.44 min

The crude fraction was purified by preparative HPLC to give 14.0 mg (99 % purity, 17 % yield) of the title compound.
[α]²⁰_{D}: +27.8° (c = 1.00, DMSO)
LC-MS (Method 1): Rt = 1.03 min; MS (ESlpos): m/z = 447 [M+H]⁺
¹H-NMR (400 MHz, DMSO-d6) δ [ppm]: 0.672 (4.91), 0.689 (5.00), 0.975 (4.85), 0.992 (4.97), 1.867 (1.06), 1.886 (2.06), 1.903 (1.14), 2.161 (0.52), 2.166 (0.44), 2.177 (0.43), 2.183 (0.54), 2.408 (0.46), 2.421 (0.62), 2.440 (0.87), 2.457 (0.64), 2.468 (0.76), 2.518 (1.77), 2.523 (1.12), 2.579 (0.74), 2.601 (0.89), 2.632 (0.77), 2.655 (2.24), 2.669 (2.64), 2.692 (0.74), 2.815 (0.83), 2.832 (0.51), 2.838 (0.65), 3.334 (16.00), 3.348 (2.09), 3.371 (1.86), 4.061 (0.90), 4.066 (0.87), 4.079 (1.72), 4.096 (0.88), 4.100 (0.89), 6.221 (7.58), 6.518 (3.50), 6.843 (0.84), 7.053 (0.81), 7.976 (2.02), 7.981 (2.03), 8.553 (1.87), 8.557 (1.82), 11.707 (0.83).

### Example 761

### 5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

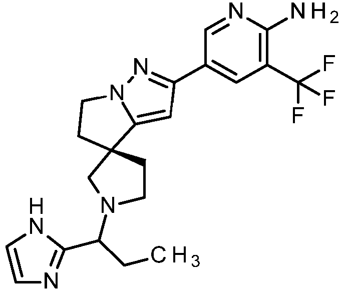

5-[(3S)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (400 mg, 85 % purity, 945 µmol) and 1-(1H-imidazol-2-yl)propan-1-one (176 mg, 1.42 mmol) were dissolved in methanol (10 mL). N,N-Diisopropylethylamine (660 µL, 3.8 mmol) and titanium(IV) isopropoxide (840 µL, 2.8 mmol) were added and the mixture was stirred for 3 h at 60 °C. To the cold reaction mixture was added sodium cyanoborohydride (178 mg, 2.84 mmol) and stirred over night at 60 °C. The cold reaction mixture was treated with water (2 mL) and stirred for 15 min. The suspension was filtered over celite and the filter cake was washed with methanol. The filtrate was concentrated. The crude product was purified by column chromatography (silica gel, hexane / ethyl acetate (0-100%) and dichloromethane / methanol (0-5%) gradient). 46 mg of the combined fractions were purified by preparative HPLC to give 23.0 mg (99 % purity, 6 % yield) of the title compound.
LC-MS (Method 1): Rt = 0.93 min; MS (ESlpos): m/z = 433 [M+H]⁺
¹H-NMR (400 MHz, DMSO-d6) δ [ppm]: 0.712 (5.21), 0.719 (5.18), 0.731 (12.53), 0.737 (11.24), 0.749 (5.81), 0.756 (4.97), 1.758 (0.56), 1.776 (1.00), 1.791 (1.67), 1.809 (2.26), 1.828 (2.36), 1.847 (2.49), 1.862 (2.08), 1.865 (2.13), 1.885 (1.89), 1.906 (3.91), 1.922 (5.72), 1.941 (3.32), 1.973 (0.42), 2.318 (0.47), 2.323 (1.04), 2.327 (1.53), 2.331 (1.02), 2.337 (0.45), 2.388 (0.49), 2.397 (0.51), 2.406 (1.20), 2.420 (1.43), 2.438 (2.14), 2.449 (1.79), 2.454 (1.47), 2.466 (2.19), 2.518 (6.47), 2.523 (4.94), 2.563 (4.19), 2.581 (4.20), 2.594 (1.90), 2.605 (5.62), 2.629 (0.90), 2.654 (3.72), 2.665 (1.40), 2.669 (1.84), 2.677 (2.60), 2.744 (0.80), 2.765 (1.64), 2.780 (1.40), 2.787 (1.15), 2.796 (3.52), 2.819 (2.94), 2.833 (0.76), 2.848 (1.19), 2.871 (1.22), 2.888 (0.54), 3.497 (1.72), 3.512 (2.54), 3.521 (2.88), 3.533 (2.11), 3.544 (1.47), 4.039 (0.52), 4.047 (1.48), 4.065 (4.62), 4.073 (2.72), 4.082 (6.57), 4.093 (2.11), 4.100 (2.99), 6.293 (13.25), 6.336 (16.00), 6.518 (13.52), 6.810 (5.89), 7.041 (3.85), 7.985 (4.26), 7.990 (7.70), 7.994 (4.68), 8.564 (3.76), 8.570 (6.77), 11.776 (3.19).

### Example 762

### 5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)

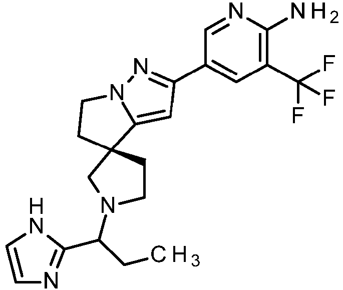

The title compound from **Example 761** was separated into diastereomers by preparative chiral HPLC (diastereomer 1 Rt = 4.60 - 5.75 min).

### Preparative chiral HPLC method:

Instrument: Sepiatec: Prep SFC100; Column: Chiral Art Amylos-SA 5µ, 250x50; eluent A: CO2; eluent B: 2-propanol + 0.4 vol % diethylamine; isocratic: 20%B; flow: 100 mL/min; temperature: 40 °C; BPR: 150 bar; UV: 265 nm

### Analytical chiral HPLC method:

Instrument: Waters Acquity UPC2 QDA; Column: Chiral Art Amylose-SA 3µ, 100x4.6; eluent A: CO2; eluent B: 2-propanol + 0.4 vol % diethylamine; isocratic: 20%B; flow: 4 mL/min; temperature: 40 °C; BPR: 1800 psi; UV: 254 nm
Analytical chiral HPLC: Rₜ = 2.75 min

The crude fraction was purified by preparative HPLC to give 25.0 mg (99 % purity, 17 % yield) of the title compound.
[α]²⁰_{D}: -34.9° (c = 1.00, DMSO)
LC-MS (Method 1): Rt = 0.93 min; MS (ESlpos): m/z = 433 [M+H]⁺
¹H-NMR (400 MHz, DMSO-d6) δ [ppm]: 0.713 (5.73), 0.731 (13.41), 0.749 (6.14), 1.758 (0.52), 1.777 (0.77), 1.792 (1.38), 1.813 (1.78), 1.829 (1.90), 1.844 (1.77), 1.862 (1.97), 1.886 (1.82), 1.906 (3.15), 1.922 (3.02), 1.941 (1.64), 1.953 (0.63), 1.973 (0.45), 2.388 (0.64), 2.406 (1.44), 2.420 (1.72), 2.438 (2.46), 2.455 (1.61), 2.477 (2.78), 2.518 (5.71), 2.523 (4.47), 2.564 (4.36), 2.593 (1.80), 2.611 (1.99), 2.630 (0.92), 2.745 (0.96), 2.766 (2.06), 2.782 (1.83), 2.788 (1.58), 2.797 (4.33), 2.820 (3.46), 3.499 (2.01), 3.513 (2.56), 3.521 (2.18), 3.535 (1.92), 4.065 (3.44), 4.083 (6.48), 4.100 (3.34), 6.336 (16.00), 6.516 (9.43), 6.927 (0.61), 7.989 (5.26), 7.995 (5.34), 8.570 (4.95), 8.574 (4.91), 11.782 (0.68).

### Example 763

### 5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)

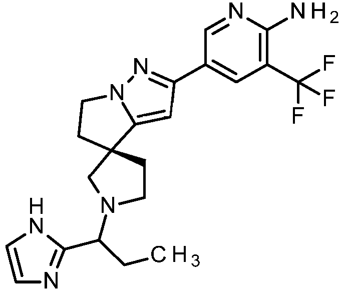

For the preparation of the diastereomeric mixture of the title compound see **Example 761.** The diastereomers were separated by preparative chiral HPLC (method see **Example 762)** (diastereomer 2 Rt = 6.28 - 7.60 min).
Analytical chiral HPLC (method see **Example 762):** Rt = 4.53 min

The crude fraction was purified by preparative HPLC to give 22.0 mg (99 % purity, 15 % yield) of the title compound.
[α]²⁰_{D}: -55.8° (c = 1.00, DMSO)
LC-MS (Method 1): Rt = 0.93 min; MS (ESlpos): m/z = 433 [M+H]⁺
¹H-NMR (400 MHz, DMSO-d6) δ [ppm]: 0.719 (5.13), 0.738 (12.15), 0.755 (5.42), 1.752 (0.57), 1.770 (0.68), 1.786 (1.19), 1.808 (1.36), 1.827 (1.43), 1.831 (1.27), 1.846 (1.54), 1.865 (1.65), 1.884 (0.91), 1.904 (2.10), 1.922 (4.52), 1.941 (2.59), 2.322 (0.72), 2.326 (1.03), 2.331 (0.73), 2.397 (0.48), 2.416 (0.89), 2.429 (1.54), 2.449 (1.95), 2.466 (2.54), 2.518 (5.09), 2.522 (3.29), 2.562 (0.90), 2.581 (4.75), 2.604 (6.16), 2.622 (0.81), 2.654 (4.48), 2.664 (1.08), 2.668 (1.32), 2.677 (2.96), 2.833 (0.80), 2.848 (1.46), 2.871 (1.50), 2.888 (0.65), 3.508 (1.79), 3.521 (2.30), 3.530 (1.93), 3.544 (1.71), 4.038 (0.57), 4.047 (1.73), 4.062 (2.67), 4.066 (2.67), 4.073 (2.45), 4.078 (2.48), 4.092 (1.66), 4.100 (0.55), 4.104 (0.51), 6.292 (16.00), 6.516 (8.16), 6.808 (3.04), 7.045 (2.76), 7.984 (4.57), 7.989 (4.60), 8.563 (4.28), 8.567 (4.13), 11.778 (1.85).

### Example 764

### 5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

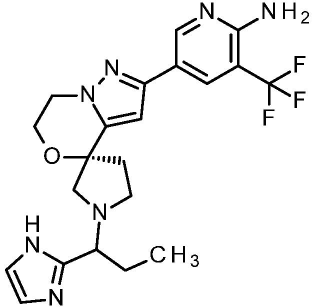

The compound was prepared similarly to **Example 761** using 5-[(3'R)-6,7-dihydrospiro[pyrazolo[5,1-c][1,4]oxazine-4,3'-pyrrolidin]-2-yl]-3-(trifluoromethyl)pyridin-2-amine hydrogen chloride (1/1) (475 mg, 70 % purity, 885 µmol) and 1-(1H-imidazol-2-yl)propan-1-one (165 mg, 1.33 mmol) to give 144 mg (36% yield). 34 mg were purified by HPLC to yileld 18.0 mg (99 % purity, 5 % yield) of the title compound.
LC-MS (Method 1): Rt = 0.90 min; MS (ESlpos): m/z = 449 [M+H]⁺
¹H-NMR (400 MHz, DMSO-d6) δ [ppm]: 0.683 (5.64), 0.691 (4.40), 0.702 (13.52), 0.710 (9.13), 0.720 (6.32), 0.728 (3.95), 1.748 (0.58), 1.767 (0.91), 1.781 (1.38), 1.790 (1.12), 1.800 (1.80), 1.823 (2.00), 1.836 (1.82), 1.850 (2.13), 1.868 (2.35), 1.887 (1.14), 1.902 (0.66), 2.029 (0.73), 2.038 (1.21), 2.043 (1.10), 2.052 (1.47), 2.061 (1.55), 2.071 (2.43), 2.090 (2.03), 2.103 (1.90), 2.121 (2.17), 2.134 (1.32), 2.152 (0.76), 2.518 (5.58), 2.523 (3.87), 2.575 (2.07), 2.597 (2.31), 2.696 (3.46), 2.721 (3.82), 2.773 (2.11), 2.791 (1.72), 2.799 (3.16), 2.811 (1.36), 2.830 (1.32), 2.852 (1.02), 2.879 (2.80), 2.905 (1.86), 3.031 (3.44), 3.057 (2.99), 3.472 (2.33), 3.485 (2.82), 3.495 (2.40), 3.509 (2.15), 3.951 (0.83), 3.965 (2.17), 3.978 (3.65), 3.990 (5.14), 4.004 (3.84), 4.048 (5.00), 4.057 (5.78), 4.062 (5.22), 6.483 (10.69), 6.523 (16.00), 6.575 (12.21), 6.801 (5.59), 6.804 (4.95), 6.808 (3.99), 7.034 (4.22), 7.052 (2.72), 8.018 (7.05), 8.593 (3.28), 8.599 (6.96), 8.603 (4.65), 11.833 (3.53).

### Example 765

### 5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)

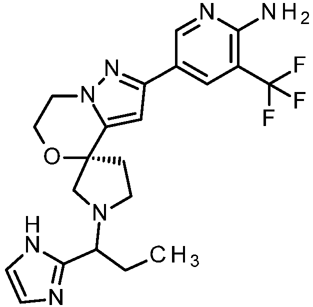

The title compound from **Example 764** was separated into diastereomers by preparative chiral HPLC (diastereomer 1 Rt = 3.03 - 3.79 min).

### Preparative chiral HPLC method:

Instrument: Sepiatec: Prep SFC100; Column: Chiral Art Amylos-SA 5µ, 250x50; eluent A: CO2; eluent B: 2-propanol + 0.4 vol % diethylamine; isocratic: 30%B; flow: 100 mL/min; temperature: 40 °C; BPR: 150 bar; UV: 265 nm

### Analytical chiral HPLC method:

Instrument: Waters Acquity UPC2 QDA; Column: Chiral Art Amylose-SA 3µ, 100x4.6; eluent A: CO2; eluent B: 2-propanol + 0.4 vol % diethylamine; isocratic: 30%B; flow: 4 mL/min; temperature: 40 °C; BPR: 1800 psi; UV: 254 nm
Analytical chiral HPLC: Rt = 0.86 min

The crude fraction was purified by preparative HPLC to give 20.0 mg (99 % purity, 18 % yield) of the title compound.
[α]²⁰_{D}: +63.2° (c = 1.00, DMSO)
LC-MS (Method 1): Rt = 0.90 min; MS (ESlpos): m/z = 449 [M+H]⁺
¹H-NMR (400 MHz, DMSO-d6) δ [ppm]: 0.691 (5.04), 0.710 (12.02), 0.728 (5.31), 1.767 (0.41), 1.786 (0.60), 1.791 (0.52), 1.801 (1.18), 1.819 (1.32), 1.824 (1.42), 1.835 (1.32), 1.843 (1.34), 1.848 (1.45), 1.853 (1.25), 1.867 (1.54), 1.886 (0.71), 2.011 (0.45), 2.030 (0.88), 2.043 (1.22), 2.061 (1.70), 2.074 (0.55), 2.080 (0.96), 2.100 (0.82), 2.118 (1.43), 2.133 (1.47), 2.148 (0.71), 2.166 (0.46), 2.518 (2.93), 2.523 (2.02), 2.569 (0.68), 2.589 (1.36), 2.604 (1.39), 2.624 (0.67), 2.773 (2.51), 2.799 (3.57), 2.812 (0.88), 2.830 (1.77), 2.852 (1.50), 2.870 (0.80), 2.880 (4.15), 2.905 (2.78), 3.469 (1.76), 3.483 (2.27), 3.493 (1.80), 3.506 (1.70), 3.933 (0.44), 3.951 (1.15), 3.965 (2.94), 3.978 (3.30), 3.990 (2.01), 4.012 (0.55), 4.019 (0.63), 4.043 (3.09), 4.054 (5.05), 4.068 (1.79), 6.483 (16.00), 6.574 (7.66), 6.810 (1.70), 7.052 (1.63), 8.015 (4.50), 8.020 (4.60), 8.593 (4.21), 8.597 (4.21), 11.837 (1.43).

### Example 766

### 5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer2)

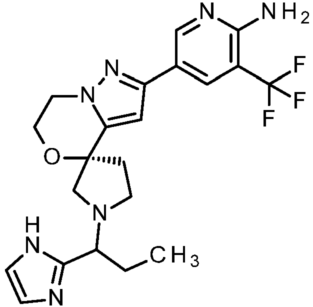

For the preparation of the diastereomeric mixture of the title compound see **Example 764.** The diastereomers were separated by preparative chiral HPLC (method see **Example 765)** (diastereomer 2 Rt = 4.43 - 5.62 min).
Analytical chiral HPLC (method see **Example 765):** Rt = 1.70 min

The crude fraction was purified by preparative HPLC (basic method) to give 30.0 mg (99 % purity, 27 % yield) of the title compound.
[α]²⁰_{D}: +43.9° (c = 1.00, DMSO)
LC-MS (Method 1): Rt = 0.90 min; MS (ESlpos): m/z = 449 [M+H]⁺
¹H-NMR (400 MHz, DMSO-d6) δ [ppm]: 0.684 (5.19), 0.702 (12.43), 0.720 (5.46), 1.748 (0.50), 1.767 (0.67), 1.772 (0.62), 1.781 (1.10), 1.789 (0.75), 1.799 (1.05), 1.805 (1.17), 1.823 (1.07), 1.837 (1.03), 1.850 (1.24), 1.855 (1.08), 1.869 (1.48), 1.888 (0.76), 1.902 (0.44), 2.038 (0.94), 2.052 (1.19), 2.071 (2.18), 2.090 (1.88), 2.107 (1.53), 2.121 (1.48), 2.137 (0.60), 2.154 (0.40), 2.518 (2.75), 2.523 (2.08), 2.575 (1.75), 2.597 (1.88), 2.615 (0.67), 2.696 (3.37), 2.722 (3.75), 2.770 (0.78), 2.784 (1.11), 2.789 (1.39), 2.805 (1.32), 2.825 (0.58), 3.031 (3.39), 3.056 (2.92), 3.473 (1.80), 3.487 (2.21), 3.497 (1.87), 3.510 (1.72), 3.991 (3.81), 4.004 (3.60), 4.048 (3.72), 4.058 (3.55), 4.062 (4.12), 4.074 (1.69), 6.523 (16.00), 6.571 (7.70), 6.801 (3.81), 7.034 (3.30), 8.018 (4.67), 8.023 (4.68), 8.599 (4.36), 8.603 (4.27), 11.832 (2.07).

### Example 767

### {(3R)-2'-[6-amino-5-(trifluoromethyl)pyridin-3-yl]-5',6'-dihydrospiro[pyrrolidine-3,4'-pyrrolo[1,2-b]pyrazol]-1-yl}(1H-imidazol-2-yl)methanone

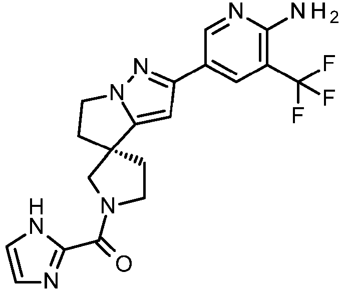

1H-Imidazole-2-carboxylic acid (128 mg, 1.14 mmol) was dissolved in DMF (6 mL). N,N-Diisopropylethylamine (500 µL, 2.8 mmol) and HATU (650 mg, 1.71 mmol) were added and the mixture was stirred for 30 min at room temperature. 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (250 mg, 82 % purity, 570 µmol) was added and stirred over the weekend at room temperature. 1H-Imidazole-2-carboxylic acid (128 mg, 1.14 mmol) and HATU (433 mg, 1.14 mmol) were added again and the mixture was stirred for 5 h at 60 °C. The reaction mixture was allowed to cool down, diluted with water and brine. The aqueous phase was extracted with ethyl acetate. The combined organic layers were dried over sodium sulfate, filtered and concentrated. The residue was purified by column chromatography (silica gel, dichloromethane / methanol (0-5%) gradient) followed by preparative HPLC to give 28.0 mg (98 % purity, 12 % yield) of the title compound.
LC-MS (Method 1): Rt = 0.87 min; MS (ESlpos): m/z = 419 [M+H]⁺
¹H-NMR (400 MHz, DMSO-d6) δ [ppm]: 2.074 (3.84), 2.097 (1.21), 2.116 (2.35), 2.133 (1.15), 2.181 (1.22), 2.198 (2.60), 2.216 (1.23), 2.318 (0.99), 2.323 (2.26), 2.327 (3.17), 2.331 (2.23), 2.337 (0.99), 2.518 (12.18), 2.523 (8.68), 2.567 (0.82), 2.582 (1.34), 2.600 (3.68), 2.617 (4.12), 2.633 (2.76), 2.649 (1.31), 2.660 (1.25), 2.665 (2.77), 2.669 (3.39), 2.673 (2.24), 2.678 (1.05), 3.705 (0.97), 3.735 (5.04), 3.754 (0.61), 3.799 (0.58), 3.816 (1.24), 3.830 (0.84), 3.846 (0.75), 4.189 (2.49), 4.207 (6.64), 4.224 (3.42), 4.238 (3.75), 4.260 (3.64), 4.277 (0.77), 4.290 (1.74), 4.294 (1.81), 4.313 (1.67), 4.329 (0.68), 6.525 (16.00), 6.530 (9.87), 6.539 (10.40), 7.046 (4.49), 7.048 (4.52), 7.132 (3.20), 7.253 (3.86), 7.282 (2.76), 8.021 (3.36), 8.027 (4.71), 8.033 (2.45), 8.589 (3.07), 8.595 (3.77), 8.602 (2.18), 12.965 (1.24).

### Example 768

### {(3R)-2'-[6-amino-5-(trifluoromethyl)pyridin-3-yl]-5',6'-dihydrospiro[pyrrolidine-3,4'-pyrrolo[1,2-b]pyrazol]-1-yl}(1-methyl-1H-imidazol-2-yl)methanone

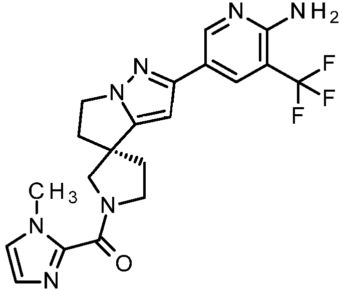

1-Methyl-1H-imidazole-2-carboxylic acid (65.7 mg, 521 µmol) was dissolved in DMF (3.5 mL). N,N-Diisopropylethylamine (240 µL, 1.4 mmol) and HATU (264 mg, 695 µmol) were added and the mixture was stirred for 30 min at room temperature. 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (125 mg, 347 µmol) was added and stirred for 1 h at room temperature. The reaction mixture was diluted with water and extracted twice with ethyl acetate. The combined organic layers were dried over sodium sulfate, filtered and concentrated. The residue was purified by preparative HPLC to give 21.0 mg (99 % purity, 14 % yield) of the title compound.
LC-MS (Method 1): Rt = 0.89 min; MS (ESlpos): m/z = 433 [M+H]⁺
¹H NMR (400 MHz, DMSO-*d*₆) δ ppm: 2.08 - 2.19 (m, 2H), 2.52 - 2.65 (m, 2H), 3.65 - 3.84 (m, 2H), 3.87 (s, 3H), 4.01 - 4.27 (m, 4H), 6.52 - 6.55 (m, 3H) 6.96 and 7.04 (2d, 1H) 7.31 and 7.35 (2d, 1H), 8.01 - 8.04 (m, 1 H), 8.58 - 8.61 (m, 1 H).

### Example 769

### 3-[(1R)-1-(2,6-dichloro-3-fluorophenyl)ethoxy]-5-{(3R)-1-[(1H-imidazol-2-yl)methyl]-5',6'-dihydrospiro[pyrrolidine-3,4'-pyrrolo[1,2-b]pyrazol]-2'-yl}pyridin-2-amine

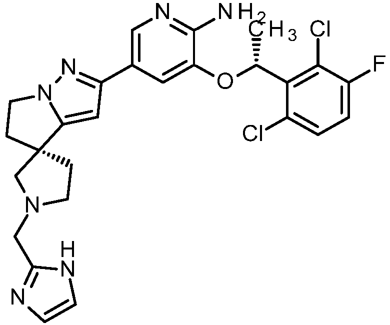

3-[(1R)-1-(2,6-Dichloro-3-fluorophenyl)ethoxy]-5-[(3R)-5',6'-dihydrospiro[pyrrolidine-3,4'-pyrrolo[1,2-b]pyrazol]-2'-yl]pyridin-2-amine hydrogen chloride (1/1) (270 mg, 541 µmol) and 1H-imidazole-2-carbaldehyde (78.0 mg, 812 µmol) were dissolved in THF (5.5 mL). Acetic acid (46 µL) was added and the mixture was stirred for 30 min at room temperature. Sodium triacetoxyborohydride (229 mg, 1.08 mmol) was added and stirred over night at room temperature. The reaction mixture was diluted with water and extracted with ethyl acetate. The combined organic layers were washed with brine, dried over sodium sulfate, filtered and concentrated. The residue was purified by preparative HPLC. The fraction was dissolved in dichloromethane and basified with 1M sodium hydoxide solution. The organic phase was concentrated to give 5.50 mg (95 % purity, 2 % yield) of the title compound.
LC-MS (Method 1): Rt = 1.06 min; MS (ESlpos): m/z = 542 [M+H]⁺
¹H NMR (400 MHz, DMSO-*d*₆) δ ppm: 8.16 (s, 1H), 7.88 (d, 1H), 7.55 (dd, 1H), 7.40 - 7.47 (m, 1H), 7.12 (d, 1H), 6.91 (br s, 2H), 6.17 (s, 1H), 6.05 (q, 1H), 3.98 - 4.09 (m, 2H), 3.67 (s, 2H), 2.53 - 2.82 (m, 5H), 2.41 - 2.47 (m, 1H), 1.92 - 2.04 (m, 2H), 1.78 (d, 3H).

### Example 770

### 5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)

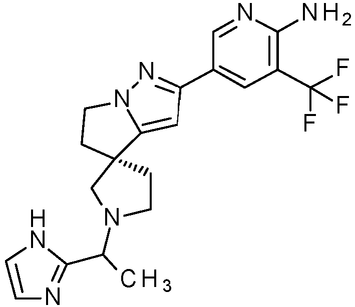

The diastereomeric mixture of compound 5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (35.0 mg, 83.8 µmol) was separated into diastereomers by preparative chiral HPLC to give 14.0 mg (90 % purity, 36 % yield) of the title compound (diastereomer 1 Rt = 7.1 - 8.9 min).

### Preparative chiral HPLC method:

Instrument: PrepCon Labomatic HPLC-1; Column: Chiralpak IG 5µ, 250x50; eluent A: hexane + 0.1 vol % diethylamine; eluent B: ethanol; isocratic: 30%B; flow: 50 mL/min; temperature: 25 °C; UV: 254 nm

### Analytical chiral HPLC method:

Instrument: Thermo Fisher UltiMate 3000; Column: Chiralpak IG 3µ, 100x4.6; eluent A: hexane + 0.1 vol % diethylamine; eluent B: ethanol; isocratic: 30%B; flow: 1.4 mL/min; temperature: 25 °C; UV: 254 nm
Analytical chiral HPLC: Rₜ = 2.65 min
LC-MS (Method 1): Rt = 0.87 min; MS (ESlpos): m/z = 419 [M+H]⁺
¹H-NMR (400 MHz, DMSO-d6) δ [ppm]: 0.776 (0.41), 0.794 (0.99), 0.813 (0.61), 0.819 (0.43), 0.836 (0.51), 0.861 (0.54), 1.005 (0.89), 1.034 (0.76), 1.084 (1.64), 1.204 (0.50), 1.231 (1.42), 1.259 (3.24), 1.367 (11.57), 1.384 (11.92), 1.907 (0.71), 1.919 (1.33), 1.939 (2.35), 1.955 (2.46), 1.960 (2.01), 1.976 (1.48), 1.987 (0.55), 1.993 (0.57), 2.007 (0.43), 2.414 (0.69), 2.432 (1.32), 2.446 (1.55), 2.465 (2.10), 2.481 (1.94), 2.518 (2.07), 2.523 (1.78), 2.530 (4.98), 2.546 (1.92), 2.554 (4.06), 2.560 (1.78), 2.577 (1.26), 2.591 (1.10), 2.598 (1.41), 2.614 (1.68), 2.633 (0.81), 2.717 (0.92), 2.737 (1.80), 2.754 (1.45), 2.760 (1.06), 2.776 (0.68), 2.794 (3.74), 2.817 (3.19), 3.675 (0.85), 3.692 (3.36), 3.709 (3.31), 3.726 (0.82), 4.071 (2.64), 4.088 (5.06), 4.107 (2.60), 6.399 (16.00), 6.519 (8.07), 6.900 (0.88), 7.996 (4.50), 8.002 (4.57), 8.579 (4.23), 8.583 (4.12), 10.856 (0.45), 11.771 (0.49).

### Example 771

### 5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)

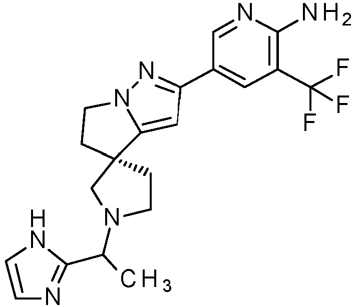

The diastereomeric mixture of compound 5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (35.0 mg, 83.8 µmol) was separated into diastereomers by preparative chiral HPLC (method see **Example 770)** to give 3.00 mg (90 % purity, 8 % yield) of the title compound (diastereomer 2 Rt = 14.4 - 18.9 min).
Analytical chiral HPLC (method see **Example 770):** Rt = 4.38 min
LC-MS (Method 1): Rt = 0.87 min; MS (ESlpos): m/z = 419 [M+H]⁺
¹H-NMR (400 MHz, DMSO-d6) δ [ppm]: 0.776 (1.31), 0.795 (2.80), 0.813 (1.82), 0.819 (1.28), 0.836 (1.39), 0.843 (1.08), 0.855 (0.76), 0.862 (1.56), 0.871 (0.56), 0.879 (0.69), 0.886 (0.42), 0.904 (0.55), 0.916 (0.51), 0.933 (0.99), 0.951 (0.53), 0.973 (0.60), 1.005 (2.64), 1.012 (0.49), 1.028 (0.52), 1.034 (2.02), 1.049 (1.10), 1.084 (4.80), 1.100 (0.69), 1.142 (0.46), 1.173 (0.46), 1.204 (2.16), 1.232 (2.65), 1.259 (7.90), 1.270 (0.87), 1.278 (0.64), 1.286 (0.82), 1.295 (0.54), 1.303 (0.63), 1.316 (0.45), 1.352 (2.26), 1.366 (11.94), 1.383 (12.22), 1.405 (0.94), 1.421 (0.88), 1.425 (0.86), 1.440 (0.66), 1.444 (0.65), 1.489 (0.40), 1.527 (0.67), 1.543 (0.58), 1.572 (0.41), 1.592 (0.44), 1.609 (0.43), 1.758 (0.43), 1.774 (0.42), 1.788 (0.48), 1.906 (0.60), 1.922 (0.55), 1.936 (1.64), 1.943 (1.77), 1.953 (3.39), 1.963 (2.43), 1.972 (1.96), 1.977 (2.00), 2.323 (0.74), 2.327 (0.98), 2.332 (0.77), 2.418 (0.78), 2.437 (1.26), 2.450 (1.90), 2.466 (2.39), 2.470 (2.68), 2.518 (3.84), 2.523 (2.24), 2.530 (1.65), 2.543 (0.84), 2.548 (0.70), 2.564 (1.12), 2.576 (3.04), 2.584 (2.12), 2.599 (4.99), 2.625 (0.88), 2.652 (4.70), 2.665 (0.93), 2.669 (1.32), 2.675 (3.27), 2.824 (0.86), 2.843 (1.37), 2.861 (1.45), 2.879 (0.70), 3.691 (0.90), 3.708 (3.42), 3.725 (3.37), 3.742 (0.84), 4.021 (0.43), 4.040 (0.67), 4.048 (1.75), 4.063 (3.28), 4.067 (2.69), 4.077 (2.53), 4.082 (3.28), 4.097 (1.80), 4.104 (0.98), 4.123 (0.68), 5.758 (0.41), 6.370 (16.00), 6.435 (0.53), 6.518 (8.58), 6.793 (0.46), 6.997 (0.43), 7.994 (4.52), 7.999 (4.75), 8.575 (4.32), 8.579 (4.40), 10.855 (0.98), 11.780 (0.65).

### Example 772

### (3R)-2'-[6-amino-5-(trifluoromethyl)pyridin-3-yl]-N-[1-(3-chloropyridin-4-yl)cyclobutyl]-5',6'-dihydrospiro[pyrrolidine-3,4'-pyrrolo[1,2-b]pyrazole]-1-carboxamide

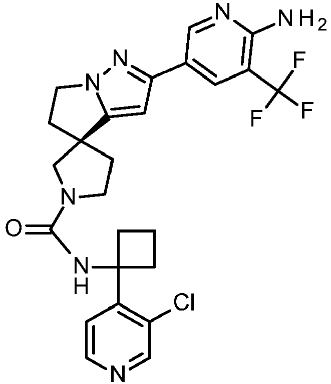

1-(3-Chloropyridin-4-yl)cyclobutan-1-amine (24.4 mg, 133 µmol), CDI (19.8 mg, 122 µmol) and N,N-diisopropylethylamine (120 µL, 670 µmol) were dissolved in DMF (1.5 mL) and stirred for 1 h at room temperature. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (50.0 mg, 80 % purity, 111 µmol) in DMF was added and the mixture was stirred over night at 60 °C. The reaction mixture was allowed to cool down, concentrated, treated with toluene and evaporated again. The residue was purified by preparative HPLC to give 2.80 mg (92 % purity, 4 % yield) of the title compound.
LC-MS (Method 1): Rt = 1.07 min; MS (ESlpos): m/z = 532 [M+H]⁺
¹H-NMR (400 MHz, CHLOROFORM-d) δ [ppm]: 0.812 (0.64), 0.989 (0.77), 1.005 (0.91), 1.110 (0.47), 1.185 (1.29), 1.752 (0.70), 1.762 (1.00), 1.774 (1.55), 1.779 (1.15), 1.790 (1.41), 1.797 (1.25), 1.802 (1.79), 1.814 (1.19), 1.824 (0.88), 1.837 (0.45), 1.978 (0.77), 1.997 (1.17), 2.010 (1.86), 2.025 (1.65), 2.041 (1.10), 2.088 (0.45), 2.109 (1.30), 2.130 (2.99), 2.150 (3.92), 2.157 (2.67), 2.170 (1.89), 2.179 (2.51), 2.199 (1.20), 2.450 (0.80), 2.467 (1.84), 2.483 (2.59), 2.500 (4.33), 2.516 (4.07), 2.526 (4.12), 2.531 (4.23), 2.536 (4.55), 2.549 (9.25), 2.557 (2.73), 2.567 (3.23), 2.581 (2.75), 2.598 (3.79), 2.632 (2.24), 2.645 (1.71), 2.665 (0.73), 2.975 (0.41), 3.035 (0.48), 3.374 (3.32), 3.398 (6.50), 3.425 (2.57), 3.431 (2.14), 3.443 (9.12), 3.449 (4.20), 3.467 (5.40), 3.516 (1.49), 3.529 (1.78), 3.536 (2.18), 3.549 (1.84), 3.560 (1.27), 3.574 (0.97), 4.172 (6.15), 4.189 (11.35), 4.207 (5.63), 4.929 (9.76), 5.150 (5.21), 6.086 (14.39), 6.116 (1.12), 6.929 (0.69), 7.451 (0.79), 7.465 (7.47), 7.477 (7.59), 8.021 (6.01), 8.026 (6.16), 8.044 (0.45), 8.404 (10.35), 8.417 (10.03), 8.427 (16.00), 8.510 (5.84), 8.514 (5.82).

### Example 773

### (3R)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide

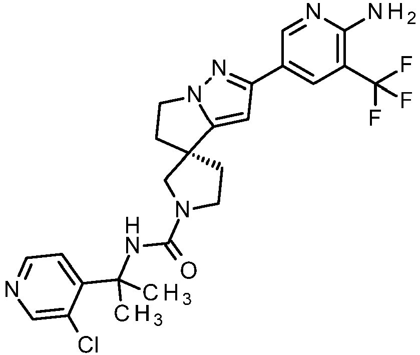

2-(3-Chloropyridin-4-yl)propan-2-amine (41.7 mg, 245 µmol), CDI (43.3 mg, 267 µmol) and N,N-diisopropylethylamine (150 µL, 890 µmol) were dissolved in DMF (1 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (100 mg, 80 % purity, 222 µmol) in DMF was added dropwise and the mixture was stirred over night at rt. The reaction mixture was poured into water and extracted three times with ethyl acetate. The combined organic layers were dried over a hydrophobic filter and concentrated. The residue was purified by column chromatography (silica gel, dichloromethane / methanol (0 - 8 %) gradient). The crude fraction was purified by preparative HPLC. The residue was dissolved in ethyl acetate and basified with a sodium bicarbonate solution. The aqueous phase was extracted three times with ethyl acetate. The combined organic layers were dried over a hydrophobic filter and concentrated to give 18.8 mg (90 % purity, 15 % yield) of the title compound.
LC-MS (Method 1): Rt = 1.03 min; MS (ESlpos): m/z = 520 [M+H]⁺
¹H NMR (400 MHz, CHLOROFORM-d) δ ppm: 0.009 (9.69), 1.192 (1.39), 1.567 (16.00), 1.620 (10.62), 1.738 (10.60), 1.742 (10.49), 2.046 (0.60), 2.060 (0.55), 2.166 (0.45), 2.185 (0.74), 2.202 (0.48), 2.214 (0.46), 2.492 (0.52), 2.507 (0.71), 2.526 (0.86), 2.542 (0.59), 2.564 (0.63), 2.581 (0.98), 2.584 (0.87), 2.600 (0.86), 2.633 (0.47), 3.415 (1.03), 3.440 (2.23), 3.476 (2.81), 3.488 (0.47), 3.495 (1.04), 3.500 (1.30), 3.512 (0.48), 3.541 (0.50), 3.556 (0.60), 3.561 (0.73), 3.566 (0.45), 3.576 (0.58), 3.580 (0.46), 4.192 (1.36), 4.211 (2.64), 4.227 (1.30), 4.229 (1.37), 4.753 (0.55), 4.843 (1.82), 4.944 (2.75), 6.150 (7.43), 6.176 (1.04), 7.218 (0.94), 7.232 (0.92), 7.349 (2.36), 7.361 (2.37), 7.432 (0.85), 7.445 (0.89), 8.045 (1.82), 8.050 (1.76), 8.276 (1.32), 8.290 (1.35), 8.364 (1.30), 8.380 (3.99), 8.393 (4.41), 8.432 (5.63), 8.459 (1.64), 8.539 (1.96), 8.542 (1.89).

### Example 774

### (3R)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide

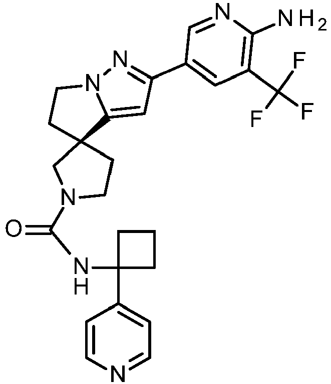

1-(Pyridin-4-yl)cyclobutan-1-amine (19.8 mg, 133 µmol), CDI (19.8 mg, 122 µmol) and N,N-diisopropylethylamine (120 µL, 670 µmol) were dissolved in DMF (1.5 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (50.0 mg, 80 % purity, 111 µmol) in DMF was added and the mixture was stirred for 2 h at 60 °C and 7 h at 100 °C. The reaction mixture was allowed to cool down, concentrated, treated with toluene and evaporated again. The residue was purified by preparative HPLC followed by preparative TLC (dichloromethane / methanol 9:1) to give 5.80 mg (96 % purity, 10 % yield) of the title compound.
LC-MS (Method 1): Rt = 0.97 min; MS (ESlpos): m/z = 498 [M+H]⁺
¹H-NMR (400 MHz, DMSO-d6) δ [ppm]: 0.835 (0.42), 0.852 (1.06), 0.868 (1.36), 0.887 (1.07), 0.906 (1.98), 0.924 (0.87), 1.233 (2.60), 1.279 (0.65), 1.290 (0.70), 1.353 (0.46), 1.411 (0.41), 1.986 (0.50), 2.006 (0.56), 2.024 (0.51), 2.065 (0.73), 2.078 (0.78), 2.327 (4.22), 2.331 (2.97), 2.337 (1.34), 2.375 (0.70), 2.390 (0.83), 2.413 (0.84), 2.518 (16.00), 2.523 (11.51), 2.669 (4.28), 2.673 (2.94), 2.678 (1.32), 3.445 (2.02), 4.174 (1.01), 4.191 (1.92), 4.208 (0.99), 4.228 (0.50), 4.234 (0.54), 4.242 (0.51), 4.249 (0.46), 5.760 (2.65), 6.459 (3.66), 6.558 (2.65), 6.871 (1.62), 7.391 (2.90), 7.395 (1.81), 7.402 (1.77), 7.406 (2.99), 8.009 (1.47), 8.014 (1.51), 8.089 (3.39), 8.458 (3.55), 8.462 (2.01), 8.469 (1.86), 8.473 (3.35), 8.584 (1.37), 8.588 (1.38).

### Example 775

### (3R)-2'-[6-amino-5-(trifluoromethyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide

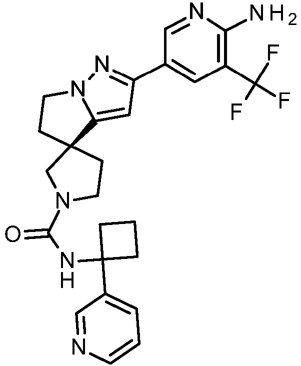

1-(Pyridin-3-yl)cyclobutan-1-amine hydrogen chloride (1/1) (24.6 mg, 133 µmol), CDI (19.8 mg, 122 µmol) and N,N-diisopropylethylamine (120 µL, 670 µmol) were dissolved in DMF (1.5 mL) and stirred for 2 h at rt. 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (50.0 mg, 80 % purity, 111 µmol) was added and the mixture was stirred for 1 h at 60 °C. CDI (9.92 mg, 61.1 µmol) and N,N-diisopropylethylamine (58 µL, 330 µmol) were added and the mixture was stirred for 3 h at 60° C. CDI (19.8 mg, 122 µmol) and 1-(pyridin-3-yl)cyclobutan-1-amine hydrogen chloride (1/1) (24.6 mg, 133 µmol) were added again and the mixture was stirred for 2 h at 60 °C. The reaction mixture was concentrated, treated with toluene and evaporated again. The residue was purified by preparative HPLC to give 3.50 mg (96 % purity, 6 % yield) of the title compound.
LC-MS (Method 1): Rt = 0.96 min; MS (ESlpos): m/z = 498 [M+H]⁺
¹H-NMR (400 MHz, CHLOROFORM-d) δ [ppm]: 1.814 (0.43), 1.831 (0.78), 1.837 (0.68), 1.843 (0.58), 1.849 (0.52), 1.854 (0.70), 1.860 (0.96), 1.877 (0.55), 1.882 (0.54), 1.990 (0.40), 2.008 (0.61), 2.022 (1.17), 2.037 (1.18), 2.045 (0.70), 2.052 (0.99), 2.060 (1.00), 2.066 (0.62), 2.075 (0.75), 2.082 (0.68), 2.088 (0.75), 2.097 (0.48), 2.103 (0.43), 2.111 (0.40), 2.143 (0.59), 2.161 (1.17), 2.174 (0.60), 2.180 (0.75), 2.192 (0.83), 2.211 (0.44), 2.431 (0.74), 2.452 (1.26), 2.462 (1.77), 2.470 (1.09), 2.479 (2.67), 2.484 (1.66), 2.495 (1.58), 2.502 (1.30), 2.512 (1.70), 2.529 (0.96), 2.545 (1.20), 2.548 (1.19), 2.554 (1.24), 2.562 (2.37), 2.568 (1.66), 2.576 (2.14), 2.579 (2.46), 2.591 (1.60), 2.595 (2.15), 3.409 (1.30), 3.434 (3.15), 3.444 (1.29), 3.451 (1.03), 3.463 (4.57), 3.469 (2.11), 3.488 (2.29), 3.537 (0.66), 3.550 (0.82), 3.557 (0.99), 3.561 (0.71), 3.570 (0.85), 3.575 (0.71), 3.581 (0.58), 3.595 (0.43), 4.177 (2.92), 4.194 (5.30), 4.212 (2.69), 4.779 (2.82), 4.949 (4.62), 6.102 (8.82), 7.187 (1.60), 7.195 (16.00), 7.205 (1.77), 7.207 (1.71), 7.217 (1.68), 7.219 (1.63), 7.771 (1.34), 7.775 (1.66), 7.781 (1.41), 7.790 (1.27), 7.795 (1.55), 7.796 (1.56), 7.800 (1.25), 8.025 (2.89), 8.029 (2.94), 8.398 (2.36), 8.401 (2.47), 8.410 (2.37), 8.413 (2.29), 8.515 (2.78), 8.519 (2.76), 8.617 (2.99), 8.621 (2.95).

### Example 776

### (3R)-2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide

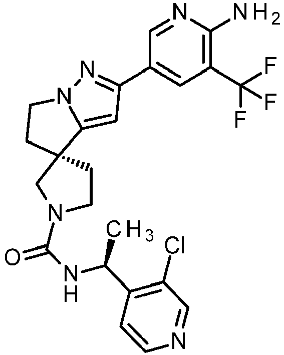

(1S)-1-(3-Chloropyridin-4-yl)ethan-1-amine hydrogen chloride (1/1) (34.5 mg, 179 µmol), CDI (31.6 mg, 195 µmol) and N,N-diisopropylethylamine (110 µL, 650 µmol) were dissolved in DMF (1 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) in DMF (1 mL) was added and the mixture was stirred over night at rt. The reaction mixture was purified by preparative HPLC to give 33.0 mg (95 % purity, 38 % yield) of the title compound.
LC-MS (Method 1): Rt = 0.99 min; MS (ESlpos): m/z = 507 [M+H]⁺
¹H-NMR (400 MHz, DMSO-d6) δ [ppm]: 0.934 (1.63), 0.951 (1.63), 1.336 (9.87), 1.354 (9.96), 2.067 (1.40), 2.085 (2.44), 2.518 (3.49), 2.523 (2.80), 2.565 (2.64), 3.437 (1.55), 3.462 (3.32), 3.498 (2.89), 3.522 (1.98), 3.539 (1.57), 3.555 (0.97), 3.565 (0.77), 4.175 (2.75), 4.193 (4.49), 4.210 (2.63), 5.052 (1.65), 5.069 (2.52), 5.088 (1.60), 6.470 (16.00), 6.553 (7.14), 6.778 (2.38), 6.796 (2.28), 7.454 (3.57), 7.467 (3.56), 8.014 (4.21), 8.019 (4.20), 8.436 (4.22), 8.448 (4.00), 8.525 (11.64), 8.589 (3.92), 8.593 (3.77).

### Example 777

### 2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (enantiomer 2)

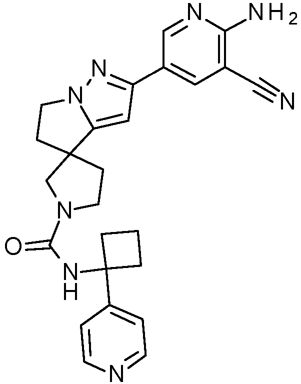

1-(Pyridin-4-yl)cyclobutan-1-amine (75.8 mg, 511 µmol), CDI (76.0 mg, 469 µmol) and N,N-diisopropylethylamine (370 µL, 2.1 mmol were dissolved in DMF (6.4 mL) and stirred for 2 h at rt. (Rac)-2-Amino-5-[5',6'-dihydrospiro[pyrrolidine-3,4'-pyrrolo[1,2-b]pyrazol]-2'-yl]pyridine-3-carbonitrile hydrochloride (150 mg, 90 % purity, 426 µmol) was added and the mixture was stirred for 2 h at 60 °C. The reaction mixture was allowed to cool down and concentrated. The residue was treated with toluene and evaporated again. The crude product was purified by column chromatography (silica gel, dichloromethane / methanol (0 - 10 %) gradient) to give 161 mg of the title compound as a racemic mixture.

The racemic mixture was separated into enantiomers by preparative chiral HPLC to give 17.5 mg (95 % purity, 9 % yield) of the title compound (enantiomer 2 Rₜ = 16.1 - 19.3 min).

### Preparative chiral HPLC method:

Instrument: PrepCon Labomatic HPLC-3; Column: YMC Cellulose SB 10µ, 250x50; eluent A: methyl tert-butyl ether + 0.1 vol % diethylamine; eluent B: acetonitrile + 0.1 vol % diethylamine; isocratic: 90%B; flow: 100 mL/min; temperature: 25 °C; UV: 254 nm

### Analytical chiral HPLC method:

Instrument: Thermo Fisher UltiMate 3000; Column: YMC Cellulose SB 3µ, 100x4.6; eluent A: methyl tert-butyl ether + 0.1 vol % diethylamine; eluent B: acetonitrile; isocratic: 90%B; flow: 1.4 mL/min; temperature: 25 °C; UV: 254 nm
Analytical chiral HPLC: Rₜ = 4.29 min
[α]²⁰_{D}: +64.0° (c = 1.00, CHCl₃)
LC-MS (Method 1): Rt = 0.83 min; MS (ESlpos): m/z = 455 [M+H]⁺
¹H NMR (CHLOROFORM-d, 400 MHz): δ (ppm) 8.62 (d, 1H), 8.58 (br d, 2H), 8.07 (d, 1H), 7.35 - 7.39 (m, 2H), 6.15 (s, 1H), 5.22 (s, 2H), 4.83 (s, 1H), 4.27 (t, 2H), 3.61 - 3.69 (m, 1H), 3.49 - 3.59 (m, 3H), 2.54 - 2.70 (m, 4H), 2.44 - 2.53 (m, 2H), 2.27 (dt, 1H), 2.07 - 2.20 (m, 2H), 1.88 - 2.00 (m, 1H).

### Example 778

### (3R)-2'-[6-amino-5-(trifluoromethyl)pyridin-3-yl]-N-[1-(3-methylpyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)

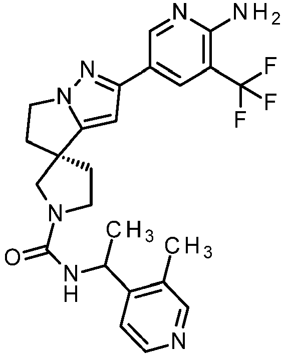

1-(3-Methylpyridin-4-yl)ethan-1-amine dihydrogen chloride (1/2) (63.9 mg, 306 µmol), CDI (54.1 mg, 334 µmol) and N,N-diisopropylethylamine (240 µL, 1.4 mmol) were dissolved in DMF (3 mL) and stirred for 1 h at rt. 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (125 mg, 80 % purity, 278 µmol) was added and stirred for 3 h at 60 °C. The reaction mixture was allowed to cool down and purified by preparative HPLC to give 45.0 mg (99 % purity, 33 % yield) of the title compound.
LC-MS (Method 1): Rt = 0.91 min; MS (ESlpos): m/z = 487 [M+H]⁺
¹H-NMR (400 MHz, DMSO-d6) δ [ppm]: 1.295 (4.27), 1.300 (4.43), 1.312 (4.40), 1.318 (4.29), 2.049 (1.00), 2.066 (1.70), 2.075 (4.17), 2.306 (16.00), 2.318 (0.60), 2.323 (1.04), 2.327 (1.38), 2.332 (0.93), 2.337 (0.41), 2.518 (5.83), 2.523 (4.42), 2.660 (0.41), 2.665 (0.96), 2.669 (1.32), 2.674 (0.92), 2.679 (0.40), 3.418 (1.20), 3.444 (3.50), 3.456 (2.87), 3.481 (0.82), 3.518 (0.58), 3.545 (0.45), 3.565 (0.51), 4.169 (1.53), 4.187 (2.87), 4.200 (1.43), 4.913 (1.04), 4.931 (1.64), 4.949 (1.05), 6.444 (2.52), 6.451 (6.30), 6.549 (4.55), 6.618 (1.02), 6.632 (1.25), 6.636 (1.22), .650 (0.94), 7.324 (1.35), 7.336 (1.37), 7.378 (1.39), 7.391 (1.41), 8.007 (2.97), 8.012 (2.99), 8.271 (3.11), 8.273 (2.70), 8.277 (3.15), 8.304 (1.43), 8.317 (1.36), 8.341 (1.65), 8.354 (1.55), 8.578 (2.60), 8.580 (2.61), 8.583 (2.57).

### Example 779

### (3R)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1)

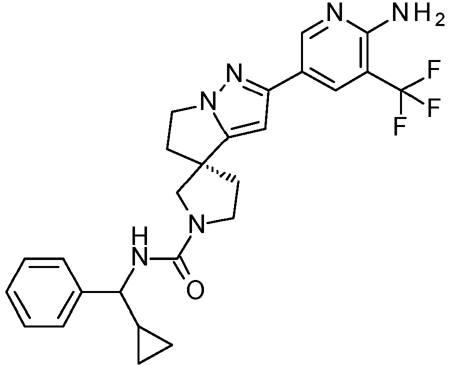

1-Cyclopropyl-1-phenylmethanamine (98.2 mg, 667 µmol), CDI (108 mg, 667 µmol) and N,N-diisopropylethylamine (387 µL, 2.2 mmol) were dissolved in DMF (10 mL) and stirred for 1 h at room temperature. 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (200 mg, 556 µmol) was added and the mixture was stirred for 2 h at room temperature. The reaction mixture was concentrated and diluted with water. The aqueous phase was extracted with dichloromethane. The combined organic layers were concentrated. The residue was purified by column chromatography (silica gel, ethyl acetate / metanol (0 - 10 %) gradient) to give 222 mg (80 % yield) of the title compound as a mixture of diastereomers.

The mixture was separated into diastereomers by preparative chiral HPLC to give 110 mg (95 % purity, 34 % yield) of the title compound (diastereomer 1 Rt = 5.2 - 6.2 min).

### Preparative chiral HPLC method:

Instrument: PrepCon Labomatic HPLC-2; Column: YMC Cellulose SB 5µ, 250x50; eluent A: methyl tert-butyl ether + 0.1 vol % diethylamine; eluent B: acetonitrile + 0.1 vol % diethylamine; isocratic: 20%B; flow: 50 mL/min; temperature: 25 °C; UV: 254 nm

### Analytical chiral HPLC method:

Instrument: Thermo Fisher UltiMate 3000; Column: YMC Cellulose SB 3µ, 100x4.6; eluent A: methyl tert-butyl ether + 0.1 vol % diethylamine; eluent B: acetonitrile; isocratic: 20%B; flow: 1.4 mL/min; temperature: 25 °C; UV: 254 nm
Analytical chiral HPLC: Rt = 2.26 min
[α]²⁰_{D}: +86.6° (c = 1.00, DMSO)
LC-MS (Method 1): Rt = 1.12 min; MS (ESlpos): m/z = 497 [M+H]⁺
¹H NMR (DMSO-d₆, 400 MHz): δ (ppm) 8.57 (d, 1H), 8.00 (d, 1H), 7.39 - 7.44 (m, 2H), 7.27 - 7.33 (m, 2H), 7.17 - 7.23 (m, 1H), 6.65 (d, 1H), 6.55 (s, 2H), 6.44 (s, 1H), 4.19 (t, 2H), 4.06 (t, 1H), 3.54 - 3.62 (m, 1H), 3.40 - 3.50 (m, 3H), 2.52 - 2.56 (m, 2H), 2.01 - 2.13 (m, 2H), 1.14 - 1.27 (m, 1H), 0.42 - 0.52 (m, 2H), 0.27 - 0.39 (m, 2H).

### Example 780

### (3R)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 2)

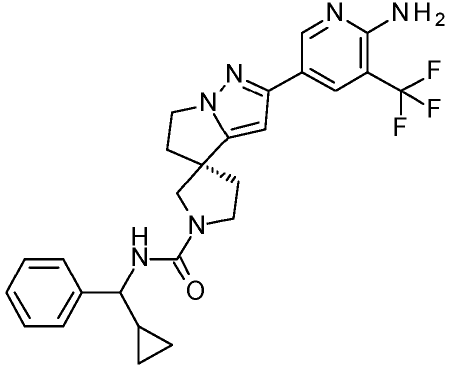

For the preparation of the diastereomeric mixture of the title compound see Example 779. The diastereomers were separated by preparative chiral HPLC (method see Example 779) to give 115 mg (95 % purity, 35 % yield) of the title compound (diastereomer 2 Rt = 7.9 - 9.9 min).
Analytical chiral HPLC (method see Example 779): Rₜ = 3.81 min.
[α]²⁰_{D}: +28.5° (c = 1.00, DMSO)
LC-MS (Method 1): Rₜ = 1.12 min; MS (ESlpos): m/z = 497 [M+H]⁺
¹H NMR (DMSO-d₆, 400 MHz): δ (ppm) 8.58 (d, 1H), 8.00 (d, 1H), 7.36-7.41 (m, 2H), 7.25 - 7.30 (m, 2H), 7.16 - 7.21 (m, 1H), 6.65 (d, 1H), 6.55 (s, 2H), 6.43 (s, 1H), 4.19 (t, 2H), 4.06 (t, 1H), 3.52 - 3.59 (m, 1H), 3.41 - 3.51 (m, 3H), 2.52 - 2.57 (m, 2H), 2.01 - 2.12 (m, 2H), 1.16 - 1.26 (m, 1H), 0.42 - 0.54 (m, 2H), 0.28 - 0.40 (m, 2H).

### Example 781

### (3R)-2'-[6-amino-5-(trifluoromethyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide

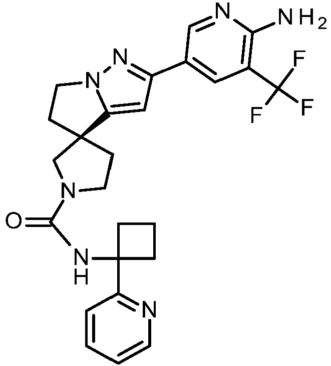

1-(Pyridin-2-yl)cyclobutan-1-amine (19.8 mg, 133 µmol), CDI (19.8 mg, 122 µmol) and N,N-diisopropylethylamine (120 µL, 670 µmol) were dissolved in DMF (1.5 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (50.0 mg, 80 % purity, 111 µmol) in DMF was added and the mixture was stirred over night at 60 °C. The reaction mixture was allowed to cool down, concentrated, treated with toluene and evaporated again. The residue was purified by preparative HPLC followed by preparative TLC (dichloromethane / methanol 9:1 gradient) to give 12.4 mg (100 % purity, 22 % yield) of the title compound.
LC-MS (Method 1): Rt = 1.02 min; MS (ESlpos): m/z = 498 [M+H]⁺
¹H-NMR (400 MHz, DMSO-d6) δ [ppm]: 0.852 (1.07), 0.868 (1.92), 0.886 (1.59), 0.906 (2.94), 0.924 (1.34), 1.232 (2.87), 1.279 (0.80), 1.297 (0.97), 1.353 (0.64), 1.392 (0.57), 1.412 (0.60), 1.431 (0.44), 1.862 (0.48), 1.878 (0.89), 1.888 (0.80), 1.905 (1.30), 1.921 (0.77), 1.927 (0.67), 1.944 (0.42), 1.962 (0.69), 1.978 (1.23), 2.001 (1.01), 2.017 (0.62), 2.069 (1.25), 2.084 (2.20), 2.099 (2.55), 2.116 (1.09), 2.129 (0.54), 2.327 (4.09), 2.331 (2.93), 2.337 (1.44), 2.373 (1.89), 2.394 (2.00), 2.409 (1.21), 2.518 (16.00), 2.523 (11.09), 2.561 (5.22), 2.577 (4.59), 2.589 (2.06), 2.594 (2.03), 2.617 (0.81), 2.669 (4.12), 2.673 (2.82), 2.678 (1.25), 3.483 (6.89), 3.569 (0.65), 3.587 (1.18), 3.627 (0.46), 4.184 (3.08), 4.202 (5.29), 4.219 (3.01), 4.228 (0.99), 4.234 (0.87), 4.242 (0.81), 4.248 (0.76), 5.760 (15.19), 6.476 (12.13), 6.558 (7.76), 6.792 (5.11), 7.158 (2.22), 7.161 (2.49), 7.170 (2.24), 7.173 (2.65), 7.177 (2.62), 7.179 (2.43), 7.189 (2.53), 7.191 (2.47), 7.360 (3.66), 7.379 (4.00), 7.645 (2.30), 7.649 (2.49), 7.663 (2.92), 7.668 (2.84), 7.683 (1.76), 7.688 (1.85), 8.011 (4.43), 8.016 (4.44), 8.089 (5.24), 8.519 (2.64), 8.521 (2.76), 8.523 (3.05), 8.526 (2.58), 8.531 (2.65), 8.533 (2.85), 8.535 (2.76), 8.538 (2.22), 8.590 (4.13), 8.594 (4.02).

### Example 782

### (3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 1 and diastereomer 2)

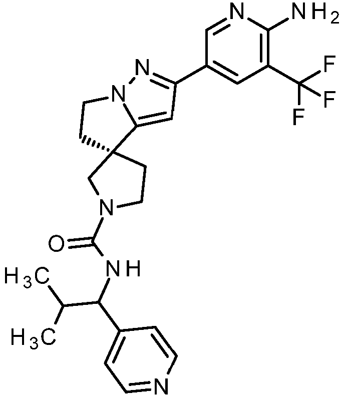

2-Methyl-1-(pyridin-4-yl)propan-1-amine (37.6 mg, 250 µmol) was dissoved in DMF (5 mL). CDI (40.6 mg, 250 µmol) and N,N-diisopropylethylamine (120 µL, 830 µmol) were added and stirred for 1 h at rt. 5-[(3R)-5',6'-Ddihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 208 µmol) was added and the mixture was stirred over night at rt. The reaction mixture was diluted with water and extracted three times with dichloromethane. The organic phase was dried over a hydrophobic filter and concentrated. The residue was purified by preparative HPLC to give 50.0 mg (100 % purity, 48 % yield) of the title compound.
LC-MS (Method 1): Rt = 0.94 min; MS (ESlpos): m/z = 500 [M+H]⁺
¹H-NMR (400 MHz, DMSO-d6) δ [ppm]: 0.663 (0.97), 0.679 (10.34), 0.684 (10.23), 0.696 (10.38), 0.701 (10.08), 0.733 (0.76), 0.750 (0.69), 0.936 (9.00), 0.953 (9.40), 0.969 (8.96), 0.986 (9.18), 1.978 (0.82), 1.986 (0.97), 1.994 (1.32), 2.003 (1.45), 2.010 (1.67), 2.016 (1.56), 2.026 (1.85), 2.041 (2.68), 2.055 (3.57), 2.067 (4.25), 2.085 (1.92), 2.097 (0.80), 2.332 (2.74), 2.336 (1.16), 2.518 (16.00), 2.523 (13.66), 2.673 (2.78), 2.678 (1.21), 3.382 (0.50), 3.405 (2.42), 3.415 (2.05), 3.430 (3.66), 3.440 (3.35), 3.450 (1.25), 3.459 (1.08), 3.477 (4.92), 3.491 (4.16), 3.502 (2.46), 3.511 (1.73), 3.516 (2.56), 3.566 (0.63), 3.585 (1.16), 3.592 (0.76), 3.599 (0.84), 3.608 (0.87), 3.625 (0.47), 4.166 (4.67), 4.183 (8.82), 4.200 (4.54), 4.334 (1.39), 4.350 (1.76), 4.356 (2.88), 4.372 (2.92), 4.378 (1.73), 4.394 (1.36), 4.485 (0.40), 6.425 (15.08), 6.430 (7.39), 6.447 (3.27), 6.471 (1.96), 6.548 (11.81), 7.315 (6.36), 7.319 (3.76), 7.326 (3.92), 7.330 (6.78), 7.338 (6.62), 7.341 (3.92), 7.349 (3.89), 7.353 (6.53), 7.986 (3.64), 7.991 (3.98), 7.997 (3.79), 8.002 (3.57), 8.451 (7.85), 8.454 (4.30), 8.461 (4.34), 8.466 (8.69), 8.468 (9.72), 8.471 (4.64), 8.478 (4.20), 8.482 (7.40), 8.563 (6.37), 8.569 (6.18).

### Example 783

### (3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 1)

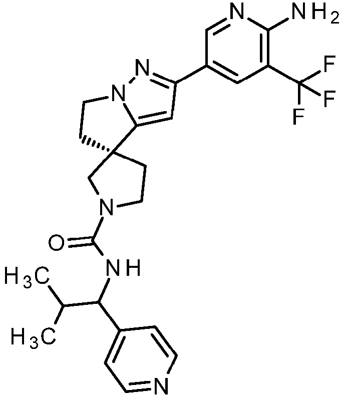

The title compound from **Example 782** was separated into diastereomers by preparative chiral HPLC to give 20.8 mg (97 % purity, 19 % yield) of the title compound (diastereomer 1 Rt = 10.84 - 12.96 min).

### Preparative chiral HPLC method:

Instrument: PrepCon Labomatic HPLC-3; Column: YMC Cellulose SC 5µ, 250x50; eluent A: hexane + 0.1 vol % diethylamine; eluent B: ethanol; isocratic: 50%B; flow: 60 mL/min; temperature: 25 °C; UV: 280 nm

### Analytical chiral HPLC method:

Instrument: Thermo Fisher UltiMate 3000; Column: YMC Cellulose SC 3µ, 100x4.6; eluent A: hexane + 0.1 vol % diethylamine; eluent B: ethanol; isocratic: 50%B; flow: 1.4 mL/min; temperature: 25 °C; UV: 280 nm
Analytical chiral HPLC: Rt = 2.11 min
[α]²⁰_{D}: +91.0° (c = 1.00, methanol)
LC-MS (Method 1): Rt = 0.96 min; MS (ESlpos): m/z = 500 [M+H]⁺
¹H-NMR (400 MHz, DMSO-d6) δ [ppm]: 0.679 (14.84), 0.696 (15.36), 0.795 (0.77), 0.798 (0.60), 0.814 (0.71), 0.822 (0.63), 0.836 (0.51), 0.851 (0.45), 0.862 (0.49), 0.905 (0.63), 0.936 (15.52), 0.953 (16.00), 1.006 (0.58), 1.035 (0.45), 1.084 (1.24), 1.204 (0.44), 1.232 (1.12), 1.259 (1.72), 1.352 (2.20), 1.961 (0.53), 1.978 (1.26), 1.994 (1.79), 1.999 (1.55), 2.011 (1.64), 2.016 (1.96), 2.033 (1.86), 2.040 (2.17), 2.055 (3.25), 2.067 (4.04), 2.085 (1.89), 2.097 (0.76), 2.114 (0.43), 2.327 (3.07), 2.332 (2.20), 2.336 (0.99), 2.518 (14.31), 2.523 (12.39), 2.562 (0.60), 2.673 (2.23), 2.678 (0.97), 3.382 (0.91), 3.401 (2.05), 3.415 (3.37), 3.426 (2.70), 3.440 (5.45), 3.477 (5.73), 3.502 (2.95), 3.566 (1.07), 3.584 (2.11), 3.592 (1.42), 3.599 (1.59), 3.610 (1.52), 3.625 (0.83), 4.166 (4.46), 4.184 (8.56), 4.201 (4.27), 4.350 (2.46), 4.372 (4.80), 4.394 (2.39), 6.430 (11.96), 6.447 (3.93), 6.549 (11.21), 7.338 (10.80), 7.341 (6.74), 7.349 (6.90), 7.353 (11.04), 7.997 (6.27), 8.002 (6.31), 8.468 (12.78), 8.471 (7.23), 8.478 (6.80), 8.483 (11.52), 8.565 (5.80), 8.569 (5.71), 10.851 (0.62).

### Example 784

### (3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 2)

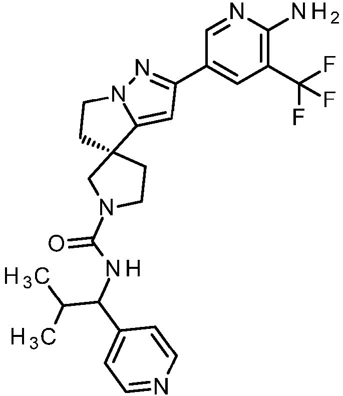

For the preparation of the diastereomeric mixture of the title compound see Example 782. The diastereomers were separated by preparative chiral HPLC (method see Example 783) to give 12.6 mg (100 % purity, 12 % yield) of the title compound (diastereomer 2 Rt = 19.29 - 22.77 min).
Analytical chiral HPLC (method see Example 783): Rt = 3.79 min.
[α]²⁰_{D}: +65.9° (c = 1.00, methanol)
LC-MS (Method 1): Rt = 0.96 min; MS (ESlpos): m/z = 500 [M+H]⁺
¹H NMR (DMSO-d₆, 400 MHz): δ (ppm) 8.56 (d, 1H), 8.44 - 8.47 (m, 2H), 7.99 (d, 1H), 7.30 - 7.34 (m, 2H), 6.53 (s, 2H), 6.48 (d,1H), 6.42 (s, 1H), 4.35 (t, 1H), 4.18 (t, 2H), 3.44 - 3.57 (m, 6H), 1.96 - 2.12 (m, 3H), 0.97 (d, 3H), 0.69 (d, 3H).

### Example 785

### (3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)

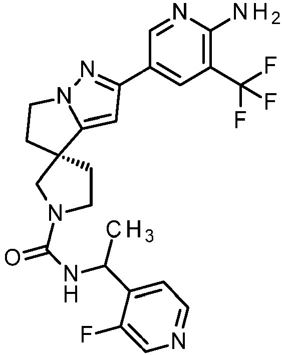

1-(3-Fluoropyridin-4-yl)ethan-1-amine (76.9 mg, 548 µmol), CDI (97.0 mg, 598 µmol) and N,N-diisopropylethylamine (350 µL, 2.0 mmol) were dissolved in DMF (5 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (230 mg, 78 % purity, 499 µmol) in DMF (1 mL) was added and the mixture was stirred over night at rt. The reaction mixture was purified by preparative HPLC to give 94.0 mg (95 % purity, 37 % yield) of the title compound.
LC-MS (Method 1): Rt = 0.94 min; MS (ESlpos): m/z = 491 [M+H]⁺
¹H-NMR (400 MHz, DMSO-d6) δ [ppm]: 0.932 (1.01), 0.948 (1.00), 1.361 (7.89), 1.366 (8.12), 1.378 (8.09), 1.383 (7.90), 2.075 (4.08), 2.083 (3.23), 2.337 (1.31), 2.518 (16.00), 2.523 (12.23), 2.565 (3.29), 2.679 (1.23), 3.438 (2.37), 3.464 (5.76), 3.483 (4.25), 3.509 (1.50), 3.542 (1.10), 3.567 (0.64), 3.592 (0.84), 4.177 (3.01), 4.193 (5.36), 4.209 (2.79), 5.052 (1.23), 5.057 (1.34), 5.070 (1.93), 5.076 (1.94), 5.088 (1.31), 5.093 (1.25), 6.474 (14.36), 6.550 (9.40), 6.686 (1.88), 6.698 (2.06), 6.704 (2.09), 6.717 (1.67), 7.414 (1.27), 7.426 (1.53), 7.443 (1.26), 7.484 (1.39), 7.497 (1.60), 7.513 (1.38), 8.014 (5.71), 8.019 (5.67), 8.346 (2.12), 8.359 (2.05), 8.381 (2.56), 8.384 (2.47), 8.396 (2.38), 8.451 (4.61), 8.456 (8.49), 8.461 (4.31), 8.588 (5.29), 8.593 (5.07).

### Example 786

### (3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 1)

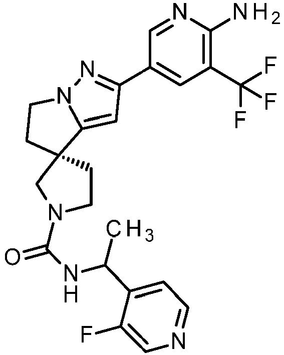

The title compound from **Example 785** was separated into diastereomers by preparative chiral HPLC (diastereomer 1 Rt = 8.8 - 12.0 min).

### Preparative chiral HPLC method:

Instrument: Sepiatec: Prep SFC100; Column: Chiral Art Cellulose-SC 5µ, 250x50; eluent A: CO2; eluent B: 2-propanol + 0.4 vol % diethylamine; isocratic: 35%B; flow: 100 mL/min; temperature: 40 °C; BPR: 150 bar; UV: 267 nm

### Analytical chiral HPLC method:

Instrument: Waters Acquity UPC2 QDA; Column: Chiral Art Cellulose-SB 3µ, 100x4.6; eluent A: CO2; eluent B: 2-propanol + 0.4 vol % diethylamine; isocratic: 35%B; flow: 4 mL/min; temperature: 37.5 °C; BPR: 100 bar; UV: 254 nm
Analytical chiral HPLC: Rt = 3.04 min

The crude fraction was purified by preparative HPLC to give 32.0 mg (99 % purity, 35 % yield) of the title compound.
[α]²⁰_{D}: +20.1° (c = 1.00, DMSO)
LC-MS (Method 1): Rt = 0.94 min; MS (ESlpos): m/z = 490 [M+H]⁺
¹H-NMR (400 MHz, DMSO-d6) δ [ppm]: 1.366 (9.23), 1.384 (9.31), 2.064 (1.34), 2.077 (2.07), 2.083 (2.42), 2.100 (1.16), 2.518 (1.87), 2.523 (1.77), 2.565 (2.66), 3.438 (1.51), 3.464 (4.01), 3.488 (3.26), 3.513 (1.47), 3.525 (0.91), 3.542 (1.50), 3.551 (0.78), 3.558 (0.95), 3.568 (0.80), 4.173 (2.76), 4.192 (4.43), 4.209 (2.66), 5.053 (1.48), 5.071 (2.26), 5.090 (1.45), 6.474 (16.00), 6.550 (7.06), 6.699 (2.32), 6.718 (2.22), 7.414 (1.75), 7.427 (2.11), 7.431 (2.08), 7.444 (1.73), 8.015 (4.11), 8.020 (4.12), 8.346 (2.92), 8.358 (2.83), 8.451 (5.97), 8.456 (5.88), 8.590 (3.84), 8.594 (3.77).

### Example 787

### (3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 2)

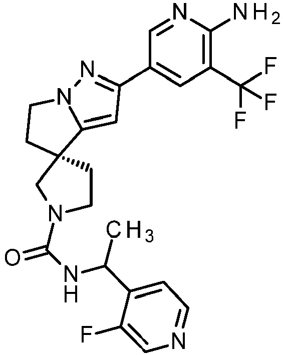

For the preparation of the diastereomeric mixture of the title compound see **Example 785.** The diastereomers were separated by preparative chiral HPLC (method see **Example 786)** (diastereomer 2 Rt = 16.5 - 22.0 min).
Analytical chiral HPLC (method see **Example 786):** Rt = 5.75 min

The crude fraction was purified by preparative HPLC to give 36.0 mg (99 % purity, 40 % yield) of the title compound.
[α]²⁰_{D}: +91.4° (c = 1.00, DMSO)
LC-MS (Method 1): Rt = 0.94 min; MS (ESlpos): m/z = 490 [M+H]⁺
¹H-NMR (400 MHz, DMSO-d6) δ [ppm]: 1.361 (15.91), 1.379 (16.00), 2.081 (3.36), 2.088 (3.84), 2.106 (1.83), 2.119 (0.62), 2.332 (0.54), 2.518 (3.21), 2.523 (2.64), 2.567 (4.59), 3.422 (0.91), 3.440 (3.32), 3.466 (7.65), 3.483 (6.90), 3.509 (1.88), 3.577 (0.99), 3.594 (1.94), 3.602 (1.32), 3.610 (1.41), 3.619 (1.46), 3.635 (0.71), 4.177 (4.93), 4.195 (8.34), 4.213 (4.74), 5.042 (0.59), 5.059 (2.59), 5.077 (3.97), 5.095 (2.56), 5.113 (0.57), 6.473 (11.38), 6.552 (12.20), 6.687 (4.11), 6.706 (3.95), 7.485 (3.25), 7.497 (3.82), 7.501 (3.81), 7.514 (3.26), 8.016 (6.78), 8.021 (6.85), 8.381 (6.04), 8.384 (5.92), 8.394 (5.73), 8.396 (5.67), 8.456 (10.28), 8.461 (10.20), 8.589 (6.31), 8.593 (6.27).

### Example 788

### (3R)-2'-[6-amino-5-(trifluoromethyl)pyridin-3-yl]-N-[(1R)-1-(3-chloropyridin-4-yl)ethyl]-5',6'-dihydrospiro[pyrrolidine-3,4'-pyrrolo[1,2-b]pyrazole]-1-carboxamide

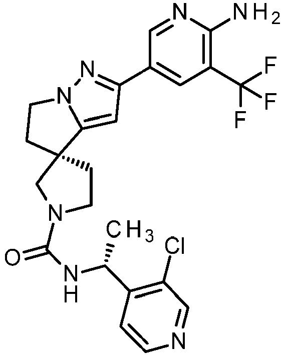

(1R)-1-(3-Chloropyridin-4-yl)ethan-1-amine (28.0 mg, 179 µmol), CDI (31.6 mg, 195 µmol) and N,N-diisopropylethylamine (110 µL, 650 µmol) were dissolved in DMF (1 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) in DMF (1 mL) was added and the mixture was stirred over night at rt. The reaction mixture was purified by preparative HPLC to give 24.0 mg (99 % purity, 29 % yield) of the title compound.
LC-MS (Method 1): Rt = 0.99 min; MS (ESlpos): m/z = 507 [M+H]⁺
¹H-NMR (400 MHz, DMSO-d6) δ [ppm]: 1.332 (14.52), 1.349 (14.56), 2.085 (3.01), 2.092 (3.23), 2.327 (3.34), 2.331 (2.33), 2.337 (1.03), 2.518 (13.69), 2.523 (9.87), 2.568 (3.53), 2.673 (2.31), 2.678 (1.04), 3.420 (0.76), 3.440 (2.84), 3.465 (5.43), 3.490 (4.05), 3.516 (1.61), 3.585 (0.77), 3.603 (1.48), 3.628 (1.16), 4.179 (4.16), 4.197 (7.28), 4.214 (3.99), 5.037 (0.53), 5.055 (2.39), 5.073 (3.73), 5.090 (2.36), 5.108 (0.50), 6.472 (9.38), 6.554 (10.26), 6.762 (3.60), 6.780 (3.44), 7.531 (5.66), 7.543 (5.80), 8.015 (5.71), 8.020 (5.76), 8.478 (9.40), 8.490 (8.96), 8.529 (16.00), 8.588 (5.35), 8.592 (5.20).

### Example 789

### (3R)-2'-[6-amino-5-(trifluoromethyl)pyridin-3-yl]-N-[1-(2-methylpyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)

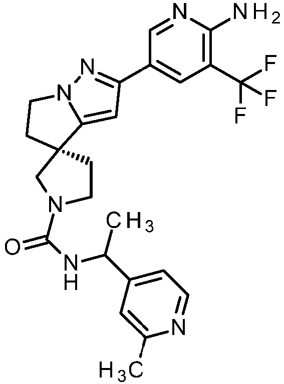

1-(2-Methylpyridin-4-yl)ethan-1-amine (24.4 mg, 179 µmol), CDI (31.6 mg, 195 µmol) and N,N-diisopropylethylamine (110 µL, 650 µmol) were dissolved in DMF (1 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) in DMF (1 mL) was added and the mixture was stirred over night at rt. The reaction mixture was purified by preparative HPLC to give 40.0 mg (95 % purity, 48 % yield) of the title compound.
LC-MS (Method 1): Rt = 0.93 min; MS (ESlpos): m/z = 487 [M+H]⁺
¹H-NMR (400 MHz, DMSO-d6) δ [ppm]: 1.204 (0.53), 1.221 (0.50), 1.335 (6.35), 1.340 (6.38), 1.353 (6.63), 1.358 (6.27), 1.385 (0.82), 1.401 (0.77), 2.059 (1.44), 2.076 (2.66), 2.084 (2.11), 2.281 (2.26), 2.318 (0.96), 2.407 (14.08), 2.427 (1.12), 2.435 (16.00), 2.456 (2.10), 2.463 (0.80), 2.468 (0.96), 2.518 (13.53), 2.522 (9.75), 2.564 (3.04), 2.604 (0.76), 2.660 (1.01), 3.344 (1.40), 3.361 (0.59), 3.436 (1.64), 3.444 (0.87), 3.461 (4.62), 3.469 (3.46), 3.480 (3.06), 3.495 (1.00), 3.506 (0.84), 3.522 (0.46), 3.539 (0.92), 3.556 (0.62), 3.564 (0.83), 3.580 (0.95), 3.596 (0.54), 3.604 (0.54), 4.175 (2.68), 4.192 (4.88), 4.209 (2.58), 4.755 (1.05), 4.773 (1.55), 4.789 (1.04), 6.469 (5.16), 6.472 (9.51), 6.547 (8.50), 6.566 (1.76), 7.106 (1.34), 7.119 (1.38), 7.141 (1.45), 7.154 (3.91), 7.190 (2.68), 8.013 (4.04), 8.018 (4.12), 8.306 (2.30), 8.319 (2.25), 8.330 (2.50), 8.343 (2.37), 8.588 (4.10).

### Example 790

### (3R)-2'-[6-amino-5-(trifluoromethyl)pyridin-3-yl]-N-[1-(3-methoxypyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)

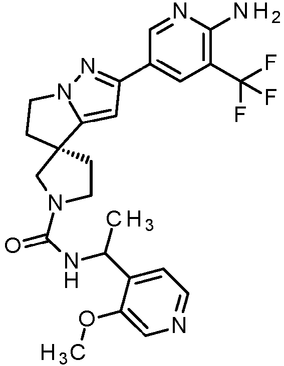

The compound was prepared similarly to **Example 789** using 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) and 1-(3-methoxypyridin-4-yl)ethan-1-amine (27.2 mg, 179 µmol) to give 48.0 mg (95 % purity, 56 % yield) of the title compound.
LC-MS (Method 1): Rt = 0.94 min; MS (ESlpos): m/z = 503 [M+H]⁺
¹H-NMR (400 MHz, DMSO-d6) δ [ppm]: 1.272 (4.99), 1.277 (5.08), 1.289 (5.15), 1.293 (5.00), 2.066 (1.07), 2.074 (1.97), 2.083 (1.83), 2.093 (1.48), 2.518 (2.01), 2.523 (1.63), 2.567 (2.09), 3.444 (1.48), 3.469 (3.56), 3.486 (2.13), 3.495 (1.68), 3.511 (0.79), 3.521 (0.59), 3.547 (0.64), 3.562 (0.41), 3.601 (0.49), 3.915 (15.70), 3.919 (16.00), 4.179 (1.73), 4.195 (3.07), 4.211 (1.60), 5.062 (0.66), 5.071 (0.73), 5.081 (0.97), 5.088 (1.01), 5.099 (0.70), 5.107 (0.67), 6.469 (7.11), 6.473 (3.62), 6.519 (1.23), 6.530 (1.42), 6.539 (1.82), 6.551 (6.67), 7.253 (1.59), 7.265 (1.63), 7.330 (1.82), 7.342 (1.83), 8.015 (3.40), 8.021 (3.44), 8.119 (2.00), 8.131 (1.87), 8.165 (2.87), 8.177 (2.65), 8.262 (4.76), 8.267 (4.96), 8.589 (2.96), 8.592 (2.88).

### Example 791

### (3R)-2'-[6-amino-5-(trifluoromethyl)pyridin-3-yl]-N-[1-(pyridin-4-yl)cyclopropyl]-5',6'-dihydrospiro[pyrrolidine-3,4'-pyrrolo[1,2-b]pyrazole]-1-carboxamide

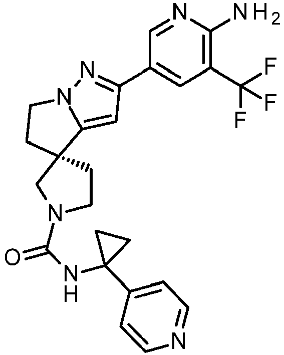

The compound was prepared similarly to **Example** 789 using 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (105 mg, 78 % purity, 228 µmol) and 1-(pyridin-4-yl)cyclopropan-1-amine (33.6 mg, 250 µmol) to give 58.0 mg (99 % purity, 52 % yield) of the title compound.
LC-MS (Method 1): Rt = 0.89 min; MS (ESlpos): m/z = 485 [M+H]⁺
¹H-NMR (400 MHz, DMSO-d6) δ [ppm]: 1.179 (0.44), 1.196 (0.51), 1.238 (0.59), 1.265 (6.93), 1.279 (6.64), 1.306 (0.45), 2.092 (1.92), 2.104 (2.18), 2.331 (2.79), 2.337 (1.21), 2.518 (16.00), 2.523 (11.14), 2.563 (4.07), 2.581 (2.80), 2.673 (2.78), 2.678 (1.23), 3.431 (0.61), 3.449 (1.54), 3.468 (10.24), 3.492 (0.83), 3.556 (0.76), 3.573 (1.40), 3.580 (0.89), 3.588 (1.01), 3.599 (0.91), 3.613 (0.52), 4.183 (2.92), 4.201 (4.63), 4.218 (2.76), 6.504 (10.71), 6.559 (7.26), 7.068 (8.07), 7.072 (4.99), 7.080 (4.96), 7.084 (8.10), 7.129 (4.10), 8.025 (4.16), 8.030 (4.20), 8.377 (9.16), 8.381 (5.27), 8.389 (4.99), 8.392 (8.65), 8.603 (3.96), 8.606 (3.84).

### Example 792

### (3R)-2'-{6-amino-5-[(1R)-1-(2-methoxyphenyl)ethoxy]pyridin-3-yl}-N-ethyl-5',6'-dihydrospiro[pyrrolidine-3,4'-pyrrolo[1,2-b]pyrazole]-1-carboxamide

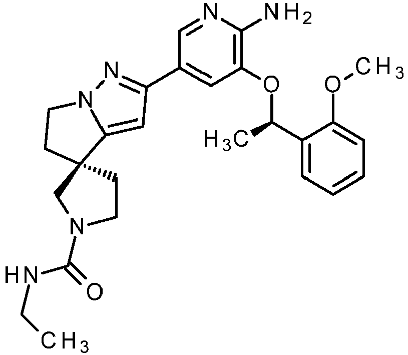

(3R)-1-(Ethylcarbamoyl)-5',6'-dihydrospiro[pyrrolidine-3,4'-pyrrolo[1,2-b]pyrazol]-2'-yl trifluoromethanesulfonate (81.0 mg, 95 % purity, 201 µmol), 3-[(1R)-1-(2-methoxyphenyl)ethoxy]-5-(4,4,5,5-tetramethyl-1,3,2-dioxaborolan-2-yl)pyridin-2-amine (142 mg, 63 % purity, 242 µmol) and potassium phosphate (1.2 mL, 0.50 M, 600 µmol) were dissolved in 1,4-dioxane (20 mL) under nitrogen. XPhos Pd G2 (15.8 mg, 20.1 µmol) was added and the mixture was stirred for 3 h at 100 °C. The cold reaction mixture was diluted with ethyl acetate and filtered over celite. The organic phase was washed with water and brine, dried over a hydrophobic filter and concentrated. The residue was purified twice by preparative HPLC to give 4.80 mg (100 % purity, 5 % yield) of the title compound.
LC-MS (Method 2): Rt = 0.87 min; MS (ESlpos): m/z = 477 [M+H]⁺
¹H NMR (DMSO-d₆, 400 MHz): δ (ppm) 7.82 (d, 1H), 7.39 (dd, 1H), 7.21 - 7.27 (m, 1H), 7.13 (d, 1H), 7.03 (d, 1H), 6.92 (td, 1H), 6.15 (t, 1H), 6.11 (s, 1H), 5.81 (s, 2H), 5.73 (q, 1H), 4.08 - 4.15 (m, 2H), 3.91 (s, 3H), 3.42 - 3.49 (m, 1H), 3.35 - 3.41 (m, 3H), 3.01 - 3.09 (m, 2H), 1.96 - 2.08 (m, 2H), 1.55 (d, 3H), 1.02 (t, 3H).

### Example 793

### 3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 1 and diastereomer 2)

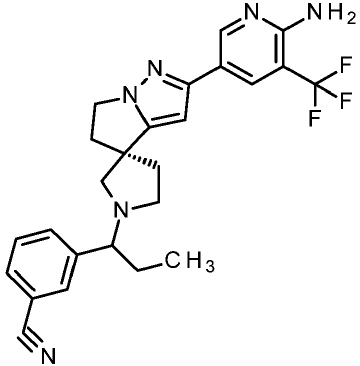

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (100 mg, 95 % purity, 264 µmol) and 3-propanoylbenzonitrile (63.1 mg, 396 µmol) were dissolved in methanol (2.8 mL). N,N-Diisopropylethylamine (180 µL, 1.1 mmol), titanium(IV) isopropoxide (230 µL, 790 µmol) and sodium cyanoborohydride (49.8 mg, 792 µmol) were added and the mixture was stirred over night at 60 °C. The reaction mixture was allowed to cool down, diluted with water (0.5 mL) and stirred for 15 min. The suspension was filtered and the filter cake was washed with methanol. The filtrate was concentrated. The crude product was purified by preparative HPLC to give 54.0 mg (100 % purity, 44 % yield) of the title compound.
LC-MS (Method 1): Rt = 1.48 min; MS (ESlpos): m/z = 467 [M+H]⁺
¹H-NMR (400 MHz, DMSO-d6) δ [ppm]: 0.619 (6.70), 0.637 (15.32), 0.656 (7.05), 0.931 (0.91), 0.948 (0.92), 1.027 (0.45), 1.042 (0.47), 1.632 (0.61), 1.657 (1.00), 1.665 (1.24), 1.684 (1.04), 1.698 (0.68), 1.706 (0.65), 1.858 (0.70), 1.870 (1.05), 1.887 (1.29), 1.903 (1.17), 1.922 (0.86), 1.941 (0.64), 1.960 (1.16), 1.980 (2.26), 1.998 (3.31), 2.010 (2.00), 2.017 (1.94), 2.036 (0.62), 2.337 (0.97), 2.458 (1.79), 2.518 (16.00), 2.523 (14.89), 2.566 (6.47), 2.586 (2.50), 2.600 (1.09), 2.618 (0.51), 2.692 (1.48), 2.714 (3.48), 2.736 (2.37), 2.788 (0.45), 2.808 (0.83), 2.826 (0.87), 3.159 (5.56), 3.172 (5.91), 3.297 (2.84), 3.308 (3.97), 4.037 (0.50), 4.045 (1.03), 4.064 (2.17), 4.078 (3.68), 4.097 (5.99), 4.110 (2.85), 4.123 (0.88), 6.424 (9.10), 6.449 (11.15), 6.518 (10.08), 7.529 (1.65), 7.548 (4.43), 7.566 (3.90), 7.584 (1.76), 7.668 (3.55), 7.672 (3.72), 7.688 (2.75), 7.692 (2.70), 7.703 (2.01), 7.707 (2.90), 7.710 (1.90), 7.726 (3.76), 7.743 (4.68), 7.746 (4.86), 7.765 (4.28), 7.998 (3.01), 8.007 (4.40), 8.012 (3.68), 8.582 (2.80), 8.592 (3.69), 8.597 (3.36).

### Example 794

### 3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 1)

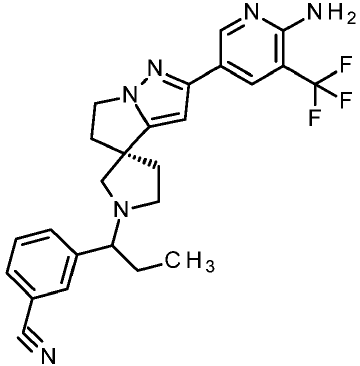

The title compound from **Example 793** was separated into diastereomers by preparative chiral HPLC to give 16.9 mg (99 % purity, 14 % yield) of the title compound (diastereomer 1 Rₜ = 8.4 - 10.14 min).

### Preparative chiral HPLC method:

Instrument: PrepCon Labomatic HPLC-1; Column: YMC Amylose SA 10µ, 250x50; eluent A: hexane + 0.1 vol % diethylamine; eluent B: ethanol; isocratic: 20%B; flow: 140 mL/min; temperature: 25 °C; UV: 280 nm

### Analytical chiral HPLC method:

Instrument: Thermo Fisher UltiMate 3000; Column: YMC Amylose SA 3µ, 100x4.6; eluent A: hexane + 0.1 vol % diethylamine; eluent B: ethanol; isocratic: 20%B; flow: 1.4 mL/min; temperature: 25 °C; UV: 280 nm
Analytical chiral HPLC: Rₜ = 2.08 min
[α]²⁰_{D}: +78.2° (c = 1.00, methanol)
LC-MS (Method 1): Rt = 1.33 min; MS (ESlpos): m/z = 467 [M+H]⁺
¹H-NMR (400MHz, DMSO-d₆): δ [ppm] = 0.64 (t, 3H), 1.66 (br dd, 1H), 1.84 - 1.96 (m, 1H), 1.97 - 2.05 (m, 2H), 2.54 - 2.60 (m, 4H), 2.82 (q, 1H), 4.01 - 4.14 (m, 2H), 6.42 (s, 1H), 6.52 (s, 2H), 7.52 - 7.60 (m, 1H), 7.68 (d, 1H), 7.71 - 7.76 (m, 2H), 8.00 (d, 1H), 8.58 (d, 1H).

### Example 795

### 3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 2)

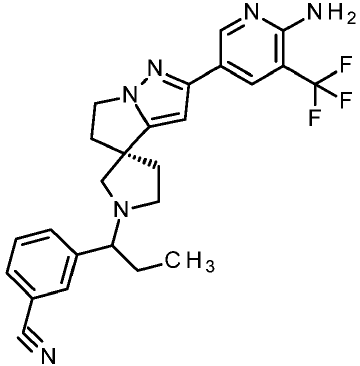

For the preparation of the diastereomeric mixture of the title compound see **Example 793.** The diastereomers were separated by preparative chiral HPLC (method see **Example 794)** to give 19.2 mg (100 % purity, 16 % yield) of the title compound (diastereomer 2 Rt = 10.14 - 13.52 min).
Analytical chiral HPLC (method see Example **794):** Rt = 2.46 min.
[α]²⁰_{D}: +98.4° (c = 1.00, methanol)
LC-MS (Method 1): Rt = 1.34 min; MS (ESlpos): m/z = 467 [M+H]⁺
¹H-NMR (400 MHz, DMSO-d6) δ [ppm]: 0.620 (3.07), 0.638 (7.08), 0.656 (3.48), 0.815 (0.44), 0.822 (0.41), 1.084 (0.54), 1.233 (1.68), 1.259 (0.94), 1.352 (0.85), 1.632 (0.43), 1.651 (0.59), 1.667 (0.70), 1.688 (0.76), 1.707 (0.53), 1.858 (0.50), 1.869 (0.60), 1.877 (0.63), 1.887 (0.72), 1.903 (0.57), 1.921 (0.46), 1.927 (0.45), 1.943 (0.47), 1.960 (0.94), 1.980 (1.10), 1.995 (1.12), 2.011 (1.18), 2.025 (0.98), 2.037 (0.47), 2.323 (1.11), 2.327 (1.55), 2.332 (1.18), 2.458 (0.74), 2.522 (16.00), 2.568 (4.68), 2.585 (3.04), 2.600 (2.01), 2.618 (1.21), 2.665 (1.56), 2.669 (2.23), 2.673 (1.95), 2.693 (1.44), 2.715 (3.05), 2.737 (2.09), 3.159 (2.27), 3.172 (2.41), 3.298 (1.20), 3.309 (1.66), 4.080 (1.75), 4.095 (3.77), 4.108 (1.46), 4.114 (1.87), 6.449 (7.92), 6.519 (5.41), 7.529 (1.28), 7.549 (3.10), 7.568 (2.10), 7.668 (2.15), 7.688 (1.67), 7.707 (2.30), 7.727 (1.92), 7.766 (3.55), 8.008 (2.93), 8.013 (3.04), 8.593 (2.79), 8.597 (2.82).

### Example 796

### (3S)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (enantiomer 1)

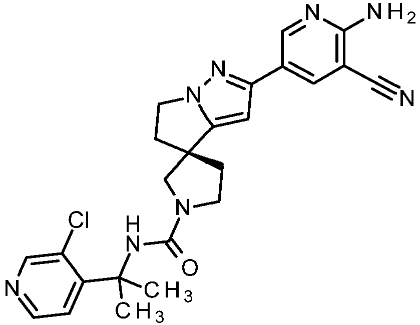

The racemic mixture of 2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide was separated into enantiomers by preparative chiral HPLC to give 23.0 mg (95 % purity, 33 % yield) of the title compound (enantiomer 1 Rt = 8.14 - 9.29 min).

### Preparative chiral HPLC method:

Instrument: PrepCon Labomatic HPLC-2; Column: YMC Cellulose SC 5µ, 250x50; eluent A: methyl tert-butyl ether + 0.1 vol % diethylamine; eluent B: ethanol; isocratic: 20%B; flow: 40 mL/min; temperature: 25 °C; UV: 254 nm

### Analytical chiral HPLC method:

Instrument: Thermo Fisher UltiMate 3000; Column: YMC Cellulose SC 3µ, 100x4.6; eluent A: methyl tert-butyl ether + 0.1 vol % diethylamine; eluent B: ethanol; isocratic: 20%B; flow: 1.4 mL/min; temperature: 25 °C; UV: 254 nm
Analytical chiral HPLC: Rt = 3.89 min
[α]²⁰_{D}: -104.2° (c = 1.00, DMSO)
LC-MS (Method 1): Rt = 0.90 min; MS (ESlpos): m/z = 477 [M+H]⁺
¹H-NMR (400 MHz, DMSO-d6) δ [ppm]: 0.796 (0.47), 0.813 (0.48), 0.820 (0.49), 0.903 (0.49), 1.230 (0.57), 1.653 (16.00), 2.049 (1.15), 2.057 (1.36), 2.074 (1.04), 2.475 (0.58), 2.518 (2.40), 2.523 (1.42), 3.382 (0.71), 3.408 (2.08), 3.421 (2.61), 3.446 (0.66), 3.496 (0.67), 3.504 (0.49), 3.511 (0.51), 3.521 (0.44), 4.162 (1.52), 4.180 (3.17), 4.198 (1.45), 6.331 (2.93), 6.431 (5.69), 7.004 (4.16), 7.426 (2.46), 7.439 (2.45), 8.127 (3.32), 8.133 (3.39), 8.390 (3.48), 8.403 (3.15), 8.419 (6.38), 8.601 (3.72), 8.607 (3.73).

### Example 797

### (3R)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (enantiomer 2)

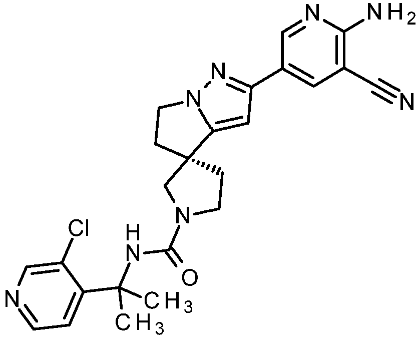

The racemic mixture of 2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide was separated into enantiomers by preparative chiral HPLC (method see Example 796) to give 20.0 mg (95 % purity, 29 % yield) of the title compound (enantiomer 2 Rₜ = 9.29 - 11.84 min).
Analytical chiral HPLC (method see Example 796): Rₜ = 5.43 min.
[α]²⁰_{D}: +131.5° (c = 1.00, DMSO)
LC-MS (Method 1): Rt = 0.90 min; MS (ESlpos): m/z = 477 [M+H]⁺
¹H NMR (DMSO-d₆, 400 MHz): δ (ppm) 8.60 (d, 1H), 8.42 (s, 1H), 8.40 (d, 1H), 8.13 (d, 1H), 7.43 (d, 1H), 7.00 (s, 2H), 6.43 (s, 1H), 6.33 (s, 1H), 4.18 (t, 2H), 3.47 - 3.54 (m, 1H), 3.37 - 3.46 (m, 3H), 2.51 - 2.55 (m, 1H), 1.99 - 2.11 (m, 2H), 1.65 (s, 6H).

### Example 798

### (3R)-2'-{6-amino-5-[(1R)-1-(3-chlorophenyl)ethoxy]pyridin-3-yl}-N-(propan-2-yl)-5',6'-dihydrospiro[pyrrolidine-3,4'-pyrrolo[1,2-b]pyrazole]-1-carboxamide

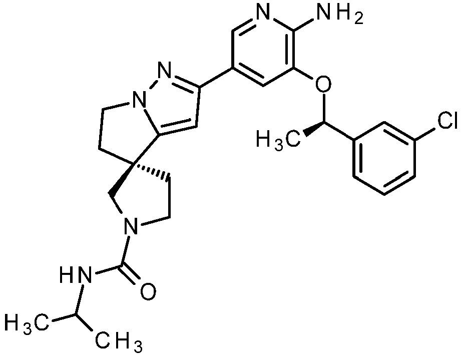

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (78.6 mg, 79 % purity, 157 µmol), 3-[(1R)-1-(3-chlorophenyl)ethoxy]-5-(4,4,5,5-tetramethyl-1,3,2-dioxaborolan-2-yl)pyridin-2-amine (80.0 mg, 88 % purity, 188 µmol) and potassium phosphate (940 µL, 0.50 M, 470 µmol) were dissolved in 1,4-dioxane (12 mL) under nitrogen. XPhos Pd G2 (12.3 mg, 15.7 µmol) was added and the mixture was stirred for 3 h at 100 °C. The cold reaction mixture was diluted with ethyl acetate and filtered over celite. The organic phase was washed with water and brine, dried over a hydrophobic filter and concentrated. The residue was purified by preparative HPLC to give 4.20 mg (94 % purity, 5 % yield) of the title compound.
LC-MS (Method 2): Rt = 0.94 min; MS (ESlpos): m/z = 495 [M+H]⁺
¹H NMR (DMSO-d₆, 400 MHz): δ (ppm) 7.86 (d, 1H), 7.58 (t, 1H), 7.43 - 7.47 (m, 1H), 7.38 (t, 1H), 7.29 - 7.33 (m, 1H), 7.27 (d, 1H), 6.25 (s, 1H), 5.91 (s, 2H), 5.81 (d, 1H), 5.64 (q, 1H), 4.13 (t, 2H), 3.70 - 3.80 (m, 1H), 3.45 - 3.52 (m, 1H), 3.37 - 3.42 (m, 3H), 1.96 - 2.08 (m, 2H), 1.56 (d, 3H), 1.07 (d, 3H), 1.06 (d, 3H).

### Example 799

### (3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)

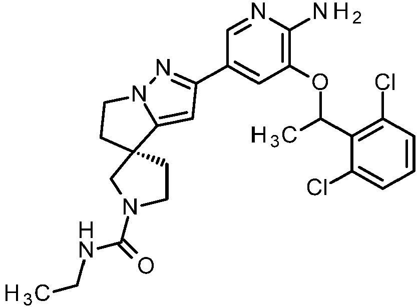

To a solution of (rac)-3-{[1-(2,6-dichlorophenyl)ethyl]oxy}-5-(4,4,5,5-tetramethyl-1,3,2-dioxaborolan-2-yl)pyridin-2-amine (380 mg, 929 µmol) in 1,4-dioxane were added (3R)-1-(ethylcarbamoyl)-5',6'-dihydrospiro[pyrrolidine-3,4'-pyrrolo[1,2-b]pyrazol]-2'-yl trifluoromethanesulfonate (391 mg, 1.02 mmol) and potassium phosphate (5.6 mL, 0.50 M, 2.8 mmol). The mixture was degassed with argon, XPhos Pd G2 (36.5 mg, 46.4 µmol) was added and stirred for 2 h at 100 °C. The reaction mixture was allowed to cool down, diluted with water and extracted twice with ethyl acetate. The combined organic layers were washed with brine, dried over sodium sulfate, filtered and concentrated. The residue was purified by flash column chromatography (silica gel, hexane / ethyl acetate (0-100 %) and dichloromethane / methanol (0-10%) gradient). The combined fractions were purified by preparative HPLC to give 38.0 mg (99 % purity, 8 % yield) of the title compound.
LC-MS (Method 1): Rt = 1.10 min; MS (ESlpos): m/z = 516 [M+H]⁺
¹H NMR (400 MHz, DMSO-*d*₆) δ ppm: 1.007 (2.09), 1.011 (2.53), 1.025 (4.78), 1.028 (5.62), 1.042 (2.41), 1.046 (2.66), 1.767 (5.50), 1.783 (5.60), 2.007 (0.85), 2.025 (1.46), 2.036 (1.25), 2.054 (0.48), 2.327 (0.40), 2.469 (1.65), 2.523 (3.36), 2.669 (0.41), 3.046 (0.97), 3.053 (1.10), 3.060 (1.14), 3.067 (1.39), 3.070 (1.26), 3.077 (1.08), 3.084 (1.10), 3.095 (0.40), 3.336 (16.00), 3.370 (4.41), 3.378 (2.82), 3.404 (0.70), 3.449 (0.66), 3.466 (0.64), 3.474 (0.56), 3.489 (0.42), 4.110 (1.49), 4.127 (2.51), 4.144 (1.52), 5.753 (3.61), 6.046 (1.35), 6.063 (1.39), 6.079 (0.46), 6.094 (3.82), 6.097 (3.89), 6.148 (0.60), 6.162 (1.17), 6.175 (0.64), 7.065 (2.48), 7.069 (2.63), 7.294 (0.77), 7.307 (0.76), 7.315 (1.30), 7.327 (1.22), 7.335 (1.32), 7.348 (1.10), 7.461 (2.91), 7.470 (2.63), 7.481 (2.19), 7.490 (1.87), 7.867 (3.09), 7.871 (3.35).

### Example 800

### (3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1)

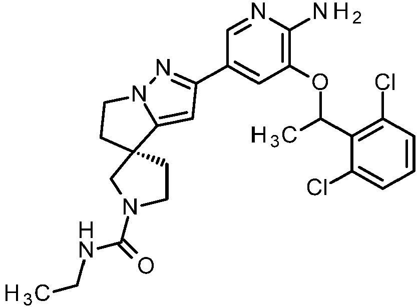

The title compound from **Example 799** was separated into diastereomers by preparative chiral HPLC to give 18.0 mg (95 % purity, 45 % yield) of the title compound (diastereomer 1 Rₜ = 6.4 - 10.2 min).

### Preparative chiral HPLC method:

Instrument: PrepCon Labomatic HPLC-3; Column: Chiralpak IF 5µ, 250x50; eluent A: methyl tert-butyl ether + 0.1 vol % diethylamine; eluent B: ethanol + 0.1 vol % diethylamine; isocratic: 40%B; flow:40 mL/min; temperature: 25 °C; UV: 280 nm

### Analytical chiral HPLC method:

Instrument: Thermo Fisher UltiMate 3000; Column: Chiralpak IF 3µ, 100x4.6; eluent A: methyl tert-butyl ether + 0.1 vol % diethylamine; eluent B: ethanol; isocratic: 40%B; flow: 1.4 mL/min; temperature: 25 °C; UV: 280 nm
Analytical chiral HPLC: Rₜ = 2.29 min
[α]²⁰_{D}: +49.9° (c = 1.00, DMSO)
LC-MS (Method 1): Rₜ = 1.08 min; MS (ESlpos): m/z = 515 [M+H]⁺
¹H NMR (DMSO-d₆, 400 MHz): δ (ppm) 7.87 (d, 1H), 7.48 (d, 2H), 7.30 - 7.35 (m, 1H), 7.07 (d, 1H), 6.16 (t, 1H), 6.10 (s, 1H), 6.05 (q, 1H), 5.75 (s, 2H), 4.13 (t, 2H), 3.42 - 3.49 (m, 1H), 3.35 - 3.41 (m, 3H), 3.02 - 3.10 (m, 2H), 1.98 - 2.06 (m, 2H), 1.77 (d, 3H), 1.02 (t, 3H).

### Example 801

### (3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 2)

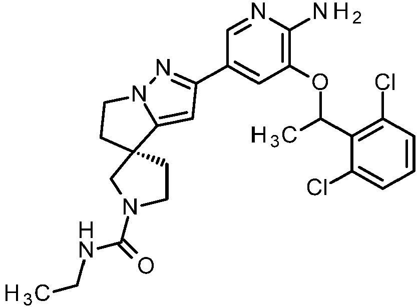

For the preparation of the diastereomeric mixture of the title compound see Example 799. The diastereomers were separated by preparative chiral HPLC (method see Example 800) to give 20.0 mg (95 % purity, 50 % yield) of the title compound (diastereomer 2 Rt = 14.1 - 19.9 min).
Analytical chiral HPLC (method see Example 800): Rt = 5.23 min.
[α]²⁰_{D}: +5.6° (c = 1.00, DMSO)
LC-MS (Method 1): Rt = 1.08 min; MS (ESlpos): m/z = 515 [M+H]⁺
¹H NMR (DMSO-d₆, 400 MHz): δ (ppm) 7.87 (d, 1H), 7.47 (d, 2H), 7.28 - 7.35 (m, 1H), 7.07 (d, 1H), 6.16 (t, 1H), 6.09 (s, 1H), 6.06 (q, 1H), 5.75 (s, 2H), 4.13 (t, 2H), 3.44 - 3.52 (m, 1H), 3.35 - 3.40 (m, 3H), 3.03 - 3.11 (m, 2H), 1.97 - 2.09 (m, 2H), 1.78 (d, 3H), 1.03 (t, 3H).

### Example 802

### 5-{(3R)-1-[1-(1H-pyrrol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

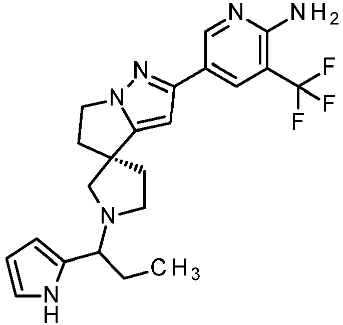

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (100 mg, 85 % purity, 236 µmol) and 1-(1H-pyrrol-2-yl)propan-1-one (43.6 mg, 354 µmol) were dissolved in methanol (2.5 mL). N,N-Diisopropylethylamine (160 µL, 950 µmol), titanium(IV) isopropoxide (210 µL, 710 µmol µmol) and sodium cyanoborohydride (44.5 mg, 709 µmol) were added and the mixture was stirred for 3 h at 60 °C. The reaction mixture was allowed to reach rt, diluted with water (0.5 mL) and stirred for 15 min. The suspension was filtered and the filter cake was washed with methanol. The filtrate was concentrated. The crude product was purified by preparative HPLC to give 4.50 mg (96 % purity, 4 % yield) of the title compound.
LC-MS (Method 1): Rt = 1.23 min; MS (ESlneg): m/z = 429 [M-H]⁻
¹H-NMR (400 MHz, DMSO-d6) δ [ppm]: 0.736 (6.82), 0.751 (13.00), 0.755 (13.38), 0.769 (7.03), 0.861 (0.47), 0.951 (5.14), 1.035 (2.42), 1.052 (4.41), 1.070 (2.31), 1.233 (0.70), 1.674 (2.09), 1.691 (2.08), 1.709 (1.39), 1.805 (2.24), 1.824 (2.25), 1.838 (1.75), 1.857 (1.26), 1.907 (4.62), 1.926 (6.80), 1.944 (3.67), 2.327 (4.75), 2.421 (4.24), 2.565 (9.85), 2.580 (8.03), 2.603 (3.04), 2.669 (6.60), 2.688 (2.20), 2.702 (3.72), 2.725 (2.78), 2.778 (1.06), 2.796 (1.84), 2.815 (1.71), 2.969 (0.44), 3.159 (3.28), 3.171 (3.45), 3.421 (2.06), 3.435 (1.97), 3.439 (1.92), 3.452 (1.62), 3.469 (0.73), 3.566 (1.28), 4.033 (1.68), 4.054 (4.79), 4.074 (6.62), 4.089 (3.86), 4.113 (1.56), 4.348 (0.81), 4.361 (1.50), 4.374 (0.72), 5.758 (0.40), 5.846 (5.24), 5.929 (6.16), 6.237 (8.92), 6.298 (8.32), 6.512 (16.00), 6.637 (5.51), 7.988 (7.77), 8.567 (5.86), 10.581 (3.30).

### Example 803

### (3R)-2'-[6-amino-5-(trifluoromethyl)pyridin-3-yl]-N-[1-(2-methoxypyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)

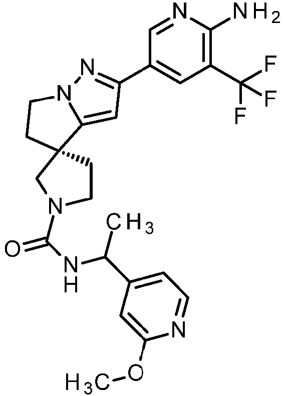

1-(2-Methoxypyridin-4-yl)ethan-1-amine (27.2 mg, 179 µmol), CDI (31.6 mg, 195 µmol) and N,N-diisopropylethylamine (110 µL, 650 µmol) were dissolved in DMF (1 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) in DMF (1 mL) was added and the mixture was stirred over night at rt. The reaction mixture was purified by preparative HPLC to give 40.0 mg (95 % purity, 47 % yield) of the title compound.
LC-MS (Method 1): Rt = 0.99 min; MS (ESlpos): m/z = 503 [M+H]⁺
¹H-NMR (400 MHz, DMSO-d6) δ [ppm]: 0.934 (0.84), 0.950 (0.82), 1.331 (4.51), 1.335 (4.22), 1.349 (4.58), 1.352 (4.17), 2.061 (0.89), 2.076 (1.67), 2.086 (1.31), 2.518 (1.89), 2.523 (1.62), 2.564 (1.96), 3.431 (1.09), 3.457 (2.55), 3.463 (3.21), 3.474 (1.17), 3.482 (1.25), 3.490 (0.56), 3.508 (0.48), 3.537 (0.54), 3.563 (0.51), 3.582 (0.57), 3.796 (10.93), 3.821 (16.00), 4.174 (1.86), 4.192 (3.18), 4.209 (1.79), 4.750 (0.87), 4.768 (1.31), 4.787 (0.86), 6.453 (2.75), 6.462 (5.27), 6.547 (4.76), 6.573 (0.93), 6.705 (1.65), 6.707 (1.80), 6.739 (2.06), 6.741 (2.23), 6.924 (0.98), 6.927 (0.97), 6.937 (1.02), 6.941 (1.00), 6.953 (1.18), 6.956 (1.16), 6.966 (1.23), 6.969 (1.17), 8.012 (2.73), 8.018 (2.82), 8.034 (1.52), 8.047 (1.51), 8.054 (2.02), 8.067 (1.90), 8.587 (2.71).

### Example 804

### (3R)-2'-[6-amino-5-(trifluoromethyl)pyridin-3-yl]-N-[1-(1-methyl-1H-pyrazol-5-yl)ethyl]-5',6'-dihydrospiro[pyrrolidine-3,4'-pyrrolo[1,2-b]pyrazole]-1-carboxamide (diastereomer 1)

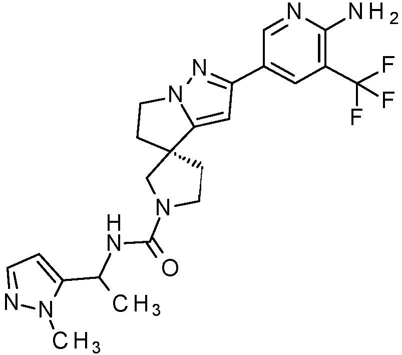

1-(1-Methyl-1H-pyrazol-5-yl)ethanamine (41 mg, 330 µmol), CDI (58 mg, 360 µmol) and N,N-diisopropylethylamine (209 µL, 1.2 mmol) were dissolved in DMF (1 mL) and stirred for 1 h at room temperature. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (135 mg, 300 µmol) in DMF was added dropwise and the mixture was stirred over night at room temperature. The reaction mixture was poured into water and extracted with ethyl acetate. The combined organic layers were dried over a hydrophobic filter and concentrated. The residue was purified by flash column chromatography (silica gel, dichloromethane / methanol (0-8%) gradient to give 144 mg (98 % purity, 99 % yield) of the title compound as a mixture of diastereomers.

The mixture was separated into diastereomers by preparative chiral HPLC to give 36.1 mg (90 % purity) of the title compound (diastereomer 1 Rₜ = 4 - 5.4 min).

### Preparative chiral HPLC method:

Instrument: Sepiatec: Prep SFC100; Column: Chiral Art Cellulose-SB 5µ, 250x50; eluent A: CO2; eluent B: 2-propanol; isocratic: 30%B; flow: 100 mL/min; temperature: 40 °C; BPR: 150 bar UV: 271 nm

### Analytical chiral HPLC method:

Instrument: Waters Acquity UPC2 QDA; Column: Chiral Art Cellulose-SB 3µ, 100x4.6; eluent A: CO2; eluent B: 2-propanol + 0.4 vol % diethylamine; isocratic: 30%B; flow: 4 mL/min; temperature: 40 °C; BPR: 100 bar; UV: 254 nm
Analytical chiral HPLC: Rₜ = 1.07 min
[α]²⁰_{D}: +63.2° (c = 1.00, methanol)
LC-MS (Method 1): Rt = 0.91 min; MS (ESlpos): m/z = 475 [M+H]⁺
¹H NMR (DMSO-d₆, 400 MHz): δ (ppm) 8.56 - 8.60 (m, 1H), 8.00 (d, 1H), 7.25 (d, 1H), 6.55 (s, 2H), 6.48 (d, 1H), 6.45 (s, 1H), 6.16 (d, 1H), 5.00 (quin, 1H), 4.18 (t, 2H), 3.75 (s, 3H), 3.55 (dt, 1H), 3.36 - 3.47 (m, 3H), 2.00 - 2.11 (m, 2H), 1.41 (d, 3H).

### Example 805

### (3R)-2'-[6-amino-5-(trifluoromethyl)pyridin-3-yl]-N-[1-(1-methyl-1H-pyrazol-5-yl)ethyl]-5',6'-dihydrospiro[pyrrolidine-3,4'-pyrrolo[1,2-b]pyrazole]-1-carboxamide (diastereomer 2)

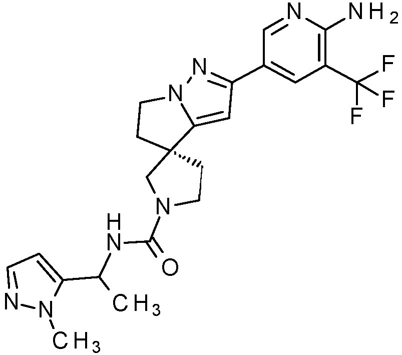

For the preparation of the diastereomeric mixture of the title compound see **Example 804.** The diastereomers were separated by preparative chiral HPLC (method see **Example** 804) to give 35.8 mg (97 % purity) of the title compound (diastereomer 2 Rₜ = 8.5 - 10.5 min).
Analytical chiral HPLC (method see **Example** 804): Rt = 2.43 min.
[α]²⁰_{D}: +76.8° (c = 1.00, methanol)
LC-MS (Method 1): Rₜ = 0.91 min; MS (ESlpos): m/z = 475 [M+H]⁺
¹H NMR (DMSO-d₆, 400 MHz): δ (ppm) 8.58 (d, 1H), 8.00 (d, 1H), 7.27 (d, 1H), 6.55 (s, 2H), 6.46 (d, 1H), 6.44 (s, 1H), 6.19 (d, 1H), 4.95 - 5.05 (m, 1H), 4.18 (t, 2H), 3.77 (s, 3H), 3.48 - 3.56 (m, 1H), 3.39 - 3.48 (m, 3H), 2.00 - 2.11 (m, 2H), 1.40 (d, 3H).

### Example 806

### 3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzamide (diastereomer 1 and diastereomer 2)

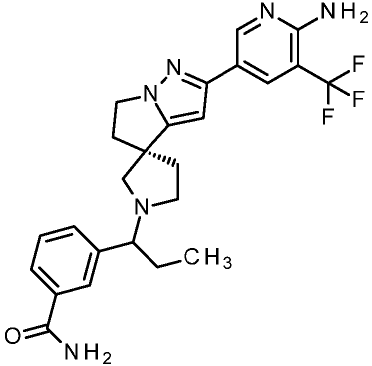

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (150 mg, 95 % purity, 396 µmol) and 3-propanoylbenzamide (105 mg, 594 µmol) were dissolved in methanol (4.2 mL). N,N-Diisopropylethylamine (280 µL, 1.6 mmol), titanium(IV) isopropoxide (350 µL, 1.2 mmol) and sodium cyanoborohydride 74.7 mg, 1.19 mmol) were added and the mixture was stirred for 16 h at 60 °C. The reaction mixture was allowed to rach rt, diluted with water (0.5 mL) and stirred for 15 min. The suspension was filtered and the filter cake was washed with methanol. The filtrate was concentrated. The crude product was purified by preparative HPLC to give 98.0 mg (100 % purity, 51 % yield) of the title compound.
LC-MS (Method 1): Rt = 1.14 min; MS (ESlpos): m/z = 486 [M+H]⁺
¹H-NMR (400 MHz, DMSO-d6) δ [ppm]: 0.628 (7.13), 0.646 (16.00), 0.664 (7.52), 0.930 (1.91), 0.947 (1.93), 1.640 (0.75), 1.658 (1.25), 1.674 (1.46), 1.691 (1.49), 1.710 (0.86), 1.870 (0.78), 1.888 (1.25), 1.899 (1.53), 1.916 (1.64), 1.932 (1.51), 1.949 (1.71), 1.976 (2.68), 1.992 (4.42), 2.011 (2.93), 2.043 (0.43), 2.323 (1.42), 2.327 (1.97), 2.331 (1.46), 2.451 (1.17), 2.518 (15.08), 2.522 (9.11), 2.558 (6.59), 2.565 (7.32), 2.584 (2.48), 2.599 (1.43), 2.616 (0.72), 2.659 (1.28), 2.665 (1.90), 2.669 (2.70), 2.673 (2.53), 2.678 (2.23), 2.693 (1.34), 2.715 (0.57), 2.740 (2.89), 2.763 (2.38), 2.812 (0.58), 2.827 (1.07), 2.849 (1.08), 2.867 (0.46), 3.159 (0.97), 3.172 (0.99), 3.241 (2.21), 3.247 (2.02), 3.253 (2.08), 3.262 (2.11), 4.025 (0.67), 4.034 (1.10), 4.052 (1.82), 4.076 (3.78), 4.092 (5.38), 4.111 (2.78), 6.418 (9.19), 6.439 (11.09), 6.514 (10.89), 7.342 (3.32), 7.373 (1.89), 7.392 (4.41), 7.407 (3.89), 7.411 (3.52), 7.426 (2.51), 7.455 (5.68), 7.474 (3.20), 7.737 (2.76), 7.748 (2.62), 7.752 (2.88), 7.756 (2.75), 7.766 (2.19), 7.794 (3.70), 7.814 (4.44), 7.968 (4.23), 7.998 (3.71), 8.007 (4.82), 8.013 (4.34), 8.577 (3.28), 8.580 (3.42), 8.588 (4.11), 8.592 (3.89).

### Example 807

### 3-(1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}propyl)benzamide (diastereomer 1)

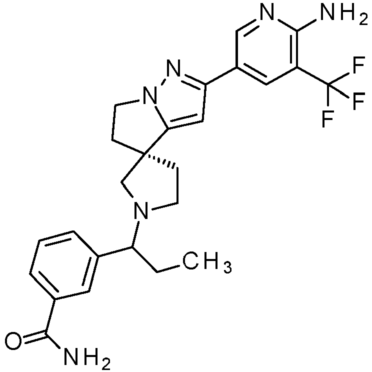

The compound 3-(1-{(3R)-2'-[6-amino-5-(trifluoromethyl)pyridin-3-yl]-5' ,6'-dihydrospiro[pyrrolidine-3,4'-pyrrolo[1,2-b]pyrazol]-1-yl}propyl)benzamide (diastereomer 1 and diastereomer 2, Example 806) was separated into diastereomers by preparative chiral HPLC to give 45.0 mg (98 % purity, 23 % yield) of the title compound (diastereomer 1 Rt = 4.1 - 4.8 min).

### Preparative chiral HPLC method:

Instrument: PrepCon Labomatic HPLC-3; Column: YMC Amylose SA 5µ, 250x50; eluent A: methyl tert-butyl ether + 0.1 vol % diethylamine; eluent B: methanol; gradient; flow: 50 mL/min; temperature: 25 °C; UV: 280 nm

### Analytical chiral HPLC method:

Instrument: Thermo Fisher UltiMate 3000; Column: YMC Amylose SA 3µ, 100x4.6; eluent A: methyl tert-butyl ether + 0.1 vol % diethylamine; eluent B: methanol; gradient; flow: 1.4 mL/min; temperature: 25 °C; UV: 280 nm
Analytical chiral HPLC Rₜ = 2.73 min
[α]²²_{D}: +70.4° (c = 1.00, methanol)
LC-MS (Method 1): Rₜ = 1.11 min; MS (ESlpos): m/z = 485 [M+H]⁺
¹H NMR (400 MHz, DMSO-*d*₆) δ ppm 0.65 (t, 3 H), 1.61 - 1.74 (m, 1 H), 1.86 - 2.03 (m, 3 H), 2.53 - 2.60 (m, 3 H), 2.80 - 2.87 (m, 1 H), 3.25 (dd, 1 H), 4.00 - 4.13 (m, 2 H), 6.42 (s, 1 H), 6.51 (s, 2 H), 7.35 (s, 1 H), 7.38 - 7.43 (m, 1 H), 7.45 - 7.48 (m, 1 H), 7.76 (dt, 1 H), 7.79 (s, 1 H), 7.97 (s, 1 H), 8.00 (d, 1 H), 8.58 (d, 1 H).

### Example 808

### 3-(1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}propyl)benzamide (diastereomer 2)

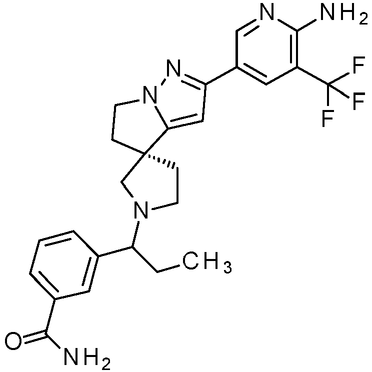

The diastereomers were separated by preparative chiral HPLC (method see Example 807) to give 47.0 mg (96 % purity, 24 % yield) of the title compound (diastereomer 2 Rt = 5.2 - 6.0 min).
Analytical chiral HPLC (method see Example 807): Rt = 3.87 min.
[α]²²_{D}: +72.2° (c = 1.00, methanol)
LC-MS (Method 1): Rt = 1.12 min; MS (ESlpos): m/z = 485 [M+H]⁺
¹H NMR (400 MHz, DMSO-*d*₆) δ ppm 0.65 (t, 3 H), 1.62 - 1.74 (m, 1 H), 1.85 - 2.05 (m, 3 H), 2.53 - 2.62 (m, 2 H), 2.65 - 2.72 (m, 1 H), 2.75 (d, 1 H), 3.25 (dd, 1 H), 4.09 (t, 2 H), 6.44 (s, 1 H), 6.51 (s, 2 H), 7.34 (s, 1 H), 7.37 - 7.42 (m, 1 H), 7.45 - 7.48 (m, 1 H), 7.75 (dt, 1 H), 7.81 (s, 1 H), 7.96 (s, 1 H), 8.01 (d, 1 H), 8.59 (d, 1 H).

### Example 809

### (3R)-2'-{6-amino-5-[(1R)-1-(1,3-thiazol-2-yl)ethoxy]pyridin-3-yl}-N-cyclobutyl-5',6'-dihydrospiro[pyrrolidine-3,4'-pyrrolo[1,2-b]pyrazole]-1-carboxamide

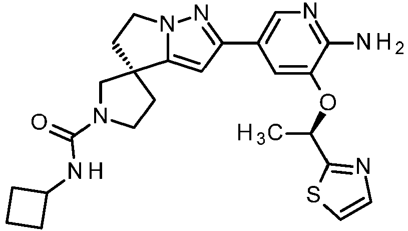

5-(4,4,5,5-Tetramethyl-1,3,2-dioxaborolan-2-yl)-3-[(1R)-1-(1,3-thiazol-2-yl)ethoxy]pyridin-2-amine (264 mg, 49 % purity, 374 µmol) was dissolved in 1,4-dioxane (2.1 mL). (3R)-1-(cyclobutylcarbamoyl)-5',6'-dihydrospiro[pyrrolidine-3,4'-pyrrolo[1,2-b]pyrazol]-2'-yl trifluoromethanesulfonate (127 mg, 312 µmol), potassium phosphate (1.9 mL, 0.50 M, 940 µmol) and XPhos Pd G2 (24.5 mg, 31.2 µmol) were added and the mixture was stirred for 1 h at 100 °C. The cold reaction mixture was diluted with dichloromethane. The organic phase was washed with water and brine, dried over a hydrophobic filter and concentrated. The residue was purified by preparative HPLC to give 54.0 mg (98 % purity, 35 % yield) of the title compound.
LC-MS (Method 1): Rt = 0.91 min; MS (ESlpos): m/z = 480 [M+H]⁺
¹H-NMR (400 MHz, DMSO-d6) δ [ppm]: 1.484 (0.72), 1.504 (1.44), 1.511 (1.18), 1.523 (1.24), 1.530 (2.75), 1.537 (1.24), 1.551 (2.39), 1.575 (1.78), 1.627 (0.49), 1.643 (0.48), 1.703 (15.59), 1.718 (15.89), 1.731 (0.90), 1.888 (1.26), 1.894 (1.21), 1.912 (2.26), 1.916 (2.16), 1.935 (2.22), 1.961 (1.30), 1.989 (0.64), 2.005 (1.68), 2.019 (2.62), 2.032 (3.36), 2.050 (1.55), 2.061 (1.52), 2.068 (1.48), 2.080 (2.62), 2.087 (3.09), 2.095 (2.21), 2.099 (2.06), 2.106 (2.56), 2.114 (1.87), 2.126 (0.87), 2.133 (0.66), 2.337 (0.51), 2.476 (4.46), 2.518 (7.31), 2.523 (5.10), 3.354 (1.53), 3.380 (13.87), 3.397 (2.81), 3.416 (1.07), 3.452 (1.04), 3.471 (1.90), 3.478 (1.10), 3.485 (1.33), 3.496 (1.09), 3.511 (0.62), 4.081 (0.98), 4.101 (1.86), 4.123 (5.33), 4.140 (6.64), 4.157 (3.52), 5.539 (0.46), 5.837 (8.98), 5.866 (1.22), 5.882 (4.08), 5.898 (4.07), 5.914 (1.08), 6.283 (16.00), 6.297 (3.39), 6.316 (3.17), 7.409 (6.10), 7.414 (6.01), 7.710 (9.93), 7.719 (10.91), 7.784 (0.61), 7.791 (12.47), 7.799 (9.10), 7.937 (8.84), 7.941 (8.54).

### Example 810

### (3R)-2'-{6-amino-5-[(1R)-1-(3-chlorophenyl)ethoxy]pyridin-3-yl}-N-ethyl-5',6'-dihydrospiro[pyrrolidine-3,4'-pyrrolo[1,2-b]pyrazole]-1-carboxamide

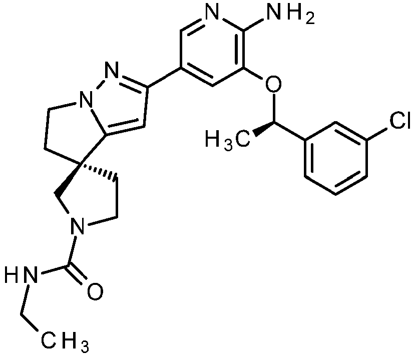

(3R)-1-(Ethylcarbamoyl)-5',6'-dihydrospiro[pyrrolidine-3,4'-pyrrolo[1,2-b]pyrazol]-2'-yl trifluoromethanesulfonate (66.8 mg, 95 % purity, 166 µmol), 3-[(1R)-1-(3-chlorophenyl)ethoxy]-5-(4,4,5,5-tetramethyl-1,3,2-dioxaborolan-2-yl)pyridin-2-amine (91.0 mg, 82 % purity, 199 µmol) and potassium phosphate (1000 µL, 0.50 M, 500 µmol) were dissolved in 1,4-dioxane (10 mL) under argon. XPhos Pd G2 (13.1 mg, 16.6 µmol) was added and the mixture was stirred for 4 h at 100 °C. The cold reaction mixture was diluted with ethyl acetate and filtered over celite. The organic phase was washed with water and brine, dried over a hydrophobic filter and concentrated. The residue was purified twice by preparative HPLC to give 9.70 mg (94 % purity, 11 % yield) of the title compound.
[α]²⁰_{D}: +139.3° (c = 1.00, methanol)
LC-MS (Method 2): Rₜ = 0.88 min; MS (ESlpos): m/z = 481 [M+H]⁺
¹H NMR (DMSO-d₆, 400 MHz): δ (ppm) 7.87 (d, 1H), 7.58 (t, 1H), 7.43 - 7.47 (m, 1H), 7.37 (t, 1H), 7.29 - 7.33 (m, 1H), 7.27 (d, 1H), 6.25 (s, 1H), 6.14 (t, 1H), 5.91 (s, 2H), 5.64 (q, 1H), 4.13 (t, 2H), 3.44 - 3.52 (m, 1H), 3.36 - 3.42 (m, 3H), 3.01 - 3.09 (m, 2H), 1.97 - 2.09 (m, 2H), 1.56 (d, 3H), 1.01 (t, 3H).

### Example 811

### (3R)-2'-[6-amino-5-(trifluoromethyl)pyridin-3-yl]-N-[1-(2-chloro-3-fluoropyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)

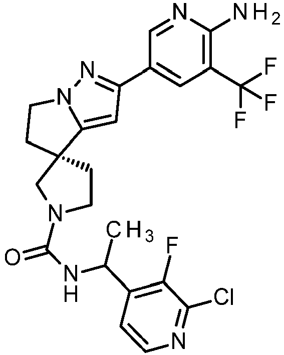

1-(2-Chloro-3-fluoropyridin-4-yl)ethan-1-amine hydrogen chloride (1/1) (64.5 mg, 306 µmol) was dissolved in DMF (3 mL). CDI (54.1 mg, 334 µmol) and N,N-diisopropylethylamine (190 µL, 1.1 mmol) were added and stirred for 1 h at rt. 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (125 mg, 80 % purity, 278 µmol) was added and the mixture was stirred for 3 h at 60 °C. The reaction mixture was allowed to cool down and purified by preparative HPLC followed by preparative TLC to give 8.00 mg (95 % purity, 5 % yield) of the title compound.
LC-MS (Method 1): Rt = 1.03 min; MS (ESlpos): m/z = 524 [M+H]⁺
¹H-NMR (400 MHz, DMSO-d6) δ [ppm]: 1.227 (1.15), 1.375 (9.30), 1.379 (9.22), 1.392 (9.56), 1.397 (8.93), 2.080 (3.69), 2.518 (2.07), 2.525 (3.83), 2.560 (3.73), 3.410 (0.66), 3.428 (2.88), 3.454 (5.77), 3.481 (5.12), 3.507 (2.59), 3.531 (1.32), 3.546 (0.86), 3.556 (0.72), 3.572 (0.80), 3.589 (1.02), 3.604 (0.76), 3.614 (0.77), 4.174 (3.51), 4.191 (6.19), 4.206 (3.23), 5.028 (0.44), 5.045 (1.75), 5.063 (2.61), 5.078 (1.70), 5.081 (1.69), 5.097 (0.42), 6.470 (16.00), 6.551 (11.59), 6.766 (2.24), 6.778 (2.40), 6.784 (2.52), 6.796 (1.93), 7.460 (1.47), 7.472 (2.83), 7.485 (1.51), 7.528 (1.67), 7.541 (3.21), 7.554 (1.70), 8.018 (6.08), 8.196 (3.69), 8.208 (3.52), 8.227 (5.69), 8.239 (5.46), 8.590 (6.45), 8.594 (6.29).

### Example 812

### (3R)-2'-[6-amino-5-(trifluoromethyl)pyridin-3-yl]-N-{[3-(methylsulfanyl)pyridin-4-yl]methyl}-5',6'-dihydrospiro[pyrrolidine-3,4'-pyrrolo[1,2-b]pyrazole]-1-carboxamide

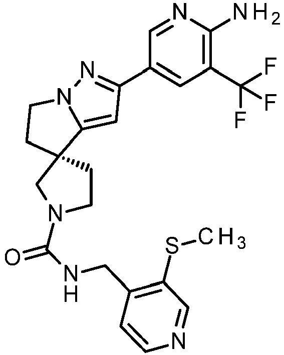

1-[3-(Methylsulfanyl)pyridin-4-yl]methanamine (27.6 mg, 179 µmol) was dissolved in DMF (1 mL). CDI (31.6 mg, 195 µmol) and N,N-diisopropylethylamine (110 µL, 650 µmol) were added and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) in DMF (1 mL) was added and the mixture was stirred over night at rt. The reaction mixture was allowed to cool down and purified by preparative HPLC to give 23.0 mg (99 % purity, 28 % yield) of the title compound.
LC-MS (Method 1): Rt = 0.94 min; MS (ESlpos): m/z = 505 [M+H]⁺
¹H-NMR (400 MHz, DMSO-d6) δ [ppm]: 2.101 (0.55), 2.119 (0.96), 2.518 (1.39), 2.523 (0.99), 2.568 (16.00), 2.591 (1.15), 3.488 (0.74), 3.500 (4.13), 3.513 (0.86), 3.596 (0.61), 4.186 (1.17), 4.204 (1.91), 4.217 (2.23), 4.230 (1.93), 6.509 (4.55), 6.554 (2.84), 6.863 (0.44), 6.877 (0.88), 6.892 (0.42), 7.211 (1.28), 7.223 (1.29), 8.028 (1.65), 8.033 (1.64), 8.328 (2.50), 8.341 (2.40), 8.417 (3.92), 8.603 (1.53), 8.607 (1.51).

### Example 813

### (3R)-2'-{6-amino-5-[(1R)-1-(1,3-thiazol-2-yl)ethoxy]pyridin-3-yl}-N-ethyl-5',6'-dihydrospiro[pyrrolidine-3,4'-pyrrolo[1,2-b]pyrazole]-1-carboxamide

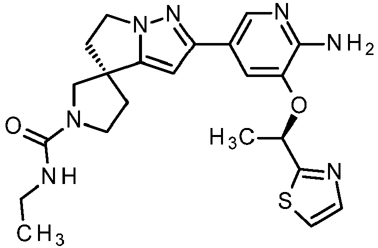

5-(4,4,5,5-Tetramethyl-1,3,2-dioxaborolan-2-yl)-3-[(1R)-1-(1,3-thiazol-2-yl)ethoxy]pyridin-2-amine (247 mg, 46 % purity, 327 µmol) was dissolved in 1,4-dioxane (1.8 mL) under argon. (3R)-1-(Ethylcarbamoyl)-5',6'-dihydrospiro[pyrrolidine-3,4'-pyrrolo[1,2-b]pyrazol]-2'-yl trifluoromethanesulfonate (104 mg, 273 µmol), potassium phosphate (1.6 mL, 0.50 M, 820 µmol) and XPhos Pd G2 (10.7 mg, 13.6 µmol) were added and the mixture was stirred for 2 h at 100 °C. The reaction mixture was allowed to cool down, diluted with dichloromethane and washed with water and brine. The organic phase was dried over a hydrophobic filter and concentrated. The crude product was purified by preparative HPLC to give 20.0 mg (99 % purity, 16 % yield) of the title compound.
LC-MS (Method 2): Rt = 0.64 min; MS (ESlpos): m/z = 454 [M+H]⁺
¹H-NMR (400MHz, DMSO-d6): δ [ppm]= 1.02 (t, 3H), 1.71 (d, 3H), 1.97 - 2.10 (m, 2H), 3.01 - 3.09 (m, 2H), 3.35 - 3.42 (m, 3H), 3.44 - 3.51 (m, 1H), 4.14 (t, 2H), 5.84 (s, 2H), 5.89 (q, 1H), 6.15 (t, 1H), 6.28 (s, 1H), 7.41 (d, 1H), 7.71 (d, 1H), 7.79 (d, 1H), 7.94 (d, 1H).

### Example 814

### (3R)-2'-[6-amino-5-(trifluoromethyl)pyridin-3-yl]-N-[1-(1,3-dimethyl-1H-pyrazol-4-yl)ethyl]-5',6'-dihydrospiro[pyrrolidine-3,4'-pyrrolo[1,2-b]pyrazole]-1-carboxamide (diastereomer 1)

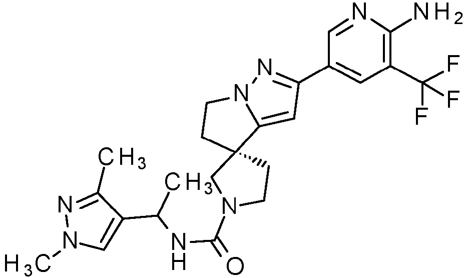

1-(1,3-Dimethyl-1H-pyrazol-4-yl)ethanamine (46 mg, 330 µmol), CDI (58 mg, 360 µmol) and N,N-diisopropylethylamine (209 µL, 1.2 mmol) were dissolved in DMF (1 mL) and stirred for 1 h at rt. A solution of 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (135 mg, 300 µmol) in DMF was added dropwise and the mixture was stirred over night at rt. The reaction mixture was poured into water and extracted with ethyl acetate. The combined organic layers were dried over a hydrophobic filter and concentrated. The residue was purified by flash column chromatography (silica gel, dichloromethane / methanol (0-8%) gradient to give 143 mg (97 % yield) of the title compound as a mixture of diastereomers.

The mixture was separated into diastereomers by preparative chiral HPLC to give 36.6 mg (100 % purity) of the title compound (diastereomer 1 Rₜ = 8.4 - 10.3 min).

### Preparative chiral HPLC method:

Instrument: Sepiatec: Prep SFC100; Column: Chiral Art Cellulose-SC 5µ, 250x50; eluent A: CO2; eluent B: methanol; isocratic: 30%B; flow: 100 mL/min; temperature: 40 °C; BPR: 150 bar UV: 271 nm

### Analytical chiral HPLC method:

Instrument: Waters Acquity UPC2 QDA; Column: Chiral Art Cellulose-SC 3µ, 100x4.6; eluent A: CO2; eluent B: methanol + 0.2 vol % aqueous ammonia (32%); isocratic: 30%B; flow: 4 mL/min; temperature: 40 °C; BPR: 100 bar; UV: 280 nm
Analytical chiral HPLC: Rₜ = 2.24 min (ee value: > 99.9 %)
[α]²⁰_{D}: +58.8° (c = 1.00, methanol)
LC-MS (Method 1): Rt = 0.90 min; MS (ESlpos): m/z = 489 [M+H]⁺
¹H NMR (DMSO-d₆, 400 MHz): δ (ppm) 8.58 (d, 1H), 8.00 (d, 1H), 7.45 (s, 1H), 6.55 (s, 2H), 6.44 (s, 1H), 6.09 (d, 1H), 4.70 - 4.79 (m, 1H), 4.18 (t, 2H), 3.69 (s, 3H), 3.36 - 3.53 (m, 4H), 1.98 - 2.11 (m, 5H), 1.31 (d, 3H).

### Example 815

### (3R)-2'-[6-amino-5-(trifluoromethyl)pyridin-3-yl]-N-[1-(1,3-dimethyl-1H-pyrazol-4-yl)ethyl]-5',6'-dihydrospiro[pyrrolidine-3,4'-pyrrolo[1,2-b]pyrazole]-1-carboxamide (diastereomer 2)

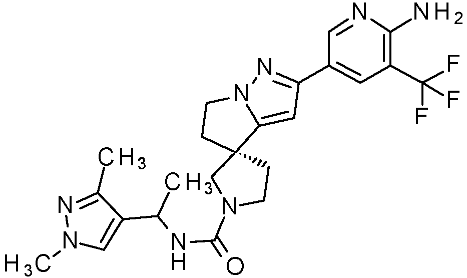

For the preparation of the diastereomeric mixture of the title compound see **Example 814.** The diastereomers were separated by preparative chiral HPLC (method see **Example 814)** to give 36.8 mg (100 % purity) of the title compound (diastereomer 2 Rₜ = 14.3 - 18.0 min).
Analytical chiral HPLC (method see **Example 814):** Rt = 3.90 min (ee value: > 99.9 %)
[α]²⁰_{D}: +67.4° (c = 1.00, methanol)
LC-MS (Method 1): Rt = 0.89 min; MS (ESlpos): m/z = 489 [M+H]⁺
¹H NMR (DMSO-d₆, 400 MHz): δ (ppm) 8.58 (d, 1H), 8.01 (d, 1H), 7.43 (s, 1H), 6.54 (s, 2H), 6.44 (s, 1H), 6.10 (d, 1H), 4.71 - 4.80 (m, 1H), 4.17 (t, 2H), 3.67 (s, 3H), 3.54 (dt, 1H), 3.36 - 3.47 (m, 3H), 1.99 - 2.10 (m, 5H), 1.32 (d, 3H).

### Example 816

### (3R)-2'-{6-amino-5-[(1R)-1-(3-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide

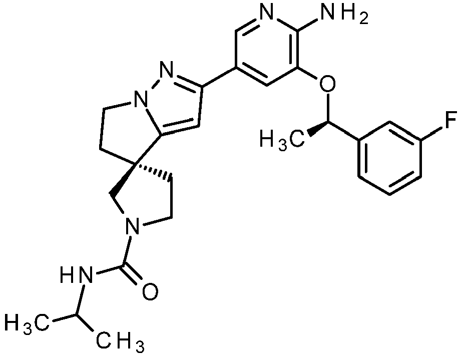

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (69.0 mg, 79 % purity, 137 µmol), 3-[(1R)-1-(3-fluorophenyl)ethoxy]-5-(4,4,5,5-tetramethyl-1,3,2-dioxaborolan-2-yl)pyridin-2-amine (104 mg, 71 % purity, 206 µmol) and potassium phosphate (820 µL, 0.50 M, 410 µmol]) were dissolved in 1,4-dioxane (10 mL) under argon. XPhos Pd G2 (10.8 mg, 13.7 µmol) was added and the mixture was stirred for 2 h at 100 °C. The cold reaction mixture was diluted with ethyl acetate and filtered over celite. The organic phase was washed with water and brine, dried over a hydrophobic filter and concentrated. The residue was purified by preparative HPLC to give 22.0 mg (98 % purity, 33 % yield) of the title compound.
[α]²⁰_{D}: +128.5° (c = 1.00, methanol)
LC-MS (Method 2): Rt = 0.89 min; MS (ESlpos): m/z = 479 [M+H]⁺
¹H NMR (DMSO-d₆, 400 MHz): δ (ppm) 7.86 (d, 1H), 7.29 - 7.42 (m, 3H), 7.27 (d, 1H), 7.05 - 7.10 (m, 1H), 6.25 (s, 1H), 5.90 (s, 2H), 5.81 (d, 1H), 5.64 (q, 1H), 4.13 (t, 2H), 3.69 - 3.82 (m, 1H), 3.45 - 3.52 (m, 1H), 3.36 - 3.42 (m, 3H), 1.96 - 2.08 (m, 2H), 1.56 (d, 3H), 1.07 (d, 3H), 1.05 (d, 3H).

### Example 817

### (3R)-2'-[6-amino-5-(trifluoromethyl)pyridin-3-yl]-N-[1-(pyridin-2-yl)cyclopropyl]-5',6'-dihydrospiro[pyrrolidine-3,4'-pyrrolo[1,2-b]pyrazole]-1-carboxamide

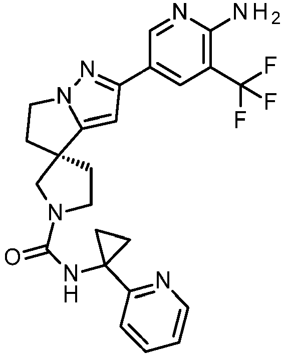

1-(Pyridin-2-yl)cyclopropan-1-amine (24.0 mg, 179 µmol), CDI (31.6 mg, 195 µmol) and N,N-diisopropylethylamine (110 µL, 650 µmol) were dissolved in DMF (2 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) in DMF was added and the mixture was stirred over night at rt. The reaction mixture was purified by preparative HPLC to give 32.0 mg (99 % purity, 40 % yield) of the title compound.
LC-MS (Method 1): Rt = 0.94 min; MS (ESlpos): m/z = 485 [M+H]⁺
¹H-NMR (400 MHz, DMSO-d6) δ [ppm]: 1.136 (2.60), 1.145 (6.72), 1.154 (6.84), 1.162 (3.19), 1.416 (3.22), 1.425 (7.62), 1.433 (6.41), 1.443 (2.89), 2.074 (0.94), 2.084 (1.63), 2.097 (2.58), 2.111 (3.01), 2.129 (1.33), 2.331 (2.04), 2.337 (0.89), 2.518 (11.95), 2.523 (8.37), 2.570 (5.59), 2.588 (3.84), 2.673 (2.00), 2.678 (0.88), 3.447 (0.76), 3.465 (2.01), 3.486 (13.95), 3.509 (1.09), 3.573 (0.98), 3.591 (1.88), 3.599 (1.21), 3.606 (1.35), 3.617 (1.18), 3.631 (0.68), 4.188 (3.93), 4.205 (6.14), 4.222 (3.76), 6.513 (16.00), 6.558 (9.86), 7.087 (3.18), 7.090 (3.01), 7.100 (3.10), 7.102 (3.42), 7.106 (3.32), 7.109 (3.14), 7.118 (3.65), 7.121 (3.86), 7.130 (5.74), 7.371 (2.93), 7.374 (5.06), 7.376 (2.91), 7.391 (3.30), 7.393 (5.19), 7.636 (2.83), 7.641 (3.12), 7.655 (3.75), 7.660 (3.63), 7.674 (2.24), 7.679 (2.30), 8.025 (5.64), 8.030 (5.71), 8.389 (3.44), 8.392 (3.88), 8.394 (3.72), 8.396 (3.49), 8.401 (3.54), 8.404 (3.94), 8.406 (3.48), 8.408 (3.15), 8.604 (5.32), 8.608 (5.22).

### Example 818

### 5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

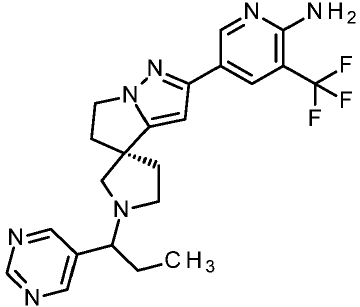

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1:1) (100 mg, 264 µmol) and 1-(pyrimidin-5-yl)propan-1-one (54 mg, 396 µmol) were dissolved in methanol (2.8 mL). N,N-Diisopropylethylamine (184 µL, 1.1 mmol), titanium(IV) isopropoxide (234 µL, 792 µmol) were added and stirred for 3 h at 60 °C. Sodium cyanoborohydride (49.8 mg, 792 µmol) was added at rt and the mixture was stirred 3 h at 60 °C. The reaction mixture was allowed to reach rt, diluted with water (0.5 mL) and stirred for 15 min. The suspension was filtered and the filter cake was washed with methanol. The filtrate was concentrated. The crude product was purified by preparative HPLC to give 67.0 mg (100 % purity, 38 % yield) of the title compound.
LC-MS (Method 1): Rt = 1.06 min; MS (ESlpos): m/z = 444 [M+H]⁺
¹H-NMR (400MHz, DMSO-d₆): δ [ppm]= 0.65 - 0.72 (m, 3H), 1.67 - 1.80 (m, 1H), 1.87 - 2.07 (m, 3H), 2.53 - 2.62 (m, 4H), 2.66 - 2.86 (m, 2H), 3.35 - 3.39 (m, 1H), 4.04 - 4.14 (m, 2H), 6.45 and 6.46 (2s, 1H), 6.52 (2br s, 2H), 7.99 - 8.03 (m, 1H), 8.58 - 8.61 (m, 1H), 8.74 - 8.78 (m, 2H), 9.09 and 9.11 (2s, 1H).

### Example 819

### 5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)

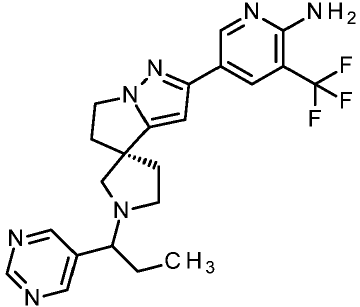

The title compound from **Example 818** was separated into diastereomers by preparative chiral HPLC to give 18.0 mg (95 % purity, 45 % yield) of the title compound (diastereomer 1 Rₜ = 14.3 - 16.3 min).

### Preparative chiral HPLC method:

Instrument: PrepCon Labomatic HPLC-2; Column: YMC Cellulose SC 5µ, 250x50; eluent A: hexane + 0.1 vol % diethylamine; eluent B: ethanol; isocratic: 50%B; flow: 60 mL/min; temperature: 25 °C; UV: 280 nm

### Analytical chiral HPLC method:

Instrument: Thermo Fisher UltiMate 3000; Column: YMC Cellulose SC 3µ, 100x4.6; eluent A: hexane + 0.1 vol % diethylamine; eluent B: ethanol; isocratic: 50%B; flow: 1.4 mL/min; temperature: 25 °C; UV: 280 nm
Analytical chiral HPLC: Rₜ = 1.96 min
[α]²⁰_{D}: +115.9° (c = 1.00, methanol)
LC-MS (Method 1): Rt = 1.06 min; MS (ESlpos): m/z = 444 [M+H]⁺
¹H-NMR (400MHz, DMSO-d₆): δ [ppm]= 0.69 (t, 3H), 1.68 - 1.81 (m, 1H), 1.87 - 2.07 (m, 3H), 2.54 - 2.61 (m, 3H), 2.70 (d, 1H), 2.76 (td, 1H), 3.35 - 3.39 (m, 1H), 4.06 - 4.13 (m, 2H), 6.46 (s, 1H), 6.52 (s, 2H), 8.01 (d, 1H), 8.60 (d, 1H), 8.76 (s, 2H), 9.09 (s, 1H).

### Example 820

### 5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)

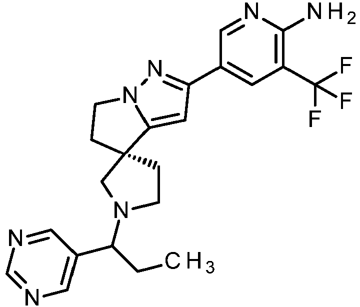

For the preparation of the diastereomeric mixture of the title compound see Example 818. The diastereomers were separated by preparative chiral HPLC (method see Example 819) to give 31.0 mg (100 % purity, 18 % yield) of the title compound (diastereomer 2 Rₜ = 18.4 - 20.5 min).
Analytical chiral HPLC (method see Example 819): Rₜ = 2.48 min.
[α]²⁰_{D}: +62.9° (c = 1.00, methanol)
LC-MS (Method 1): Rt = 1.06 min; MS (ESlpos): m/z = 444 [M+H]⁺
¹H-NMR (400MHz, DMSO-d₆): δ [ppm]= 0.68 (t, 3H), 1.67 - 1.79 (m, 1H), 1.88 - 2.07 (m, 3H), 2.54 - 2.62 (m, 4H), 2.78 - 2.85 (m, 1H), 3.35 - 3.40 (m, 1H), 4.02 - 4.14 (m, 2H), 6.45 (s, 1H), 6.52 (s, 2H), 8.01 (d, 1H), 8.59 (d, 1H), 8.77 (s, 2H), 9.11 (s, 1H).

### Example 821

### (3R)-2'-{6-amino-5-[(1R)-1-(1,3-thiazol-2-yl)ethoxy]pyridin-3-yl}-N-(propan-2-yl)-5',6'-dihydrospiro[pyrrolidine-3,4'-pyrrolo[1,2-b]pyrazole]-1-carboxamide

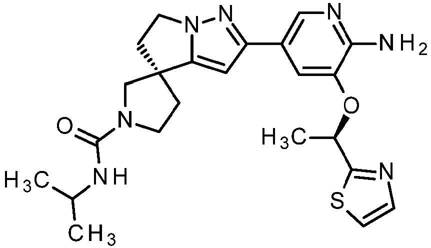

5-(4,4,5,5-Tetramethyl-1,3,2-dioxaborolan-2-yl)-3-[(1R)-1-(1,3-thiazol-2-yl)ethoxy]pyridin-2-amine (249 mg, 45 % purity, 323 µmol) was dissolved in 1,4-dioxane (1.8 mL) under argon. (3R)-1-[(Propan-2-yl)carbamoyl]-5',6'-dihydrospiro[pyrrolidine-3,4'-pyrrolo[1,2-b]pyrazol]-2'-yl trifluoromethanesulfonate (107 mg, 269 µmol), potassium phosphate (1.6 mL, 0.50 M, 810 µmol) and XPhos Pd G2 (21.2 mg, 26.9 µmol) were added and the mixture was stirred for 1 h at 100 °C. The cold reaction mixture was diluted with dichloromethane. The organic phase was washed with water and brine, dried over a hydrophobic filter and concentrated. The residue was purified by preparative HPLC to give 34.3 mg (97 % purity, 26 % yield) of the title compound.
LC-MS (Method 2): Rt = 0.73 min; MS (ESlpos): m/z = 468 [M+H]⁺
¹H-NMR (400 MHz, DMSO-d6) δ [ppm]: 1.049 (14.46), 1.058 (16.00), 1.065 (15.63), 1.074 (15.05), 1.703 (13.88), 1.719 (13.91), 2.005 (1.53), 2.019 (2.45), 2.035 (3.21), 2.052 (1.29), 2.064 (0.60), 2.336 (0.91), 2.518 (12.63), 2.523 (8.48), 2.678 (0.85), 3.351 (1.21), 3.370 (2.16), 3.382 (12.65), 3.395 (2.74), 3.413 (0.97), 3.459 (0.92), 3.478 (1.60), 3.485 (0.99), 3.492 (1.16), 3.504 (0.98), 3.518 (0.56), 3.566 (1.02), 3.710 (0.42), 3.727 (1.16), 3.743 (1.70), 3.762 (1.71), 3.779 (1.13), 3.795 (0.40), 4.124 (3.21), 4.142 (5.63), 4.159 (3.09), 5.802 (3.02), 5.822 (3.13), 5.845 (6.56), 5.863 (1.44), 5.879 (3.57), 5.895 (3.54), 5.911 (0.98), 6.280 (12.74), 7.407 (5.33), 7.411 (5.14), 7.711 (8.17), 7.719 (9.24), 7.791 (9.70), 7.799 (8.43), 7.932 (7.24), 7.936 (7.10).

### Example 822

### (3R)-2'-[6-amino-5-(trifluoromethyl)pyridin-3-yl]-N-[(3-methylpyridin-4-yl)methyl]-5',6'-dihydrospiro[pyrrolidine-3,4'-pyrrolo[1,2-b]pyrazole]-1-carboxamide

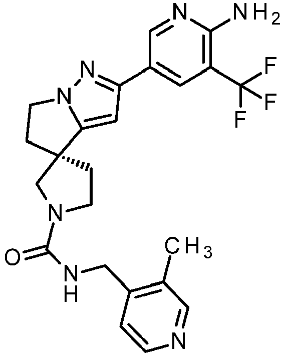

1-(3-Methylpyridin-4-yl)methanamine hydrogen chloride (1/2) (36.7 mg, 95 % purity, 179 µmol), CDI (31.6 mg, 195 µmol) and N,N-diisopropylethylamine (140 µL, 810 µmol) were dissolved in DMF (1 mL) and stirred for 1 h at rt. A solution of 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (75.0 mg, 78 % purity, 163 µmol) in DMF (1 mL) was added and the mixture was stirred over night at rt. The reaction mixture was purified by preparative HPLC to give 43.0 mg (95 % purity, 53 % yield) of the title compound.
LC-MS (Method 1): Rt = 0.87 min; MS (ESlpos): m/z = 473 [M+H]⁺
¹H-NMR (400 MHz, DMSO-d6) δ [ppm]: 0.937 (0.73), 0.953 (0.70), 2.093 (1.08), 2.111 (1.85), 2.120 (0.97), 2.128 (0.89), 2.255 (16.00), 2.518 (1.55), 2.523 (1.15), 2.565 (3.08), 2.582 (2.11), 3.457 (0.41), 3.475 (1.15), 3.489 (8.22), 3.499 (1.80), 3.518 (0.55), 3.566 (0.55), 3.584 (1.14), 3.592 (0.61), 3.599 (0.72), 3.610 (0.72), 4.181 (2.19), 4.200 (3.43), 4.217 (5.48), 4.232 (3.51), 6.494 (9.08), 6.551 (5.33), 6.801 (0.81), 6.816 (1.69), 6.831 (0.79), 7.190 (2.38), 7.202 (2.42), 8.024 (3.06), 8.029 (3.07), 8.293 (5.25), 8.321 (2.86), 8.333 (2.76), 8.597 (2.83), 8.602 (2.77).

### Example 823

### 5-{(3R)-1-[1-(6-methoxypyridin-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)

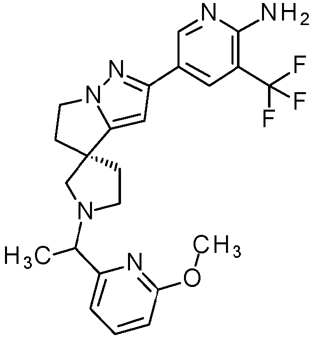

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (250 mg, 695 µmol) and 1-(6-methoxypyridin-2-yl)ethan-1-one (158 mg, 1.04 mmol) were dissolved in methanol (2.5 mL). N,N-Diisopropylethylamine (610 µL, 3.5 mmol), titanium(IV) isopropoxide (620 µL, 2.1 mmol) were added and stirred for 16 h at 60 °C. The reaction mixture was allowed to cool down, sodium cyanoborohydride (131 mg, 2.08 mmol) was added and the mixture was stirred for 2 h at 60 °C. The cold reaction mixture was allowed to cool down, diluted with water and stirred for 30 min. The suspension was diluted with methanol and filtered over celite. The filtrate was concentrated. The residue was diluted with dichloromethane / methanol (small amount of methanol) and washed with brine. The aqueous phase was extracted with dichloromethane / methanol. The combined organic layers were dried over sodium sulfate, filtered and concentrated. The crude product was purified by column chromatography (silica gel, dichloromethane / methanol (0 - 5 %) gradient) followed by preparative TLC (dichloromethane / methanol 9:1 gradient) and preparative HPLC to give 49 mg of the diastereomeric mixture which was separated separated into diastereomers by preparative chiral HPLC to give 8.00 mg (75 % purity, 2 % yield) of the title compound.
LC-MS (Method 1): Rt = 1.29 min; MS (ESlpos): m/z = 460 [M+H]⁺
¹H-NMR (400 MHz, CHLOROFORM-d) δ [ppm]: 1.447 (2.20), 1.463 (2.11), 2.089 (0.46), 2.159 (0.48), 2.168 (0.52), 2.179 (0.68), 2.580 (0.59), 2.596 (0.53), 2.600 (0.61), 2.675 (0.44), 2.689 (0.48), 2.732 (0.42), 2.753 (0.44), 2.795 (0.91), 3.920 (16.00), 3.937 (3.67), 4.132 (0.66), 4.147 (0.72), 4.151 (0.76), 4.168 (0.77), 4.170 (0.86), 4.178 (0.41), 4.184 (0.85), 4.191 (0.79), 4.197 (0.65), 4.204 (0.73), 4.211 (0.41), 5.014 (2.37), 6.153 (1.16), 6.611 (1.12), 6.631 (1.15), 6.982 (1.29), 7.000 (1.39), 7.548 (1.26), 7.568 (1.44), 7.587 (0.89), 8.107 (1.46), 8.112 (1.50), 8.606 (1.39), 8.610 (1.38).

### Example 824

### 5-{(3R)-1-[1-(6-methoxypyridin-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)

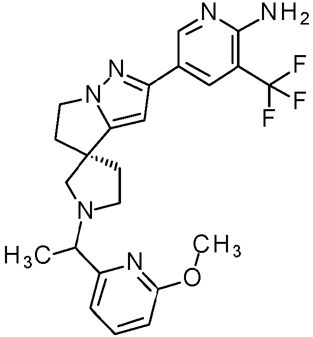

For the preparation of the diastereomeric mixture of the title compound see Example 823. The diastereomers were separated by preparative HPLC to give 4.9 mg (98 % purity, 2 % yield) of the title compound.
LC-MS (Method 1): Rt = 1.30 min; MS (ESlpos): m/z = 459 [M+H]⁺
¹H-NMR (400MHz, DMSO-d₆): δ [ppm]= 0.68 (t, 3H), 1.67 - 1.79 (m, 1H), 1.88 - 2.07 (m, 3H), 2.54 - 2.62 (m, 4H), 2.78 - 2.85 (m, 1H), 3.35 - 3.40 (m, 1H), 4.02 - 4.14 (m, 2H), 6.45 (s, 1H), 6.52 (s, 2H), 8.01 (d, 1H), 8.59 (d, 1H), 8.77 (s, 2H), 9.11 (s, 1H).

### Example 825

### (3R)-2'-(6-amino-5-{1-[3-(3-hydroxy-3-methylbut-1-yn-1-yl)phenyl]ethoxy}pyridin-3-yl)-N-ethyl-5',6'-dihydro-1H-spiro[pyrrolidine-3,4'-pyrrolo[1,2-b]pyrazole]-1-carboxamide (diastereomer 1 and diastereomer 2)

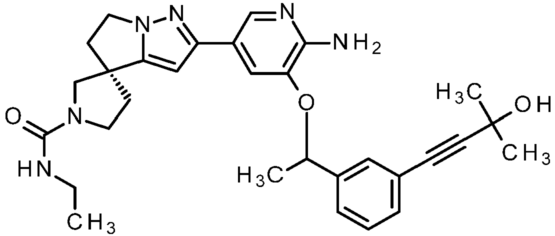

(3R)-1-(Ethylcarbamoyl)-5',6'-dihydrospiro[pyrrolidine-3,4'-pyrrolo[1,2-b]pyrazol]-2'-yl trifluoromethanesulfonate (121 mg, 317 µmol), 4-[3-(1-{[2-amino-5-(4,4,5,5-tetramethyl-1,3,2-dioxaborolan-2-yl)pyridin-3-yl]oxy}ethyl)phenyl]-2-methylbut-3-yn-2-ol (174 mg, 412 µmol), potassium phosphate (1.9 mL) and XPhos Pd G2 (12.5 mg, 15.8 µmol) were combined in degassed 1,4-dioxane (5.2 mL). The mixture was stirred for 80 min at 100 °C. The cold reaction mixture was diluted with dichloromethane and water. The phases were separated and the aqueous phase was extracted with dichloromethane / ethanol 9:1. The combined organic layers were concentrated. The residue was purified by flash column chromatography (silica gel, dichloromethane / methanol (0-10 %) gradient). The combined fractions were purified by preparative HPLC to give 33.0 mg (90 % purity, 18 % yield) of the title compound.
[α]²⁰_{D}: +43.3° (c = 1.00, methanol)
LC-MS (Method 1): Rₜ = 1.00 min; MS (ESlpos): m/z = 529 [M+H]⁺
¹H-NMR (400MHz, DMSO-d₆): δ [ppm]= 1.01 and 1.02 (2t, 3H), 1.44 (s, 6H), 1.52 - 1.59 (m, 3H), 1.95 - 2.09 (m, 2H), 3.00 - 3.09 (m, 2H), 3.35 - 3.42 (m, 3H), 3.43 - 3.52 (m, 1H), 4.12 (t, 2H), 5.47 (s, 1H), 5.52 - 5.64 (m, 1H), 5.79 - 5.92 (m, 2H), 6.14 (t, 1H), 6.20 - 6.25 (m, 1H), 7.20 - 7.57 (m, 5H), 7.84 - 7.86 (m, 1H).

### Example 826

### (3R)-2'-{6-amino-5-[(1R)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide

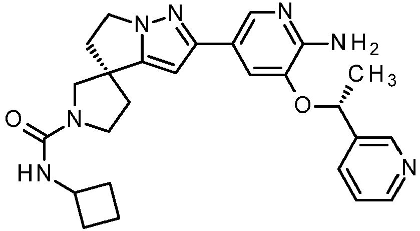

Cyclobutanamine (21.2 mg, 299 µmol) was dissolved in DMF (3.6 mL). CDI (44.4 mg, 274 µmol) and N,N-diisopropylethylamine (140 µL, 1000 µmol) was added and stirred for 1 h at rt. 5-[(3R)-5',6'-Dihydrospiro[pyrrolidine-3,4'-pyrrolo[1,2-b]pyrazol]-2'-yl]-3-[(1R)-1-(pyridin-3-yl)ethoxy]pyridin-2-amine hydrogen chloride (1/1) (150 mg, 69 % purity, 249 µmol) was added and the mixture was stirred for 1 h at 60 °C. The reaction mixture was allowed to cool down and purified by preparative HPLC to give 54.7 mg (100 % purity, 46 % yield) of the title compound.
LC-MS (Method 1): Rt = 0.85 min; MS (ESlpos): m/z = 474 [M+H]⁺
¹H-NMR (400MHz, DMSO-d₆): δ [ppm]= 1.47 - 1.62 (m, 5H), 1.85 - 1.97 (m, 2H), 1.98 - 2.14 (m, 4H), 3.36 - 3.43 (m, 3H), 3.44 - 3.52 (m, 1H), 4.05 - 4.17 (m, 3H), 5.71 (q, 1H), 5.89 (s, 2H), 6.28 (s, 1H), 6.31 (d, 1H), 7.33 (d, 1H), 7.37 (dd, 1H), 7.87 (d, 1H), 7.91 (dt, 1H), 8.47 (dd, 1H), 8.71 (d, 1H).

### Example 827

### (3R)-2'-{6-amino-5-[1-(1,3-oxazol-2-yl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)

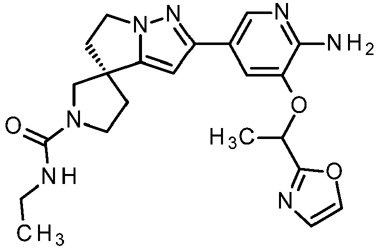

3-{[1-(1,3-Oxazol-2-yl)ethyl]oxy}-5-(4,4,5,5-tetramethyl-1,3,2-dioxaborolan-2-yl)pyridin-2-amine (178 mg, 48 % purity, 258 µmol) was dissolved in 1,4-dioxane under nitrogen. (3R)-1-(Ethylcarbamoyl)-5',6'-dihydrospiro[pyrrolidine-3,4'-pyrrolo[1,2-b]pyrazol]-2'-yl trifluoromethanesulfonate (82.2 mg, 215 µmol), potassium phosphate (1.3 mL, 0.50 M, 640 µmol) and XPhos Pd G2 (8.46 mg, 10.7 µmol) were added and the mixture was stirred for 1 h at 100 °C. The cold reaction mixture was diluted with dichloromethane. The organic phase was washed with water and brine, dried over a hydrophobic filter and concentrated. The residue was purified by preparative HPLC to give 19.5 mg (100 % purity, 21 % yield) of the title compound.
LC-MS (Method 2): Rₜ = 0.76 min; MS (ESlpos): m/z = 438 [M+H]⁺
¹H-NMR (400MHz, DMSO-d₆): δ [ppm]= 1.02 (t, 3H), 1.70 (d, 3H), 1.99 - 2.11 (m, 2H), 3.01 - 3.09 (m, 2H), 3.36 - 3.43 (m, 3H), 3.45 - 3.52 (m, 1H), 4.16 (t, 2H), 5.67 (q, 1H), 5.81 (br s, 2H), 6.15 (t, 1H), 6.28 (s, 1H), 7.22 - 7.24 (m, 1H), 7.46 (d, 1H), 7.94 (d, 1H), 8.13 (d, 1H).

### Example 828

### (3R)-2'-{6-amino-5-[(1R)-1-(3-fluorophenyl)ethoxy]pyridin-3-yl}-N-ethyl-5',6'-dihydrospiro[pyrrolidine-3,4'-pyrrolo[1,2-b]pyrazole]-1-carboxamide

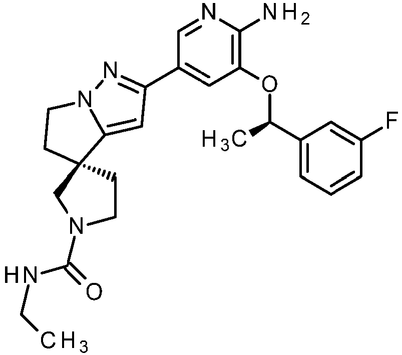

(3R)-1-(Ethylcarbamoyl)-5',6'-dihydrospiro[pyrrolidine-3,4'-pyrrolo[1,2-b]pyrazol]-2'-yl trifluoromethanesulfonate (101 mg, 95 % purity, 250 µmol), 3-[(1R)-1-(3-fluorophenyl)ethoxy]-5-(4,4,5,5-tetramethyl-1,3,2-dioxaborolan-2-yl)pyridin-2-amine (170 mg, 79 % purity, 375 µmol) and potassium phosphate (1.5 mL, 0.50 M, 750 µmol) were dissolved in 1,4-dioxane (15 mL) under argon. XPhos Pd G2 (19.7 mg, 25.0 µmol) was added and the mixture was stirred for 1 h at 100 °C. The cold reaction mixture was diluted with ethyl acetate and filtered over celite. The organic phase was washed with water and brine, dried over a hydrophobic filter and concentrated. The residue was purified by preparative HPLC to give 24.1 mg (100 % purity, 21 % yield) of the title compound.
[α]²⁰_{D}: +121.6° (c = 1.00, methanol)
LC-MS (Method 2): Rt = 0.85 min; MS (ESlpos): m/z = 465 [M+H]⁺
¹H NMR (DMSO-d₆, 400 MHz): δ (ppm) 7.86 (d, 1H), 7.30 - 7.43 (m, 4H), 7.05 - 7.12 (m, 1H), 6.29 (s, 2H), 6.15 (t, 1H), 5.70 (q, 1H), 4.14 (t, Hz, 2H), 3.43 - 3.51 (m, 1H), 3.36 - 3.42 (m, 3H), 3.00 - 3.10 (m, 2H), 1.97 - 2.09 (m, 2H), 1.57 (d, 3H), 1.01 (t, 3H).

### Example 829

### (3R)-2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (diastereomer 2)

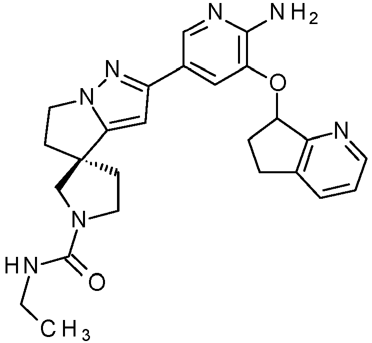

(3R)-1-(Ethylcarbamoyl)-5',6'-dihydrospiro[pyrrolidine-3,4'-pyrrolo[1,2-b]pyrazol]-2'-yl trifluoromethanesulfonate (126 mg, 329 µmol), {6-amino-5-[(6,7-dihydro-5H-cyclopenta[b]pyridin-7-yl)oxy]pyridin-3-yl}boronic acid (enantiomer 2) (223 mg, 40 % purity, 329 µmol) and potassium phosphate (2.0 mL, 0.50 M, 990 µmol) were dissolved in 1,4-dioxane (5.4 mL) under argon. XPhos Pd G2 (12.9 mg, 16.5 µmol) was added and the mixture was stirred for 2 h at 100 °C. The cold reaction mixture was diluted with ethyl acetate and washed with brine. The organic phase was dried over sodium sulfate, filtered and concentrated. The residue was purified by preparative HPLC followed by preparative TLC (dichloromethane / methanol 9:1 gradient) to give 22.2 mg (98 % purity, 14 % yield) of the title compound.
[α]²⁰_{D}: -13.7° (c = 1.00, methanol)
LC-MS (Method 1): Rt = 0.86 min; MS (ESlneg): m/z = 458 [M-H]^{- 1}H-NMR (400 MHz, CHLOROFORM-d) δ [ppm]: 0.065 (0.72), 1.148 (5.63), 1.166 (12.88), 1.184 (5.83), 1.812 (1.06), 2.018 (0.54), 2.068 (0.56), 2.072 (0.56), 2.086 (0.95), 2.099 (0.77), 2.103 (0.72), 2.118 (0.56), 2.219 (0.56), 2.238 (1.07), 2.250 (0.55), 2.256 (0.71), 2.269 (0.76), 2.281 (0.46), 2.287 (0.50), 2.292 (0.51), 2.294 (0.54), 2.303 (0.56), 2.305 (0.51), 2.316 (0.92), 2.327 (0.96), 2.338 (0.64), 2.340 (0.66), 2.349 (0.64), 2.351 (0.58), 2.362 (0.50), 2.540 (0.93), 2.556 (1.04), 2.573 (1.56), 2.590 (0.80), 2.615 (0.82), 2.628 (0.59), 2.633 (1.77), 2.641 (0.66), 2.649 (1.34), 2.659 (0.70), 2.663 (1.18), 2.666 (1.45), 2.676 (0.58), 2.680 (1.09), 2.684 (0.96), 2.694 (0.51), 2.698 (0.52), 2.701 (0.47), 2.715 (0.44), 2.898 (0.44), 2.912 (0.45), 2.920 (0.41), 2.940 (0.67), 2.953 (0.67), 2.961 (0.60), 2.974 (0.52), 3.122 (0.56), 3.135 (0.60), 3.143 (0.59), 3.157 (0.58), 3.176 (0.42), 3.185 (0.41), 3.277 (0.79), 3.291 (1.01), 3.295 (2.53), 3.309 (2.70), 3.313 (2.65), 3.327 (2.60), 3.331 (0.99), 3.345 (0.80), 3.500 (1.07), 3.524 (2.26), 3.532 (1.04), 3.539 (0.76), 3.557 (1.40), 3.564 (2.96), 3.575 (0.66), 3.588 (1.37), 3.619 (0.54), 3.633 (0.68), 3.639 (0.78), 3.653 (0.66), 3.663 (0.46), 4.173 (0.63), 4.187 (1.16), 4.200 (0.61), 4.250 (2.65), 4.268 (5.09), 4.285 (2.49), 4.986 (3.97), 5.297 (13.38), 5.658 (1.22), 5.669 (1.31), 5.676 (1.34), 5.687 (1.21), 6.207 (9.16), 7.214 (1.58), 7.226 (1.63), 7.233 (1.75), 7.245 (1.81), 7.260 (16.00), 7.628 (1.49), 7.631 (1.54), 7.648 (1.39), 7.650 (1.39), 7.693 (3.24), 7.697 (3.34), 8.093 (4.53), 8.098 (4.32), 8.518 (1.48), 8.520 (1.50), 8.522 (1.56), 8.531 (1.52), 8.534 (1.47).

### Example 830

### 5-{(3R)-1-[cyclopropyl(5-fluoropyridin-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

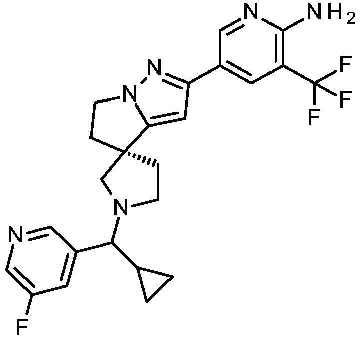

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (150 mg, 95 % purity, 396 µmol) and cyclopropyl(5-fluoropyridin-3-yl)methanone (98.1 mg, 594 µmol) were dissolved in methanol (4.2 mL). N,N-Diisopropylethylamine (280 µL, 1.6 mmol), titanium(IV) isopropoxide (350 µL, 1.2 mmol) were added and stirred for 1 h at 60 °C. Sodium cyanoborohydride (74.7 mg, 1.19 mmol) was added and the mixture was stirred for 30 h at 60 °C and for 20 h at 80 °C. The cooled down reaction mixture was diluted with water (0.5 mL) and stirred for 15 min. The suspension was filtered and the filter cake was washed with methanol. The filtrate was concentrated. The crude product was purified by preparative HPLC to give 81.0 mg (100 % purity, 43 % yield) of the title compound.
LC-MS (Method 1): Rt = 1.23 min; MS (ESlpos): m/z = 473 [M+H]⁺
¹H-NMR (400 MHz, DMSO-d6) δ [ppm]: 0.088 (0.65), 0.100 (0.99), 0.110 (1.13), 0.122 (0.91), 0.134 (0.66), 0.285 (0.92), 0.297 (1.15), 0.307 (0.97), 0.318 (0.69), 0.455 (0.63), 0.469 (0.84), 0.481 (0.92), 0.493 (0.90), 0.505 (0.75), 0.517 (0.56), 0.713 (1.04), 0.931 (1.32), 0.948 (1.32), 1.087 (0.74), 1.096 (0.90), 1.106 (0.88), 1.118 (0.81), 1.757 (0.73), 1.984 (0.44), 1.997 (0.77), 2.016 (1.18), 2.035 (1.51), 2.054 (1.36), 2.059 (1.37), 2.074 (0.87), 2.085 (0.43), 2.318 (0.91), 2.322 (1.96), 2.327 (2.78), 2.331 (2.05), 2.336 (0.97), 2.518 (16.00), 2.523 (9.98), 2.564 (2.56), 2.584 (2.71), 2.605 (2.58), 2.619 (2.53), 2.638 (1.79), 2.652 (0.84), 2.659 (1.29), 2.665 (2.20), 2.669 (3.19), 2.673 (2.21), 2.678 (1.00), 2.728 (1.43), 2.751 (1.09), 2.774 (0.47), 2.795 (0.94), 2.810 (0.82), 2.870 (1.85), 2.893 (1.73), 2.907 (0.57), 2.924 (0.53), 3.159 (0.40), 3.171 (0.42), 3.599 (0.60), 4.056 (0.62), 4.072 (1.20), 4.091 (2.22), 4.110 (2.69), 4.129 (1.32), 5.758 (0.84), 6.495 (5.64), 6.514 (9.83), 6.525 (5.05), 7.724 (1.40), 7.731 (1.77), 7.735 (1.57), 7.749 (1.44), 7.754 (1.67), 7.756 (1.68), 7.760 (1.51), 8.014 (1.89), 8.019 (3.97), 8.025 (2.63), 8.446 (7.60), 8.450 (5.14), 8.453 (5.36), 8.464 (2.59), 8.471 (2.41), 8.606 (3.68).

### Example 831

### (3R)-2'-{6-amino-5-[(1R)-1-(pyrimidin-2-yl)ethoxy]pyridin-3-yl}-N-(propan-2-yl)-5',6'-dihydrospiro[pyrrolidine-3,4'-pyrrolo[1,2-b]pyrazole]-1-carboxamide

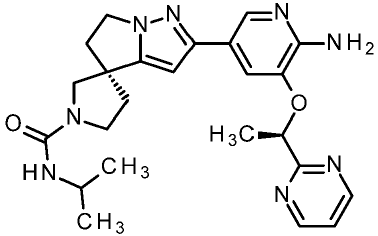

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (121 mg, 306 µmol), 3-[(1R)-1-(pyrimidin-2-yl)ethoxy]-5-(4,4,5,5-tetramethyl-1,3,2-dioxaborolan-2-yl)pyridin-2-amine (115 mg, 336 µmol), potassium phosphate (1.8 mL) and XPhos Pd G2 (12.0 mg, 15.3 µmol) were combined in degassed 1,4-dioxane (5.1 mL). The mixture was stirred for 80 min at 100 °C. The cold reaction mixture was diluted with dichloromethane and water. The phases were separated and the aqueous phase was extracted with dichloromethane / ethanol 9:1. The combined organic layers were dried and concentrated. The residue was purified by flash column chromatography (silica gel NH2, dichloromethane / methanol (0-1%) gradient) to give 83.0 mg (90 % purity, 53 % yield) of the title compound.
LC-MS (Method 1): Rₜ = 0.81 min; MS (ESlpos): m/z = 463 [M+H]⁺
¹H-NMR (400MHz, DMSO-d₆): δ [ppm]= 1.04 - 1.08 (m, 6H), 1.68 (d, 3H), 1.96 - 2.08 (m, 2H), 2.44 - 2.48 (m, 2H), 3.35 - 3.41 (m, 3H), 3.44 - 3.52 (m, 1H), 3.70 - 3.81 (m, 1H), 4.09 - 4.15 (m, 2H), 5.51 (q, 1H), 5.78 (s, 2H), 5.81 (d, 1H), 6.19 (s, 1H), 7.20 (d, 1H), 7.44 (t, 1H), 7.87 (d, 1H), 8.83 (d, 2H).

### Example 832

### (3R)-2'-{6-amino-5-[(1R)-1-(4-fluorophenyl)ethoxy]pyridin-3-yl}-N-ethyl-5',6'-dihydrospiro[pyrrolidine-3,4'-pyrrolo[1,2-b]pyrazole]-1-carboxamide

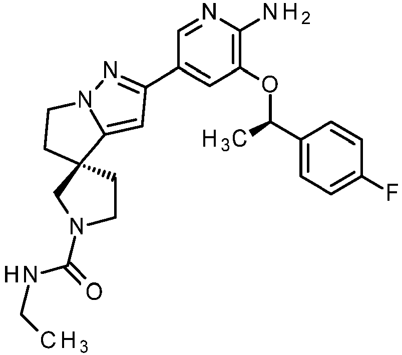

(3R)-1-(Ethylcarbamoyl)-5',6'-dihydrospiro[pyrrolidine-3,4'-pyrrolo[1,2-b]pyrazol]-2'-yl trifluoromethanesulfonate (99.5 mg, 95 % purity, 247 µmol), 3-[(1R)-1-(4-fluorophenyl)ethoxy]-5-(4,4,5,5-tetramethyl-1,3,2-dioxaborolan-2-yl)pyridin-2-amine (160 mg, 83 % purity, 371 µmol) and potassium phosphate (1.5 mL, 0.50 M, 740 µmol) were dissolved in 1,4-dioxane (15 mL) under argon. XPhos Pd G2 (19.4 mg, 24.7 µmol) was added and the mixture was stirred for 3 h at 100 °C. The reaction mixture was allowed to cool down and diluted with ethyl acetate (50 mL). It was filtered over celite and washed with ethyl acetate. The organic phase was washed with water and brine, dried over a hydrophobic filter and concentrated. The residue was purified by preparative HPLC to obtain 37.7 mg (100 % purity, 33 % yield) of the title compound.
[α]²⁰_{D}: +143.0° (c = 1.00, methanol)
LC-MS (Method 1): Rt = 0.98 min; MS (ESlpos): m/z = 466 [M+H]⁺
¹H-NMR (400 MHz, DMSO-d6) δ [ppm]: 0.998 (6.62), 1.007 (0.85), 1.016 (16.00), 1.024 (0.81), 1.033 (6.99), 1.550 (8.05), 1.566 (8.07), 2.009 (1.07), 2.016 (1.09), 2.023 (1.55), 2.034 (2.13), 2.052 (0.96), 2.472 (1.87), 2.518 (2.27), 2.522 (1.51), 3.015 (0.77), 3.032 (2.48), 3.046 (2.69), 3.050 (2.69), 3.065 (2.43), 3.083 (0.73), 3.373 (6.79), 3.377 (6.27), 3.394 (2.14), 3.403 (1.08), 3.411 (0.90), 3.449 (0.72), 3.466 (1.19), 3.473 (0.72), 3.481 (0.84), 3.492 (0.70), 3.506 (0.40), 4.114 (2.11), 4.131 (3.66), 4.149 (2.16), 5.617 (0.46), 5.633 (1.65), 5.649 (1.66), 5.664 (0.46), 6.022 (1.04), 6.135 (0.99), 6.149 (1.95), 6.163 (0.97), 6.180 (0.43), 6.263 (9.07), 7.148 (3.02), 7.153 (0.98), 7.165 (1.22), 7.170 (6.01), 7.176 (1.18), 7.187 (1.02), 7.193 (3.27), 7.293 (3.32), 7.298 (3.37), 7.512 (2.90), 7.517 (1.22), 7.526 (3.12), 7.533 (2.96), 7.542 (1.06), 7.547 (2.64), 7.849 (4.55), 7.853 (4.48), 8.133 (0.56).

### Example 833

### (3R)-2'-{6-amino-5-[(1R)-1-(4-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide

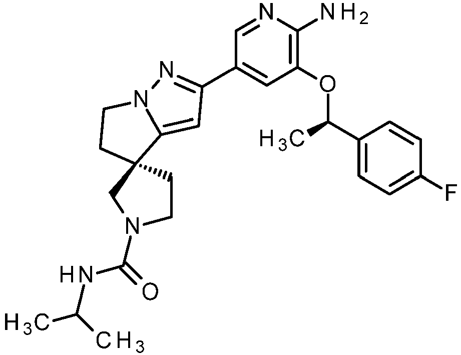

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (119 mg, 79 % purity, 237 µmol), 3-[(1R)-1-(4-fluorophenyl)ethoxy]-5-(4,4,5,5-tetramethyl-1,3,2-dioxaborolan-2-yl)pyridin-2-amine (157 mg, 81 % purity, 355 µmol) and potassium phosphate (1.4 mL, 0.50 M, 710 µmol) were dissolved in 1,4-dioxane (15 mL) under argon. XPhos Pd G2 (18.6 mg, 23.7 µmol) was added and the mixture was stirred for 2 h at 100 °C. The reaction mixture was allowed to cool down and diluted with ethyl acetate (50 mL). It was filtered over celite and washed with ethyl acetate. The organic phase was washed with water and brine, dried over a hydrophobic filter and concentrated. The residue was purified by preparative HPLC affording 60.0 mg (100 % purity, 53 % yield) of the title compound.
[α]²⁰_{D}: +137.1° (c = 1.00, methanol)
LC-MS (Method 1): Rt = 1.07 min; MS (ESlpos): m/z = 479 [M+H]⁺
¹H NMR (DMSO-d₆, 400 MHz): δ (ppm) 7.85 (d, 1H), 7.50 - 7.55 (m, 2H), 7.26 (d, 1H), 7.13 - 7.20 (m, 2H), 6.25 (s, 1H), 5.85 (s, 2H), 5.81 (d, 1H), 5.61 (q, 1H), 4.13 (t, 2H), 3.71 - 3.80 (m, 1H), 3.45 - 3.53 (m, 1H), 3.36 - 3.42 (m, 3H), 1.96 - 2.08 (m, 2H), 1.55 (d, 3H), 1.07 (d, 3H), 1.06 (d, 3H).

### Example 834

### 5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

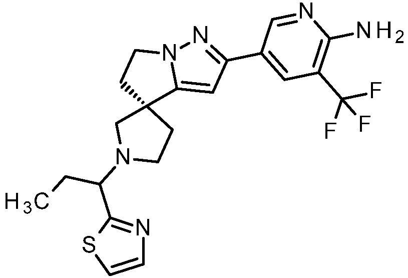

5-[(3R)-5',6'-Dihydrospiro[pyrrolidine-3,4'-pyrrolo[1,2-b]pyrazol]-2'-yl]-3-(trifluoromethyl)pyridin-2-amine (200 mg, 619 µmol) and 1-(1,3-thiazol-2-yl)propan-1-one (87.3 mg, 619 µmol, CAS 43039-98-1) were dissolved in methanol (2.1 mL). N,N-Diisopropylethylamine (430 µL, 2.5 mmol) and titanium(IV) isopropoxide (550 µL, 1.9 mmol) were added and the mixture was stirred for 16 h at 60 °C. The suspension was allowed to cool down, sodium cyanoborohydride (117 mg, 1.86 mmol) was added and stirred for 2 h at 60 °C. 1-(1,3-Thiazol-2-yl)propan-1-one (43.6 mg, 309 µmol) and sodium cyanoborohydride (58 mg, 0.93 mmol) were added again and the mixture was stirred for 1 h at 60 °C. The reaction mixture was allowed to cool down, diluted with water (1 mL) and stirred for 30 min. The suspension was filtered over celite and the filter cake was washed with methanol. The filtrate was concentrated. The residue was dissolved in dichloromethane / methanol (small amount of methanol) and twice with a saturated sodium bicarbonate solution. The aqueous phase was extracted with dichloromethane / methanol. The combined organic layers were dried over a hydrophobic filter and concentrated. The residue was purified twice by flash column chromatography (silica gel, dichloromehane / methanol (0-5%) gradient) to give 93.0 mg (100 % purity, 34 % yield) of the title compound.
LC-MS (Method 1): Rt = 1.22 min; MS (ESlpos): m/z = 449 [M+H]⁺
¹H-NMR (400 MHz, DMSO-d6) δ [ppm]: 0.787 (7.38), 0.806 (16.00), 0.824 (7.98), 0.851 (0.94), 1.173 (0.52), 1.233 (4.40), 1.254 (2.18), 1.720 (0.96), 1.738 (1.62), 1.755 (1.89), 1.772 (1.97), 1.791 (1.18), 1.931 (1.14), 1.947 (2.22), 1.973 (4.54), 1.979 (4.92), 1.990 (6.26), 2.008 (3.32), 2.048 (0.42), 2.327 (3.71), 2.522 (11.27), 2.560 (1.86), 2.575 (1.32), 2.592 (0.56), 2.669 (4.01), 2.674 (3.84), 2.699 (3.54), 2.711 (2.86), 2.734 (4.46), 2.754 (4.83), 2.776 (4.02), 2.798 (0.97), 2.814 (3.85), 2.837 (3.54), 2.843 (1.95), 2.858 (1.44), 2.881 (0.54), 2.918 (0.72), 2.935 (1.38), 2.957 (1.24), 2.974 (0.53), 3.835 (1.67), 3.849 (2.34), 3.857 (3.31), 3.866 (2.13), 3.879 (1.41), 4.068 (1.53), 4.077 (1.93), 4.086 (5.49), 4.103 (7.40), 4.120 (3.18), 5.760 (2.04), 6.368 (8.87), 6.399 (10.28), 6.521 (14.32), 7.673 (4.54), 7.681 (6.01), 7.689 (3.75), 7.697 (5.07), 7.735 (7.33), 7.742 (5.63), 7.998 (6.68), 8.004 (4.39), 8.573 (6.00).

### Example 835

### 5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)

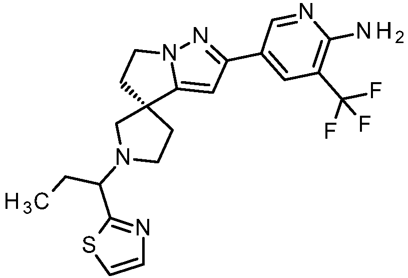

The title compound from **Example 834** was separated into diastereomers by preparative chiral HPLC to give 28.0 mg (99 % purity) of the title compound (diastereomer 1 Rt = 6.4 - 7.2 min).

### Preparative chiral HPLC method:

Instrument: PrepCon Labomatic HPLC-3; Column: YMC Amylose SB 5µ, 250x50; eluent A: hexane + 0.1 vol % diethylamine; eluent B: ethanol; isocratic: 20%B; flow: 50 mL/min; temperature: 25 °C; UV: 280 nm

### Analytical chiral HPLC method:

Instrument: Thermo Fisher UltiMate 3000; Column: YMC Amylose SB 3µ, 100x4.6; eluent A: hexane + 0.1 vol % diethylamine; eluent B: ethanol; isocratic: 20%B; flow: 1.4 mL/min; temperature: 25 °C; UV: 280 nm
Analytical chiral HPLC: Rₜ = 2.41 min
LC-MS (Method 1): Rt = 1.22 min; MS (ESlpos): m/z = 449 [M+H]⁺
¹H-NMR (400 MHz, CHLOROFORM-d) δ [ppm]: 0.772 (0.68), 0.783 (0.81), 0.798 (7.26), 0.816 (16.00), 0.835 (7.36), 1.185 (6.58), 1.215 (1.20), 1.231 (1.07), 1.746 (0.76), 1.764 (0.95), 1.767 (0.90), 1.779 (1.20), 1.786 (0.97), 1.798 (1.17), 1.801 (1.21), 1.820 (0.96), 1.920 (0.82), 1.941 (1.50), 1.946 (1.41), 1.952 (1.65), 1.959 (1.86), 1.969 (1.90), 1.973 (1.90), 1.978 (1.61), 1.989 (1.52), 1.998 (0.94), 2.012 (0.68), 2.043 (1.23), 2.056 (1.49), 2.065 (1.54), 2.077 (1.92), 2.088 (0.98), 2.096 (0.90), 2.109 (0.83), 2.438 (0.81), 2.454 (0.97), 2.459 (1.05), 2.471 (1.59), 2.486 (1.62), 2.491 (1.62), 2.506 (1.35), 2.558 (1.27), 2.573 (1.58), 2.578 (1.53), 2.592 (1.80), 2.706 (2.06), 2.729 (4.44), 2.740 (1.03), 2.762 (5.88), 2.784 (3.20), 2.801 (0.92), 2.922 (0.93), 2.934 (1.09), 2.944 (1.57), 2.956 (1.49), 2.965 (0.81), 2.978 (0.67), 3.795 (1.57), 3.807 (1.76), 3.816 (1.68), 3.830 (1.49), 4.033 (0.75), 4.048 (0.92), 4.053 (0.98), 4.060 (1.99), 4.068 (0.97), 4.075 (2.17), 4.080 (2.32), 4.092 (2.30), 4.095 (2.21), 4.106 (2.28), 4.113 (2.12), 4.120 (0.98), 4.127 (1.93), 4.133 (0.93), 4.140 (0.88), 4.154 (0.71), 4.917 (5.55), 5.233 (0.67), 6.077 (11.66), 7.252 (4.61), 7.260 (4.78), 7.658 (6.77), 7.666 (6.41), 8.029 (4.07), 8.033 (4.06), 8.521 (3.87), 8.525 (3.76).

### Example 836

### 5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)

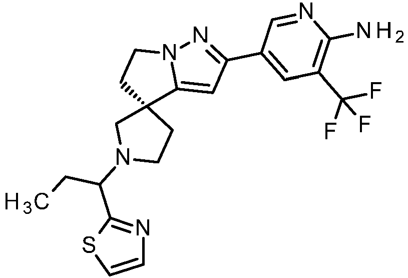

For the preparation of the diastereomeric mixture of the title compound see **Example 834** The diastereomers were separated by preparative chiral HPLC (method see **Example 835)** to give 26.0 mg (99 % purity) of the title compound (diastereomer 2 Rₜ = 7.8 - 8.9 min).
Analytical chiral HPLC (method see **Example 835):** Rₜ = 2.99 min.
LC-MS (Method 1): Rt = 1.22 min; MS (ESlpos): m/z = 449 [M+H]⁺
¹H-NMR (400 MHz, CHLOROFORM-d) δ [ppm]: 0.798 (6.93), 0.817 (16.00), 0.835 (7.38), 1.185 (2.68), 1.231 (0.64), 1.330 (0.48), 1.755 (0.77), 1.773 (0.92), 1.776 (0.90), 1.788 (1.18), 1.794 (0.95), 1.807 (1.16), 1.810 (1.27), 1.829 (1.03), 1.889 (0.78), 1.903 (0.93), 1.910 (1.01), 1.922 (1.82), 1.935 (1.84), 1.938 (1.64), 1.942 (1.67), 1.957 (2.12), 1.971 (1.41), 1.986 (0.89), 1.991 (0.88), 2.004 (0.70), 2.060 (1.22), 2.076 (1.50), 2.081 (1.60), 2.092 (1.10), 2.096 (1.53), 2.107 (1.13), 2.113 (1.08), 2.129 (0.97), 2.430 (0.84), 2.445 (0.98), 2.450 (1.12), 2.463 (1.72), 2.478 (1.61), 2.483 (1.75), 2.498 (1.40), 2.554 (1.39), 2.570 (1.79), 2.574 (1.62), 2.589 (1.79), 2.602 (1.15), 2.726 (0.88), 2.744 (2.54), 2.767 (6.54), 2.784 (6.65), 2.806 (1.75), 2.892 (1.04), 2.906 (1.16), 2.914 (1.77), 2.928 (1.67), 2.936 (0.97), 2.950 (0.75), 3.786 (1.67), 3.800 (1.86), 3.808 (1.79), 3.821 (1.63), 4.053 (0.55), 4.068 (0.71), 4.073 (0.61), 4.080 (2.29), 4.088 (0.79), 4.095 (4.29), 4.100 (2.92), 4.111 (2.91), 4.116 (4.08), 4.123 (0.79), 4.131 (2.19), 4.139 (0.61), 4.143 (0.68), 4.158 (0.52), 4.909 (5.42), 5.233 (0.46), 6.123 (14.12), 6.929 (0.50), 7.235 (4.60), 7.243 (4.79), 7.451 (0.50), 7.653 (7.28), 7.661 (6.95), 8.038 (3.90), 8.042 (3.95), 8.534 (3.79), 8.537 (3.72).

### Example 837

### (3R)-2'-{6-amino-5-[(1R)-1-(4-chlorophenyl)ethoxy]pyridin-3-yl}-N-ethyl-5',6'-dihydrospiro[pyrrolidine-3,4'-pyrrolo[1,2-b]pyrazole]-1-carboxamide

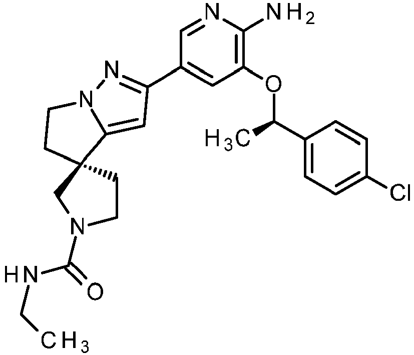

(3R)-1-(Ethylcarbamoyl)-5',6'-dihydrospiro[pyrrolidine-3,4'-pyrrolo[1,2-b]pyrazol]-2'-yl trifluoromethanesulfonate (66.4 mg, 95 % purity, 165 µmol), 3-[(1R)-1-(4-chlorophenyl)ethoxy]-5-(4,4,5,5-tetramethyl-1,3,2-dioxaborolan-2-yl)pyridin-2-amine (122 mg, 76 % purity, 247 µmol) and potassium phosphate (990 µL, 0.50 M, 740 µmol) were dissolved in 1,4-dioxane (10 mL) under argon. XPhos Pd G2 (13.0 mg, 16.5 µmol) was added and the mixture was stirred for 4 h at 100 °C. The reaction mixture was allowed to cool down and diluted with ethyl acetate (50 mL). It was filtered over celite and washed with ethyl acetate. The organic phase was washed with water and brine, dried over a hydrophobic filter and concentrated. The residue was purified by preparative HPLC to obtain 6.8 mg (95 % purity, 8 % yield) of the title compound.
[α]²⁰_{D}: +189.0° (c = 1.00, methanol)
LC-MS (Method 1): Rt = 1.04 min; MS (ESlpos): m/z = 481 [M+H]⁺
¹H NMR (DMSO-d₆, 400 MHz): δ (ppm) 7.86 (d, 1H), 7.48 - 7.52 (m, 2H), 7.38 - 7.42 (m, 2H), 7.24 (d, 1H), 6.25 (s, 1H), 6.15 (t, 1H), 5.86 (s, 2H), 5.62 (q, 1H), 4.13 (t, 2H), 3.44 - 3.52 (m, 1H), 3.36 - 3.42 (m, 3H), 3.00 - 3.09 (m, 2H), 1.96 - 2.07 (m, 2H), 1.55 (d, 3H), 1.01 (t, 3H).

### Example 838

### 5-{(3R)-1-[1-(dimethylamino)propan-2-yl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

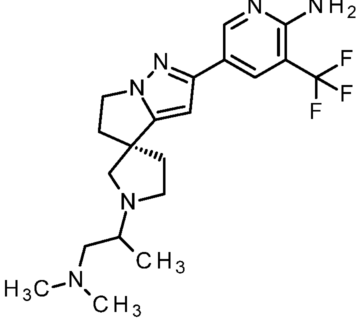

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (250 mg, 79 % purity, 549 µmol) and 2-chloro-N,N-dimethylpropan-1-amine hydrogen chloride (1/1) (104 mg, 659 µmol) were dissolved in THF (6 mL). Potassium carbonate (303 mg, 2.20 mmol) and sodium iodide (85 mg, 549 µmol) were added and the mixture was stirred for 48 h at 70 °C. The reaction mixture was allowed to cool down, diluted with water and extracted with ethyl acetate. The combined organic layers were washed with brine, dried over sodium sulfate, filtered and concentrated. The residue was purified by preparative HPLC to give 41.0 mg (95 % purity, 17 % yield) of the title compound.
[α]²⁰_{D}: +36.8° (c = 1.00, DMSO)
LC-MS (Method 1): Rt = 1.15 min; MS (ESlpos): m/z = 410 [M+H]⁺
¹H NMR (DMSO-d₆, 400 MHz): δ (ppm) 8.58 (d, 1H), 8.01 (d, 1H), 6.52 (s, 2H), 6.42 and 6.43 (2s, 1H), 4.10 (t, 2H), 2.53 - 2.85 (m, 6H), 2.36 - 2.48 (m, 3H), 2.16 (2s, 6H), 1.93 - 2.08 (m, 2H), 0.94 and 0.95 (2d, 3H).

### Example 839

### (3R)-2'-{6-amino-5-[(1R)-1-(1,2-thiazol-5-yl)ethoxy]pyridin-3-yl}-N-ethyl-5',6'-dihydrospiro[pyrrolidine-3,4'-pyrrolo[1,2-b]pyrazole]-1-carboxamide

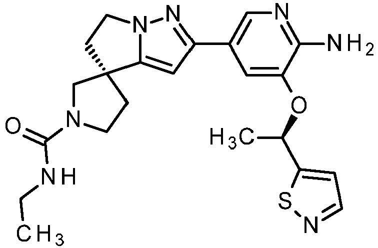

5-(4,4,5,5-Tetramethyl-1,3,2-dioxaborolan-2-yl)-3-[(1R)-1-(1,2-thiazol-5-yl)ethoxy]pyridin-2-amine (150 mg, 62 % purity, 268 µmol) was dissolved in 1,4-dioxane (1.5 mL) under argon. (3R)-1-(Ethylcarbamoyl)-5',6'-dihydrospiro[pyrrolidine-3,4'-pyrrolo[1,2-b]pyrazol]-2'-yl trifluoromethanesulfonate (85.3 mg, 223 µmol), potassium phosphate (1.3 mL, 0.50 M, 670 µmol) and XPhos Pd G2 (8.78 mg, 11.2 µmol) were added and the mixture was stirred for 1 h at 100 °C. The cold reaction mixture was diluted with dichloromethane and washed with water and brine. The organic phase was dried over a hydrophobic filter and concentrated. The crude product was purified by preparative HPLC to give 18.7 mg (98 % purity, 18 % yield) of the title compound.
[α]²⁰_{D}: +101.0° (c = 1.00, methanol)
LC-MS (Method 1): Rt = 0.81 min; MS (ESlpos): m/z = 454 [M+H]⁺
¹H NMR (DMSO-d₆, 400 MHz): δ (ppm) 8.49 (d, 1H), 7.94 (d, 1H), 7.46 (d, 1H), 7.43 (d, 1H), 6.30 (s, 1H), 6.08 - 6.17 (m, 2H), 5.84 (s, 2H), 4.14 (t, 2H), 3.44 - 3.51 (m, 1H), 3.35 - 3.42 (m, 3H), 3.01 - 3.09 (m, 2H), 1.98 - 2.10 (m, 2H), 1.69 (d, 3H), 1.02 (t, 3H).

### Example 840

### 6-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyridin-2(1H)-one

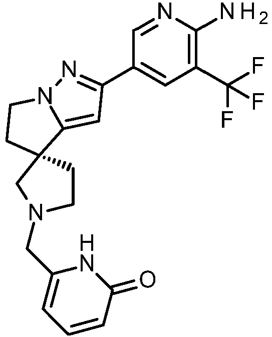

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (60.0 mg, 167 µmol), 6-oxo-1,6-dihydropyridine-2-carbaldehyde (20.5 mg, 167 µmol), sodium triacetoxyborohydride (177 mg, 834 µmol) and trifluoroacetic acid (58 µL, 750 µmol) were dissolved in DMF (2.8 mL) and stirred for 12 h at 50 °C. The reaction mixture was poured into a sodium bicarbonate solution and extracted with ethyl acetate. The combined organic layers were dried over sodium sulfate, filtered and concentrated. The residue was purified by preparative TLC (dichloromethane / methanol 9:1 gradient) to give 16.1 mg (95 % purity, 21 % yield) of the title compound.
LC-MS (Method 1): Rt = 0.93 min; MS (ESlpos): m/z = 431 [M+H]⁺
¹H-NMR (400 MHz, CHLOROFORM-d) δ [ppm]: 2.083 (0.65), 2.102 (1.02), 2.116 (1.18), 2.120 (0.99), 2.133 (1.48), 2.153 (1.08), 2.243 (1.09), 2.256 (1.32), 2.264 (1.44), 2.277 (1.86), 2.289 (1.00), 2.297 (0.96), 2.310 (0.85), 2.599 (0.66), 2.631 (1.43), 2.647 (1.54), 2.651 (1.64), 2.667 (1.34), 2.696 (1.42), 2.709 (2.55), 2.715 (1.77), 2.729 (3.58), 2.741 (1.21), 2.748 (2.46), 2.752 (1.44), 2.761 (0.92), 2.769 (1.02), 2.782 (3.22), 2.805 (4.77), 2.887 (4.29), 2.893 (2.69), 2.911 (2.85), 2.940 (1.00), 2.952 (1.18), 2.960 (1.41), 2.967 (3.16), 2.972 (1.33), 2.983 (0.93), 2.995 (0.75), 3.539 (0.46), 3.608 (10.27), 4.153 (0.69), 4.169 (0.84), 4.173 (0.85), 4.181 (1.93), 4.189 (0.84), 4.197 (2.17), 4.200 (2.36), 4.210 (1.94), 4.216 (1.96), 4.223 (2.22), 4.230 (2.02), 4.237 (0.92), 4.243 (1.86), 4.251 (0.82), 4.257 (0.80), 4.271 (0.65), 5.014 (5.18), 5.310 (5.59), 6.036 (2.75), 6.038 (2.72), 6.052 (2.87), 6.055 (2.85), 6.241 (16.00), 6.458 (2.89), 6.480 (3.04), 7.006 (0.62), 7.324 (3.83), 7.340 (3.59), 7.347 (3.40), 7.364 (3.39), 7.528 (0.65), 8.117 (3.59), 8.121 (3.58), 8.622 (3.47), 8.626 (3.34), 9.630 (0.69).

### Example 841

### (3R)-2'-{6-amino-5-[(1R)-1-(pyridin-3-yl)ethoxy]pyridin-3-yl}-N-(propan-2-yl)-5',6'-dihydrospiro[pyrrolidine-3,4'-pyrrolo[1,2-b]pyrazole]-1-carboxamide

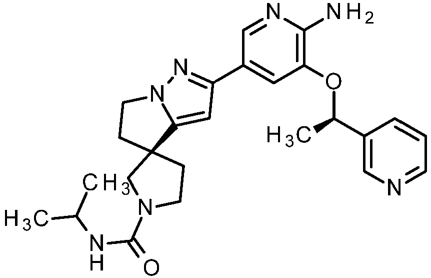

5-[(3R)-5',6'-Dihydrospiro[pyrrolidine-3,4'-pyrrolo[1,2-b]pyrazol]-2'-yl]-3-[(1R)-1-(pyridin-3-yl)ethoxy]pyridin-2-amine hydrogen chloride (1/1) (36.0 mg, 87.2 µmol) was suspended in dichloromethane (270 µL). N,N-Diisopropylethylamine (150 µL, 870 µmol) was added and the mixture was cooled to 0 °C and 2-isocyanatopropane (10 µL, 100 µmol) was added. The mixture was allowed to warm up to rt and stirred over night. The reaction mixture was concentrated. The residue was purified by preparative TLC (dichloromethane / methanol 9:1 gradient) to give 7.70 mg (96 % purity, 18 % yield) of the title compound.
LC-MS (Method 1): Rt = 0.85 min; MS (ESlpos): m/z = 462 [M+H]⁺
¹H-NMR (400 MHz, CHLOROFORM-d) δ [ppm]: 1.087 (0.67), 1.093 (3.16), 1.103 (3.91), 1.109 (3.97), 1.118 (3.06), 1.184 (2.24), 1.633 (4.40), 1.649 (4.45), 1.990 (0.44), 2.142 (0.52), 2.464 (0.44), 2.482 (0.57), 2.539 (0.63), 2.558 (0.47), 2.572 (0.42), 3.415 (1.20), 3.436 (1.60), 3.457 (0.68), 3.460 (0.66), 3.914 (1.00), 3.926 (0.66), 3.941 (0.43), 4.141 (1.10), 4.159 (1.91), 4.177 (1.02), 4.706 (1.80), 5.233 (0.66), 5.452 (0.72), 5.468 (0.72), 6.013 (4.14), 7.194 (16.00), 7.206 (1.94), 7.210 (1.48), 7.217 (0.67), 7.218 (0.66), 7.224 (0.63), 7.226 (0.63), 7.236 (0.61), 7.238 (0.62), 7.629 (0.41), 7.634 (0.70), 7.639 (0.44), 7.654 (0.65), 7.922 (2.00), 7.927 (1.98), 8.466 (0.97), 8.470 (1.01), 8.478 (0.99), 8.482 (0.95), 8.603 (1.10), 8.608 (1.09).

### Example 842

### 2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (4 isomers)

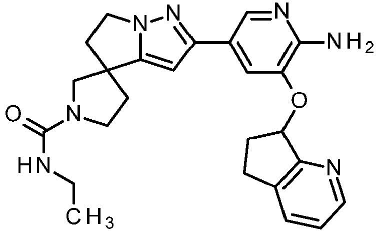

3-[(6,7-Dihydro-5H-cyclopenta[b]pyridin-7-yl)oxy]-5-[5',6'-dihydrospiro(pyrrolidine-3,4'-pyrrolo[1,2-b]pyrazol)-2'-yl]pyridin-2-amine dihydrogen chloride (1/2) (89.0 mg, 193 µmol) was suspended in dichloromethane (3.1 mL). N,N-Diisopropylethylamine (200 µL, 1.2 mmol) and isocyanatoethane (16 µL, 200 µmol) were added and stirred at rt. The reaction mixture was diluted with water and stirred for 5 min. The layers were separated and the aqueous phase was extracted with dichloromethane / methanol 9:1. The combined organic layers were concentrated. The residue was purified by flash column chromatography (silica gel, dichloromethane / methanol (0-5%) gradient) to give 52.0 mg (90 % purity, 53 % yield) of the title compound.
LC-MS (Method 1): Rt = 0.85 min; MS (ESlpos): m/z = 460 [M+H]⁺
¹H NMR (DMSO-d₆, 400 MHz): δ (ppm) 8.43 - 8.47 (m, 1H), 7.97 (d, 1H), 7.78 (dd, 1H), 7.75 (d, 1H), 7.32 (dd, 1H), 6.35 (s, 1H), 6.16 (t, 1H), 5.69 - 5.77 (m, 3H), 4.18 (t, 2H), 3.46 - 3.54 (m, 1H), 3.36 - 3.44 (m, 3H), 3.01 - 3.18 (m, 3H), 2.91 (ddd, 1H), 2.55 - 2.63 (m, 1H), 2.20 (qd, 1H), 2.00 - 2.13 (m, 2H), 1.02 (t, 3H).

### Example 843

### (3R)-2'-{6-amino-5-[1-(1,3-oxazol-4-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 1 and diastereomer 2)

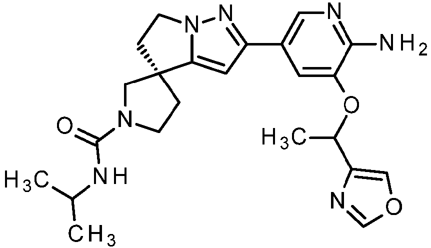

3-{[1-(1,3-Oxazol-4-yl)ethyl]oxy}-5-(4,4,5,5-tetramethyl-1,3,2-dioxaborolan-2-yl)pyridin-2-amine (275 mg, 36 % purity, 296 µmol) was dissolved in 1,4-dioxane (1.7 mL) under argon. (3R)-1-[(Propan-2-yl)carbamoyl]-5',6'-dihydrospiro[pyrrolidine-3,4'-pyrrolo[1,2-b]pyrazol]-2'-yl trifluoromethanesulfonate (97.9 mg, 247 µmol), potassium phosphate (1.5 mL, 0.50 M, 740 µmol) and XPhos Pd G2 (19.4 mg, 24.7 µmol) were added and the mixture was stirred for 1 h at 100 °C. The cold reaction mixture was diluted with dichloromethane, washed with water and brine. The organic phase was dried over a hydrophobic filter and concentrated. The residue was purified by preparative HPLC to give 4.00 mg (93 % purity, 3 % yield) of the title compound.
LC-MS (Method 1): Rt = 0.82 min; MS (ESlpos): m/z = 452 [M+H]⁺
1H-NMR (400MHz, DMSO-d6): δ [ppm]= 1.06 (d, 3H), 1.07 (d, 3H), 1.59 (d, 3H), 1.98 - 2.10 (m, 2H), 3.36 - 3.43 (m, 3H), 3.46 - 3.54 (m, 1H), 3.71 - 3.80 (m, 1H), 4.16 (t, 2H), 5.49 (q, 1H), 5.75 (s, 2H), 5.82 (d, 1H), 6.33 (s, 1H), 7.44 (d, 1H), 7.94 (d, 1H), 8.17 (d, 1H), 8.38 (s, 1H).

### Example 844

### 5-{(3R)-1-[(1,3-thiazol-2-yl)methyl]-5',6'-dihydrospiro[pyrrolidine-3,4'-pyrrolo[1,2-b]pyrazol]-2'-yl}-3-(trifluoromethyl)pyridin-2-amine

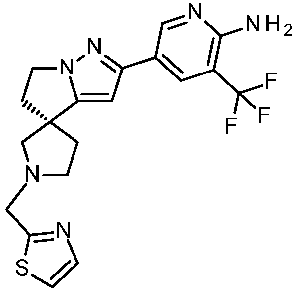

5-[(3R)-5',6'-Dihydrospiro[pyrrolidine-3,4'-pyrrolo[1,2-b]pyrazol]-2'-yl]-3-(trifluoromethyl)pyridin-2-amine (100 mg, 309 µmol) and 1,3-thiazole-2-carbaldehyde (35.0 mg, 309 µmol, CAS 10200-59-6) were dissolved in methanol (1.1 mL). N,N-Diisopropylethylamine (220 µL, 1.2 mmol) and titanium(IV) isopropoxide (270 µL, 930 µmol) were added and the mixture was stirred for 16 h at 60 °C. To the cold reaction mixture was added sodium cyanoborohydride (58.3 mg, 928 µmol) and stirred for 2 h at 60 °C. The reaction mixture was allowed to cool down, diluted with water (1 mL) and stirred for 30 min. The suspension was diluted with methanol, filtered over celite and the filter cake was washed with methanol. The filtrate was concentrated. The residue was dissolved in dichloromethane / methanol (small amount of methanol) and washed twice with a saturated sodium bicarbonate solution. The aqueous phase was extracted with dichloromethane / methanol. The combined organic layers were dried over a hydrophobic filter and concentrated. The residue was purified by flash column chromatography (silica gel, dichloromehane / methanol (0-4%) gradient) to give 65.0 mg (97 % purity, 48 % yield) of the title compound.
LC-MS (Method 1): Rt = 1.09 min; MS (ESlpos): m/z = 421 [M+H]⁺
¹H-NMR (400 MHz, DMSO-d6) δ [ppm]: 1.238 (1.41), 1.253 (1.06), 1.269 (0.59), 1.988 (0.46), 2.039 (0.48), 2.055 (1.50), 2.071 (2.75), 2.084 (3.51), 2.089 (2.90), 2.104 (1.67), 2.116 (0.46), 2.121 (0.50), 2.318 (0.49), 2.323 (1.06), 2.327 (1.55), 2.332 (1.13), 2.336 (0.47), 2.518 (5.84), 2.523 (4.12), 2.562 (2.03), 2.578 (1.32), 2.592 (1.29), 2.608 (1.98), 2.611 (1.75), 2.626 (1.80), 2.641 (1.06), 2.660 (1.00), 2.665 (1.19), 2.669 (1.60), 2.673 (1.15), 2.765 (3.21), 2.788 (4.83), 2.818 (0.68), 2.841 (1.63), 2.855 (6.12), 2.878 (4.02), 2.896 (1.14), 2.911 (1.57), 2.916 (1.62), 2.931 (1.35), 2.954 (0.60), 4.002 (0.66), 4.039 (10.88), 4.043 (11.08), 4.081 (0.69), 4.090 (0.48), 4.100 (2.21), 4.105 (2.07), 4.118 (3.85), 4.124 (2.66), 4.135 (2.07), 4.140 (2.14), 4.152 (0.42), 5.759 (14.68), 6.482 (16.00), 6.525 (7.87), 7.656 (8.72), 7.664 (11.85), 7.711 (11.15), 7.719 (8.33), 8.013 (4.44), 8.018 (4.45), 8.589 (4.12), 8.594 (3.99).

### Example 845

### 6-({(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}methyl)pyridin-2(1H)-one

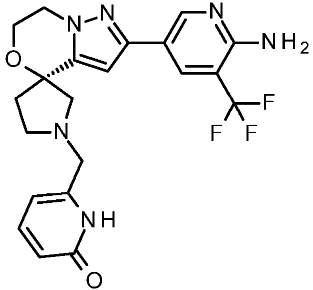

5-[(3'R)-6,7-dihydrospiro[pyrazolo[5,1-c][1,4]oxazine-4,3'-pyrrolidin]-2-yl]-3-(trifluoromethyl)pyridin-2-amine hydrogen chloride (1/1) (50.0 mg, 133 µmol), 6-oxo-1,6-dihydropyridine-2-carbaldehyde (16.4 mg, 133 µmol, CAS 358751-77-6), sodium triacetoxyborohydride (141 mg, 665 µmol) and trifluoroacetic acid (46 µL, 600 µmol) were dissolved in DMF (2.2 mL) and stirred for 12 h at 50 °C. The reaction mixture was poured into a sodium bicarbonate solution and extracted with ethyl acetate. The combined organic layers were dried over sodium sulfate, filtered and concentrated. The residue was purified by preparative TLC (dichloromethane / methanol 9:1) to give 15.4 mg (98 % purity, 25 % yield) of the title compound.
LC-MS (Method 1): Rt = 0.89 min; MS (ESlpos): m/z = 448 [M+H]⁺
¹H-NMR (400 MHz, CHLOROFORM-d) δ [ppm]: 1.261 (0.44), 2.226 (0.78), 2.246 (1.72), 2.260 (1.43), 2.265 (1.17), 2.279 (2.61), 2.299 (1.32), 2.377 (0.95), 2.389 (1.24), 2.394 (1.33), 2.408 (1.31), 2.423 (0.82), 2.428 (0.82), 2.441 (0.68), 2.841 (0.80), 2.854 (0.96), 2.864 (1.66), 2.877 (1.76), 2.884 (1.39), 2.897 (1.16), 2.926 (1.28), 2.944 (2.73), 2.963 (1.45), 2.967 (2.03), 2.985 (0.68), 3.041 (2.65), 3.067 (7.36), 3.091 (5.29), 3.118 (1.74), 3.646 (9.23), 4.070 (0.57), 4.075 (0.56), 4.085 (0.90), 4.089 (1.58), 4.101 (2.18), 4.104 (2.93), 4.108 (1.87), 4.114 (2.32), 4.121 (4.19), 4.133 (2.59), 4.151 (0.63), 4.157 (0.85), 4.161 (1.07), 4.166 (0.43), 4.186 (3.37), 4.191 (2.83), 4.198 (4.44), 4.202 (3.95), 4.210 (1.86), 4.216 (1.82), 5.073 (6.00), 5.310 (12.49), 6.043 (3.07), 6.045 (3.17), 6.060 (3.23), 6.062 (3.14), 6.307 (16.00), 6.455 (3.35), 6.476 (3.54), 7.006 (0.52), 7.321 (4.17), 7.338 (4.14), 7.344 (4.01), 7.361 (3.60), 7.528 (0.55), 8.104 (4.16), 8.108 (4.17), 8.626 (4.13), 8.629 (4.02), 9.838 (0.78).

### Example 846

### (3S)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1)

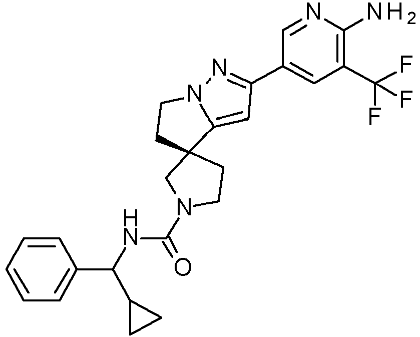

1-Cyclopropyl-1-phenylmethanamine (147 mg, 1 mmol) was dissolved in DMF (15 mL). CDI (162 mg, 1 mmol) and N,N-diisopropylethylamine (581 µL, 3.3 mmol) were added and stirred for 1 h at rt. 5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (300 mg, 834 µmol) was added and the mixture was stirred for 2 h at rt. The reaction mixture was concentrated and diluted with water. The aqueous phase was extracted with dichloromethane. The combined organic layers were concentrated. The residue was purified by column chromatography (silica gel, ethyl acetate / methanol (0 - 10 %) gradient) to give 342 mg of the title compound as a mixture of diastereomer.

The mixture was separated into diastereomers by preparative chiral HPLC to give 132 mg (95 % purity, 40 % yield) of the title compound (diastereomer 1 Rₜ = 8.4 - 11.0 min).

### Preparative chiral HPLC method:

Instrument: PrepCon Labomatic HPLC-2; Column: YMC Amylose SA 10µ, 250x50; eluent A: metyl tert-butyl ether + 0.1 vol % diethylamine; eluent B: acetonitrile + 0.1 vol % diethylamine; isocratic: 50%B; flow: 100 mL/min; temperature: 25 °C; UV: 254 nm

### Analytical chiral HPLC method:

Instrument: Thermo Fisher UltiMate 3000; Column: YMC Cellulose SA 3µ, 100x4.6; eluent A: metyl tert-butyl ether + 0.1 vol % diethylamine; eluent B: acetonitrile; isocratic: 50%B; flow: 1.4 mL/min; temperature: 25 °C; UV: 254 nm
Analytical chiral HPLC: Rₜ = 2.38 min
[α]²⁰_{D}: -87.7° (c = 1.00, DMSO)
LC-MS (Method 1): Rt = 1.12 min; MS (ESlpos): m/z = 497 [M+H]⁺
¹H NMR (DMSO-d₆, 400 MHz): δ (ppm) 8.57 (d, 1H), 8.00 (d, 1H), 7.39 - 7.44 (m, 2H), 7.30 (t, 2H), 7.17 - 7.23 (m, 1H), 6.65 (d, 1H), 6.55 (s, 2H), 6.44 (s, 1H), 4.19 (t, 2H), 4.06 (t, 1H), 3.54 - 3.62 (m, 1H), 3.40 - 3.50 (m, 3H), 2.52 - 2.57 (m, 2H), 2.02 - 2.13 (m, 2H), 1.14 - 1.25 (m, 1H), 0.42 - 0.52 (m, 2H), 0.27 - 0.39 (m, 2H).

### Example 847

### (3S)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 2)

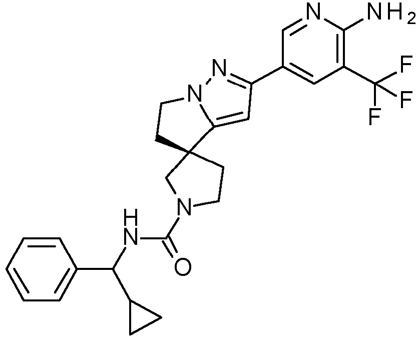

For the preparation of the diastereomeric mixture of the title compound see **Example 846.** The diastereomers were separated by preparative chiral HPLC (method see **Example 846)** to give 135 mg (95 % purity, 41 % yield) of the title compound (diastereomer 2 Rₜ = 14.4 - 19.2 min).
Analytical chiral HPLC (method see **Example 846):** Rt = 4.25 min.
[α]²⁰_{D}: -26.7° (c = 1.00, DMSO)
¹H NMR (DMSO-d₆, 400 MHz): δ (ppm) 8.58 (d, 1H), 8.00 (d, 1H), 7.39 (d, 2H), 7.27 (t, 2H), 7.15 - 7.21 (m, 1H), 6.65 (d, 1H), 6.55 (s, 2H), 6.43 (s, 1H), 4.19 (t, 2H), 4.06 (s, 1H), 3.52 - 3.59 (m, 1H), 3.41 - 3.51 (m, 3H), 2.52 - 2.57 (m, 2H), 2.01 - 2.12 (m, 2H), 1.15 - 1.26 (m, 1H), 0.42 - 0.54 (m, 2H), 0.28 - 0.40 (m, 2H).
LC-MS (Method 1): Rt = 1.12 min; MS (ESlpos): m/z = 497 [M+H]⁺

### Example 848

### (3R)-2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (diastereomer 1)

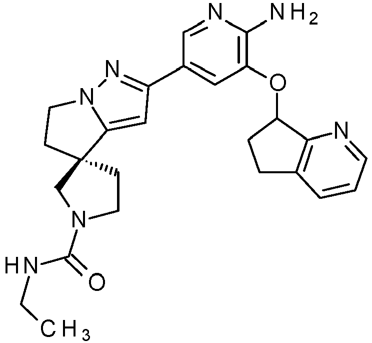

(3R)-1-(Ethylcarbamoyl)-5',6'-dihydrospiro[pyrrolidine-3,4'-pyrrolo[1,2-b]pyrazol]-2'-yl trifluoromethanesulfonate (120 mg, 313 µmol), {6-amino-5-[(6,7-dihydro-5H-cyclopenta[b]pyridin-7-yl)oxy]pyridin-3-yl}boronic acid (enantiomer 1) (212 mg, 40 % purity, 313 µmol) and potassium phosphate (1.9 mL, 0.50 M, 940 µmol) were dissolved in degassed 1,4-dioxane (5.1 mL) under argon. XPhos Pd G2 (12.3 mg, 15.6 µmol) was added and the mixture was stirred for 2 h at 100 °C. The reaction mixture was allowed to cool down, diluted with ethyl acetate and filtered. The filtrate was washed with water and brine. The organc phase was dried over sodium sulfate, filtered and concentrated. The residue was purified by preparative HPLC followed by preparative TLC (dichloromethane / methanol 9:1 gradient) to give 22.2 mg (98 % purity, 15 % yield) of the title compound.
[α]²⁰_{D}: +140.2° (c = 1.00, methanol)
LC-MS (Method 1): Rt = 0.86 min; MS (ESlpos): m/z = 461 [M+H]⁺
¹H-NMR (400 MHz, CHLOROFORM-d) δ [ppm]: 1.149 (5.84), 1.168 (13.30), 1.185 (6.05), 1.839 (1.44), 2.018 (0.96), 2.055 (0.43), 2.069 (0.65), 2.086 (1.01), 2.100 (0.84), 2.119 (0.60), 2.219 (0.60), 2.238 (1.16), 2.250 (0.60), 2.256 (0.76), 2.269 (0.82), 2.283 (0.54), 2.287 (0.53), 2.296 (0.53), 2.305 (0.59), 2.318 (0.99), 2.329 (1.02), 2.342 (0.69), 2.350 (0.66), 2.353 (0.61), 2.364 (0.51), 2.523 (0.42), 2.540 (0.96), 2.556 (1.12), 2.573 (1.56), 2.590 (0.84), 2.614 (0.88), 2.631 (1.80), 2.641 (0.71), 2.649 (1.65), 2.659 (0.77), 2.663 (1.64), 2.676 (0.62), 2.680 (1.28), 2.694 (0.52), 2.697 (0.53), 2.701 (0.50), 2.715 (0.45), 2.898 (0.49), 2.912 (0.48), 2.919 (0.43), 2.939 (0.72), 2.952 (0.70), 2.960 (0.64), 2.974 (0.55), 3.122 (0.61), 3.136 (0.65), 3.144 (0.63), 3.158 (0.63), 3.176 (0.44), 3.185 (0.43), 3.279 (0.85), 3.293 (1.10), 3.297 (2.63), 3.311 (2.91), 3.315 (2.75), 3.328 (2.62), 3.333 (1.03), 3.347 (0.84), 3.484 (1.34), 3.504 (1.19), 3.528 (3.19), 3.553 (1.33), 3.568 (3.32), 3.593 (1.45), 3.616 (0.58), 3.630 (0.71), 3.635 (0.83), 3.649 (0.71), 3.660 (0.49), 4.176 (0.69), 4.190 (1.24), 4.202 (0.65), 4.249 (2.77), 4.267 (5.08), 4.285 (2.59), 4.995 (4.14), 5.297 (12.20), 5.660 (1.31), 5.671 (1.40), 5.677 (1.42), 5.688 (1.27), 6.207 (9.43), 7.214 (1.70), 7.226 (1.77), 7.233 (1.89), 7.245 (1.92), 7.260 (16.00), 7.628 (1.63), 7.632 (1.64), 7.647 (1.51), 7.651 (1.48), 7.698 (3.44), 7.703 (3.47), 8.085 (4.45), 8.090 (4.39), 8.518 (1.63), 8.520 (1.62), 8.522 (1.65), 8.530 (1.64), 8.532 (1.58), 8.534 (1.56).

### Example 849

### 2'-{6-amino-5-[(1R)-1-(pyridin-2-yl)ethoxy]pyridin-3-yl}-N-ethyl-5',6'-dihydrospiro[azetidine-3,4'-pyrrolo[1,2-b]pyrazole]-1-carboxamide

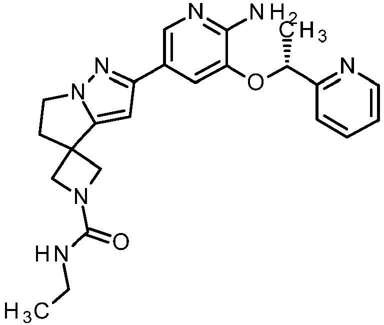

3-[(1R)-1-(Pyridin-2-yl)ethoxy]-5-(4,4,5,5-tetramethyl-1,3,2-dioxaborolan-2-yl)pyridin-2-amine (109 mg, 319 µmol), 1-(ethylcarbamoyl)-5',6'-dihydrospiro[azetidine-3,4'-pyrrolo[1,2-b]pyrazol]-2'-yl trifluoromethanesulfonate (129 mg, 351 µmol) and potassium phosphate (1.9 mL, 0.50 M, 960 µmol) were dissolved in degassed 1,4-dioxane. XPhos Pd G2 (12.6 mg, 16.0 µmol) was added and the mixture was stirred for 2 h at 100 °C. The reaction mixture was allowed to cool down, diluted with water and extracted with ethyl acetate. The combined organic layers were washed with brine, dried over sodium sulfate, filtered and concentrated. The residue was purified by preparative HPLC to give 34.0 mg (95 % purity, 23 % yield) of the title comound.
LC-MS (Method 1): Rt = 0.80 min; MS (ESlpos): m/z = 435 [M+H]⁺
¹H NMR (DMSO-d₆, 400 MHz): δ (ppm) 8.53 - 8.56 (m, 1H), 7.90 (d, 1H), 7.79 (td, 1H), 7.51 (d, 1H), 7.29 (ddd, 1H), 7.24 (d, 1H), 6.48 (s, 1H), 6.44 (t, 1H), 5.89 (s, 2H), 5.53 (q, 1H), 4.03 - 4.10 (m, 2H), 3.91 - 3.98 (m, 4H), 2.98 - 3.06 (m, 2H), 2.79 (t, 2H), 1.62 (d, 3H), 1.01 (t, 3H).

### Example 850

### 1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-(methylamino)ethan-1-one

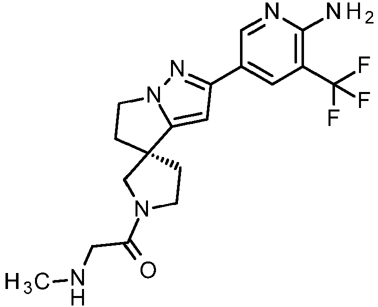

Tert-butyl (2-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-oxoethyl)methylcarbamate (43.5 mg, 88.0 µmol, **Intermediate 550)** was dissolved in dichloromethane. Hydrochloric acid in 1,4-dioxane (220 µL, 4.0 M, 880 µmol) was added and the mixture was stirred for 1 h at room temperature. The reaction mixture was diluted with water, basified with aqueous sodium hydroxide (30%) and extracted with dichloromethane. The combined organic layers were dried over a hydrophobic filter and concentrated to give 34.0 mg (99 % purity, 97 % yield) of the title compound.
LC-MS (Method 1): Rt = 0.82 min; MS (ESlpos): m/z = 395 [M+H]⁺
¹H-NMR (400 MHz, DMSO-d6) δ [ppm]: 1.844 (0.49), 2.024 (0.53), 2.040 (0.82), 2.052 (1.08), 2.069 (0.48), 2.110 (0.85), 2.127 (1.83), 2.144 (0.90), 2.264 (10.89), 2.307 (11.71), 2.322 (0.52), 2.327 (0.60), 2.332 (0.44), 2.522 (2.11), 2.565 (2.36), 2.580 (1.13), 2.596 (0.40), 2.665 (0.41), 2.669 (0.56), 2.673 (0.42), 3.143 (0.44), 3.183 (1.79), 3.205 (1.80), 3.245 (0.52), 3.250 (0.70), 3.291 (1.74), 3.330 (16.00), 3.368 (0.66), 3.484 (0.52), 3.509 (3.03), 3.540 (1.00), 3.588 (0.67), 3.606 (1.87), 3.632 (1.23), 3.658 (0.80), 3.675 (0.49), 3.684 (0.45), 4.166 (0.77), 4.176 (1.73), 4.183 (1.67), 4.194 (3.02), 4.211 (1.42), 6.504 (4.09), 6.521 (4.46), 6.542 (5.22), 8.008 (1.57), 8.013 (1.74), 8.019 (1.60), 8.025 (1.52), 8.591 (2.81), 8.595 (2.77).

### Example 851

### (2R)-1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-(methylamino)propan-1-one

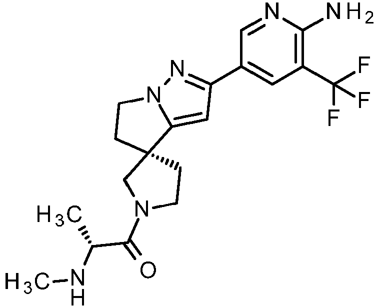

Tert-butyl [(2R)-1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-1-oxopropan-2-yl]methylcarbamate (29.3 mg, 57.6 µmol, **Intermediate 551)** was dissolved in dichloromethane. Hydrochloric acid in 1,4-dioxane (140 µL, 4.0 M, 580 µmol) was added and the mixture was stirred for 1 h at room temperature. The reaction mixture was diluted with water, basified with aqeous sodium hydroxide (30%) and extracted with dichloromethane. The combined organic layers were dried over a hydrophobic filter and concentrated to give 23.0 mg (95 % purity, 93 % yield) of the title compound.
LC-MS (Method 1): Rt = 0.84 min; MS (ESlpos): m/z = 409 [M+H]⁺
¹H-NMR (400 MHz, DMSO-d6) δ [ppm]: 1.056 (1.25), 1.072 (1.30), 1.119 (1.15), 1.135 (1.19), 2.133 (0.71), 2.140 (0.75), 2.157 (0.47), 2.176 (0.73), 2.189 (0.53), 2.518 (1.80), 2.523 (1.26), 2.560 (0.43), 3.479 (0.44), 3.566 (16.00), 3.578 (0.41), 3.647 (0.49), 3.714 (0.51), 4.186 (0.54), 4.199 (0.41), 4.205 (0.62), 6.483 (1.31), 6.489 (1.49), 6.545 (1.32), 8.005 (0.77), 8.011 (0.78), 8.575 (0.41), 8.582 (0.49).

### Example 852

### (5R)-5-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyrrolidin-2-one

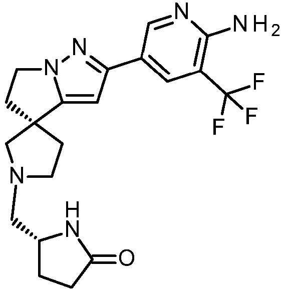

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (250 mg, 695 µmol), (5R)-5-(bromomethyl)pyrrolidin-2-one (136 mg, 764 µmol, CAS 98612-60-3) and cesium carbonate (249 mg, 764 µmol) were suspended in DMF (10 mL). The reaction mixture was stirred over night at room temperature, for 1 h at 50 °C and for 1 h at 80 °C. The suspension was diluted with ethyl acetate and filtered. The organic phase was washed with water and brine, dried and concentrated. The residue was purified by column chromatography (silica gel, dichloromethane / methanol (0 - 9 %) gradient) to give 57.0 mg (99 % purity, 19 % yield) of the title compound.
LC-MS (Method 1): Rt = 0.92 min; MS (ESlpos): m/z = 421 [M+H]⁺
¹H-NMR (400 MHz, DMSO-d6) δ [ppm]: 0.852 (0.48), 1.154 (1.22), 1.172 (2.41), 1.190 (1.16), 1.232 (1.56), 1.712 (1.52), 1.722 (1.14), 1.736 (1.39), 1.749 (0.75), 1.773 (0.43), 1.968 (0.66), 1.980 (1.54), 1.988 (5.26), 1.998 (3.07), 2.014 (2.64), 2.033 (1.56), 2.051 (0.55), 2.069 (1.13), 2.085 (1.80), 2.102 (1.21), 2.112 (6.78), 2.121 (2.01), 2.130 (3.07), 2.135 (2.62), 2.139 (3.17), 2.155 (0.61), 2.164 (1.72), 2.188 (0.75), 2.318 (1.16), 2.323 (2.49), 2.327 (3.48), 2.331 (2.47), 2.336 (1.12), 2.397 (1.58), 2.413 (1.73), 2.427 (2.51), 2.443 (2.66), 2.518 (14.67), 2.523 (10.09), 2.561 (1.85), 2.574 (1.31), 2.590 (1.99), 2.608 (1.86), 2.621 (4.37), 2.643 (5.48), 2.665 (3.96), 2.669 (4.51), 2.673 (2.76), 2.678 (1.41), 2.684 (0.93), 2.765 (3.90), 2.787 (3.34), 2.806 (1.40), 2.812 (1.63), 2.828 (1.52), 2.848 (0.64), 3.620 (1.20), 3.634 (1.75), 3.650 (1.24), 4.018 (0.95), 4.035 (0.96), 4.091 (2.40), 4.110 (4.57), 4.129 (2.33), 5.760 (6.66), 6.487 (16.00), 6.521 (8.00), 7.634 (4.28), 8.007 (4.55), 8.012 (4.55), 8.585 (4.21), 8.590 (4.16).

### Example 853

### 5-{(3R)-1-[(1,3,4-thiadiazol-2-yl)methyl]-5',6'-dihydrospiro[pyrrolidine-3,4'-pyrrolo[1,2-b]pyrazol]-2'-yl}-3-(trifluoromethyl)pyridin-2-amine

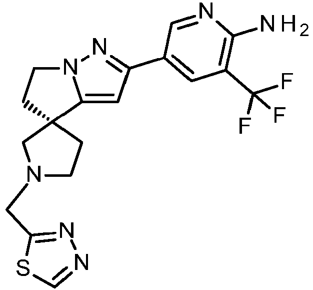

5-[(3R)-5',6'-Dihydrospiro[pyrrolidine-3,4'-pyrrolo[1,2-b]pyrazol]-2'-yl]-3-(trifluoromethyl)pyridin-2-amine (100 mg, 309 µmol) and 1,3,4-thiadiazole-2-carbaldehyde (35.3 mg, 309 µmol, CAS 144876-24-4) were dissolved in methanol (1.1 mL). N,N-Diisopropylethylamine (220 µL, 1.2 mmol), titanium(IV) isopropoxide (270 µL, 930 µmol) were added and stirred for 16 h at 60 °C. To the cold reaction mixture was added sodium cyanoborohydride (58.3 mg, 928 µmol) and the mixture was stirred for 2 h at 60 °C. The reaction mixture was allowed to cool down, diluted with water (1 mL) and stirred for 30 min. The suspension was diluted with methanol and filtered over celite. The filtrate was concentrated. The residue was diluted with dichloromethane / methanol (small amount of methanol) and washed with a saturated sodium bicarbonate solution. The aqueous phase was extracted with dichloromethane / methanol. The combined organic layers were dried over a hydrophobic filter and concentrated. The crude product was purified by column chromatography (silica gel, dichloromethane / methanol (0 - 4 %) gradient). The combined fractions were washed with a saturated sodium bicarbonate solution. The organic phase was dried over a hydrophobic filter and concentrated to give 18.0 mg (100 % purity, 14 % yield) of the title compound.
LC-MS (Method 1): Rt = 0.97 min; MS (ESlpos): m/z = 422 [M+H]⁺
¹H-NMR (400 MHz, DMSO-d6) δ [ppm]: 0.852 (0.49), 1.232 (2.39), 2.039 (0.40), 2.052 (1.40), 2.071 (2.25), 2.080 (2.12), 2.084 (2.24), 2.099 (1.56), 2.115 (0.41), 2.331 (1.73), 2.336 (0.76), 2.518 (9.61), 2.523 (6.77), 2.570 (1.65), 2.573 (1.63), 2.589 (2.04), 2.607 (1.70), 2.622 (0.95), 2.639 (0.58), 2.673 (1.75), 2.678 (0.81), 2.754 (2.95), 2.776 (4.69), 2.820 (0.66), 2.832 (5.04), 2.842 (1.69), 2.855 (3.79), 2.872 (1.40), 2.878 (1.34), 2.888 (1.79), 2.891 (1.64), 2.907 (1.30), 2.930 (0.48), 4.096 (2.10), 4.101 (1.95), 4.115 (3.90), 4.131 (1.99), 4.135 (2.05), 4.156 (0.65), 4.193 (10.25), 4.196 (10.48), 4.234 (0.57), 5.760 (7.06), 6.475 (16.00), 6.528 (7.29), 8.013 (4.08), 8.018 (4.16), 8.591 (3.77), 8.595 (3.73), 9.566 (15.56).

### Example 854

### (5S)-5-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyrrolidin-2-one

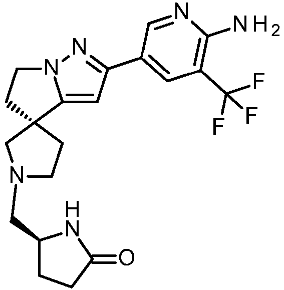

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (250 mg, 695 µmol), (5S)-5-(bromomethyl)pyrrolidin-2-one (136 mg, 764 µmol, CAS 72479-05-1) and cesium carbonate (249 mg, 764 µmol) were suspended in DMF (10 mL). The reaction mixture was stirred over night at room temperature, for 1 h at 50 °C and for 1 h at 80 °C. The suspension was diluted with ethyl acetate and filtered. The organic phase was washed with water and brine, dried and concentrated. The residue was purified by column chromatography (silica gel, dichloromethane / methanol (0 - 9 %) gradient) to give 26.0 mg (98 % purity, 9 % yield) of the title compound.
LC-MS (Method 1): Rt = 0.92 min; MS (ESlpos): m/z = 421 [M+H]⁺
¹H-NMR (400 MHz, DMSO-d6) δ [ppm]: 0.852 (0.56), 1.154 (0.64), 1.172 (1.31), 1.190 (0.67), 1.232 (1.89), 1.705 (1.33), 1.722 (1.01), 1.735 (1.07), 1.759 (0.42), 1.944 (0.45), 1.964 (0.79), 1.975 (1.38), 1.988 (3.24), 1.992 (1.80), 2.010 (1.93), 2.024 (1.64), 2.030 (1.61), 2.044 (1.54), 2.061 (0.99), 2.067 (1.31), 2.081 (2.59), 2.111 (3.92), 2.122 (1.69), 2.130 (2.31), 2.137 (1.81), 2.144 (2.39), 2.169 (2.08), 2.193 (0.86), 2.323 (2.23), 2.327 (3.08), 2.331 (2.21), 2.373 (1.53), 2.388 (1.69), 2.402 (2.22), 2.418 (2.19), 2.449 (1.45), 2.518 (16.00), 2.523 (9.42), 2.565 (1.72), 2.584 (3.17), 2.606 (3.79), 2.619 (1.17), 2.632 (1.47), 2.638 (1.41), 2.651 (1.85), 2.665 (3.94), 2.669 (4.20), 2.673 (2.62), 2.685 (2.16), 2.703 (1.68), 2.723 (0.87), 2.758 (3.98), 2.780 (3.40), 2.808 (0.56), 3.619 (1.02), 3.633 (1.61), 3.649 (1.21), 4.018 (0.50), 4.035 (0.47), 4.053 (0.54), 4.080 (1.69), 4.096 (3.10), 4.108 (2.30), 4.114 (2.98), 4.127 (1.70), 4.140 (0.54), 5.760 (8.46), 6.494 (15.33), 6.521 (8.03), 7.654 (4.26), 8.006 (4.62), 8.012 (4.61), 8.589 (4.35), 8.592 (4.25).

### Example 855

### 5-{(3'R)-1'-[1-(6-methoxypyridin-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

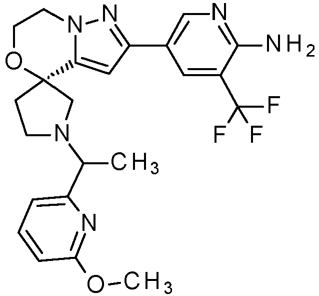

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (250 mg, 665 µmol) and 1-(6-methoxypyridin-2-yl)ethan-1-one (151 mg, 998 µmol, CAS 21190-93-2) were dissolved in methanol (2.5 mL). N,N-Diisopropylethylamine (580 µL, 3.3 mmol), titanium(IV) isopropoxide (590 µL, 2.0 mmol) were added and stirred for 16 h at 60 °C. To the cold reaction mixture was added sodium cyanoborohydride (125 mg, 2.00 mmol) and the mixture was stirred for 2 h at 60 °C. The reaction mixture was allowed to cool down, diluted with water and stirred for 30 min. The suspension was diluted with methanol and filtered over celite. The filtrate was concentrated. The residue was diluted with dichloromethane / methanol (small amount of methanol) and washed with brine. The aqueous phase was extracted with dichloromethane / methanol. The combined organic layers were dried over sodium sulfate, filtered and concentrated. The crude product was purified by column chromatography (silica gel, dichloromethane / methanol (0 - 5 %) gradient) followed by preparative HPLC (basic HT) to give 29.2 mg (98 % purity, 9 % yield) of the title compound.
LC-MS (Method 1): Rt = 1.24 min; MS (ESlpos): m/z = 476 [M+H]⁺
¹H-NMR (400 MHz, CHLOROFORM-d) δ [ppm]: 1.453 (4.99), 1.459 (4.59), 1.470 (5.13), 1.476 (4.41), 2.183 (5.10), 2.210 (0.45), 2.224 (0.63), 2.227 (0.60), 2.237 (0.53), 2.243 (0.81), 2.261 (1.20), 2.281 (0.61), 2.297 (0.54), 2.303 (0.54), 2.315 (0.55), 2.348 (0.43), 2.366 (0.58), 2.382 (0.47), 2.710 (0.52), 2.725 (0.52), 2.845 (0.67), 2.867 (0.83), 2.896 (1.11), 2.923 (1.35), 2.941 (1.20), 2.967 (1.71), 2.976 (0.43), 2.989 (0.45), 2.996 (0.52), 3.009 (0.49), 3.071 (1.52), 3.096 (1.92), 3.121 (1.29), 3.138 (0.44), 3.556 (1.05), 3.570 (1.31), 3.573 (1.22), 3.586 (1.15), 3.927 (14.08), 3.931 (16.00), 4.076 (0.75), 4.079 (0.77), 4.089 (1.82), 4.093 (1.97), 4.097 (1.81), 4.101 (2.27), 4.108 (1.65), 4.161 (1.62), 4.165 (2.08), 4.175 (2.34), 4.178 (2.98), 4.190 (1.20), 5.005 (3.79), 6.290 (4.20), 6.325 (5.03), 6.589 (1.59), 6.604 (1.55), 6.608 (1.70), 6.625 (1.40), 6.981 (1.45), 7.000 (2.65), 7.007 (0.47), 7.019 (1.30), 7.516 (1.21), 7.529 (0.44), 7.534 (1.38), 7.536 (1.47), 7.543 (1.15), 7.554 (1.14), 7.561 (1.22), 7.563 (1.29), 7.582 (0.94), 8.112 (2.57), 8.609 (2.52).

### Example 856

### 5-{(3R)-1-[cyclopropyl(6-methoxypyridin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

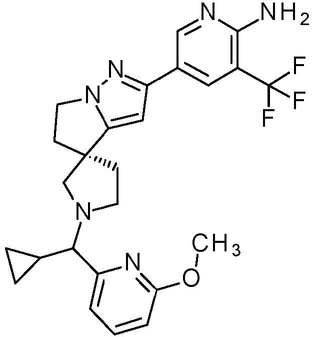

5-[(3R)-5',6'-Dihydrospiro[pyrrolidine-3,4'-pyrrolo[1,2-b]pyrazol]-2'-yl]-3-(trifluoromethyl)pyridin-2-amine (70.0 mg, 217 µmol) and cyclopropyl(6-methoxypyridin-2-yl)methanone (82.2 mg, 70 % purity, 325 µmol, Intermediate 554) were dissolved in methanol (780 µL). N,N-Diisopropylethylamine (150 µL, 870 µmol), titanium(IV) isopropoxide (190 µL, 650 µmol) were added and stirred for 16 h at 60 °C. To the cold reaction mixture was added sodium cyanoborohydride (40.8 mg, 650 µmol) and the mixture was stirred for 4 h at 60 °C. The reaction mixture was allowed to cool down, diluted with water and stirred for 30 min. The suspension was diluted with methanol and filtered over celite. The filtrate was concentrated. The residue was dissolved with dichloromethane / methanol (small amount of methanol) and washed with brine. The aqueous phase was extracted with dichloromethane / methanol. The combined organic layers were dried over sodium sulfate, filtered and concentrated. The crude product was purified by column chromatography (silica gel, dichloromethane / methanol (0 - 5 %) gradient) to give 18.4 mg (95 % purity, 17 % yield) of the title compound.
LC-MS (Method 1): Rt = 1.39 min; MS (ESlpos): m/z = 486 [M+H]⁺
¹H-NMR (400 MHz, CHLOROFORM-d) δ [ppm]: 0.309 (0.88), 0.320 (1.45), 0.333 (1.50), 0.342 (1.87), 0.355 (1.88), 0.366 (0.93), 0.376 (0.85), 0.388 (0.46), 0.414 (0.82), 0.426 (1.12), 0.438 (1.27), 0.450 (1.09), 0.462 (0.75), 0.474 (0.41), 0.658 (0.71), 0.672 (0.99), 0.690 (0.91), 1.128 (0.47), 1.139 (0.79), 1.150 (0.82), 1.161 (1.07), 1.172 (0.77), 1.183 (0.75), 1.195 (0.40), 1.262 (0.99), 1.997 (0.49), 2.010 (0.77), 2.025 (0.68), 2.029 (0.65), 2.044 (0.62), 2.056 (0.57), 2.072 (0.46), 2.090 (0.70), 2.110 (0.43), 2.163 (0.70), 2.171 (0.64), 2.179 (1.09), 2.192 (0.97), 2.211 (0.78), 2.224 (0.42), 2.232 (0.45), 2.460 (1.46), 2.469 (1.86), 2.484 (1.47), 2.493 (1.74), 2.539 (0.40), 2.558 (0.62), 2.571 (1.05), 2.586 (1.14), 2.603 (0.83), 2.662 (0.51), 2.675 (0.65), 2.681 (0.69), 2.693 (1.39), 2.710 (2.27), 2.725 (1.81), 2.733 (2.34), 2.759 (2.55), 2.783 (2.25), 2.840 (1.47), 2.864 (1.08), 2.918 (0.51), 2.932 (0.58), 2.940 (0.85), 2.954 (0.80), 2.977 (2.11), 3.000 (1.54), 3.138 (0.58), 3.150 (0.57), 3.914 (13.80), 3.923 (16.00), 4.119 (0.61), 4.140 (0.94), 4.155 (0.85), 4.167 (1.41), 4.183 (2.40), 4.203 (2.26), 4.218 (1.13), 4.230 (0.57), 4.989 (4.23), 5.310 (4.11), 6.203 (3.77), 6.244 (4.77), 6.599 (1.93), 6.610 (1.58), 6.619 (2.09), 6.630 (1.55), 7.013 (1.52), 7.029 (2.72), 7.045 (2.03), 7.529 (0.41), 7.535 (1.33), 7.554 (2.84), 7.574 (2.60), 7.592 (0.92), 8.110 (1.80), 8.114 (1.86), 8.126 (2.20), 8.131 (2.14), 8.608 (1.77), 8.625 (2.12).

### Example 857

### 5-{(3'R)-1'-[cyclopropyl(6-methoxypyridin-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

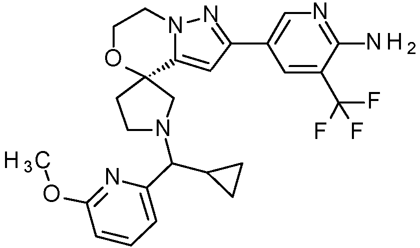

The compound was prepared similarly to **Example 856** using 5-[(3'R)-6,7-dihydrospiro[pyrazolo[5,1-c][1,4]oxazine-4,3'-pyrrolidin]-2-yl]-3-(trifluoromethyl)pyridin-2-amine (71.5 mg, 211 µmol, **Intermediate 537)** and cyclopropyl(6-methoxypyridin-2-yl)methanone (80.0 mg, 70 % purity, 316 µmol, **Intermediate 554)** to give 30.5 mg (95 % purity, 27 % yield) of the title compound.
LC-MS (Method 1): Rt = 1.33 min; MS (ESlpos): m/z = 502 [M+H]⁺
¹H-NMR (400 MHz, CHLOROFORM-d) δ [ppm]: 0.293 (0.44), 0.306 (0.65), 0.317 (0.76), 0.330 (0.86), 0.343 (0.65), 0.352 (0.54), 0.365 (0.72), 0.375 (0.52), 0.386 (0.42), 0.451 (0.62), 0.463 (0.75), 0.474 (0.67), 0.487 (0.46), 0.708 (0.58), 1.193 (0.45), 1.213 (0.53), 1.228 (0.44), 2.243 (0.61), 2.258 (0.68), 2.277 (0.91), 2.295 (0.57), 2.308 (0.52), 2.322 (0.48), 2.485 (1.33), 2.508 (1.26), 2.820 (0.45), 2.841 (0.51), 2.893 (0.80), 2.919 (0.94), 3.031 (1.01), 3.045 (0.95), 3.073 (0.53), 3.189 (1.02), 3.216 (0.92), 3.921 (16.00), 4.079 (0.45), 4.092 (1.41), 4.098 (1.47), 4.103 (2.62), 4.115 (1.96), 4.143 (0.49), 4.165 (1.99), 4.181 (2.55), 4.189 (1.00), 4.194 (1.13), 5.016 (3.26), 5.310 (5.59), 6.347 (2.37), 6.357 (3.51), 6.595 (1.17), 6.614 (1.97), 6.634 (0.85), 7.038 (1.23), 7.057 (1.45), 7.078 (0.92), 7.526 (1.06), 7.546 (1.28), 7.560 (0.81), 7.565 (1.00), 7.580 (0.88), 7.599 (0.65), 8.117 (2.38), 8.121 (2.41), 8.614 (2.18), 8.619 (2.22).

### Example 858

### 5-{(3R)-1-[1-(3-fluoropyridin-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

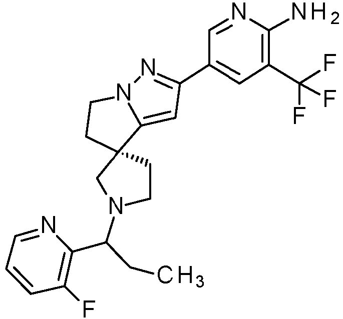

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (150 mg, 95 % purity, 396 µmol) and 1-(3-fluoropyridin-2-yl)propan-1-one (91.0 mg, 594 µmol) were dissolved in methanol (4.2 mL). N,N-Diisopropylethylamine (280 µL, 1.6 mmol), titanium(IV) isopropoxide (350 µL, 1.2 mmol) were added and stirred for 1 h at 60 °C. To the cold reaction mixture was added sodium cyanoborohydride (74.7 mg, 1.19 mmol) and the mixture was stirred for 16 h at 60 °C. The reaction mixture was allowed to cool down, diluted with water (0.5 mL) and stirred for 15 min. The suspension was filtered and the filter cake was washed with methanol. The filtrate was concentrated. The residue was purified by preparatve HPLC to give 91.4 mg (100 % purity, 50 % yield) of the title compound.
LC-MS (Method 1): Rt = 1.26 min; MS (ESlpos): m/z = 461 [M+H]⁺
¹H-NMR (400MHz, DMSO-d₆): δ [ppm]= 0.75 (t, 3H), 1.82 - 2.03 (m, 4H), 2.37 - 2.48 (m, 1H), 2.54 - 3.00 (m, 4H), 3.93 - 4.00 (m, 1H), 4.07 (q, 2H), 6.22 and 6.31 (2s, 1H), 6.51 (s, 2H), 7.36 - 7.42 (m, 1H), 7.66 - 7.73 (m, 1H), 7.96 - 8.00 (m, 1H), 8.43 - 8.49 (m, 1H), 8.53 - 8.57 (m, 1H).

### Example 859

### 3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1 and diastereomer 2)

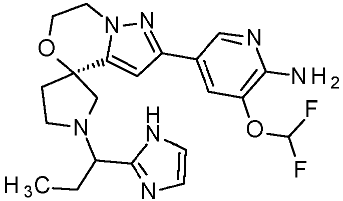

3-(Difluoromethoxy)-5-[(3'R)-6,7-dihydrospiro[pyrazolo[5,1-c][1,4]oxazine-4,3'-pyrrolidin]-2-yl]pyridin-2-amine (100 mg, 296 µmol, **Intermediate 525)** and 1-(1H-imidazol-2-yl)propan-1-one hydrogen chloride (1/1) (71.4 mg, 445 µmol, CAS 1314916-37-4) were dissolved in methanol (1 mL). N,N-Diisopropylethylamine (310 µL, 1.8 mmol), titanium(IV) isopropoxide (260 µL, 890 µmol) were added and the mixture was stirred for 20 h at 60 °C. Sodium cyanoborohydride (55.9 mg, 889 µmol) was added and the mixture was stirred for 5 h at 60 °C. The reaction mixture was allowed to cool down, diluted with water (1 mL) and stirred for 30 min. The suspension was diluted with methanol and filtered over celite. The filtrate was concentrated. The residue was diluted with dichloromethane / methanol (small amount of methanol) and washed with a saturated sodium bicarbonate solution. The aqueous phase was extracted with dichloromethane / methanol. The combined organic layers were dried over a hydrophobic filter and concentrated. The crude product was purified by column chromatography (silica gel, dichloromethane / methanol (0 - 7 %) gradient) to give 78.0 mg (99 % purity, 58 % yield) of the title compound.
LC-MS (Method 1): Rt = 0.83 min; MS (ESlpos): m/z = 446 [M+H]⁺
¹H-NMR (400 MHz, CHLOROFORM-d) δ [ppm]: 0.740 (3.25), 0.759 (7.57), 0.777 (4.15), 0.794 (1.21), 0.811 (1.92), 0.820 (1.04), 1.035 (0.63), 1.072 (0.44), 1.087 (0.50), 1.185 (16.00), 1.210 (1.65), 1.215 (1.79), 1.264 (0.85), 1.267 (0.91), 1.614 (0.98), 1.727 (0.44), 1.745 (0.43), 1.890 (0.44), 1.903 (0.52), 1.922 (0.46), 1.978 (0.74), 2.150 (0.43), 2.165 (0.68), 2.184 (0.73), 2.201 (0.48), 2.231 (0.47), 2.248 (0.41), 2.736 (0.52), 2.764 (0.81), 2.783 (0.58), 2.801 (0.44), 2.861 (0.53), 2.877 (0.67), 3.150 (0.85), 3.177 (0.75), 3.542 (0.44), 3.997 (0.78), 4.009 (1.33), 4.021 (1.28), 4.027 (1.19), 4.034 (0.85), 4.039 (0.91), 4.080 (0.94), 4.092 (1.48), 4.096 (1.32), 4.108 (1.51), 4.113 (1.00), 4.120 (0.53), 4.125 (0.50), 4.728 (2.42), 5.233 (3.62), 6.170 (2.10), 6.184 (3.36), 6.302 (0.79), 6.306 (1.12), 6.485 (1.54), 6.489 (2.24), 6.669 (0.73), 6.672 (0.99), 6.935 (6.17), 6.945 (4.20), 7.620 (0.84), 7.630 (1.26), 8.213 (1.38), 8.218 (1.39), 8.223 (2.07), 8.227 (2.02).

### Example 860

### 3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1)

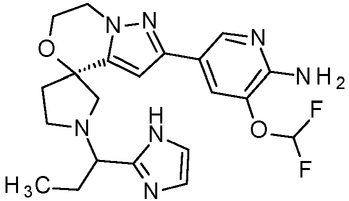

The compound 3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1 and diastereomer 2, **Example 859)** was separated into diastereomers by preparative chiral HPLC to give 19.5 mg (99 % purity) of the title compound (diastereomer 1 Rₜ = 4.0 - 5.1 min).

### Preparative chiral HPLC method:

Instrument: Sepiatec: Prep SFC100; Column: Chiral Art Amylose SA 5µ, 250x50; eluent A: CO2; eluent B: 2-propanol + 0.4 vol % diethylamine; isocratic: 30%B; flow: 100 mL/min; temperature: 40 °C; BPR: 150 bar; UV: 266 nm

### Analytical chiral HPLC method:

Instrument: Waters Acquity UPC2 QDA; Column: Chiral Art Amylose SA 3µ, 100x4.6; eluent A: CO2; eluent B: 2-propanol + 0.4 vol % diethylamine; isocratic: 30%B; flow: 4 mL/min; temperature: 37.5 °C; BPR: 100 bar; UV: 220 nm
Analytical chiral HPLC Rt = 1.05 min
[α]²²_{D}: +57.1° (c = 1.00, methanol)
LC-MS (Method 1): Rt = 0.83 min; MS (ESlpos): m/z = 446 [M+H]⁺
¹H-NMR (400 MHz, CHLOROFORM-d) δ [ppm]: -0.125 (1.54), 0.014 (14.96), 0.033 (16.00), 0.042 (3.78), 0.049 (0.84), 0.170 (1.54), 0.764 (5.47), 0.782 (12.42), 0.801 (5.71), 1.141 (0.20), 1.160 (2.83), 1.175 (2.73), 1.212 (2.53), 1.668 (1.79), 1.723 (0.89), 1.741 (0.94), 1.746 (0.89), 1.757 (1.09), 1.764 (0.94), 1.775 (0.99), 1.780 (1.09), 1.798 (0.84), 1.817 (0.30), 1.896 (0.30), 1.914 (0.84), 1.926 (0.99), 1.933 (0.94), 1.945 (1.04), 1.948 (0.89), 1.959 (0.75), 1.967 (0.75), 1.978 (0.65), 1.997 (0.20), 2.129 (0.65), 2.135 (0.94), 2.151 (0.60), 2.166 (0.75), 2.171 (0.84), 2.185 (1.49), 2.200 (1.34), 2.206 (1.24), 2.221 (1.09), 2.277 (0.80), 2.296 (1.29), 2.312 (1.44), 2.328 (0.80), 2.346 (0.55), 2.573 (3.53), 2.584 (0.75), 2.591 (0.60), 2.600 (0.89), 2.627 (0.94), 2.643 (0.65), 2.853 (1.34), 2.880 (4.57), 2.897 (4.17), 2.923 (1.09), 3.053 (0.80), 3.073 (1.29), 3.091 (1.14), 3.111 (0.65), 3.369 (0.20), 3.524 (1.44), 3.536 (1.59), 3.548 (1.54), 3.558 (1.39), 3.978 (0.25), 3.992 (0.45), 4.003 (0.70), 4.007 (1.34), 4.021 (3.63), 4.033 (5.12), 4.045 (2.34), 4.063 (0.55), 4.074 (0.75), 4.094 (0.65), 4.103 (4.02), 4.115 (6.16), 4.129 (1.94), 4.747 (5.81), 6.191 (12.47), 6.325 (3.83), 6.508 (7.20), 6.692 (3.43), 6.954 (0.75), 6.968 (8.99), 7.476 (0.40), 7.640 (3.78), 7.641 (4.02), 7.644 (3.88), 8.237 (6.31), 8.241 (6.16), 9.363 (0.55).

### Example 861

### 3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 2)

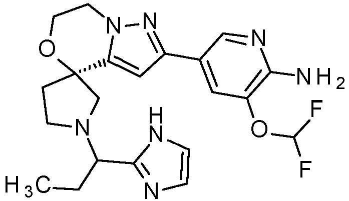

For the preparation of the diastereomeric mixture of the title compound see **Example 859.** The diastereomers were separated by preparative chiral HPLC (method see **Example 860**) to give 28.2 mg (99 % purity) of the title compound (diastereomer 2 Rₜ = 7.3 - 9.5 min).
Analytical chiral HPLC (for method see **Example 860)**: Rₜ = 1.97 min
[α]²²_{D}: +58.3° (c = 1.00, methanol)
LC-MS (Method 1): Rt = 0.83 min; MS (ESlpos): m/z = 446 [M+H]⁺
¹H-NMR (400 MHz, CHLOROFORM-d) δ [ppm]: -0.108 (0.54), 0.016 (0.74), 0.032 (5.11), 0.050 (5.44), 0.058 (1.24), 0.065 (0.21), 0.187 (0.54), 0.782 (1.44), 0.801 (3.37), 0.819 (1.52), 1.229 (0.16), 1.623 (1.52), 1.724 (0.16), 1.743 (0.21), 1.747 (0.20), 1.759 (0.26), 1.777 (0.25), 1.781 (0.26), 1.799 (0.20), 1.909 (0.23), 1.921 (0.25), 1.928 (0.25), 1.940 (0.28), 1.943 (0.25), 1.955 (0.20), 1.962 (0.20), 1.973 (0.16), 2.187 (0.23), 2.201 (0.34), 2.221 (0.46), 2.238 (0.31), 2.251 (0.23), 2.268 (0.43), 2.284 (0.36), 2.301 (0.20), 2.589 (16.00), 2.770 (0.85), 2.797 (1.21), 2.816 (0.84), 2.832 (0.57), 2.851 (0.29), 3.184 (0.77), 3.210 (0.67), 3.574 (0.38), 3.586 (0.43), 3.597 (0.41), 3.609 (0.38), 4.047 (0.38), 4.054 (0.44), 4.059 (0.74), 4.066 (0.87), 4.073 (0.69), 4.078 (0.70), 4.136 (0.84), 4.148 (1.11), 4.153 (0.79), 4.160 (0.43), 4.165 (0.41), 4.766 (1.52), 6.221 (3.49), 6.346 (0.92), 6.530 (1.88), 6.713 (0.92), 6.970 (1.05), 6.980 (1.05), 7.669 (1.00), 7.671 (1.05), 7.674 (1.00), 8.264 (1.60), 8.269 (1.67), 9.353 (0.18).

### Example 862

### 2-amino-5-{(3'R)-1'-[1-(1H-imidazol-2-yl)propyl]-6, 7-dihydrospiro[pyrazolo[5,1-c][1,4]oxazine-4,3'-pyrrolidin]-2-yl}pyridine-3-carbonitrile (diastereomer 1 and diastereomer 2)

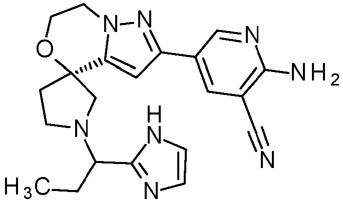

The compound was prepared similarly to **Example 859** using 2-amino-5-[(3'R)-6,7-dihydrospiro[pyrazolo[5,1-c][1,4]oxazine-4,3'-pyrrolidin]-2-yl]pyridine-3-carbonitrile (100 mg, 337 µmol, **Intermediate 524)** and 1-(1H-imidazol-2-yl)propan-1-one hydrogen chloride (1/1) (81.3 mg, 506 µmol, CAS 1314916-37-4) to give 88.0 mg (99 % purity, 64 % yield) of the title compound.
LC-MS (Method 1): Rₜ = 0.77 min; MS (ESlpos): m/z = 405 [M+H]⁺
¹H-NMR (400 MHz, CHLOROFORM-d) δ [ppm]: 0.740 (6.19), 0.759 (14.18), 0.777 (6.87), 0.811 (0.87), 1.184 (3.86), 1.209 (1.03), 1.267 (0.52), 1.353 (0.48), 1.697 (0.54), 1.732 (1.06), 1.741 (0.92), 1.750 (1.10), 1.766 (0.82), 1.783 (0.59), 1.876 (0.88), 1.887 (0.99), 1.895 (1.17), 1.907 (1.34), 1.928 (1.18), 1.940 (1.00), 1.959 (0.61), 1.978 (1.29), 2.120 (0.81), 2.141 (1.16), 2.155 (1.92), 2.175 (1.89), 2.191 (1.38), 2.221 (0.75), 2.237 (1.22), 2.254 (1.17), 2.275 (1.02), 2.295 (0.89), 2.312 (0.55), 2.329 (0.42), 2.571 (0.41), 2.592 (0.73), 2.729 (1.83), 2.756 (2.10), 2.771 (1.00), 2.789 (1.70), 2.807 (1.33), 2.833 (1.07), 2.861 (1.84), 2.881 (1.94), 2.908 (0.64), 3.040 (0.41), 3.059 (0.69), 3.078 (0.62), 3.160 (2.11), 3.186 (1.83), 3.517 (0.68), 3.528 (0.76), 3.540 (0.77), 3.552 (1.47), 3.564 (1.07), 3.576 (1.02), 3.587 (0.89), 3.989 (0.71), 4.002 (1.86), 4.014 (3.04), 4.027 (3.10), 4.033 (2.80), 4.039 (1.80), 4.045 (2.81), 4.062 (0.57), 4.080 (1.94), 4.093 (3.65), 4.108 (3.50), 4.120 (1.29), 4.125 (1.21), 5.188 (6.38), 5.233 (13.90), 6.177 (5.05), 6.189 (7.52), 6.929 (0.54), 6.944 (16.00), 6.952 (11.33), 8.012 (2.76), 8.018 (3.12), 8.021 (4.22), 8.027 (4.08), 8.554 (2.91), 8.560 (3.10), 8.564 (4.45), 8.570 (4.20).

### Example 863

### 2-amino-5-{(3'R)-1'-[1-(1H-imidazol-2-yl)propyl]-6, 7-dihydrospiro[pyrazolo[5,1-c][1,4]oxazine-4,3'-pyrrolidin]-2-yl}pyridine-3-carbonitrile (diastereomer 1)

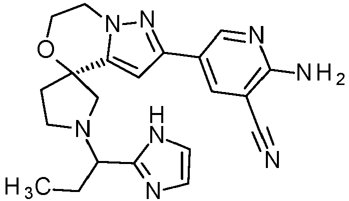

The compound 2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastereomer 1 and diastereomer 2, **Example 862)** was separated into diastereomers by preparative chiral HPLC to give 22.2 mg (98 % purity) of the title compound (diastereomer 1 Rₜ = 5.45 - 6.4 min).

### Preparative chiral HPLC method:

Instrument: sepiatec: Prep SFC100; Column: Chiralpak IG 5µ, 250x50; eluent A: CO2 ; eluent B: methanol + 0.2 % aqueous ammonia (32%); isocratic: 40%B; flow: 100 mL/min; temperature: 40 °C; BPR: 150 bar UV: 210 nm

### Analytical chiral HPLC method:

Instrument: Waters Acquity UPC2 QDA; Column: Chiralpak IG 3µ, 100x4.6; eluent A: CO2; eluent B: methanol + 0.2 % aqueous ammonia (32%); isocratic: 40%B; flow: 4 mL/min; temperature: 40 °C; BPR: 100 bar UV: 220 nm
Analytical chiral HPLC Rₜ = 1.54 min
[α]²²_{D}: +64.9° (c = 1.00, methanol)
LC-MS (Method 1): Rt = 0.77 min; MS (ESlpos): m/z = 405 [M+H]⁺
1H-NMR (400 MHz, CHLOROFORM-d) δ [ppm]: -0.124 (0.81), 0.015 (8.66), 0.033 (9.39), 0.042 (1.96), 0.050 (0.50), 0.170 (0.83), 0.763 (6.99), 0.782 (16.00), 0.801 (7.30), 1.213 (0.98), 1.731 (0.98), 1.750 (1.29), 1.755 (1.31), 1.765 (1.62), 1.784 (1.67), 1.788 (1.76), 1.807 (1.45), 1.899 (0.48), 1.918 (1.22), 1.929 (1.36), 1.936 (1.31), 1.947 (1.48), 1.951 (1.26), 1.963 (1.05), 1.970 (0.98), 1.981 (0.83), 2.144 (0.79), 2.159 (0.98), 2.165 (1.17), 2.178 (1.96), 2.193 (1.69), 2.199 (1.65), 2.214 (1.36), 2.282 (1.00), 2.299 (1.62), 2.315 (1.79), 2.332 (1.03), 2.351 (0.72), 2.584 (0.86), 2.600 (1.10), 2.643 (0.74), 2.850 (1.17), 2.877 (3.31), 2.901 (4.32), 2.927 (1.45), 3.057 (1.00), 3.076 (1.65), 3.096 (1.45), 3.115 (0.79), 3.529 (1.50), 3.539 (1.69), 3.551 (1.65), 3.563 (1.48), 3.994 (0.43), 3.997 (0.45), 4.013 (1.62), 4.025 (4.41), 4.037 (5.79), 4.044 (1.55), 4.049 (2.89), 4.067 (0.52), 4.073 (0.67), 4.078 (0.86), 4.104 (4.10), 4.116 (7.23), 4.127 (2.31), 4.130 (2.34), 5.231 (7.77), 6.198 (11.66), 6.955 (0.57), 6.973 (12.35), 8.035 (7.96), 8.041 (8.11), 8.577 (8.49), 8.584 (8.35), 9.453 (0.45).

### Example 864

### 2-amino-5-{(3'R)-1'-[1-(1H-imidazol-2-yl)propyl]-6, 7-dihydrospiro[pyrazolo[5,1-c][1,4]oxazine-4,3'-pyrrolidin]-2-yl}pyridine-3-carbonitrile (diastereomer 2)

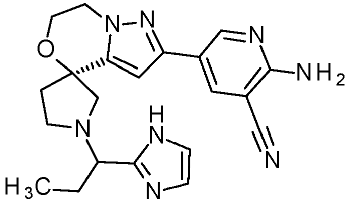

For the preparation of the diastereomeric mixture of the title compound see **Example 862.**

The diastereomers were separated by preparative chiral HPLC (method see **Example 863)** to give 29.1 mg (99 % purity) of the title compound (diastereomer 2 Rₜ = 7.14 - 8.20 min).
Analytical chiral HPLC (method see **Example 863):** Rₜ = 2.16 min
[α]²²_{D}: +75.0° (c = 1.00, methanol)
LC-MS (Method 1): Rₜ = 0.77 min; MS (ESlpos): m/z = 405 [M+H]⁺
¹H-NMR (400 MHz, CHLOROFORM-d) δ [ppm]: 0.320 (0.62), 0.330 (16.00), 0.339 (0.53), 1.060 (0.97), 1.079 (2.26), 1.097 (1.03), 2.056 (0.20), 2.064 (0.17), 2.074 (0.20), 2.080 (0.22), 2.098 (0.20), 2.161 (0.32), 2.195 (0.20), 2.202 (0.27), 2.213 (0.26), 2.221 (0.24), 2.232 (0.25), 2.235 (0.22), 2.247 (0.17), 2.463 (0.18), 2.477 (0.25), 2.497 (0.30), 2.514 (0.20), 2.561 (0.31), 2.578 (0.26), 2.595 (0.16), 3.036 (0.64), 3.062 (0.75), 3.086 (0.27), 3.097 (0.34), 3.103 (0.51), 3.112 (0.34), 3.117 (0.37), 3.132 (0.22), 3.498 (0.55), 3.525 (0.49), 3.863 (0.28), 3.874 (0.32), 3.886 (0.30), 3.898 (0.27), 4.343 (0.26), 4.355 (0.54), 4.361 (0.65), 4.368 (0.42), 4.373 (0.62), 4.425 (0.58), 4.437 (0.76), 4.442 (0.58), 4.449 (0.29), 4.454 (0.28), 5.626 (1.24), 6.512 (2.41), 7.525 (3.13), 8.347 (1.16), 8.353 (1.18), 8.891 (1.25), 8.896 (1.21).

### Example 865

### 3-(difluoromethyl)-5-{(3'R)-1'-[1-(1H-imidazol-2-yl)propyl]-6, 7-dihydrospiro[pyrazolo[5,1-c][1,4]oxazine-4,3'-pyrrolidin]-2-yl}pyridin-2-amine (diastereomer 1 and diastereomer 2)

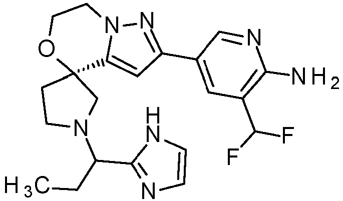

The compound was prepared similarly to **Example 859** using 3-(difluoromethyl)-5-[(3'R)-6,7-dihydrospiro[pyrazolo[5,1-c][1,4]oxazine-4,3'-pyrrolidin]-2-yl]pyridin-2-amine (130 mg, 405 µmol, **Intermediate 523)** and 1-(1H-imidazol-2-yl)propan-1-one hydrogen chloride (1/1) (97.5 mg, 607 µmol, CAS 1314916-37-4) to give 65.0 mg (95 % purity, 36 % yield) of the title compound.
LC-MS (Method 1): Rt = 0.81 min; MS (ESlpos): m/z = 430 [M+H]⁺
¹H-NMR (400 MHz, CHLOROFORM-d) δ [ppm]: 0.009 (16.00), 0.018 (0.68), 0.752 (4.86), 0.770 (11.02), 0.789 (5.26), 0.820 (0.61), 1.193 (1.63), 1.206 (1.10), 1.354 (2.74), 1.388 (2.40), 1.407 (4.08), 1.426 (2.01), 1.721 (0.43), 1.740 (0.62), 1.755 (0.85), 1.774 (0.87), 1.795 (0.67), 1.809 (0.40), 1.894 (0.70), 1.905 (0.83), 1.912 (0.98), 1.923 (1.11), 1.946 (0.94), 1.957 (0.81), 1.965 (0.61), 1.975 (0.45), 2.150 (0.52), 2.166 (0.83), 2.184 (1.18), 2.194 (0.79), 2.202 (1.40), 2.215 (0.69), 2.222 (0.94), 2.237 (0.76), 2.254 (1.13), 2.270 (1.10), 2.289 (0.96), 2.309 (0.75), 2.325 (0.47), 2.633 (0.61), 2.647 (0.59), 2.770 (2.04), 2.797 (2.85), 2.814 (1.46), 2.832 (1.17), 2.852 (0.91), 2.867 (0.80), 2.889 (0.43), 2.912 (2.61), 2.954 (0.69), 2.972 (1.70), 2.991 (1.66), 3.009 (0.61), 3.086 (0.57), 3.106 (0.50), 3.183 (1.80), 3.210 (1.58), 3.291 (0.88), 3.486 (0.43), 3.503 (0.94), 3.519 (1.22), 3.536 (0.91), 3.553 (0.43), 3.565 (0.53), 3.576 (0.58), 3.588 (0.63), 3.598 (1.26), 3.609 (1.08), 3.620 (1.00), 3.631 (0.89), 3.999 (0.57), 4.012 (1.33), 4.024 (2.41), 4.036 (2.61), 4.042 (2.44), 4.049 (1.77), 4.054 (1.93), 4.084 (0.62), 4.094 (1.53), 4.107 (2.63), 4.111 (2.80), 4.122 (3.13), 4.127 (2.17), 4.134 (1.21), 4.139 (1.16), 4.929 (4.21), 5.242 (0.85), 6.213 (3.86), 6.219 (7.09), 6.422 (1.45), 6.560 (2.89), 6.698 (1.39), 6.938 (0.46), 6.963 (13.56), 6.970 (7.48), 7.878 (2.77), 8.470 (2.66).

### Example 866

### 3-(difluoromethyl)-5-{(3'R)-1'-[1-(1H-imidazol-2-yl)propyl]-6, 7-dihydrospiro[pyrazolo[5,1-c][1,4]oxazine-4,3'-pyrrolidin]-2-yl}pyridin-2-amine (diastereomer 1)

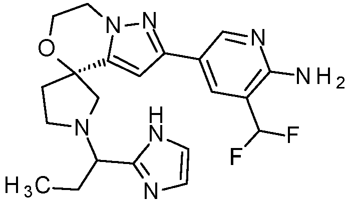

The compound 3-(difluoromethyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1 and diastereomer 2, **Example 865)** was separated into diastereomers by preparative chiral HPLC to give 11.0 mg (90 % purity) of the title compound (diastereomer 1 Rt = 4.3 -5.8 min).

### Preparative chiral HPLC method:

Instrument: Sepiatec: Prep SFC100; Column: Chiral Art Amylose SA 5µ, 250x50; eluent A: CO2; eluent B: 2-propanol + 0.4 vol % diethylamine; isocratic: 30%B; flow: 100 mL/min; temperature: 40 °C; BPR: 150 bar; UV: 254 nm

### Analytical chiral HPLC method:

Instrument: Waters Acquity UPC2 QDA; Column: Chiral Art Amylose SA 3µ, 100x4.6; eluent A: CO2; eluent B: 2-propanol + 0.4 vol % diethylamine; isocratic: 30%B; flow: 4 mL/min; temperature: 40 °C; BPR: 1800 psi; UV: 254 nm
Analytical chiral HPLC Rₜ = 1.12 min
[α]²²_{D}: +59.4° (c = 1.00, methanol)
LC-MS (Method 1): Rt = 0.81 min; MS (ESlpos): m/z = 430 [M+H]⁺
¹H-NMR (400 MHz, CHLOROFORM-d) δ [ppm]: -0.125 (1.33), 0.014 (12.69), 0.033 (13.82), 0.042 (3.03), 0.170 (1.33), 0.764 (6.02), 0.782 (13.98), 0.801 (6.46), 1.198 (0.48), 1.212 (0.40), 1.709 (0.77), 1.727 (1.17), 1.745 (1.33), 1.750 (1.29), 1.761 (1.49), 1.779 (1.41), 1.784 (1.49), 1.803 (1.21), 1.821 (0.61), 1.899 (0.44), 1.917 (1.05), 1.928 (1.17), 1.936 (1.13), 1.947 (1.25), 1.963 (0.93), 1.970 (0.89), 1.981 (0.77), 2.155 (0.65), 2.170 (0.85), 2.175 (0.97), 2.190 (1.70), 2.204 (1.45), 2.210 (1.41), 2.225 (1.25), 2.283 (0.93), 2.301 (1.49), 2.317 (1.62), 2.334 (0.97), 2.351 (0.69), 2.586 (0.77), 2.645 (0.73), 2.856 (1.33), 2.883 (4.16), 2.902 (4.28), 2.929 (1.29), 3.057 (0.85), 3.076 (1.45), 3.096 (1.33), 3.115 (0.73), 3.300 (0.53), 3.303 (0.53), 3.526 (1.49), 3.537 (1.66), 3.549 (1.58), 3.560 (1.45), 3.997 (0.53), 4.013 (1.54), 4.026 (4.08), 4.038 (5.29), 4.051 (2.59), 4.068 (0.89), 4.079 (1.05), 4.089 (0.40), 4.099 (0.97), 4.108 (4.32), 4.120 (6.71), 4.131 (2.30), 4.932 (6.42), 6.210 (11.96), 6.230 (0.44), 6.430 (2.55), 6.567 (5.17), 6.605 (0.73), 6.705 (2.38), 6.954 (0.61), 6.971 (16.00), 7.476 (0.40), 7.886 (3.88), 8.472 (3.68), 8.474 (3.80), 8.477 (3.72).

### Example 867

### 3-(difluoromethyl)-5-{(3'R)-1'-[1-(1H-imidazol-2-yl)propyl]-6, 7-dihydrospiro[pyrazolo[5, 1-c][1,4]oxazine-4,3'-pyrrolidin]-2-yl}pyridin-2-amine (diastereomer 2)

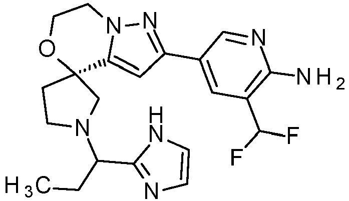

For the preparation of the diastereomeric mixture of the title compound see **Example 865.** The diastereomers were separated by preparative chiral HPLC (method see **Example 866**) to give 21.0 mg (95 % purity) of the title compound (diastereomer 2 Rₜ = 7.6 - 9.9 min).
Analytical chiral HPLC (method see **Example 866):** Rt = 2.08 min
[α]²²D: +147.9° (c = 1.00, methanol)
LC-MS (Method 1): Rₜ = 0.81 min; MS (ESlpos): m/z = 430 [M+H]⁺
¹H-NMR (400 MHz, CHLOROFORM-d) δ [ppm]: -0.111 (1.04), 0.014 (1.63), 0.029 (11.24), 0.047 (9.21), 0.056 (2.00), 0.184 (1.01), 0.776 (6.99), 0.795 (16.00), 0.813 (7.44), 1.728 (2.20), 1.747 (3.15), 1.751 (3.10), 1.762 (3.10), 1.781 (2.20), 1.785 (2.17), 1.803 (1.49), 1.821 (0.56), 1.891 (0.42), 1.909 (1.15), 1.921 (1.32), 1.928 (1.27), 1.940 (1.49), 1.943 (1.24), 1.955 (1.01), 1.962 (0.93), 1.973 (0.82), 2.166 (0.68), 2.186 (1.15), 2.201 (1.83), 2.220 (2.23), 2.237 (1.55), 2.253 (1.21), 2.270 (2.25), 2.286 (1.89), 2.304 (1.04), 2.320 (0.59), 2.766 (4.45), 2.792 (6.08), 2.814 (4.48), 2.832 (3.07), 2.849 (1.58), 3.192 (4.00), 3.219 (3.55), 3.571 (2.11), 3.583 (2.34), 3.594 (2.23), 3.605 (1.97), 4.033 (0.48), 4.047 (1.97), 4.060 (3.83), 4.068 (4.42), 4.073 (3.41), 4.079 (3.38), 4.108 (0.73), 4.137 (4.31), 4.149 (5.94), 4.153 (3.86), 4.160 (2.17), 4.165 (2.06), 4.987 (7.58), 6.233 (15.38), 6.451 (2.99), 6.588 (6.00), 6.726 (2.85), 6.974 (10.90), 7.907 (4.73), 8.500 (4.68), 8.502 (4.73), 8.505 (4.39), 9.532 (0.65).

### Example 868

### (3R)-2'-(6-amino-5-{[1-(1-propyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)

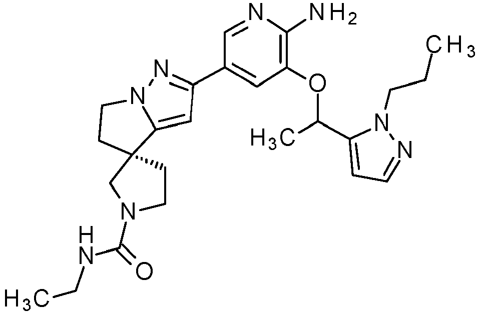

3-{[1-(1-Propyl-1H-pyrazol-5-yl)ethyl]oxy}-5-(4,4,5,5-tetramethyl-1,3,2-dioxaborolan-2-yl)pyridin-2-amine (91.0 mg, 244 µmol), (3R)-1-(ethylcarbamoyl)-5',6'-dihydrospiro[pyrrolidine-3,4'-pyrrolo[1,2-b]pyrazol]-2'-yl trifluoromethanesulfonate (140 mg, 367 µmol) and potassium phosphate (1.5 mL, 0.50 M, 730 µmol) were dissolved in degassed 1,4-dioxane. XPhos Pd G2 (9.60 mg, 12.2 µmol) was added and the mixture was stirred for 2 h at 100 °C. The cold reaction mixture was diluted with water and extracted with ethyl acetate. The combined organic layers were washed with brine, dried over sodium sulfate, filtered and concentrated. The residue was purified by preparatie HPLC followed by preparative TLC (dichloromethane / methanol 9:1 gradient) to give 5.00 mg (98 % purity, 4 % yield) of the title compound.
LC-MS (Method 1): Rt = 0.88 min; MS (ESlpos): m/z = 480 [M+H]⁺
¹H NMR (DMSO-d₆, 400 MHz): δ (ppm) 7.92 (d, 1H), 7.41 (d, 1H), 7.37 (d, 1H), 6.36 (d, 1H), 6.28 (d, 1H), 6.16 (t, 1H), 5.81 (q, 1H), 5.75 (s, 2H), 4.15 (t, 2H), 4.03 - 4.08 (m, 2H), 3.45 - 3.52 (m, 1H), 3.35 - 3.43 (m, 3H), 3.01 - 3.09 (m, 2H), 1.98 - 2.10 (m, 2H), 1.73 (sxt, 2H), 1.62 (d, 3H), 1.02 (t, 3H), 0.80 (2t, 3H).

### Example 869

### (3R)-2'-(6-amino-5-{[1-(1,3-thiazol-4-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)

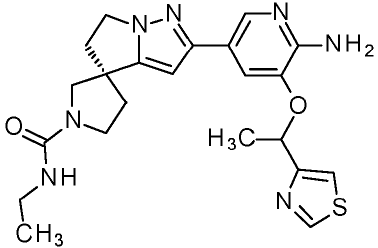

5-(4,4,5,5-Tetramethyl-1,3,2-dioxaborolan-2-yl)-3-{[1-(1,3-thiazol-4-yl)ethyl]oxy}pyridin-2-amine (285 mg, 36 % purity, 295 µmol) was dissolved in 1,4-dioxane (1.7 mL) under argon. (3R)-1-(Ethylcarbamoyl)-5',6'-dihydrospiro[pyrrolidine-3,4'-pyrrolo[1,2-b]pyrazol]-2'-yl trifluoromethanesulfonate (94.1 mg, 246 µmol), potassium phosphate (1.5 mL, 0.50 M, 740 µmol) and XPhos Pd G2 (9.69 mg, 12.3 µmol) were added and the mixture was stirred for 1 h at 100 °C. The reaction mixture was allowed to cool down, diluted with dichloromethane, washed with water and brine.The organic phase was dried over a hydrophobic filter and concentrated. The residue was purified by preparative HPLC to give 15.3 mg (95 % purity, 13 % yield) of the title compound.
LC-MS (Method 1): Rt = 0.82 min; MS (ESlpos): m/z = 454 [M+H]⁺
¹H-NMR (400MHz, DMSO-d₆): δ [ppm]= 1.02 (t, 3H), 1.65 (d, 3H), 1.97 - 2.10 (m, 2H), 3.00 - 3.10 (m, 2H), 3.35 - 3.42 (m, 3H), 3.45 - 3.52 (m, 1H), 4.14 (t, 2H), 5.70 (q, 1H), 5.80 (s, 2H), 6.15 (t, 1H), 6.30 (d, 1H), 7.38 (s, 1H), 7.73 (d, 1H), 7.92 (d, 1H), 9.10 (d, 1H).

### Example 870

### (3R)-2'-(6-amino-5-{[1-(pyrimidin-4-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1 and diastereomer 2)

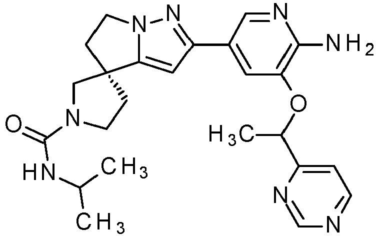

(3R)-1-[(Propan-2-yl)carbamoyl]-5',6'-dihydrospiro[pyrrolidine-3,4'-pyrrolo[1,2-b]pyrazol]-2'-yl trifluoromethanesulfonate (146 mg, 369 µmol), 3-{[1-(pyrimidin-4-yl)ethyl]oxy}-5-(4,4,5,5-tetramethyl-1,3,2-dioxaborolan-2-yl)pyridin-2-amine (139 mg, 406 µmol), potassium phosphate (2.2 mL, 0.50M, 1.10 mmol) and Xphos Pd G2 (14.5 mg, 18.5 µmol) were combined in degassed 1,4-dioxane (6.1 mL) and the mixture was stirred for 80 min at 100 °C. The reaction mixture was allowed to cooled down and diluted with water and dichloromethane. The layers were separated and the aqueous phase was extracted with dichloromethane / methanol 9:1. The combined organic layers were dried and concentrated. The residue was purified by column chromatography (silica gel, dichloromethane / methanol (0 - 10 %) gradient) to give 106 mg (90 % purity, 56 % yield) of the title compound.
LC-MS (Method 1): Rt = 0.80 min; MS (ESlpos): m/z = 463 [M+H]⁺
¹H NMR (DMSO-d₆, 400 MHz): δ (ppm) 9.18 (t, 1H), 8.81 (dd, 1H), 7.91 (d, 1H), 7.67 (dt, 1H), 7.24 (t, 1H), 6.26 (s, 1H), 5.97 (s, 2H), 5.81 (d, 1H), 5.55 (q, 1H), 4.12 (t, 2H), 3.69 - 3.82 (m, 1H), 3.44 - 3.52 (m, 1H), 3.35 - 3.41 (m, 3H), 1.95 - 2.08 (m, 2H), 1.62 (d, 3H), 1.06 and 1.07 (2d, 6H).

### Example 871

### (3R)-2'-(6-amino-5-{[1-(2,4-dimethylpyrimidin-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1)

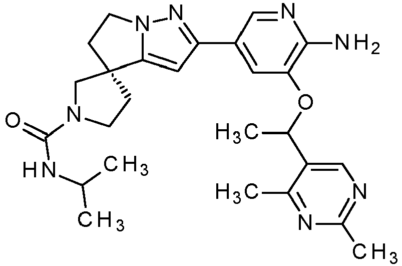

The compound was prepared similarly to **Example 870** using (3R)-1-[(propan-2-yl)carbamoyl]-5',6'-dihydrospiro[pyrrolidine-3,4'-pyrrolo[1,2-b]pyrazol]-2'-yl trifluoromethanesulfonate (88.6 mg, 223 µmol) and 3-{[1-(2,4-dimethylpyrimidin-5-yl)ethyl]oxy}-5-(4,4,5,5-tetramethyl-1,3,2-dioxaborolan-2-yl)pyridin-2-amine (enantiomer 1) (91.0 mg, 246 µmol) to give 65.0 mg (90 % purity, 53 % yield) of the title compound.
[α]²⁰_{D}: -14.3° (c = 1.00, methanol)
LC-MS (Method 1): Rt = 0.84 min; MS (ESlpos): m/z = 491 [M+H]⁺
¹H-NMR (500MHz, DMSO-d₆): δ [ppm]= 1.05 - 1.08 (m, 6H), 1.60 (d, 3H), 1.97 - 2.09 (m, 2H), 2.51 (br s, 3H), 2.54 (s, 3H), 3.34 - 3.41 (m, 3H), 3.46 - 3.52 (m, 1H), 3.71 - 3.79 (m, 1H), 4.11 - 4.16 (m, 2H), 5.77 - 5.83 (m, 2H), 5.93 (s, 2H), 6.27 (s, 1H), 7.28 (d, 1H), 7.88 (d, 1H), 8.72 (s, 1H).

### Example 872

### (3R)-2'-(6-amino-5-{[1-(2,4-dimethylpyrimidin-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 2)

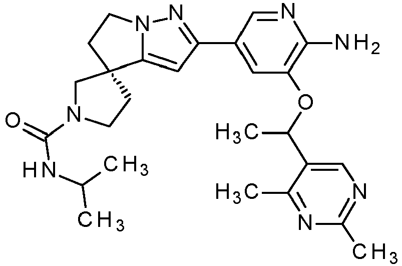

The compound was prepared similarly to **Example 870** using (3R)-1-[(propan-2-yl)carbamoyl]-5',6'-dihydrospiro[pyrrolidine-3,4'-pyrrolo[1,2-b]pyrazol]-2'-yl trifluoromethanesulfonate (88.6 mg, 223 µmol) and 3-{[1-(2,4-dimethylpyrimidin-5-yl)ethyl]oxy}-5-(4,4,5,5-tetramethyl-1,3,2-dioxaborolan-2-yl)pyridin-2-amine (enantiomer 2) (91.0 mg, 246 µmol) to give 67.0 mg (90 % purity, 55 % yield) of the title compound.
[α]²⁰_{D}: +106.3° (c = 1.00, methanol)
LC-MS (Method 1): Rt = 0.84 min; MS (ESlpos): m/z = 491 [M+H]⁺
¹H-NMR (500MHz, DMSO-d₆): δ [ppm]= 1.04 - 1.08 (m, 6H), 1.60 (d, 3H), 1.98 - 2.09 (m, 2H), 2.51 (s, 3H), 2.54 (s, 3H), 3.35 - 3.42 (m, 3H), 3.49 (dq, 1H), 3.71 - 3.80 (m, 1H), 4.14 (t, 2H), 5.77 - 5.83 (m, 2H), 5.93 (s, 2H), 6.27 (s, 1H), 7.28 (d, 1H), 7.88 (d, 1H), 8.73 (s, 1H).

### Example 873

### (3R)-2'-{6-amino-5-[(1R)-1-(5-methyl-1,3,4-oxadiazol-2-yl)ethoxy]pyridin-3-yl}-N-(propan-2-yl)-5',6'-dihydrospiro[pyrrolidine-3,4'-pyrrolo[1,2-b]pyrazole]-1-carboxamide

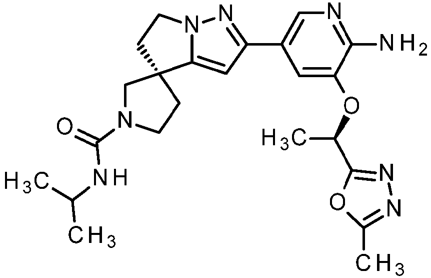

3-[(1R)-1-(5-Methyl-1,3,4-oxadiazol-2-yl)ethoxy]-5-(4,4,5,5-tetramethyl-1,3,2-dioxaborolan-2-yl)pyridin-2-amine (172 mg, 33 % purity, 161 µmol) was dissolved in 1,4-dioxane (910 µL) under argon. (3R)-1-[(Propan-2-yl)carbamoyl]-5',6'-dihydrospiro[pyrrolidine-3,4'-pyrrolo[1,2-b]pyrazol]-2'-yl trifluoromethanesulfonate (53.3 mg, 135 µmol), potassium phosphate (810 µL, 0.50 M, 400 µmol) and XPhos Pd G2 (10.6 mg, 13.5 µmol) were added and the mixture was stirred for 1 h at 100 °C. The reaction mixture was allowed to cool down, diluted with dichloromethane, washed with water and brine. The organic phase was dried over a hydrophobic filter and concentrated. The residue was purified by preparative HPLC to give 15.4 mg (100 % purity, 25 % yield) of the title compound.
[α]²⁰_{D}: +198.7° (c = 1.00, methanol)
LC-MS (Method 1): Rt = 0.80 min; MS (ESlpos): m/z = 467 [M+H]⁺
¹H-NMR (400MHz, DMSO-d₆): δ [ppm]= 1.06 (d, 3H), 1.97 (d, 3H), 1.71 (d, 3H), 1.98 - 2.11 (m, 2H), 3.36 - 3.43 (m, 3H), 3.46 - 3.54 (m, 1H), 3.71 - 3.80 (m, 1H), 4.16 (t, 2H), 5.79 - 5.85 (m, 4H), 6.31 (s, 1H), 7.51 (d, 1H), 7.97 (d, 1H).

### Example 874

### (3R)-2'-{6-amino-5-[(1R)-1-(1-methyl-1H-1,2,3-triazol-5-yl)ethoxy]pyridin-3-yl}-N-(propan-2-yl)-5',6'-dihydrospiro[pyrrolidine-3,4'-pyrrolo[1,2-b]pyrazole]-1-carboxamide

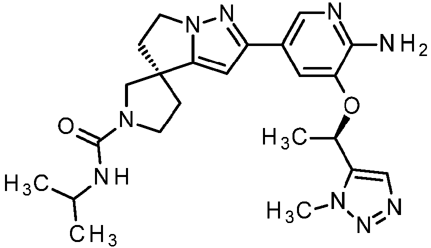

The compound was prepared similarly to **Example 873** using (3R)-1-[(propan-2-yl)carbamoyl]-5',6'-dihydrospiro[pyrrolidine-3,4'-pyrrolo[1,2-b]pyrazol]-2'-yl trifluoromethanesulfonate (104 mg, 98 % purity, 257 µmol) and 3-[(1R)-1-(1-methyl-1H-1,2,3-triazol-5-yl)ethoxy]-5-(4,4,5,5-tetramethyl-1,3,2-dioxaborolan-2-yl)pyridin-2-amine (222 mg, 40 % purity, 257 µmol) to give 47.5 mg (96 % purity, 38 % yield) of the title compound.
[α]²⁰_{D}: +82.7° (c = 1.00, methanol)
LC-MS (Method 1): Rt = 0.77 min; MS (ESlpos): m/z = 466 [M+H]⁺
¹H-NMR (400MHz, DMSO-d₆): δ [ppm]= 1.06 (d, 3H), 1.07 (d, 3H), 1.65 (d, 3H), 1.98 - 2.10 (m, 2H), 3.35 - 3.44 (m, 3H), 3.46 - 3.54 (m, 1H), 3.69 - 3.82 (m, 1H), 4.02 (s, 3H), 4.16 (t, 2H), 5.82 (d, 1H), 5.86 (s, 2H), 5.92 (q, 1H), 6.32 (s, 1H), 7.46 (d, 1H), 7.86 (s, 1H), 7.95 (d, 1H).

### Example 875

### (3R)-2'-(6-amino-5-{[1-(1-methyl-1H-1,2,3-triazol-4-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1 and diastereomer 2)

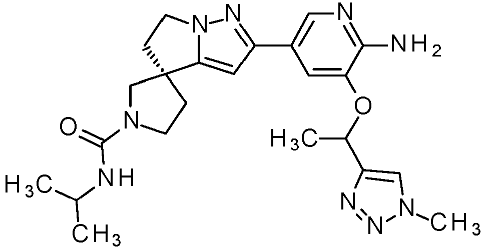

3-{[1-(1-Methyl-1H-1,2,3-triazol-4-yl)ethyl]oxy}-5-(4,4,5,5-tetramethyl-1,3,2-dioxaborolan-2-yl)pyridin-2-amine (701 mg, 24 % purity, 487 µmol) was dissolved in 1,4-dioxane (3.3 mL) under argon. (3R)-1-[(Propan-2-yl)carbamoyl]-5',6'-dihydrospiro[pyrrolidine-3,4'-pyrrolo[1,2-b]pyrazol]-2'-yl trifluoromethanesulfonate (197 mg, 98 % purity, 487 µmol), potassium phosphate (2.9 mL, 0.50 M, 1.5 mmol) and XPhos Pd G2 (19.2 mg, 24.4 µmol) were added and the mixture was stirred for 4 h at 100 °C. The reaction mixture was allowed to cool down, diluted with ethyl acetate and washed with water. The organic phase was dried over a hydrophobic filter and concentrated. The residue was purified twice by preparative HPLC to give 26.6 mg (90 % purity, 11 % yield) of the title compound.
[α]²⁰_{D}: +48.3° (c = 1.00, methanol)
LC-MS (Method 1): Rt = 0.80 min; MS (ESlpos): m/z = 466 [M+H]⁺
¹H-NMR (400 MHz, DMSO-d6) δ [ppm]: 1.053 (2.55), 1.060 (3.47), 1.069 (2.82), 1.077 (3.40), 1.233 (0.48), 1.622 (0.72), 1.629 (3.13), 1.644 (3.14), 1.664 (0.48), 2.030 (0.55), 2.043 (0.56), 2.048 (0.56), 2.336 (0.58), 2.518 (16.00), 2.523 (11.61), 2.673 (1.31), 2.678 (0.65), 3.376 (0.72), 3.395 (2.12), 3.399 (2.42), 3.566 (1.51), 3.986 (0.93), 4.007 (8.45), 4.014 (1.14), 4.019 (0.53), 4.140 (0.71), 4.158 (1.31), 4.175 (0.75), 5.654 (0.72), 5.670 (0.74), 5.727 (1.73), 5.808 (0.55), 5.828 (0.55), 6.311 (2.28), 7.456 (1.19), 7.460 (1.27), 7.905 (1.04), 7.906 (1.25), 7.909 (1.27), 7.911 (1.20), 8.116 (2.52).

### Example 876

### (5S)-5-{[(3R)-2'-{6-ami no-5-[(1R)-1-phenylethoxy]pyridin-3-yl}-5',6'-dihydrospiro[pyrrolidine-3,4'-pyrrolo[1,2-b]pyrazol]-1-yl]methyl}pyrrolidin-2-one

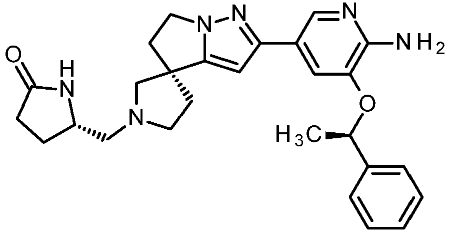

5-[(3R)-5',6'-Dihydrospiro[pyrrolidine-3,4'-pyrrolo[1,2-b]pyrazol]-2'-yl]-3-[(1R)-1-phenylethoxy]pyridin-2-amine (20.0 mg, 100 % purity, 53.3 µmol) and (5S)-5-(bromomethyl)pyrrolidin-2-one (11.4 mg, 63.9 µmol) were dissolved in THF (1.2 mL) under argon. Potassium carbonate (29.4 mg, 213 µmol) was added and the mixture was stirred for 2 h at rt. Sodium iodide (7.98 mg, 53.3 µmol) was added and the mixture was stirred over night at 70 °C. The reaction mixture was allowed to cool down, concentrated and purified by preparative HPLC to give 4.10 mg (95 % purity, 15 % yield) of the title compound.
LC-MS (Method 1): Rt = 1.00 min; MS (ESlpos): m/z = 474 [M+H]⁺
¹H-NMR (400 MHz, DMSO-d6) δ [ppm]: 1.233 (0.48), 1.561 (11.74), 1.577 (11.65), 1.686 (0.62), 1.703 (1.54), 1.720 (0.90), 1.730 (0.87), 1.734 (0.89), 1.914 (0.42), 1.935 (0.67), 1.946 (1.16), 1.963 (1.47), 1.983 (1.58), 1.999 (1.33), 2.005 (1.41), 2.019 (1.40), 2.030 (0.75), 2.037 (0.63), 2.052 (0.78), 2.061 (1.12), 2.075 (2.48), 2.088 (1.66), 2.092 (1.47), 2.097 (2.02), 2.104 (3.49), 2.111 (2.48), 2.122 (2.34), 2.132 (1.54), 2.140 (2.02), 2.165 (1.90), 2.178 (0.57), 2.190 (0.78), 2.318 (1.04), 2.323 (2.32), 2.327 (3.25), 2.331 (2.32), 2.336 (1.05), 2.365 (1.46), 2.381 (1.62), 2.395 (2.21), 2.411 (2.46), 2.428 (1.37), 2.440 (1.60), 2.460 (2.57), 2.518 (16.00), 2.523 (10.43), 2.565 (3.85), 2.579 (1.20), 2.593 (1.47), 2.598 (1.41), 2.611 (1.84), 2.624 (1.31), 2.630 (1.46), 2.652 (1.88), 2.660 (1.57), 2.665 (3.15), 2.669 (4.92), 2.673 (3.58), 2.690 (0.88), 2.728 (3.54), 2.750 (3.59), 2.765 (1.27), 2.772 (1.62), 2.787 (1.51), 2.808 (0.60), 3.601 (0.95), 3.616 (1.53), 3.632 (1.21), 3.995 (0.41), 4.014 (0.66), 4.021 (1.56), 4.038 (3.29), 4.052 (2.19), 4.058 (2.78), 4.072 (1.64), 4.085 (0.58), 4.098 (0.40), 5.556 (0.73), 5.572 (2.57), 5.588 (2.63), 5.604 (0.78), 5.814 (7.69), 6.148 (0.46), 6.244 (15.09), 7.226 (1.71), 7.234 (5.55), 7.238 (6.18), 7.244 (4.26), 7.249 (1.41), 7.259 (1.68), 7.262 (2.77), 7.265 (1.54), 7.319 (4.19), 7.338 (7.85), 7.352 (1.42), 7.357 (3.83), 7.454 (6.63), 7.472 (5.27), 7.476 (3.92), 7.641 (4.43), 7.845 (7.25), 7.850 (7.09), 8.553 (1.22).

### Example 877

### (5R)-5-{[(3R)-2'-{6-amino-5-[(1R)-1-phenylethoxy]pyridin-3-yl}-5',6'-dihydrospiro[pyrrolidine-3,4'-pyrrolo[1,2-b]pyrazol]-1-yl]methyl}pyrrolidin-2-one

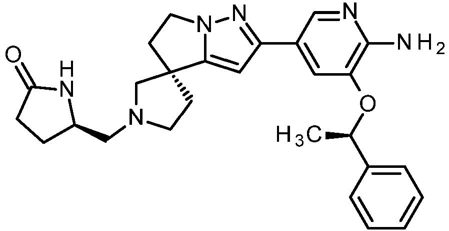

5-[(3R)-5',6'-Dihydrospiro[pyrrolidine-3,4'-pyrrolo[1,2-b]pyrazol]-2'-yl]-3-[(1R)-1-phenylethoxy]pyridin-2-amine (20.0 mg, 100 % purity, 53.3 µmol) and (5R)-5-(bromomethyl)pyrrolidin-2-one (11.4 mg, 63.9 µmol) were dissolved in THF (1.2 mL). Potassium carbonate (29.4 mg, 213 µmol) and sodium iodide (7.98 mg, 53.3 µmol) were added and the mixture was stirred over night at 70 °C.The reaction mixture was allowed to cool down and concentrated. The residue was purified by preparative HPLC to give 10 mg (95 % purity, 38 % yield) of the title compound.
LC-MS (Method 1): Rt = 1.00 min; MS (ESlpos): m/z = 474 [M+H]⁺
¹H-NMR (400 MHz, DMSO-d6) δ [ppm]: 1.232 (0.48), 1.346 (0.42), 1.562 (12.64), 1.578 (12.56), 1.706 (0.99), 1.712 (1.21), 1.720 (1.17), 1.734 (1.32), 1.748 (0.74), 1.938 (0.61), 1.950 (1.32), 1.970 (1.97), 1.988 (2.07), 1.995 (1.70), 2.010 (1.45), 2.021 (0.57), 2.027 (0.58), 2.041 (0.51), 2.067 (1.05), 2.083 (2.33), 2.099 (1.18), 2.110 (5.78), 2.126 (2.49), 2.131 (2.75), 2.137 (2.61), 2.151 (0.57), 2.162 (1.97), 2.185 (0.76), 2.387 (1.50), 2.402 (1.69), 2.417 (2.98), 2.433 (3.25), 2.450 (2.12), 2.469 (4.15), 2.518 (15.29), 2.523 (10.38), 2.572 (3.74), 2.580 (1.63), 2.594 (4.39), 2.620 (1.96), 2.636 (1.71), 2.659 (1.58), 2.740 (3.79), 2.763 (3.06), 2.784 (0.90), 2.805 (1.61), 2.821 (1.52), 2.841 (0.65), 3.608 (1.16), 3.622 (1.75), 3.638 (1.26), 4.033 (1.99), 4.039 (1.93), 4.051 (3.54), 4.067 (1.91), 4.072 (1.97), 5.557 (0.77), 5.573 (2.64), 5.589 (2.69), 5.604 (0.77), 5.813 (7.96), 6.238 (16.00), 7.226 (1.95), 7.232 (5.61), 7.236 (5.75), 7.244 (4.24), 7.249 (1.45), 7.259 (1.69), 7.262 (3.06), 7.265 (1.69), 7.318 (4.40), 7.338 (8.24), 7.351 (1.48), 7.356 (4.00), 7.454 (6.88), 7.472 (5.47), 7.476 (4.00), 7.620 (4.57), 7.842 (8.34), 7.847 (8.18), 8.552 (1.64).

### Example 878

### (3R)-2'-(6-amino-5-{1-[4-(methylsulfonyl)phenyl]ethoxy}pyridin-3-yl)-N-isopropyl-5',6'-dihydro-1H-spiro[pyrrolidine-3,4'-pyrrolo[1,2-b]pyrazole]-1-carboxamide (diastereomer 1 and diastereomer 2)

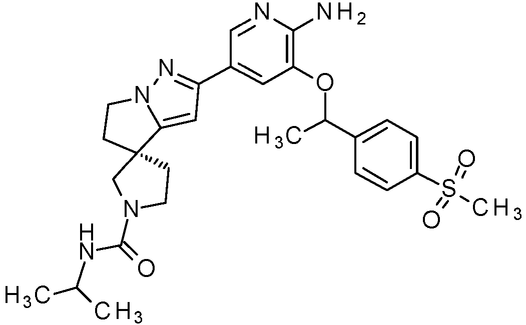

3-({1-[4-(Methanesulfonyl)phenyl]ethyl}oxy)-5-(4,4,5,5-tetramethyl-1,3,2-dioxaborolan-2-yl)pyridin-2-amine (111 mg, 265 µmol) was dissoved in degassed 1,4-dioxane (3 mL) under argon. (3R)-1-[(Propan-2-yl)carbamoyl]-5',6'-dihydrospiro[pyrrolidine-3,4'-pyrrolo[1,2-b]pyrazol]-2'-yl trifluoromethanesulfonate (110 mg, 279 µmol), potassium phosphate (1.6 mL, 0.50 M, 800 µmol) and XPhos Pd G2 (10.4 mg, 13.3 µmol) were added and the mixture was stirred for 1 h at 100 °C. The cooled down reaction mixture was diluted with water and extracted with ethyl acetate. The combined organic layers were washed with brine, dried over sodium sulfate, filtered and concentrated. The residue was purified by preparative HPLC to give 5.00 mg (95 % purity, 3 % yield) of the title compound.
LC-MS (Method 1): Rt = 0.89 min; MS (ESlpos): m/z = 540 [M+H]⁺
¹H-NMR (400 MHz, DMSO-d6) δ [ppm]: 1.045 (5.15), 1.052 (5.98), 1.056 (6.81), 1.060 (8.36), 1.068 (6.09), 1.072 (6.32), 1.076 (5.39), 1.572 (6.44), 1.588 (6.43), 1.606 (0.41), 1.987 (0.70), 1.998 (1.00), 2.016 (1.51), 2.023 (1.32), 2.035 (0.65), 2.041 (0.52), 2.464 (1.72), 2.518 (1.71), 2.523 (1.17), 3.198 (16.00), 3.371 (5.80), 3.379 (5.59), 3.398 (0.99), 3.457 (0.54), 3.473 (0.93), 3.486 (0.68), 3.498 (0.67), 3.725 (0.51), 3.730 (0.55), 3.745 (0.84), 3.761 (0.86), 3.777 (0.53), 3.782 (0.46), 4.106 (1.85), 4.124 (3.25), 4.141 (1.76), 5.748 (0.45), 5.765 (1.48), 5.781 (1.49), 5.800 (1.36), 5.805 (1.17), 5.819 (1.13), 5.825 (1.06), 5.940 (4.51), 6.257 (5.13), 6.260 (4.83), 7.275 (3.19), 7.279 (3.20), 7.754 (3.46), 7.774 (4.40), 7.864 (2.86), 7.866 (3.48), 7.868 (3.23), 7.871 (2.88), 7.895 (4.03), 7.898 (4.50), 7.916 (3.22), 7.919 (2.99).

### Example 879

### (3R)-2'-{6-amino-5-[(1R)-1-(2-methyl-1,3-thiazol-4-yl)ethoxy]pyridin-3-yl}-N-(propan-2-yl)-5',6'-dihydrospiro[pyrrolidine-3,4'-pyrrolo[1,2-b]pyrazole]-1-carboxamide

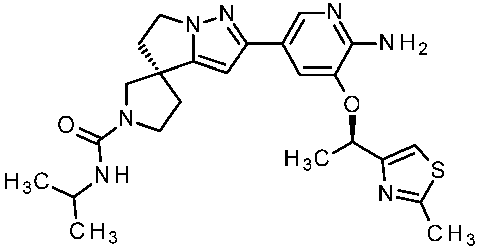

The compound was prepared similarly to **Example 878** using (3R)-1-[(propan-2-yl)carbamoyl]-5',6'-dihydrospiro[pyrrolidine-3,4'-pyrrolo[1,2-b]pyrazol]-2'-yl trifluoromethanesulfonate (83.3 mg, 98 % purity, 206 µmol) and 3-[(1R)-1-(2-methyl-1,3-thiazol-4-yl)ethoxy]-5-(4,4,5,5-tetramethyl-1,3,2-dioxaborolan-2-yl)pyridin-2-amine (186 mg, 40 % purity, 206 µmol) to give 26.9 mg (96 % purity, 26 % yield) of the title compound.
[α]²⁰_{D}: +119,17° (c = 1.00, methanol)
LC-MS (Method 1): Rt = 0.90 min; MS (ESlneg): m/z = 480 [M-H]⁻
¹H-NMR (400 MHz, DMSO-d6) δ [ppm]: 1.052 (6.31), 1.060 (6.73), 1.068 (6.70), 1.076 (6.30), 1.605 (5.51), 1.621 (5.52), 1.893 (3.12), 2.012 (0.68), 2.026 (1.08), 2.039 (1.41), 2.057 (0.60), 2.323 (0.73), 2.327 (1.00), 2.332 (0.75), 2.518 (7.20), 2.523 (4.32), 2.647 (16.00), 2.660 (0.55), 2.665 (0.84), 2.669 (1.08), 2.673 (0.77), 3.375 (1.22), 3.392 (5.95), 3.419 (0.51), 3.468 (0.43), 3.486 (0.71), 3.494 (0.47), 3.501 (0.53), 3.512 (0.45), 3.730 (0.50), 3.746 (0.73), 3.765 (0.75), 3.782 (0.49), 4.130 (1.37), 4.147 (2.49), 4.164 (1.33), 5.568 (1.24), 5.584 (1.23), 5.784 (3.50), 5.806 (1.40), 5.825 (1.26), 6.297 (5.37), 7.375 (2.27), 7.380 (2.27), 7.467 (4.54), 7.915 (3.05), 7.919 (3.00).

### Example 880

### (3R)-2'-[6-amino-5-({1-[3-(methanesulfonyl)phenyl]ethyl}oxy)pyridin-3-yl]-N-(propan-2-yl)-5',6'-dihydrospiro[pyrrolidine-3,4'-pyrrolo[1,2-b]pyrazole]-1-carboxamide (diastereomer 1 and diastereomer 2)

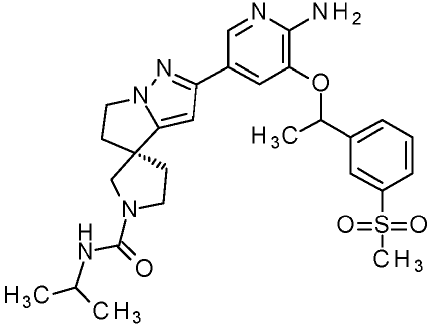

The compound was prepared similarly to **Example 878** using 3-({1-[3-(methanesulfonyl)phenyl]ethyl}oxy)-5-(4,4,5,5-tetramethyl-1,3,2-dioxaborolan-2-yl)pyridin-2-amine (555 mg, 1.33 mmol) and (3R)-1-[(propan-2-yl)carbamoyl]-5',6'-dihydrospiro[pyrrolidine-3,4'-pyrrolo[1,2-b]pyrazol]-2'-yl trifluoromethanesulfonate (552 mg, 1.39 mmol) to give 136 mg (98 % purity, 19 % yield) of the title compound.
LC-MS (Method 1): Rt = 0.90 min; MS (ESlpos): m/z = 540 [M+H]⁺
¹H-NMR (400 MHz, DMSO-d6) δ [ppm]: 1.047 (6.20), 1.052 (7.25), 1.060 (9.84), 1.069 (7.43), 1.075 (8.10), 1.595 (7.51), 1.610 (7.48), 2.008 (1.74), 2.017 (2.28), 2.035 (1.03), 2.465 (2.09), 2.518 (2.12), 2.523 (1.36), 3.210 (16.00), 3.212 (15.12), 3.371 (6.46), 3.376 (5.52), 3.388 (1.98), 3.403 (1.19), 3.457 (0.69), 3.474 (1.18), 3.488 (0.86), 3.499 (0.79), 3.514 (0.43), 3.730 (0.70), 3.747 (1.11), 3.763 (1.08), 3.779 (0.64), 4.098 (2.04), 4.115 (3.81), 4.133 (2.03), 5.738 (0.49), 5.754 (1.69), 5.770 (1.72), 5.786 (0.68), 5.803 (1.56), 5.822 (1.47), 5.919 (4.88), 6.254 (5.21), 6.259 (4.75), 7.296 (3.42), 7.300 (3.51), 7.619 (1.59), 7.638 (3.51), 7.657 (2.16), 7.814 (2.22), 7.818 (1.91), 7.835 (1.94), 7.838 (1.94), 7.844 (2.44), 7.864 (6.10), 7.868 (5.36), 8.094 (2.66), 8.100 (2.51).

### Example 881

### (3R)-2'-(6-amino-5-{[1-(4-methyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)

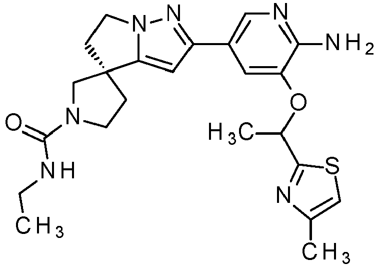

3-{[1-(4-Methyl-1,3-thiazol-2-yl)ethyl]oxy}-5-(4,4,5,5-tetramethyl-1,3,2-dioxaborolan-2-yl)pyridin-2-amine (198 mg, 44 % purity, 241 µmol) was dissolved in 1,4-dioxane (1.4 mL) under argon. (3R)-1-(Ethylcarbamoyl)-5',6'-dihydrospiro[pyrrolidine-3,4'-pyrrolo[1,2-b]pyrazol]-2'-yl trifluoromethanesulfonate (76.8 mg, 201 µmol), potassium phosphate (1.2 mL, 0.50 M, 600 µmol) and XPhos Pd G2 (15.8 mg, 20.1 µmol) were added and the mixture was stirre for 1 h at 100 °C. The reaction mixture was allowed to cool down, diluted with dichloromethane and washed with water and brine. The organic phase was dried over a hydrophobic filter and concentrated. The residue was purified by preparative HPLC to give 29.0 mg (93 % purity, 29 % yield) of the title compound.
LC-MS (Method 1): Rt = 0.89 min; MS (ESlpos): m/z = 468 [M+H]⁺
¹H-NMR (400 MHz, DMSO-d6) δ [ppm]: 0.999 (6.45), 1.017 (15.13), 1.035 (6.76), 1.677 (9.47), 1.693 (9.38), 2.030 (1.64), 2.037 (2.04), 2.055 (0.92), 2.332 (1.18), 2.337 (0.73), 2.347 (10.27), 2.350 (16.00), 2.353 (9.88), 2.518 (7.89), 2.523 (4.83), 2.673 (1.05), 2.679 (0.48), 3.016 (0.78), 3.034 (2.48), 3.048 (2.80), 3.051 (2.70), 3.065 (2.48), 3.083 (0.72), 3.357 (0.83), 3.383 (8.50), 3.399 (1.81), 3.418 (0.69), 3.447 (0.65), 3.465 (1.20), 3.473 (0.67), 3.480 (0.82), 3.490 (0.70), 4.125 (2.32), 4.143 (3.75), 4.160 (2.23), 5.793 (0.77), 5.808 (3.07), 5.821 (6.16), 5.840 (0.91), 6.133 (1.03), 6.147 (2.02), 6.160 (1.00), 6.275 (10.12), 7.233 (3.87), 7.235 (4.63), 7.237 (3.72), 7.405 (3.59), 7.938 (4.78), 7.943 (4.59).

### Example 882

### (3R)-2'-(6-amino-5-{[1-(4,5-dimethyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)

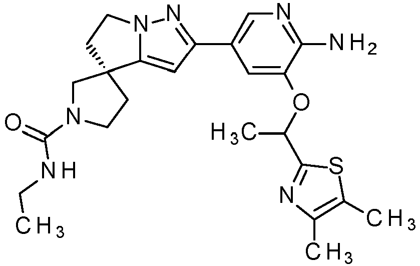

3-{[1-(4,5-Dimethyl-1,3-thiazol-2-yl)ethyl]oxy}-5-(4,4,5,5-tetramethyl-1,3,2-dioxaborolan-2-yl)pyridin-2-amine (350 mg, 39 % purity, 364 µmol) was dissolved in 1,4-dioxane (2.1 mL) under argon. (3R)-1-(Ethylcarbamoyl)-5',6'-dihydrospiro[pyrrolidine-3,4'-pyrrolo[1,2-b]pyrazol]-2'-yl trifluoromethanesulfonate (116 mg, 303 µmol), potassium phosphate (1.8 mL, 0.50 M, 910 µmol) and XPhos Pd G2 (23.8 mg, 30.3 µmol) were added and the mixture was stirred for 1.5 h at 100 °C. The reaction mixture was allowed to cool down, diluted with dichloromethane and washed with water and brine. The organic phase was dried over a hydrophobic filter and concentrated. The residue was purified by preparative HPLC to give 85.0 mg (100 % purity, 58 % yield) of the title compound.
LC-MS (Method 2): Rt = 0.79 min; MS (ESlpos): m/z = 482 [M+H]⁺
¹H-NMR (400 MHz, DMSO-d6) δ [ppm]: 1.000 (6.67), 1.017 (16.00), 1.035 (7.20), 1.651 (9.58), 1.667 (9.62), 2.030 (1.56), 2.037 (1.88), 2.055 (0.86), 2.226 (9.47), 2.228 (12.46), 2.231 (9.57), 2.278 (14.06), 2.332 (0.69), 2.518 (5.58), 2.523 (2.79), 2.673 (0.69), 3.017 (0.77), 3.034 (2.46), 3.048 (2.70), 3.052 (2.64), 3.066 (2.42), 3.084 (0.72), 3.358 (0.84), 3.384 (8.33), 3.401 (1.78), 3.419 (0.71), 3.448 (0.68), 3.466 (1.23), 3.473 (0.67), 3.480 (0.83), 3.491 (0.71), 4.126 (2.32), 4.144 (3.68), 4.161 (2.25), 5.704 (0.57), 5.720 (2.10), 5.736 (2.11), 5.752 (0.60), 5.797 (5.39), 6.134 (1.02), 6.148 (2.08), 6.162 (1.02), 6.268 (11.18), 7.396 (3.76), 7.925 (5.27), 7.930 (5.20), 8.138 (0.71).

### Example 883

### (3R)-2'-(6-amino-5-{[1-(5-methyl-1 , 3-thiazol-2-yl)ethyl]oxy}pyridi n-3-yl)- N-ethyl-5', 6'-dihydrospiro[pyrrolidine-3,4'-pyrrolo[1,2-b]pyrazole]-1-carboxamide (diastereomer 1 and diastereomer 2)

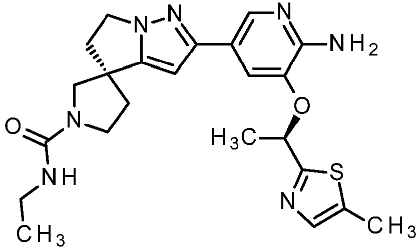

The compound was prepared similarly to **Example 882** using (3R)-1-(ethylcarbamoyl)-5',6'-dihydrospiro[pyrrolidine-3,4'-pyrrolo[1,2-b]pyrazol]-2'-yl trifluoromethanesulfonate (111 mg, 290 µmol) and 3-{[1-(5-methyl-1,3-thiazol-2-yl)ethyl]oxy}-5-(4,4,5,5-tetramethyl-1,3,2-dioxaborolan-2-yl)pyridin-2-amine (331 mg, 38 % purity, 348 µmol) to give 37.0 mg (100 % purity, 27 % yield) of the title compound.
LC-MS (Method 1): Rt = 0.89 min; MS (ESlpos): m/z = 468 [M+H]⁺
¹H-NMR (400 MHz, DMSO-d6) δ [ppm]: 0.998 (5.08), 1.016 (11.57), 1.018 (10.49), 1.034 (5.22), 1.036 (4.88), 1.672 (10.46), 1.688 (10.59), 2.017 (1.09), 2.028 (1.63), 2.035 (1.70), 2.043 (1.79), 2.054 (0.78), 2.061 (0.65), 2.077 (0.45), 2.392 (16.00), 2.480 (3.43), 2.518 (4.63), 2.523 (2.83), 3.016 (0.64), 3.033 (2.16), 3.050 (2.84), 3.065 (2.03), 3.067 (2.03), 3.083 (0.63), 3.351 (0.87), 3.370 (1.48), 3.382 (5.45), 3.385 (6.25), 3.394 (2.12), 3.413 (0.77), 3.450 (0.69), 3.468 (1.18), 3.475 (0.71), 3.483 (0.86), 3.494 (0.69), 3.509 (0.41), 4.126 (2.40), 4.144 (3.97), 4.161 (2.36), 5.755 (0.69), 5.772 (2.53), 5.787 (2.70), 5.812 (5.77), 6.134 (1.07), 6.148 (2.21), 6.162 (1.08), 6.275 (7.83), 7.401 (3.96), 7.438 (3.91), 7.440 (4.53), 7.443 (4.25), 7.923 (4.37), 7.927 (3.98), 8.133 (0.73).

### Example 884

### (3R)-2'-{6-amino-5-[(1R)-1-(pyridin-4-yl)ethoxy]pyridin-3-yl}-N-(propan-2-yl)-5',6'-dihydrospiro[pyrrolidine-3,4'-pyrrolo[1,2-b]pyrazole]-1-carboxamide

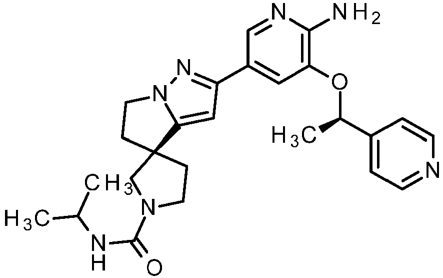

5-[(3R)-5',6'-Dihydrospiro[pyrrolidine-3,4'-pyrrolo[1,2-b]pyrazol]-2'-yl]-3-[(1R)-1-(pyridin-4-yl)ethoxy]pyridin-2-amine hydrogen chloride (1/1) (40.0 mg, 58 % purity, 56.2 µmol, **Intermediate 532**) was suspended in dichloromethane (0.5 mL). N,N-Diisopropylethylamine (98 µL, 560 µmol) was added and the mixture was cooled to 0 °C. 2-Isocyanatopropane (6.6 µL, 67 µmol) was added and the mixture was allowed to warm up to rt and stirred overnight. The reaction mixture was concentrated. The residue was purified by preparative TLC (dichloromethane / methanol 9:1 gradient) to give 10.0 mg (93 % purity, 36 % yield) of the title compound.
LC-MS (Method 1): Rt = 0.86 min; MS (ESlpos): m/z = 462 [M+H]⁺
¹H-NMR (400 MHz, DMSO-d6) δ [ppm]: 0.834 (0.41), 0.852 (1.02), 0.868 (1.31), 0.886 (1.07), 0.906 (1.88), 0.924 (0.92), 1.045 (14.05), 1.057 (16.00), 1.062 (15.93), 1.073 (14.43), 1.233 (4.79), 1.256 (2.61), 1.332 (0.42), 1.353 (0.49), 1.393 (0.43), 1.410 (0.43), 1.555 (12.06), 1.571 (11.99), 1.907 (0.60), 1.995 (1.56), 2.008 (2.34), 2.022 (3.12), 2.040 (1.32), 2.052 (0.63), 2.112 (0.59), 2.466 (4.45), 2.518 (12.12), 2.523 (8.41), 2.678 (0.97), 3.371 (12.06), 3.388 (2.45), 3.406 (0.96), 3.454 (0.88), 3.471 (1.55), 3.479 (1.01), 3.486 (1.14), 3.493 (0.93), 3.511 (0.55), 3.725 (1.12), 3.742 (1.64), 3.761 (1.68), 3.777 (1.12), 3.940 (0.46), 4.104 (3.04), 4.122 (5.48), 4.139 (2.94), 4.228 (0.44), 4.234 (0.48), 4.242 (0.46), 4.248 (0.42), 5.632 (0.73), 5.647 (2.51), 5.663 (2.52), 5.679 (0.71), 5.760 (11.88), 5.800 (2.74), 5.819 (2.68), 5.929 (7.41), 6.247 (12.76), 7.236 (5.10), 7.240 (4.94), 7.476 (7.88), 7.479 (4.69), 7.487 (4.91), 7.491 (7.94), 7.875 (7.51), 7.879 (7.28), 8.089 (3.12), 8.533 (10.08), 8.537 (5.64), 8.544 (5.67), 8.548 (9.01).

### Example 885

### (3R)-2'-{6-amino-5-[(1R)-1-(pyridin-4-yl)ethoxy]pyridin-3-yl}-N-cyclobutyl-5',6'-dihydrospiro[pyrrolidine-3,4'-pyrrolo[1,2-b]pyrazole]-1-carboxamide

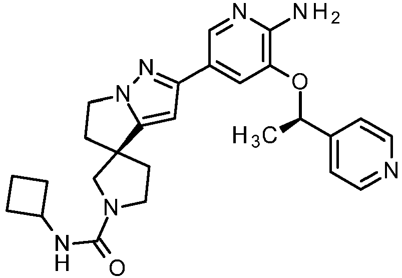

The compound was prepared similarly to **Example 884** using 5-[(3R)-5',6'-dihydrospiro[pyrrolidine-3,4'-pyrrolo[1,2-b]pyrazol]-2'-yl]-3-[(1R)-1-(pyridin-4-yl)ethoxy]pyridin-2-amine hydrogen chloride (1/1) (40.0 mg, 58 % purity, 56.2 µmol, **Intermediate 532)** and isocyanatocyclobutane (6.55 mg, 67.4 µmol) to give 7.70 mg (100 % purity, 29 % yield) of the title compound.
LC-MS (Method 1): Rt = 0.87 min; MS (ESlpos): m/z = 474 [M+H]⁺
¹H-NMR (400 MHz, DMSO-d6) δ [ppm]: 0.833 (0.65), 0.851 (1.80), 0.868 (3.50), 0.887 (2.94), 0.906 (5.32), 0.924 (2.45), 1.232 (4.06), 1.256 (1.11), 1.279 (1.47), 1.289 (1.65), 1.296 (1.76), 1.317 (1.34), 1.339 (0.88), 1.353 (1.09), 1.374 (0.84), 1.392 (1.08), 1.411 (1.20), 1.431 (0.83), 1.450 (0.40), 1.484 (0.78), 1.503 (1.43), 1.510 (1.20), 1.522 (1.31), 1.529 (2.73), 1.553 (14.67), 1.570 (13.48), 1.684 (0.49), 1.700 (0.57), 1.715 (0.43), 1.885 (1.20), 1.912 (2.13), 1.933 (2.20), 1.960 (1.32), 1.995 (1.71), 2.009 (2.47), 2.020 (3.15), 2.038 (1.41), 2.051 (0.82), 2.069 (1.31), 2.078 (2.06), 2.085 (2.63), 2.095 (2.09), 2.104 (2.33), 2.112 (1.84), 2.123 (0.85), 2.318 (1.24), 2.463 (4.76), 2.518 (16.00), 2.523 (11.07), 3.367 (9.94), 3.383 (1.54), 3.391 (2.67), 3.409 (1.01), 3.446 (0.95), 3.464 (1.74), 3.472 (1.08), 3.479 (1.26), 3.490 (1.05), 3.504 (1.06), 4.079 (0.91), 4.102 (4.48), 4.120 (6.98), 4.137 (3.68), 4.228 (1.34), 4.234 (1.43), 4.242 (1.43), 4.248 (1.32), 5.635 (0.76), 5.651 (2.67), 5.667 (2.70), 5.682 (0.77), 5.760 (14.90), 5.927 (8.11), 6.251 (14.59), 6.293 (2.86), 6.312 (2.84), 7.240 (5.32), 7.243 (5.40), 7.476 (8.40), 7.479 (5.10), 7.487 (5.24), 7.491 (8.72), 7.879 (7.75), 7.883 (7.62), 8.089 (9.47), 8.533 (10.46), 8.537 (6.00), 8.544 (6.00), 8.548 (10.36).

### Example 886

### 6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 1 and diastereomer 2)

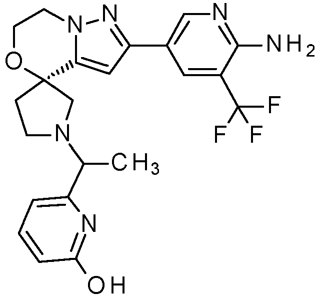

5-{(3'R)-1'-[1-(6-Methoxypyridin-2-yl)ethyl]-6,7-dihydrospiro[pyrazolo[5,1-c][1,4]oxazine-4,3'-pyrrolidin]-2-yl}-3-(trifluoromethyl)pyridin-2-amine (27.3 mg, 57.5 µmol, **Example 855**) was dissolved in acetonitrile (0.4 mL). Sodium iodide (43.1 mg, 288 µmol) was added and chlorotrimethylsilane (37 µL, 290 µmol) was added dropwise. The mixture was stirred over night at room temperature. Sodium iodide (43.1 mg, 288 µmol) and chlorotrimethylsilane (37 µL, 290 µmol) were added again and stirred 2 h at 50 °C and 3 h at 60 °C. The reaction mixture was concentrated. The residue was diluted with ethyl acteate and washed with brine. The organic phase was dried over sodium sulfate, filtered and concentrated. The crude product was purified by preparative TLC (dichloromethane / methanol 9:1 gradient) to give 17.1 mg (95 % purity, 61 % yield) of the title compound.
LC-MS (Method 1): Rt = 0.93 min; MS (ESlpos): m/z = 462 [M+H]⁺
¹H-NMR (400 MHz, CHLOROFORM-d) δ [ppm]: 1.260 (1.03), 1.406 (3.48), 1.417 (5.65), 1.422 (4.32), 1.434 (5.10), 1.512 (0.45), 1.517 (0.49), 1.535 (0.55), 1.634 (1.40), 2.101 (0.60), 2.224 (0.80), 2.243 (0.87), 2.258 (1.31), 2.277 (1.14), 2.296 (0.41), 2.356 (0.60), 2.372 (0.85), 2.387 (0.79), 2.403 (0.55), 2.806 (0.40), 2.831 (0.45), 2.844 (0.44), 2.854 (0.55), 2.867 (0.56), 2.913 (0.47), 2.931 (1.02), 2.950 (1.89), 2.976 (1.92), 3.004 (0.85), 3.016 (1.56), 3.096 (1.15), 3.123 (0.82), 3.319 (0.64), 3.455 (0.79), 3.471 (1.29), 3.487 (0.93), 4.078 (0.56), 4.093 (1.14), 4.096 (1.14), 4.110 (2.30), 4.119 (2.29), 4.130 (1.44), 4.152 (0.45), 4.159 (0.47), 4.176 (1.48), 4.179 (1.47), 4.187 (3.50), 4.198 (3.39), 4.209 (1.01), 5.057 (3.91), 5.310 (16.00), 6.073 (1.40), 6.089 (1.57), 6.339 (3.29), 6.349 (1.99), 6.448 (1.44), 6.464 (1.22), 6.470 (1.70), 6.486 (1.18), 7.007 (0.45), 7.332 (1.33), 7.338 (0.76), 7.349 (1.40), 7.355 (1.88), 7.361 (0.83), 7.372 (1.28), 7.378 (0.67), 7.529 (0.47), 8.121 (1.89), 8.127 (3.02), 8.133 (1.72), 8.629 (1.73), 8.635 (2.08).

### Example 887

### 6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 1)

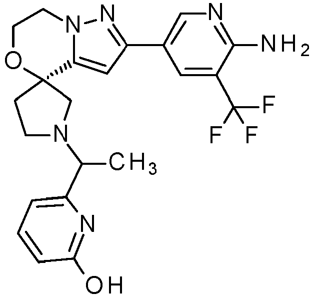

The compound 6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 1 and diastereomer 2, **Example 886**) was separated into diastereomers by preparative chiral HPLC to give 3.50 mg (90 % purity, 21 % yield) of the title compound (diastereomer 1 Rt = 11.25 - 13.73 min).

### Preparative chiral HPLC method:

Instrument: PrepCon Labomatic HPLC-2; Column: YMC Cellulose SB 5µ, 250x50; eluent A: hexane + 0.1 vol % diethylamine; eluent B: 2-propanol; isocratic: 30%B; flow: 50 mL/min; temperature: 25 °C; UV: 280 nm

### Analytical chiral HPLC method:

Instrument: Thermo Fisher UltiMate 3000; Column: YMC Cellulose SB 3µ, 100x4.6; eluent A: hexane + 0.1 vol % diethylamine; eluent B: 2-propanol; isocratic: 30%B; flow: 1.4 mL/min; temperature: 25 °C; UV: 280 nm
Analytical chiral HPLC Rₜ = 2.21 min
[α]²⁰_{D}: +32.6° (c = 1.00, methanol)
LC-MS (Method 1): Rt = 0.93 min; MS (ESlpos): m/z = 462 [M+H]⁺
1H-NMR (400 MHz, CHLOROFORM-d) δ [ppm]: 0.764 (0.62), 0.776 (0.74), 0.783 (1.10), 0.795 (2.65), 0.800 (1.24), 0.810 (2.88), 0.814 (4.85), 0.828 (2.56), 0.833 (2.53), 0.838 (1.26), 0.845 (2.12), 0.856 (1.86), 0.859 (0.74), 0.864 (0.92), 0.874 (0.96), 0.883 (0.47), 0.923 (0.41), 0.971 (0.48), 1.066 (0.74), 1.076 (3.31), 1.095 (0.78), 1.100 (3.26), 1.114 (0.52), 1.116 (0.53), 1.120 (0.45), 1.126 (0.63), 1.129 (0.67), 1.135 (1.93), 1.142 (1.69), 1.151 (8.86), 1.168 (1.93), 1.184 (10.20), 1.215 (1.83), 1.230 (1.37), 1.253 (1.32), 1.264 (1.10), 1.284 (0.73), 1.294 (2.05), 1.306 (0.63), 1.313 (0.91), 1.328 (13.22), 1.333 (12.88), 1.345 (12.75), 1.361 (0.64), 1.383 (0.54), 1.403 (0.84), 1.416 (0.79), 1.422 (0.82), 1.435 (0.76), 1.445 (0.84), 1.451 (0.67), 1.455 (0.88), 1.465 (0.59), 1.470 (0.60), 1.473 (0.96), 1.492 (1.90), 1.502 (0.69), 1.511 (1.65), 1.520 (0.88), 1.531 (1.76), 1.541 (1.28), 1.545 (1.00), 1.552 (0.99), 1.557 (1.06), 1.563 (0.89), 1.569 (0.87), 1.574 (0.92), 1.582 (0.89), 1.589 (0.81), 1.611 (1.01), 1.623 (0.77), 1.630 (0.86), 1.639 (1.12), 1.649 (1.06), 1.656 (1.24), 1.668 (1.14), 1.676 (0.93), 1.699 (0.67), 1.778 (0.63), 1.794 (0.60), 1.814 (0.70), 1.818 (0.64), 1.822 (0.60), 1.825 (0.60), 1.832 (0.73), 1.848 (0.75), 1.863 (0.60), 1.878 (0.45), 2.111 (0.78), 2.130 (1.59), 2.145 (1.30), 2.150 (1.07), 2.164 (2.35), 2.183 (1.17), 2.275 (1.08), 2.292 (1.26), 2.306 (1.20), 2.322 (0.78), 2.338 (0.62), 2.484 (0.45), 2.728 (0.74), 2.740 (0.88), 2.750 (1.49), 2.763 (1.57), 2.770 (1.19), 2.783 (0.95), 2.825 (1.04), 2.843 (2.33), 2.858 (3.99), 2.865 (1.79), 2.884 (5.40), 3.011 (3.42), 3.037 (2.46), 3.349 (0.80), 3.366 (2.67), 3.382 (2.59), 3.399 (0.75), 3.984 (0.45), 3.995 (0.44), 3.999 (0.50), 4.014 (1.47), 4.026 (3.42), 4.038 (4.98), 4.049 (2.74), 4.068 (0.52), 4.079 (0.74), 4.099 (0.80), 4.107 (4.27), 4.120 (6.44), 4.131 (1.94), 4.134 (1.91), 5.001 (6.15), 5.979 (3.33), 5.995 (3.45), 6.245 (16.00), 6.363 (3.82), 6.365 (3.76), 6.386 (4.01), 6.388 (3.70), 7.248 (3.85), 7.264 (3.99), 7.271 (3.76), 7.287 (3.72), 8.044 (3.90), 8.048 (3.98), 8.545 (3.71), 8.549 (3.68).

### Example 888

### 6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 2)

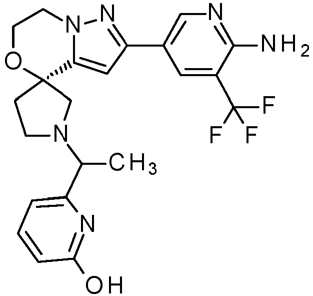

For the preparation of the diastereomeric mixture of the title compound see **Example 886.The** diastereomers were separated by preparative chiral HPLC (method see **Example 887**) to give 3.30 mg (90 % purity, 20 % yield) of the title compound (diastereomer 2 Rt = 13.73 - 17.01 min).
Analytical chiral HPLC (method see **Example 887**): Rt = 2.52 min
[α]²⁰_{D}: +74.6° (c = 1.00, methanol)
LC-MS (Method 1): Rt = 0.93 min; MS (ESlpos): m/z = 462 [M+H]⁺
¹H-NMR (400 MHz, CHLOROFORM-d) δ [ppm]: 0.764 (0.80), 0.783 (1.57), 0.794 (2.99), 0.800 (1.70), 0.811 (4.09), 0.813 (4.79), 0.826 (2.48), 0.828 (2.91), 0.832 (2.53), 0.838 (1.21), 0.845 (2.16), 0.857 (1.76), 0.861 (0.92), 0.864 (0.93), 0.875 (0.99), 0.883 (0.57), 0.971 (0.43), 0.997 (0.41), 1.050 (0.52), 1.066 (1.17), 1.075 (2.98), 1.095 (1.26), 1.099 (2.99), 1.114 (0.79), 1.126 (1.04), 1.129 (0.99), 1.135 (4.21), 1.142 (2.90), 1.150 (8.00), 1.157 (1.89), 1.166 (2.44), 1.185 (15.60), 1.255 (1.52), 1.294 (3.44), 1.323 (14.32), 1.331 (10.59), 1.340 (14.80), 1.363 (2.42), 1.385 (0.60), 1.404 (0.90), 1.409 (0.64), 1.422 (0.90), 1.435 (0.80), 1.443 (0.91), 1.454 (0.88), 1.464 (0.70), 1.472 (0.97), 1.491 (1.76), 1.510 (1.60), 1.530 (2.18), 1.541 (1.58), 1.558 (1.33), 1.574 (1.22), 1.581 (1.17), 1.610 (1.35), 1.638 (1.27), 1.649 (1.25), 1.656 (1.39), 1.668 (1.31), 1.675 (1.14), 1.767 (0.81), 1.782 (0.71), 1.809 (0.74), 1.827 (0.73), 1.843 (0.72), 1.858 (0.60), 1.936 (0.57), 2.085 (0.41), 2.131 (0.81), 2.150 (1.66), 2.166 (1.51), 2.169 (1.19), 2.185 (2.67), 2.204 (1.59), 2.243 (0.41), 2.266 (1.23), 2.278 (1.48), 2.283 (1.59), 2.296 (1.48), 2.312 (0.93), 2.317 (0.89), 2.329 (0.74), 2.680 (0.85), 2.692 (1.03), 2.702 (1.57), 2.714 (1.65), 2.722 (1.23), 2.735 (0.98), 2.826 (1.18), 2.844 (2.63), 2.863 (1.51), 2.867 (2.08), 2.885 (0.88), 2.894 (1.96), 2.921 (7.32), 2.935 (5.90), 2.961 (1.39), 3.320 (0.90), 3.337 (2.93), 3.354 (2.86), 3.370 (0.85), 3.574 (0.47), 3.967 (0.52), 3.978 (0.85), 3.981 (0.78), 3.992 (1.07), 3.997 (1.58), 4.011 (2.86), 4.021 (2.56), 4.031 (4.11), 4.043 (2.43), 4.061 (0.87), 4.073 (1.09), 4.080 (0.43), 4.094 (0.99), 4.100 (3.70), 4.112 (5.43), 4.124 (1.99), 4.127 (1.98), 5.006 (6.65), 5.980 (3.64), 5.996 (3.76), 6.255 (16.00), 6.375 (3.96), 6.377 (4.07), 6.398 (4.41), 6.401 (4.04), 7.252 (4.41), 7.268 (4.25), 7.275 (4.24), 7.292 (3.85), 8.049 (4.26), 8.054 (4.34), 8.555 (4.05), 8.558 (4.05).

### Example 889

### 6-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}(cyclopropyl)methyl)pyridin-2-ol (diastereomer 1 and diastereomer 2)

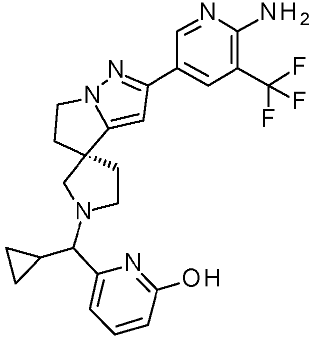

5-{(3R)-1-[Cyclopropyl(6-methoxypyridin-2-yl)methyl]-5',6'-dihydrospiro[pyrrolidine-3,4'-pyrrolo[1,2-b]pyrazol]-2'-yl}-3-(trifluoromethyl)pyridin-2-amine (17.6 mg, 36.3 µmol, **Example 856**) was dissolved in acetonitrile (1.2 mL). Sodium iodide (27.2 mg, 182 µmol) was added and chlorotrimethylsilane (23 µL, 180 µmol) was added dropwise. The mixture was stirred for 2 h at 60 °C. The reaction mixture was concentrated and diluted with ethyl acetate. The organic phase was washed with brine, dried over sodium sulfate, filtered and concentrated. The residue was purified by preparative TLC (dichloromethane / methanol 9:1 gradient) to give 4.80 mg (95 % purity, 27 % yield) of the title compound.
LC-MS (Method 1): Rt = 1.05 min; MS (ESlpos): m/z = 472 [M+H]⁺
¹H-NMR (400 MHz, CHLOROFORM-d) δ [ppm]: 0.181 (1.96), 0.192 (2.52), 0.204 (2.62), 0.218 (1.90), 0.441 (0.59), 0.453 (1.39), 0.467 (2.08), 0.479 (2.05), 0.489 (2.64), 0.501 (2.97), 0.512 (4.29), 0.525 (3.35), 0.534 (2.58), 0.546 (1.92), 0.557 (1.07), 0.570 (0.48), 0.750 (0.71), 0.761 (0.89), 0.769 (1.35), 0.785 (1.82), 0.792 (1.82), 0.796 (1.73), 0.807 (1.87), 0.817 (1.51), 0.823 (1.26), 0.838 (1.08), 0.858 (0.55), 0.885 (0.67), 0.974 (0.41), 0.986 (0.79), 0.994 (1.04), 1.006 (1.86), 1.018 (1.78), 1.029 (2.19), 1.038 (1.65), 1.049 (1.50), 1.061 (0.82), 1.259 (3.41), 1.393 (12.23), 2.026 (0.79), 2.046 (1.36), 2.059 (2.01), 2.078 (2.86), 2.096 (2.15), 2.109 (1.58), 2.129 (1.03), 2.202 (1.41), 2.207 (1.38), 2.214 (1.82), 2.224 (2.29), 2.236 (2.71), 2.247 (1.53), 2.257 (1.60), 2.278 (4.87), 2.288 (3.30), 2.301 (4.59), 2.311 (3.04), 2.584 (1.04), 2.601 (1.90), 2.633 (4.65), 2.654 (3.54), 2.660 (2.56), 2.675 (2.46), 2.681 (2.01), 2.694 (2.11), 2.708 (4.66), 2.721 (1.93), 2.733 (6.79), 2.753 (3.08), 2.760 (3.93), 2.772 (1.29), 2.785 (1.45), 2.803 (1.39), 2.824 (1.39), 2.844 (0.82), 2.857 (3.20), 2.880 (2.61), 2.897 (1.79), 2.909 (1.62), 2.919 (1.06), 2.930 (0.83), 3.019 (0.74), 3.031 (0.92), 3.040 (1.23), 3.055 (1.37), 3.074 (0.60), 3.156 (3.40), 3.179 (3.07), 4.126 (0.59), 4.145 (0.83), 4.153 (1.73), 4.161 (0.88), 4.169 (2.77), 4.172 (2.44), 4.181 (1.97), 4.189 (2.88), 4.197 (3.23), 4.201 (2.46), 4.214 (4.61), 4.223 (2.92), 4.236 (3.10), 4.243 (2.67), 4.250 (1.18), 4.257 (2.15), 4.263 (0.95), 4.271 (0.86), 4.284 (0.72), 5.032 (12.87), 5.308 (6.27), 5.993 (3.08), 6.002 (4.11), 6.009 (3.42), 6.017 (3.99), 6.244 (16.00), 6.463 (6.12), 6.486 (6.40), 7.321 (2.50), 7.333 (4.06), 7.337 (2.90), 7.343 (2.73), 7.350 (4.02), 7.356 (4.04), 7.361 (2.65), 7.373 (3.49), 8.124 (8.23), 8.129 (5.54), 8.627 (8.26), 8.631 (8.23), 9.658 (1.42).

### Example 890

### 6-({(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}(cyclopropyl)methyl)pyridin-2-ol (diastereomer 1 and diastereomer 2)

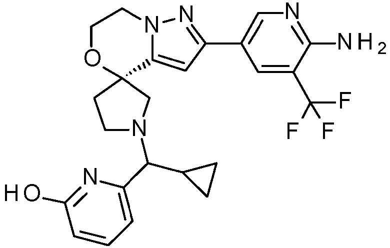

The compound was prepared similarly to **Example 878** using 5-{(3'R)-1'-[cyclopropyl(6-methoxypyridin-2-yl)methyl]-6,7-dihydrospiro[pyrazolo[5,1-c][1,4]oxazine-4,3'-pyrrolidin]-2-yl}-3-(trifluoromethyl)pyridin-2-amine (26.4 mg, 52.7 µmol, **Example 857**) to give 5.20 mg (95 % purity, 19 % yield) of the title compound.
LC-MS (Method 1): Rt = 1.00 min; MS (ESlpos): m/z = 488 [M+H]⁺
¹H-NMR (400 MHz, CHLOROFORM-d) δ [ppm]: 0.204 (3.40), 0.215 (2.80), 0.226 (1.90), 0.493 (2.69), 0.504 (5.68), 0.517 (6.66), 0.528 (6.42), 0.563 (0.91), 0.786 (1.72), 0.804 (3.48), 0.826 (2.62), 0.848 (0.86), 0.885 (0.68), 1.057 (2.65), 1.068 (2.29), 1.079 (2.10), 1.225 (0.50), 1.258 (3.45), 1.783 (6.13), 2.190 (1.00), 2.208 (2.18), 2.225 (2.99), 2.242 (3.56), 2.260 (2.92), 2.279 (0.89), 2.344 (9.28), 2.366 (8.80), 2.377 (3.47), 2.395 (2.10), 2.411 (1.13), 2.824 (1.14), 2.851 (1.19), 2.873 (2.15), 2.893 (7.04), 2.907 (1.58), 2.919 (6.09), 2.945 (1.60), 2.963 (3.40), 2.985 (3.17), 3.003 (1.76), 3.064 (1.25), 3.090 (2.80), 3.118 (1.95), 3.145 (0.84), 3.281 (3.84), 3.308 (3.35), 4.060 (0.75), 4.077 (1.64), 4.095 (4.42), 4.108 (8.66), 4.115 (7.54), 4.126 (5.04), 4.148 (1.33), 4.177 (5.72), 4.188 (9.81), 4.196 (10.09), 5.080 (13.23), 5.308 (16.00), 6.010 (5.32), 6.026 (5.91), 6.317 (10.96), 6.350 (3.44), 6.452 (5.16), 6.475 (5.80), 6.489 (2.52), 7.319 (4.54), 7.336 (4.86), 7.342 (5.67), 7.359 (4.30), 8.122 (9.81), 8.632 (6.83), 8.636 (6.97), 8.648 (3.74), 9.892 (1.04).

### Example 891

### 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)

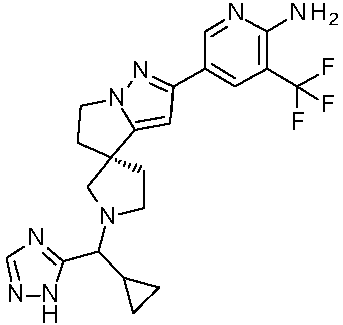

The compound 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) was separated into diastereomers by preparative chiral HPLC to give 24.0 mg (99 % purity, 13 % yield) of the title compound (diastereomer 1 Rt = 4.1 - 4.8 min).

### Preparative chiral HPLC method:

Instrument: PrepCon Labomatic HPLC-3; Column: YMC Amylose SA 5µ, 250x50; eluent A: hexane + 0.1 vol % diethylamine; eluent B: ethanol; isocratic: 20%B; flow: 50 mL/min; temperature: 25 °C; UV: 280 nm

### Analytical chiral HPLC method:

Instrument: Thermo Fisher UltiMate 3000; Column: YMC Amylose SA 3µ, 100x4.6; eluent A: hexane + 0.1 vol % diethylamine; eluent B: ethanol; isocratic: 20%B; flow: 1.4 mL/min; temperature: 25 °C; UV: 280 nm
Analytical chiral HPLC Rt = 3.56 min
[α]²²_{D}: +61.4° (c = 1.00, methanol)
LC-MS (Method 1): Rt = 0.83 min; MS (ESlpos): m/z = 445 [M+H]^{+ 1}H NMR (DMSO-d₆) δ: 13.79 (br s, 1H), 8.58 (d, 1H), 8.00 (d, 1H), 6.52 (s, 2H), 6.43 (s, 1H), 4.11 (t, 2H), 2.99 (br d, 1H), 2.86 (d, 1H), 2.75 - 2.82 (m, 1H), 2.55 - 2.69 (m, 3H), 1.90 - 2.06 (m, 2H), 1.21 - 1.32 (m, 1H), 0.59 - 0.69 (m, 1H), 0.30 - 0.43 (m, 2H), 0.04 - 0.13 (m, 1H).

### Example 892

### 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

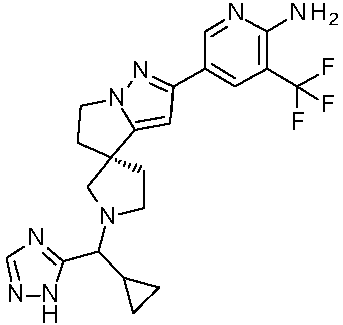

The diastereomers were separated by preparative chiral HPLC (method see **Example 891)** to give 29.0 mg (100 % purity, 16 % yield) of the title compound (diastereomer 2 Rₜ = 5.2 - 6.0 min).
Analytical chiral HPLC (method see **Example 891**): Rt = 5.10 min
[α]²²_{D}: +41.0° (c = 1.00, methanol)
LC-MS (Method 1): Rₜ = 0.84 min; MS (ESlpos): m/z = 445 [M+H]⁺
¹H NMR (DMSO-d₆) δ: 13.81 (br s, 1H), 8.58 (d, 1H), 8.00 (d, 1H), 6.52 (s, 2H), 6.43 (s, 1H), 4.01 - 4.14 (m, 2H), 2.91 - 3.00 (m, 1H), 2.88 (d, 1H), 2.78 (d, 1H), 2.53 - 2.68 (m, 3H), 1.93 - 2.04 (m, 2H), 1.21 - 1.32 (m, 1H), 0.60 - 0.69 (m, 1H), 0.30 - 0.45 (m, 2H), 0.05 - 0.13 (m, 1H).

### Example 893

### 5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

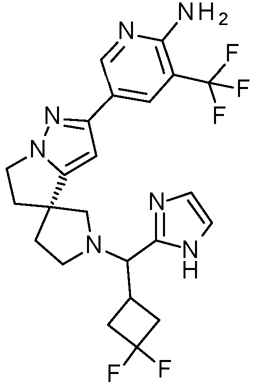

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (129 mg, 358 µmol) and (3,3-difluorocyclobutyl)(1H-imidazol-2-yl)methanone (100 mg, 537 µmol, Intermediate 570) were provided in methanol (3 mL). N,N-Diisopropylethylamine (250 µL, 1.4 mmol) and titanium(IV) isopropoxide (320 µL, 1.1 mmol) were added and the mixture was stirred for 2 h at 60 °C. Sodium cyanoborohydride (67.5 mg, 1.07 mmol) was added and the mixture was stirred for 6 days at 60 °C. The reaction mixture was allowed to cool down, diluted with ethyl acetate and extracted with a saturated aqueous sodium bicarbonate solution and brine. The organic phase was dried over a hydrophobic filter and concentrated. The residue was purified by coloumn chromatography (silica gel, dichloromethane / ethanol gradient). The combined fractions were purified by preparative HPLC to give 60.8 mg (32 % yield) of the title compound.
LC-MS (Method 1): Rt = 1.02 min; MS (ESlpos): m/z = 494 [M+H]⁺
¹H-NMR (400 MHz, DMSO-d6) δ [ppm]: 1.850 (0.63), 1.862 (1.43), 1.878 (3.64), 1.896 (5.86), 1.912 (3.49), 1.926 (1.58), 1.937 (0.63), 1.957 (0.48), 2.043 (0.48), 2.094 (1.27), 2.126 (1.11), 2.318 (0.63), 2.323 (1.43), 2.327 (2.06), 2.332 (1.43), 2.336 (0.63), 2.357 (0.95), 2.375 (2.38), 2.389 (2.85), 2.406 (4.12), 2.418 (3.80), 2.435 (3.01), 2.449 (3.96), 2.466 (4.75), 2.518 (6.81), 2.523 (4.91), 2.589 (3.33), 2.611 (4.12), 2.629 (2.38), 2.636 (3.33), 2.652 (6.81), 2.664 (6.81), 2.669 (3.64), 2.674 (3.01), 2.686 (1.74), 2.744 (3.33), 2.769 (6.50), 2.779 (3.49), 2.792 (4.28), 2.812 (1.27), 2.829 (1.90), 2.846 (1.11), 2.852 (1.58), 2.868 (0.63), 3.301 (0.95), 3.378 (1.58), 3.385 (1.11), 3.684 (2.22), 3.708 (4.44), 3.732 (2.06), 4.050 (2.22), 4.061 (4.12), 4.069 (4.75), 4.078 (7.29), 4.096 (3.33), 6.216 (13.47), 6.276 (16.00), 6.514 (15.37), 6.861 (8.87), 7.083 (4.91), 7.087 (5.54), 7.977 (4.75), 7.982 (8.87), 7.987 (5.23), 8.557 (4.44), 8.563 (7.92), 11.915 (4.12).

### Example 894

### 5-{(3R)-1-[(3,3-difluorocyclobutyl)(1H-imidazol-2-yl)methyl]-5',6'-dihydrospiro[pyrrolidine-3,4'-pyrrolo[1 ,2-b]pyrazol]-2'-yl}-3-(trifluoromethyl)pyridin-2-amine (diastereomer 1)

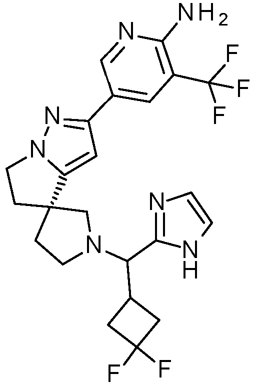

The diastereomeric mixtureof the title compound from **Example 893** was separated by preparative chiral HPLC to give 38.5 mg of the title compound (diastereomer 1, Rₜ = 4.0 - 5.2 min).

### Preparative chiral HPLC method:

Instrument: Sepiatec: Prep SFC100; Column: Chiralpak IG 5µ, 250x50; eluent A: CO2; eluent B: methanol + 0.2 vol % diethylamine; isocratic: 20%B; flow: 100 mL/min; temperature: 40 °C; BPR: 150 bar; UV: 271 nm

### Analytical chiral HPLC method:

Instrument: Waters Acquity UPC2 QDA; Column: Chiralpak IG 3µ, 100x4.6; eluent A: CO2; eluent B: methanol + 0.2 vol % diethylamine; isocratic: 20%B; flow: 4 mL/min; temperature: 40 °C; BPR: 100 bar; UV: 280 nm
Analytical chiral HPLC: Rₜ = 1.38 min
[α]²²_{D}: +58.2° (c = 1.00, methanol)
LC-MS (Method 7): Rt = 0.80 min; MS (ESlpos): m/z = 494 [M+H]⁺
¹H-NMR (400 MHz, DMSO-d6) δ [ppm]: 1.137 (1.27), 1.233 (0.96), 1.352 (0.51), 1.850 (0.57), 1.862 (1.34), 1.877 (1.59), 1.891 (1.47), 1.907 (1.66), 1.910 (1.66), 1.926 (1.34), 1.938 (0.64), 1.958 (0.45), 2.085 (1.02), 2.116 (1.21), 2.318 (1.15), 2.323 (2.61), 2.327 (3.82), 2.332 (2.68), 2.337 (1.15), 2.357 (0.70), 2.375 (1.66), 2.389 (1.98), 2.407 (2.93), 2.424 (1.85), 2.439 (1.08), 2.452 (2.10), 2.468 (4.14), 2.518 (12.69), 2.523 (8.92), 2.589 (3.06), 2.612 (3.63), 2.640 (1.47), 2.660 (2.42), 2.665 (3.31), 2.669 (4.27), 2.673 (3.31), 2.745 (2.29), 2.771 (4.72), 2.780 (2.42), 2.794 (3.19), 3.684 (2.04), 3.708 (2.10), 4.062 (3.25), 4.079 (6.57), 4.097 (3.25), 6.278 (16.00), 6.515 (8.54), 6.863 (3.89), 7.083 (3.44), 7.982 (4.91), 7.987 (4.97), 8.564 (4.53), 8.568 (4.46), 11.907 (2.23).

### Example 895

### 5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

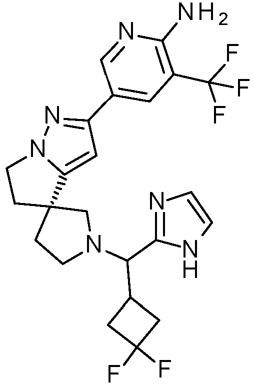

For the preparation of the diastereomeric mixture of the title compound see **Example 893.** The diastereomers were separated by preparative chiral HPLC (method see **Example 894**) to give 22.4 mg of the title compound (diastereomer 2: Rₜ = 8.0 - 9.8 min).
Analytical chiral HPLC (method see **Example 894**): Rt = 3.69 min
[α]²²_{D}: +40.6° (c = 1.00, methanol)
LC-MS (Method 7): Rₜ = 0.80 min; MS (ESlpos): m/z = 494 [M+H]⁺
¹H-NMR (400 MHz, DMSO-d6) δ [ppm]: 1.137 (1.41), 1.232 (1.46), 1.880 (2.42), 1.898 (4.88), 1.915 (2.64), 2.084 (0.87), 2.095 (0.68), 2.116 (1.09), 2.318 (0.77), 2.323 (1.82), 2.327 (2.74), 2.332 (1.91), 2.336 (0.87), 2.369 (0.68), 2.387 (1.32), 2.401 (2.01), 2.419 (2.78), 2.432 (2.37), 2.447 (2.92), 2.465 (2.78), 2.518 (8.57), 2.523 (6.34), 2.618 (0.73), 2.630 (1.78), 2.637 (2.05), 2.653 (5.15), 2.660 (4.15), 2.665 (6.29), 2.673 (3.05), 2.678 (1.87), 2.689 (1.55), 2.770 (1.46), 2.815 (1.19), 2.831 (1.91), 2.853 (1.50), 2.871 (0.59), 3.712 (2.19), 3.736 (1.96), 4.052 (2.23), 4.055 (2.10), 4.070 (4.47), 4.090 (2.23), 6.218 (16.00), 6.517 (8.57), 7.059 (0.41), 7.976 (4.88), 7.981 (4.97), 8.558 (4.51), 8.562 (4.42), 11.945 (0.77).

### Example 896

### 5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 1)

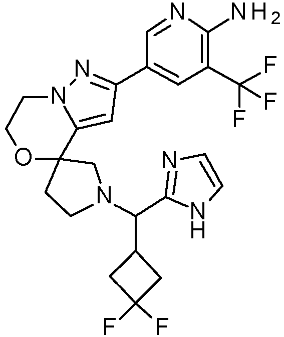

5-[6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (135 mg, 358 µmol) and (3,3-difluorocyclobutyl)(1H-imidazol-2-yl)methanone (100 mg, 537 µmol, Intermediate 570) were provided in methanol (3 mL). N,N-Diisopropylethylamine (250 µL, 1.4 mmol) and titanium(IV) isopropoxide (320 µL, 1.1 mmol) were added and the mixture was stirred for 2 h at 60 °C. Sodium cyanoborohydride (67.5 mg, 1.07 mmol) was added and the mixture was stirred over the weekend at 60 °C. The reaction mixture was allowed to cool down, diluted with ethyl acetate and washed with saturated sodium bicarbonate solution and brine. The organic phase was dried over a hydrophobic filter and concentrated. The residue was purified by column chromatography (silica gel, dichloromethane / ethyl acetate gradient) to give 58.0 mg (29 % yield) of the title compound as a mixture of isomers.

### The isomers were separated by preparative chiral HPLC to give 9.5 mg of the title compound (isomer 1: Rₜ = 5.0 - 6.9 min).

### Preparative chiral HPLC method:

Instrument: Sepiatec: Prep SFC100; Column: Chiral Art Amylose SA 5µ, 250x30; eluent A: CO2; eluent B: ethanol + 0.2 vol % aqueous ammonia (32 %); isocratic: 20%B; flow: 100 mL/min; temperature: 40 °C; BPR: 150 bar; UV: 267 nm

### Analytical chiral HPLC method:

Instrument: Waters Acquity UPC2 QDA; Column: Chiral Art Amylose SA 3µ, 100x4.6; eluent A: CO2; eluent B: ethanol + 0.2 vol % aqueous ammonia (32 %); isocratic: 20%B; flow: 4 mL/min; temperature: 40 °C; BPR: 1800 psi; UV: 254 nm
Analytical chiral HPLC Rₜ = 1.59 min
[α]²⁰_{D}: +55.4° (c = 1.00, methanol)
LC-MS (Method 2): Rt = 0.81 min; MS (ESlpos): m/z = 510 [M+H]⁺
¹H-NMR (400 MHz, DMSO-d6) δ [ppm]: 0.798 (2.35), 0.803 (1.04), 0.815 (2.61), 0.822 (2.78), 0.841 (1.74), 0.852 (0.96), 0.860 (0.87), 0.877 (0.78), 0.886 (1.57), 0.896 (0.52), 0.905 (3.30), 0.923 (1.65), 0.984 (0.70), 1.000 (0.61), 1.043 (0.52), 1.053 (0.52), 1.068 (0.78), 1.109 (0.43), 1.137 (2.96), 1.223 (1.30), 1.232 (2.00), 1.333 (0.43), 1.352 (0.78), 1.779 (0.52), 1.872 (0.52), 1.907 (2.17), 2.023 (1.48), 2.036 (1.65), 2.055 (3.13), 2.075 (3.39), 2.085 (3.04), 2.093 (2.61), 2.116 (2.87), 2.174 (0.70), 2.211 (0.43), 2.318 (1.74), 2.323 (3.91), 2.327 (5.48), 2.332 (3.83), 2.337 (1.91), 2.359 (1.22), 2.375 (1.57), 2.389 (1.48), 2.394 (1.65), 2.413 (1.57), 2.518 (16.00), 2.523 (11.65), 2.631 (1.91), 2.651 (2.09), 2.660 (2.61), 2.665 (4.70), 2.669 (6.26), 2.673 (4.43), 2.679 (2.26), 2.739 (2.61), 2.775 (3.22), 2.801 (3.91), 2.964 (2.96), 2.989 (2.35), 3.674 (2.26), 3.697 (2.00), 3.970 (6.43), 3.983 (5.22), 4.041 (5.57), 4.054 (6.52), 4.066 (3.04), 4.559 (0.52), 6.461 (12.26), 6.575 (12.61), 6.855 (2.52), 7.079 (2.61), 8.006 (7.39), 8.011 (7.13), 8.589 (6.78), 8.593 (6.35), 11.950 (2.09).

### Example 897

### 5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 2)

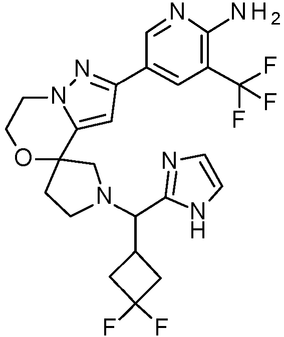

For the preparation of the isomeric mixture of the title compound see **Example 896.** The isomers were separated by preparative chiral HPLC (method see **Example 896)** to give 6.7 mg of the title compound (isomer 2: Rₜ = 7.5 - 9.4 min).
Analytical chiral HPLC (method see **Example 896**): Rt = 2.56 min
[α]²⁰_{D}: +40.2° (c = 1.00, methanol)
LC-MS (Method 2): Rₜ = 0.81 min; MS (ESlpos): m/z = 510 [M+H]⁺
¹H-NMR (400 MHz, DMSO-d6) δ [ppm]: 0.798 (2.52), 0.803 (1.22), 0.815 (2.87), 0.822 (3.04), 0.841 (2.00), 0.852 (1.65), 0.860 (1.30), 0.877 (1.22), 0.886 (1.83), 0.896 (0.70), 0.905 (3.48), 0.923 (1.74), 0.950 (0.52), 0.984 (0.96), 1.000 (0.70), 1.029 (0.61), 1.053 (0.78), 1.068 (1.04), 1.113 (0.61), 1.125 (0.78), 1.137 (6.43), 1.223 (1.74), 1.233 (3.04), 1.333 (0.70), 1.352 (0.78), 1.776 (0.43), 1.884 (0.52), 1.907 (3.13), 1.995 (1.48), 2.011 (1.83), 2.028 (2.43), 2.047 (1.48), 2.085 (3.65), 2.088 (2.87), 2.104 (2.87), 2.116 (4.78), 2.141 (1.65), 2.174 (0.96), 2.191 (0.78), 2.211 (0.52), 2.231 (0.43), 2.318 (1.74), 2.323 (3.83), 2.327 (5.39), 2.332 (3.83), 2.337 (1.91), 2.374 (1.22), 2.389 (1.57), 2.394 (1.57), 2.407 (1.65), 2.413 (1.65), 2.518 (16.00), 2.523 (11.83), 2.660 (2.52), 2.665 (5.13), 2.669 (6.96), 2.673 (5.22), 2.679 (2.78), 2.686 (2.00), 2.706 (1.22), 2.754 (3.22), 2.772 (4.61), 2.789 (3.22), 2.835 (2.61), 2.860 (3.65), 2.918 (3.65), 2.944 (2.26), 2.994 (1.30), 3.681 (2.43), 3.704 (2.09), 3.967 (5.13), 3.977 (4.26), 4.039 (5.22), 4.051 (6.87), 4.063 (3.13), 4.559 (1.13), 6.397 (13.04), 6.461 (1.13), 6.578 (12.17), 6.864 (2.96), 7.099 (2.87), 8.001 (6.96), 8.006 (7.30), 8.585 (6.35), 8.589 (6.43), 11.951 (2.26).

### Example 898

### 5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 3)

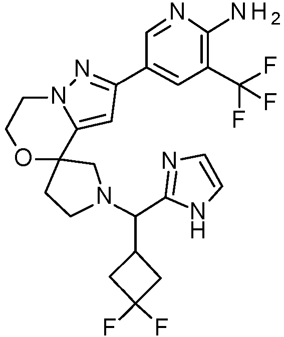

For the preparation of the isomeric mixture of the title compound see **Example 896.** The isomers were separated by preparative chiral HPLC (method see **Example 896)** to give 10.7 mg of the title compound (isomer 3: Rt = 10.1 - 13.7 min).
Analytical chiral HPLC (method see **Example 896**): Rt = 3.81 min
[α]²⁰_{D}: -71.1° (c = 1.00, methanol)
LC-MS (Method 2): Rₜ = 0.81 min; MS (ESlpos): m/z = 510 [M+H]⁺
¹H-NMR (400 MHz, DMSO-d6) δ [ppm]: 0.798 (4.24), 0.803 (1.86), 0.815 (4.69), 0.822 (4.95), 0.841 (3.01), 0.852 (1.77), 0.860 (1.68), 0.877 (1.50), 0.886 (2.92), 0.896 (0.80), 0.905 (5.83), 0.923 (2.83), 0.950 (0.53), 0.984 (1.06), 1.000 (0.88), 1.018 (0.53), 1.029 (0.80), 1.043 (0.80), 1.053 (0.88), 1.068 (1.15), 1.109 (0.62), 1.125 (1.06), 1.137 (3.27), 1.161 (0.62), 1.195 (0.71), 1.223 (1.86), 1.233 (2.21), 1.256 (1.15), 1.334 (0.71), 1.352 (0.88), 1.393 (0.62), 1.412 (0.53), 1.780 (0.62), 1.871 (0.62), 1.907 (3.01), 2.023 (1.68), 2.036 (1.68), 2.054 (3.09), 2.075 (3.36), 2.084 (3.27), 2.093 (2.74), 2.116 (2.74), 2.130 (1.59), 2.142 (1.50), 2.171 (0.97), 2.191 (0.62), 2.210 (0.71), 2.231 (0.62), 2.318 (1.68), 2.323 (3.80), 2.327 (5.48), 2.332 (3.80), 2.337 (1.94), 2.359 (1.41), 2.374 (1.77), 2.389 (1.77), 2.394 (1.94), 2.407 (1.77), 2.413 (1.86), 2.518 (16.00), 2.523 (12.02), 2.631 (1.94), 2.650 (2.03), 2.660 (2.56), 2.665 (4.60), 2.669 (6.28), 2.673 (4.51), 2.679 (2.30), 2.739 (2.56), 2.775 (3.09), 2.801 (3.89), 2.964 (3.01), 2.988 (2.39), 3.674 (2.12), 3.697 (1.94), 3.970 (6.28), 3.983 (5.13), 4.041 (5.39), 4.054 (6.36), 4.559 (0.53), 6.461 (12.73), 6.574 (12.02), 6.855 (2.83), 7.080 (2.92), 8.005 (7.16), 8.010 (6.98), 8.589 (6.45), 8.593 (6.10), 11.948 (2.03).

### Example 899

### 5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 4)

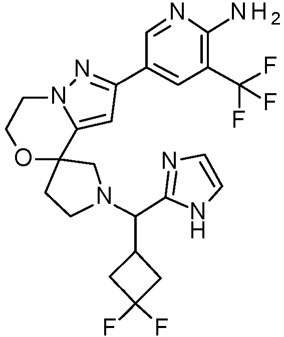

For the preparation of the isomeric mixture of the title compound see **Example 896.** The iusomers were separated by preparative chiral HPLC (method see **Example 896)** to give 8.4 mg of the title compound (isomer 4: Rt = 17.8 - 23.5 min).
Analytical chiral HPLC (method see **Example 896**): Rt = 6.54 min
[α]²⁰_{D}: -43.5° (c = 1.00, methanol)
LC-MS (Method 2): Rt = 0.81 min; MS (ESlpos): m/z = 510 [M+H]⁺
¹H-NMR (400 MHz, DMSO-d6) δ [ppm]: 0.724 (0.73), 0.742 (0.45), 0.798 (1.36), 0.804 (0.91), 0.815 (1.64), 0.822 (1.73), 0.834 (1.45), 0.841 (1.91), 0.845 (1.64), 0.853 (3.00), 0.861 (2.82), 0.872 (1.45), 0.877 (2.18), 0.886 (1.18), 0.896 (0.91), 0.905 (1.91), 0.923 (1.18), 0.929 (0.91), 0.950 (0.64), 0.984 (1.09), 1.000 (0.91), 1.030 (1.55), 1.043 (1.09), 1.053 (1.09), 1.068 (1.18), 1.126 (1.55), 1.138 (2.27), 1.195 (1.09), 1.223 (2.64), 1.233 (2.73), 1.281 (1.09), 1.300 (0.91), 1.334 (1.27), 1.352 (1.18), 1.428 (0.55), 1.446 (0.55), 1.776 (0.55), 1.844 (0.45), 1.884 (0.73), 1.907 (3.09), 1.997 (1.36), 2.011 (1.55), 2.028 (2.00), 2.046 (1.09), 2.088 (2.09), 2.104 (2.27), 2.116 (2.18), 2.174 (1.09), 2.332 (4.00), 2.337 (2.00), 2.518 (16.00), 2.523 (11.64), 2.669 (6.64), 2.673 (4.82), 2.679 (2.55), 2.772 (3.09), 2.789 (2.27), 2.836 (1.64), 2.860 (2.36), 2.919 (2.36), 2.943 (1.36), 3.682 (1.45), 3.704 (1.36), 3.967 (3.45), 3.977 (2.82), 4.038 (3.64), 4.050 (4.73), 4.130 (0.45), 6.397 (8.82), 6.578 (8.36), 6.668 (0.45), 6.864 (1.73), 7.098 (1.82), 8.001 (4.82), 8.006 (4.82), 8.585 (4.55), 11.951 (1.27).

### Example 900

### 5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

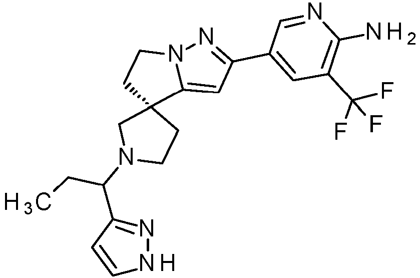

5-[(3R)-5',6'-Dihydrospiro[pyrrolidine-3,4'-pyrrolo[1,2-b]pyrazol]-2'-yl]-3-(trifluoromethyl)pyridin-2-amine (166 mg, 514 µmol) and 1-(1H-pyrazol-3-yl)propan-1-one (58.0 mg, 467 µmol, **Intermediate 573**) were dissolved in methanol (1.6 mL). N,N-Diisopropylethylamine (330 µL, 1.9 mmol) and titanium(IV) isopropoxide (410 µL, 1.4 mmol) were added and the mixture was stirred for 16 h at 60 °C. The reaction mixture was allowed to cool down, sodium cyanoborohydride (88.1 mg, 1.40 mmol) was added and stirred for 2 h at 60 °C. The reaction mixture was allowed to cool down, dluted with water (1 mL) and stirred for 30 min. The suspension was diluted with MeOH and filtered over celite. The filtrate was concentrated. The residue was dissolved in dichloromethane / methanol (small amount of methanol) and washed twice with saturated sodium bicarbonate solution. The aqueous phase was extracted with dichloromethane / methanol. The combined organic layers were dried over a hydrophobic filter and concentrated. The residue was purified by flash column chromatography (silica gel, dichloromehane / methanol (0-8%) gradient) to give 118.5 mg (100 % purity, 53 % yield) of the title compound.
LC-MS (Method 1): Rt = 1.02 min; MS (ESlpos): m/z = 432 [M+H]⁺

### Example 901

### 5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

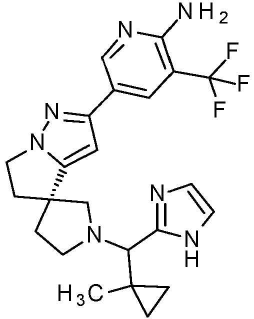

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (258 mg, 718 µmol) and (1H-imidazol-2-yl)(1-methylcyclopropyl)methanone (162 mg, 1.08 mmol, Intermediate 569) were provided in methanol (6 mL). N,N-Diisopropylethylamine (500 µL, 2.8 mmol) and titanium(IV) isopropoxide (640 µL, 2.2 mmol) were added and the mixture was stirred for 2 h at 60 °C. Sodium cyanoborohydride (135 mg, 2.15 mmol) was added and the mixture was stirred for 7 days at 60 °C. 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (50 mg, 139 µmol) was added and the reaction mixture was stirred for 3 days at 60 °C. The reaction mixture was allowed to cool down, diluted with ethyl acetate and extracted with saturated aqueous sodium bicarbonate solution and brine. The organic phase was dried over a hydrophobic filter and concentrated. The residue was purified by preparative HPLC to give 121 mg (33 % yield) of the title compound.
LC-MS (Method 1): Rt = 1.05 min; MS (ESlpos): m/z = 458 [M+H]⁺
¹H-NMR (400 MHz, DMSO-d6) δ [ppm]: -0.035 (0.99), 0.058 (1.44), 0.068 (2.15), 0.077 (2.09), 0.089 (1.51), 0.375 (1.00), 0.388 (1.72), 0.396 (2.22), 0.419 (2.96), 0.441 (2.34), 0.452 (2.17), 0.464 (1.31), 0.485 (0.92), 0.492 (0.97), 0.506 (1.12), 0.543 (0.85), 0.552 (0.87), 0.566 (1.29), 0.578 (0.81), 1.022 (0.40), 1.040 (1.17), 1.055 (16.00), 1.095 (15.14), 1.938 (0.41), 1.957 (0.88), 1.971 (1.73), 1.988 (3.05), 2.003 (3.08), 2.021 (1.69), 2.033 (1.31), 2.046 (1.09), 2.323 (0.87), 2.327 (1.21), 2.331 (0.91), 2.458 (2.71), 2.522 (5.52), 2.589 (6.31), 2.608 (7.77), 2.617 (4.53), 2.636 (3.23), 2.647 (2.39), 2.655 (1.73), 2.664 (2.43), 2.669 (2.39), 2.673 (1.87), 2.679 (1.63), 2.688 (0.77), 2.696 (0.62), 2.824 (2.58), 2.847 (2.28), 2.951 (2.15), 2.974 (2.04), 3.410 (1.05), 4.073 (1.22), 4.088 (2.55), 4.107 (2.83), 4.115 (2.87), 4.122 (2.09), 4.132 (4.51), 4.150 (2.31), 6.449 (0.68), 6.507 (9.97), 6.527 (12.83), 6.590 (9.53), 6.777 (4.61), 6.999 (2.69), 7.040 (2.85), 7.999 (4.76), 8.005 (5.68), 8.013 (3.61), 8.488 (1.24), 8.493 (1.20), 8.594 (6.10), 11.585 (1.81), 11.612 (1.73).

### Example 902

### 5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)

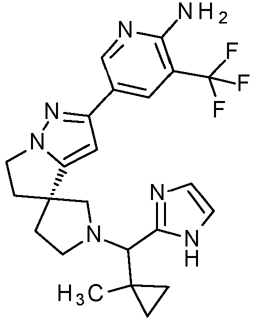

The diastereomeric mixture of the title compound from **Example 901** was separated by preparative chiral HPLC to give 25.0 mg of the title compound (diastereomer 1: Rₜ = 3.6 - 4.6 min).

### Preparative chiral HPLC method:

Instrument: Sepiatec: Prep SFC100; Column: Chiral Art Amylose SA, 250x30; eluent A: CO2; eluent B: methanol + 0.2 vol % aqueous ammonia (32%); isocratic: 20%B; flow: 100 mL/min; temperature: 40 °C; BPR: 150 bar; UV: 271 nm

### Analytical chiral HPLC method:

Instrument: Waters Acquity UPC2 QDA; Column: Chiral Art Amylose SA 3µ, 100x4.6; eluent A: CO2; eluent B: methanol + 0.2 vol % aqueous ammonia (32%); isocratic: 20%B; flow: 4 mL/min; temperature: 40 °C; BPR: 100 bar; UV: 280 nm
Analytical chiral HPLC Rt = 0.91 min
[α]²²_{D}: +103.6° (c = 1.00, methanol)
¹H-NMR (400 MHz, DMSO-d6) δ [ppm]: 0.058 (0.53), 0.065 (0.86), 0.079 (1.36), 0.090 (0.79), 0.100 (0.64), 0.374 (0.49), 0.383 (0.76), 0.387 (0.86), 0.395 (1.32), 0.409 (0.94), 0.415 (1.25), 0.420 (1.14), 0.426 (0.74), 0.440 (1.10), 0.451 (1.06), 0.463 (0.69), 0.484 (0.85), 0.492 (0.88), 0.507 (1.05), 0.519 (0.56), 1.097 (16.00), 1.137 (0.68), 1.956 (0.51), 1.969 (0.82), 1.985 (1.05), 2.004 (0.78), 2.011 (0.74), 2.026 (0.89), 2.033 (0.92), 2.047 (0.92), 2.057 (0.47), 2.323 (0.58), 2.327 (0.82), 2.331 (0.58), 2.458 (2.32), 2.518 (3.96), 2.523 (3.10), 2.560 (0.87), 2.590 (5.61), 2.603 (0.99), 2.609 (1.14), 2.624 (1.06), 2.632 (1.19), 2.648 (1.71), 2.665 (1.83), 2.669 (1.19), 2.673 (0.83), 2.680 (1.21), 2.696 (0.48), 2.953 (2.11), 2.976 (1.89), 4.117 (2.18), 4.134 (4.23), 4.151 (2.09), 6.507 (10.93), 6.527 (5.66), 6.776 (1.07), 6.999 (1.04), 8.007 (3.17), 8.013 (3.24), 8.594 (2.96), 8.598 (2.90), 11.610 (0.86).

### Example 903

### 5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

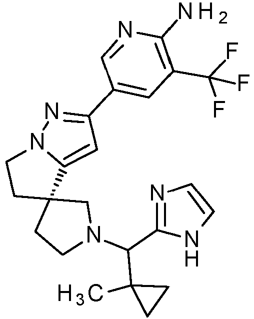

For the preparation of the diastereomeric mixture of the title compound see **Example 901.** The diastereomers were separated by preparative chiral HPLC (method see **Example 902**) to give 22.4 mg of the title compound (diastereomer 2 Rₜ = 6.2 - 8.1 min).
Analytical chiral HPLC (method see **Example 902**): Rt = 2.15 min
[α]²²_{D}: -9.6° (c = 1.00, methanol)
¹H-NMR (400 MHz, DMSO-d6) δ [ppm]: 0.058 (0.53), 0.065 (0.86), 0.079 (1.36), 0.090 (0.79), 0.100 (0.64), 0.374 (0.49), 0.383 (0.76), 0.387 (0.86), 0.395 (1.32), 0.409 (0.94), 0.415 (1.25), 0.420 (1.14), 0.426 (0.74), 0.440 (1.10), 0.451 (1.06), 0.463 (0.69), 0.484 (0.85), 0.492 (0.88), 0.507 (1.05), 0.519 (0.56), 1.097 (16.00), 1.137 (0.68), 1.956 (0.51), 1.969 (0.82), 1.985 (1.05), 2.004 (0.78), 2.011 (0.74), 2.026 (0.89), 2.033 (0.92), 2.047 (0.92), 2.057 (0.47), 2.323 (0.58), 2.327 (0.82), 2.331 (0.58), 2.458 (2.32), 2.518 (3.96), 2.523 (3.10), 2.560 (0.87), 2.590 (5.61), 2.603 (0.99), 2.609 (1.14), 2.624 (1.06), 2.632 (1.19), 2.648 (1.71), 2.665 (1.83), 2.669 (1.19), 2.673 (0.83), 2.680 (1.21), 2.696 (0.48), 2.953 (2.11), 2.976 (1.89), 4.117 (2.18), 4.134 (4.23), 4.151 (2.09), 6.507 (10.93), 6.527 (5.66), 6.776 (1.07), 6.999 (1.04), 8.007 (3.17), 8.013 (3.24), 8.594 (2.96), 8.598 (2.90), 11.610 (0.86).

### Example 904

### 5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

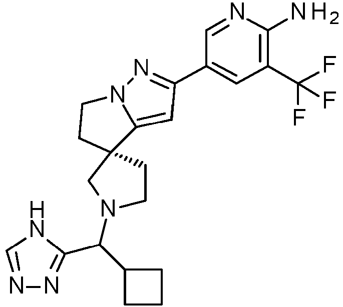

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (63.5 mg, 176 µmol) and cyclobutyl(4H-1,2,4-triazol-3-yl)methanone (40.0 mg, 265 µmol, Intermediate 558) were dissolved in methanol (3.8 mL). N,N-Diisopropylethylamine (120 µL, 710 µmol) and titanium(IV) isopropoxide (160 µL, 530 µmol) were added and the mixture was stirred for 3 h at 60 °C. The reaction mixture was allowed to cool down, sodium cyanoborohydride (33.3 mg, 529 µmol) was added and the mixture was stirred over night at 60 °C. The reaction mixture was allowed to cool down and the precipitate was filtered off. The filtrate was diluted with ethyl acetate and extracted with a saturated sodium bicarbonate solution and water. The organic phase was washed with brine, dried and concentrated. The residue was purified by coloumn chromatography (silica gel NH2, hexane / ethyl acetatel (0 - 100 %) gradient). The combined fractions were purified by preparative HPLC to give 36.0 mg (95 % purity, 42 % yield) of the title compound.
LC-MS (Method 1): Rt = 0.92 min; MS (ESlpos): m/z = 459 [M+H]⁺
¹H-NMR (400 MHz, DMSO-d6) δ [ppm]: 1.222 (0.98), 1.240 (1.90), 1.258 (0.98), 1.299 (1.05), 1.541 (0.59), 1.564 (0.98), 1.587 (0.79), 1.769 (1.05), 1.798 (0.92), 1.820 (0.52), 1.850 (0.52), 1.870 (0.85), 1.893 (0.92), 1.953 (1.90), 1.974 (2.03), 1.995 (1.05), 2.055 (3.08), 2.189 (0.59), 2.386 (1.31), 2.390 (2.82), 2.395 (4.07), 2.399 (2.82), 2.404 (1.31), 2.586 (16.00), 2.590 (11.08), 2.608 (2.16), 2.659 (1.18), 2.680 (1.25), 2.706 (1.90), 2.727 (1.77), 2.732 (3.28), 2.737 (4.39), 2.741 (3.08), 2.746 (1.51), 2.840 (0.92), 2.948 (0.72), 3.816 (0.85), 3.840 (0.85), 4.085 (0.66), 4.102 (0.66), 4.120 (1.31), 4.136 (2.49), 4.148 (2.62), 4.166 (1.25), 5.827 (5.97), 6.338 (0.79), 6.370 (1.05), 6.583 (5.77), 8.048 (3.41), 8.054 (3.48), 8.585 (0.85), 8.623 (3.15), 13.863 (1.64).

### Example 905

### 5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)

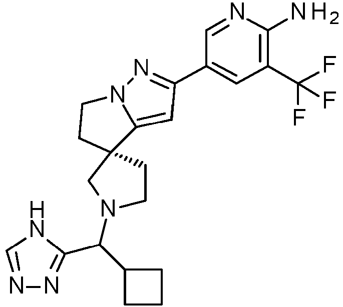

The title compound from **Example 904** was separated into diastereomers by preparative chiral HPLC to give 2.60 mg (90 % purity) of the title compound (diastereomer 1 Rt = 8.5 - 10.5 min).

### Preparative chiral HPLC method:

Instrument: Sepiatec: Prep SFC100; Column: Chiralpak IG 5µ 250x50; eluent A: CO2; eluent B: methanol + 0.2 vol % aqueous ammonia (32%); isocratic: 25%B; flow: 100 mL/min; temperature: 40 °C; BPR: 150 bar; UV: 280 nm

### Analytical chiral HPLC method:

Instrument: Agilent: 1260, Aurora SFC-Modul; Column: Chiralpak IG 3µ, 100x4.6; eluent A: CO2; eluent B: methanol + 0.2 vol % aqueous ammonia (32%); isocratic: 25%B; flow: 4 mL/min; temperature: 37.5 °C; BPR: 100 bar; UV: 280 nm
Analytical chiral HPLC Rt = 2.70 min
LC-MS (Method 1): Rt = 0.92 min; MS (ESlpos): m/z = 459 [M+H]⁺
¹H-NMR (400 MHz, METHANOL-d4) δ [ppm]: -0.028 (1.85), 0.119 (3.76), 0.904 (1.21), 0.920 (1.62), 0.937 (0.89), 1.241 (1.70), 1.259 (3.32), 1.270 (11.60), 1.277 (2.22), 1.285 (1.08), 1.308 (5.96), 1.359 (0.96), 1.389 (0.66), 1.397 (0.73), 1.408 (0.54), 1.419 (0.64), 1.438 (0.73), 1.473 (0.66), 1.577 (0.56), 1.600 (1.58), 1.626 (2.58), 1.649 (2.49), 1.672 (1.04), 1.721 (1.23), 1.729 (1.73), 1.737 (1.70), 1.749 (1.73), 1.757 (1.95), 1.765 (1.54), 1.785 (2.00), 1.812 (1.97), 1.834 (0.96), 1.896 (1.33), 1.918 (2.12), 1.941 (1.85), 1.963 (1.81), 1.977 (1.97), 1.995 (3.89), 2.023 (3.16), 2.032 (6.19), 2.046 (1.97), 2.069 (0.75), 2.084 (1.21), 2.104 (1.79), 2.119 (1.66), 2.131 (1.18), 2.152 (0.83), 2.199 (5.07), 2.217 (1.25), 2.228 (1.64), 2.236 (1.62), 2.245 (1.50), 2.254 (1.37), 2.264 (0.96), 2.283 (0.56), 2.521 (0.91), 2.539 (1.70), 2.554 (2.12), 2.571 (2.39), 2.589 (1.45), 2.647 (3.26), 2.661 (2.60), 2.678 (5.96), 2.694 (2.43), 2.718 (2.72), 2.736 (2.99), 2.878 (2.12), 3.002 (1.21), 3.021 (1.18), 3.797 (4.86), 3.821 (4.57), 4.110 (1.27), 4.128 (1.60), 4.141 (4.47), 4.159 (8.13), 4.176 (4.03), 5.513 (6.84), 6.324 (16.00), 8.111 (6.86), 8.116 (7.02), 8.530 (5.67).

### Example 906

### 5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

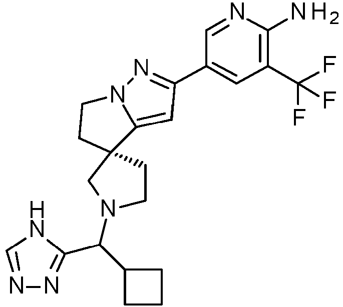

For the preparation of the diastereomeric mixture of the title compound see **Example 904.** The diastereomers were separated by preparative chiral HPLC (method see **Example 905**) to give 3.30 mg (90 % purity) of the title compound (diastereomer 2 Rₜ = 16.3 - 19.3 min).
Analytical chiral HPLC (method see **Example 905**): Rt = 4.48 min
LC-MS (Method 1): Rt = 0.92 min; MS (ESlpos): m/z = 459 [M+H]⁺
¹H-NMR (400 MHz, METHANOL-d4) δ [ppm]: 0.101 (3.27), 0.884 (1.24), 0.901 (1.78), 1.120 (0.57), 1.136 (0.57), 1.223 (2.05), 1.241 (4.31), 1.251 (8.03), 1.259 (2.70), 1.267 (1.65), 1.289 (6.55), 1.378 (1.00), 1.400 (0.67), 1.441 (1.11), 1.562 (0.59), 1.585 (1.63), 1.611 (2.65), 1.634 (2.50), 1.658 (1.07), 1.707 (1.87), 1.716 (1.76), 1.727 (1.85), 1.736 (1.74), 1.770 (1.85), 1.797 (2.11), 1.819 (1.04), 1.859 (0.57), 1.882 (1.39), 1.904 (2.29), 1.928 (1.98), 1.937 (1.28), 1.951 (1.28), 1.968 (1.37), 1.988 (2.48), 2.014 (10.36), 2.029 (3.61), 2.037 (3.77), 2.059 (2.70), 2.073 (2.11), 2.106 (0.65), 2.180 (3.13), 2.230 (1.76), 2.238 (1.72), 2.248 (1.65), 2.510 (0.92), 2.529 (1.41), 2.543 (2.39), 2.562 (2.57), 2.577 (1.91), 2.595 (1.54), 2.612 (2.33), 2.629 (3.57), 2.644 (1.44), 2.660 (14.69), 2.722 (2.85), 2.743 (2.07), 2.827 (1.63), 2.904 (1.48), 2.986 (1.35), 3.012 (1.44), 3.789 (3.63), 3.814 (3.40), 4.074 (0.85), 4.091 (2.29), 4.103 (3.00), 4.110 (2.81), 4.117 (5.16), 4.122 (4.40), 4.131 (4.25), 4.136 (5.11), 4.151 (2.85), 4.178 (0.55), 5.495 (4.31), 6.298 (16.00), 8.046 (0.55), 8.094 (7.32), 8.098 (7.62), 8.516 (5.40).

### Example 907

### 5-{(3'R)-1'-[cyclohexyl(1H-imidazol-2-yl)methyl]-6, 7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

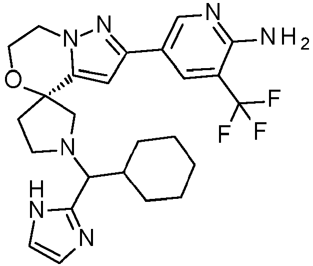

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (78.1 mg, 90 % purity, 187 µmol) and cyclohexyl(1H-imidazol-2-yl)methanone (50.0 mg, 281 µmol, Intermediate 557) were dissolved in methanol (3.2 mL). N,N-Diisopropylethylamine (130 µL, 751 µmol) and titanium(IV) isopropoxide (170 µL, 560 µmol) were added and the mixture was stirred for 3 h at 60 °C. The reaction mixture was allowed to cool down, sodium cyanoborohydride (35 mg, 561 µmol) was added and the mixture was stirred over night at 60 °C. The reaction mixture was allowed to cool down and the precipitate was filtered off. The filtrate was diluted with ethyl acetate and extracted with a saturated sodium bicarbonate solution and water. The organic phase was washed with brine, dried and concentrated. The residue was purified by coloumn chromatography (silica gel NH2, hexane / ethyl acetatel (0 - 100 %) and ethyl acetate / ethanol (0 - 100 %) gradient). The combined fractions were purified by preparative HPLC to give 10.0 mg (95 % purity, 10 % yield) of the title compound.
LC-MS (Method 1): Rt = 1.11 min; MS (ESlpos): m/z = 503 [M+H]⁺
¹H-NMR (400 MHz, DMSO-d6) δ [ppm]: 0.822 (0.99), 0.918 (0.63), 0.935 (0.99), 0.965 (0.99), 0.992 (0.49), 1.162 (1.27), 1.184 (1.48), 1.221 (1.27), 1.239 (1.55), 1.299 (2.54), 1.420 (1.20), 1.633 (2.04), 1.659 (1.83), 1.719 (0.70), 1.751 (0.99), 1.920 (0.78), 1.945 (0.92), 2.014 (0.85), 2.032 (0.85), 2.055 (2.04), 2.062 (1.34), 2.091 (1.27), 2.110 (2.26), 2.130 (1.90), 2.146 (0.78), 2.152 (1.76), 2.394 (4.44), 2.398 (3.10), 2.404 (1.41), 2.585 (16.00), 2.590 (10.85), 2.632 (0.92), 2.654 (1.13), 2.676 (0.70), 2.692 (0.49), 2.736 (4.37), 2.740 (2.96), 2.745 (1.27), 2.823 (0.78), 2.856 (2.26), 2.870 (1.97), 2.881 (2.47), 2.895 (1.90), 3.009 (1.41), 3.034 (1.06), 3.083 (1.90), 3.108 (1.62), 3.518 (2.82), 3.541 (2.82), 4.035 (3.88), 4.047 (3.17), 4.108 (3.31), 4.121 (4.09), 4.133 (1.83), 5.827 (10.22), 6.407 (5.43), 6.461 (7.82), 6.644 (6.70), 6.909 (2.26), 7.096 (1.83), 7.119 (1.34), 8.060 (3.95), 8.064 (3.95), 8.552 (0.49), 8.640 (4.02), 11.803 (1.69).

### Example 908

### 5-{(3R)-1-[cyclohexyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

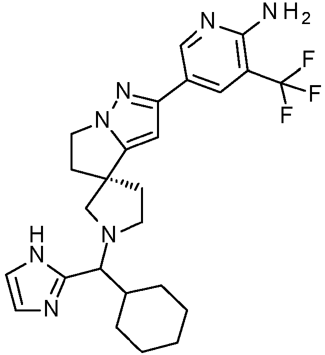

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (67.3 mg, 187 µmol) and cyclohexyl(1H-imidazol-2-yl)methanone (50.0 mg, 281 µmol, Intermediate 557) were dissolved in methanol (4 mL). N,N-Diisopropylethylamine (130 µL, 750 µmol) and titanium(IV) isopropoxide (170 µL, 560 µmol) were added and the mixture was stirred for 3 h at 60 °C. The reaction mixture was allowed to cool down, sodium cyanoborohydride (35 mg, 561 µmol) was added and the mixture was stirred over night at 60 °C. The reaction mixture was allowed to cool down and the precipitate was filtered off. The filtrate was diluted with ethyl acetate and extracted with a saturated sodium bicarbonate solution and water. The organic phase was washed with brine, dried and concentrated. The residue was purified by coloumn chromatography (silica gel NH2, hexane / ethyl acetatel (0 - 100 %) and ethyl acetate / ethanol (0 - 100 %) gradient). The combined fractions were purified by preparative HPLC to give 11.0 mg (90 % purity, 11 % yield) of the title compound.
LC-MS (Method 1): Rt = 1.15 min; MS (ESlpos): m/z = 487 [M+H]⁺
¹H-NMR (400 MHz, DMSO-d6) δ [ppm]: -0.020 (1.80), 0.765 (0.69), 0.793 (0.83), 0.822 (0.42), 0.899 (0.48), 0.942 (0.69), 0.975 (0.76), 1.001 (0.48), 1.110 (0.62), 1.138 (1.32), 1.163 (1.39), 1.202 (0.76), 1.220 (0.83), 1.239 (0.83), 1.280 (2.08), 1.356 (0.97), 1.388 (0.83), 1.620 (1.45), 1.641 (1.45), 1.742 (0.76), 1.897 (1.66), 1.915 (2.63), 1.929 (1.87), 1.945 (1.32), 2.036 (0.83), 2.053 (0.83), 2.084 (0.76), 2.384 (1.52), 2.408 (0.69), 2.423 (0.83), 2.440 (1.32), 2.456 (1.04), 2.470 (1.32), 2.488 (1.73), 2.497 (2.35), 2.566 (16.00), 2.571 (10.87), 2.621 (1.18), 2.643 (1.59), 2.655 (0.83), 2.682 (1.45), 2.726 (1.32), 2.750 (1.52), 2.772 (0.83), 2.867 (1.94), 2.889 (1.39), 3.487 (0.42), 3.507 (1.80), 3.531 (1.73), 4.106 (2.08), 4.124 (4.09), 4.141 (2.08), 5.808 (11.01), 6.228 (5.75), 6.290 (5.68), 6.564 (5.33), 6.890 (1.39), 7.076 (1.04), 7.229 (0.42), 8.021 (3.39), 8.026 (3.39), 8.481 (0.42), 8.597 (3.12), 8.602 (3.12), 11.757 (0.83).

### Example 909

### 5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

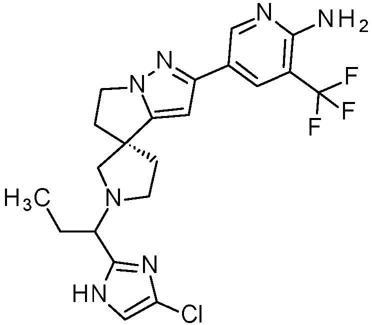

(3R)-2'-[6-Amino-5-(trifluoromethyl)pyridin-3-yl]-5',6'-dihydrospiro[pyrrolidin-1-ium-3,4'-pyrrolo[1,2-b]pyrazole] chloride (51.6 mg, 144 µmol) and 4-chloro-2-propanoyl-1H-imidazol-1-ium chloride (60.0 mg, 70 % purity, 215 µmol, **Intermediate 566**) were dissolved in methanol (0.9 mL). N,N-Diisopropylethylamine (130 µL, 720 µmol) and titanium(IV) isopropoxide (130 µL, 430 µmol) were added and the mixture was stirred for 16 h at 60 °C. The reaction mixture was cooled to room temperature and sodium cyanoborohydride (27.1 mg, 431 µmol) was added and stirred for 2 h at 60 °C. The reaction mixture was allowed to cool down, dluted with water and stirred for 30 min. The suspension was diluted with MeOH and filtered over celite. The filtrate was concentrated. The residue was dissolved in ethyl acetate and washed with a saturated sodium bicarbonate solution and brine. The organic phase was dried over sodium sulfate, filtered and concentrated. The residue was purified by coloumn chromatography (silica gel, dichloromethane / methanol (0 - 15 %) gradient) to give 46.3 mg (95 % purity, 66 % yield) of the title compound.
LC-MS (Method 1): Rt = 1.04 min; MS (ESlpos): m/z = 466 [M+H]⁺
¹H-NMR (400 MHz, CHLOROFORM-d) δ [ppm]: 0.826 (2.33), 0.836 (2.63), 0.844 (5.41), 0.855 (5.46), 0.863 (2.71), 0.874 (2.49), 1.407 (0.27), 1.446 (0.34), 1.465 (0.45), 1.484 (0.21), 1.729 (0.30), 1.747 (0.45), 1.762 (0.70), 1.774 (0.58), 1.781 (0.73), 1.796 (0.59), 1.814 (0.47), 1.833 (0.27), 1.912 (0.67), 1.928 (0.95), 1.942 (0.96), 1.947 (0.97), 1.961 (1.01), 1.976 (0.73), 1.980 (0.69), 1.992 (0.66), 2.004 (0.68), 2.010 (0.64), 2.022 (0.92), 2.037 (0.96), 2.042 (0.89), 2.058 (0.96), 2.074 (0.51), 2.123 (0.47), 2.138 (0.58), 2.144 (0.60), 2.158 (0.67), 2.165 (0.54), 2.170 (0.49), 2.181 (0.72), 2.186 (0.66), 2.201 (0.57), 2.213 (0.41), 2.219 (0.39), 2.234 (0.33), 2.541 (0.35), 2.558 (0.57), 2.574 (1.09), 2.591 (1.45), 2.600 (0.81), 2.635 (1.35), 2.641 (0.91), 2.649 (1.39), 2.655 (1.11), 2.673 (1.50), 2.688 (0.37), 2.696 (0.37), 2.713 (1.29), 2.736 (2.13), 2.755 (0.86), 2.772 (0.60), 2.799 (1.23), 2.822 (0.73), 2.879 (0.34), 2.909 (1.43), 2.922 (0.82), 2.931 (1.30), 2.944 (0.61), 2.966 (0.26), 3.521 (0.79), 3.527 (0.88), 3.532 (0.85), 3.538 (0.88), 3.543 (0.82), 3.549 (0.78), 4.126 (0.21), 4.133 (0.25), 4.141 (0.28), 4.146 (0.32), 4.153 (1.00), 4.160 (1.02), 4.169 (1.77), 4.173 (1.29), 4.177 (1.18), 4.180 (1.39), 4.185 (1.61), 4.188 (1.67), 4.197 (1.80), 4.204 (1.47), 4.210 (0.55), 4.217 (0.92), 4.224 (0.33), 4.231 (0.43), 4.244 (0.22), 5.043 (3.95), 5.084 (0.19), 5.308 (16.00), 6.165 (5.12), 6.187 (0.18), 6.200 (4.98), 6.217 (0.18), 6.886 (2.98), 6.923 (2.97), 8.105 (1.72), 8.110 (2.89), 8.116 (1.64), 8.598 (1.58), 8.604 (2.08), 8.611 (1.51), 9.317 (0.25), 9.414 (0.22).

### Example 910

### 5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

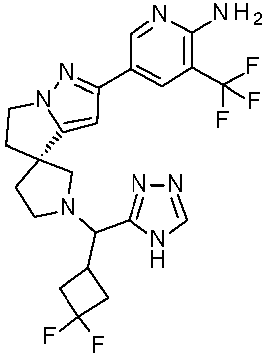

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (216 mg, 601 µmol) and (3,3-difluorocyclobutyl)(1H-1,2,4-triazol-5-yl)methanone (135 mg, 721 µmol, Intermediate 565) were provided in methanol (5 mL). N,N-Diisopropylethylamine (420 µL, 2.4 mmol) and titanium(IV) isopropoxide (530 µL, 1.8 mmol) were added and the mixture was stirred for 2 h at 60 °C. Sodium cyanoborohydride (113 mg, 1.80 mmol) was added and the mixture was stirred for 3 days at 60 °C. The reaction mixture was allowed to cool down, diluted with ethyl acetate and washed with saturated aqueous sodium bicarbonate solution and brine. The organic phase was dried over a hydrophobic filter and concentrated. The residue was purified several times by column chromatography (silica gel, dichloromethane / ethyl acetate / ethanol and ethyl acetate / ethanol gradient) to give 57.0 mg (19 % yield) of the title compound.
LC-MS (Method 2): Rt = 0.76 min; MS (ESlpos): m/z = 495 [M+H]⁺
¹H-NMR (400 MHz, DMSO-d6) δ [ppm]: 0.798 (0.92), 0.802 (1.05), 0.814 (0.98), 0.821 (1.05), 0.840 (0.46), 0.886 (0.52), 0.904 (1.18), 0.923 (0.52), 1.035 (6.23), 1.052 (16.00), 1.070 (8.26), 1.089 (0.92), 1.160 (1.18), 1.232 (0.92), 1.353 (0.59), 1.842 (0.46), 1.863 (1.11), 1.881 (2.03), 1.898 (1.84), 1.912 (1.64), 2.121 (0.72), 2.147 (0.72), 2.318 (1.11), 2.323 (2.43), 2.327 (3.48), 2.331 (2.43), 2.337 (1.11), 2.364 (0.46), 2.378 (0.92), 2.395 (1.57), 2.409 (1.90), 2.437 (2.36), 2.454 (2.69), 2.518 (12.59), 2.523 (8.52), 2.615 (1.25), 2.636 (1.97), 2.654 (1.84), 2.659 (1.84), 2.665 (3.08), 2.669 (4.20), 2.673 (3.41), 2.678 (2.62), 2.703 (2.36), 2.725 (1.97), 2.746 (2.82), 2.768 (2.95), 2.786 (3.41), 2.807 (2.03), 2.820 (1.05), 2.843 (0.85), 2.864 (0.98), 2.882 (0.59), 3.364 (0.66), 3.404 (1.05), 3.417 (1.05), 3.422 (3.61), 3.435 (3.74), 3.440 (3.15), 3.452 (3.34), 3.457 (1.11), 3.469 (1.11), 3.817 (0.98), 3.839 (1.11), 3.901 (0.52), 3.920 (0.92), 3.944 (0.59), 4.063 (1.97), 4.079 (3.87), 4.089 (2.49), 4.344 (2.49), 4.357 (4.79), 4.370 (2.36), 5.759 (3.48), 6.245 (4.20), 6.257 (2.75), 6.298 (4.13), 6.308 (2.69), 6.509 (5.64), 6.520 (4.33), 7.938 (2.56), 7.988 (4.66), 8.522 (3.67), 8.527 (3.67), 8.562 (3.54), 13.920 (2.56), 13.947 (1.38).

### Example 911

### 5-{(3'R)-1'-[cyclopropyl(1H-imidazol-2-yl)methyl]-6, 7-dihydrospiro[pyrazolo[5,1-c][1,4]oxazine-4,3'-pyrrolidin]-2-yl}-3-(trifluoromethyl)pyrazin-2-amine (diastereomer 1 and diastereomer 2)

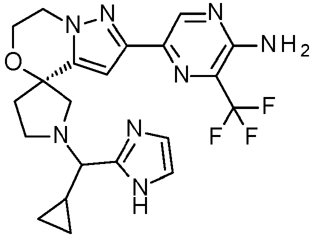

5-[(3'R)-6,7-Dihydrospiro[pyrazolo[5,1-c][1,4]oxazine-4,3'-pyrrolidin]-2-yl]-3-(trifluoromethyl)pyrazin-2-amine (100 mg, 90 % purity, 264 µmol) and cyclopropyl(1H-imidazol-2-yl)methanone (54.0 mg, 397 µmol) were dissoved in methanol (4.6 mL). N,N-Diisopropylethylamine (184 µL, 1.06 mmol) and titanium(IV) isopropoxide (230 µL, 790 µmol) were added and the mixture was stirred for 3 h at 60 °C. The reaction mixture was allowed to cool down, sodium cyanoborohydride (49.8 mg, 0.7 mmol) was added and the mixture was stirred for 20 h at 60 °C. Sodium cyanoborohydride (24.9 mg, 0.35 mmol) was added and stirred for 20 h at 60 °C. A second batch of sodium cyanoborohydride (24.9 mg, 0.35 mmol) was added and stirred for 20 h at 60 °C. Sodium cyanoborohydride (24.9 mg, 0.35 mmol) was added and stirred for 3 days at 60 °C. The reaction mixture was allowed to cool down, diluted with water and stirred for 15 min. The precipitate was filtered off and washed with methanol. The filtrate was concentrated. The residue was purified by preparative HPLC to give 10.0 mg (90 % purity, 7 % yield) of the title compound.
LC-MS (Method 1): Rt = 0.92 min; MS (ESlpos): m/z = 462 [M+H]⁺
¹H-NMR (400 MHz, DMSO-d6) δ [ppm]: -0.134 (0.19), 0.251 (2.22), 0.281 (1.72), 0.293 (2.17), 0.304 (2.46), 0.316 (2.32), 0.327 (1.53), 0.412 (0.54), 0.432 (0.54), 0.559 (2.13), 0.781 (0.79), 0.933 (0.83), 0.944 (0.93), 0.949 (1.12), 0.959 (1.22), 0.980 (0.43), 1.069 (0.37), 1.162 (4.84), 1.283 (0.35), 1.684 (2.98), 1.834 (0.31), 1.918 (0.66), 1.939 (1.10), 1.951 (0.81), 1.973 (1.32), 1.993 (0.91), 2.025 (1.30), 2.040 (1.97), 2.056 (2.61), 2.075 (2.26), 2.105 (2.92), 2.118 (2.61), 2.227 (3.08), 2.259 (1.39), 2.263 (1.18), 2.287 (0.60), 2.471 (3.71), 2.601 (1.78), 2.617 (3.19), 2.626 (3.50), 2.639 (4.59), 2.649 (3.48), 2.665 (2.28), 2.699 (1.01), 2.714 (2.22), 2.740 (3.19), 2.777 (1.06), 2.802 (0.64), 2.845 (1.37), 2.864 (2.48), 2.896 (2.69), 2.927 (2.94), 2.939 (2.26), 3.097 (3.08), 3.109 (2.03), 3.133 (2.77), 3.159 (2.48), 3.496 (0.62), 3.611 (0.46), 3.640 (0.31), 3.757 (0.31), 3.893 (0.77), 3.910 (1.22), 3.924 (2.28), 3.944 (2.92), 3.957 (3.42), 3.972 (5.44), 3.982 (5.40), 3.990 (5.28), 4.002 (3.27), 4.042 (7.49), 4.054 (6.75), 4.065 (5.40), 4.073 (3.85), 4.107 (1.22), 5.746 (0.15), 6.038 (0.41), 6.043 (0.37), 6.095 (0.21), 6.448 (2.03), 6.460 (14.14), 6.525 (6.46), 6.601 (0.48), 6.634 (1.06), 6.655 (0.97), 6.684 (1.03), 6.696 (0.95), 6.890 (14.76), 7.009 (0.81), 7.247 (0.68), 7.318 (0.60), 8.119 (0.41), 8.142 (0.35), 8.548 (0.27), 8.674 (16.00), 11.816 (0.58).

### Example 912

### 5-{(3R)-1-[(1H-imidazol-2-yl)(1-methylcyclobutyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

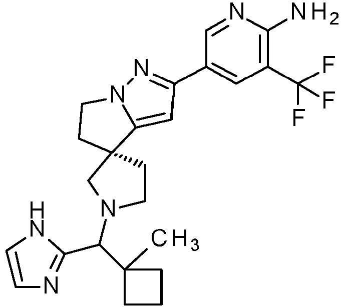

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (49.7 mg, 138 µmol) and (1H-imidazol-2-yl)(1-methylcyclobutyl)methanone (34.0 mg, 207 µmol, Intermediate 556) were dissolved in methanol (3 mL). N,N-Diisopropylethylamine (96 µL, 550 µmol) and titanium(IV) isopropoxide (120 µL, 410 µmol) were added and the mixture was stirred for 3 h at 60 °C. The reaction mixture was allowed to cool down, sodium cyanoborohydride (26.0 mg, 414 µmol) was added and the mixture was stirred over night at 60 °C. Titanium(IV) isopropoxide (120 µL, 410 µmol) and sodium cyanoborohydride (26.0 mg, 414 µmol) were added again and stirred over the weekened at 60 °C. The reaction mixture was allowed to cool down and the precipitate was filtered off. The filtrate was diluted with ethyl acetate and washed with a saturated sodium bicarbonate solution and water. The organic phase was washed with brine, dried and concentrated. The residue was purified by coloumn chromatography (silica gel NH2, hexane / ethyl acetatel (0 - 100 %) and ethyl acetate / ethanol (0 - 100 %) gradient). The combined fractions were purified by preparative HPLC to give 1.00 mg (90 % purity, 1 % yield) of the title compound.
LC-MS (Method 1): Rt = 1.11 min; MS (ESlpos): m/z = 472 [M+H]⁺
¹H-NMR (400 MHz, METHANOL-d4) δ [ppm]: 0.119 (4.98), 0.902 (2.90), 0.920 (3.16), 0.937 (1.71), 1.008 (0.57), 1.059 (0.83), 1.151 (1.88), 1.241 (1.45), 1.260 (2.42), 1.277 (2.11), 1.308 (12.30), 1.327 (4.67), 1.335 (3.93), 1.363 (15.86), 1.393 (16.00), 1.408 (2.19), 1.419 (1.94), 1.425 (1.96), 1.474 (2.08), 1.541 (0.94), 1.614 (0.91), 1.650 (0.85), 1.760 (1.65), 1.786 (1.82), 1.809 (1.00), 1.831 (0.94), 1.859 (1.57), 1.927 (0.54), 1.965 (0.97), 1.984 (3.10), 2.010 (1.94), 2.032 (3.96), 2.046 (2.05), 2.062 (2.33), 2.079 (1.77), 2.097 (1.62), 2.112 (1.71), 2.119 (2.22), 2.132 (2.65), 2.147 (2.93), 2.163 (2.28), 2.175 (1.74), 2.199 (2.05), 2.226 (2.33), 2.254 (1.59), 2.274 (0.97), 2.292 (0.60), 2.479 (2.14), 2.501 (2.39), 2.561 (2.14), 2.583 (3.56), 2.601 (2.05), 2.608 (1.54), 2.615 (2.70), 2.622 (2.73), 2.630 (3.44), 2.634 (3.30), 2.645 (3.33), 2.659 (1.91), 2.666 (2.25), 2.678 (2.82), 2.704 (0.57), 2.716 (1.08), 2.722 (1.14), 2.731 (1.51), 2.736 (1.82), 2.747 (3.27), 2.757 (1.48), 2.769 (3.42), 2.773 (3.36), 2.796 (2.19), 3.545 (4.93), 3.553 (4.87), 4.111 (0.85), 4.118 (0.54), 4.124 (1.17), 4.128 (1.00), 4.139 (1.31), 4.144 (1.68), 4.150 (1.45), 4.156 (1.85), 4.165 (2.31), 4.171 (2.96), 4.174 (2.45), 4.178 (2.45), 4.182 (2.08), 4.190 (1.79), 4.198 (1.77), 5.513 (7.60), 6.465 (8.88), 6.583 (8.85), 7.004 (12.67), 7.021 (11.64), 8.140 (3.47), 8.144 (5.49), 8.563 (3.02), 8.567 (2.96), 8.576 (2.68), 8.580 (2.56).

### Example 913

### (3R)-2'-{5-amino-6-[(1R)-1-phenylethoxy]pyrazin-2-yl}-N-ethyl-5',6'-dihydrospiro[pyrrolidine-3,4'-pyrrolo[1,2-b]pyrazole]-1-carboxamide

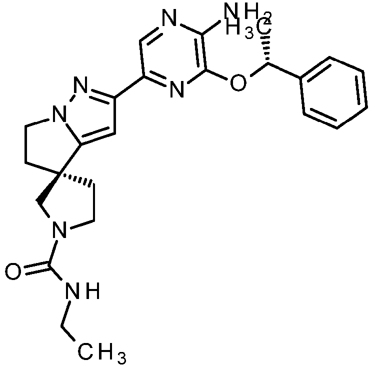

(3R)-1-(Ethylcarbamoyl)-5',6'-dihydrospiro[pyrrolidine-3,4'-pyrrolo[1,2-b]pyrazol]-2'-yl trifluoromethanesulfonate (43.0 mg, 112 µmol), 3-[(1R)-1-phenylethoxy]-5-(4,4,5,5-tetramethyl-1,3,2-dioxaborolan-2-yl)pyrazin-2-amine (42.2 mg, 124 µmol) and potassium phosphate (670 µL, 0.50 M, 340 µmol) were dissolved in degassed 1,4-dioxane (1.7 mL) under argon. XPhos Pd G2 (4.42 mg, 5.62 µmol) was added and the mixture was stirred for 2 h at 100 °C. The reaction mixture was allowed to cool down, diluted with water and extracted with ethyl acetate. The combined organic layers were washed with brine, dried over sodium sulfate, filtered and concentrated. The residue was purified by preparative HPLC to give 14.0 mg (95 % purity, 26 % yield) of the title compound.
LC-MS (Method 1): Rt = 0.98 min; MS (ESlpos): m/z = 448 [M+H]⁺
¹H-NMR (400 MHz, DMSO-d6) δ [ppm]: 1.026 (7.20), 1.044 (16.00), 1.062 (7.35), 1.232 (0.65), 1.600 (10.90), 1.617 (10.97), 2.007 (0.50), 2.020 (1.21), 2.036 (2.39), 2.055 (2.56), 2.074 (1.19), 2.086 (0.62), 2.529 (2.74), 3.051 (0.92), 3.069 (2.90), 3.083 (3.37), 3.086 (3.34), 3.100 (2.85), 3.118 (0.85), 3.373 (2.35), 3.384 (1.70), 3.398 (4.37), 3.410 (2.15), 3.427 (0.94), 3.445 (4.05), 3.470 (3.43), 3.487 (1.08), 3.494 (0.86), 3.512 (0.50), 4.136 (2.58), 4.153 (5.19), 4.171 (2.59), 6.166 (0.83), 6.183 (3.03), 6.199 (3.56), 6.207 (2.64), 6.215 (1.53), 6.221 (1.44), 6.234 (11.93), 6.244 (0.97), 6.344 (6.99), 7.231 (1.30), 7.249 (3.21), 7.264 (1.36), 7.268 (2.19), 7.333 (3.94), 7.352 (6.57), 7.367 (1.23), 7.371 (3.19), 7.508 (5.99), 7.525 (5.01), 7.528 (3.83), 7.904 (13.64).

### Example 914

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1 and diastereomer 2)

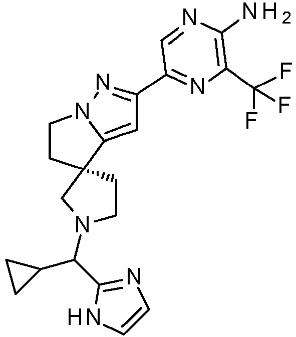

5-[(3R)-5',6'-Dihydrospiro[pyrrolidine-3,4'-pyrrolo[1,2-b]pyrazol]-2'-yl]-3-(trifluoromethyl)pyrazin-2-amine (64.0 mg, 197 µmol, Intermediate 535) and cyclopropyl(1H-imidazol-2-yl)methanone (40.3 mg, 296 µmol) were dissolved in methanol (3.4 mL). N,N-Diisopropylethylamine (140 µL, 0.79 mmol) and titanium(IV) isopropoxide (170 µL, 590 µmol) were added and stirred for 3 h at 60 °C. To the reaction mixture allowed to cool down, sodium cyanoborohydride (1.2 mL, 0.5 M, 0.59 mmol) was added and stirred for 16 h at 60 °C. The reaction mixture was allowed to cool down, diluted with water and stirred for 15 min. The suspension was filtered and washed with methanol. The filtrate was concentrated. The residue was purified by preparative HPLC to give 13.0 mg (92 % purity, 14 % yield) of the title compound.
LC-MS (Mehod 1): Rt = 0.94 min; MS (ESlpos): m/z = 446 [M+H]⁺
¹H-NMR (400 MHz, DMSO-d6) δ [ppm]: -0.023 (0.66), 0.063 (1.78), 0.075 (2.39), 0.087 (1.67), 0.100 (0.57), 0.279 (0.66), 0.302 (1.94), 0.315 (2.53), 0.321 (3.19), 0.338 (4.03), 0.350 (2.45), 0.362 (2.03), 0.374 (1.41), 0.384 (0.80), 0.597 (1.11), 0.618 (1.85), 0.628 (2.00), 0.640 (1.43), 0.851 (0.70), 1.188 (0.55), 1.201 (1.09), 1.212 (1.46), 1.224 (2.81), 1.232 (4.39), 1.263 (1.37), 1.276 (0.73), 1.751 (0.40), 1.904 (0.59), 1.911 (0.53), 1.930 (0.88), 1.944 (1.36), 1.957 (1.85), 1.968 (1.57), 1.986 (2.09), 2.001 (2.14), 2.014 (2.40), 2.020 (2.52), 2.034 (2.57), 2.046 (1.27), 2.052 (1.20), 2.066 (0.98), 2.291 (0.66), 2.318 (0.77), 2.323 (1.60), 2.327 (2.24), 2.331 (1.64), 2.337 (0.77), 2.469 (1.88), 2.518 (11.30), 2.523 (6.59), 2.565 (5.74), 2.576 (4.89), 2.585 (3.48), 2.607 (3.16), 2.624 (2.10), 2.644 (4.05), 2.668 (4.97), 2.675 (4.16), 2.699 (3.20), 2.748 (0.77), 2.771 (4.16), 2.786 (1.56), 2.794 (2.96), 2.807 (0.68), 2.895 (0.97), 2.909 (0.95), 2.916 (1.17), 2.931 (1.27), 2.942 (3.36), 2.965 (2.76), 3.159 (0.67), 3.171 (0.68), 4.077 (0.63), 4.085 (1.33), 4.104 (2.82), 4.119 (2.87), 4.127 (3.84), 4.144 (6.63), 4.162 (3.12), 6.386 (14.87), 6.393 (16.00), 6.437 (0.74), 6.749 (3.24), 6.898 (11.83), 7.012 (2.88), 8.716 (10.78), 11.817 (2.79).

### Example 915

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1)

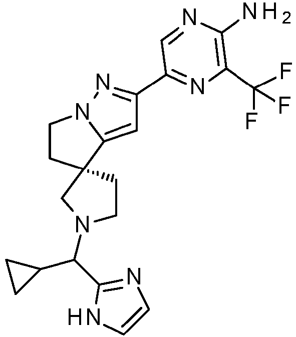

The compound 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1 and diastereomer 2, **Example 914**) was separated into diastereomers by preparative chiral HPLC to give 27.0 mg (92 % purity, 18 % yield) of the title compound (diastereomer 1 Rt = 6.1 - 7.3 min).

### Preparative chiral HPLC method:

Instrument: Sepiatec: Prep SFC100; Column: Chiral Art Cellulose SB 5µ, 250x50; eluent A: CO2; eluent B: 2-propanol + 0.4 vol % diethylamine; isocratic: 20%B; flow: 100 mL/min; temperature: 40 °C; BPR: 150 bar UV: 272 nm

### Analytical chiral HPLC method:

Instrument: Waters Acquity UPC2 QDA; Column: Chiral Art Cellulose SB 3µ, 100x4.6; eluent A: CO2; eluent B: 2-propanol + 0.4 vol % diethylamine; isocratic: 30%B; flow: 4 mL/min; temperature: 40 °C; BPR: 1800 psi UV: 280 nm
Analytical chiral HPLC Rt = 1.19 min
LC-MS (Method 1): Rt = 0.94 min; MS (ESlpos): m/z = 445 [M+H]⁺
¹H-NMR (400 MHz, DMSO-d6) δ [ppm]: 0.011 (0.59), 0.023 (1.08), 0.037 (1.23), 0.044 (0.80), 0.049 (0.69), 0.255 (0.50), 0.265 (0.82), 0.277 (1.16), 0.282 (2.10), 0.300 (2.54), 0.313 (1.12), 0.324 (0.69), 0.556 (0.66), 0.574 (0.98), 0.581 (0.83), 0.587 (0.93), 0.602 (0.52), 0.946 (0.89), 0.962 (0.98), 0.989 (14.51), 1.004 (15.14), 1.163 (0.47), 1.174 (0.62), 1.186 (1.15), 1.194 (1.66), 1.207 (1.21), 1.218 (0.65), 1.225 (0.62), 1.892 (0.59), 1.905 (0.98), 1.921 (1.22), 1.939 (0.76), 1.961 (0.72), 1.976 (0.96), 1.982 (1.10), 1.996 (1.00), 2.008 (0.59), 2.013 (0.53), 2.028 (0.42), 2.285 (0.56), 2.289 (0.82), 2.294 (0.59), 2.431 (0.77), 2.481 (4.26), 2.485 (2.37), 2.498 (1.19), 2.516 (3.57), 2.569 (1.84), 2.586 (1.26), 2.601 (1.61), 2.607 (3.29), 2.619 (0.73), 2.622 (0.67), 2.631 (3.70), 2.710 (0.60), 2.732 (1.29), 2.748 (1.09), 2.754 (0.72), 2.769 (0.47), 2.904 (2.55), 2.927 (2.24), 4.089 (2.48), 4.106 (5.09), 4.124 (2.43), 4.310 (0.57), 4.320 (0.57), 6.349 (0.44), 6.356 (16.00), 6.711 (1.25), 6.861 (5.93), 6.971 (1.21), 8.678 (6.01), 8.680 (5.84), 11.779 (1.00).

### Example 916

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 2)

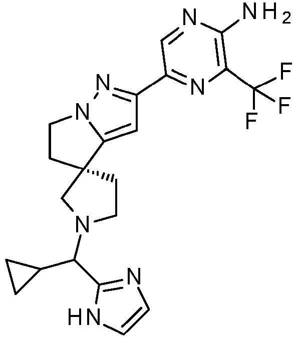

The diastereomers were separated by preparative chiral HPLC (method see **Example 915**) to give 22.0 mg (92 % purity, 15 % yield) of the title compound (diastereomer 2 Rₜ = 7.6 - 10.0 min).
Analytical chiral HPLC (method see **Example 915**): Rₜ = 1.92 min
LC-MS (Method 1): Rt = 0.94 min; MS (ESlpos): m/z = 445 [M+H]⁺
¹H-NMR (400 MHz, DMSO-d6) δ [ppm]: 0.011 (0.81), 0.023 (1.22), 0.035 (1.35), 0.047 (1.05), 0.059 (0.45), 0.253 (0.60), 0.262 (0.96), 0.274 (1.20), 0.284 (1.41), 0.296 (1.29), 0.307 (1.41), 0.320 (1.44), 0.333 (1.29), 0.343 (0.84), 0.554 (0.47), 0.564 (0.69), 0.576 (1.05), 0.586 (1.14), 0.598 (0.94), 0.609 (0.56), 0.809 (0.39), 0.822 (0.43), 0.942 (1.37), 0.958 (1.37), 0.985 (11.86), 1.001 (12.55), 1.010 (0.67), 1.028 (0.43), 1.160 (0.58), 1.171 (0.79), 1.181 (1.42), 1.191 (2.19), 1.203 (1.69), 1.221 (0.84), 1.913 (0.75), 1.918 (0.69), 1.927 (1.16), 1.944 (1.87), 1.961 (1.63), 1.973 (1.35), 1.979 (1.41), 1.992 (1.42), 2.004 (0.56), 2.011 (0.52), 2.024 (0.41), 2.280 (0.75), 2.286 (1.05), 2.290 (0.77), 2.426 (0.96), 2.476 (5.70), 2.481 (3.54), 2.500 (3.15), 2.510 (1.57), 2.524 (4.95), 2.561 (2.06), 2.576 (0.82), 2.618 (0.54), 2.623 (0.97), 2.627 (1.52), 2.635 (4.01), 2.659 (3.48), 2.730 (3.35), 2.754 (2.75), 2.854 (0.67), 2.867 (0.96), 2.874 (1.24), 2.890 (1.22), 2.909 (0.58), 4.032 (0.54), 4.035 (0.64), 4.044 (1.42), 4.051 (0.75), 4.063 (3.04), 4.077 (2.68), 4.089 (0.84), 4.097 (1.42), 4.104 (0.81), 4.110 (0.60), 4.124 (0.43), 4.305 (0.82), 4.315 (0.81), 6.344 (16.00), 6.352 (0.81), 6.709 (1.48), 6.853 (7.19), 6.974 (1.42), 8.671 (6.99), 11.777 (1.22).

### Example 917

### 5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)

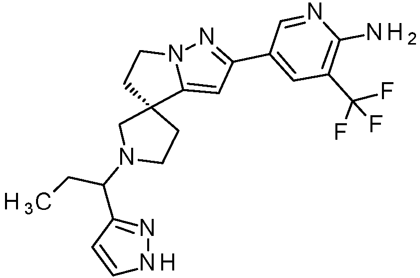

The compound 5-{(3R)-1-[1-(1H-pyrazol-3-yl)propyl]-5',6'-dihydrospiro[pyrrolidine-3,4'-pyrrol o[1,2-b]pyrazol]-2'-yl}-3-(trifluoromethyl) pyridin-2-amine (diastereomer 1 and diastereomer 2, **Example 900**) was separated into diastereomers by preparative chiral HPLC to give 37.0 mg (99 % purity) of the title compound (diastereomer 1 Rt = 18.15 - 23.58 min).

### Preparative chiral HPLC method:

Instrument: PrepCon Labomatic HPLC-2; Column: YMC Cellulose SB 5µ, 250x50; eluent A: hexane + 0.1 vol % diethylamine; eluent B: ethanol; isocratic: 30%B; flow: 90 mL/min; temperature: 25 °C; UV: 280 nm

### Analytical chiral HPLC method:

Instrument: Thermo Fisher UltiMate 3000; Column: YMC Cellulose SA 3µ, 100x4.6; eluent A: hexane + 0.1 vol % diethylamine; eluent B: ethanol; isocratic: 30%B; flow: 1.4 mL/min; temperature: 25 °C; UV: 280 nm
Analytical chiral HPLC Rₜ = 2.71 min
LC-MS (Method 1): Rt = 1.02 min; MS (ESlpos): m/z = 432 [M+H]⁺
¹H-NMR (400 MHz, CHLOROFORM-d) δ [ppm]: -0.125 (0.75), 0.014 (7.26), 0.033 (7.24), 0.042 (1.47), 0.170 (0.73), 0.776 (7.06), 0.784 (1.52), 0.794 (16.00), 0.813 (7.59), 0.820 (1.30), 0.825 (0.68), 0.832 (0.70), 0.839 (1.41), 0.850 (0.68), 0.854 (0.66), 0.858 (0.81), 0.864 (0.44), 0.871 (0.62), 0.881 (0.59), 1.101 (0.97), 1.125 (0.90), 1.167 (0.70), 1.175 (2.13), 1.182 (0.46), 1.192 (0.46), 1.210 (1.80), 1.318 (0.97), 1.356 (3.32), 1.517 (0.48), 1.535 (0.42), 1.679 (0.79), 1.697 (0.92), 1.701 (0.92), 1.713 (1.17), 1.731 (1.14), 1.734 (1.19), 1.753 (0.95), 1.845 (1.01), 1.857 (1.19), 1.863 (1.14), 1.876 (1.34), 1.890 (1.01), 1.898 (1.39), 1.914 (1.21), 1.918 (1.14), 1.931 (1.50), 1.946 (1.47), 1.951 (1.34), 1.966 (1.17), 2.041 (0.57), 2.061 (1.21), 2.075 (1.47), 2.081 (1.63), 2.096 (1.65), 2.107 (1.14), 2.113 (1.10), 2.128 (0.97), 2.456 (0.88), 2.472 (1.01), 2.476 (1.19), 2.488 (1.80), 2.504 (1.83), 2.508 (1.96), 2.524 (1.58), 2.568 (1.36), 2.582 (1.78), 2.586 (1.63), 2.601 (1.87), 2.634 (0.86), 2.653 (0.70), 2.675 (1.54), 2.686 (2.60), 2.709 (4.56), 2.739 (3.39), 2.763 (1.80), 2.788 (1.54), 2.804 (1.39), 2.826 (0.62), 3.474 (1.45), 3.486 (1.63), 3.496 (1.61), 3.508 (1.39), 4.070 (0.57), 4.085 (0.79), 4.089 (0.68), 4.097 (2.33), 4.105 (0.90), 4.113 (4.80), 4.116 (3.26), 4.127 (3.04), 4.132 (4.53), 4.140 (0.95), 4.148 (2.29), 4.155 (0.68), 4.160 (0.79), 4.175 (0.57), 4.970 (6.40), 6.110 (10.41), 6.162 (5.37), 6.167 (5.35), 7.491 (6.29), 7.496 (6.16), 8.057 (4.42), 8.061 (4.38), 8.553 (4.20), 8.557 (4.20).

### Example 918

### 5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)

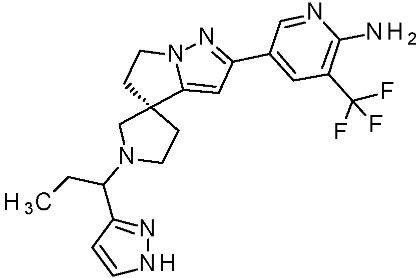

### For the preparation of the diastereomeric mixture of the title compound see Example 900. The diastereomers were separated by preparative chiral HPLC (method see Example 917Example 915

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1)

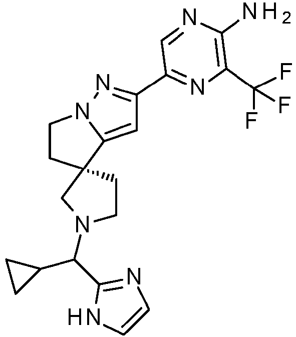

The compound 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1 and diastereomer 2, **Example 914**) was separated into diastereomers by preparative chiral HPLC to give 27.0 mg (92 % purity, 18 % yield) of the title compound (diastereomer 1 Rt = 6.1 - 7.3 min).

### Preparative chiral HPLC method:

Instrument: Sepiatec: Prep SFC100; Column: Chiral Art Cellulose SB 5µ, 250x50; eluent A: CO2; eluent B: 2-propanol + 0.4 vol % diethylamine; isocratic: 20%B; flow: 100 mL/min; temperature: 40 °C; BPR: 150 bar UV: 272 nm

### Analytical chiral HPLC method:

Instrument: Waters Acquity UPC2 QDA; Column: Chiral Art Cellulose SB 3µ, 100x4.6; eluent A: CO2; eluent B: 2-propanol + 0.4 vol % diethylamine; isocratic: 30%B; flow: 4 mL/min; temperature: 40 °C; BPR: 1800 psi UV: 280 nm
Analytical chiral HPLC Rt = 1.19 min
LC-MS (Method 1): Rt = 0.94 min; MS (ESlpos): m/z = 445 [M+H]⁺
¹H-NMR (400 MHz, DMSO-d6) δ [ppm]: 0.011 (0.59), 0.023 (1.08), 0.037 (1.23), 0.044 (0.80), 0.049 (0.69), 0.255 (0.50), 0.265 (0.82), 0.277 (1.16), 0.282 (2.10), 0.300 (2.54), 0.313 (1.12), 0.324 (0.69), 0.556 (0.66), 0.574 (0.98), 0.581 (0.83), 0.587 (0.93), 0.602 (0.52), 0.946 (0.89), 0.962 (0.98), 0.989 (14.51), 1.004 (15.14), 1.163 (0.47), 1.174 (0.62), 1.186 (1.15), 1.194 (1.66), 1.207 (1.21), 1.218 (0.65), 1.225 (0.62), 1.892 (0.59), 1.905 (0.98), 1.921 (1.22), 1.939 (0.76), 1.961 (0.72), 1.976 (0.96), 1.982 (1.10), 1.996 (1.00), 2.008 (0.59), 2.013 (0.53), 2.028 (0.42), 2.285 (0.56), 2.289 (0.82), 2.294 (0.59), 2.431 (0.77), 2.481 (4.26), 2.485 (2.37), 2.498 (1.19), 2.516 (3.57), 2.569 (1.84), 2.586 (1.26), 2.601 (1.61), 2.607 (3.29), 2.619 (0.73), 2.622 (0.67), 2.631 (3.70), 2.710 (0.60), 2.732 (1.29), 2.748 (1.09), 2.754 (0.72), 2.769 (0.47), 2.904 (2.55), 2.927 (2.24), 4.089 (2.48), 4.106 (5.09), 4.124 (2.43), 4.310 (0.57), 4.320 (0.57), 6.349 (0.44), 6.356 (16.00), 6.711 (1.25), 6.861 (5.93), 6.971 (1.21), 8.678 (6.01), 8.680 (5.84), 11.779 (1.00).

### Example 916

### 5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 2)

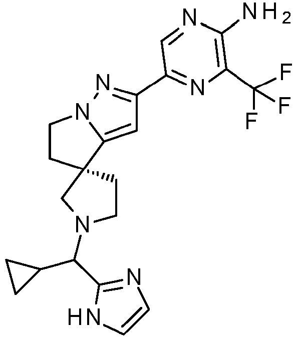

The diastereomers were separated by preparative chiral HPLC (method see **Example 915**) to give 22.0 mg (92 % purity, 15 % yield) of the title compound (diastereomer 2 Rt = 7.6 - 10.0 min).
Analytical chiral HPLC (method see **Example 915**): Rt = 1.92 min
LC-MS (Method 1): Rt = 0.94 min; MS (ESlpos): m/z = 445 [M+H]⁺
¹H-NMR (400 MHz, DMSO-d6) δ [ppm]: 0.011 (0.81), 0.023 (1.22), 0.035 (1.35), 0.047 (1.05), 0.059 (0.45), 0.253 (0.60), 0.262 (0.96), 0.274 (1.20), 0.284 (1.41), 0.296 (1.29), 0.307 (1.41), 0.320 (1.44), 0.333 (1.29), 0.343 (0.84), 0.554 (0.47), 0.564 (0.69), 0.576 (1.05), 0.586 (1.14), 0.598 (0.94), 0.609 (0.56), 0.809 (0.39), 0.822 (0.43), 0.942 (1.37), 0.958 (1.37), 0.985 (11.86), 1.001 (12.55), 1.010 (0.67), 1.028 (0.43), 1.160 (0.58), 1.171 (0.79), 1.181 (1.42), 1.191 (2.19), 1.203 (1.69), 1.221 (0.84), 1.913 (0.75), 1.918 (0.69), 1.927 (1.16), 1.944 (1.87), 1.961 (1.63), 1.973 (1.35), 1.979 (1.41), 1.992 (1.42), 2.004 (0.56), 2.011 (0.52), 2.024 (0.41), 2.280 (0.75), 2.286 (1.05), 2.290 (0.77), 2.426 (0.96), 2.476 (5.70), 2.481 (3.54), 2.500 (3.15), 2.510 (1.57), 2.524 (4.95), 2.561 (2.06), 2.576 (0.82), 2.618 (0.54), 2.623 (0.97), 2.627 (1.52), 2.635 (4.01), 2.659 (3.48), 2.730 (3.35), 2.754 (2.75), 2.854 (0.67), 2.867 (0.96), 2.874 (1.24), 2.890 (1.22), 2.909 (0.58), 4.032 (0.54), 4.035 (0.64), 4.044 (1.42), 4.051 (0.75), 4.063 (3.04), 4.077 (2.68), 4.089 (0.84), 4.097 (1.42), 4.104 (0.81), 4.110 (0.60), 4.124 (0.43), 4.305 (0.82), 4.315 (0.81), 6.344 (16.00), 6.352 (0.81), 6.709 (1.48), 6.853 (7.19), 6.974 (1.42), 8.671 (6.99), 11.777 (1.22).

Example 917) to give 40.0 mg (99 % purity) of the title compound (diastereomer 2 Rt = 25.51 - 31.68 min).
Analytical chiral HPLC (method see **Example 917**): Rt = 4.06 min
LC-MS (Method 1): Rt = 1.02 min; MS (ESlpos): m/z = 432 [M+H]⁺
¹H-NMR (400 MHz, CHLOROFORM-d) δ [ppm]: -0.125 (1.19), 0.015 (10.90), 0.033 (11.49), 0.042 (2.47), 0.170 (1.15), 0.777 (6.95), 0.796 (16.00), 0.814 (7.57), 0.825 (0.56), 0.832 (0.59), 0.839 (1.15), 0.850 (0.56), 0.854 (0.53), 0.858 (0.66), 0.871 (0.53), 0.882 (0.53), 1.101 (0.82), 1.125 (0.72), 1.167 (0.63), 1.175 (1.78), 1.210 (1.09), 1.318 (1.02), 1.356 (3.36), 1.516 (0.43), 1.679 (0.79), 1.697 (0.92), 1.702 (0.89), 1.713 (1.09), 1.720 (0.89), 1.731 (1.05), 1.735 (1.12), 1.754 (0.89), 1.849 (0.40), 1.867 (0.99), 1.880 (1.09), 1.885 (1.09), 1.898 (1.25), 1.913 (0.79), 1.918 (0.79), 1.931 (0.72), 1.937 (0.89), 1.956 (1.09), 1.969 (1.25), 1.987 (1.65), 2.006 (1.09), 2.047 (0.69), 2.062 (1.22), 2.075 (1.45), 2.083 (1.58), 2.095 (2.07), 2.106 (1.02), 2.115 (0.95), 2.127 (0.89), 2.458 (0.76), 2.473 (0.89), 2.478 (0.99), 2.490 (1.65), 2.505 (1.74), 2.510 (1.84), 2.526 (1.48), 2.553 (1.38), 2.567 (1.68), 2.573 (1.61), 2.586 (1.91), 2.599 (0.95), 2.641 (2.30), 2.663 (5.07), 2.674 (4.31), 2.698 (0.95), 2.896 (0.69), 2.909 (0.92), 2.917 (1.25), 2.929 (1.19), 2.939 (0.76), 2.951 (0.63), 3.463 (1.25), 3.475 (1.42), 3.486 (1.35), 3.498 (1.22), 4.044 (0.76), 4.059 (0.89), 4.063 (0.99), 4.071 (2.11), 4.079 (0.99), 4.086 (2.24), 4.091 (2.40), 4.101 (2.17), 4.106 (2.07), 4.115 (2.37), 4.122 (2.21), 4.129 (1.05), 4.135 (2.04), 4.142 (0.99), 4.149 (0.92), 4.163 (0.79), 4.972 (5.73), 6.069 (8.59), 6.161 (5.20), 6.166 (5.20), 7.497 (6.06), 7.502 (5.93), 8.049 (4.12), 8.054 (4.18), 8.543 (4.02), 8.547 (3.95).

### Example 919

### 5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

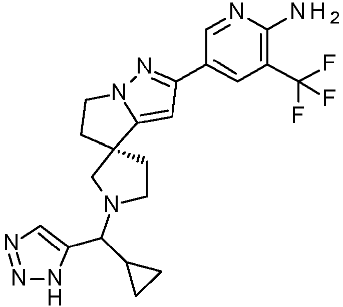

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (414 mg, 93 % purity, 1.07 mmol) and cyclopropyl(1H-1,2,3-triazol-5-yl)methanone (220 mg, 1.61 mmol) were dissolved in methanol (11 mL). N,N-Diisopropylethylamine (930 µL, 5.4 mmol) and titanium(IV) isopropoxide (950 µL, 3.2 mmol) were added and stirred over night at 60 °C. The reaction mixture was allowed to cool down, sodium cyanoborohydride (168 mg, 2.68 mmol) was added and stirred for 2 h at 60 °C, for 2 h at 80 °C and overnight at room temperature. The reaction mixture was diluted with water and stirred for 10 min. The suspension was filtered and the residue was washed with methanol. The filtrate was concentrated. The aqueous residue was extracted with ethyl acetate. The combined organic layers were washed with brine, dried and concentrated. The crude product was purified by preparative HPLC to give 176 mg (95 % purity, 35 % yield) of the title compound.
LC-MS (Method 1): Rₜ = 0.73 min; MS (ESlpos): m/z = 445 [M+H]⁺
¹H-NMR (400 MHz, DMSO-d6) δ [ppm]: -0.099 (0.64), 0.291 (2.28), 0.303 (2.81), 0.322 (2.42), 0.339 (1.64), 0.351 (1.81), 0.362 (1.64), 0.373 (1.42), 0.598 (2.10), 0.801 (0.53), 1.163 (2.13), 1.181 (2.95), 1.892 (0.71), 1.907 (1.56), 1.932 (3.31), 1.941 (4.94), 1.963 (3.02), 2.272 (1.42), 2.277 (2.03), 2.281 (1.49), 2.398 (1.32), 2.415 (2.77), 2.467 (10.63), 2.472 (7.18), 2.489 (2.38), 2.516 (2.52), 2.567 (3.09), 2.596 (3.38), 2.619 (5.65), 2.648 (3.56), 2.671 (1.67), 2.729 (1.35), 2.762 (1.78), 2.787 (2.60), 2.825 (2.49), 2.847 (1.96), 2.878 (1.32), 4.003 (2.03), 4.021 (3.59), 4.032 (5.23), 4.049 (7.40), 4.067 (2.99), 6.367 (3.88), 6.386 (6.36), 6.416 (0.43), 6.465 (16.00), 7.702 (0.71), 7.956 (6.04), 7.961 (9.64), 7.967 (4.98), 8.542 (9.14).

### Example 920

### 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 1 and diastereomer 2)

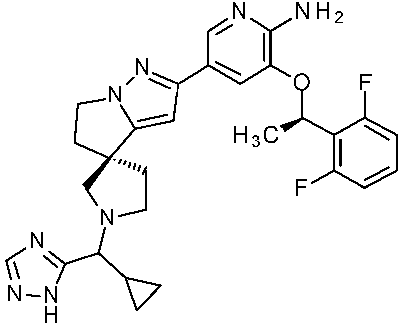

3-[(1R)-1-(2,6-Difluorophenyl)ethoxy]-5-[(3R)-5',6'-dihydrospiro[pyrrolidine-3,4'-pyrrolo[1,2-b]pyrazol]-2'-yl]pyridin-2-amine hydrogen chloride (1/1) (120 mg, 268 µmol) and cyclopropyl(1H-1,2,4-triazol-5-yl)methanone (55.1 mg, 402 µmol) were dissolved in methanol (2.8 mL). N,N-Diisopropylethylamine (140 µL, 800 µmol) and titanium(IV) isopropoxide (240 µL, 800 µmol) were added and stirred for 1.5 h at 60 °C. The reaction mixture was allowed to cool down, sodium cyanoborohydride (50.5 mg, 804 µmol) was added and stirred for 16 h at 60 °C. The reaction mixture was allowed to cool down, water (0.5 mL) was added and stirred for 15 min. The suspension was filtered and the filter cake was washed with methanol. The filtrate was concentrated. The residue was purified by preparative HPLC to give 50.0 mg (94 % purity, 33 % yield) of the title compound.
LC-MS (Method 1): Rt = 0.94 min; MS (ESlpos): m/z = 533 [M+H]⁺
¹H NMR (DMSO-d₆, 400 MHz): δ (ppm) 8.04 (s, 1H), 7.89 (d, 1H), 7.35 - 7.44 (m, 1H), 7.31 - 7.34 (m, 1H), 7.06 - 7.15 (m, 2H), 6.17 - 6.21 (m, 1H), 5.79 - 5.86 (m, 1H), 5.64 (s, 2H), 3.98 - 4.10 (m, 2H), 2.92 - 3.00 (m, 1H), 2.83 - 2.90 (m, 1H), 2.74 - 2.81 (m, 1H), 2.54 - 2.66 (m, 3H), 2.39 - 2.46 (m, 1H), 1.87 - 2.03 (m, 2H), 1.75 (d, 3H), 1.21 - 1.32 (m, 1H), 0.58 - 0.68 (m, 1H), 0.29 - 0.43 (m, 2H), 0.04 - 0.13 (m, 1H).

### Example 921

### 5-{(3R)-1-[(1-methylcyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diasteroemr 1 and diastereomer 2)

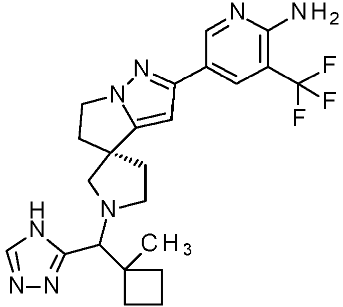

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (97.3 mg, 270 µmol) and (1-methylcyclobutyl)(4H-1,2,4-triazol-3-yl)methanone (67.0 mg, 406 µmol, Intermediate 572) were dissolved in methanol (5.8 mL). N,N-Diisopropylethylamine (190 µL, 1.1 mmol) and titanium(IV) isopropoxide (240 µL, 810 µmol) were added and stirred for 3 h at 60 °C. The reaction mixture was allowed tocool down, sodium cyanoborohydride (51.0 mg, 811 µmol) was added and the mixture was stirred over the weekend at 60 °C. The reaction mixture was allowed to cool down, filtered and the filtrate was diluted with ethyl acetate. The organic phase was washed with saturated sodium bicarbonate solution, water and brine, dried and concentrated. The residue was purified by coloumn chromatography (silica gel NH2, hexane / ethyl acetate (0 - 100 %) and ethyl acetate / ethanol (0 - 100 %) gradient) followed by preparative HPLC to give 22.6 mg (90 % purity, 16 % yield) of the title compound.
LC-MS (Method 1): Rt = 1.05 min; MS (ESlpos): m/z = 473 [M+H]⁺
¹H-NMR (400 MHz, DMSO-d6) δ [ppm]: 1.195 (0.66), 1.223 (3.99), 1.241 (8.28), 1.258 (4.30), 1.300 (0.61), 1.401 (3.51), 1.429 (4.30), 1.671 (0.48), 1.693 (0.57), 1.789 (0.53), 1.919 (0.44), 1.942 (0.83), 1.963 (0.88), 1.980 (1.01), 1.999 (1.49), 2.018 (1.10), 2.040 (0.66), 2.056 (16.00), 2.064 (0.61), 2.092 (0.88), 2.117 (1.23), 2.142 (1.18), 2.166 (0.66), 2.357 (0.92), 2.386 (0.83), 2.391 (1.84), 2.395 (2.63), 2.400 (1.84), 2.405 (0.79), 2.511 (1.49), 2.586 (10.26), 2.591 (7.98), 2.604 (1.36), 2.620 (1.88), 2.636 (1.23), 2.653 (0.88), 2.672 (0.57), 2.728 (1.18), 2.733 (2.19), 2.737 (2.98), 2.742 (2.41), 2.747 (2.24), 2.769 (1.01), 2.852 (0.75), 2.875 (0.66), 3.669 (2.06), 3.692 (2.28), 4.068 (1.10), 4.085 (3.38), 4.103 (3.33), 4.121 (1.27), 4.132 (0.66), 4.143 (0.88), 4.151 (0.96), 4.158 (1.40), 4.167 (0.92), 4.175 (1.71), 4.193 (0.83), 4.822 (0.48), 6.507 (3.33), 6.521 (0.66), 6.535 (0.88), 6.591 (4.08), 7.447 (0.39), 7.463 (0.44), 7.486 (0.66), 8.066 (2.41), 8.646 (2.32).

### Example 922

### 5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)

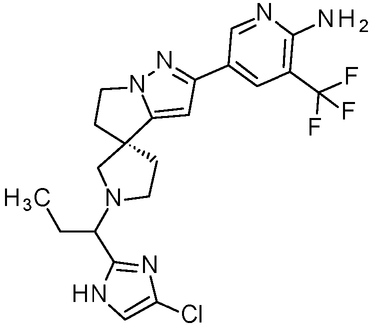

The compound 5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2, **Example 909**) was separated into diastereomers by preparative chiral HPLC to give 15.0 mg (98 % purity, 37 % yield) of the title compound (diastereomer 1 Rt = 13.8 - 16.8 min).

### Preparative chiral HPLC method:

Instrument: Sepiatec: Prep SFC100; Column: Chiral Art Amylose SA 5µ, 250x50; eluent A: CO2; eluent B: methanol + 0.2 vol % aqueous ammonia (32 %); isocratic: 10%B; flow: 100 mL/min; temperature: 40 °C; BPR: 150 bar; UV: 267 nm

### Analytical chiral HPLC method:

Instrument: Waters acquity UPC2 QDA; Column: Chiral Art Amylose SA 3µ, 100x4.6; eluent A: CO2; eluent B: methanol + 0.2 vol % aqueous ammonia (32 %); isocratic: 10%B; flow: 4 mL/min; temperature: 40 °C; BPR: 1800 psi; UV: 280 nm
Analytical chiral HPLC Rt = 3.96 min
Specific Rotation: 150,13
LC-MS (Method 1): Rt = 1.04 min; MS (ESlpos): m/z = 466 [M+H]⁺
¹H-NMR (400 MHz, CHLOROFORM-d) δ [ppm]: 0.830 (7.06), 0.848 (16.00), 0.867 (7.58), 1.047 (0.41), 1.265 (0.85), 1.426 (0.57), 1.631 (11.10), 1.727 (0.75), 1.746 (1.07), 1.763 (1.20), 1.781 (1.25), 1.799 (0.81), 1.931 (1.07), 1.941 (1.24), 1.949 (1.26), 1.960 (1.41), 1.976 (1.05), 1.983 (1.00), 1.994 (1.50), 2.009 (1.10), 2.014 (1.22), 2.027 (1.62), 2.041 (1.41), 2.047 (1.36), 2.061 (1.12), 2.173 (1.10), 2.188 (1.62), 2.194 (1.52), 2.209 (1.57), 2.221 (1.11), 2.226 (1.04), 2.242 (0.85), 2.548 (0.70), 2.568 (1.01), 2.580 (1.79), 2.596 (2.04), 2.600 (2.17), 2.628 (1.46), 2.642 (1.87), 2.647 (1.82), 2.661 (2.00), 2.674 (0.90), 2.679 (0.89), 2.696 (1.19), 2.711 (3.17), 2.734 (5.20), 2.756 (0.91), 2.797 (3.38), 2.820 (2.04), 2.905 (0.96), 2.927 (1.63), 2.941 (1.52), 2.964 (0.69), 3.516 (1.61), 3.526 (1.82), 3.537 (1.77), 3.548 (1.53), 4.138 (0.64), 4.157 (0.96), 4.166 (2.17), 4.174 (1.00), 4.185 (3.35), 4.202 (4.14), 4.209 (2.48), 4.216 (1.13), 4.223 (2.18), 4.230 (0.86), 4.236 (0.79), 4.250 (0.62), 5.011 (7.75), 5.310 (11.88), 6.207 (11.33), 6.887 (6.56), 7.007 (0.41), 7.529 (0.41), 8.116 (5.03), 8.120 (5.09), 8.612 (4.84), 8.616 (4.78), 9.193 (0.95).

### Example 923

### 5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)

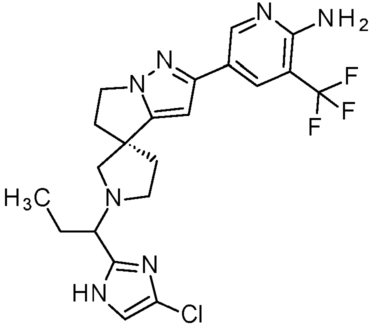

For the preparation of the diastereomeric mixture of the title compound see **Example 909.** The diastereomers were separated by preparative chiral HPLC (method see **Example 922**) to give 13.7 mg (98 % purity, 34 % yield) of the title compound (diastereomer 2 Rₜ = 17.1 - 20.0 min).
Analytical chiral HPLC (method see **Example 922**): Rt = 4.97 min
Specific Rotation: 36,99
LC-MS (Method 1): Rt = 1.04 min; MS (ESlpos): m/z = 466 [M+H]⁺
¹H-NMR (400 MHz, CHLOROFORM-d) δ [ppm]: 0.841 (7.15), 0.859 (16.00), 0.877 (7.65), 1.265 (0.80), 1.634 (12.31), 1.718 (0.42), 1.736 (0.78), 1.754 (1.09), 1.770 (1.29), 1.791 (1.34), 1.810 (0.92), 1.932 (1.13), 1.943 (1.29), 1.950 (1.31), 1.962 (1.48), 1.978 (1.06), 1.984 (0.99), 1.997 (0.84), 2.010 (0.83), 2.031 (1.10), 2.043 (1.59), 2.059 (1.72), 2.079 (1.25), 2.130 (1.19), 2.144 (1.58), 2.150 (1.65), 2.164 (1.81), 2.176 (1.03), 2.182 (1.06), 2.197 (0.82), 2.546 (0.57), 2.561 (0.78), 2.565 (0.83), 2.578 (2.20), 2.593 (2.38), 2.597 (2.69), 2.640 (2.79), 2.647 (3.55), 2.670 (3.98), 2.734 (0.88), 2.755 (1.78), 2.772 (1.80), 2.793 (0.97), 2.874 (1.02), 2.895 (2.15), 2.905 (4.76), 2.929 (3.71), 3.519 (1.80), 3.530 (2.04), 3.541 (1.94), 3.552 (1.72), 4.132 (0.55), 4.147 (0.78), 4.151 (0.80), 4.159 (2.39), 4.166 (1.09), 4.174 (4.83), 4.178 (3.62), 4.194 (4.76), 4.202 (1.11), 4.210 (2.37), 4.218 (0.83), 4.221 (0.84), 4.237 (0.55), 5.008 (7.89), 5.310 (12.43), 6.171 (11.84), 6.923 (7.18), 7.007 (0.41), 7.529 (0.43), 8.107 (5.26), 8.112 (5.33), 8.604 (5.00), 8.607 (4.94), 9.256 (1.04).

### Example 924

### 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 1 and diastereomer 2)

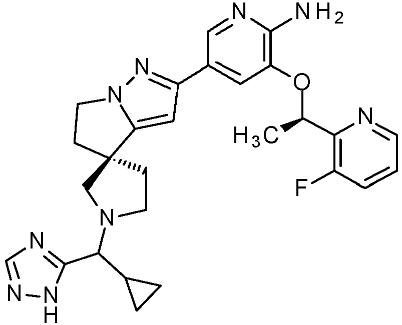

5-[(3R)-5',6'-Dihydrospiro[pyrrolidine-3,4'-pyrrolo[1,2-b]pyrazol]-2'-yl]-3-[(1R)-1-(3-fluoropyridin-2-yl)ethoxy]pyridin-2-amine hydrogen chloride (1/1) (76.0 mg, 176 µmol) and cyclopropyl(1H-1,2,4-triazol-5-yl)methanone (36.3 mg, 265 µmol) were dissolved in methanol (1.9 mL). N,N-Diisopropylethylamine (120 µL, 710 µmol) and titanium(IV) isopropoxide (160 µL, 530 µmol) were added and stirred for 1.5 h at 60 °C. The reaction mixture was allowed to cool down, sodium cyanoborohydride (33.3 mg, 529 µmol) was added and the mixture was stirred for 16 h at 60 °C. The reaction mixture was allowed to cool down, diluted with water (0.5 mL) and stirred for 15 min. The suspension was filtered and washed with methanol. The filtrate was concentrated. The residue was purified by preparative HPLC to give 43.1 mg (99 % purity, 47 % yield) of the title compound.
LC-MS (Method 1): Rt = 0.80 min; MS (ESlpos): m/z = 516 [M+H]⁺
¹H NMR (DMSO-d₆, 400 MHz): δ (ppm) 13.80 (br s, 1H), 8.44 (dt, 1H), 7.89 (d, 1H), 7.74 (ddd, 1H), 7.42 - 7.49 (m, 1H), 7.32 (t, 1H), 6.20 (s, 1H), 5.74 - 5.81 (m, 1H), 5.66 (s, 2H), 3.98 - 4.10 (m, 2H), 2.92 - 3.00 (m, 1H), 2.86 (t, 1H), 2.73 - 2.81 (m, 1H), 2.53 - 2.69 (m, 3H), 2.40 - 2.47 (m, 1H), 1.88 - 2.04 (m, 2H), 1.69 (d, 3H), 1.21 - 1.32 (m, 1H), 0.60 - 0.69 (m, 1H), 0.31 - 0.45 (m, 2H), 0.04 - 0.13 (m, 1H).

### Example 925

### 5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)

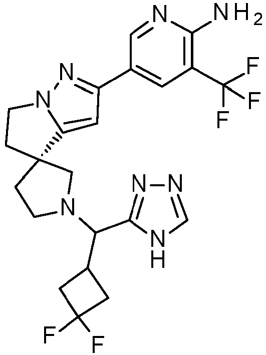

The compound 5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2, **Example 910**) was separated into diastereomers by preparative chiral HPLC to give 20.0 mg (6 % yield) of the title compound (diastereomer 1: Rt = 5.2 - 6.5 min).

### Preparative chiral HPLC method:

Instrument: Sepiatec: Prep SFC100; Column: Chiralpak IG 5µ, 250x50; eluent A: CO2; eluent B: methanol + 0.2 vol % aqueous ammonia (32 %); isocratic: 20%B; flow: 100 mL/min; temperature: 40 °C; BPR: 150 bar; UV: 271 nm

### Analytical chiral HPLC method:

Instrument: Waters acquity UPC2 QDA; Column: Chiralpak IG 3µ, 100x4.6; eluent A: CO2; eluent B: methanol + 0.2 vol % aqueous ammonia (32 %); isocratic: 20%B; flow: 4 mL/min; temperature: 40 °C; BPR: 1800 psi; UV: 280 nm
Analytical chiral HPLC Rt = 1.83 min
¹H-NMR (400 MHz, DMSO-d6) δ [ppm]: 0.898 (0.71), 0.916 (1.55), 0.935 (0.78), 1.232 (0.78), 1.276 (2.46), 1.294 (2.46), 1.363 (6.28), 1.885 (1.49), 1.904 (1.62), 1.921 (1.10), 2.074 (2.46), 2.105 (0.71), 2.144 (0.78), 2.318 (1.23), 2.323 (2.79), 2.327 (3.95), 2.331 (2.85), 2.336 (1.23), 2.356 (0.45), 2.374 (0.97), 2.390 (1.36), 2.406 (2.07), 2.425 (1.55), 2.450 (2.20), 2.518 (16.00), 2.523 (11.34), 2.660 (2.33), 2.665 (3.95), 2.669 (5.31), 2.673 (4.34), 2.678 (2.85), 2.724 (2.14), 2.745 (2.14), 2.777 (4.73), 2.799 (3.43), 3.857 (0.97), 4.066 (3.43), 4.084 (7.00), 4.101 (3.30), 5.582 (0.45), 5.600 (0.39), 6.302 (13.41), 6.513 (8.55), 7.264 (0.65), 7.284 (0.52), 7.360 (0.45), 7.507 (0.45), 7.988 (5.25), 7.993 (5.31), 8.564 (4.92), 8.567 (4.86), 13.929 (1.55).

### Example 926

### 5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

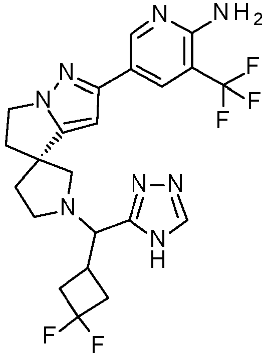

The diastereomers were separated by preparative chiral HPLC (method see **Example 925**) to give 17.0 mg (6 % yield) of the title compound (diastereomer 2 Rₜ = 9.0 - 10.6 min).
Analytical chiral HPLC (method see **Example 925**): Rt = 3.19 min
LC-MS (Method 1): Rt = 0.89 min; MS (ESlpos): m/z = 495 [M+H]⁺
¹H-NMR (400 MHz, DMSO-d6) δ [ppm]: 1.233 (0.67), 1.877 (1.00), 1.894 (2.07), 1.912 (1.13), 2.336 (1.20), 2.385 (0.47), 2.400 (0.73), 2.418 (1.47), 2.435 (1.27), 2.449 (1.93), 2.518 (16.00), 2.523 (11.47), 2.624 (1.33), 2.646 (1.73), 2.678 (1.53), 2.687 (0.73), 2.709 (0.80), 2.728 (1.67), 2.750 (1.27), 2.791 (0.80), 2.838 (0.53), 2.855 (0.87), 2.877 (0.73), 3.863 (0.47), 4.061 (1.60), 4.079 (3.33), 4.096 (1.53), 6.251 (6.47), 6.514 (4.00), 7.983 (2.33), 7.988 (2.40), 8.556 (2.20), 8.560 (2.20).

### Example 927

### 5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)

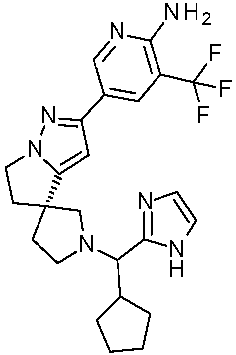

5-[(3R)-5',6'-Dihydrospiro[pyrrolidine-3,4'-pyrrolo[1,2-b]pyrazol]-2'-yl]-3-(trifluoromethyl)pyridin-2-amine hydrogen chloride (1/1) (80.0 mg, 222 µmol) and cyclopentyl(1H-imidazol-2-yl)methanone (54.8 mg, 334 µmol, Intermediate 571) were provided in methanol (1.9 mL). N,N-Diisopropylethylamine (160 µL, 890 µmol) and titanium(IV) isopropoxide (200 µL, 670 µmol) were added and stirred for 2 h at 60 °C. Sodium cyanoborohydride (41.9 mg, 667 µmol) was added and the mixture was stirred for 3 days at 60 °C. 5-[(3R)-5',6'-dihydrospiro[pyrrolidine-3,4'-pyrrolo[1,2-b]pyrazol]-2'-yl]-3-(trifluoromethyl)pyridin-2-amine hydrogen chloride (1/1) (40.0 mg, 111 µmol) was added and the reaction mixture was stirred for 7 days at 60 °C. The reaction mixture was allowed to cool down, diluted with ethyl acetate and washed with a saturated aqueous sodium bicarbonate solution and brine. The organic phase was dried over a hydrophobic filter and concentrated. The residue was purified by coloumn chromatography (silica gel, dichloromethane / ethanol gradient) to give 53.5 mg (46 % yield) of the title compound.
LC-MS (Method ): Rt = 1.08 min; MS (ESlpos): m/z = 472 [M+H]⁺
¹H-NMR (400 MHz, DMSO-d6) δ [ppm]: 0.798 (0.81), 0.803 (0.43), 0.815 (0.85), 0.822 (0.89), 0.840 (0.50), 0.886 (0.43), 0.904 (0.89), 0.922 (0.58), 0.968 (0.46), 1.035 (8.85), 1.053 (16.00), 1.070 (9.20), 1.137 (2.13), 1.232 (1.39), 1.368 (0.46), 1.386 (0.58), 1.404 (0.66), 1.422 (0.85), 1.437 (0.93), 1.455 (1.08), 1.472 (1.12), 1.504 (1.55), 1.832 (0.58), 1.846 (1.12), 1.865 (1.51), 1.877 (0.85), 1.907 (0.43), 2.115 (0.89), 2.323 (0.77), 2.327 (1.08), 2.332 (0.81), 2.336 (0.43), 2.371 (0.43), 2.388 (0.77), 2.394 (0.54), 2.406 (0.50), 2.412 (0.73), 2.431 (0.58), 2.441 (0.73), 2.449 (0.70), 2.458 (0.73), 2.466 (0.85), 2.518 (4.52), 2.523 (3.25), 2.620 (0.46), 2.639 (1.24), 2.660 (1.86), 2.669 (1.62), 2.673 (1.00), 2.679 (0.54), 2.685 (0.46), 2.731 (0.93), 2.753 (0.62), 2.790 (0.97), 2.812 (1.00), 2.831 (0.54), 2.850 (0.50), 3.404 (1.28), 3.417 (1.35), 3.422 (3.75), 3.435 (3.86), 3.440 (3.40), 3.452 (3.48), 3.457 (1.35), 3.469 (1.24), 3.483 (1.66), 3.509 (1.51), 4.057 (1.20), 4.074 (2.43), 4.091 (1.16), 4.347 (2.36), 4.359 (4.56), 4.372 (2.20), 6.148 (3.21), 6.220 (3.63), 6.511 (3.79), 6.844 (0.54), 7.036 (0.50), 7.975 (2.20), 8.549 (2.01), 11.759 (0.62).

### Example 928

### 5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)

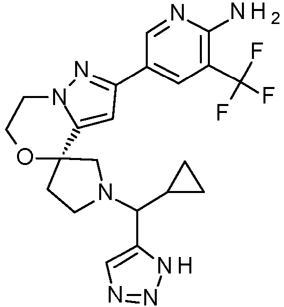

5-[(3'R)-6,7-Dihydrospiro[pyrazolo[5,1-c][1,4]oxazine-4,3'-pyrrolidin]-2-yl]-3-(trifluoromethyl)pyridin-2-amine hydrogen chloride (1/1) (462 mg, 93 % purity, 1.14 mmol) and cyclopropyl(1H-1,2,3-triazol-5-yl)methanone (235 mg, 1.71 mmol) were dissolved in methanol (12 mL). N,N-Diisopropylethylamine (990 µL, 5.7 mmol) and titanium(IV) isopropoxide (1.0 mL, 3.4 mmol) were added and stirred for 2 h at 60 °C. The reaction mixture was allowed to cool down, sodium cyanoborohydride (179 mg, 2.86 mmol) was added and stirred over night at 60 °C. The reaction mixture was allowed to cool down, diluted with water and stirred for 10 min. The suspension was filtered and the residue was washed with methanol. The filtrate was concentrated. The aqueous residue was extracted with ethyl acetate. The combined organic layers were washed with brine, dried and concentrated. The crude product was purified by preparative HPLC to give 129 mg (95 % purity, 23 % yield) of the title compound.
LC-MS (Method 1): Rt = 0.76 min; MS (ESlpos): m/z = 461 [M+H]⁺
¹H-NMR (400 MHz, DMSO-d6) δ [ppm]: 0.011 (2.23), 0.311 (2.28), 0.373 (2.23), 0.624 (2.08), 0.816 (0.63), 1.174 (2.33), 1.196 (3.54), 2.039 (1.26), 2.049 (1.60), 2.064 (2.23), 2.081 (2.91), 2.099 (1.75), 2.137 (2.42), 2.153 (1.89), 2.288 (2.23), 2.292 (3.01), 2.297 (2.23), 2.483 (12.90), 2.488 (8.34), 2.505 (0.87), 2.630 (4.90), 2.634 (5.43), 2.639 (3.88), 2.650 (2.38), 2.667 (0.92), 2.714 (2.62), 2.740 (2.81), 2.774 (3.10), 2.796 (5.33), 2.818 (2.86), 2.872 (5.62), 2.902 (1.55), 2.924 (1.21), 3.087 (2.86), 3.112 (2.47), 3.598 (0.48), 3.913 (0.68), 3.931 (1.31), 3.947 (2.86), 3.960 (4.17), 3.972 (4.02), 3.982 (5.19), 3.993 (6.21), 4.015 (4.70), 4.025 (8.48), 4.033 (6.79), 6.537 (16.00), 6.592 (8.73), 7.696 (0.63), 8.001 (9.55), 8.006 (9.79), 8.580 (9.41), 8.584 (9.41).

### Example 929

### 5-{(3R)-1-[cyclopropyl(1-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)

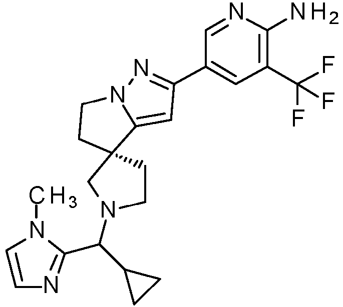

5-{(3R)-1-[Cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (stereoisomer 2) (50.0 mg, 113 µmol, **Example 591**) was dissolved in DMF (910 µL) under argon. Potassium carbonate (18.7 mg, 135 µmol) and methyliodide (8.4 µL, 140 µmol) were added and the reaction mixture was stirred for 4 h at rt. Potassium carbonate (7.79 mg, 56.4 µmol) and methyliodide (3.5 µL, 56 µmol) were added and the reaction mixture was stirred for 1 h at rt. Water was added and the aqueous phase was extracted three times with dichloromethane. The combined organic layers were washed with brine and dried through a hydrophobic filter. It was concentrated and purified by HPLC to yield 6.50 mg (98 % purity, 12 % yield) of the title compound.
[α]²⁰_{D}: +32.6° (c = 1.00, methanol)
LC-MS (Method 1): Rₜ = 1.04 min; MS (ESlpos): m/z = 458 [M+H]⁺
¹H NMR (400 MHz, DMSO-*d*₆, 22°C) δ ppm 0.04 (dq, 1 H), 0.29 - 0.45 (m, 2 H), 0.62 - 0.71 (m, 1 H), 1.25 - 1.36 (m, 1 H), 1.95 - 2.08 (m, 2 H), 2.54 - 2.60 (m, 2 H), 2.81 (dd, 2 H), 2.88 - 2.96 (m, 1 H), 3.80 (s, 3 H), 4.03 - 4.14 (m, 2 H), 6.41 (s, 1 H), 6.52 (s, 2 H), 6.71 (d, 1 H), 7.03 (d, 1 H), 8.00 (d, 1 H), 8.58 (d, 1 H).

In analogy to the procedures described above, the following examples were prepared using the appropriate intermediates as starting materials.

**Table 2: Example 930-973**

| **Example** | **Structure IUPAC-Name NMR** | **Yield Analytics** |
|---|---|---|
| **Example 930** | 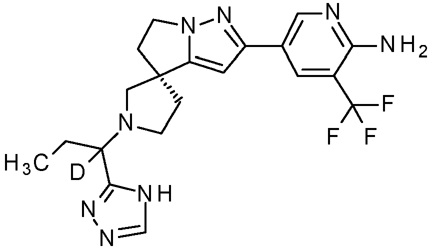 | (90 % purity, 54 % yield) |
| | | LC-MS (Method 1): Rₜ = 0.80 min; |
| | 5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | MS (ESlpos): m/z = 434 [M+H]⁺ |
| | ¹H-NMR (400 MHz, DMSO-d6) δ [ppm]: 0.758 (3.19), 0.775 (6.69), 0.793 (3.84), 0.814 (1.20), 0.821 (1.19), 0.840 (0.56), 0.886 (0.48), 0.904 (0.99), 0.922 (0.55), 1.035 (8.42), 1.052 (16.00), 1.070 (8.79), 1.089 (0.45), 1.159 (0.56), 1.196 (1.60), 1.212 (1.69), 1.241 (2.27), 1.258 (3.09), 1.274 (1.56), 1.871 (1.57), 1.907 (2.64), 1.928 (2.50), 2.323 (1.22), 2.327 (1.74), 2.331 (1.25), 2.412 (0.70), 2.450 (1.08), 2.518 (10.69), 2.523 (7.52), 2.665 (2.81), 2.669 (3.09), 2.673 (2.30), 2.799 (0.87), 3.404 (1.00), 3.417 (1.12), 3.422 (2.72), 3.435 (2.81), 3.439 (2.70), 3.452 (2.76), 3.457 (1.01), 3.469 (0.94), 4.065 (1.62), 4.073 (2.02), 4.082 (3.40), 4.090 (3.71), 4.099 (2.06), 4.108 (1.79), 4.345 (1.51), 4.358 (2.97), 4.370 (1.46), 6.317 (1.19), 6.343 (1.41), 6.519 (7.87), 7.890 (1.08), 7.992 (4.78), 8.514 (0.47), 8.565 (4.20), 13.830 (1.04). | |
| **Example 931** | 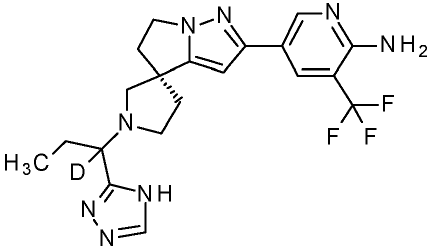 | (98 % purity, 9 % yield) |
| | | Rₜ = 4.68 min; Instrument: Thermo Fisher UltiMate 3000; Column: YMC Amylose SA 3µ, 100x4.6; eluent A: hexane + 0.1 vol % diethylamine; eluent B: ethanol; isocratic: 80%A+20%B; flow: 1.4 mL/min; temperature: 25°C; UV: 280 nm; |
| | 5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 2) | |
| | Instrument: PrepCon Labomatic HPLC-3; Column: YMC Amylose SA 5µ, 250x30; eluent A: hexane + 0.1 vol %; diethylamine; eluent B: ethanol ; isocratic: 80%A+20%B; flow: 80 mL/min; temperature: 25°C; UV: 280 nm; | |
| | ¹H-NMR (400 MHz, DMSO-d6) δ [ppm]: 0.762 (6.65), 0.780 (16.00), 0.798 (7.06), 1.021 (0.54), 1.039 (1.03), 1.057 (0.57), 1.801 (0.80), 1.819 (1.25), 1.834 (2.65), 1.853 (2.66), 1.859 (3.08), 1.877 (2.98), 1.896 (3.89), 1.913 (6.34), 1.931 (3.37), 2.075 (1.13), 2.322 (1.31), 2.327 (1.90), 2.332 (1.37), 2.394 (0.65), 2.411 (1.40), 2.425 (1.79), 2.444 (2.89), 2.462 (2.96), 2.518 (7.28), 2.523 (5.16), 2.614 (1.01), 2.632 (2.70), 2.654 (2.98), 2.672 (14.54), 2.850 (1.06), 2.866 (2.06), 2.889 (2.13), 2.906 (0.86), 4.061 (3.55), 4.078 (7.24), 4.097 (3.61), 6.301 (14.83), 6.513 (10.41), 6.564 (0.58), 7.988 (5.93), 7.993 (6.09), 8.133 (0.70), 8.561 (5.57), 8.565 (5.46), 13.763 (0.21), 13.771 (0.22), 13.776 (0.22), 13.779 (0.23), 13.787 (0.24), 13.790 (0.24), 13.797 (0.24), 13.804 (0.25), 13.816 (0.24), 13.822 (0.24), 13.832 (0.22). | |
| **Example 932** | 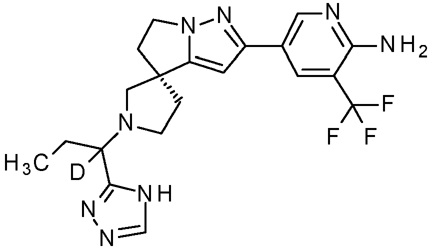 | (100 % purity, 16 % yield) |
| | | Rₜ = 0.83 min; Instrument: Thermo Fisher UltiMate 3000; Column: YMC Amylose SA 3µ, 100x4.6; eluent A: hexane + 0.1 vol % diethylamine; eluent B: ethanol; isocratic: 80%A+20%B; flow: 1.4 mL/min; temperature: 25°C; UV: 280 nm; |
| | 5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1) | |
| | Instrument: PrepCon Labomatic HPLC-3; Column: YMC Amylose SA 5µ, 250x30; eluent A: hexane + 0.1 vol %; diethylamine; eluent B: ethanol ; isocratic: 80%A+20%B; flow: 80 mL/min; temperature: 25°C; UV: 280 nm; | |
| | ¹H-NMR (400 MHz, DMSO-d6) δ [ppm]: 0.758 (6.95), 0.777 (16.00), 0.795 (7.29), 1.231 (0.42), 1.801 (0.64), 1.819 (1.25), 1.835 (3.13), 1.853 (3.99), 1.870 (3.19), 1.896 (3.17), 1.914 (3.07), 1.932 (1.61), 2.318 (0.74), 2.322 (1.61), 2.327 (2.36), 2.332 (1.68), 2.336 (0.73), 2.383 (0.53), 2.401 (1.30), 2.416 (1.59), 2.433 (2.55), 2.450 (1.46), 2.518 (9.93), 2.523 (7.24), 2.606 (2.40), 2.628 (2.84), 2.644 (0.97), 2.665 (3.45), 2.669 (3.82), 2.673 (2.60), 2.679 (2.03), 2.683 (2.03), 2.703 (0.83), 2.768 (1.07), 2.789 (2.21), 2.809 (5.27), 2.832 (3.53), 4.070 (4.69), 4.087 (9.79), 4.104 (4.56), 6.333 (11.23), 6.513 (11.34), 6.547 (0.63), 7.992 (6.88), 7.997 (6.90), 8.566 (6.22), 8.570 (6.04), 13.807 (1.76). | |
| **Example 933** | 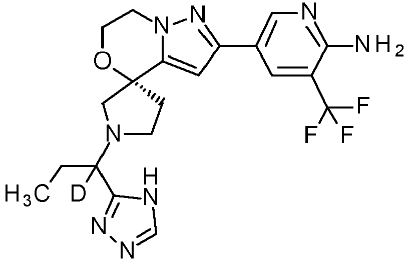 | (98 % purity, 2 % yield) |
| | | LC-MS (Method 1): Rₜ = 0.82 min; MS (ESlpos): m/z = 450 [M+H]⁺ |
| | 5-{(3'R)-1'-[1-(4H-1,2,4-triazol-3-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2) | |
| | ¹H-NMR (400 MHz, DMSO-d6) δ [ppm]: 0.729 (4.93), 0.733 (4.28), 0.747 (11.56), 0.751 (9.12), 0.765 (5.51), 1.232 (0.77), 1.752 (0.60), 1.798 (0.49), 1.814 (0.93), 1.831 (2.17), 1.849 (2.85), 1.860 (1.88), 1.866 (2.06), 1.878 (1.56), 1.905 (0.61), 2.017 (0.57), 2.030 (1.03), 2.049 (1.34), 2.062 (1.71), 2.074 (0.98), 2.081 (1.36), 2.095 (1.46), 2.110 (1.52), 2.125 (1.06), 2.142 (0.59), 2.318 (0.83), 2.323 (1.87), 2.327 (2.61), 2.332 (1.80), 2.337 (0.83), 2.518 (10.04), 2.523 (6.88), 2.621 (0.56), 2.640 (1.71), 2.660 (2.99), 2.665 (3.08), 2.669 (3.38), 2.674 (2.83), 2.679 (1.81), 2.692 (0.49), 2.781 (2.13), 2.807 (3.09), 2.820 (2.11), 2.830 (1.54), 2.840 (1.43), 2.871 (0.76), 2.897 (1.86), 2.914 (2.71), 2.940 (1.08), 3.056 (2.91), 3.082 (2.61), 3.963 (3.24), 3.973 (4.73), 3.986 (3.32), 4.044 (4.34), 4.053 (5.44), 6.509 (3.44), 6.529 (5.94), 6.568 (10.11), 8.019 (6.37), 8.024 (6.39), 8.204 (16.00), 8.592 (5.49), 8.595 (5.25). | |
| **Example 934** | 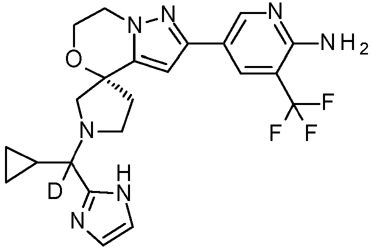 | (90 % purity, 19 % yield) |
| | | LC-MS (Method 1): Rₜ = 0.89 min; MS (ESlpos): m/z = 461 [M+H]⁺ |
| | 5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diatereomer 1 and diastereomer 2) | |
| | ¹H-NMR (400 MHz, DMSO-d6) δ [ppm]: -0.048 (1.37), 0.053 (1.15), 0.063 (1.37), 0.076 (1.39), 0.310 (1.09), 0.321 (1.34), 0.331 (1.57), 0.335 (1.53), 0.350 (1.82), 0.357 (1.78), 0.370 (1.64), 0.381 (1.21), 0.393 (0.65), 0.639 (1.28), 0.798 (0.67), 0.802 (0.41), 0.814 (0.72), 0.821 (0.74), 0.886 (0.41), 0.904 (0.79), 1.035 (2.02), 1.052 (4.31), 1.070 (2.45), 1.196 (0.40), 1.208 (0.87), 1.229 (2.07), 1.243 (1.71), 2.081 (0.78), 2.097 (1.17), 2.114 (1.66), 2.131 (1.04), 2.157 (1.35), 2.173 (1.19), 2.180 (0.93), 2.190 (0.70), 2.336 (0.82), 2.518 (16.00), 2.523 (11.36), 2.568 (0.81), 2.586 (1.59), 2.607 (1.39), 2.624 (0.59), 2.669 (2.70), 2.673 (1.97), 2.678 (1.28), 2.684 (2.21), 2.780 (1.18), 2.794 (0.59), 2.807 (2.09), 2.829 (0.88), 2.923 (1.78), 2.949 (1.79), 2.974 (0.63), 3.184 (1.89), 3.210 (1.70), 3.422 (0.90), 3.435 (0.92), 3.440 (0.88), 3.452 (0.89), 3.959 (0.56), 3.973 (1.12), 3.992 (1.44), 4.004 (1.67), 4.020 (2.62), 4.031 (3.03), 4.051 (1.98), 4.063 (4.48), 4.072 (2.97), 4.345 (0.57), 4.358 (1.09), 4.370 (0.55), 6.579 (7.90), 6.622 (6.55), 6.628 (9.01), 6.794 (0.40), 8.027 (4.38), 8.032 (4.46), 8.613 (4.52), 8.618 (4.43), 11.889 (1.35). | |
| **Example 935** | 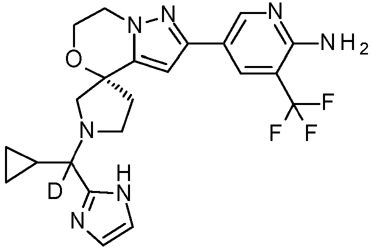 | (98 % purity, 18 % yield) |
| | | Rₜ = 1.72 min; Instrument: Thermo Fisher UltiMate 3000; Column: YMC Amylose SA 3µ, 100x4.6; eluent A: hexane + 0.1 vol % diethylamine; eluent B: 2-propanol; isocratic: 60%B+40%B; flow: 1.4 mL/min; temperature: 25°C; UV: 280 nm; |
| | 5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diastereomer 1) | |
| | Instrument: PrepCon Labomatic HPLC-3; Column: YMC Amylose SA 5µ, 250x30; eluent A: hexane + 0.1 vol %; diethylamine; eluent B: 2-propanol; isocratic: 60%A+40%B; flow: 60 mL/min; temperature: 25°C; UV: 280 nm; | |
| | ¹H-NMR (400 MHz, DMSO-d6) δ [ppm]: 0.030 (0.51), 0.041 (1.13), 0.053 (1.69), 0.065 (1.85), 0.077 (1.44), 0.089 (0.63), 0.288 (0.42), 0.302 (0.80), 0.312 (1.31), 0.323 (1.61), 0.334 (1.88), 0.345 (1.73), 0.356 (1.98), 0.369 (2.00), 0.381 (1.81), 0.392 (1.18), 0.406 (0.43), 0.614 (0.62), 0.623 (0.95), 0.635 (1.42), 0.645 (1.55), 0.658 (1.28), 0.669 (0.80), 0.680 (0.50), 1.210 (0.79), 1.223 (1.69), 1.231 (2.74), 1.243 (2.63), 1.255 (1.49), 1.263 (1.35), 1.276 (0.61), 2.066 (0.54), 2.085 (0.96), 2.100 (1.77), 2.115 (1.95), 2.133 (1.26), 2.145 (1.09), 2.163 (2.11), 2.180 (1.84), 2.196 (1.02), 2.213 (0.55), 2.518 (9.00), 2.523 (5.99), 2.562 (1.00), 2.582 (1.81), 2.601 (1.88), 2.620 (0.84), 2.779 (3.21), 2.805 (4.14), 2.922 (4.88), 2.933 (1.29), 2.949 (5.16), 2.972 (1.70), 2.989 (0.77), 3.941 (0.82), 3.958 (1.42), 3.973 (2.90), 3.979 (2.23), 3.991 (3.49), 4.004 (2.32), 4.025 (1.14), 4.028 (1.06), 4.036 (0.93), 4.050 (4.22), 4.063 (5.43), 6.578 (9.50), 6.622 (16.00), 6.765 (0.77), 7.032 (0.79), 8.028 (5.65), 8.033 (5.78), 8.613 (5.34), 8.618 (5.29), 11.870 (1.84). | |
| | [α]²⁰_{D}: +53.0° (c = 1.00, methanol | |
| **Example 936** | 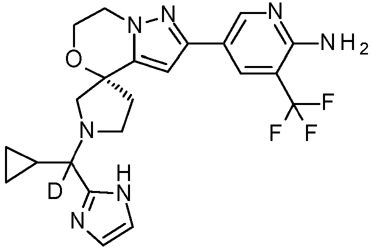 | (99 % purity, 20 % yield) |
| | | Rₜ = 3.11 min; Instrument: Thermo Fisher UltiMate 3000; Column: YMC Amylose SA 3µ, 100x4.6; eluent A: hexane + 0.1 vol % diethylamine; eluent B: 2-propanol; isocratic: 60%B+40%B; flow: 1.4 mL/min; temperature: 25°C; UV: 280 nm; |
| | 5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diastereomer 2) | |
| | Instrument: PrepCon Labomatic HPLC-3; Column: YMC Amylose SA 5µ, 250x30; eluent A: hexane + 0.1 vol %; diethylamine; eluent B: 2-propanol; isocratic: 60%A+40%B; flow: 60 mL/min; temperature: 25°C; UV: 280 nm; | |
| | ¹H-NMR (400 MHz, DMSO-d6) δ [ppm]: -0.049 (1.28), 0.038 (0.48), 0.049 (1.06), 0.062 (1.65), 0.074 (1.82), 0.084 (1.41), 0.096 (0.61), 0.285 (0.41), 0.298 (0.80), 0.309 (1.28), 0.320 (1.60), 0.330 (2.18), 0.335 (1.58), 0.349 (2.70), 0.357 (2.08), 0.369 (1.77), 0.380 (1.16), 0.393 (0.43), 0.609 (0.59), 0.619 (0.96), 0.631 (1.46), 0.639 (1.51), 0.653 (1.28), 0.664 (0.75), 0.674 (0.51), 0.794 (0.65), 0.844 (0.41), 0.861 (0.51), 1.006 (0.56), 1.034 (0.57), 1.083 (1.30), 1.196 (0.76), 1.208 (1.41), 1.216 (1.60), 1.229 (3.48), 1.248 (1.52), 1.259 (2.36), 2.061 (0.56), 2.074 (1.40), 2.081 (1.01), 2.095 (1.65), 2.112 (2.07), 2.130 (1.33), 2.140 (1.16), 2.157 (2.06), 2.173 (1.82), 2.189 (0.94), 2.206 (0.55), 2.323 (1.07), 2.327 (1.49), 2.331 (1.09), 2.518 (6.17), 2.523 (4.08), 2.568 (0.85), 2.586 (1.88), 2.608 (1.96), 2.625 (0.81), 2.658 (4.09), 2.665 (1.59), 2.669 (1.79), 2.673 (1.52), 2.684 (4.13), 2.793 (0.94), 2.813 (1.75), 2.831 (1.64), 2.850 (0.69), 3.183 (3.78), 3.209 (3.37), 4.020 (4.62), 4.031 (5.14), 4.063 (5.08), 4.072 (4.28), 4.076 (4.21), 6.578 (9.35), 6.627 (16.00), 6.792 (0.46), 6.927 (0.42), 8.026 (5.58), 8.031 (5.60), 8.613 (5.21), 8.617 (5.16), 10.856 (0.66), 11.891 (1.30). | |
| **Example 937** | 5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2) | (90 % purity, 43 % yield) |
| | | LC-MS: (Method 1): Rₜ = 0.92 min; MS (ESlpos): m/z = 449 [M+H]⁺ |
| | ¹H-NMR (400 MHz, DMSO-d6) δ [ppm]: 0.692 (3.62), 0.699 (2.85), 0.711 (8.35), 0.717 (5.54), 0.729 (4.06), 0.736 (2.52), 0.806 (1.80), 0.825 (3.99), 0.843 (1.90), 1.034 (0.75), 1.052 (1.64), 1.070 (0.82), 1.205 (2.72), 1.220 (3.31), 1.236 (5.67), 1.246 (12.80), 1.253 (12.37), 1.262 (16.00), 1.284 (9.41), 1.750 (0.56), 1.769 (0.70), 1.784 (0.76), 1.803 (0.99), 1.820 (1.26), 1.838 (1.33), 1.858 (1.17), 1.876 (1.62), 1.894 (1.58), 1.909 (1.15), 2.053 (1.04), 2.067 (1.32), 2.086 (1.57), 2.145 (1.22), 2.322 (1.82), 2.326 (2.39), 2.331 (1.69), 2.518 (14.99), 2.522 (10.25), 2.664 (2.48), 2.668 (2.99), 2.673 (2.24), 2.751 (0.89), 2.777 (1.01), 2.849 (1.04), 3.113 (1.89), 3.601 (1.55), 3.980 (2.51), 3.992 (3.20), 4.006 (2.28), 4.061 (3.77), 6.507 (3.76), 6.540 (7.04), 6.580 (7.25), 7.065 (4.84), 7.084 (3.14), 8.014 (4.34), 8.595 (4.35), 8.688 (0.41), 8.692 (0.41), 8.694 (0.40), 8.699 (0.37), 12.580 (0.15), 12.589 (0.15), 12.608 (0.14), 12.618 (0.14), 12.658 (0.14), 12.672 (0.14), 12.677 (0.14), 12.693 (0.14), 12.696 (0.14), 12.703 (0.14). | |
| **Example 938** | 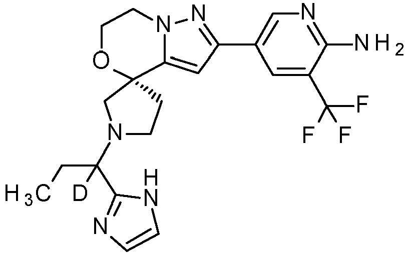 | (97 % purity, 16 % yield) |
| | | Rₜ = 1.76 min; Instrument: Thermo Fisher UltiMate 3000; Column: YMC Amylose SA 3µ, 100x4.6; eluent A: hexane + 0.1 vol % |
| | 5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1) | |
| | Instrument: PrepCon Labomatic HPLC-3; Column: YMC Amylose SA 5µ, 250x30; eluent A: hexane + 0.1 vol %; diethylamine; eluent B: 2-propanol; isocratic: 70%A+30%B; flow: 70 mL/min; temperature: 25°C; UV: 280 nm; | |
| | ¹H-NMR (400 MHz, DMSO-d6) δ [ppm]: 0.700 (6.73), 0.719 (16.00), 0.736 (7.01), 0.775 (0.96), 0.794 (2.21), 0.813 (1.18), 0.818 (0.98), 0.836 (1.03), 0.843 (0.89), 0.861 (1.36), 0.871 (0.45), 0.880 (0.58), 1.006 (1.82), 1.026 (2.82), 1.033 (1.63), 1.042 (2.87), 1.083 (4.39), 1.099 (0.58), 1.142 (4.57), 1.161 (9.36), 1.179 (4.59), 1.232 (1.69), 1.258 (5.55), 1.352 (1.22), 1.391 (0.56), 1.405 (0.46), 1.421 (0.59), 1.439 (0.48), 1.443 (0.48), 1.788 (0.57), 1.807 (1.01), 1.826 (1.22), 1.841 (2.03), 1.859 (2.16), 1.880 (2.35), 1.899 (2.16), 1.914 (1.29), 1.931 (0.90), 2.021 (0.62), 2.039 (1.28), 2.054 (1.43), 2.072 (2.11), 2.090 (1.08), 2.130 (0.98), 2.148 (1.63), 2.163 (1.63), 2.180 (1.07), 2.195 (0.64), 2.318 (0.76), 2.323 (1.57), 2.327 (2.23), 2.331 (1.61), 2.336 (0.75), 2.518 (8.70), 2.523 (5.80), 2.660 (1.75), 2.665 (2.67), 2.669 (3.28), 2.673 (2.63), 2.888 (2.60), 2.927 (2.38), 2.994 (1.33), 3.942 (0.57), 3.957 (1.59), 3.971 (3.86), 3.982 (4.89), 3.992 (2.93), 4.011 (0.69), 4.022 (0.81), 4.049 (4.64), 4.061 (6.77), 4.073 (2.64), 6.510 (13.98), 6.584 (10.00), 6.621 (0.46), 7.094 (7.95), 8.012 (5.91), 8.017 (6.18), 8.591 (5.53), 8.595 (5.67), 10.854 (0.56). | diethylamine; eluent B: 2-propanol; isocratic: 70%B+30%B; flow: 1.4 mL/min; temperature: 25°C; UV: 280 nm; |
| **Example 939** | 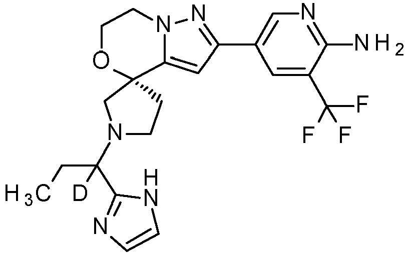 | (99 % purity, 23 % yield) |
| | | Rₜ = 3.54 min; Instrument: Thermo Fisher UltiMate 3000; Column: YMC Amylose SA 3µ, 100x4.6; eluent A: hexane + 0.1 vol % |
| | 5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 2) | |
| | Instrument: PrepCon Labomatic HPLC-3; Column: YMC Amylose SA 5µ, 250x30; eluent A: hexane + 0.1 vol %; diethylamine; eluent B: 2-propanol; isocratic: 70%A+30%B; flow: 70 mL/min; temperature: 25°C; UV: 280 nm; | |
| | ¹H-NMR (400 MHz, DMSO-d6) δ [ppm]: 0.686 (6.11), 0.705 (14.76), 0.723 (6.54), 0.775 (1.01), 0.794 (2.41), 0.813 (1.19), 0.818 (0.93), 0.836 (1.11), 0.843 (0.92), 0.861 (1.46), 0.880 (0.64), 0.984 (0.37), 1.006 (2.03), 1.026 (11.91), 1.034 (2.11), 1.042 (11.76), 1.083 (4.64), 1.100 (0.59), 1.136 (0.38), 1.154 (0.66), 1.172 (0.39), 1.232 (0.98), 1.259 (6.24), 1.352 (0.59), 1.391 (0.60), 1.421 (0.67), 1.440 (0.52), 1.527 (0.39), 1.609 (0.36), 1.758 (1.26), 1.776 (1.53), 1.791 (2.46), 1.810 (2.15), 1.840 (2.15), 1.859 (2.28), 1.873 (1.48), 1.892 (1.08), 2.019 (0.49), 2.038 (1.13), 2.052 (1.43), 2.070 (2.63), 2.074 (2.40), 2.089 (2.18), 2.107 (1.85), 2.121 (1.76), 2.137 (0.74), 2.518 (7.08), 2.523 (4.64), 2.564 (0.82), 2.582 (2.00), 2.605 (2.08), 2.622 (0.77), 2.707 (3.54), 2.733 (3.92), 2.775 (0.89), 2.795 (1.62), 2.810 (1.52), 2.829 (0.70), 3.033 (3.61), 3.059 (3.11), 3.764 (0.34), 3.990 (4.56), 4.003 (4.21), 4.049 (4.31), 4.062 (4.96), 4.348 (0.81), 4.358 (0.82), 6.519 (16.00), 6.574 (9.13), 6.937 (2.74), 8.016 (5.44), 8.021 (5.45), 8.598 (5.09), 8.601 (4.99), 10.518 (0.65), 10.638 (0.60), 10.854 (1.71), 11.902 (0.28), 11.910 (0.30), 11.915 (0.30), 11.919 (0.31), 11.921 (0.31), 11.925 (0.32), 11.929 (0.31), 11.950 (0.29). | diethylamine; eluent B: 2-propanol; isocratic: 70%B+30%B; flow: 1.4 mL/min; temperature: 25°C; UV: 280 nm; |
| **Example 940** | 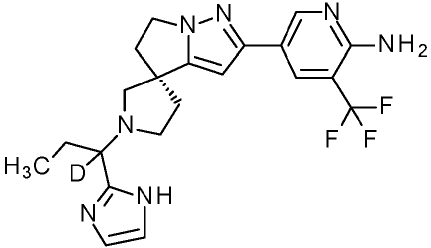 | (90 % purity, 58 % yield) |
| | | LC-MS: (Method 1): Rₜ = 0.93 min; MS (ESIpos): m/z = 433 [M+H]⁺ |
| | 5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: 0.716 (3.72), 0.723 (3.87), 0.735 (8.79), 0.741 (8.25), 0.753 (4.34), 0.759 (3.80), 0.810 (1.51), 0.829 (3.74), 0.847 (1.73), 1.196 (0.74), 1.204 (1.82), 1.213 (0.76), 1.220 (2.06), 1.235 (4.16), 1.247 (7.75), 1.253 (11.09), 1.263 (11.25), 1.283 (5.93), 1.715 (0.50), 1.733 (0.44), 1.762 (0.52), 1.781 (0.54), 1.815 (0.46), 1.832 (0.66), 1.848 (0.95), 1.866 (1.37), 1.884 (1.32), 1.907 (2.51), 1.928 (2.20), 1.943 (3.44), 1.960 (4.31), 1.976 (2.28), 2.323 (2.01), 2.327 (2.73), 2.331 (1.96), 2.416 (0.51), 2.428 (1.09), 2.448 (1.37), 2.461 (2.03), 2.518 (16.00), 2.523 (12.56), 2.660 (1.32), 2.665 (2.48), 2.669 (3.32), 2.673 (2.56), 2.678 (1.57), 2.716 (1.28), 2.959 (0.87), 3.114 (1.74), 3.133 (1.83), 3.164 (8.77), 3.585 (1.33), 3.602 (1.55), 3.618 (1.26), 4.072 (1.56), 4.082 (3.66), 4.098 (5.20), 4.115 (2.52), 6.321 (3.67), 6.359 (4.35), 6.532 (9.52), 7.149 (5.91), 7.181 (2.26), 7.986 (5.30), 8.135 (0.70), 8.567 (5.23). | |
| **Example 941** | 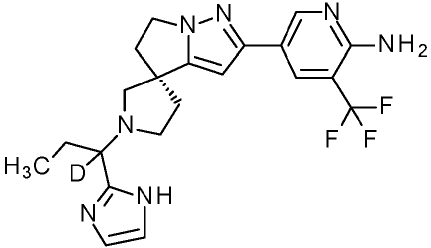 | (100 % purity, 30 % yield) |
| | | Rₜ = 3.29 min; Instrument: Waters Acquity UPC2 QDA; Column: Chiral Art Amylose-SA 3µ 100x4.6mm; eluent A: CO2; eluent B: methanol + 0.2 vol % aqueous ammonia (32%); isocratic: 10%B; gradient: no; flow: 4 mL/min; temperature: 40.0°C; BPR: 1800 psi; UV: 280 nm |
| | 5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1) | |
| | Instrument: Sepiatec: Prep SFC100; Column: Chiral Art Amylose-SA 5µ 250x30mm; eluent A: CO2; eluent B: methanol + 0.2 vol % aqueous ammonia (32%); isocratic: 10%B; gradient: no; flow: 100 mL/min; temperature: 40°C; BPR: 150 bar; UV: 267 nm. | |
| | ¹H-NMR (400 MHz, DMSO-d6) δ [ppm]: 0.715 (5.89), 0.733 (14.25), 0.752 (6.31), 0.817 (0.63), 0.868 (0.41), 1.765 (0.88), 1.783 (1.22), 1.798 (2.31), 1.816 (2.21), 1.829 (2.39), 1.848 (2.44), 1.862 (1.40), 1.866 (1.40), 1.882 (2.06), 1.902 (3.02), 1.918 (2.82), 1.938 (1.55), 1.949 (0.56), 1.953 (0.60), 1.969 (0.43), 2.385 (0.59), 2.402 (1.38), 2.417 (1.55), 2.435 (2.30), 2.452 (1.48), 2.472 (2.26), 2.518 (6.47), 2.523 (4.71), 2.569 (4.24), 2.597 (1.59), 2.615 (1.82), 2.634 (0.83), 2.747 (0.93), 2.768 (1.96), 2.784 (1.73), 2.796 (4.34), 2.819 (3.46), 3.298 (0.76), 4.064 (3.20), 4.081 (6.16), 4.099 (3.15), 6.328 (16.00), 6.335 (0.88), 6.514 (8.98), 6.814 (6.81), 7.034 (3.88), 7.038 (5.19), 7.041 (4.03), 7.989 (4.99), 7.994 (5.08), 8.568 (4.67), 8.573 (4.55), 11.776 (2.48). | |
| | [α]²⁰_{D}: +69.3° (c = 1.00, methanol) | |
| **Example 942** | 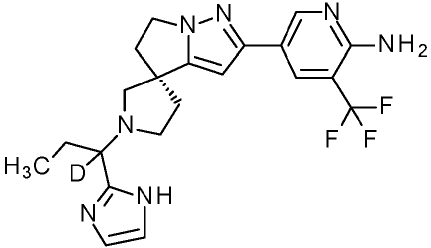 | (99 % purity, 30 % yield) |
| | | Rₜ = 4.90 min; Instrument: Waters Acquity UPC2 QDA; Column: Chiral Art Amylose-SA 3µ 100x4.6mm; eluent A: CO2; eluent B: methanol + 0.2 vol % aqueous ammonia (32%); isocratic: 10%B; gradient: no; flow: 4 mL/min; temperature: 40.0°C; BPR: 1800 psi; UV: 280 nm |
| | 5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 2) | |
| | Instrument: Sepiatec: Prep SFC100; Column: Chiral Art Amylose-SA 5µ 250x30mm; eluent A: CO2; eluent B: methanol + 0.2 vol % aqueous ammonia (32%); isocratic: 10%B; gradient: no; flow: 100 mL/min; temperature: 40°C; BPR: 150 bar; UV: 267 nm. | |
| | ¹H-NMR (400 MHz, DMSO-d6) δ [ppm]: 0.722 (5.35), 0.740 (13.18), 0.758 (5.71), 1.759 (0.86), 1.777 (1.13), 1.793 (2.06), 1.811 (2.05), 1.832 (2.15), 1.851 (2.08), 1.866 (1.22), 1.884 (1.05), 1.903 (2.24), 1.918 (4.54), 1.937 (2.62), 2.318 (0.41), 2.322 (0.92), 2.326 (1.29), 2.332 (0.91), 2.394 (0.46), 2.414 (0.85), 2.427 (1.41), 2.446 (1.90), 2.463 (2.38), 2.518 (5.54), 2.522 (3.54), 2.566 (0.79), 2.587 (4.00), 2.610 (5.00), 2.625 (0.85), 2.655 (4.65), 2.664 (1.30), 2.669 (1.53), 2.673 (1.52), 2.678 (3.02), 2.832 (0.80), 2.848 (1.46), 2.865 (1.12), 2.871 (1.50), 2.888 (0.66), 4.039 (0.54), 4.047 (1.71), 4.058 (1.99), 4.062 (2.58), 4.066 (2.67), 4.073 (2.43), 4.078 (2.40), 4.081 (2.03), 4.092 (1.71), 4.100 (0.55), 4.104 (0.52), 6.284 (16.00), 6.292 (0.76), 6.515 (8.08), 6.811 (1.54), 7.045 (1.51), 7.984 (4.58), 7.989 (4.66), 8.562 (4.20), 8.567 (4.16), 11.780 (1.32). | |
| | [α]²⁰_{D}: +52.1° (c = 1.00, methanol) | |
| **Example 943** | 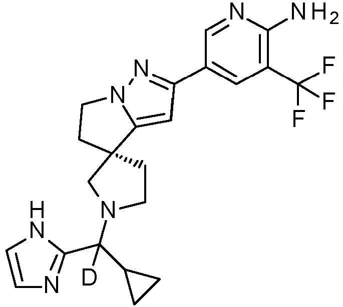 | (98 % purity, 49 % yield) |
| | | LC-MS Method 1): Rₜ = 0.92 min; MS (ESIpos): m/z = 445 [M+H]⁺ |
| | 5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: 0.074 (0.71), 0.086 (1.45), 0.097 (2.48), 0.110 (2.64), 0.120 (1.78), 0.310 (0.62), 0.334 (1.95), 0.350 (3.36), 0.366 (3.92), 0.378 (2.12), 0.393 (1.58), 0.405 (1.54), 0.417 (1.39), 0.429 (0.87), 0.620 (1.22), 0.651 (2.28), 0.666 (1.41), 1.168 (0.44), 1.187 (1.17), 1.194 (3.71), 1.204 (1.32), 1.210 (4.21), 1.240 (4.86), 1.246 (4.42), 1.253 (4.17), 1.907 (2.41), 1.926 (0.41), 1.945 (0.74), 1.958 (1.38), 1.978 (2.82), 1.996 (4.14), 2.002 (3.56), 2.016 (2.55), 2.035 (0.86), 2.055 (0.46), 2.318 (1.15), 2.323 (2.48), 2.327 (3.49), 2.331 (2.51), 2.337 (1.11), 2.452 (1.48), 2.470 (3.80), 2.518 (16.00), 2.523 (10.42), 2.558 (1.87), 2.572 (3.19), 2.593 (3.95), 2.610 (3.71), 2.625 (3.84), 2.641 (3.09), 2.659 (2.09), 2.665 (3.28), 2.669 (4.10), 2.673 (2.85), 2.784 (1.94), 2.805 (2.27), 2.968 (2.70), 2.992 (2.15), 3.166 (7.64), 4.026 (0.46), 4.046 (0.75), 4.053 (1.55), 4.072 (3.07), 4.091 (5.61), 4.108 (7.22), 4.125 (3.55), 6.437 (13.10), 6.446 (15.46), 6.528 (15.01), 6.973 (7.54), 6.984 (6.93), 7.999 (7.85), 8.136 (1.05), 8.583 (7.98), 8.587 (7.99). | |
| **Example 944** | 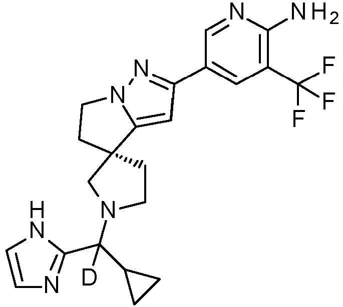 | (100 % purity, 22 % yield) |
| | | Rₜ = 1.19 min; |
| | | Instrument: Waters Acquity UPC2 QDA; Column: Chiralpak IG 3µ 100x4.6mm; eluent A: CO2; eluent B: methanol + 0.2 vol % aqueous ammonia (32%); isocratic: 30%B; gradient: no; flow: 4 mL/min; temperature: 40.0°C; BPR: 100 bar; UV: 280 nm |
| | 5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 1) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 30%B; gradient: no; flow: 100 mL/min; temperature: 40°C; BPR: 150 bar; UV: 271 nm. | |
| | ¹H-NMR (400 MHz, DMSO-d6) δ [ppm]: -0.065 (0.40), 0.109 (0.88), 0.122 (1.29), 0.134 (1.39), 0.145 (1.08), 0.157 (0.46), 0.353 (1.01), 0.365 (1.29), 0.376 (1.23), 0.387 (0.79), 0.398 (0.61), 0.411 (0.59), 0.422 (1.18), 0.434 (1.45), 0.445 (1.35), 0.457 (0.91), 0.639 (0.47), 0.649 (0.77), 0.660 (1.02), 0.672 (1.29), 0.681 (0.94), 1.191 (0.53), 1.208 (0.56), 1.218 (0.57), 1.231 (1.33), 1.239 (1.33), 1.251 (1.87), 1.263 (1.04), 1.271 (0.94), 1.283 (0.40), 1.906 (0.66), 1.994 (1.89), 2.009 (4.40), 2.028 (2.56), 2.318 (0.76), 2.322 (1.80), 2.326 (2.47), 2.332 (1.78), 2.336 (0.79), 2.461 (1.07), 2.518 (9.71), 2.522 (6.51), 2.561 (1.68), 2.566 (1.58), 2.580 (1.68), 2.593 (0.95), 2.597 (0.79), 2.613 (0.64), 2.660 (2.58), 2.664 (3.58), 2.669 (4.28), 2.673 (3.40), 2.678 (2.44), 2.684 (2.45), 2.811 (2.11), 2.834 (1.68), 2.973 (0.78), 2.989 (1.38), 3.010 (1.34), 3.028 (0.79), 4.030 (0.51), 4.046 (0.71), 4.050 (0.76), 4.057 (1.77), 4.073 (3.48), 4.093 (3.26), 4.108 (1.75), 4.115 (0.78), 4.120 (0.68), 4.135 (0.47), 6.442 (16.00), 6.525 (8.42), 7.060 (6.79), 7.993 (4.84), 7.998 (4.95), 8.133 (0.59), 8.580 (4.45), 8.584 (4.45). | |
| | [α]²⁰_{D}: +33.5° (c = 1.00, methanol) | |
| **Example 945** | 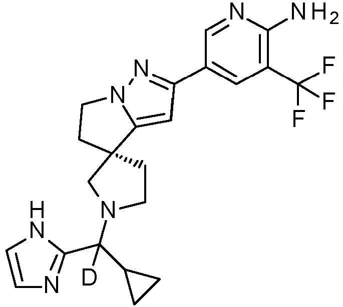 | (99 % purity, 22 % yield) |
| | | Rₜ = 2.45 min; |
| | | Instrument: Waters Acquity UPC2 QDA; Column: Chiralpak IG 3µ 100x4.6mm; eluent A: CO2; eluent B: methanol + 0.2 vol % aqueous ammonia (32%); isocratic: 30%B; gradient: no; flow: 4 mL/min; temperature: 40.0°C; BPR: 100 bar; UV: 280 nm |
| | 5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 2) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 30%B; gradient: no; flow: 100 mL/min; temperature: 40°C; BPR: 150 bar; UV: 271 nm. | |
| | ¹H-NMR (400 MHz, DMSO-d6) δ [ppm]: 0.056 (0.86), 0.069 (1.46), 0.083 (1.62), 0.089 (0.84), 0.095 (0.88), 0.301 (0.74), 0.310 (1.34), 0.321 (2.78), 0.332 (3.01), 0.342 (2.45), 0.354 (1.05), 0.602 (1.03), 0.612 (0.97), 0.617 (0.98), 0.624 (1.34), 0.629 (1.19), 0.632 (1.11), 0.644 (0.69), 1.202 (0.55), 1.215 (1.05), 1.221 (1.24), 1.227 (0.94), 1.235 (1.94), 1.241 (0.91), 1.247 (1.09), 1.254 (0.96), 1.266 (0.44), 1.927 (0.63), 1.940 (1.28), 1.956 (1.56), 1.974 (1.87), 1.988 (1.39), 1.993 (1.57), 2.008 (1.34), 2.020 (0.63), 2.025 (0.59), 2.040 (0.46), 2.442 (0.70), 2.460 (1.73), 2.477 (2.29), 2.518 (7.01), 2.522 (4.27), 2.566 (2.05), 2.571 (1.62), 2.583 (2.52), 2.599 (1.87), 2.609 (0.98), 2.614 (1.60), 2.632 (0.67), 2.753 (0.82), 2.774 (1.70), 2.790 (1.41), 2.796 (0.94), 2.812 (0.58), 2.931 (3.48), 2.954 (3.12), 4.085 (2.89), 4.102 (5.49), 4.119 (2.83), 6.440 (16.00), 6.521 (8.09), 6.753 (1.40), 7.017 (1.35), 7.999 (4.60), 8.004 (4.60), 8.583 (4.25), 8.587 (4.20), 11.784 (1.26). | |
| | [α]²⁰_{D}: +63.3° (c = 1.00, methanol) | |
| **Example 946** | 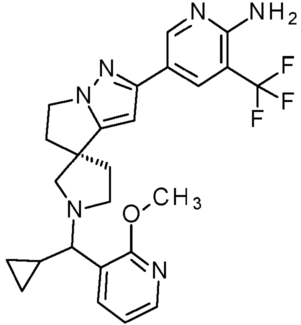 | (99 % purity, 10 % yield) |
| | | LC-MS (Method 1): Rₜ = 1.34 min; MS (ESlpos): m/z = 485 [M+H]⁺ |
| | 5-{(3R)-1-[cyclopropyl(2-methoxypyridin-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: 0.110 (0.89), 0.121 (1.15), 0.132 (1.17), 0.146 (0.85), 0.156 (0.52), 0.226 (1.08), 0.236 (1.11), 0.336 (0.51), 0.347 (0.63), 0.359 (0.63), 0.379 (0.76), 0.391 (0.89), 0.403 (0.80), 0.414 (0.55), 0.621 (1.13), 1.057 (1.07), 1.067 (1.00), 1.078 (1.02), 1.964 (0.66), 1.980 (0.74), 2.000 (1.25), 2.013 (2.31), 2.033 (1.84), 2.073 (6.05), 2.323 (1.90), 2.327 (2.52), 2.331 (1.92), 2.560 (1.56), 2.569 (2.09), 2.576 (2.24), 2.591 (3.02), 2.599 (2.88), 2.606 (2.57), 2.629 (1.47), 2.673 (3.85), 2.696 (1.39), 2.738 (0.69), 2.753 (0.61), 2.852 (1.26), 2.875 (1.51), 2.898 (2.97), 2.920 (2.48), 3.159 (1.24), 3.172 (1.24), 3.826 (16.00), 3.841 (11.69), 4.031 (0.46), 4.039 (0.82), 4.059 (1.61), 4.075 (1.74), 4.085 (1.66), 4.101 (2.46), 4.112 (0.89), 4.120 (1.21), 6.473 (6.38), 6.478 (4.88), 6.513 (5.26), 6.985 (1.00), 6.998 (1.09), 7.004 (1.10), 7.016 (2.28), 7.029 (1.51), 7.035 (1.48), 7.048 (1.42), 7.874 (1.09), 7.879 (1.24), 7.889 (1.74), 7.893 (2.58), 7.906 (1.49), 7.911 (1.47), 8.017 (4.14), 8.028 (1.71), 8.033 (1.50), 8.044 (2.61), 8.048 (2.09), 8.056 (1.85), 8.060 (1.61), 8.601 (3.83). | |
| **Example 947** | 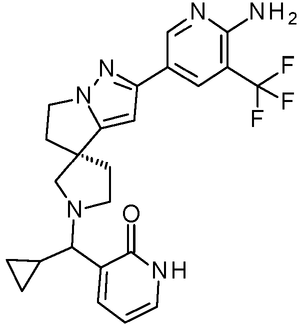 | (100 % purity, 50 % yield) |
| | | LC-MS (Method 1): Rₜ = 0.98 min; MS (ESIpos): m/z = 471 [M+H]⁺ |
| | 3-[{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}(cyclopropyl)methyl]pyridin-2(1H)-one (diastereomer 1 and diastereomer 2) | |
| | ¹H NMR (400 MHz, DMSO-d6, 22°C) δ ppm 0.17 - 0.38 (m, 3 H), 0.51 - 0.60 (m, 1 H), 0.91 - 1.02 (m, 1 H), 1.92 - 2.08 (m, 2 H), 2.54 - 2.76 (m, 4 H), 2.83 - 2.94 (m, 2 H), 4.04 - 4.14 (m, 2 H), 6.17 - 6.25 (m, 1 H), 6.47 and 6.48 (2s, 1 H), 6.51 and 6.52 (2s, 2 H), 7.20 - 7.26 (m, 1 H), 7.58 (2t, 1 H), 8.02 (s, 1 H), 8.60 (d, 1 H), 11.49 (br s, 1 H). | |
| **Example 948** | 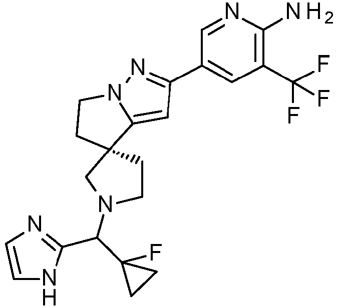 | (95 % purity, 33 % yield) |
| | | LC-MS (Method 1): Rₜ = 0.94 min; MS (ESlpos): m/z = 462 [M+H]⁺ |
| | 5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: 0.741 (2.51), 0.765 (4.93), 0.777 (2.77), 0.788 (3.21), 0.802 (1.62), 0.833 (1.38), 0.865 (1.56), 0.883 (1.23), 0.915 (1.42), 1.072 (1.40), 1.098 (1.34), 1.118 (1.52), 1.231 (1.78), 2.014 (4.32), 2.030 (4.86), 2.075 (0.75), 2.294 (0.99), 2.322 (1.88), 2.327 (2.62), 2.332 (1.93), 2.518 (14.39), 2.522 (9.81), 2.583 (1.38), 2.599 (2.64), 2.616 (2.88), 2.631 (2.60), 2.642 (1.31), 2.648 (1.45), 2.664 (2.58), 2.669 (3.16), 2.678 (4.47), 2.691 (3.54), 2.701 (5.50), 2.713 (4.47), 2.722 (3.41), 2.743 (2.40), 2.761 (0.73), 2.818 (3.32), 2.841 (3.00), 2.864 (1.70), 2.879 (1.45), 2.951 (3.64), 2.973 (3.45), 3.449 (3.60), 3.467 (3.34), 3.513 (3.67), 3.530 (3.25), 4.039 (0.48), 4.066 (1.61), 4.085 (3.40), 4.099 (5.87), 4.117 (6.43), 4.133 (3.11), 6.454 (0.78), 6.482 (13.72), 6.494 (15.14), 6.526 (16.00), 6.809 (5.85), 7.038 (3.15), 7.056 (2.76), 8.002 (8.30), 8.591 (8.82), 8.595 (8.41), 11.777 (4.24). | |
| **Example 949** | 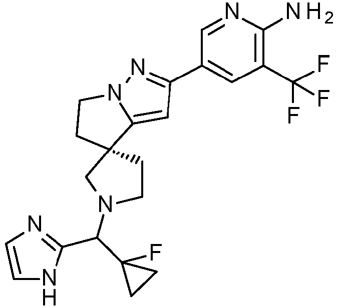 | (95 % purity, 31 % yield) |
| | | Rₜ = 0.81 min; |
| | | Instrument: Waters Acquity UPC2 QDA; Column: Chiral Art Amylose-SA 3µ 100x4.6mm; eluent A: CO2; eluent B: 2-propanol + 0.4 vol % diethylamine; isocratic: 30%B; gradient: no; flow: 4 mL/min; temperature: 40.0°C; BPR: 1800 psi; UV: 280 nm. |
| | 5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1) | |
| | Instrument: Sepiatec: Prep SFC100; Column: Chiral Art Amylose-SA 5µ 250x30mm; eluent A: CO2; eluent B: 2-propanol + 0.4 vol % diethylamine; isocratic: 30%B; gradient: no; flow: 100 mL/min; temperature: 40°C; BPR: 150 bar; UV: 271 nm | |
| | ¹H NMR (400 MHz, DMSO-*d*₆, 22°C) δ ppm 0.71 - 0.93 (m, 3 H), 1.06 - 1.17 (m, 1 H), 1.95 - 2.08 (m, 2 H), 2.59 - 2.77 (m, 3 H), 2.83 (d, 1 H), 2.93 - 3.01 (m, 1 H), 3.49 (d, 1 H), 4.03 - 4.15 (m, 2 H), 6.48 (s, 1 H), 6.53 (s, 2 H), 6.81 (s, 1 H), 7.06 (s, 1 H), 8.00 (d, 1 H), 8.59 (d, 1 H), 11.77 (br s, 1 H). | |
| | [α]²⁰_{D}: +29.8° (c = 1.00, methanol) | |
| **Example 950** | 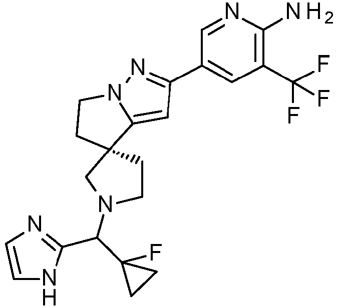 | (95 % purity, 32 % yield) |
| | | Rₜ = 1.40 min; |
| | | Instrument: Waters Acquity UPC2 QDA; Column: Chiral Art Amylose-SA 3µ 100x4.6mm; eluent A: CO2; eluent B: 2-propanol + 0.4 vol % diethylamine; isocratic: 30%B; gradient: no; flow: 4 mL/min; temperature: 40.0°C; BPR: 1800 psi; UV: 280 nm. |
| | 5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2) | |
| | Instrument: Sepiatec: Prep SFC100; Column: Chiral Art Amylose-SA 5µ 250x30mm; eluent A: CO2; eluent B: 2-propanol + 0.4 vol % diethylamine; isocratic: 30%B; gradient: no; flow: 100 mL/min; temperature: 40°C; BPR: 150 bar; UV: 271 nm | |
| | ¹H NMR (400 MHz, DMSO-*d*₆, 22°C) δ ppm 0.68 - 0.81 (m, 2 H), 0.83 - 0.95 (m, 1 H), 1.04 - 1.17 (m, 1 H), 1.96 - 2.10 (m, 2 H), 2.57 - 2.76 (m, 3 H), 2.83 - 2.91 (m, 1 H), 2.96 (d, 1 H), 3.47 (d, 1 H), 4.12 (t, 2 H), 6.49 (s, 1 H), 6.53 (s, 2 H), 6.81 (s, 1 H), 7.04 (d, 1 H), 8.00 (d, 1 H), 8.59 (d, 1 H), 11.76 (br s, 1 H). | |
| | [α]²⁰_{D}: +70.9° (c = 1.00, methanol) | |
| **Example 951** | 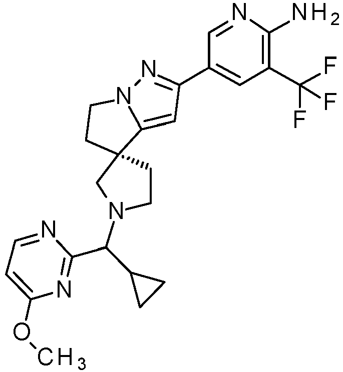 | (95 % purity, 3 % yield) |
| | | LC-MS (Method 1): Rₜ = 1.16 min; MS (ESlpos): m/z = 486 [M+H]⁺ |
| | 5-{(3R)-1-[cyclopropyl(4-methoxypyrimidin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: 0.090 (0.55), 0.102 (0.84), 0.114 (0.89), 0.125 (0.69), 0.309 (0.67), 0.321 (0.81), 0.331 (0.73), 0.342 (0.50), 0.407 (0.43), 0.418 (0.59), 0.431 (0.68), 0.442 (0.70), 0.455 (0.61), 0.468 (0.44), 0.625 (0.40), 0.636 (0.60), 0.647 (0.78), 0.658 (0.74), 0.670 (0.53), 1.230 (0.42), 1.316 (0.46), 1.328 (0.70), 1.339 (0.64), 1.349 (0.68), 1.359 (0.42), 1.945 (0.52), 1.959 (0.56), 1.979 (0.85), 1.986 (0.76), 1.998 (1.37), 2.004 (1.25), 2.018 (0.93), 2.322 (0.58), 2.326 (0.80), 2.332 (0.56), 2.465 (0.90), 2.518 (4.67), 2.522 (3.40), 2.558 (0.79), 2.574 (1.12), 2.591 (0.77), 2.606 (0.69), 2.635 (1.41), 2.647 (0.82), 2.658 (1.77), 2.664 (1.37), 2.669 (1.93), 2.673 (1.47), 2.689 (2.58), 2.694 (1.62), 2.705 (1.75), 2.712 (2.19), 2.717 (1.64), 2.729 (1.44), 2.793 (1.32), 2.815 (0.90), 2.917 (0.68), 2.933 (0.60), 3.022 (1.32), 3.045 (1.15), 3.128 (0.43), 3.912 (14.09), 3.915 (16.00), 4.052 (0.55), 4.071 (0.95), 4.078 (0.90), 4.086 (1.80), 4.104 (2.48), 4.121 (1.20), 6.384 (4.81), 6.406 (5.81), 6.516 (5.37), 6.805 (5.82), 6.819 (6.21), 7.984 (1.43), 7.989 (1.66), 7.994 (1.91), 7.999 (1.83), 8.461 (2.85), 8.469 (3.77), 8.475 (2.70), 8.484 (3.35), 8.564 (1.39), 8.570 (2.34), 8.576 (1.70). | |
| **Example 952** | 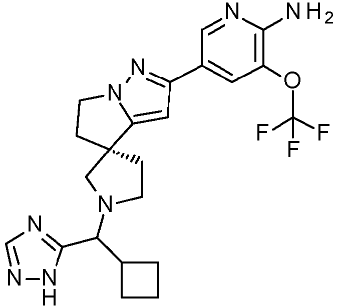 | (95 % purity, 48 % yield) |
| | | LC-MS (Method 1): Rₜ = 0.92 min; MS (ESIpos): m/z = 475 [M+H]⁺ |
| | 5-{(3R)-1-[cyclobutyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2) | |
| | ¹H-NMR (400 MHz, DMSO-d6) δ [ppm]: 1.233 (0.90), 1.474 (1.44), 1.496 (2.41), 1.519 (1.96), 1.541 (0.64), 1.693 (2.66), 1.702 (2.67), 1.730 (2.25), 1.753 (1.35), 1.781 (1.41), 1.803 (2.16), 1.824 (2.18), 1.885 (4.97), 1.906 (4.96), 1.927 (2.61), 2.119 (1.49), 2.322 (1.99), 2.326 (2.70), 2.332 (1.92), 2.411 (2.23), 2.518 (10.33), 2.523 (7.02), 2.591 (2.66), 2.611 (2.82), 2.637 (5.00), 2.660 (1.67), 2.664 (2.51), 2.669 (3.12), 2.673 (2.25), 2.761 (2.20), 2.884 (1.62), 3.748 (2.06), 3.773 (2.17), 4.045 (2.70), 4.061 (6.15), 4.073 (6.80), 4.090 (3.07), 6.213 (1.56), 6.249 (2.30), 6.470 (16.00), 7.719 (8.21), 7.723 (8.04), 7.874 (0.77), 8.297 (10.16), 8.301 (7.15), 8.430 (0.45), 13.785 (4.96). | |
| **Example 953** | 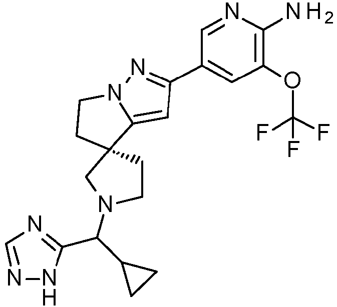 | (95 % purity, 33 % yield) |
| | | LC-MS (Method 1): Rₜ = 0.84 min; MS (ESIpos): m/z = 461 [M+H]⁺ |
| | 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2) | |
| | ¹H-NMR (400 MHz, DMSO-d6) δ [ppm]: 0.112 (1.51), 0.377 (2.07), 0.659 (1.42), 0.851 (0.51), 1.014 (0.47), 1.232 (2.78), 1.993 (3.16), 2.322 (2.21), 2.326 (3.05), 2.332 (2.20), 2.518 (16.00), 2.522 (10.20), 2.575 (3.14), 2.599 (3.19), 2.622 (2.93), 2.644 (3.26), 2.660 (3.06), 2.664 (3.66), 2.669 (4.25), 2.673 (3.42), 2.770 (2.53), 2.794 (2.22), 2.851 (1.88), 2.875 (2.73), 2.900 (1.69), 2.947 (1.63), 2.969 (1.33), 4.085 (4.37), 4.102 (5.14), 4.119 (2.35), 6.336 (1.04), 6.404 (3.18), 6.418 (2.83), 6.480 (12.07), 6.540 (1.10), 7.738 (8.05), 7.839 (3.64), 8.326 (6.91), 8.446 (0.97), 13.738 (0.78), 13.827 (2.67). | |
| **Example 954** | 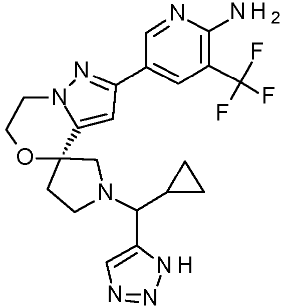 | (95 % purity, 44 % yield) |
| | | Rₜ = 2.70 min |
| | | Instrument: Agilent: 1260, Aurora SFC-Modul; Column: Chiralpak IG 3µ 100x4.6mm; eluent A: CO2; eluent B: 2-propanol + 0.4 vol % diethylamine; isocratic: 25%B; gradient: no; flow: 4 mL/min; temperature: 37.5°C; BPR: 100 bar; UV: 280 nm |
| | 5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1) | |
| | Instrument: Sepiatec: Prep SFC100; Column: Chiralpak IG 5µ 250x30mm; eluent A: CO2; eluent B: 2-propanol + 0.4 vol % diethylamine; isocratic: 25%B; gradient: no; flow: 100 mL/min; temperature: 40°C; BPR: 150 bar; UV: 280 nm | |
| | ¹H-NMR (400 MHz, DMSO-d6) δ [ppm]: 0.036 (1.02), 0.048 (1.10), 0.060 (0.83), 0.327 (0.79), 0.339 (1.01), 0.350 (1.02), 0.372 (0.75), 0.386 (0.86), 0.398 (1.02), 0.409 (0.98), 0.659 (0.90), 0.851 (0.32), 1.026 (16.00), 1.042 (15.72), 1.137 (0.95), 1.187 (0.92), 1.198 (0.90), 1.210 (0.94), 1.231 (1.21), 2.063 (0.33), 2.084 (2.38), 2.097 (0.97), 2.115 (1.80), 2.133 (0.77), 2.165 (1.12), 2.180 (1.02), 2.197 (0.56), 2.323 (0.92), 2.327 (1.32), 2.331 (0.95), 2.518 (7.72), 2.523 (5.01), 2.645 (0.54), 2.664 (2.13), 2.669 (2.11), 2.673 (1.40), 2.684 (1.47), 2.703 (0.58), 2.748 (2.45), 2.774 (2.65), 2.808 (3.20), 2.831 (3.42), 3.121 (2.53), 3.147 (2.19), 3.749 (0.43), 3.759 (0.43), 3.764 (0.58), 3.774 (0.55), 3.779 (0.44), 3.790 (0.41), 4.017 (3.21), 4.028 (3.57), 4.060 (3.26), 4.068 (2.94), 4.346 (1.70), 4.356 (1.66), 6.573 (6.28), 6.628 (8.61), 7.767 (0.39), 8.035 (3.81), 8.040 (3.86), 8.615 (3.57), 8.619 (3.53), 14.664 (0.10), 14.676 (0.10), 14.687 (0.11), 14.693 (0.11), 14.696 (0.11), 14.704 (0.11), 14.712 (0.12), 14.716 (0.12), 14.719 (0.11), 14.726 (0.11), 14.733 (0.12), 14.741 (0.11), 14.746 (0.11), 14.755 (0.11), 14.762 (0.10), 14.771 (0.10). | |
| | [α]²⁰_{D}: +78.8° (c = 1.00, methanol) | |
| **Example 955** | 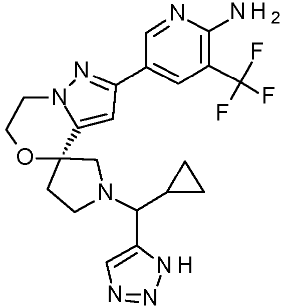 | (95 % purity, 26 % yield) |
| | | Rₜ = 3.61 min |
| | | Instrument: Agilent: 1260, Aurora SFC-Modul; Column: Chiralpak IG 3µ 100x4.6mm; eluent A: CO2; eluent B: 2-propanol + 0.4 vol % diethylamine; isocratic: 25%B; gradient: no; flow: 4 mL/min; temperature: 37.5°C; BPR: 100 bar; UV: 280 nm |
| | 5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2) | |
| | Instrument: Sepiatec: Prep SFC100; Column: Chiralpak IG 5µ 250x30mm; eluent A: CO2; eluent B: 2-propanol + 0.4 vol % diethylamine; isocratic: 25%B; gradient: no; flow: 100 mL/min; temperature: 40°C; BPR: 150 bar; UV: 280 nm | |
| | ¹H-NMR (400 MHz, DMSO-d6) δ [ppm]: 0.022 (1.19), 0.032 (1.28), 0.044 (1.00), 0.332 (0.93), 0.345 (1.18), 0.356 (1.17), 0.396 (1.03), 0.407 (1.23), 0.419 (1.16), 0.665 (1.07), 0.851 (0.44), 1.026 (16.00), 1.042 (15.74), 1.137 (1.60), 1.231 (2.02), 1.352 (0.32), 1.959 (0.32), 2.084 (1.15), 2.100 (1.17), 2.116 (2.03), 2.135 (0.80), 2.156 (0.77), 2.173 (1.45), 2.189 (1.36), 2.205 (0.75), 2.323 (1.08), 2.327 (1.52), 2.331 (1.11), 2.518 (8.75), 2.523 (5.63), 2.631 (0.65), 2.650 (1.33), 2.665 (2.29), 2.669 (2.84), 2.673 (1.99), 2.687 (0.73), 2.830 (3.13), 2.853 (2.92), 2.876 (0.63), 2.907 (6.43), 2.938 (1.60), 2.959 (1.26), 2.976 (0.57), 3.632 (0.30), 3.749 (0.47), 3.759 (0.45), 3.764 (0.61), 3.775 (0.58), 3.779 (0.46), 3.790 (0.45), 3.949 (0.57), 3.966 (1.08), 3.981 (2.48), 3.995 (3.39), 4.006 (1.94), 4.027 (1.24), 4.049 (3.19), 4.061 (3.82), 4.346 (1.83), 4.356 (1.83), 6.572 (7.24), 6.621 (5.87), 7.766 (0.46), 8.037 (4.36), 8.042 (4.41), 8.615 (4.08), 8.619 (4.04), 14.707 (0.13), 14.716 (0.13), 14.719 (0.13), 14.722 (0.13), 14.728 (0.13), 14.735 (0.13), 14.742 (0.13), 14.748 (0.14), 14.756 (0.13), 14.759 (0.13), 14.769 (0.13), 14.771 (0.13). | |
| | [α]²⁰_{D}: +66.8° (c = 1.00, methanol) | |
| **Example 956** | 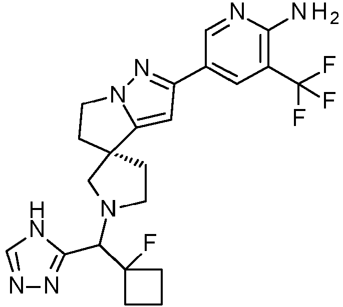 | (90 % purity, 1 % yield) |
| | | LC-MS (Method 8): Rₜ = 0.94 min; MS (ESlpos): m/z = 477 [M+H]⁺ |
| | 5-{(3R)-1-[(1-fluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, METHANOL-d4) δ [ppm]: 0.100 (0.64), 0.885 (0.87), 0.903 (1.81), 0.924 (1.23), 0.979 (0.79), 1.221 (1.25), 1.288 (5.86), 1.334 (1.94), 1.401 (0.75), 1.541 (0.81), 1.562 (1.55), 1.571 (1.50), 1.583 (1.82), 1.593 (1.68), 1.600 (1.40), 1.892 (2.04), 1.900 (1.62), 1.908 (1.60), 1.924 (1.47), 1.936 (1.92), 1.952 (1.35), 1.959 (1.18), 1.970 (1.81), 1.985 (2.03), 1.990 (1.92), 2.000 (2.15), 2.004 (2.15), 2.021 (2.57), 2.031 (2.32), 2.036 (4.97), 2.045 (2.36), 2.052 (2.32), 2.066 (2.86), 2.077 (1.28), 2.083 (2.03), 2.088 (1.80), 2.098 (1.71), 2.104 (1.60), 2.114 (1.53), 2.126 (2.20), 2.135 (2.12), 2.148 (3.37), 2.157 (6.66), 2.174 (2.58), 2.199 (2.14), 2.221 (1.06), 2.254 (0.44), 2.323 (0.84), 2.354 (1.37), 2.378 (1.58), 2.401 (1.24), 2.432 (0.96), 2.455 (0.51), 2.506 (1.04), 2.525 (2.22), 2.542 (2.93), 2.546 (2.45), 2.558 (4.42), 2.574 (3.97), 2.578 (3.15), 2.594 (2.33), 2.610 (1.63), 2.621 (2.08), 2.626 (2.17), 2.629 (1.90), 2.635 (2.45), 2.641 (2.55), 2.655 (2.57), 2.660 (7.57), 2.668 (1.69), 2.673 (1.40), 2.677 (1.06), 2.688 (1.29), 2.701 (0.53), 2.799 (0.42), 2.822 (0.40), 2.855 (0.63), 2.871 (2.09), 2.893 (7.00), 2.911 (10.09), 2.939 (2.14), 2.952 (2.36), 2.960 (2.91), 2.974 (2.63), 2.982 (1.41), 2.996 (1.00), 3.129 (0.59), 3.133 (0.85), 3.137 (0.66), 3.141 (0.47), 3.330 (0.94), 3.334 (0.69), 3.478 (0.50), 3.482 (0.88), 3.487 (0.50), 3.762 (0.42), 3.787 (0.45), 4.091 (0.54), 4.106 (0.81), 4.110 (0.75), 4.117 (2.75), 4.133 (4.91), 4.136 (4.33), 4.146 (5.61), 4.151 (6.47), 4.166 (3.88), 4.169 (2.41), 4.178 (1.02), 4.204 (3.13), 4.211 (2.57), 4.275 (3.03), 4.284 (2.40), 4.844 (0.68), 5.344 (0.48), 6.249 (16.00), 6.292 (13.22), 6.302 (0.89), 8.090 (6.09), 8.094 (8.44), 8.096 (7.44), 8.259 (1.32), 8.509 (7.89), 8.511 (7.17), 8.551 (1.29). | |
| **Example 957** | 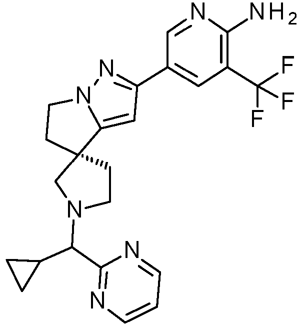 | (98 % purity, 9 % yield) |
| | | LC-MS (Method 1): Rₜ = 1.02 min; MS (ESIpos): m/z = 456 [M+H]⁺ |
| | 5-{(3R)-1-[cyclopropyl(pyrimidin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: 0.027 (0.55), 0.037 (0.67), 0.049 (0.65), 0.279 (0.56), 0.290 (0.71), 0.301 (0.62), 0.311 (0.45), 0.426 (0.46), 0.440 (0.44), 0.450 (0.44), 0.463 (0.40), 0.474 (0.44), 0.488 (0.41), 0.651 (0.44), 0.671 (0.58), 0.678 (0.57), 1.203 (0.51), 1.220 (0.51), 1.352 (0.56), 1.361 (0.55), 1.372 (0.47), 1.942 (0.46), 1.968 (0.70), 1.984 (1.46), 2.001 (1.09), 2.318 (0.85), 2.467 (1.37), 2.518 (16.00), 2.523 (10.19), 2.579 (1.32), 2.589 (1.38), 2.601 (2.54), 2.622 (1.98), 2.729 (1.38), 2.753 (1.69), 2.759 (1.74), 2.773 (0.59), 2.783 (1.33), 2.796 (1.29), 2.819 (0.91), 3.051 (0.42), 3.074 (0.45), 3.114 (0.99), 3.137 (0.91), 4.034 (0.45), 4.051 (1.03), 4.069 (0.93), 4.084 (0.57), 4.092 (1.17), 4.109 (2.01), 4.127 (0.98), 6.395 (4.34), 6.418 (4.63), 6.516 (4.15), 7.386 (1.36), 7.389 (1.39), 7.398 (2.76), 7.402 (2.88), 7.410 (1.43), 7.414 (1.53), 7.995 (1.25), 8.001 (2.08), 8.007 (1.46), 8.577 (2.26), 8.779 (5.22), 8.785 (5.89), 8.791 (5.51), 8.797 (5.17). | |
| **Example** 958 | 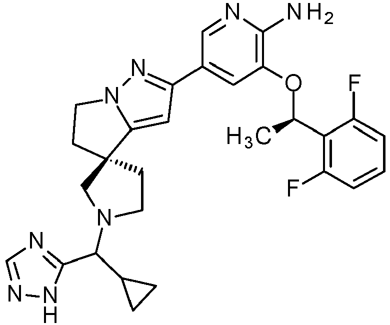 | (99 % purity, 33 % yield) |
| | | Rₜ = 2.91 min; Instrument: Agilent: 1260, Aurora SFC-Modul; Column: Chiralpak IG 3µ 100x4.6mm; eluent A: CO2; eluent B: methanol + 0.2 vol % aqueous ammonia (32%); isocratic: 40%B; gradient: no; flow: 4 ml/min; temperature: 37.5°C; BPR: 100 bar; UV: 280 nm |
| | | |
| | 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 1) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 40%B; gradient: no; flow: 100 mL/min; temperature: 40°C; BPR: 150 bar; UV: 280 nm | |
| | ¹H NMR (400 MHz, DMSO-d6, 22°C) δ ppm -0.01 - 0.15 (m, 1 H) 0.28 - 0.45 (m, 2 H) 0.56 - 0.71 (m, 1 H) 1.18 - 1.35 (m, 1 H) 1.75 (d, 3 H) 1.86 - 2.07 (m, 2 H) 2.56 - 2.66 (m, 3 H) 2.73 - 2.81 (m, 1 H) 2.86 (br d, 1 H) 2.93 - 3.04 (m, 1 H) 4.02 - 4.14 (m, 2 H) 5.64 (br s, 2 H) 5.82 (q, 1 H) 6.11 - 6.25 (m, 1 H) 7.05 - 7.15 (m, 2 H) 7.33 (s, 1 H) 7.35 - 7.44 (m, 1 H) 7.80 - 8.47 (m, 2 H) 13.69 - 13.89 (m, 1 H). | |
| | [α]²⁰_{D}: +224.2° (c = 1.00, methanol) | |
| | | |
| **Example 959** | 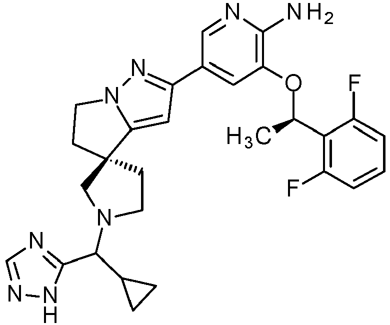 | (99 % purity, 33 % yield) |
| | | Rₜ = 4.72 min; |
| | | Instrument: Agilent: 1260, Aurora SFC-Modul; Column: Chiralpak IG 3µ 100x4.6mm; eluent A: CO2; eluent B: methanol + 0.2 vol % aqueous ammonia (32%); isocratic: 40%B; gradient: no; flow: 4 ml/min; temperature: 37.5°C; BPR: 100 bar; UV: 280 nm |
| | 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 2) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 40%B; gradient: no; flow: 100 mL/min; temperature: 40°C; BPR: 150 bar; UV: 280 nm | |
| | ¹H NMR (400 MHz, DMSO-d6, 22°C) δ ppm 0.01 - 0.15 (m, 1 H) 0.27 - 0.49 (m, 2 H) 0.57 - 0.72 (m, 1 H) 1.16 - 1.37 (m, 1 H) 1.75 (d, 3 H) 1.89 - 2.04 (m, 2 H) 2.62 (br d, 2 H) 2.76 (br d, 1 H) 2.84 - 3.04 (m, 2 H) 3.98 - 4.12 (m, 2 H) 5.64 (s, 2 H) 5.82 (q, 1 H) 6.10 - 6.28 (m, 1 H) 7.05 - 7.15 (m, 2 H) 7.32 (s, 1 H) 7.39 (tt, 1 H) 7.82 - 8.48 (m, 2 H) 13.64 - 13.89 (m, 1 H). | |
| | [α]²⁰_{D}: +221.7° (c = 1.00, methanol) | |
| **Example 960** | 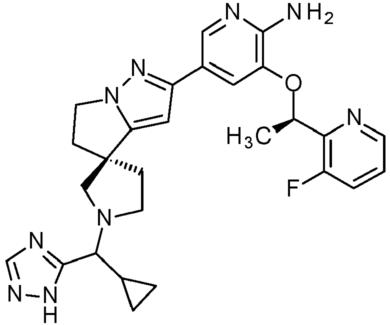 | (100 % purity, 41 % yield) |
| | | Rₜ = 3.93 min; Instrument: Agilent: 1260, Aurora SFC-Modul; Column: Chiralpak IG 3µ 100x4.6mm; eluent A: CO2; eluent B: methanol + 0.2 vol % aqueous ammonia (32%); isocratic: 40%B; gradient: no; flow: 4 mL/min; temperature: 37.5°C; BPR: 100 bar; UV: 280 nm |
| | | |
| | 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 1) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 40%B; gradient: no; flow: 100 mL/min; temperature: 40°C; BPR: 150 bar; UV: 280 nm | |
| | ¹H NMR (400 MHz, DMSO-d6, 22°C) δ ppm 0.01 - 0.14 (m, 1 H) 0.27 - 0.45 (m, 2 H) 0.54 - 0.70 (m, 1 H) 1.19 - 1.34 (m, 1 H) 1.69 (d, 3 H) 1.82 - 2.06 (m, 2 H) 2.55 - 2.66 (m, 3 H) 2.73 - 2.81 (m, 1 H) 2.86 (br d, 1 H) 2.82 - 2.89 (m, 1 H) 2.92 - 3.03 (m, 1 H) 4.03 - 4.11 (m, 2 H) 5.66 (br s, 2 H) 5.78 (q, 1 H) 6.13 - 6.26 (m, 1 H) 7.32 (d, Hz, 1 H) 7.45 (dt, 1 H) 7.74 (ddd, 1 H) 7.84 (s, 1 H) 7.89 (s, 1 H) 8.44 (dt, 1 H) 13.70 - 13.86 (m, 1 H). | |
| | [α]²⁰_{D}: +156.2° (c = 1.00, methanol) | |
| **Example 961** | 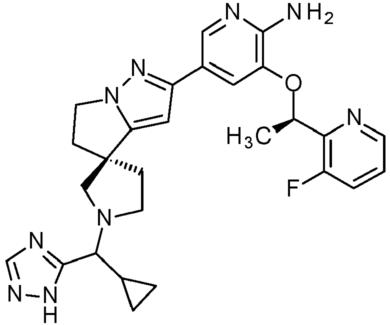 | (99 % purity, 33 % yield) |
| | | Rₜ = 7.54 min; Instrument: Agilent: 1260, Aurora SFC-Modul; Column: Chiralpak IG 3µ 100x4.6mm; eluent A: CO2; eluent B: methanol + 0.2 vol % aqueous ammonia (32%); isocratic: 40%B; gradient: no; flow: 4 mL/min; temperature: 37.5°C; BPR: 100 bar; UV: 280 nm |
| | | |
| | 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 2) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 40%B; gradient: no; flow: 100 mL/min; temperature: 40°C; BPR: 150 bar; UV: 280 nm | |
| | ¹H NMR (400 MHz, DMSO-*d*₆, 22°C) δ ppm 0.01 - 0.14 (m, 1 H) 0.29 - 0.48 (m, 2 H) 0.57 - 0.71 (m, 1 H) 1.16 - 1.37 (m, 1 H) 1.69 (d, 3 H) 1.89 - 2.05 (m, 2 H) 2.62 (br d, 2 H) 2.75 (br d, 1 H) 2.84 - 3.03 (m, 2 H) 3.97 - 4.10 (m, 2 H) 5.66 (s, 2 H) 5.77 (q, 1 H) 6.11 - 6.27 (m, 1 H) 7.32 (s, 1 H) 7.45 (dt, 1 H) 7.74 (ddd, 1 H) 7.84 (br s, 1 H) 7.89 (s, 1 H) 8.42 - 8.47 (m, 1 H) 13.70 - 13.87 (m, 1 H). | |
| | [α]²⁰_{D}: +198.2° (c = 1.00, methanol) | |
| **Example 962** | 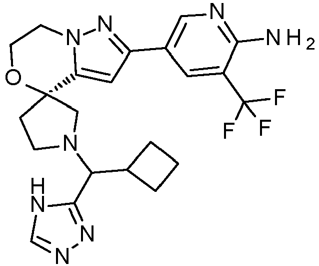 | 10.5 mg (93 % purity) |
| | | Rₜ = 3.95 min; |
| | | Instrument: Waters Acquity UPC2 QDA; Column: Chiral Art Amylose-SA 3µ 100x4.6mm; eluent A: CO2; eluent B: 2-propanol + 0.4 vol % diethylamine; isocratic: 20%B; gradient: no; flow: 4 mL/min; temperature: 40.0°C; BPR: 1800 psi; UV: 280 nm |
| | 5-{(3'R)-1'-[cyclobutyl(4H-1,2,4-triazol-3-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1) | |
| | Instrument: Sepiatec: Prep SFC100; Column: Chiral Art Amylose-SA 5µ 250x30mm; eluent A: CO2; eluent B: 2-propanol + 0.4 vol % diethylamine; isocratic: 20%B; gradient: no; flow: 100 mL/min; temperature: 40°C; BPR: 150 bar; UV: 267 nm | |
| | ¹H-NMR (400 MHz, DMSO-d6) δ [ppm]: 0.852 (0.69), 1.233 (1.93), 1.483 (0.90), 1.508 (1.33), 1.531 (1.21), 1.691 (1.31), 1.777 (0.81), 1.798 (1.27), 1.820 (1.06), 1.876 (1.10), 1.902 (1.38), 1.924 (1.06), 1.990 (1.19), 2.005 (1.20), 2.021 (1.43), 2.081 (1.33), 2.095 (1.58), 2.114 (1.50), 2.323 (2.87), 2.327 (4.11), 2.332 (2.90), 2.518 (16.00), 2.523 (11.75), 2.632 (1.30), 2.646 (1.34), 2.665 (3.49), 2.669 (4.45), 2.673 (2.94), 2.787 (1.66), 2.808 (1.42), 2.841 (1.35), 2.867 (2.99), 2.897 (4.75), 2.921 (2.68), 3.504 (1.51), 3.719 (2.85), 3.744 (2.65), 3.944 (3.43), 3.958 (2.99), 4.032 (3.22), 4.045 (4.21), 6.479 (5.15), 6.571 (7.14), 8.010 (4.32), 8.015 (4.37), 8.580 (3.89), 8.585 (3.88), 13.782 (0.30), 13.789 (0.34), 13.798 (0.38), 13.804 (0.40), 13.809 (0.40), 13.814 (0.39), 13.816 (0.40), 13.818 (0.37), 13.824 (0.34), 13.828 (0.30), 13.832 (0.29). | |
| **Example 963** | 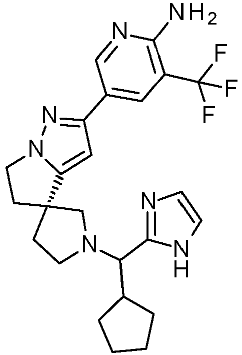 | (95 % purity, 8 % yield) |
| | | Rₜ = 1.22 min; |
| | | Instrument: Agilent: 1260, Aurora SFC-Modul; Column: Chiralpak IG 3µ 100x4.6mm; eluent A: CO2; eluent B: methanol + 0.2 vol % aqueous ammonia (32%); isocratic: 30%B; gradient: no; flow: 4 mL/min; temperature: 37.5°C; BPR: 100 bar; UV: 280 nm |
| | 5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 30%B; gradient: no; flow: 100 mL/min; temperature: 40°C; BPR: 150 bar; UV: 280 nm | |
| | ¹H-NMR (400 MHz, DMSO-d6) δ [ppm]: -0.073 (0.44), 0.798 (0.75), 0.814 (0.81), 0.821 (0.82), 0.840 (0.44), 0.904 (0.91), 0.922 (0.69), 0.956 (0.90), 0.970 (1.07), 0.990 (0.72), 1.137 (6.62), 1.232 (1.03), 1.369 (0.66), 1.387 (1.11), 1.404 (1.45), 1.423 (2.07), 1.433 (2.38), 1.454 (2.25), 1.466 (2.51), 1.486 (2.15), 1.504 (2.93), 1.522 (2.02), 1.541 (1.07), 1.561 (0.66), 1.780 (1.30), 1.799 (1.25), 1.815 (1.04), 1.832 (1.48), 1.846 (1.62), 1.851 (1.64), 1.857 (1.48), 1.865 (1.38), 1.877 (1.73), 1.894 (1.27), 1.907 (0.80), 1.925 (0.41), 2.084 (4.07), 2.115 (2.92), 2.318 (0.99), 2.323 (2.20), 2.327 (3.17), 2.332 (2.26), 2.337 (1.31), 2.356 (1.37), 2.371 (1.47), 2.388 (2.44), 2.406 (1.26), 2.425 (1.19), 2.441 (2.55), 2.458 (2.18), 2.518 (11.59), 2.523 (9.08), 2.635 (2.83), 2.658 (3.94), 2.665 (3.82), 2.669 (4.69), 2.673 (2.82), 2.679 (1.51), 2.685 (1.67), 2.705 (0.68), 2.790 (3.57), 2.813 (3.72), 2.830 (1.48), 2.852 (0.54), 3.485 (3.38), 3.511 (3.17), 4.055 (3.27), 4.073 (6.74), 4.090 (3.21), 4.562 (1.18), 6.221 (16.00), 6.510 (8.43), 6.977 (0.53), 7.975 (4.79), 7.980 (4.84), 8.549 (4.45), 8.553 (4.30), 11.786 (0.56). | |
| | [α]²⁰_{D}: +51.5° (c = 1.00, methanol) | |
| **Example 964** | 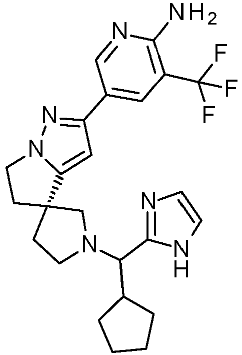 | (95 % purity, 6 % yield) |
| | | Rₜ = 2.40 min; |
| | | Instrument: Agilent: 1260, Aurora SFC-Modul; Column: Chiralpak IG 3µ 100x4.6mm; eluent A: CO2; eluent B: methanol + 0.2 vol % aqueous ammonia (32%); isocratic: 30%B; gradient: no; flow: 4 mL/min; temperature: 37.5°C; BPR: 100 bar; UV: 280 nm |
| | 5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 30%B; gradient: no; flow: 100 mL/min; temperature: 40°C; BPR: 150 bar; UV: 280 nm | |
| | ¹H-NMR (400 MHz, DMSO-d6) δ [ppm]: 0.798 (1.26), 0.803 (0.58), 0.815 (1.39), 0.822 (1.44), 0.840 (0.66), 0.886 (0.77), 0.905 (1.56), 0.922 (0.97), 0.932 (0.66), 0.949 (0.88), 0.962 (0.98), 0.980 (0.80), 1.071 (0.51), 1.137 (1.32), 1.159 (0.42), 1.232 (0.70), 1.352 (0.75), 1.368 (1.04), 1.385 (1.03), 1.398 (0.91), 1.420 (1.20), 1.441 (1.70), 1.456 (1.91), 1.471 (1.83), 1.478 (1.68), 1.505 (3.03), 1.520 (2.87), 1.820 (0.56), 1.847 (2.95), 1.865 (4.64), 1.882 (2.54), 2.116 (0.52), 2.318 (0.79), 2.323 (1.73), 2.327 (2.50), 2.332 (1.78), 2.336 (0.76), 2.363 (0.47), 2.381 (0.98), 2.394 (1.48), 2.413 (2.35), 2.432 (1.87), 2.449 (2.22), 2.466 (2.52), 2.518 (9.69), 2.523 (7.59), 2.601 (0.61), 2.620 (1.56), 2.639 (3.94), 2.661 (4.59), 2.669 (3.03), 2.673 (1.95), 2.678 (0.84), 2.732 (3.55), 2.755 (2.36), 2.833 (0.70), 2.850 (1.61), 2.866 (1.02), 2.872 (1.25), 2.888 (0.56), 3.485 (3.53), 3.511 (3.28), 4.059 (2.44), 4.076 (5.13), 4.094 (2.53), 6.148 (16.00), 6.512 (7.62), 6.540 (0.44), 6.563 (0.53), 6.852 (0.49), 7.030 (0.50), 7.970 (4.34), 7.976 (4.39), 8.544 (3.99), 8.548 (3.89), 11.762 (1.02). | |
| | [α]²⁰_{D}: +42.4° (c = 1.00, methanol) | |
| **Example 965** | 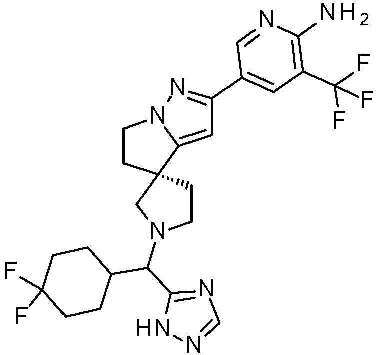 | (95 % purity, 39 % yield) |
| | | LC-MS (Method 1): Rₜ = 1.01 min; MS (ESIpos): m/z = 524 [M+H]⁺ |
| | 5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: -0.052 (0.89), 0.852 (0.47), 1.044 (0.47), 1.232 (1.71), 1.352 (0.61), 1.787 (0.49), 1.898 (1.64), 1.987 (0.51), 2.006 (0.74), 2.024 (0.78), 2.084 (1.04), 2.104 (1.01), 2.289 (0.68), 2.318 (0.52), 2.322 (1.16), 2.327 (1.67), 2.332 (1.23), 2.336 (0.55), 2.440 (0.77), 2.518 (6.33), 2.523 (4.54), 2.629 (0.70), 2.660 (1.08), 2.665 (1.72), 2.669 (2.25), 2.673 (1.80), 2.678 (1.05), 2.701 (1.29), 2.839 (1.52), 2.861 (1.46), 3.159 (16.00), 3.172 (14.20), 3.642 (0.55), 3.700 (0.44), 4.069 (1.43), 4.082 (3.39), 4.095 (4.93), 4.108 (4.60), 4.121 (1.32), 6.207 (0.43), 6.259 (0.52), 6.281 (0.47), 6.299 (0.46), 6.452 (0.42), 6.514 (4.46), 7.939 (0.60), 7.983 (3.34), 7.988 (3.43), 8.513 (0.62), 8.559 (2.68), 13.849 (1.39). | |
| **Example 966** | 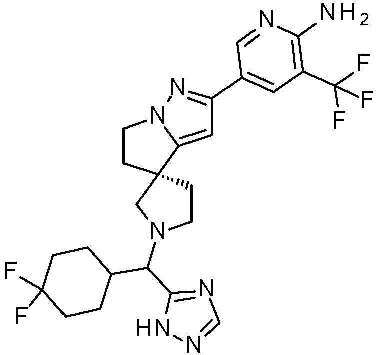 | (90 % purity, 35 % yield) |
| | | Rₜ = 2.14 min; |
| | | Instrument: Agilent: 1260, Aurora SFC-Modul; Column: Chiralpak IG 3µ 100x4.6mm; eluent A: CO2; eluent B: methanol + 0.2 vol % aqueous ammonia (32%); isocratic: 20%B; gradient: no; flow: 4 mL/min; temperature: 37.5°C; BPR: 100 bar; UV: 280 nm |
| | 5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1) | |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 20%B; gradient: no; flow: 100 mL/min; temperature: 40°C; BPR: 150 bar; UV: 280 nm | |
| | ¹H-NMR (400 MHz, DMSO-d6) δ [ppm]: 1.137 (0.53), 1.232 (0.75), 1.358 (0.64), 1.786 (0.53), 1.892 (1.16), 1.906 (1.15), 2.005 (0.50), 2.084 (16.00), 2.101 (0.84), 2.115 (0.73), 2.322 (0.86), 2.327 (1.21), 2.332 (0.91), 2.336 (0.44), 2.387 (0.46), 2.406 (0.45), 2.422 (0.54), 2.436 (0.56), 2.451 (0.56), 2.518 (4.38), 2.523 (3.16), 2.604 (0.50), 2.630 (0.55), 2.651 (0.76), 2.660 (0.88), 2.664 (1.36), 2.669 (1.71), 2.673 (1.28), 2.678 (0.78), 2.839 (2.40), 2.861 (2.04), 3.628 (0.43), 3.651 (0.42), 3.699 (0.44), 4.079 (1.48), 4.091 (1.49), 6.277 (1.04), 6.305 (1.10), 6.513 (3.08), 7.943 (0.87), 7.982 (2.88), 7.987 (2.88), 8.518 (0.95), 8.556 (2.19), 13.841 (0.91), 13.853 (0.89). | |
| **Example 967** | 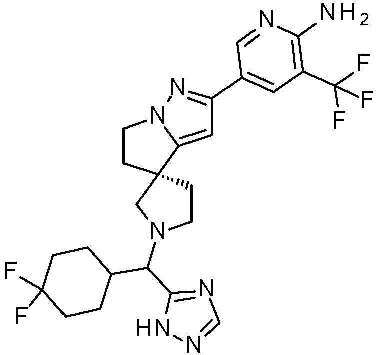 | (90 % purity, 34 % yield) |
| | | Rₜ = 3.56 min; |
| | | Instrument: Agilent: 1260, Aurora SFC-Modul; Column: Chiralpak IG 3µ |
| | 5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2) | |
| | | 100x4.6mm; eluent A: CO2; eluent B: methanol + 0.2 vol % aqueous ammonia (32%); isocratic: 20%B; gradient: no; flow: 4 mL/min; temperature: 37.5°C; BPR: 100 bar; UV: 280 nm |
| | Instrument: Sepiatec: Prep SFC100; Column: Chiralpak IG 5µ 250x30mm; eluent A: CO2; eluent B: methanol + 0.2 vol % aqueous ammonia (32%); isocratic: 20%B; gradient: no; flow: 100 mL/min; temperature: 40°C; BPR: 150 bar; UV: 280 nm | |
| | ¹H-NMR (400 MHz, DMSO-d6) δ [ppm]: 1.028 (1.62), 1.043 (2.00), 1.232 (2.82), 1.352 (2.35), 1.665 (1.04), 1.780 (1.36), 1.898 (6.37), 2.055 (2.97), 2.084 (11.83), 2.109 (3.53), 2.289 (2.38), 2.318 (1.25), 2.322 (2.71), 2.327 (3.89), 2.332 (2.82), 2.336 (1.19), 2.439 (3.32), 2.518 (15.73), 2.523 (11.23), 2.629 (2.55), 2.660 (2.88), 2.665 (4.24), 2.669 (5.66), 2.673 (4.62), 2.678 (2.84), 2.702 (7.42), 2.877 (2.55), 3.616 (1.24), 3.641 (1.40), 3.700 (1.52), 4.068 (5.83), 4.085 (11.47), 4.102 (5.79), 6.209 (2.55), 6.257 (2.77), 6.452 (1.50), 6.515 (16.00), 7.935 (2.34), 7.984 (13.59), 7.989 (13.67), 8.014 (0.48), 8.511 (2.20), 8.558 (9.43), 8.594 (0.48), 13.853 (5.72). | |
| **Example** | 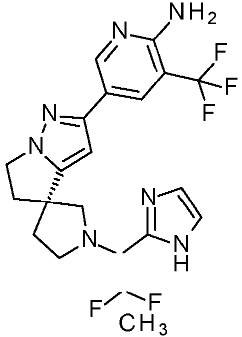 | (99 % purity, 1 % yield) |
| **968** | | LC-MS (Method 1): Rₜ = 0.96 min; MS (ESlpos): m/z = 468 [M+H]⁺ |
| | 5-{(3R)-1-[2,2-difluoro-1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2) | |
| | ¹H-NMR (400 MHz, DMSO-d6) δ [ppm]: 1.232 (0.78), 1.352 (0.65), 1.752 (5.79), 1.800 (11.16), 1.848 (5.38), 1.878 (0.62), 1.895 (1.50), 1.912 (4.58), 1.929 (7.11), 1.947 (3.54), 1.961 (0.76), 2.318 (1.23), 2.337 (1.18), 2.392 (0.44), 2.406 (1.63), 2.416 (1.83), 2.424 (3.70), 2.433 (3.39), 2.442 (2.74), 2.450 (2.35), 2.455 (2.02), 2.518 (15.86), 2.523 (10.67), 2.567 (0.65), 2.678 (1.27), 2.714 (3.15), 2.723 (2.46), 2.737 (4.78), 2.745 (6.89), 2.764 (6.58), 2.787 (2.23), 2.862 (0.83), 2.882 (1.65), 2.905 (4.32), 2.920 (2.27), 2.928 (3.14), 2.943 (1.40), 2.959 (0.54), 4.074 (3.79), 4.089 (7.00), 4.108 (3.62), 4.141 (1.62), 4.160 (1.69), 4.168 (1.80), 4.174 (2.01), 4.186 (1.60), 4.193 (1.87), 4.201 (1.59), 4.220 (1.22), 6.244 (13.87), 6.344 (16.00), 6.524 (13.97), 6.575 (0.67), 6.907 (3.68), 6.910 (6.86), 6.913 (7.32), 6.916 (7.41), 6.919 (4.08), 7.111 (3.60), 7.118 (6.81), 7.125 (3.72), 7.978 (4.18), 7.983 (4.68), 7.987 (4.96), 7.993 (4.58), 8.555 (3.90), 8.561 (4.04), 8.566 (4.42), 8.571 (4.17), 11.969 (2.16), 11.991 (2.23). | |
| **Example 969** | 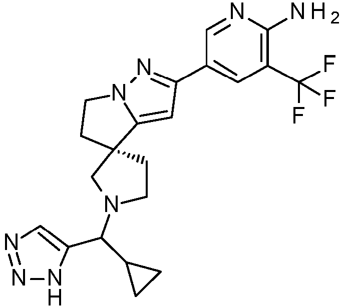 | (95 % purity, 31 % yield) |
| | | Rₜ = 2.49 min; |
| | | Instrument: Thermo Fisher UltiMate 3000; Column: YMC Amylose SA 3µ, 100x4.6; eluent A: hexane + 0.1 vol % diethylamine; eluent B: ethanol; isocratic: 70%A+30%B; flow: 1.4 mL/min; temperature: 25°C; UV: 280 nm; |
| | 5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1) | |
| | Instrument: PrepCon Labomatic HPLC-3; Column: YMC Amylose SA 5µ, 250x50; eluent A: hexane + 0.1 vol % diethylamine; eluent B: ethanol; isocratic: 70%A+30%B; flow: 60 mL/min; temperature: 25°C; UV: 280 nm; | |
| | ¹H NMR (DMSO-d6, 400 MHz): δ (ppm) 8.59 (d, 1H), 8.01 (d, 1H), 7.77 (br s, 1H), 6.51 (s, 2H), 6.42 (s, 1H), 4.02 - 4.14 (m, 2H), 2.90 - 2.98 (m, 1H), 2.85 (d, 1H), 2.53 - 2.73 (m, 4H), 1.93 - 2.04 (m, 2H), 1.14 - 1.25 (m, 1H), 0.61 - 0.69 (m, 1H), 0.29 - 0.45 (m, 2H), 0.04 (dq, 1H). | |
| | [α]²⁰_{D}: +43.7° (c = 1.00, methanol) | |
| **Example 970** | 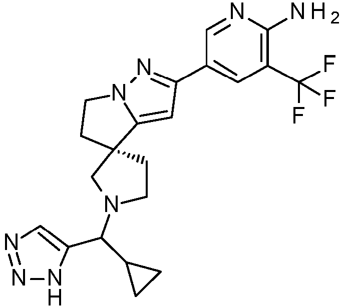 | (95 % purity, 37 % yield) |
| | | Rₜ = 3.69 min; |
| | | Instrument: Thermo Fisher UltiMate 3000; Column: YMC Amylose SA 3µ, 100x4.6; eluent A: hexane + 0.1 vol % diethylamine; eluent B: ethanol; isocratic: 70%A+30%B; flow: 1.4 mL/min; temperature: 25°C; UV: 280 nm; |
| | 5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2) | |
| | Instrument: PrepCon Labomatic HPLC-3; Column: YMC Amylose SA 5µ, 250x50; eluent A: hexane + 0.1 vol % diethylamine; eluent B: ethanol; isocratic: 70%A+30%B; flow: 60 mL/min; temperature: 25°C; UV: 280 nm; | |
| | ¹H NMR (DMSO-d6, 400 MHz): δ (ppm) 8.59 (d, 1H), 8.01 (d, 1H), 7.77 (br s, 1H), 6.51 (s, 2H), 6.42 (s, 1H), 4.02 - 4.14 (m, 2H), 2.90 - 2.98 (m, 1H), 2.85 (d, 1H), 2.53 - 2.73 (m, 4H), 1.93 - 2.04 (m, 2H), 1.14 - 1.25 (m, 1H), 0.61 - 0.69 (m, 1H), 0.29 - 0.45 (m, 2H), 0.04 (dq, 1H) | |
| | [α]²⁰_{D}: +43.7° (c = 1.00, methanol) | |
| **Example 971** | 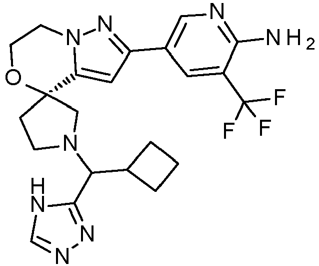 | (92 % purity, 24 % yield) |
| | | LC-MS (Method 1): Rₜ = 0.89 min; MS (ESIpos): m/z = 475 [M+H]⁺ |
| | 5-{(3'R)-1'-[cyclobutyl(4H-1,2,4-triazol-3-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2) | |
| | ¹H-NMR (400 MHz, DMSO-d6) δ [ppm]: 1.154 (4.01), 1.171 (7.72), 1.190 (3.90), 1.231 (0.52), 1.502 (1.08), 1.688 (1.28), 1.798 (0.90), 1.879 (0.99), 1.902 (1.33), 1.924 (0.83), 1.987 (16.00), 2.084 (1.47), 2.327 (1.17), 2.518 (5.03), 2.523 (3.46), 2.629 (1.06), 2.665 (1.13), 2.669 (1.38), 2.807 (1.37), 2.818 (1.27), 2.898 (2.21), 3.003 (0.61), 3.734 (1.01), 3.959 (3.24), 3.999 (1.33), 4.017 (3.74), 4.034 (6.13), 4.052 (4.04), 5.758 (5.87), 6.513 (0.98), 6.570 (6.43), 7.867 (0.65), 8.011 (4.80), 8.444 (0.35), 8.456 (0.39), 8.581 (3.31), 13.825 (0.95). | |
| **Example 972** | 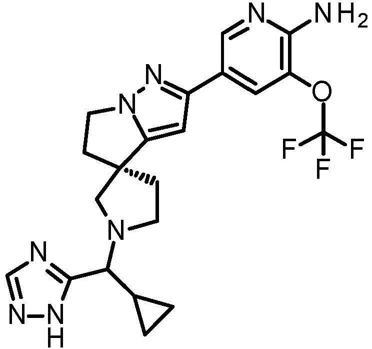 | (95 % purity, 44 % yield) |
| | | Rₜ = 3.16 min; Instrument: Thermo Fisher UltiMate 3000; Column: YMC Amylose SA 3µ, 100x4.6; eluent A: hexane + 0.1 vol % diethylamine; eluent B: ethanol; isocratic: 80%A+20%B; flow: 1.4 mL/min; temperature: 25°C; UV: 280 nm; |
| | | |
| | 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1) | |
| | Instrument: PrepCon Labomatic HPLC-3; Column: YMC Amylose SA 5µ, 250x30; eluent A: hexane + 0.1 vol % diethylamine; eluent B: ethanol; isocratic: 80%A+20%B; flow: 60 mL/min; temperature: 25°C; UV: 280 nm; | |
| | ¹H NMR (400 MHz, DMSO-*d*₆, 22°C) δ ppm 0.04 - 0.13 (m, 1 H) 0.29 - 0.43 (m, 2 H) 0.59 - 0.68 (m, 1 H) 1.21 - 1.32 (m, 1 H) 1.89 - 2.05 (m, 2 H) 2.54 - 2.68 (m, 3 H) 2.74 - 2.82 (m, 1 H) 2.85 (d, 1 H) 2.98 (br d, 1 H) 4.10 (t, 2 H) 6.38 (s, 1 H) 6.48 (s, 2 H) 7.71 - 7.76 (m, 1 H) 7.79 - 7.99 (m, 1 H) 8.33 (d, 1 H) 13.79 (br s, 1 H). | |
| | [α]²⁰_{D}: +61.6° (c = 1.00, methanol) | |
| Example 973 | 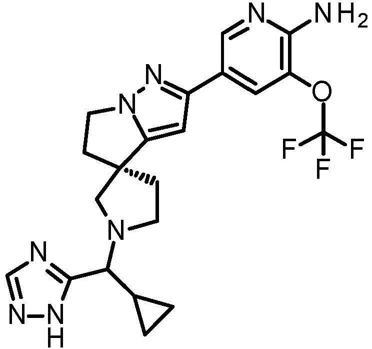 | (95 % purity, 46 % yield) |
| | | Rₜ = 4.32 min; |
| | | Instrument: Thermo Fisher UltiMate 3000; Column: YMC Amylose SA 3µ, 100x4.6; eluent A: hexane + 0.1 vol % diethylamine; eluent B: ethanol; isocratic: 80%A+20%B; flow: 1.4 mL/min; temperature: 25°C; UV: 280 nm; |
| | 5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 2) | |
| | Instrument: PrepCon Labomatic HPLC-3; Column: YMC Amylose SA 5µ, 250x30; eluent A: hexane + 0.1 vol % diethylamine; eluent B: ethanol; isocratic: 80%A+20%B; flow: 60 mL/min; temperature: 25°C; UV: 280 nm; | |
| | ¹H NMR (400 MHz, DMSO-d6, 22°C) δ ppm 0.01 - 0.15 (m, 1 H) 0.28 - 0.48 (m, 2 H) 0.56 - 0.72 (m, 1 H) 1.19 - 1.29 (m, 1 H) 1.93 - 2.04 (m, 2 H) 2.54 - 2.68 (m, 3 H) 2.78 (br d, 1 H) 2.84 - 3.06 (m, 2 H) 4.00 - 4.14 (m, 2 H) 6.30 - 6.43 (m, 1 H) 6.48 (br s, 2 H) 7.74 (t, 1 H) 7.84 (s, 1 H, tautomer 1) 8.33 (br s, 1 H) 8.45 (br s, 1 H tautomer 2) 13.70 - 13.87 (m, 1 H). | |
| | [α]²⁰_{D}: +41.6° (c = 1.00, methanol) | |

### EXPERIMENTAL SECTION - BIOLOGICAL ASSAYS

### Biological in vitro assays

The *in vitro* activity of the compounds of the present invention can be demonstrated in the following assays:
The example testing experiments described herein serve to illustrate the present invention and the invention is not limited to the examples given.

### Biological Evaluation

In order that this invention may be better understood, the following examples are set forth. These examples are for the purpose of illustration only, and are not to be construed as limiting the scope of the invention in any manner. All publications mentioned herein are incorporated by reference in their entirety.

Demonstration of the activity of the compounds of the present invention may be accomplished through in vitro and in vivo assays that are well known in the art. For example, to demonstrate the efficacy of a pharmaceutical agent to inhibit and be selective against something the following assays may be used.

### Binding competition assay

The ability of the compounds of the present invention to inhibit the binding of an Alexa647-labelled ATP-competitive kinase inhibitor to a Glutathione-S-transferase- (GST-) fusion protein was quantified employing the TR-FRET-based binding competition assay as described in the following paragraphs. A recombinant fusion protein of N-terminal GST and full-length human , expressed by baculovirus infected SF9 insect cells and purified by Glutathione Sepharose affinity chromatography, was used as GST- fusion protein. Tracer 222 from Invitrogen (catalogue no. PR9198A) was used as Alexa647-labelled ATP-competitive kinase inhibitor.

For the assay 50 nl of a 100fold concentrated solution of the test compound in DMSO was pipetted into either a black low volume 384well microtiter plate or a black 1536well microtiter plate (both Greiner Bio-One, Frickenhausen, Germany), 3 µL solution of Tracer 222 (25 nM => final concentration in 5 µL assay volume is 15 nM) in aqueous assay buffer [25 mM Tris/HCl pH 7.5, 10 mM MgCl₂, 5 mM β-glycerolphosphate, 2.5 mM dithiothreitol, 0.5 mM ethylene glycol-bis(2-aminoethylether)-*N,N,N',N'-*tetraacetic acid [EGTA], 0.5 mM sodium ortho-vanadate, 0.01 % (w/v) bovine serum albumin [BSA], 0.005% (w/v) Pluronic F-127 (Sigma)] were added. Then the binding competition was started by the addition of 2 µL of a solution of the GST- fusion protein (2.5 nM => final conc. in the 5 µL assay volume is 1 nM) and of Anti-GST-Tb (1.25 nM => final conc. in the 5 µL assay volume is 0.5 nM), a Lumi4^{®}-Tb Cryptate-conjugated anti-GST-antibody from Cisbio Bioassays (France), in assay buffer. The resulting mixture was incubated 30 min at 22°C to allow the formation of a complex between the Tracer 222, the fusion protein and Anti-GST-Tb. Subsequently the amount of this complex was evaluated by measurement of the resonance energy transfer from the Tb-cryptate to the Tracer 222.

Therefore, the fluorescence emissions at 620 nm and 665 nm after excitation at 350 nm were measured in a TR-FRET reader, e.g. a Pherastar (BMG Labtechnologies, Offenburg, Germany) or a Viewlux (Perkin-Elmer). The ratio of the emissions at 665 nm and at 622 nm was taken as the measure for the amount of the complex. The data were normalised (assay reaction without inhibitor = 0 % inhibition, all other assay components but GST- fusion protein = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC50 values were calculated using Genedata Screener^{™} software.

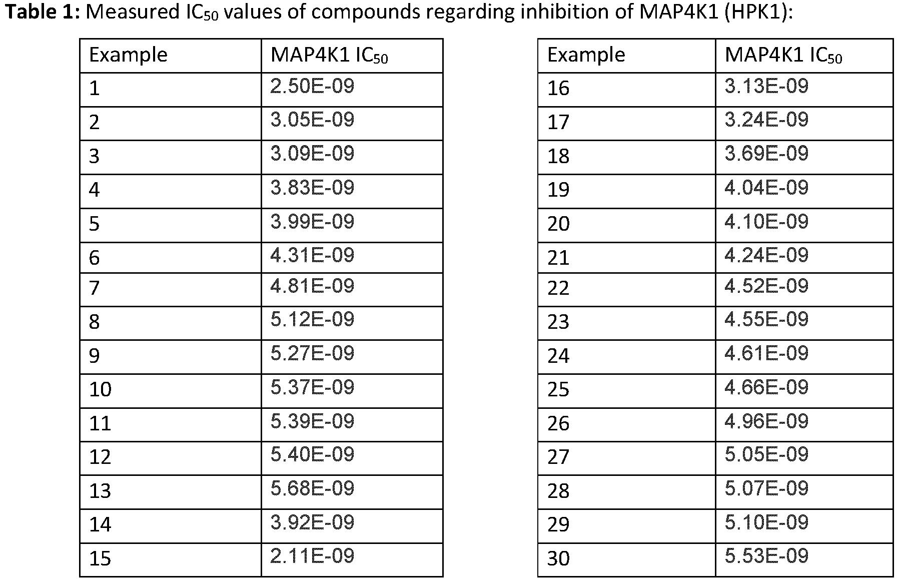

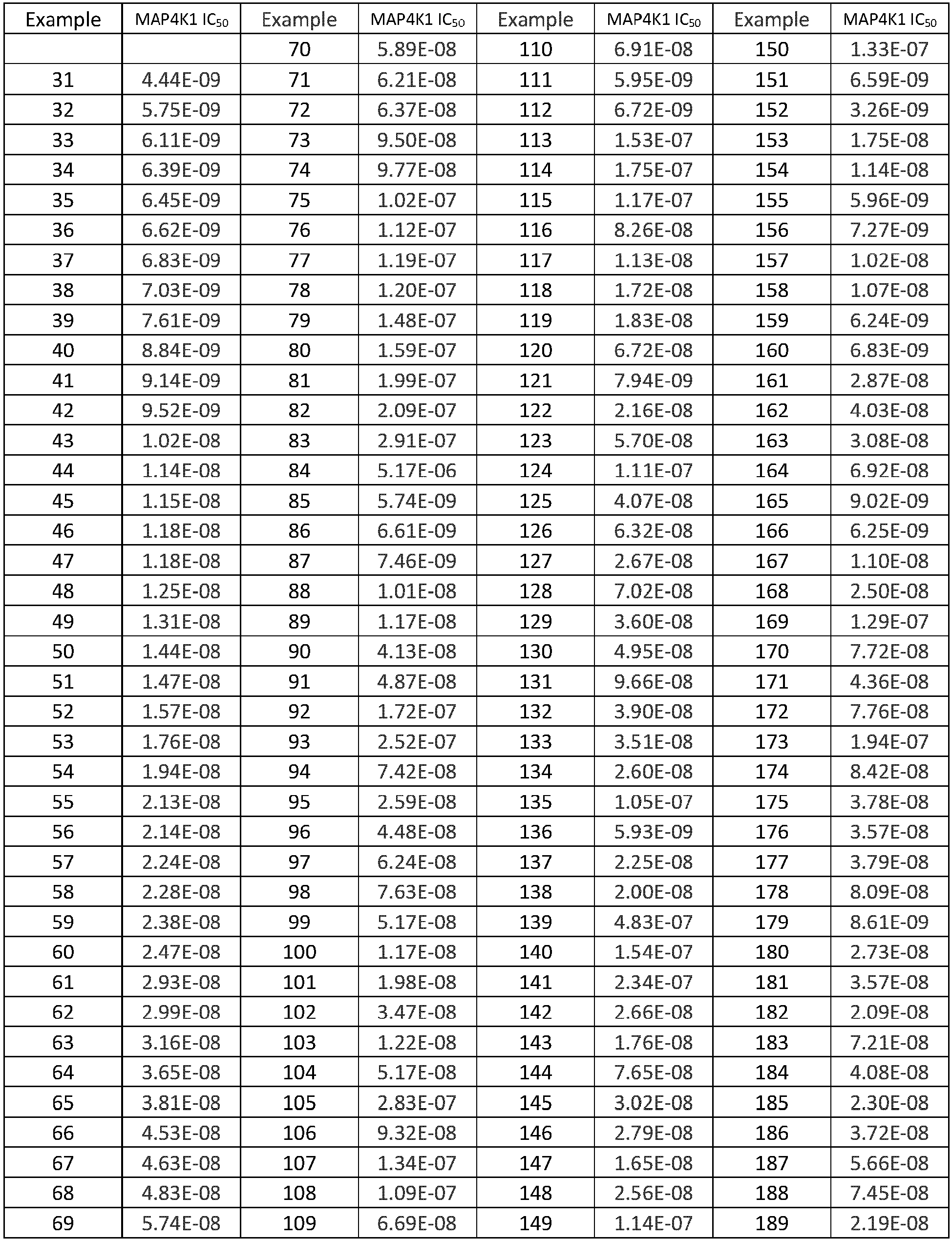

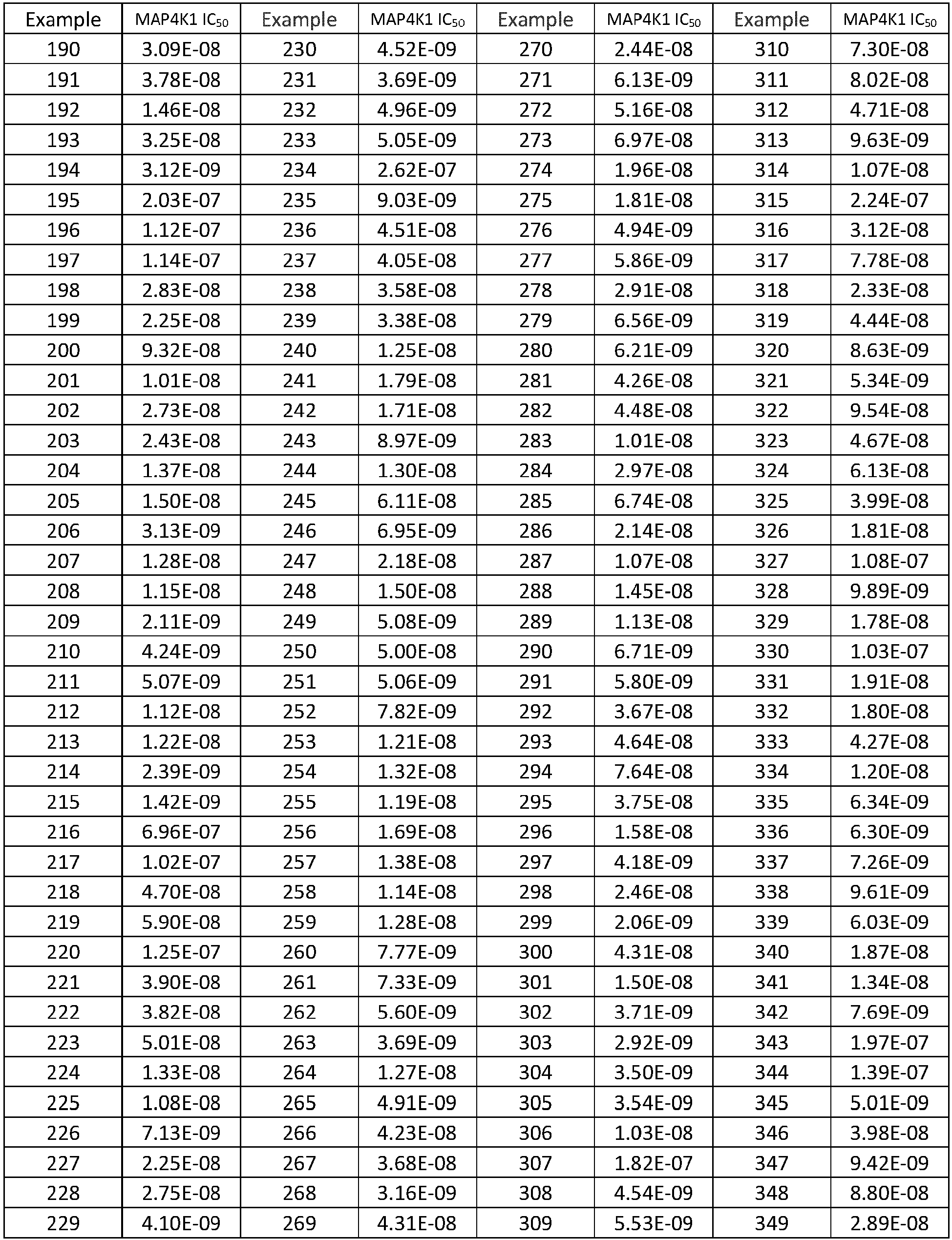

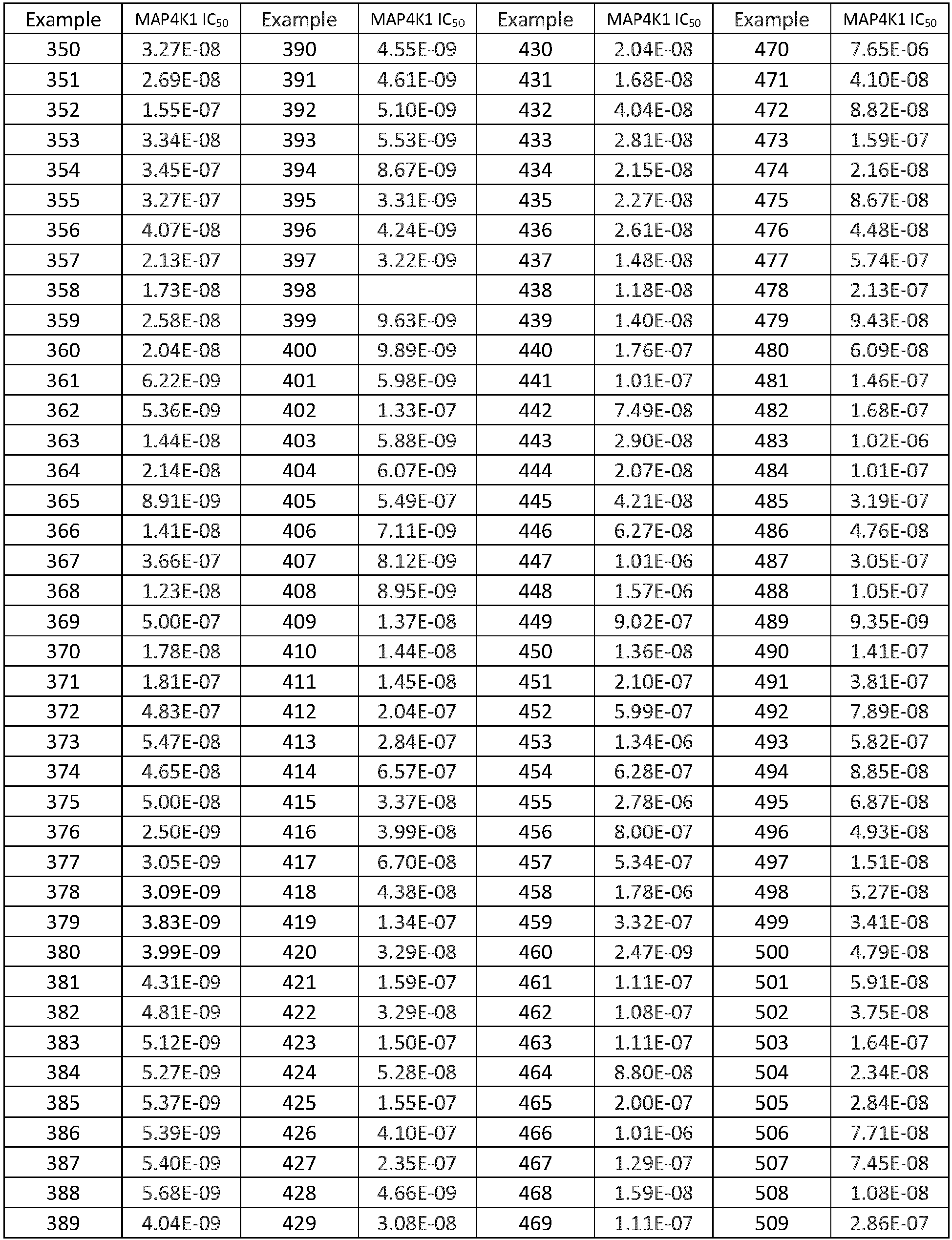

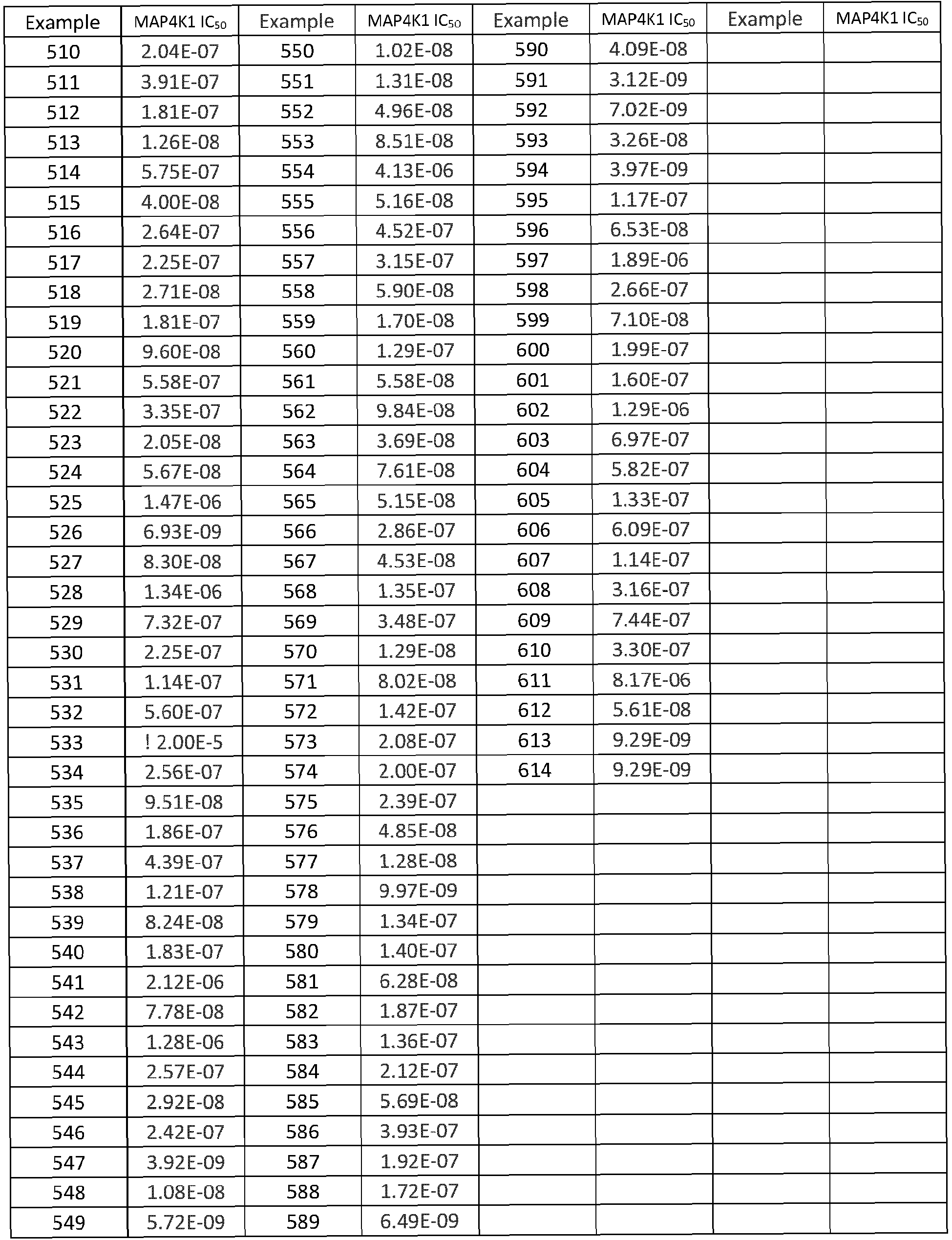

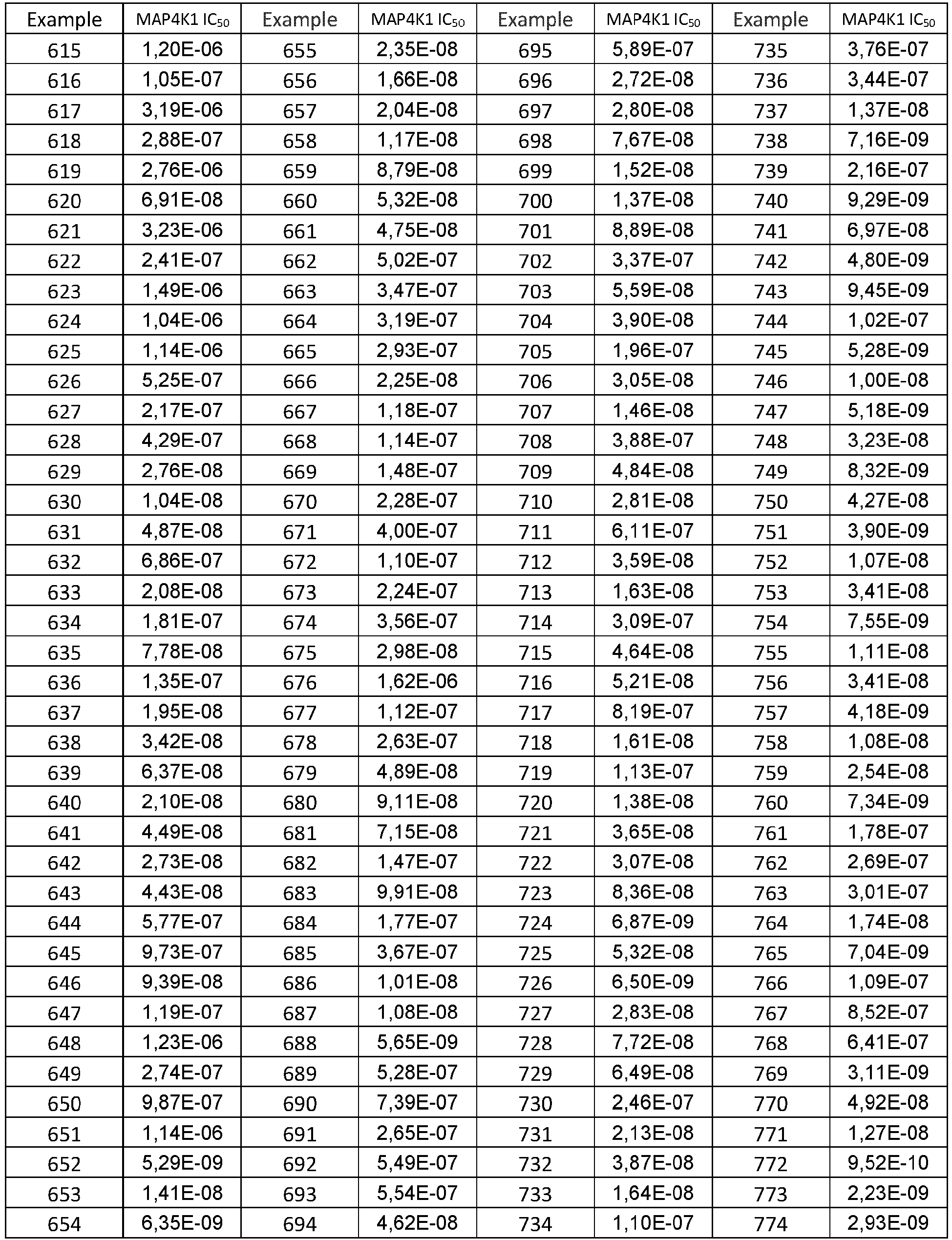

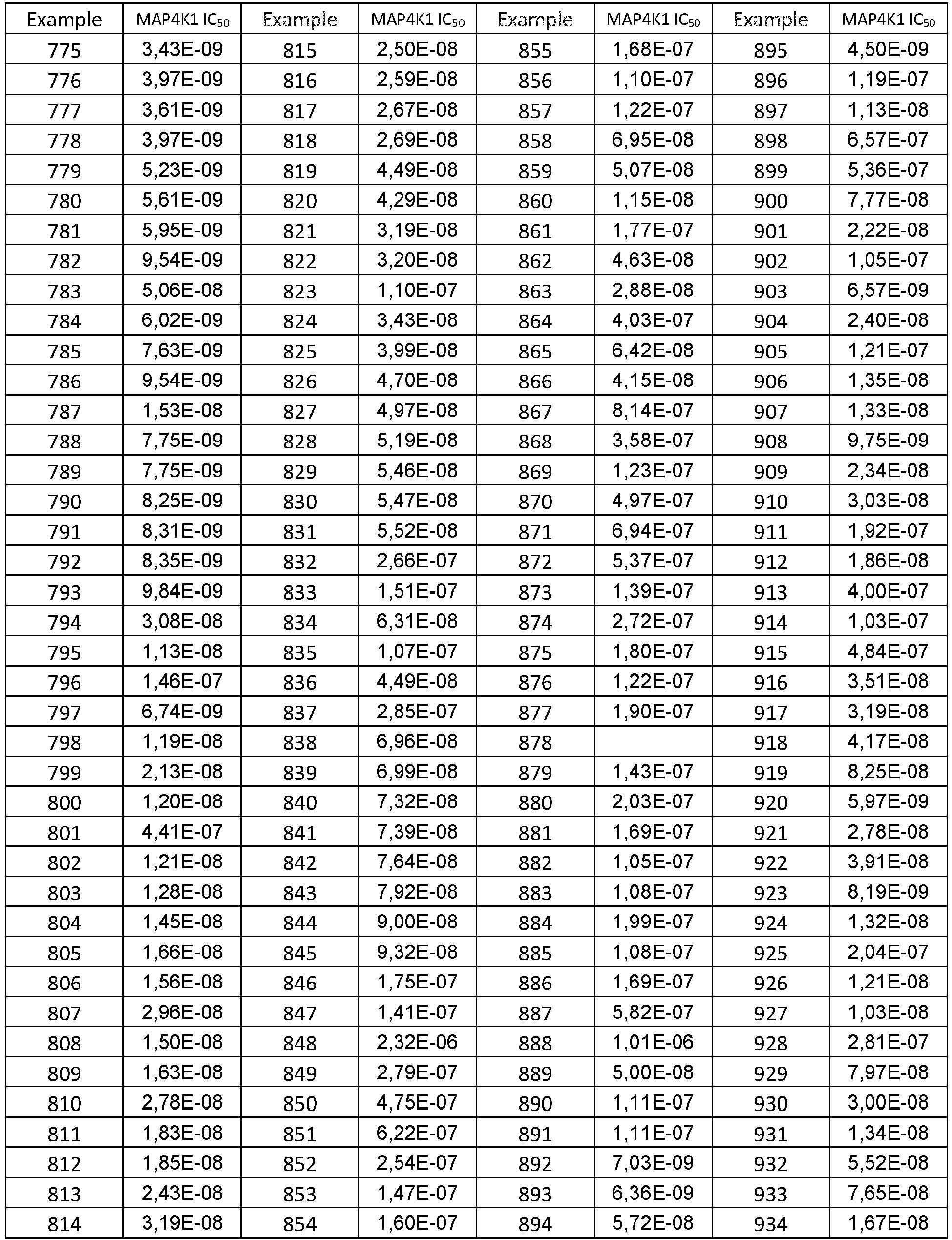

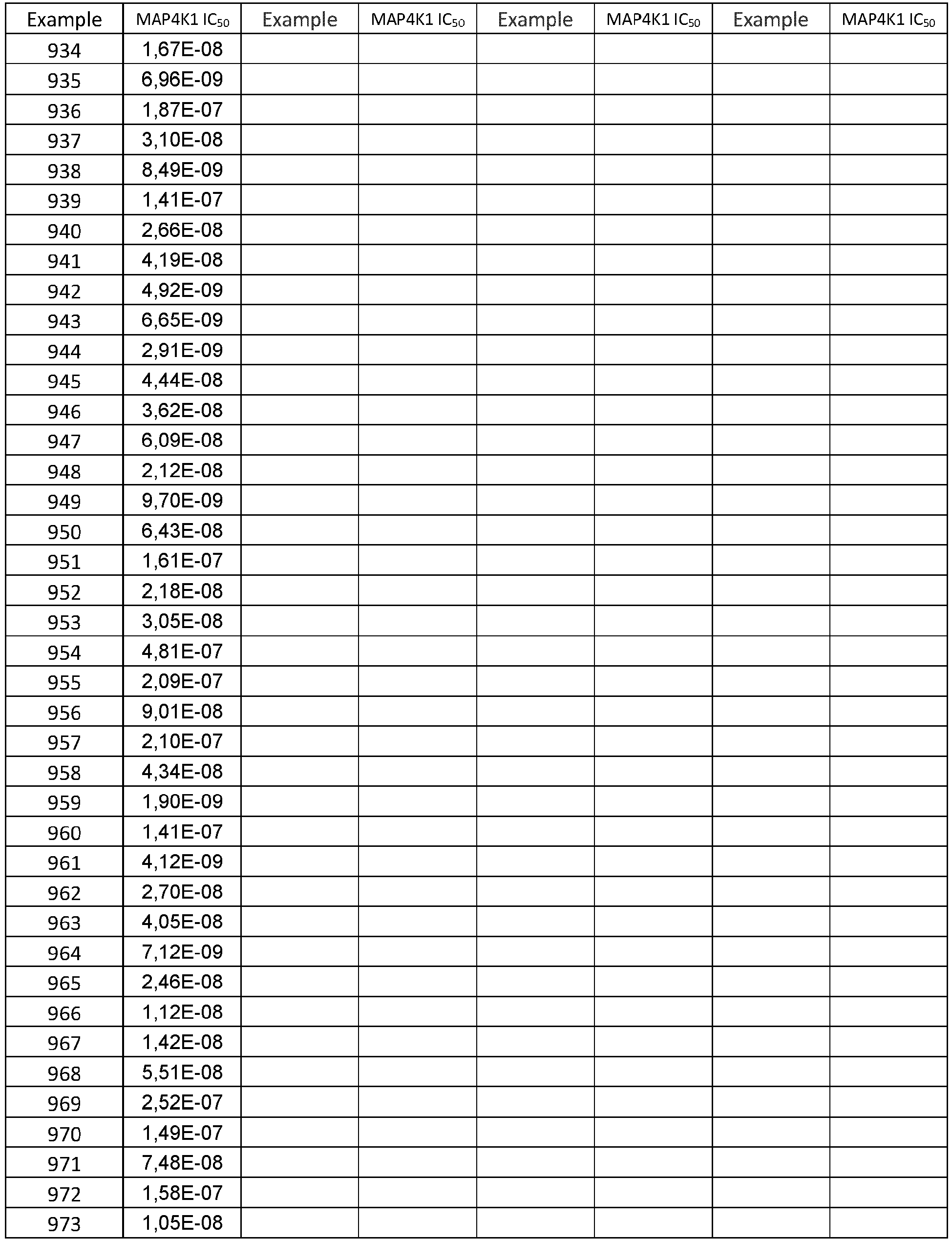

## Claims

1. Compound of formula (I) 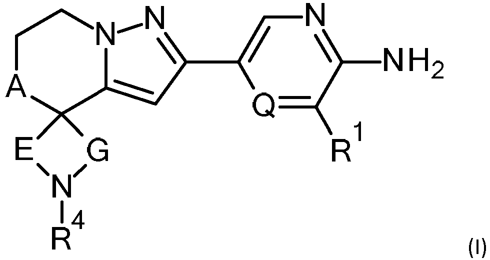 wherein
A is selected from a single bond, -CH₂- or -O-;
E is -(CH₂)ₙ- with n = 1 or 2;
G is -(CH₂)- and with the condition, that the cyclic substructure comprising E-N-G is a 4 to 5 membered heterocycloalkyl;
Q is selected from N or C-R²;
R¹ is selected from
• -NO₂; -CN;
• C₁₋₄-alkyl optionally substituted by -OH or 1 to 3 -F;
• cyclopropyl;
• 4 to 6 membered heterocycloalkyl or heterocycloalkenyl, both with 1- 2 nitrogen atoms, but always linked via a carbon atom to the phenyl group of compounds of formula (I) and both optionally substituted by C₁₋₄-alkyl;
• phenyl optionally substituted by halogen, -CN, C₁₋₄-alkyl, C₁₋₄-haloalkyl, C₃₋₆-cycloalkyl, O-C₁₋₄-alkyl, O-C₃₋₆-cycloalky, C₁₋₃-alkinyl, -C(O)-R_{d}, SR_{d}, SOR_{d}, SO₂R_{d}, SONR_{d}, SON(H)R_{d}, -SO₂-NH₂, -SO₂-N(H)-R_{d} or -SO₂-N(R₄)₂, wherein R_{d} is selected from methyl, ethyl, cyclopropyl, isopropyl and tert-butyl;
• 5 to 6 membered heteroaryl with 1, 2 or 3 heteroatoms selected from N, O or S and optionally substituted by 1 or 2 of the following substituents: halogen, -CN, C₁₋₄-alkyl, C₁₋₄-haloalkyl, C₃₋₆-cycloalkyl, O-C₁₋₄-alkyl, O-C₃₋₆-cycloalkyl, C₁₋₃-alkinyl, SR_{d}, SOR_{d}, SO₂R_{d}, SONR_{d} or SON(H)R_{d} wherein R_{d} is selected from methyl, ethyl, cyclopropyl, isopropyl and tert-butyl;
• -O-CH₃; -O-CH(CH₃)₂; -O-CHF₂; -O-CF₃; 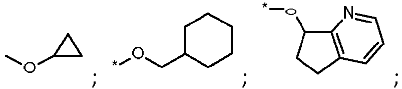
• -O-CHRₐ-phenyl optionally substituted by one, two or three of the following substituents: halogen, -CN, C₁₋₄-alkyl optionally substituted by one or more -F, O-C₁₋₄-alkyl, C₁₋₆-alkinyl-OH or -SO₂-CH₃ and with Rₐ selected from -H, -F or -CH₃, -CF₃, -CH₂-CH₃, -CH(CH₃)₂ or -cyclopropyl;
• -O-CHRₐ-6 membered heteroaryl optionally substituted one, two or three of the following substituents: halogen, -CN, C₁₋₄-alkyl optionally substituted by one or more -F or -SO₂-CH₃ and with Rₐ selected from -H, -F or -CH₃, -CF₃, -CH₂-CH₃, -CH(CH₃)₂ or -cyclopropyl;
• -COORₐ with Rₐ selected from -H or -CH₃;
• C(=O)-methyl, optionally substituted by 1 to 3 -F;
• -C(=O)-NR_{b}R_{c} wherein R_{b} is selected from -H, -CH₃ or -CH₂-CH₃ and wherein R_{c} is selected from - H, -CH₃, -CH₂-CH₃, cyclohexyl, phenyl or 5 to 6 membered heteroaryl, all optionally substituted by one or two halogen, -CN, -SO₂-CH₃ or C₁₋₄-alkyl, optionally substituted one or more times with halogen;
• -S-CR_{f}R_{g}Rₕ, wherein R_{f} is selected from -H or -F, wherein R_{g} is selected from -H, -F or -CH₃ and wherein Rₕ is selected from H, F, -CH₃ or C₅₋₆-cycloalkyl or phenyl;
• 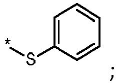
R² is selected from -H;
R⁴ is selected from
• C₁₋₆-alkyl, C₃₋₆-cycloalkyl or C₁₋₃alkyl-C₃₋₆-cycloalkyl, all optionally substituted one or independently more times by
∘ -F, -Cl, -Br, -CN;
∘ -OH, -ORᵢ wherein Rᵢ is selected from C₁₋₄-alkyl or C₃₋₆-Cycloalkyl, both optionally substituted one or more times by halogen,
∘ -CF₃, -CHF₂, -CH₂F, or -CH₂-CF₃;
∘ -NRⱼRₖ wherein Rⱼ and Rₖ are independently selected from -H and C₁-C₄-alkyl,
∘ -CH₂-O-CH₃ or
∘ C₃₋₄ cycloalkyl or 4-6 membered heterocycloalkyl, both optionally substituted by (=O), or optionally substituted one or more times by F or CH₃;
∘ C₆₋₈-spiro-alkyl and C₅₋₇-bicyclo-alkyl
∘ phenyl or 5 to 6 membered heteroaryl, both optionally substituted one or more times by
▪ halogen, -CN,
▪ -CF₃, -CHF₂, -CH₂F,
▪ C₁-₄-alkyl optionally substituted with one or more halogen; ▪ C₃₋₆-cycloalkyl;
▪ O-C₁₋₄-alkyl,
▪ O-C₃₋₆-cycloalky,
▪ C₁₋₃-alkinyl,
▪ -NRⱼRₖ wherein Rⱼ and Rₖ are independently selected from -H and C₁-C₄-alkyl ▪ SR_{d}, SOR_{d}, SO₂R_{d}, SONR_{d} or SON(H)R_{d} wherein R_{d} is selected from methyl, ethyl, cyclopropyl, isopropyl and tert-butyl; or
∘ phenyl is optionally substituted one or more times by -C(O)NRⱼRₖ wherein Rⱼ and Rₖ are independently selected from -H and C₁-C₄-alkyl or
∘ 5 to 6 membered heteroaryl is optionally substituted one or more times by -OH;
• C(=O)-R_{I} in which R_{I} is selected from
∘ C₁₋₄-alkyl, C₃₋₆-cycloalkyl or C₃₋₆-heterocycloalkyl, both optionally substituted one or more times by
▪ -F, -Cl, -Br, -CN;
▪ -OH, -ORᵢ with Rᵢ selected from C₁₋₄-alkyl or C₃₋₆Cycloalkyl), -CF₃, -CHF₂, -CH₂F; ▪ -NRⱼRₖ wherein Rⱼ and Rₖ are independently selected from -H and C₁-C₄-alkyl ▪ phenyl or 5 to 6 membered heteroaryl, both optionally substituted one or more times by
• halogen, -CN,
• -CF₃, -CHF₂, -CH₂F,
• C₁-₄-alkyl optionally substituted with one or more halogen;
• C₃₋₆-cycloalkyl;
• O-C₁₋₄-alkyl,
• O-C₃₋₆-cycloalky,
• C₁₋₃-alkinyl,
• -NRⱼRₖ wherein Rⱼ and Rₖ are independently selected from -H and C₁-C₄-alkyl
• SR_{d}, SOR_{d}, SO₂R_{d}, SONR_{d} or SON(H)R_{d} wherein R_{d} is selected from methyl, ethyl, cyclopropyl, isopropyl and tert-butyl;
o phenyl or 5 to 6 membered heteroaryl, both optionally substituted one or more times by
▪ halogen, -CN,
▪ -CF₃, -CHF₂, -CH₂F,
▪ C₁₋₄-alkyl optionally substituted with one or more halogen;
▪ C₃₋₆-cycloalkyl;
▪ O-C₁₋₄-alkyl,
▪ O-C₃₋₆-cycloalky,
▪ C₁₋₃-alkinyl,
▪ -NRⱼRₖ wherein Rⱼ and Rₖ are independently selected from -H and C₁-C₄-alkyl
▪ SR_{d}, SOR_{d}, SO₂R_{d}, SONR_{d} or SON(H)R_{d} wherein R_{d} is selected from methyl, ethyl, cyclopropyl, isopropyl and tert-butyl;
• C(=O)-NRₒRₚ wherein Rₒ and Rₚ are independently selected from
∘ H
∘ C₁₋₄-alkyl, C₃₋₆-cycloalkyl or C₁₋₃alkyl-C₃₋₆-cycloalkyl, both optionally substituted one or more times by
▪ -F, -Cl, -Br, -CN;
▪ -OH, -ORᵢ with Rᵢ selected from C₁₋₄-alkyl or C₃₋₆-cycloalkyl, -CF₃, -CHF₂, -CH₂F; ▪ -NRⱼRₖ wherein Rⱼ and Rₖ are independently selected from -H and C₁-C₄-alkyl ▪ phenyl or 5 to 6 membered heteroaryl, both optionally substituted one or more times by
• halogen, -CN,
• -CF₃, -CHF₂, -CH₂F,
• C₁₋₄-alkyl optionally substituted with one or more halogen;
• C₃₋₆-cycloalkyl;
• O-C₁₋₄-alkyl,
• O-C₃₋₆-cycloalky,
• C₁₋₃-alkinyl,
• -NRⱼRₖ wherein Rⱼ and Rₖ are independently selected from -H and C₁-C₄-alkyl
• SR_{d}, SOR_{d}, SO₂R_{d}, SONR_{d} or SON(H)R_{d} wherein R_{d} is selected from methyl, ethyl, cyclopropyl, isopropyl and tert-butyl;
o phenyl or 5 to 6 membered heteroaryl, both optionally substituted one or more times by
▪ halogen, -CN,
▪ -CF₃, -CHF₂, -CH₂F,
▪ C₁₋₄-alkyl optionally substituted with one or more halogen;
▪ C₃₋₆-cycloalkyl;
▪ O-C₁₋₄-alkyl,
▪ O-C₃₋₆-cycloalky,
▪ C₁₋₃-alkinyl,
▪ -NRⱼRₖ wherein Rⱼ and Rₖ are independently selected from -H and C₁-C₄-alkyl ▪ SR_{d}, SOR_{d}, SO₂R_{d}, SONR_{d} or SON(H)R_{d} wherein R_{d} is selected from methyl, ethyl,
cyclopropyl, isopropyl and tert-butyl;
or a stereoisomer, a tautomer, an N-oxide, a hydrate, a solvate, or a salt thereof, or a mixture of same.

2. Compound of formula (la) according to claim 1 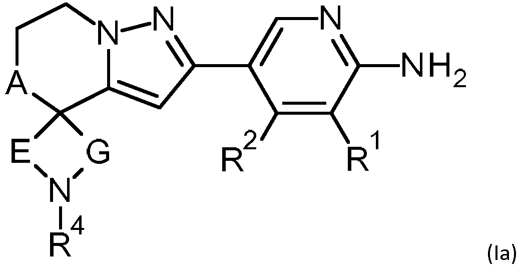 wherein
A is selected from a single bond, -CH₂- or -O-;
E is -(CH₂)ₙ- with n = 1 or 2;
G is -(CH₂)- and with the condition, that the cyclic substructure comprising E-N-G is a 4 to 5 membered heterocycloalkyl;
R¹ is selected from
• -NO₂; -CN;
• C₁₋₄-alkyl optionally substituted by -OH or 1 to 3 -F;
• cyclopropyl;
• 4 to 6 membered heterocycloalkyl or heterocycloalkenyl, both with 1- 2 nitrogen atoms, but always linked via a carbon atom to the phenyl group of compounds of formula (I) and both optionally substituted by C₁₋₄-alkyl;
• phenyl optionally substituted by halogen, -CN, C₁₋₄-alkyl, C₁₋₄-haloalkyl, C₃₋₆-cycloalkyl, O-C₁₋₄-alkyl, O-C₃₋₆-cycloalky, C₁₋₃-alkinyl, -C(O)-R_{d}, SR_{d}, SOR_{d}, SO₂R_{d}, SONR_{d}, SON(H)R_{d}, -SO₂-NH₂, -SO₂-N(H)-R_{d} or -SO₂-N(R₄)₂, wherein R_{d} is selected from methyl, ethyl, cyclopropyl, isopropyl and tert-butyl;
• 5 to 6 membered heteroaryl with 1 or 2 heteroatoms selected from N or S and optionally substituted by halogen, -CN, C₁₋₄-alkyl, C₁₋₄-haloalkyl, C₃₋₆-cycloalkyl, O-C₁₋₄-alkyl, O-C₃₋₆-cycloalkyl, C₁₋₃-alkinyl, SR_{d}, SOR_{d}, SO₂R_{d}, SONR_{d} or SON(H)R_{d} wherein R_{d} is selected from methyl, ethyl, cyclopropyl, isopropyl and tert-butyl;
• -O-CH₃; -O-CH(CH₃)₂; -O-CHF₂; -O-CF₃; 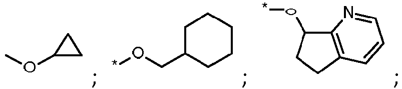
• -O-CHRₐ-phenyl optionally substituted by one or two of the following substituents: halogen, - CN, C₁₋₄-alkyl optionally substituted by one or more -F or -SO₂-CH₃ and with Rₐ selected from - H, -F or -CH₃, -CF₃, -CH₂-CH₃, -CH(CH₃)₂ or -cyclopropyl;
• -O-CHRₐ-6 membered heteroaryl optionally substituted one or two of the following substituents: halogen, -CN, C₁₋₄-alkyl optionally substituted by one or more -F or -SO₂-CH₃ and with Rₐ selected from -H, -F or -CH₃, -CF₃, -CH₂-CH₃, -CH(CH₃)₂ or -cyclopropyl;
• -COORₐ with Rₐ selected from -H or -CH₃;
• C(=O)-methyl, optionally substituted by 1 to 3 -F;
• -C(=O)-NR_{b}R_{c} wherein R_{b} is selected from -H, -CH₃ or -CH₂-CH₃ and wherein R_{c} is selected from - H, -CH₃, -CH₂-CH₃, cyclohexyl, phenyl or 5 to 6 membered heteroaryl, all optionally substituted by one or two halogen, -CN, -SO₂-CH₃ or C₁₋₄-alkyl, optionally substituted one or more times with halogen;
• -S-CR_{f}R_{g}Rₕ, wherein R_{f} is selected from -H or -F, wherein R_{g} is selected from -H, -F or -CH₃ and wherein Rₕ is selected from H, F, -CH₃ or C₅₋₆-cycloalkyl or phenyl;
• 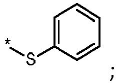
R² is selected from -H,; or
R⁴ is selected from
• C₁₋₅-alkyl or C₃₋₆-cycloalkyl, both optionally substituted one or independently more times by
∘ -F, -Cl, -Br, -CN;
∘ -OH, -ORᵢ wherein Rᵢ is selected from C₁₋₄-alkyl or C₃₋₆Cycloalkyl),
∘ -CF₃, -CHF₂, -CH₂F, or -CH₂-CF₃;
∘ -NRⱼRₖ wherein Rⱼ and Rₖ are independently selected from -H and C₁-C₄-alkyl,
∘ -CH₂-O-CH₃ or
∘ C₃₋₄ cycloalkyl or 4 membered heterocycloalkyl, both optionally substituted by -F or - CH₃;
∘ phenyl or 5 to 6 membered heteroaryl, both optionally substituted one or more times by
▪ halogen, -CN,
▪ -CF₃, -CHF₂, -CH₂F,
▪ C₁₋₄-alkyl optionally substituted with one or more halogen;
▪ C₃₋₆-cycloalkyl;
▪ O-C₁₋₄-alkyl,
▪ O-C₃₋₆-cycloalky,
▪ C₁₋₃-alkinyl,
▪ -NRⱼRₖ wherein Rⱼ and Rₖ are independently selected from -H and C₁-C₄-alkyl
▪ SR_{d}, SOR_{d}, SO₂R_{d}, SONR_{d} or SON(H)R_{d} wherein R_{d} is selected from methyl, ethyl, cyclopropyl, isopropyl and tert-butyl;
• C(=O)-R_{I} in which R_{I} is selected from
∘ C₁₋₄-alkyl, C₃₋₆-cycloalkyl or C₃₋₆-heterocycloalkyl, both optionally substituted one or more times by
▪ -F, -Cl, -Br, -CN;
▪ -OH, -ORᵢ with Rᵢ selected from C₁₋₄-alkyl or C₃₋₆Cycloalkyl), -CF₃, -CHF₂, -CH₂F; ▪ -NRⱼRₖ wherein Rⱼ and Rₖ are independently selected from -H and C₁-C₄-alkyl ▪ phenyl or 5 to 6 membered heteroaryl, both optionally substituted one or more times by
• halogen, -CN,
• -CF₃, -CHF₂, -CH₂F,
• C₁₋₄-alkyl optionally substituted with one or more halogen;
• C₃₋₆-cycloalkyl;
• O-C₁₋₄-alkyl,
• O-C₃₋₆-cycloalky,
• C₁₋₃-alkinyl,
• -NRⱼRₖ wherein Rⱼ and Rₖ are independently selected from -H and C₁-C₄-alkyl
• SR_{d}, SOR_{d}, SO₂R_{d}, SONR_{d} or SON(H)R_{d} wherein R_{d} is selected from methyl, ethyl, cyclopropyl, isopropyl and tert-butyl;
∘ phenyl or 5 to 6 membered heteroaryl, both optionally substituted one or more times by
▪ halogen, -CN,
▪ -CF₃, -CHF₂, -CH₂F,
▪ C₁₋₄-alkyl optionally substituted with one or more halogen;
▪ C₃₋₆-cycloalkyl;
▪ O-C₁₋₄-alkyl,
▪ O-C₃₋₆-cycloalky,
▪ C₁₋₃-alkinyl,
▪ -NRⱼRₖ wherein Rⱼ and Rₖ are independently selected from -H and C₁-C₄-alkyl
▪ SR_{d}, SOR_{d}, SO₂R_{d}, SONR_{d} or SON(H)R_{d} wherein R_{d} is selected from methyl, ethyl, cyclopropyl, isopropyl and tert-butyl;
• C(=O)-NRₒRₚ wherein Rₒ and Rₚ are independently selected from
∘ H
∘ C₁₋₄-alkyl or C₃₋₆-cycloalkyl, both optionally substituted one or more times by
▪ -F, -Cl, -Br, -CN;
▪ -OH, -ORᵢ with Rᵢ selected from C₁₋₄-alkyl or C₃₋₆-cycloalkyl, -CF₃, -CHF₂, -CH₂F;
▪ -NRⱼRₖ wherein Rⱼ and Rₖ are independently selected from -H and C₁-C₄-alkyl
▪ phenyl or 5 to 6 membered heteroaryl, both optionally substituted one or more times by
• halogen, -CN,
• -CF₃, -CHF₂, -CH₂F,
• C₁₋₄-alkyl optionally substituted with one or more halogen;
• C₃₋₆-cycloalkyl;
• O-C₁₋₄-alkyl,
• O-C₃₋₆-cycloalky,
• C₁₋₃-alkinyl,
• -NRⱼRₖ wherein Rⱼ and Rₖ are independently selected from -H and C₁-C₄-alkyl
• SR_{d}, SOR_{d}, SO₂R_{d}, SONR_{d} or SON(H)R_{d} wherein R_{d} is selected from methyl, ethyl, cyclopropyl, isopropyl and tert-butyl;
o phenyl or 5 to 6 membered heteroaryl, both optionally substituted one or more times by
▪ halogen, -CN,
▪ -CF₃, -CHF₂, -CH₂F,
▪ C₁₋₄-alkyl optionally substituted with one or more halogen;
▪ C₃₋₆-cycloalkyl;
▪ O-C₁₋₄-alkyl,
▪ O-C₃₋₆-cycloalky,
▪ C₁₋₃-alkinyl,
▪ -NRⱼRₖ wherein Rⱼ and Rₖ are independently selected from -H and C₁-C₄-alkyl
▪ SR_{d}, SOR_{d}, SO₂R_{d}, SONR_{d} or SON(H)R_{d} wherein R_{d} is selected from methyl, ethyl, cyclopropyl, isopropyl and tert-butyl;
or a stereoisomer, a tautomer, an N-oxide, a hydrate, a solvate, or a salt thereof, or a mixture of same.

3. Compounds of formula (I) according to claim 1, wherein A is selected from a single bond or -O-or a stereoisomer, a tautomer, an N-oxide, a hydrate, a solvate, or a salt thereof, or a mixture of same.

4. Compounds of formula (I) according to claim 1, wherein E is -CH₂-CH₂- or a stereoisomer, a tautomer, an N-oxide, a hydrate, a solvate, or a salt thereof, or a mixture of same.

5. Compounds of formula (I) according to claim 1, wherein Q is C-R² or a stereoisomer, a tautomer, an N-oxide, a hydrate, a solvate, or a salt thereof, or a mixture of same.

6. Compounds of formula (I) according to claim 1, wherein Q is N or a stereoisomer, a tautomer, an N-oxide, a hydrate, a solvate, or a salt thereof, or a mixture of same.

7. Compounds of formula (I) according to claim 1, wherein R₄ is 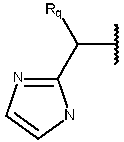 and R_{q} is selected from H, -CH₃,-CH₂-CH₃, -CH(CH₃)₂, -CH₂-O-CH₃, -CF₃, -CHF₂, -CH₂F, -CH₂-CF₃, cyclopropyl or cyclobutyl or a stereoisomer, a tautomer, an N-oxide, a hydrate, a solvate, or a salt thereof, or a mixture of same.

8. Compounds of formula (I) according to claim 1, wherein R₄ is selected from C(=O)-NRₒRₚ wherein Rₒ and Rₚ are independently selected from
∘ H
∘ C₁₋₄-alkyl or C₃₋₆-cycloalkyl, both optionally substituted one or more times by
▪ -F, -Cl, -Br, -CN;
▪ -OH, -ORᵢ with Rᵢ selected from C₁₋₄-alkyl or C₃₋₆-cycloalkyl, -CF₃, -CHF₂, -CH₂F; ▪ -NRⱼRₖ wherein Rⱼ and Rₖ are independently selected from -H and C₁-C₄-alkyl
▪ phenyl or 5 to 6 membered heteroaryl, both optionally substituted one or more times by
• halogen, -CN,
• -CF₃, -CHF₂, -CH₂F,
• C₁₋₄-alkyl optionally substituted with one or more halogen;
• C₃₋₆-cycloalkyl;
• O-C₁₋₄-alkyl,
• O-C₃₋₆-cycloalky,
• C₁₋₃-alkinyl,
• -NRⱼRₖ wherein Rⱼ and Rₖ are independently selected from -H and C₁-C₄-alkyl
• SR_{d}, SOR_{d}, SO₂R_{d}, SONR_{d} or SON(H)R_{d} wherein R_{d} is selected from methyl, ethyl, cyclopropyl, isopropyl and tert-butyl;
or a stereoisomer, a tautomer, an N-oxide, a hydrate, a solvate, or a salt thereof, or a mixture of same.

9. Compound according to claim 1, which is selected from:
(*rac*)-2'-[6-amino-5-(trifluoromethyl)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-(1-phenylcyclobutyl)-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-(1-phenylcyclobutyl)-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(rac)-2'-(5-acetyl-6-aminopyridin-3-yl)-N-(1-phenylcyclobutyl)-5',6'-dihydrospiro[pyrrolidine-3,4'-pyrrolo[1,2-b]pyrazole]-1-carboxamide
(rac)-2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide
(rac)-2'-(6-amino-5-cyanopyridin-3-yl)-N-[1-(2-chlorophenyl)cyclobutyl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
(rac)-2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(rac)-2'-(6-amino-5-cyanopyridin-3-yl)-N-(1-phenylcyclobutyl)-5',6'-dihydrospiro[pyrrolidine-3,4'-pyrrolo[1,2-b]pyrazole]-1-carboxamide
(rac)-2-(6-amino-5-cyanopyridin-3-yl)-N-[1-(4-fluorophenyl)cyclobutyl]-6,7-dihydrospiro[pyrazolo[5,1-c][1,4]oxazine-4,3'-pyrrolidine]-1'-carboxamide
(rac)-2'-(6-amino-5-cyanopyridin-3-yl)-N-[1-(4-fluorophenyl)cyclobutyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide
(rac)-2'-[6-amino-5-(trifluoromethyl)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide
(rac)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide
(rac)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide
(rac)-2-(6-amino-5-cyanopyridin-3-yl)-N-(1-phenylcyclobutyl)-6,7-dihydrospiro[pyrazolo[5,1-c][1,4]oxazine-4,3'-pyrrolidine]-1'-carboxamide
(rac)-2-(6-amino-5-cyanopyridin-3-yl)-N-[2-(4-fluorophenyl)propan-2-yl]-6,7-dihydrospiro[pyrazolo[5,1-c][1,4]oxazine-4,3'-pyrrolidine]-1'-carboxamide
(rac)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(rac)-2-[6-amino-5-(trifluoromethyl)pyridin-3-yl]-N-[1-(3-fluorophenyl)cyclobutyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-{(1R)-1-[3-(trifluoromethyl)phenyl]ethyl}-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(2-fluorophenyl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(3-fluorophenyl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-phenylpropyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-{(1S)-1-[3-(trifluoromethyl)phenyl]ethyl}-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(2-cyanophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1,3,5-trimethyl-1H-pyrazol-4-yl)ethyl]-5',6'-dihydrospiro[pyrrolidine-3,4'-pyrrolo[1,2-b]pyrazole]-1-carboxamide (diastereomeric mixture)
2'-[6-amino-5-(trifluoromethyl)pyridin-3-yl]-N-[(1R)-2,3-dihydro-1H-inden-1-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(2-cyanopyridin-4-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-ethyl-3,5-dimethyl-1H-pyrazol-4-yl)ethyl]-5',6'-dihydrospiro[pyrrolidine-3,4'-pyrrolo[1,2-b]pyrazole]-1-carboxamide (diastereometric mixture)
(rac)-2'-[6-amino-5-(trifluoromethyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1,3,4-trimethyl-1H-pyrazol-5-yl)ethyl]-5',6'-dihydrospiro[pyrrolidine-3,4'-pyrrolo[1,2-b]pyrazole]-1-carboxamide (diastereometric mixture)
2'-[6-amino-5-(trifluoromethyl)pyridin-3-yl]-N-[(1R)-1-(4-cyanopyridin-2-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(pyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-{[3-(trifluoromethyl)phenyl]methyl}-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-phenyl-1H-pyrazol-4-yl)ethyl]-5',6'-dihydrospiro[pyrrolidine-3,4'-pyrrolo[1,2-b]pyrazole]-1-carboxamide (diastereometric mixture)
2'-[6-amino-5-(trifluoromethyl)pyridin-3-yl]-N-[(3S)-2,3-dihydro-1-benzofuran-3-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(6-cyanopyridin-3-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-{(1S)-1-[1-methyl-3-(trifluoromethyl)-1H-pyrazol-4-yl]ethyl}-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(pyridin-2-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-benzyl-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-phenylethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1,2-oxazol-3-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1,5-dimethyl-1H-pyrazol-4-yl)ethyl]-5',6'-dihydrospiro[pyrrolidine-3,4'-pyrrolo[1,2-b]pyrazole]-1-carboxamide (diastereomeric mixture)
(*rac*)-2'-[6-amino-5-(trifluoromethyl)pyridin-3-yl]-N-[2-(oxan-4-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(pyridin-4-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(15)-1-(1-ethyl-5-methyl-1H-pyrazol-4-yl)ethyl]-5',6'-dihydrospiro[pyrrolidine-3,4'-pyrrolo[1,2-b]pyrazole]-1-carboxamide (diastereomeric mixture)
(*rac*)-2'-[6-amino-5-(trifluoromethyl)pyridin-3-yl]-N-[(pyridin-3-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-2-methoxy-1-phenylethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-{[3-(trifluoromethyl)bicyclo[1.1.1]pentan-1-yl]methyl}-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(pyridin-4-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(3-cyanopyridin-2-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1-methyl-1H-benzimidazol-2-yl)ethyl]-5',6'-dihydrospiro[pyrrolidine-3,4'-pyrrolo[1,2-b]pyrazole]-1-carboxamide (diastereomeric mixture)
2'-[6-amino-5-(trifluoromethyl)pyridin-3-yl]-N-[1-(4-fluorophenyl)-2-methoxyethyl]-5',6'-dihydrospiro[pyrrolidine-3,4'-pyrrolo[1,2-b]pyrazole]-1-carboxamide (mixture of isomers)
(*rac*)-2'-[6-amino-5-(trifluoromethyl)pyridin-3-yl]-N-[(pyridin-2-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(1-ethyl-1H-imidazol-2-yl)ethyl]-5',6'-dihydrospiro[pyrrolidine-3,4'-pyrrolo[1,2-b]pyrazole]-1-carboxamide (diastereomeric mixture)
2'-[6-amino-5-(trifluoromethyl)pyridin-3-yl]-N-[(1S)-1-(1-methyl-1H-imidazol-2-yl)ethyl]-5',6'-dihydrospiro[pyrrolidine-3,4'-pyrrolo[1,2-b]pyrazole]-1-carboxamide (diastereomeric mixture)
(*rac*)-2'-[6-amino-5-(trifluoromethyl)pyridin-3-yl]-N-[(pyrazin-2-yl)methyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-2-methoxy-1-(pyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(pyridazin-4-yl)methyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(1-methylpiperidin-4-yl)methyl]-5',6'-dihydrospiro[pyrrolidine-3,4'-pyrrolo[1,2-b]pyrazole]-1-carboxamide
*(rac)-2'-[6-amino-5-(trifluoromethyl)pyridin-3-yl]-N-[3-(methylsulfamoyl)pyridin-2-yl]-5',6'-*dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(1-methyl-2-oxabicyclo[2.1.1]hexan-4-yl)methyl]-5',6'-dihydrospiro[pyrrolidine-3,4'-pyrrolo[1,2-b]pyrazole]-1-carboxamide
*(rac)-2'-[6-amino-5-(trifluoromethyl)pyridin-3-yl]-N-[(1-methyl-2-oxopiperidin-4-yl)methyl]-5',6'-*dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(2-cyclopropylpropan-2-yl)-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(4-cyanophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-methyl-2,3-dihydro-1H-inden-1-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[-2-methyl-1-phenylpropyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[2,2,2-trifluoro-1-phenylethyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[1-(pyrazin-2-yl)ethyl]-5',6'-dihydrospiro[pyrrolidine-3,4'-pyrrolo[1,2-b]pyrazole]-1-carboxamide (mixture of isomers)
2'-[6-amino-5-(trifluoromethyl)pyridin-3-yl]-N-[(1R)-2-methoxy-1-phenylethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[(pyridazin-3-yl)methyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(2-cyanophenyl)cyclobutyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(15)-2,3-dihydro-1H-inden-1-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(4-fluorophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-(2-cyanophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(6-cyanopyridin-3-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(pyridin-2-yl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyridin-4-yl)methyl]-5',6'-dihydrospiro[azetidine-3,4'-pyrrolo[1,2-b]pyrazole]-1-carboxamide
(3'RS)-2-[6-amino-5-(trifluoromethyl)pyridin-3-yl]-N-[(1R)-1-(4-cyanophenyl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(3-cyanopyridin-2-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(4-cyanophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(2-chloro-4-cyanophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(2-fluorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1-ethyl-3,5-dimethyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(pyridin-2-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1-methyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1,3,4-trimethyl-1H-pyrazol-5-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-{1-[3-(difluoromethyl)-1-methyl-1H-pyrazol-4-yl]ethyl}-5',6'-dihydrospiro[azetidine-3,4'-pyrrolo[1,2-b]pyrazole]-1-carboxamide
(*rac*)-2-[6-amino-5-(trifluoromethyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[1-(1-ethyl-5-methyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1,5-dimethyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(1,3,5-trimethyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(pyridin-3-yl)ethyl]-5',6'-dihydrospiro[azetidine-3,4'-pyrrolo[1,2-b]pyrazole]-1-carboxamide
2-[6-amino-5-(trifluoromethyl)pyridin-3-yl]-N-[(1R)-1-(2-cyanopyridin-4-yl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(3-cyanopyridin-2-yl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(6-cyanopyridin-3-yl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(pyridin-2-yl)methyl]-5',6'-dihydrospiro[azetidine-3,4'-pyrrolo[1,2-b]pyrazole]-1-carboxamide
(rac)-2-[6-amino-5-(trifluoromethyl)pyridin-3-yl]-N-[(1R)-1-(2-cyanophenyl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(4-fluorophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(4-fluorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(pyridin-3-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(2-fluorophenyl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(pyridin-3-yl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(4-fluorophenyl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(pyridin-4-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(3-fluorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1S)-1-(3-chloropyridin-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1-methyl-2-oxabicyclo[2.1.1]hexan-4-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-{[3-(trifluoromethyl)bicyclo[1.1.1]pentan-1-yl]methyl}-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(2-chlorophenyl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(3-fluorophenyl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(3-fluoropyridin-2-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(2-chlorophenyl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(3-fluorophenyl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-phenylpropyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyrimidin-4-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-fluoropyridin-2-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyrimidin-5-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-chloropyridin-4-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-fluorophenyl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(pyridin-3-yl)methyl]-5',6'-dihydrospiro[azetidine-3,4'-pyrrolo[1,2-b]pyrazole]-1-carboxamide
(*rac*)-2-[6-amino-5-(trifluoromethyl)pyridin-3-yl]-N-benzyl-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[(1R)-1-(2-methylpyrimidin-5-yl)ethyl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[(1R)-1-(pyridin-3-yl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-benzyl-5',6'-dihydrospiro[azetidine-3,4'-pyrrolo[1,2-b]pyrazole]-1-carboxamide
2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide (enantiomer 1)
2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (enantiomer 1)
2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide (enantiomer 2)
2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (enantiomer 2)
2-[6-amino-5-(trifluoromethyl)pyridin-3-yl]-N-[(1R)-1-(4-cyanopyridin-2-yl)ethyl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-[2-(1-methyl-1H-1,2,3-triazol-5-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(4-fluorophenyl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-2,3-dihydro-1H-inden-1-yl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(5-fluoropyridin-3-yl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-(3-fluorophenyl)ethyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1R)-1-phenylpropyl]-6,7-dihydrospiro[pyrazolo[5,1-c][1,4]oxazine-4,3'-pyrrolidine]-1'-carboxamide
(3'RS)-2-[6-amino-5-(trifluoromethyl)pyridin-3-yl]-N-[(3S)-2,3-dihydro-1-benzofuran-3-yl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-[(bicyclo[1.1.1]pentan-1-yl)methyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(1,2-oxazol-3-yl)ethyl]-5',6'-dihydrospiro[azetidine-3,4'-pyrrolo[1,2-b]pyrazole]-1-carboxamide
2-[6-amino-5-(trifluoromethyl)pyridin-3-yl]-N-[(1R)-1-(2-fluorophenyl)ethyl]-6,7-dihydrospiro[pyrazolo[5,1-c][1,4]oxazine-4,3'-pyrrolidine]-1'-carboxamide
(rac)-2-[6-amino-5-(trifluoromethyl)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-[(1R)-1-(1-phenyl-1H-pyrazol-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1S)-2-methoxy-1-phenylethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(3-fluorobicyclo[1.1.1]pentan-1-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(bicyclo[1.1.1]pentan-1-yl)methyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1S)-1-(pyridin-3-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(2-methylpyrimidin-5-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[2-(3-chloropyridin-4-yl)propan-2-yl]-5',6'-dihydrospiro[azetidine-3,4'-pyrrolo[1,2-b]pyrazole]-1-carboxamide
2'-[6-amino-5-(trifluoromethyl)pyridin-3-yl]-N-[1-(pyridin-4-yl)cyclobutyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-phenylethyl]-5',6'-dihydrospiro[azetidine-3,4'-pyrrolo[1,2-b]pyrazole]-1-carboxamide
2-[6-amino-5-(trifluoromethyl)pyridin-3-yl]-N-[(1S)-1-(pyridin-3-yl)ethyl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[(3-fluorobicyclo[1.1.1]pentan-1-yl)methyl]-6,7-dihydrospiro[pyrazolo[5,1-c][1,4]oxazine-4,3'-pyrrolidine]-1'-carboxamide
2-[6-amino-5-(trifluoromethyl)pyridin-3-yl]-N-[(1S)-1-(pyridin-4-yl)ethyl]-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-(1-phenylcyclobutyl)-5',6'-dihydrospiro[azetidine-3,4'-pyrrolo[1,2-b]pyrazole]-1-carboxamide
2'-(6-amino-5-cyanopyridin-3-yl)-N-[1-(pyridin-4-yl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[1-(3-fluorophenyl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[2-(3-chloropyridin-4-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(5-fluoropyridin-3-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(4-fluorophenyl)ethyl]-5',6'-dihydrospiro[azetidine-3,4'-pyrrolo[1,2-b]pyrazole]-1-carboxamide
2'-(6-amino-5-cyanopyridin-3-yl)-N-[2-(3-fluorophenyl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[(1S)-1-(3-chloropyridin-4-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1-methyl-2-oxabicyclo[2.1.1]hexan-4-yl)methyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(4-cyanopyridin-2-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(2-cyanopyridin-4-yl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(2-chloro-4-cyanophenyl)ethyl]-5',6'-dihydrospiro[azetidine-3,4'-pyrrolo[1,2-b]pyrazole]-1-carboxamide
2'-(6-amino-5-cyanopyridin-3-yl)-N-[1-(2-fluorophenyl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[2-(4-fluorophenyl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[1-(2-chlorophenyl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[2-(pyridin-2-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[2-(pyridin-4-yl)propan-2-yl]-5',6'-dihydrospiro[azetidine-3,4'-pyrrolo[1,2-b]pyrazole]-1-carboxamide
2'-(6-amino-5-cyanopyridin-3-yl)-N-[(1R)-1-phenylpropyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(3-fluorophenyl)ethyl]-5',6'-dihydrospiro[azetidine-3,4'-pyrrolo[1,2-b]pyrazole]-1-carboxamide
2'-(6-amino-5-cyanopyridin-3-yl)-N-[(1R)-1-(2-cyanophenyl)ethyl]-5',6'-dihydrospiro[azetidine-3,4'-pyrrolo[1,2-b]pyrazole]-1-carboxamide
(*rac*)-2'-(6-amino-5-cyanopyridin-3-yl)-N-[2-(pyridin-4-yl)propan-2-yl]-5',6'-dihydrospiro[pyrrolidine-3,4'-pyrrolo[1,2-b]pyrazole]-1-carboxamide
2'-(6-amino-5-cyanopyridin-3-yl)-N-[(1R)-1-(5-fluoropyridin-3-yl)ethyl]-5',6'-dihydrospiro[pyrrolidine-3,4'-pyrrolo[1,2-b]pyrazole]-1-carboxamide
(rac)-2'-(6-amino-5-cyanopyridin-3-yl)-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(pyridin-2-yl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(pyridin-2-yl)cyclobutyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[1-(2-chlorophenyl)cyclobutyl]-5',6'-dihydrospiro[pyrrolidine-3,4'-pyrrolo[1,2-b]pyrazole]-1-carboxamide
2-(6-amino-5-cyanopyridin-3-yl)-N-[(1R)-1-phenylethyl]-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-phenylethyl]-5',6'-dihydrospiro[pyrrolidine-3,4'-pyrrolo[1,2-b]pyrazole]-1-carboxamide
(rac)-2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide
(rac)-2-(6-amino-5-cyanopyridin-3-yl)-N-[1-(4-fluorophenyl)cyclobutyl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[2-(4-fluorophenyl)propan-2-yl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide
2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (enantiomer 2)
2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide (enantiomer 2)
2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide (enantiomer 1)
2-(6-amino-5-cyanopyridin-3-yl)-N-[1-(2-chlorophenyl)cyclobutyl]-6,7-dihydrospiro[pyrazolo[5,1-c][1,4]oxazine-4,3'-pyrrolidine]-1'-carboxamide (enantiomer 1)
2'-(6-amino-5-cyanopyridin-3-yl)-N-[1-(pyridin-3-yl)cyclobutyl]-5',6'-dihydrospiro[azetidine-3,4'-pyrrolo[1,2-b]pyrazole]-1-carboxamide
2'-(6-amino-5-cyanopyridin-3-yl)-N-[1-(pyridin-2-yl)cyclobutyl]-5',6'-dihydrospiro[azetidine-3,4'-pyrrolo[1,2-b]pyrazole]-1-carboxamide
2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (enantiomer 1)
2'-(6-amino-5-cyanopyridin-3-yl)-N-(1-phenylcyclobutyl)-5',6'-dihydrospiro[azetidine-3,4'-pyrrolo[1,2-b]pyrazole]-1-carboxamide
2'-(6-amino-5-cyanopyridin-3-yl)-N-[(3S)-2,3-dihydro-1-benzofuran-3-yl]-5',6'-dihydrospiro[pyrrolidine-3,4'-pyrrolo[1,2-b]pyrazole]-1-carboxamide
2-(6-amino-5-cyanopyridin-3-yl)-N-[(3S)-2,3-dihydro-1-benzofuran-3-yl]-6,7-dihydrospiro[pyrazolo[5,1-c][1,4]oxazine-4,3'-pyrrolidine]-1'-carboxamide
2-(6-amino-5-cyanopyridin-3-yl)-N-[(1R)-1-(2-fluorophenyl)ethyl]-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-(2-fluorophenyl)ethyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[2-(4-fluorophenyl)propan-2-yl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[2-(1-methyl-1H-1,2,3-triazol-4-yl)propan-2-yl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[(1R)-1-(5-fluoropyridin-3-yl)ethyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-(1-phenylcyclobutyl)-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[1-(4-fluorophenyl)cyclobutyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-(1-phenylcyclobutyl)-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-(1,3,4-trimethyl-1H-pyrazol-5-yl)ethyl]-5',6'-dihydrospiro[azetidine-3,4'-pyrrolo[1,2-b]pyrazole]-1-carboxamide
(*rac*)-2-(6-amino-5-cyanopyridin-3-yl)-N-[2-(pyridin-4-yl)propan-2-yl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[1-(pyridin-3-yl)cyclobutyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-(2-methylpyrimidin-5-yl)ethyl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-(pyridin-2-yl)ethyl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide (mixture of stereoisomer)
2'-[6-amino-5-(trifluoromethoxy)pyridin-3-yl]-N-[(1R)-1-(pyridin-3-yl)ethyl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-(1-phenylcyclobutyl)-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[2-(2-fluorophenyl)propan-2-yl]-5',6'-dihydrospiro[azetidine-3,4'-pyrrolo[1,2-b]pyrazole]-1-carboxamide
(*rac*)-2-[6-amino-5-(difluoromethoxy)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide
2-[6-amino-5-(difluoromethoxy)pyridin-3-yl]-N-[(1S)-1-(3-chloropyridin-4-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[1-(1,3,5-trimethyl-1H-pyrazol-4-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[1-(1-ethyl-3,5-dimethyl-1H-pyrazol-4-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-(2-cyanophenyl)ethyl]-6,7-dihydrospiro[pyrazolo[5,1-c][1,4]oxazine-4,3'-pyrrolidine]-1'-carboxamide
(*rac*)-2'-[6-amino-5-(difluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2-[6-amino-5-(trifluoromethoxy)pyridin-3-yl]-N-[(1R)-1-(4-fluorophenyl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-phenylpropyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-phenylethyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide
2-[6-amino-5-(difluoromethoxy)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[2-(4-fluorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(*rac*)-2'-[6-amino-5-(difluoromethoxy)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[1-(2-chlorophenyl)cyclobutyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(4-cyanophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(1,3,4-trimethyl-1H-pyrazol-5-yl)ethyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(5-fluoropyridin-3-yl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(4-fluorophenyl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(2-cyanophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(pyridin-4-yl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(4-fluorophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(2-chlorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(4-fluorophenyl)propan-2-yl]-6,7-dihydrospiro[pyrazolo[5,1-c][1,4]oxazine-4,3'-pyrrolidine]-1'-carboxamide
(rac)-2'-[6-amino-5-(trifluoromethoxy)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide
2'-[6-amino-5-(trifluoromethoxy)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[2-(4-fluorophenyl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(2-chlorophenyl)propan-2-yl]-5',6'-dihydrospiro[azetidine-3,4'-pyrrolo[1,2-b]pyrazole]-1-carboxamide
2-[6-amino-5-(trifluoromethoxy)pyridin-3-yl]-N-[(1R)-1-(3-fluorophenyl)ethyl]-6,7-dihydrospiro[pyrazolo[5,1-c][1,4]oxazine-4,3'-pyrrolidine]-1'-carboxamide
2'-[6-amino-5-(trifluoromethoxy)pyridin-3-yl]-N-(1-phenylcyclobutyl)-5',6'-dihydrospiro[azetidine-3,4'-pyrrolo[1,2-b]pyrazole]-1-carboxamide
2'-[6-amino-5-(trifluoromethoxy)pyridin-3-yl]-N-benzyl-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(4-cyanopyridin-2-yl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(2-cyanopyridin-4-yl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1R)-1-(5-fluoropyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[2-(3-fluorophenyl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1S)-1-(3-chloropyridin-4-yl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(3-chloropyridin-4-yl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[(1R)-1-(2-chloro-4-cyanophenyl)ethyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(2-fluorophenyl)cyclobutyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide
2-[6-amino-5-(trifluoromethoxy)pyridin-3-yl]-N-[(1R)-1-(4-cyanopyridin-2-yl)ethyl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[1-(2-chlorophenyl)cyclobutyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(pyridin-2-yl)cyclobutyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-(2-cyanopyridin-4-yl)ethyl]-6,7-dihydrospiro[pyrazolo[5,1-c][1,4]oxazine-4,3'-pyrrolidine]-1'-carboxamide
(*rac*)-2-[6-amino-5-(difluoromethoxy)pyridin-3-yl]-N-[2-(2-chlorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(2-chlorophenyl)cyclobutyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluoromethoxy)pyridin-3-yl]-N-[1-(pyridin-4-yl)cyclobutyl]-5',6'-dihydro-1H-spiro[pyrrolidine-3,4'-pyrrolo[1,2-b]pyrazole]-1-carboxamide acetate (1:1)
(*rac*)-2-[6-amino-5-(trifluoromethoxy)pyridin-3-yl]-N-[(1-methyl-2-oxabicyclo[2.1.1]hexan-4-yl)methyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[2-(pyridin-2-yl)propan-2-yl]-5',6'-dihydrospiro[azetidine-3,4'-pyrrolo[1,2-b]pyrazole]-1-carboxamide
2'-[6-amino-5-(difluoromethoxy)pyridin-3-yl]-N-[2-(1-methyl-1H-1,2,3-triazol-4-yl)propan-2-yl]-5',6'-dihydrospiro[azetidine-3,4'-pyrrolo[1,2-b]pyrazole]-1-carboxamide
2-[6-amino-5-(trifluoromethoxy)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1S)-1-(3-chloropyridin-4-yl)ethyl]-6,7-dihydrospiro[pyrazolo[5,1-c][1,4]oxazine-4,3'-pyrrolidine]-1'-carboxamide
(*rac*)-2-[6-amino-5-(trifluoromethoxy)pyridin-3-yl]-N-[2-(3-fluorophenyl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluoromethoxy)pyridin-3-yl]-N-[(1-methyl-2-oxabicyclo[2.1.1]hexan-4-yl)methyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-phenylpropyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1S)-2-methoxy-1-phenylethyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(3-fluorophenyl)ethyl]-5',6'-dihydrospiro[azetidine-3,4'-pyrrolo[1,2-b]pyrazole]-1-carboxamide
2'-[6-amino-5-(trifluoromethoxy)pyridin-3-yl]-N-[(1S)-1-(pyridin-3-yl)ethyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[1-(propan-2-yl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(pyridin-4-yl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-(bicyclo[2.2.1]heptan-1-yl)-5',6'-dihydrospiro[azetidine-3,4'-pyrrolo[1,2-b]pyrazole]-1-carboxamide
2'-[6-amino-5-(difluoromethoxy)pyridin-3-yl]-N-(1-benzylcyclobutyl)-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(4-chlorophenyl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-(1-methylcyclobutyl)-5',6'-dihydrospiro[azetidine-3,4'-pyrrolo[1,2-b]pyrazole]-1-carboxamide
(rac)-2-[6-amino-5-(trifluoromethoxy)pyridin-3-yl]-N-[1-(1,3,4-trimethyl-1H-pyrazol-5-yl)ethyl]-6,7-dihydrospiro[pyrazolo[5,1-c][1,4]oxazine-4,3'-pyrrolidine]-1'-carboxamide
2-[6-amino-5-(trifluoromethoxy)pyridin-3-yl]-N-[(1R)-2,3-dihydro-1H-inden-1-yl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[1-(pyridin-2-yl)cyclobutyl]-5',6'-dihydrospiro[azetidine-3,4'-pyrrolo[1,2-b]pyrazole]-1-carboxamide
2'-[6-amino-5-(difluoromethoxy)pyridin-3-yl]-N-[1-(3-fluorophenyl)cyclobutyl]-5',6'-dihydrospiro[azetidine-3,4'-pyrrolo[1,2-b]pyrazole]-1-carboxamide
(*rac*)-2'-[6-amino-5-(trifluoromethoxy)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
2-[6-amino-5-(difluoromethoxy)pyridin-3-yl]-N-[(1S)-2-methoxy-1-phenylethyl]-6,7-dihydrospiro[pyrazolo[5,1-c][1,4]oxazine-4,3'-pyrrolidine]-1'-carboxamide
2'-[6-amino-5-(difluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(4-fluorophenyl)ethyl]-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
2'-[6-amino-5-(difluoromethoxy)pyridin-3-yl]-N-[(1R)-1-(5-fluoropyridin-3-yl)ethyl]-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-[(1R)-1-phenylethyl]-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-[(1R)-1-phenylethyl]-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-{6-amino-5-[(trifluoromethyl)sulfanyl]pyridin-3-yl}-N-[(1R)-1-(3-fluorophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(3R)-2,3-dihydro-1-benzofuran-3-yl]-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-methyl-1H-imidazol-5-yl)ethyl]-5',6'-dihydrospiro[pyrrolidine-3,4'-pyrrolo[1,2-b]pyrazole]-1-carboxamide (diastereomeric mixture)
2'-{6-amino-5-[(1R)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-[2-(1-methyl-1H-1,2,3-triazol-4-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[cyclopropyl(pyridin-2-yl)methyl]-5',6'-dihydrospiro[pyrrolidine-3,4'-pyrrolo[1,2-b]pyrazole]-1-carboxamide (diastereomeric mixture)
2'-{6-amino-5-[(1S)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide
2'-{6-amino-5-[(1S)-1-(pyridin-3-yl)ethoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(2-cyanophenyl)propan-2-yl]-5',6'-dihydrospiro[pyrrolidine-3,4'-pyrrolo[1,2-b]pyrazole]-1-carboxamide
[2'-{6-amino-5-[(1R)-1-(pyridin-3-yl)ethoxy]pyridin-3-yl}-5',6'-dihydrospiro[pyrrolidine-3,4'-pyrrolo[1,2-b]pyrazol]-1-yl](1H-imidazol-1-yl)methanone
(*rac*)-2'-[6-amino-5-(trifluoromethyl)pyridin-3-yl]-N-(1-methylcyclobutyl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(3-methyloxetan-3-yl})-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(3-fluoropyridin-2-yl)propan-2-yl]-6,7-dihydrospiro[pyrazolo[5,1-c][1,4]oxazine-4,3'-pyrrolidine]-1'-carboxamide
2'-{6-amino-5-[(1S)-1-phenylethoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide
2'-{6-amino-5-[(1S)-1-phenylethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-phenylethoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide
2'-{6-amino-5-[(1R)-1-phenylethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2-[6-amino-5-(trifluoromethoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
2'-{6-amino-5-[(1R)-1-(pyridin-2-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-(pyridin-4-yl)ethoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-(6-amino-5-cyanopyridin-3-yl)-N-[(1R)-1-(pyridin-4-yl)ethyl]-5',6'-dihydrospiro[pyrrolidine-3,4'-pyrrolo[1,2-b]pyrazole]-1-carboxamide (isomer 1)
2'-(6-amino-5-cyanopyridin-3-yl)-N-[(1R)-1-(pyridin-4-yl)ethyl]-5',6'-dihydrospiro[pyrrolidine-3,4'-pyrrolo[1,2-b]pyrazole]-1-carboxamide (isomer 2)
2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (enantiomer 1)
(3R)-2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-[(1S)-1-(pyridin-4-yl)ethyl]-5',6'-dihydrospiro[pyrrolidine-3,4'-pyrrolo[1,2-b]pyrazole]-1-carboxamide
2'-(6-amino-5-cyanopyridin-3-yl)-N-[(1S)-1-(pyridin-4-yl)ethyl]-5',6'-dihydrospiro[pyrrolidine-3,4'-pyrrolo[1,2-b]pyrazole]-1-carboxamide (isomer 1)
2'-(6-amino-5-cyanopyridin-3-yl)-N-[(1S)-1-(pyridin-4-yl)ethyl]-5',6'-dihydrospiro[pyrrolidine-3,4'-pyrrolo[1,2-b]pyrazole]-1-carboxamide (isomer 2)
2'-[6-amino-5-(trifluoromethyl)pyridin-3-yl]-N-[(1S)-1-(pyridin-4-yl)ethyl]-5',6'-dihydrospiro[azetidine-3,4'-pyrrolo[1,2-b]pyrazole]-1-carboxamide
2'-[6-amino-5-(trifluoromethyl)pyridin-3-yl]-N-[(1R)-1-(pyridin-4-yl)ethyl]-5',6'-dihydrospiro[azetidine-3,4'-pyrrolo[1,2-b]pyrazole]-1-carboxamide
(*rac*)-2'-[6-amino-5-(trifluoromethyl)pyridin-3-yl]-N-[1-(2-chlorophenyl)cyclobutyl]-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[2-(2-fluorophenyl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-(1-phenylcyclobutyl)-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-[2-(4-fluorophenyl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(2-chlorophenyl)propan-2-yl]-5',6'-dihydrospiro[pyrrolidine-3,4'-pyrrolo[1,2-b]pyrazole]-1-carboxamide
2-[6-amino-5-(trifluoromethyl)pyridin-3-yl]-N-[(1R)-1-(2-chloro-4-cyanophenyl)ethyl]-6,7-dihydrospiro[pyrazolo[5,1-c][1,4]oxazine-4,3'-pyrrolidine]-1'-carboxamide (mixture of stereoisomer)
2'-[6-amino-5-(trifluoromethyl)pyridin-3-yl]-N-[(1R)-1-(2-chloro-4-cyanophenyl)ethyl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(3-fluorophenyl)propan-2-yl]-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-[1-(2-fluorophenyl)cyclobutyl]-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-[1-(2-fluorophenyl)cyclobutyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(3-fluorophenyl)cyclobutyl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-[1-(3-fluorophenyl)cyclobutyl]-6,7-dihydrospiro[pyrazolo[5,1-c][1,4]oxazine-4,3'-pyrrolidine]-1'-carboxamide
(3'R)-2-[6-amino-5-(trifluoromethoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(3'R)-2-[6-amino-5-(trifluoromethyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide
(3R)-2'-[6-amino-5-(trifluoromethyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(3'R)-2-[6-amino-5-(trifluoromethyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(3R)-2'-[6-amino-5-(trifluoromethyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
(3R)-2'-[6-amino-5-(difluoromethoxy)pyridin-3-yl]-N-[(1R)-1-phenylethyl]-5',6'-dihydrospiro[pyrrolidine-3,4'-pyrrolo[1,2-b]pyrazole]-1-carboxamide
(3'R)-2-[6-amino-5-(difluoromethoxy)pyridin-3-yl]-N-[(1R)-1-phenylethyl]-6,7-dihydrospiro[pyrazolo[5,1-c][1,4]oxazine-4,3'-pyrrolidine]-1'-carboxamide
(3R)-2'-[6-amino-5-(trifluoromethyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide (stereoisomer 1)
2'-[6-amino-5-(trifluoromethyl)pyridin-3-yl]-N-[(1R)-1-phenylethyl]-5',6'-dihydrospiro[pyrrolidine-3,4'-pyrrolo[1,2-b]pyrazole]-1-carboxamide (stereoisomer 2)
(*rac*)-2'-[6-amino-5-(trifluoromethyl)pyridin-3-yl]-N-[2-(3,5-difluorophenyl)propan-2-yl]-5',6'-dihydrospiro[pyrrolidine-3,4'-pyrrolo[1,2-b]pyrazole]-1-carboxamide
(*rac*)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-(5-fluoropyridin-2-yl)-2-methylpropan-1-one
(*rac*)-2'-[6-amino-5-(trifluoromethyl)pyridin-3-yl]-N-[2-(6-methylpyridin-3-yl)propan-2-yl]-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(1-phenylcyclohexyl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-[2-(5-fluoropyridin-2-yl)propan-2-yl]-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[(1R)-2,2,2-trifluoro-1-methoxy-1-phenylethyl]-5',6'-dihydrospiro[pyrrolidine-3,4'-pyrrolo[1,2-b]pyrazole]-1-carboxamide
*(rac)-2'-[6-amino-5-(trifluoromethyl)pyridin-3-yl]-N-[2-(pyrimidin-4-yl)propan-2-yl]-5',6'-*dihydrospiro[pyrrolidine-3,4'-pyrrolo[1,2-b]pyrazole]-1-carboxamide
(*rac)*-2'-[6-amino-5-(trifluoromethyl)pyridin-3-yl]-N-[2-(pyrimidin-5-yl)propan-2-yl]-5',6'-dihydrospiro[pyrrolidine-3,4'-pyrrolo[1,2-b]pyrazole]-1-carboxamide
(*rac*)-{2'-[6-amino-5-(trifluoromethyl)pyridin-3-yl]-5',6'-dihydrospiro[pyrrolidine-3,4'-pyrrolo[1,2-b]pyrazol]-1-yl}(3-phenyloxetan-3-yl)methanone
(*rac*)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-(3,5-difluorophenyl)-2-methylpropan-1-one
(*rac*)-1-{2'-[6-amino-5-(trifluoromethyl)pyridin-3-yl]-5',6'-dihydrospiro[pyrrolidine-3,4'-pyrrolo[1,2-b]pyrazol]-1-yl}-2-methyl-2-(6-methylpyridin-3-yl)propan-1-one
(*rac*)-2'-[6-amino-5-(trifluoromethyl)pyridin-3-yl]-N-(3-phenyloxetan-3-yl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide
(*rac*)-2'-[6-amino-5-(trifluoromethyl)pyridin-3-yl]-N-cyclobutyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-(propan-2-yl)-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-cyclobutyl-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-ethyl-6,7-dihydrospiro[pyrazolo[5,1-c][1,4]oxazine-4,3'-pyrrolidine]-1'-carboxamide
(*rac*)-2-[6-amino-5-(trifluoromethyl)pyridin-3-yl]-N-(propan-2-yl)-6,7-dihydrospiro[pyrazolo[5,1-c][1,4]oxazine-4,3'-pyrrolidine]-1'-carboxamide
2'-[6-amino-5-(trifluoromethyl)pyridin-3-yl]-N-cyclobutyl-5',6'-dihydrospiro[azetidine-3,4'-pyrrolo[1,2-b]pyrazole]-1-carboxamide
2'-[6-amino-5-(trifluoromethyl)pyridin-3-yl]-N-ethyl-5',6'-dihydrospiro[azetidine-3,4'-pyrrolo[1,2-b]pyrazole]-1-carboxamide
2'-[6-amino-5-(trifluoromethyl)pyridin-3-yl]-N-(propan-2-yl)-5',6'-dihydrospiro[azetidine-3,4'-pyrrolo[1,2-b]pyrazole]-1-carboxamide
(*rac*)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-{6-amino-5-[(2-chlorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(2-chlorophenyl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(2-chlorophenyl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-fluorophenyl)methoxy]pyridin-3-yl}-N-benzyl-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(3-fluorophenyl)methoxy]pyridin-3-yl}-N-benzyl-5',6'-dihydrospiro[pyrrolidine-3,4'-pyrrolo[1,2-b]pyrazole]-1-carboxamide
(*rac*)-2'-{6-amino-5-[(pyridin-2-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(pyridin-2-yl)methoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(pyridin-2-yl)methoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-chloro-5-fluorophenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(2-chloro-5-fluorophenyl)methoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1R)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide
2'-(6-amino-5-{[1-(5-chloro-3-fluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomeric mixture)
(*rac*)-2'-[6-amino-5-(benzyloxy)pyridin-3-yl]-N-cyclobutyl-5',6'-dihydrospiro[pyrrolidine-3,4'-pyrrolo[1,2-b]pyrazole]-1-carboxamide
2'-{6-amino-5-[(pyridazin-3-yl)methoxy]pyridin-3-yl}-N-ethyl-5',6'-dihydrospiro[pyrrolidine-3,4'-pyrrolo[1,2-b]pyrazole]-1-carboxamide (stereoisomer 2)
2'-{6-amino-5-[(pyridazin-3-yl)methoxy]pyridin-3-yl}-N-ethyl-5',6'-dihydrospiro[pyrrolidine-3,4'-pyrrolo[1,2-b]pyrazole]-1-carboxamide (stereoisomer 1)
(3S)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 1)
(3S)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 2)
(*rac*)-2'-(2-amino-1'-methyl-1',2',3',6'-tetrahydro[3,4'-bipyridin]-5-yl)-N-ethyl-5',6'-dihydrospiro[pyrrolidine-3,4'-pyrrolo[1,2-b]pyrazole]-1-carboxamide
2'-{6-amino-5-[(1S)-1-(pyridin-2-yl)ethoxy]pyridin-3-yl}-N-ethyl-5',6'-dihydrospiro[azetidine-3,4'-pyrrolo[1,2-b]pyrazole]-1-carboxamide
2'-{6-amino-5-[(1S)-1-(pyridin-2-yl)ethoxy]pyridin-3-yl}-N-(propan-2-yl)-5',6'-dihydrospiro[azetidine-3,4'-pyrrolo[1,2-b]pyrazole]-1-carboxamide
2'-{6-amino-5-[(1S)-1-phenylethoxy]pyridin-3-yl}-N-ethyl-5',6'-dihydrospiro[azetidine-3,4'-pyrrolo[1,2-b]pyrazole]-1-carboxamide
2'-{6-amino-5-[(1R)-1-phenylethoxy]pyridin-3-yl}-N-ethyl-5',6'-dihydrospiro[azetidine-3,4'-pyrrolo[1,2-b]pyrazole]-1-carboxamide
(*rac*)-2-[6-amino-5-(trifluoromethyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluoromethyl)pyridin-3-yl]-N-ethyl-5',6'-dihydrospiro[pyrrolidine-3,4'-pyrrolo[1,2-b]pyrazole]-1-carboxamide
(*rac*)-2-[6-amino-5-(trifluoromethyl)pyridin-3-yl]-N-ethyl-6,7-dihydro-5H-spiro[pyrazolo[1,5-a]pyridine-4,3'-pyrrolidine]-1'-carboxamide
(*rac*)-2'-(6-amino-5-cyanopyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(trifluoromethoxy)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(difluoromethoxy)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(trifluoroacetyl)pyridin-3-yl]-N-ethyl-5',6'-dihydrospiro[pyrrolidine-3,4'-pyrrolo[1,2-b]pyrazole]-1-carboxamide
2'-(6-amino-5-{[1-phenylethyl]sulfanyl}pyridin-3-yl)-N-ethyl-5',6'-dihydrospiro[pyrrolidine-3,4'-pyrrolo[1,2-b]pyrazole]-1-carboxamide (diastereomeric mixture)
(*rac*)-2'-{6-amino-5-[(cyclohexylmethyl)sulfanyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(benzylsulfanyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(2-hydroxypropan-2-yl)pyridin-3-yl]-N-ethyl-5',6'-dihydrospiro[pyrrolidine-3,4'-pyrrolo[1,2-b]pyrazole]-1-carboxamide
2'-(6-amino-5-{[1-cyclohexylethyl]sulfanyl}pyridin-3-yl)-N-ethyl-5',6'-dihydrospiro[pyrrolidine-3,4'-pyrrolo[1,2-b]pyrazole]-1-carboxamide (diastereomeric mixture)
2'-{6-amino-5-[(1R)-1-(pyridin-3-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1S)-1-(pyridin-3-yl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(cyclopentylmethyl)sulfanyl]pyridin-3-yl}-N-ethyl-5',6'-dihydrospiro[pyrrolidine-3,4'-pyrrolo[1,2-b]pyrazole]-1-carboxamide
(*rac*)-2'-(6-amino-5-nitropyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(benzyloxy)pyridin-3-yl]-N-ethyl-5',6'-dihydrospiro[pyrrolidine-3,4'-pyrrolo[1,2-b]pyrazole]-1-carboxamide
(*rac*)-2'-(6-amino-5-methylpyridin-3-yl)-N-ethyl-5',6'-dihydrospiro[pyrrolidine-3,4'-pyrrolo[1,2-b]pyrazole]-1-carboxamide
(*rac*)-2-(6-amino-5-nitropyridin-3-yl)-N-ethyl-6,7-dihydro-5H-spiro[pyrazolo[1,5-a]pyridine-4,3'-pyrrolidine]-1'-carboxamide
methyl 2-amino-5-[(*rac*)-1-(ethylcarbamoyl)-5',6'-dihydrospiro[pyrrolidine-3,4'-pyrrolo[1,2-b]pyrazol]-2'-yl]pyridine-3-carboxylate
(*rac*)-2'-(6-amino-5-phenylpyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(methylsulfanyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(4-methylphenyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(2-methylphenyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(3-methylphenyl)pyridin-3-yl]-N-ethyl-5',6'-dihydrospiro[pyrrolidine-3,4'-pyrrolo[1,2-b]pyrazole]-1-carboxamide
(*rac*)-2'-(6-amino-5-methoxypyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(3-fluorophenyl)pyridin-3-yl]-N-ethyl-5',6'-dihydrospiro[pyrrolidine-3,4'-pyrrolo[1,2-b]pyrazole]-1-carboxamide
(*rac*)-2'-(2-amino[3,3'-bipyridin]-5-yl)-N-ethyl-5',6'-dihydrospiro[pyrrolidine-3,4'-pyrrolo[1,2-b]pyrazole]-1-carboxamide
(*rac*)-2'-[6-amino-5-(2-fluorophenyl)pyridin-3-yl]-N-ethyl-5',6'-dihydrospiro[pyrrolidine-3,4'-pyrrolo[1,2-b]pyrazole]-1-carboxamide
2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (diastereomeric mixture)
(*rac*)-2-[6-amino-5-(2-fluorophenyl)pyridin-3-yl]-N-ethyl-6,7-dihydro-5H-spiro[pyrazolo[1,5-a]pyridine-4,3'-pyrrolidine]-1'-carboxamide
(*rac*)-2'-[6-amino-5-(2-methoxyphenyl)pyridin-3-yl]-N-ethyl-5',6'-dihydrospiro[pyrrolidine-3,4'-pyrrolo[1,2-b]pyrazole]-1-carboxamide
(*rac*)-2'-(2-amino[3,4'-bipyridin]-5-yl)-N-ethyl-5',6'-dihydrospiro[pyrrolidine-3,4'-pyrrolo[1,2-b]pyrazole]-1-carboxamide
(*rac*)-2'-{6-amino-5-[3-(methanesulfonyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(phenylsulfanyl)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[4-(methanesulfonyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[5-(4-acetylphenyl)-6-aminopyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[4-(methanesulfinyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(3-methoxyphenyl)pyridin-3-yl]-N-ethyl-5',6'-dihydrospiro[pyrrolidine-3,4'-pyrrolo[1,2-b]pyrazole]-1-carboxamide
2'-{6-amino-5-[(1R)-1-(2,6-dichloro-3-fluorophenyl)ethoxy]pyridin-3-yl}-N-ethyl-5',6'-dihydrospiro[azetidine-3,4'-pyrrolo[1,2-b]pyrazole]-1-carboxamide
(*rac*)-2'-{6-amino-5-[4-(cyclopropanesulfonyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(4-sulfamoylphenyl)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[4-(methylsulfamoyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[4-(dimethylsulfamoyl)phenyl]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(cyclohexylmethoxy)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[(pyridin-3-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(4-fluorophenyl)methoxy]pyridin-3-yl}-N-ethyl-5',6'-dihydrospiro[pyrrolidine-3,4'-pyrrolo[1,2-b]pyrazole]-1-carboxamide
2'-(6-amino-5-{[1-phenylethyl]oxy}pyridin-3-yl)-N-ethyl-5',6'-dihydrospiro[pyrrolidine-3,4'-pyrrolo[1,2-b]pyrazole]-1-carboxamide (diastereomeric mixture)
2-{6-amino-5-[(1R)-1-(2,6-dichloro-3-fluorophenyl)ethoxy]pyridin-3-yl}-N-ethyl-6,7-dihydrospiro[pyrazolo[5,1-c][1,4]oxazine-4,3'-pyrrolidine]-1'-carboxamide
(*rac*)-2'-[6-amino-5-(propan-2-yl)pyridin-3-yl]-N-ethyl-5',6'-dihydrospiro[pyrrolidine-3,4'-pyrrolo[1,2-b]pyrazole]-1-carboxamide
(*rac*)-2-[6-amino-5-(methylcarbamoyl)pyridin-3-yl]-N-ethyl-6,7-dihydrospiro[pyrazolo[5,1-c][1,4]oxazine-4,3'-pyrrolidine]-1'-carboxamide
(*rac*)-2'-[6-amino-5-(methylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-(6-amino-5-cyclopropylpyridin-3-yl)-N-ethyl-5',6'-dihydrospiro[pyrrolidine-3,4'-pyrrolo[1,2-b]pyrazole]-1-carboxamide
(*rac*)-2'-[6-amino-5-(methylcarbamoyl)pyridin-3-yl]-N-(1-phenylcyclobutyl)-5',6'-dihydrospiro[pyrrolidine-3,4'-pyrrolo[1,2-b]pyrazole]-1-carboxamide
(*rac*)-2'-[6-amino-5-(dimethylcarbamoyl)pyridin-3-yl]-N-(1-phenylcyclobutyl)-5',6'-dihydrospiro[pyrrolidine-3,4'-pyrrolo[1,2-b]pyrazole]-1-carboxamide
2'-{6-amino-5-[(1R)-1-(pyridin-4-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1S)-1-(pyridin-4-yl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-[6-amino-5-(1-methyl-1H-pyrazol-3-yl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(1,2-thiazol-5-yl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(1,5-dimethyl-1H-pyrazol-4-yl)pyridin-3-yl]-N-ethyl-5',6'-dihydrospiro[pyrrolidine-3,4'-pyrrolo[1,2-b]pyrazole]-1-carboxamide
(*rac*)-2'-{6-amino-5-[(trifluoromethyl)sulfanyl]pyridin-3-yl}-N-ethyl-5',6'-dihydrospiro[pyrrolidine-3,4'-pyrrolo[1,2-b]pyrazole]-1-carboxamide
5-[1-methyl-5',6'-dihydrospiro[pyrrolidine-3,4'-pyrrolo[1,2-b]pyrazol]-2'-yl]-3-(trifluoromethyl)pyridin-2-amine (stereoisomer 1)
5-[1-methyl-5',6'-dihydrospiro[pyrrolidine-3,4'-pyrrolo[1,2-b]pyrazol]-2'-yl]-3-(trifluoromethyl)pyridin-2-amine (stereoisomer 2)
(3R)-2'-{6-amino-5-[(1R)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(2-chlorophenyl)ethoxy]pyridin-3-yl}-N-ethyl-5',6'-dihydrospiro[azetidine-3,4'-pyrrolo[1,2-b]pyrazole]-1-carboxamide
2'-{6-amino-5-[(1S)-1-(2-chlorophenyl)ethoxy]pyridin-3-yl}-N-ethyl-5',6'-dihydrospiro[azetidine-3,4'-pyrrolo[1,2-b]pyrazole]-1-carboxamide
(3'R)-2-{6-amino-5-[(1R)-1-(2,6-dichloro-3-fluorophenyl)ethoxy]pyridin-3-yl}-N-ethyl-6,7-dihydrospiro[pyrazolo[5,1-c][1,4]oxazine-4,3'-pyrrolidine]-1'-carboxamide
(3R)-2'-{6-amino-5-[(1R)-1-(pyridin-2-yl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(pyridin-3-yl)ethoxy]pyridin-3-yl}-N-ethyl-5',6'-dihydrospiro[azetidine-3,4'-pyrrolo[1,2-b]pyrazole]-1-carboxamide
(3R)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 1)
((3R)-2'-{6-amino-5-[1-(pyrimidin-5-yl)ethoxy]pyridin-3-yl}-N-ethyl-5',6'-dihydro-1H-spiro[pyrrolidine-3,4'-pyrrolo[1,2-b]pyrazole]-1-carboxamide (isomer 2)
(*rac*)-2'-[6-amino-5-(difluoromethyl)pyridin-3-yl]-N-ethyl-5',6'-dihydrospiro[pyrrolidine-3,4'-pyrrolo[1,2-b]pyrazole]-1-carboxamide
2'-{6-amino-5-[(1S)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(2-chlorophenyl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(3-methylphenyl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(1-methyl-1H-pyrazol-5-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(1-methyl-1H-pyrazol-4-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(1,5-dimethyl-1H-pyrazol-3-yl)methoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(4-methylphenyl)methoxy]pyridin-3-yl}-N-ethyl-5',6'-dihydrospiro[pyrrolidine-3,4'-pyrrolo[1,2-b]pyrazole]-1-carboxamide
2'-{6-amino-5-[(1R)-1-(3-methylphenyl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-{6-amino-5-[(propan-2-yl)oxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1S)-1-(1-methyl-1H-pyrazol-3-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(1-methyl-1H-pyrazol-3-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-(6-amino-5-{[1-(3,5-difluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (stereoisomer 1)
(3R)-2'-(6-amino-5-{[1-(3,5-difluoropyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (stereoisomer 2)
(3R)-2'-{6-amino-5-[(1R)-1-(2-fluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1S)-1-(2-fluorophenyl)ethoxy]pyridin-3-yl}-N-ethyl-5',6'-dihydrospiro[pyrrolidine-3,4'-pyrrolo[1,2-b]pyrazole]-1-carboxamide
(*rac*)-2'-(5-acetyl-6-aminopyridin-3-yl)-N-(1-phenylcyclobutyl)-5',6'-dihydrospiro[pyrrolidine-3,4'-pyrrolo[1,2-b]pyrazole]-1-carboxamide
(3R)-2'-[6-amino-5-(trifluoromethoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
(3'R)-2-[6-amino-5-(trifluoromethyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(3R)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide
(3'R)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2'-[6-amino-5-(phenylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(cyclohexylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-[6-amino-5-(diethylcarbamoyl)pyridin-3-yl]-N-ethyl-5',6'-dihydrospiro[pyrrolidine-3,4'-pyrrolo[1,2-b]pyrazole]-1-carboxamide
(*rac*)-2'-(5-acetyl-6-aminopyridin-3-yl)-N-ethyl-5',6'-dihydrospiro[pyrrolidine-3,4'-pyrrolo[1,2-b]pyrazole]-1-carboxamide
5-{1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
5-{1-[1-(1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-imidazol-5-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1-methyl-1H-pyrazol-3-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-pyrazol-4-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(4-methyl-1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(3-methyl-1H-pyrazol-5-yl)ethyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(4-methyl-1H-pyrazol-5-yl)ethyl]-5',6'-dihydrospiro[pyrrolidine-3,4'-pyrrolo[1,2-b]pyrazol]-2'-yl}-3-(trifluoromethyl)pyridin-2-amine
5-[(3S)-1-(pyridin-3-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(pyridin-3-ylmethyl)-5',6'-dihydrospiro[pyrrolidine-3,4'-pyrrolo[1,2-b]pyrazol]-2'-yl]-3-(trifluoromethyl)pyridin-2-amine
5-{(*rac*)-1-[(1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
5-[(3S)-1-(1H-imidazol-2-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(1H-imidazol-2-ylmethyl)-5',6'-dihydrospiro[pyrrolidine-3,4'-pyrrolo[1,2-b]pyrazol]-2'-yl]-3-(trifluoromethyl)pyridin-2-amine
5-{(rac)-1-[(4-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1H-pyrazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1-methyl-1H-pyrazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1H-imidazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1-methyl-1H-imidazol-4-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(4-chloro-1H-pyrazol-5-yl)methyl]-5',6'-dihydrospiro[pyrrolidine-3,4'-pyrrolo[1,2-b]pyrazol]-2'-yl}-3-(trifluoromethyl)pyridin-2-amine
3-[(2-fluorophenyl)methoxy]-5-{(*rac*)-1-[(1H-imidazol-2-yl)methyl]-5',6'-dihydrospiro[pyrrolidine-3,4'-pyrrolo[1,2-b]pyrazol]-2'-yl}pyridin-2-amine
3-[(3-fluorophenyl)methoxy]-5-{(*rac*)-1-[(1H-imidazol-2-yl)methyl]-5',6'-dihydrospiro[pyrrolidine-3,4'-pyrrolo[1,2-b]pyrazol]-2'-yl}pyridin-2-amine
5-{(*rac*)-1-[(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
5-{1-[(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine
5-{(*rac*)-1'-[(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine
2-amino-5-{1-[(1H-imidazol-2-yl)methyl]-5',6'-dihydrospiro[azetidine-3,4'-pyrrolo[1,2-b]pyrazol]-2'-yl}pyridine-3-carbonitrile
5-{(*rac*)-1-[(1-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(1-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
2-amino-5-{(*rac*)-1-[(1H-imidazol-2-yl)methyl]-5',6'-dihydrospiro[pyrrolidine-3,4'-pyrrolo[1,2-b]pyrazol]-2'-yl}pyridine-3-carbonitrile
5-{1-[(1-methyl-1H-imidazol-2-yl)methyl]-5',6'-dihydrospiro[azetidine-3,4'-pyrrolo[1,2-b]pyrazol]-2'-yl}-3-(trifluoromethyl)pyridin-2-amine
5-{(*rac*)-1-[(5-methyl-1H-pyrazol-3-yl)methyl]-5',6'-dihydrospiro[pyrrolidine-3,4'-pyrrolo[1,2-b]pyrazol]-2'-yl}-3-(trifluoromethyl)pyridin-2-amine
5-{(*rac*)-1-[(2,4-dimethyl-1H-imidazol-5-yl)methyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (stereoisomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (stereoisomer 2)
5-{(3R)-1-[(1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (stereoisomer 1)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (stereoisomer 2)
5-{(3R)-1-[1-(1-methyl-1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine
3-(trifluoromethyl)-5-[(3R)-1-{[4-(trifluoromethyl)-1H-imidazol-2-yl]methyl}-5',6'-dihydrospiro[pyrrolidine-3,4'-pyrrolo[1,2-b]pyrazol]-2'-yl]pyridin-2-amine
2-amino-5-[(*rac*)-1-(ethylcarbamoyl)-5',6'-dihydrospiro[pyrrolidine-3,4'-pyrrolo[1,2-b]pyrazol]-2'-yl]pyridine-3-carboxylic acid
5-[(3S)-1-(pyridin-2-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(pyridin-2-ylmethyl)-5',6'-dihydrospiro[pyrrolidine-3,4'-pyrrolo[1,2-b]pyrazol]-2'-yl]-3-(trifluoromethyl)pyridin-2-amine
5-[(3S)-1-(pyridin-4-ylmethyl)-5',6'-dihydrospiro[pyrrolidine-3,4'-pyrrolo[1,2-b]pyrazol]-2'-yl]-3-(trifluoromethyl)pyridin-2-amine
5-[(3R)-1-(pyridin-4-ylmethyl)-5',6'-dihydrospiro[pyrrolidine-3,4'-pyrrolo[1,2-b]pyrazol]-2'-yl]-3-(trifluoromethyl)pyridin-2-amine
5-{(3R)-1-[3-(dimethylamino)propyl]-5',6'-dihydrospiro[pyrrolidine-3,4'-pyrrolo[1,2-b]pyrazol]-2'-yl}-3-(trifluoromethyl)pyridin-2-amine
5-{(3R)-1-[2-(dimethylamino)ethyl]-5',6'-dihydrospiro[pyrrolidine-3,4'-pyrrolo[1,2-b]pyrazol]-2'-yl}-3-(trifluoromethyl)pyridin-2-amine
2-amino-1-{2'-[6-amino-5-(trifluoromethyl)pyridin-3-yl]-5',6'-dihydrospiro[pyrrolidine-3,4'-pyrrolo[1,2-b]pyrazol]-1-yl}ethan-1-one (stereoisomer 2)
2-amino-1-{2'-[6-amino-5-(trifluoromethyl)pyridin-3-yl]-5',6'-dihydrospiro[pyrrolidine-3,4'-pyrrolo[1,2-b]pyrazol]-1-yl}ethan-1-one (stereoisomer 1)
2-amino-1-{2-[6-amino-5-(trifluoromethyl)pyridin-3-yl]-6,7-dihydrospiro[pyrazolo[5,1-c][1,4]oxazine-4,3'-pyrrolidin]-1'-yl}ethan-1-one (stereoisomer 2)
2-amino-1-{2-[6-amino-5-(trifluoromethyl)pyridin-3-yl]-6,7-dihydrospiro[pyrazolo[5,1-c][1,4]oxazine-4,3'-pyrrolidin]-1'-yl}ethan-1-one (stereoisomer 1)
2-amino-1-{2'-[6-amino-5-(trifluoromethyl)pyridin-3-yl]-5',6'-dihydrospiro[azetidine-3,4'-pyrrolo[1,2-b]pyrazol]-1-yl}ethan-1-one
(2S)-2-amino-1-{2'-[6-amino-5-(trifluoromethyl)pyridin-3-yl]-5',6'-dihydrospiro[azetidine-3,4'-pyrrolo[1,2-b]pyrazol]-1-yl}propan-1-one
(2R)-2-amino-1-{2'-[6-amino-5-(trifluoromethyl)pyridin-3-yl]-5',6'-dihydrospiro[azetidine-3,4'-pyrrolo[1,2-b]pyrazol]-1-yl}propan-1-one
(*rac*)-2'-[6-amino-5-(1-methylpiperidin-4-yl)pyridin-3-yl]-N-ethyl-5',6'-dihydrospiro[pyrrolidine-3,4'-pyrrolo[1,2-b]pyrazole]-1-carboxamide
5-{(rac)-1-[(5-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine
2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (isomer 1)
2'-{6-amino-5-[(1R)-1-(2,6-dichloro-3-fluorophenyl)ethoxy]pyridin-3-yl}-N-ethyl-5',6'-dihydrospiro[pyrrolidine-3,4'-pyrrolo[1,2-b]pyrazole]-1-carboxamide (isomer 2)
5-{(3'R)-1'-[(1S)-1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine
5-{(3'R)-1'-[(1R)-1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine
5-{(3'R)-1'-[(S)-cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine
5-{(3'R)-1'-[(R)-cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine
5-{(3R)-1-[(S)-cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(R)-cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(1S)-1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(1R)-1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine
5-{(3R)-1-[(R)-cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine
5-{(3R)-1-[(S)-cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine
3-(difluoromethoxy)-5-{(3R)-1-[(1R)-1-(1H-imidazol-2-yl)propyl]-5',6'-dihydrospiro[pyrrolidine-3,4'-pyrrolo[1,2-b]pyrazol]-2'-yl}pyridin-2-amine
3-(difluoromethoxy)-5-{(3R)-1-[(1S)-1-(1H-imidazol-2-yl)propyl]-5',6'-dihydrospiro[pyrrolidine-3,4'-pyrrolo[1,2-b]pyrazol]-2'-yl}pyridin-2-amine
2-amino-5-{(3R)-1-[(R)-cyclopropyl(1H-imidazol-2-yl)methyl]-5',6'-dihydrospiro[pyrrolidine-3,4'-pyrrolo[1,2-b]pyrazol]-2'-yl}nicotinonitrile
2-amino-5-{(3R)-1-[(S)-cyclopropyl(1H-imidazol-2-yl)methyl]-5',6'-dihydrospiro[pyrrolidine-3,4'-pyrrolo[1,2-b]pyrazol]-2'-yl}nicotinonitrile
2-amino-5-{(3R)-1-[(1R)-1-(1H-imidazol-2-yl)propyl]-5',6'-dihydrospiro[pyrrolidine-3,4'-pyrrolo[1,2-b]pyrazol]-2'-yl}nicotinonitrile
2-amino-5-{(3R)-1-[(1S)-1-(1H-imidazol-2-yl)propyl]-5',6'-dihydrospiro[pyrrolidine-3,4'-pyrrolo[1,2-b]pyrazol]-2'-yl}nicotinonitrile
(3R)-2'-(6-amino-5-{[1-(6-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(6-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(5-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(5-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(4-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(4-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(3-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(3-methylpyridin-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[1-(1,4-dimethyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1)
(3R)-2'-(6-amino-5-{[1-(1,4-dimethyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 2)
(3R)-2'-(6-amino-5-{[-1-(1,4-dimethyl-1H-pyrazol-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1)
(3R)-2'-(6-amino-5-{[1-(1,4-dimethyl-1H-pyrazol-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 2)
(3R)-2'-{6-amino-5-[(1S)-1-(2,6-difluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(5-fluoropyridin-2-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(3-fluoropyridin-2-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(2,6-difluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(2-cyanophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 2)
(3'R)-2-[6-amino-5-(trifluoromethyl)pyridin-3-yl]-N-[1-(1-ethyl-1H-pyrazol-3-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[(1-methyl-1H-pyrazol-5-yl)methyl]-6,7-dihydrospiro[pyrazolo[5,1-c][1,4]oxazine-4,3'-pyrrolidine]-1'-carboxamide
(3'R)-2-[6-amino-5-(trifluoromethyl)pyridin-3-yl]-N-[(1-methyl-1H-pyrazol-4-yl)methyl]-6,7-dihydrospiro[pyrazolo[5,1-c][1,4]oxazine-4,3'-pyrrolidine]-1'-carboxamide
(3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1)
(3'R)-2-[6-amino-5-(trifluoromethyl)pyridin-3-yl]-N-[1-(1-methyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 2)
(3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1)
(3'R)-2-[6-amino-5-(trifluoromethyl)pyridin-3-yl]-N-[1-(1,3-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 2)
(3R)-2'-{6-amino-5-[(1R)-1-(3-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(4-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 1)
(3R)-2'-[6-amino-5-(1-phenylpropoxy)pyridin-3-yl]-N-ethyl-5',6'-dihydro-1H-spiro[pyrrolidine-3,4'-pyrrolo[1,2-b]pyrazole]-1-carboxamide (diastereomer 2)
(3R)-2'-{6-amino-5-[(1S)-1-(3-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1S)-1-(4-cyanophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1S)-1-(3,5-difluoropyridin-4-yl)ethoxy]pyridin-3-yl}-N-ethyl-5',6'-dihydrospiro[pyrrolidine-3,4'-pyrrolo[1,2-b]pyrazole]-1-carboxamide
(3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 1)
(3'R)-2-[6-amino-5-(trifluoromethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazine-4,3'-pyrrolidine]-1'-carboxamide (diastereomer 2)
(3'R)-2-[6-amino-5-(trifluoromethyl)pyridin-3-yl]-N-[1-(1,5-dimethyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3'R)-2-[6-amino-5-(trifluoromethyl)pyridin-3-yl]-N-[1-(1-methyl-1H-pyrazol-4-yl)ethyl]-6,7-dihydro-1'H-spiro[pyrazolo[5,1-c][1,4]oxazine-4,3'-pyrrolidine]-1'-carboxamide (diastereomer 1 and diastereomer 2)
(3R)-2'-{6-amino-5-[(1R)-1-(3-fluoropyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(3-fluoropyridin-2-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-phenylethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[1-(1-methyl-1H-pyrazol-5-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(1-methyl-1H-pyrazol-5-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 2)
(3R)-2'-{6-amino-5-[1-(1-methyl-1H-pyrazol-5-yl)ethoxy]pyridin-3-yl}-N-cyclobutyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(1-methyl-1H-pyrazol-5-yl)ethoxy]pyridin-3-yl}-N-cyclobutyl-5',6'-dihydro-1H-spiro[pyrrolidine-3,4'-pyrrolo[1,2-b]pyrazole]-1-carboxamide (diastereomer 2)
(3R)-2'-{6-amino-5-[(1R)-1-(2-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 1 and enantiomer 2)
2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 1)
2'-[6-amino-5-(trifluoromethyl)pyridin-3-yl]-N-[1-(1-methyl-1H-pyrazol-5-yl)ethyl]-5',6'-dihydro-1H-spiro[azetidine-3,4'-pyrrolo[1,2-b]pyrazole]-1-carboxamide (enantiomer 2)
2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 1 and enantiomer 2)
2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 1)
2'-[6-amino-5-(trifluoromethyl)pyridin-3-yl]-N-[1-(1,3-dimethyl-1H-pyrazol-4-yl)ethyl]-5',6'-dihydro-1H-spiro[azetidine-3,4'-pyrrolo[1,2-b]pyrazole]-1-carboxamide (enantiomer 2)
(3S)-2'-[6-amino-5-(trifluoromethyl)pyridin-3-yl]-N-[1-(1-methyl-1H-pyrazol-5-yl)ethyl]-5',6'-dihydro-1H-spiro[pyrrolidine-3,4'-pyrrolo[1,2-b]pyrazole]-1-carboxamide (diastereomer 1 and diastereomer 2)
(3S)-2'-[6-amino-5-(trifluoromethyl)pyridin-3-yl]-N-[1-(1,3-dimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(2,6-difluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-(6-amino-5-{methyl[(1R)-1-phenylethyl]amino}pyridin-3-yl)-N-ethyl-5',6'-dihydrospiro[pyrrolidine-3,4'-pyrrolo[1,2-b]pyrazole]-1-carboxamide
2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 1 and diastereomer 2)
2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 1)
2'-[6-amino-5-(trifluoromethyl)pyridin-3-yl]-N-[1-(1,3,5-trimethyl-1H-pyrazol-4-yl)ethyl]-5',6'-dihydro-1H-spiro[azetidine-3,4'-pyrrolo[1,2-b]pyrazole]-1-carboxamide (diastereomer 2)
(3R)-2'-{6-amino-5-[(1R)-1-(4-methylpyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(4-methylpyridin-2-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(6-methylpyridin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(6-methylpyridin-2-yl)ethoxy]pyridin-3-yl}-N-cyclobutyl-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide
(3S)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(1,3,5-trimethyl-1H-pyrazol-4-yl)ethyl]-5',6'-dihydro-1H-spiro[pyrrolidine-3,4'-pyrrolo[1,2-b]pyrazole]-1-carboxamide (diastereomer 2)
(2'-{6-amino-5-[(1R)-1-phenylethoxy]pyridin-3-yl}-5',6'-dihydrospiro[azetidine-3,4'-pyrrolo[1,2-b]pyrazol]-1-yl)(cyclopropyl)methanone
2'-[6-amino-5-(trifluoromethyl)pyridin-3-yl]-N-[1-(1-ethyl-1H-pyrazol-3-yl)ethyl]-5',6'-dihydro-1H-spiro[azetidine-3,4'-pyrrolo[1,2-b]pyrazole]-1-carboxamide (enantiomer 1)
2'-[6-amino-5-(trifluoromethyl)pyridin-3-yl]-N-[1-(1-ethyl-1H-pyrazol-3-yl)ethyl]-5',6'-dihydro-1H-spiro[azetidine-3,4'-pyrrolo[1,2-b]pyrazole]-1-carboxamide (enantiomer 2)
(35)-2'-[6-amino-5-(trifluoromethyl)pyridin-3-yl]-N-[1-(1-ethyl-1H-pyrazol-3-yl)ethyl]-5',6'-dihydro-1H-spiro[pyrrolidine-3,4'-pyrrolo[1,2-b]pyrazole]-1-carboxamide (diastereomer 1)
(3S)-2'-[6-am ino-5-(trifluoromethyl)pyridin-3-yl]-N-[1-(1-ethyl-1H-pyrazol-3-yl)ethyl]-5',6'-dihydro-1H-spiro[pyrrolidine-3,4'-pyrrolo[1,2-b]pyrazole]-1-carboxamide (diastereomer 2)
5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-1,2,4-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-1,2,4-Triazol-5-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[2,2-difluoro-1-(1H-imidazol-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[2,2-difluoro-1-(1H-imidazol-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethyl)pyridin-2-amine (diastereomer 2)
2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 1 and diastereomer 2)
2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 1)
2-amino-5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}nicotinonitrile (diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(difluoromethoxy)pyridin-2-amine (diastereomer 2)
5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 1 and enantiomer 2)
5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 1)
5-{1-[1-(1H-imidazol-2-yl)propyl]-5',6'-dihydrospiro[azetidine-3,4'-pyrrolo[1,2-b]pyrazol]-2'-yl}-3-(trifluoromethyl)pyridin-2-amine (enantiomer 2)
5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclobutyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-imidazol-2-yl)-2-methylpropyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclobutyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[1-(1H-imidazol-2-yl)-2,2-dimethylpropyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 1 and enantiomer 2)
5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 1)
5-{1-[cyclopropyl(1H-imidazol-2-yl)methyl]-5',6'-dihydrospiro[azetidine-3,4'-pyrrolo[1,2-b]pyrazol]-2'-yl}-3-(trifluoromethyl)pyridin-2-amine (enantiomer 2)
5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[2,2-difluoro-1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3S)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3S)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3S)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 1 and diastereomer 2)
2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 1)
2-amino-5-{(3R)-1-[cyclopropyl(1H-imidazol-2-yl)methyl]-5',6'-dihydrospiro[pyrrolidine-3,4'-pyrrolo[1,2-b]pyrazol]-2'-yl}nicotinonitrile (diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 1 and diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(difluoromethoxy)pyridin-2-amine (diastereomer 2)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 1 and diastereomer 2)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 1)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 2)
3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 1 and diastereomer 2)
3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 1)
3-(difluoromethoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastereomer 2)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 1)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluoromethoxy)pyridin-2-amine (diastereomer 2)
5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-imidazol-2-yl)-2-methylpropyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluoromethyl)pyridin-2-amine (diastereomer2)
{(3R)-2'-[6-amino-5-(trifluoromethyl)pyridin-3-yl]-5',6'-dihydrospiro[pyrrolidine-3,4'-pyrrolo[1,2-b]pyrazol]-1-yl}(1H-imidazol-2-yl)methanone
{(3R)-2'-[6-amino-5-(trifluoromethyl)pyridin-3-yl]-5',6'-dihydrospiro[pyrrolidine-3,4'-pyrrolo[1,2-b]pyrazol]-1-yl}(1-methyl-1H-imidazol-2-yl)methanone
3-[(1R)-1-(2,6-dichloro-3-fluorophenyl)ethoxy]-5-{(3R)-1-[(1H-imidazol-2-yl)methyl]-5',6'-dihydrospiro[pyrrolidine-3,4'-pyrrolo[1,2-b]pyrazol]-2'-yl}pyridin-2-amine
5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-imidazol-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-[6-amino-5-(trifluoromethyl)pyridin-3-yl]-N-[1-(3-chloropyridin-4-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[(1S)-1-(3-chloropyridin-4-yl)ethyl]-5',6'-dihydrospiro[pyrrolidine-3,4'-pyrrolo[1,2-b]pyrazole]-1-carboxamide
2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (enantiomer 2)
(3R)-2'-[6-amino-5-(trifluoromethyl)pyridin-3-yl]-N-[1-(3-methylpyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 2)
(3R)-2'-[6-amino-5-(trifluoromethyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[2-methyl-1-(pyridin-4-yl)propyl]-5',6'-dihydro-1H-spiro[pyrrolidine-3,4'-pyrrolo[1,2-b]pyrazole]-1-carboxamide (diastereomer 2)
(3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(3-fluoropyridin-4-yl)ethyl]-5',6'-dihydro-1H-spiro[pyrrolidine-3,4'-pyrrolo[1,2-b]pyrazole]-1-carboxamide (diastereomer 2)
(3R)-2'-[6-amino-5-(trifluoromethyl)pyridin-3-yl]-N-[(1R)-1-(3-chloropyridin-4-yl)ethyl]-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(2-methylpyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(3-methoxypyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(pyridin-4-yl)cyclopropyl]-5',6'-dihydrospiro[pyrrolidine-3,4'-pyrrolo[1,2-b]pyrazole]-1-carboxamide
(3R)-2'-{6-amino-5-[(1R)-1-(2-methoxyphenyl)ethoxy]pyridin-3-yl}-N-ethyl-5',6'-dihydrospiro[pyrrolidine-3,4'-pyrrolo[1,2-b]pyrazole]-1-carboxamide
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 1 and diastereomer 2)
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 1)
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzonitrile (diastereomer 2)
(3S)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (enantiomer 1)
(3R)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (enantiomer 2)
(3R)-2'-{6-amino-5-[(1R)-1-(3-chlorophenyl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1)
(3R)-2'-{6-amino-5-[1-(2,6-dichlorophenyl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 2)
5-{(3R)-1-[1-(1H-pyrrol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
(3R)-2'-[6-amino-5-(trifluoromethyl)pyridin-3-yl]-N-[1-(2-methoxypyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-[1-(1-methyl-1H-pyrazol-5-yl)ethyl]-5',6'-dihydrospiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(1-methyl-1H-pyrazol-5-yl)ethyl]-5',6'-dihydrospiro[pyrrolidine-3,4'-pyrrolo[1,2-b]pyrazole]-1-carboxamide (diastereomer 2)
3-(1-{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}propyl)benzamide (diastereomer 1 and diastereomer 2)
3-(1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}propyl)benzamide (diastereomer 1)
3-(1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}propyl)benzamide (diastereomer 2)
(3R)-2'-{6-amino-5-[(1R)-1-(1,3-thiazol-2-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(3-chlorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(2-chloro-3-fluoropyridin-4-yl)ethyl]-5',6'-dihydro-1H-spiro[pyrrolidine-3,4'-pyrrolo[1,2-b]pyrazole]-1-carboxamide (diastereomer 1 and diastereomer 2)
(3R)-2'-[6-amino-5-(trifluoromethyl)pyridin-3-yl]-N-{[3-(methylsulfanyl)pyridin-4-yl]methyl}-5',6'-dihydrospiro[pyrrolidine-3,4'-pyrrolo[1,2-b]pyrazole]-1-carboxamide
(3R)-2'-{6-amino-5-[(1R)-1-(1,3-thiazol-2-yl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(1,3-dimethyl-1H-pyrazol-4-yl)ethyl]-5',6'-dihydrospiro[pyrrolidine-3,4'-pyrrolo[1,2-b]pyrazole]-1-carboxamide (diastereomer 1)
(3R)-2'-[6-amino-5-(trifluoromethyl)pyridin-3-yl]-N-[1-(1,3-dimethyl-1H-pyrazol-4-yl)ethyl]-5',6'-dihydrospiro[pyrrolidine-3,4'-pyrrolo[1,2-b]pyrazole]-1-carboxamide (diastereomer 2)
(3R)-2'-{6-amino-5-[(1R)-1-(3-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[1-(pyridin-2-yl)cyclopropyl]-5',6'-dihydrospiro[pyrrolidine-3,4'-pyrrolo[1,2-b]pyrazole]-1-carboxamide
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-{6-amino-5-[(1R)-1-(1,3-thiazol-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-(trifluoromethyl)pyridin-3-yl]-N-[(3-methylpyridin-4-yl)methyl]-5',6'-dihydrospiro[pyrrolidine-3,4'-pyrrolo[1,2-b]pyrazole]-1-carboxamide
5-{(3R)-1-[1-(6-methoxypyridin-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(6-methoxypyridin-2-yl)ethyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-(6-amino-5-{1-[3-(3-hydroxy-3-methylbut-1-yn-1-yl)phenyl]ethoxy}pyridin-3-yl)-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(pyridin-3-yl)ethoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[1-(1,3-oxazol-2-yl)ethoxy]pyridin-3-yl}-N-ethyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(3-fluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (diastereomer 2)
5-{(3R)-1-[cyclopropyl(5-fluoropyridin-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
(3R)-2'-{6-amino-5-[(1R)-1-(pyrimidin-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(4-fluorophenyl)ethoxy]pyridin-3-yl}-N-ethyl-5',6'-dihydrospiro[pyrrolidine-3,4'-pyrrolo[1,2-b]pyrazole]-1-carboxamide
(3R)-2'-{6-amino-5-[(1R)-1-(4-fluorophenyl)ethoxy]pyridin-3-yl}-N-(propan-2-yl)-5',6'-dihydrospiro[pyrrolidine-3,4'-pyrrolo[1,2-b]pyrazole]-1-carboxamide
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
(3R)-2'-{6-amino-5-[(1R)-1-(4-chlorophenyl)ethoxy]pyridin-3-yl}-N-ethyl-5',6'-dihydrospiro[pyrrolidine-3,4'-pyrrolo[1,2-b]pyrazole]-1-carboxamide
5-{(3R)-1-[1-(dimethylamino)propan-2-yl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
(3R)-2'-{6-amino-5-[(1R)-1-(1,2-thiazol-5-yl)ethoxy]pyridin-3-yl}-N-ethyl-5',6'-dihydrospiro[pyrrolidine-3,4'-pyrrolo[1,2-b]pyrazole]-1-carboxamide
6-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyridin-2(1H)-one
(3R)-2'-{6-amino-5-[(1R)-1-(pyridin-3-yl)ethoxy]pyridin-3-yl}-N-(propan-2-yl)-5',6'-dihydrospiro[pyrrolidine-3,4'-pyrrolo[1,2-b]pyrazole]-1-carboxamide
2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (4 isomers)
(3R)-2'-{6-amino-5-[1-(1,3-oxazol-4-yl)ethoxy]pyridin-3-yl}-N-isopropyl-5',6'-dihydro-1H-spiro[pyrrolidine-3,4'-pyrrolo[1,2-b]pyrazole]-1-carboxamide (diastereomer 1 and diastereomer 2)
5-{(3R)-1-[(1,3-thiazol-2-yl)methyl]-5',6'-dihydrospiro[pyrrolidine-3,4'-pyrrolo[1,2-b]pyrazol]-2'-yl}-3-(trifluoromethyl)pyridin-2-amine
6-({(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}methyl)pyridin-2(1H)-one
(3S)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 1)
(3S)-2'-[6-amino-5-(trifluoromethyl)pyridin-3-yl]-N-[cyclopropyl(phenyl)methyl]-5',6'-dihydrospiro[pyrrolidine-3,4'-pyrrolo[1,2-b]pyrazole]-1-carboxamide (diastereomer 2)
(3R)-2'-[6-amino-5-(6,7-dihydro-5H-cyclopenta[b]pyridin-7-yloxy)pyridin-3-yl]-N-ethyl-5',6'-dihydro-1H-spiro[pyrrolidine-3,4'-pyrrolo[1,2-b]pyrazole]-1-carboxamide (diastereomer 1)
2'-{6-amino-5-[(1R)-1-(pyridin-2-yl)ethoxy]pyridin-3-yl}-N-ethyl-5',6'-dihydrospiro[azetidine-3,4'-pyrrolo[1,2-b]pyrazole]-1-carboxamide
1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-(methylamino)ethan-1-one
(2R)-1-{(3R)-2'-[6-amino-5-(trifluoromethyl)pyridin-3-yl]-5',6'-dihydrospiro[pyrrolidine-3,4'-pyrrolo[1,2-b]pyrazol]-1-yl}-2-(methylamino)propan-1-one
(5R)-5-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyrrolidin-2-one
5-{(3R)-1-[(1,3,4-thiadiazol-2-yl)methyl]-5',6'-dihydrospiro[pyrrolidine-3,4'-pyrrolo[1,2-b]pyrazol]-2'-yl}-3-(trifluoromethyl)pyridin-2-amine
(5S)-5-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}methyl)pyrrolidin-2-one
5-{(3'R)-1'-[1-(6-methoxypyridin-2-yl)ethyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclopropyl(6-methoxypyridin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[cyclopropyl(6-methoxypyridin-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[1-(3-fluoropyridin-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1 and diastereomer 2)
3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1)
3-(difluoromethoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 2)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastereomer 1 and diastereomer 2)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastereomer 1)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastereomer 2)
3-(difluoromethyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1 and diastereomer 2)
3-(difluoromethyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 1)
3-(difluoromethyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastereomer 2)
(3R)-2'-(6-amino-5-{[1-(1-propyl-1H-pyrazol-5-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(1,3-thiazol-4-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(pyrimidin-4-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(2,4-dimethylpyrimidin-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1)
(3R)-2'-(6-amino-5-{[1-(2,4-dimethylpyrimidin-5-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 2)
(3R)-2'-{6-amino-5-[(1R)-1-(5-methyl-1,3,4-oxadiazol-2-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(1-methyl-1H-1,2,3-triazol-5-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-(6-amino-5-{[1-(1-methyl-1H-1,2,3-triazol-4-yl)ethyl]oxy}pyridin-3-yl)-N-(propan-2-yl)-5',6'-dihydrospiro[pyrrolidine-3,4'-pyrrolo[1,2-b]pyrazole]-1-carboxamide (diastereomer 1 and diastereomer 2)
(5S)-5-{[(3R)-2'-{6-amino-5-[(1R)-1-phenylethoxy]pyridin-3-yl}-5',6'-dihydrospiro[pyrrolidine-3,4'-pyrrolo[1,2-b]pyrazol]-1-yl]methyl}pyrrolidin-2-one
(5R)-5-{[(3R)-2'-{6-amino-5-[(1R)-1-phenylethoxy]pyridin-3-yl}-5',6'-dihydrospiro[pyrrolidine-3,4'-pyrrolo[1,2-b]pyrazol]-1-yl]methyl}pyrrolidin-2-one
(3R)-2'-(6-amino-5-{1-[4-(methylsulfonyl)phenyl]ethoxy}pyridin-3-yl)-N-isopropyl-5',6'-dihydro-1H-spiro[pyrrolidine-3,4'-pyrrolo[1,2-b]pyrazole]-1-carboxamide (diastereomer 1 and diastereomer 2)
(3R)-2'-{6-amino-5-[(1R)-1-(2-methyl-1,3-thiazol-4-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-({1-[3-(methanesulfonyl)phenyl]ethyl}oxy)pyridin-3-yl]-N-(propan-2-yl)-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(4-methyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(4,5-dimethyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-(6-amino-5-{[1-(5-methyl-1,3-thiazol-2-yl)ethyl]oxy}pyridin-3-yl)-N-ethyl-5',6'-dihydrospiro[pyrrolidine-3,4'-pyrrolo[1,2-b]pyrazole]-1-carboxamide (diastereomer 1 and diastereomer 2)
(3R)-2'-{6-amino-5-[(1R)-1-(pyridin-4-yl)ethoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(pyridin-4-yl)ethoxy]pyridin-3-yl}-N-cyclobutyl-5',6'-dihydrospiro[pyrrolidine-3,4'-pyrrolo[1,2-b]pyrazole]-1-carboxamide
6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 1 and diastereomer 2)
6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 1)
6-(1-{(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}ethyl)pyridin-2-ol (diastereomer 2)
6-({(3R)-2'-[6-amino-5-(trifluoromethyl)pyridin-3-yl]-5',6'-dihydrospiro[pyrrolidine-3,4'-pyrrolo[1,2-b]pyrazol]-1-yl}(cyclopropyl)methyl)pyridin-2-ol (diastereomer 1 and diastereomer 2)
6-({(3'R)-2-[6-amino-5-(trifluoromethyl)pyridin-3-yl]-6,7-dihydrospiro[pyrazolo[5,1-c][1,4]oxazine-4,3'-pyrrolidin]-1'-yl}(cyclopropyl)methyl)pyridin-2-ol (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(3,3-difluorocyclobutyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 1)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 2)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 3)
5-{1'-[(3,3-difluorocyclobutyl)(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (isomer 4)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(1H-imidazol-2-yl)(1-methylcyclopropyl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclobutyl(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[cyclohexyl(1H-imidazol-2-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclohexyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[cyclopropyl(1H-imidazol-2-yl)methyl]-6,7-dihydrospiro[pyrazolo[5,1-c][1,4]oxazine-4,3'-pyrrolidin]-2-yl}-3-(trifluoromethyl)pyrazin-2-amine (diastereomer 1 and diastereomer 2)
5-{(3R)-1-[(1H-imidazol-2-yl)(1-methylcyclobutyl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 1 and diastereomer 2)
5-{(3R)-1-[(1-methylcyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diasteroemr 1 and diastereomer 2)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 1 and diastereomer 2)
5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(3,3-difluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclopropyl(1-methyl-1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3'R)-1'-[1-(4H-1,2,4-triazol-3-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diatereomer 1 and diastereomer 2)
5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-imidazol-2-yl)(²H)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopropyl(1H-imidazol-2-yl)(²H)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(2-methoxypyridin-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
3-[{(3R)-2'-[6-amino-5-(trifluoromethyl)pyridin-3-yl]-5',6'-dihydro-1H-spiro[pyrrolidine-3,4'-pyrrolo[1,2-b]pyrazol]-1-yl}(cyclopropyl)methyl]pyridin-2(1H)-one (diastereomer 1 and diastereomer 2)
5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(1-fluorocyclopropyl)(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[cyclopropyl(4-methoxypyrimidin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclobutyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1 and diastereomer 2)
5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3'R)-1'-[cyclopropyl(1H-1,2,3-triazol-5-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 2)
5-{(3R)-1-[(1-fluorocyclobutyl)(4H-1,2,4-triazol-3-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(pyrimidin-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(2,6-difluorophenyl)ethoxy]pyridin-2-amine (diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-[(1R)-1-(3-fluoropyridin-2-yl)ethoxy]pyridin-2-amine (diastereomer 2)
5-{(3'R)-1'-[cyclobutyl(4H-1,2,4-triazol-3-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1)
5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopentyl(1H-imidazol-2-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[(4,4-difluorocyclohexyl)(1H-1,2,4-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3R)-1-[2,2-difluoro-1-(1H-imidazol-2-yl)propyl]-5',6'-dihydrospiro[pyrrolidine-3,4'-pyrrolo[1,2-b]pyrazol]-2'-yl}-3-(trifluoromethyl)pyridin-2-amine (diastereomer 1 and diastereomer 2)
5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 1)
5-{(3R)-1-[cyclopropyl(1H-1,2,3-triazol-5-yl)methyl]-5',6'-dihydrospiro[pyrrolidine-3,4'-pyrrolo[1,2-b]pyrazol]-2'-yl}-3-(trifluoromethyl)pyridin-2-amine (diastereomer 2)
5-{(3'R)-1'-[cyclobutyl(4H-1,2,4-triazol-3-yl)methyl]-6,7-dihydrospiro[pyrazolo[5,1-c][1,4]oxazine-4,3'-pyrrolidin]-2-yl}-3-(trifluoromethyl)pyridin-2-amine (diastereomer 1 and diastereomer 2)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 1)
5-{(3R)-1-[cyclopropyl(1H-1,2,4-triazol-5-yl)methyl]-5',6'-dihydrospiro[pyrrolidine-3,4'-pyrrolo[1,2-b]pyrazol]-2'-yl}-3-(trifluoromethoxy)pyridin-2-amine (diastereomer 2)
(3R)-2'-{5-amino-6-[(1R)-1-phenylethoxy]pyrazin-2-yl}-N-ethyl-5',6'-dihydrospiro[pyrrolidine-3,4'-pyrrolo[1,2-b]pyrazole]-1-carboxamide
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1 and diastereomer 2)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 1)
5-{(3R)-1-[cyclopropyl(1H-imidazol-2-yl)methyl]-5',6'-dihydrospiro[pyrrolidine-3,4'-pyrrolo[1,2-b]pyrazol]-2'-yl}-3-(trifluoromethyl)pyrazin-2-amine (diastereomer 2)
or a stereoisomer, a tautomer, an N-oxide, a hydrate, a solvate, or a salt thereof, or a mixture of same.

10. A compound of general formula (I) according to any one of claims 1, 2, 3, 4, 5, 6, 7, 8 or 9 for the use as a medicament.

11. A compound of general formula (I) according to any one of claims 1, 2, 3, 4, 5, 6, 7, 8 or 9 for use in the treatment or prophylaxis of a disease.

12. A pharmaceutical composition comprising a compound of general formula (I) according to any one of claims 1, 2, 3, 4, 5, 6, 7, 8 or 9 and one or more pharmaceutically acceptable excipients.

13. A pharmaceutical combination comprising:
one or more first active ingredients being compounds of general formula (I) according to any one of claims 1, 2, 3, 4, 5, 6, 7, 8 or 9 and
one or more pharmaceutical active anti cancer compounds or
one or more pharmaceutical active immune checkpoint inhibitors.

14. A pharmaceutical combination according to claim 13, **characterized in that** the pharmaceutical active immune checkpoint inhibitor is an antibody.

15. A compound of general formula (I) according to any one of claims 1, 2, 3, 4, 5, 6, 7, 8 or 9 for use in the treatment or prophylaxis of cancer, a benign hyperplasia, an atherosclerotic disorder, sepsis, an autoimmune disorder, a vascular disorder, a viral infection, a neurodegenerative disorder, or an inflammatory disorder.

16. Use of a compound of general formula (I) according to any one of claims 1, 2, 3, 4, 5, 6, 7, 8 or 9 for male fertility control.

## Patentansprüche

1. Verbindung der Formel (I) 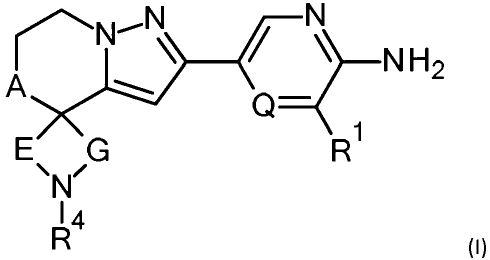 wobei
A ausgewählt ist aus einer Einfachbindung, -CH₂- oder - O-;
E -(CH₂)ₙ- mit n = 1 oder 2 ist;
G -(CH₂)- ist, mit der Maßgabe, dass die cyclische Teilstruktur, die E-N-G umfasst, ein 4- bis 5-gliedriges Heterocycloalkyl ist;
Q ausgewählt ist aus N oder C-R²;
R¹ ausgewählt ist aus
• -NO₂; -CN;
• C₁₋₄-Alkyl, gegebenenfalls substituiert mit -OH oder 1 bis 3 -F;
• Cyclopropyl;
• 4 bis 6-gliedrigem Heterocycloalkyl oder Heterocycloalkenyl, beide mit 1-2 Stickstoffatomen, jedoch immer über ein Kohlenstoffatom mit der Phenylgruppe von Verbindungen der Formel (I) verknüpft und beide gegebenenfalls mit C₁₋₄-Alkyl substituiert;
• Phenyl, gegebenenfalls substituiert mit Halogen, -CN, C₁₋₄-Alkyl, C₁₋₄-Halogenalkyl, C_{3-6 -}Cycloalkyl, O-C₁₋₄-Alkyl, O-C₃₋₆-Cycloalkyl, C₁₋₃-Alkinyl, -C (O)-R_{d}, SR_{d}, SOR_{d}, SO₂R_{d}, SONR_{d}, SON (H) R_{d}, -SO₂-NH₂, -SO₂-N(H) -R_{d} oder - SO₂-N(R_{d})₂, wobei R_{d} ausgewählt ist aus Methyl, Ethyl, Cyclopropyl, Isopropyl und tert-Butyl;
• 5- bis 6-gliedrigem Heteroaryl mit 1, 2 oder 3 Heteroatomen ausgewählt aus N, O oder S und gegebenenfalls substituiert mit 1 oder 2 der folgenden Substituenten: Halogen, -CN, C₁₋₄-Alkyl, C₁₋₄-Halogenalkyl, C₃₋₆-Cycloalkyl, O-C₁₋₄-Alkyl, O-C₃₋₆-Cycloalkyl, C₁₋₃-Alkinyl, SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
• -O-CH₃; -O-CH(CH₃)₂; -O-CHF₂; -O-CF₃; 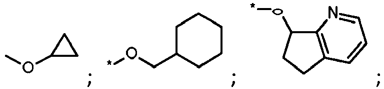
• -O-CHRₐ-Phenyl, gegebenenfalls substituiert mit einem, zwei oder drei der folgenden Substituenten: Halogen, - CN, C₁₋₄-Alkyl, gegebenenfalls substituiert mit einem oder mehreren -F, O-C₁₋₄-Alkyl, C₁₋₆-Alkinyl-OH oder -SO₂-CH₃, und mit Rₐ ausgewählt aus -H, -F oder -CH₃, -CF₃, -CH₂-CH₃, -CH(CH₃)₂ oder -Cyclopropyl;
• -O-CHRₐ-6-gliedriges-Heteroaryl, gegebenenfalls substituiert mit einem, zwei oder drei der folgenden Substituenten: Halogen, -CN, C₁₋₄-Alkyl, gegebenenfalls substituiert mit einem oder mehreren -F oder -SO₂-CH₃, und mit Rₐ ausgewählt aus -H, -F oder -CH₃, -CF₃, -CH₂-CH₃, -CH(CH₃)₂ oder -Cyclopropyl;
• -COORₐ mit Rₐ ausgewählt aus -H oder -CH₃;
• C(=O)-Methyl, gegebenenfalls substituiert mit 1 bis 3 -F;
• -C(=O)-NR_{b}R_{c}, wobei R_{b} ausgewählt ist aus -H, -CH₃ oder -CH₂-CH₃ und wobei R_{c} ausgewählt ist aus -H, -CH₃, -CH₂-CH₃, Cyclohexyl, Phenyl oder 5 bis 6-gliedrigem Heteroaryl, alle gegebenenfalls substituiert mit einem oder zwei Halogen, -CN, -SO₂-CH₃ oder C₁₋₄-Alkyl; gegebenenfalls einfach oder mehrfach substituiert mit Halogen;
• -S-CR_{f}R_{g}Rₕ, wobei R_{f} ausgewählt ist aus -H oder -F, wobei R_{g} ausgewählt ist aus -H, -F oder -CH₃ und wobei Rₕ ausgewählt ist aus H, F, -CH₃ oder C₅₋₆-Cycloalkyl oder Phenyl;
• 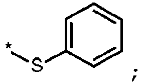
R² ausgewählt ist aus -H;
R⁴ ausgewählt ist aus
• C₁₋₆-Alkyl, C₃₋₆-Cycloalkyl oder C₁₋₃-Alkyl-C₃₋₆-cycloalkyl, alle gegebenenfalls ein- oder unabhängig mehrfach substituiert mit
∘ -F, -Cl, -Br, -CN;
∘ -OH, -ORᵢ, wobei Rᵢ ausgewählt ist aus C₁₋₄-Alkyl oder C₃₋₆-Cycloalkyl, beide gegebenenfalls ein- oder mehrfach substituiert mit Halogen,
∘ -CF₃, -CHF₂, -CH₂F oder -CH₂-CF₃;
∘ -NRⱼRₖ, wobei Rⱼ und Rₖ unabhängig ausgewählt sind aus -H und C₁-C₄-Alkyl,
∘ -CH₂-O-CH₃ oder
∘ C₃₋₄-Cycloalkyl oder 4-6-gliedriges Heterocycloalkyl, beide gegebenenfalls substituiert mit (=O) oder gegebenenfalls ein- oder mehrfach substituiert mit F oder CH₃;
∘ C₆₋₈-Spiroalkyl und C₅₋₇-Bicycloalkyl
∘ Phenyl oder 5- bis 6-gliedriges Heteroaryl, beide gegebenenfalls ein- oder mehrfach substituiert mit
■ Halogen, -CN,
■ -CF₃, -CHF₂, -CH₂F,
■ C₁₋₄-Alkyl, gegebenenfalls substituiert mit einem oder mehreren Halogenen;
■ C₃₋₆-Cycloalkyl;
■ O-C₁₋₄-Alkyl,
■ O-C₃₋₆-Cycloalkyl,
■ C₁₋₃-Alkinyl,
■ -NRⱼRₖ, wobei Rⱼ und Rₖ unabhängig ausgewählt sind aus -H und C₁-C₄-Alkyl
■ SR_{d}, SOR_{d}, SO₂R_{d}, SONR_{d} oder SON (H)R_{d}, wobei R_{d} ausgewählt ist aus Methyl, Ethyl, Cyclopropyl, Isopropyl und tert-Butyl; oder
o Phenyl gegebenenfalls ein- oder mehrfach substituiert ist mit -C(O)NRⱼRₖ, wobei Rⱼ und Rₖ unabhängig ausgewählt sind aus -H und C₁-C₄-Alkyl, oder
o 5 bis 6-gliedriges Heteroaryl gegebenenfalls ein- oder mehrfach substituiert ist mit -OH;
• C(=O)-Rₗ, bei dem Rₗ ausgewählt ist aus
∘ C₁₋₄-Alkyl, C₃₋₆-Cycloalkyl oder C₃₋₆-Heterocycloalkyl, beide gegebenenfalls ein- oder mehrfach substituiert mit
■ -F, -Cl, -Br, -CN;
■ -OH, -ORᵢ mit Rᵢ ausgewählt aus C₁₋₄-Alkyl oder C₃₋₆-Cycloalkyl, -CF₃, -CHF₂, -CH₂F;
■ -NRⱼRₖ, wobei Rⱼ und Rₖ unabhängig ausgewählt sind aus -H und C₁-C₄-Alkyl
■ Phenyl oder 5- bis 6-gliedriges Heteroaryl, beide gegebenenfalls ein- oder mehrfach substituiert mit
• Halogen, -CN,
• -CF₃, -CHF₂, -CH₂F,
• C₁₋₄-Alkyl, gegebenenfalls substituiert mit einem oder mehreren Halogenen;
• C₃₋₆-Cycloalkyl;
• O-C₁₋₄-Alkyl,
• O-C₃₋₆-Cycloalky,
• C₁₋₃-Alkinyl,
• -NRⱼRₖ, wobei Rⱼ und Rₖ unabhängig ausgewählt sind aus -H und C₁-C₄-Alkyl
• SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
∘ Phenyl oder 5- bis 6-gliedriges Heteroaryl, beide gegebenenfalls ein- oder mehrfach substituiert mit
■ Halogen, -CN,
■ -CF₃, -CHF₂, -CH₂F,
■ C₁₋₄-Alkyl, gegebenenfalls substituiert mit einem oder mehreren Halogenen;
■ C₃₋₆-Cycloalkyl;
■ O-C₁₋₄-Alkyl,
■ O-C₃₋₆-Cycloalkyl,
■ C₁₋₃-Alkinyl,
■ -NRⱼRₖ, wobei Rⱼ und Rₖ unabhängig ausgewählt sind aus -H und C₁-C₄-Alkyl
■ SR_{d}, SOR_{d}, SO₂R_{d}, SONR_{d} oder SON (H)R_{d}, wobei R_{d} ausgewählt ist aus Methyl, Ethyl, Cyclopropyl, Isopropyl und tert-Butyl;
• C(=O)-NRₒRₚ, wobei Rₒ und Rₚ unabhängig ausgewählt sind aus
∘ H
∘ C₁₋₄-Alkyl, C₃₋₆-Cycloalkyl oder C₁₋₃-Alkyl-C₃₋₆-cycloalkyl, beide gegebenenfalls ein- oder mehrfach substituiert mit
■ -F, -Cl, -Br, -CN;
■ -OH, -ORᵢ mit Rᵢ ausgewählt aus C₁₋₄-Alkyl oder C₃₋₆-Cycloalkyl, -CF₃, -CHF₂, -CH₂F;
■ -NRⱼRₖ, wobei Rⱼ und Rₖ unabhängig ausgewählt sind aus -H und C₁-C₄-Alkyl
■ Phenyl oder 5- bis 6-gliedriges Heteroaryl, beide gegebenenfalls ein- oder mehrfach substituiert mit
• Halogen, -CN,
• -CF₃, -CHF₂, -CH₂F,
• C₁₋₄-Alkyl, gegebenenfalls substituiert mit einem oder mehreren Halogenen;
• C₃₋₆-Cycloalkyl;
• O-C₁₋₄-Alkyl,
• O-C₃₋₆-Cycloalky,
• C₁₋₃-Alkinyl,
• -NRⱼRₖ, wobei Rⱼ und Rₖ unabhängig ausgewählt sind aus -H und C₁-C₄-Alkyl
• SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
∘ Phenyl oder 5- bis 6-gliedriges Heteroaryl, beide gegebenenfalls ein- oder mehrfach substituiert mit
■ Halogen, -CN,
■ -CF₃, -CHF₂, -CH₂F,
■ C₁₋₄-Alkyl, gegebenenfalls substituiert mit einem oder mehreren Halogenen;
■ C₃₋₆-Cycloalkyl;
■ O-C₁₋₄-Alkyl,
■ O-C₃₋₆-Cycloalky,
■ C₁₋₃-Alkinyl,
■ -NRⱼRₖ, wobei Rⱼ und Rₖ unabhängig ausgewählt sind aus -H und C₁-C₄-Alkyl
■ SR_{d}, SOR_{d}, SO₂R_{d}, SONR_{d} oder SON (H)R_{d}, wobei R_{d} ausgewählt ist aus Methyl, Ethyl, Cyclopropyl, Isopropyl und tert-Butyl;
oder ein Stereoisomer, ein Tautomer, ein N-Oxid, ein Hydrat, ein Solvat oder ein Salz davon oder ein Gemisch davon.

2. Verbindung der Formel (Ia) nach Anspruch 1, 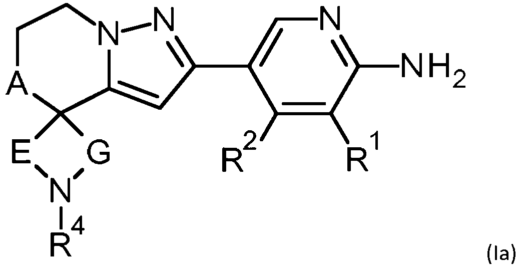 wobei
A ausgewählt ist aus einer Einfachbindung, -CH₂- oder - O-;
E -(CH₂)ₙ- mit n = 1 oder 2 ist;
G -(CH₂)- ist, mit der Maßgabe, dass die cyclische Teilstruktur, die E-N-G umfasst, ein 4- bis 5-gliedriges Heterocycloalkyl ist;
R¹ ausgewählt ist aus
• -NO₂; -CN;
• C₁₋₄-Alkyl, gegebenenfalls substituiert mit -OH oder 1 bis 3 -F;
• Cyclopropyl;
• 4 bis 6-gliedrigem Heterocycloalkyl oder Heterocycloalkenyl, beide mit 1-2 Stickstoffatomen, jedoch immer über ein Kohlenstoffatom mit der Phenylgruppe von Verbindungen der Formel (I) verknüpft und beide gegebenenfalls mit C₁₋₄-Alkyl substituiert;
• Phenyl, gegebenenfalls substituiert mit Halogen, -CN, C₁₋₄-Alkyl, C₁₋₄-Halogenalkyl, C_{3-6 -}Cycloalkyl, O-C₁₋₄-Alkyl, O-C₃₋₆-Cycloalkyl, C₁₋₃-Alkinyl, -C(O)-R_{d}, SR_{d}, SOR_{d}, SO₂R_{d}, SONR_{d}, SON(H)R_{d}, -SO₂-NH₂, -SO₂-N(H)-R_{d} oder - SO₂-N(R_{d})₂, wobei R_{d} ausgewählt ist aus Methyl, Ethyl, Cyclopropyl, Isopropyl und tert-Butyl;
• 5 bis 6-gliedrigem Heteroaryl mit 1 oder 2 Heteroatomen ausgewählt aus N oder S und gegebenenfalls substituiert mit Halogen, -CN, C₁₋₄-Alkyl, C₁₋₄-Halogenalkyl, C₃₋₆-Cycloalkyl, O-C₁₋₄-Alkyl, O-C₃₋₆-Cycloalkyl, C₁₋₃-Alkinyl, SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
• -O-CH₃; -O-CH(CH₃)₂; -O-CHF₂; -O-CF₃; 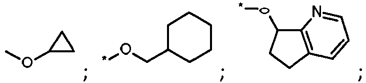
• -O-CHRₐ-Phenyl, gegebenenfalls substituiert mit einem oder zwei der folgenden Substituenten: Halogen, -CN, C₁₋₄-Alkyl gegebenenfalls substituiert mit einem oder mehreren -F oder -SO₂-CH₃, und mit Rₐ ausgewählt aus -H, -F oder -CH₃, -CF₃, -CH₂-CH₃, -CH(CH₃)₂ oder -Cyclopropyl;
• -O-CHRₐ-6-gliedriges Heteroaryl, gegebenenfalls substituiert mit einem oder zwei der folgenden Substituenten: Halogen, -CN, C₁₋₄-Alkyl gegebenenfalls substituiert mit einem oder mehreren -F oder -SO₂-CH₃, und mit Rₐ ausgewählt aus -H, -F oder -CH₃, -CF₃, -CH₂-CH₃, -CH(CH₃)₂ oder -Cyclopropyl;
• -COORₐ mit Rₐ ausgewählt aus -H oder -CH₃;
• C(=O)-Methyl, gegebenenfalls substituiert mit 1 bis 3 -F;
• -C(=O)-NR_{b}R_{c}, wobei R_{b} ausgewählt ist aus -H, -CH₃ oder -CH₂-CH₃ und wobei R_{c} ausgewählt ist aus -H, -CH₃, -CH₂-CH₃, Cyclohexyl, Phenyl oder 5 bis 6-gliedrigem Heteroaryl, alle gegebenenfalls substituiert mit einem oder zwei Halogen, -CN, -SO₂-CH₃ oder C₁₋₄-Alkyl; gegebenenfalls einfach oder mehrfach substituiert mit Halogen;
• -S-CR_{f}R_{g}Rₕ, wobei R_{f} ausgewählt ist aus -H oder -F, wobei R_{g} ausgewählt ist aus -H, -F oder -CH₃ und wobei Rₕ ausgewählt ist aus H, F, -CH₃ oder C₅₋₆-Cycloalkyl oder Phenyl;
• 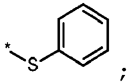
R² ausgewählt ist aus -H; oder
R⁴ ausgewählt ist aus
• C₁₋₅-Alkyl oder C₃₋₆-Cycloalkyl, beide gegebenenfalls ein- oder unabhängig mehrfach substituiert mit
∘ -F, -Cl, -Br, -CN;
∘ -OH, -ORᵢ, wobei Rᵢ ausgewählt ist aus C₁₋₄-Alkyl oder C₃₋₆-Cycloalkyl,
∘ -CF₃, -CHF₂, -CH₂F oder -CH₂-CF₃;
∘ -NRⱼRₖ, wobei Rⱼ und Rₖ unabhängig ausgewählt sind aus -H und C₁-C₄-Alkyl,
∘ -CH₂-O-CH₃ oder
∘ C₃₋₄-Cycloalkyl oder 4-gliedriges Heterocycloalkyl, beide gegebenenfalls substituiert mit -F oder -CH₃;
∘ Phenyl oder 5- bis 6-gliedriges Heteroaryl, beide gegebenenfalls ein- oder mehrfach substituiert mit
■ Halogen, -CN,
■ -CF₃, -CHF₂, -CH₂F,
■ C₁₋₄-Alkyl, gegebenenfalls substituiert mit einem oder mehreren Halogenen;
■ C₃₋₆-Cycloalkyl;
■ O-C₁₋₄-Alkyl,
■ O-C₃₋₆-Cycloalky,
■ C₁₋₃-Alkinyl,
■ -NRⱼRₖ, wobei Rⱼ und Rₖ unabhängig ausgewählt sind aus -H und C₁-C₄-Alkyl
■ SR_{d}, SOR_{d}, SO₂R_{d}, SONR_{d} oder SON (H)R_{d}, wobei R_{d} ausgewählt ist aus Methyl, Ethyl, Cyclopropyl, Isopropyl und tert-Butyl;
• C(=O)-R₁, bei dem R₁ ausgewählt ist aus
∘ C₁₋₄-Alkyl, C₃₋₆-Cycloalkyl oder C₃₋₆-Heterocycloalkyl, beide gegebenenfalls ein- oder mehrfach substituiert mit
■ -F, -Cl, -Br, -CN;
■ -OH, -ORᵢ mit Rᵢ ausgewählt aus C₁₋₄-Alkyl oder C₃₋₆-Cycloalkyl, -CF₃, -CHF₂, -CH₂F;
■ -NRⱼRₖ, wobei Rⱼ und Rₖ unabhängig ausgewählt sind aus -H und C₁-C₄-Alkyl
■ Phenyl oder 5- bis 6-gliedriges Heteroaryl, beide gegebenenfalls ein- oder mehrfach substituiert mit
• Halogen, -CN,
• -CF₃, -CHF₂, -CH₂F,
• C₁₋₄-Alkyl, gegebenenfalls substituiert mit einem oder mehreren Halogenen;
• C₃₋₆-Cycloalkyl;
• O-C₁₋₄-Alkyl,
• O-C₃₋₆-Cycloalky,
• C₁₋₃-Alkinyl,
• -NRⱼRₖ, wobei Rⱼ und Rₖ unabhängig ausgewählt sind aus -H und C₁-C₄-Alkyl
• SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
∘ Phenyl oder 5- bis 6-gliedriges Heteroaryl, beide gegebenenfalls ein- oder mehrfach substituiert mit
■ Halogen, -CN,
■ -CF₃, -CHF₂, -CH₂F,
■ C₁₋₄-Alkyl, gegebenenfalls substituiert mit einem oder mehreren Halogenen;
■ C₃₋₆-Cycloalkyl;
■ O-C₁₋₄-Alkyl,
■ O-C₃₋₆-Cycloalky,
■ C₁₋₃-Alkinyl,
■ -NRⱼRₖ, wobei Rⱼ und Rₖ unabhängig ausgewählt sind aus -H und C₁-C₄-Alkyl
■ SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
• C(=O)-NRₒRₚ, wobei Rₒ und Rₚ unabhängig ausgewählt sind aus
∘ H
∘ C₁₋₄-Alkyl oder C₃₋₆-Cycloalkyl, beide gegebenenfalls ein- oder mehrfach substituiert mit
■ -F, -Cl, -Br, -CN;
■ -OH, -ORᵢ mit Rᵢ ausgewählt aus C₁₋₄-Alkyl oder C₃₋₆-Cycloalkyl, -CF₃, -CHF₂, -CH₂F;
■ -NRⱼRₖ, wobei Rⱼ und Rₖ unabhängig ausgewählt sind aus -H und C₁-C₄-Alkyl
■ Phenyl oder 5- bis 6-gliedriges Heteroaryl, beide gegebenenfalls ein- oder mehrfach substituiert mit
• Halogen, -CN,
• -CF₃, -CHF₂, -CH₂F,
• C₁₋₄-Alkyl, gegebenenfalls substituiert mit einem oder mehreren Halogenen;
• C₃₋₆-Cycloalkyl;
• O-C₁₋₄-Alkyl,
• O-C₃₋₆-Cycloalkyl,
• C₁₋₃-Alkinyl,
• -NRⱼRₖ, wobei Rⱼ und Rₖ unabhängig ausgewählt sind aus
-H und C₁-C₄-Alkyl
• SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
∘ Phenyl oder 5- bis 6-gliedriges Heteroaryl, beide gegebenenfalls ein- oder mehrfach substituiert mit
■ Halogen, -CN,
■ -CF₃, -CHF₂, -CH₂F,
■ C₁₋₄-Alkyl, gegebenenfalls substituiert mit einem oder mehreren Halogenen;
■ C₃₋₆-Cycloalkyl;
■ O-C₁₋₄-Alkyl,
■ O-C₃₋₆-Cycloalky,
■ C₁₋₃-Alkinyl,
■ -NRⱼRₖ, wobei Rⱼ und Rₖ unabhängig ausgewählt sind aus -H und C₁-C₄-Alkyl
■ SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl; oder ein Stereoisomer, ein Tautomer, ein N-Oxid, ein Hydrat, ein Solvat oder ein Salz davon oder ein Gemisch davon.

3. Verbindungen der Formel (I) nach Anspruch 1, wobei A ausgewählt ist aus einer Einfachbindung oder -O-, oder ein Stereoisomer, ein Tautomer, ein N-Oxid, ein Hydrat, ein Solvat oder ein Salz davon oder ein Gemisch davon.

4. Verbindungen der Formel (I) nach Anspruch 1, wobei E -CH₂-CH₂- ist, oder ein Stereoisomer, ein Tautomer, ein N-Oxid, ein Hydrat, ein Solvat oder ein Salz davon oder ein Gemisch davon.

5. Verbindungen der Formel (I) nach Anspruch 1, wobei Q C-R² ist, oder ein Stereoisomer, ein Tautomer, ein N-Oxid, ein Hydrat, ein Solvat oder ein Salz davon oder ein Gemisch davon.

6. Verbindungen der Formel (I) nach Anspruch 1, wobei Q N ist, oder ein Stereoisomer, ein Tautomer, ein N-Oxid, ein Hydrat, ein Solvat oder ein Salz davon oder ein Gemisch davon.

7. Verbindungen der Formel (I) nach Anspruch 1, wobei R₄ 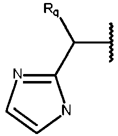 ist und R_{q} ausgewählt ist aus H, -CH₃, -CH₂-CH₃, -CH(CH₃)₂, -CH₂-O-CH₃, -CF₃, -CHF₂, -CH₂F, -CH₂-CF₃, Cyclopropyl oder Cyclobutyl, oder ein Stereoisomer, ein Tautomer, ein N-Oxid, ein Hydrat, ein Solvat, oder ein Salz davon oder ein Gemisch davon.

8. Verbindungen der Formel (I) nach Anspruch 1, wobei R₄ ausgewählt ist aus C(=O)-NRₒRₚ, wobei Rₒ und Rₚ unabhängig ausgewählt sind aus
∘ H
∘ C₁₋₄-Alkyl oder C₃₋₆-Cycloalkyl, beide gegebenenfalls ein- oder mehrfach substituiert mit
■ -F, -Cl, -Br, -CN;
■ -OH, -ORᵢ mit Rᵢ ausgewählt aus C₁₋₄-Alkyl oder C₃₋₆-Cycloalkyl, -CF₃, -CHF₂, -CH₂F;
■ -NRⱼRₖ, wobei Rⱼ und Rₖ unabhängig ausgewählt sind aus -H und C₁-C₄-Alkyl
■ Phenyl oder 5- bis 6-gliedriges Heteroaryl, beide gegebenenfalls ein- oder mehrfach substituiert mit
• Halogen, -CN,
• -CF₃, -CHF₂, -CH₂F,
• C₁₋₄-Alkyl, gegebenenfalls substituiert mit einem oder mehreren Halogenen;
• C₃₋₆-Cycloalkyl;
• O-C₁₋₄-Alkyl,
• O-C₃₋₆-Cycloalkyl,
• C₁₋₃-Alkinyl,
• -NRⱼRₖ, wobei Rⱼ und Rₖ unabhängig ausgewählt sind aus -H und C₁-C₄-Alkyl
• SR_{d}, SOR_{d}, SO₂R_{d}, SONR_{d} oder SON(H)R_{d}, wobei R_{d} ausgewählt ist aus Methyl, Ethyl, Cyclopropyl, Isopropyl und tert-Butyl;
oder ein Stereoisomer, ein Tautomer, ein N-Oxid, ein Hydrat, ein Solvat oder ein Salz davon oder ein Gemisch davon.

9. Verbindung nach Anspruch 1, die ausgewählt ist aus:
(rac)-2'-[6-Amino-5-(trifluormethyl)pyridin-3-yl]-N-[1-(2-chlorphenyl)cyclobutyl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[2-(2-fluorphenyl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-(1-phenylcyclobutyl)-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-[2-(4-fluorphenyl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(2-chlorphenyl)propan-2-yl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[(1R)-1-(2-chlor-4-cyanophenyl)ethyl]-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-[(1R)-1-(2-chlor-4-cyanophenyl)ethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[(1S)-1-(3-chlorpyridin-4-yl)ethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(3-fluorphenyl)propan-2-yl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[1-(pyridin-4-yl)cyclobutyl]-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-[2-(3-chlorpyridin-4-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-(1-phenylcyclobutyl)-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[2-(pyridin-2-yl)propan-2-yl]-6,7-dihydrospiro[pyrazolo[5,1-c][1,4]-oxazin-4,3'-pyrrolidin]-1'-carboxamid
(rac)-2'-(5-Acetyl-6-aminopyridin-3-yl)-N-(1-phenylcyclobutyl)-5',6'-dihydrospiro[pyrrolidin-3,4'-pyrrolo[1,2-b]pyrazol]-1-carboxamid
(rac)-2-(6-Amino-5-cyanopyridin-3-yl)-N-[1-(2-chlorphenyl)cyclobutyl]-6,7-dihydrospiro[pyrazolo[5,1-c][1,4]-oxazin-4,3'-pyrrolidin]-1'-carboxamid
(rac)-2'-(6-Amino-5-cyanopyridin-3-yl)-N-[1-(2-chlorphenyl)cyclobutyl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[2-(3-chlorpyridin-4-yl)propan-2-yl]-6,7-dihydrospiro[pyrazolo[5,1-c][1,4]-oxazin-4,3'-pyrrolidin]-1'-carboxamid
(rac)-2-(6-Amino-5-cyanopyridin-3-yl)-N-[1-(pyridin-4-yl)cyclobutyl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[1-(2-fluorphenyl)cyclobutyl]-6,7-dihydrospiro[pyrazolo[5,1-c][1,4]-oxazin-4,3'-pyrrolidin]-1'-carboxamid
(rac)-2'-(6-Amino-5-cyanopyridin-3-yl)-N-(1-phenylcyclobutyl)-5',6'-dihydrospiro[pyrrolidin-3,4'-pyrrolo[1,2-b]pyrazol]-1-carboxamid
(rac)-2-(6-Amino-5-cyanopyridin-3-yl)-N-[1-(4-fluorphenyl)cyclobutyl]-6,7-dihydrospiro[pyrazolo[5,1-c][1,4]-oxazin-4,3'-pyrrolidin]-1'-carboxamid
(rac)-2'-(6-Amino-5-cyanopyridin-3-yl)-N-[1-(4-fluorphenyl)cyclobutyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[1-(2-fluorphenyl)cyclobutyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[1-(3-fluorphenyl)cyclobutyl]-5',6'-dihydrospiro[pyrrolidin-3,4'-pyrrolo[1,2-b]pyrazol]-1-carboxamid
(rac)-2'-(6-Amino-5-cyanopyridin-3-yl)-N-[2-(3-chlorpyridin-4-yl)propan-2-yl]-5',6'-dihydrospiro[pyrrolidin-3,4'-pyrrolo[1,2-b]pyrazol]-1-carboxamid
(rac)-2'-(6-Amino-5-cyanopyridin-3-yl)-N-[1-(pyridin-4-yl)cyclobutyl]-5',6'-dihydrospiro[pyrrolidin-3,4'-pyrrolo[1,2-b]pyrazol]-1-carboxamid
(rac)-2-(6-Amino-5-cyanopyridin-3-yl)-N-(1-phenylcyclobutyl)-6,7-dihydrospiro[pyrazolo[5,1-c][1,4]-oxazin-4,3'-pyrrolidin]-1'-carboxamid
(rac)-2-(6-Amino-5-cyanopyridin-3-yl)-N-[2-(4-fluorphenyl)propan-2-yl]-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-[1-(pyridin-4-yl)cyclobutyl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[1-(3-fluorphenyl)cyclobutyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-{(1R)-1-[3-(trifluormethyl)phenyl]ethyl}-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(3-fluorpyridin-2-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(pyridin-4-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(2-fluorphenyl)propan-2-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(3-fluorphenyl)ethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(pyridin-3-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[1-(pyridin-3-yl)cyclobutyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(4-fluorphenyl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-phenylpropyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-{(1S)-1-[3-(trifluormethyl)phenyl]ethyl}-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-phenylethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(pyridin-2-yl)propan-2-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(2-cyanophenyl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-(1,3,5-trimethyl-1H-pyrazol-4-yl)ethyl]-5',6'-dihydrospiro[pyrrolidin-3,4'-pyrrolo[1,2-b]pyrazol]-1-carboxamid (diastereomeres Gemisch)
2'-[6-Amino-5-(trifluormethyl)pyridin-3-yl]-N-[(1R)-2,3-dihydro-1H-inden-1-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(2-cyanopyridin-4-yl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-(1-ethyl-3,5-dimethyl-1H-pyrazol-4-yl)ethyl]-5',6'-dihydrospiro[pyrrolidin-3,4'-pyrrolo[1,2-b]pyrazol]-1-carboxamid (diastereomeres Gemisch)
(rac)-2'-[6-Amino-5-(trifluormethyl)pyridin-3-yl]-N-[1-(pyridin-2-yl)cyclobutyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-(1,3,4-trimethyl-1H-pyrazol-5-yl)ethyl]-5',6'-dihydrospiro[pyrrolidin-3,4'-pyrrolo[1,2-b]pyrazol]-1-carboxamid (diastereomeres Gemisch)
2'-[6-Amino-5-(trifluormethyl)pyridin-3-yl]-N-[(1R)-1-(4-cyanopyridin-2-yl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(pyridin-3-yl)ethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(1-methyl-1H-1,2,3-triazol-4-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-{[3-(trifluormethyl)phenyl]methyl}-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-(1-phenyl-1H-pyrazol-4-yl)ethyl]-5',6'-dihydrospiro[pyrrolidin-3,4'-pyrrolo[1,2-b]pyrazol]-1-carboxamid (diastereomeres Gemisch)
2'-[6-Amino-5-(trifluormethyl)pyridin-3-yl]-N-[(3S)-2,3-dihydro-1-benzofuran-3-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(6-cyanopyridin-3-yl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-{(1S)-1-[1-methyl-3-(trifluormethyl)-1H-pyrazol-4-yl]ethyl}-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(pyridin-2-yl)ethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-benzyl-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-phenylethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-(1,2-oxazol-3-yl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-(1,5-dimethyl-1H-pyrazol-4-yl)ethyl]-5',6'-dihydrospiro[pyrrolidin-3,4'-pyrrolo[1,2-b]pyrazol]-1-carboxamid (diastereomeres Gemisch)
(rac)-2'-[6-Amino-5-(trifluormethyl)pyridin-3-yl]-N-[2-(oxan-4-yl)propan-2-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(pyridin-4-yl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-(1-ethyl-5-methyl-1H-pyrazol-4-yl)ethyl]-5',6'-dihydrospiro[pyrrolidin-3,4'-pyrrolo[1,2-b]pyrazol]-1-carboxamid (diastereomeres Gemisch)
(rac)-2'-[6-Amino-5-(trifluormethyl)pyridin-3-yl]-N-[(pyridin-3-yl)methyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-2-methoxy-1-phenylethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-{[3-(trifluormethyl)bicyclo[1.1.1]-pentan-1-yl]methyl}-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[(pyridin-4-yl)methyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(3-cyanopyridin-2-yl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-(1-methyl-1H-benzimidazol-2-yl)ethyl]-5',6'-dihydrospiro[pyrrolidin-3,4'-pyrrolo[1,2-b]pyrazol]-1-carboxamid (diastereomeres Gemisch)
2'-[6-Amino-5-(trifluormethyl)pyridin-3-yl]-N-[1-(4-fluorphenyl)-2-methoxyethyl]-5',6'-dihydrospiro[pyrrolidin-3,4'-pyrrolo[1,2-b]pyrazol]-1-carboxamid (Isomerengemisch)
(rac)-2'-[6-Amino-5-(trifluormethyl)pyridin-3-yl]-N-[(pyridin-2-yl)methyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-(1-ethyl-1H-imidazol-2-yl)ethyl]-5',6'-dihydrospiro[pyrrolidin-3,4'-pyrrolo[1,2-b]pyrazol]-1-carboxamid (diastereomeres Gemisch)
2'-[6-Amino-5-(trifluormethyl)pyridin-3-yl]-N-[(1S)-1-(1-methyl-1H-imidazol-2-yl)ethyl]-5',6'-dihydrospiro[pyrrolidin-3,4'-pyrrolo[1,2-b]pyrazol]-1-carboxamid (diastereomeres Gemisch)
(rac)-2'-[6-Amino-5-(trifluormethyl)pyridin-3-yl]-N-[(pyrazin-2-yl)methyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-2-methoxy-1-(pyridin-3-yl)ethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[(pyridazin-4-yl)methyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[(1-methylpiperidin-4-yl)methyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[3-(methylsulfamoyl)pyridin-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[(1-methyl-2-oxabicyclo[2.1.1]-hexan-4-yl)methyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[(1-methyl-2-oxopiperidin-4-yl)methyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-(2-cyclopropylpropan-2-yl)-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(4-cyanophenyl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-methyl-2,3-dihydro-1H-inden-1-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[cyclopropyl(phenyl)methyl]-5',6'-dihydrospiro[pyrrolidin-3,4'-pyrrolo[1,2-b]pyrazol]-1-carboxamid (Isomerengemisch)
2'-[6-Amino-5-(trifluormethyl)pyridin-3-yl]-N-[-2-methyl-1-phenylpropyl]-5',6'-dihydrospiro[pyrrolidin-3,4'-pyrrolo[1,2-b]pyrazol]-1-carboxamid (Isomerengemisch)
2'-[6-Amino-5-(trifluormethyl)pyridin-3-yl]-N-[(1S)-1-(pyridin-4-yl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[2,2,2-trifluor-1-phenylethyl]-5',6'-dihydrospiro[pyrrolidin-3,4'-pyrrolo[1,2-b]pyrazol]-1-carboxamid (Isomerengemisch)
2'-[6-Amino-5-(trifluormethyl)pyridin-3-yl]-N-[1-(pyrazin-2-yl)ethyl]-5',6'-dihydrospiro[pyrrolidin-3,4'-pyrrolo[1,2-b]pyrazol]-1-carboxamid (Isomerengemisch)
2'-[6-Amino-5-(trifluormethyl)pyridin-3-yl]-N-[(1R)-2-methoxy-1-phenylethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[(pyridazin-3-yl)methyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[1-(2-cyanophenyl)cyclobutyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-2,3-dihydro-1H-inden-1-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-(4-fluorphenyl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-(2-cyanophenyl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(6-cyanopyridin-3-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(pyridin-2-yl)cyclobutyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(pyridin-4-yl)methyl]-5',6'-dihydrospiro[azetidin-3,4'-pyrrolo[1,2-b]pyrazol]-1-carboxamid
(3'RS)-2-[6-Amino-5-(trifluormethyl)pyridin-3-yl]-N-[(1R)-1-(4-cyanophenyl)ethyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-[(1R)-1-(3-cyanopyridin-2-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(4-cyanophenyl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(2-cyanopyridin-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(2-chlor-4-cyanophenyl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(2-fluorphenyl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(1-ethyl-3,5-dimethyl-1H-pyrazol-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(pyridin-2-yl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(1-methyl-1H-pyrazol-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(1,3,4-trimethyl-1H-pyrazol-5-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-{1-[3-(difluormethyl)-1-methyl-1H-pyrazol-4-yl]ethyl}-5',6'-dihydrospiro[azetidin-3,4'-pyrrolo[1,2-b]pyrazol]-1-carboxamid
(rac)-2-[6-Amino-5-(trifluormethyl)pyridin-3-yl]-N-[2-(pyridin-4-yl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[1-(pyridin-3-yl)cyclobutyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-[1-(1-ethyl-5-methyl-1H-pyrazol-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(1,5-dimethyl-1H-pyrazol-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(1,3,5-trimethyl-1H-pyrazol-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(pyridin-3-yl)ethyl]-5',6'-dihydrospiro[azetidin-3,4'-pyrrolo[1,2-b]pyrazol]-1-carboxamid
2-[6-Amino-5-(trifluormethyl)pyridin-3-yl]-N-[(1R)-1-(2-cyanopyridin-4-yl)ethyl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1R)-1-(3-cyanopyridin-2-yl)ethyl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1R)-1-(6-cyanopyridin-3-yl)ethyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-[(pyridin-2-yl)methyl]-5',6'-dihydrospiro[azetidin-3,4'-pyrrolo[1,2-b]pyrazol]-1-carboxamid
(rac)-2-[6-Amino-5-(trifluormethyl)pyridin-3-yl]-N-[(1R)-1-(2-cyanophenyl)ethyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-[(1R)-1-(4-fluorphenyl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(4-fluorphenyl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(pyridin-3-yl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(2-fluorphenyl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(pyridin-3-yl)cyclobutyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(4-fluorphenyl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(pyridin-4-yl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(3-fluorphenyl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1S)-1-(3-chlorpyridin-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1-methyl-2-oxabicyclo[2.1.1]-hexan-4-yl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-{[3-(trifluormethyl)bicyclo[1.1.1]-pentan-1-yl]methyl}-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(2-chlorphenyl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(3-fluorphenyl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(3-fluorpyridin-2-yl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(2-chlorphenyl)cyclobutyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(3-fluorphenyl)cyclobutyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-phenylpropyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(pyrimidin-4-yl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(3-fluorpyridin-2-yl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(pyrimidin-5-yl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(3-chlorpyridin-4-yl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(3-fluorphenyl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(pyridin-3-yl)methyl]-5',6'-dihydrospiro[azetidin-3,4'-pyrrolo[1,2-b]pyrazol]-1-carboxamid
(rac)-2-[6-Amino-5-(trifluormethyl)pyridin-3-yl]-N-benzyl-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[(1R)-1-(2-methylpyrimidin-5-yl)ethyl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[(1R)-1-(pyridin-3-yl)ethyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-benzyl-5',6'-dihydrospiro[azetidin-3,4'-pyrrolo[1,2-b]pyrazol]-1-carboxamid
2-[6-Amino-5-(trifluormethyl)pyridin-3-yl]-N-[2-(2-fluorphenyl)propan-2-yl]-6,7-dihydrospiro[pyrazolo[5,1-c][1,4]-oxazin-4,3'-pyrrolidin]-1'-carboxamid (Enantiomer 1)
2-[6-Amino-5-(trifluormethyl)pyridin-3-yl]-N-[2-(3-chlorpyridin-4-yl)propan-2-yl]-6,7-dihydrospiro[pyrazolo[5,1-c][1,4]-oxazin-4,3'-pyrrolidin]-1'-carboxamid (Enantiomer 1)
2-[6-Amino-5-(trifluormethyl)pyridin-3-yl]-N-[2-(2-fluorphenyl)propan-2-yl]-6,7-dihydrospiro[pyrazolo[5,1-c][1,4]-oxazin-4,3'-pyrrolidin]-1'-carboxamid (Enantiomer 2)
2-[6-Amino-5-(trifluormethyl)pyridin-3-yl]-N-[2-(3-chlorpyridin-4-yl)propan-2-yl]-6,7-dihydrospiro[pyrazolo[5,1-c][1,4]-oxazin-4,3'-pyrrolidin]-1'-carboxamid (Enantiomer 2)
2-[6-Amino-5-(trifluormethyl)pyridin-3-yl]-N-[(1R)-1-(4-cyanopyridin-2-yl)ethyl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[2-(1-methyl-1H-1,2,3-triazol-5-yl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[2-(pyridin-3-yl)propan-2-yl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1R)-1-(4-fluorphenyl)ethyl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1R)-2,3-dihydro-1H-inden-1-yl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1R)-1-(5-fluorpyridin-3-yl)ethyl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1R)-1-(3-fluorphenyl)ethyl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1R)-1-phenylpropyl]-6,7-dihydrospiro[pyrazolo[5,1-c][1,4]-oxazin-4,3'-pyrrolidin]-1'-carboxamid
(3'RS)-2-[6-Amino-5-(trifluormethyl)pyridin-3-yl]-N-[(3S)-2,3-dihydro-1-benzofuran-3-yl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[(bicyclo[1.1.1]-pentan-1-yl)methyl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1S)-2-methoxy-1-phenylethyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-[(1R)-1-(1,2-oxazol-3-yl)ethyl]-5',6'-dihydrospiro[azetidin-3,4'-pyrrolo[1,2-b]pyrazol]-1-carboxamid
2-[6-Amino-5-(trifluormethyl)pyridin-3-yl]-N-[(1R)-1-(2-fluorphenyl)ethyl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[2-(3-fluorphenyl)propan-2-yl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-[(1R)-1-(1-phenyl-1H-pyrazol-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1S)-2-methoxy-1-phenylethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(3-fluorbicyclo[1.1.1]-pentan-1-yl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(bicyclo[1.1.1]-pentan-1-yl)methyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1S)-1-(pyridin-3-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(2-methylpyrimidin-5-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(1-methyl-1H-1,2,3-triazol-4-yl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[2-(3-chlorpyridin-4-yl)propan-2-yl]-5',6'-dihydrospiro[azetidin-3,4'-pyrrolo[1,2-b]pyrazol]-1-carboxamid
2'-[6-Amino-5-(trifluormethyl)pyridin-3-yl]-N-[1-(pyridin-4-yl)cyclobutyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-phenylethyl]-5',6'-dihydrospiro[azetidin-3,4'-pyrrolo[1,2-b]pyrazol]-1-carboxamid
2-[6-Amino-5-(trifluormethyl)pyridin-3-yl]-N-[(1S)-1-(pyridin-3-yl)ethyl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[(3-fluorbicyclo[1.1.1]-pentan-1-yl)methyl]-6,7-dihydrospiro[pyrazolo[5,1-c][1,4]-oxazin-4,3'-pyrrolidin]-1'-carboxamid
2-[6-Amino-5-(trifluormethyl)pyridin-3-yl]-N-[(1S)-1-(pyridin-4-yl)ethyl]-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-(1-phenylcyclobutyl)-5',6'-dihydrospiro[azetidin-3,4'-pyrrolo[1,2-b]pyrazol]-1-carboxamid
2'-(6-Amino-5-cyanopyridin-3-yl)-N-[1-(pyridin-4-yl)cyclobutyl]-5',6'-dihydrospiro[azetidin-3,4'-pyrrolo[1,2-b]pyrazol]-1-carboxamid
2'-(6-Amino-5-cyanopyridin-3-yl)-N-[1-(3-fluorphenyl)cyclobutyl]-5',6'-dihydrospiro[azetidin-3,4'-pyrrolo[1,2-b]pyrazol]-1-carboxamid
2'-(6-Amino-5-cyanopyridin-3-yl)-N-[2-(3-chlorpyridin-4-yl)propan-2-yl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-(5-fluorpyridin-3-yl)ethyl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-(4-fluorphenyl)ethyl]-5',6'-dihydrospiro[azetidin-3,4'-pyrrolo[1,2-b]pyrazol]-1-carboxamid
2'-(6-Amino-5-cyanopyridin-3-yl)-N-[2-(3-fluorphenyl)propan-2-yl]-5',6'-dihydrospiro[azetidin-3,4'-pyrrolo[1,2-b]pyrazol]-1-carboxamid
2'-(6-Amino-5-cyanopyridin-3-yl)-N-[(1S)-1-(3-chlorpyridin-4-yl)ethyl]-5',6'-dihydrospiro[azetidin-3,4'-pyrrolo[1,2-b]pyrazol]-1-carboxamid
2'-(6-Amino-5-cyanopyridin-3-yl)-N-[(1-methyl-2-oxabicyclo[2.1.1]hexan-4-yl)methyl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-(4-cyanopyridin-2-yl)ethyl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-(2-cyanopyridin-4-yl)ethyl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-(2-Chlor-4-cyanophenyl)ethyl]-5',6'-dihydrospiro[azetidin-3,4'-pyrrolo[1,2-b]pyrazol]-1-carboxamid
2'-(6-Amino-5-cyanopyridin-3-yl)-N-[1-(2-fluorphenyl)cyclobutyl]-5',6'-dihydrospiro[azetidin-3,4'-pyrrolo[1,2-b]pyrazol]-1-carboxamid
2'-(6-Amino-5-cyanopyridin-3-yl)-N-[2-(4-fluorphenyl)propan-2-yl]-5',6'-dihydrospiro[azetidin-3,4'-pyrrolo[1,2-b]pyrazol]-1-carboxamid
2'-(6-Amino-5-cyanopyridin-3-yl)-N-[1-(2-chlorphenyl)cyclobutyl]-5',6'-dihydrospiro[azetidin-3,4'-pyrrolo[1,2-b]pyrazol]-1-carboxamid
2'-(6-Amino-5-cyanopyridin-3-yl)-N-[2-(1-methyl-1H-1,2,3-triazol-4-yl)propan-2-yl]-5',6'-dihydrospiro[azetidin-3,4'-pyrrolo[1,2-b]pyrazol]-1-carboxamid
2'-(6-Amino-5-cyanopyridin-3-yl)-N-[2-(pyridin-2-yl)propan-2-yl]-5',6'-dihydrospiro[azetidin-3,4'-pyrrolo[1,2-b]pyrazol]-1-carboxamid
2'-(6-Amino-5-cyanopyridin-3-yl)-N-[2-(pyridin-4-yl)propan-2-yl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-phenylpropyl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-(3-fluorphenyl)ethyl]-5',6'-dihydrospiro[azetidin-3,4'-pyrrolo[1,2-b]pyrazol]-1-carboxamid
2'-(6-Amino-5-cyanopyridin-3-yl)-N-[(1R)-1-(2-cyanophenyl)ethyl]-5',6'-dihydrospiro[azetidin-3,4'-pyrrolo[1,2-b]pyrazol]-1-carboxamid
(rac)-2'-(6-Amino-5-cyanopyridin-3-yl)-N-[2-(pyridin-4-yl)propan-2-yl]-5',6'-dihydrospiro[pyrrolidin-3,4'-pyrrolo[1,2-b]pyrazol]-1-carboxamid
2'-(6-Amino-5-cyanopyridin-3-yl)-N-[(1R)-1-(5-fluorpyridin-3-yl)ethyl]-5',6'-dihydrospiro[pyrrolidin-3,4'-pyrrolo[1,2-b]pyrazol]-1-carboxamid
(rac)-2'-(6-Amino-5-cyanopyridin-3-yl)-N-[2-(1-methyl-1H-1,2,3-triazol-4-yl)propan-2-yl]-5',6'-dihydrospiro[pyrrolidin-3,4'-pyrrolo[1,2-b]pyrazol]-1-carboxamid
(rac)-2-(6-Amino-5-cyanopyridin-3-yl)-N-[1-(pyridin-2-yl)cyclobutyl]-6,7-dihydrospiro[pyrazolo[5,1-c][1,4]-oxazin-4,3'-pyrrolidin]-1'-carboxamid
(rac)-2'-(6-Amino-5-cyanopyridin-3-yl)-N-[1-(pyridin-2-yl)cyclobutyl]-5',6'-dihydrospiro[pyrrolidin-3,4'-pyrrolo[1,2-b]pyrazol]-1-carboxamid
(rac)-2'-(6-Amino-5-cyanopyridin-3-yl)-N-[1-(2-chlorphenyl)cyclobutyl]-5',6'-dihydrospiro[pyrrolidin-3,4'-pyrrolo[1,2-b]pyrazol]-1-carboxamid
2-(6-Amino-5-cyanopyridin-3-yl)-N-[(1R)-1-phenylethyl]-6,7-dihydrospiro[pyrazolo[5,1-c][1,4]-oxazin-4,3'-pyrrolidin]-1'-carboxamid
2'-(6-Amino-5-cyanopyridin-3-yl)-N-[(1R)-1-phenylethyl]-5',6'-dihydrospiro[pyrrolidin-3,4'-pyrrolo[1,2-b]pyrazol]-1-carboxamid
(rac)-2-(6-Amino-5-cyanopyridin-3-yl)-N-[1-(2-chlorphenyl)cyclobutyl]-6,7-dihydrospiro[pyrazolo[5,1-c][1,4]-oxazin-4,3'-pyrrolidin]-1'-carboxamid
(rac)-2-(6-Amino-5-cyanopyridin-3-yl)-N-[1-(4-fluorphenyl)cyclobutyl]-6,7-dihydrospiro[pyrazolo[5,1-c][1,4]-oxazin-4,3'-pyrrolidin]-1'-carboxamid
(rac)-2-(6-Amino-5-cyanopyridin-3-yl)-N-[2-(4-fluorphenyl)propan-2-yl]-6,7-dihydrospiro[pyrazolo[5,1-c][1,4]-oxazin-4,3'-pyrrolidin]-1'-carboxamid
(rac)-2-(6-Amino-5-cyanopyridin-3-yl)-N-[2-(pyridin-2-yl)propan-2-yl]-6,7-dihydrospiro[pyrazolo[5,1-c][1,4]-oxazin-4,3'-pyrrolidin]-1'-carboxamid
(rac)-2'-(6-Amino-5-cyanopyridin-3-yl)-N-[2-(pyridin-2-yl)propan-2-yl]-5',6'-dihydrospiro[pyrrolidin-3,4'-pyrrolo[1,2-b]pyrazol]-1-carboxamid
2-(6-Amino-5-cyanopyridin-3-yl)-N-[1-(pyridin-4-yl)cyclobutyl]-6,7-dihydrospiro[pyrazolo[5,1-c][1,4]-oxazin-4,3'-pyrrolidin]-1'-carboxamid (Enantiomer 2)
2-(6-Amino-5-cyanopyridin-3-yl)-N-[1-(2-chlorphenyl)cyclobutyl]-6,7-dihydrospiro[pyrazolo[5,1-c][1,4]-oxazin-4,3'-pyrrolidin]-1'-carboxamid (Enantiomer 2)
2-(6-Amino-5-cyanopyridin-3-yl)-N-[2-(pyridin-2-yl)propan-2-yl]-6,7-dihydrospiro[pyrazolo[5,1-c][1,4]-oxazin-4,3'-pyrrolidin]-1'-carboxamid (Enantiomer 1)
2-(6-Amino-5-cyanopyridin-3-yl)-N-[1-(2-chlorphenyl)cyclobutyl]-6,7-dihydrospiro[pyrazolo[5,1-c][1,4]-oxazin-4,3'-pyrrolidin]-1'-carboxamid (Enantiomer 1)
2'-(6-Amino-5-cyanopyridin-3-yl)-N-[1-(pyridin-3-yl)cyclobutyl]-5',6'-dihydrospiro[azetidin-3,4'-pyrrolo[1,2-b]pyrazol]-1-carboxamid
2'-(6-Amino-5-cyanopyridin-3-yl)-N-[1-(pyridin-2-yl)cyclobutyl]-5',6'-dihydrospiro[azetidin-3,4'-pyrrolo[1,2-b]pyrazol]-1-carboxamid
2-(6-Amino-5-cyanopyridin-3-yl)-N-[1-(pyridin-4-yl)cyclobutyl]-6,7-dihydrospiro[pyrazolo[5,1-c][1,4]-oxazin-4,3'-pyrrolidin]-1'-carboxamid (Enantiomer 1)
2'-(6-Amino-5-cyanopyridin-3-yl)-N-(1-phenylcyclobutyl)-5',6'-dihydrospiro[azetidin-3,4'-pyrrolo[1,2-b]pyrazol]-1-carboxamid
2'-(6-Amino-5-cyanopyridin-3-yl)-N-[(3S)-2,3-dihydro-1-benzofuran-3-yl]-5',6'-dihydrospiro[pyrrolidin-3,4'-pyrrolo[1,2-b]pyrazol]-1-carboxamid
2-(6-Amino-5-cyanopyridin-3-yl)-N-[(3S)-2,3-dihydro-1-benzofuran-3-yl]-6,7-dihydrospiro[pyrazolo[5,1-c][1,4]-oxazin-4,3'-pyrrolidin]-1'-carboxamid
2-(6-Amino-5-cyanopyridin-3-yl)-N-[(1R)-1-(2-fluorphenyl)ethyl]-6,7-dihydrospiro[pyrazolo[5,1-c][1,4]-oxazin-4,3'-pyrrolidin]-1'-carboxamid
2'-(6-Amino-5-cyanopyridin-3-yl)-N-[(1R)-1-(2-fluorphenyl)ethyl]-5',6'-dihydrospiro[pyrrolidin-3,4'-pyrrolo[1,2-b]pyrazol]-1-carboxamid
(rac)-2'-(6-Amino-5-cyanopyridin-3-yl)-N-[2-(4-fluorphenyl)propan-2-yl]-5',6'-dihydrospiro[pyrrolidin-3,4'-pyrrolo[1,2-b]pyrazol]-1-carboxamid
(rac)-2-(6-Amino-5-cyanopyridin-3-yl)-N-[2-(1-methyl-1H-1,2,3-triazol-4-yl)propan-2-yl]-6,7-dihydrospiro[pyrazolo[5,1-c][1,4]-oxazin-4,3'-pyrrolidin]-1'-carboxamid
(rac)-2-(6-Amino-5-cyanopyridin-3-yl)-N-[(1R)-1-(5-fluorpyridin-3-yl)ethyl]-6,7-dihydrospiro[pyrazolo[5,1-c][1,4]-oxazin-4,3'-pyrrolidin]-1'-carboxamid
(rac)-2'-(6-Amino-5-cyanopyridin-3-yl)-N-(1-phenylcyclobutyl)-5',6'-dihydrospiro[pyrrolidin-3,4'-pyrrolo[1,2-b]pyrazol]-1-carboxamid
(rac)-2'-(6-Amino-5-cyanopyridin-3-yl)-N-[1-(4-fluorphenyl)cyclobutyl]-5',6'-dihydrospiro[pyrrolidin-3,4'-pyrrolo[1,2-b]pyrazol]-1-carboxamid
(rac)-2-(6-Amino-5-cyanopyridin-3-yl)-N-[1-(pyridin-4-yl)cyclobutyl]-6,7-dihydrospiro[pyrazolo[5,1-c][1,4]-oxazin-4,3'-pyrrolidin]-1'-carboxamid
(rac)-2'-(6-Amino-5-cyanopyridin-3-yl)-N-[1-(pyridin-4-yl)cyclobutyl]-5',6'-dihydrospiro[pyrrolidin-3,4'-pyrrolo[1,2-b]pyrazol]-1-carboxamid
(rac)-2-(6-Amino-5-cyanopyridin-3-yl)-N-(1-phenylcyclobutyl)-6,7-dihydrospiro[pyrazolo[5,1-c][1,4]-oxazin-4,3'-pyrrolidin]-1'-carboxamid
2'-(6-Amino-5-cyanopyridin-3-yl)-N-[(1R)-1-(1,3,4-trimethyl-1H-pyrazol-5-yl)ethyl]-5',6'-dihydrospiro[azetidin-3,4'-pyrrolo[1,2-b]pyrazol]-1-carboxamid
(rac)-2-(6-Amino-5-cyanopyridin-3-yl)-N-[2-(pyridin-4-yl)propan-2-yl]-6,7-dihydrospiro[pyrazolo[5,1-c][1,4]-oxazin-4,3'-pyrrolidin]-1'-carboxamid
2'-[6-Amino-5-(difluormethoxy)pyridin-3-yl]-N-[1-(pyridin-3-yl)cyclobutyl]-5',6'-dihydrospiro[azetidin-3,4'-pyrrolo[1,2-b]pyrazol]-1-carboxamid
(rac)-2'-[6-Amino-5-(trifluormethoxy)pyridin-3-yl]-N-[2-(1-methyl-1H-1,2,3-triazol-4-yl)propan-2-yl]-5',6'-dihydrospiro[pyrrolidin-3,4'-pyrrolo[1,2-b]pyrazol]-1-carboxamid
2'-[6-Amino-5-(trifluormethoxy)pyridin-3-yl]-N-[(1R)-1-(2-methylpyrimidin-5-yl)ethyl]-5',6'-dihydrospiro[pyrrolidin-3,4'-pyrrolo[1,2-b]pyrazol]-1-carboxamid
2'-[6-Amino-5-(trifluormethoxy)pyridin-3-yl]-N-[(1R)-1-(pyridin-2-yl)ethyl]-5',6'-dihydrospiro[pyrrolidin-3,4'-pyrrolo[1,2-b]pyrazol]-1-carboxamid
2'-[6-Amino-5-(trifluormethoxy)pyridin-3-yl]-N-[(1R)-1-phenylethyl]-5',6'-dihydrospiro[pyrrolidin-3,4'-pyrrolo[1,2-b]pyrazol]-1-carboxamid (Gemisch von Stereoisomer)
2'-[6-Amino-5-(trifluormethoxy)pyridin-3-yl]-N-[(1R)-1-(pyridin-3-yl)ethyl]-5',6'-dihydrospiro[pyrrolidin-3,4'-pyrrolo[1,2-b]pyrazol]-1-carboxamid
2'-[6-Amino-5-(trifluormethoxy)pyridin-3-yl]-N-[(1S)-1-(pyridin-4-yl)ethyl]-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-[2-(4-fluorphenyl)propan-2-yl]-5',6'-dihydrospiro[pyrrolidin-3,4'-pyrrolo[1,2-b]pyrazol]-1-carboxamid
2'-[6-Amino-5-(trifluormethoxy)pyridin-3-yl]-N-[(1R)-1-(4-fluorphenyl)ethyl]-5',6'-dihydrospiro[pyrrolidin-3,4'-pyrrolo[1,2-b]pyrazol]-1-carboxamid
(rac)-2-[6-Amino-5-(trifluormethoxy)pyridin-3-yl]-N-[2-(pyridin-4-yl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[1-(pyridin-4-yl)cyclobutyl]-6,7-dihydrospiro[pyrazolo[5,1-c][1,4]-oxazin-4,3'-pyrrolidin]-1'-carboxamid
2'-[6-Amino-5-(difluormethoxy)pyridin-3-yl]-N-(1-phenylcyclobutyl)-5',6'-dihydrospiro[azetidin-3,4'-pyrrolo[1,2-b]pyrazol]-1-carboxamid
(rac)-2'-[6-Amino-5-(trifluormethoxy)pyridin-3-yl]-N-[2-(pyridin-2-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-[2-(3-chlorpyridin-4-yl)propan-2-yl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[2-(2-fluorphenyl)propan-2-yl]-5',6'-dihydrospiro[azetidin-3,4'-pyrrolo[1,2-b]pyrazol]-1-carboxamid
(rac)-2-[6-Amino-5-(difluormethoxy)pyridin-3-yl]-N-[2-(3-fluorphenyl)propan-2-yl]-6,7-dihydrospiro[pyrazolo[5,1-c][1,4]-oxazin-4,3'-pyrrolidin]-1'-carboxamid
2-[6-Amino-5-(difluormethoxy)pyridin-3-yl]-N-[(1S)-1-(3-chlorpyridin-4-yl)ethyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[1-(1,3,5-trimethyl-1H-pyrazol-4-yl)ethyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[1-(1-ethyl-3,5-dimethyl-1H-pyrazol-4-yl)ethyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[(1R)-1-(2-cyanophenyl)ethyl]-6,7-dihydrospiro[pyrazolo[5,1-c][1,4]-oxazin-4,3'-pyrrolidin]-1'-carboxamid
(rac)-2'-[6-Amino-5-(difluormethoxy)pyridin-3-yl]-N-[2-(pyridin-2-yl)propan-2-yl]-5',6'-dihydrospiro[pyrrolidin-3,4'-pyrrolo[1,2-b]pyrazol]-1-carboxamid
2-[6-Amino-5-(trifluormethoxy)pyridin-3-yl]-N-[(1R)-1-(4-fluorphenyl)ethyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[(1R)-1-phenylpropyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[(1R)-1-phenylethyl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[1-(pyridin-3-yl)cyclobutyl]-6,7-dihydrospiro[pyrazolo[5,1-c][1,4]-oxazin-4,3'-pyrrolidin]-1'-carboxamid
2-[6-Amino-5-(difluormethoxy)pyridin-3-yl]-N-[(1R)-1-(2-chlor-4-cyanophenyl)ethyl]-6,7-dihydrospiro[pyrazolo[5,1-c][1,4]-oxazin-4,3'-pyrrolidin]-1'-carboxamid
(rac)-2-[6-Amino-5-(difluormethoxy)pyridin-3-yl]-N-[2-(4-fluorphenyl)propan-2-yl]-6,7-dihydrospiro[pyrazolo[5,1-c][1,4]-oxazin-4,3'-pyrrolidin]-1'-carboxamid
(rac)-2-[6-Amino-5-(difluormethoxy)pyridin-3-yl]-N-[1-(2-fluorphenyl)cyclobutyl]-6,7-dihydrospiro[pyrazolo[5,1-c][1,4]-oxazin-4,3'-pyrrolidin]-1'-carboxamid
(rac)-2'-[6-Amino-5-(difluormethoxy)pyridin-3-yl]-N-[2-(pyridin-3-yl)propan-2-yl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[1-(2-chlorphenyl)cyclobutyl]-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1R)-1-(4-cyanophenyl)ethyl]-5',6'-dihydrospiro[azetidin-3,4'-pyrrolo[1,2-b]pyrazol]-1-carboxamid
2'-[6-Amino-5-(difluormethoxy)pyridin-3-yl]-N-[(1R)-1-(1,3,4-trimethyl-1H-pyrazol-5-yl)ethyl]-5',6'-dihydrospiro[azetidin-3,4'-pyrrolo[1,2-b]pyrazol]-1-carboxamid
(rac)-2'-[6-Amino-5-(trifluormethoxy)pyridin-3-yl]-N-[1-(2-chlorphenyl)cyclobutyl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[(1R)-1-(5-fluorpyridin-3-yl)ethyl]-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1R)-1-(4-fluorphenyl)ethyl]-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1R)-1-(2-chlor-4-cyanophenyl)ethyl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[(1R)-1-(2-cyanophenyl)ethyl]-5',6'-dihydrospiro[azetidin-3,4'-pyrrolo[1,2-b]pyrazol]-1-carboxamid
2'-[6-Amino-5-(difluormethoxy)pyridin-3-yl]-N-[2-(pyridin-4-yl)propan-2-yl]-5',6'-dihydrospiro[azetidin-3,4'-pyrrolo[1,2-b]pyrazol]-1-carboxamid
2'-[6-Amino-5-(difluormethoxy)pyridin-3-yl]-N-[(1R)-1-(4-fluorphenyl)ethyl]-5',6'-dihydrospiro[azetidin-3,4'-pyrrolo[1,2-b]pyrazol]-1-carboxamid
2'-[6-Amino-5-(difluormethoxy)pyridin-3-yl]-N-[(1R)-1-(2-cyanopyridin-4-yl)ethyl]-5',6'-dihydrospiro[azetidin-3,4'-pyrrolo[1,2-b]pyrazol]-1-carboxamid
(rac)-2'-[6-Amino-5-(trifluormethoxy)pyridin-3-yl]-N-[1-(2-fluorphenyl)cyclobutyl]-5',6'-dihydrospiro[pyrrolidin-3,4'-pyrrolo[1,2-b]pyrazol]-1-carboxamid
(rac)-2-[6-Amino-5-(trifluormethoxy)pyridin-3-yl]-N-[2-(2-chlorphenyl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[2-(4-fluorphenyl)propan-2-yl]-6,7-dihydrospiro[pyrazolo[5,1-c][1,4]-oxazin-4,3'-pyrrolidin]-1'-carboxamid
(rac)-2'-[6-Amino-5-(trifluormethoxy)pyridin-3-yl]-N-[2-(3-fluorphenyl)propan-2-yl]-5',6'-dihydrospiro[pyrrolidin-3,4'-pyrrolo[1,2-b]pyrazol]-1-carboxamid
2'-[6-Amino-5-(trifluormethoxy)pyridin-3-yl]-N-[(1S)-1-(3-chlorpyridin-4-yl)ethyl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[2-(4-fluorphenyl)propan-2-yl]-5',6'-dihydrospiro[azetidin-3,4'-pyrrolo[1,2-b]pyrazol]-1-carboxamid
2'-[6-Amino-5-(difluormethoxy)pyridin-3-yl]-N-[2-(2-chlorphenyl)propan-2-yl]-5',6'-dihydrospiro[azetidin-3,4'-pyrrolo[1,2-b]pyrazol]-1-carboxamid
2-[6-Amino-5-(trifluormethoxy)pyridin-3-yl]-N-[(1R)-1-(3-fluorphenyl)ethyl]-6,7-dihydrospiro[pyrazolo[5,1-c][1,4]-oxazin-4,3'-pyrrolidin]-1'-carboxamid
2'-[6-Amino-5-(trifluormethoxy)pyridin-3-yl]-N-(1-phenylcyclobutyl)-5',6'-dihydrospiro[azetidin-3,4'-pyrrolo[1,2-b]pyrazol]-1-carboxamid
2'-[6-Amino-5-(trifluormethoxy)pyridin-3-yl]-N-benzyl-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1R)-1-(4-cyanopyridin-2-yl)ethyl]-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1R)-1-(2-cyanopyridin-4-yl)ethyl]-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1R)-1-(5-fluorpyridin-3-yl)ethyl]-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-[2-(pyridin-4-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-(1-phenylcyclobutyl)-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-[1-(pyridin-4-yl)cyclobutyl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[2-(3-fluorphenyl)propan-2-yl]-5',6'-dihydrospiro[azetidin-3,4'-pyrrolo[1,2-b]pyrazol]-1-carboxamid
2'-[6-Amino-5-(difluormethoxy)pyridin-3-yl]-N-[(1S)-1-(3-chlorpyridin-4-yl)ethyl]-5',6'-dihydrospiro[azetidin-3,4'-pyrrolo[1,2-b]pyrazol]-1-carboxamid
2'-[6-Amino-5-(difluormethoxy)pyridin-3-yl]-N-[2-(3-chlorpyridin-4-yl)propan-2-yl]-5',6'-dihydrospiro[azetidin-3,4'-pyrrolo[1,2-b]pyrazol]-1-carboxamid
2'-[6-Amino-5-(difluormethoxy)pyridin-3-yl]-N-[(1R)-1-(2-chlor-4-cyanophenyl)ethyl]-5',6'-dihydrospiro[azetidin-3,4'-pyrrolo[1,2-b]pyrazol]-1-carboxamid
2'-[6-Amino-5-(difluormethoxy)pyridin-3-yl]-N-[1-(2-fluorphenyl)cyclobutyl]-5',6'-dihydrospiro[azetidin-3,4'-pyrrolo[1,2-b]pyrazol]-1-carboxamid
(rac)-2'-[6-Amino-5-(trifluormethoxy)pyridin-3-yl]-N-[2-(2-chlorphenyl)propan-2-yl]-5',6'-dihydrospiro[pyrrolidin-3,4'-pyrrolo[1,2-b]pyrazol]-1-carboxamid
2-[6-Amino-5-(trifluormethoxy)pyridin-3-yl]-N-[(1R)-1-(4-cyanopyridin-2-yl)ethyl]-6,7-dihydrospiro[pyrazolo[5,1-c][1,4]-oxazin-4,3'-pyrrolidin]-1'-carboxamid
(rac)-2-[6-Amino-5-(difluormethoxy)pyridin-3-yl]-N-[1-(2-chlorphenyl)cyclobutyl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[1-(pyridin-2-yl)cyclobutyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[(1R)-1-(2-cyanopyridin-4-yl)ethyl]-6,7-dihydrospiro[pyrazolo[5,1-c][1,4]-oxazin-4,3'-pyrrolidin]-1'-carboxamid
(rac)-2-[6-Amino-5-(difluormethoxy)pyridin-3-yl]-N-[2-(2-chlorphenyl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[1-(2-chlorphenyl)cyclobutyl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[1-(2-fluorphenyl)cyclobutyl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[2-(3-chlorpyridin-4-yl)propan-2-yl]-6,7-dihydrospiro[pyrazolo[5,1-c][1,4]-oxazin-4,3'-pyrrolidin]-1'-carboxamid
(rac)-2'-[6-Amino-5-(trifluormethoxy)pyridin-3-yl]-N-[1-(pyridin-4-yl)cyclobutyl]-5',6'-dihydro-1H-spiro[pyrrolidin-3,4'-pyrrolo[1,2-b]pyrazol]-1-carboxamid-acetat (1:1)
(rac)-2-[6-Amino-5-(trifluormethoxy)pyridin-3-yl]-N-[(1-methyl-2-oxabicyclo[2.1.1]hexan-4-yl)methyl]-6,7-dihydrospiro[pyrazolo[5,1-c][1,4]oxazin-4,3'-pyrrolidin]-1'-carboxamid
(rac)-2-[6-Amino-5-(trifluormethoxy)pyridin-3-yl]-N-[2-(2-fluorphenyl)propan-2-yl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[(1R)-1-(2-chlor-4-cyanophenyl)ethyl]-6,7-dihydrospiro[pyrazolo[5,1-c][1,4]-oxazin-4,3'-pyrrolidin]-1'-carboxamid
2'-[6-Amino-5-(difluormethoxy)pyridin-3-yl]-N-[2-(pyridin-2-yl)propan-2-yl]-5',6'-dihydrospiro[azetidin-3,4'-pyrrolo[1,2-b]pyrazol]-1-carboxamid
2'-[6-Amino-5-(difluormethoxy)pyridin-3-yl]-N-[2-(1-methyl-1H-1,2,3-triazol-4-yl)propan-2-yl]-5',6'-dihydrospiro[azetidin-3,4'-pyrrolo[1,2-b]pyrazol]-1-carboxamid
2-[6-Amino-5-(trifluormethoxy)pyridin-3-yl]-N-[(1S)-2-methoxy-1-phenylethyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[(1S)-1-(3-chlorpyridin-4-yl)ethyl]-6,7-dihydrospiro[pyrazolo[5,1-c][1,4]-oxazin-4,3'-pyrrolidin]-1'-carboxamid
(rac)-2-[6-Amino-5-(trifluormethoxy)pyridin-3-yl]-N-[2-(3-fluorphenyl)propan-2-yl]-6,7-dihydrospiro[pyrazolo[5,1-c][1,4]-oxazin-4,3'-pyrrolidin]-1'-carboxamid
(rac)-2'-[6-Amino-5-(trifluormethoxy)pyridin-3-yl]-N-[(1-methyl-2-oxabicyclo[2.1.1]hexan-4-yl)methyl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[(1R)-1-phenylpropyl]-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1S)-2-methoxy-1-phenylethyl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[(1R)-1-(3-fluorphenyl)ethyl]-5',6'-dihydrospiro[azetidin-3,4'-pyrrolo[1,2-b]pyrazol]-1-carboxamid
2'-[6-Amino-5-(trifluormethoxy)pyridin-3-yl]-N-[(1S)-1-(pyridin-3-yl)ethyl]-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-[1-(pyridin-3-yl)cyclobutyl]-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-[1-(3-fluorphenyl)cyclobutyl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[1-(propan-2-yl)cyclobutyl]-5',6'-dihydrospiro[azetidin-3,4'-pyrrolo[1,2-b]pyrazol]-1-carboxamid
2'-[6-Amino-5-(difluormethoxy)pyridin-3-yl]-N-[1-(pyridin-4-yl)cyclobutyl]-5',6'-dihydrospiro[azetidin-3,4'-pyrrolo[1,2-b]pyrazol]-1-carboxamid
2'-[6-Amino-5-(difluormethoxy)pyridin-3-yl]-N-(bicyclo[2.2.1]-heptan-1-yl)-5',6'-dihydrospiro[azetidin-3,4'-pyrrolo[1,2-b]pyrazol]-1-carboxamid
2'-[6-Amino-5-(difluormethoxy)pyridin-3-yl]-N-(1-benzylcyclobutyl)-5',6'-dihydrospiro[azetidin-3,4'-pyrrolo[1,2-b]pyrazol]-1-carboxamid
2'-[6-Amino-5-(difluormethoxy)pyridin-3-yl]-N-[1-(4-chlorphenyl)cyclobutyl]-5',6'-dihydrospiro[azetidin-3,4'-pyrrolo[1,2-b]pyrazol]-1-carboxamid
2'-[6-Amino-5-(difluormethoxy)pyridin-3-yl]-N-(1-methylcyclobutyl)-5',6'-dihydrospiro[azetidin-3,4'-pyrrolo[1,2-b]pyrazol]-1-carboxamid
(rac)-2-[6-Amino-5-(trifluormethoxy)pyridin-3-yl]-N-[1-(1,3,4-trimethyl-1H-pyrazol-5-yl)ethyl]-6,7-dihydrospiro[pyrazolo[5,1-c][1,4]-oxazin-4,3'-pyrrolidin]-1'-carboxamid
2-[6-Amino-5-(trifluormethoxy)pyridin-3-yl]-N-[(1R)-2,3-dihydro-1H-inden-1-yl]-6,7-dihydrospiro[pyrazolo[5,1-c][1,4]-oxazin-4,3'-pyrrolidin]-1'-carboxamid
2'-[6-Amino-5-(difluormethoxy)pyridin-3-yl]-N-[1-(pyridin-2-yl)cyclobutyl]-5',6'-dihydrospiro[azetidin-3,4'-pyrrolo[1,2-b]pyrazol]-1-carboxamid
2'-[6-Amino-5-(difluormethoxy)pyridin-3-yl]-N-[1-(3-fluorphenyl)cyclobutyl]-5',6'-dihydrospiro[azetidin-3,4'-pyrrolo[1,2-b]pyrazol]-1-carboxamid
(rac)-2'-[6-Amino-5-(trifluormethoxy)pyridin-3-yl]-N-[1-(pyridin-2-yl)cyclobutyl]-5',6'-dihydrospiro[pyrrolidin-3,4'-pyrrolo[1,2-b]pyrazol]-1-carboxamid
2-[6-Amino-5-(difluormethoxy)pyridin-3-yl]-N-[(1S)-2-methoxy-1-phenylethyl]-6,7-dihydrospiro[pyrazolo[5,1-c][1,4]-oxazin-4,3'-pyrrolidin]-1'-carboxamid
2'-[6-Amino-5-(difluormethoxy)pyridin-3-yl]-N-[(1R)-1-phenylethyl]-5',6'-dihydrospiro[pyrrolidin-3,4'-pyrrolo[1,2-b]pyrazol]-1-carboxamid
(rac)-2'-[6-Amino-5-(difluormethoxy)pyridin-3-yl]-N-[1-(pyridin-4-yl)cyclobutyl]-5',6'-dihydrospiro[pyrrolidin-3,4'-pyrrolo[1,2-b]pyrazol]-1-carboxamid
(rac)-2'-[6-Amino-5-(difluormethoxy)pyridin-3-yl]-N-(1-phenylcyclobutyl)-5',6'-dihydrospiro[pyrrolidin-3,4'-pyrrolo[1,2-b]pyrazol]-1-carboxamid
(rac)-2'-[6-Amino-5-(difluormethoxy)pyridin-3-yl]-N-[2-(pyridin-4-yl)propan-2-yl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[(1R)-1-(4-fluorphenyl)ethyl]-5',6'-dihydrospiro[pyrrolidin-3,4'-pyrrolo[1,2-b]pyrazol]-1-carboxamid
(rac)-2'-[6-Amino-5-(difluormethoxy)pyridin-3-yl]-N-[2-(4-fluorphenyl)propan-2-yl]-5',6'-dihydrospiro[pyrrolidin-3,4'-pyrrolo[1,2-b]pyrazol]-1-carboxamid
2'-[6-Amino-5-(difluormethoxy)pyridin-3-yl]-N-[(1R)-1-(5-fluorpyridin-3-yl)ethyl]-5',6'-dihydrospiro[pyrrolidin-3,4'-pyrrolo[1,2-b]pyrazol]-1-carboxamid
2'-{6-Amino-5-[(1R)-1-(pyridin-2-yl)ethoxy]pyridin-3-yl}-N-[(1R)-1-phenylethyl]-5',6'-dihydrospiro[pyrrolidin-3,4'-pyrrolo[1,2-b]pyrazol]-1-carboxamid
2'-{6-Amino-5-[(1R)-1-(pyridin-2-yl)ethoxy]pyridin-3-yl}-N-[2-(pyridin-4-yl)propan-2-yl]-5',6'-dihydrospiro[pyrrolidin-3,4'-pyrrolo[1,2-b]pyrazol]-1-carboxamid
2'-{6-Amino-5-[(1S)-1-(pyridin-2-yl)ethoxy]pyridin-3-yl}-N-[(1R)-1-phenylethyl]-5',6'-dihydrospiro[pyrrolidin-3,4'-pyrrolo[1,2-b]pyrazol]-1-carboxamid
2'-{6-Amino-5-[(1S)-1-(pyridin-2-yl)ethoxy]pyridin-3-yl}-N-[2-(pyridin-4-yl)propan-2-yl]-5',6'-dihydrospiro[pyrrolidin-3,4'-pyrrolo[1,2-b]pyrazol]-1-carboxamid
2'-{6-Amino-5-[(trifluormethyl)sulfanyl]pyridin-3-yl}-N-[(1R)-1-(3-fluorphenyl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(3R)-2,3-dihydro-1-benzofuran-3-yl]-5',6'-dihydrospiro[pyrrolidin-3,4'-pyrrolo[1,2-b]pyrazol]-1-carboxamid
2'-{6-Amino-5-[(1R)-1-(pyridin-3-yl)ethoxy]pyridin-3-yl}-N-[2-(pyridin-4-yl)propan-2-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-(1-methyl-1H-imidazol-5-yl)ethyl]-5',6'-dihydrospiro[pyrrolidin-3,4'-pyrrolo[1,2-b]pyrazol]-1-carboxamid (diastereomeres Gemisch)
2'-{6-Amino-5-[(1R)-1-(pyridin-3-yl)ethoxy]pyridin-3-yl}-N-cyclobutyl-5',6'-dihydrospiro[pyrrolidin-3,4'-pyrrolo[1,2-b]pyrazol]-1-carboxamid
2'-{6-Amino-5-[(1R)-1-(pyridin-3-yl)ethoxy]pyridin-3-yl}-N-[2-(1-methyl-1H-1,2,3-triazol-4-yl)propan-2-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[cyclopropyl(pyridin-2-yl)methyl]-5',6'-dihydrospiro[pyrrolidin-3,4'-pyrrolo[1,2-b]pyrazol]-1-carboxamid (diastereomeres Gemisch)
2'-{6-Amino-5-[(1S)-1-(pyridin-3-yl)ethoxy]pyridin-3-yl}-N-cyclobutyl-5',6'-dihydrospiro[pyrrolidin-3,4'-pyrrolo[1,2-b]pyrazol]-1-carboxamid
2'-{6-Amino-5-[(1S)-1-(pyridin-3-yl)ethoxy]pyridin-3-yl}-N-[2-(pyridin-4-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(2-cyanophenyl)propan-2-yl]-5',6'-dihydrospiro[pyrrolidin-3,4'-pyrrolo[1,2-b]pyrazol]-1-carboxamid
[2'-{6-Amino-5-[(1R)-1-(pyridin-3-yl)ethoxy]pyridin-3-yl}-5',6'-dihydrospiro[pyrrolidin-3,4'-pyrrolo[1,2-b]pyrazol]-1-yl](1H-imidazol-1-yl)methanon
(rac)-2'-[6-Amino-5-(trifluormethyl)pyridin-3-yl]-N-(1-methylcyclobutyl)-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-(3-methyloxetan-3-yl)-5',6'-dihydrospiro[pyrrolidin-3,4'-pyrrolo[1,2-b]pyrazol]-1-carboxamid
(rac)-2-[6-Amino-5-(trifluormethoxy)pyridin-3-yl]-N-[2-(3-fluorpyridin-2-yl)propan-2-yl]-6,7-dihydrospiro[pyrazolo[5,1-c][1,4]-oxazin-4,3'-pyrrolidin]-1'-carboxamid
2'-{6-Amino-5-[(1S)-1-phenylethoxy]pyridin-3-yl}-N-[1-(pyridin-4-yl)cyclobutyl]-5',6'-dihydrospiro[pyrrolidin-3,4'-pyrrolo[1,2-b]pyrazol]-1-carboxamid
2'-{6-Amino-5-[(1S)-1-phenylethoxy]pyridin-3-yl}-N-[2-(3-chlorpyridin-4-yl)propan-2-yl]-5',6'-dihydrospiro[pyrrolidin-3,4'-pyrrolo[1,2-b]pyrazol]-1-carboxamid
2'-{6-Amino-5-[(1R)-1-phenylethoxy]pyridin-3-yl}-N-[1-(pyridin-4-yl)cyclobutyl]-5',6'-dihydrospiro[pyrrolidin-3,4'-pyrrolo[1,2-b]pyrazol]-1-carboxamid
2'-{6-Amino-5-[(1R)-1-phenylethoxy]pyridin-3-yl}-N-[2-(3-chlorpyridin-4-yl)propan-2-yl]-5',6'-dihydrospiro[pyrrolidin-3,4'-pyrrolo[1,2-b]pyrazol]-1-carboxamid
(rac)-2-[6-Amino-5-(trifluormethoxy)pyridin-3-yl]-N-[2-(pyridin-2-yl)propan-2-yl]-6,7-dihydrospiro[pyrazolo[5,1-c][1,4]-oxazin-4,3'-pyrrolidin]-1'-carboxamid
2'-{6-Amino-5-[(1R)-1-(pyridin-2-yl)ethoxy]pyridin-3-yl}-N-[2-(3-chlorpyridin-4-yl)propan-2-yl]-5',6'-dihydrospiro[pyrrolidin-3,4'-pyrrolo[1,2-b]pyrazol]-1-carboxamid
2'-{6-Amino-5-[(1R)-1-(pyridin-3-yl)ethoxy]pyridin-3-yl}-N-[2-(3-chlorpyridin-4-yl)propan-2-yl]-5',6'-dihydrospiro[pyrrolidin-3,4'-pyrrolo[1,2-b]pyrazol]-1-carboxamid
2'-{6-Amino-5-[(1R)-1-(pyridin-4-yl)ethoxy]pyridin-3-yl}-N-[2-(3-chlorpyridin-4-yl)propan-2-yl]-5',6'-dihydrospiro[pyrrolidin-3,4'-pyrrolo[1,2-b]pyrazol]-1-carboxamid
2'-(6-Amino-5-cyanopyridin-3-yl)-N-[(1R)-1-(pyridin-4-yl)ethyl]-5',6'-dihydrospiro[pyrrolidin-3,4'-pyrrolo[1,2-b]pyrazol]-1-carboxamid (Isomer 1)
2'-(6-Amino-5-cyanopyridin-3-yl)-N-[(1R)-1-(pyridin-4-yl)ethyl]-5',6'-dihydrospiro[pyrrolidin-3,4'-pyrrolo[1,2-b]pyrazol]-1-carboxamid (Isomer 2)
2'-(6-Amino-5-cyanopyridin-3-yl)-N-[1-(pyridin-4-yl)cyclobutyl]-5',6'-dihydrospiro[pyrrolidin-3,4'-pyrrolo[1,2-b]pyrazol]-1-carboxamid (Enantiomer 1)
(3R)-2'-[6-Amino-5-(trifluormethyl)pyridin-3-yl]-N-[(1S)-1-(pyridin-4-yl)ethyl]-5',6'-dihydrospiro[pyrrolidin-3,4'-pyrrolo[1,2-b]pyrazol]-1-carboxamid
(3S)-2'-[6-Amino-5-(trifluormethyl)pyridin-3-yl]-N-[(1S)-1-(pyridin-4-yl)ethyl]-5',6'-dihydrospiro[pyrrolidin-3,4'-pyrrolo[1,2-b]pyrazol]-1-carboxamid
2'-(6-Amino-5-cyanopyridin-3-yl)-N-[(1S)-1-(pyridin-4-yl)ethyl]-5',6'-dihydrospiro[pyrrolidin-3,4'-pyrrolo[1,2-b]pyrazol]-1-carboxamid (Isomer 1)
2'-(6-Amino-5-cyanopyridin-3-yl)-N-[(1S)-1-(pyridin-4-yl)ethyl]-5',6'-dihydrospiro[pyrrolidin-3,4'-pyrrolo[1,2-b]pyrazol]-1-carboxamid (Isomer 2)
2'-[6-Amino-5-(trifluormethyl)pyridin-3-yl]-N-[(1S)-1-(pyridin-4-yl)ethyl]-5',6'-dihydrospiro[azetidin-3,4'-pyrrolo[1,2-b]pyrazol]-1-carboxamid
2'-[6-Amino-5-(trifluormethyl)pyridin-3-yl]-N-[(1R)-1-(pyridin-4-yl)ethyl]-5',6'-dihydrospiro[azetidin-3,4'-pyrrolo[1,2-b]pyrazol]-1-carboxamid
(rac)-2'-[6-Amino-5-(trifluormethyl)pyridin-3-yl]-N-[1-(2-chlorphenyl)cyclobutyl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[2-(2-fluorphenyl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-(1-phenylcyclobutyl)-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-[2-(4-fluorphenyl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(2-chlorphenyl)propan-2-yl]-5',6'-dihydrospiro[pyrrolidin-3,4'-pyrrolo[1,2-b]pyrazol]-1-carboxamid
2-[6-Amino-5-(trifluormethyl)pyridin-3-yl]-N-[(lR)-1-(2-chlor-4-cyanophenyl)ethyl]-6,7-dihydrospiro[pyrazolo[5,1-c][1,4]-oxazin-4,3'-pyrrolidin]-1'-carboxamid (Gemisch von Stereoisomer)
2'-[6-Amino-5-(trifluormethyl)pyridin-3-yl]-N-[(lR)-1-(2-chlor-4-cyanophenyl)ethyl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1S)-1-(3-chlorpyridin-4-yl)ethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(3-fluorphenyl)propan-2-yl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[1-(pyridin-4-yl)cyclobutyl]-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-[2-(3-chlorpyridin-4-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-(1-phenylcyclobutyl)-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[2-(pyridin-2-yl)propan-2-yl]-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-[1-(2-fluorphenyl)cyclobutyl]-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-[1-(2-fluorphenyl)cyclobutyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[1-(3-fluorphenyl)cyclobutyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[1-(pyridin-4-yl)cyclobutyl]-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-[1-(3-fluorphenyl)cyclobutyl]-6,7-dihydrospiro[pyrazolo[5,1-c][1,4]-oxazin-4,3'-pyrrolidin]-1'-carboxamid
(3'R)-2-[6-Amino-5-(trifluormethoxy)pyridin-3-yl]-N-[2-(pyridin-2-yl)propan-2-yl]-6,7-dihydrospiro[pyrazolo[5,1-c][1,4]-oxazin-4,3'-pyrrolidin]-1'-carboxamid
(3'R)-2-[6-Amino-5-(trifluormethyl)pyridin-3-yl]-N-[2-(pyridin-3-yl)propan-2-yl]-6,7-dihydrospiro[pyrazolo[5,1-c][1,4]-oxazin-4,3'-pyrrolidin]-1'-carboxamid
(3R)-2'-[6-Amino-5-(trifluormethyl)pyridin-3-yl]-N-[2-(pyridin-4-yl)propan-2-yl]-5',6'-dihydrospiro[pyrrolidin-3,4'-pyrrolo[1,2-b]pyrazol]-1-carboxamid
(3'R)-2-[6-Amino-5-(trifluormethyl)pyridin-3-yl]-N-[2-(pyridin-4-yl)propan-2-yl]-6,7-dihydrospiro[pyrazolo[5,1-c][1,4]-oxazin-4,3'-pyrrolidin]-1'-carboxamid
(3R)-2'-[6-Amino-5-(trifluormethyl)pyridin-3-yl]-N-[2-(pyridin-3-yl)propan-2-yl]-5',6'-dihydrospiro[pyrrolidin-3,4'-pyrrolo[1,2-b]pyrazol]-1-carboxamid
(3R)-2'-[6-Amino-5-(difluormethoxy)pyridin-3-yl]-N-[(1R)-1-phenylethyl]-5',6'-dihydrospiro[pyrrolidin-3,4'-pyrrolo[1,2-b]pyrazol]-1-carboxamid
(3'R)-2-[6-Amino-5-(difluormethoxy)pyridin-3-yl]-N-[(1R)-1-phenylethyl]-6,7-dihydrospiro[pyrazolo[5,1-c][1,4]-oxazin-4,3'-pyrrolidin]-1'-carboxamid
(3R)-2'-[6-Amino-5-(trifluormethyl)pyridin-3-yl]-N-[2-(pyridin-2-yl)propan-2-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-1-phenylethyl]-5',6'-dihydrospiro[pyrrolidin-3,4'-pyrrolo[1,2-b]pyrazol]-1-carboxamid (Stereoisomer 1)
2'-[6-Amino-5-(trifluormethyl)pyridin-3-yl]-N-[(1R)-1-phenylethyl]-5',6'-dihydrospiro[pyrrolidin-3,4'-pyrrolo[1,2-b]pyrazol]-1-carboxamid (Stereoisomer 2)
(rac)-2'-[6-Amino-5-(trifluormethyl)pyridin-3-yl]-N-[2-(3,5-difluorphenyl)propan-2-yl]-5',6'-dihydrospiro[pyrrolidin-3,4'-pyrrolo[1,2-b]pyrazol]-1-carboxamid
(rac)-1-{2'-[6-Amino-5-(trifluormethyl)pyridin-3-yl]-5',6'-dihydrospiro[pyrrolidin-3,4'-pyrrolo[1,2-b]pyrazol]-1-yl}-2-(5-fluorpyridin-2-yl)-2-methylpropan-1-on
(rac)-2'-[6-Amino-5-(trifluormethyl)pyridin-3-yl]-N-[2-(6-methylpyridin-3-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-(1-phenylcyclohexyl)-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(5-fluorpyridin-2-yl)propan-2-yl]-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[(1R)-2,2,2-trifluor-1-methoxy-1-phenylethyl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(pyrimidin-4-yl)propan-2-yl]-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-[2-(pyrimidin-5-yl)propan-2-yl]-5',6'-dihydrospiro[pyrrolidin-3,4'-pyrrolo[1,2-b]pyrazol]-1-carboxamid
(rac)-{2'-[6-Amino-5-(trifluormethyl)pyridin-3-yl]-5',6'-dihydrospiro[pyrrolidin-3,4'-pyrrolo[1,2-b]pyrazol]-1-yl}(3-phenyloxetan-3-yl)methanon
(rac)-1-{2'-[6-Amino-5-(trifluormethyl)pyridin-3-yl]-5',6'-dihydrospiro[pyrrolidin-3,4'-pyrrolo[1,2-b]pyrazol]-1-yl}-2-(3,5-difluorphenyl)-2-methylpropan-1-on
(rac)-1-{2'-[6-Amino-5-(trifluormethyl)pyridin-3-yl]-5',6'-dihydrospiro[pyrrolidin-3,4'-pyrrolo[1,2-b]pyrazol]-1-yl}-2-methyl-2-(6-methylpyridin-3-yl)propan-1-on
(rac)-2'-[6-Amino-5-(trifluormethyl)pyridin-3-yl]-N-(3-phenyloxetan-3-yl)-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-(Propan-2-yl)-5',6'-dihydrospiro[pyrrolidin-3,4'-pyrrolo[1,2-b]pyrazol]-1-carboxamid
(rac)-2'-[6-Amino-5-(trifluormethyl)pyridin-3-yl]-N-cyclobutyl-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-ethyl-5',6'-dihydrospiro[pyrrolidin-3,4'-pyrrolo[1,2-b]pyrazol]-1-carboxamid
(3S)-2'-[6-Amino-5-(trifluormethyl)pyridin-3-yl]-N-ethyl-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-(Propan-2-yl)-5',6'-dihydrospiro[pyrrolidin-3,4'-pyrrolo[1,2-b]pyrazol]-1-carboxamid
(3S)-2'-[6-Amino-5-(trifluormethyl)pyridin-3-yl]-N-(Propan-2-yl)-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-cyclobutyl-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-ethyl-6,7-dihydrospiro[pyrazolo[5,1-c][1,4]-oxazin-4,3'-pyrrolidin]-1'-carboxamid
(rac)-2-[6-Amino-5-(trifluormethyl)pyridin-3-yl]-N-(Propan-2-yl)-6,7-dihydrospiro[pyrazolo[5,1-c][1,4]-oxazin-4,3'-pyrrolidin]-1'-carboxamid
2'-[6-Amino-5-(trifluormethyl)pyridin-3-yl]-N-cyclobutyl-5',6'-dihydrospiro[azetidin-3,4'-pyrrolo[1,2-b]pyrazol]-1-carboxamid
2'-[6-Amino-5-(trifluormethyl)pyridin-3-yl]-N-ethyl-5',6'-dihydrospiro[azetidin-3,4'-pyrrolo[1,2-b]pyrazol]-1-carboxamid
2'-[6-Amino-5-(trifluormethyl)pyridin-3-yl]-N-(Propan-2-yl)-5',6'-dihydrospiro[azetidin-3,4'-pyrrolo[1,2-b]pyrazol]-1-carboxamid
(rac)-2'-(6-Amino-5-cyanopyridin-3-yl)-N-[2-(3-chlorpyridin-4-yl)propan-2-yl]-5',6'-dihydrospiro[pyrrolidin-3,4'-pyrrolo[1,2-b]pyrazol]-1-carboxamid
(rac)-2'-{6-Amino-5-[(2-chlorphenyl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(2-chlorphenyl)methoxy]pyridin-3-yl}-N-(Propan-2-yl)-5',6'-dihydrospiro[pyrrolidin-3,4'-pyrrolo[1,2-b]pyrazol]-1-carboxamid
(rac)-2'-{6-Amino-5-[(2-chlorphenyl)methoxy]pyridin-3-yl}-N-cyclobutyl-5',6'-dihydrospiro[pyrrolidin-3,4'-pyrrolo[1,2-b]pyrazol]-1-carboxamid
(rac)-2'-{6-Amino-5-[(2-fluorphenyl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(2-fluorphenyl)methoxy]pyridin-3-yl}-N-(Propan-2-yl)-5',6'-dihydrospiro[pyrrolidin-3,4'-pyrrolo[1,2-b]pyrazol]-1-carboxamid
(rac)-2'-{6-Amino-5-[(2-fluorphenyl)methoxy]pyridin-3-yl}-N-cyclobutyl-5',6'-dihydrospiro[pyrrolidin-3,4'-pyrrolo[1,2-b]pyrazol]-1-carboxamid
(rac)-2'-{6-Amino-5-[(2-fluorphenyl)methoxy]pyridin-3-yl}-N-benzyl-5',6'-dihydrospiro[pyrrolidin-3,4'-pyrrolo[1,2-b]pyrazol]-1-carboxamid
(rac)-2'-{6-Amino-5-[(3-fluorphenyl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(3-fluorphenyl)methoxy]pyridin-3-yl}-N-(Propan-2-yl)-5',6'-dihydrospiro[pyrrolidin-3,4'-pyrrolo[1,2-b]pyrazol]-1-carboxamid
(rac)-2'-{6-Amino-5-[(3-fluorphenyl)methoxy]pyridin-3-yl}-N-cyclobutyl-5',6'-dihydrospiro[pyrrolidin-3,4'-pyrrolo[1,2-b]pyrazol]-1-carboxamid
(rac)-2'-{6-Amino-5-[(3-fluorphenyl)methoxy]pyridin-3-yl}-N-benzyl-5',6'-dihydrospiro[pyrrolidin-3,4'-pyrrolo[1,2-b]pyrazol]-1-carboxamid
(rac)-2'-{6-Amino-5-[(pyridin-2-yl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(pyridin-2-yl)methoxy]pyridin-3-yl}-N-(Propan-2-yl)-5',6'-dihydrospiro[pyrrolidin-3,4'-pyrrolo[1,2-b]pyrazol]-1-carboxamid
(rac)-2'-{6-Amino-5-[(pyridin-2-yl)methoxy]pyridin-3-yl}-N-cyclobutyl-5',6'-dihydrospiro[pyrrolidin-3,4'-pyrrolo[1,2-b]pyrazol]-1-carboxamid
(rac)-2'-{6-Amino-5-[(2-Chlor-5-fluorphenyl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(2-Chlor-5-fluorphenyl)methoxy]pyridin-3-yl}-N-(Propan-2-yl)-5',6'-dihydrospiro[pyrrolidin-3,4'-pyrrolo[1,2-b]pyrazol]-1-carboxamid
2'-{6-Amino-5-[(1R)-1-(pyridin-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
2'-{6-Amino-5-[(1R)-1-(pyridin-2-yl)ethoxy]pyridin-3-yl}-N-ethyl-5',6'-dihydrospiro[pyrrolidin-3,4'-pyrrolo[1,2-b]pyrazol]-1-carboxamid
2'-{6-Amino-5-[(1S)-1-(pyridin-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
2'-{6-Amino-5-[(1S)-1-(pyridin-2-yl)ethoxy]pyridin-3-yl}-N-ethyl-5',6'-dihydrospiro[pyrrolidin-3,4'-pyrrolo[1,2-b]pyrazol]-1-carboxamid
2'-(6-Amino-5-{[1-(5-Chlor-3-fluorpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (diastereomeres Gemisch)
(rac)-2'-[6-Amino-5-(benzyloxy)pyridin-3-yl]-N-cyclobutyl-5',6'-dihydrospiro[pyrrolidin-3,4'-pyrrolo[1,2-b]pyrazol]-1-carboxamid
2'-{6-Amino-5-[(pyridazin-3-yl)methoxy]pyridin-3-yl}-N-ethyl-5',6'-dihydrospiro[pyrrolidin-3,4'-pyrrolo[1,2-b]pyrazol]-1-carboxamid (Stereoisomer 2)
2'-{6-Amino-5-[(pyridazin-3-yl)methoxy]pyridin-3-yl}-N-ethyl-5',6'-dihydrospiro[pyrrolidin-3,4'-pyrrolo[1,2-b]pyrazol]-1-carboxamid (Stereoisomer 1)
(3S)-2'-{6-Amino-5-[1-(pyrimidin-5-yl)ethoxy]pyridin-3-yl}-N-ethyl-5',6'-dihydro-1H-spiro[pyrrolidin-3,4'-pyrrolo[1,2-b]pyrazol]-1-carboxamid (Isomer 1)
(3S)-2'-{6-Amino-5-[1-(pyrimidin-5-yl)ethoxy]pyridin-3-yl}-N-ethyl-5',6'-dihydro-1H-spiro[pyrrolidin-3,4'-pyrrolo[1,2-b]pyrazol]-1-carboxamid (Isomer 2)
(rac)-2'-(2-Amino-1'-methyl-1',2',3',6'-tetrahydro[3,4'-bipyridin]-5-yl)-N-ethyl-5',6'-dihydrospiro[pyrrolidin-3,4'-pyrrolo[1,2-b]pyrazol]-1-carboxamid
2'-{6-Amino-5-[(1S)-1-(pyridin-2-yl)ethoxy]pyridin-3-yl}-N-ethyl-5',6'-dihydrospiro[azetidin-3,4'-pyrrolo[1,2-b]pyrazol]-1-carboxamid
2'-{6-Amino-5-[(1S)-1-(pyridin-2-yl)ethoxy]pyridin-3-yl}-N-(Propan-2-yl)-5',6'-dihydrospiro[azetidin-3,4'-pyrrolo[1,2-b]pyrazol]-1-carboxamid
2'-{6-Amino-5-[(1S)-1-phenylethoxy]pyridin-3-yl}-N-ethyl-5',6'-dihydrospiro[azetidin-3,4'-pyrrolo[1,2-b]pyrazol]-1-carboxamid
2'-{6-Amino-5-[(1R)-1-phenylethoxy]pyridin-3-yl}-N-ethyl-5',6'-dihydrospiro[azetidin-3,4'-pyrrolo[1,2-b]pyrazol]-1-carboxamid
(rac)-2-[6-Amino-5-(trifluormethyl)pyridin-3-yl]-N-[2-(3-chlorpyridin-4-yl)propan-2-yl]-6,7-dihydrospiro[pyrazolo[5,1-c][1,4]-oxazin-4,3'-pyrrolidin]-1'-carboxamid
(rac)-2'-[6-Amino-5-(trifluormethyl)pyridin-3-yl]-N-ethyl-5',6'-dihydrospiro[pyrrolidin-3,4'-pyrrolo[1,2-b]pyrazol]-1-carboxamid
(rac)-2-[6-Amino-5-(trifluormethyl)pyridin-3-yl]-N-ethyl-6,7-dihydro-5H-spiro[pyrazolo[1,5-a]pyridin-4,3'-pyrrolidin]-1'-carboxamid
(rac)-2'-(6-Amino-5-cyanopyridin-3-yl)-N-ethyl-5',6'-dihydrospiro[pyrrolidin-3,4'-pyrrolo[1,2-b]pyrazol]-1-carboxamid
(rac)-2'-[6-Amino-5-(trifluormethoxy)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(difluormethoxy)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(trifluoracetyl)pyridin-3-yl]-N-ethyl-5',6'-dihydrospiro[pyrrolidin-3,4'-pyrrolo[1,2-b]pyrazol]-1-carboxamid
2'-(6-Amino-5-{[1-phenylethyl]sulfanyl}pyridin-3-yl)-N-ethyl-5',6'-dihydrospiro[pyrrolidin-3,4'-pyrrolo[1,2-b]pyrazol]-1-carboxamid (diastereomeres Gemisch)
(rac)-2'-{6-Amino-5-[(cyclohexylmethyl)sulfanyl]pyridin-3-yl}-N-ethyl-5',6'-dihydrospiro[pyrrolidin-3,4'-pyrrolo[1,2-b]pyrazol]-1-carboxamid
(rac)-2'-[6-Amino-5-(benzylsulfanyl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(2-hydroxypropan-2-yl)pyridin-3-yl]-N-ethyl-5',6'-dihydrospiro[pyrrolidin-3,4'-pyrrolo[1,2-b]pyrazol]-1-carboxamid
2'-(6-Amino-5-{[1-cyclohexylethyl]sulfanyl}pyridin-3-yl)-N-ethyl-5',6'-dihydrospiro[pyrrolidin-3,4'-pyrrolo[1,2-b]pyrazol]-1-carboxamid (diastereomeres Gemisch)
2'-{6-Amino-5-[(1R)-1-(pyridin-3-yl)ethoxy]pyridin-3-yl}-N-ethyl-5',6'-dihydrospiro[pyrrolidin-3,4'-pyrrolo[1,2-b]pyrazol]-1-carboxamid
2'-{6-Amino-5-[(1S)-1-(pyridin-3-yl)ethoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(cyclopentylmethyl)sulfanyl]pyridin-3-yl}-N-ethyl-5',6'-dihydrospiro[pyrrolidin-3,4'-pyrrolo[1,2-b]pyrazol]-1-carboxamid
(rac)-2'-(6-Amino-5-nitropyridin-3-yl)-N-ethyl-5',6'-dihydrospiro[pyrrolidin-3,4'-pyrrolo[1,2-b]pyrazol]-1-carboxamid
(rac)-2'-[6-Amino-5-(benzyloxy)pyridin-3-yl]-N-ethyl-5',6'-dihydrospiro[pyrrolidin-3,4'-pyrrolo[1,2-b]pyrazol]-1-carboxamid
(rac)-2'-(6-Amino-5-methylpyridin-3-yl)-N-ethyl-5',6'-dihydrospiro[pyrrolidin-3,4'-pyrrolo[1,2-b]pyrazol]-1-carboxamid
(rac)-2-(6-Amino-5-nitropyridin-3-yl)-N-ethyl-6,7-dihydro-5H-spiro[pyrazolo[1,5-a]pyridin-4,3'-pyrrolidin]-1'-carboxamid
2-Amino-5-[(rac)-1-(ethylcarbamoyl)-5',6'-dihydrospiro[pyrrolidin-3,4'-pyrrolo[1,2-b]pyrazol]-2'-yl]pyridin-3-carbonsäuremethylester
(rac)-2'-(6-Amino-5-phenylpyridin-3-yl)-N-ethyl-5',6'-dihydrospiro[pyrrolidin-3,4'-pyrrolo[1,2-b]pyrazol]-1-carboxamid
(rac)-2'-[6-Amino-5-(methylsulfanyl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(4-methylphenyl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(2-methylphenyl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(3-methylphenyl)pyridin-3-yl]-N-ethyl-5',6'-dihydrospiro[pyrrolidin-3,4'-pyrrolo[1,2-b]pyrazol]-1-carboxamid
(rac)-2'-(6-Amino-5-methoxypyridin-3-yl)-N-ethyl-5',6'-dihydrospiro[pyrrolidin-3,4'-pyrrolo[1,2-b]pyrazol]-1-carboxamid
(rac)-2'-[6-Amino-5-(3-fluorphenyl)pyridin-3-yl]-N-ethyl-5',6'-dihydrospiro[pyrrolidin-3,4'-pyrrolo[1,2-b]pyrazol]-1-carboxamid
(rac)-2'-(2-Amino[3,3'-bipyridin]-5-yl)-N-ethyl-5',6'-dihydrospiro[pyrrolidin-3,4'-pyrrolo[1,2-b]pyrazol]-1-carboxamid
(rac)-2'-[6-Amino-5-(2-fluorphenyl)pyridin-3-yl]-N-ethyl-5',6'-dihydrospiro[pyrrolidin-3,4'-pyrrolo[1,2-b]pyrazol]-1-carboxamid
2'-{6-Amino-5-[(1R)-1-(2,6-Dichlor-3-fluorphenyl)ethoxy]pyridin-3-yl}-N-ethyl-5',6'-dihydrospiro[pyrrolidin-3,4'-pyrrolo[1,2-b]pyrazol]-1-carboxamid (diastereomeres Gemisch)
(rac)-2-[6-Amino-5-(2-fluorphenyl)pyridin-3-yl]-N-ethyl-6,7-dihydro-5H-spiro[pyrazolo[1,5-a]pyridin-4,3'-pyrrolidin]-1'-carboxamid
(rac)-2'-[6-Amino-5-(2-methoxyphenyl)pyridin-3-yl]-N-ethyl-5',6'-dihydrospiro[pyrrolidin-3,4'-pyrrolo[1,2-b]pyrazol]-1-carboxamid
(rac)-2'-(2-Amino[3,4'-bipyridin]-5-yl)-N-ethyl-5',6'-dihydrospiro[pyrrolidin-3,4'-pyrrolo[1,2-b]pyrazol]-1-carboxamid
(rac)-2'-{6-Amino-5-[3-(methansulfonyl)phenyl]pyridin-3-yl}-N-ethyl-5',6'-dihydrospiro[pyrrolidin-3,4'-pyrrolo[1,2-b]pyrazol]-1-carboxamid
(rac)-2'-[6-Amino-5-(phenylsulfanyl)pyridin-3-yl]-N-ethyl-5',6'-dihydrospiro[pyrrolidin-3,4'-pyrrolo[1,2-b]pyrazol]-1-carboxamid
(rac)-2'-{6-Amino-5-[4-(methansulfonyl)phenyl]pyridin-3-yl}-N-ethyl-5',6'-dihydrospiro[pyrrolidin-3,4'-pyrrolo[1,2-b]pyrazol]-1-carboxamid
(rac)-2'-[5-(4-Acetylphenyl)-6-aminopyridin-3-yl]-N-ethyl-5',6'-dihydrospiro[pyrrolidin-3,4'-pyrrolo[1,2-b]pyrazol]-1-carboxamid
(rac)-2'-{6-Amino-5-[4-(methansulfinyl)phenyl]pyridin-3-yl}-N-ethyl-5',6'-dihydrospiro[pyrrolidin-3,4'-pyrrolo[1,2-b]pyrazol]-1-carboxamid
(rac)-2'-[6-Amino-5-(3-methoxyphenyl)pyridin-3-yl]-N-ethyl-5',6'-dihydrospiro[pyrrolidin-3,4'-pyrrolo[1,2-b]pyrazol]-1-carboxamid
2'-{6-Amino-5-[(1R)-1-(2,6-Dichlor-3-fluorphenyl)ethoxy]pyridin-3-yl}-N-ethyl-5',6'-dihydrospiro[azetidin-3,4'-pyrrolo[1,2-b]pyrazol]-1-carboxamid
(rac)-2'-{6-Amino-5-[4-(cyclopropansulfonyl)phenyl]pyridin-3-yl}-N-ethyl-5',6'-dihydrospiro[pyrrolidin-3,4'-pyrrolo[1,2-b]pyrazol]-1-carboxamid
(rac)-2'-[6-Amino-5-(4-sulfamoylphenyl)pyridin-3-yl]-N-ethyl-5',6'-dihydrospiro[pyrrolidin-3,4'-pyrrolo[1,2-b]pyrazol]-1-carboxamid
(rac)-2'-{6-Amino-5-[4-(methylsulfamoyl)phenyl]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[4-(dimethylsulfamoyl)phenyl]pyridin-3-yl}-N-ethyl-5',6'-dihydrospiro[pyrrolidin-3,4'-pyrrolo[1,2-b]pyrazol]-1-carboxamid
(rac)-2'-[6-Amino-5-(cyclohexylmethoxy)pyridin-3-yl]-N-ethyl-5',6'-dihydrospiro[pyrrolidin-3,4'-pyrrolo[1,2-b]pyrazol]-1-carboxamid
(rac)-2'-{6-Amino-5-[(pyridin-3-yl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(4-fluorphenyl)methoxy]pyridin-3-yl}-N-ethyl-5',6'-dihydrospiro[pyrrolidin-3,4'-pyrrolo[1,2-b]pyrazol]-1-carboxamid
2'-(6-Amino-5-{[1-phenylethyl]oxy}pyridin-3-yl)-N-ethyl-5',6'-dihydrospiro[pyrrolidin-3,4'-pyrrolo[1,2-b]pyrazol]-1-carboxamid (diastereomeres Gemisch)
2-{6-Amino-5-[(1R)-1-(2,6-Dichlor-3-fluorphenyl)ethoxy]pyridin-3-yl}-N-ethyl-6,7-dihydrospiro[pyrazolo[5,1-c][1,4]-oxazin-4,3'-pyrrolidin]-1'-carboxamid
(rac)-2'-[6-Amino-5-(Propan-2-yl)pyridin-3-yl]-N-ethyl-5',6'-dihydrospiro[pyrrolidin-3,4'-pyrrolo[1,2-b]pyrazol]-1-carboxamid
(rac)-2-[6-Amino-5-(methylcarbamoyl)pyridin-3-yl]-N-ethyl-6,7-dihydrospiro[pyrazolo[5,1-c][1,4]-oxazin-4,3'-pyrrolidin]-1'-carboxamid
(rac)-2'-[6-Amino-5-(methylcarbamoyl)pyridin-3-yl]-N-ethyl-5',6'-dihydrospiro[pyrrolidin-3,4'-pyrrolo[1,2-b]pyrazol]-1-carboxamid
(rac)-2'-(6-Amino-5-cyclopropylpyridin-3-yl)-N-ethyl-5',6'-dihydrospiro[pyrrolidin-3,4'-pyrrolo[1,2-b]pyrazol]-1-carboxamid
(rac)-2'-[6-Amino-5-(methylcarbamoyl)pyridin-3-yl]-N-(1-phenylcyclobutyl)-5',6'-dihydrospiro[pyrrolidin-3,4'-pyrrolo[1,2-b]pyrazol]-1-carboxamid
(rac)-2'-[6-Amino-5-(dimethylcarbamoyl)pyridin-3-yl]-N-(1-phenylcyclobutyl)-5',6'-dihydrospiro[pyrrolidin-3,4'-pyrrolo[1,2-b]pyrazol]-1-carboxamid
2'-{6-Amino-5-[(1R)-1-(pyridin-4-yl)ethoxy]pyridin-3-yl}-N-ethyl-5',6'-dihydrospiro[pyrrolidin-3,4'-pyrrolo[1,2-b]pyrazol]-1-carboxamid
2'-{6-Amino-5-[(1S)-1-(pyridin-4-yl)ethoxy]pyridin-3-yl}-N-ethyl-5',6'-dihydrospiro[pyrrolidin-3,4'-pyrrolo[1,2-b]pyrazol]-1-carboxamid
(rac)-2'-[6-Amino-5-(1-methyl-1H-pyrazol-3-yl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(1,2-thiazol-5-yl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(1,5-dimethyl-1H-pyrazol-4-yl)pyridin-3-yl]-N-ethyl-5',6'-dihydrospiro[pyrrolidin-3,4'-pyrrolo[1,2-b]pyrazol]-1-carboxamid
(rac)-2'-{6-Amino-5-[(trifluormethyl)sulfanyl]pyridin-3-yl}-N-ethyl-5',6'-dihydrospiro[pyrrolidin-3,4'-pyrrolo[1,2-b]pyrazol]-1-carboxamid
5-[1-Methyl-5',6'-dihydrospiro[pyrrolidin-3,4'-pyrrolo[1,2-b]pyrazol]-2'-yl]-3-(trifluormethyl)pyridin-2-amin (Stereoisomer 1)
5-[1-Methyl-5',6'-dihydrospiro[pyrrolidin-3,4'-pyrrolo[1,2-b]pyrazol]-2'-yl]-3-(trifluormethyl)pyridin-2-amin (Stereoisomer 2)
(3R)-2'-{6-Amino-5-[(1R)-1-(2-chlorphenyl)ethoxy]pyridin-3-yl}-N-ethyl-5',6'-dihydrospiro[pyrrolidin-3,4'-pyrrolo[1,2-b]pyrazol]-1-carboxamid
2'-{6-Amino-5-[(1R)-1-(2-chlorphenyl)ethoxy]pyridin-3-yl}-N-ethyl-5',6'-dihydrospiro[azetidin-3,4'-pyrrolo[1,2-b]pyrazol]-1-carboxamid
2'-{6-Amino-5-[(1S)-1-(2-chlorphenyl)ethoxy]pyridin-3-yl}-N-ethyl-5',6'-dihydrospiro[azetidin-3,4'-pyrrolo[1,2-b]pyrazol]-1-carboxamid
(3'R)-2-{6-Amino-5-[(1R)-1-(2,6-Dichlor-3-fluorphenyl)ethoxy]pyridin-3-yl}-N-ethyl-6,7-dihydrospiro[pyrazolo[5,1-c][1,4]-oxazin-4,3'-pyrrolidin]-1'-carboxamid
(3R)-2'-{6-Amino-5-[(1R)-1-(pyridin-2-yl)ethoxy]pyridin-3-yl}-N-ethyl-5',6'-dihydrospiro[pyrrolidin-3,4'-pyrrolo[1,2-b]pyrazol]-1-carboxamid
2'-{6-Amino-5-[(1R)-1-(pyridin-3-yl)ethoxy]pyridin-3-yl}-N-ethyl-5',6'-dihydrospiro[azetidin-3,4'-pyrrolo[1,2-b]pyrazol]-1-carboxamid
(3R)-2'-{6-Amino-5-[1-(pyrimidin-5-yl)ethoxy]pyridin-3-yl}-N-ethyl-5',6'-dihydro-1H-spiro[pyrrolidin-3,4'-pyrrolo[1,2-b]pyrazol]-1-carboxamid (Isomer 1)
((3R)-2'-{6-Amino-5-[1-(pyrimidin-5-yl)ethoxy]pyridin-3-yl}-N-ethyl-5',6'-dihydro-1H-spiro[pyrrolidin-3,4'-pyrrolo[1,2-b]pyrazol]-1-carboxamid (Isomer 2)
(rac)-2'-[6-Amino-5-(difluormethyl)pyridin-3-yl]-N-ethyl-5',6'-dihydrospiro[pyrrolidin-3,4'-pyrrolo[1,2-b]pyrazol]-1-carboxamid
2'-{6-Amino-5-[(1S)-1-(2-chlorphenyl)ethoxy]pyridin-3-yl}-N-ethyl-5',6'-dihydrospiro[pyrrolidin-3,4'-pyrrolo[1,2-b]pyrazol]-1-carboxamid
2'-{6-Amino-5-[(1R)-1-(2-chlorphenyl)ethoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(3-methylphenyl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(1-methyl-1H-pyrazol-5-yl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(1-methyl-1H-pyrazol-4-yl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(1,5-dimethyl-1H-pyrazol-3-yl)methoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(4-methylphenyl)methoxy]pyridin-3-yl}-N-ethyl-5',6'-dihydrospiro[pyrrolidin-3,4'-pyrrolo[1,2-b]pyrazol]-1-carboxamid
2'-{6-Amino-5-[(1R)-1-(3-methylphenyl)ethoxy]pyridin-3-yl}-N-ethyl-5',6'-dihydrospiro[pyrrolidin-3,4'-pyrrolo[1,2-b]pyrazol]-1-carboxamid
(rac)-2'-{6-Amino-5-[(Propan-2-yl)oxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1S)-1-(1-methyl-1H-pyrazol-3-yl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1R)-1-(1-methyl-1H-pyrazol-3-yl)ethoxy]pyridin-3-yl}-N-ethyl-5',6'-dihydrospiro[pyrrolidin-3,4'-pyrrolo[1,2-b]pyrazol]-1-carboxamid
(3R)-2'-(6-Amino-5-{[1-(3,5-difluorpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Stereoisomer 1)
(3R)-2'-(6-Amino-5-{[1-(3,5-difluorpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Stereoisomer 2)
(3R)-2'-{6-Amino-5-[(1R)-1-(2-fluorphenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1S)-1-(2-fluorphenyl)ethoxy]pyridin-3-yl}-N-ethyl-5',6'-dihydrospiro[pyrrolidin-3,4'-pyrrolo[1,2-b]pyrazol]-1-carboxamid
(rac)-2'-(5-Acetyl-6-aminopyridin-3-yl)-N-(1-phenylcyclobutyl)-5',6'-dihydrospiro[pyrrolidin-3,4'-pyrrolo[1,2-b]pyrazol]-1-carboxamid
(3R)-2'-[6-Amino-5-(trifluormethoxy)pyridin-3-yl]-N-[2-(pyridin-2-yl)propan-2-yl]-5',6'-dihydrospiro[pyrrolidin-3,4'-pyrrolo[1,2-b]pyrazol]-1-carboxamid
(3'R)-2-[6-Amino-5-(trifluormethyl)pyridin-3-yl]-N-[2-(pyridin-2-yl)propan-2-yl]-6,7-dihydrospiro[pyrazolo[5,1-c][1,4]-oxazin-4,3'-pyrrolidin]-1'-carboxamid
(3R)-2'-(6-Amino-5-cyanopyridin-3-yl)-N-[2-(pyridin-2-yl)propan-2-yl]-5',6'-dihydrospiro[pyrrolidin-3,4'-pyrrolo[1,2-b]pyrazol]-1-carboxamid
(3'R)-2-(6-Amino-5-cyanopyridin-3-yl)-N-[2-(pyridin-2-yl)propan-2-yl]-6,7-dihydrospiro[pyrazolo[5,1-c][1,4]-oxazin-4,3'-pyrrolidin]-1'-carboxamid
(rac)-2'-[6-Amino-5-(phenylcarbamoyl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(cyclohexylcarbamoyl)pyridin-3-yl]-N-ethyl-5',6'-dihydrospiro[pyrrolidin-3,4'-pyrrolo[1,2-b]pyrazol]-1-carboxamid
(rac)-2'-[6-Amino-5-(diethylcarbamoyl)pyridin-3-yl]-N-ethyl-5',6'-dihydrospiro[pyrrolidin-3,4'-pyrrolo[1,2-b]pyrazol]-1-carboxamid
(rac)-2'-(5-Acetyl-6-aminopyridin-3-yl)-N-ethyl-5',6'-dihydrospiro[pyrrolidin-3,4'-pyrrolo[1,2-b]pyrazol]-1-carboxamid
5-{1-[1-(1H-Imidazol-2-yl)ethyl]-5',6'-dihydrospiro[pyrrolidin-3,4'-pyrrolo[1,2-b]pyrazol]-2'-yl}-3-(trifluormethyl)pyridin-2-amin
5-{1-[1-(1H-Pyrazol-3-yl)ethyl]-5',6'-dihydrospiro[pyrrolidin-3,4'-pyrrolo[1,2-b]pyrazol]-2'-yl}-3-(trifluormethyl)pyridin-2-amin
5-{ (3R)-1-[1-(1H-Pyrazol-3-yl)ethyl]-5',6'-dihydrospiro[pyrrolidin-3,4'-pyrrolo[1,2-b]pyrazol]-2'-yl}-3-(trifluormethyl)pyridin-2-amin
5-{ (3R)-1-[1-(1H-Imidazol-2-yl)ethyl]-5',6'-dihydrospiro[pyrrolidin-3,4'-pyrrolo[1,2-b]pyrazol]-2'-yl}-3-(trifluormethyl)pyridin-2-amin
5-{ (3R)-1-[1-(1H-Imidazol-5-yl)ethyl]-5',6'-dihydrospiro[pyrrolidin-3,4'-pyrrolo[1,2-b]pyrazol]-2'-yl}-3-(trifluormethyl)pyridin-2-amin
5-{ (3R)-1-[1-(1-Methyl-1H-pyrazol-3-yl)ethyl]-5',6'-dihydrospiro[pyrrolidin-3,4'-pyrrolo[1,2-b]pyrazol]-2'-yl}-3-(trifluormethyl)pyridin-2-amin
5-{(3R)-1-[1-(1H-Pyrazol-4-yl)ethyl]-5',6'-dihydrospiro[pyrrolidin-3,4'-pyrrolo[1,2-b]pyrazol]-2'-yl}-3-(trifluormethyl)pyridin-2-amin
5-{(3R)-1-[1-(4-Methyl-1H-imidazol-2-yl)ethyl]-5',6'-dihydrospiro[pyrrolidin-3,4'-pyrrolo[1,2-b]pyrazol]-2'-yl}-3-(trifluormethyl)pyridin-2-amin
5-{(3R)-1-[1-(3-Methyl-1H-pyrazol-5-yl)ethyl]-5',6'-dihydrospiro[pyrrolidin-3,4'-pyrrolo[1,2-b]pyrazol]-2'-yl}-3-(trifluormethyl)pyridin-2-amin
5-{(3R)-1-[1-(4-Methyl-1H-pyrazol-5-yl)ethyl]-5',6'-dihydrospiro[pyrrolidin-3,4'-pyrrolo[1,2-b]pyrazol]-2'-yl}-3-(trifluormethyl)pyridin-2-amin
5-[(3S)-1-(Pyridin-3-ylmethyl)-5',6'-dihydrospiro[pyrrolidin-3,4'-pyrrolo[1,2-b]pyrazol]-2'-yl]-3-(trifluormethyl)pyridin-2-amin
5-[(3R)-1-(Pyridin-3-ylmethyl)-5',6'-dihydrospiro[pyrrolidin-3,4'-pyrrolo[1,2-b]pyrazol]-2'-yl]-3-(trifluormethyl)pyridin-2-amin
5-{(rac)-1-[(1H-Pyrazol-3-yl)methyl]-5',6'-dihydrospiro[pyrrolidin-3,4'-pyrrolo[1,2-b]pyrazol]-2'-yl}-3-(trifluormethyl)pyridin-2-amin
5-[(3S)-1-(1H-Imidazol-2-ylmethyl)-5',6'-dihydrospiro[pyrrolidin-3,4'-pyrrolo[1,2-b]pyrazol]-2'-yl]-3-(trifluormethyl)pyridin-2-amin
5-[(3R)-1-(1H-Imidazol-2-ylmethyl)-5',6'-dihydrospiro[pyrrolidin-3,4'-pyrrolo[1,2-b]pyrazol]-2'-yl]-3-(trifluormethyl)pyridin-2-amin
5-{(rac)-1-[(4-Methyl-1H-pyrazol-3-yl)methyl]-5',6'-dihydrospiro[pyrrolidin-3,4'-pyrrolo[1,2-b]pyrazol]-2'-yl}-3-(trifluormethyl)pyridin-2-amin
5-{(rac)-1-[(1H-Pyrazol-4-yl)methyl]-5',6'-dihydrospiro[pyrrolidin-3,4'-pyrrolo[1,2-b]pyrazol]-2'-yl}-3-(trifluormethyl)pyridin-2-amin
5-{(rac)-1-[(1-Methyl-1H-pyrazol-4-yl)methyl]-5',6'-dihydrospiro[pyrrolidin-3,4'-pyrrolo[1,2-b]pyrazol]-2'-yl}-3-(trifluormethyl)pyridin-2-amin
5-{ (rac)-1-[(1H-Imidazol-4-yl)methyl]-5',6'-dihydrospiro[pyrrolidin-3,4'-pyrrolo[1,2-b]pyrazol]-2'-yl}-3-(trifluormethyl)pyridin-2-amin
5-{(rac)-1-[(1-Methyl-1H-imidazol-4-yl)methyl]-5',6'-dihydrospiro[pyrrolidin-3,4'-pyrrolo[1,2-b]pyrazol]-2'-yl}-3-(trifluormethyl)pyridin-2-amin
5-{(rac)-1-[(4-Chlor-1H-pyrazol-5-yl)methyl]-5',6'-dihydrospiro[pyrrolidin-3,4'-pyrrolo[1,2-b]pyrazol]-2'-yl}-3-(trifluormethyl)pyridin-2-amin
3-[(2-Fluorphenyl)methoxy]-5-{(rac)-1-[(1H-imidazol-2-yl)methyl]-5',6'-dihydrospiro[pyrrolidin-3,4'-pyrrolo[1,2-b]pyrazol]-2'-yl}pyridin-2-amin
3-[(3-Fluorphenyl)methoxy]-5-{(rac)-1-[(1H-imidazol-2-yl)methyl]-5',6'-dihydrospiro[pyrrolidin-3,4'-pyrrolo[1,2-b]pyrazol]-2'-yl}pyridin-2-amin
5-{(rac)-1-[(1H-Imidazol-2-yl)methyl]-5',6'-dihydrospiro[pyrrolidin-3,4'-pyrrolo[1,2-b]pyrazol]-2'-yl}-3-(trifluormethyl)pyridin-2-amin
5-{1-[(1H-Imidazol-2-yl)methyl]-5',6'-dihydrospiro[azetidin-3,4'-pyrrolo[1,2-b]pyrazol]-2'-yl}-3-(trifluormethyl)pyridin-2-amin
5-{(rac)-1'-[(1H-Imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin
2-Amino-5-{1-[(1H-imidazol-2-yl)methyl]-5',6'-dihydrospiro[azetidin-3,4'-pyrrolo[1,2-b]pyrazol]-2'-yl}pyridin-3-carbonitril
5-{(rac)-1-[(1-Methyl-1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin
5-{(rac)-1-[(1-Methyl-1H-pyrazol-3-yl)methyl]-5',6'-dihydrospiro[pyrrolidin-3,4'-pyrrolo[1,2-b]pyrazol]-2'-yl}-3-(trifluormethyl)pyridin-2-amin
2-Amino-5-{(rac)-1-[(1H-imidazol-2-yl)methyl]-5',6'-dihydrospiro[pyrrolidin-3,4'-pyrrolo[1,2-b]pyrazol]-2'-yl}pyridin-3-carbonitril
5-{1-[(1-Methyl-1H-imidazol-2-yl)methyl]-5',6'-dihydrospiro[azetidin-3,4'-pyrrolo[1,2-b]pyrazol]-2'-yl}-3-(trifluormethyl)pyridin-2-amin
5-{(rac)-1-[(5-Methyl-1H-pyrazol-3-yl)methyl]-5',6'-dihydrospiro[pyrrolidin-3,4'-pyrrolo[1,2-b]pyrazol]-2'-yl}-3-(trifluormethyl)pyridin-2-amin
5-{(rac)-1-[(2,4-Dimethyl-1H-imidazol-5-yl)methyl]-5',6'-dihydrospiro[pyrrolidin-3,4'-pyrrolo[1,2-b]pyrazol]-2'-yl}-3-(trifluormethyl)pyridin-2-amin
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Stereoisomer 1)
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Stereoisomer 2)
5-{(3R)-1-[(1-(1H-Imidazol-2-yl)propyl]-5',6'-dihydrospiro[pyrrolidin-3,4'-pyrrolo[1,2-b]pyrazol]-2'-yl}-3-(trifluormethyl)pyridin-2-amin
5-{(3R)-1-[1-(1H-Imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Stereoisomer 1)
5-{(3R)-1-[1-(1H-Imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Stereoisomer 2)
5-{(3R)-1-[1-(1-Methyl-1H-imidazol-2-yl)ethyl]-5',6'-dihydrospiro[pyrrolidin-3,4'-pyrrolo[1,2-b]pyrazol]-2'-yl}-3-(trifluormethyl)pyridin-2-amin
3-(Trifluormethyl)-5-[(3R)-1-{[4-(trifluormethyl)-1H-imidazol-2-yl]methyl}-5',6'-dihydrospiro[pyrrolidin-3,4'-pyrrolo[1,2-b]pyrazol]-2'-yl]pyridin-2-amin
2-Amino-5-[(rac)-1-(ethylcarbamoyl)-5',6'-dihydrospiro[pyrrolidin-3,4'-pyrrolo[1,2-b]pyrazol]-2'-yl]pyridin-3-carbonsäure
5-[(3S)-1-(Pyridin-2-ylmethyl)-5',6'-dihydrospiro[pyrrolidin-3,4'-pyrrolo[1,2-b]pyrazol]-2'-yl]-3-(trifluormethyl)pyridin-2-amin
5-[(3R)-1-(Pyridin-2-ylmethyl)-5',6'-dihydrospiro[pyrrolidin-3,4'-pyrrolo[1,2-b]pyrazol]-2'-yl]-3-(trifluormethyl)pyridin-2-amin
5-[(3S)-1-(Pyridin-4-ylmethyl)-5',6'-dihydrospiro[pyrrolidin-3,4'-pyrrolo[1,2-b]pyrazol]-2'-yl]-3-(trifluormethyl)pyridin-2-amin
5-[(3R)-1-(Pyridin-4-ylmethyl)-5',6'-dihydrospiro[pyrrolidin-3,4'-pyrrolo[1,2-b]pyrazol]-2'-yl]-3-(trifluormethyl)pyridin-2-amin
5-{ (3R)-1-[3-(Dimethylamino)propyl]-5',6'-dihydrospiro[pyrrolidin-3,4'-pyrrolo[1,2-b]pyrazol]-2'-yl}-3-(trifluormethyl)pyridin-2-amin
5-{ (3R)-1-[2-(Dimethylamino)ethyl]-5',6'-dihydrospiro[pyrrolidin-3,4'-pyrrolo[1,2-b]pyrazol]-2'-yl}-3-(trifluormethyl)pyridin-2-amin
2-Amino-1-{2'-[6-amino-5-(trifluormethyl)pyridin-3-yl]-5',6'-dihydrospiro[pyrrolidin-3,4'-pyrrolo[1,2-b]pyrazol]-1-yl}ethan-1-on (Stereoisomer 2)
2-Amino-1-{2'-[6-amino-5-(trifluormethyl)pyridin-3-yl]-5',6'-dihydrospiro[pyrrolidin-3,4'-pyrrolo[1,2-b]pyrazol]-1-yl}ethan-1-on (Stereoisomer 1)
2-Amino-1-{2-[6-amino-5-(trifluormethyl)pyridin-3-yl]-6,7-dihydrospiro[pyrazolo[5,1-c][1,4]-oxazin-4,3'-pyrrolidin]-1'-yl}ethan-1-on (Stereoisomer 2)
2-Amino-1-{2-[6-amino-5-(trifluormethyl)pyridin-3-yl]-6,7-dihydrospiro[pyrazolo[5,1-c][1,4]-oxazin-4,3'-pyrrolidin]-1'-yl}ethan-1-on (Stereoisomer 1)
2-Amino-1-{2'-[6-amino-5-(trifluormethyl)pyridin-3-yl]-5',6'-dihydrospiro[azetidin-3,4'-pyrrolo[1,2-b]pyrazol]-1-yl}ethan-1-on
(2S)-2-Amino-1-{2'-[6-amino-5-(trifluormethyl)pyridin-3-yl]-5',6'-dihydrospiro[azetidin-3,4'-pyrrolo[1,2-b]pyrazol]-1-yl}Propan-1-on
(2R)-2-Amino-1-{2'-[6-amino-5-(trifluormethyl)pyridin-3-yl]-5',6'-dihydrospiro[azetidin-3,4'-pyrrolo[1,2-b]pyrazol]-1-yl}Propan-1-on
(rac)-2'-[6-Amino-5-(1-methylpiperidin-4-yl)pyridin-3-yl]-N-ethyl-5',6'-dihydrospiro[pyrrolidin-3,4'-pyrrolo[1,2-b]pyrazol]-1-carboxamid
5-{(rac)-1-[(5-Methyl-1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin
2'-{6-Amino-5-[(1R)-1-(2,6-Dichlor-3-fluorphenyl)ethoxy]pyridin-3-yl}-N-ethyl-5',6'-dihydrospiro[pyrrolidin-3,4'-pyrrolo[1,2-b]pyrazol]-1-carboxamid (Isomer 1)
2'-{6-Amino-5-[(1R)-1-(2,6-Dichlor-3-fluorphenyl)ethoxy]pyridin-3-yl}-N-ethyl-5',6'-dihydrospiro[pyrrolidin-3,4'-pyrrolo[1,2-b]pyrazol]-1-carboxamid (Isomer 2)
5-{(3'R)-1'-[(1S)-1-(1H-Imidazol-2-yl)propyl]-6,7-dihydrospiro[pyrazolo[5,1-c][1,4]-oxazin-4,3'-pyrrolidin]-2-yl}-3-(trifluormethyl)pyridin-2-amin
5-{(3'R)-1'-[(1R)-1-(1H-Imidazol-2-yl)propyl]-6,7-dihydrospiro[pyrazolo[5,1-c][1,4]-oxazin-4,3'-pyrrolidin]-2-yl}-3-(trifluormethyl)pyridin-2-amin
5-{(3'R)-1'-[(S)-Cyclopropyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin
5-{(3'R)-1'-[(R)-Cyclopropyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin
5-{(3R)-1-[(S)-Cyclopropyl(1H-imidazol-2-yl)methyl]-5',6'-dihydrospiro[pyrrolidin-3,4'-pyrrolo[1,2-b]pyrazol]-2'-yl}-3-(trifluormethoxy)pyridin-2-amin
5-{(3R)-1-[(R)-Cyclopropyl(1H-imidazol-2-yl)methyl]-5',6'-dihydrospiro[pyrrolidin-3,4'-pyrrolo[1,2-b]pyrazol]-2'-yl}-3-(trifluormethoxy)pyridin-2-amin
5-{(3R)-1-[(1S)-1-(1H-Imidazol-2-yl)propyl]-5',6'-dihydrospiro[pyrrolidin-3,4'-pyrrolo[1,2-b]pyrazol]-2'-yl}-3-(trifluormethoxy)pyridin-2-amin
5-{(3R)-1-[(1R)-1-(1H-Imidazol-2-yl)propyl]-5',6'-dihydrospiro[pyrrolidin-3,4'-pyrrolo[1,2-b]pyrazol]-2'-yl}-3-(trifluormethoxy)pyridin-2-amin
5-{(3R)-1-[(R)-Cyclopropyl(1H-imidazol-2-yl)methyl]-5',6'-dihydrospiro[pyrrolidin-3,4'-pyrrolo[1,2-b]pyrazol]-2'-yl}-3-(difluormethoxy)pyridin-2-amin
5-{(3R)-1-[(S)-Cyclopropyl(1H-imidazol-2-yl)methyl]-5',6'-dihydrospiro[pyrrolidin-3,4'-pyrrolo[1,2-b]pyrazol]-2'-yl}-3-(difluormethoxy)pyridin-2-amin
3-(Difluormethoxy)-5-{(3R)-1-[(1R)-1-(1H-imidazol-2-yl)propyl]-5',6'-dihydrospiro[pyrrolidin-3,4'-pyrrolo[1,2-b]pyrazol]-2'-yl}pyridin-2-amin
3-(Difluormethoxy)-5-{(3R)-1-[(1S)-1-(1H-imidazol-2-yl)propyl]-5',6'-dihydrospiro[pyrrolidin-3,4'-pyrrolo[1,2-b]pyrazol]-2'-yl}pyridin-2-amin
2-Amino-5-{(3R)-1-[(R)-cyclopropyl(1H-imidazol-2-yl)methyl]-5',6'-dihydrospiro[pyrrolidin-3,4'-pyrrolo[1,2-b]pyrazol]-2'-yl}nicotinonitril
2-Amino-5-{(3R)-1-[(S)-cyclopropyl(1H-imidazol-2-yl)methyl]-5',6'-dihydrospiro[pyrrolidin-3,4'-pyrrolo[1,2-b]pyrazol]-2'-yl}nicotinonitril
2-Amino-5-{(3R)-1-[(1R)-1-(1H-imidazol-2-yl)propyl]-5',6'-dihydrospiro[pyrrolidin-3,4'-pyrrolo[1,2-b]pyrazol]-2'-yl}nicotinonitril
2-Amino-5-{(3R)-1-[(1S)-1-(1H-imidazol-2-yl)propyl]-5',6'-dihydrospiro[pyrrolidin-3,4'-pyrrolo[1,2-b]pyrazol]-2'-yl}nicotinonitril
(3R)-2'-(6-Amino-5-{[1-(6-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1)
(3R)-2'-(6-Amino-5-{[1-(6-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 2)
(3R)-2'-(6-Amino-5-{[1-(5-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1)
(3R)-2'-(6-Amino-5-{[1-(5-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 2)
(3R)-2'-(6-Amino-5-{[1-(4-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1)
(3R)-2'-(6-Amino-5-{[1-(4-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 2)
(3R)-2'-(6-Amino-5-{[1-(3-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1)
(3R)-2'-(6-Amino-5-{[1-(3-methylpyridin-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 2)
(3R)-2'-(6-Amino-5-{[1-(1,4-dimethyl-1H-pyrazol-5-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1)
(3R)-2'-(6-Amino-5-{[1-(1,4-dimethyl-1H-pyrazol-5-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 2)
(3R)-2'-(6-Amino-5-{[-1-(1,4-dimethyl-1H-pyrazol-5-yl)ethyl]oxy}pyridin-3-yl)-N-(Propan-2-yl)-5',6'-dihydrospiro[pyrrolidin-3,4'-pyrrolo[1,2-b]pyrazol]-1-carboxamid (Diastereomer 1)
(3R)-2'-(6-Amino-5-{[1-(1,4-dimethyl-1H-pyrazol-5-yl)ethyl]oxy}pyridin-3-yl)-N-(Propan-2-yl)-5',6'-dihydrospiro[pyrrolidin-3,4'-pyrrolo[1,2-b]pyrazol]-1-carboxamid (Diastereomer 2)
(3R)-2'-{6-Amino-5-[(1S)-1-(2,6-difluorphenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1R)-1-(5-fluorpyridin-2-yl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1R)-1-(3-fluorpyridin-2-yl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1R)-1-(2,6-difluorphenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[1-(2-cyanophenyl)ethoxy]pyridin-3-yl}-N-ethyl-5',6'-dihydro-1H-spiro[pyrrolidin-3,4'-pyrrolo[1,2-b]pyrazol]-1-carboxamid (Diastereomer 1 und Diastereomer 2)
(3R)-2'-{6-Amino-5-[1-(2-cyanophenyl)ethoxy]pyridin-3-yl}-N-ethyl-5',6'-dihydro-1H-spiro[pyrrolidin-3,4'-pyrrolo[1,2-b]pyrazol]-1-carboxamid (Diastereomer 1)
(3R)-2'-{6-Amino-5-[1-(2-cyanophenyl)ethoxy]pyridin-3-yl}-N-ethyl-5',6'-dihydro-1H-spiro[pyrrolidin-3,4'-pyrrolo[1,2-b]pyrazol]-1-carboxamid (Diastereomer 2)
(3'R)-2-[6-Amino-5-(trifluormethyl)pyridin-3-yl]-N-[1-(1-ethyl-1H-pyrazol-3-yl)ethyl]-6,7-dihydro-1'H-spiro[pyrazolo[5,1-c][1,4]-oxazin-4,3'-pyrrolidin]-1'-carboxamid (Diastereomer 1 und Diastereomer 2)
(3'R)-2-[6-Amino-5-(trifluormethyl)pyridin-3-yl]-N-[(1-methyl-1H-pyrazol-5-yl)methyl]-6,7-dihydrospiro[pyrazolo[5,1-c][1,4]-oxazin-4,3'-pyrrolidin]-1'-carboxamid
(3'R)-2-[6-Amino-5-(trifluormethyl)pyridin-3-yl]-N-[(1-methyl-1H-pyrazol-4-yl)methyl]-6,7-dihydrospiro[pyrazolo[5,1-c][1,4]-oxazin-4,3'-pyrrolidin]-1'-carboxamid
(3'R)-2-[6-Amino-5-(trifluormethyl)pyridin-3-yl]-N-[1-(1-methyl-1H-pyrazol-5-yl)ethyl]-6,7-dihydro-1'H-spiro[pyrazolo[5,1-c][1,4]-oxazin-4,3'-pyrrolidin]-1'-carboxamid (Diastereomer 1 und Diastereomer 2)
(3'R)-2-[6-Amino-5-(trifluormethyl)pyridin-3-yl]-N-[1-(1-methyl-1H-pyrazol-5-yl)ethyl]-6,7-dihydro-1'H-spiro[pyrazolo[5,1-c][1,4]-oxazin-4,3'-pyrrolidin]-1'-carboxamid (Diastereomer 1)
(3'R)-2-[6-Amino-5-(trifluormethyl)pyridin-3-yl]-N-[1-(1-methyl-1H-pyrazol-5-yl)ethyl]-6,7-dihydro-1'H-spiro[pyrazolo[5,1-c][1,4]-oxazin-4,3'-pyrrolidin]-1'-carboxamid (Diastereomer 2)
(3'R)-2-[6-Amino-5-(trifluormethyl)pyridin-3-yl]-N-[1-(1,3-dimethyl-1H-pyrazol-4-yl)ethyl]-6,7-dihydro-1'H-spiro[pyrazolo[5,1-c][1,4]-oxazin-4,3'-pyrrolidin]-1'-carboxamid (Diastereomer 1 und Diastereomer 2)
(3'R)-2-[6-Amino-5-(trifluormethyl)pyridin-3-yl]-N-[1-(1,3-dimethyl-1H-pyrazol-4-yl)ethyl]-6,7-dihydro-1'H-spiro[pyrazolo[5,1-c][1,4]-oxazin-4,3'-pyrrolidin]-1'-carboxamid (Diastereomer 1)
(3'R)-2-[6-Amino-5-(trifluormethyl)pyridin-3-yl]-N-[1-(1,3-dimethyl-1H-pyrazol-4-yl)ethyl]-6,7-dihydro-1'H-spiro[pyrazolo[5,1-c][1,4]-oxazin-4,3'-pyrrolidin]-1'-carboxamid (Diastereomer 2)
(3R)-2'-{6-Amino-5-[(1R)-1-(3-cyanophenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1R)-1-(4-cyanophenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1R)-1-(3,5-difluorpyridin-4-yl)ethoxy]pyridin-3-yl}-N-ethyl-5',6'-dihydrospiro[pyrrolidin-3,4'-pyrrolo[1,2-b]pyrazol]-1-carboxamid
(3R)-2'-[6-Amino-5-(1-phenylpropoxy)pyridin-3-yl]-N-ethyl-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(1-phenylpropoxy)pyridin-3-yl]-N-ethyl-5',6'-dihydro-1H-spiro[pyrrolidin-3,4'-pyrrolo[1,2-b]pyrazol]-1-carboxamid (Diastereomer 1)
(3R)-2'-[6-Amino-5-(1-phenylpropoxy)pyridin-3-yl]-N-ethyl-5',6'-dihydro-1H-spiro[pyrrolidin-3,4'-pyrrolo[1,2-b]pyrazol]-1-carboxamid (Diastereomer 2)
(3R)-2'-{6-Amino-5-[(1S)-1-(3-cyanophenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1S)-1-(4-cyanophenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1S)-1-(3,5-difluorpyridin-4-yl)ethoxy]pyridin-3-yl}-N-ethyl-5',6'-dihydrospiro[pyrrolidin-3,4'-pyrrolo[1,2-b]pyrazol]-1-carboxamid
(3'R)-2-[6-Amino-5-(trifluormethyl)pyridin-3-yl]-N-[1-(1,3,4-trimethyl-1H-pyrazol-5-yl)ethyl]-6,7-dihydro-1'H-spiro[pyrazolo[5,1-c][1,4]oxazin-4,3'-pyrrolidin]-1'-carboxamid (Diastereomer 1 und Diastereomer 2)
(3'R)-2-[6-Amino-5-(trifluormethyl)pyridin-3-yl]-N-[1-(1,3,4-trimethyl-1H-pyrazol-5-yl)ethyl]-6,7-dihydro-1'H-spiro[pyrazolo[5,1-c][1,4]-oxazin-4,3'-pyrrolidin]-1'-carboxamid (Diastereomer 1)
(3'R)-2-[6-Amino-5-(trifluormethyl)pyridin-3-yl]-N-[1-(1,3,4-trimethyl-1H-pyrazol-5-yl)ethyl]-6,7-dihydro-1'H-spiro[pyrazolo[5,1-c][1,4]-oxazin-4,3'-pyrrolidin]-1'-carboxamid (Diastereomer 2)
(3'R)-2-[6-Amino-5-(trifluormethyl)pyridin-3-yl]-N-[1-(1,5-dimethyl-1H-pyrazol-4-yl)ethyl]-6,7-dihydro-1'H-spiro[pyrazolo[5,1-c][1,4]-oxazin-4,3'-pyrrolidin]-1'-carboxamid (Diastereomer 1 und Diastereomer 2)
(3'R)-2-[6-Amino-5-(trifluormethyl)pyridin-3-yl]-N-[1-(1-methyl-1H-pyrazol-4-yl)ethyl]-6,7-dihydro-1'H-spiro[pyrazolo[5,1-c][1,4]-oxazin-4,3'-pyrrolidin]-1'-carboxamid (Diastereomer 1 und Diastereomer 2)
(3R)-2'-{6-Amino-5-[(1R)-1-(3-fluorpyridin-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[(1R)-1-(3-fluorpyridin-2-yl)ethoxy]pyridin-3-yl}-N-cyclobutyl-5',6'-dihydrospiro[pyrrolidin-3,4'-pyrrolo[1,2-b]pyrazol]-1-carboxamid
(3R)-2'-{6-Amino-5-[(1R)-1-(3,5-difluorpyridin-4-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[(1R)-1-(3,5-difluorpyridin-4-yl)ethoxy]pyridin-3-yl}-N-cyclobutyl-5',6'-dihydrospiro[pyrrolidin-3,4'-pyrrolo[1,2-b]pyrazol]-1-carboxamid
(3R)-2'-{6-Amino-5-[(1R)-1-phenylethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[1-(1-methyl-1H-pyrazol-5-yl)ethoxy]pyridin-3-yl}-N-isopropyl-5',6'-dihydro-1H-spiro[pyrrolidin-3,4'-pyrrolo[1,2-b]pyrazol]-1-carboxamid (Diastereomer 1)
(3R)-2'-{6-Amino-5-[1-(1-methyl-1H-pyrazol-5-yl)ethoxy]pyridin-3-yl}-N-isopropyl-5',6'-dihydro-1H-spiro[pyrrolidin-3,4'-pyrrolo[1,2-b]pyrazol]-1-carboxamid (Diastereomer 2)
(3R)-2'-{6-Amino-5-[1-(1-methyl-1H-pyrazol-5-yl)ethoxy]pyridin-3-yl}-N-cyclobutyl-5',6'-dihydro-1H-spiro[pyrrolidin-3,4'-pyrrolo[1,2-b]pyrazol]-1-carboxamid (Diastereomer 1)
(3R)-2'-{6-Amino-5-[1-(1-methyl-1H-pyrazol-5-yl)ethoxy]pyridin-3-yl}-N-cyclobutyl-5',6'-dihydro-1H-spiro[pyrrolidin-3,4'-pyrrolo[1,2-b]pyrazol]-1-carboxamid (Diastereomer 2)
(3R)-2'-{6-Amino-5-[(1R)-1-(2-fluorphenyl)ethoxy]pyridin-3-yl}-N-(Propan-2-yl)-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-(1-methyl-1H-pyrazol-5-yl)ethyl]-5',6'-dihydro-1H-spiro[azetidin-3,4'-pyrrolo[1,2-b]pyrazol]-1-carboxamid (Enantiomer 1 und Enantiomer 2)
2'-[6-Amino-5-(trifluormethyl)pyridin-3-yl]-N-[1-(1-methyl-1H-pyrazol-5-yl)ethyl]-5',6'-dihydro-1H-spiro[azetidin-3,4'-pyrrolo[1,2-b]pyrazol]-1-carboxamid (Enantiomer 1)
2'-[6-Amino-5-(trifluormethyl)pyridin-3-yl]-N-[1-(1-methyl-1H-pyrazol-5-yl)ethyl]-5',6'-dihydro-1H-spiro[azetidin-3,4'-pyrrolo[1,2-b]pyrazol]-1-carboxamid (Enantiomer 2)
2'-[6-Amino-5-(trifluormethyl)pyridin-3-yl]-N-[1-(1,3-dimethyl-1H-pyrazol-4-yl)ethyl]-5',6'-dihydro-1H-spiro[azetidin-3,4'-pyrrolo[1,2-b]pyrazol]-1-carboxamid (Enantiomer 1 und Enantiomer 2)
2'-[6-Amino-5-(trifluormethyl)pyridin-3-yl]-N-[1-(1,3-dimethyl-1H-pyrazol-4-yl)ethyl]-5',6'-dihydro-1H-spiro[azetidin-3,4'-pyrrolo[1,2-b]pyrazol]-1-carboxamid (Enantiomer 1)
2'-[6-Amino-5-(trifluormethyl)pyridin-3-yl]-N-[1-(1,3-dimethyl-1H-pyrazol-4-yl)ethyl]-5',6'-dihydro-1H-spiro[azetidin-3,4'-pyrrolo[1,2-b]pyrazol]-1-carboxamid (Enantiomer 2)
(3S)-2'-[6-Amino-5-(trifluormethyl)pyridin-3-yl]-N-[1-(1-methyl-1H-pyrazol-5-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3S)-2'-[6-Amino-5-(trifluormethyl)pyridin-3-yl]-N-[1-(1,3-dimethyl-1H-pyrazol-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-{6-Amino-5-[(1R)-1-(2,6-difluorphenyl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-(6-Amino-5-{methyl[(1R)-1-phenylethyl]amino}pyridin-3-yl)-N-ethyl-5',6'-dihydrospiro[pyrrolidin-3,4'-pyrrolo[1,2-b]pyrazol]-1-carboxamid
2'-[6-Amino-5-(trifluormethyl)pyridin-3-yl]-N-[1-(1,3,5-trimethyl-1H-pyrazol-4-yl)ethyl]-5',6'-dihydro-1H-spiro[azetidin-3,4'-pyrrolo[1,2-b]pyrazol]-1-carboxamid (Diastereomer 1 und Diastereomer 2)
2'-[6-Amino-5-(trifluormethyl)pyridin-3-yl]-N-[1-(1,3,5-trimethyl-1H-pyrazol-4-yl)ethyl]-5',6'-dihydro-1H-spiro[azetidin-3,4'-pyrrolo[1,2-b]pyrazol]-1-carboxamid (Diastereomer 1)
2'-[6-Amino-5-(trifluormethyl)pyridin-3-yl]-N-[1-(1,3,5-trimethyl-1H-pyrazol-4-yl)ethyl]-5',6'-dihydro-1H-spiro[azetidin-3,4'-pyrrolo[1,2-b]pyrazol]-1-carboxamid (Diastereomer 2)
(3R)-2'-{6-Amino-5-[(1R)-1-(4-methylpyridin-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[(1R)-1-(4-methylpyridin-2-yl)ethoxy]pyridin-3-yl}-N-cyclobutyl-5',6'-dihydrospiro[pyrrolidin-3,4'-pyrrolo[1,2-b]pyrazol]-1-carboxamid
(3R)-2'-{6-Amino-5-[(1R)-1-(6-methylpyridin-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[(1R)-1-(6-methylpyridin-2-yl)ethoxy]pyridin-3-yl}-N-cyclobutyl-5',6'-dihydrospiro[pyrrolidin-3,4'-pyrrolo[1,2-b]pyrazol]-1-carboxamid
(3S)-2'-[6-Amino-5-(trifluormethyl)pyridin-3-yl]-N-[2-(3-fluorpyridin-2-yl)propan-2-yl]-5',6'-dihydrospiro[pyrrolidin-3,4'-pyrrolo[1,2-b]pyrazol]-1-carboxamid
(3S)-2'-[6-Amino-5-(trifluormethyl)pyridin-3-yl]-N-[1-(1,3,5-trimethyl-1H-pyrazol-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[1-(1,3,5-trimethyl-1H-pyrazol-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[1-(1,3,5-trimethyl-1H-pyrazol-4-yl)ethyl]-5',6'-dihydro-1H-spiro[pyrrolidin-3,4'-pyrrolo[1,2-b]pyrazol]-1-carboxamid (Diastereomer 1)
(3R)-2'-[6-Amino-5-(trifluormethyl)pyridin-3-yl]-N-[1-(1,3,5-trimethyl-1H-pyrazol-4-yl)ethyl]-5',6'-dihydro-1H-spiro[pyrrolidin-3,4'-pyrrolo[1,2-b]pyrazol]-1-carboxamid (Diastereomer 2)
(2'-{6-Amino-5-[(1R)-1-phenylethoxy]pyridin-3-yl}-5',6'-dihydrospiro[azetidin-3,4'-pyrrolo[1,2-b]pyrazol]-1-yl)(cyclopropyl)methanon
2'-[6-Amino-5-(trifluormethyl)pyridin-3-yl]-N-[1-(1-ethyl-1H-pyrazol-3-yl)ethyl]-5',6'-dihydro-1H-spiro[azetidin-3,4'-pyrrolo[1,2-b]pyrazol]-1-carboxamid (Enantiomer 1)
2'-[6-Amino-5-(trifluormethyl)pyridin-3-yl]-N-[1-(1-ethyl-1H-pyrazol-3-yl)ethyl]-5',6'-dihydro-1H-spiro[azetidin-3,4'-pyrrolo[1,2-b]pyrazol]-1-carboxamid (Enantiomer 2)
(3S)-2'-[6-Amino-5-(trifluormethyl)pyridin-3-yl]-N-[1-(1-ethyl-1H-pyrazol-3-yl)ethyl]-5',6'-dihydro-1H-spiro[pyrrolidin-3,4'-pyrrolo[1,2-b]pyrazol]-1-carboxamid (Diastereomer 1)
(3S)-2'-[6-Amino-5-(trifluormethyl)pyridin-3-yl]-N-[1-(1-ethyl-1H-pyrazol-3-yl)ethyl]-5',6'-dihydro-1H-spiro[pyrrolidin-3,4'-pyrrolo[1,2-b]pyrazol]-1-carboxamid (Diastereomer 2)
5-{(3'R)-1'-[Cyclopropyl(1H-1,2,4-triazol-5-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3'R)-1'-[Cyclopropyl(1H-1,2,4-triazol-5-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[Cyclopropyl(1H-1,2,4-triazol-5-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
5-{(3R)-1-[1-(1H-1,2,4-Triazol-5-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(1H-1,2,4-Triazol-5-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(1H-1,2,4-Triazol-5-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3'R)-1'-[1-(1H-1,2,4-Triazol-5-yl)propyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3'R)-1'-[1-(1H-1,2,4-Triazol-5-yl)propyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[1-(1H-1,2,4-Triazol-5-yl)propyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
5-{(3'R)-1'-[2,2-Difluor-1-(1H-imidazol-2-yl)ethyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
5-{(3'R)-1'-[2,2-Difluor-1-(1H-imidazol-2-yl)ethyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(difluormethyl)pyridin-2-amin (Diastereomer 1 und Diastereomer 2)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(difluormethyl)pyridin-2-amin (Diastereomer 1)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(difluormethyl)pyridin-2-amin (Diastereomer 2)
2-Amino-5-{(3'R)-1'-[cyclopropyl(1H-imidazol-2-yl)methyl]-6,7-dihydrospiro[pyrazolo[5,1-c][1,4]-oxazin-4,3'-pyrrolidin]-2-yl}Nicotinonitril (Diastereomer 1 und Diastereomer 2)
2-Amino-5-{(3'R)-1'-[cyclopropyl(1H-imidazol-2-yl)methyl]-6,7-dihydrospiro[pyrazolo[5,1-c][1,4]-oxazin-4,3'-pyrrolidin]-2-yl}Nicotinonitril (Diastereomer 1)
2-Amino-5-{(3'R)-1'-[cyclopropyl(1H-imidazol-2-yl)methyl]-6,7-dihydrospiro[pyrazolo[5,1-c][1,4]-oxazin-4,3'-pyrrolidin]-2-yl}Nicotinonitril (Diastereomer 2)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(difluormethoxy)pyridin-2-amin (Diastereomer 1 und Diastereomer 2)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(difluormethoxy)pyridin-2-amin (Diastereomer 1)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(difluormethoxy)pyridin-2-amin (Diastereomer 2)
5-{1-[1-(1H-Imidazol-2-yl)propyl]-5',6'-dihydrospiro[azetidin-3,4'-pyrrolo[1,2-b]pyrazol]-2'-yl}-3-(trifluormethyl)pyridin-2-amin (Enantiomer 1 und Enantiomer 2)
5-{1-[1-(1H-Imidazol-2-yl)propyl]-5',6'-dihydrospiro[azetidin-3,4'-pyrrolo[1,2-b]pyrazol]-2'-yl}-3-(trifluormethyl)pyridin-2-amin (Enantiomer 1)
5-{1-[1-(1H-Imidazol-2-yl)propyl]-5',6'-dihydrospiro[azetidin-3,4'-pyrrolo[1,2-b]pyrazol]-2'-yl}-3-(trifluormethyl)pyridin-2-amin (Enantiomer 2)
5-{(3'R)-1'-[Cyclobutyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3'R)-1'-[Cyclobutyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[Cyclobutyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
5-{(3'R)-1'-[1-(1H-Imidazol-2-yl)-2-methylpropyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3'R)-1'-[1-(1H-Imidazol-2-yl)-2-methylpropyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[1-(1H-Imidazol-2-yl)-2-methylpropyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
5-{(3R)-1-[Cyclobutyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[Cyclobutyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[Cyclobutyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[1-(1H-Imidazol-2-yl)-2,2-dimethylpropyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{1-[Cyclopropyl(1H-imidazol-2-yl)methyl]-5',6'-dihydrospiro[azetidin-3,4'-pyrrolo[1,2-b]pyrazol]-2'-yl}-3-(trifluormethyl)pyridin-2-amin (Enantiomer 1 und Enantiomer 2)
5-{1-[Cyclopropyl(1H-imidazol-2-yl)methyl]-5',6'-dihydrospiro[azetidin-3,4'-pyrrolo[1,2-b]pyrazol]-2'-yl}-3-(trifluormethyl)pyridin-2-amin (Enantiomer 1)
5-{1-[Cyclopropyl(1H-imidazol-2-yl)methyl]-5',6'-dihydrospiro[azetidin-3,4'-pyrrolo[1,2-b]pyrazol]-2'-yl}-3-(trifluormethyl)pyridin-2-amin (Enantiomer 2)
5-{(3R)-1-[2,2-Difluor-1-(1H-imidazol-2-yl)ethyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[2,2-Difluor-1-(1H-imidazol-2-yl)ethyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[2,2-Difluor-1-(1H-imidazol-2-yl)ethyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3S)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3S)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3S)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
2-Amino-5-{(3R)-1-[cyclopropyl(1H-imidazol-2-yl)methyl]-5',6'-dihydrospiro[pyrrolidin-3,4'-pyrrolo[1,2-b]pyrazol]-2'-yl}Nicotinonitril (Diastereomer 1 und Diastereomer 2)
2-Amino-5-{(3R)-1-[cyclopropyl(1H-imidazol-2-yl)methyl]-5',6'-dihydrospiro[pyrrolidin-3,4'-pyrrolo[1,2-b]pyrazol]-2'-yl}Nicotinonitril (Diastereomer 1)
2-Amino-5-{(3R)-1-[cyclopropyl(1H-imidazol-2-yl)methyl]-5',6'-dihydrospiro[pyrrolidin-3,4'-pyrrolo[1,2-b]pyrazol]-2'-yl}Nicotinonitril (Diastereomer 2)
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(difluormethoxy)pyridin-2-amin (Diastereomer 1 und Diastereomer 2)
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(difluormethoxy)pyridin-2-amin (Diastereomer 1)
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(difluormethoxy)pyridin-2-amin (Diastereomer 2)
2-Amino-5-{(3R)-1-[1-(1H-imidazol-2-yl)propyl]-5',6'-dihydrospiro[pyrrolidin-3,4'-pyrrolo[1,2-b]pyrazol]-2'-yl}Nicotinonitril (Diastereomer 1 und Diastereomer 2)
2-Amino-5-{(3R)-1-[1-(1H-imidazol-2-yl)propyl]-5',6'-dihydrospiro[pyrrolidin-3,4'-pyrrolo[1,2-b]pyrazol]-2'-yl}Nicotinonitril (Diastereomer 1)
2-Amino-5-{(3R)-1-[1-(1H-imidazol-2-yl)propyl]-5',6'-dihydrospiro[pyrrolidin-3,4'-pyrrolo[1,2-b]pyrazol]-2'-yl}Nicotinonitril (Diastereomer 2)
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethoxy)pyridin-2-amin (Diastereomer 1 und Diastereomer 2)
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethoxy)pyridin-2-amin (Diastereomer 1)
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethoxy)pyridin-2-amin (Diastereomer 2)
3-(Difluormethoxy)-5-{(3R)-1-[1-(1H-imidazol-2-yl)propyl]-5',6'-dihydrospiro[pyrrolidin-3,4'-pyrrolo[1,2-b]pyrazol]-2'-yl}pyridin-2-amin (Diastereomer 1 und Diastereomer 2)
3-(Difluormethoxy)-5-{(3R)-1-[1-(1H-imidazol-2-yl)propyl]-5',6'-dihydrospiro[pyrrolidin-3,4'-pyrrolo[1,2-b]pyrazol]-2'-yl}pyridin-2-amin (Diastereomer 1)
3-(Difluormethoxy)-5-{(3R)-1-[1-(1H-imidazol-2-yl)propyl]-5',6'-dihydrospiro[pyrrolidin-3,4'-pyrrolo[1,2-b]pyrazol]-2'-yl}pyridin-2-amin (Diastereomer 2)
5-{(3R)-1-[1-(1H-Imidazol-2-yl)propyl]-5',6'-dihydrospiro[pyrrolidin-3,4'-pyrrolo[1,2-b]pyrazol]-2'-yl}-3-(trifluormethoxy)pyridin-2-amin (Diastereomer 1 und Diastereomer 2)
5-{(3R)-1-[1-(1H-Imidazol-2-yl)propyl]-5',6'-dihydrospiro[pyrrolidin-3,4'-pyrrolo[1,2-b]pyrazol]-2'-yl}-3-(trifluormethoxy)pyridin-2-amin (Diastereomer 1)
5-{(3R)-1-[1-(1H-Imidazol-2-yl)propyl]-5',6'-dihydrospiro[pyrrolidin-3,4'-pyrrolo[1,2-b]pyrazol]-2'-yl}-3-(trifluormethoxy)pyridin-2-amin (Diastereomer 2)
5-{(3R)-1-[1-(1H-Imidazol-2-yl)-2-methylpropyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(1H-Imidazol-2-yl)-2-methylpropyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(1H-Imidazol-2-yl)-2-methylpropyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3S)-1-[1-(1H-Imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3S)-1-[1-(1H-Imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3S)-1-[1-(1H-Imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3'R)-1'-[1-(1H-Imidazol-2-yl)propyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3'R)-1'-[1-(1H-Imidazol-2-yl)propyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[1-(1H-Imidazol-2-yl)propyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
{(3R)-2'-[6-Amino-5-(trifluormethyl)pyridin-3-yl]-5',6'-dihydrospiro[pyrrolidin-3,4'-pyrrolo[1,2-b]pyrazol]-1-yl}(1H-imidazol-2-yl)methanon
{(3R)-2'-[6-Amino-5-(trifluormethyl)pyridin-3-yl]-5',6'-dihydrospiro[pyrrolidin-3,4'-pyrrolo[1,2-b]pyrazol]-1-yl}(1-methyl-1H-imidazol-2-yl)methanon
3-[(1R)-1-(2,6-Dichlor-3-fluorphenyl)ethoxy]-5-{(3R)-1-[(1H-imidazol-2-yl)methyl]-5',6'-dihydrospiro[pyrrolidin-3,4'-pyrrolo[1,2-b]pyrazol]-2'-yl}pyridin-2-amin
5-{(3R)-1-[1-(1H-Imidazol-2-yl)ethyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(1H-Imidazol-2-yl)ethyl]-5',6'-dihydrospiro[pyrrolidin-3,4'-pyrrolo[1,2-b]pyrazol]-2'-yl}-3-(trifluormethyl)pyridin-2-amin (Diastereomer 2)
(3R)-2'-[6-Amino-5-(trifluormethyl)pyridin-3-yl]-N-[1-(3-chlorpyridin-4-yl)cyclobutyl]-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[2-(3-chlorpyridin-4-yl)propan-2-yl]-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[1-(pyridin-4-yl)cyclobutyl]-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[1-(pyridin-3-yl)cyclobutyl]-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[(1S)-1-(3-chlorpyridin-4-yl)ethyl]-5',6'-dihydrospiro[pyrrolidin-3,4'-pyrrolo[1,2-b]pyrazol]-1-carboxamid
2'-(6-Amino-5-cyanopyridin-3-yl)-N-[1-(pyridin-4-yl)cyclobutyl]-5',6'-dihydrospiro[pyrrolidin-3,4'-pyrrolo[1,2-b]pyrazol]-1-carboxamid (Enantiomer 2)
(3R)-2'-[6-Amino-5-(trifluormethyl)pyridin-3-yl]-N-[1-(3-methylpyridin-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[cyclopropyl(phenyl)methyl]-5',6'-dihydrospiro[pyrrolidin-3,4'-pyrrolo[1,2-b]pyrazol]-1-carboxamid (Diastereomer 1)
(3R)-2'-[6-Amino-5-(trifluormethyl)pyridin-3-yl]-N-[cyclopropyl(phenyl)methyl]-5',6'-dihydrospiro[pyrrolidin-3,4'-pyrrolo[1,2-b]pyrazol]-1-carboxamid (Diastereomer 2)
(3R)-2'-[6-Amino-5-(trifluormethyl)pyridin-3-yl]-N-[1-(pyridin-2-yl)cyclobutyl]-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[2-methyl-1-(pyridin-4-yl)propyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[2-methyl-1-(pyridin-4-yl)propyl]-5',6'-dihydro-1H-spiro[pyrrolidin-3,4'-pyrrolo[1,2-b]pyrazol]-1-carboxamid (Diastereomer 1)
(3R)-2'-[6-Amino-5-(trifluormethyl)pyridin-3-yl]-N-[2-methyl-1-(pyridin-4-yl)propyl]-5',6'-dihydro-1H-spiro[pyrrolidin-3,4'-pyrrolo[1,2-b]pyrazol]-1-carboxamid (Diastereomer 2)
(3R)-2'-[6-Amino-5-(trifluormethyl)pyridin-3-yl]-N-[1-(3-fluorpyridin-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[1-(3-fluorpyridin-4-yl)ethyl]-5',6'-dihydro-1H-spiro[pyrrolidin-3,4'-pyrrolo[1,2-b]pyrazol]-1-carboxamid (Diastereomer 1)
(3R)-2'-[6-Amino-5-(trifluormethyl)pyridin-3-yl]-N-[1-(3-fluorpyridin-4-yl)ethyl]-5',6'-dihydro-1H-spiro[pyrrolidin-3,4'-pyrrolo[1,2-b]pyrazol]-1-carboxamid (Diastereomer 2)
(3R)-2'-[6-Amino-5-(trifluormethyl)pyridin-3-yl]-N-[(1R)-1-(3-chlorpyridin-4-yl)ethyl]-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[1-(2-methylpyridin-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[1-(3-methoxypyridin-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[1-(pyridin-4-yl)cyclopropyl]-5',6'-dihydrospiro[pyrrolidin-3,4'-pyrrolo[1,2-b]pyrazol]-1-carboxamid
(3R)-2'-{6-Amino-5-[(1R)-1-(2-methoxyphenyl)ethoxy]pyridin-3-yl}-N-ethyl-5',6'-dihydrospiro[pyrrolidin-3,4'-pyrrolo[1,2-b]pyrazol]-1-carboxamid
3-(1-{(3R)-2'-[6-Amino-5-(trifluormethyl)pyridin-3-yl]-5',6'-dihydro-1H-spiro[pyrrolidin-3,4'-pyrrolo[1,2-b]pyrazol]-1-yl}propyl)benzonitril (Diastereomer 1 und Diastereomer 2)
3-(1-{(3R)-2'-[6-Amino-5-(trifluormethyl)pyridin-3-yl]-5',6'-dihydro-1H-spiro[pyrrolidin-3,4'-pyrrolo[1,2-b]pyrazol]-1-yl}propyl)benzonitril (Diastereomer 1)
3-(1-{(3R)-2'-[6-Amino-5-(trifluormethyl)pyridin-3-yl]-5',6'-dihydro-1H-spiro[pyrrolidin-3,4'-pyrrolo[1,2-b]pyrazol]-1-yl}propyl)benzonitril (Diastereomer 2)
(3S)-2'-(6-Amino-5-cyanopyridin-3-yl)-N-[2-(3-chlorpyridin-4-yl)propan-2-yl]-5',6'-dihydrospiro[pyrrolidin-3,4'-pyrrolo[1,2-b]pyrazol]-1-carboxamid (Enantiomer 1)
(3R)-2'-(6-Amino-5-cyanopyridin-3-yl)-N-[2-(3-chlorpyridin-4-yl)propan-2-yl]-5',6'-dihydrospiro[pyrrolidin-3,4'-pyrrolo[1,2-b]pyrazol]-1-carboxamid (Enantiomer 2)
(3R)-2'-{6-Amino-5-[(1R)-1-(3-chlorphenyl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[1-(2,6-dichlorphenyl)ethoxy]pyridin-3-yl}-N-ethyl-5',6'-dihydro-1H-spiro[pyrrolidin-3,4'-pyrrolo[1,2-b]pyrazol]-1-carboxamid (Diastereomer 1 und Diastereomer 2)
(3R)-2'-{6-Amino-5-[1-(2,6-dichlorphenyl)ethoxy]pyridin-3-yl}-N-ethyl-5',6'-dihydro-1H-spiro[pyrrolidin-3,4'-pyrrolo[1,2-b]pyrazol]-1-carboxamid (Diastereomer 1)
(3R)-2'-{6-Amino-5-[1-(2,6-dichlorphenyl)ethoxy]pyridin-3-yl}-N-ethyl-5',6'-dihydro-1H-spiro[pyrrolidin-3,4'-pyrrolo[1,2-b]pyrazol]-1-carboxamid (Diastereomer 2)
5-{(3R)-1-[1-(1H-Pyrrol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
(3R)-2'-[6-Amino-5-(trifluormethyl)pyridin-3-yl]-N-[1-(2-methoxypyridin-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-[1-(1-methyl-1H-pyrazol-5-yl)ethyl]-5',6'-dihydrospiro[pyrrolidin-3,4'-pyrrolo[1,2-b]pyrazol]-1-carboxamid (Diastereomer 1)
(3R)-2'-[6-Amino-5-(trifluormethyl)pyridin-3-yl]-N-[1-(1-methyl-1H-pyrazol-5-yl)ethyl]-5',6'-dihydrospiro[pyrrolidin-3,4'-pyrrolo[1,2-b]pyrazol]-1-carboxamid (Diastereomer 2)
3-(1-{(3R)-2'-[6-Amino-5-(trifluormethyl)pyridin-3-yl]-5',6'-dihydro-1H-spiro[pyrrolidin-3,4'-pyrrolo[1,2-b]pyrazol]-1-yl}propyl)benzamid (Diastereomer 1 und Diastereomer 2)
3-(1-{(3R)-2'-[6-Amino-5-(trifluormethyl)pyridin-3-yl]-5',6'-dihydrospiro[pyrrolidin-3,4'-pyrrolo[1,2-b]pyrazol]-1-yl}propyl)benzamid (Diastereomer 1)
3-(1-{(3R)-2'-[6-Amino-5-(trifluormethyl)pyridin-3-yl]-5',6'-dihydrospiro[pyrrolidin-3,4'-pyrrolo[1,2-b]pyrazol]-1-yl}propyl)benzamid (Diastereomer 2)
(3R)-2'-{6-Amino-5-[(1R)-1-(1,3-thiazol-2-yl)ethoxy]pyridin-3-yl}-N-cyclobutyl-5',6'-dihydrospiro[pyrrolidin-3,4'-pyrrolo[1,2-b]pyrazol]-1-carboxamid
(3R)-2'-{6-Amino-5-[(1R)-1-(3-chlorphenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[1-(2-Chlor-3-fluorpyridin-4-yl)ethyl]-5',6'-dihydro-1H-spiro[pyrrolidin-3,4'-pyrrolo[1,2-b]pyrazol]-1-carboxamid (Diastereomer 1 und Diastereomer 2)
(3R)-2'-[6-Amino-5-(trifluormethyl)pyridin-3-yl]-N-{[3-(methylsulfanyl)pyridin-4-yl]methyl}-5',6'-dihydrospiro[pyrrolidin-3,4'-pyrrolo[1,2-b]pyrazol]-1-carboxamid
(3R)-2'-{6-Amino-5-[(1R)-1-(1,3-thiazol-2-yl)ethoxy]pyridin-3-yl}-N-ethyl-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[1-(1,3-dimethyl-1H-pyrazol-4-yl)ethyl]-5',6'-dihydrospiro[pyrrolidin-3,4'-pyrrolo[1,2-b]pyrazol]-1-carboxamid (Diastereomer 1)
(3R)-2'-[6-Amino-5-(trifluormethyl)pyridin-3-yl]-N-[1-(1,3-dimethyl-1H-pyrazol-4-yl)ethyl]-5',6'-dihydrospiro[pyrrolidin-3,4'-pyrrolo[1,2-b]pyrazol]-1-carboxamid (Diastereomer 2)
(3R)-2'-{6-Amino-5-[(1R)-1-(3-fluorphenyl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[1-(pyridin-2-yl)cyclopropyl]-5',6'-dihydrospiro[pyrrolidin-3,4'-pyrrolo[1,2-b]pyrazol]-1-carboxamid
5-{(3R)-1-[1-(Pyrimidin-5-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(Pyrimidin-5-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(Pyrimidin-5-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
(3R)-2'-{6-Amino-5-[(1R)-1-(1,3-thiazol-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-[6-Amino-5-(trifluormethyl)pyridin-3-yl]-N-[(3-methylpyridin-4-yl)methyl]-5',6'-dihydrospiro[pyrrolidin-3,4'-pyrrolo[1,2-b]pyrazol]-1-carboxamid
5-{(3R)-1-[1-(6-Methoxypyridin-2-yl)ethyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(6-Methoxypyridin-2-yl)ethyl]-5',6'-dihydrospiro[pyrrolidin-3,4'-pyrrolo[1,2-b]pyrazol]-2'-yl}-3-(trifluormethyl)pyridin-2-amin (Diastereomer 2)
(3R)-2'-(6-Amino-5-{1-[3-(3-hydroxy-3-methylbut-1-in-1-yl)phenyl]ethoxy}pyridin-3-yl)-N-ethyl-5',6'-dihydro-1H-spiro[pyrrolidin-3,4'-pyrrolo[1,2-b]pyrazol]-1-carboxamid (Diastereomer 1 und Diastereomer 2)
(3R)-2'-{6-Amino-5-[(1R)-1-(pyridin-3-yl)ethoxy]pyridin-3-yl}-N-cyclobutyl-5',6'-dihydrospiro[pyrrolidin-3,4'-pyrrolo[1,2-b]pyrazol]-1-carboxamid
(3R)-2'-{6-Amino-5-[1-(1,3-oxazol-2-yl)ethoxy]pyridin-3-yl}-N-ethyl-5',6'-dihydro-1H-spiro[pyrrolidin-3,4'-pyrrolo[1,2-b]pyrazol]-1-carboxamid (Diastereomer 1 und Diastereomer 2)
(3R)-2'-{6-Amino-5-[(1R)-1-(3-fluorphenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-[6-Amino-5-(6,7-dihydro-5H-cyclopenta[b]pyridin-7-yloxy)pyridin-3-yl]-N-ethyl-5',6'-dihydro-1H-spiro[pyrrolidin-3,4'-pyrrolo[1,2-b]pyrazol]-1-carboxamid (Diastereomer 2)
5-{(3R)-1-[Cyclopropyl(5-fluorpyridin-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
(3R)-2'-{6-Amino-5-[(1R)-1-(pyrimidin-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[(1R)-1-(4-fluorphenyl)ethoxy]pyridin-3-yl}-N-ethyl-5',6'-dihydrospiro[pyrrolidin-3,4'-pyrrolo[1,2-b]pyrazol]-1-carboxamid
(3R)-2'-{6-Amino-5-[(1R)-1-(4-fluorphenyl)ethoxy]pyridin-3-yl}-N-(Propan-2-yl)-5',6'-dihydrospiro[pyrrolidin-3,4'-pyrrolo[1,2-b]pyrazol]-1-carboxamid
5-{(3R)-1-[1-(1,3-Thiazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(1,3-Thiazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(1,3-Thiazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
(3R)-2'-{6-Amino-5-[(1R)-1-(4-chlorphenyl)ethoxy]pyridin-3-yl}-N-ethyl-5',6'-dihydrospiro[pyrrolidin-3,4'-pyrrolo[1,2-b]pyrazol]-1-carboxamid
5-{(3R)-1-[1-(Dimethylamino)Propan-2-yl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
(3R)-2'-{6-Amino-5-[(1R)-1-(1,2-thiazol-5-yl)ethoxy]pyridin-3-yl}-N-ethyl-5',6'-dihydrospiro[pyrrolidin-3,4'-pyrrolo[1,2-b]pyrazol]-1-carboxamid
6-({(3R)-2'-[6-Amino-5-(trifluormethyl)pyridin-3-yl]-5',6'-dihydrospiro[pyrrolidin-3,4'-pyrrolo[1,2-b]pyrazol]-1-yl}methyl)pyridin-2(1H)-on
(3R)-2'-{6-Amino-5-[(1R)-1-(pyridin-3-yl)ethoxy]pyridin-3-yl}-N-(Propan-2-yl)-5',6'-dihydrospiro[pyrrolidin-3,4'-pyrrolo[1,2-b]pyrazol]-1-carboxamid
2'-[6-Amino-5-(6,7-dihydro-5H-cyclopenta[b]pyridin-7-yloxy)pyridin-3-yl]-N-ethyl-5',6'-dihydro-1H-spiro[pyrrolidin-3,4'-pyrrolo[1,2-b]pyrazol]-1-carboxamid (4 Isomere)
(3R)-2'-{6-Amino-5-[1-(1,3-oxazol-4-yl)ethoxy]pyridin-3-yl}-N-isopropyl-5',6'-dihydro-1H-spiro[pyrrolidin-3,4'-pyrrolo[1,2-b]pyrazol]-1-carboxamid (Diastereomer 1 und Diastereomer 2)
5-{(3R)-1-[(1,3-Thiazol-2-yl)methyl]-5',6'-dihydrospiro[pyrrolidin-3,4'-pyrrolo[1,2-b]pyrazol]-2'-yl}-3-(trifluormethyl)pyridin-2-amin
6-({(3'R)-2-[6-Amino-5-(trifluormethyl)pyridin-3-yl]-6,7-dihydrospiro[pyrazolo[5,1-c][1,4]-oxazin-4,3'-pyrrolidin]-1'-yl}methyl)pyridin-2(1H)-on
(3S)-2'-[6-Amino-5-(trifluormethyl)pyridin-3-yl]-N-[cyclopropyl(phenyl)methyl]-5',6'-dihydrospiro[pyrrolidin-3,4'-pyrrolo[1,2-b]pyrazol]-1-carboxamid (Diastereomer 1)
(3S)-2'-[6-Amino-5-(trifluormethyl)pyridin-3-yl]-N-[cyclopropyl(phenyl)methyl]-5',6'-dihydrospiro[pyrrolidin-3,4'-pyrrolo[1,2-b]pyrazol]-1-carboxamid (Diastereomer 2)
(3R)-2'-[6-Amino-5-(6,7-dihydro-5H-cyclopenta[b]pyridin-7-yloxy)pyridin-3-yl]-N-ethyl-5',6'-dihydro-1H-spiro[pyrrolidin-3,4'-pyrrolo[1,2-b]pyrazol]-1-carboxamid (Diastereomer 1)
2'-{6-Amino-5-[(1R)-1-(pyridin-2-yl)ethoxy]pyridin-3-yl}-N-ethyl-5',6'-dihydrospiro[azetidin-3,4'-pyrrolo[1,2-b]pyrazol]-1-carboxamid
1-{(3R)-2'-[6-Amino-5-(trifluormethyl)pyridin-3-yl]-5',6'-dihydrospiro[pyrrolidin-3,4'-pyrrolo[1,2-b]pyrazol]-1-yl}-2-(methylamino)ethan-1-on
(2R)-1-{(3R)-2'-[6-Amino-5-(trifluormethyl)pyridin-3-yl]-5',6'-dihydrospiro[pyrrolidin-3,4'-pyrrolo[1,2-b]pyrazol]-1-yl}-2-(methylamino)Propan-1-on
(5R)-5-({(3R)-2'-[6-Amino-5-(trifluormethyl)pyridin-3-yl]-5',6'-dihydrospiro[pyrrolidin-3,4'-pyrrolo[1,2-b]pyrazol]-1-yl}methyl)pyrrolidin-2-on
5-{(3R)-1-[(1,3,4-Thiadiazol-2-yl)methyl]-5',6'-dihydrospiro[pyrrolidin-3,4'-pyrrolo[1,2-b]pyrazol]-2'-yl}-3-(trifluormethyl)pyridin-2-amin
(5S)-5-({(3R)-2'-[6-Amino-5-(trifluormethyl)pyridin-3-yl]-5',6'-dihydrospiro[pyrrolidin-3,4'-pyrrolo[1,2-b]pyrazol]-1-yl}methyl)pyrrolidin-2-on
5-{(3'R)-1'-[1-(6-Methoxypyridin-2-yl)ethyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3R)-1-[Cyclopropyl(6-methoxypyridin-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3'R)-1'-[Cyclopropyl(6-methoxypyridin-2-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3R)-1-[1-(3-Fluorpyridin-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
3-(Difluormethoxy)-5-{(3'R)-1'-[1-(1H-imidazol-2-yl)propyl]-6,7-dihydrospiro[pyrazolo[5,1-c][1,4]oxazin-4,3'-pyrrolidin]-2-yl}pyridin-2-amin (Diastereomer 1 und Diastereomer 2)
3-(Difluormethoxy)-5-{(3'R)-1'-[1-(1H-imidazol-2-yl)propyl]-6,7-dihydrospiro[pyrazolo[5,1-c][1,4]oxazin-4,3'-pyrrolidin]-2-yl}pyridin-2-amin (Diastereomer 1)
3-(Difluormethoxy)-5-{(3'R)-1'-[1-(1H-imidazol-2-yl)propyl]-6,7-dihydrospiro[pyrazolo[5,1-c][1,4]oxazin-4,3'-pyrrolidin]-2-yl}pyridin-2-amin (Diastereomer 2)
2-Amino-5-{(3'R)-1'-[1-(1H-imidazol-2-yl)propyl]-6,7-dihydrospiro[pyrazolo[5,1-c][1,4]-oxazin-4,3'-pyrrolidin]-2-yl}pyridin-3-carbonitril (Diastereomer 1 und Diastereomer 2)
2-Amino-5-{(3'R)-1'-[1-(1H-imidazol-2-yl)propyl]-6,7-dihydrospiro[pyrazolo[5,1-c][1,4]-oxazin-4,3'-pyrrolidin]-2-yl}pyridin-3-carbonitril (Diastereomer 1)
2-Amino-5-{(3'R)-1'-[1-(1H-imidazol-2-yl)propyl]-6,7-dihydrospiro[pyrazolo[5,1-c][1,4]-oxazin-4,3'-pyrrolidin]-2-yl}pyridin-3-carbonitril (Diastereomer 2)
3-(Difluormethyl)-5-{(3'R)-1'-[1-(1H-imidazol-2-yl)propyl]-6,7-dihydrospiro[pyrazolo[5,1-c][1,4]-oxazin-4,3'-pyrrolidin]-2-yl}pyridin-2-amin (Diastereomer 1 und Diastereomer 2)
3-(Difluormethyl)-5-{(3'R)-1'-[1-(1H-imidazol-2-yl)propyl]-6,7-dihydrospiro[pyrazolo[5,1-c][1,4]-oxazin-4,3'-pyrrolidin]-2-yl}pyridin-2-amin (Diastereomer 1)
3-(Difluormethyl)-5-{(3'R)-1'-[1-(1H-imidazol-2-yl)propyl]-6,7-dihydrospiro[pyrazolo[5,1-c][1,4]-oxazin-4,3'-pyrrolidin]-2-yl}pyridin-2-amin (Diastereomer 2)
(3R)-2'-(6-Amino-5-{[1-(1-propyl-1H-pyrazol-5-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1 und Diastereomer 2)
(3R)-2'-(6-Amino-5-{[1-(1,3-thiazol-4-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1 und Diastereomer 2)
(3R)-2'-(6-Amino-5-{ [1-(pyrimidin-4-yl)ethyl]oxy}pyridin-3-yl)-N-(Propan-2-yl)-5',6'-dihydrospiro[pyrrolidin-3,4'-pyrrolo[1,2-b]pyrazol]-1-carboxamid (Diastereomer 1 und Diastereomer 2)
(3R)-2'-(6-Amino-5-{[1-(2,4-dimethylpyrimidin-5-yl)ethyl]oxy}pyridin-3-yl)-N-(Propan-2-yl)-5',6'-dihydrospiro[pyrrolidin-3,4'-pyrrolo[1,2-b]pyrazol]-1-carboxamid (Diastereomer 1)
(3R)-2'-(6-Amino-5-{[1-(2,4-dimethylpyrimidin-5-yl)ethyl]oxy}pyridin-3-yl)-N-(Propan-2-yl)-5',6'-dihydrospiro[pyrrolidin-3,4'-pyrrolo[1,2-b]pyrazol]-1-carboxamid (Diastereomer 2)
(3R)-2'-{6-Amino-5-[(1R)-1-(5-methyl-1,3,4-oxadiazol-2-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[(1R)-1-(1-methyl-1H-1,2,3-triazol-5-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-(6-Amino-5-{[1-(1-methyl-1H-1,2,3-triazol-4-yl)ethyl]oxy}pyridin-3-yl)-N-(Propan-2-yl)-5',6'-dihydrospiro[pyrrolidin-3,4'-pyrrolo[1,2-b]pyrazol]-1-carboxamid und (Diastereomer 1 und Diastereomer 2)
(5S)-5-{[(3R)-2'-{6-Amino-5-[(1R)-1-phenylethoxy]pyridin-3-yl}-5',6'-dihydrospiro[pyrrolidin-3,4'-pyrrolo[1,2-b]pyrazol]-1-yl]methyl}pyrrolidin-2-on
(5R)-5-{[(3R)-2'-{6-Amino-5-[(1R)-1-phenylethoxy]pyridin-3-yl}-5',6'-dihydrospiro[pyrrolidin-3,4'-pyrrolo[1,2-b]pyrazol]-1-yl]methyl}pyrrolidin-2-on
(3R)-2'-(6-Amino-5-{1-[4-(methylsulfonyl)phenyl]ethoxy}pyridin-3-yl)-N-isopropyl-5',6'-dihydro-1H-spiro[pyrrolidin-3,4'-pyrrolo[1,2-b]pyrazol]-1-carboxamid (Diastereomer 1 und Diastereomer 2)
(3R)-2'-{6-Amino-5-[(1R)-1-(2-methyl-1,3-thiazol-4-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-[6-Amino-5-({1-[3-(methansulfonyl)phenyl]ethyl}oxy)pyridin-3-yl]-N-(Propan-2-yl)-5',6'-dihydrospiro[pyrrolidin-3,4'-pyrrolo[1,2-b]pyrazol]-1-carboxamid (Diastereomer 1 und Diastereomer 2)
(3R)-2'-(6-Amino-5-{[1-(4-methyl-1,3-thiazol-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1 und Diastereomer 2)
(3R)-2'-(6-Amino-5-{[1-(4,5-dimethyl-1,3-thiazol-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1 und Diastereomer 2)
(3R)-2'-(6-Amino-5-{[1-(5-methyl-1,3-thiazol-2-yl)ethyl]oxy}pyridin-3-yl)-N-ethyl-5',6'-dihydrospiro[pyrrolidin-3,4'-pyrrolo[1,2-b]pyrazol]-1-carboxamid (Diastereomer 1 und Diastereomer 2)
(3R)-2'-{6-Amino-5-[(1R)-1-(pyridin-4-yl)ethoxy]pyridin-3-yl}-N-(Propan-2-yl)-5',6'-dihydrospiro[pyrrolidin-3,4'-pyrrolo[1,2-b]pyrazol]-1-carboxamid
(3R)-2'-{6-Amino-5-[(1R)-1-(pyridin-4-yl)ethoxy]pyridin-3-yl}-N-cyclobutyl-5',6'-dihydrospiro[pyrrolidin-3,4'-pyrrolo[1,2-b]pyrazol]-1-carboxamid
6-(1-{(3'R)-2-[6-Amino-5-(trifluormethyl)pyridin-3-yl]-6,7-dihydrospiro[pyrazolo[5,1-c][1,4]oxazin-4,3'-pyrrolidin]-1'-yl}ethyl)pyridin-2-ol (Diastereomer 1 und Diastereomer 2)
6-(1-{(3'R)-2-[6-Amino-5-(trifluormethyl)pyridin-3-yl]-6,7-dihydrospiro[pyrazolo[5,1-c][1,4]oxazin-4,3'-pyrrolidin]-1'-yl}ethyl)pyridin-2-ol (Diastereomer 1)
6-(1-{(3'R)-2-[6-Amino-5-(trifluormethyl)pyridin-3-yl]-6,7-dihydrospiro[pyrazolo[5,1-c][1,4]oxazin-4,3'-pyrrolidin]-1'-yl}ethyl)pyridin-2-ol (Diastereomer 2)
6-({(3R)-2'-[6-Amino-5-(trifluormethyl)pyridin-3-yl]-5',6'-dihydrospiro[pyrrolidin-3,4'-pyrrolo[1,2-b]pyrazol]-1-yl}(cyclopropyl)methyl)pyridin-2-ol (Diastereomer 1 und Diastereomer 2)
6-({(3'R)-2-[6-Amino-5-(trifluormethyl)pyridin-3-yl]-6,7-dihydrospiro[pyrazolo[5,1-c][1,4]-oxazin-4,3'-pyrrolidin]-1'-yl}(cyclopropyl)methyl)pyridin-2-ol (Diastereomer 1 und Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[(3,3-Difluorcyclobutyl)(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[(3,3-Difluorcyclobutyl)(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[(3,3-Difluorcyclobutyl)(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{1'-[(3,3-Difluorcyclobutyl)(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Isomer 1)
5-{1'-[(3,3-Difluorcyclobutyl)(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Isomer 2)
5-{1'-[(3,3-Difluorcyclobutyl)(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Isomer 3)
5-{1'-[(3,3-Difluorcyclobutyl)(1H-imidazol-2-yl)methyl]-6,7-dihydrospiro[pyrazolo[5,1-c][1,4]-oxazin-4,3'-pyrrolidin]-2-yl}-3-(trifluormethyl)pyridin-2-amin (Isomer 4)
5-{(3R)-1-[1-(1H-Pyrazol-3-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[(1H-Imidazol-2-yl)(1-methylcyclopropyl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[(1H-Imidazol-2-yl)(1-methylcyclopropyl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[(1H-Imidazol-2-yl)(1-methylcyclopropyl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[Cyclobutyl(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[Cyclobutyl(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[Cyclobutyl(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3'R)-1'-[Cyclohexyl(1H-imidazol-2-yl)methyl]-6,7-dihydrospirol[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3R)-1-[Cyclohexyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(4-Chlor-1H-imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[(3,3-Difluorcyclobutyl)(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3'R)-1'-[Cyclopropyl(1H-imidazol-2-yl)methyl]-6,7-dihydrospiro[pyrazolo[5,1-c][1,4]-oxazin-4,3'-pyrrolidin]-2-yl}-3-(trifluormethyl)pyrazin-2-amin (Diastereomer 1 und Diastereomer 2)
5-{(3R)-1-[(1H-Imidazol-2-yl)(1-methylcyclobutyl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(1H-Pyrazol-3-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(1H-Pyrazol-3-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[Cyclopropyl(1H-1,2,3-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-[(1R)-1-(2,6-difluorphenyl)ethoxy]pyridin-2-amin (Diastereomer 1 und Diastereomer 2)
5-{(3R)-1-[(1-Methylcyclobutyl)(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(4-Chlor-1H-imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[1-(4-Chlor-1H-imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-[(1R)-1-(3-fluorpyridin-2-yl)ethoxy]pyridin-2-amin (Diastereomer 1 und Diastereomer 2)
5-{(3R)-1-[(3,3-Difluorcyclobutyl)(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[(3,3-Difluorcyclobutyl)(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[cyclopentyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3'R)-1'-[Cyclopropyl(1H-1,2,3-triazol-5-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3R)-1-[Cyclopropyl(1-methyl-1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[1-(4H-1,2,4-Triazol-3-yl)(1-²H)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(4H-1,2,4-Triazol-3-yl)(1-²H)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[1-(4H-1,2,4-Triazol-3-yl)(1-²H)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3'R)-1'-[1-(4H-1,2,4-Triazol-3-yl)(1-²H)propyl]-6,7-dihydrospiro[pyrazolo[5,1-c][1,4]oxazin-4,3'-pyrrolidin]-2-yl}-3-(trifluormethyl)pyridin-2-amin (Diastereomer 1 und Diastereomer 2)
5-{(3'R)-1'-[Cyclopropyl(1H-imidazol-2-yl)(²H)methyl]-6,7-dihydrospiro[pyrazolo[5,1-c][1,4]oxazin-4,3'-pyrrolidin]-2-yl}-3-(trifluormethyl)pyridin-2-amin (Diatereomer 1 und Diastereomer 2)
5-{ (3'R)-1'-[Cyclopropyl (1H-imidazol-2-yl)(²H)methyl]-6,7-dihydrospiro[pyrazolo[5,1-c][1,4]oxazin-4,3'-pyrrolidin]-2-yl}-3-(trifluormethyl)pyridin-2-amin (Diastereomer 1)
5-{(3'R)-1'-[Cyclopropyl (1H-imidazol-2-yl)(²H)methyl]-6,7-dihydrospiro[pyrazolo[5,1-c][1,4]oxazin-4,3'-pyrrolidin]-2-yl}-3-(trifluormethyl)pyridin-2-amin (Diastereomer 2)
5-{(3'R)-1'-[1-(1H-Imidazol-2-yl)(1-²H)propyl]-6,7-dihydrospiro[pyrazolo[5,1-c][1,4]oxazin-4,3'-pyrrolidin]-2-yl}-3-(trifluormethyl)pyridin-2-amin (Diastereomer 1 und Diastereomer 2)
5-{ (3'R)-1'-[1-(1H-Imidazol-2-yl)(1-²H)propyl]-6,7-dihydrospiro[pyrazolo[5,1-c][1,4]oxazin-4,3'-pyrrolidin]-2-yl}-3-(trifluormethyl)pyridin-2-amin (Diastereomer 1)
5-{ (3'R)-1'-[1-(1H-Imidazol-2-yl)(1-²H)propyl]-6,7-dihydrospiro[pyrazolo[5,1-c][1,4]oxazin-4,3'-pyrrolidin]-2-yl}-3-(trifluormethyl)pyridin-2-amin (Diastereomer 2)
5-{(3R)-1-[1-(1H-Imidazol-2-yl)(1-²H)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[1-(1H-Imidazol-2-yl)(1-²H)propyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{ (3R)-1-[1-(1H-Imidazol-2-yl)(1-²H)propyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{ (3R)-1-[Cyclopropyl (1H-imidazol-2-yl) (²H)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{ (3R)-1-[Cyclopropyl (1H-imidazol-2-yl)(²H)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{ (3R)-1-[Cyclopropyl (1H-imidazol-2-yl)(²H)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[Cyclopropyl(2-methoxypyridin-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
3-[{(3R)-2'-[6-Amino-5-(trifluormethyl)pyridin-3-yl]-5',6'-dihydro-1H-spiro[pyrrolidin-3,4'-pyrrolo[1,2-b]pyrazol]-1-yl}(cyclopropyl)methyl]pyridin-2(1H)-on (Diastereomer 1 und Diastereomer 2)
5-{(3R)-1-[(1-Fluorcyclopropyl)(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[(1-Fluorcyclopropyl)(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[(1-Fluorcyclopropyl)(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[Cyclopropyl(4-methoxypyrimidin-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[Cyclobutyl(1H-1,2,4-triazol-5-yl)methyl]-5',6'-dihydrospiro[pyrrolidin-3,4'-pyrrolo[1,2-b]pyrazol]-2'-yl}-3-(trifluormethoxy)pyridin-2-amin (Diastereomer 1 und Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-(trifluormethoxy)pyridin-2-amin (Diastereomer 1 und Diastereomer 2)
5-{(3'R)-1'-[Cyclopropyl(1H-1,2,3-triazol-5-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3'R)-1'-[Cyclopropyl(1H-1,2,3-triazol-5-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 2)
5-{(3R)-1-[(1-Fluorcyclobutyl)(4H-1,2,4-triazol-3-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[Cyclopropyl(pyrimidin-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-[(1R)-1-(2,6-difluorphenyl)ethoxy]pyridin-2-amin (Diastereomer 1)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-[(1R)-1-(2,6-difluorphenyl)ethoxy]pyridin-2-amin (Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-[(1R)-1-(3-fluorpyridin-2-yl)ethoxy]pyridin-2-amin (Diastereomer 1)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-[(1R)-1-(3-fluorpyridin-2-yl)ethoxy]pyridin-2-amin (Diastereomer 2)
5-{(3'R)-1'-[Cyclobutyl(4H-1,2,4-triazol-3-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1)
5-{(3R)-1-[cyclopentyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[cyclopentyl(1H-imidazol-2-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[(4,4-Difluorcyclohexyl)(1H-1,2,4-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[(4,4-Difluorcyclohexyl)(1H-1,2,4-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[(4,4-Difluorcyclohexyl)(1H-1,2,4-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3R)-1-[2,2-Difluor-1-(1H-imidazol-2-yl)propyl]-5',6'-dihydrospiro[pyrrolidin-3,4'-pyrrolo[1,2-b]pyrazol]-2'-yl}-3-(trifluormethyl)pyridin-2-amin (Diastereomer 1 und Diastereomer 2)
5-{(3R)-1-[Cyclopropyl(1H-1,2,3-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 1)
5-{(3R)-1-[Cyclopropyl(1H-1,2,3-triazol-5-yl)methyl]-5',6'-dihydrospiro[pyrrolidin-3,4'-pyrrolo[1,2-b]pyrazol]-2'-yl}-3-(trifluormethyl)pyridin-2-amin (Diastereomer 2)
5-{(3'R)-1'-[Cyclobutyl(4H-1,2,4-triazol-3-yl)methyl]-6,7-dihydrospiro[pyrazolo[5,1-c][1,4]-oxazin-4,3'-pyrrolidin]-2-yl}-3-(trifluormethyl)pyridin-2-amin (Diastereomer 1 und Diastereomer 2)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-(trifluormethoxy)pyridin-2-amin (Diastereomer 1)
5-{(3R)-1-[Cyclopropyl(1H-1,2,4-triazol-5-yl)methyl]-5',6'-dihydrospiro[pyrrolidin-3,4'-pyrrolo[1,2-b]pyrazol]-2'-yl}-3-(trifluormethoxy)pyridin-2-amin (Diastereomer 2)
(3R)-2'-{5-Amino-6-[(1R)-1-phenylethoxy]pyrazin-2-yl}-N-ethyl-5',6'-dihydrospiro[pyrrolidin-3,4'-pyrrolo[1,2-b]pyrazol]-1-carboxamid
5-{(3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyrazin-2-amin (Diastereomer 1 und Diastereomer 2)
5-{ (3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyrazin-2-amin (Diastereomer 1)
5-{ (3R)-1-[Cyclopropyl(1H-imidazol-2-yl)methyl]-5',6'-dihydrospiro[pyrrolidin-3,4'-pyrrolo[1,2-b]pyrazol]-2'-yl}-3-(trifluormethyl)pyrazin-2-amin (Diastereomer 2) oder ein Stereoisomer, ein Tautomer, ein N-Oxid, ein Hydrat, ein Solvat oder ein Salz davon oder ein Gemisch davon.

10. Verbindung der allgemeinen Formel (I) nach einem der Ansprüche 1, 2, 3, 4, 5, 6, 7, 8 oder 9 zur Verwendung als ein Arzneimittel.

11. Verbindung der allgemeinen Formel (I) nach einem der Ansprüche 1, 2, 3, 4, 5, 6, 7, 8 oder 9 zur Verwendung bei der Behandlung oder Prophylaxe einer Erkrankung.

12. Pharmazeutische Zusammensetzung, umfassend eine Verbindung der allgemeinen Formel (I) nach einem der Ansprüche 1, 2, 3, 4, 5, 6, 7, 8 oder 9 und einen oder mehrere pharmazeutisch annehmbare Hilfsstoffe.

13. Pharmazeutische Kombination, umfassend:
einen oder mehrere erste Wirkstoffe, die Verbindungen der allgemeinen Formel (I) nach einem der Ansprüche 1, 2, 3, 4, 5, 6, 7, 8 oder 9 sind, und
eine oder mehrere pharmazeutisch wirksame Antikrebsverbindungen oder
einen oder mehrere pharmazeutisch wirksame Immuncheckpoint-Inhibitoren.

14. Pharmazeutische Kombination nach Anspruch 13, **dadurch gekennzeichnet, dass** der pharmazeutische aktive Immuncheckpoint-Inhibitor ein Antikörper ist.

15. Verbindung der allgemeinen Formel (I) nach einem der Ansprüche 1, 2, 3, 4, 5, 6, 7, 8 oder 9 zur Verwendung bei der Behandlung oder Prophylaxe von Krebs, einer gutartigen Hyperplasie, einer atherosklerotischen Störung, von Sepsis, einer Autoimmunstörung, einer Gefäßerkrankung, einer Virusinfektion, einer neurodegenerativen Störung oder einer entzündlichen Erkrankung.

16. Verwendung einer Verbindung der allgemeinen Formel (I) nach einem der Ansprüche 1, 2, 3, 4, 5, 6, 7, 8 oder 9 zur Kontrolle der männlichen Fertilität.

## Revendications

1. Composé de formule (I) 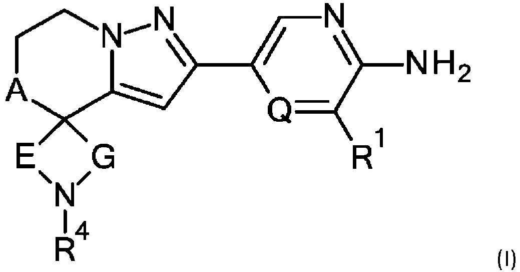 dans lequel
A est choisi parmi une liaison simple, -CH₂- ou -O- ;
E est -(CH₂)ₙ- avec n = 1 ou 2 ;
G est -(CH₂)- et sous réserve que la sous-structure cyclique comprenant E-N-G soit un hétérocycloalkyle à 4 ou 5 chaînons ;
Q est choisi parmi N ou C-R² ;
R' est choisi parmi
• -NO₂ ; -CN ;
• alkyle en C₁ à C₄ éventuellement substitué par -OH ou 1 à 3 -F ;
• cyclopropyle ;
• hétérocycloalkyle ou hétérocycloalcényle à 4 à 6 chaînons, les deux ayant 1 ou 2 atomes d'azote, mais toujours liés via un atome de carbone au groupe phényle des composés de formule (I), et les deux éventuellement substitués par alkyle en C₁ à C₄ ;
• phényle éventuellement substitué par halogéno, -CN, alkyle en C₁ à C₄, halogénoalkyle en C₁ à C₄, cycloalkyle en C₃ à C₆, O-alkyle en C₁ à C₄, O-cycloalkyle en C₃ à C₆, alcynyle en C₁ à C₃, -C(O)-R_{d}, SR_{d}, SOR_{d}, SO₂R_{d}, SONR_{d}, SON(H)R_{d}, -SO₂-NH₂, -SO₂-N(H)-R_{d} ou -SO₂-N(R_{d})₂, où R_{d} est choisi parmi méthyle, éthyle, cyclopropyle, isopropyle et tert-butyle ;
• hétéroaryle à 5 ou 6 chaînons ayant 1, 2 ou 3 hétéroatomes choisis parmi N, O ou S et éventuellement substitué par 1 ou 2 des substituants suivants : halogéno, -CN, alkyle en C₁ à C₄, halogénoalkyle en C₁ à C₄, cycloalkyle en C₃ à C₆, O-alkyle en C₁ à C₄, O-cycloalkyle en C₃ à C₆, alcynyle en C₁ à C₃, SR_{d}, SOR_{d}, SO₂R_{d}, SONR_{d} et SON(H)R_{d} où R_{d} est choisi parmi méthyle, éthyle, cyclopropyle, isopropyle et tert-butyle ;
• -O-CH₃ ; -O-CH(CH₃)₂ ; -O-CHF₂ ; -O-CF₃ ; 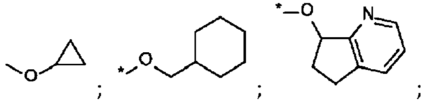
• -O-CHRₐ-phényle éventuellement substitué par un, deux ou trois des substituants suivants : halogéno, -CN, alkyle en C₁ à C₄ éventuellement substitué par un ou plusieurs -F, O-alkyle en C₁ à C₄, (alcynyle en C₁ à C₆)-OH et -SO₂-CH₃, et où Rₐ est choisi parmi -H, -F et -CH₃, -CF₃, -CH₂-CH₃, -CH(CH₃)₂ et -cyclopropyle ;
• -O-CHRₐ-hétéroaryle à 6 chaînons éventuellement substitué par un, deux ou trois des substituants suivants : halogéno, -CN, alkyle en C₁ à C₄ éventuellement substitué par un ou plusieurs -F et -SO₂-CH₃, et où Rₐ est choisi parmi -H, -F et -CH₃, -CF₃, -CH₂-CH₃, -CH(CH₃)₂ et -cyclopropyle ;
• -COORₐ où Rₐ est choisi parmi -H et -CH₃ ;
• C(=O)-méthyle, éventuellement substitué par 1 à 3 -F ;
• -C(=O)-NR_{b}R_{c} où R_{b} est choisi parmi -H, -CH₃ et -CH₂-CH₃ et où R_{c} est choisi parmi -H, -CH₃, -CH₂-CH₃, cyclohexyle, phényle ou hétéroaryle à 5 ou 6 chaînons, tous éventuellement substitués par un ou deux halogéno, -CN, -SO₂-CH₃ ou alkyle en C₁ à C₄, éventuellement substitués une ou plusieurs fois par halogéno ;
• -S-CR_{f}R_{g}Rₕ où R_{f} est choisi parmi -H et -F, où R_{g} est choisi parmi -H, -F ou -CH₃ et où Rₕ est choisi parmi H, F, -CH₃ et cycloalkyle en C₅ à C₆ et phényle ;
• 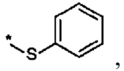
R² est choisi parmi -H ;
R⁴ est choisi parmi
• alkyle en C₁ à C₆, cycloalkyle en C₃ à C₆ ou (alkyle en C₁ à C₃)-cycloalkyle en C₃ à C₆, tous éventuellement substitués une ou plusieurs fois indépendamment par
∘ -F, -Cl, -Br, -CN ;
∘ -OH, -ORᵢ où Rᵢ est choisi parmi alkyle en C₁ à C₄ et cycloalkyle en C₃ à C₆, les deux éventuellement substitués une ou plusieurs fois par halogéno,
∘ -CF₃, -CHF₂, -CH₂F ou -CH₂-CF₃ ;
∘ NRⱼRₖ où Rⱼ et Rₖ sont indépendamment choisis parmi -H et alkyle en C₁ à C₄,
∘ -CH₂-O-CH₃ ou
∘ cycloalkyle en C₃ à C₄ ou hétérocycloalkyle à 4 à 6 chaînons, les deux éventuellement substitués par (=O), ou éventuellement substitués une ou plusieurs fois par F ou CH₃ ;
∘ spiroalkyle en C₆ à C₈ ou bicycloalkyle en C₅ à C₇ :
∘ phényle ou hétéroaryle à 5 ou 6 chaînons, les deux éventuellement substitués une ou plusieurs fois par
▪ halogéno, -CN ;
▪ -CF₃, -CHF₂, -CH₂F ;
▪ alkyle en C₁ à C₄ éventuellement substitué par un ou plusieurs halogéno ;
▪ cycloalkyle en C₃ à C₆ ;
▪ O-alkyle en C₁ à C₄ ;
▪ O-cycloalkyle en C₃ à C₆ ;
▪ alcynyle en C₁ à C₃ ;
▪ -NRⱼRₖ où Rⱼ et Rₖ sont indépendamment choisis parmi -H et alkyle en C₁ à C₄ ;
▪ SR_{d}, SOR_{d}, SO₂R_{d}, SONR_{d} ou SON(H)R_{d} où R_{d} est choisi parmi méthyle, éthyle, cyclopropyle, isopropyle et tert-butyle ; ou
o phényle éventuellement substitué une ou plusieurs fois par -C(O)NRⱼRₖ où Rⱼ et Rₖ sont indépendamment choisis parmi -H et alkyle en Cₗ à C₄ ; ou
o hétéroaryle à 5 ou 6 chaînons éventuellement substitué une ou plusieurs fois par -OH ;
• C(=O)-Rₗ où Rₗ est choisi parmi
∘ alkyle en C₁ à C₄, cycloalkyle en C₃ à C₆ ou hétérocycloalkyle en C₃ à C₆, tous éventuellement substitués une ou plusieurs fois par
▪ -F, -Cl, -Br, -CN ;
▪ -OH, -ORᵢ où Rᵢ est choisi parmi alkyle en C₁ à C₄ et cycloalkyle en C₃ à C₆, -CF₃, -CHF₂, -CH₂F ;
▪ -NRⱼRₖ où Rⱼ et Rₖ sont indépendamment choisis parmi -H et alkyle en C₁ à C₄ ;
▪ phényle ou hétéroaryle à 5 ou 6 chaînons, les deux éventuellement substitués une ou plusieurs fois par :
• halogéno, -CN ;
• -CF₃, -CHF₂, -CH₂F ;
• alkyle en C₁ à C₄ éventuellement substitué par un ou plusieurs halogéno ;
• cycloalkyle en C₃ à C₆ ;
• O-alkyle en C₁ à C₄ ;
• O-cycloalkyle en C₃ à C₆ ;
• alcynyle en C₁ à C₃ ;
• -NRⱼRₖ où Rⱼ et Rₖ sont indépendamment choisis parmi -H et alkyle en C₁ à C₄ ;
• SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
∘ phényle ou hétéroaryle à 5 ou 6 chaînons, les deux éventuellement substitués une ou plusieurs fois par
▪ halogéno, -CN ;
▪ -CF₃, -CHF₂, -CH₂F ;
▪ alkyle en C₁ à C₄ éventuellement substitué par un ou plusieurs halogéno ;
▪ cycloalkyle en C₃ à C₆ ;
▪ O-alkyle en C₁ à C₄ ;
▪ O-cycloalkyle en C₃ à C₆ ;
▪ alcynyle en C₁ à C₃ ;
▪ NRⱼRₖ où Rⱼ et Rₖ sont indépendamment choisis parmi -H et alkyle en C₁ à C₄;
▪ SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
• C(=O)-NRₒRₚ où Rₒ et Rₚ sont indépendamment choisis parmi
∘ H;
∘ alkyle en C₁ à C₄, cycloalkyle en C₃ à C₆ ou (alkyle en C₁ à C₃)-cycloalkyle en C₃ à C₆, tous éventuellement substitués une ou plusieurs fois par
▪ -F, -Cl, -Br, -CN ;
▪ -OH, -ORᵢ où Rᵢ est choisi parmi alkyle en C₁ à C₄ et cycloalkyle en C₃ à C₆, -CF₃, -CHF₂, -CH₂F ;
▪ -NRⱼRₖ où Rⱼ et Rₖ sont indépendamment choisis parmi -H et alkyle en C₁ à C₄ ;
▪ phényle ou hétéroaryle à 5 ou 6 chaînons, les deux éventuellement substitués une ou plusieurs fois par
• halogéno, -CN ;
• -CF₃, -CHF₂, -CH₂F ;
• alkyle en C₁ à C₄ éventuellement substitué par un ou plusieurs halogéno ;
• cycloalkyle en C₃ à C₆ ;
• O-alkyle en C₁ à C₄ ;
• O-cycloalkyle en C₃ à C₆ ;
• alcynyle en C₁ à C₃ ;
• -NRⱼRₖ où Rⱼ et Rₖ sont indépendamment choisis parmi -H et alkyle en C₁ à C₄ ;
• SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
∘ phényle ou hétéroaryle à 5 ou 6 chaînons, les deux éventuellement substitués une ou plusieurs fois par
▪ halogéno, -CN ;
▪ -CF₃, -CHF₂, -CH₂F ;
▪ alkyle en C₁ à C₄ éventuellement substitué par un ou plusieurs halogéno ;
▪ cycloalkyle en C₃ à C₆ ;
▪ O-alkyle en C₁ à C₄ ;
▪ O-cycloalkyle en C₃ à C₆ ;
▪ alcynyle en C₁ à C₃ ;
▪ -NRⱼRₖ où Rⱼ et Rₖ sont indépendamment choisis parmi -H et alkyle en C₁ à C₄ ;
▪ SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
ou un stéréoisomère, un tautomère, un N-oxyde, un hydrate, un solvate, ou un sel d'un tel composé, ou un mélange de ceux-ci.

2. Composé de formule (Ia) selon la revendication 1 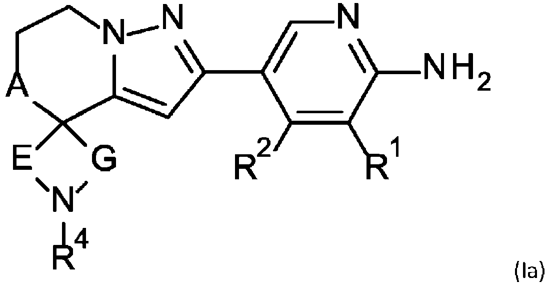 dans lequel
A est choisi parmi une liaison simple, -CH₂- ou -O- ;
E est -(CH₂)ₙ- où n = 1 ou 2 ;
G est -(CH₂)- sous réserve que la sous-structure cyclique comprenant E-N-G soit un hétérocycloalkyle à 4 ou 5 chaînons ;
R¹ est choisi parmi
• -NO₂ ; -CN ;
• alkyle en C₁ à C₄ éventuellement substitué par -OH ou 1 à 3 -F ;
• cyclopropyle ;
• hétérocycloalkyle ou hétérocycloalcényle à 4 à 6 chaînons, les deux ayant 1 ou 2 atomes d'azote, mais toujours liés via un atome de carbone au groupe phényle de composés de formule (I), et les deux éventuellement substitués par alkyle en C₁ à C₄ ;
• phényle éventuellement substitué par halogéno, -CN, alkyle en C₁ à C₄, halogénoalkyle en C₁ à C₄, cycloalkyle en C₃ à C₆, O-alkyle en C₁ à C₄, O-cycloalkyle en C₃ à C₆, alcynyle en C₁ à C₃, -C(O)-R_{d}, SR_{d}, SOR_{d}, SO₂R_{d}, SONR_{d}, SON(H)R_{d}, -SO₂-NH₂, -SO₂-N(H)-R_{d} ou -SO₂-N(R_{d})₂, où R_{d} est choisi parmi méthyle, éthyle, cyclopropyle, isopropyle et tert-butyle ;
• hétéroaryle à 5 ou 6 chaînons ayant 1 ou 2 hétéroatomes choisis parmi N et S et éventuellement substitué par halogéno, -CN, alkyle en C₁ à C₄, halogénoalkyle en C₁ à C₄, cycloalkyle en C₃ à C₆, O-alkyle en C₁ à C₄, O-cycloalkyle en C₃ à C₆, alcynyle en C₁ à C₃, SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
• -O-CH₃ ; -O-CH(CH₃)₂ ; -O-CHF₂ ; -O-CF₃ ; 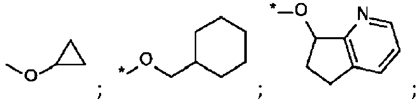
• -O-CHRₐ-phényle éventuellement substitué par un ou deux des substituants suivants : halogéno, -CN, alkyle en C₁ à C₄ éventuellement substitué par un ou plusieurs -F ou -SO₂-CH₃, et où Rₐ est choisi parmi - H, -F et -CH₃, -CF₃, -CH₂-CH₃, -CH(CH₃)₂ ou -cyclopropyle ;
• -O-CHRₐ-hétéroaryle à 6 chaînons éventuellement substitué par un ou deux des substituants suivants : halogéno, -CN, alkyle en C₁ à C₄ éventuellement substitué par un ou plusieurs -F et -SO₂-CH₃, et où Rₐ est choisi parmi -H, -F et -CH₃, -CF₃, -CH₂-CH₃, -CH(CH₃)₂ ou -cyclopropyle ;
• -COORₐ où Rₐ est choisi parmi -H ou -CH₃ ;
• C(=O)-méthyle, éventuellement substitué par 1 à 3 -F ;
• -C(=O)-NR_{b}R_{c} où R_{b} est choisi parmi -H, -CH₃ et -CH₂-CH₃ et où R_{c} est choisi parmi -H, -CH₃, -CH₂-CH₃, cyclohexyle, phényle et hétéroaryle à 5 ou 6 chaînons, tous éventuellement substitués par un ou deux halogéno, -CN, -SO₂-CH₃ ou alkyle en C₁ à C₄, éventuellement substitués une ou plusieurs fois par halogéno ;
• -S-CR_{f}R_{g}Rₕ où R_{f} est choisi parmi -H et -F, où R_{g} est choisi parmi -H, -F et -CH₃ et où Rₕ est choisi parmi H, F, -CH₃ et cycloalkyle en C₅ à C₆ ou phényle ;
• 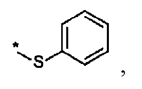
R² est choisi parmi -H ; ou
R⁴ est choisi parmi
• alkyle en C₁ à C₅ et cycloalkyle en C₃ à C₆, les deux éventuellement substitués une ou plusieurs fois indépendamment par
∘ -F, -Cl, -Br, -CN ;
∘ -OH, -ORᵢ où Rᵢ est choisi parmi alkyle en C₁ à C₄ ou cycloalkyle en C₃ à C₆ ;
∘ -CF₃, -CHF₂, -CH₂F ou -CH₂-CF₃ ;
∘ NRⱼRₖ où Rⱼ et Rₖ sont indépendamment choisis parmi -H et alkyle en C₁ à C₄ ;
∘ -CH₂-O-CH₃ ou
∘ cycloalkyle en C₃ à C₄ ou hétérocycloalkyle à 4 chaînons, les deux éventuellement substitués par -F ou -CH₃ ;
o phényle ou hétéroaryle à 5 ou 6 chaînons, les deux éventuellement substitués une ou plusieurs fois par
▪ halogéno, -CN ;
▪ -CF₃, -CHF₂, -CH₂F ;
▪ alkyle en C₁ à C₄ éventuellement substitué par un ou plusieurs halogéno ;
▪ cycloalkyle en C₃ à C₆ ;
▪ O-alkyle en C₁ à C₄ ;
▪ O-cycloalkyle en C₃ à C₆ ;
▪ alcynyle en C₁ à C₃ ;
▪ -NRⱼRₖ où Rⱼ et Rₖ sont indépendamment choisis parmi -H et alkyle en C₁ à C₄ ;
▪ SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
• C(=O)-R₁ où R₁ est choisi parmi
∘ alkyle en C₁ à C₄, cycloalkyle en C₃ à C₆ ou hétérocycloalkyle en C₃ à C₆, tous éventuellement substitués une ou plusieurs fois par
▪ -F, -Cl, -Br, -CN ;
▪ -OH, -ORᵢ où Rᵢ est choisi parmi alkyle en C₁ à C₄ et cycloalkyle en C₃ à C₆, -CF₃, -CHF₂, -CH₂F ;
▪ -NRⱼRₖ où Rⱼ et Rₖ sont indépendamment choisis parmi -H et alkyle en C₁ à C₄ ;
▪ phényle ou hétéroaryle à 5 ou 6 chaînons, les deux éventuellement substitués une ou plusieurs fois par
• halogéno, -CN ;
• -CF₃, -CHF₂, -CH₂F ;
• alkyle en C₁ à C₄ éventuellement substitué par un ou plusieurs halogéno ;
• cycloalkyle en C₃ à C₆ ;
• O-alkyle en C₁ à C₄ ;
• O-cycloalkyle en C₃ à C₆ ;
• alcynyle en C₁ à C₃ ;
• -NRⱼRₖ où Rⱼ et Rₖ sont indépendamment choisis parmi -H et alkyle en C₁ à C₄ ;
• SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
o phényle ou hétéroaryle à 5 ou 6 chaînons, les deux éventuellement substitués une ou plusieurs fois par
▪ halogéno, -CN ;
▪ -CF₃, -CHF₂, -CH₂F ;
▪ alkyle en C₁ à C₄ éventuellement substitué par un ou plusieurs halogéno ;
▪ cycloalkyle en C₃ à C₆ ;
▪ O-alkyle en C₁ à C₄ ;
▪ O-cycloalkyle en C₃ à C₆ ;
▪ alcynyle en C₁ à C₃ ;
▪ NRⱼRₖ où Rⱼ et Rₖ sont indépendamment choisis parmi -H et alkyle en C₁ à C₄ ;
▪ SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
• C(=O)-NRₒRₚ où Rₒ et Rₚ sont indépendamment choisis parmi
∘ H;
∘ alkyle en C₁ à C₄, cycloalkyle en C₃ à C₆, les deux éventuellement substitués une ou plusieurs fois par
▪ -F, -Cl, -Br, -CN ;
▪ -OH, -ORᵢ où Rᵢ est choisi parmi alkyle en C₁ à C₄ et cycloalkyle en C₃ à C₆, -CF₃, -CHF₂, -CH₂F ;
▪ -NRⱼRₖ où Rⱼ et Rₖ sont indépendamment choisis parmi -H et alkyle en C₁ à C₄ ;
▪ phényle ou hétéroaryle à 5 ou 6 chaînons, les deux éventuellement substitués une ou plusieurs fois par
• halogéno, -CN ;
• -CF₃, -CHF₂, -CH₂F ;
• alkyle en C₁ à C₄ éventuellement substitué par un ou plusieurs halogéno ;
• cycloalkyle en C₃ à C₆ ;
• O-alkyle en C₁ à C₄ ;
• O-cycloalkyle en C₃ à C₆ ;
• alcynyle en C₁ à C₃ ;
• -NRⱼRₖ où Rⱼ et Rₖ sont indépendamment choisis parmi -H et alkyle en C₁ à C₄ ;
• SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
o phényle ou hétéroaryle à 5 ou 6 chaînons, les deux éventuellement substitués une ou plusieurs fois par
▪ halogéno, -CN ;
▪ -CF₃, -CHF₂, -CH₂F ;
▪ alkyle en C₁ à C₄ éventuellement substitué par un ou plusieurs halogéno ;
▪ cycloalkyle en C₃ à C₆ ;
▪ O-alkyle en C₁ à C₄ ;
▪ O-cycloalkyle en C₃ à C₆ ;
▪ alcynyle en C₁ à C₃ ;
▪ -NRⱼRₖ où Rⱼ et Rₖ sont indépendamment choisis parmi -H et alkyle en C₁ à C₄ ;
▪ SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
ou un stéréoisomère, un tautomère, un N-oxyde, un hydrate, un solvate, ou un sel d'un tel composé, ou un mélange de ceux-ci.

3. Composés de formule (I) selon la revendication 1, dans lesquels A est choisi parmi une liaison simple ou -O-, ou un stéréoisomère, un tautomère, un N-oxyde, un hydrate, un solvate, ou un sel de tels composés, ou un mélange de ceux-ci.

4. Composés de formule (I) selon la revendication 1, dans lesquels E est -CH₂-CH₂-, ou un stéréoisomère, un tautomère, un N-oxyde, un hydrate, un solvate, ou un sel de tels composés, ou un mélange de ceux-ci.

5. Composés de formule (I) selon la revendication 1, dans lesquels Q est C-R², ou un stéréoisomère, un tautomère, un N-oxyde, un hydrate, un solvate, ou un sel de tels composés, ou un mélange de ceux-ci.

6. Composés de formule (I) selon la revendication 1, dans lesquels Q est N, ou un stéréoisomère, un tautomère, un N-oxyde, un hydrate, un solvate, ou un sel de tels composés, ou un mélange de ceux-ci.

7. Composés de formule (I) selon la revendication 1, dans lesquels R⁴ est 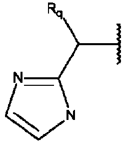 et R_{q} est choisi parmi -H, -CH₃, -CH₂-CH₃, -CH(CH₃)₂, -CH₂-O-CH₃, -CF₃, -CHF₂, -CH₂F, -CH₂-CF₃, cyclopropyle ou cyclobutyle, ou un stéréoisomère, un tautomère, un N-oxyde, un hydrate, un solvate, ou un sel de tels composés, ou un mélange de ceux-ci.

8. Composés de formule (I) selon la revendication 1, dans lesquels R⁴ est choisi parmi C(=O)-NRₒRₚ où Rₒ et Rₚ sont indépendamment choisis parmi
∘ H;
∘ alkyle en C₁ à C₄ ou cycloalkyle en C₃ à C₆, les deux éventuellement substitués une ou plusieurs fois par
▪ -F, -Cl, -Br, -CN ;
▪ -OH, -ORᵢ où Rᵢ est choisi parmi alkyle en C₁ à C₄ et cycloalkyle en C₃ à C₆, -CF₃, -CHF₂, -CH₂F ;
▪ -NRⱼRₖ où Rⱼ et Rₖ sont indépendamment choisis parmi -H et alkyle en C₁ à C₄;
▪ phényle ou hétéroaryle à 5 ou 6 chaînons, les deux éventuellement substitués une ou plusieurs fois par
• halogéno, -CN ,
• -CF₃, -CHF₂, -CH₂F,
• alkyle en C₁ à C₄ éventuellement substitué par un ou plusieurs halogènes ;
• cycloalkyle en C₃ à C₆ ;
• O-alkyle en C₁ à C₄ ;
• O-cycloalkyle en C₃ à C₆ ;
• alcynyle en C₁ à C₃ ;
• -NRⱼRₖ où Rⱼ et Rₖ sont indépendamment choisis parmi -H et alkyle en C₁ à C₄ ;
• SR_{d}, SOR_{d}, SO₂R_{d}, SONR_{d} ou SON(H)R_{d} où R_{d} est choisi parmi méthyle, éthyle, cyclopropyle, isopropyle et tert-butyle ;
ou un stéréoisomère, un tautomère, un N-oxyde, un hydrate, un solvate, ou un sel d'un tel composé, ou un mélange de ceux-ci.

9. Composé selon la revendication 1, qui est choisi parmi les suivants :
(*rac*)-2'-[6-amino-5-(trifluorométhyl)pyridin-3-yl]-N-[1-(2-chlorophényl)cyclobutyl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[2-(2-fluorophényl)propan-2-yl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-(1-phénylcyclobutyl)-6,7-dihydrospiro[pyrazolo[5,1-c][1,4]oxazine-4,3'-pyrrolidine]-1'-carboxamide
(*rac*)-2'-[6-amino-5-(trifluorométhyl)pyridin-3-yl]-N-[2-(4-fluorophényl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(2-chlorophényl)propan-2-yl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[(1R)-1-(2-chloro-4-cyanophényl)éthyl]-6,7-dihydrospiro[pyrazolo[5, 1-c][1,4]oxazine-4,3'-pyrrolidine]-1'-carboxamide
(*rac*)-2'-[6-amino-5-(trifluorométhyl)pyridin-3-yl]-N-[(1R)-1-(2-chloro-4-cyanophényl)éthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[(1S)-1-(3-chloropyridin-4-yl)éthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(3-fluorophényl)propan-2-yl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
(*rac*)-2'-[6-amino-5-(trifluorométhyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-(1-phénylcyclobutyl)-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[2-(pyridin-2-yl)propan-2-yl]-6,7-dihydrospiro[pyrazolo[5,1-c][1,4]oxazine-4,3'-pyrrolidine]-l'-carboxamide
(*rac*)-2'-(5-acétyl-6-aminopyridin-3-yl)-N-(1-phénylcyclobutyl)-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(2-chlorophényl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(2-chlorophényl)cyclobutyl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-l'-carboxamide
(*rac*)-2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[1-(2-fluorophényl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-(1-phénylcyclobutyl)-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(4-fluorophényl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(4-fluorophényl)cyclobutyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[1-(2-fluorophényl)cyclobutyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[1-(3-fluorophényl)cyclobutyl]-5',6'-dihydrospiro[pyrrolidine-3,4'-pyrrolo[1,2-b]pyrazole]-1-carboxamide
(rac)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-(1-phénylcyclobutyl)-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[2-(4-fluorophényl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluorométhyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[1-(3-fluorophényl)cyclobutyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-{(1R)-1-[3-(trifluorométhyl)phényl]éthyl}-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(2-fluorophényl)propan-2-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(3-fluorophényl)éthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(4-fluorophényl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-phénylpropyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-{(1S)-1-[3-(trifluorométhyl)phényl]éthyl}-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-phényléthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(2-cyanophényl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-(1,3,5-triméthyl-1H-pyrazol-4-yl)éthyl]-5',6'-dihydrospiro[pyrrolidine-3,4'-pyrrolo[1,2-b]pyrazole]-1-carboxamide (mélange de diastéréomères)
2'-[6-amino-5-(trifluorométhyl)pyridin-3-yl]-N-[(1R)-2,3-dihydro-1H-indén-1-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(2-cyanopyridin-4-yl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-(1-éthyl-3,5-diméthyl-1H-pyrazol-4-yl)éthyl]-5',6'-dihydrospiro[pyrrolidine-3,4'-pyrrolo[1,2-b]pyrazole]-1-carboxamide (mélange de diastéréomères)
(*rac*)-2'-[6-amino-5-(trifluorométhyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-(1,3,4-triméthyl-1H-pyrazol-5-yl)éthyl]-5',6'-dihydrospiro[pyrrolidine-3,4'-pyrrolo[1,2-b]pyrazole]-1-carboxamide (mélange de diastéréomères)
2'-[6-amino-5-(trifluorométhyl)pyridin-3-yl]-N-[(1R)-1-(4-cyanopyridin-2-yl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(pyridin-3-yl)éthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(1-méthyl-1H-1,2,3-triazol-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-{[3-(trifluorométhyl)phényl]méthyl}-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-(1-phényl-1H-pyrazol-4-yl)éthyl]-5',6'-dihydrospiro[pyrrolidine-3,4'-pyrrolo[1,2-b]pyrazole]-1-carboxamide (mélange de diastéréomères)
2'-[6-amino-5-(trifluorométhyl)pyridin-3-yl]-N-[(3S)-2,3-dihydro-1-benzofuran-3-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(6-cyanopyridin-3-yl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-{(1S)-1-[1-méthyl-3-(trifluorométhyl)-1H-pyrazol-4-yl]éthyl}-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(pyridin-2-yl)éthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-benzyl-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-phényléthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-(1,2-oxazol-3-yl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-(1,5-diméthyl-1H-pyrazol-4-yl)éthyl]-5',6'-dihydrospiro[pyrrolidine-3,4'-pyrrolo[1,2-b]pyrazole]-1-carboxamide (mélange de diastéréomères)
(*rac*)-2'-[6-amino-5-(trifluorométhyl)pyridin-3-yl]-N-[2-(oxan-4-yl)propan-2-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(pyridin-4-yl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-(1-éthyl-5-méthyl-1H-pyrazol-4-yl)éthyl]-5',6'-dihydrospiro[pyrrolidine-3,4'-pyrrolo[1,2-b]pyrazole]-1-carboxamide (mélange de diastéréomères)
(*rac*)-2'-[6-amino-5-(trifluorométhyl)pyridin-3-yl]-N-[(pyridin-3-yl)méthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-2-méthoxy-1-phényléthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-{[3-(trifluorométhyl)bicyclo[1.1.1]pentan-1-yl]méthyl}-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[(pyridin-4-yl)méthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(3-cyanopyridin-2-yl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-(1-méthyl-1H-benzimidazol-2-yl)éthyl]-5',6'-dihydrospiro[pyrrolidine-3,4'-pyrrolo[1,2-b]pyrazole]-1-carboxamide (mélange de diastéréomères)
2'-[6-amino-5-(trifluorométhyl)pyridin-3-yl]-N-[1-(4-fluorophényl)-2-méthoxyéthyl]-5',6'-dihydrospiro[pyrrolidine-3,4'-pyrrolo[1,2-b]pyrazole]-1-carboxamide (mélange d'isomères)
(*rac*)-2'-[6-amino-5-(trifluorométhyl)pyridin-3-yl]-N-[(pyridin-2-yl)méthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-(1-éthyl-1H-imidazol-2-yl)éthyl]-5',6'-dihydrospiro[pyrrolidine-3,4'-pyrrolo[1,2-b]pyrazole]-1-carboxamide (mélange de diastéréomères)
2'-[6-amino-5-(trifluorométhyl)pyridin-3-yl]-N-[(1S)-1-(1-méthyl-1H-imidazol-2-yl)éthyl]-5',6'-dihydrospiro[pyrrolidine-3,4'-pyrrolo[1,2-b]pyrazole]-1-carboxamide (mélange de diastéréomères)
(*rac*)-2'-[6-amino-5-(trifluorométhyl)pyridin-3-yl]-N-[(pyrazin-2-yl)méthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-2-méthoxy-1-(pyridin-3-yl)éthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[(pyridazin-4-yl)méthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[(1-méthylpipéridin-4-yl)méthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[3-(méthylsulfamoyl)pyridin-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[(1-méthyl-2-oxabicyclo[2.1.1]hexan-4-yl)méthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[(1-méthyl-2-oxopipéridin-4-yl)méthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-(2-cyclopropylpropan-2-yl)-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(4-cyanophényl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-méthyl-2,3-dihydro-1H-indén-1-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[cyclopropyl(phényl)méthyl]-5',6'-dihydrospiro[pyrrolidine-3,4'-pyrrolo[1,2-b]pyrazole]-1-carboxamide (mélange d'isomères)
2'-[6-amino-5-(trifluorométhyl)pyridin-3-yl]-N-[-2-méthyl-1-phénylpropyl]-5',6'-dihydrospiro[pyrrolidine-3,4'-pyrrolo[1,2-b]pyrazole]-1-carboxamide (mélange d'isomères)
2'-[6-amino-5-(trifluorométhyl)pyridin-3-yl]-N-[(1S)-1-(pyridin-4-yl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[2,2,2-trifluoro-1-phényléthyl]-5',6'-dihydrospiro[pyrrolidine-3,4'-pyrrolo[1,2-b]pyrazole]-1-carboxamide (mélange d'isomères)
2'-[6-amino-5-(trifluorométhyl)pyridin-3-yl]-N-[1-(pyrazin-2-yl)éthyl]-5',6'-dihydrospiro[pyrrolidine-3,4'-pyrrolo[1,2-b]pyrazole]-1-carboxamide (mélange d'isomères)
2'-[6-amino-5-(trifluorométhyl)pyridin-3-yl]-N-[(1R)-2-méthoxy-1-phényléthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[(pyridazin-3-yl)méthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[1-(2-cyanophényl)cyclobutyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-2,3-dihydro-1H-indén-1-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-(4-fluorophényl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-(2-cyanophényl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(6-cyanopyridin-3-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(pyridin-2-yl)cyclobutyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(pyridin-4-yl)méthyl]-5',6'-dihydrospiro[azétidine-3,4'-pyrrolo[1,2-b]pyrazole]-1-carboxamide
(3'RS)-2-[6-amino-5-(trifluorométhyl)pyridin-3-yl]-N-[(1R)-1-(4-cyanophényl)éthyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-[(1R)-1-(3-cyanopyridin-2-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(4-cyanophényl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(2-cyanopyridin-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(2-chloro-4-cyanophényl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(2-fluorophényl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(1-éthyl-3,5-diméthyl-1H-pyrazol-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(pyridin-2-yl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(1-méthyl-1H-pyrazol-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(1,3,4-triméthyl-1H-pyrazol-5-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-{1-[3-(difluorométhyl)-1-méthyl-1H-pyrazol-4-yl]éthyl}-5',6'-dihydrospiro[azétidine-3,4'-pyrrolo[1,2-b]pyrazole]-1-carboxamide
(*rac*)-2-[6-amino-5-(trifluorométhyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-[1-(1-éthyl-5-méthyl-1H-pyrazol-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(1,5-diméthyl-1H-pyrazol-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(1,3,5-triméthyl-1H-pyrazol-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(pyridin-3-yl)éthyl]-5',6'-dihydrospiro[azétidine-3,4'-pyrrolo[1,2-b]pyrazole]-1-carboxamide
2-[6-amino-5-(trifluorométhyl)pyridin-3-yl]-N-[(1R)-1-(2-cyanopyridin-4-yl)éthyl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1R)-1-(3-cyanopyridin-2-yl)éthyl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1R)-1-(6-cyanopyridin-3-yl)éthyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-[(pyridin-2-yl)méthyl]-5',6'-dihydrospiro[azétidine-3,4'-pyrrolo[1,2-b]pyrazole]-1-carboxamide
(*rac*)-2-[6-amino-5-(trifluorométhyl)pyridin-3-yl]-N-[(1R)-1-(2-cyanophényl)éthyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-[(1R)-1-(4-fluorophényl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(4-fluorophényl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(pyridin-3-yl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(2-fluorophényl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(pyridin-3-yl)cyclobutyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(4-fluorophényl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(pyridin-4-yl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(3-fluorophényl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1S)-1-(3-chloropyridin-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1-méthyl-2-oxabicyclo[2.1.1]hexan-4-yl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-{[3-(trifluorométhyl)bicyclo[1.1.1]pentan-1-yl]méthyl}-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(2-chlorophényl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(3-fluorophényl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(3-fluoropyridin-2-yl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(2-chlorophényl)cyclobutyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(3-fluorophényl)cyclobutyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-phénylpropyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(pyrimidin-4-yl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(3-fluoropyridin-2-yl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(pyrimidin-5-yl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(3-chloropyridin-4-yl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(3-fluorophényl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(pyridin-3-yl)méthyl]-5',6'-dihydrospiro[azétidine-3,4'-pyrrolo[1,2-b]pyrazole]-1-carboxamide
(*rac*)-2-[6-amino-5-(trifluorométhyl)pyridin-3-yl]-N-benzyl-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[(1R)-1-(2-méthylpyrimidin-5-yl)éthyl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[(1R)-1-(pyridin-3-yl)éthyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-benzyl-5',6'-dihydrospiro[azétidine-3,4'-pyrrolo[1,2-b]pyrazole]-1-carboxamide
2-[6-amino-5-(trifluorométhyl)pyridin-3-yl]-N-[2-(2-fluorophényl)propan-2-yl]-6,7-dihydrospiro[pyrazolo[5,1-c][1,4]oxazine-4,3'-pyrrolidine]-1'-carboxamide (énantiomère 1)
2-[6-amino-5-(trifluorométhyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (énantiomère 1)
2-[6-amino-5-(trifluorométhyl)pyridin-3-yl]-N-[2-(2-fluorophényl)propan-2-yl]-6,7-dihydrospiro[pyrazolo[5,1-c][1,4]oxazine-4,3'-pyrrolidine]-1'-carboxamide (énantiomère 2)
2-[6-amino-5-(trifluorométhyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide (énantiomère 2)
2-[6-amino-5-(trifluorométhyl)pyridin-3-yl]-N-[(1R)-1-(4-cyanopyridin-2-yl)éthyl1]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[2-(1-méthyl-1H-1,2,3-triazol-5-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1R)-1-(4-fluorophényl)éthyl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1R)-2,3-dihydro-1H-indén-1-yl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1R)-1-(5-fluoropyridin-3-yl)éthyl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1R)-1-(3-fluorophényl)éthyl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1R)-1-phénylpropyl]-6,7-dihydrospiro[pyrazolo[5,1-c][1,4]oxazine-4,3'-pyrrolidine]-1'-carboxamide
(3'RS)-2-[6-amino-5-(trifluorométhyl)pyridin-3-yl]-N-[(3S)-2,3-dihydro-1-benzofuran-3-yl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[(bicyclo[1.1.1]pentan-1-yl)méthyl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1S)-2-méthoxy-1-phényléthyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-[(1R)-1-(1,2-oxazol-3-yl)éthyl]-5',6'-dihydrospiro[azétidine-3,4'-pyrrolo[1,2-b]pyrazole]-1-carboxamide
2-[6-amino-5-(trifluorométhyl)pyridin-3-yl]-N-[(1R)-1-(2-fluorophényl)éthyl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[2-(3-fluorophényl)propan-2-yl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-[(1R)-1-(1-phényl-1H-pyrazol-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1S)-2-méthoxy-1-phényléthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(3-fluorobicyclo[1.1.1]pentan-1-yl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(bicyclo[1.1.1]pentan-1-yl)méthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1S)-1-(pyridin-3-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(2-méthylpyrimidin-5-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(1-méthyl-1H-1,2,3-triazol-4-yl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[2-(3-chloropyridin-4-yl)propan-2-yl]-5',6'-dihydrospiro[azétidine-3,4'-pyrrolo[1,2-b]pyrazole]-1-carboxamide
2'-[6-amino-5-(trifluorométhyl)pyridin-3-yl]-N-[1-(pyridin-4-yl)cyclobutyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-phényléthyl]-5',6'-dihydrospiro[azétidine-3,4'-pyrrolo[1,2-b]pyrazole]-1-carboxamide
2-[6-amino-5-(trifluorométhyl)pyridin-3-yl]-N-[(1S)-1-(pyridin-3-yl)éthyl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[(3-fluorobicyclo[1.1.1]pentan-1-yl)méthyl]-6,7-dihydrospiro[pyrazolo[5,1-c][1,4]oxazine-4,3'-pyrrolidine]-1'-carboxamide
2-[6-amino-5-(trifluorométhyl)pyridin-3-yl]-N-[(1S)-1-(pyridin-4-yl)éthyl]-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-(1-phénylcyclobutyl)-5',6'-dihydrospiro[azétidine-3,4'-pyrrolo[1,2-b]pyrazole]-1-carboxamide
2'-(6-amino-5-cyanopyridin-3-yl)-N-[1-(pyridin-4-yl)cyclobutyl]-5',6'-dihydrospiro[azétidine-3,4'-pyrrolo[1,2-b]pyrazole]-1-carboxamide
2'-(6-amino-5-cyanopyridin-3-yl)-N-[1-(3-fluorophényl)cyclobutyl]-5',6'-dihydrospiro[azétidine-3,4'-pyrrolo[1,2-b]pyrazole]-1-carboxamide
2'-(6-amino-5-cyanopyridin-3-yl)-N-[2-(3-chloropyridin-4-yl)propan-2-yl]-5',6'-dihydrospiro[azétidine-3,4'-pyrrolo[1,2-b]pyrazole]-1-carboxamide
2'-(6-amino-5-cyanopyridin-3-yl)-N-[(1R)-1-(5-fluoropyridin-3-yl)éthyl]-5',6'-dihydrospiro[azétidine-3,4'-pyrrolo[1,2-b]pyrazole]-1-carboxamide
2'-(6-amino-5-cyanopyridin-3-yl)-N-[(1R)-1-(4-fluorophényl)éthyl]-5',6'-dihydrospiro[azétidine-3,4'-pyrrolo[1,2-b]pyrazole]-1-carboxamide
2'-(6-amino-5-cyanopyridin-3-yl)-N-[2-(3-fluorophényl)propan-2-yl]-5',6'-dihydrospiro[azétidine-3,4'-pyrrolo[1,2-b]pyrazole]-1-carboxamide
2'-(6-amino-5-cyanopyridin-3-yl)-N-[(1S)-1-(3-chloropyridin-4-yl)éthyl]-5',6'-dihydrospiro[azétidine-3,4'-pyrrolo[1,2-b]pyrazole]-1-carboxamide
2'-(6-amino-5-cyanopyridin-3-yl)-N-[(1-méthyl-2-oxabicyclo[2.1.1]hexan-4-yl)méthyl]-5',6'-dihydrospiro[azétidine-3,4'-pyrrolo[1,2-b]pyrazole]-1-carboxamide
2'-(6-amino-5-cyanopyridin-3-yl)-N-[(1R)-1-(4-cyanopyridin-2-yl)éthyl]-5',6'-dihydrospiro[azétidine-3,4'-pyrrolo[1,2-b]pyrazole]-1-carboxamide
2'-(6-amino-5-cyanopyridin-3-yl)-N-[(1R)-1-(2-cyanopyridin-4-yl)éthyl]-5',6'-dihydrospiro[azétidine-3,4'-pyrrolo[1,2-b]pyrazole]-1-carboxamide
2'-(6-amino-5-cyanopyridin-3-yl)-N-[(1R)-1-(2-chloro-4-cyanophényl)éthyl]-5',6'-dihydrospiro[azétidine-3,4'-pyrrolo[1,2-b]pyrazole]-1-carboxamide
2'-(6-amino-5-cyanopyridin-3-yl)-N-[1-(2-fluorophényl)cyclobutyl]-5',6'-dihydrospiro[azétidine-3,4'-pyrrolo[1,2-b]pyrazole]-1-carboxamide
2'-(6-amino-5-cyanopyridin-3-yl)-N-[2-(4-fluorophényl)propan-2-yl]-5',6'-dihydrospiro[azétidine-3,4'-pyrrolo[1,2-b]pyrazole]-1-carboxamide
2'-(6-amino-5-cyanopyridin-3-yl)-N-[1-(2-chlorophényl)cyclobutyl]-5',6'-dihydrospiro[azétidine-3,4'-pyrrolo[1,2-b]pyrazole]-1-carboxamide
2'-(6-amino-5-cyanopyridin-3-yl)-N-[2-(1-méthyl-1H-1,2,3-triazol-4-yl)propan-2-yl]-5',6'-dihydrospiro[azétidine-3,4'-pyrrolo[1,2-b]pyrazole]-1-carboxamide
2'-(6-amino-5-cyanopyridin-3-yl)-N-[2-(pyridin-2-yl)propan-2-yl]-5',6'-dihydrospiro[azétidine-3,4'-pyrrolo[1,2-b]pyrazole]-1-carboxamide
2'-(6-amino-5-cyanopyridin-3-yl)-N-[2-(pyridin-4-yl)propan-2-yl]-5',6'-dihydrospiro[azétidine-3,4'-pyrrolo[1,2-b]pyrazole]-1-carboxamide
2'-(6-amino-5-cyanopyridin-3-yl)-N-[(1R)-1-phénylpropyl]-5',6'-dihydrospiro[azétidine-3,4'-pyrrolo[1,2-b]pyrazole]-1-carboxamide
2'-(6-amino-5-cyanopyridin-3-yl)-N-[(1R)-1-(3-fluorophényl)éthyl]-5',6'-dihydrospiro[azétidine-3,4'-pyrrolo[1,2-b]pyrazole]-1-carboxamide
2'-(6-amino-5-cyanopyridin-3-yl)-N-[(1R)-1-(2-cyanophényl)éthyl]-5',6'-dihydrospiro[azétidine-3,4'-pyrrolo[1,2-b]pyrazole]-1-carboxamide
(*rac*)-2'-(6-amino-5-cyanopyridin-3-yl)-N-[2-(pyridin-4-yl)propan-2-yl]-5',6'-dihydrospiro[pyrrolidine-3,4'-pyrrolo[1,2-b]pyrazole]-1-carboxamide
2'-(6-amino-5-cyanopyridin-3-yl)-N-[(1R)-1-(5-fluoropyridin-3-yl)éthyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[2-(1-méthyl-1H-1,2,3-triazol-4-yl)propan-2-yl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-{pyridin-2-yl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(pyridin-2-yl)cyclobutyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[1-(2-chlorophényl)cyclobutyl]-5',6'-dihydrospiro[pyrrolidine-3,4'-pyrrolo[1,2-b]pyrazole]-1-carboxamide
2-(6-amino-5-cyanopyridin-3-yl)-N-[(1R)-1-phényléthyl]-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-phényléthyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[1-(2-chlorophényl)cyclobutyl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[1-(4-fluorophényl)cyclobutyl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[2-(4-fluorophényl)propan-2-yl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide
2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (énantiomère 2)
2-(6-amino-5-cyanopyridin-3-yl)-N-[1-(2-chlorophényl)cyclobutyl]-6,7-dihydrospiro[pyrazolo[5,1-c][1,4]oxazine-4,3'-pyrrolidine]-1'-carboxamide (énantiomère 2)
2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide (énantiomère 1)
2-(6-amino-5-cyanopyridin-3-yl)-N-[1-(2-chlorophényl)cyclobutyl]-6,7-dihydrospiro[pyrazolo[5,1-c][1,4]oxazine-4,3'-pyrrolidine]-1'-carboxamide (énantiomère 1)
2'-(6-amino-5-cyanopyridin-3-yl)-N-[1-(pyridin-3-yl)cyclobutyl]-5',6'-dihydrospiro[azétidine-3,4'-pyrrolo[1,2-b]pyrazole]-1-carboxamide
2'-(6-amino-5-cyanopyridin-3-yl)-N-[1-(pyridin-2-yl)cyclobutyl]-5',6'-dihydrospiro[azétidine-3,4'-pyrrolo[1,2-b]pyrazole]-1-carboxamide
2-(6-amino-5-cyanopyridin-3-yl)-N-[1-(pyridin-4-yl)cyclobutyl]-6,7-dihydrospiro[pyrazolo[5,1-c][1,4]oxazine-4,3'-pyrrolidine]-1'-carboxamide (énantiomère 1)
2'-(6-amino-5-cyanopyridin-3-yl)-N-(1-phénylcyclobutyl)-5',6'-dihydrospiro[azétidine-3,4'-pyrrolo[1,2-b]pyrazole]-1-carboxamide
2'-(6-amino-5-cyanopyridin-3-yl)-N-[(3S)-2,3-dihydro-1-benzofuran-3-yl]-5',6'-dihydrospiro[pyrrolidine-3,4'-pyrrolo[1,2-b]pyrazole]-1-carboxamide
2-(6-amino-5-cyanopyridin-3-yl)-N-[(3S)-2,3-dihydro-1-benzofuran-3-yl]-6,7-dihydrospiro[pyrazolo[5,1-c][1,4]oxazine-4,3'-pyrrolidine]-1'-carboxamide
2-(6-amino-5-cyanopyridin-3-yl)-N-[(1R)-1-(2-fluorophényl)éthyl]-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-(2-fluorophényl)éthyl]-5',6'-dihydrospiro[pyrrolidine-3,4'-pyrrolo[1,2-b]pyrazole]-1-carboxamide
(*rac*)-2'-(6-amino-5-cyanopyridin-3-yl)-N-[2-(4-fluorophényl)propan-2-yl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-[2-(1-méthyl-1H-1,2,3-triazol-4-yl)propan-2-yl]-6,7-dihydrospiro[pyrazolo[5,1-c][1,4]oxazine-4,3'-pyrrolidine]-1'-carboxamide
(*rac*)-2-(6-amino-5-cyanopyridin-3-yl)-N-[(1R)-1-(5-fluoropyridin-3-yl)éthyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-(1-phénylcyclobutyl)-5',6'-dihydrospiro[pyrrolidine-3,4'-pyrrolo[1,2-b]pyrazole]-1-carboxamide
(*rac*)-2'-(6-amino-5-cyanopyridin-3-yl)-*N*-[1*-(*4-fluorophényl)cyclobutyl]-5',6'-dihydrospiro[pyrrolidine-3,4'-pyrrolo[1,2-b]pyrazole]-1-carboxamide
(*rac*)-2-(6-amino-5-cyanopyridin-3-y1)-N-[1-{pyridin-4-yl)cyclobutyl]-6,7-dihydrospiro[pyrazolo[5,1-c][1,4]oxazine-4,3'-pyrrolidine]-1'-carboxamide
(*rac*)-2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide
(*rac*)-2-(6-amino-5-cyanopyridin-3-yl)-N-(1-phénylcyclobutyl)-6,7-dihydrospiro[pyrazolo[5,1-c][1,4]oxazine-4,3'-pyrrolidine]-1'-carboxamide
2'-(6-amino-5-cyanopyridin-3-yl)-N-[(1R)-1-(1,3,4-triméthyl-1H-pyrazol-5-yl)éthyl]-5',6'-dihydrospiro[azétidine-3,4'-pyrrolo[1,2-b]pyrazole]-1-carboxamide
(*rac*)-2-(6-amino-5-cyanopyridin-3-yl)-N-[2-(pyridin-4-yl)propan-2-yl]-6,7-dihydrospiro[pyrazolo[5,1-c][1,4]oxazine-4,3'-pyrrolidine]-1'-carboxamide
2'-[6-amino-5-(difluorométhoxy)pyridin-3-yl]-N-[1-(pyridin-3-yl)cyclobutyl]-5',6'-dihydrospiro[azétidine-3,4'-pyrrolo[1,2-b]pyrazole]-1-carboxamide
(*ra*c)-2'-[6-amino-5-(trifluorométhoxy)pyridin-3-yl]-N-[2-(1-méthyl-1H-1,2,3-triazol-4-yl)propan-2-yl]-5',6'-dihydrospiro[pyrrolidine-3,4'-pyrrolo[1,2-b]pyrazole]-1-carboxamide
2'-[6-amino-5-(trifluorométhoxy)pyridin-3-yl]-N-[(1R)-1-(2-méthylpyrimidin-5-yl)éthyl]-5',6'-dihydrospiro[pyrrolidine-3,4'-pyrrolo[1,2-b]pyrazole]-1-carboxamide
2'-[6-amino-5-(trifluorométhoxy)pyridin-3-yl]-N-[(1R)-1-(pyridin-2-yl)éthyl]-5',6'-dihydrospiro[pyrrolidine-3,4'-pyrrolo[1,2-b]pyrazole]-1-carboxamide
2'-[6-amino-5-(trifluorométhoxy)pyridin-3-yl]-N-[(1R)-1-phényléthyl]-5',6'-dihydrospiro[pyrrolidine-3,4'-pyrrolo[1,2-b]pyrazole]-1-carboxamide (mélange de stéréoisomères)
2'-[6-amino-5-(trifluorométhoxy)pyridin-3-yl]-N-[(1R)-1-(pyridin-3-yl)éthyl]-5',6'-dihydrospiro[pyrrolidine-3,4'-pyrrolo[1,2-b]pyrazole]-1-carboxamide
2'-[6-amino-5-(trifluorométhoxy)pyridin-3-yl]-N-[(1S)-1-(pyridin-4-yl)éthyl]-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-[2-(4-fluorophényl)propan-2-yl]-5',6'-dihydrospiro[pyrrolidine-3,4'-pyrrolo[1,2-b]pyrazole]-1-carboxamide
2'-[6-amino-5-(trifluorométhoxy)pyridin-3-yl]-N-[(1R)-1-(4-fluorophényl)éthyl]-5',6'-dihydrospiro[pyrrolidine-3,4'-pyrrolo[1,2-b]pyrazole]-1-carboxamide
(*rac*)-2-[6-amino-5-(trifluorométhoxy)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
2'-[6-amino-5-(difluorométhoxy)pyridin-3-yl]-N-(1-phénylcyclobutyl)-5',6'-dihydrospiro[azétidine-3,4'-pyrrolo[1,2-b]pyrazole]-1-carboxamide
(*rac*)-2'-[6-amino-5-(trifluorométhoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[2-(2-fluorophényl)propan-2-yl]-5',6'-dihydrospiro[azétidine-3,4'-pyrrolo[1,2-b]pyrazole]-1-carboxamide
(*rac*)-2-[6-amino-5-(difluorométhoxy)pyridin-3-yl]-N-[2-(3-fluorophényl)propan-2-yl]-6,7-dihydrospiro[pyrazolo[5,1-c][1,4]oxazine-4,3'-pyrrolidine]-1'-carboxamide
2-[6-amino-5-(difluorométhoxy)pyridin-3-yl]-N-[(1S)-1-(3-chloropyridin-4-yl)éthyl]-6,7-dihydrospiro[pyrazolo[5,1-c][1,4]oxazine-4,3'-pyrrolidine]-1'-carboxamide
2-[6-amino-5-(trifluorométhoxy)pyridin-3-yl]-N-[1-(1,3,5-triméthyl-1H-pyrazol-4-yl)éthyl]-6,7-dihydrospiro[pyrazolo[5,1-c][1,4]oxazine-4,3'-pyrrolidine]-1'-carboxamide
2-[6-amino-5-(trifluorométhoxy)pyridin-3-yl]-N-[1-(1-éthyl-3,5-diméthyl-1H-pyrazol-4-yl)éthyl]-6,7-dihydrospiro[pyrazolo[5,1-c][1,4]oxazine-4,3'-pyrrolidine]-1'-carboxamide
2-[6-amino-5-(trifluorométhoxy)pyridin-3-yl]-N-[(1R)-1-(2-cyanophényl)éthyl]-6,7-dihydrospiro[pyrazolo[5,1-c][1,4]oxazine-4,3'-pyrrolidine]-1'-carboxamide
(*rac*)-2'-[6-amino-5-(difluorométhoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2-[6-amino-5-(trifluorométhoxy)pyridin-3-yl]-N-[(1R)-1-(4-fluorophényl)éthyl]-6,7-dihydrospiro[pyrazolo[5,1-c][1,4]oxazine-4,3'-pyrrolidine]-1'-carboxamide
2-[6-amino-5-(trifluorométhoxy)pyridin-3-yl]-N-[(1R)-1-phénylpropyl]-6,7-dihydrospiro[pyrazolo[5,1-c][1,4]oxazine-4,3'-pyrrolidine]-1'-carboxamide
2-[6-amino-5-(trifluorométhoxy)pyridin-3-yl]-N-[(1R)-1-phényléthyl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[1-(pyridin-3-yl)cyclobutyl]-6,7-dihydrospiro[pyrazolo[5,1-c][1,4]oxazine-4,3'-pyrrolidine]-1'-carboxamide
2-[6-amino-5-(difluorométhoxy)pyridin-3-yl]-N-[(1R)-1-(2-chloro-4-cyanophényl)éthyl]-6,7-dihydrospiro[pyrazolo[5,1-c][1,4]oxazine-4,3'-pyrrolidine]-1'-carboxamide
(*rac*)-2-[6-amino-5-(difluorométhoxy)pyridin-3-yl]-N-[2-(4-fluorophényl)propan-2-yl]-6,7-dihydrospiro[pyrazolo[5,1-c][1,4]oxazine-4,3'-pyrrolidine]-1'-carboxamide
(*rac*)-2-[6-amino-5-(difluorométhoxy)pyridin-3-yl]-N-[1-(2-fluorophényl)cyclobutyl]-6,7-dihydrospiro[pyrazolo[5,1-c][1,4]oxazine-4,3'-pyrrolidine]-1'-carboxamide
(*rac*)-2'-[6-amino-5-(difluorométhoxy)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[1-(2-chlorophényl)cyclobutyl]-5',6'-dihydrospiro[azétidine-3,4'-pyrrolo[1,2-b]pyrazole]-1-carboxamide
2'-[6-amino-5-(trifluorométhoxy)pyridin-3-yl]-N-[(1R)-1-(4-cyanophényl)éthyl]-5',6'-dihydrospiro[azétidine-3,4'-pyrrolo[1,2-b]pyrazole]-1-carboxamide
2'-[6-amino-5-(difluorométhoxy)pyridin-3-yl]-N-[(1R)-1-(1,3,4-triméthyl-1H-pyrazol-5-yl)éthyl]-5',6'-dihydrospiro[azétidine-3,4'-pyrrolo[1,2-b]pyrazole]-1-carboxamide
(*rac*)-2'-[6-amino-5-(trifluorométhoxy)pyridin-3-yl]-N-[1-(2-chlorophényl)cyclobutyl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[(1R)-1-(5-fluoropyridin-3-yl)éthyl]-5',6'-dihydrospiro[azétidine-3,4'-pyrrolo[1,2-b]pyrazole]-1-carboxamide
2'-[6-amino-5-(trifluorométhoxy)pyridin-3-yl] N-[(1R)-1-(4-fluorophényl)éthyl]-5',6'-dihydrospiro[azétidine-3,4'-pyrrolo[1,2-b]pyrazole]-1-carboxamide
2'-[6-amino-5-(trifluorométhoxy)pyridin-3-yl]-N-[(1R)-1-(2-chloro-4-cyanophényl)éthyl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[(1R)-1-(2-cyanophényl)éthyl]-5',6'-dihydrospiro[azétidine-3,4'-pyrrolo[1,2-b]pyrazole]-1-carboxamide
2'-[6-amino-5-(difluorométhoxy)pyridin-3-yl]-N-[2-(pyridin-4-yl)propan-2-yl]-5',6'-dihydrospiro[azétidine-3,4'-pyrrolo[1,2-b]pyrazole]-1-carboxamide
2'-[6-amino-5-(difluorométhoxy)pyridin-3-yl]-N-[(1R)-1-(4-fluorophényl)éthyl]-5',6'-dihydrospiro[azétidine-3,4'-pyrrolo[1,2-b]pyrazole]-1-carboxamide
2'-[6-amino-5-(difluorométhoxy)pyridin-3-yl]-N-[(1R)-1-(2-cyanopyridin-4-yl)éthyl]-5',6'-dihydrospiro[azétidine-3,4'-pyrrolo[1,2-b]pyrazole]-1-carboxamide
(*rac*)-2'-[6-amino-5-(trifluorométhoxy)pyridin-3-yl]-N-[1-(2-fluorophényl)cyclobutyl]-5',6'-dihydrospiro[pyrrolidine-3,4'-pyrrolo[1,2-b]pyrazole]-1-carboxamide
(*rac*)-2-[6-amino-5-(trifluorométhoxy)pyridin-3-yl]-N-[2-(2-chlorophényl)propan-2-yl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[2-(4-fluorophényl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluorométhoxy)pyridin-3-yl]-N-[2-(3-fluorophényl)propan-2-yl]-5',6'-dihydrospiro[pyrrolidine-3,4'-pyrrolo[1,2-b]pyrazole]-1-carboxamide
2'-[6-amino-5-(trifluorométhoxy)pyridin-3-yl]-N-[(1S)-1-(3-chloropyridin-4-yl)éthyl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[2-(4-fluorophényl)propan-2-yl]-5',6'-dihydrospiro[azétidine-3,4'-pyrrolo[1,2-b]pyrazole]-1-carboxamide
2'-[6-amino-5-(difluorométhoxy)pyridin-3-yl]-N-[2-(2-chlorophényl)propan-2-yl]-5',6'-dihydrospiro[azétidine-3,4'-pyrrolo[1,2-b]pyrazole]-1-carboxamide
2-[6-amino-5-(trifluorométhoxy)pyridin-3-yl]-N-[(1R)-1-(3-fluorophényl)éthyl]-6,7-dihydrospiro[pyrazolo[5,1-c][1,4]oxazine-4,3'-pyrrolidine]-1'-carboxamide
2'-[6-amino-5-(trifluorométhoxy)pyridin-3-yl]-N-(1-phénylcyclobutyl)-5',6'-dihydrospiro[azétidine-3,4'-pyrrolo[1,2-b]pyrazole]-1-carboxamide
2'-[6-amino-5-(trifluorométhoxy)pyridin-3-yl]-N-benzyl-5',6'-dihydrospiro[azétidine-3,4'-pyrrolo[1,2-b]pyrazole]-1-carboxamide
2'-[6-amino-5-(trifluorométhoxy)pyridin-3-yl]-N-[(1R)-1-(4-cyanopyridin-2-yl)éthyl]-5',6'-dihydrospiro[azétidine-3,4'-pyrrolo[1,2-b]pyrazole]-1-carboxamide
2'-[6-amino-5-(trifluorométhoxy)pyridin-3-yl]-N-[(1R)-1-(2-cyanopyridin-4-yl)éthyl]-5',6'-dihydrospiro[azétidine-3,4'-pyrrolo[1,2-b]pyrazole]-1-carboxamide
2'-[6-amino-5-(trifluorométhoxy)pyridin-3-yl]-N-[(1R)-1-(5-fluoropyridin-3-yl)éthyl]-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-(1-phénylcyclobutyl)-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[2-(3-fluorophényl)propan-2-yl]-5',6'-dihydrospiro[azétidine-3,4'-pyrrolo[1,2-b]pyrazole]-1-carboxamide
2'-[6-amino-5-(difluorométhoxy)pyridin-3-yl]-N-[(1S)-1-(3-chloropyridin-4-yl)éthyl]-5',6'-dihydrospiro[azétidine-3,4'-pyrrolo[1,2-b]pyrazole]-1-carboxamide
2'-[6-amino-5-(difluorométhoxy)pyridin-3-yl]-N-[2-(3-chloropyridin-4-yl)propan-2-yl]-5',6'-dihydrospiro[azétidine-3,4'-pyrrolo[1,2-b]pyrazole]-1-carboxamide
2'-[6-amino-5-(difluorométhoxy)pyridin-3-yl]-N-[(1R)-1-(2-chloro-4-cyanophényl)éthyl]-5',6'-dihydrospiro[azétidine-3,4'-pyrrolo[1,2-b]pyrazole]-1-carboxamide
2'-[6-amino-5-(difluorométhoxy)pyridin-3-yl]-N-[1-(2-fluorophényl)cyclobutyl]-5',6'-dihydrospiro[azétidine-3,4'-pyrrolo[1,2-b]pyrazole]-1-carboxamide
(*rac*)-2'-[6-amino-5-(trifluorométhoxy)pyridin-3-yl]-N-[2-(2-chlorophényl)propan-2-yl]-5',6'-dihydrospiro[pyrrolidine-3,4'-pyrrolo[1,2-b]pyrazole]-1-carboxamide
2-[6-amino-5-(trifluorométhoxy)pyridin-3-yl]-N-[(1R)-1-(4-cyanopyridin-2-yl)éthyl]-6,7-dihydrospiro[pyrazolo[5,1-c][1,4]oxazine-4,3'-pyrrolidine]-1'-carboxamide
(*rac*)-2-[6-amino-5-(difluorométhoxy)pyridin-3-yl]-N-[1-(2-chlorophényl)cyclobutyl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[1-(pyridin-2-yl)cyclobutyl]-6,7-dihydrospiro[pyrazolo[5,1-c][1,4]oxazine-4,3'-pyrrolidine]-l'-carboxamide
2-[6-amino-5-(trifluorométhoxy)pyridin-3-yl]-N-[(1R)-1-(2-cyanopyridin-4-yl)éthyl]-6,7-dihydrospiro[pyrazolo[5,1-c][1,4]oxazine-4,3'-pyrrolidine]-1'-carboxamide
(*rac*)-2-[6-amino-5-(difluorométhoxy)pyridin-3-yl]-N-[2-(2-chlorophényl)propan-2-yl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[1-(2-chlorophényl)cyclobutyl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[1-(2-fluorophényl)cyclobutyl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
acétate de (*rac*)-2'-[6-amino-5-(trifluorométhoxy)pyridin-3-yl]-N-[1-(pyridin-4-yl)cyclobutyl]-5',6'-dihydro-1H-spiro[pyrrolidine-3,4'-pyrrolo[1,2-b]pyrazole]-1-(1/1)
(*rac*)-2-[6-amino-5-(trifluorométhoxy)pyridin-3-yl]-N-[(1-méthyl-2-oxabicyclo[2.1.1]hexan-4-yl)méthyl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[2-(2-fluorophényl)propan-2-yl]-6,7-dihydrospiro[pyrazolo[5,1-c][1,4]oxazine-4,3'-pyrrolidine]-1'-carboxamide
2-[6-amino-5-(trifluorométhoxy)pyridin-3-yl]-N-[(1R)-1-(2-chloro-4-cyanophényl)éthyl]-6,7-dihydrospiro[pyrazolo[5,1-c][1,4]oxazine-4,3'-pyrrolidine]-1'-carboxamide
2'-[6-amino-5-(difluorométhoxy)pyridin-3-yl]-N-[2-(pyridin-2-yl)propan-2-yl]-5',6'-dihydrospiro[azétidine-3,4'-pyrrolo[1,2-b]pyrazole]-1-carboxamide
2'-[6-amino-5-(difluorométhoxy)pyridin-3-yl]-N-[2-(1-méthyl-1H-1,2,3-triazol-4-yl)propan-2-yl]-5',6'-dihydrospiro[azétidine-3,4'-pyrrolo[1,2-b]pyrazole]-1-carboxamide
2-[6-amino-5-(trifluorométhoxy)pyridin-3-yl]-N-[(1S)-2-méthoxy-1-phényléthyl]-6,7-dihydrospiro[pyrazolo[5,1-c][1,4]oxazine-4,3'-pyrrolidine]-1'-carboxamide
2-[6-amino-5-(trifluorométhoxy)pyridin-3-yl]-N-[(1S)-1-(3-chloropyridin-4-yl)éthyl]-6,7-dihydrospiro[pyrazolo[5,1-c][1,4]oxazine-4,3'-pyrrolidine]-1'-carboxamide
(*rac*)-2-[6-amino-5-(trifluorométhoxy)pyridin-3-yl]-N-[2-(3-fluorophényl)propan-2-yl]-6,7-dihydrospiro[pyrazolo[5,1-c][1,4]oxazine-4,3'-pyrrolidine]-l'-carboxamide
(*rac*)-2'-[6-amino-5-(trifluorométhoxy)pyridin-3-yl]-N-[(1-méthyl-2-oxabicyclo[2.1.1]hexan-4-yl)méthyl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[(1R)-1-phénylpropyl]-5',6'-dihydrospiro[azétidine-3,4'-pyrrolo[1,2-b]pyrazole]-1-carboxamide
2'-[6-amino-5-(trifluorométhoxy)pyridin-3-yl]-N-[(1S)-2-méthoxy-1-phényléthyl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[(1R)-1-(3-fluorophényl)éthyl]-5',6'-dihydrospiro[azétidine-3,4'-pyrrolo[1,2-b]pyrazole]-1-carboxamide
2'-[6-amino-5-(trifluorométhoxy)pyridin-3-yl]-N-[(1S)-1-(pyridin-3-yl)éthyl]-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-[1-(3-fluorophényl)cyclobutyl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[1-(propan-2-yl)cyclobutyl]-5',6'-dihydrospiro[azétidine-3,4'-pyrrolo[1,2-b]pyrazole]-1-carboxamide
2'-[6-amino-5-(difluorométhoxy)pyridin-3-yl]-N-[1-(pyridin-4-yl)cyclobutyl]-5',6'-dihydrospiro[azétidine-3,4'-pyrrolo[1,2-b]pyrazole]-1-carboxamide
2'-[6-amino-5-(difluorométhoxy)pyridin-3-yl]-N-(bicyclo[2.2.1]heptan-1-yl)-5',6'-dihydrospiro[azétidine-3,4'-pyrrolo[1,2-b]pyrazole]-1-carboxamide
2'-[6-amino-5-(difluorométhoxy)pyridin-3-yl]-N-(1-benzylcyclobutyl)-5',6'-dihydrospiro[azétidine-3,4'-pyrrolo[1,2-b]pyrazole]-1-carboxamide
2'-[6-amino-5-(difluorométhoxy)pyridin-3-yl]-N-[1-(4-chlorophényl)cyclobutyl]-5',6'-dihydrospiro[azétidine-3,4'-pyrrolo[1,2-b]pyrazole]-1-carboxamide
2'-[6-amino-5-(difluorométhoxy)pyridin-3-yl]-N-(1-méthylcyclobutyl)-5',6'-dihydrospiro[azétidine-3,4'-pyrrolo[1,2-b]pyrazole]-1-carboxamide
(*rac*)-2-[6-amino-5-(trifluorométhoxy)pyridin-3-yl]-N-[1-(1,3,4-triméthyl-1H-pyrazol-5-yl)éthyl]-6,7-dihydrospiro[pyrazolo[5,1-c][1,4]oxazine-4,3'-pyrrolidine]-l'-carboxamide
2-[6-amino-5-(trifluorométhoxy)pyridin-3-yl]-N-[(1R)-2,3-dihydro-1H-indén-1-yl]-6,7-dihydrospiro[pyrazolo[5,1-c][1,4]oxazine-4,3'-pyrrolidine]-1'-carboxamide
2'-[6-amino-5-(difluorométhoxy)pyridin-3-yl]-N-[1-(pyridin-2-yl)cyclobutyl]-5',6'-dihydrospiro[azétidine-3,4'-pyrrolo[1,2-b]pyrazole]-1-carboxamide
2'-[6-amino-5-(difluorométhoxy)pyridin-3-yl]-N-[1-(3-fluorophényl)cyclobutyl]-5',6'-dihydrospiro[azétidine-3,4'-pyrrolo[1,2-b]pyrazole]-1-carboxamide
(*rac*)-2'-[6-amino-5-(trifluorométhoxy)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
2-[6-amino-5-(difluorométhoxy)pyridin-3-yl]-N-[(1S)-2-méthoxy-1-phényléthyl]-6,7-dihydrospiro[pyrazolo[5,1-c][1,4]oxazine-4,3'-pyrrolidine]-1'-carboxamide
2'-[6-amino-5-(difluorométhoxy)pyridin-3-yl]-N-[(1R)-1-phényléthyl]-5',6'-dihydrospiro[pyrrolidine-3,4'-pyrrolo[1,2-b]pyrazole]-1-carboxamide
(*rac*)-2'-[6-amino-5-(difluorométhoxy)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(*rac*)-2'-[6-amino-5-(difluorométhoxy)pyridin-3-yl]-N-(1-phénylcyclobutyl)-5',6'-dihydrospiro[pyrrolidine-3,4'-pyrrolo[1,2-b]pyrazole]-1-carboxamide
(*rac*)-2'-[6-amino-5-(difluorométhoxy)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[(1R)-1-(4-fluorophényl)éthyl]-5',6'-dihydrospiro[pyrrolidine-3,4'-pyrrolo[1,2-b]pyrazole]-1-carboxamide
(*rac*)-2'-[6-amino-5-(difluorométhoxy)pyridin-3-yl]-N-[2-(4-fluorophényl)propan-2-yl]-5',6'-dihydrospiro[pyrrolidine-3,4'-pyrrolo[1,2-b]pyrazole]-1-carboxamide
2'-[6-amino-5-(difluorométhoxy)pyridin-3-yl]-N-[(1R)-1-(5-fluoropyridin-3-yl)éthyl]-5',6'-dihydrospiro[pyrrolidine-3,4'-pyrrolo[1,2-b]pyrazole]-1-carboxamide
2'-{6-amino-5-[(1R)-1-(pyridin-2-yl)éthoxy]pyridin-3-yl}-N-[(1R)-1-phényléthyl]-5',6'-dihydrospiro[pyrrolidine-3,4'-pyrrolo[1,2-b]pyrazole]-1-carboxamide
2'-{6-amino-5-[(1R)-1-(pyridin-2-yl)éthoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-{6-amino-5-[(1S)-1-(pyridin-2-yl)éthoxy]pyridin-3-yl}-N-[(1R)-1-phényléthyl]-5',6'-dihydrospiro[pyrrolidine-3,4'-pyrrolo[1,2-b]pyrazole]-1-carboxamide
2'-{6-amino-5-[(1S)-1-(pyridin-2-yl)éthoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-{6-amino-5-[(trifluorométhyl)sulfanyl]pyridin-3-yl}-N-[(1R)-1-(3-fluorophényl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(3R)-2,3-dihydro-1-benzofuran-3-yl]-5',6'-dihydrospiro[pyrrolidine-3,4'-pyrrolo[1,2-b]pyrazole]-1-carboxamide
2'-{6-amino-5-[(1R)-1-(pyridin-3-yl)éthoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-(1-méthyl-1H-imidazol-5-yl)éthyl]-5',6'-dihydrospiro[pyrrolidine-3,4'-pyrrolo[1,2-b]pyrazole]-1-carboxamide (mélange de diastéréomères)
2'-{6-amino-5-[(1R)-1-(pyridin-3-yl)éthoxy]pyridin-3-yl}-N-cyclobutyl-5',6'-dihydrospiro[pyrrolidine-3,4'-pyrrolo[1,2-b]pyrazole]-1-carboxamide
2'-{6-amino-5-[(1R)-1-(pyridin-3-yl)éthoxy]pyridin-3-yl}-N-[2-(1-méthyl-1H-1,2,3-triazol-4-yl)propan-2-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[cyclopropyl(pyridin-2-yl)méthyl]-5',6'-dihydrospiro[pyrrolidine-3,4'-pyrrolo[1,2-b]pyrazole]-1-carboxamide (mélange de diastéréomères)
2'-{6-amino-5-[(1S)-1-(pyridin-3-yl)éthoxy]pyridin-3-yl}-N-cyclobutyl-5',6'-dihydrospiro[pyrrolidine-3,4'-pyrrolo[1,2-b]pyrazole]-1-carboxamide
2'-{6-amino-5-[(1S)-1-(pyridin-3-yl)éthoxy]pyridin-3-yl}-N-[2-(pyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(2-cyanophényl)propan-2-yl]-5',6'-dihydrospiro[pyrrolidine-3,4'-pyrrolo[1,2-b]pyrazole]-1-carboxamide
[2'-{6-amino-5-[(1R)-1-(pyridin-3-yl)éthoxy]pyridin-3-yl}-5',6'-dihydrospiro[pyrrolidine-3,4'-pyrrolo[1,2-b]pyrazol]-1-yl](1H-imidazol-1-yl)méthanone
(*rac*)-2'-[6-amino-5-(trifluorométhyl)pyridin-3-yl]-N-(1-méthylcyclobutyl)-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-(3-méthyloxétan-3-yl)-5',6'-dihydrospiro[pyrrolidine-3,4'-pyrrolo[1,2-b]pyrazole]-1-carboxamide
(*rac*)-2-[6-amino-5-(trifluorométhoxy)pyridin-3-yl]-N-[2-(3-fluoropyridin-2-yl)propan-2-yl]-6,7-dihydrospiro[pyrazolo[5,1-c][1,4]oxazine-4,3'-pyrrolidine]-1'-carboxamide
2'-{6-amino-5-[(1S)-1-phényléthoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide
2'-{6-amino-5-[(1S)-1-phényléthoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-phényléthoxy]pyridin-3-yl}-N-[1-(pyridin-4-yl)cyclobutyl]-5',6'-dihydrospiro[pyrrolidine-3,4'-pyrrolo[1,2-b]pyrazole]-1-carboxamide
2'-{6-amino-5-[(1R)-1-phényléthoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2-[6-amino-5-(trifluorométhoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
2'-{6-amino-5-[(1R)-1-(pyridin-2-yl)éthoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-(pyridin-3-yl)éthoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-{6-amino-5-[(1R)-1-(pyridin-4-yl)éthoxy]pyridin-3-yl}-N-[2-(3-chloropyridin-4-yl)propan-2-yl]-5',6'-dihydrospiro[pyrrolidine-3,4'-pyrrolo[1,2-b]pyrazole]-1-carboxamide
2'-(6-amino-5-cyanopyridin-3-yl)-N-[(1R)-1-(pyridin-4-yl)éthyl]-5',6'-dihydrospiro[pyrrolidine-3,4'-pyrrolo[1,2-b]pyrazole]-1-carboxamide (isomère 1)
2'-(6-amino-5-cyanopyridin-3-yl)-N-[(1R)-1-(pyridin-4-yl)éthyl]-5',6'-dihydrospiro[pyrrolidine-3,4'-pyrrolo[1,2-b]pyrazole]-1-carboxamide (isomère 2)
2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (énantiomère 1)
(3R)-2'-[6-amino-5-(trifluorométhyl)pyridin-3-yl]-N-[(1S)-1-(pyridin-4-yl)éthyl]-5',6'-dihydrospiro[pyrrolidine-3,4'-pyrrolo[1,2-b]pyrazole]-1-carboxamide
(3S)-2'-[6-amino-5-(trifluorométhyl)pyridin-3-yl]-N-[(1S)-1-(pyridin-4-yl)éthyl]-5',6'-dihydrospiro[pyrrolidine-3,4'-pyrrolo[1,2-b]pyrazole]-1-carboxamide
2'-(6-amino-5-cyanopyridin-3-yl)-N-[(1S)-1-(pyridin-4-yl)éthyl]-5',6'-dihydrospiro[pyrrolidine-3,4'-pyrrolo[1,2-b]pyrazole]-1-carboxamide (isomère 1)
2'-(6-amino-5-cyanopyridin-3-yl)-N-[(1S)-1-(pyridin-4-yl)éthyl]-5',6'-dihydrospiro[pyrrolidine-3,4'-pyrrolo[1,2-b]pyrazole]-1-carboxamide (isomère 2)
2'-[6-amino-5-(trifluorométhyl)pyridin-3-yl]-N-[(1S)-1-(pyridin-4-yl)éthyl]-5',6'-dihydrospiro[azétidine-3,4'-pyrrolo[1,2-b]pyrazole]-1-carboxamide
2'-[6-amino-5-(trifluorométhyl)pyridin-3-yl]-N-[(1R)-1-(pyridin-4-yl)éthyl]-5',6'-dihydrospiro[azétidine-3,4'-pyrrolo[1,2-b]pyrazole]-1-carboxamide
(*rac*)-2'-[6-amino-5-(trifluorométhyl)pyridin-3-yl]-N-[1-(2-chlorophényl)cyclobutyl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[2-(2-fluorophényl)propan-2-yl]-6,7-dihydrospiro[pyrazolo[5,1-c][1,4]oxazine-4,3'-pyrrolidine]-l'-carboxamide
(*rac*)-2-[6-amino-5-(trifluorométhyl)pyridin-3-yl]-N-(1-phénylcyclobutyl)-6,7-dihydrospiro[pyrazolo[5,1-c][1,4]oxazine-4,3'-pyrrolidine]-1'-carboxamide
(*rac*)-2'-[6-amino-5-(trifluorométhyl)pyridin-3-yl]-N-[2-(4-fluorophényl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(2-chlorophényl)propan-2-yl]-5',6'-dihydrospiro[pyrrolidine-3,4'-pyrrolo[1,2-b]pyrazole]-1-carboxamide
2-[6-amino-5-(trifluorométhyl)pyridin-3-yl]-N-[(1R)-1-(2-chloro-4-cyanophényl)éthyl]-6,7-dihydrospiro[pyrazolo[5,1-c][1,4]oxazine-4,3'-pyrrolidine]-1'-carboxamide (mélange de stéréoisomères)
2'-[6-amino-5-(trifluorométhyl)pyridin-3-yl]-N-[(1R)-1-(2-chloro-4-cyanophényl)éthyl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1S)-1-(3-chloropyridin-4-yl)éthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(3-fluorophényl)propan-2-yl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[1-(pyridin-4-yl)cyclobutyl]-6,7-dihydrospiro[pyrazolo[5,1-c][1,4]oxazine-4,3'-pyrrolidine]-1'-carboxamide
(*rac*)-2'-[6-amino-5-(trifluorométhyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-(1-phénylcyclobutyl)-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-[1-(2-fluorophényl)cyclobutyl]-6,7-dihydrospiro[pyrazolo[5,1-c][1,4]oxazine-4,3'-pyrrolidine]-l'-carboxamide
(*rac*)-2'-[6-amino-5-(trifluorométhyl)pyridin-3-yl]-N-[1-(2-fluorophényl)cyclobutyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[1-(3-fluorophényl)cyclobutyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-[1-(3-fluorophényl)cyclobutyl]-6,7-dihydrospiro[pyrazolo[5,1-c][1,4]oxazine-4,3'-pyrrolidine]-l'-carboxamide
(3'R)-2-[6-amino-5-(trifluorométhoxy)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(3'R)-2-[6-amino-5-(trifluorométhyl)pyridin-3-yl]-N-[2-(pyridin-3-yl)propan-2-yl]-6,7-dihydrospiro[pyrazolo[5,1-c][1,4]oxazine-4,3'-pyrrolidine]-1'-carboxamide
(3R)-2'-[6-amino-5-(trifluorométhyl)pyridin-3-yl]-N-[2-(pyridin-4-yl)propan-2-yl]-5',6'-dihydrospiro[pyrrolidine-3,4'-pyrrolo[1,2-b]pyrazole]-1-carboxamide
(3'R)-2-[6-amino-5-(trifluorométhyl)pyridin-3-yl]-N-[2-(pyridin-4-yl)propan-2-yl]-6,7-dihydrospiro[pyrazolo[5,1-c][1,4]oxazine-4,3'-pyrrolidine]-1'-carboxamide
(3R)-2'-[6-amino-5-(trifluorométhyl)pyridin-3-yl]-N-[2-(pyridin-3-yl)propan-2-yl]-5',6'-dihydrospiro[pyrrolidine-3,4'-pyrrolo[1,2-b]pyrazole]-1-carboxamide
(3R)-2'-[6-amino-5-(difluorométhoxy)pyridin-3-yl]-N-[(1R)-1-phényléthyl]-5',6'-dihydrospiro[pyrrolidine-3,4'-pyrrolo[1,2-b]pyrazole]-1-carboxamide
(3'R)-2-[6-amino-5-(difluorométhoxy)pyridin-3-yl]-N-[(1R)-1-phényléthyl]-6,7-dihydrospiro[pyrazolo[5,1-c][1,4]oxazine-4,3'-pyrrolidine]-1'-carboxamide
(3R)-2'-[6-amino-5-(trifluorométhyl)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-1-phényléthyl]-5',6'-dihydrospiro[pyrrolidine-3,4'-pyrrolo[1,2-b]pyrazole]-1-carboxamide (stéréoisomère 1)
2'-[6-amino-5-(trifluorométhyl)pyridin-3-yl]-N-[(1R)-1-phényléthyl]-5',6'-dihydrospiro[pyrrolidine-3,4'-pyrrolo[1,2-b]pyrazole]-1-carboxamide (stéréoisomère 2)
(*rac*)-2'-[6-amino-5-(trifluorométhyl)pyridin-3-yl]-N-[2-(3,5-difluorophényl)propan-2-yl]-5',6'-dihydrospiro[pyrrolidine-3,4'-pyrrolo[1,2-b]pyrazole]-1-carboxamide
(*rac*)-1-{2'-[6-amino-5-(trifluorométhyl)pyridin-3-yl]-5',6'-dihydrospiro[pyrrolidine-3,4'-pyrrolo[1,2-b]pyrazol]-1-yl}-2-(5-fluoropyridin-2-yl)-2-méthylpropan-1-one
(*rac*)-2'-[6-amino-5-(trifluorométhyl)pyridin-3-yl]-N-[2-(6-méthylpyridin-3-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-(1-phénylcyclohexyl)-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(5-fluoropyridin-2-yl)propan-2-yl]-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[(1R)-2,2,2-trifluoro-1-méthoxy-1-phényléthyl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(pyrimidin-4-yl)propan-2-yl]-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-[2-(pyrimidin-5-yl)propan-2-yl]-5',6'-dihydrospiro[pyrrolidine-3,4'-pyrrolo[1,2-b]pyrazole]-1-carboxamide
(*rac*)-{2'-[6-amino-5-(trifluorométhyl)pyridin-3-yl]-5',6'-dihydrospiro[pyrrolidine-3,4'-pyrrolo[1,2-b]pyrazol]-1-yl}(3-phényloxétan-3-yl)méthanone
(*rac*)-1-{2'-[6-amino-5-(trifluorométhyl)pyridin-3-yl]-5',6'-dihydrospiro[pyrrolidine-3,4'-pyrrolo[1,2-b]pyrazol]-1-yl}-2-(3,5-difluorophényl)-2-méthylpropan-1-one
(*rac*)-1-{2'-[6-amino-5-(trifluorométhyl)pyridin-3-yl]-5',6'-dihydrospiro[pyrrolidine-3,4'-pyrrolo[1,2-b]pyrazol]-1-yl}-2-méthyl-2-(6-méthylpyridin-3-yl)propan-1-one
(*rac*)-2'-[6-amino-5-(trifluorométhyl)pyridin-3-yl]-N-(3-phényloxétan-3-yl)-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-(propan-2-yl)-5',6'-dihydrospiro[pyrrolidine-3,4'-pyrrolo[1,2-b]pyrazole]-1-carboxamide
(*rac*)-2'-[6-amino-5-(trifluorométhyl)pyridin-3-yl]-N-cyclobutyl-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-éthyl-5',6'-dihydrospiro[pyrrolidine-3,4'-pyrrolo[1,2-b]pyrazole]-1-carboxamide
(3S)-2'-[6-amino-5-(trifluorométhyl)pyridin-3-yl]-N-éthyl-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-(propan-2-yl)-5',6'-dihydrospiro[pyrrolidine-3,4'-pyrrolo[1,2-b]pyrazole]-1-carboxamide
(3S)-2'-[6-amino-5-(trifluorométhyl)pyridin-3-yl]-N-(propan-2-yl)-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-cyclobutyl-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-éthyl-6,7-dihydrospiro[pyrazolo[5,1-c][1,4]oxazine-4,3'-pyrrolidine]-1'-carboxamide
(*rac*)-2-[6-amino-5-(trifluorométhyl)pyridin-3-yl]-N-(propan-2-yl)-6,7-dihydrospiro[pyrazolo[5,1-c][1,4]oxazine-4,3'-pyrrolidine]-1'-carboxamide
2'-[6-amino-5-(trifluorométhyl)pyridin-3-yl]-N-cyclobutyl-5',6'-dihydrospiro[azétidine-3,4'-pyrrolo[1,2-b]pyrazole]-1-carboxamide
2'-[6-amino-5-(trifluorométhyl)pyridin-3-yl]-N-éthyl-5',6'-dihydrospiro[azétidine-3,4'-pyrrolo[1,2-b]pyrazole]-1-carboxamide
2'-[6-amino-5-(trifluorométhyl)pyridin-3-yl]-N-(propan-2-yl)-5',6'-dihydrospiro[azétidine-3,4'-pyrrolo[1,2-b]pyrazole]-1-carboxamide
(rac)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide
(*rac*)-2'-{6-amino-5-[(2-chlorophényl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(2-chlorophényl)méthoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-[6-amino-5-[(2-chlorophényl)méthoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-fluorophényl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(2-fluorophényl)méthoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(2-fluorophényl)méthoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-fluorophényl)méthoxy]pyridin-3-yl}-N-benzyl-5',6'-dihydrospiro[pyrrolidine-3,4'-pyrrolo[1,2-b]pyrazole]-1-carboxamide
(*rac*)-2'-{6-amino-5-[(3-fluorophényl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(3-fluorophényl)méthoxy]pyridin-3-yl}-N-(propan-2-yl)-5',6'-dihydrospiro[pyrrolidine-3,4'-pyrrolo[1,2-b]pyrazole]-1-carboxamide
(*rac*)-2'-{6-amino-5-[(3-fluorophényl)méthoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(3-fluorophényl)méthoxy]pyridin-3-yl}-N-benzyl-5',6'-dihydrospiro[pyrrolidine-3,4'-pyrrolo[1,2-b]pyrazole]-1-carboxamide
(*rac*)-2'-{6-amino-5-[(pyridin-2-yl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(pyridin-2-yl)méthoxy]pyridin-3-yl}-N-(propan-2-yl)-5',6'-dihydrospiro[pyrrolidine-3,4'-pyrrolo[1,2-b]pyrazole]-1-carboxamide (*rac*)-*2*'*-*{6-amino-5-[(pyridin-2-yl)méthoxy]pyridin-3-yl}-N-cyclobutyl-5',6'-dihydrospiro[pyrrolidine-3,4'-pyrrolo[1,2-b]pyrazole]-1-carboxamide
(*rac*)-2'-{6-amino-5-[(2-chloro-5-fluorophényl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(2-chloro-5-fluorophényl)méthoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1R)-1-(pyridin-2-yl)éthoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1R)-1-(pyridin-2-yl)éthoxy]pyridin-3-yl}-N-éthyl-5',6'-dihydrospiro[pyrrolidine-3,4'-pyrrolo[1,2-b]pyrazole]-1-carboxamide
2'-{6-amino-5-[(1S)-1-(pyridin-2-yl)éthoxy]pyridin-3-yl}-N-(propan-2-yl)-5',6'-dihydrospiro[pyrrolidine-3,4'-pyrrolo[1,2-b]pyrazole]-1-carboxamide
2'-{6-amino-5-[(1S)-1-(pyridin-2-yl)éthoxy]pyridin-3-yl}-N-éthyl-5',6'-dihydrospiro[pyrrolidine-3,4'-pyrrolo[1,2-b]pyrazole]-1-carboxamide
2'-(6-amino-5-{[1-(5-chloro-3-fluoropyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (mélange de diastéréomères)
(rac)-2'-[6-amino-5-(benzyloxy)pyridin-3-yl] N-cyclobutyl-5',6'-dihydrospiro[pyrrolidine-3,4'-pyrrolo[1,2-b]pyrazole]-1-carboxamide
2'-{6-amino-5-[(pyridazin-3-yl)méthoxy]pyridin-3-yl}-N-éthyl-5',6'-dihydrospiro[pyrrolidine-3,4'-pyrrolo[1,2-b]pyrazole]-1-carboxamide (stéréoisomère 2)
2'-{6-amino-5-[(pyridazin-3-yl)méthoxy]pyridin-3-yl}-N-éthyl-5',6'-dihydrospiro[pyrrolidine-3,4'-pyrrolo[1,2-b]pyrazole]-1-carboxamide (stéréoisomère 1)
(3S)-2'-{6-amino-5-[1-(pyrimidin-5-yl)éthoxy]pyridin-3-yl}-N-éthyl-5',6'-dihydro-1H-spiro[pyrrolidine-3,4'-pyrrolo[1,2-b]pyrazole]-1-carboxamide (isomère 1)
(3S)-2'-{6-amino-5-[1-(pyrimidin-5-yl)éthoxy]pyridin-3-yl}-N-éthyl-5',6'-dihydro-1H-spiro[pyrrolidine-3,4'-pyrrolo[1,2-b]pyrazole]-1-carboxamide (isomère 2)
(*rac*)-2'-(2-amino-1'-méthyl-l',2',3',6'-tétrahydro[3,4'-bipyridin]-5-yl)-N-éthyl-5',6'-dihydrospiro[pyrrolidine-3,4'-pyrrolo[1,2-b]pyrazole]-1-carboxamide
2'-{6-amino-5-[(1S)-1-(pyridin-2-yl)éthoxy]pyridin-3-yl}-N-éthyl-5',6'-dihydrospiro[azétidine-3,4'-pyrrolo[1,2-b]pyrazole]-1-carboxamide
2'-{6-amino-5-[(1S)-1-(pyridin-2-yl)éthoxy]pyridin-3-yl}-N-(propan-2-yl)-5',6'-dihydrospiro[azétidine-3,4'-pyrrolo[1,2-b]pyrazole]-1-carboxamide
2'-{6-amino-5-[(1S)-1-phényléthoxy]pyridin-3-yl}-N-éthyl-5',6'-dihydrospiro[azétidine-3,4'-pyrrolo[1,2-b]pyrazole]-1-carboxamide
2'-{6-amino-5-[(1R)-1-phényléthoxy]pyridin-3-yl}-N-éthyl-5',6'-dihydrospiro[azétidine-3,4'-pyrrolo[1,2-b]pyrazole]-1-carboxamide
(*rac*)-2-[6-amino-5-(trifluorométhyl)pyridin-3-yl]-N-[2-(3-chloropyridin-4-yl)propan-2-yl]-6,7-dihydrospiro[pyrazolo[5,1-c][1,4]oxazine-4,3'-pyrrolidine]-1'-carboxamide
(*rac*)-2'-[6-amino-5-(trifluorométhyl)pyridin-3-yl]-N-éthyl-5',6'-dihydrospiro[pyrrolidine-3,4'-pyrrolo[1,2-b]pyrazole]-1-carboxamide
(*rac*)-2-[6-amino-5-(trifluorométhyl)pyridin-3-yl]-N-éthyl-6,7-dihydro-5H-spiro[pyrazolo[1,5-a]pyridine-4,3'-pyrrolidine]-1'-carboxamide
(*rac*)-2'-(6-amino-5-cyanopyridin-3-yl)-N-éthyl-5',6'-dihydrospiro[pyrrolidine-3,4'-pyrrolo[1,2-b]pyrazole]-1-carboxamide
(*rac*)-2'-[6-amino-5-(trifluorométhoxy)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(difluorométhoxy)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(trifluoroacétyl)pyridin-3-yl]-N-éthyl-5',6'-dihydrospiro[pyrrolidine-3,4'-pyrrolo[1,2-b]pyrazole]-1-carboxamide
2'-(6-amino-5-{[1-phényléthyl]sulfanyl}pyridin-3-yl)-N-éthyl-5',6'-dihydrospiro[pyrrolidine-3,4'-pyrrolo[1,2-b]pyrazole]-1-carboxamide (mélange de diastéréomères)
(*rac*)-2'-{6-amino-5-[(cyclohexylméthyl)sulfanyl]pyridin-3-yl}-N-éthyl-5',6'-dihydrospiro[pyrrolidine-3,4'-pyrrolo[1,2-b]pyrazole]-1-carboxamide
(*rac*)-2'-[6-amino-5-(benzylsulfanyl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(2-hydroxypropan-2-yl)pyridin-3-yl]-N-éthyl-5',6'-dihydrospiro[pyrrolidine-3,4'-pyrrolo[1,2-b]pyrazole]-1-carboxamide
2'-(6-amino-5-{[1-cyclohexyléthyl]sulfanyl}pyridin-3-yl)-N-éthyl-5',6'-dihydrospiro[pyrrolidine-3,4'-pyrrolo[1,2-b]pyrazole]-1-carboxamide (mélange de diastéréomères)
2'-{6-amino-5-[(1R)-1-(pyridin-3-yl)éthoxy]pyridin-3-yl}-N-éthyl-5',6'-dihydrospiro[pyrrolidine-3,4'-pyrrolo[1,2-b]pyrazole]-1-carboxamide
2'-{6-amino-5-[(1S)-1-(pyridin-3-yl)éthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(cyclopentylméthyl)sulfanyl]pyridin-3-yl}-N-éthyl-5',6'-dihydrospiro[pyrrolidine-3,4'-pyrrolo[1,2-b]pyrazole]-1-carboxamide
(*rac*)-2'-(6-amino-5-nitropyridin-3-yl)-N-éthyl-5',6'-dihydrospiro[pyrrolidine-3,4'-pyrrolo[1,2-b]pyrazole]-1-carboxamide
(*rac*)-2'-[6-amino-5-(benzyloxy)pyridin-3-yl]-N-éthyl-5',6'-dihydrospiro[pyrrolidine-3,4'-pyrrolo[1,2-b]pyrazole]-1-carboxamide
(*rac*)-2'-(6-amino-5-méthylpyridin-3-yl)-N-éthyl-5',6'-dihydrospiro[pyrrolidine-3,4'-pyrrolo[1,2-b]pyrazole]-1-carboxamide
(*rac*)-2-(6-amino-5-nitropyridin-3-yl)-N-éthyl-6,7-dihydro-5H-spiro[pyrazolo[1,5-a]pyridine-4,3'-pyrrolidine]-1'-carboxamide
2-amino-5-[(*rac*)-1-(éthylcarbamoyl)-5',6'-dihydrospiro[pyrrolidine-3,4'-pyrrolo[1,2-b]pyrazol]-2'-yl]pyridine-3-carboxylate de méthyle
(*rac*)-2'-(6-amino-5-phénylpyridin-3-yl)-N-éthyl-5',6'-dihydrospiro[pyrrolidine-3,4'-pyrrolo[1,2-b]pyrazole]-1-carboxamide
(*rac*)-2'-[6-amino-5-(méthylsulfanyl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(4-méthylphényl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(2-méthylphényl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(3-méthylphényl)pyridin-3-yl]-N-éthyl-5',6'-dihydrospiro[pyrrolidine-3,4'-pyrrolo[1,2-b]pyrazole]-1-carboxamide
(*rac*)-2'-(6-amino-5-méthoxypyridin-3-yl)-N-éthyl-5',6'-dihydrospiro[pyrrolidine-3,4'-pyrrolo[1,2-b]pyrazole]-1-carboxamide
(*rac*)-2'-[6-amino-5-(3-fluorophényl)pyridin-3-yl]-N-éthyl-5',6'-dihydrospiro[pyrrolidine-3,4'-pyrrolo[1,2-b]pyrazole]-1-carboxamide
(*rac*)-2'-(2-amino[3,3'-bipyridin]-5-yl)-N-éthyl-5',6'-dihydrospiro[pyrrolidine-3,4'-pyrrolo[1,2-b]pyrazole]-1-carboxamide
(*rac*)-2'-[6-amino-5-(2-fluorophényl)pyridin-3-yl]-N-éthyl-5',6'-dihydrospiro[pyrrolidine-3,4'-pyrrolo[1,2-b]pyrazole]-1-carboxamide
2'-{6-amino-5-[(1R)-1-(2,6-dichloro-3-fluorophényl)éthoxy]pyridin-3-yl}-N-éthyl-5',6'-dihydrospiro[pyrrolidine-3,4'-pyrrolo[1,2-b]pyrazole]-1-carboxamide (mélange de diastéréomères)
(*rac*)-2-[6-amino-5-(2-fluorophényl)pyridin-3-yl]-N-éthyl-6,7-dihydro-5H-spiro[pyrazolo[1,5-a]pyridine-4,3'-pyrrolidine]-1'-carboxamide
(*rac*)-2'-[6-amino-5-(2-méthoxyphényl)pyridin-3-yl]-N-éthyl-5',6'-dihydrospiro[pyrrolidine-3,4'-pyrrolo[1,2-b]pyrazole]-1-carboxamide
(*rac*)-2'-(2-amino[3,4'-bipyridin]-5-yl)-N-éthyl-5',6'-dihydrospiro[pyrrolidine-3,4'-pyrrolo[1,2-b]pyrazole]-1-carboxamide
(*rac*)-2'-{6*-*amino*-*5-[3-(méthanesulfonyl)phényl]pyridin-3-yl}-N-éthyl-5',6'-dihydrospiro[pyrrolidine-3,4'-pyrrolo[1,2-b]pyrazole]-1-carboxamide
(*rac*)-2'-[6-amino-5-(phénylsulfanyl)pyridin-3-yl]-N-éthyl-5',6'-dihydrospiro[pyrrolidine-3,4'-pyrrolo[1,2-b]pyrazole]-1-carboxamide
(*rac*)-2'-{6-amino-5-[4-(méthanesulfonyl)phényl]pyridin-3-yl}-N-éthyl-5',6'-dihydrospiro[pyrrolidine-3,4'-pyrrolo[1,2-b]pyrazole]-1-carboxamide
(*rac*)-2'-[5-(4-acétylphényl)-6-aminopyridin-3-yl]-N-éthyl-5',6'-dihydrospiro[pyrrolidine-3,4'-pyrrolo[1,2-b]pyrazole]-1-carboxamide
(*rac*)-2'-{6-amino-5-[4-(méthanesulfinyl)phényl]pyridin-3-yl}-N-éthyl-5',6'-dihydrospiro[pyrrolidine-3,4'-pyrrolo[1,2-b]pyrazole]-1-carboxamide
(*rac*)-2'-[6-amino-5-(3-méthoxyphényl)pyridin-3-yl]-N-éthyl-5',6'-dihydrospiro[pyrrolidine-3,4'-pyrrolo[1,2-b]pyrazole]-1-carboxamide
2'-{6-amino-5-[(1R)-1-(2,6-dichloro-3-fluorophényl)éthoxy]pyridin-3-yl}-N-éthyl-5',6'-dihydrospiro[azétidine-3,4'-pyrrolo[1,2-b]pyrazole]-1-carboxamide
(*rac*)-2'-{6-amino-5-[4-(cyclopropanesulfonyl)phényl]pyridin-3-yl}-N-éthyl-5',6'-dihydrospiro[pyrrolidine-3,4'-pyrrolo[1,2-b]pyrazole]-1-carboxamide
(*rac*)-2'-[6-amino-5-(4-sulfamoylphényl)pyridin-3-yl]-N-éthyl-5',6'-dihydrospiro[pyrrolidine-3,4'-pyrrolo[1,2-b]pyrazole]-1-carboxamide
(*rac*)-2'-{6-amino-5-[4-(méthylsulfamoyl)phényl]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[4-(diméthylsulfamoyl)phényl]pyridin-3-yl}-N-éthyl-5',6'-dihydrospiro[pyrrolidine-3,4'-pyrrolo[1,2-b]pyrazole]-1-carboxamide
(*rac*)-2'-[6-amino-5-(cyclohexylméthoxy)pyridin-3-yl]-N-éthyl-5',6'-dihydrospiro[pyrrolidine-3,4'-pyrrolo[1,2-b]pyrazole]-1-carboxamide
(*rac*)-2'-{6-amino-5-[(pyridin-3-yl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(4-fluorophényl)méthoxy]pyridin-3-yl}-N-éthyl-5',6'-dihydrospiro[pyrrolidine-3,4'-pyrrolo[1,2-b]pyrazole]-1-carboxamide
2'-(6-amino-5-{[1-phényléthyl]oxy}pyridin-3-yl)-N-éthyl-5',6'-dihydrospiro[pyrrolidine-3,4'-pyrrolo[1,2-b]pyrazole]-1-carboxamide (mélange de diastéréomères)
2-{6-amino-5-[(1R)-1-(2,6-dichloro-3-fluorophényl)éthoxy]pyridin-3-yl}-N-éthyl-6,7-dihydrospiro[pyrazolo[5,1-c][1,4]oxazine-4,3'-pyrrolidine]-1'-carboxamide
(*rac*)-2'-[6-amino-5-(propan-2-yl)pyridin-3-yl]-N-éthyl-5',6'-dihydrospiro[pyrrolidine-3,4'-pyrrolo[1,2-b]pyrazole]-1-carboxamide
(*rac*)-2-[6-amino-5-(méthylcarbamoyl)pyridin-3-yl]-N-éthyl-6,7-dihydrospiro[pyrazolo[5,1-c][1,4]oxazine-4,3'-pyrrolidine]-1'-carboxamide
(*rac*)-2'-[6-amino-5-(méthylcarbamoyl)pyridin-3-yl]-N-éthyl-5',6'-dihydrospiro[pyrrolidine-3,4'-pyrrolo[1,2-b]pyrazole]-1-carboxamide
(*rac*)-2'-(6-amino-5-cyclopropylpyridin-3-yl)-N-éthyl-5',6'-dihydrospiro[pyrrolidine-3,4'-pyrrolo[1,2-b]pyrazole]-1-carboxamide
(*rac*)-2'-[6-amino-5-(méthylcarbamoyl)pyridin-3-yl]-N-(1-phénylcyclobutyl)-5',6'-dihydrospiro[pyrrolidine-3,4'-pyrrolo[1,2-b]pyrazole]-1-carboxamide
(*rac*)-2'-[6-amino-5-(diméthylcarbamoyl)pyridin-3-yl]-N-(1-phénylcyclobutyl)-5',6'-dihydrospiro[pyrrolidine-3,4'-pyrrolo[1,2-b]pyrazole]-1-carboxamide
2'-{6-amino-5-[(1R)-1-(pyridin-4-yl)éthoxy]pyridin-3-yl}-N-éthyl-5',6'-dihydrospiro[pyrrolidine-3,4'-pyrrolo[1,2-b]pyrazole]-1-carboxamide
2'-{6-amino-5-[(1S)-1-(pyridin-4-yl)éthoxy]pyridin-3-yl}-N-éthyl-5',6'-dihydrospiro[pyrrolidine-3,4'-pyrrolo[1,2-b]pyrazole]-1-carboxamide
(*rac*)-2'-[6-amino-5-(1-méthyl-1H-pyrazol-3-yl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(1,2-thiazol-5-yl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(1,5-diméthyl-1H-pyrazol-4-yl)pyridin-3-yl]-N-éthyl-5',6'-dihydrospiro[pyrrolidine-3,4'-pyrrolo[1,2-b]pyrazole]-1-carboxamide
(*rac*)-2'-{6-amino-5-[(trifluorométhyl)sulfanyl]pyridin-3-yl}-N-éthyl-5',6'-dihydrospiro[pyrrolidine-3,4'-pyrrolo[1,2-b]pyrazole]-1-carboxamide
5-[1-méthyl-5',6'-dihydrospiro[pyrrolidine-3,4'-pyrrolo[1,2-b]pyrazol]-2'-yl]-3-(trifluorométhyl)pyridin-2-amine (stéréoisomère 1)
5-[1-méthyl-5',6'-dihydrospiro[pyrrolidine-3,4'-pyrrolo[1,2-b]pyrazol]-2'-yl]-3-(trifluorométhyl)pyridin-2-amine (stéréoisomère 2)
(3R)-2'-{6-amino-5-[(1R)-1-(2-chlorophényl)éthoxy]pyridin-3-yl}-N-éthyl-5',6'-dihydrospiro[pyrrolidine-3,4'-pyrrolo[1,2-b]pyrazole]-1-carboxamide
2'-{6-amino-5-[(1R)-1-(2-chlorophényl)éthoxy]pyridin-3-yl}-N-éthyl-5',6'-dihydrospiro[azétidine-3,4'-pyrrolo[1,2-b]pyrazole]-1-carboxamide
2'-{6-amino-5-[(1S)-1-(2-chlorophényl)éthoxy]pyridin-3-yl}-N-éthyl-5',6'-dihydrospiro[azétidine-3,4'-pyrrolo[1,2-b]pyrazole]-1-carboxamide
(3'R)-2-{6-amino-5-[(1R)-1-(2,6-dichloro-3-fluorophényl)éthoxy]pyridin-3-yl}-N-éthyl-6,7-dihydrospiro[pyrazolo[5,1-c][1,4]oxazine-4,3'-pyrrolidine]-1'-carboxamide
(3R)-2'-{6-amino-5-[(1R)-1-(pyridin-2-yl)éthoxy]pyridin-3-yl}-N-éthyl-5',6'-dihydrospiro[pyrrolidine-3,4'-pyrrolo[1,2-b]pyrazole]-1-carboxamide
2'-{6-amino-5-[(1R)-1-(pyridin-3-yl)éthoxy]pyridin-3-yl}-N-éthyl-5',6'-dihydrospiro[azétidine-3,4'-pyrrolo[1,2-b]pyrazole]-1-carboxamide
(3R)-2'-{6-amino-5-[1-(pyrimidin-5-yl)éthoxy]pyridin-3-yl}-N-éthyl-5',6'-dihydro-1H-spiro[pyrrolidine-3,4'-pyrrolo[1,2-b]pyrazole]-1-carboxamide (isomère 1)
((3R)-2'-{6-amino-5-[1-(pyrimidin-5-yl)éthoxy]pyridin-3-yl}-N-éthyl-5',6'-dihydro-1H-spiro[pyrrolidine-3,4'-pyrrolo[1,2-b]pyrazole]-1-carboxamide (isomère 2)
(*rac*)-2'-[6-amino-5-(difluorométhyl)pyridin-3-yl]-N-éthyl-5',6'-dihydrospiro[pyrrolidine-3,4'-pyrrolo[1,2-b]pyrazole]-1-carboxamide
2'-{6-amino-5-[(1S)-1-(2-chlorophényl)éthoxy]pyridin-3-yl}-N-éthyl-5',6'-dihydrospiro[pyrrolidine-3,4'-pyrrolo[1,2-b]pyrazole]-1-carboxamide
2'-{6-amino-5-[(1R)-1-(2-chlorophényl)éthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(3-méthylphényl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(1-méthyl-1H-pyrazol-5-yl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(1-méthyl-1H-pyrazol-4-yl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(1,5-diméthyl-1H-pyrazol-3-yl)méthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(4-méthylphényl)méthoxy]pyridin-3-yl}-N-éthyl-5',6'-dihydrospiro[pyrrolidine-3,4'-pyrrolo[1,2-b]pyrazole]-1-carboxamide
2'-{6-amino-5-[(1R)-1-(3-méthylphényl)éthoxy]pyridin-3-yl}-N-éthyl-5',6'-dihydrospiro[pyrrolidine-3,4'-pyrrolo[1,2-b]pyrazole]-1-carboxamide
(*rac*)-2'-{6-amino-5-[(propan-2-yl)oxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1S)-1-(1-méthyl-1H-pyrazol-3-yl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1R)-1-(1-méthyl-1H-pyrazol-3-yl)éthoxy]pyridin-3-yl}-N-éthyl-5',6'-dihydrospiro[pyrrolidine-3,4'-pyrrolo[1,2-b]pyrazole]-1-carboxamide
(3R)-2'-(6-amino-5-{[1-(3,5-difluoropyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (stéréoisomère 1)
(3R)-2'-(6-amino-5-{[1-(3,5-difluoropyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (stéréoisomère 2)
(3R)-2'-{6-amino-5-[(1R)-1-(2-fluorophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1S)-1-(2-fluorophényl)éthoxy]pyridin-3-yl}-N-éthyl-5',6'-dihydrospiro[pyrrolidine-3,4'-pyrrolo[1,2-b]pyrazole]-1-carboxamide
(*rac*)-2'-(5-acétyl-6-aminopyridin-3-yl)-N-(1-phénylcyclobutyl)-5',6'-dihydrospiro[pyrrolidine-3,4'-pyrrolo[1,2-b]pyrazole]-1-carboxamide
(3R)-2'-[6-amino-5-(trifluorométhoxy)pyridin-3-yl]-N-[2-(pyridin-2-yl)propan-2-yl]-5',6'-dihydrospiro[pyrrolidine-3,4'-pyrrolo[1,2-b]pyrazole]-1-carboxamide
(3'R)-2-[6-amino-5-(trifluorométhyl)pyridin-3-yl]-N-[2-(pyridin-2-yl)propan-2-yl]-6,7-dihydrospiro[pyrazolo[5,1-c][1,4]oxazine-4,3'-pyrrolidine]-1'-carboxamide
(3R)-2'-(6-amino-5-cyanopyridin-3-yl)-N-[2-(pyridin-2-yl)propan-2-yl]-5',6'-dihydrospiro[pyrrolidine-3,4'-pyrrolo[1,2-b]pyrazole]-1-carboxamide
(3'R)-2-(6-amino-5-cyanopyridin-3-yl)-N-[2-(pyridin-2-yl)propan-2-yl]-6,7-dihydrospiro[pyrazolo[5,1-c][1,4]oxazine-4,3'-pyrrolidine]-1'-carboxamide
(*rac*)-2'-[6-amino-5-(phénylcarbamoyl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(cyclohexylcarbamoyl)pyridin-3-yl]-N-éthyl-5',6'-dihydrospiro[pyrrolidine-3,4'-pyrrolo[1,2-b]pyrazole]-1-carboxamide
(*rac*)-2'-[6-amino-5-(diéthylcarbamoyl)pyridin-3-yl]-N-éthyl-5',6'-dihydrospiro[pyrrolidine-3,4'-pyrrolo[1,2-b]pyrazole]-1-carboxamide
(*rac*)-2'-(5-acétyl-6-aminopyridin-3-yl)-N-éthyl-5',6'-dihydrospiro[pyrrolidine-3,4'-pyrrolo[1,2-b]pyrazole]-1-carboxamide
5-{1-[1-(1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine
5-{1-[1-(1H-pyrazol-3-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(1H-pyrazol-3-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(1H-imidazol-5-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(1-méthyl-1H-pyrazol-3-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(1H-pyrazol-4-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(4-méthyl-1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(3-méthyl-1H-pyrazol-5-yl)éthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(4-méthyl-1H-pyrazol-5-yl)éthyl]-5',6'-dihydrospiro[pyrrolidine-3,4'-pyrrolo[1,2-b]pyrazol]-2'-yl}-3-(trifluorométhyl)pyridin-2-amine
5-[(3S)-1-(pyridin-3-ylméthyl)-5',6'-dihydrospiro[pyrrolidine-3,4'-pyrrolo[1,2-b]pyrazol]-2'-yl]-3-(trifluorométhyl)pyridin-2-amine
5-[(3R)-1-(pyridin-3-ylméthyl)-5',6'-dihydrospiro[pyrrolidine-3,4'-pyrrolo[1,2-b]pyrazol]-2'-yl]-3-(trifluorométhyl)pyridin-2-amine
5-{(*rac*)-1-[(1H-pyrazol-3-yl)méthyl]-5',6'-dihydrospiro[pyrrolidine-3,4'-pyrrolo[1,2-b]pyrazol]-2'-yl}-3-(trifluorométhyl)pyridin-2-amine
5-[(3S)-1-(1H-imidazol-2-ylméthyl)-5',6'-dihydrospiro[pyrrolidine-3,4'-pyrrolo[1,2-b]pyrazol]-2'-yl]-3-(trifluorométhyl)pyridin-2-amine
5-[(3R)-1-(1H-imidazol-2-ylméthyl)-5',6'-dihydrospiro[pyrrolidine-3,4'-pyrrolo[1,2-b]pyrazol]-2'-yl]-3-(trifluorométhyl)pyridin-2-amine
5-{(*rac*)-1-[(4-méthyl-1H-pyrazol-3-yl)méthyl]-5',6'-dihydrospiro[pyrrolidine-3,4'-pyrrolo[1,2-b]pyrazol]-2'-yl}-3-(trifluorométhyl)pyridin-2-amine
5-{(*rac*)-1-[(1H-pyrazol-4-yl)méthyl]-5',6'-dihydrospiro[pyrrolidine-3,4'-pyrrolo[1,2-b]pyrazol]-2'-yl}-3-(trifluorométhyl)pyridin-2-amine
5-{(*rac*)-1-[(1-méthyl-1H-pyrazol-4-yl)méthyl]-5',6'-dihydrospiro[pyrrolidine-3,4'-pyrrolo[1,2-b]pyrazol]-2'-yl}-3-(trifluorométhyl)pyridin-2-amine
5-{(*rac*)-1-[(1H-imidazol-4-yl)méthyl]-5',6'-dihydrospiro[pyrrolidine-3,4'-pyrrolo[1,2-b]pyrazol]-2'-yl}-3-(trifluorométhyl)pyridin-2-amine
5-{(*rac*)-1-[(1-méthyl-1H-imidazol-4-yl)méthyl]-5',6'-dihydrospiro[pyrrolidine-3,4'-pyrrolo[1,2-b]pyrazol]-2'-yl}-3-(trifluorométhyl)pyridin-2-amine
5-{(*rac*)-1-[(4-chloro-1H-pyrazol-5-yl)méthyl]-5',6'-dihydrospiro[pyrrolidine-3,4'-pyrrolo[1,2-b]pyrazol]-2'-yl}-3-(trifluorométhyl)pyridin-2-amine
3-[(*2*-fluorophényl)méthoxy]-5-{(*rac*)-1-[(1H-imidazol-2-yl)méthyl]-5',6'-dihydrospiro[pyrrolidine-3,4'-pyrrolo[1,2-b]pyrazol]-2'-yl}pyridin-2-amine
3-[(3-fluorophényl)méthoxy]-5-{(*rac*)-1-[(1H-imidazol-2-yl)méthyl]-5',6'-dihydrospiro[pyrrolidine-3,4'-pyrrolo[1,2-b]pyrazol]-2'-yl}pyridin-2-amine
5*-*{(*rac*)-1-[(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine
5-{1-[(1H-imidazol-2-yl)méthyl]-5',6'-dihydrospiro[azétidine-3,4'-pyrrolo[1,2-b]pyrazol]-2'-yl}-3-(trifluorométhyl)pyridin-2-amine
5-{(*rac*)-1'-[(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine
2-amino-5-{1-[(1H-imidazol-2-yl)méthyl]-5',6'-dihydrospiro[azétidine-3,4'-pyrrolo[1,2-b]pyrazol]-2'-yl}pyridine-3-carbonitrile
5-{(*rac*)-1-[(1-méthyl-1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine
5-{(*rac*)-1-[(1-méthyl-1H-pyrazol-3-yl)méthyl]-5',6'-dihydrospiro[pyrrolidine-3,4'-pyrrolo[1,2-b]pyrazol]-2'-yl}-3-(trifluorométhyl)pyridin-2-amine
2-amino-5-{(*rac*)-1-[(1H-imidazol-2-yl)méthyl]-5',6'-dihydrospiro[pyrrolidine-3,4'-pyrrolo[1,2-b]pyrazol]-2'-yl}pyridine-3-carbonitrile
5-{1-[(1-méthyl-1H-imidazol-2-yl)méthyl]-5',6'-dihydrospiro[azétidine-3,4'-pyrrolo[1,2-b]pyrazol]-2'-yl}-3-(trifluorométhyl)pyridin-2-amine
5-{(*rac*)-1-[(5-méthyl-1H-pyrazol-3-yl)méthyl]-5',6'-dihydrospiro[pyrrolidine-3,4'-pyrrolo[1,2-b]pyrazol]-2'-yl}-3-(trifluorométhyl)pyridin-2-amine
5-{(*rac*)-1-[(2,4-diméthyl-1H-imidazol-5-yl)méthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (stéréoisomère 1)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (stéréoisomère 2)
5-{(3R)-1-[(1-(1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (stéréoisomère 1)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (stéréoisomère 2)
5-{(3R)-1-[1-(1-méthyl-1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine
3-(trifluorométhyl)-5-[(3R)-1-{[4-(trifluorométhyl)-1H-imidazol-2-yl]méthyl}-5',6'-dihydrospiro[pyrrolidine-3,4'-pyrrolo[1,2-b]pyrazol]-2'-yl]pyridin-2-amine
acide 2-amino-5-[(*rac*)-1-(éthylcarbamoyl)-5',6'-dihydrospiro[pyrrolidine-3,4'-pyrrolo[1,2-b]pyrazol]-2'-yl]pyridine-3-carboxylique
5-[(3S)-1-(pyridin-2-ylméthyl)-5',6'-dihydrospiro[pyrrolidine-3,4'-pyrrolo[1,2-b]pyrazol]-2'-yl]-3-(trifluorométhyl)pyridin-2-amine
5-[(3R)-1-(pyridin-2-ylméthyl)-5',6'-dihydrospiro[pyrrolidine-3,4'-pyrrolo[1,2-b]pyrazol]-2'-yl]-3-(trifluorométhyl)pyridin-2-amine
5-[(3S)-1-(pyridin-4-ylméthyl)-5',6'-dihydrospiro[pyrrolidine-3,4'-pyrrolo[1,2-b]pyrazol]-2'-yl]-3-(trifluorométhyl)pyridin-2-amine
5-[(3R)-1-(pyridin-4-ylméthyl)-5',6'-dihydrospiro[pyrrolidine-3,4'-pyrrolo[1,2-b]pyrazol]-2'-yl]-3-(trifluorométhyl)pyridin-2-amine
5-{(3R)-1-[3-(diméthylamino)propyl]-5',6'-dihydrospiro[pyrrolidine-3,4'-pyrrolo[1,2-b]pyrazol]-2'-yl}-3-(trifluorométhyl)pyridin-2-amine
5-{(3R)-1-[2-(diméthylamino)éthyl]-5',6'-dihydrospiro[pyrrolidine-3,4'-pyrrolo[1,2-b]pyrazol]-2'-yl}-3-(trifluorométhyl)pyridin-2-amine
2-amino-1-{2'-[6-amino-5-(trifluorométhyl)pyridin-3-yl]-5',6'-dihydrospiro[pyrrolidine-3,4'-pyrrolo[1,2-b]pyrazol]-1-yl}éthan-1-one (stéréoisomère 2)
2-amino-1-{2'-[6-amino-5-(trifluorométhyl)pyridin-3-yl]-5',6'-dihydrospiro[pyrrolidine-3,4'-pyrrolo[1,2-b]pyrazol]-1-yl}éthan-1-one (stéréoisomère 1)
2-amino-1-{2-[6-amino-5-(trifluorométhyl)pyridin-3-yl]-6,7-dihydrospiro[pyrazolo[5,1-c][1,4]oxazine-4,3'-pyrrolidin]-1'-yl}éthan-1-one (stéréoisomère 2)
2-amino-1-{2-[6-amino-5-(trifluorométhyl)pyridin-3-yl]-6,7-dihydrospiro[pyrazolo[5,1-c][1,4]oxazine-4,3'-pyrrolidin]-1'-y1} éthan-1-one (stéréoisomère 1)
2-amino-1-{2'-[6-amino-5-(trifluorométhyl)pyridin-3-yl]-5',6'-dihydrospiro[azétidine-3,4'-pyrrolo[1,2-b]pyrazol]-1-yl}éthan-1-one
(2S)-2-amino-1-{2'-[6-amino-5-(trifluorométhyl)pyridin-3-yl]-5',6'-dihydrospiro[azétidine-3,4'-pyrrolo[1,2-b]pyrazol]-1-yl}propan-1-one
(2R)-2-amino-1-{2'-[6-amino-5-(trifluorométhyl)pyridin-3-yl]-5',6'-dihydrospiro[azétidine-3,4'-pyrrolo[1,2-b]pyrazol]-1-yl}propan-1-one
(*rac*)-2'-[6-amino-5-(1-méthylpipéridin-4-yl)pyridin-3-yl]-N-éthyl-5',6'-dihydrospiro[pyrrolidine-3,4'-pyrrolo[1,2-b]pyrazole]-1-carboxamide
5-{(*rac*)-1-[(5-méthy1-1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine
2'-{6-amino-5-[(1R)-1-(2,6-dichloro-3-fluorophényl)éthoxy]pyridin-3-yl}-N-éthyl-5',6'-dihydrospiro[pyrrolidine-3,4'-pyrrolo[1,2-b]pyrazole]-1-carboxamide (isomère 1)
2'-{6-amino-5-[(1R)-1-(2,6-dichloro-3-fluorophényl)éthoxy]pyridin-3-yl}-N-éthyl-5',6'-dihydrospiro[pyrrolidine-3,4'-pyrrolo[1,2-b]pyrazole]-1-carboxamide (isomère 2)
5-{(3'R)-1'-[(1S)-1-(1H-imidazol-2-yl)propyl]-6,7-dihydrospiro[pyrazolo[5,1-c][1,4]oxazine-4,3'-pyrrolidin]-2-yl}-3-(trifluorométhyl)pyridin-2-amine
5-{(3'R)-1'-[(1R)-1-(1H-imidazol-2-yl)propyl]-6,7-dihydrospiro[pyrazolo[5,1-c][1,4]oxazine-4,3'-pyrrolidin]-2-yl}-3-(trifluorométhyl)pyridin-2-amine
5-{(3'R)-1'-[(S)-cyclopropyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine
5-{(3'R)-1'-[(R)-cyclopropyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine
5-{(3R)-1-[(S)-cyclopropyl(1H-imidazol-2-yl)méthyl]-5',6'-dihydrospiro[pyrrolidine-3,4'-pyrrolo[1,2-b]pyrazol]-2'-yl}-3-(trifluorométhoxy)pyridin-2-amine
5-{(3R)-1-[(R)-cyclopropyl(1H-imidazol-2-yl)méthyl]-5',6'-dihydrospiro[pyrrolidine-3,4'-pyrrolo[1,2-b]pyrazol]-2'-yl}-3-(trifluorométhoxy)pyridin-2-amine
5-{(3R)-1-[(1S)-1-(1H-imidazol-2-yl)propyl]-5',6'-dihydrospiro[pyrrolidine-3,4'-pyrrolo[1,2-b]pyrazol]-2'-yl}-3-(trifluorométhoxy)pyridin-2-amine
5-{(3R)-1-[(1R)-1-(1H-imidazol-2-yl)propyl]-5',6'-dihydrospiro[pyrrolidine-3,4'-pyrrolo[1,2-b]pyrazol]-2'-yl}-3-(trifluorométhoxy)pyridin-2-amine
5-{(3R)-1-[(R)-cyclopropyl(1H-imidazol-2-yl)méthyl]-5',6'-dihydrospiro[pyrrolidine-3,4'-pyrrolo[1,2-b]pyrazol]-2'-yl}-3-(difluorométhoxy)pyridin-2-amine
5-{(3R)-1-[(S)-cyclopropyl(1H-imidazol-2-yl)méthyl]-5',6'-dihydrospiro[pyrrolidine-3,4'-pyrrolo[1,2-b]pyrazol]-2'-yl}-3-(difluorométhoxy)pyridin-2-amine
3-(difluorométhoxy)-5-{(3R)-1-[(1R)-1-(1H-imidazol-2-yl)propyl]-5',6'-dihydrospiro[pyrrolidine-3,4'-pyrrolo[1,2-b]pyrazol]-2'-yl}pyridin-2-amine
3-(difluorométhoxy)-5-{(3R)-1-[(1S)-1-(1H-imidazol-2-yl)propyl]-5',6'-dihydrospiro[pyrrolidine-3,4'-pyrrolo[1,2-b]pyrazol]-2'-yl}pyridin-2-amine
2-amino-5-{(3R)-1-[(R)-cyclopropyl(1H-imidazol-2-yl)méthyl]-5',6'-dihydrospiro[pyrrolidine-3,4'-pyrrolo[1,2-b]pyrazol]-2'-yl}nicotinonitrile
2-amino-5-{(3R)-1-[(S)-cyclopropyl(1H-imidazol-2-yl)méthyl]-5',6'-dihydrospiro[pyrrolidine-3,4'-pyrrolo[1,2-b]pyrazol]-2'-yl}nicotinonitrile
2-amino-5-{(3R)-1-[(1R)-1-(1H-imidazol-2-yl)propyl]-5',6'-dihydrospiro[pyrrolidine-3,4'-pyrrolo[1,2-b]pyrazol]-2'-yl}nicotinonitrile
2-amino-5-{(3R)-1-[(1S)-1-(1H-imidazol-2-yl)propyl]-5',6'-dihydrospiro[pyrrolidine-3,4'-pyrrolo[1,2-b]pyrazol]-2'-yl}nicotinonitrile
(3R)-2'-(6-amino-5-{[1-(6-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1)
(3R)-2'-(6-amino-5-{[1-(6-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 2)
(3R)-2'-(6-amino-5-{[1-(5-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1)
(3R)-2'-(6-amino-5-{[1-(5-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 2)
(3R)-2'-(6-amino-5-{[1-(4-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1)
(3R)-2'-(6-amino-5-{[1-(4-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 2)
(3R)-2'-(6-amino-5-{[1-(3-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1)
(3R)-2'-(6-amino-5-{[1-(3-méthylpyridin-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 2)
(3R)-2'-(6-amino-5-{[1-(1,4-diméthyl-1H-pyrazol-5-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1)
(3R)-2'-(6-amino-5-{[1-(1,4-diméthyl-1H-pyrazol-5-yl)éthyl]oxy}pyridin-3-yl)-N-éthyl-5',6'-dihydrospiro [pyrrolidine-3,4'-pyrrolo[1,2-b]pyrazole]-1-carboxamide (diastéréomère 2)
(3R)-2'-(6-amino-5-{[-1-(1,4-diméthyl-1H-pyrazol-5-yl)éthyl]oxy}pyridin-3-yl)-N-(propan-2-yl)-5',6'-dihydrospiro[pyrrolidine-3,4'-pyrrolo[1,2-b]pyrazole]-1-carboxamide (diastéréomère 1)
(3R)-2'-(6-amino-5-{[1-(1,4-diméthyl-1H-pyrazol-5-yl)éthyl]oxy}pyridin-3-yl)-N-(propan-2-yl)-5',6'-dihydrospiro[pyrrolidine-3,4'-pyrrolo[1,2-b]pyrazole]-1-carboxamide (diastéréomère 2)
(3R)-2'-{6-amino-5-[(1S)-1-(2,6-difluorophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1R)-1-(5-fluoropyridin-2-yl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1R)-1-(3-fluoropyridin-2-yl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1R)-1-(2,6-difluorophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[1-(2-cyanophényl)éthoxy]pyridin-3-yl}-N-éthyl-5',6'-dihydro-1H-spiro[pyrrolidine-3,4'-pyrrolo[1,2-b]pyrazole]-1-carboxamide (diastéréomère 1 et diastéréomère 2)
(3R)-2'-{6-amino-5-[1-(2-cyanophényl)éthoxy]pyridin-3-yl}-N-éthyl-5',6'-dihydro-1H-spiro[pyrrolidine-3,4'-pyrrolo[1,2-b]pyrazole]-1-carboxamide (diastéréomère 1)
(3R)-2'-{6-amino-5-[1-(2-cyanophényl)éthoxy]pyridin-3-yl}-N-éthyl-5',6'-dihydro-1H-spiro[pyrrolidine-3,4'-pyrrolo[1,2-b]pyrazole]-1-carboxamide (diastéréomère 2)
(3'R)-2-[6-amino-5-(trifluorométhyl)pyridin-3-yl]-N-[1-(1-éthyl-1H-pyrazol-3-yl)éthyl]-6,7-dihydro-1'H-spiro[pyrazolo[5,1-c][1,4]oxazine-4,3'-pyrrolidine]-l'-carboxamide (diastéréomère 1 et diastéréomère 2)
(3'R)-2-[6-amino-5-(trifluorométhyl)pyridin-3-yl]-N-[(1-méthyl-1H-pyrazol-5-yl)méthyl]-6,7-dihydrospiro[pyrazolo[5,1-c][1,4]oxazine-4,3'-pyrrolidine]-1'-carboxamide
(3'R)-2-[6-amino-5-(trifluorométhyl)pyridin-3-yl]-N-[(1-méthyl-1H-pyrazol-4-yl)méthyl]-6,7-dihydrospiro[pyrazolo[5,1-c][1,4]oxazine-4,3'-pyrrolidine]-1'-carboxamide
(3'R)-2-[6-amino-5-(trifluorométhyl)pyridin-3-yl]-N-[1-(1-méthyl-1H-pyrazol-5-yl)éthyl]-6,7-dihydro-1'H*-*spiro[pyrazolo[5,1-c][1,4]oxazine-4,3'-pyrrolidine]-l'-carboxamide (diastéréomère 1 et diastéréomère 2)
(3'R)-2-[6-amino-5-(trifluorométhyl)pyridin-3-yl]-N-[1-(1-méthyl-1H-pyrazol-5-yl)éthyl]-6,7-dihydro-1'H-spiro[pyrazolo[5,1-c][1,4]oxazine-4,3'-pyrrolidine]-l'-carboxamide (diastéréomère 1)
(3'R)-2-[6-amino-5-(trifluorométhyl)pyridin-3-yl]-N-[1-(1-méthyl-1H-pyrazol-5-yl)éthyl]-6,7-dihydro-1'H-spiro[pyrazolo[5,1-c][1,4]oxazine-4,3'-pyrrolidine]-l'-carboxamide (diastéréomère 2)
(3'R)-2-[6-amino-5-(trifluorométhyl)pyridin-3-yl]-N-[1-(1,3-diméthyl-1H-pyrazol-4-yl)éthyl]-6,7-dihydro-1'H-spiro[pyrazolo[5,1-c][1,4]oxazine-4,3'-pyrrolidine]-l'-carboxamide (diastéréomère 1 et diastéréomère 2)
(3'R)-2-[6-amino-5-(trifluorométhyl)pyridin-3-yl]-N-[1-(1,3-diméthyl-1H-pyrazol-4-yl)éthyl]-6,7-dihydro-1'H-spiro[pyrazolo[5,1-c][1,4]oxazine-4,3'-pyrrolidine]-l'-carboxamide (diastéréomère 1)
(3'R)-2-[6-amino-5-(trifluorométhyl)pyridin-3-yl]-N-[1-(1,3-diméthyl-1H-pyrazol-4-yl)éthyl]-6,7-dihydro-1'H-spiro[pyrazolo[5,1-c][1,4]oxazine-4,3'-pyrrolidine]-l'-carboxamide (diastéréomère 2)
(3R)-2'-{6-amino-5-[(1R)-1-(3-cyanophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1R)-1-(4-cyanophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)éthoxy]pyridin-3-yl}-N-éthyl-5',6'-dihydrospiro[pyrrolidine-3,4'-pyrrolo[1,2-b]pyrazole]-1-carboxamide
(3R)-2'-[6-amino-5-(1-phénylpropoxy)pyridin-3-yl]-N-éthyl-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(1-phénylpropoxy)pyridin-3-yl]-N-éthyl-5',6'-dihydro-1H-spiro[pyrrolidine-3,4'-pyrrolo[1,2-b]pyrazole]-1-carboxamide (diastéréomère 1)
(3R)-2'-[6-amino-5-(1-phénylpropoxy)pyridin-3-yl]-N-éthyl-5',6'-dihydro-1H-spiro[pyrrolidine-3,4'-pyrrolo[1,2-b]pyrazole]-1-carboxamide (diastéréomère 2)
(3R)-2'-{6-amino-5-[(1S)-1-(3-cyanophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1S)-1-(4-cyanophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1S)-1-(3,5-difluoropyridin-4-yl)éthoxy]pyridin-3-yl}-N-éthyl-5',6'-dihydrospiro[pyrrolidine-3,4'-pyrrolo[1,2-b]pyrazole]-1-carboxamide
(3'R)-2-[6-amino-5-(trifluorométhyl)pyridin-3-yl]-N-[1-(1,3,4-triméthyl-1H-pyrazol-5-yl)éthyl]-6,7-dihydro-1'H-spiro[pyrazolo[5,1-c][1,4]oxazine-4,3'-pyrrolidine]-l'-carboxamide (diastéréomère 1 et diastéréomère 2)
(3'R)-2-[6-amino-5-(trifluorométhyl)pyridin-3-yl]-N-[1-(1,3,4-triméthyl-1H-pyrazol-5-yl)éthyl]-6,7-dihydro-1'H-spiro[pyrazolo[5,1-c][1,4]oxazine-4,3'-pyrrolidine]-l'-carboxamide (diastéréomère 1)
(3'R)-2-[6-amino-5-(trifluorométhyl)pyridin-3-yl]-N-[1-(1,3,4-triméthyl-1H-pyrazol-5-yl)éthyl]-6,7-dihydro-1'H-spiro[pyrazolo[5,1-c][1,4]oxazine-4,3'-pyrrolidine]-l'-carboxamide (diastéréomère 2)
(3'R)-2-[6-amino-5-(trifluorométhyl)pyridin-3-yl]-N-[1-(1,5-diméthyl-1H-pyrazol-4-yl)éthyl]-6,7-dihydro-1'H-spiro[pyrazolo[5,1-c][1,4]oxazine-4,3'-pyrrolidine]-l'-carboxamide (diastéréomère 1 et diastéréomère 2)
(3'R)-2-[6-amino-5-(trifluorométhyl)pyridin-3-yl]-N-[1-(1-méthyl-1H-pyrazol-4-yl)éthyl]-6,7-dihydro-1'H-spiro[pyrazolo[5,1-c][1,4]oxazine-4,3'-pyrrolidine]-l'-carboxamide (diastéréomère 1 et diastéréomère 2)
(3R)-2'-{6-amino-5-[(1R)-1-(3-fluoropyridin-2-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(3-fluoropyridin-2-yl)éthoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(3,5-difluoropyridin-4-yl)éthoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-phényléthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[1-(1-méthyl-1H-pyrazol-5-yl)éthoxy]pyridin-3-yl}-N-isopropyl-5',6'-dihydro-1H-spiro[pyrrolidine-3,4'-pyrrolo[1,2-b]pyrazole]-1-carboxamide (diastéréomère 1)
(3R)-2'-{6-amino-5-[1-(1-méthyl-1H-pyrazol-5-yl)éthoxy]pyridin-3-yl}-N-isopropyl-5',6'-dihydro-1H-spiro[pyrrolidine-3,4'-pyrrolo[1,2-b]pyrazole]-1-carboxamide (diastéréomère 2)
(3R)-2'-{6-amino-5-[1-(1-méthyl-1H-pyrazol-5-yl)éthoxy]pyridin-3-yl}-N-cyclobutyl-5',6'-dihydro-1H-spiro[pyrrolidine-3,4'-pyrrolo[1,2-b]pyrazole]-1-carboxamide (diastéréomère 1)
(3R)-2'-{6-amino-5-[1-(1-méthyl-1H-pyrazol-5-yl)éthoxy]pyridin-3-yl}-N-cyclobutyl-5',6'-dihydro-1H-spiro[pyrrolidine-3,4'-pyrrolo[1,2-b]pyrazole]-1-carboxamide (diastéréomère 2)
(3R)-2'-{6-amino-5-[(1R)-1-(2-fluorophényl)éthoxy]pyridin-3-yl}-N-(propan-2-yl)-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-(1-méthyl-1H-pyrazol-5-yl)éthyl]-5',6'-dihydro-1H-spiro[azétidine-3,4'-pyrrolo[1,2-b]pyrazole]-1-carboxamide (énantiomère 1 et énantiomère 2)
2'-[6-amino-5-(trifluorométhyl)pyridin-3-yl]-N-[1-(1-méthyl-1H-pyrazol-5-yl)éthyl]-5',6'-dihydro-1H-spiro[azétidine-3,4'-pyrrolo[1,2-b]pyrazole]-1-carboxamide (énantiomère 1)
2'-[6-amino-5-(trifluorométhyl)pyridin-3-yl]-N-[1-(1-méthyl-1H-pyrazol-5-yl)éthyl]-5',6'-dihydro-1H-spiro[azétidine-3,4'-pyrrolo[1,2-b]pyrazole]-1-carboxamide (énantiomère 2)
2'-[6-amino-5-(trifluorométhyl)pyridin-3-yl]-N-[1-(1,3-diméthyl-1H-pyrazol-4-yl)éthyl]-5',6'-dihydro-1H-spiro[azétidine-3,4'-pyrrolo[1,2-b]pyrazole]-1-carboxamide (énantiomère 1 et énantiomère 2)
2'-[6-amino-5-(trifluorométhyl)pyridin-3-yl]-N-[1-(1,3-diméthyl-1H-pyrazol-4-yl)éthyl]-5',6'-dihydro-1H-spiro[azétidine-3,4'-pyrrolo[1,2-b]pyrazole]-1-carboxamide (énantiomère 1)
2'-[6-amino-5-(trifluorométhyl)pyridin-3-yl]-N-[1-(1,3-diméthyl-1H-pyrazol-4-yl)éthyl]-5',6'-dihydro-1H-spiro[azétidine-3,4'-pyrrolo[1,2-b]pyrazole]-1-carboxamide (énantiomère 2)
(3S)-2'-[6-amino-5-(trifluorométhyl)pyridin-3-yl]-N-[1-(1-méthyl-1H-pyrazol-5-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3S)-2'-[6-amino-5-(trifluorométhyl)pyridin-3-yl]-N-[1-(1,3-diméthyl-1H-pyrazol-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-{6-amino-5-[(1R)-1-(2,6-difluorophényl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-(6-amino-5-{méthyl[(1R)-1-phényléthyl]amino}pyridin-3-yl)-N-éthyl-5',6'-dihydrospiro[pyrrolidine-3,4'-pyrrolo[1,2-b]pyrazole]-1-carboxamide
2'-[6-amino-5-(trifluorométhyl)pyridin-3-yl]-N-[1-(1,3,5-triméthyl-1H-pyrazol-4-yl)éthyl]-5',6'-dihydro-1H-spiro[azétidine-3,4'-pyrrolo[1,2-b]pyrazole]-1-carboxamide (diastéréomère 1 et diastéréomère 2)
2'-[6-amino-5-(trifluorométhyl)pyridin-3-yl]-N-[1-(1,3,5-triméthyl-1H-pyrazol-4-yl)éthyl]-5',6'-dihydro-1H-spiro[azétidine-3,4'-pyrrolo[1,2-b]pyrazole]-1-carboxamide (diastéréomère 1)
2'-[6-amino-5-(trifluorométhyl)pyridin-3-yl]-N-[1-(1,3,5-triméthyl-1H-pyrazol-4-yl)éthyl]-5',6'-dihydro-1H-spiro[azétidine-3,4'-pyrrolo[1,2-b]pyrazole]-1-carboxamide (diastéréomère 2)
(3R)-2'-{6-amino-5-[(1R)-1-(4-méthylpyridin-2-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(4-méthylpyridin-2-yl)éthoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(6-méthylpyridin-2-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(6-méthylpyridin-2-yl)éthoxy]pyridin-3-yl}-N-cyclobutyl-5',6'-dihydrospiro[pyrrolidine-3,4'-pyrrolo[1,2-b]pyrazole]-1-carboxamide
(3S)-2'-[6-amino-5-(trifluorométhyl)pyridin-3-yl]-N-[2-(3-fluoropyridin-2-yl)propan-2-yl]-5',6'-dihydrospiro[pyrrolidine-3,4'-pyrrolo[1,2-b]pyrazole]-1-carboxamide
(3S)-2'-[6-amino-5-(trifluorométhyl)pyridin-3-yl]-N-[1-(1,3,5-triméthyl-1H-pyrazol-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[1-(1,3,5-triméthyl-1H-pyrazol-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[1-(1,3,5-triméthyl-1H-pyrazol-4-yl)éthyl]-5',6'-dihydro-1H-spiro[pyrrolidine-3,4'-pyrrolo[1,2-b]pyrazole]-1-carboxamide (diastéréomère 1)
(3R)-2'-[6-amino-5-(trifluorométhyl)pyridin-3-yl]-N-[1-(1,3,5-triméthyl-1H-pyrazol-4-yl)éthyl]-5',6'-dihydro-1H-spiro[pyrrolidine-3,4'-pyrrolo[1,2-b]pyrazole]-1-carboxamide (diastéréomère 2)
(2'-{6-amino-5-[(1R)-1-phényléthoxy]pyridin-3-yl}-5',6'-dihydrospiro[azétidine-3,4'-pyrrolo[1,2-b]pyrazol]-1-yl)(cyclopropyl)méthanone
2'-[6-amino-5-(trifluorométhyl)pyridin-3-yl]-N-[1-(1-éthyl-1H-pyrazol-3-yl)éthyl]-5',6'-dihydro-1H-spiro[azétidine-3,4'-pyrrolo[1,2-b]pyrazole]-1-carboxamide (énantiomère 1)
2'-[6-amino-5-(trifluorométhyl)pyridin-3-yl]-N-[1-(1-éthyl-1H-pyrazol-3-yl)éthyl]-5',6'-dihydro-1H-spiro[azétidine-3,4'-pyrrolo[1,2-b]pyrazole]-1-carboxamide (énantiomère 2)
(3S)-2'-[6-amino-5-(trifluorométhyl)pyridin-3-yl]-N-[1-(1-éthyl-1H-pyrazol-3-yl)éthyl]-5',6'-dihydro-1H-spiro[pyrrolidine-3,4'-pyrrolo[1,2-b]pyrazole]-1-carboxamide (diastéréomère 1)
(3S)-2'-[6-amino-5-(trifluorométhyl)pyridin-3-yl]-N-[1-(1-éthyl-1H-pyrazol-3-yl)éthyl]-5',6'-dihydro-1H-spiro[pyrrolidine-3,4'-pyrrolo[1,2-b]pyrazole]-1-carboxamide (diastéréomère 2)
5-{(3'R)-1'-[cyclopropyl(1H-1,2,4-triazol-5-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[cyclopropyl(1H-1,2,4-triazol-5-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[cyclopropyl(1H-1,2,4-triazol-5-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(1H-1,2,4-triazol-5-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[1-(1H-1,2,4-triazol-5-yl)propyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[1-(1H-1,2,4-Triazol-5-yl)propyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3'R)-1'-[2,2-difluoro-1-(1H-imidazol-2-yl)éthyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3'R)-1'-[2,2-difluoro-1-(1H-imidazol-2-yl)éthyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(difluorométhyl)pyridin-2-amine (diastéréomère 1 et diastéréomère 2)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(difluorométhyl)pyridin-2-amine (diastéréomère 1)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(difluorométhyl)pyridin-2-amine (diastéréomère 2)
2-amino-5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}nicotinonitrile (diastéréomère 1 et diastéréomère 2)
2-amino-5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}nicotinonitrile (diastéréomère 1)
2-amino-5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}nicotinonitrile (diastéréomère 2)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(difluorométhoxy)pyridin-2-amine (diastéréomère 1 et diastéréomère 2)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(difluorométhoxy)pyridin-2-amine (diastéréomère 1)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(difluorométhoxy)pyridin-2-amine (diastéréomère 2)
5-{1-[1-(1H-imidazol-2-yl)propyl]-5',6'-dihydrospiro[azétidine-3,4'-pyrrolo[1,2-b]pyrazol]-2'-yl}-3-(trifluorométhyl)pyridin-2-amine (énantiomère 1 et énantiomère 2)
5-{1-[1-(1H-imidazol-2-yl)propyl]-5',6'-dihydrospiro[azétidine-3,4'-pyrrolo[1,2-b]pyrazol]-2'-yl}-3-(trifluorométhyl)pyridin-2-amine (énantiomère 1)
5-{1-[1-(1H-imidazol-2-yl)propyl]-5',6'-dihydrospiro[azétidine-3,4'-pyrrolo[1,2-b]pyrazol]-2'-yl}-3-(trifluorométhyl)pyridin-2-amine (énantiomère 2)
5-{(3'R)-1'-[cyclobutyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[cyclobutyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[cyclobutyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3'R)-1'-[1-(1H-imidazol-2-yl)-2-méthylpropyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[1-(1H-imidazol-2-yl)-2-méthylpropyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[1-(1H-imidazol-2-yl)-2-méthylpropyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3R)-1-[cyclobutyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclobutyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[cyclobutyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[1-(1H-imidazol-2-yl)-2,2-diméthylpropyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{1-[cyclopropyl(1H-imidazol-2-yl)méthyl]-5',6'-dihydrospiro[azétidine-3,4'-pyrrolo[1,2-b]pyrazol]-2'-yl}-3-(trifluorométhyl)pyridin-2-amine (énantiomère 1 et énantiomère 2)
5-{1-[cyclopropyl(1H-imidazol-2-yl)méthyl]-5',6'-dihydrospiro[azétidine-3,4'-pyrrolo[1,2-b]pyrazol]-2'-yl}-3-(trifluorométhyl)pyridin-2-amine (énantiomère 1)
5-{1-[cyclopropyl(1H-imidazol-2-yl)méthyl]-5',6'-dihydrospiro[azétidine-3,4'-pyrrolo[1,2-b]pyrazol]-2'-yl}-3-(trifluorométhyl)pyridin-2-amine (énantiomère 2)
5-{(3R)-1-[2,2-difluoro-1-(1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[2,2-difluoro-1-(1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[2,2-difluoro-1-(1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3S)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3S)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3S)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
2-amino-5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}nicotinonitrile (diastéréomère 1 et diastéréomère 2)
2-amino-5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}nicotinonitrile (diastéréomère 1)
2-amino-5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}nicotinonitrile (diastéréomère 2)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(difluorométhoxy)pyridin-2-amine (diastéréomère 1 et diastéréomère 2)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(difluorométhoxy)pyridin-2-amine (diastéréomère 1)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(difluorométhoxy)pyridin-2-amine (diastéréomère 2)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastéréomère 1 et diastéréomère 2)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastéréomère 1)
2-amino-5-{(3R)-1-[1-(1H-imidazol-2-yl)propyl]-5',6'-dihydrospiro[pyrrolidine-3,4'-pyrrolo[1,2-b]pyrazol]-2'-yl}nicotinonitrile (diastéréomère 2)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhoxy)pyridin-2-amine (diastéréomère 1 et diastéréomère 2)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhoxy)pyridin-2-amine (diastéréomère 1)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhoxy)pyridin-2-amine (diastéréomère 2)
3-(difluorométhoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastéréomère 1 et diastéréomère 2)
3-(difluorométhoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastéréomère 1)
3-(difluorométhoxy)-5-{(3R)-1-[1-(1H-imidazol-2-yl)propyl]-5',6'-dihydrospiro[pyrrolidine-3,4'-pyrrolo[1,2-b]pyrazol]-2'-yl}pyridin-2-amine (diastéréomère 2)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluorométhoxy)pyridin-2-amine (diastéréomère 1 et diastéréomère 2)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluorométhoxy)pyridin-2-amine (diastéréomère 1)
5-{(3R)-1-[1-(1H-imidazol-2-yl)propyl]-5',6'-dihydrospiro[pyrrolidine-3,4'-pyrrolo[1,2-b]pyrazol]-2'-yl}-3-(trifluorométhoxy)pyridin-2-amine (diastéréomère 2)
5-{(3R)-1-[1-(1H-imidazol-2-yl)-2-méthylpropyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(1H-imidazol-2-yl)-2-méthylpropyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(1H-imidazol-2-yl)-2-méthylpropyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3S)-1-[1-(1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[1-(1H-imidazol-2-yl)propyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
{(3R)-2'-[6-amino-5-(trifluorométhyl)pyridin-3-yl]-5',6'-dihydrospiro[pyrrolidine-3,4'-pyrrolo[1,2-b]pyrazol]-1-yl}(1H-imidazol-2-yl)méthanone
{(3R)-2'-[6-amino-5-(trifluorométhyl)pyridin-3-yl]-5',6'-dihydrospiro[pyrrolidine-3,4'-pyrrolo[1,2-b]pyrazol]-1-yl}(1-méthyl-1H-imidazol-2-yl)méthanone
3-[(1R)-1-(2,6-dichloro-3-fluorophényl)éthoxy]-5-{(3R)-1-[(1H-imidazol-2-yl)méthyl]-5',6'-dihydrospiro[pyrrolidine-3,4'-pyrrolo[1,2-b]pyrazol]-2'-yl}pyridin-2-amine
5-{(3R)-1-[1-(1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(1H-imidazol-2-yl)éthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
(3R)-2'-[6-amino-5-(trifluorométhyl)pyridin-3-yl]-N-[1-(3-chloropyridin-4-yl)cyclobutyl]-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[2-(3-chloropyridin-4-yl)propan-2-yl]-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[1-(pyridin-4-yl)cyclobutyl]-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[1-(pyridin-3-yl)cyclobutyl]-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[(1S)-1-(3-chloropyridin-4-yl)éthyl]-5',6'-dihydrospiro[pyrrolidine-3,4'-pyrrolo[1,2-b]pyrazole]-1-carboxamide
2'-(6-amino-5-cyanopyridin-3-yl)-N-[1-(pyridin-4-yl)cyclobutyl]-5',6'-dihydrospiro[pyrrolidine-3,4'-pyrrolo[1,2-b]pyrazole]-1-carboxamide (énantiomère 2)
(3R)-2'-[6-amino-5-(trifluorométhyl)pyridin-3-yl]-N-[1-(3-méthylpyridin-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[cyclopropyl(phényl)méthyl]-5',6'-dihydrospiro[pyrrolidine-3,4'-pyrrolo[1,2-b]pyrazole]-1-carboxamide (diastéréomère 1)
(3R)-2'-[6-amino-5-(trifluorométhyl)pyridin-3-yl]-N-[cyclopropyl(phényl)méthyl]-5',6'-dihydrospiro[pyrrolidine-3,4'-pyrrolo[1,2-b]pyrazole]-1-carboxamide (diastéréomère 2)
(3R)-2'-[6-amino-5-(trifluorométhyl)pyridin-3-yl]-N-[1-(pyridin-2-yl)cyclobutyl]-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[2-méthyl-1-(pyridin-4-yl)propyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[2-méthyl-1-(pyridin-4-yl)propyl]-5',6'-dihydro-1H-spiro[pyrrolidine-3,4'-pyrrolo[1,2-b]pyrazole]-1-carboxamide (diastéréomère 1)
(3R)-2'-[6-amino-5-(trifluorométhyl)pyridin-3-yl]-N-[2-méthyl-1-(pyridin-4-yl)propyl]-5',6'-dihydro-1H-spiro[pyrrolidine-3,4'-pyrrolo[1,2-b]pyrazole]-1-carboxamide (diastéréomère 2)
(3R)-2'-[6-amino-5-(trifluorométhyl)pyridin-3-yl]-N-[1-(3-fluoropyridin-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[1-(3-fluoropyridin-4-yl)éthyl]-5',6'-dihydro-1H-spiro[pyrrolidine-3,4'-pyrrolo[1,2-b]pyrazole]-1-carboxamide (diastéréomère 1)
(3R)-2'-[6-amino-5-(trifluorométhyl)pyridin-3-yl]-N-[1-(3-fluoropyridin-4-yl)éthyl]-5',6'-dihydro-1H-spiro[pyrrolidine-3,4'-pyrrolo[1,2-b]pyrazole]-1-carboxamide (diastéréomère 2)
(3R)-2'-[6-amino-5-(trifluorométhyl)pyridin-3-yl]-N-[(1R)-1-(3-chloropyridin-4-yl)éthyl]-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[1-(2-méthylpyridin-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[1-(3-méthoxypyridin-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[1-(pyridin-4-yl)cyclopropyl]-5',6'-dihydrospiro[pyrrolidine-3,4'-pyrrolo[1,2-b]pyrazole]-1-carboxamide
(3R)-2'-{6-amino-5-[(1R)-1-(2-méthoxyphényl)éthoxy]pyridin-3-yl}-N-éthyl-5',6'-dihydrospiro[pyrrolidine-3,4'-pyrrolo[1,2-b]pyrazole]-1-carboxamide
3-(1-{(3R)-2'-[6-amino-5-(trifluorométhyl)pyridin-3-yl]-5',6'-dihydro-1H-spiro[pyrrolidine-3,4'-pyrrolo[1,2-b]pyrazol]-1-yl}propyl)benzonitrile (diastéréomère 1 et diastéréomère 2)
3-(1-{(3R)-2'-[6-amino-5-(trifluorométhyl)pyridin-3-yl]-5',6'-dihydro-1H-spiro[pyrrolidine-3,4'-pyrrolo[1,2-b]pyrazol]-1-yl}propyl)benzonitrile (diastéréomère 1)
3-(1-{(3R)-2'-[6-amino-5-(trifluorométhyl)pyridin-3-yl]-5',6'-dihydro-1H-spiro[pyrrolidine-3,4'-pyrrolo[1,2-b]pyrazol]-1-yl}propyl)benzonitrile (diastéréomère 2)
(3S)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (énantiomère 1)
(3R)-2'-(6-amino-5-cyanopyridin-3-yl)-N-[2-(3-chloropyridin-4-yl)propan-2-yl]-5',6'-dihydrospiro[pyrrolidine-3,4'-pyrrolo[1,2-b]pyrazole]-1-carboxamide (énantiomère 2)
(3R)-2'-{6-amino-5-[(1R)-1-(3-chlorophényl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[1-(2,6-dichlorophényl)éthoxy]pyridin-3-yl}-N-éthyl-5',6'-dihydro-1H-spiro[pyrrolidine-3,4'-pyrrolo[1,2-b]pyrazole]-1-carboxamide (diastéréomère 1 et diastéréomère 2)
(3R)-2'-{6-amino-5-[1-(2,6-dichlorophényl)éthoxy]pyridin-3-yl}-N-éthyl-5',6'-dihydro-1H-spiro[pyrrolidine-3,4'-pyrrolo[1,2-b]pyrazole]-1-carboxamide (diastéréomère 1)
(3R)-2'-{6-amino-5-[1-(2,6-dichlorophényl)éthoxy]pyridin-3-yl}-N-éthyl-5',6'-dihydro-1H-spiro[pyrrolidine-3,4'-pyrrolo[1,2-b]pyrazole]-1-carboxamide (diastéréomère 2)
5-{(3R)-1-[1-(1H-pyrrol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
(3R)-2'-[6-amino-5-(trifluorométhyl)pyridin-3-yl]-N-[1-(2-méthoxypyridin-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-[1-(1-méthyl-1H-pyrazol-5-yl)éthyl]-5',6'-dihydrospiro[pyrrolidine-3,4'-pyrrolo[1,2-b]pyrazole]-1-carboxamide (diastéréomère 1)
(3R)-2'-[6-amino-5-(trifluorométhyl)pyridin-3-yl]-N-[1-(1-méthyl-1H-pyrazol-5-yl)éthyl]-5',6'-dihydrospiro[pyrrolidine-3,4'-pyrrolo[1,2-b]pyrazole]-1-carboxamide (diastéréomère 2)
3-(1-{(3R)-2'-[6-amino-5-(trifluorométhyl)pyridin-3-yl]-5',6'-dihydro-1H-spiro[pyrrolidine-3,4'-pyrrolo[1,2-b]pyrazol]-1-yl}propyl)benzamide (diastéréomère 1 et diastéréomère 2)
3-(1-{(3R)-2'-[6-amino-5-(trifluorométhyl)pyridin-3-yl]-5',6'-dihydrospiro[pyrrolidine-3,4'-pyrrolo[1,2-b]pyrazol]-1-yl}propyl)benzamide (diastéréomère 1)
3-(1-{(3R)-2'-[6-amino-5-(trifluorométhyl)pyridin-3-yl]-5',6'-dihydrospiro[pyrrolidine-3,4'-pyrrolo[1,2-b]pyrazol]-1-yl}propyl)benzamide (diastéréomère 2)
(3R)-2'-{6-amino-5-[(1R)-1-(1,3-thiazol-2-yl)éthoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[(1R)-1-(3-chlorophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[1-(2-chloro-3-fluoropyridin-4-yl)éthyl]-5',6'-dihydro-1H-spiro[pyrrolidine-3,4'-pyrrolo[1,2-b]pyrazole]-1-carboxamide (diastéréomère 1 et diastéréomère 2)
(3R)-2'-[6-amino-5-(trifluorométhyl)pyridin-3-yl]-N-{[3-(méthylsulfanyl)pyridin-4-yl]méthyl}-5',6'-dihydrospiro[pyrrolidine-3,4'-pyrrolo[1,2-b]pyrazole]-1-carboxamide
(3R)-2'-{6-amino-5-[(1R)-1-(1,3-thiazol-2-yl)éthoxy]pyridin-3-yl}-N-éthyl-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[1-(1,3-diméthyl-1H-pyrazol-4-yl)éthyl]-5',6'-dihydrospiro[pyrrolidine-3,4'-pyrrolo[1,2-b]pyrazole]-1-carboxamide (diastéréomère 1)
(3R)-2'-[6-amino-5-(trifluorométhyl)pyridin-3-yl]-N-[1-(1,3-diméthyl-1H-pyrazol-4-yl)éthyl]-5',6'-dihydrospiro[pyrrolidine-3,4'-pyrrolo[1,2-b]pyrazole]-1-carboxamide (diastéréomère 2)
(3R)-2'-{6-amino-5-[(1R)-1-(3-fluorophényl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[1-(pyridin-2-yl)cyclopropyl]-5',6'-dihydrospiro[pyrrolidine-3,4'-pyrrolo[1,2-b]pyrazole]-1-carboxamide
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(pyrimidin-5-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
(3R)-2'-{6-amino-5-[(1R)-1-(1,3-thiazol-2-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-(trifluorométhyl)pyridin-3-yl]-N-[(3-méthylpyridin-4-yl)méthyl]-5',6'-dihydrospiro[pyrrolidine-3,4'-pyrrolo[1,2-b]pyrazole]-1-carboxamide
5-{(3R)-1-[ 1-(6-méthoxypyridin-2-yl)éthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(6-méthoxypyridin-2-yl)éthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
(3R)-2'-(6-amino-5-{1-[3-(3-hydroxy-3-méthylbut-1-yn-1-yl)phényl]éthoxy}pyridin-3-yl)-N-éthyl-5',6'-dihydro-1H-spiro[pyrrolidine-3,4'-pyrrolo[1,2-b]pyrazole]-1-carboxamide (diastéréomère 1 et diastéréomère 2)
(3R)-2'-{6-amino-5-[(1R)-1-(pyridin-3-yl)éthoxy]pyridin-3-yl}-N-cyclobutyl-5',6'-dihydrospiro[pyrrolidine-3,4'-pyrrolo[1,2-b]pyrazole]-1-carboxamide
(3R)-2'-{6-amino-5-[1-(1,3-oxazol-2-yl)éthoxy]pyridin-3-yl}-N-éthyl-5',6'-dihydro-1H-spiro[pyrrolidine-3,4'-pyrrolo[1,2-b]pyrazole]-1-carboxamide (diastéréomère 1 et diastéréomère 2)
(3R)-2'-{6-amino-5-[(1R)-1-(3-fluorophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-[6-amino-5-(6,7-dihydro-5H-cyclopenta[b]pyridin-7-yloxy)pyridin-3-yl]-N-éthyl-5',6'-dihydro-1H-spiro[pyrrolidine-3,4'-pyrrolo[1,2-b]pyrazole]-1-carboxamide (diastéréomère 2)
5-{(3R)-1-[cyclopropyl(5-fluoropyridin-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
(3R)-2'-{6-amino-5-[(1R)-1-(pyrimidin-2-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(4-fluorophényl)éthoxy]pyridin-3-yl}-N-éthyl-5',6'-dihydrospiro[pyrrolidine-3,4'-pyrrolo[1,2-b]pyrazole]-1-carboxamide
(3R)-2'-{6-amino-5-[(1R)-1-(4-fluorophényl)éthoxy]pyridin-3-yl}-N-(propan-2-yl)-5',6'-dihydrospiro[pyrrolidine-3,4'-pyrrolo[1,2-b]pyrazole]-1-carboxamide
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(1,3-thiazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
(3R)-2'-{6-amino-5-[(1R)-1-(4-chlorophényl)éthoxy]pyridin-3-yl}-N-éthyl-5',6'-dihydrospiro[pyrrolidine-3,4'-pyrrolo[1,2-b]pyrazole]-1-carboxamide
5-{(3R)-1-[1-(diméthylamino)propan-2-yl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
(3R)-2'-{6-amino-5-[(1R)-1-(1,2-thiazol-5-yl)éthoxy]pyridin-3-yl}-N-éthyl-5',6'-dihydrospiro[pyrrolidine-3,4'-pyrrolo[1,2-b]pyrazole]-1-carboxamide
6-({(3R)-2'-[6-amino-5-(trifluorométhyl)pyridin-3-yl]-5',6'-dihydrospiro[pyrrolidine-3,4'-pyrrolo[1,2-b]pyrazol]-1-yl}méthyl)pyridin-2(1H)-one
(3R)-2'-{6-amino-5-[(1R)-1-(pyridin-3-yl)éthoxy]pyridin-3-yl}-N-(propan-2-yl)-5',6'-dihydrospiro[pyrrolidine-3,4'-pyrrolo[1,2-b]pyrazole]-1-carboxamide
2'-[6-amino-5-(6,7-dihydro-5H-cyclopenta[b]pyridin-7-yloxy)pyridin-3-yl]-N-éthyl-5',6'-dihydro-1H-spiro[pyrrolidine-3,4'-pyrrolo[1,2-b]pyrazole]-1-carboxamide (4 isomères)
(3R)-2'-{6-amino-5-[1-(1,3-oxazol-4-yl)éthoxy]pyridin-3-yl}-N-isopropyl-5',6'-dihydro-1H-spiro[pyrrolidine-3,4'-pyrrolo[1,2-b]pyrazole]-1-carboxamide (diastéréomère 1 et diastéréomère 2)
5-{(3R)-1-[(1,3-thiazol-2-yl)méthyl]-5',6'-dihydrospiro[pyrrolidine-3,4'-pyrrolo[1,2-b]pyrazol]-2'-yl}-3-(trifluorométhyl)pyridin-2-amine
6-({(3'R)-2-[6-amino-5-(trifluorométhyl)pyridin-3-yl]-6,7-dihydrospiro[pyrazolo[5,1-c][1,4]oxazine-4,3'-pyrrolidin]-1'-yl}méthyl)pyridin-2(1H)-one
(3S)-2'-[6-amino-5-(trifluorométhyl)pyridin-3-yl]-N-[cyclopropyl(phényl)méthyl]-5',6'-dihydrospiro[pyrrolidine-3,4'-pyrrolo[1,2-b]pyrazole]-1-carboxamide (diastéréomère 1)
(3S)-2'-[6-amino-5-(trifluorométhyl)pyridin-3-yl]-N-[cyclopropyl(phényl)méthyl]-5',6'-dihydrospiro[pyrrolidine-3,4'-pyrrolo[1,2-b]pyrazole]-1-carboxamide (diastéréomère 2)
(3R)-2'-[6-amino-5-(6,7-dihydro-5H-cyclopenta[b]pyridin-7-yloxy)pyridin-3-yl]-N-éthyl-5',6'-dihydro-1H-spiro[pyrrolidine-3,4'-pyrrolo[1,2-b]pyrazole]-1-carboxamide (diastéréomère 1)
2'-{6-amino-5-[(1R)-1-(pyridin-2-yl)éthoxy]pyridin-3-yl}-N-éthyl-5',6'-dihydrospiro[azétidine-3,4'-pyrrolo[1,2-b]pyrazole]-1-carboxamide
1-{(3R)-2'-[6-amino-5-(trifluorométhyl)pyridin-3-yl]-5',6'-dihydrospiro[pyrrolidine-3,4'-pyrrolo[1,2-b]pyrazol]-1-yl}-2-(méthylamino)éthan-1-one
(2R)-1-{(3R)-2'-[6-amino-5-(trifluorométhyl)pyridin-3-yl]-5',6'-dihydrospiro[pyrrolidine-3,4'-pyrrolo[1,2-b]pyrazol]-1-yl}-2-(méthylamino)propan-1-one
(5R)-5-({(3R)-2'-[6-amino-5-(trifluorométhyl)pyridin-3-yl]-5',6'-dihydrospiro[pyrrolidine-3,4'-pyrrolo[1,2-b]pyrazol]-1-yl}méthyl)pyrrolidin-2-one
5-{(3R)-1-[(1,3,4-thiadiazol-2-yl)méthyl]-5',6'-dihydrospiro[pyrrolidine-3,4'-pyrrolo[1,2-b]pyrazol]-2'-yl}-3-(trifluorométhyl)pyridin-2-amine
(5S)-5-({(3R)-2'-[6-amino-5-(trifluorométhyl)pyridin-3-yl]-5',6'-dihydrospiro[pyrrolidine-3,4'-pyrrolo[1,2-b]pyrazol]-1-yl}méthyl)pyrrolidin-2-one
5-{(3'R)-1'-[1-(6-méthoxypyridin-2-yl)éthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3R)-1-[cyclopropyl(6-méthoxypyridin-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3'R)-1'-[cyclopropyl(6-méthoxypyridin-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3R)-1-[1-(3-fluoropyridin-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
3-(difluorométhoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastéréomère 1 et diastéréomère 2)
3-(difluorométhoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastéréomère 1)
3-(difluorométhoxy)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastéréomère 2)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastéréomère 1 et diastéréomère 2)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastéréomère 1)
2-amino-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridine-3-carbonitrile (diastéréomère 2)
3-(difluorométhyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastéréomère 1 et diastéréomère 2)
3-(difluorométhyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastéréomère 1)
3-(difluorométhyl)-5-{(3'R)-1'-[1-(1H-imidazol-2-yl)propyl]-6,7-dihydrospiro[pyrazolo[5,1-c][1,4]oxazine-4,3'-pyrrolidin]-2-yl}pyridin-2-amine (diastéréomère 2)
(3R)-2'-(6-amino-5-{[1-(1-propyl-1H-pyrazol-5-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1 et diastéréomère 2)
(3R)-2'-(6-amino-5-{[1-(1,3-thiazol-4-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1 et diastéréomère 2)
(3R)-2'-(6-amino-5-{[1-(pyrimidin-4-yl)éthyl]oxy}pyridin-3-yl)-N-(propan-2-yl)-5',6'-dihydrospiro[pyrrolidine-3,4'-pyrrolo[1,2-b]pyrazole]-1-carboxamide (diastéréomère 1 et diastéréomère 2)
(3R)-2'-(6-amino-5-{[1-(2,4-diméthylpyrimidin-5-yl)éthyl]oxy}pyridin-3-yl)-N-(propan-2-yl)-5',6'-dihydrospiro[pyrrolidine-3,4'-pyrrolo[1,2-b]pyrazole]-1-carboxamide (diastéréomère 1)
(3R)-2'-(6-amino-5-{[1-(2,4-diméthylpyrimidin-5-yl)éthyl]oxy}pyridin-3-yl)-N-(propan-2-yl)-5',6'-dihydrospiro[pyrrolidine-3,4'-pyrrolo[1,2-b]pyrazole]-1-carboxamide (diastéréomère 2)
(3R)-2'-{6-amino-5-[(1R)-1-(5-méthyl-1,3,4-oxadiazol-2-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(1-méthyl-1H-1,2,3-triazol-5-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-(6-amino-5-{[1-(1-méthyl-1H-1,2,3-triazol-4-yl)éthyl]oxy}pyridin-3-yl)-N-(propan-2-yl)-5',6'-dihydrospiro[pyrrolidine-3,4'-pyrrolo[1,2-b]pyrazole]-1-carboxamide (diastéréomère 1 et diastéréomère 2)
(5S)-5-{[(3R)-2'-{6-amino-5-[(1R)-1-phényléthoxy]pyridin-3-yl}-5',6'-dihydrospiro[pyrrolidine-3,4'-pyrrolo[1,2-b]pyrazol]-1-yl]méthyl}pyrrolidin-2-one
(5R)-5-{[(3R)-2'-{6-amino-5-[(1R)-1-phényléthoxy]pyridin-3-yl}-5',6'-dihydrospiro[pyrrolidine-3,4'-pyrrolo[1,2-b]pyrazol]-1-yl]méthyl}pyrrolidin-2-one
(3R)-2'-(6-amino-5-{1-[4-(méthylsulfonyl)phényl]éthoxy}pyridin-3-yl)-N-isopropyl-5',6'-dihydro-1H-spiro[pyrrolidine-3,4'-pyrrolo[1,2-b]pyrazole]-1-carboxamide (diastéréomère 1 et diastéréomère 2)
(3R)-2'-{6-amino-5-[(1R)-1-(2-méthyl-1,3-thiazol-4-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-[6-amino-5-({1-[3-(méthanesulfonyl)phényl]éthyl}oxy)pyridin-3-yl]-N-(propan-2-yl)-5',6'-dihydrospiro[pyrrolidine-3,4'-pyrrolo[1,2-b]pyrazole]-1-carboxamide (diastéréomère 1 et diastéréomère 2)
(3R)-2'-(6-amino-5-{[1-(4-méthyl-1,3-thiazol-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1 et diastéréomère 2)
(3R)-2'-(6-amino-5-{[1-(4,5-diméthyl-1,3-thiazol-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1 et diastéréomère 2)
(3R)-2'-(6-amino-5-{[1-(5-méthyl-1,3-thiazol-2-yl)éthyl]oxy}pyridin-3-yl)-N-éthyl-5',6'-dihydrospiro[pyrrolidine-3,4'-pyrrolo[1,2-b]pyrazole]-1-carboxamide (diastéréomère 1 et diastéréomère 2)
(3R)-2'-{6-amino-5-[(1R)-1-(pyridin-4-yl)éthoxy]pyridin-3-yl}-N-(propan-2-yl)-5',6'-dihydrospiro[pyrrolidine-3,4'-pyrrolo[1,2-b]pyrazole]-1-carboxamide
(3R)-2'-{6-amino-5-[(1R)-1-(pyridin-4-yl)éthoxy]pyridin-3-yl}-N-cyclobutyl-5',6'-dihydrospiro[pyrrolidine-3,4'-pyrrolo[1,2-b]pyrazole]-1-carboxamide
6-(1-{(3'R)-2-[6-amino-5-(trifluorométhyl)pyridin-3-yl]-6,7-dihydrospiro[pyrazolo[5,1-c][1,4]oxazine-4,3'-pyrrolidin]-1'-yl}éthyl)pyridin-2-ol (diastéréomère 1 et diastéréomère 2)
6-(1-{(3'R)-2-[6-amino-5-(trifluorométhyl)pyridin-3-yl]-6,7-dihydrospiro[pyrazolo[5,1-c][1,4]oxazine-4,3'-pyrrolidin]-1'-yl}éthyl)pyridin-2-ol (diastéréomère 1)
6-(1-{(3'R)-2-[6-amino-5-(trifluorométhyl)pyridin-3-yl]-6,7-dihydrospiro[pyrazolo[5,1-c][1,4]oxazine-4,3'-pyrrolidin]-1'-yl}éthyl)pyridin-2-ol (diastéréomère 2)
6-({(3R)-2'-[6-amino-5-(trifluorométhyl)pyridin-3-yl]-5',6'-dihydrospiro[pyrrolidine-3,4'-pyrrolo[1,2-b]pyrazol]-1-yl} (cyclopropyl)méthyl)pyridin-2-ol (diastéréomère 1 et diastéréomère 2)
6-({(3'R)-2-[6-amino-5-(trifluorométhyl)pyridin-3-yl]-6,7-dihydrospiro[pyrazolo[5,1-c][1,4]oxazine-4,3'-pyrrolidin]-1'-yl} (cyclopropyl)méthyl)pyridin-2-ol (diastéréomère 1 et diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[(3,3-difluorocyclobutyl)(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[(3,3-difluorocyclobutyl)(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[(3,3-difluorocyclobutyl)(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{l'-[(3,3-difluorocyclobutyl)(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (isomère 1)
5-{l'-[(3,3-difluorocyclobutyl)(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (isomère 2)
5-{l'-[(3,3-difluorocyclobutyl)(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (isomère 3)
5-{l'-[(3,3-difluorocyclobutyl)(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (isomère 4)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[(1H-imidazol-2-yl)(1-méthylcyclopropyl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[(1H-imidazol-2-yl)(1-méthylcyclopropyl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[(1H-imidazol-2-yl)(1-méthylcyclopropyl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclobutyl(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclobutyl(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[cyclobutyl(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3'R)-1'-[cyclohexy1(1H-imidazol-2-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3R)-1-[cyclohexyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[(3,3-difluorocyclobutyl)(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3'R)-1'-[cyclopropyl(1H-imidazol-2-yl)méthyl]-6,7-dihydrospiro[pyrazolo[5,1-c][1,4]oxazine-4,3'-pyrrolidin]-2-yl}-3-(trifluorométhyl)pyrazin-2-amine (diastéréomère 1 et diastéréomère 2)
5-{(3R)-1-[(1H-imidazol-2-yl)(1-méthylcyclobutyl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(1H-pyrazol-3-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclopropyl(1H-1,2,3-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-[(1R)-1-(2,6-difluorophényl)éthoxy]pyridin-2-amine (diastéréomère 1 et diastéréomère 2)
5-{(3R)-1-[(1-méthylcyclobutyl)(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(4-chloro-1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-[(1R)-1-(3-fluoropyridin-2-yl)éthoxy]pyridin-2-amine (diastéréomère 1 et diastéréomère 2)
5-{(3R)-1-[(3,3-difluorocyclobutyl)(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[(3,3-difluorocyclobutyl)(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclopentyl(1H-imidazol-2-yl)méthyl]-5',6"-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3'R)-1'-[cyclopropyl(1H-1,2,3-triazol-5-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3R)-1-[cyclopropyl(1-méthyl-1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{ (3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[1-(4H-1,2,4-triazol-3-yl)(1-²H)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3'R)-1'-[1-(4H-1,2,4-triazol-3-yl)(1-²H)propyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[cyclopropyl(1H-imidazol-2-yl)(²H)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[cyclopropyl(1H-imidazol-2-yl)(²H)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[cyclopropyl(1H-imidazol-2-yl)(²H)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[1-(1H-imidazol-2-yl)(1-²H)propyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[1-(1H-imidazol-2-yl)(1-²H)propyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclopropyl(1H-imidazol-2-yl)(²H)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclopropyl(1H-imidazol-2-yl)(²H)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[cyclopropyl(1H-imidazol-2-yl)(²H)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclopropyl(2-méthoxypyridin-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
3-[{(3R)-2'-[6-amino-5-(trifluorométhyl)pyridin-3-yl]-5',6'-dihydro-1H-spiro[pyrrolidine-3,4'-pyrrolo[1,2-b]pyrazol]-1-yl}(cyclopropyl)méthyl]pyridin-2(1H)-one (diastéréomère 1 et diastéréomère 2)
5-{(3R)-1-[(1-fluorocyclopropyl)(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[(1-fluorocyclopropyl)(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[(1-fluorocyclopropyl)(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[cyclopropyl(4-méthoxypyrimidin-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclobutyl(1H-1,2,4-triazol-5-yl)méthyl]-5',6'-dihydrospiro[pyrrolidine-3,4'-pyrrolo[1,2-b]pyrazol]-2'-yl}-3-(trifluorométhoxy)pyridin-2-amine (diastéréomère 1 et diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-(trifluorométhoxy)pyridin-2-amine (diastéréomère 1 et diastéréomère 2)
5-{(3'R)-1'-[cyclopropyl(1H-1,2,3-triazol-5-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3'R)-1'-[cyclopropyl(1H-1,2,3-triazol-5-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 2)
5-{(3R)-1-[(1-fluorocyclobutyl)(4H-1,2,4-triazol-3-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclopropyl(pyrimidin-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-[(1R)-1-(2,6-difluorophényl)éthoxy]pyridin-2-amine (diastéréomère 1)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-[(1R)-1-(2,6-difluorophényl)éthoxy]pyridin-2-amine (diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-[(1R)-1-(3-fluoropyridin-2-yl)éthoxy]pyridin-2-amine (diastéréomère 1)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-[(1R)-1-(3-fluoropyridin-2-yl)éthoxy]pyridin-2-amine (diastéréomère 2)
5-{(3'R)-1'-[cyclobutyl(4H-1,2,4-triazol-3-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1)
5-{(3R)-1-[cyclopentyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[cyclopentyl(1H-imidazol-2-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[(4,4-difluorocyclohexyl)(1H-1,2,4-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[(4,4-difluorocyclohexyl)(1H-1,2,4-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[(4,4-difluorocyclohexyl)(1H-1,2,4-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3R)-1-[2,2-difluoro-1-(1H-imidazol-2-yl)propyl]-5',6'-dihydrospiro[pyrrolidine-3,4'-pyrrolo[1,2-b]pyrazol]-2'-yl}-3-(trifluorométhyl)pyridin-2-amine (diastéréomère 1 et diastéréomère 2)
5-{(3R)-1-[cyclopropyl(1H-1,2,3-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 1)
5-{(3R)-1-[cyclopropyl(1H-1,2,3-triazol-5-yl)méthyl]-5',6'-dihydrospiro[pyrrolidine-3,4'-pyrrolo[1,2-b]pyrazol]-2'-yl}-3-(trifluorométhyl)pyridin-2-amine (diastéréomère 2)
5-{(3'R)-1'-[cyclobutyl(4H-1,2,4-triazol-3-yl)méthyl]-6,7-dihydrospiro[pyrazolo[5,1-c][1,4]oxazine-4,3'-pyrrolidin]-2-yl}-3-(trifluorométhyl)pyridin-2-amine (diastéréomère 1 et diastéréomère 2)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-(trifluorométhoxy)pyridin-2-amine (diastéréomère 1)
5-{(3R)-1-[cyclopropyl(1H-1,2,4-triazol-5-yl)méthyl]-5',6'-dihydrospiro[pyrrolidine-3,4'-pyrrolo[1,2-b]pyrazol]-2'-yl}-3-(trifluorométhoxy)pyridin-2-amine (diastéréomère 2)
(3R)-2'-{5-amino-6-[(1R)-1-phényléthoxy]pyrazin-2-yl}-N-éthyl-5',6'-dihydrospiro[pyrrolidine-3,4'-pyrrolo[1,2-b]pyrazole]-1-carboxamide
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyrazin-2-amine (diastéréomère 1 et diastéréomère 2)
5-{(3R)-1-[cyclopropyl(1H-imidazol-2-yl)méthyl]-5',6"-dihydrospiro[pyrrolidine-3,4'-pyrrolo[1,2-b]pyrazol]-2'-yl}-3-(trifluorométhyl)pyrazin-2-amine (diastéréomère 1)
5-{(3R)-1-[cyclopropyl(1H-imidazol-2-yl)méthyl]-5',6'-dihydrospiro[pyrrolidine-3,4'-pyrrolo[1,2-b]pyrazol]-2'-yl}-3-(trifluorométhyl)pyrazin-2-amine (diastéréomère 2)
ou un stéréoisomère, un tautomère, un N-oxyde, un hydrate, un solvate, ou un sel d'un tel composé, ou un mélange de ceux-ci.

10. Composé de formule générale (I) selon l'une quelconque des revendications 1, 2, 3, 4, 5, 6, 7, 8 et 9 pour une utilisation en tant que médicament.

11. Composé de formule générale (I) selon l'une quelconque des revendications 1, 2, 3, 4, 5, 6, 7, 8 et 9 pour une utilisation dans le traitement ou la prophylaxie d'une maladie.

12. Composition pharmaceutique comprenant un composé de formule générale (I) selon l'une quelconque des revendications 1, 2, 3, 4, 5, 6, 7, 8 et 9 et un ou plusieurs excipients pharmaceutiquement acceptables.

13. Combinaison pharmaceutique comprenant :
un ou plusieurs principes actifs qui sont des composés de formule générale (I) selon l'une quelconque des revendications 1, 2, 3, 4, 5, 6, 7, 8 et 9 et
un ou plusieurs composés anticancéreux pharmaceutiques actifs ou
un ou plusieurs inhibiteurs de point de contrôle immunitaire pharmaceutiques actifs.

14. Combinaison pharmaceutique selon la revendication 13, **caractérisée en ce que** l'inhibiteur de point de contrôle immunitaire pharmaceutique actif est un anticorps.

15. Composé de formule générale (I) selon l'une quelconque des revendications 1, 2, 3, 4, 5, 6, 7, 8 et 9 pour une utilisation dans le traitement ou la prophylaxie d'un cancer, d'une hyperplasie bénigne, d'un trouble athérosclérotique, d'une septicémie, d'un trouble auto-immun, d'un trouble vasculaire, d'une infection virale, d'un trouble neurodégénératif, ou d'un trouble inflammatoire.

16. Utilisation d'un composé de formule générale (I) selon l'une quelconque des revendications 1, 2, 3, 4, 5, 6, 7, 8 et 9 pour la contraception masculine.
